# EUROPEAN PATENT APPLICATION

(11) **EP 4 159 717 A1**
(43) Date of publication of application: **05.04.2023**
(21) Application number: 22194095.0
(22) Date of filing: 06.10.2015
(51) Int. Cl.: C07D 209/42, C07D 401/12, A61K 31/404, A61K 31/4439, A61P 11/00

(54) **MODULATORS OF CYSTIC FIBROSIS TRANSMEMBRANE CONDUCTANCE REGULATOR**

(30) Priority: 06.10.2014 US 201462060182 P; 11.02.2015 US 201562114767 P; 27.04.2015 US 201562153120 P
(62) Divisional of application: 20188642.1
(71) Applicant: Vertex Pharmaceuticals Incorporated, Boston, MA 02210 (US)
(72) Inventor: MILLER, Mark Thomas, San Diego, 92109 (US); ANDERSON, Corey, San Diego, 92122 (US); ARUMUGAM, Vijayalaksmi, San Marcos, 92078 (US); BEAR, Brian Richard, Carlsbad, 92009 (US); BINCH, Hayley Marie, Encinitas, 92024 (US); CLEMENS, Jeremy J., San Diego, 92130 (US); CLEVELAND, Thomas, San Diego, 92121 (US); CONROY, Erica, Columbus, 43206 (US); COON, Timothy Richard, Carlsbad, 92009 (US); FRIEMAN, Bryan A., La Jolla, 92037 (US); GROOTENHUIS, Peter Diederik Jan, San Diego, 92130 (US); GROSS, Raymond Stanley, Poway, 92064 (US); HADIDA-RUAH, Sara Sabina, La Jolla, 92037 (US); KHATUYA, Haripada, San Diego, 92129 (US); JOSHI, Pramod Virupax, San Diego, 92129 (US); KRENITSKY, Paul John, San Diego, 92126 (US); LIN, Chun-Chieh, San Diego, 92128 (US); MARELIUS, Gulin Erdogan, San Diego, 92124 (US); MELILLO, Vito, Escondido, 92025 (US); MCCARTNEY, Jason, Cardiff by the Sea, 92007 (US); NICHOLLS, Georgia McGaughey, Winchester, 01890 (US); PIERRE, Fabrice Jean Denis, La Jolla, 92037 (US); SILINA, Alina, San Diego, 92122 (US); TERMIN, Andreas P., Encinitas, 92024 (US); UY, Johnny, San Diego, 92104 (US); ZHOU, Jinglan, San Diego, 92130 (US)
(74) Representative: Carpmaels & Ransford LLP

(57) **Abstract**

The present invention features a compound of formula I: 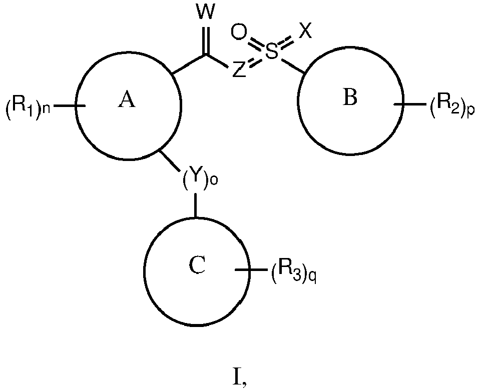 or a pharmaceutically acceptable salt thereof, where R₁, R₂, R₃, W, X, Y, Z, n, o, p, and q are defined herein, for the treatment of CFTR mediated diseases, such as cystic fibrosis. The present invention also features pharmaceutical compositions, method of treating, and kits thereof.

## Description

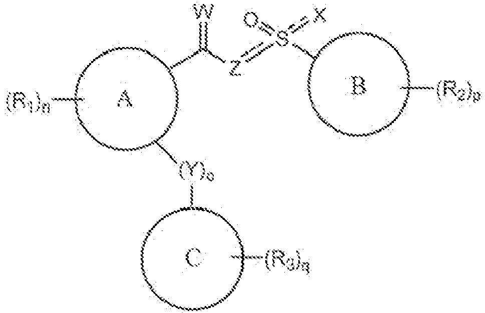

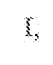

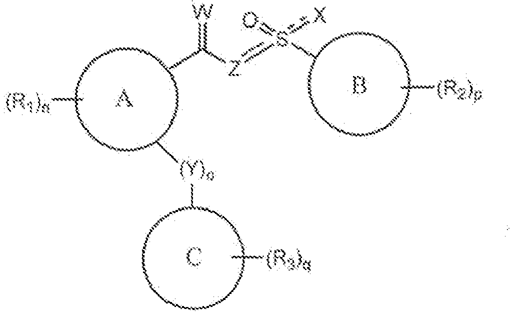

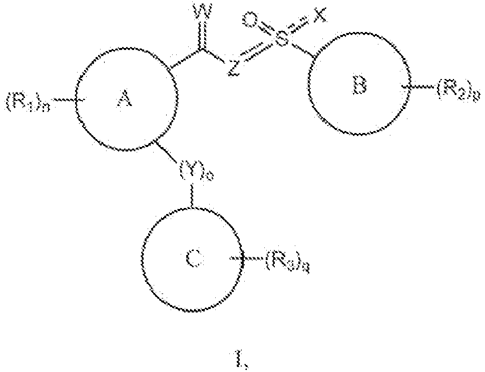

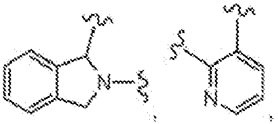

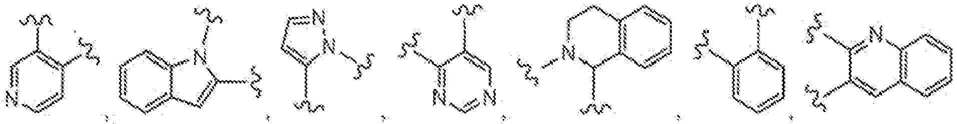

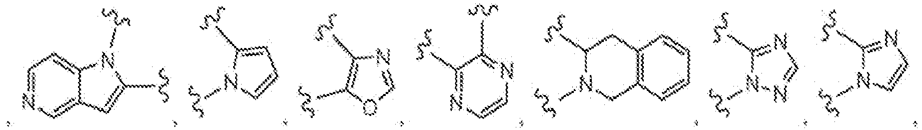

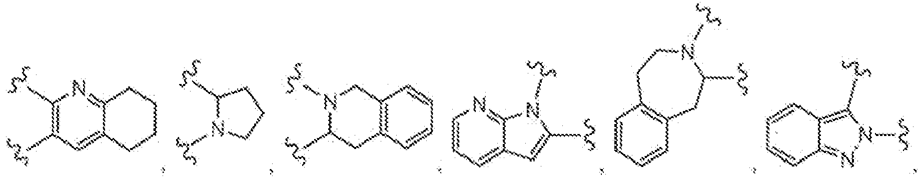

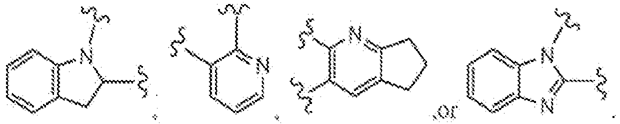

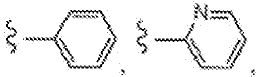

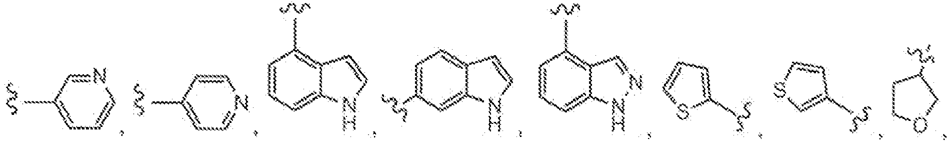

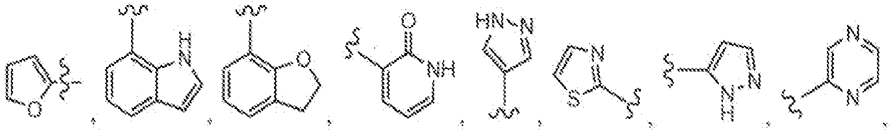

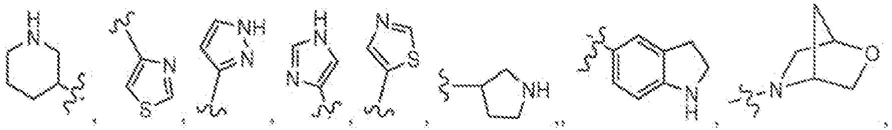

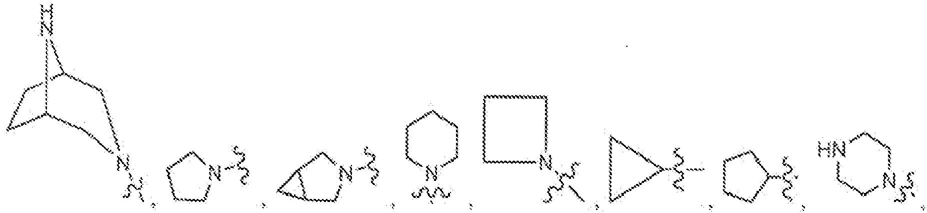

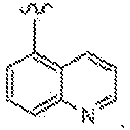

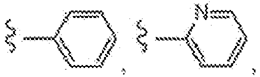

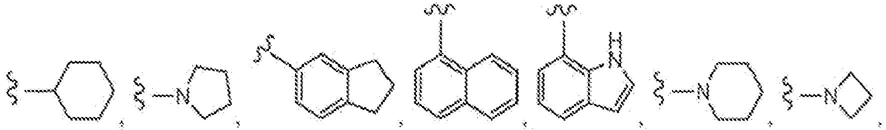

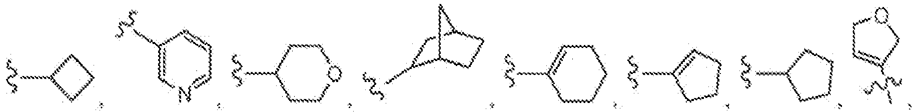

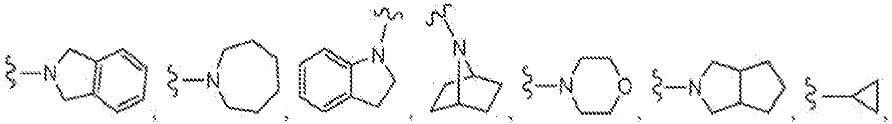

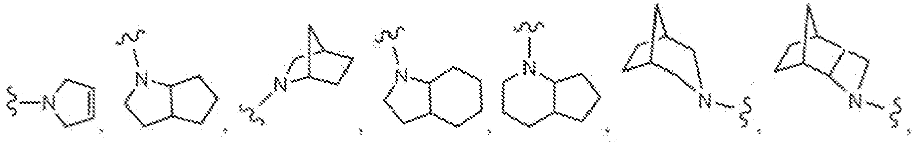

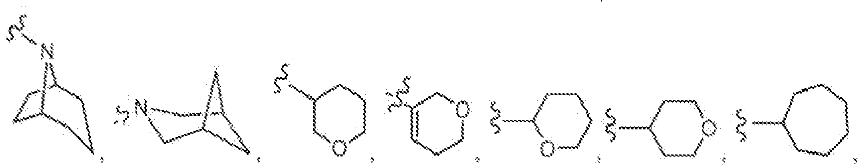

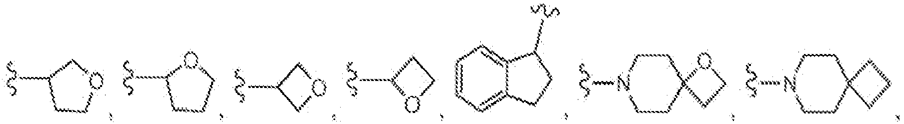

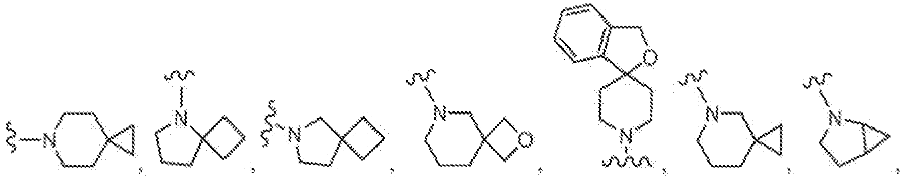

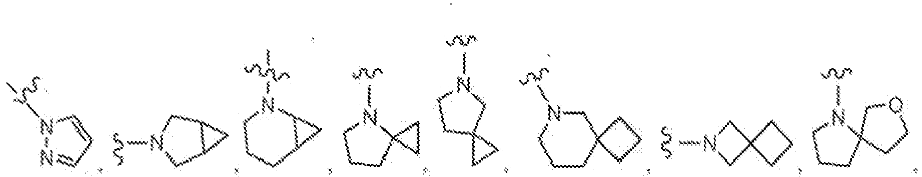

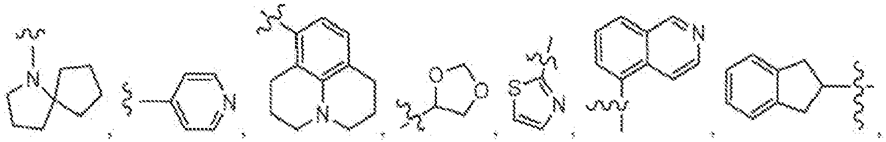

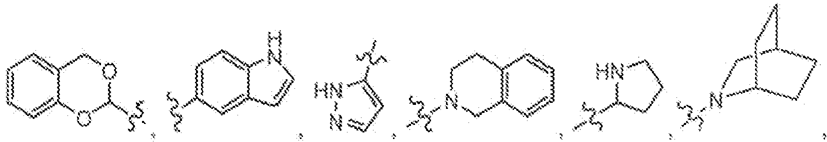

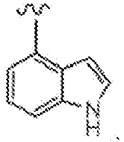

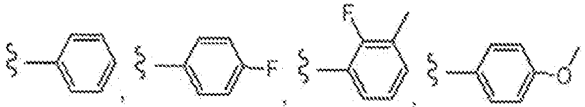

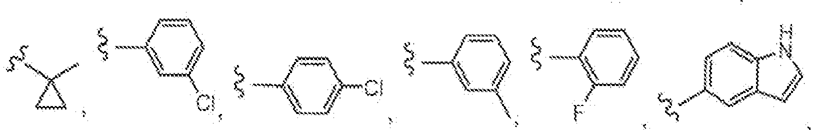

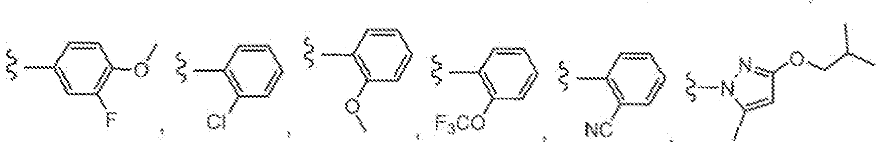

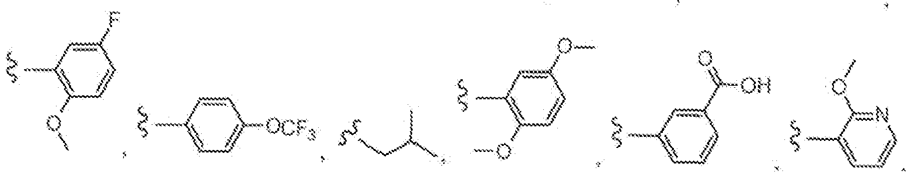

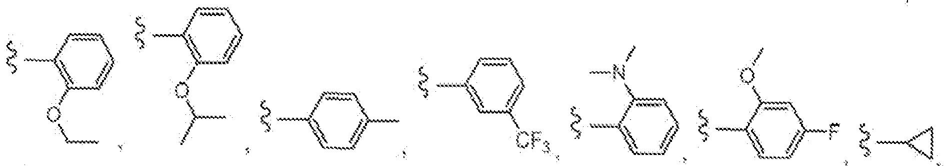

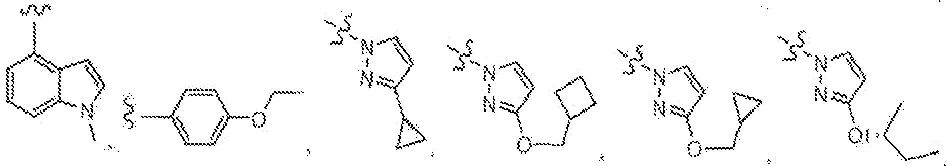

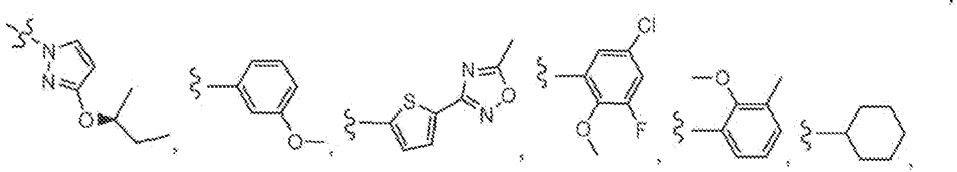

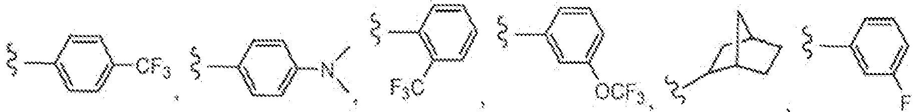

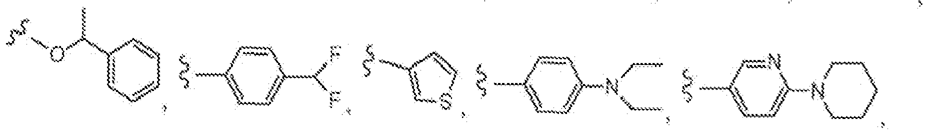

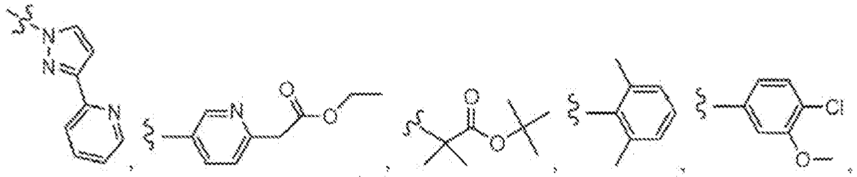

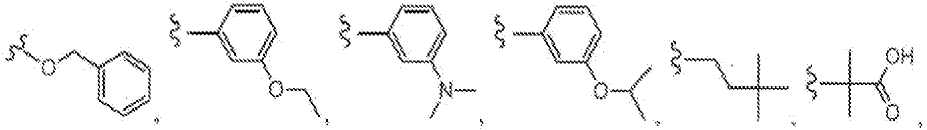

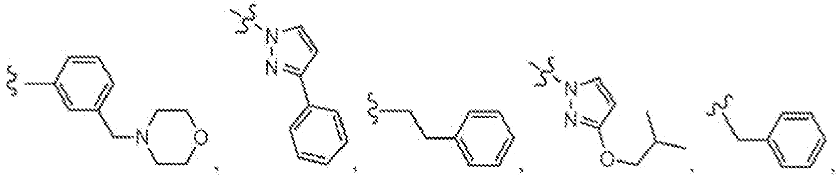

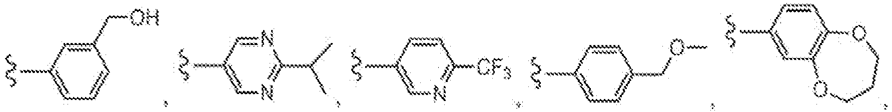

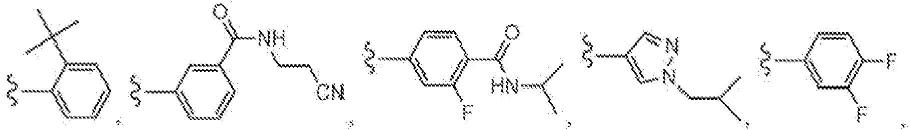

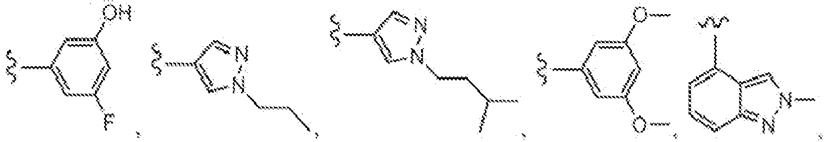

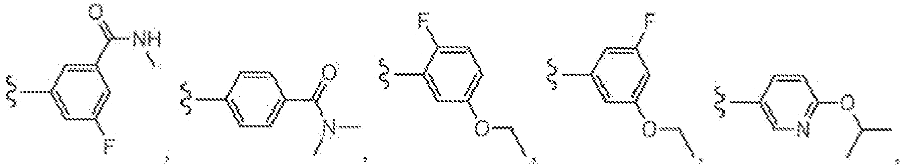

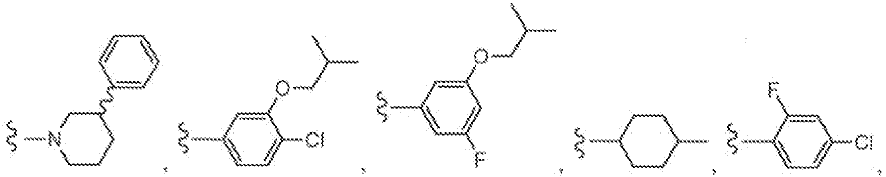

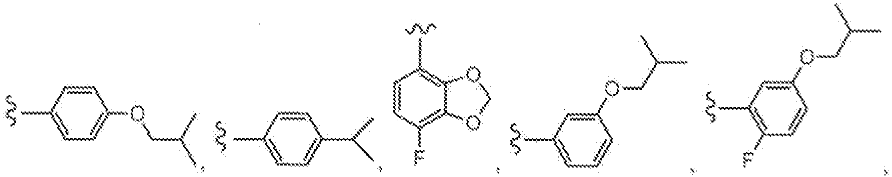

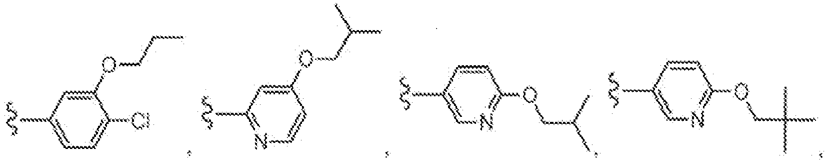

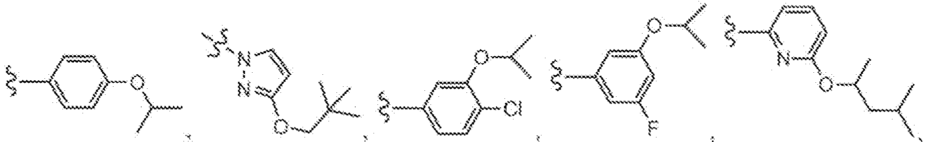

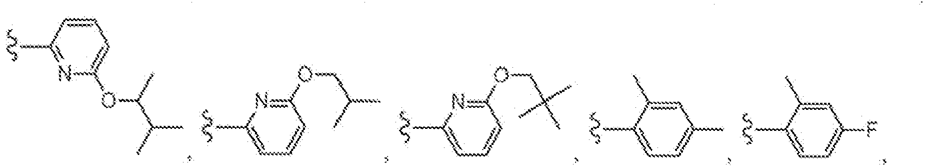

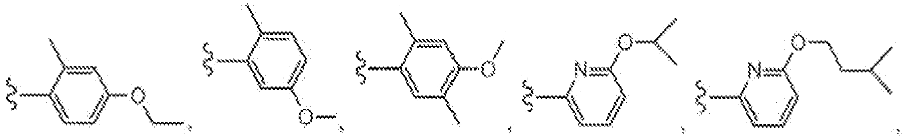

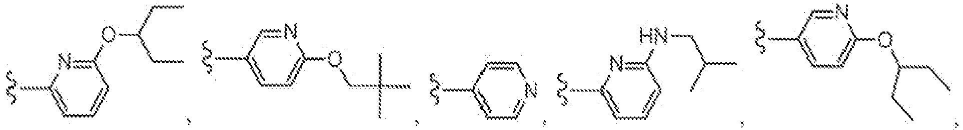

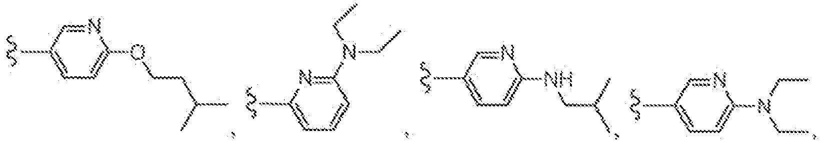

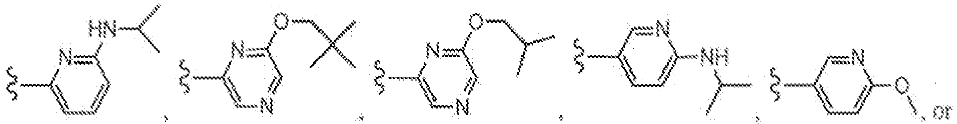

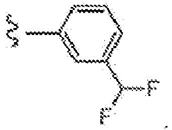

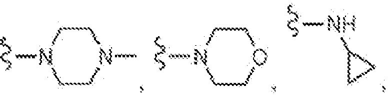

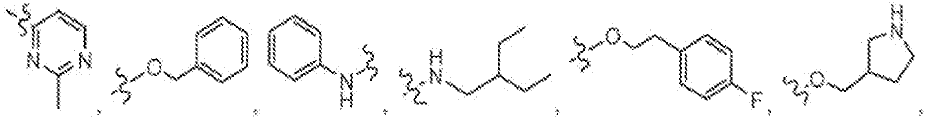

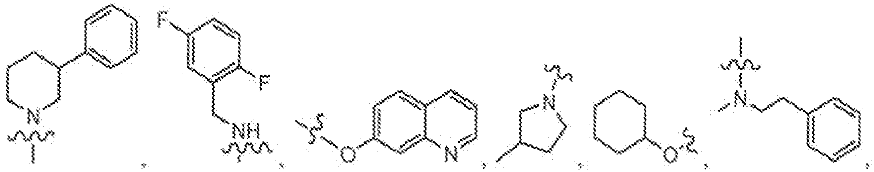

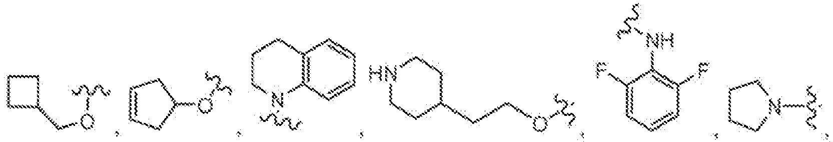

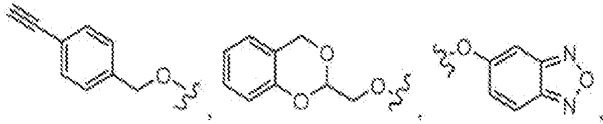

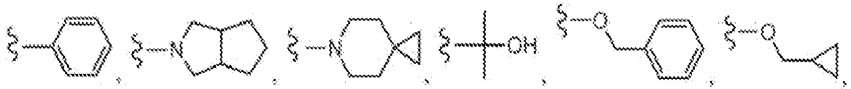

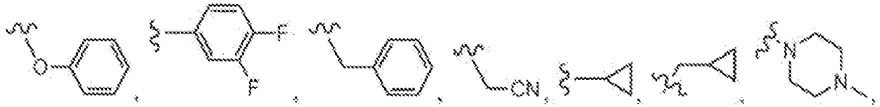

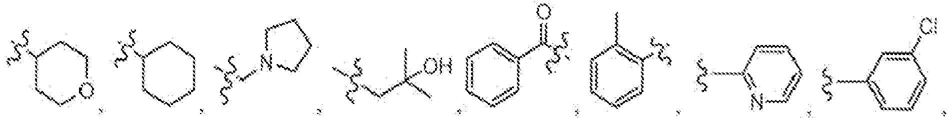

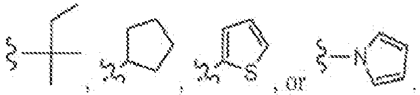

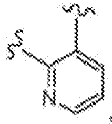

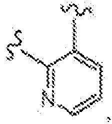

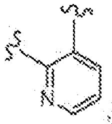

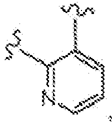

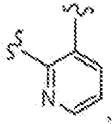

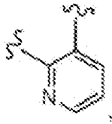

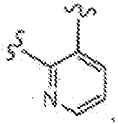

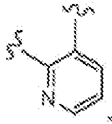

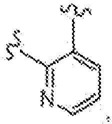

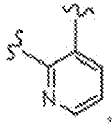

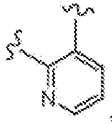

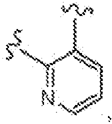

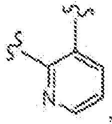

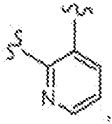

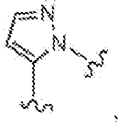

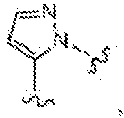

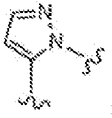

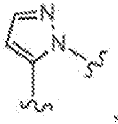

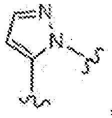

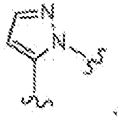

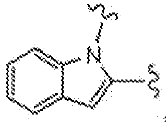

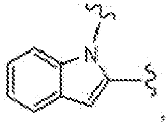

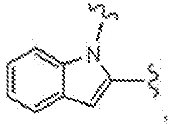

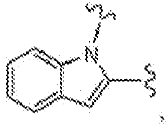

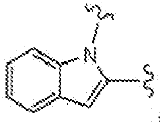

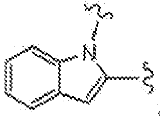

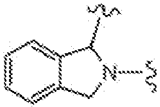

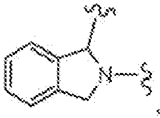

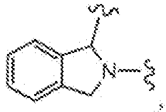

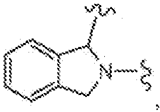

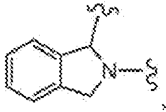

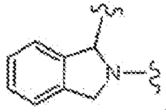

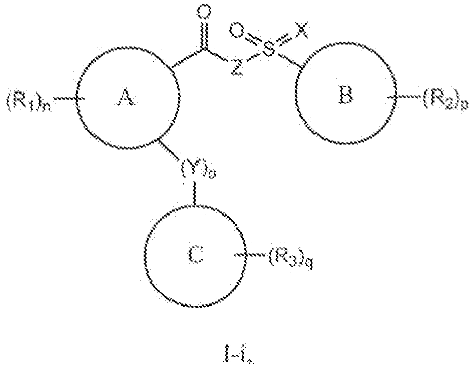

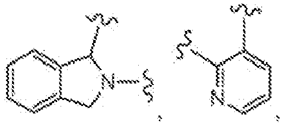

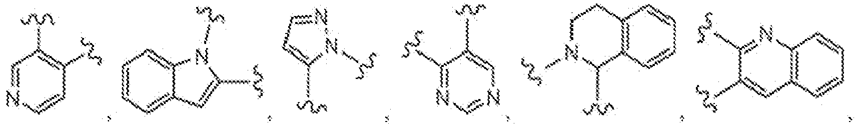

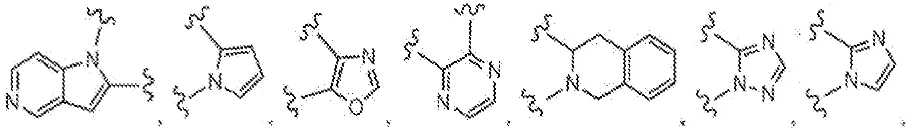

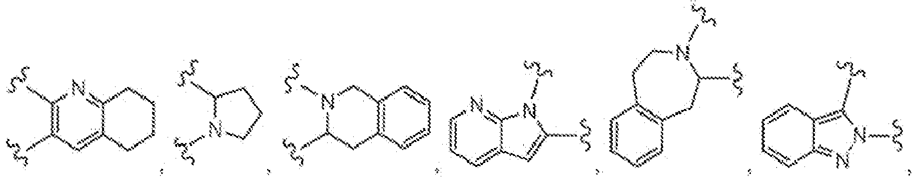

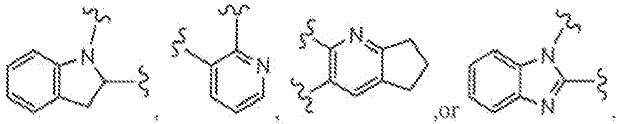

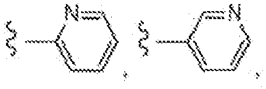

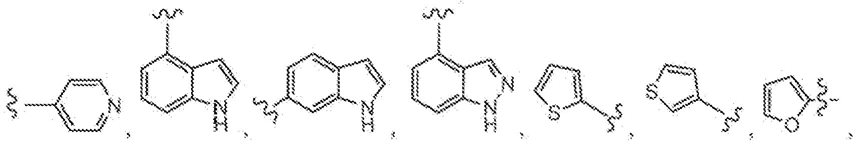

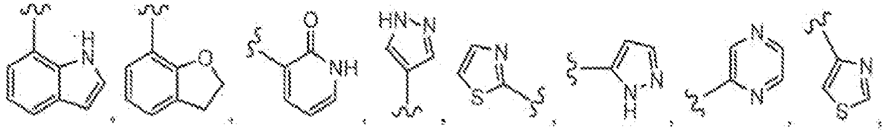

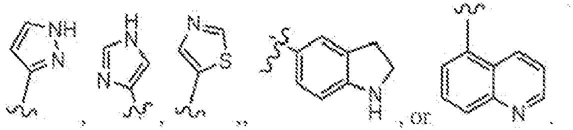

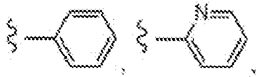

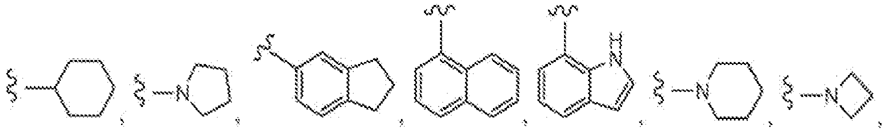

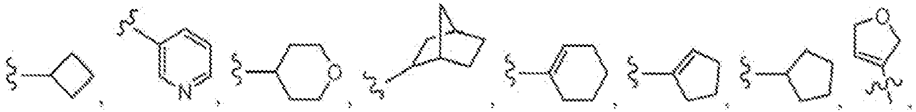

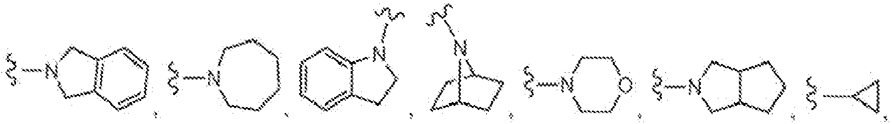

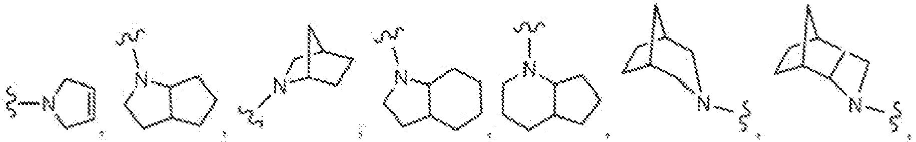

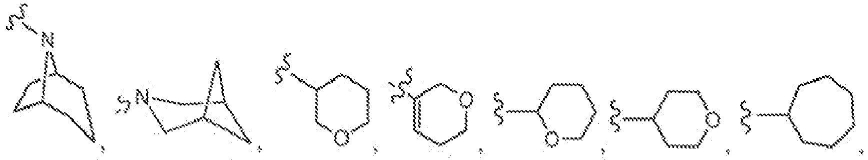

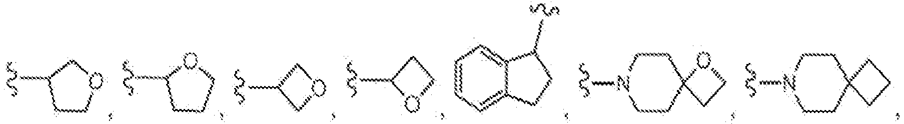

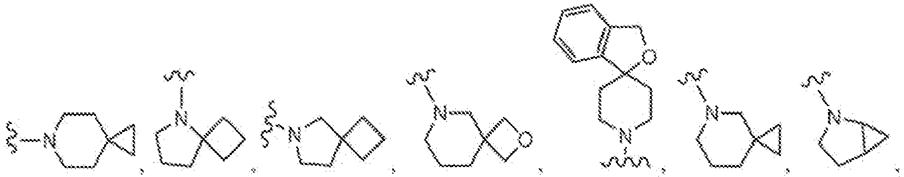

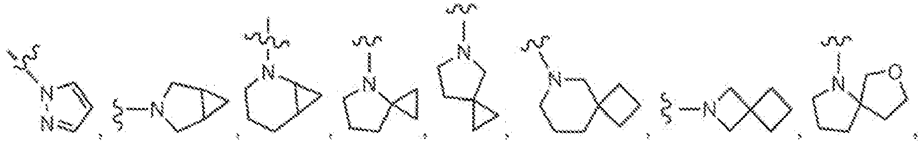

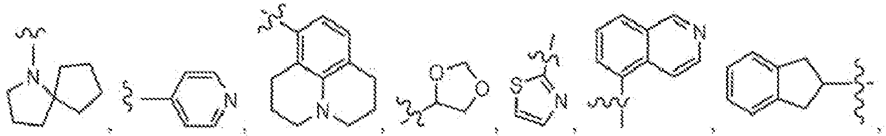

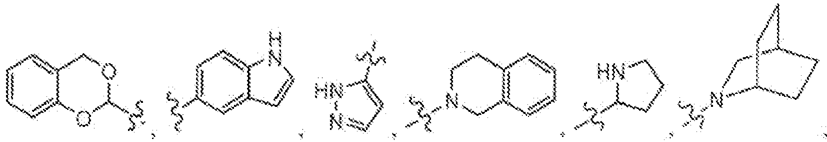

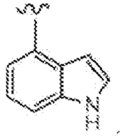

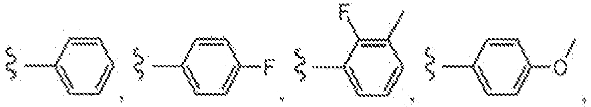

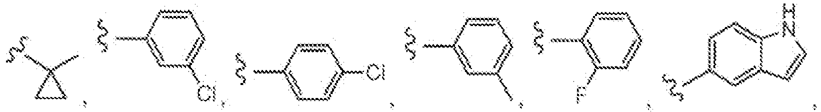

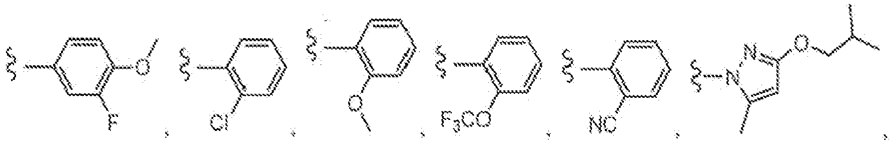

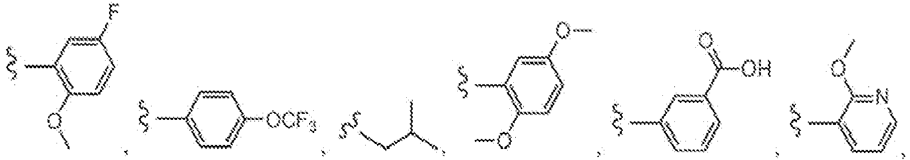

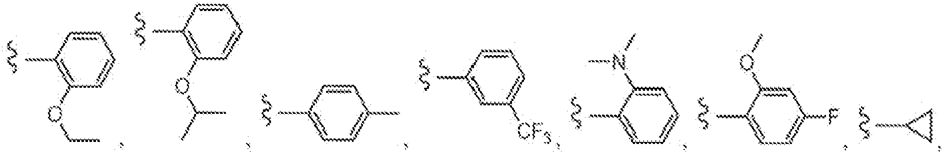

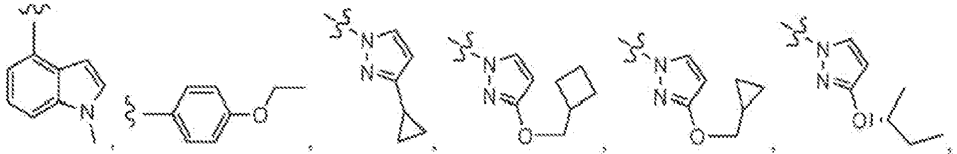

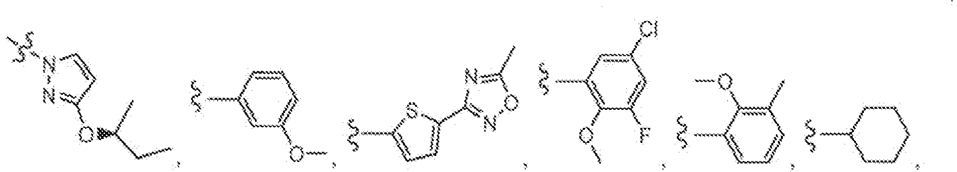

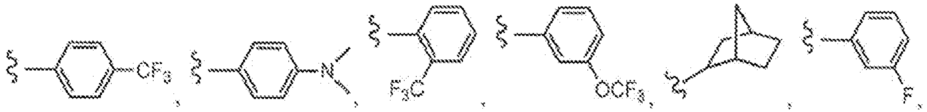

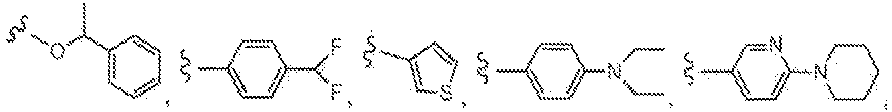

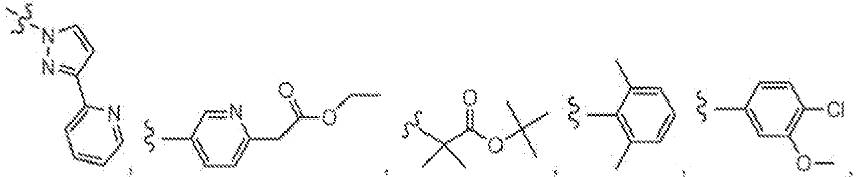

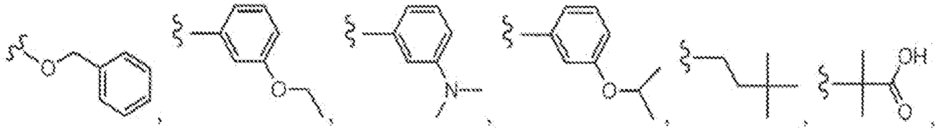

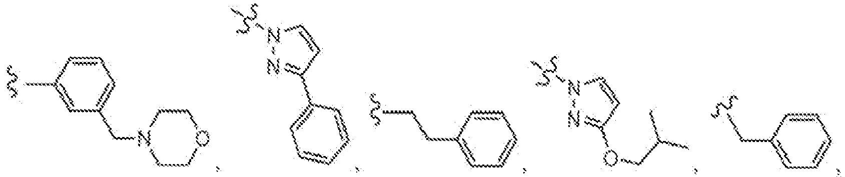

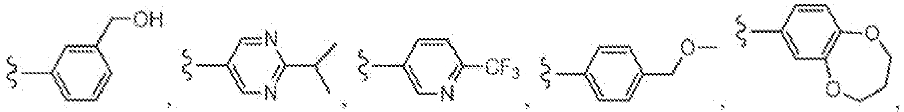

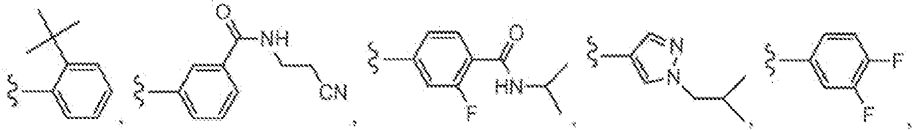

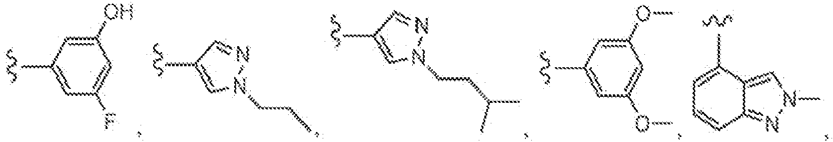

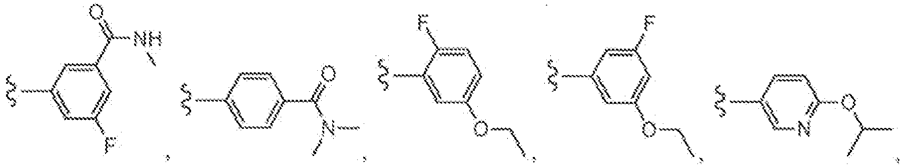

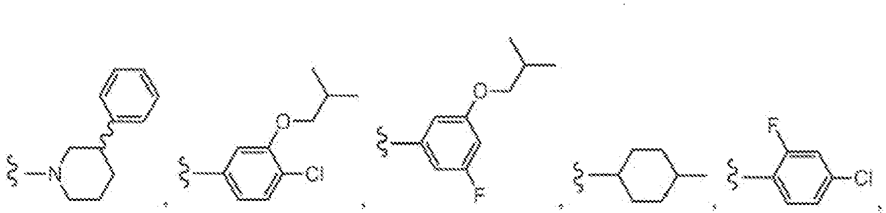

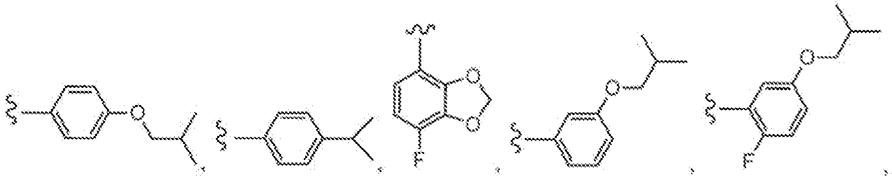

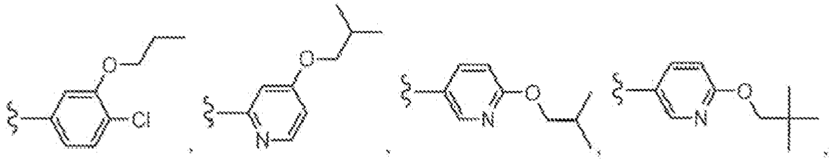

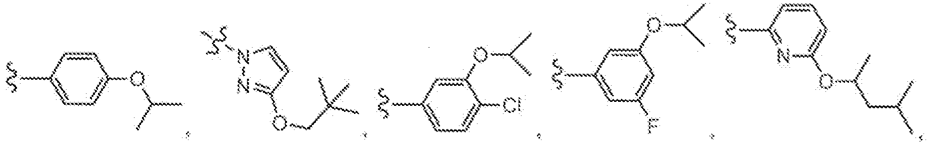

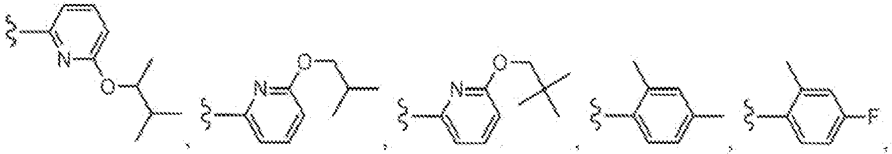

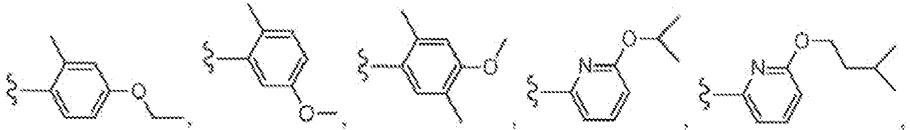

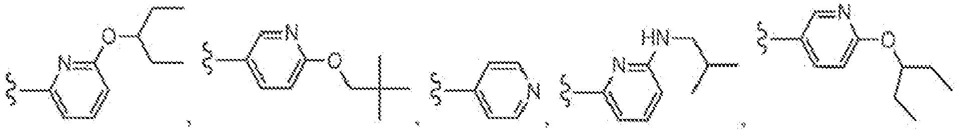

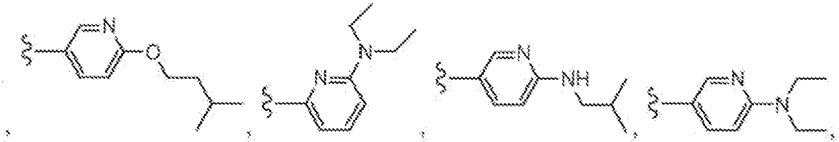

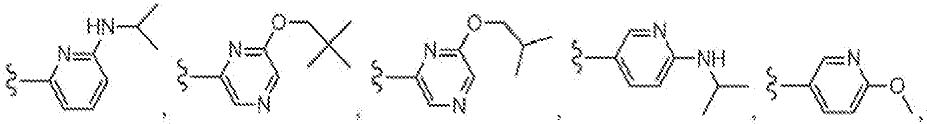

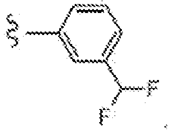

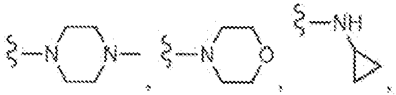

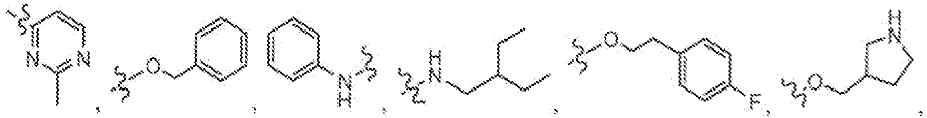

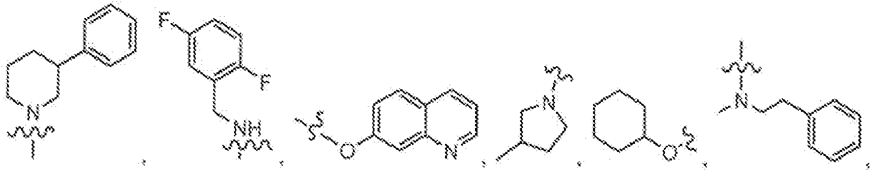

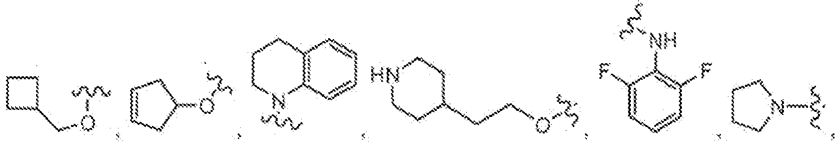

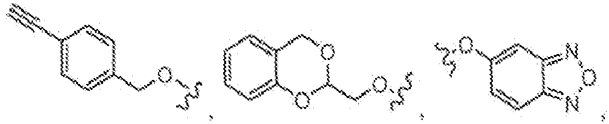

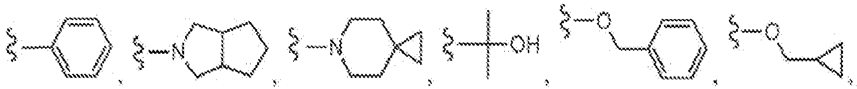

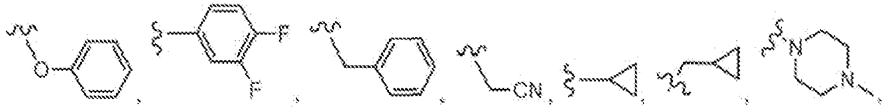

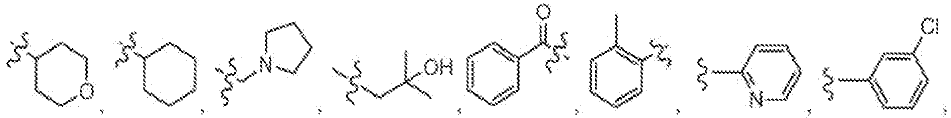

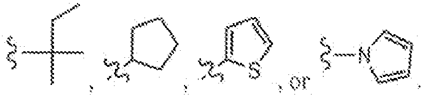

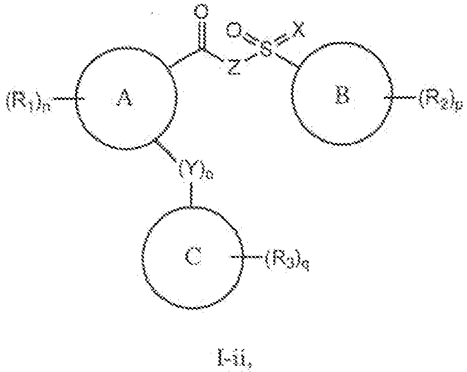

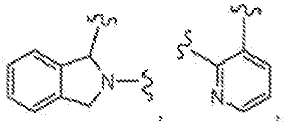

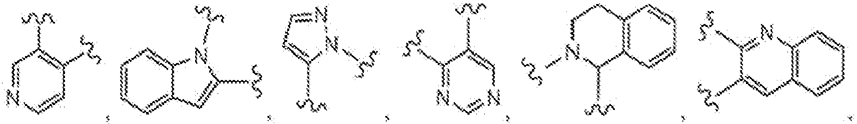

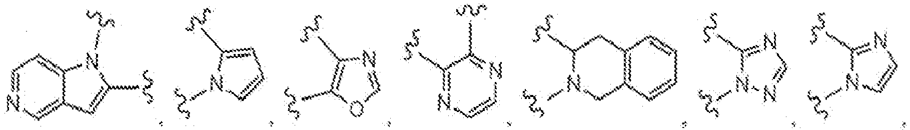

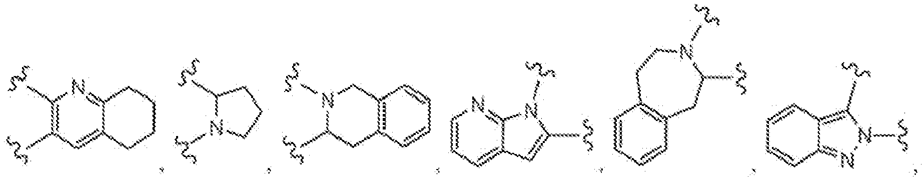

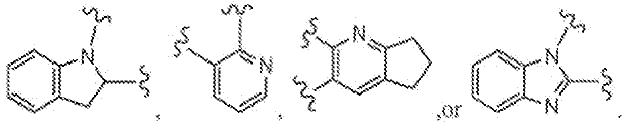

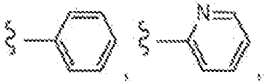

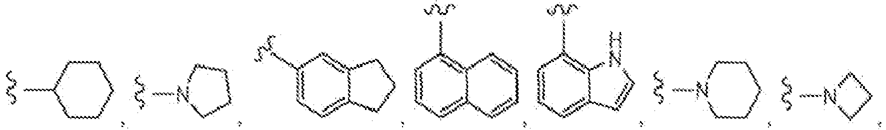

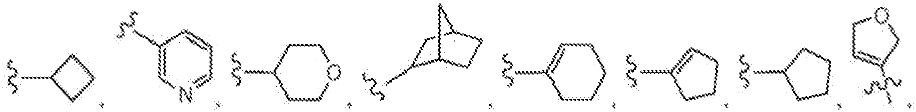

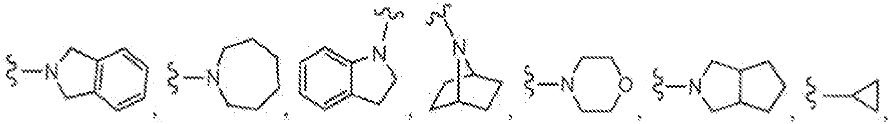

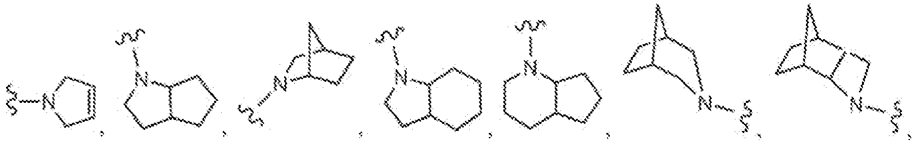

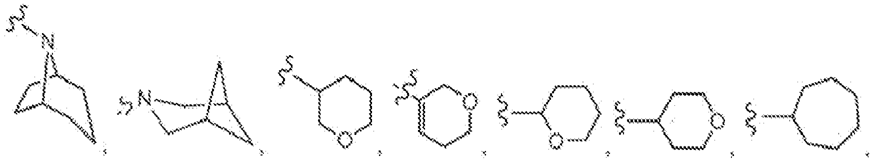

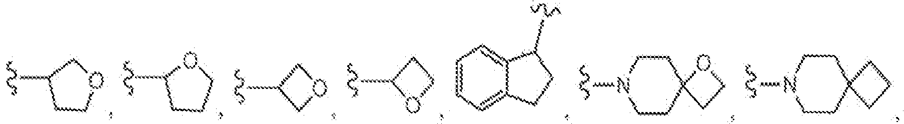

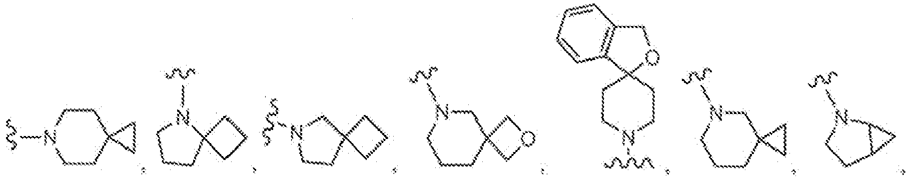

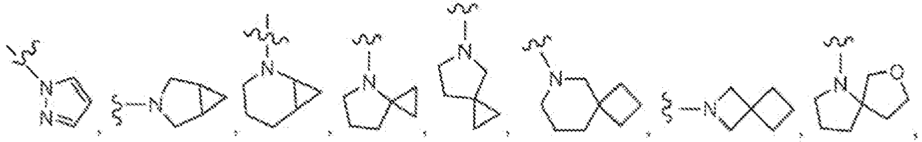

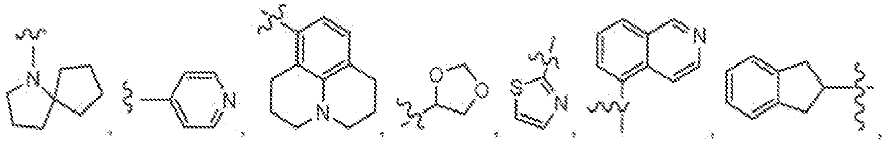

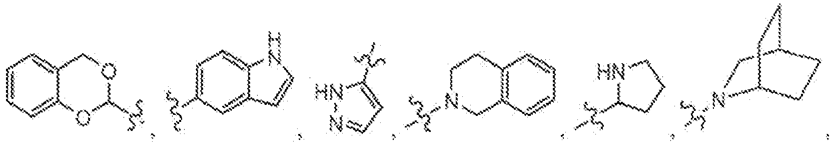

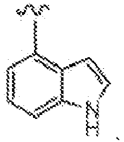

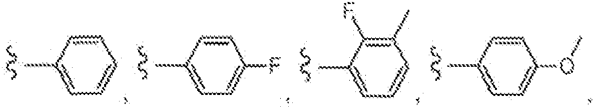

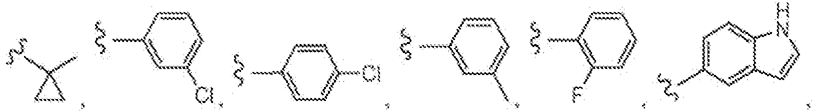

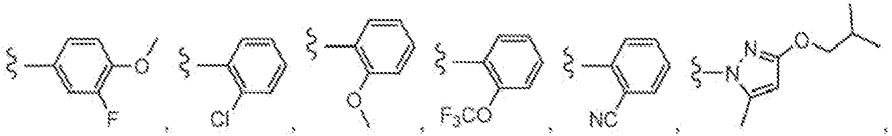

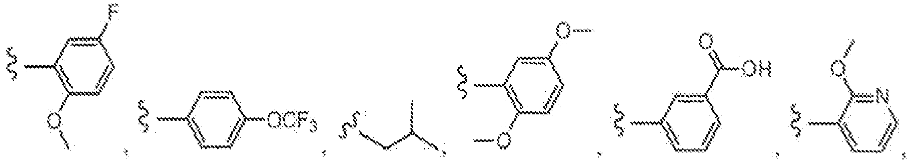

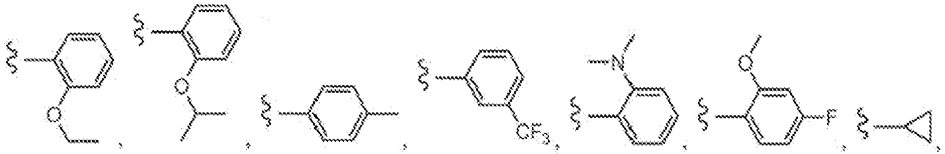

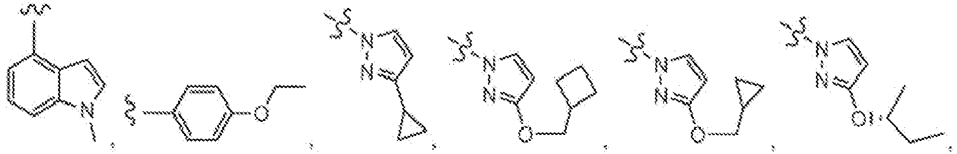

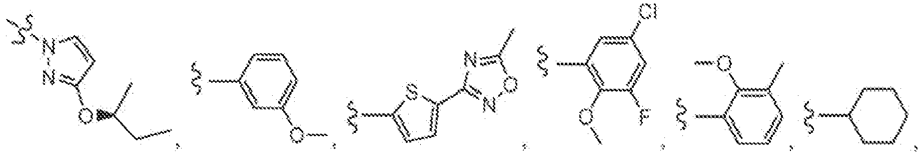

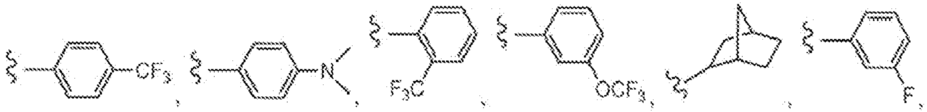

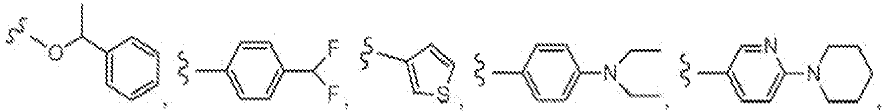

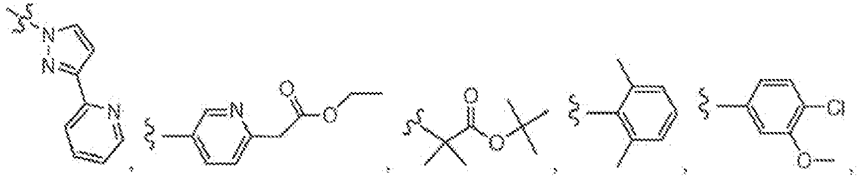

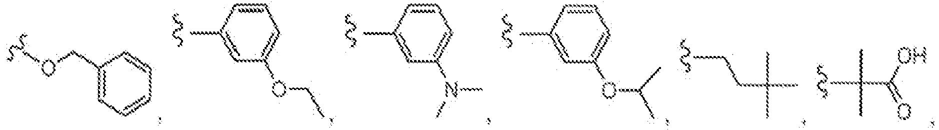

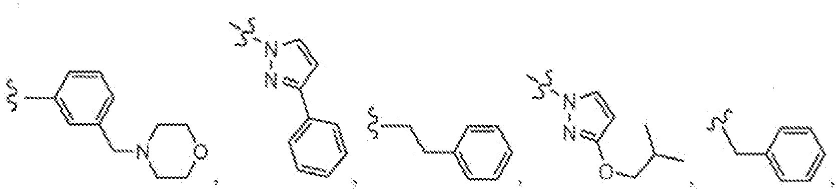

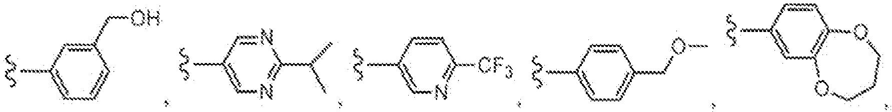

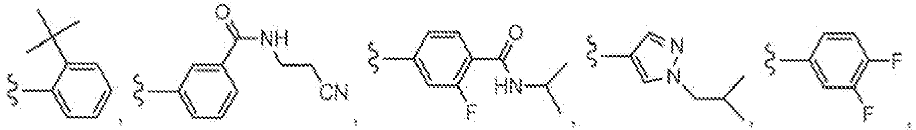

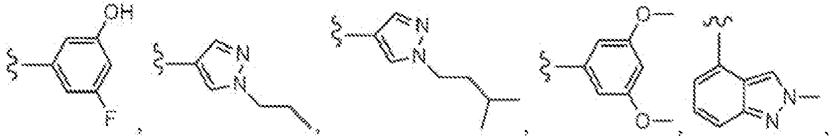

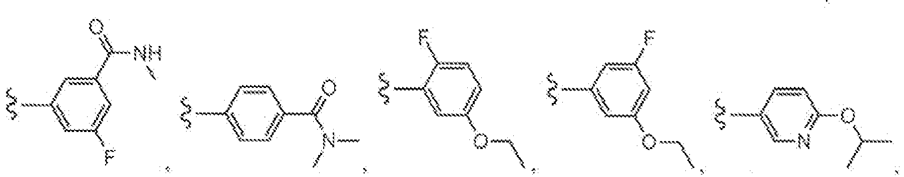

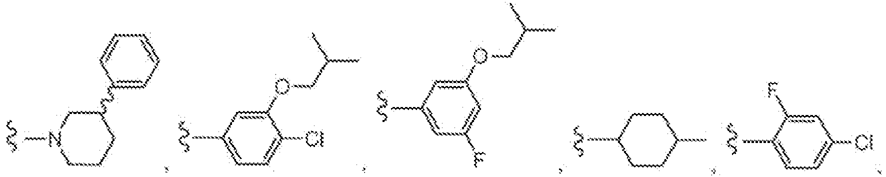

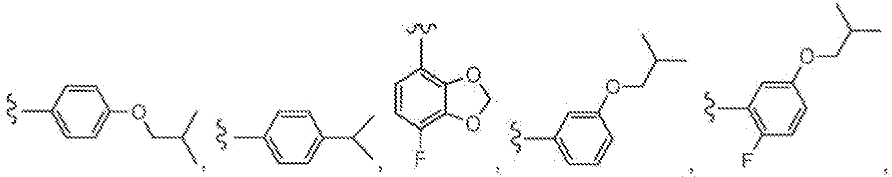

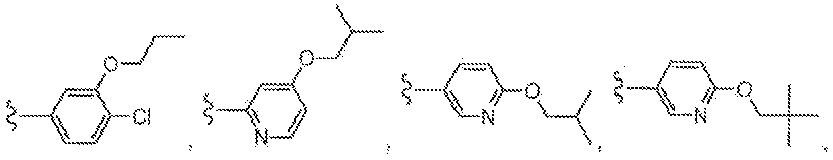

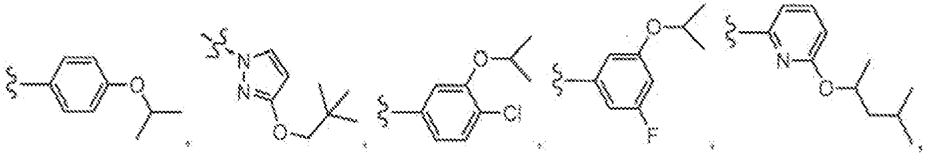

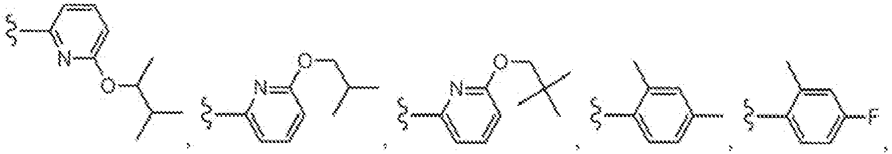

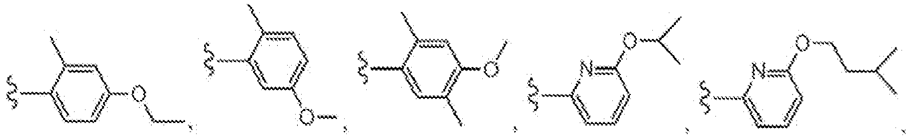

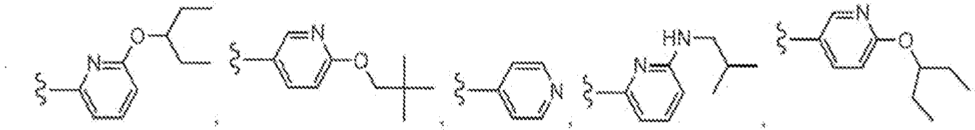

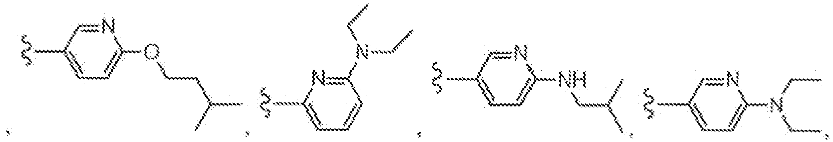

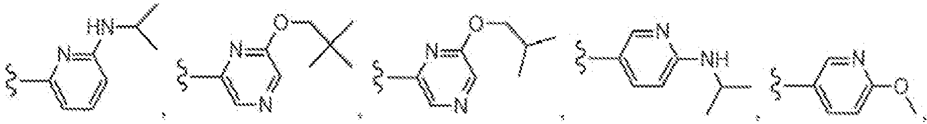

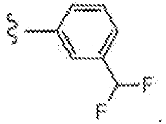

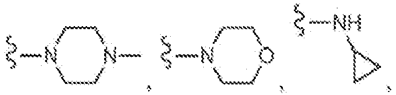

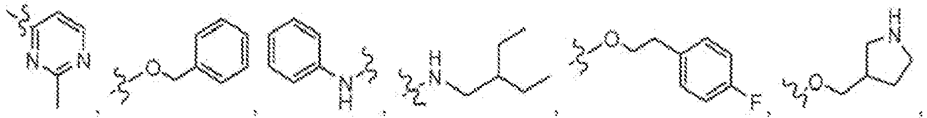

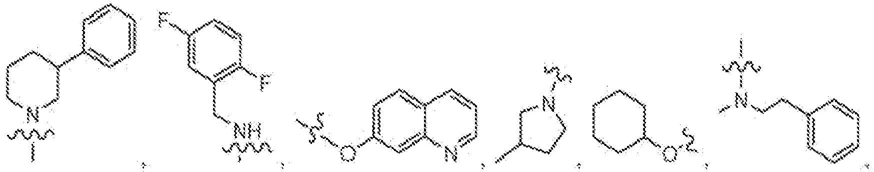

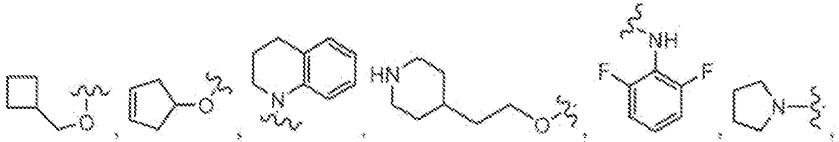

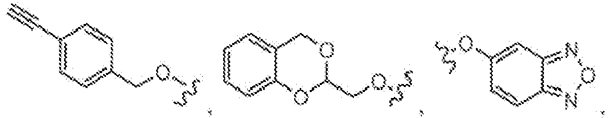

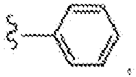

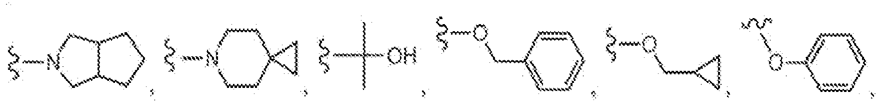

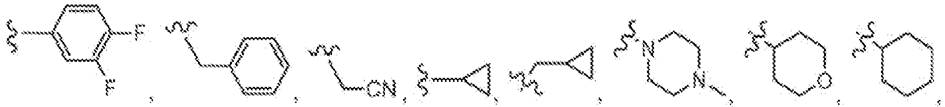

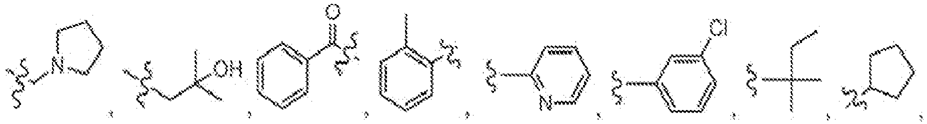

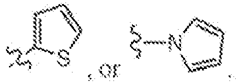

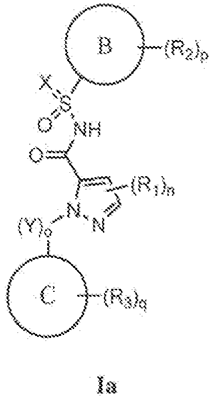

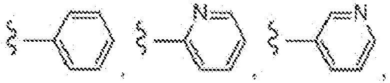

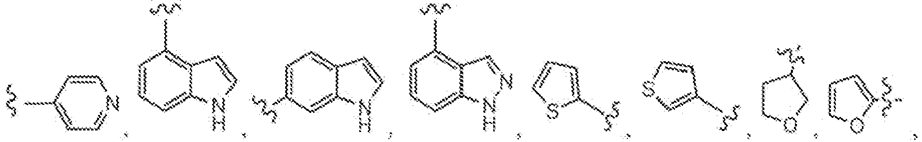

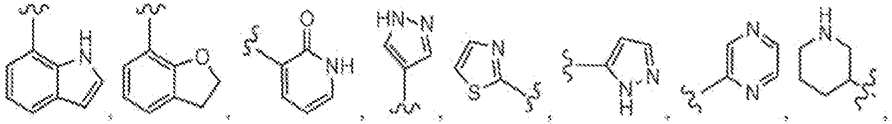

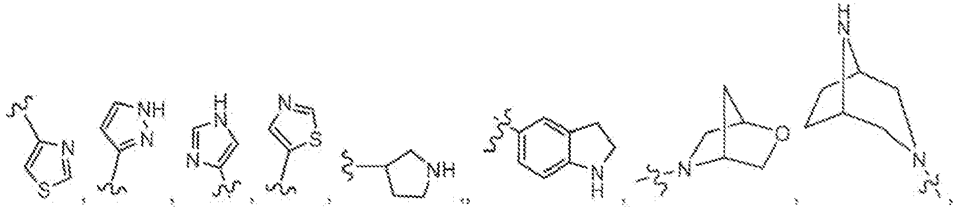

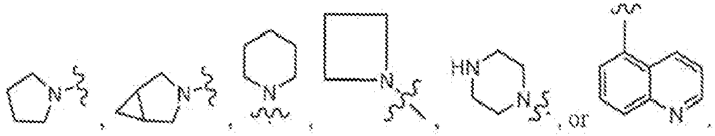

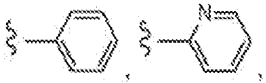

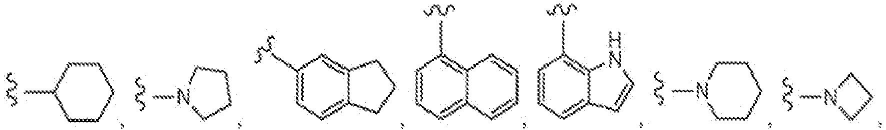

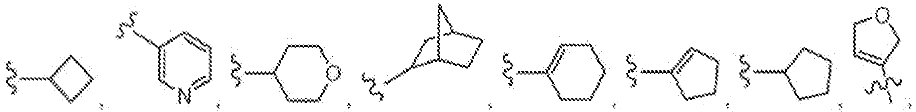

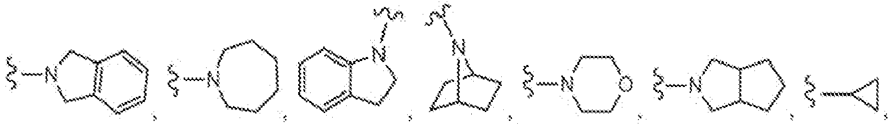

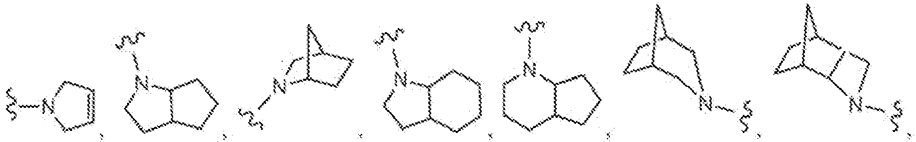

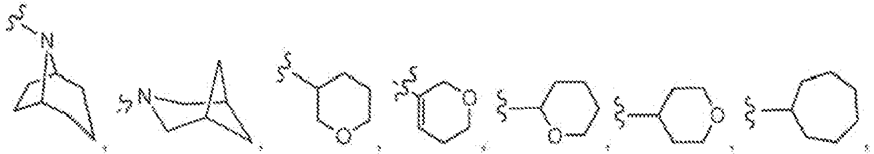

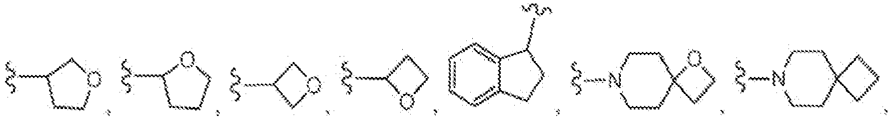

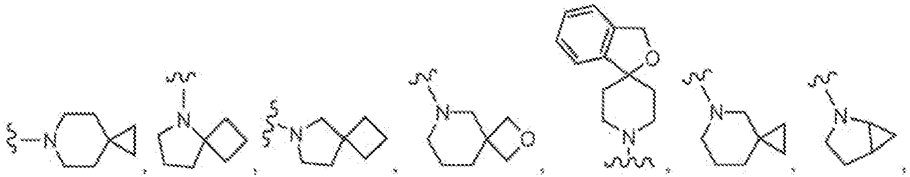

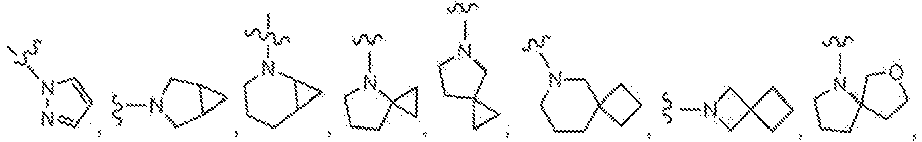

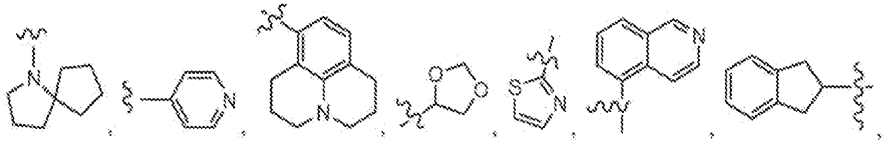

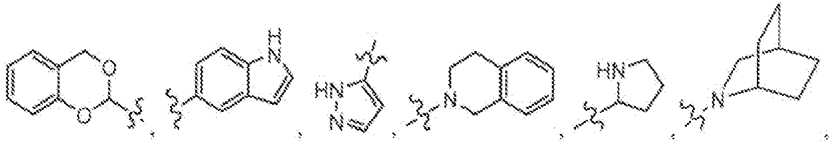

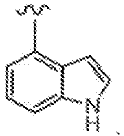

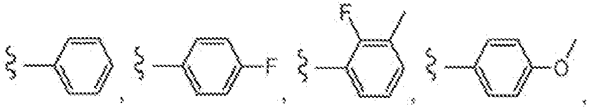

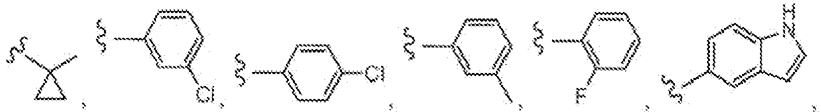

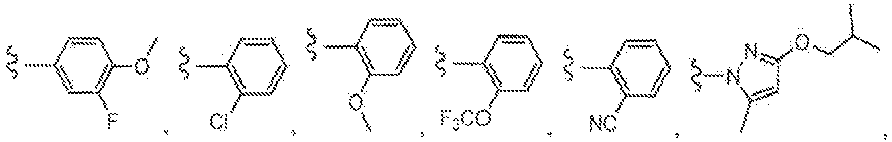

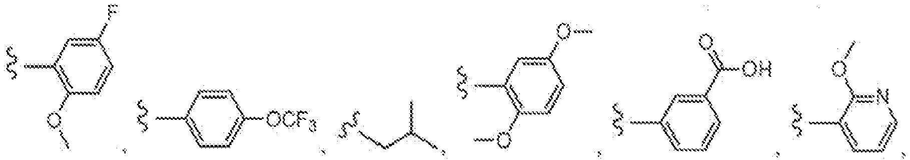

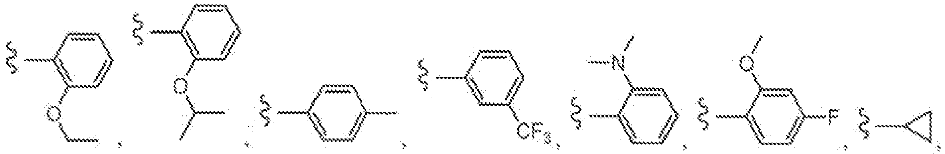

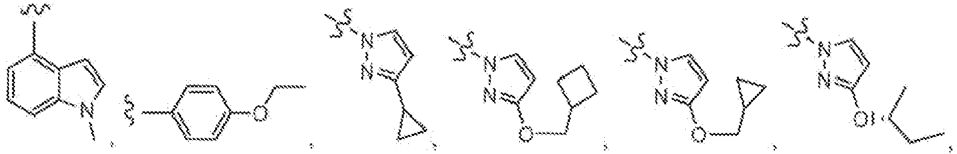

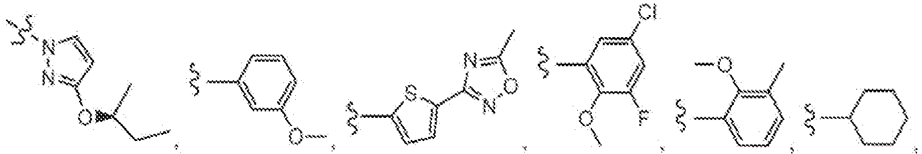

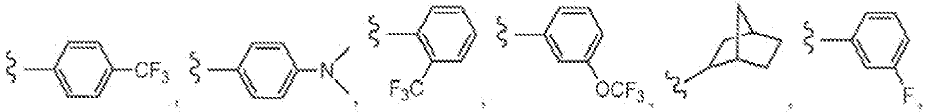

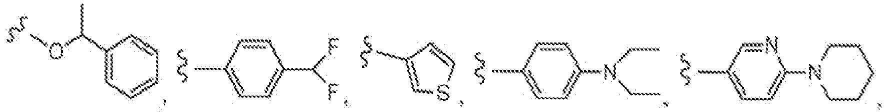

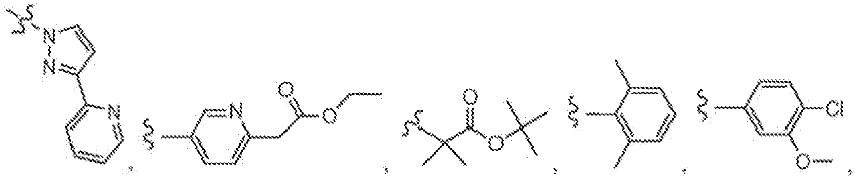

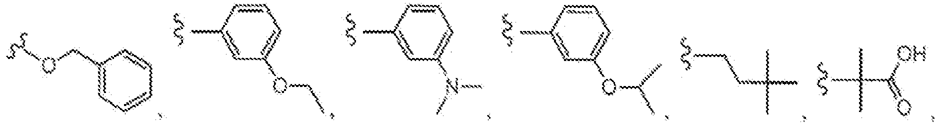

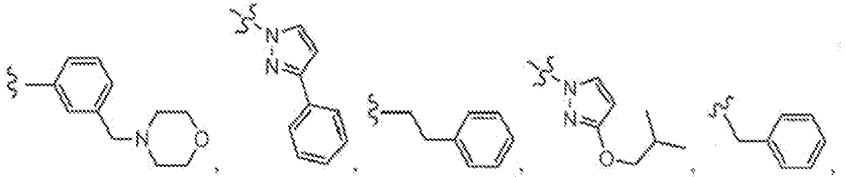

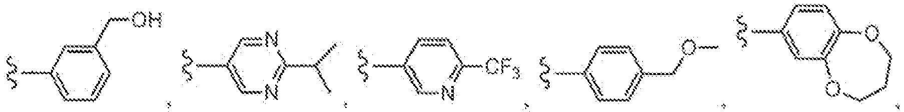

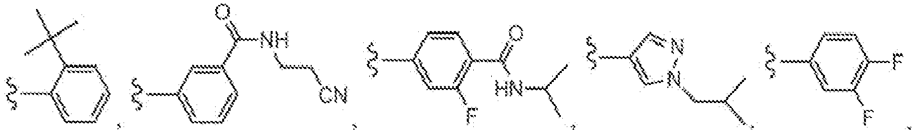

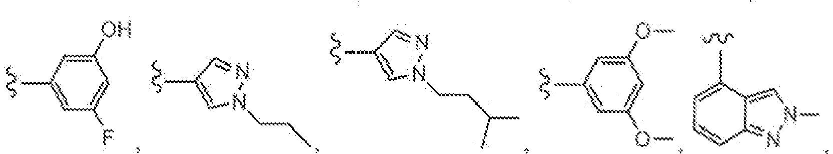

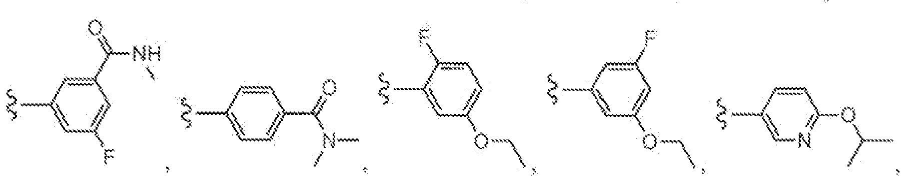

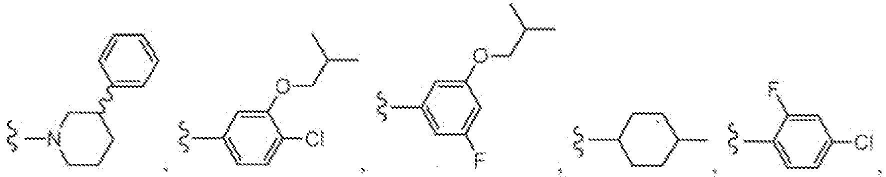

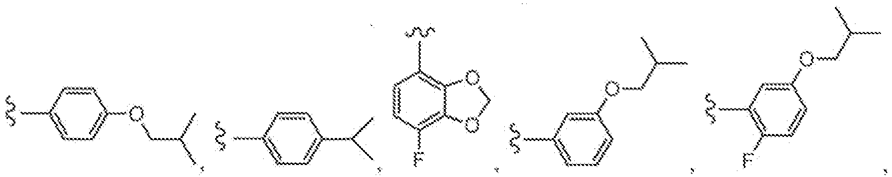

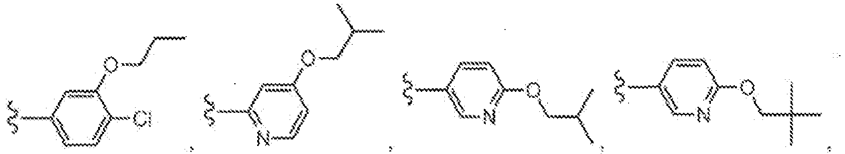

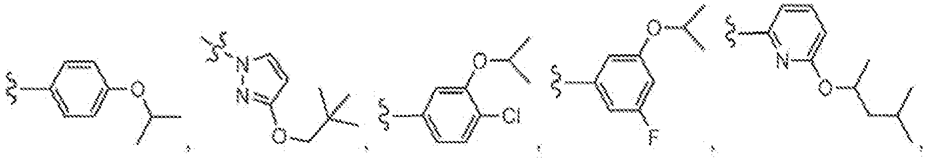

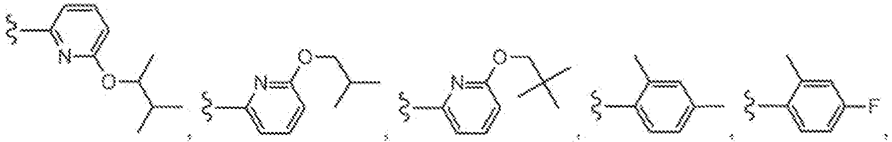

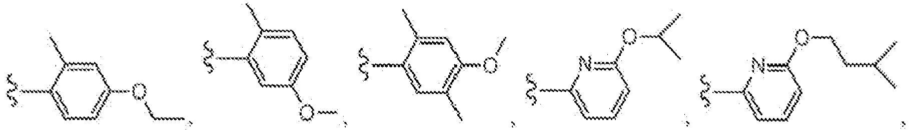

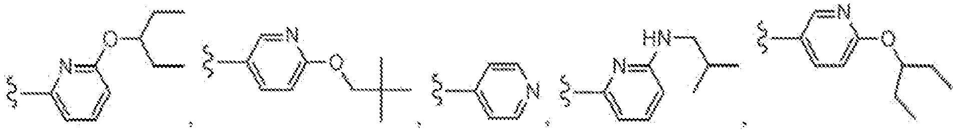

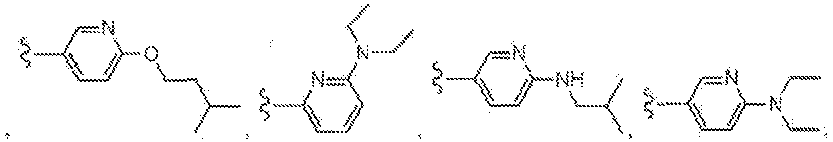

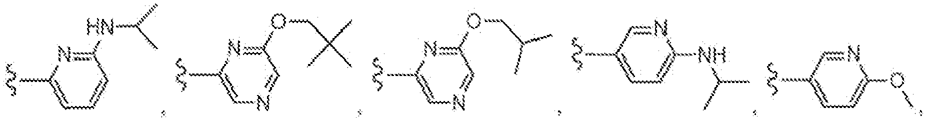

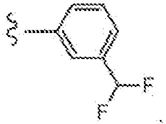

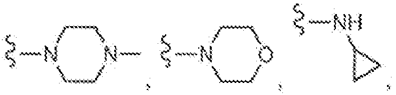

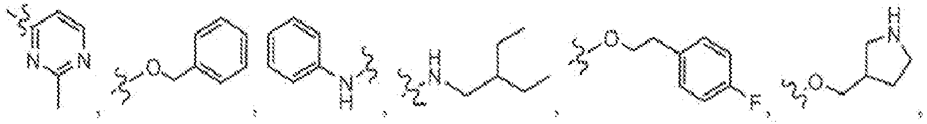

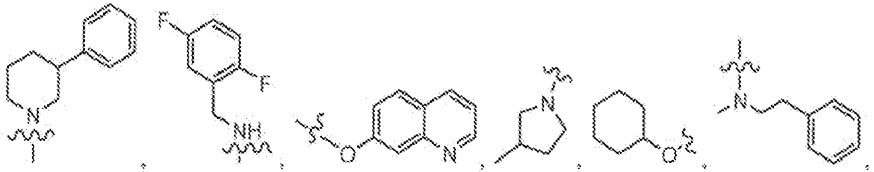

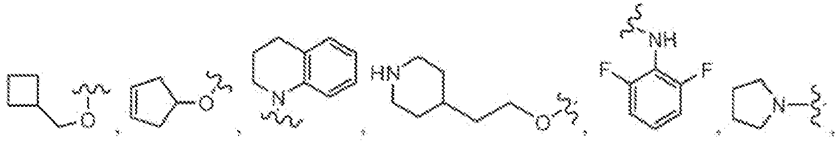

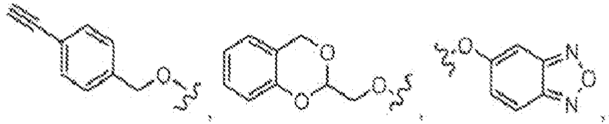

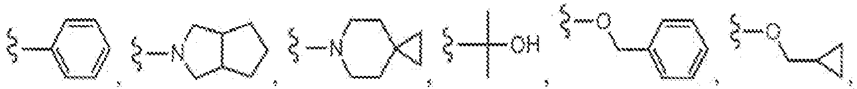

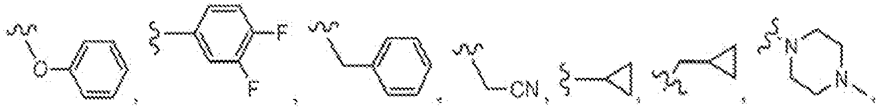

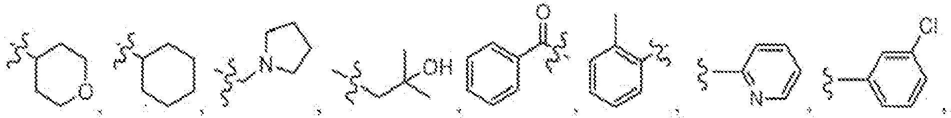

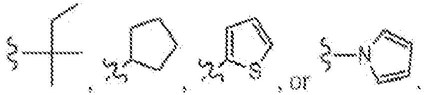

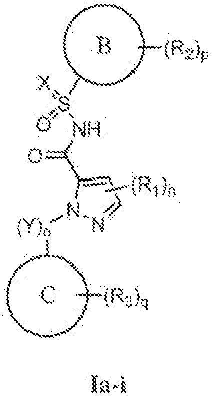

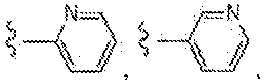

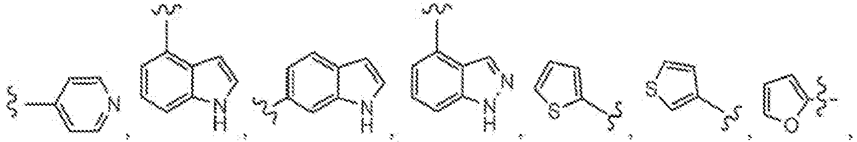

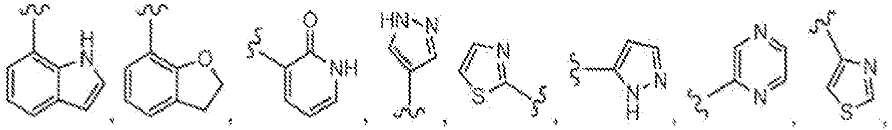

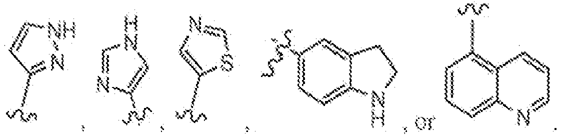

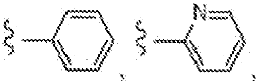

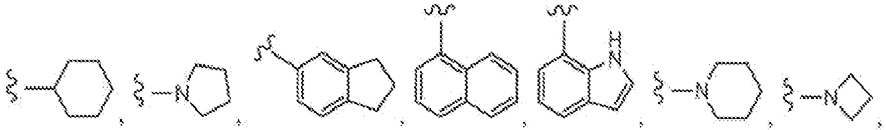

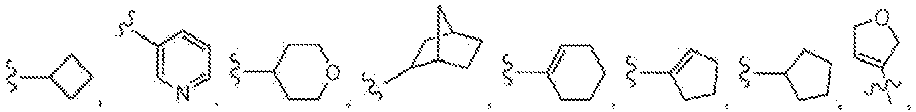

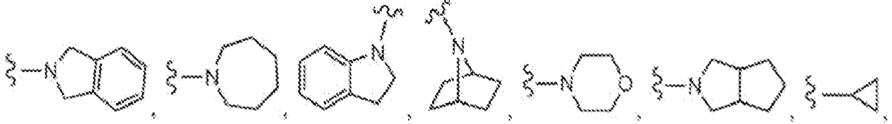

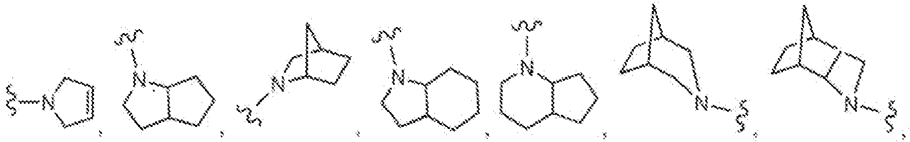

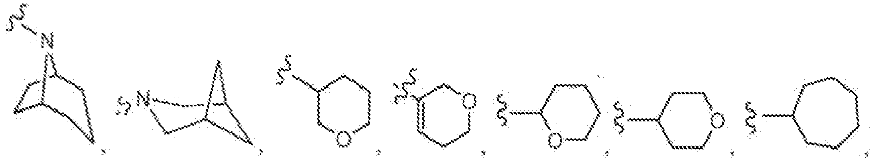

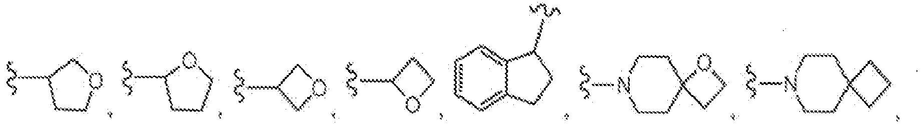

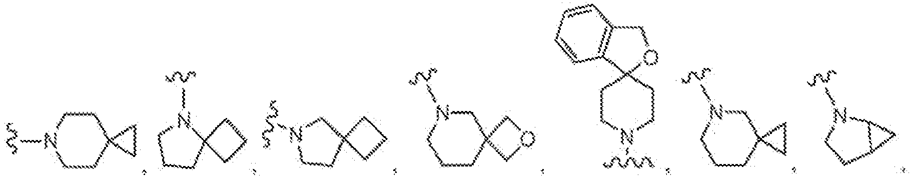

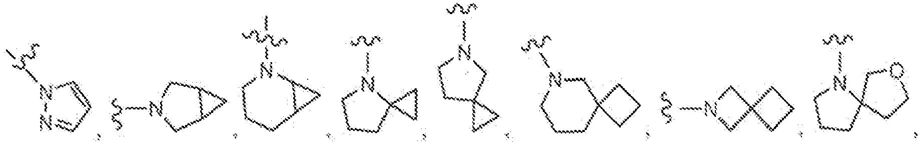

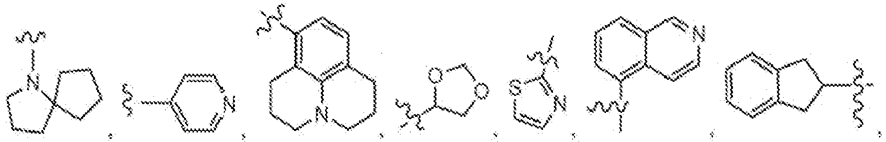

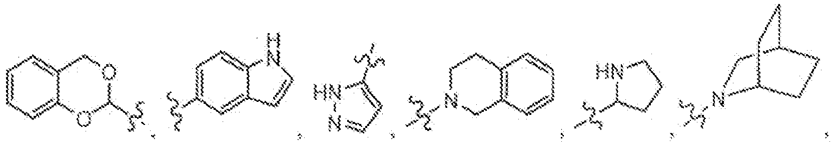

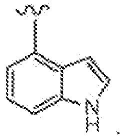

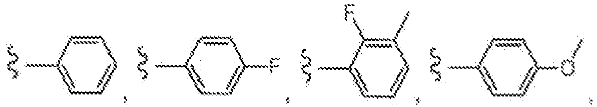

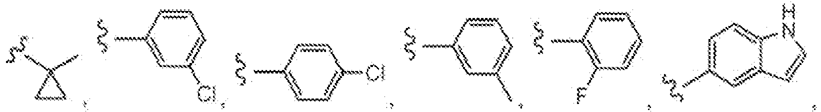

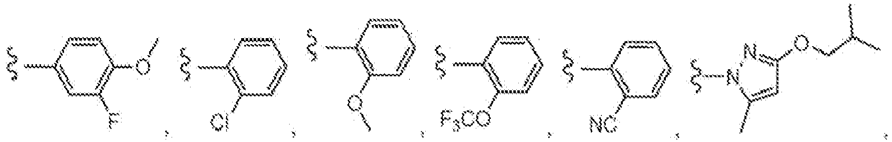

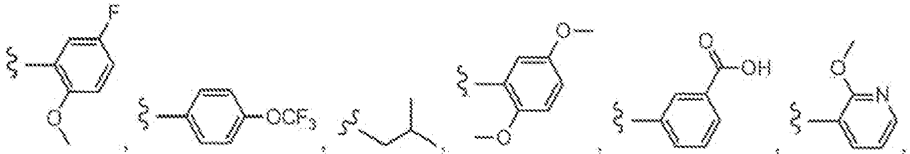

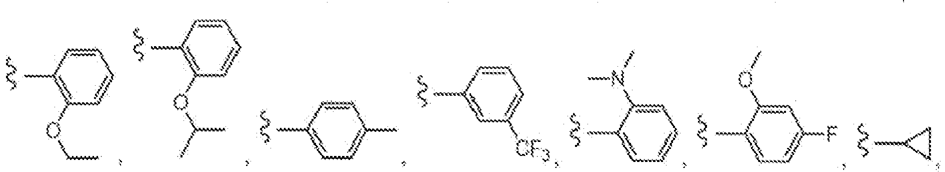

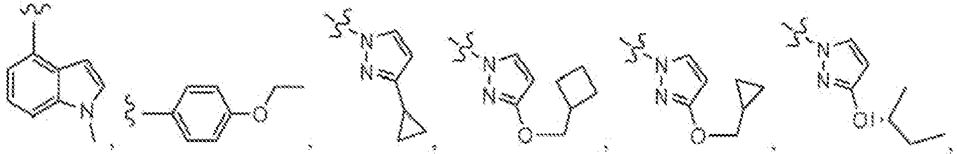

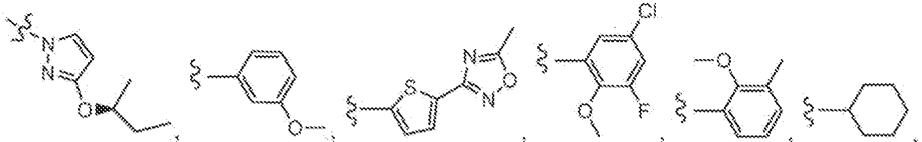

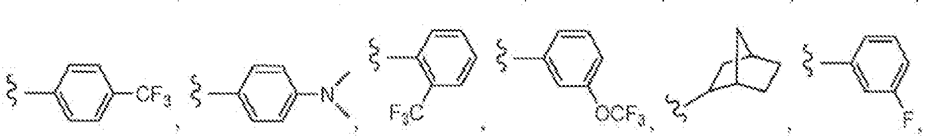

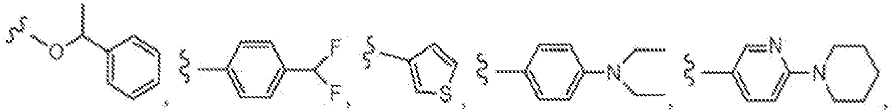

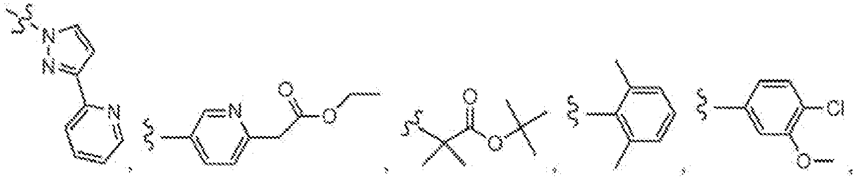

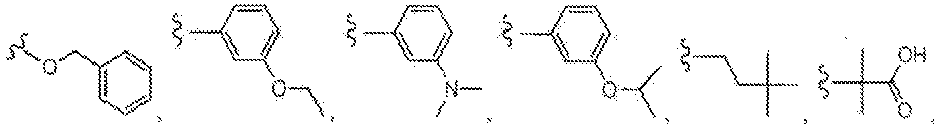

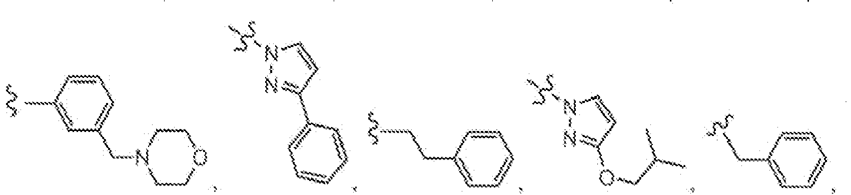

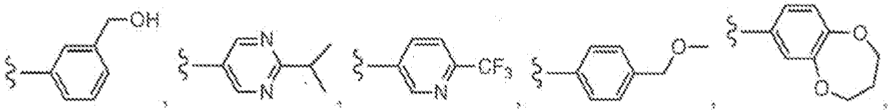

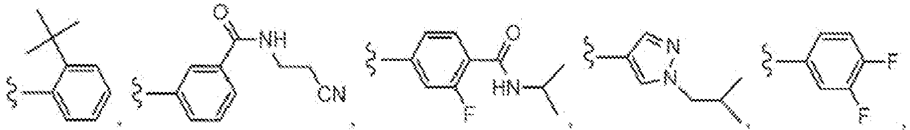

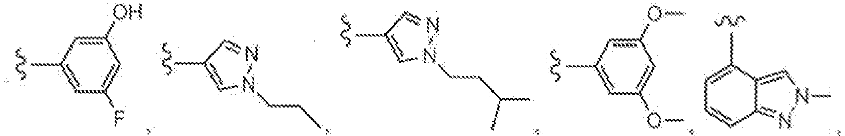

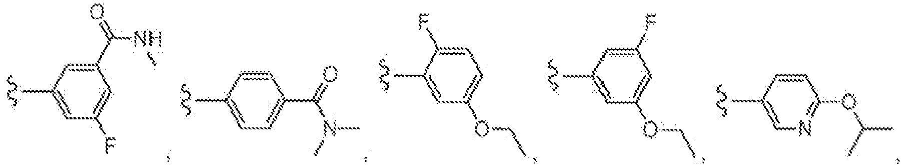

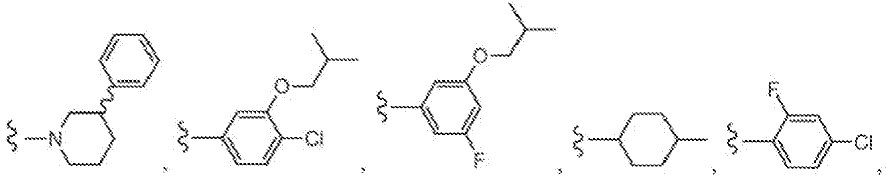

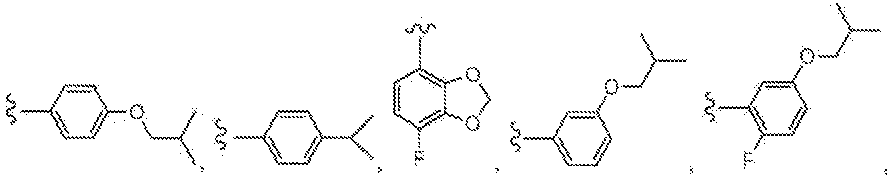

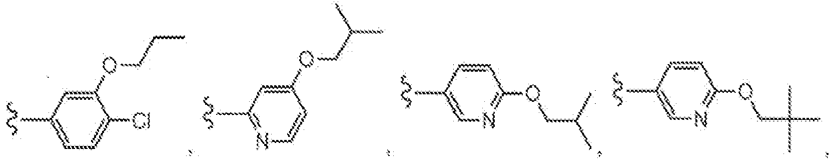

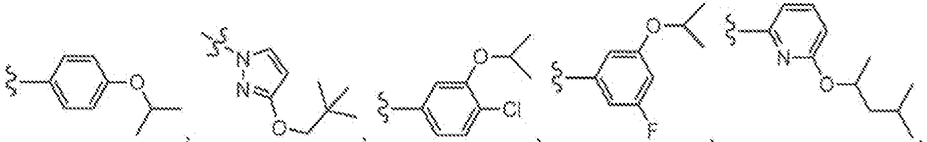

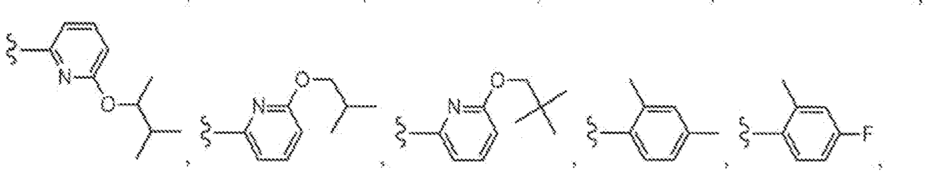

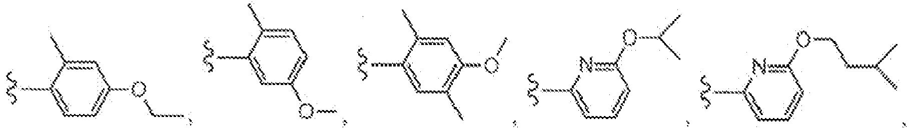

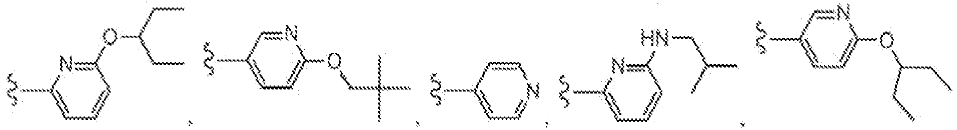

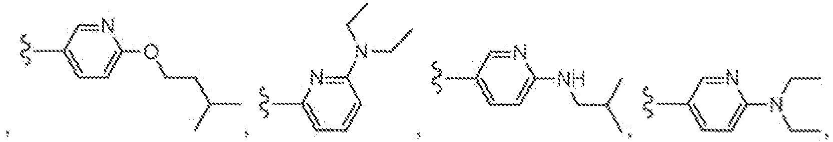

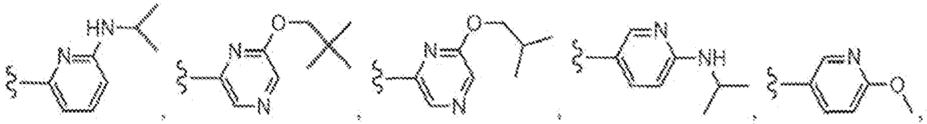

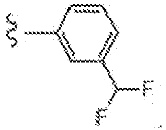

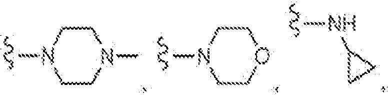

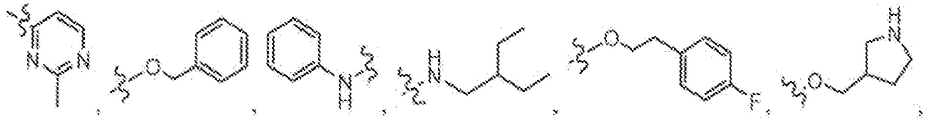

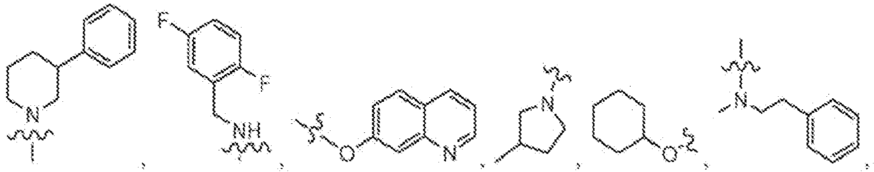

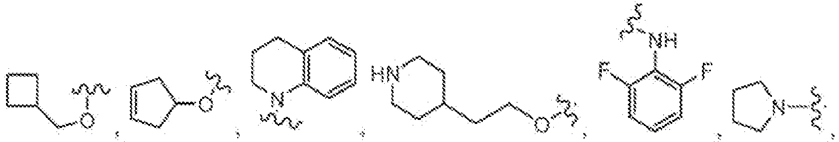

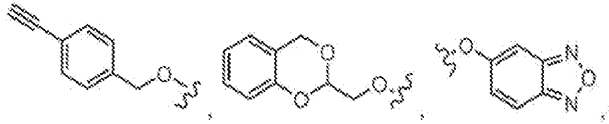

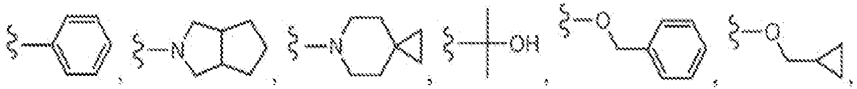

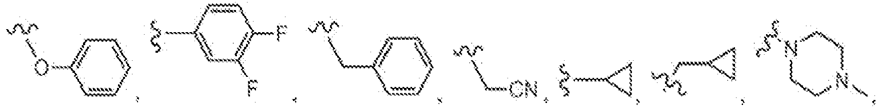

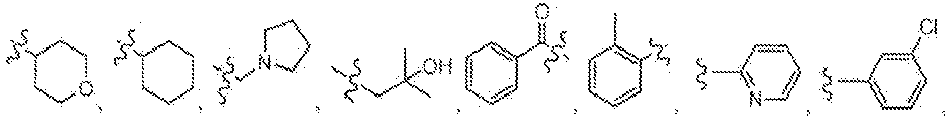

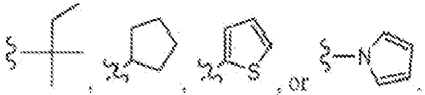

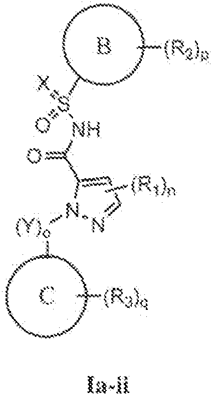

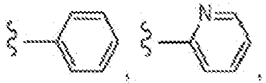

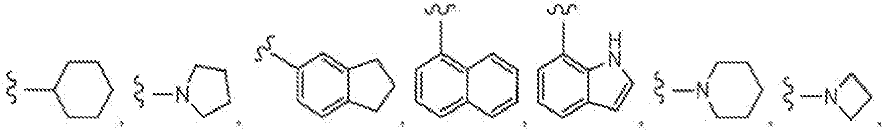

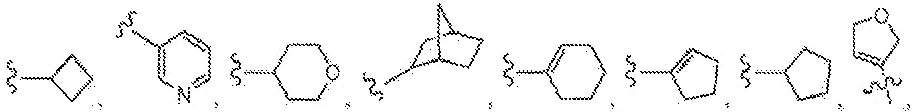

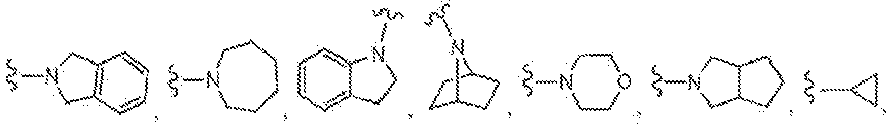

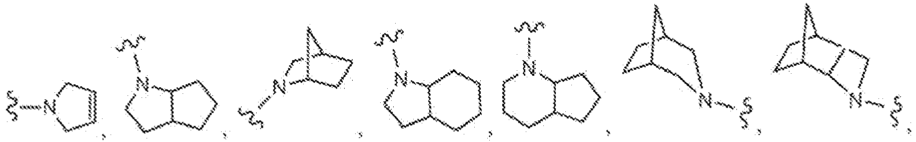

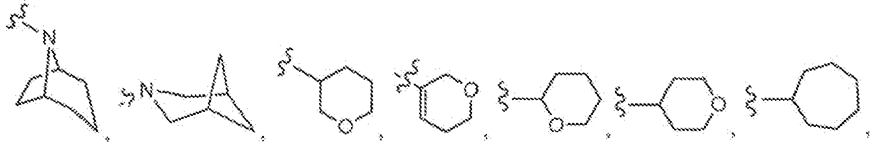

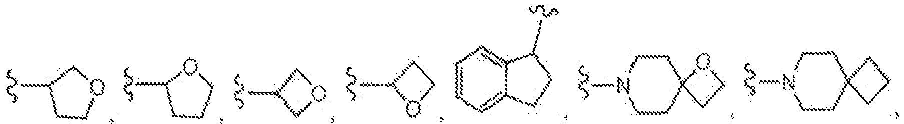

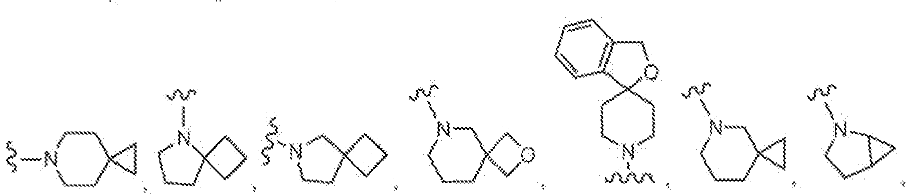

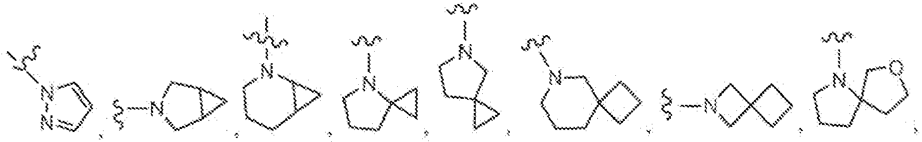

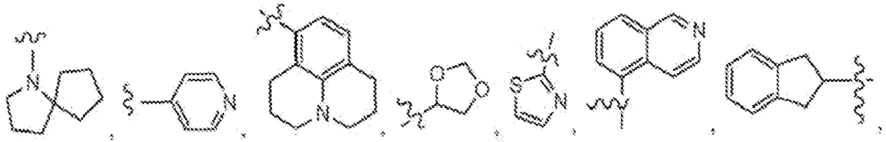

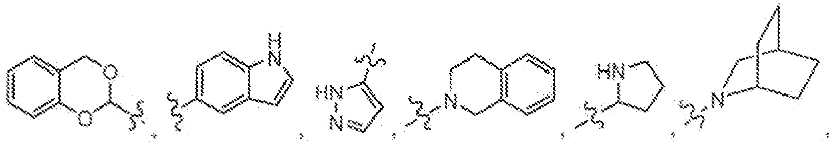

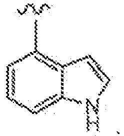

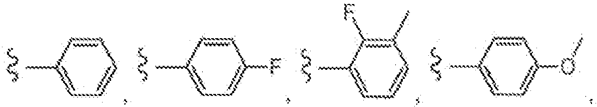

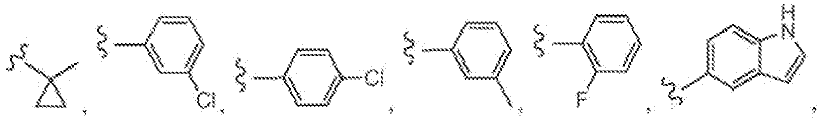

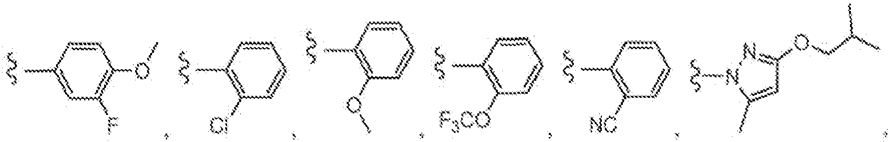

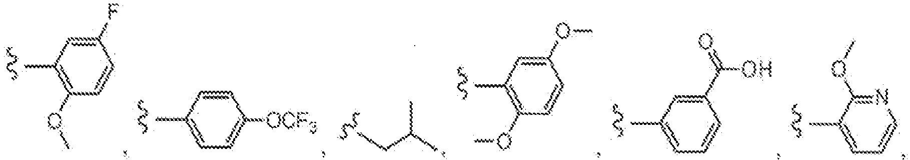

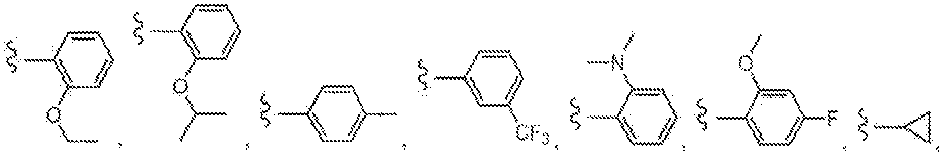

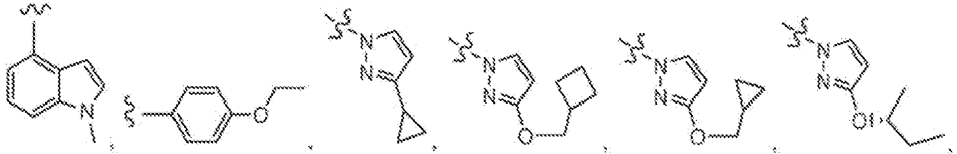

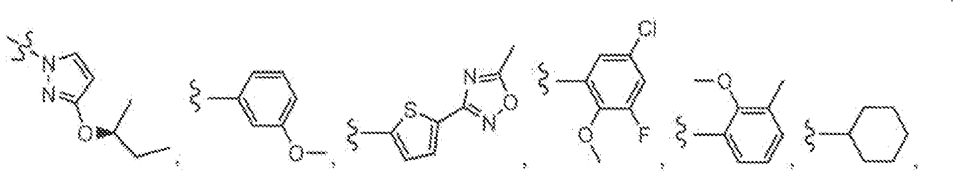

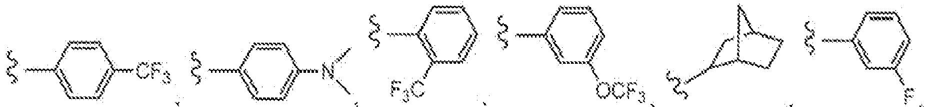

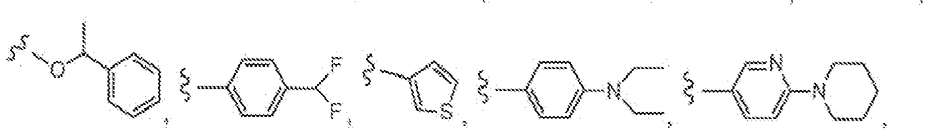

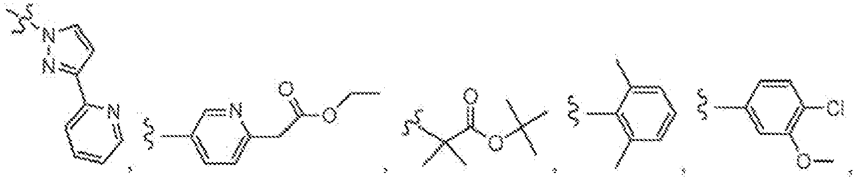

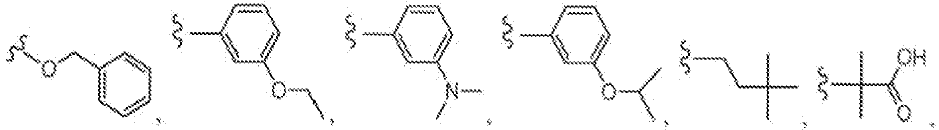

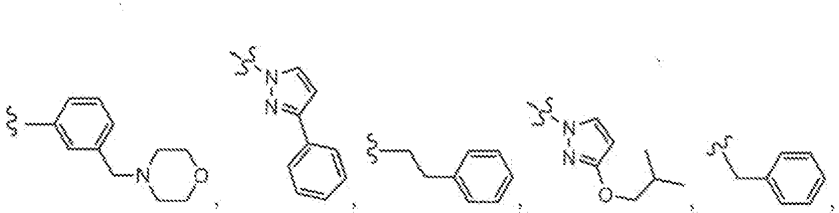

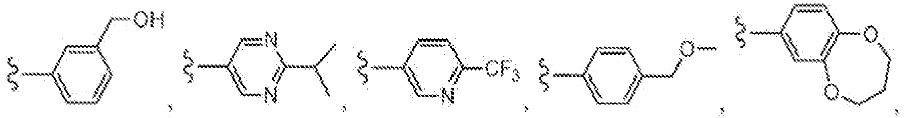

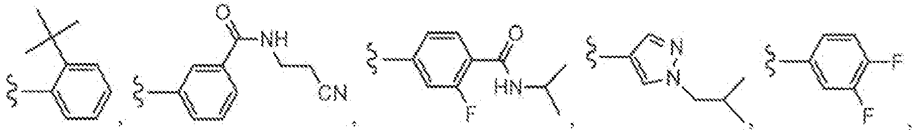

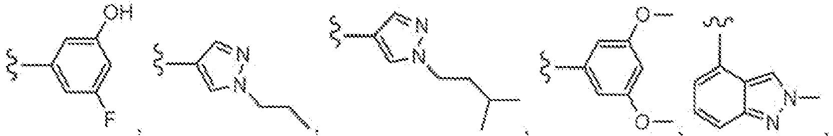

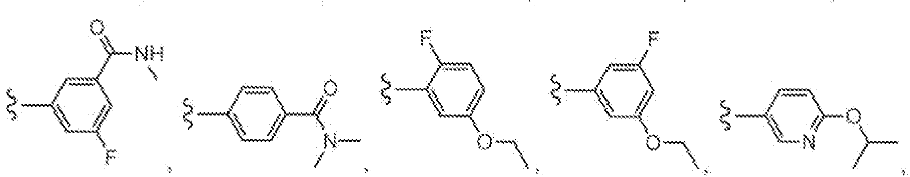

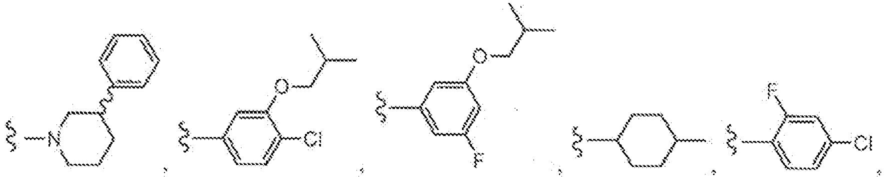

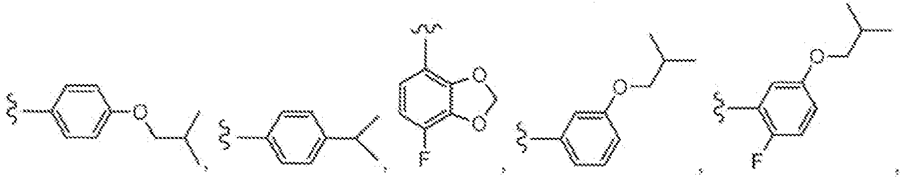

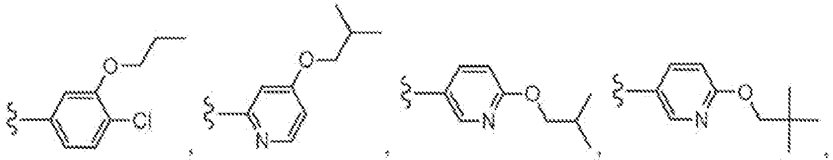

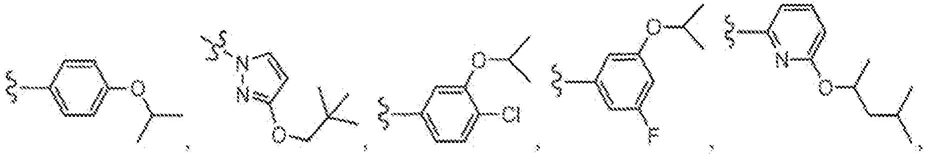

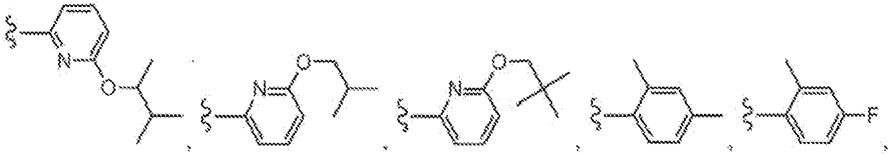

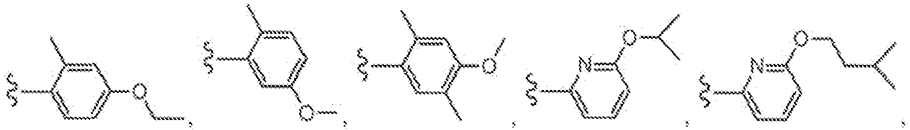

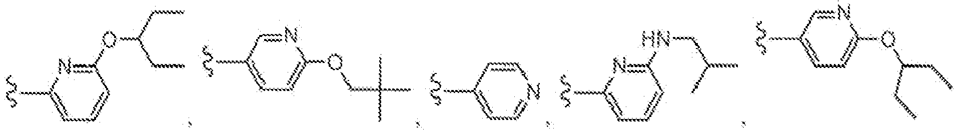

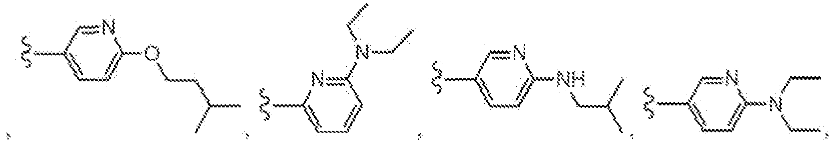

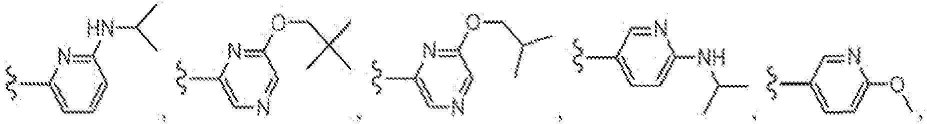

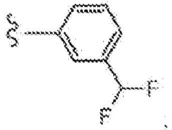

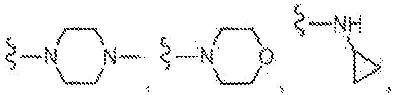

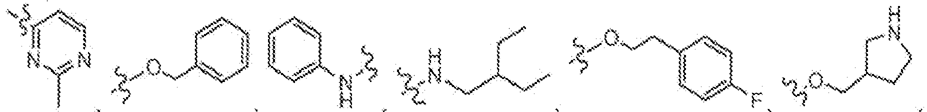

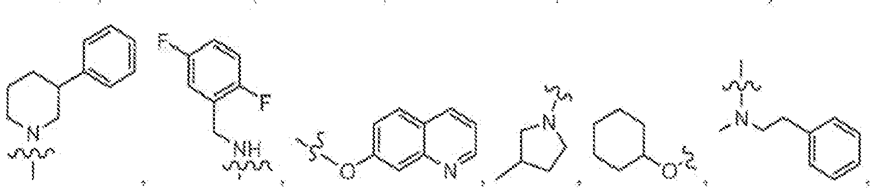

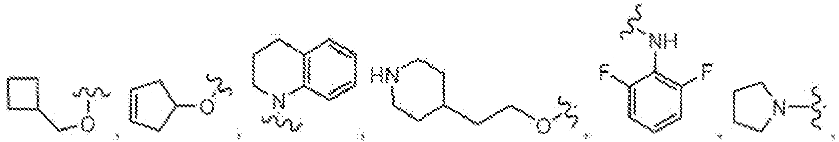

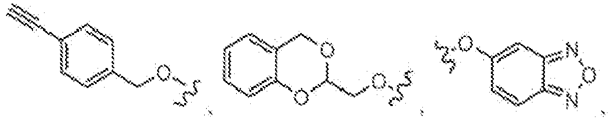

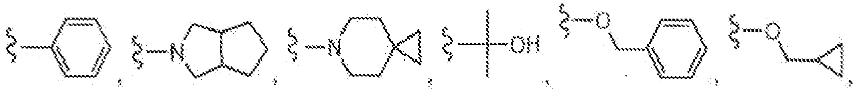

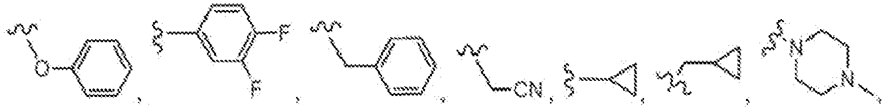

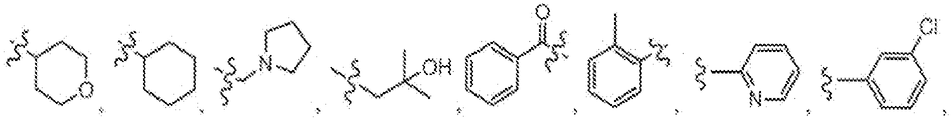

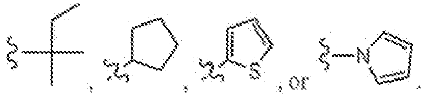

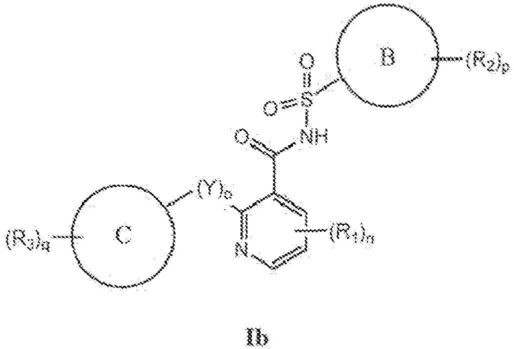

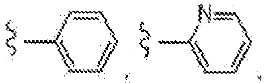

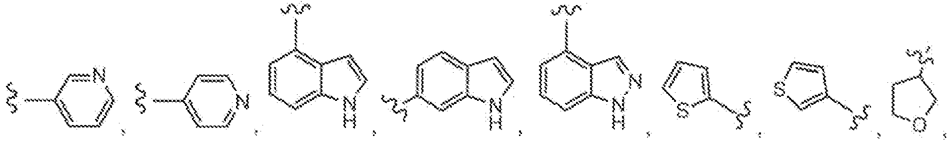

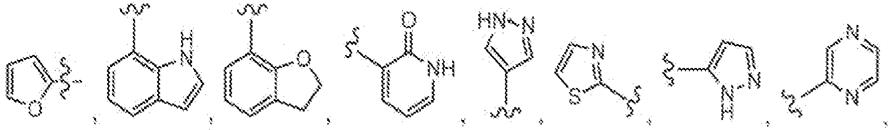

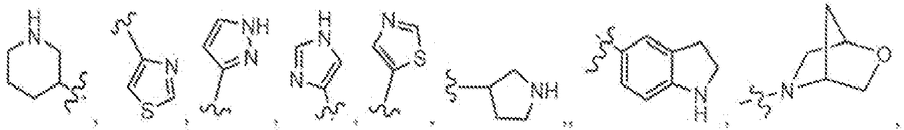

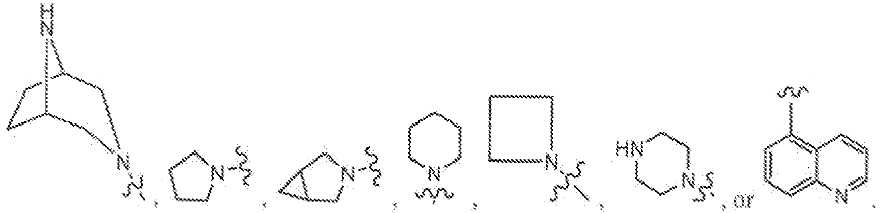

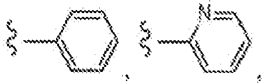

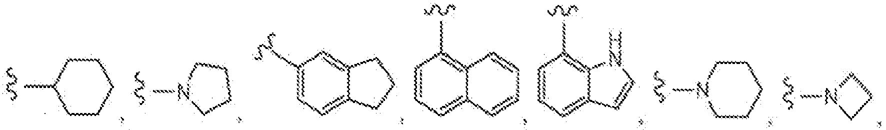

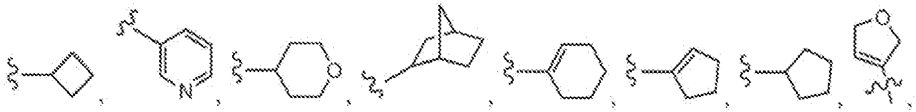

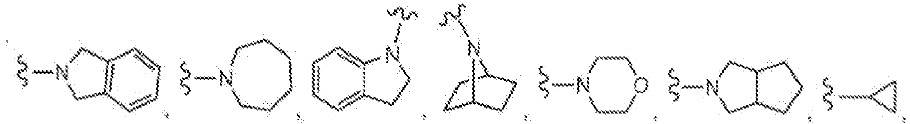

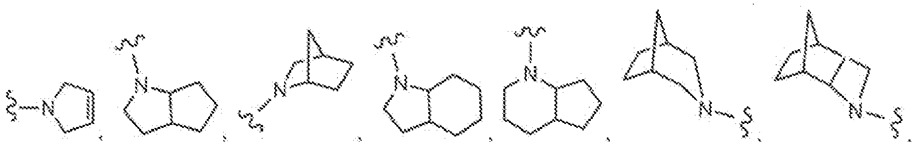

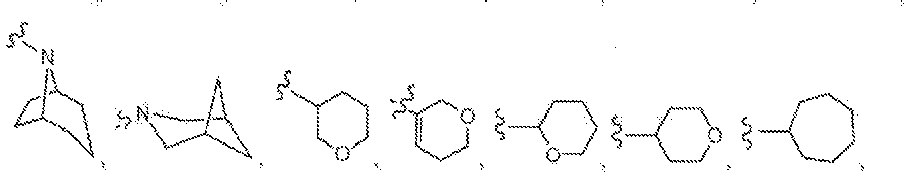

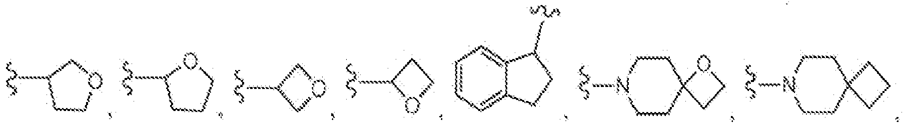

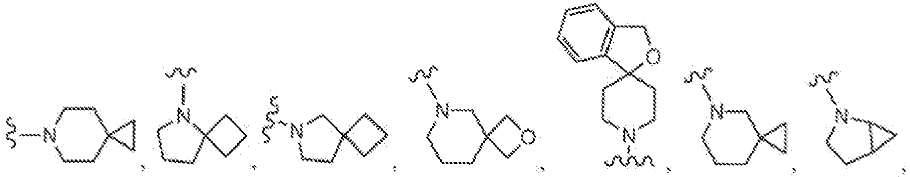

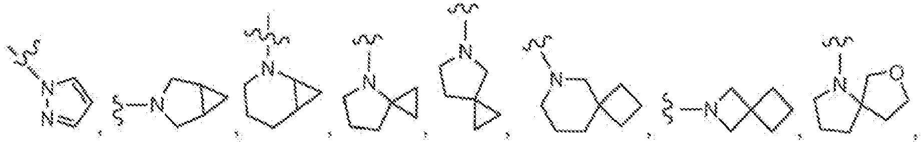

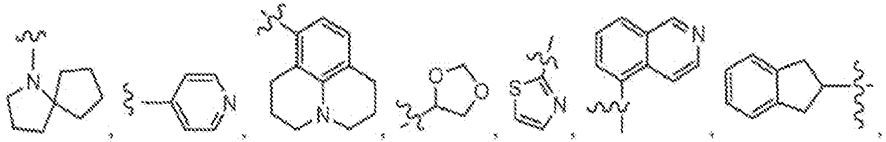

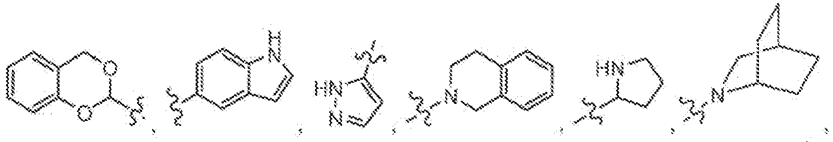

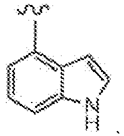

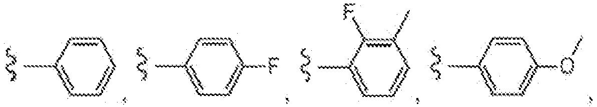

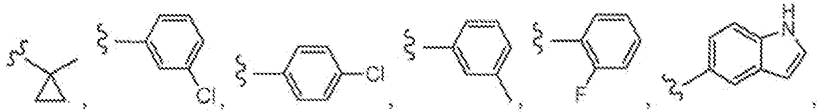

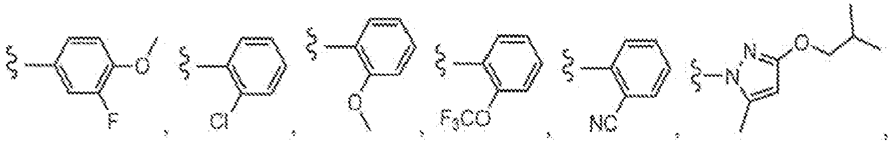

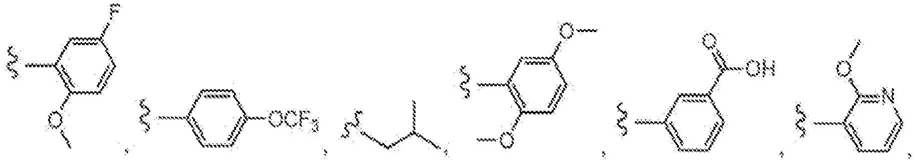

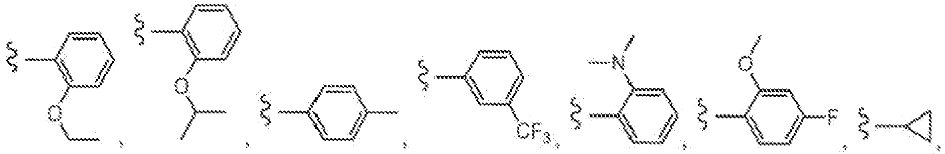

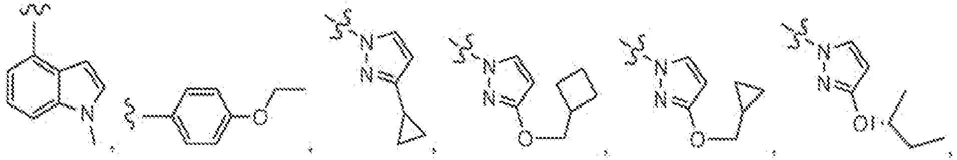

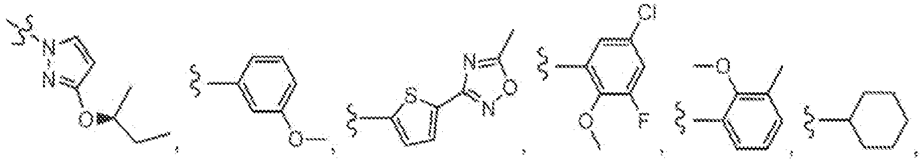

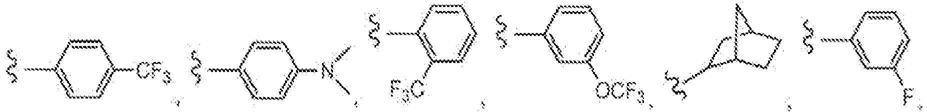

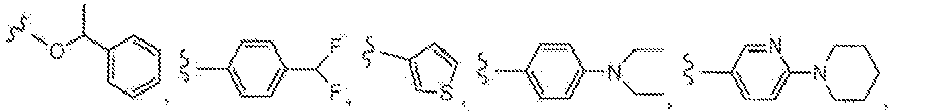

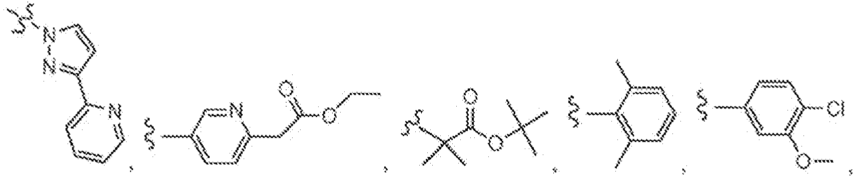

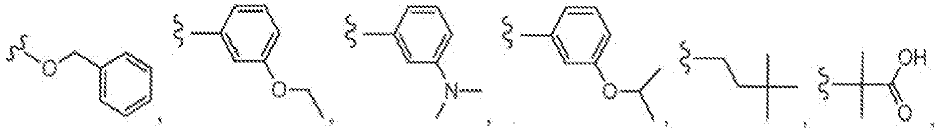

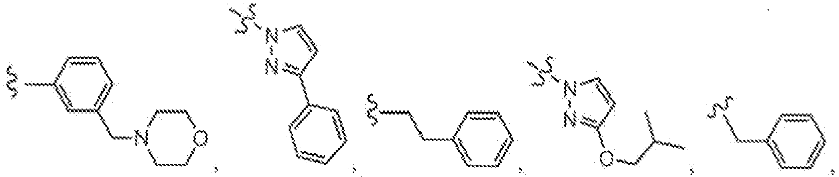

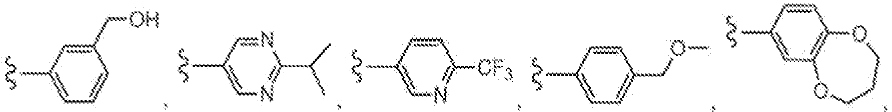

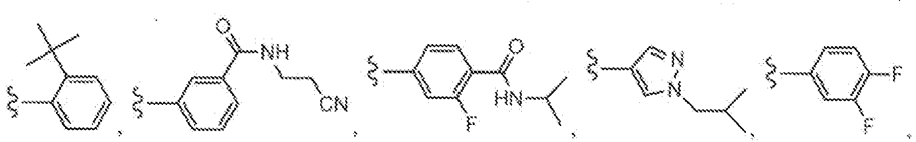

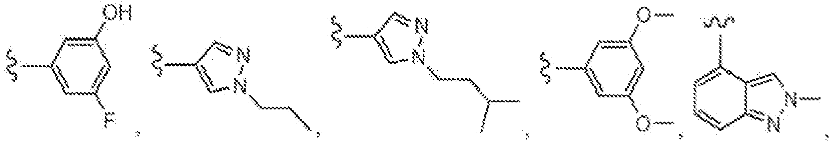

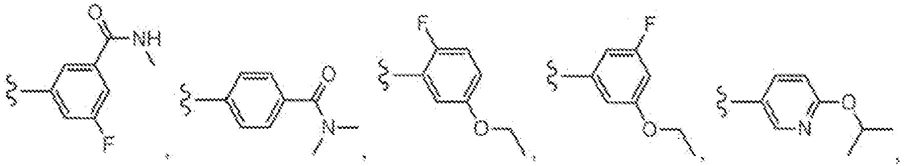

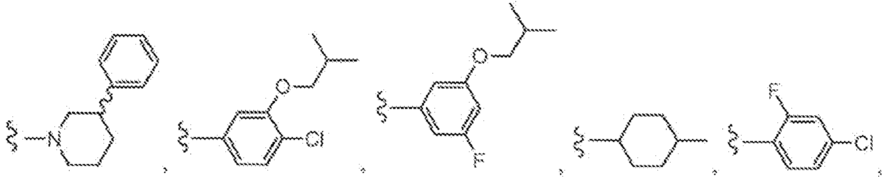

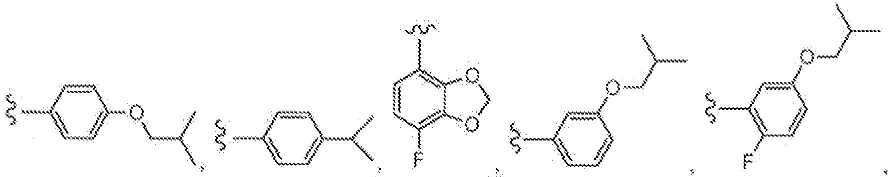

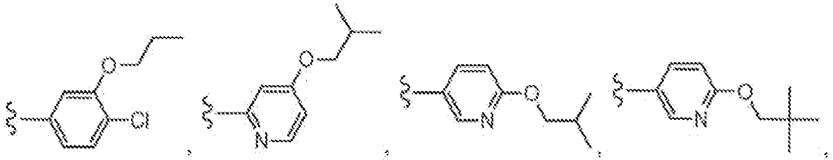

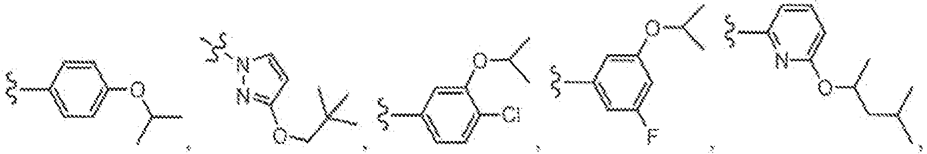

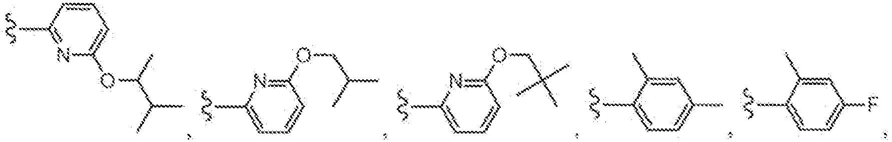

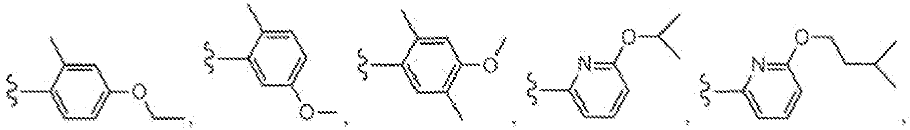

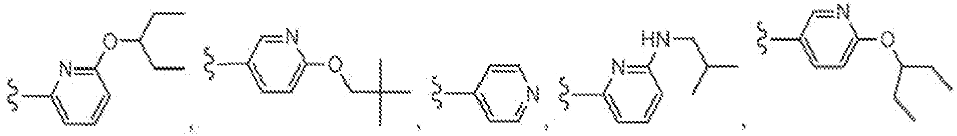

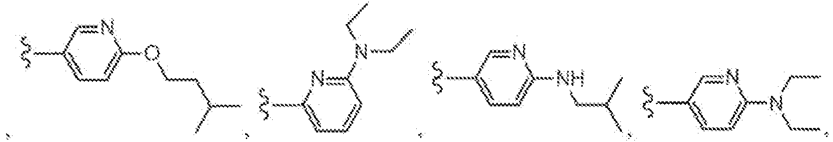

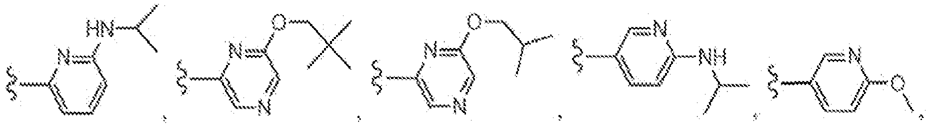

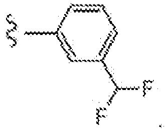

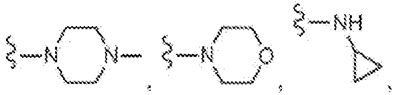

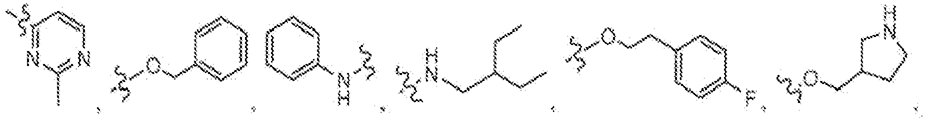

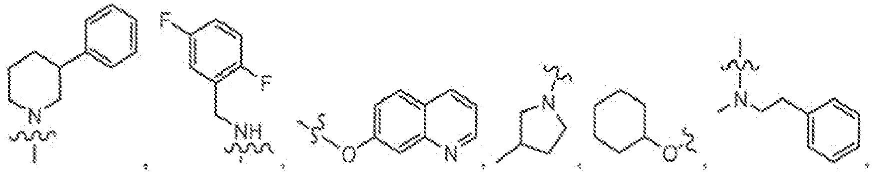

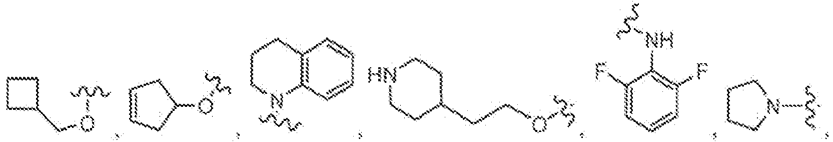

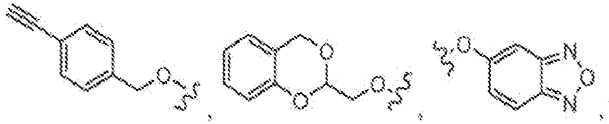

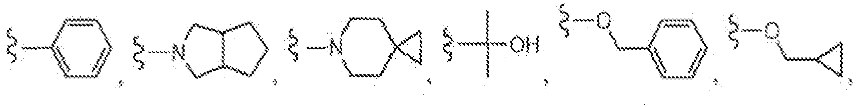

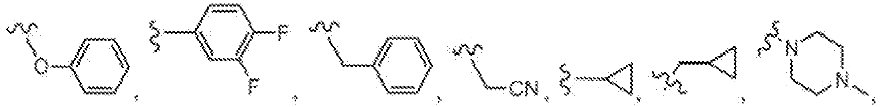

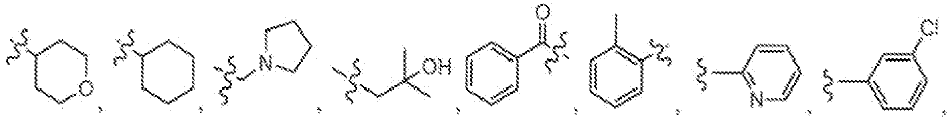

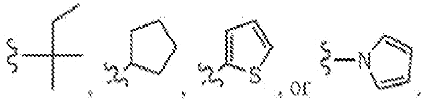

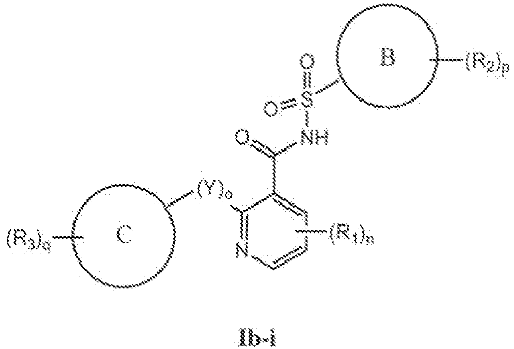

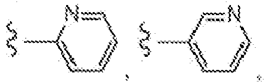

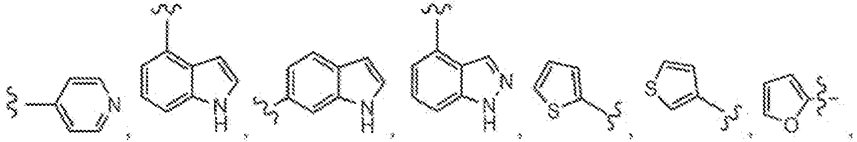

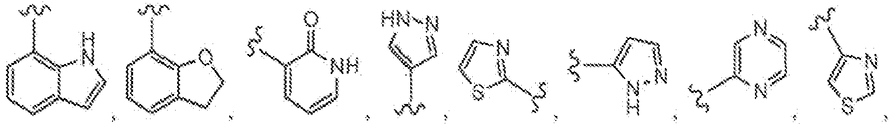

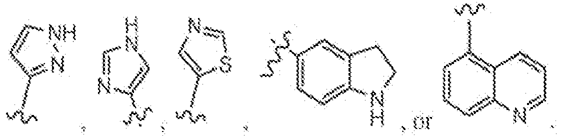

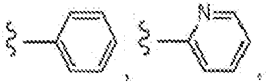

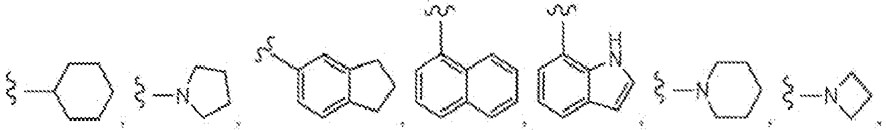

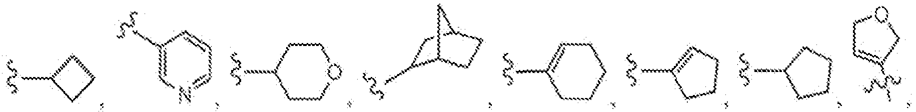

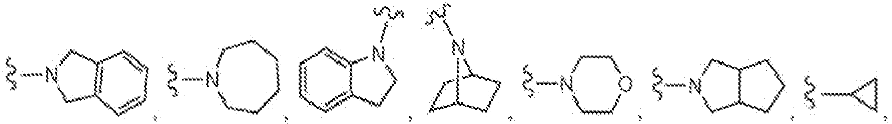

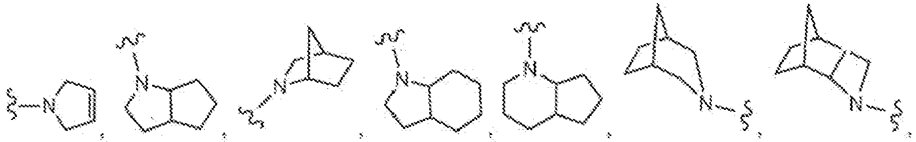

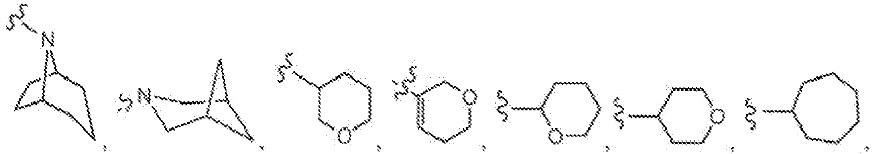

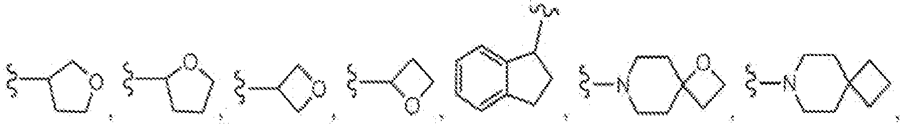

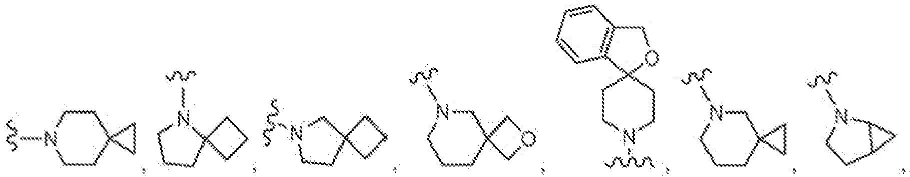

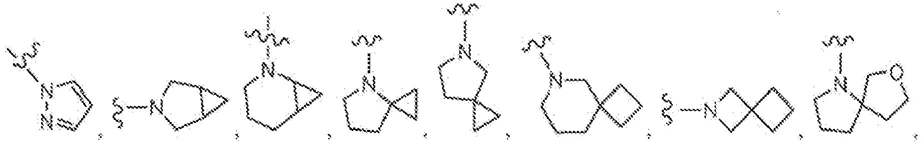

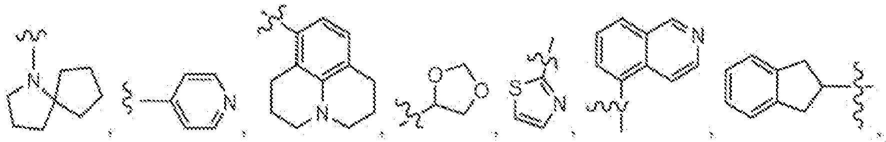

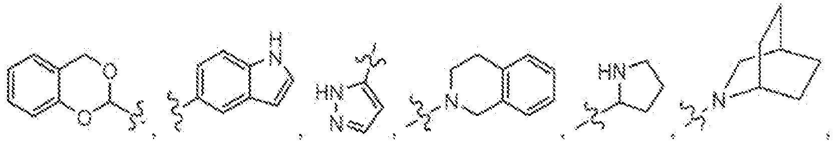

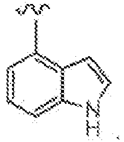

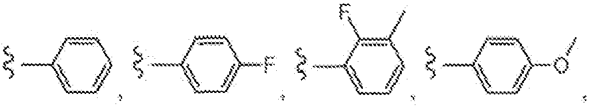

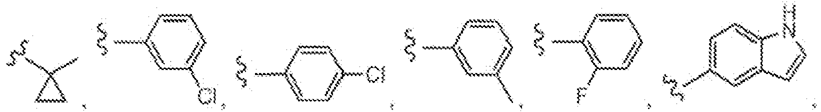

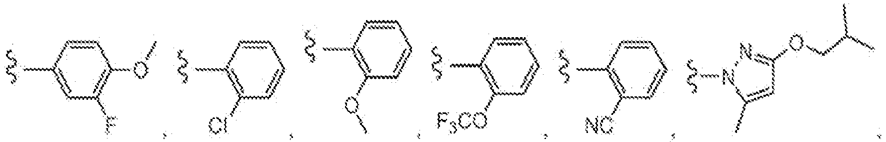

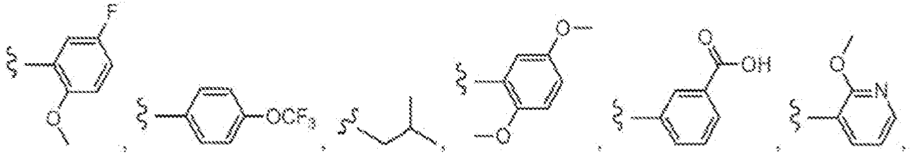

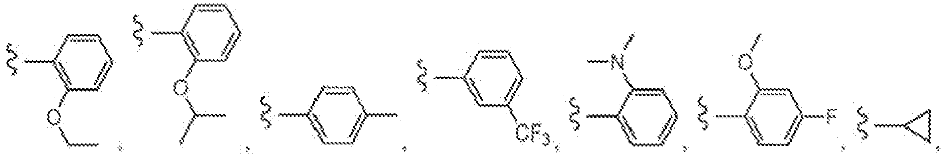

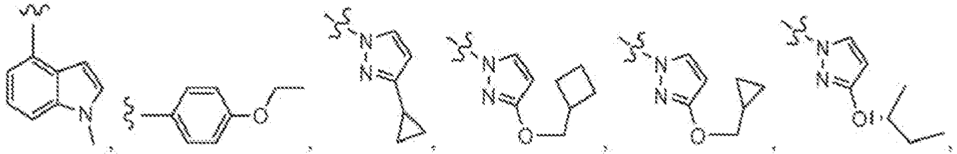

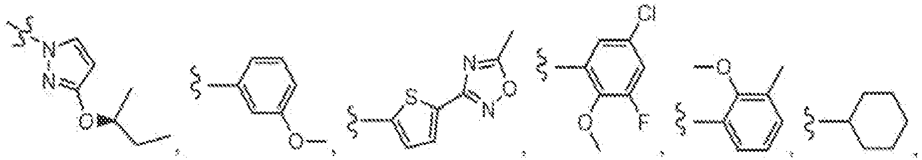

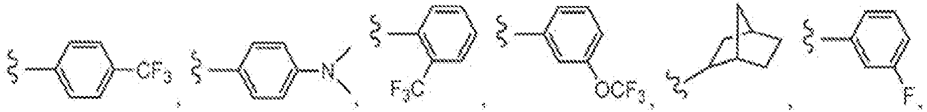

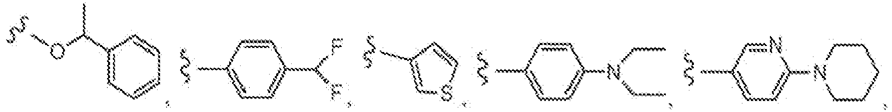

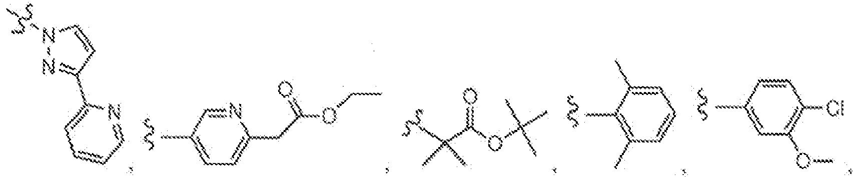

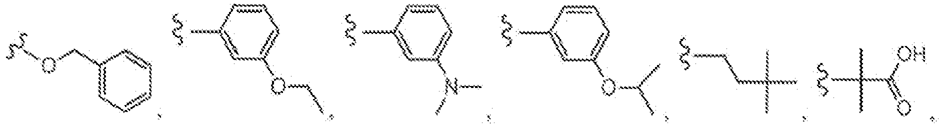

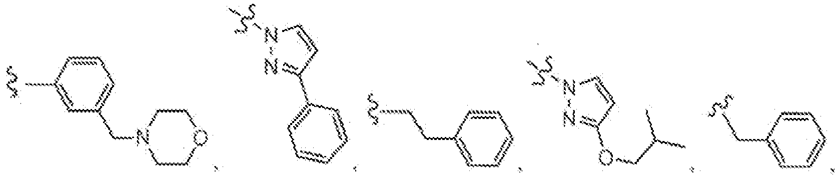

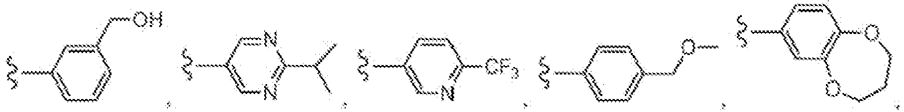

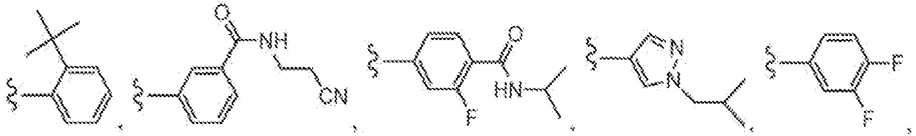

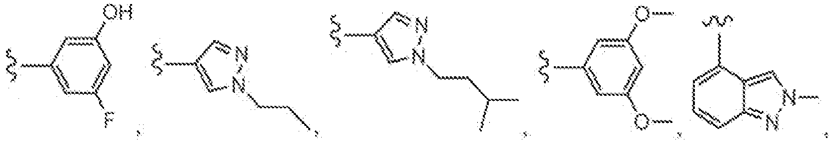

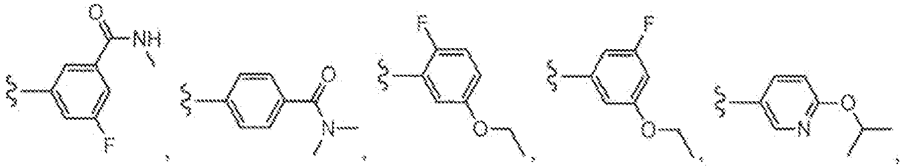

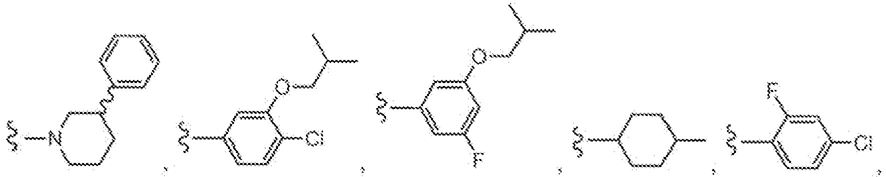

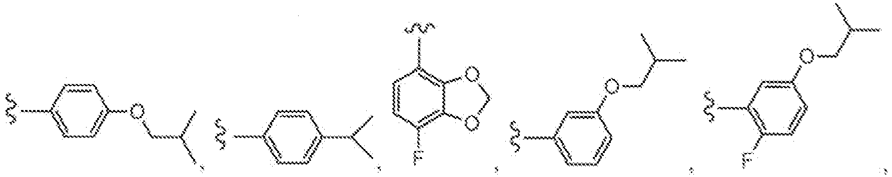

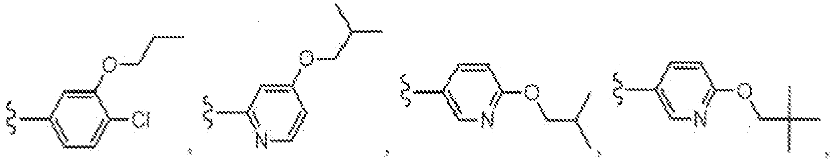

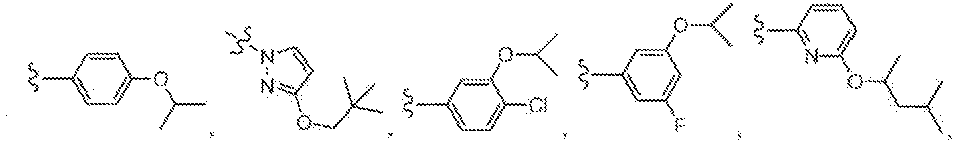

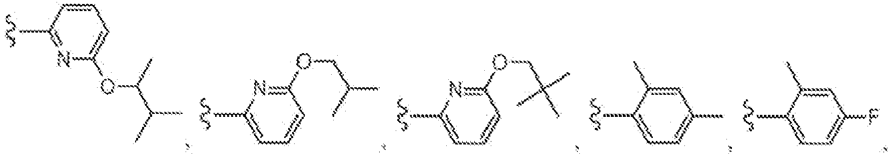

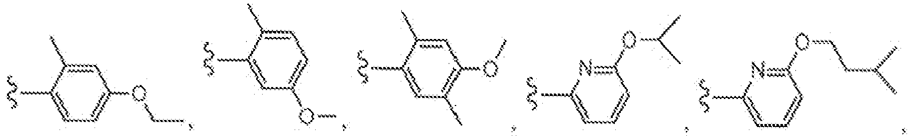

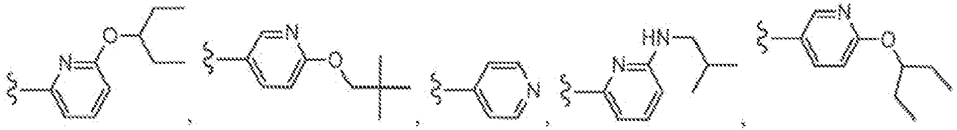

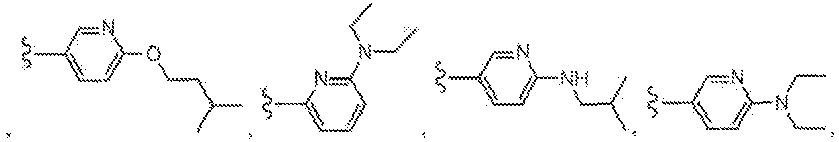

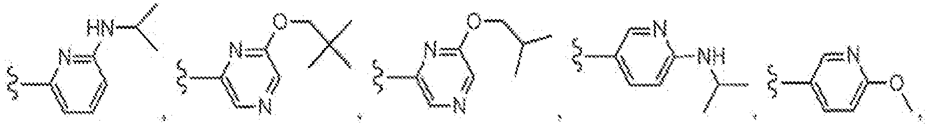

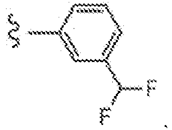

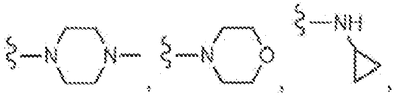

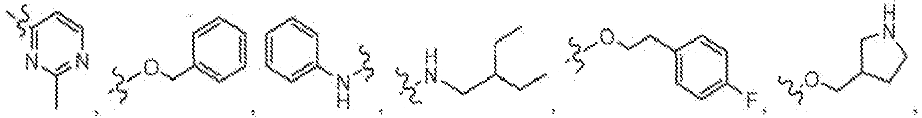

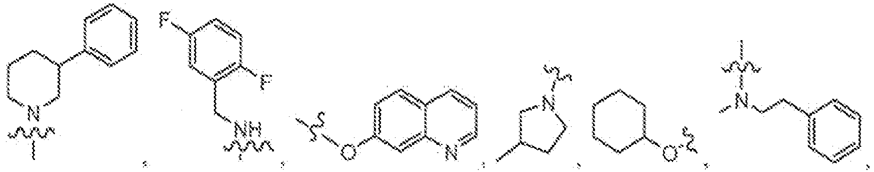

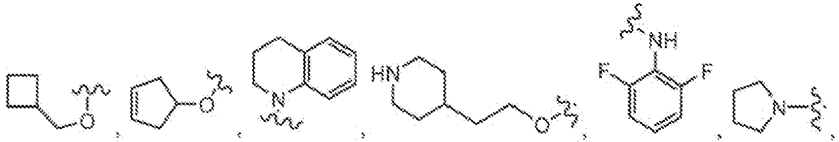

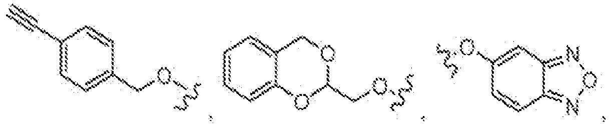

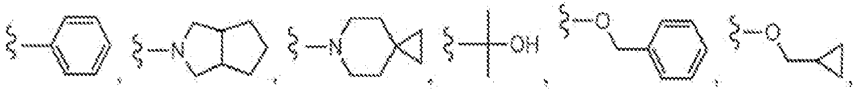

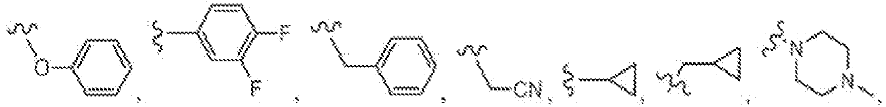

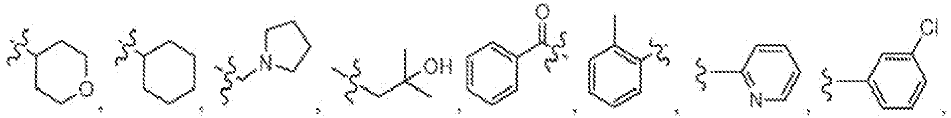

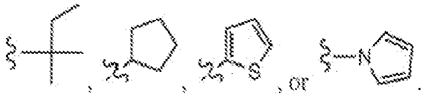

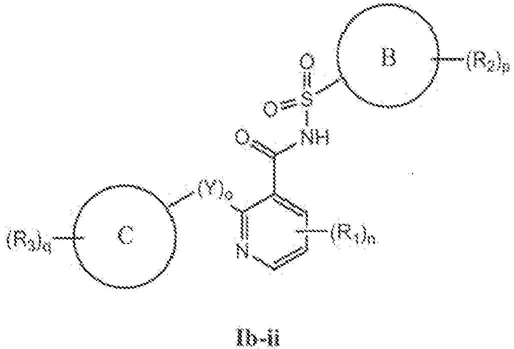

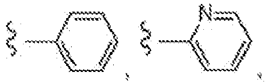

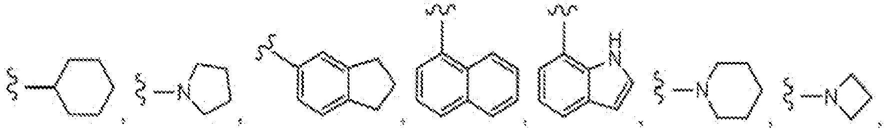

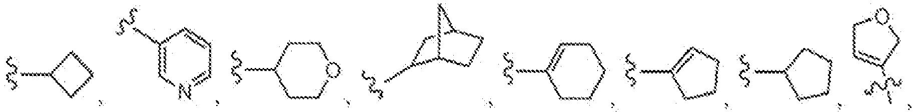

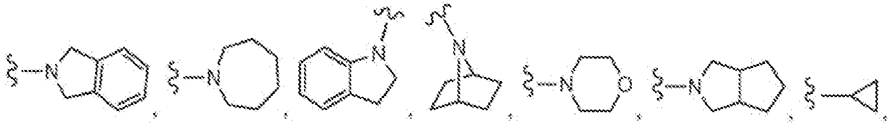

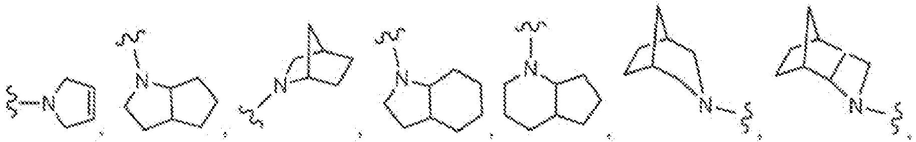

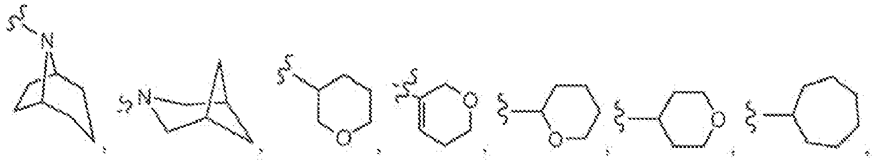

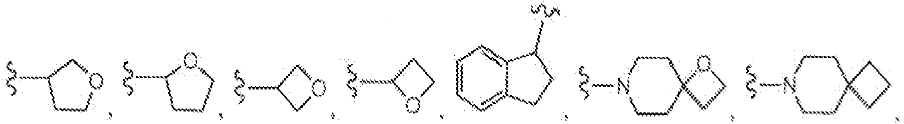

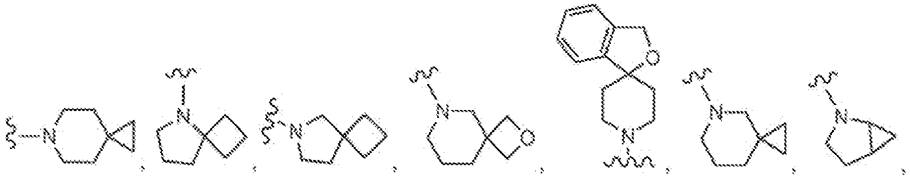

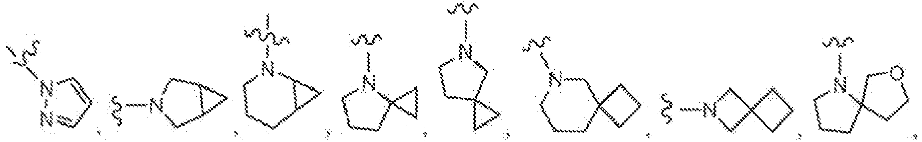

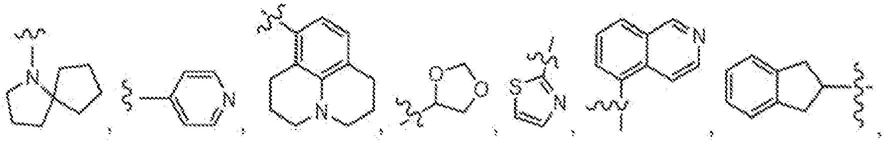

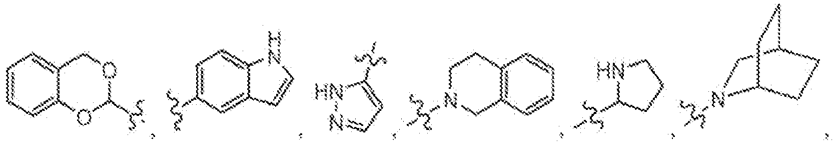

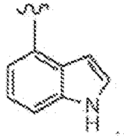

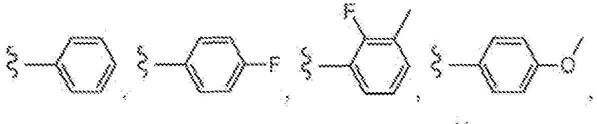

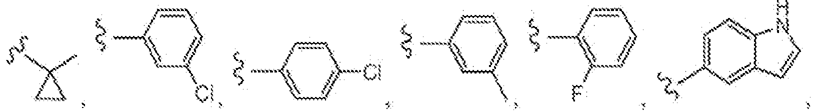

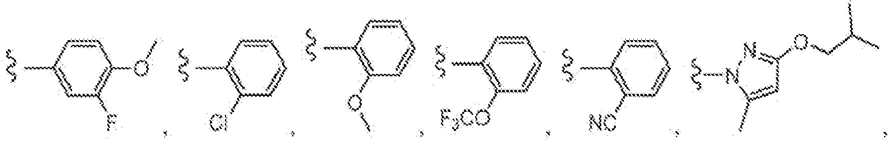

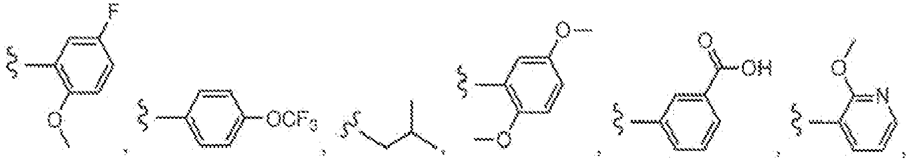

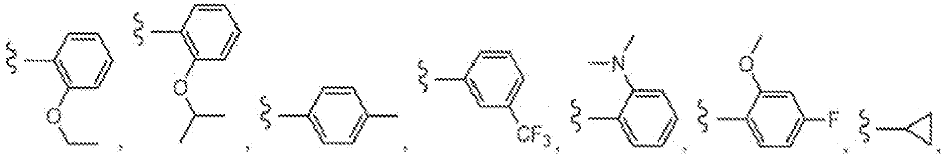

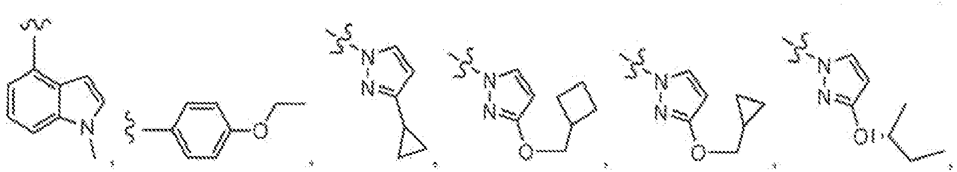

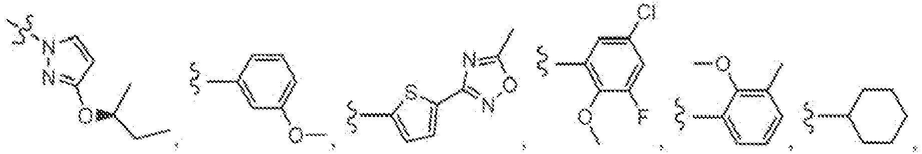

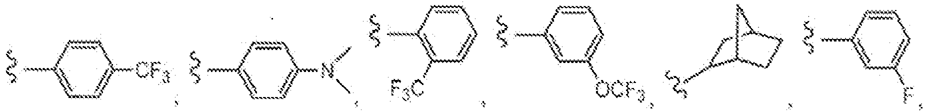

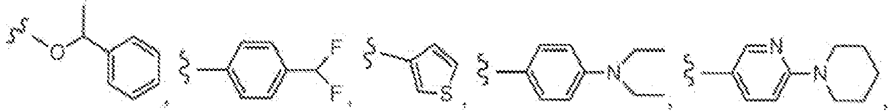

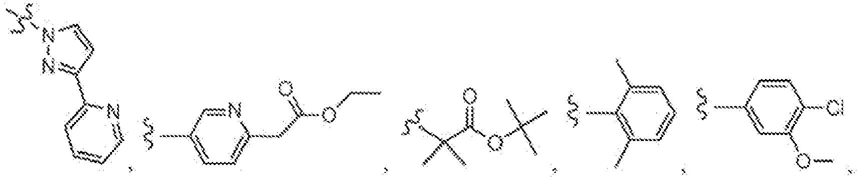

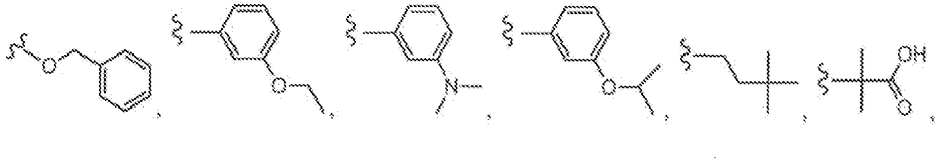

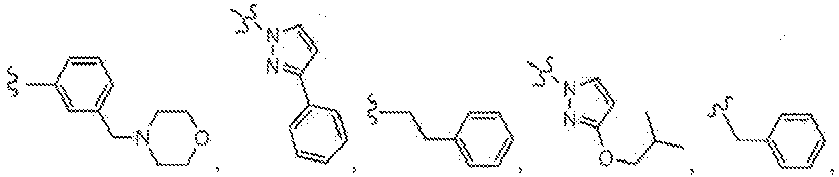

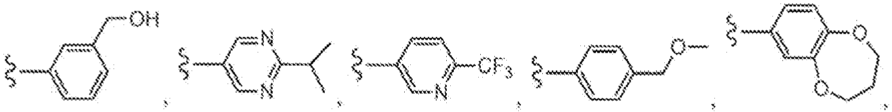

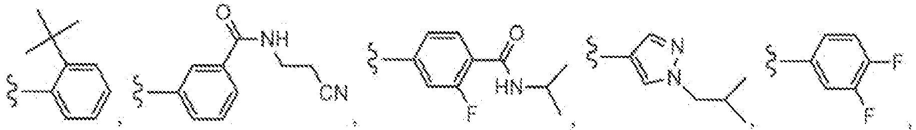

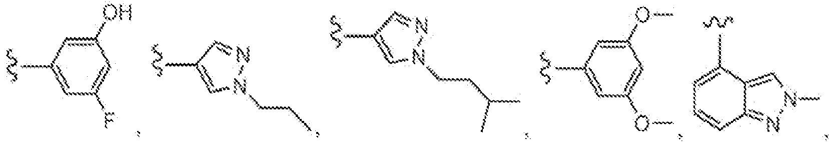

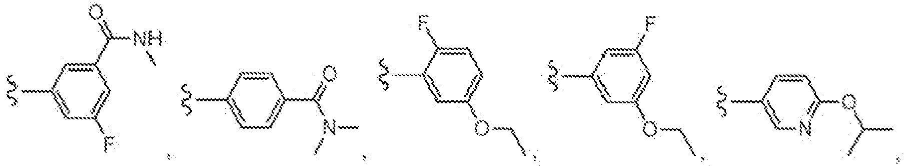

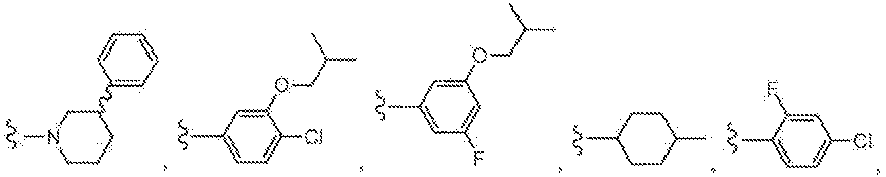

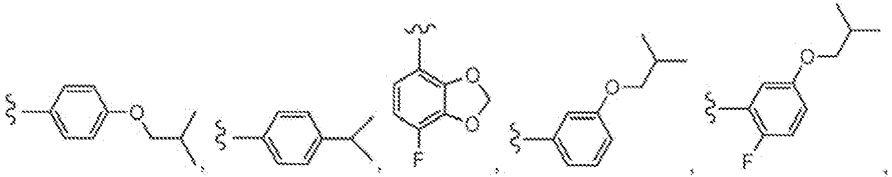

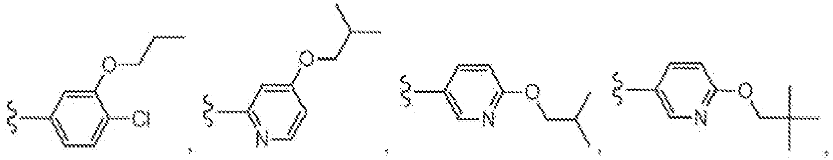

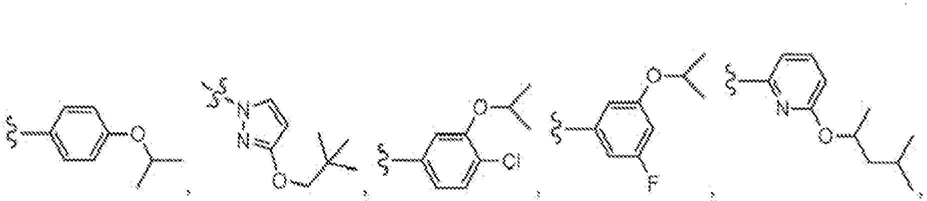

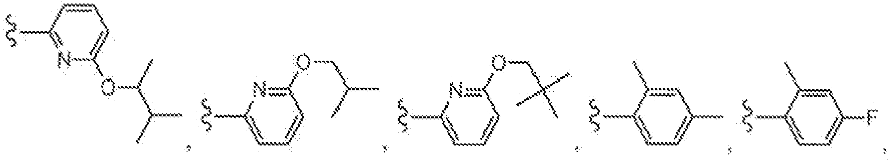

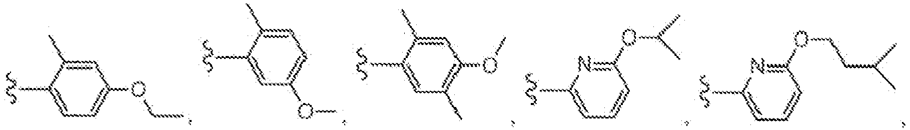

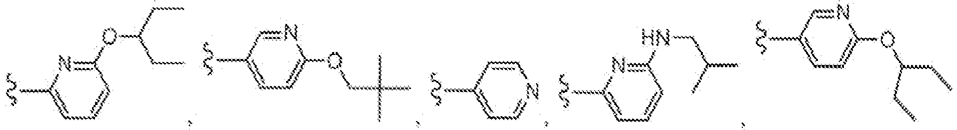

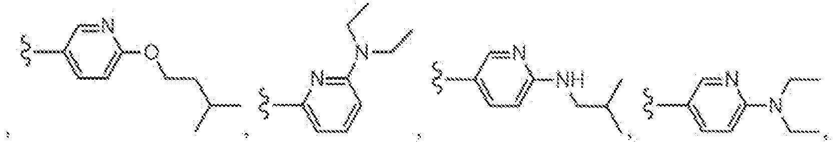

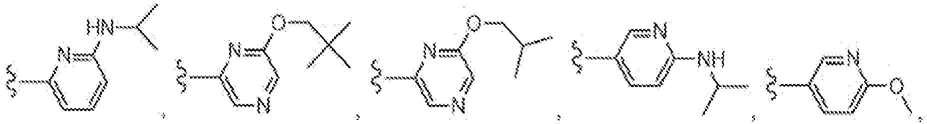

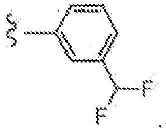

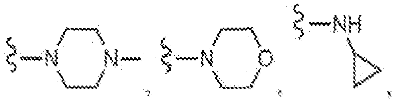

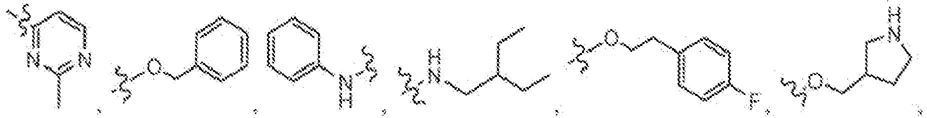

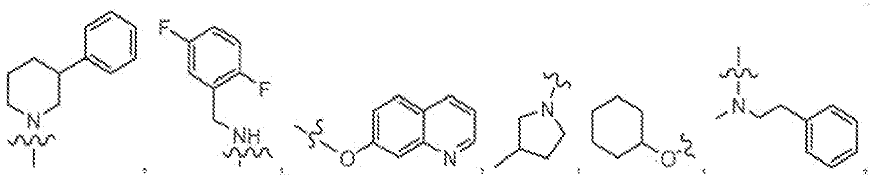

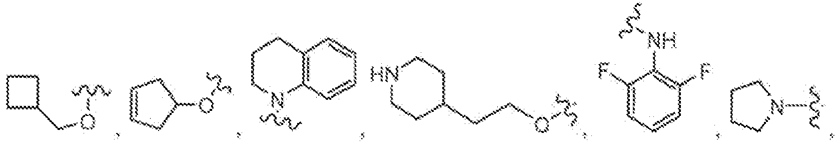

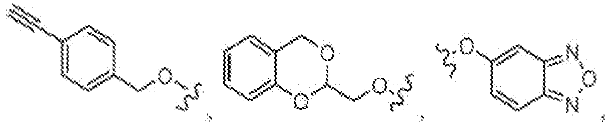

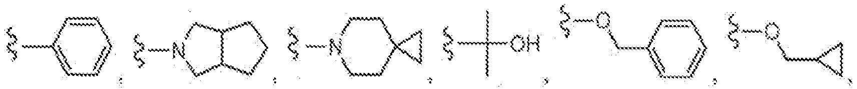

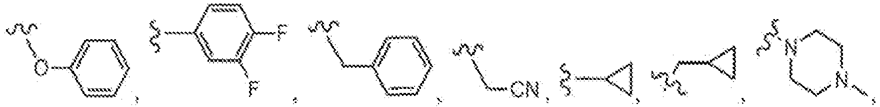

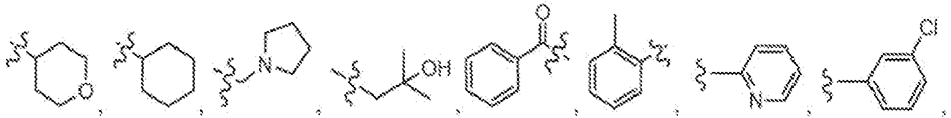

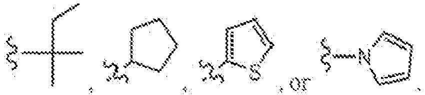

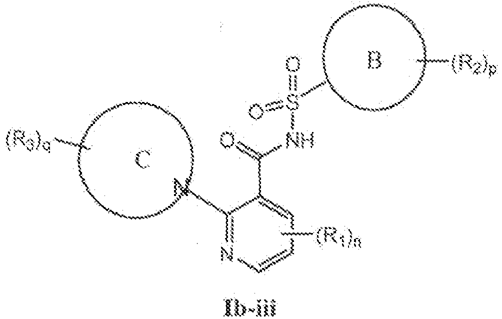

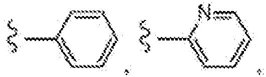

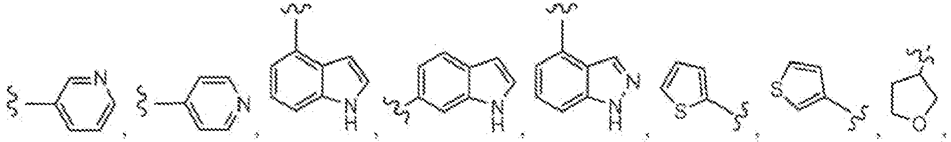

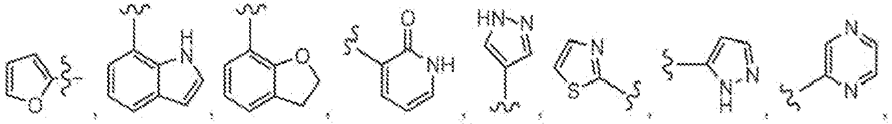

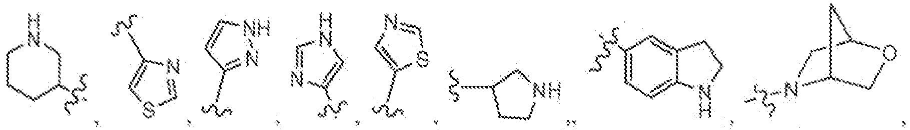

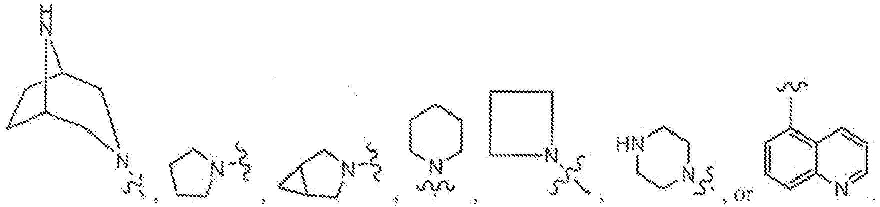

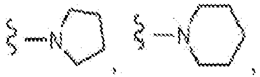

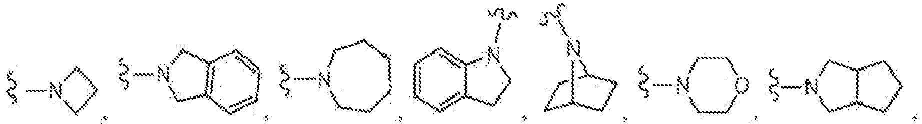

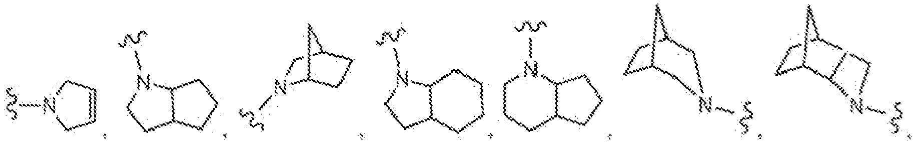

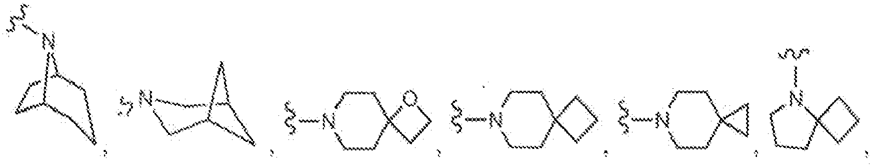

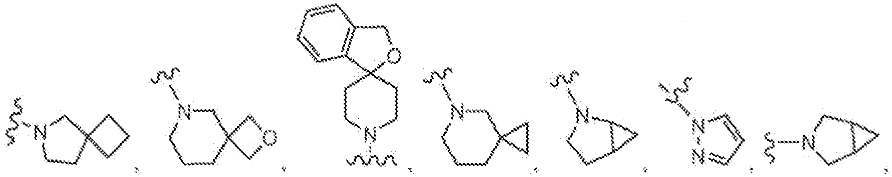

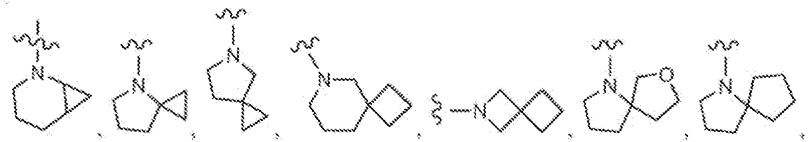

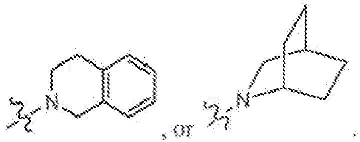

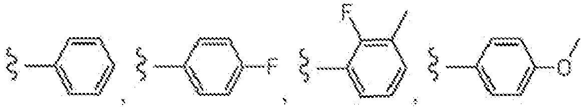

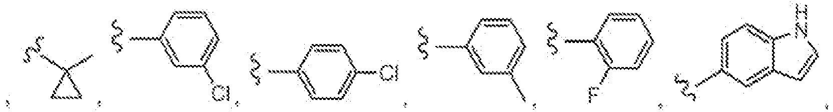

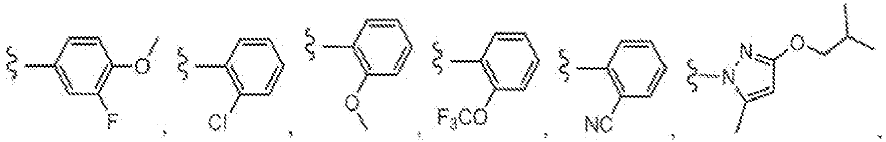

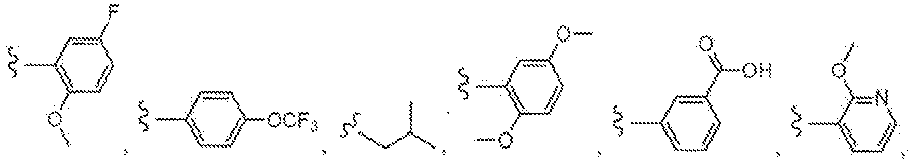

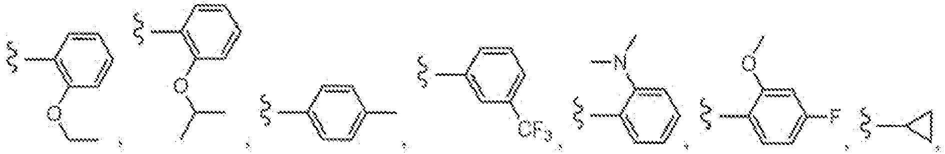

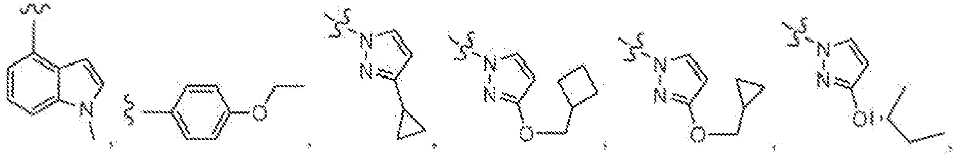

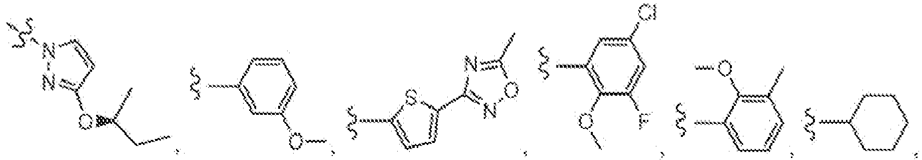

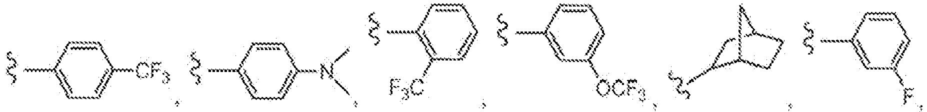

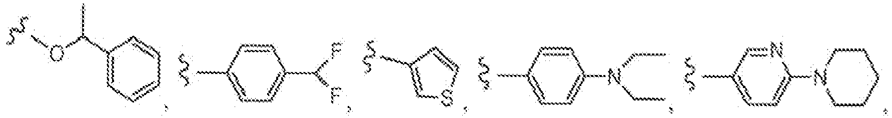

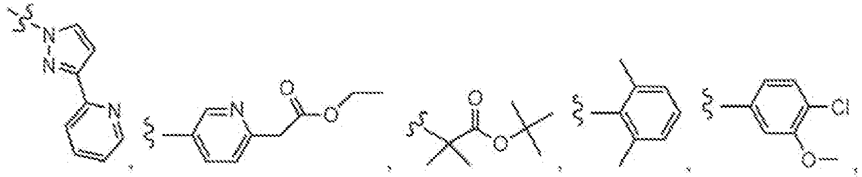

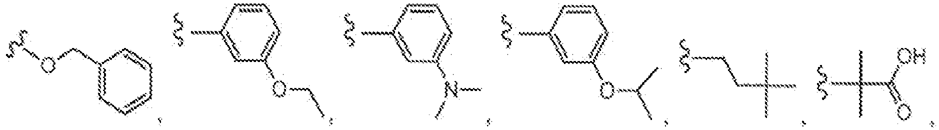

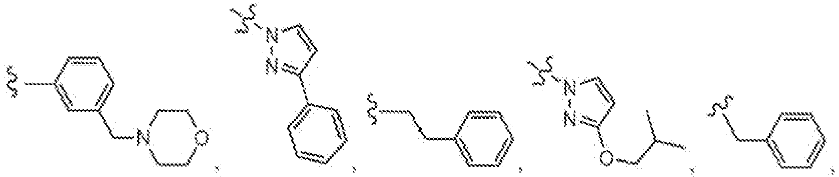

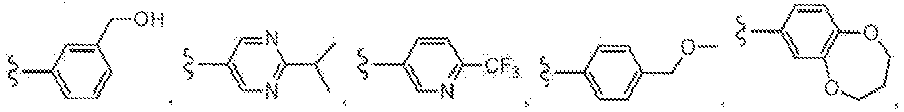

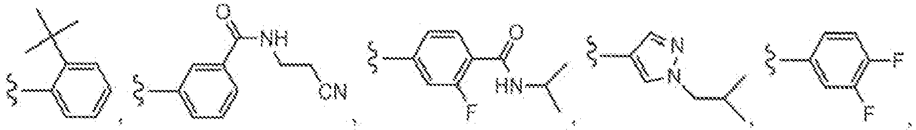

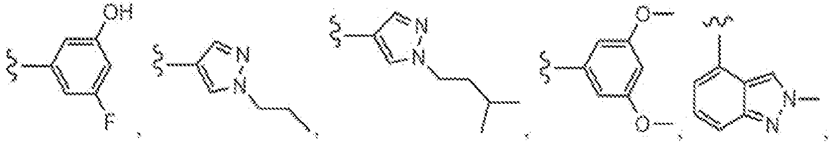

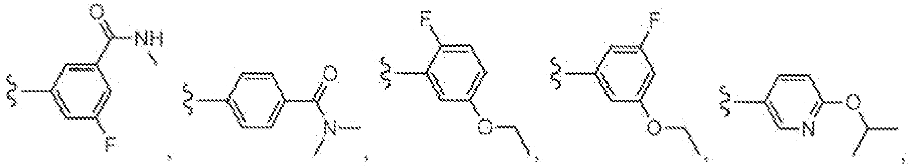

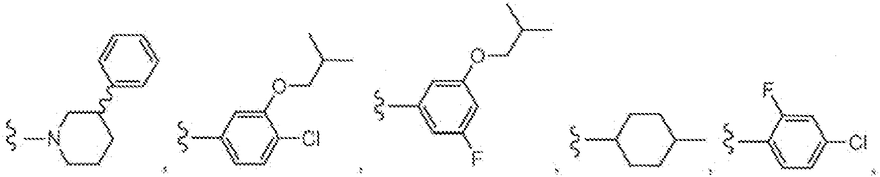

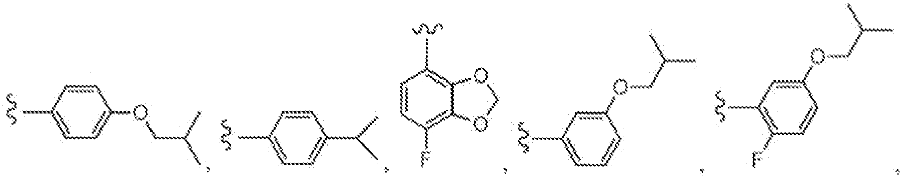

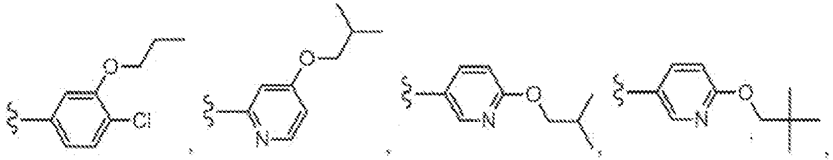

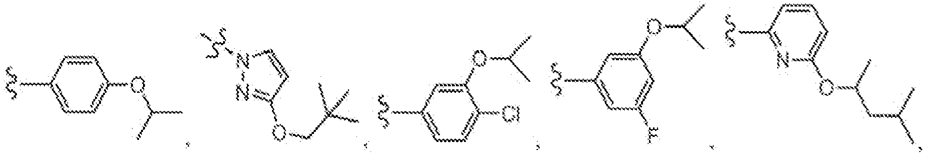

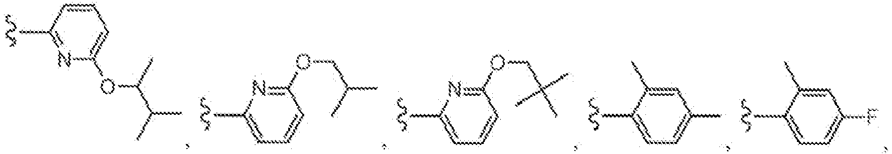

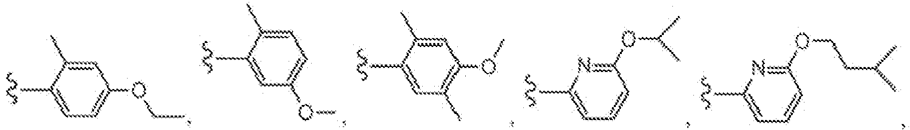

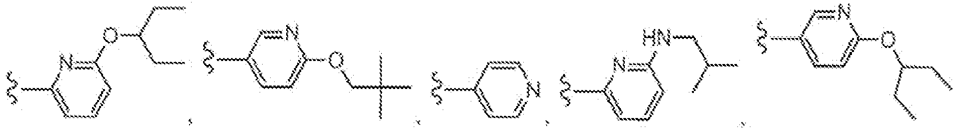

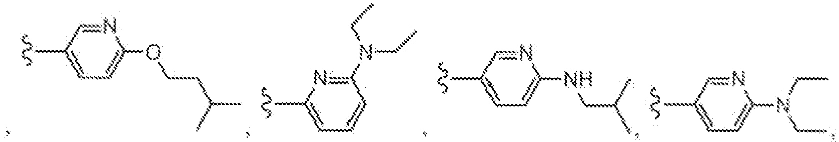

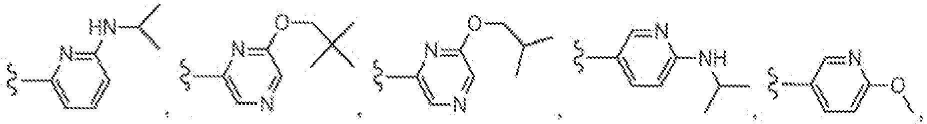

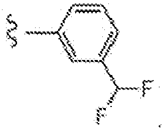

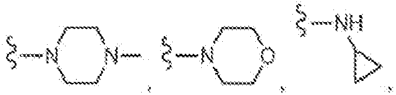

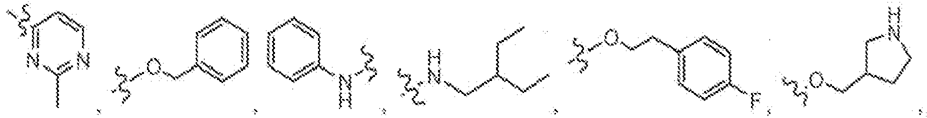

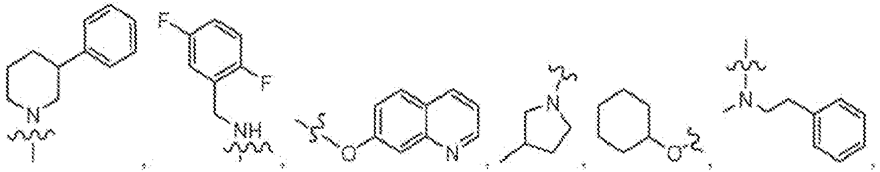

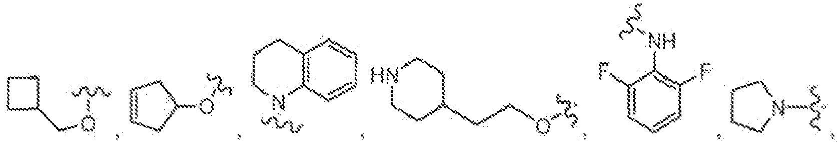

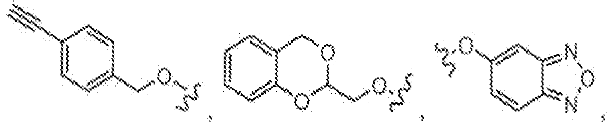

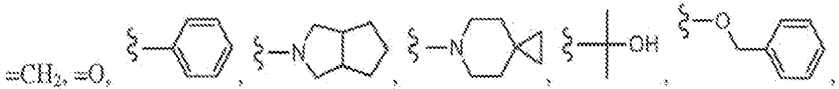

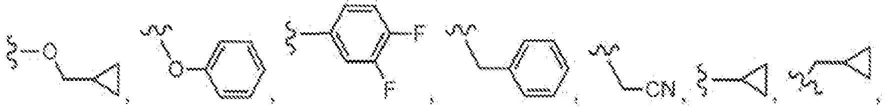

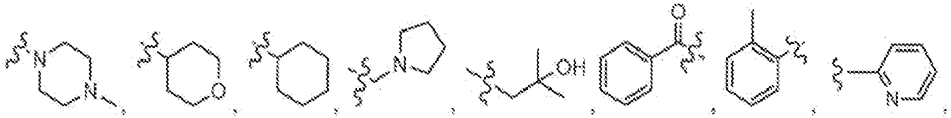

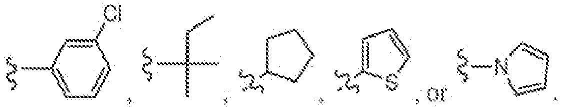

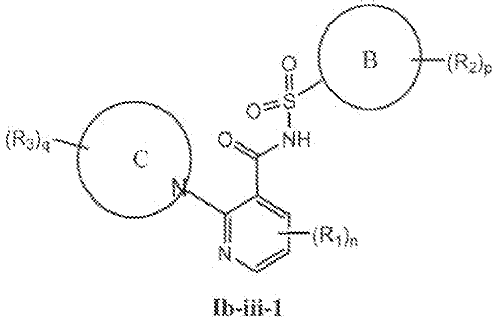

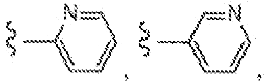

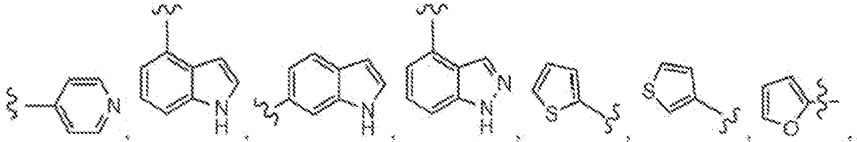

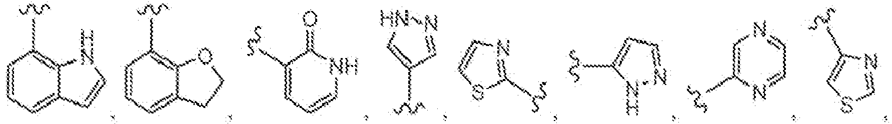

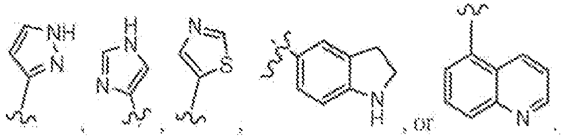

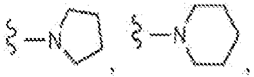

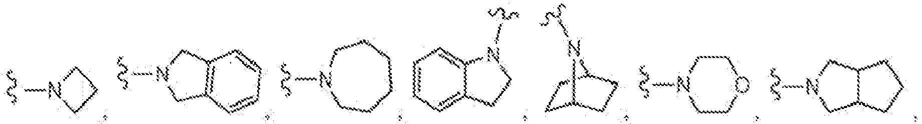

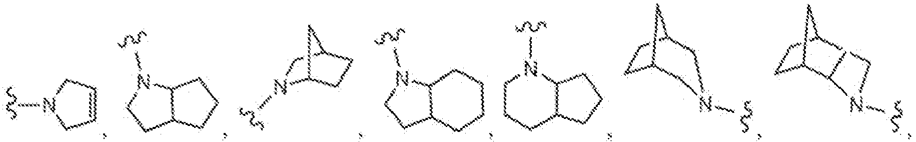

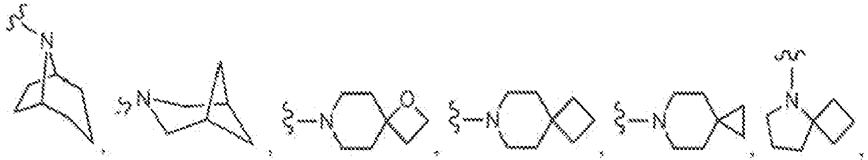

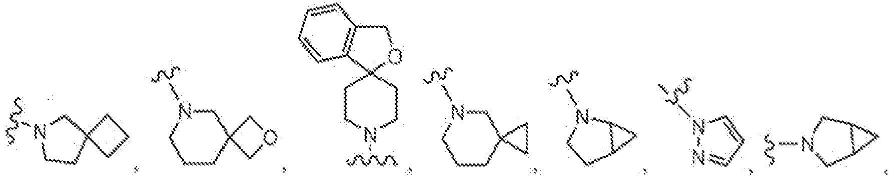

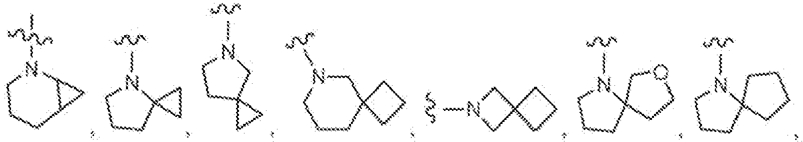

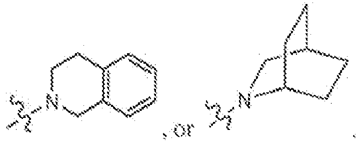

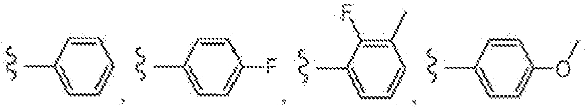

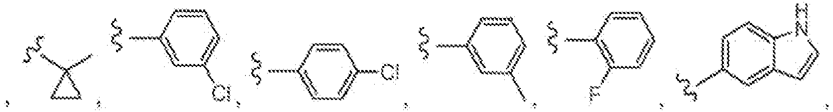

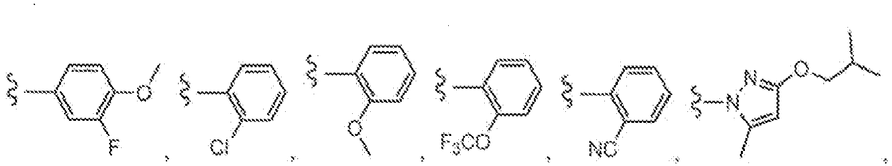

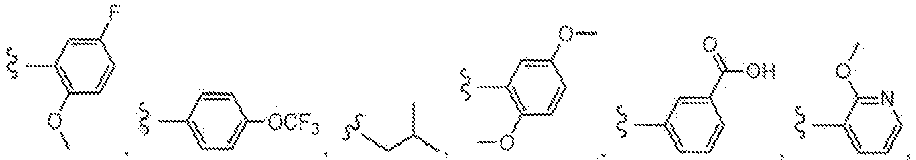

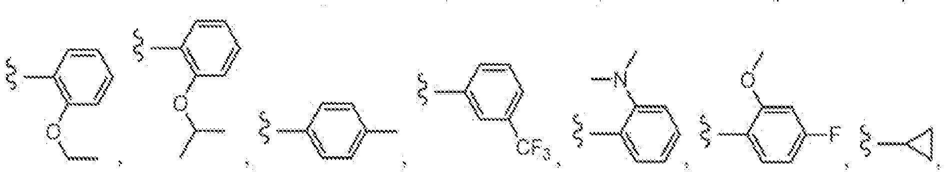

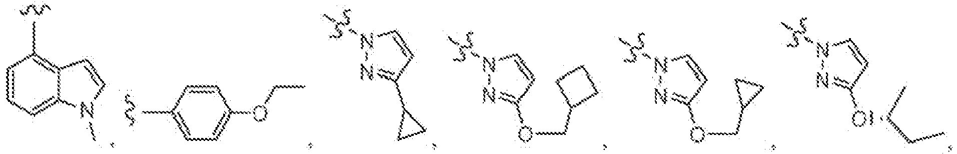

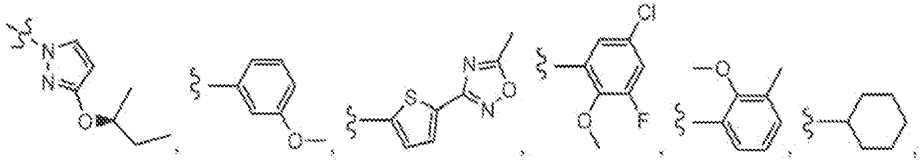

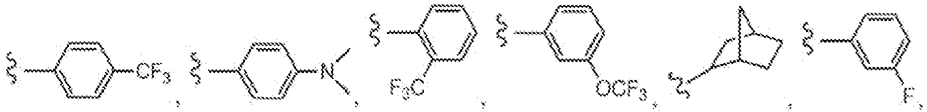

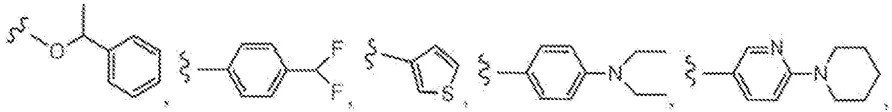

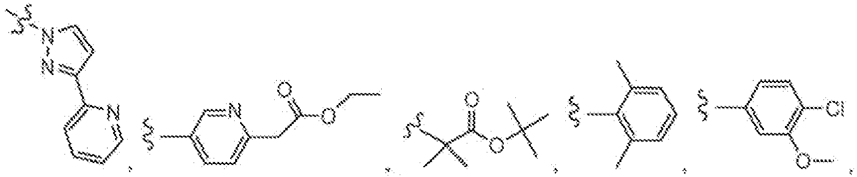

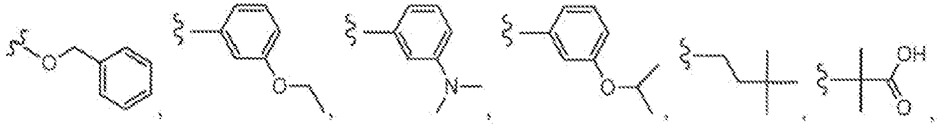

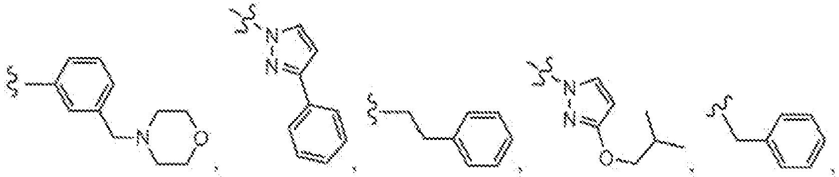

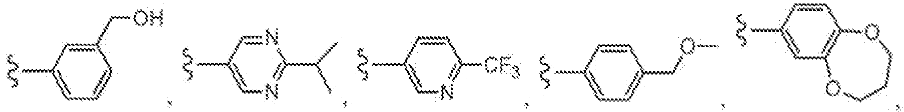

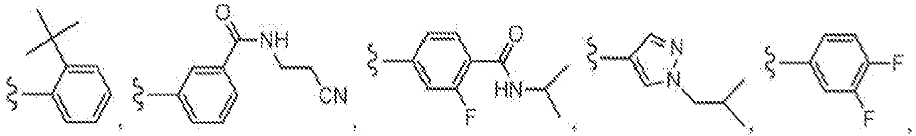

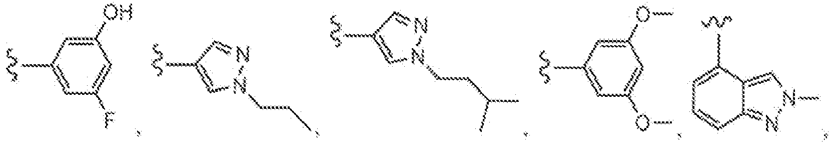

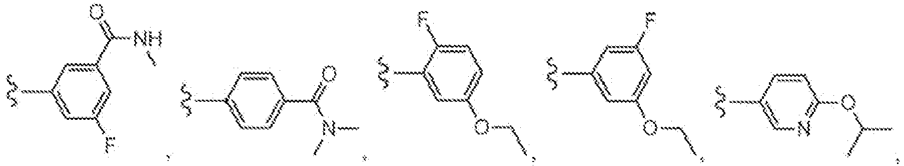

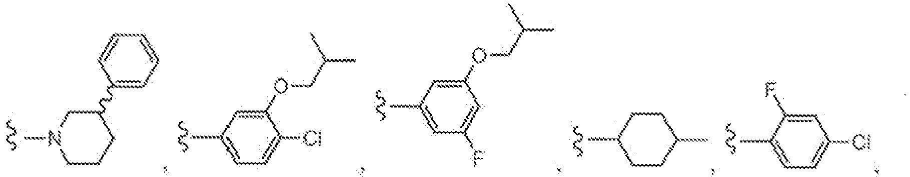

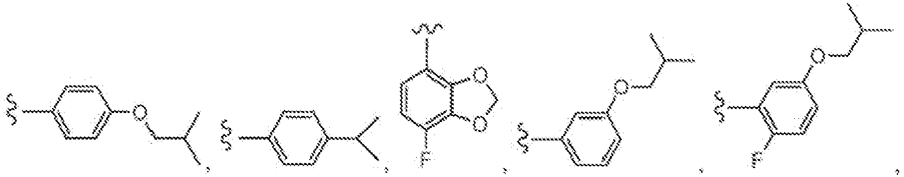

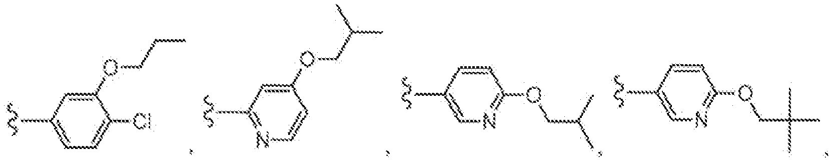

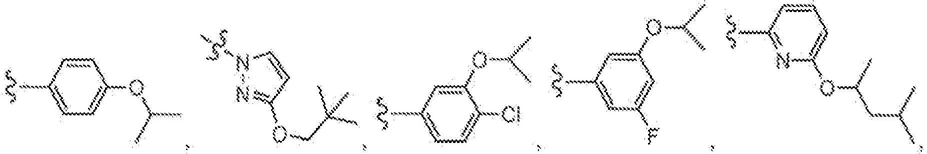

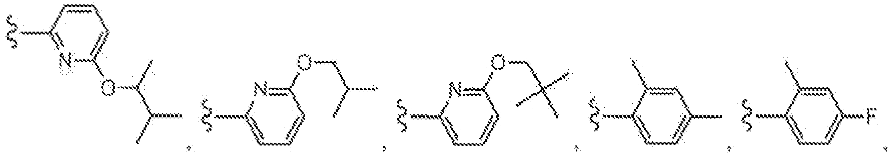

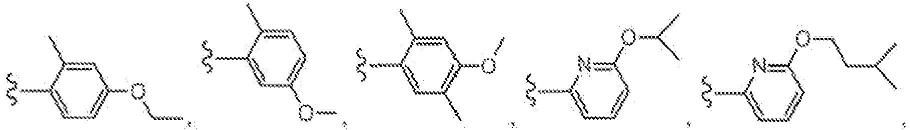

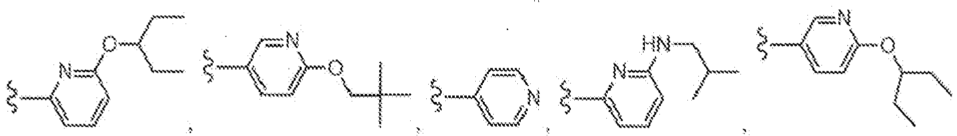

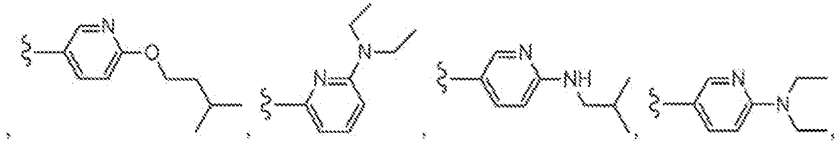

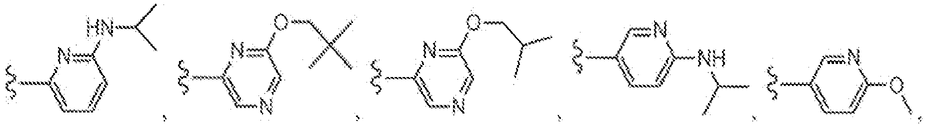

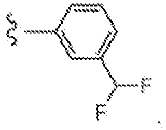

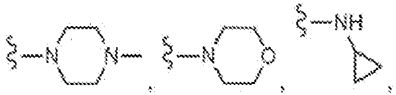

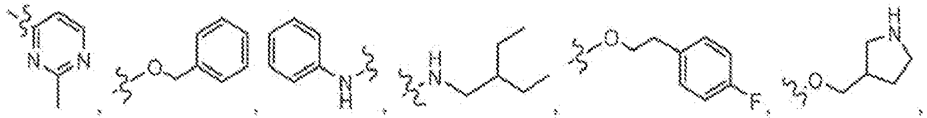

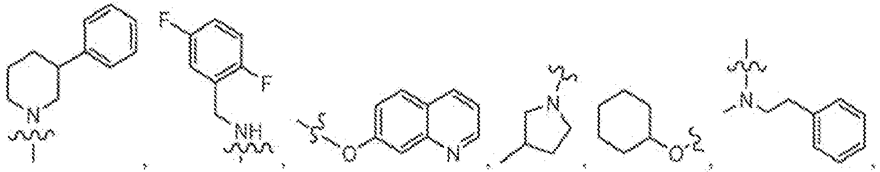

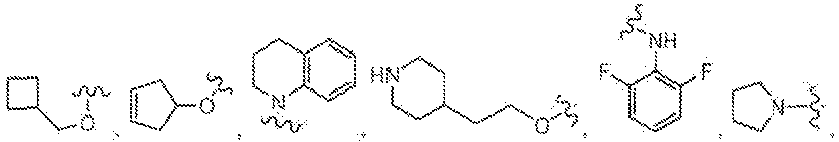

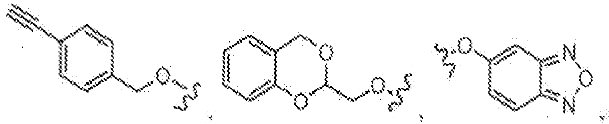

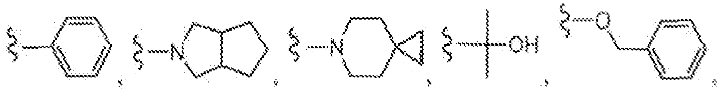

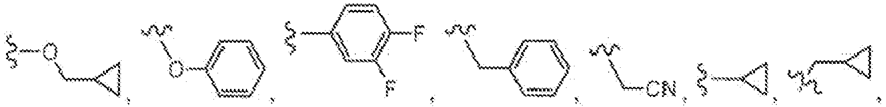

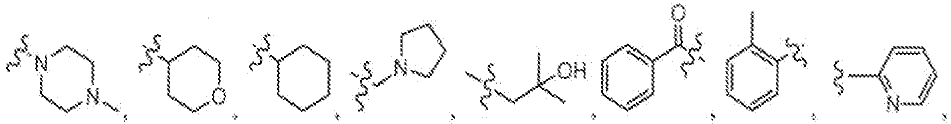

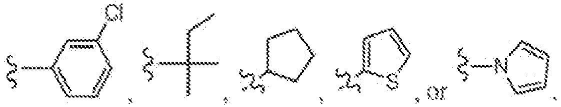

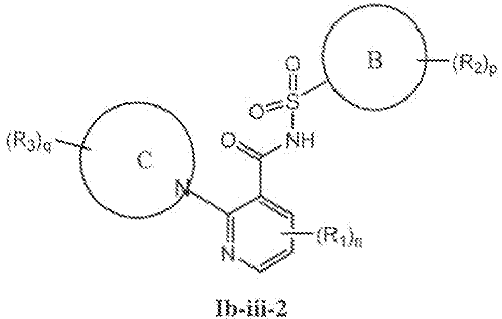

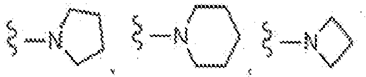

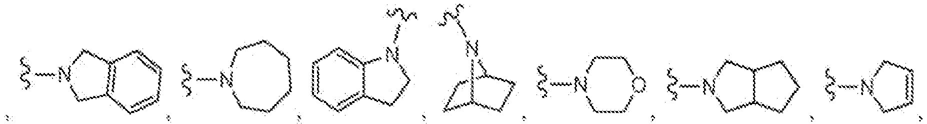

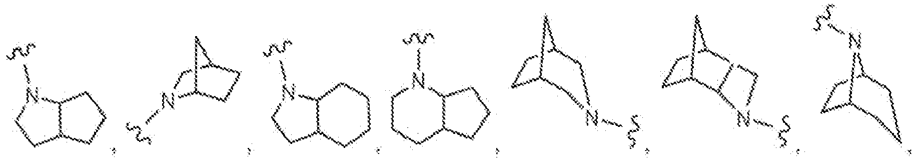

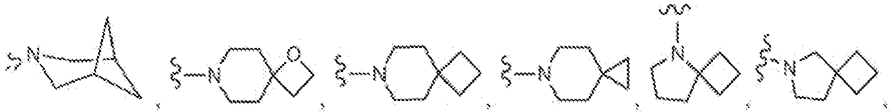

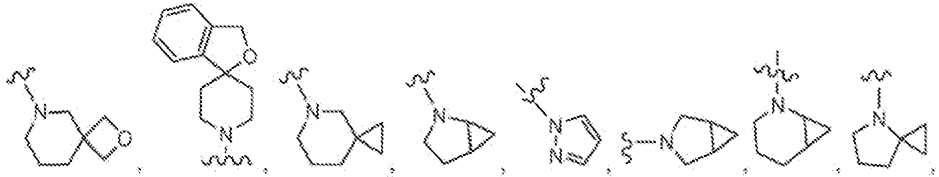

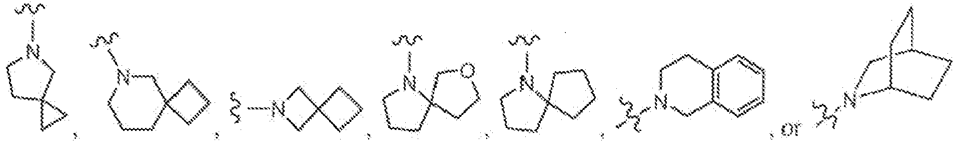

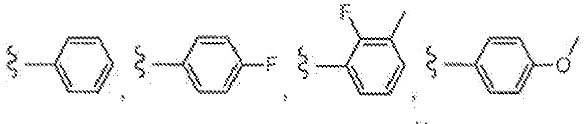

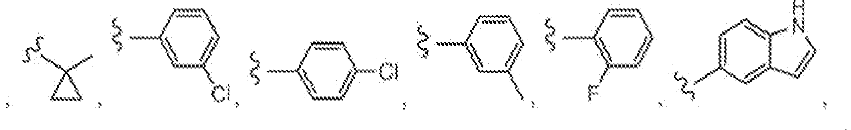

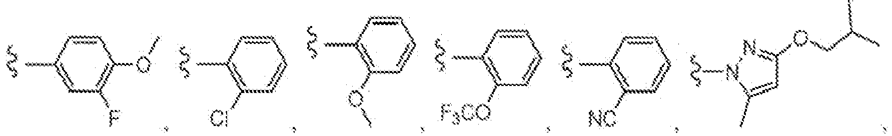

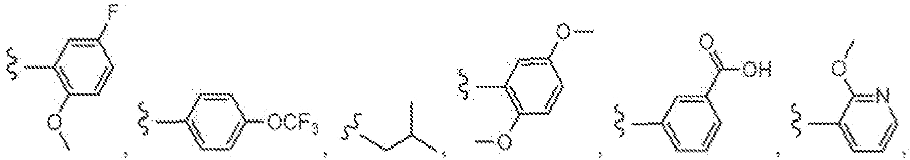

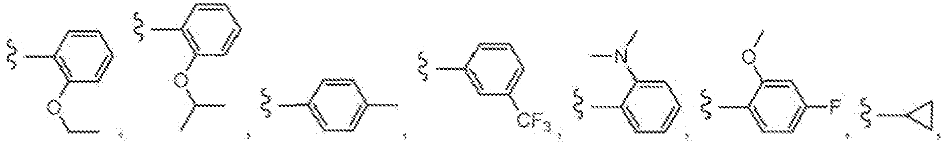

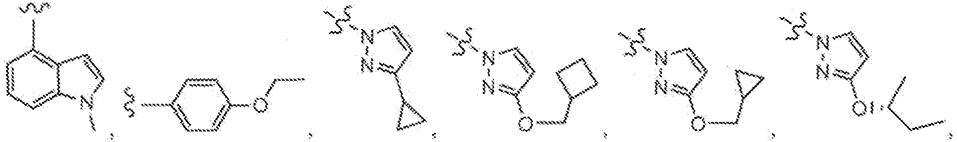

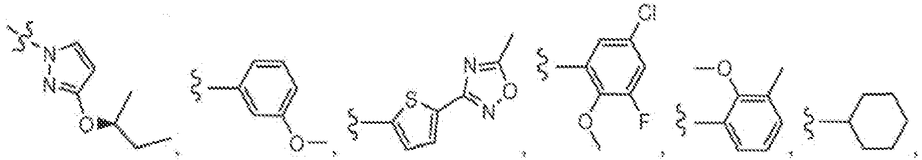

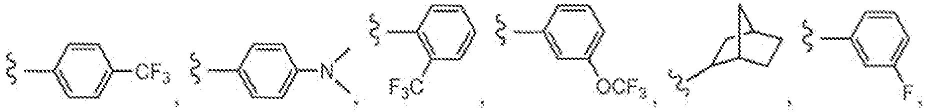

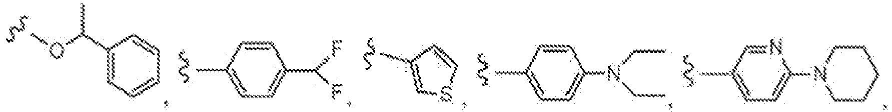

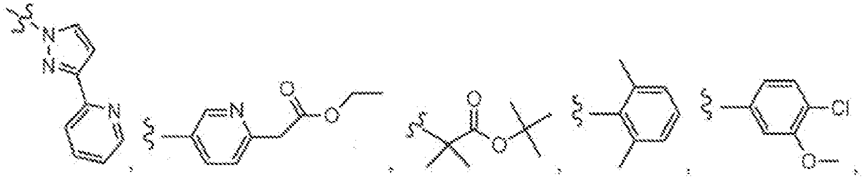

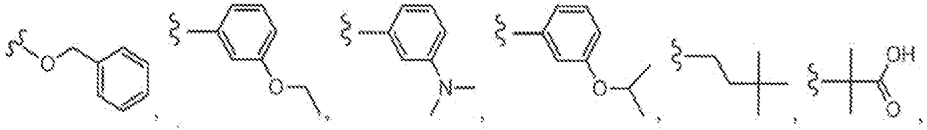

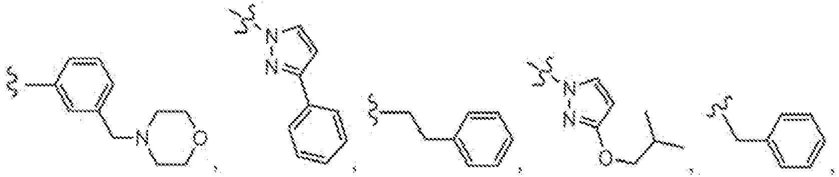

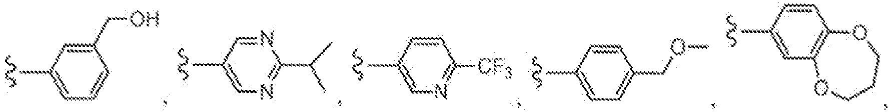

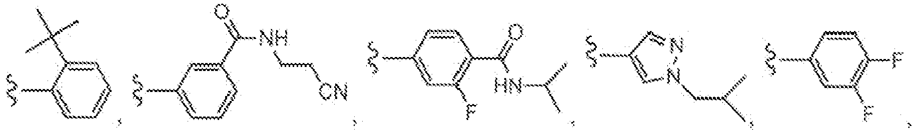

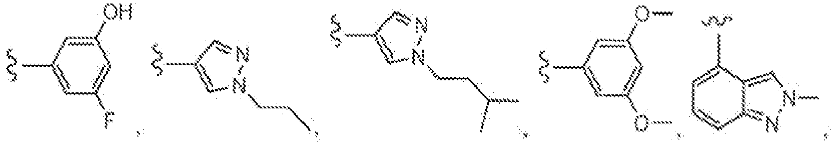

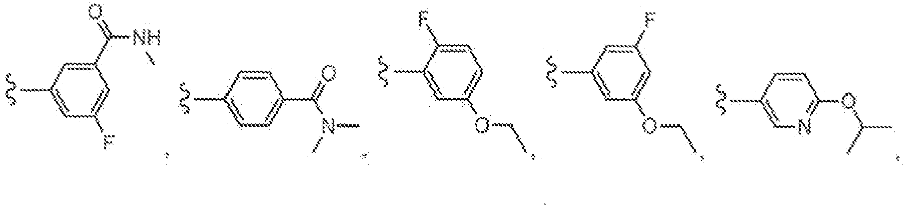

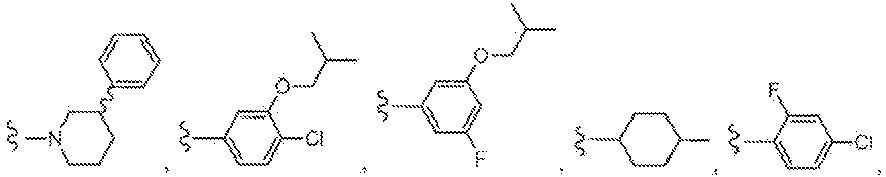

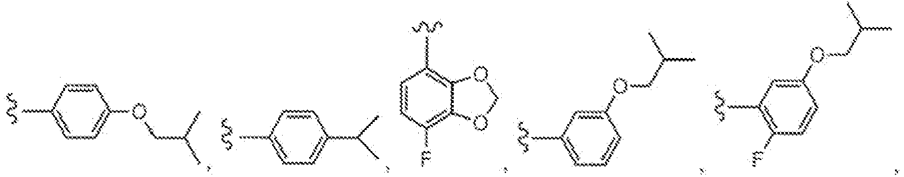

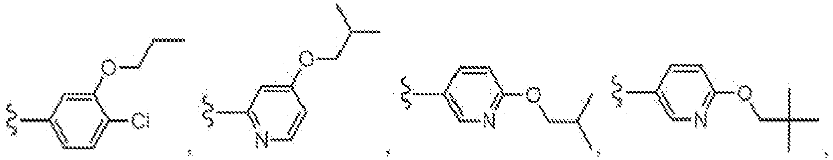

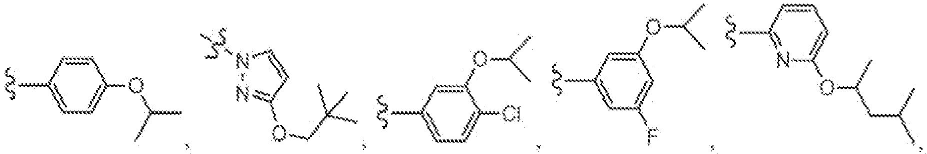

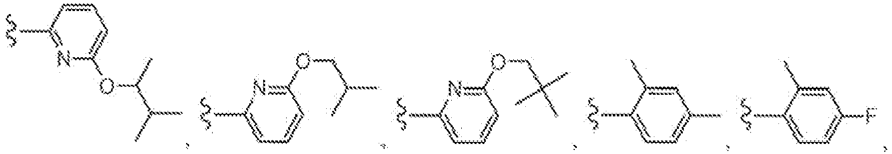

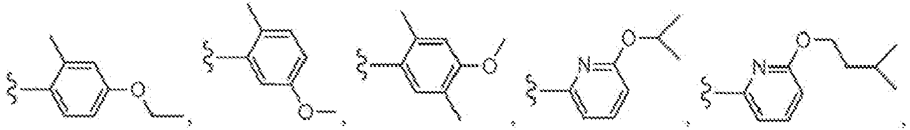

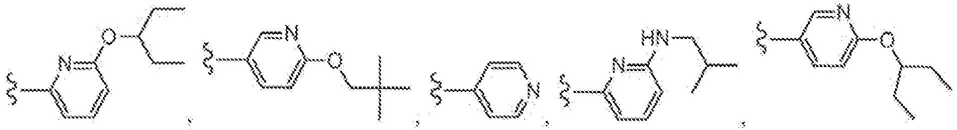

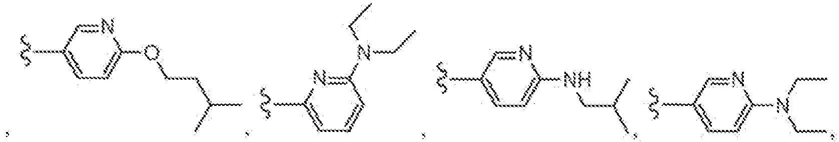

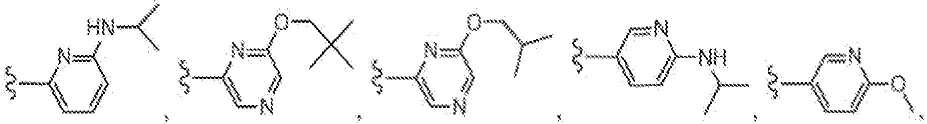

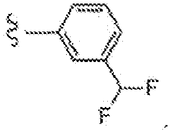

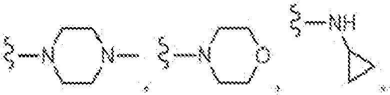

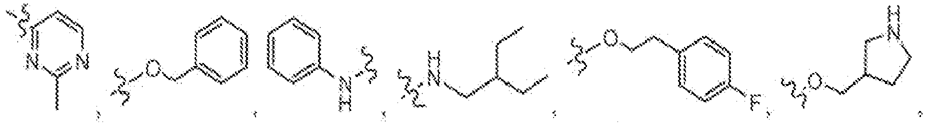

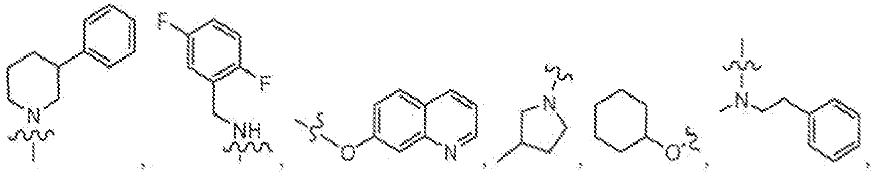

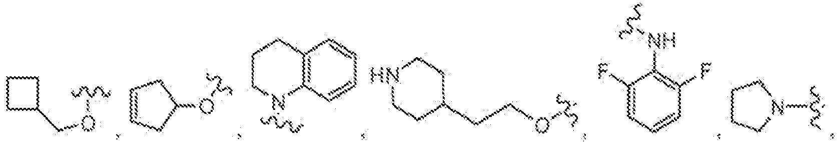

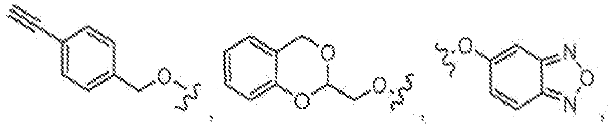

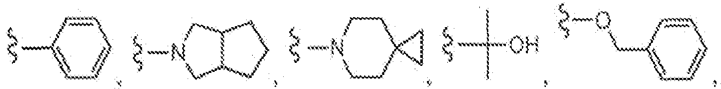

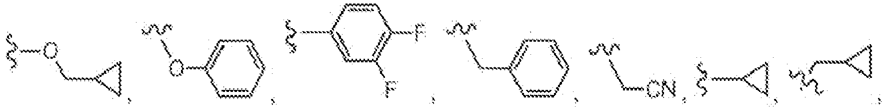

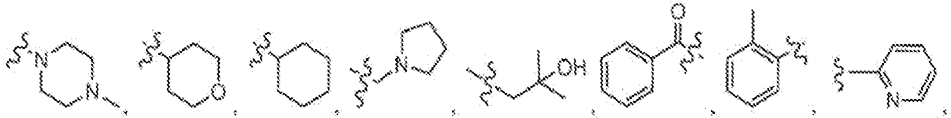

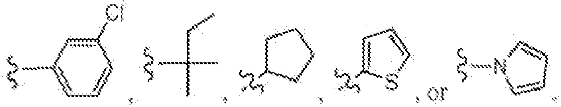

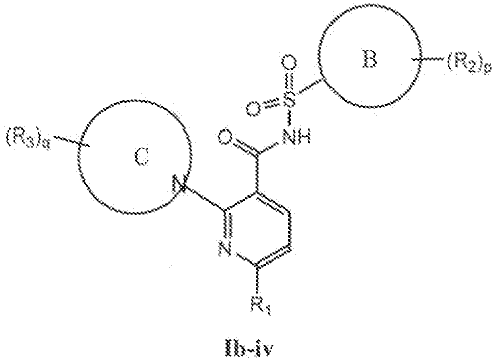

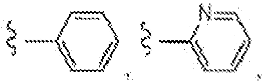

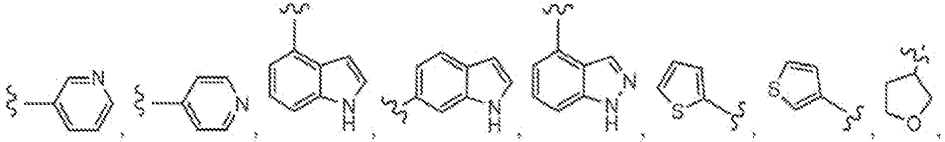

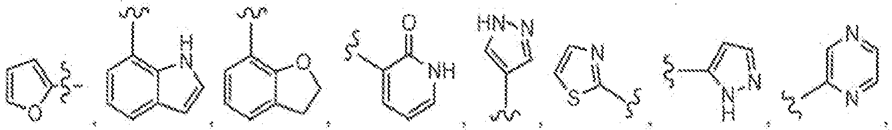

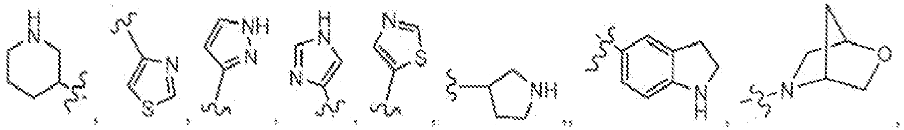

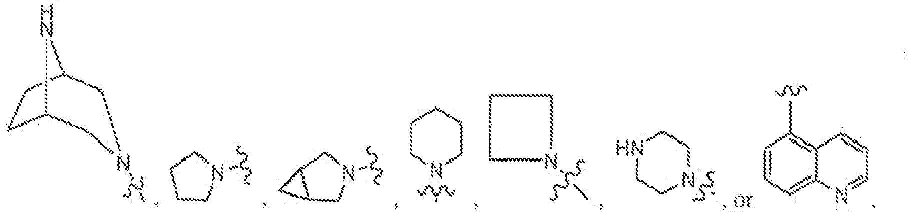

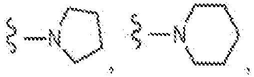

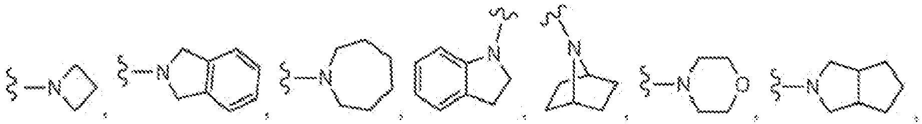

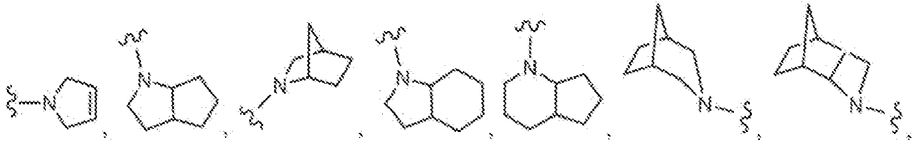

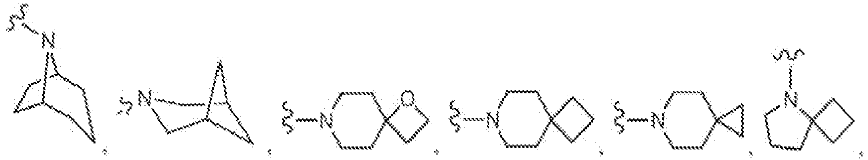

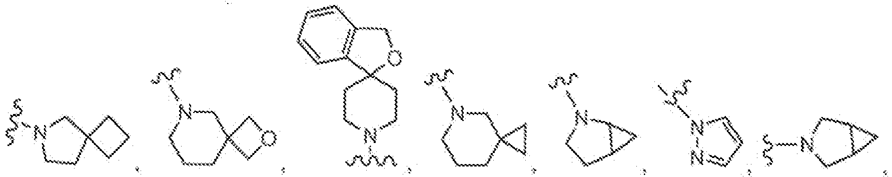

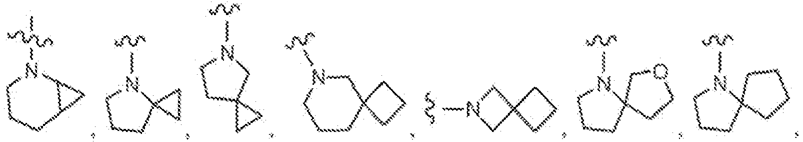

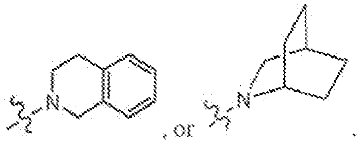

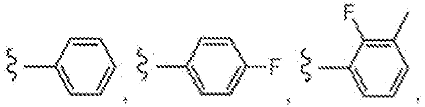

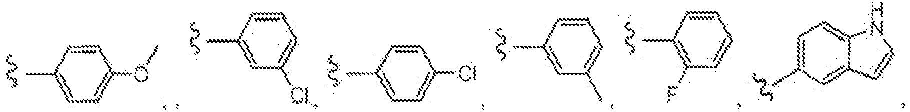

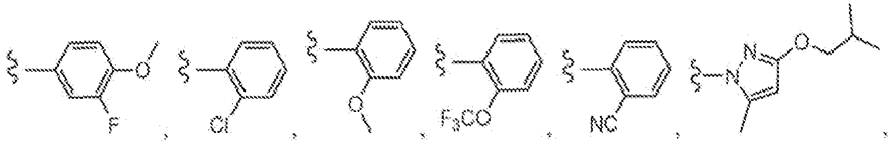

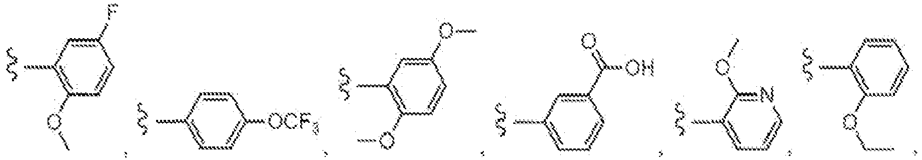

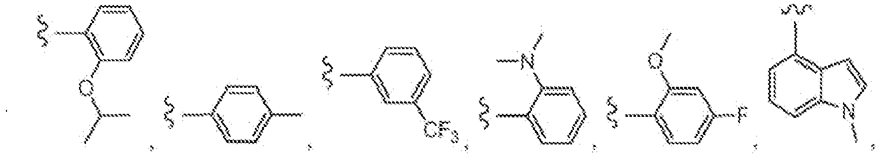

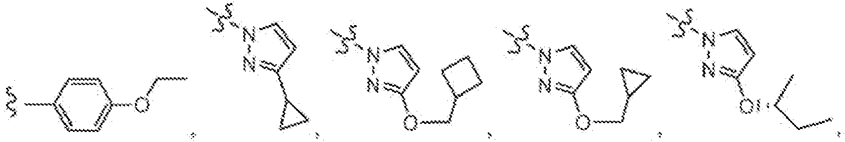

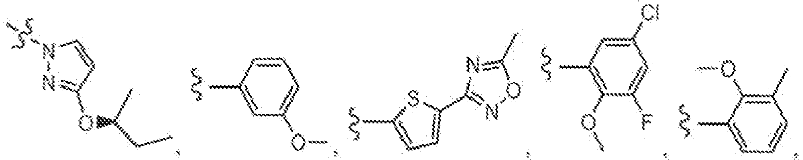

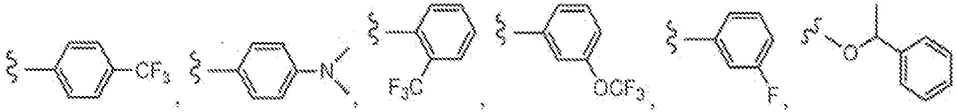

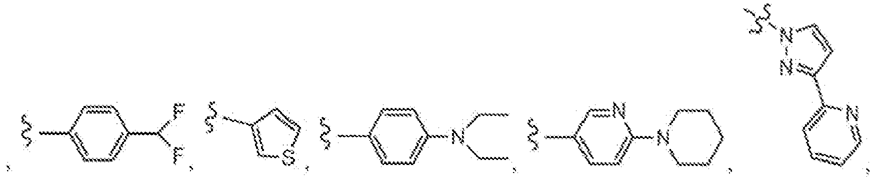

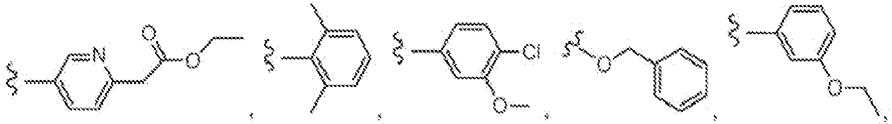

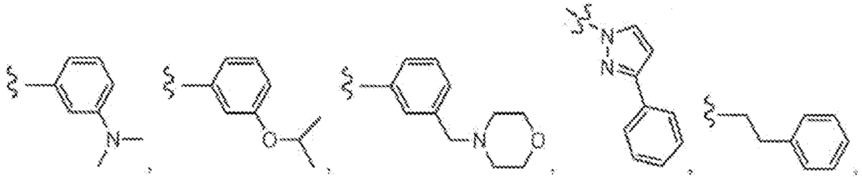

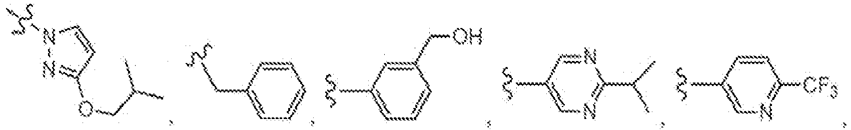

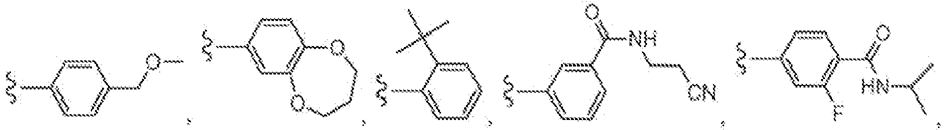

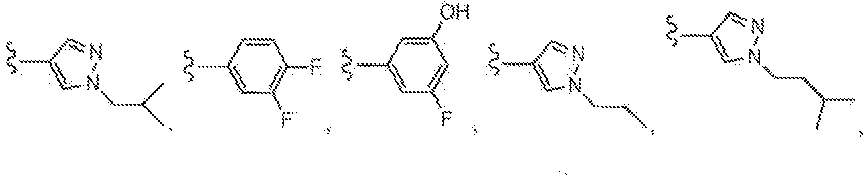

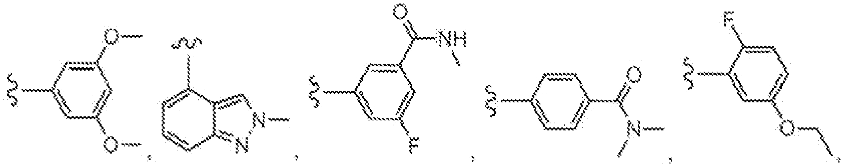

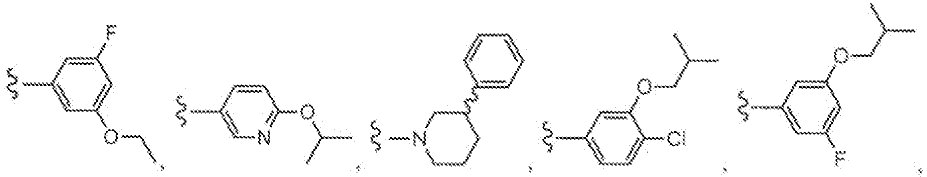

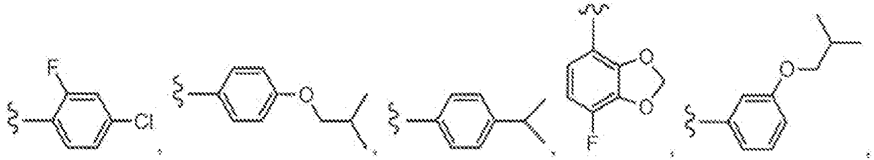

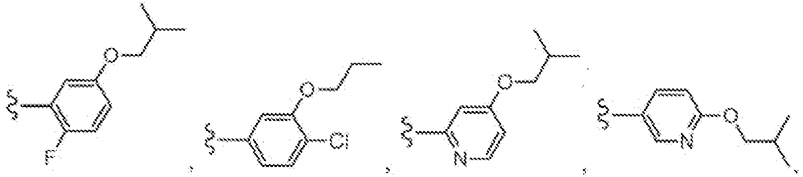

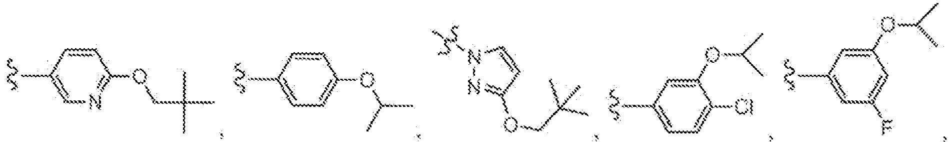

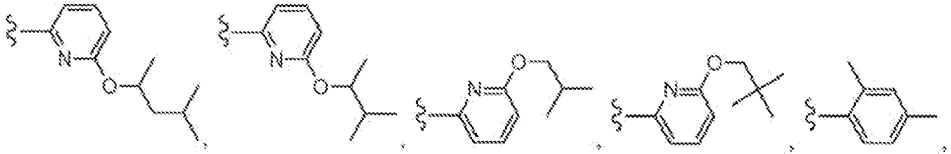

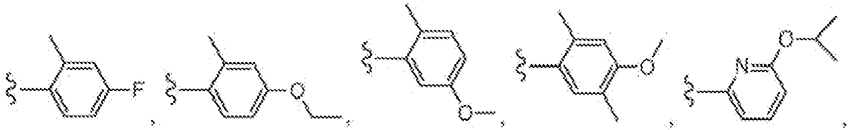

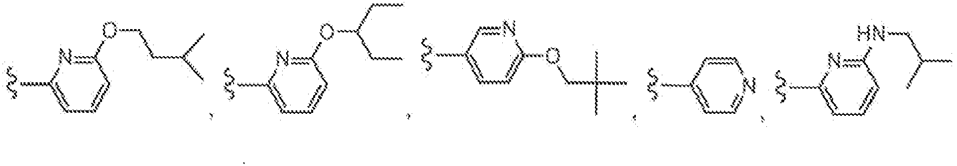

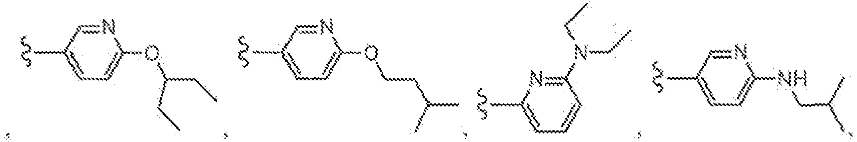

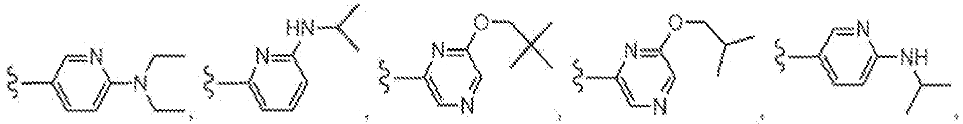

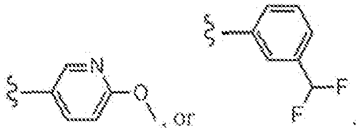

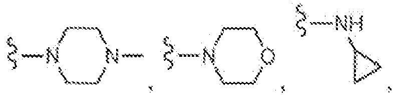

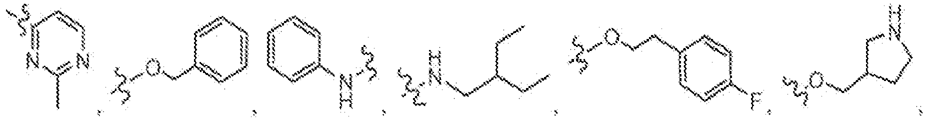

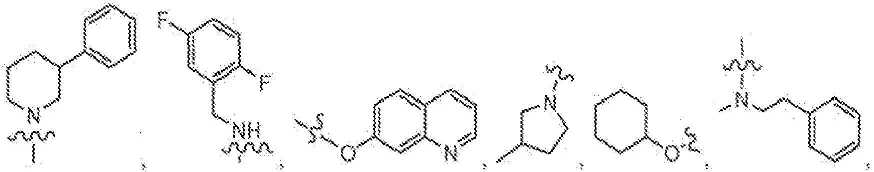

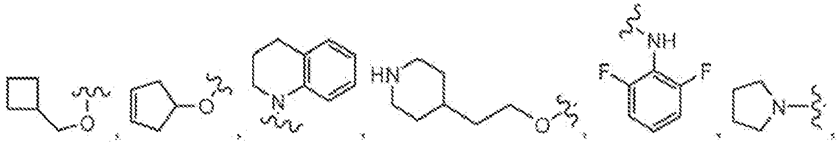

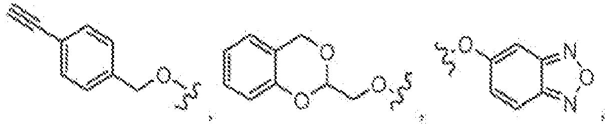

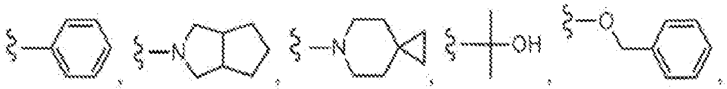

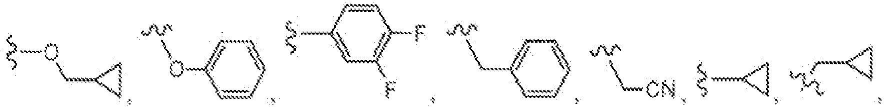

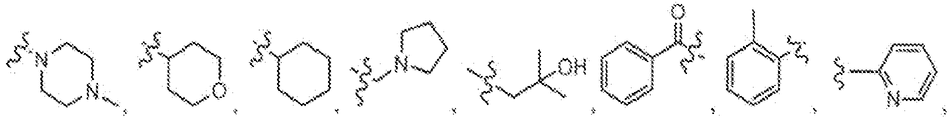

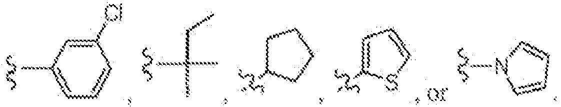

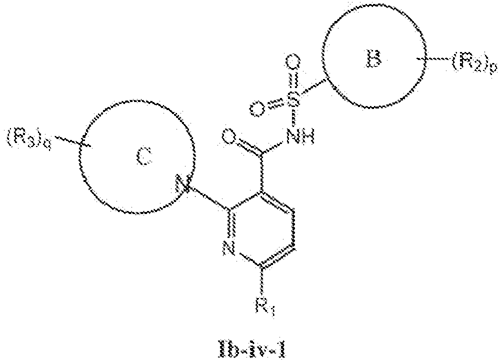

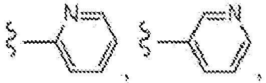

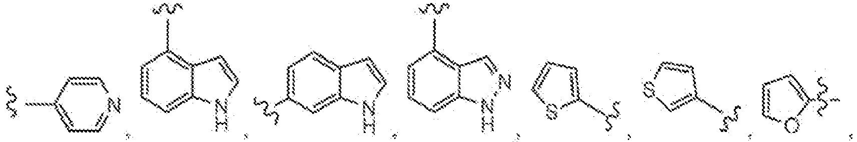

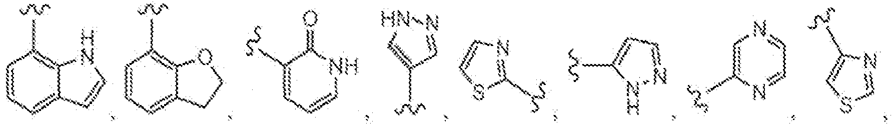

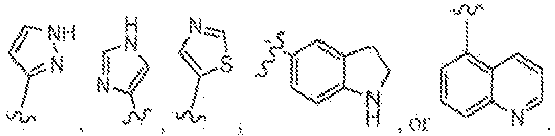

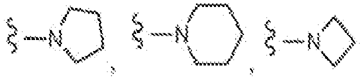

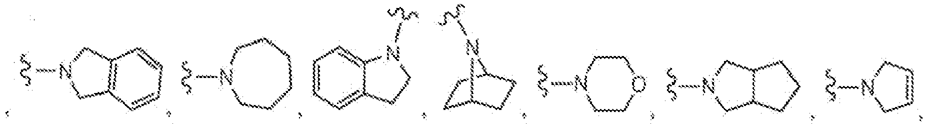

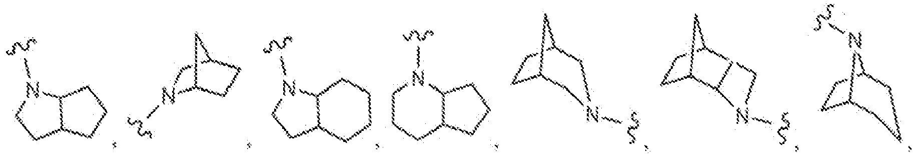

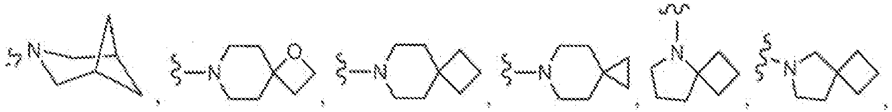

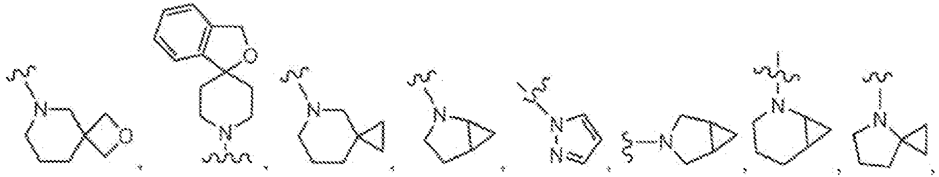

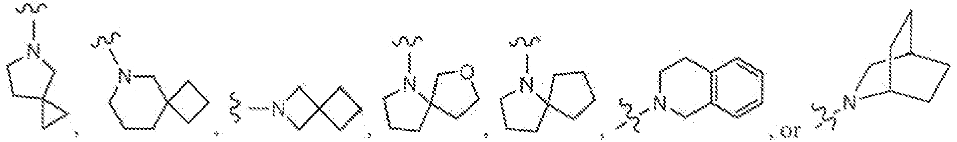

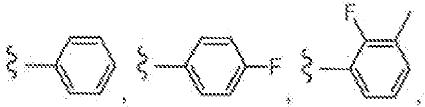

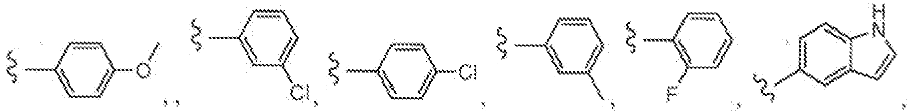

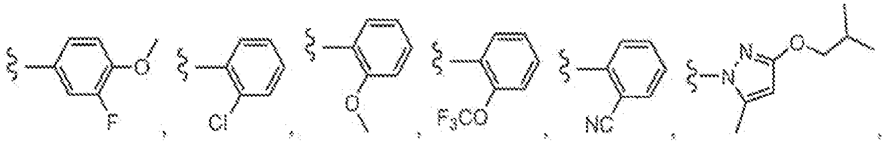

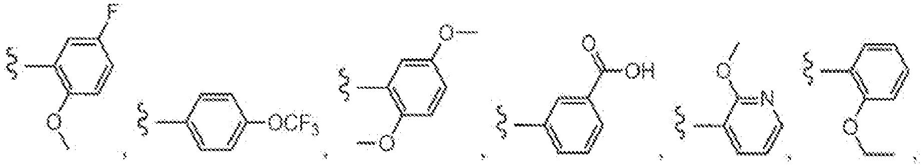

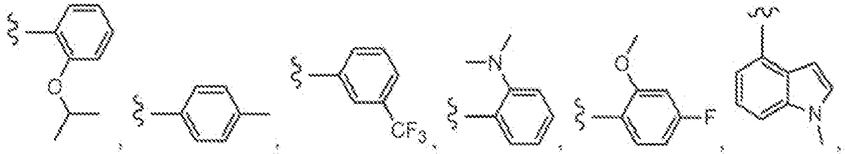

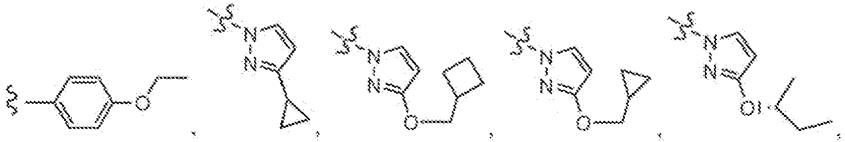

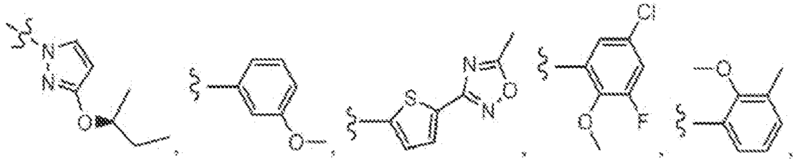

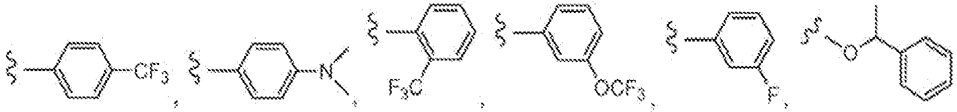

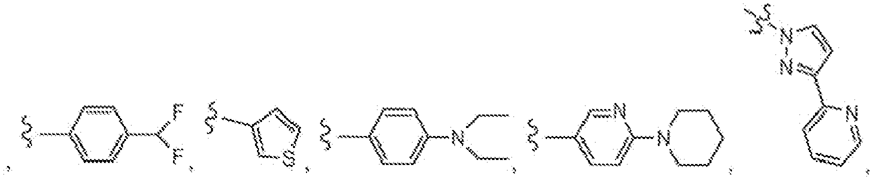

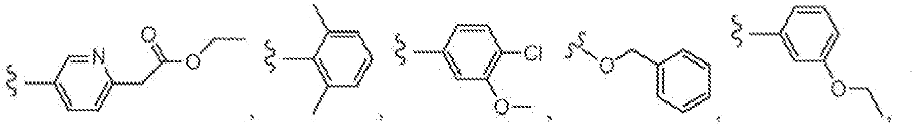

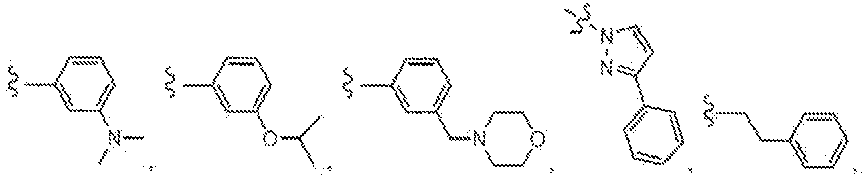

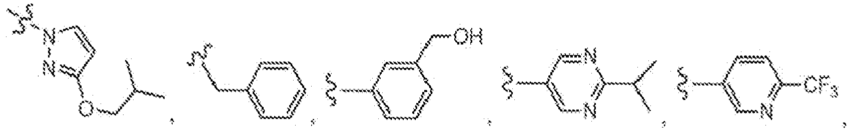

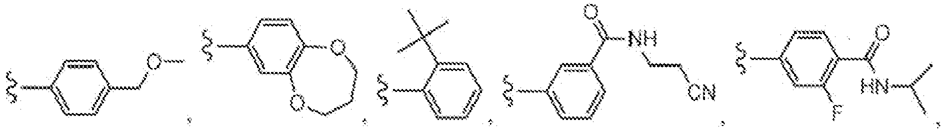

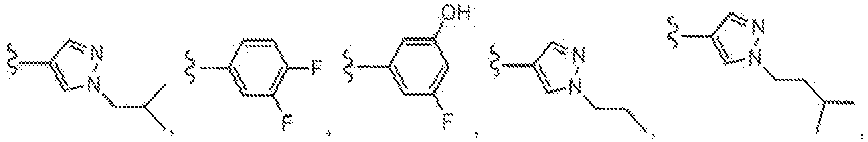

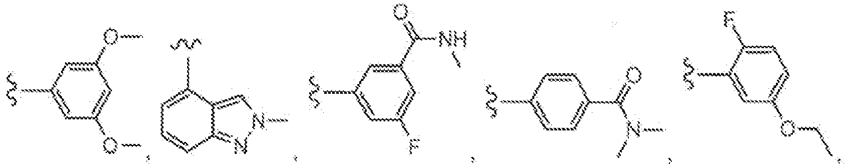

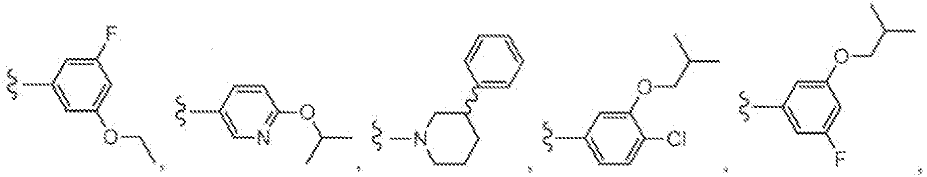

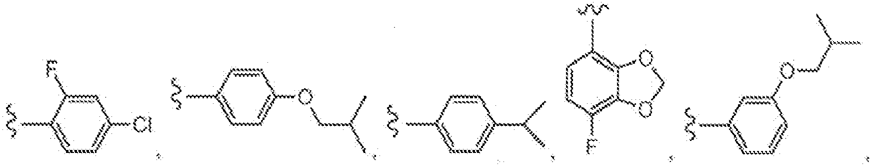

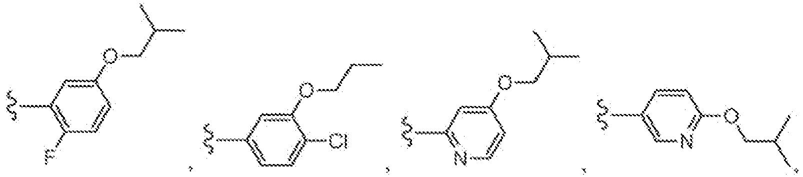

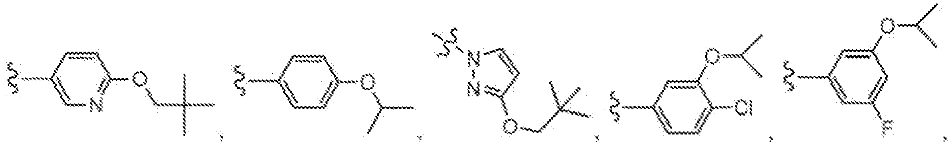

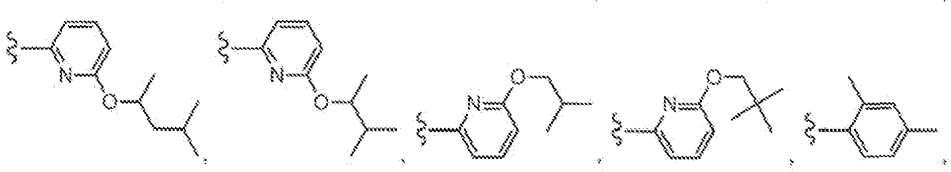

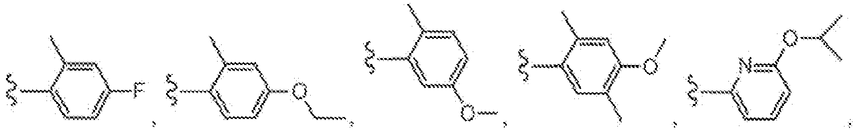

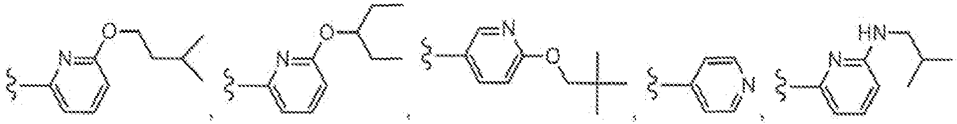

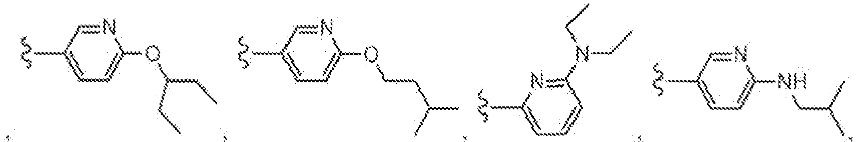

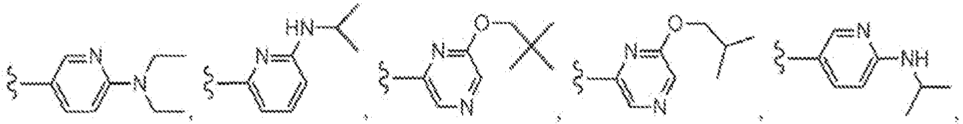

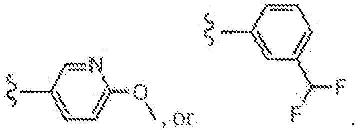

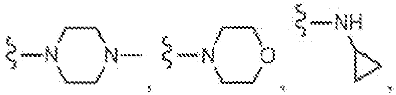

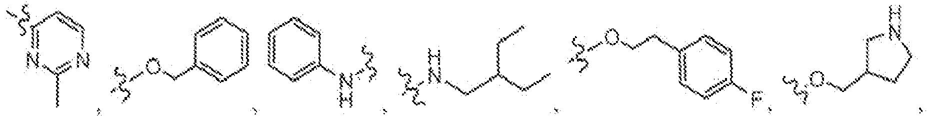

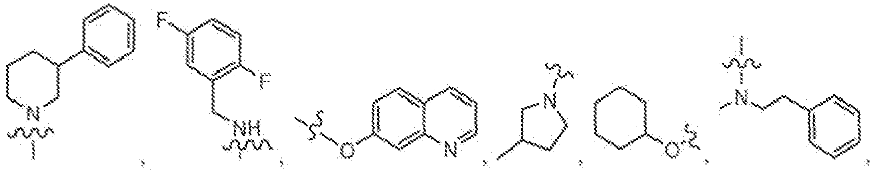

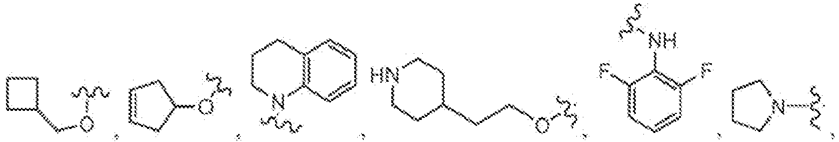

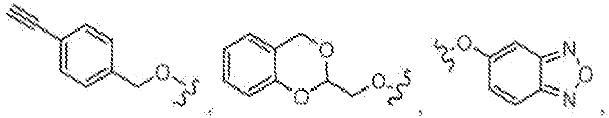

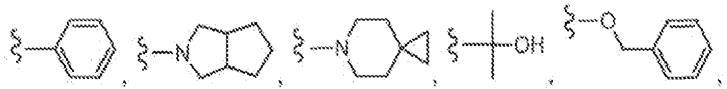

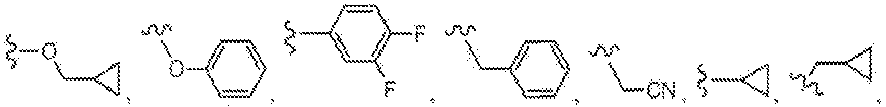

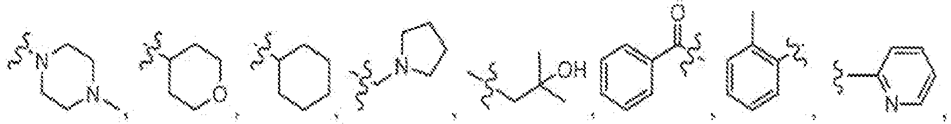

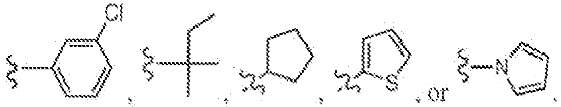

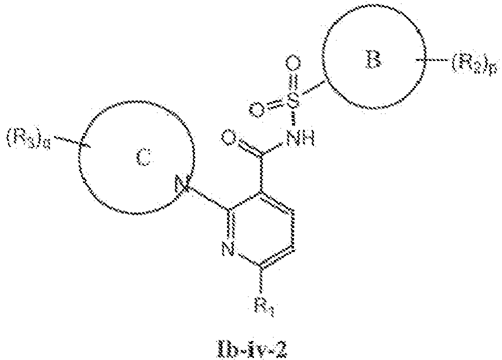

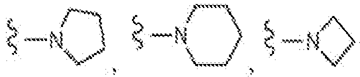

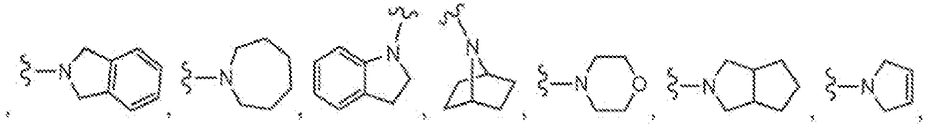

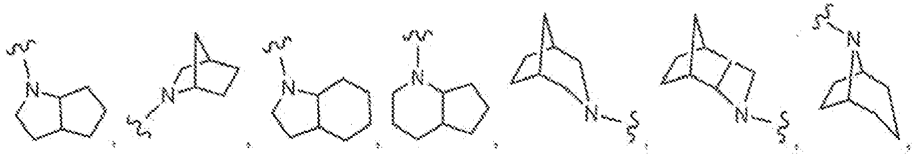

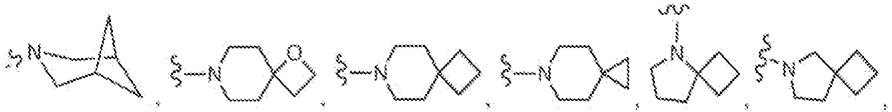

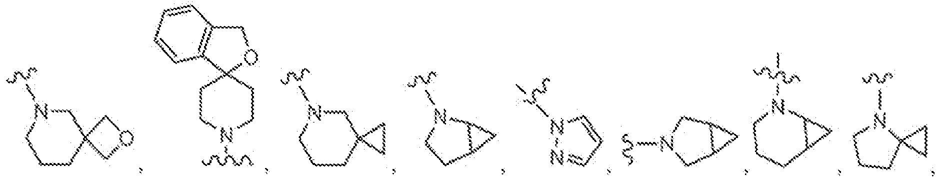

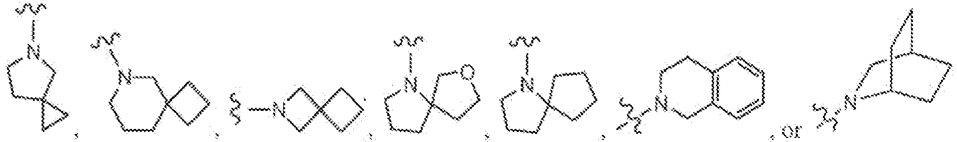

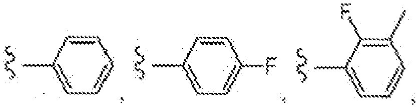

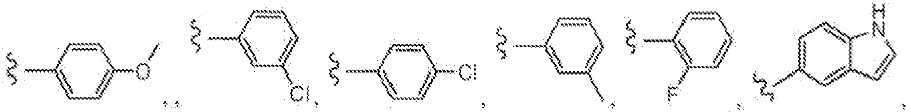

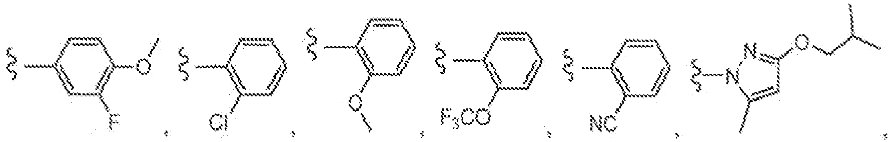

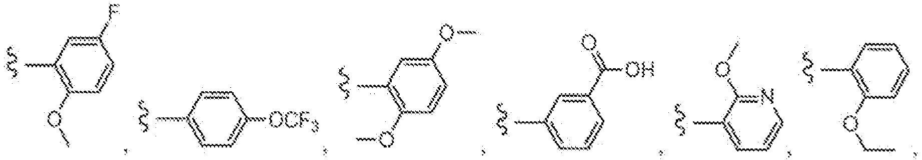

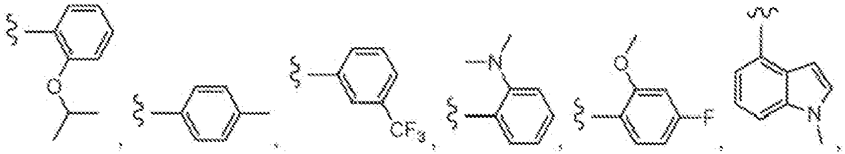

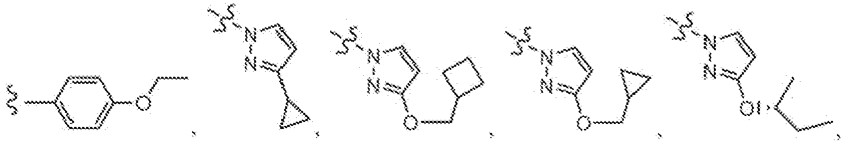

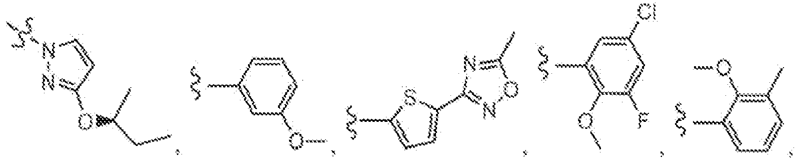

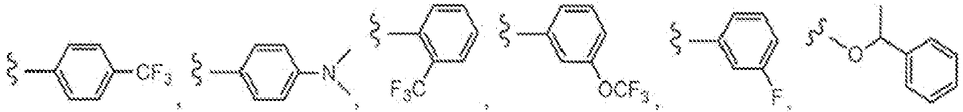

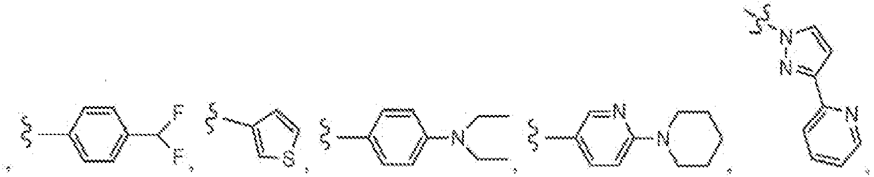

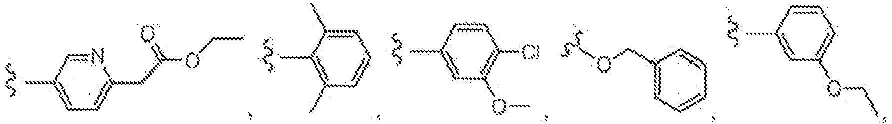

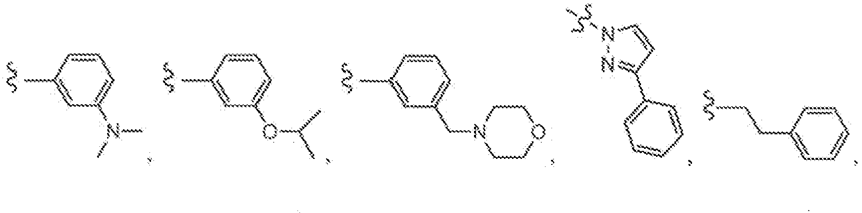

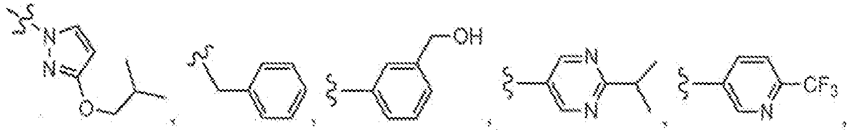

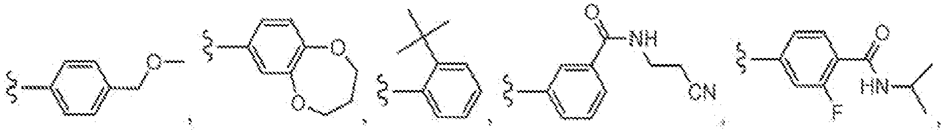

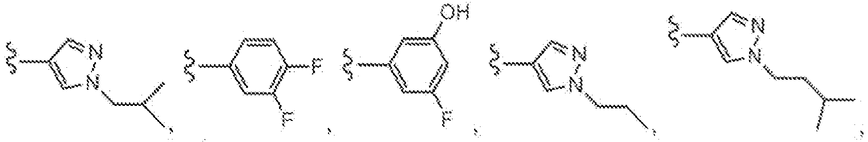

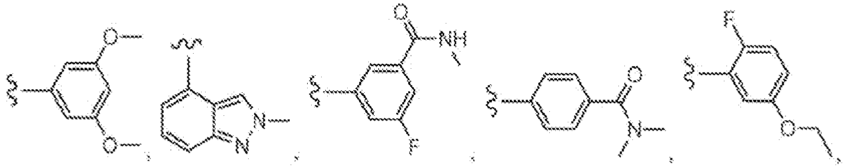

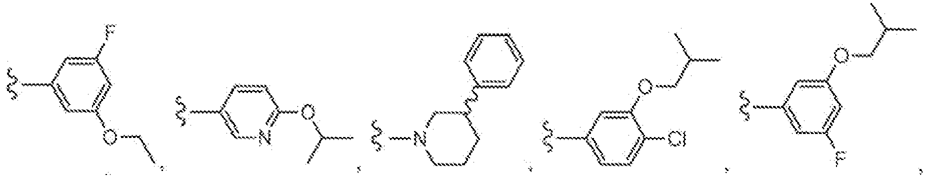

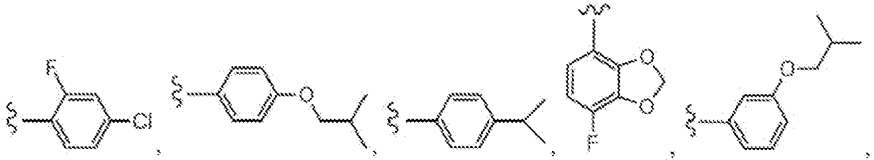

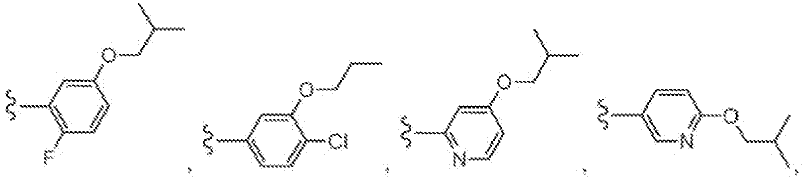

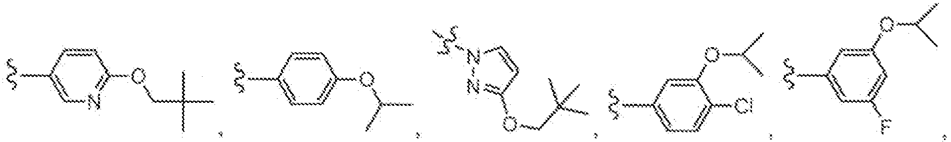

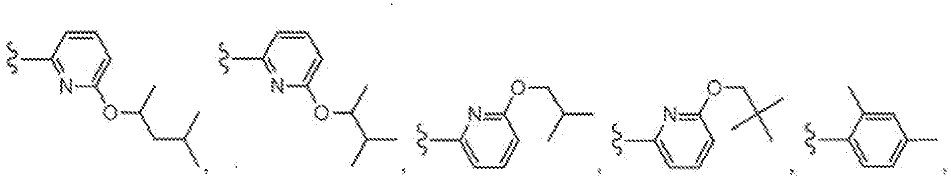

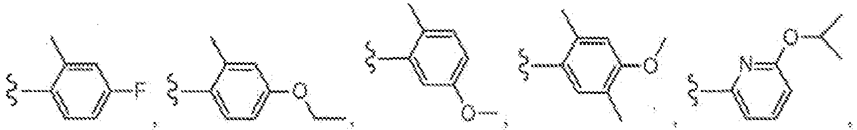

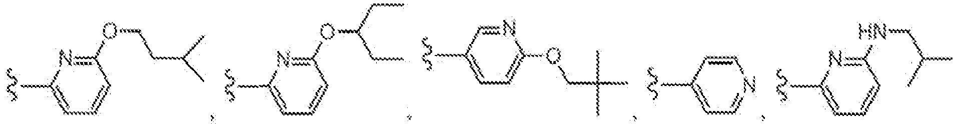

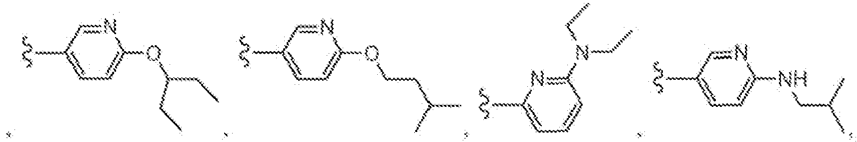

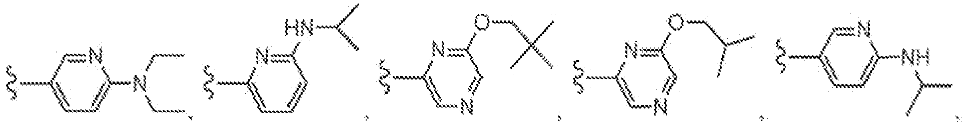

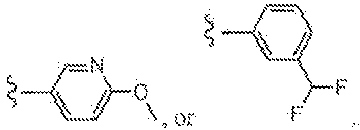

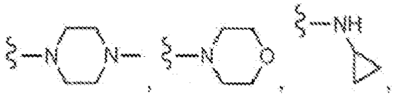

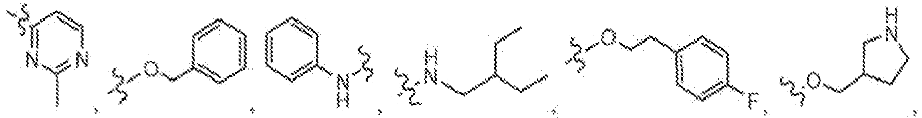

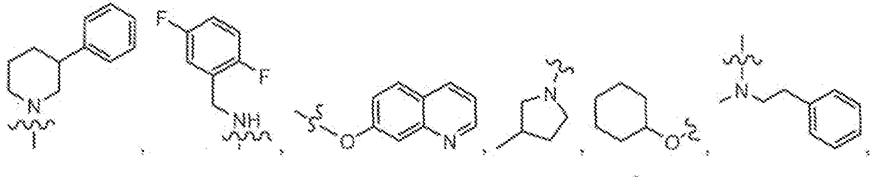

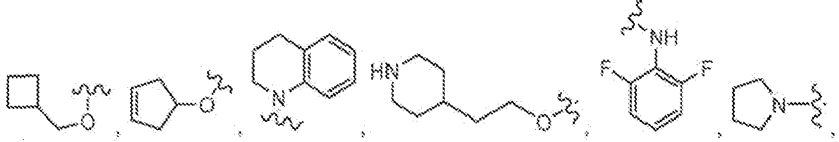

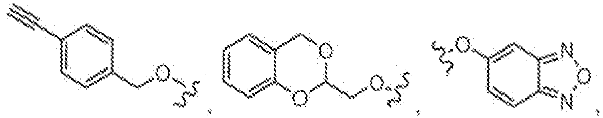

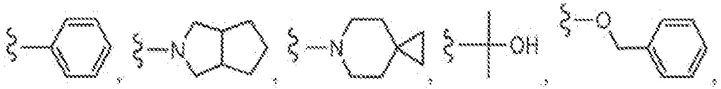

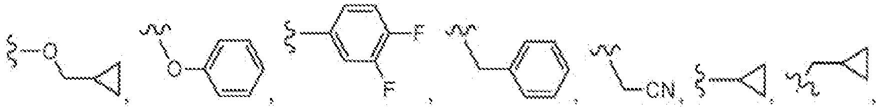

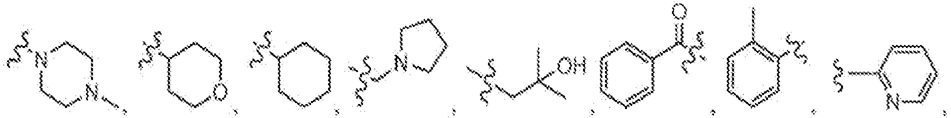

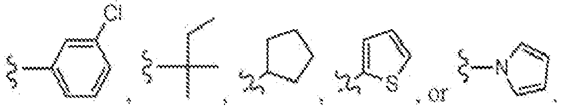

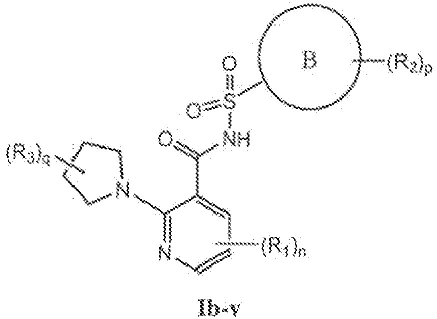

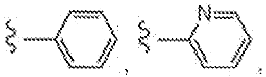

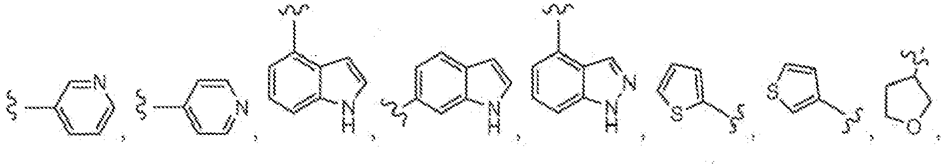

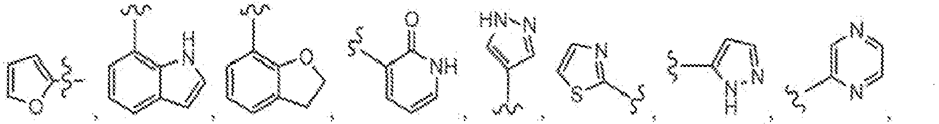

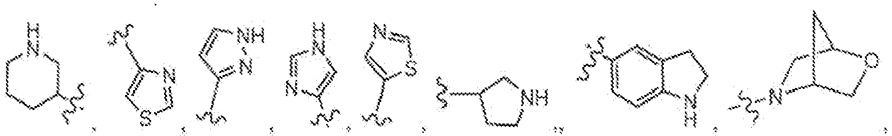

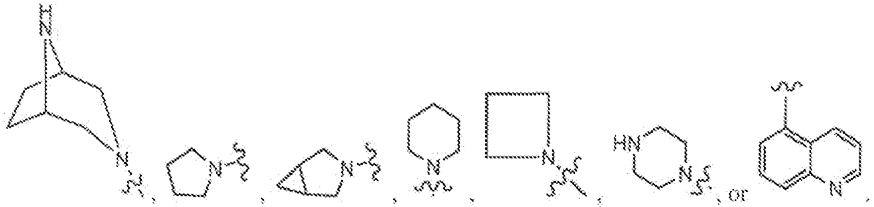

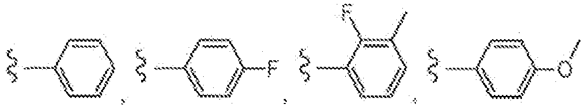

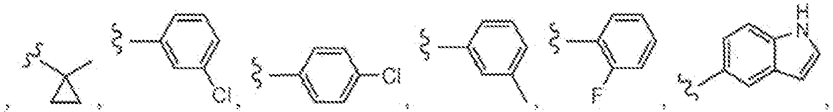

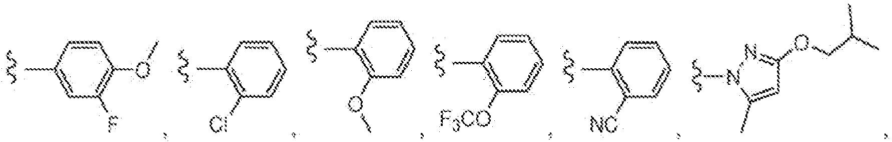

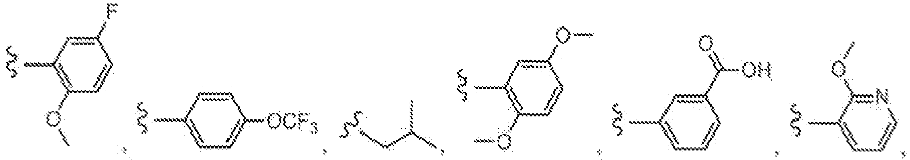

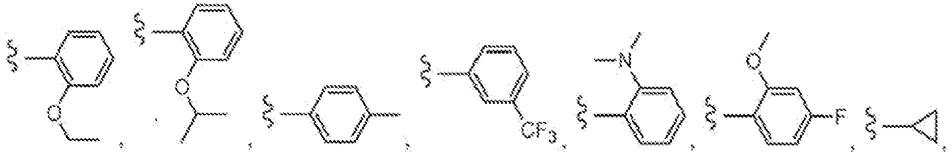

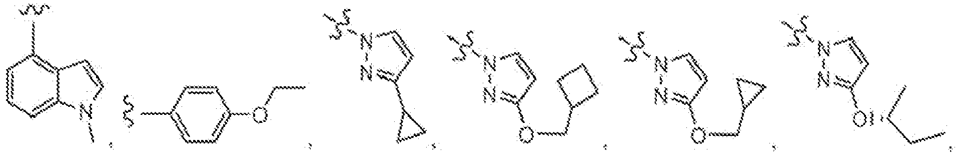

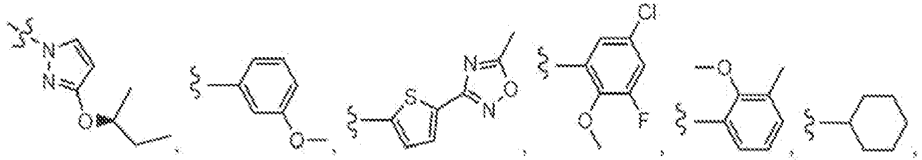

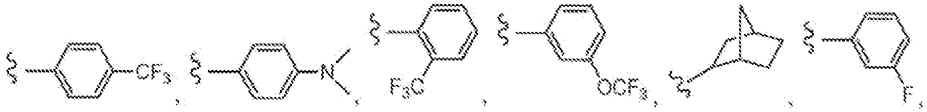

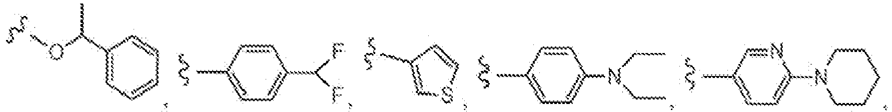

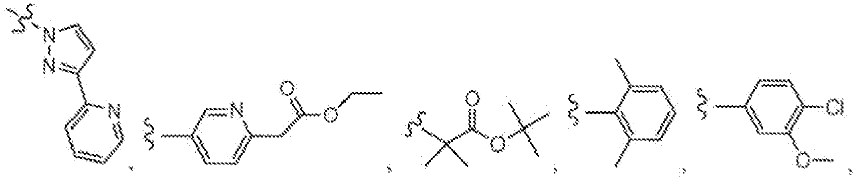

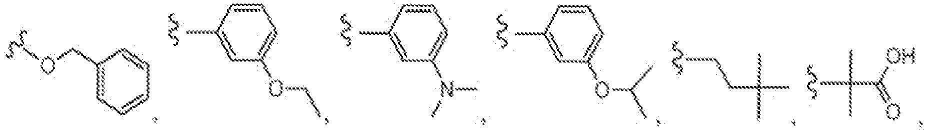

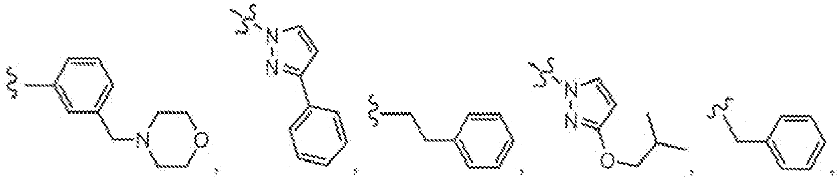

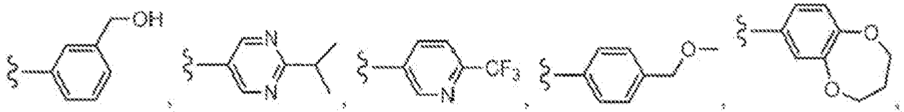

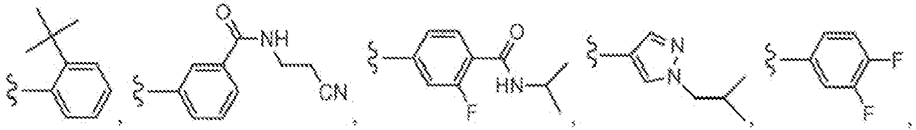

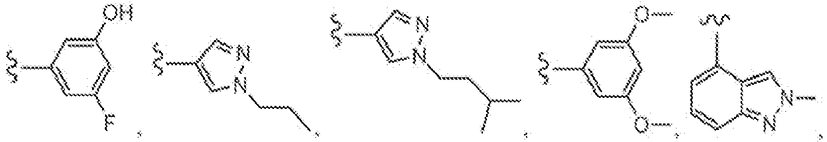

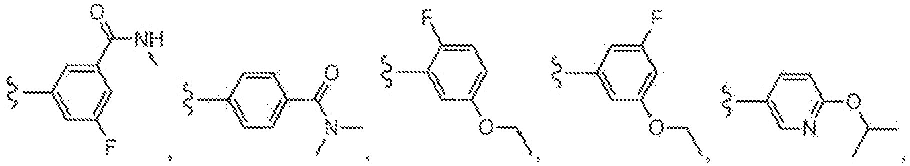

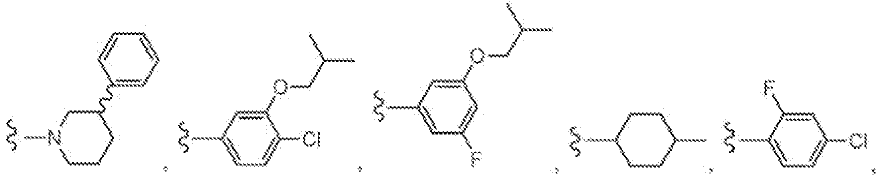

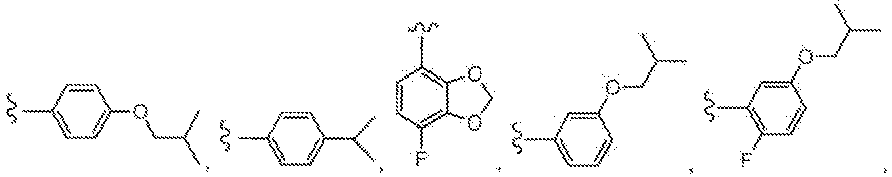

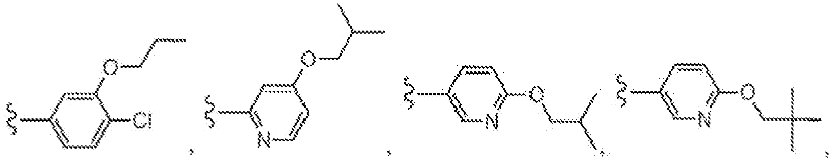

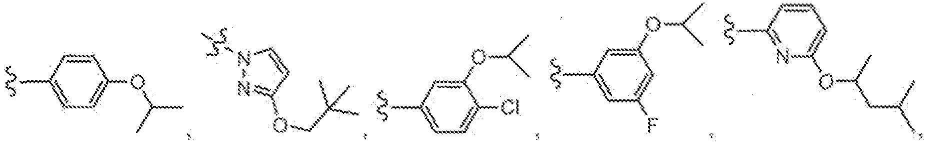

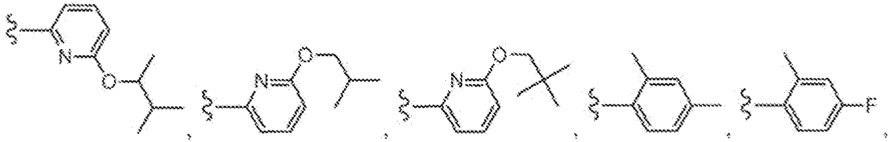

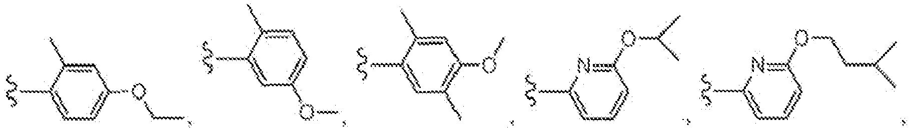

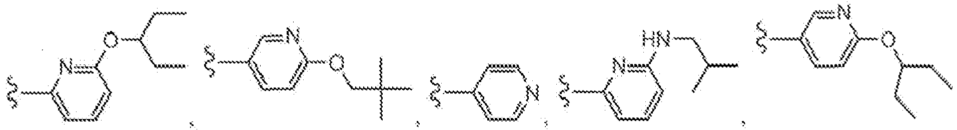

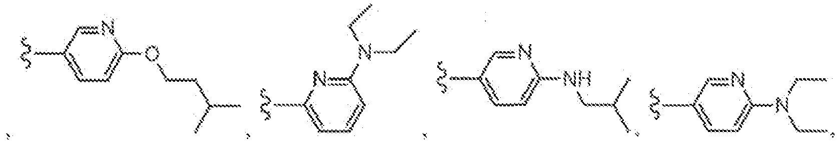

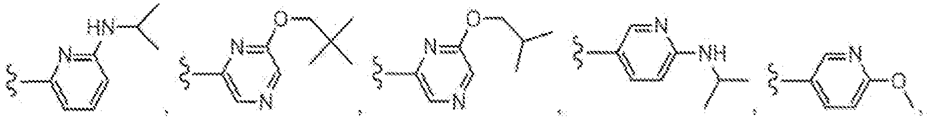

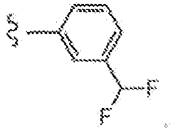

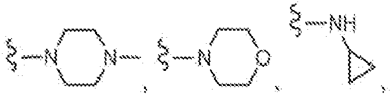

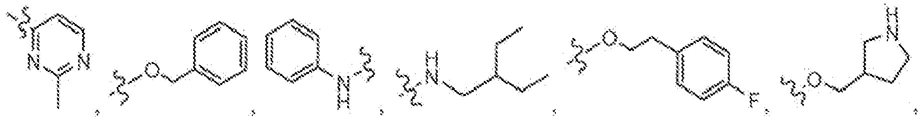

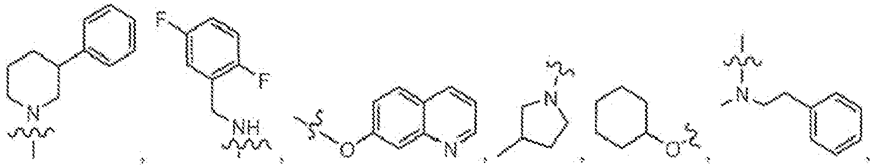

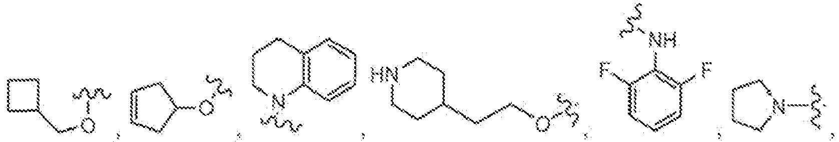

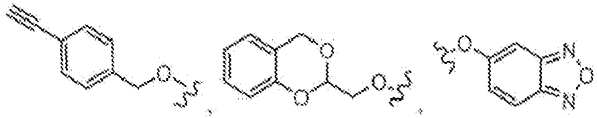

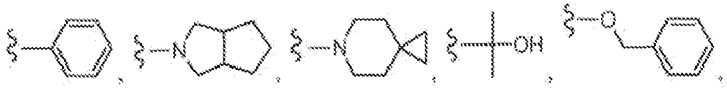

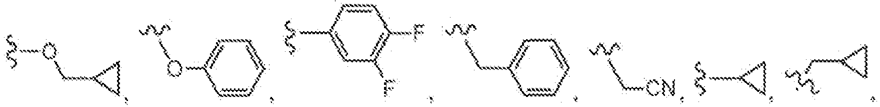

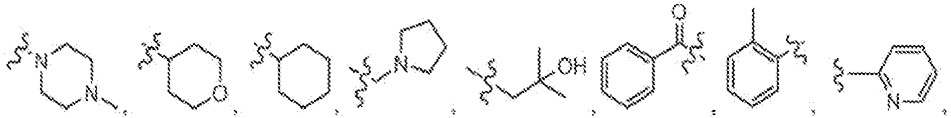

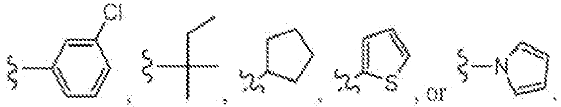

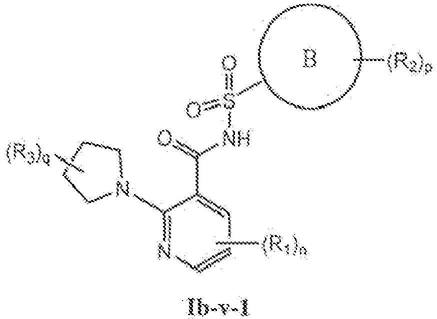

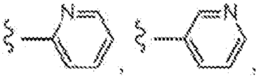

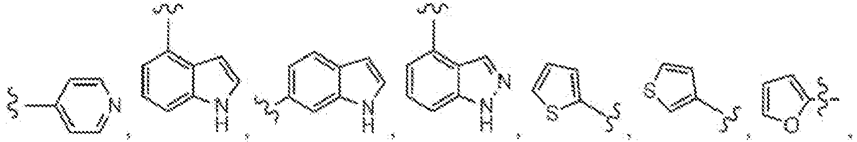

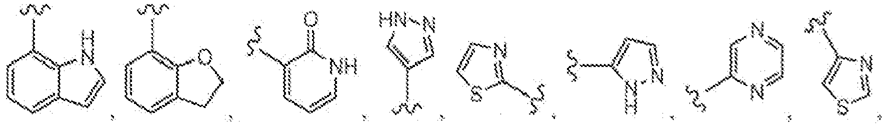

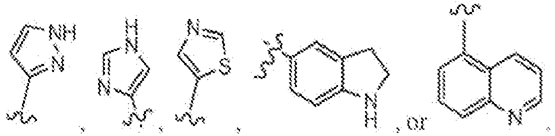

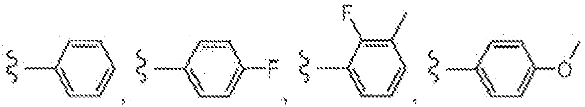

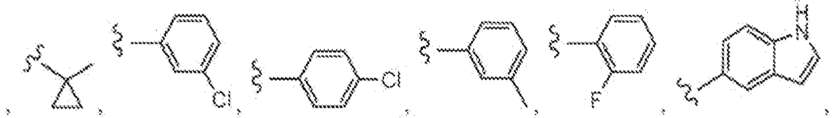

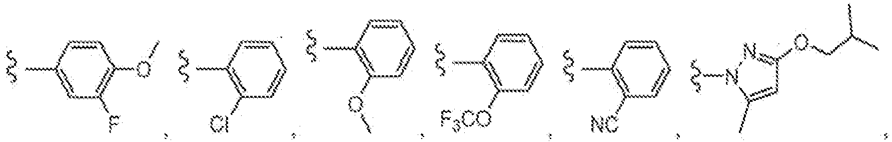

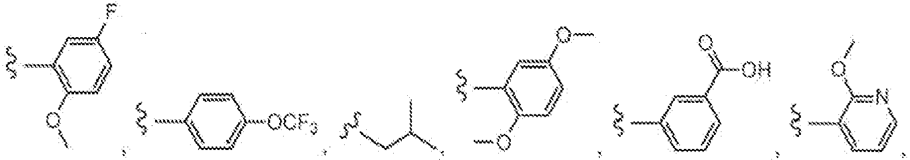

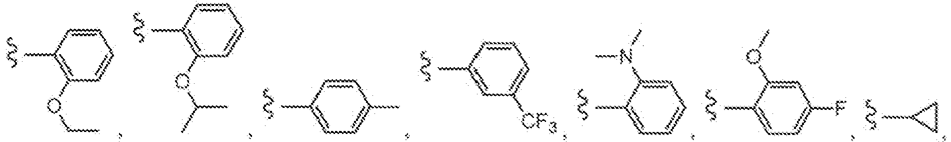

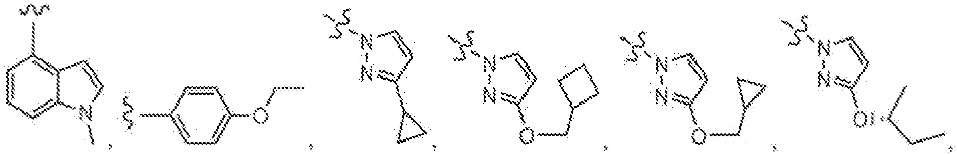

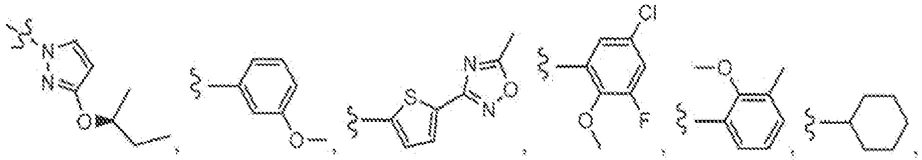

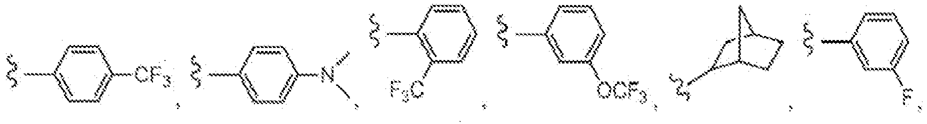

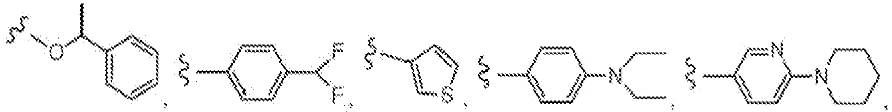

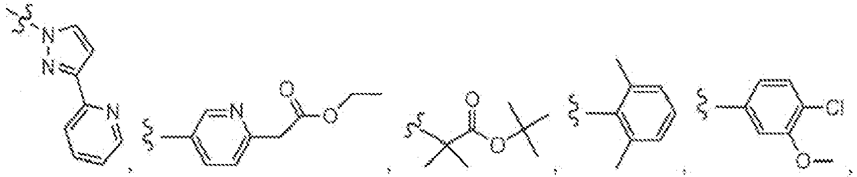

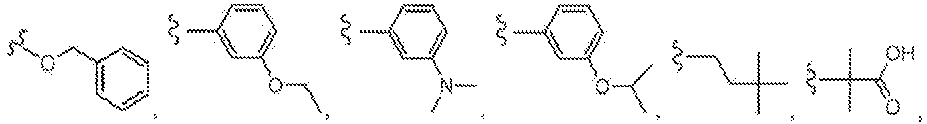

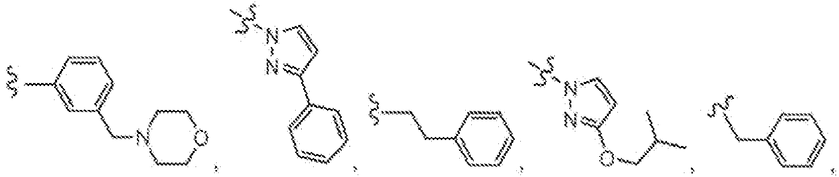

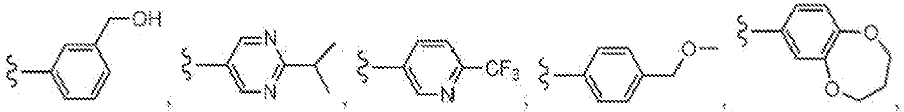

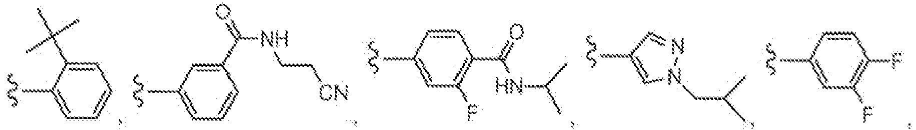

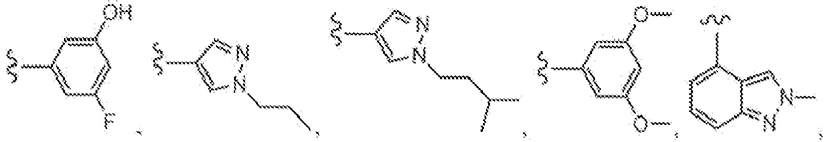

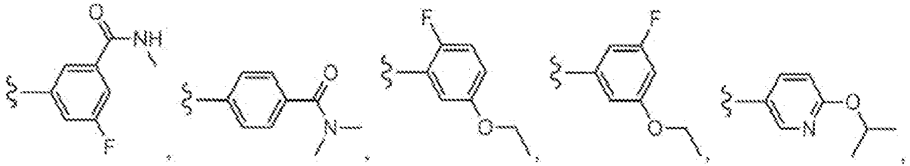

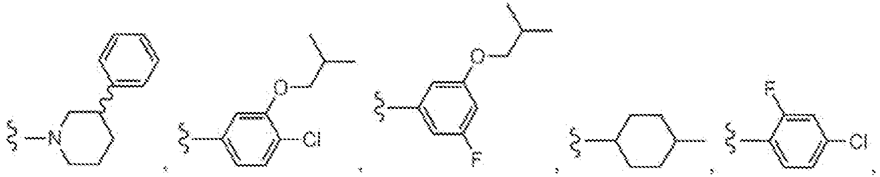

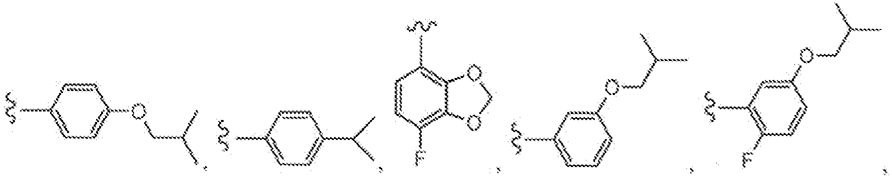

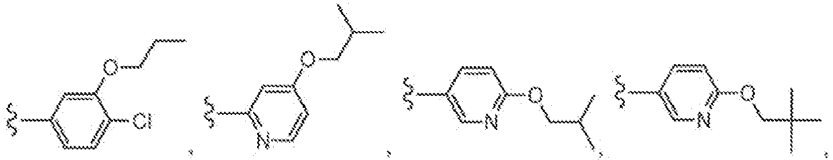

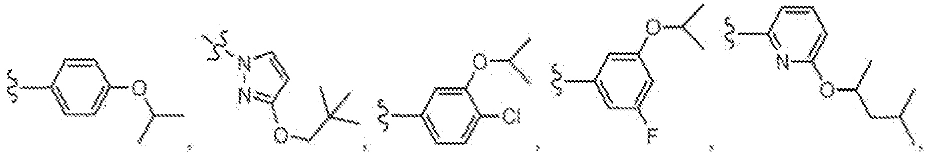

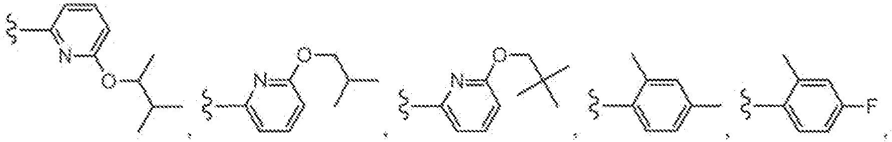

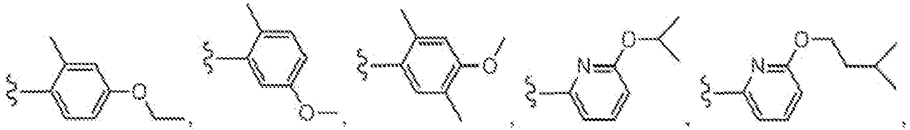

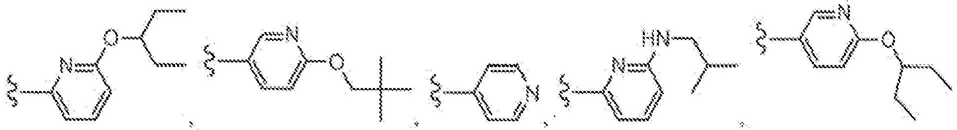

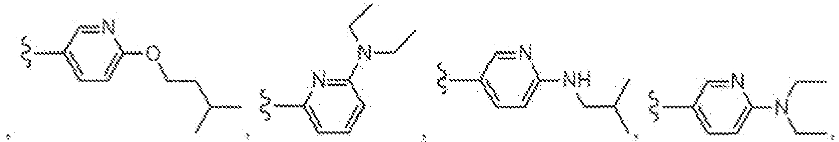

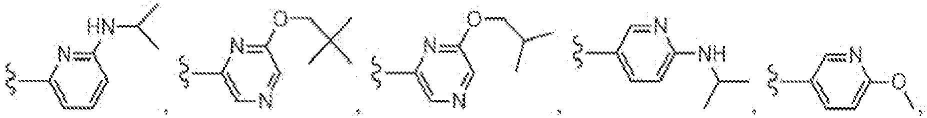

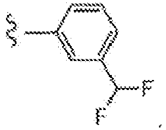

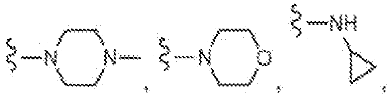

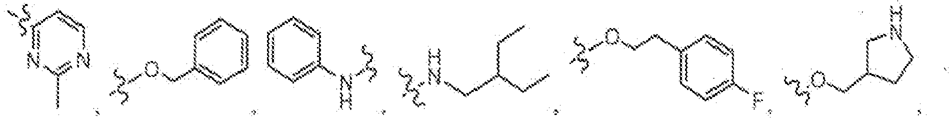

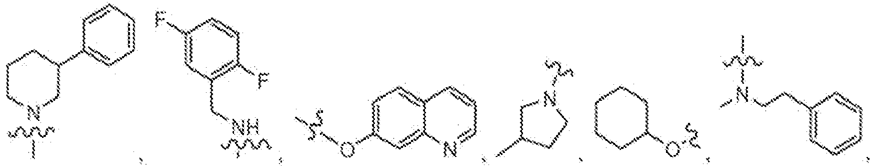

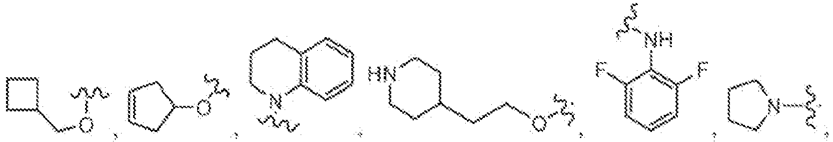

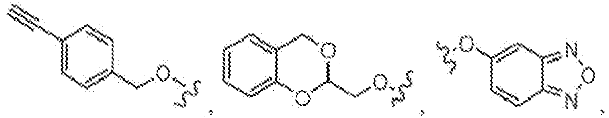

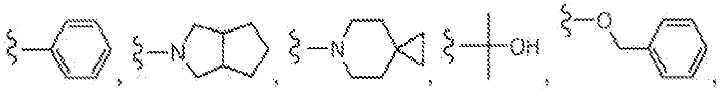

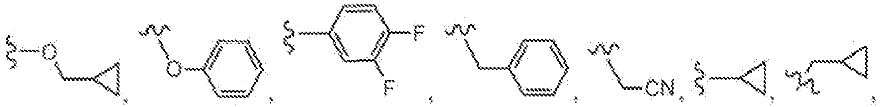

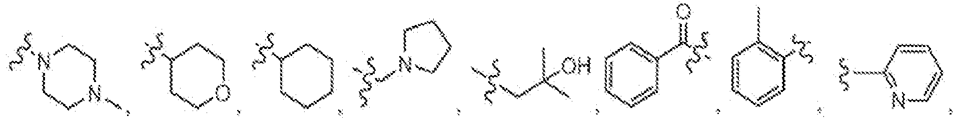

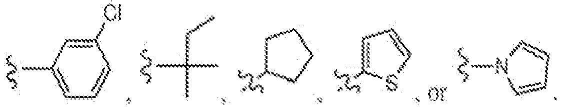

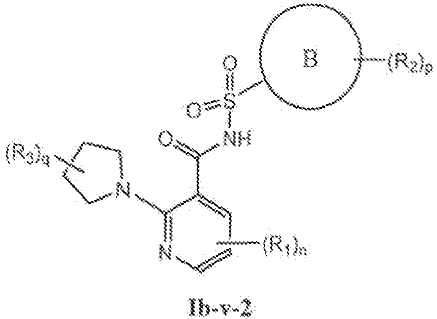

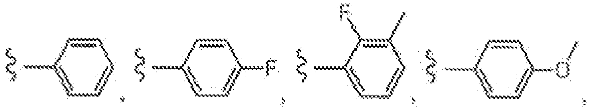

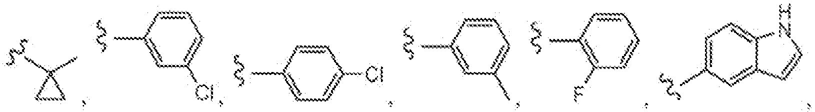

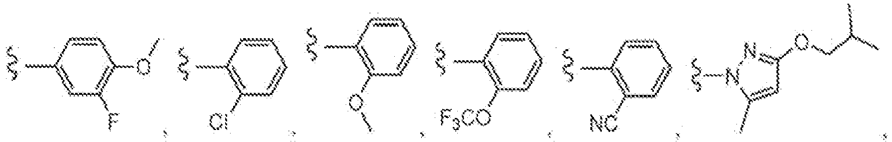

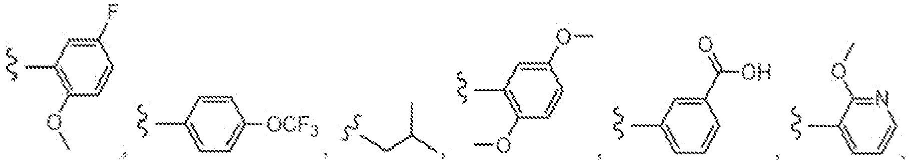

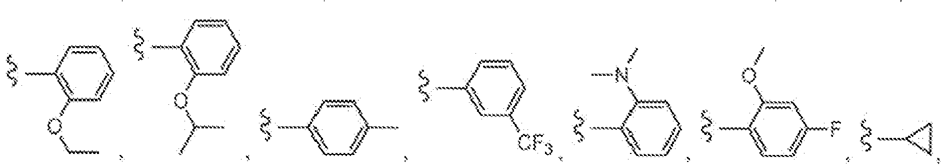

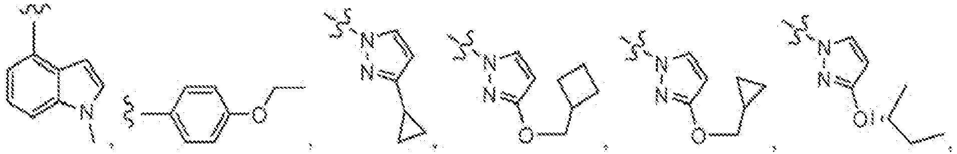

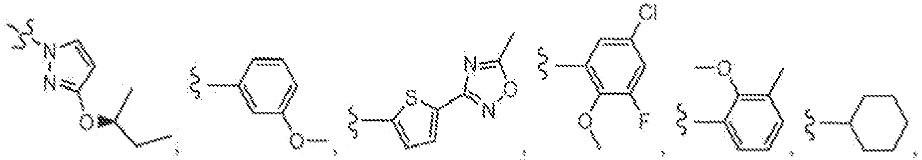

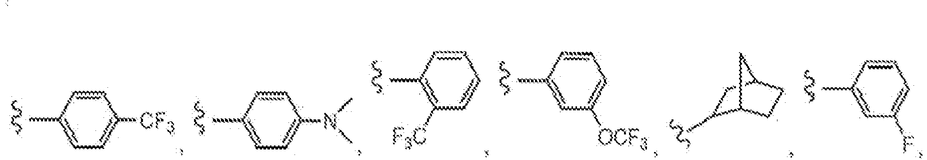

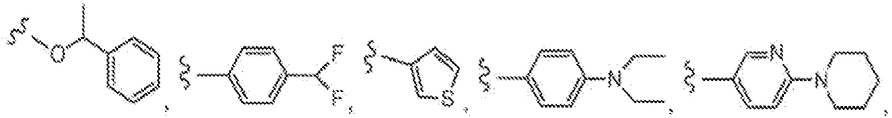

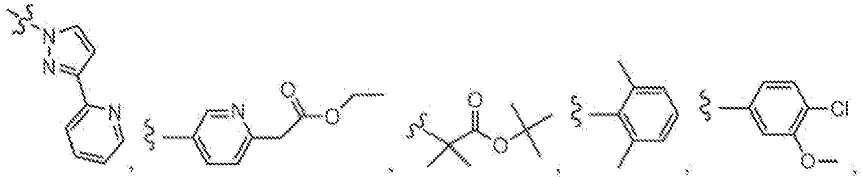

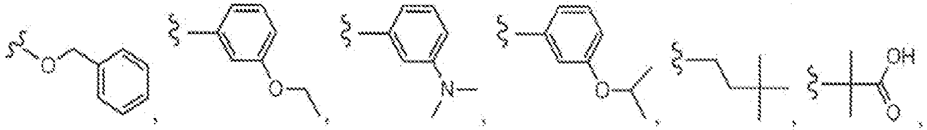

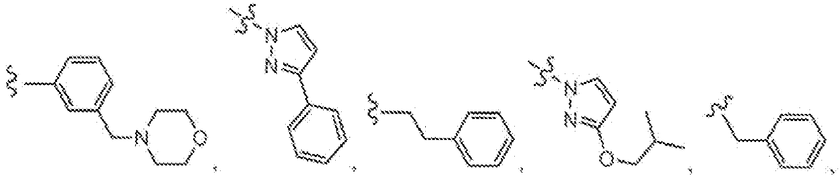

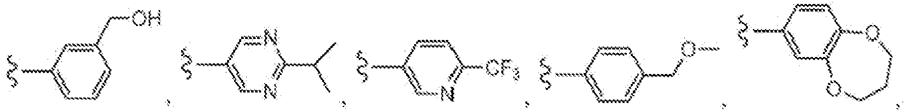

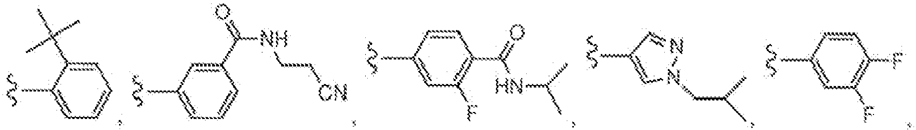

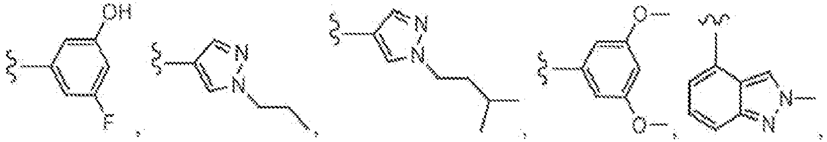

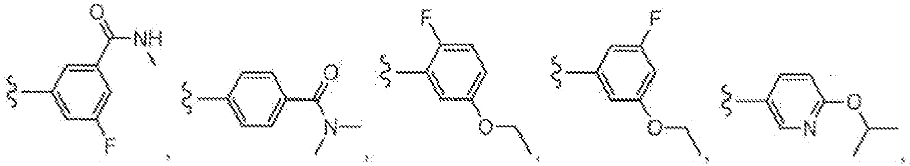

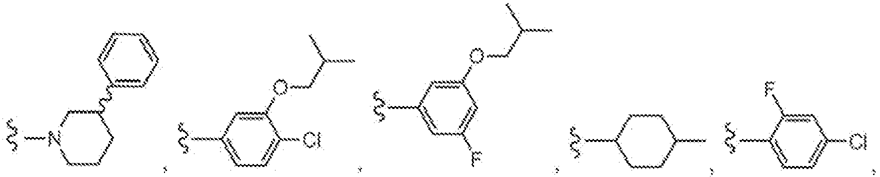

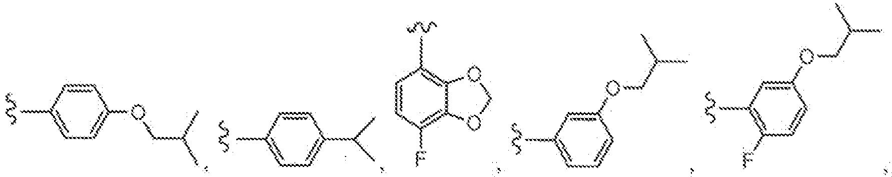

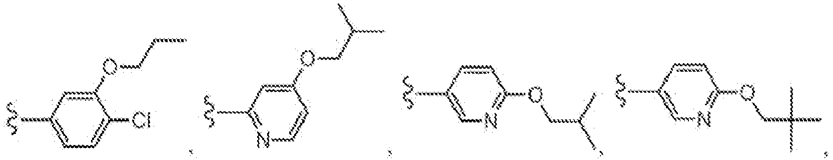

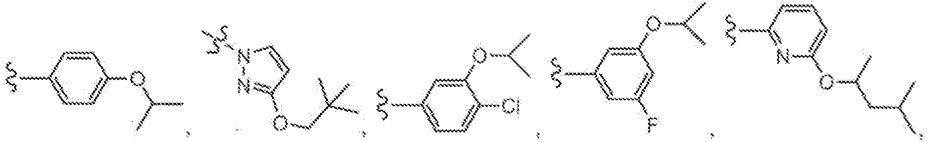

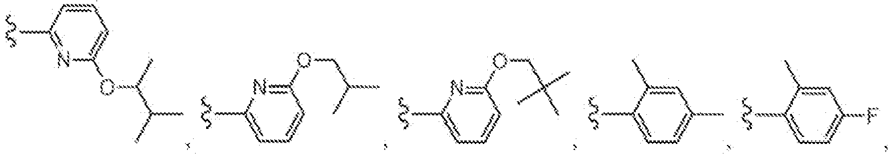

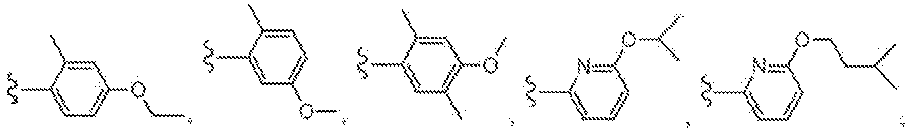

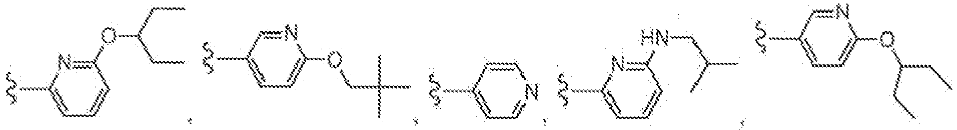

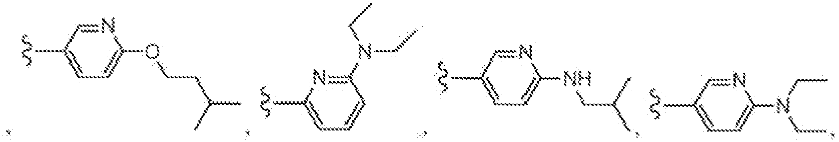

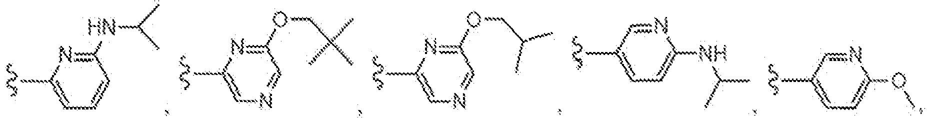

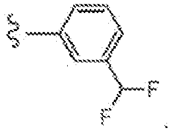

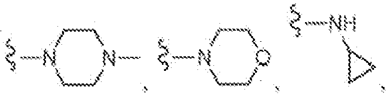

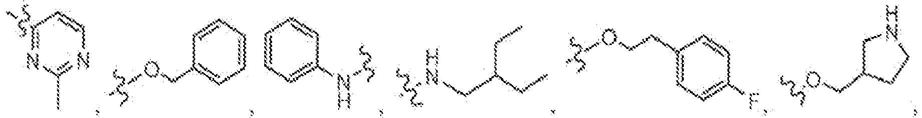

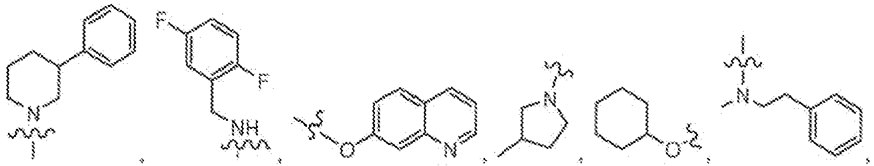

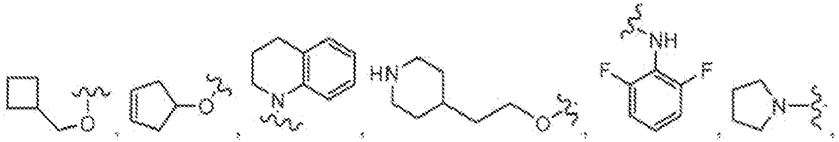

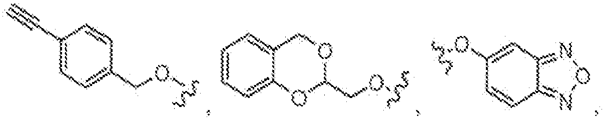

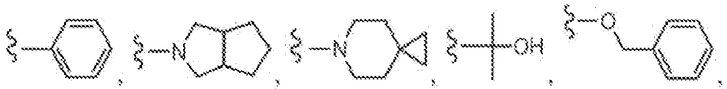

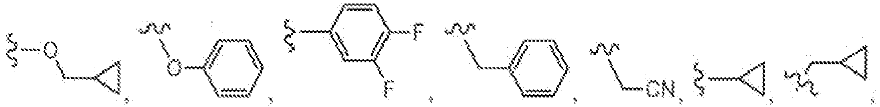

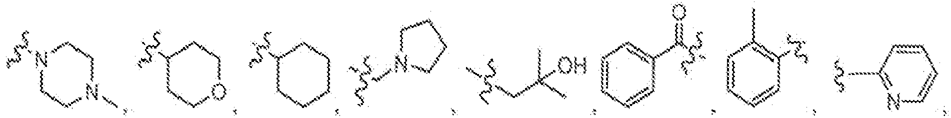

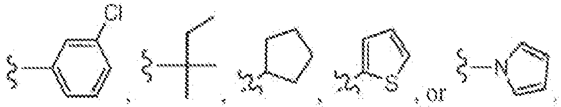

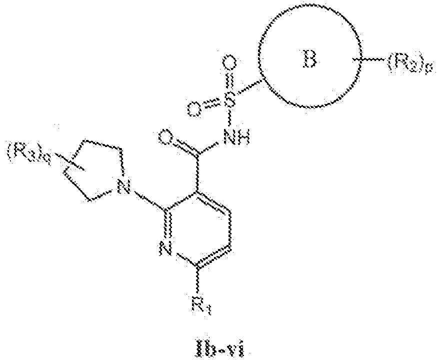

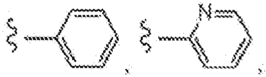

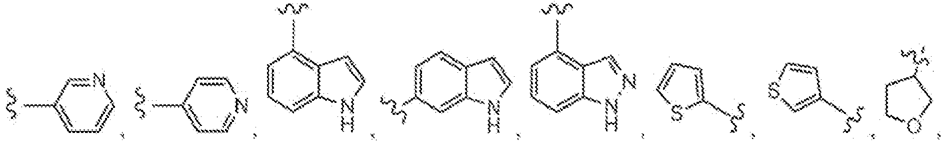

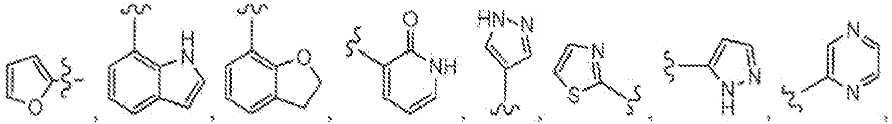

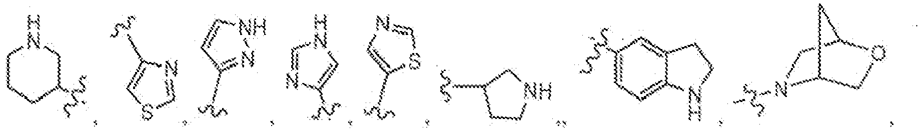

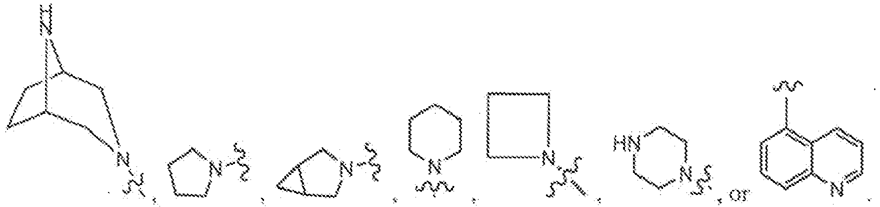

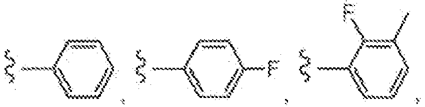

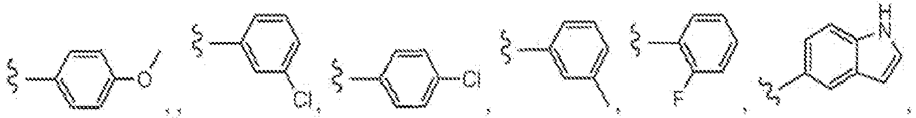

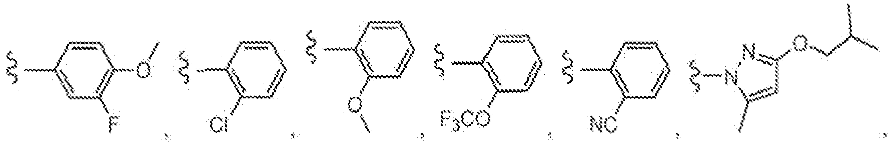

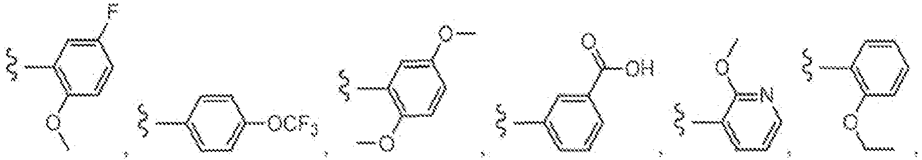

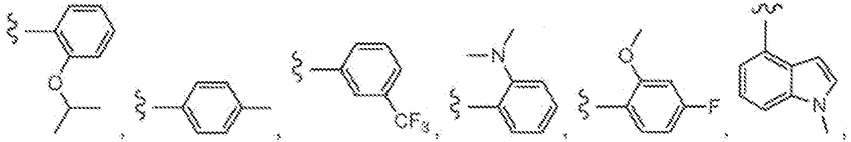

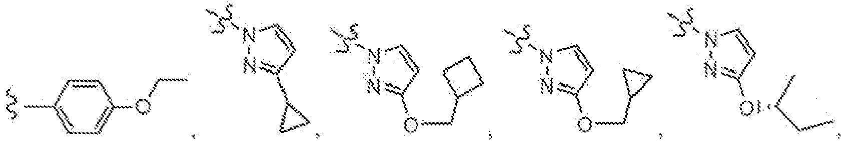

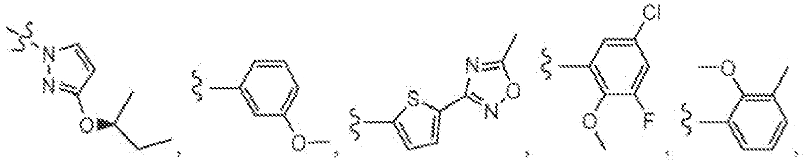

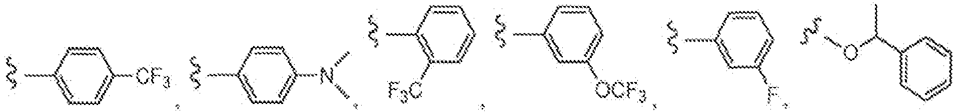

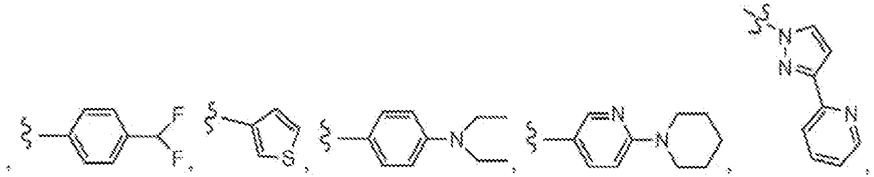

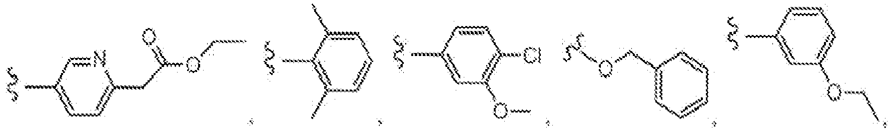

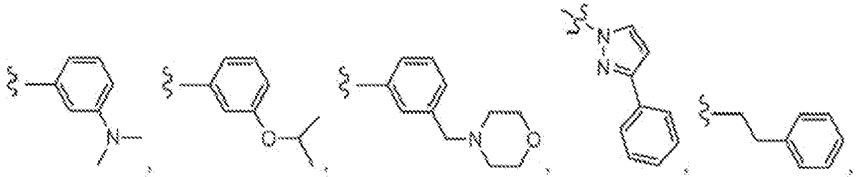

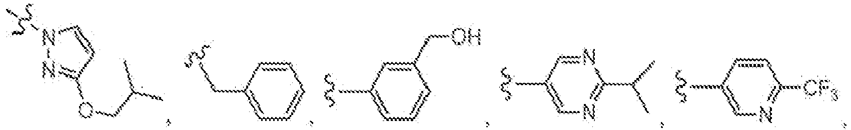

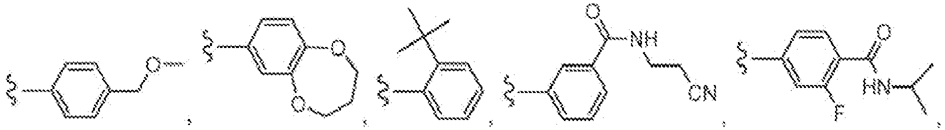

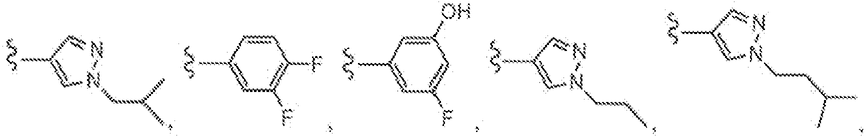

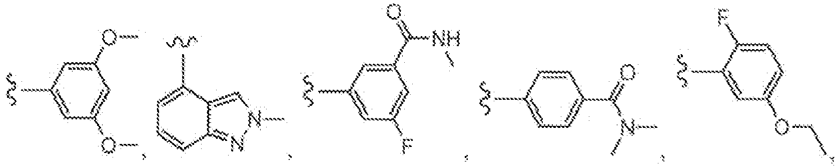

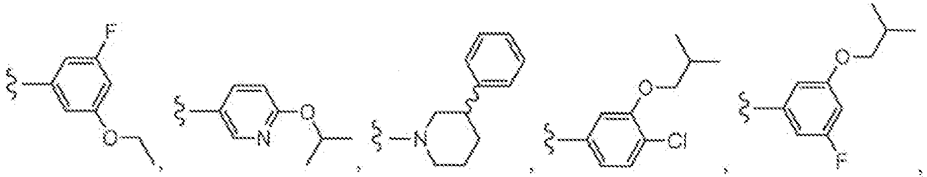

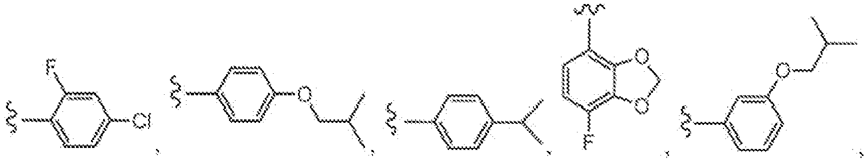

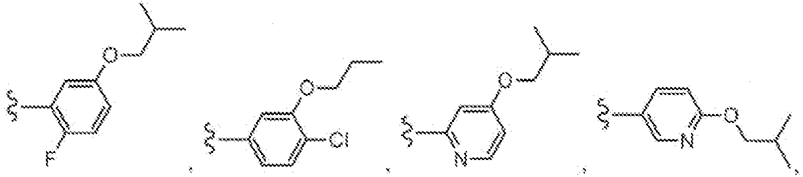

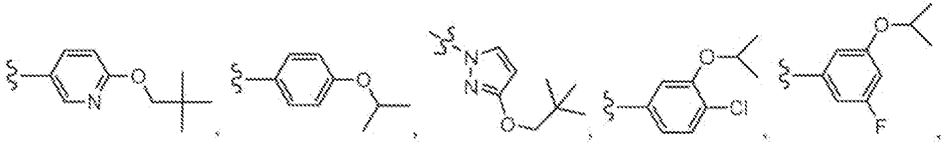

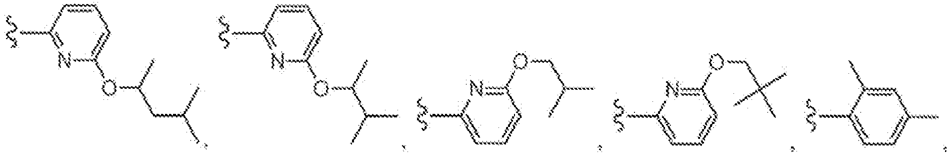

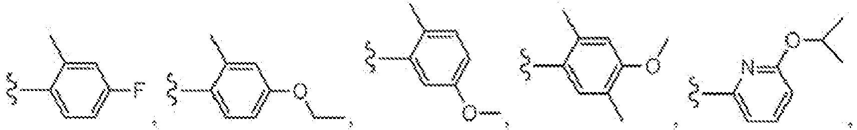

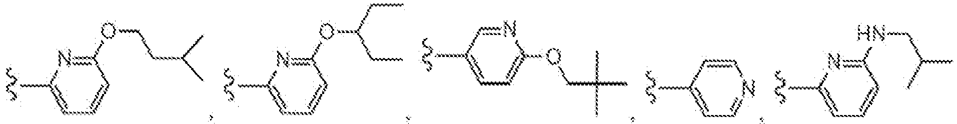

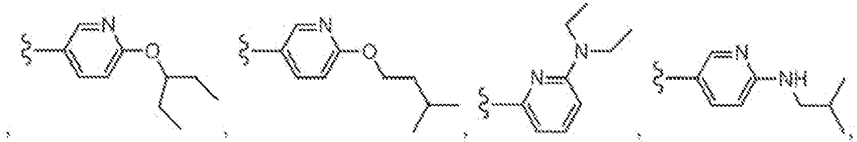

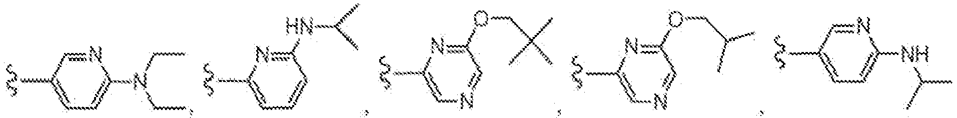

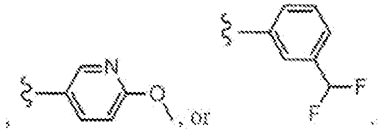

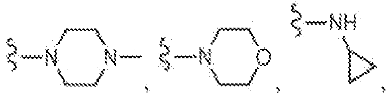

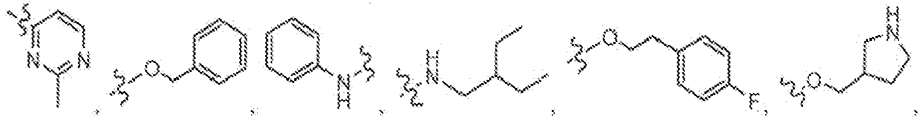

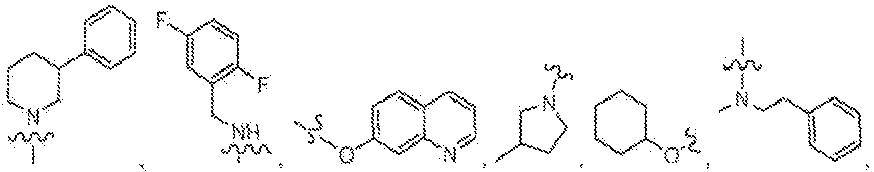

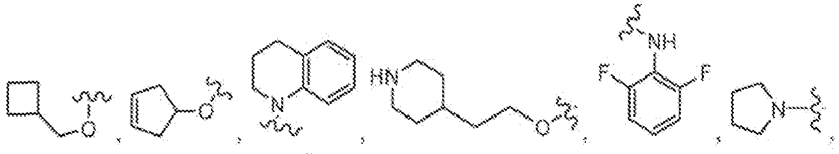

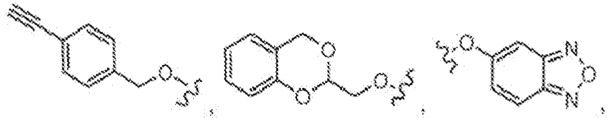

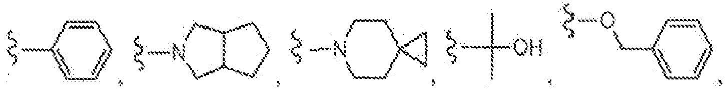

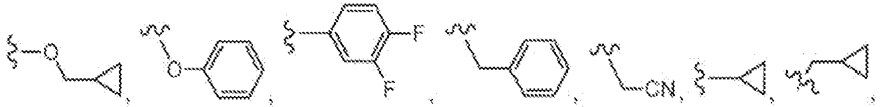

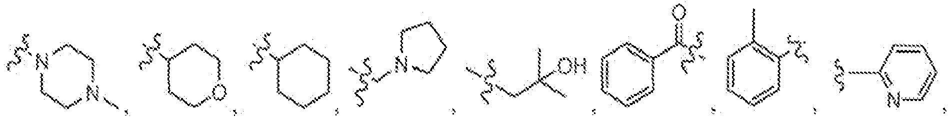

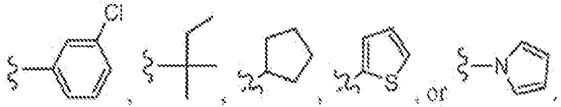

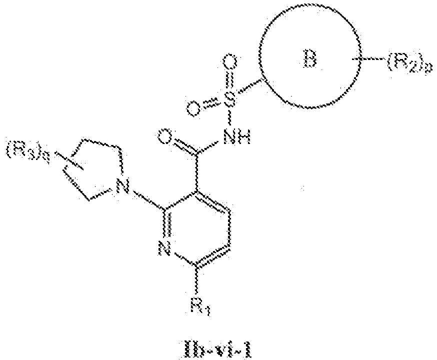

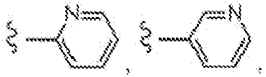

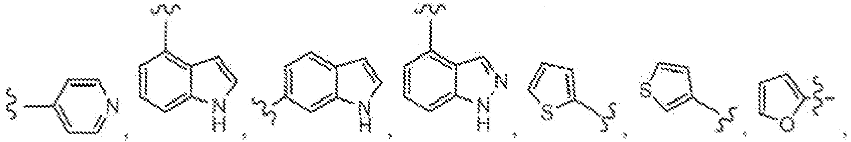

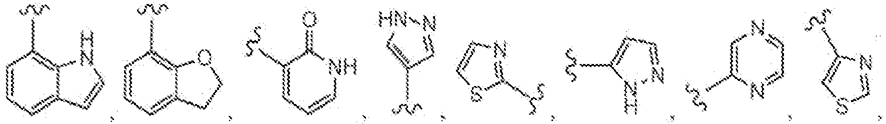

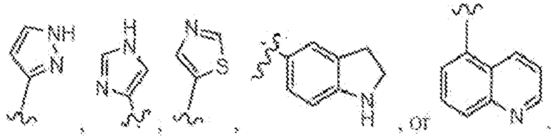

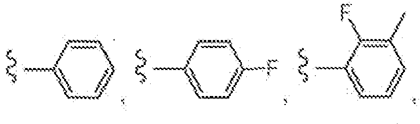

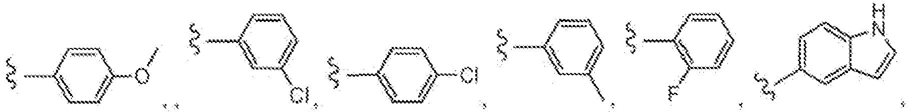

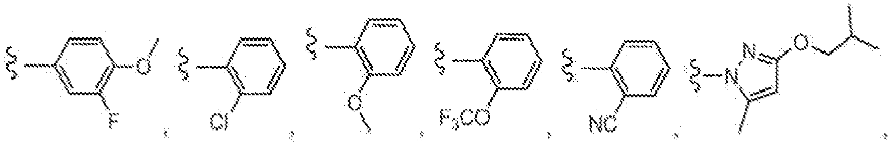

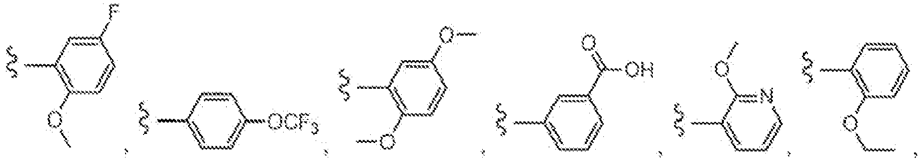

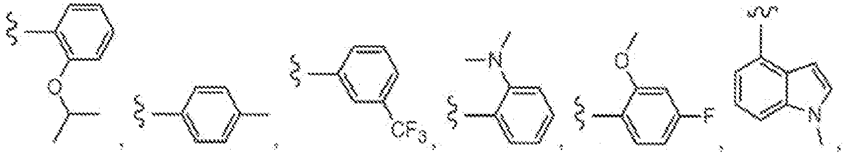

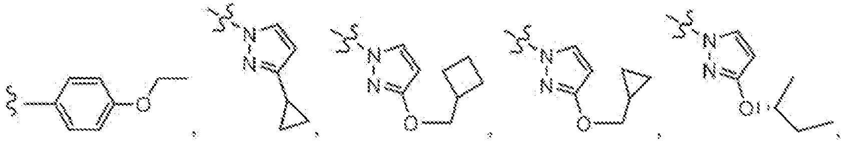

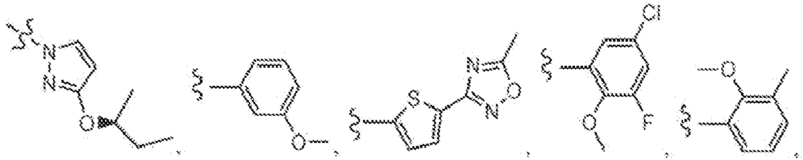

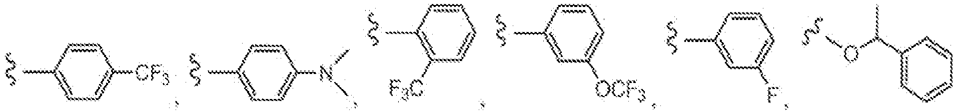

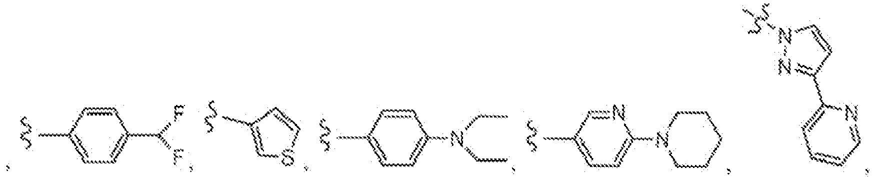

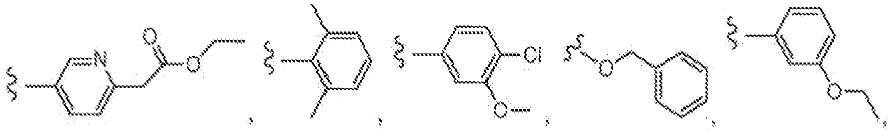

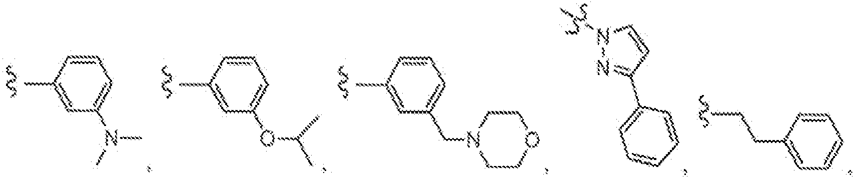

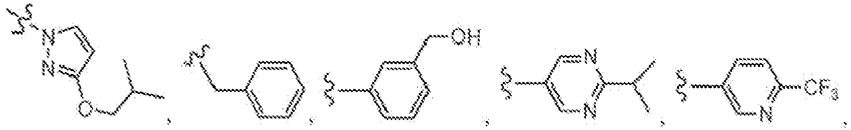

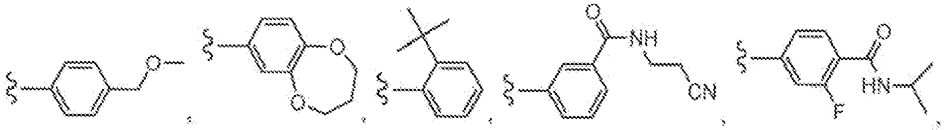

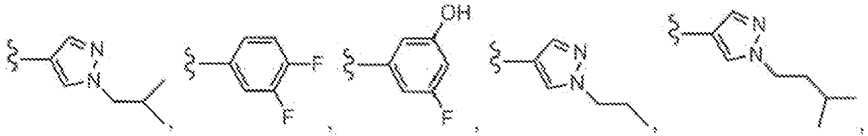

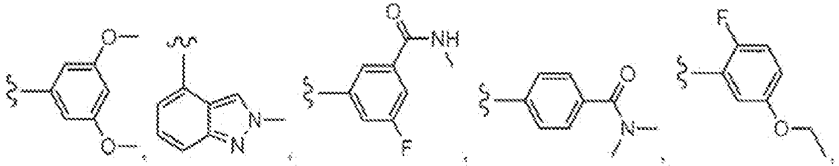

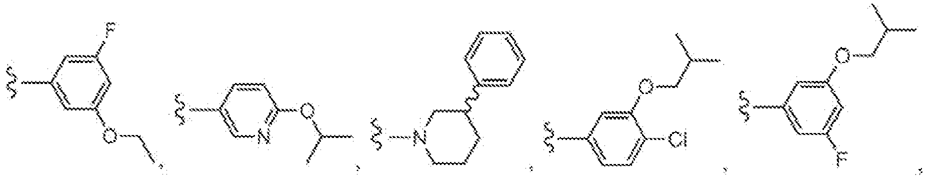

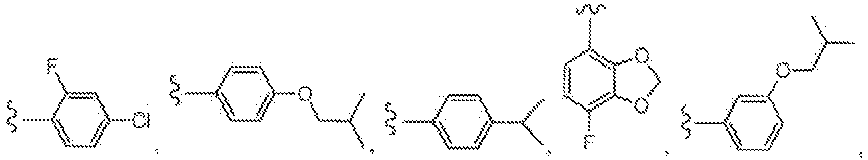

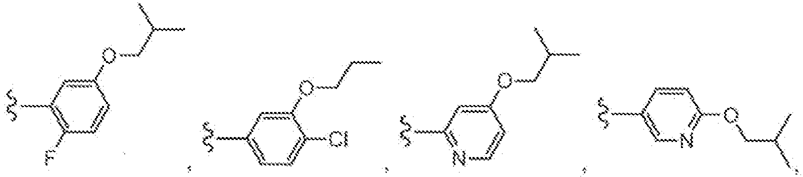

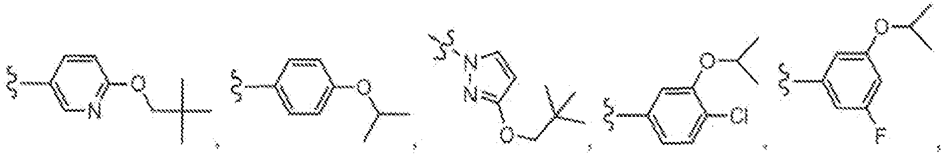

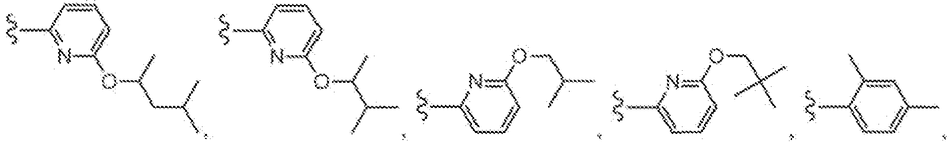

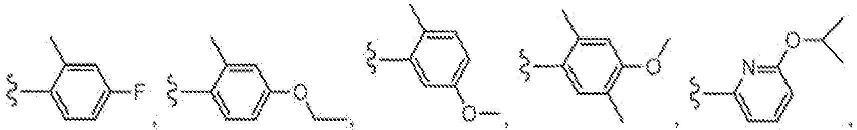

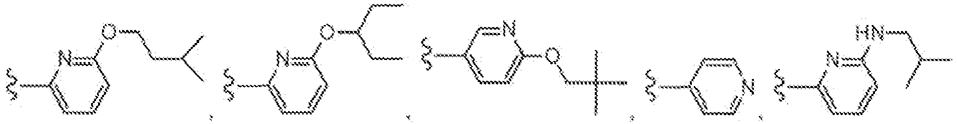

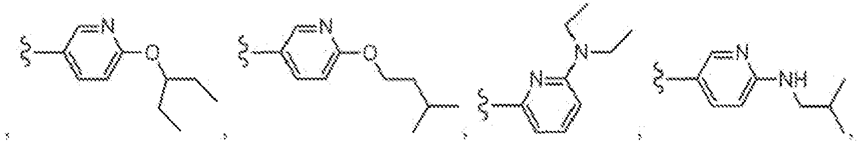

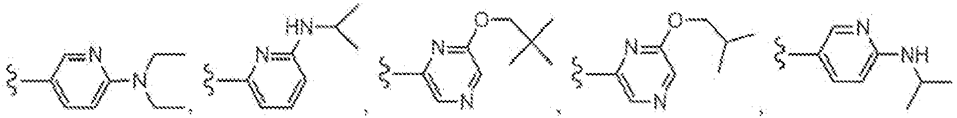

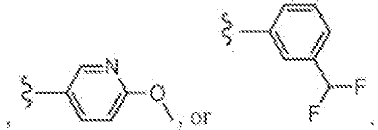

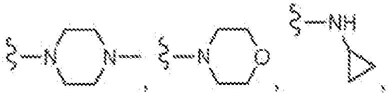

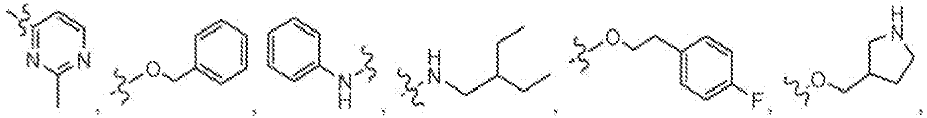

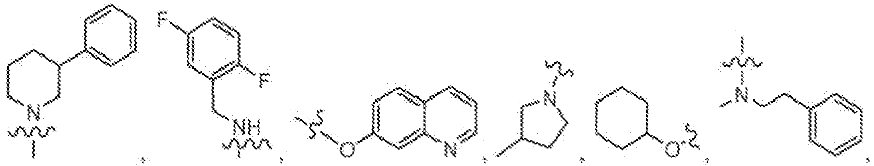

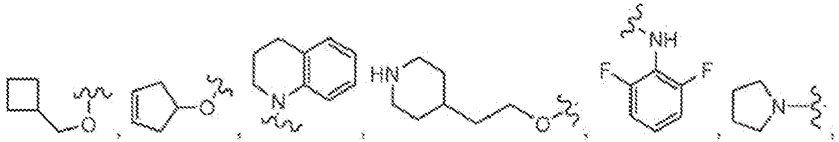

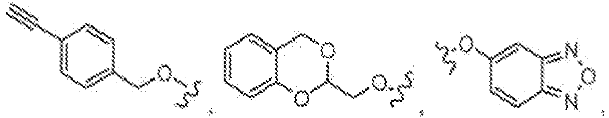

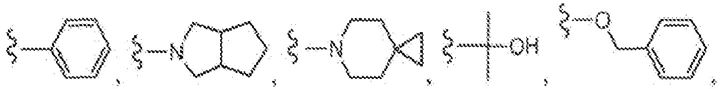

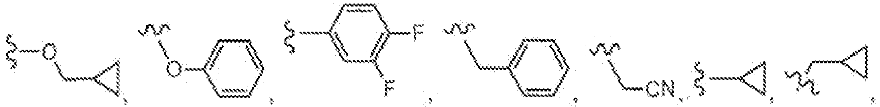

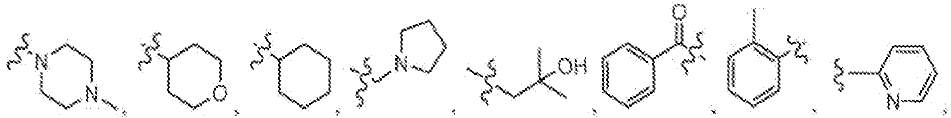

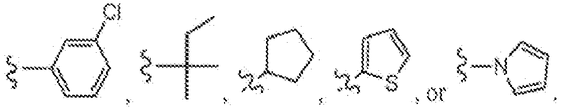

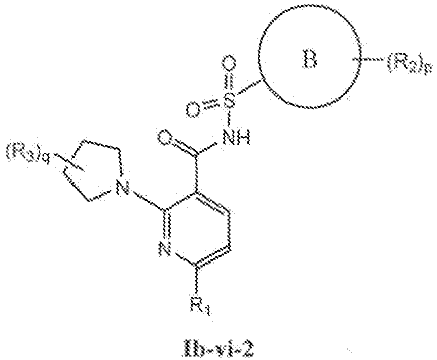

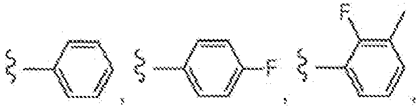

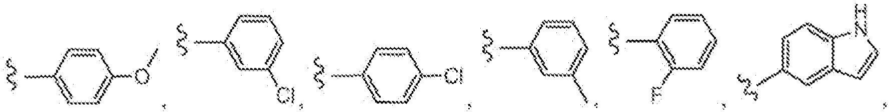

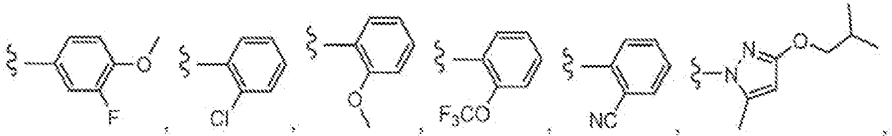

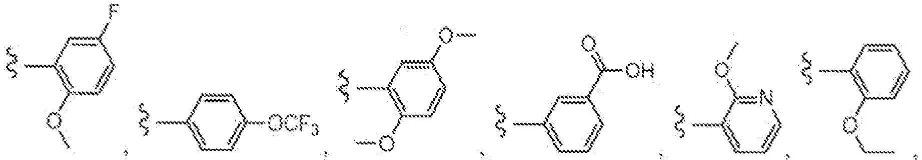

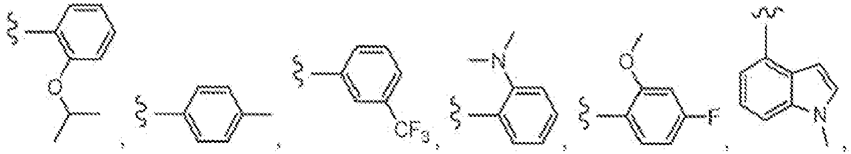

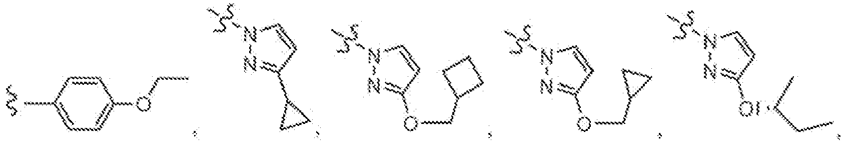

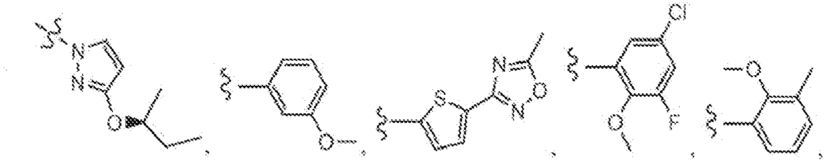

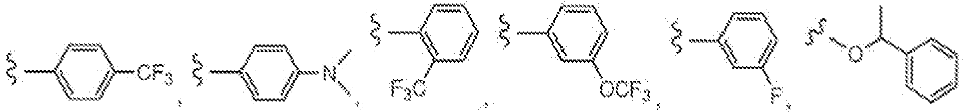

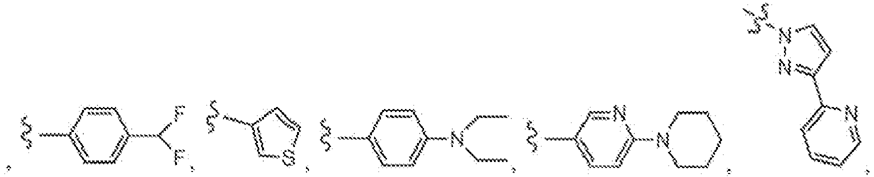

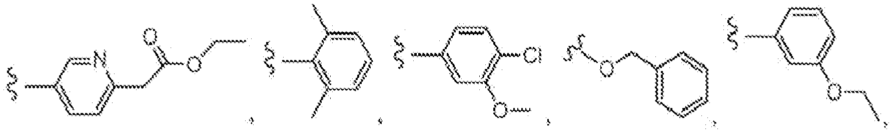

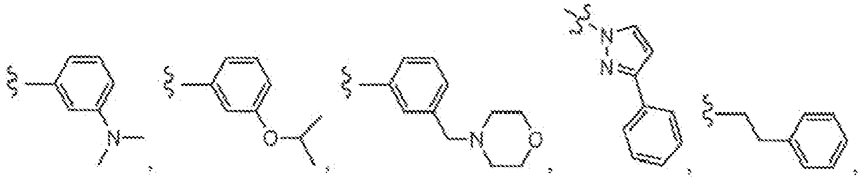

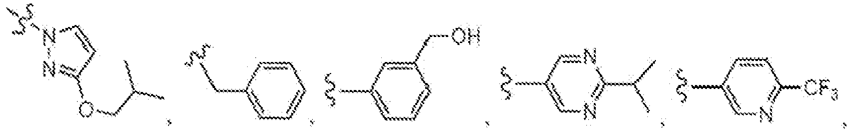

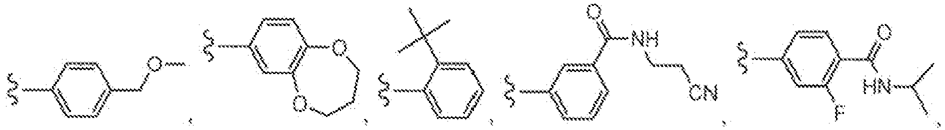

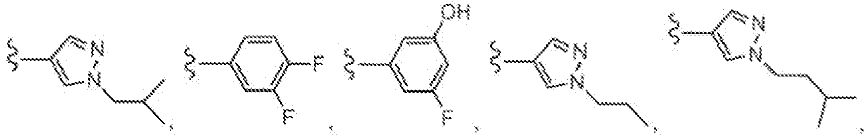

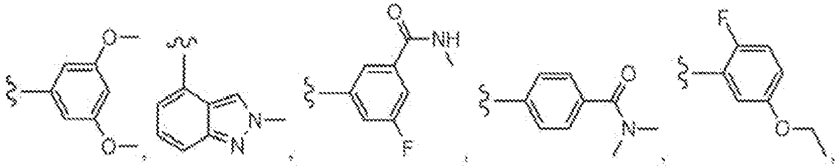

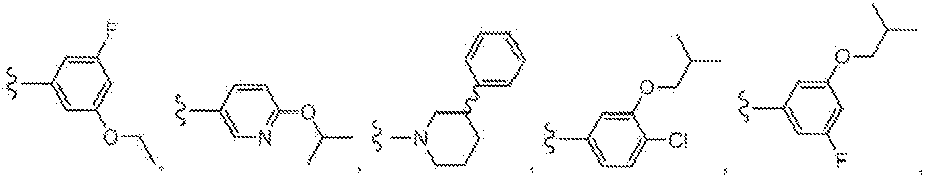

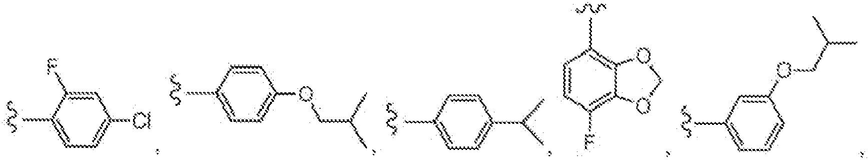

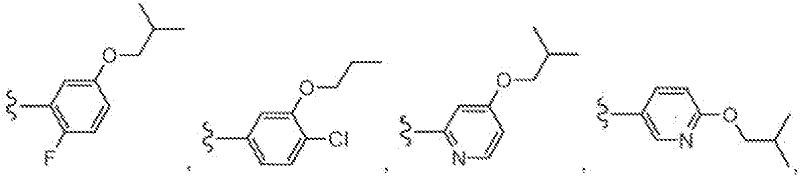

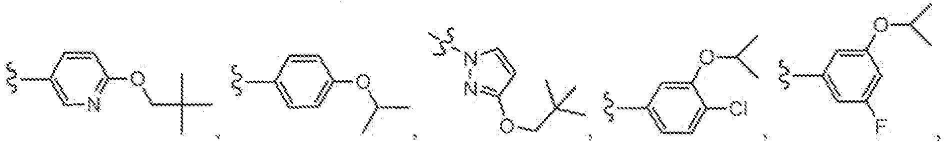

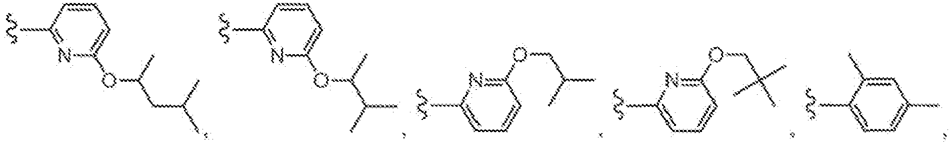

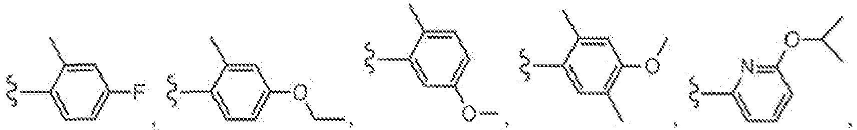

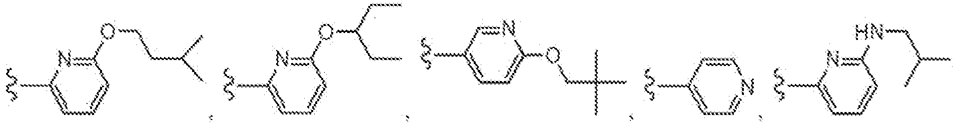

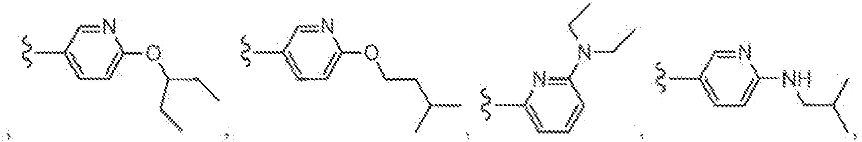

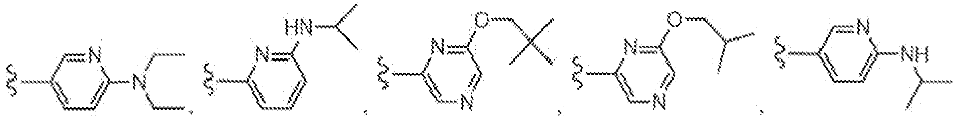

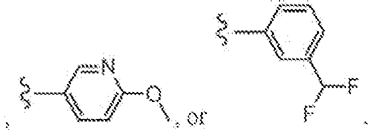

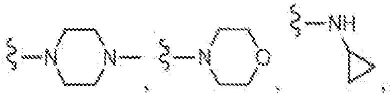

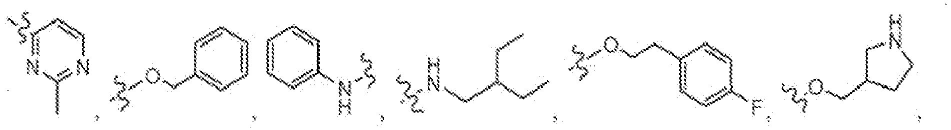

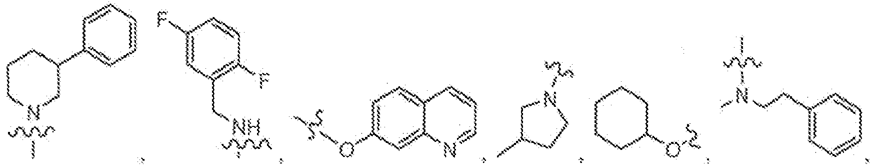

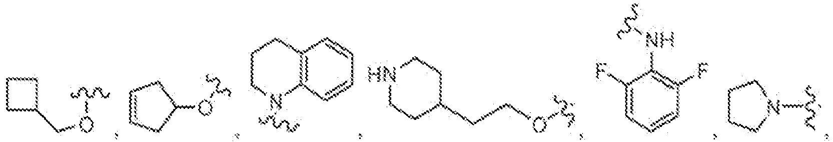

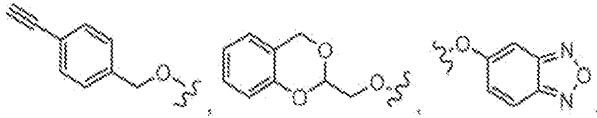

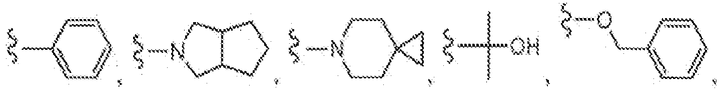

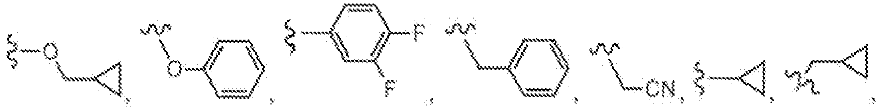

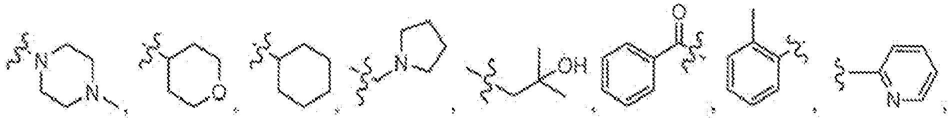

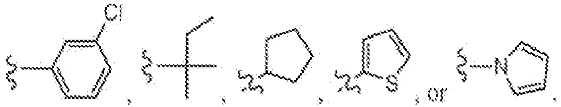

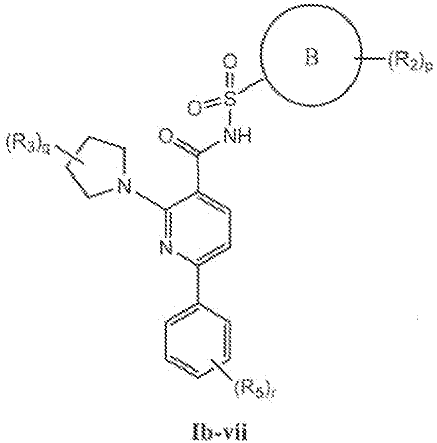

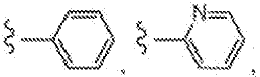

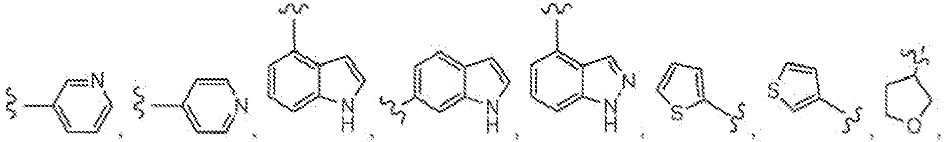

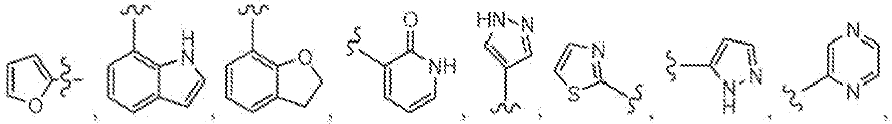

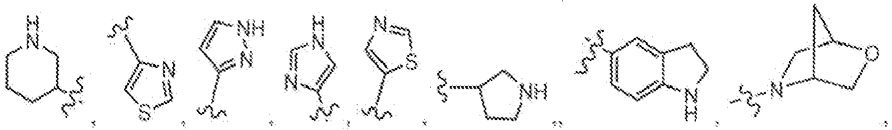

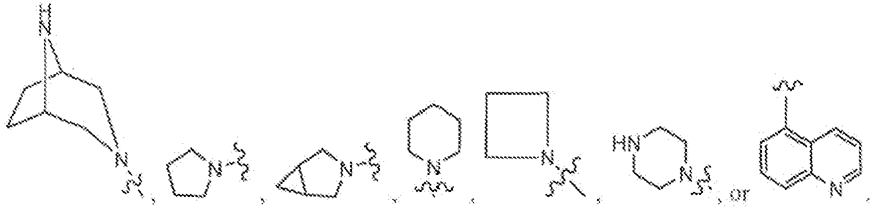

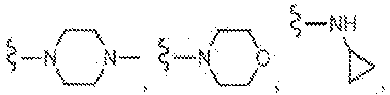

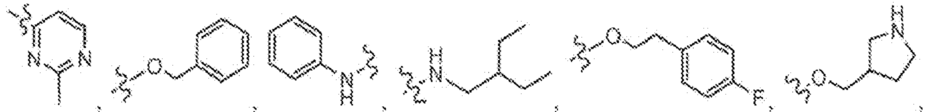

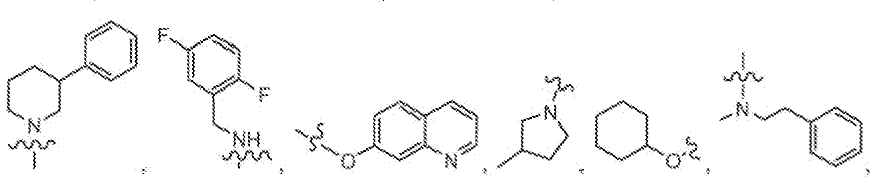

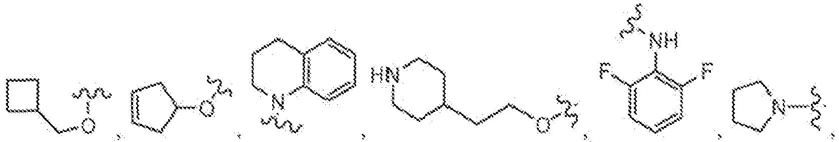

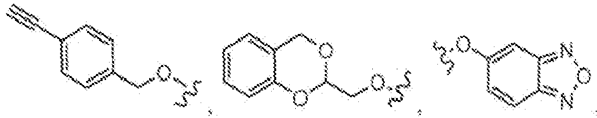

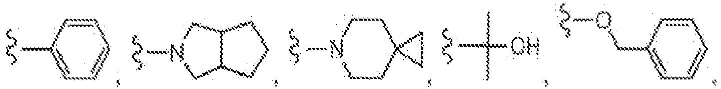

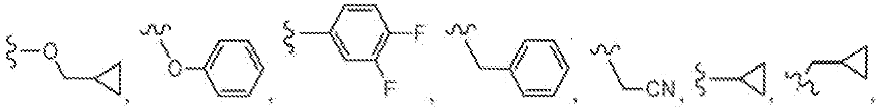

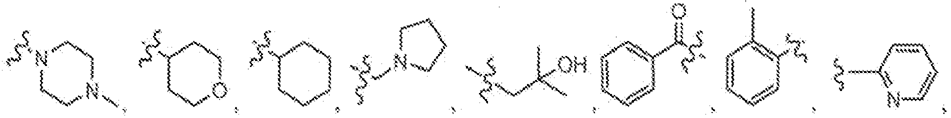

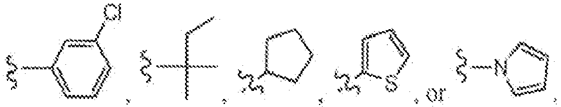

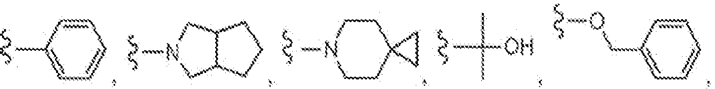

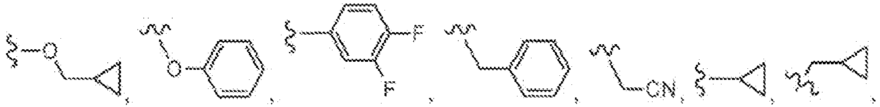

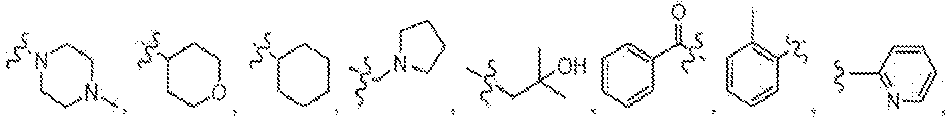

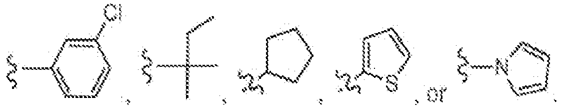

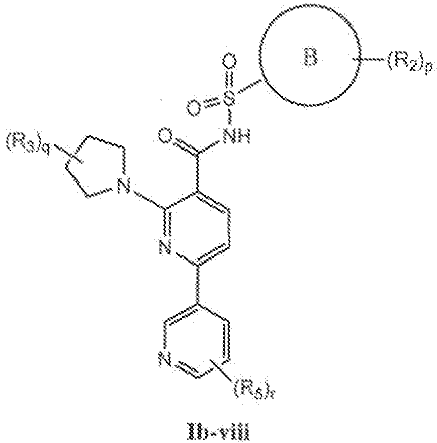

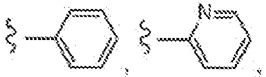

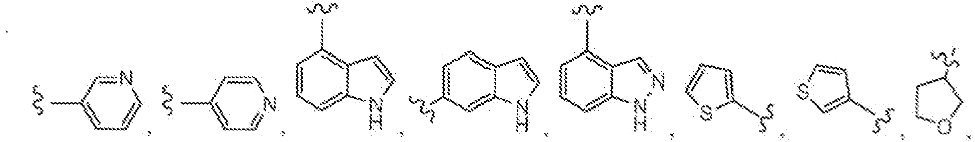

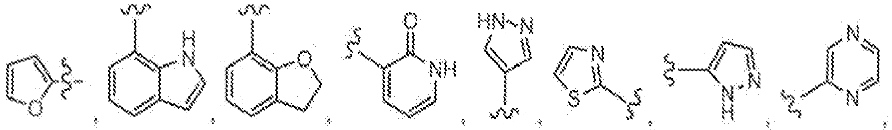

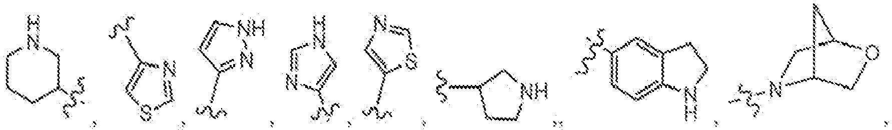

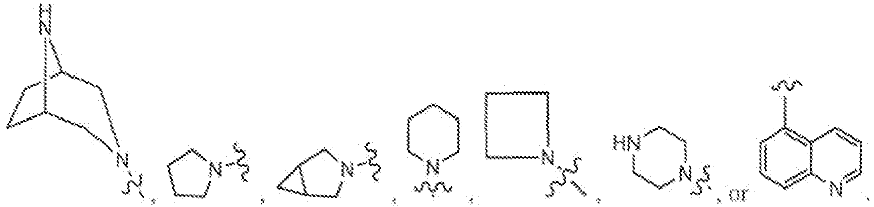

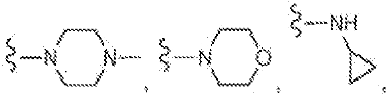

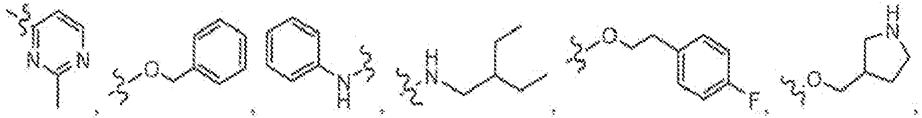

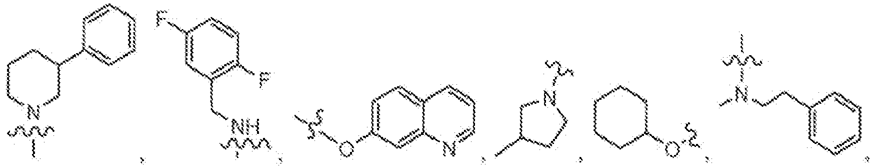

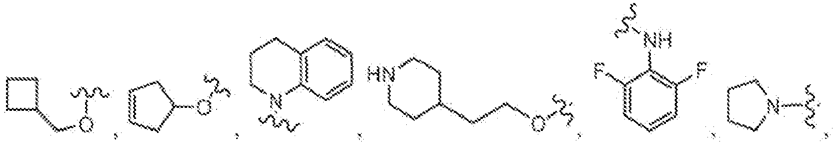

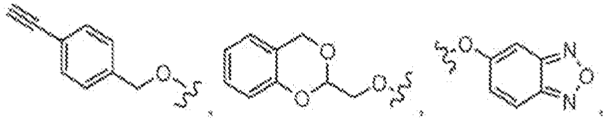

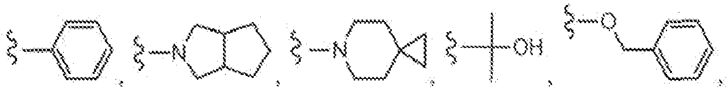

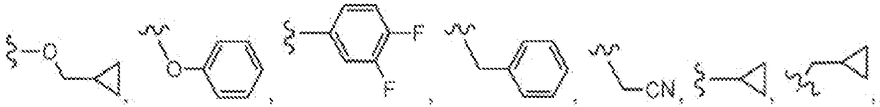

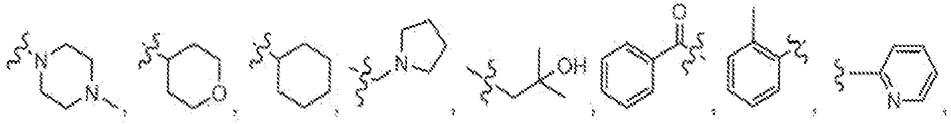

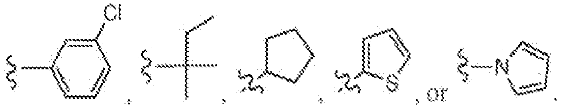

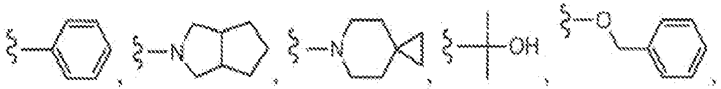

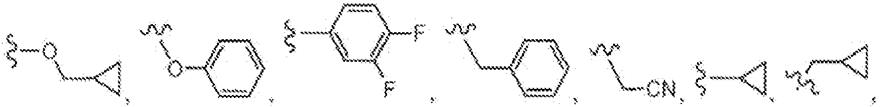

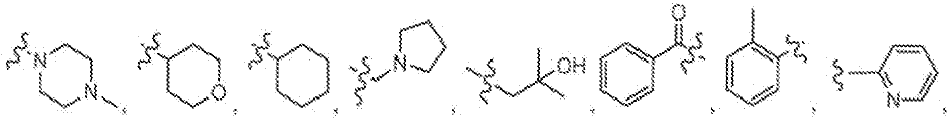

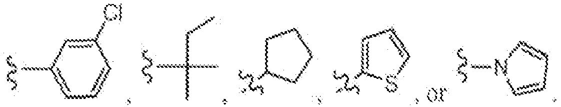

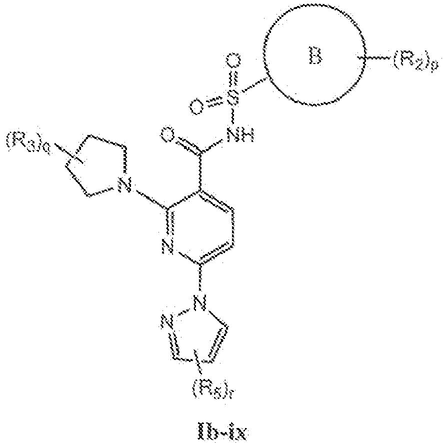

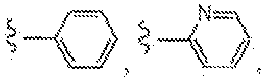

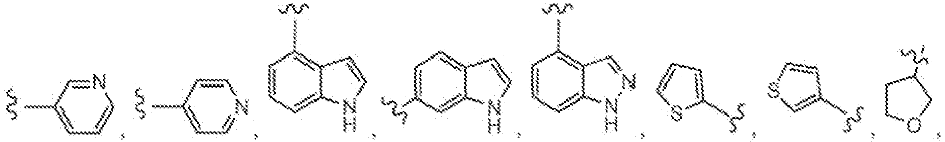

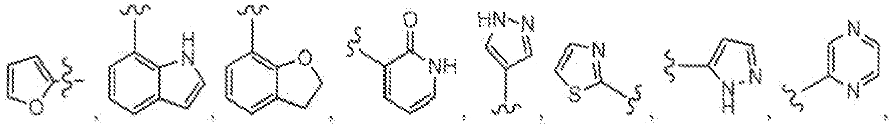

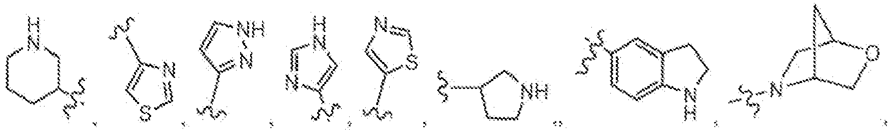

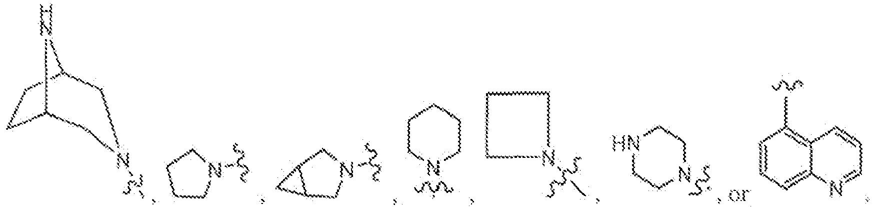

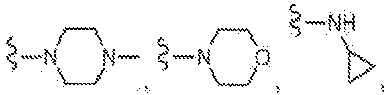

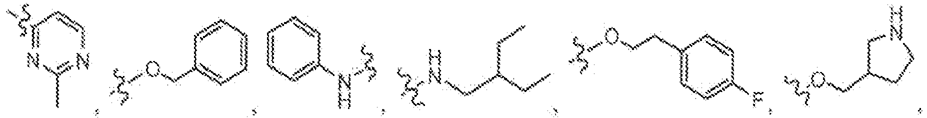

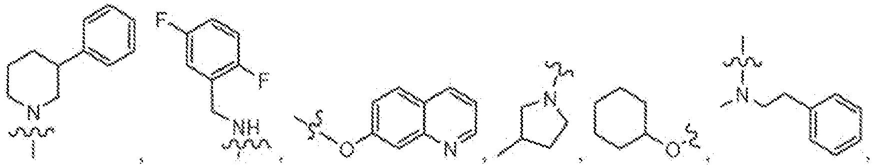

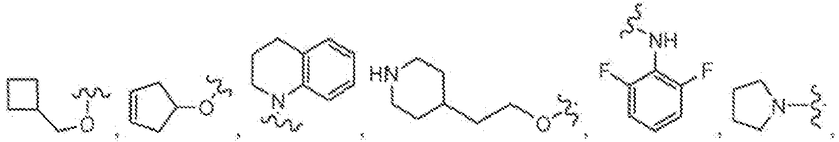

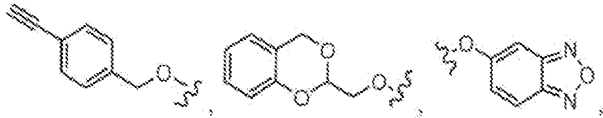

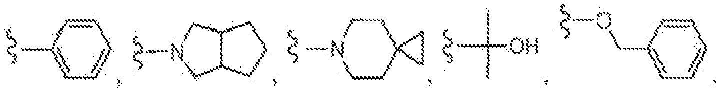

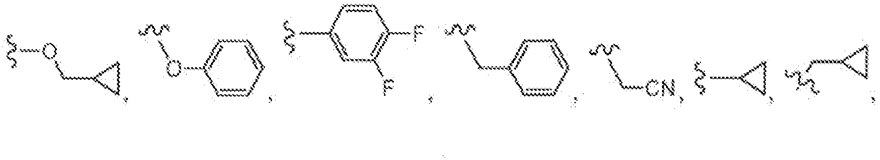

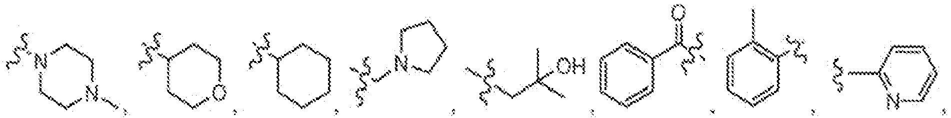

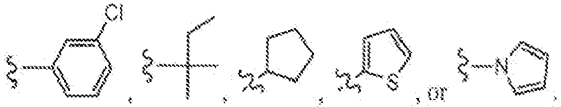

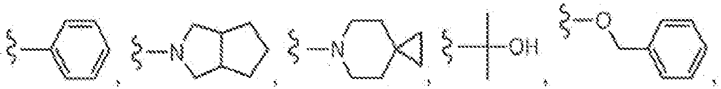

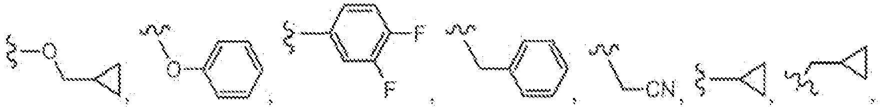

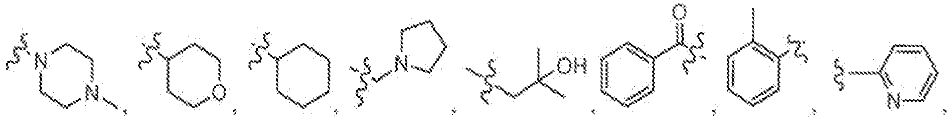

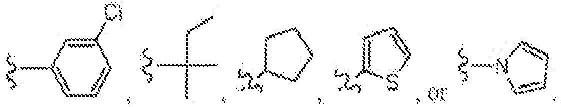

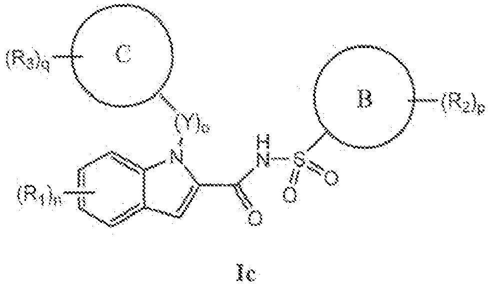

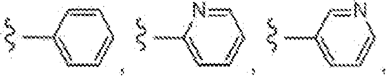

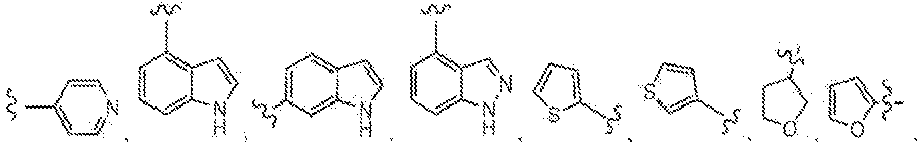

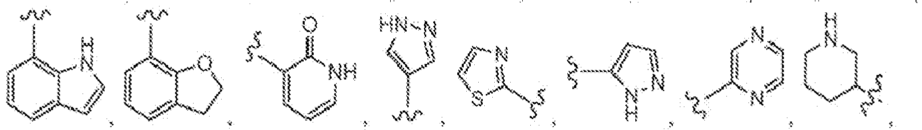

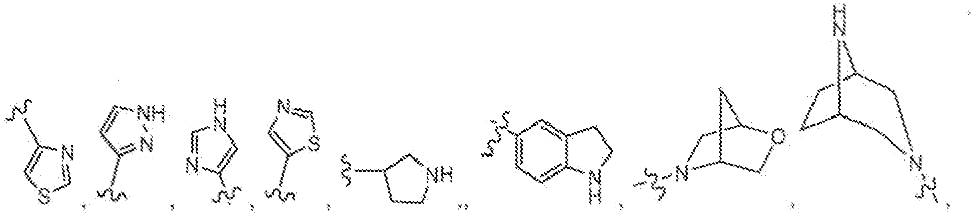

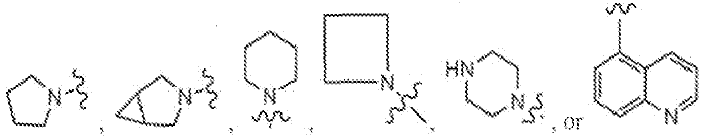

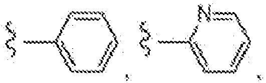

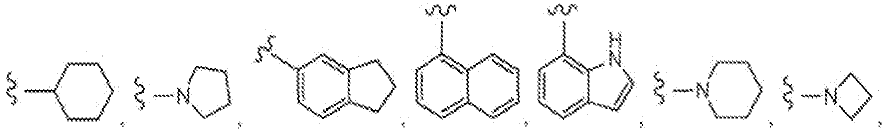

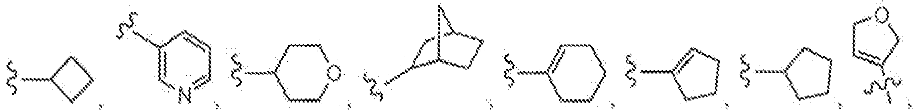

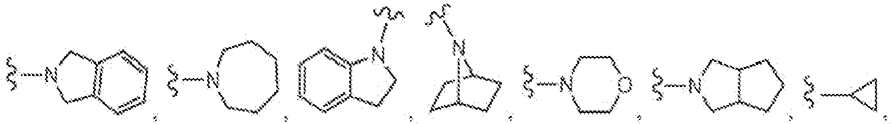

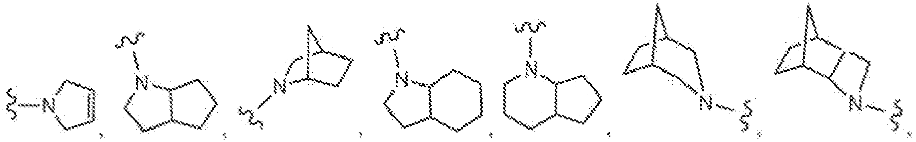

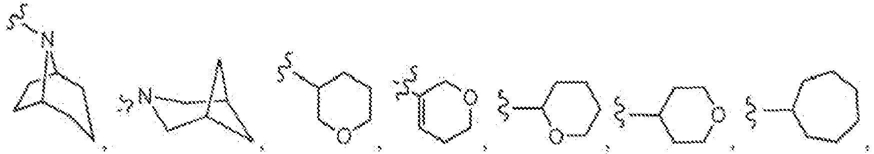

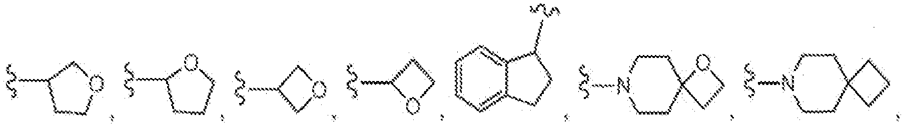

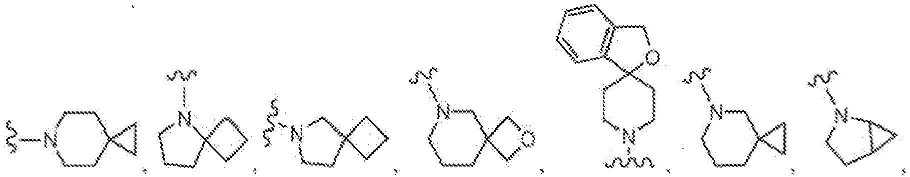

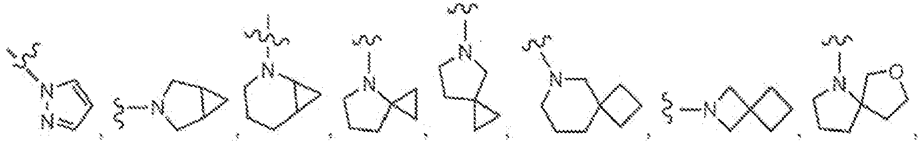

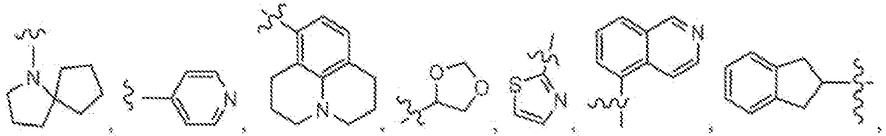

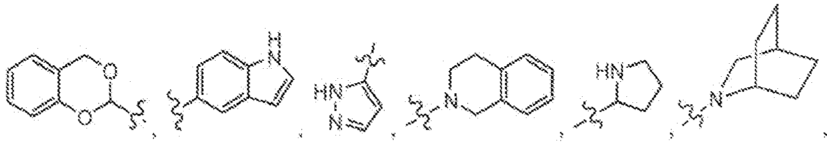

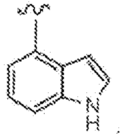

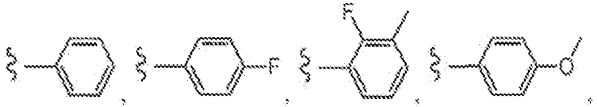

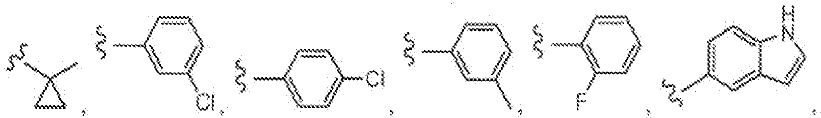

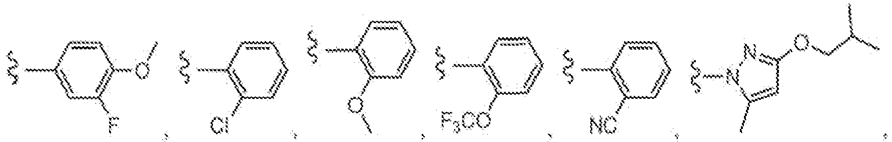

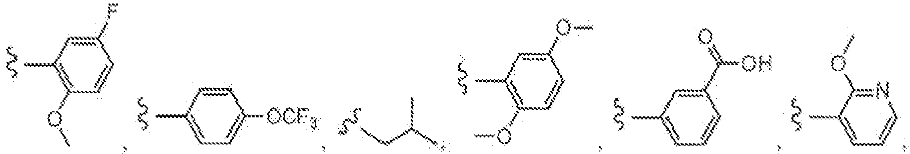

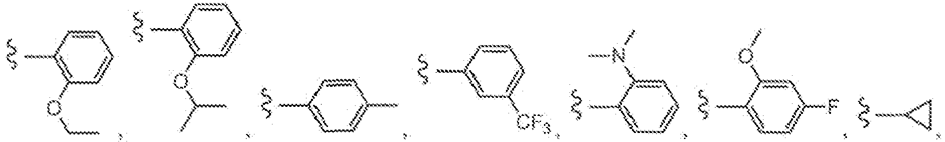

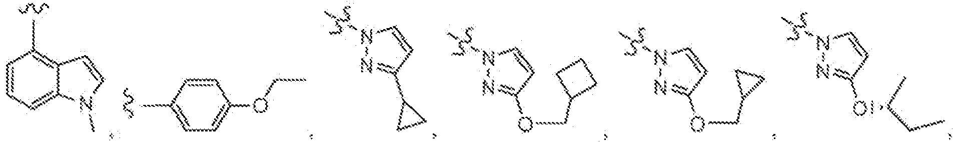

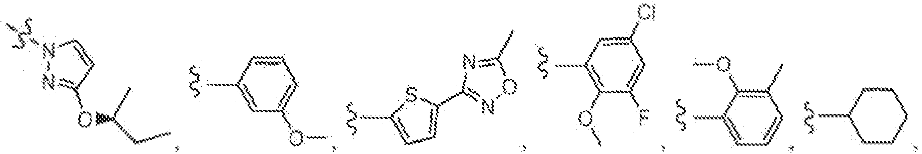

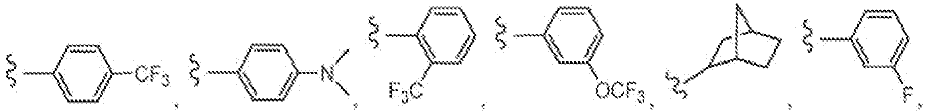

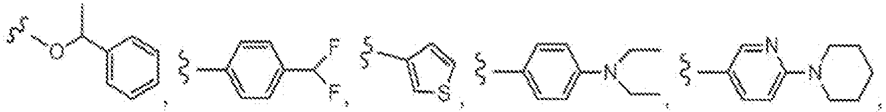

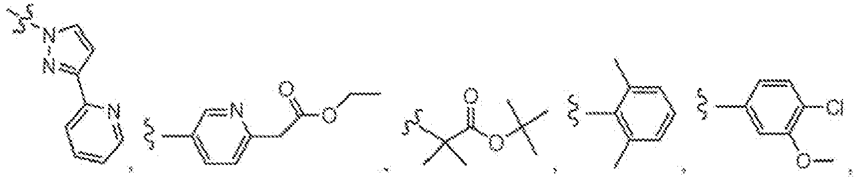

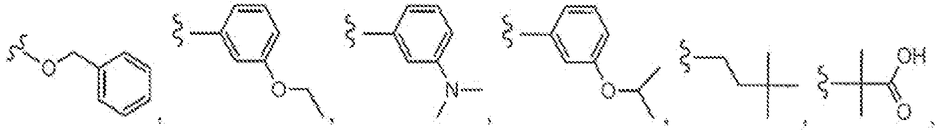

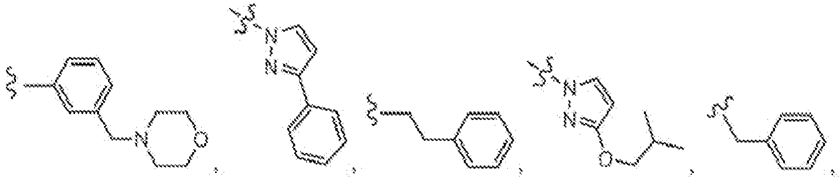

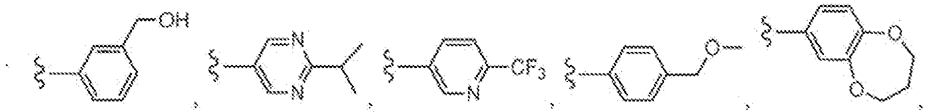

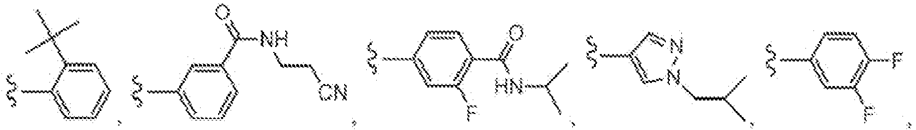

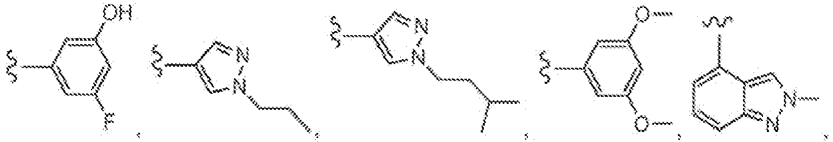

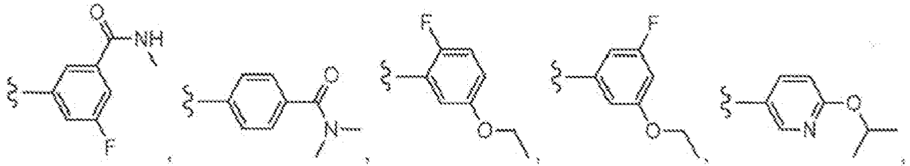

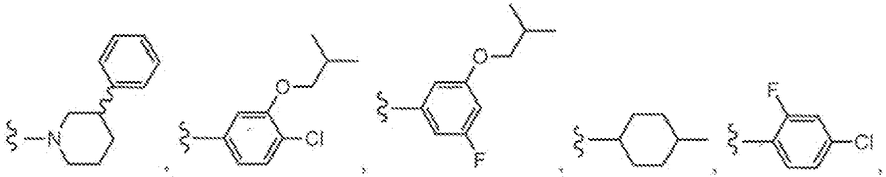

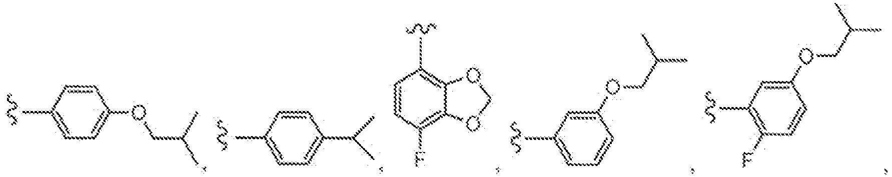

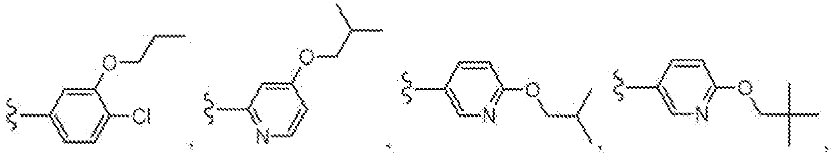

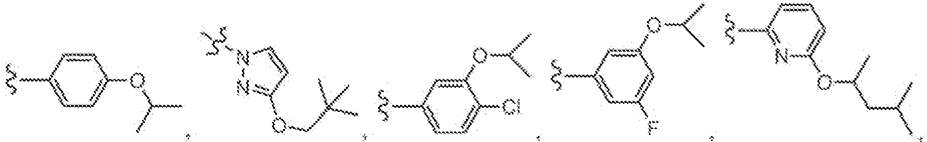

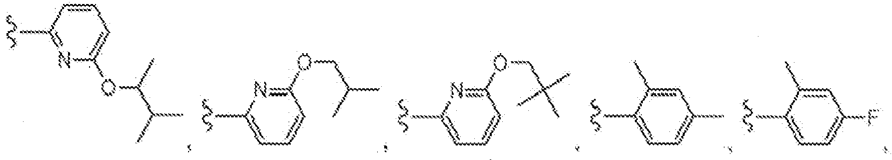

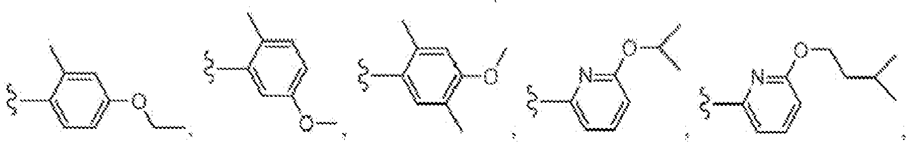

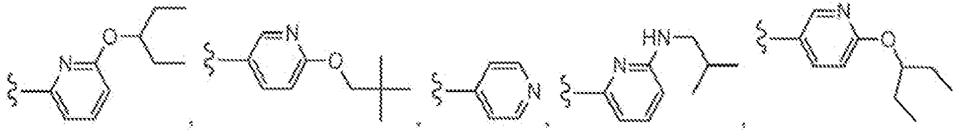

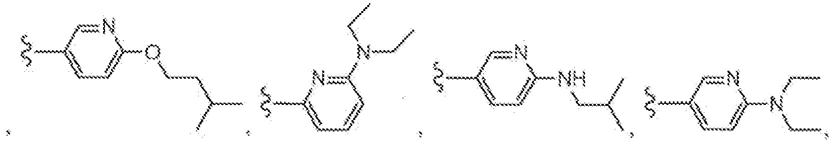

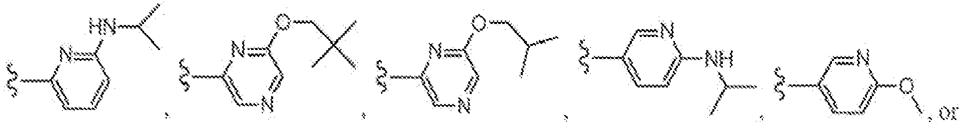

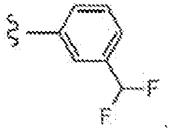

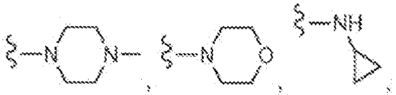

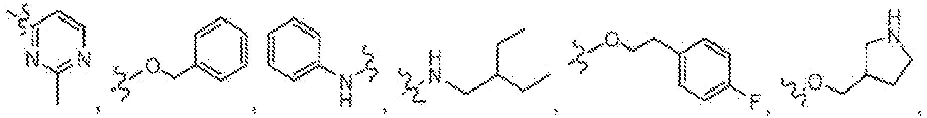

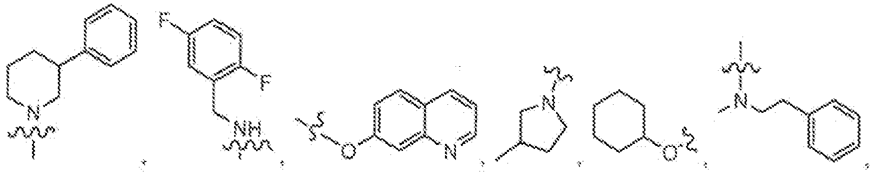

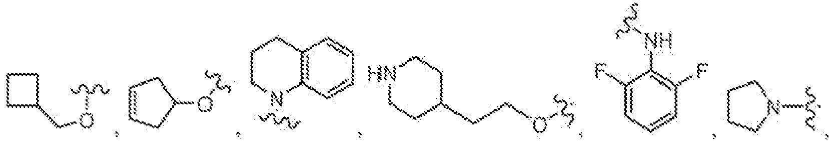

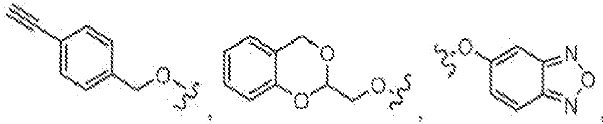

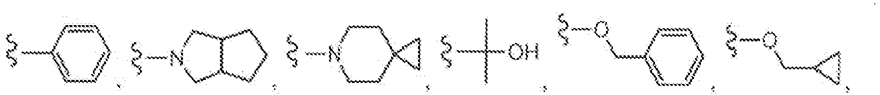

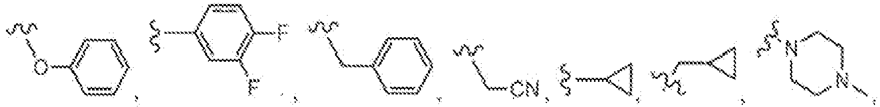

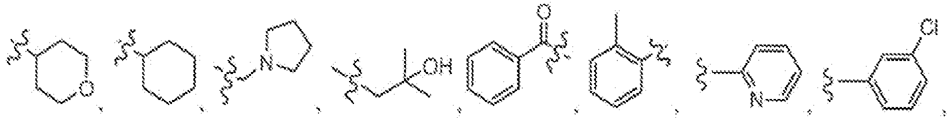

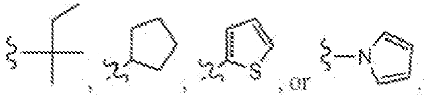

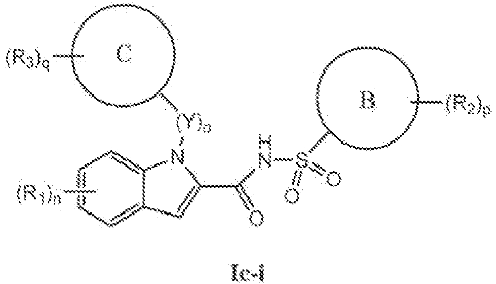

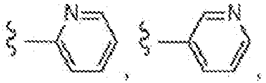

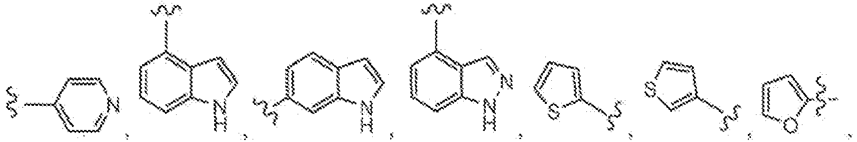

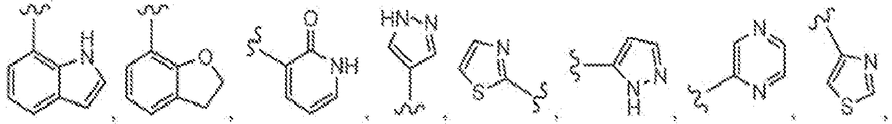

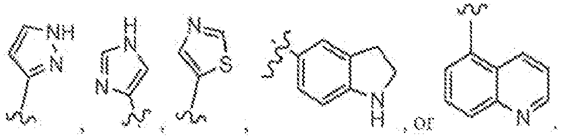

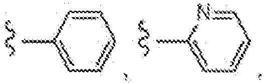

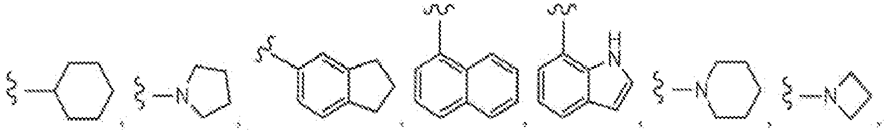

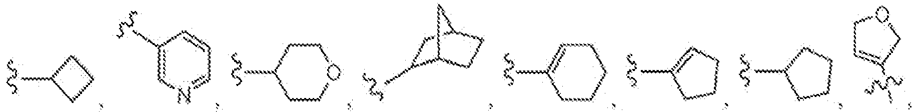

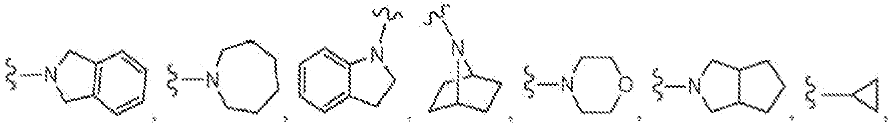

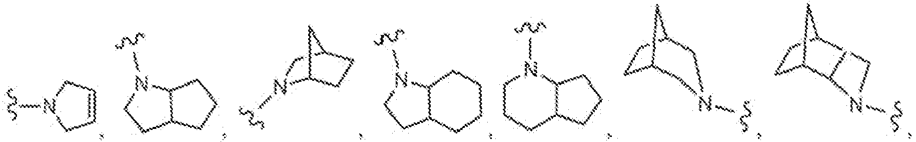

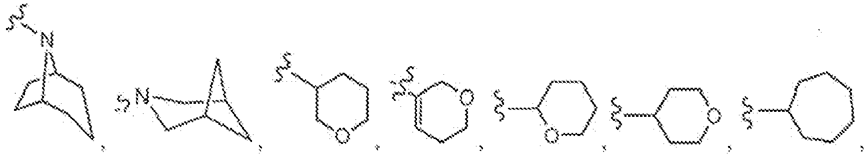

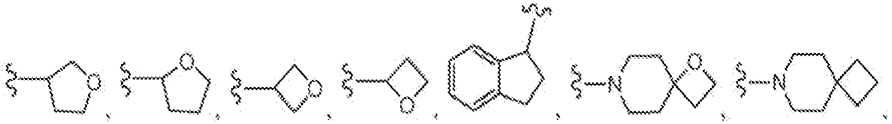

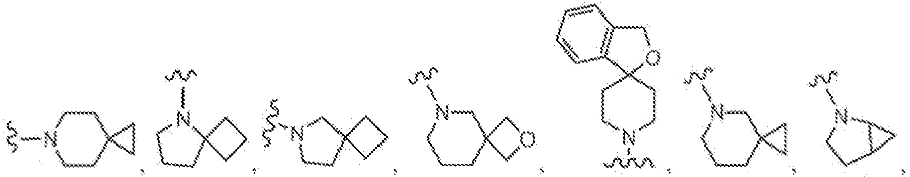

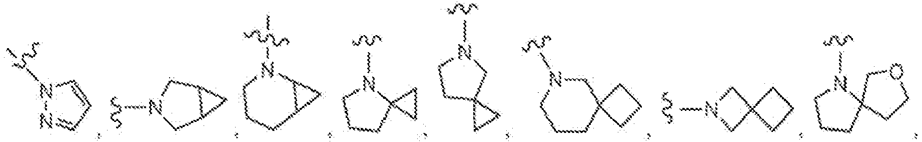

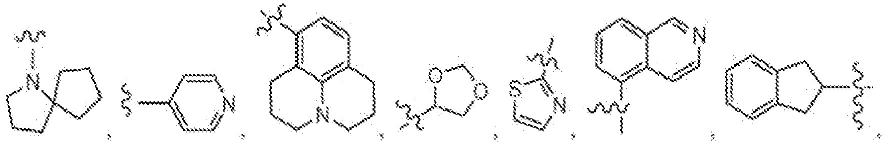

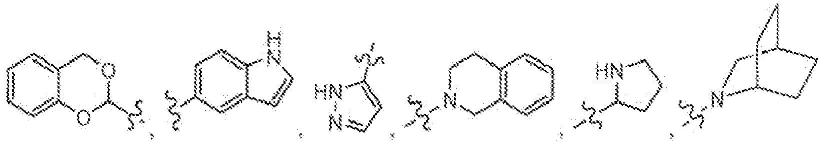

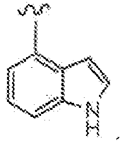

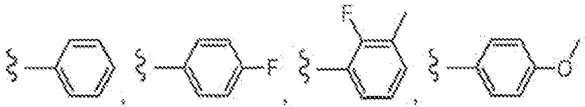

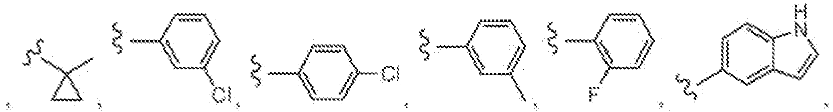

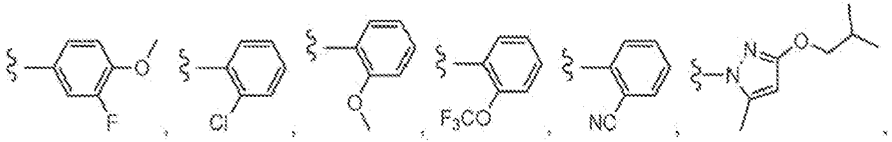

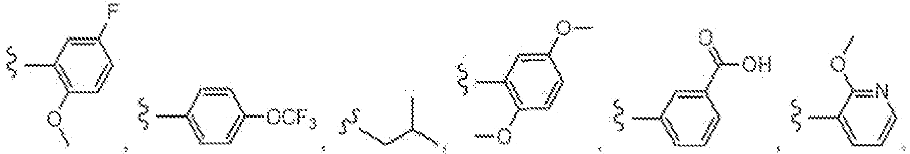

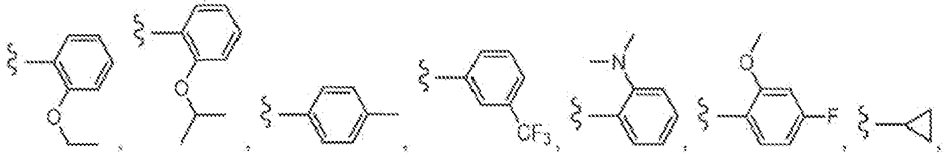

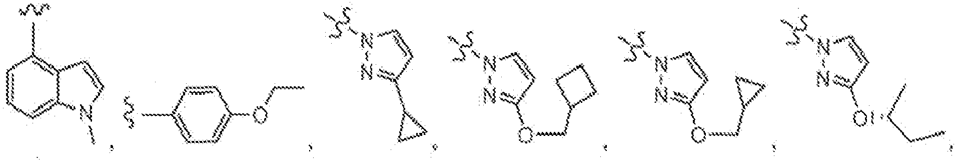

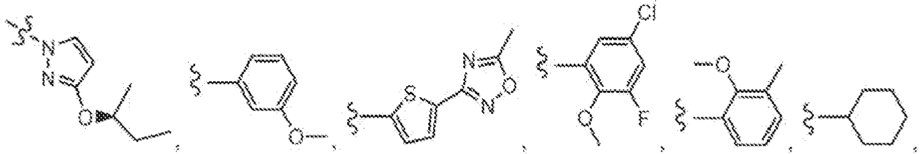

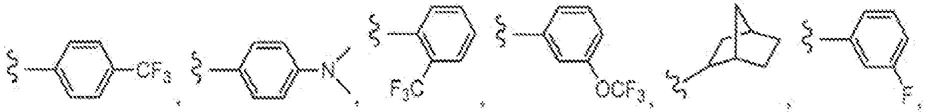

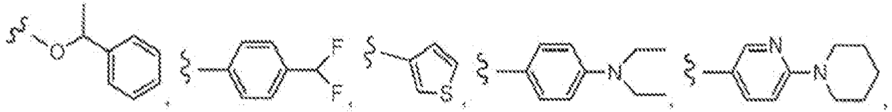

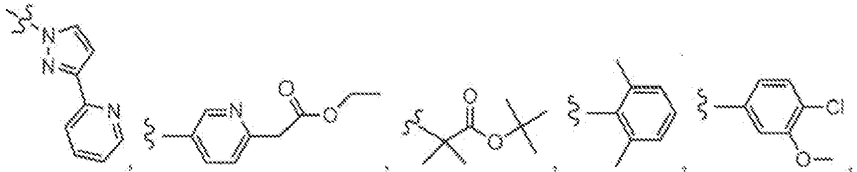

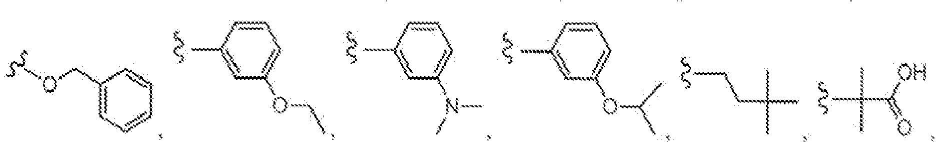

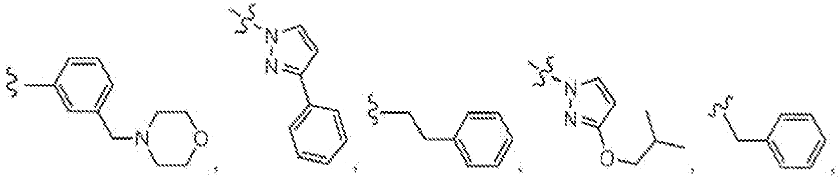

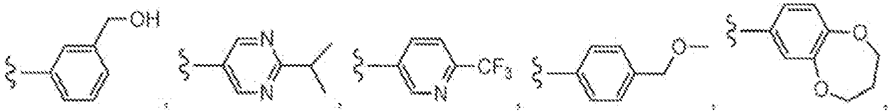

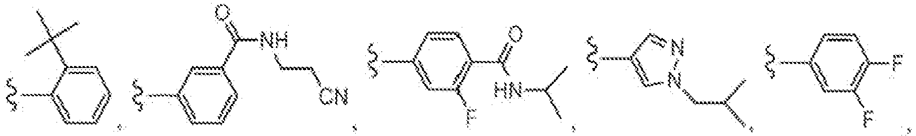

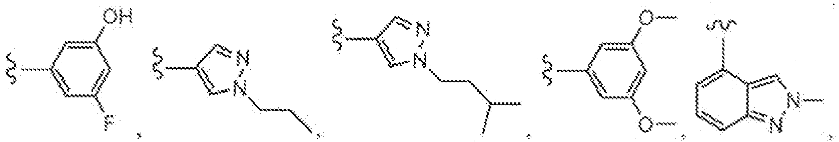

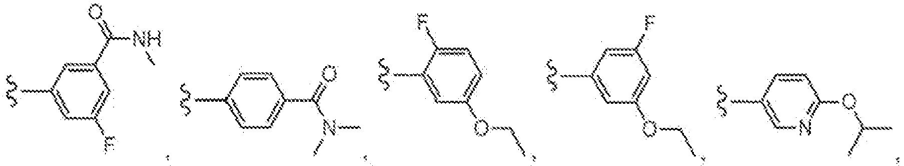

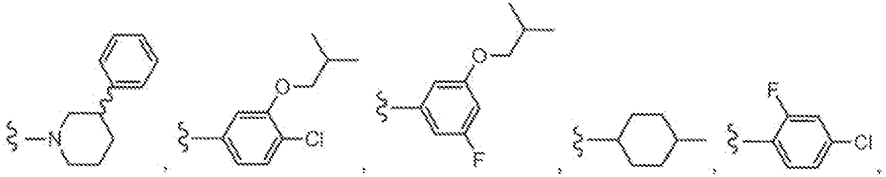

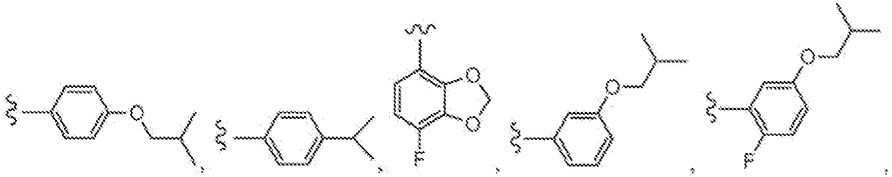

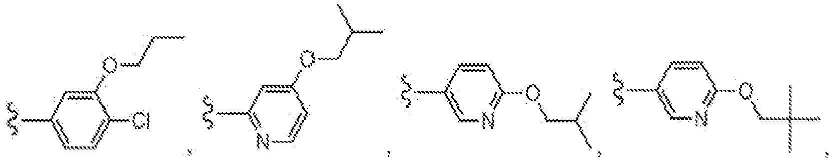

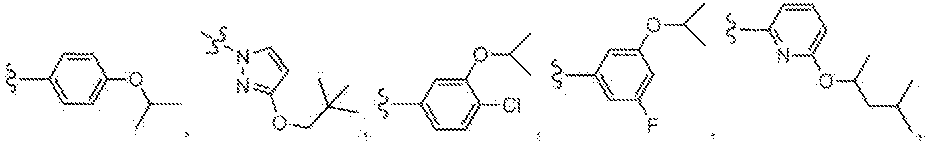

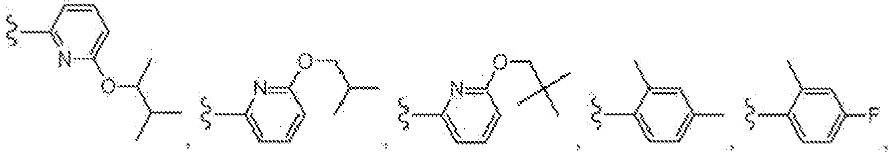

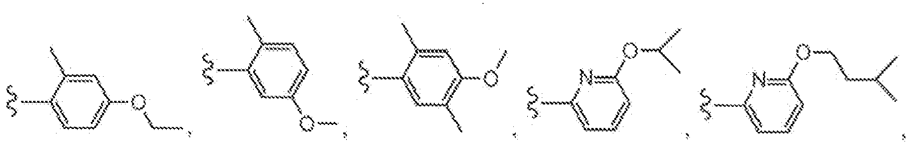

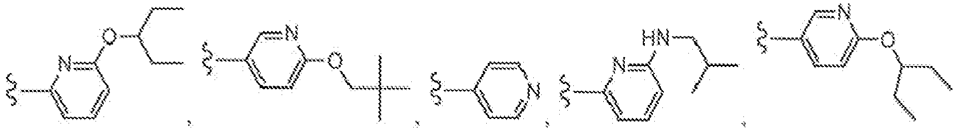

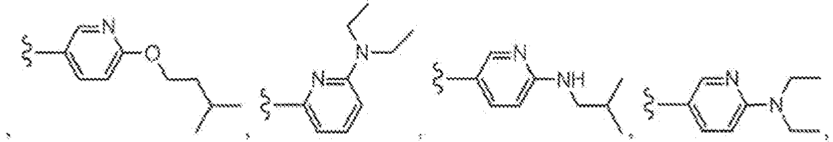

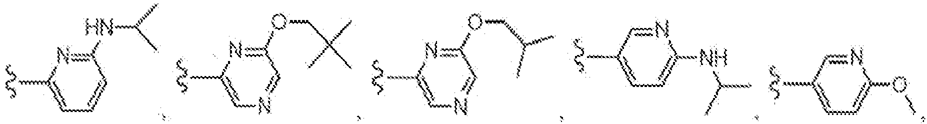

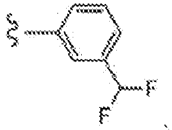

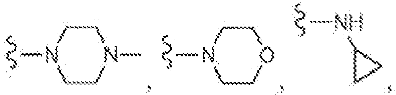

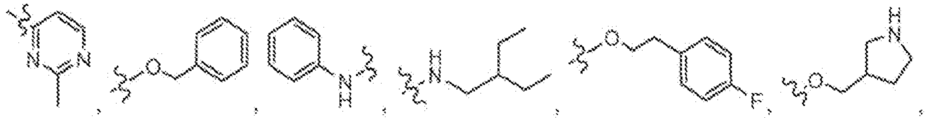

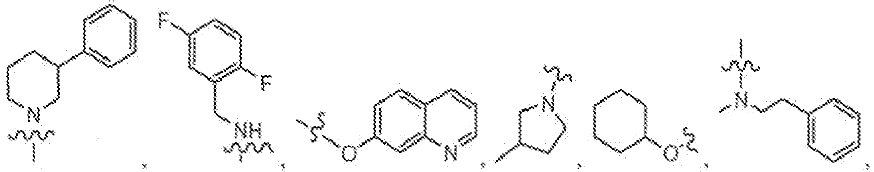

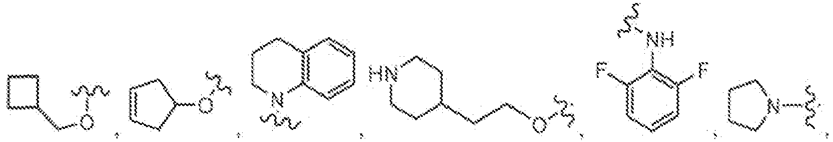

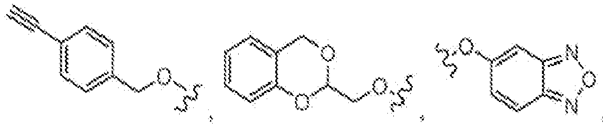

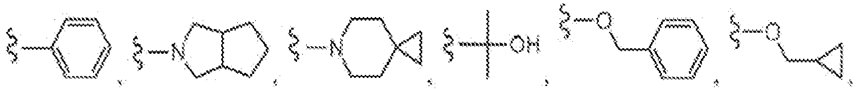

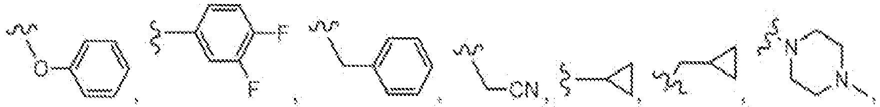

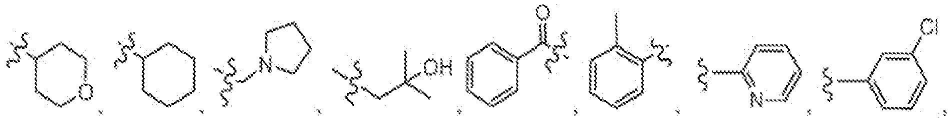

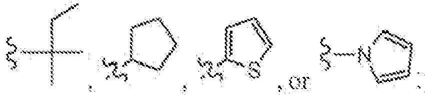

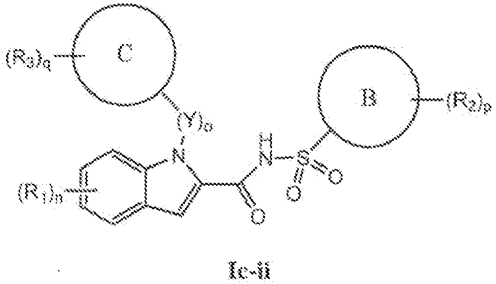

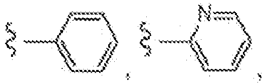

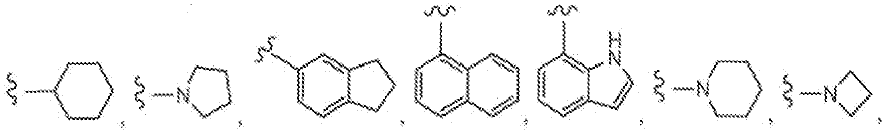

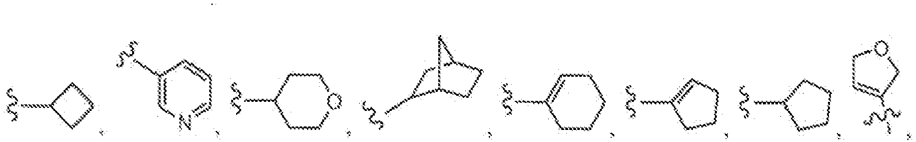

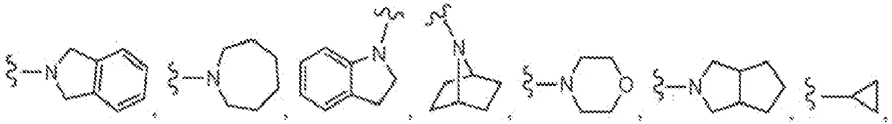

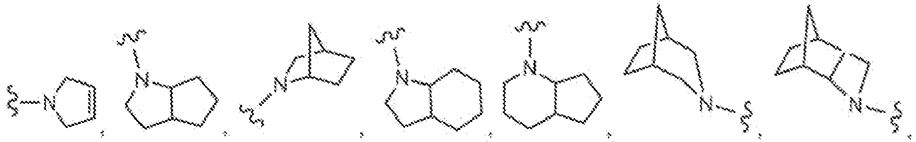

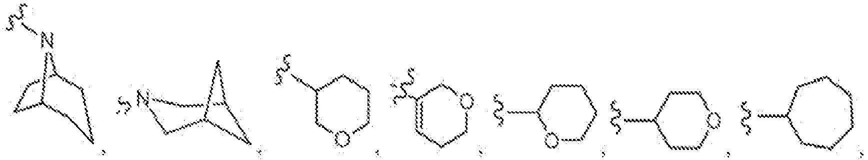

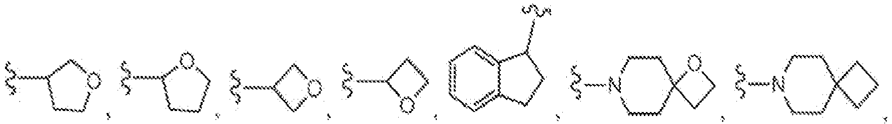

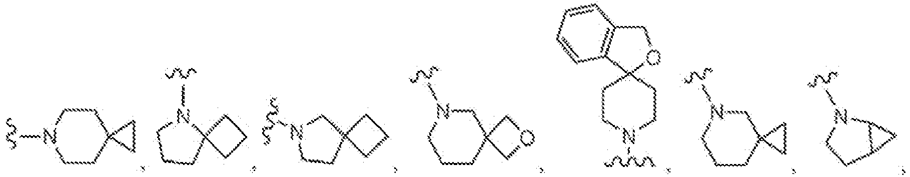

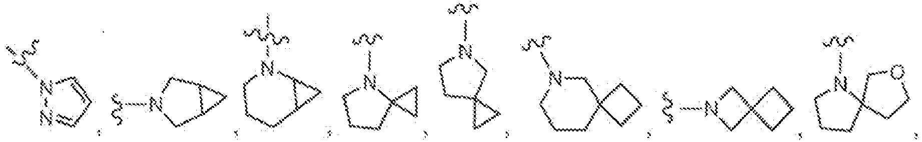

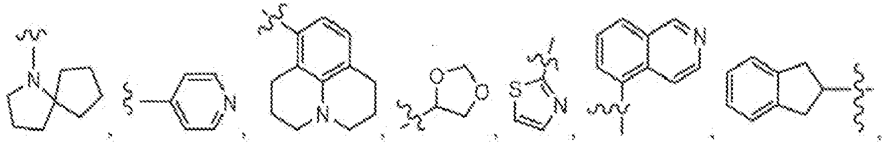

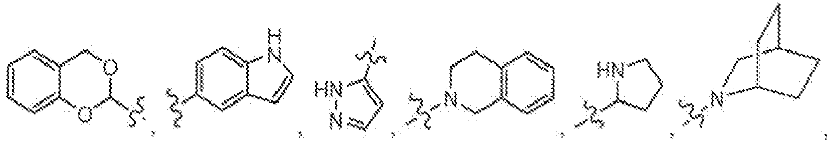

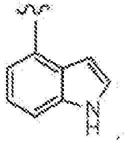

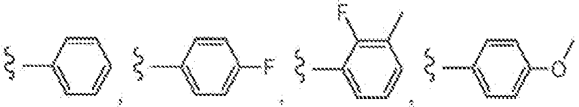

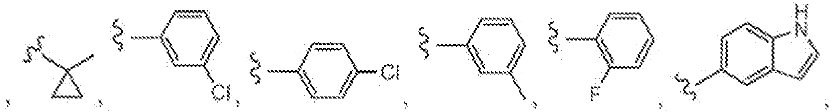

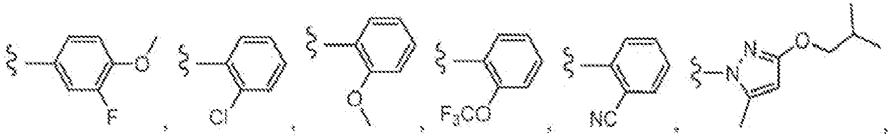

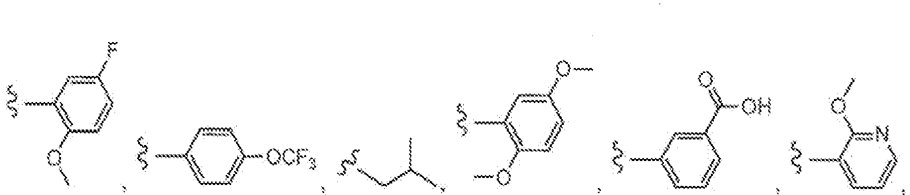

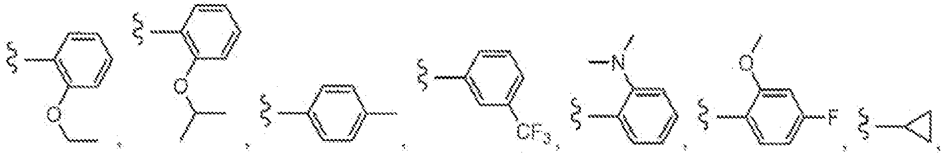

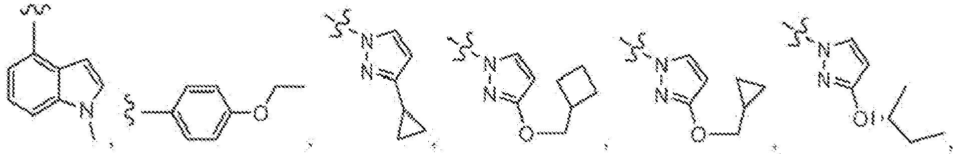

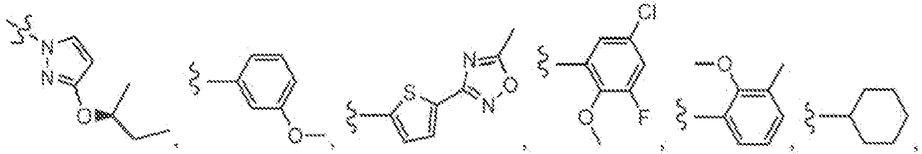

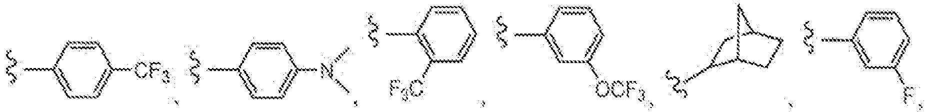

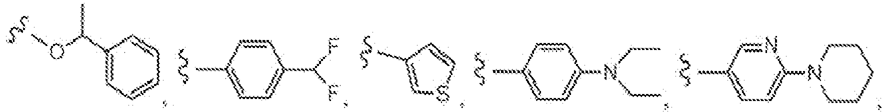

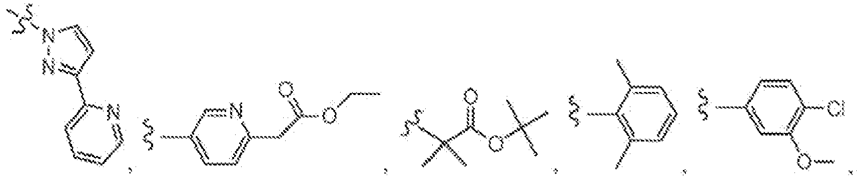

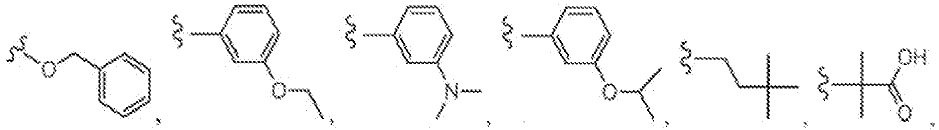

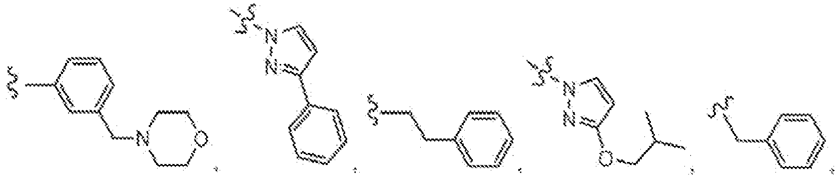

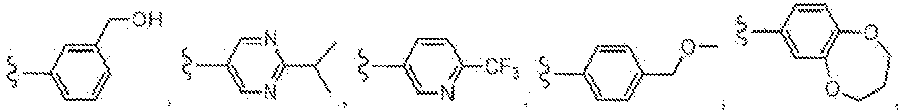

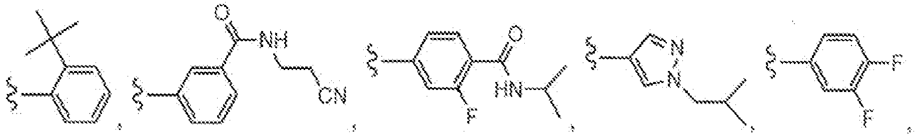

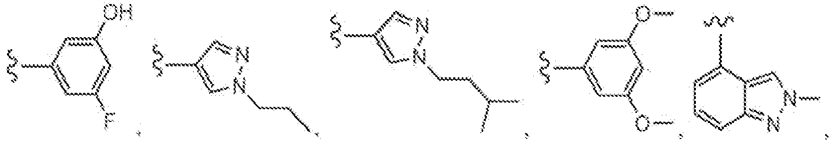

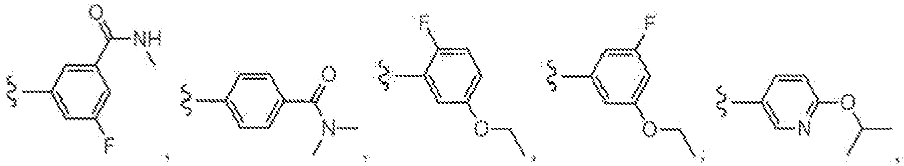

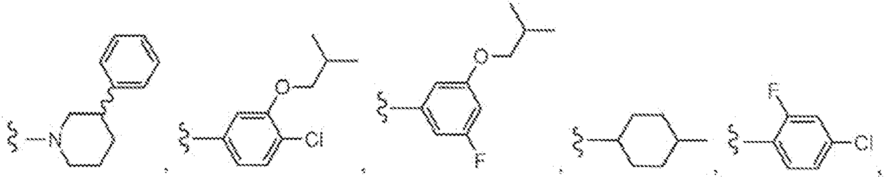

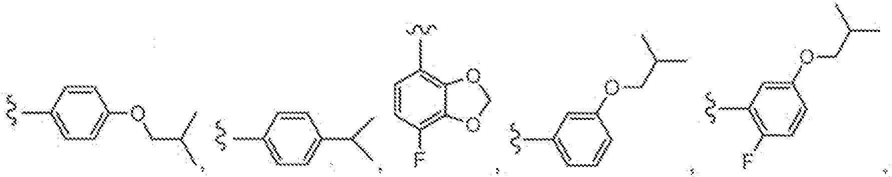

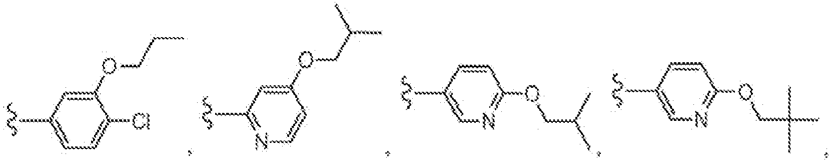

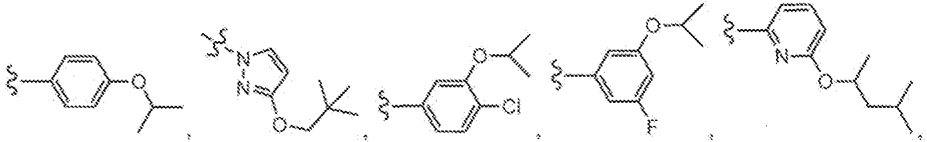

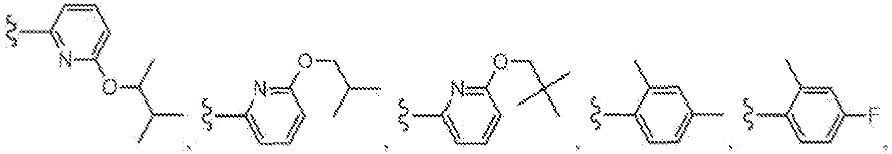

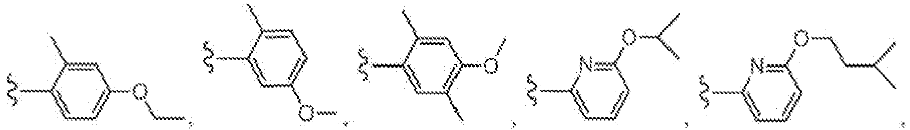

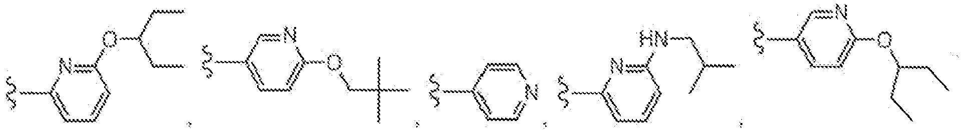

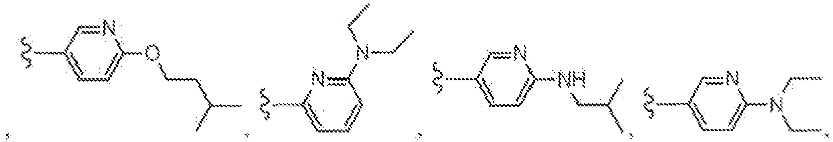

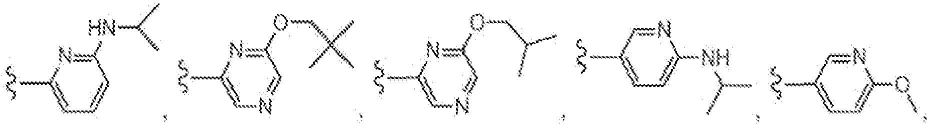

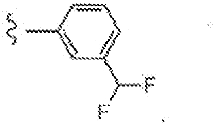

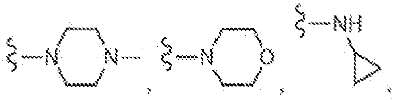

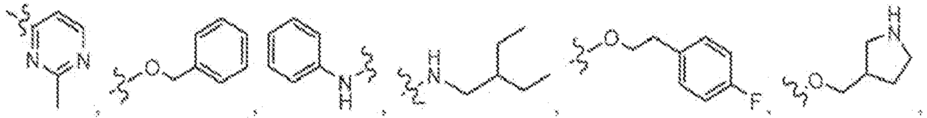

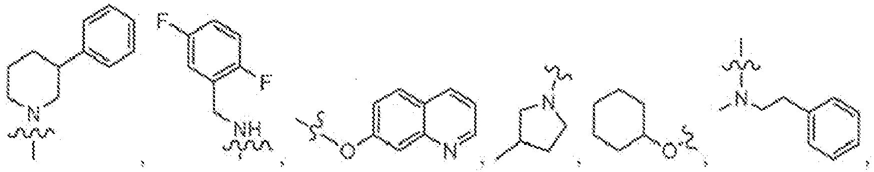

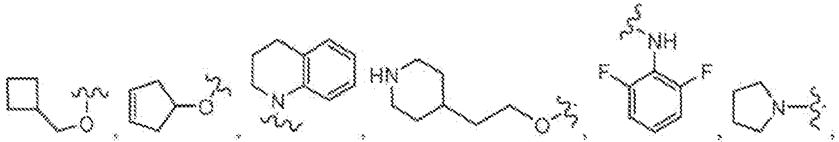

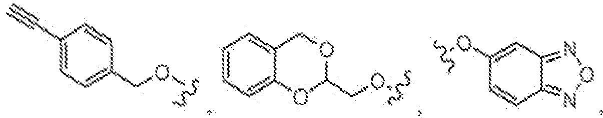

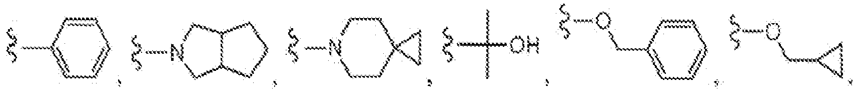

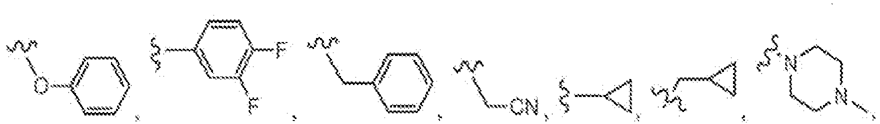

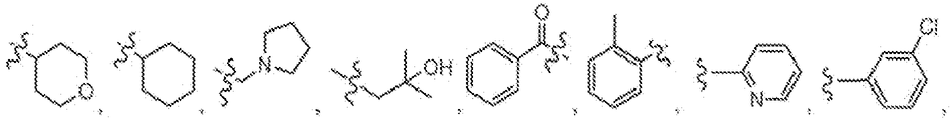

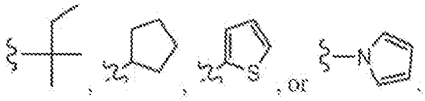

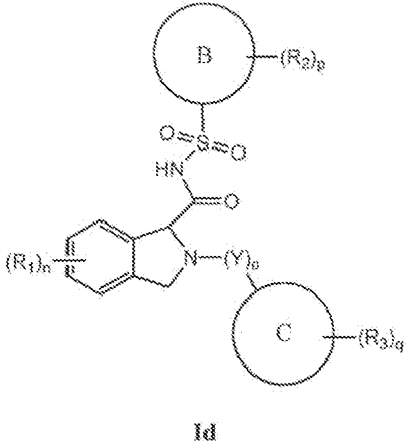

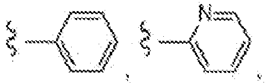

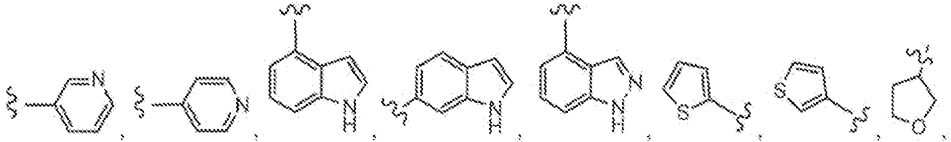

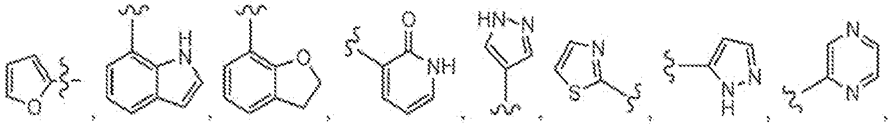

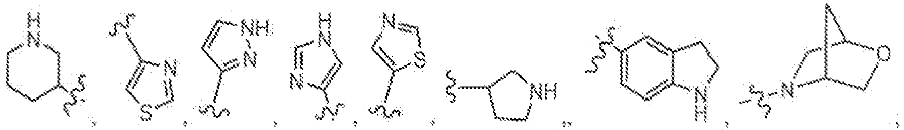

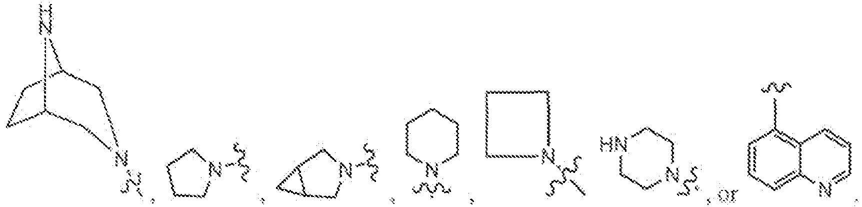

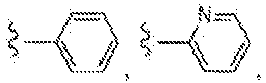

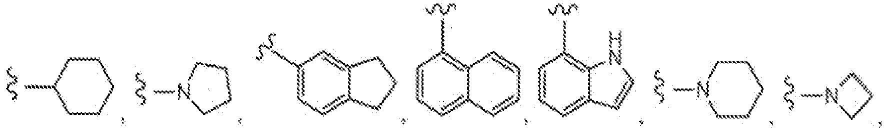

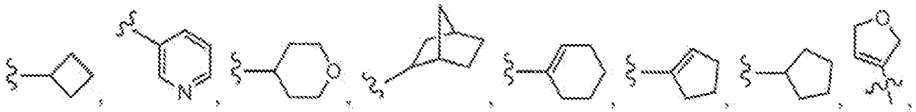

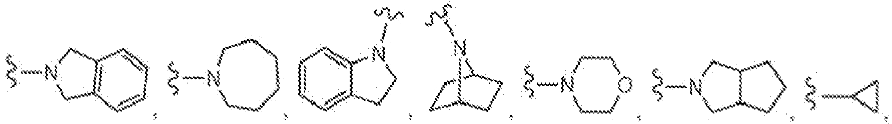

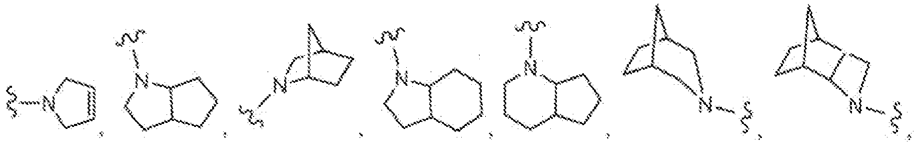

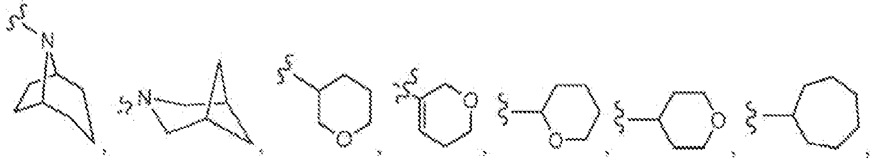

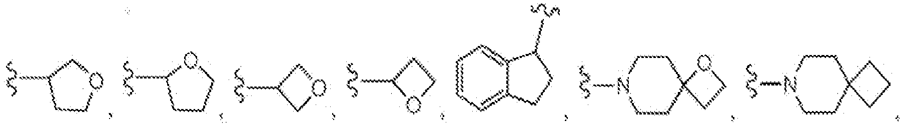

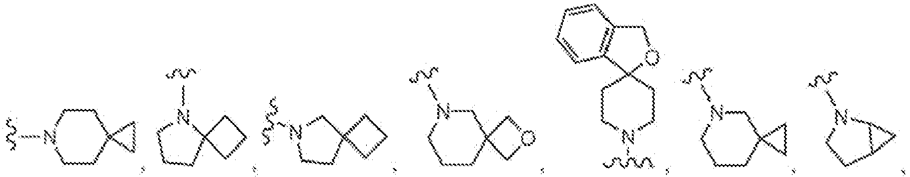

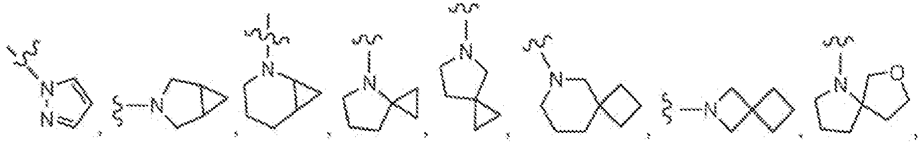

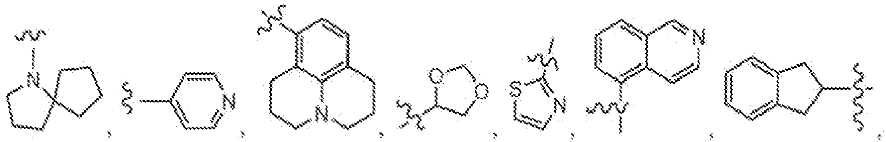

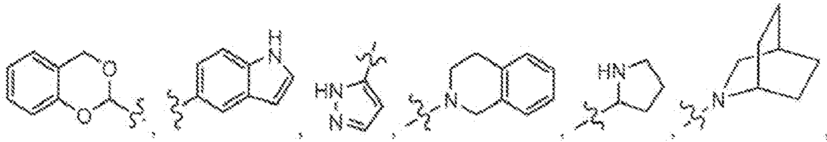

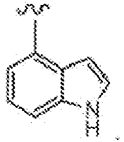

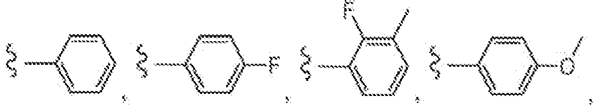

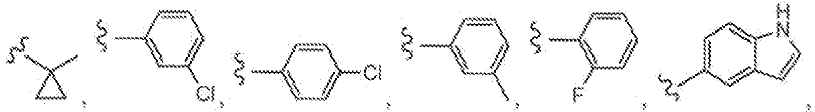

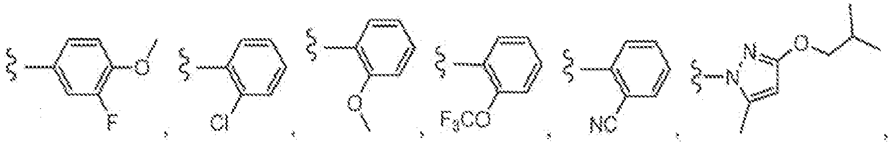

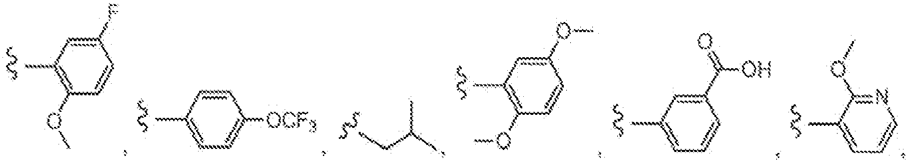

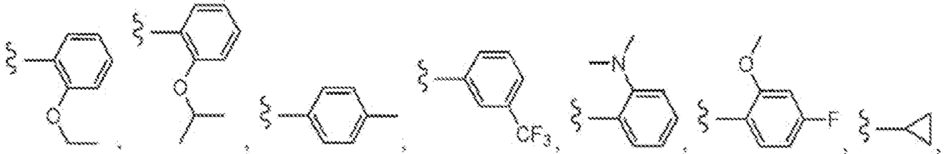

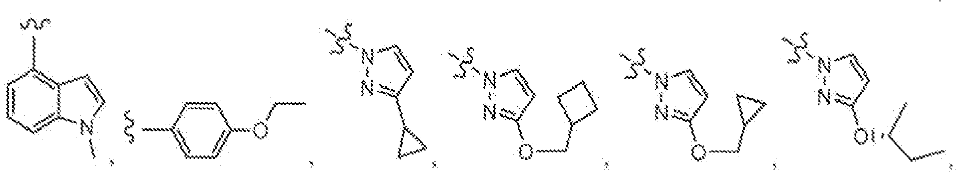

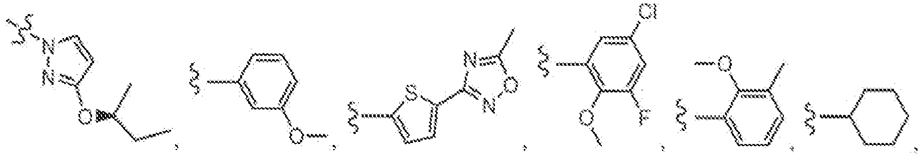

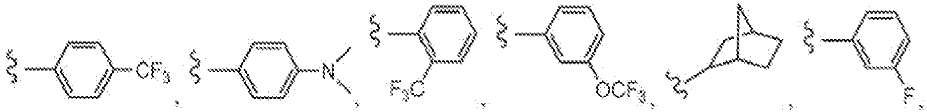

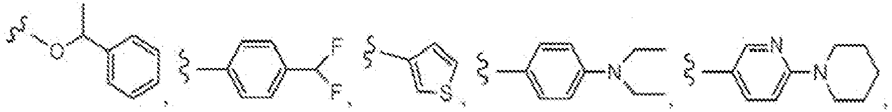

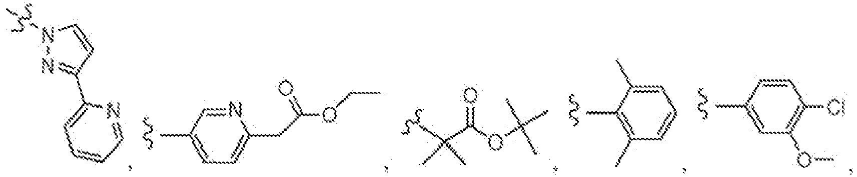

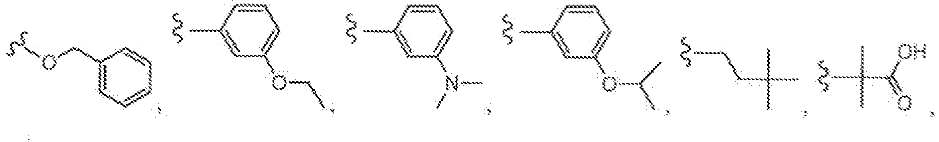

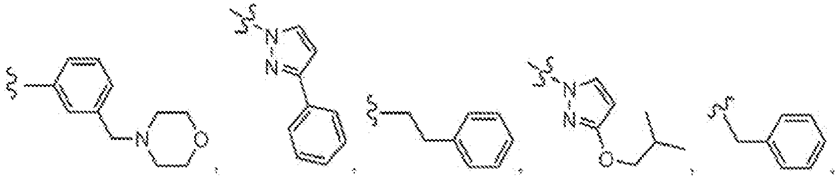

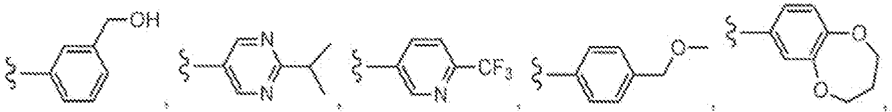

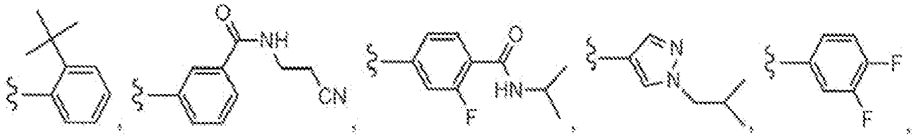

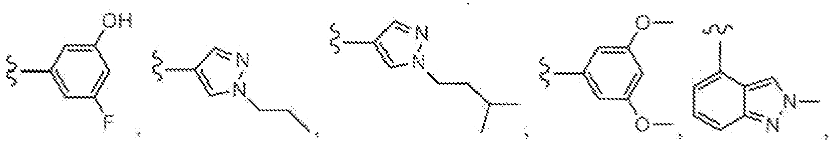

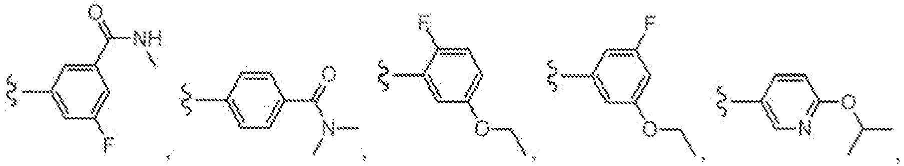

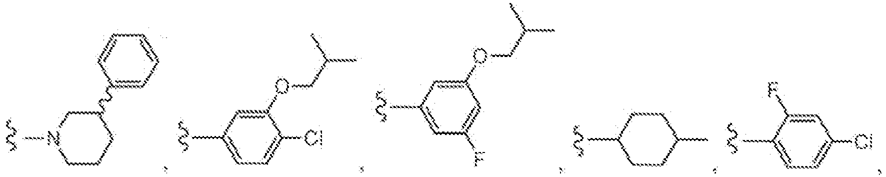

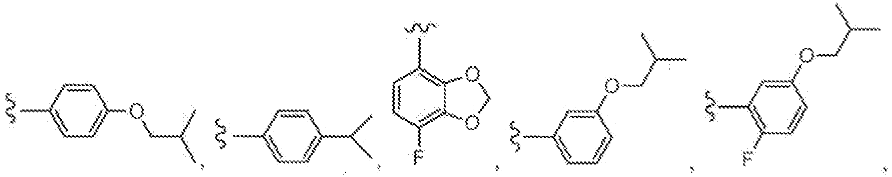

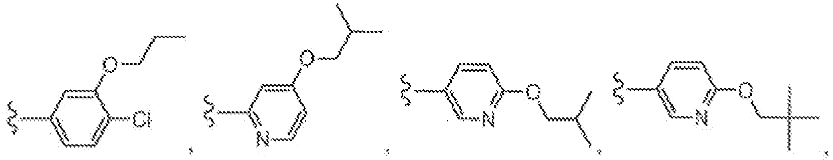

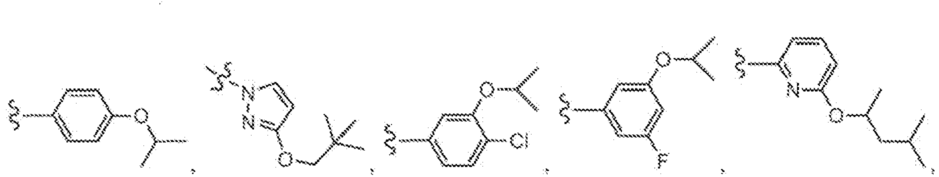

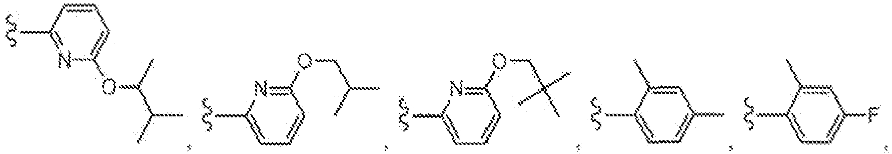

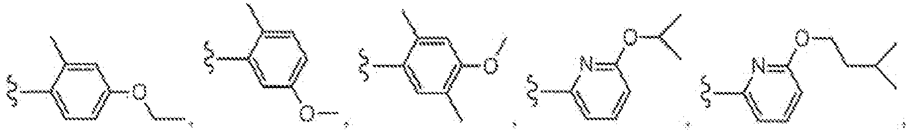

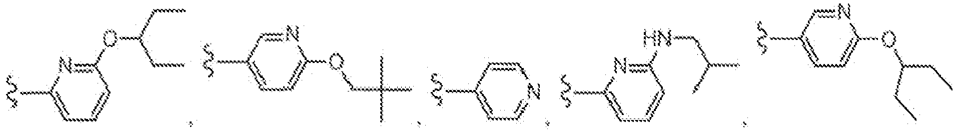

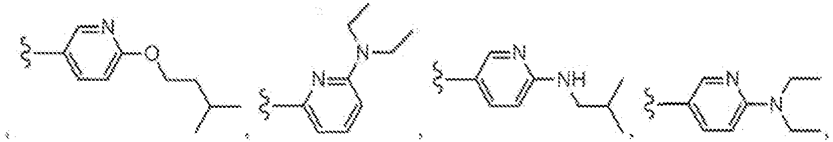

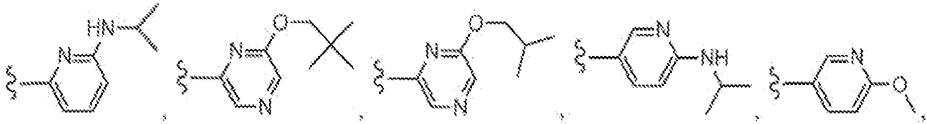

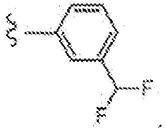

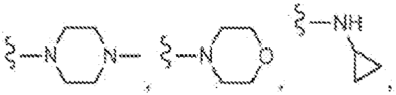

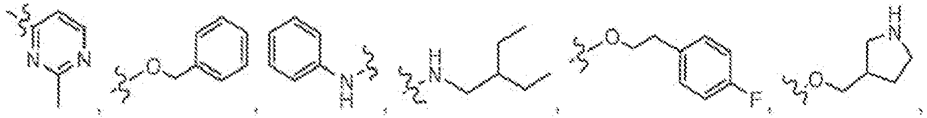

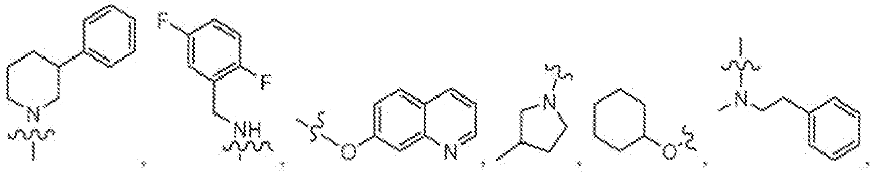

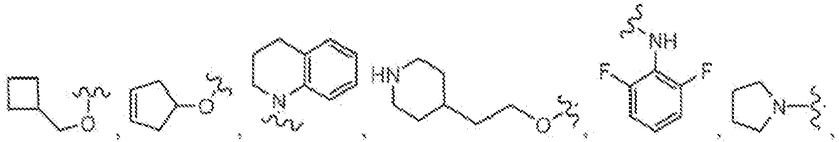

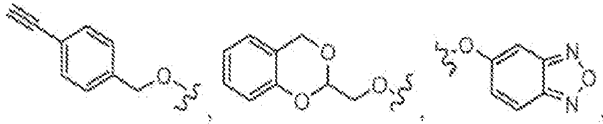

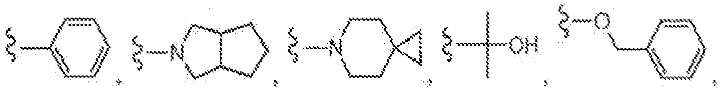

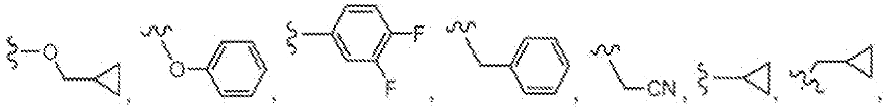

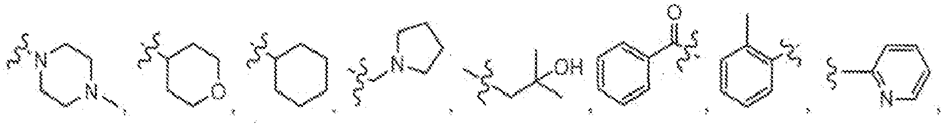

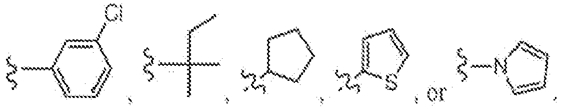

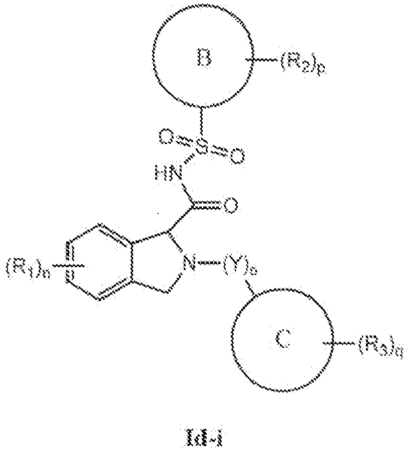

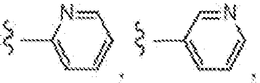

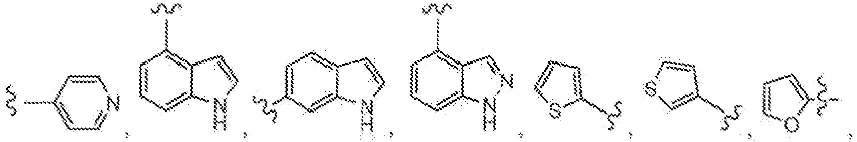

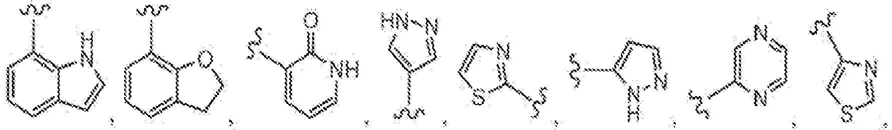

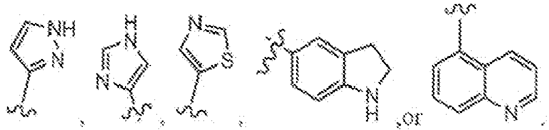

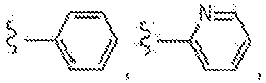

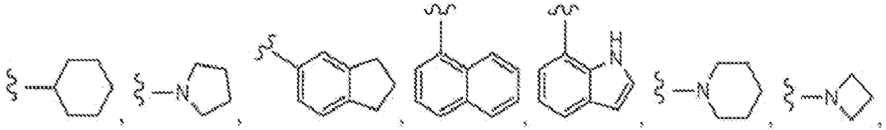

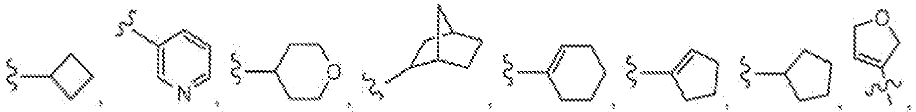

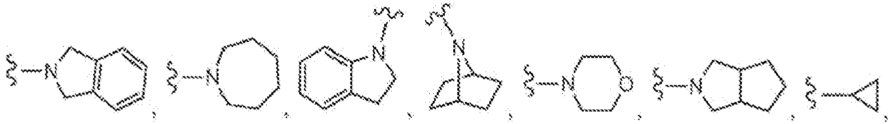

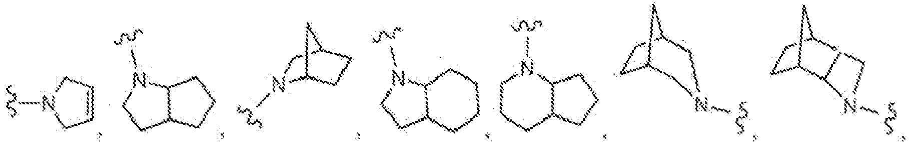

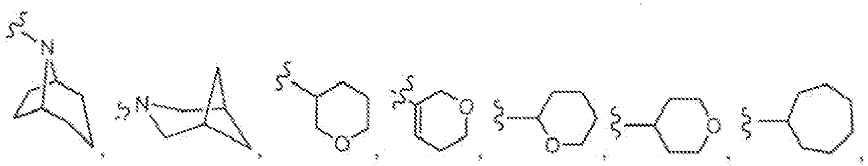

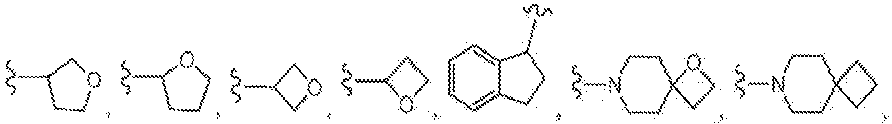

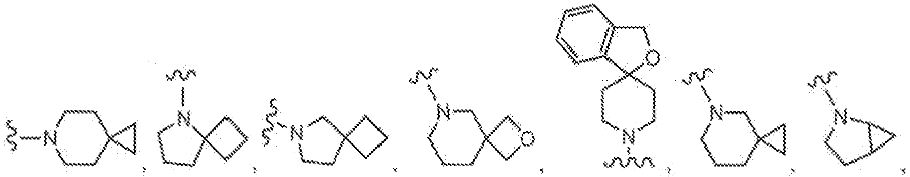

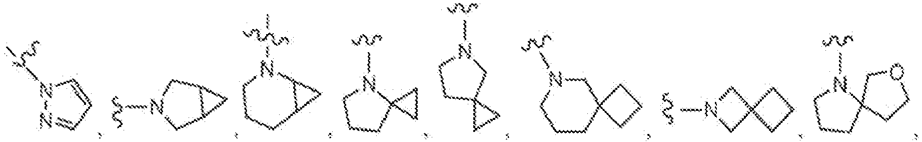

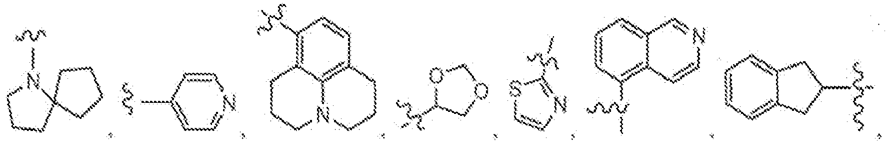

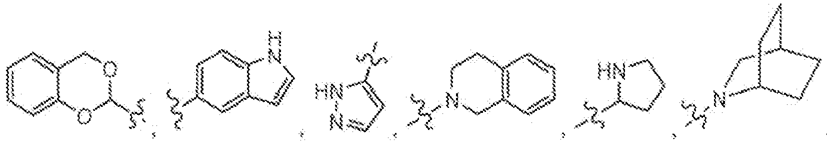

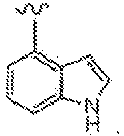

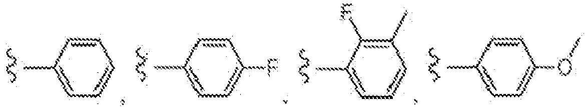

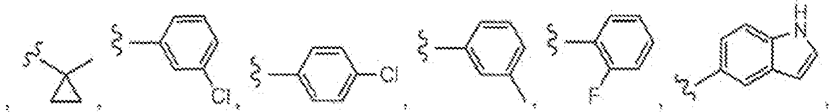

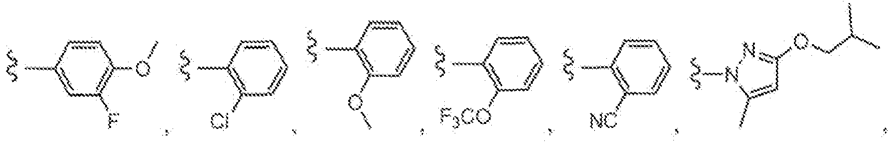

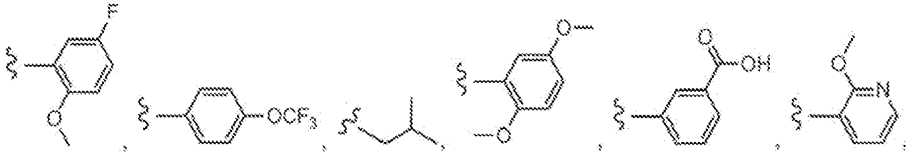

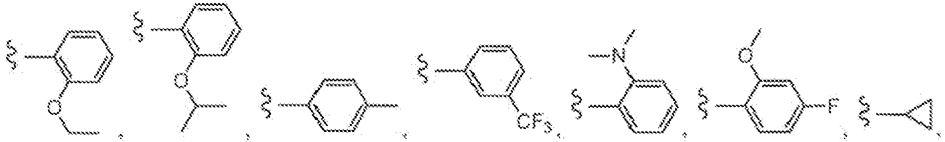

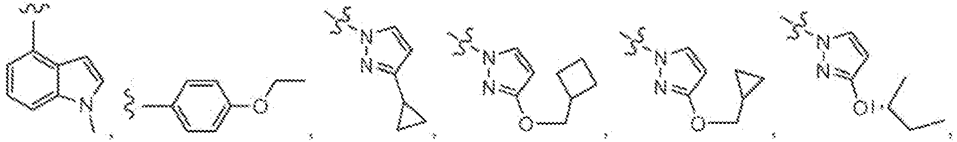

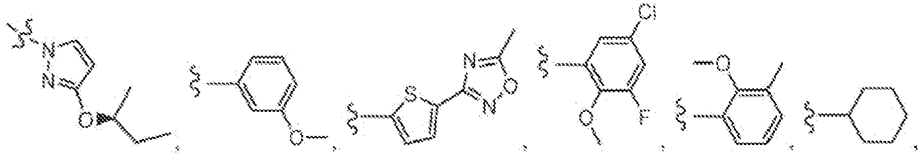

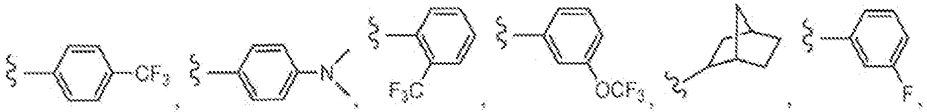

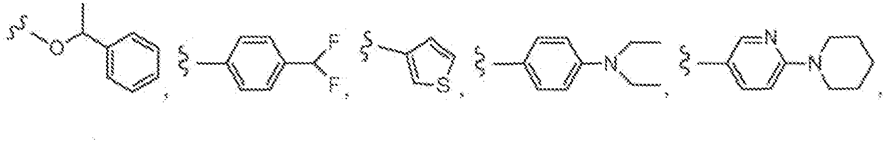

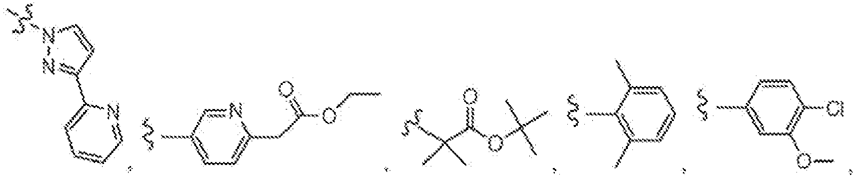

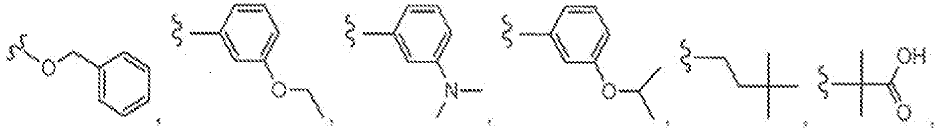

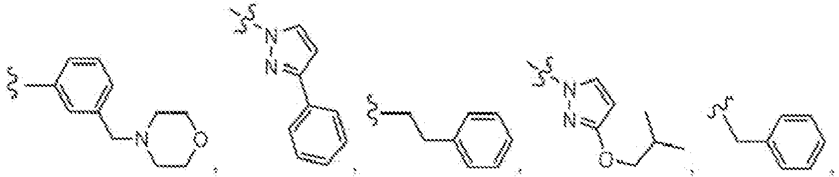

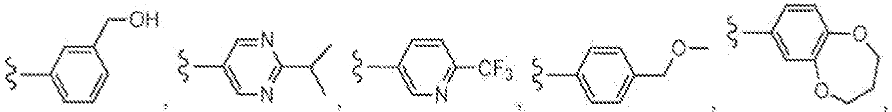

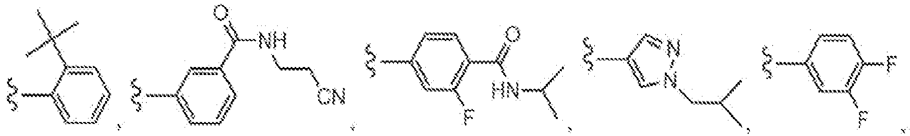

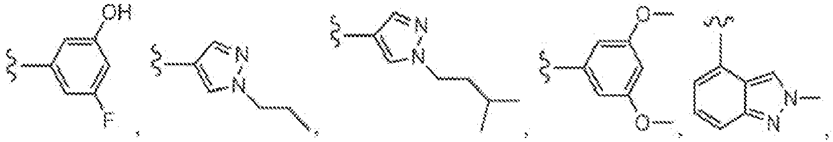

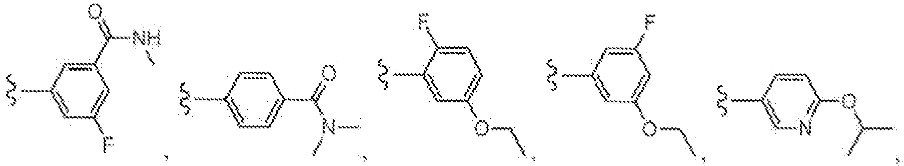

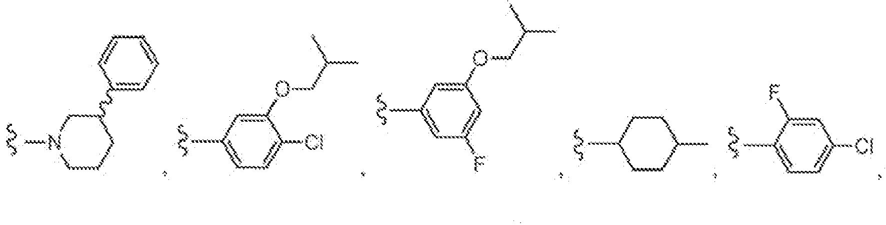

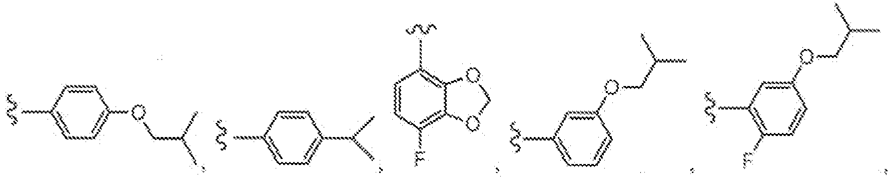

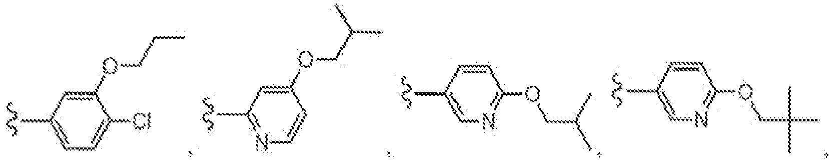

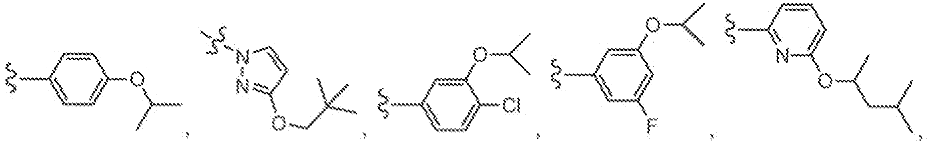

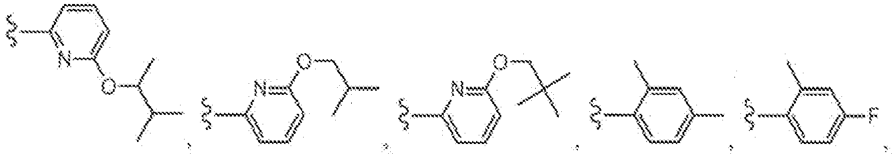

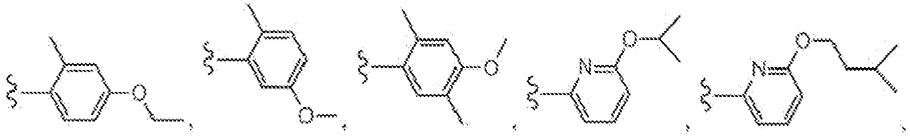

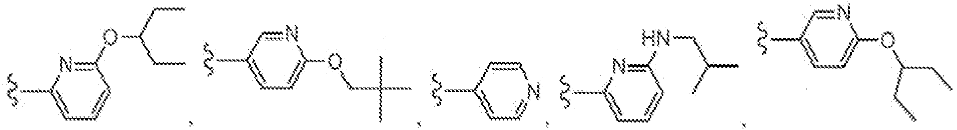

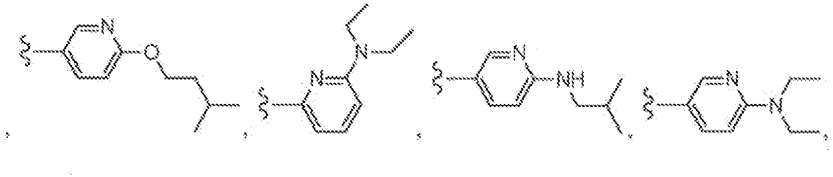

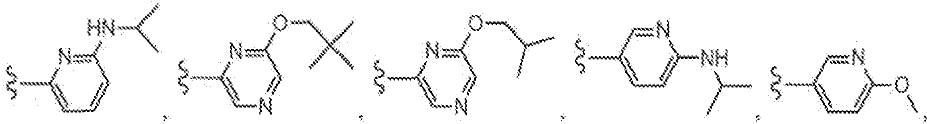

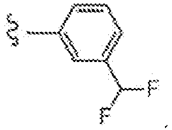

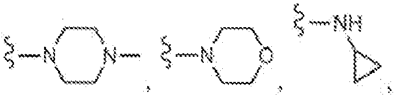

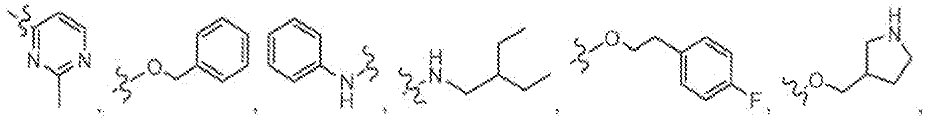

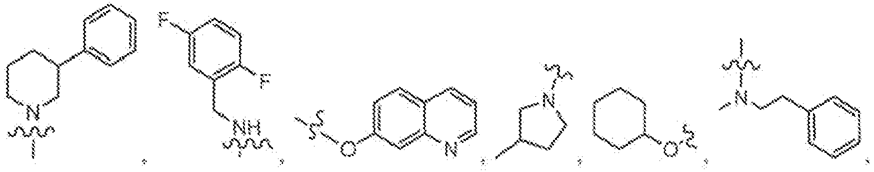

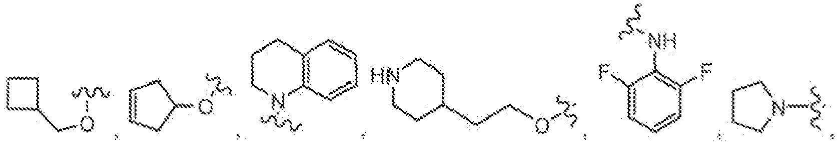

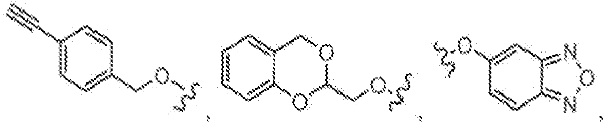

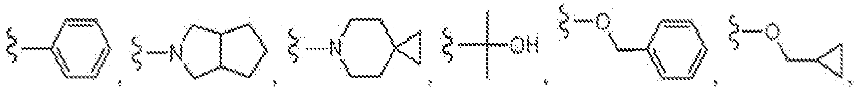

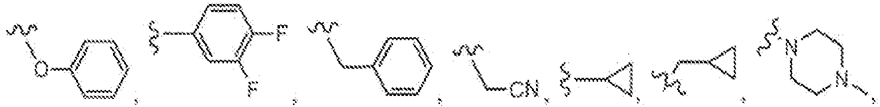

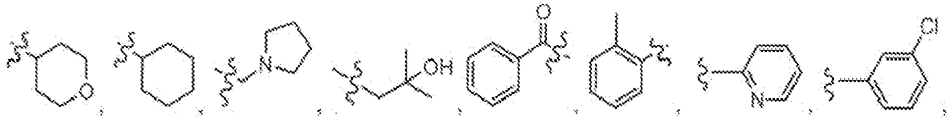

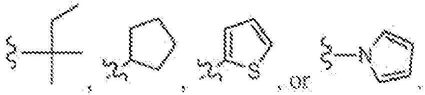

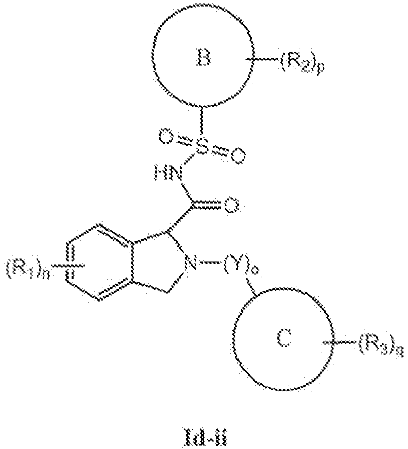

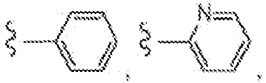

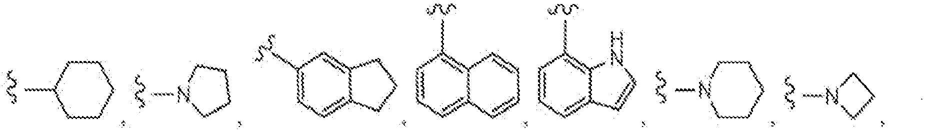

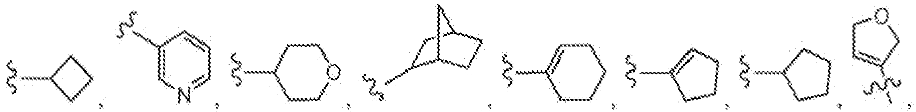

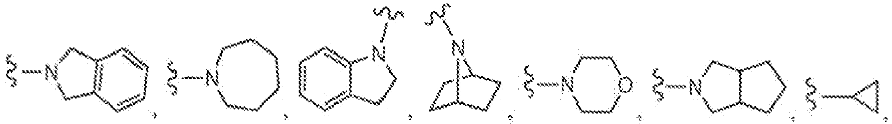

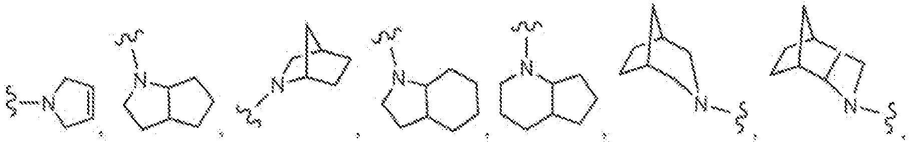

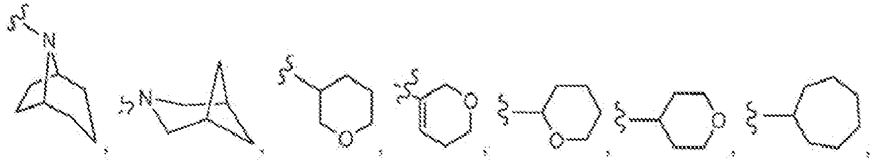

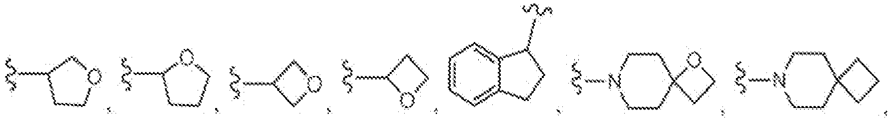

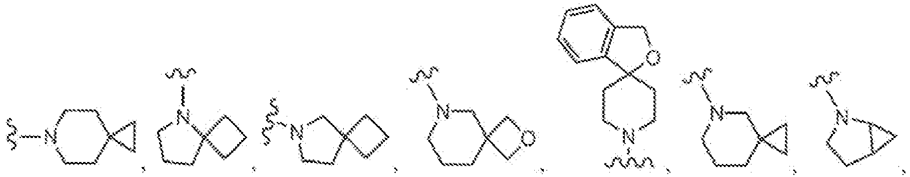

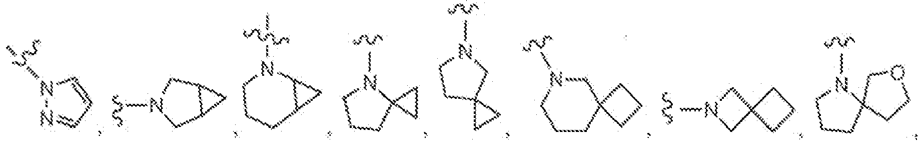

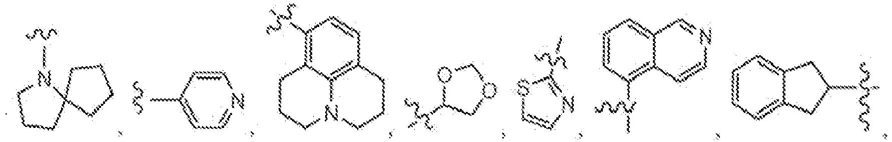

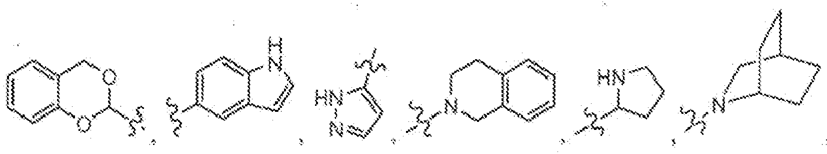

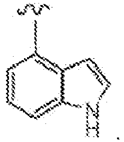

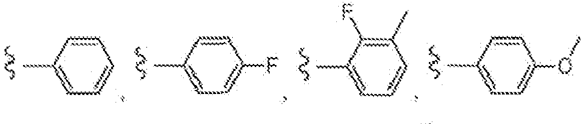

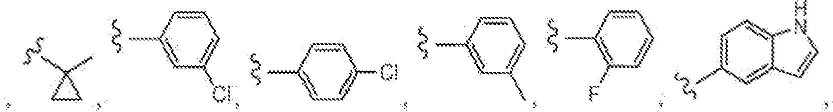

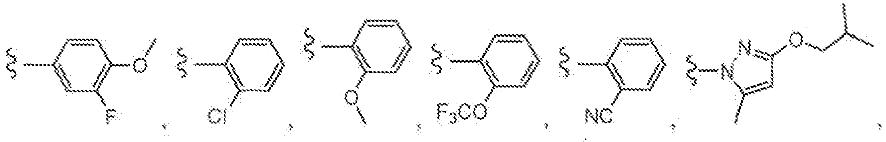

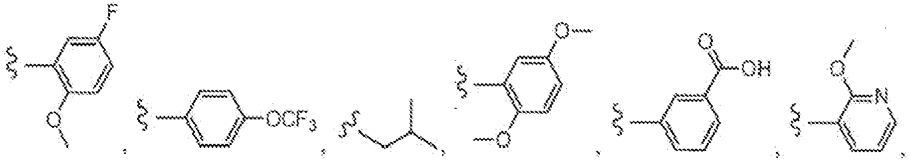

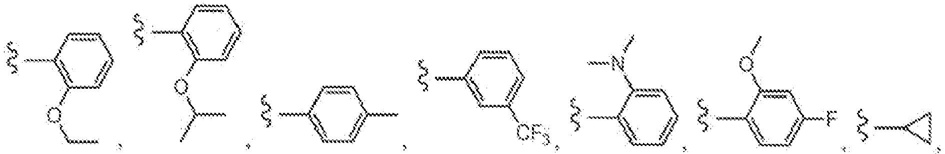

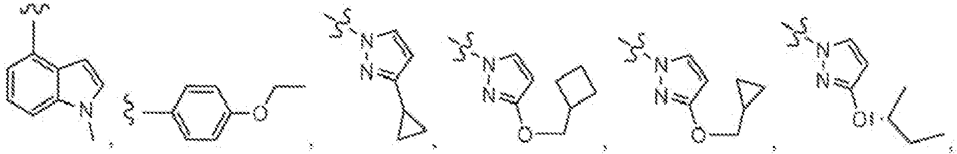

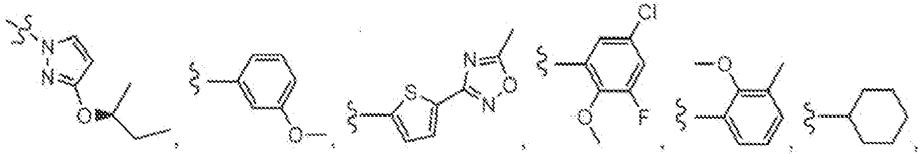

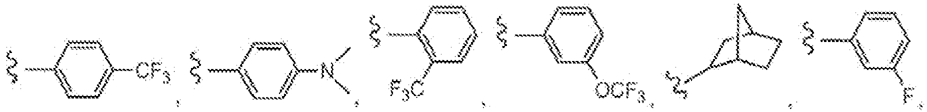

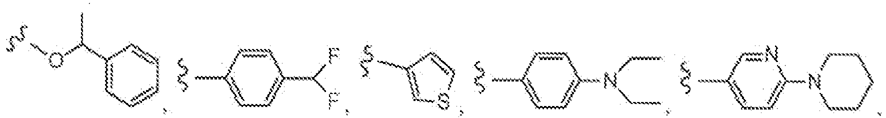

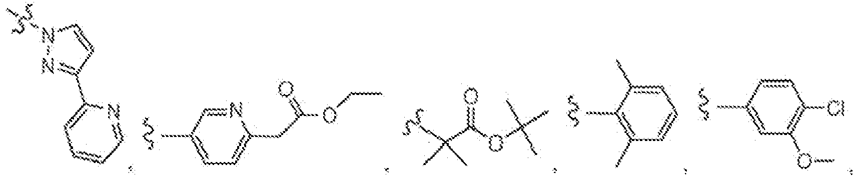

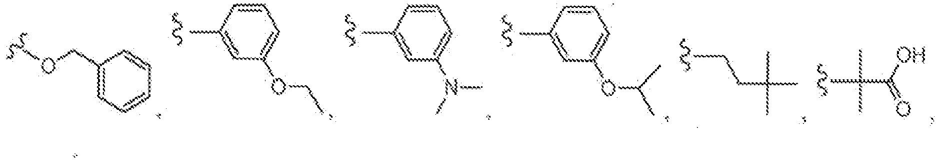

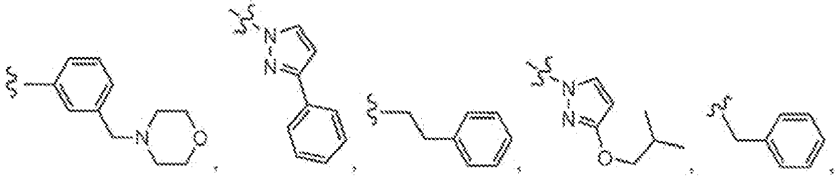

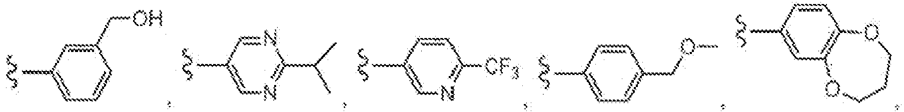

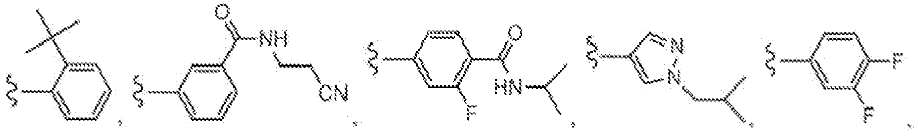

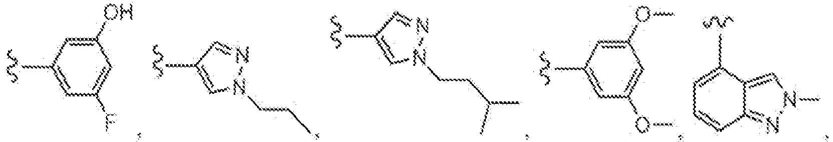

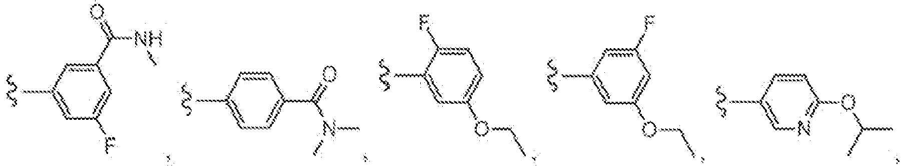

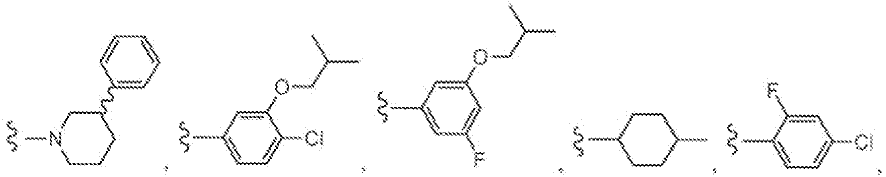

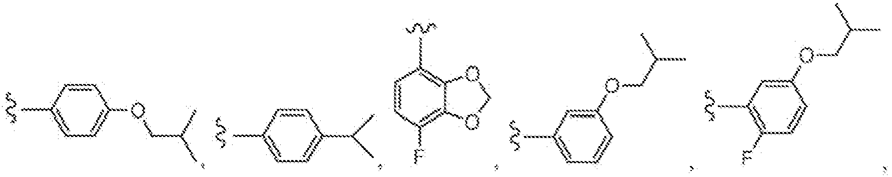

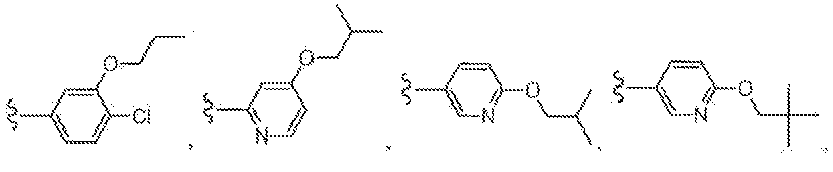

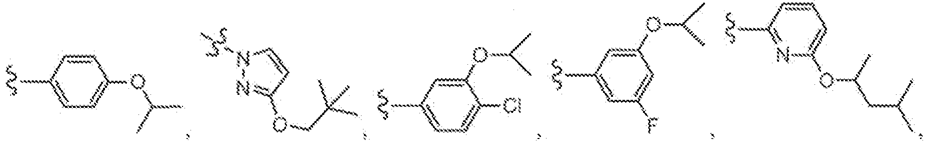

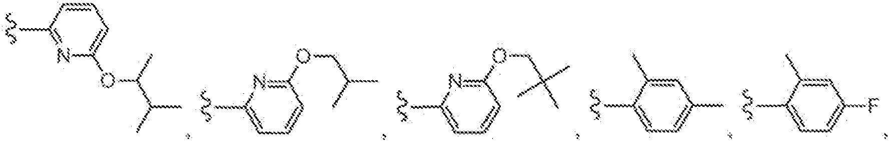

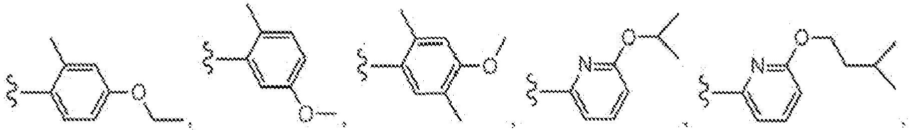

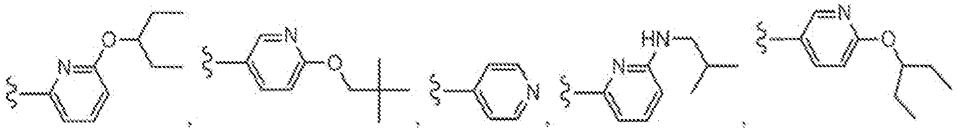

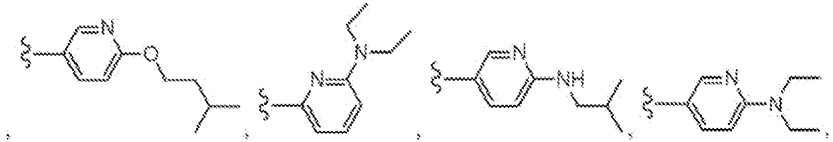

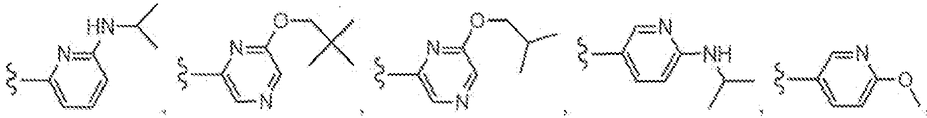

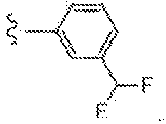

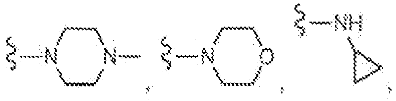

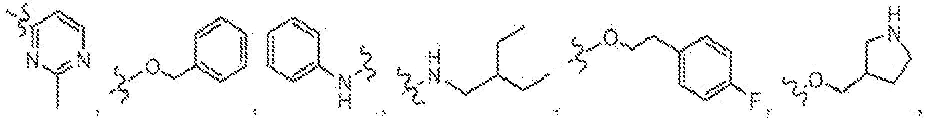

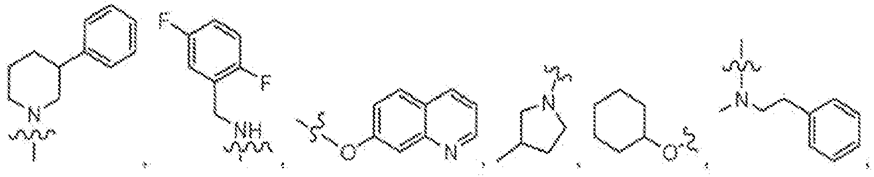

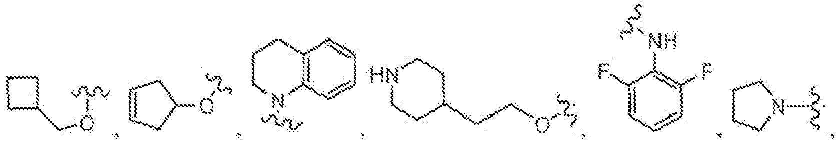

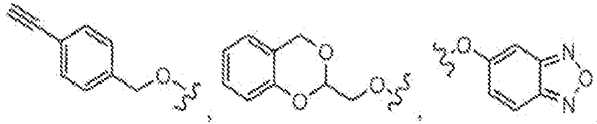

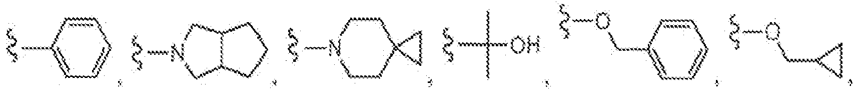

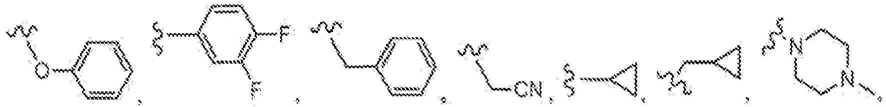

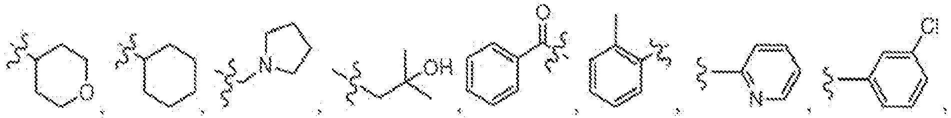

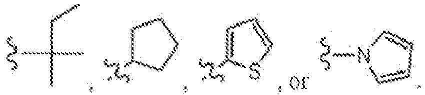

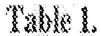

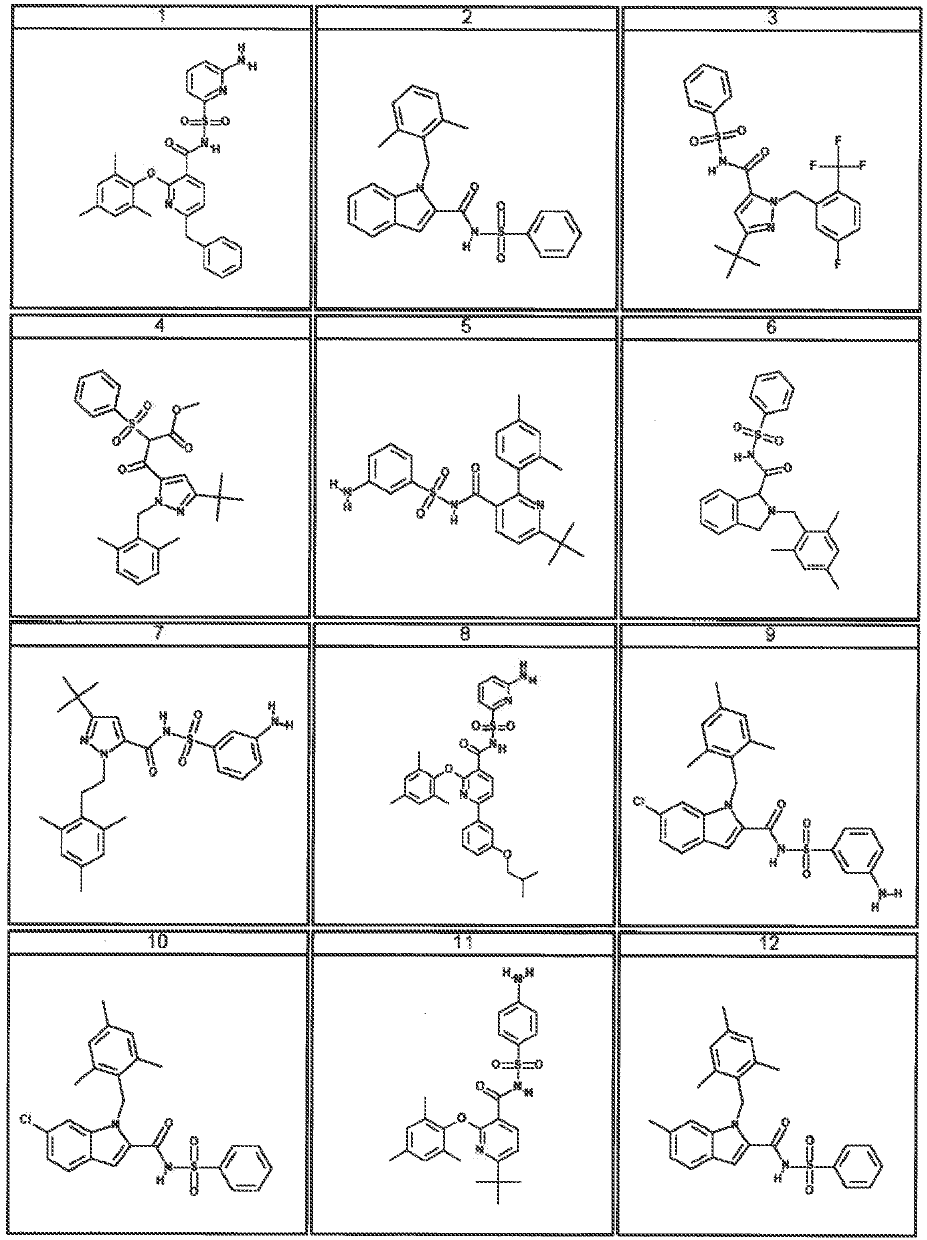

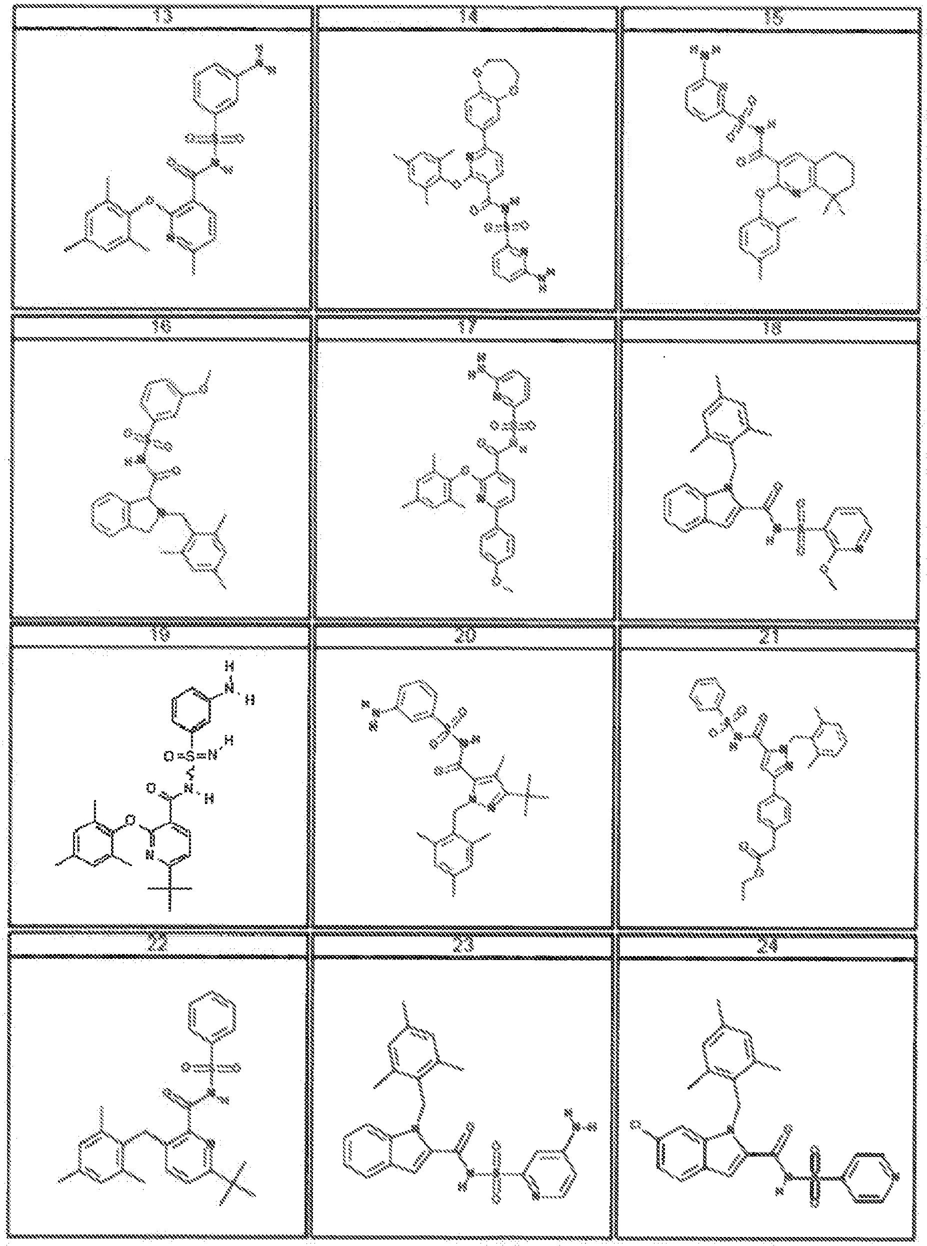

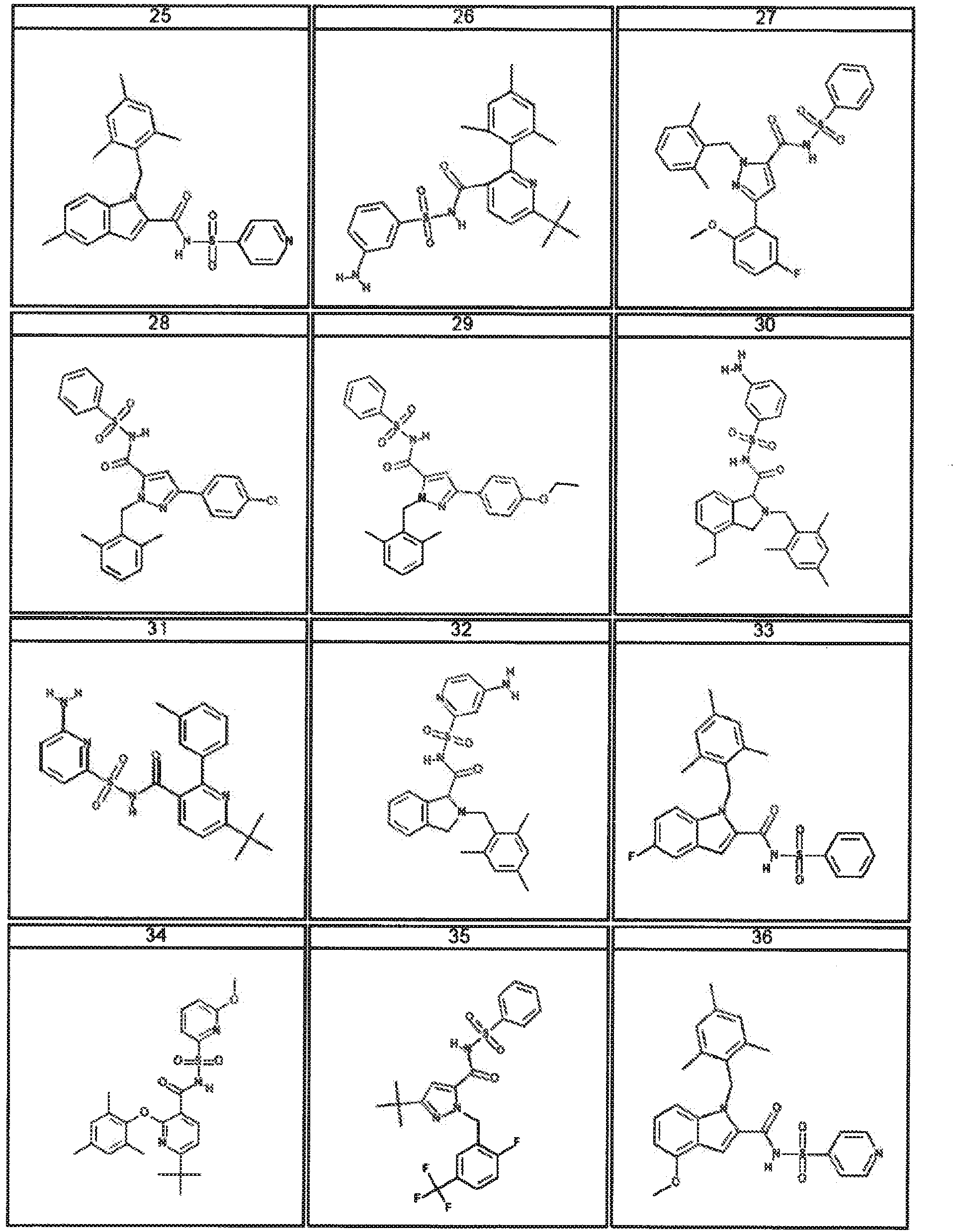

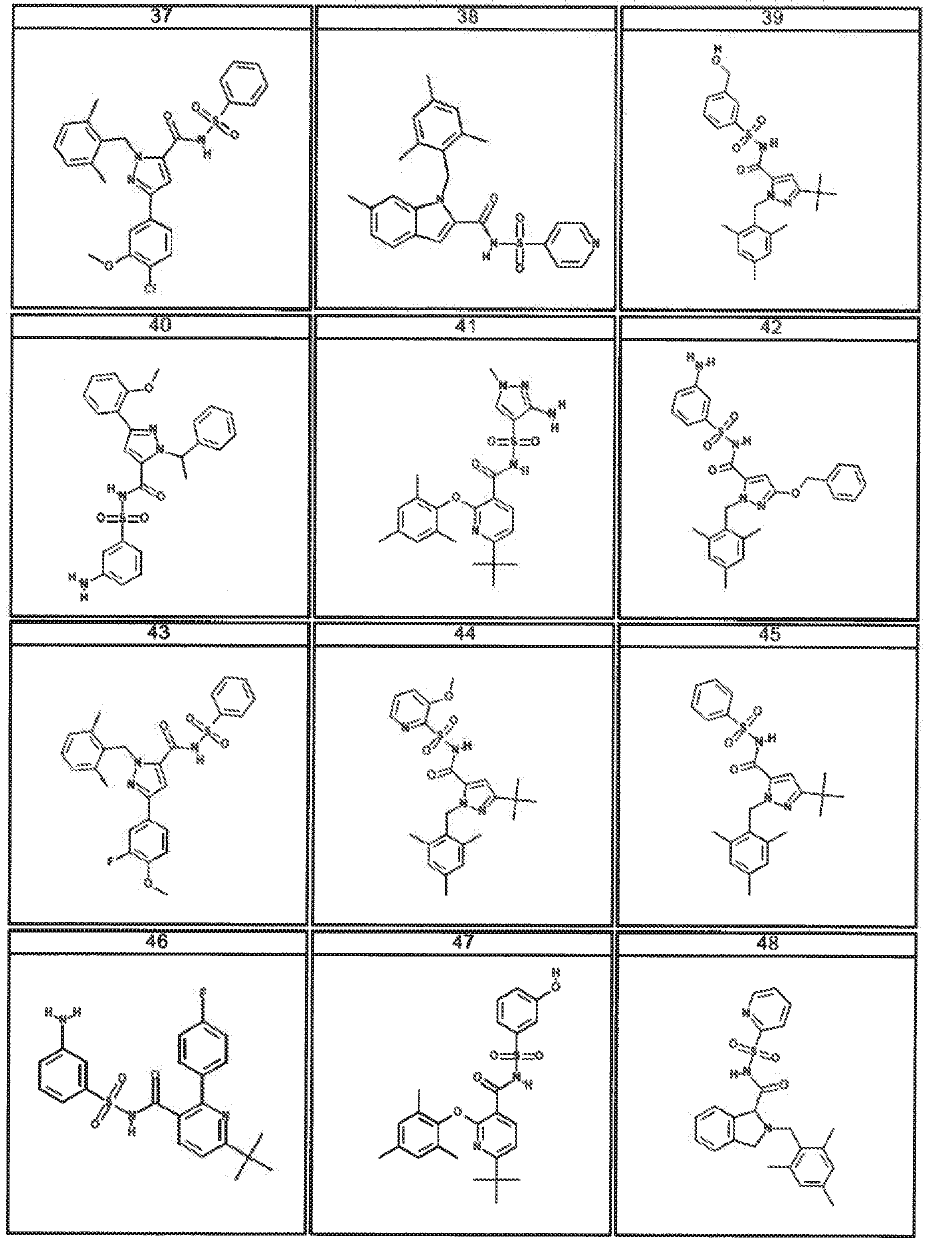

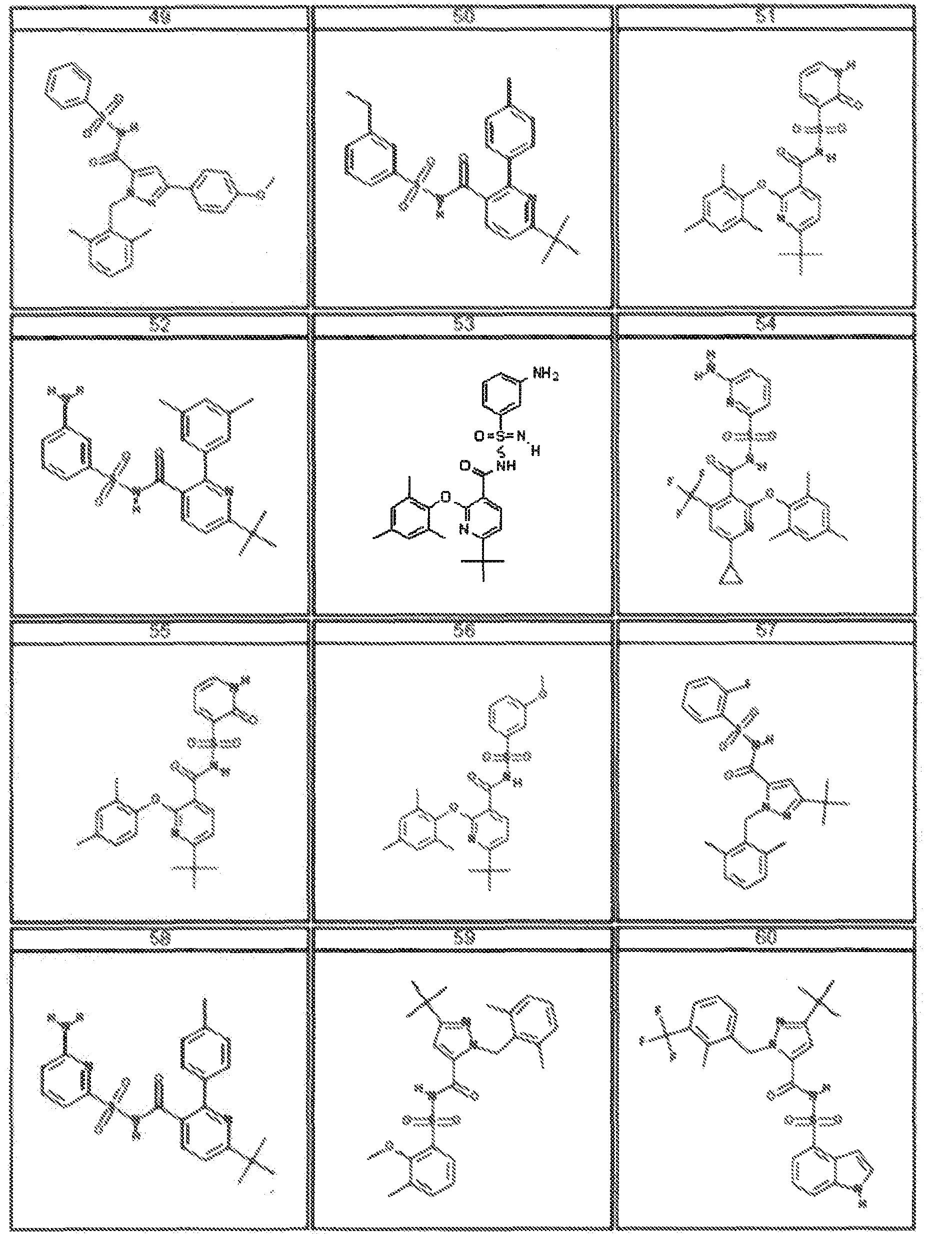

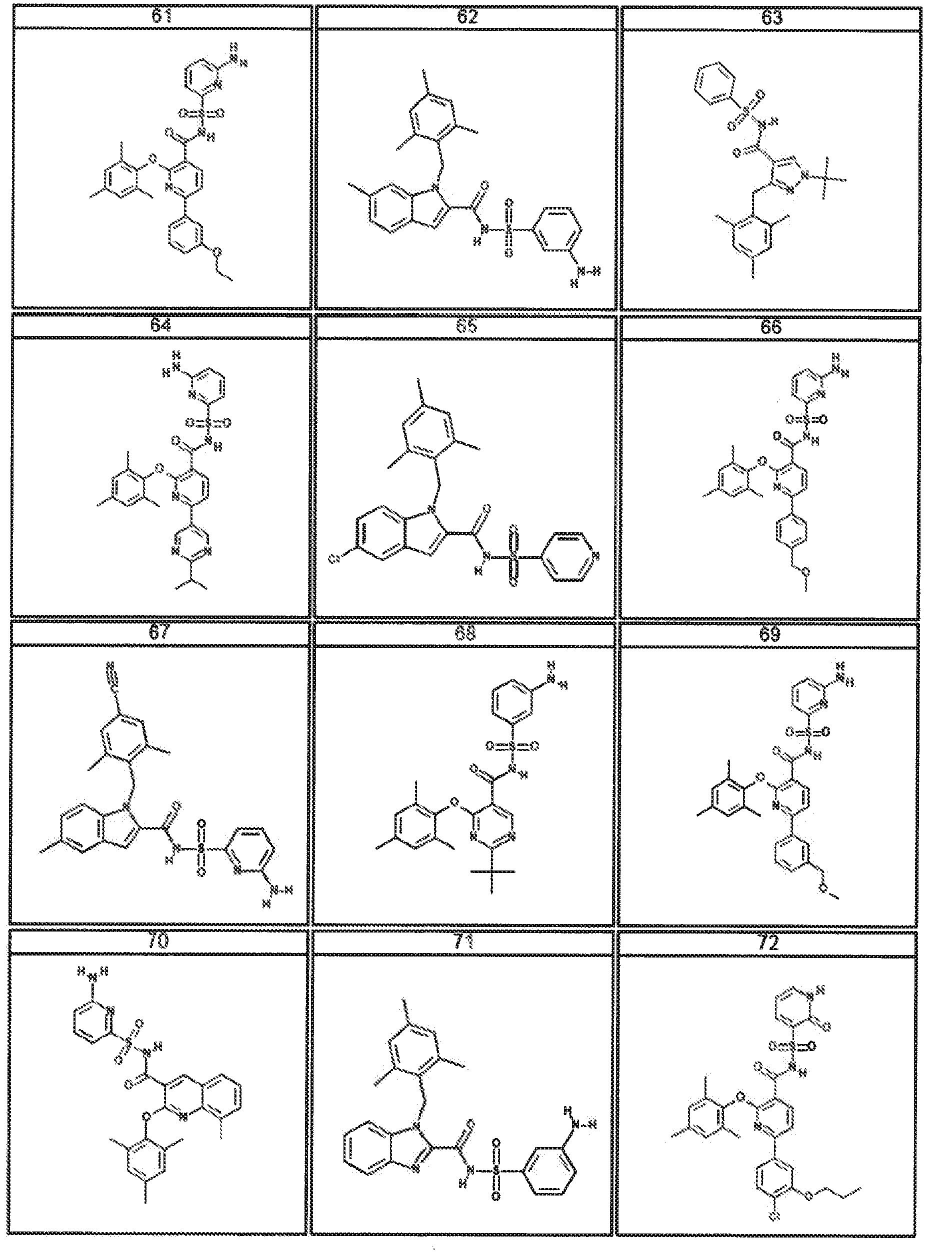

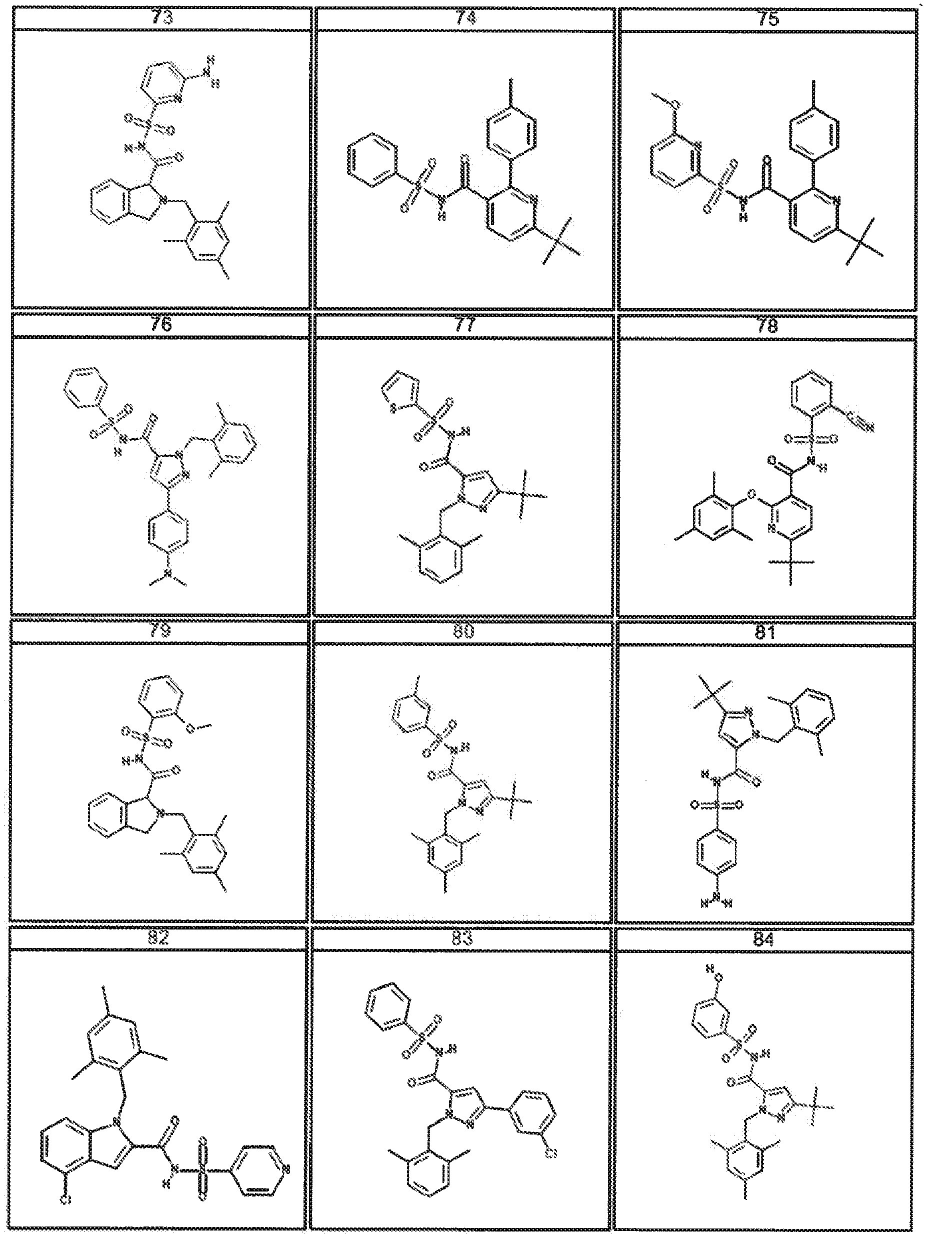

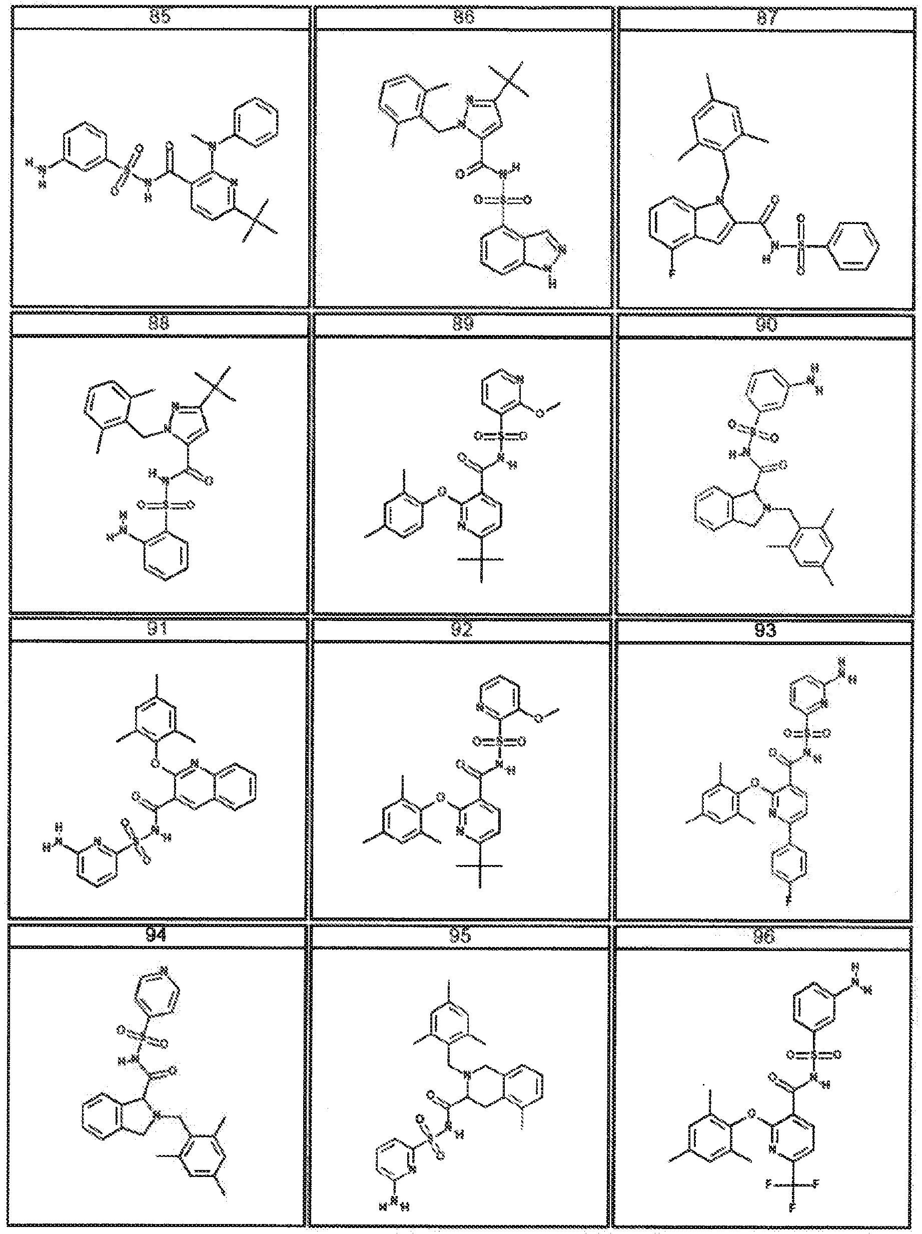

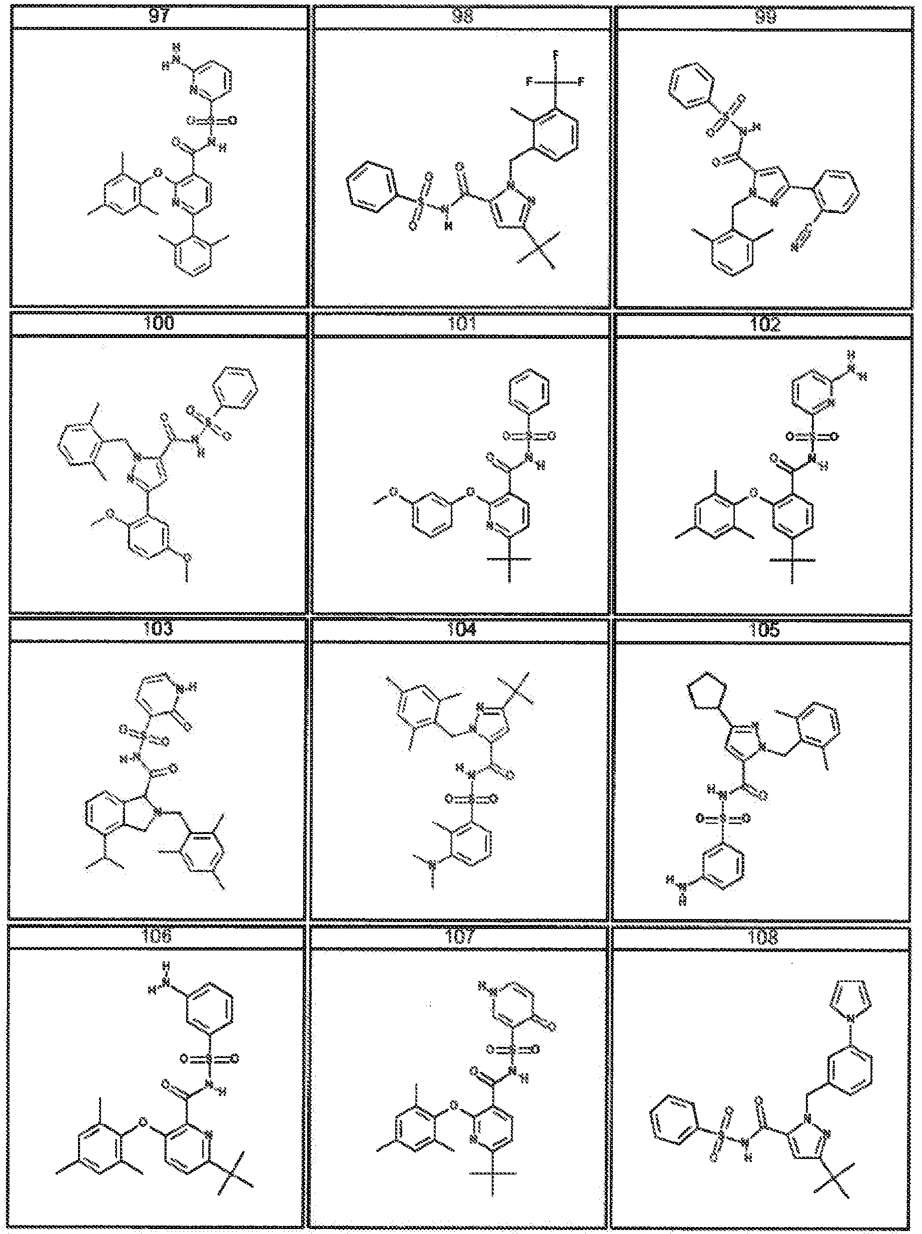

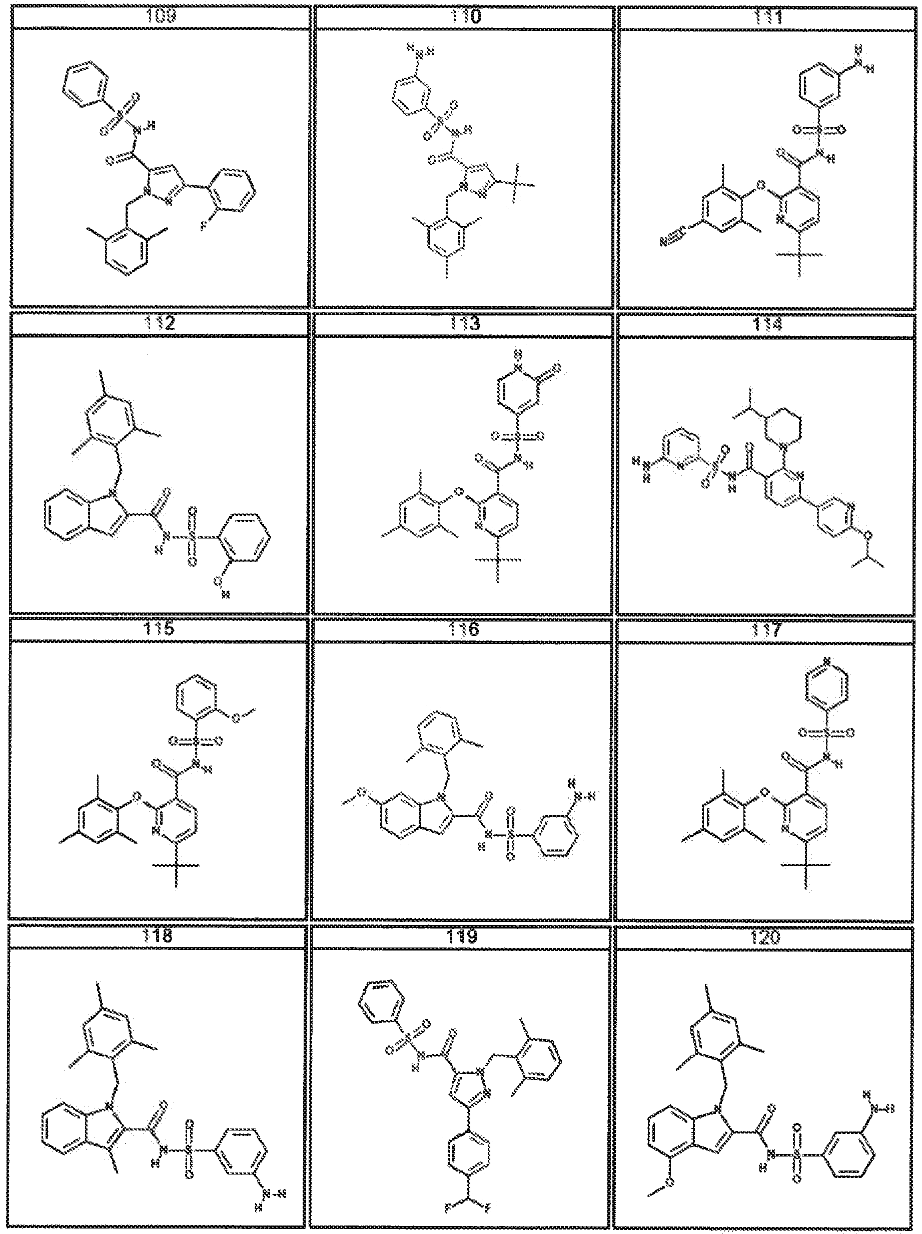

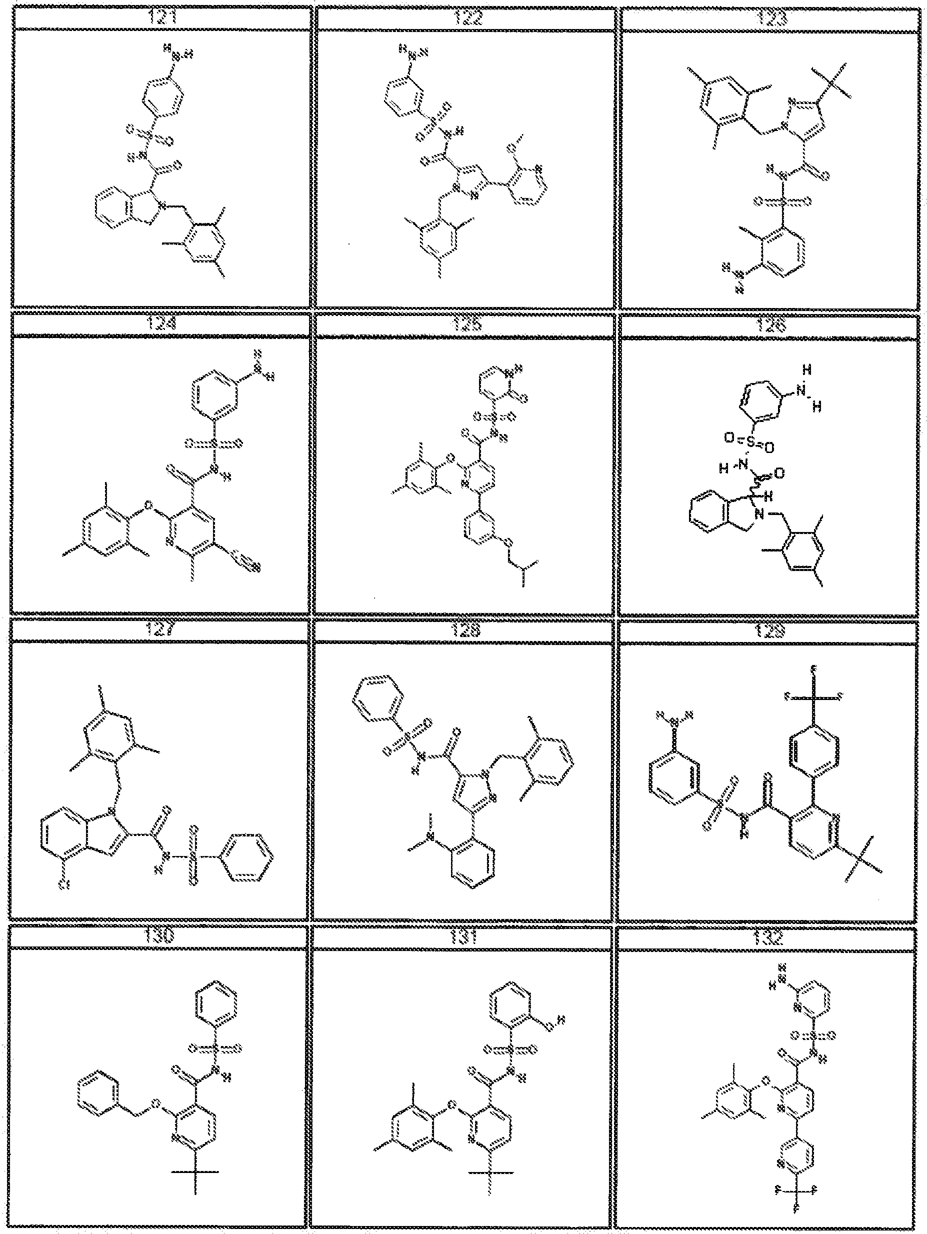

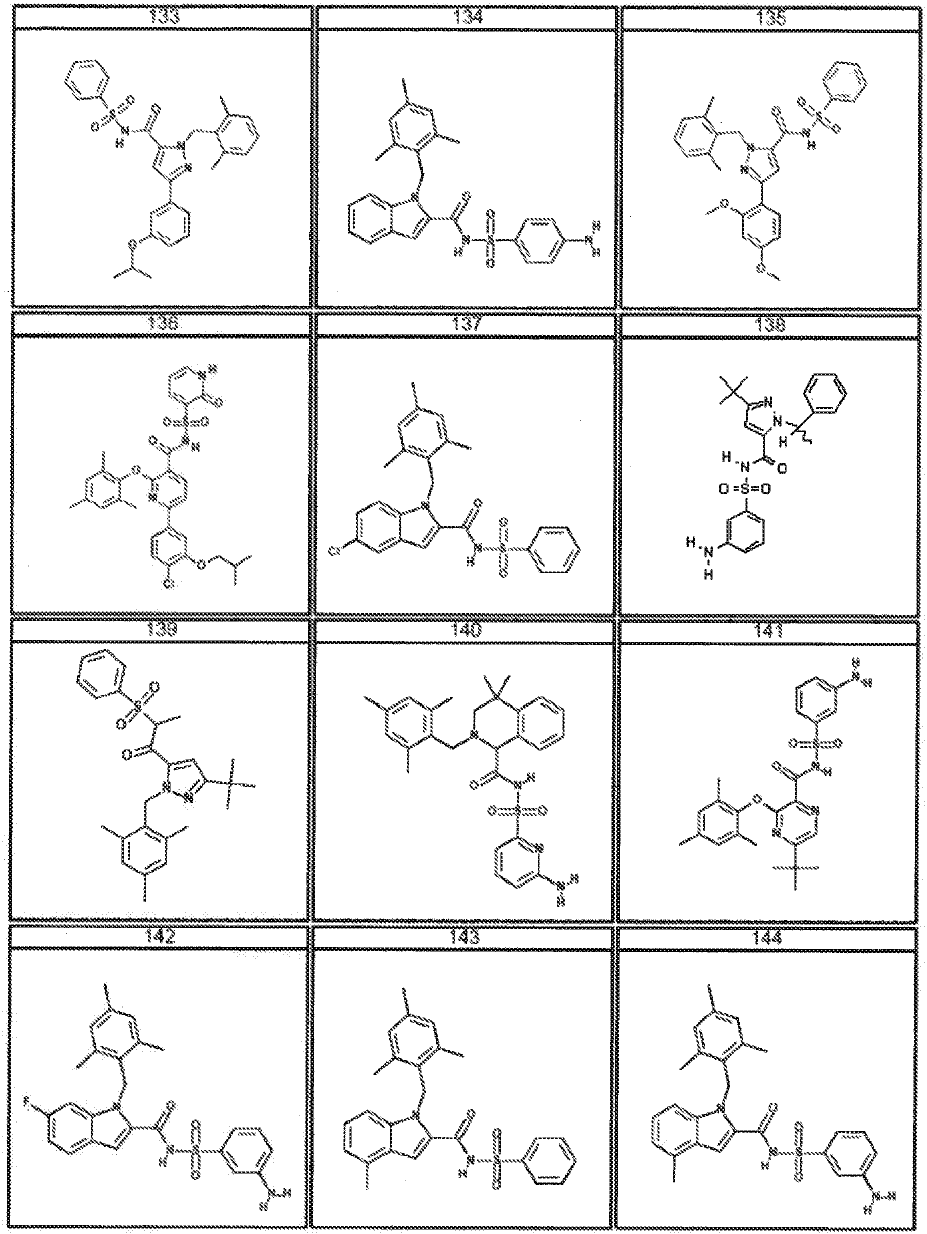

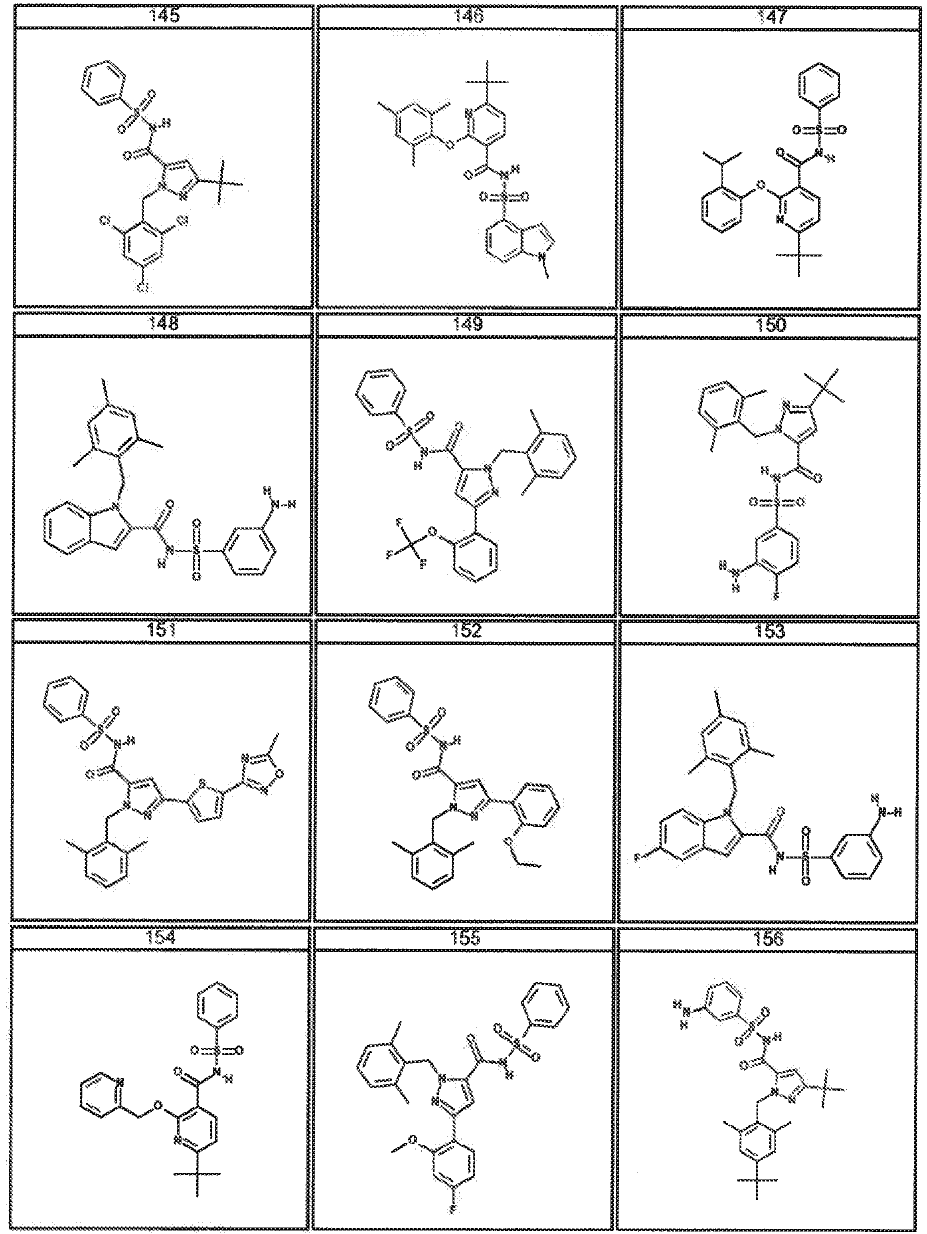

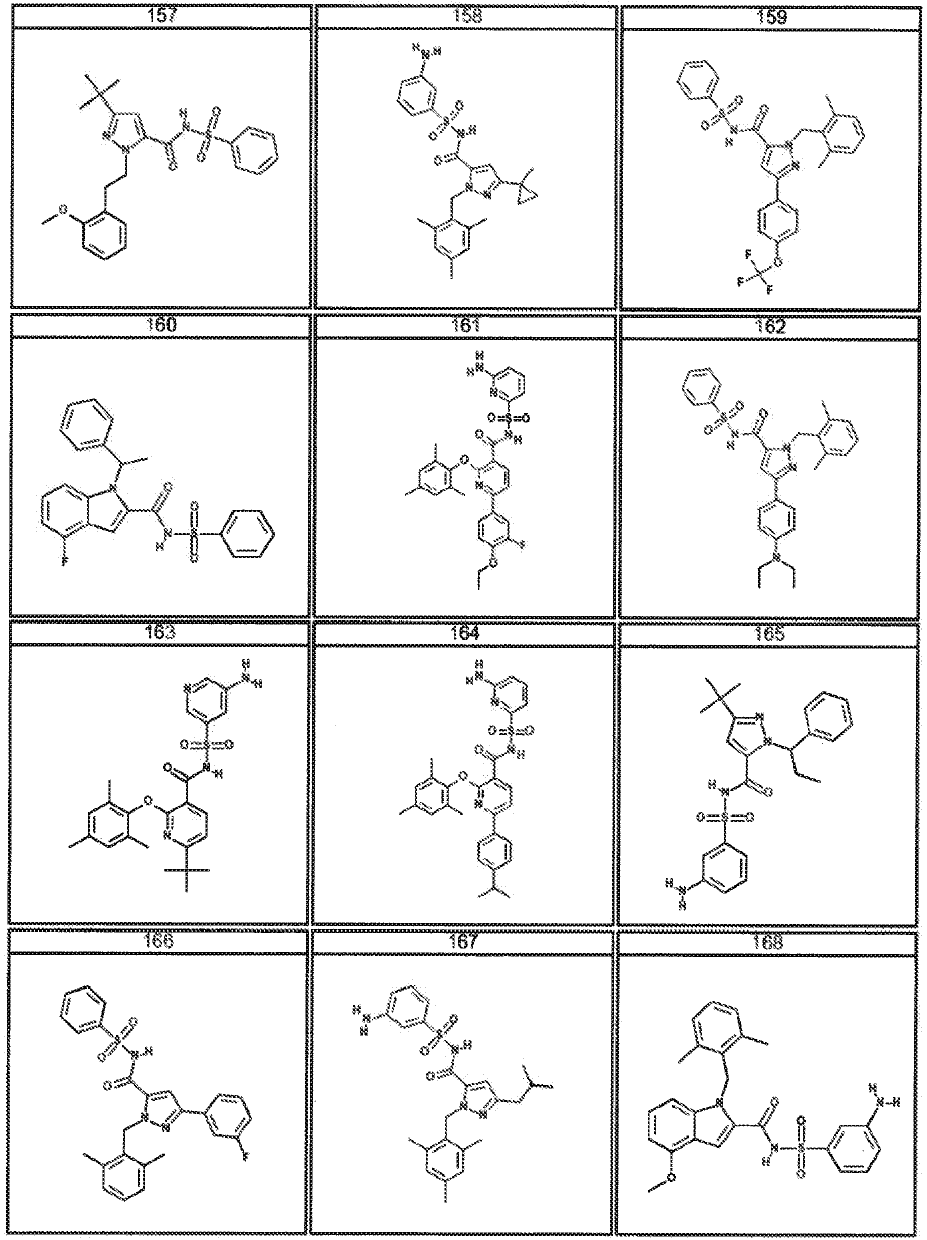

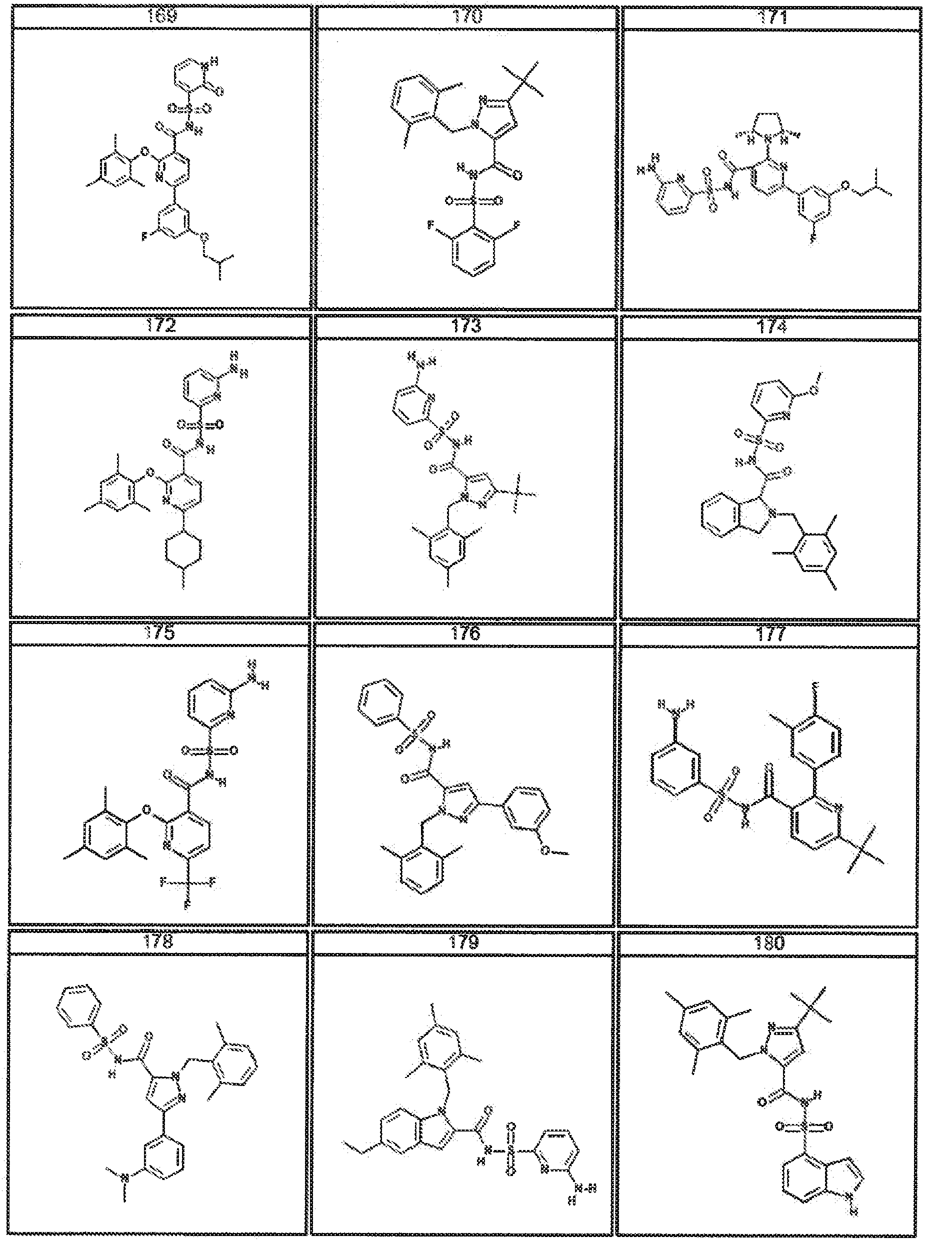

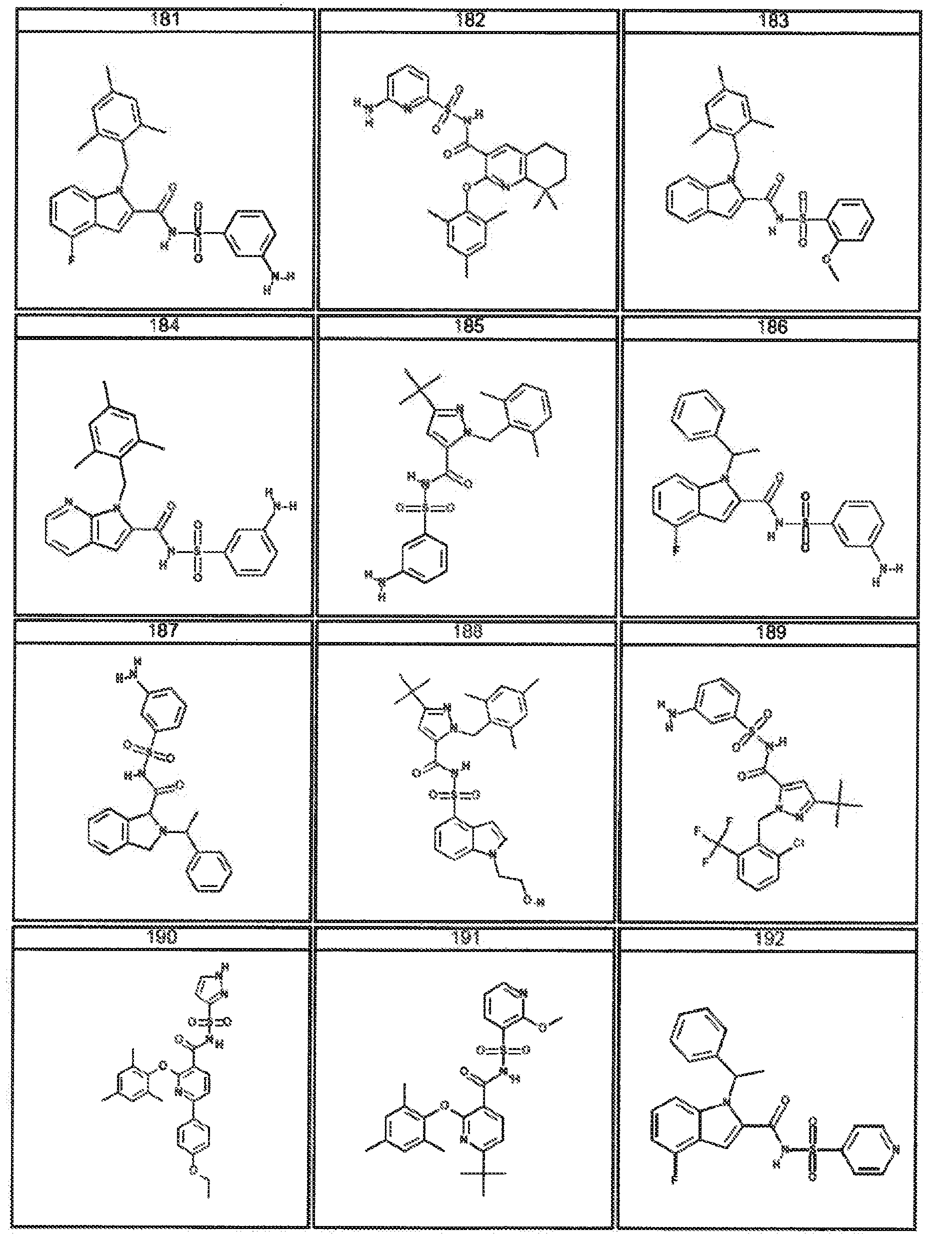

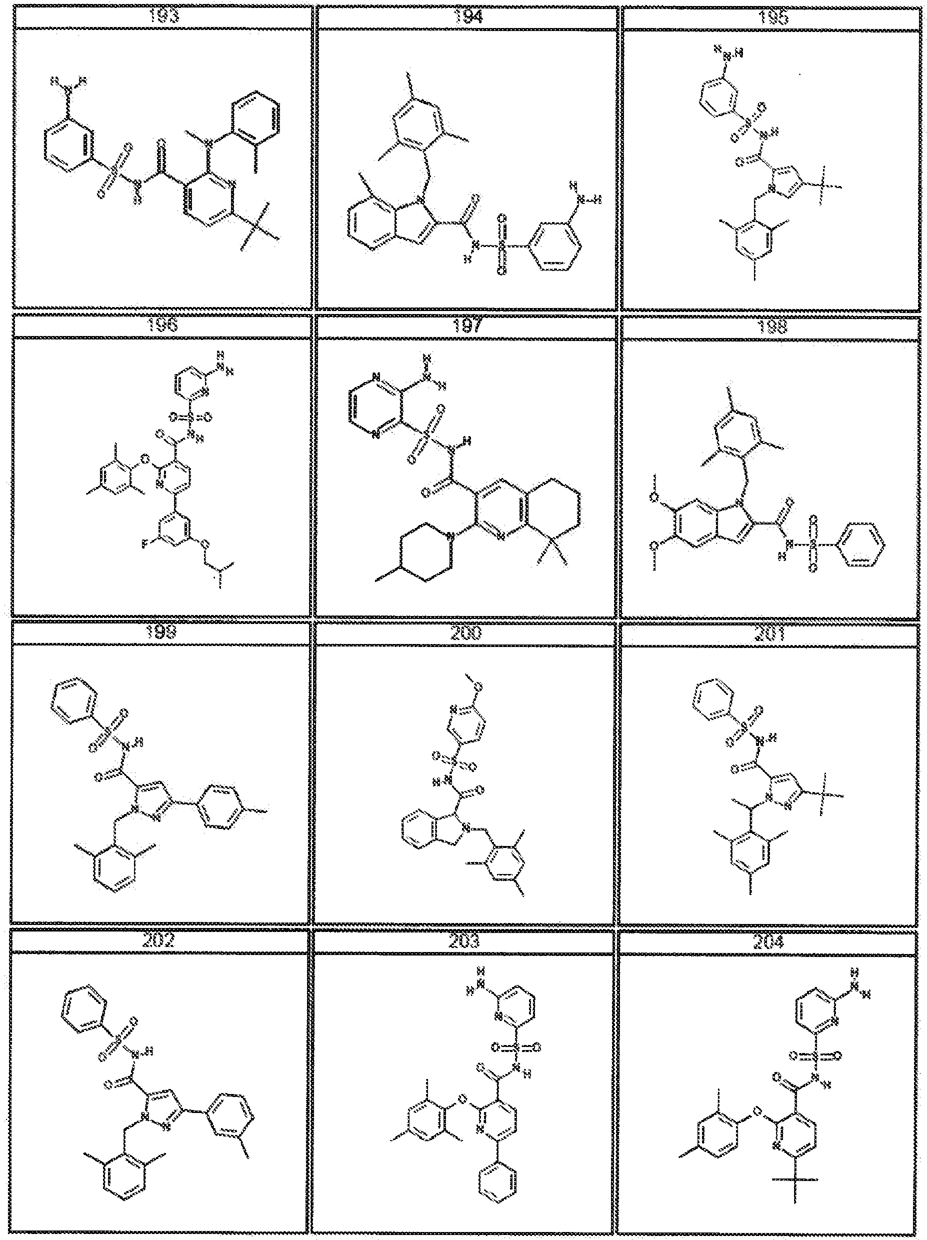

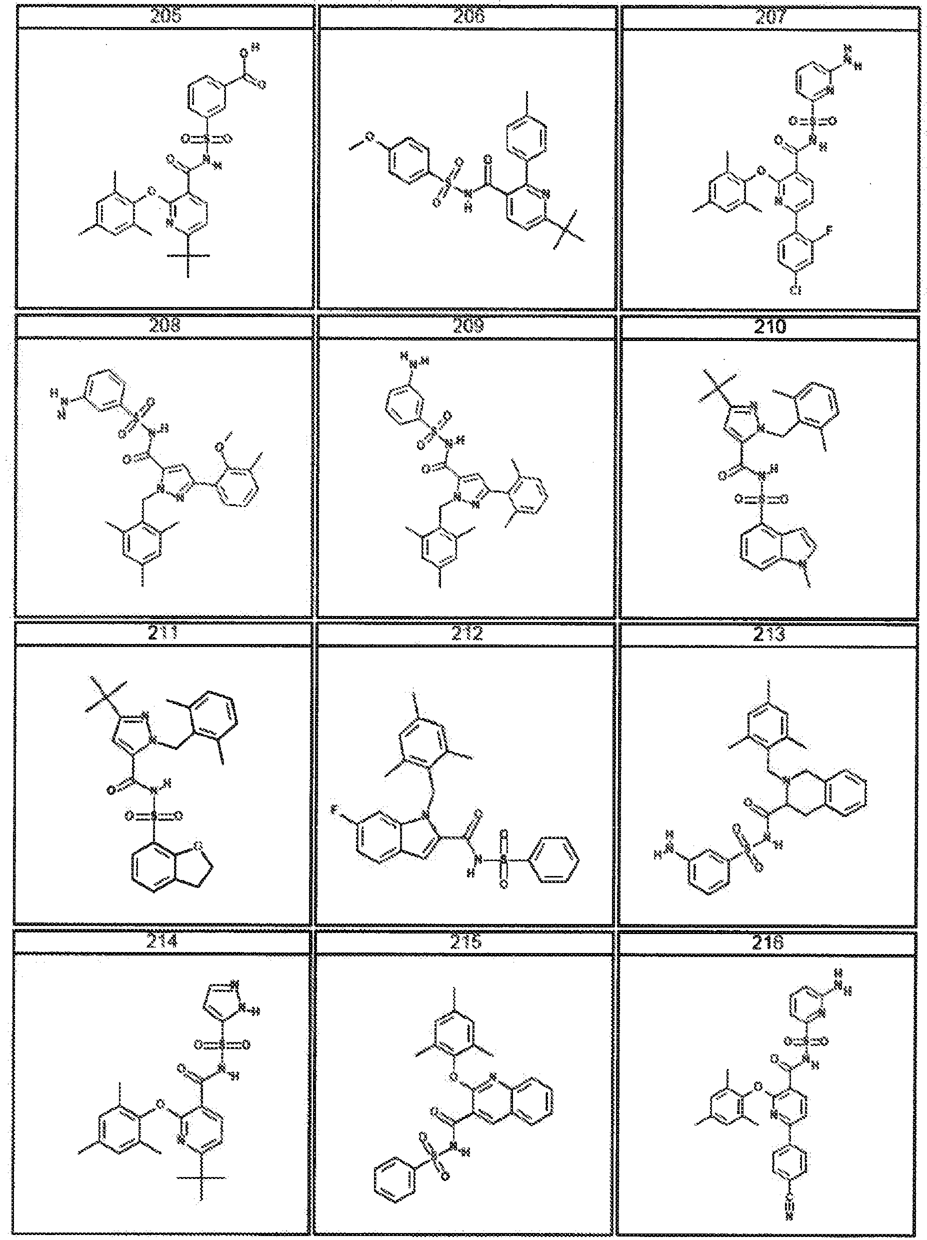

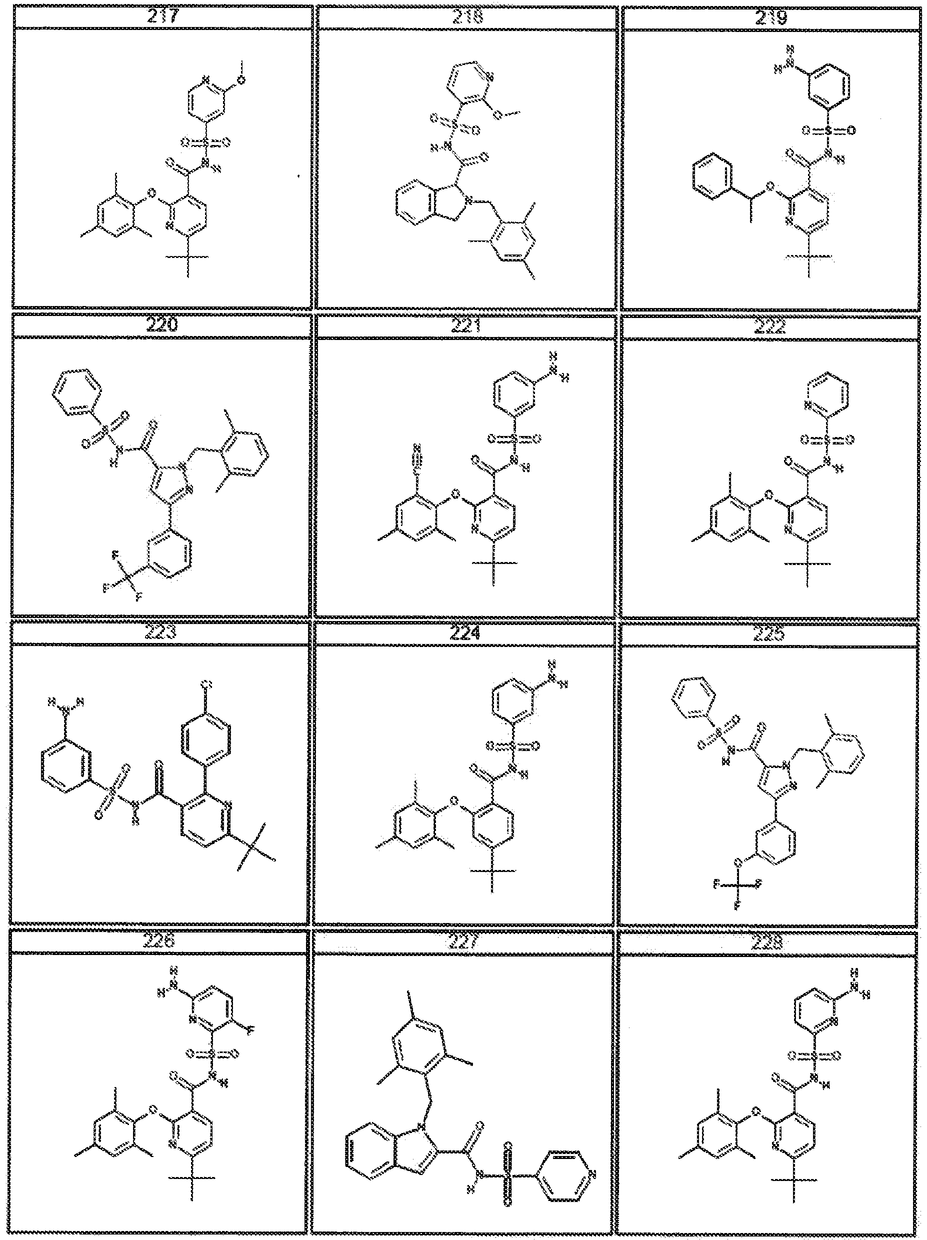

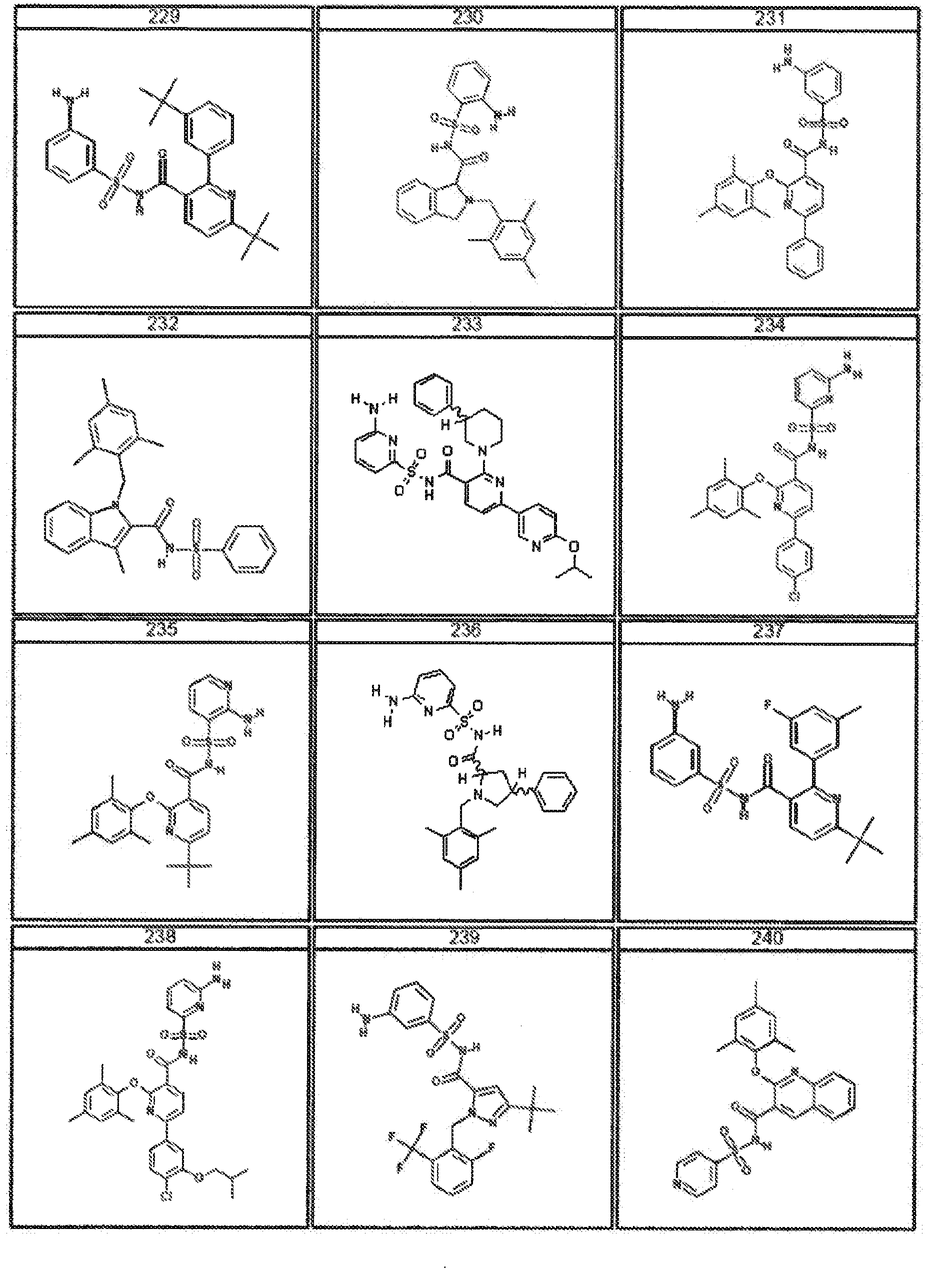

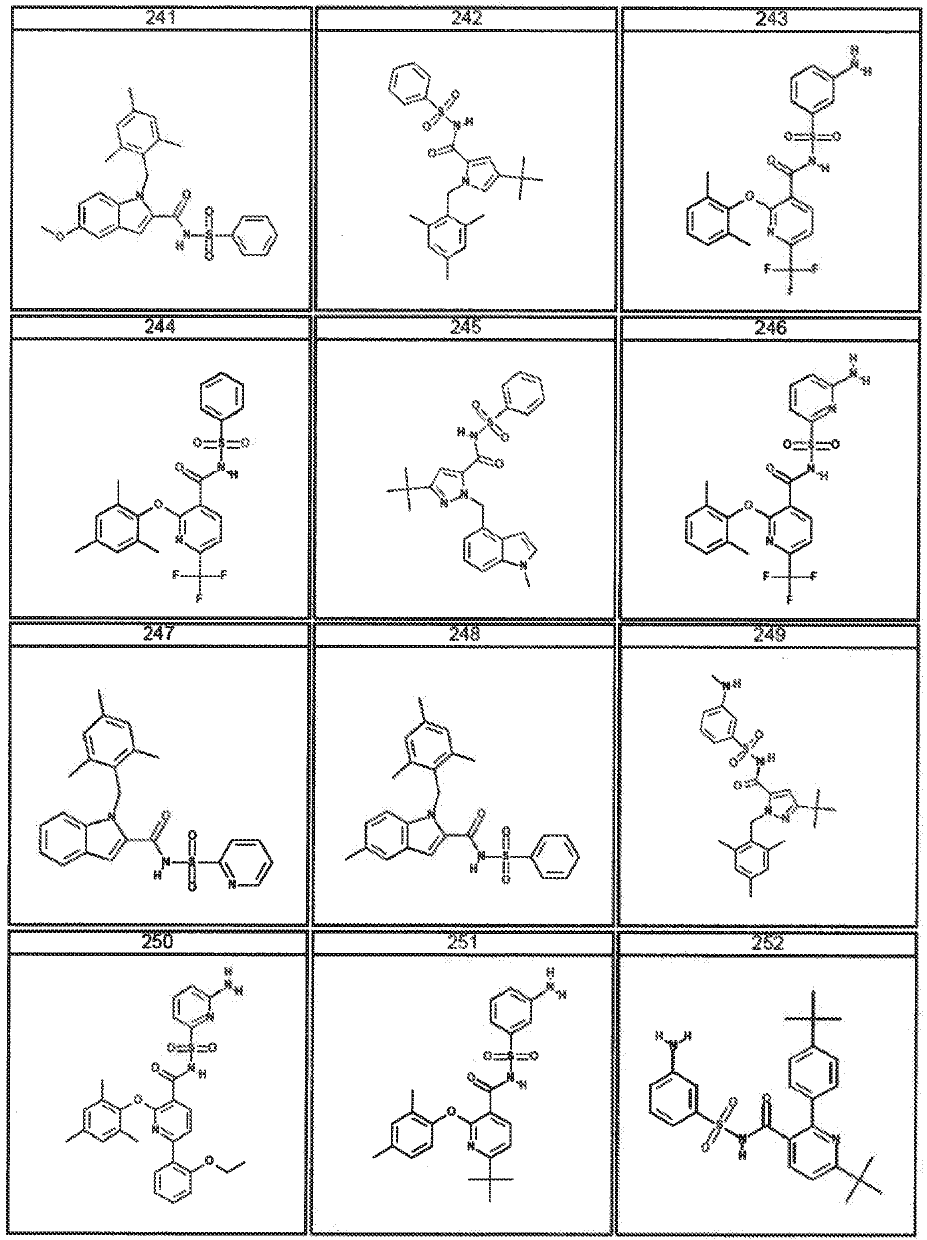

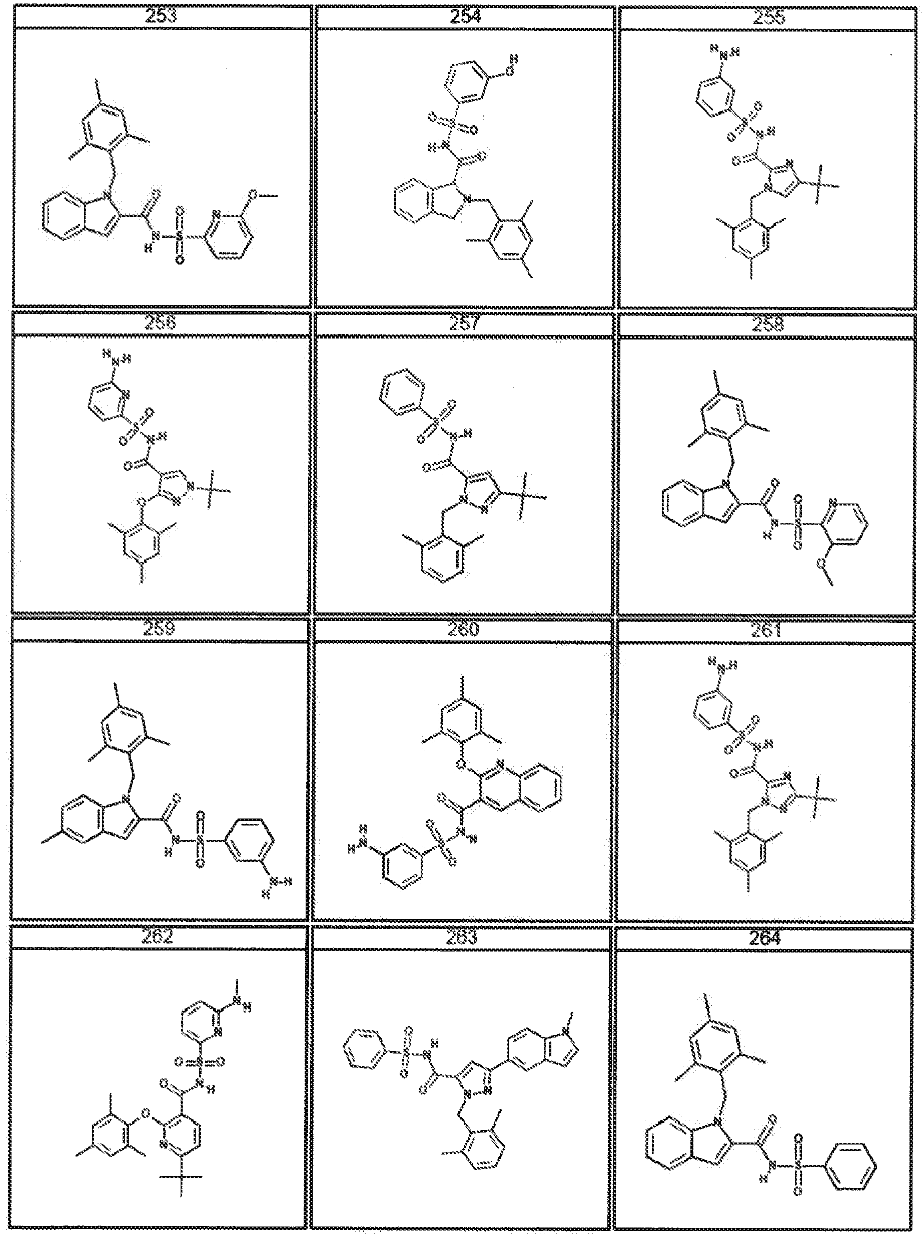

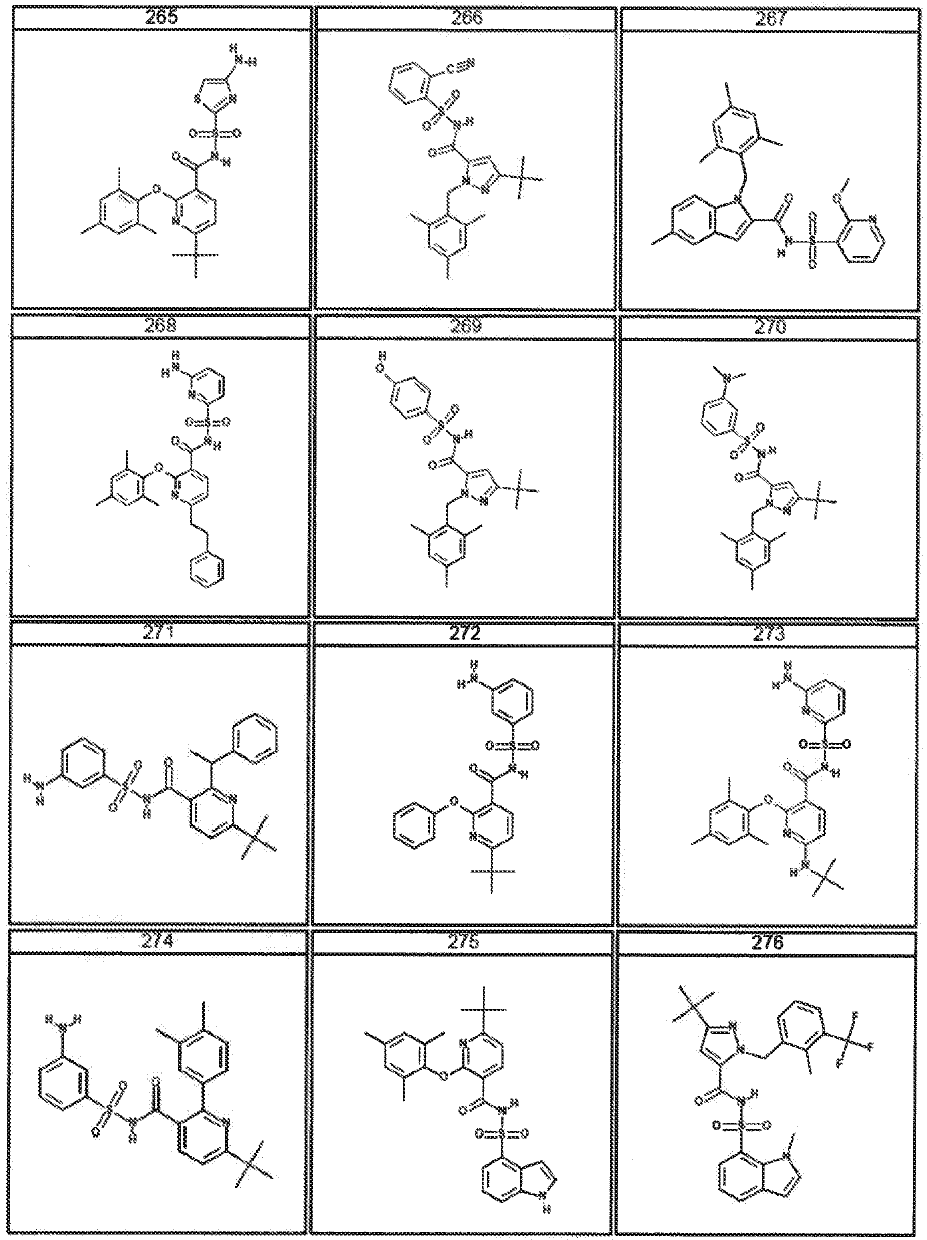

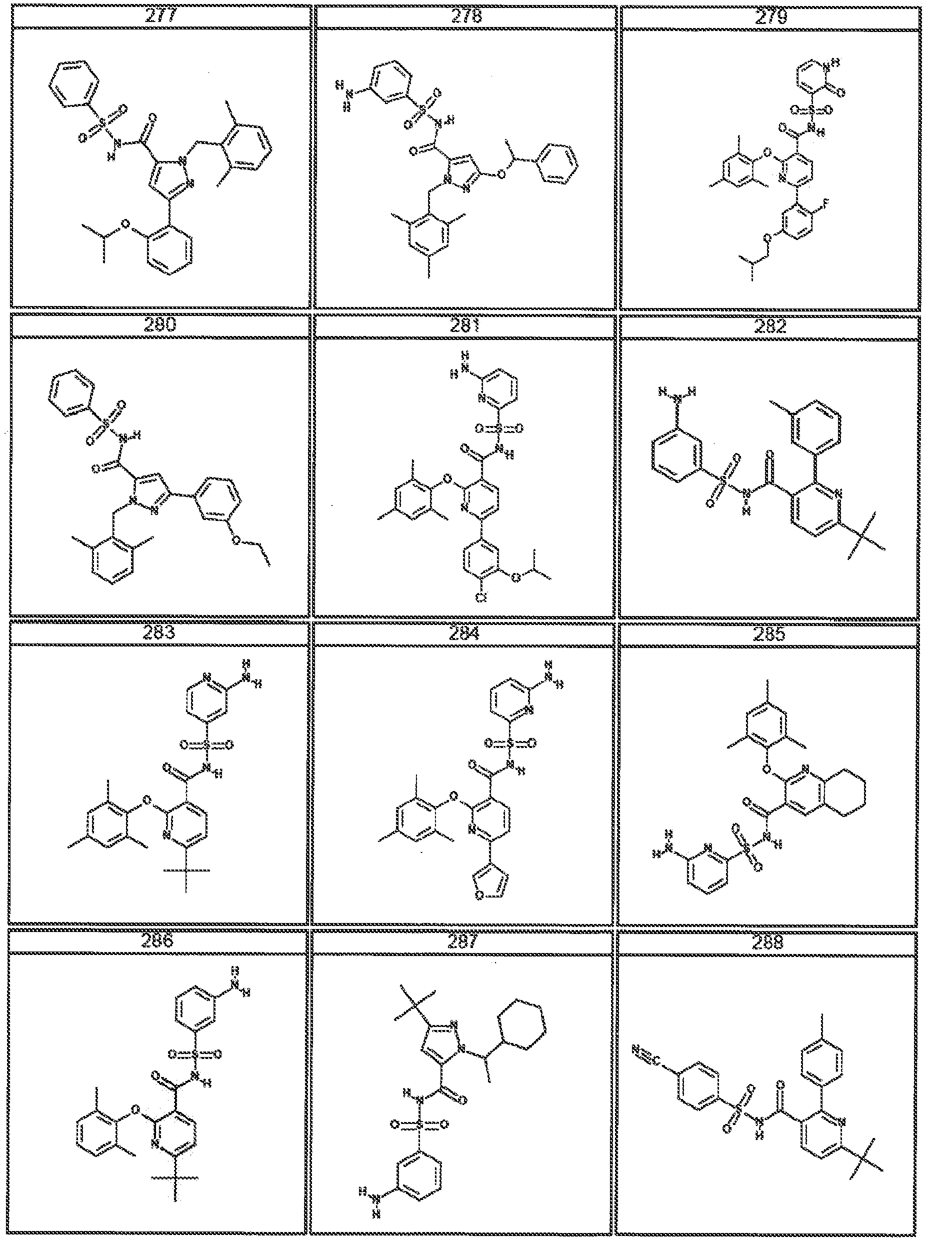

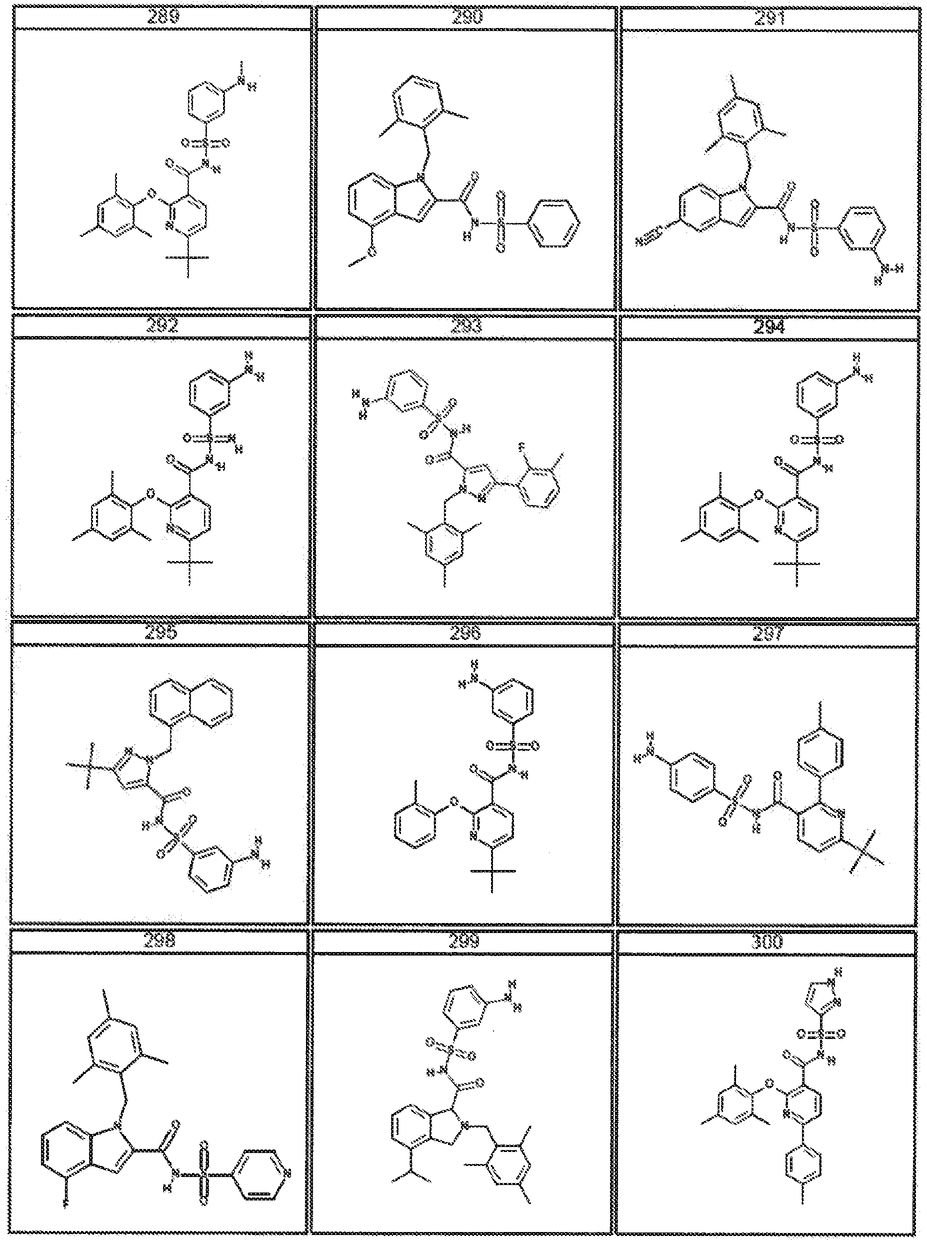

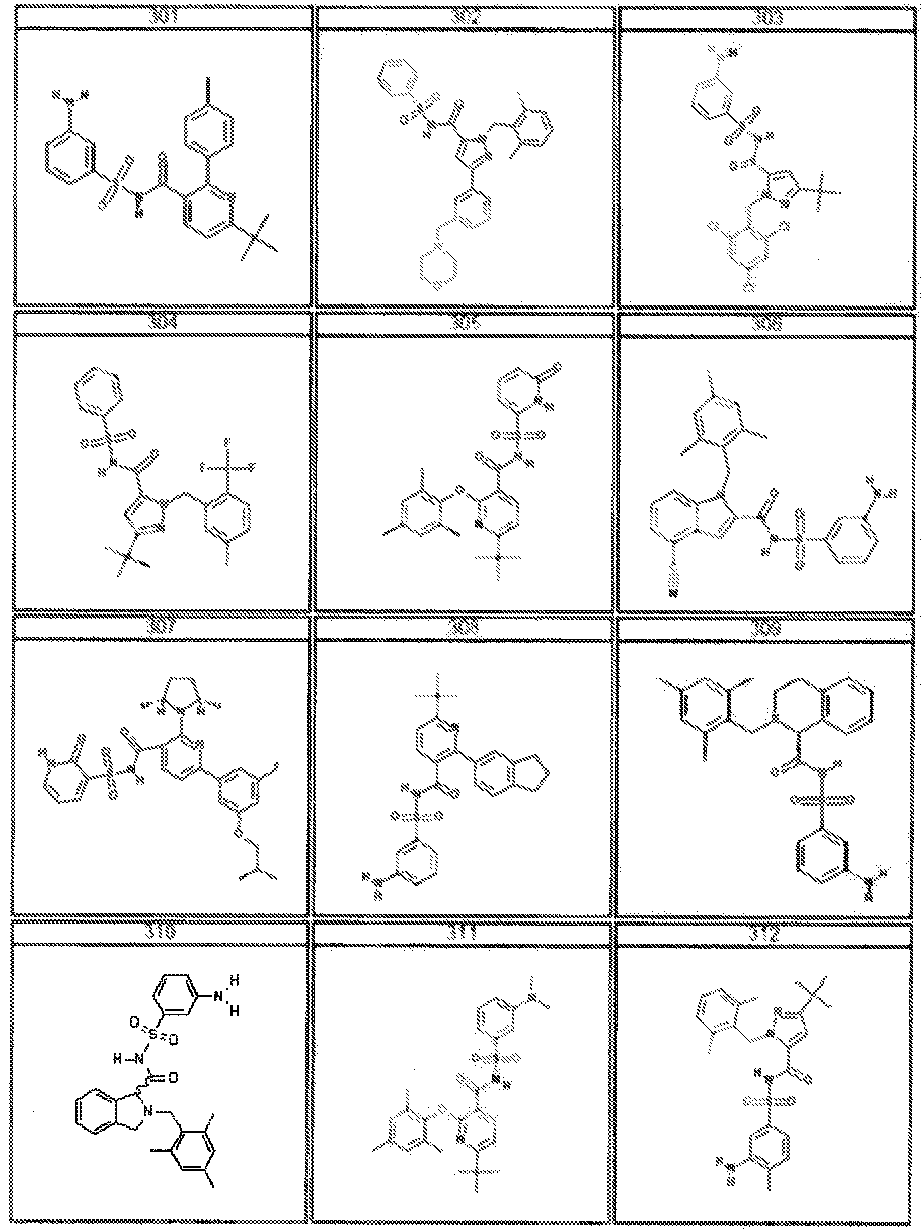

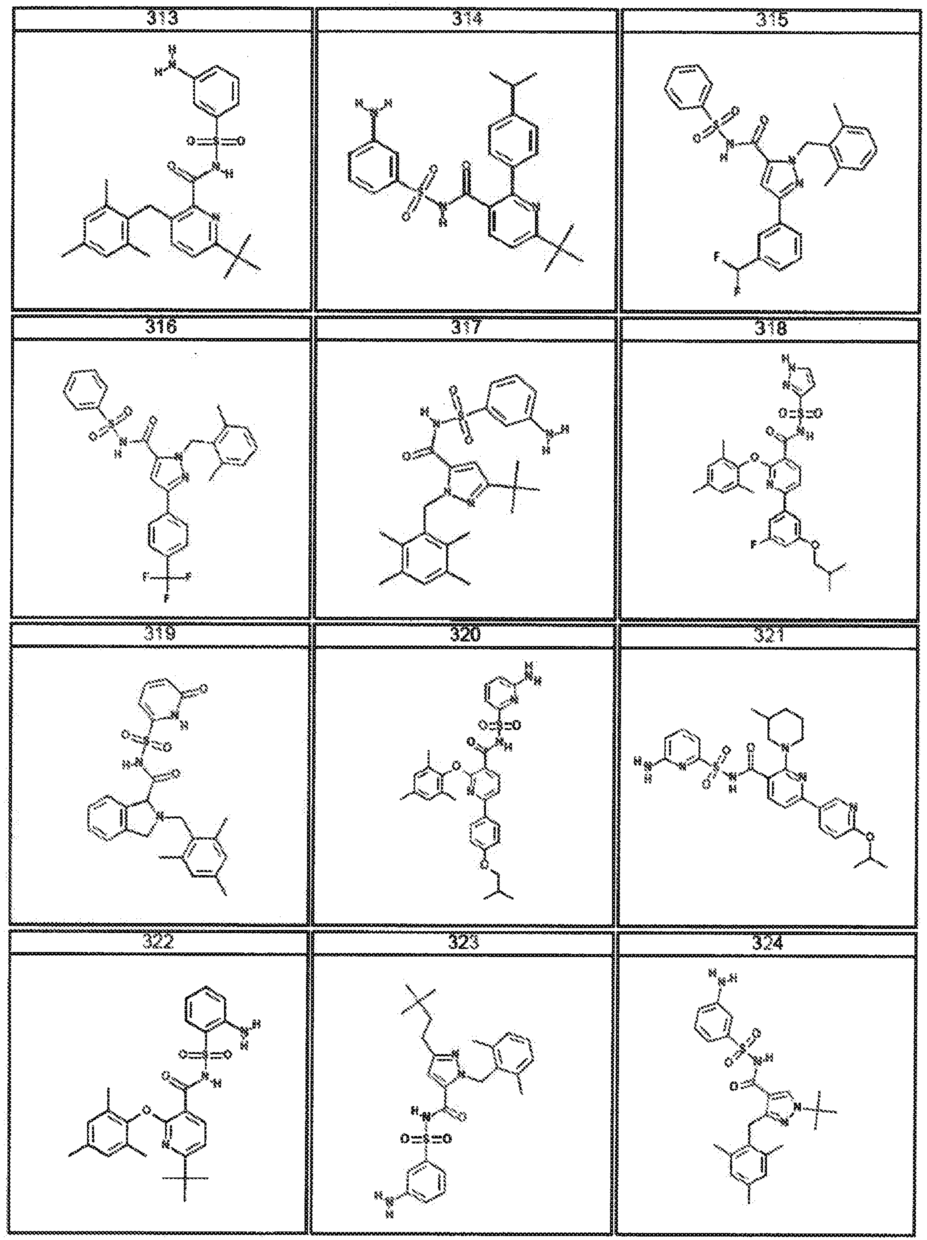

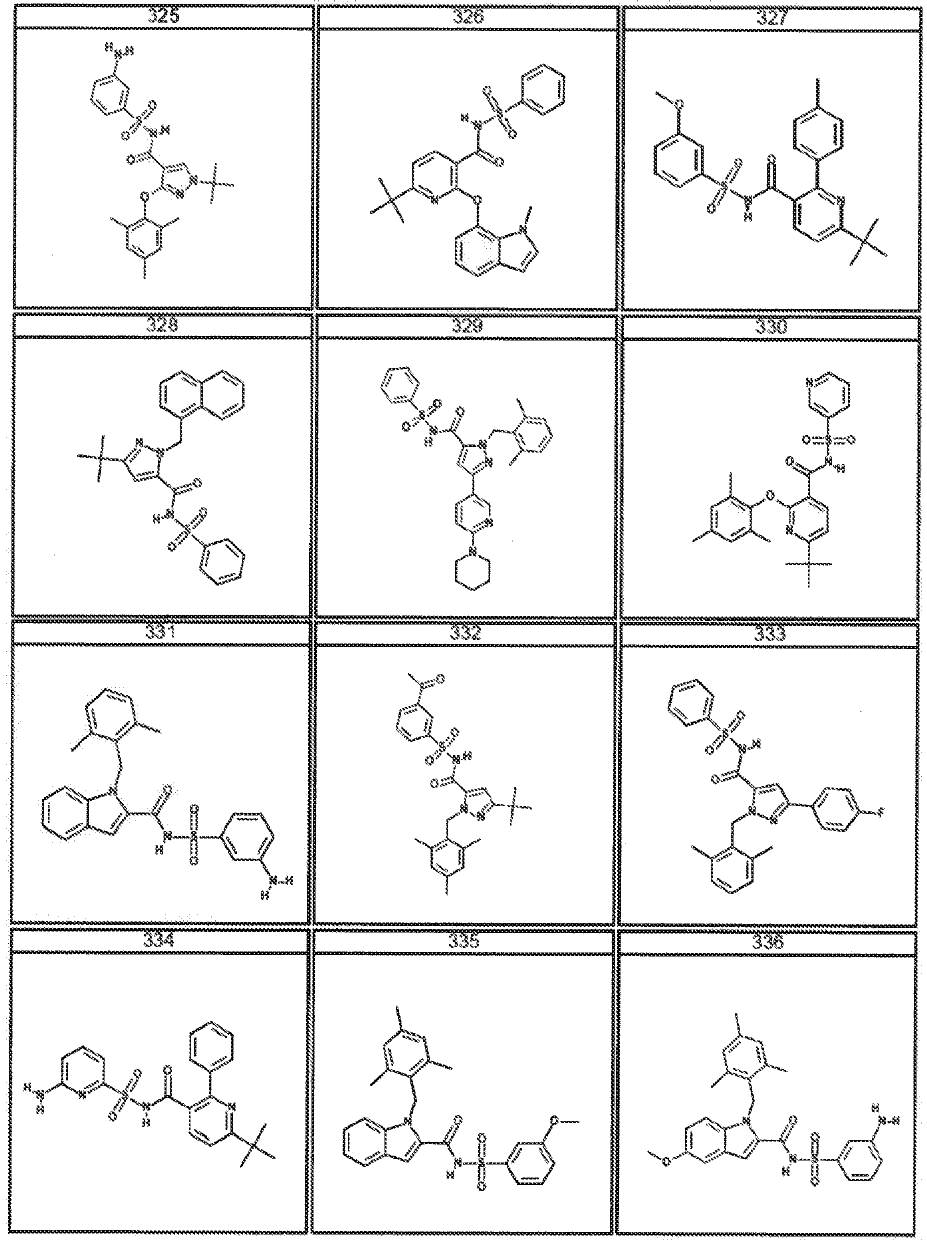

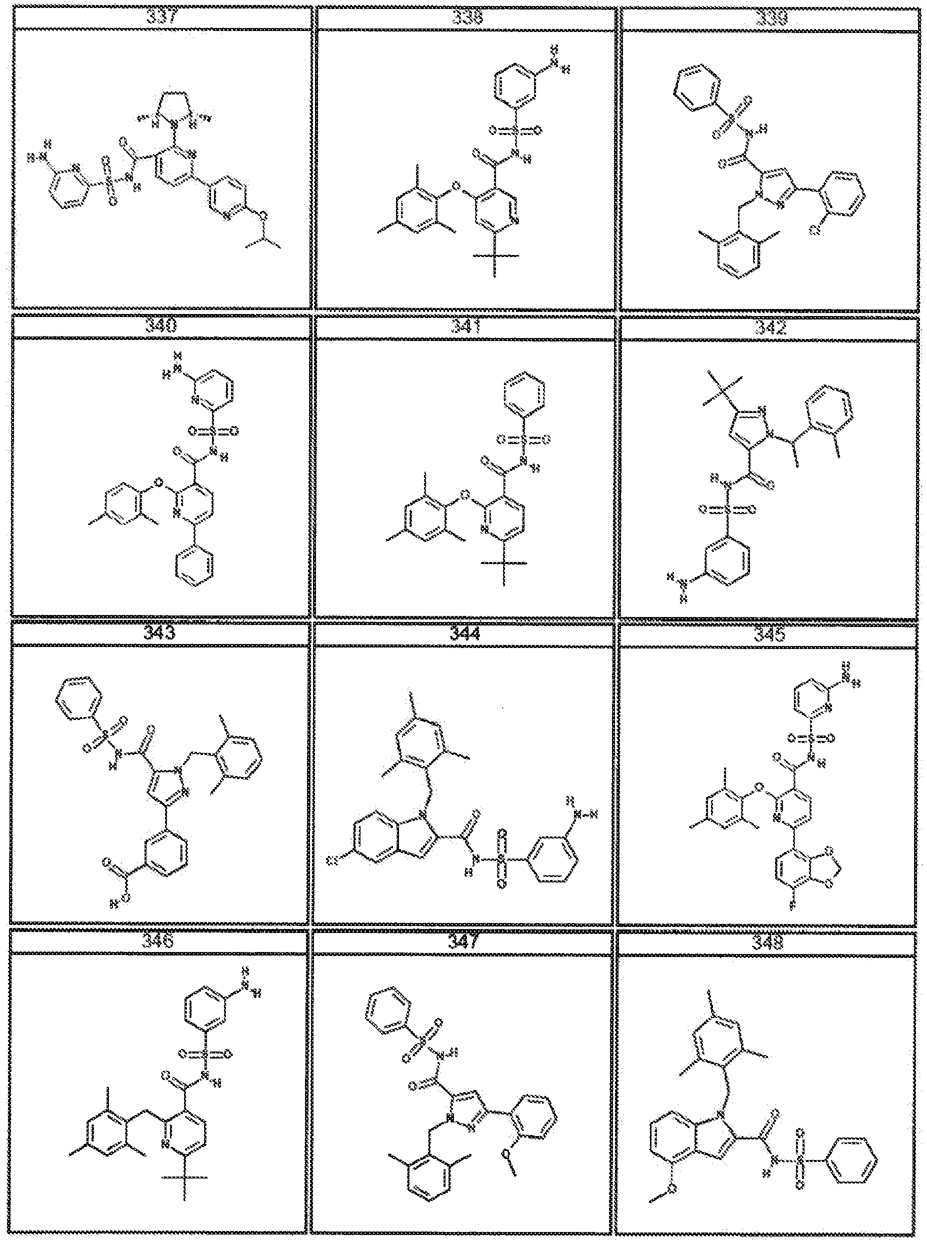

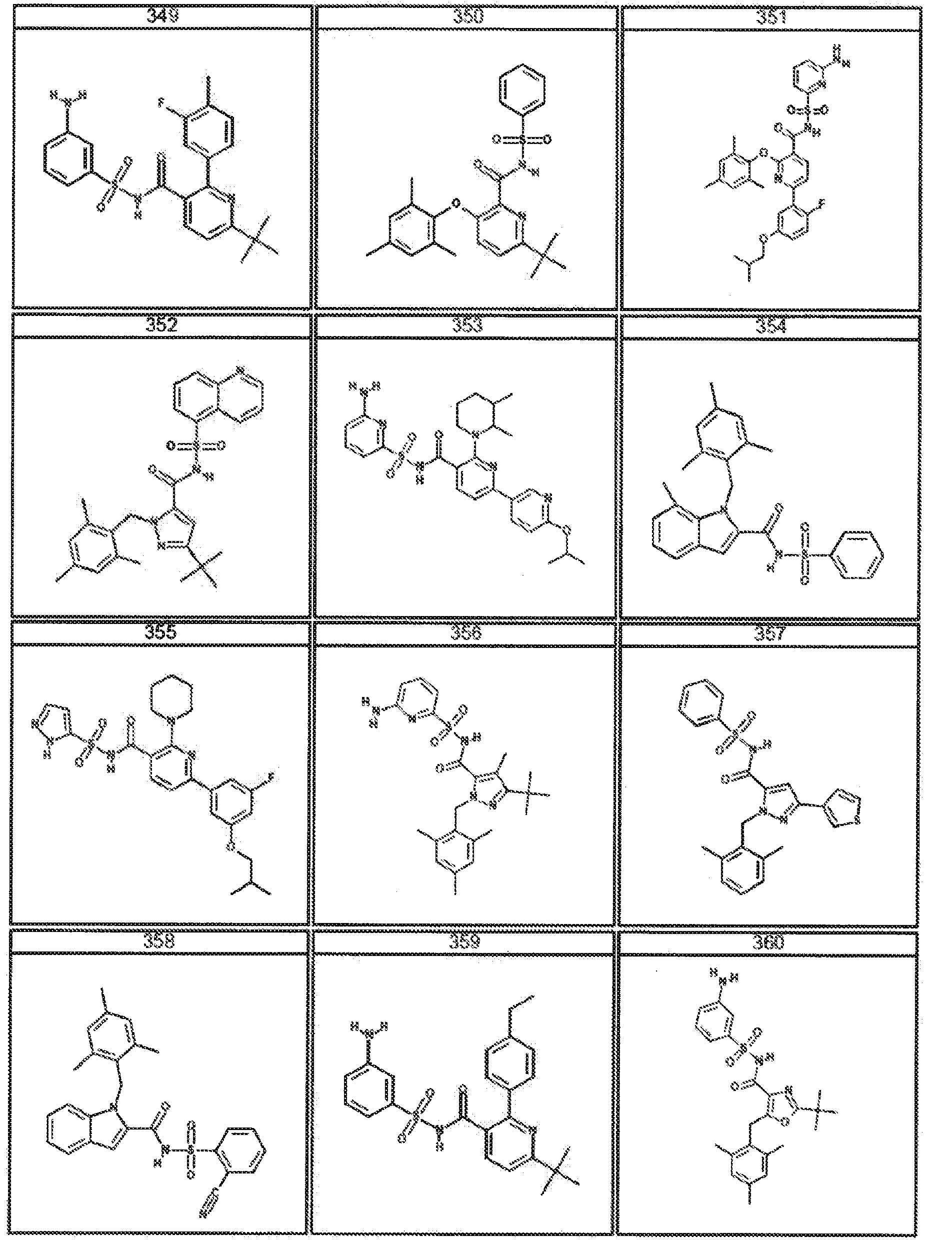

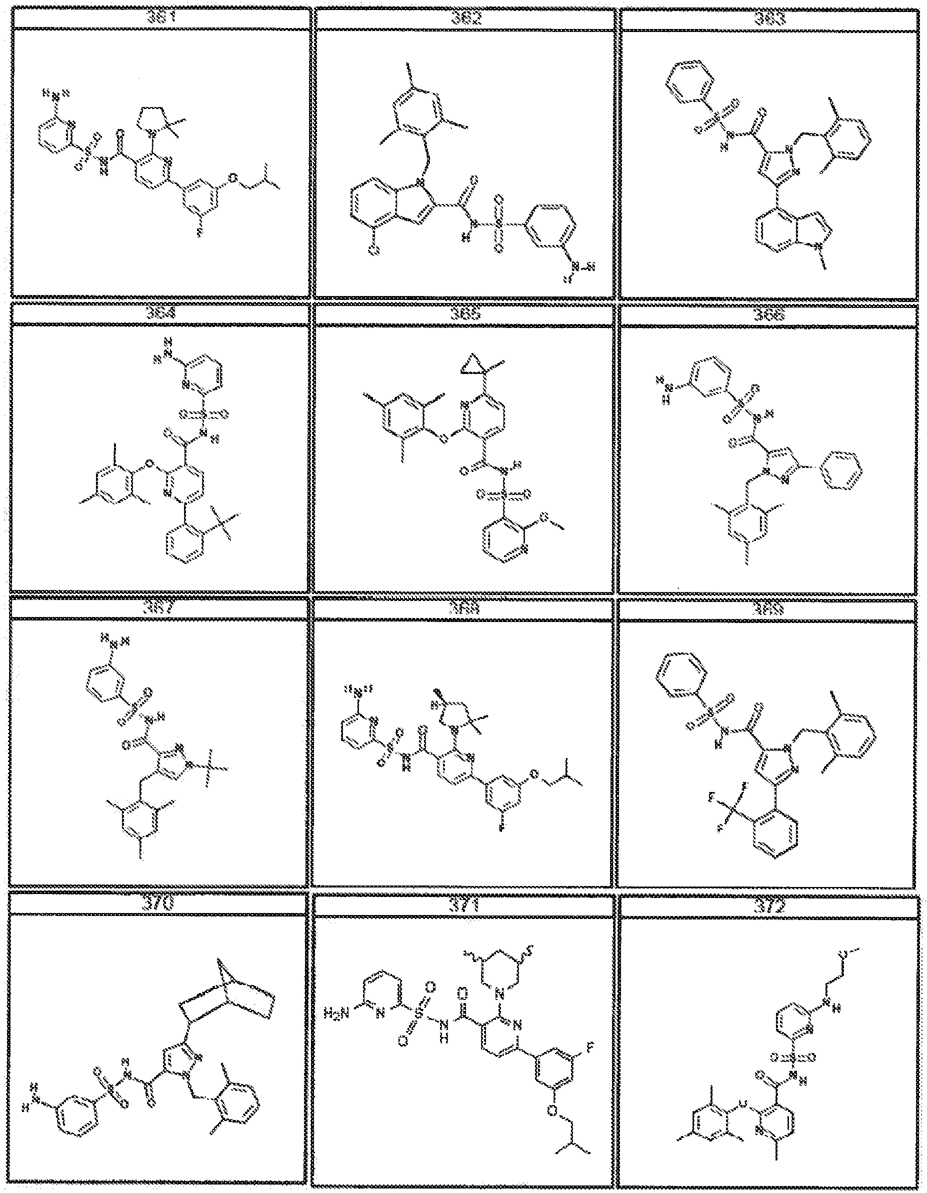

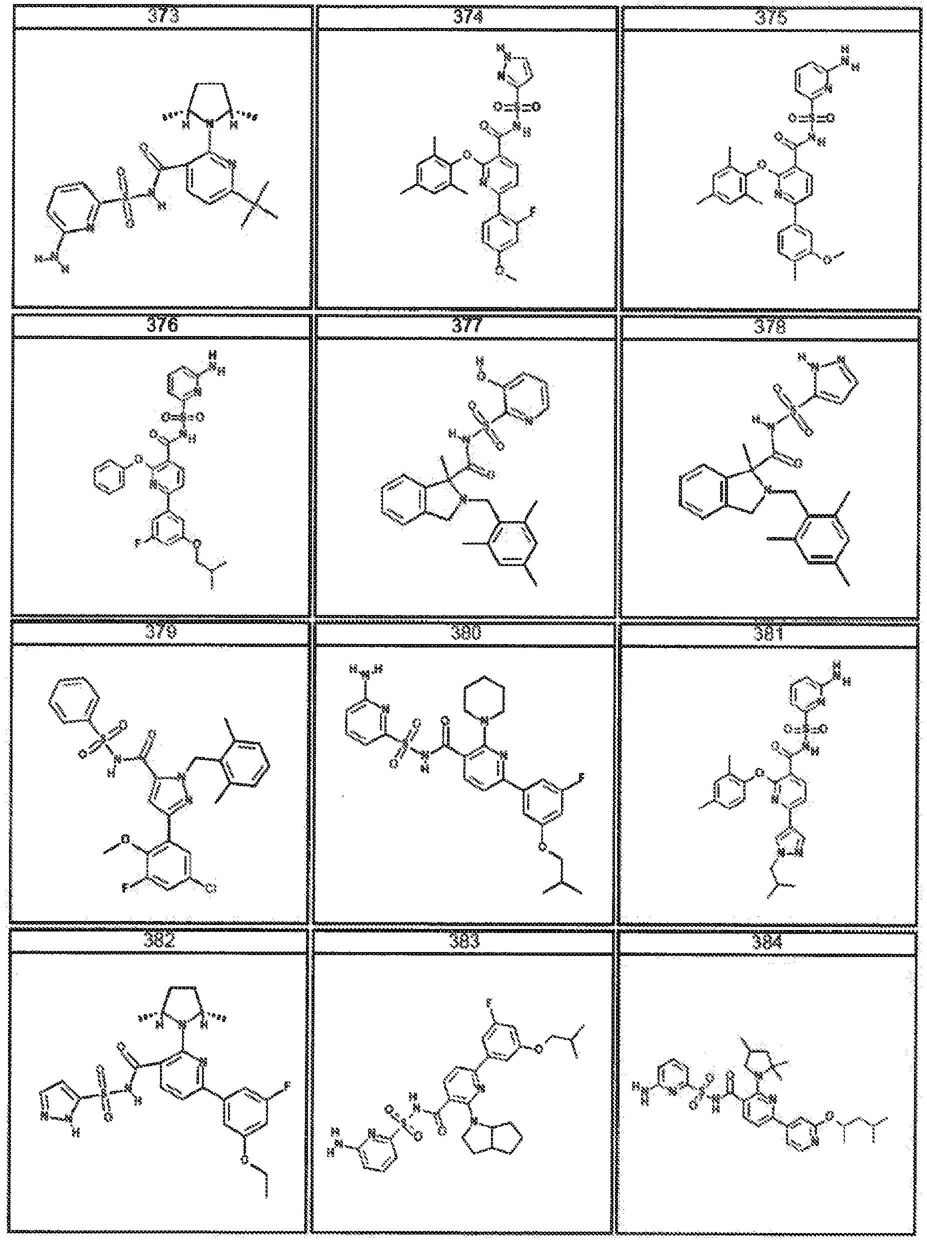

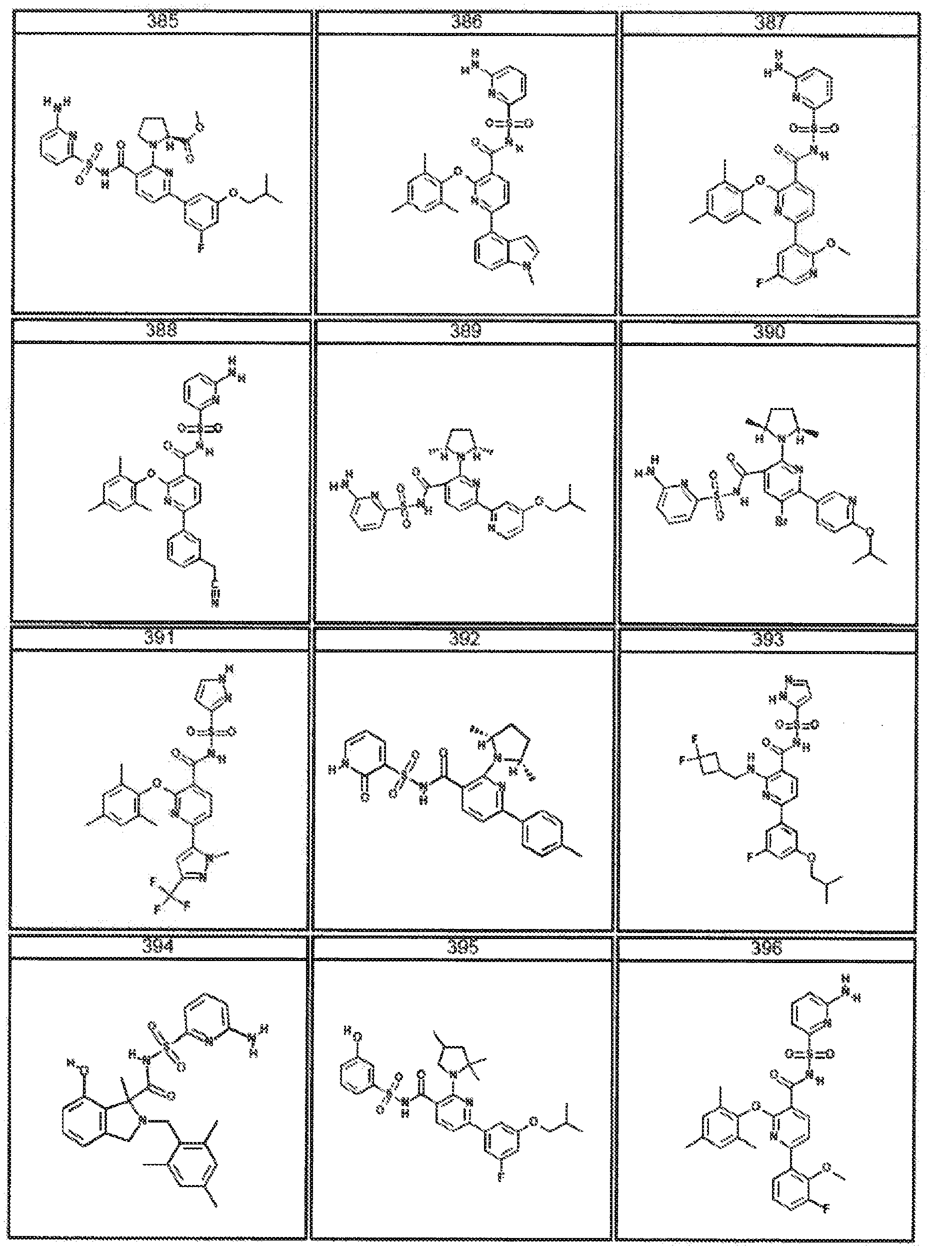

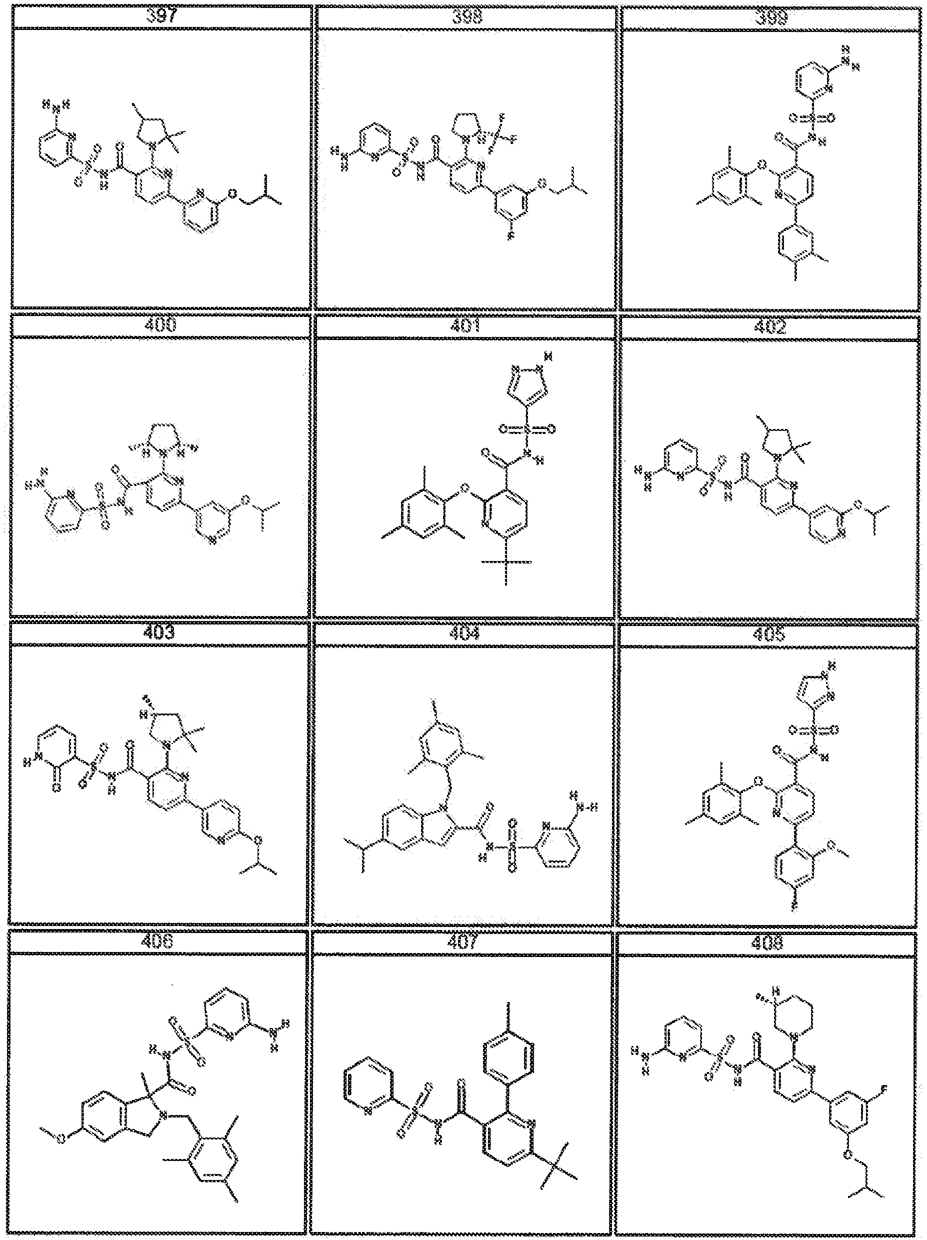

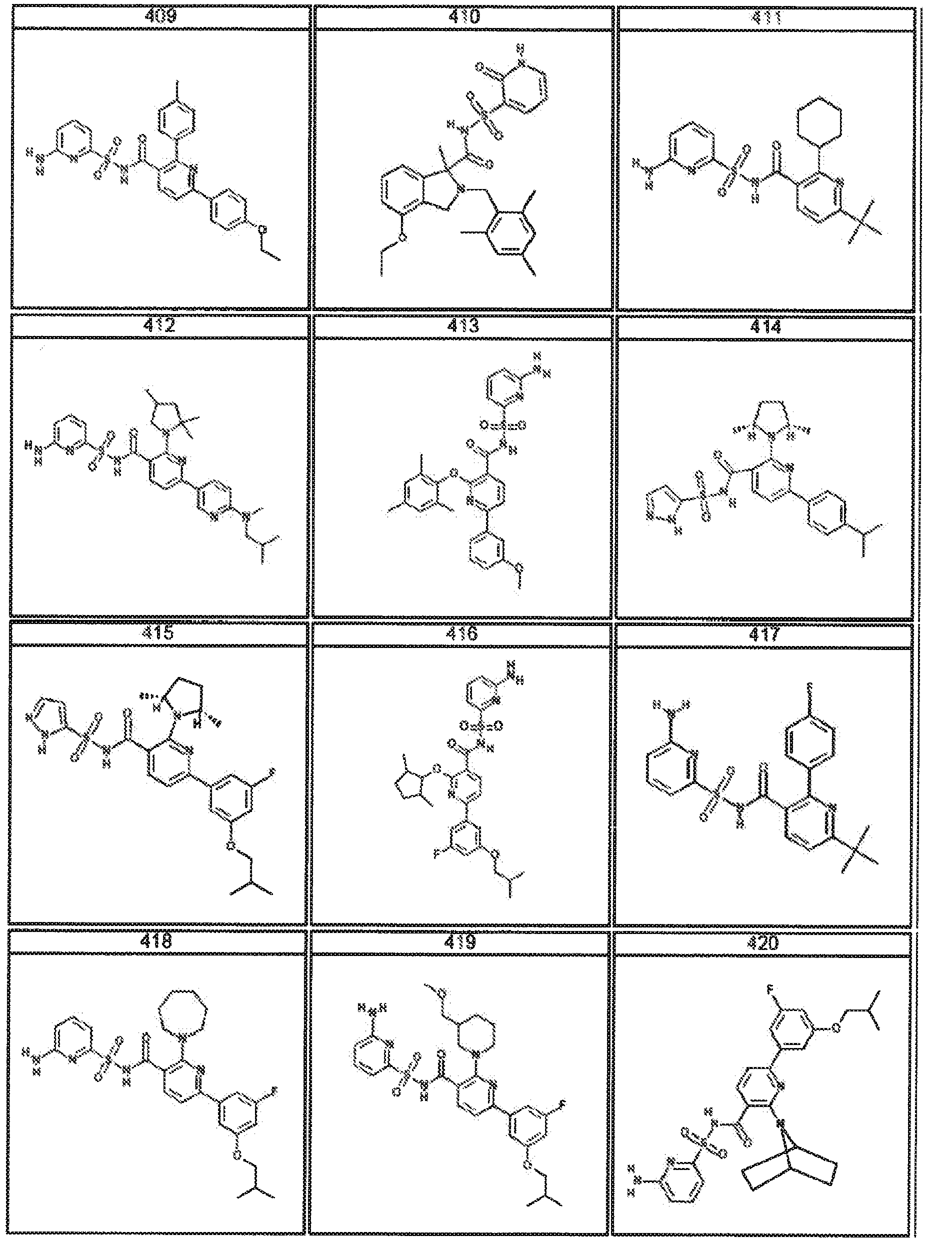

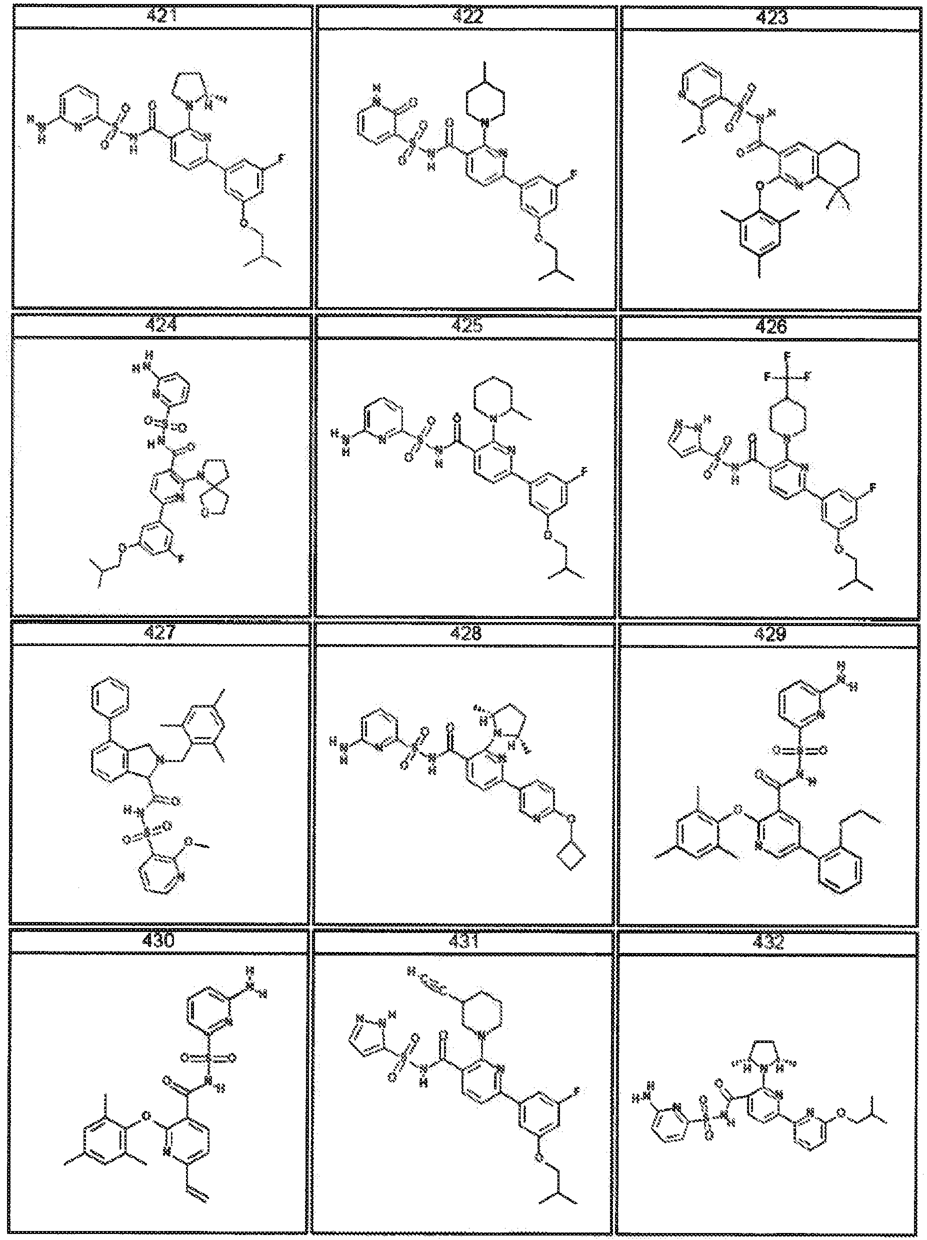

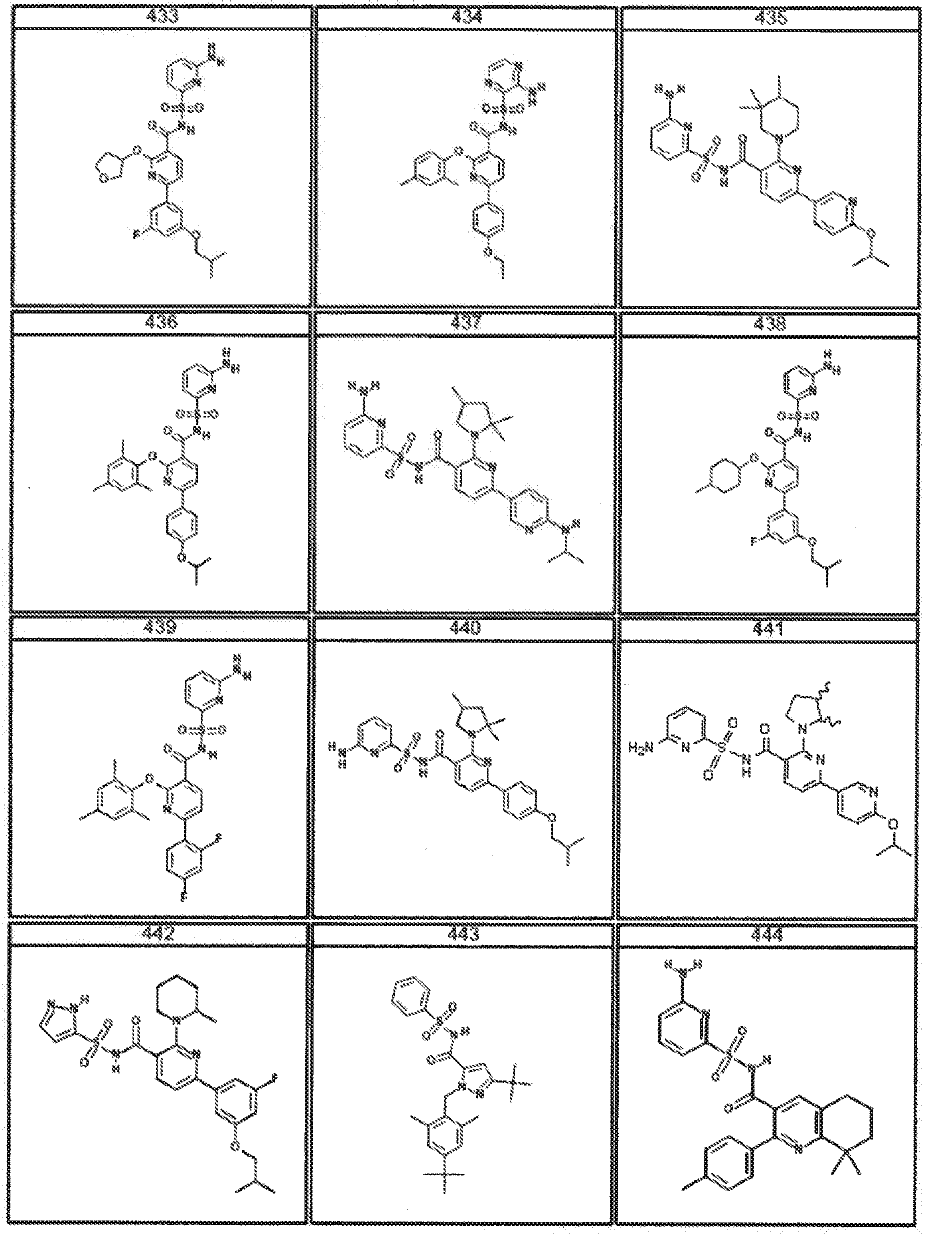

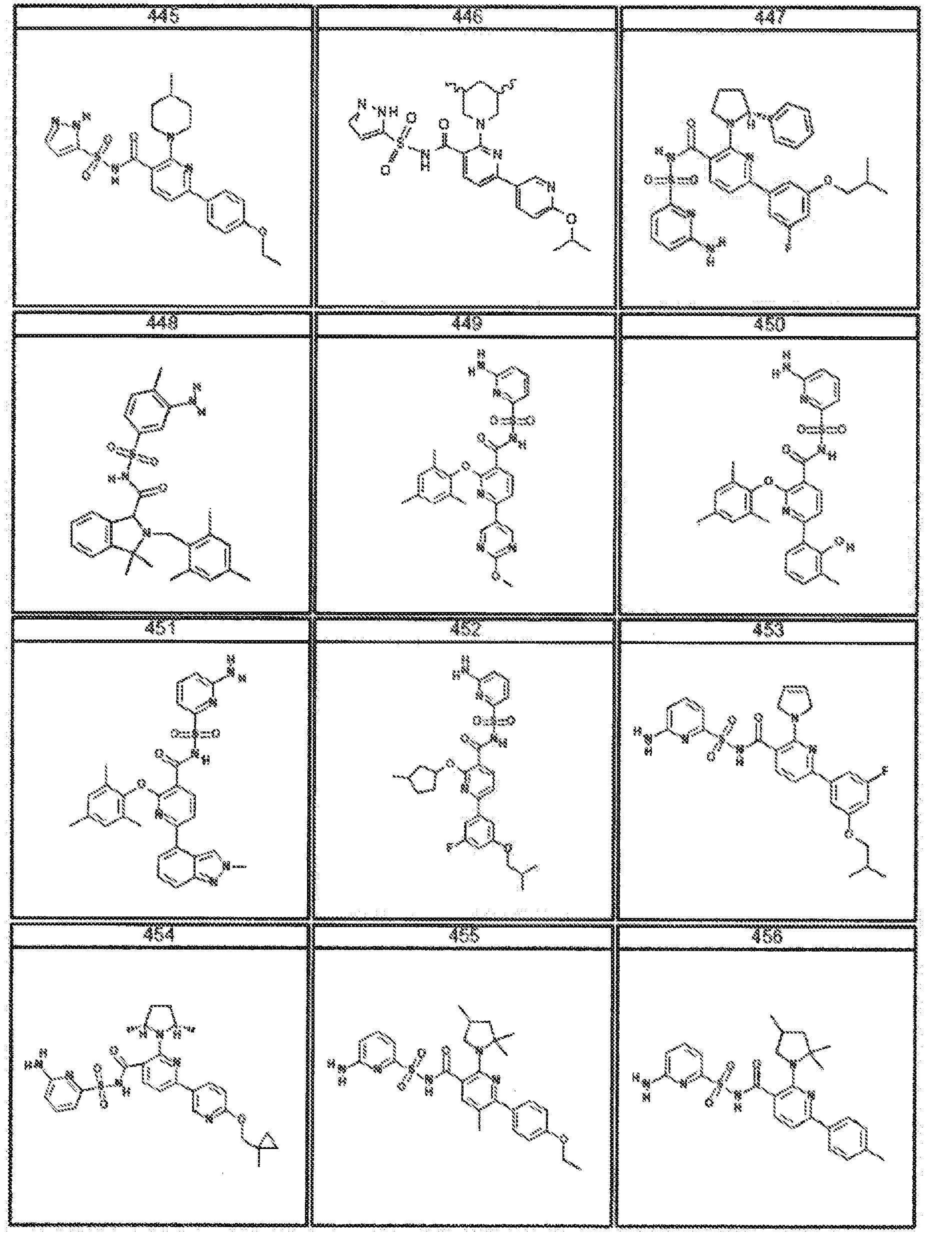

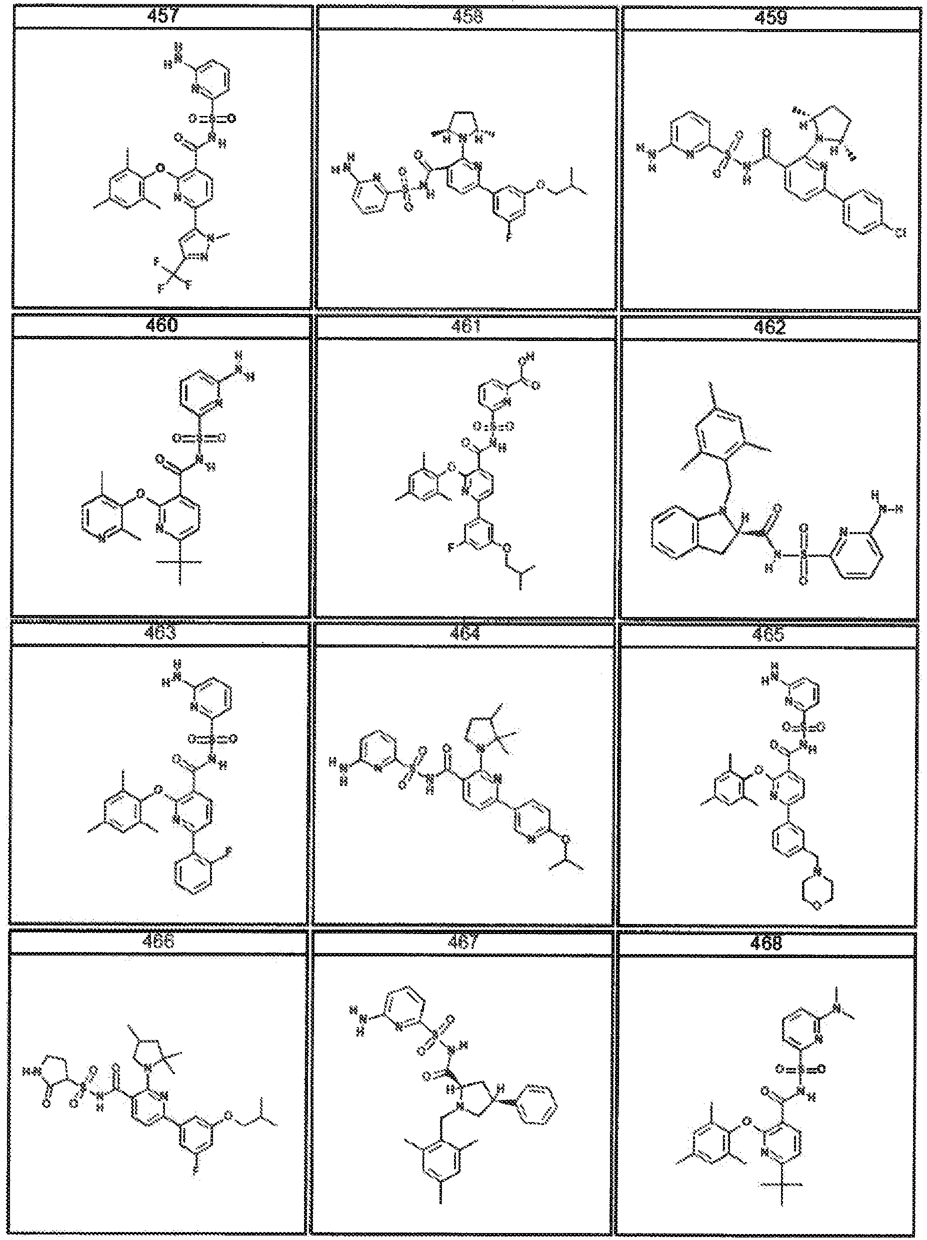

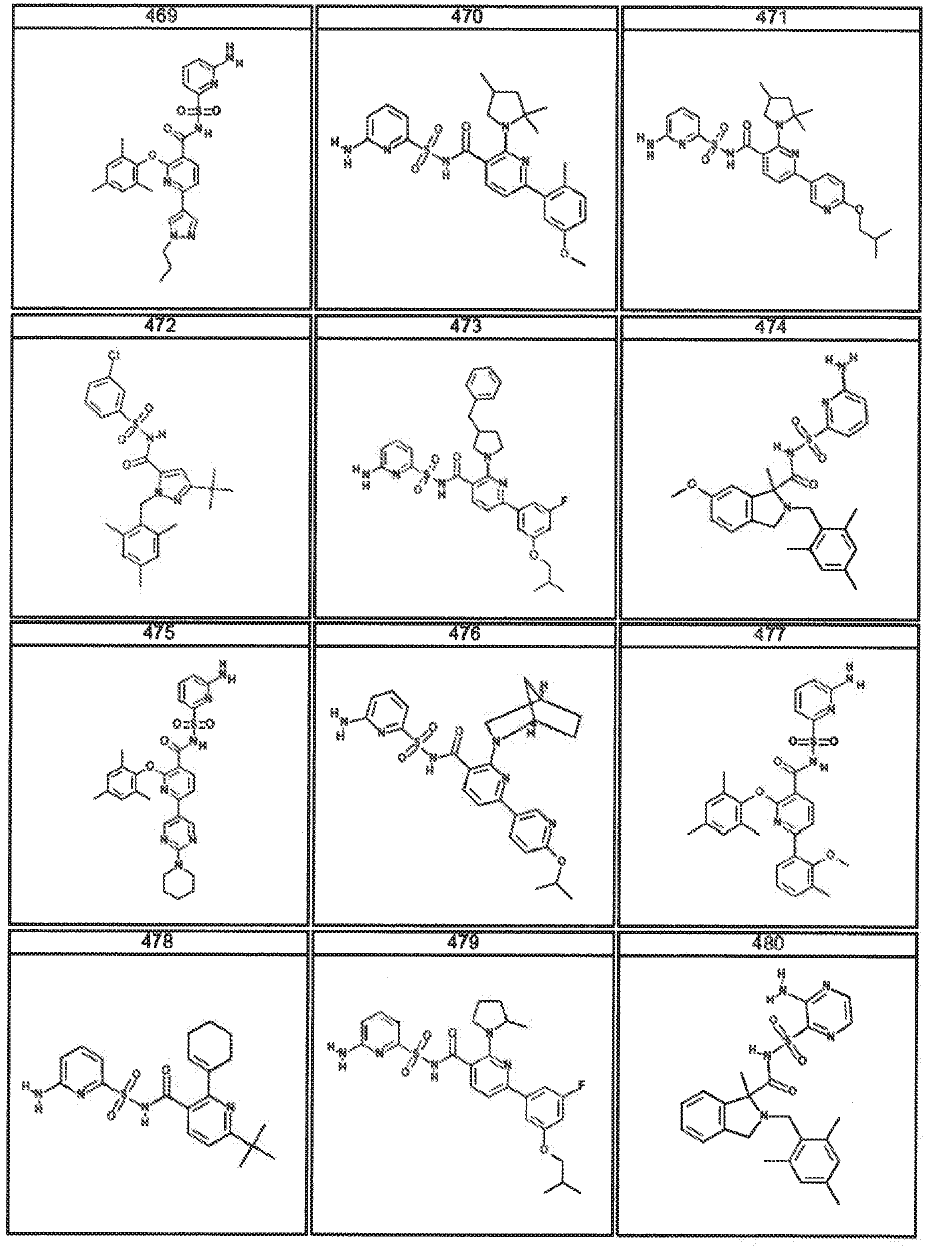

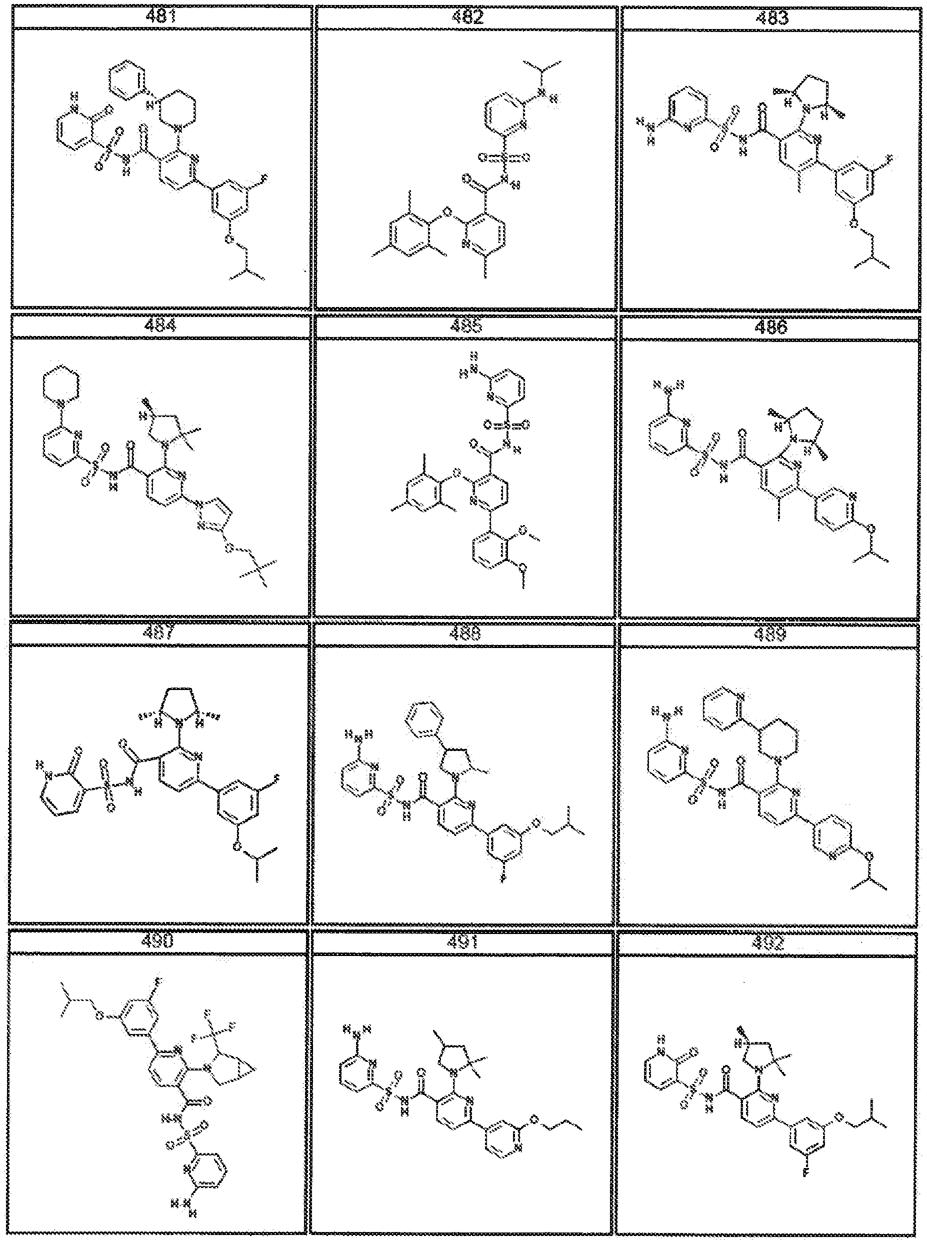

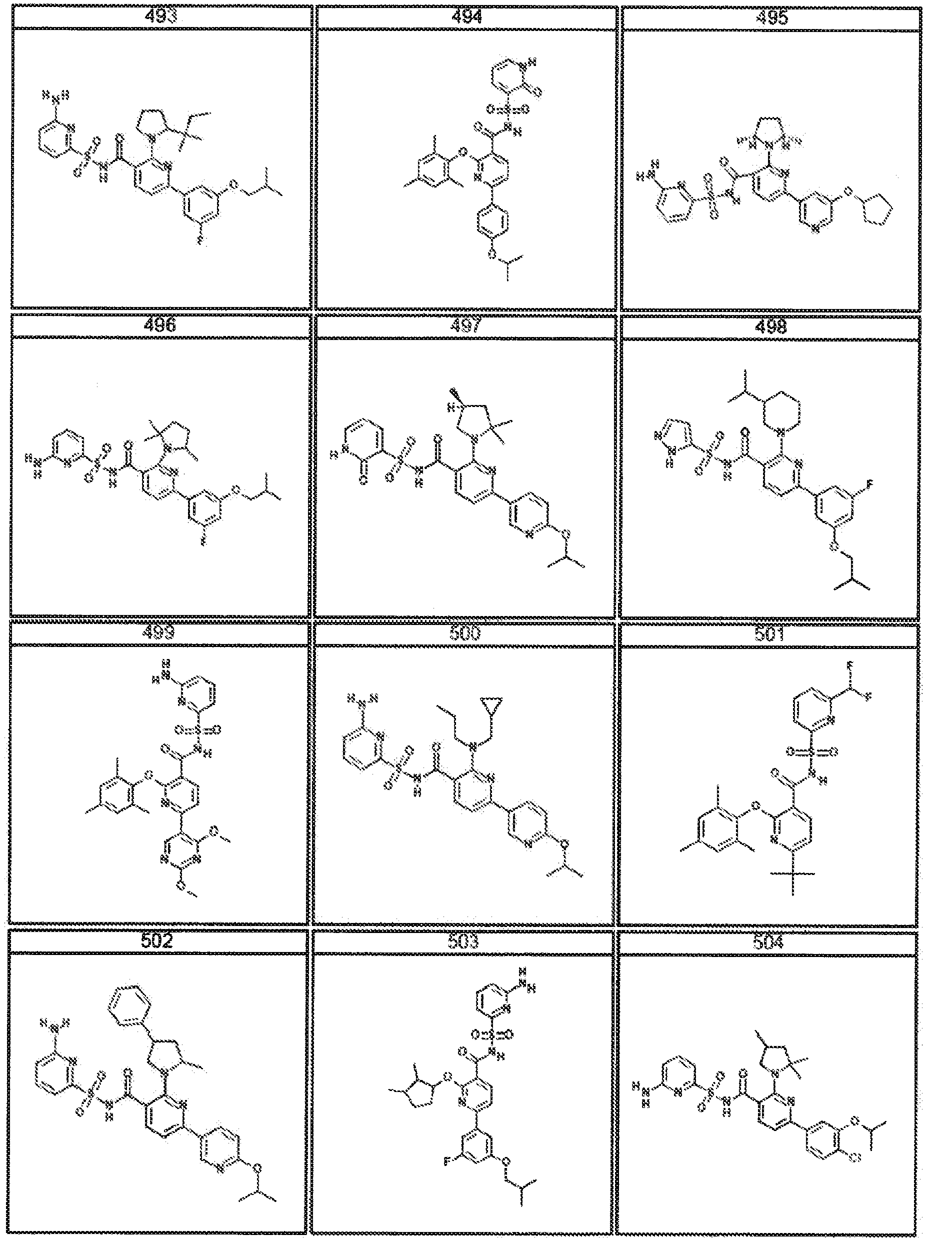

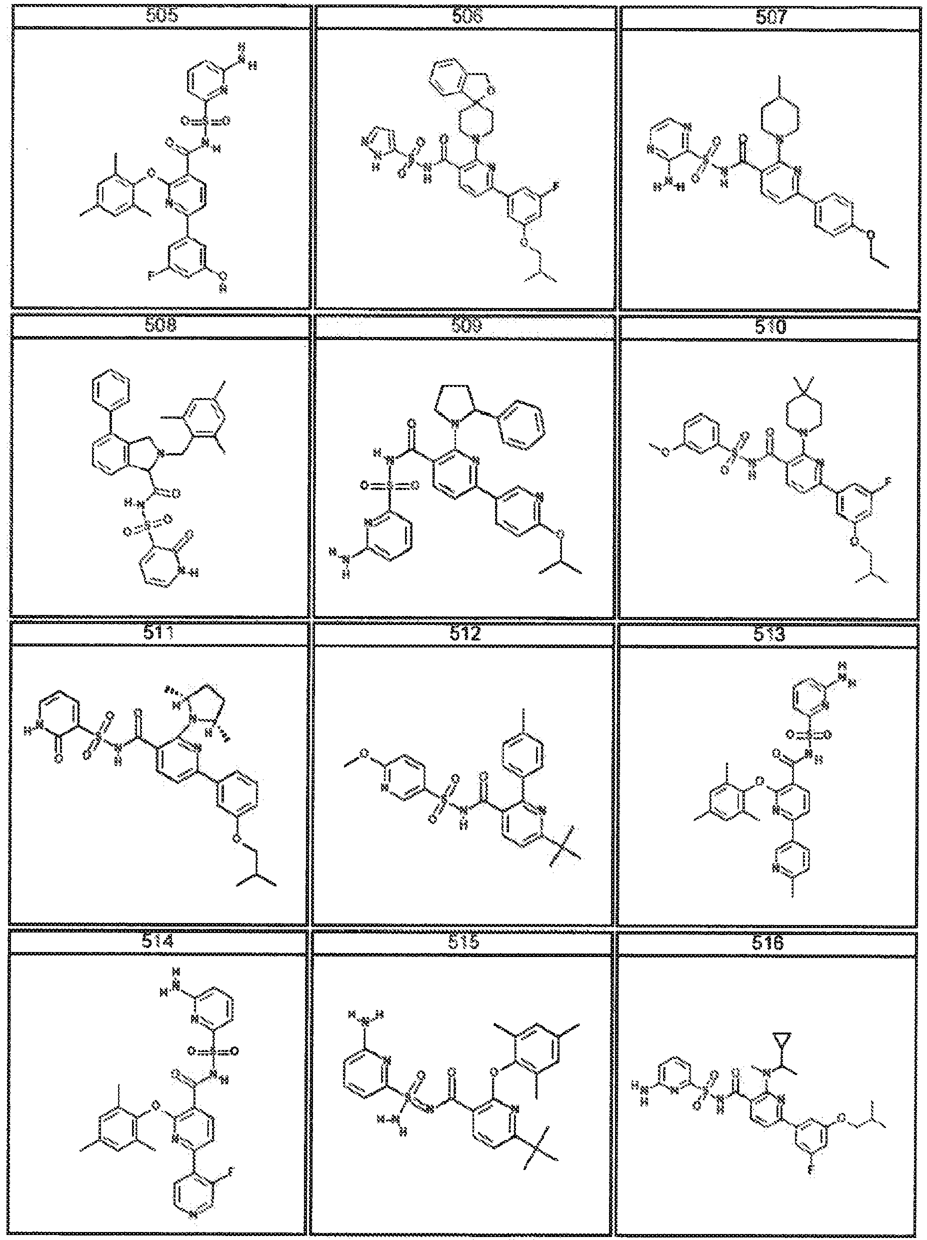

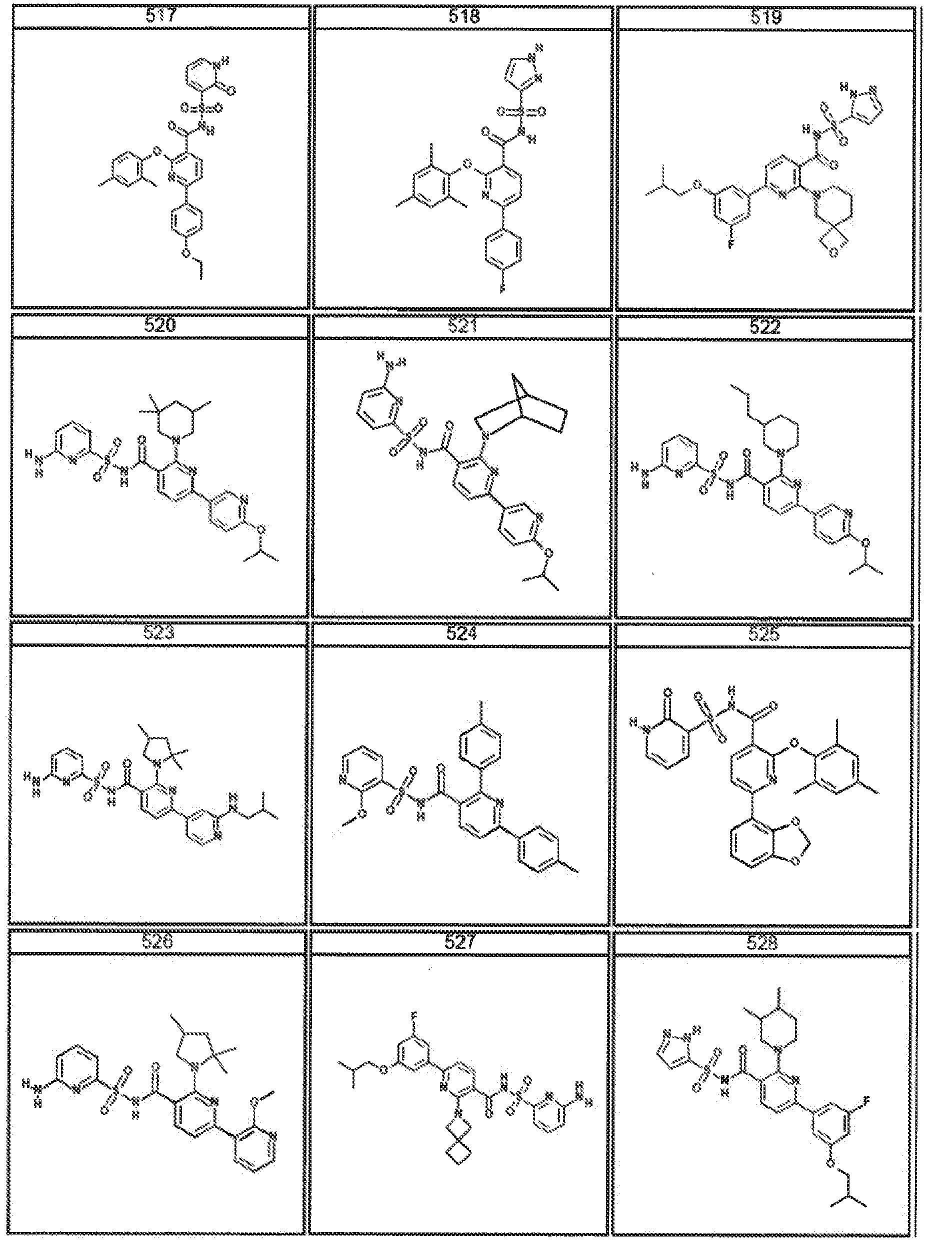

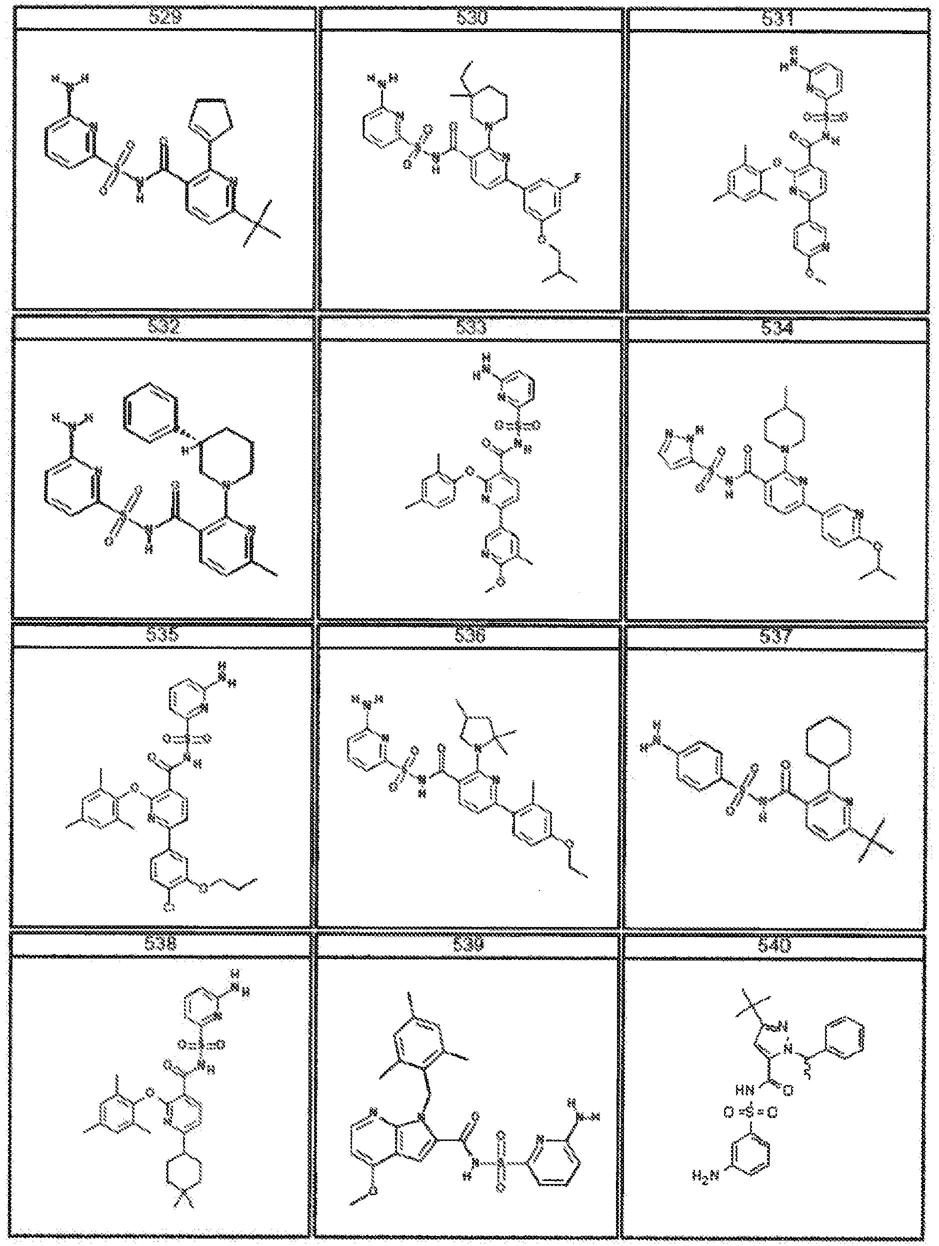

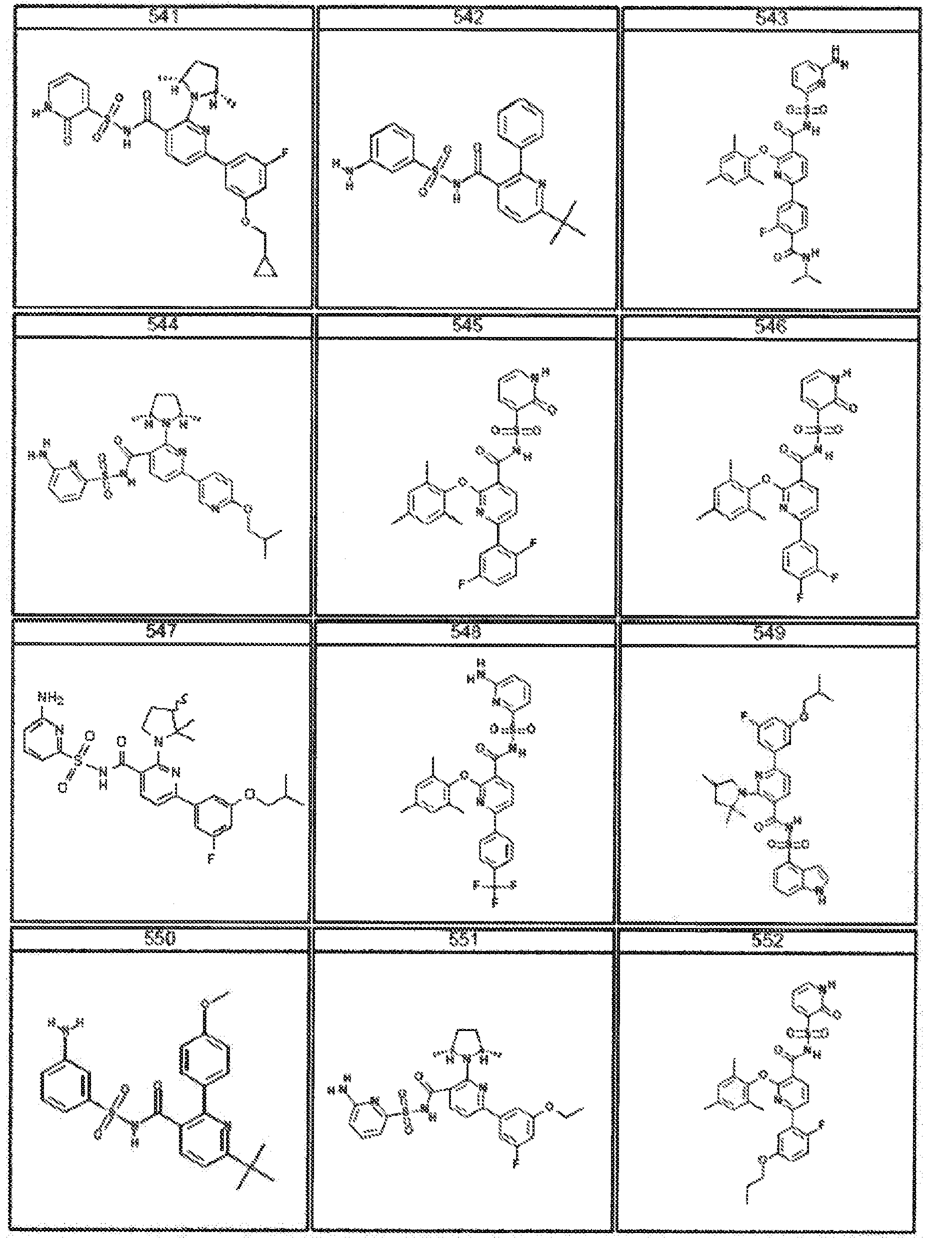

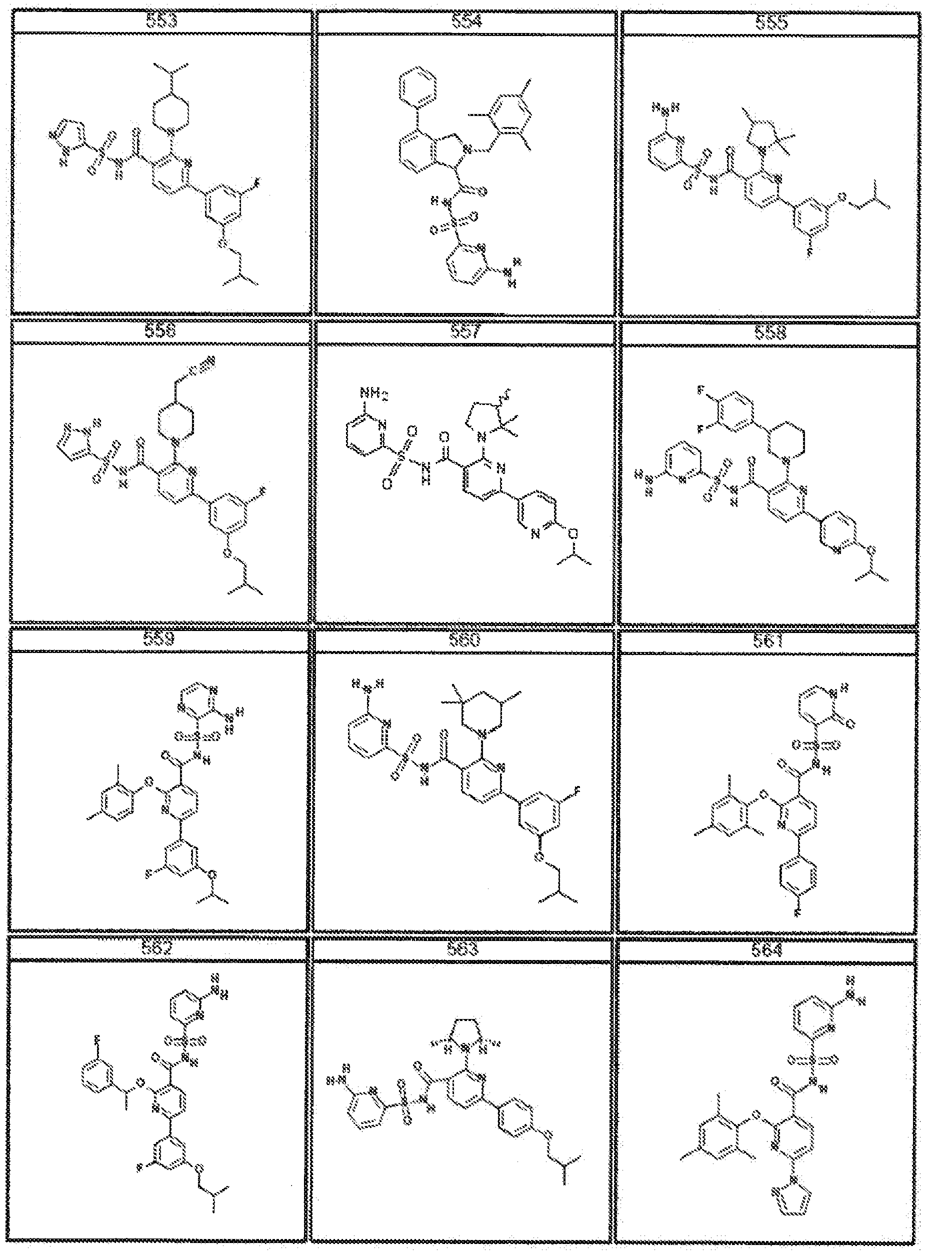

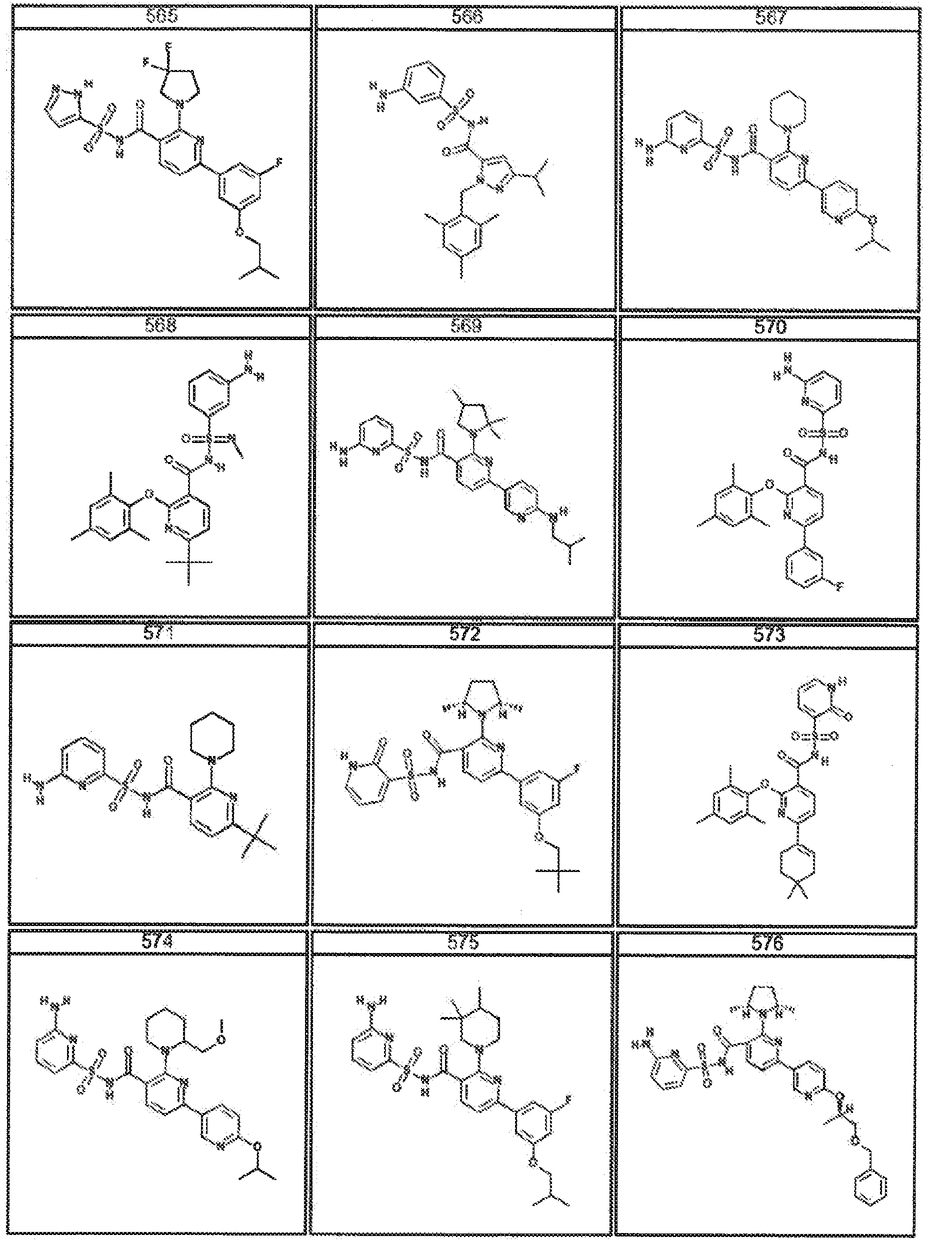

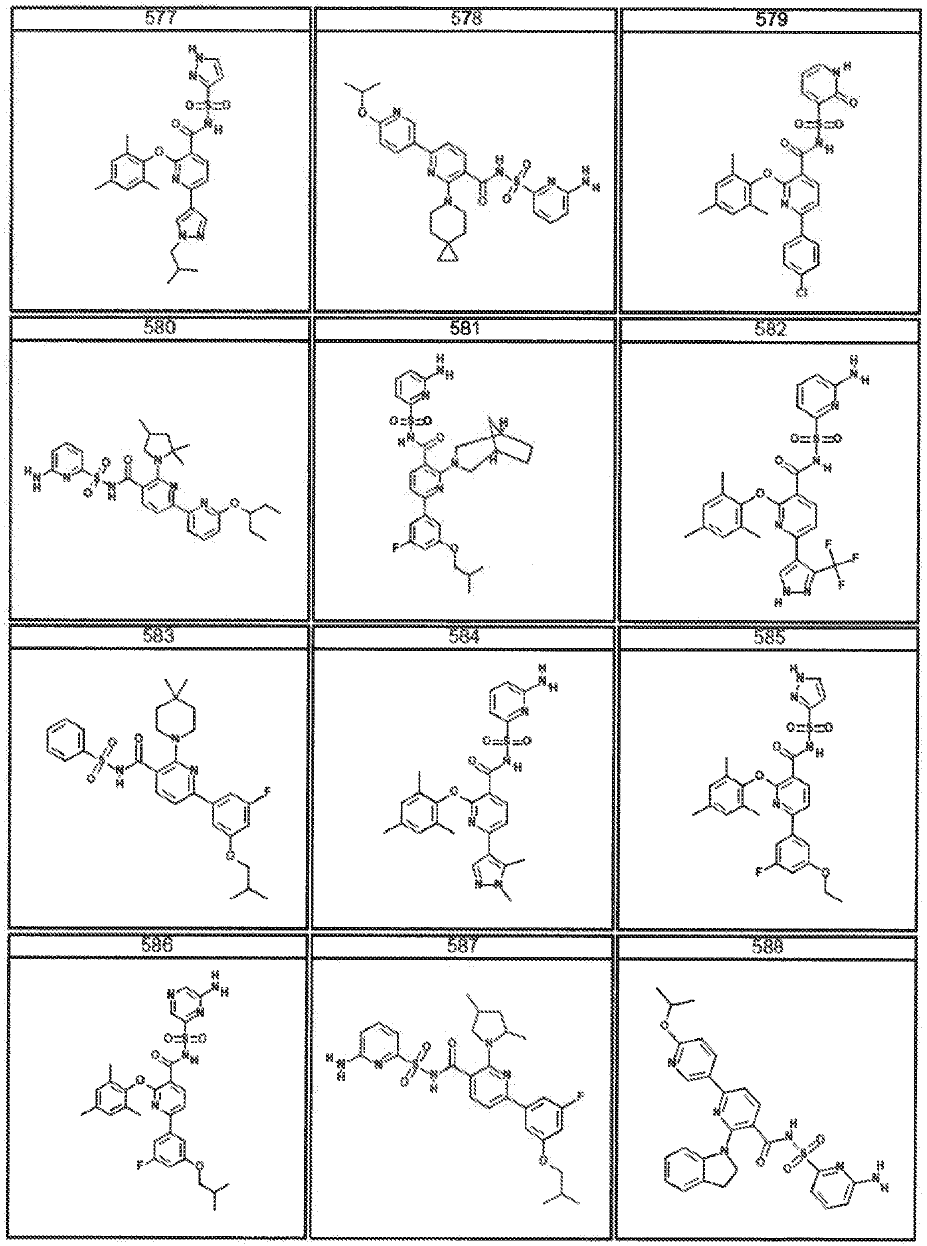

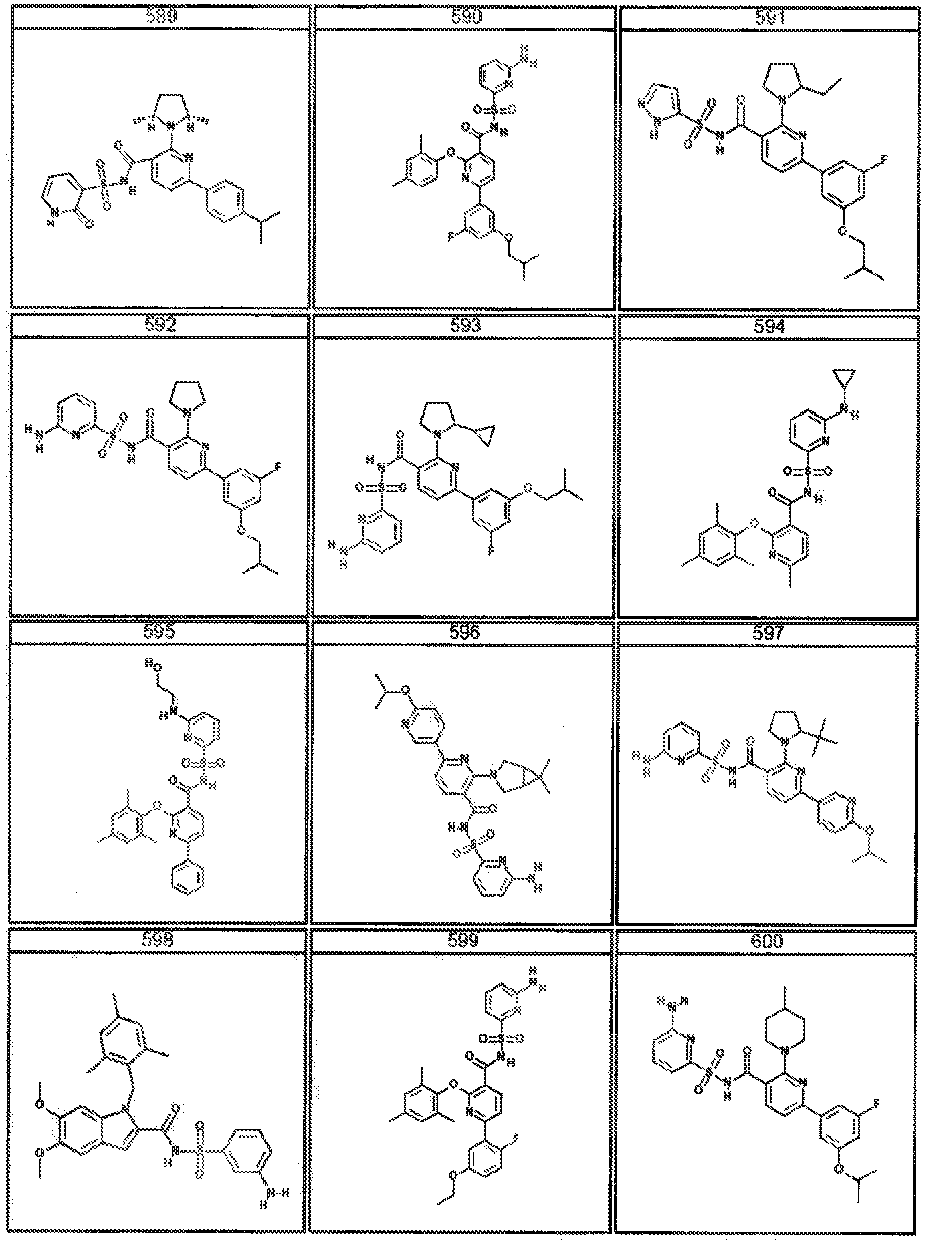

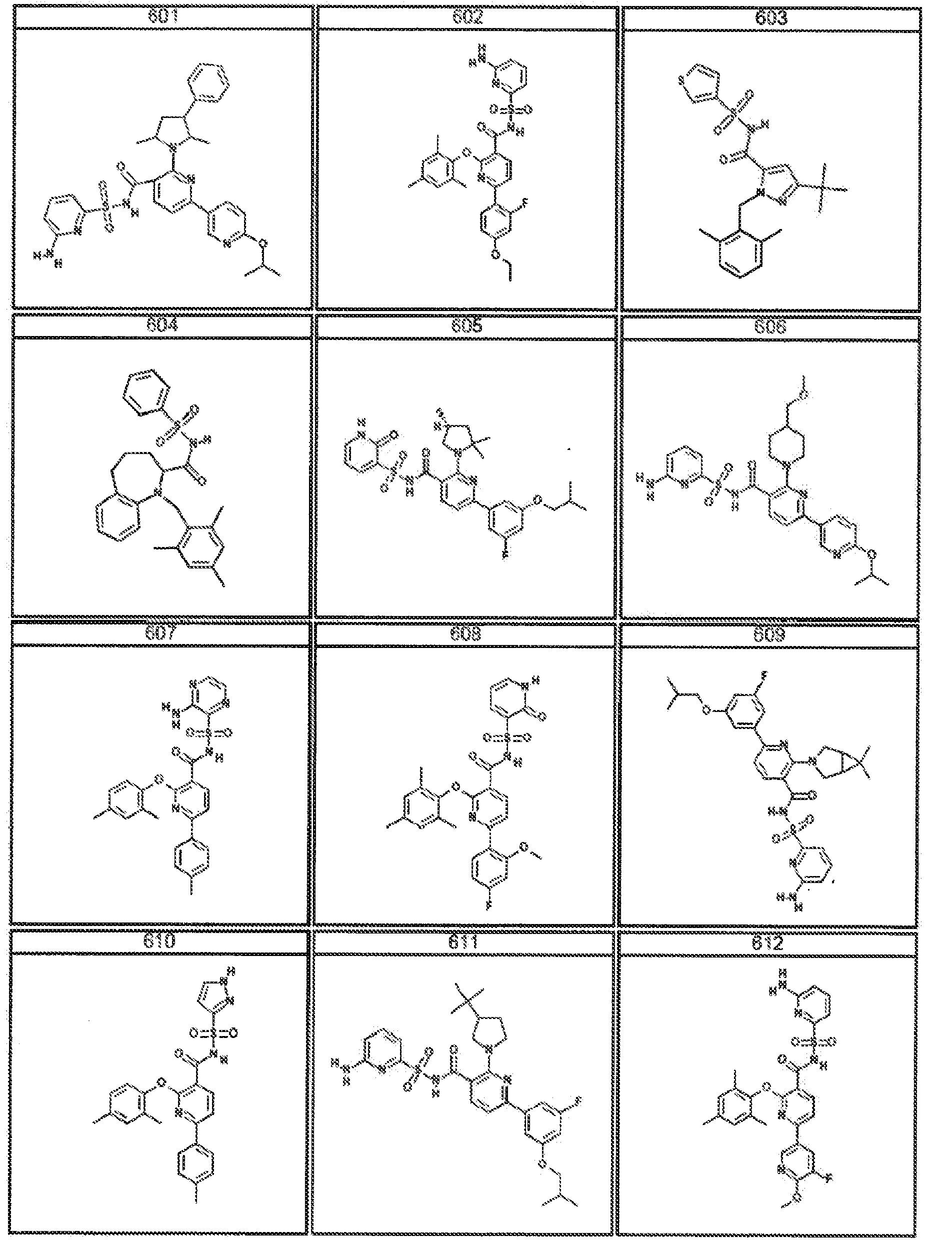

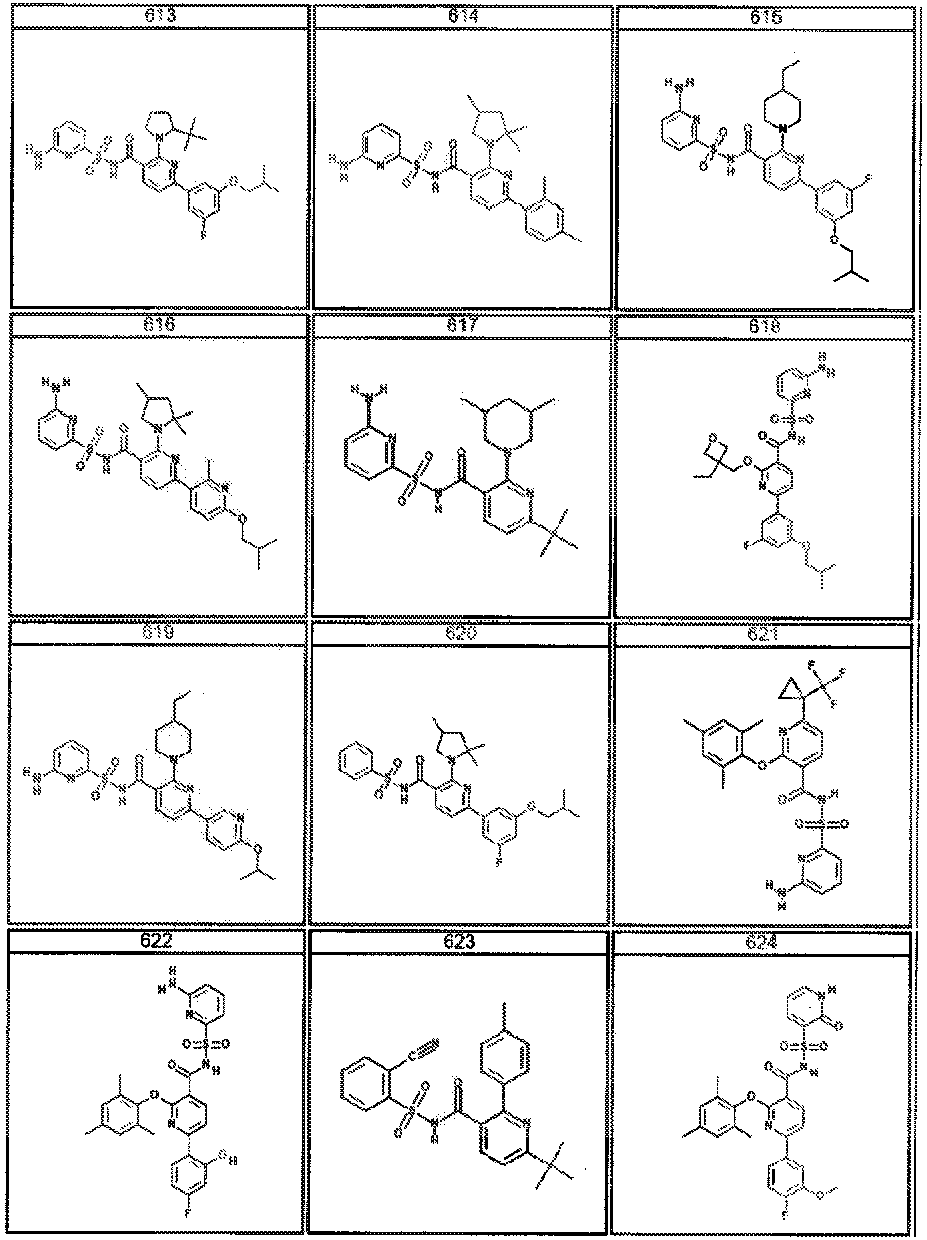

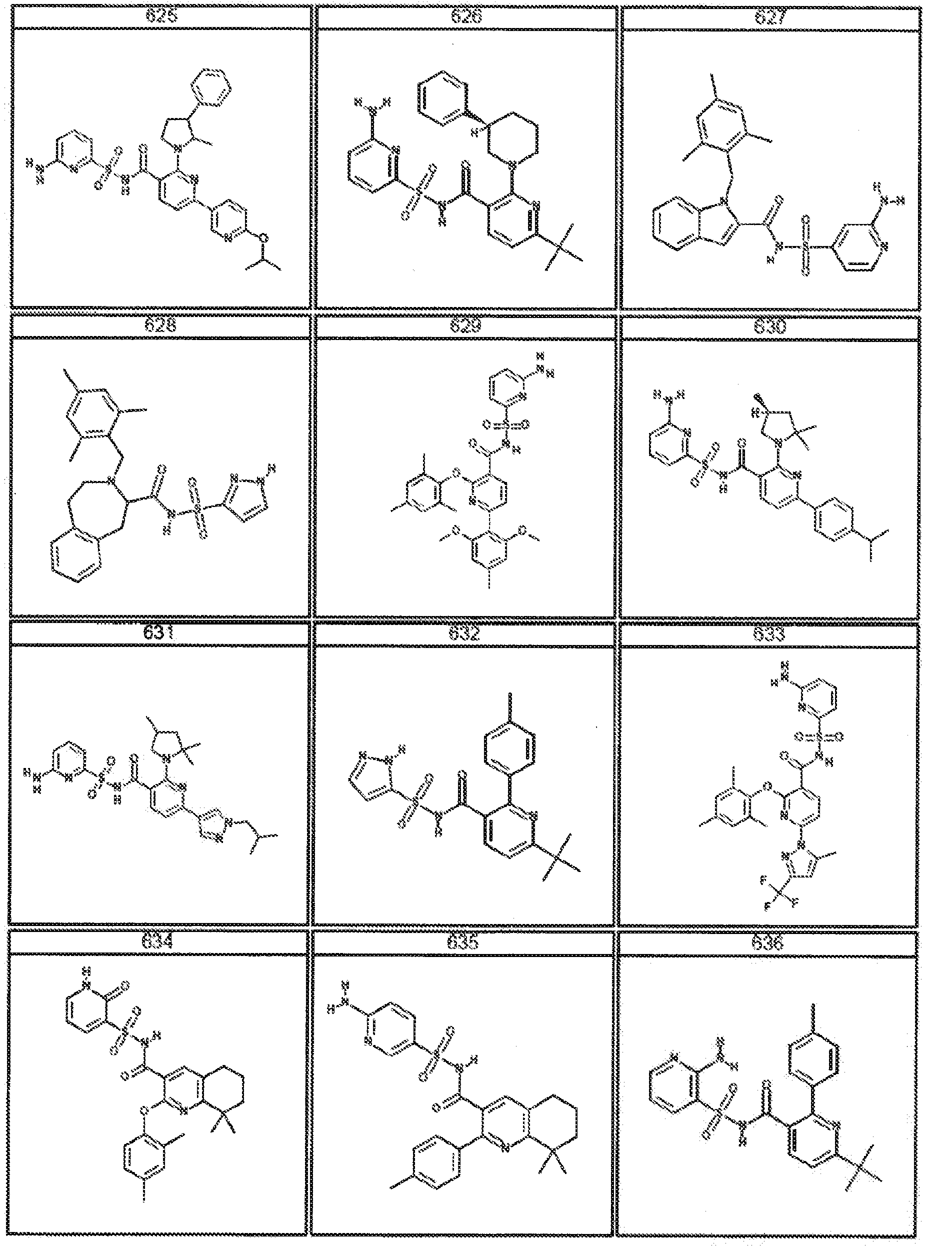

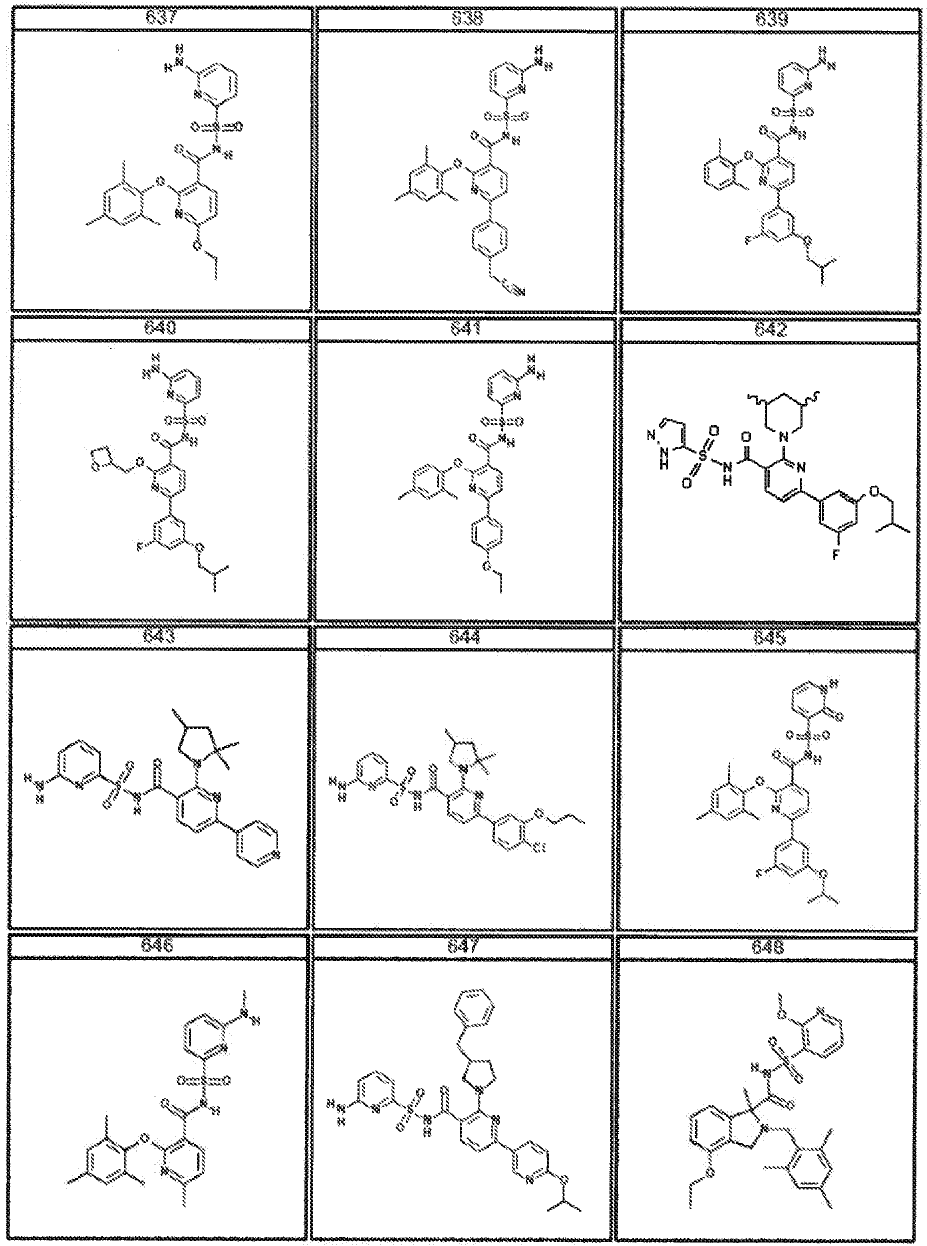

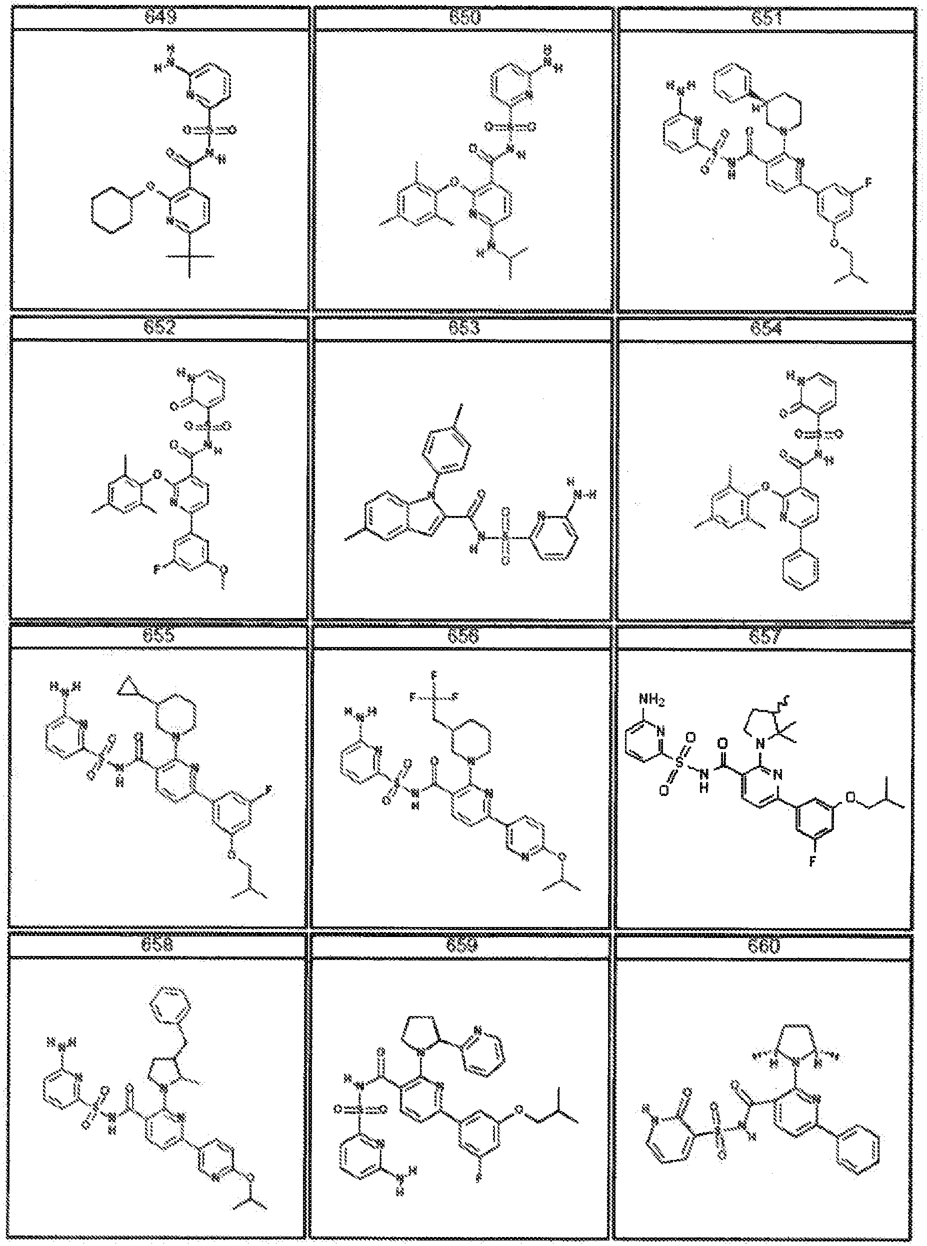

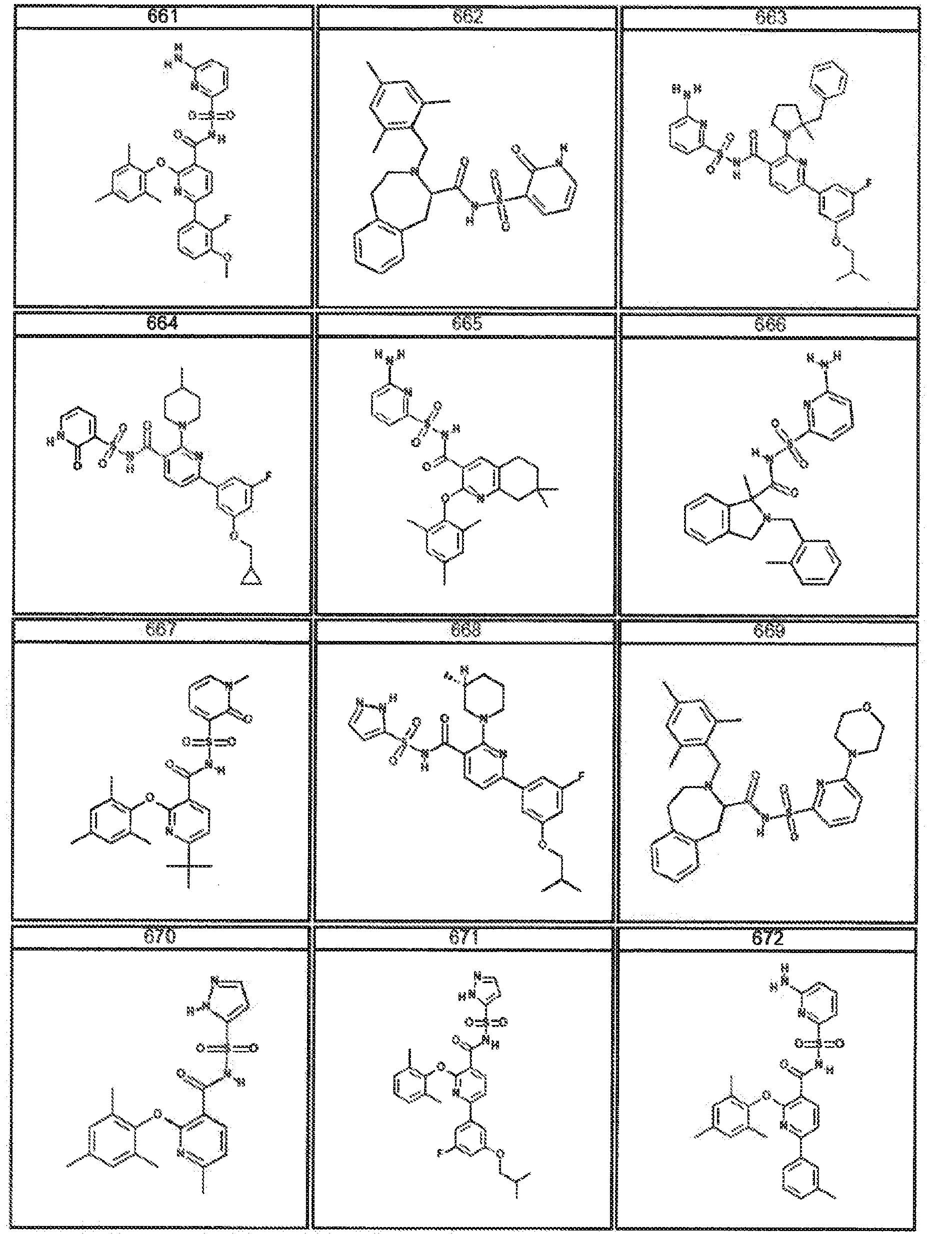

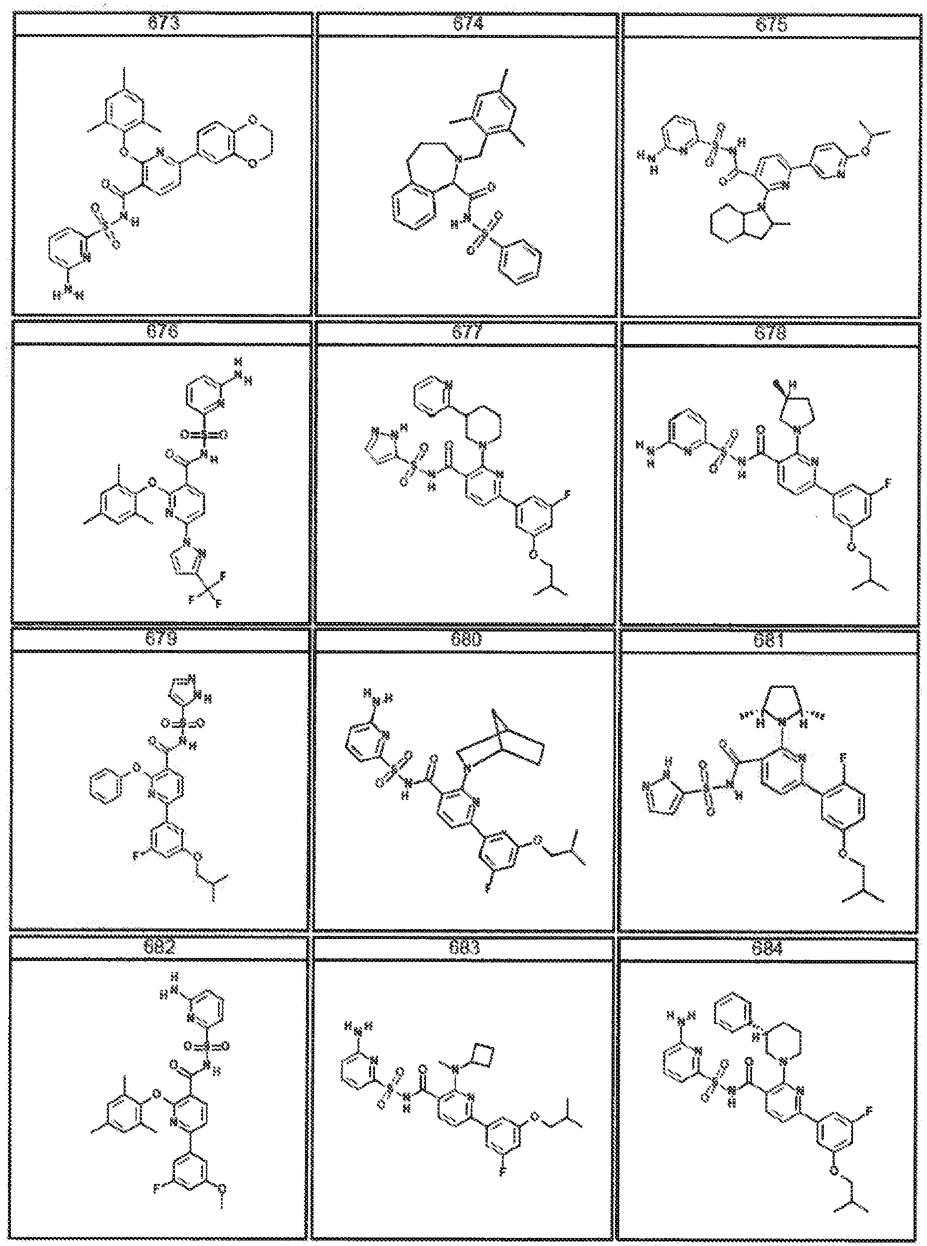

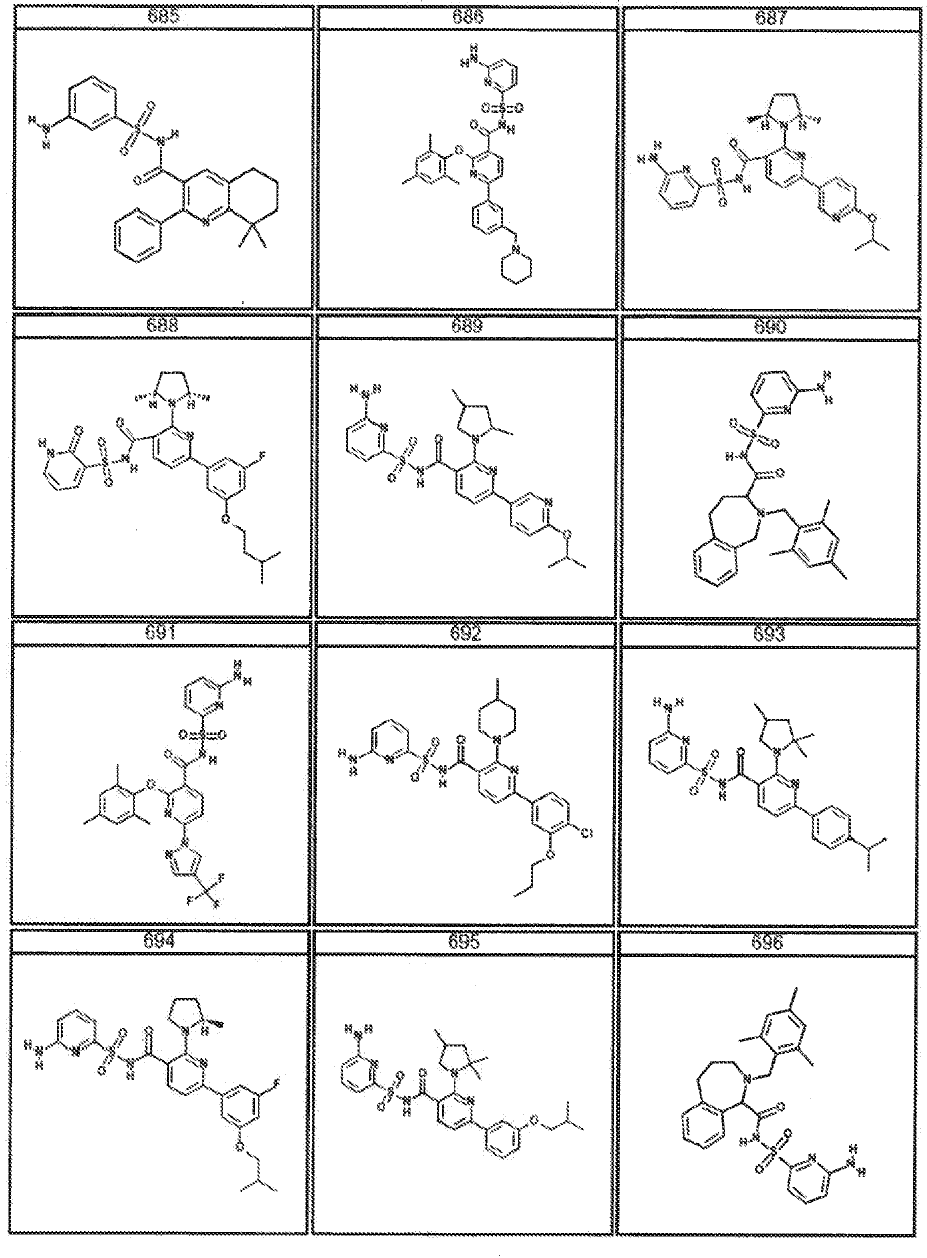

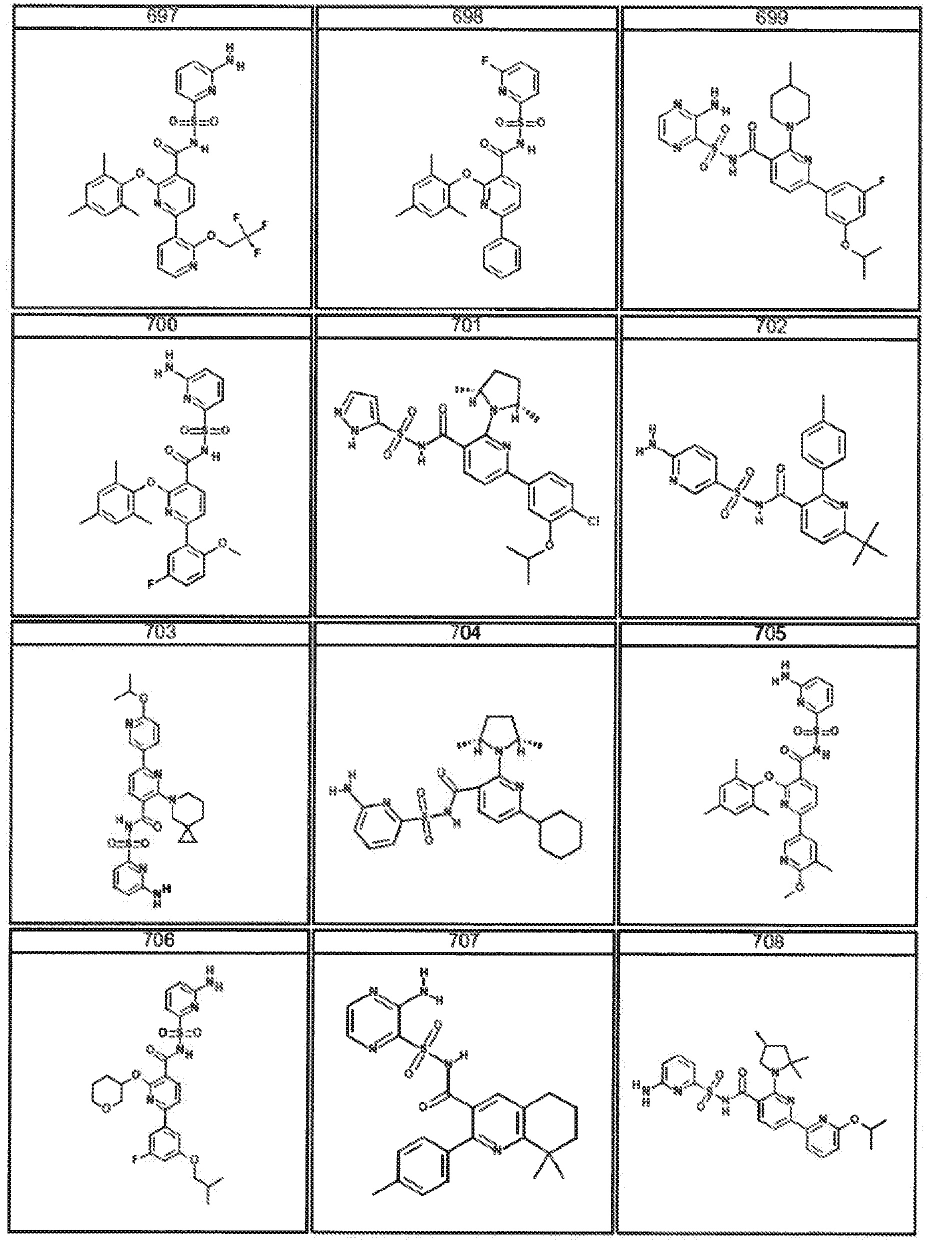

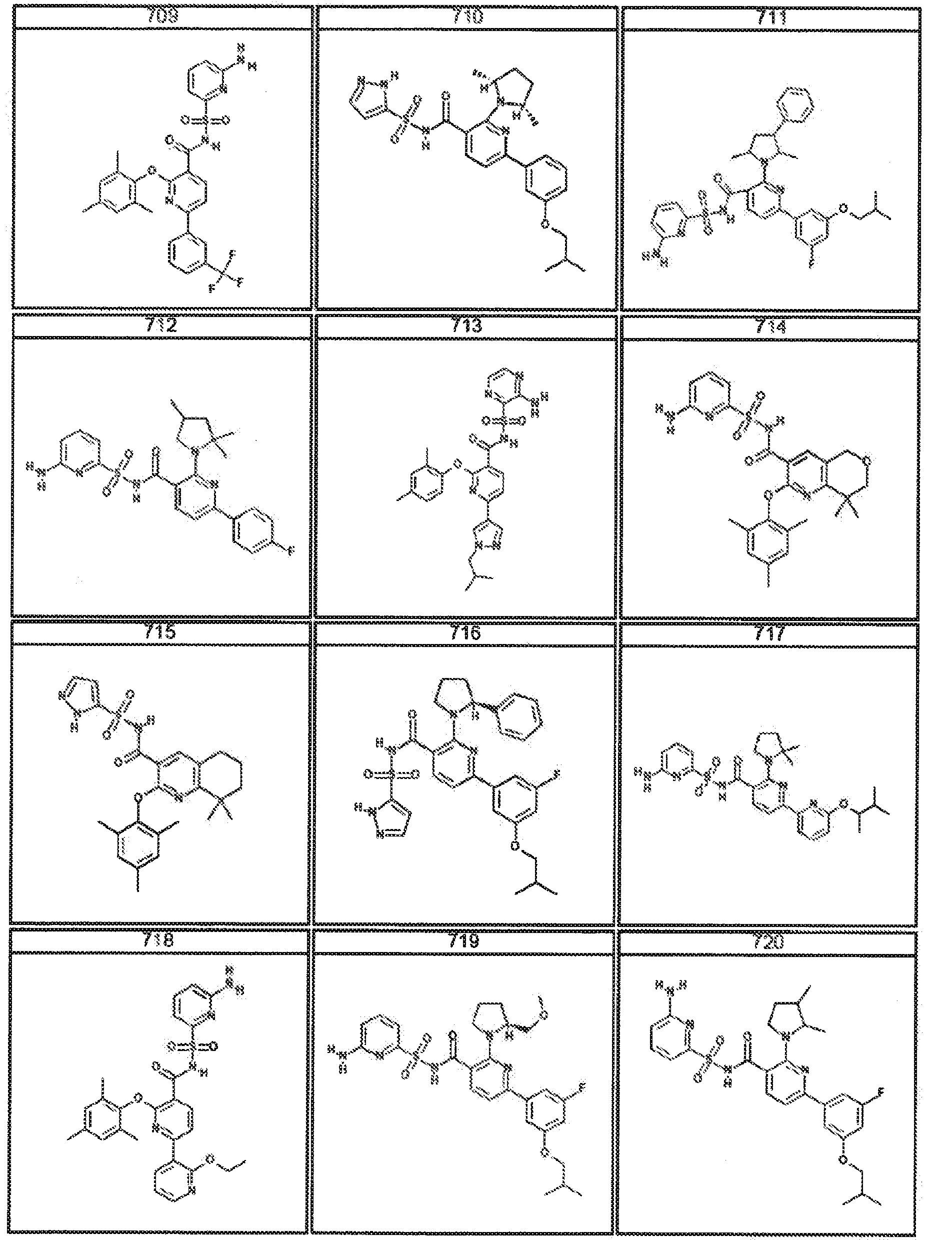

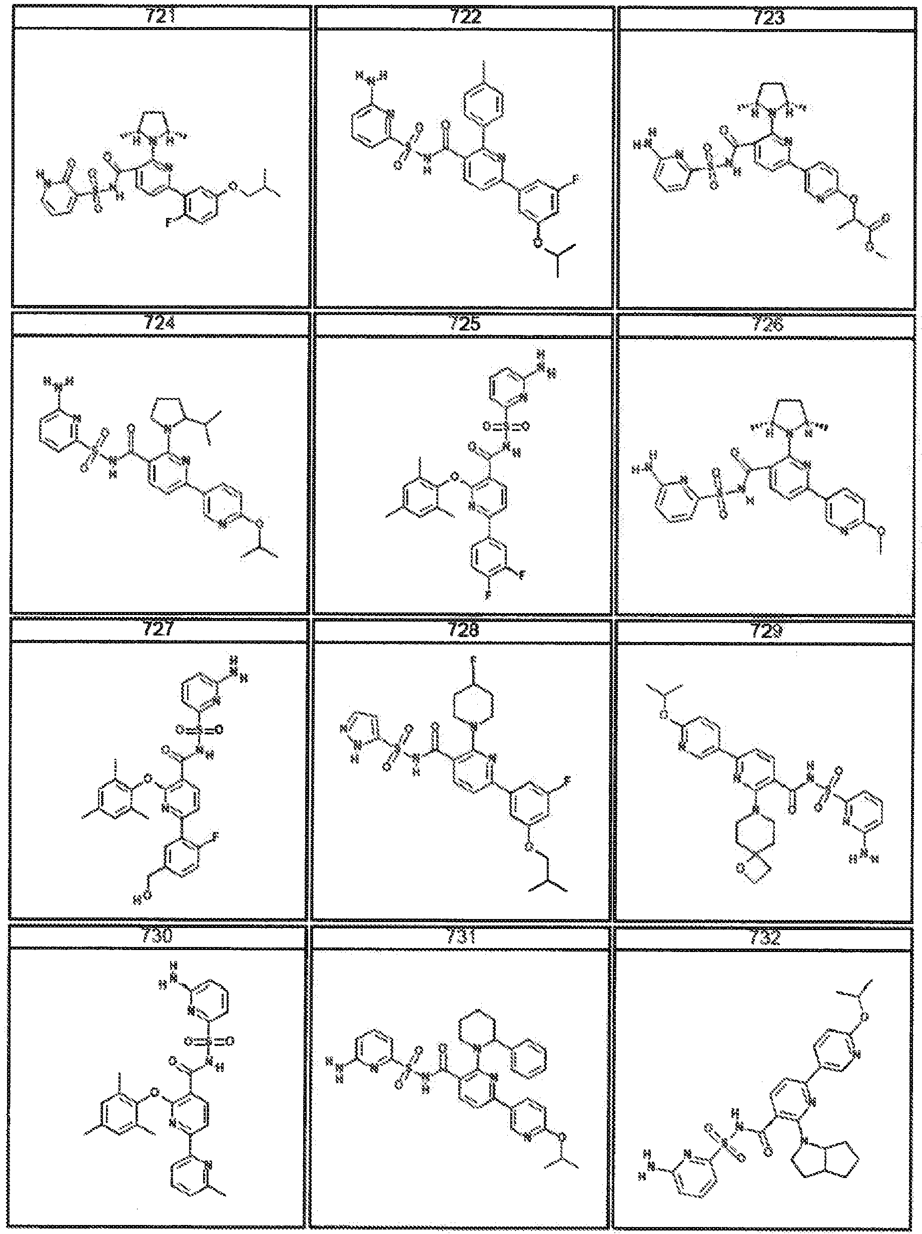

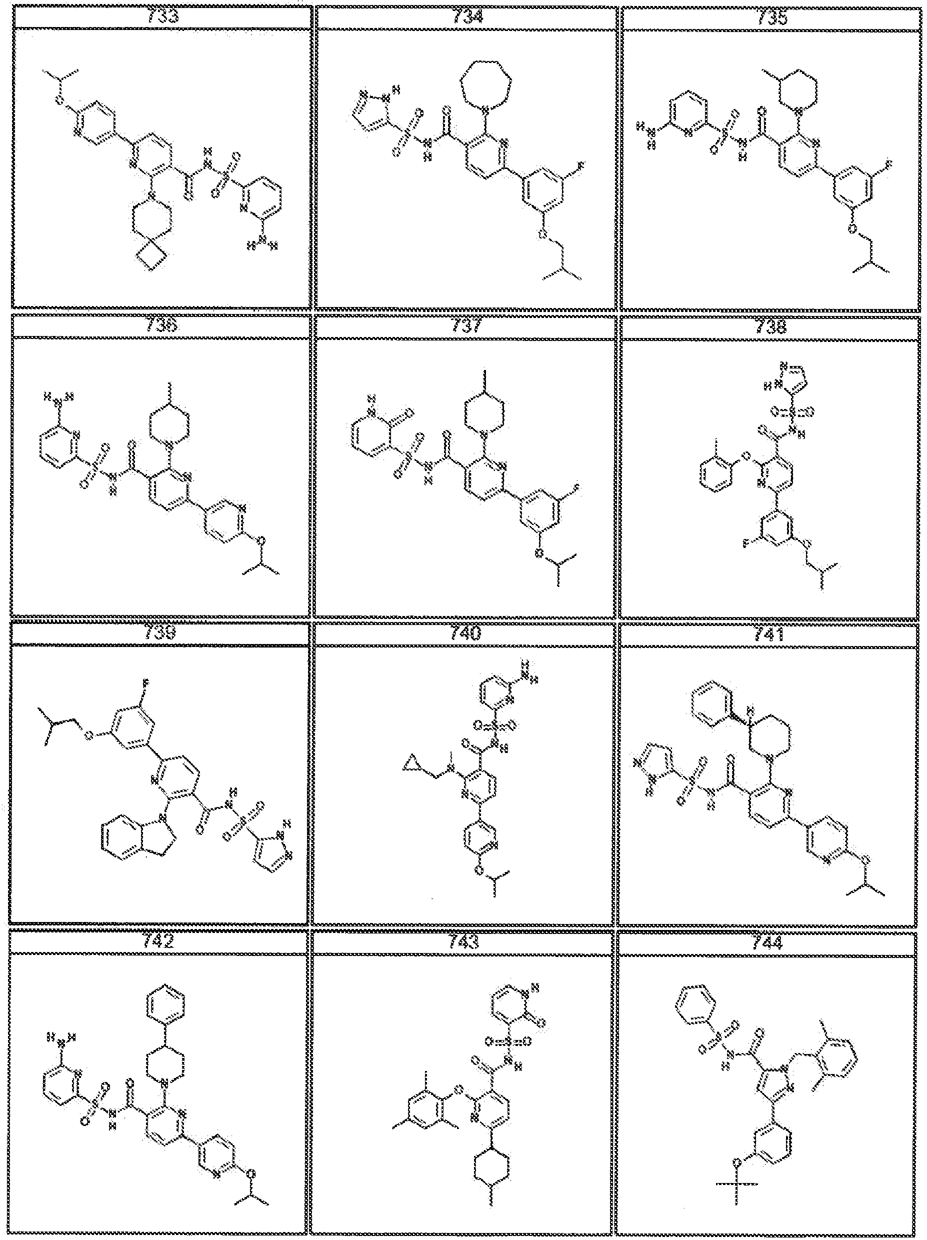

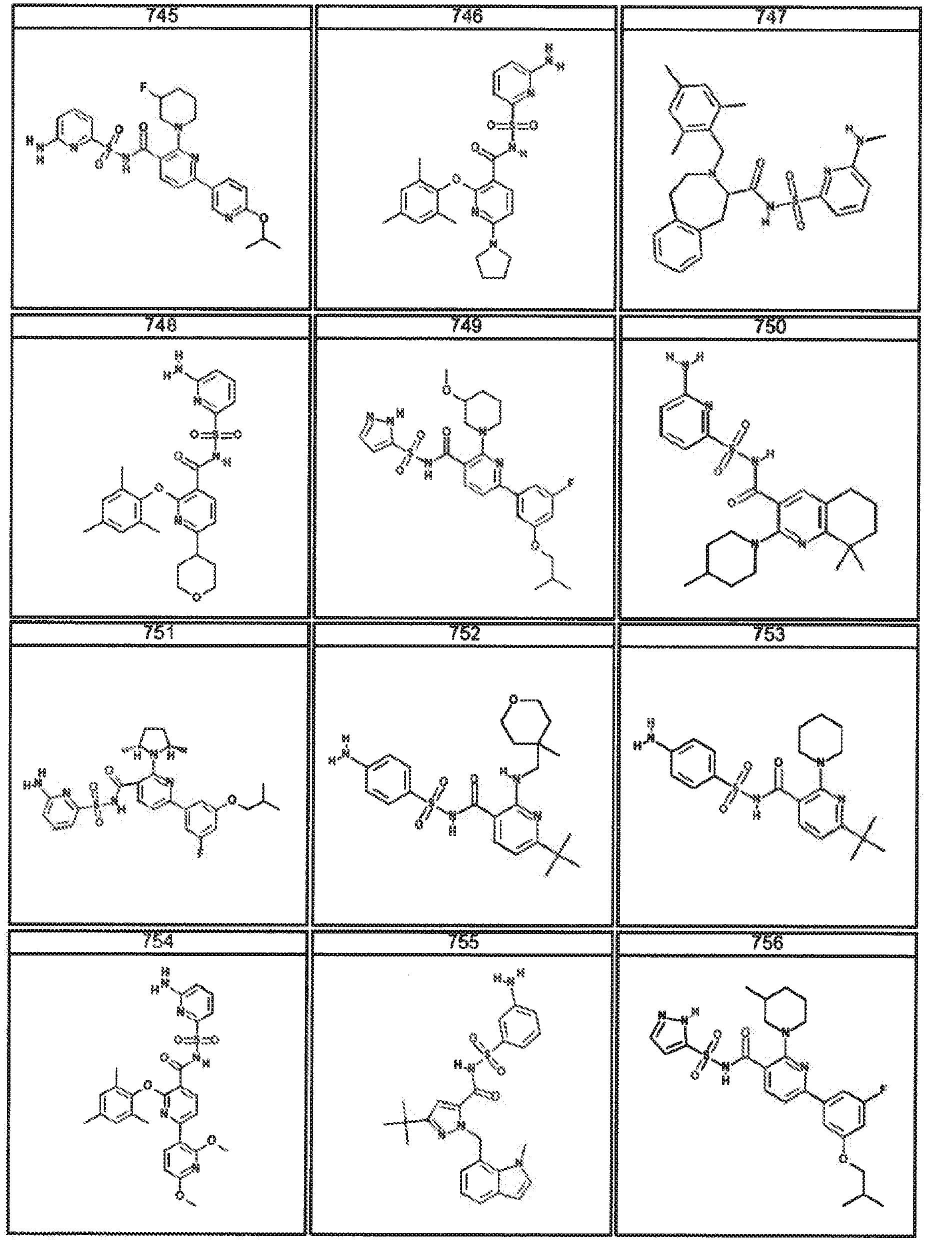

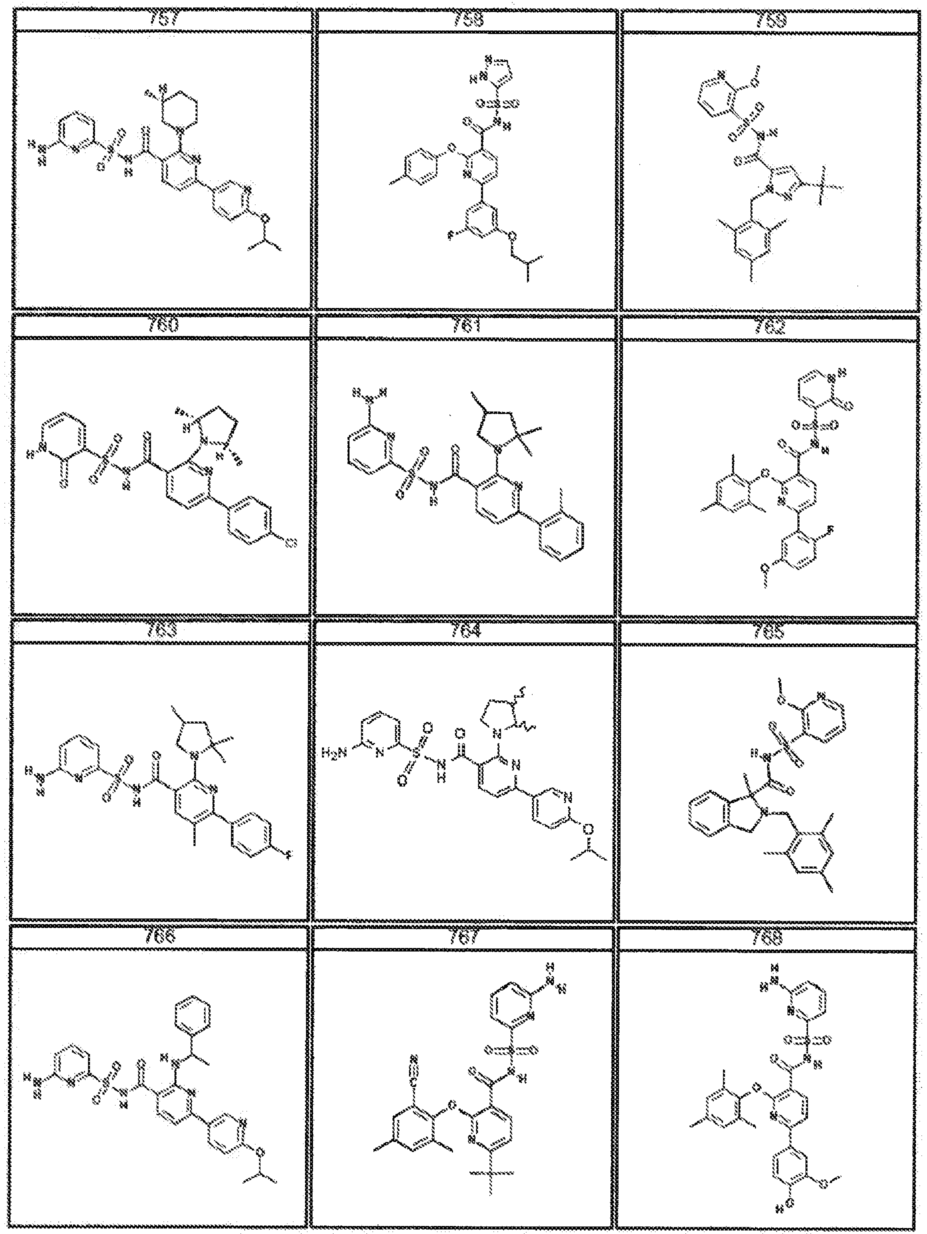

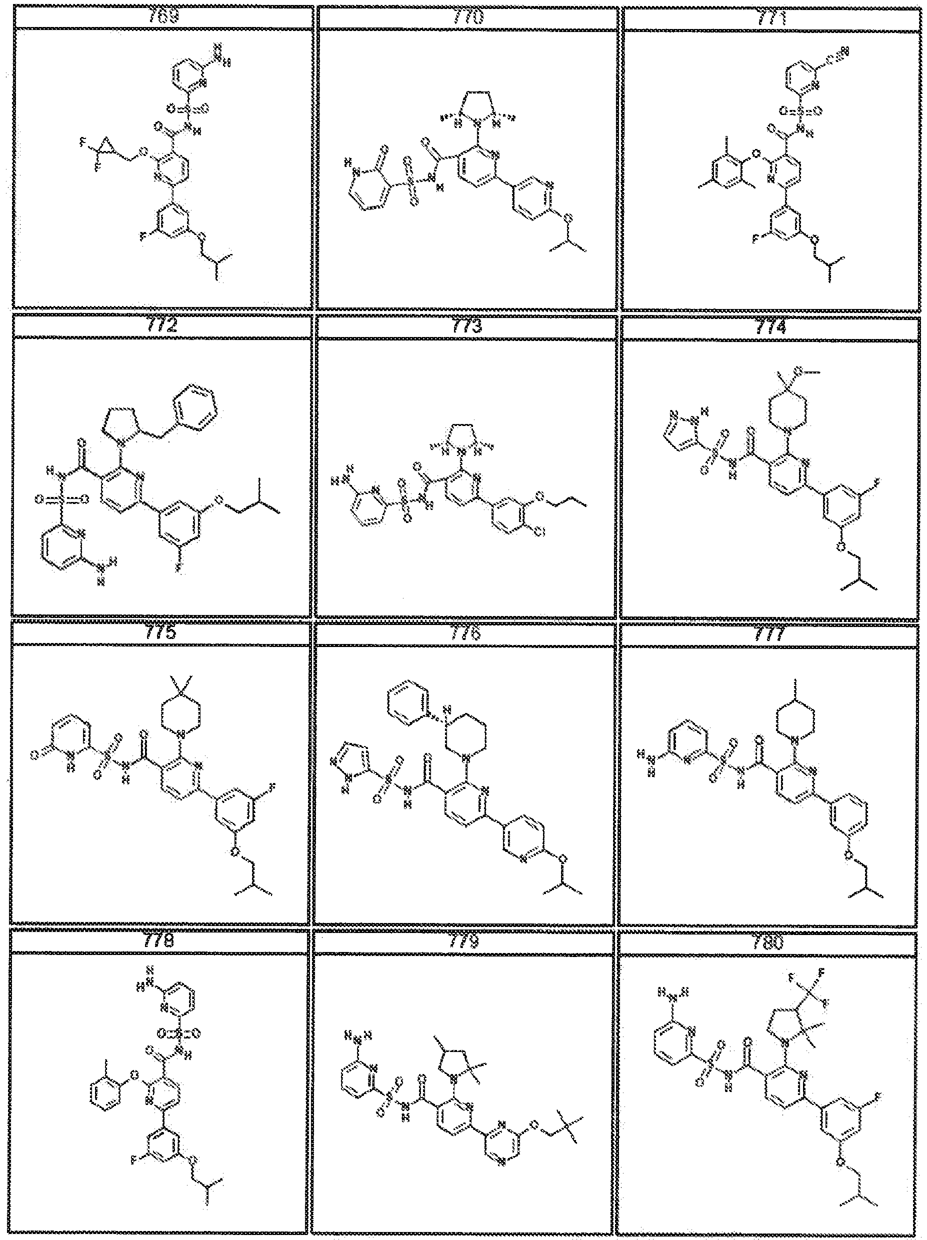

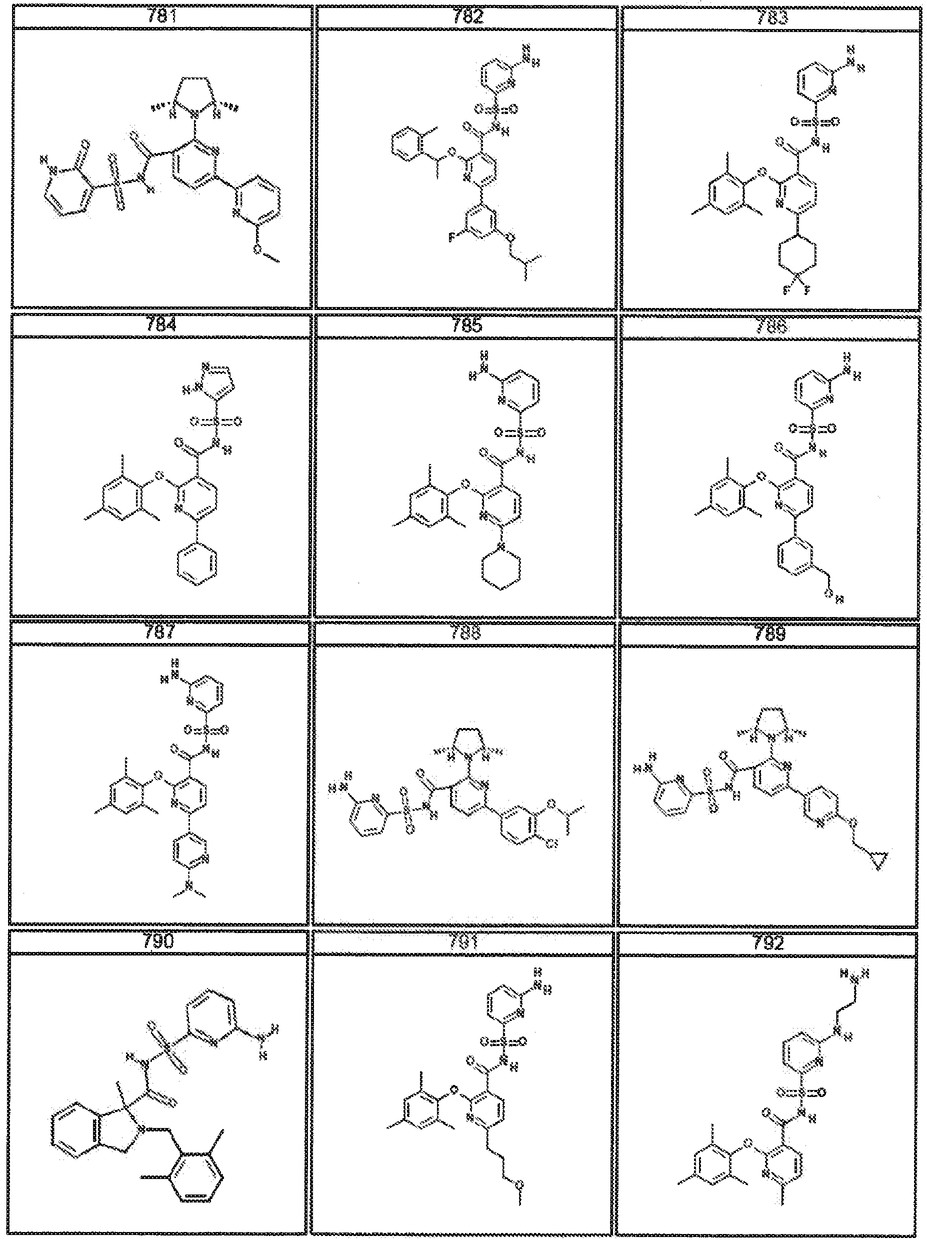

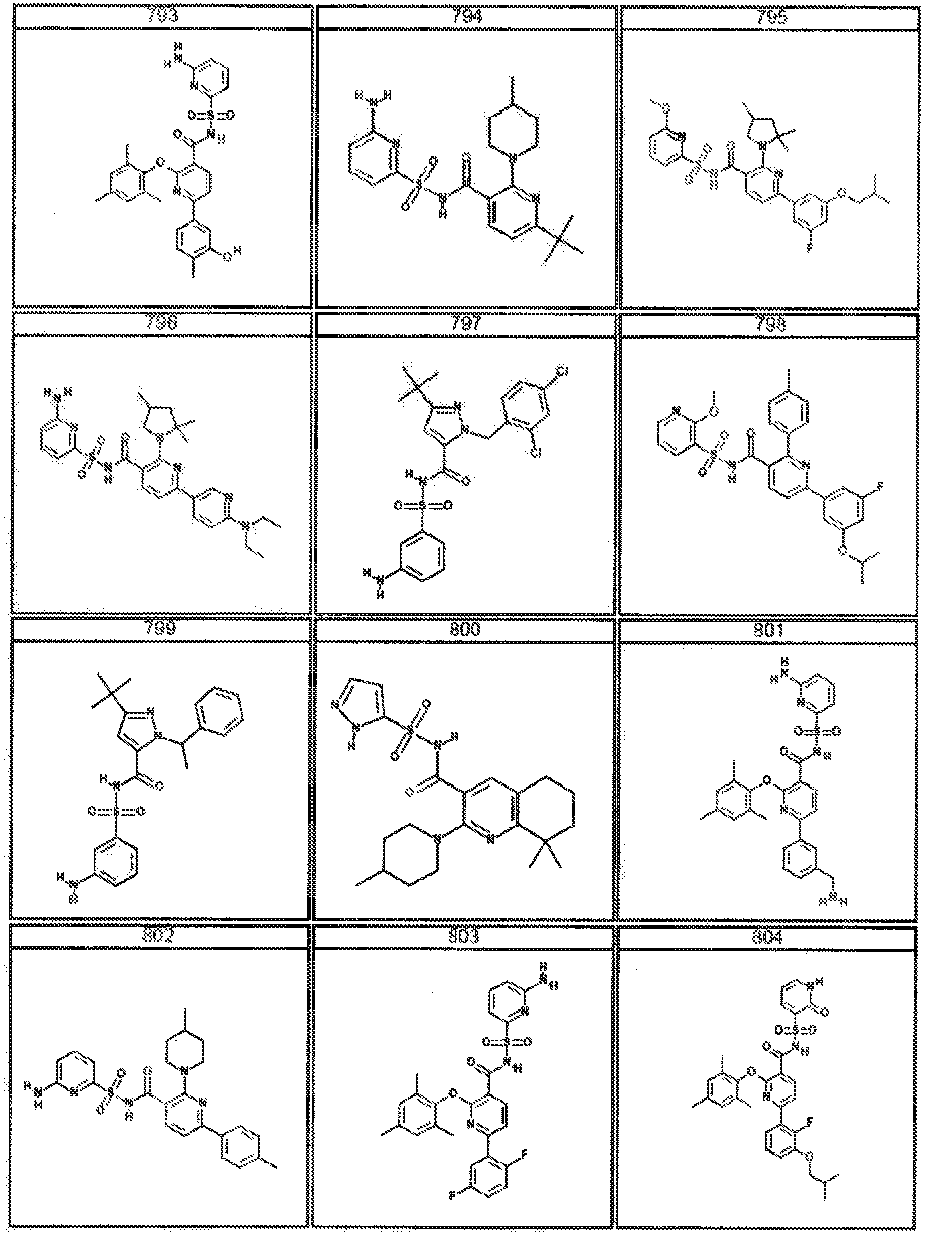

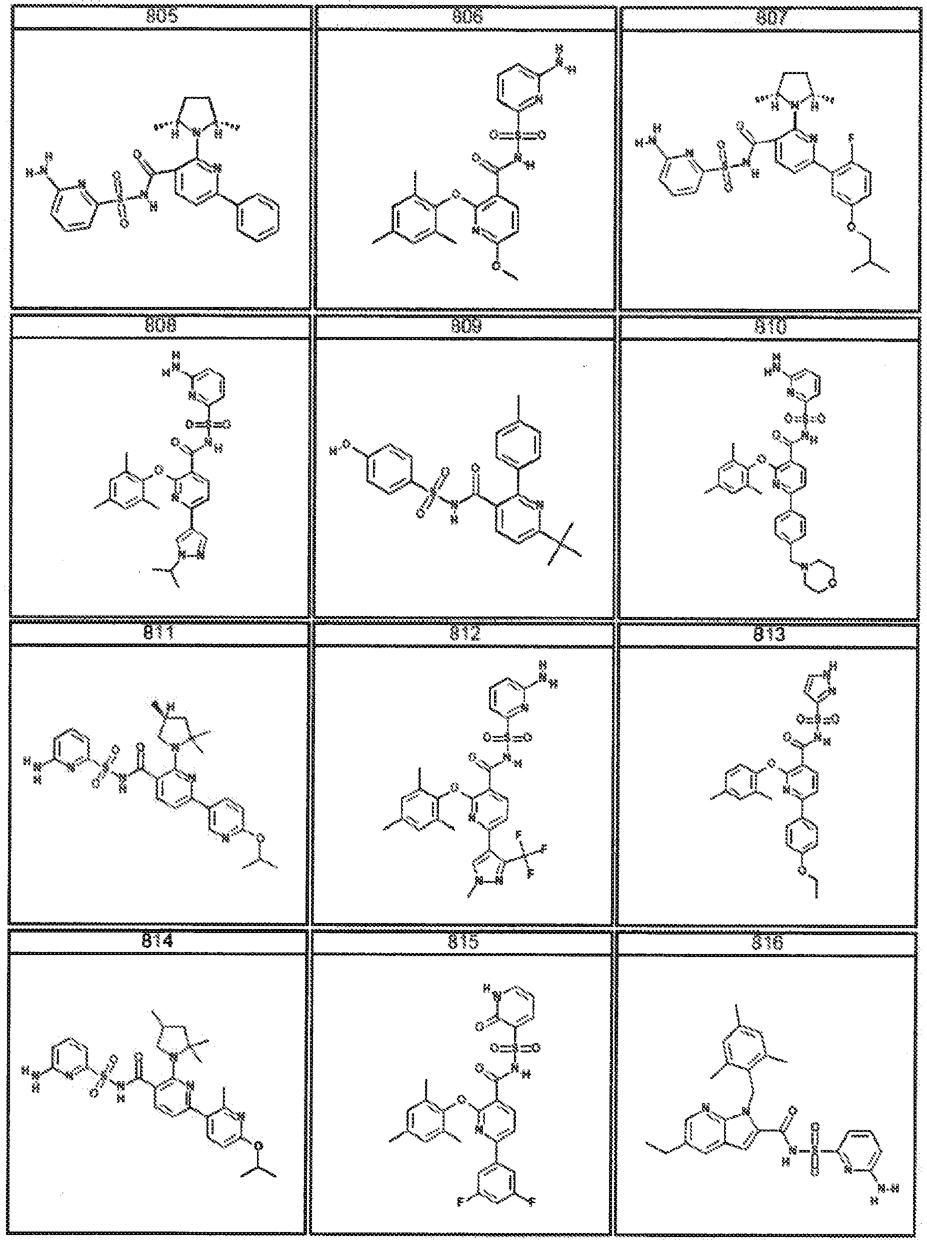

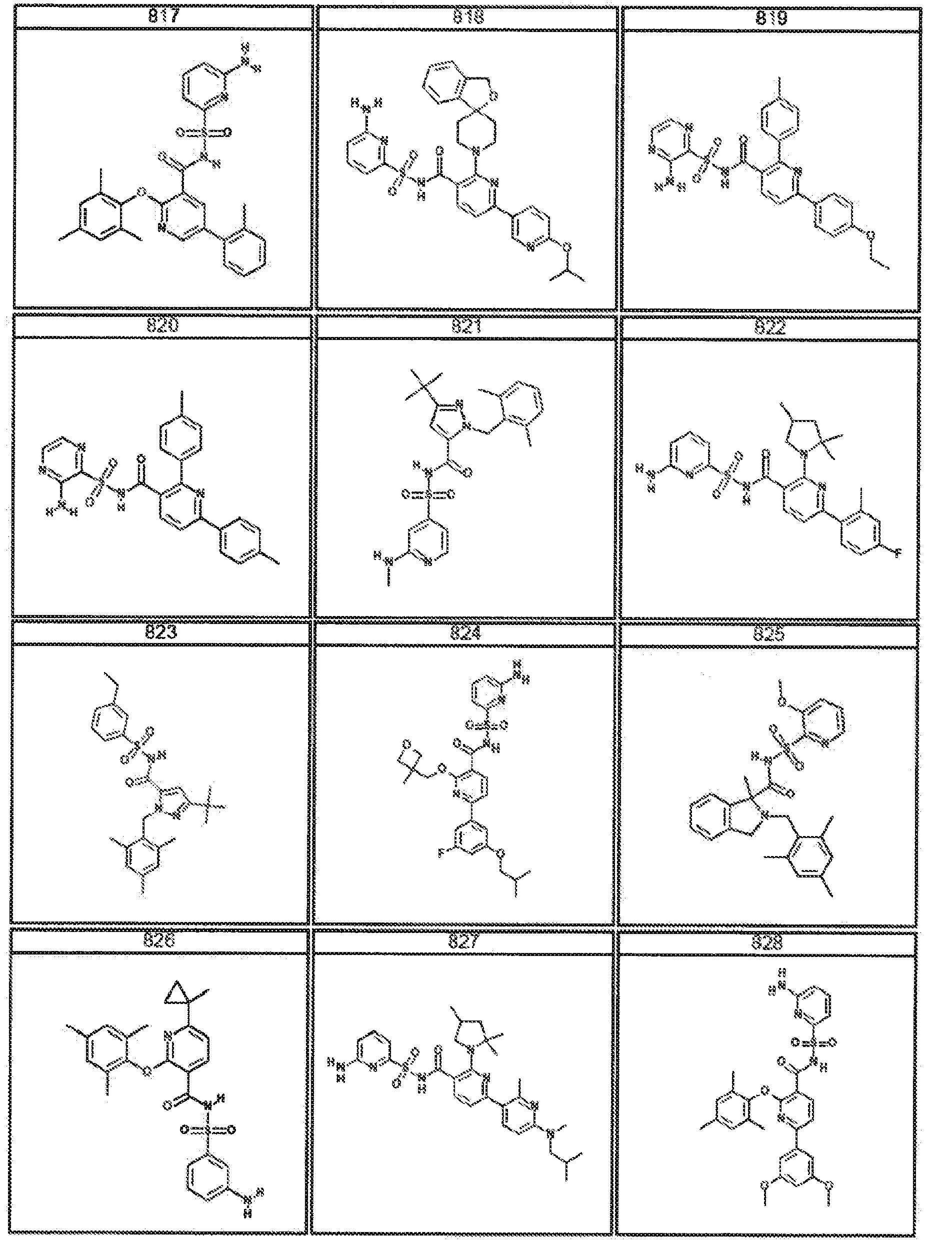

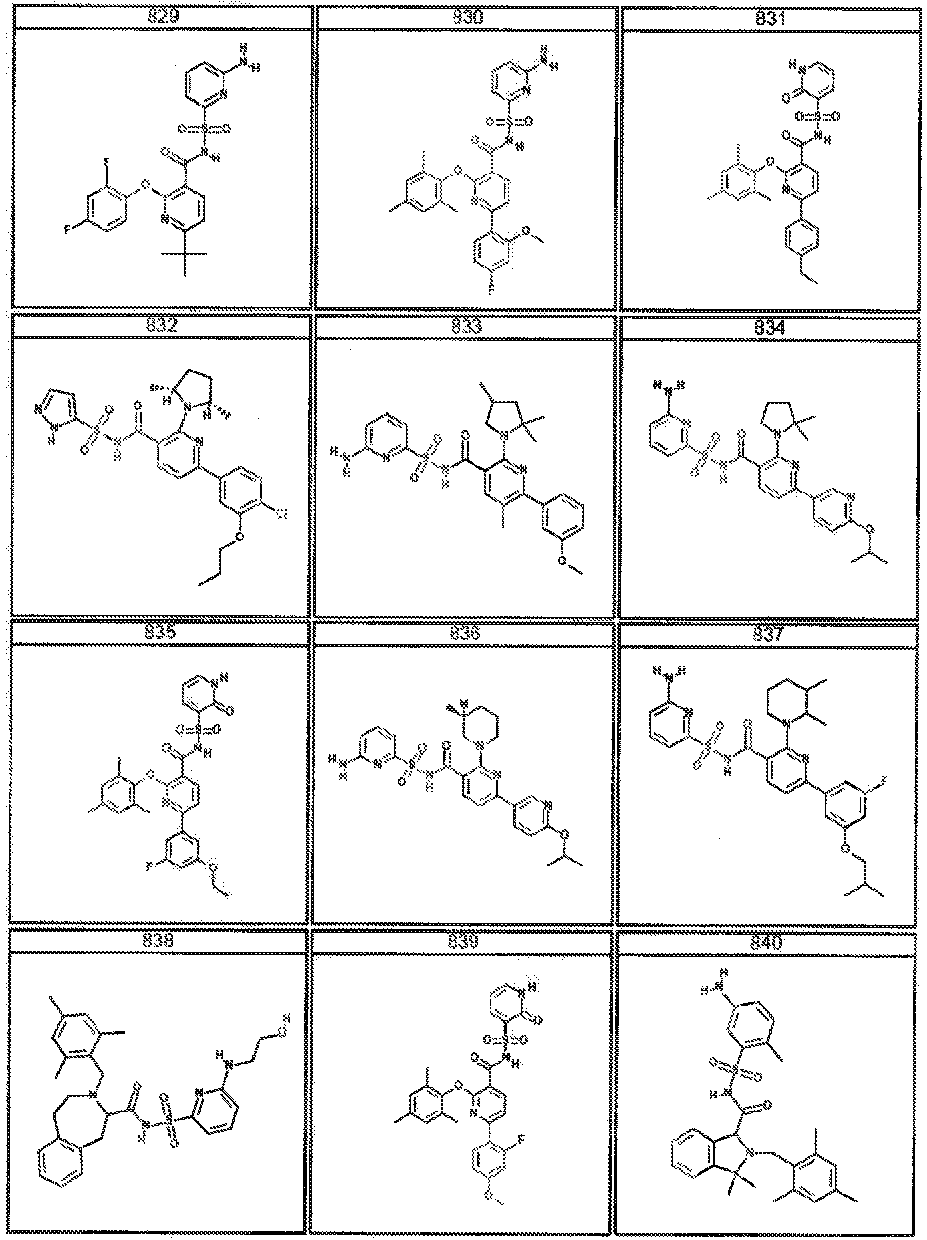

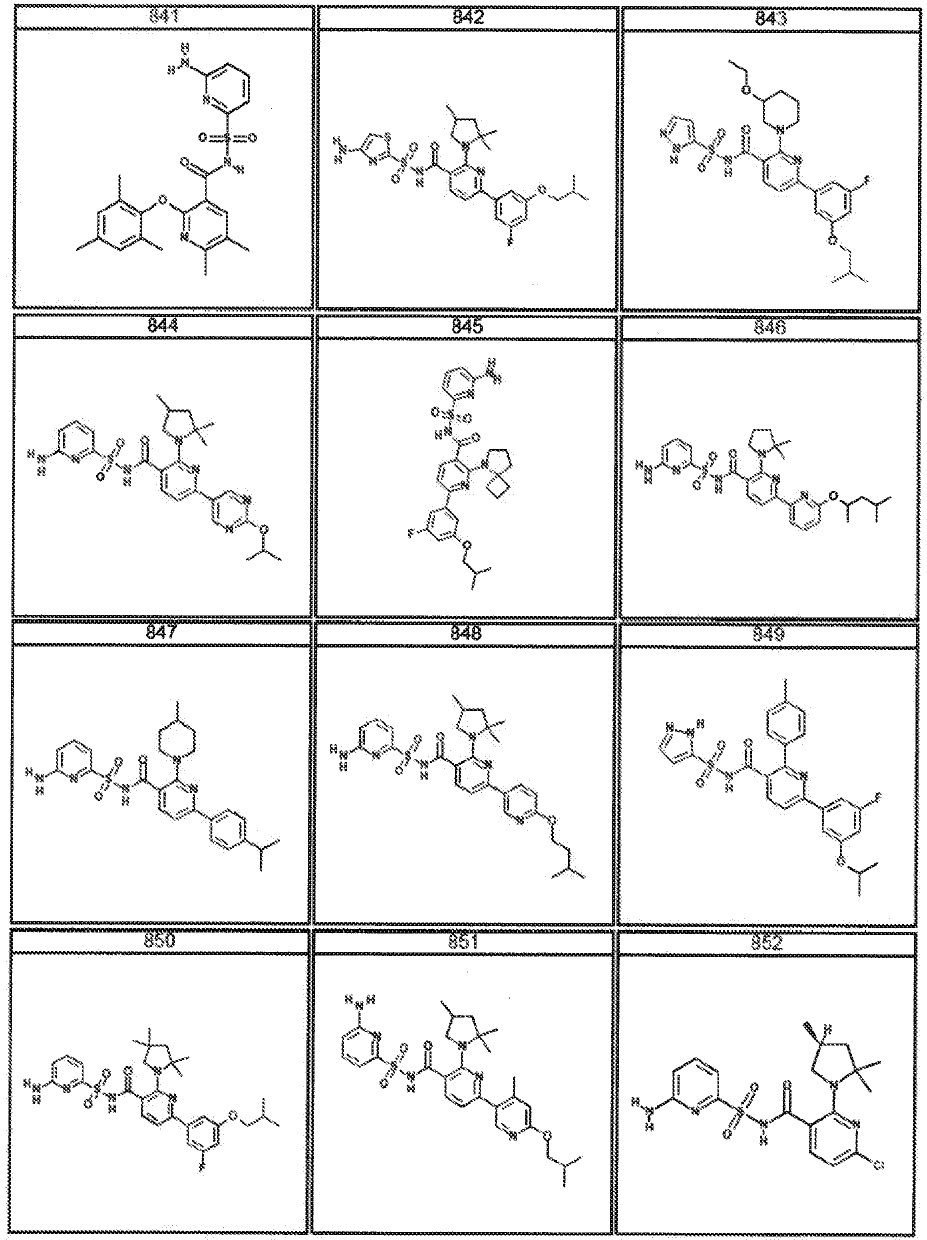

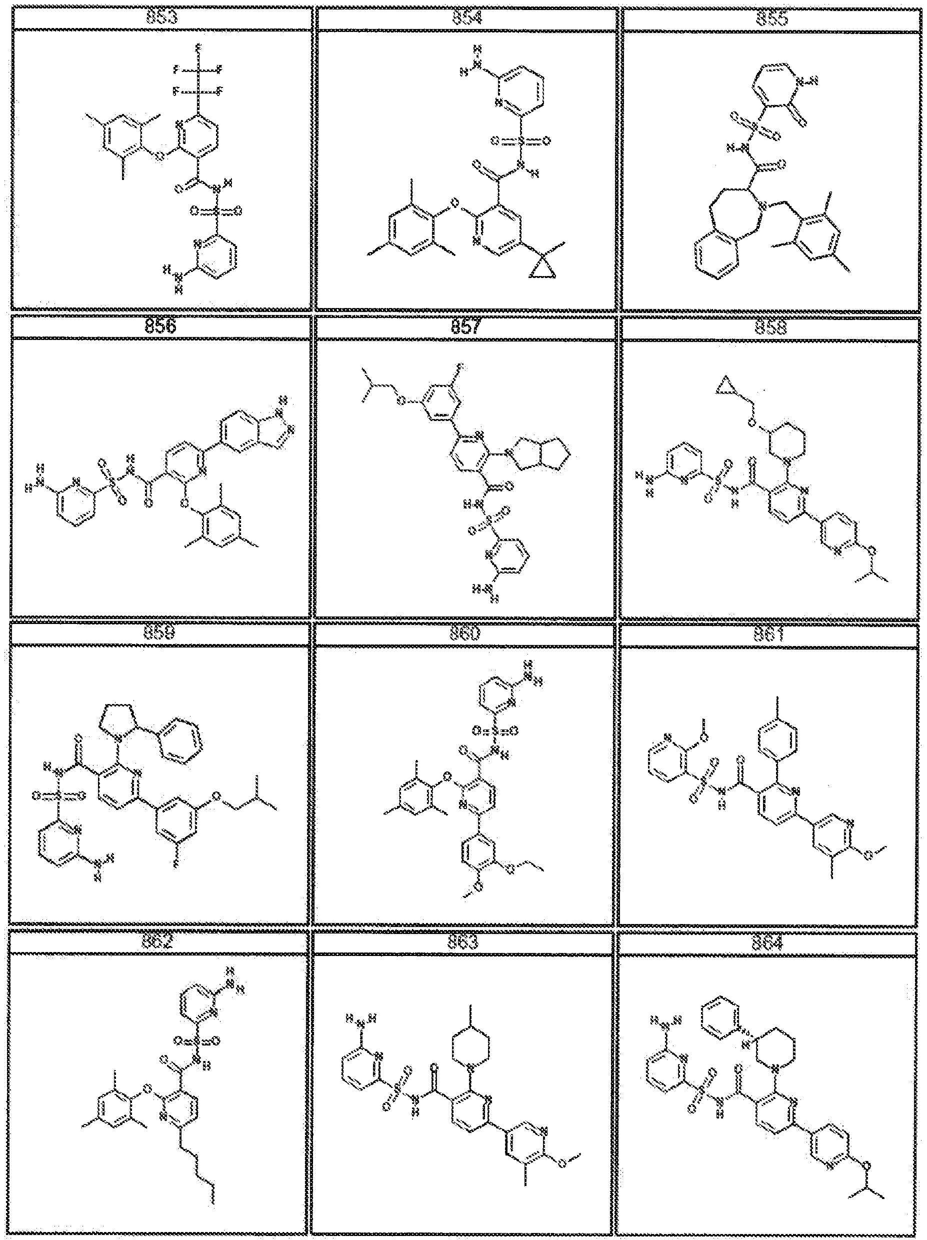

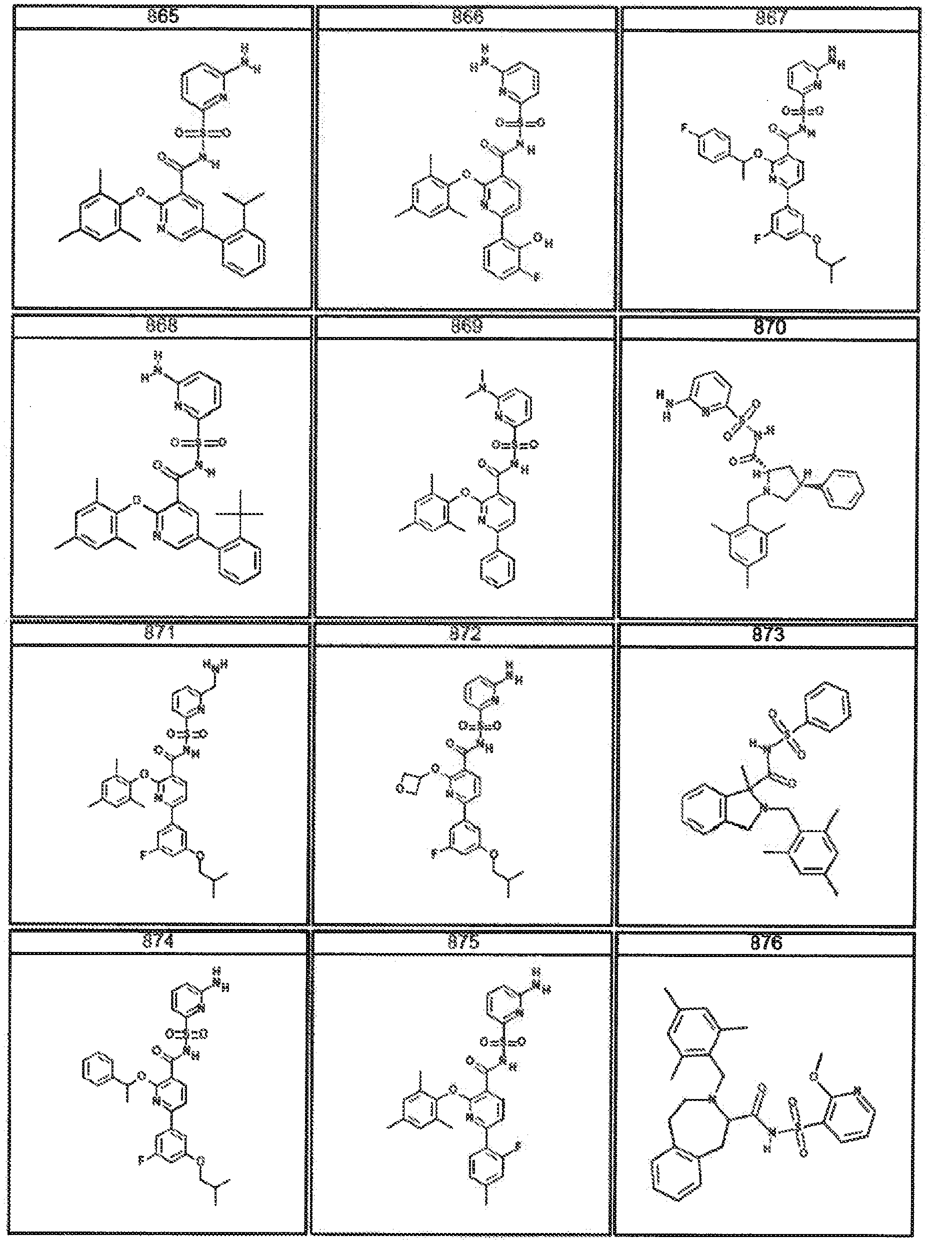

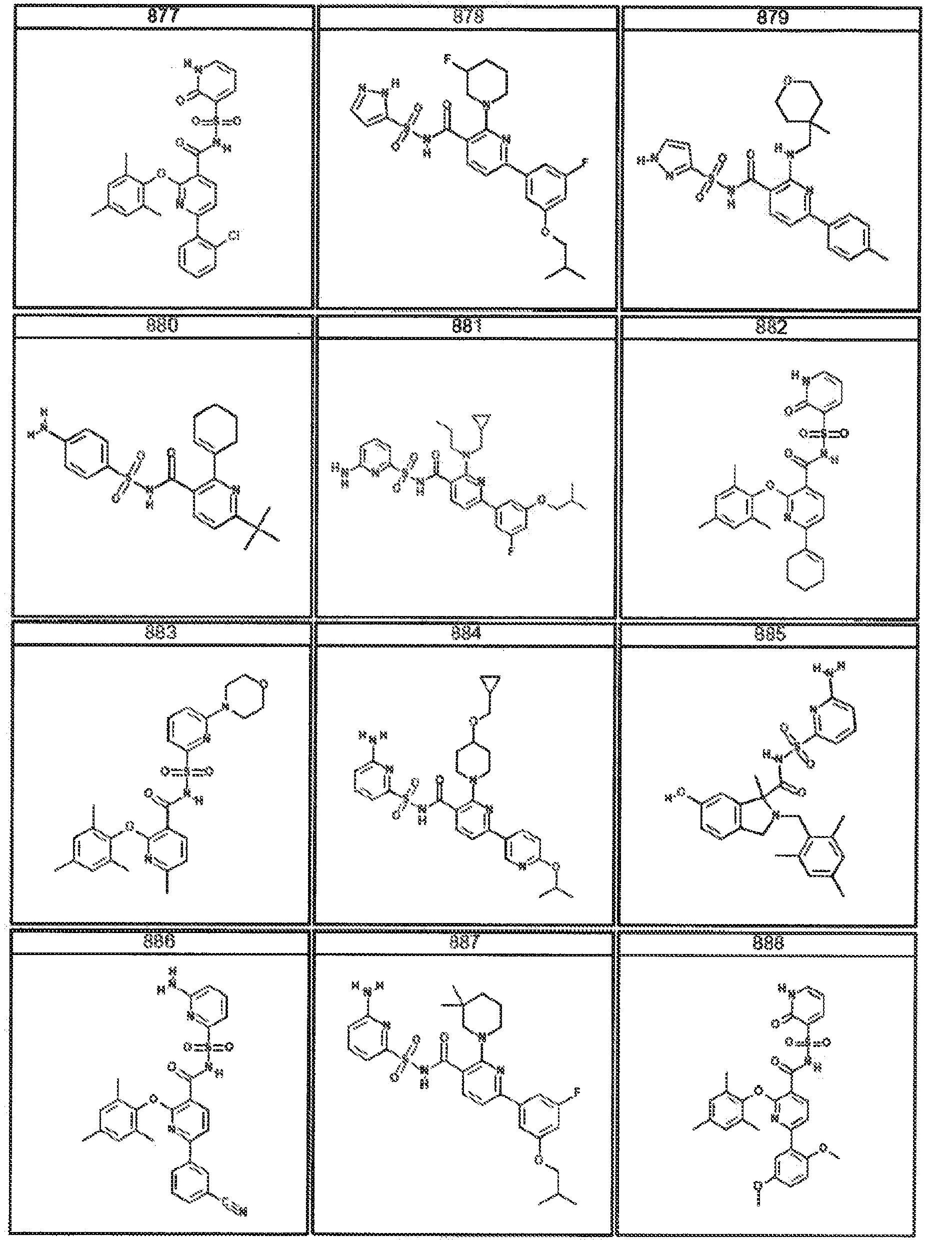

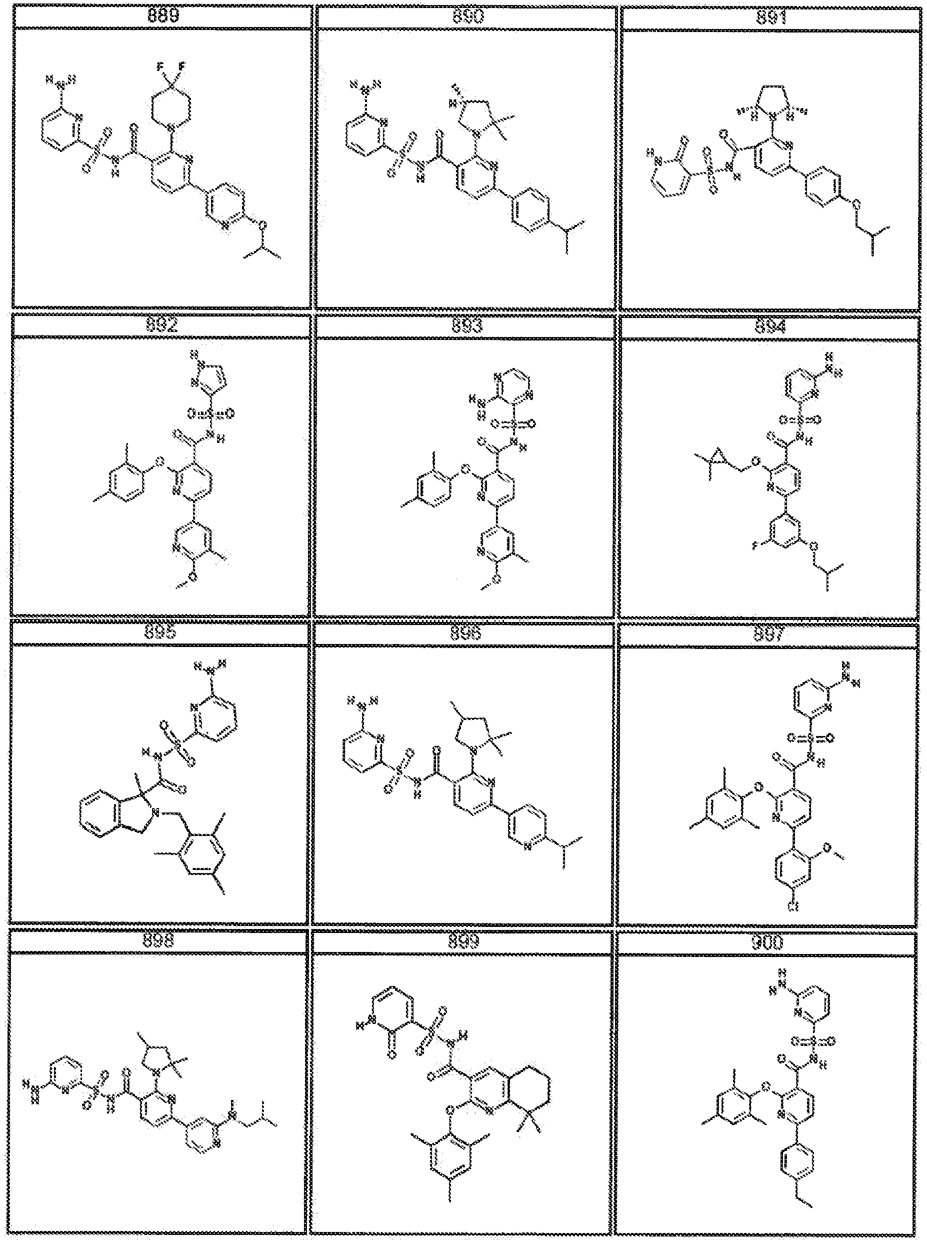

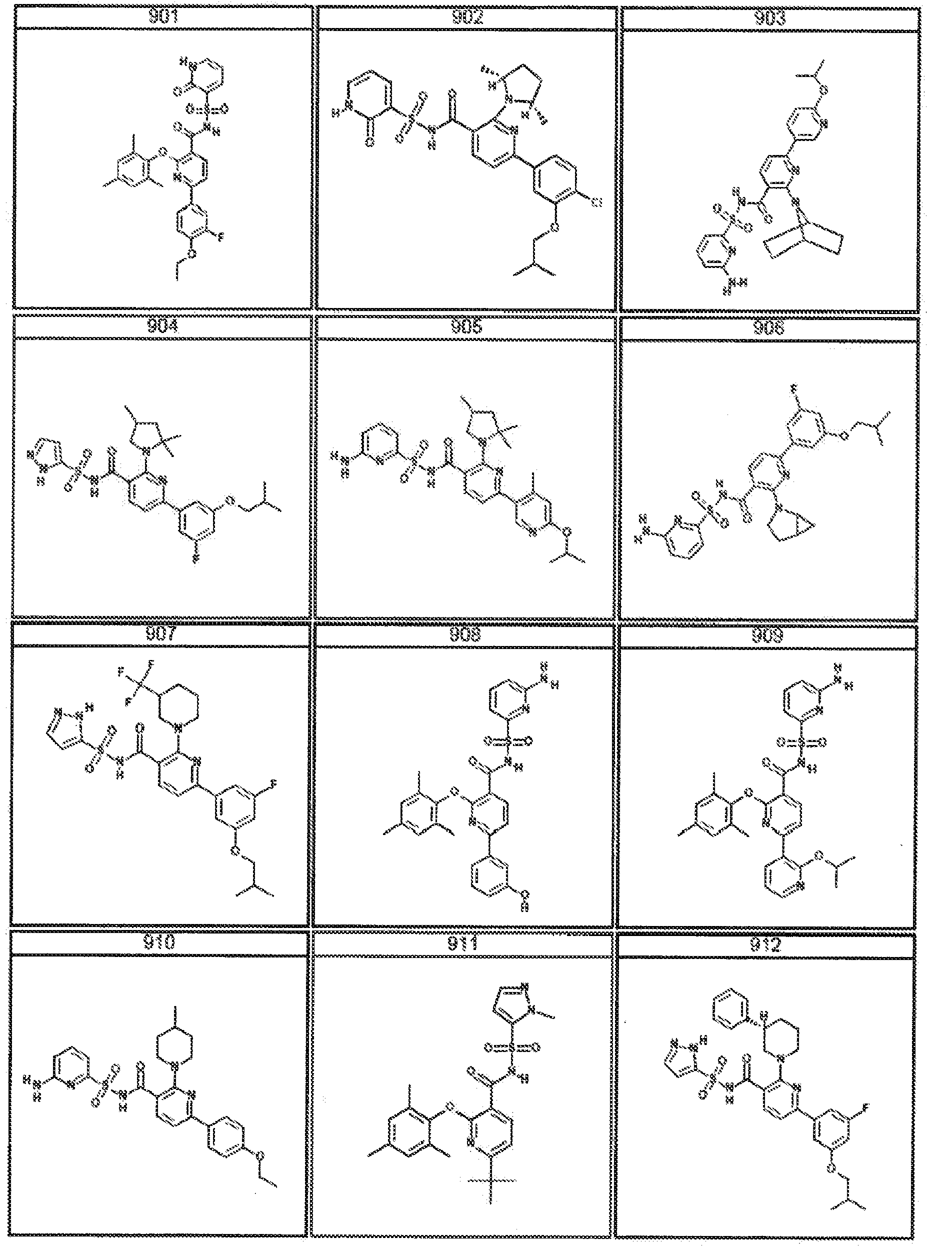

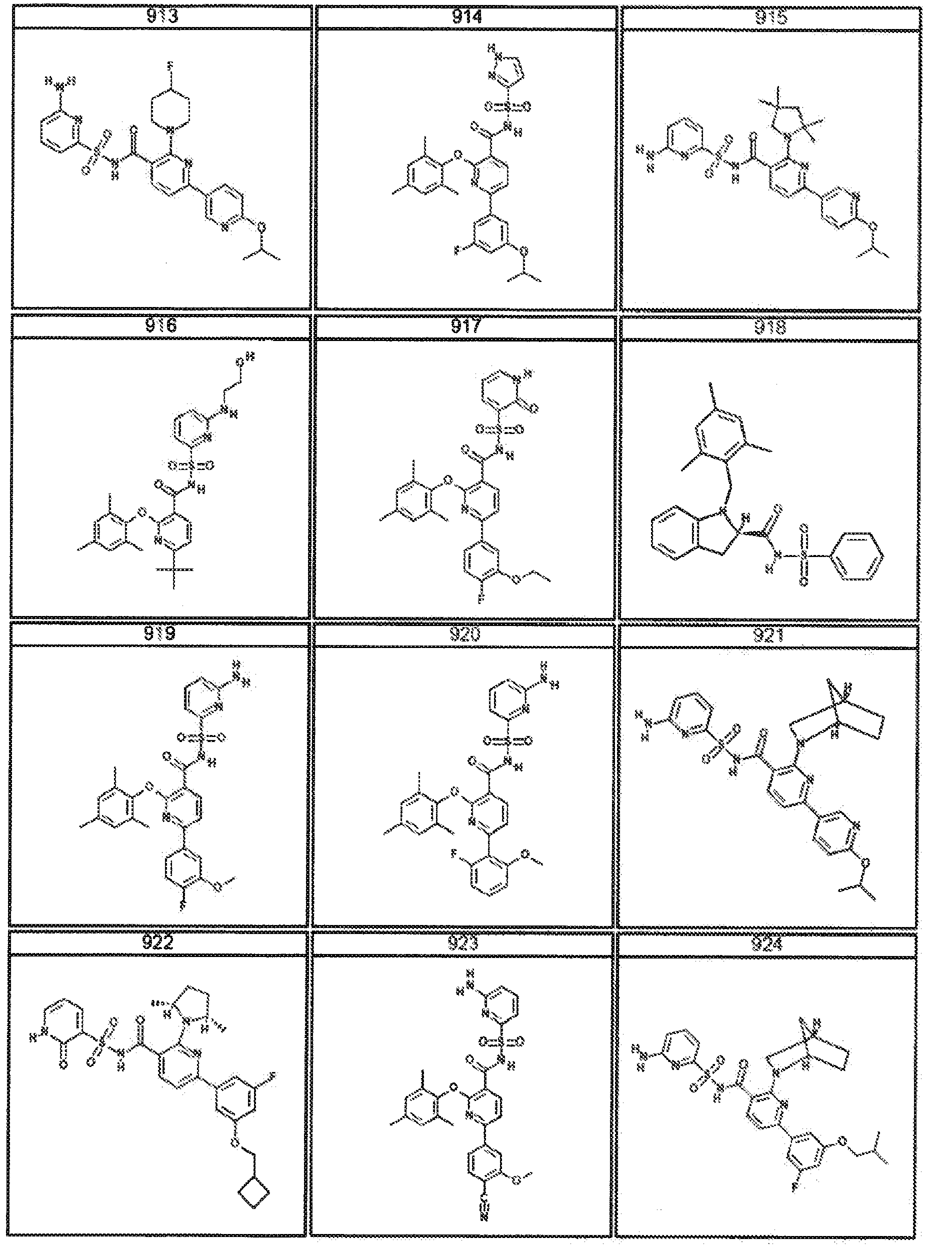

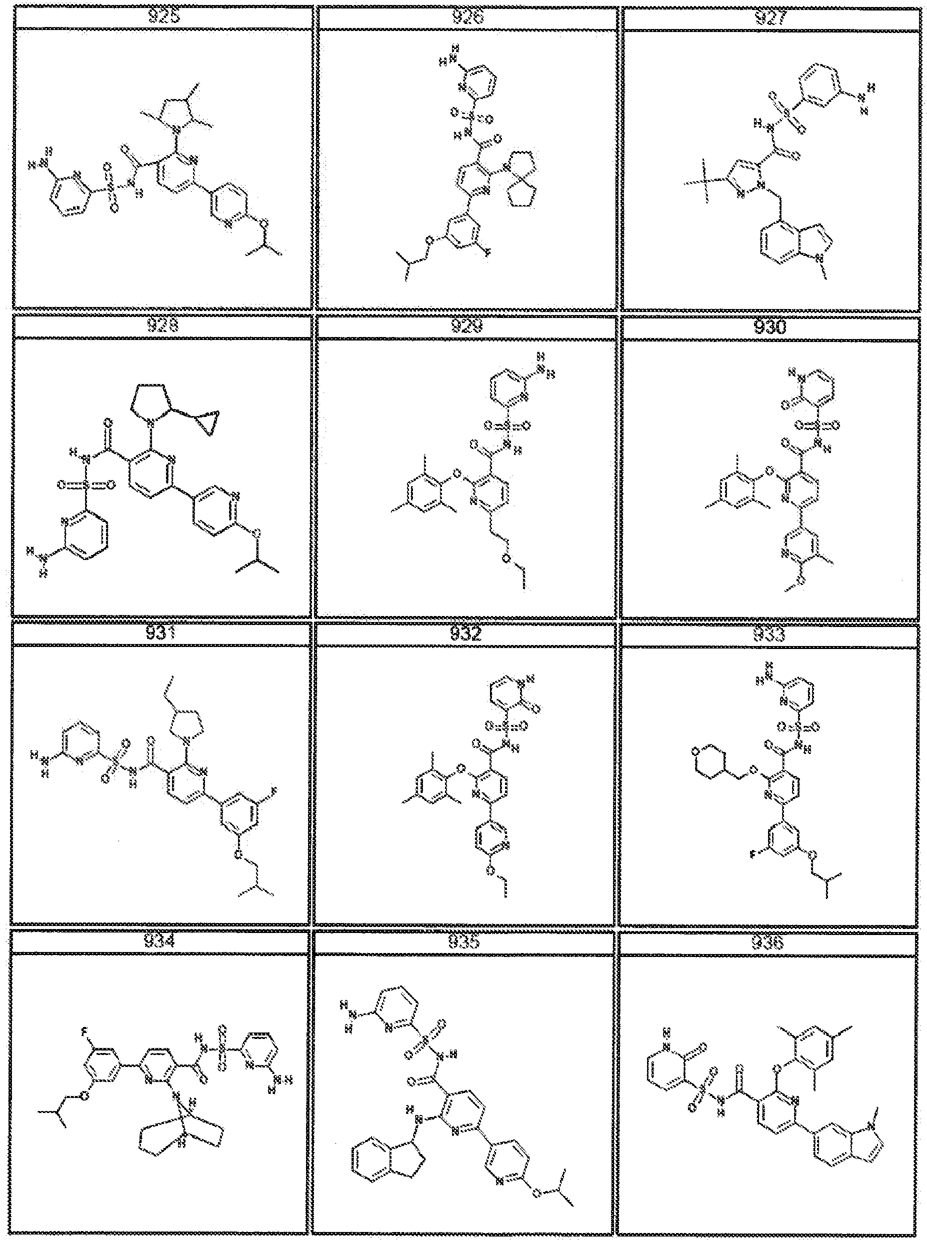

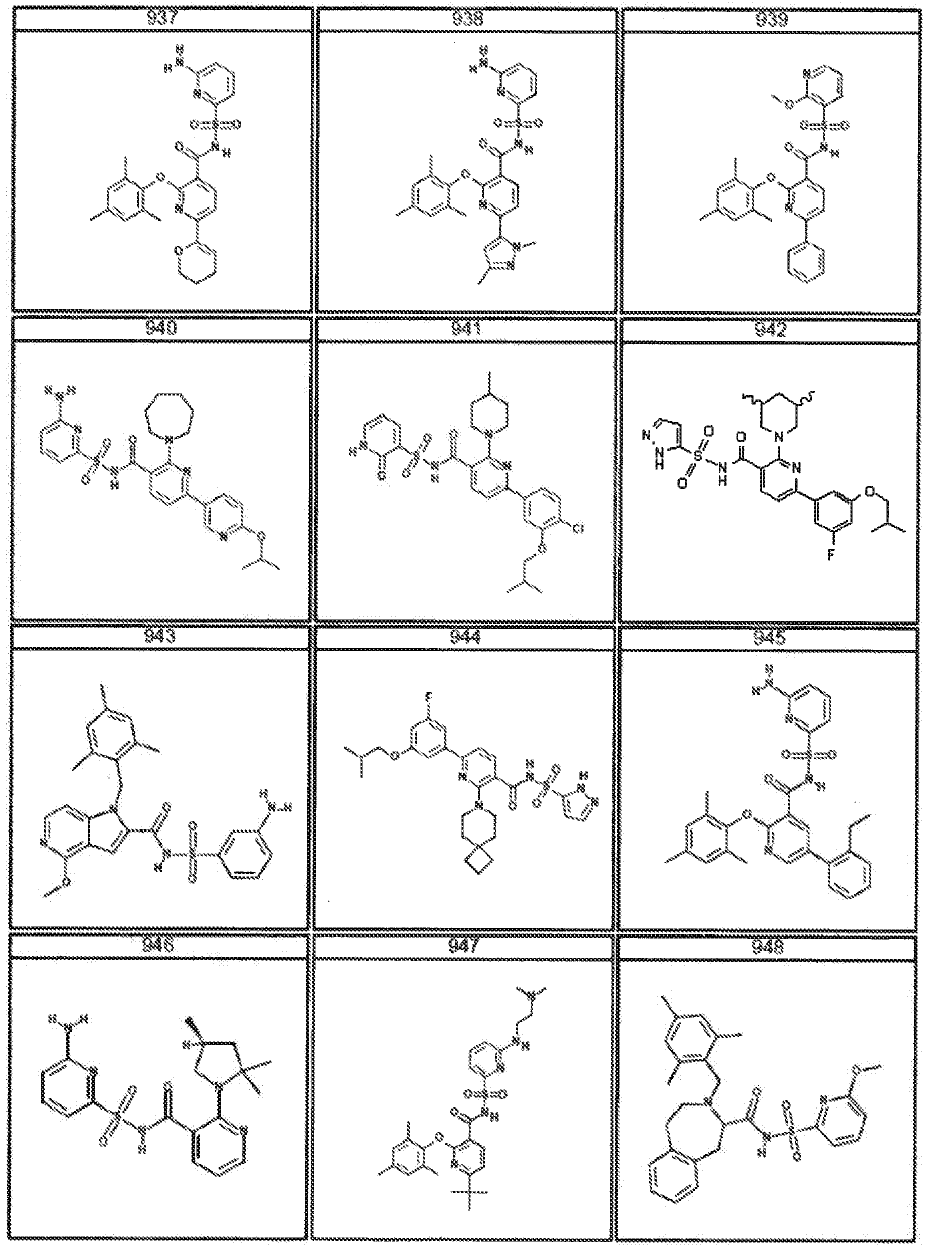

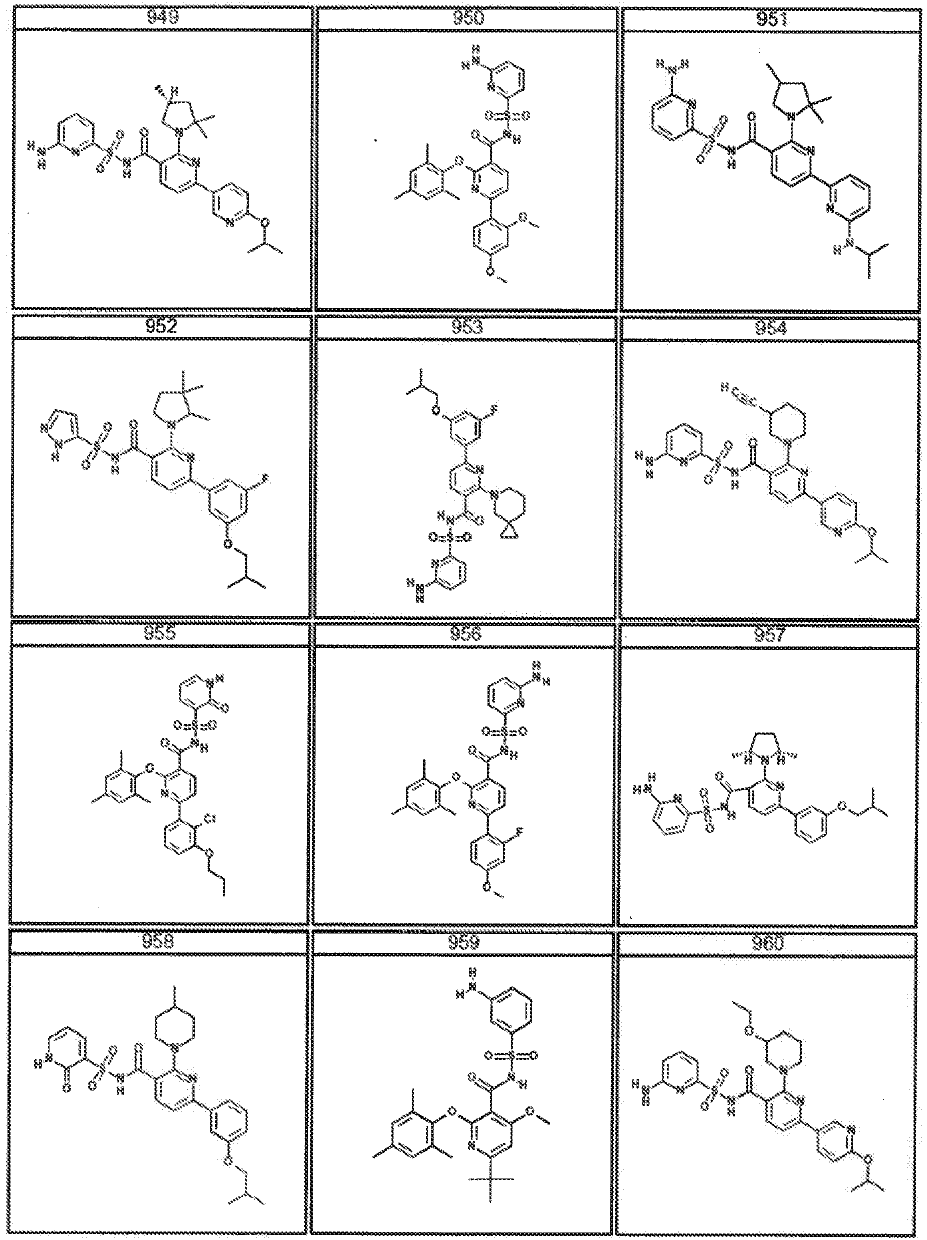

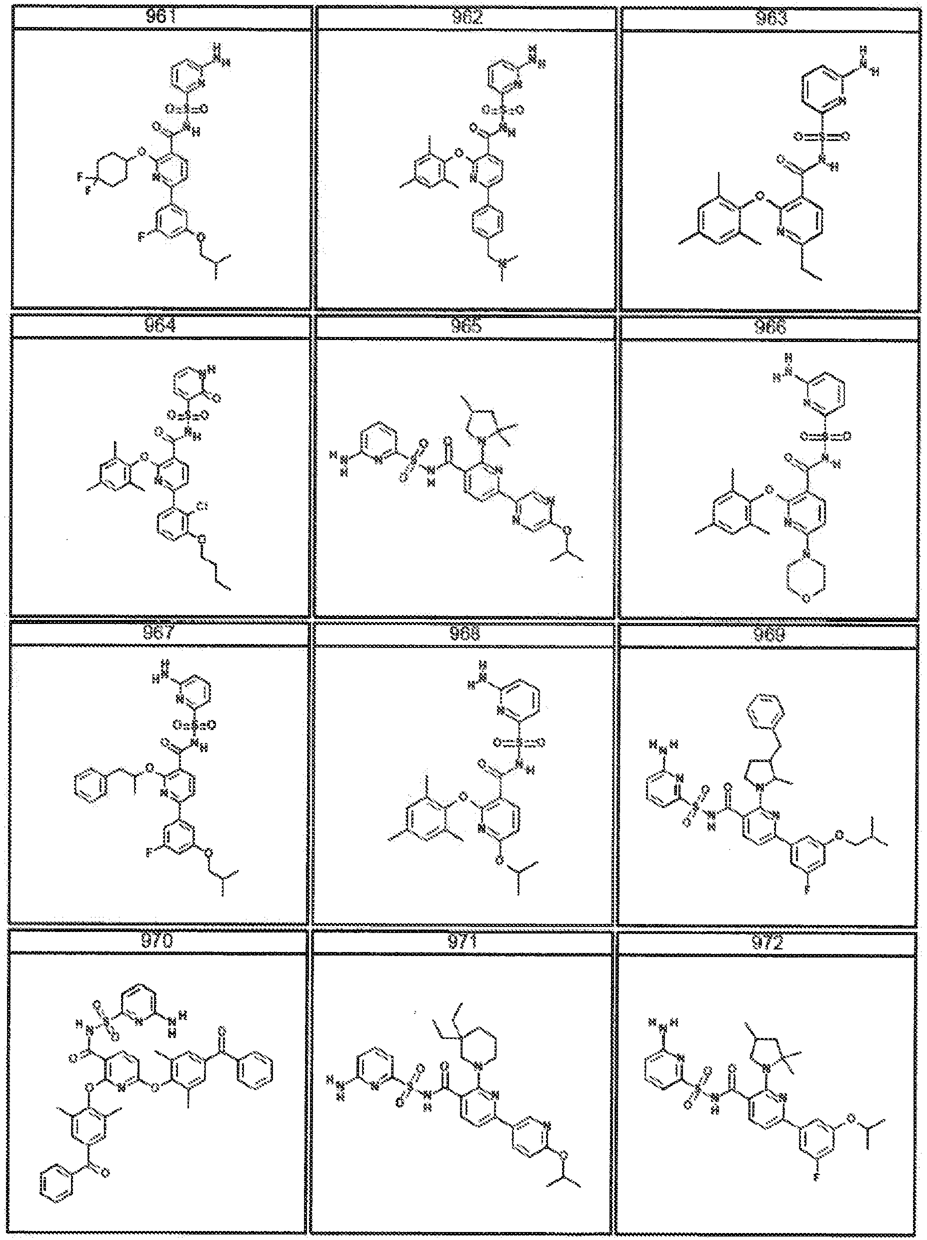

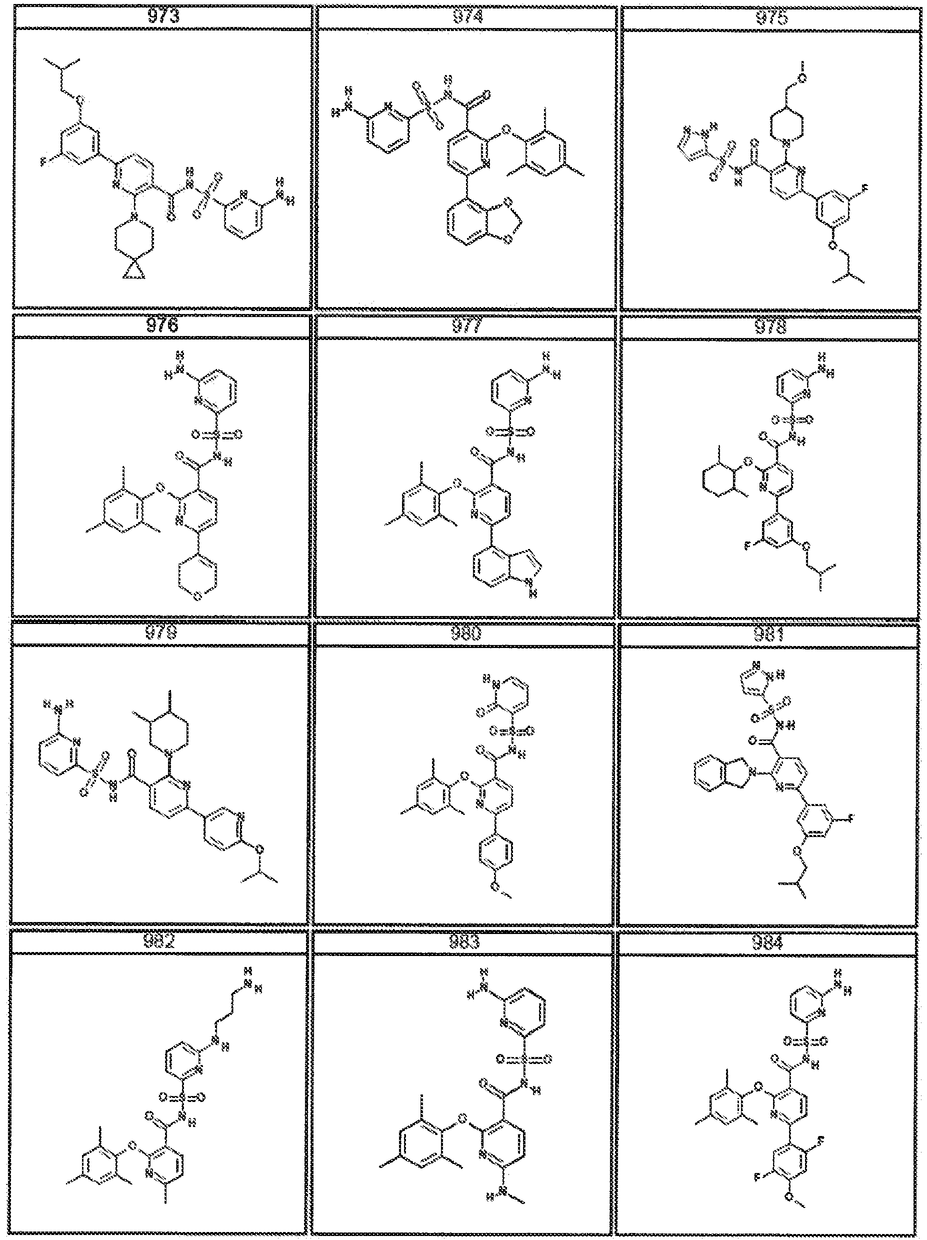

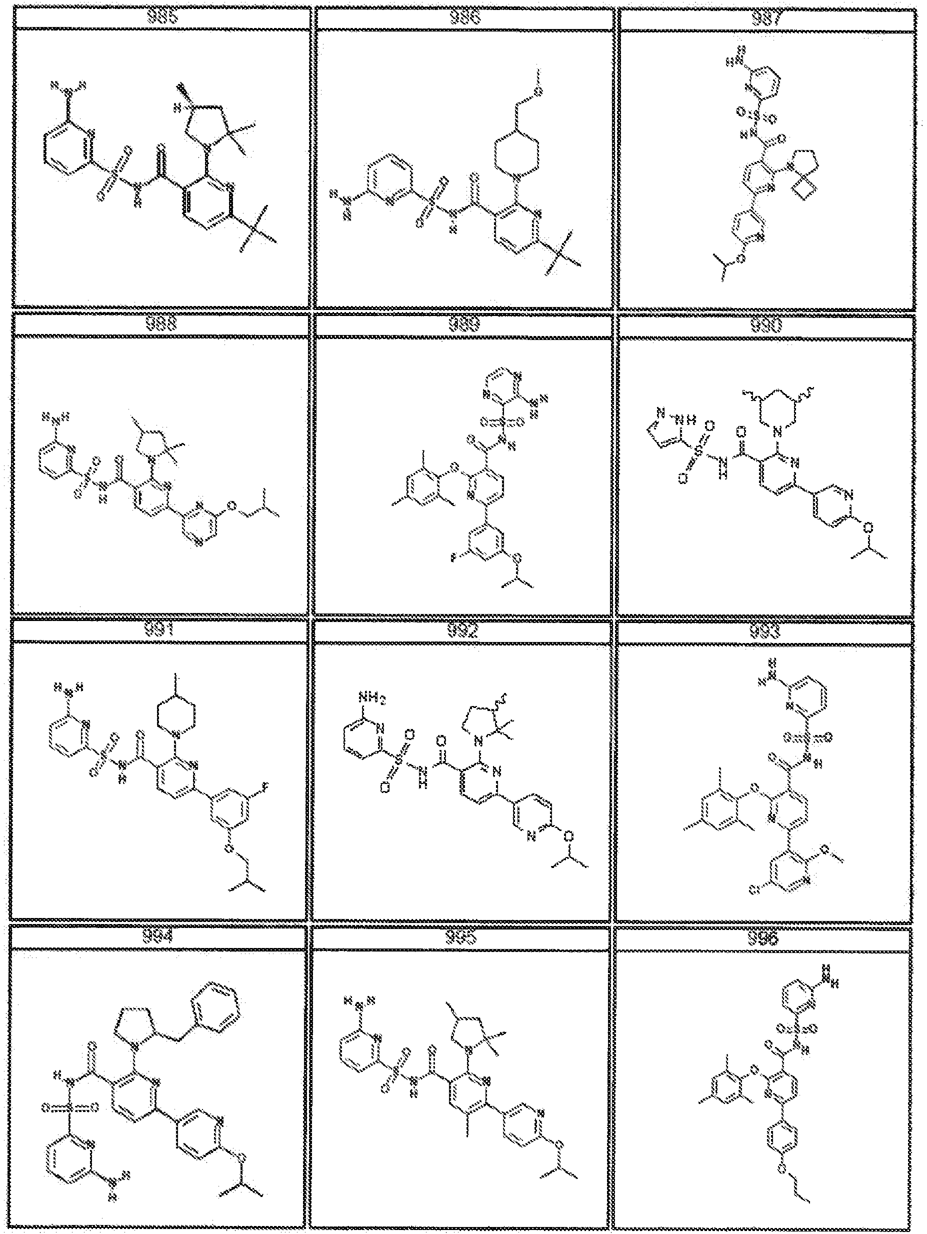

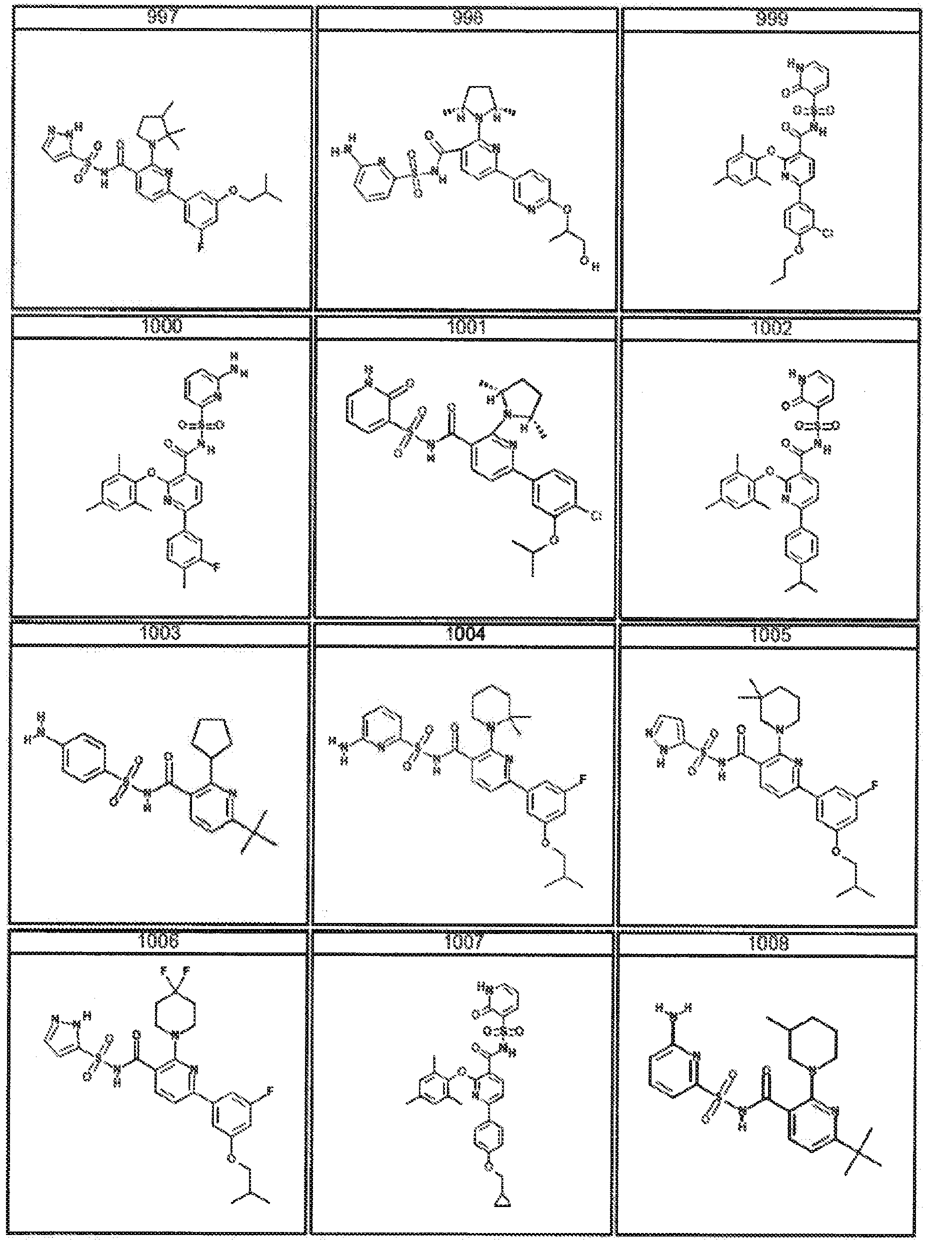

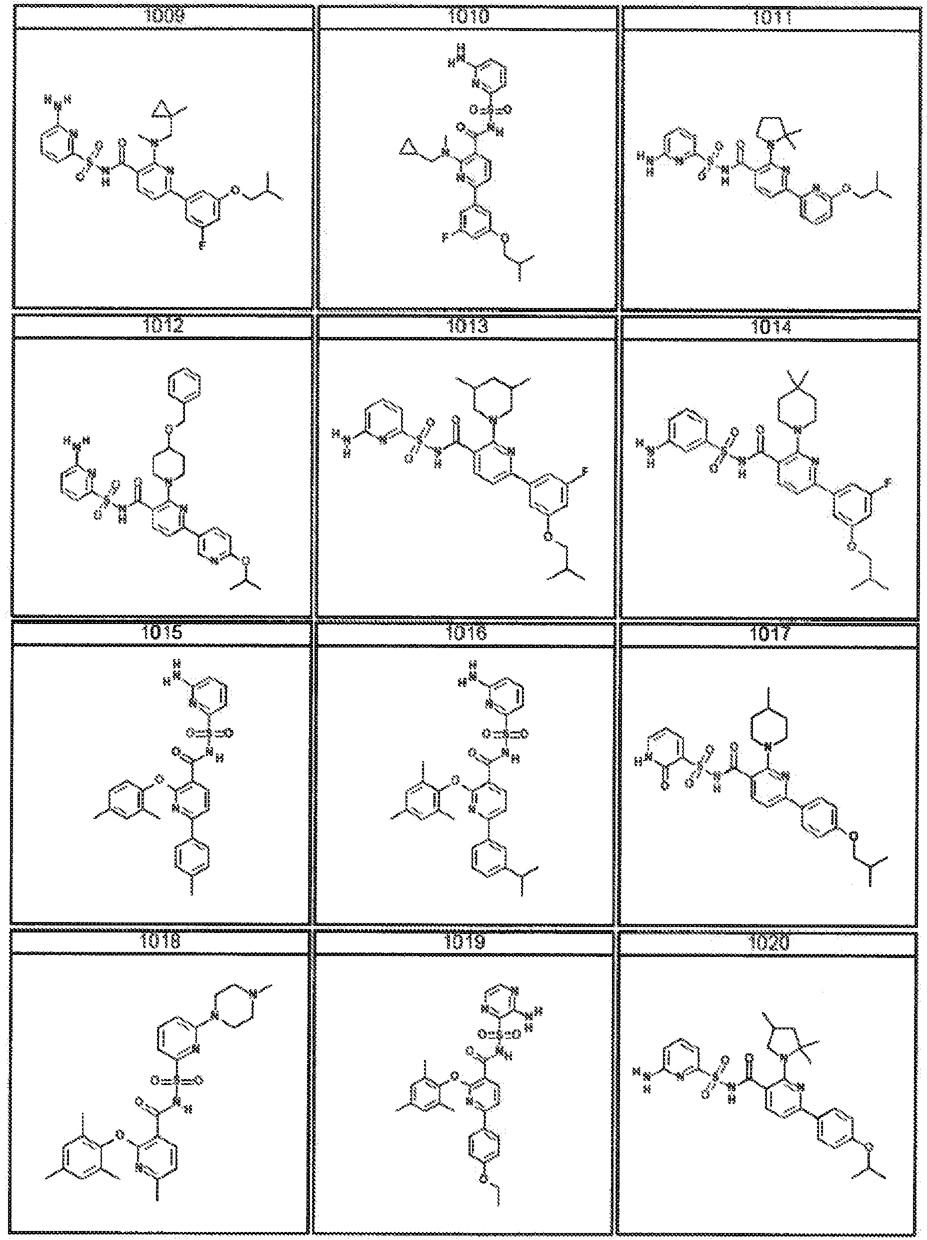

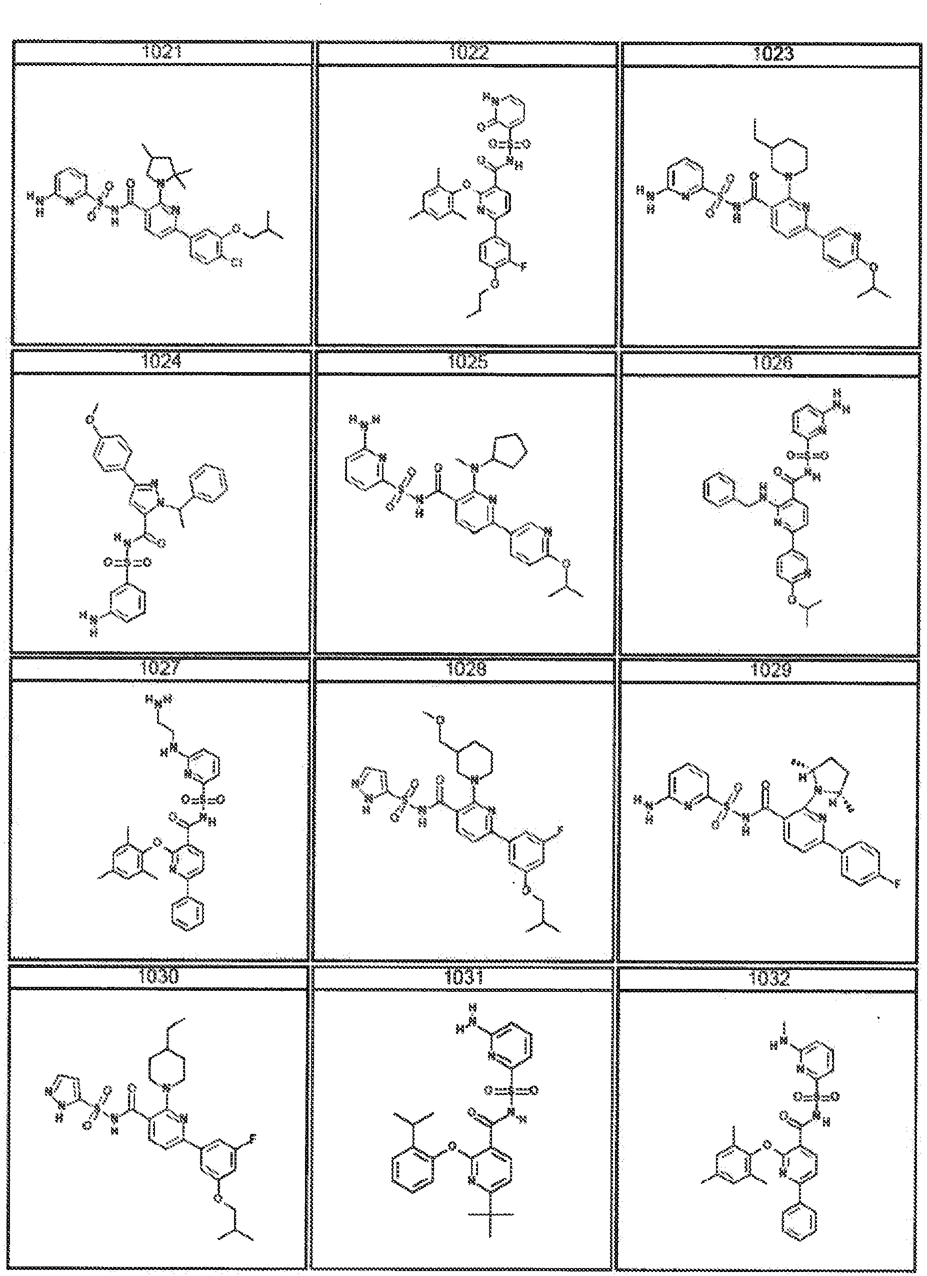

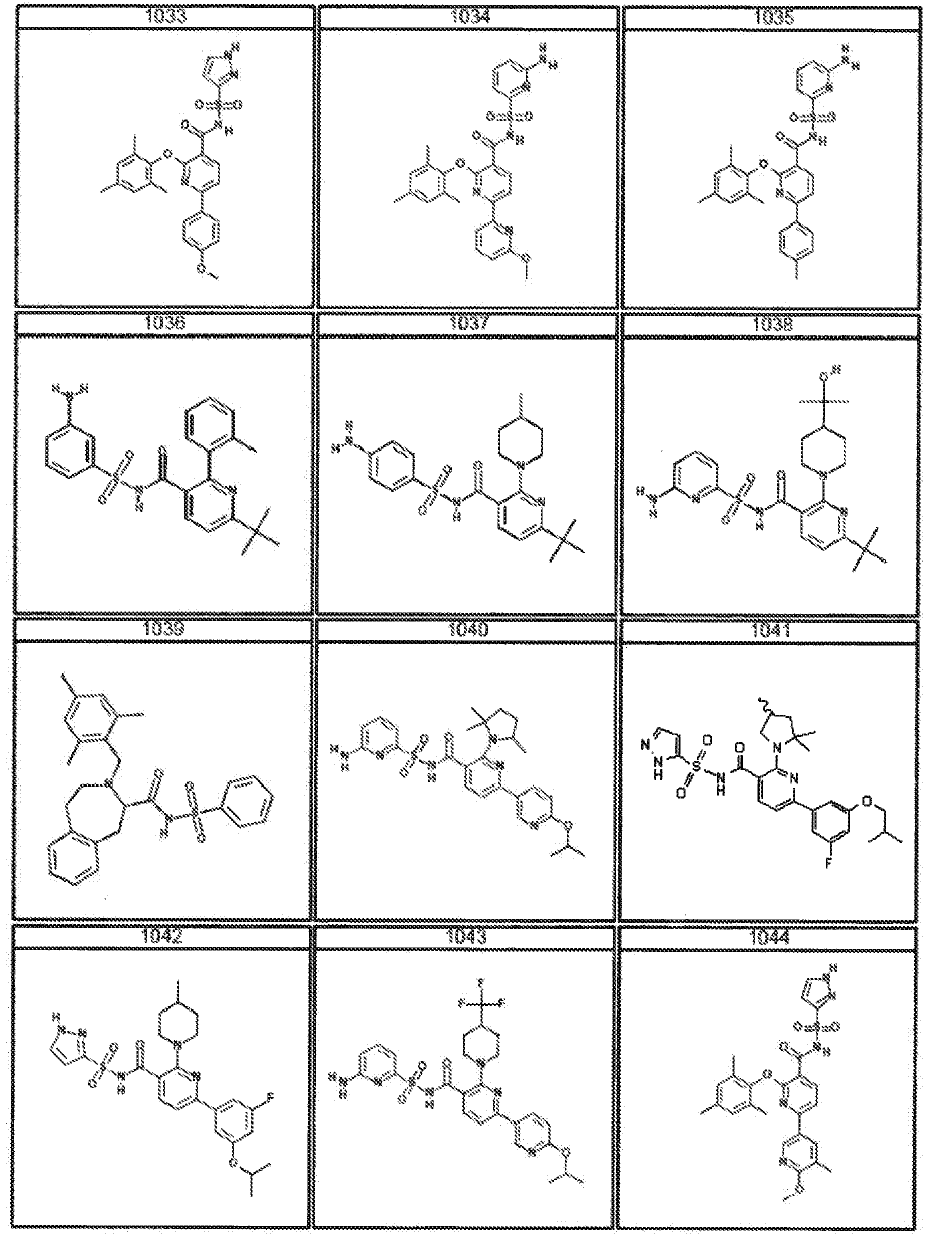

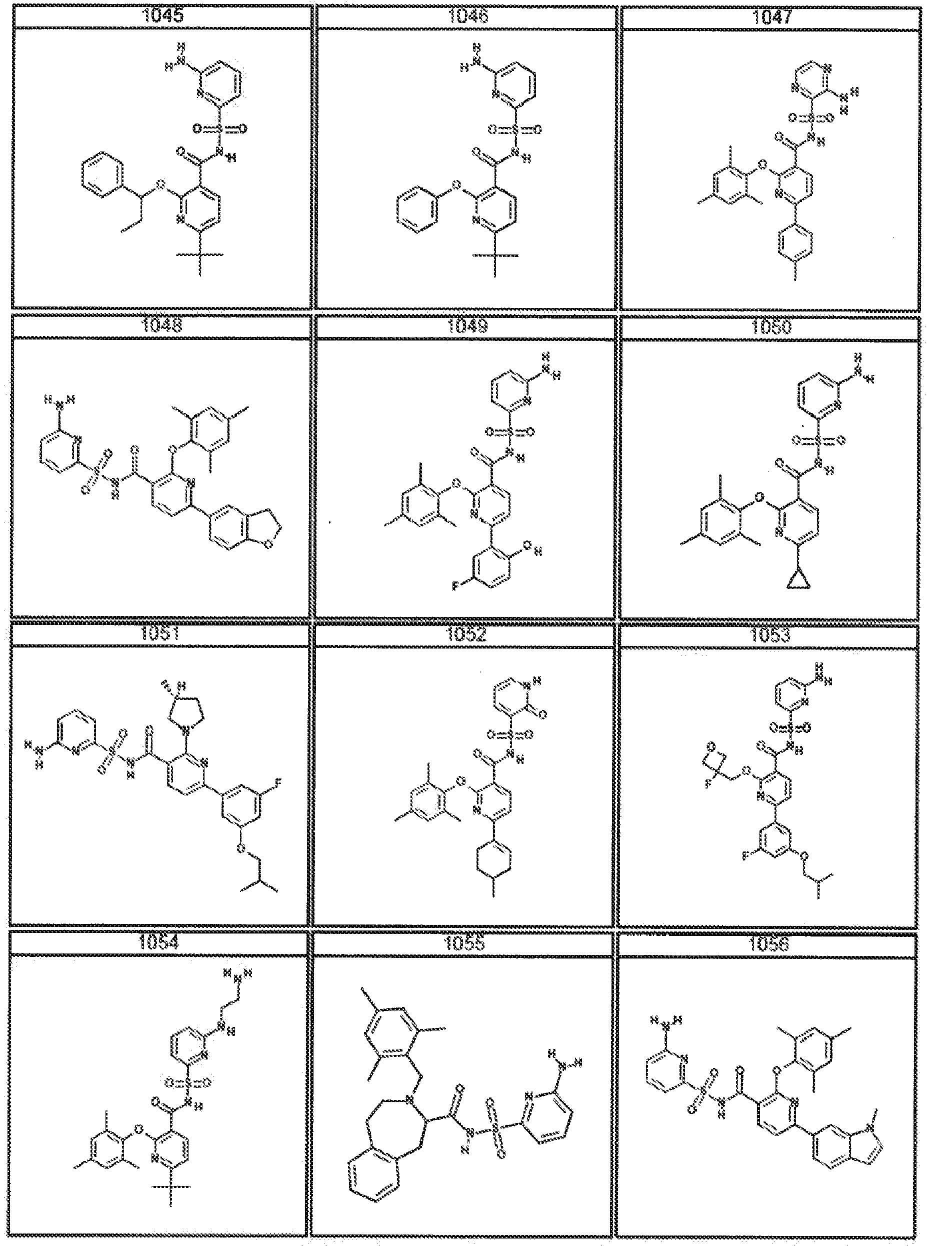

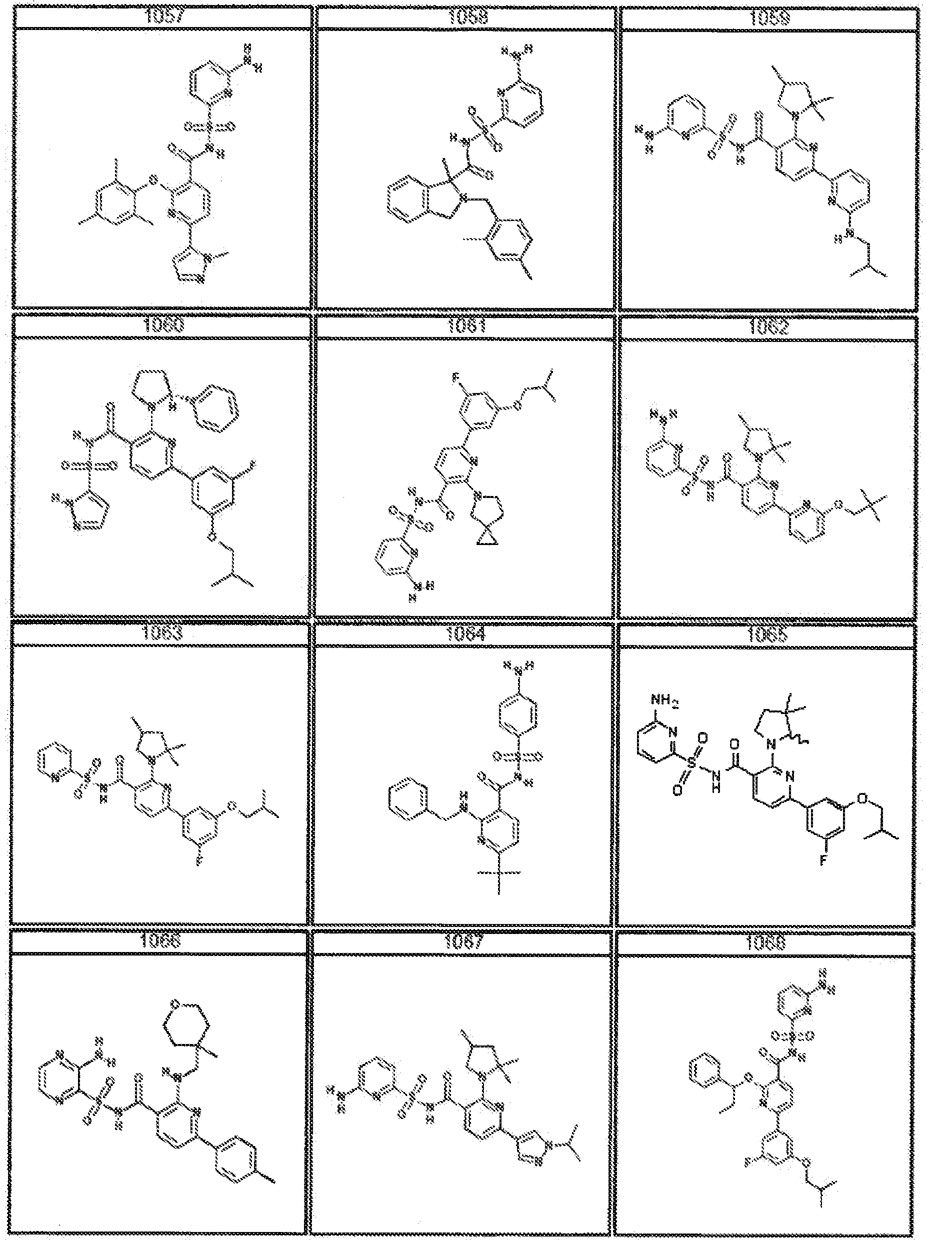

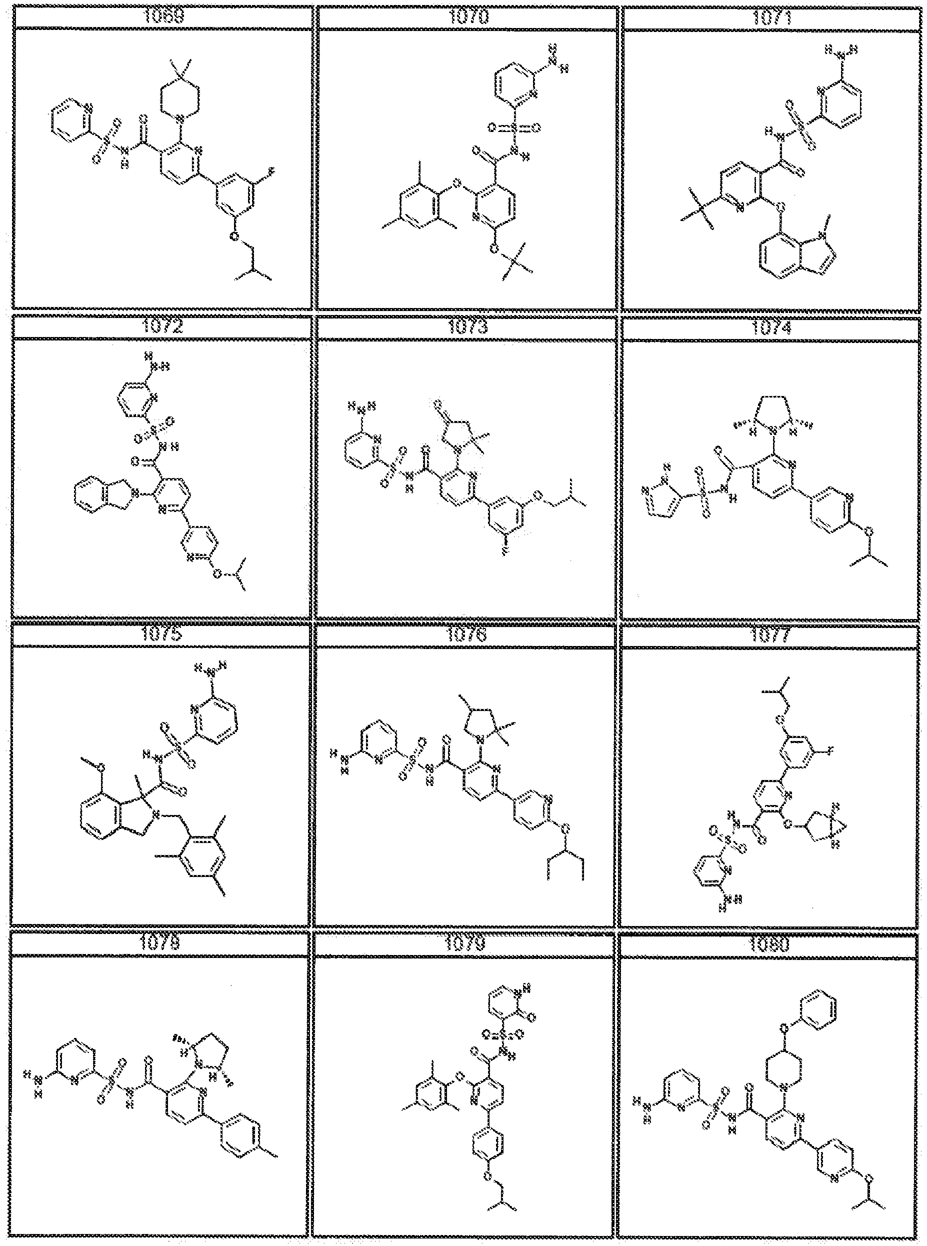

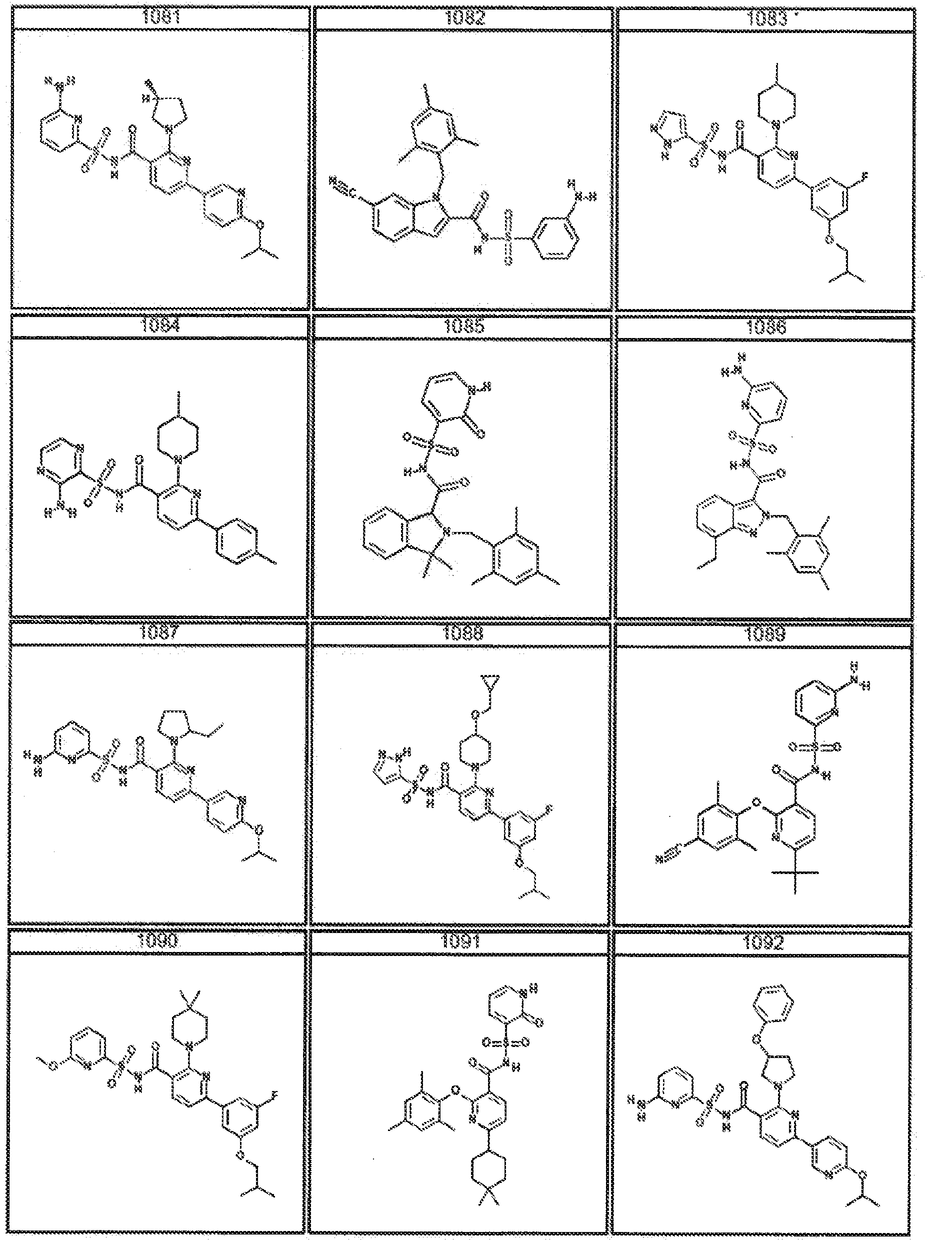

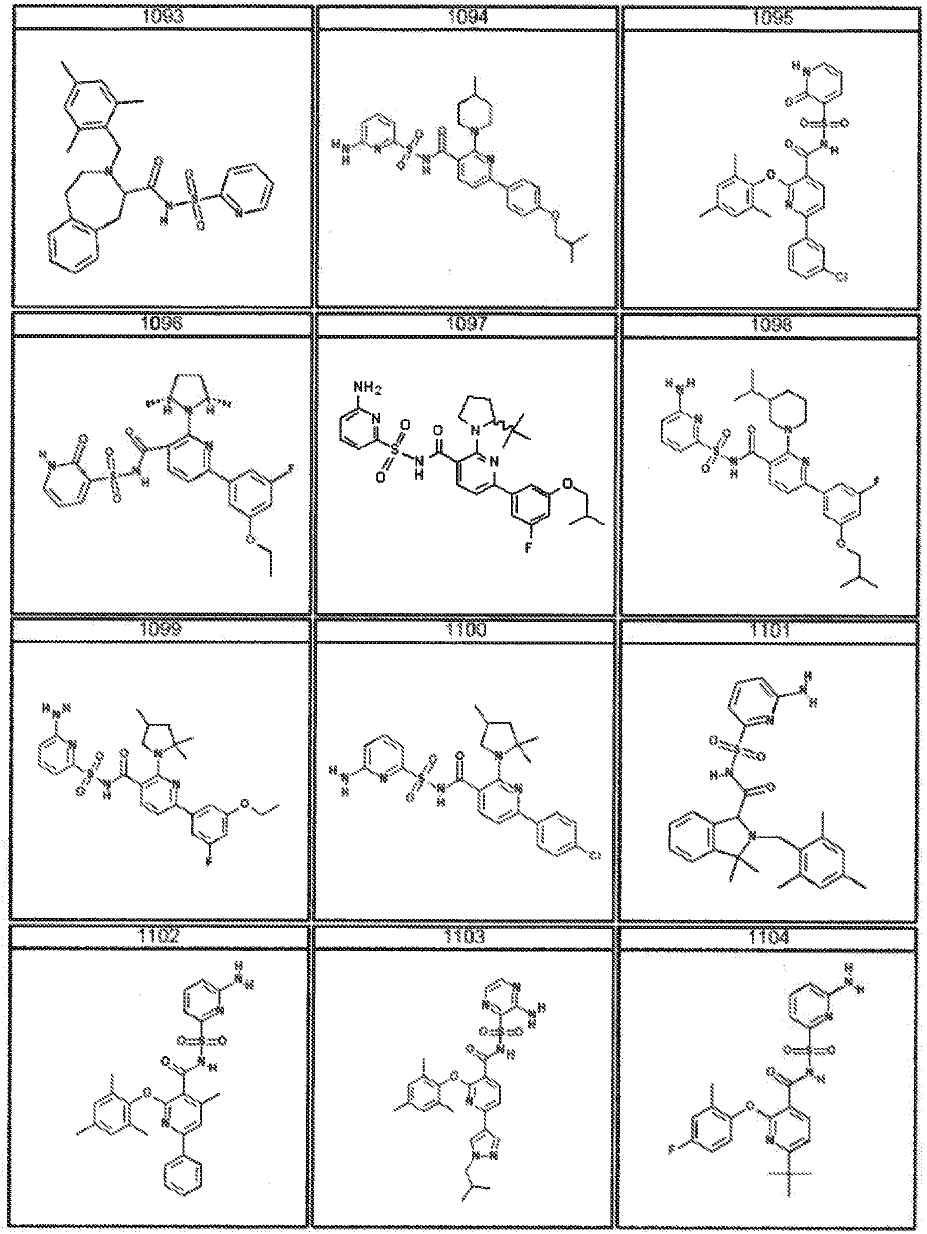

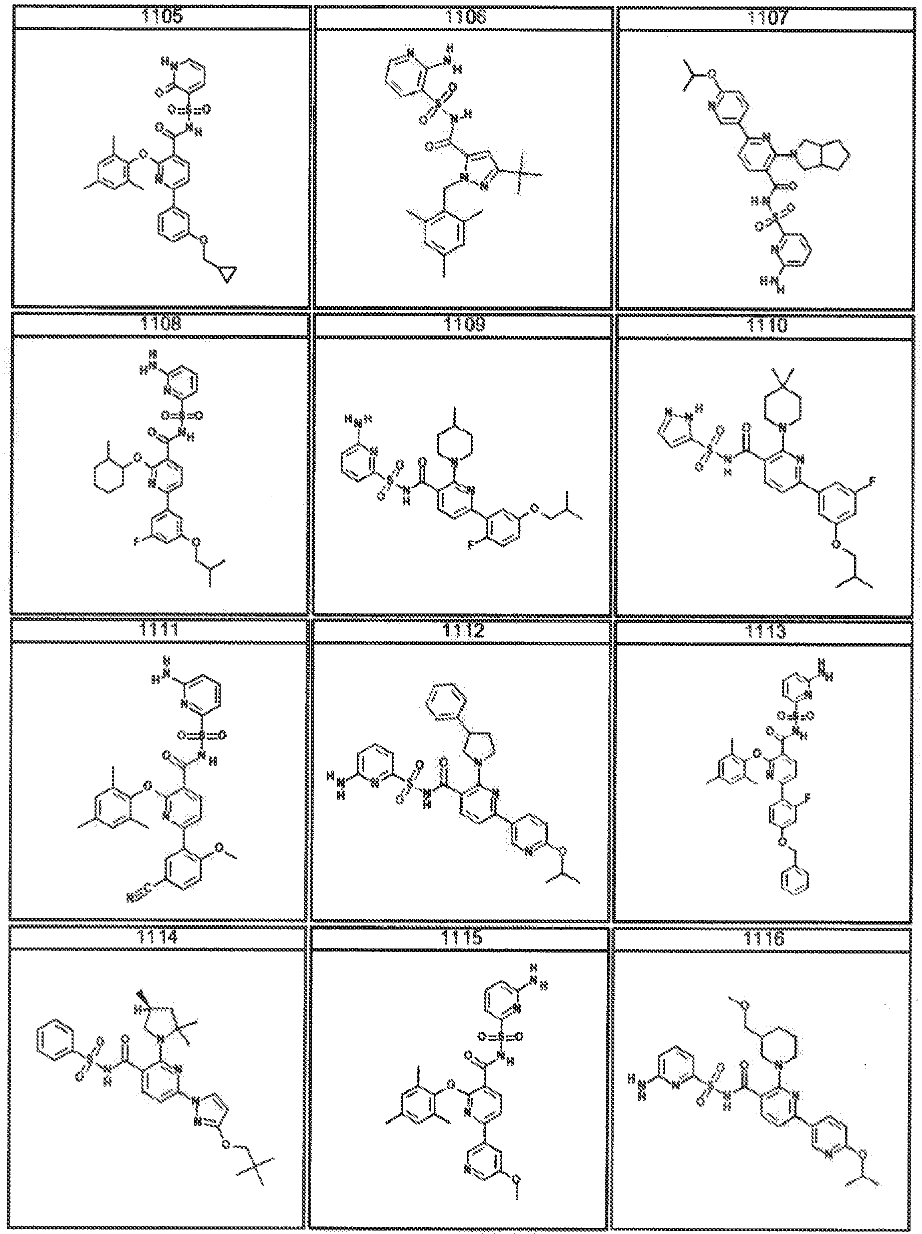

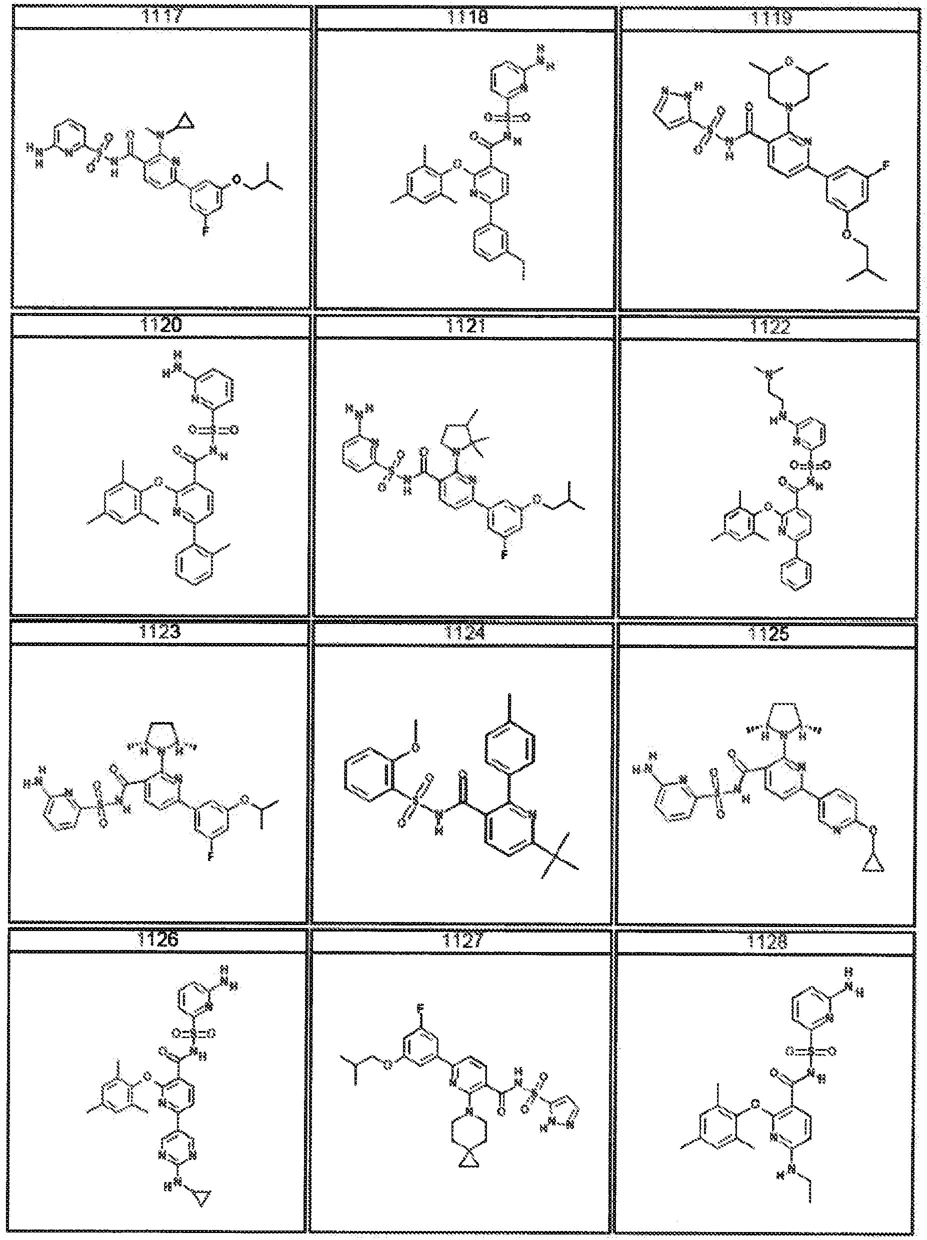

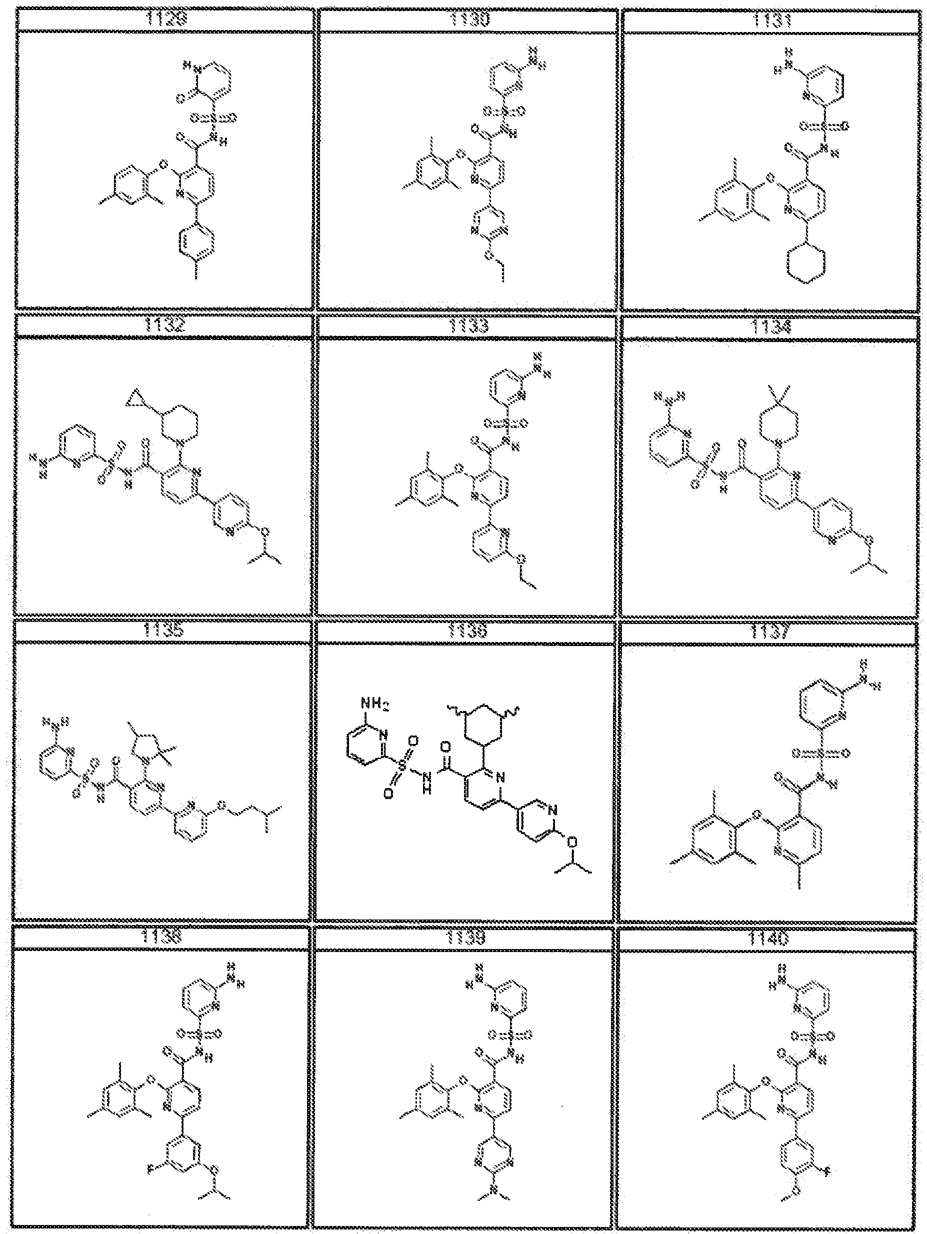

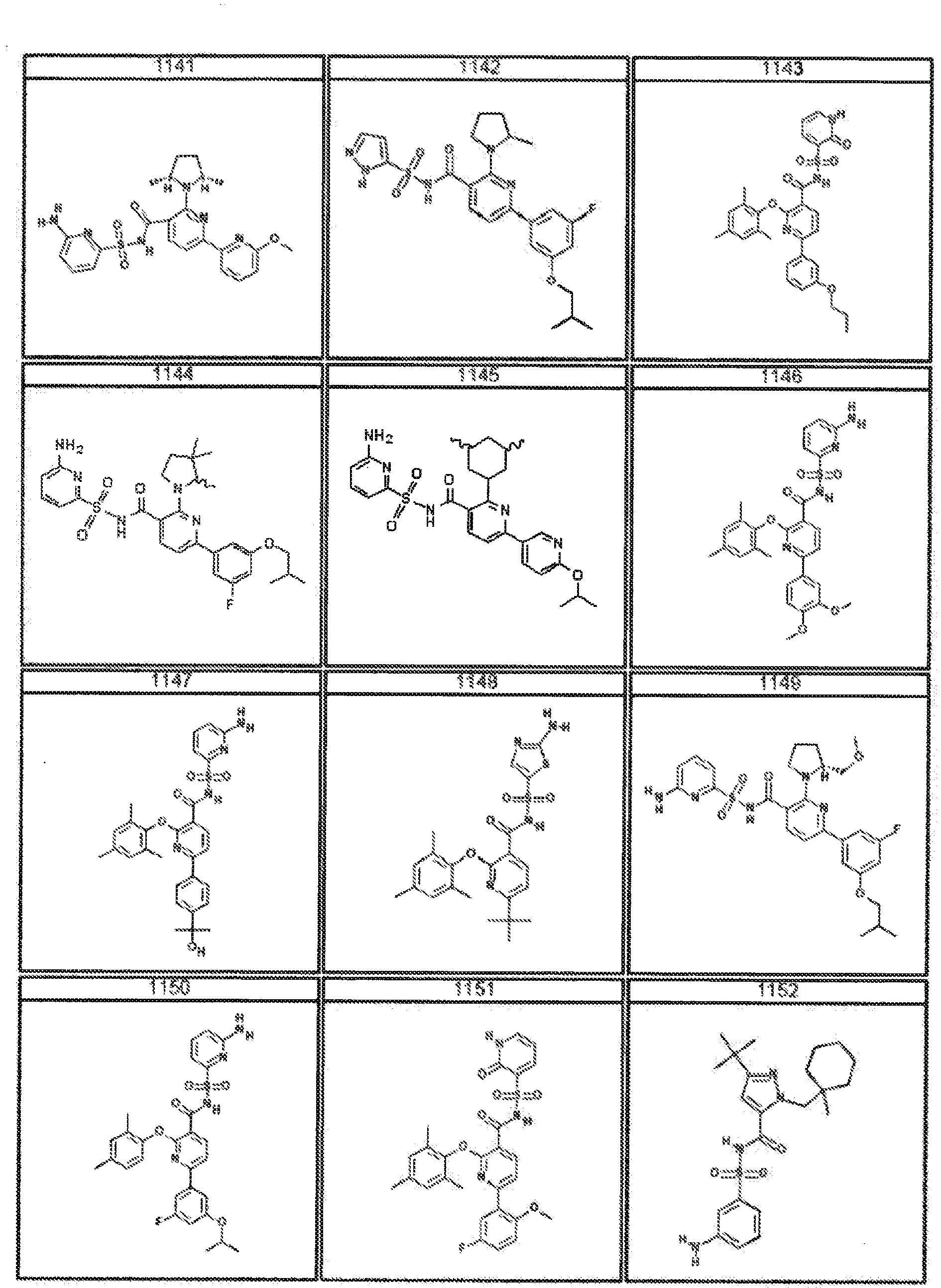

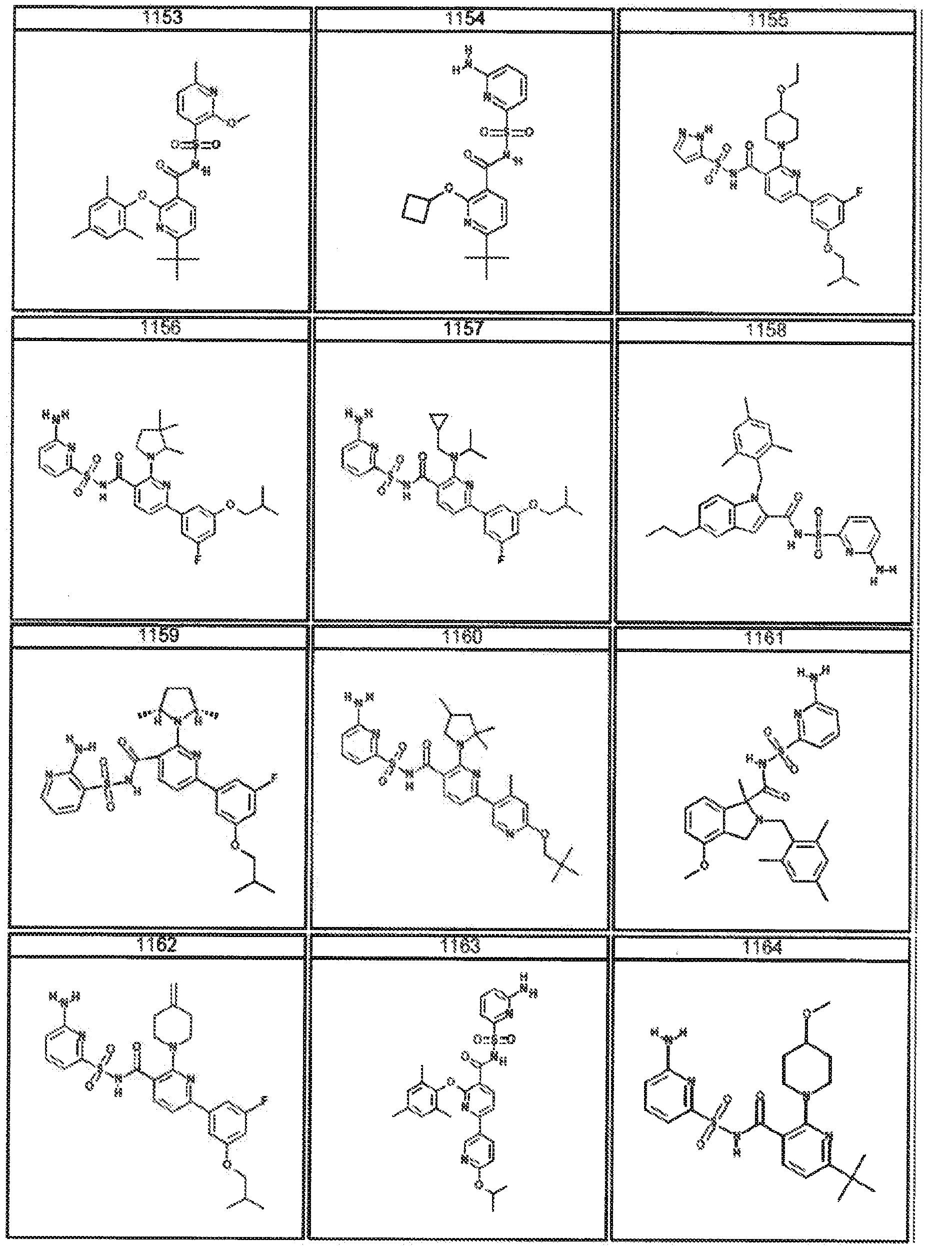

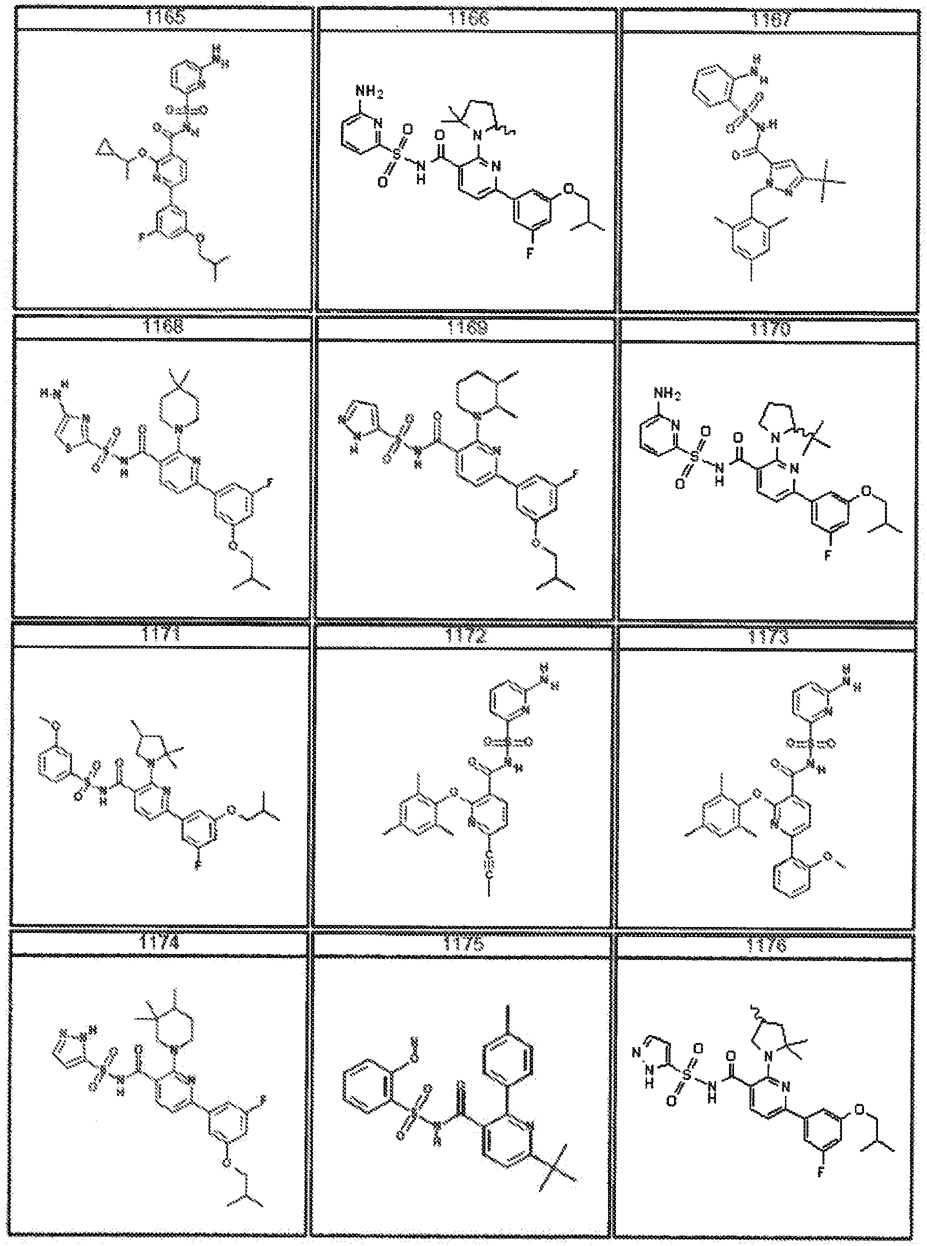

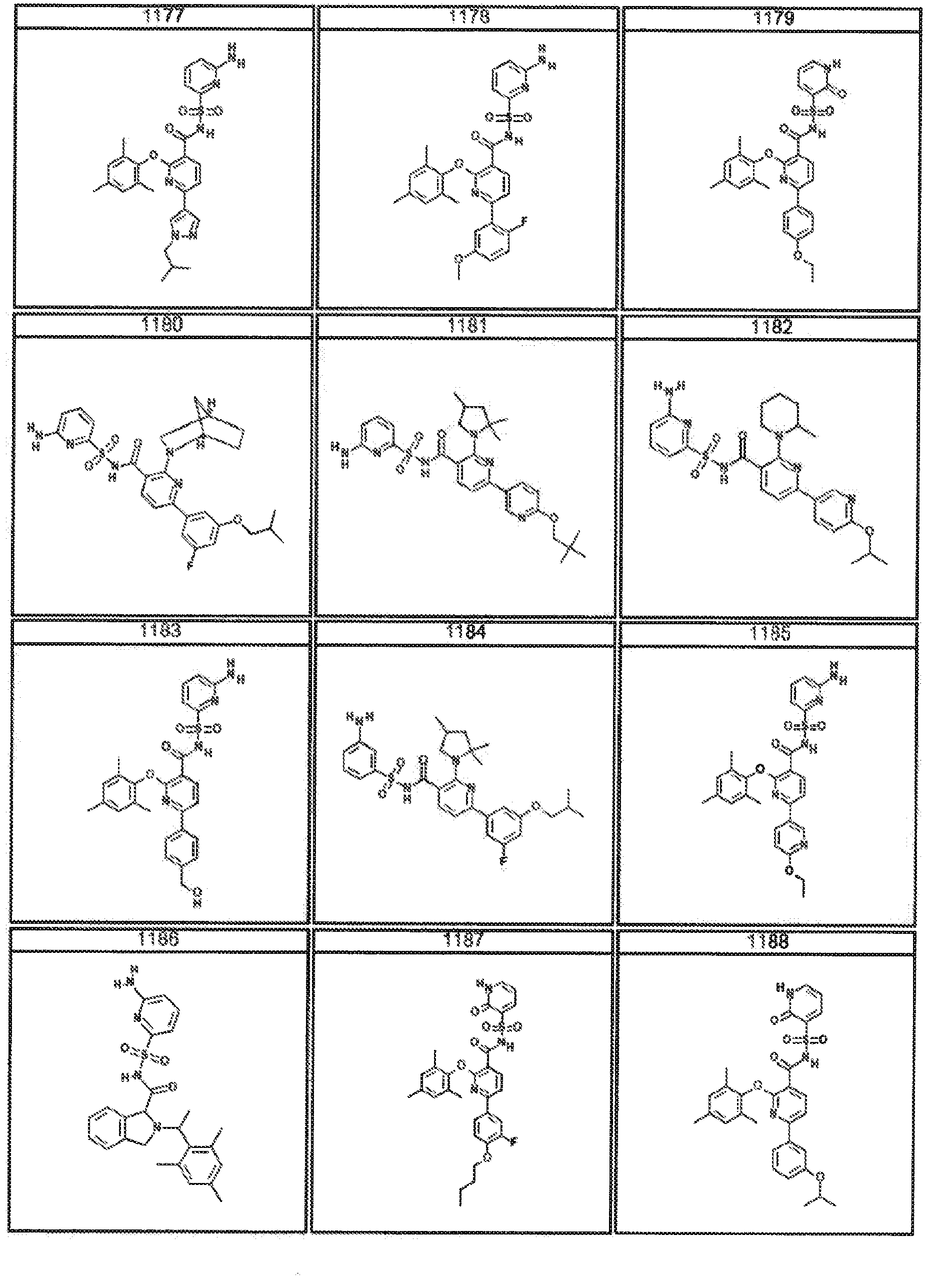

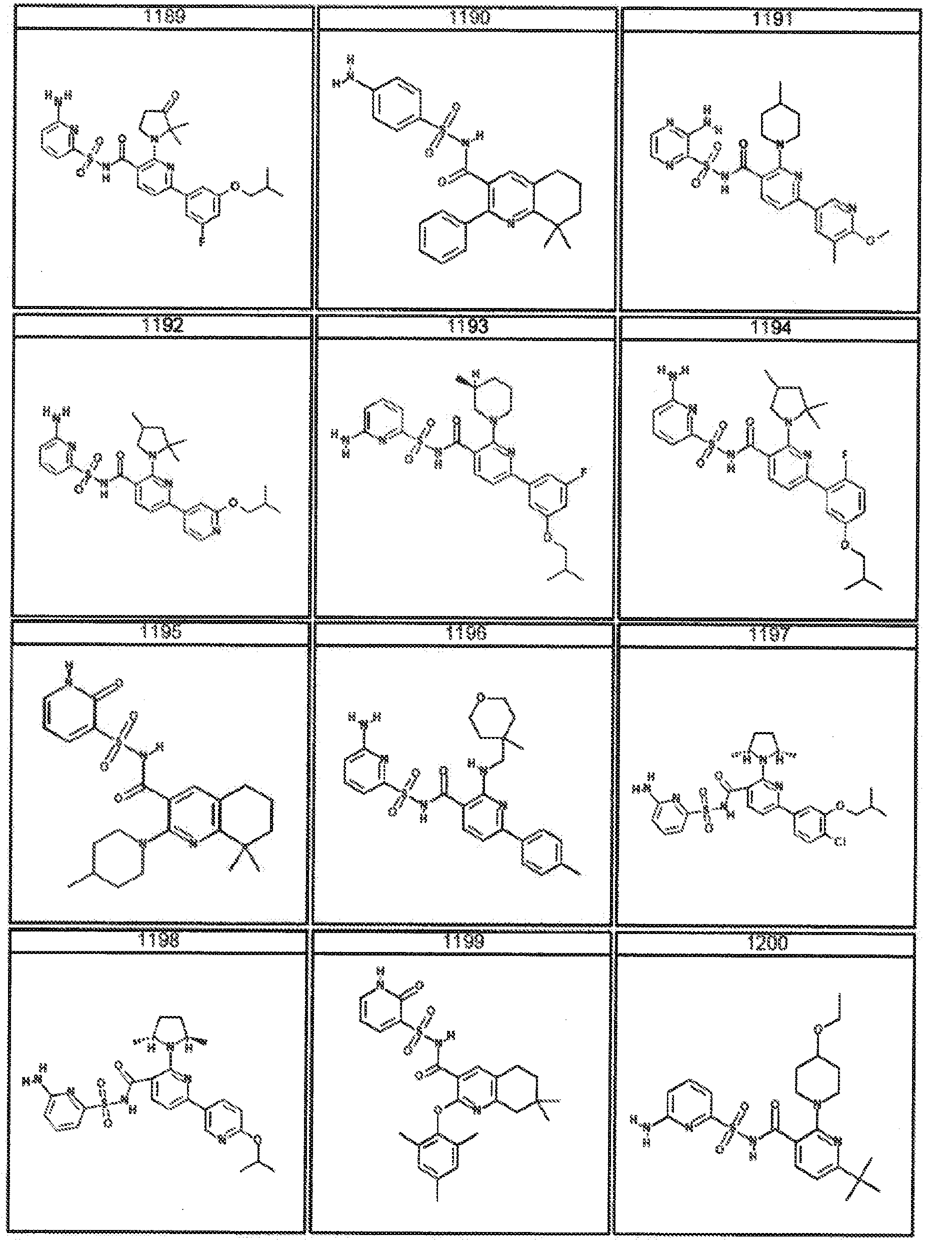

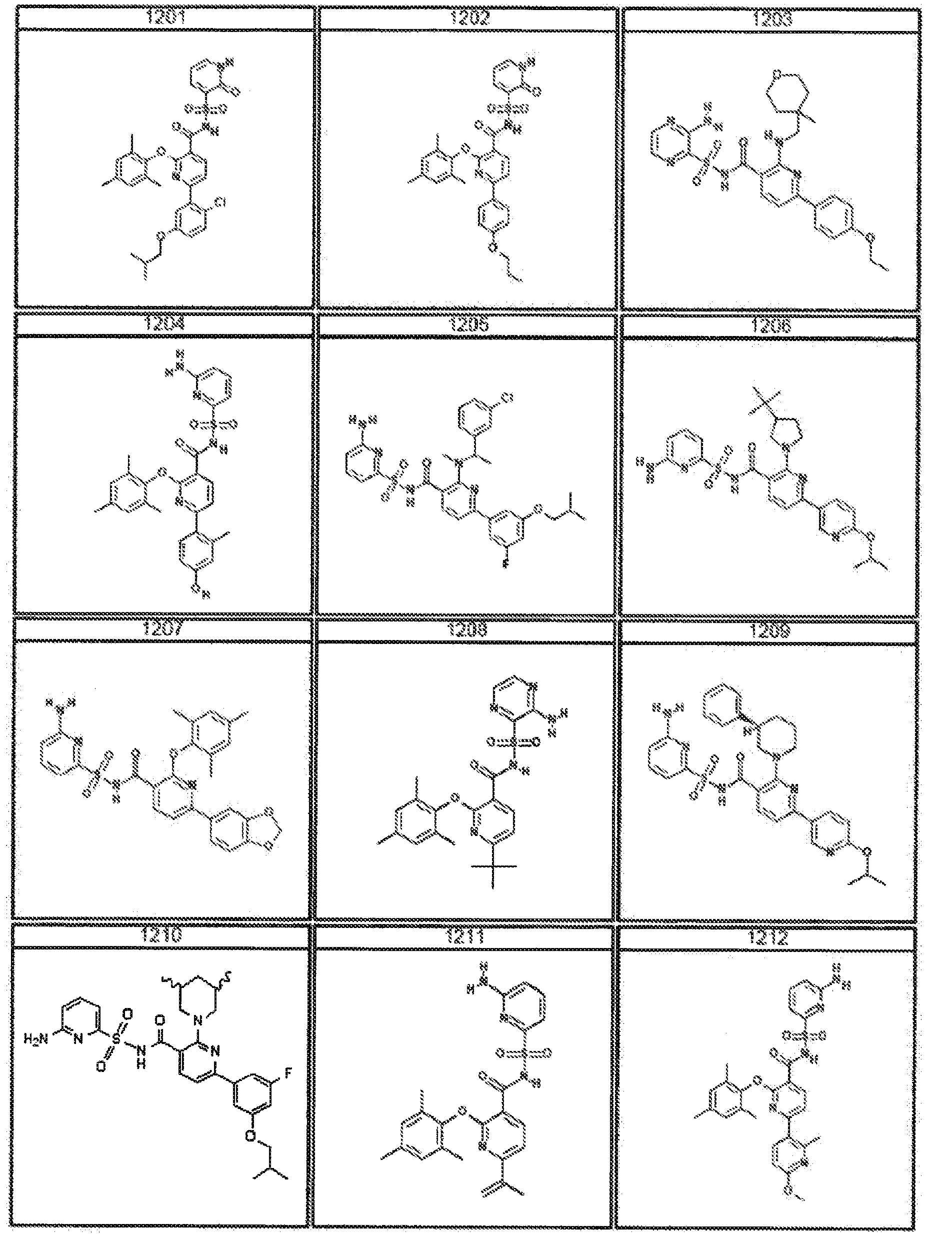

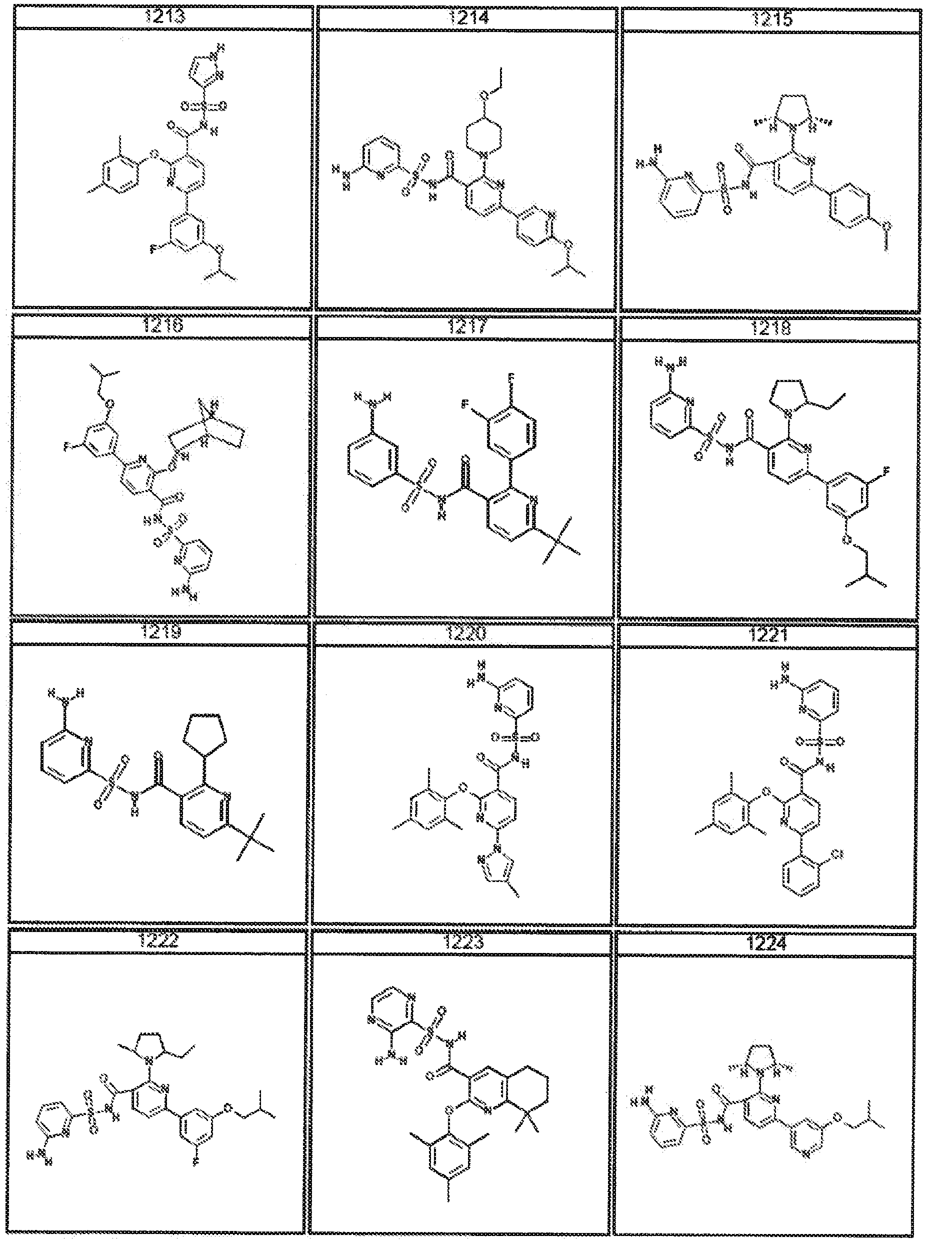

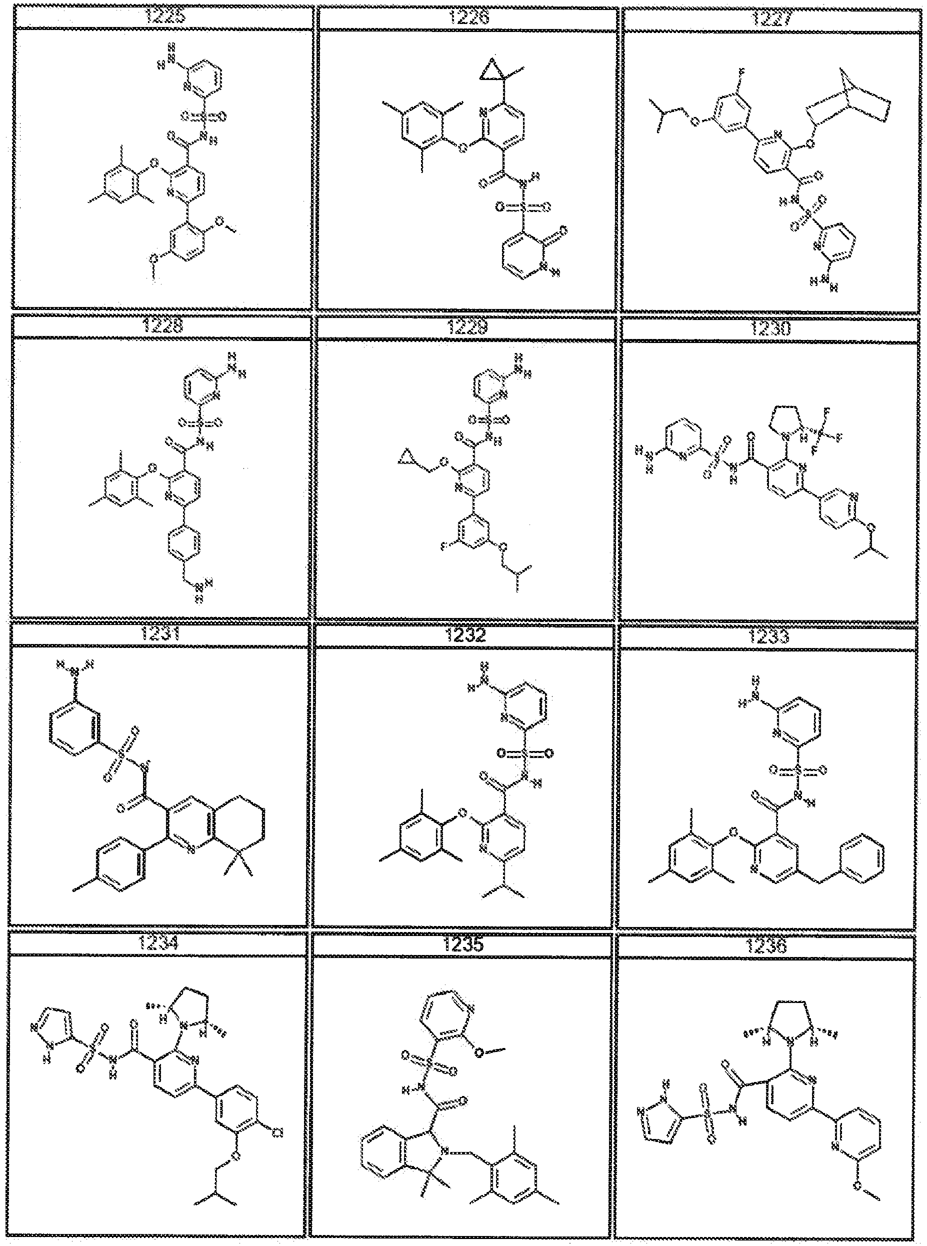

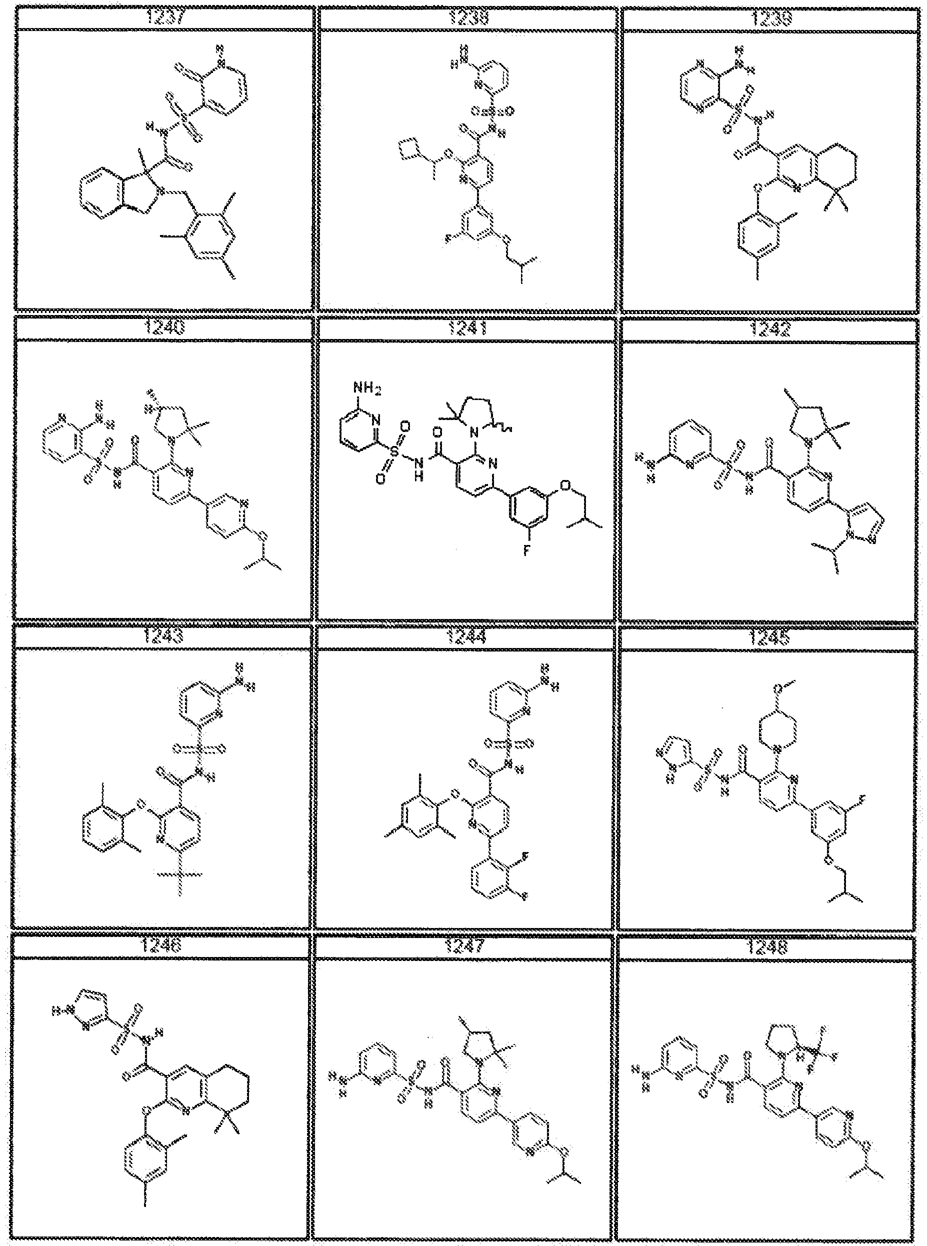

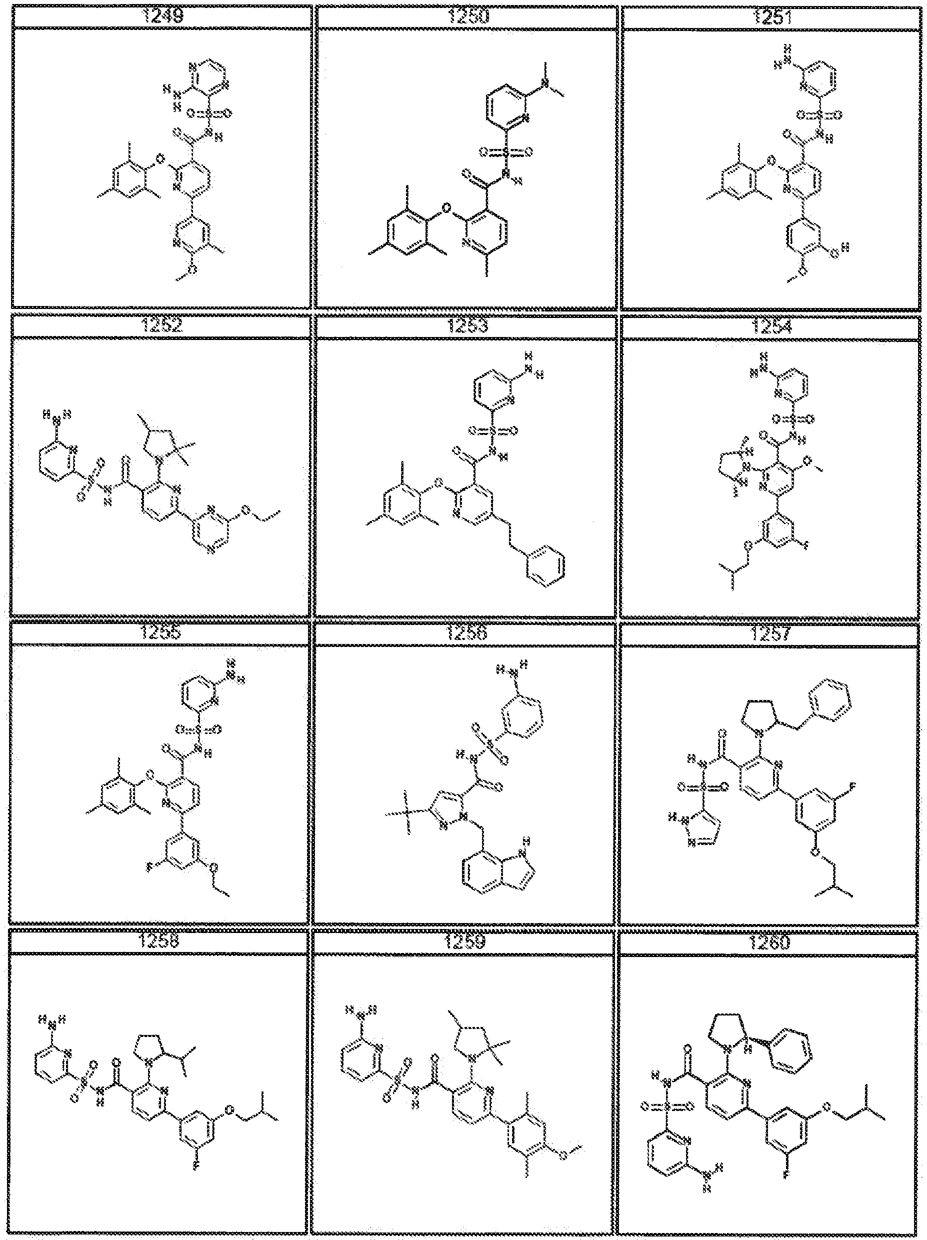

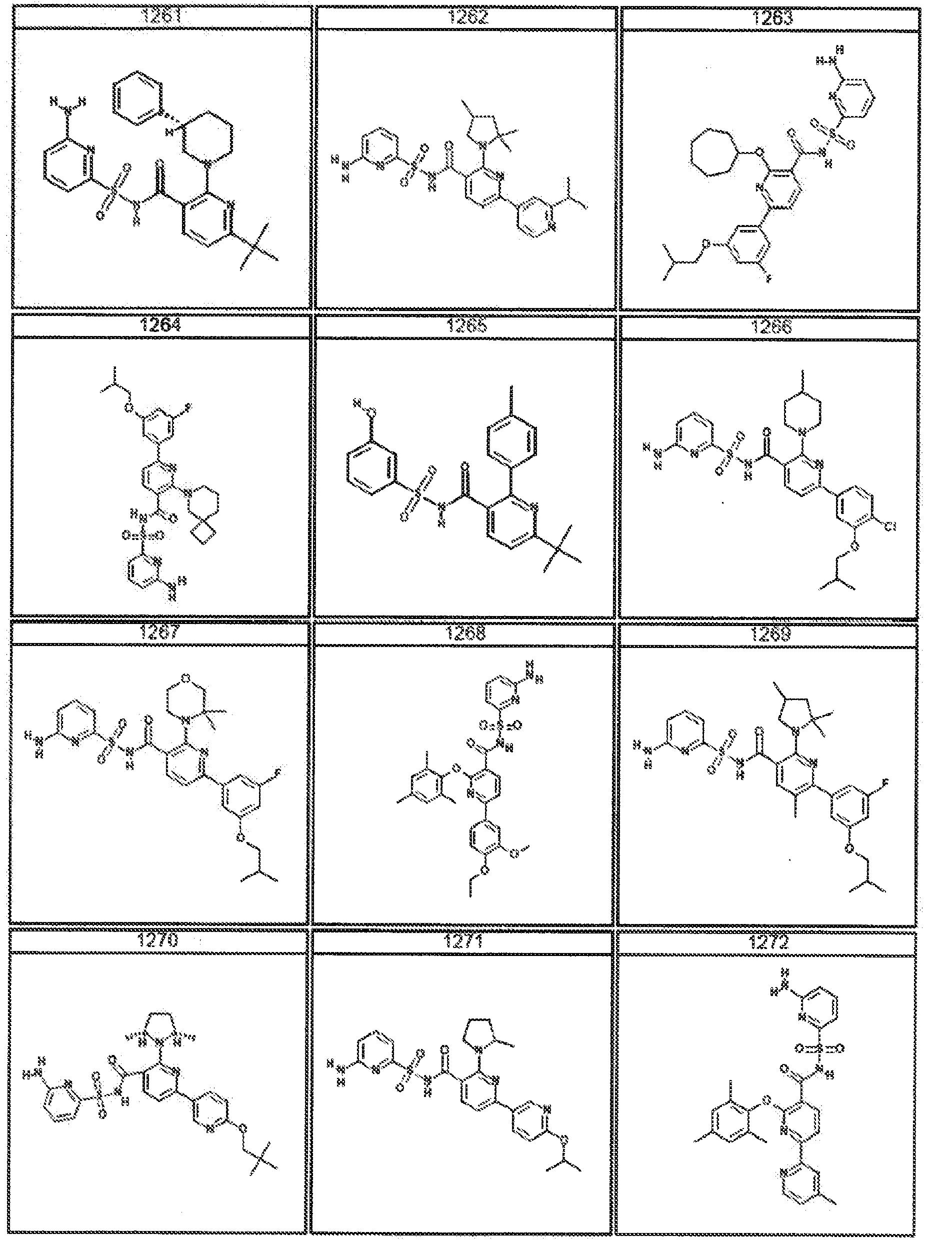

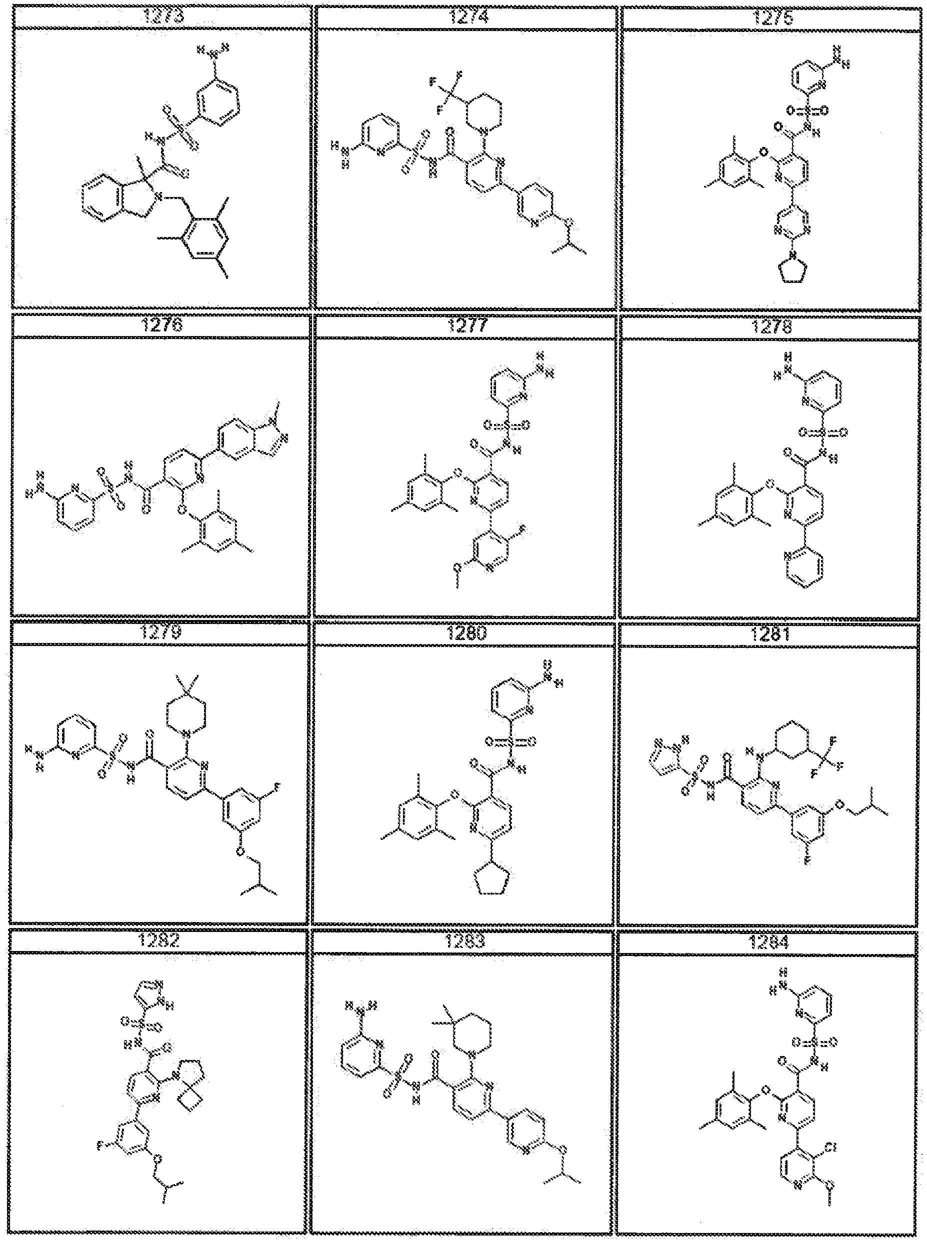

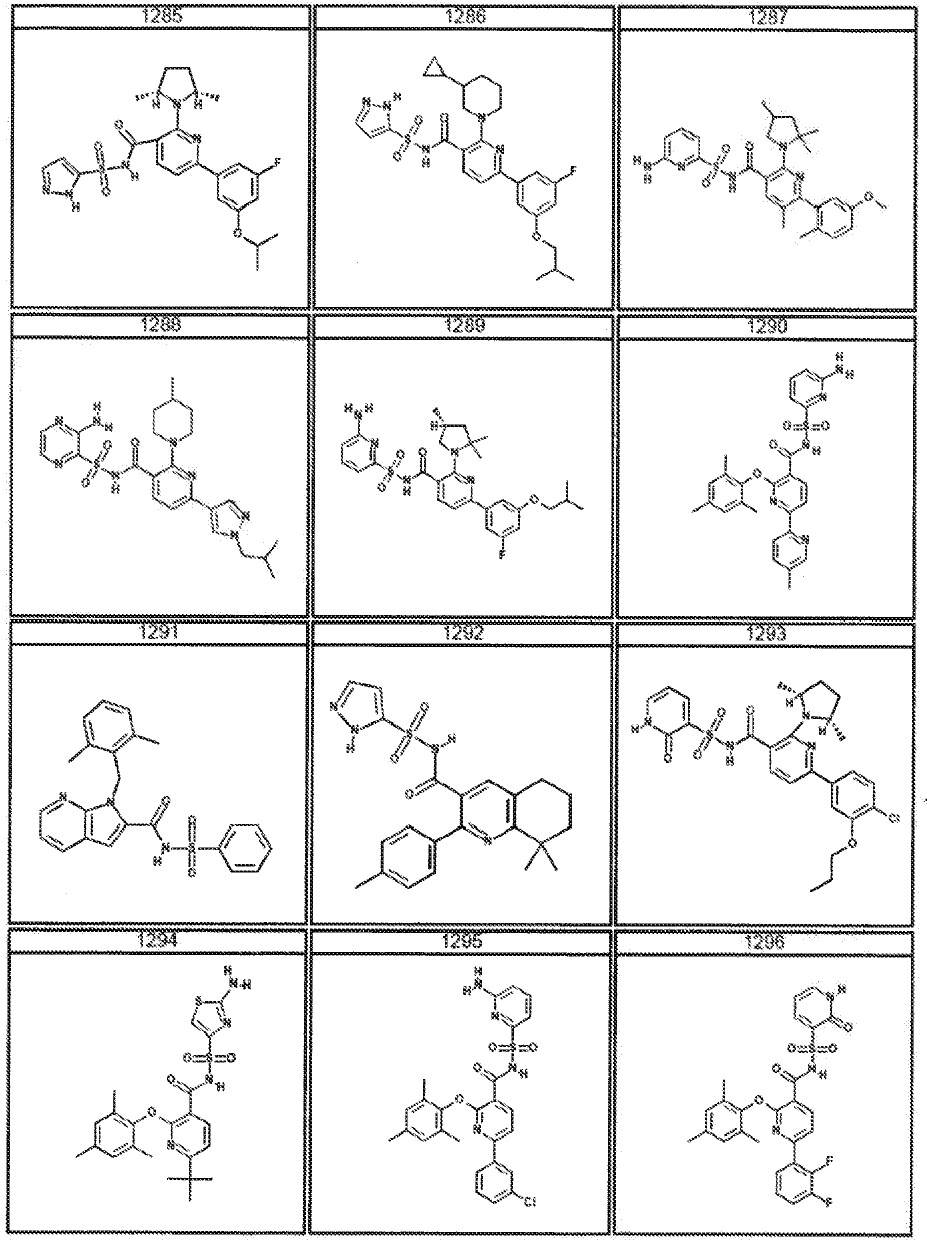

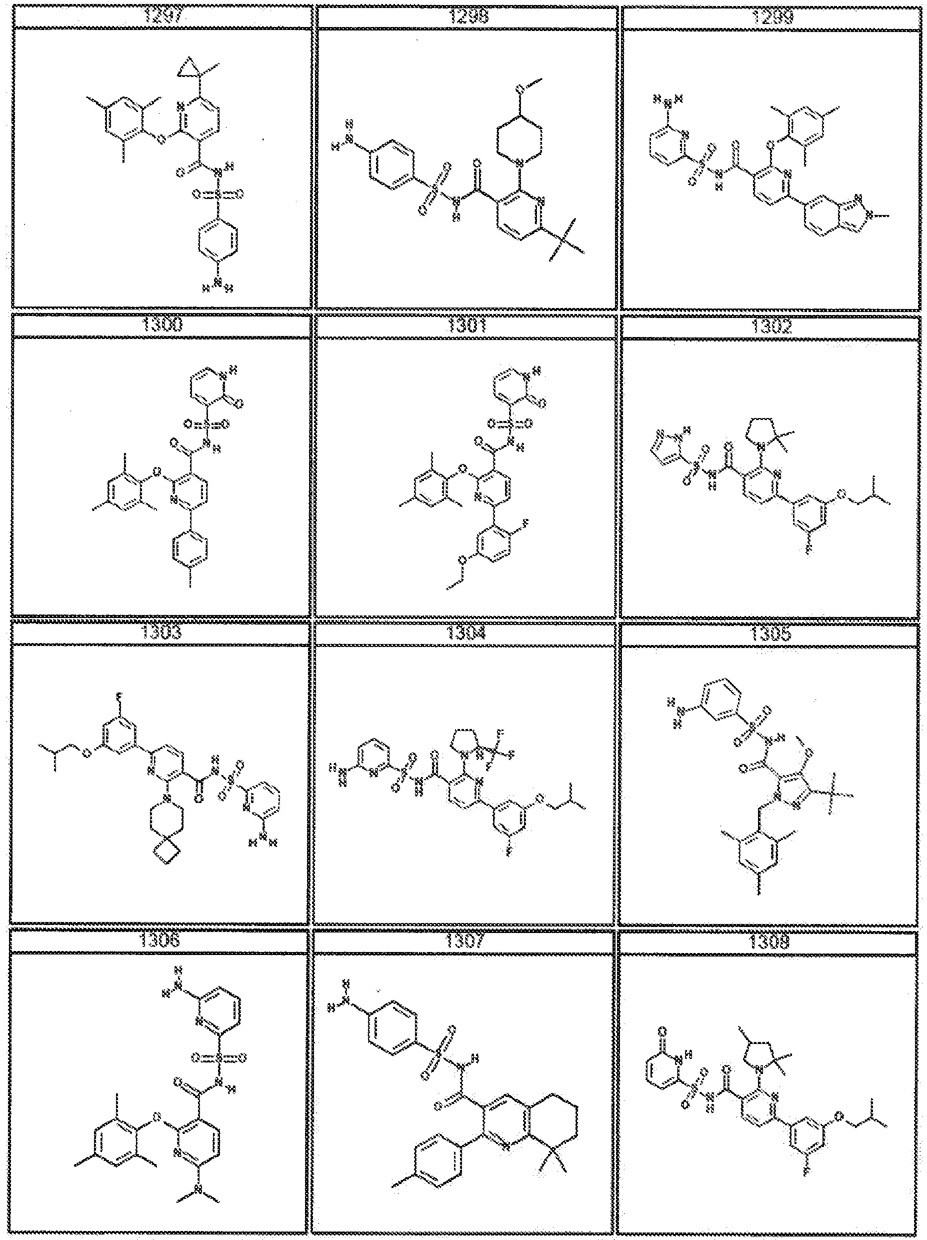

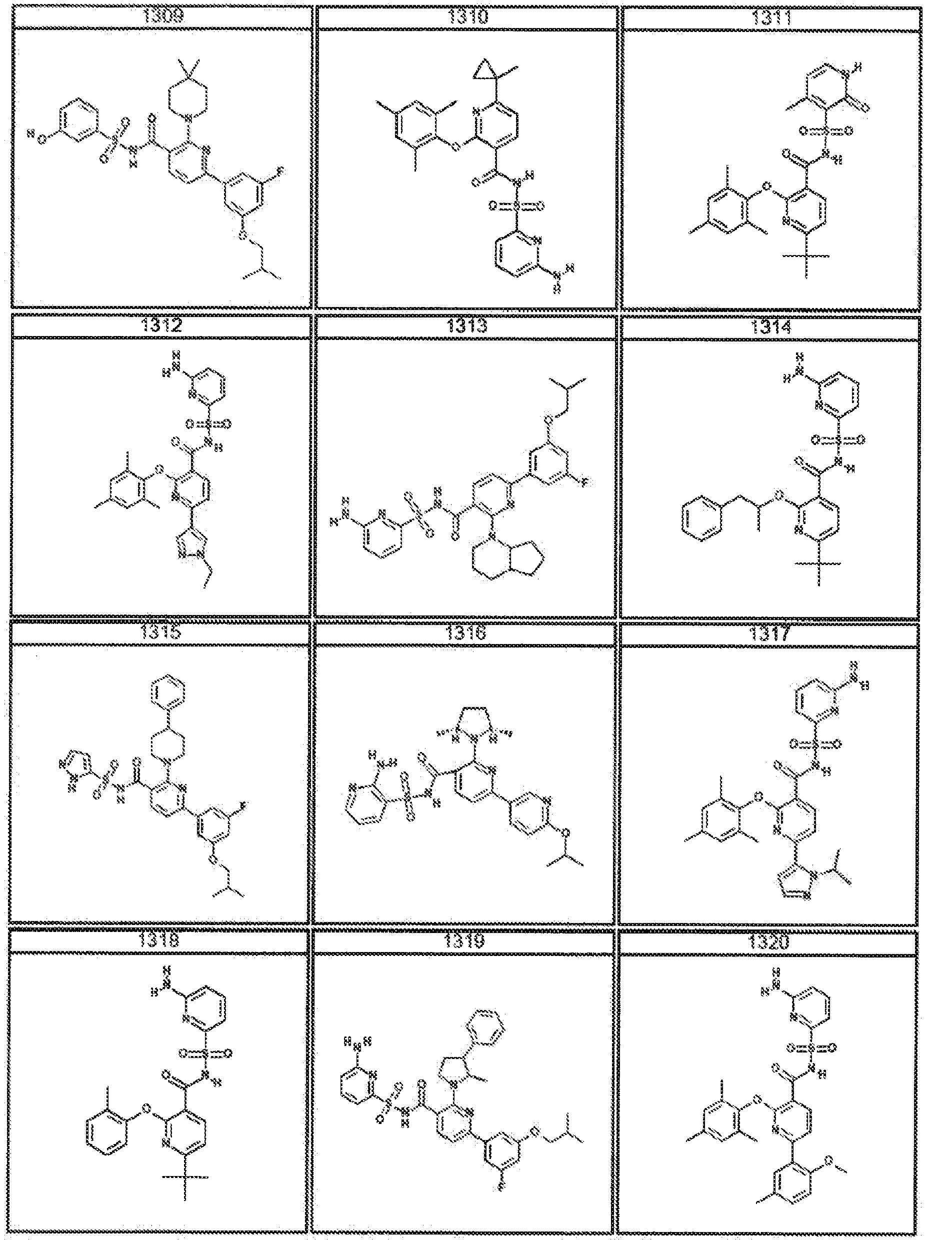

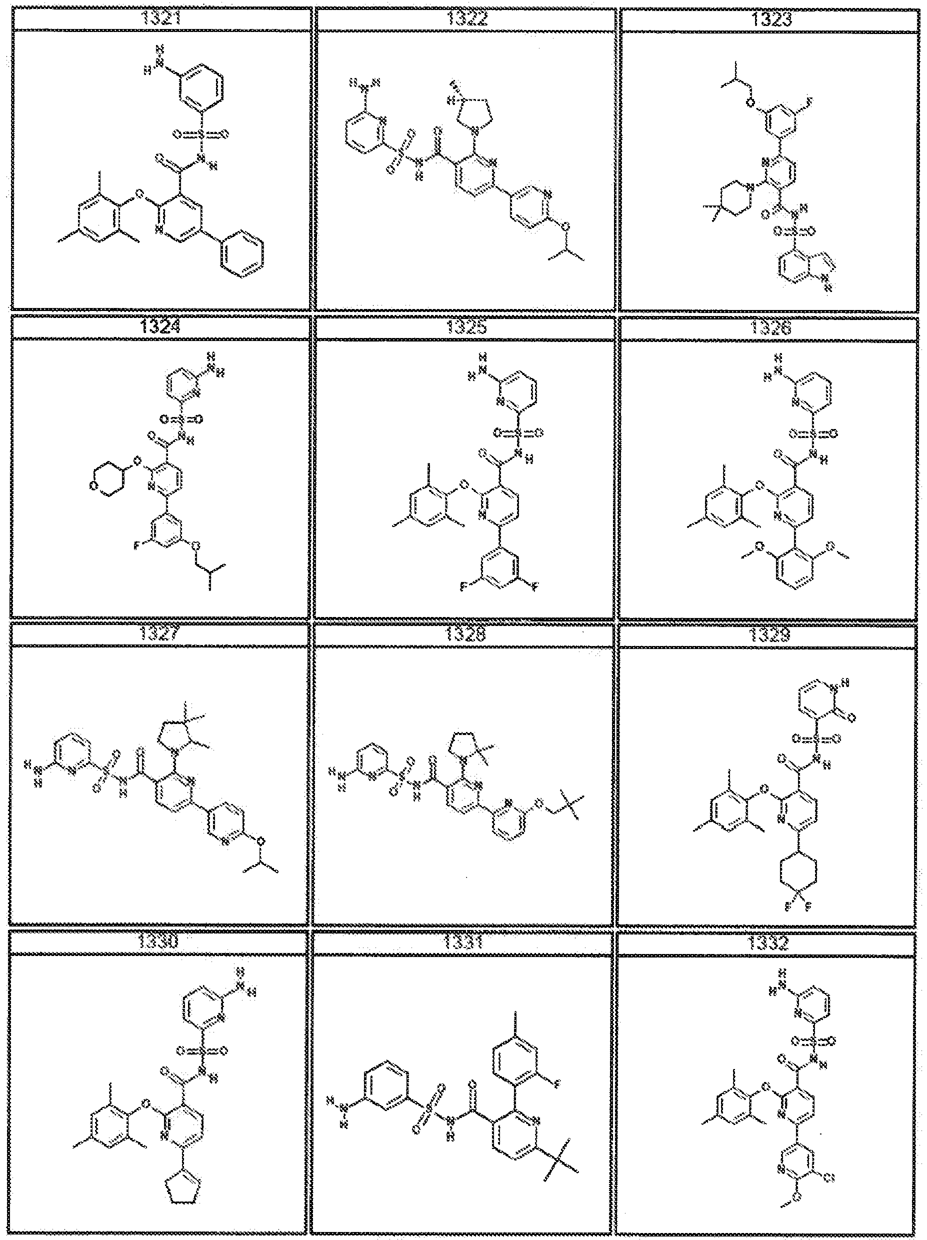

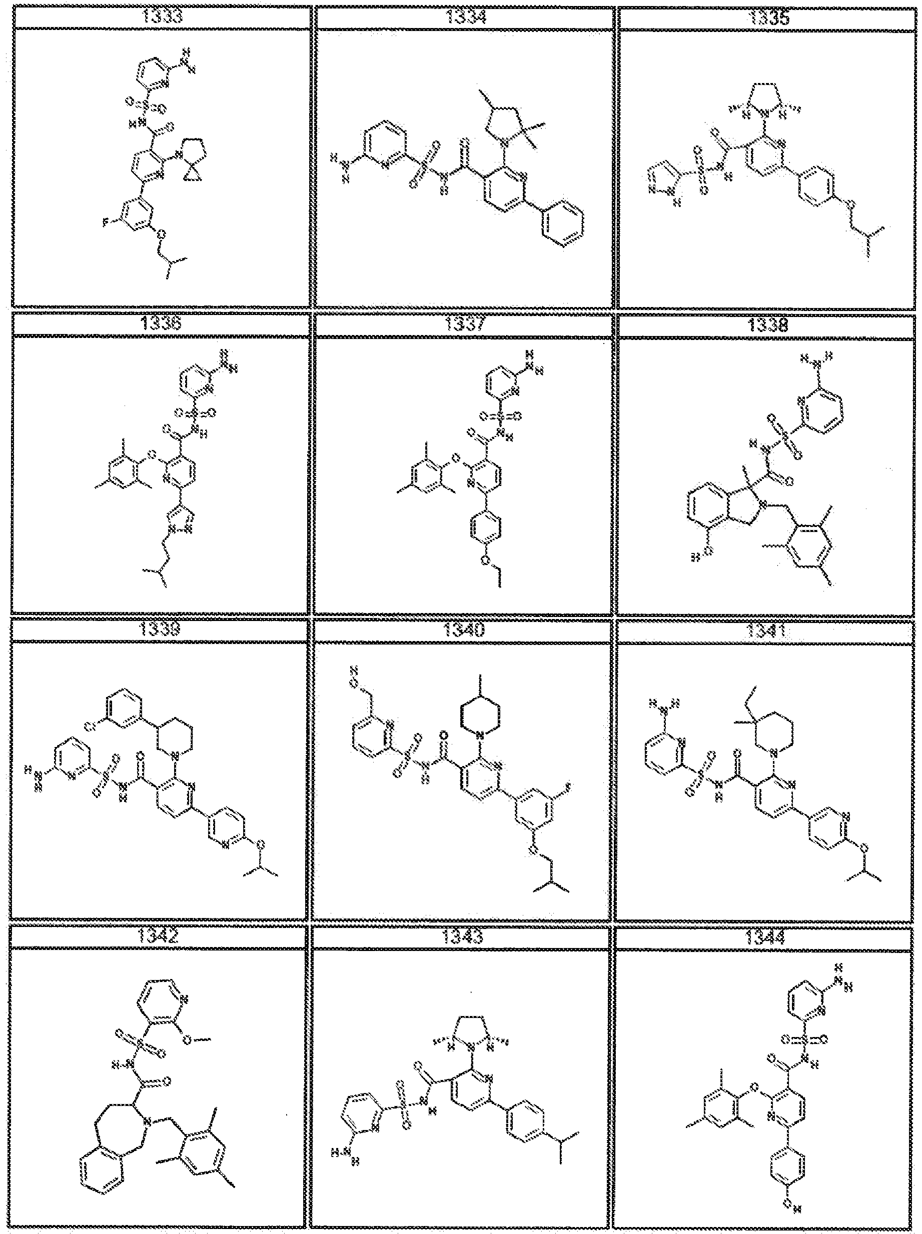

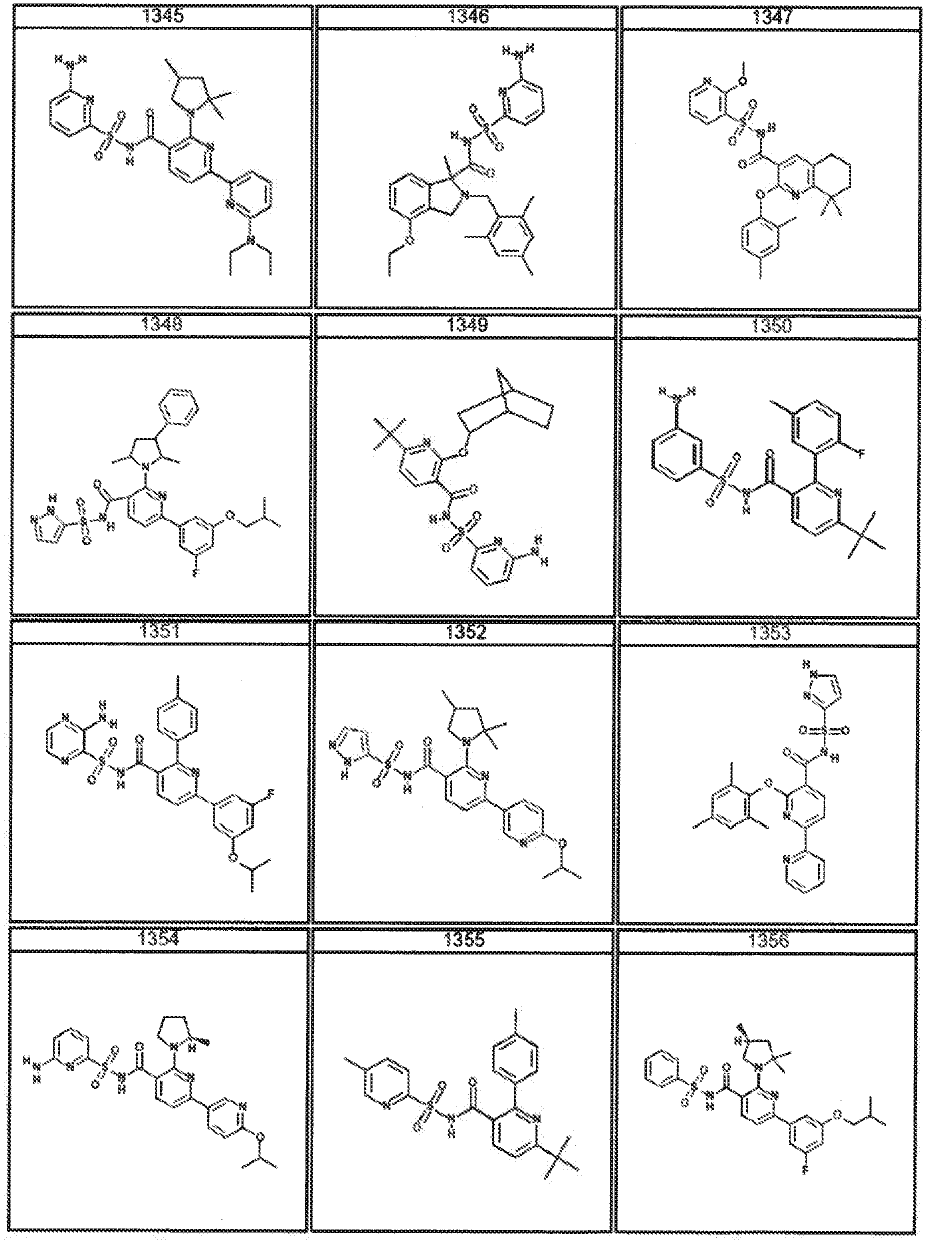

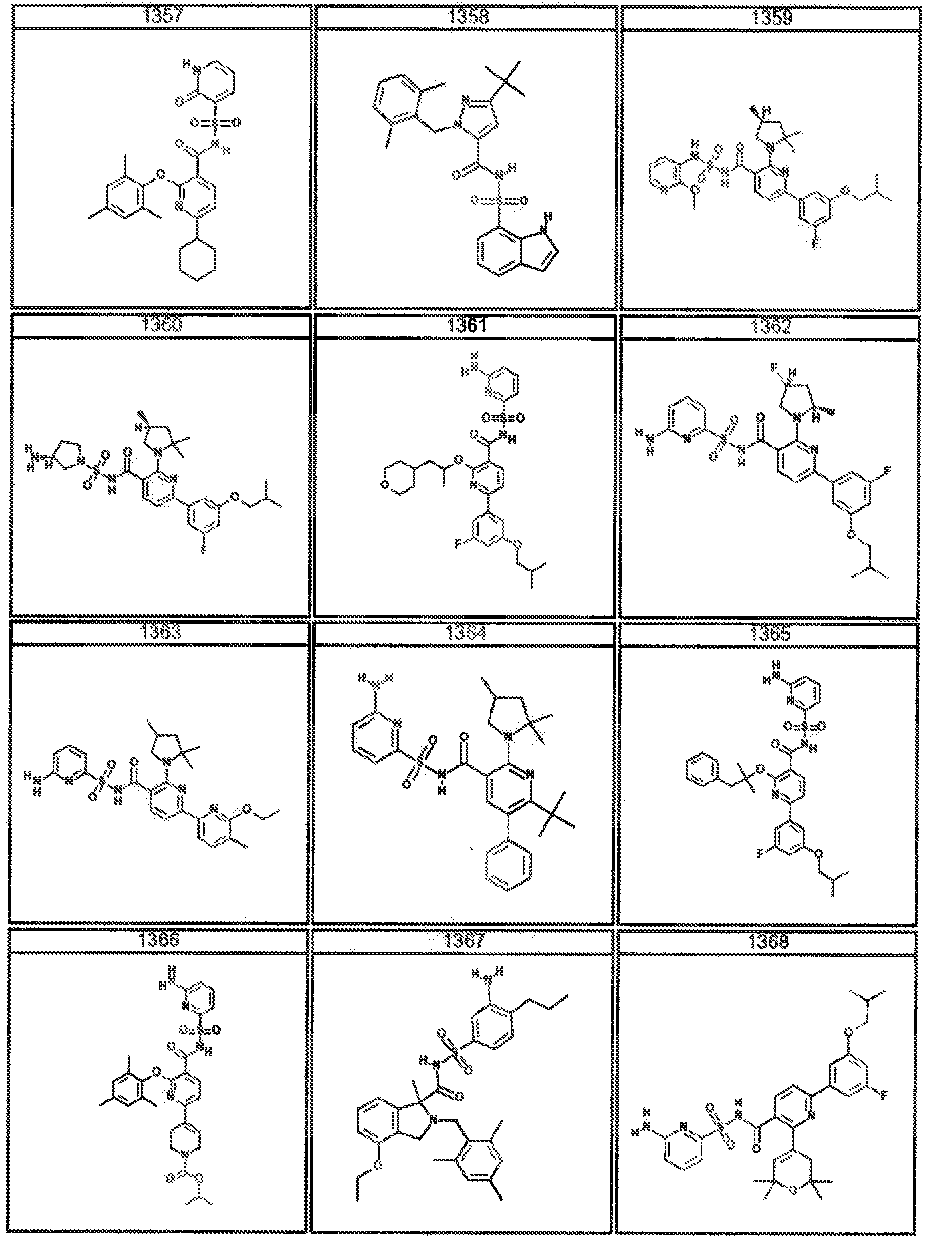

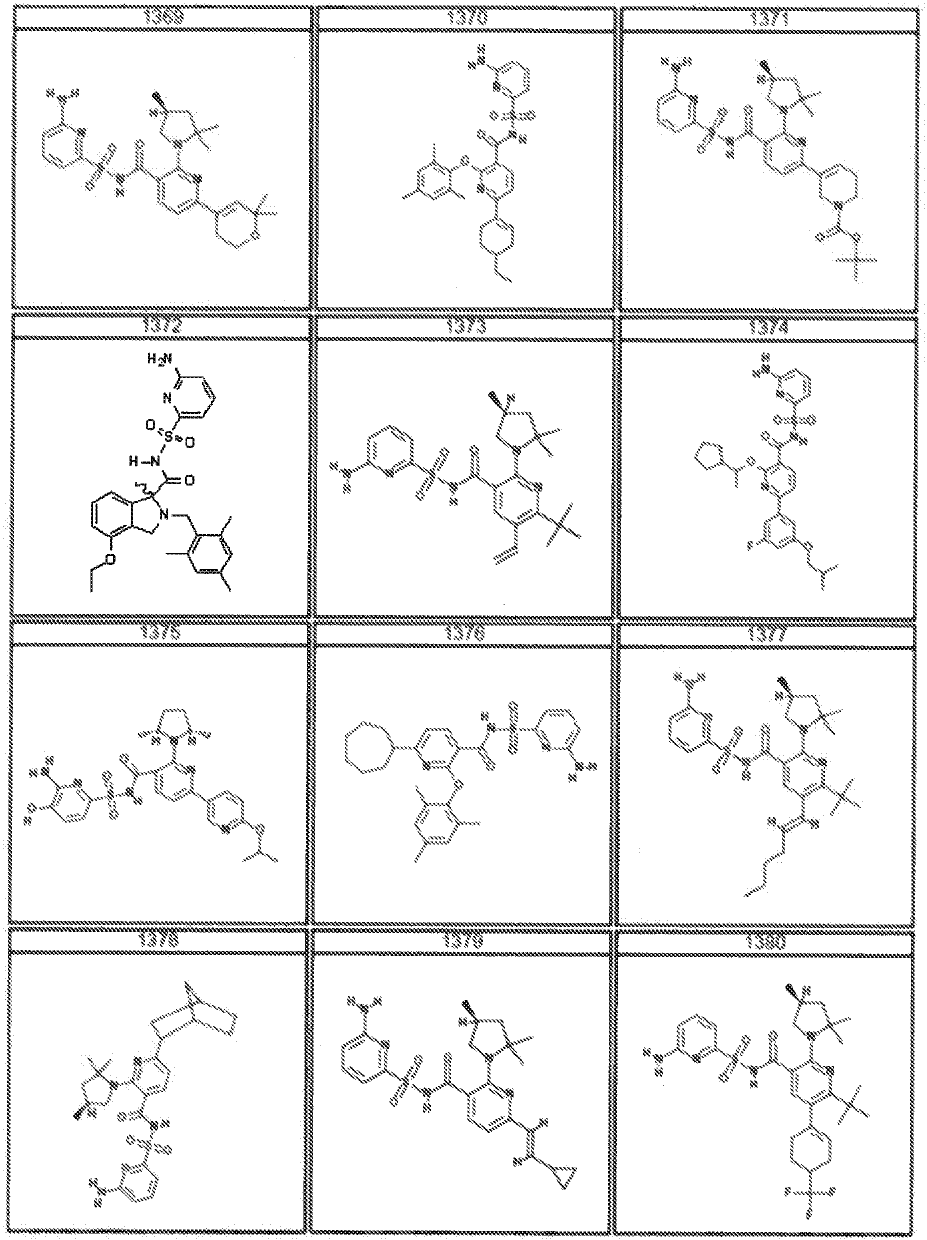

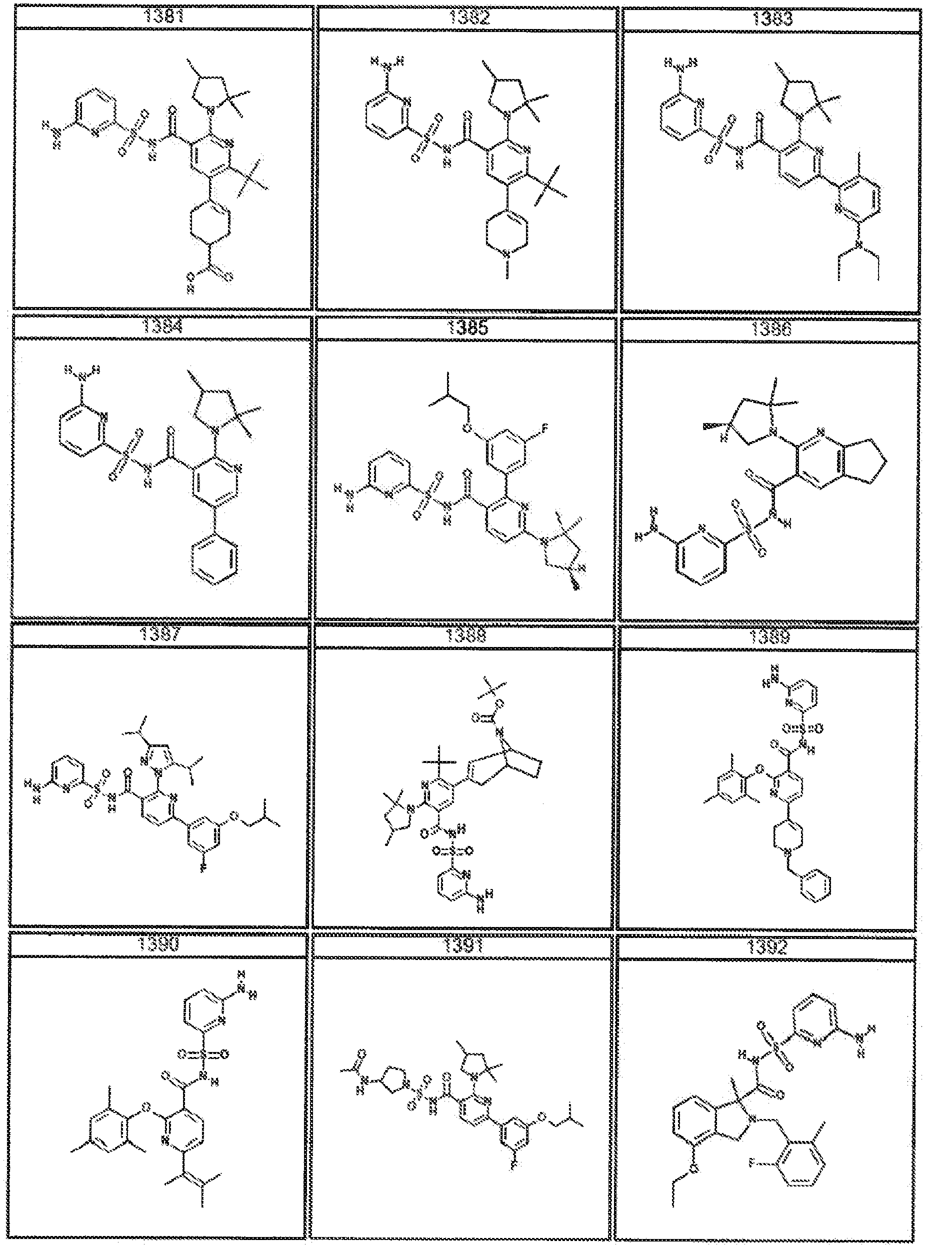

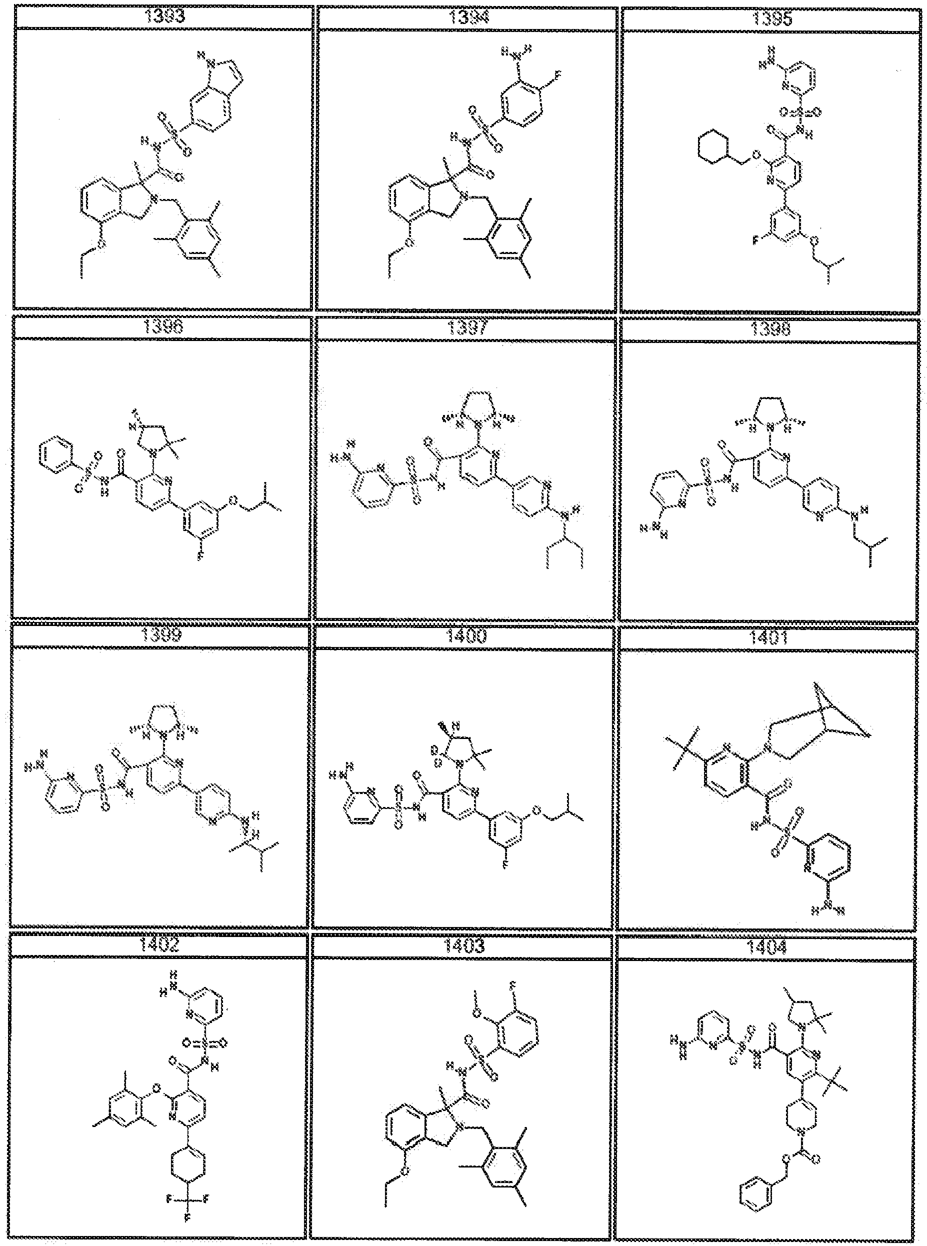

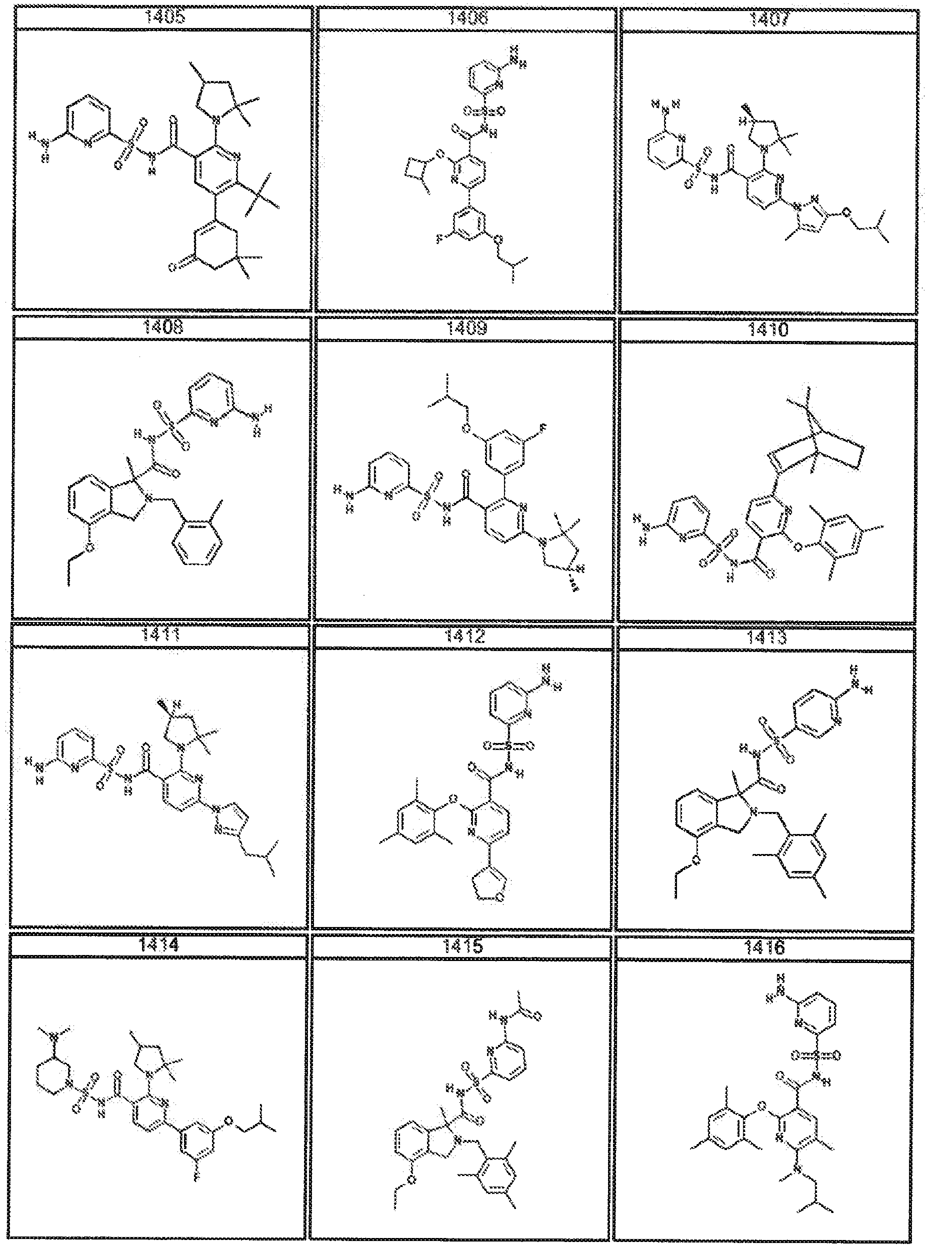

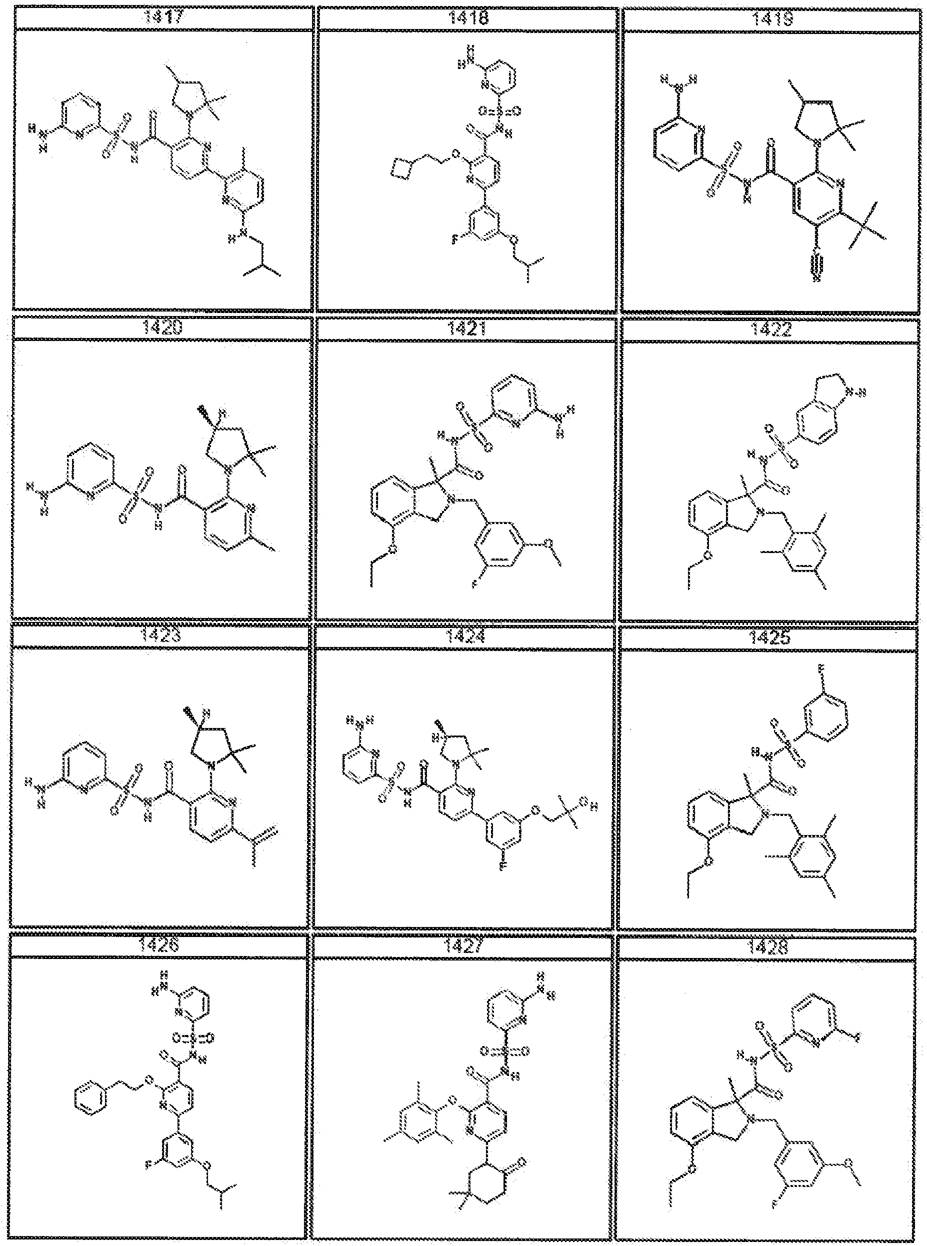

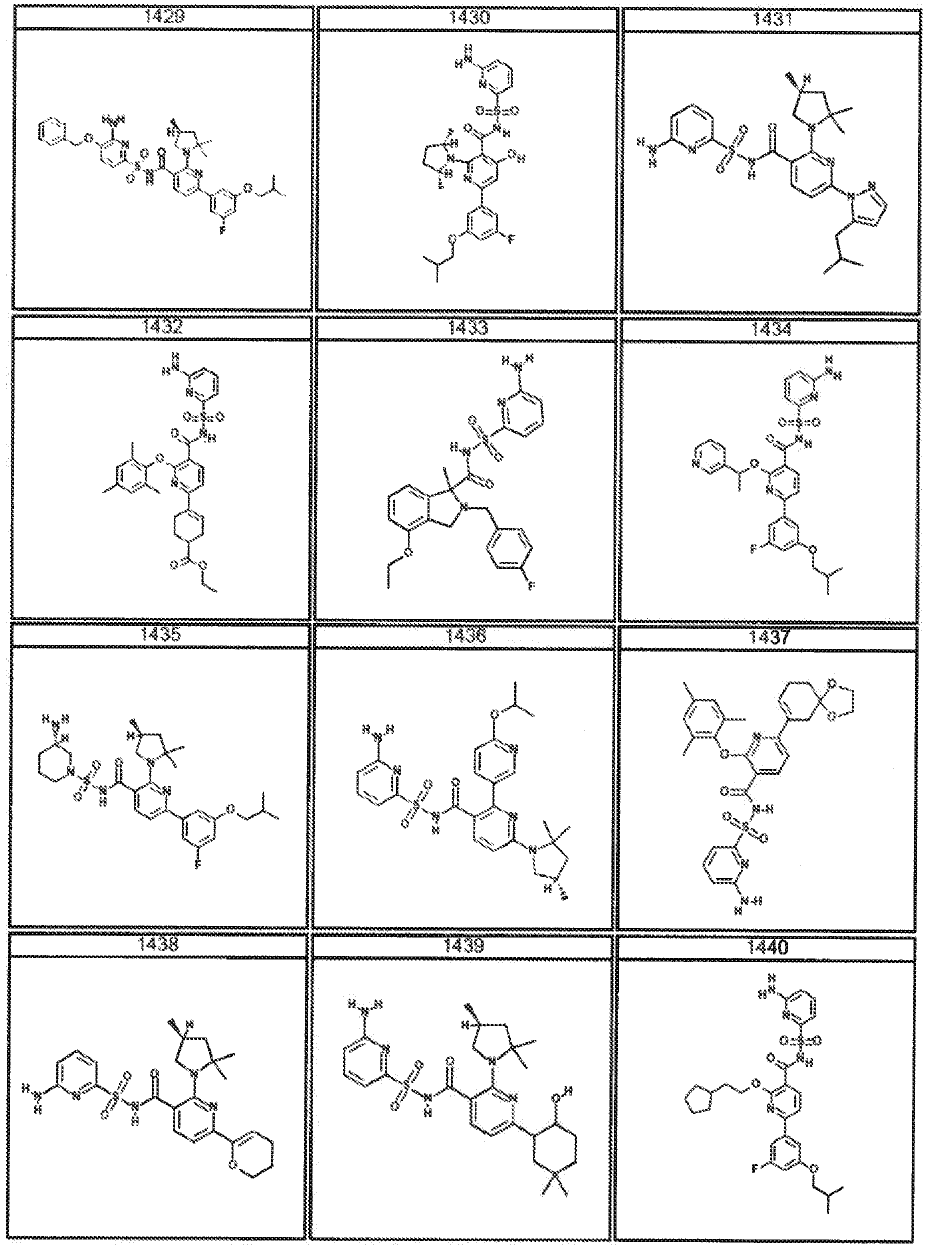

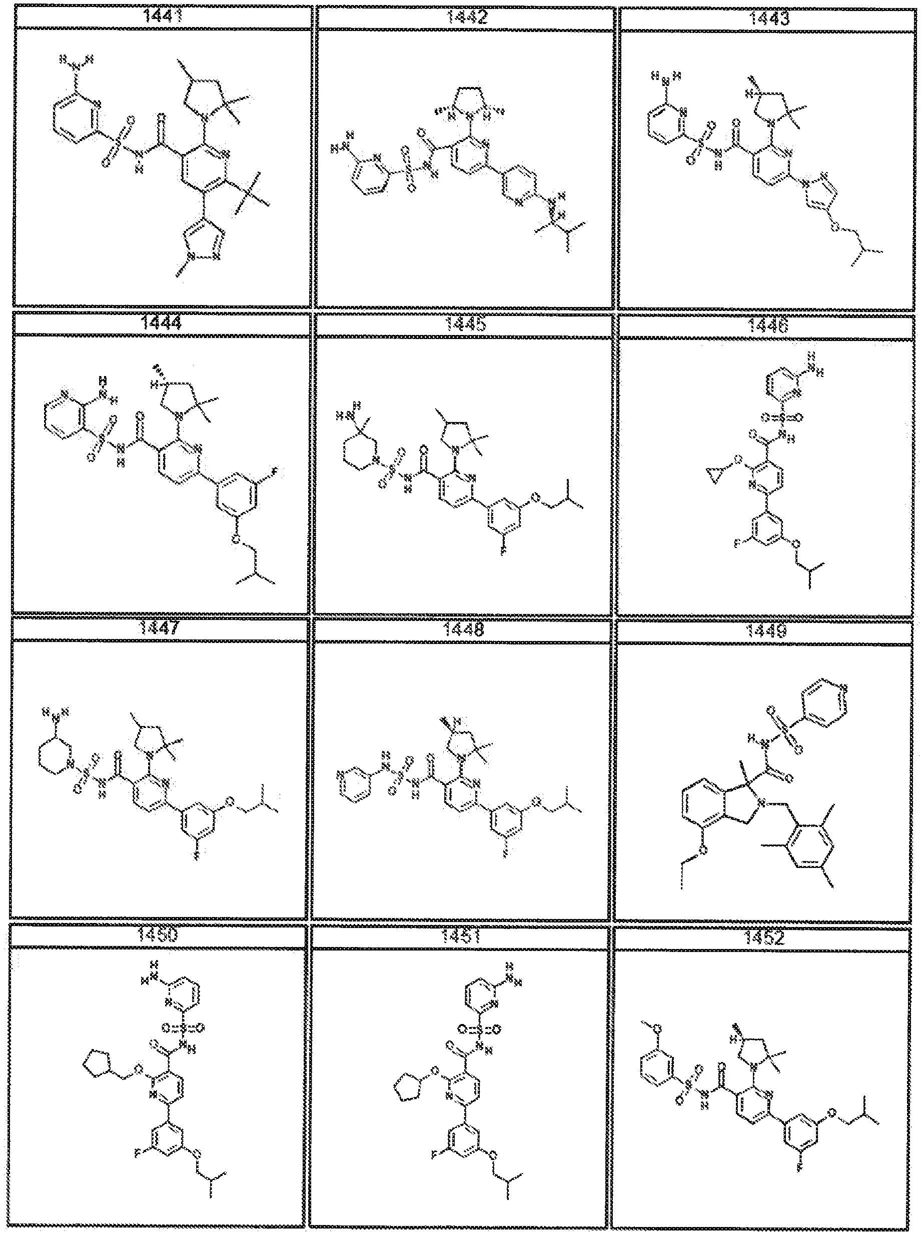

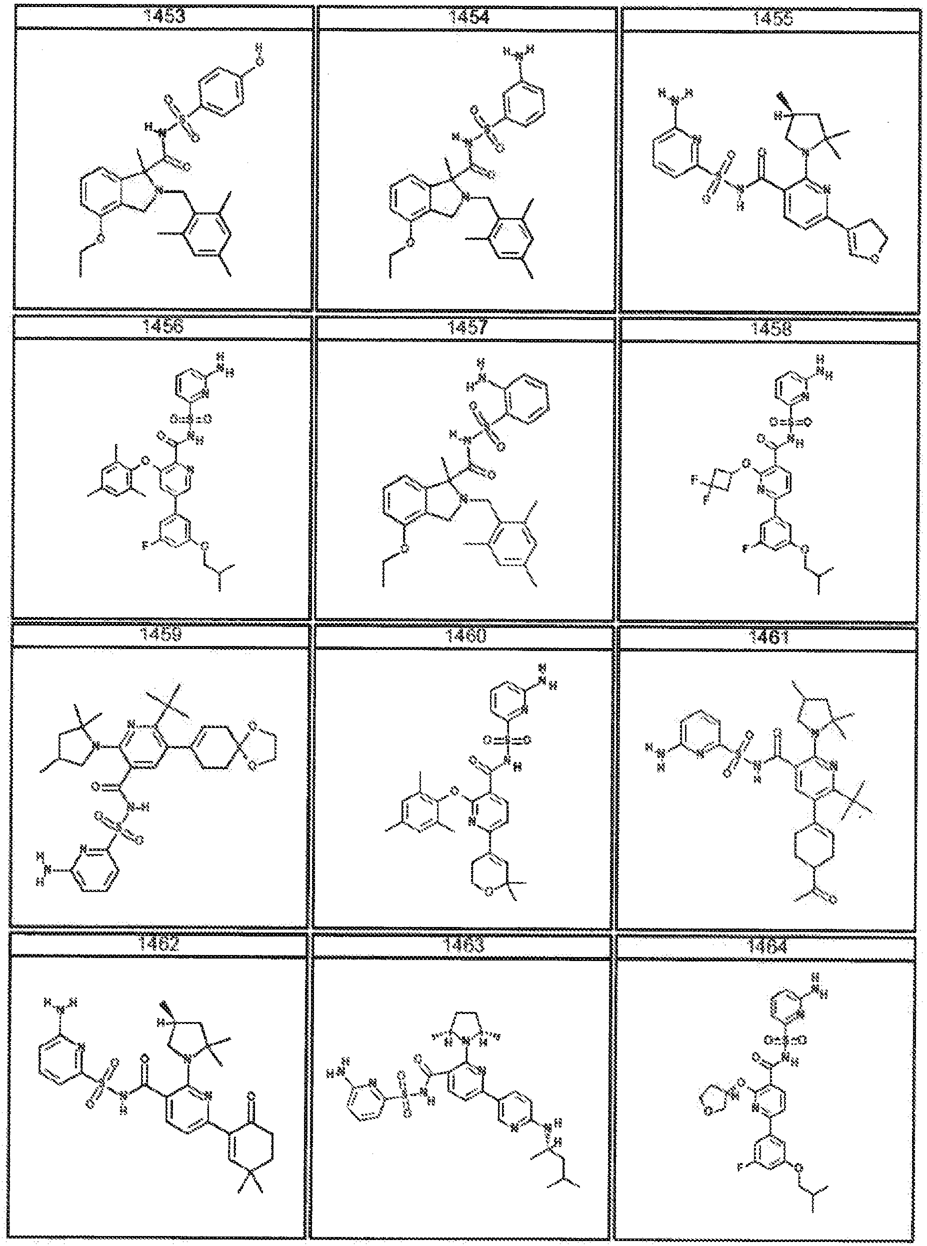

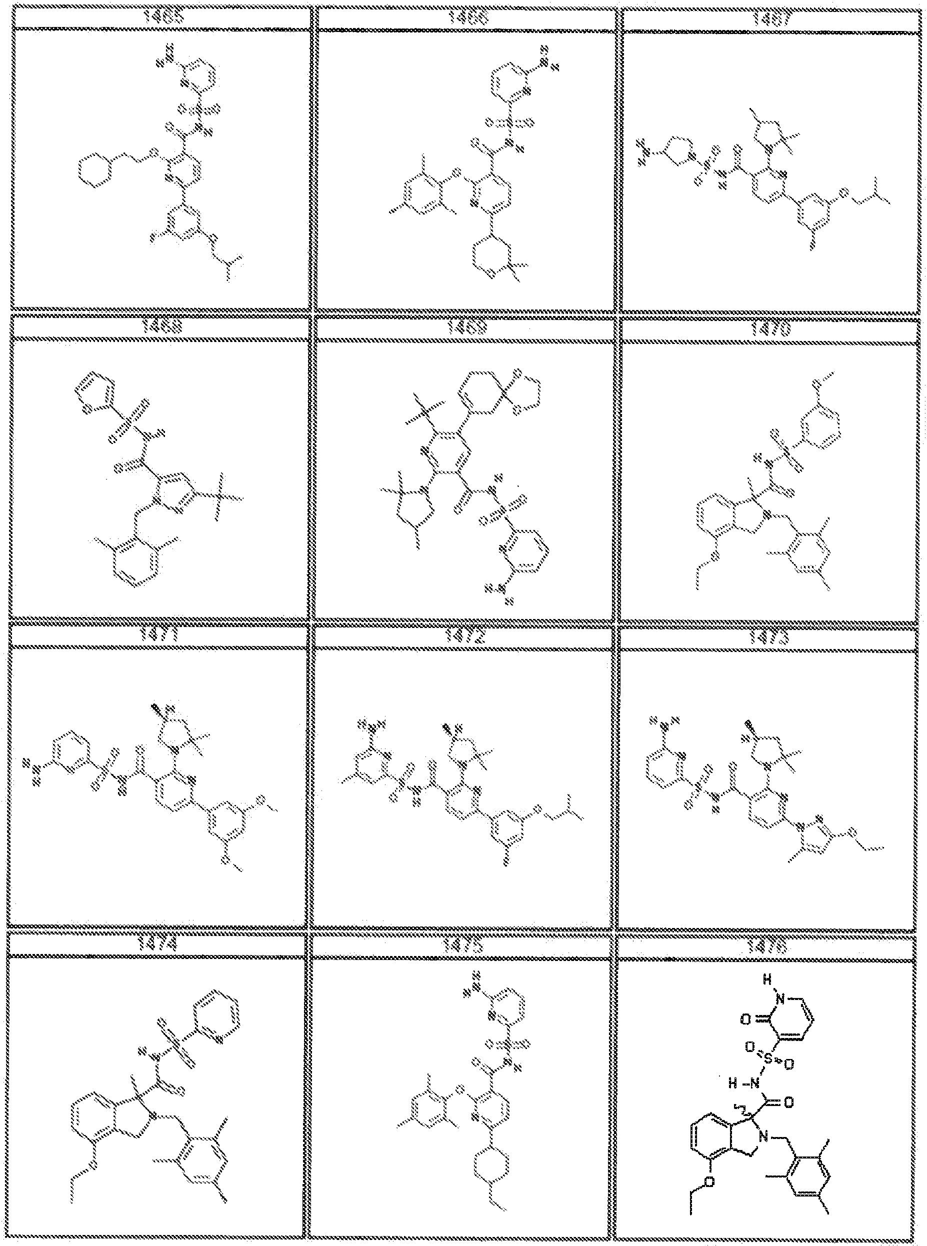

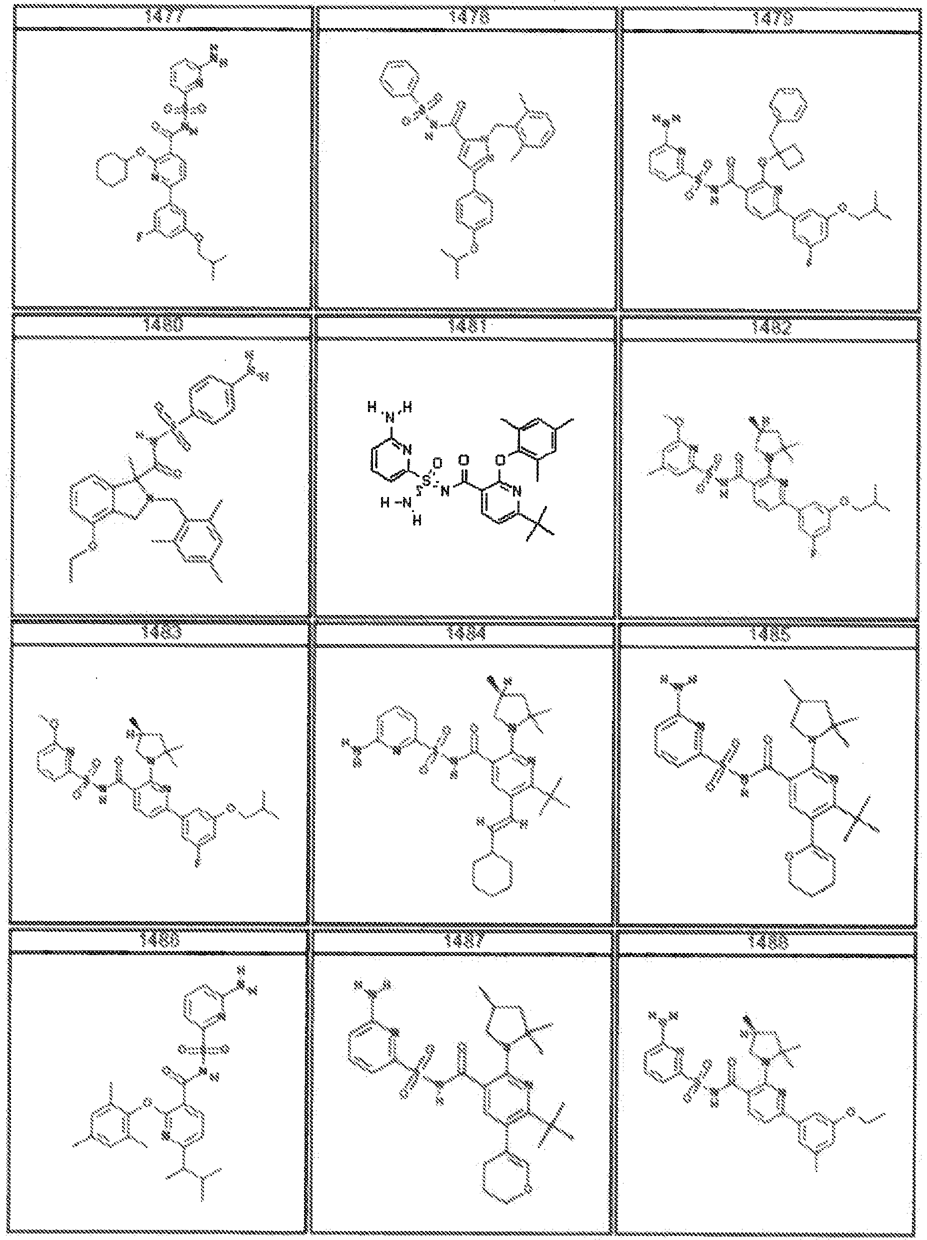

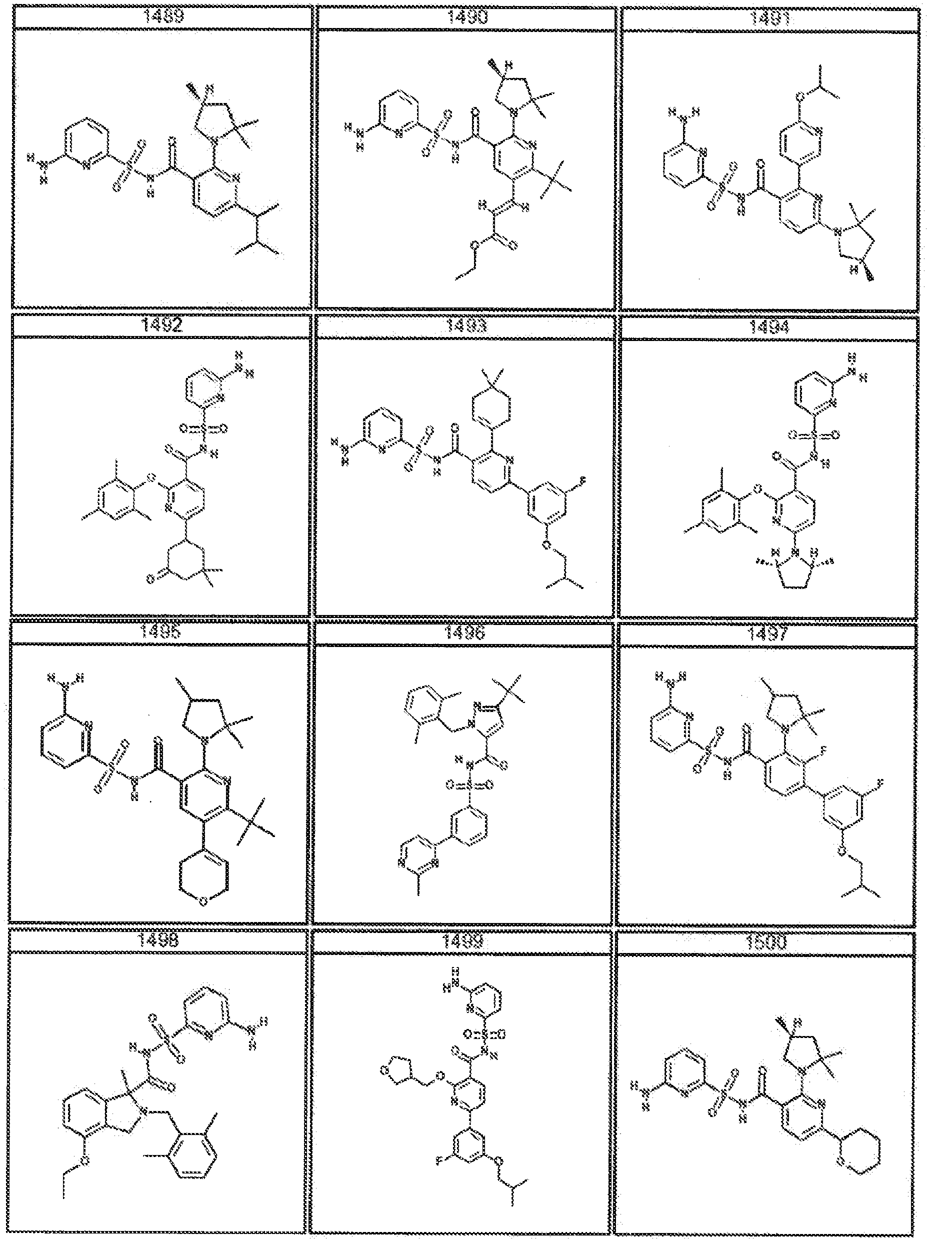

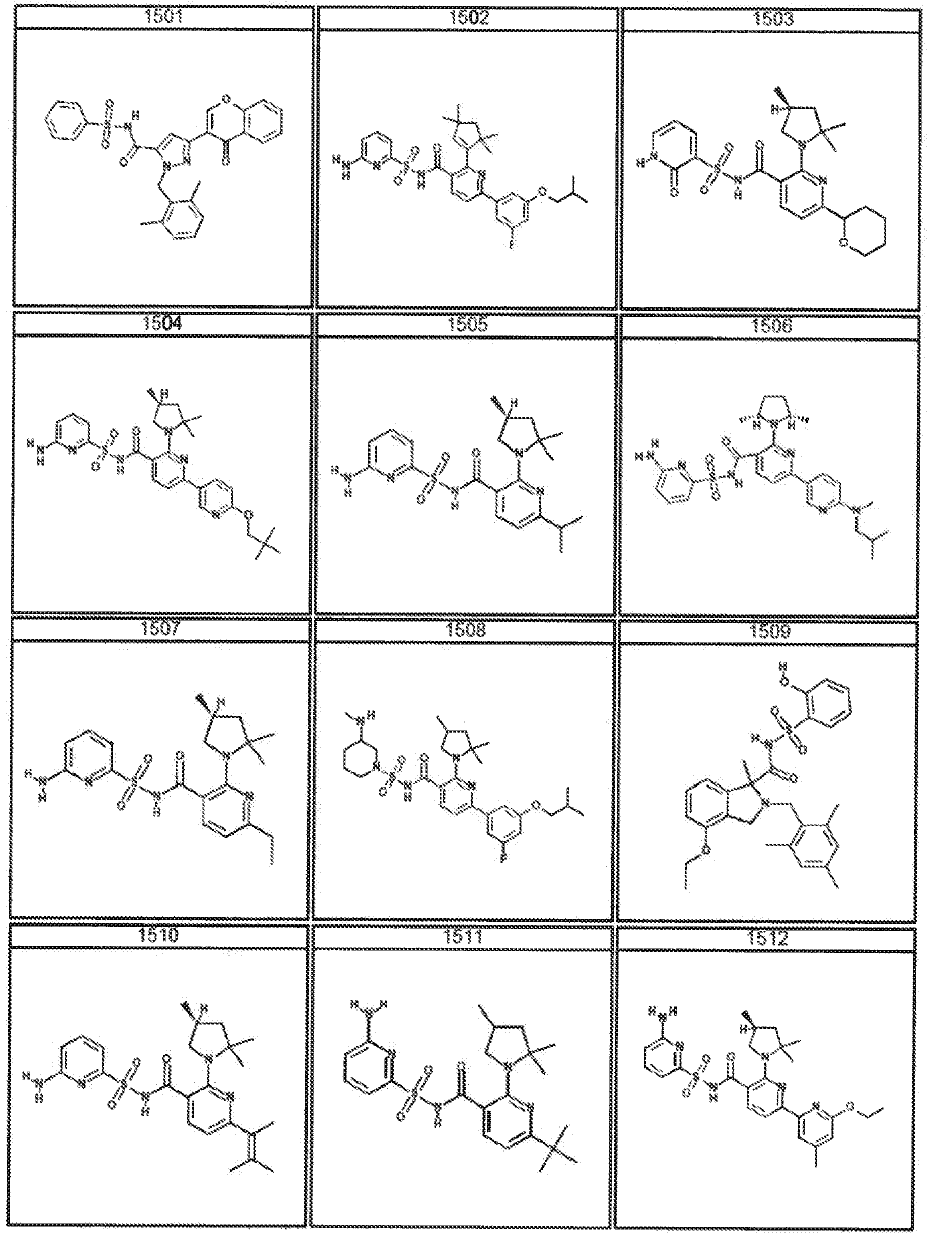

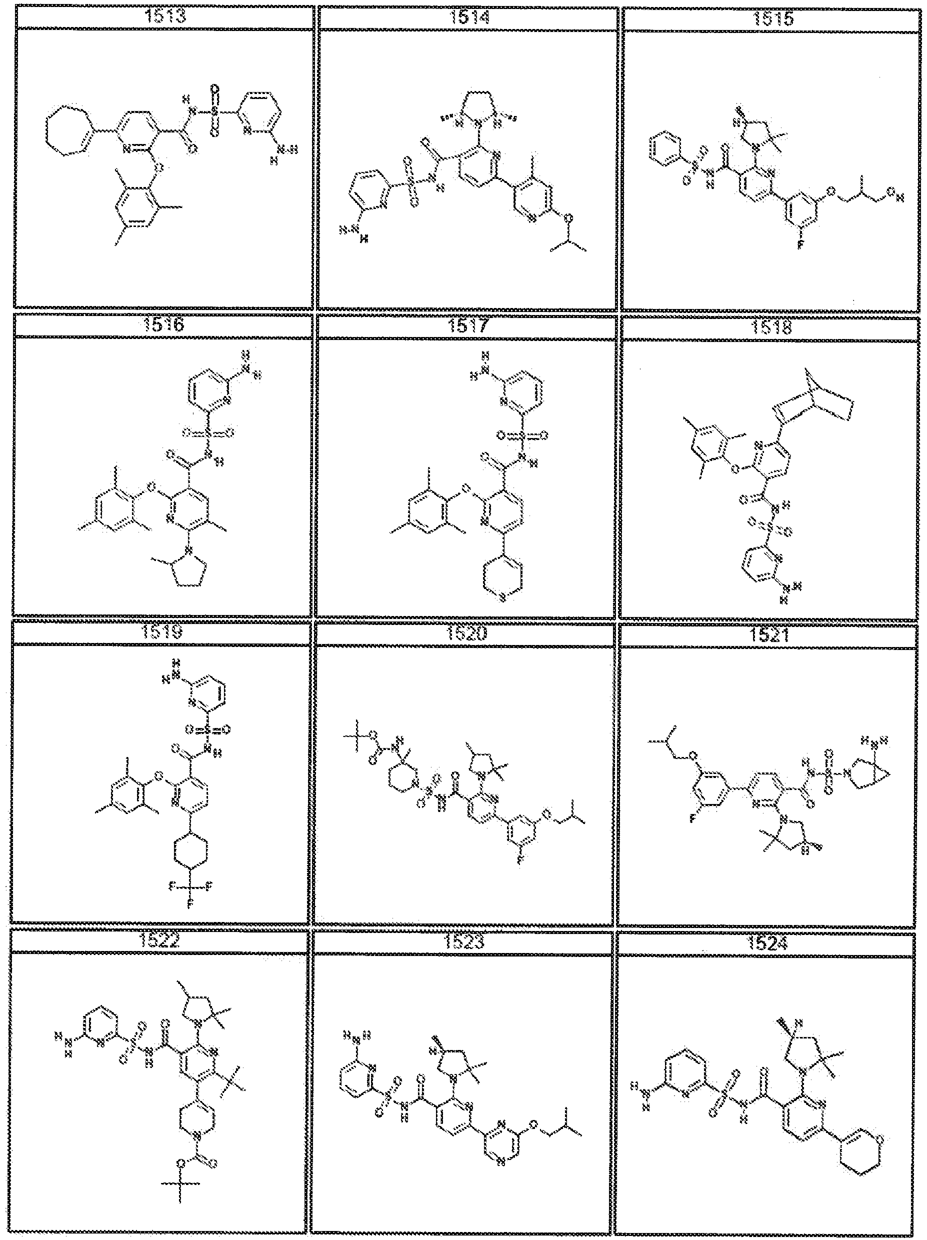

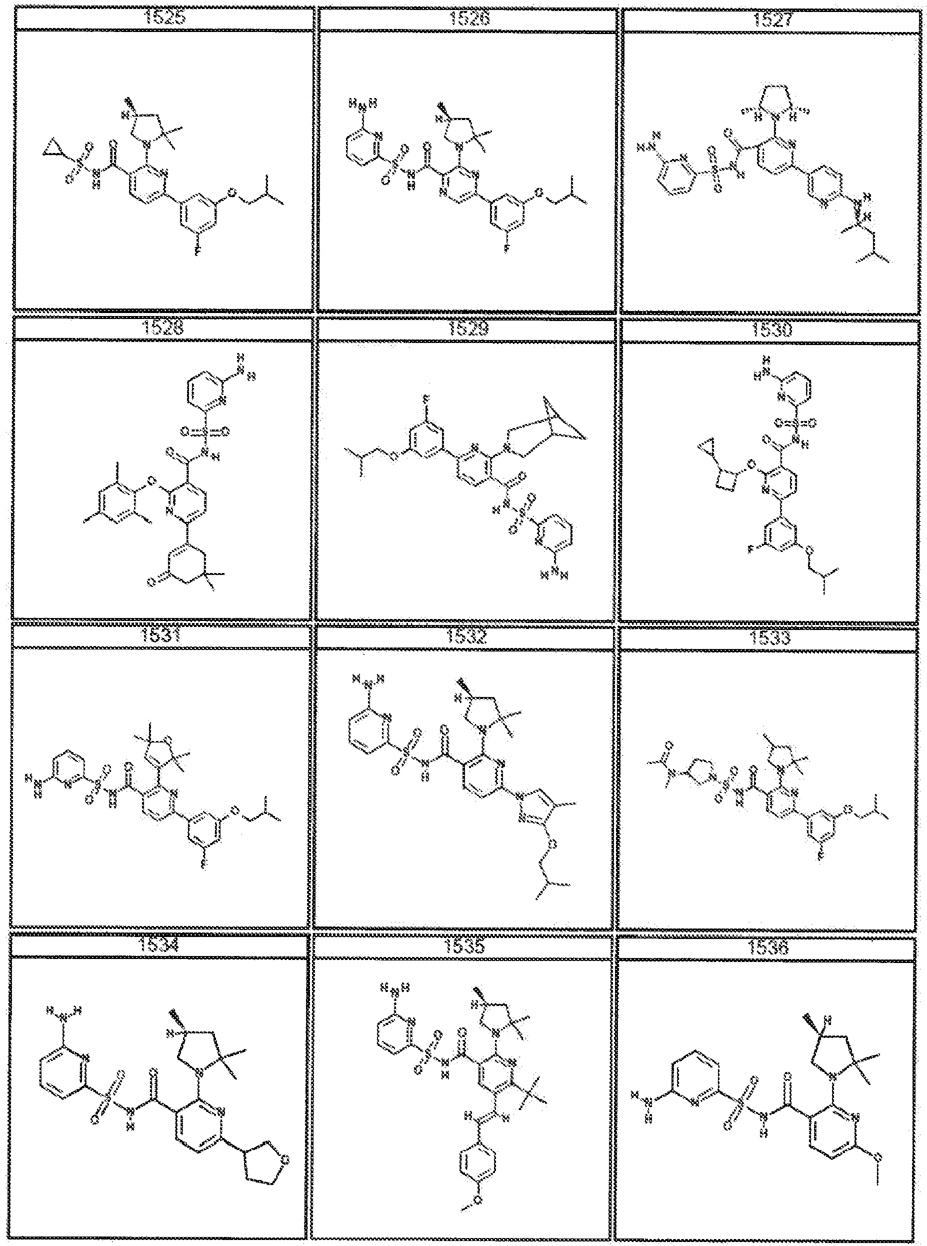

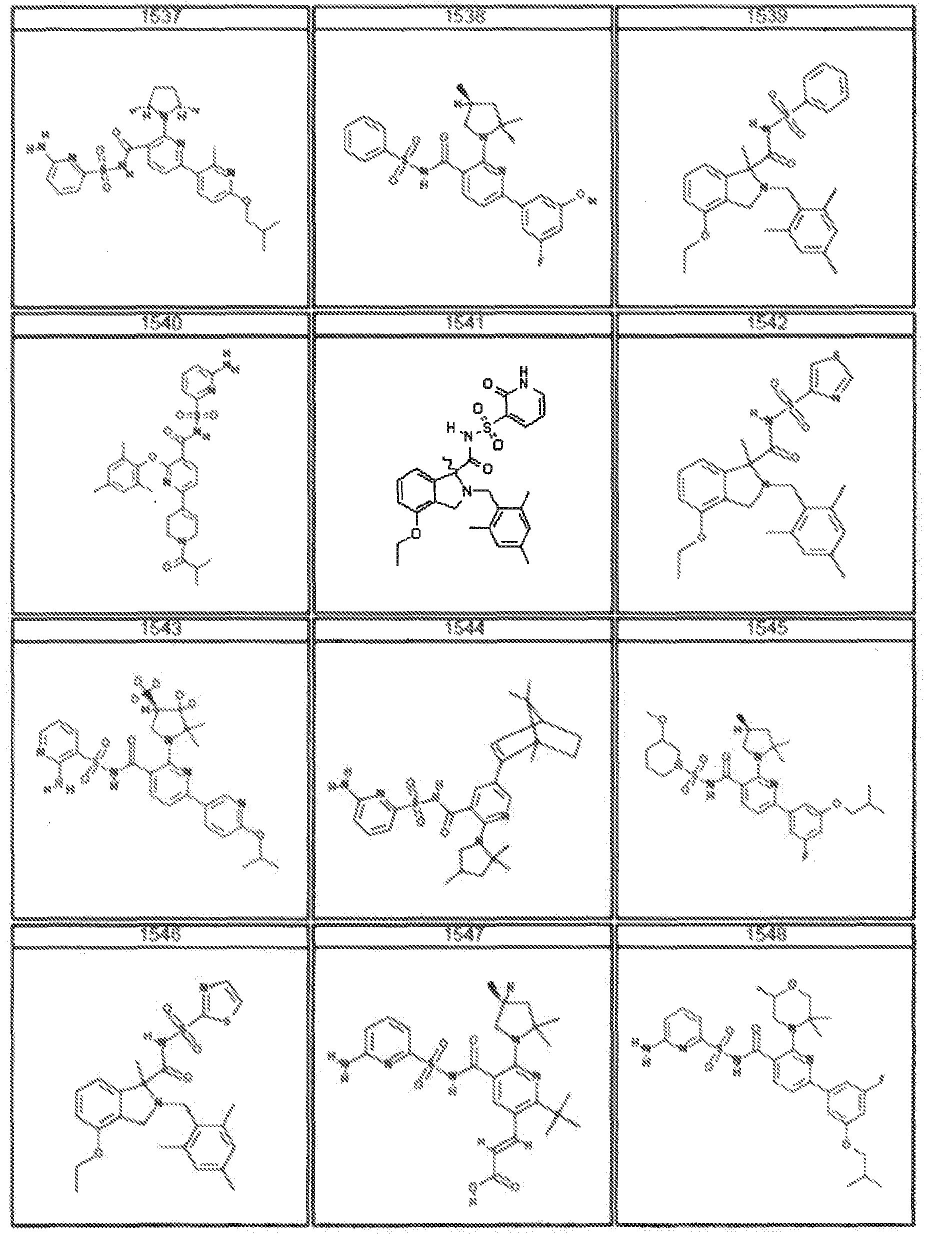

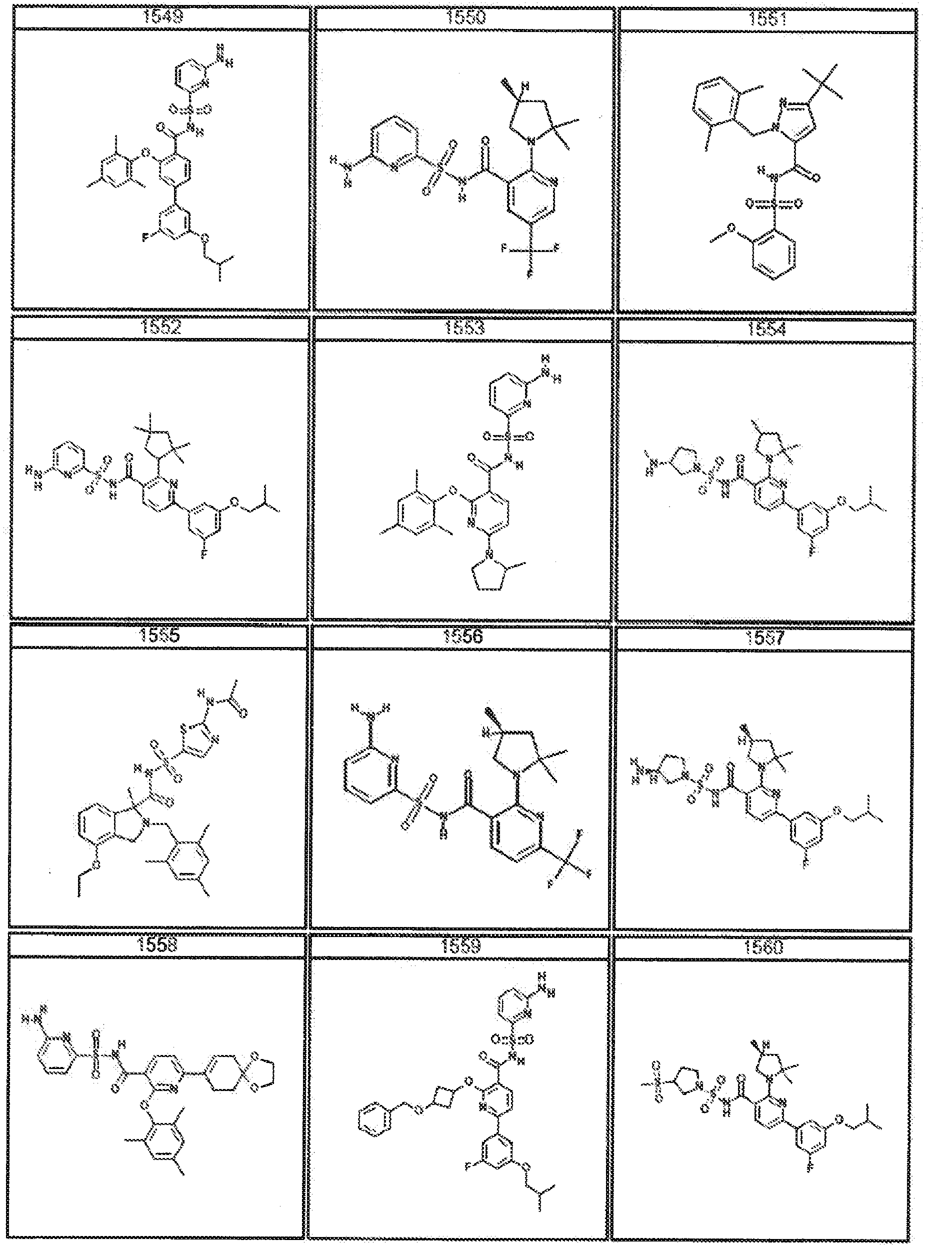

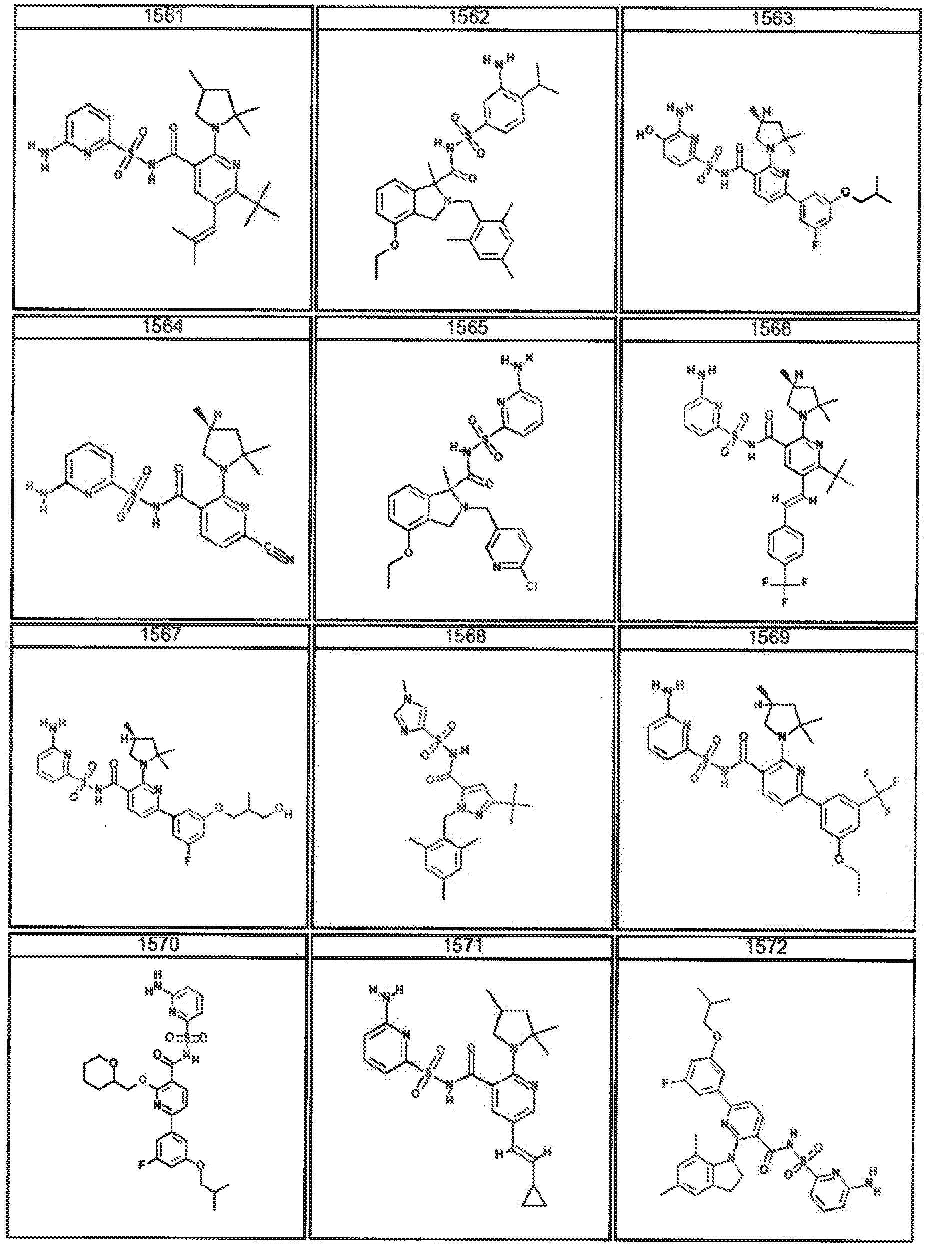

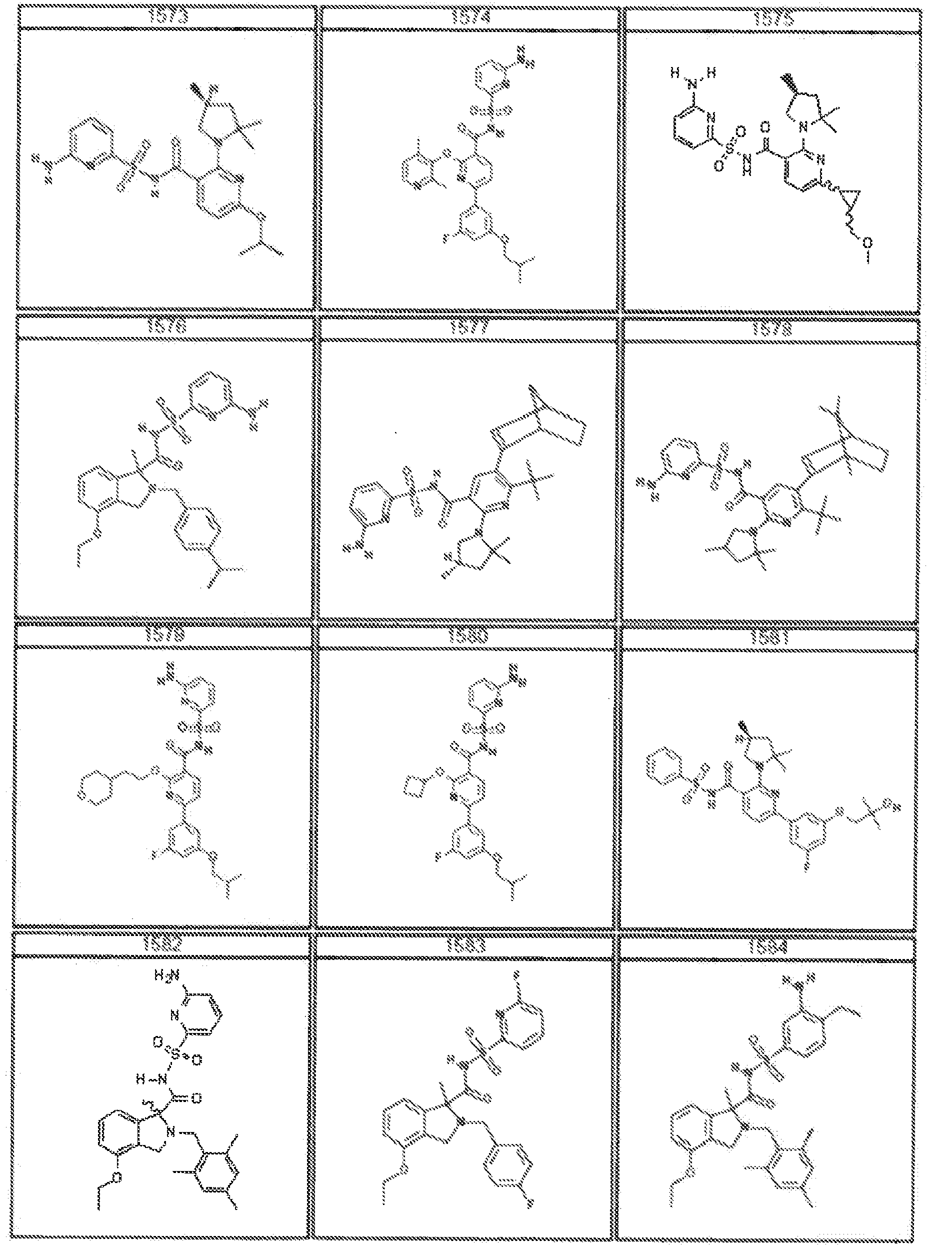

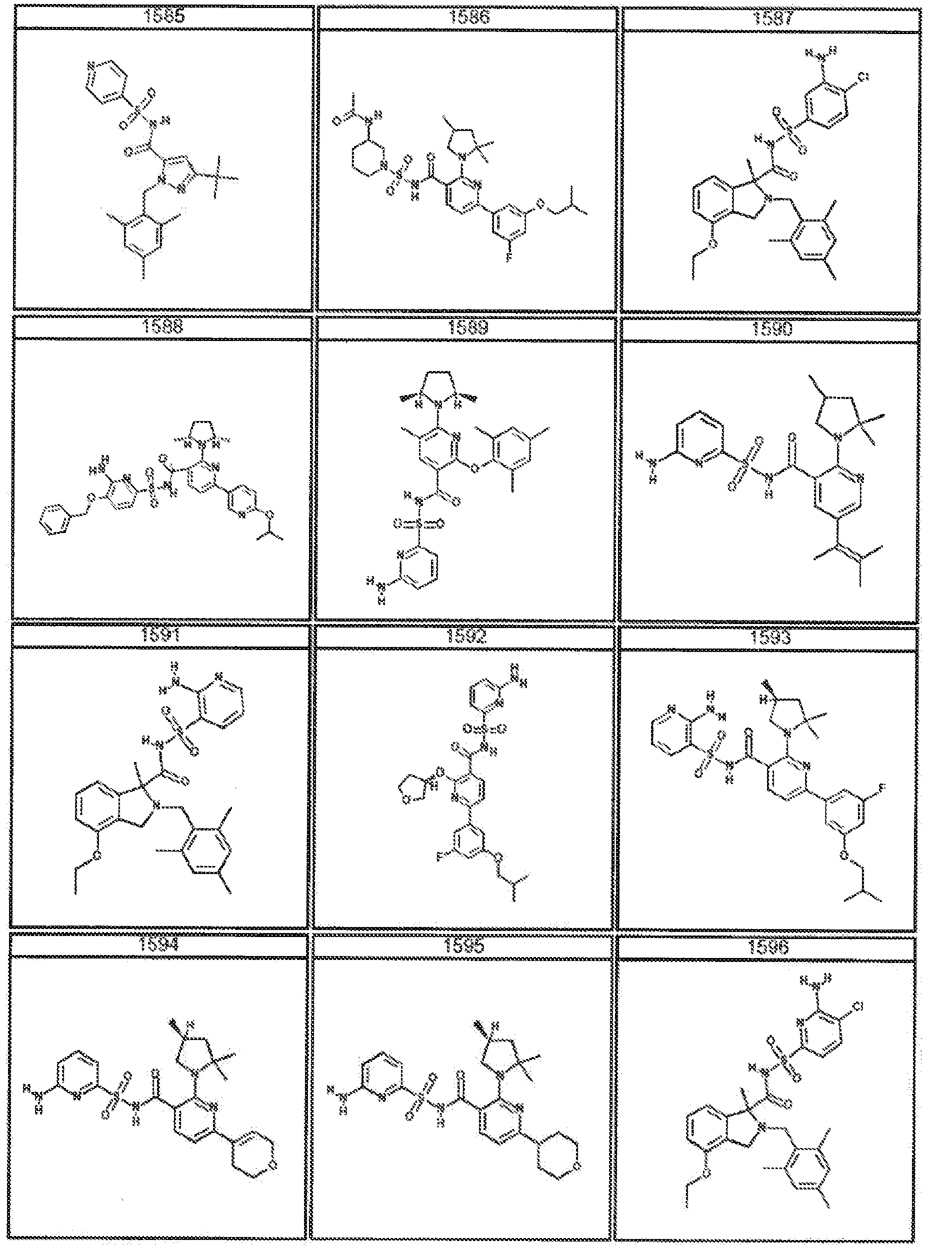

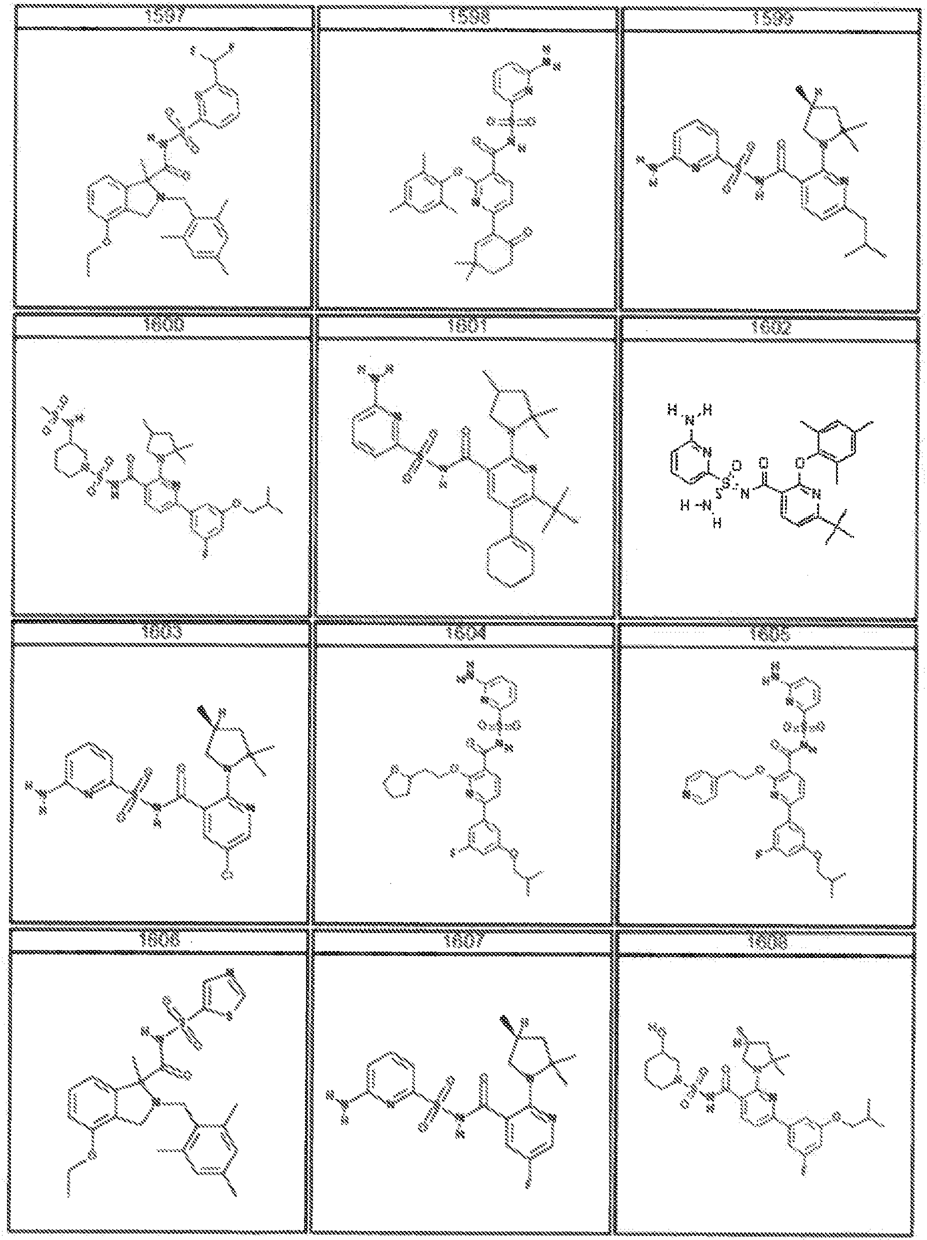

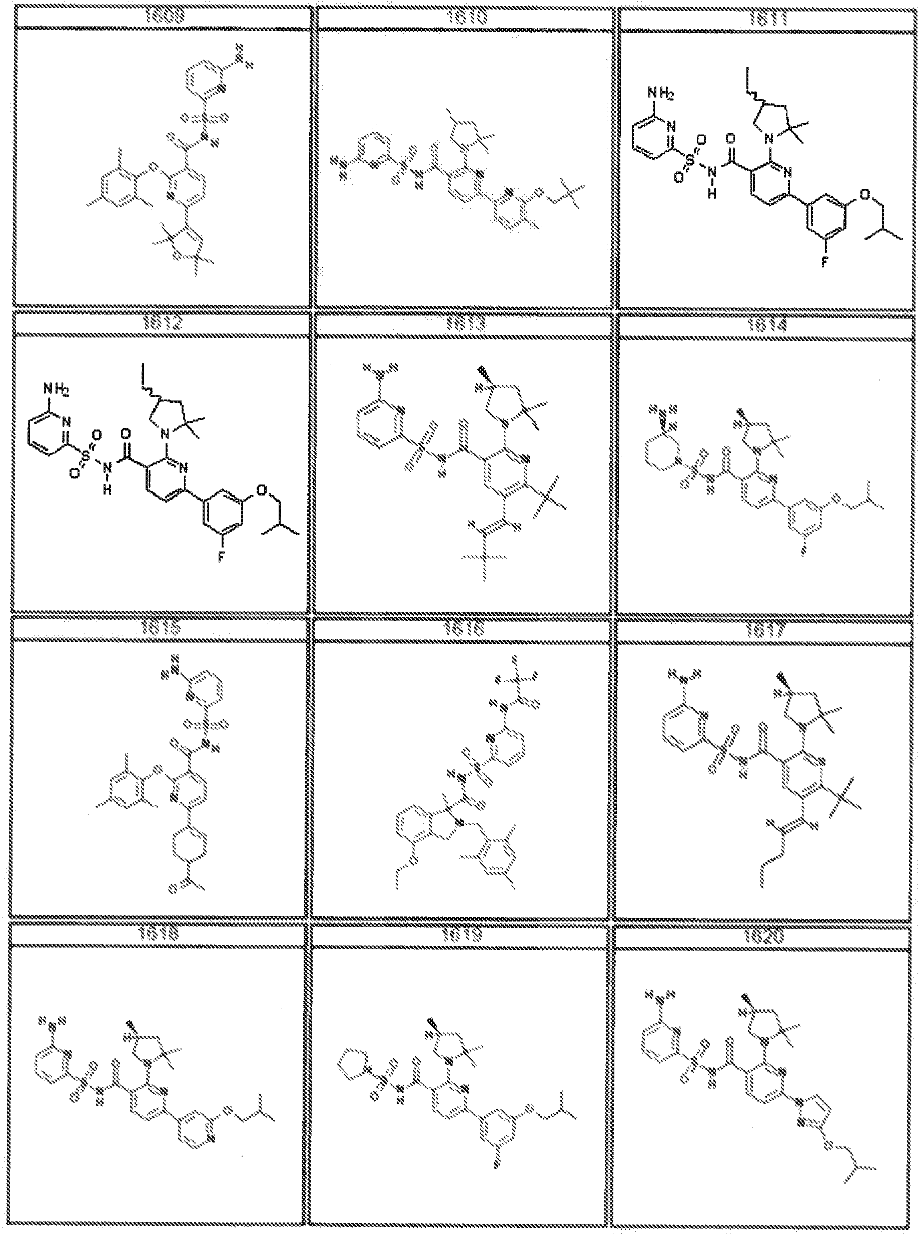

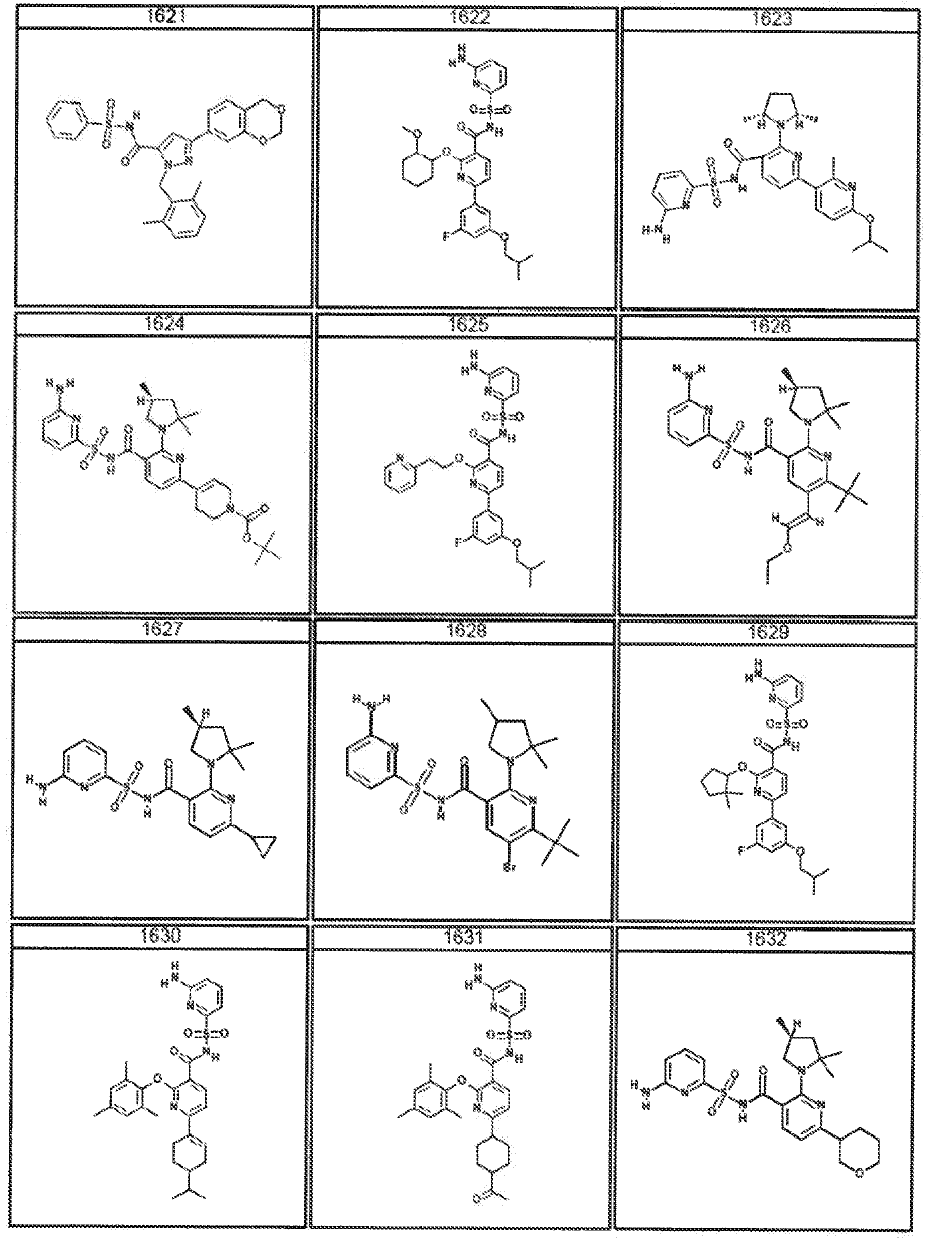

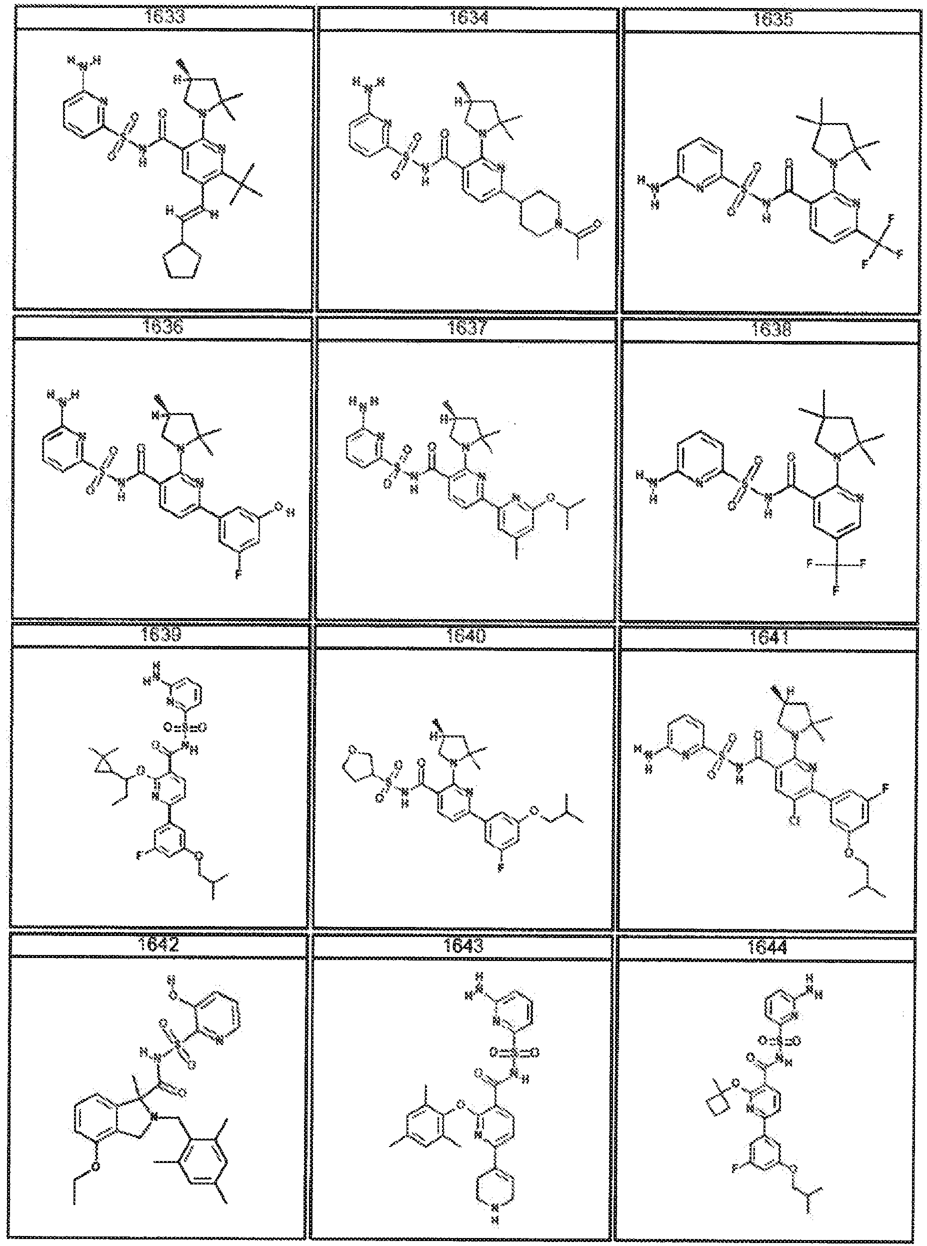

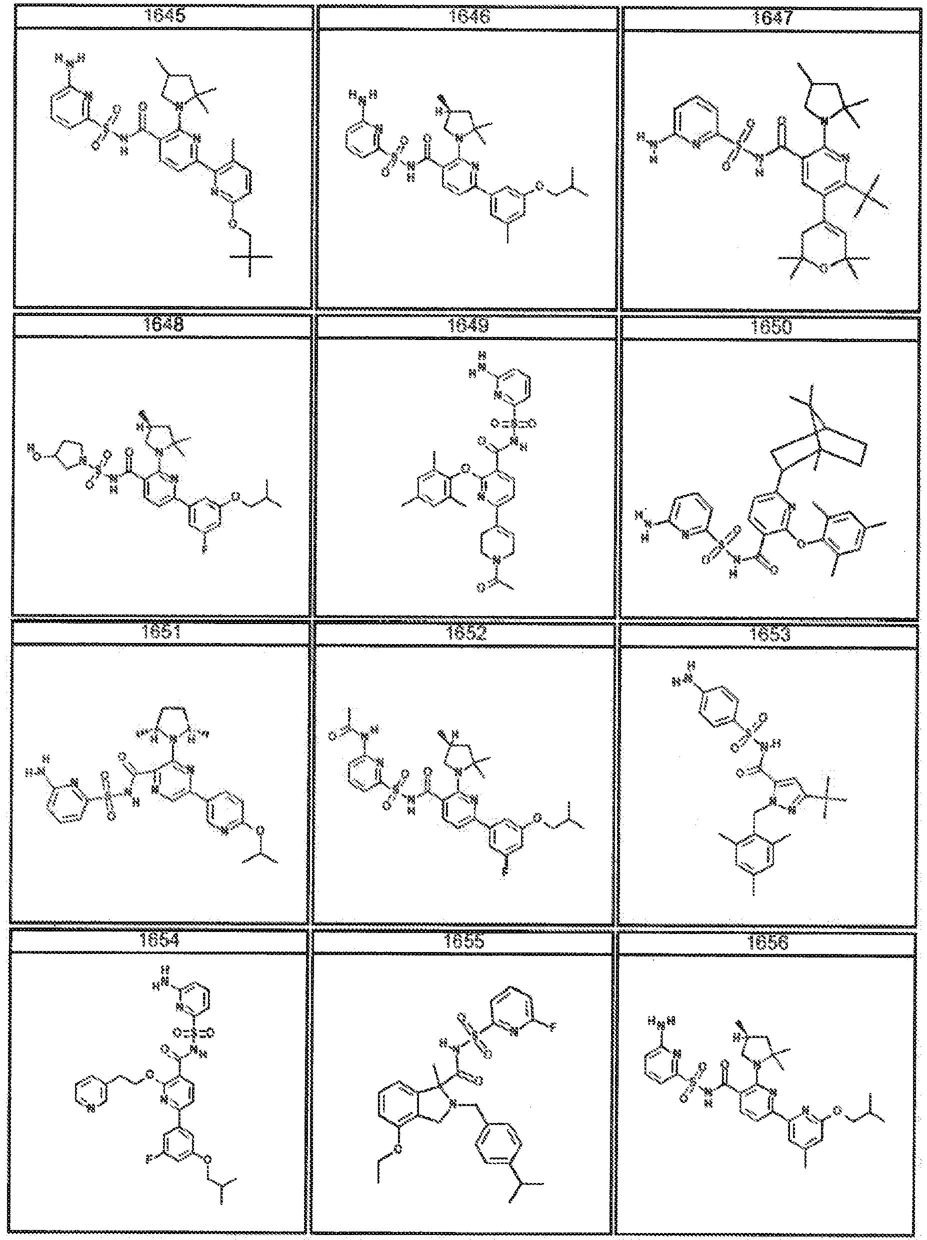

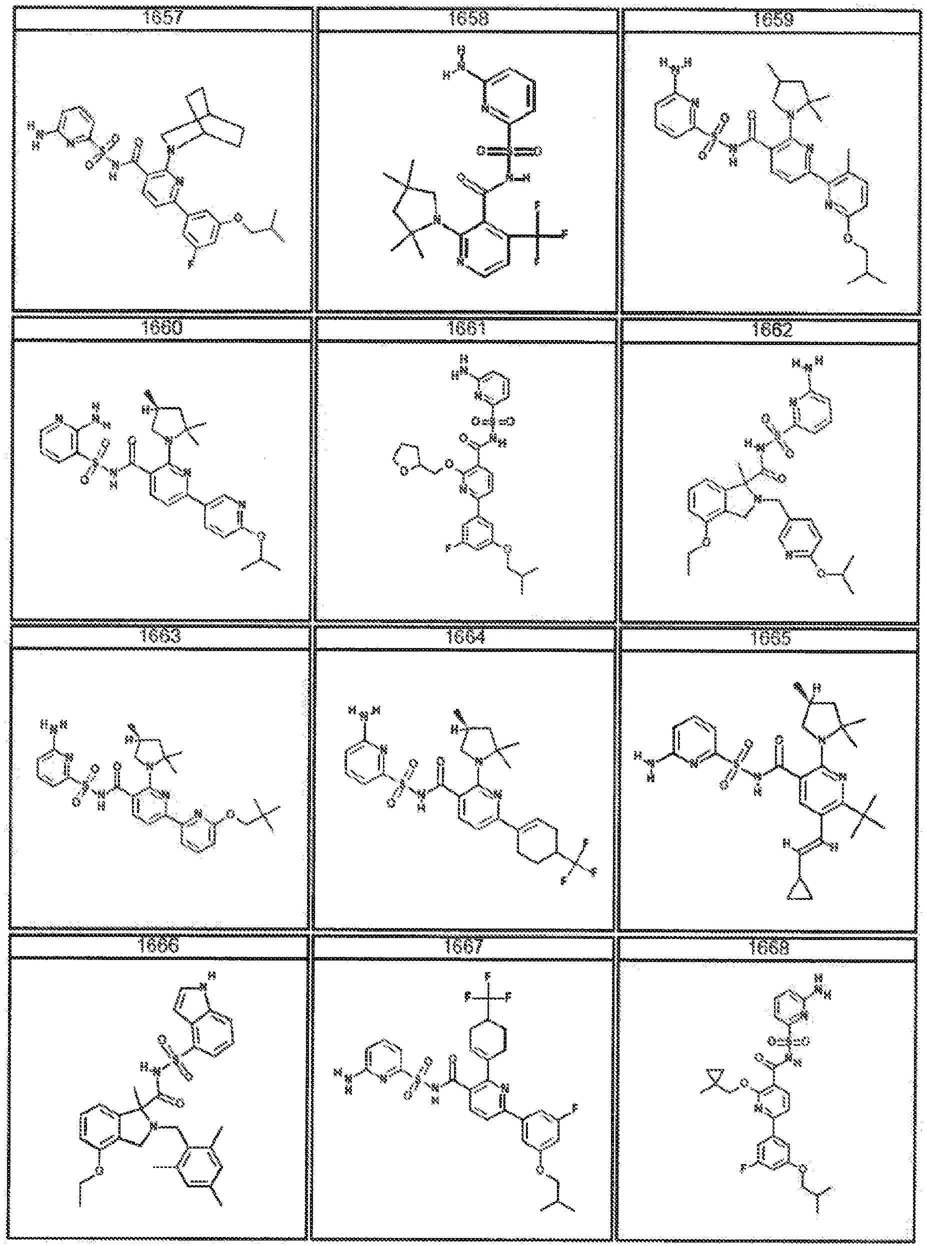

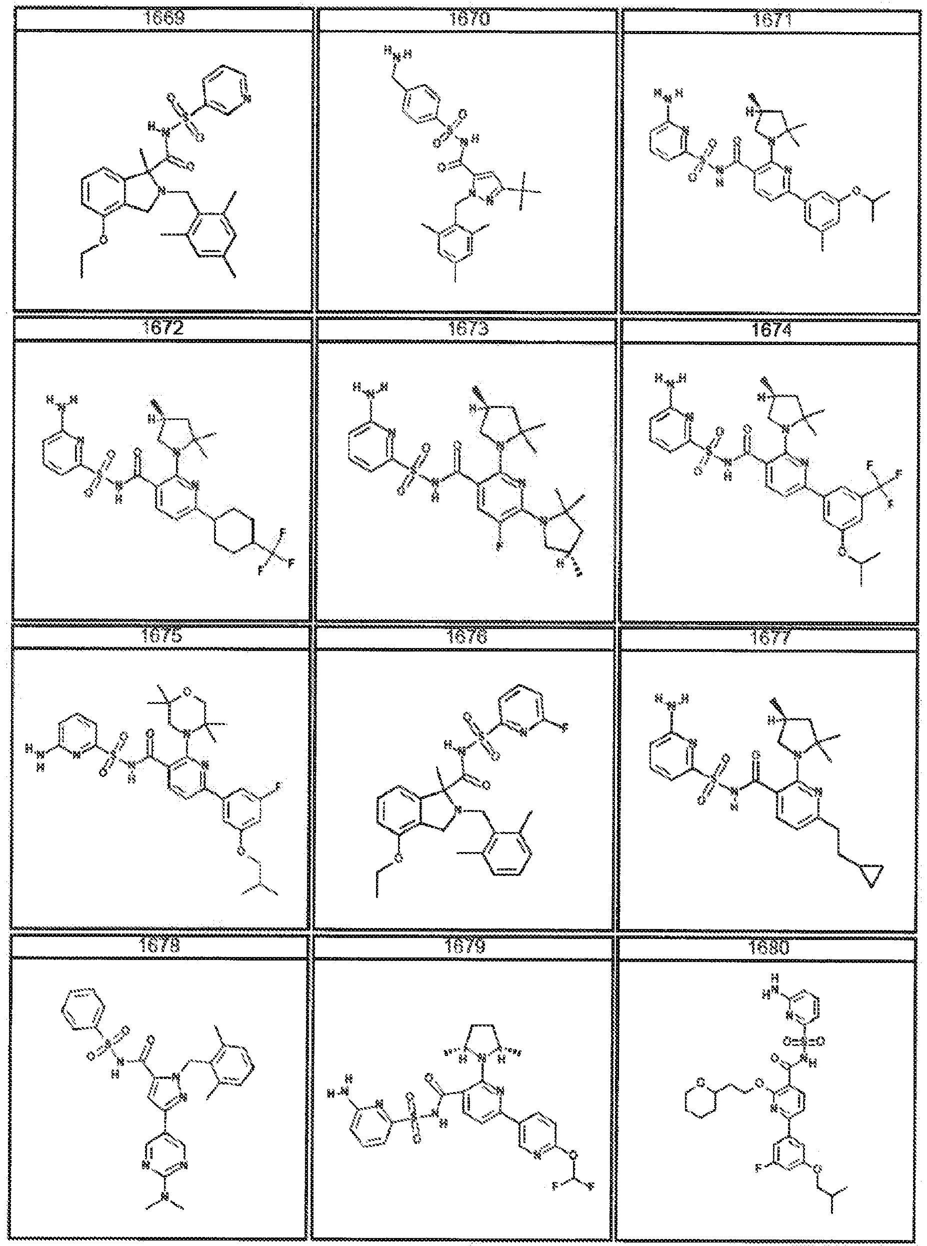

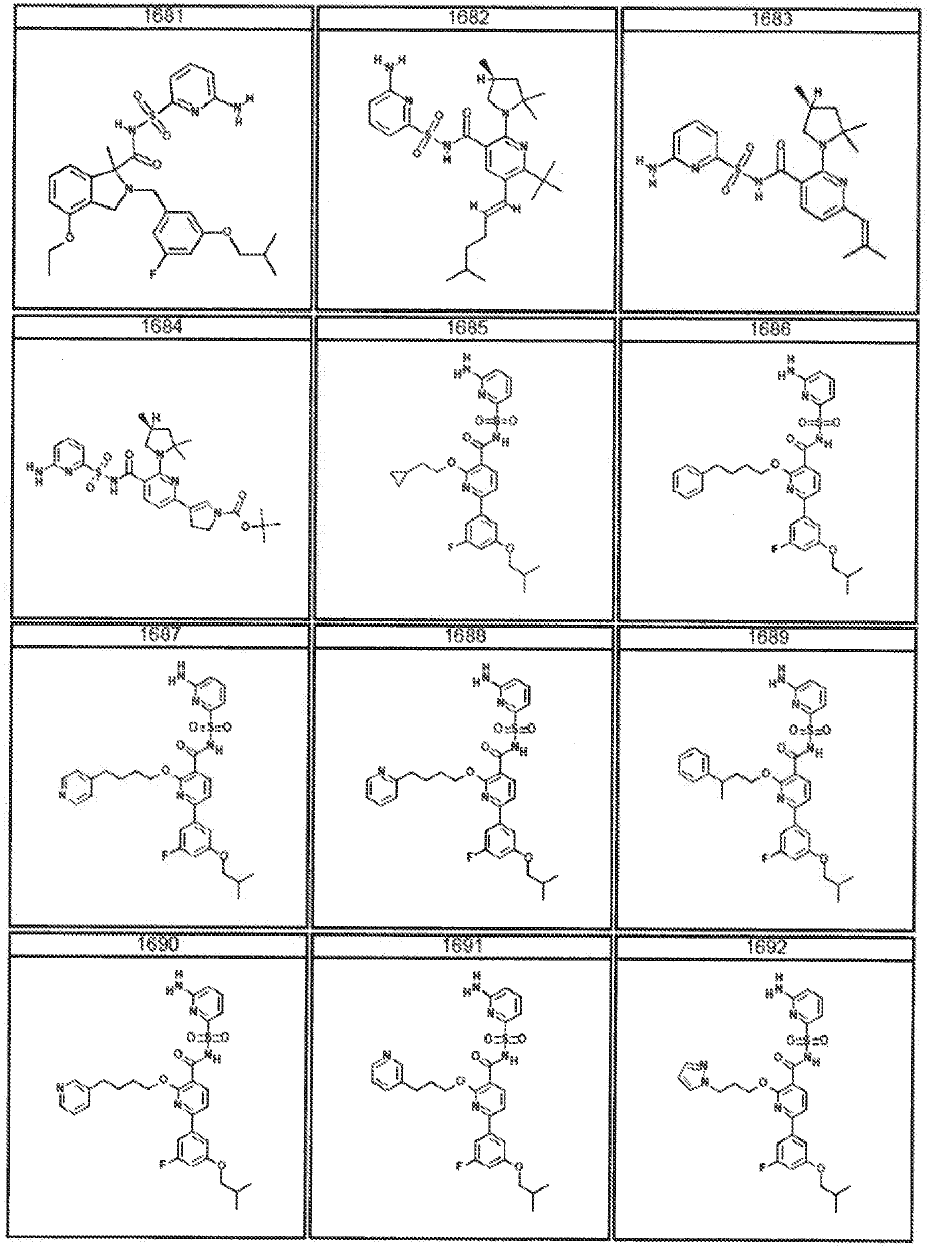

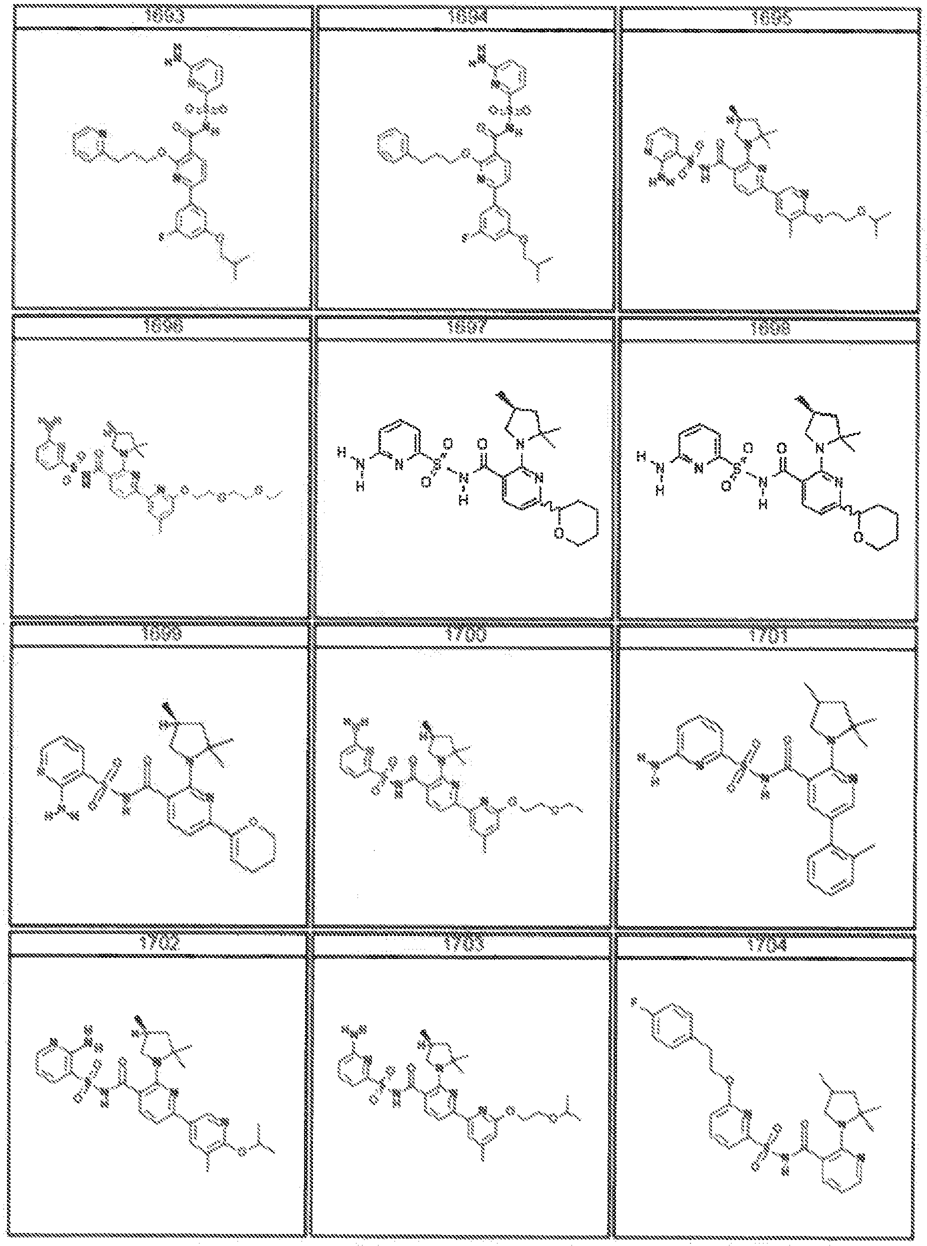

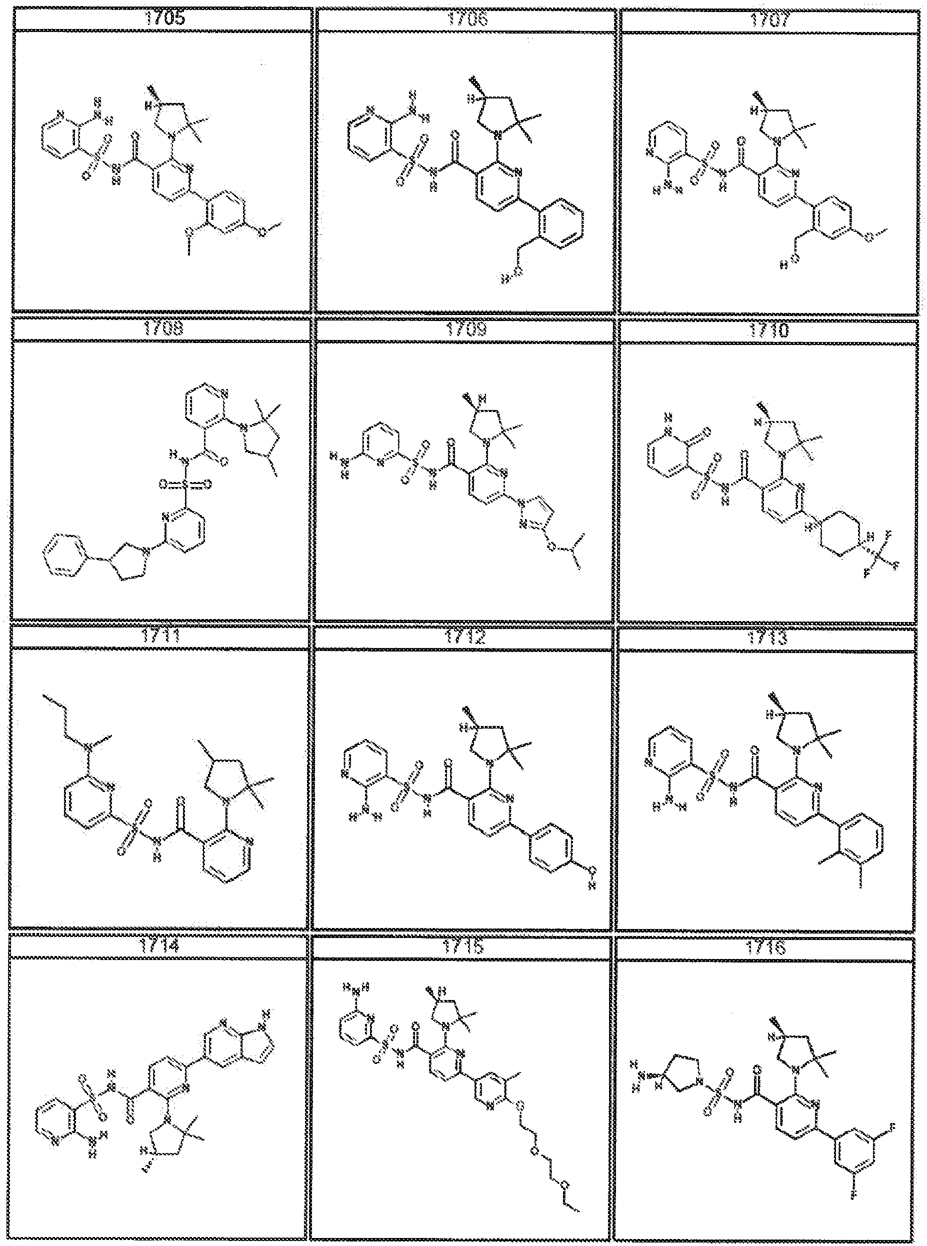

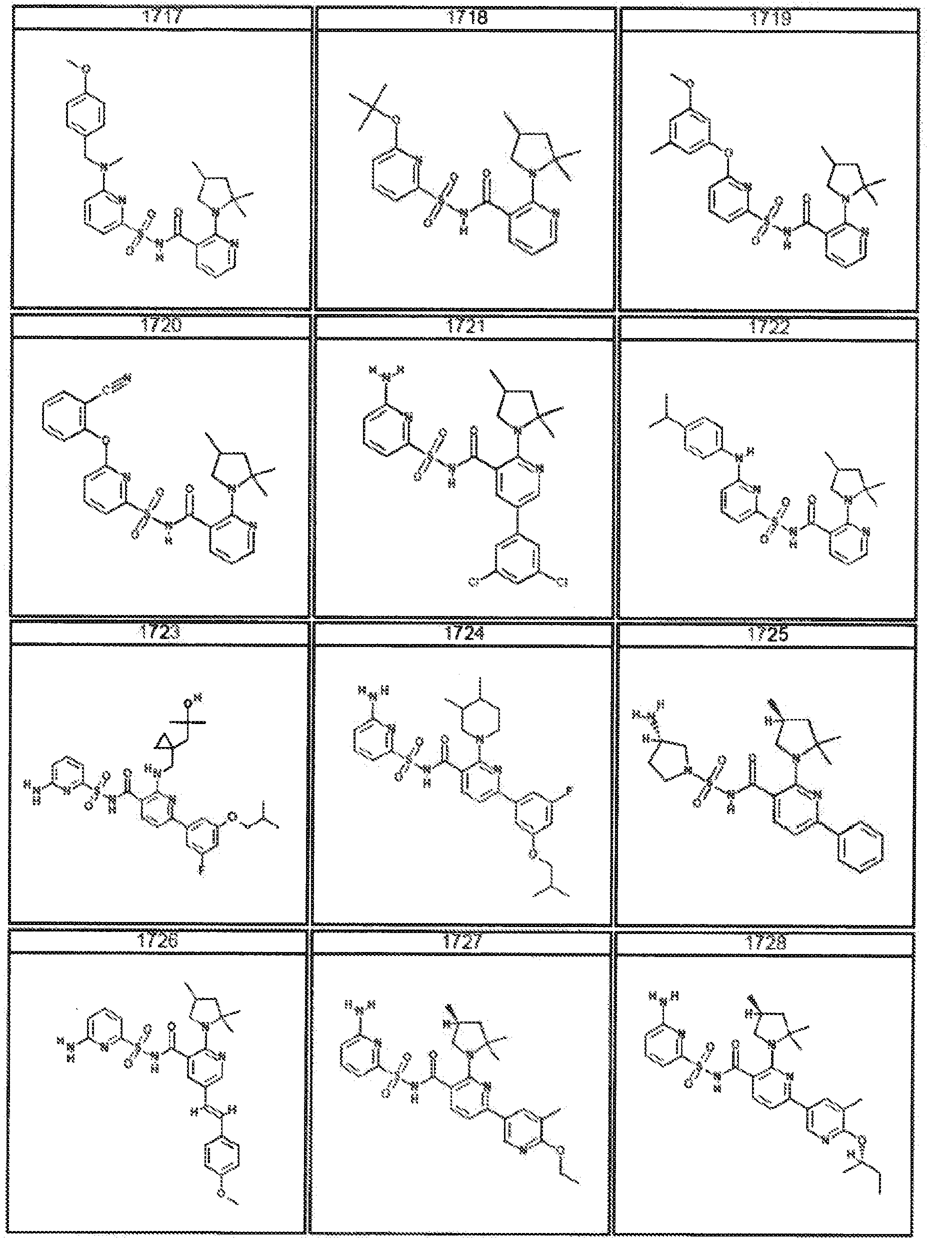

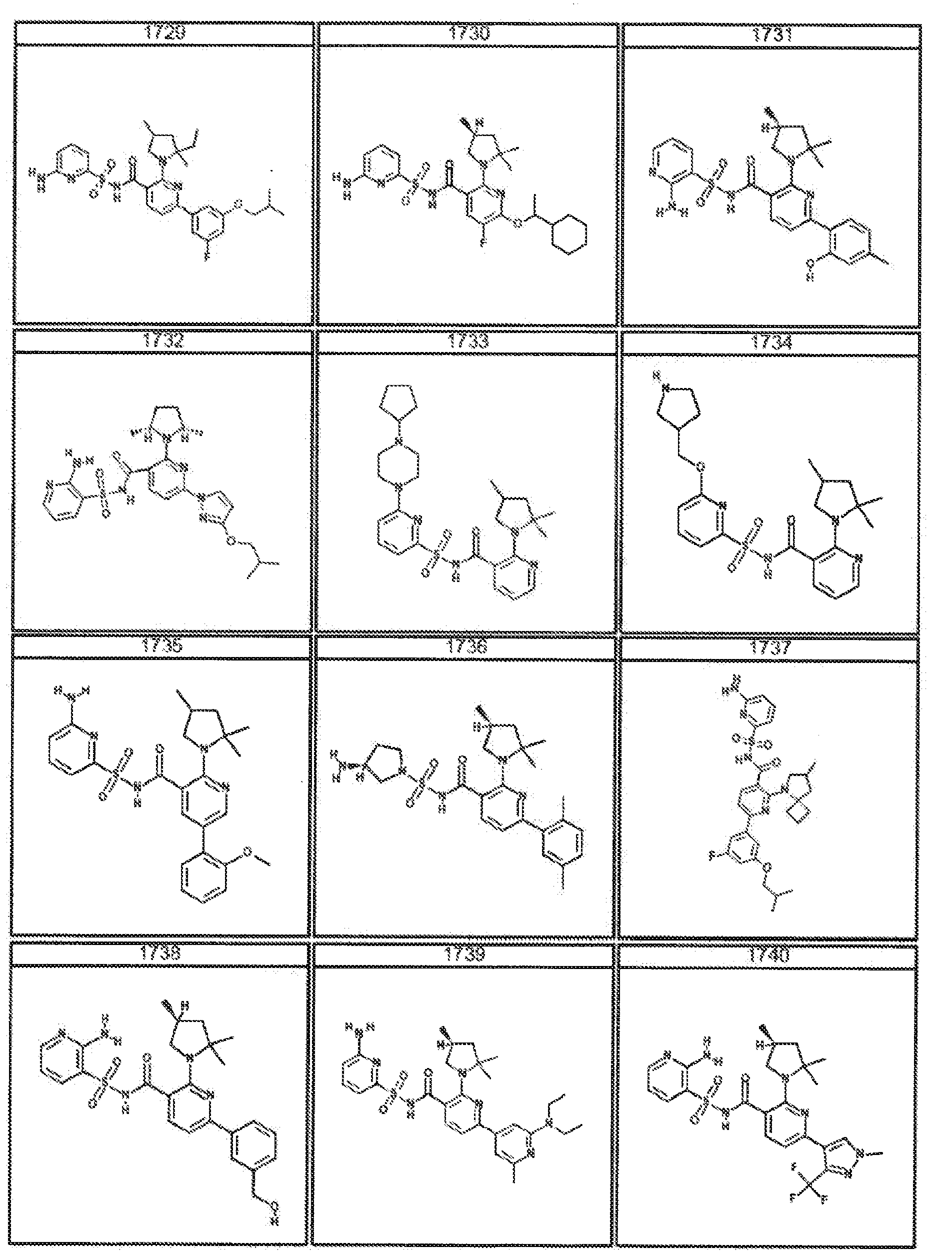

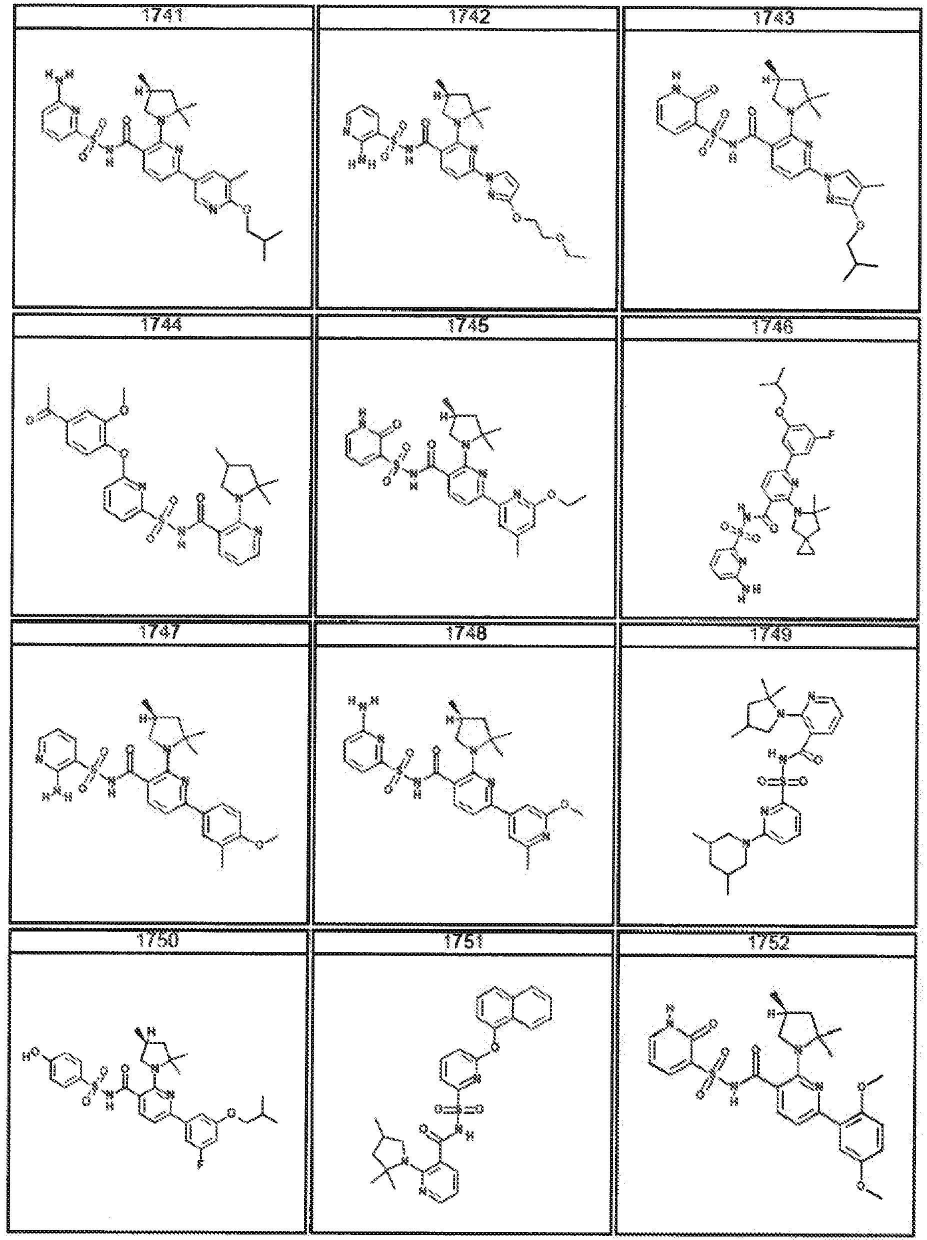

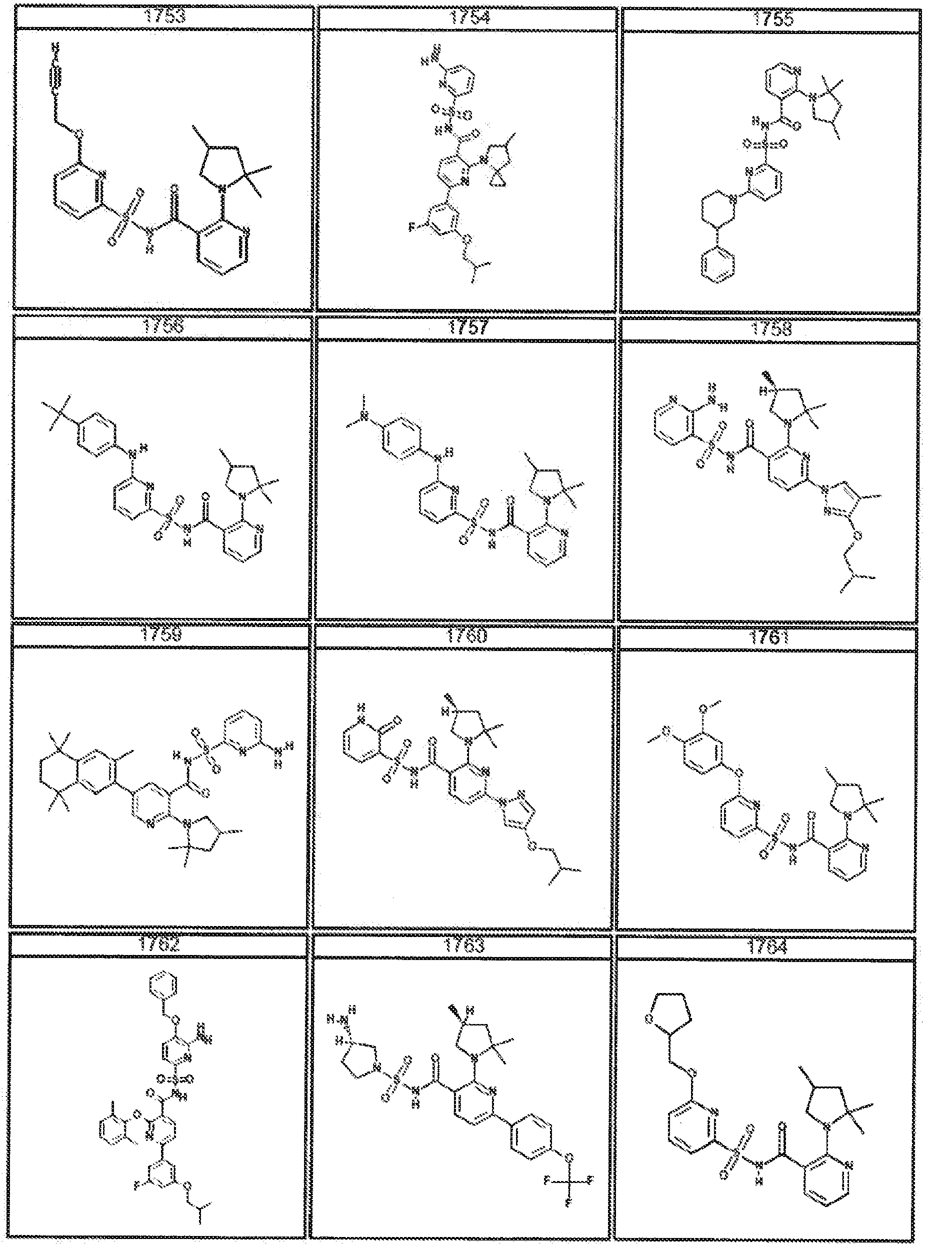

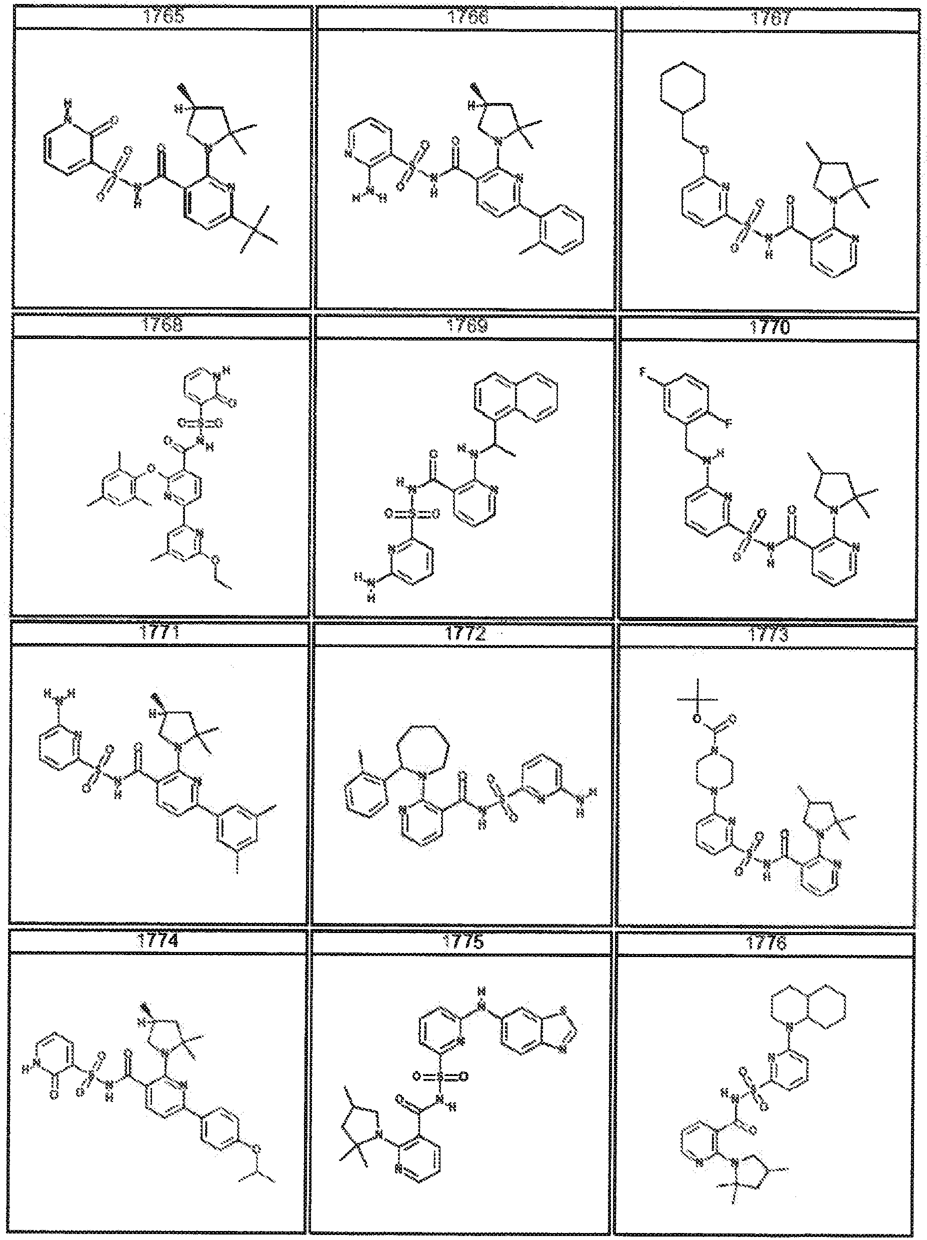

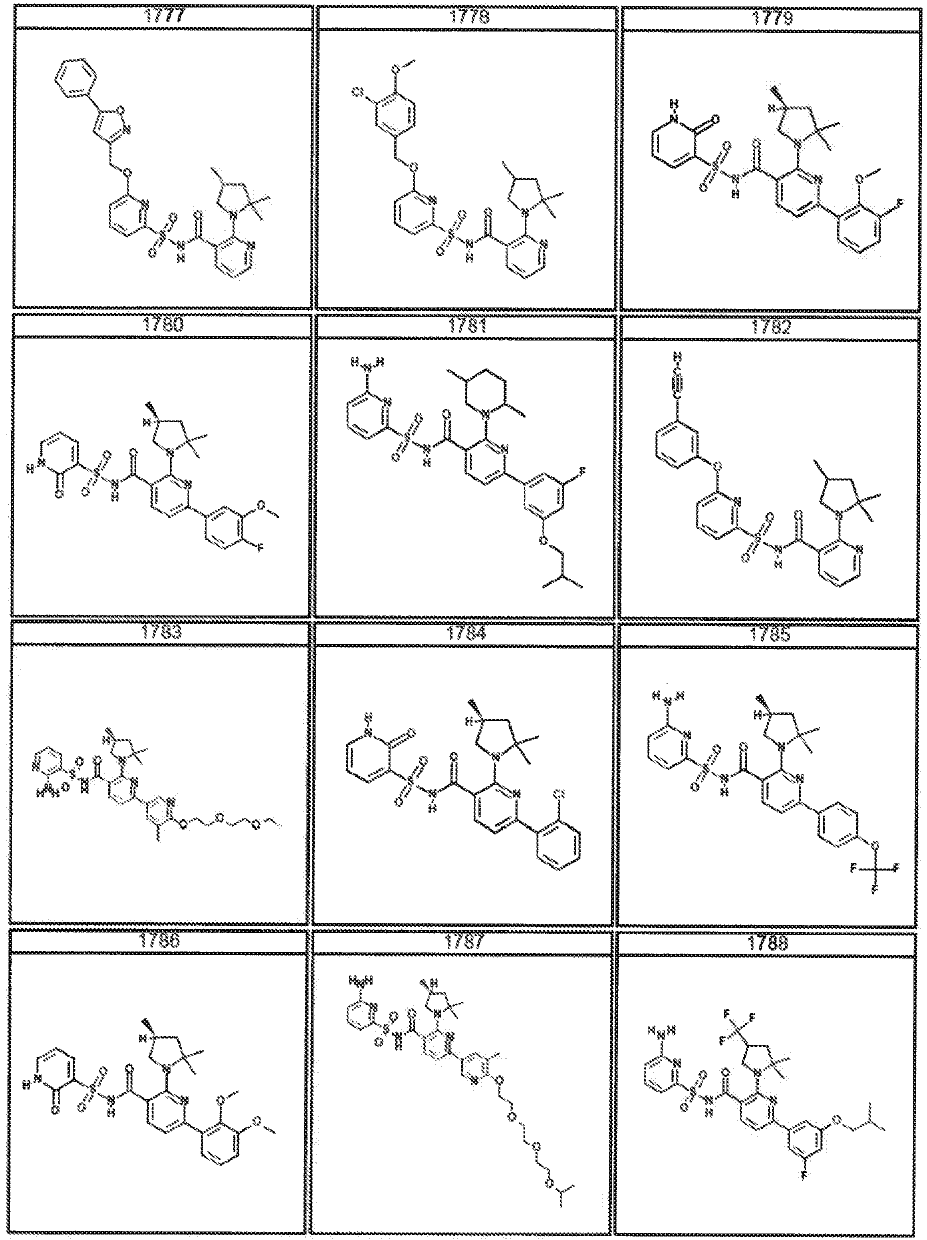

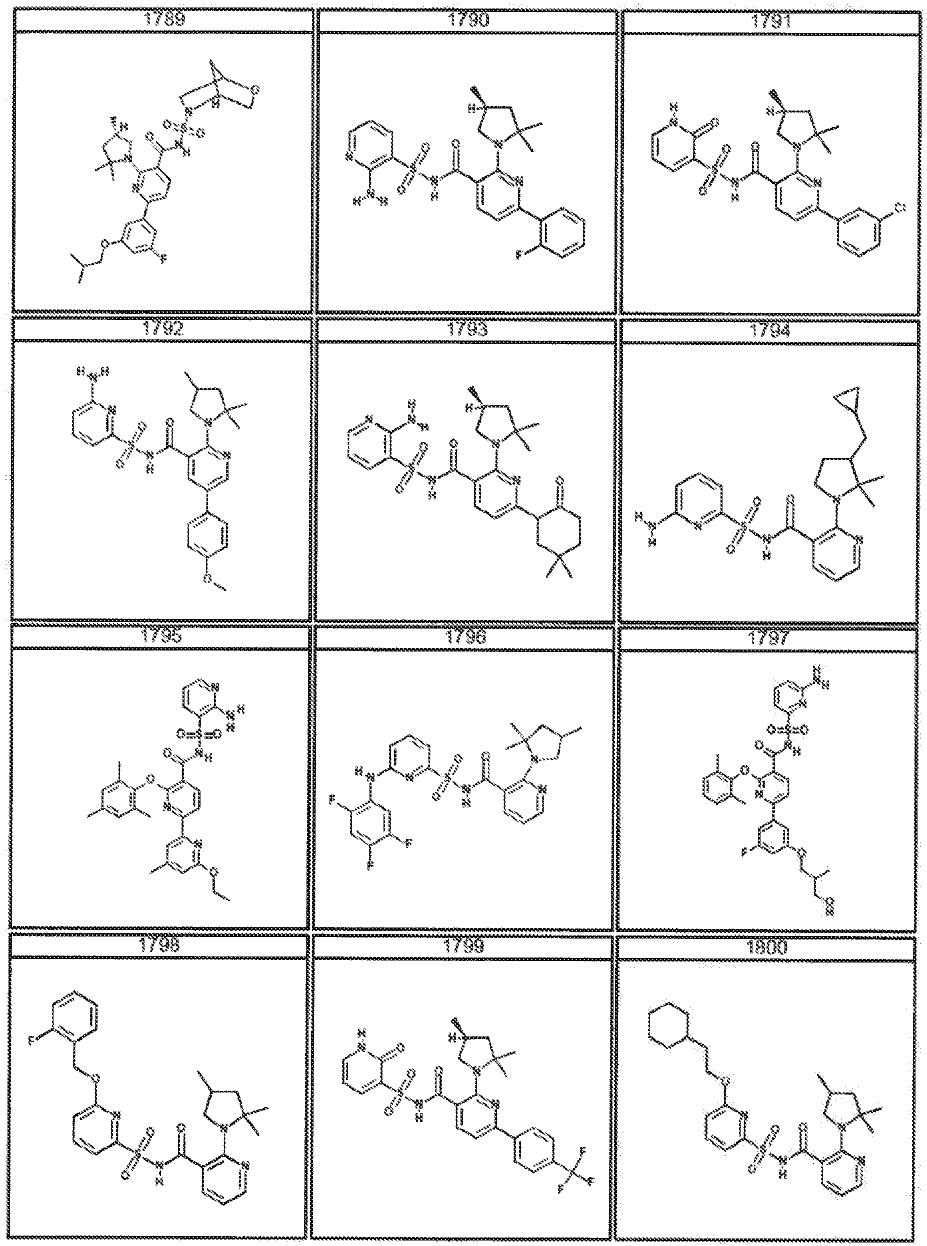

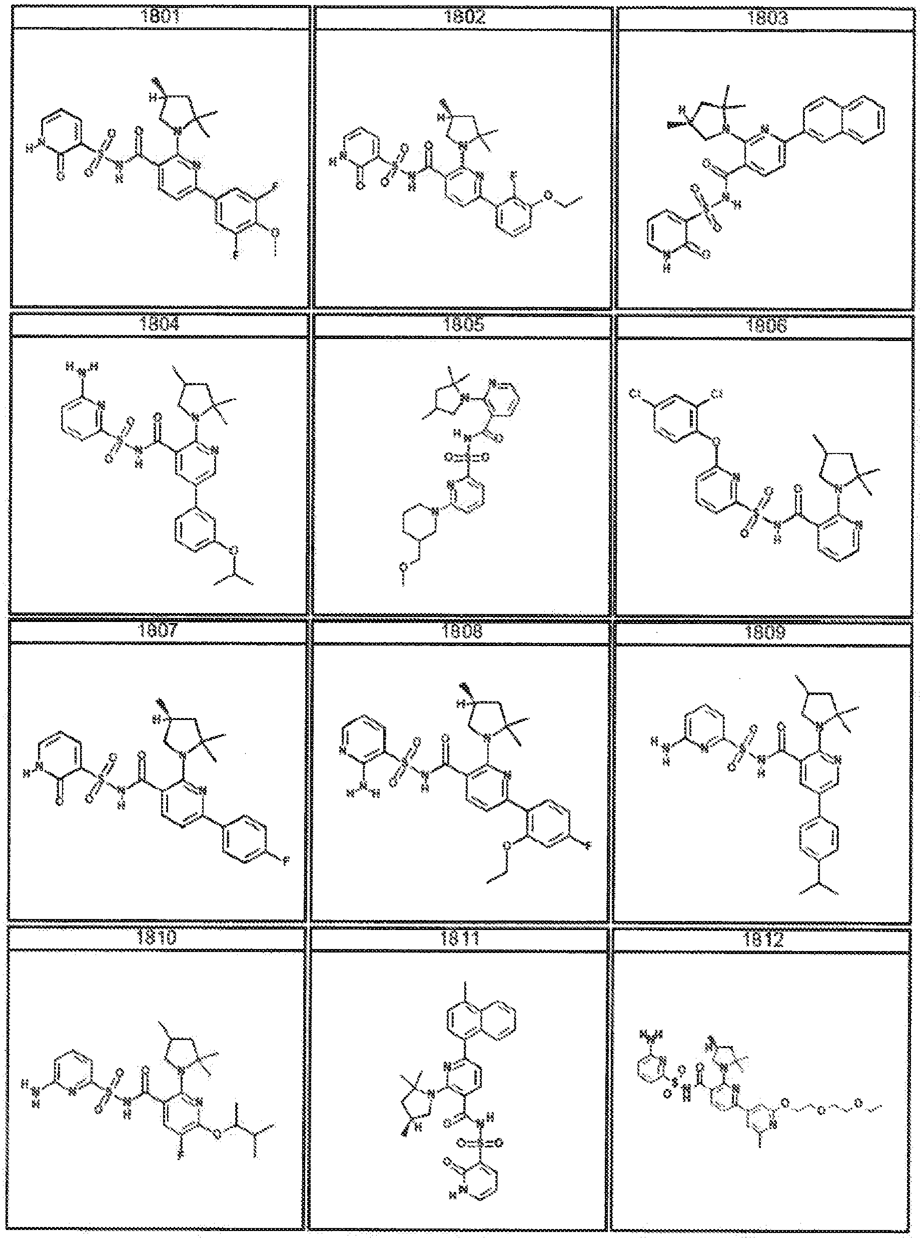

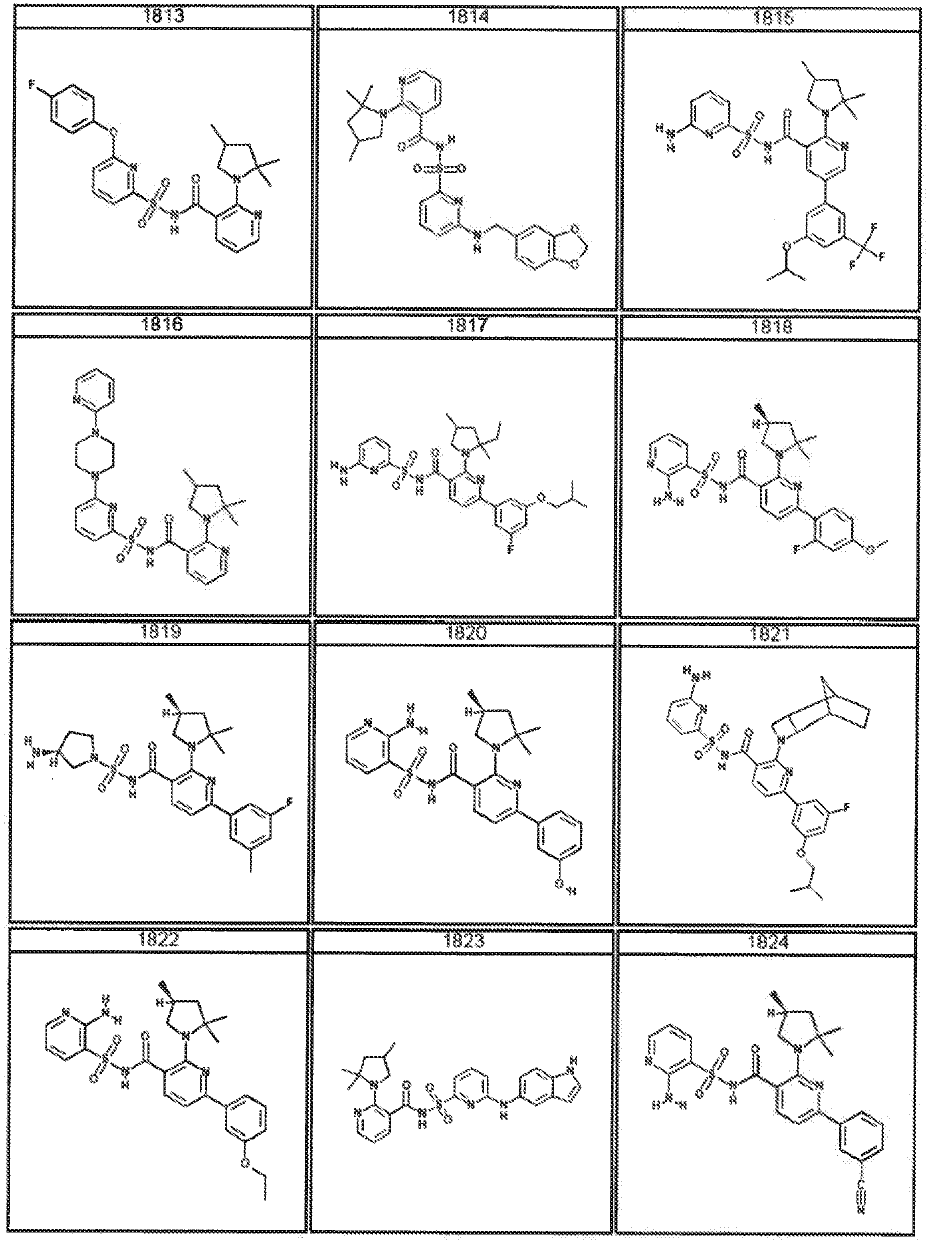

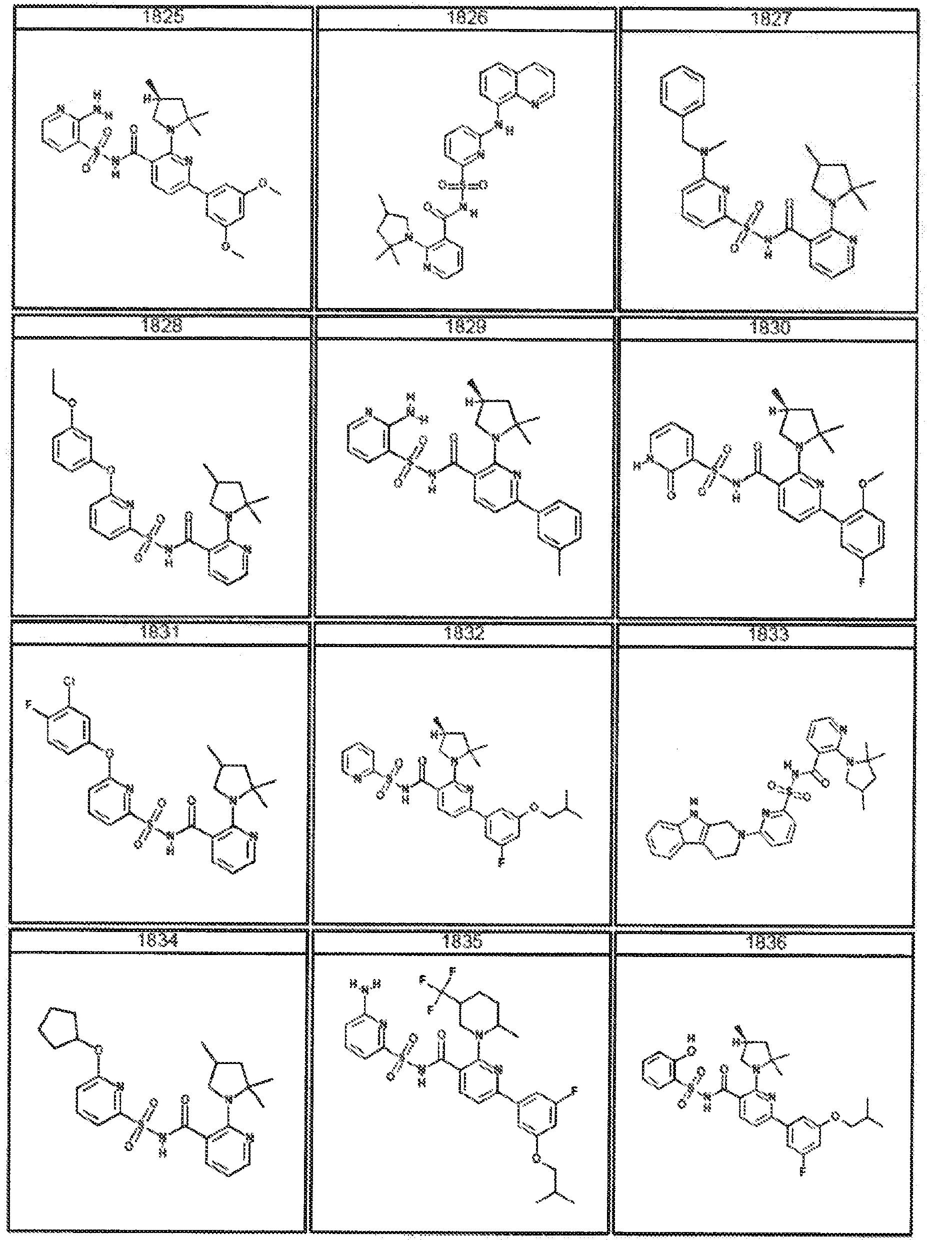

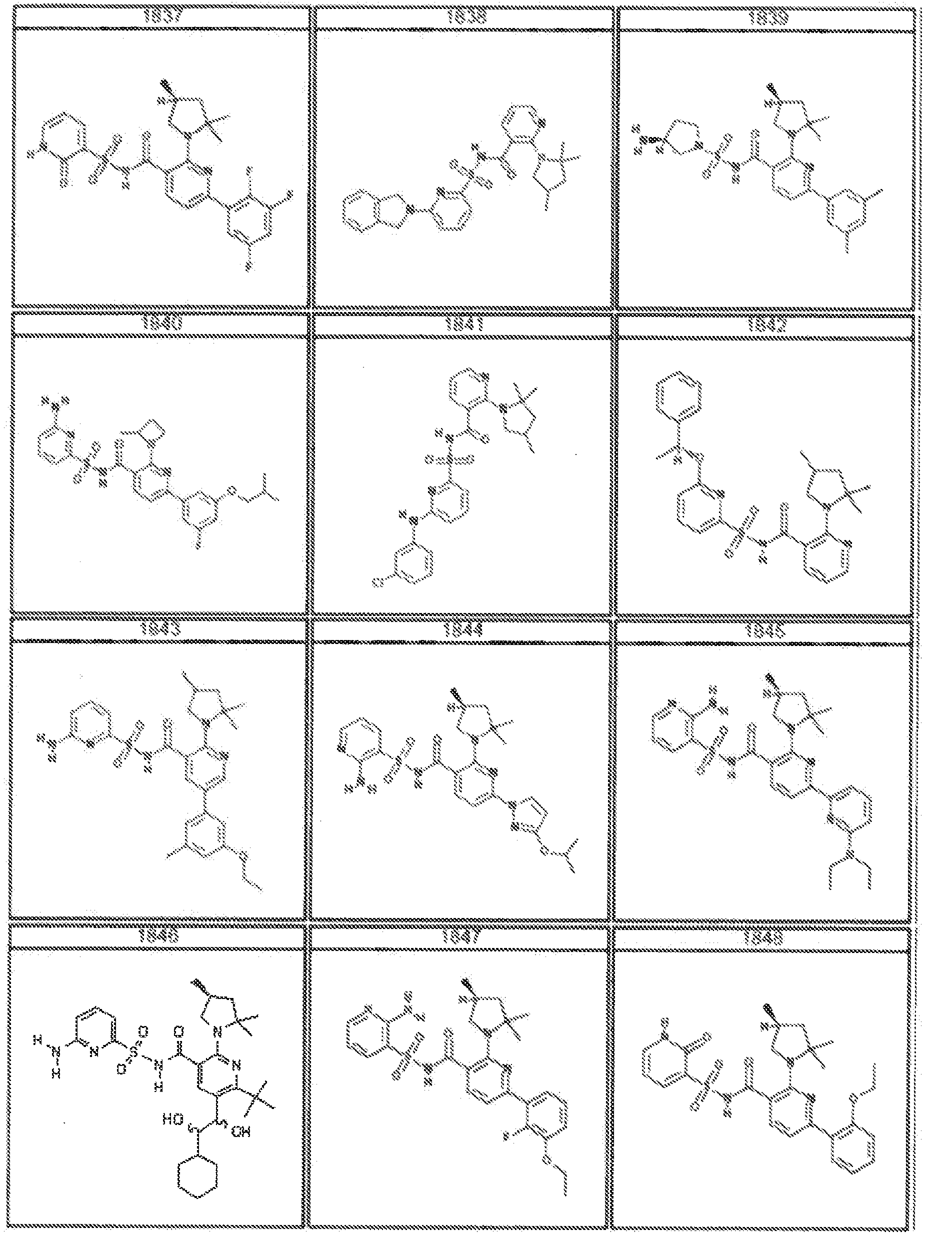

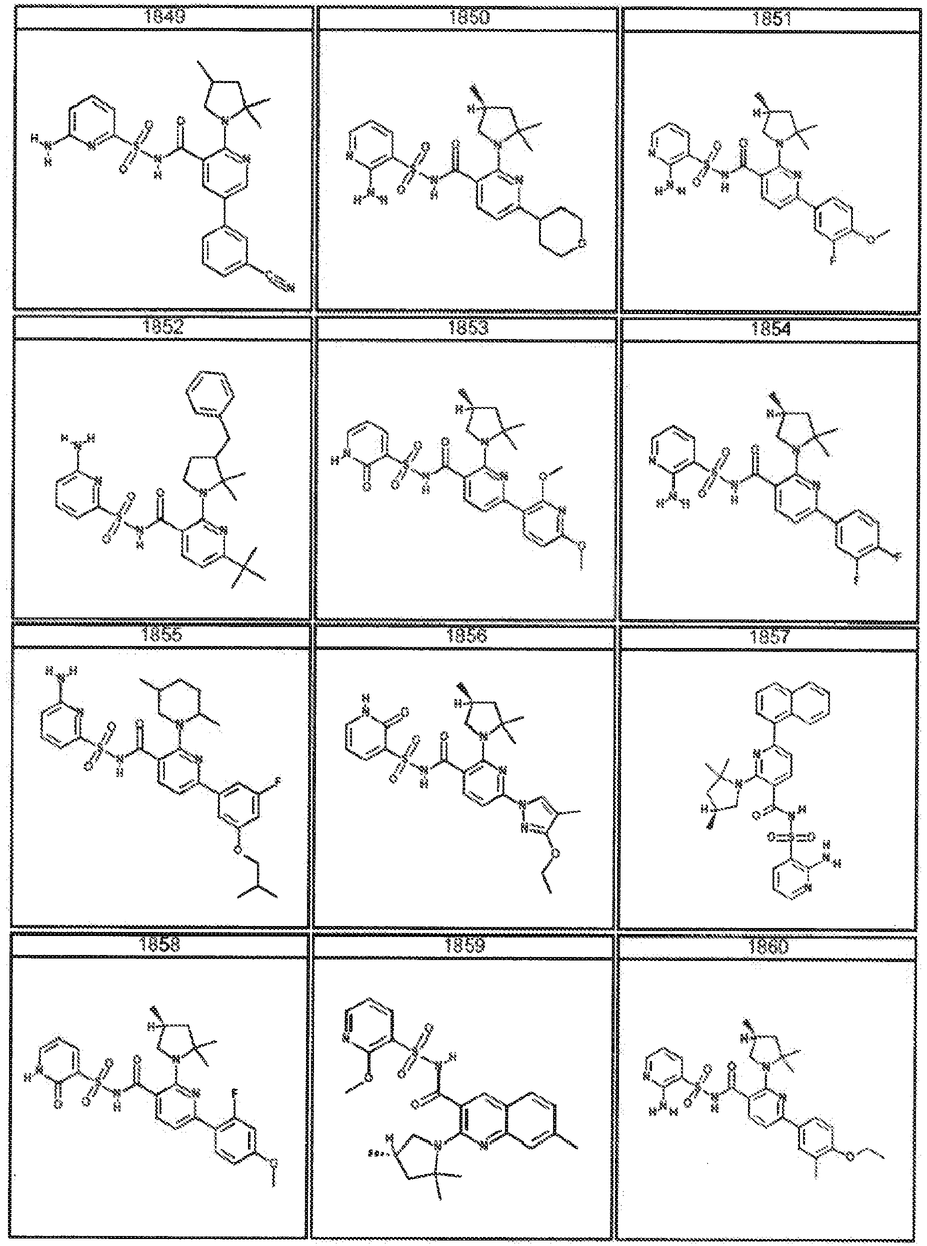

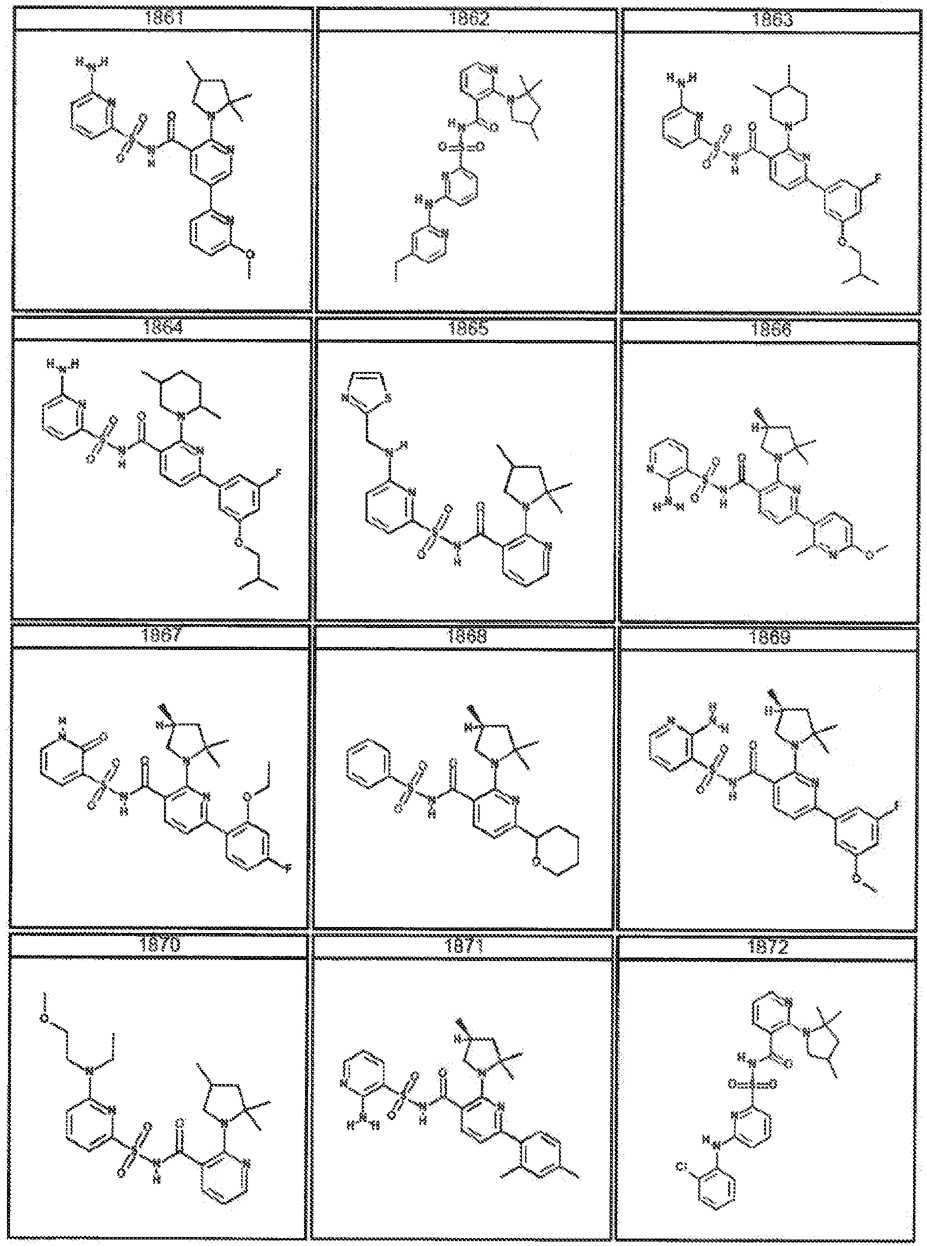

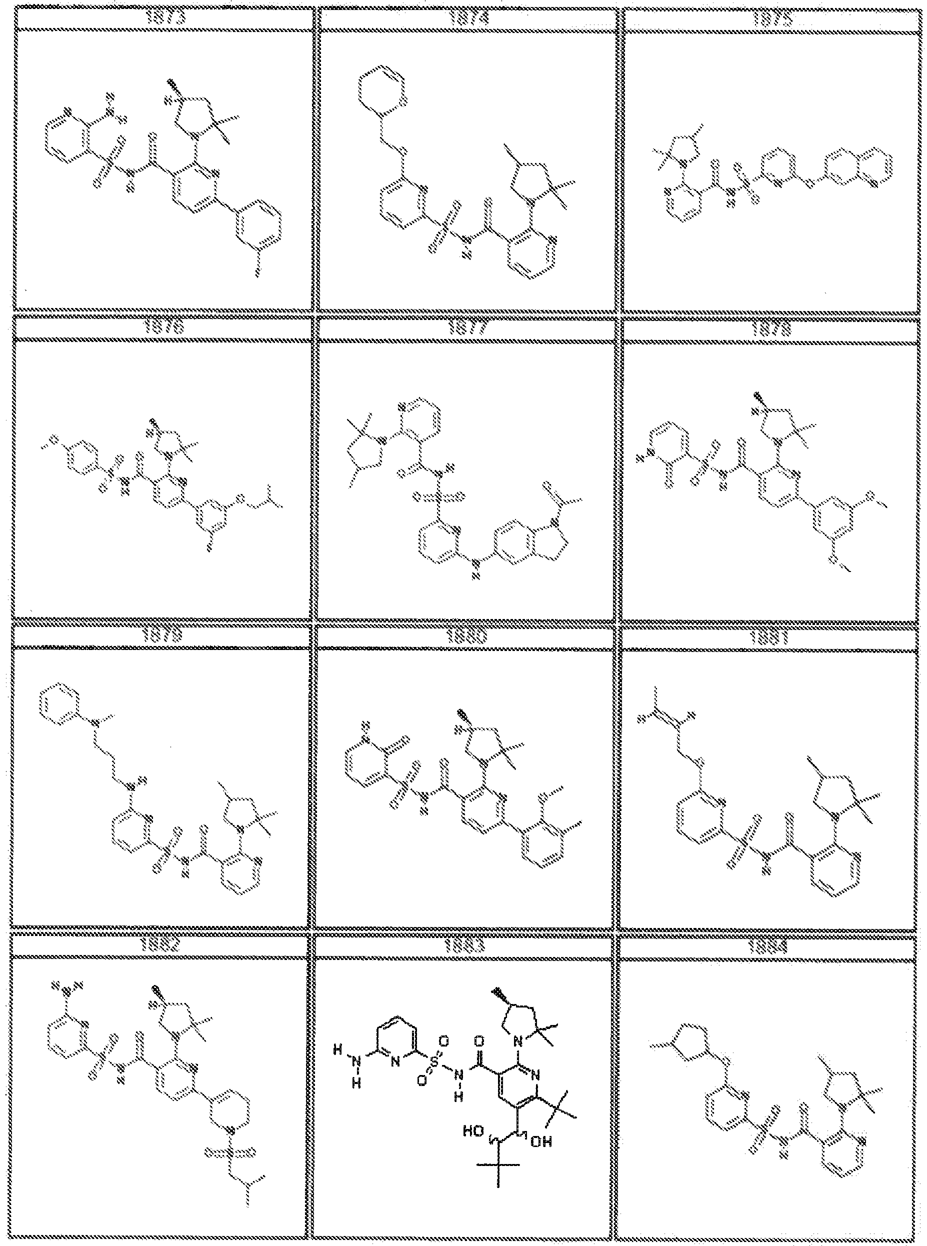

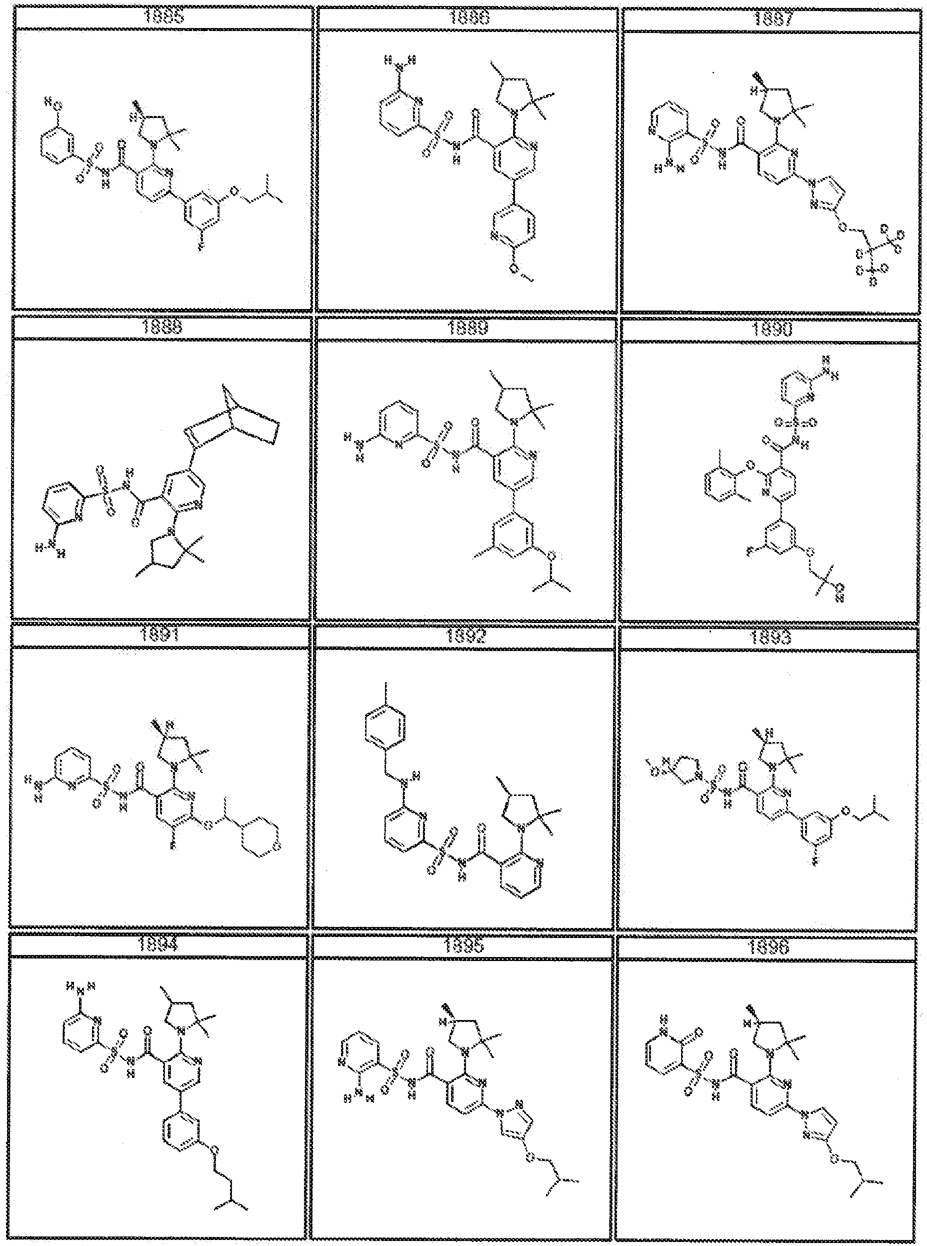

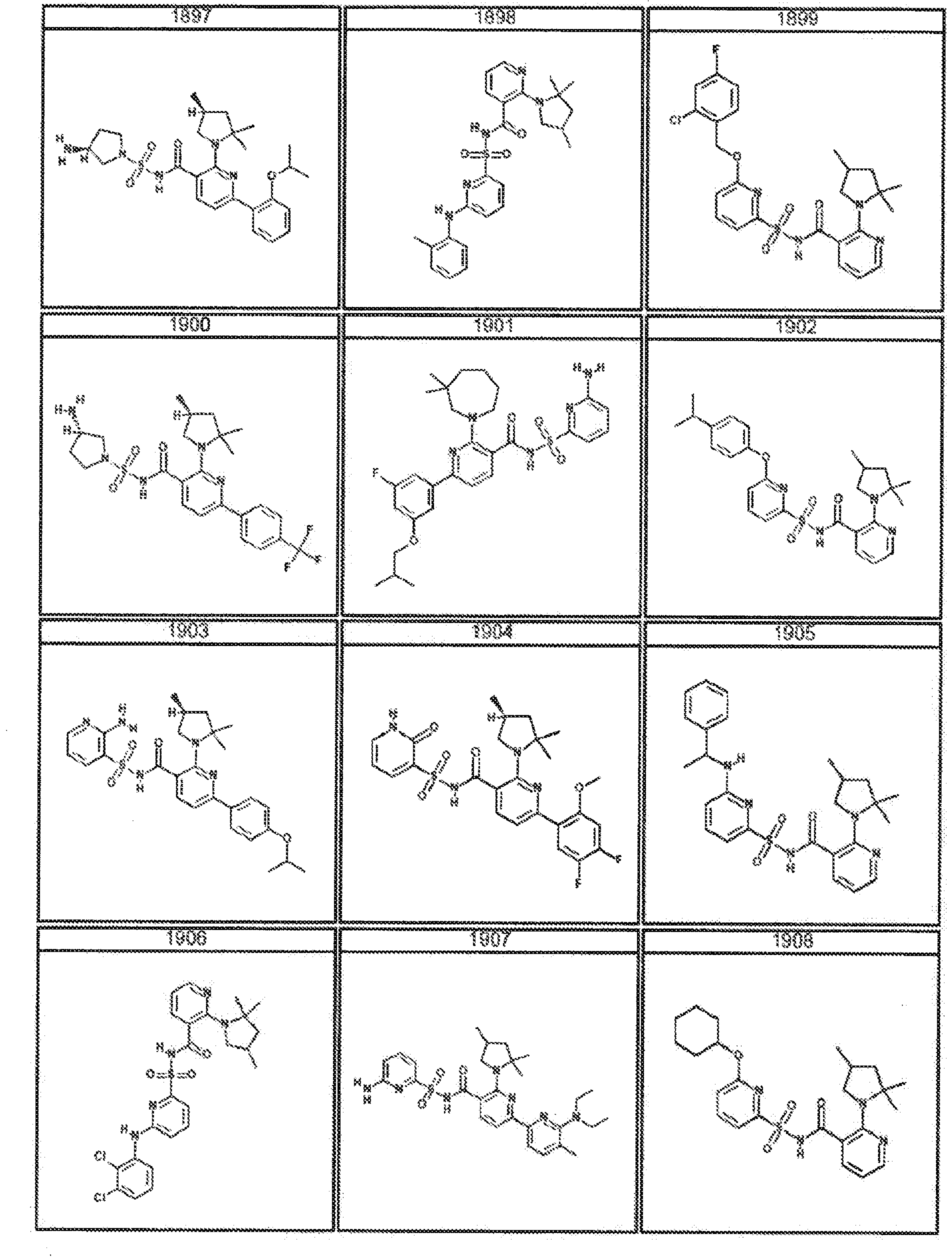

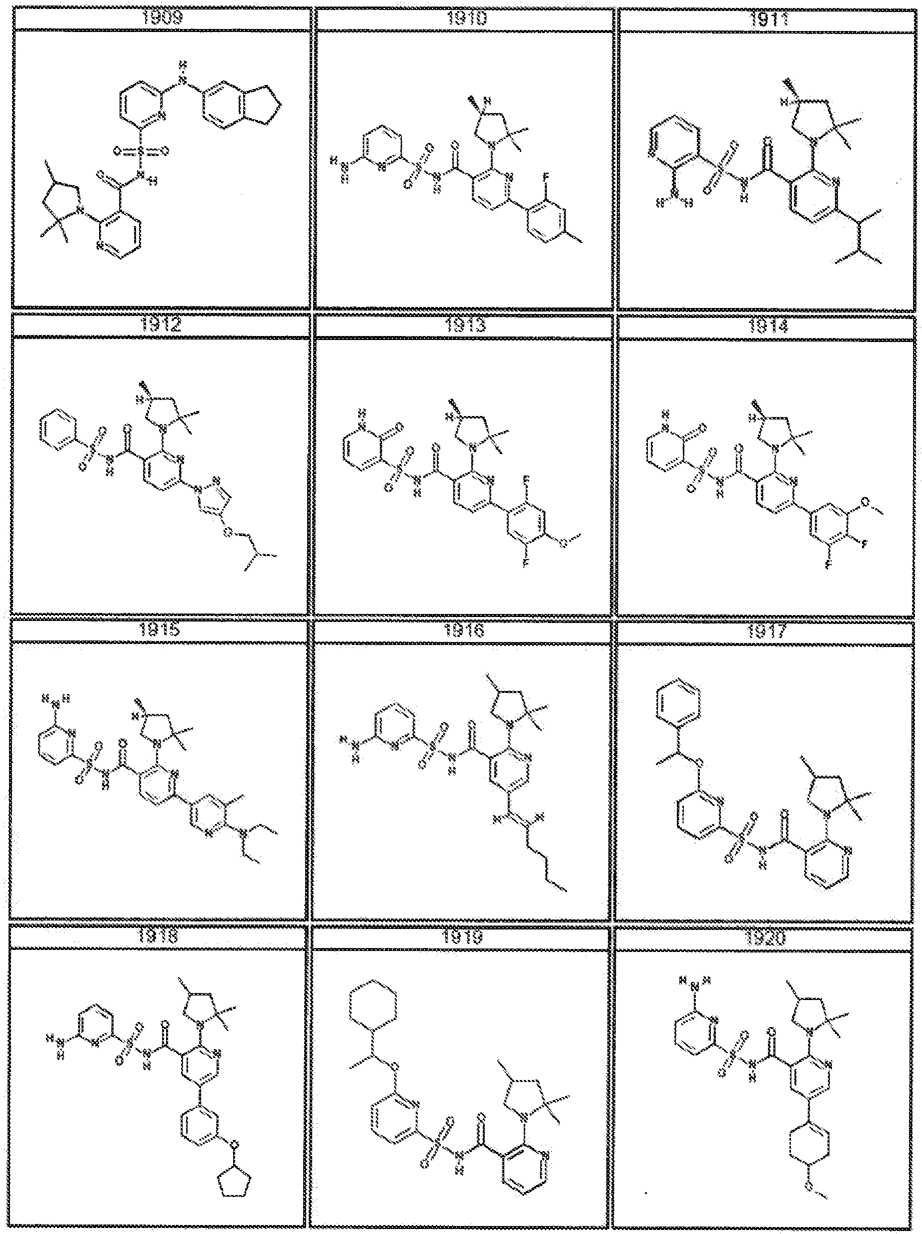

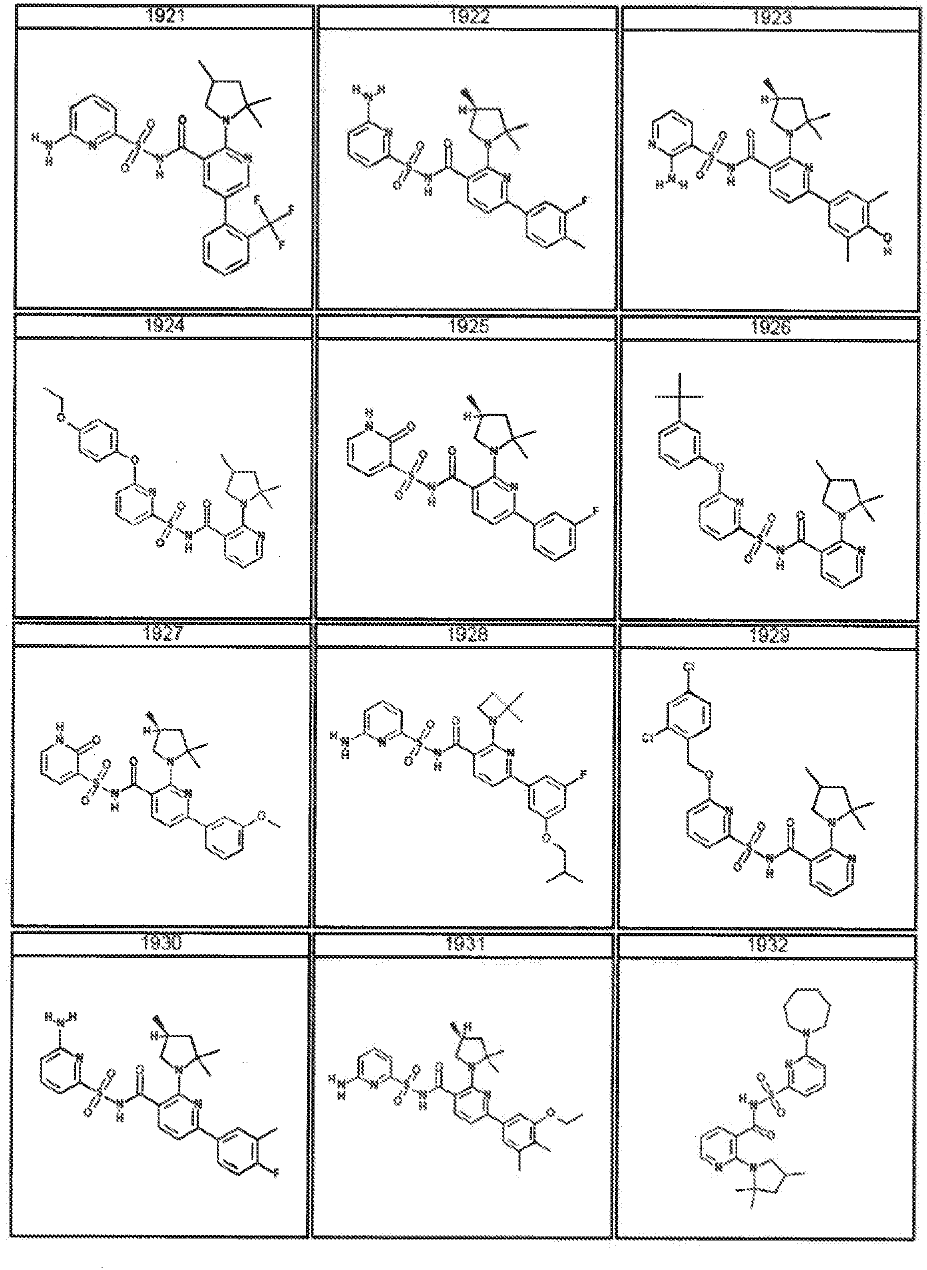

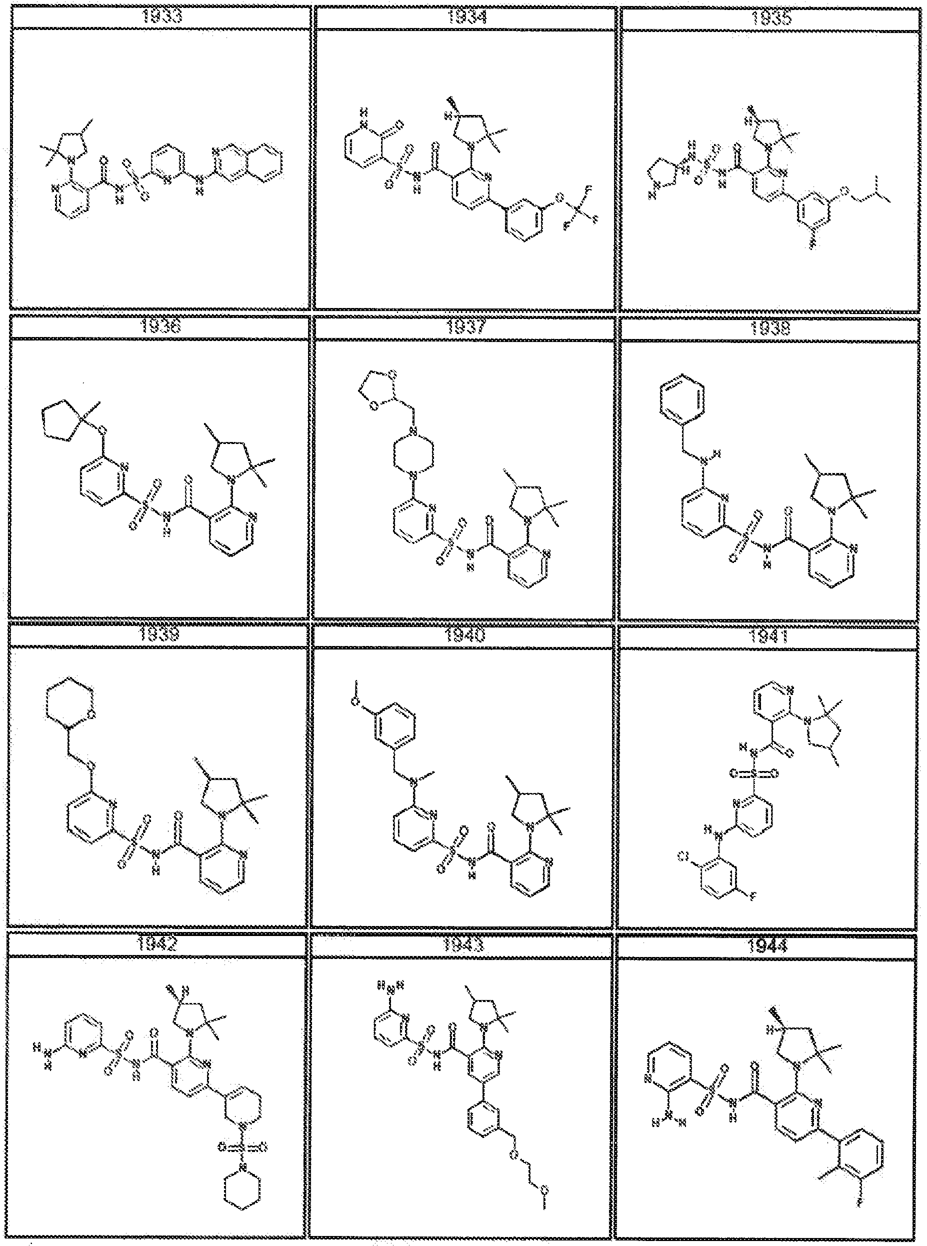

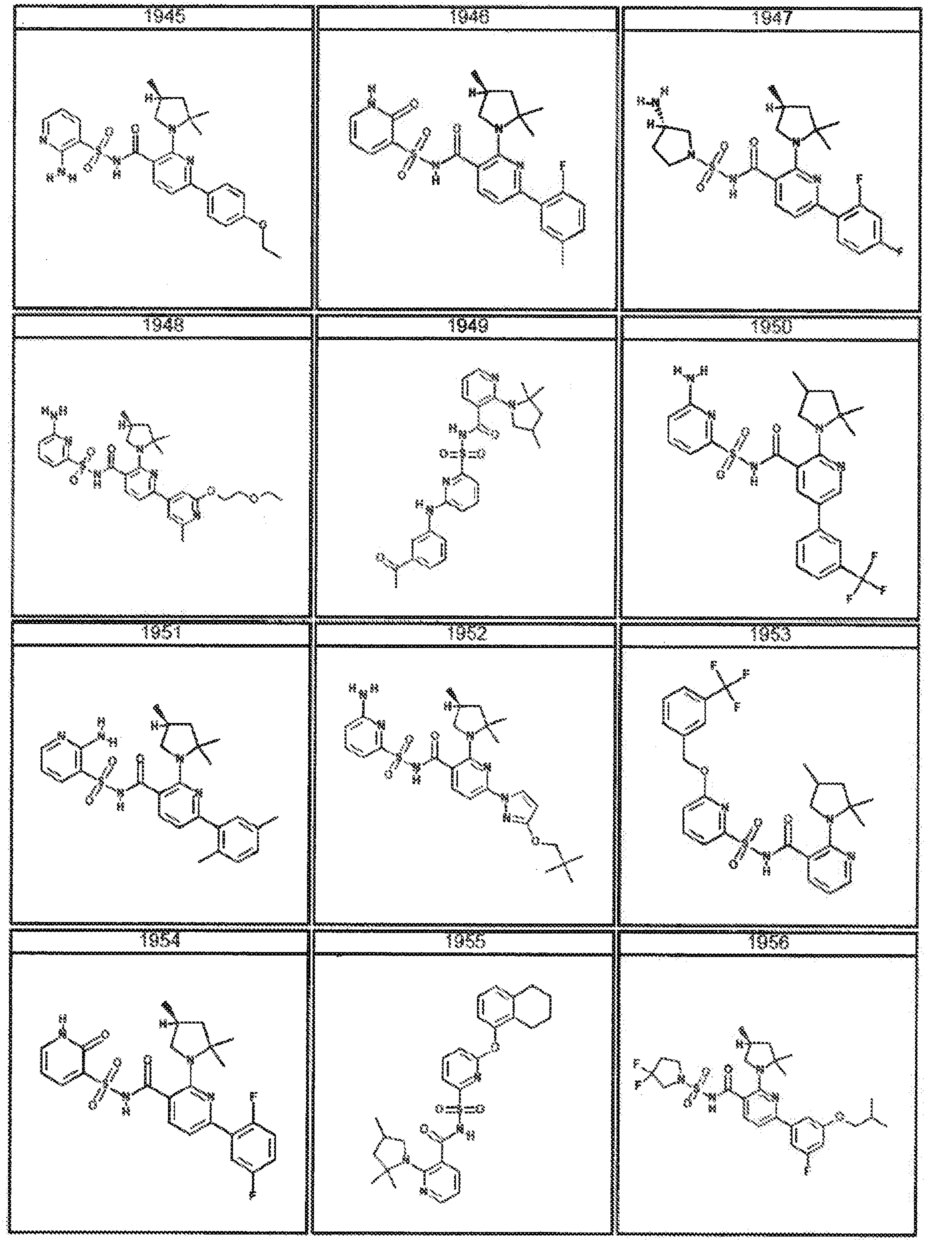

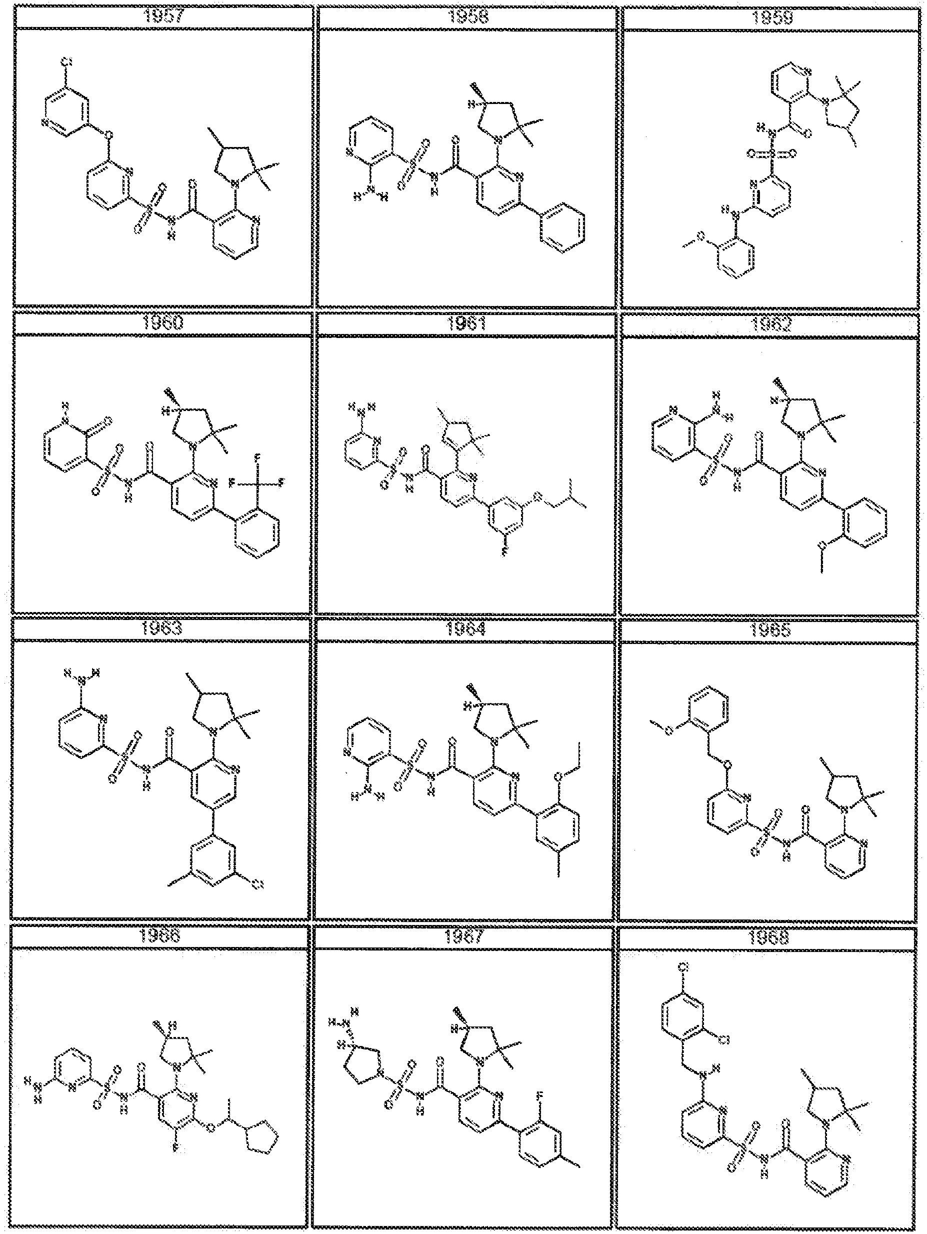

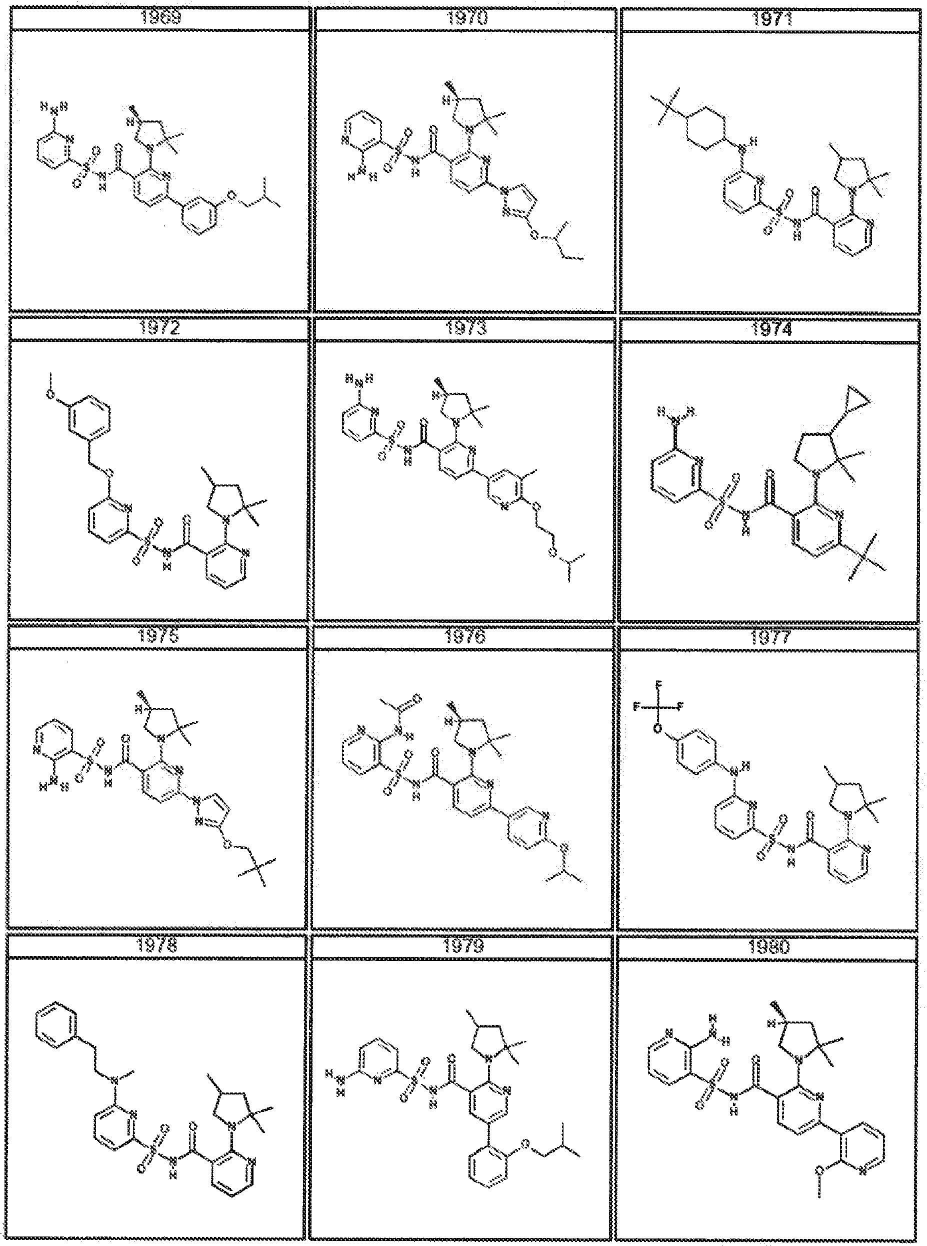

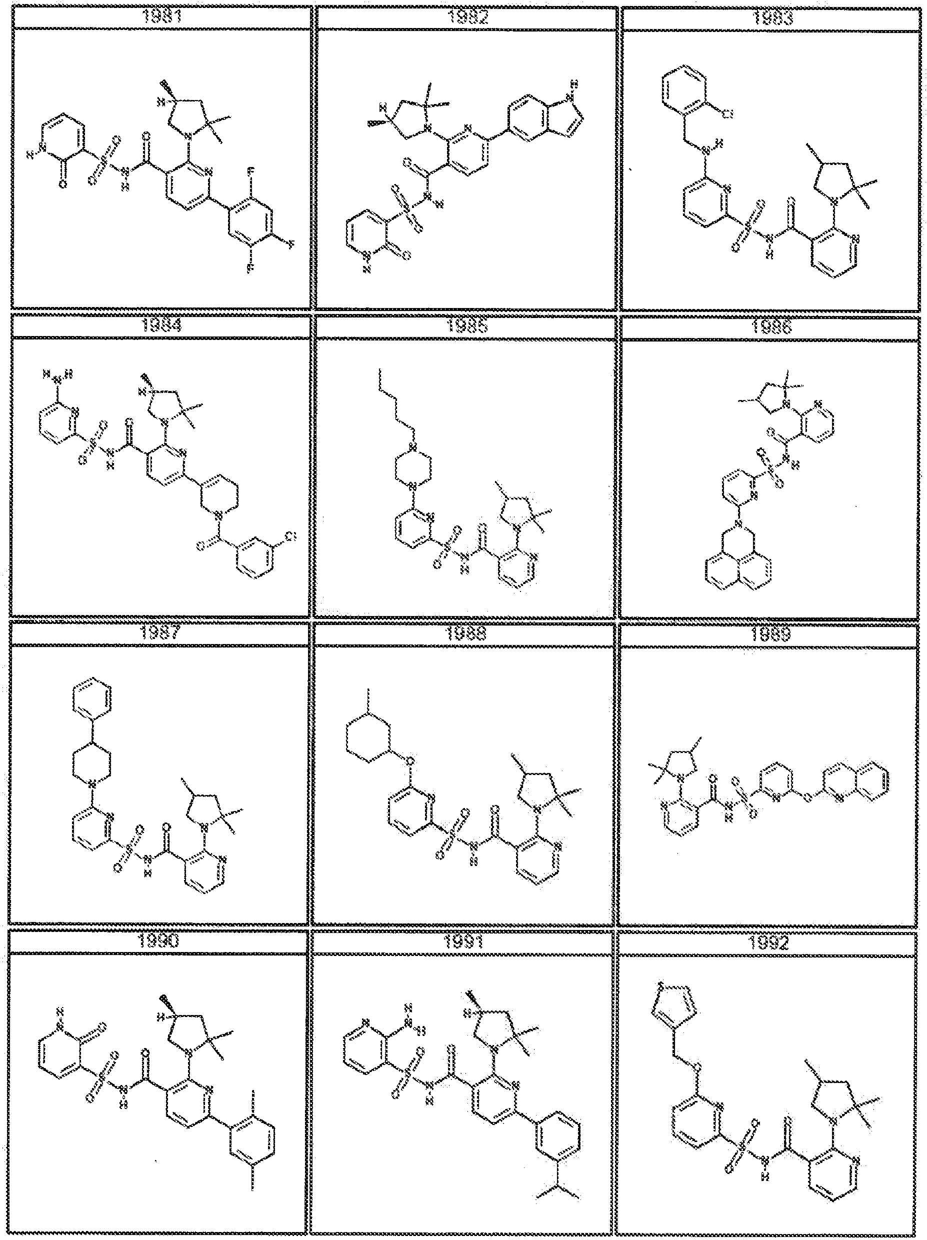

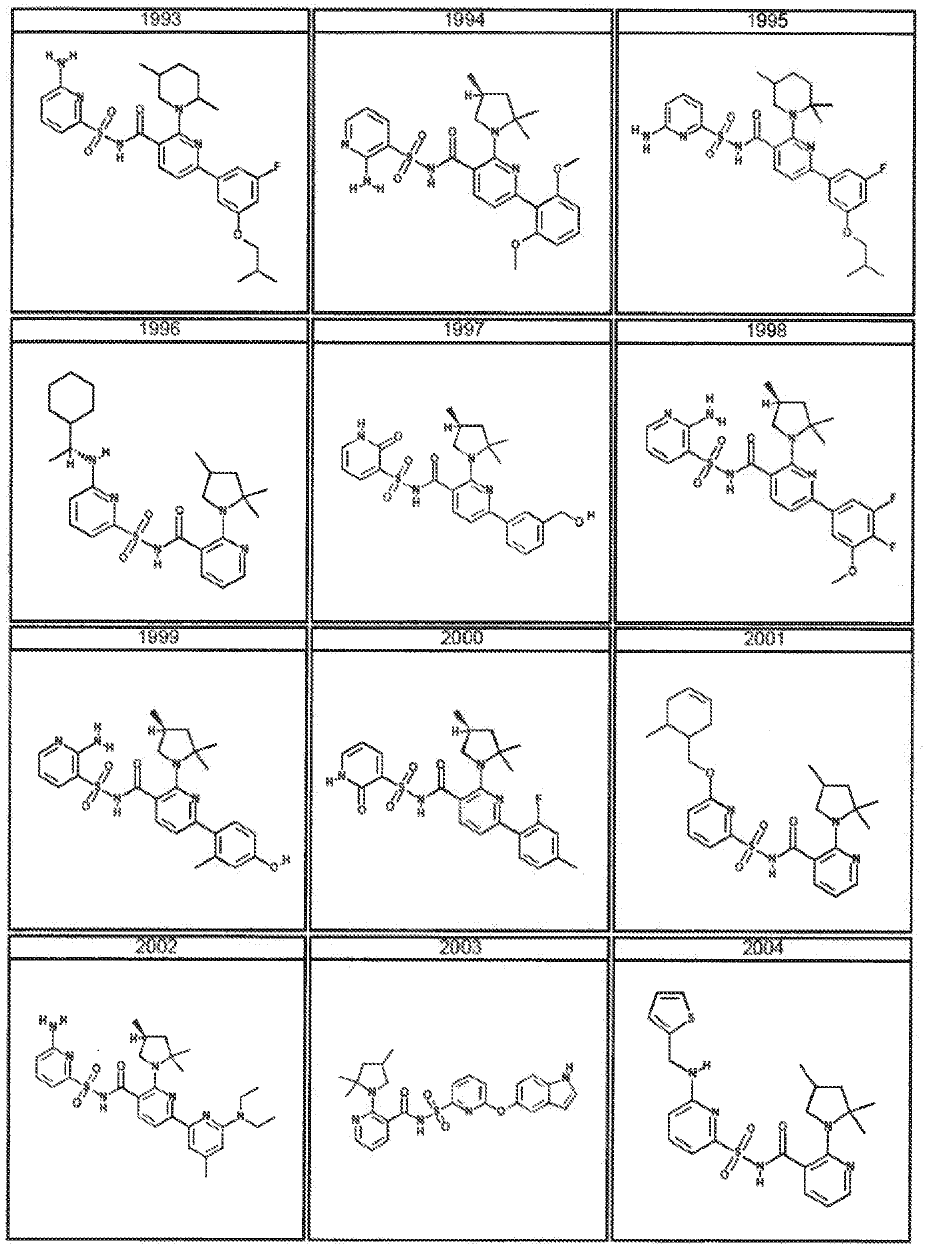

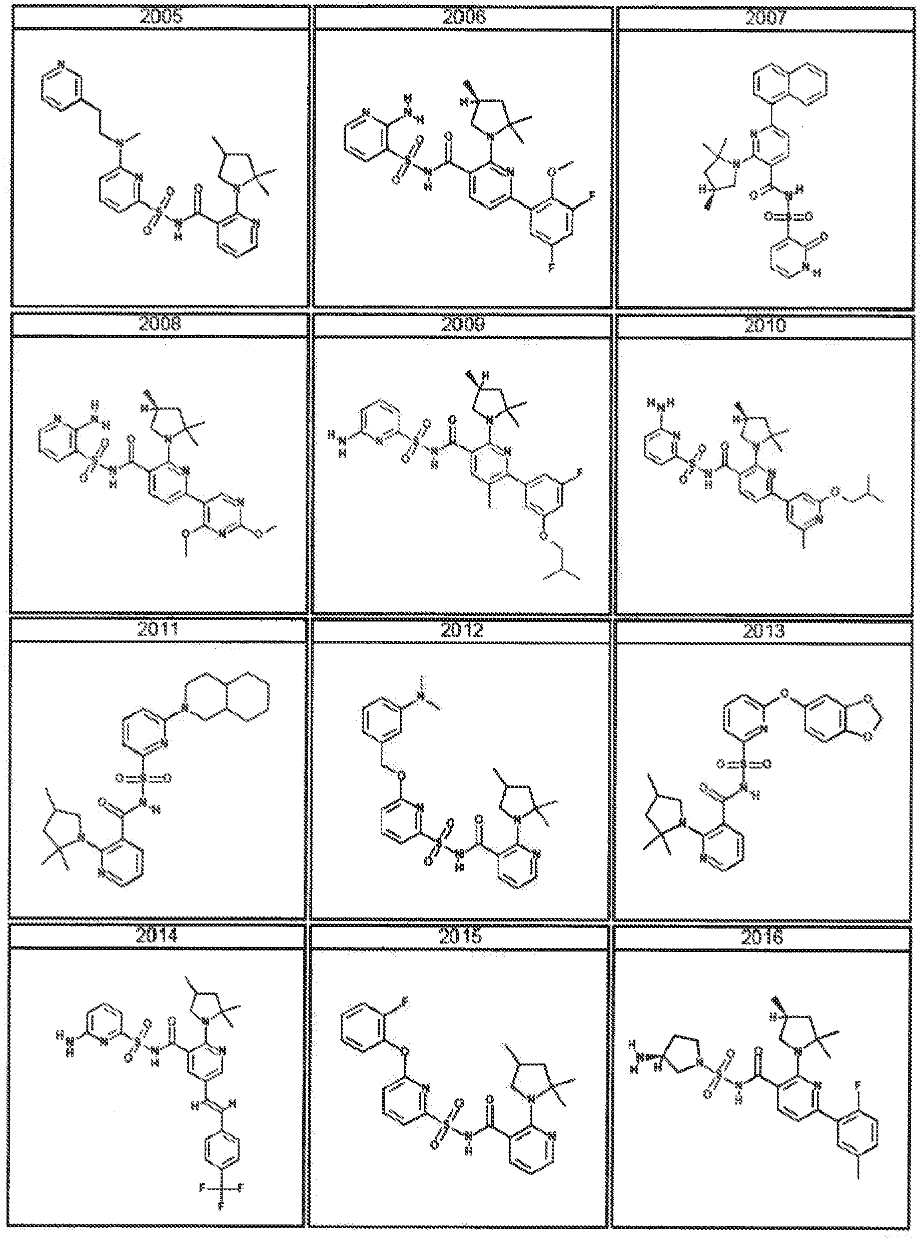

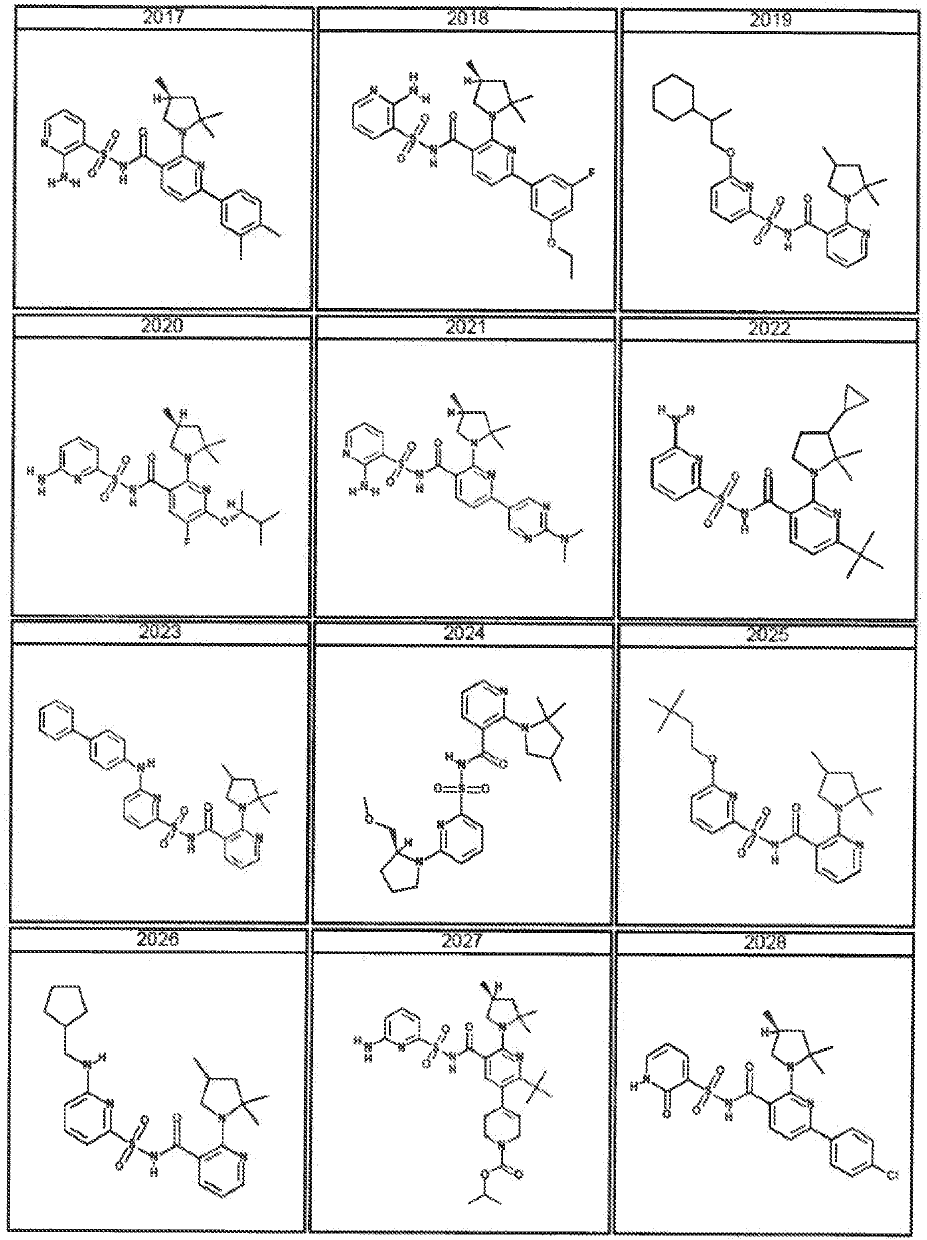

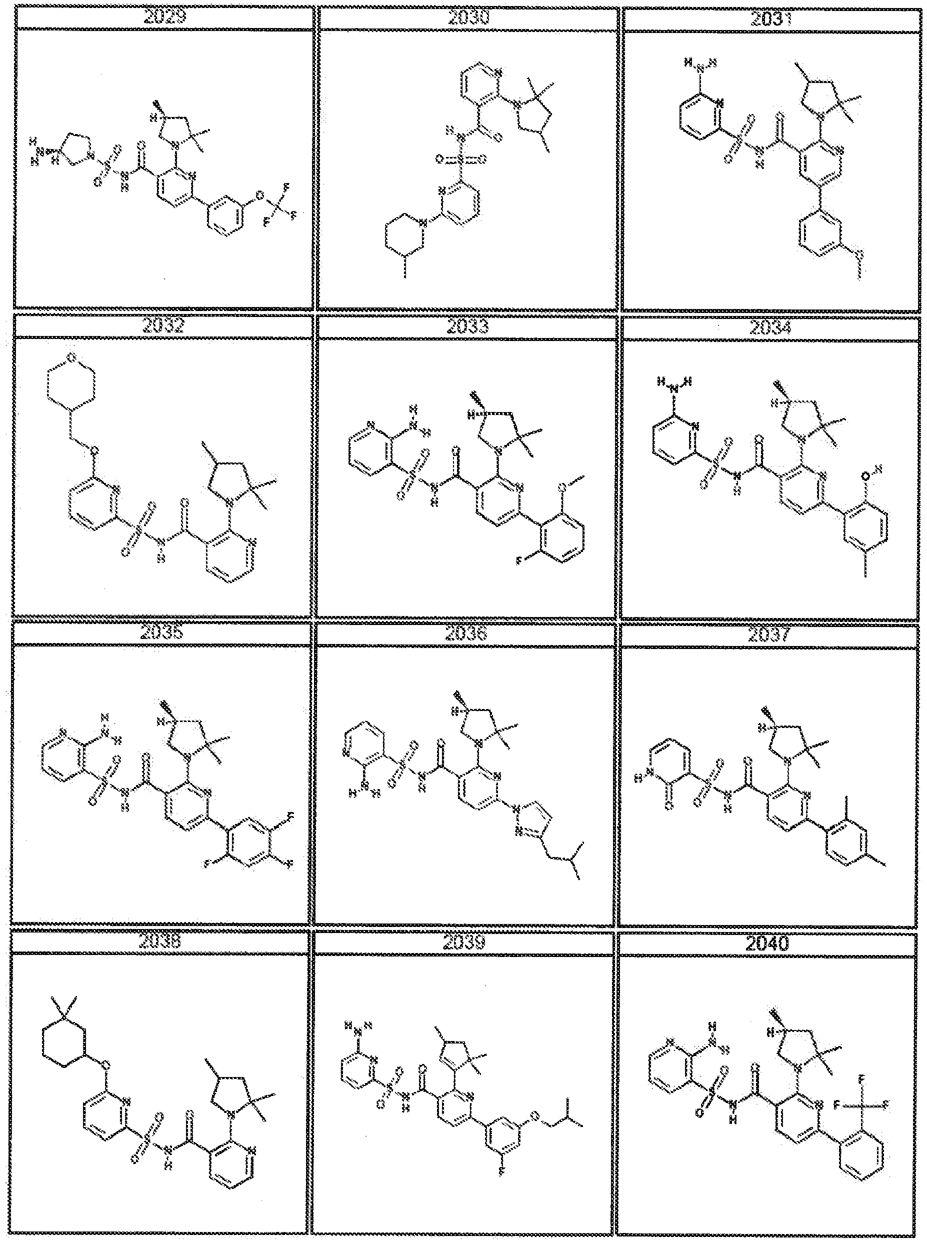

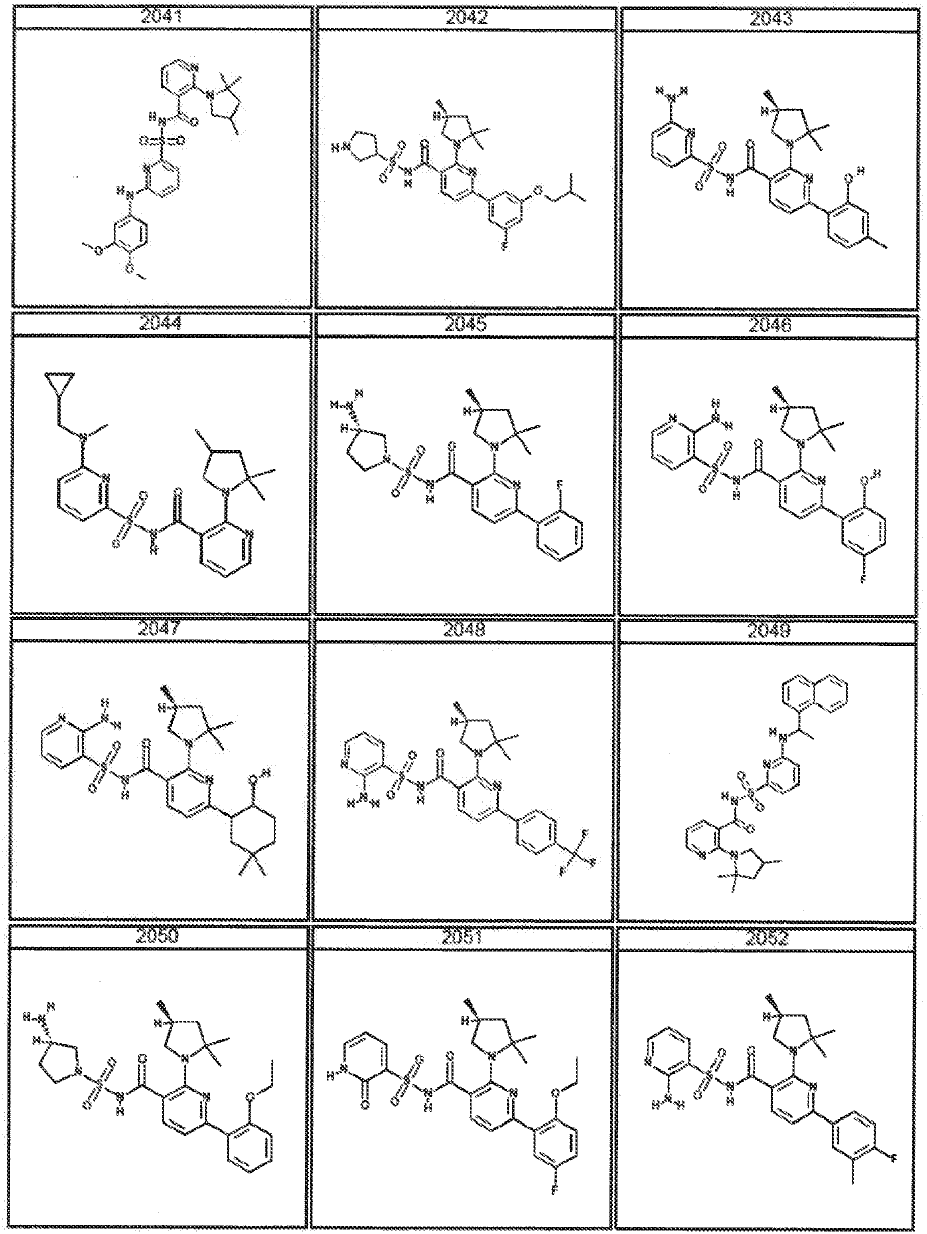

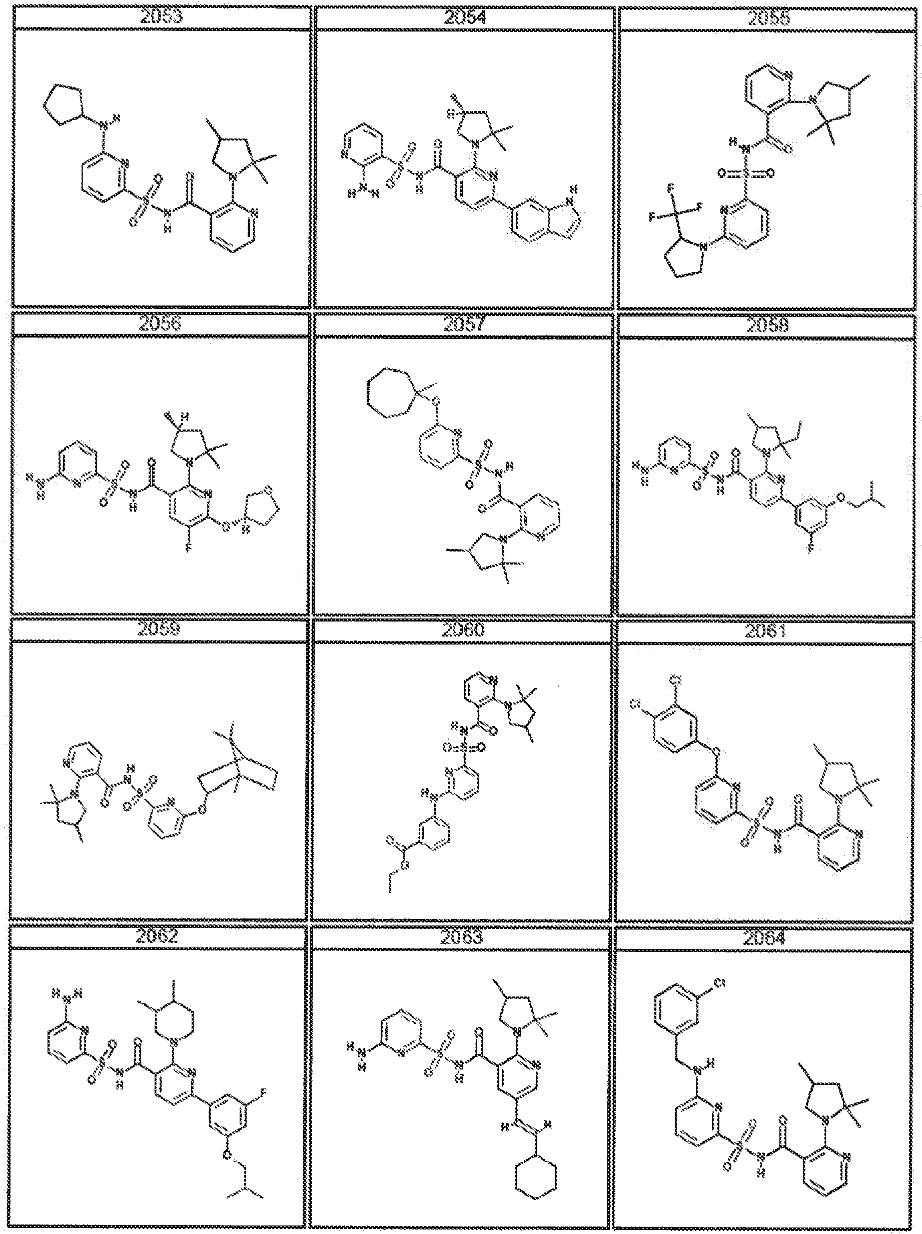

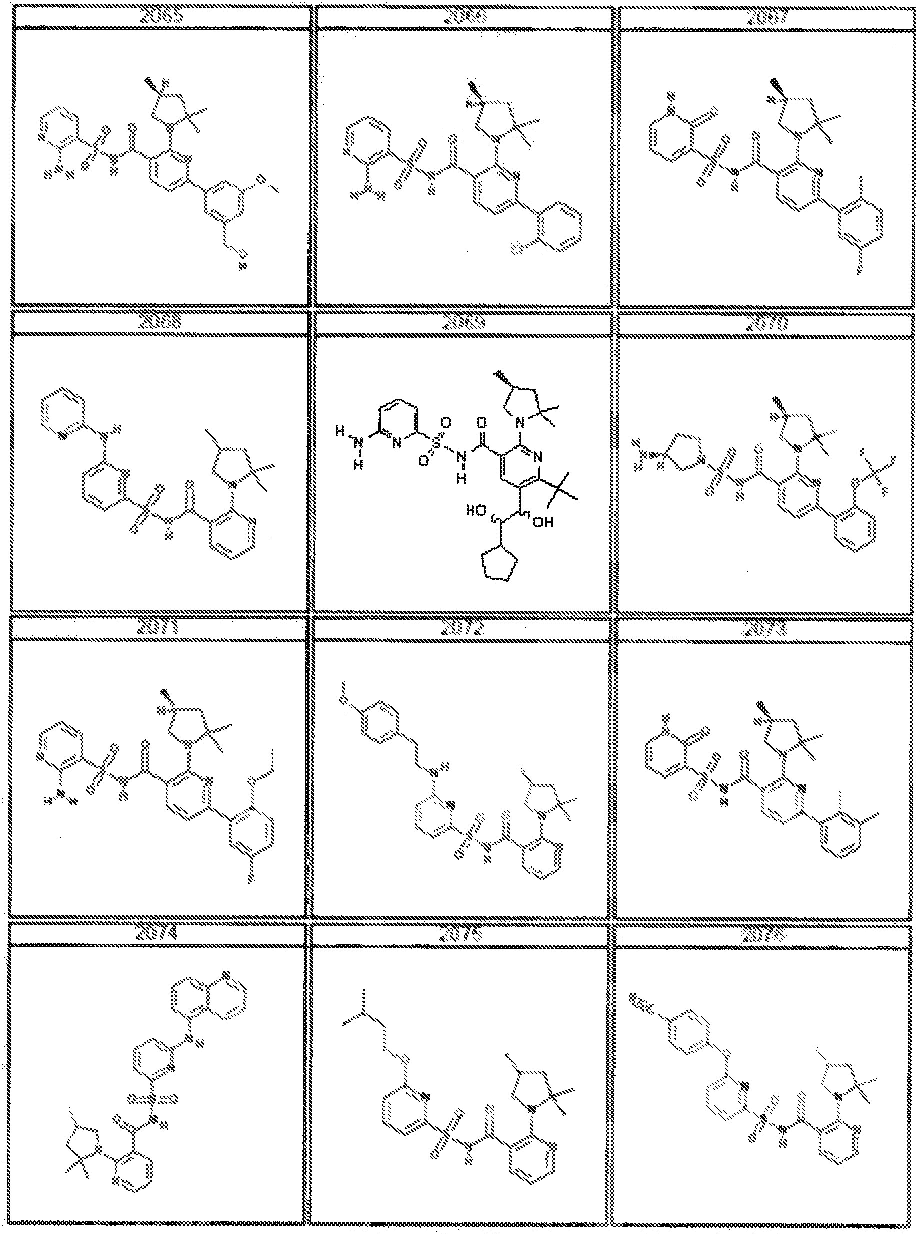

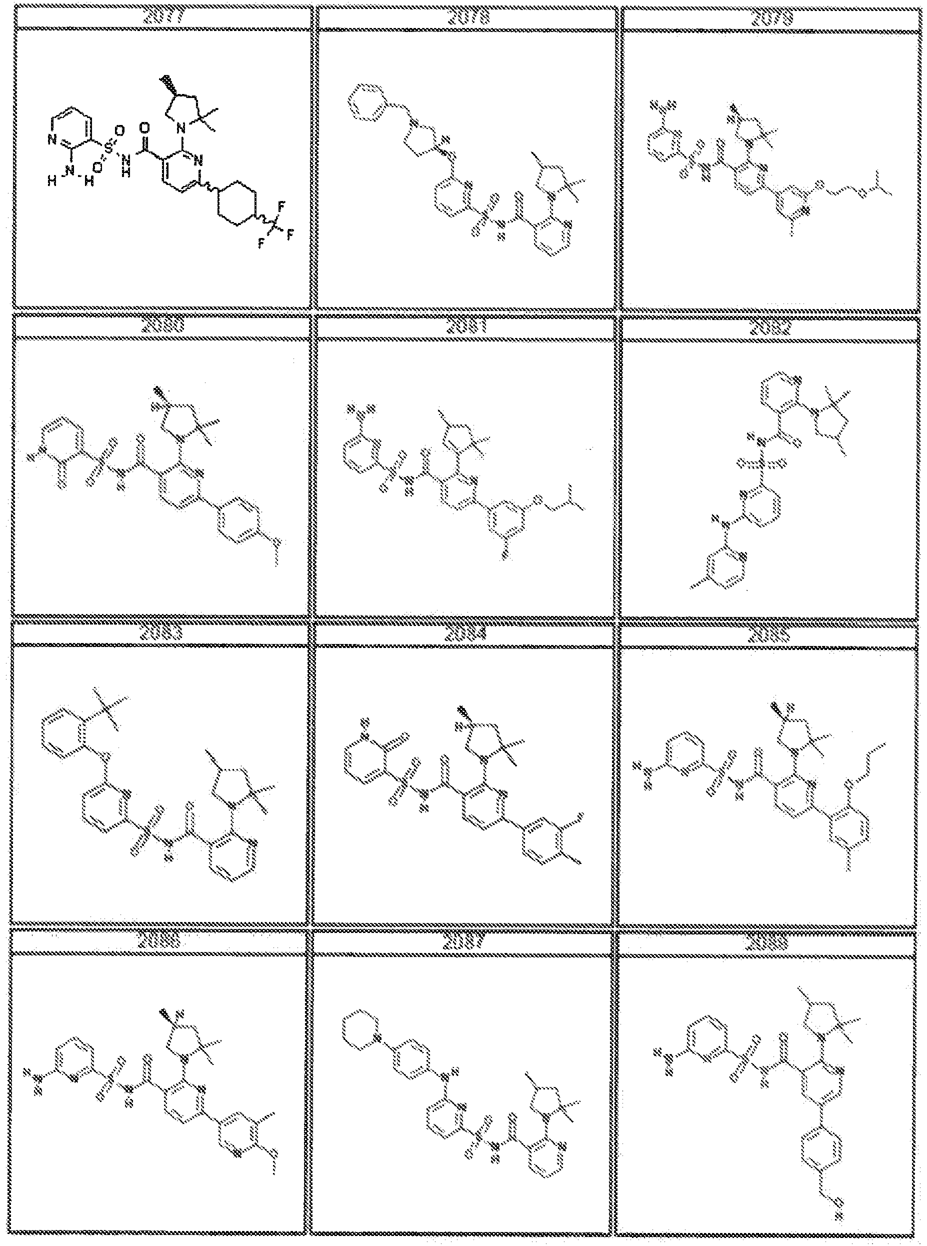

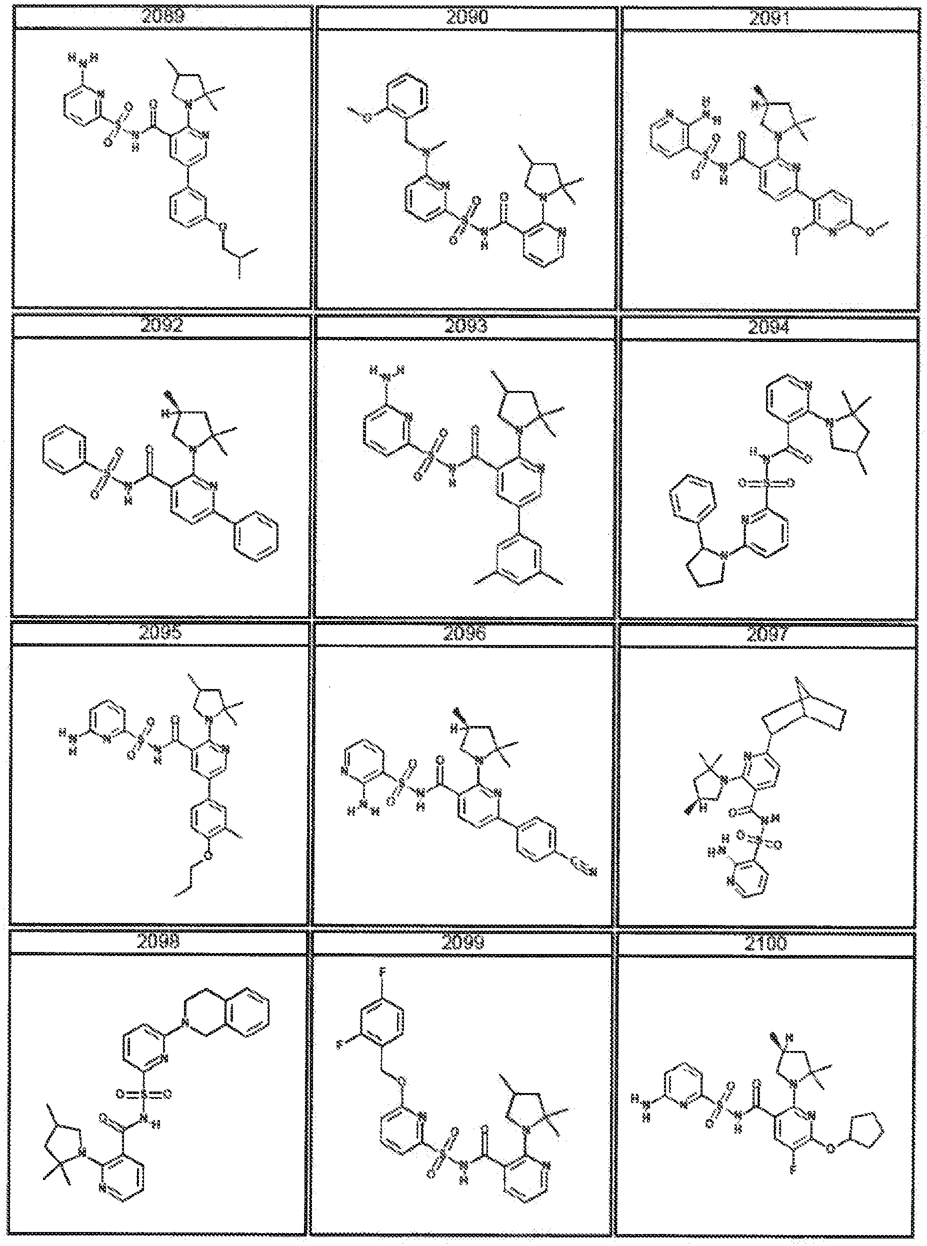

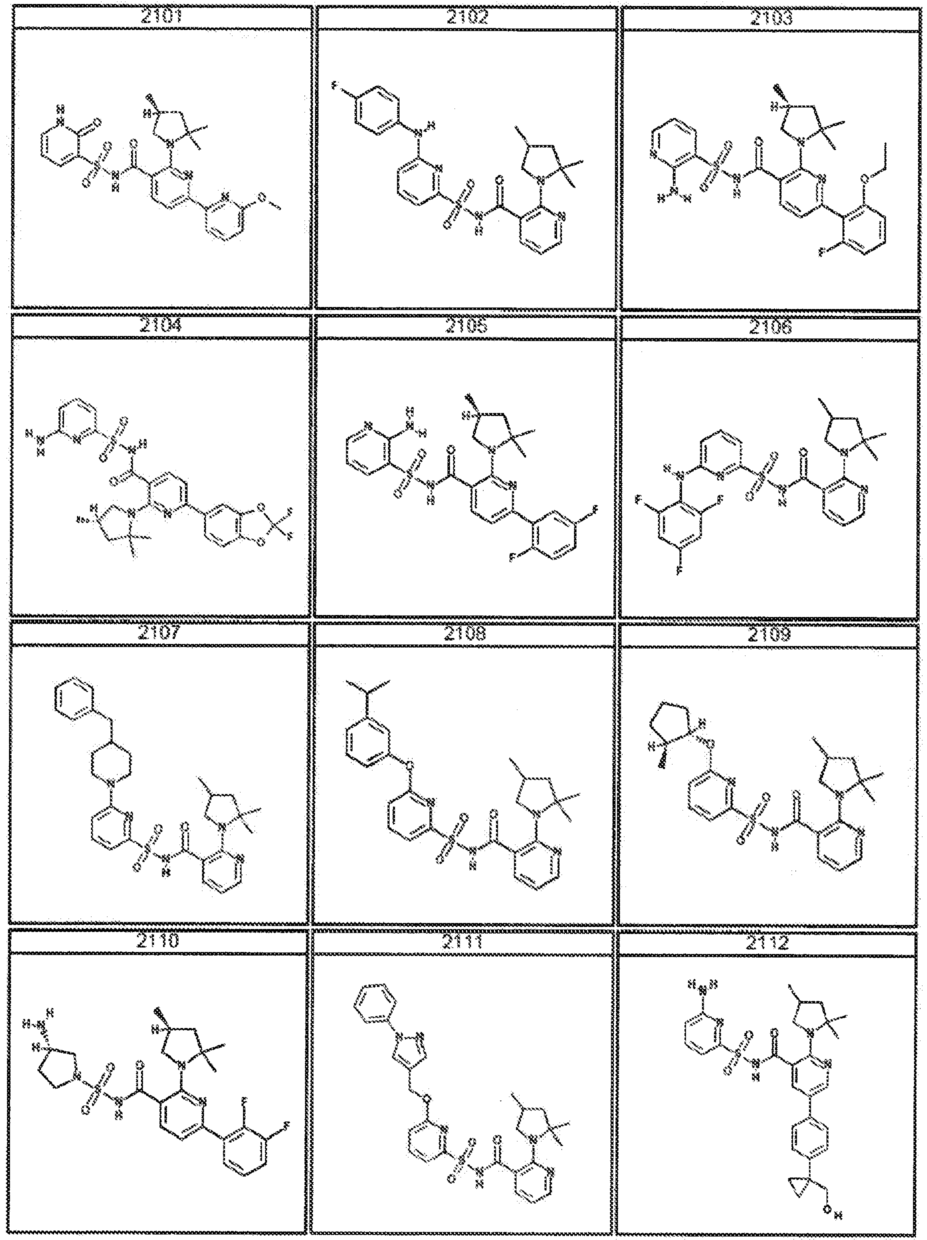

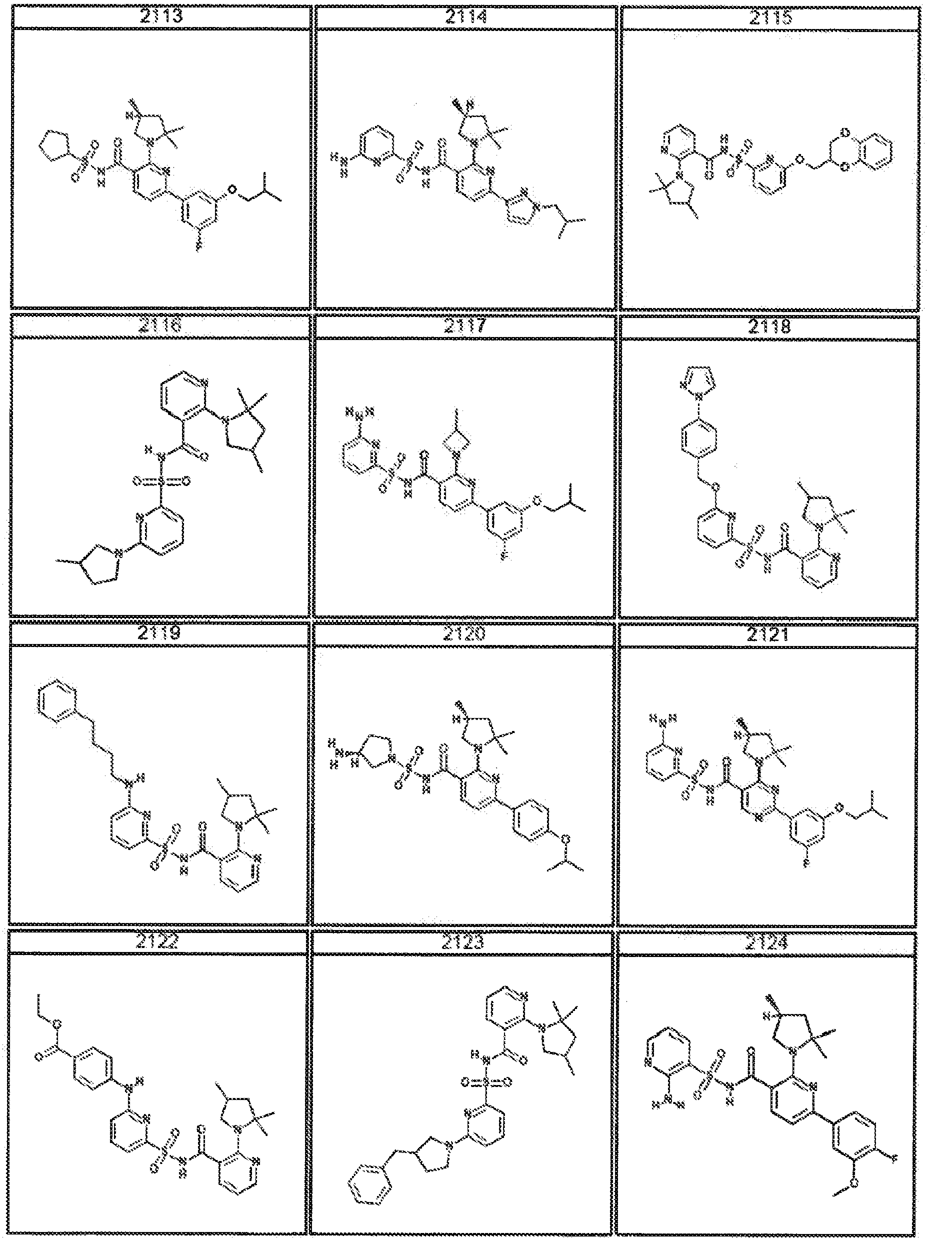

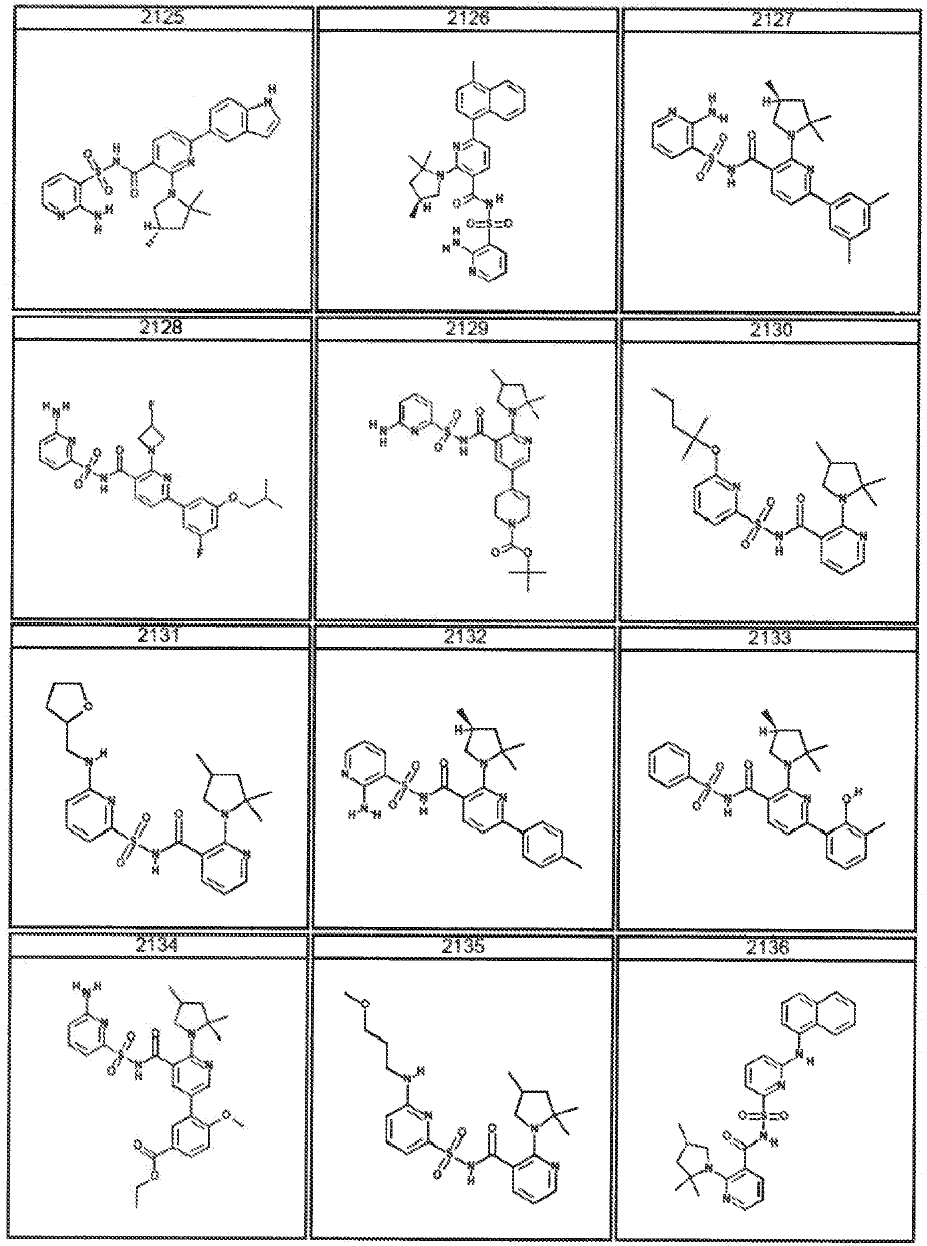

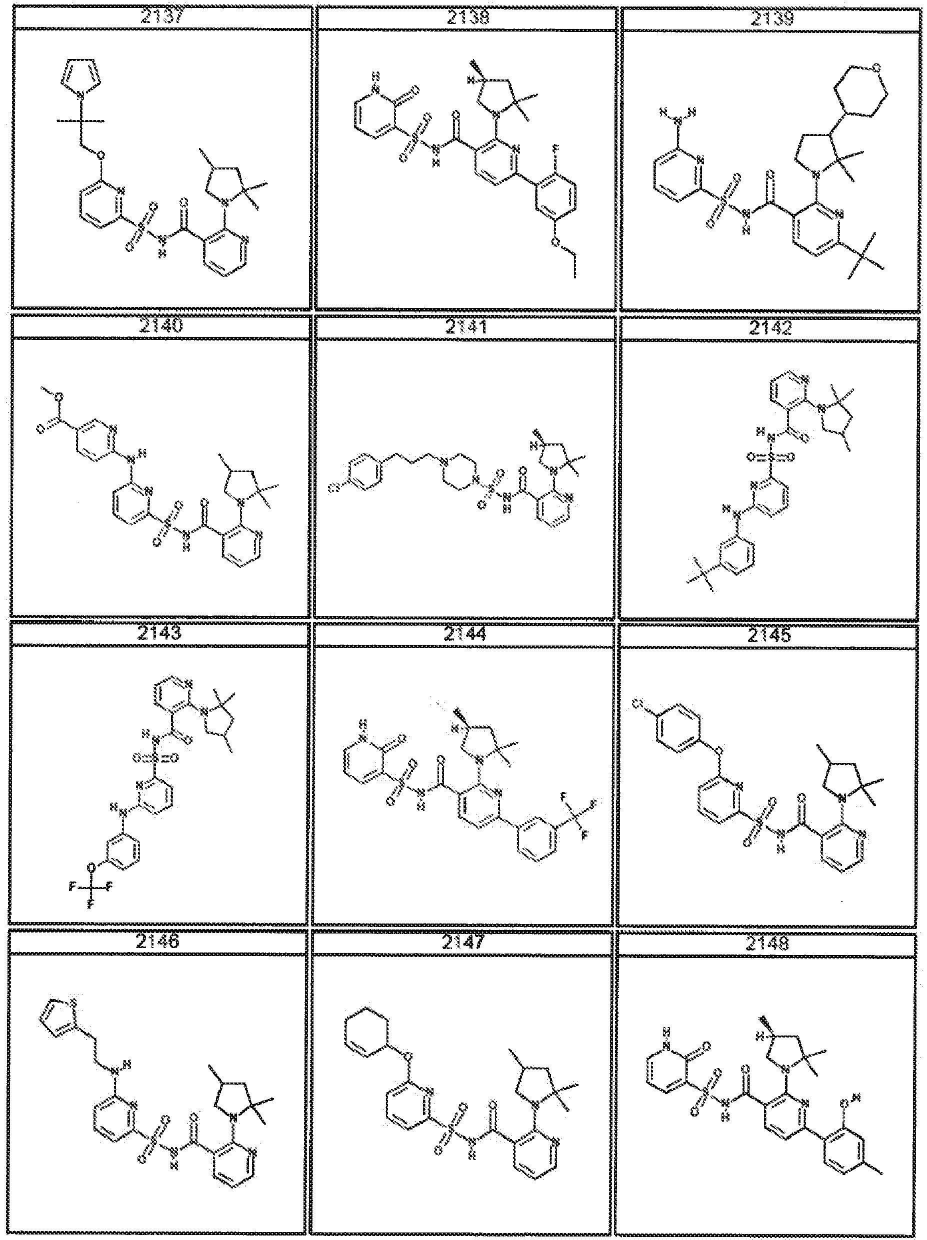

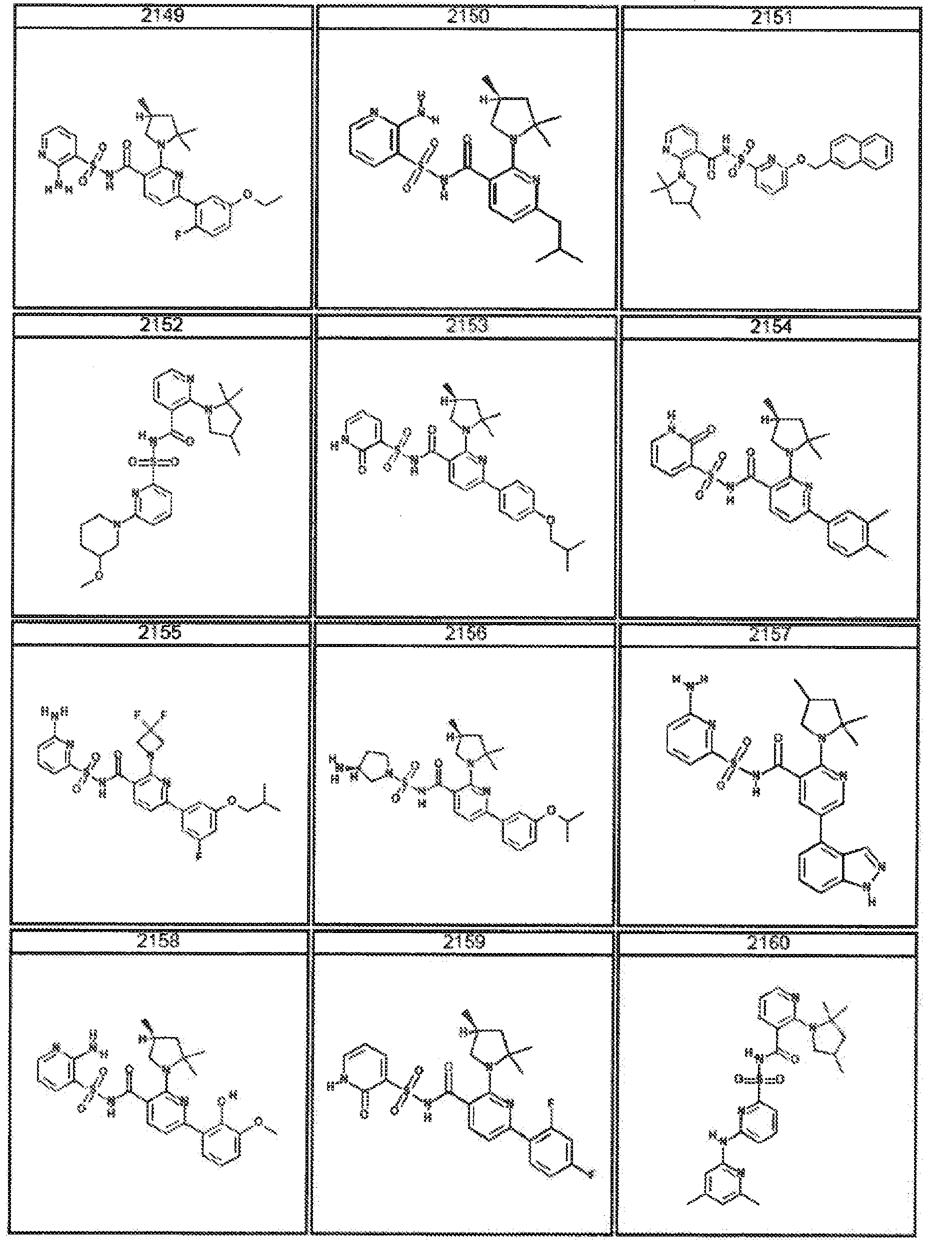

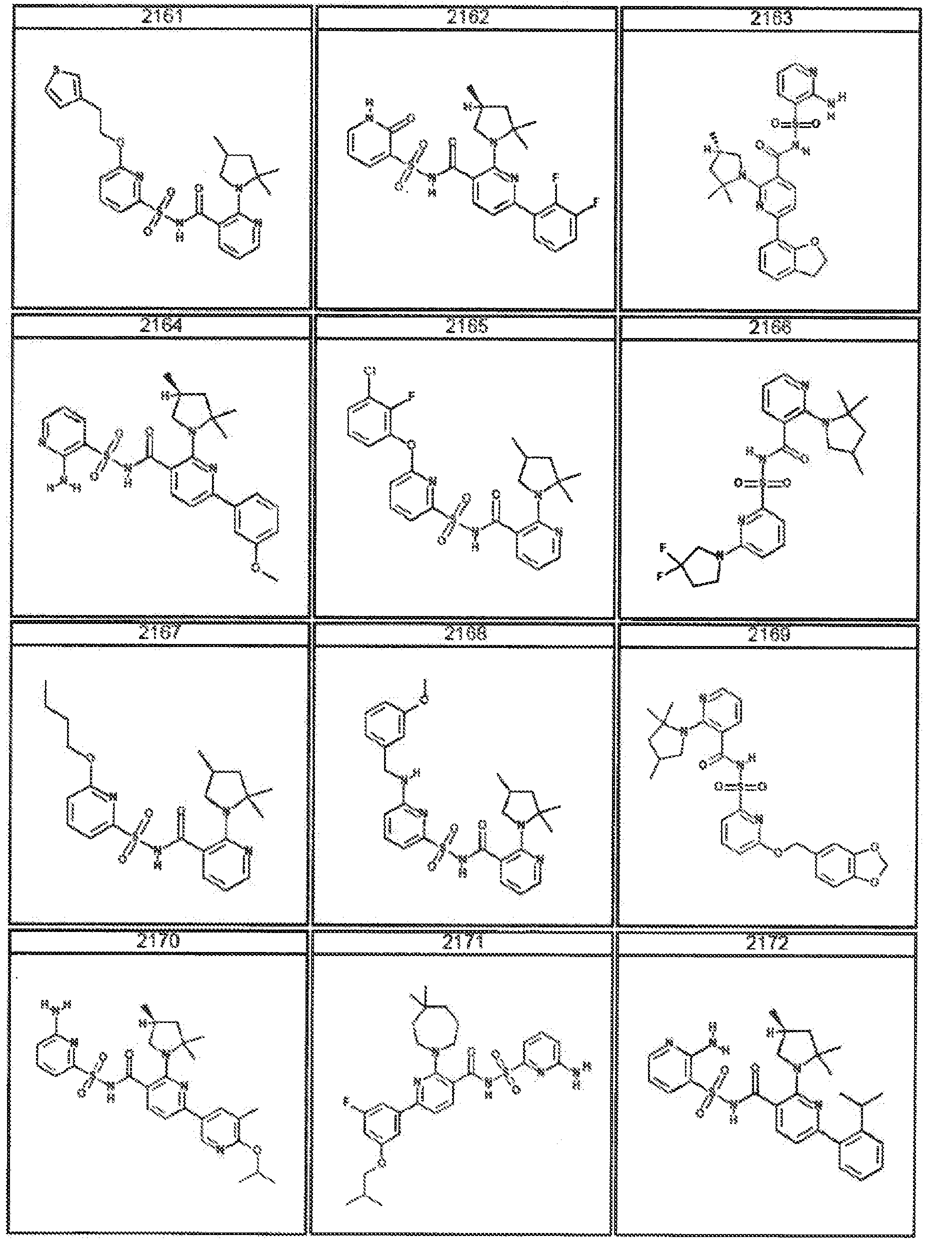

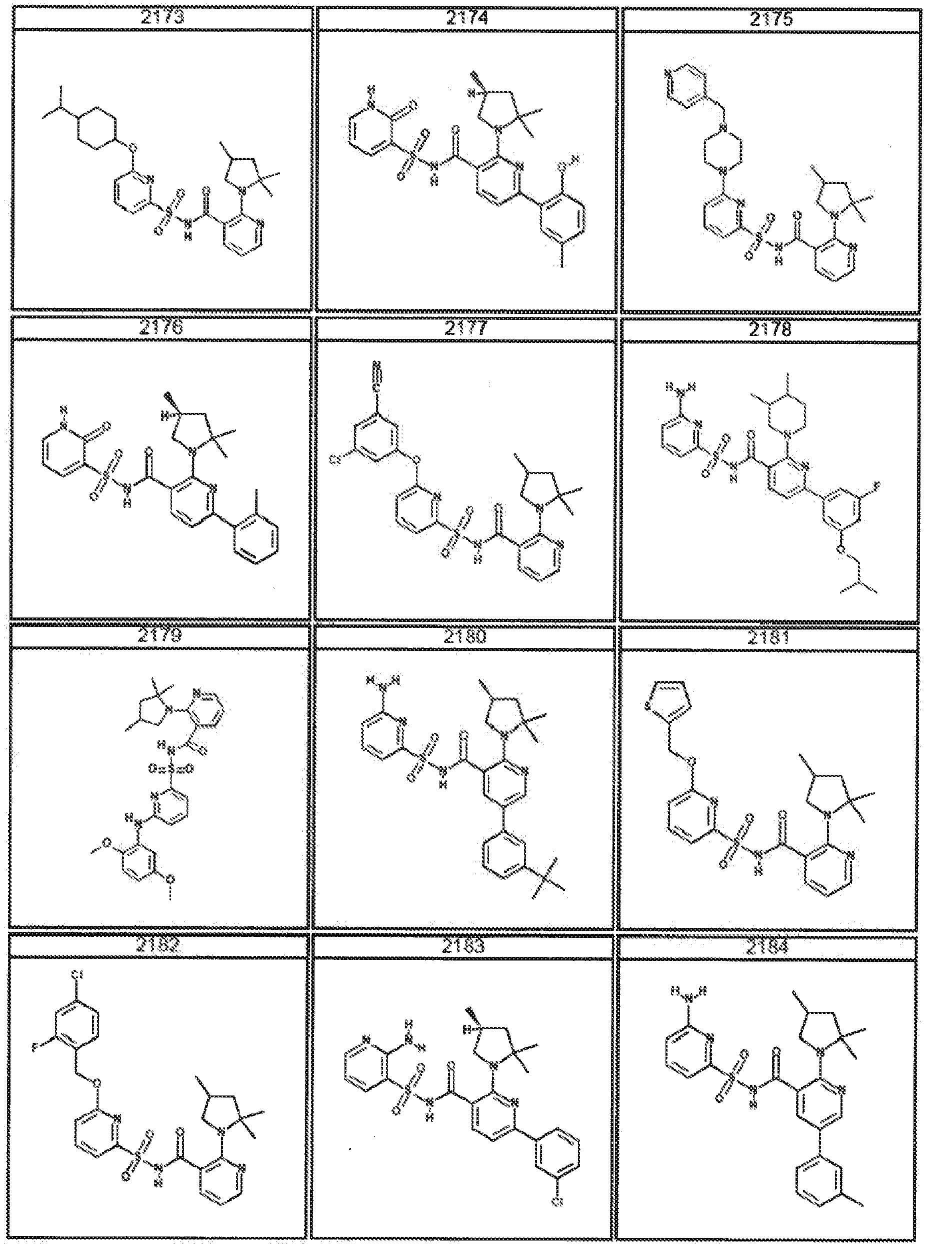

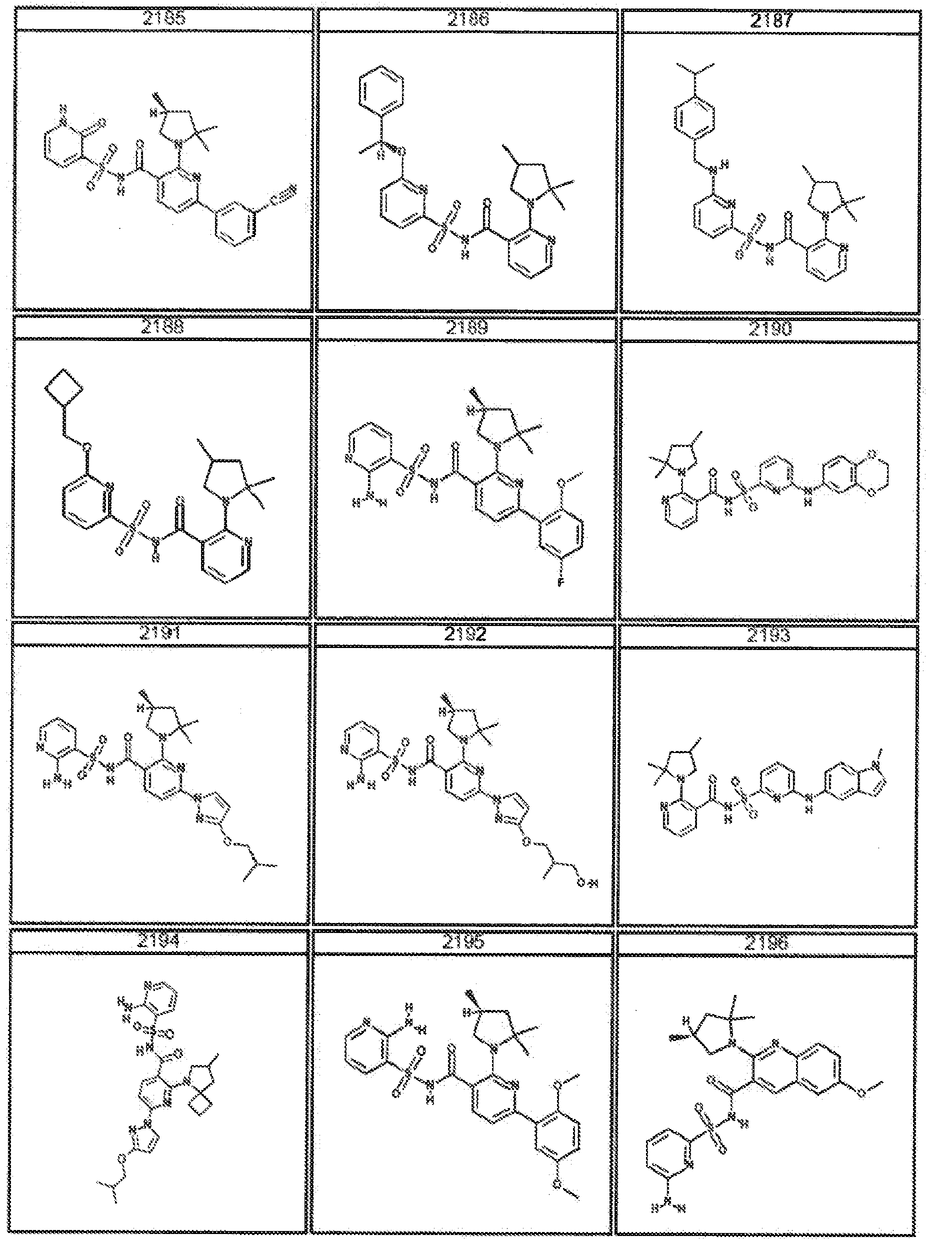

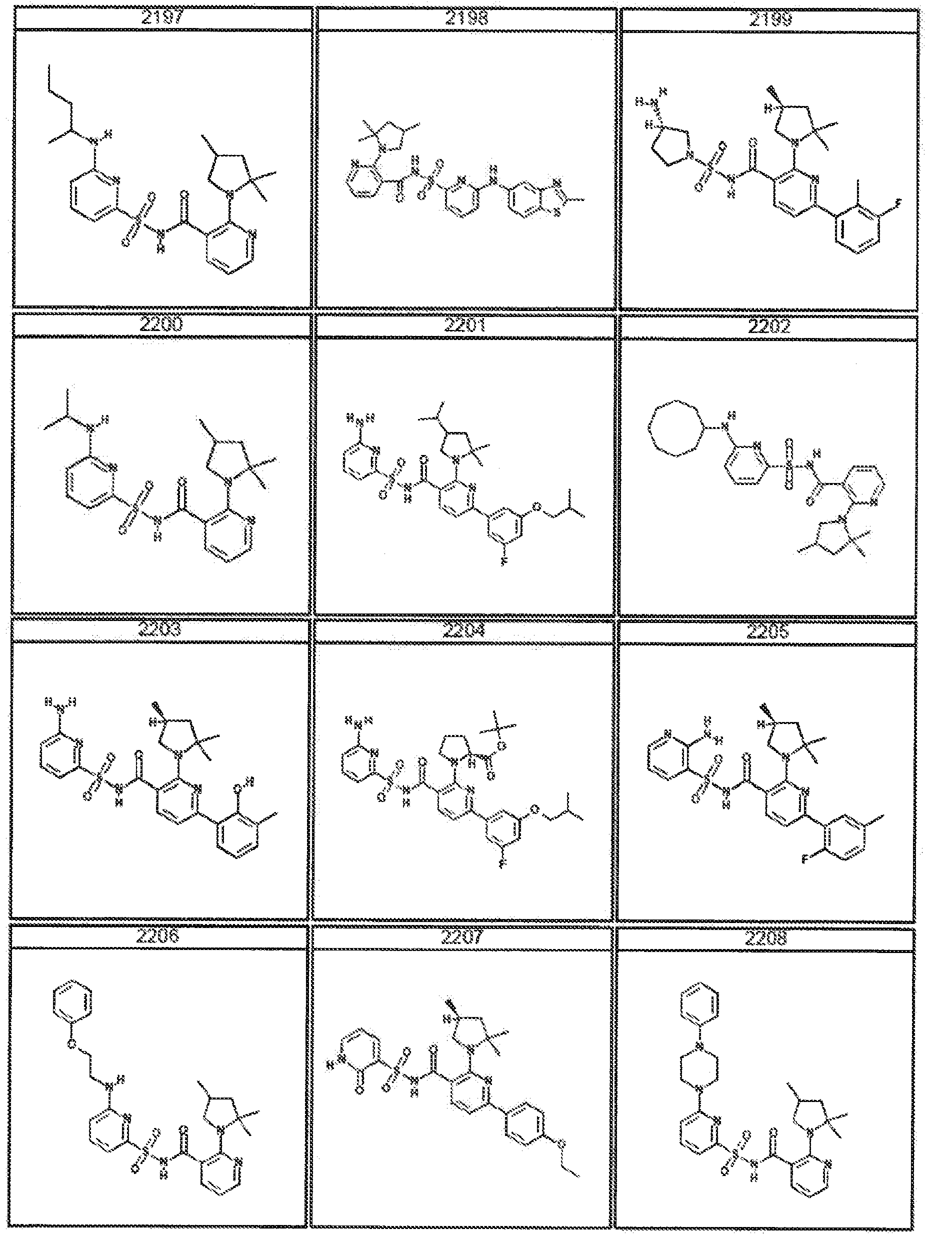

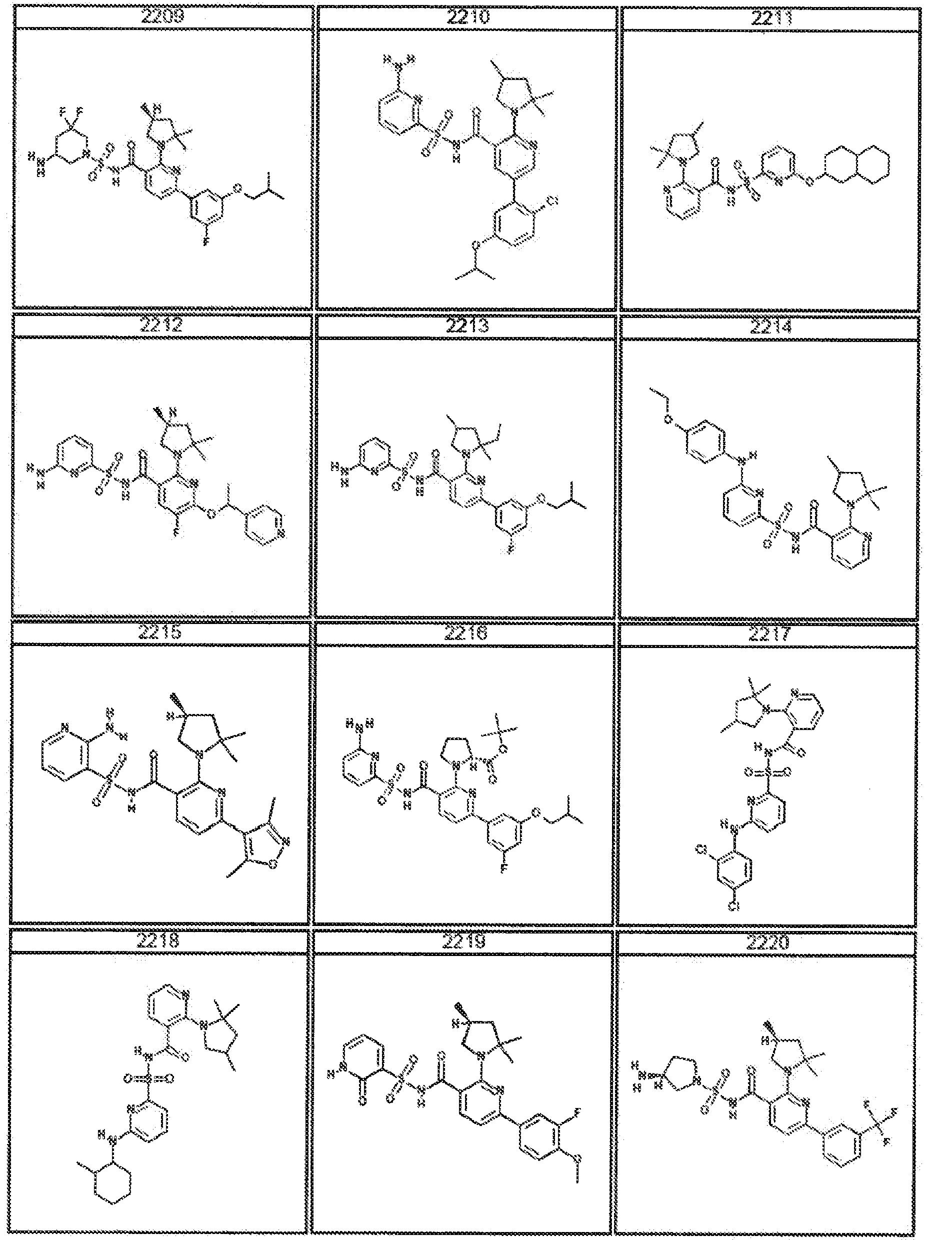

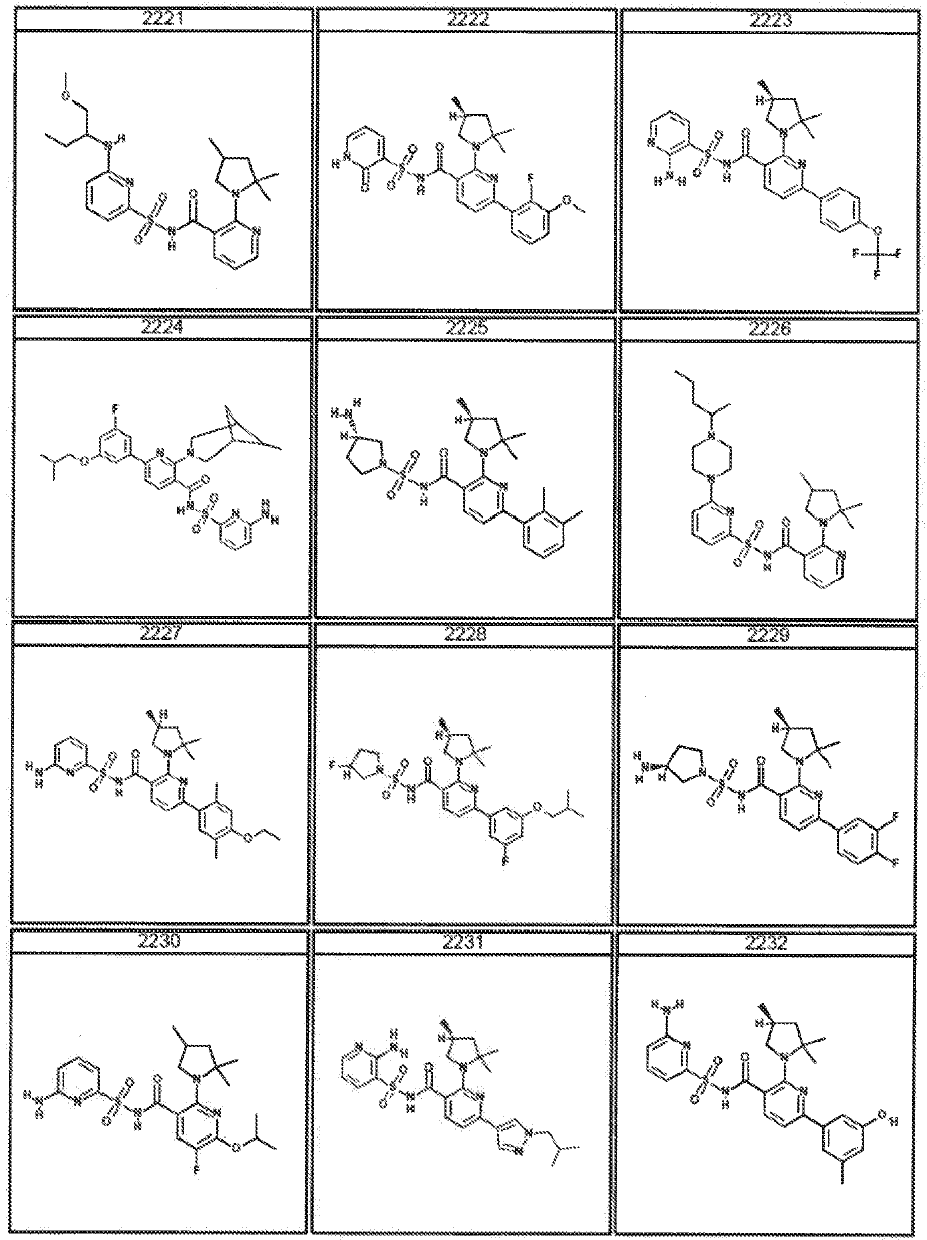

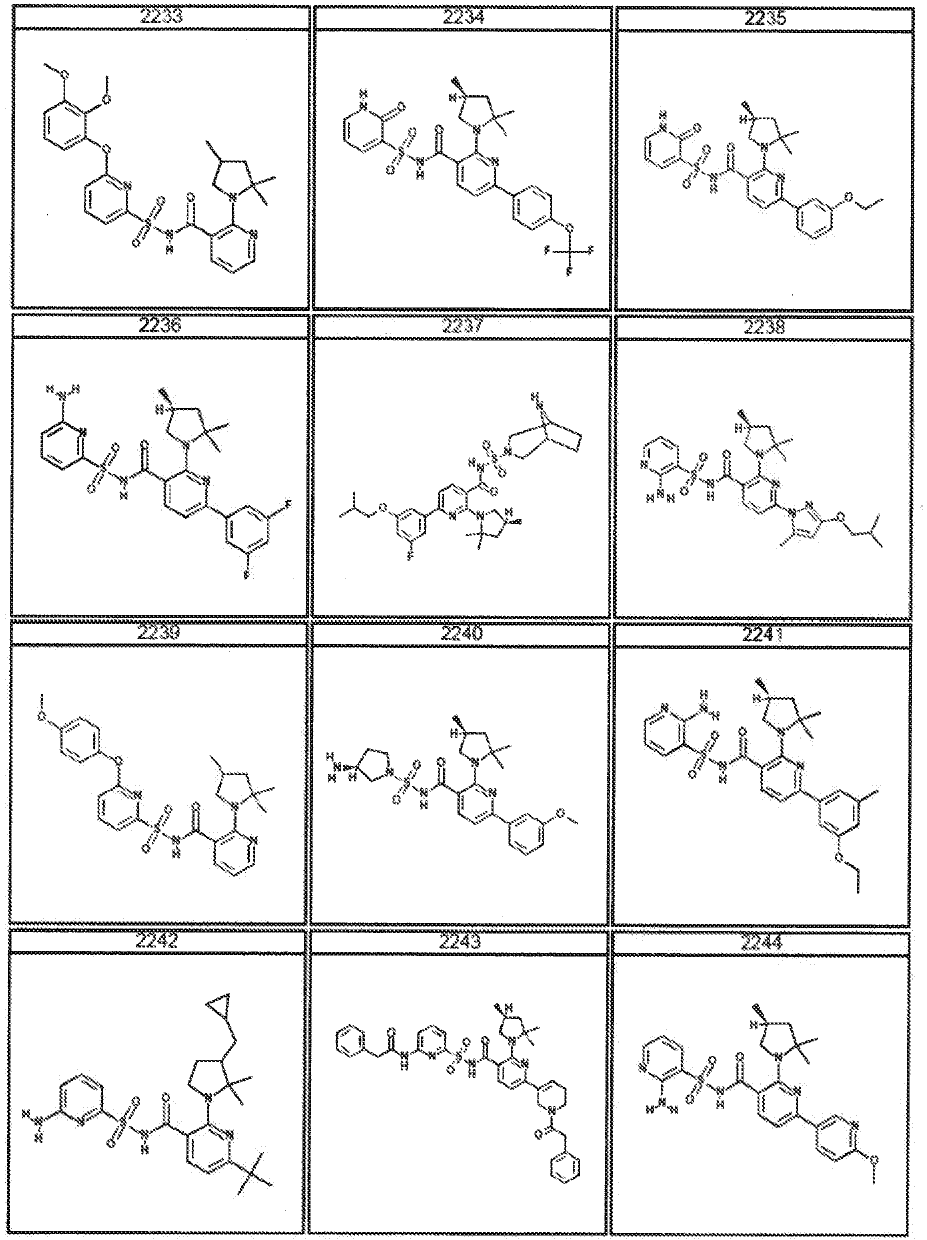

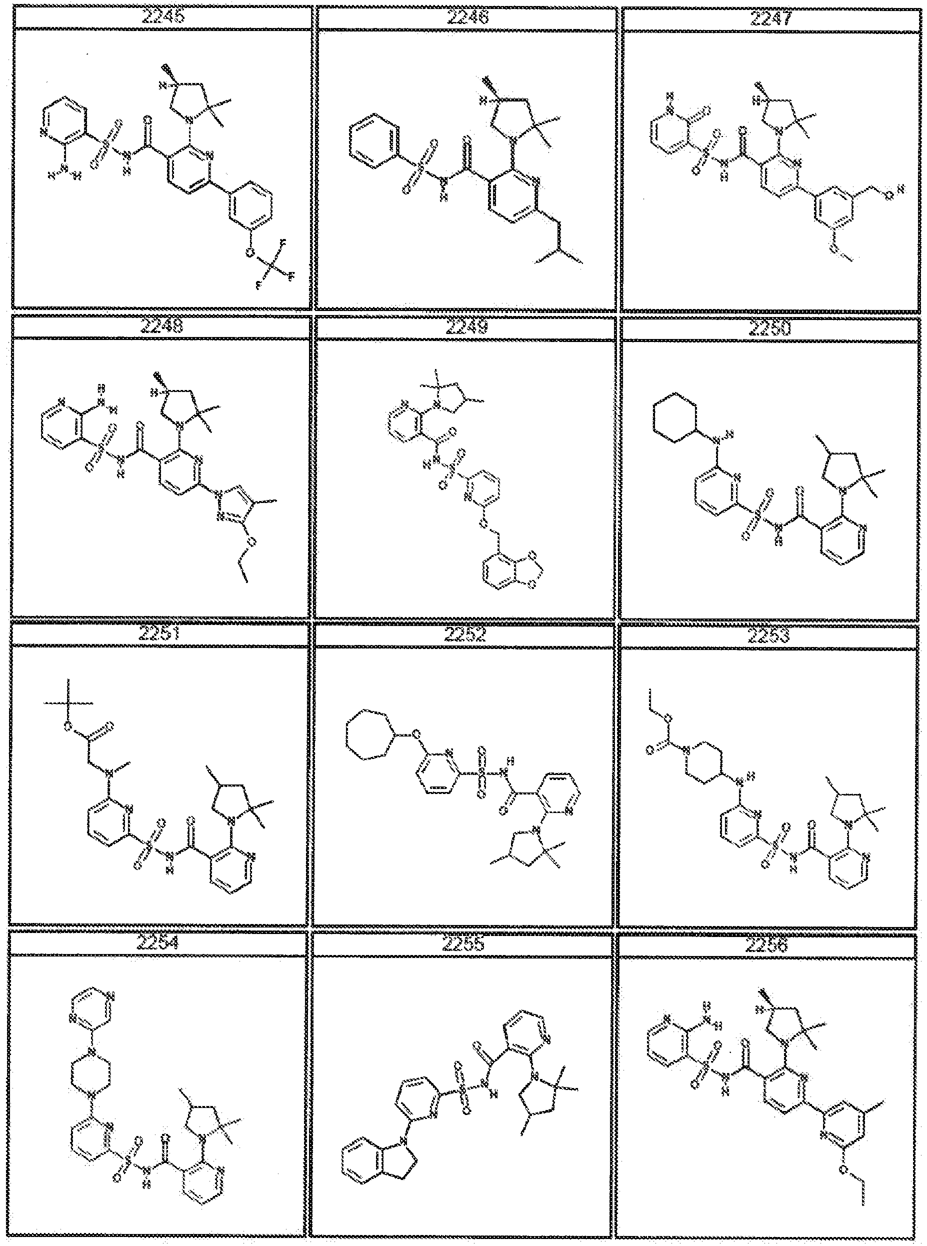

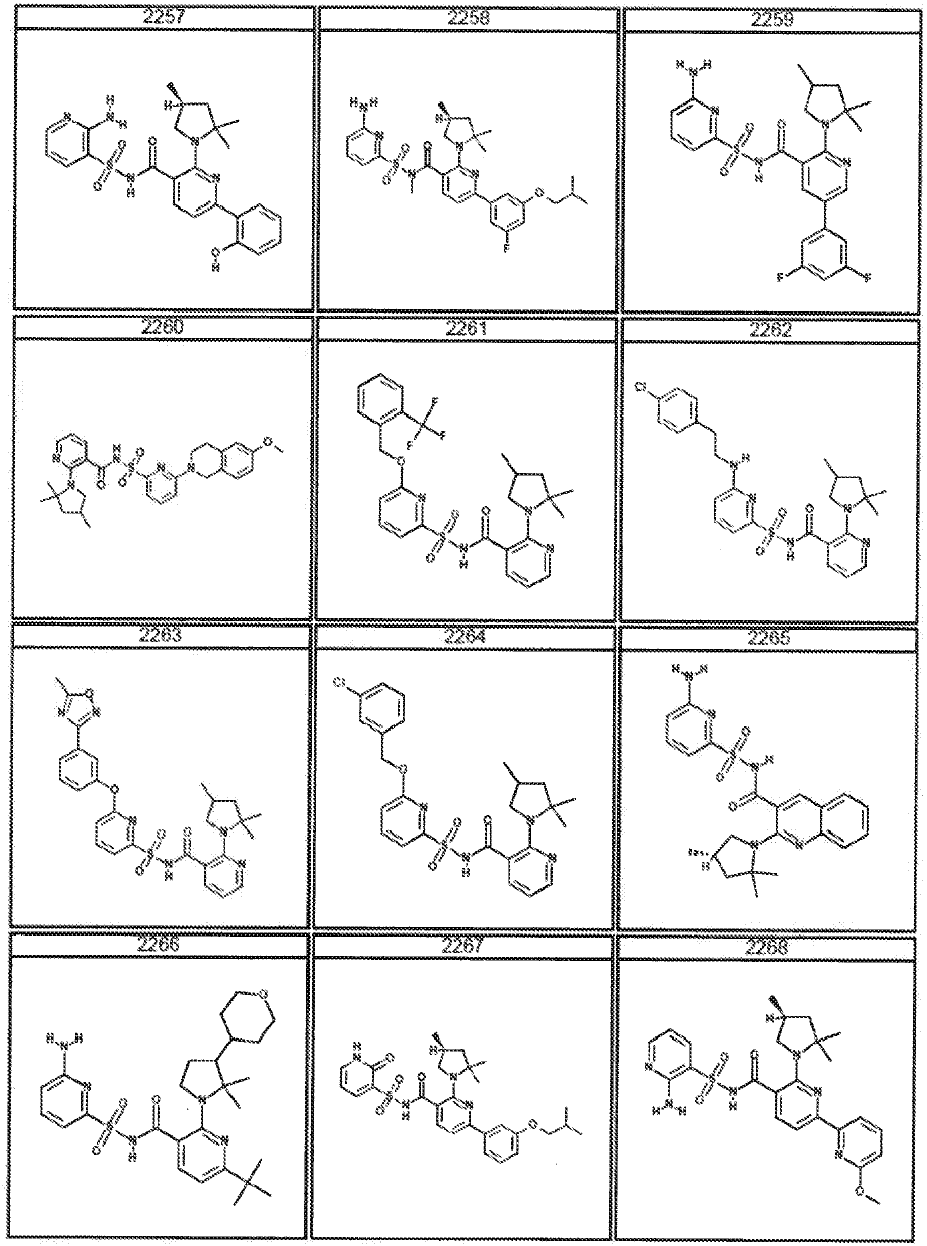

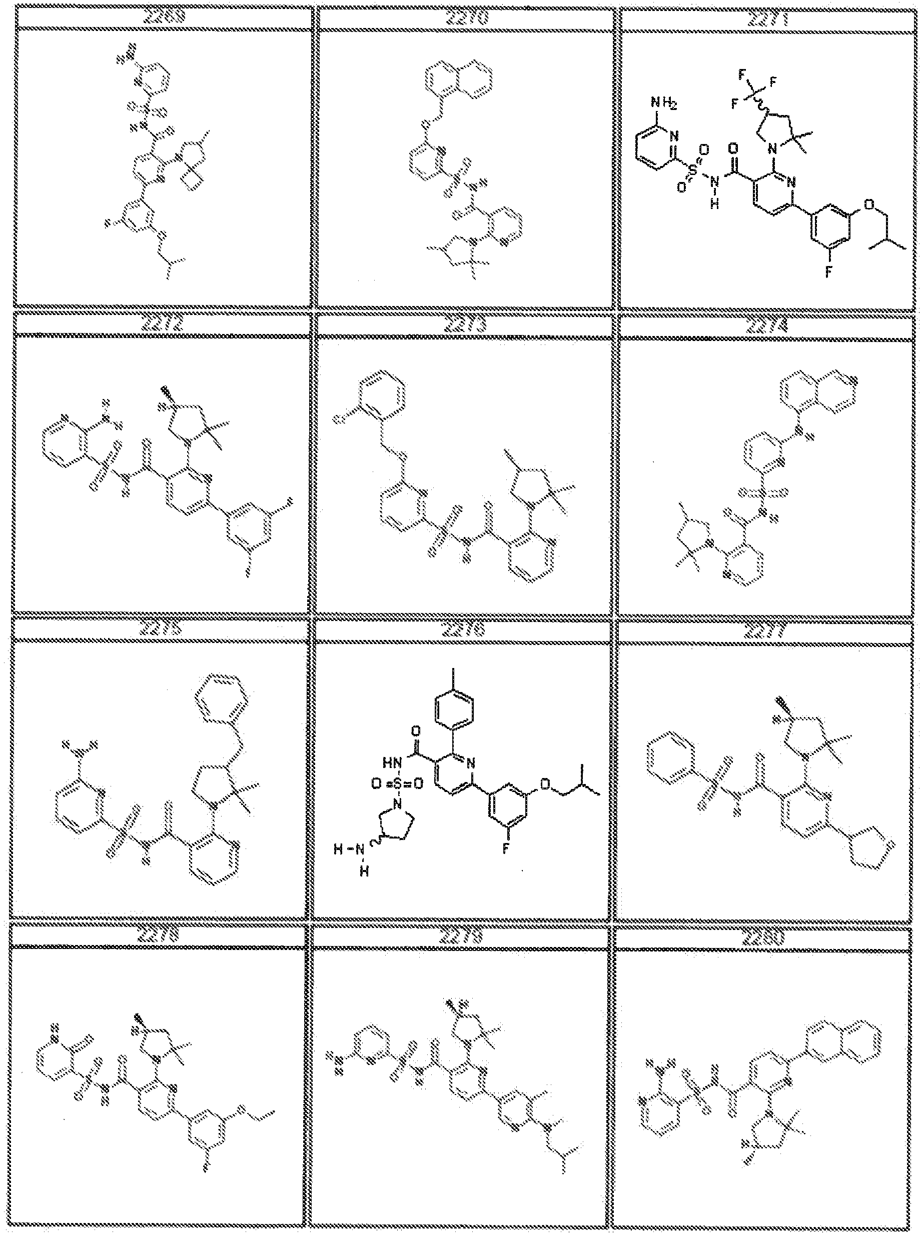

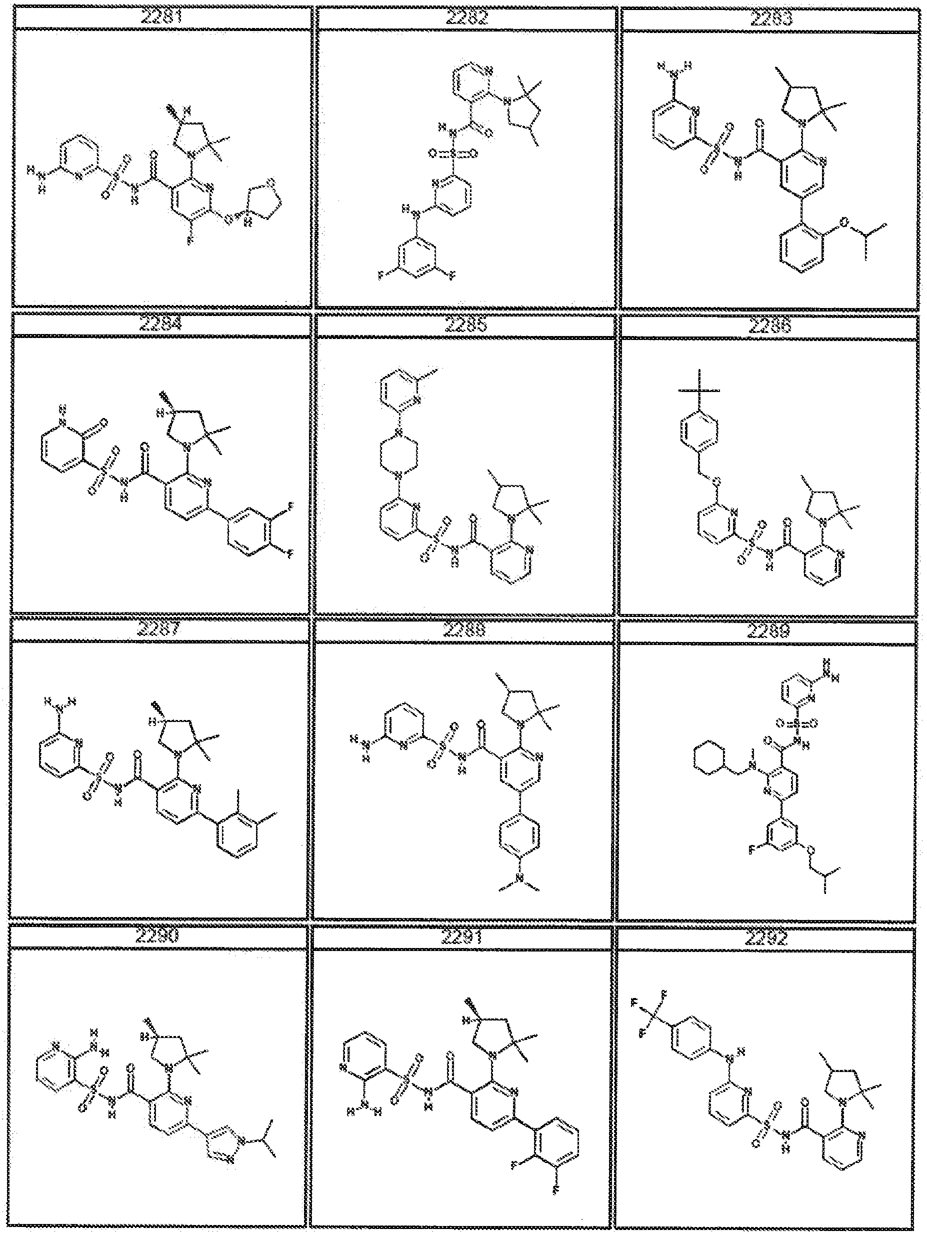

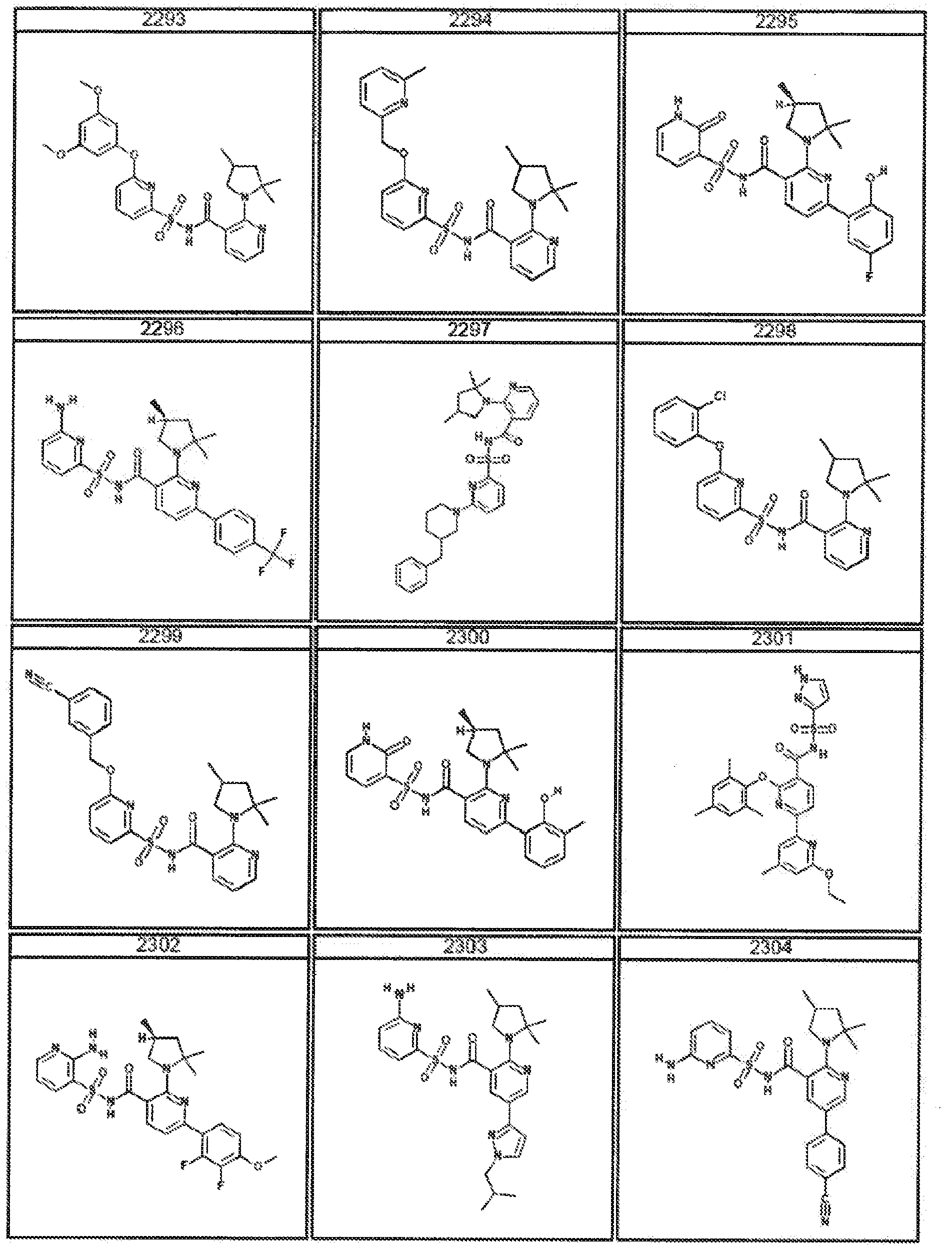

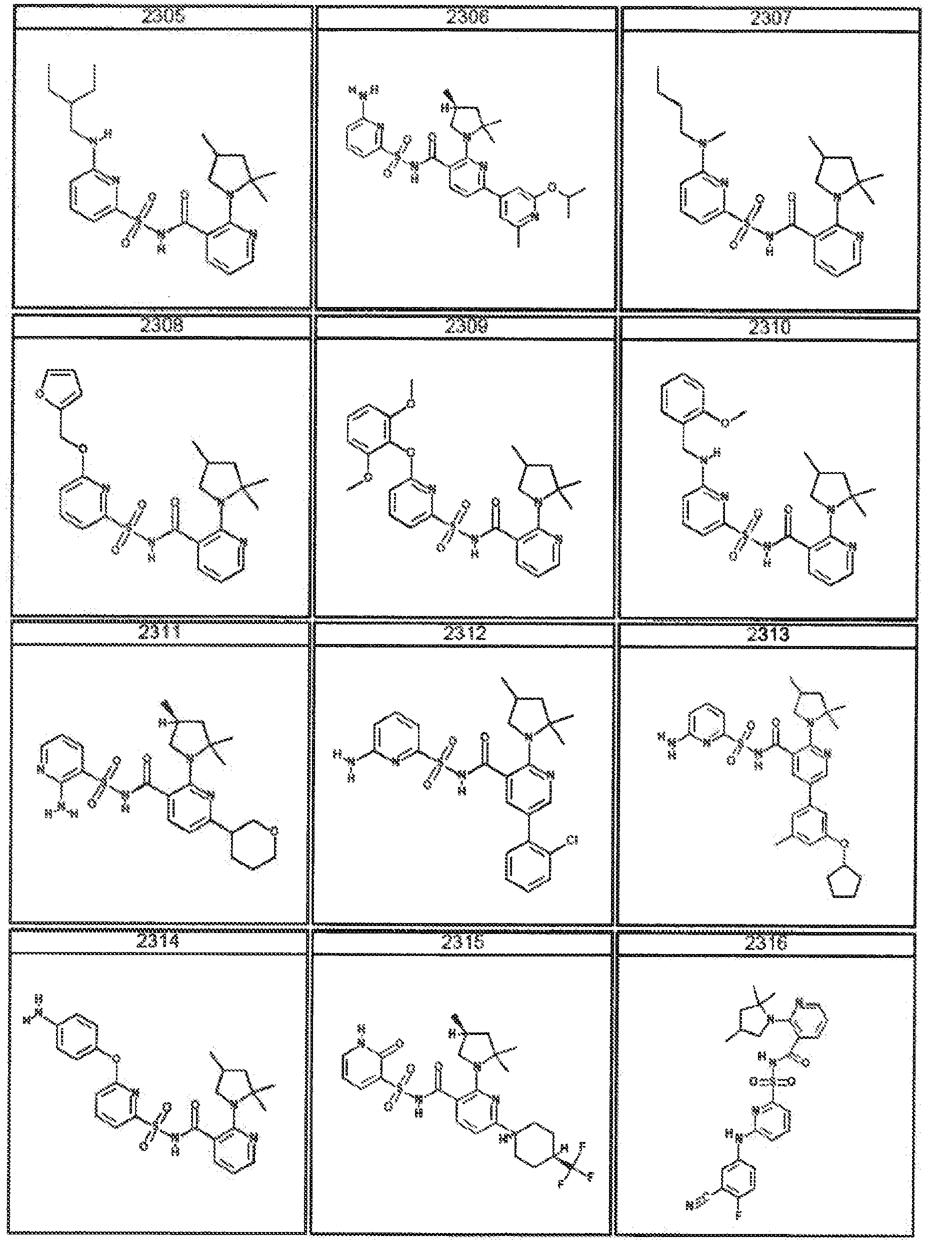

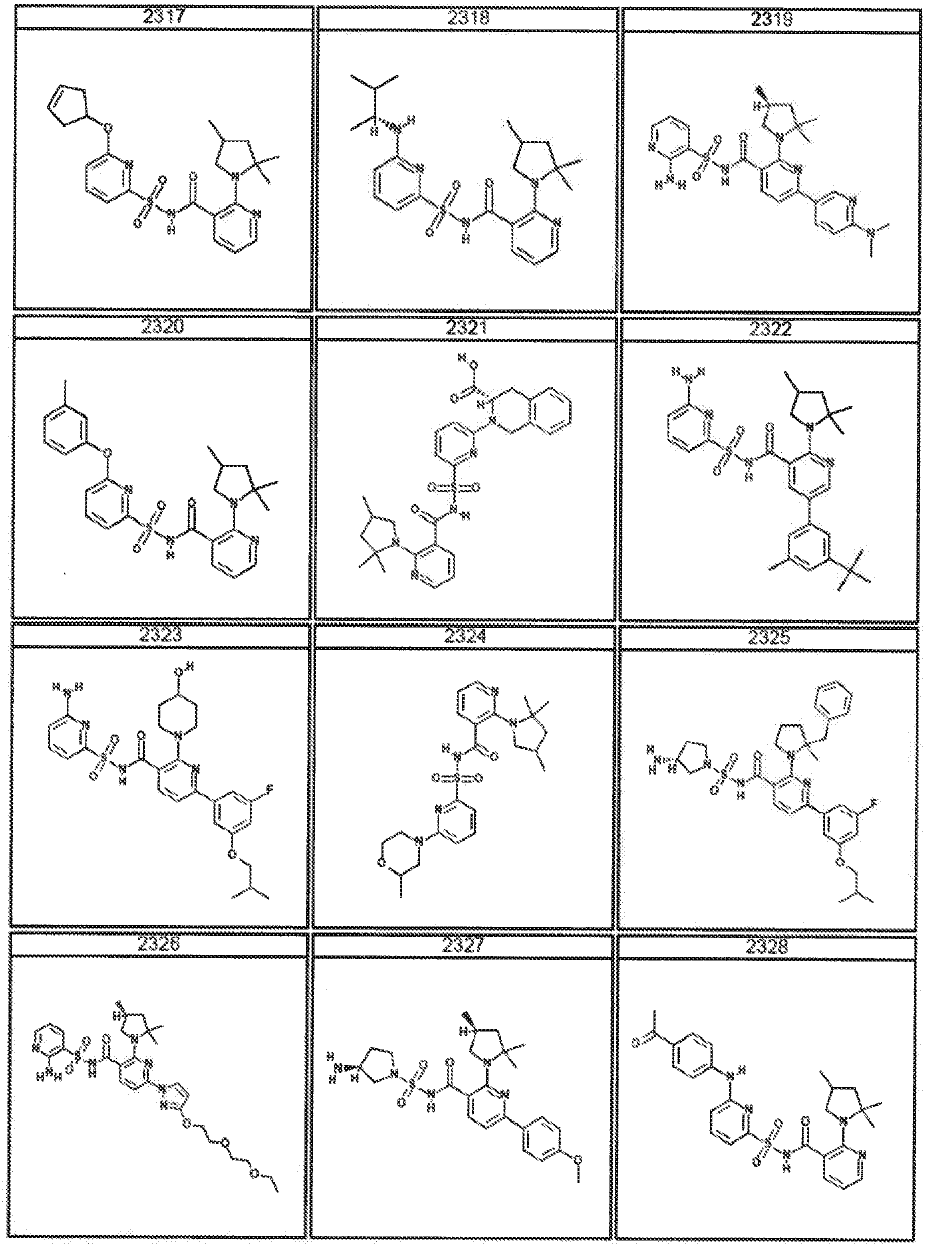

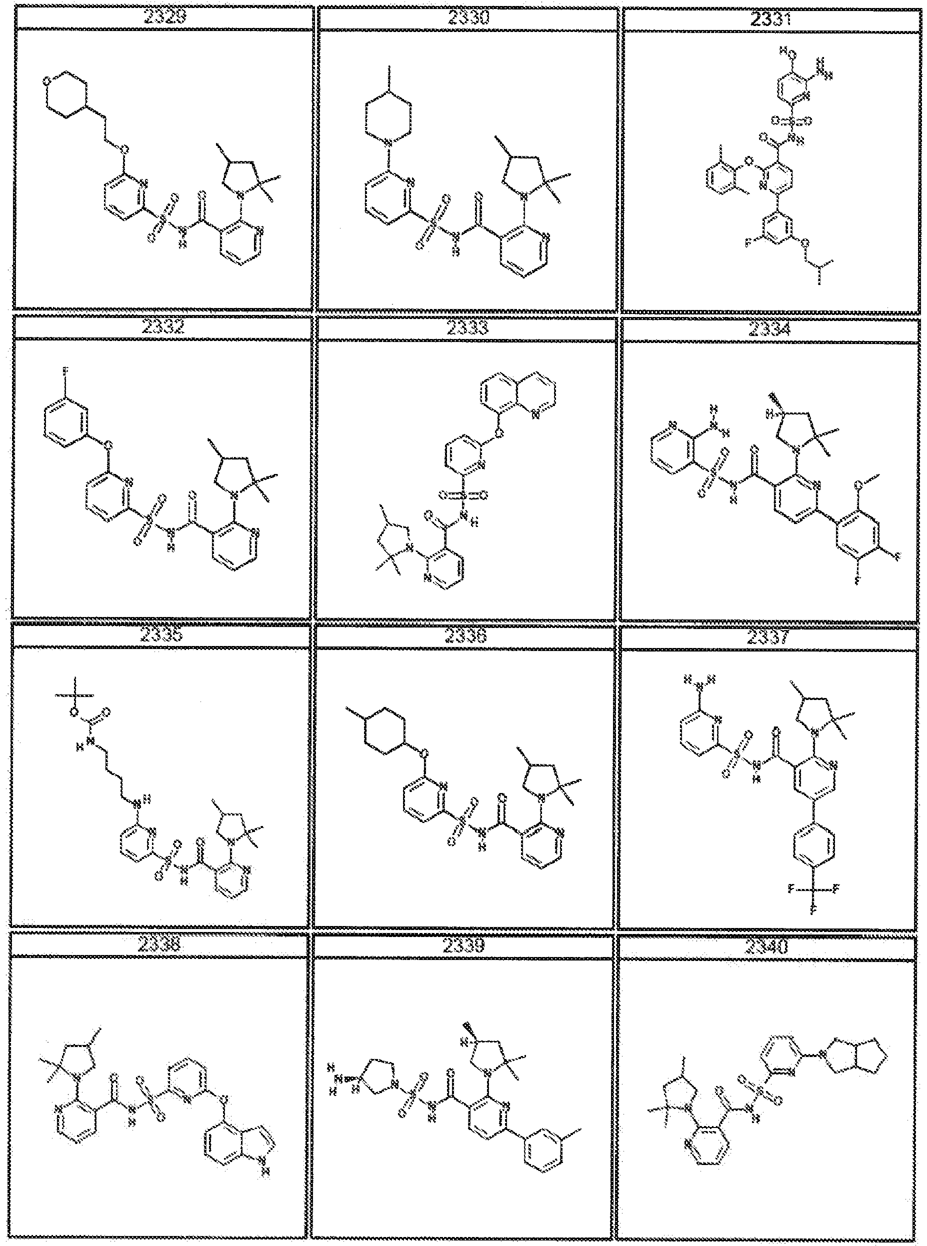

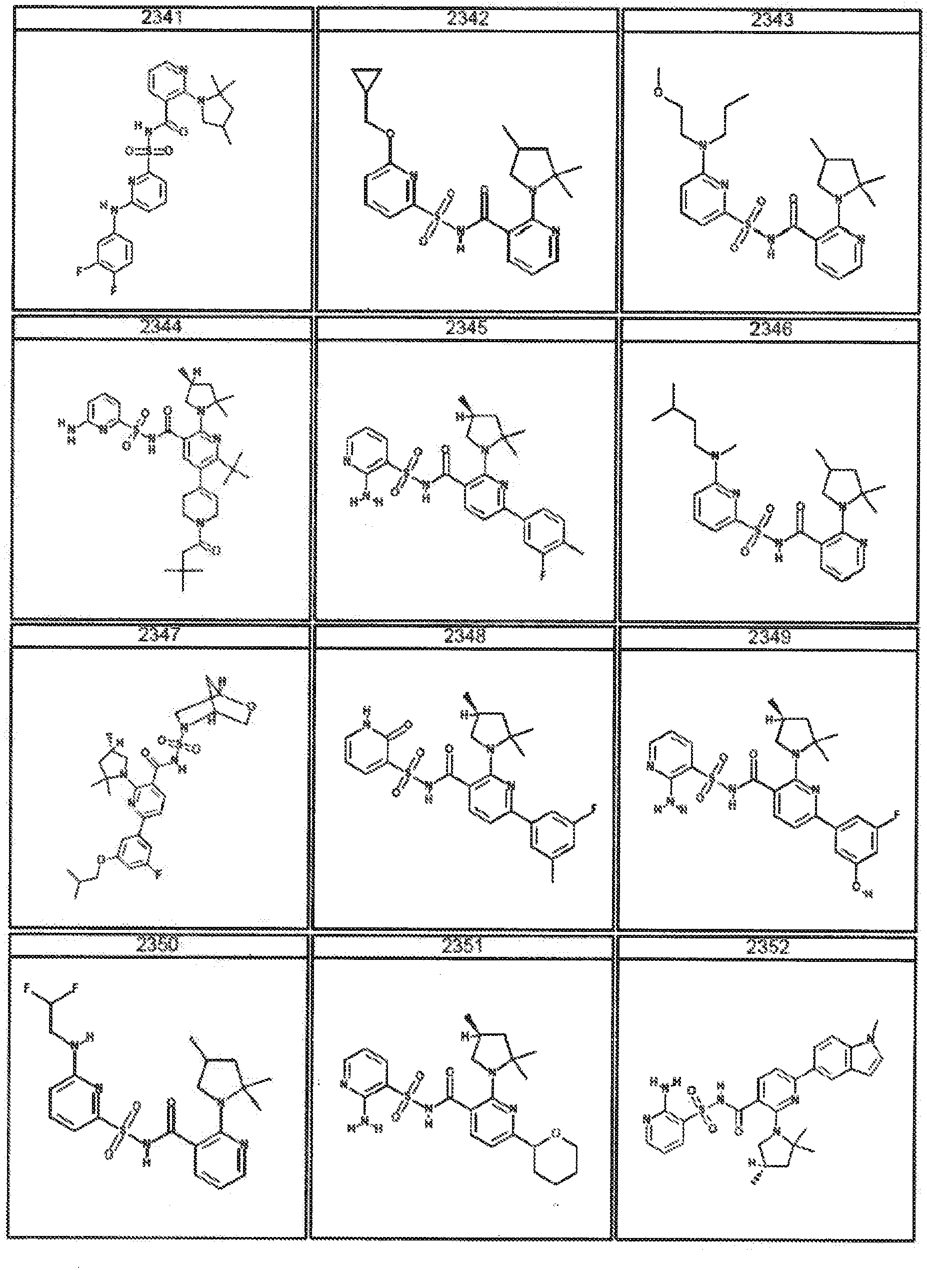

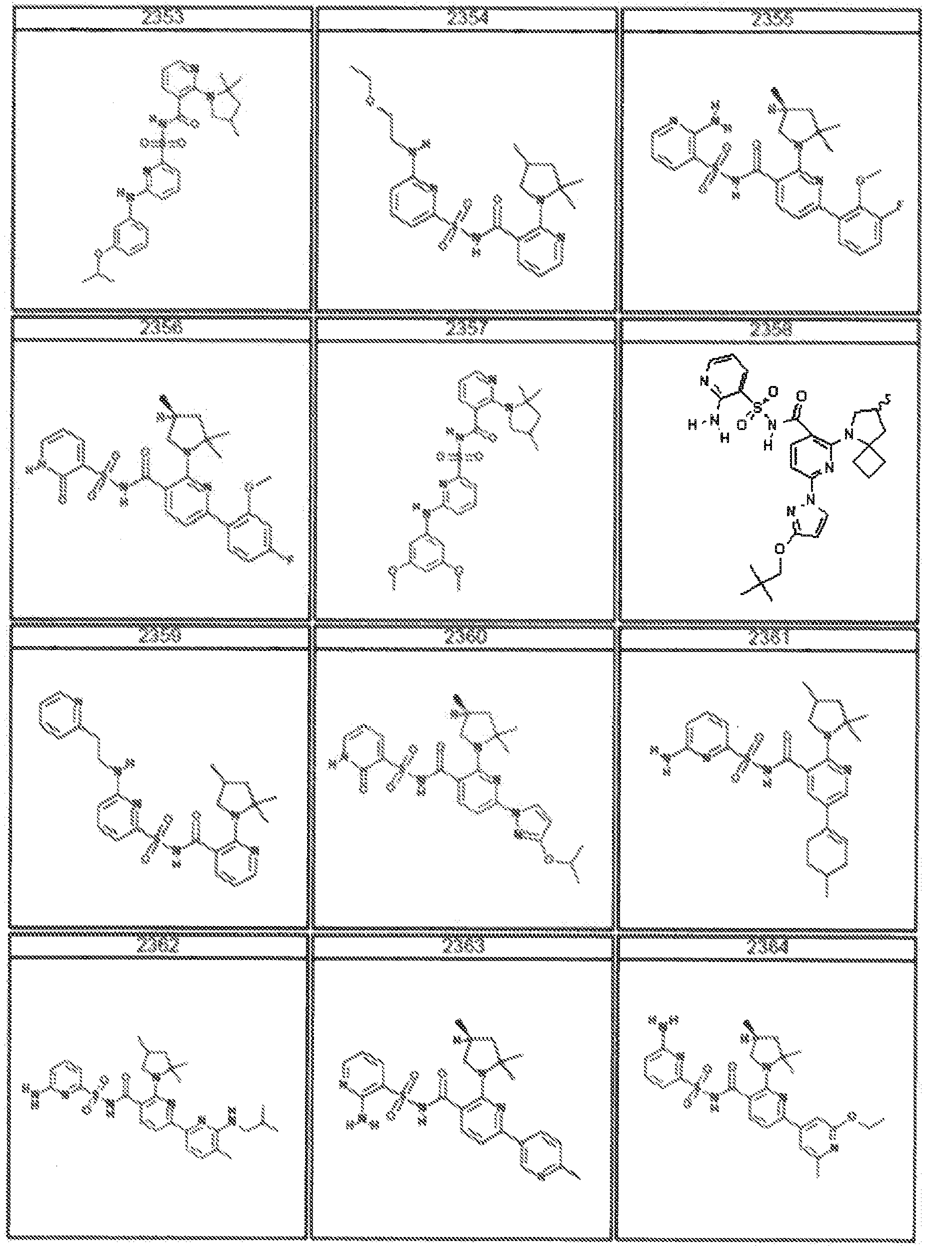

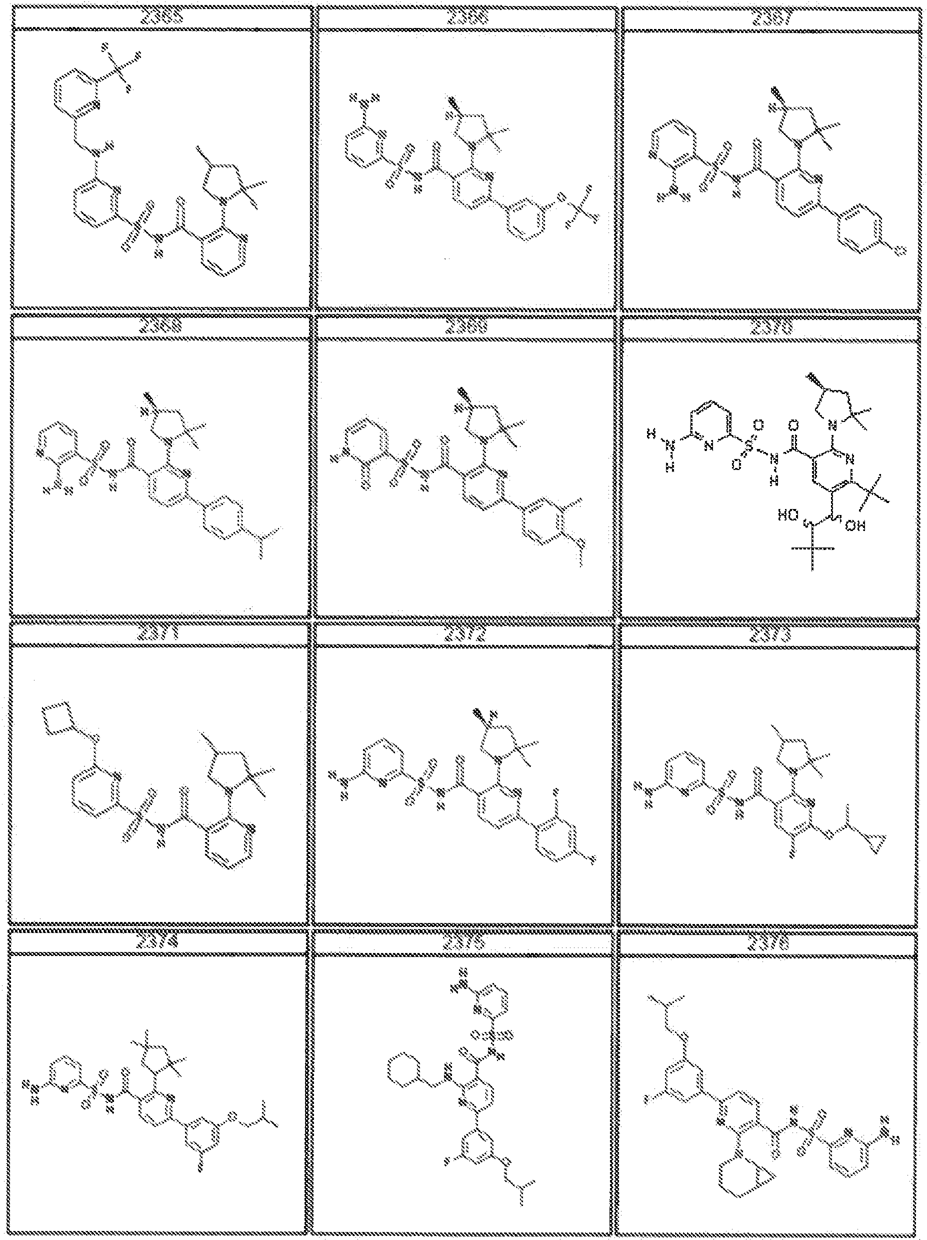

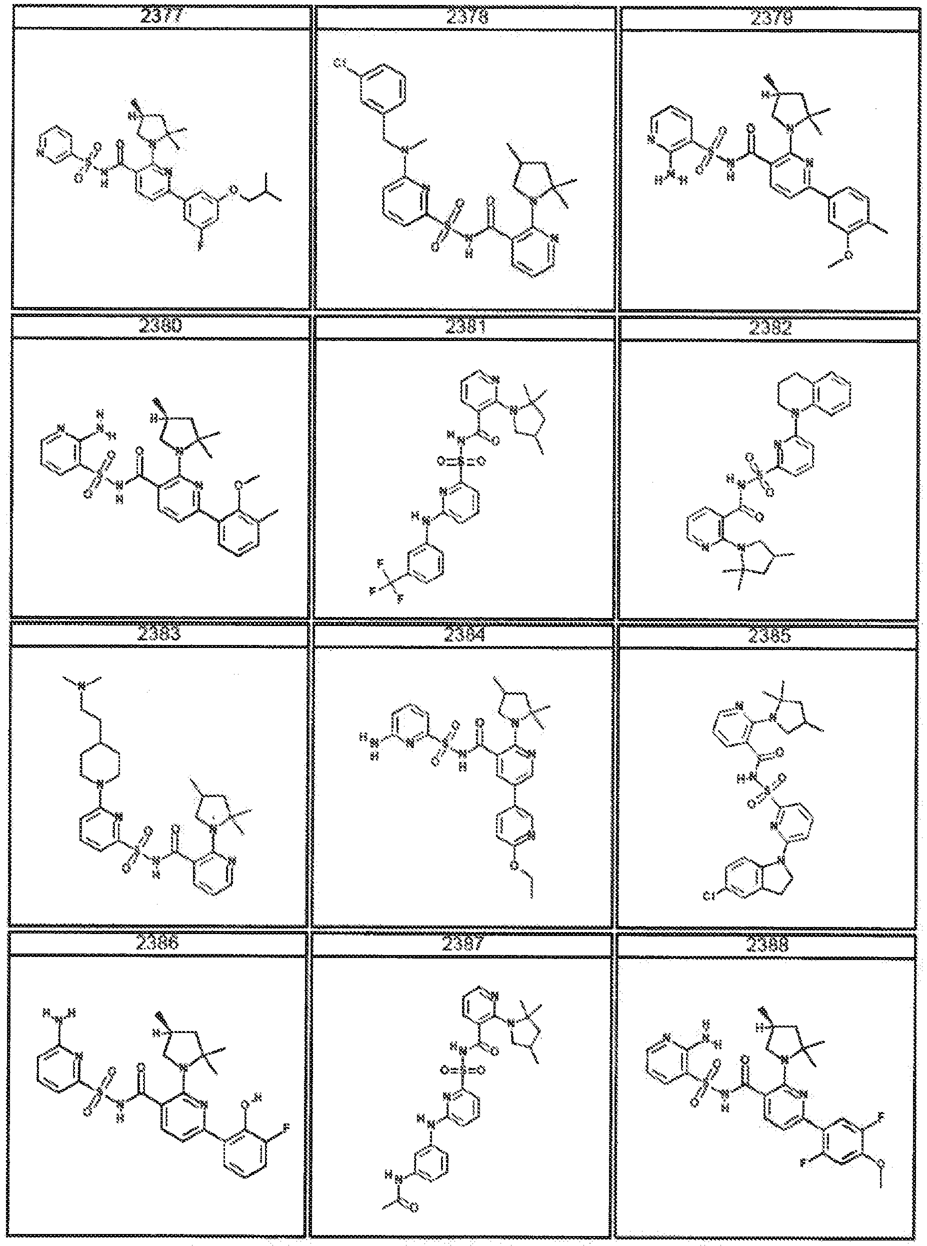

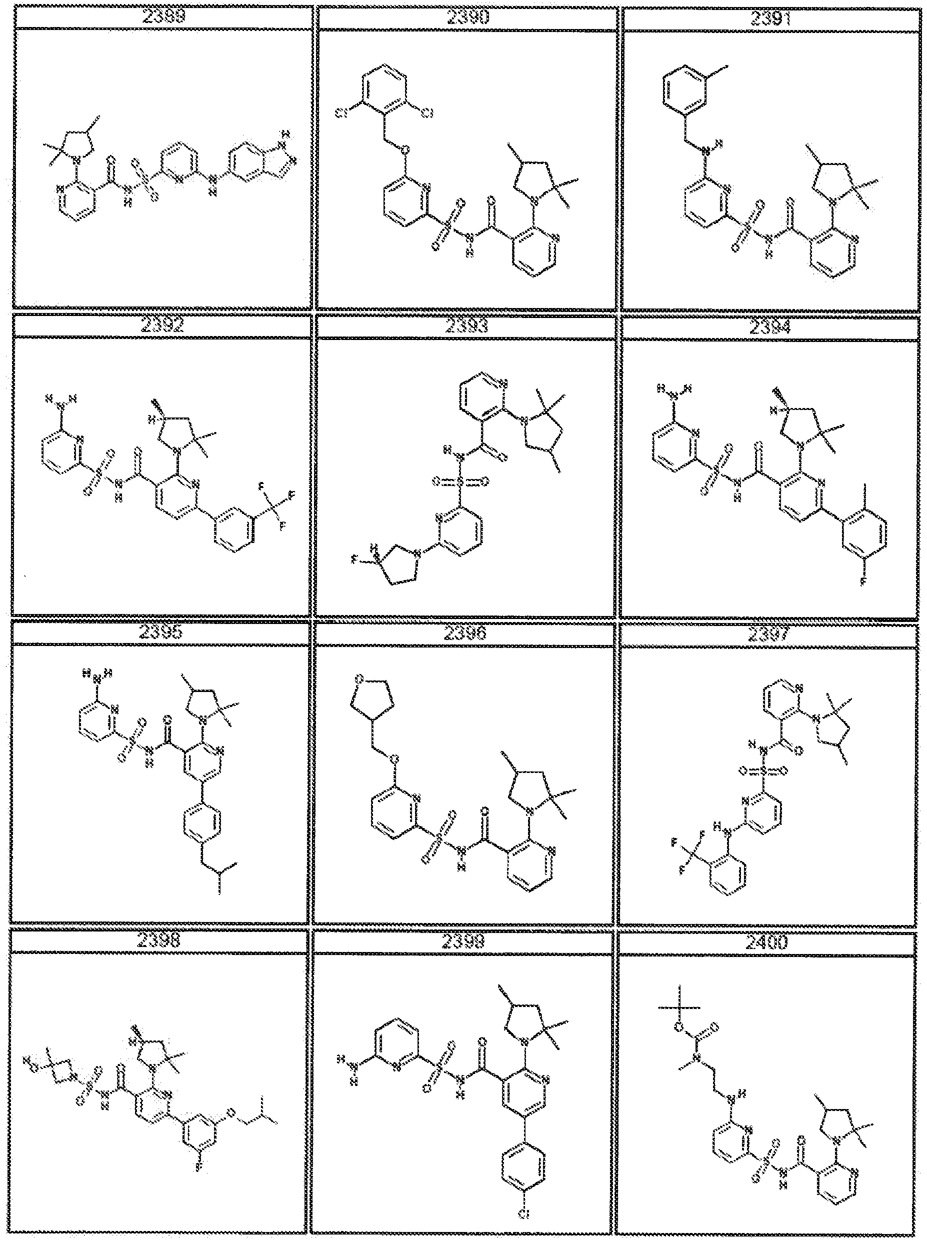

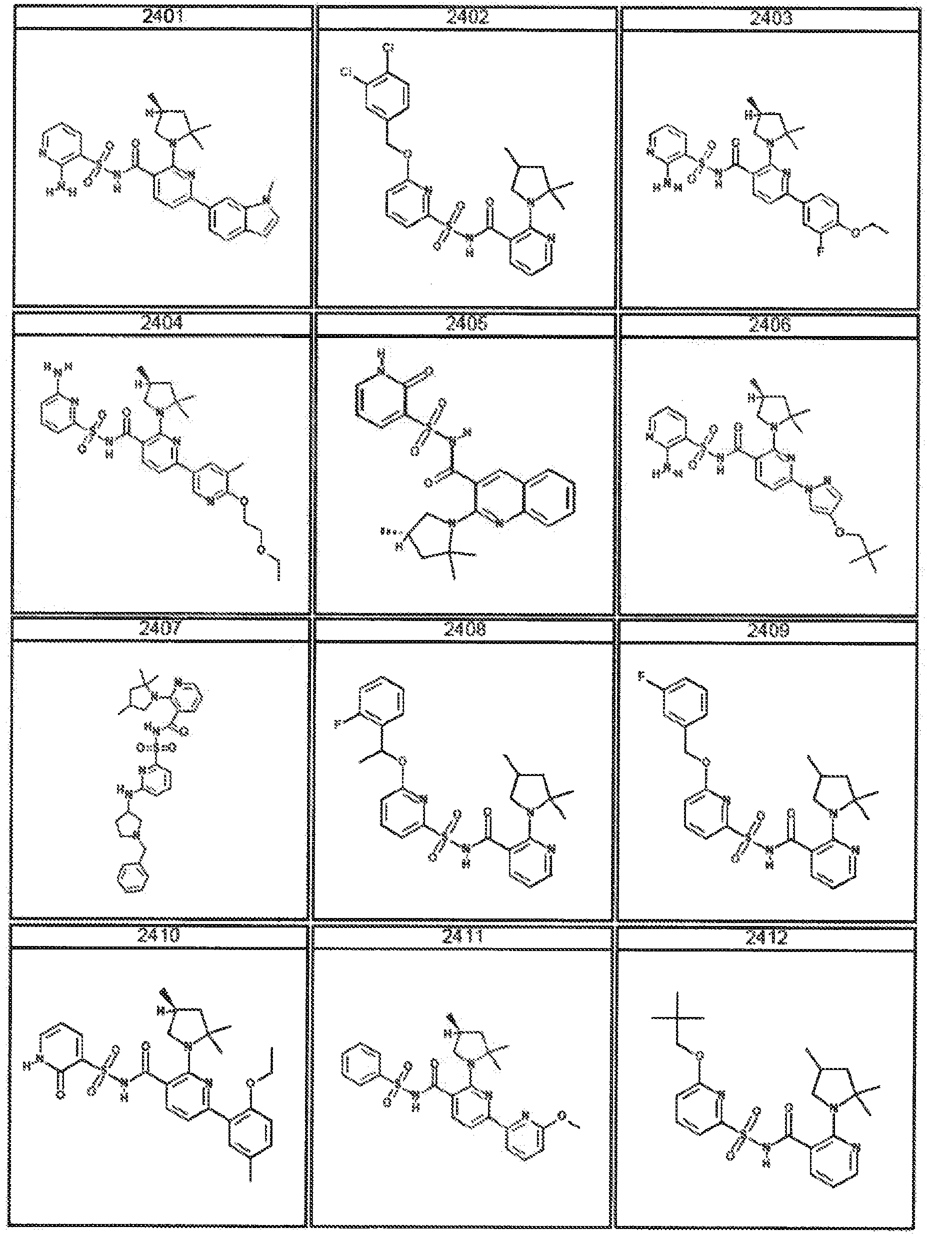

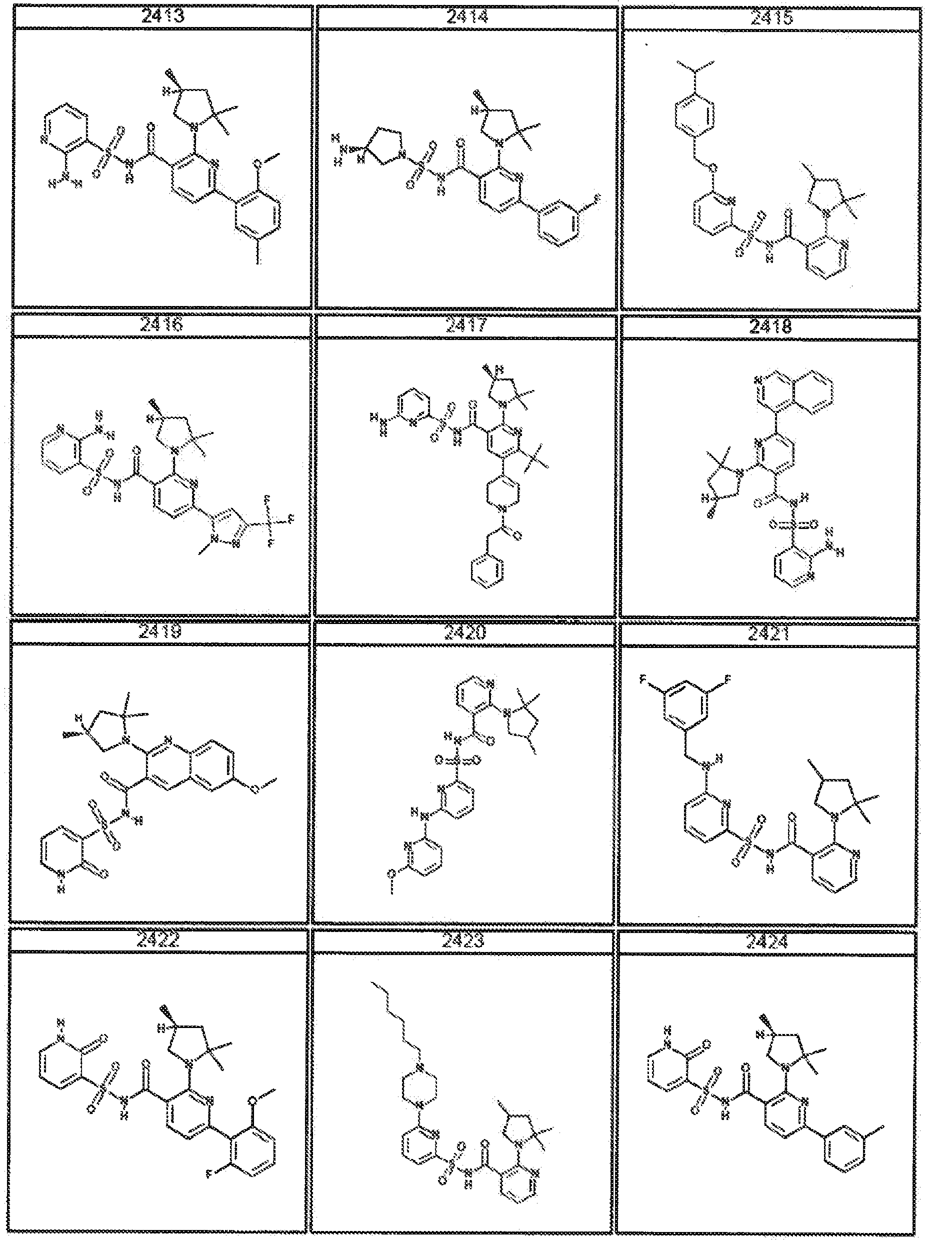

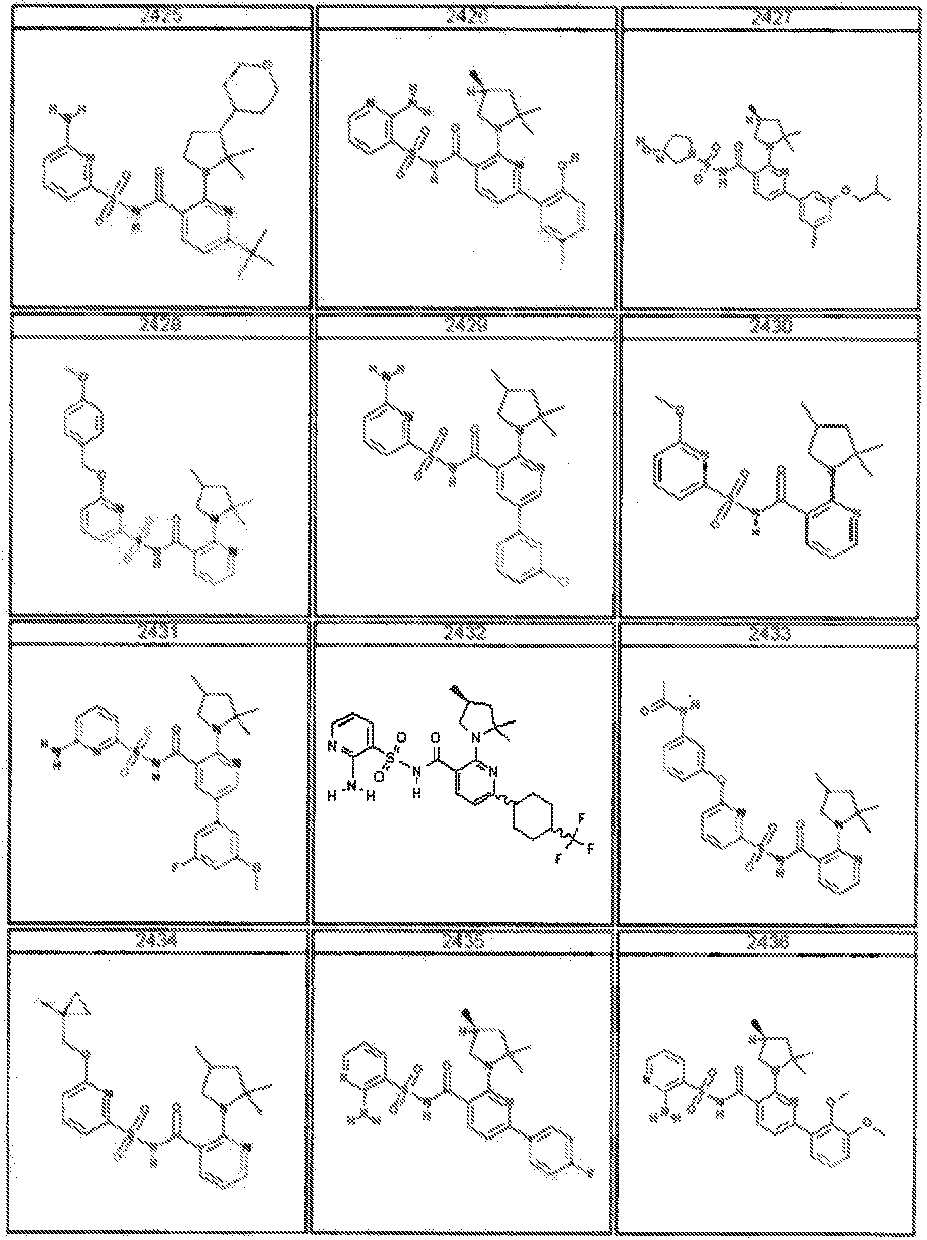

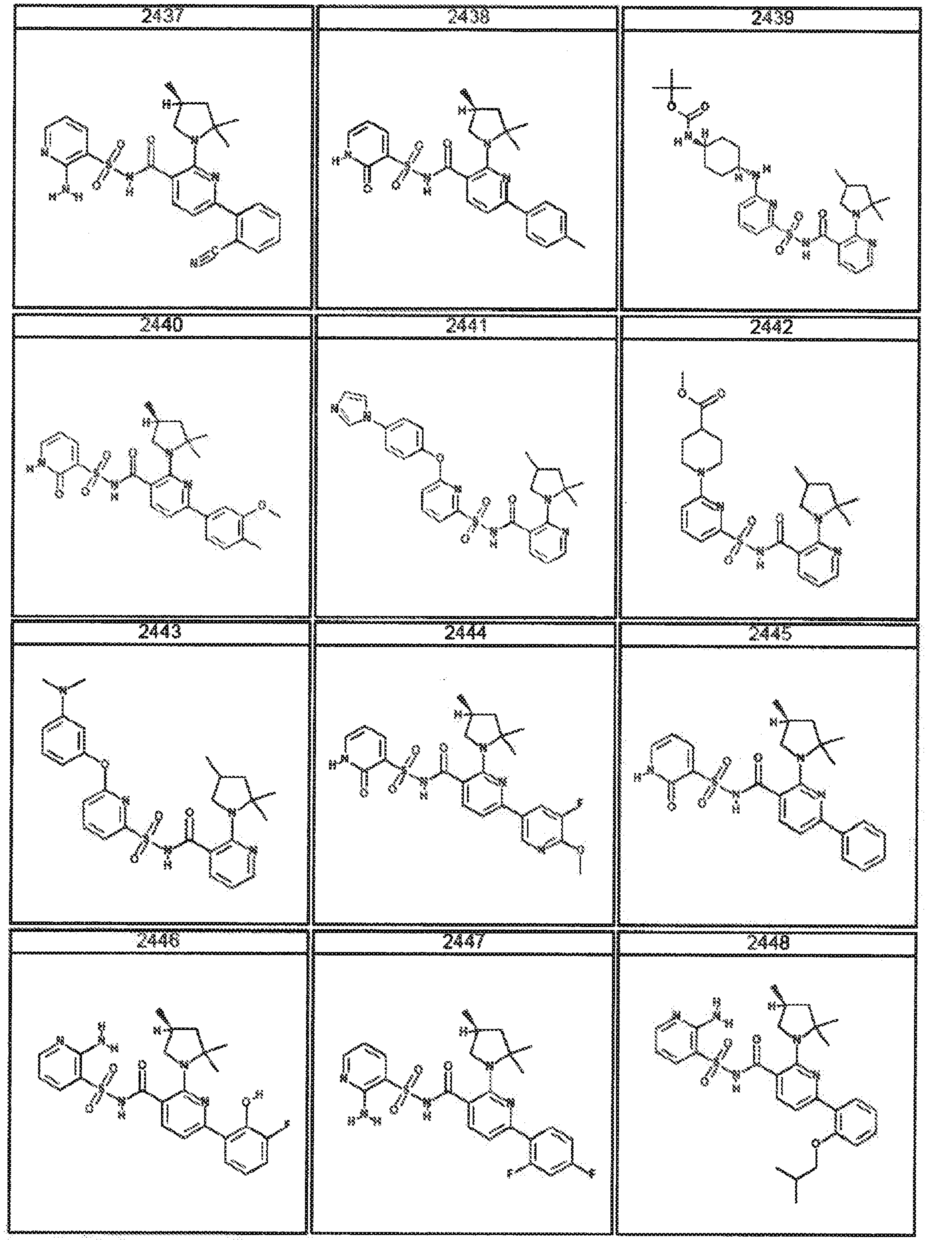

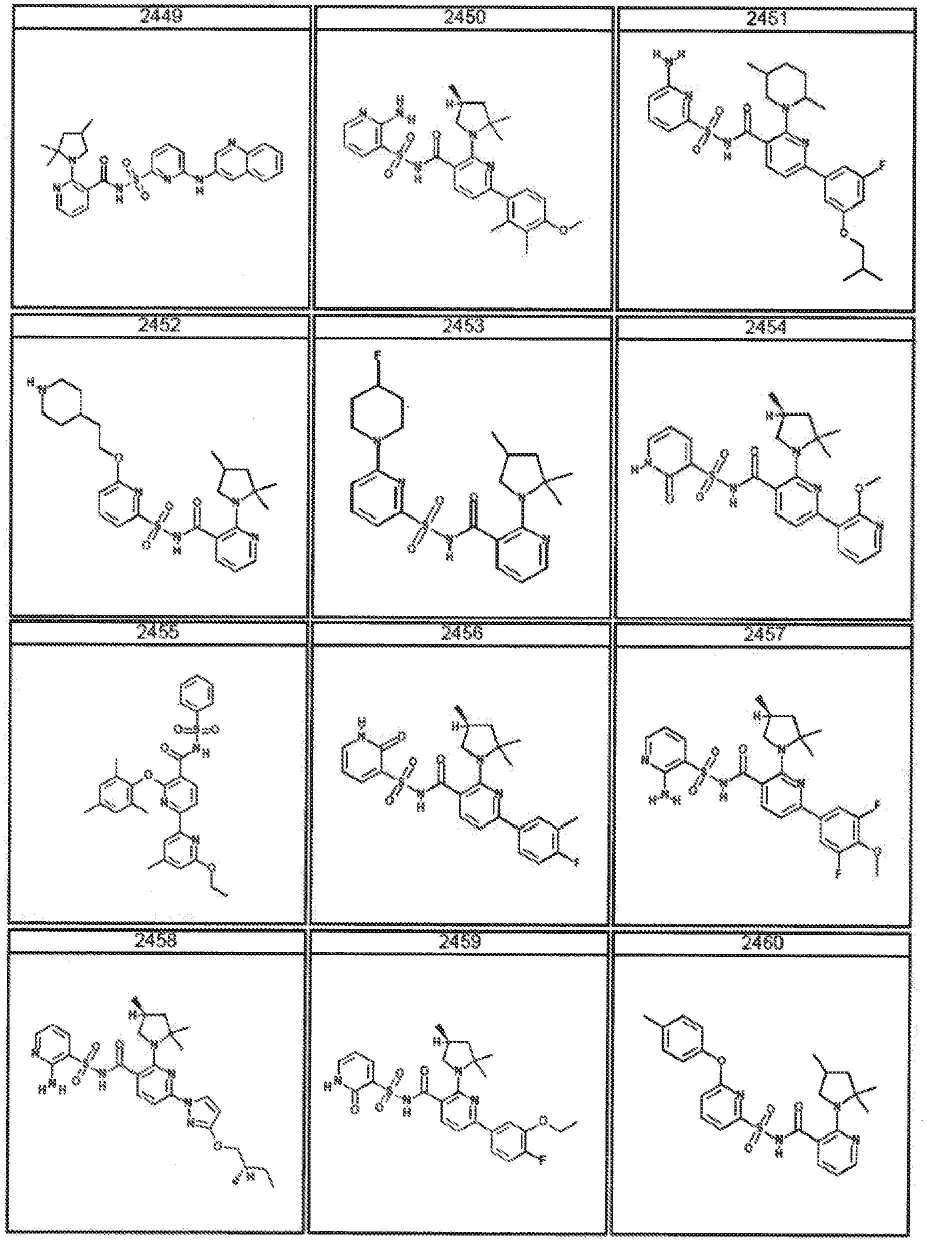

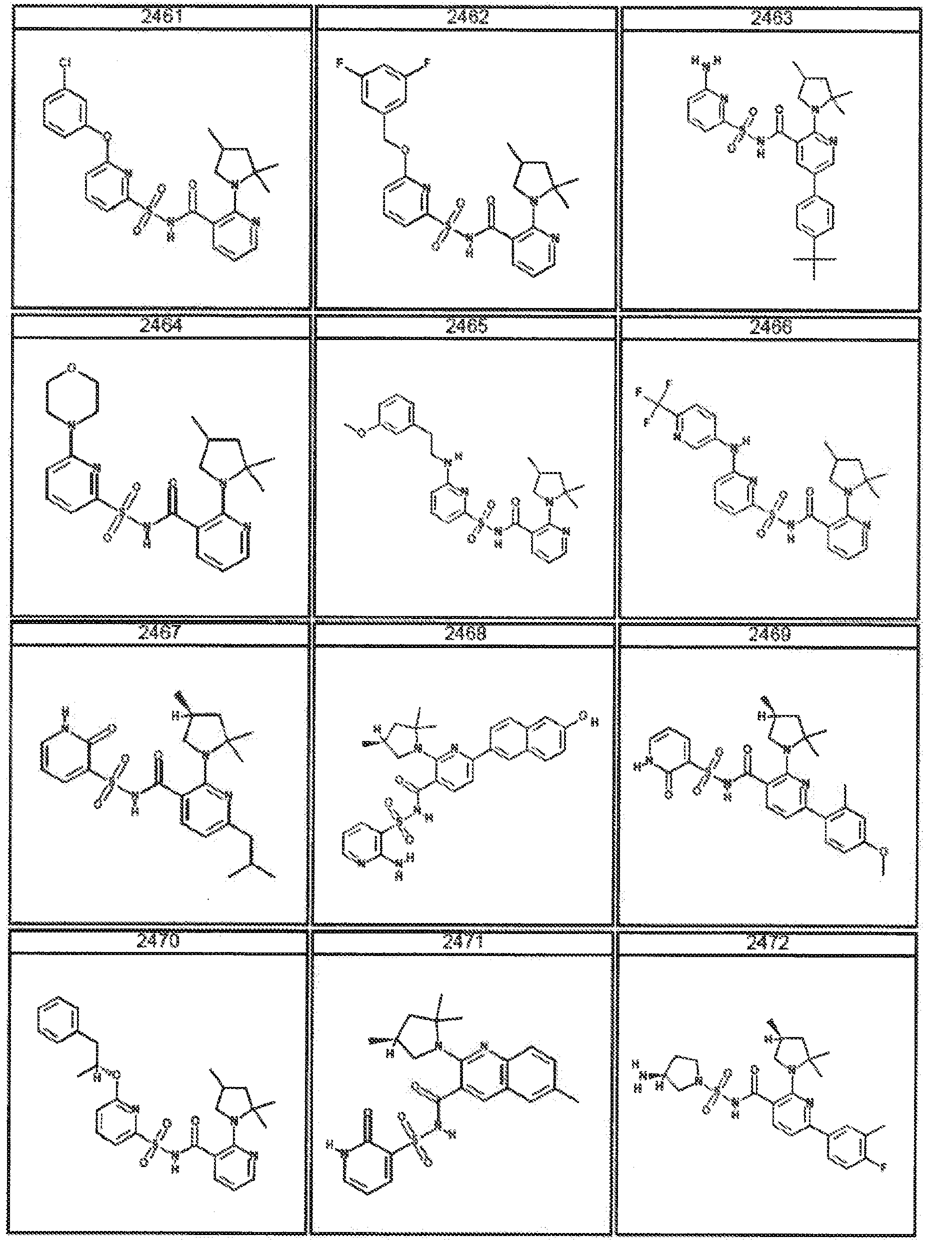

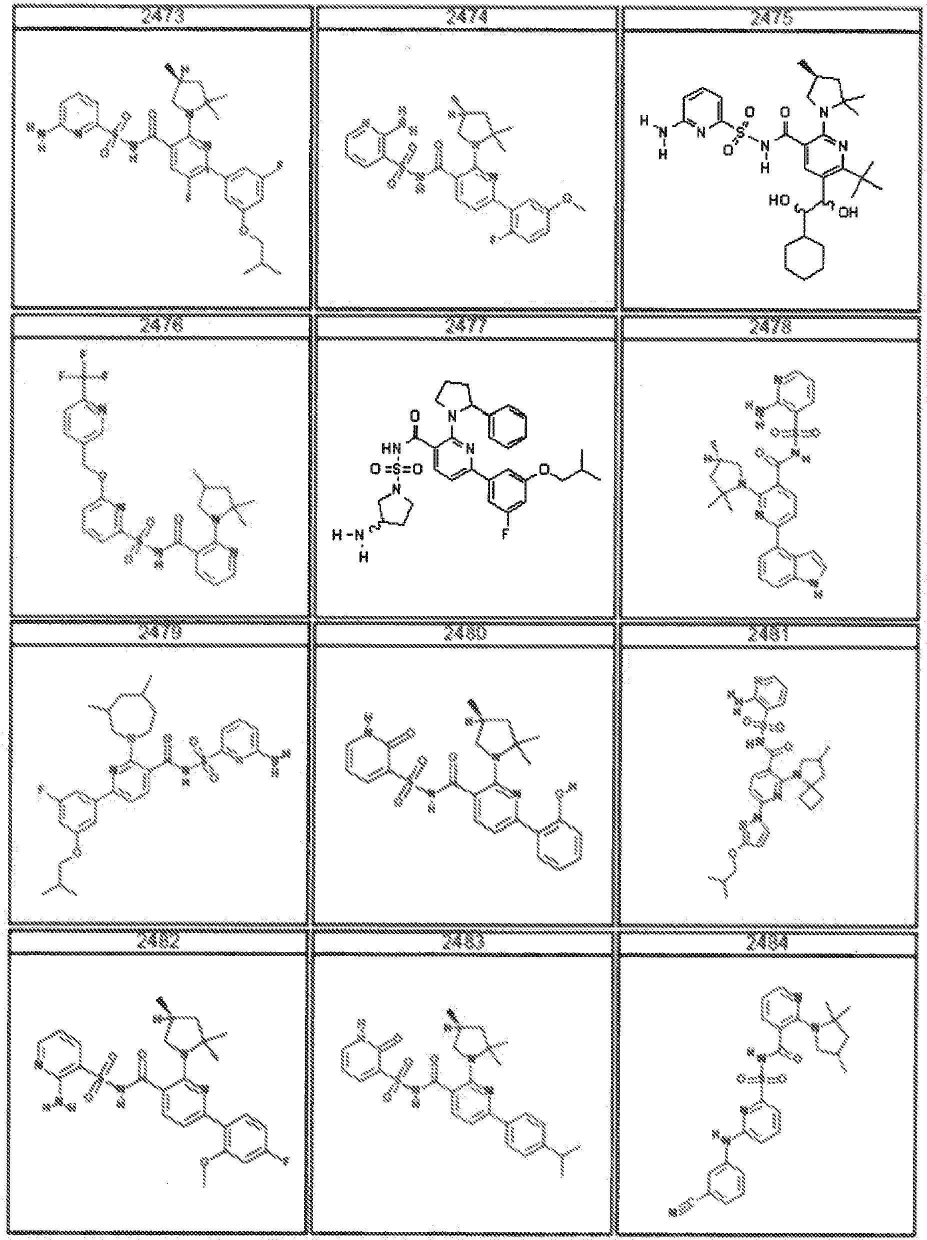

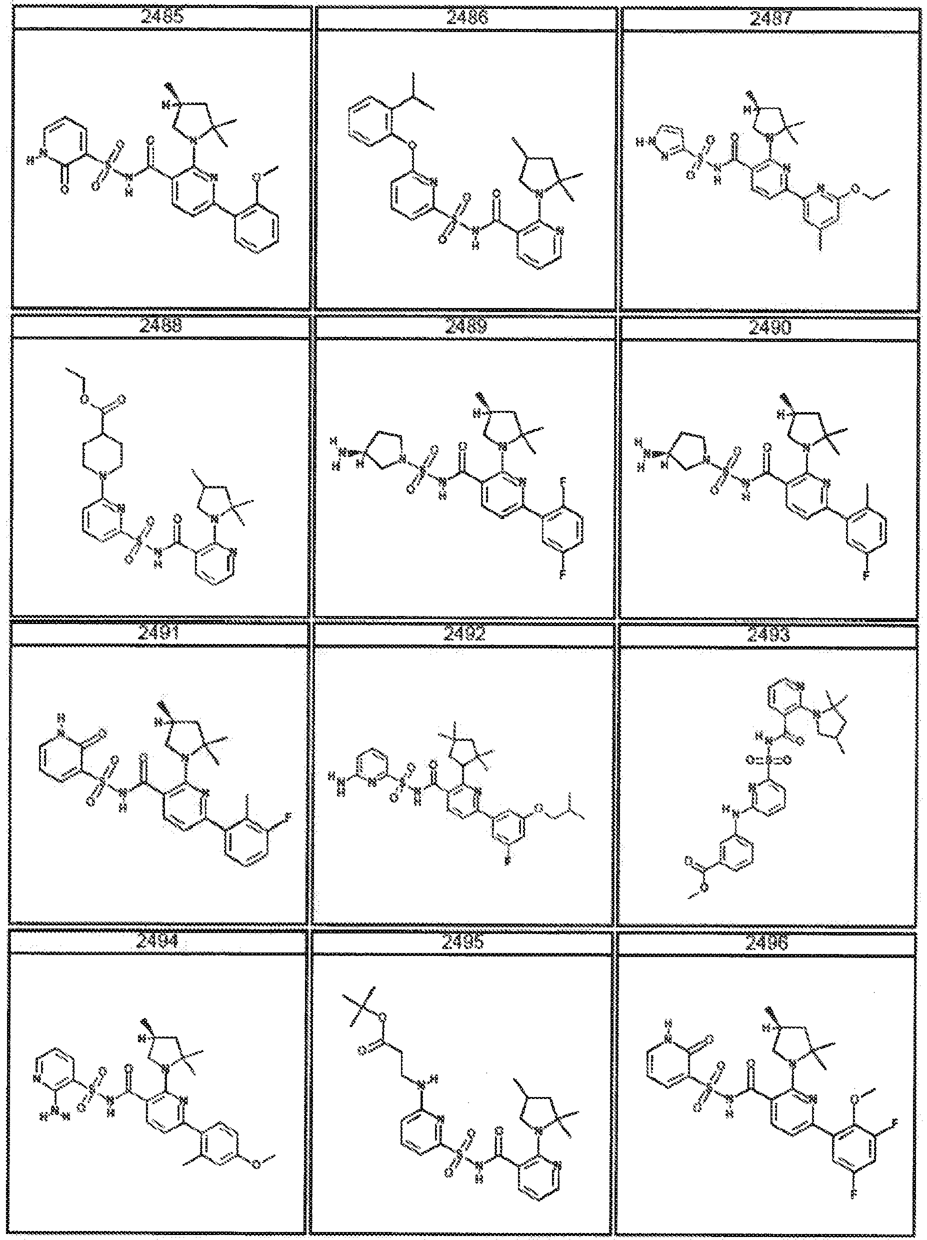

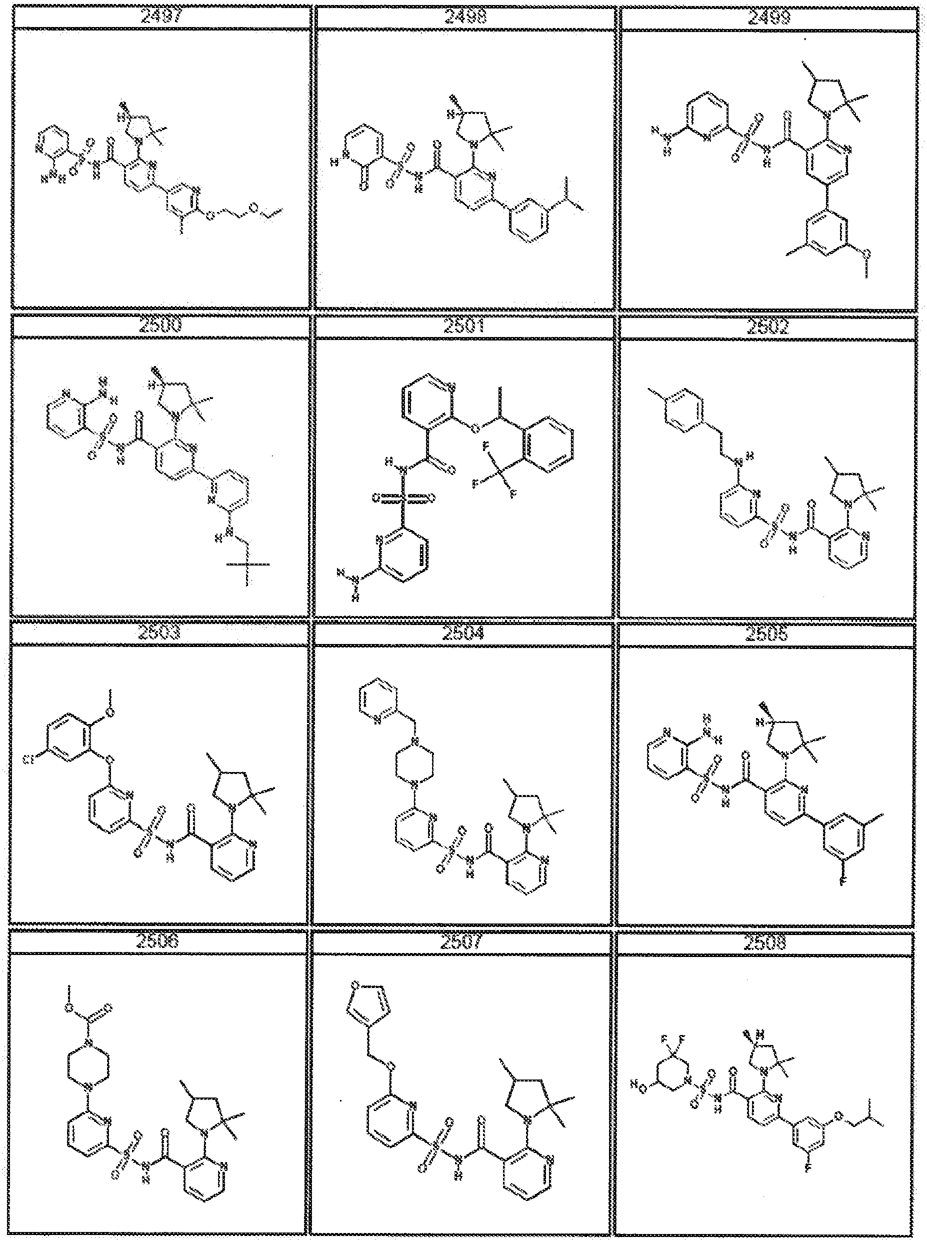

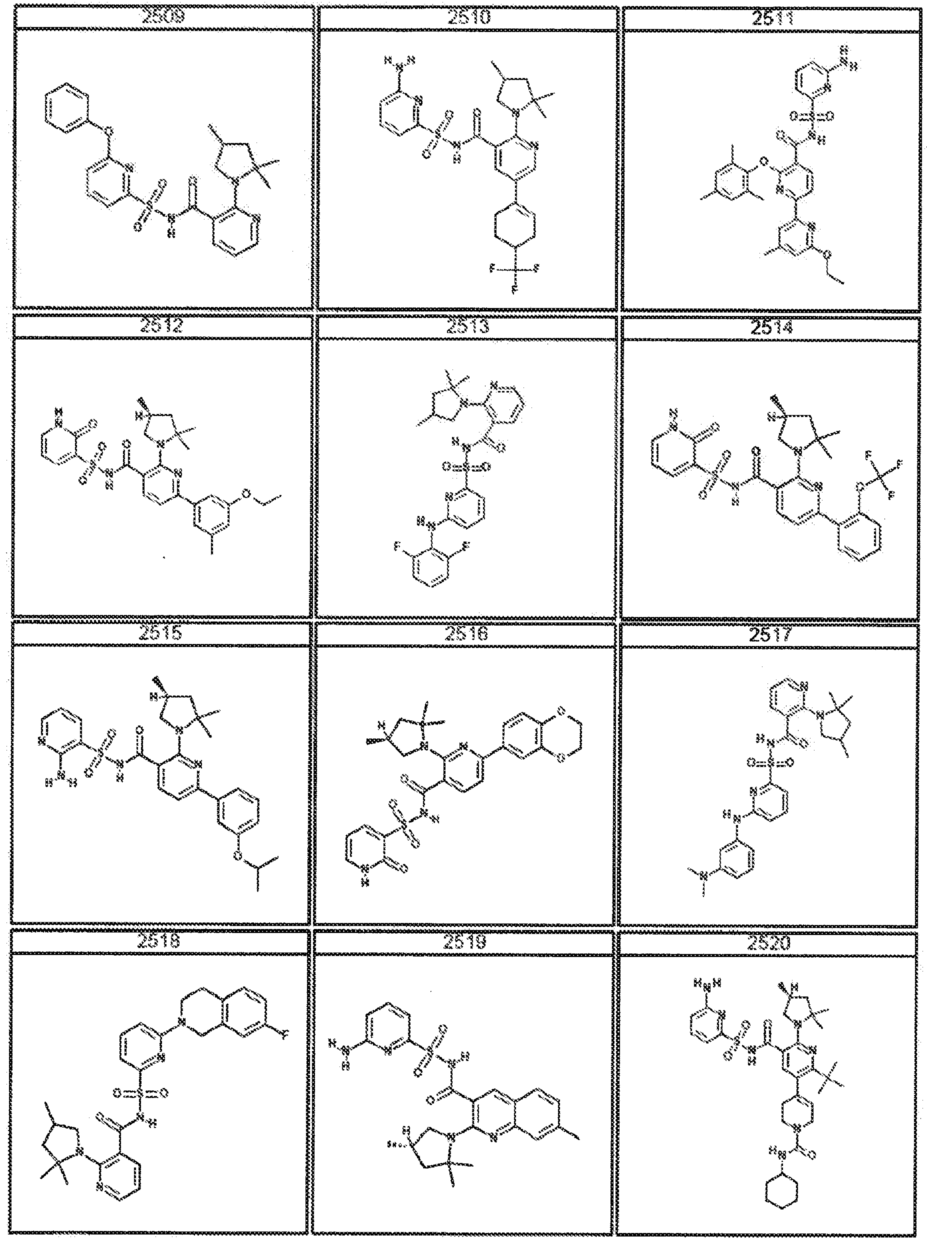

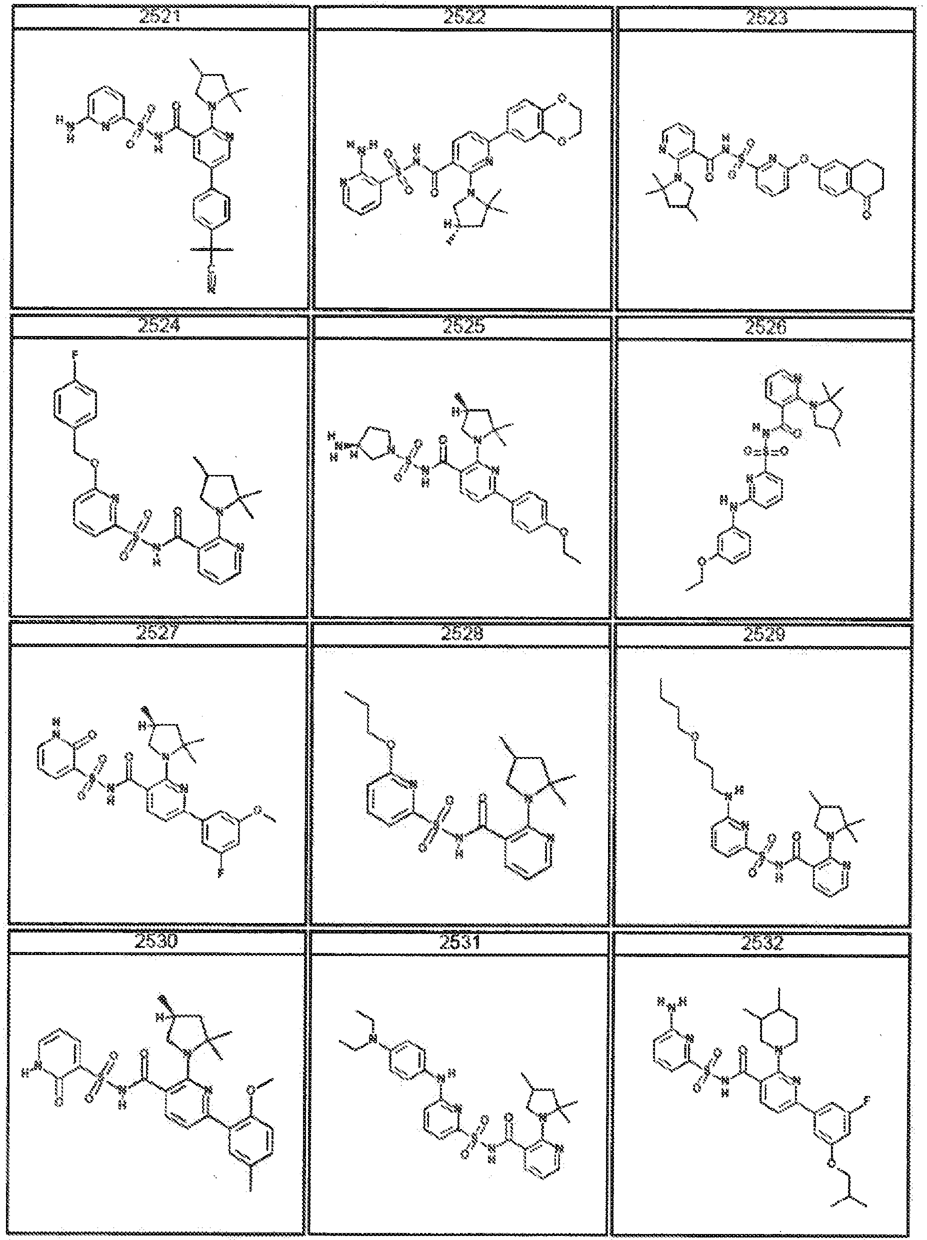

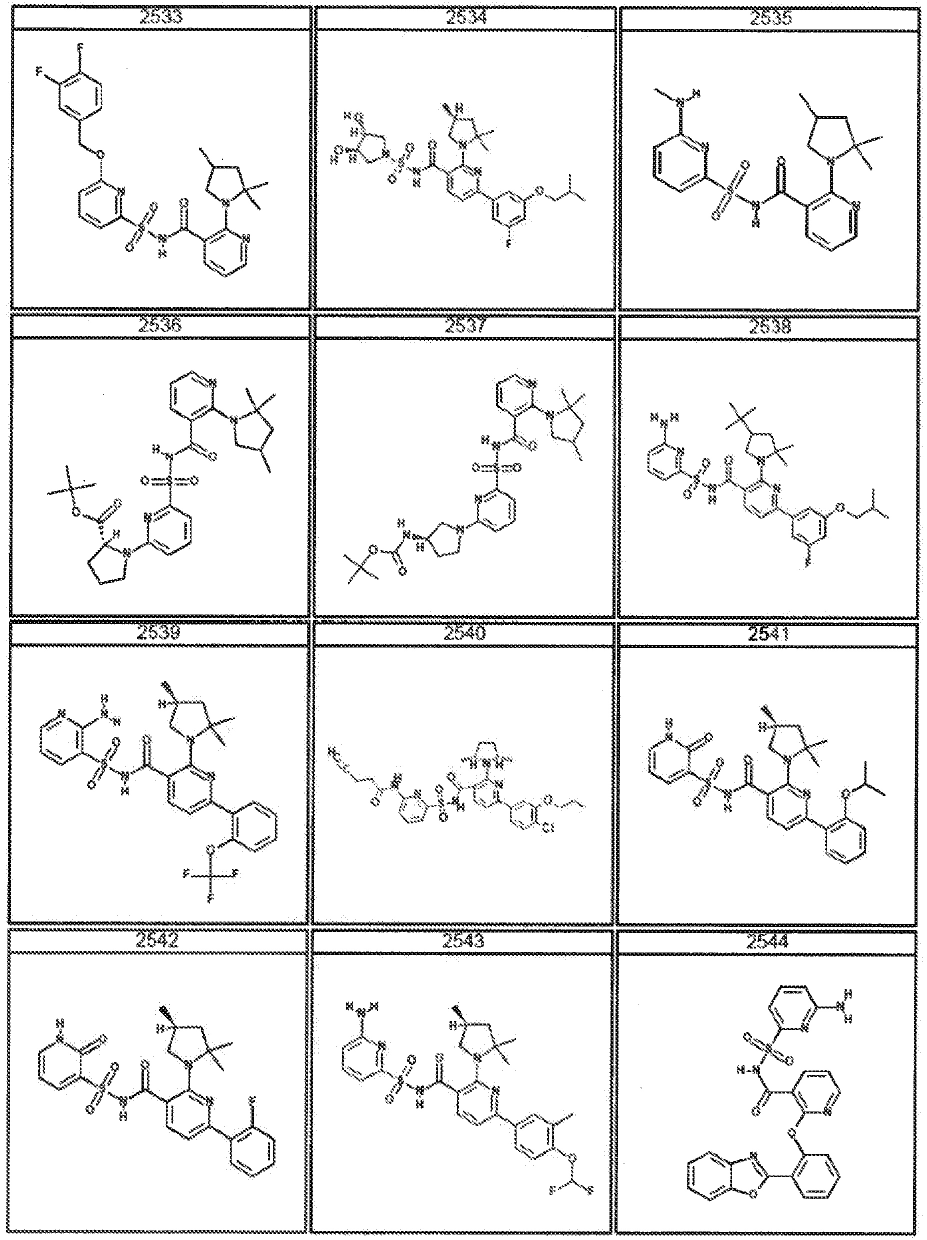

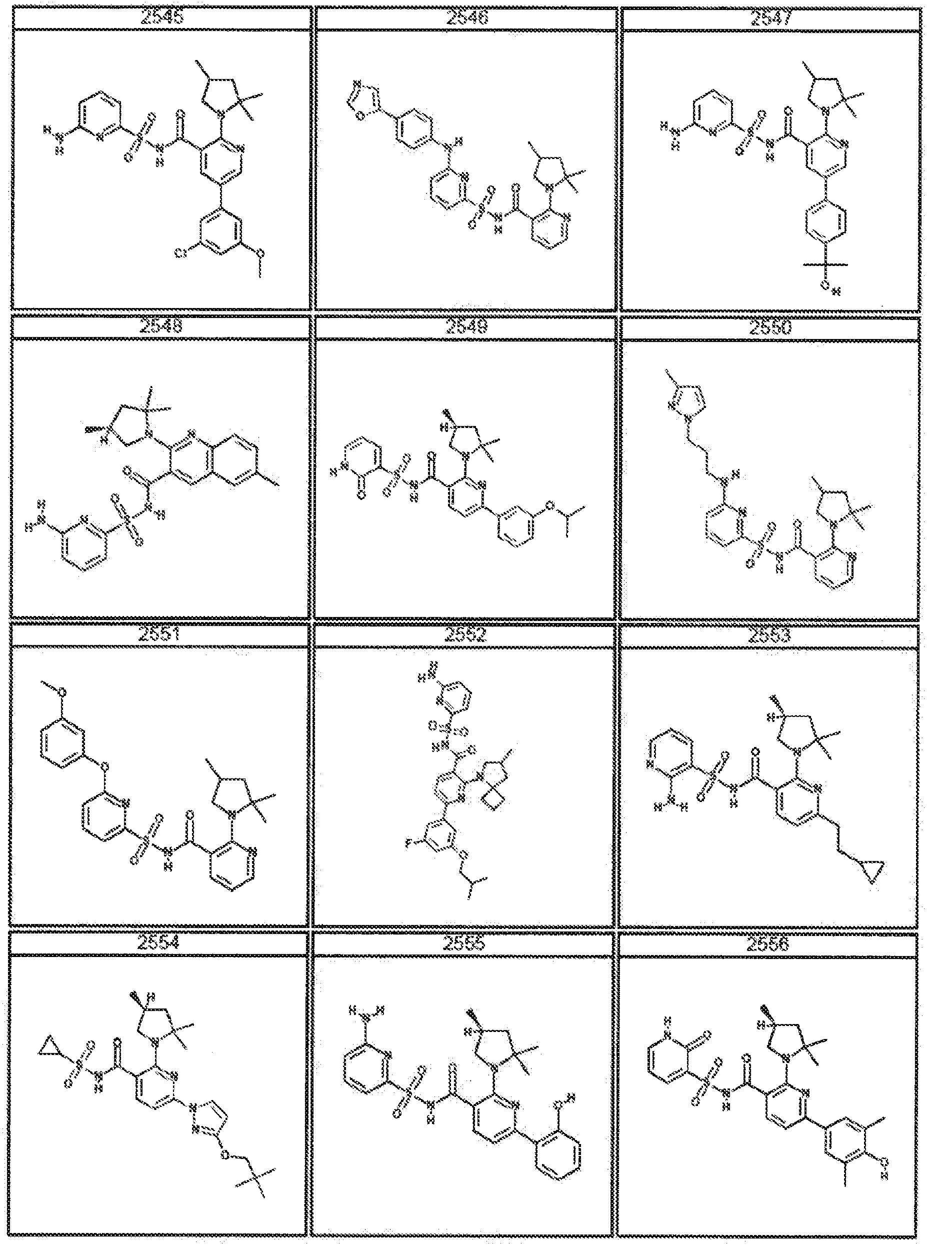

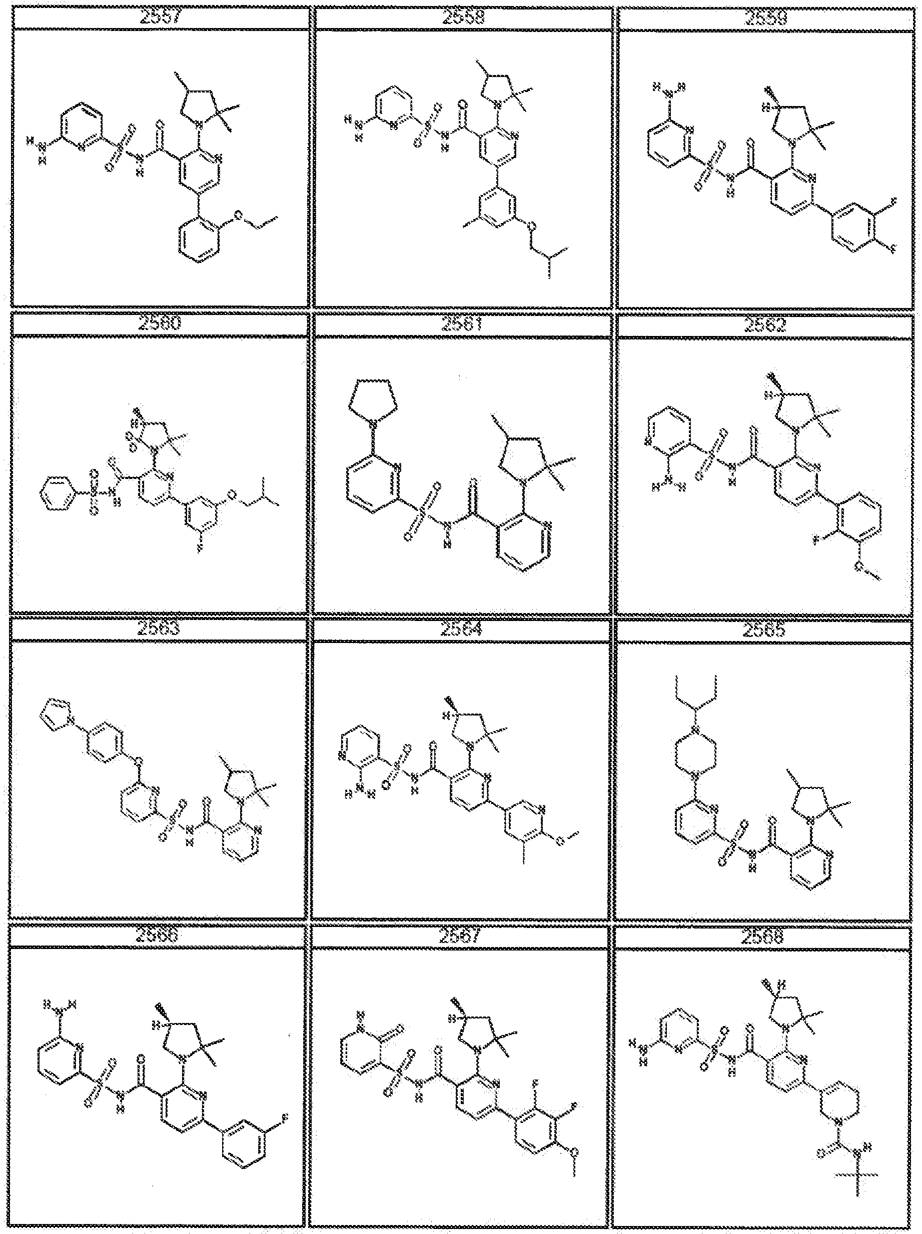

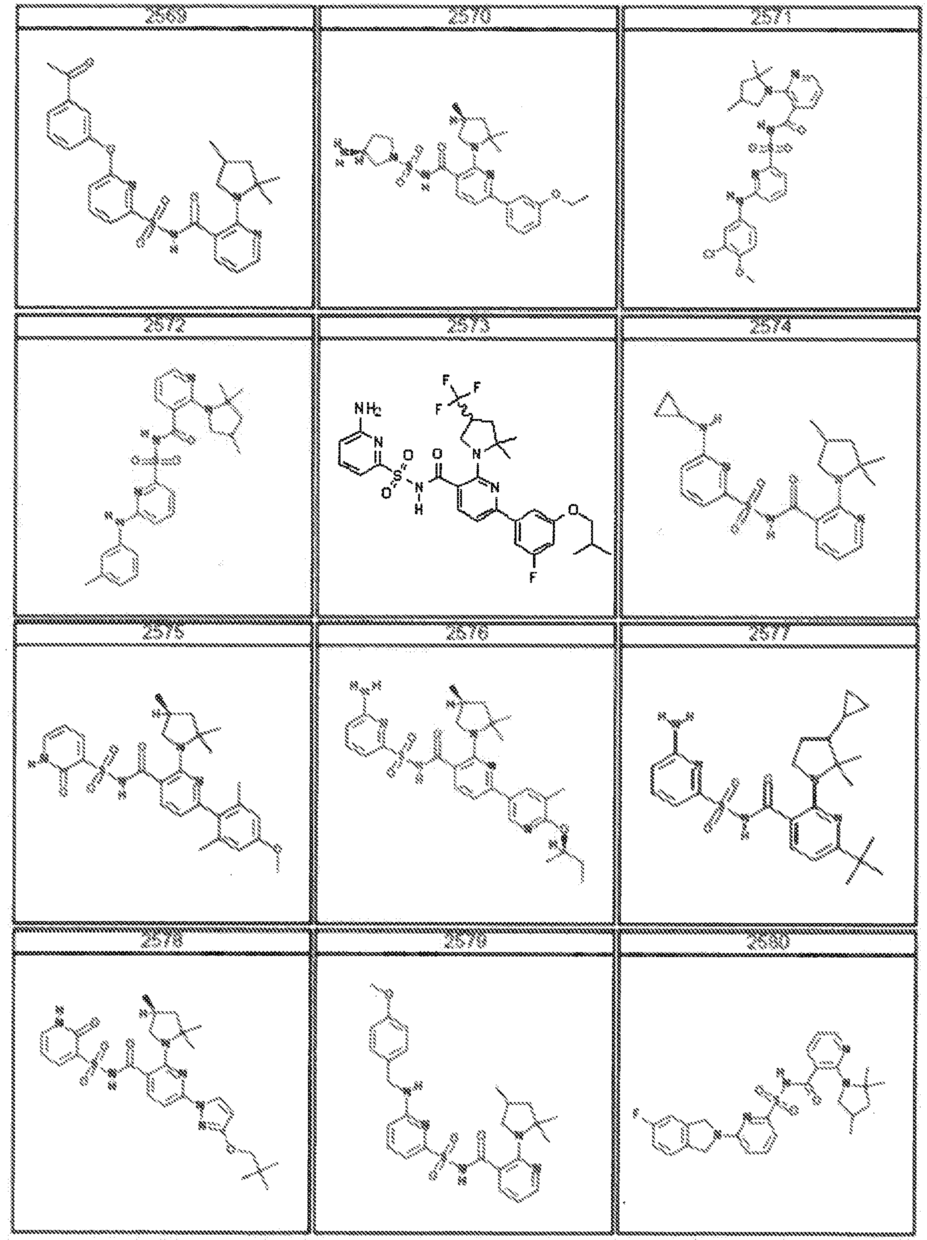

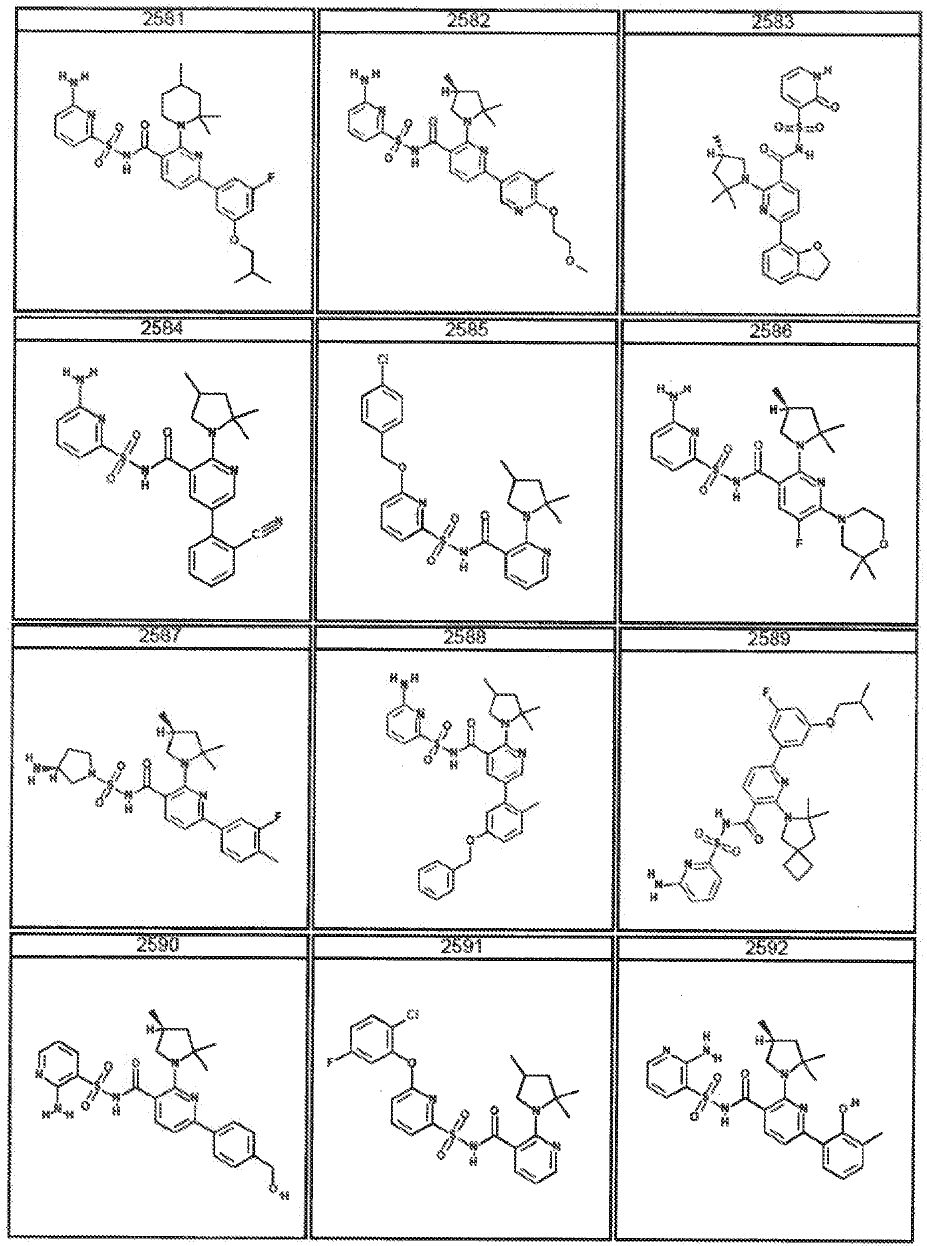

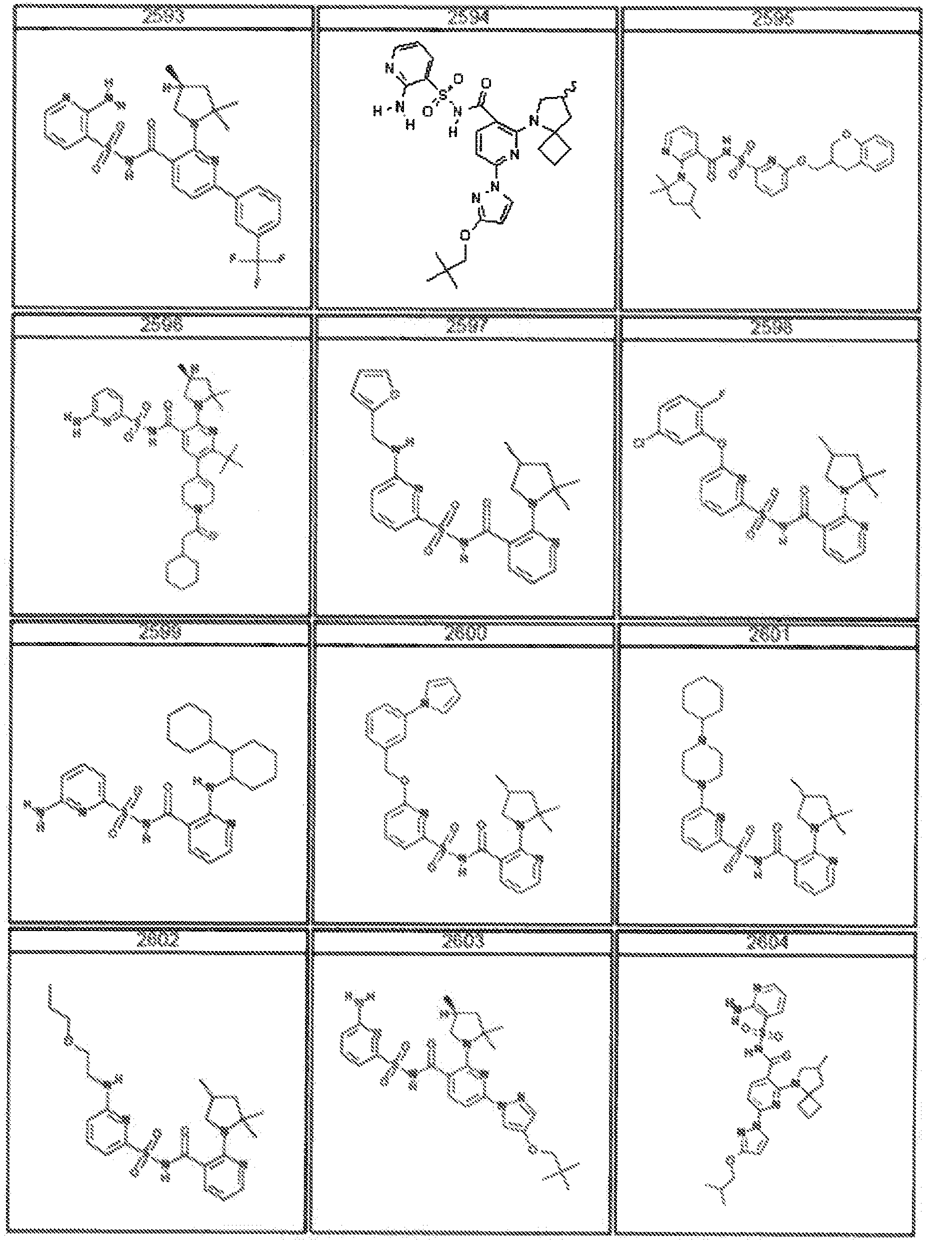

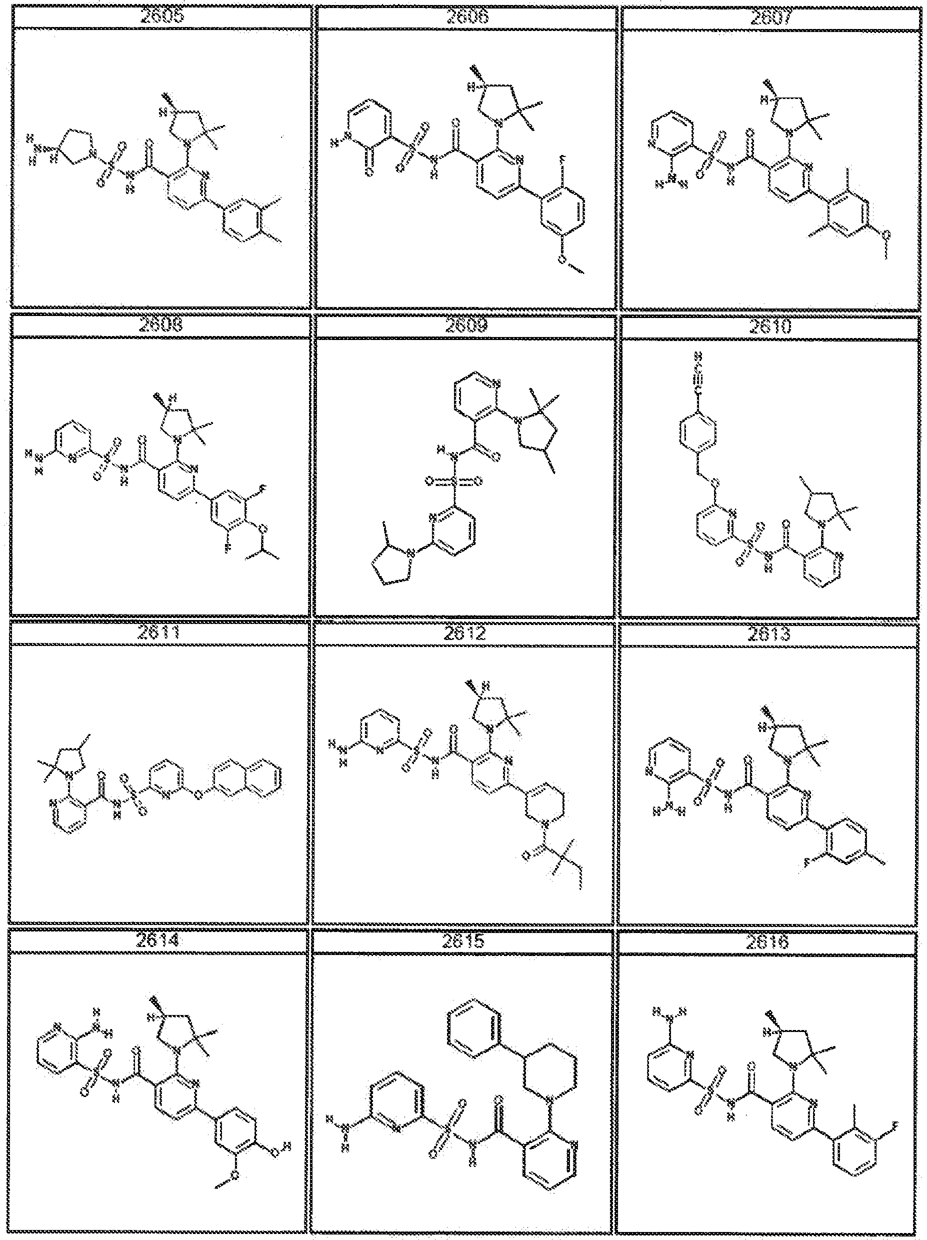

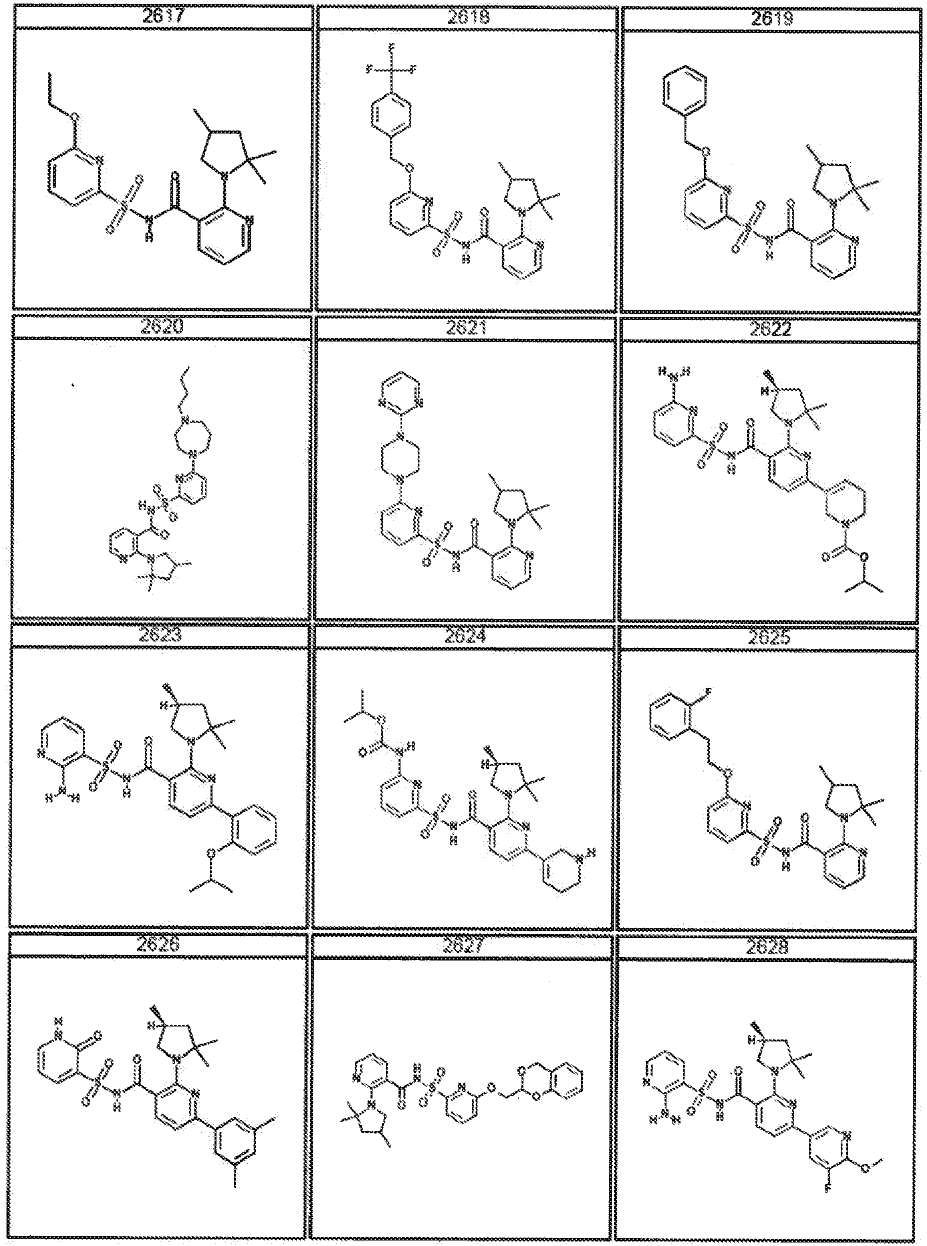

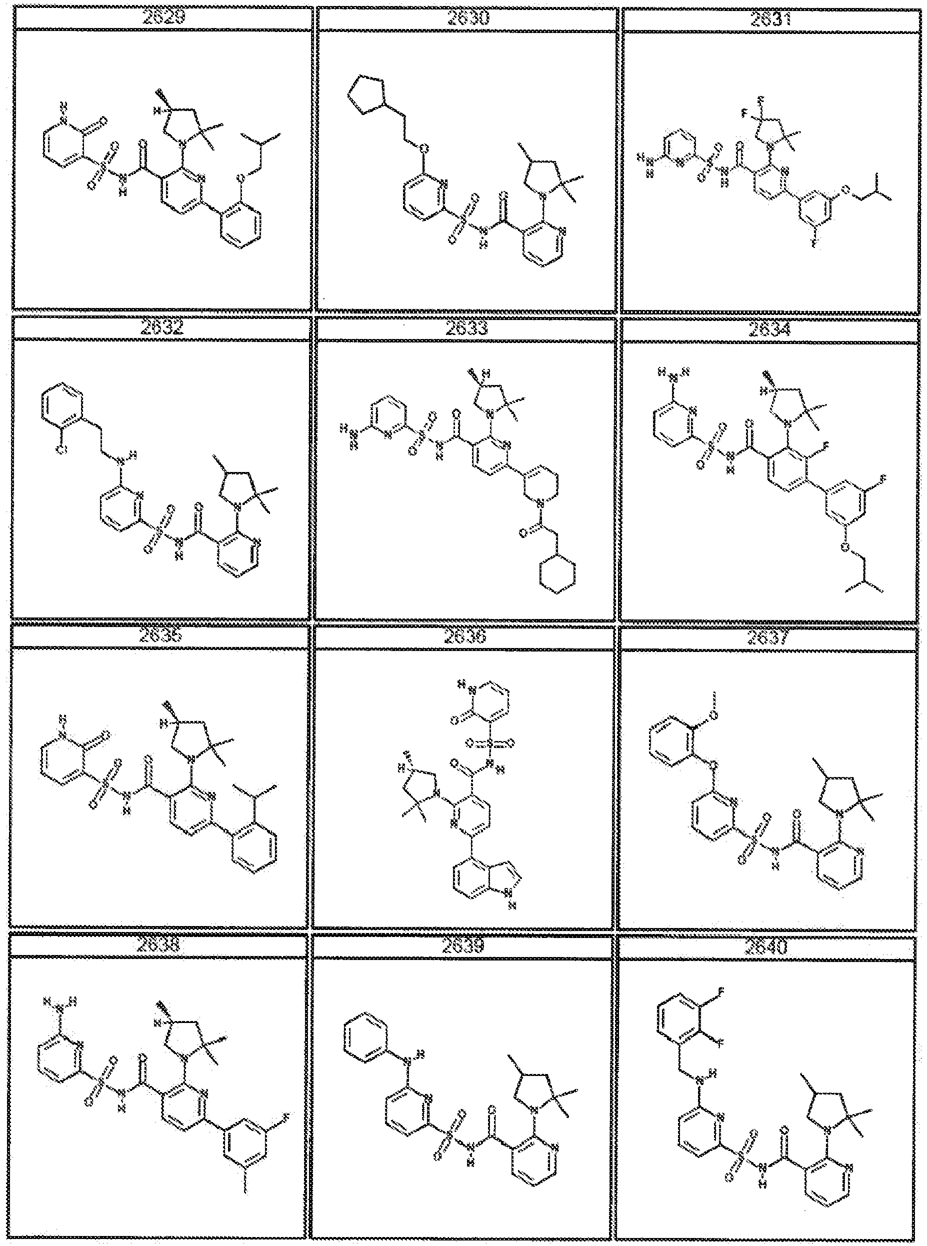

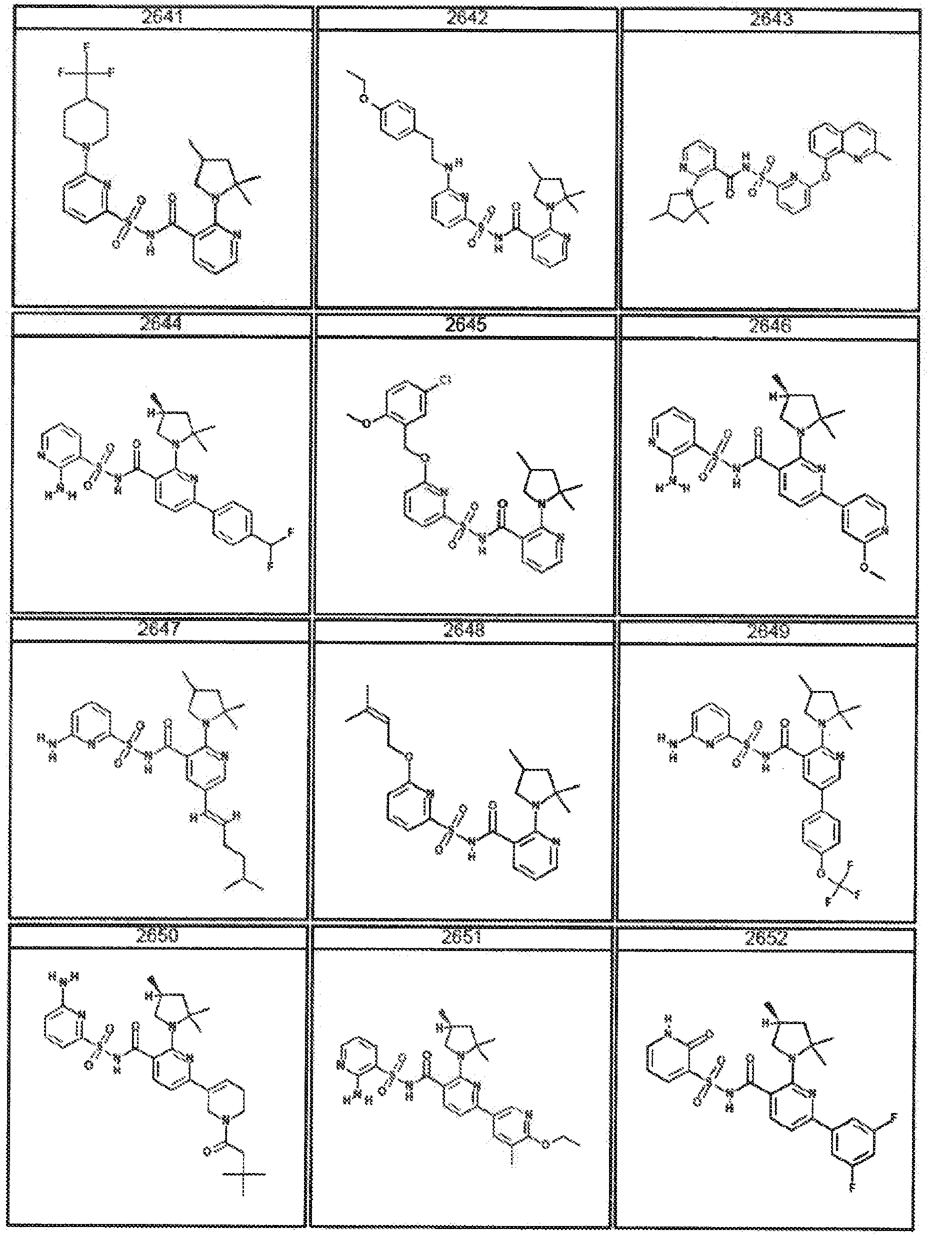

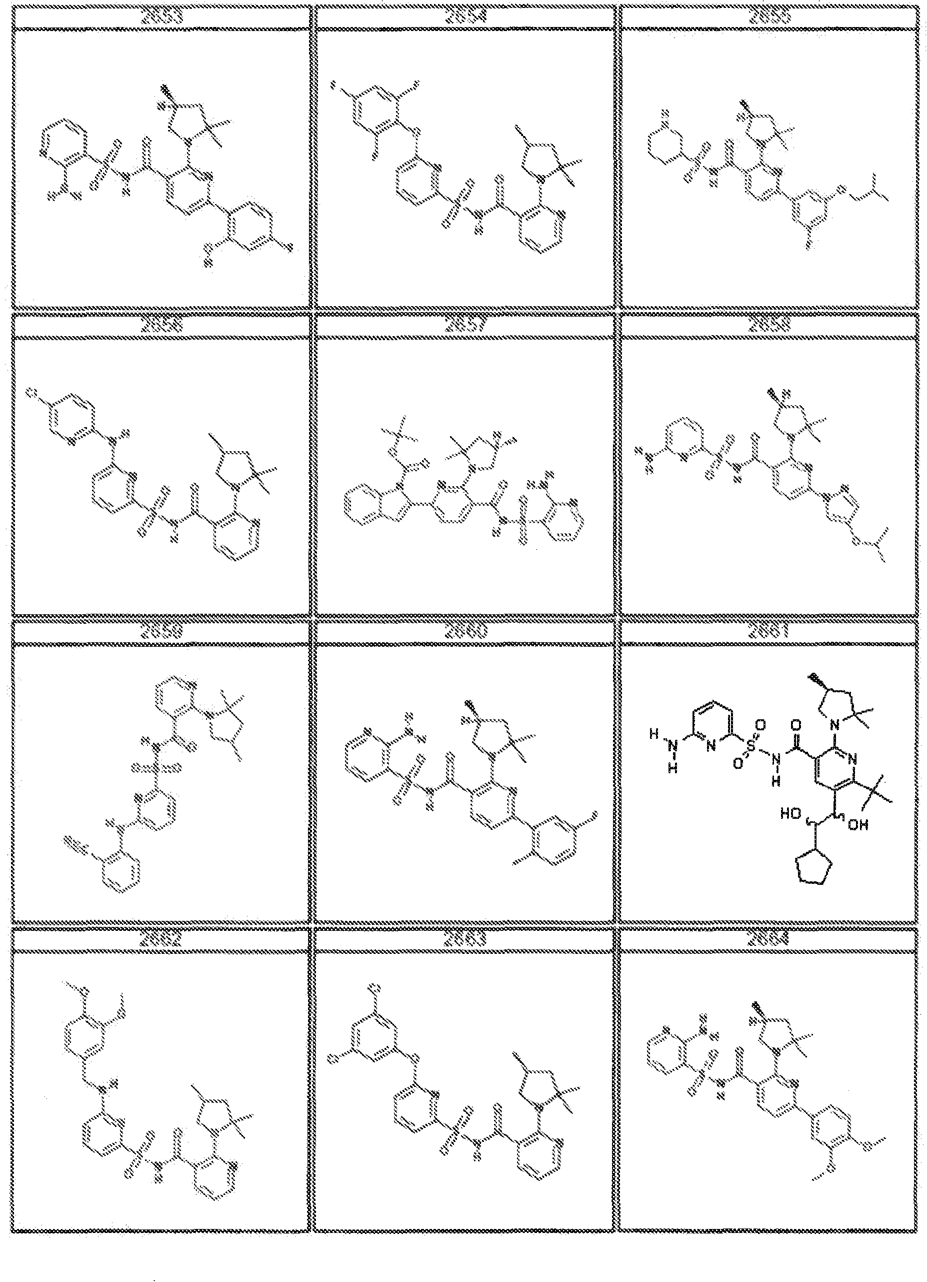

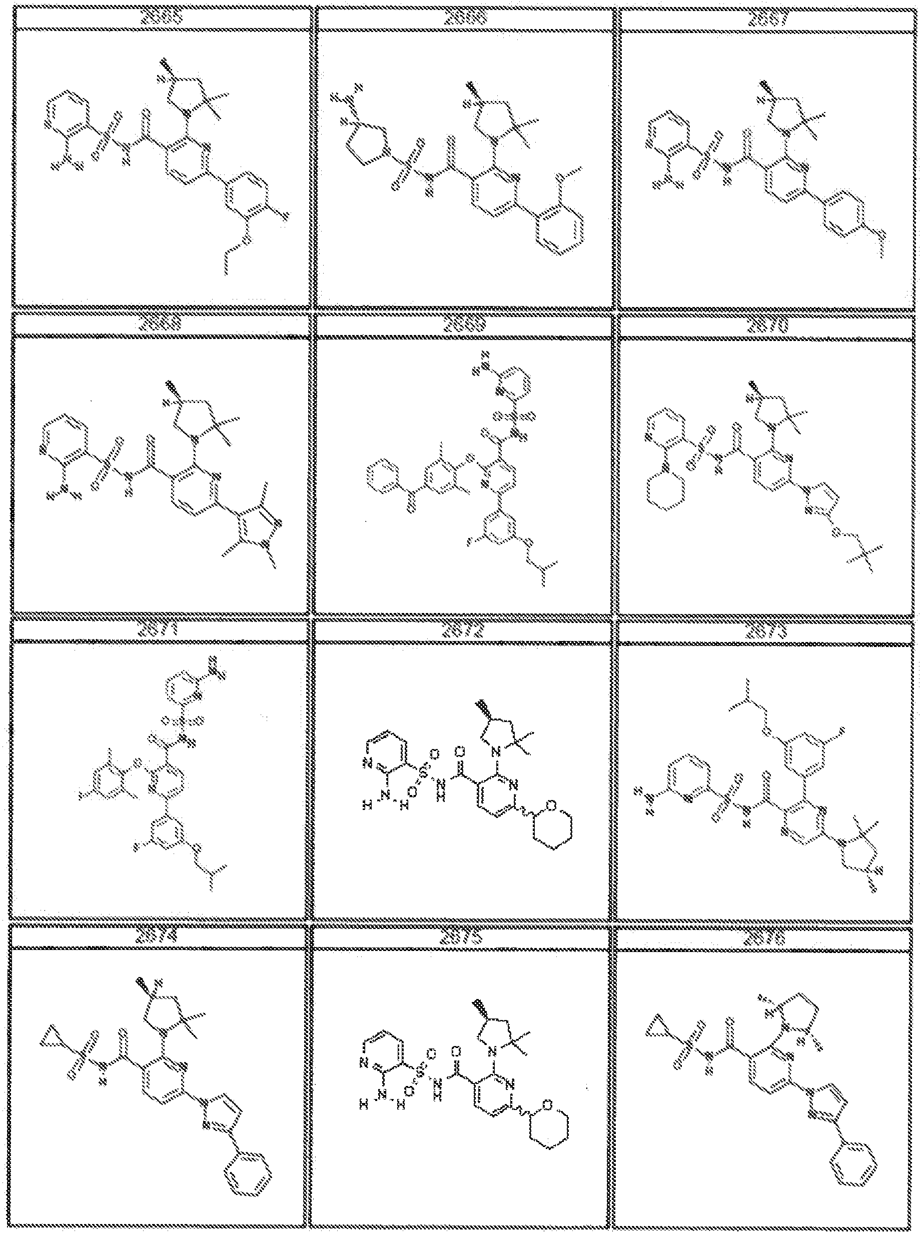

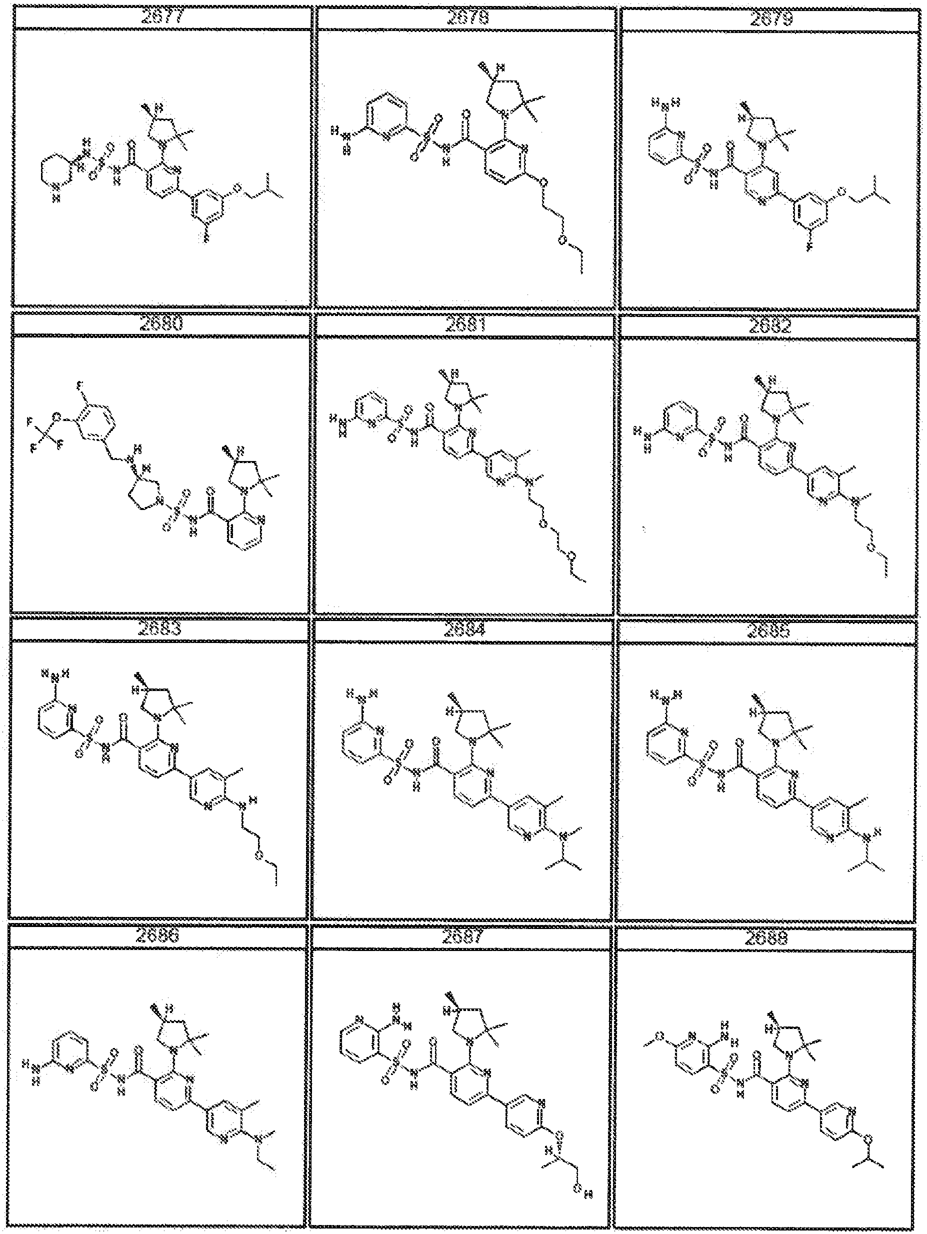

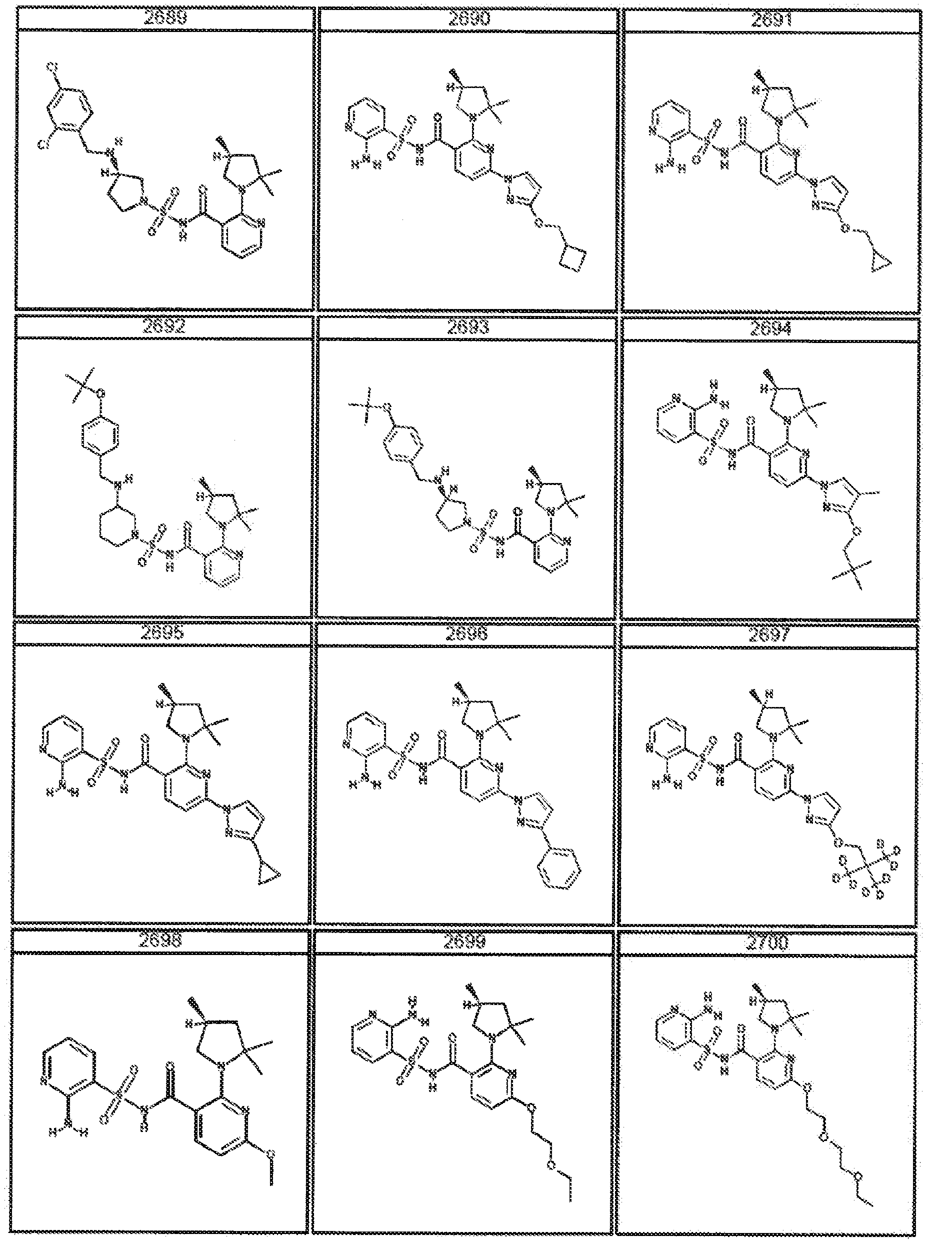

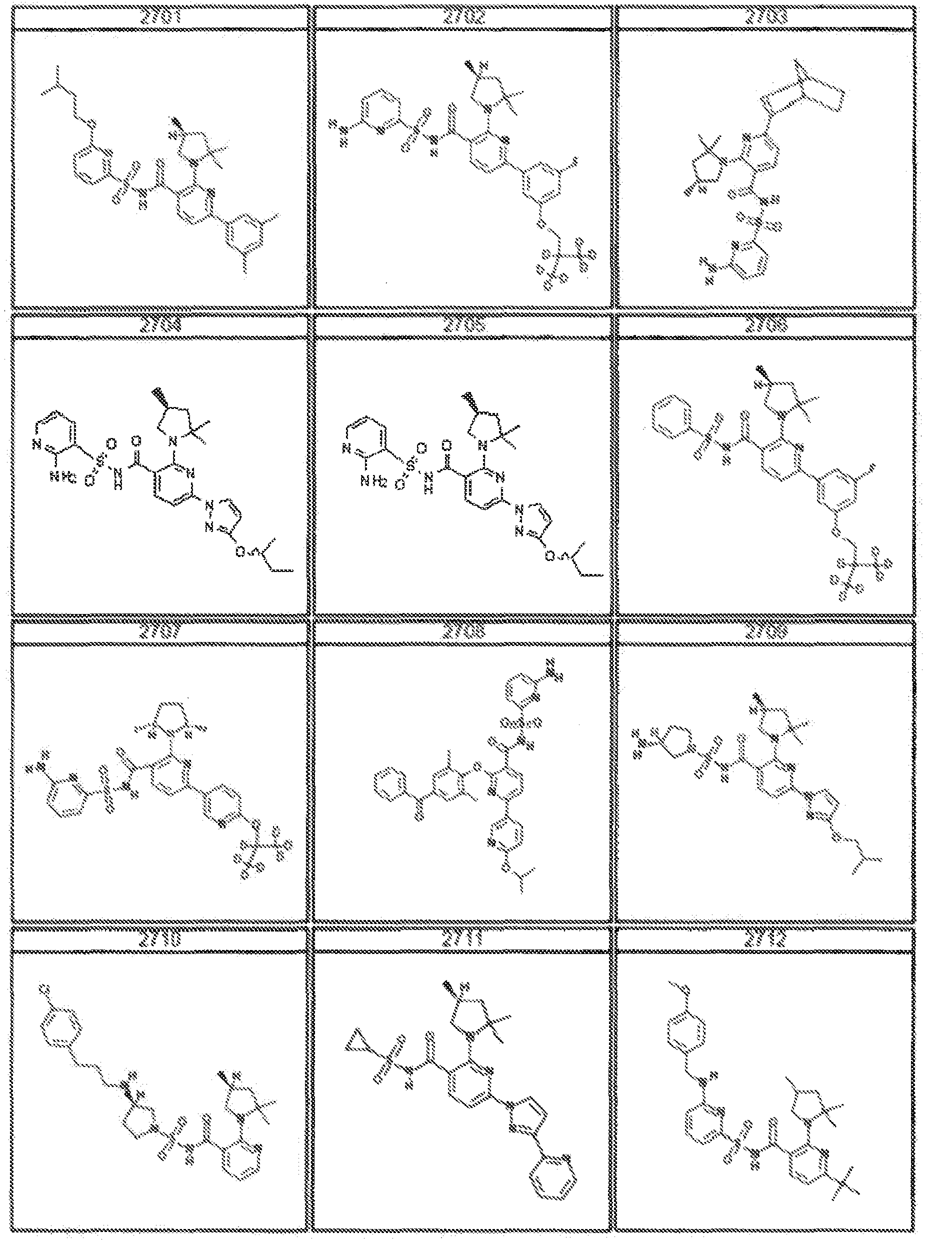

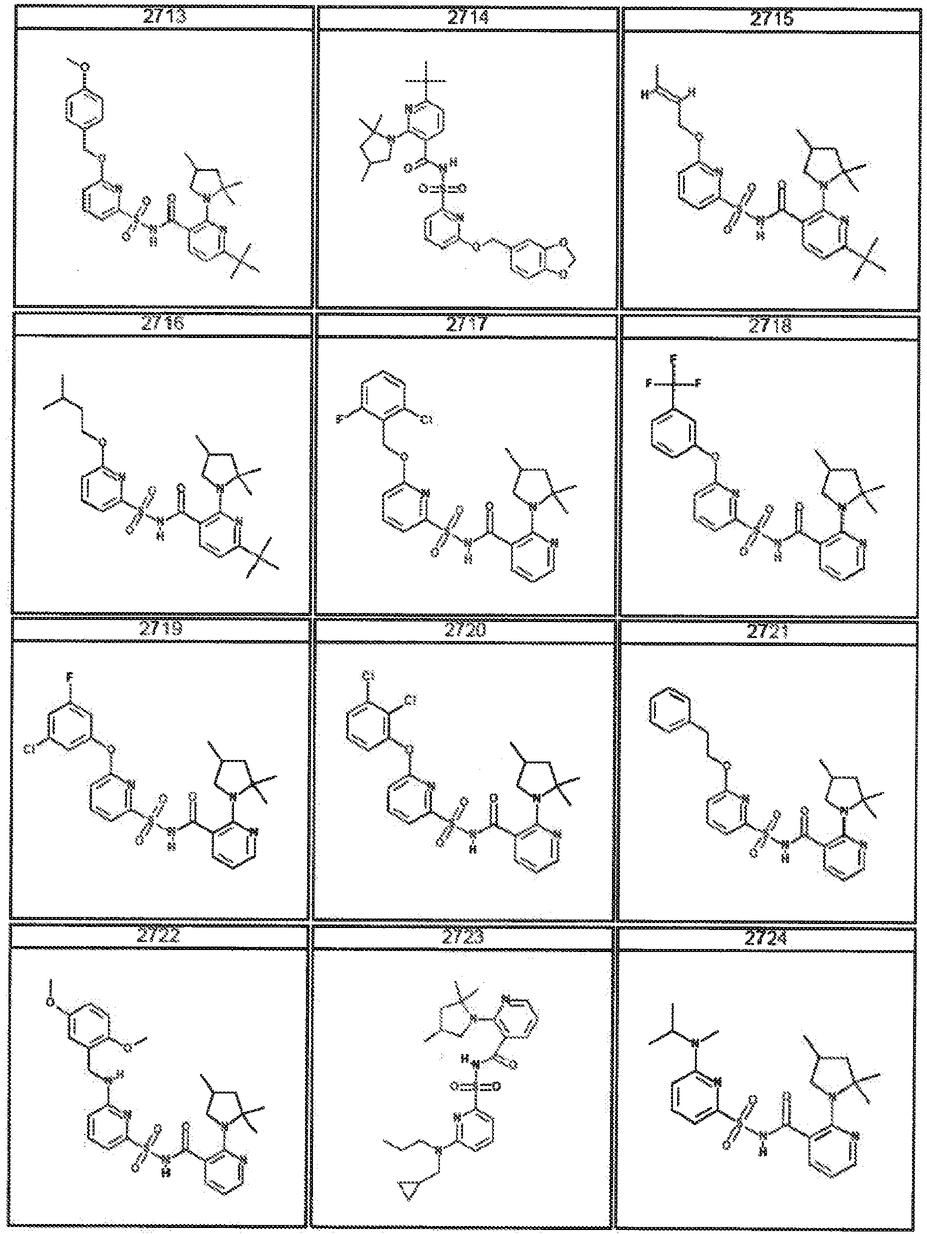

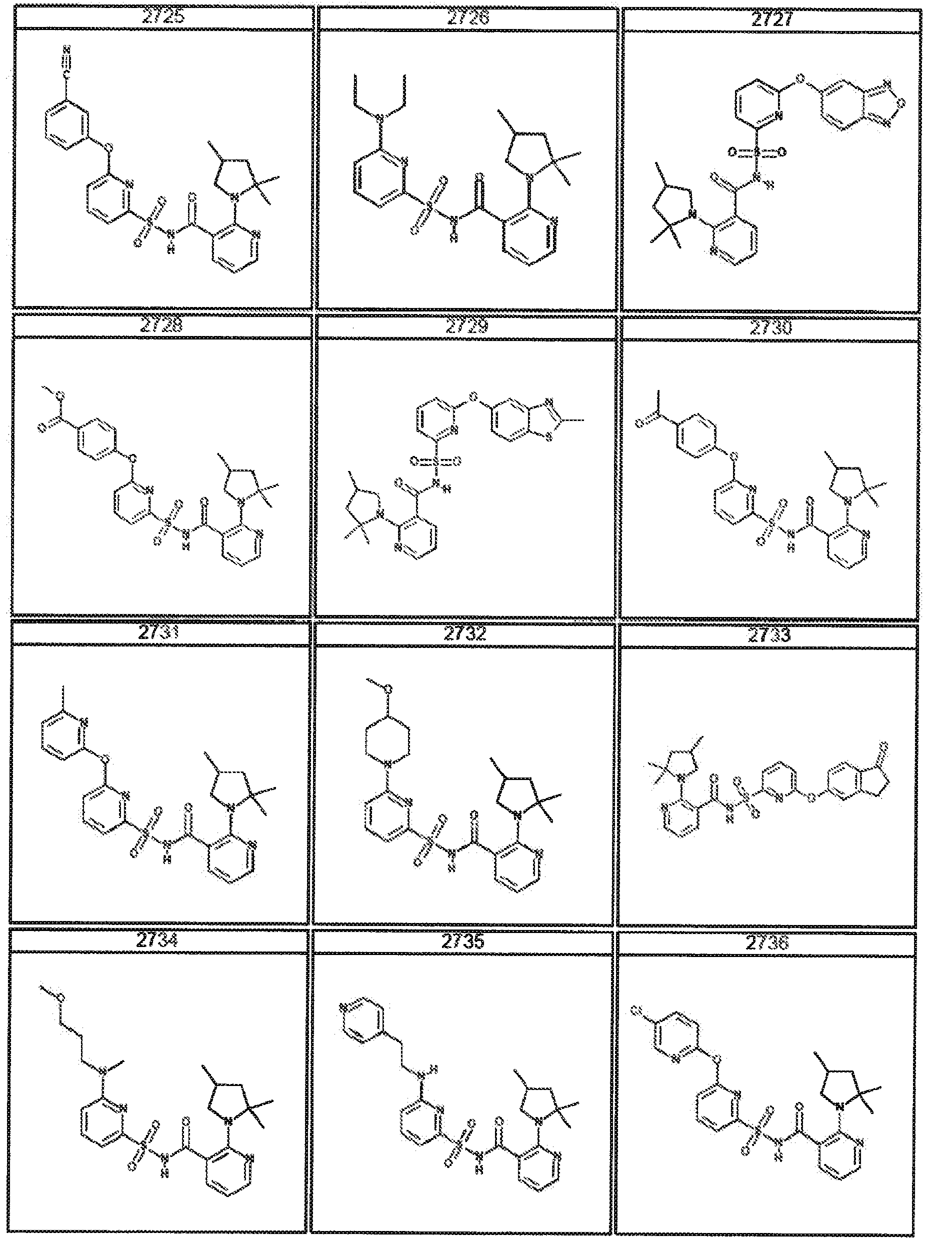

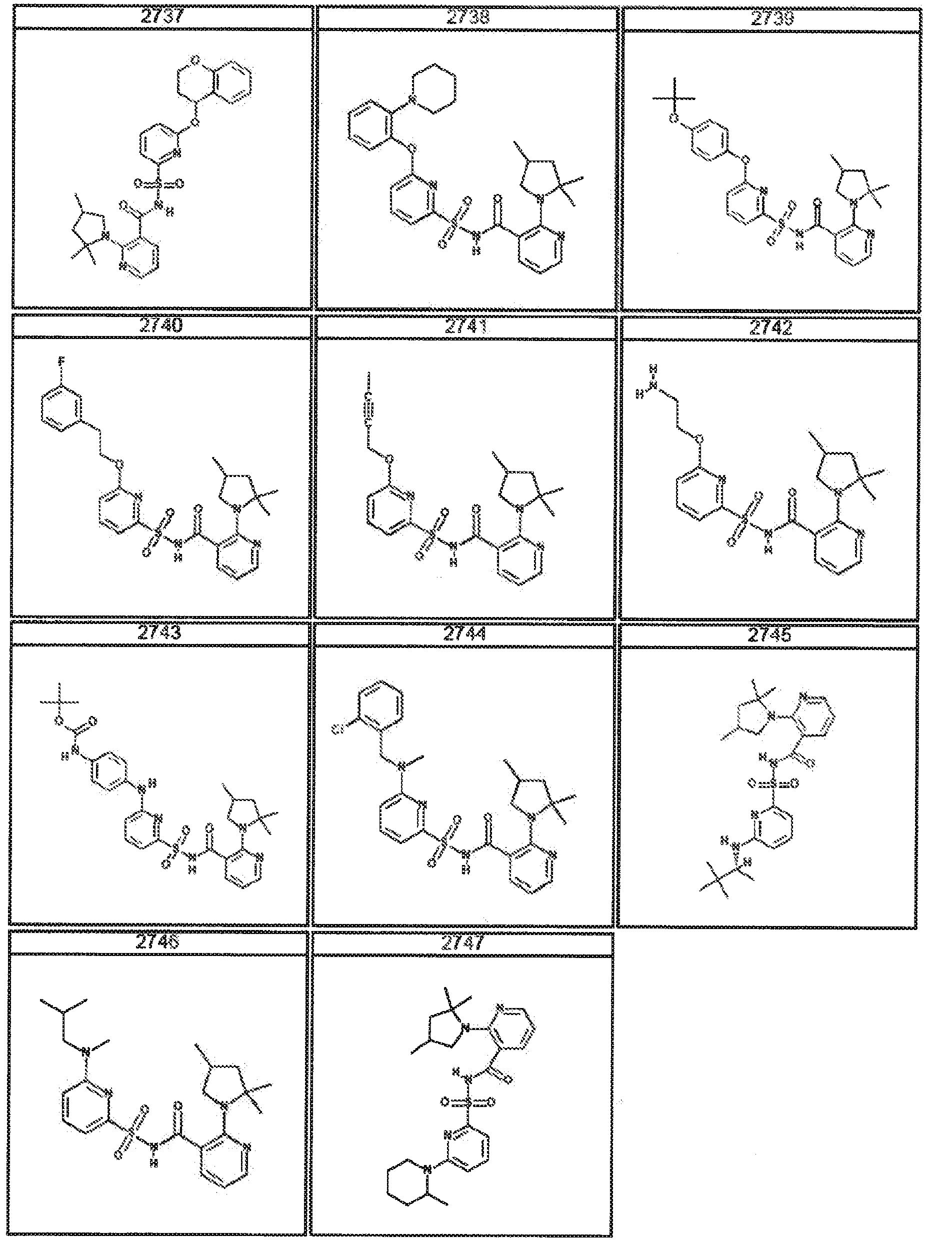

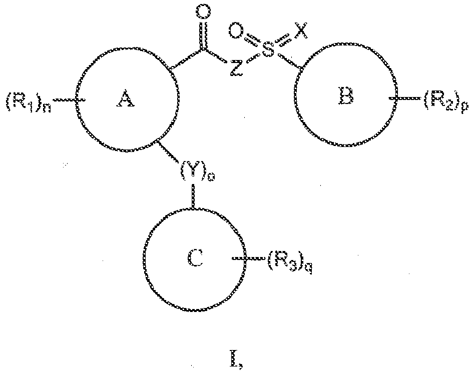

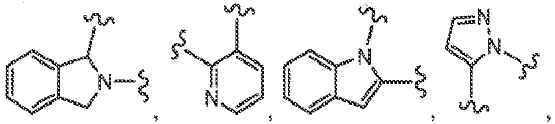

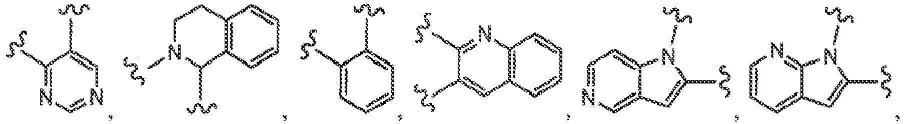

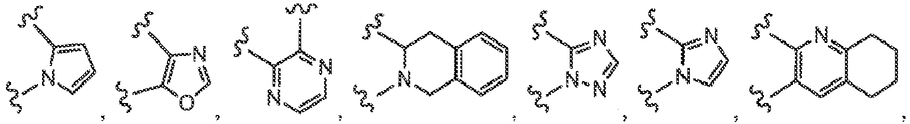

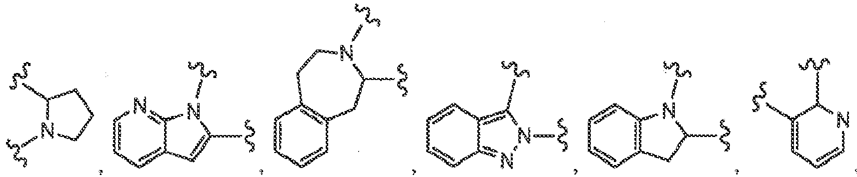

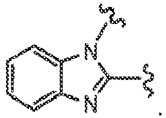

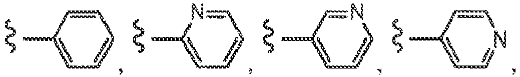

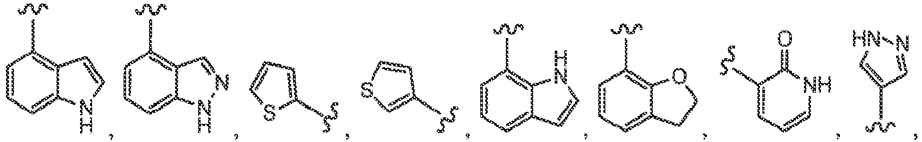

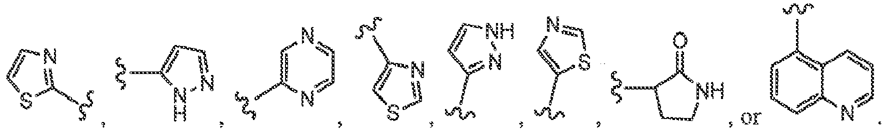

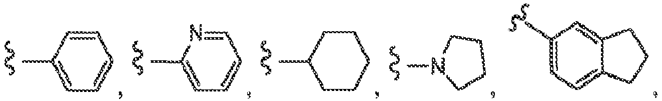

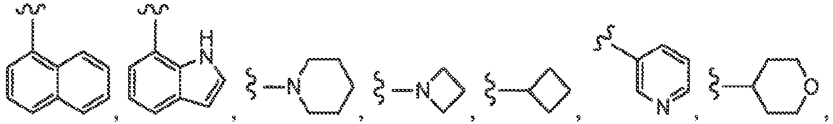

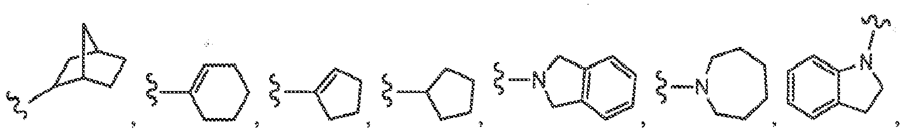

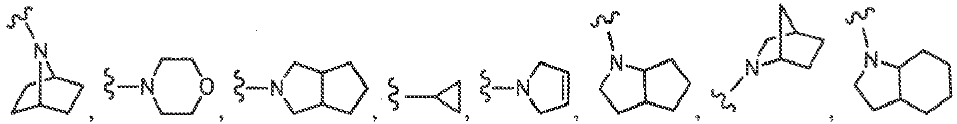

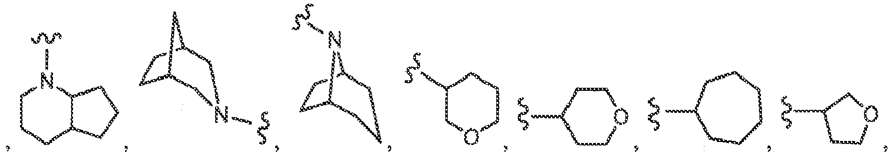

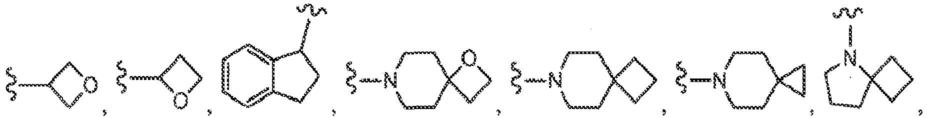

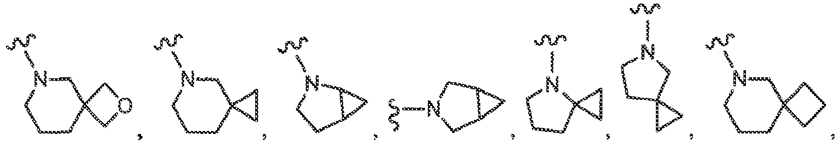

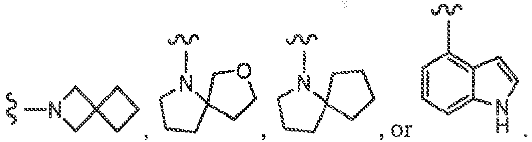

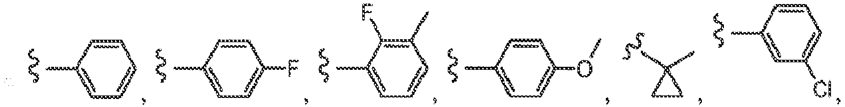

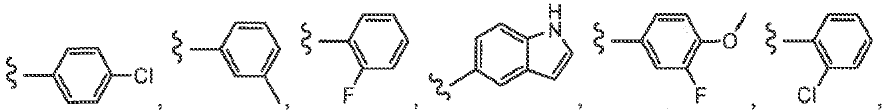

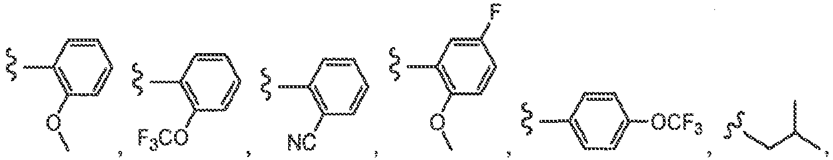

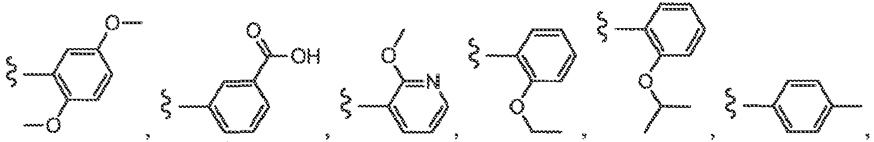

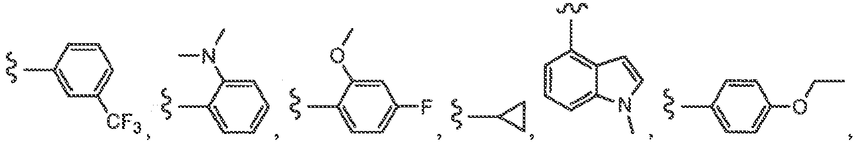

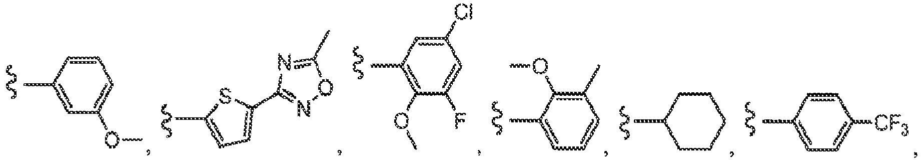

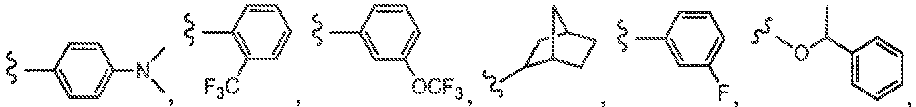

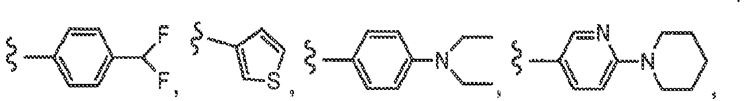

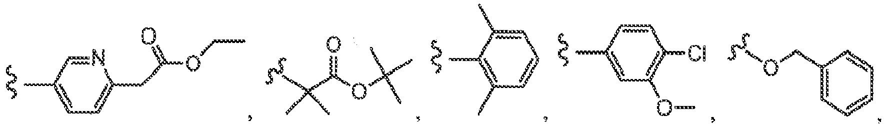

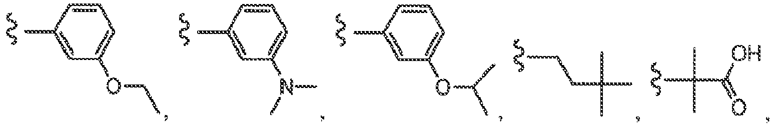

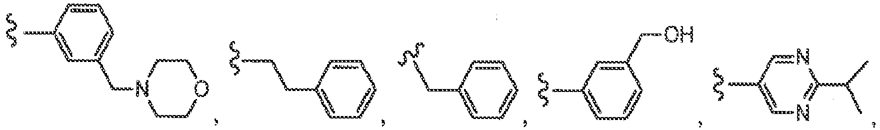

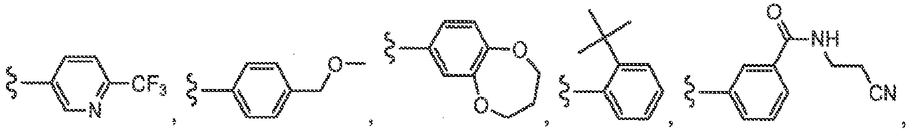

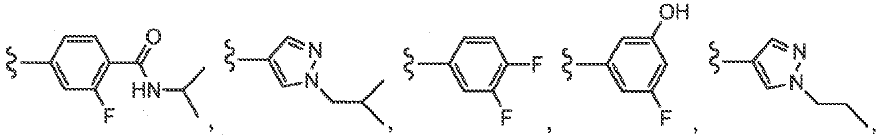

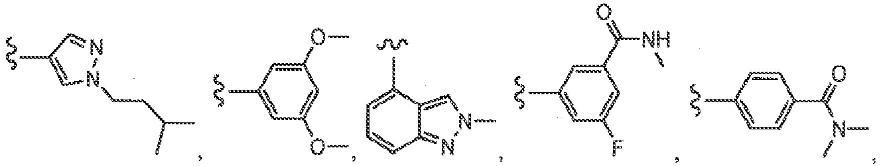

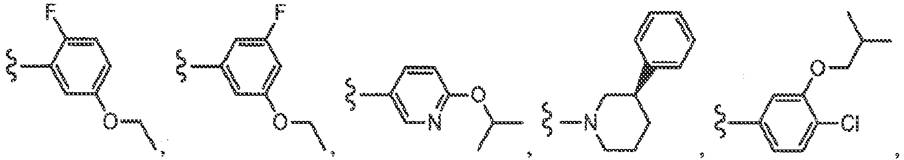

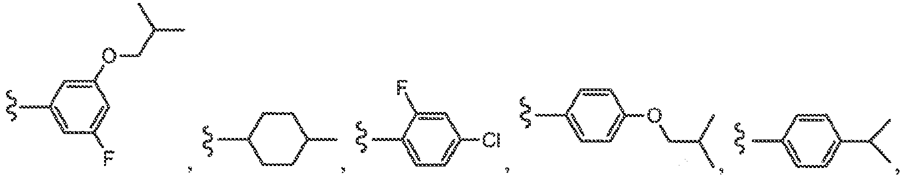

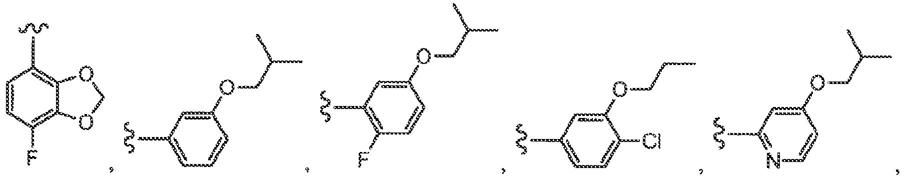

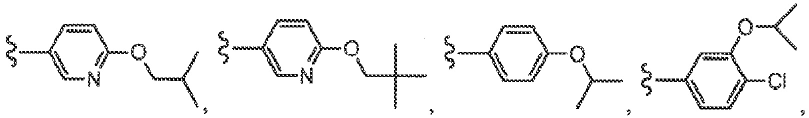

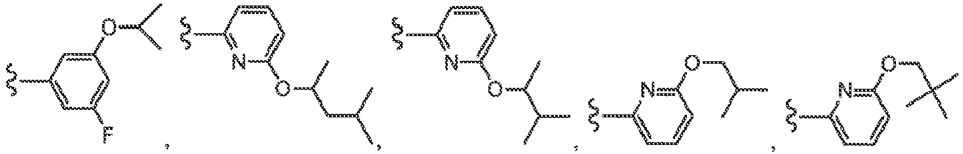

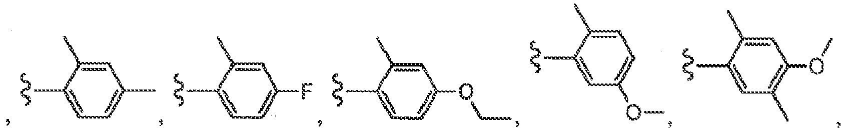

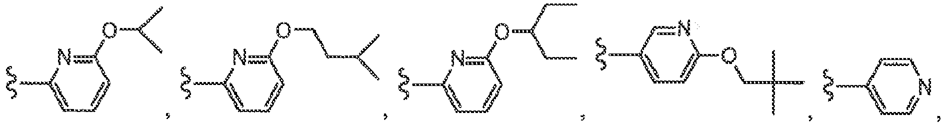

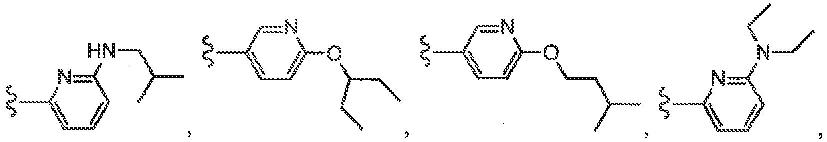

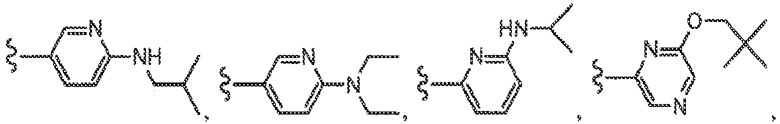

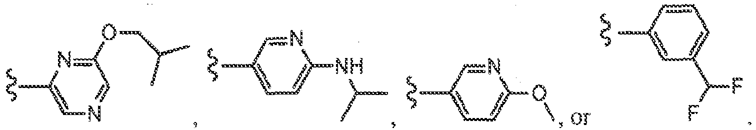

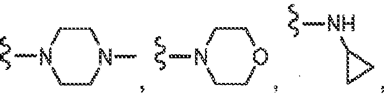

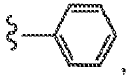

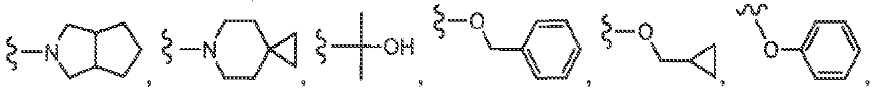

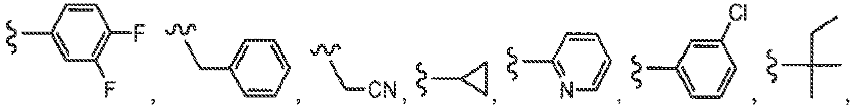

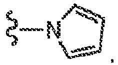

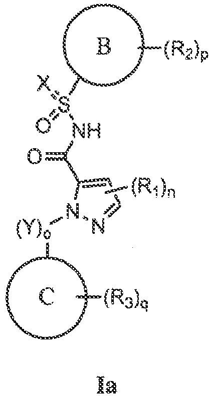

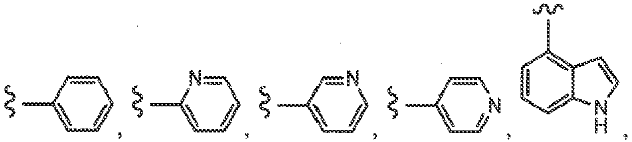

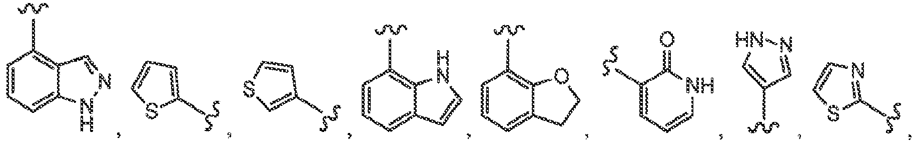

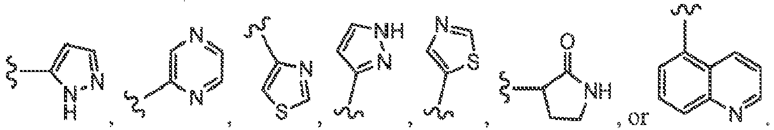

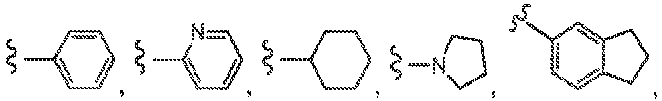

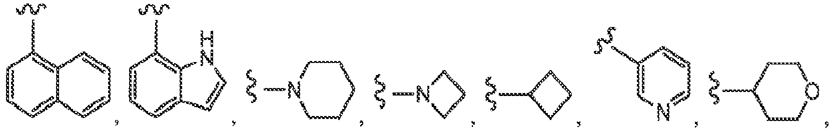

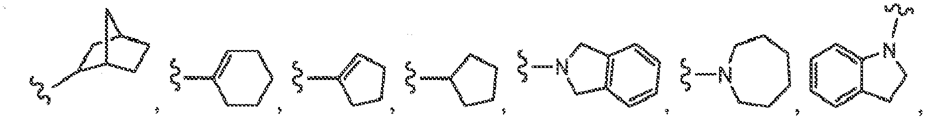

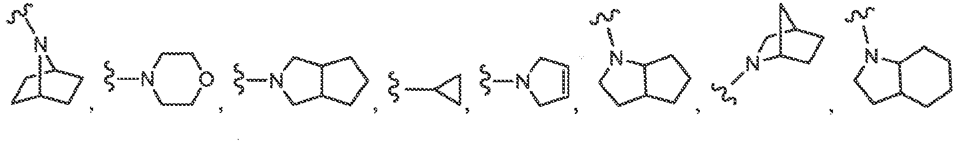

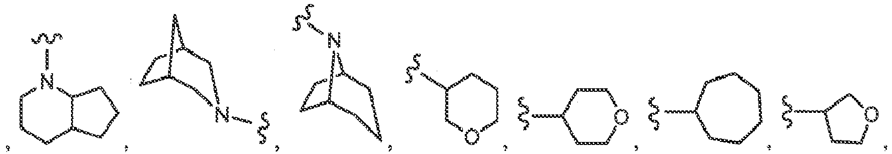

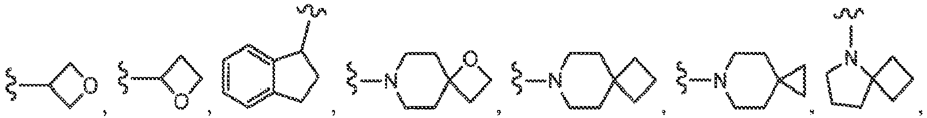

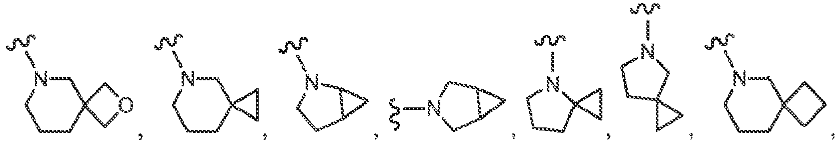

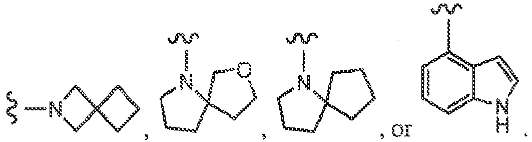

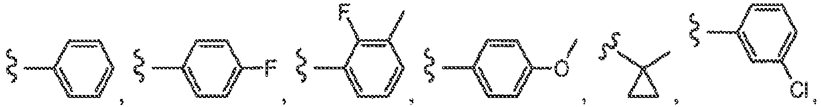

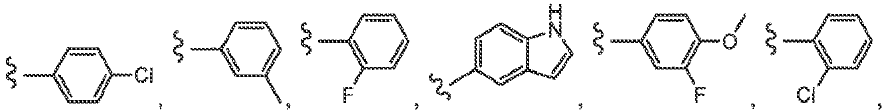

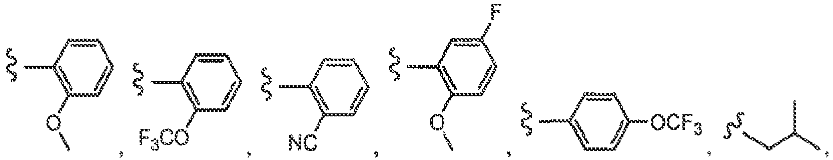

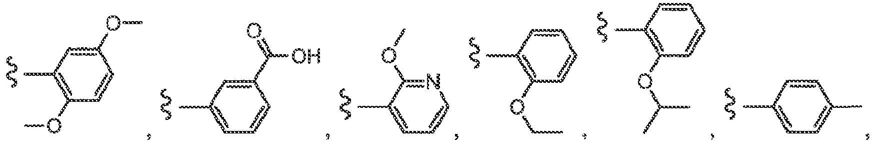

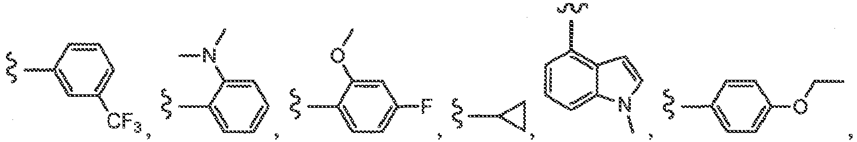

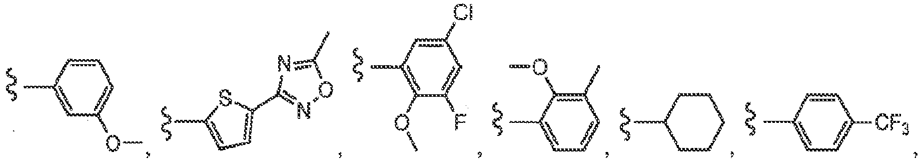

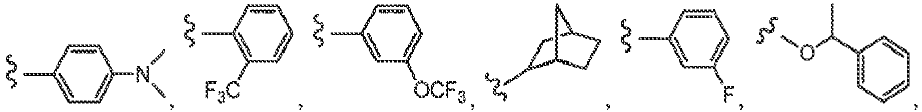

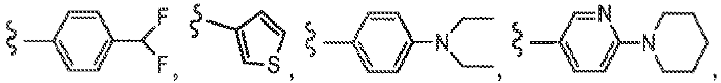

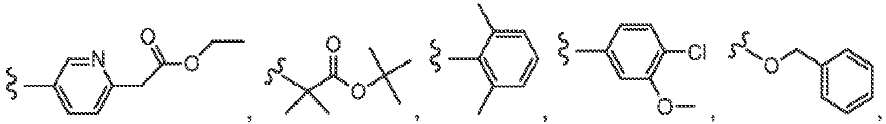

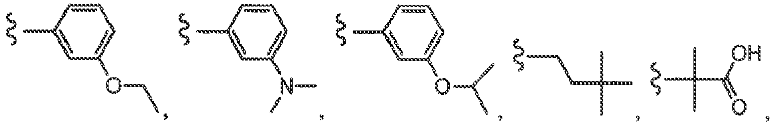

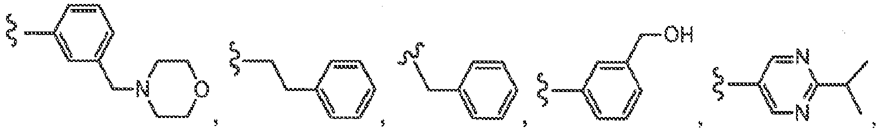

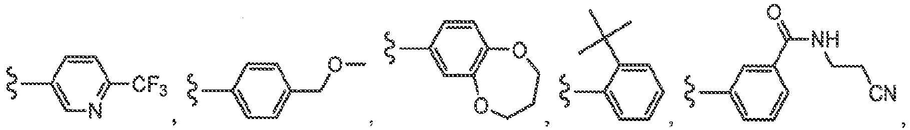

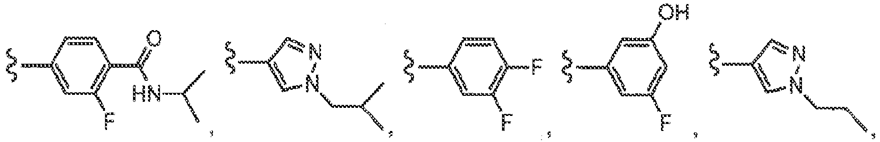

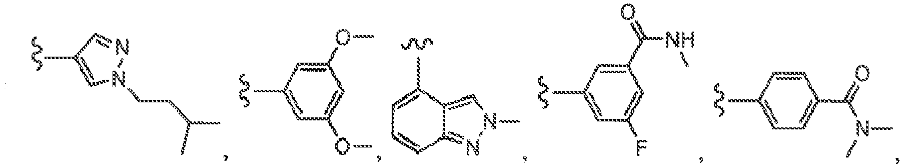

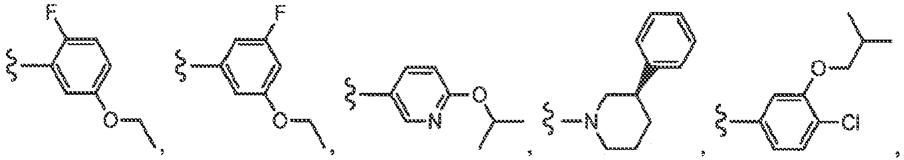

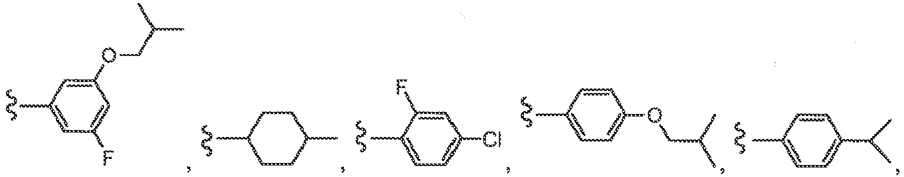

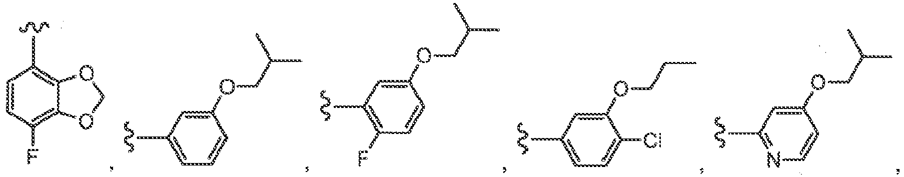

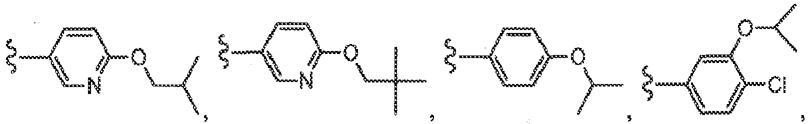

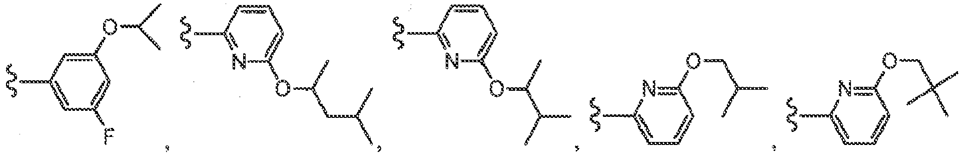

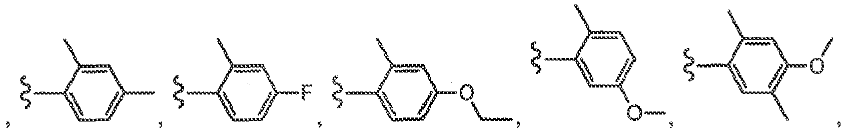

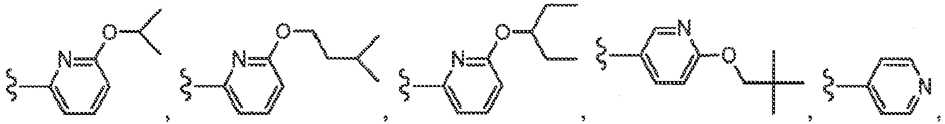

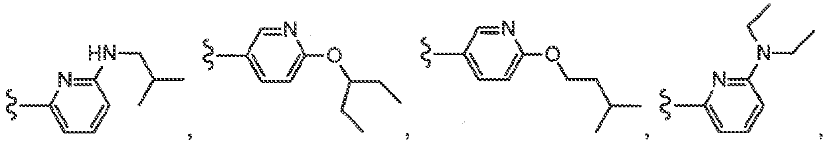

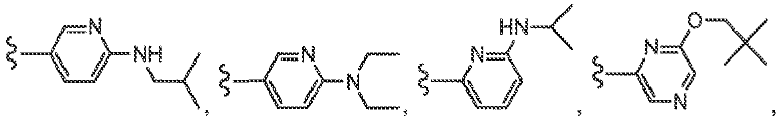

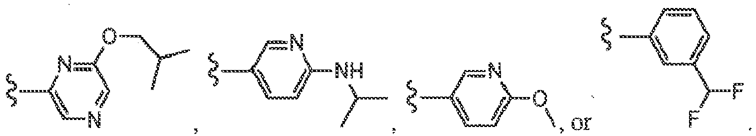

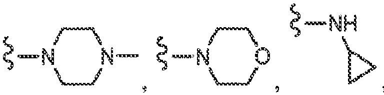

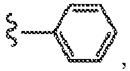

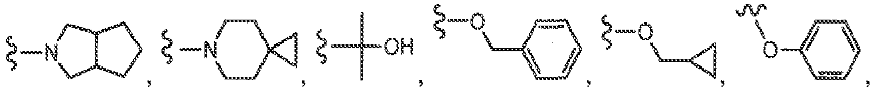

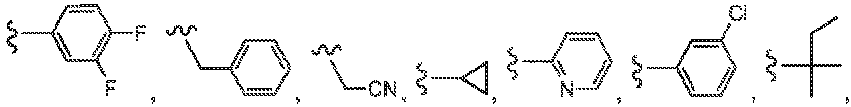

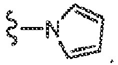

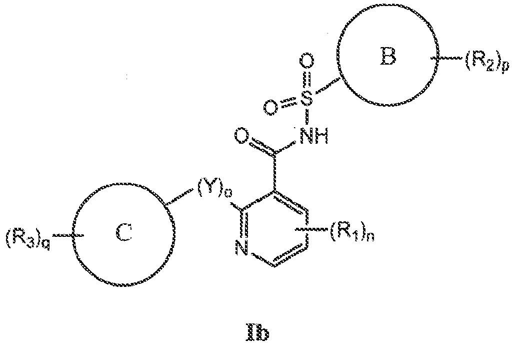

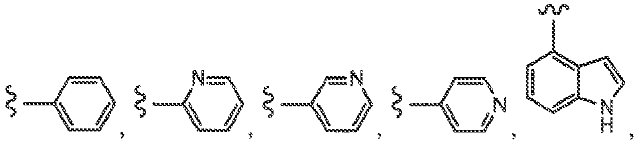

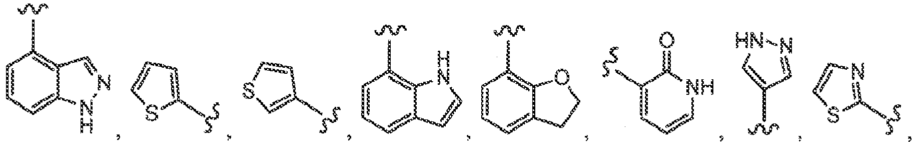

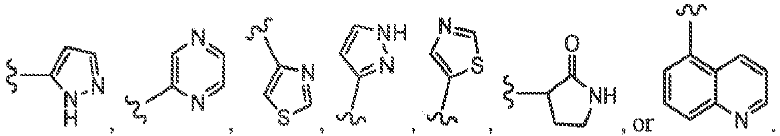

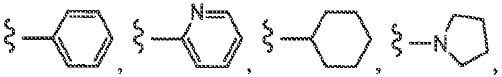

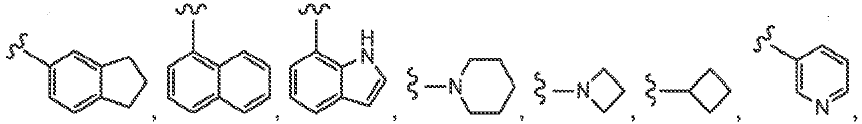

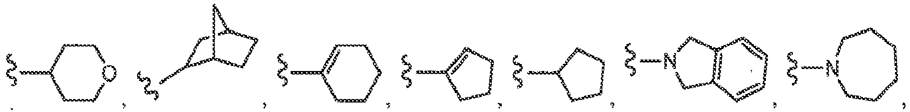

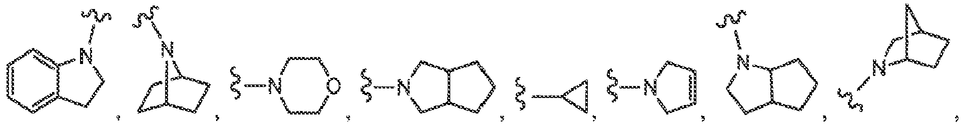

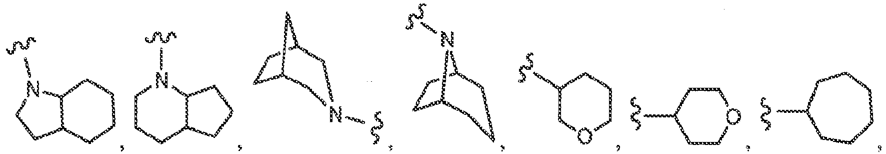

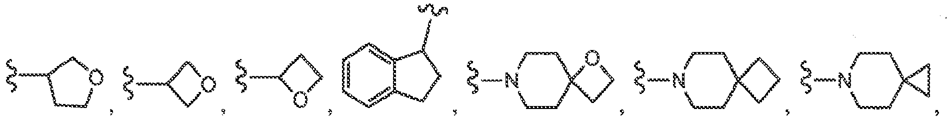

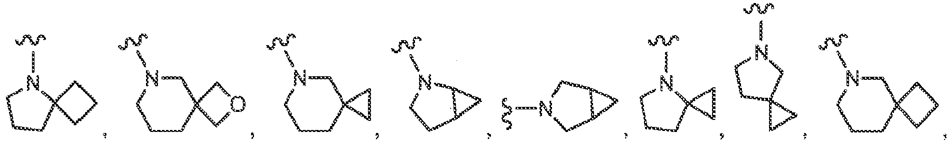

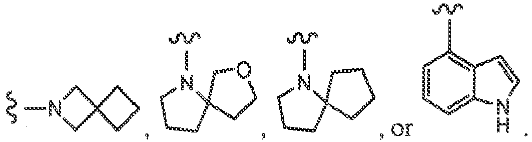

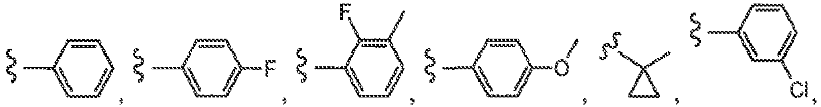

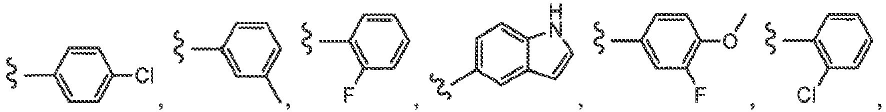

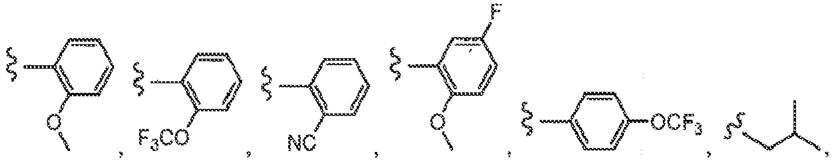

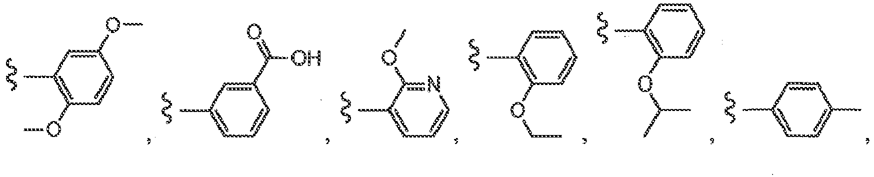

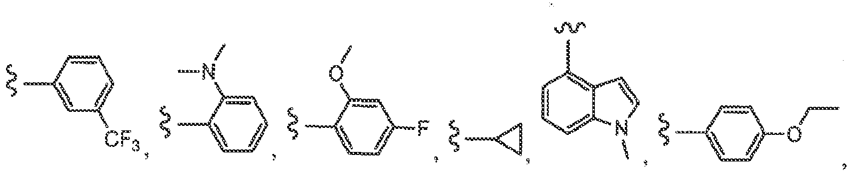

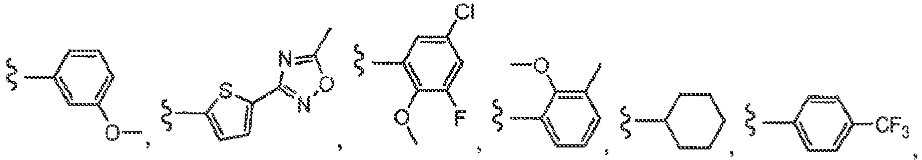

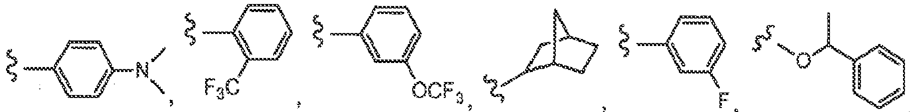

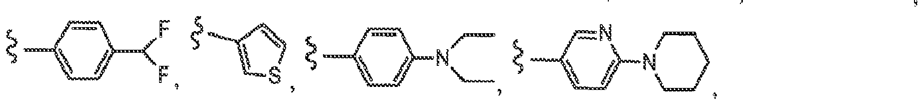

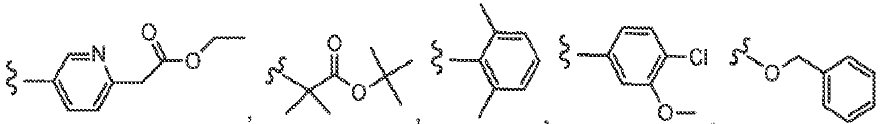

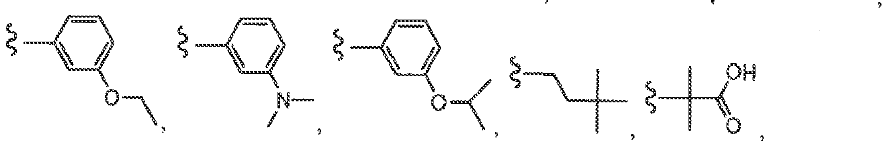

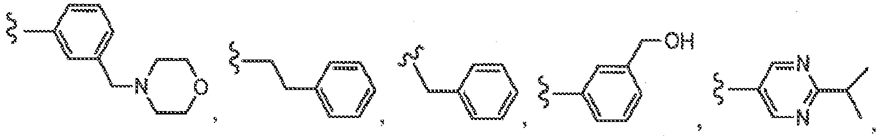

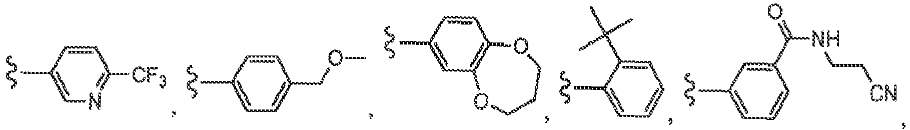

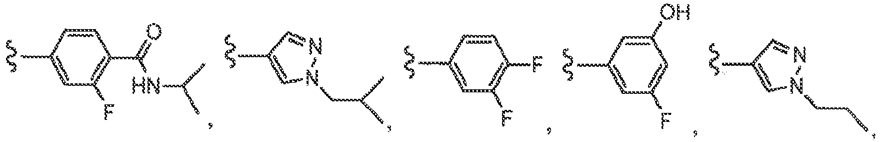

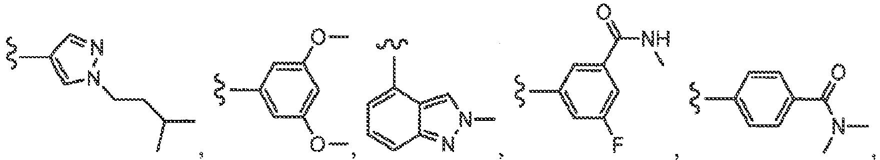

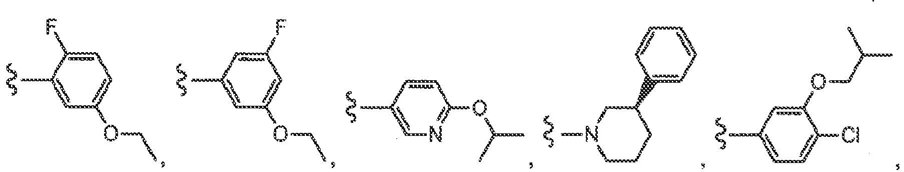

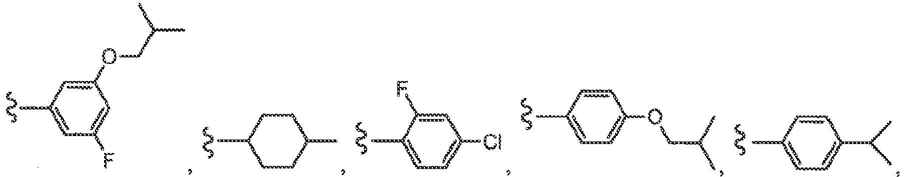

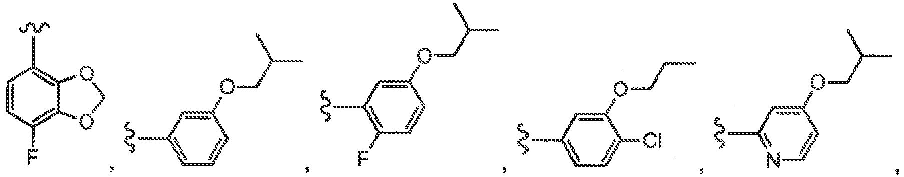

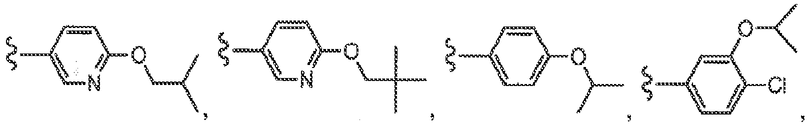

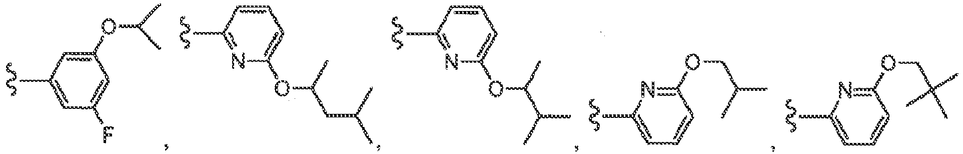

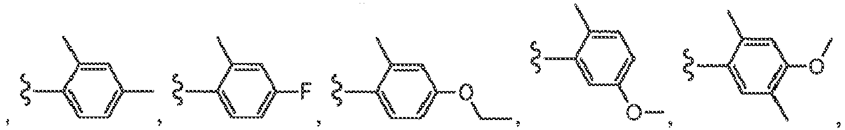

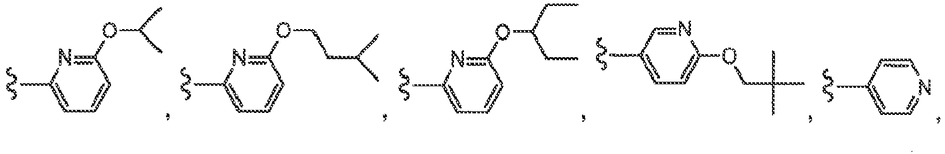

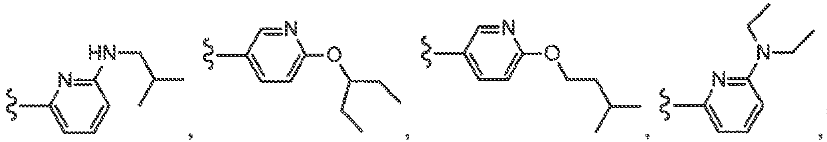

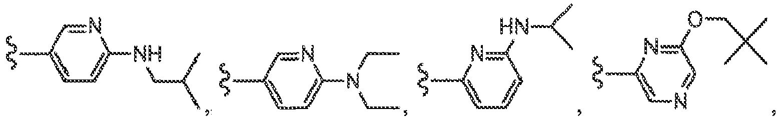

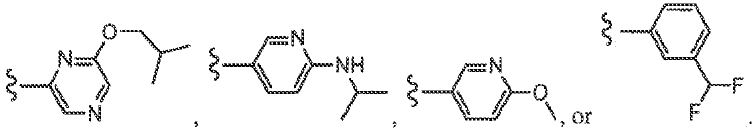

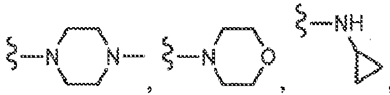

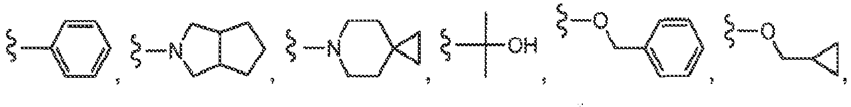

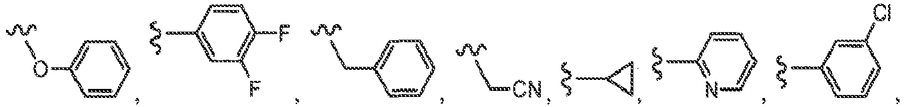

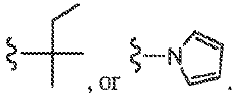

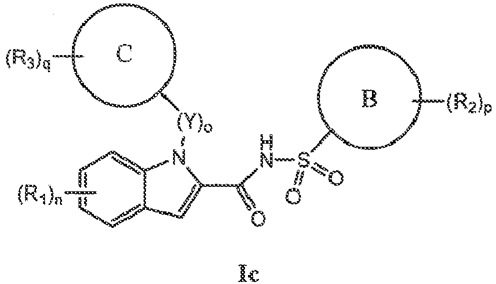

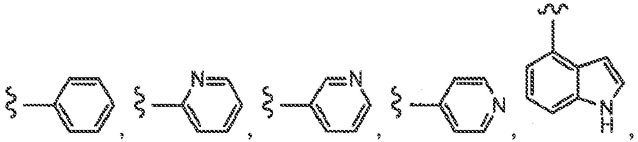

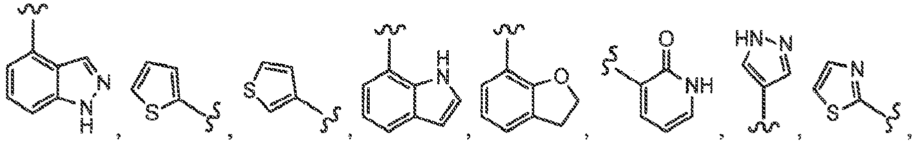

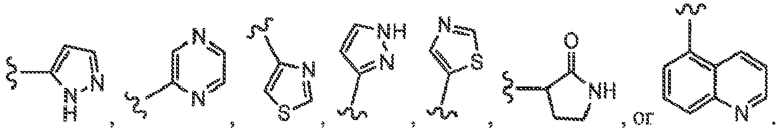

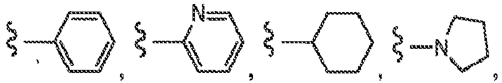

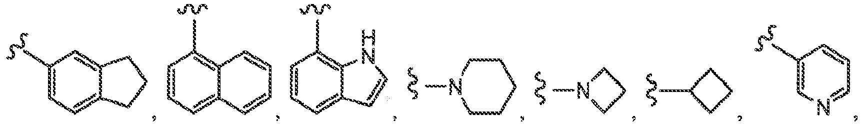

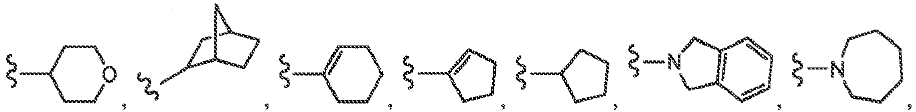

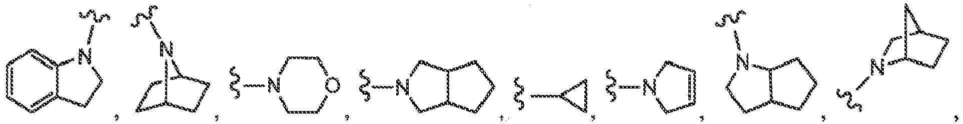

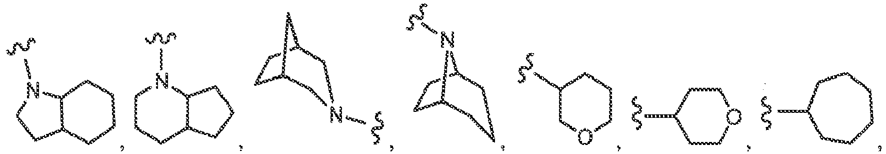

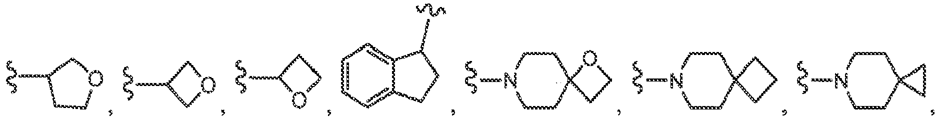

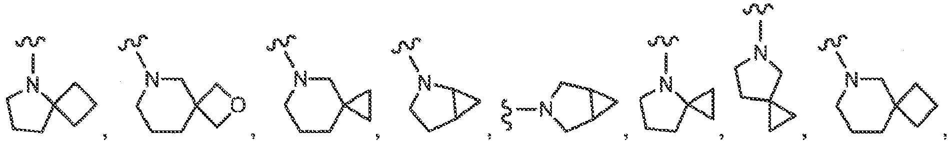

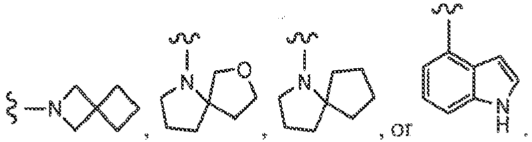

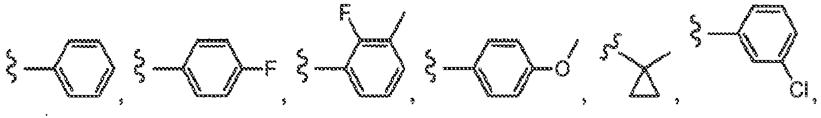

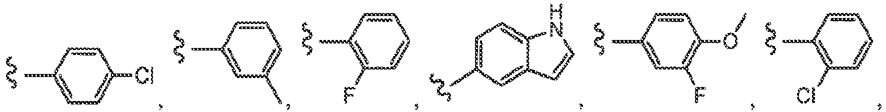

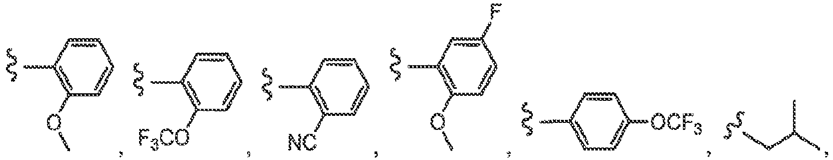

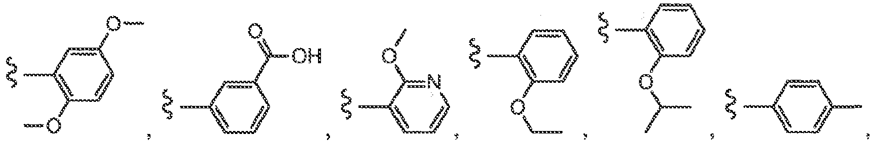

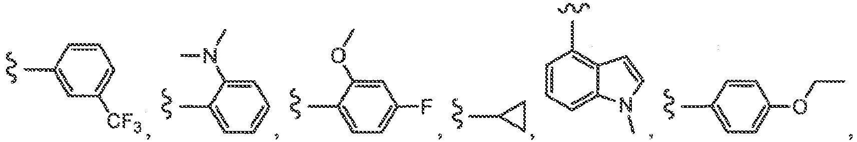

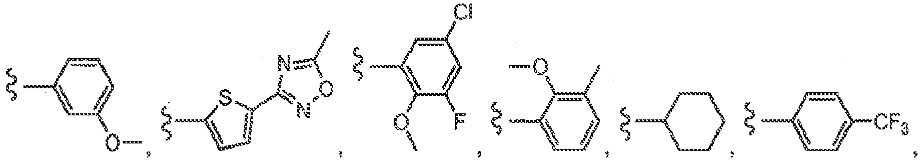

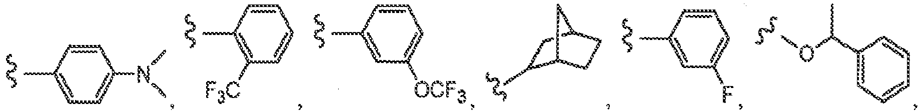

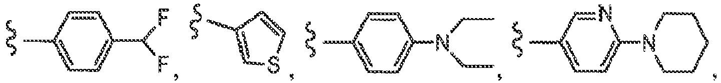

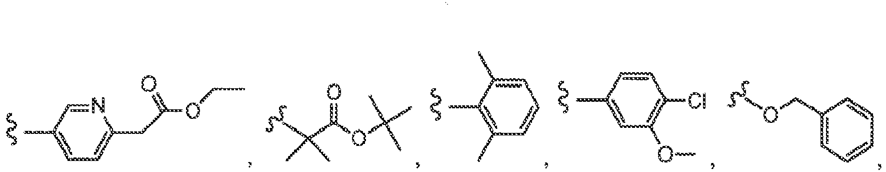

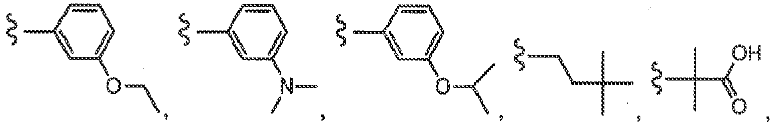

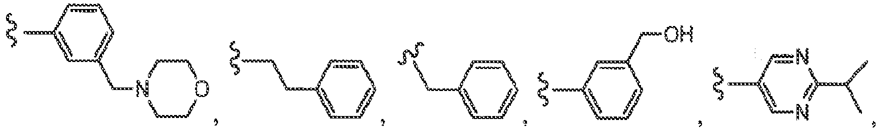

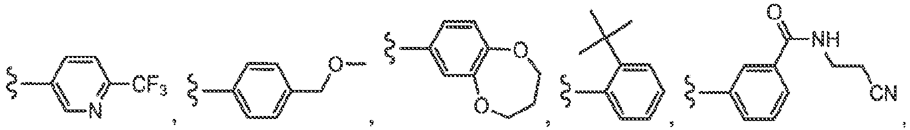

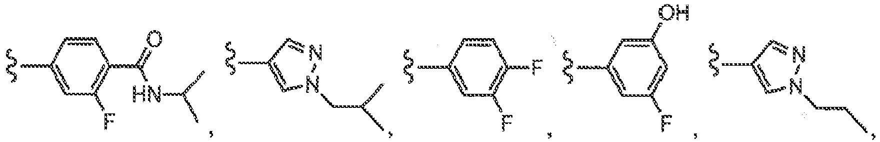

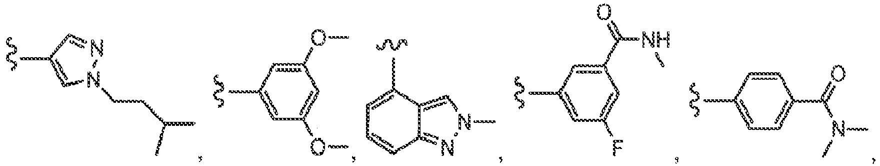

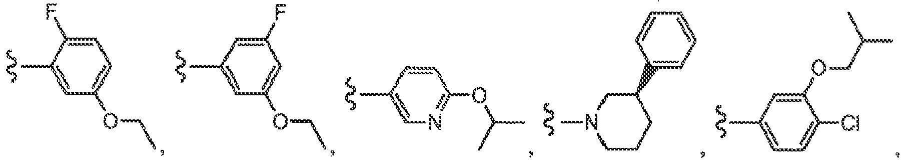

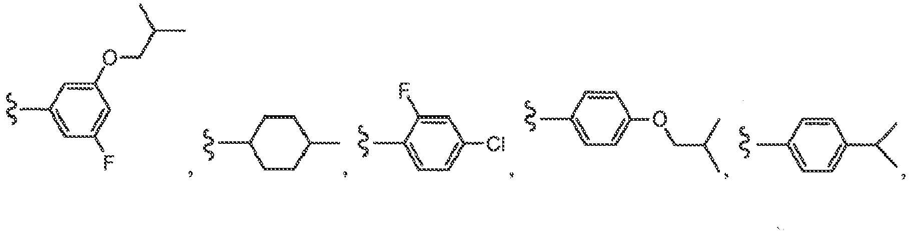

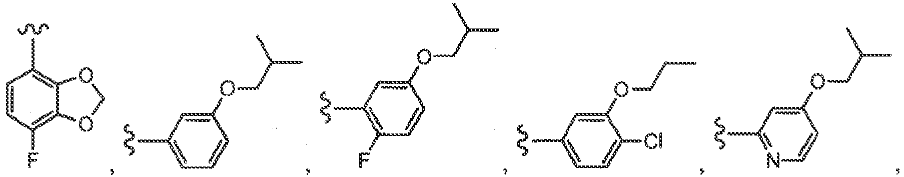

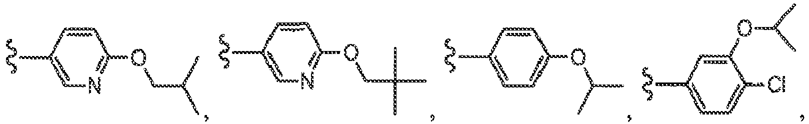

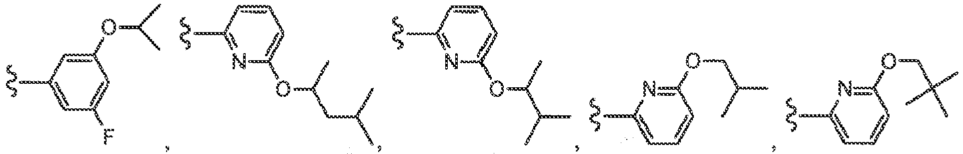

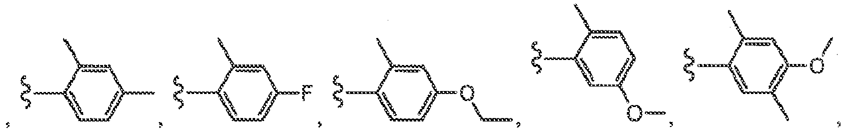

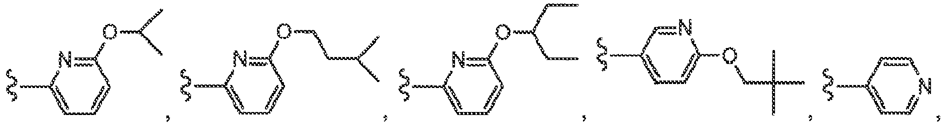

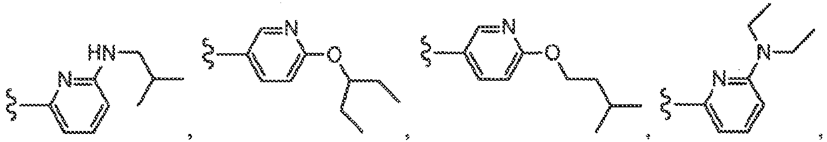

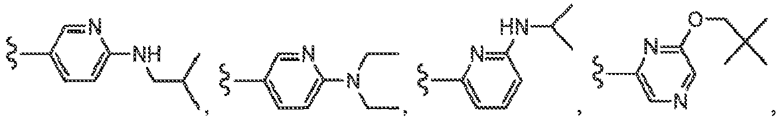

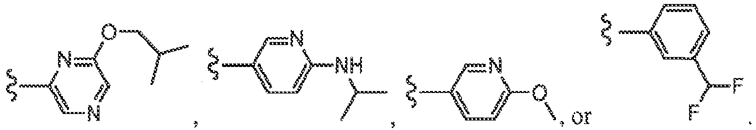

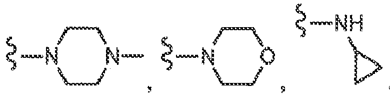

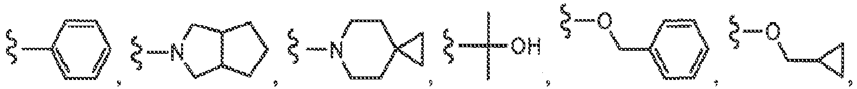

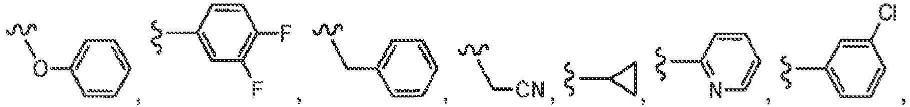

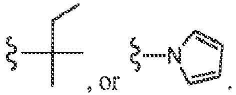

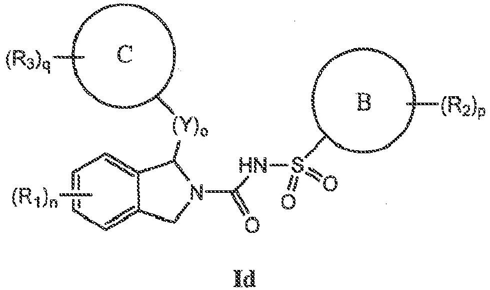

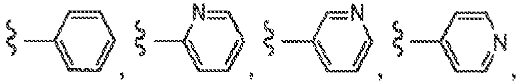

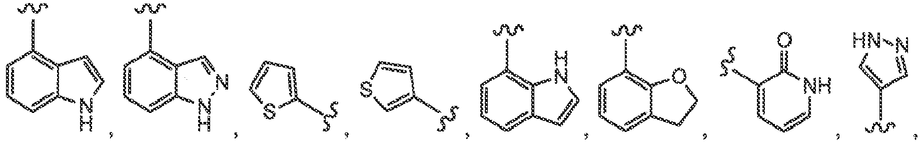

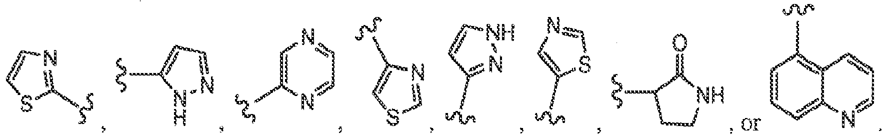

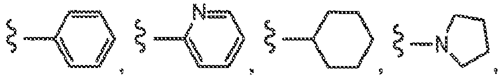

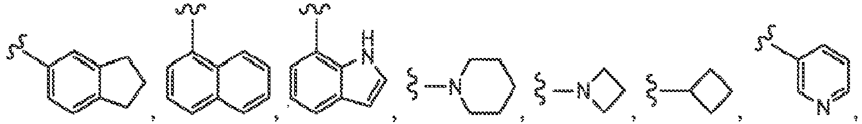

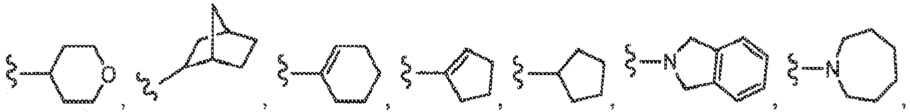

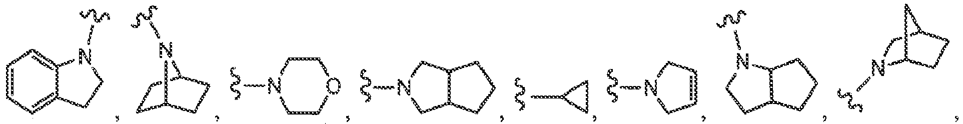

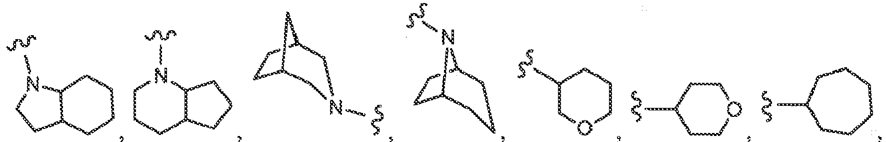

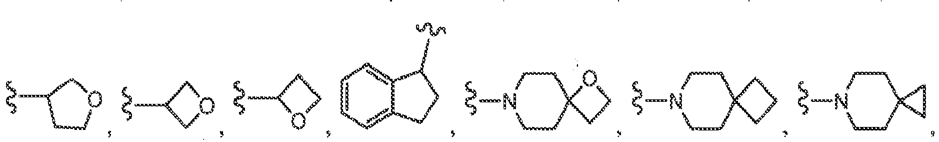

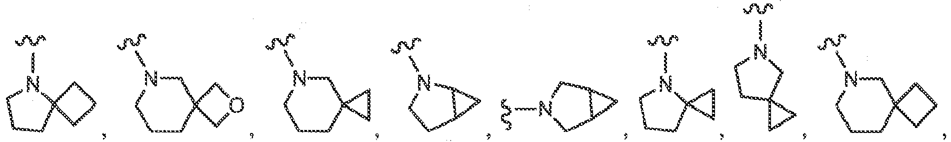

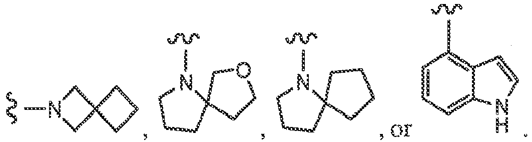

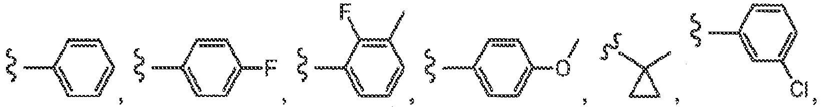

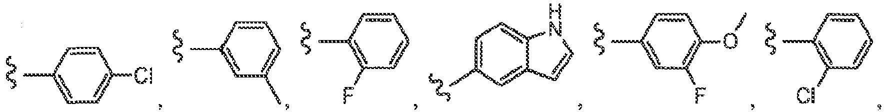

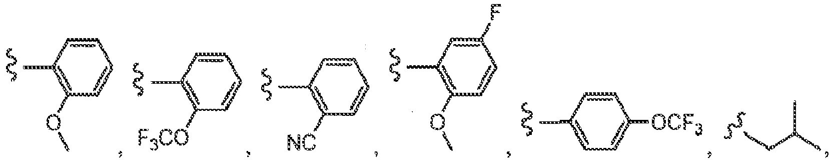

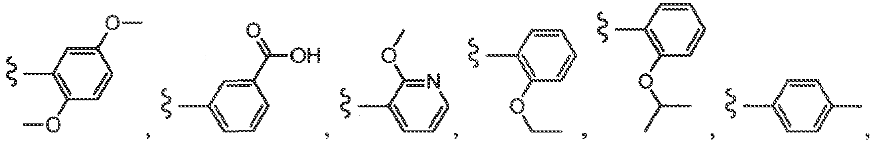

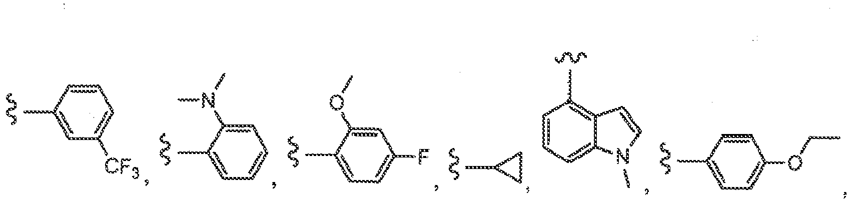

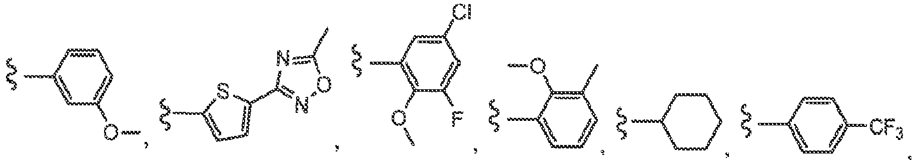

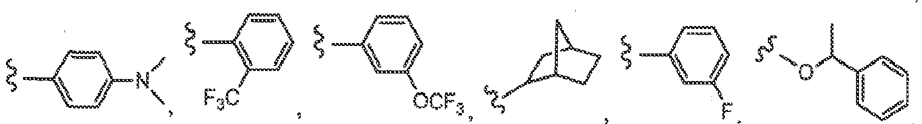

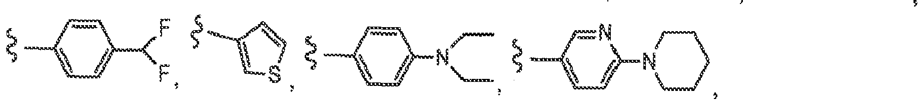

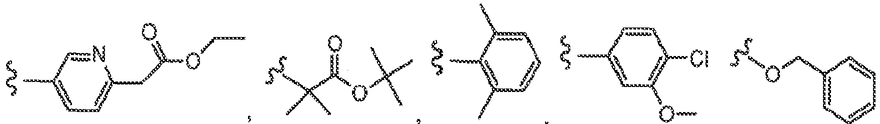

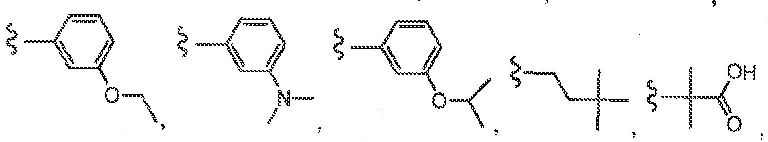

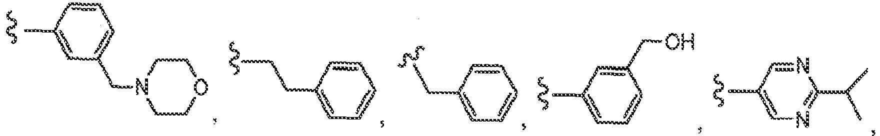

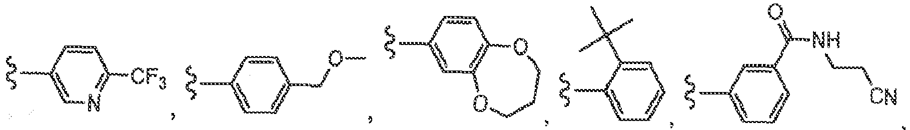

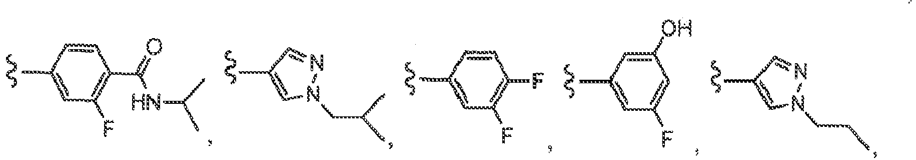

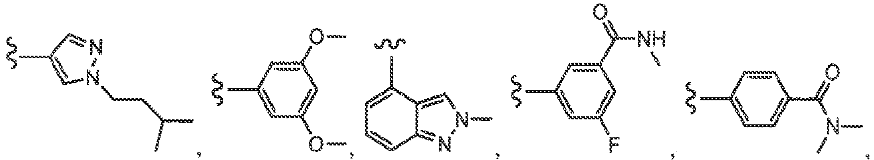

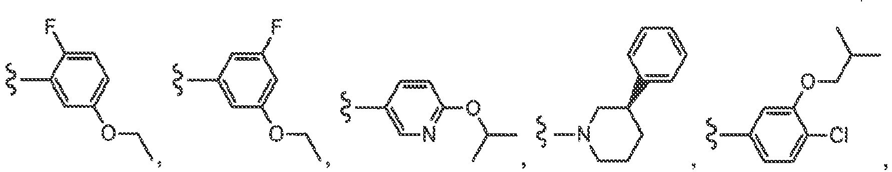

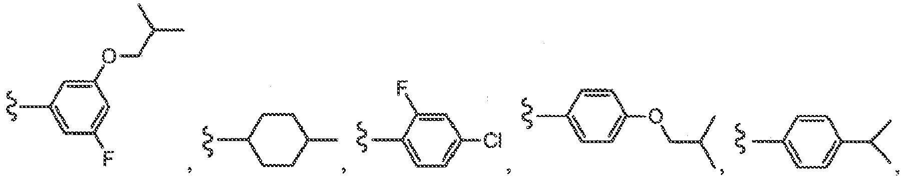

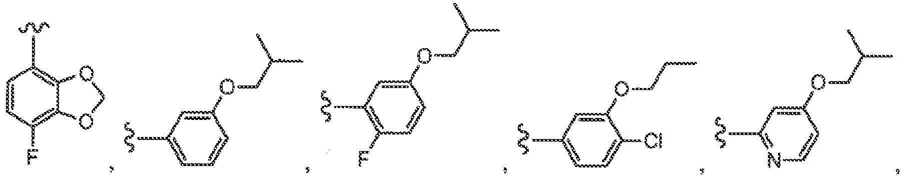

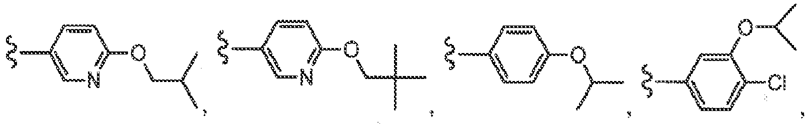

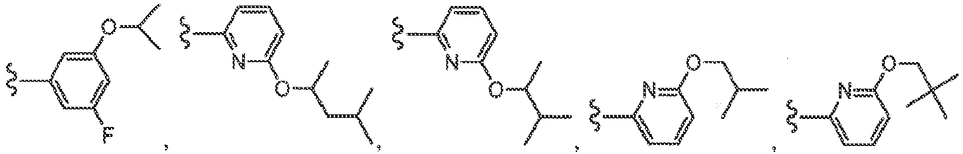

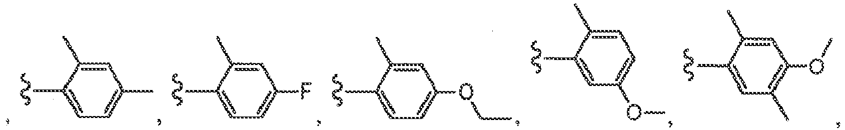

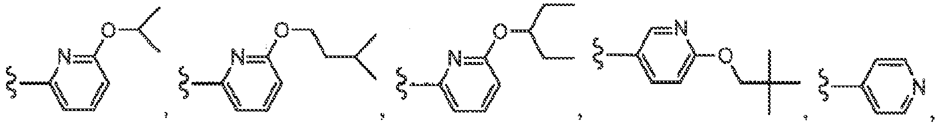

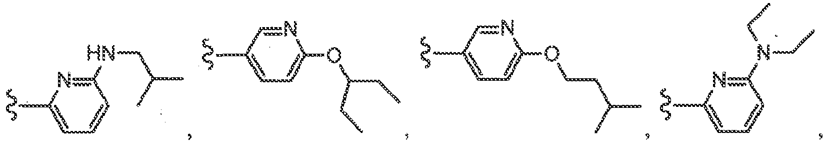

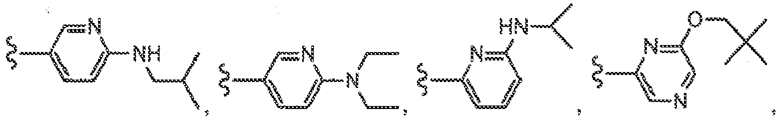

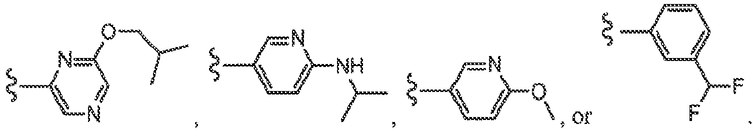

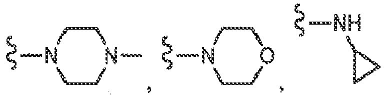

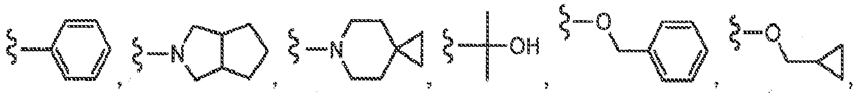

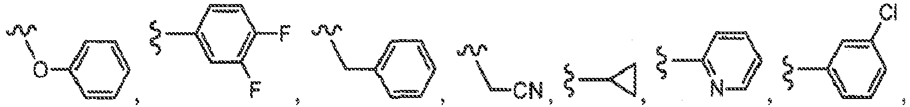

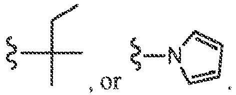

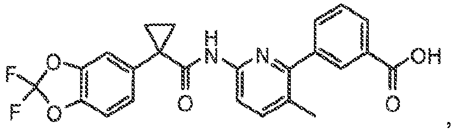

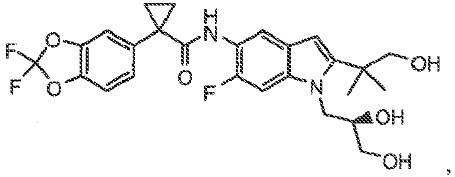

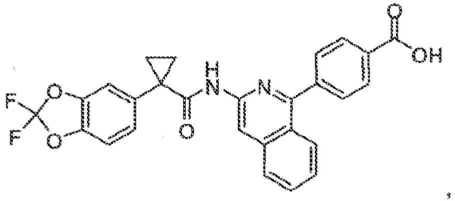

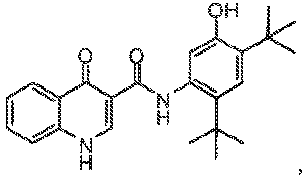

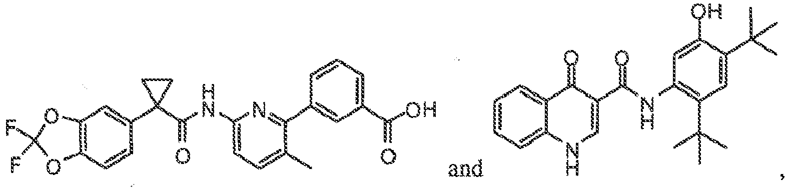

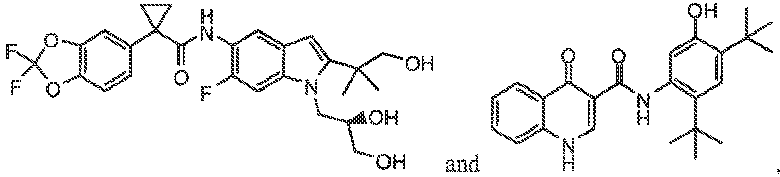

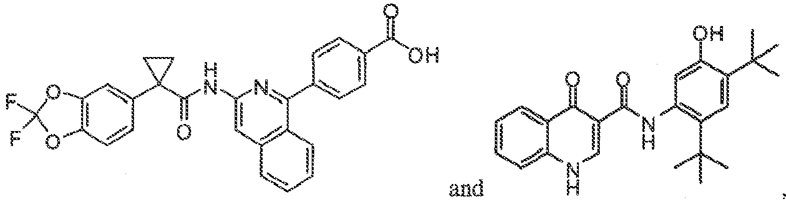

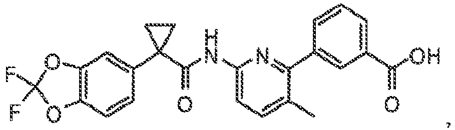

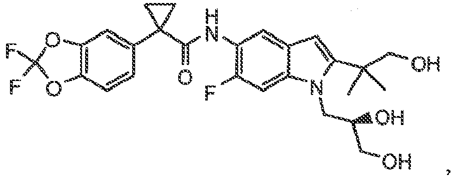

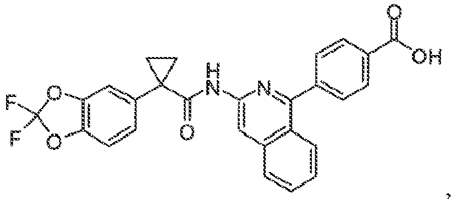

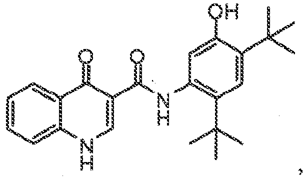

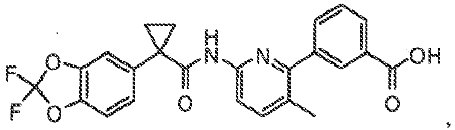

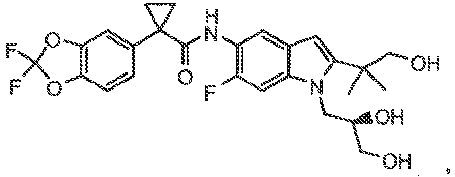

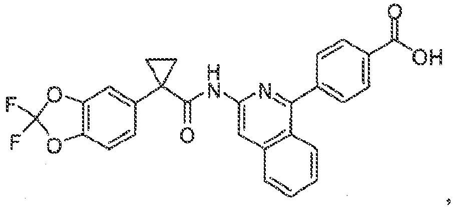

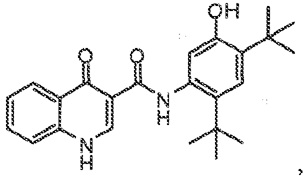

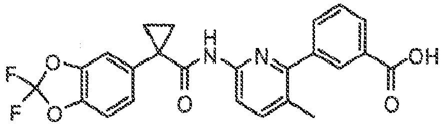

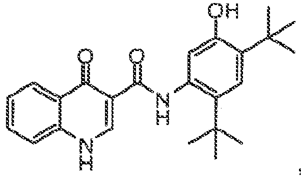

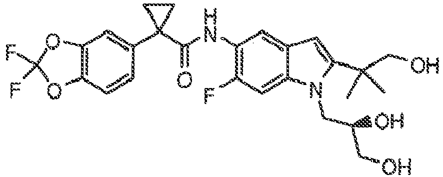

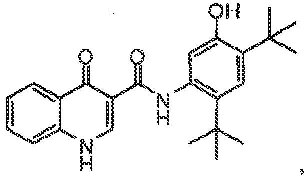

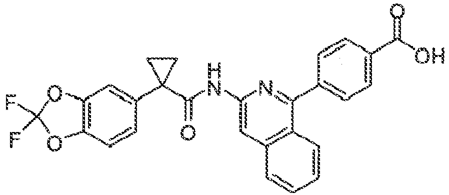

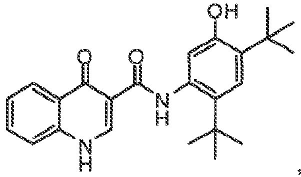

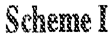

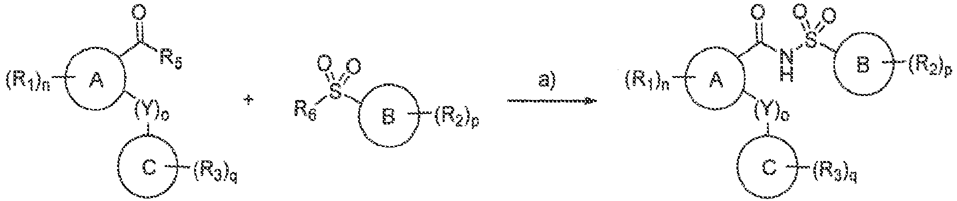

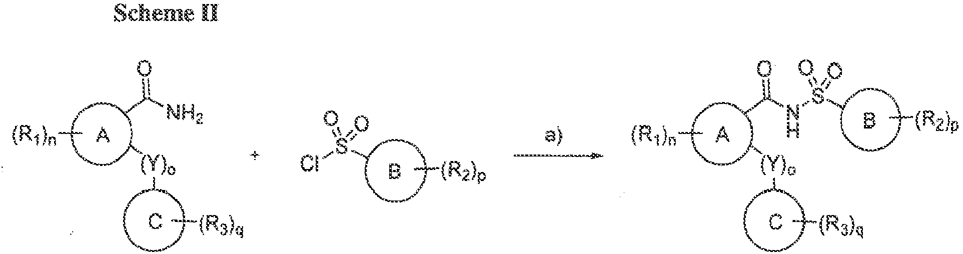

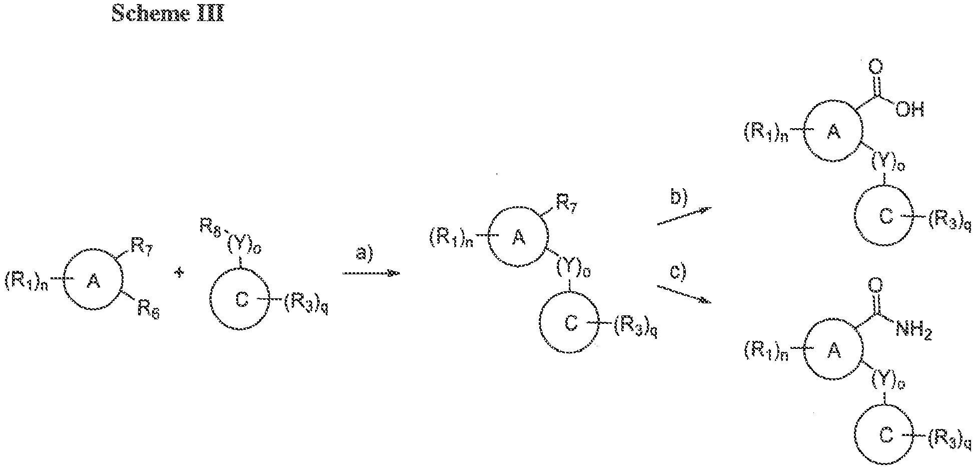

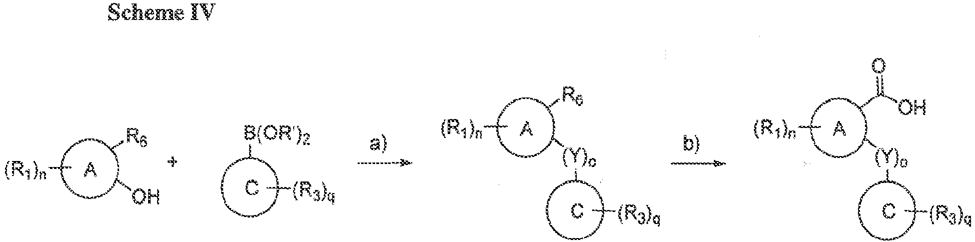

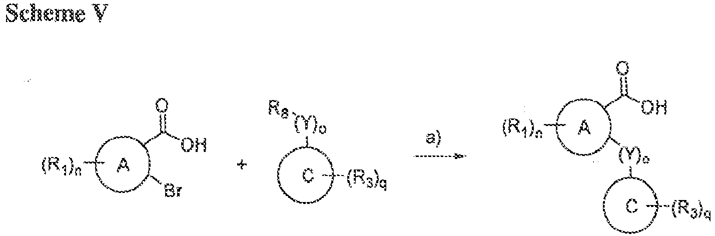

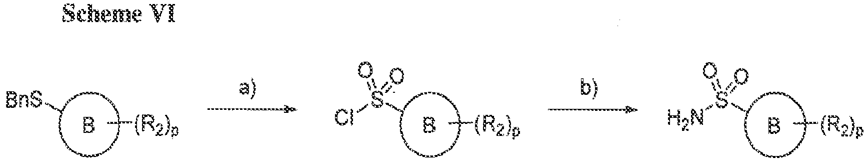

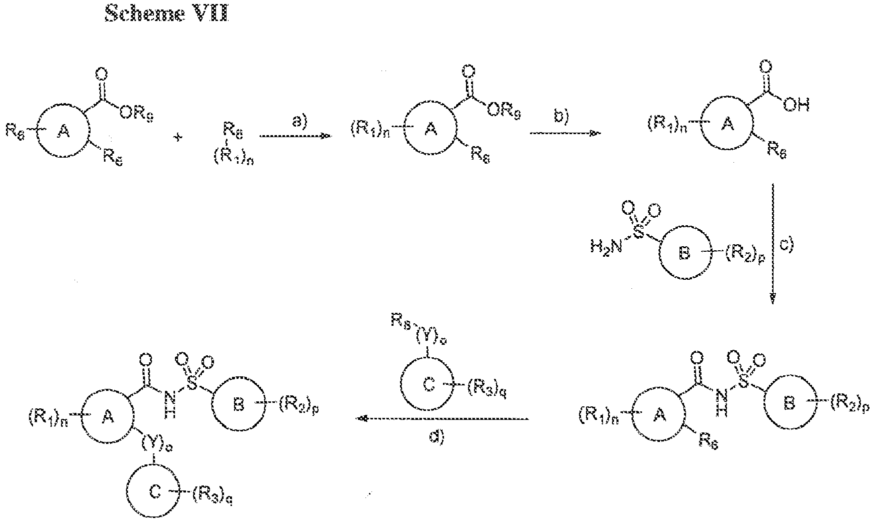

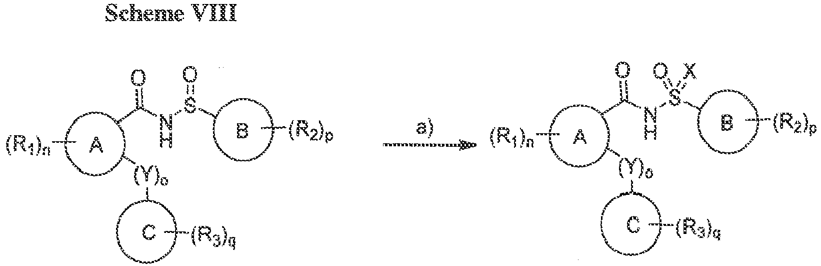

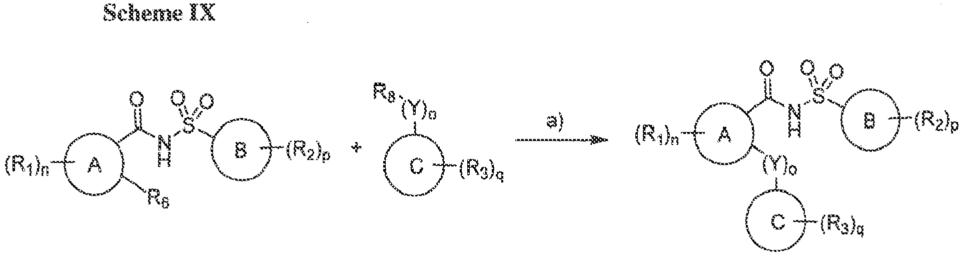

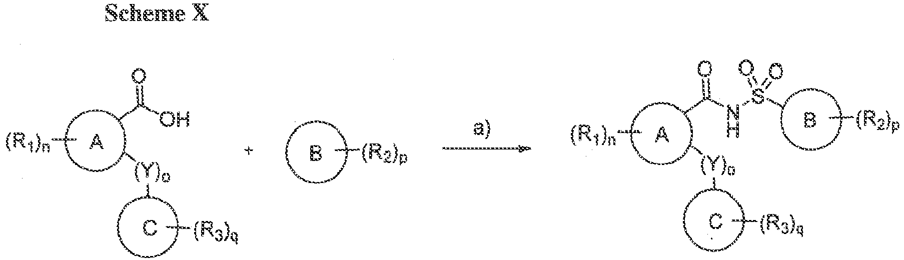

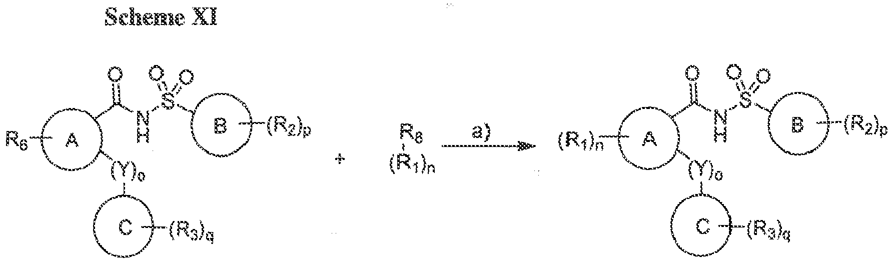

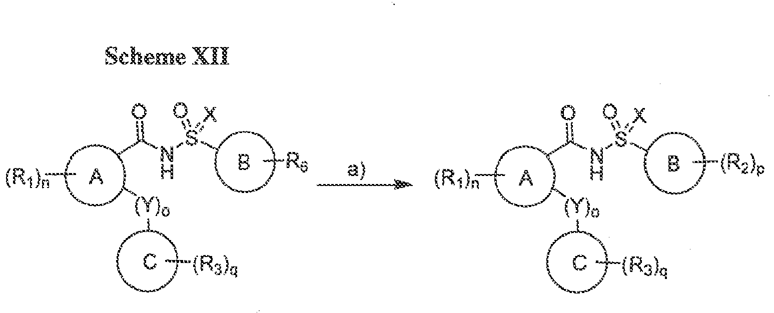

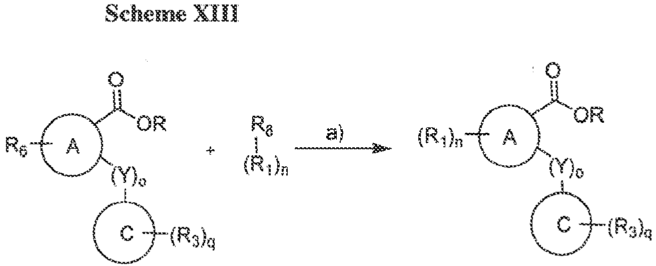

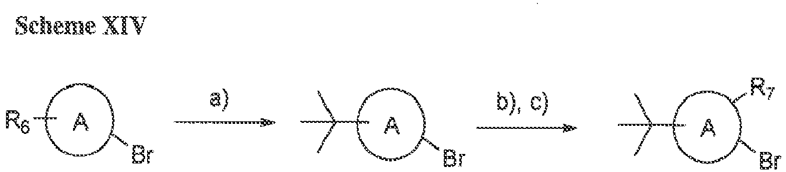

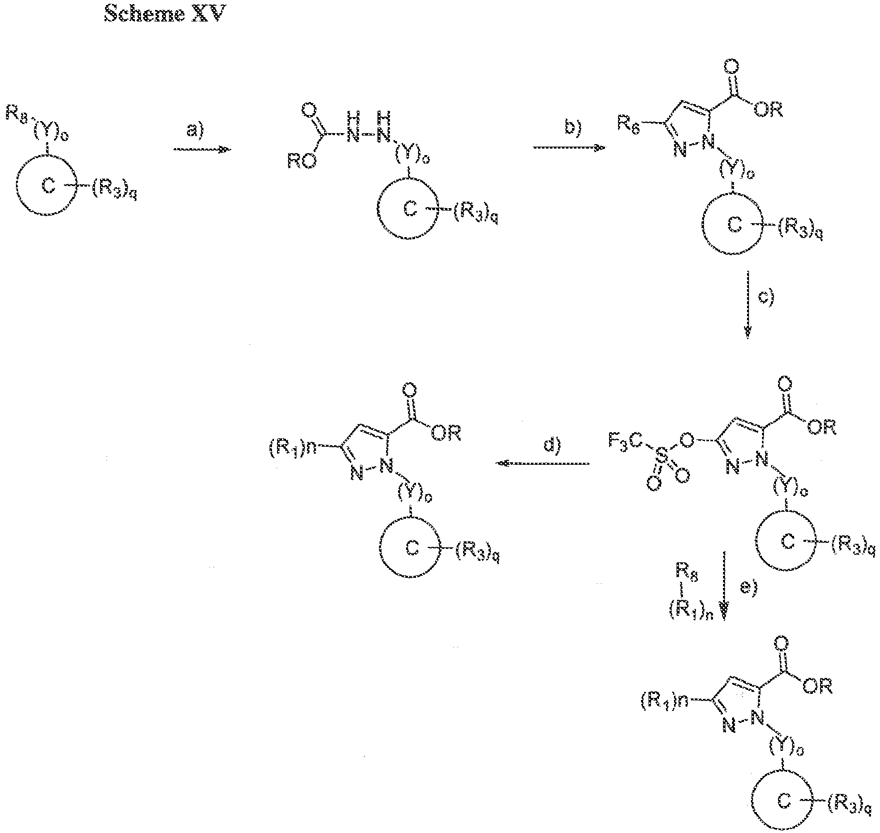

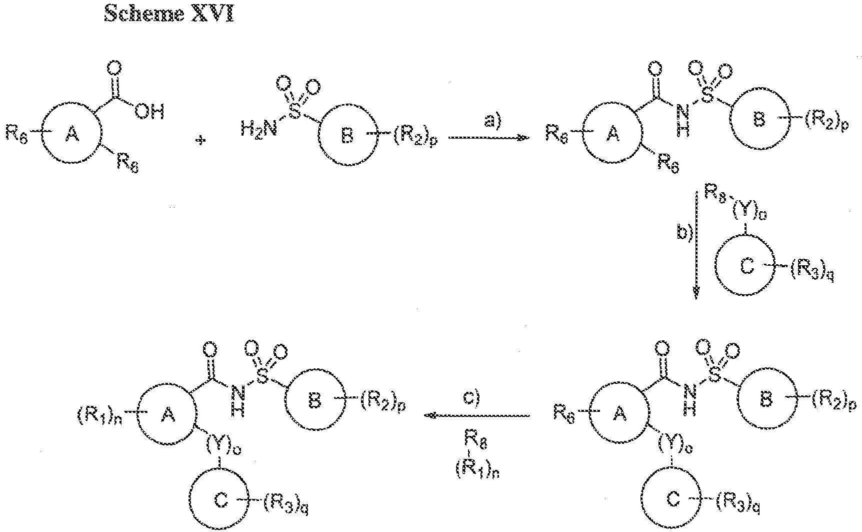

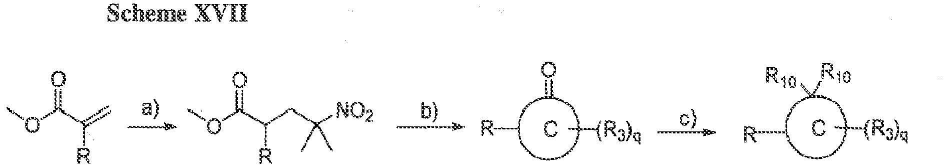

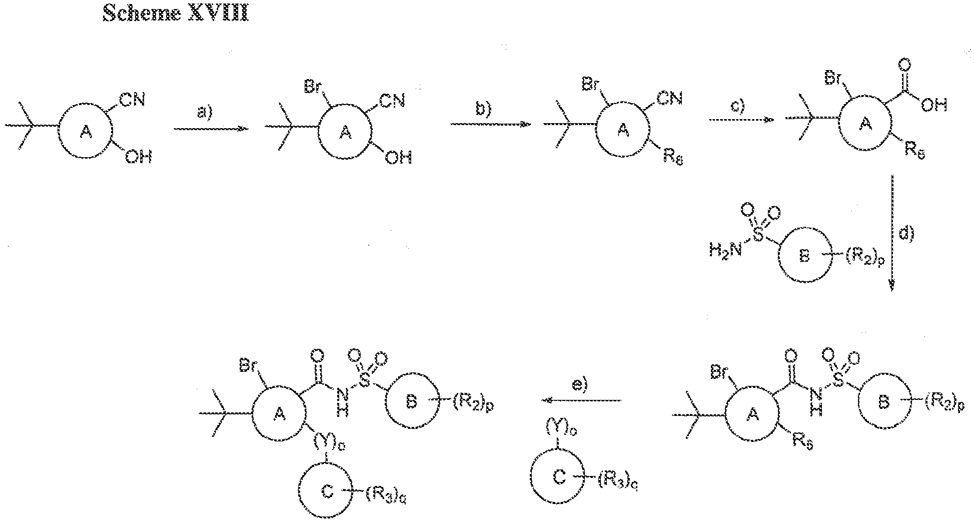

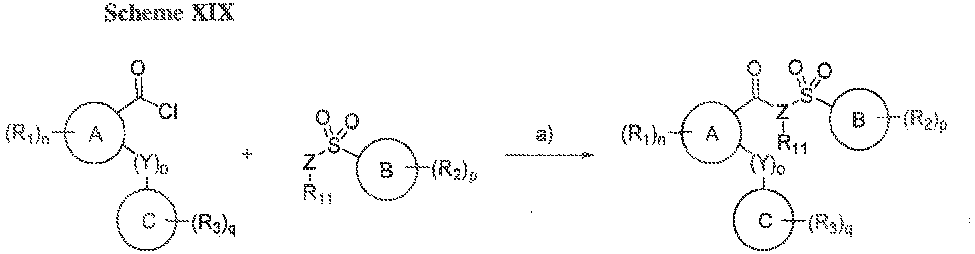

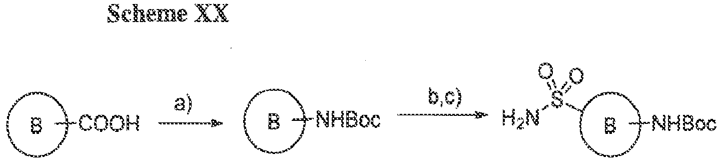

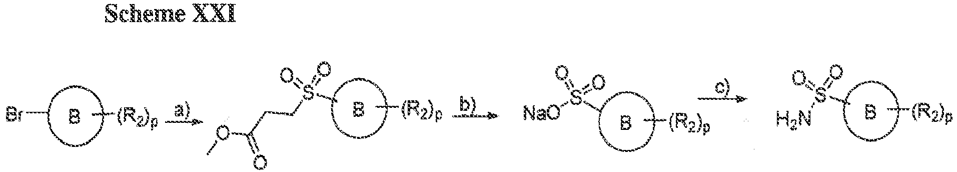

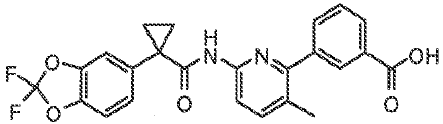

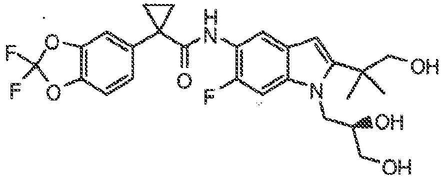

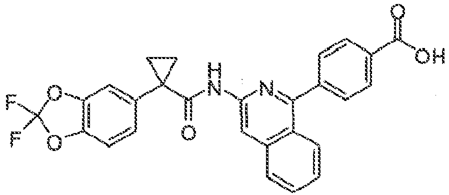

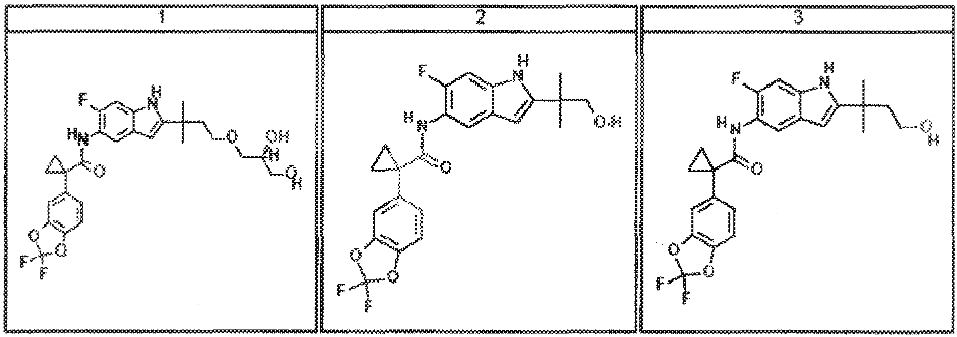

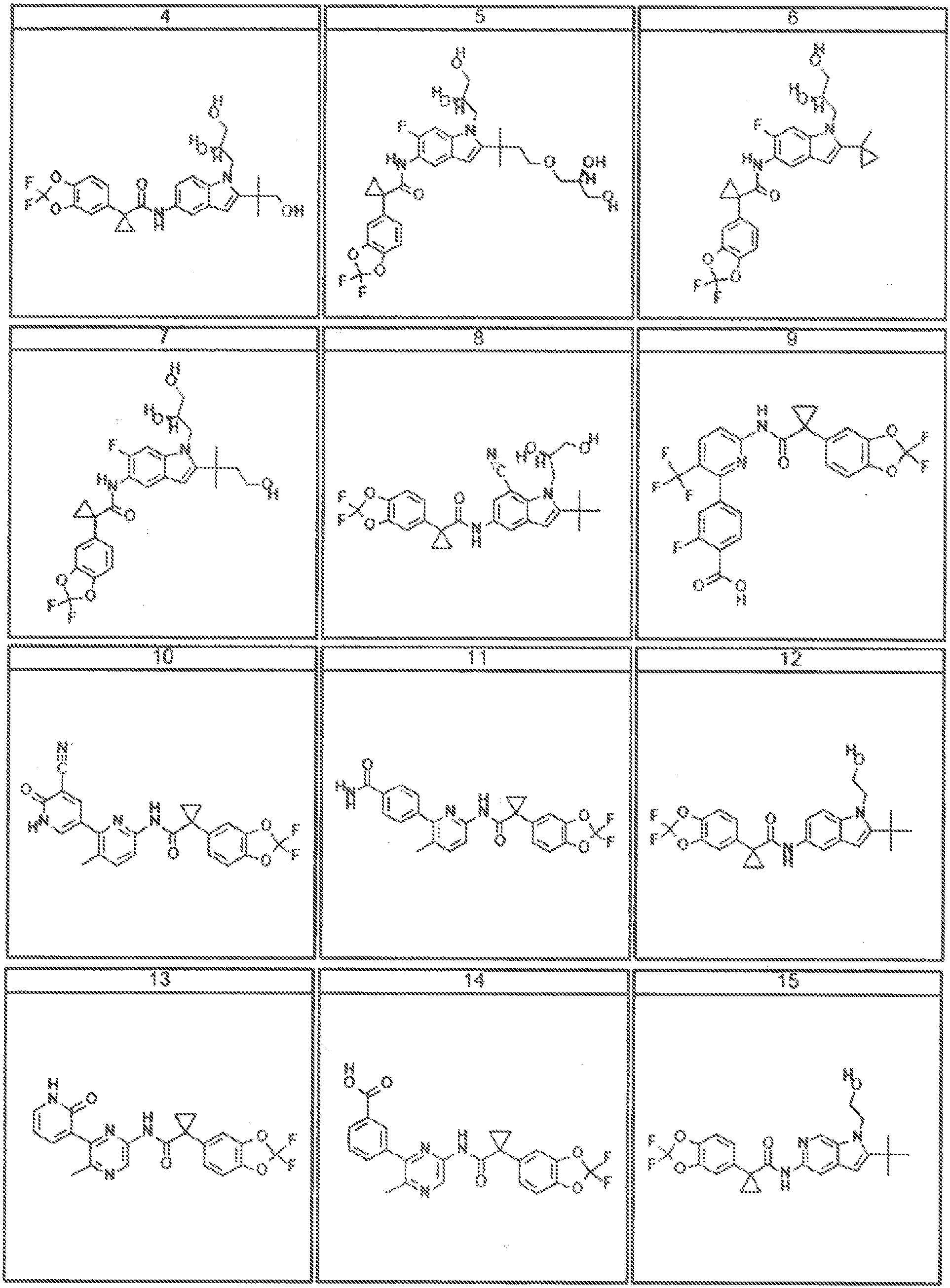

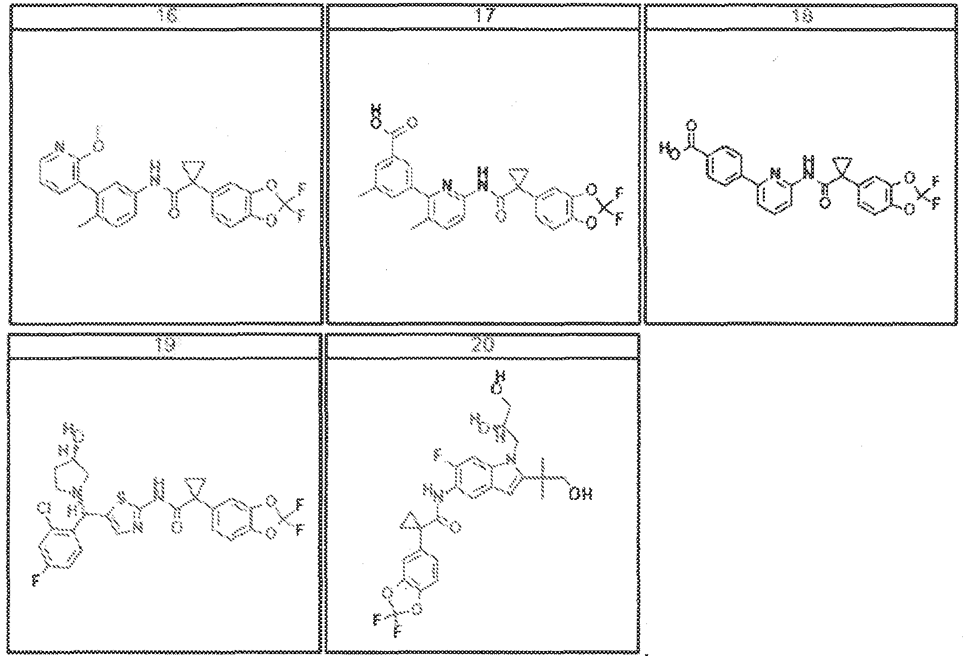

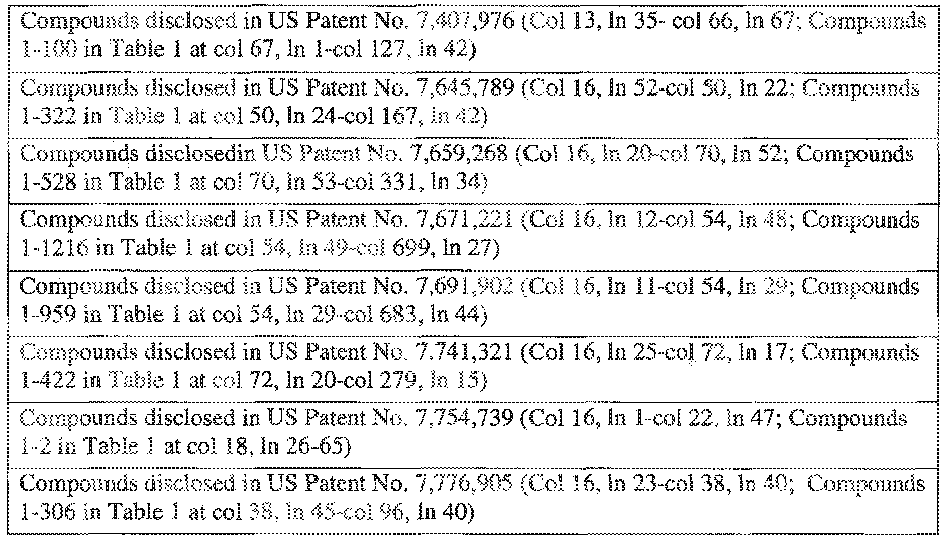

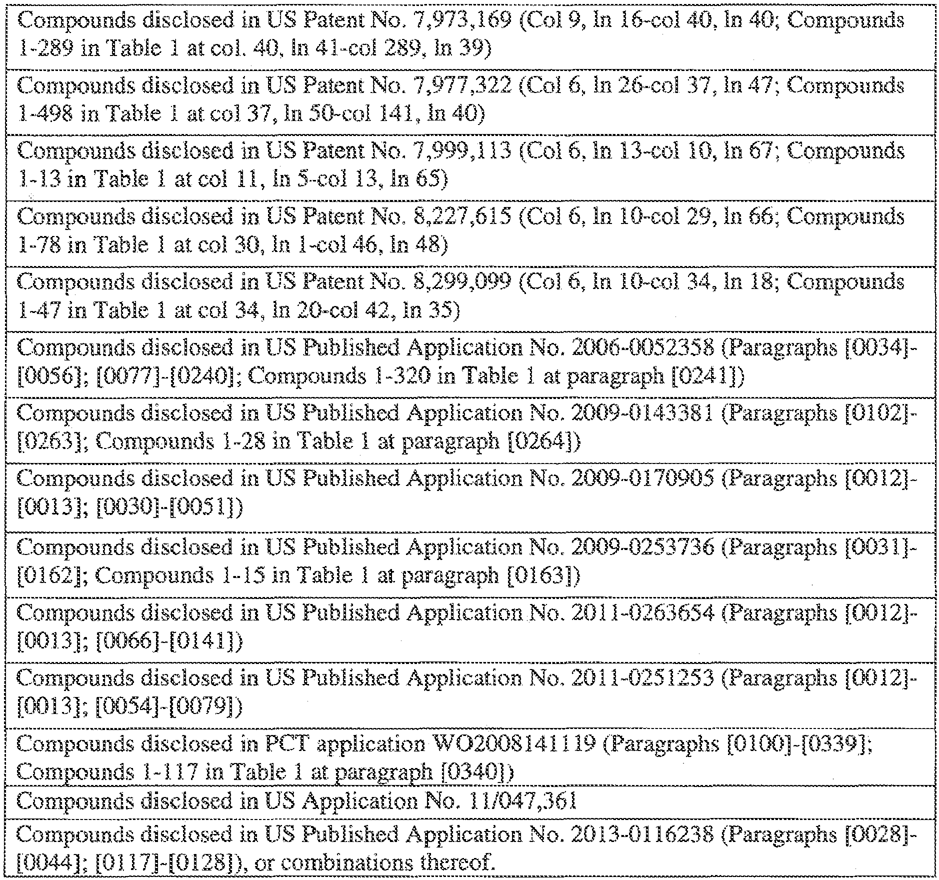

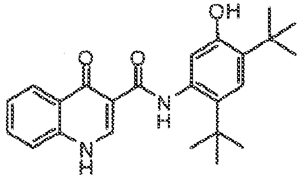

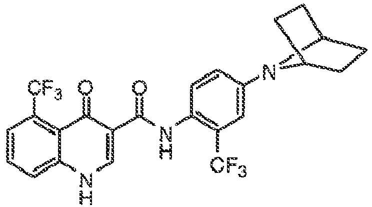

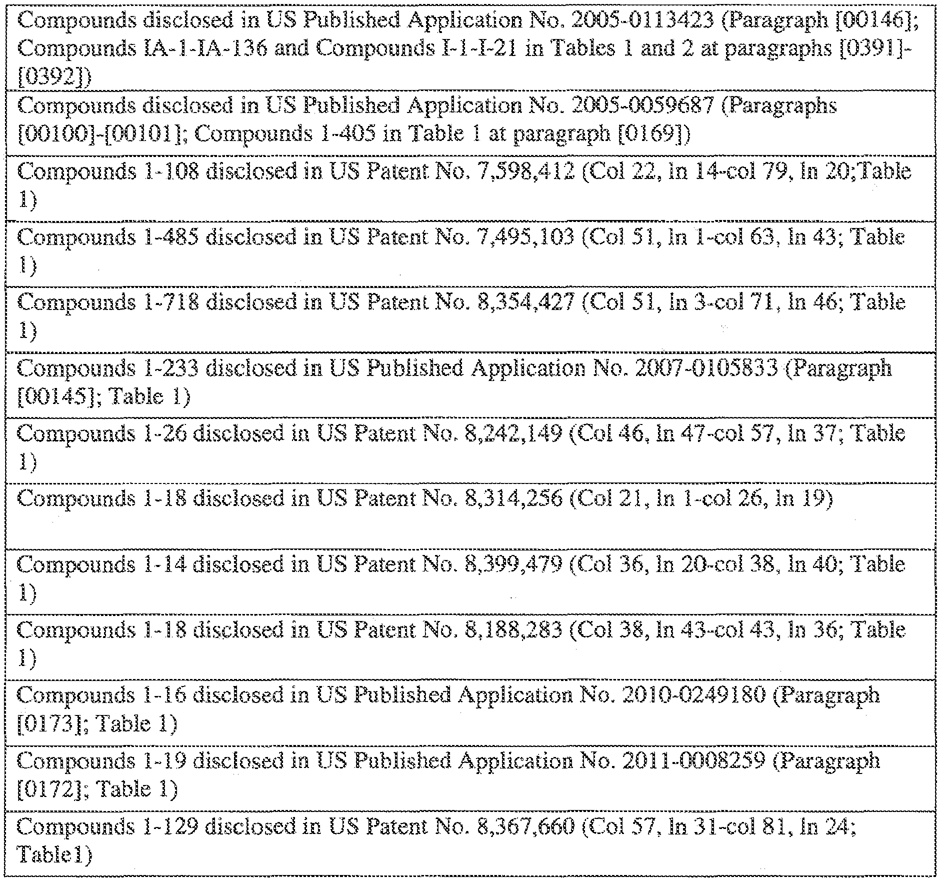

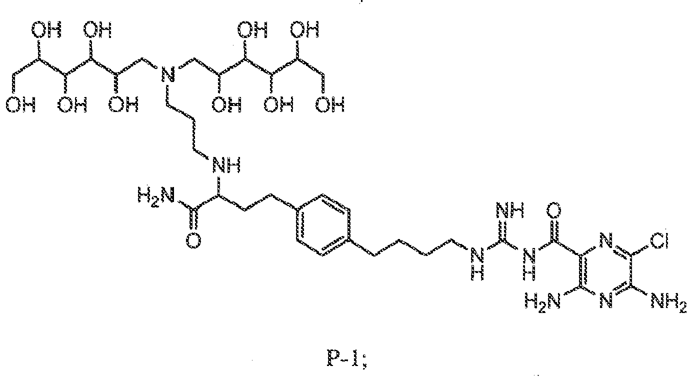

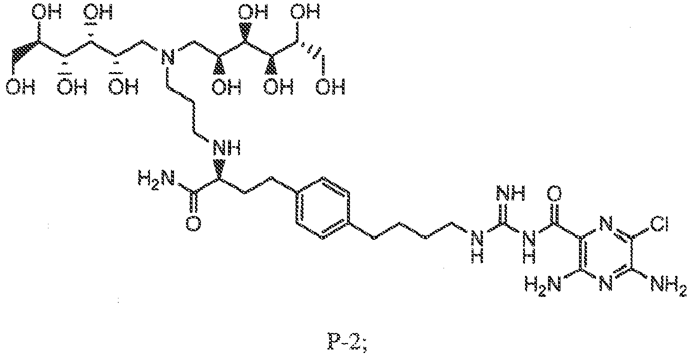

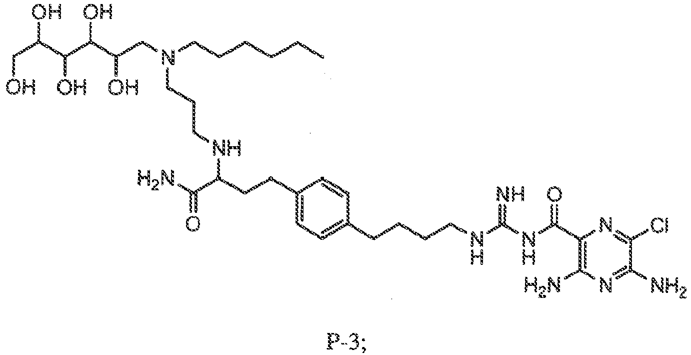

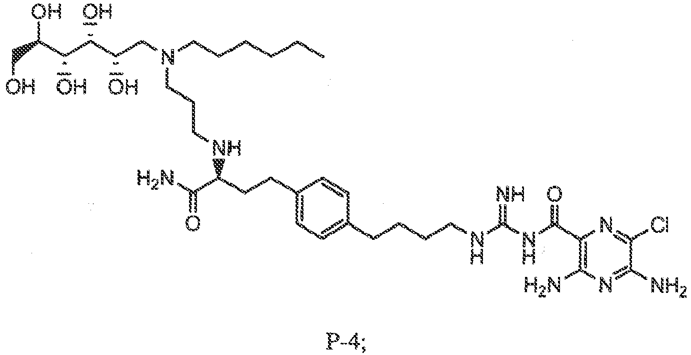

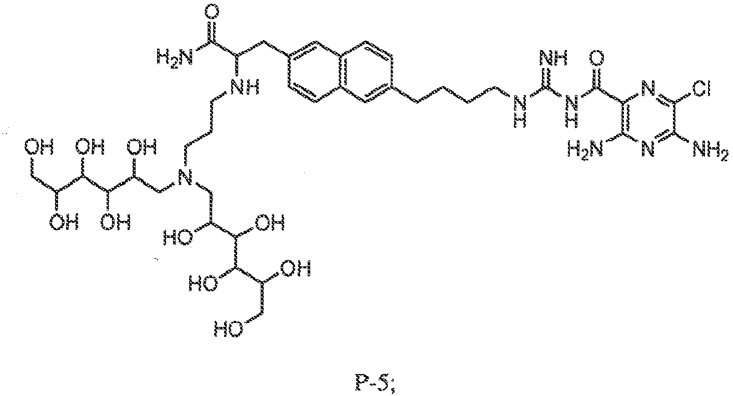

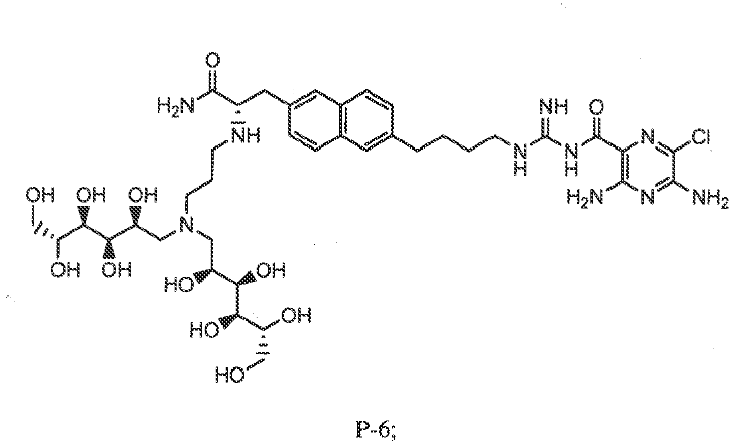

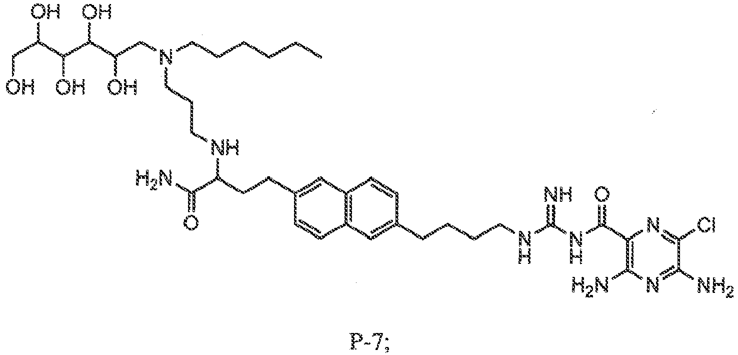

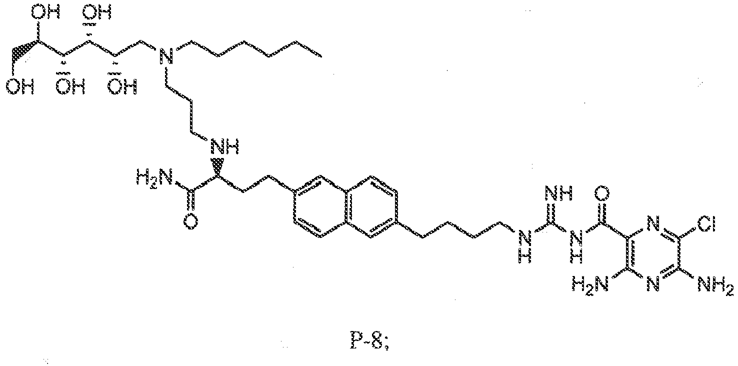

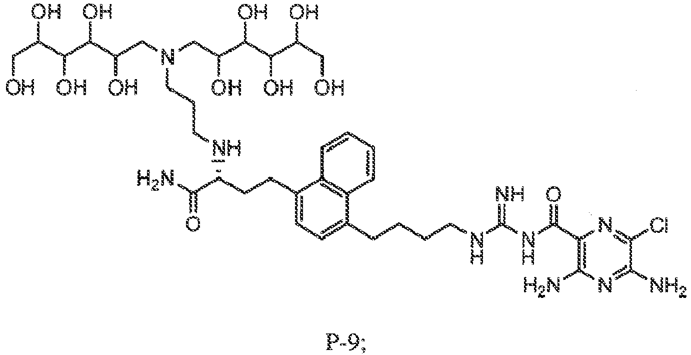

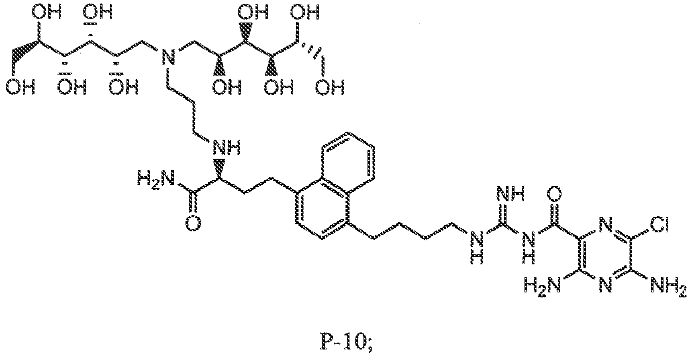

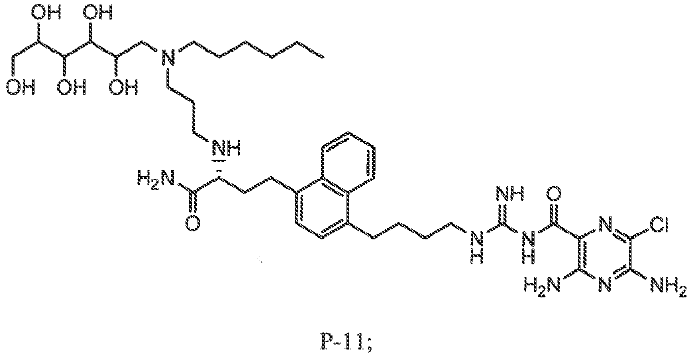

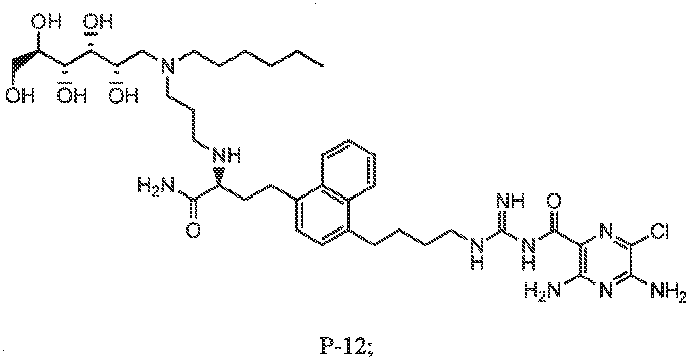

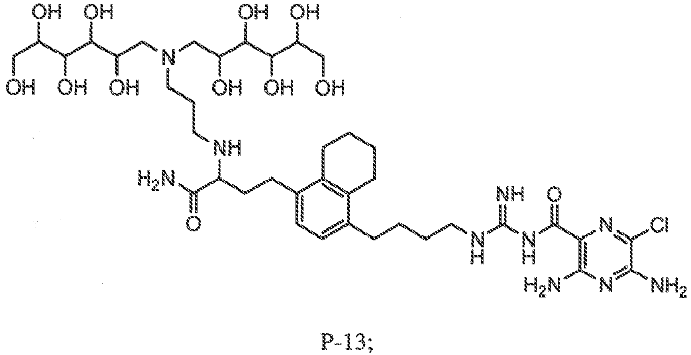

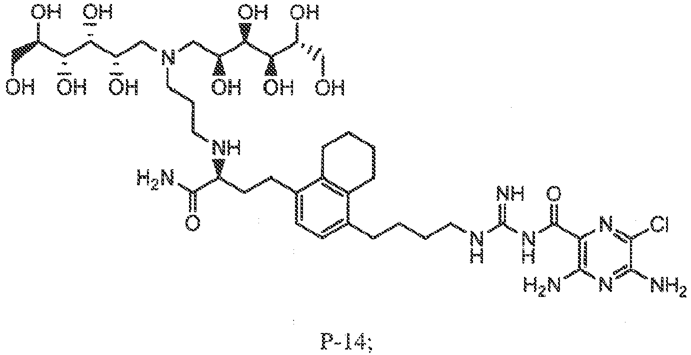

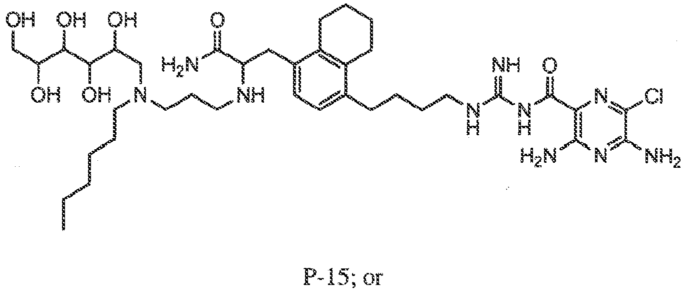

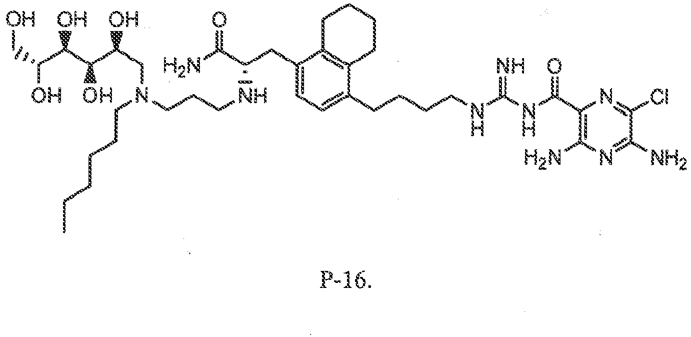

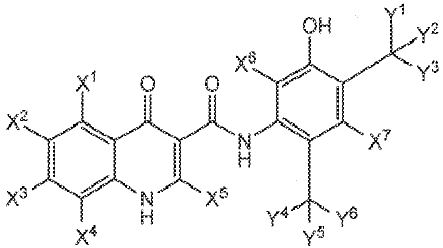

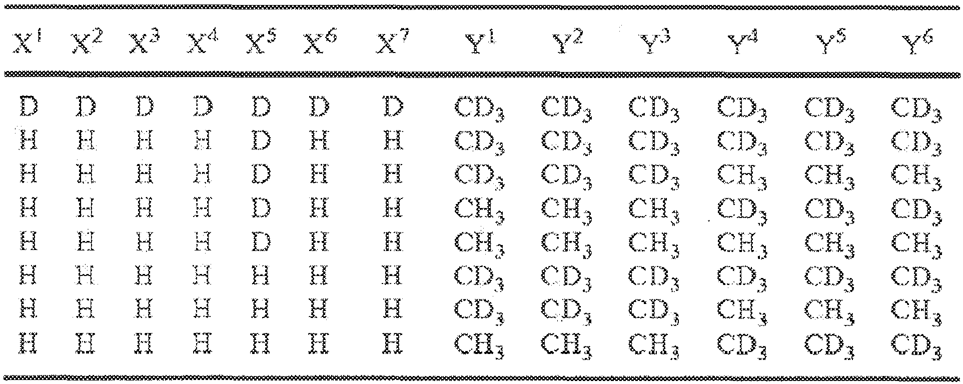

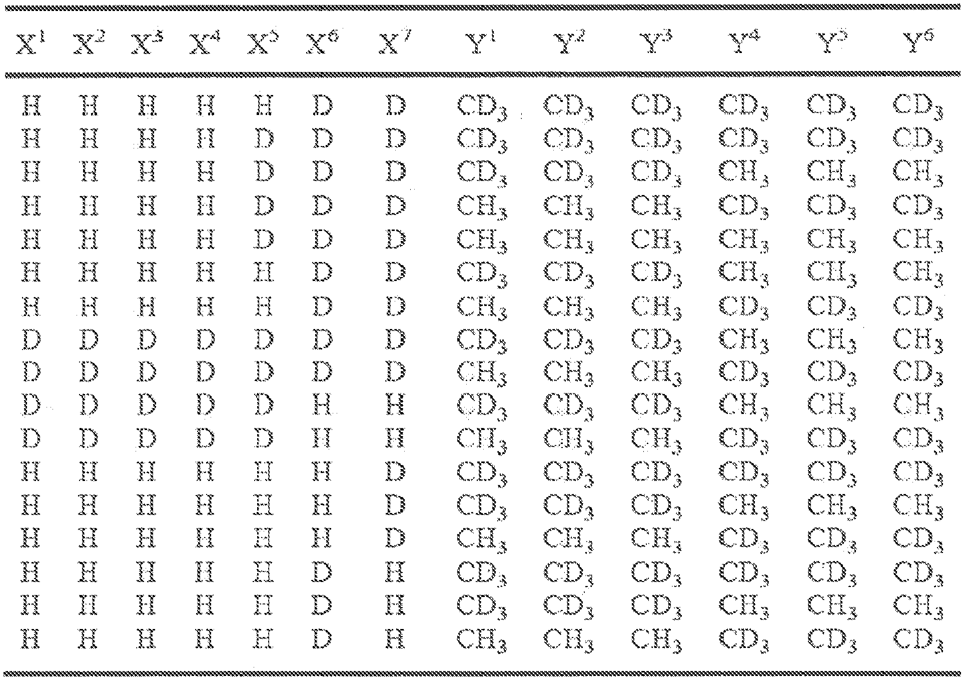

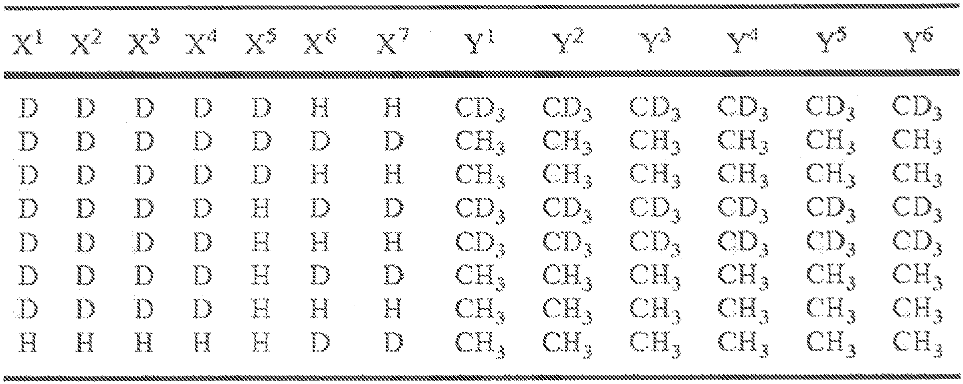

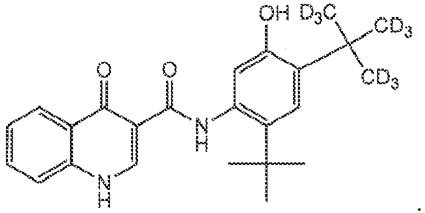

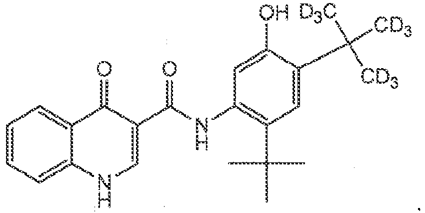

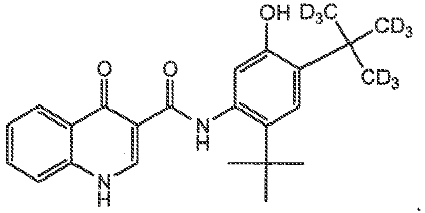

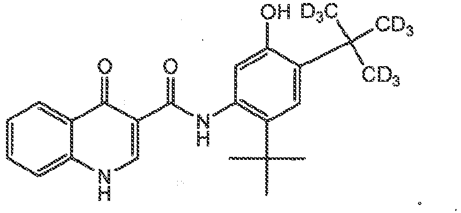

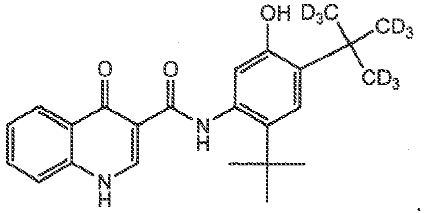

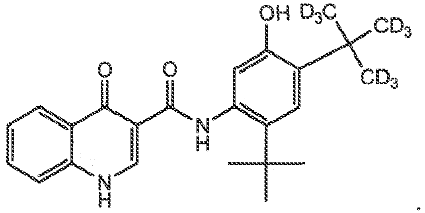

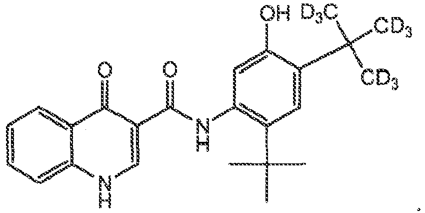

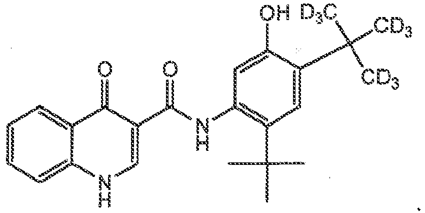

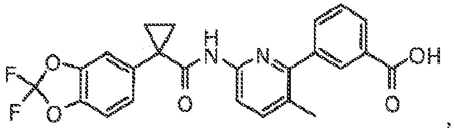

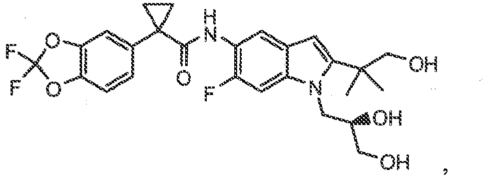

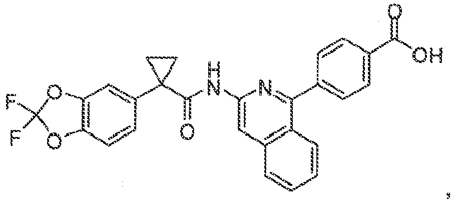

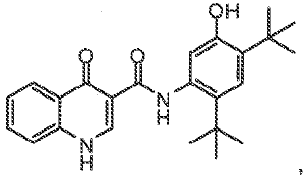

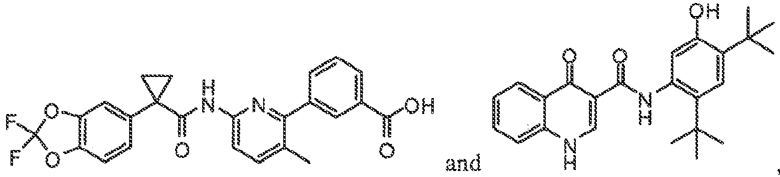

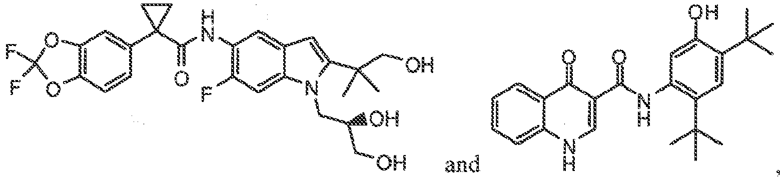

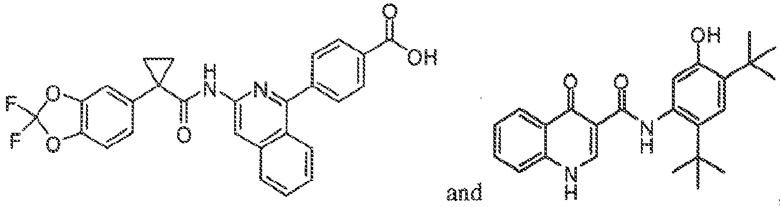

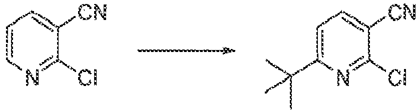

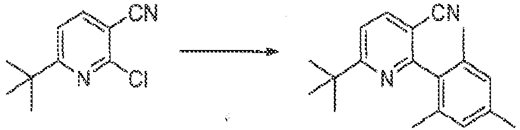

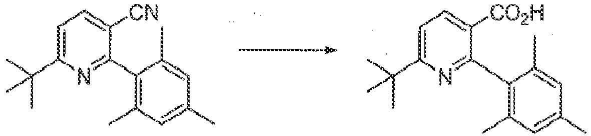

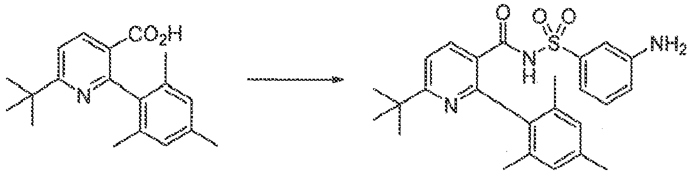

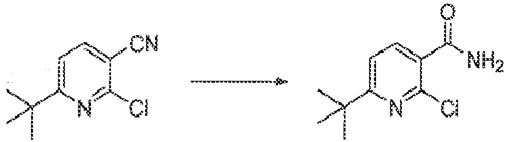

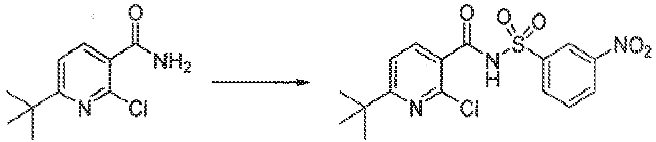

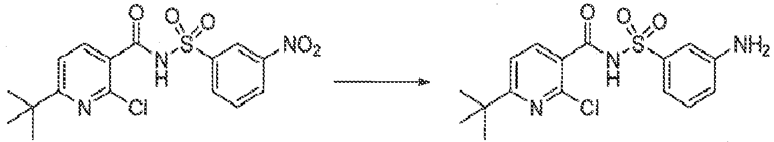

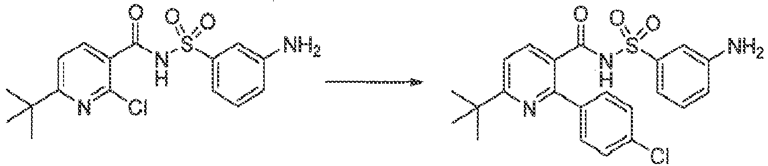

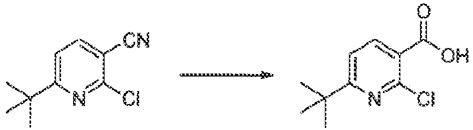

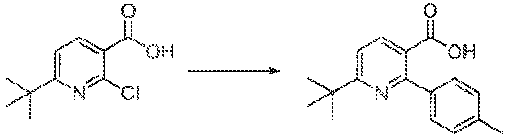

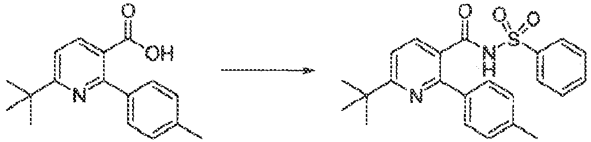

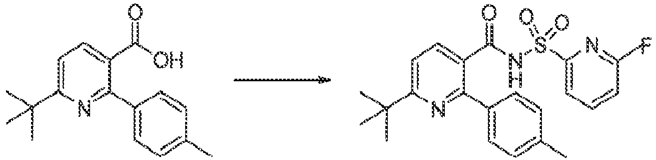

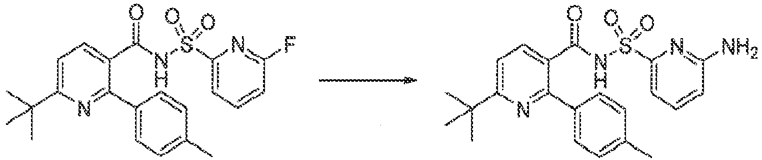

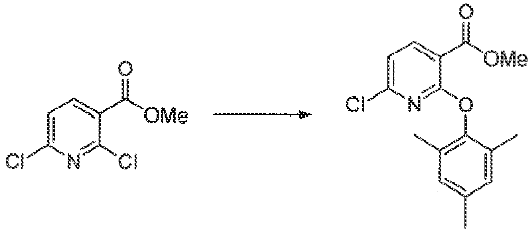

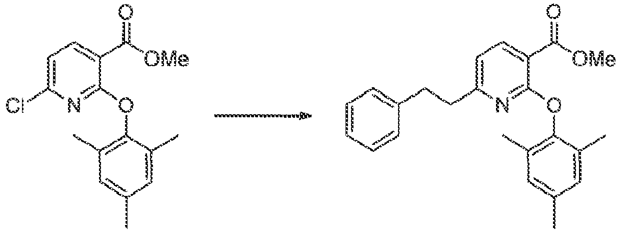

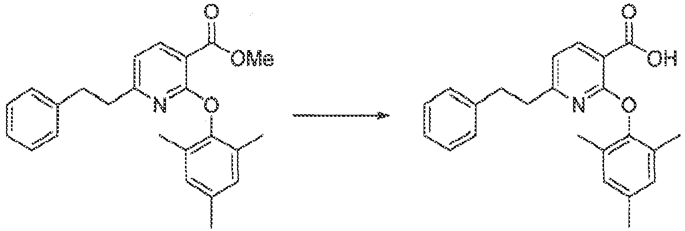

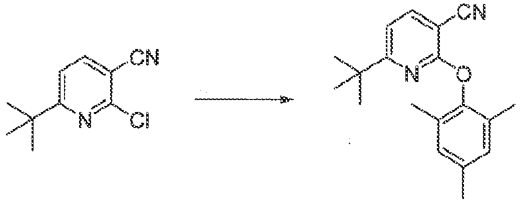

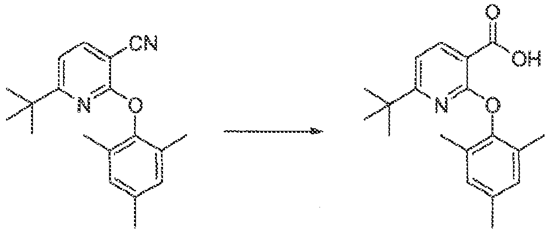

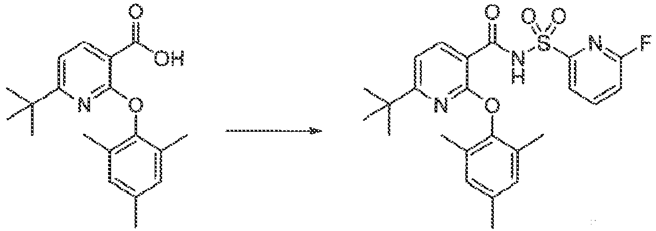

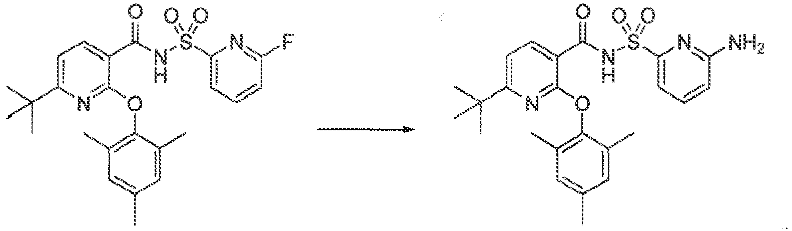

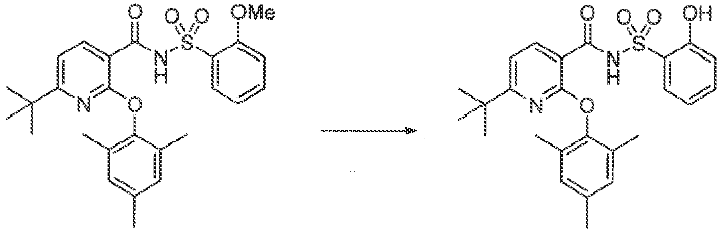

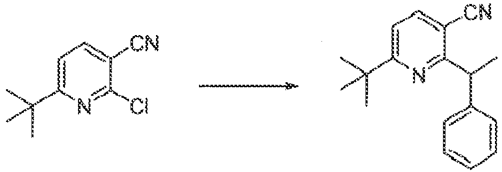

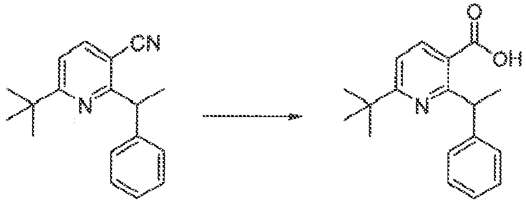

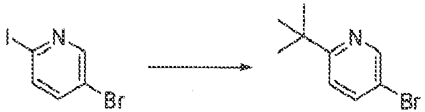

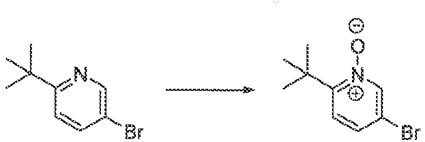

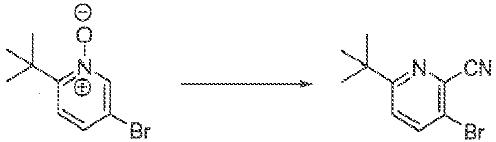

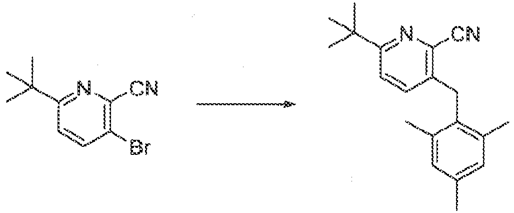

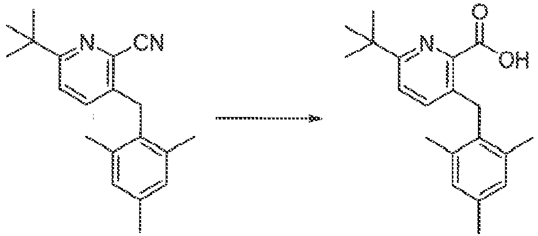

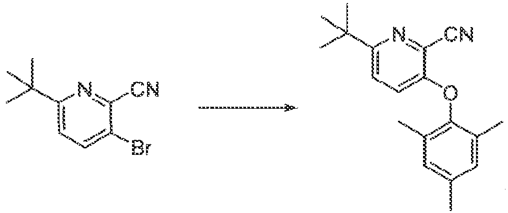

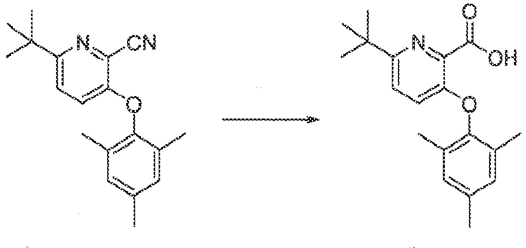

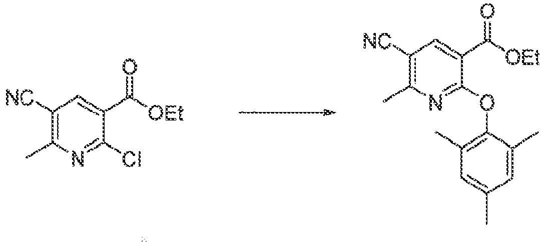

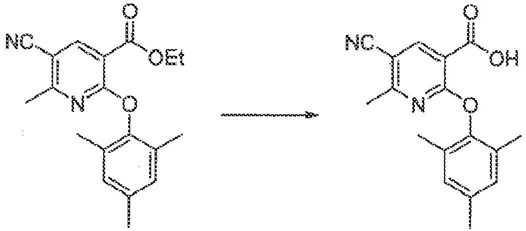

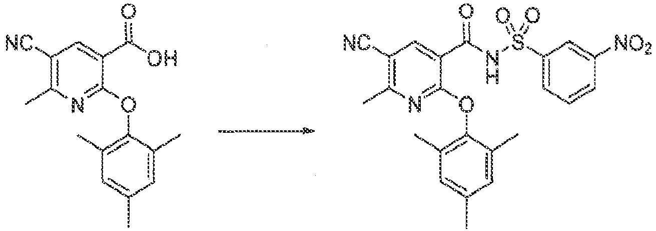

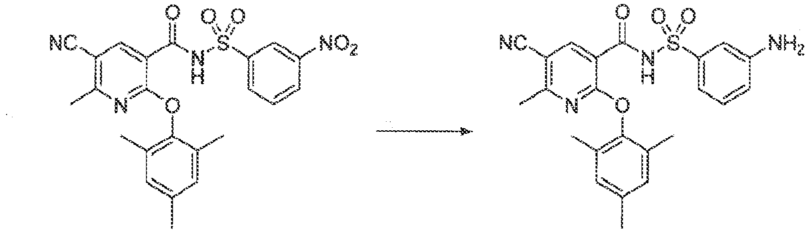

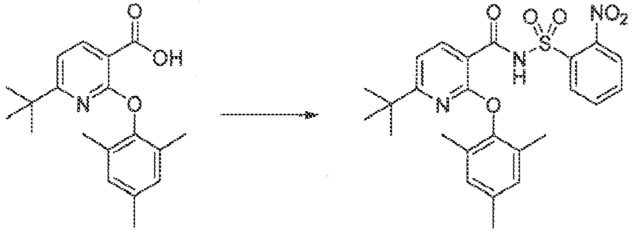

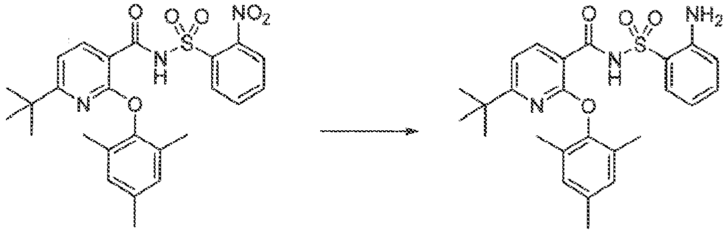

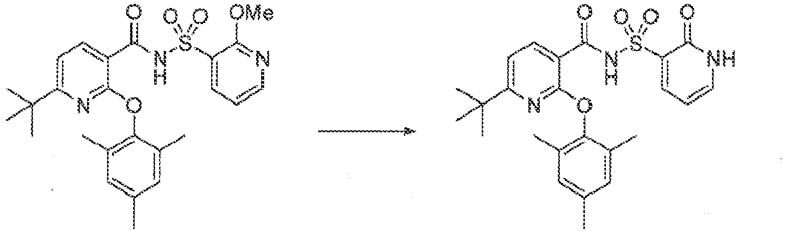

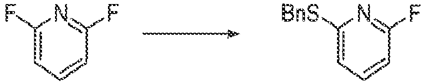

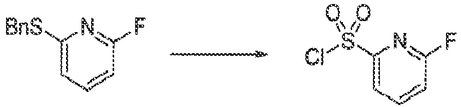

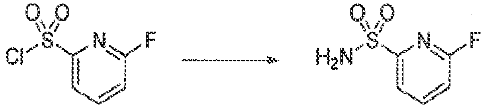

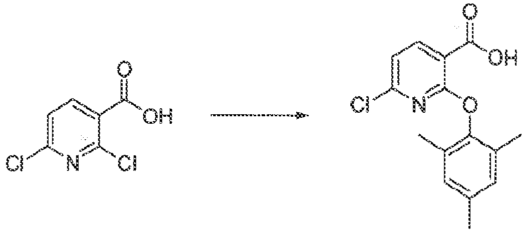

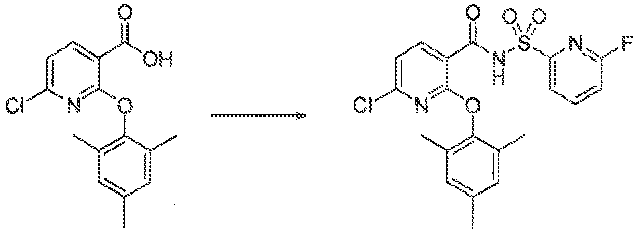

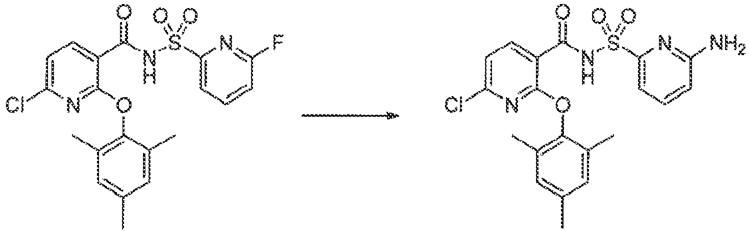

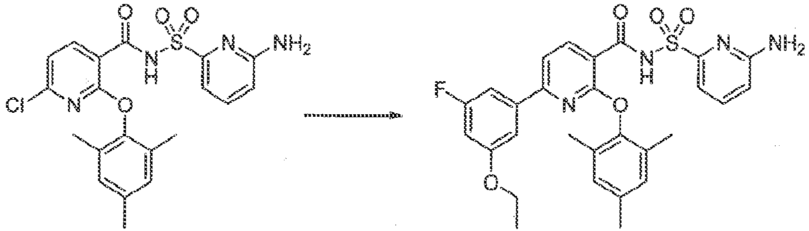

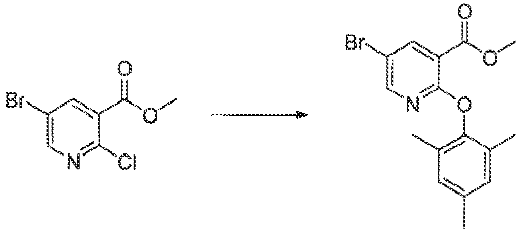

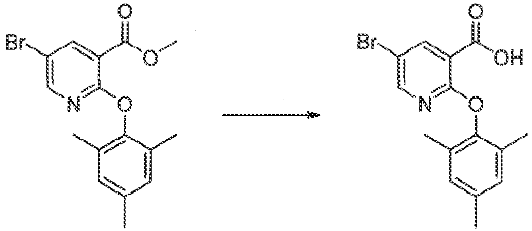

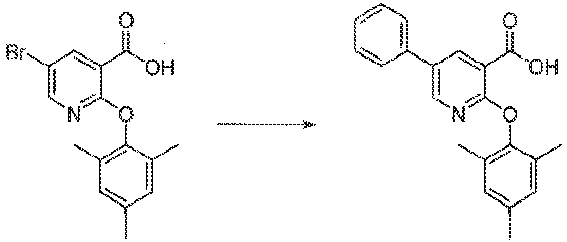

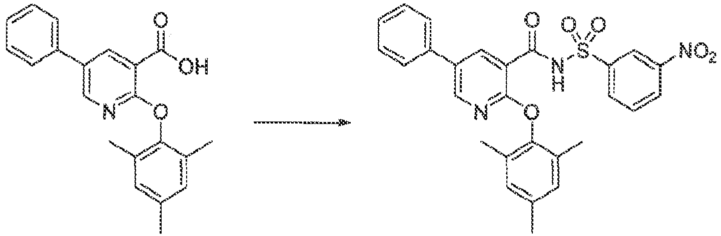

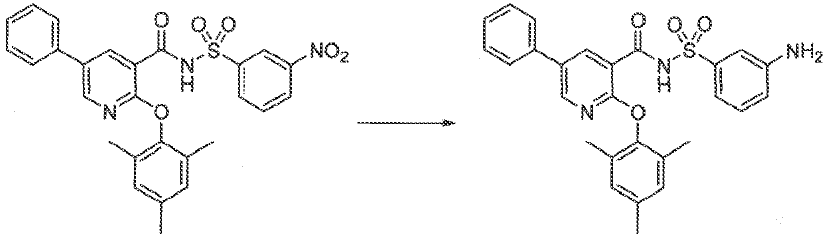

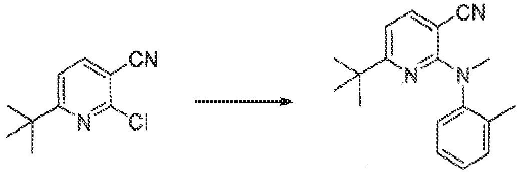

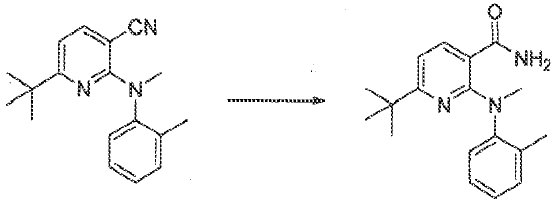

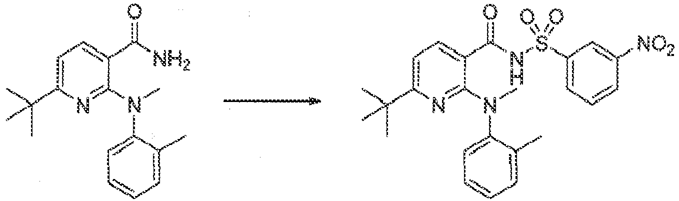

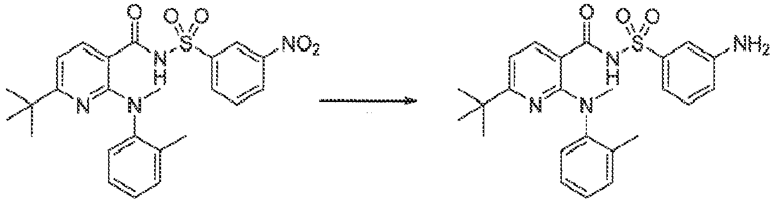

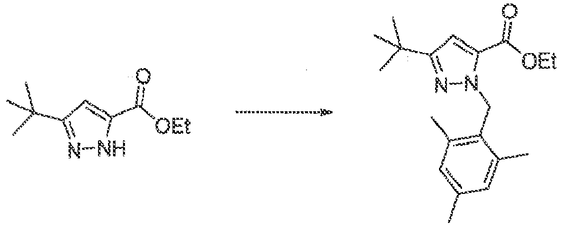

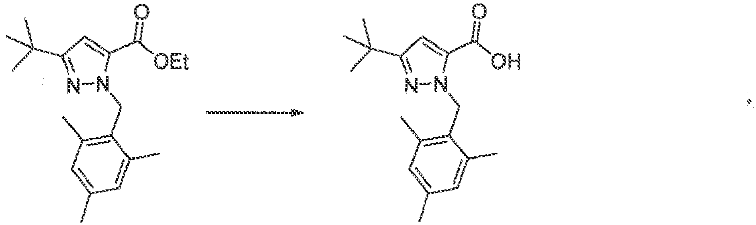

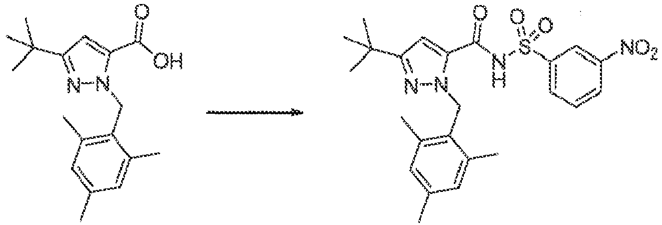

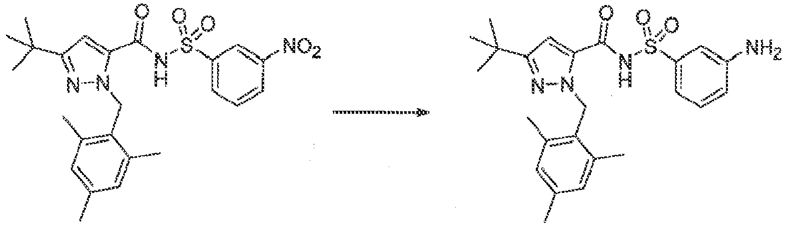

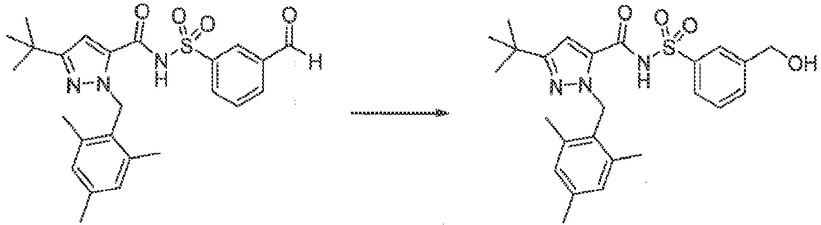

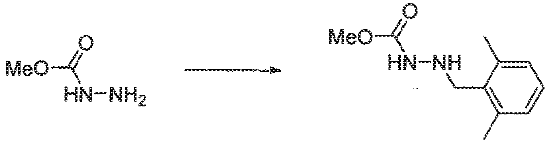

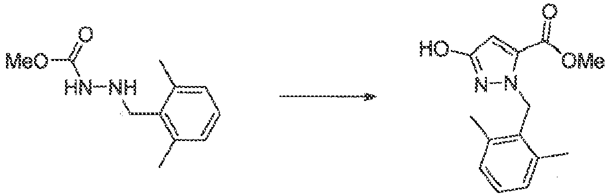

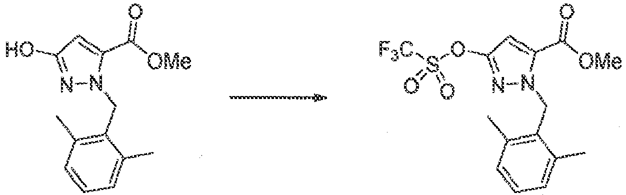

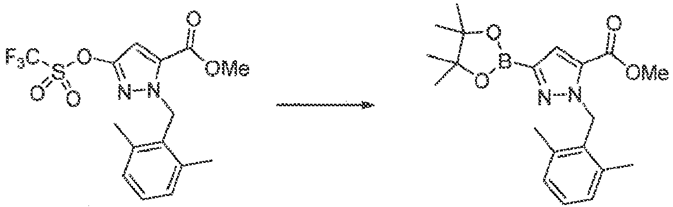

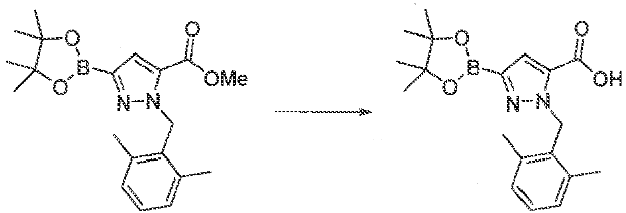

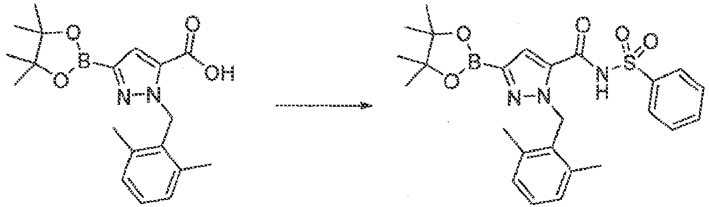

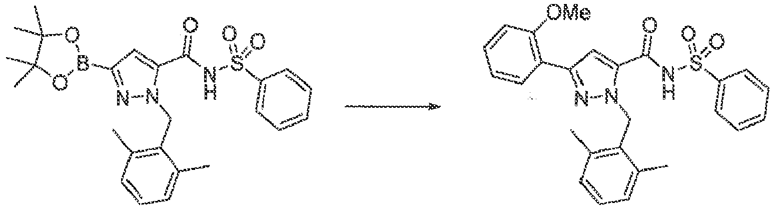

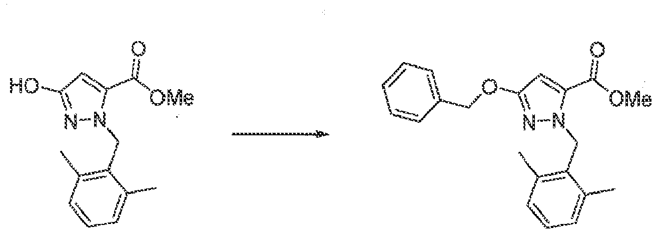

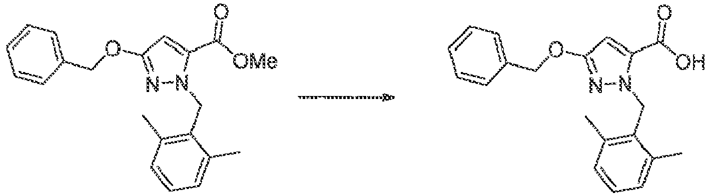

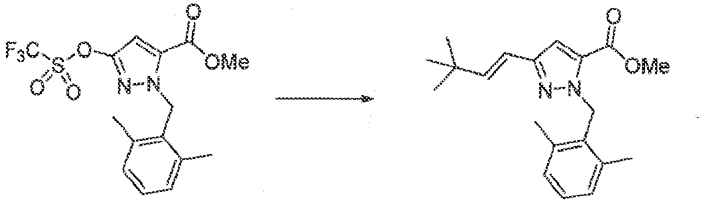

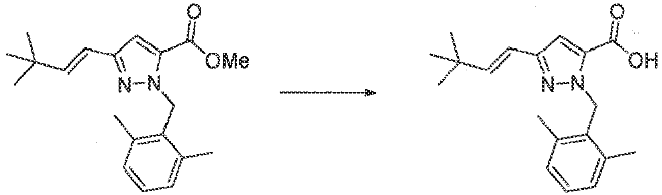

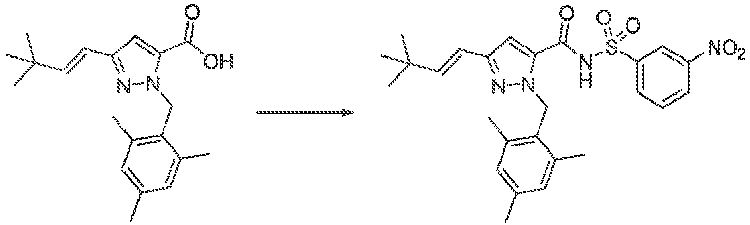

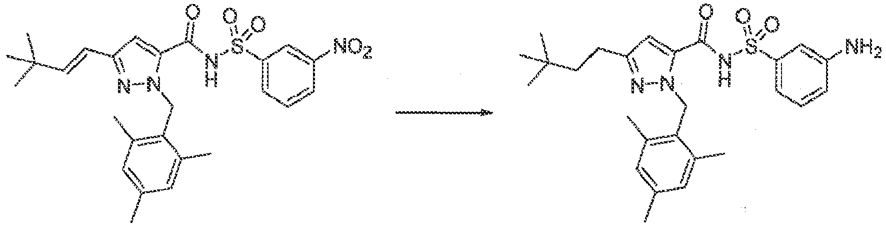

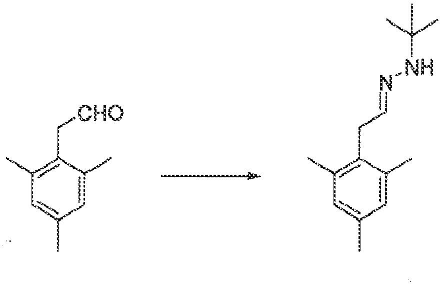

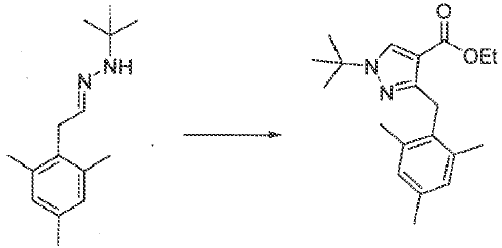

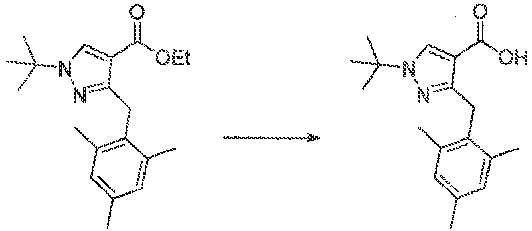

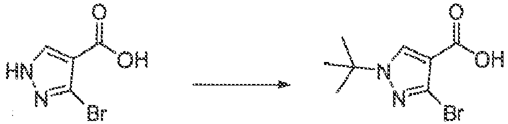

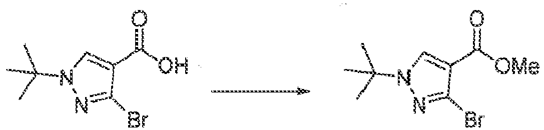

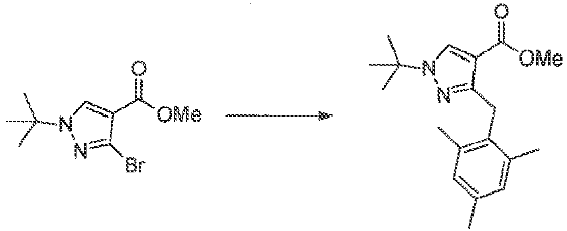

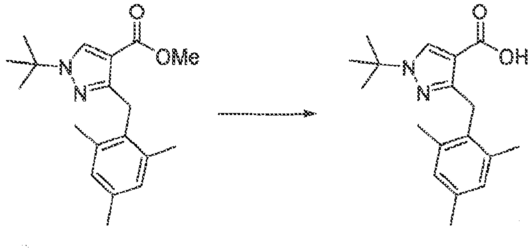

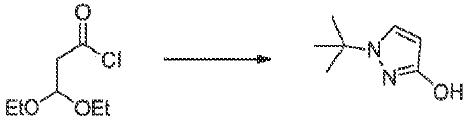

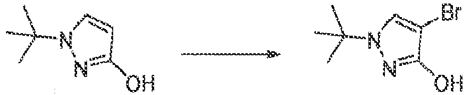

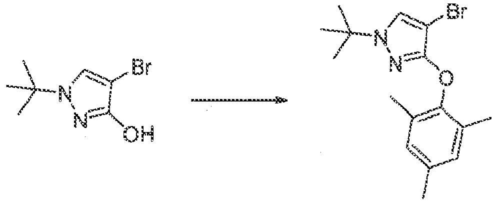

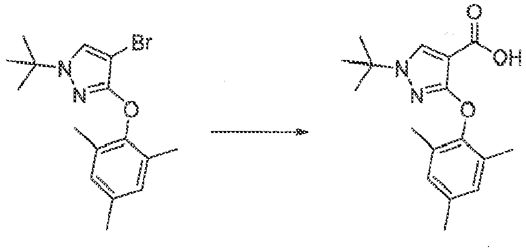

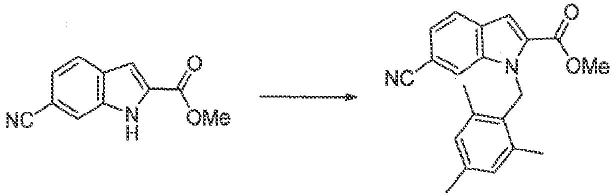

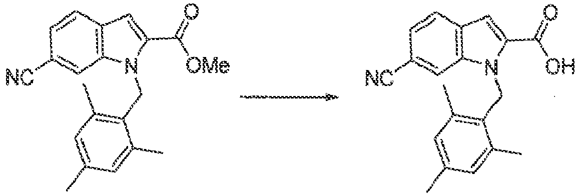

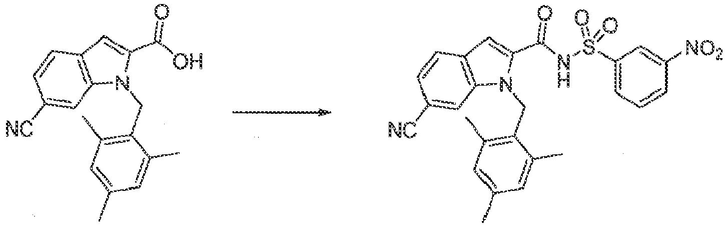

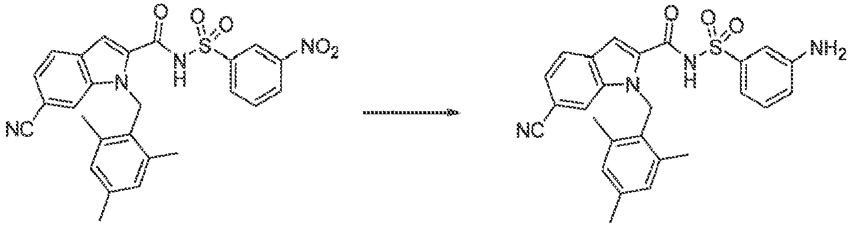

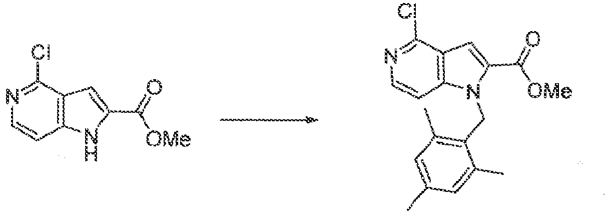

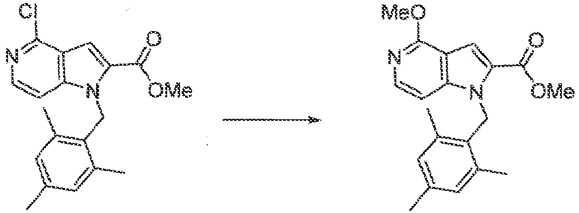

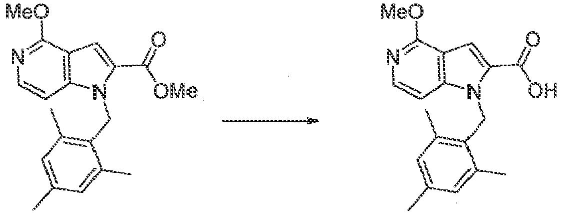

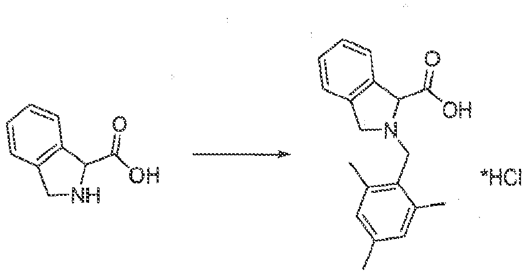

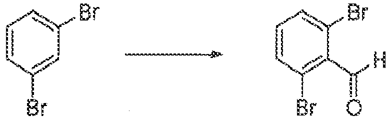

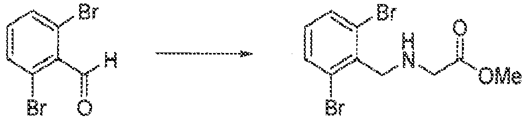

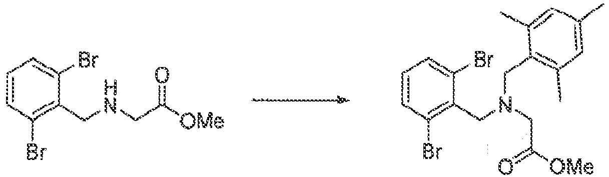

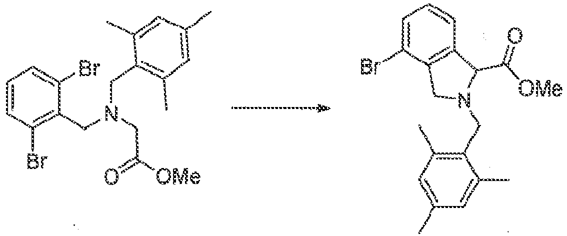

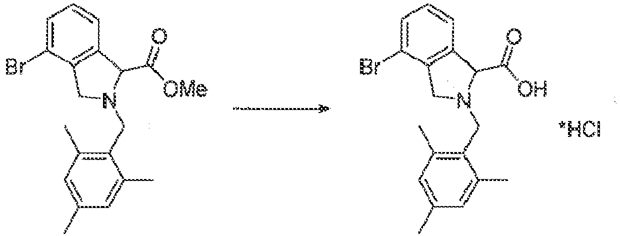

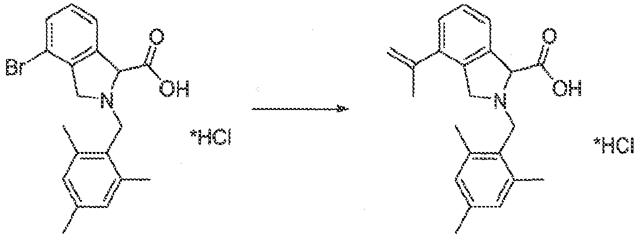

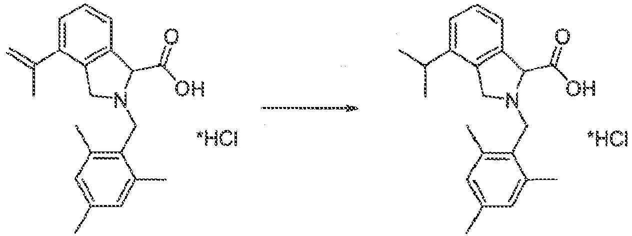

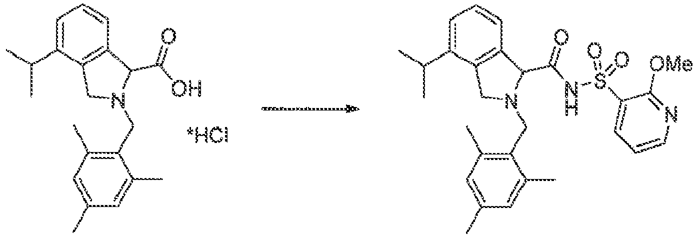

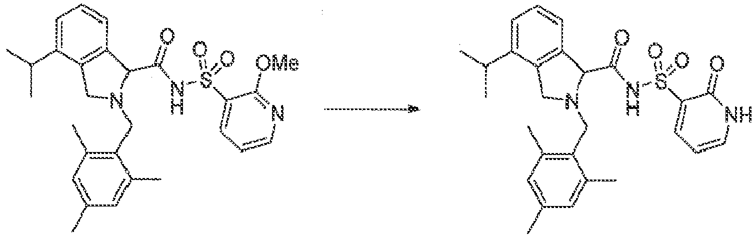

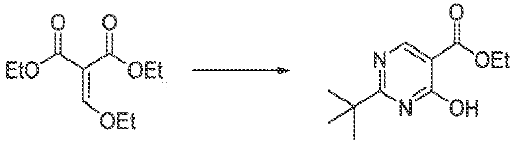

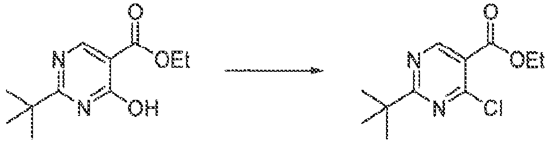

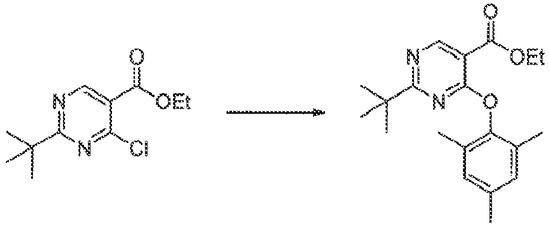

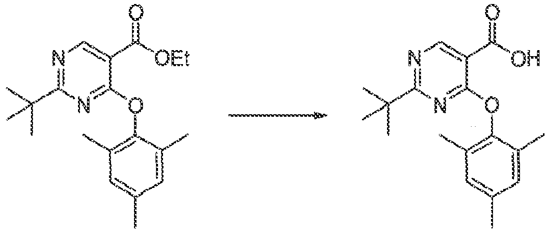

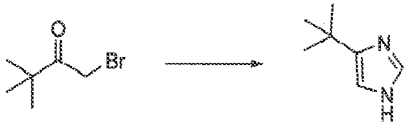

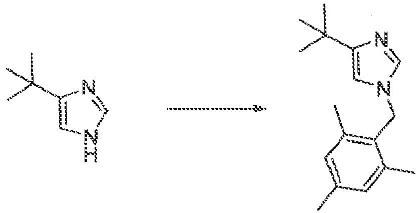

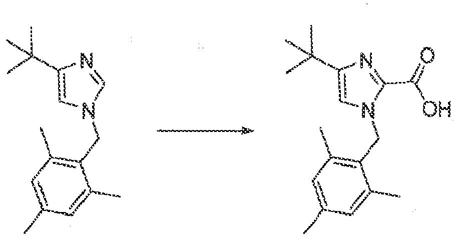

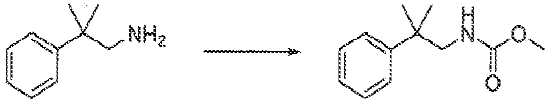

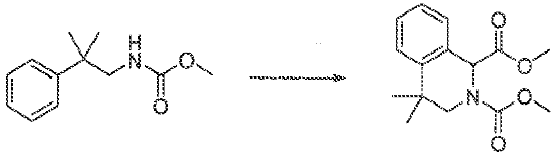

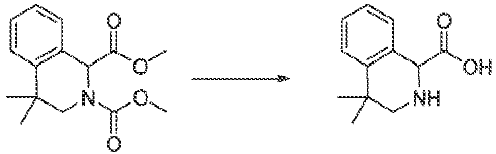

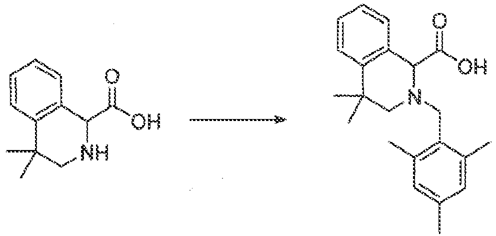

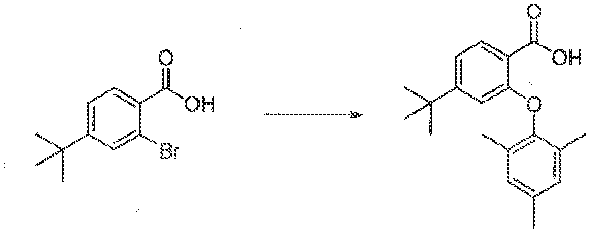

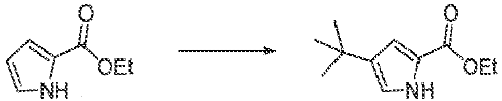

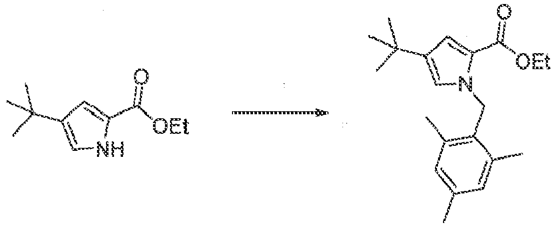

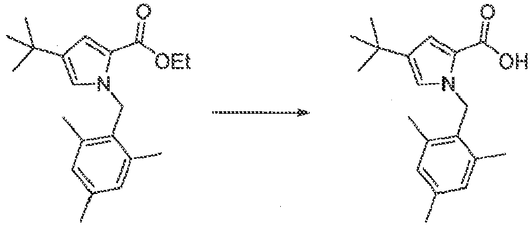

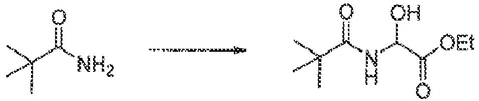

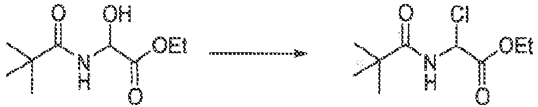

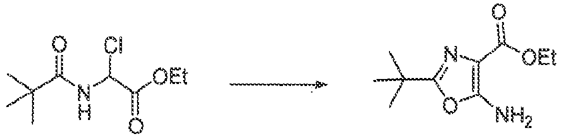

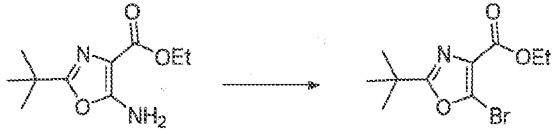

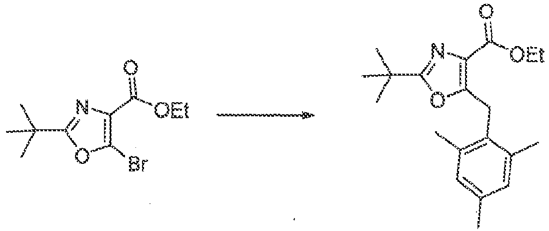

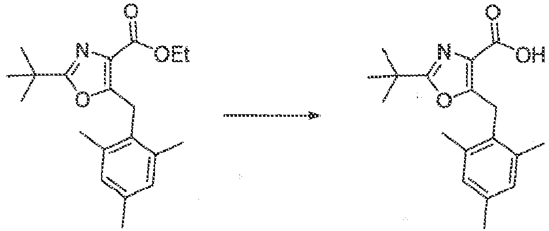

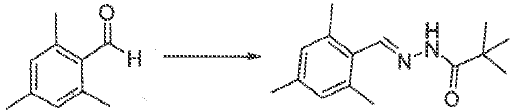

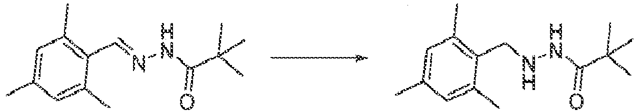

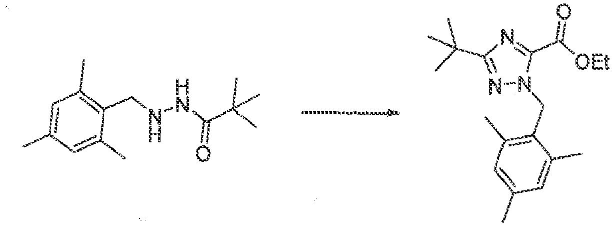

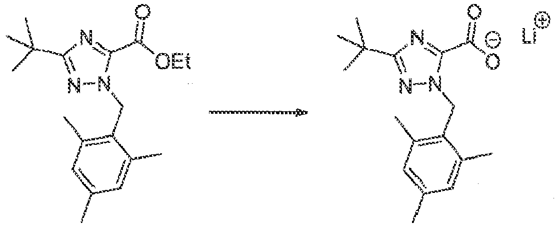

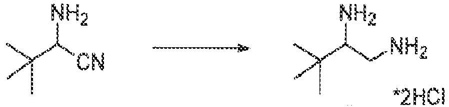

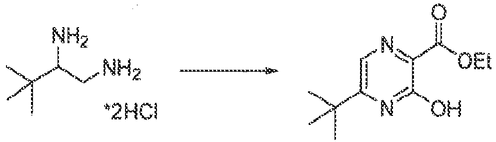

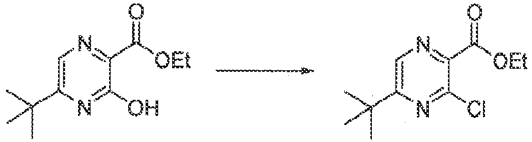

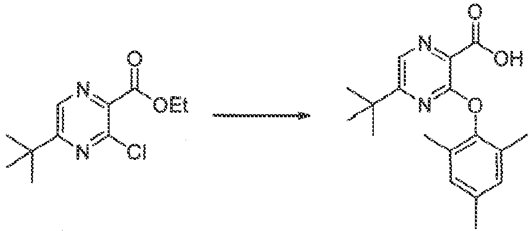

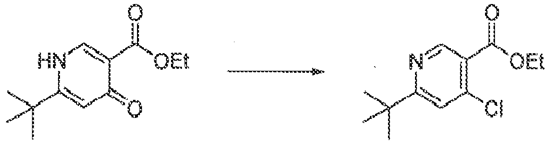

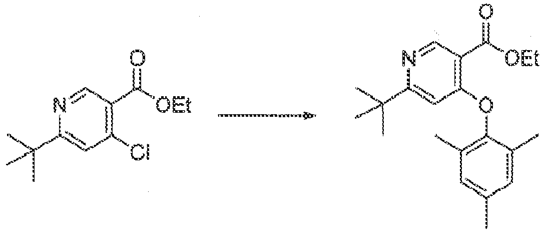

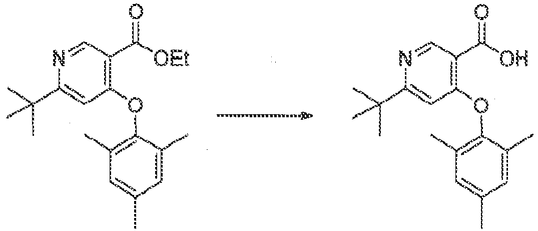

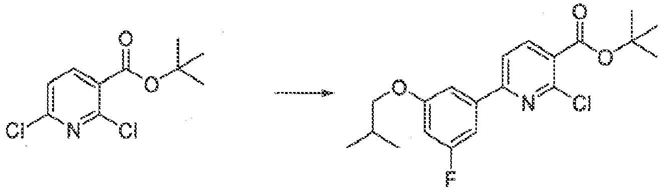

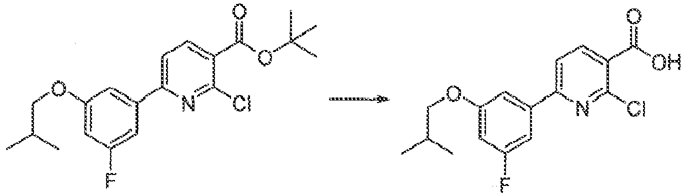

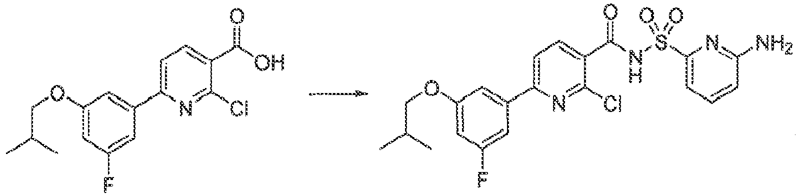

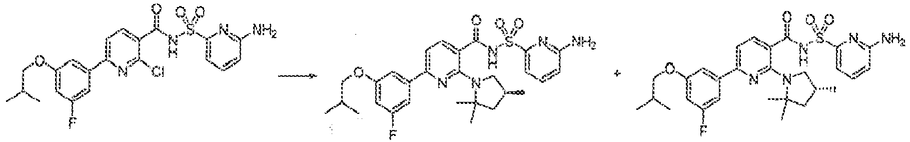

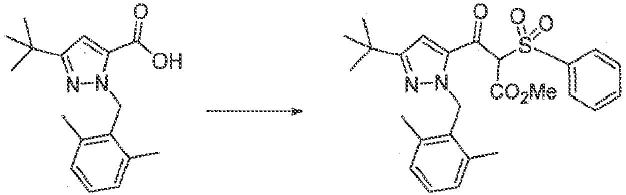

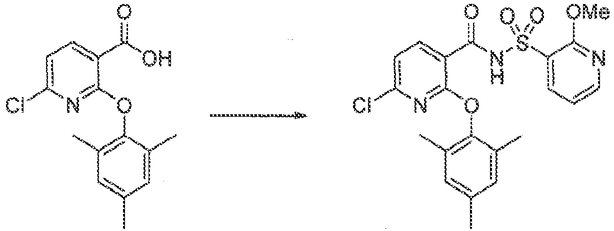

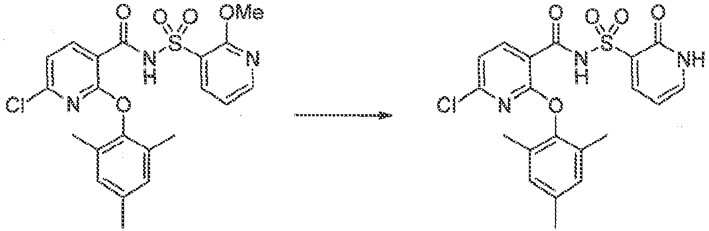

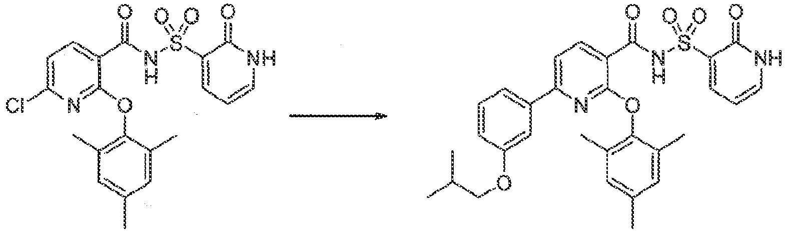

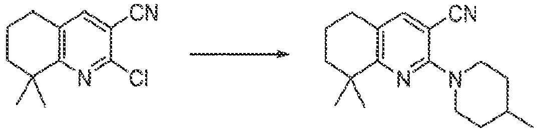

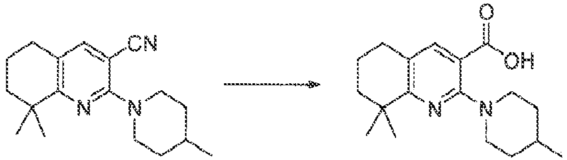

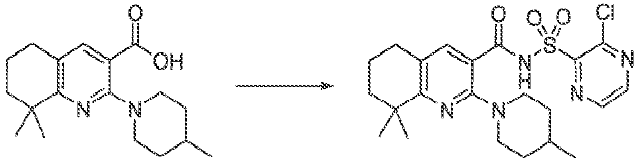

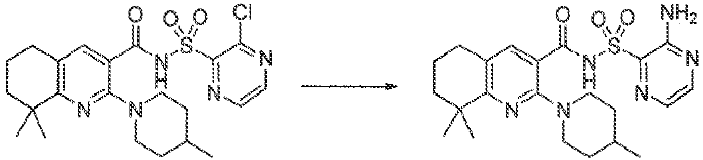

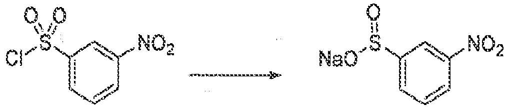

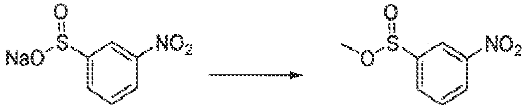

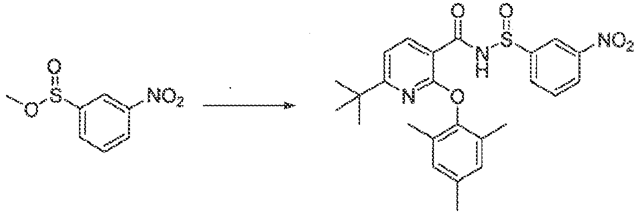

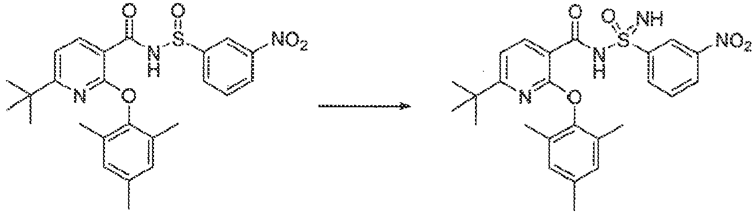

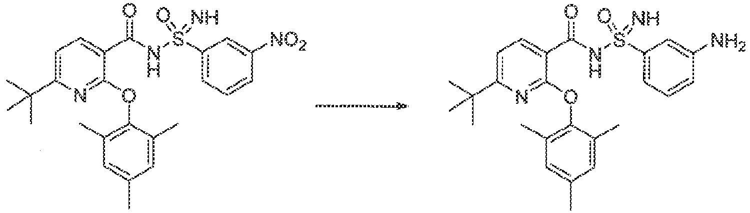

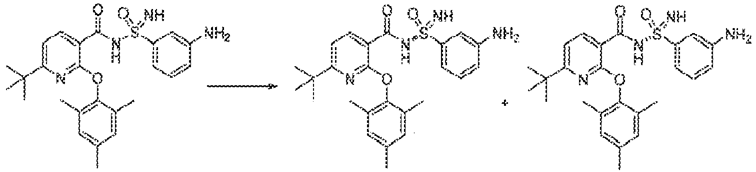

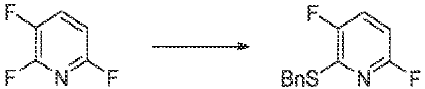

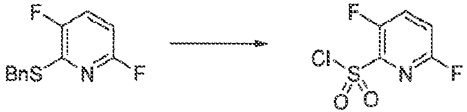

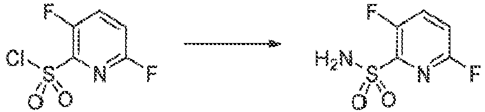

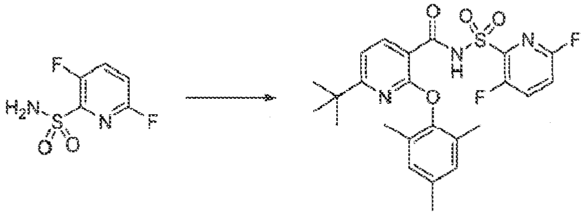

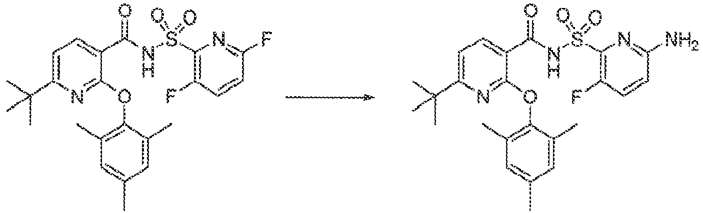

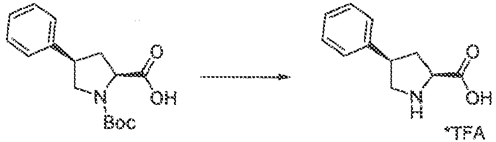

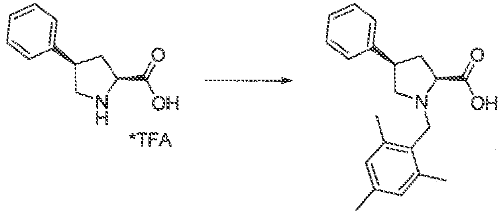

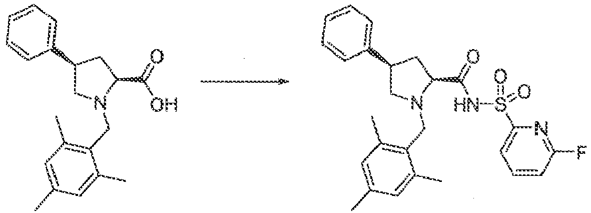

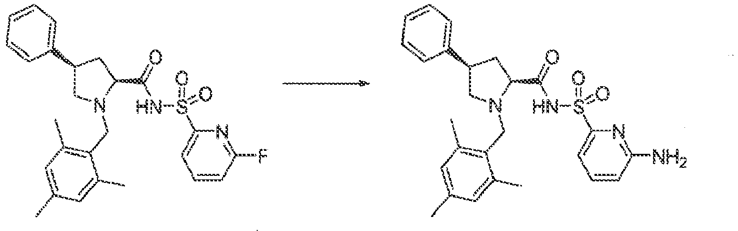

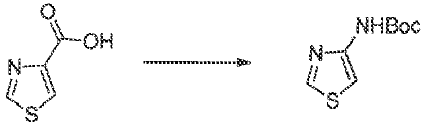

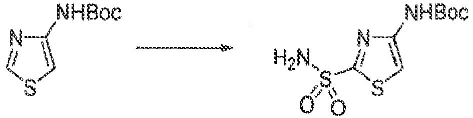

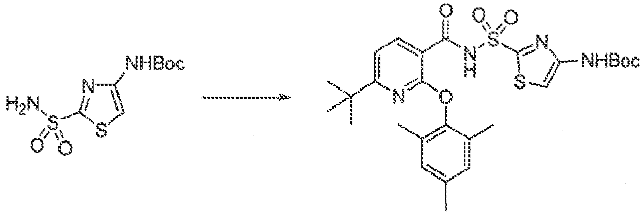

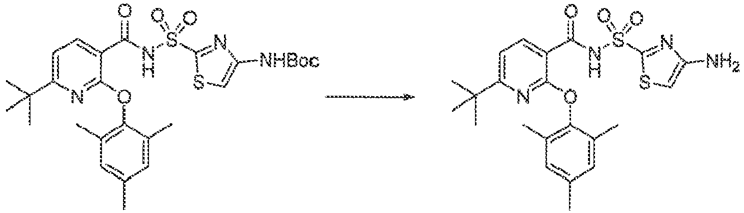

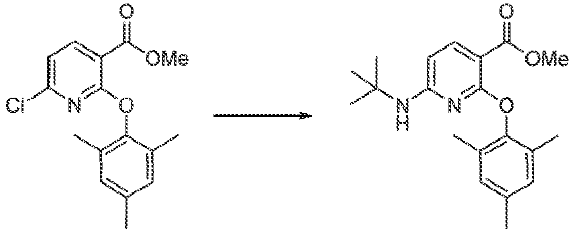

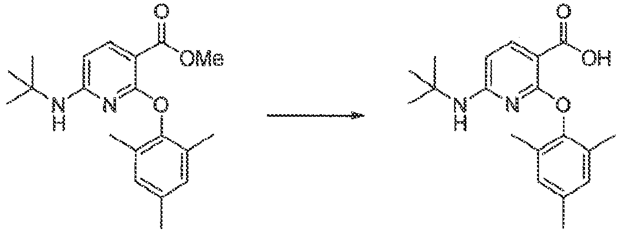

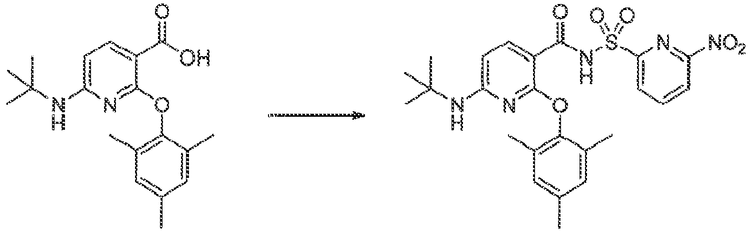

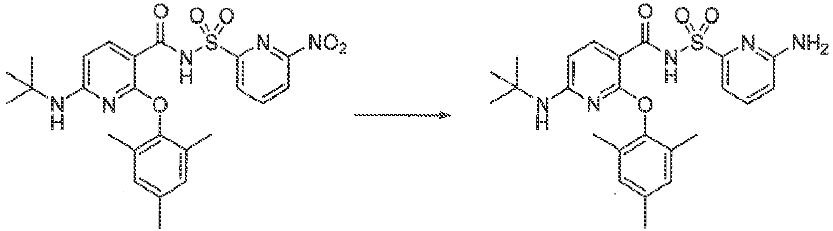

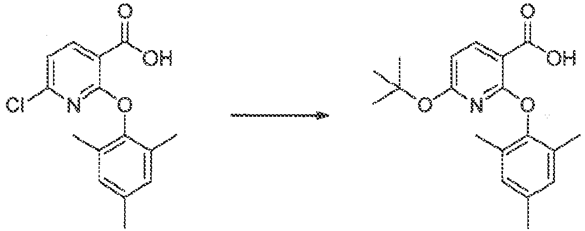

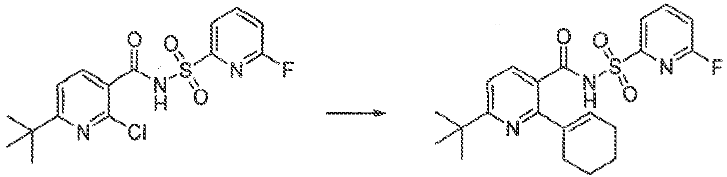

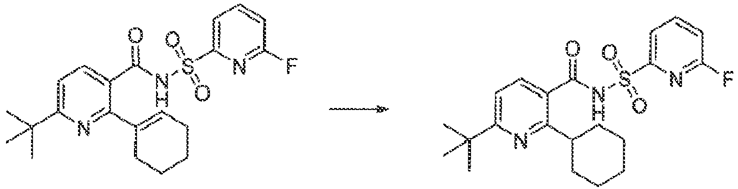

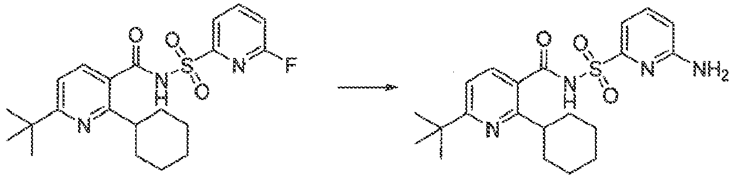

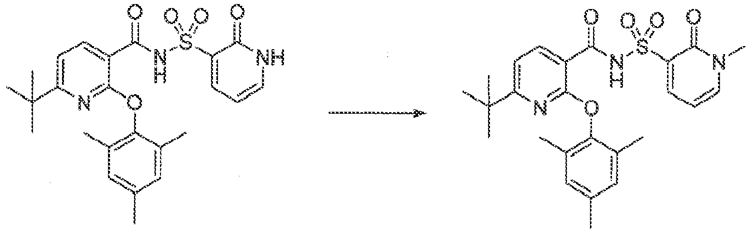

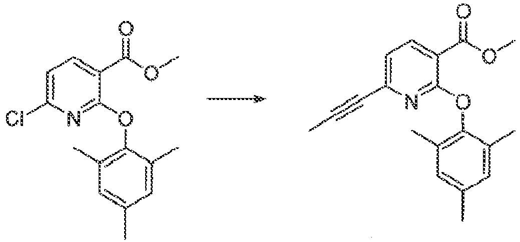

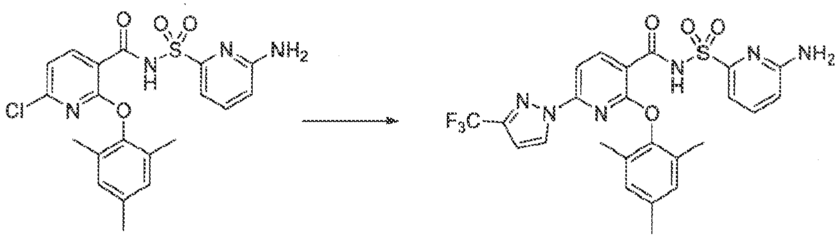

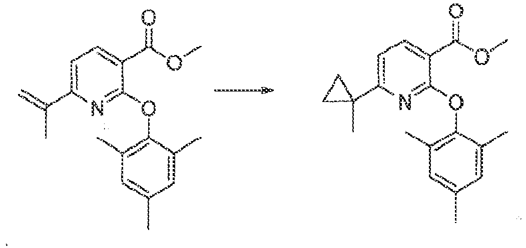

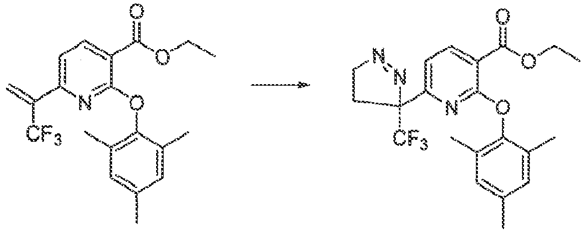

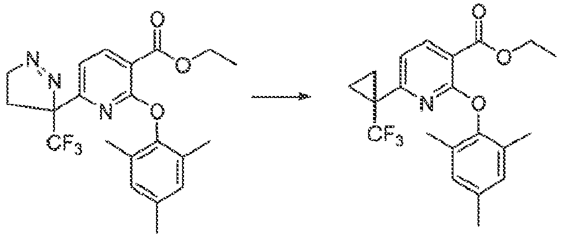

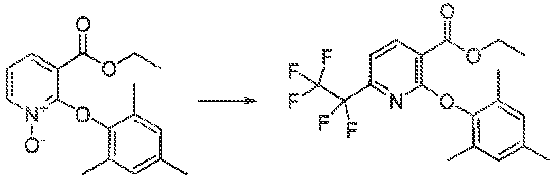

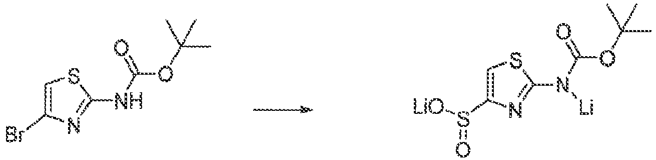

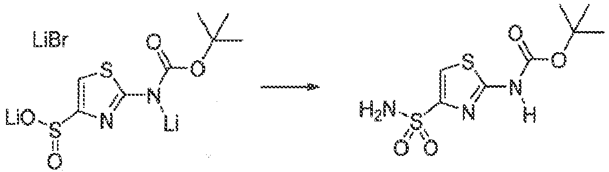

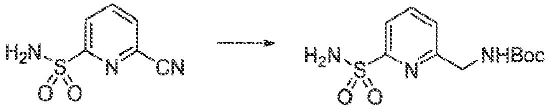

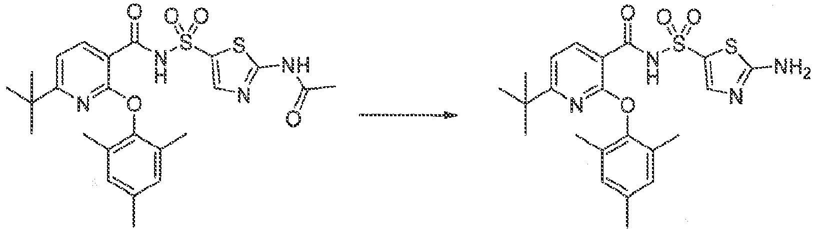

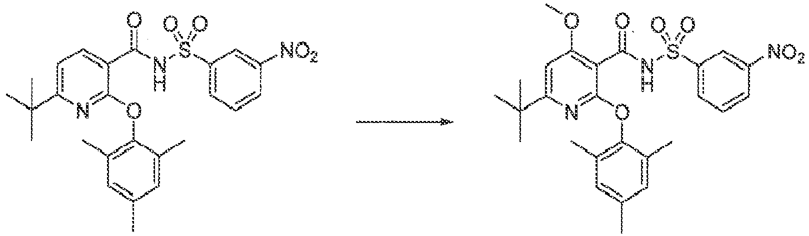

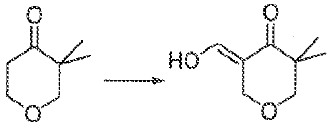

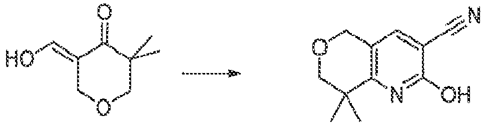

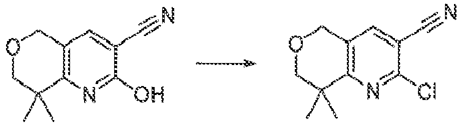

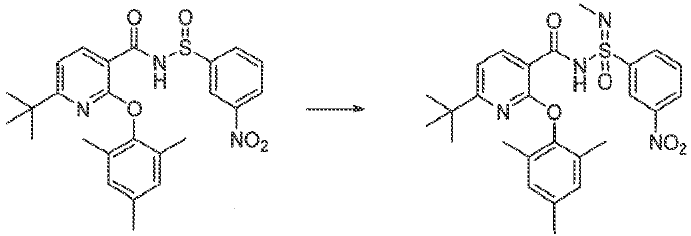

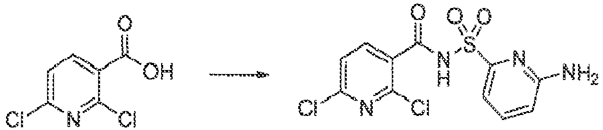

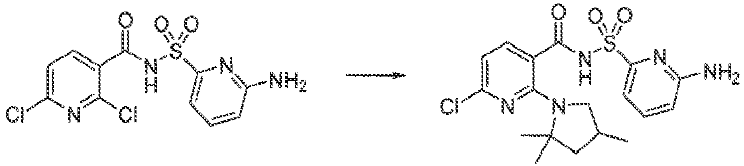

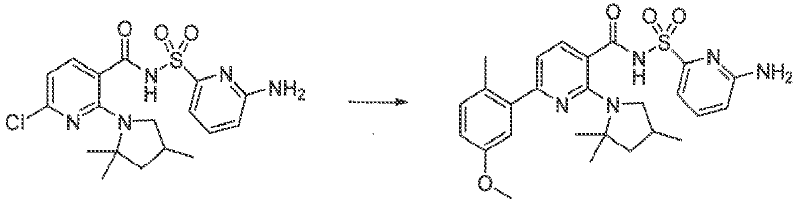

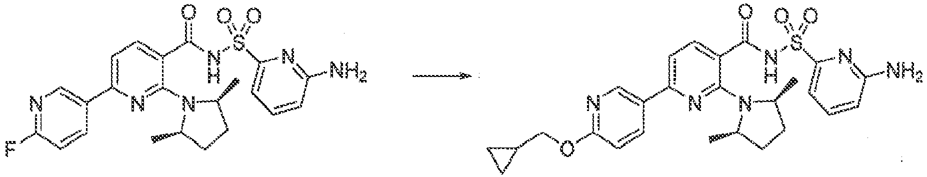

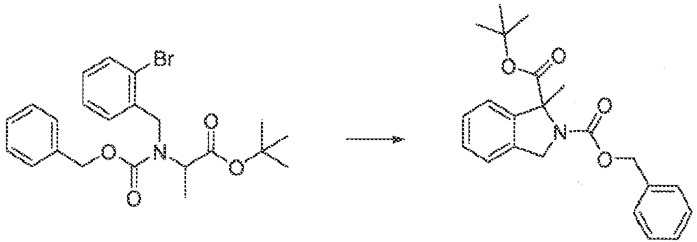

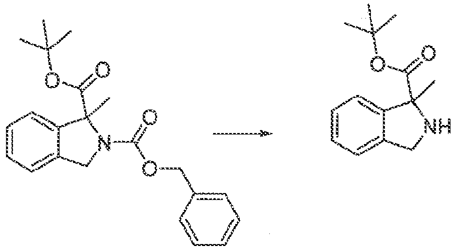

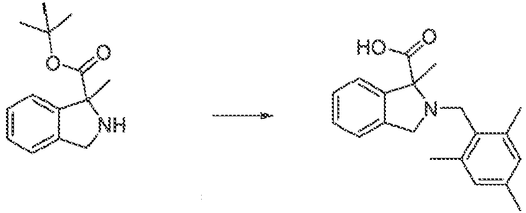

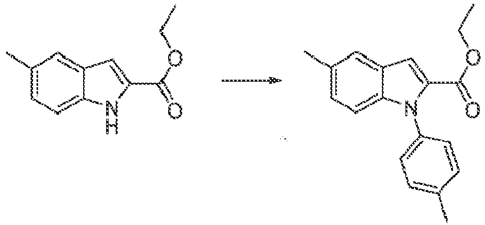

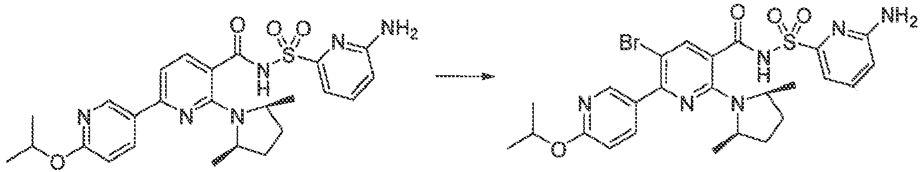

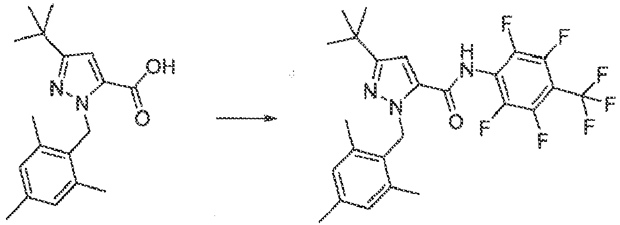

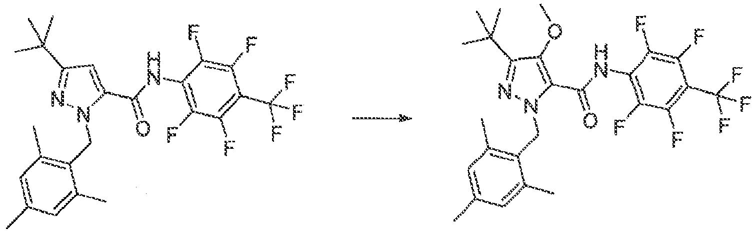

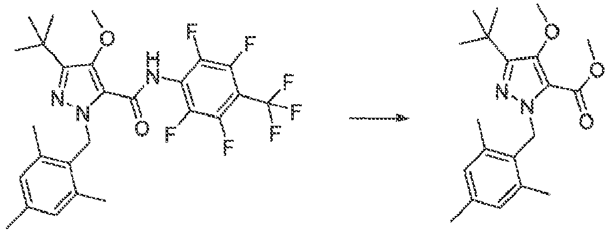

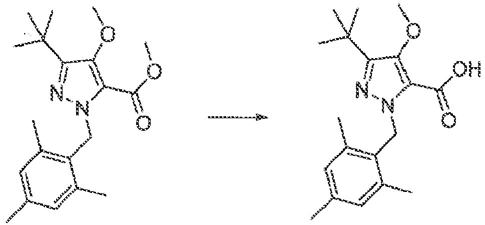

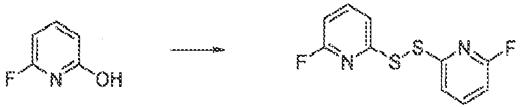

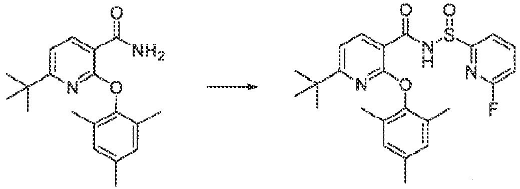

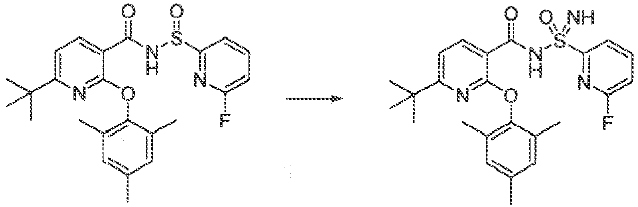

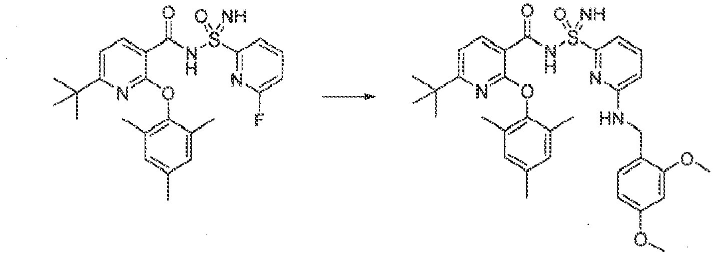

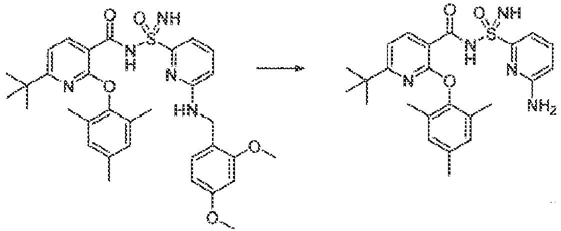

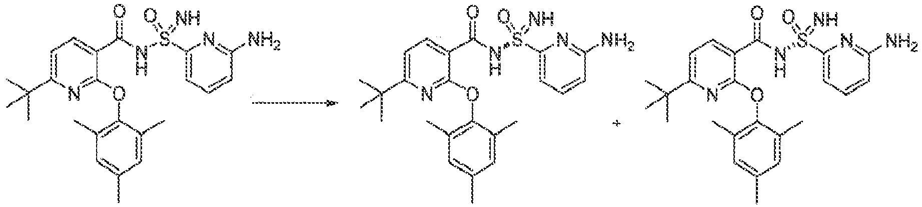

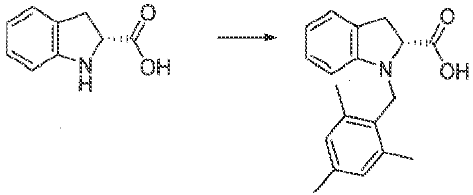

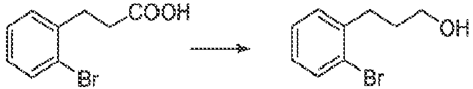

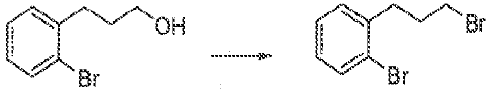

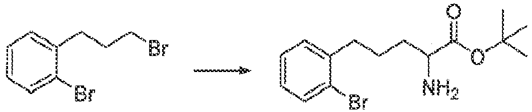

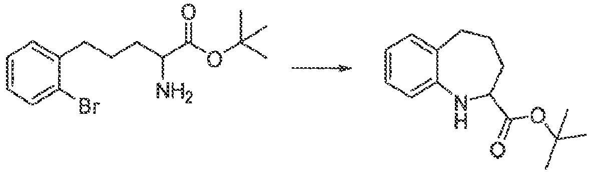

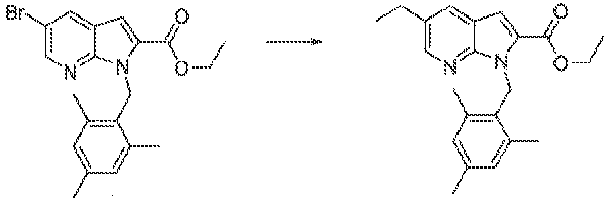

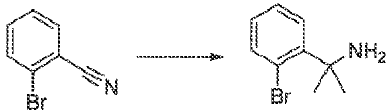

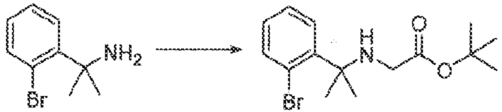

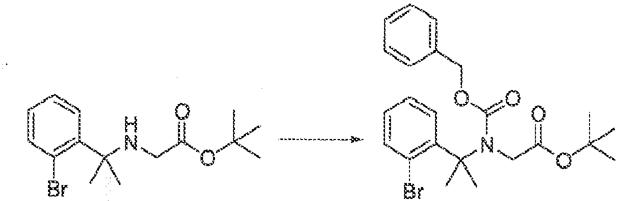

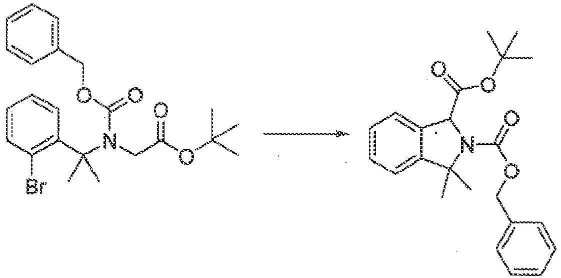

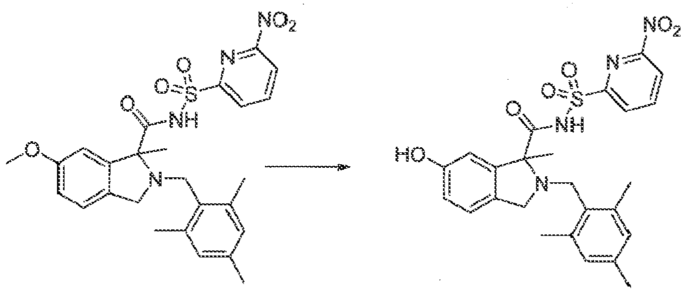

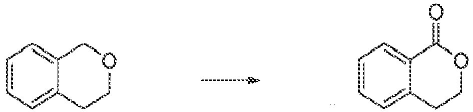

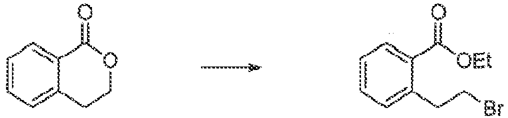

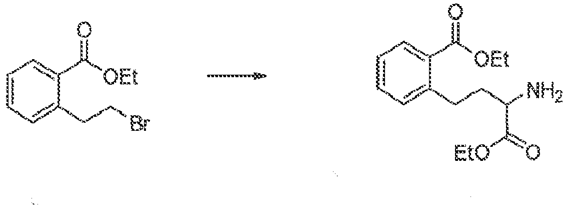

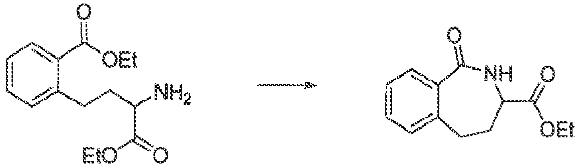

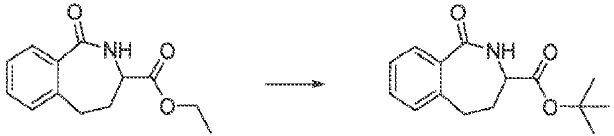

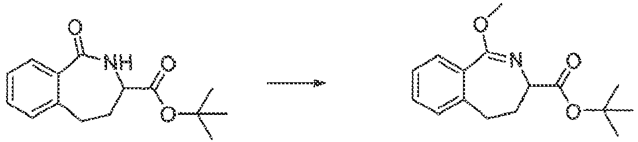

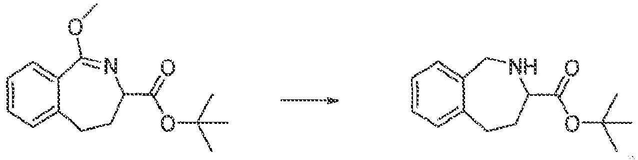

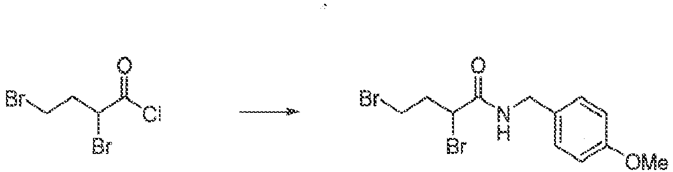

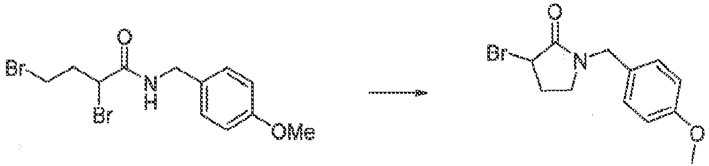

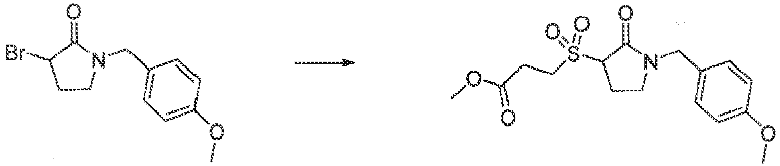

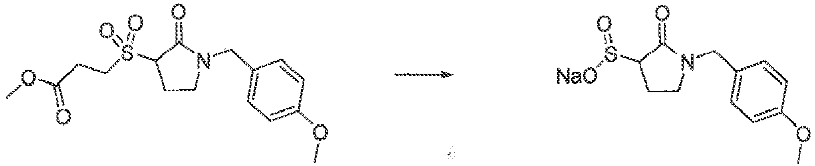

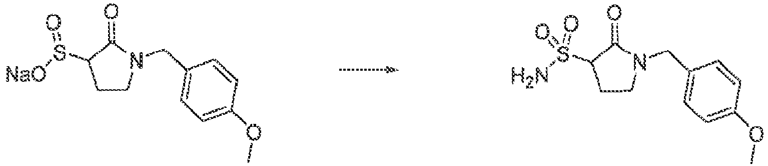

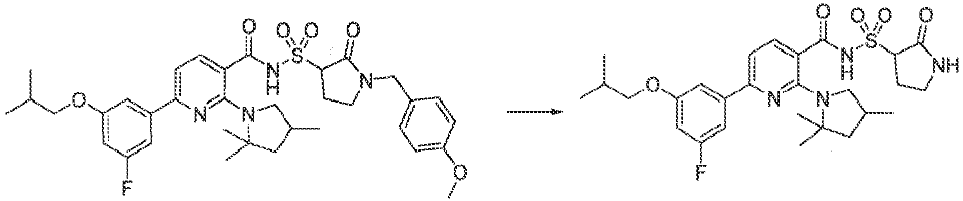

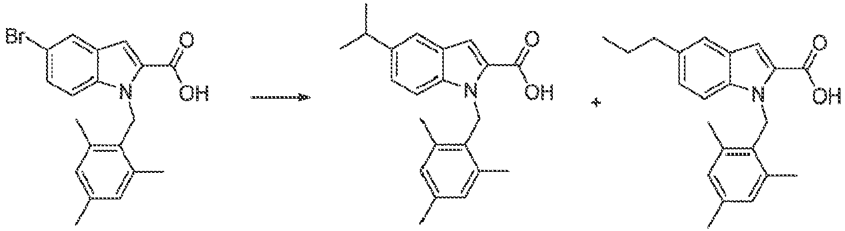

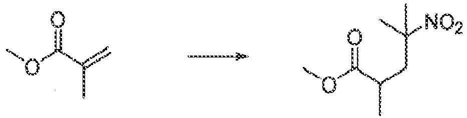

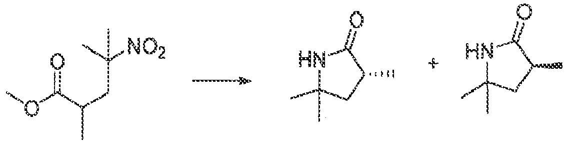

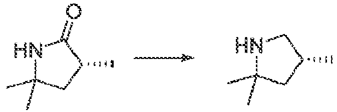

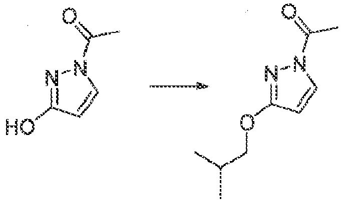

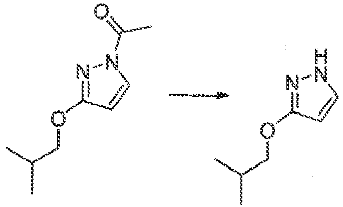

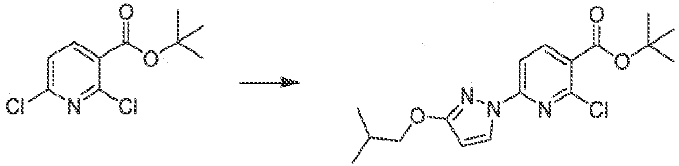

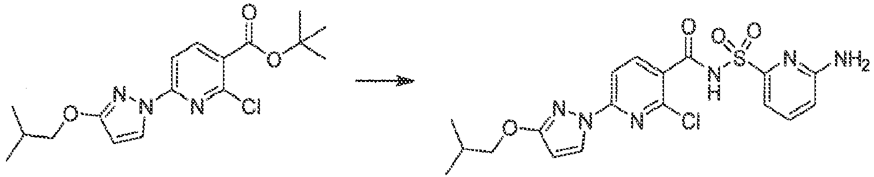

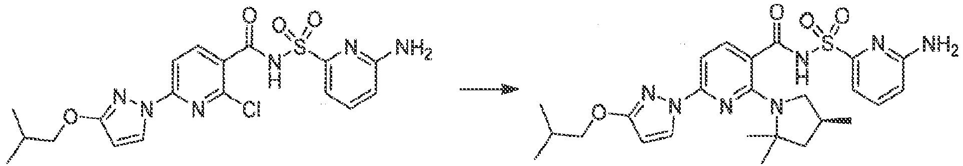

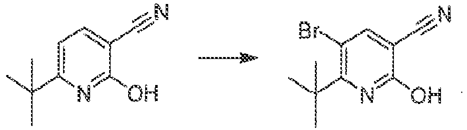

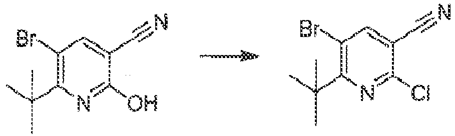

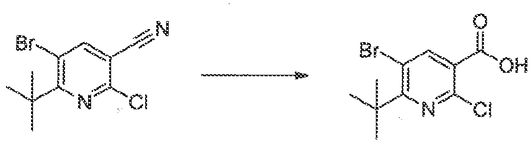

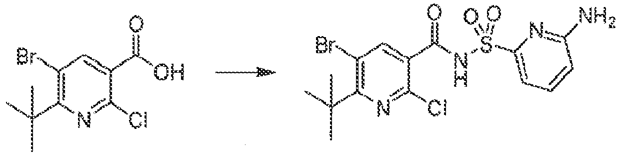

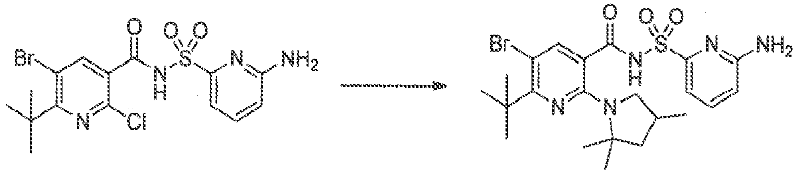

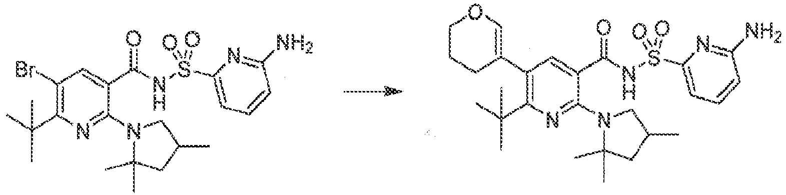

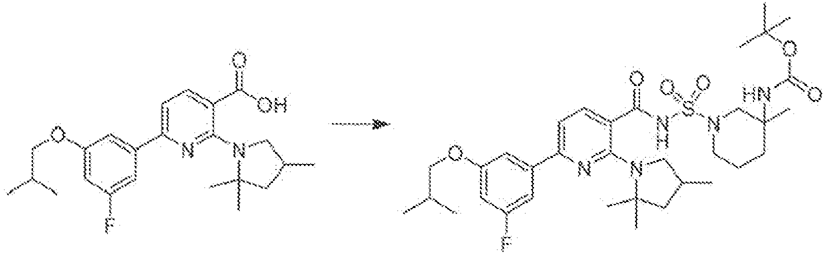

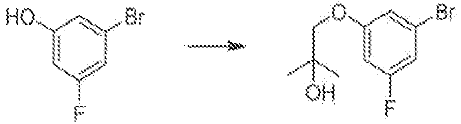

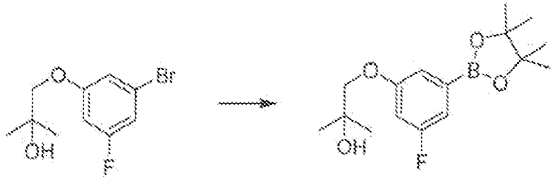

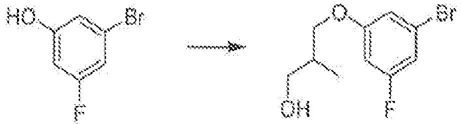

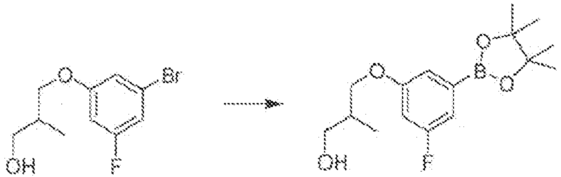

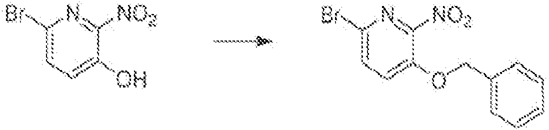

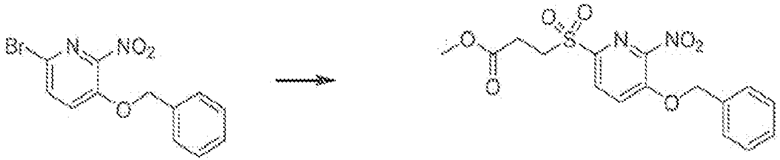

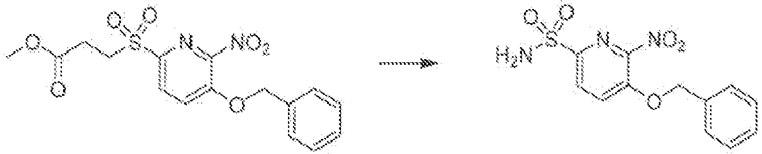

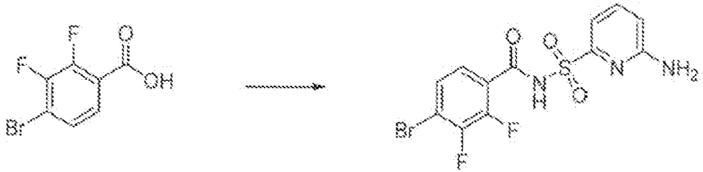

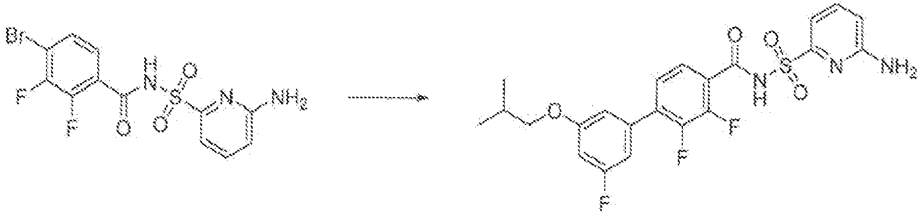

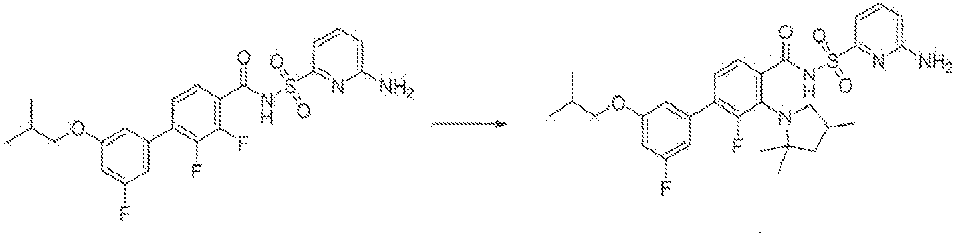

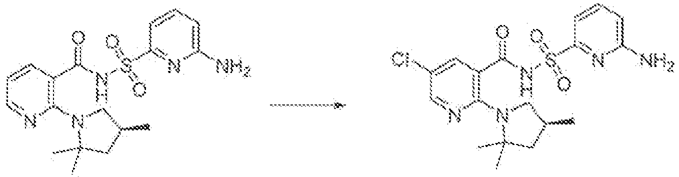

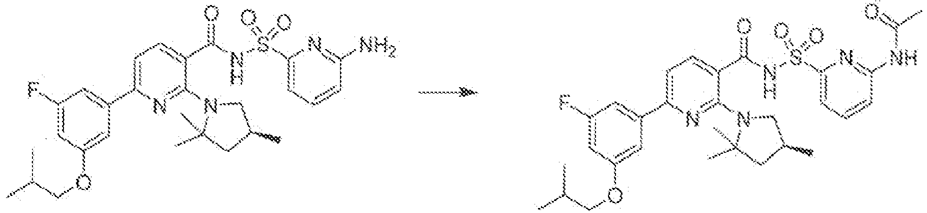

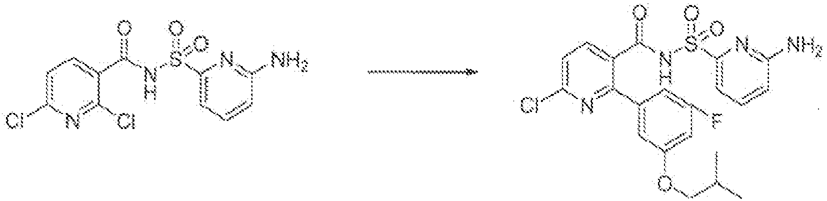

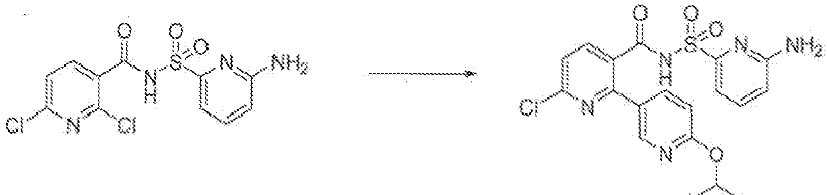

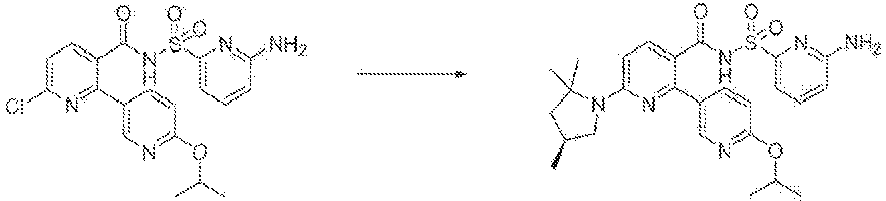

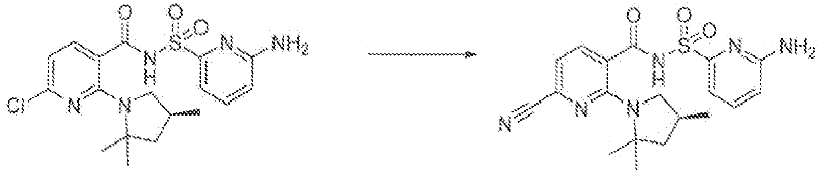

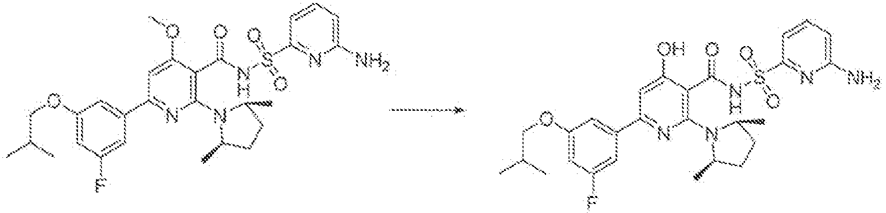

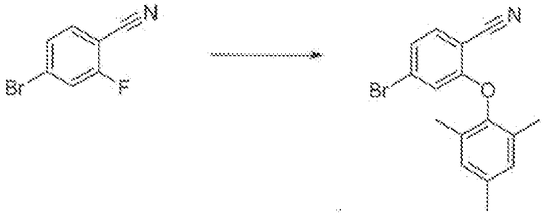

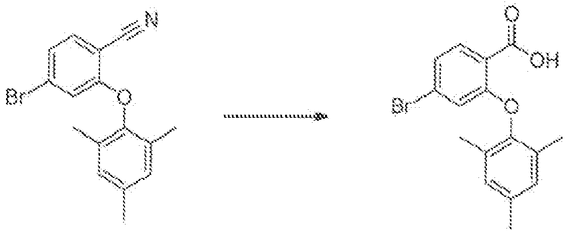

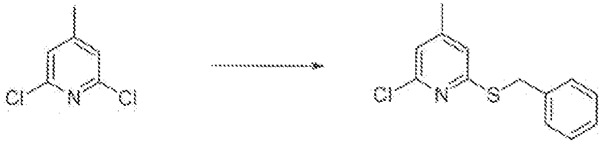

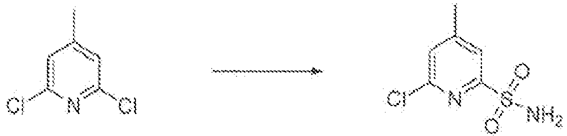

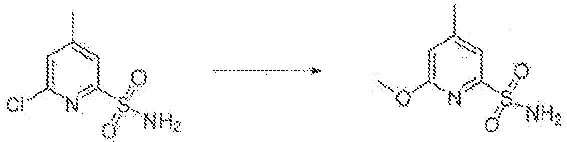

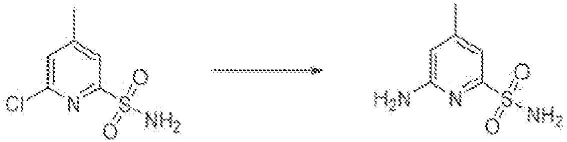

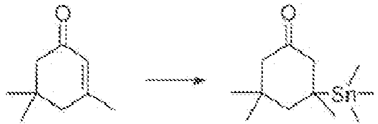

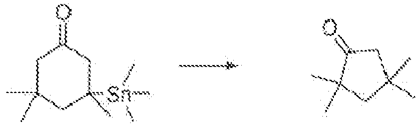

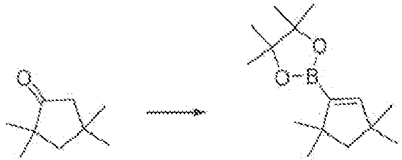

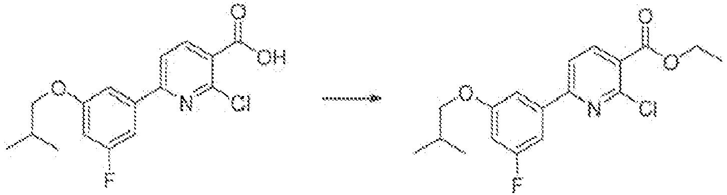

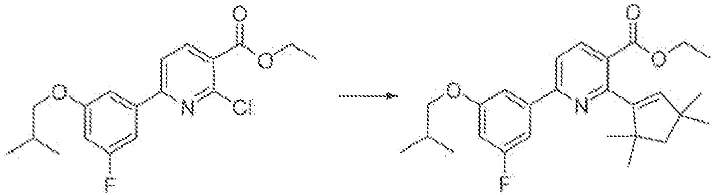

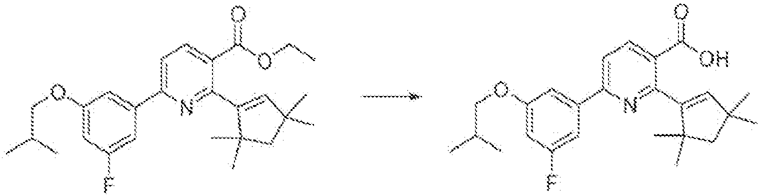

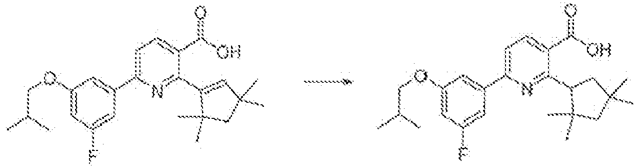

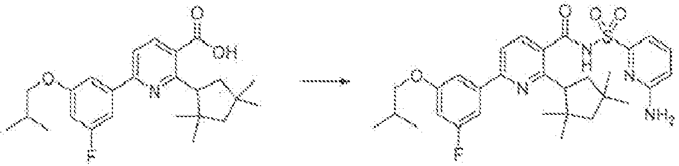

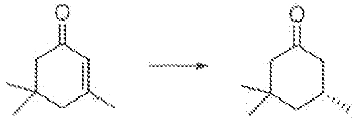

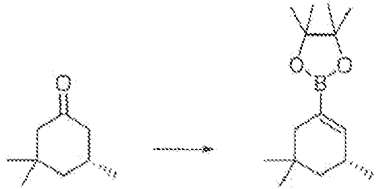

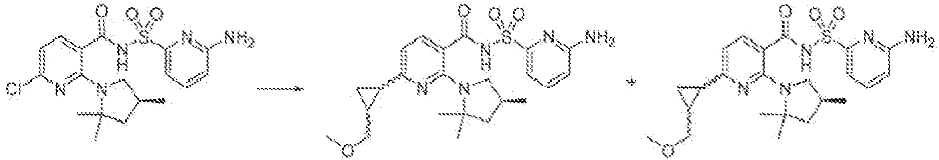

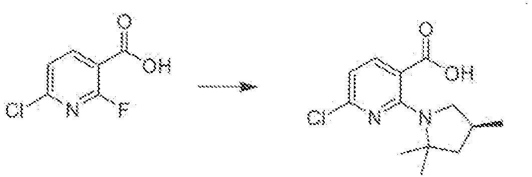

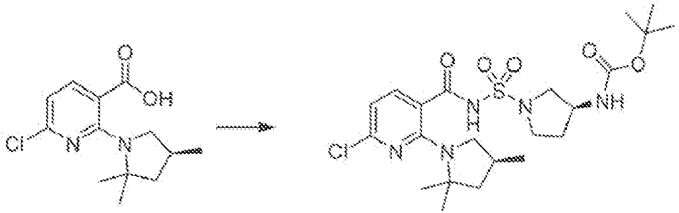

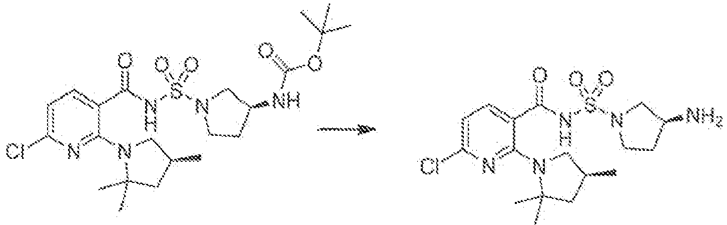

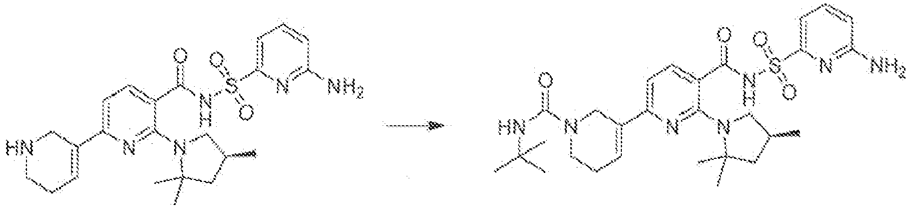

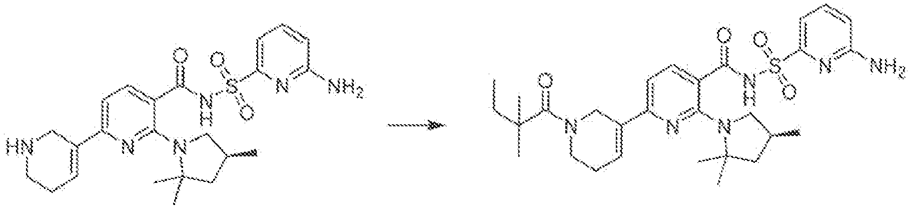

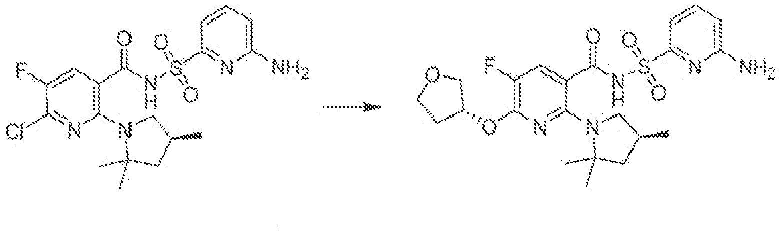

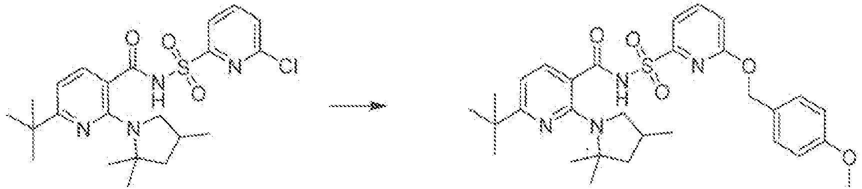

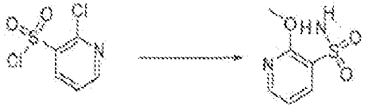

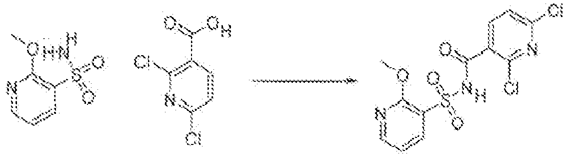

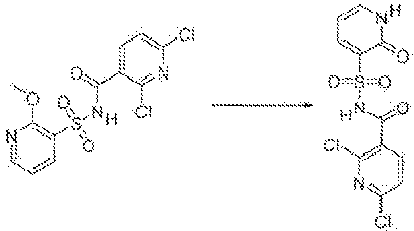

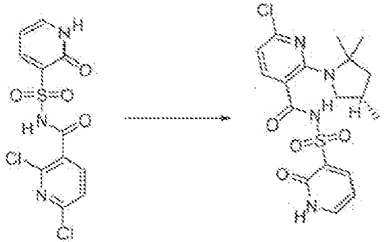

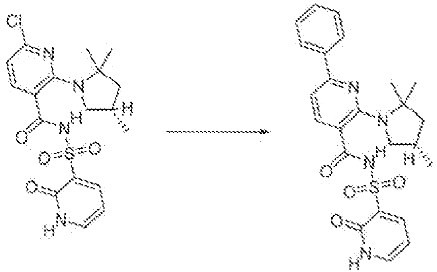

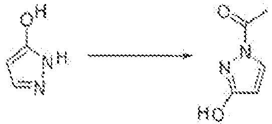

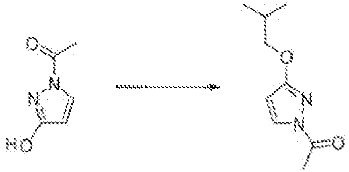

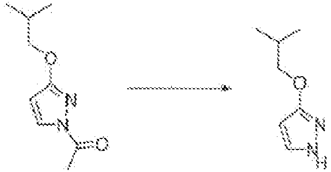

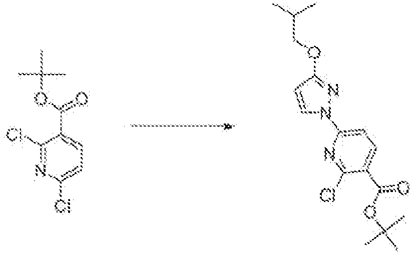

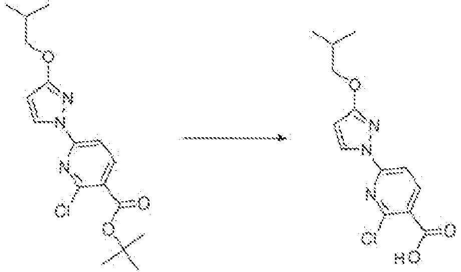

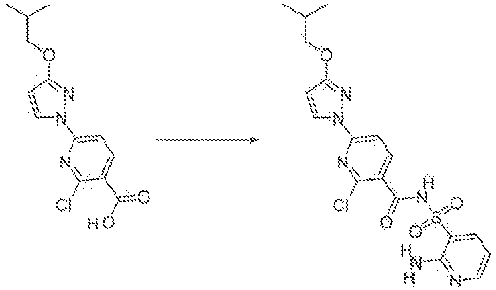

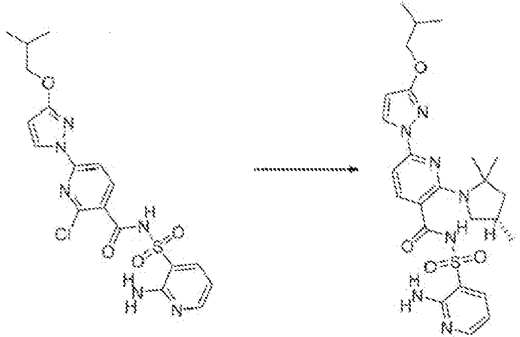

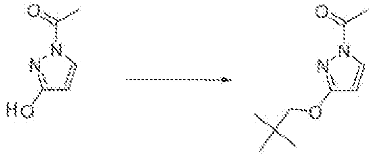

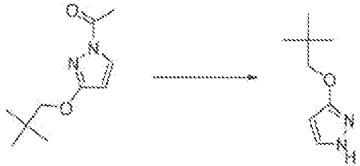

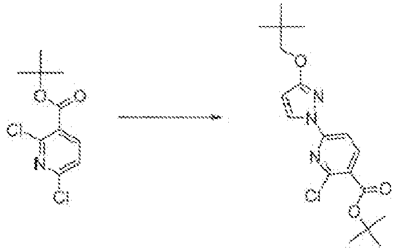

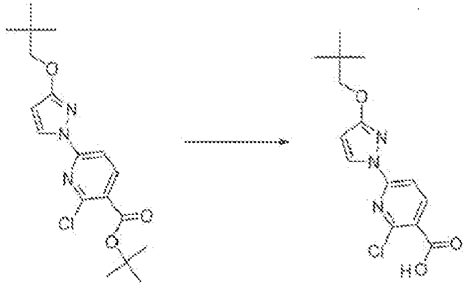

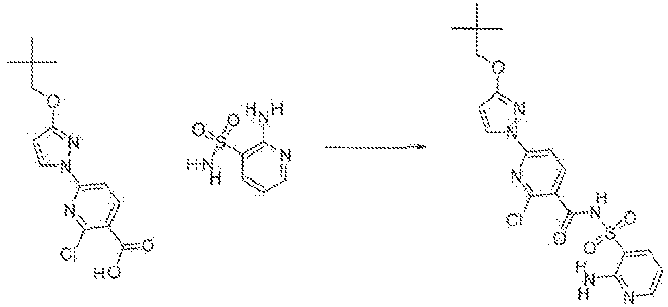

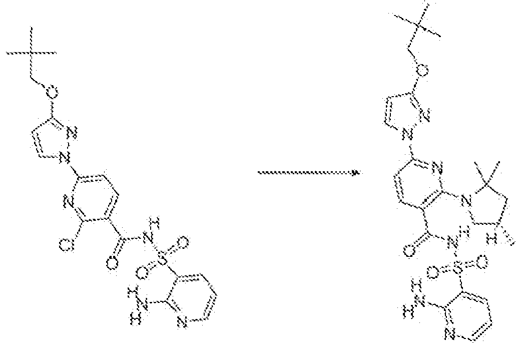

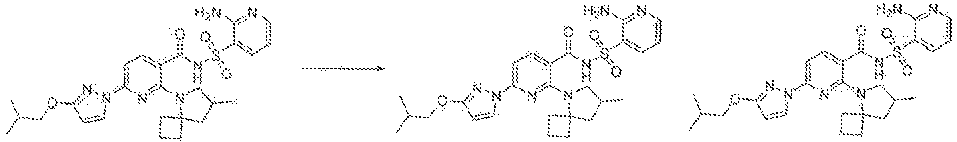

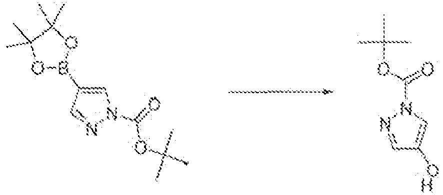

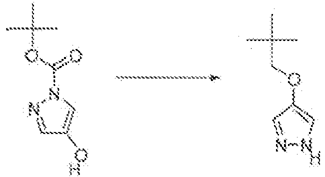

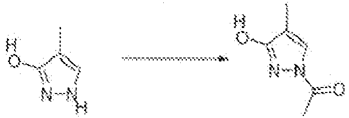

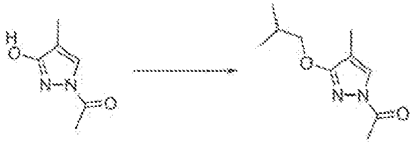

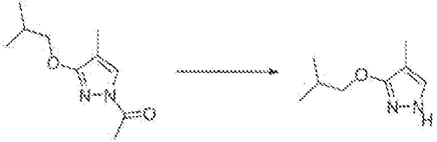

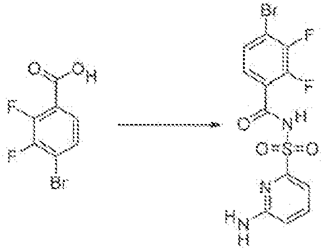

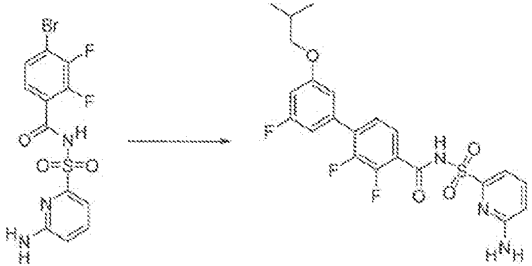

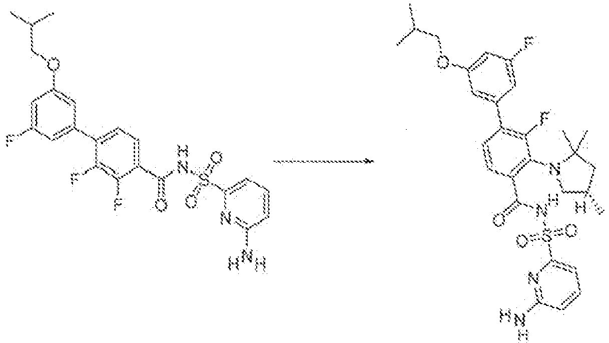

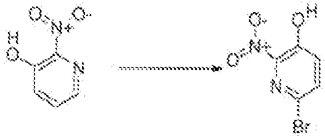

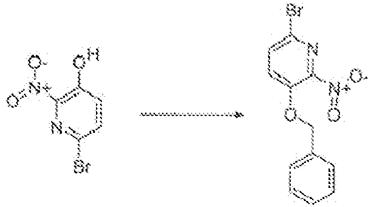

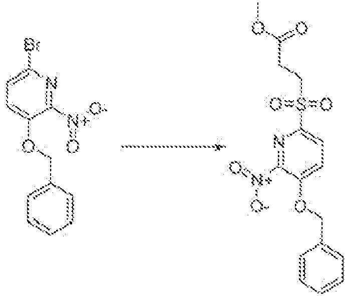

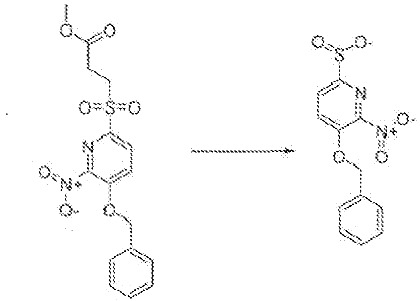

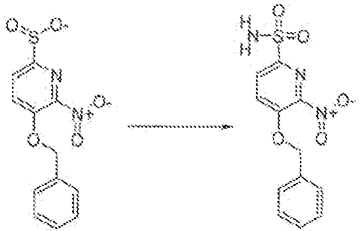

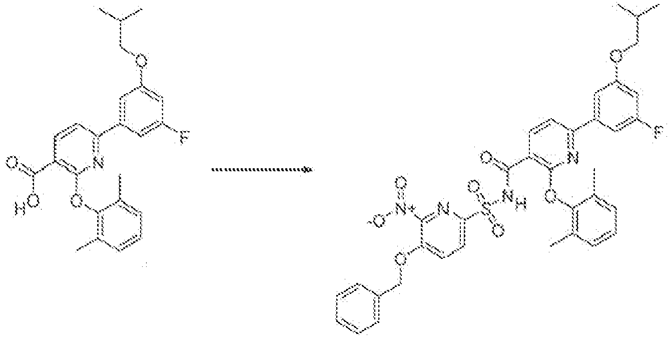

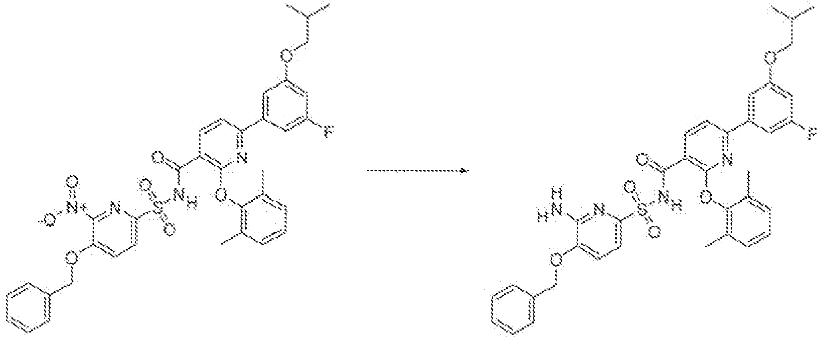

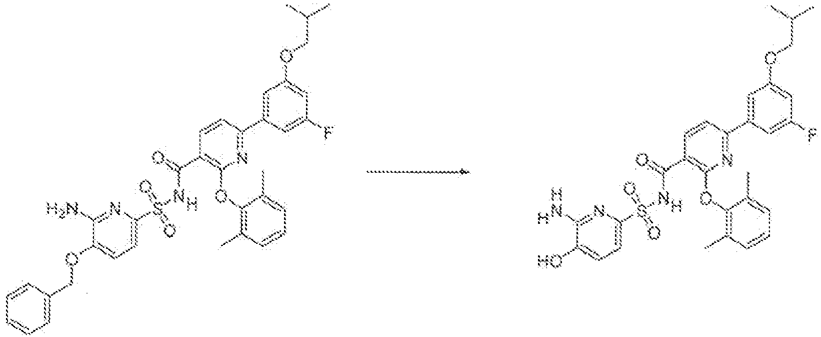

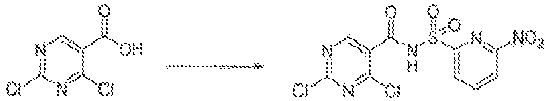

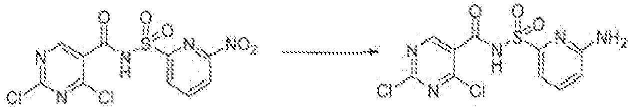

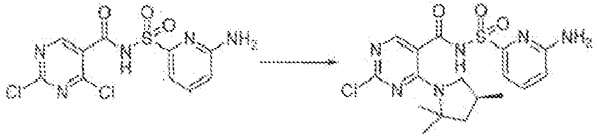

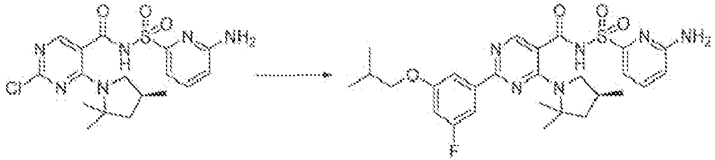

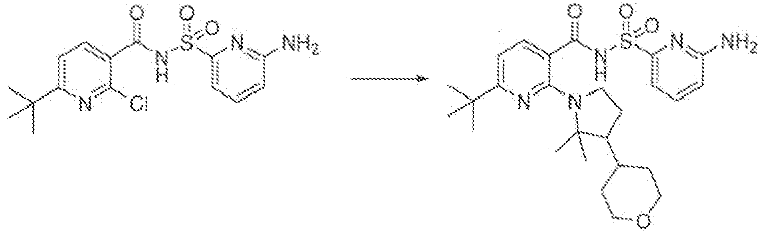

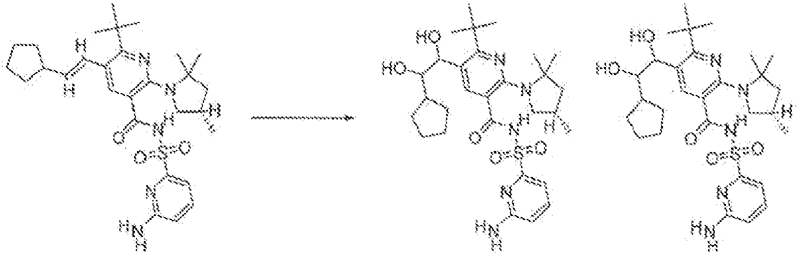

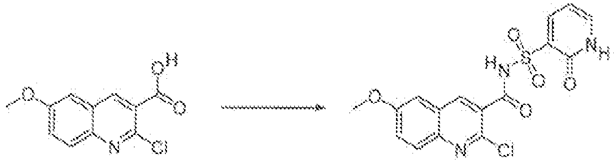

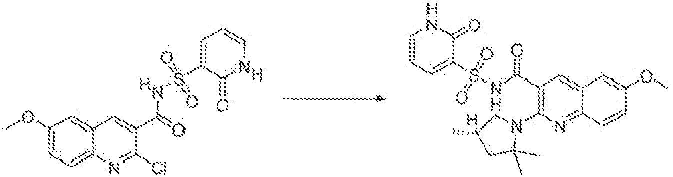

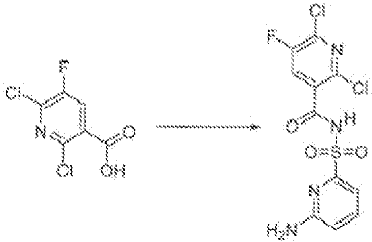

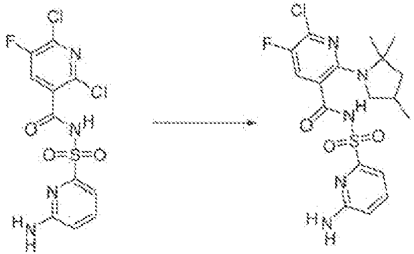

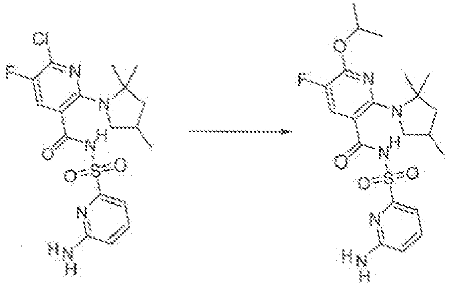

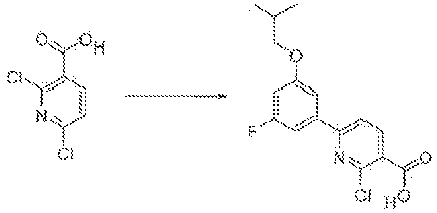

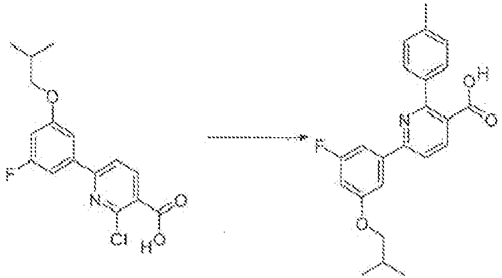

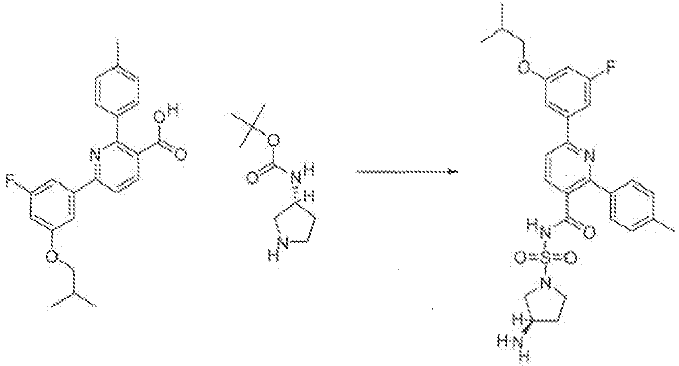

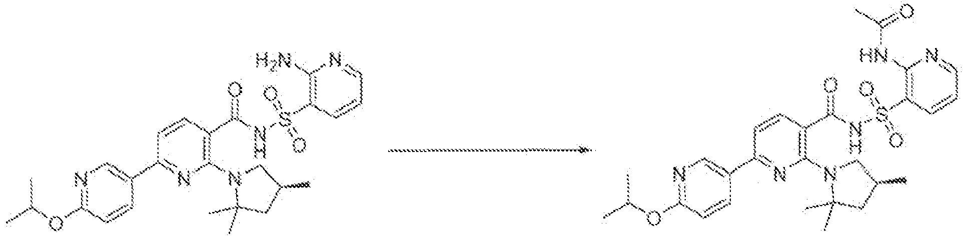

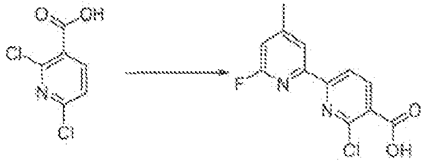

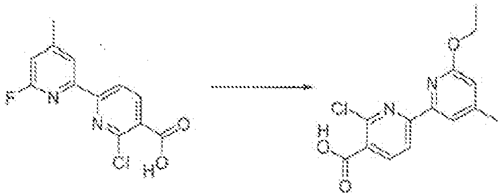

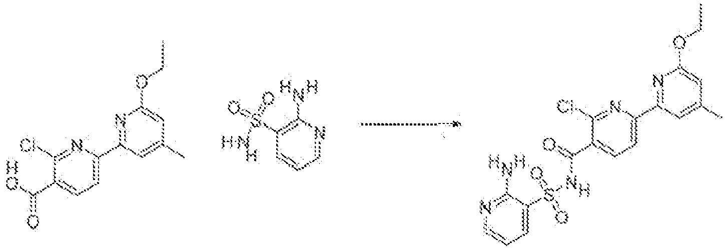

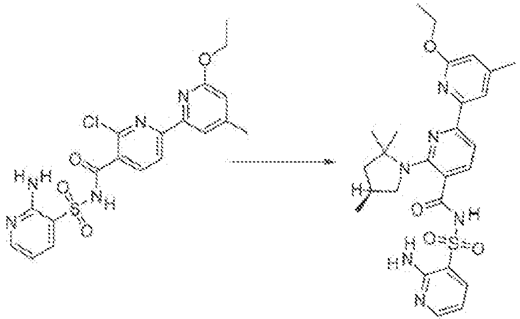

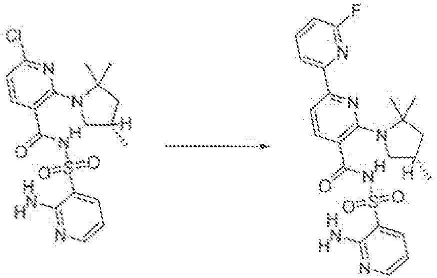

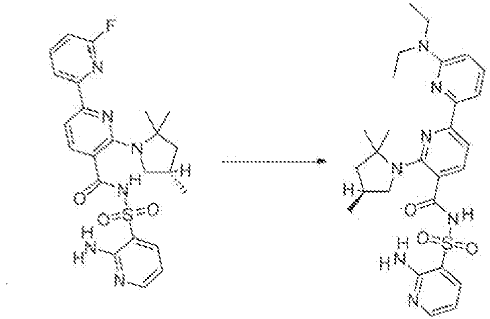

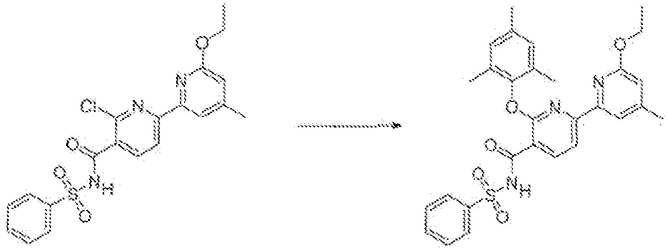

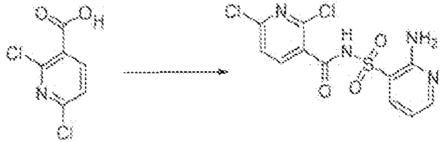

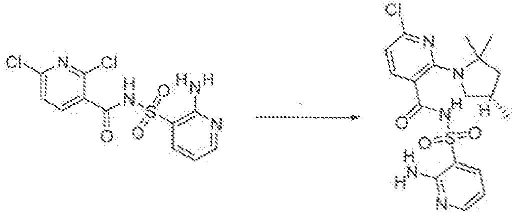

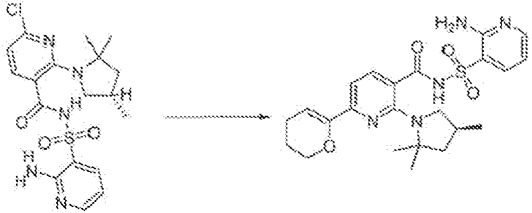

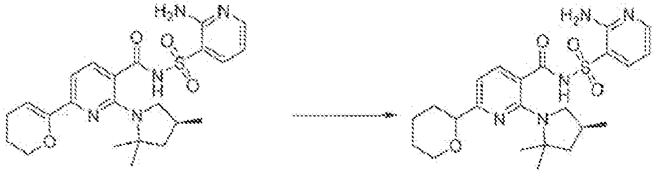

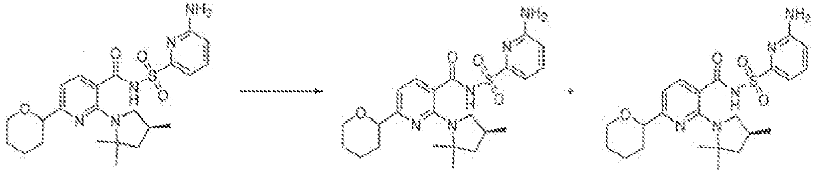

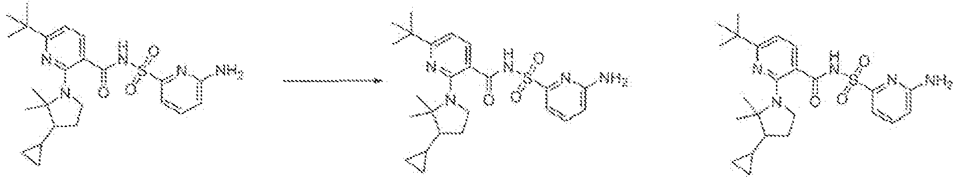

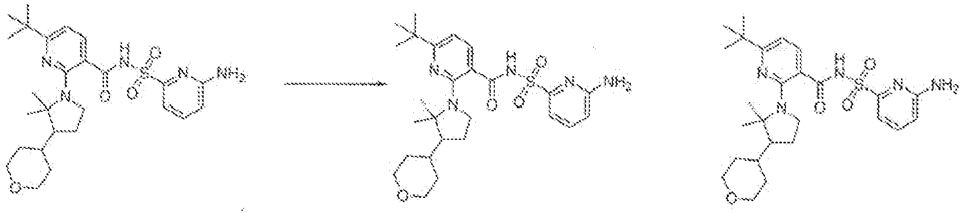

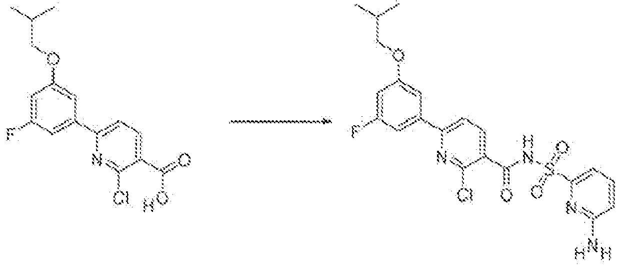

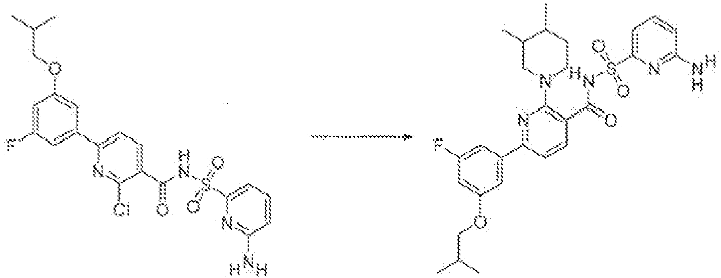

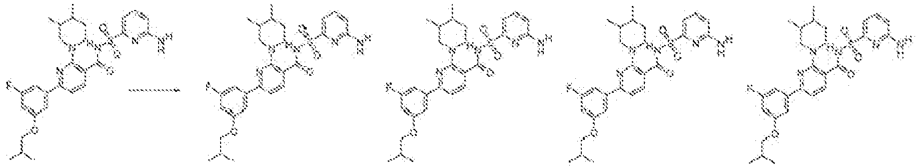

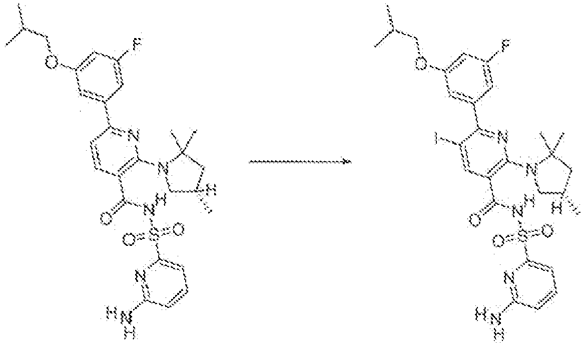

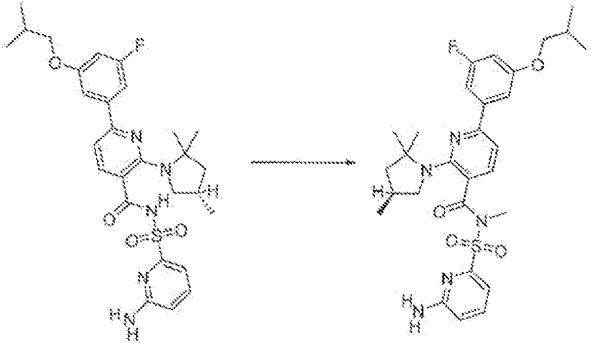

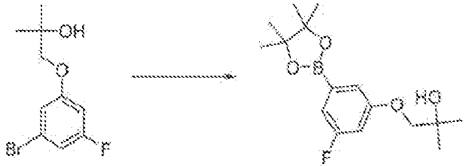

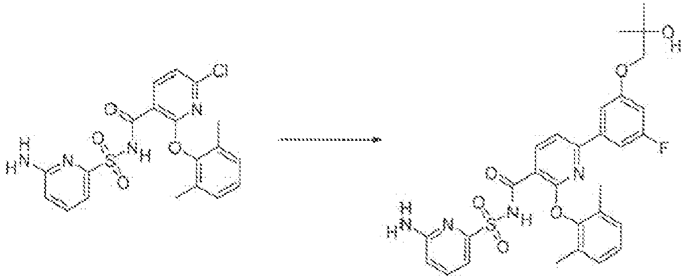

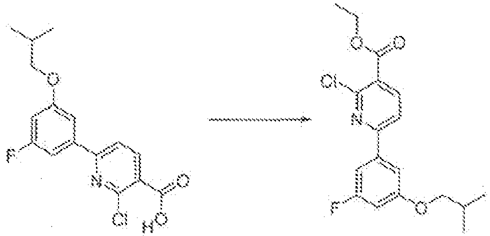

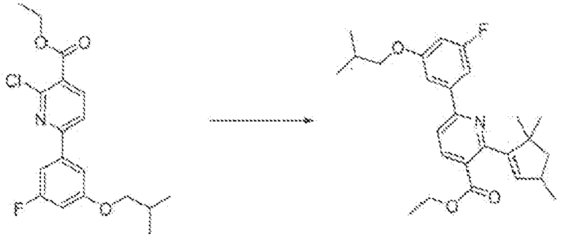

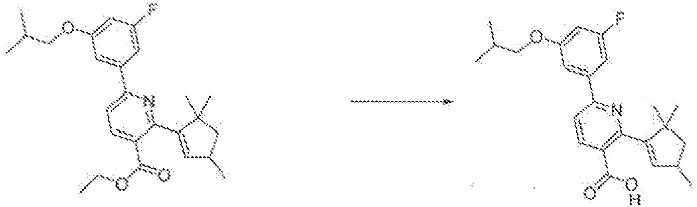

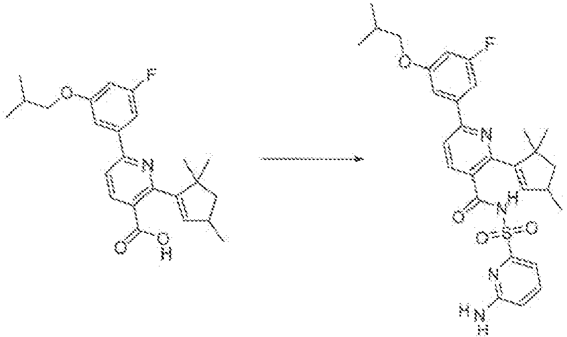

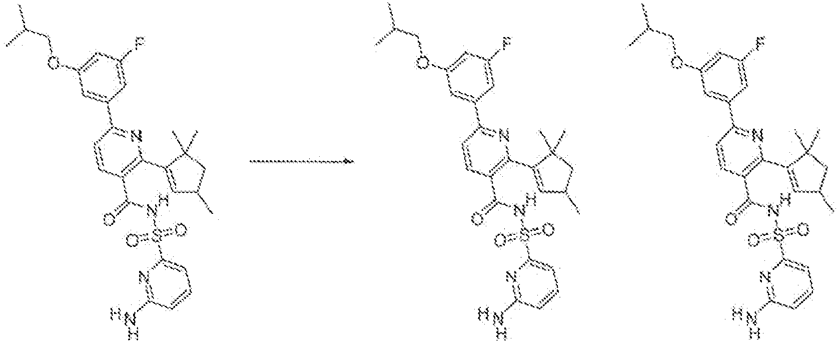

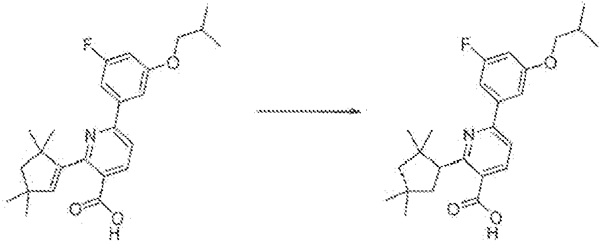

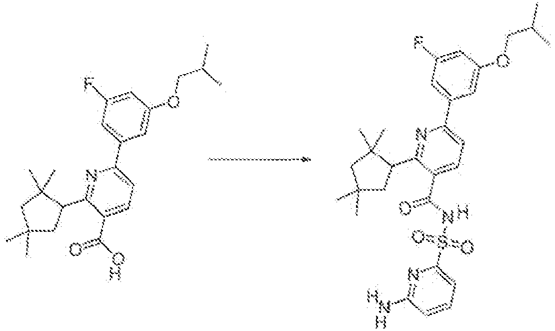

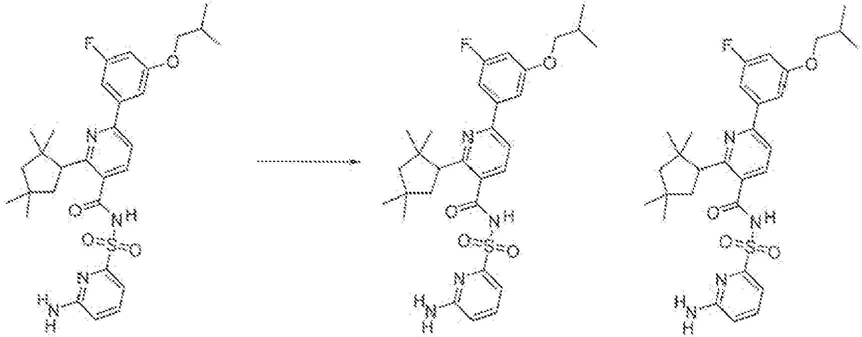

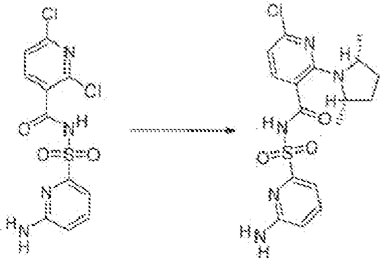

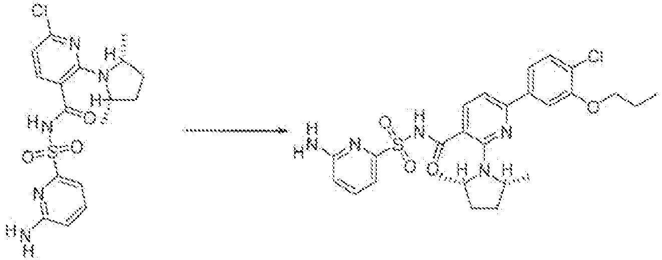

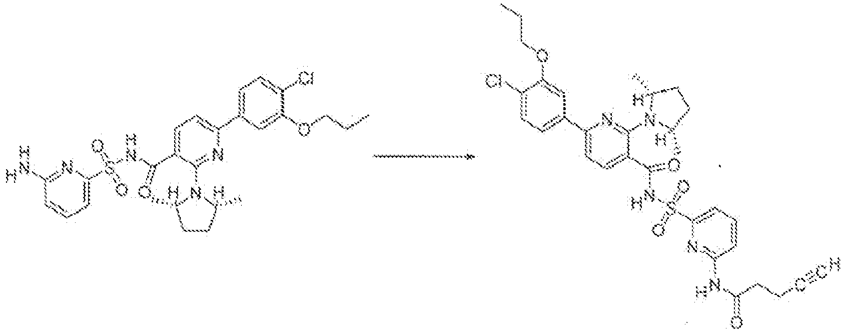

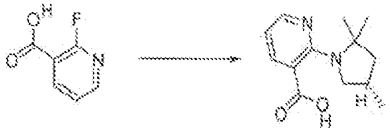

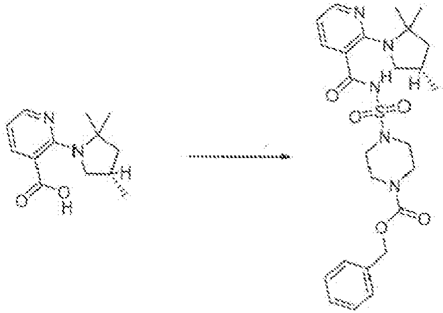

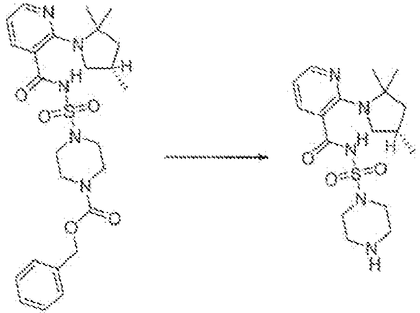

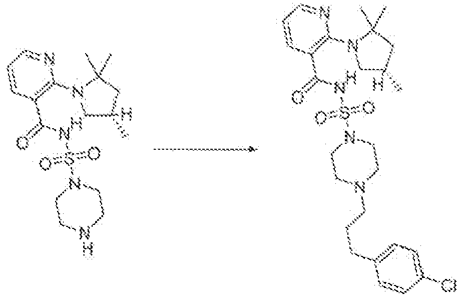

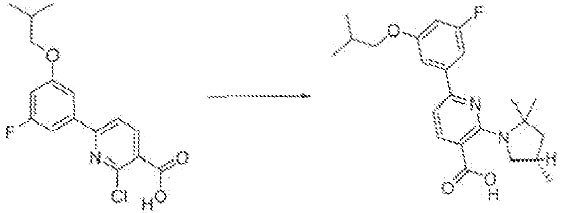

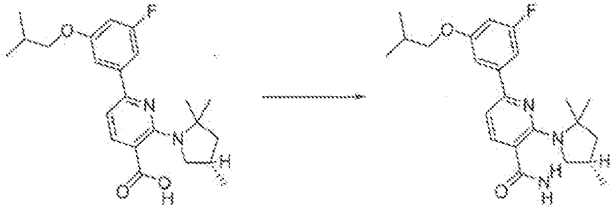

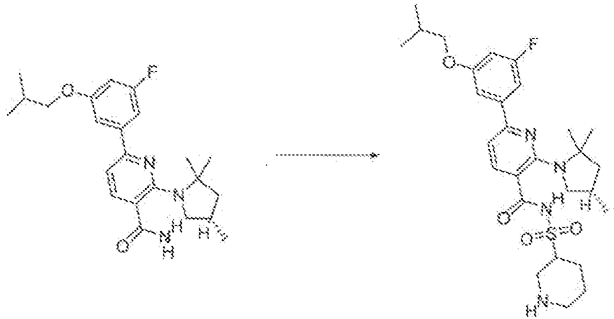

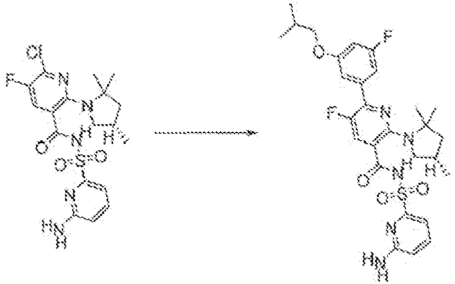

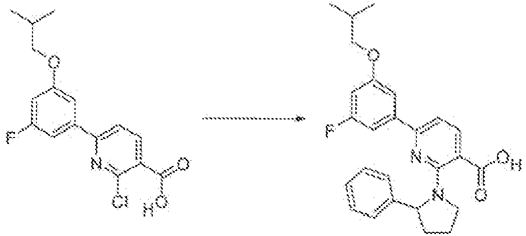

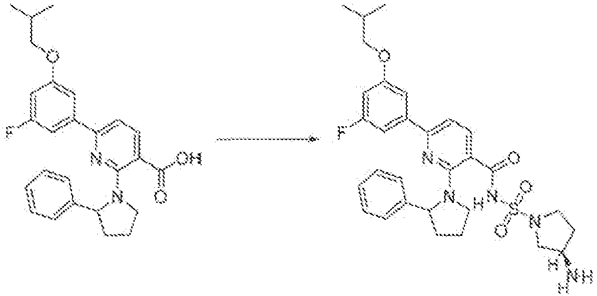

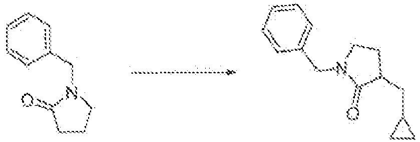

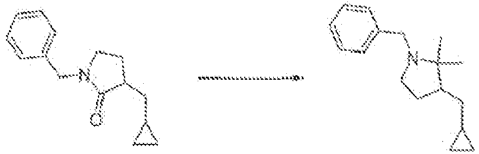

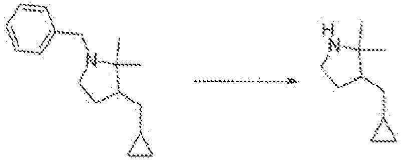

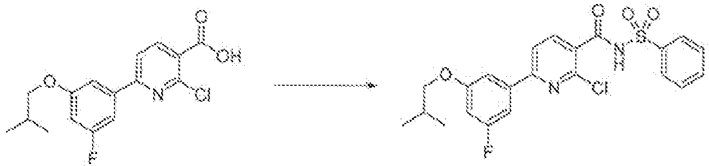

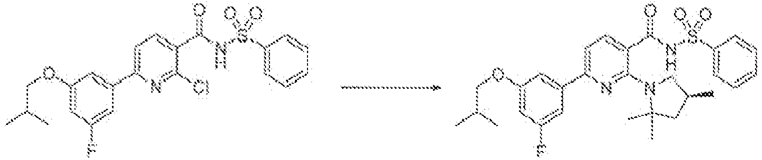

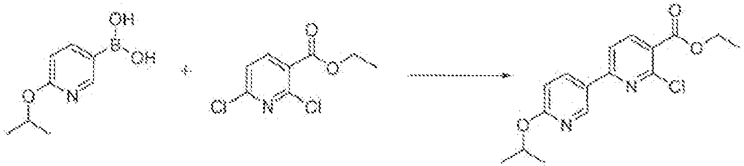

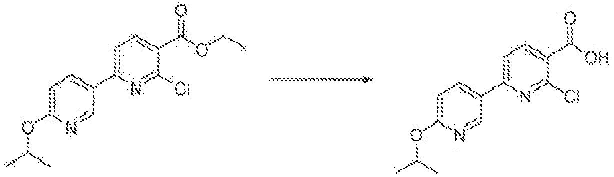

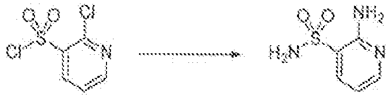

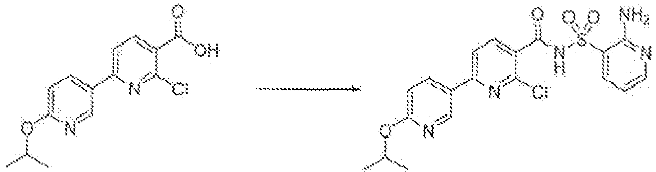

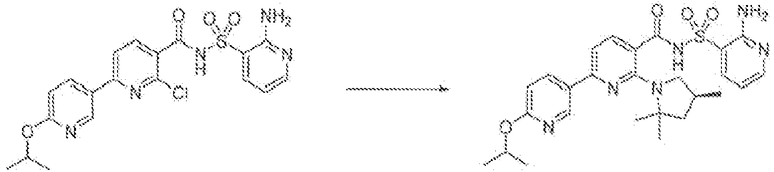

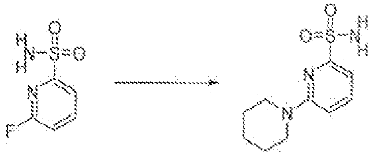

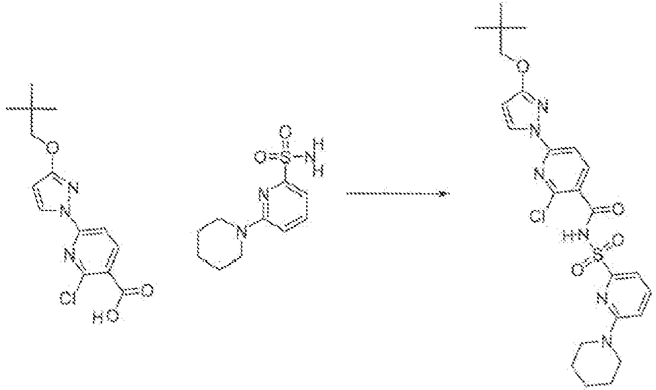

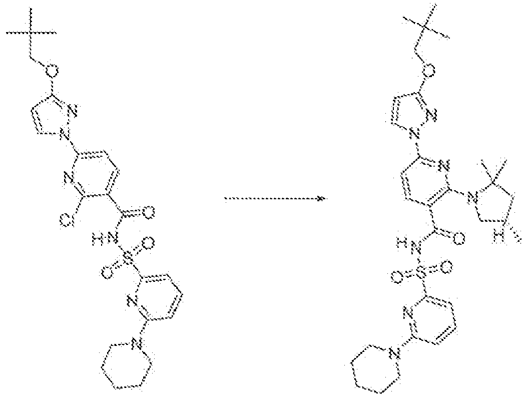

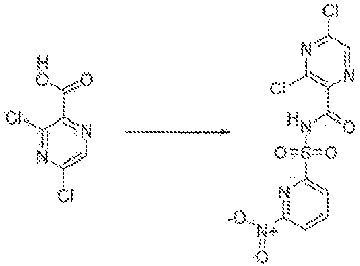

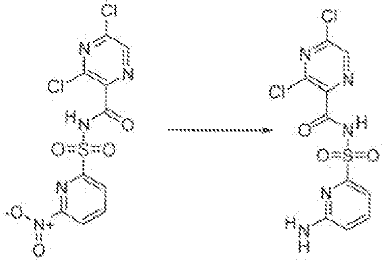

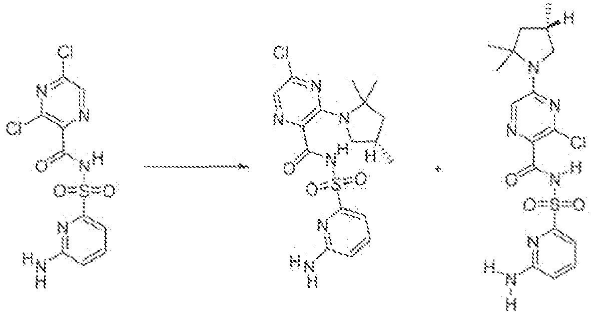

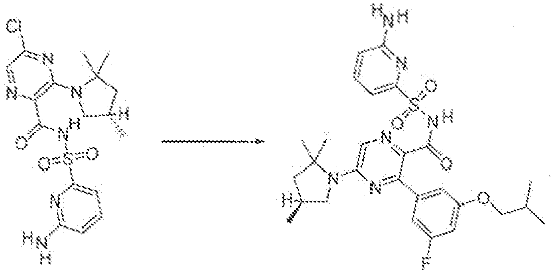

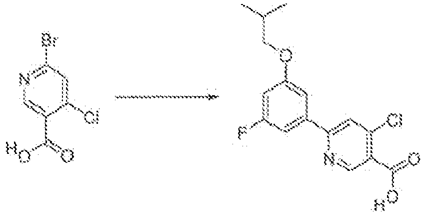

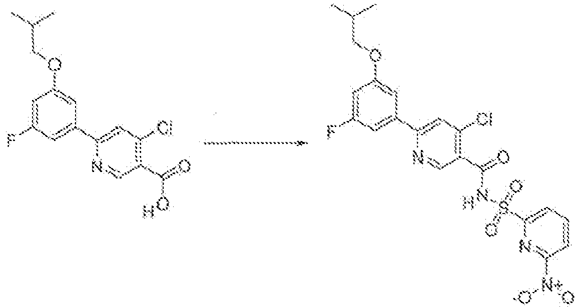

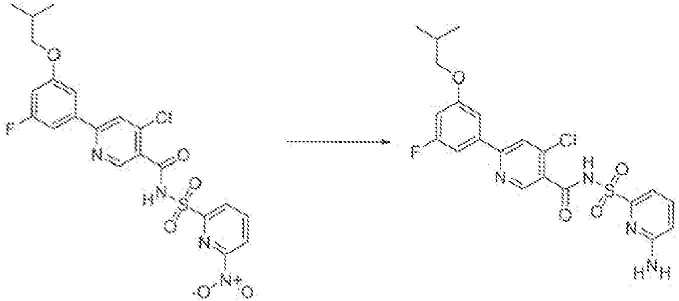

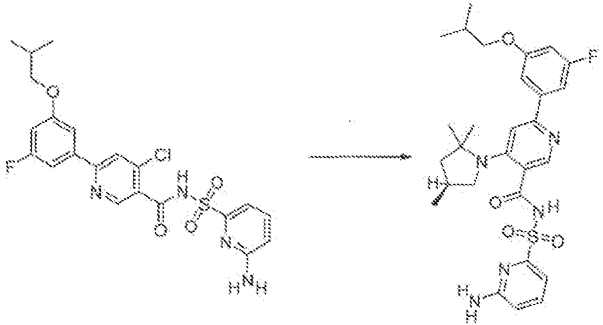

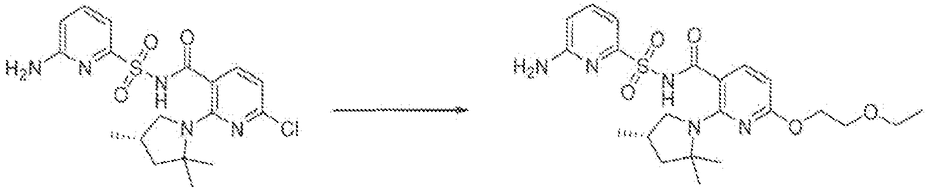

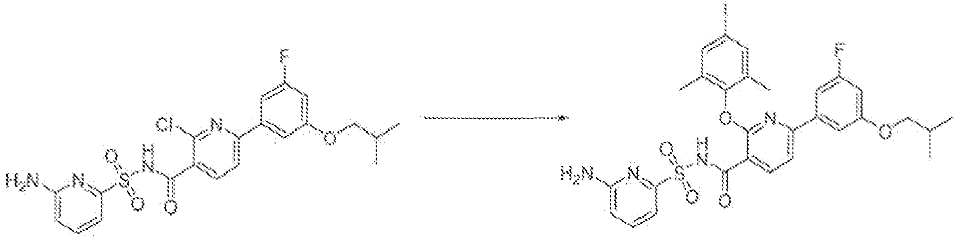

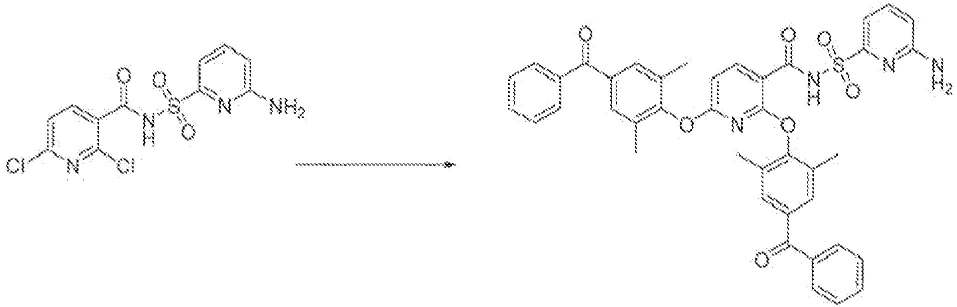

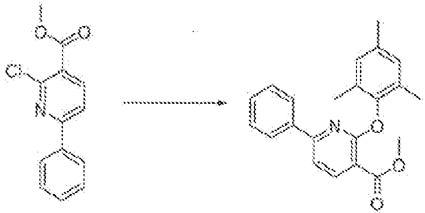

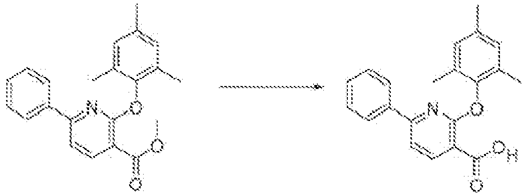

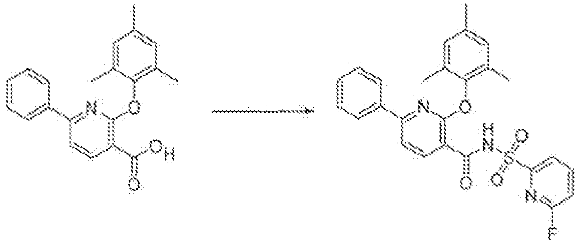

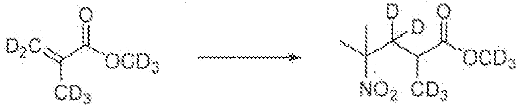

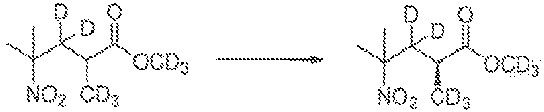

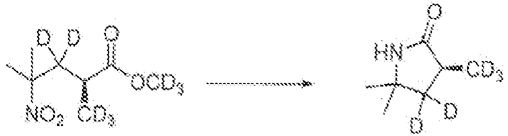

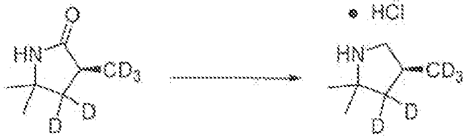

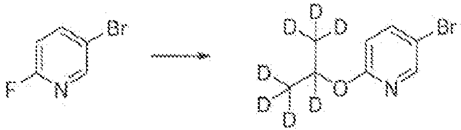

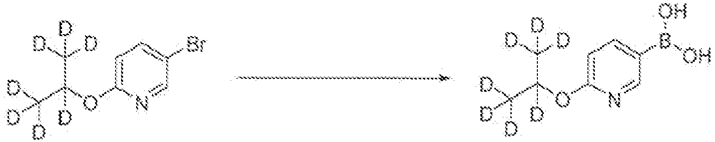

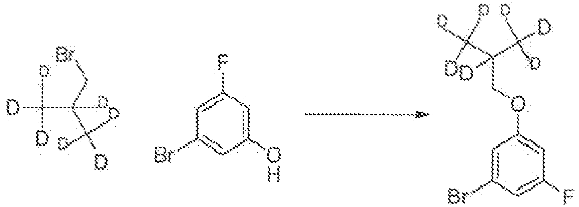

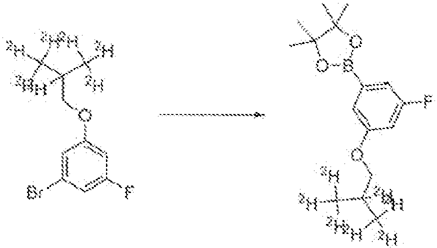

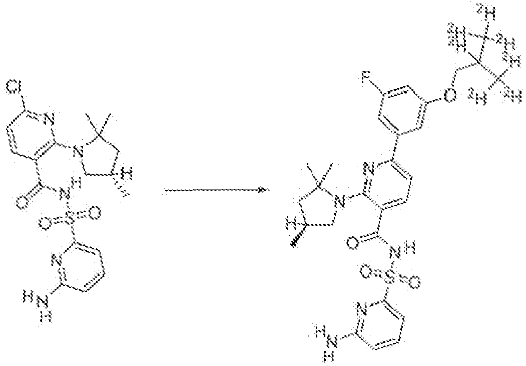

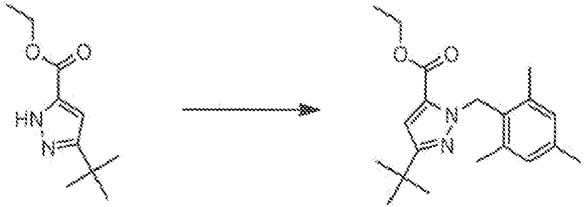

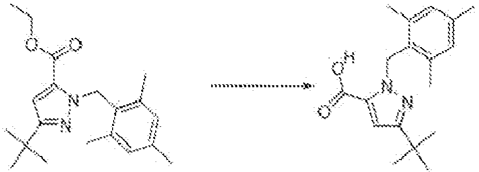

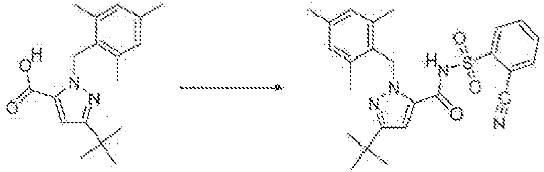

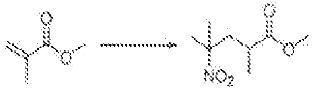

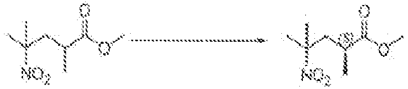

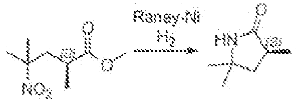

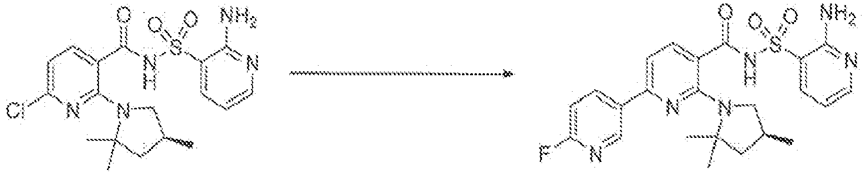

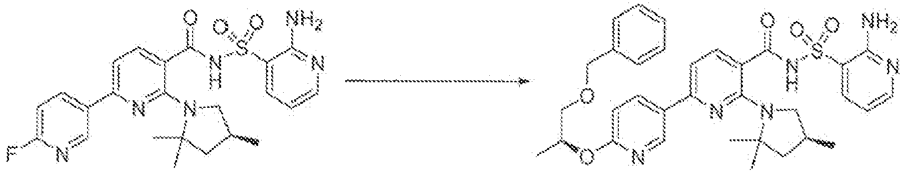

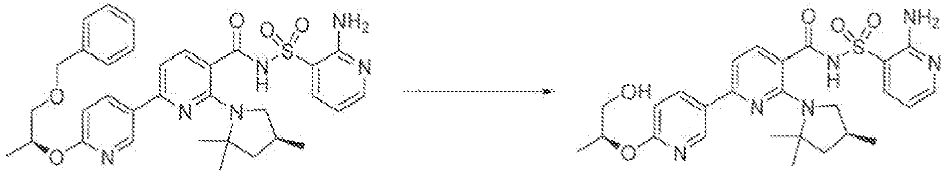

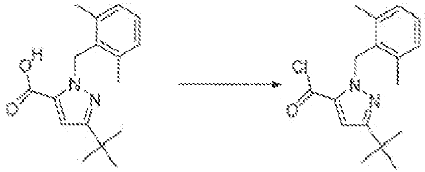

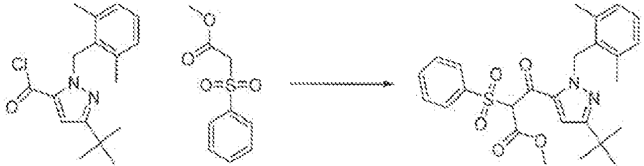

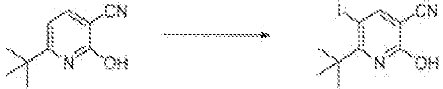

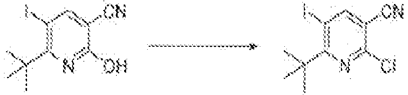

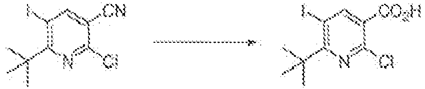

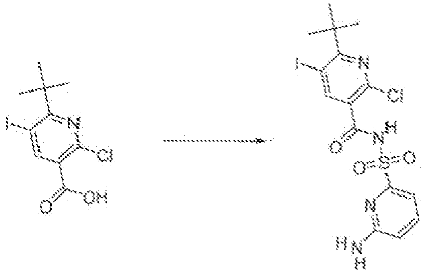

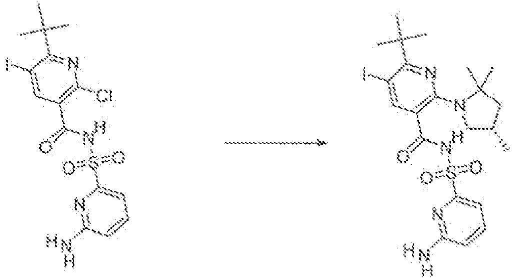

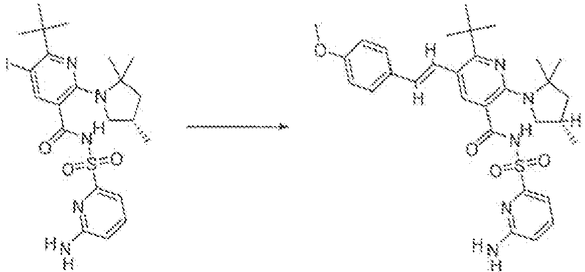

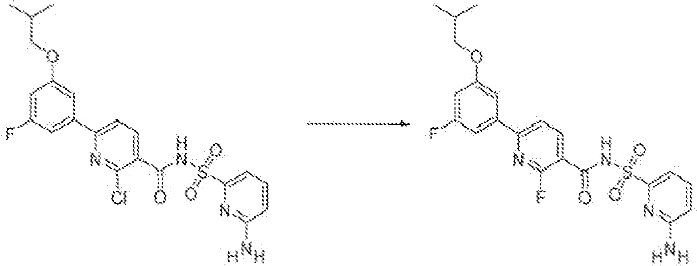

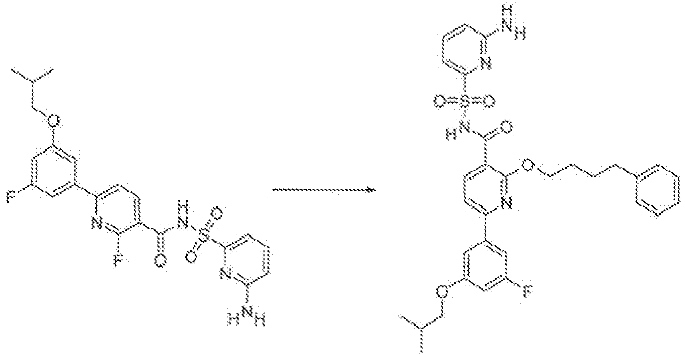

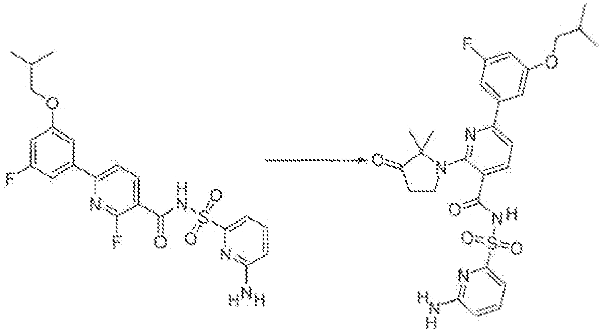

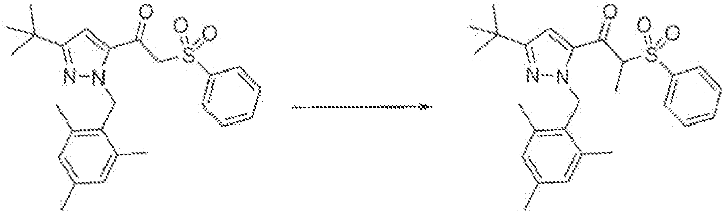

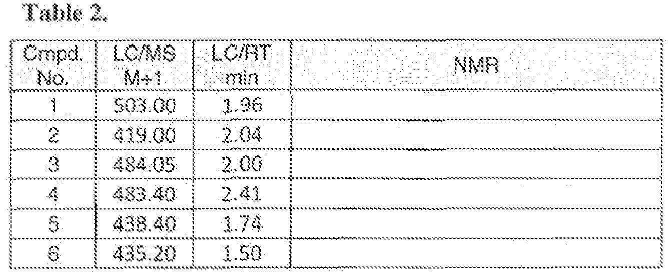

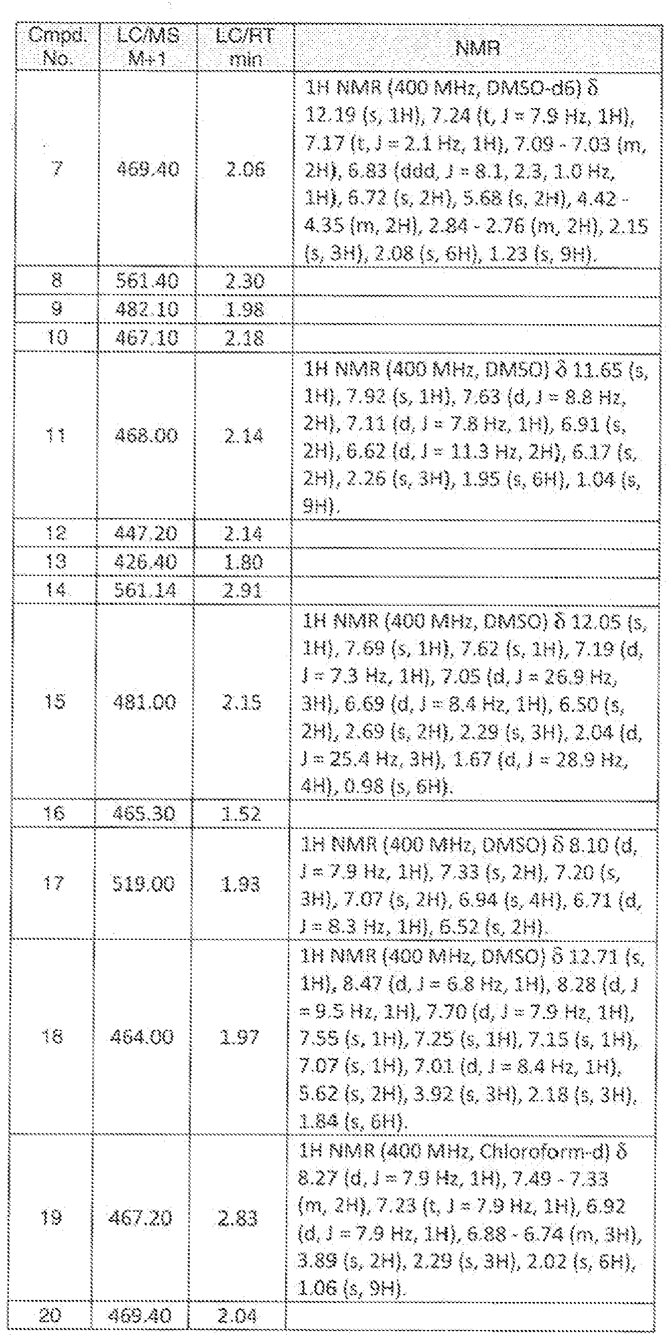

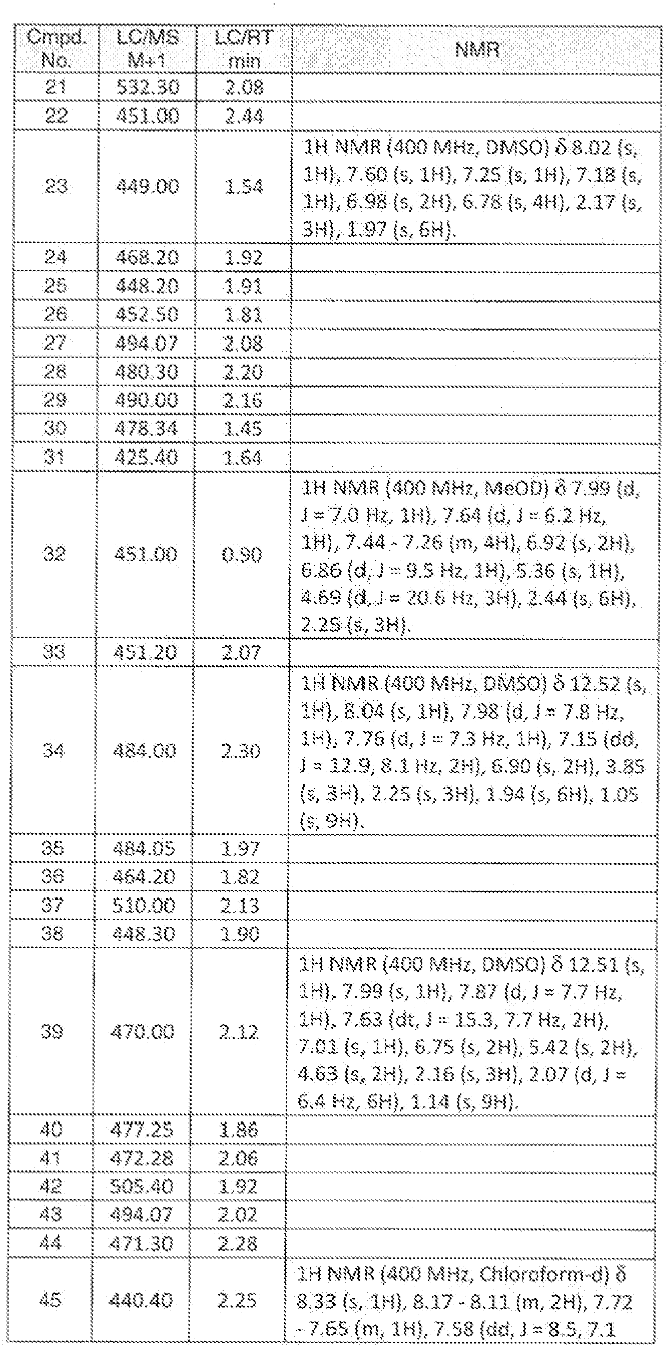

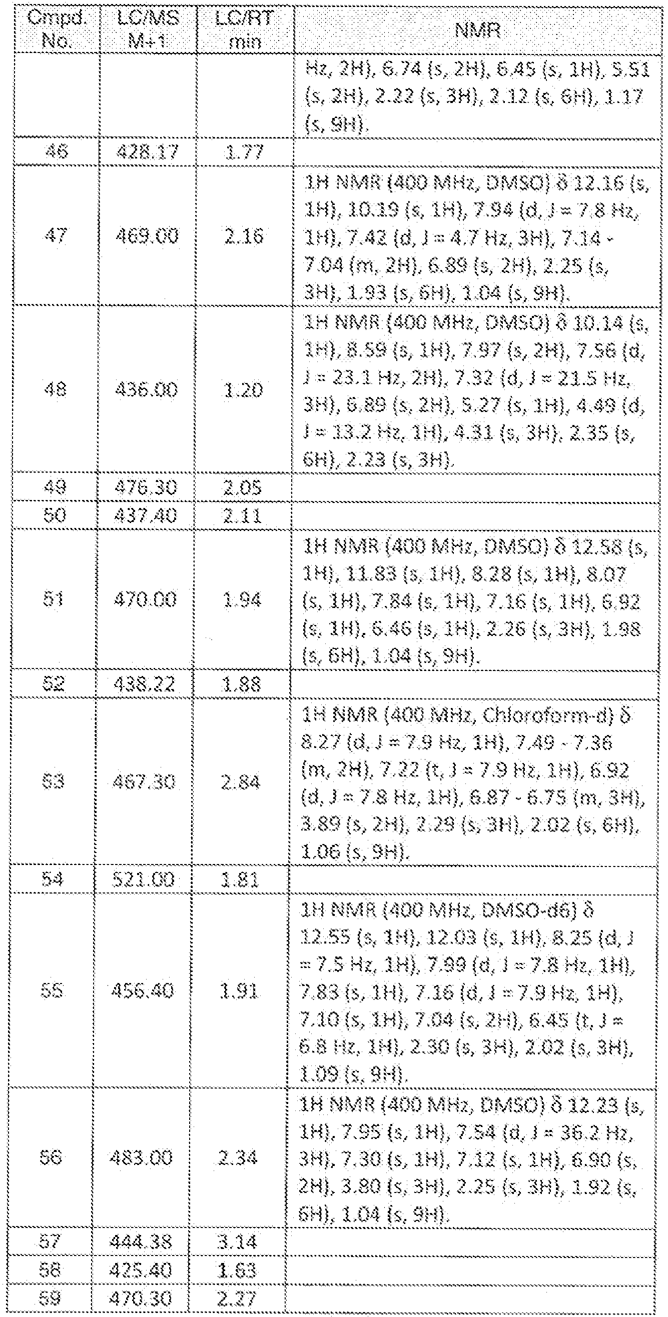

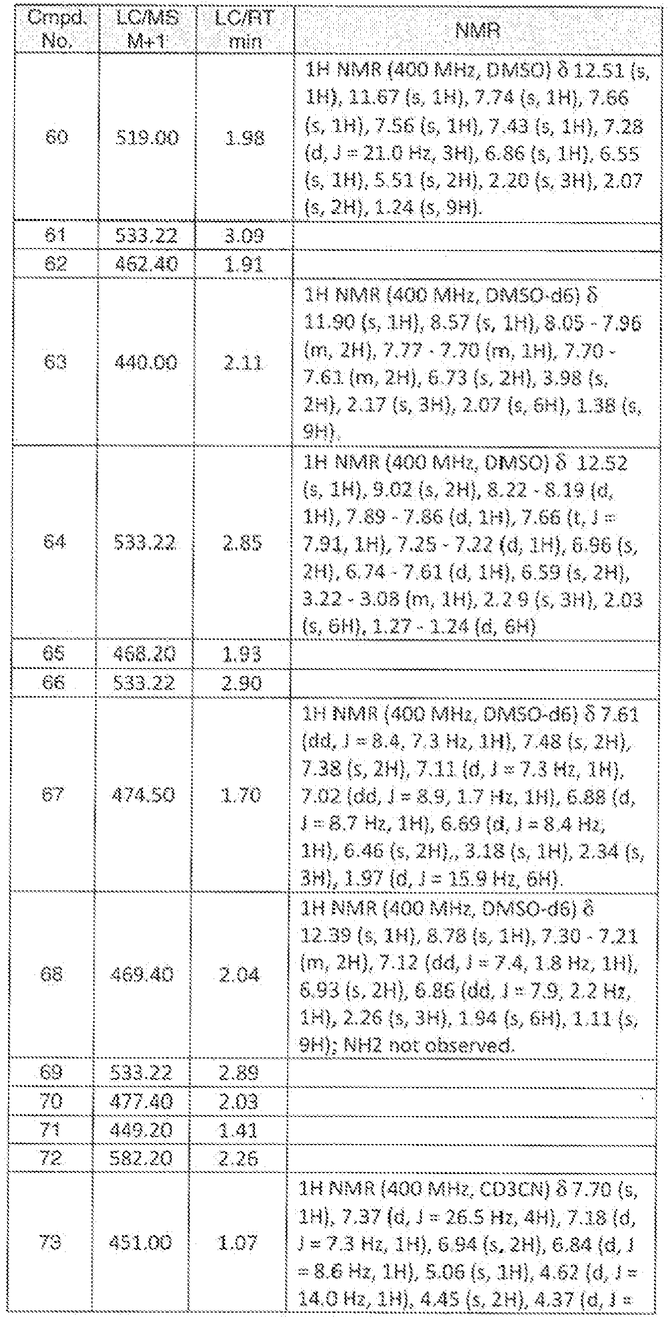

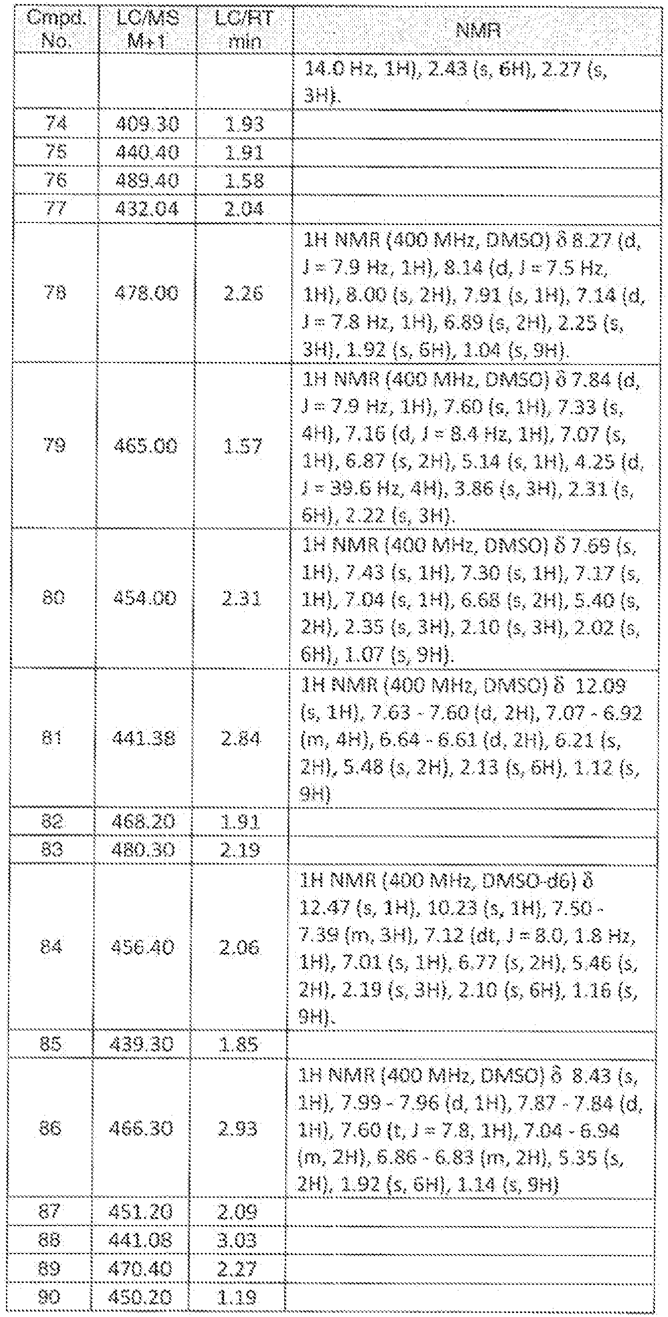

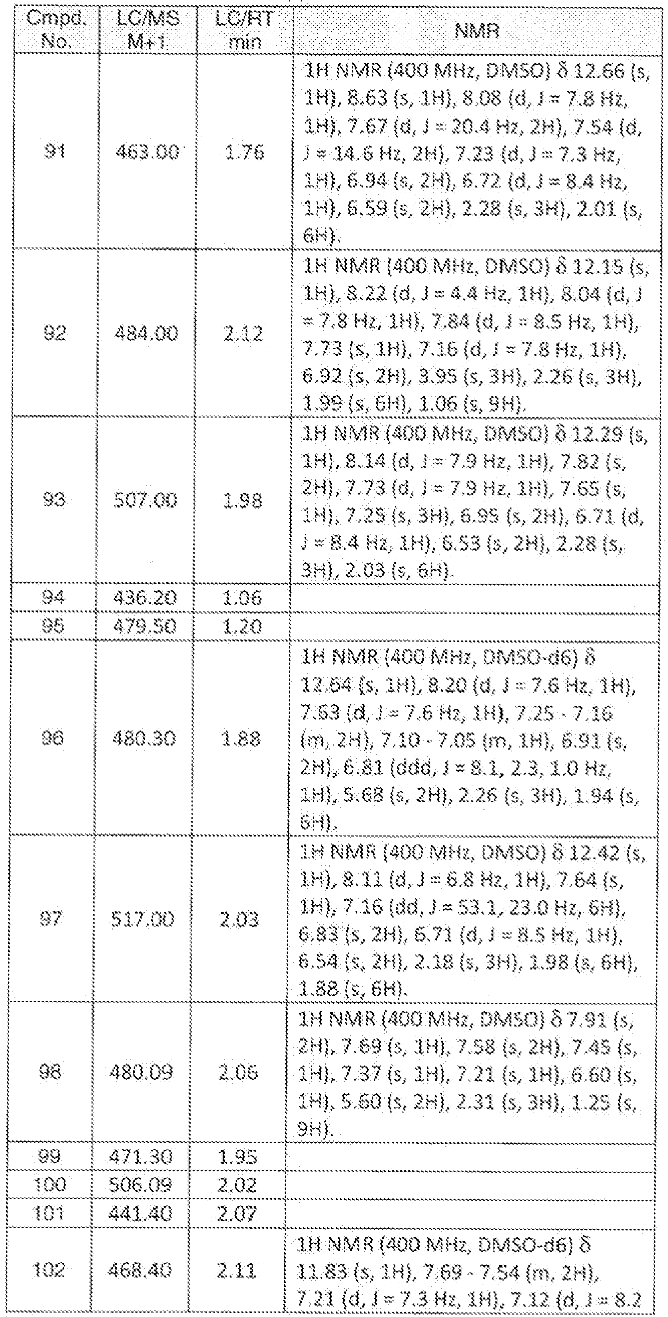

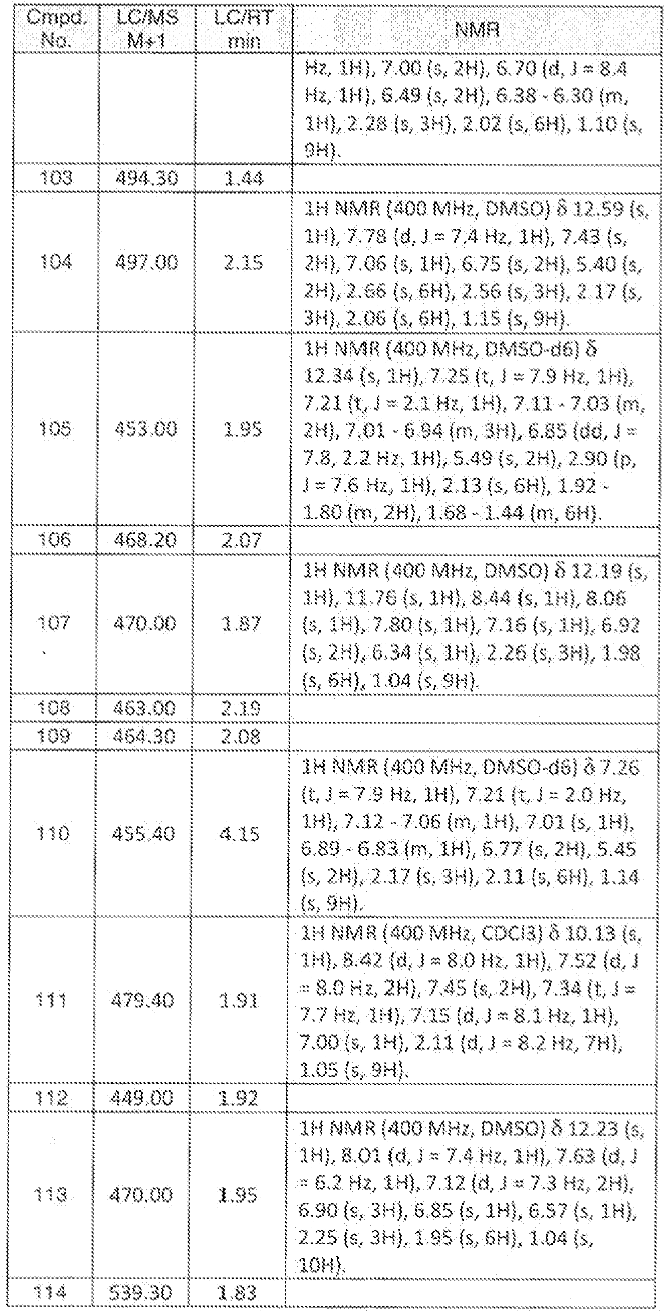

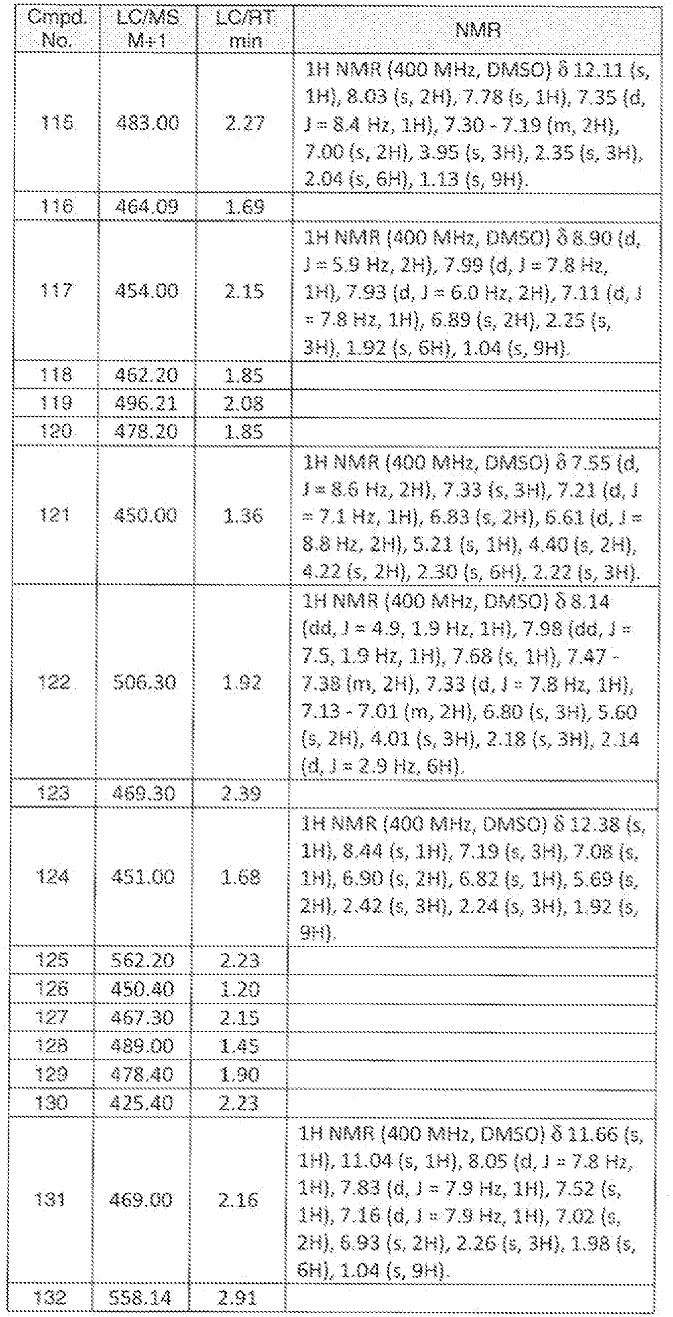

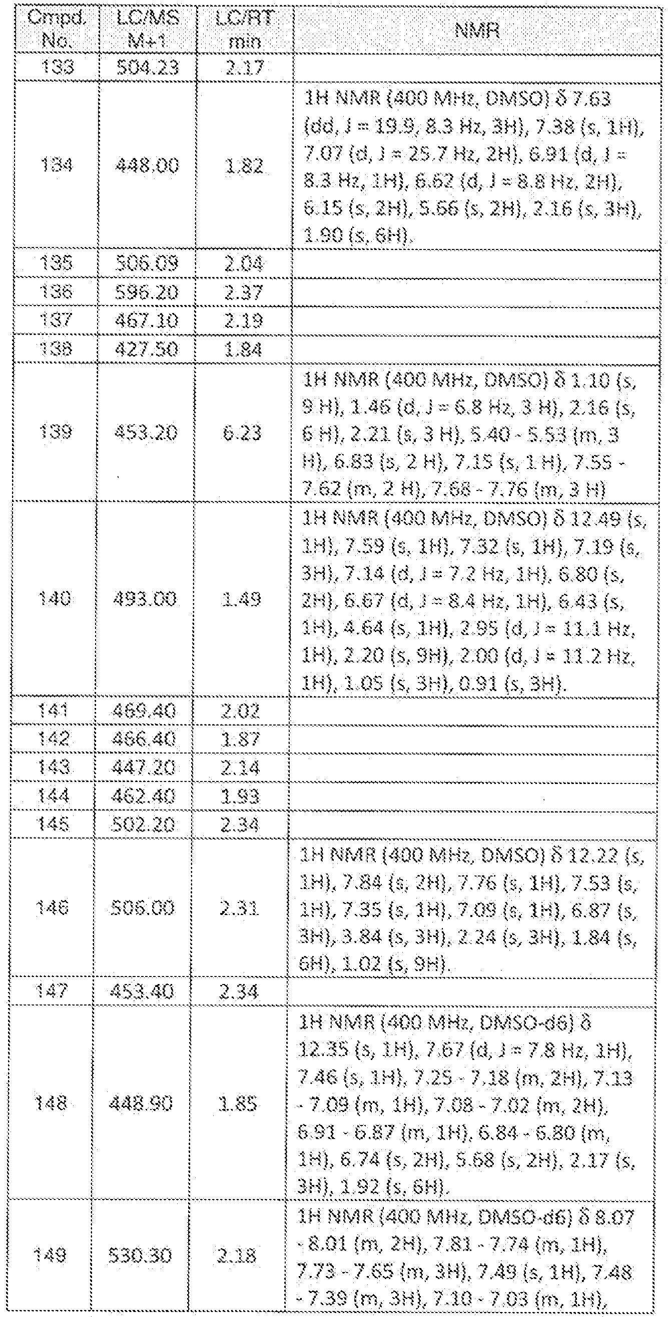

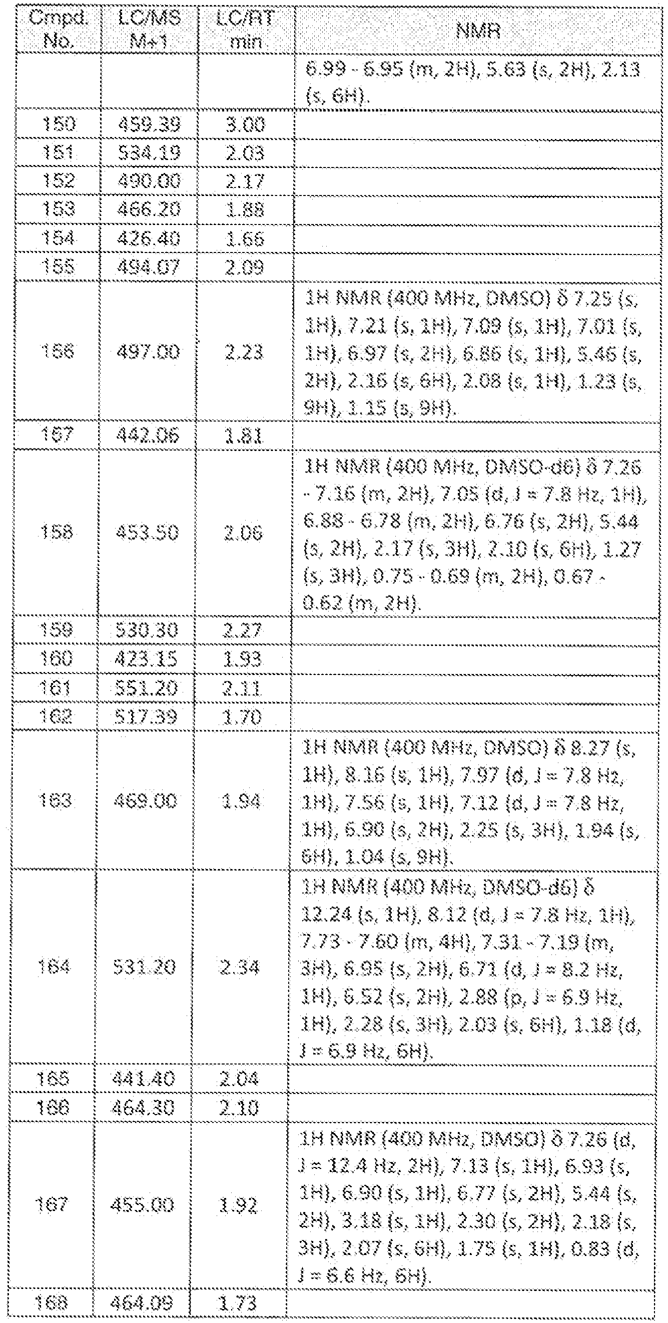

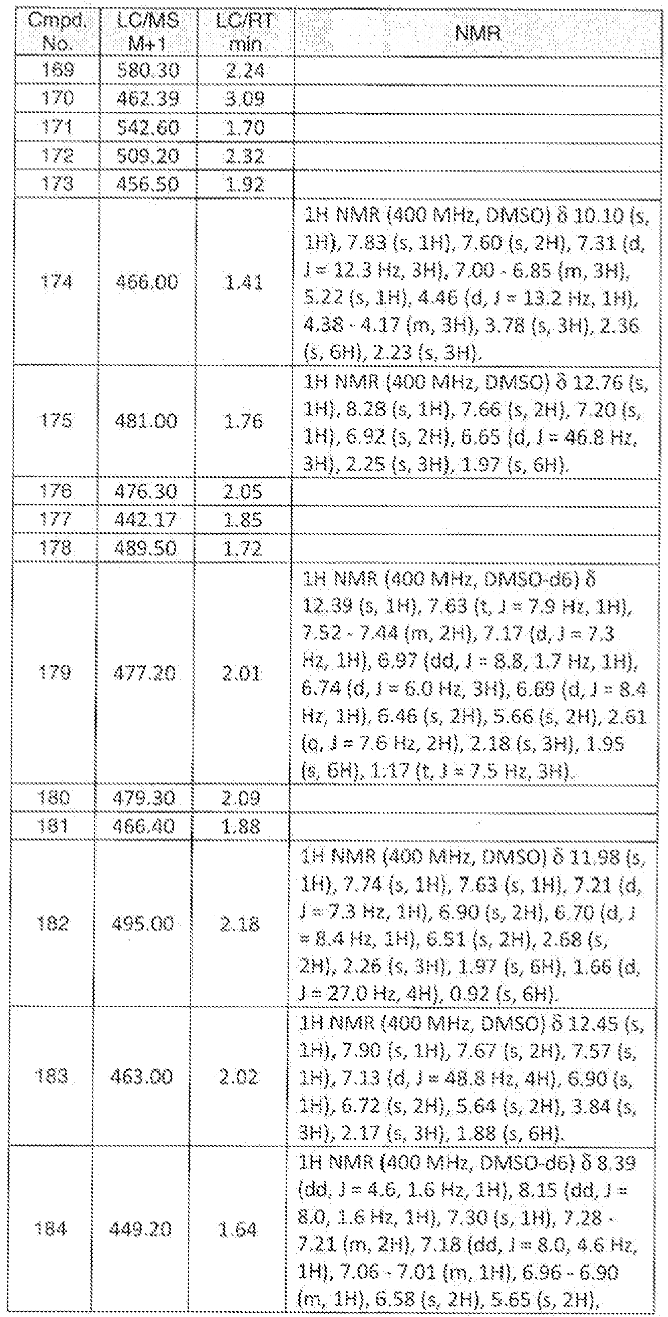

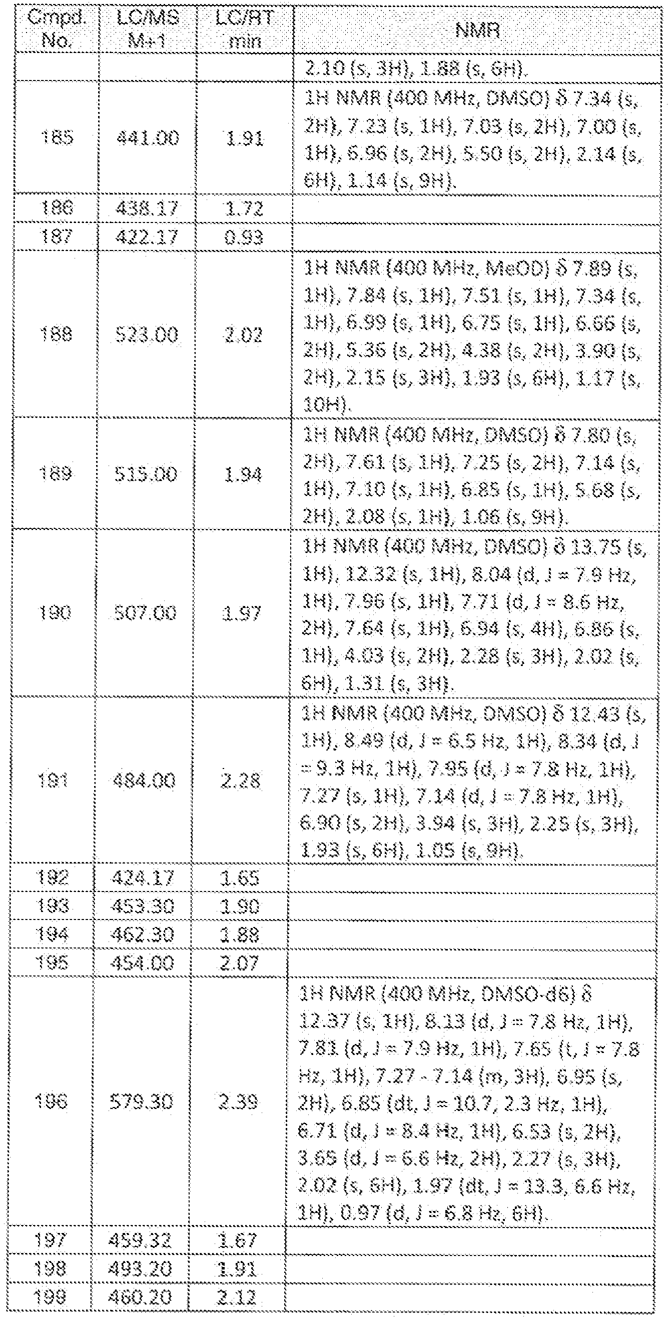

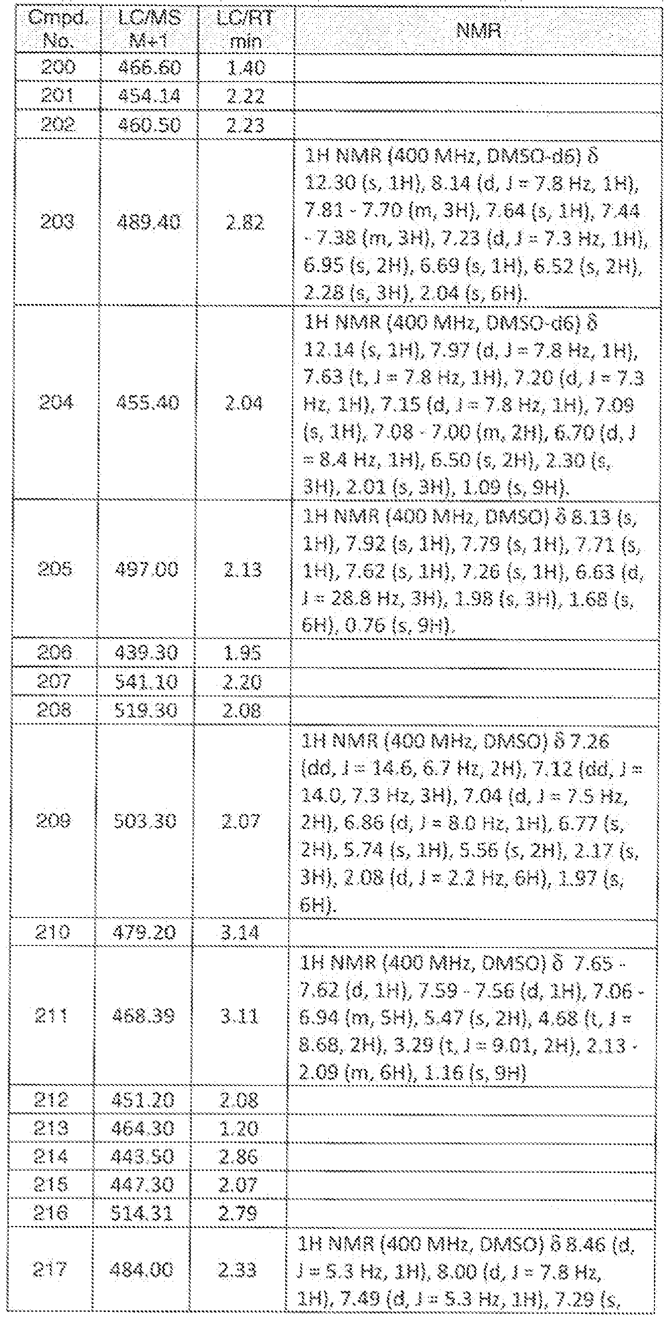

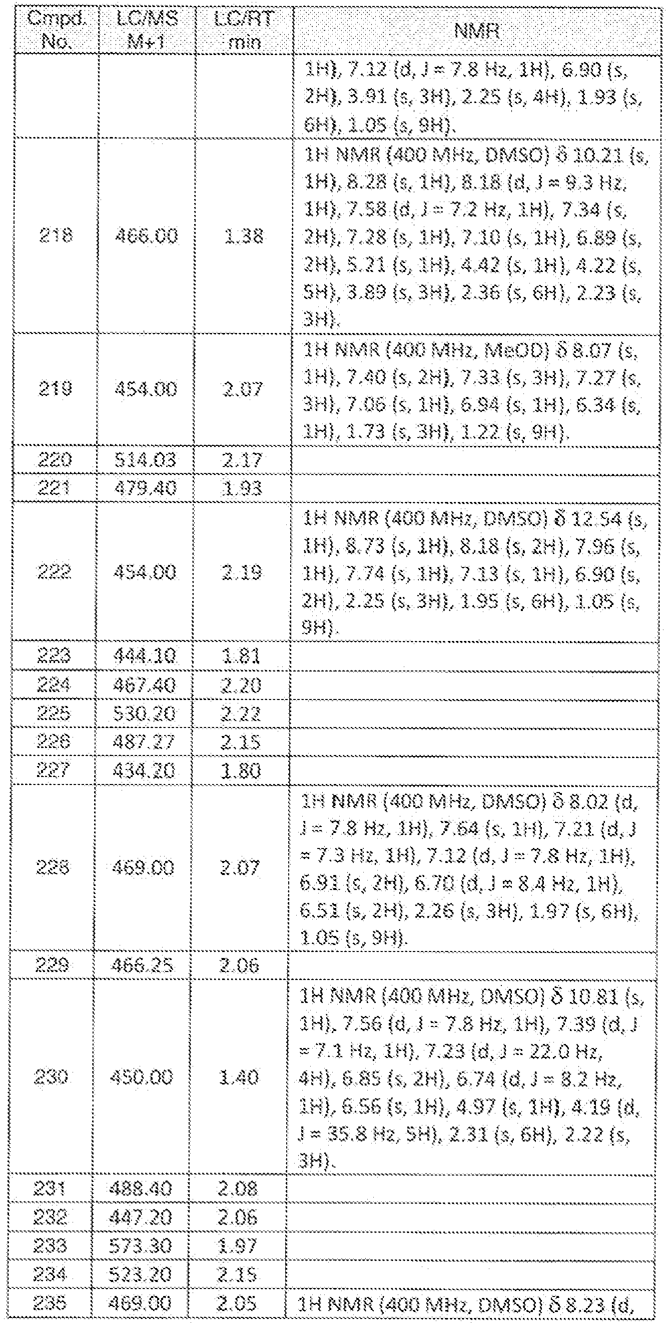

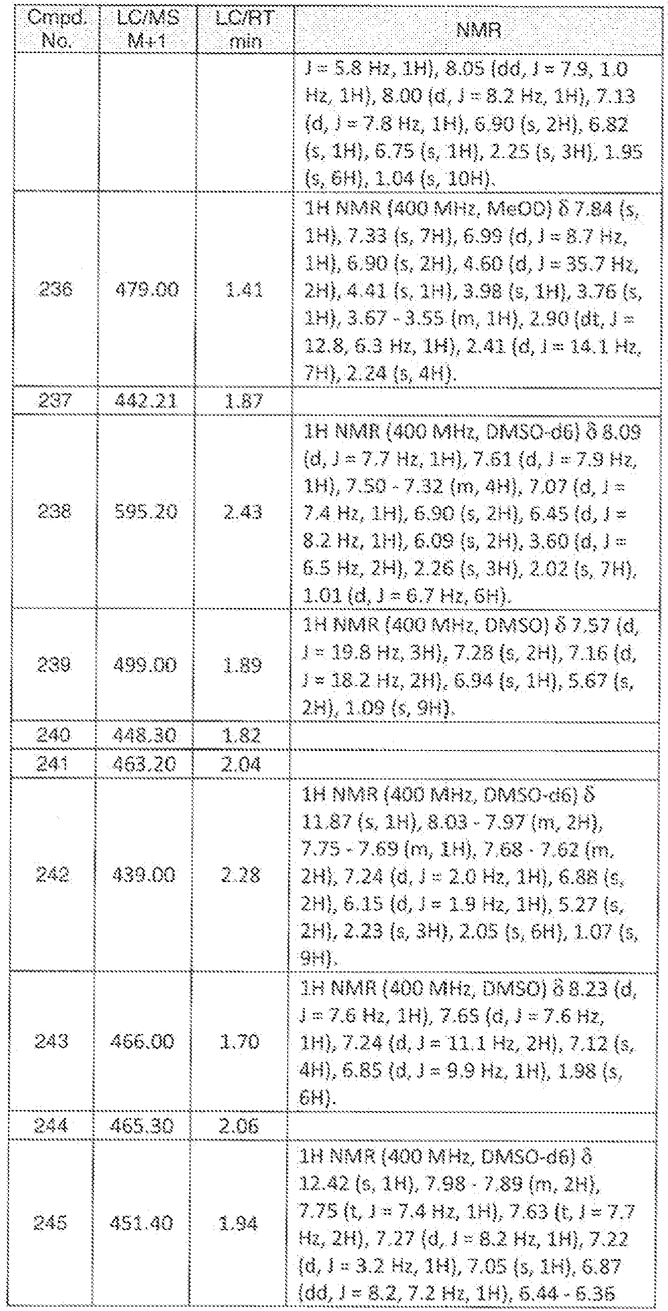

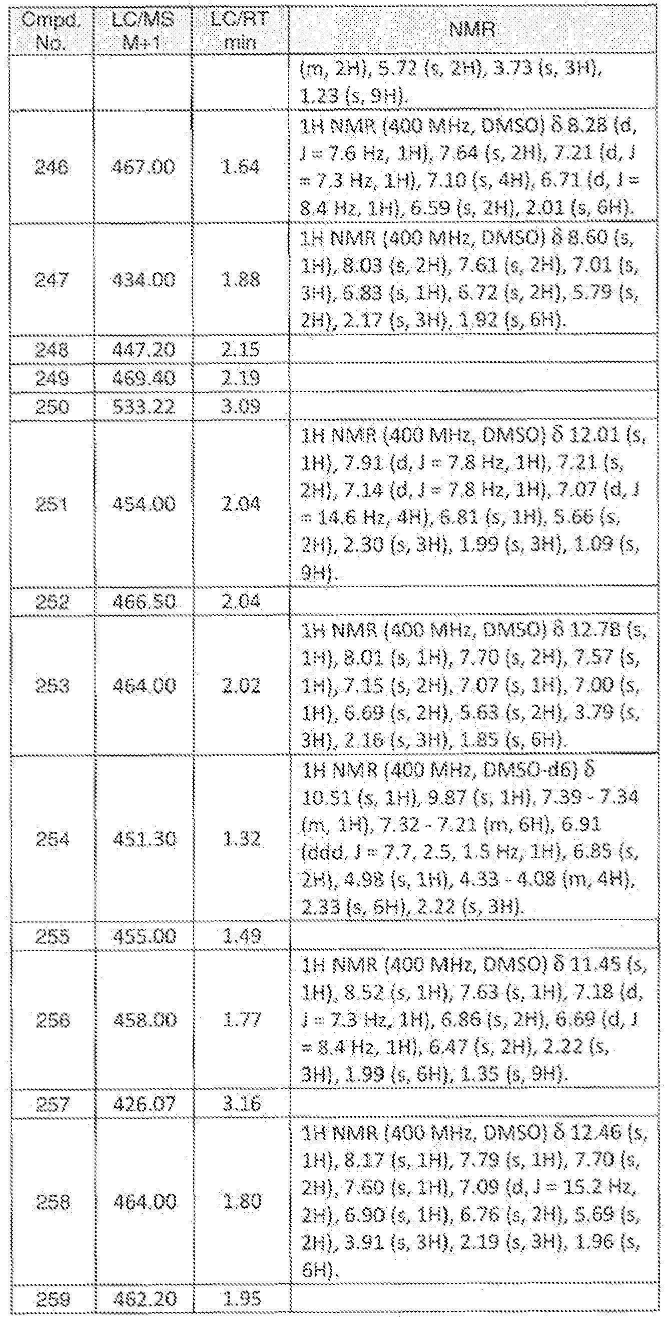

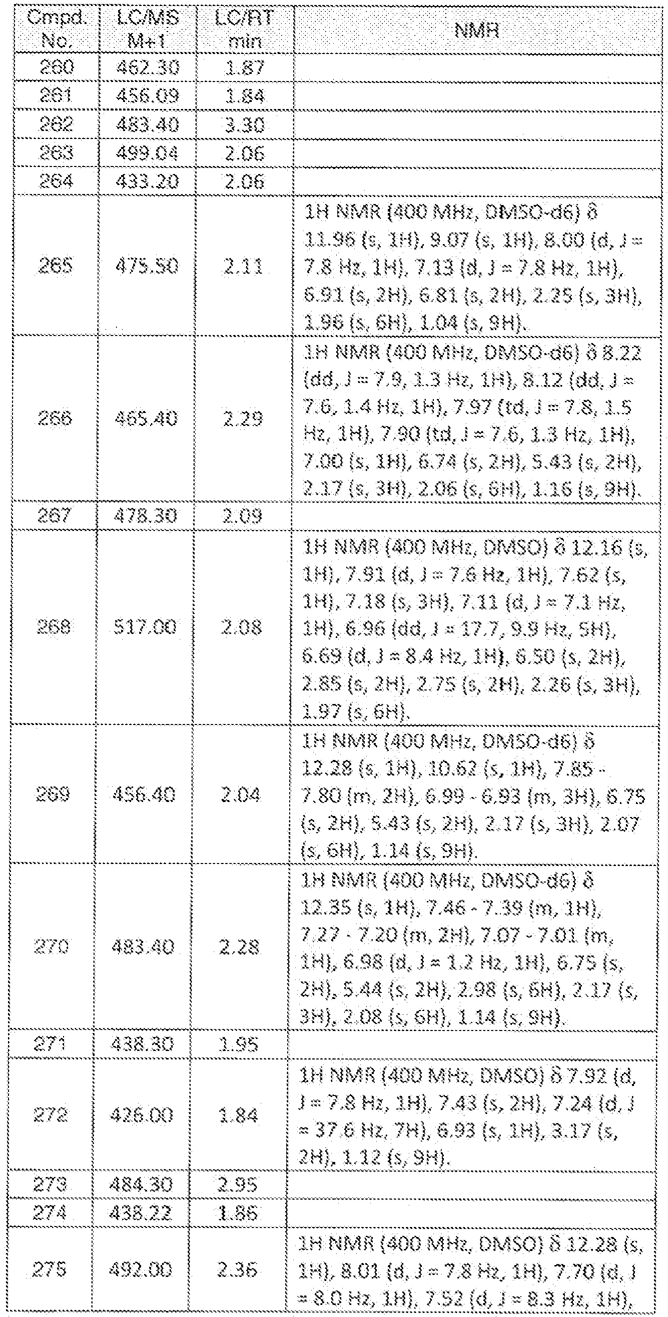

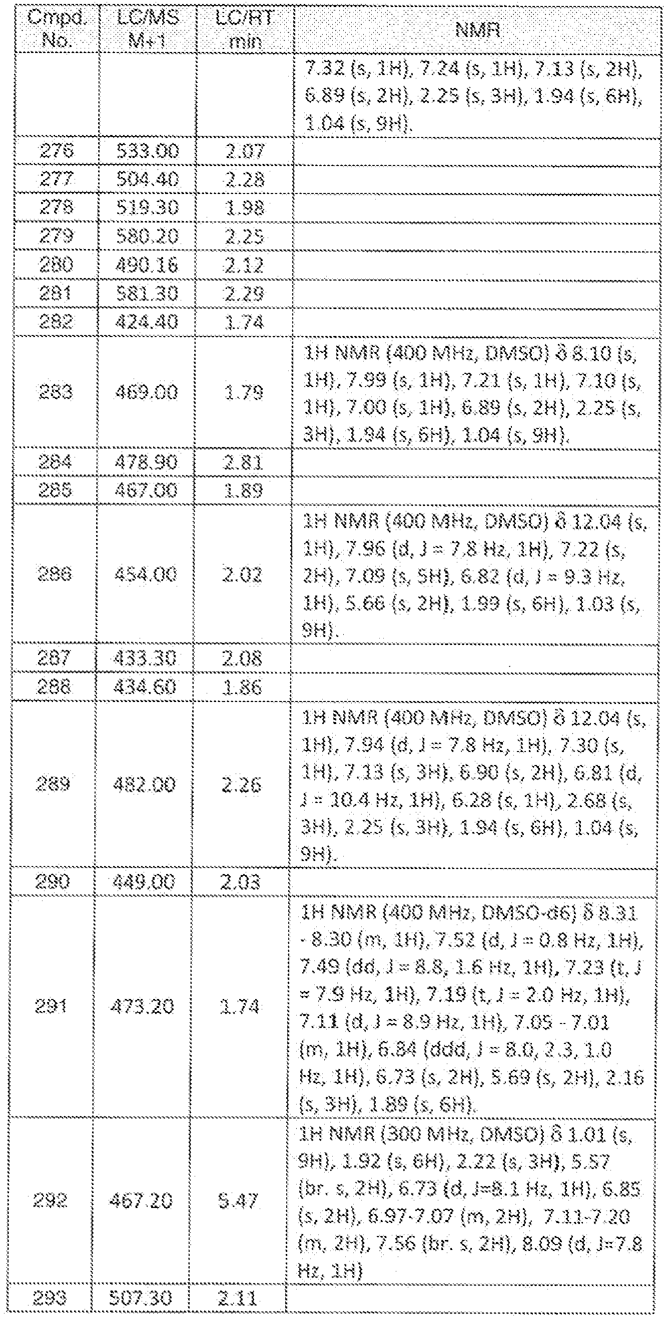

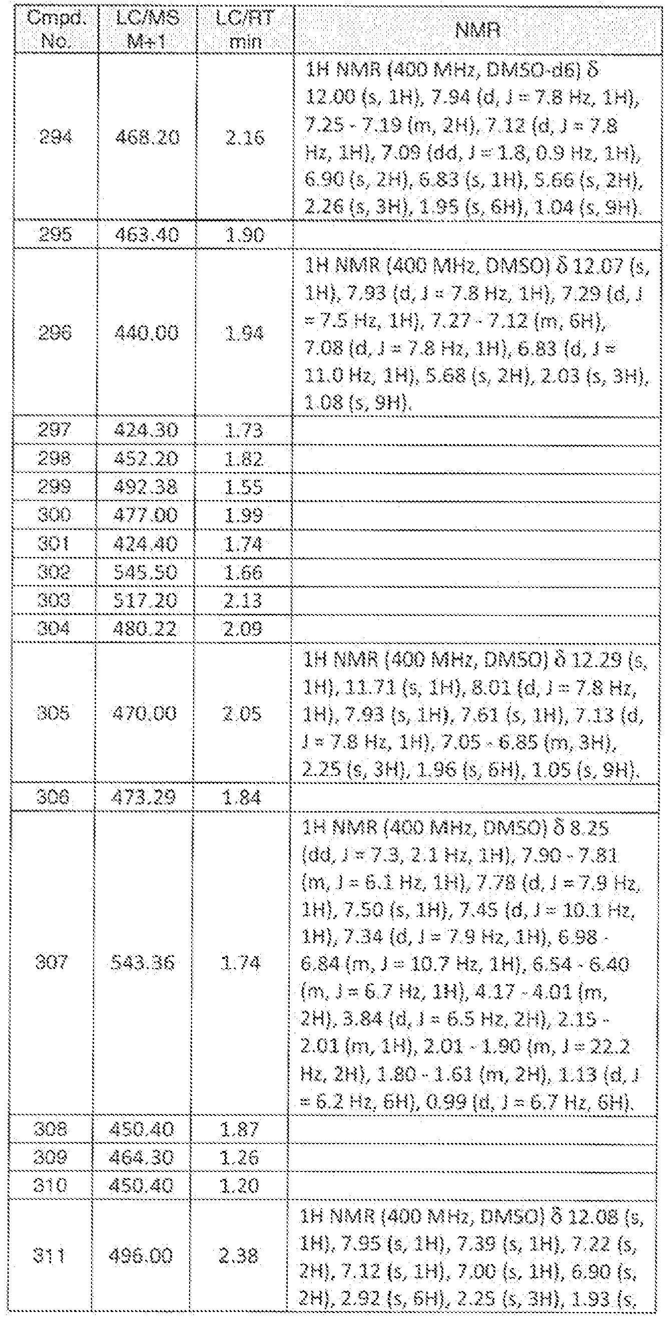

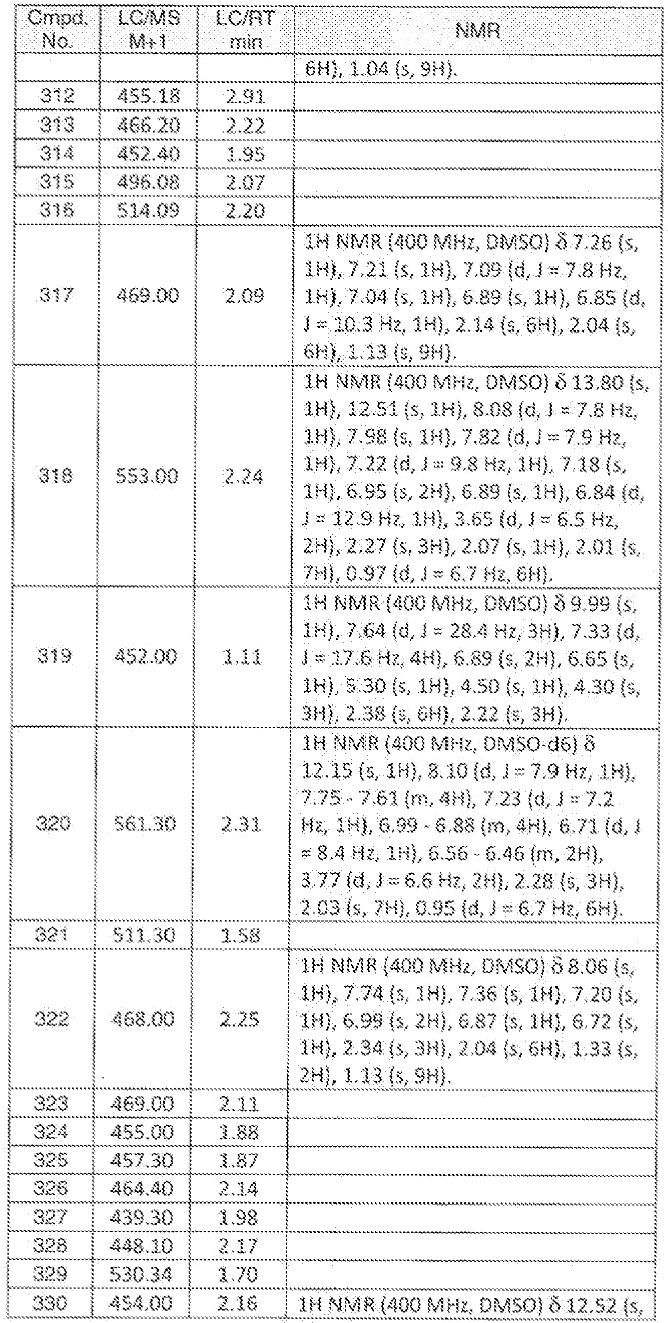

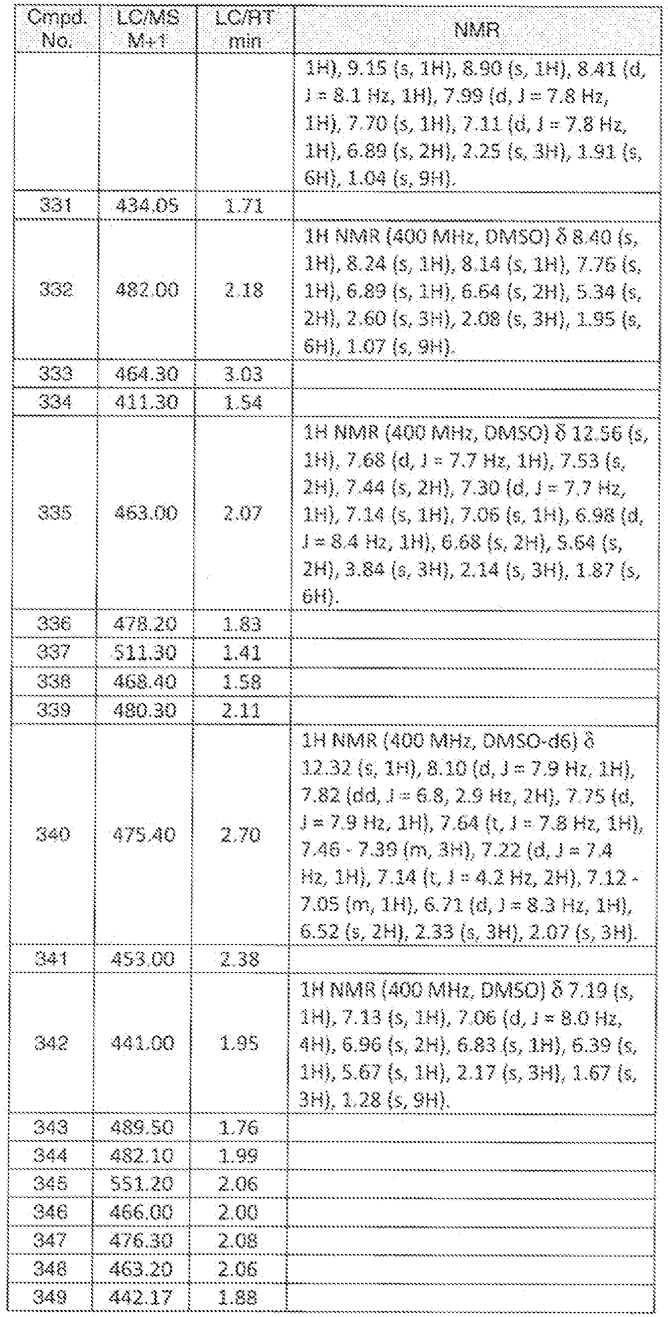

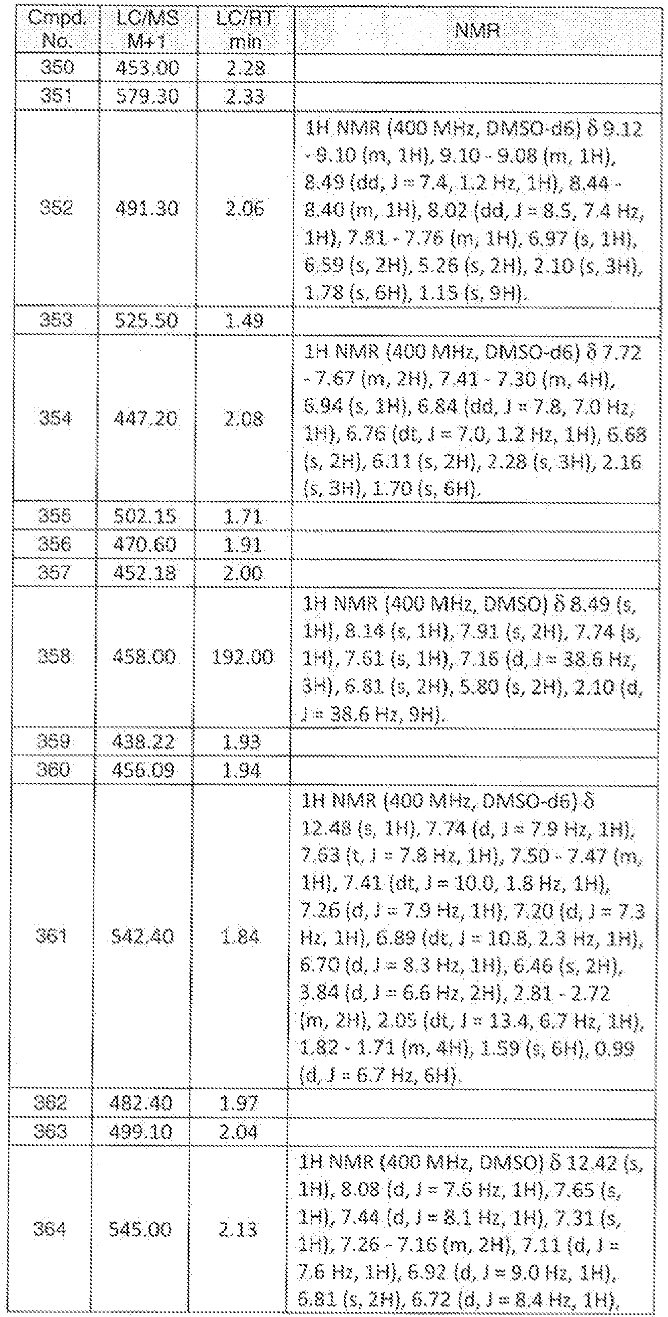

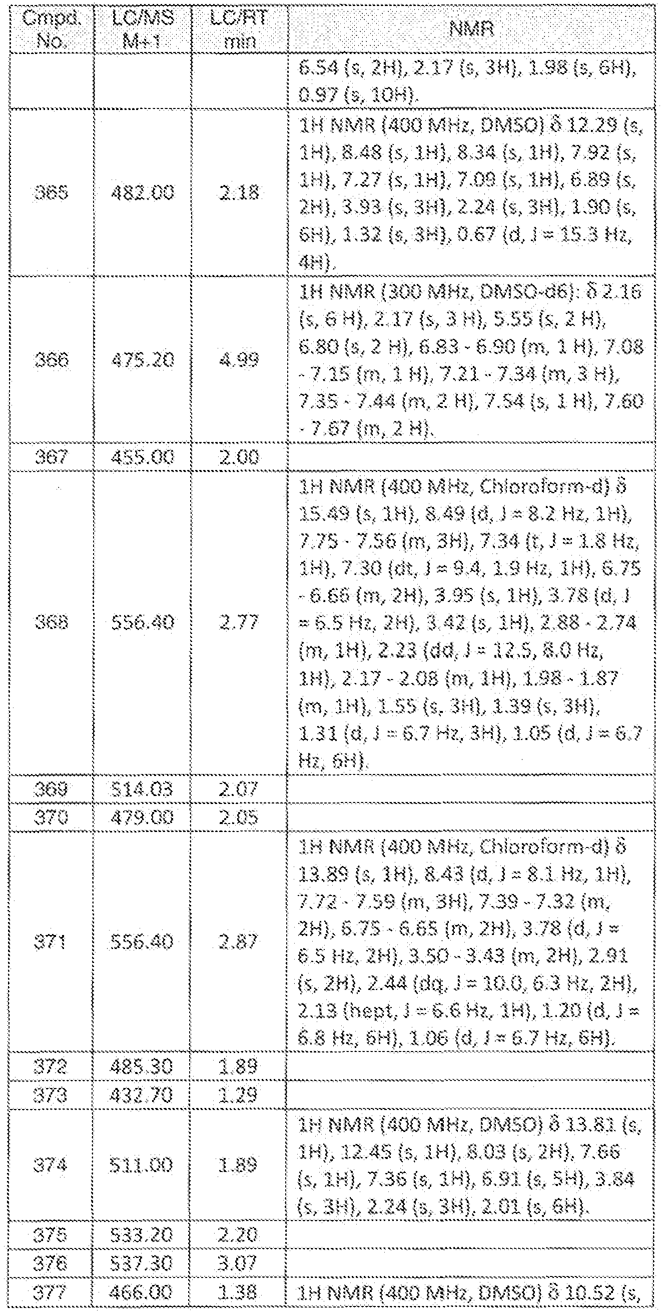

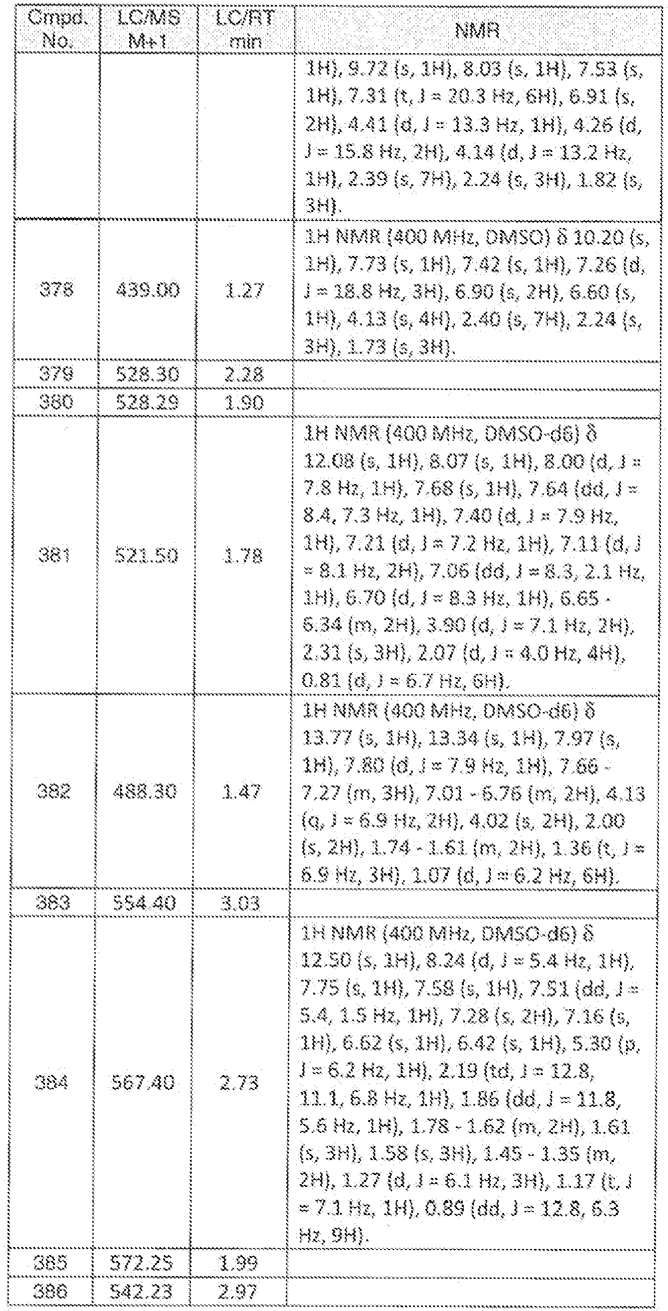

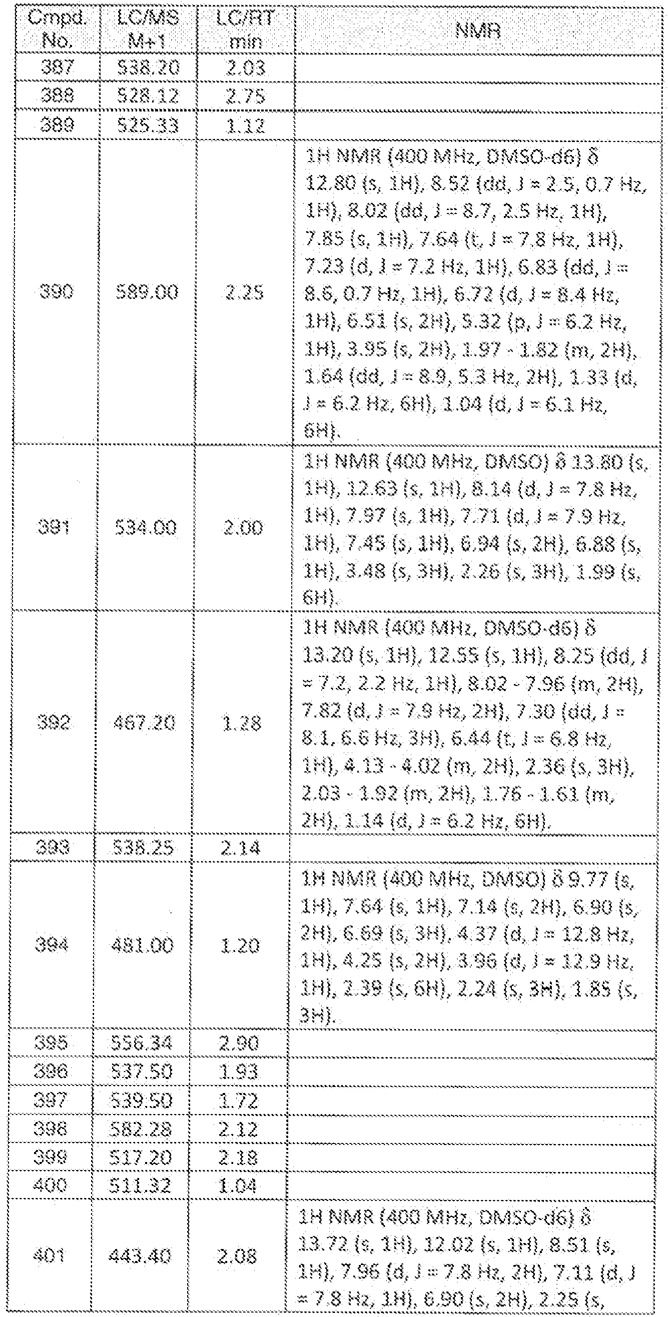

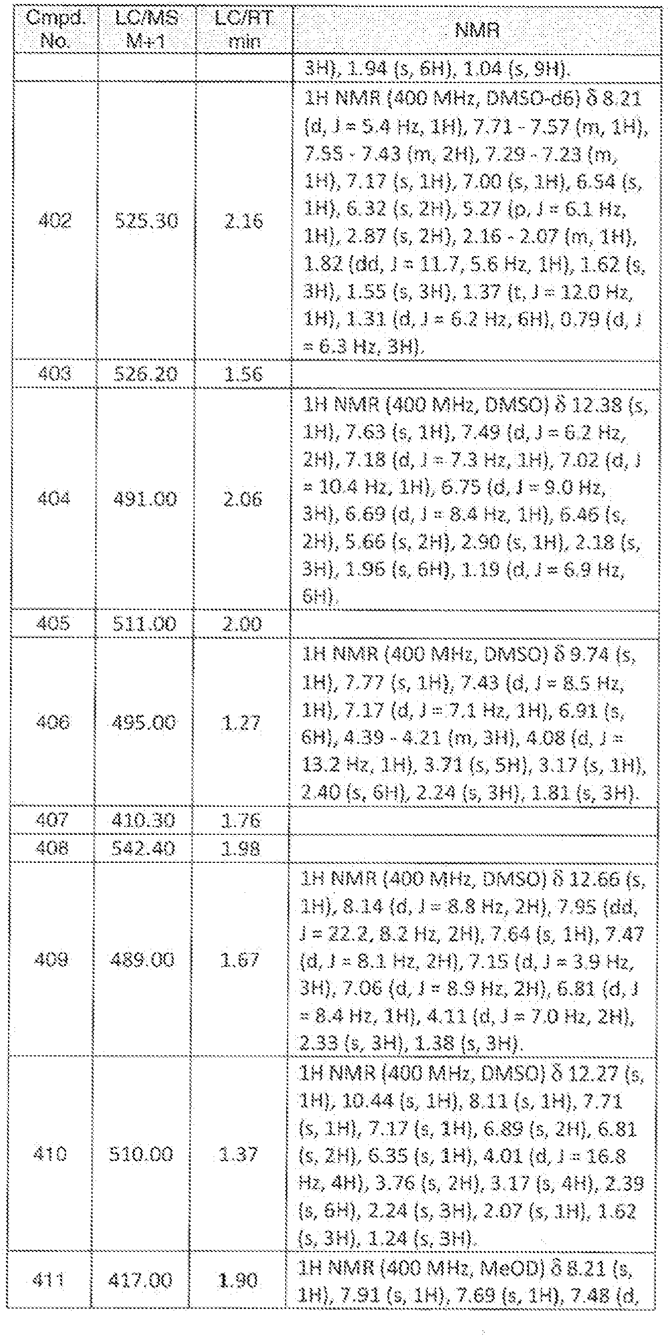

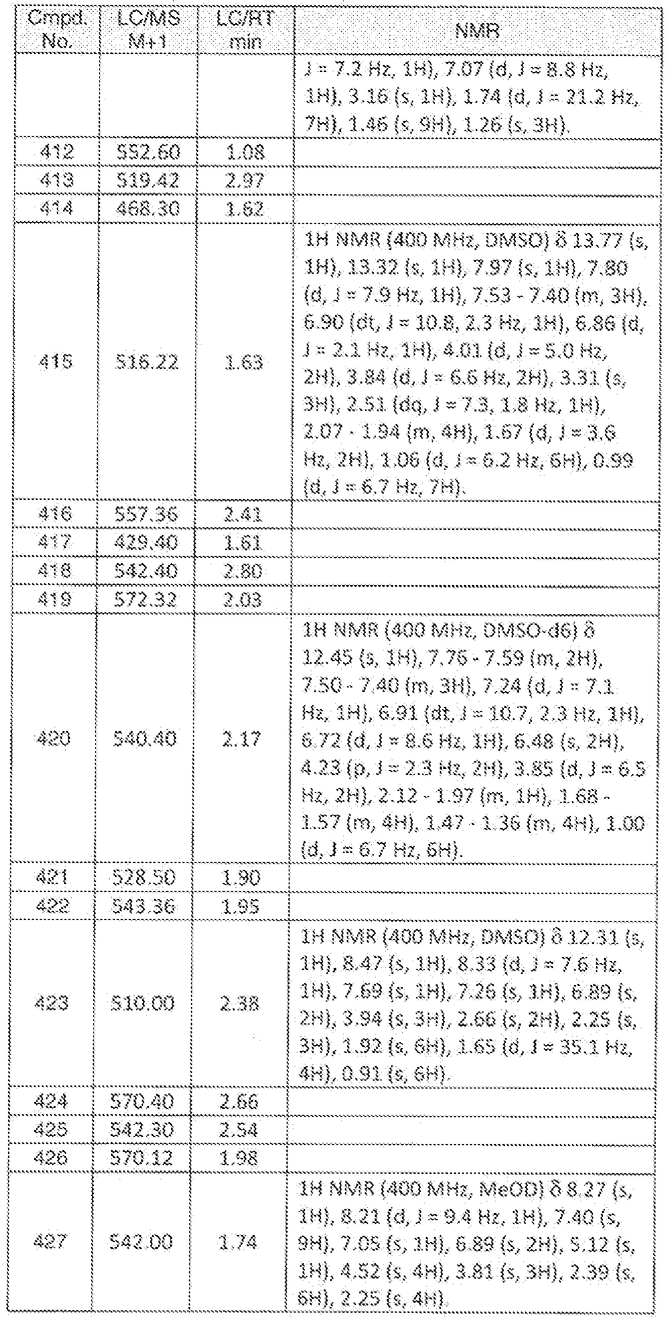

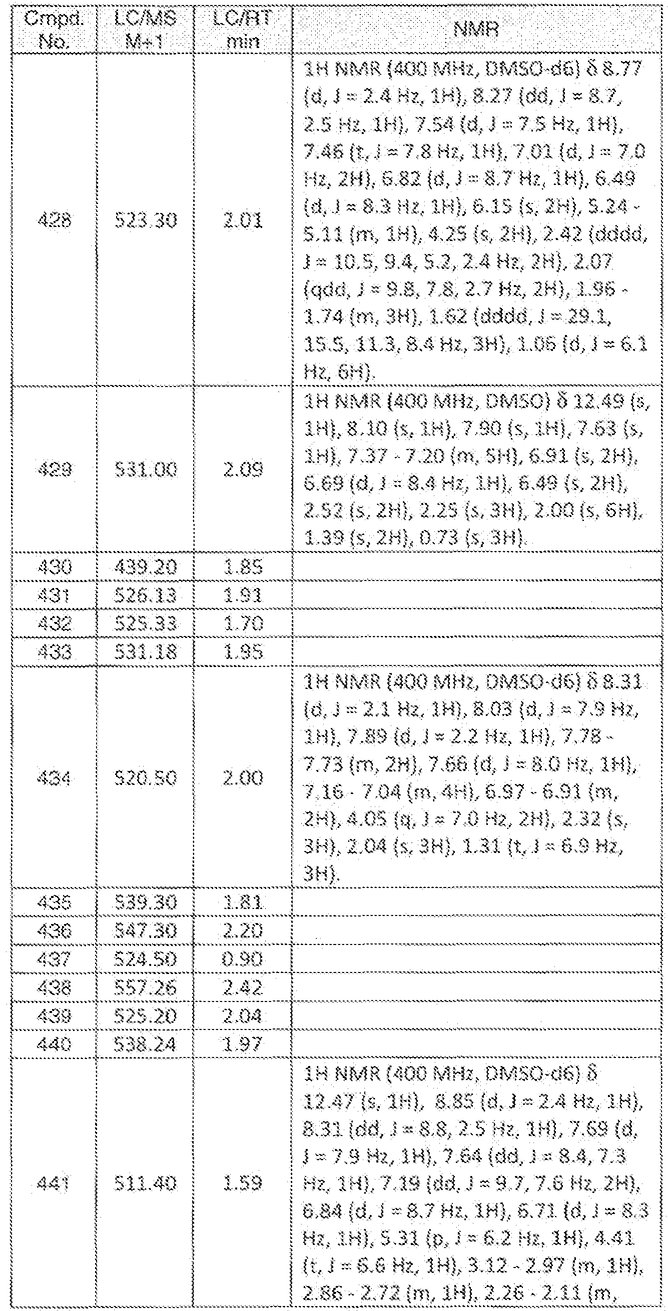

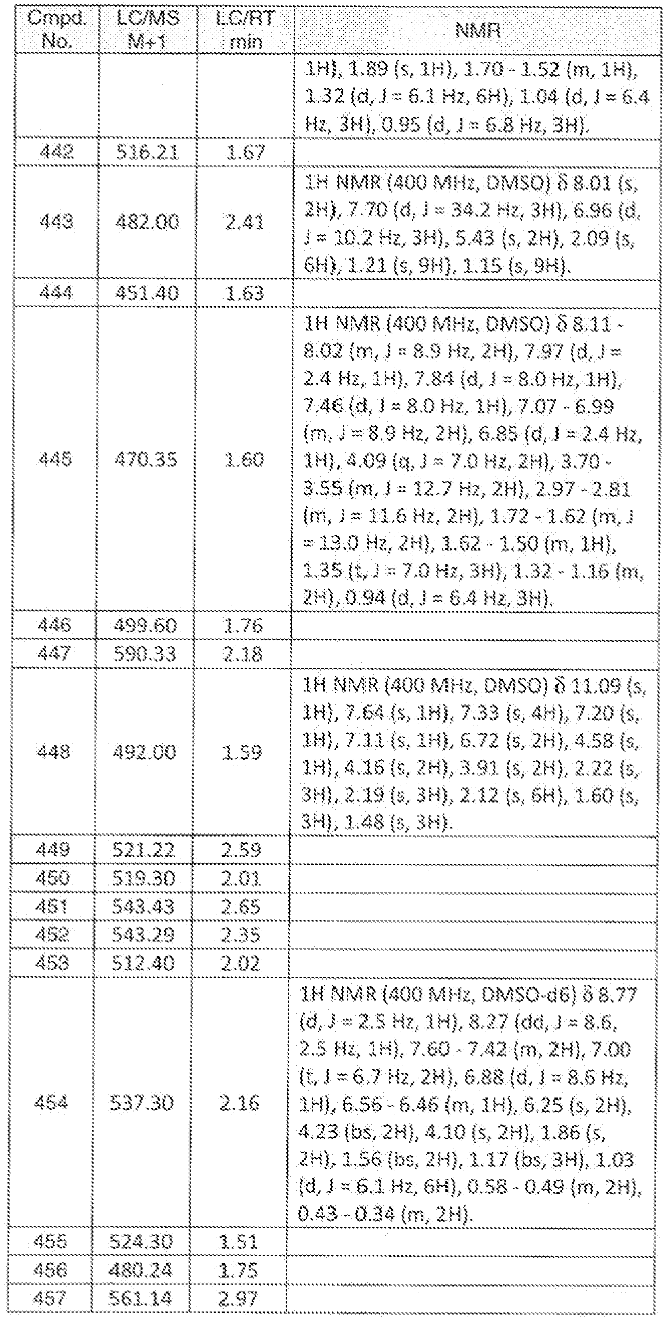

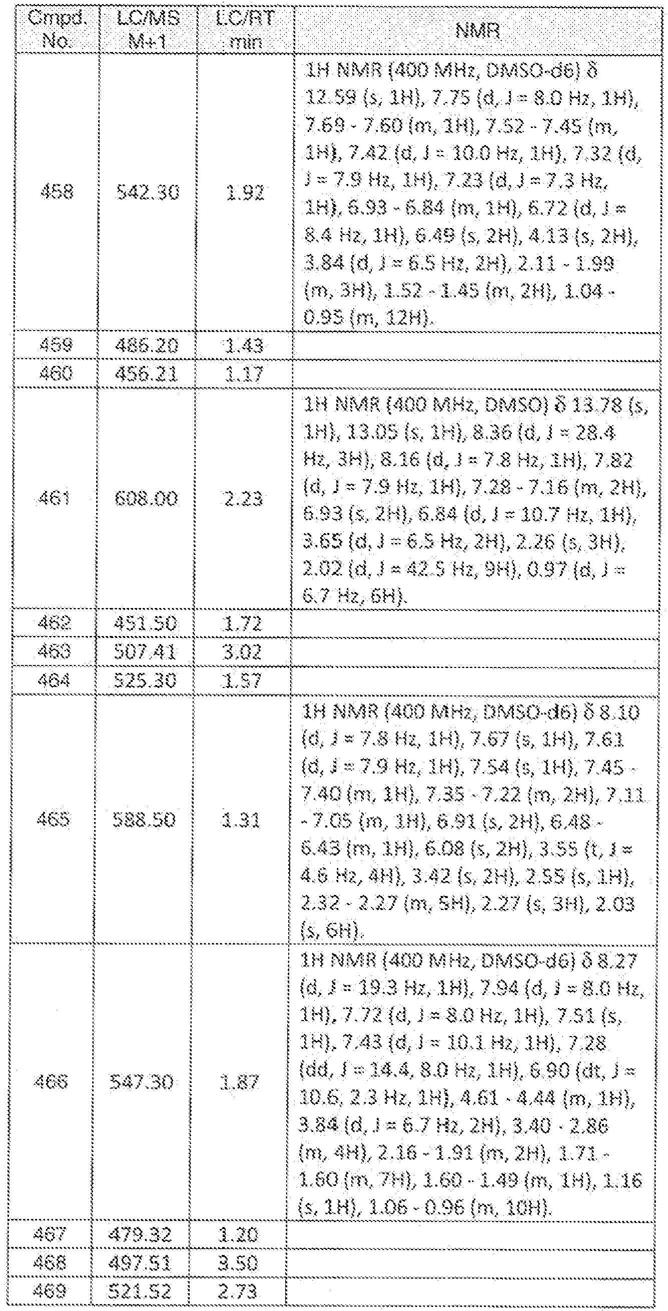

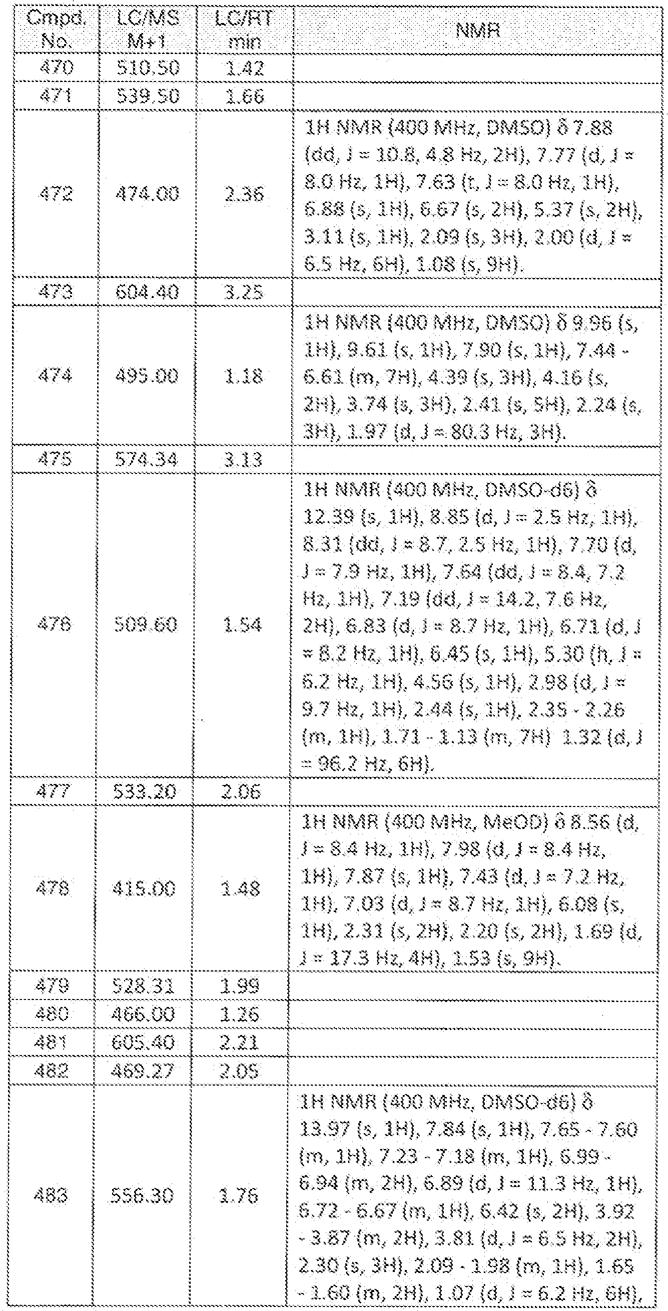

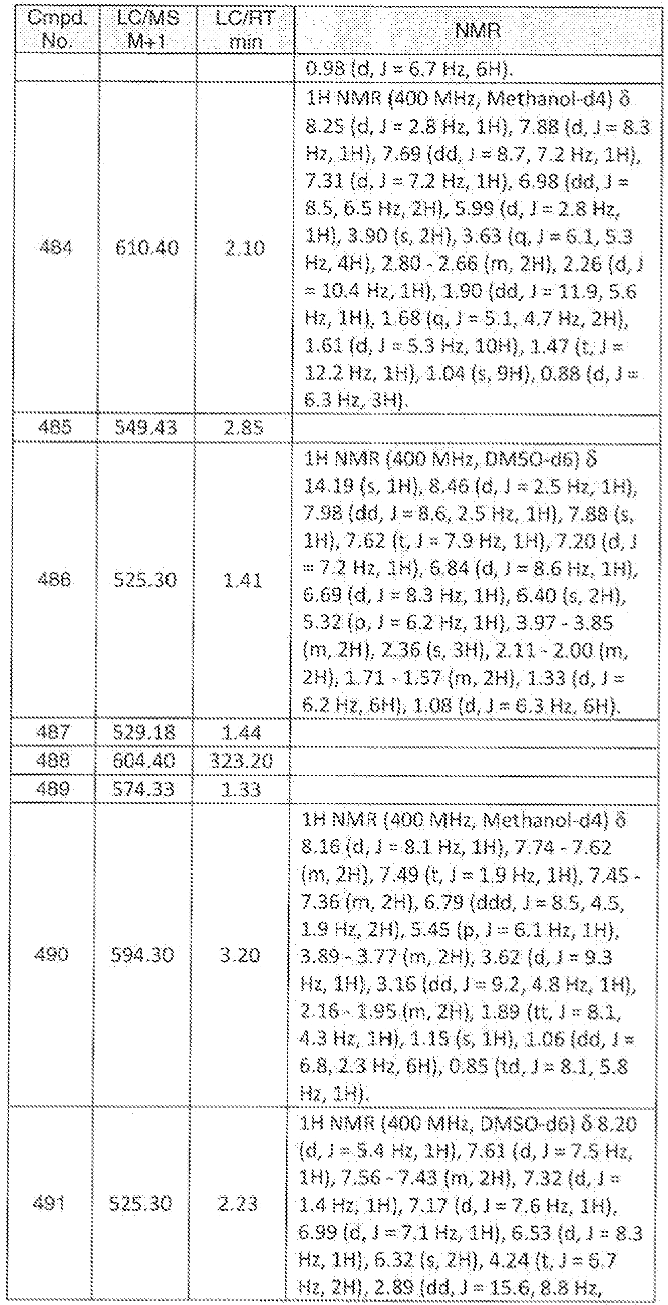

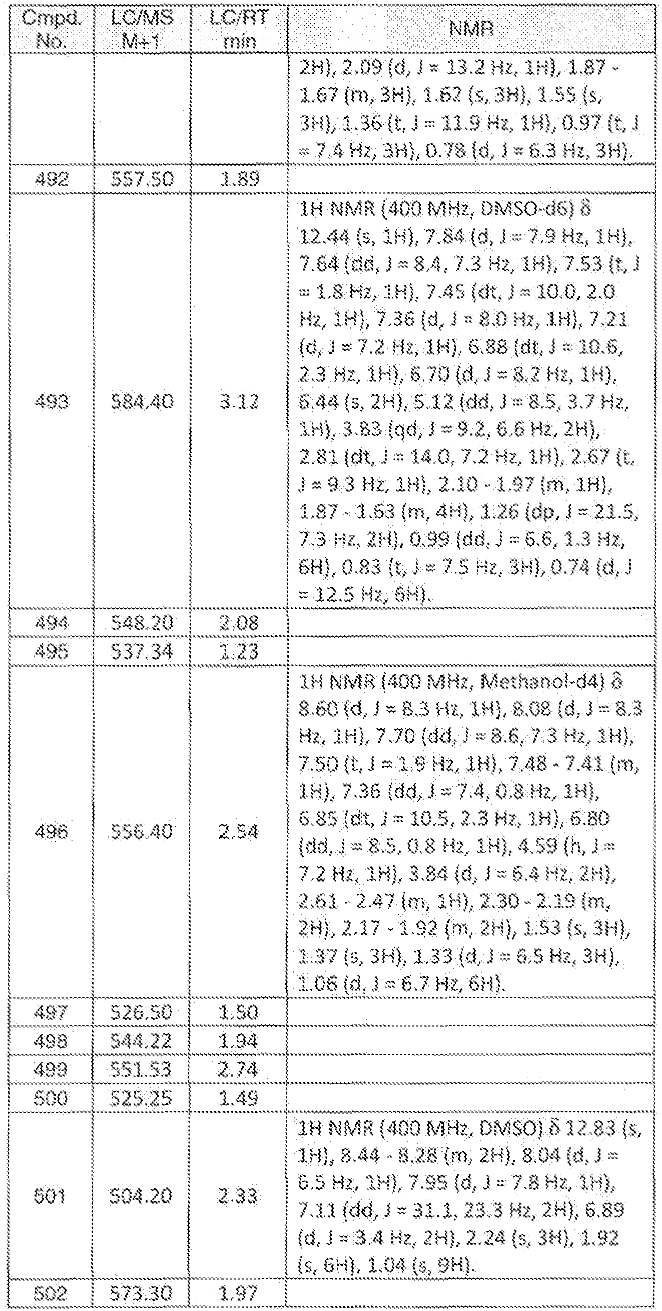

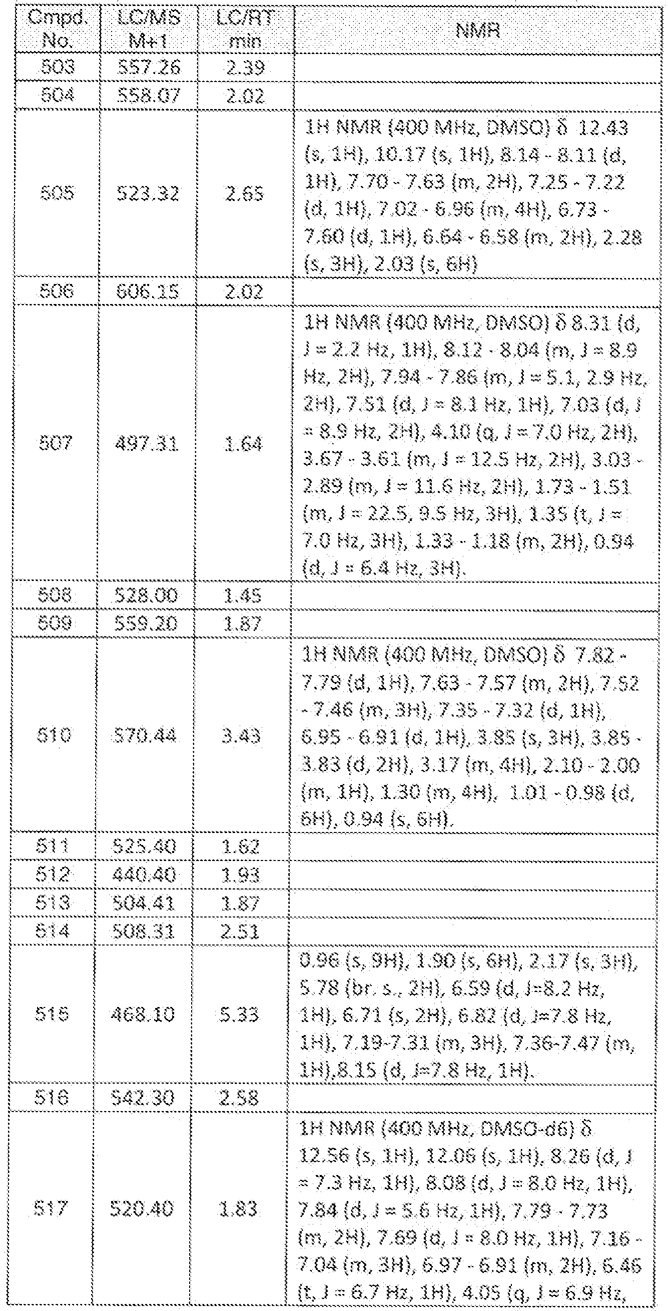

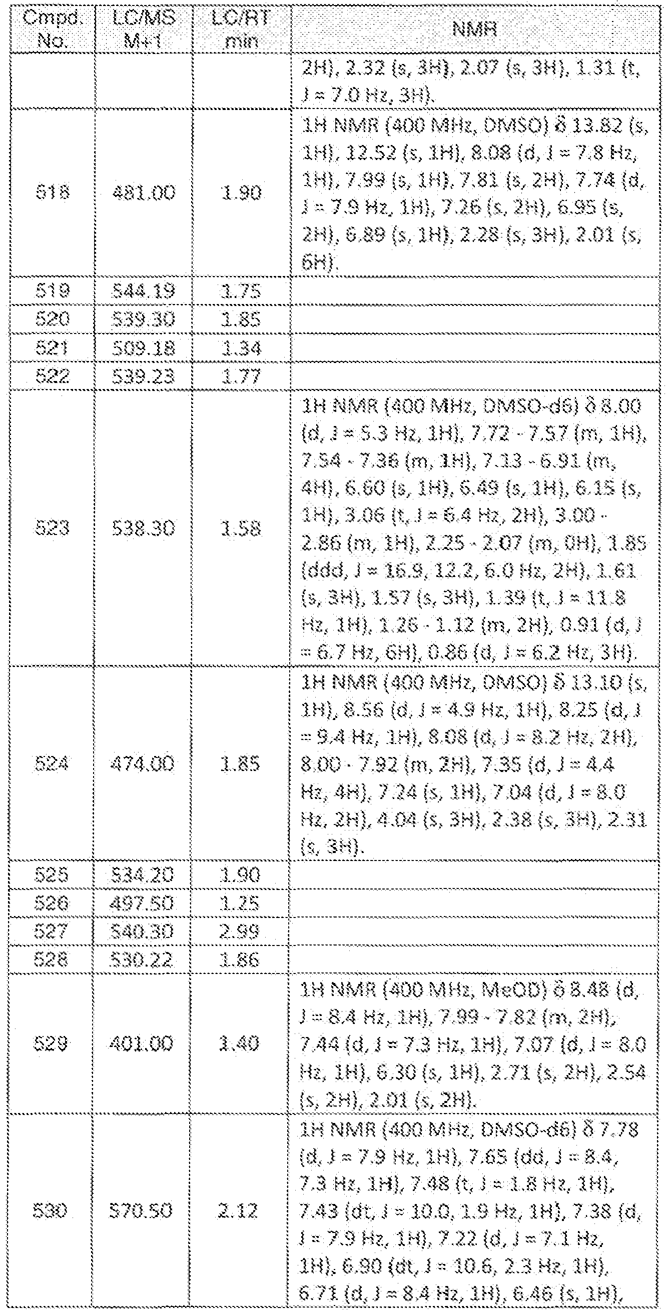

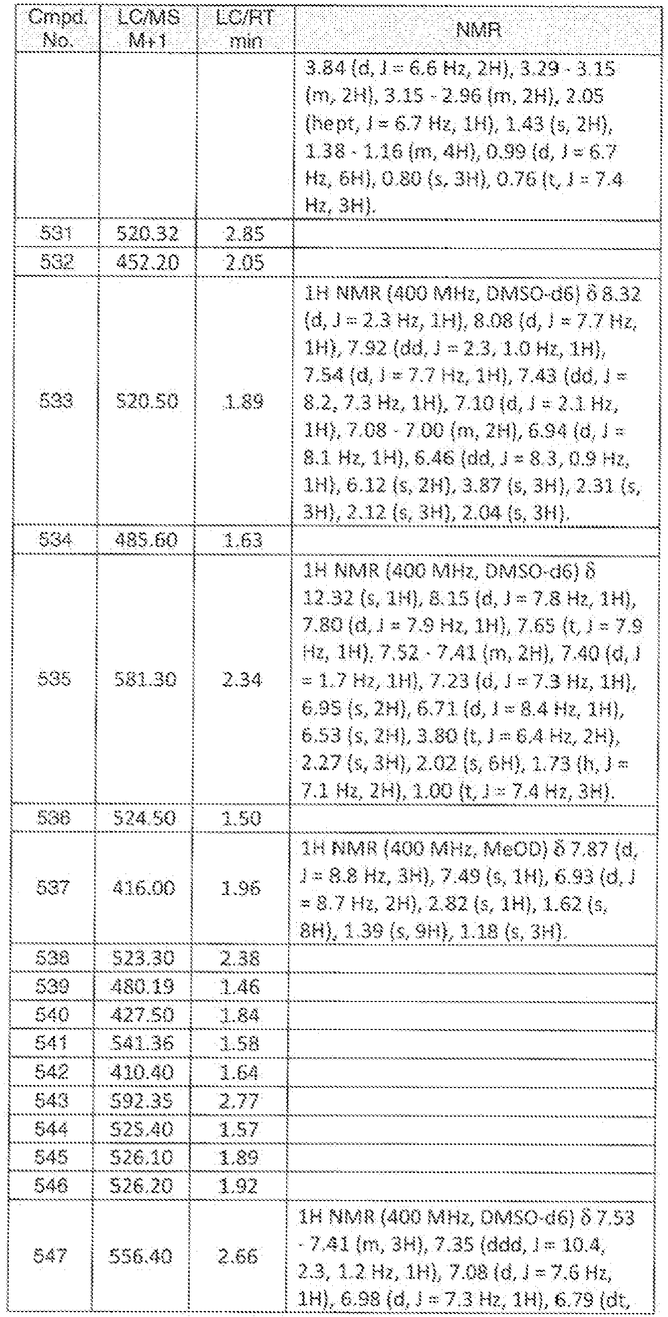

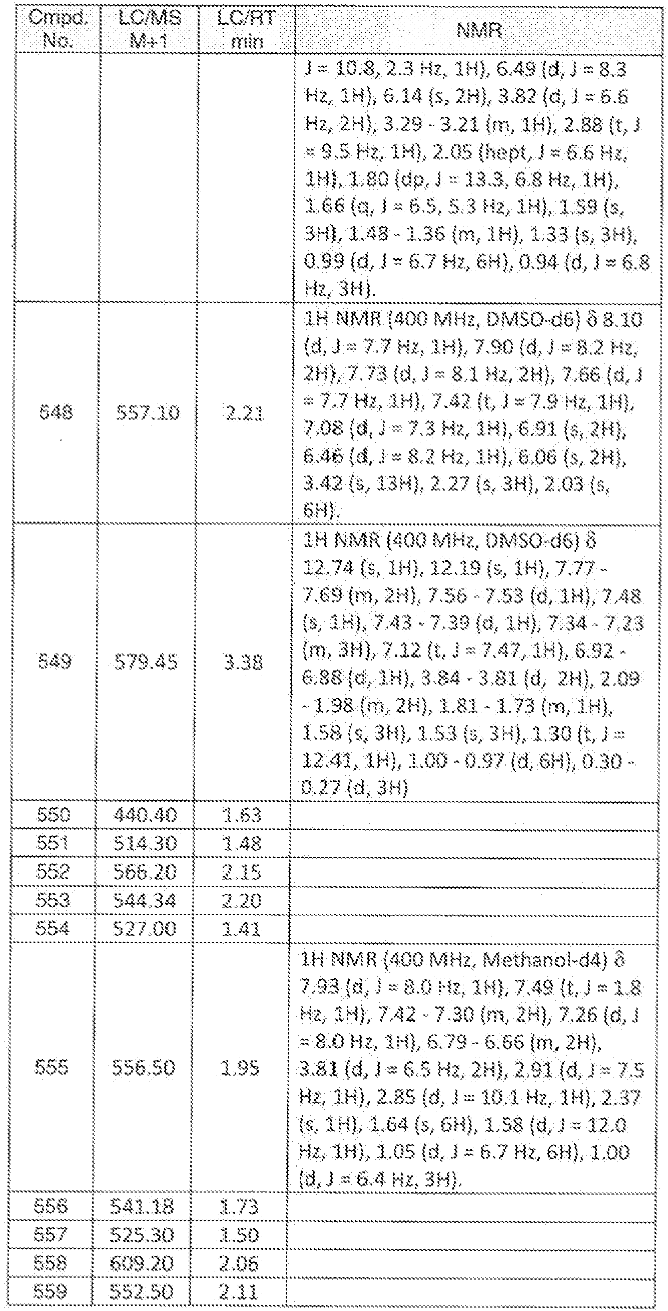

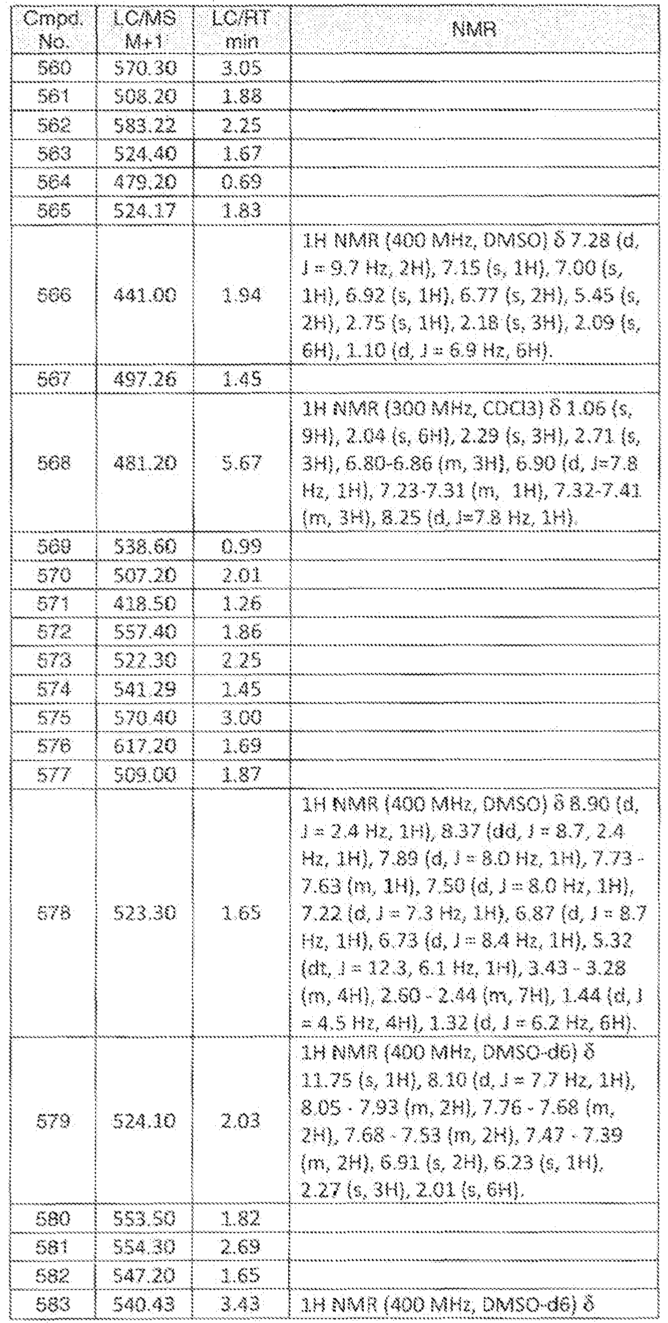

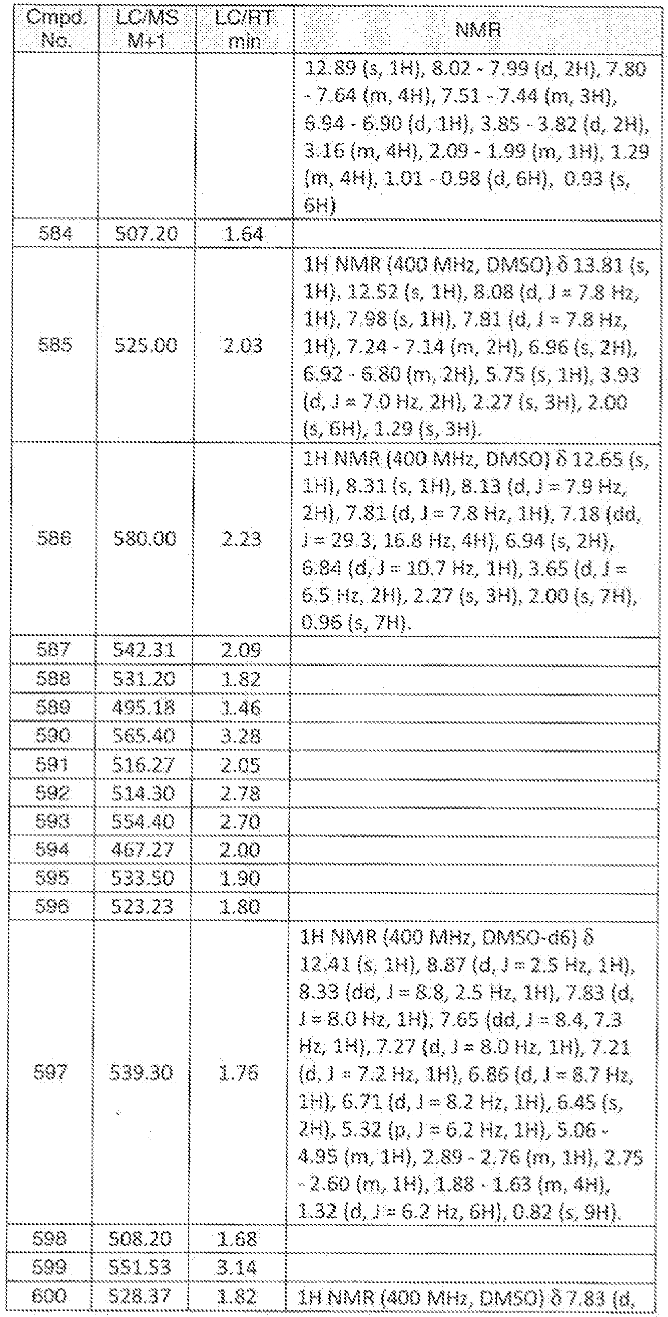

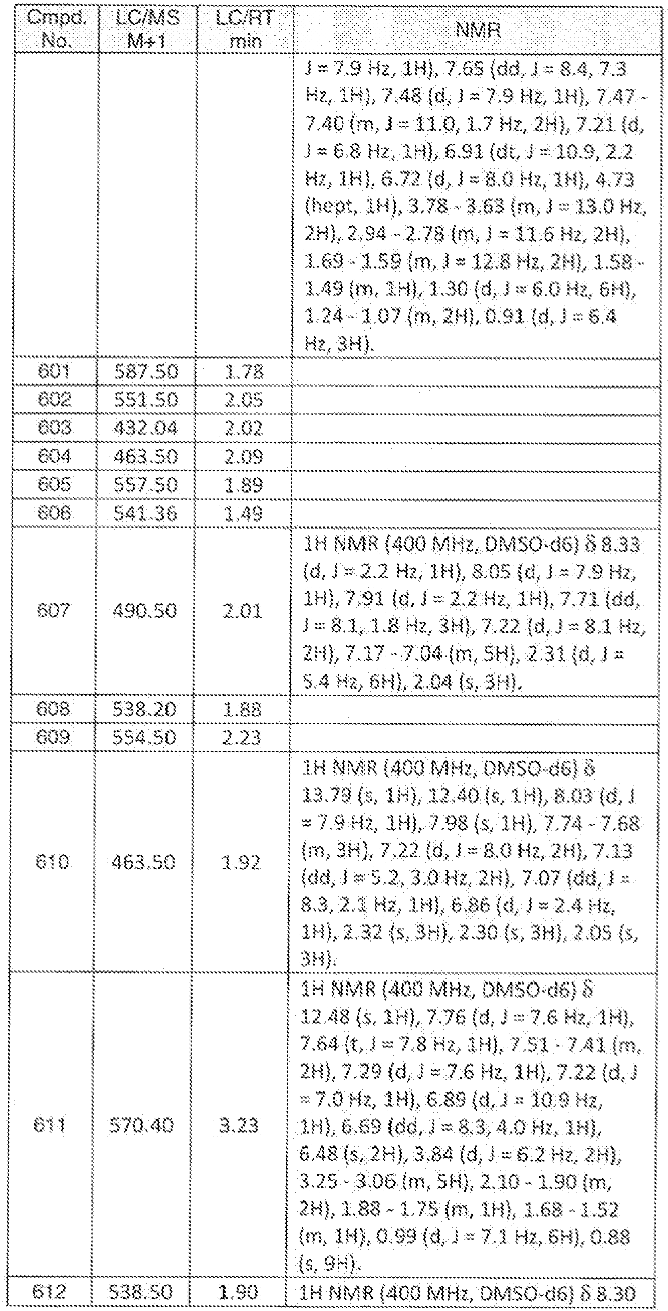

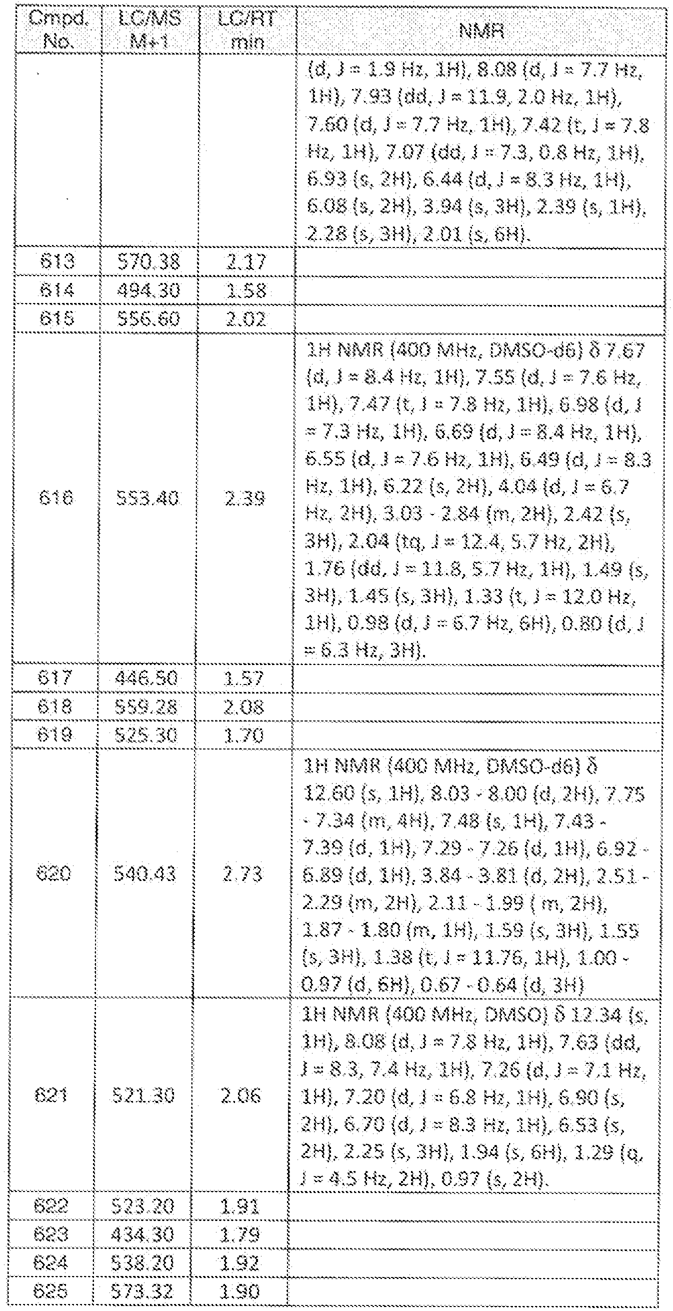

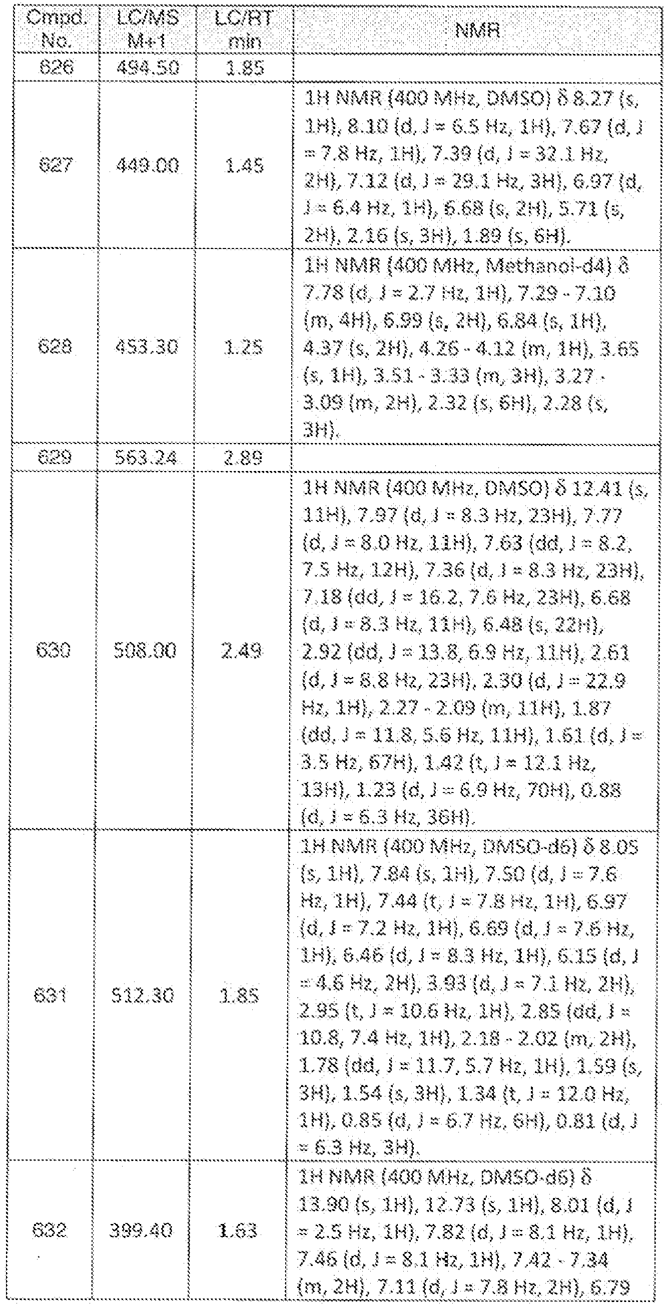

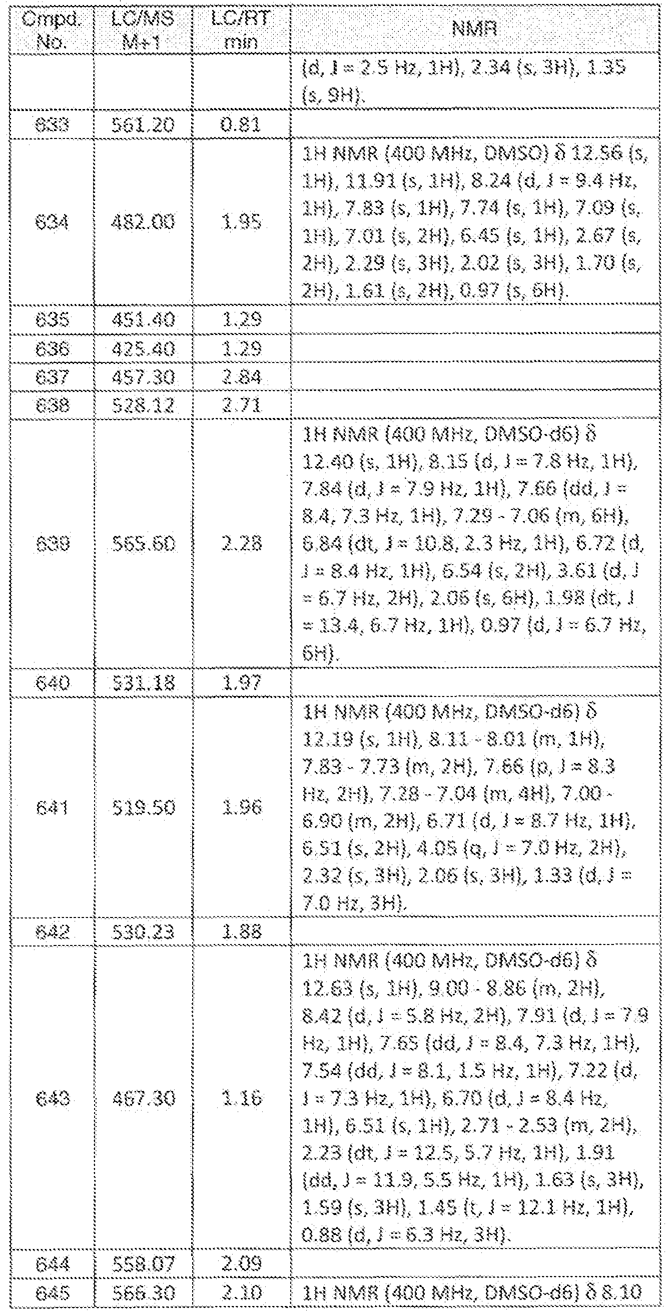

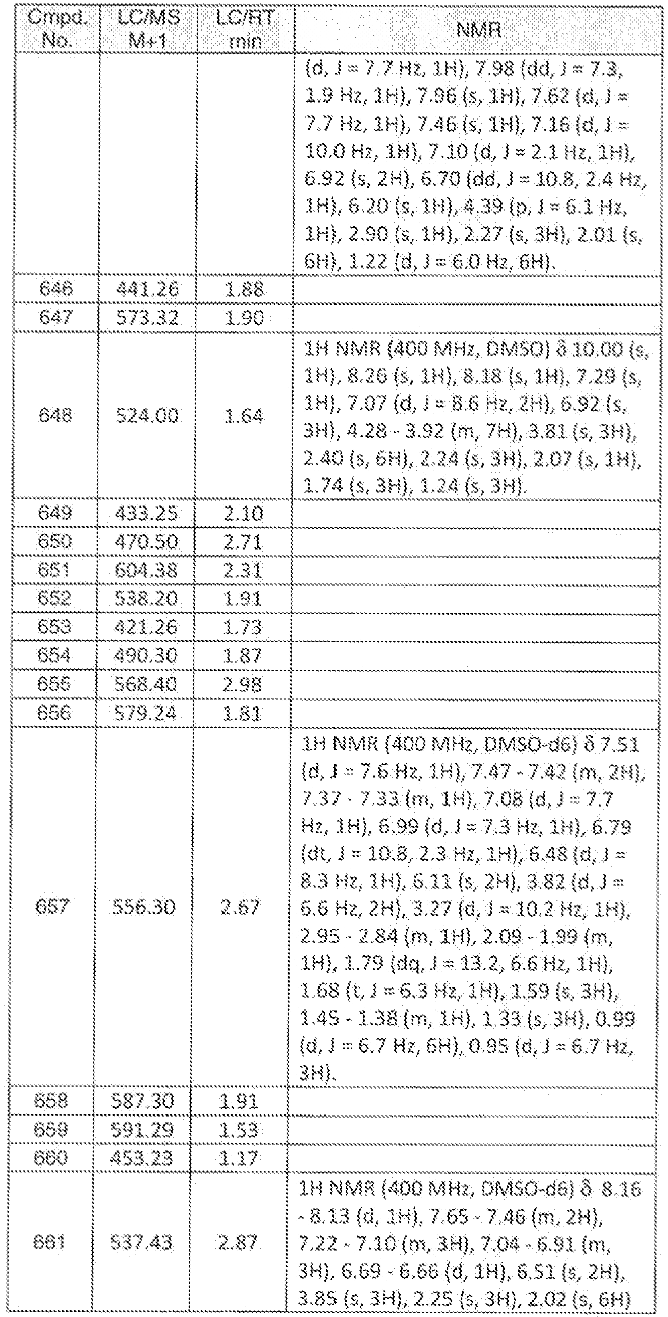

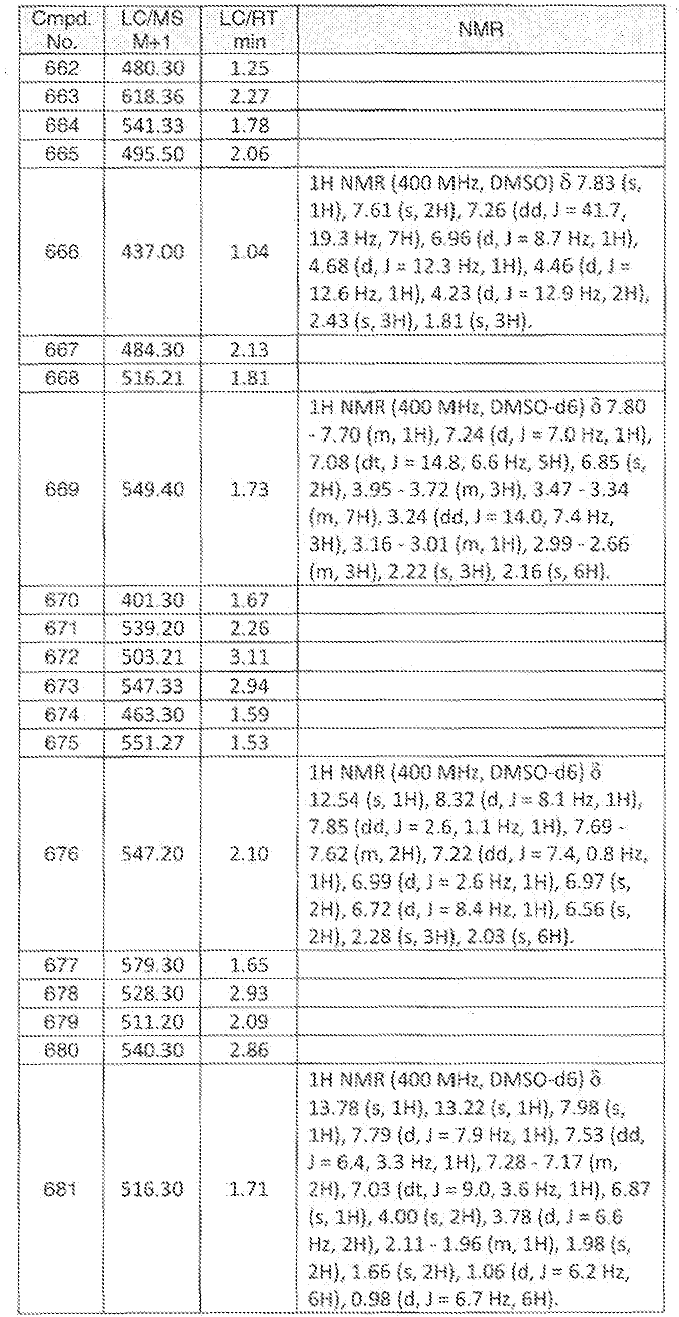

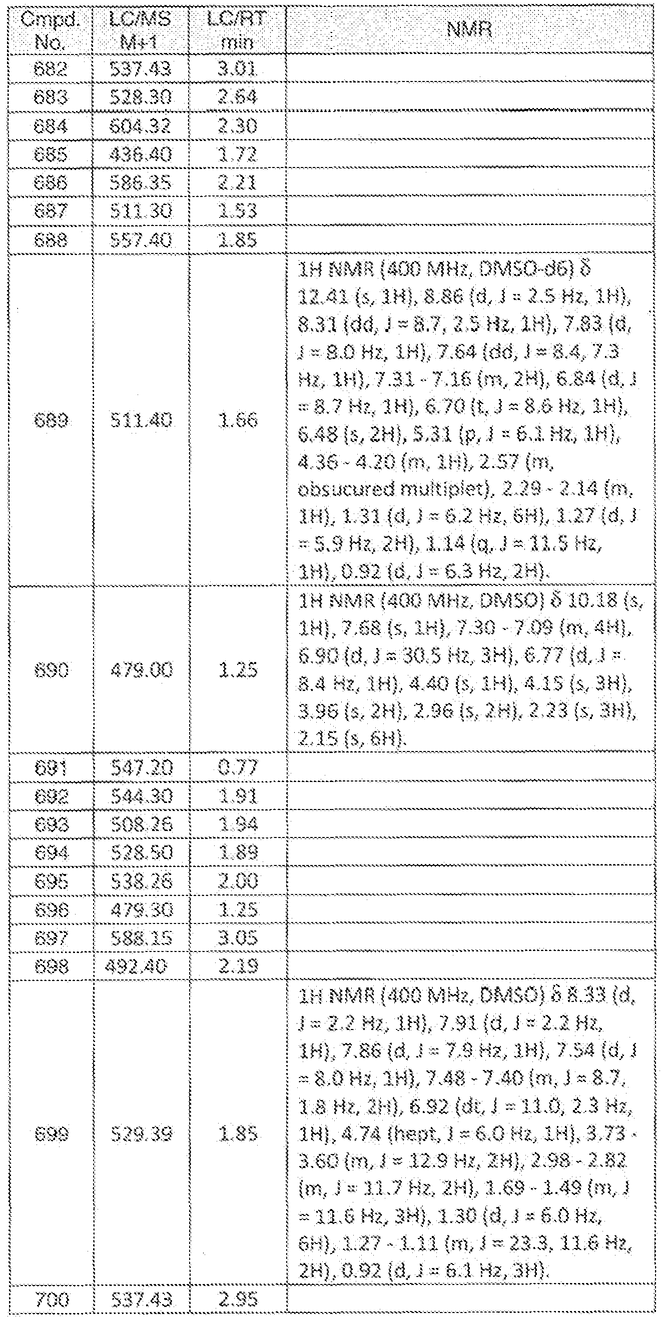

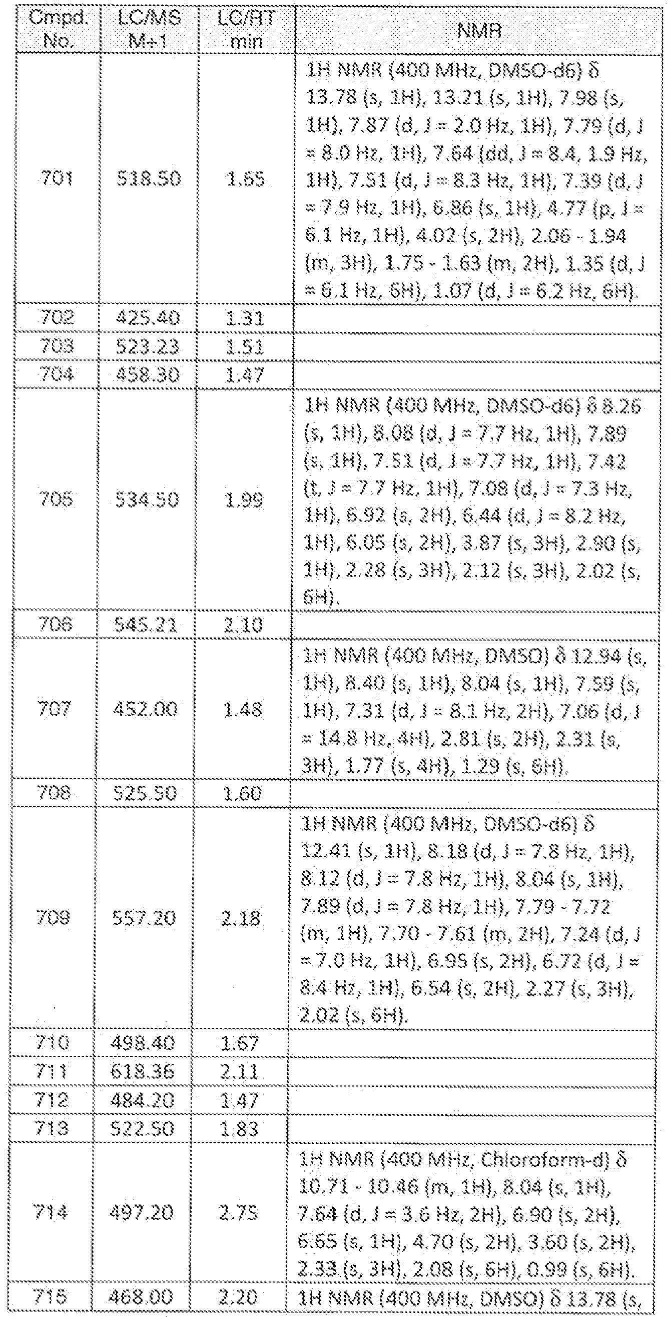

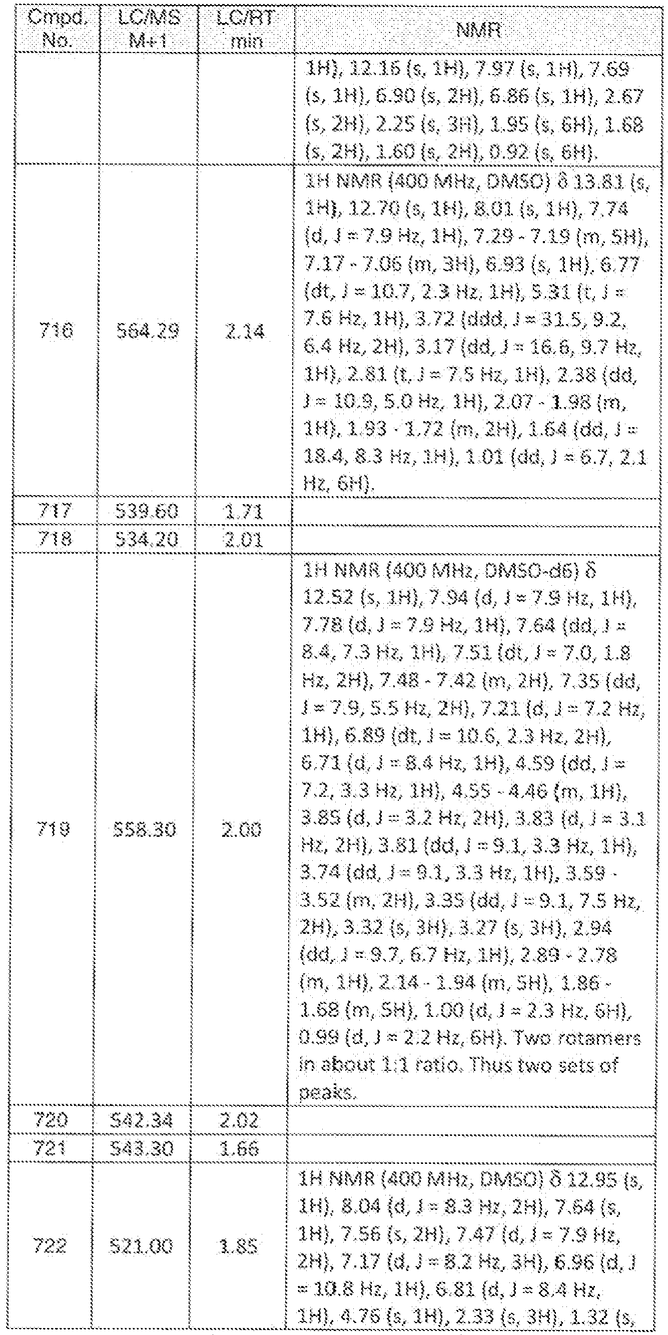

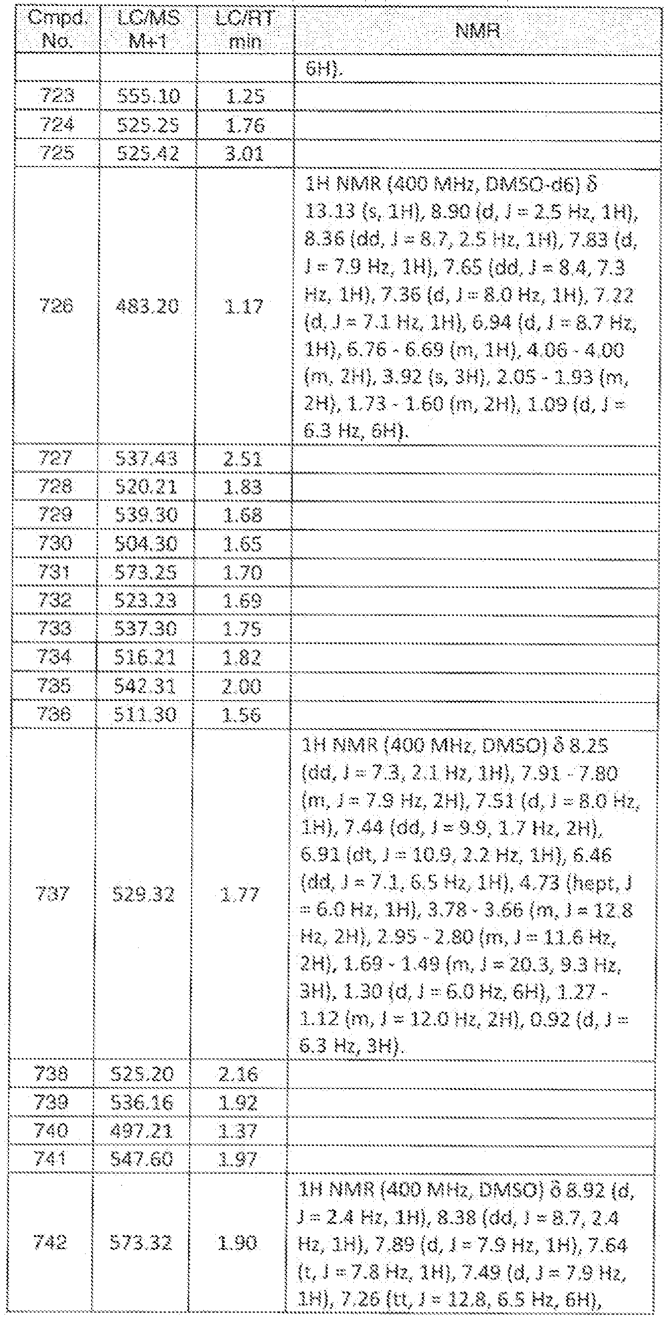

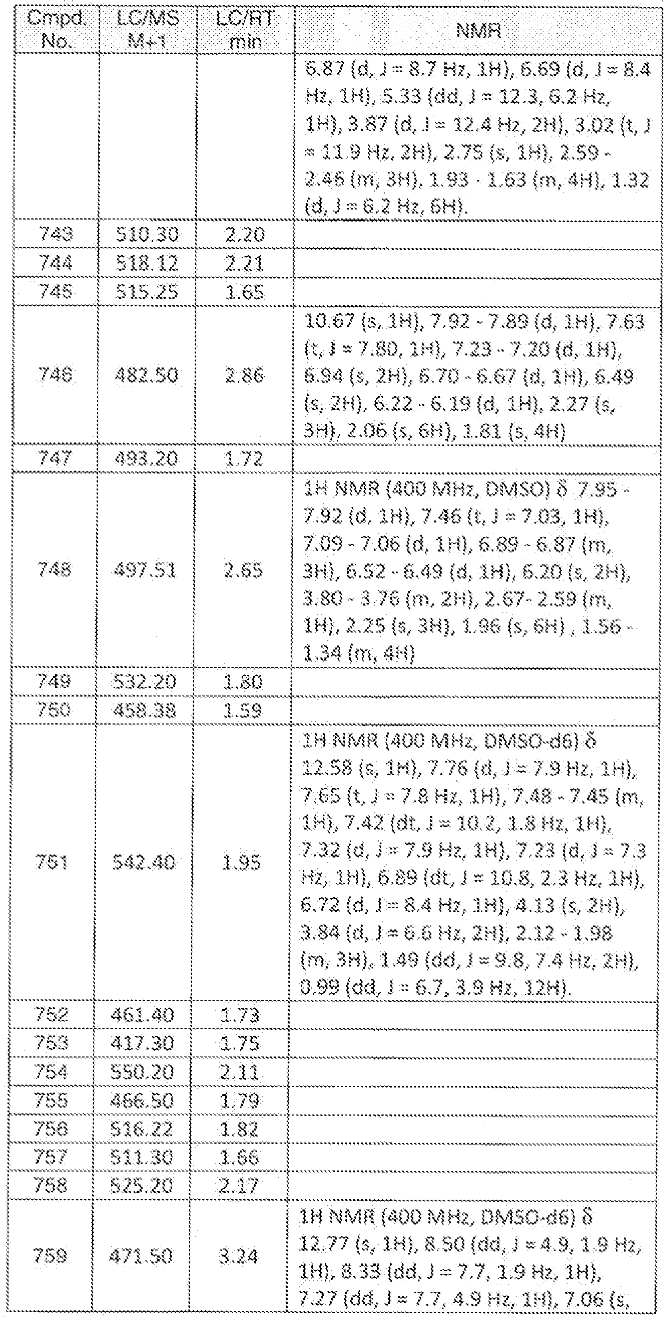

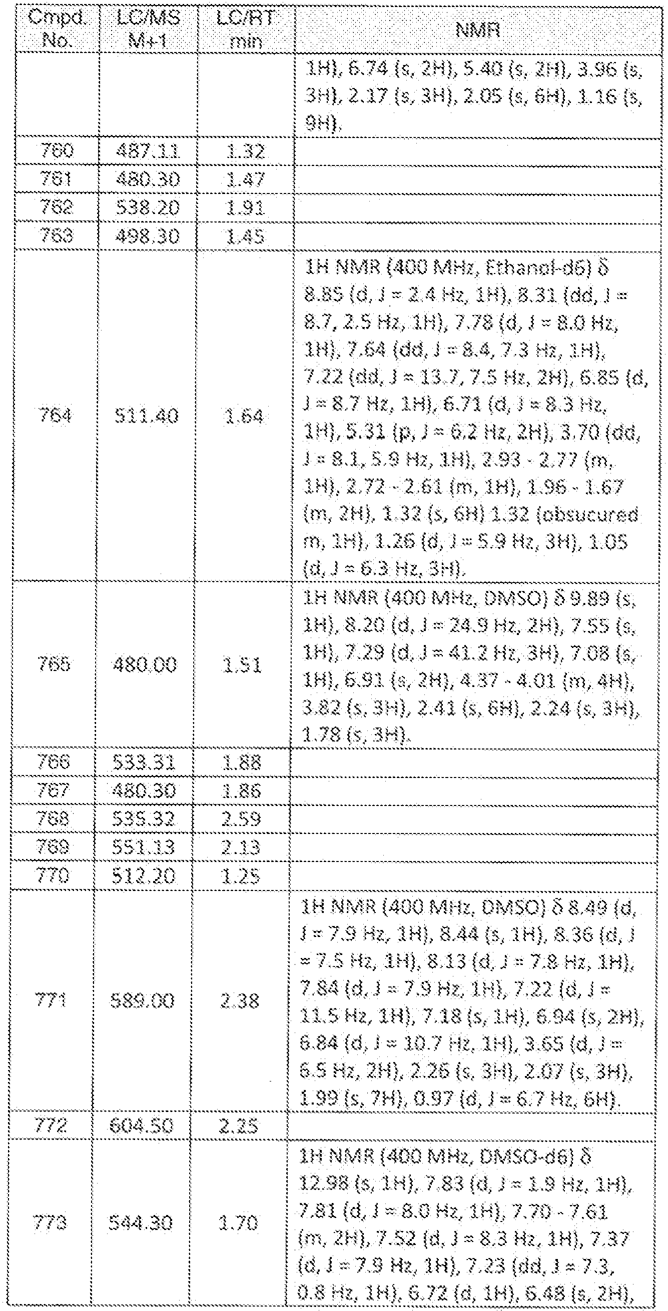

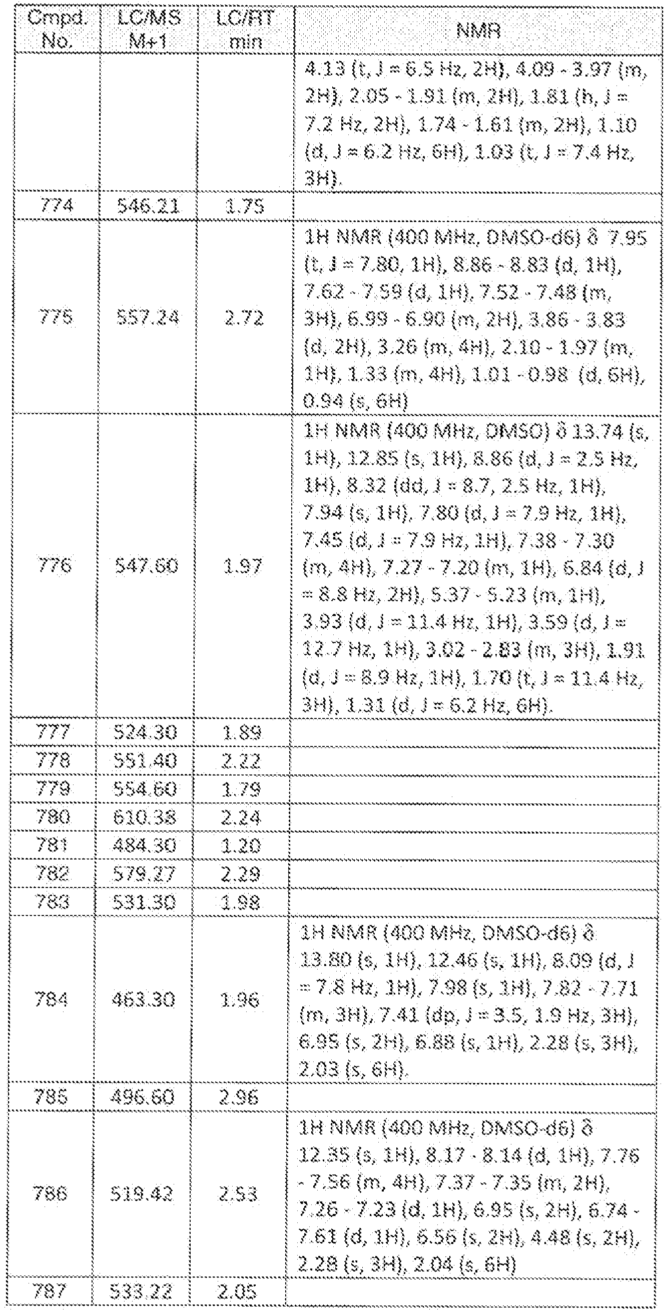

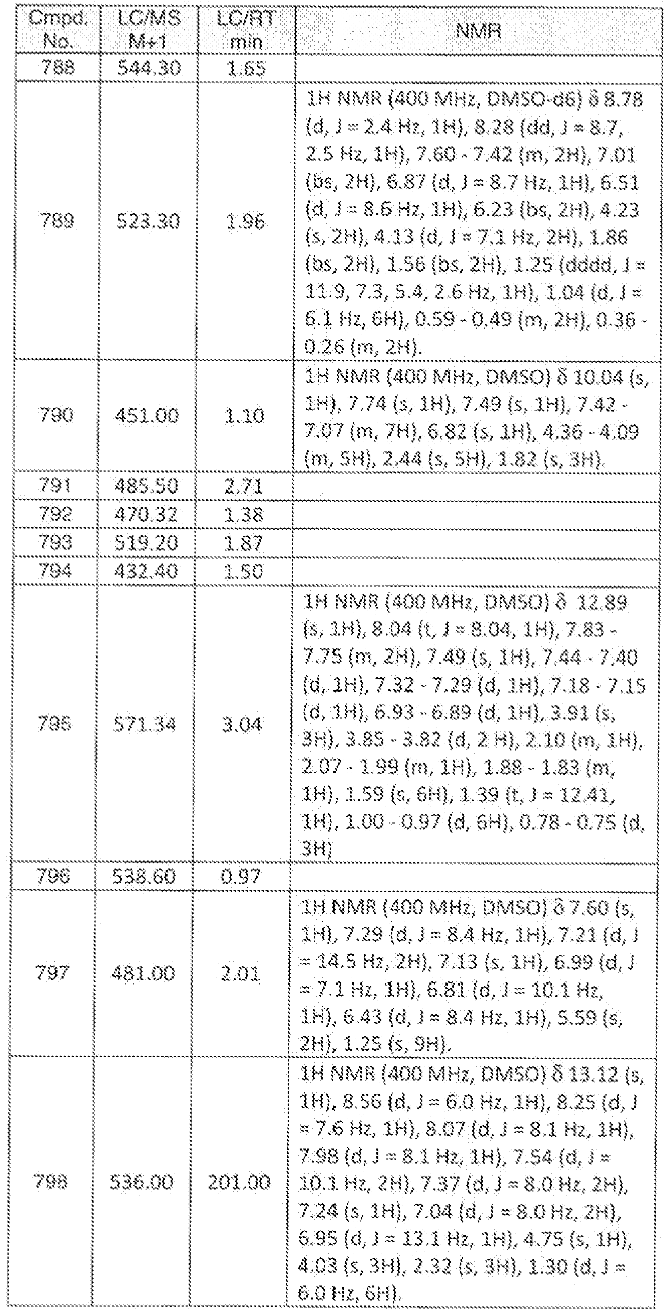

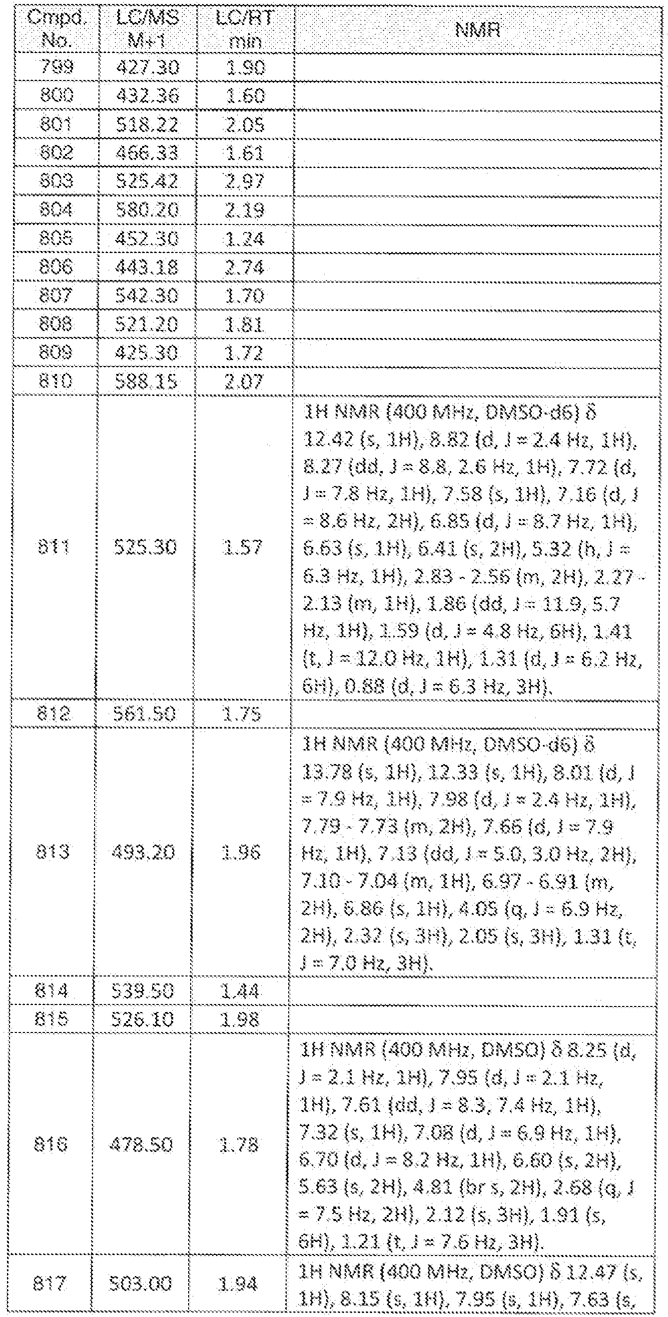

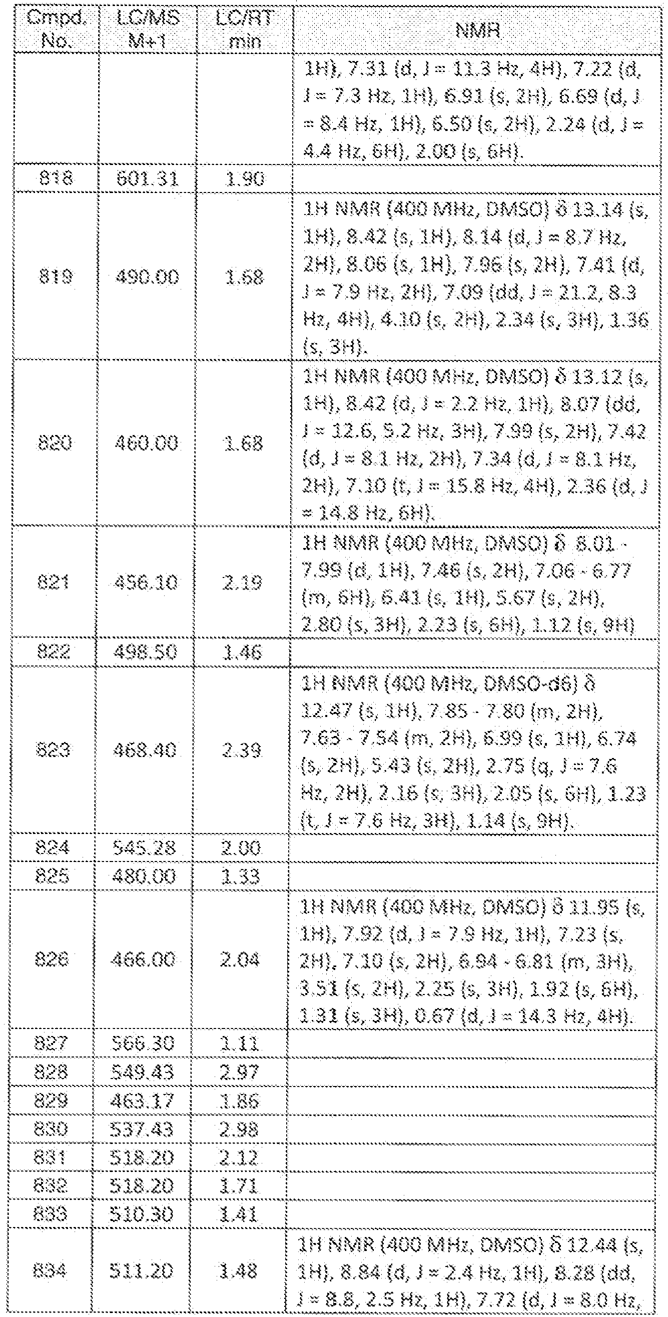

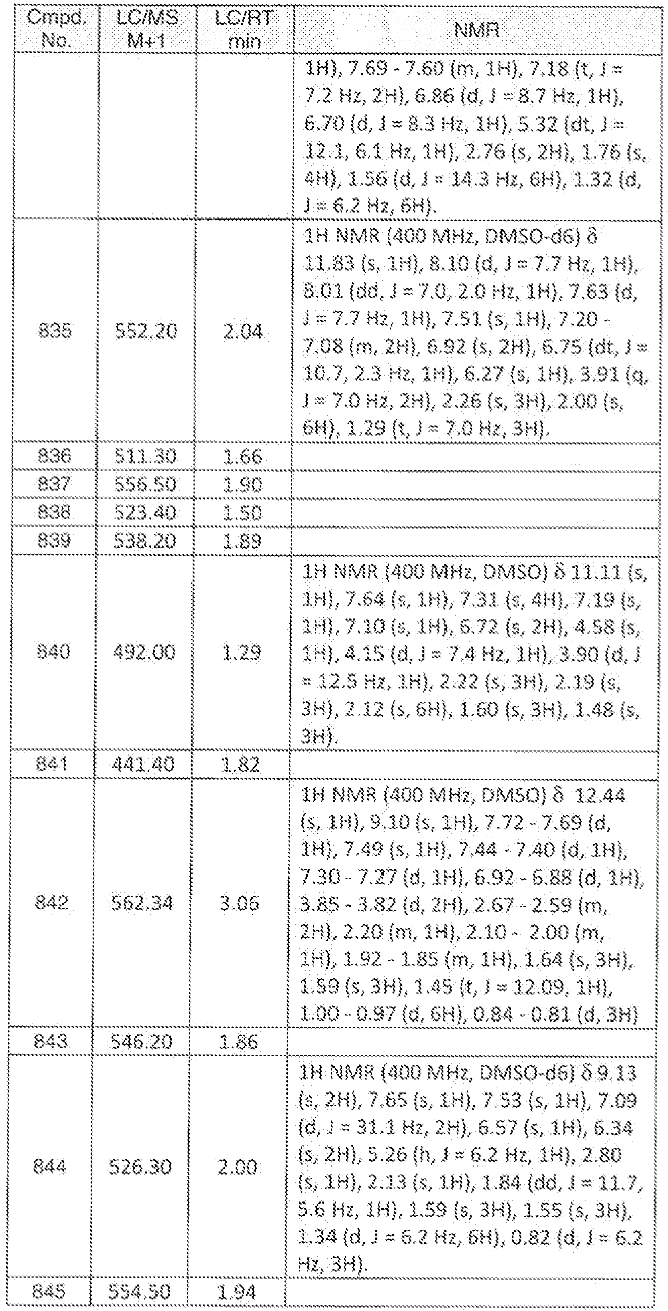

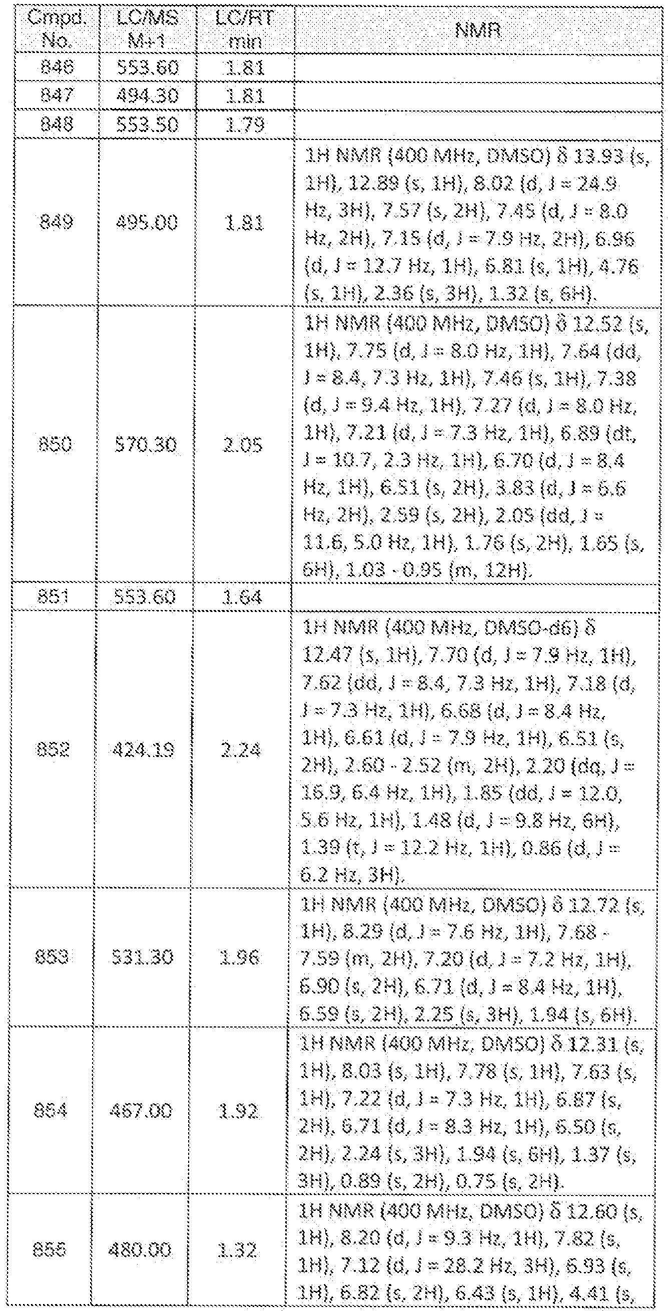

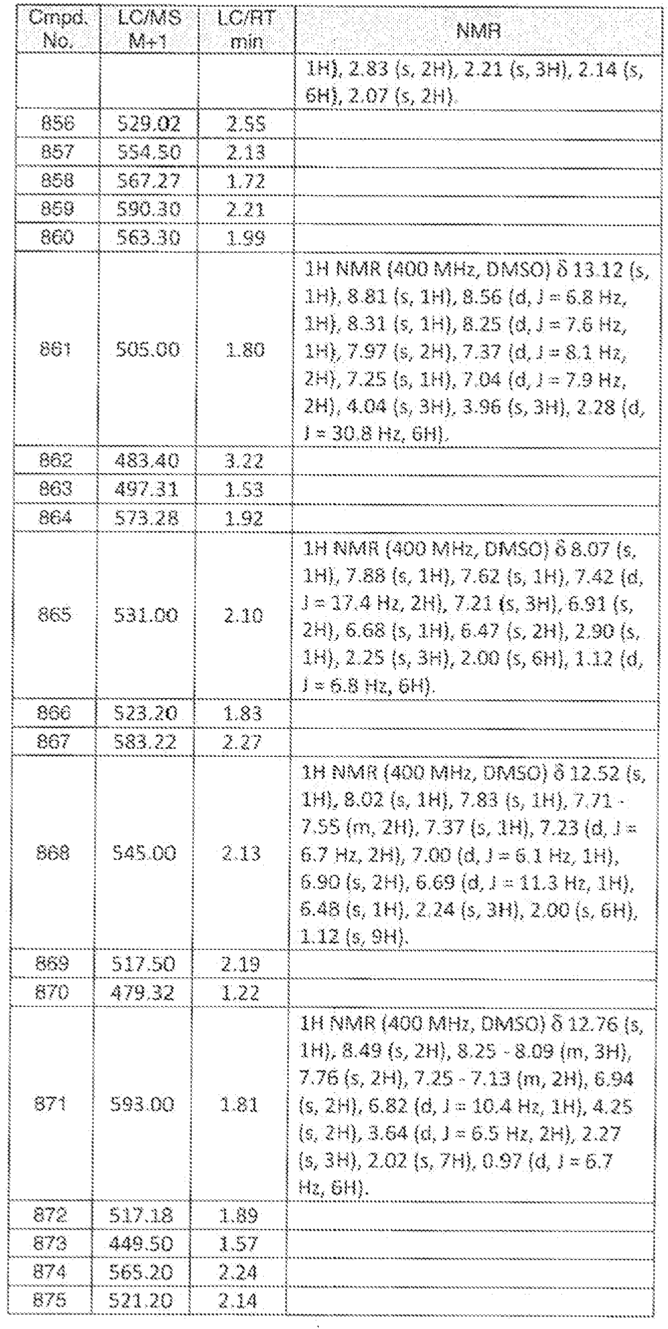

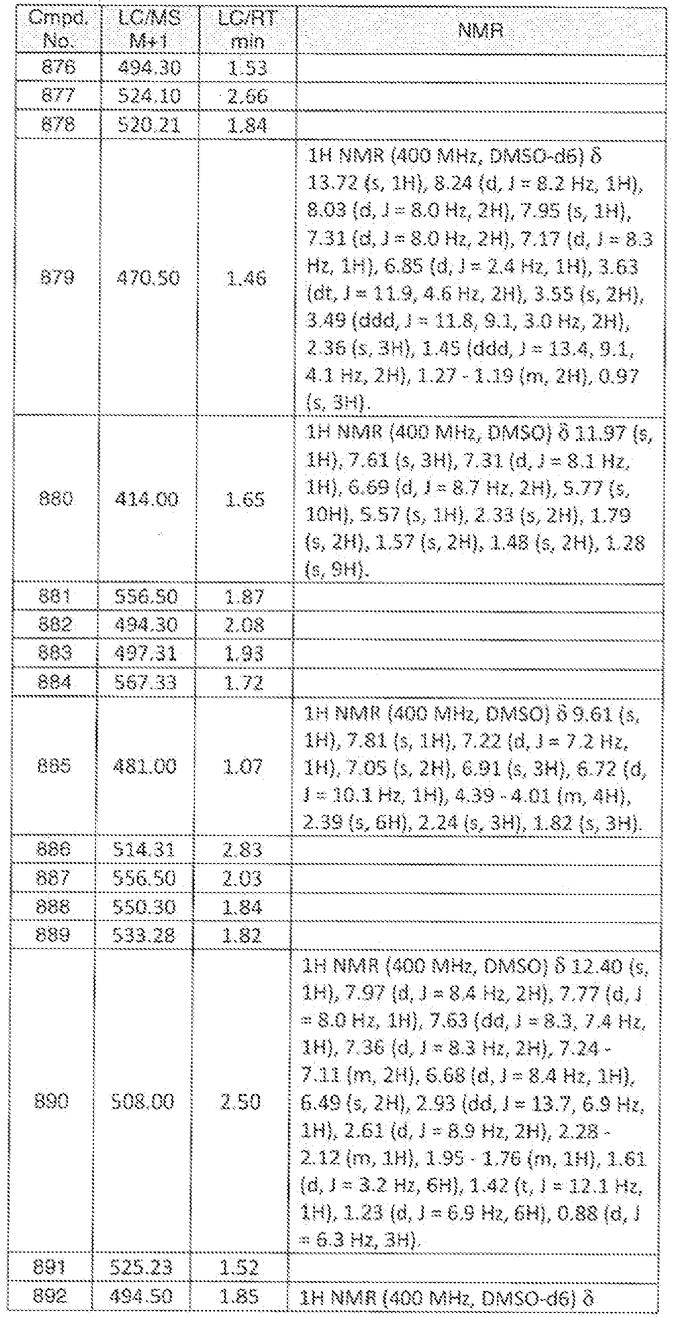

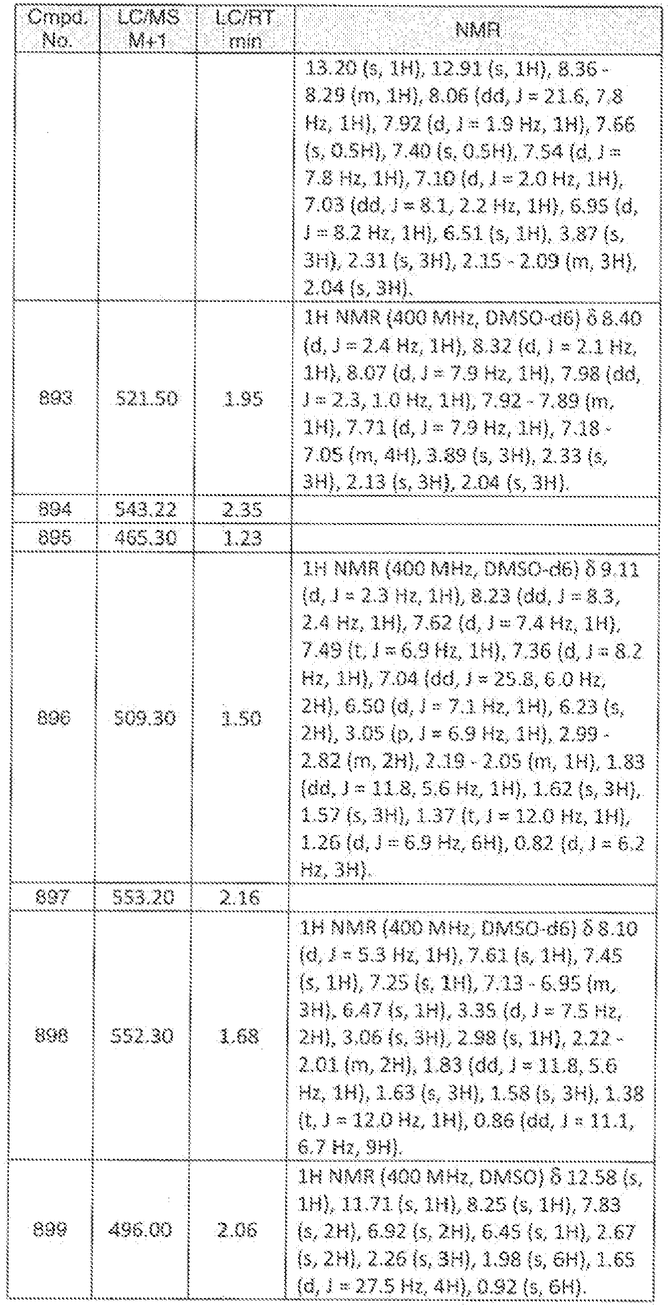

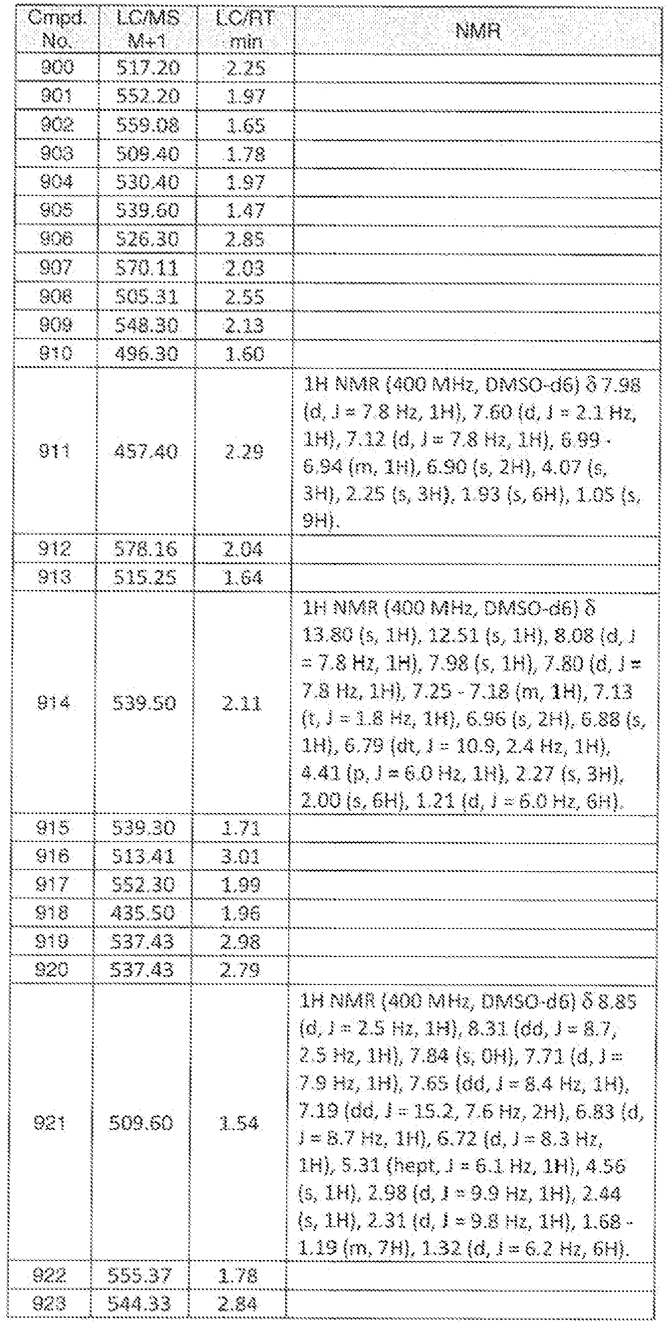

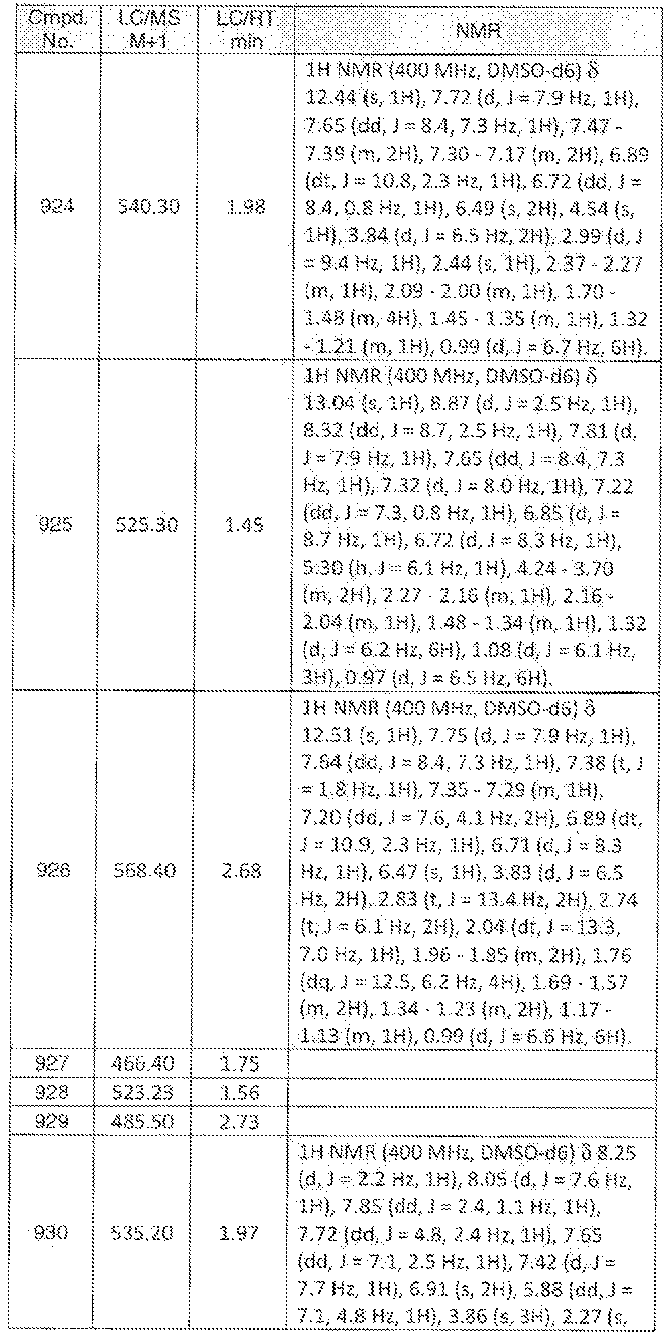

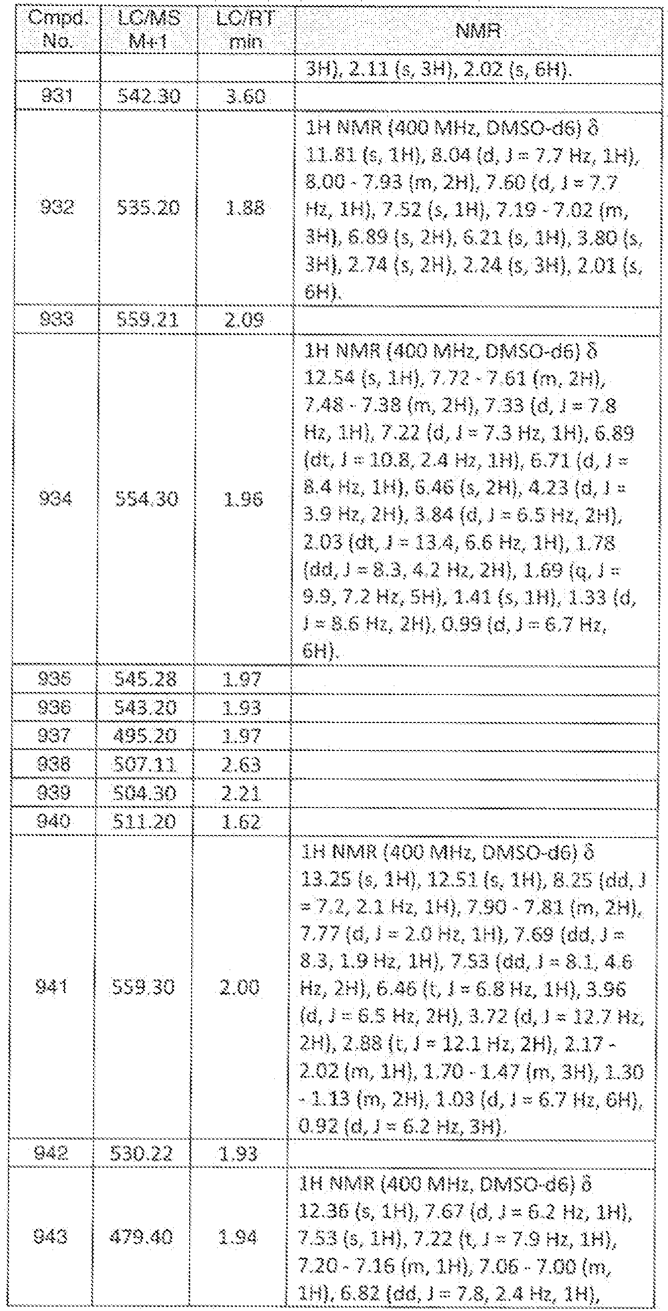

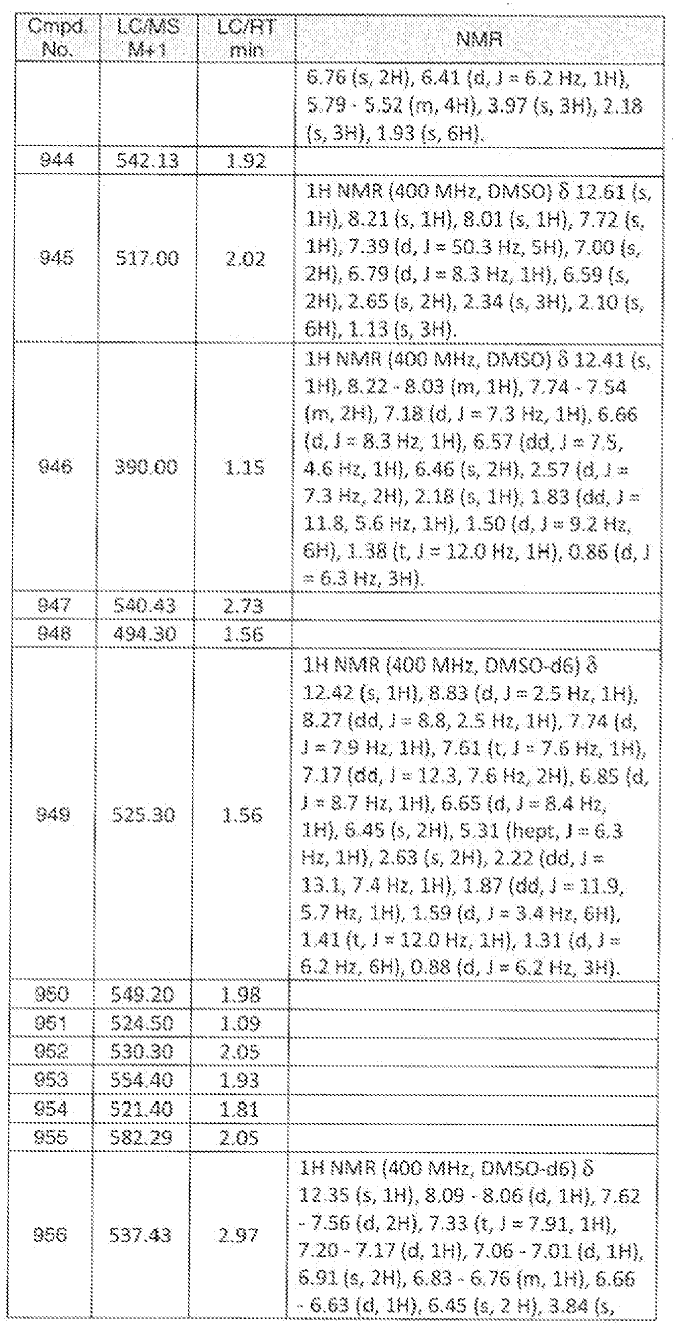

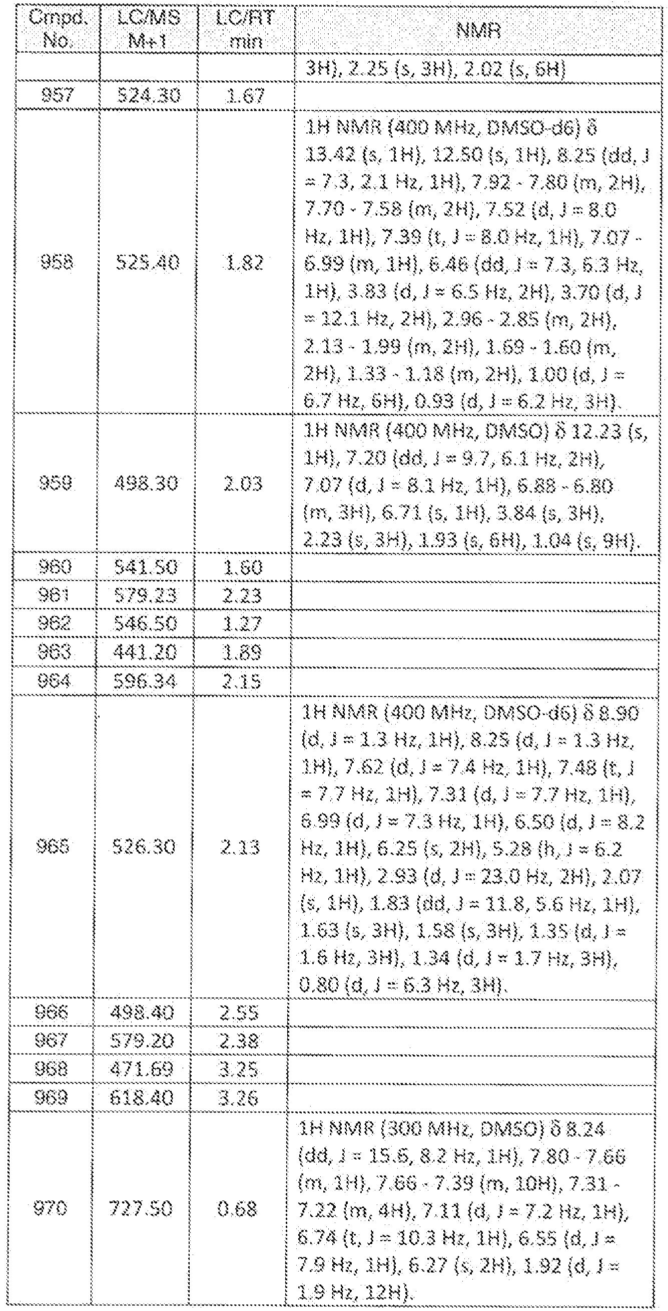

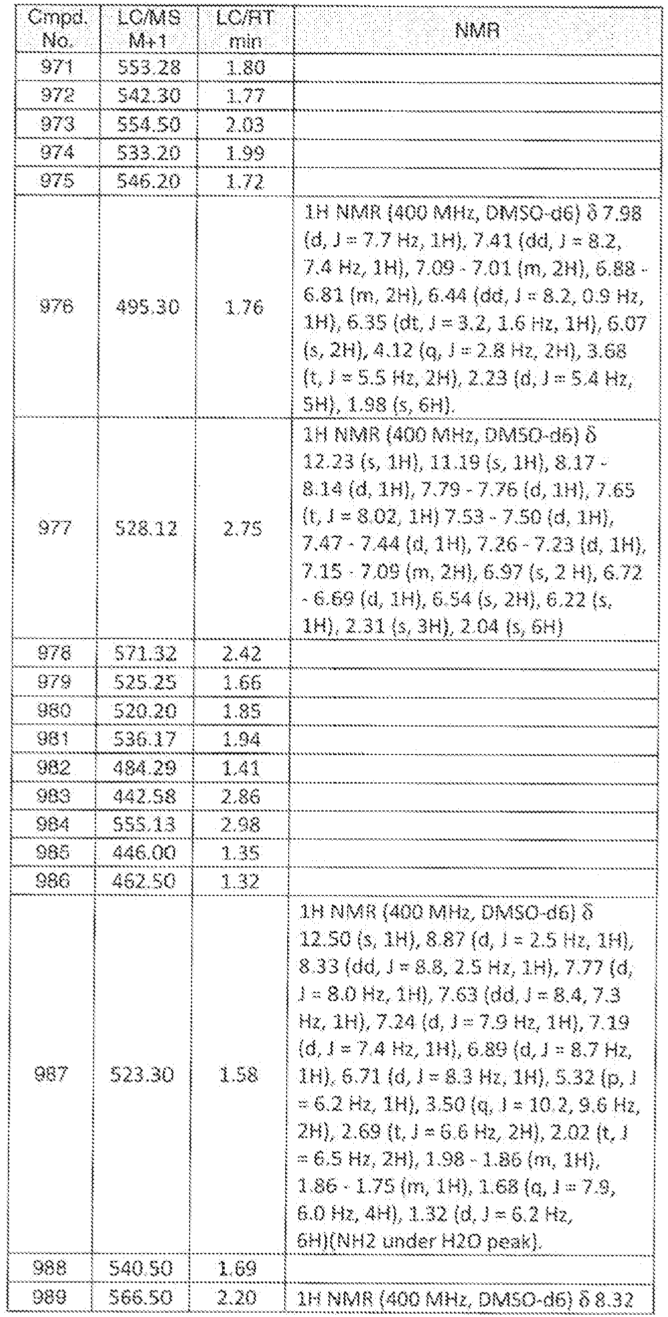

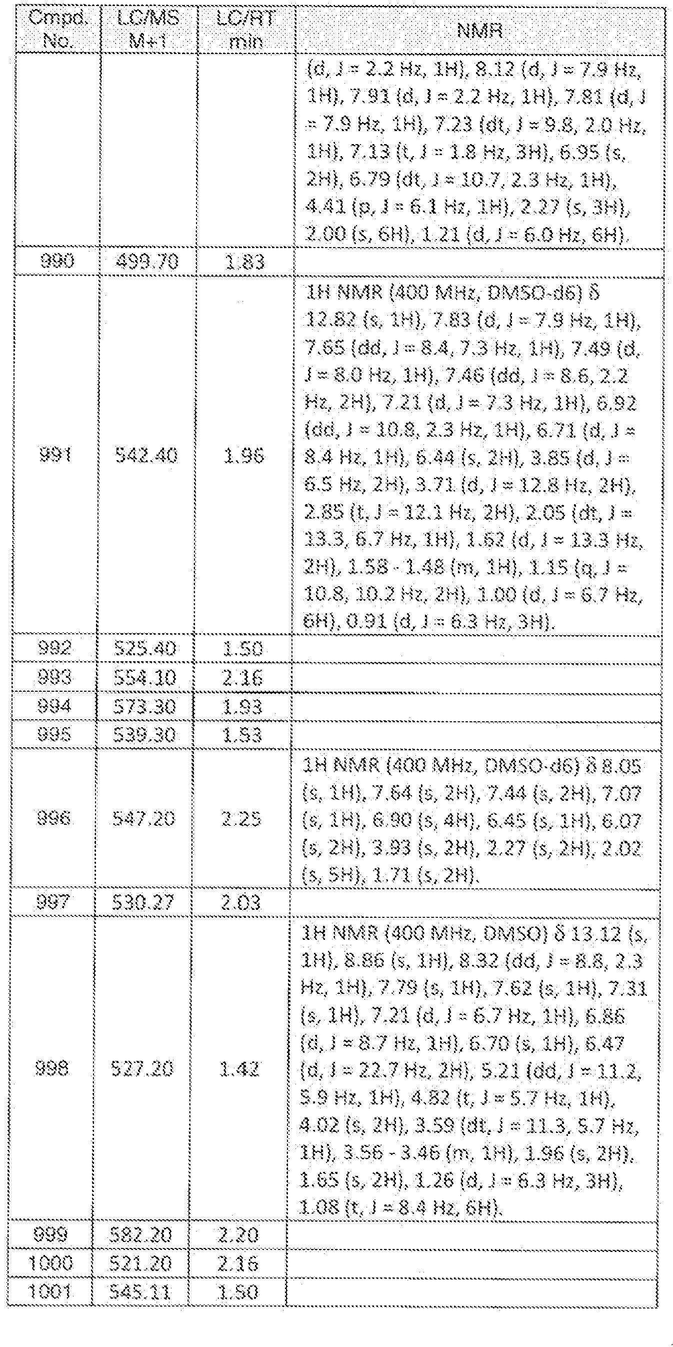

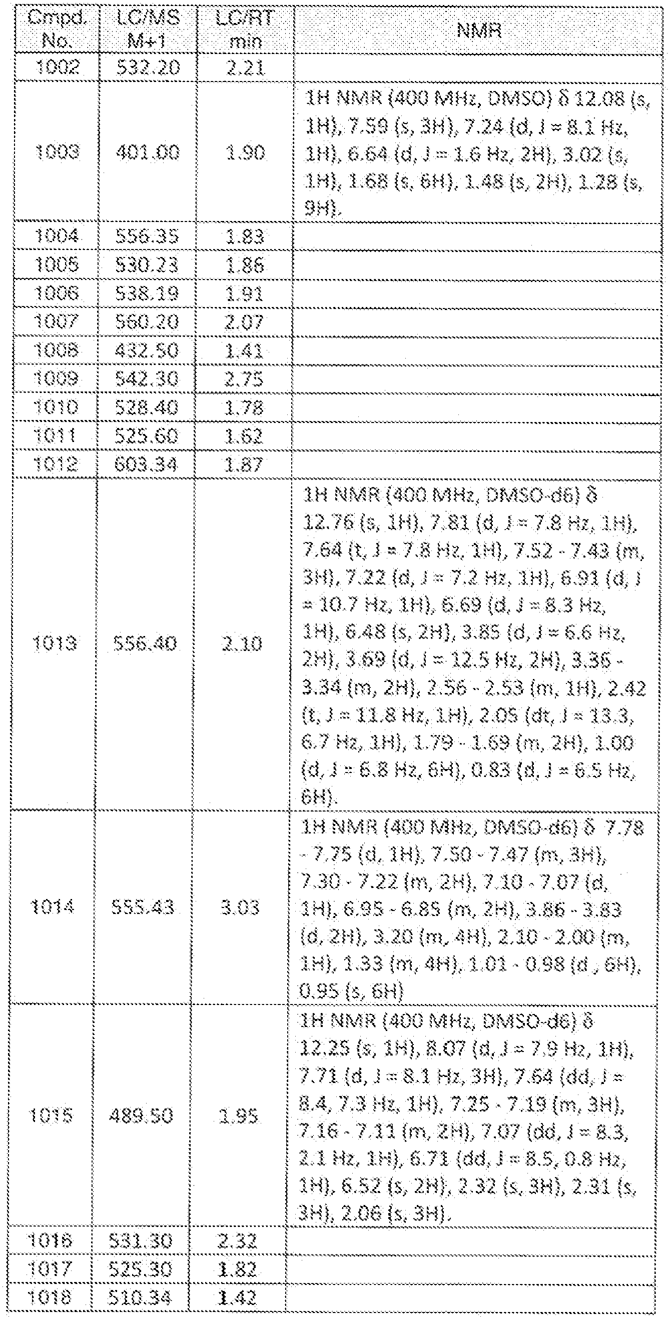

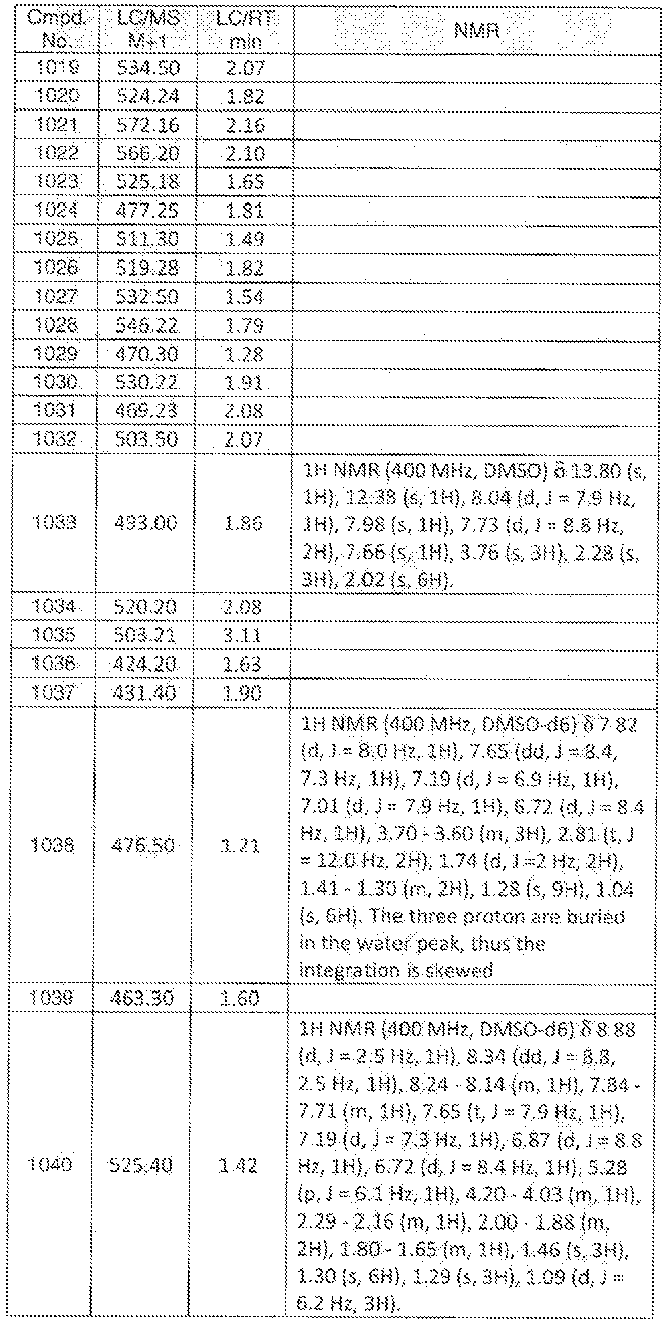

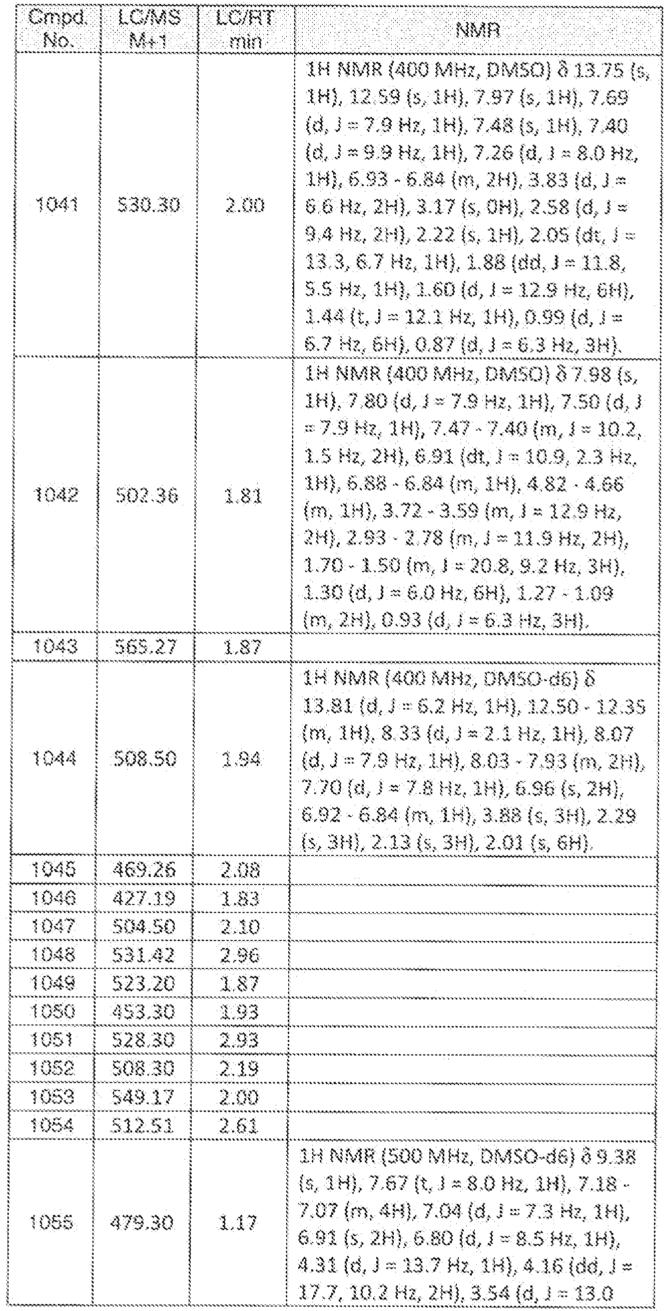

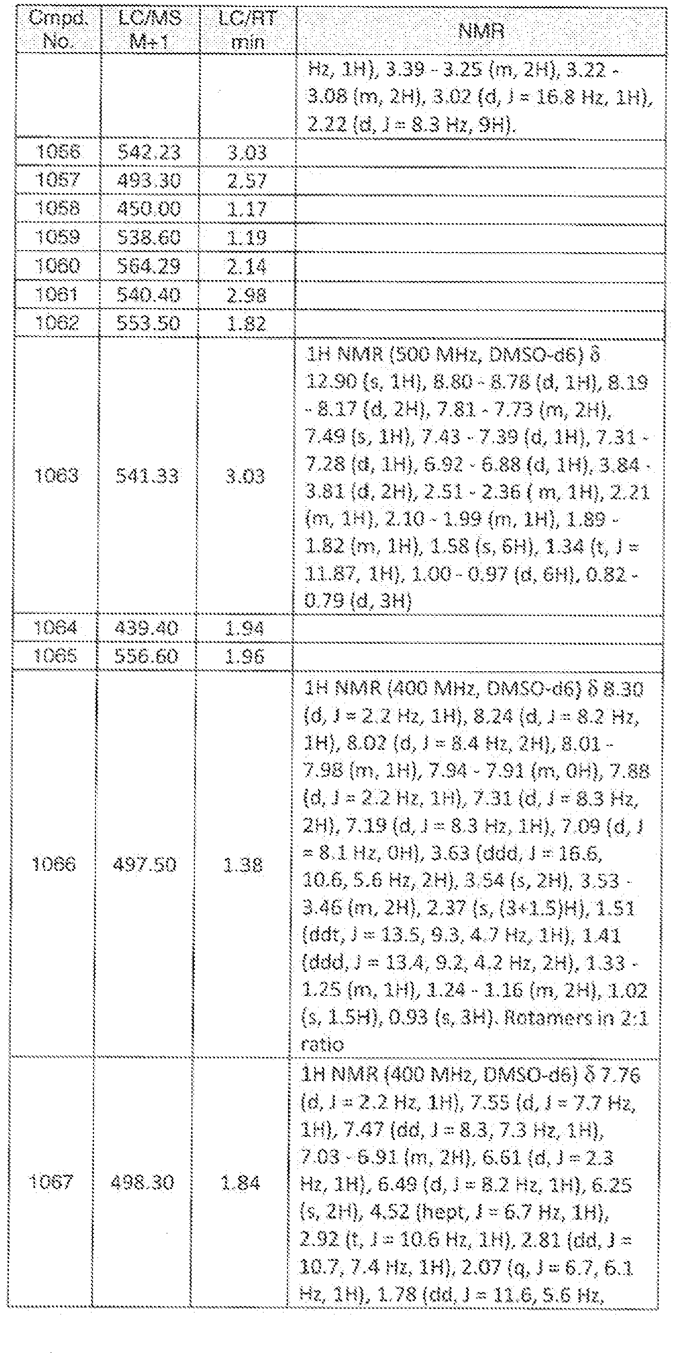

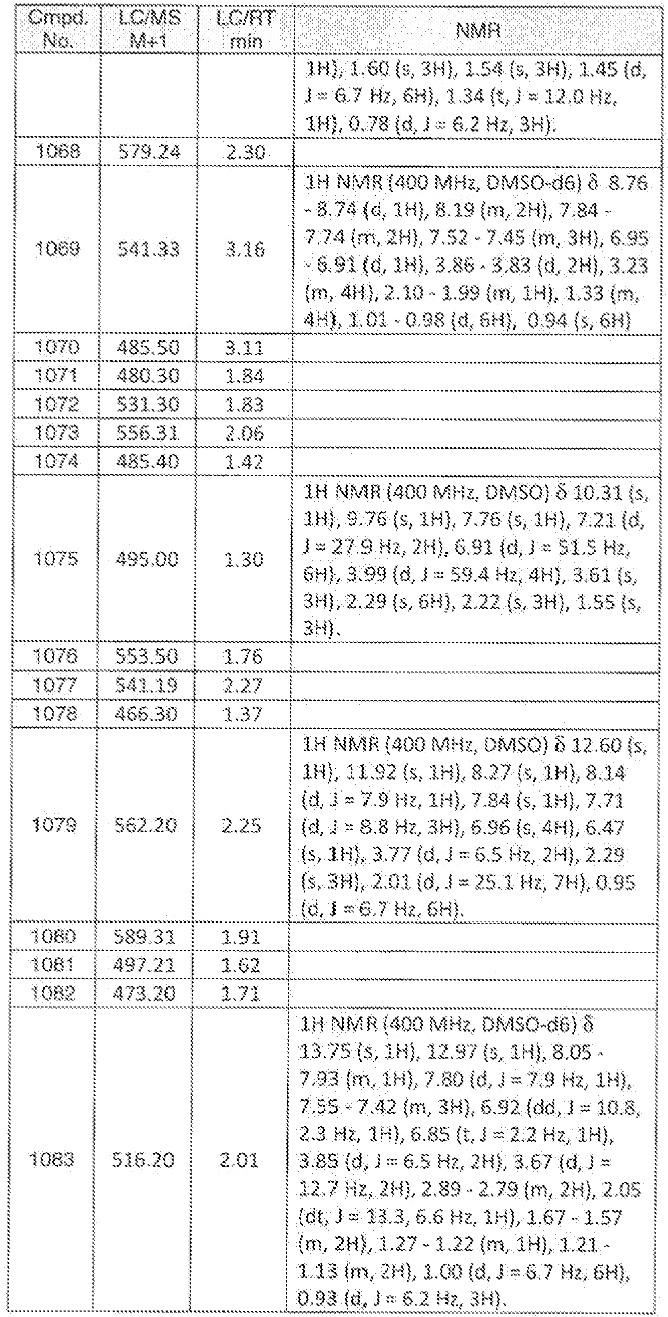

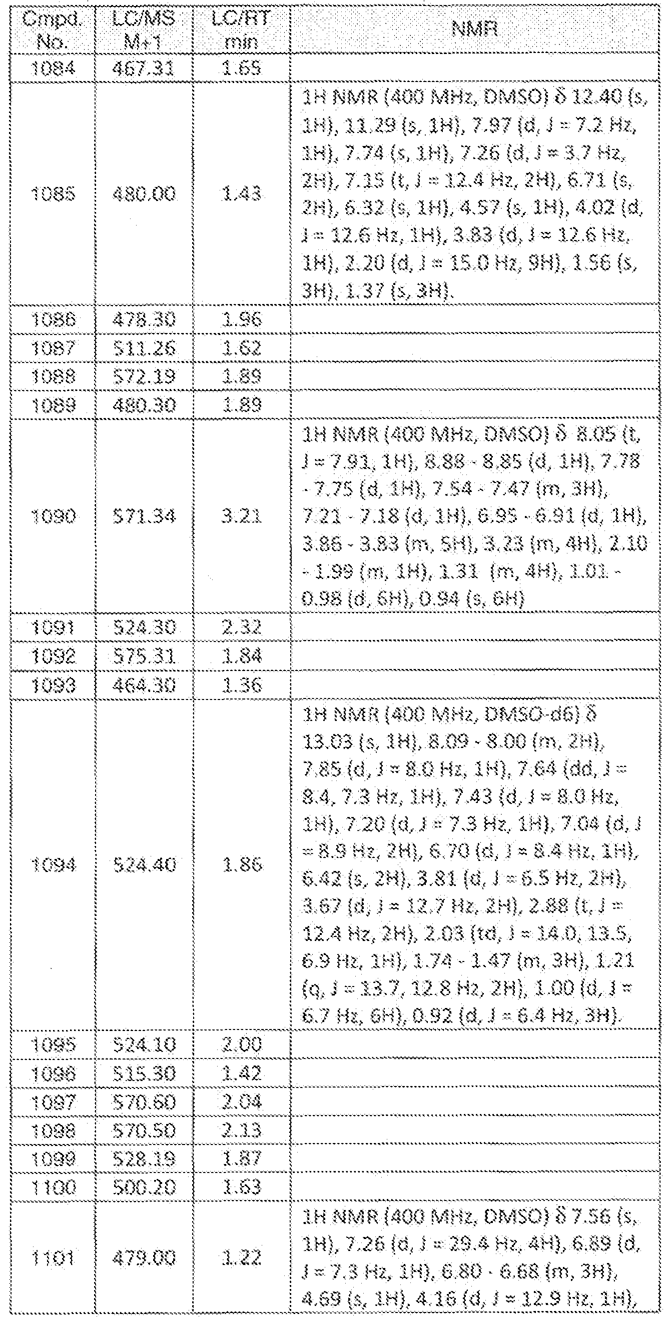

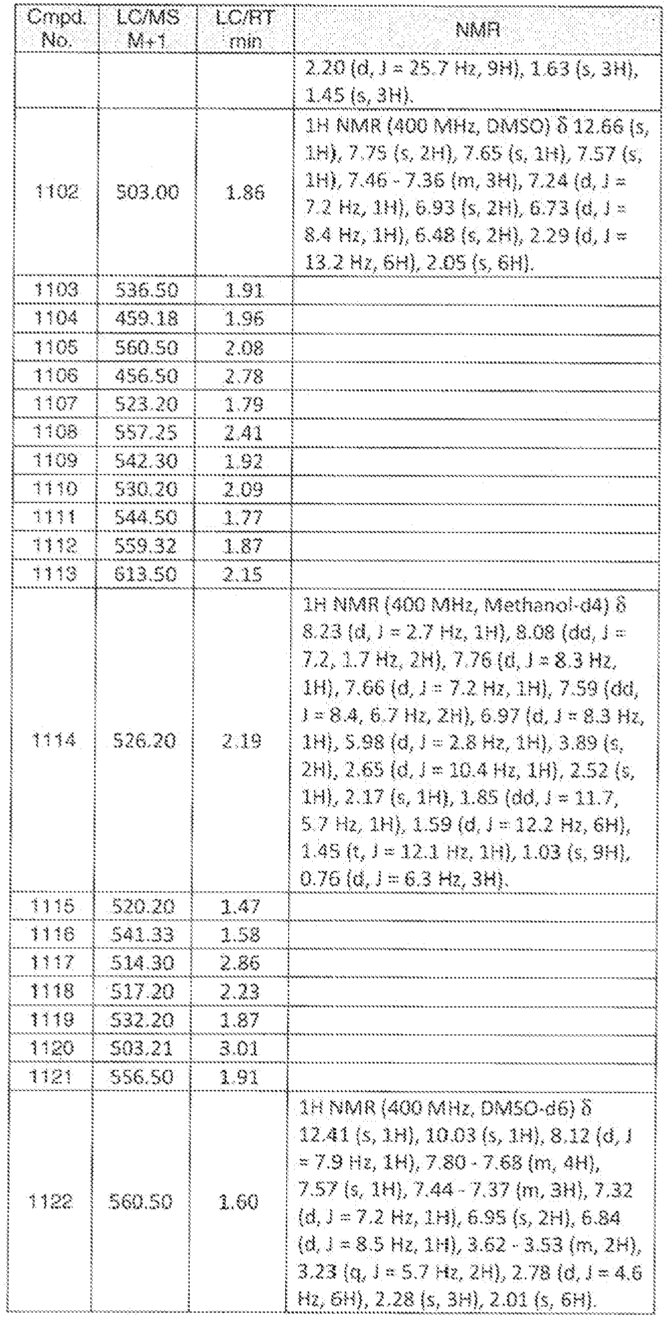

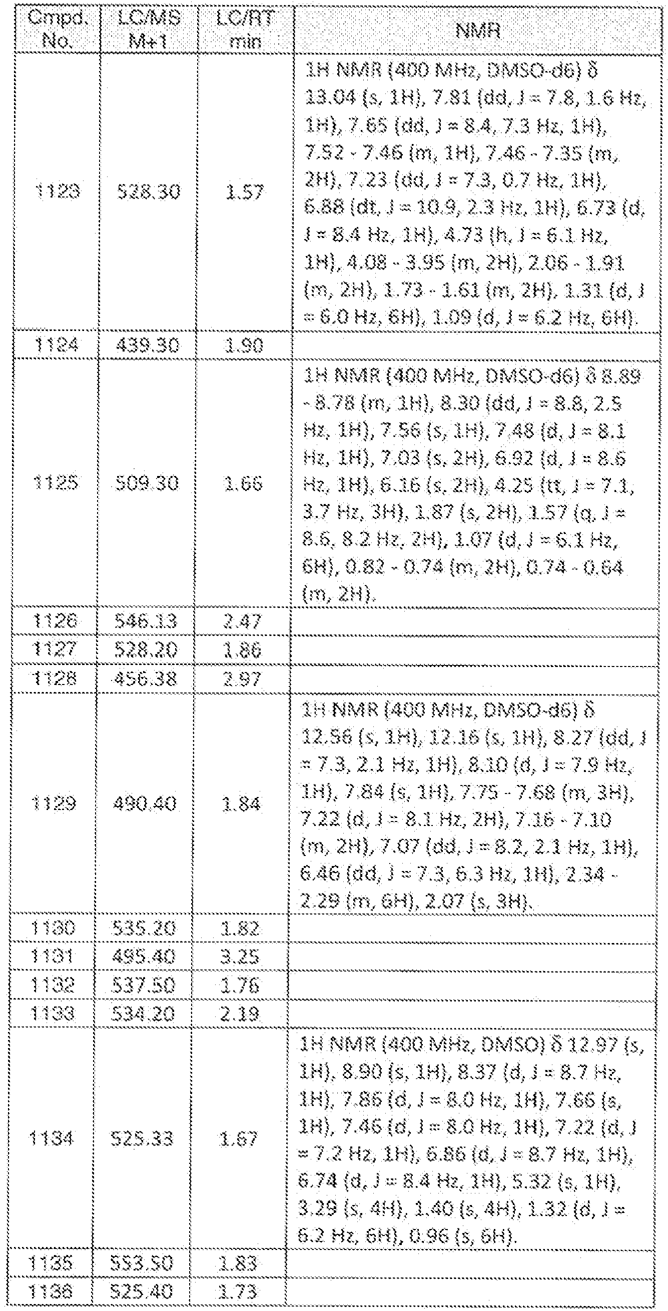

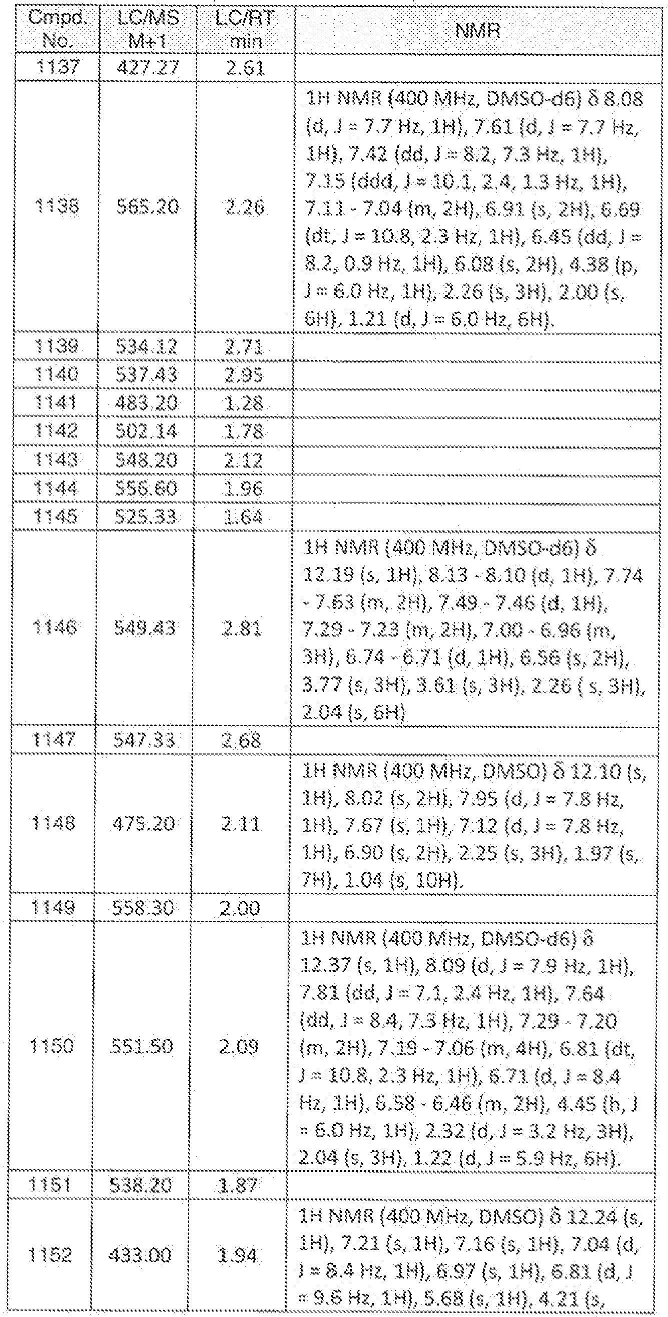

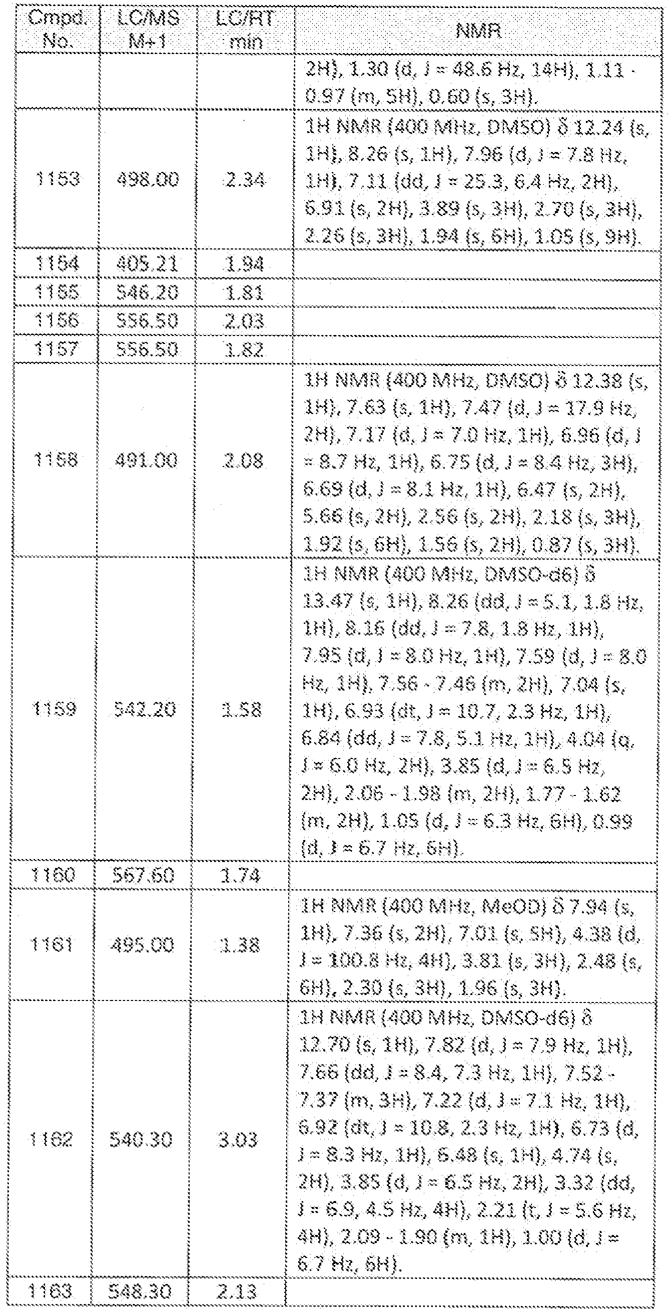

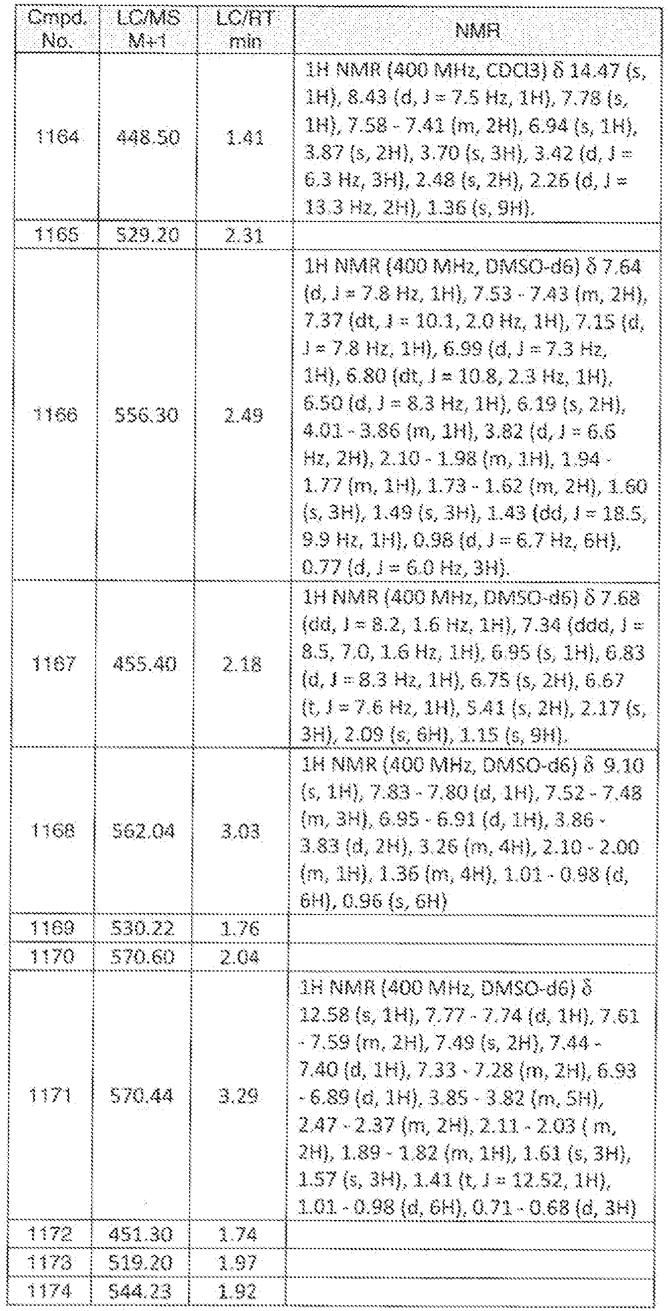

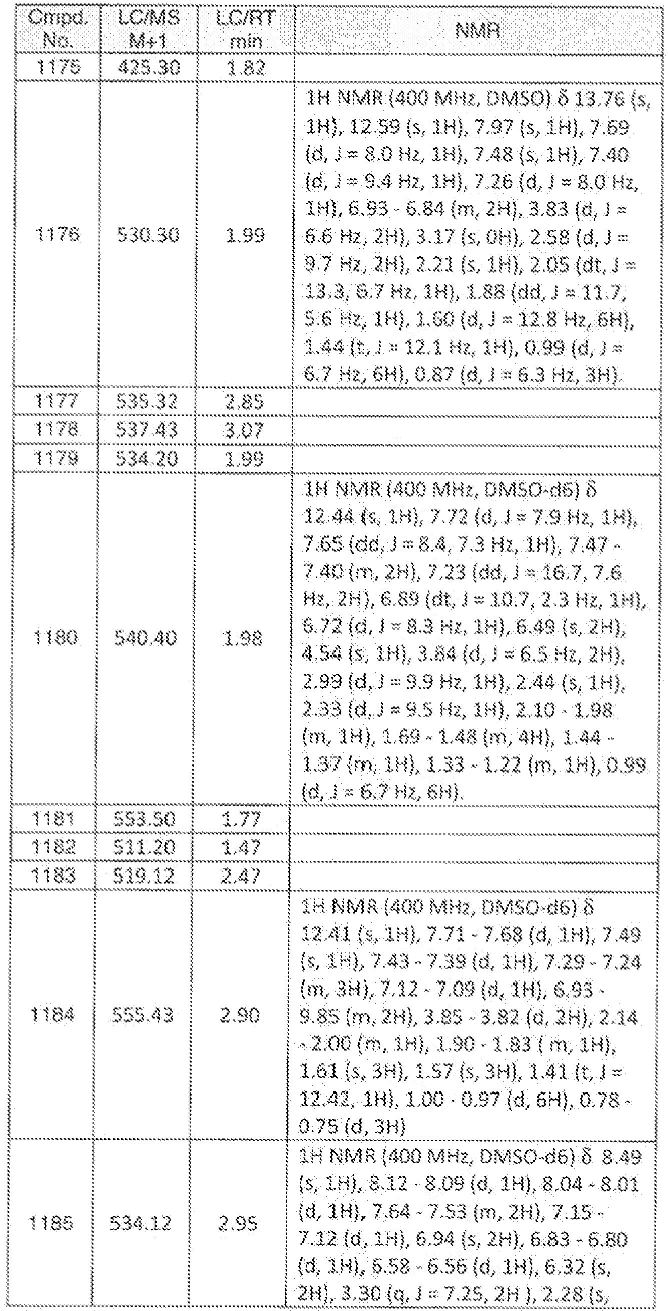

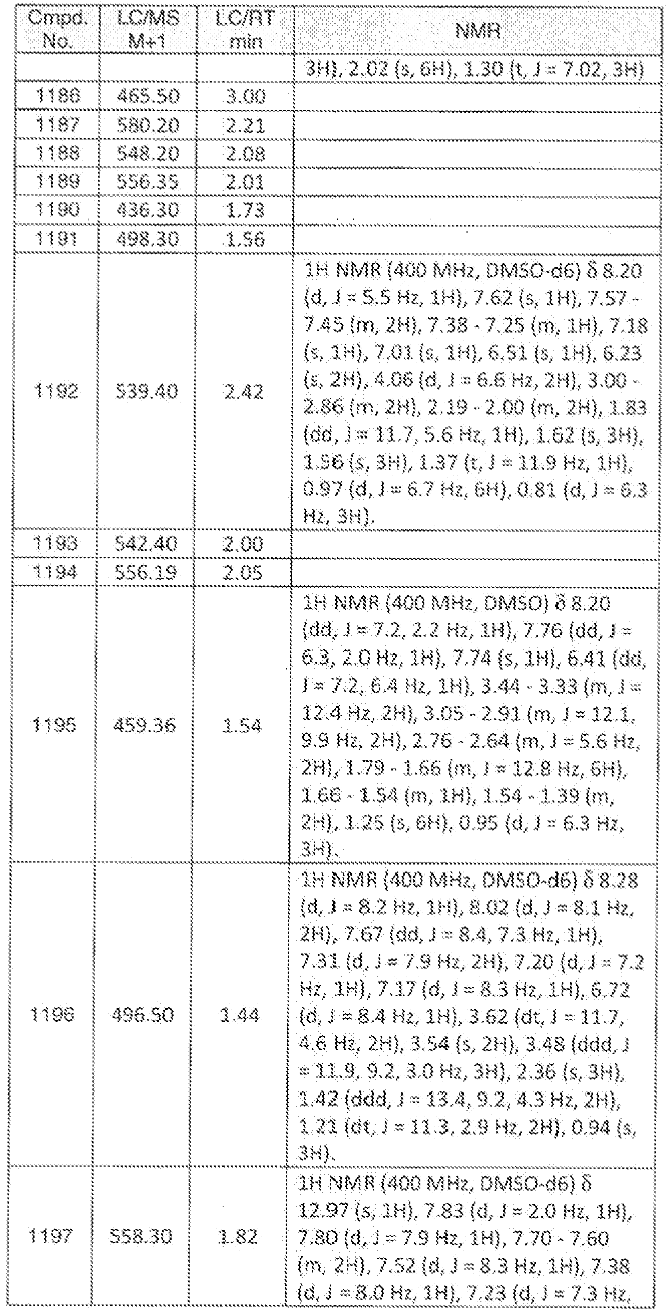

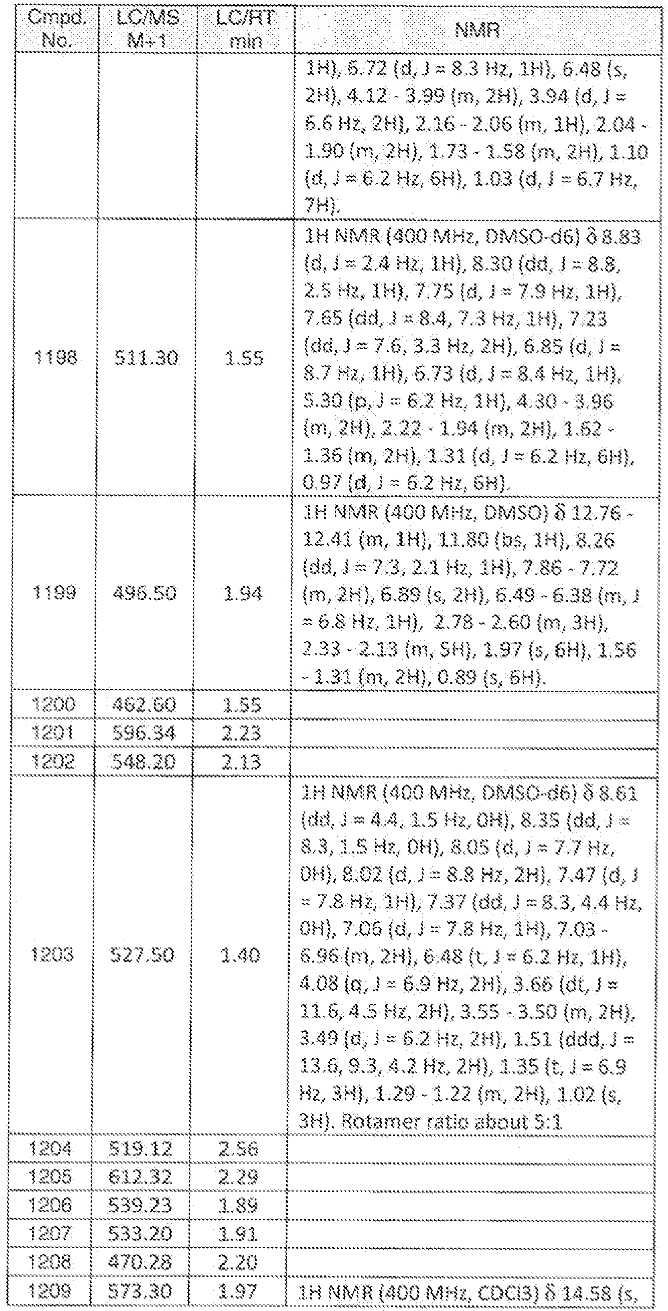

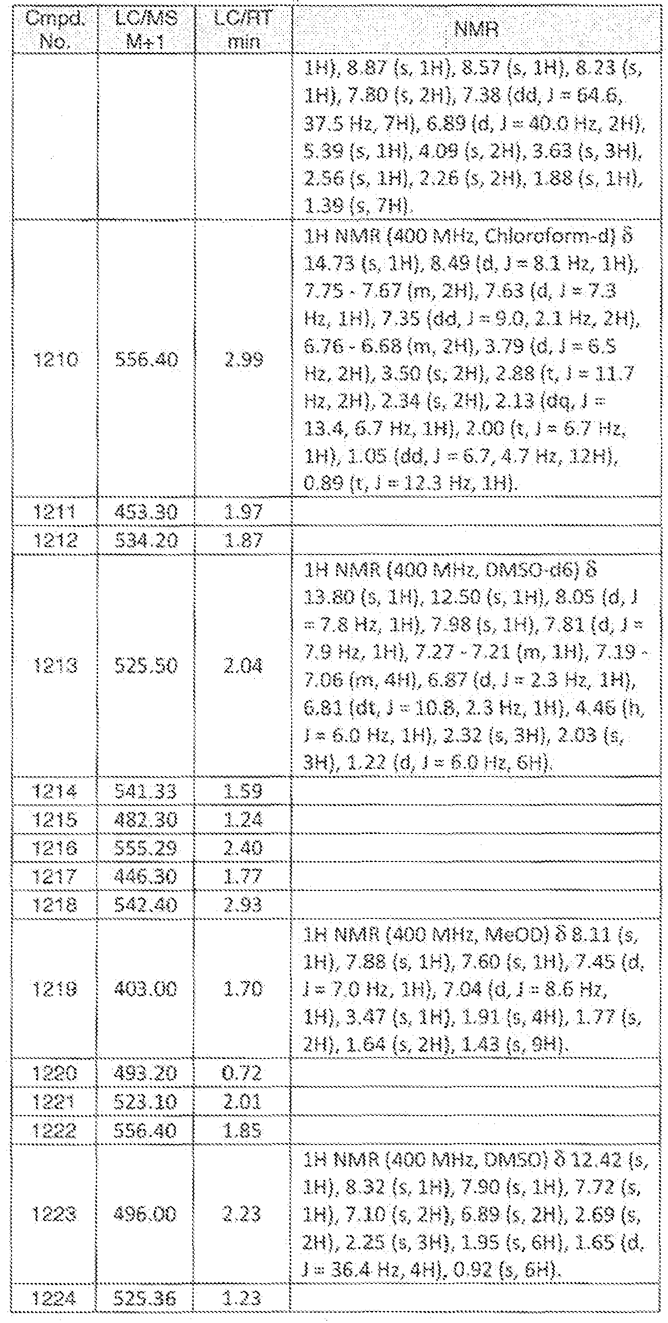

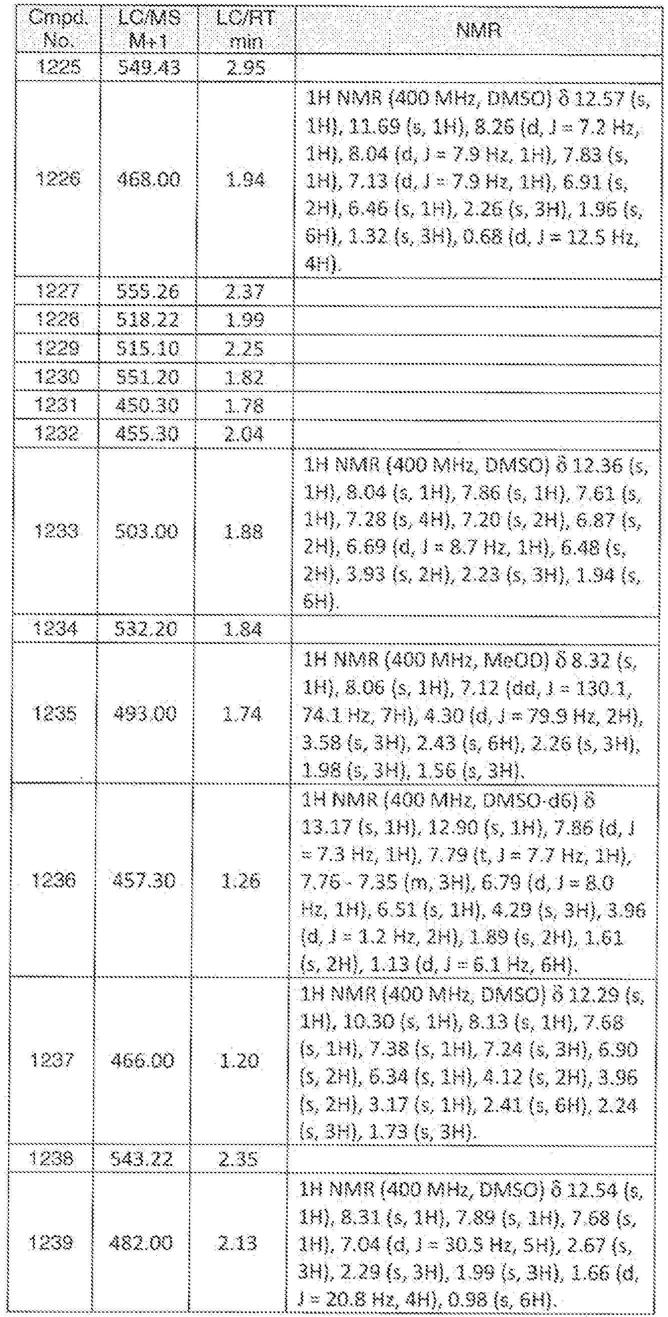

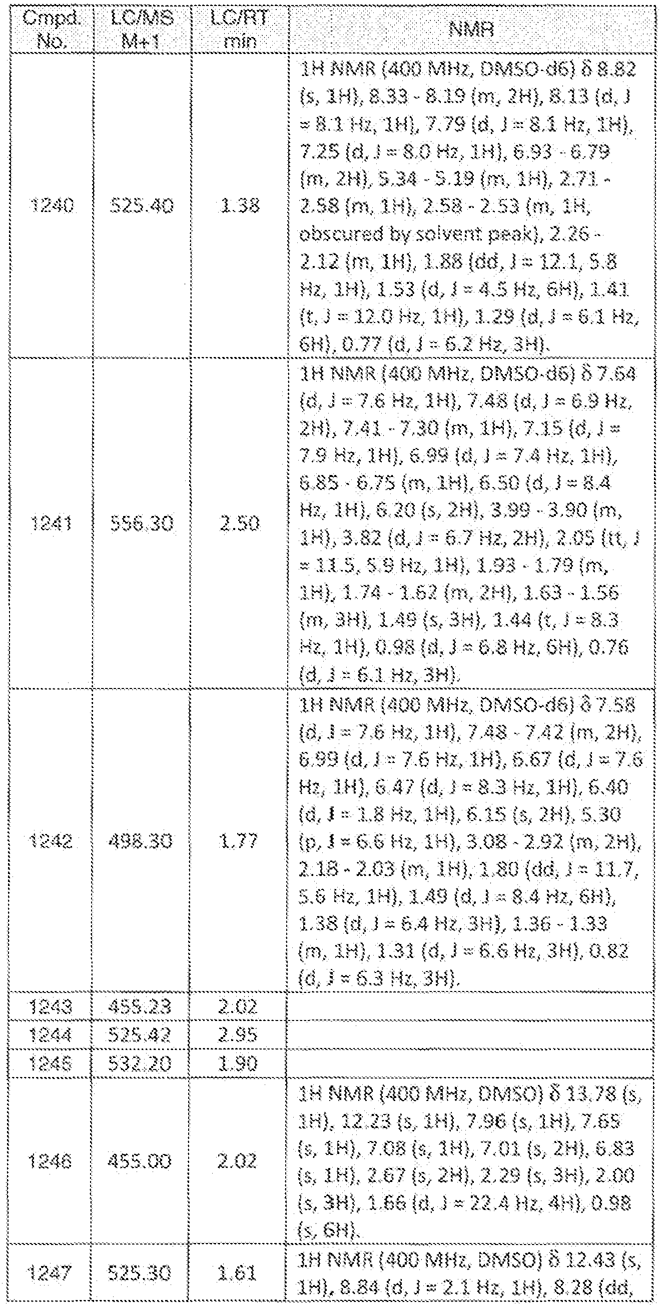

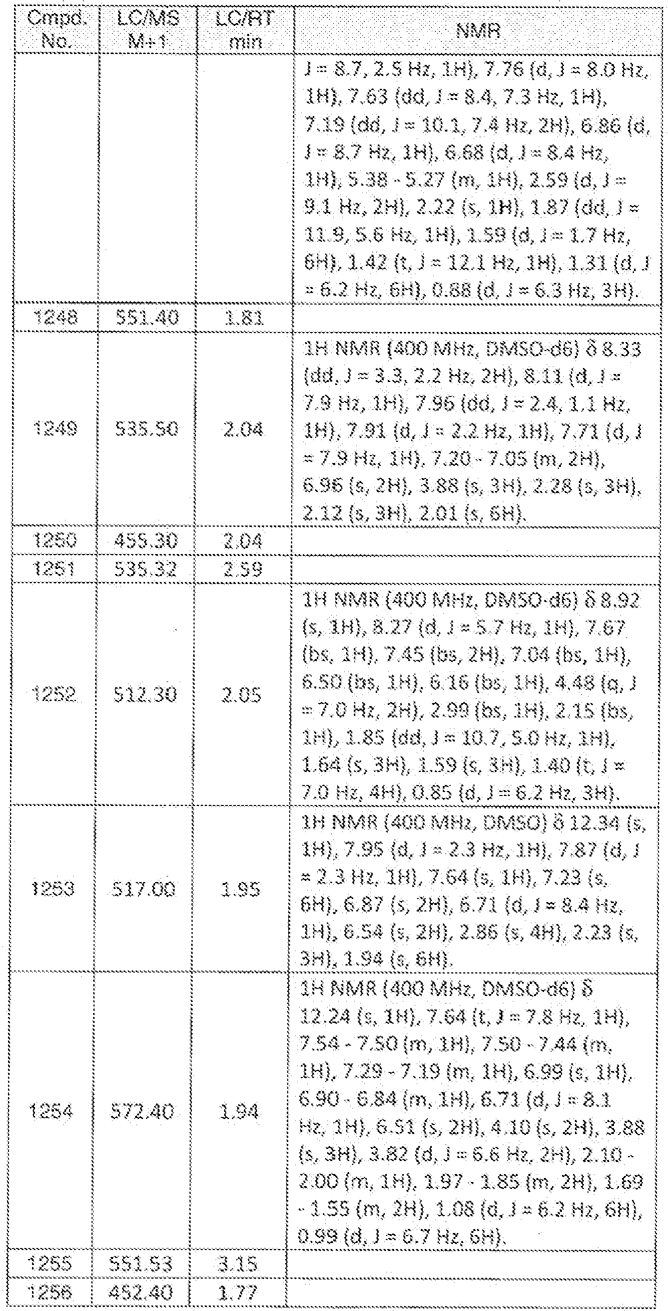

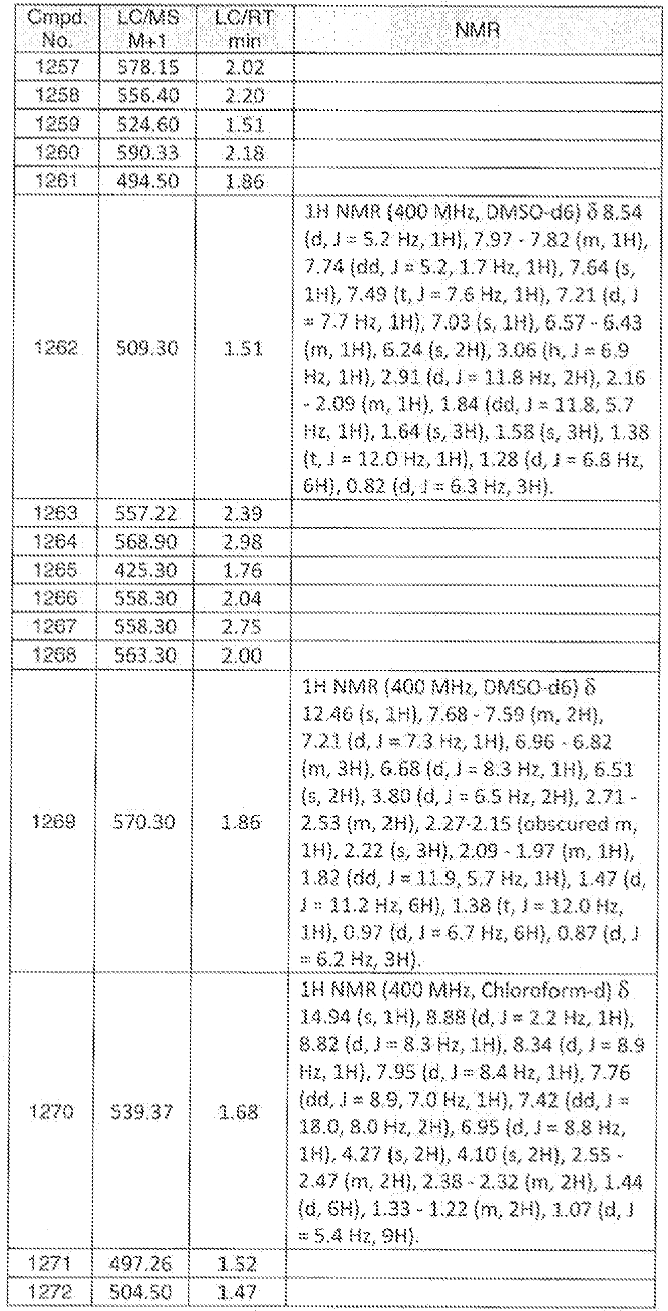

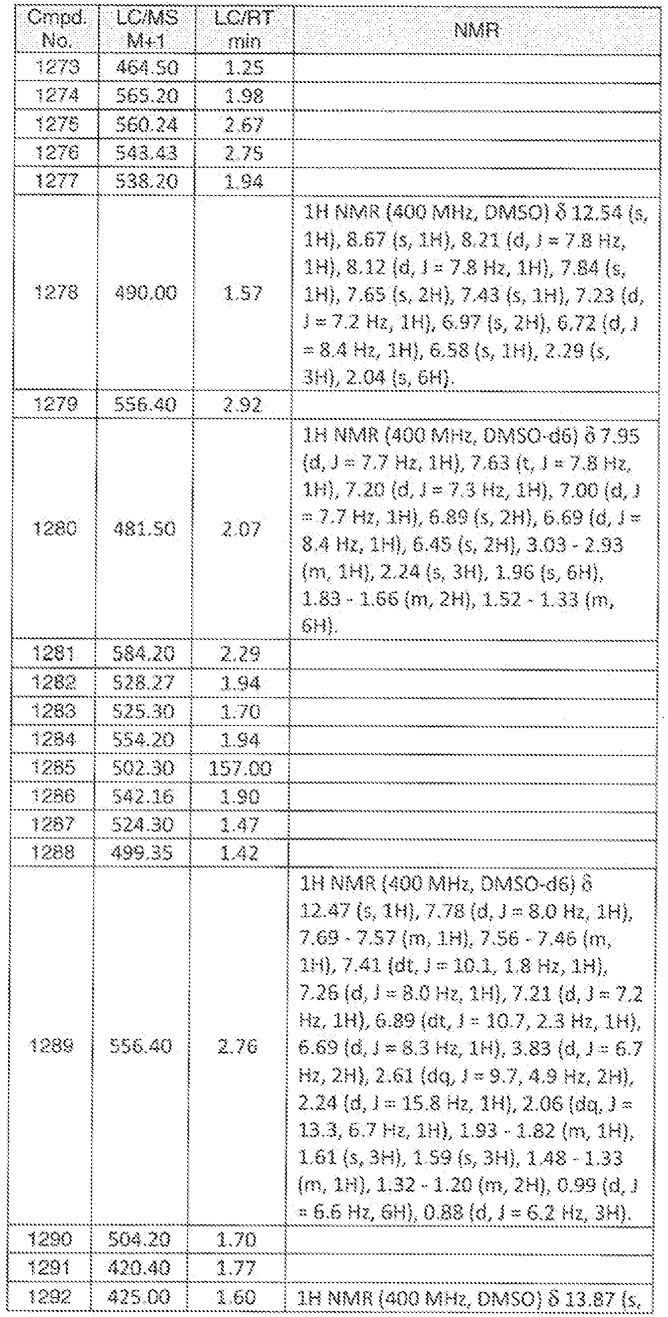

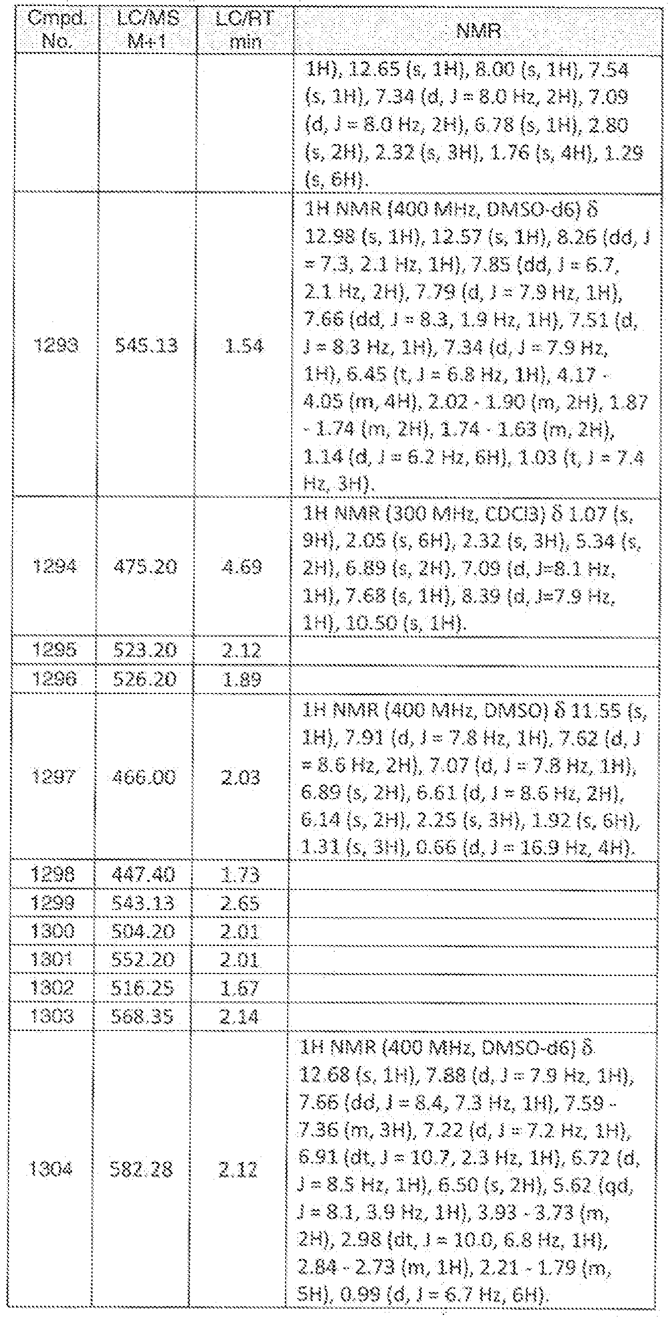

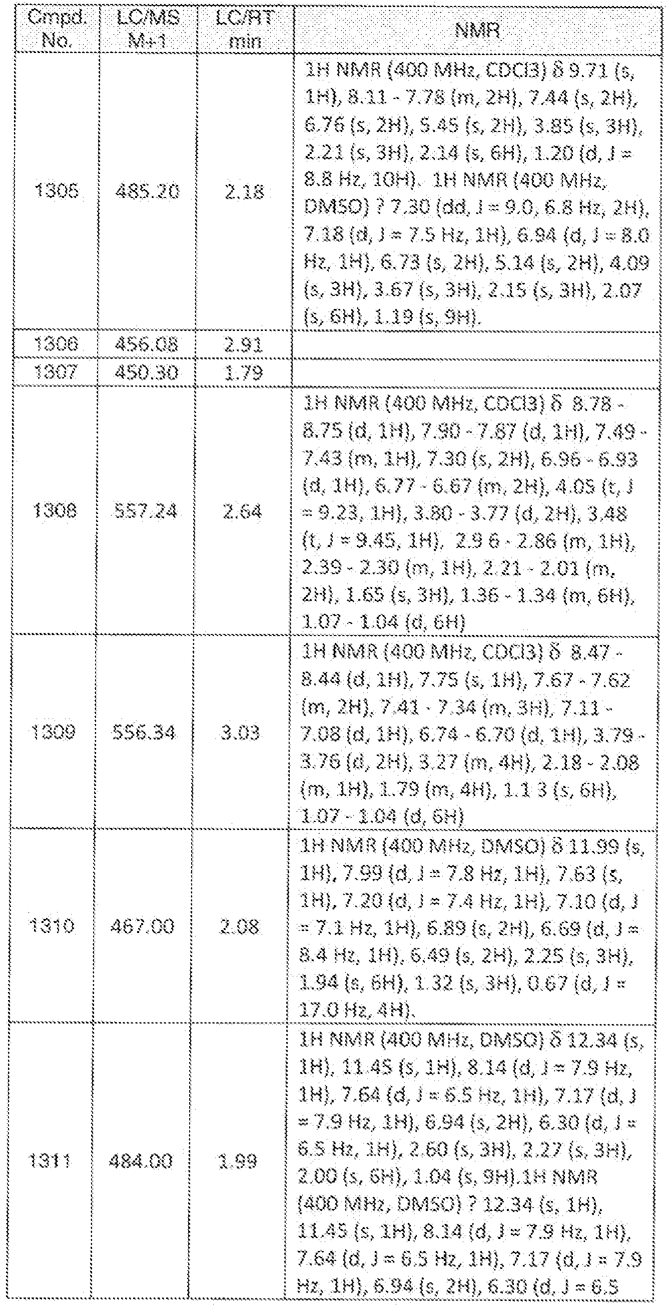

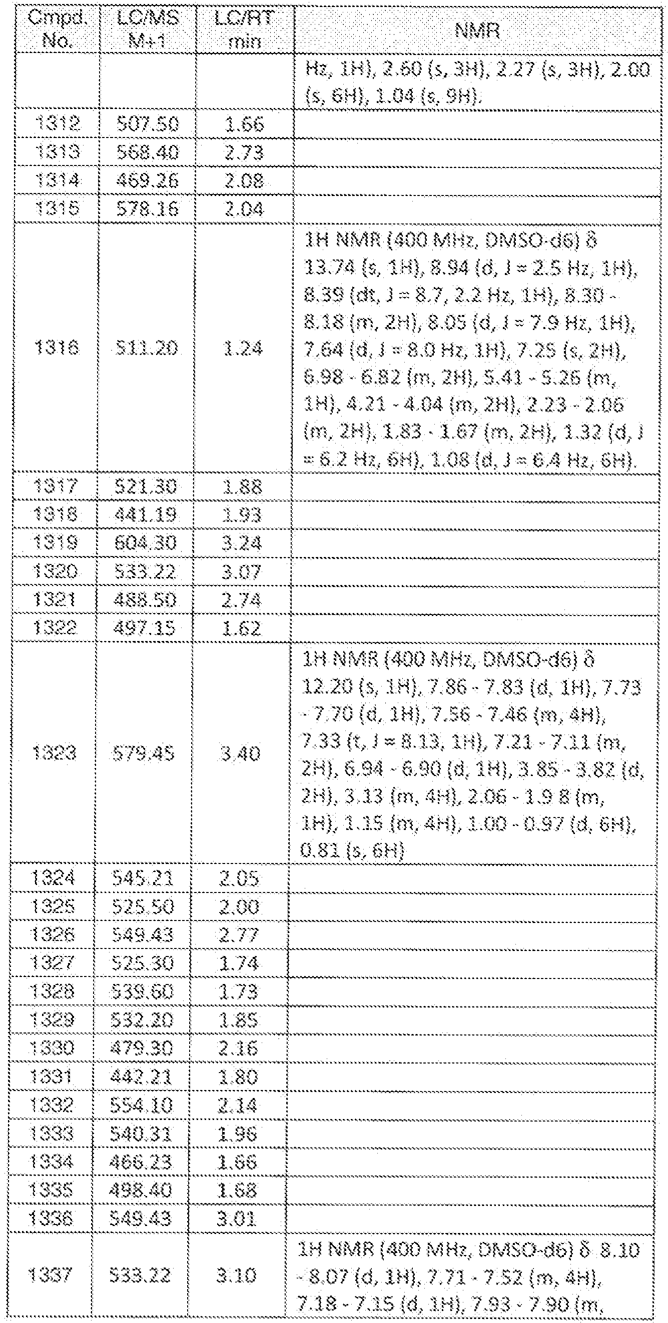

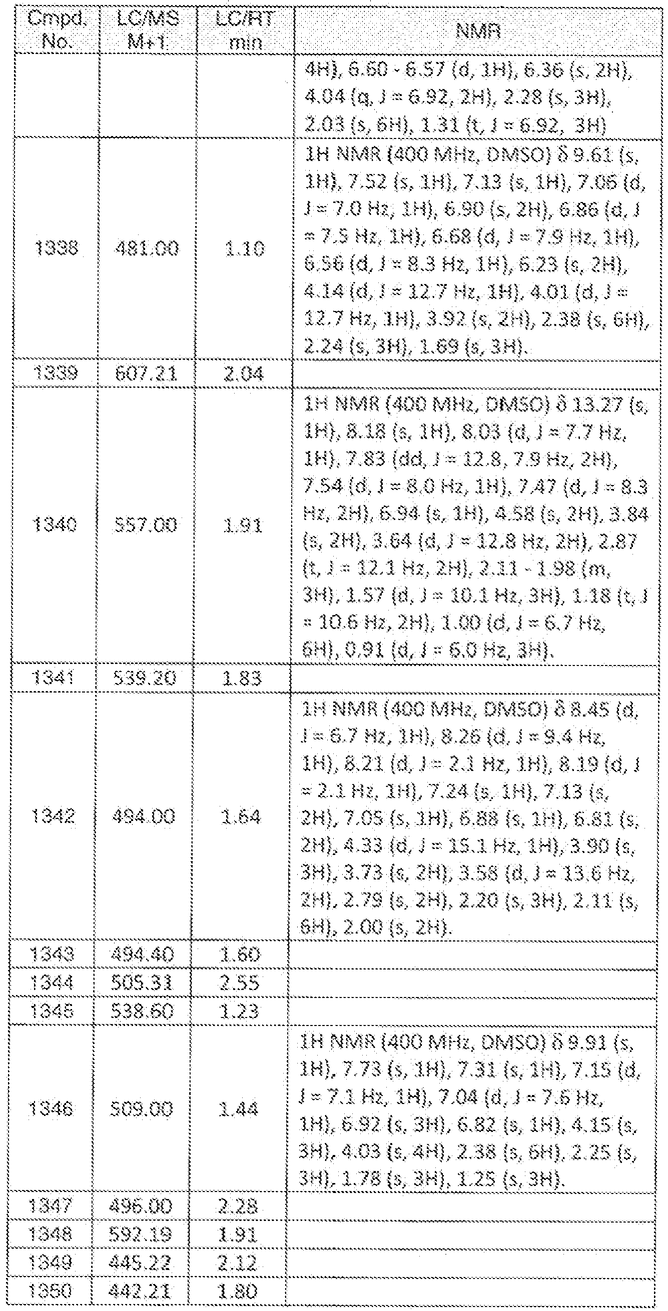

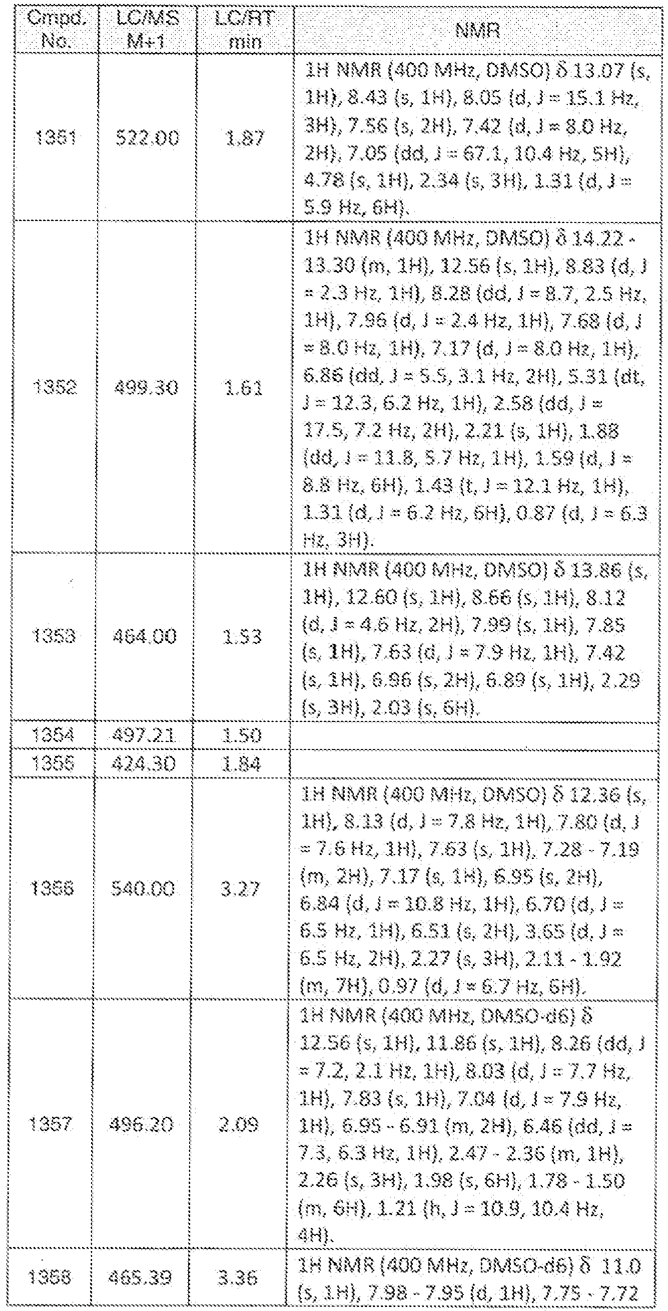

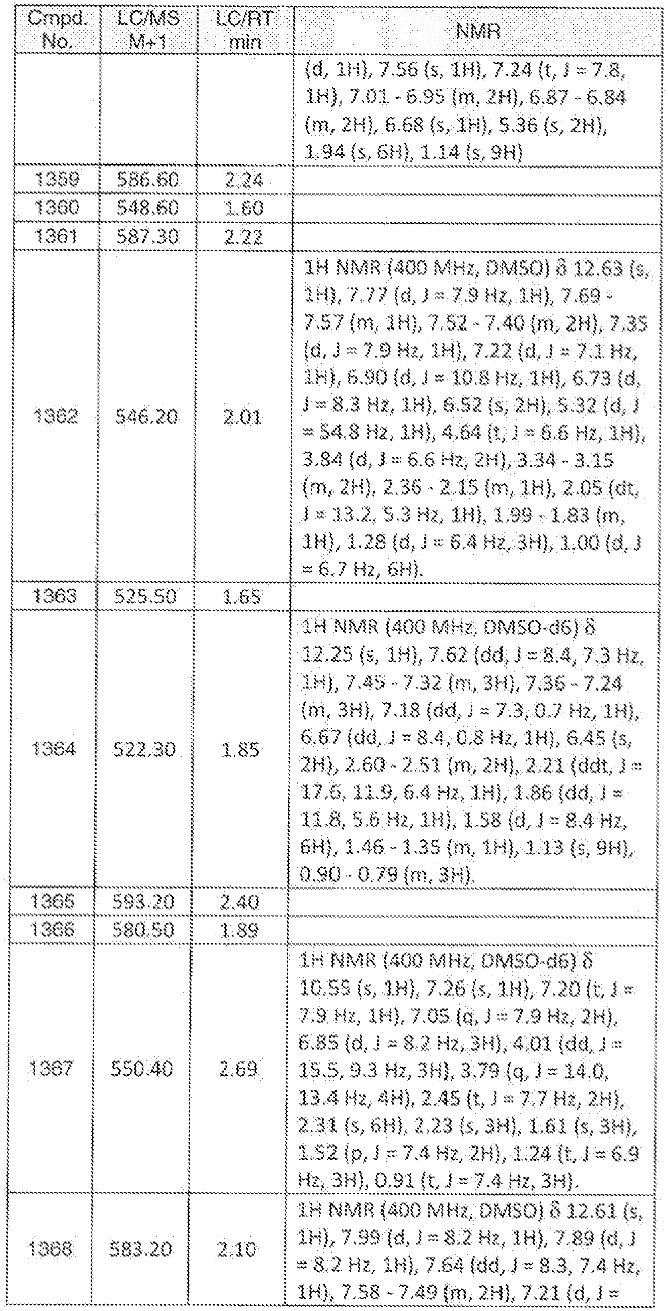

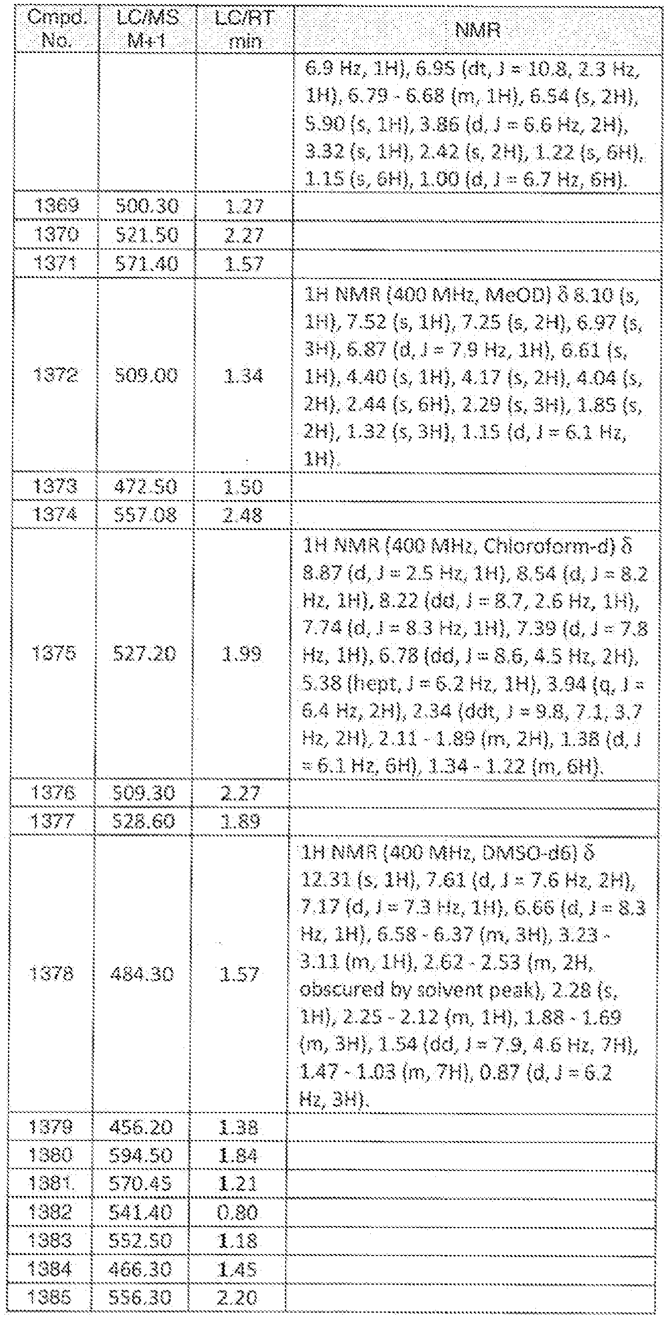

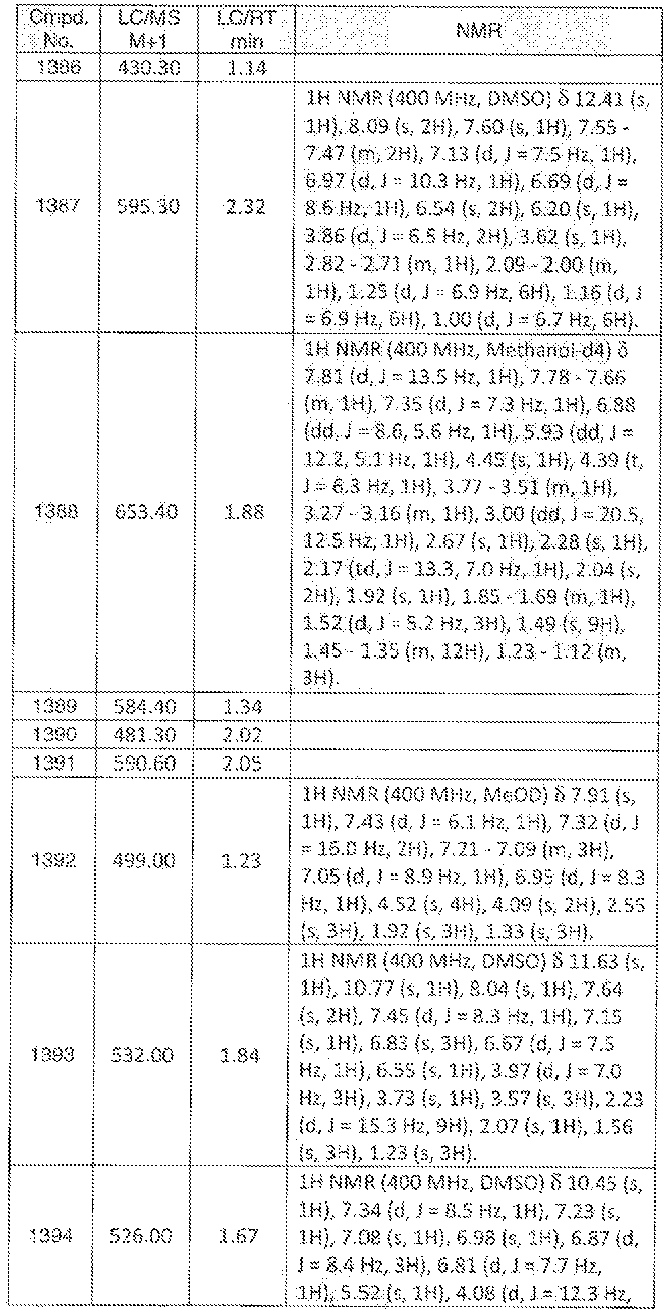

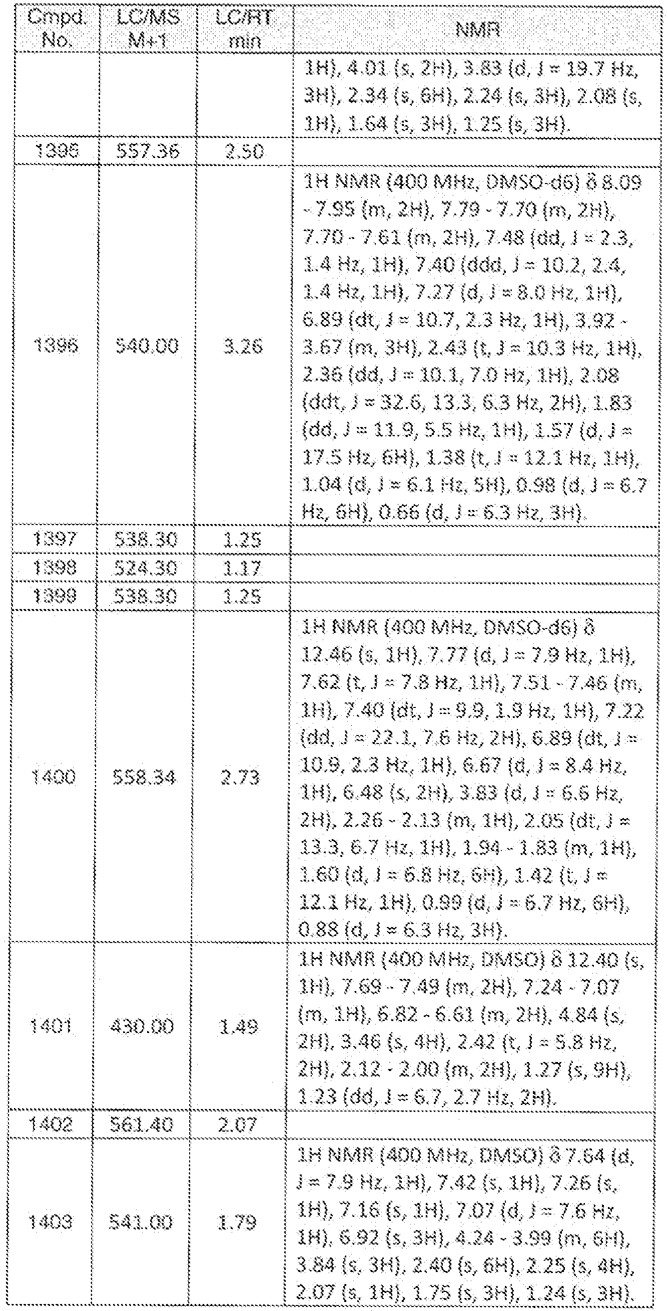

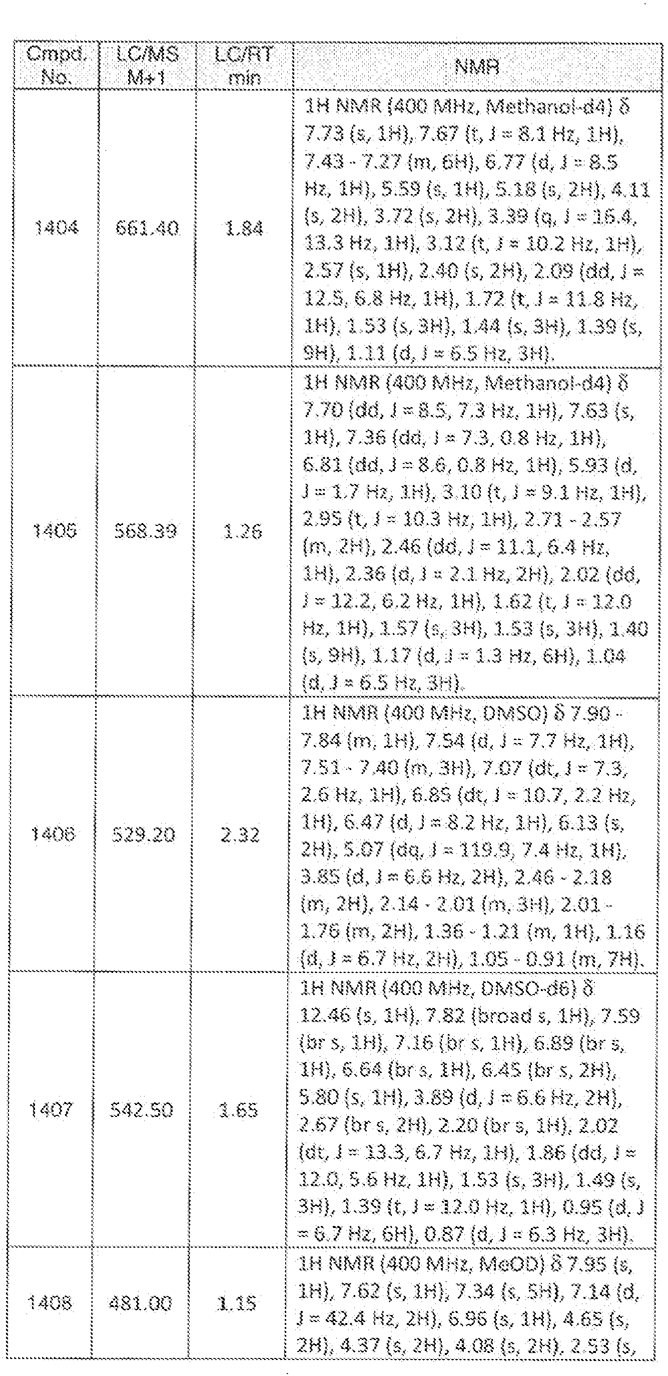

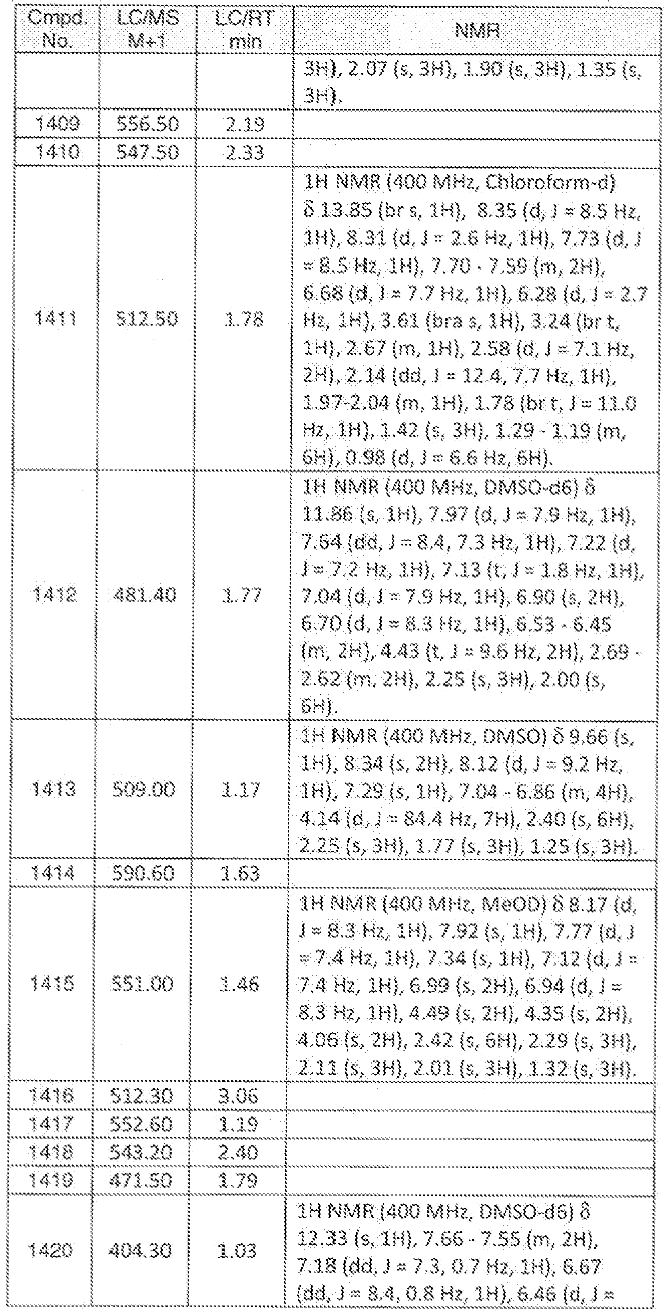

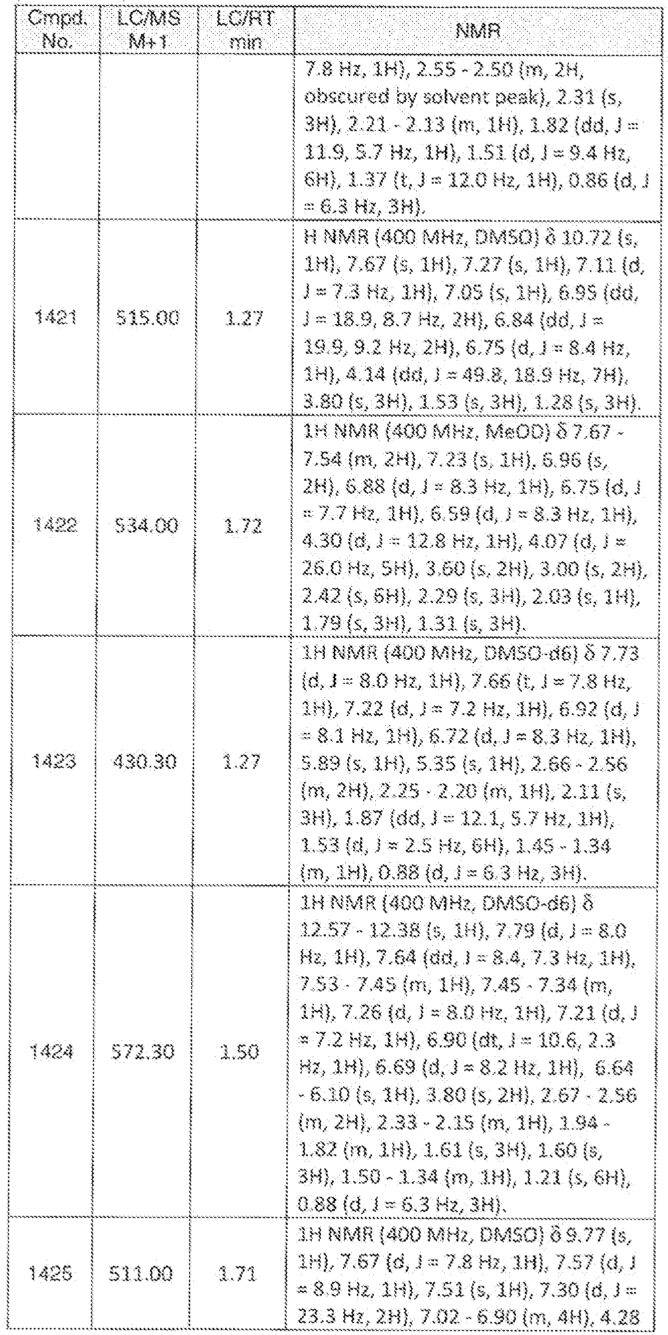

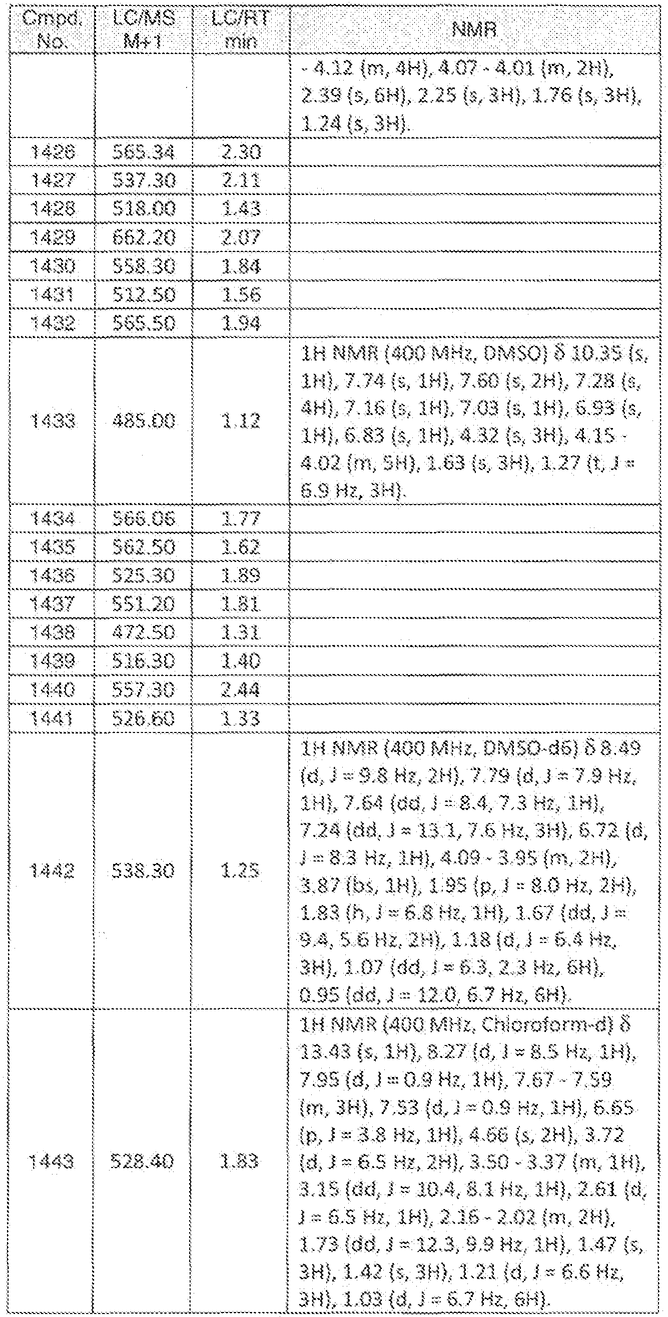

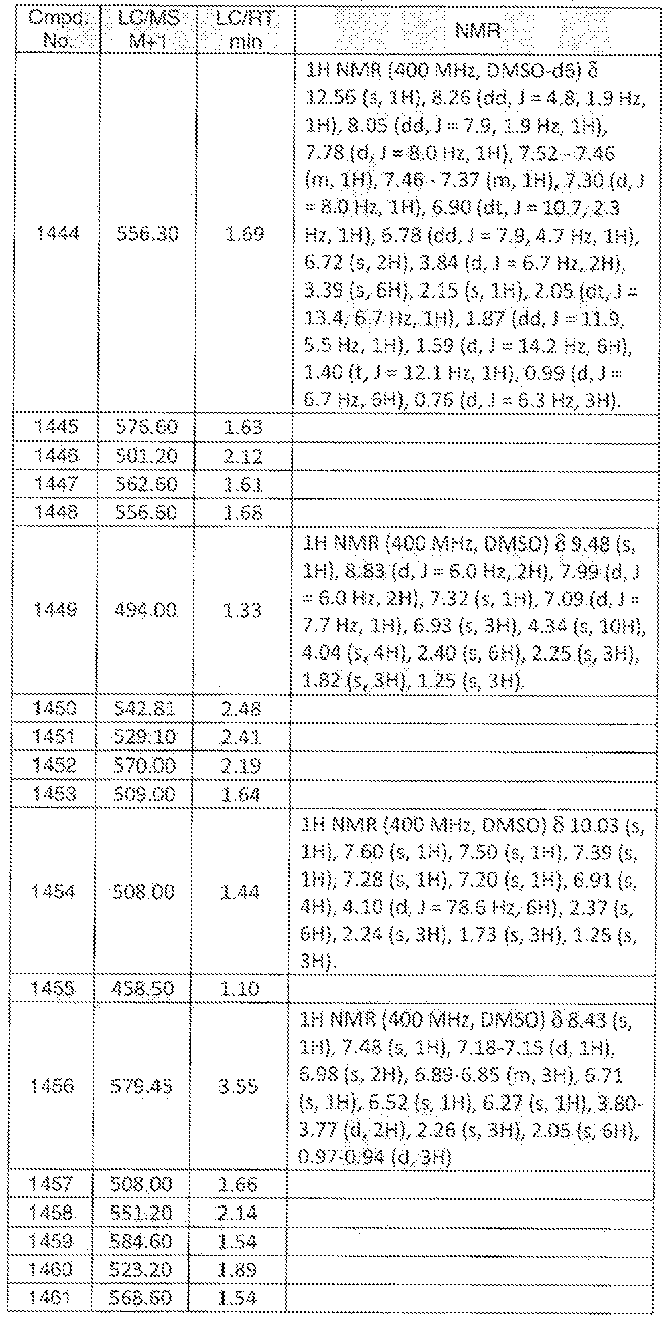

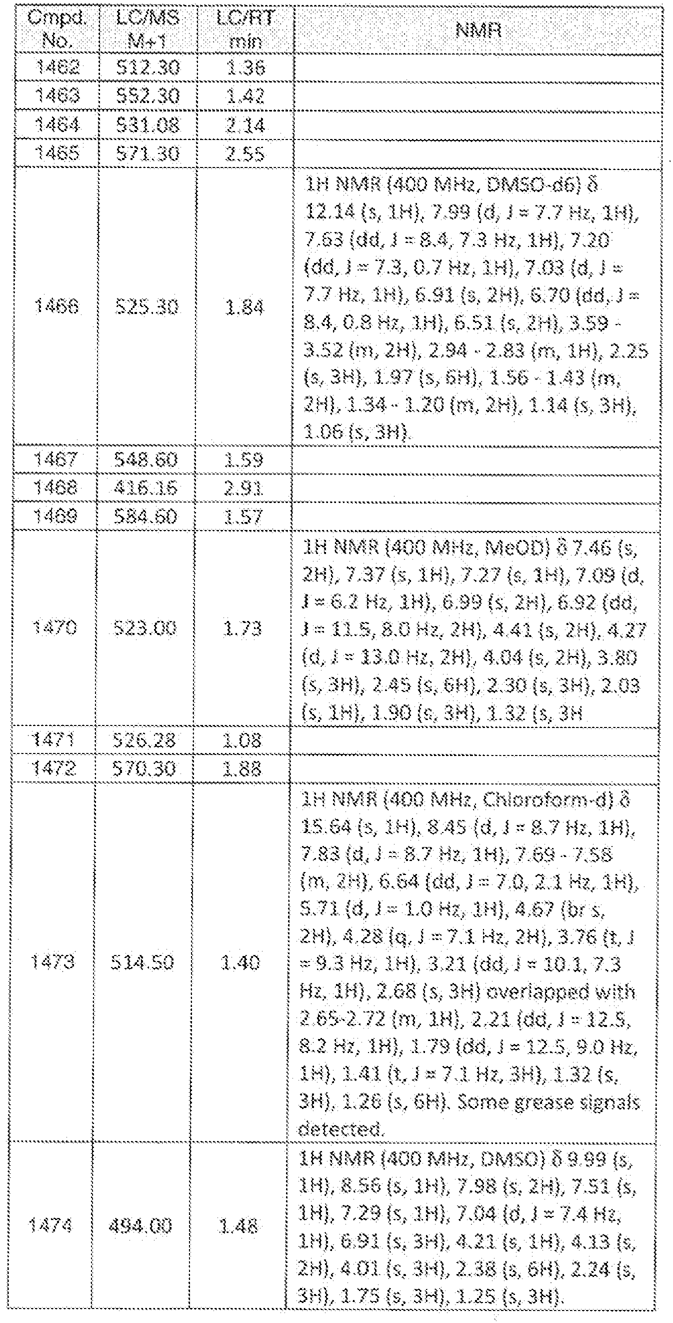

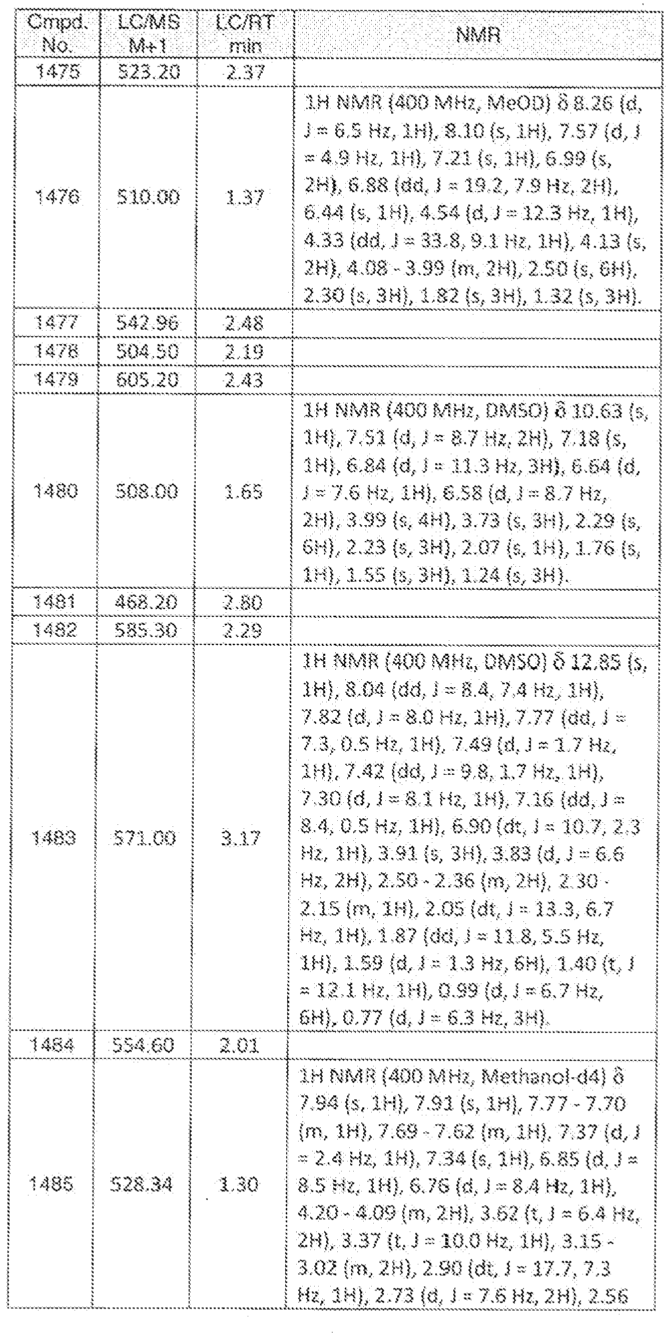

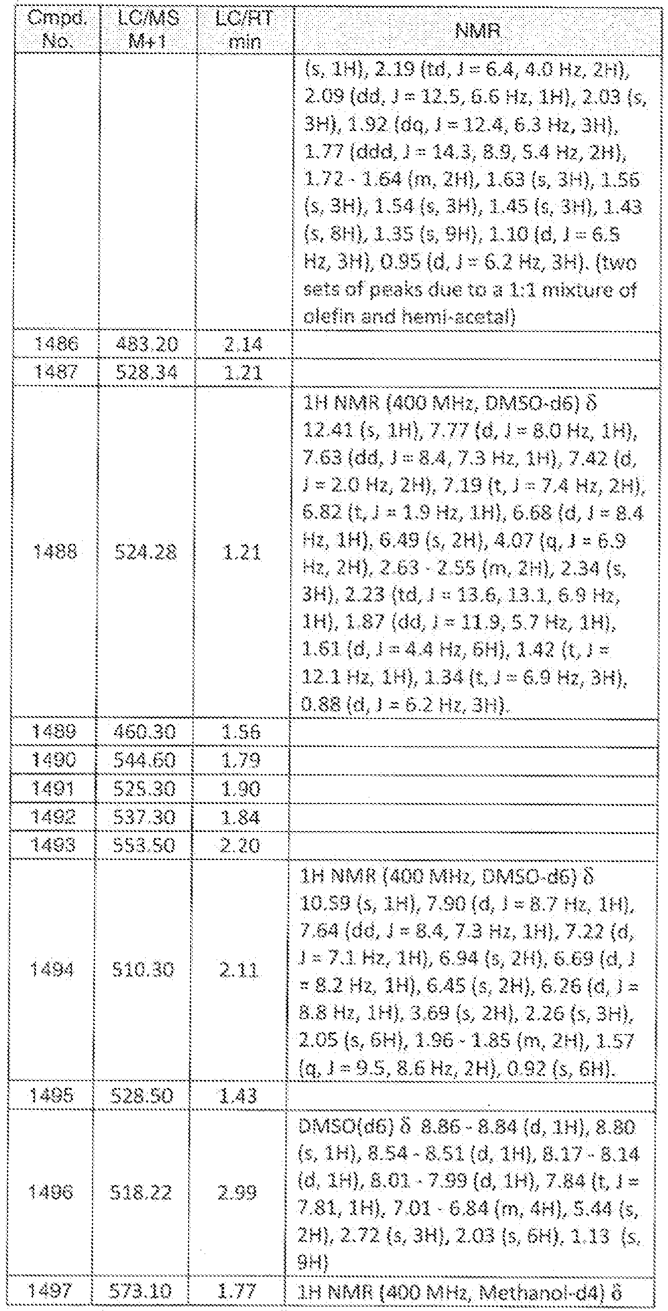

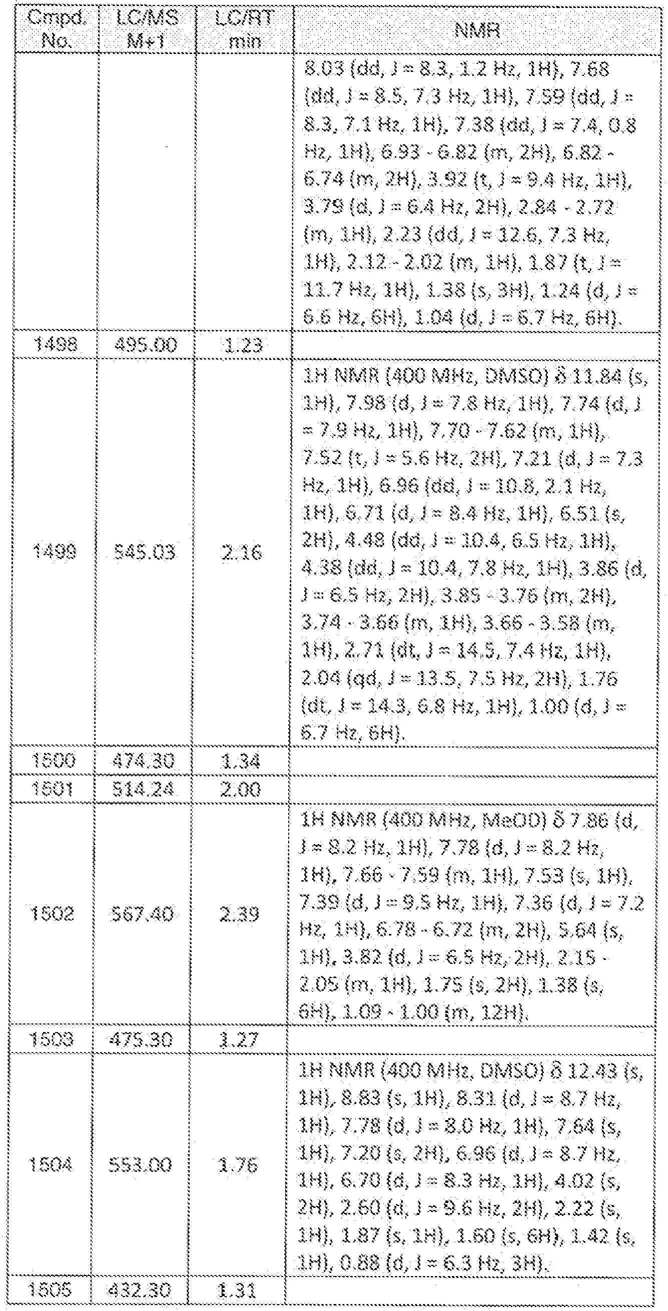

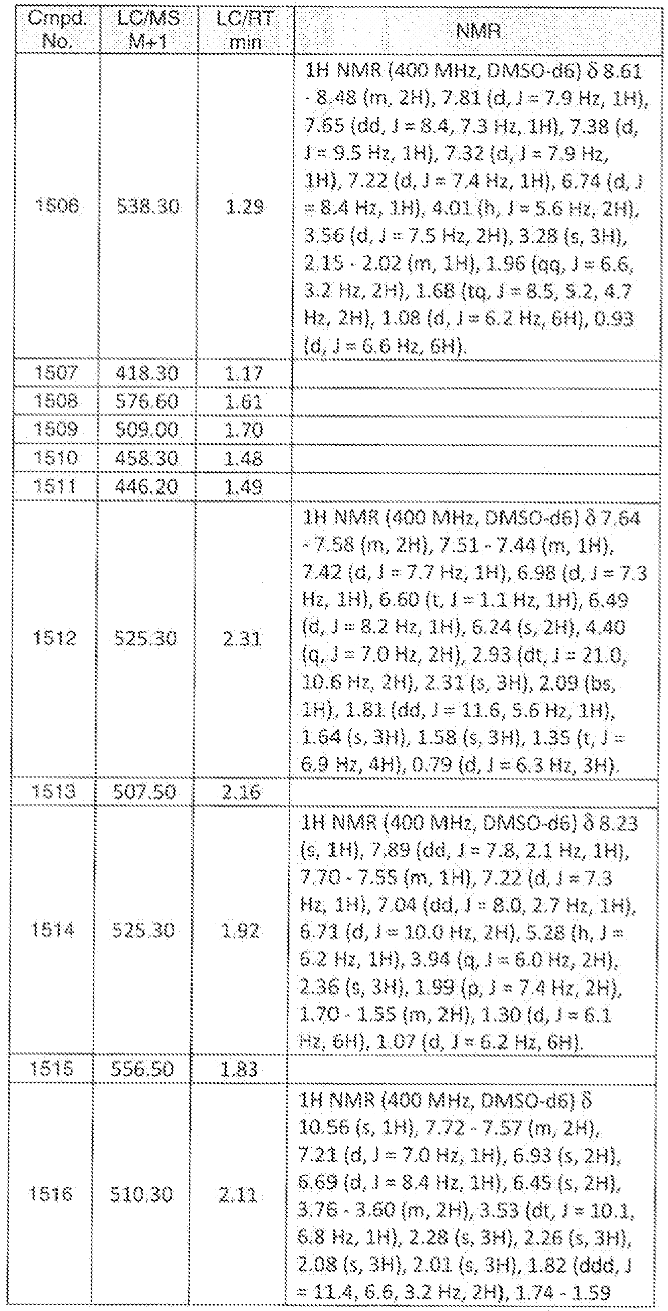

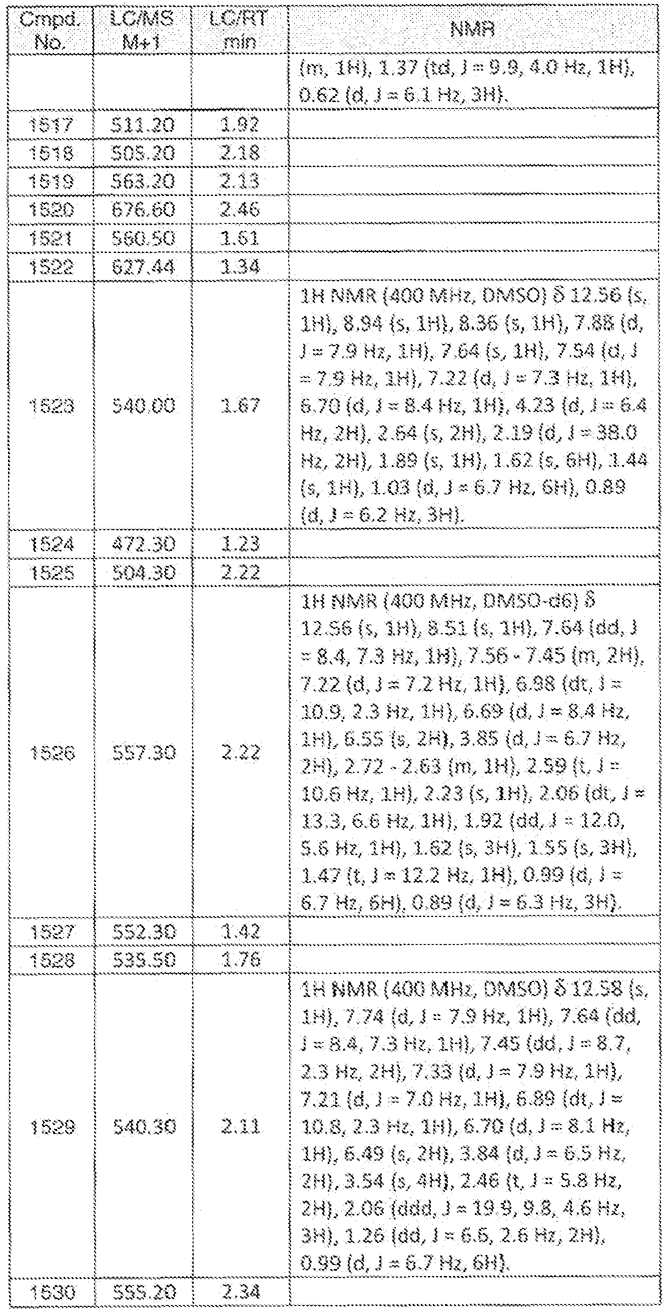

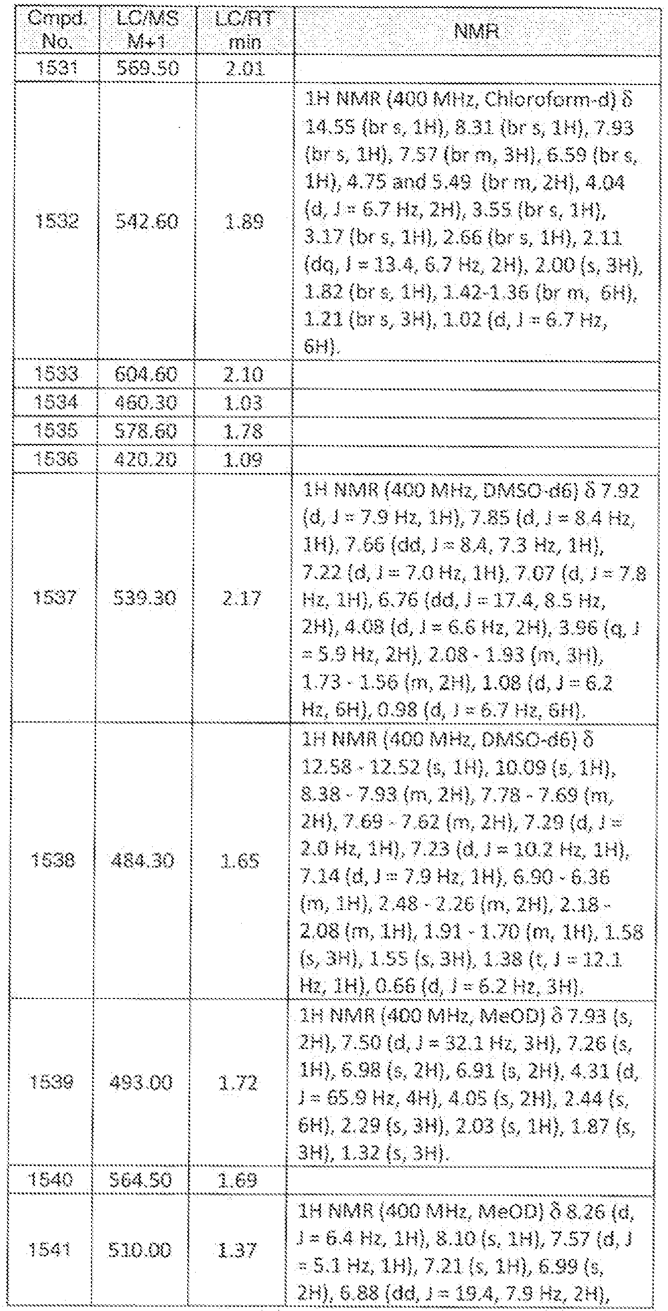

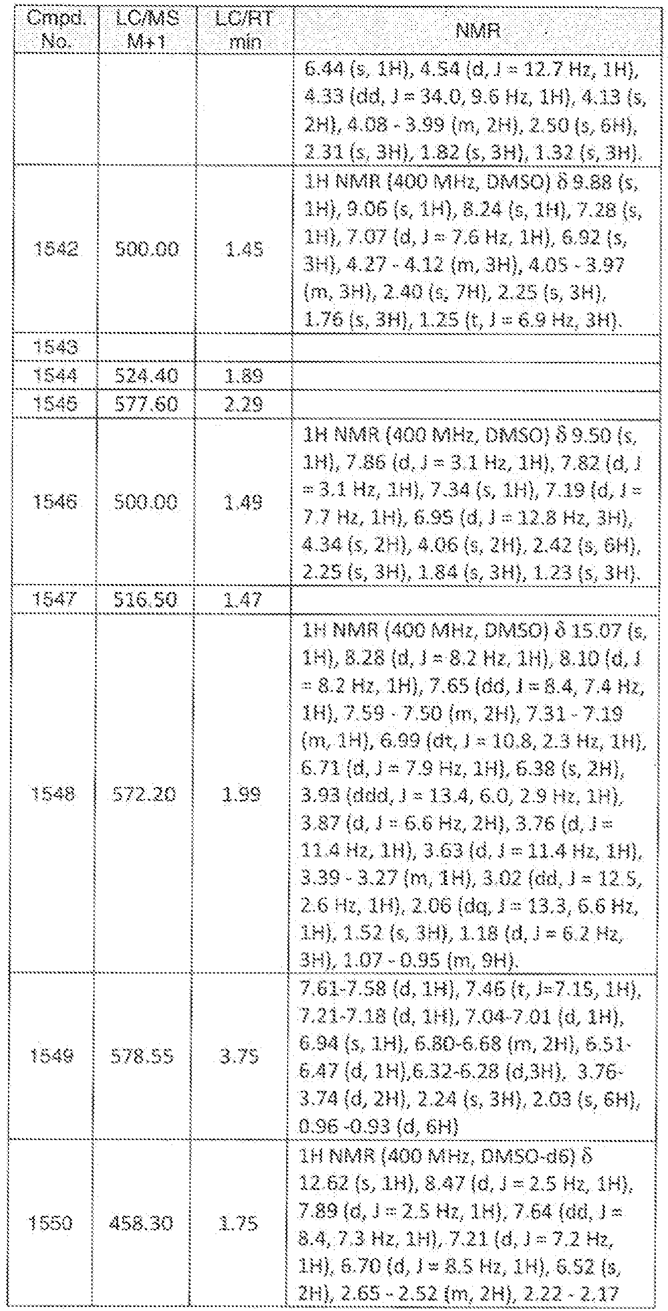

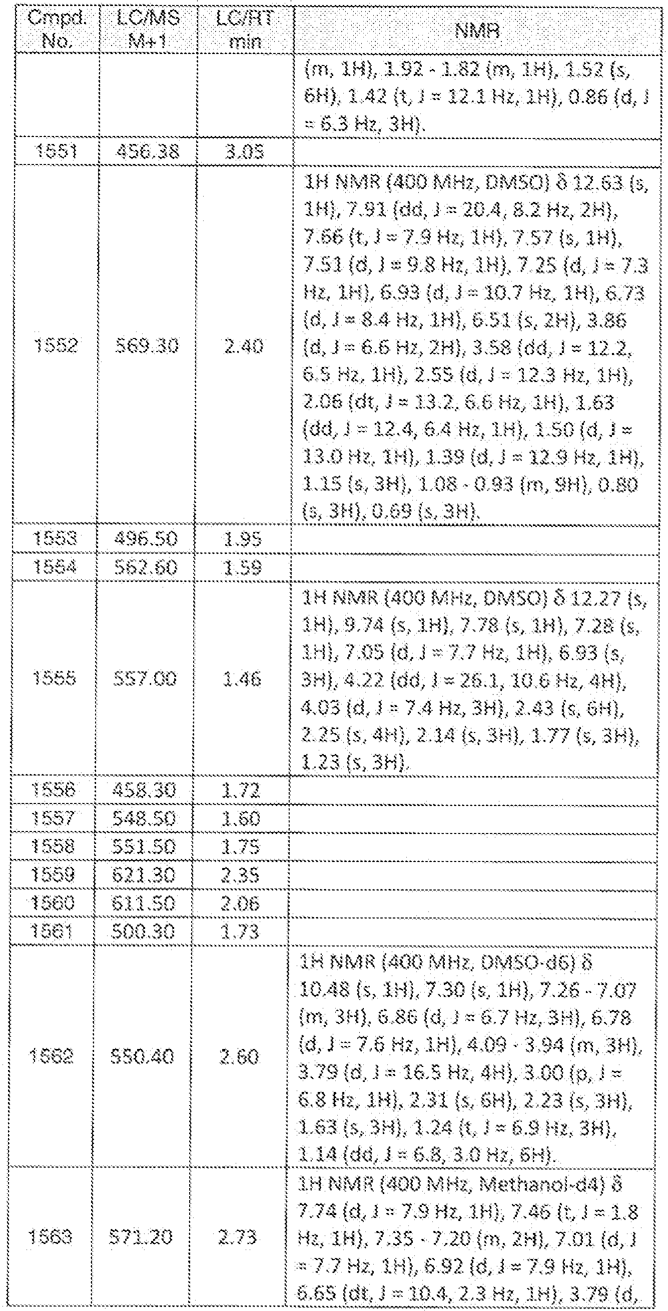

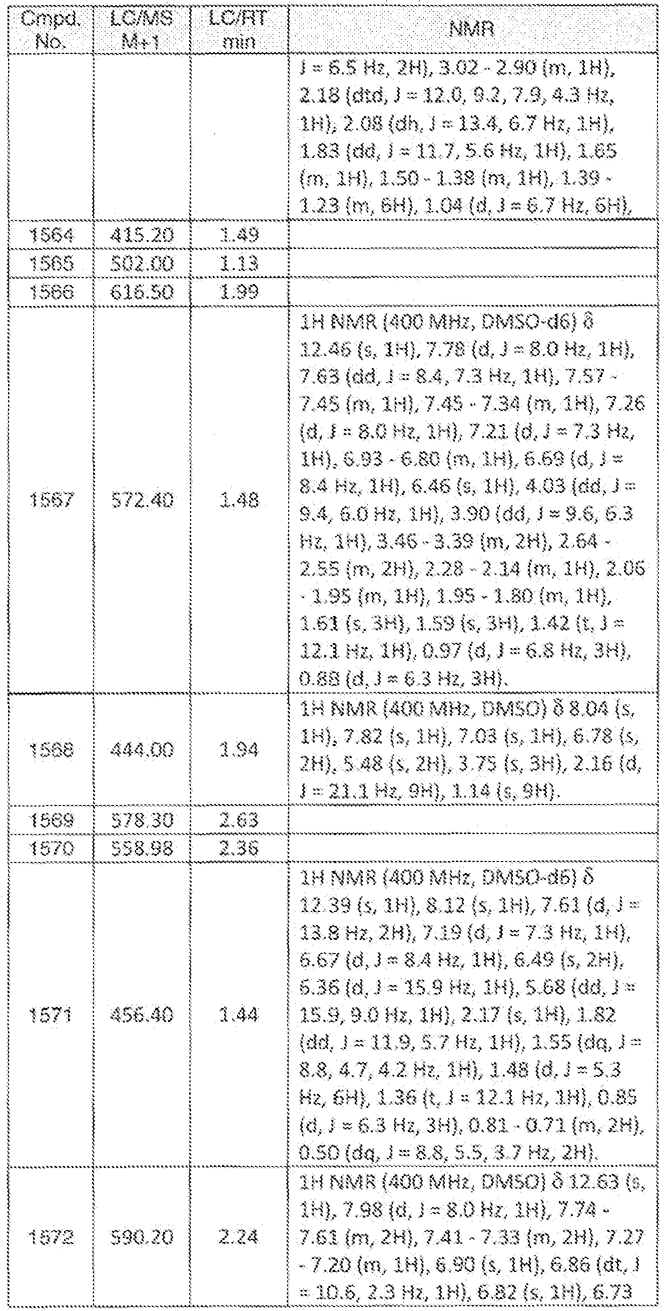

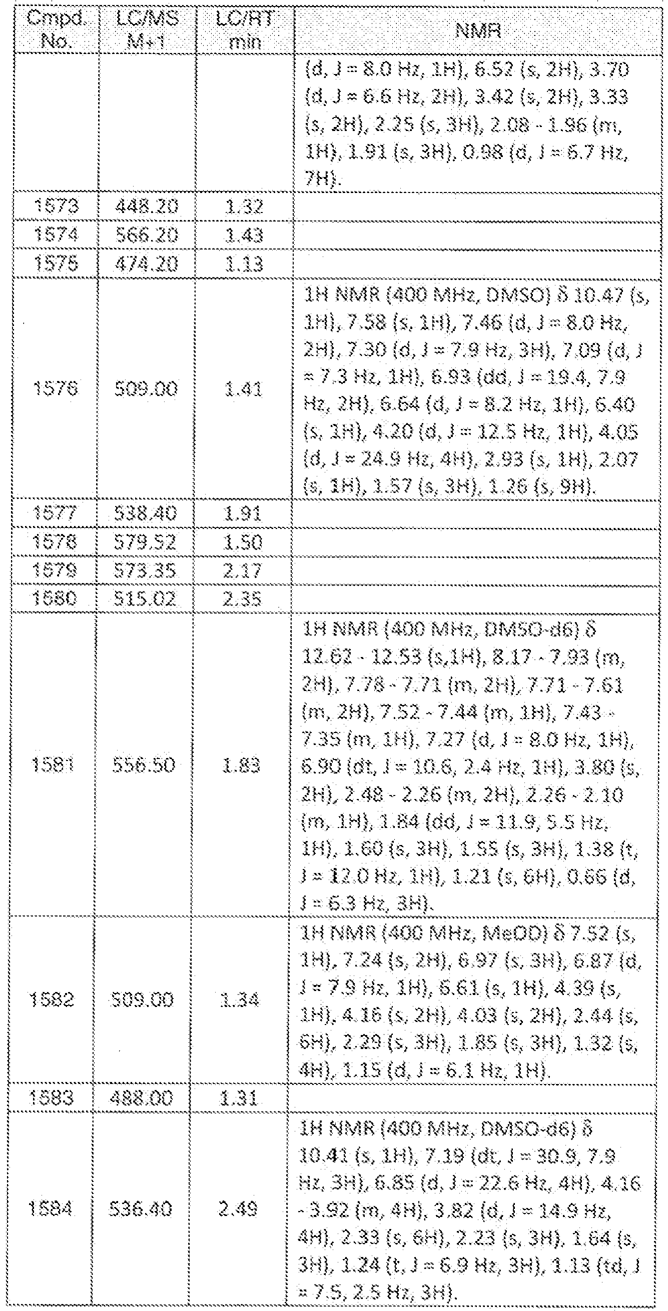

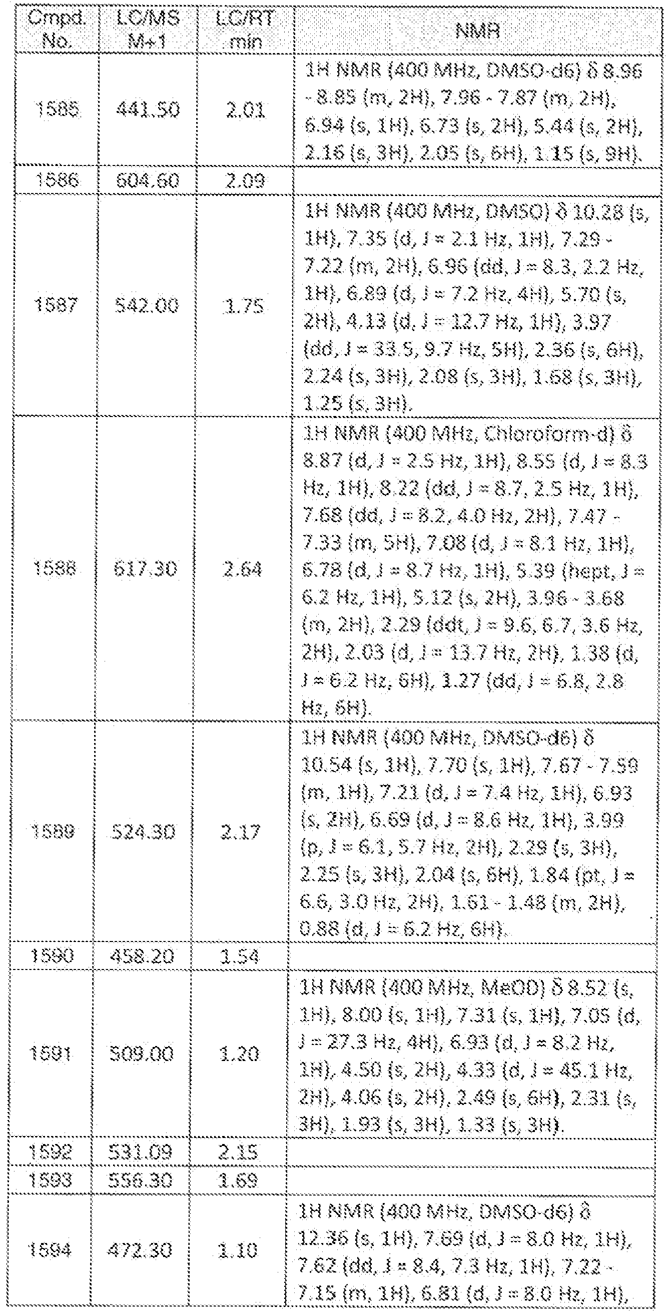

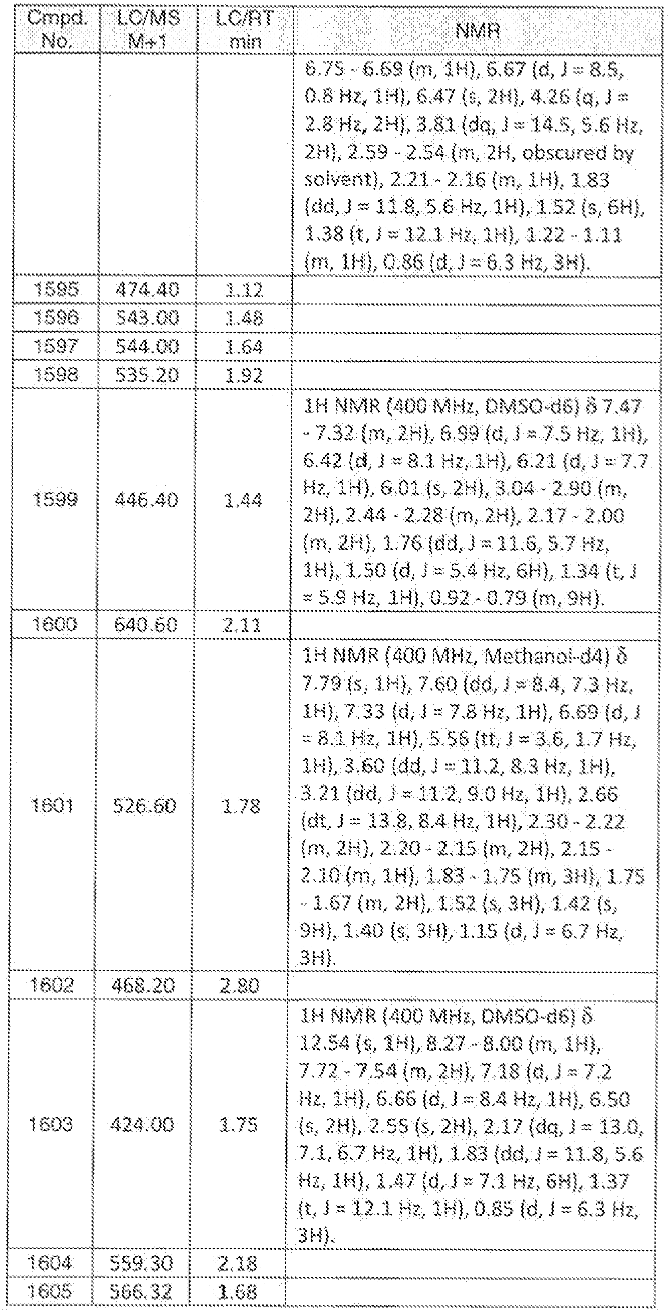

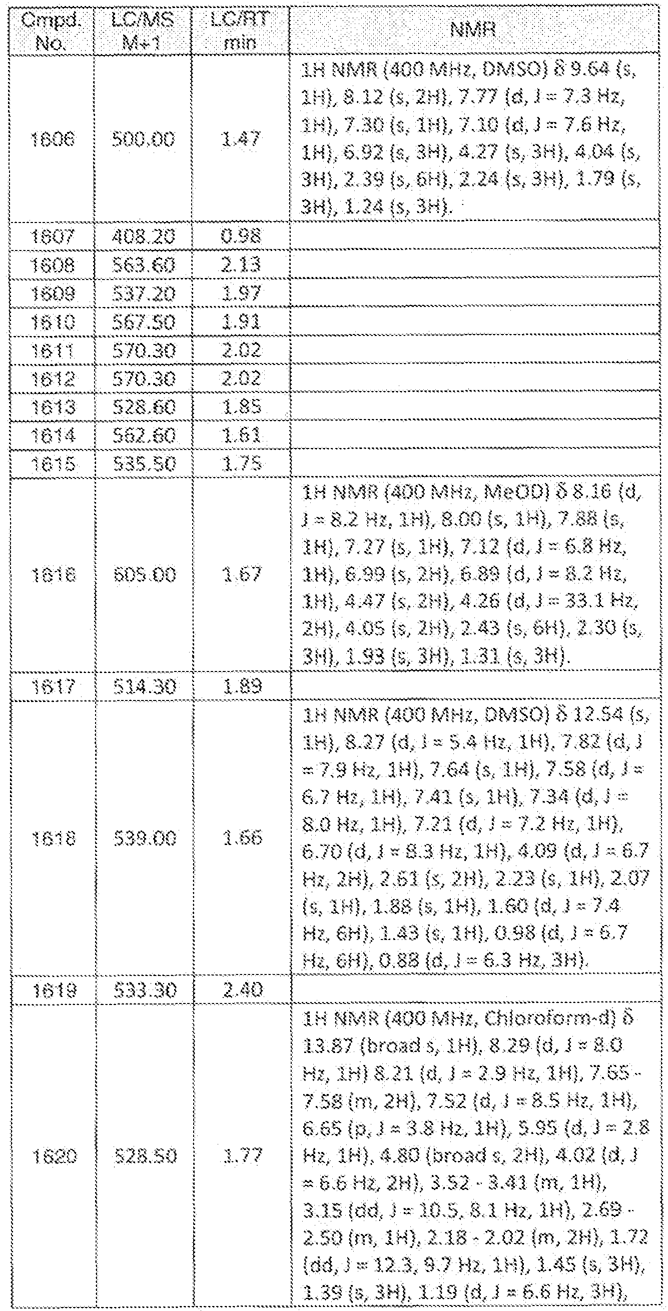

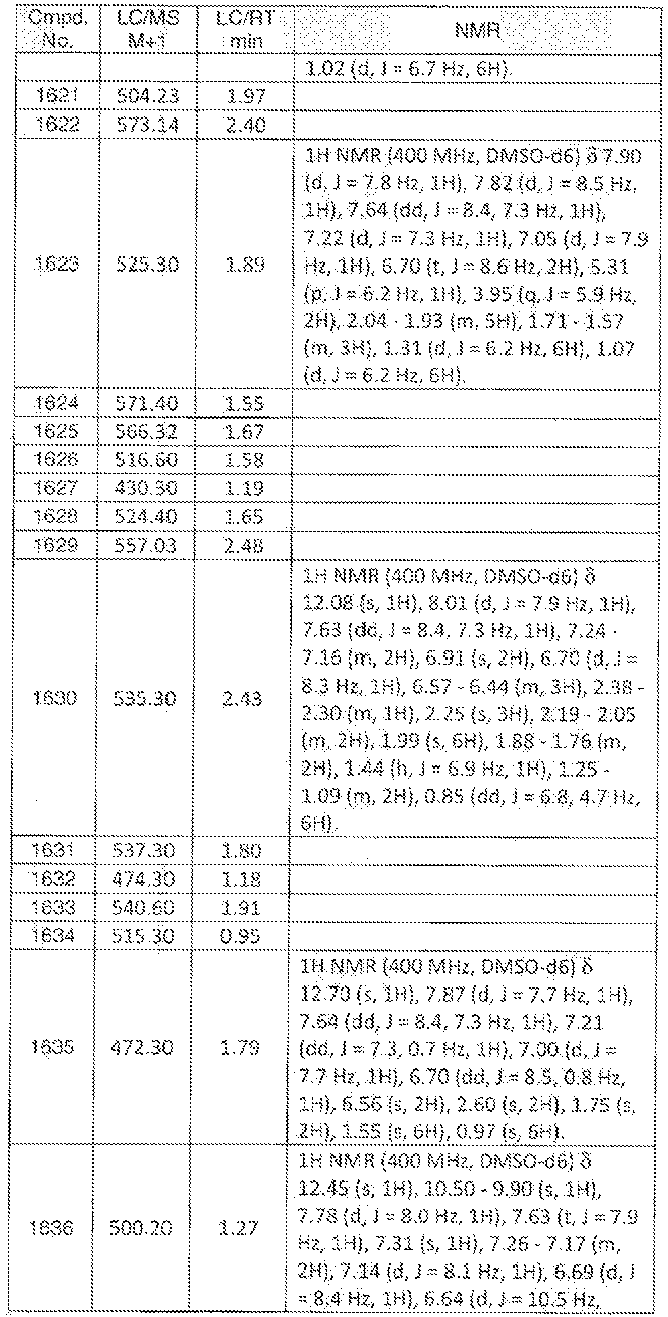

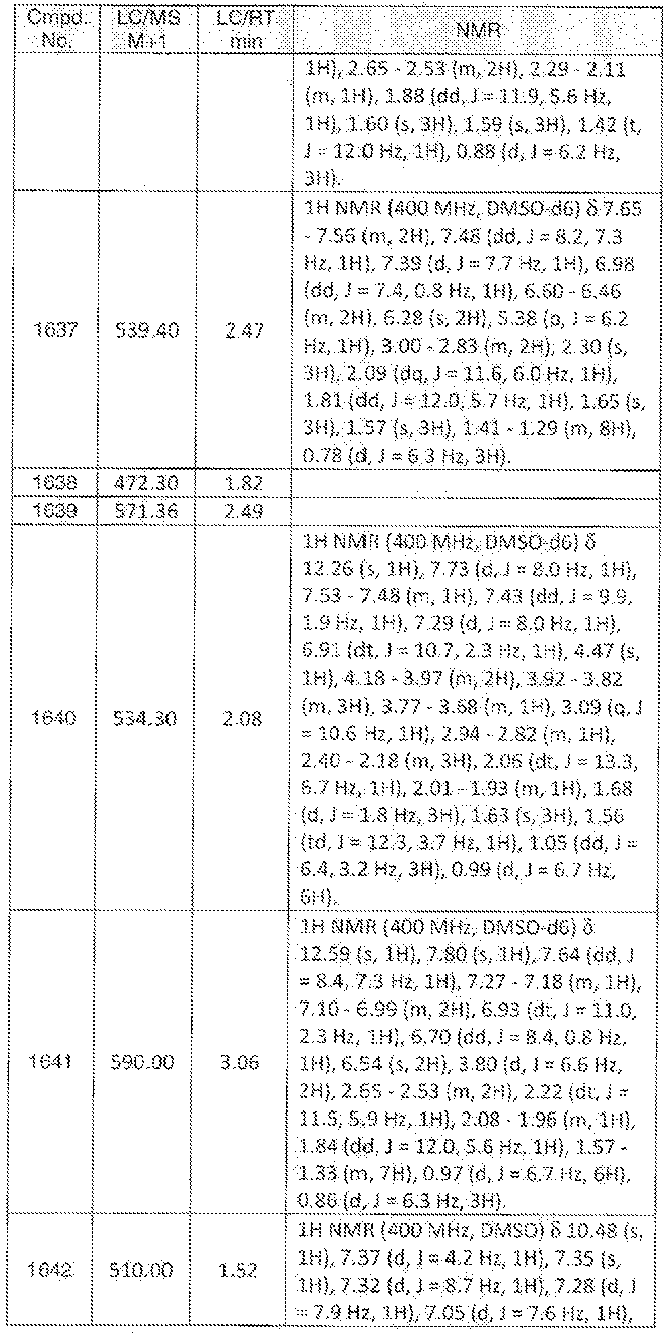

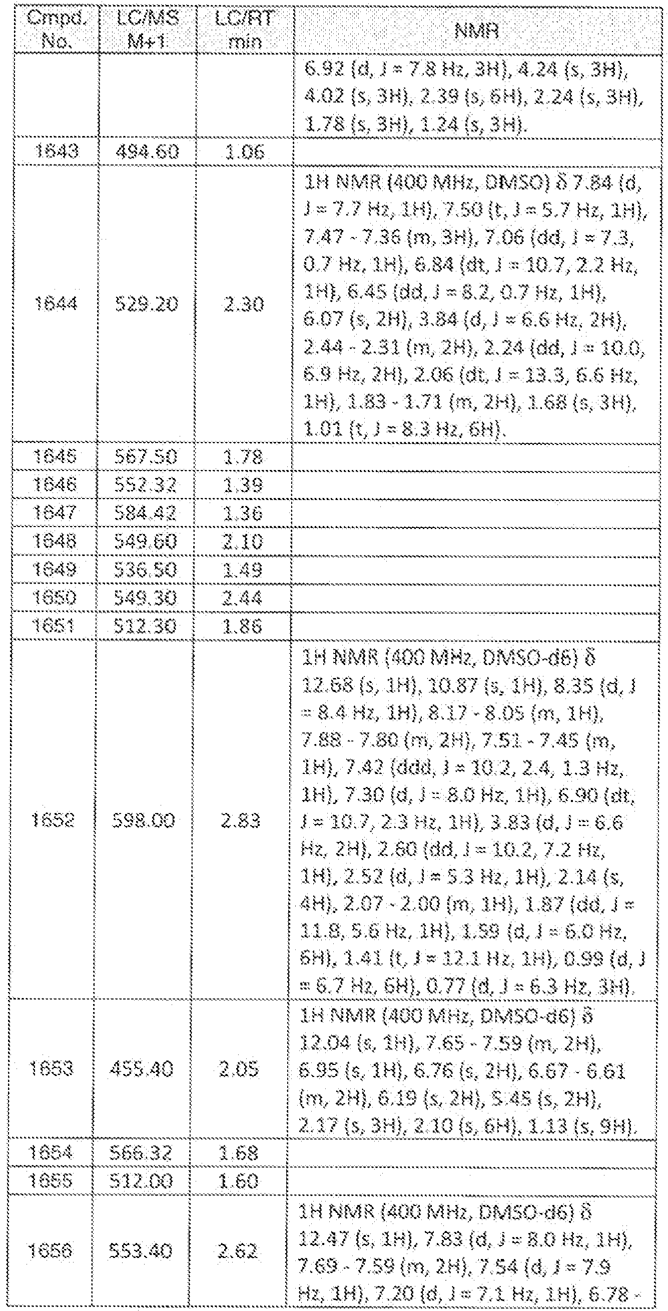

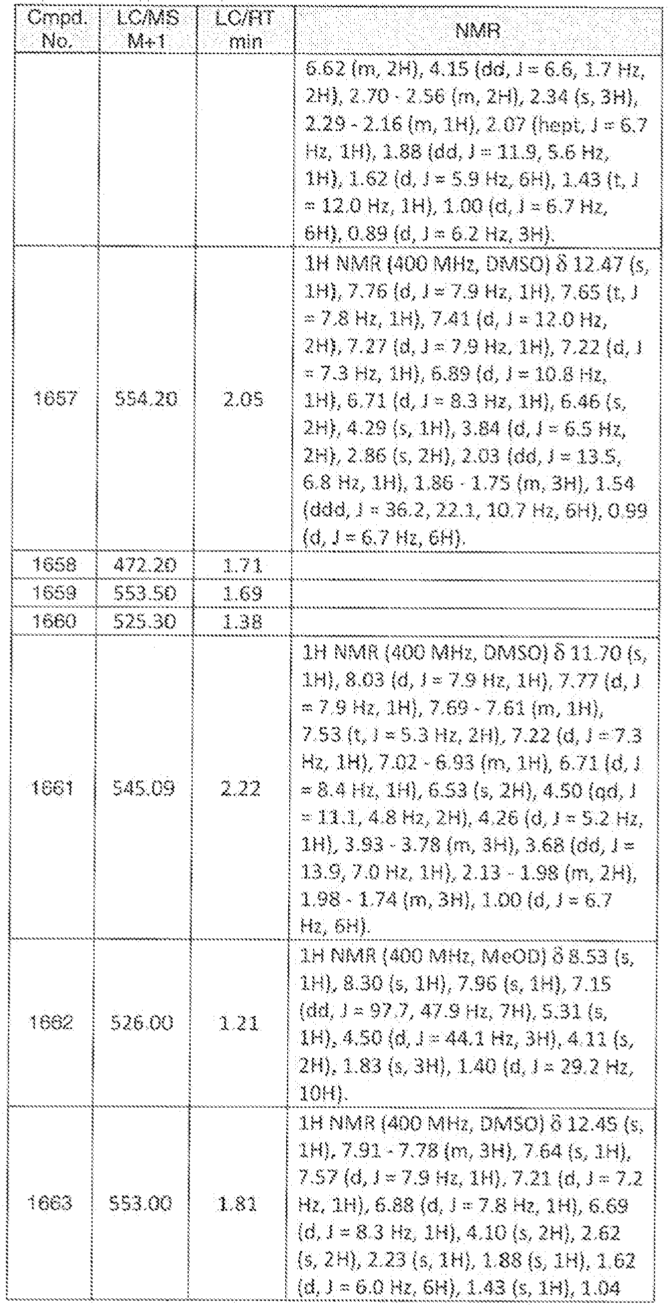

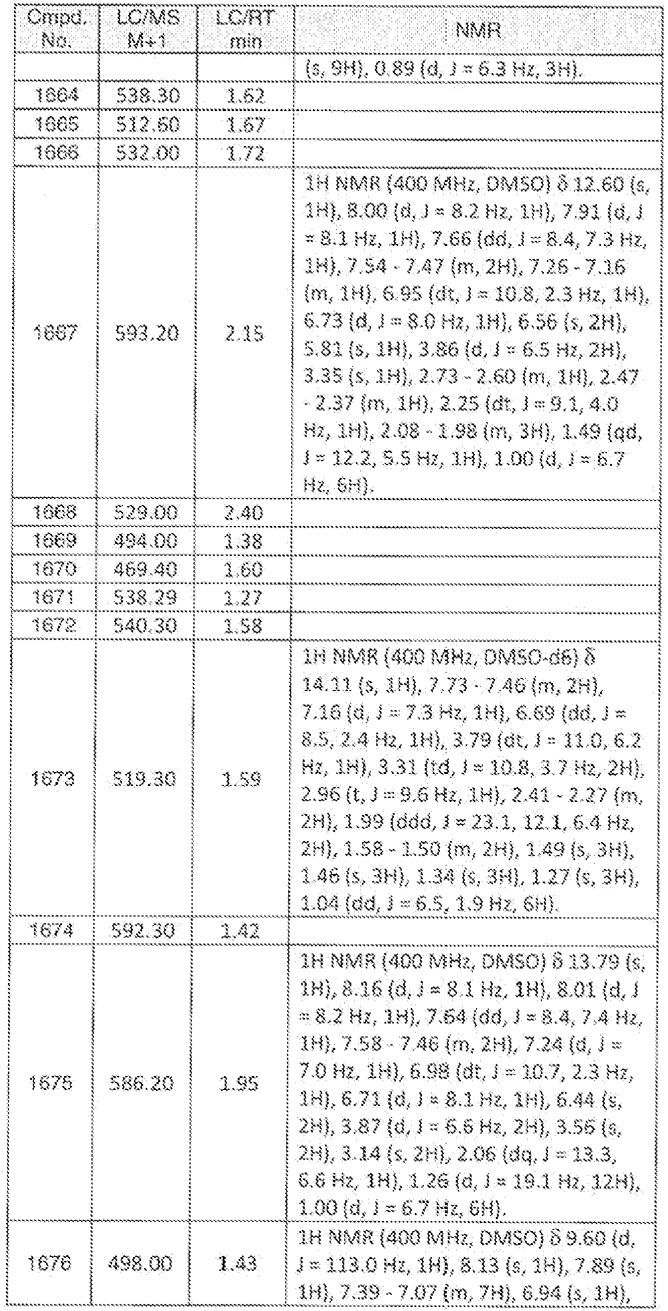

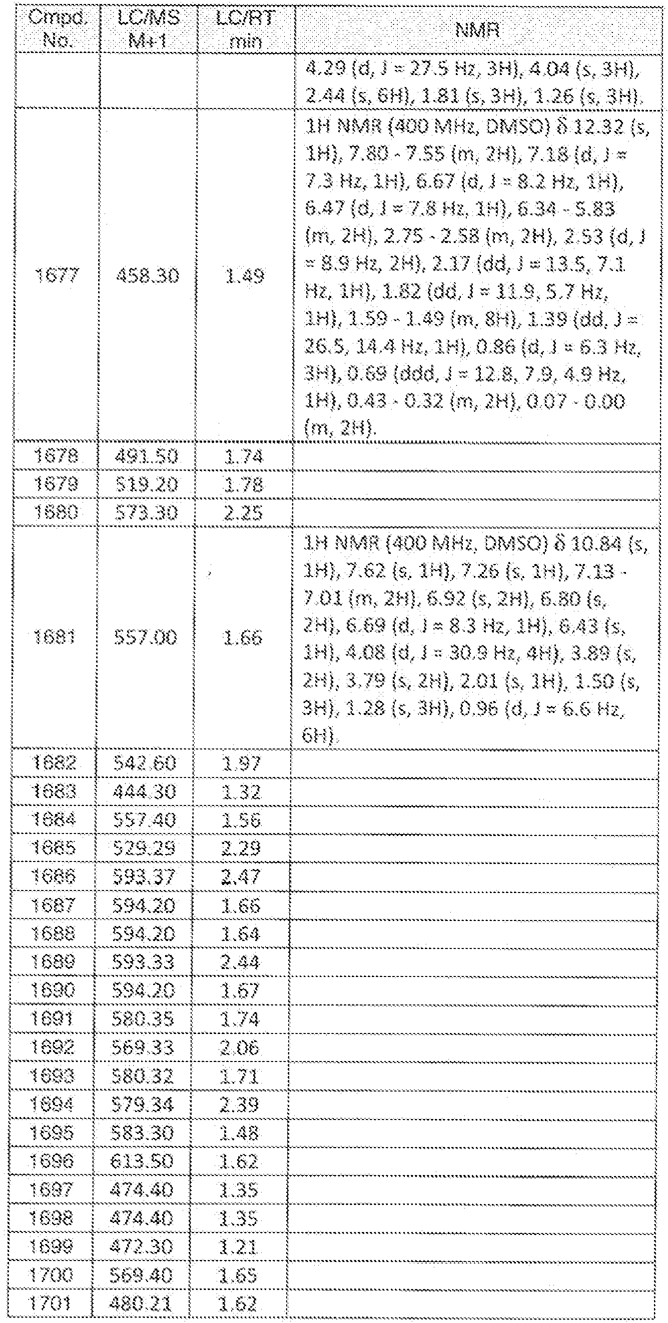

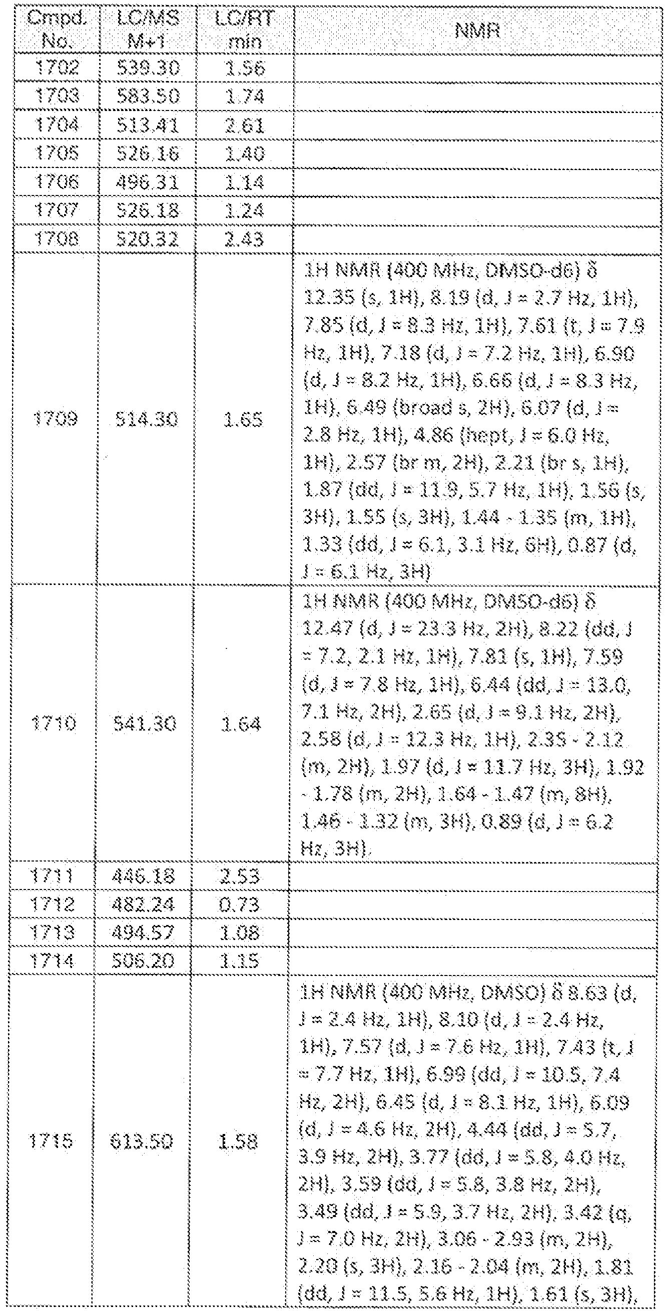

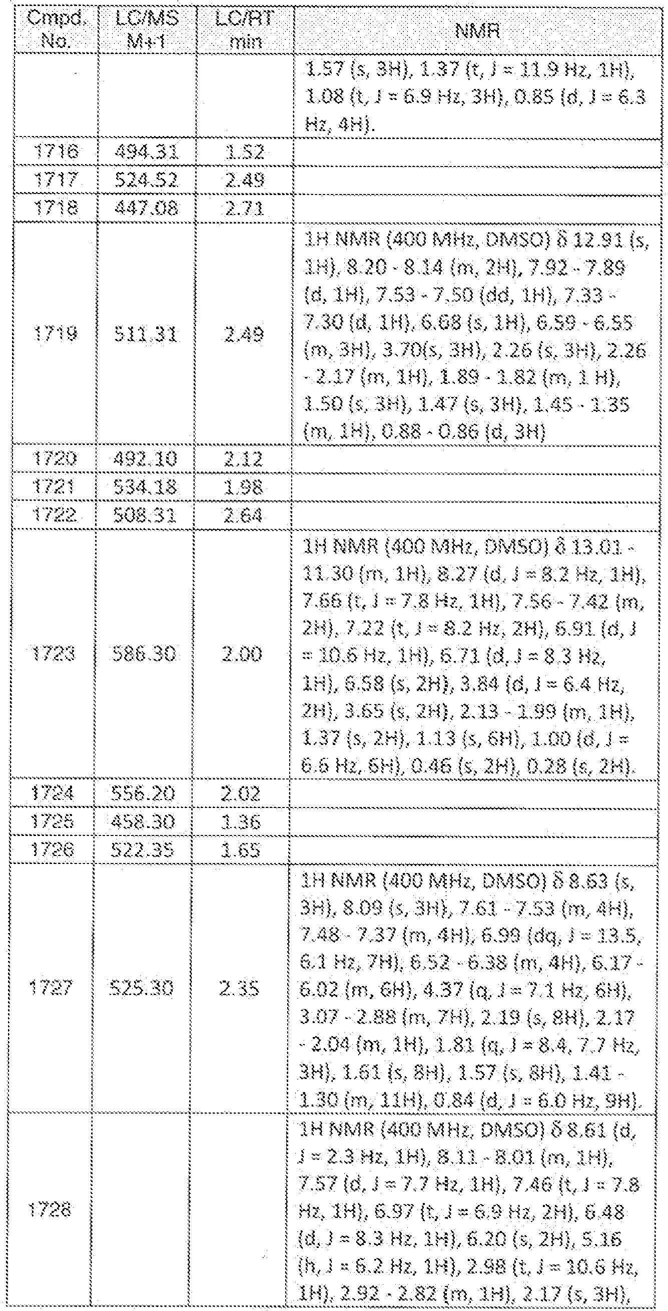

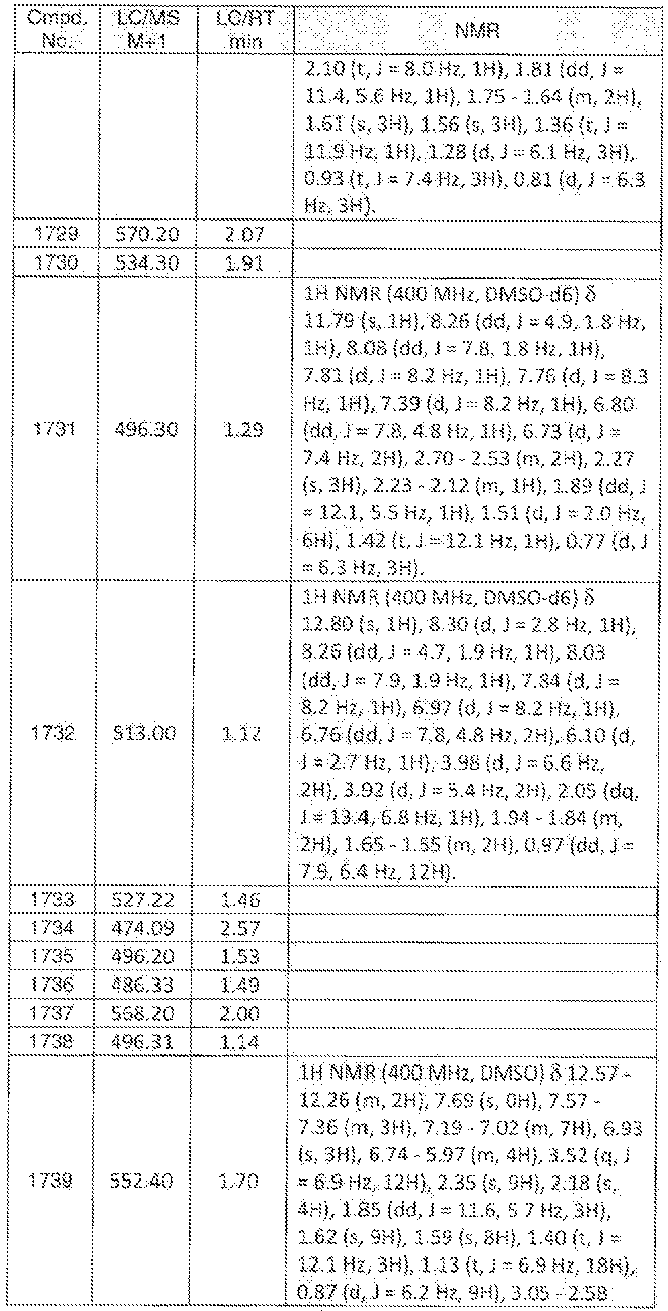

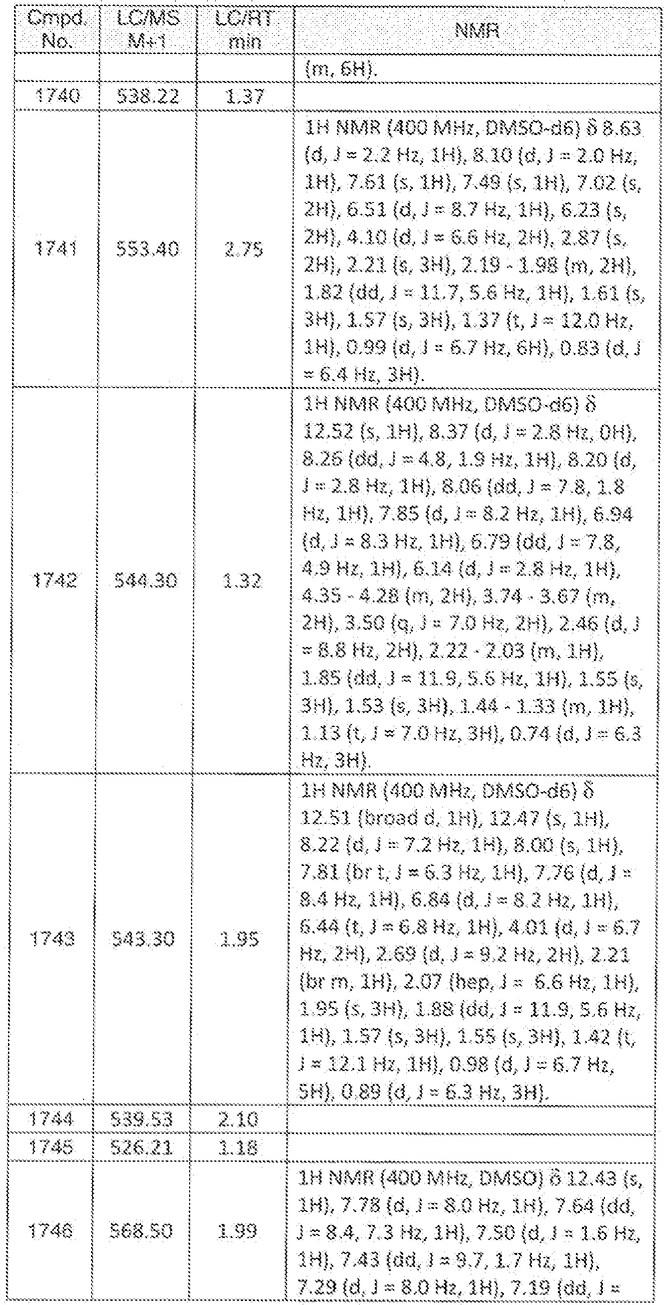

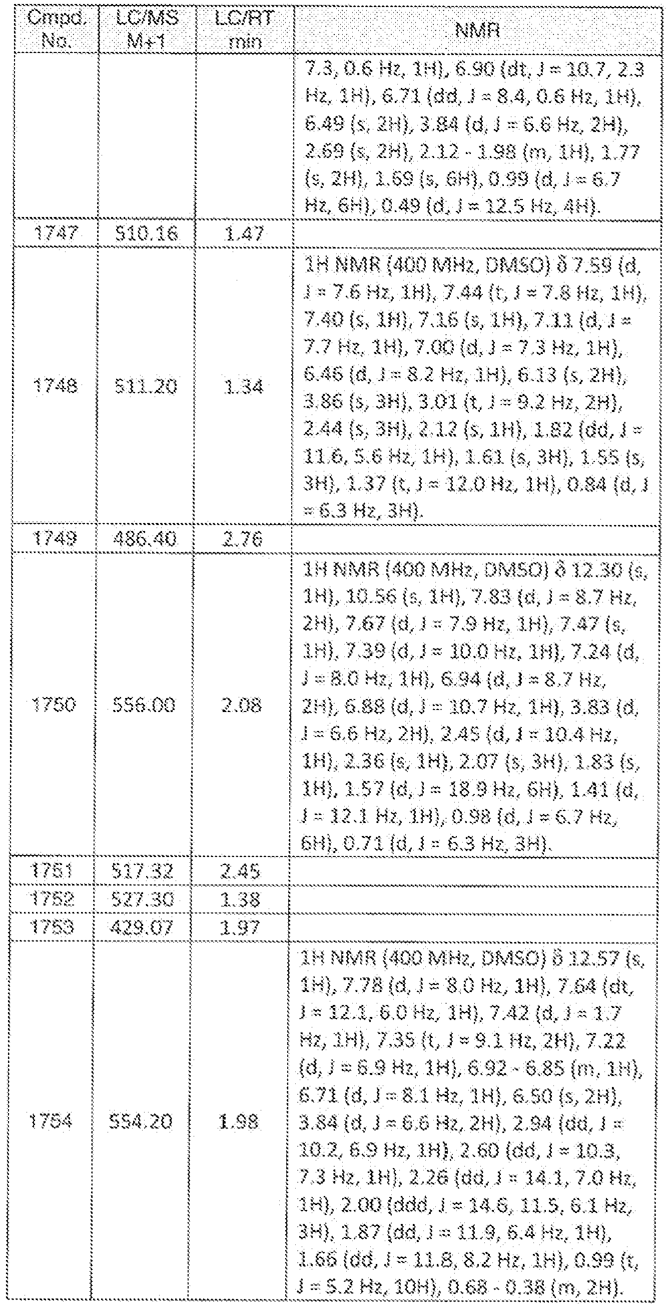

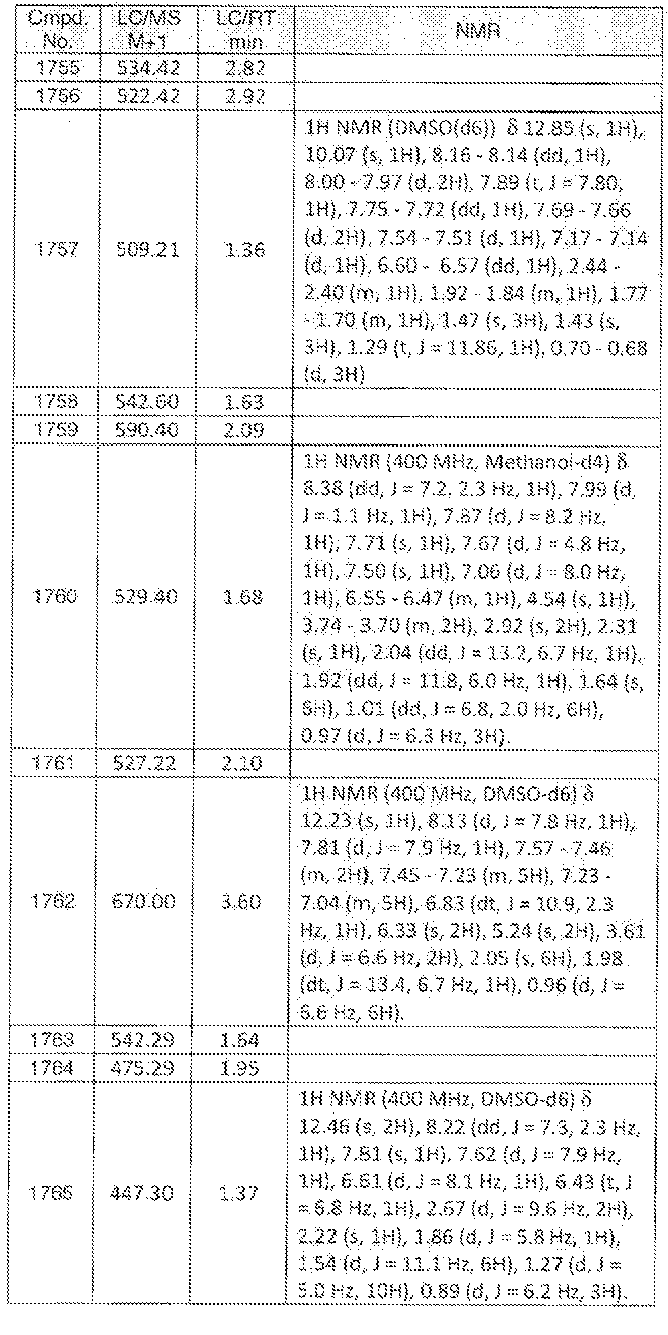

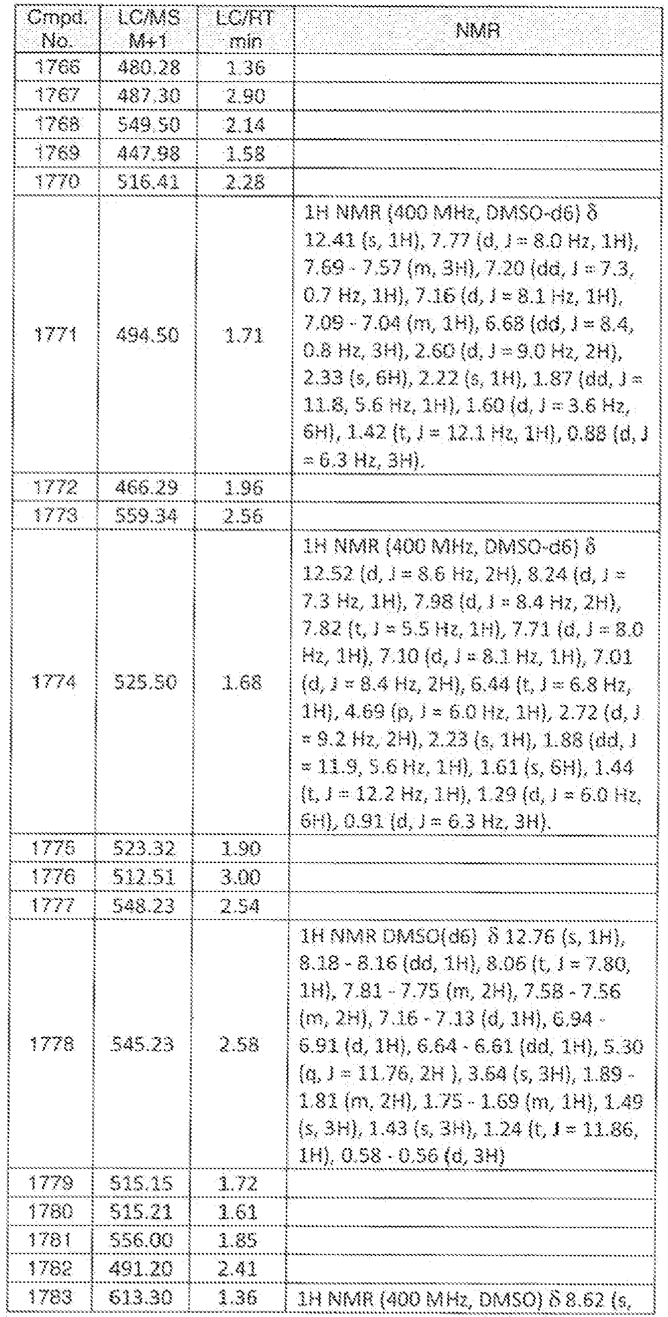

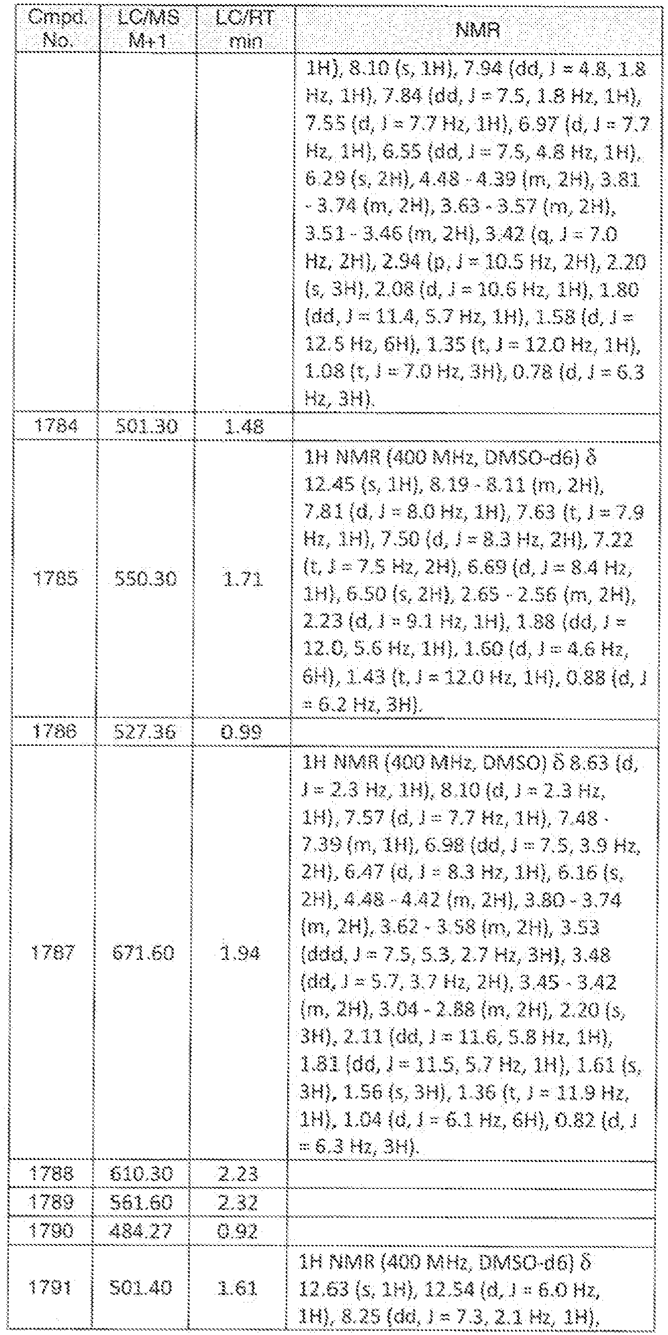

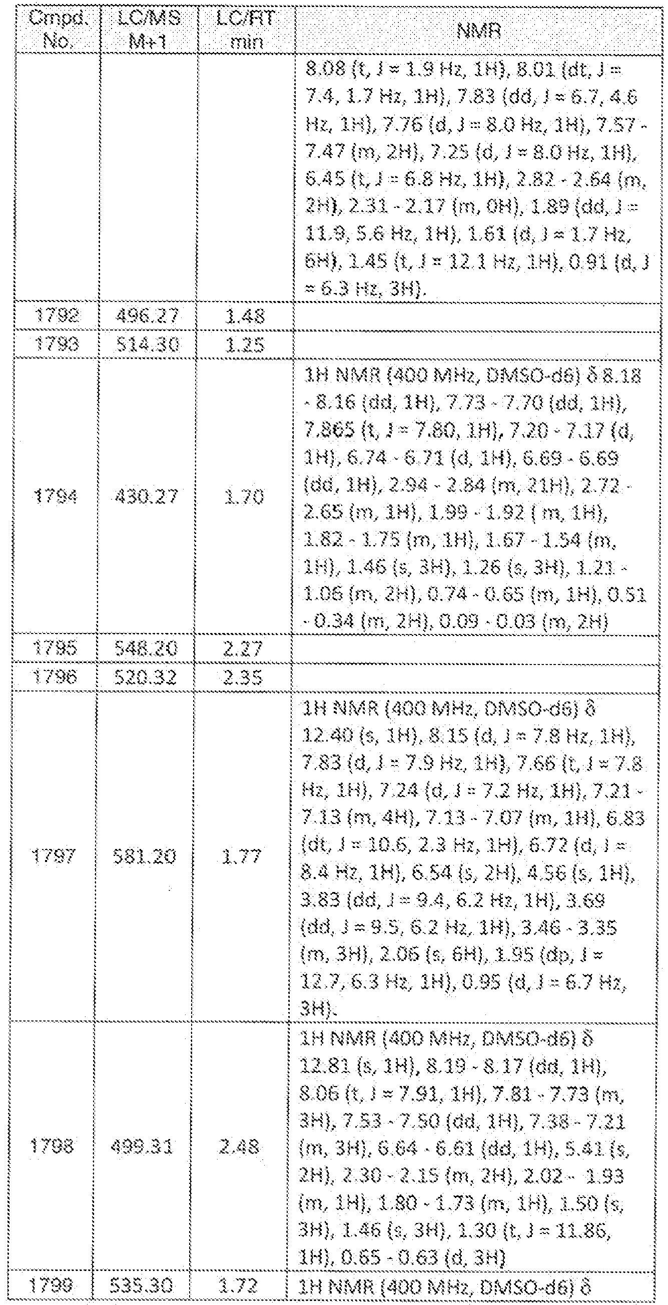

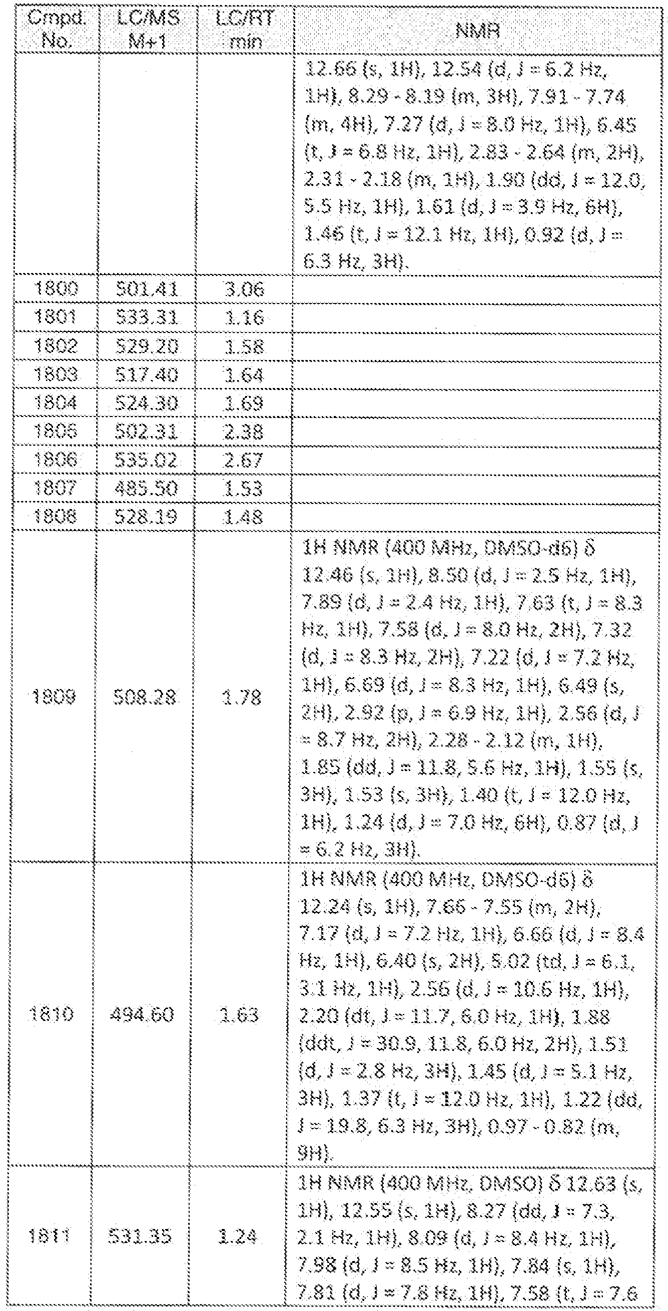

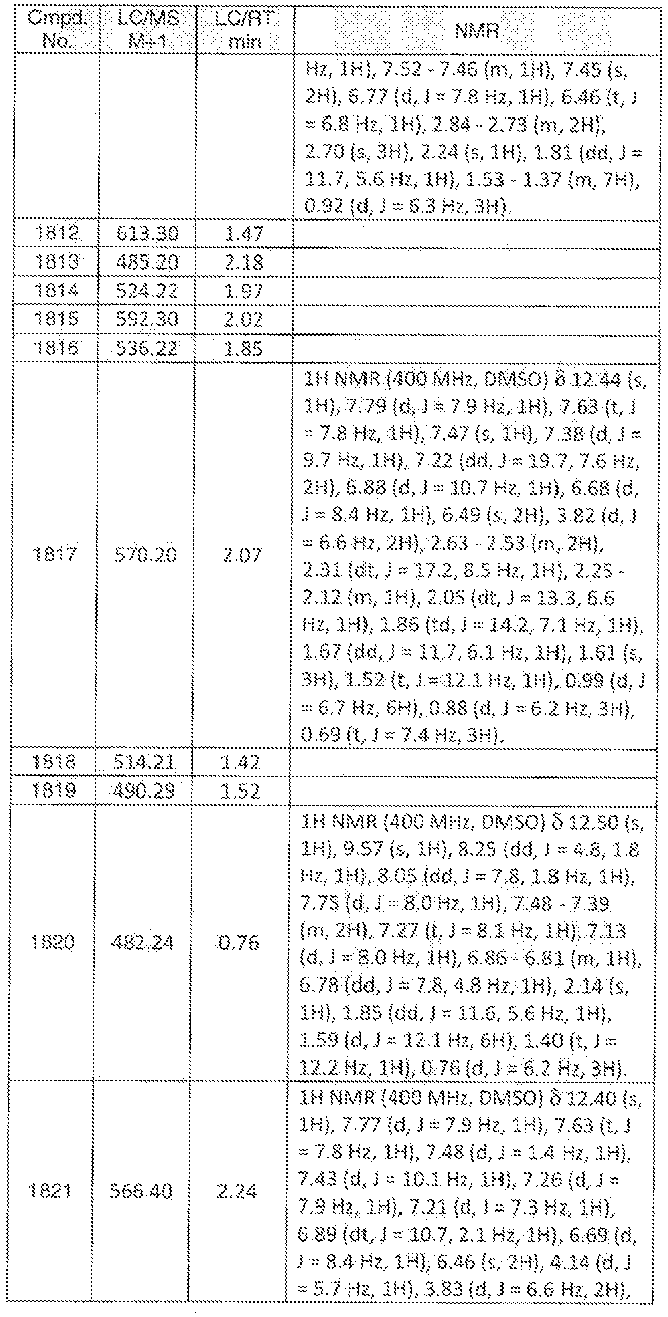

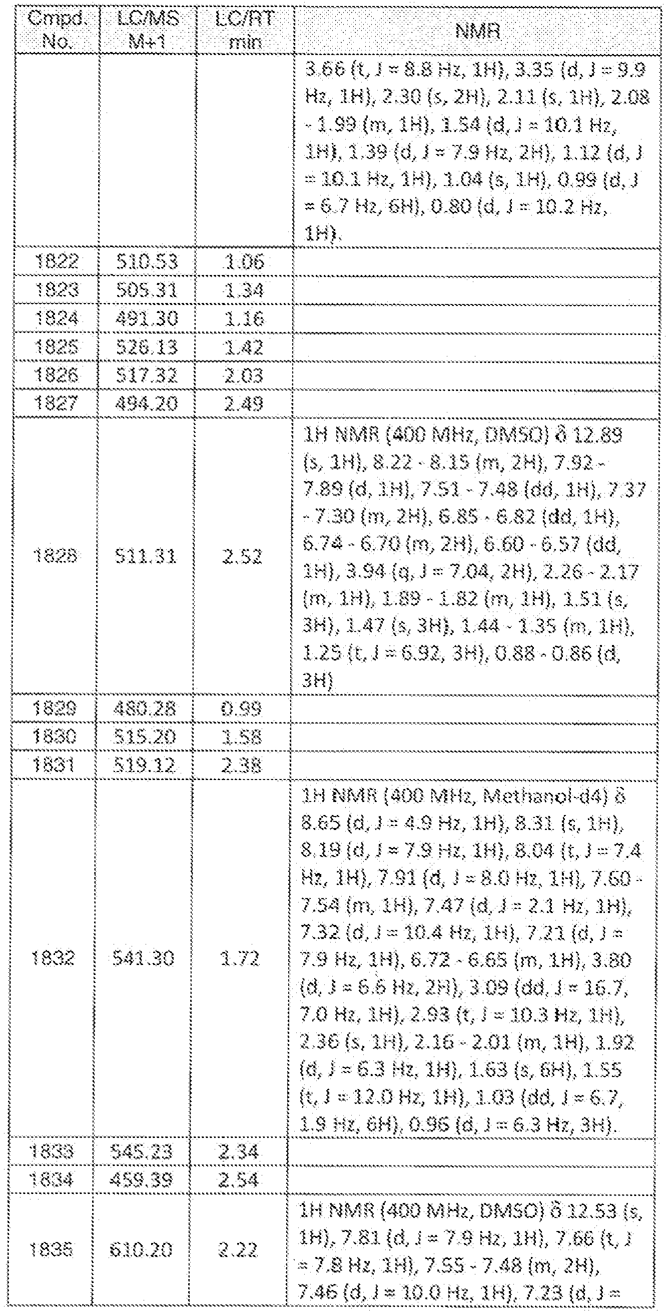

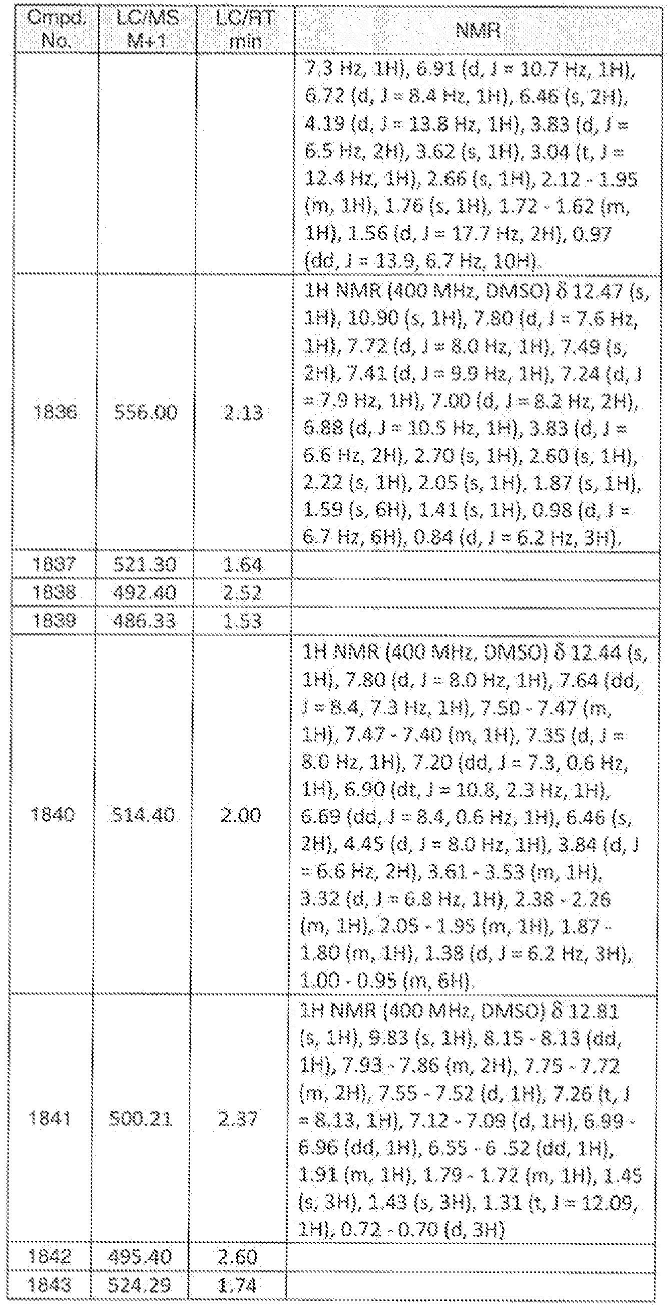

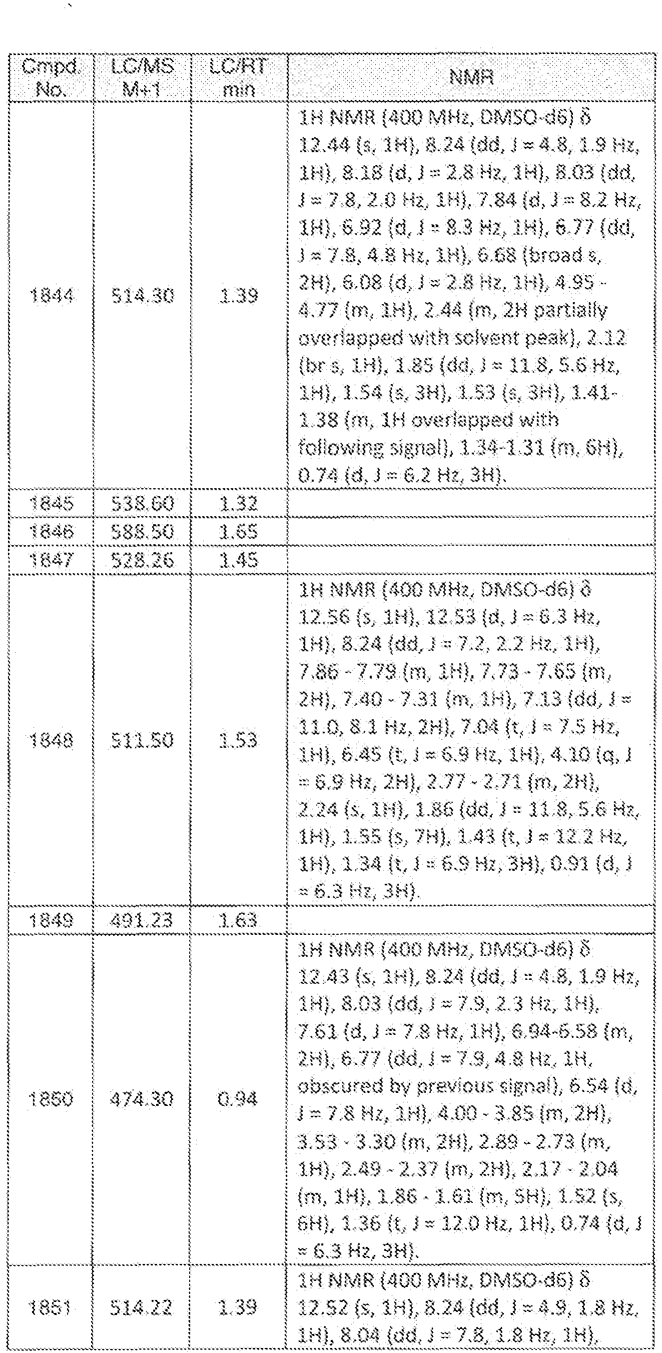

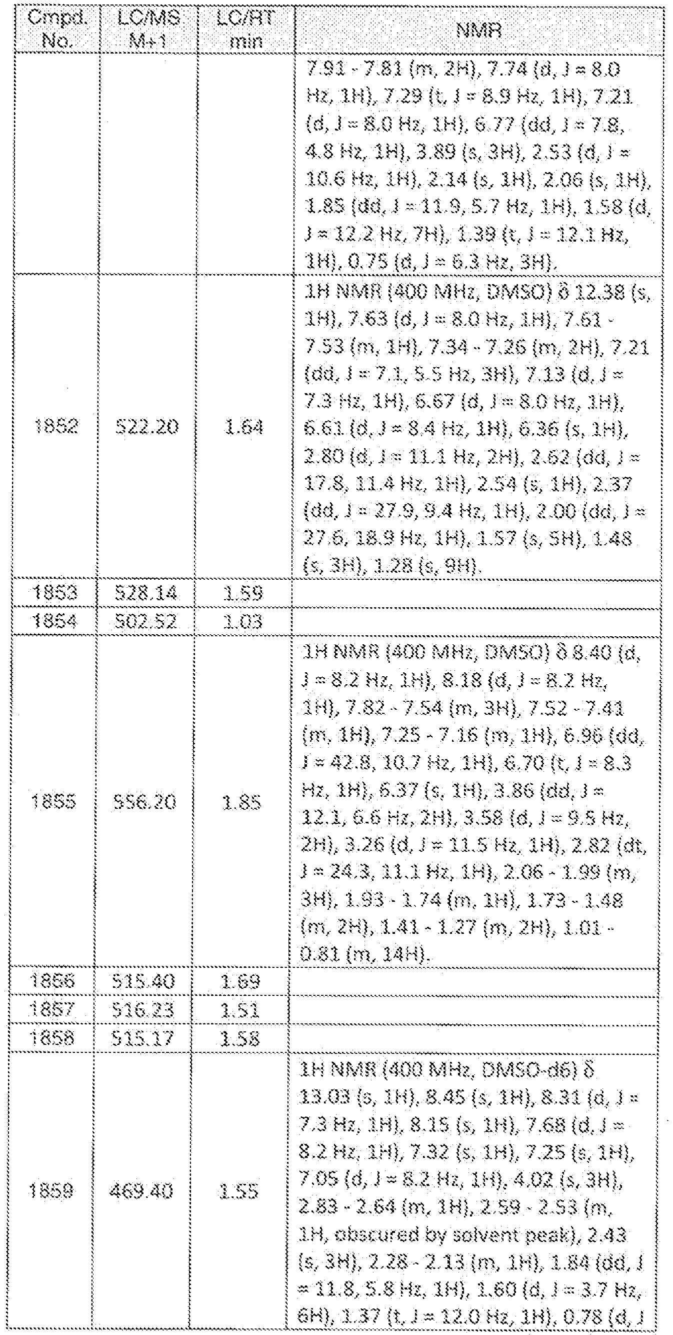

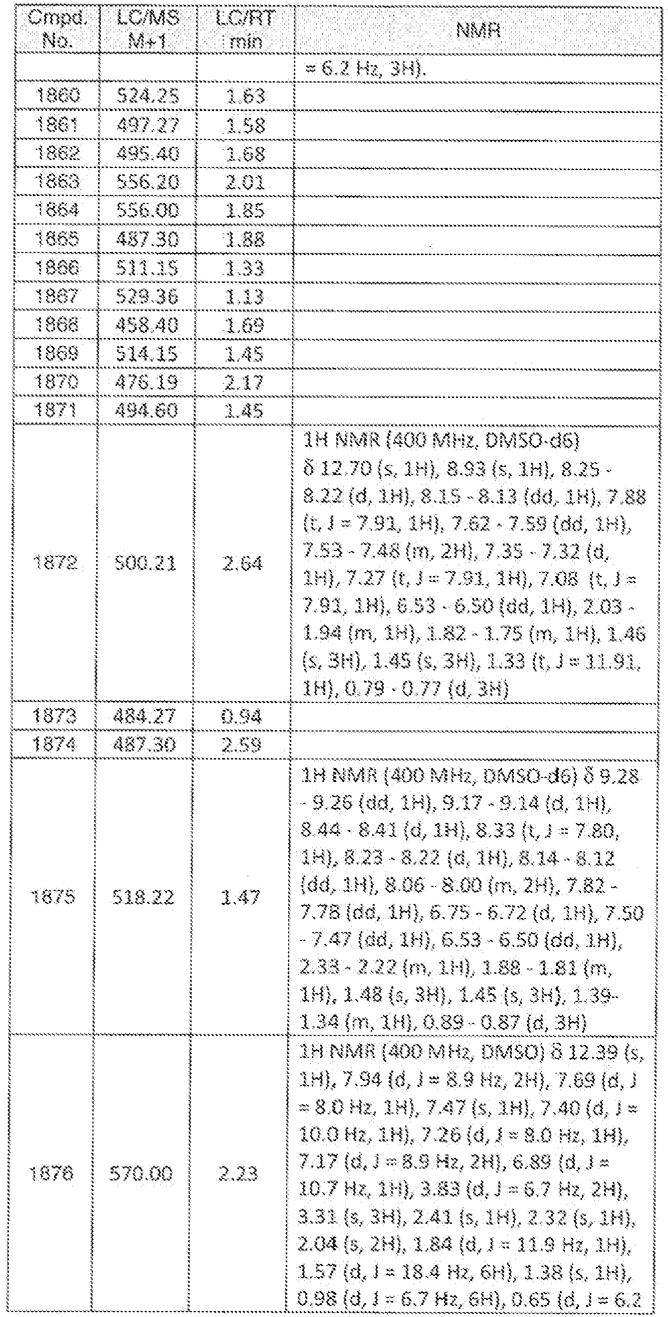

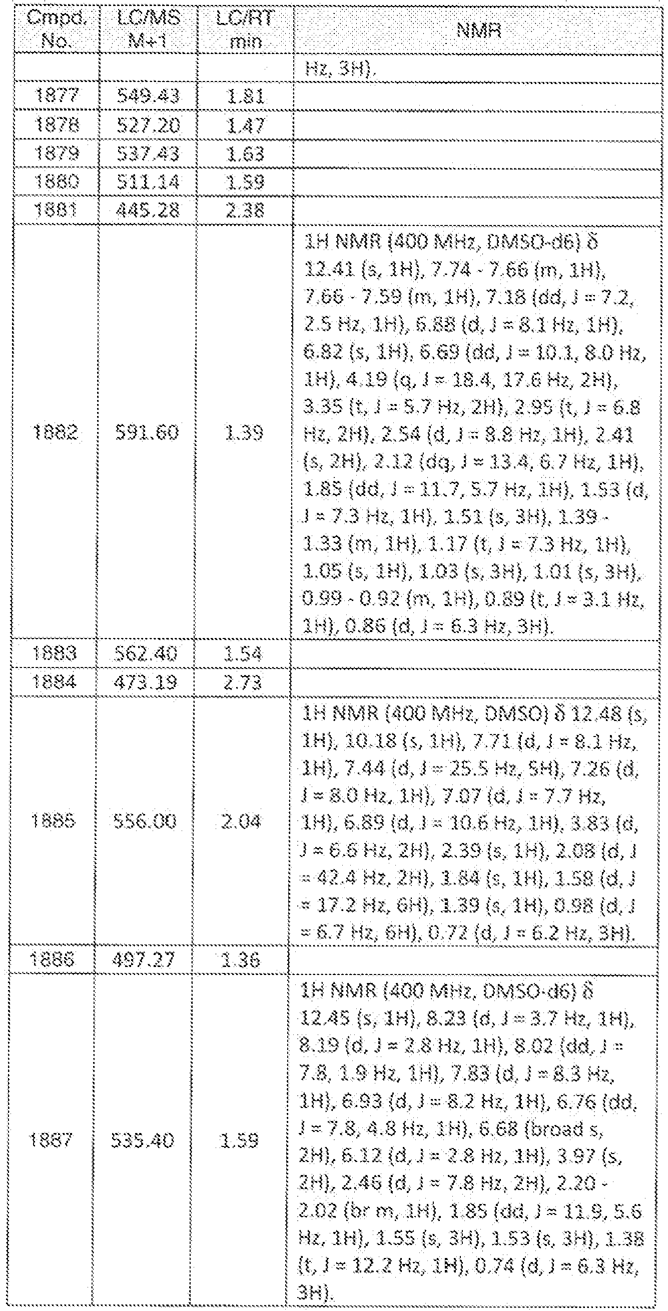

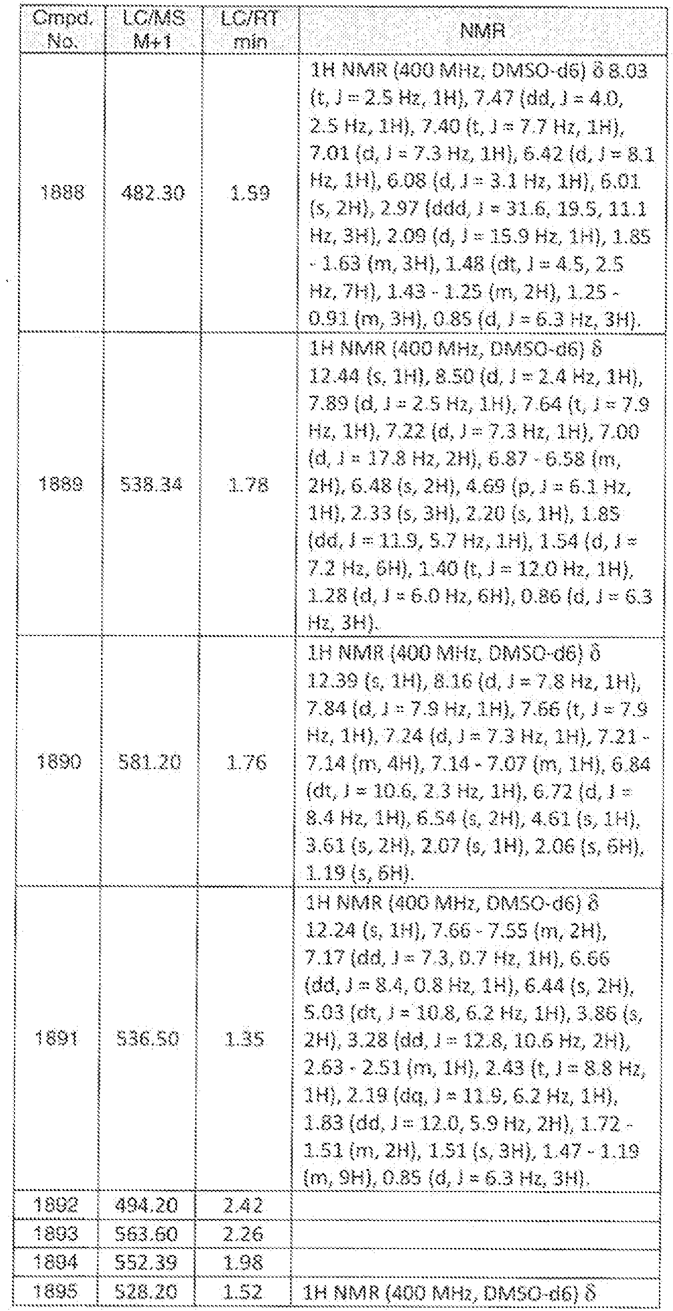

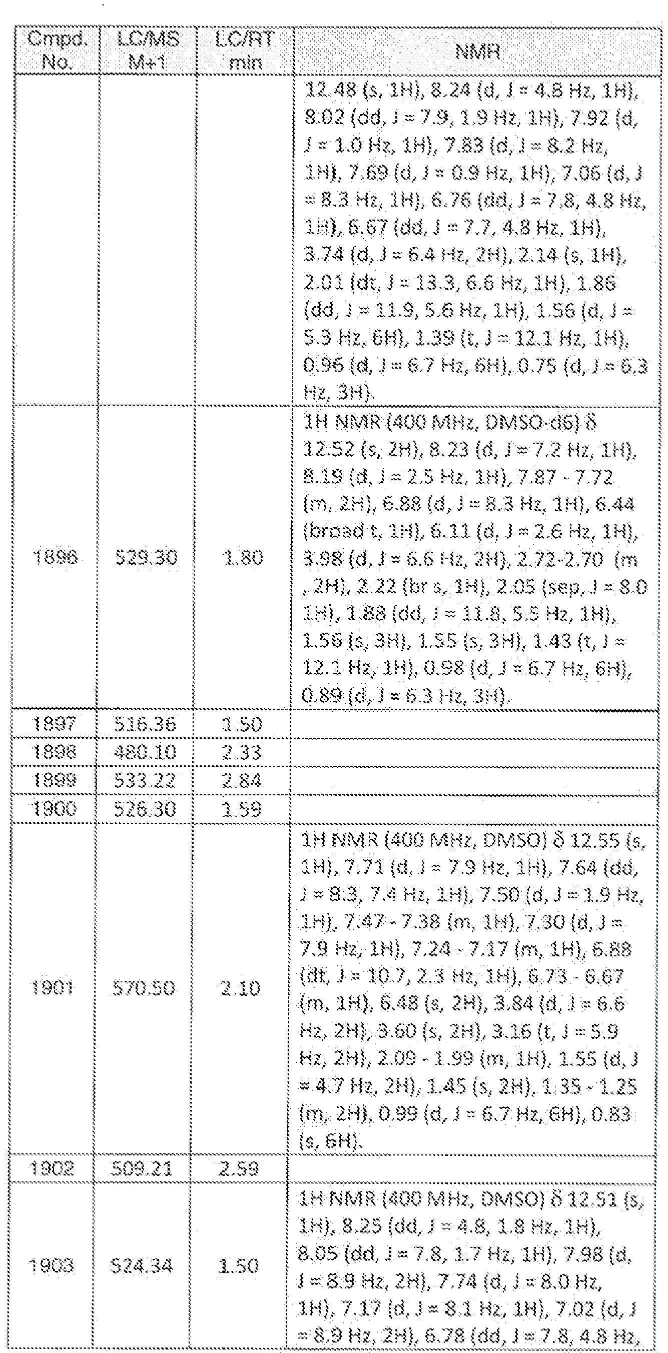

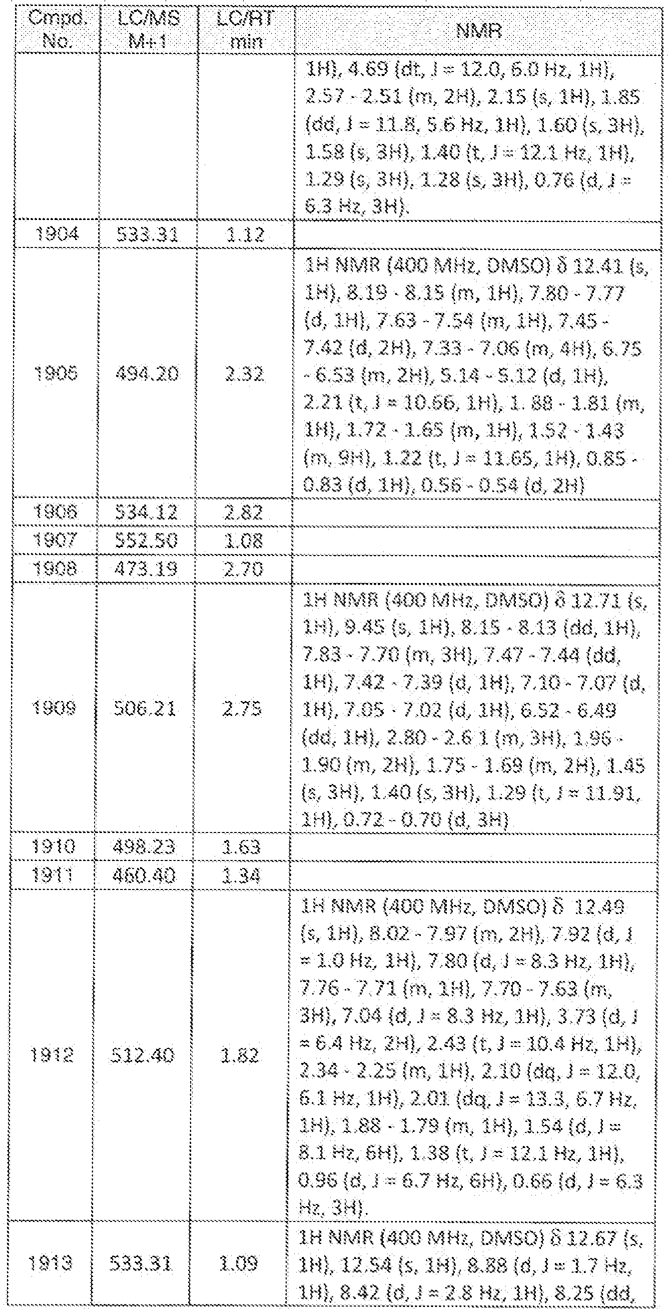

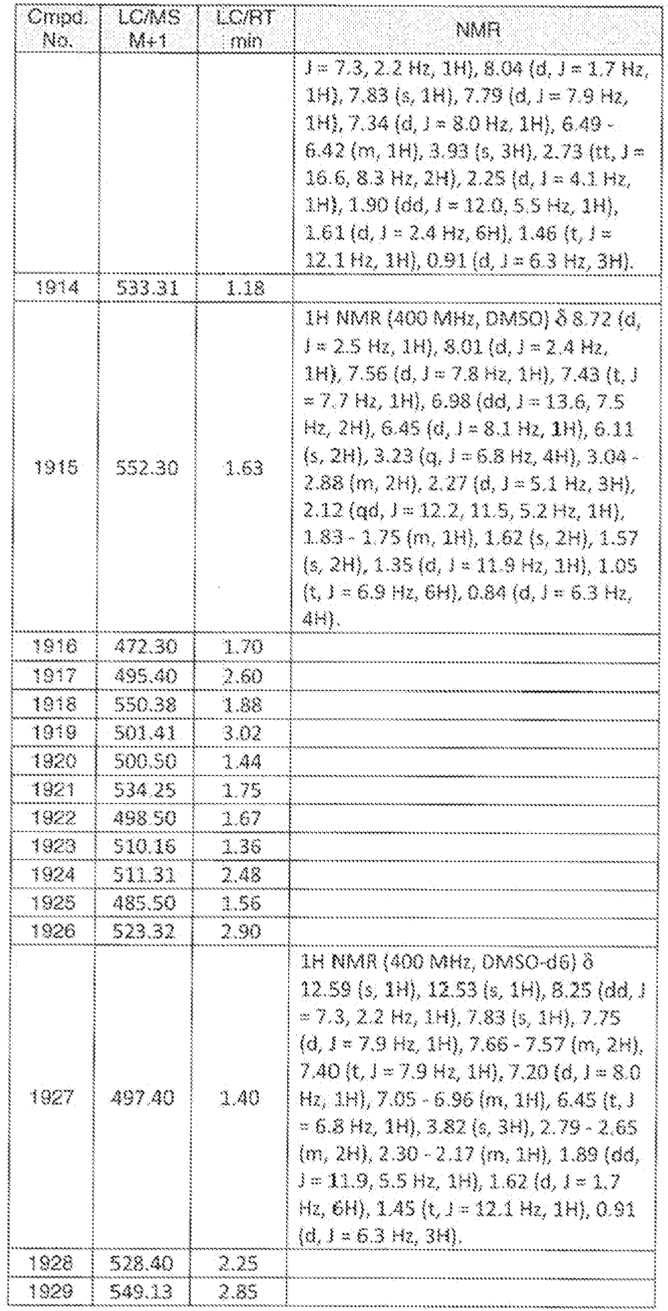

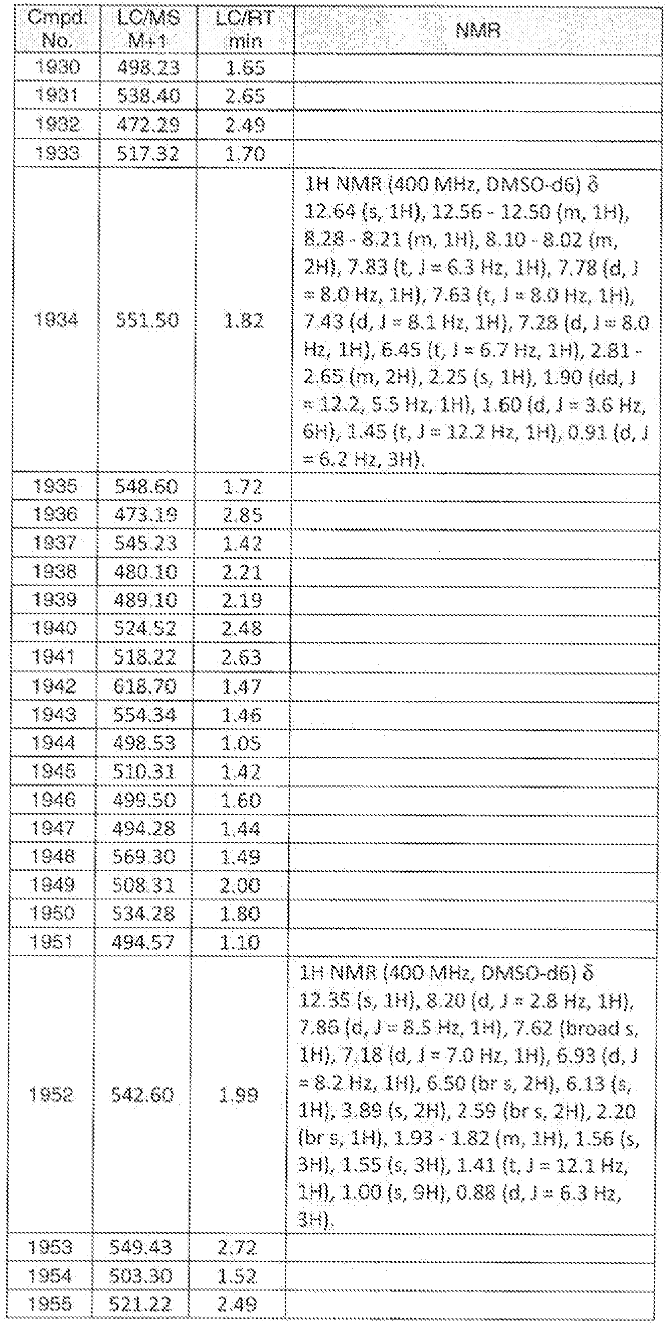

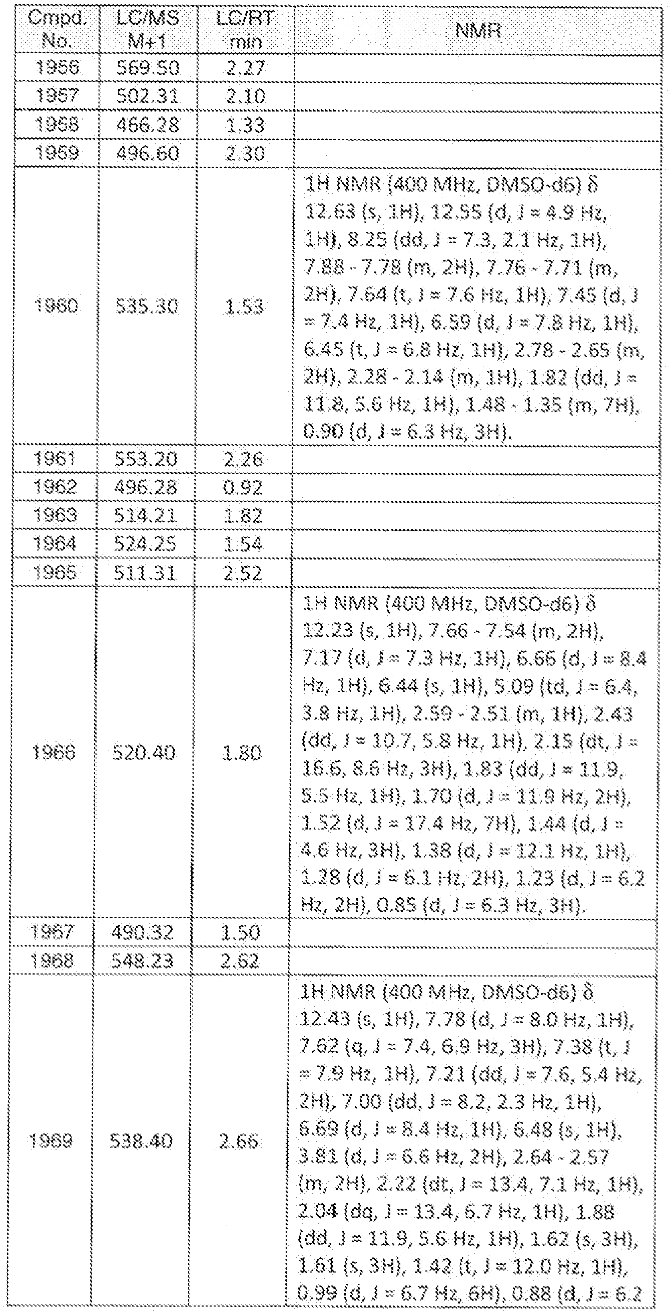

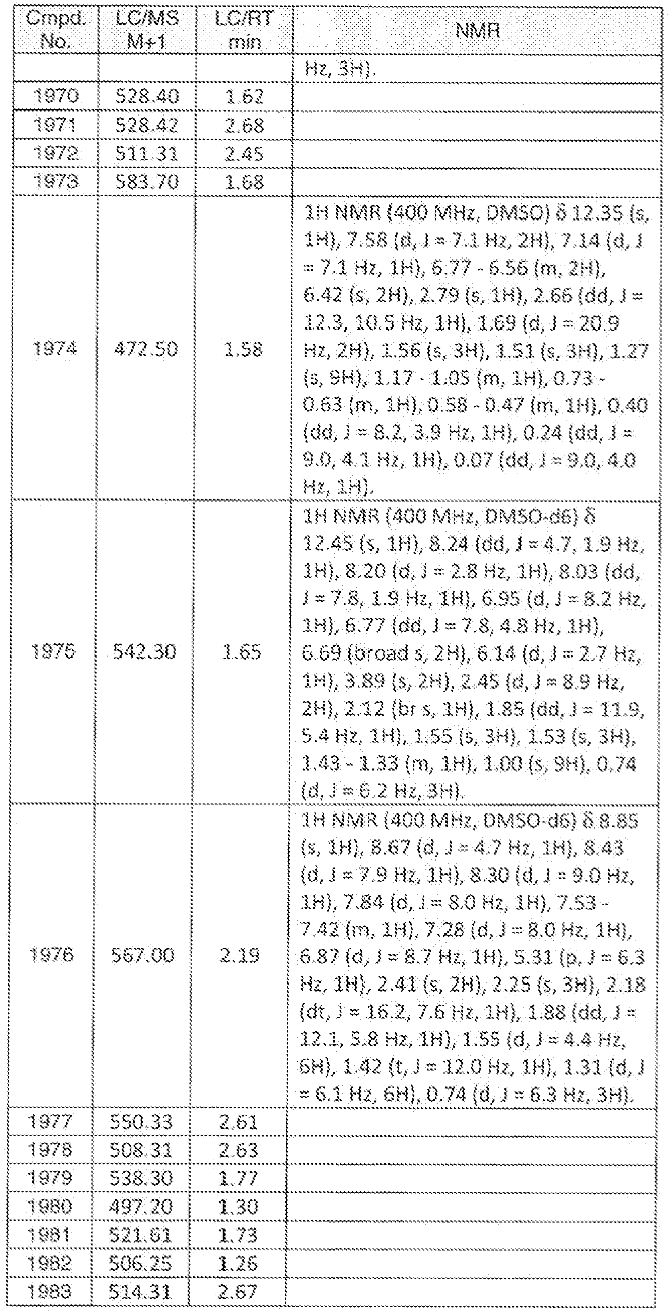

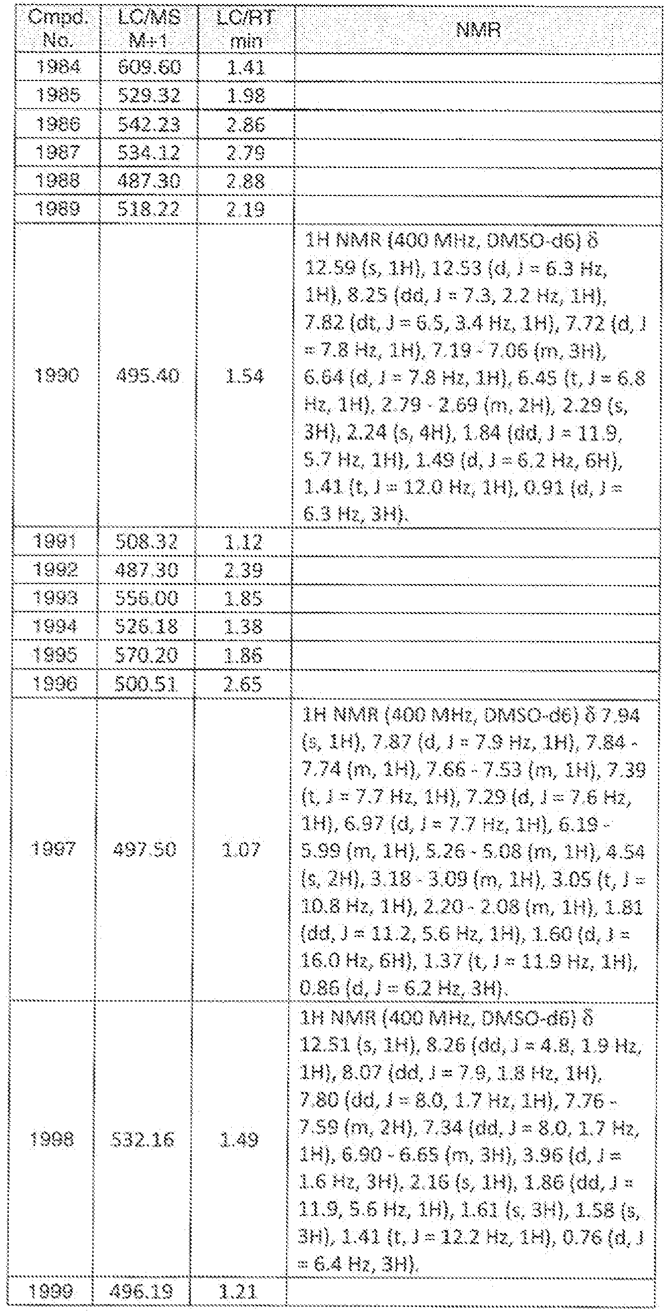

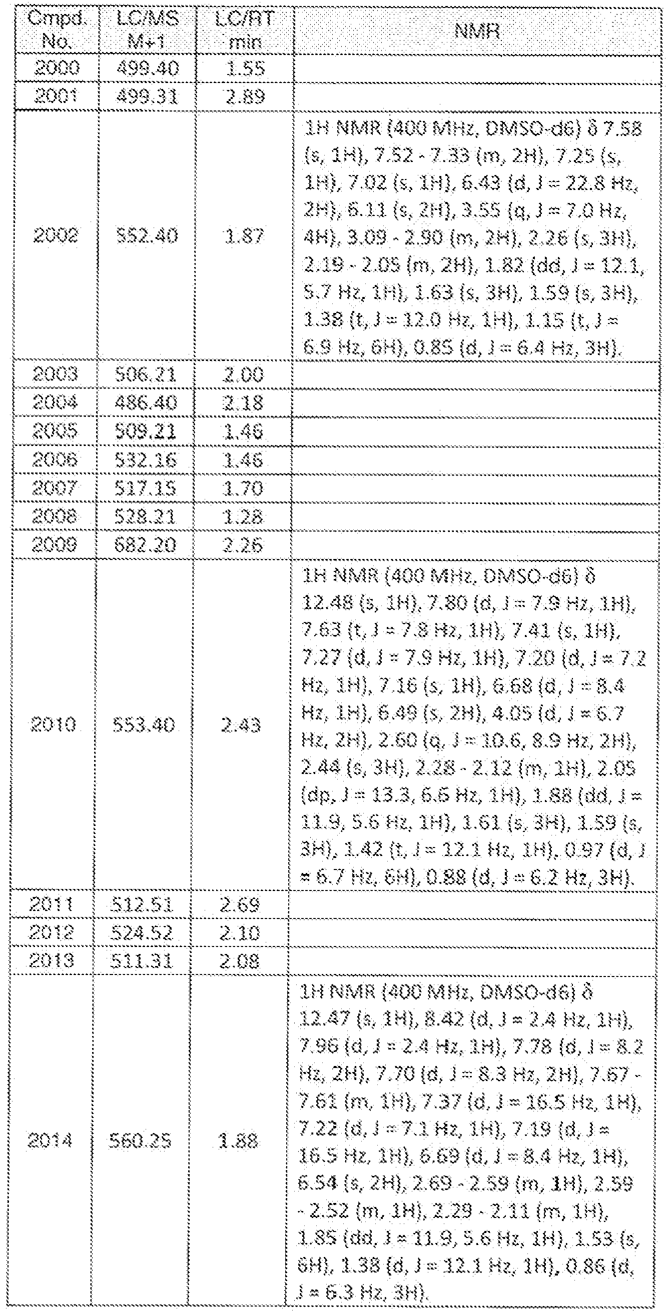

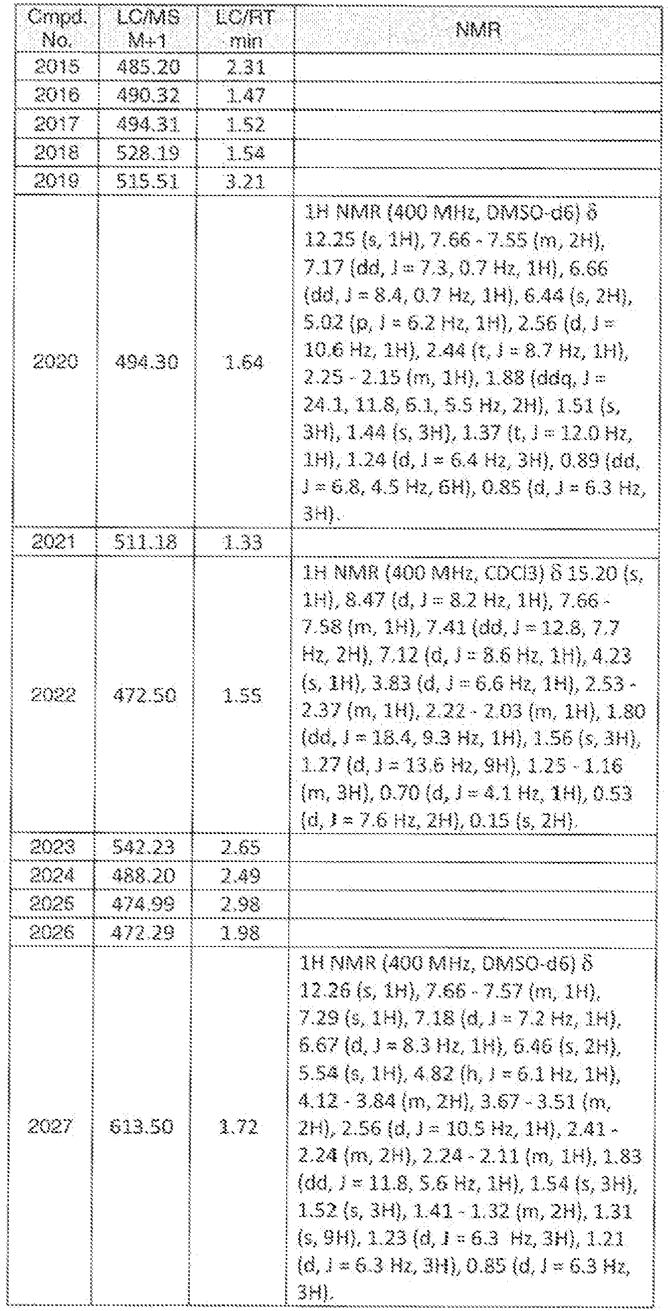

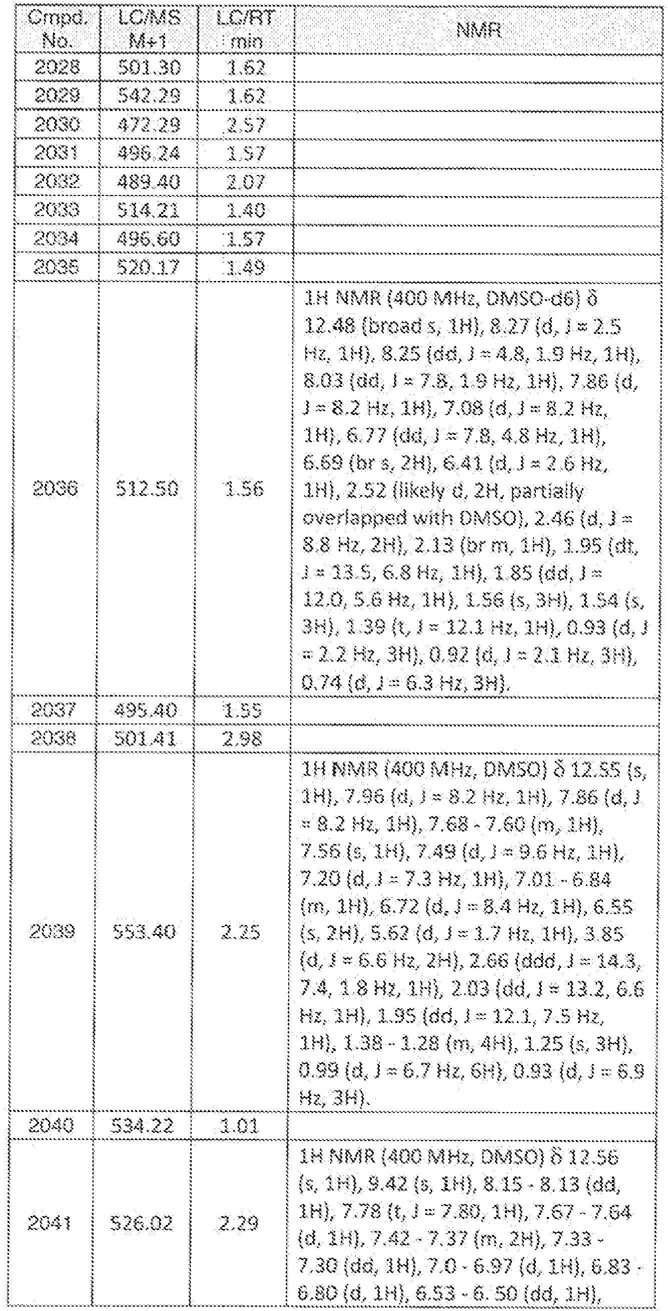

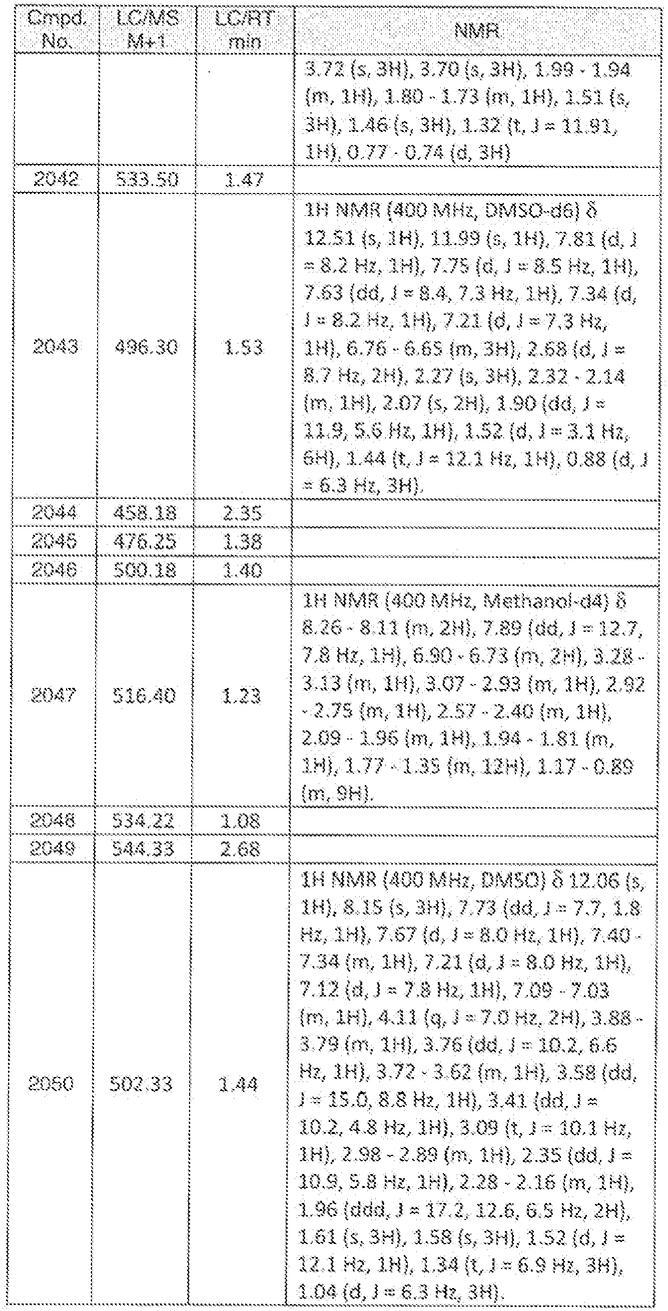

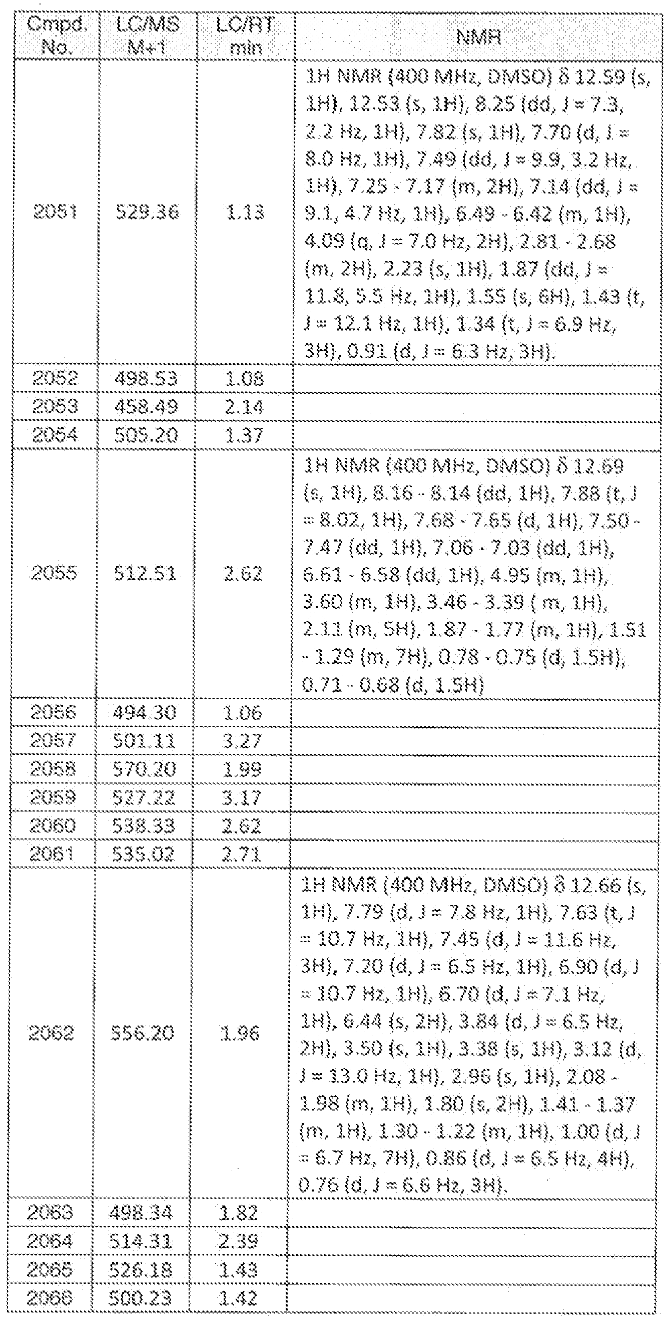

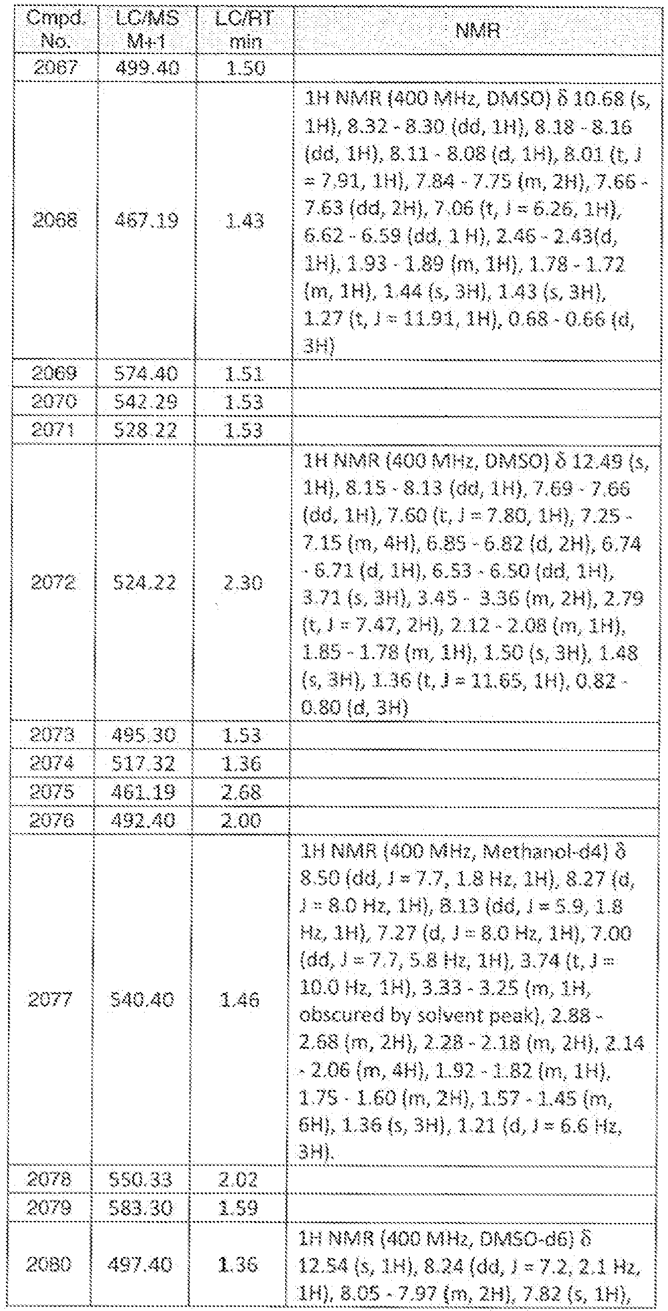

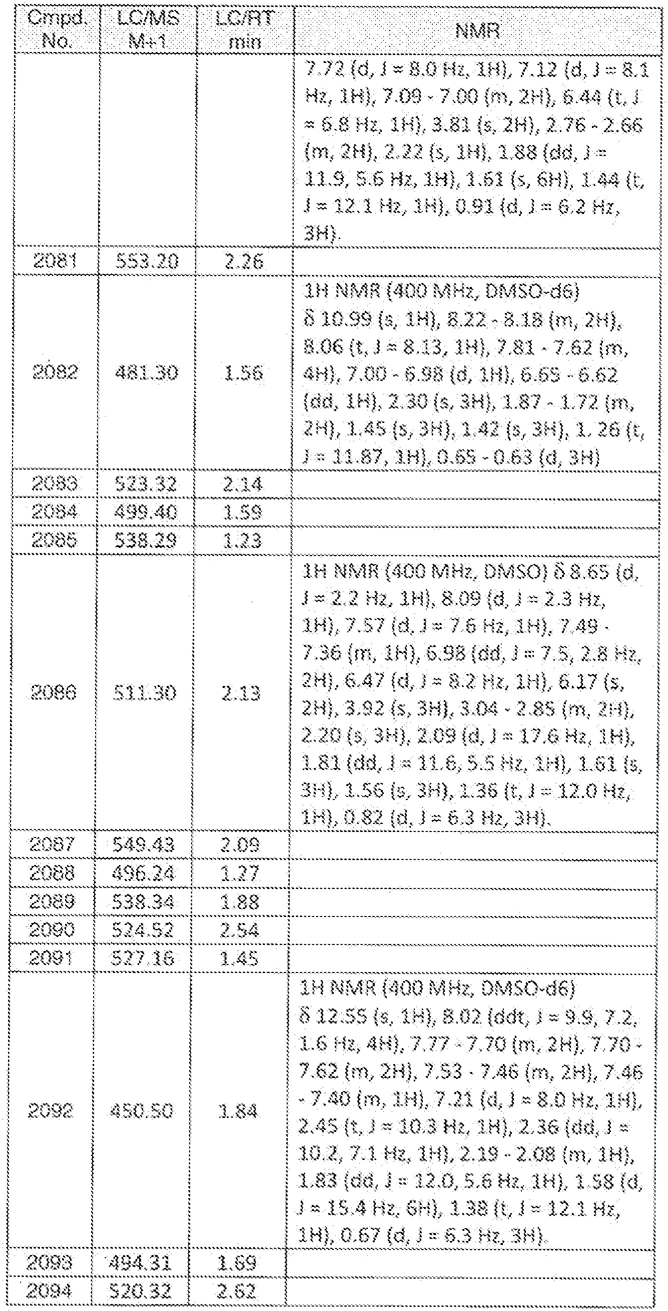

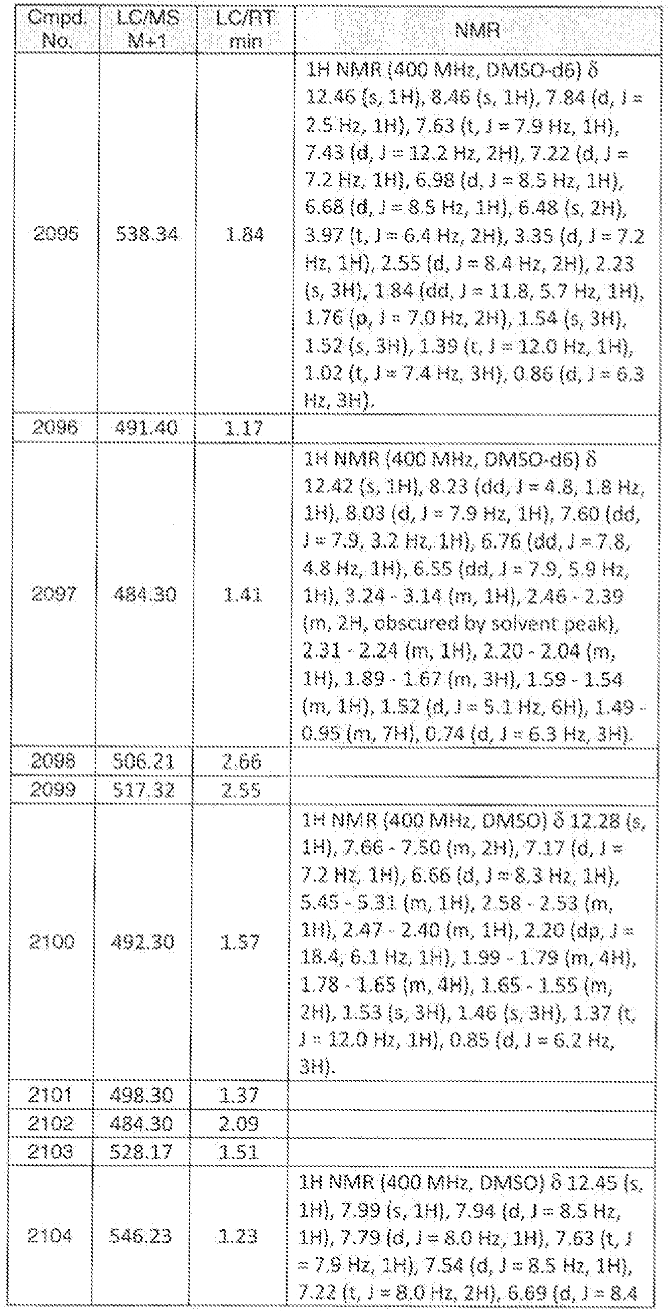

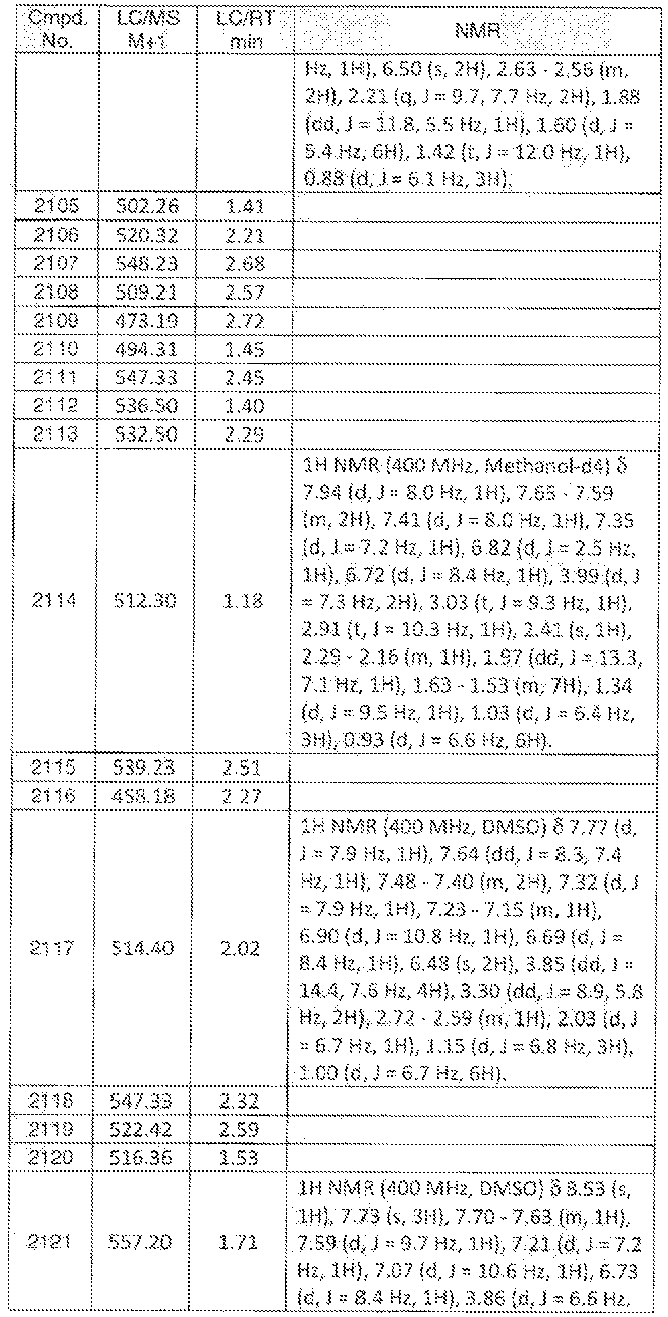

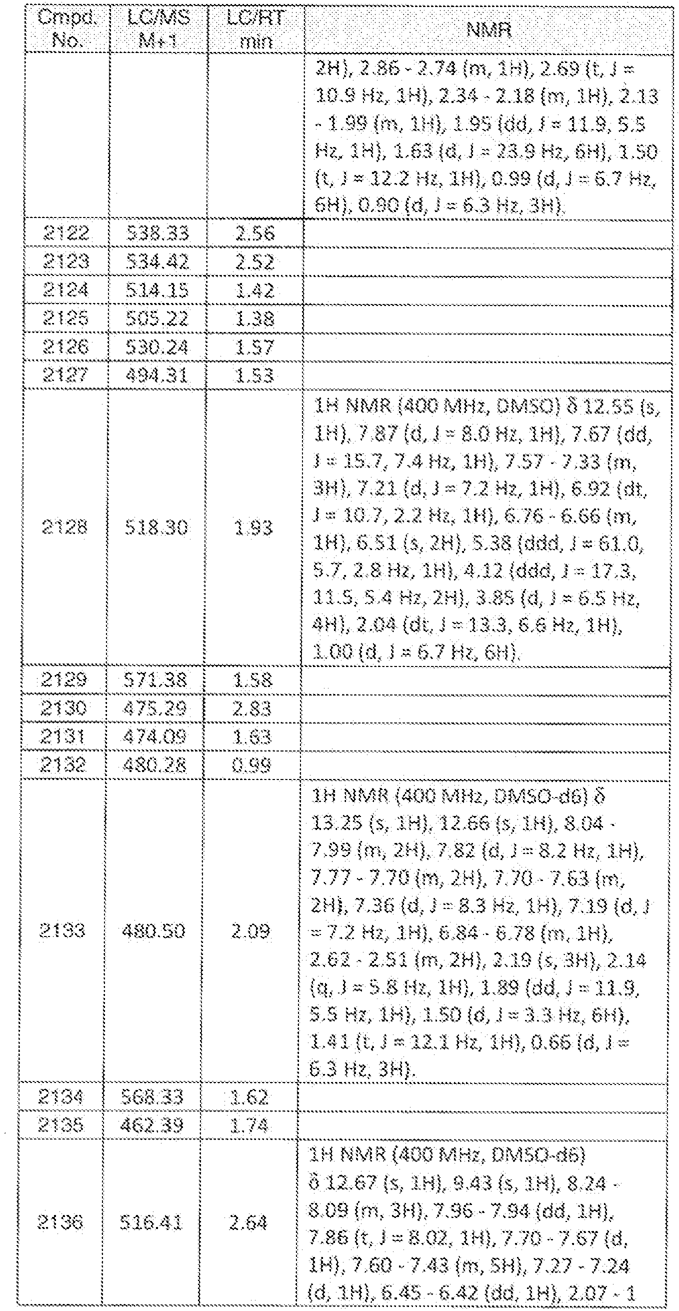

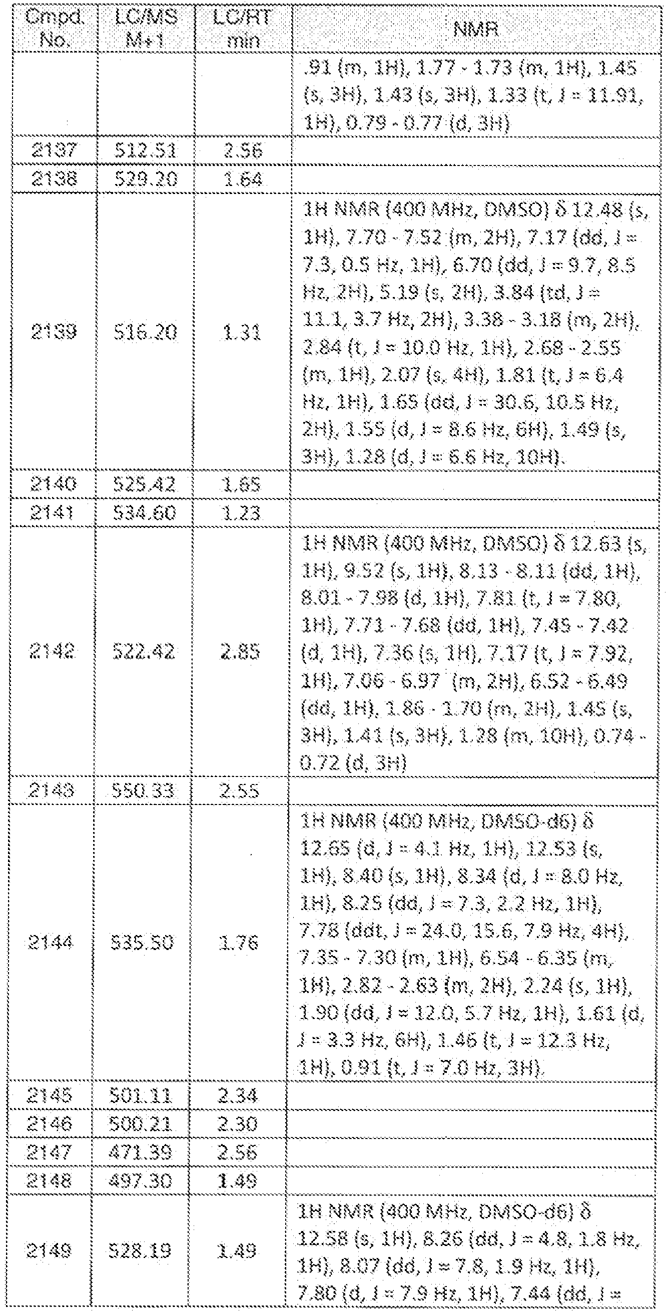

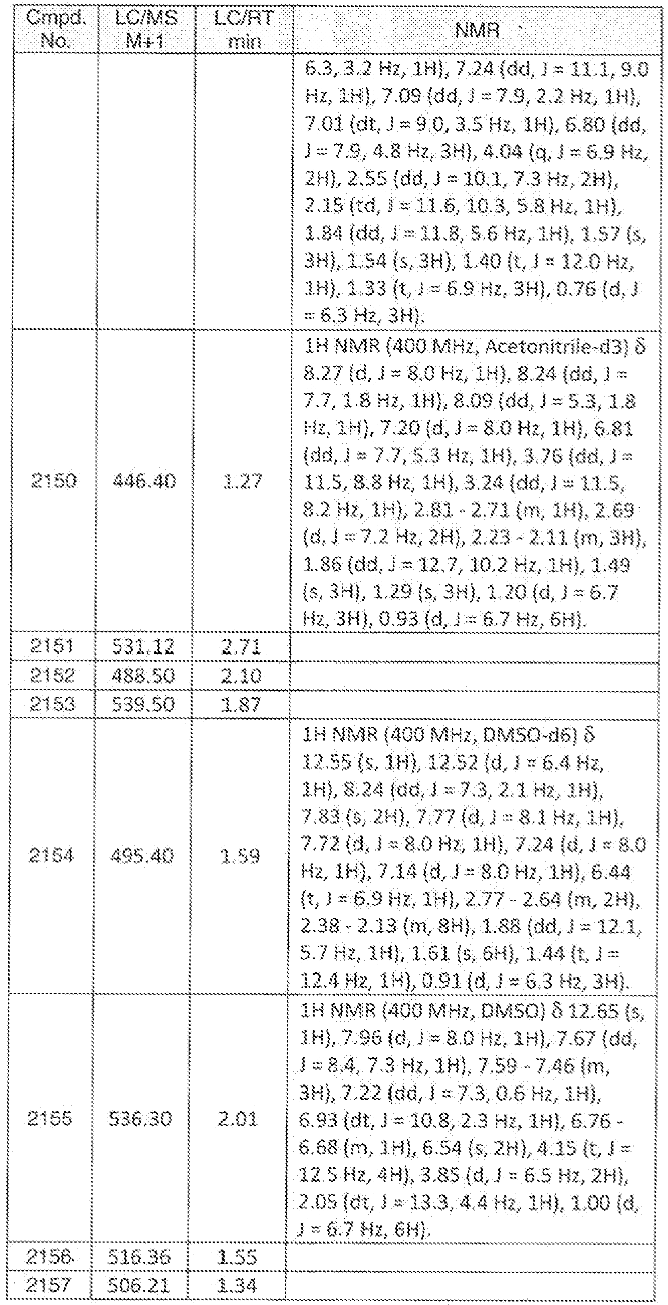

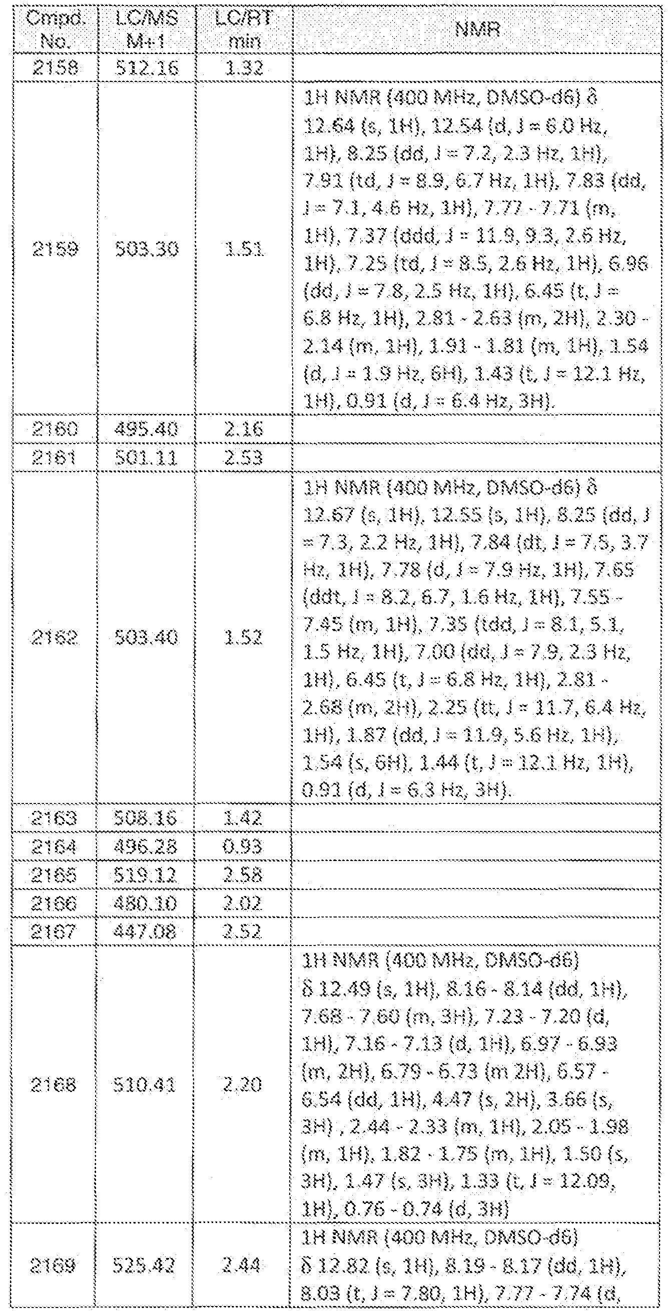

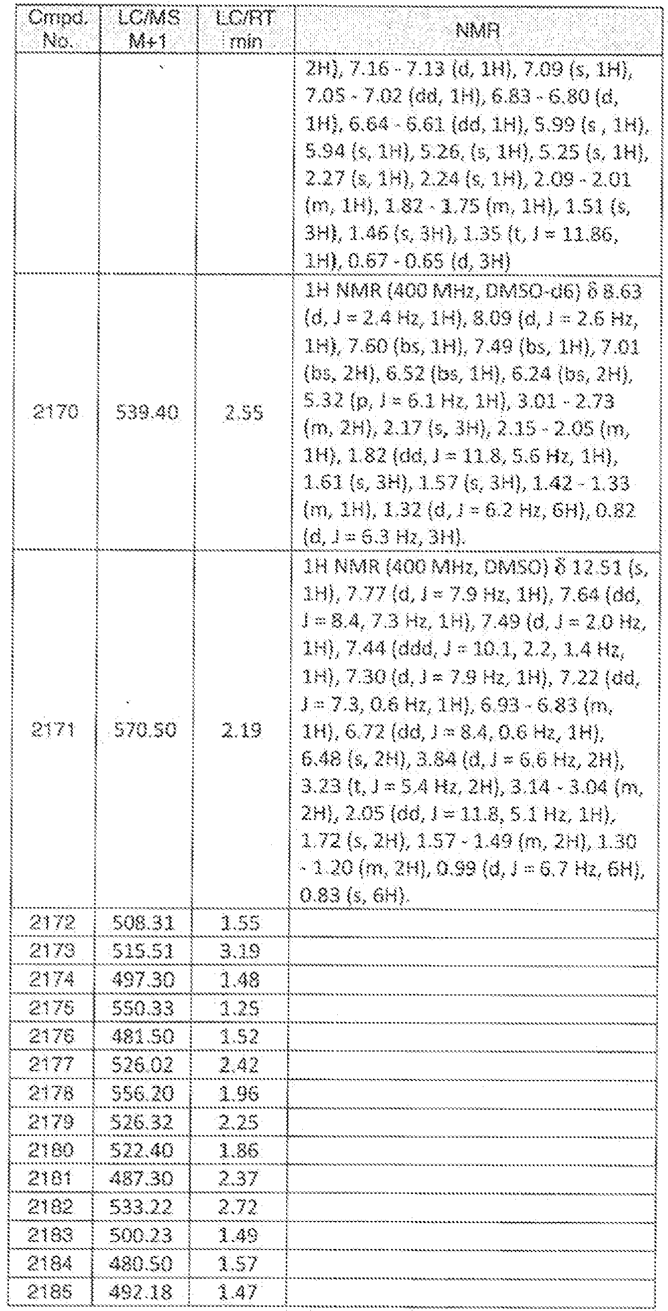

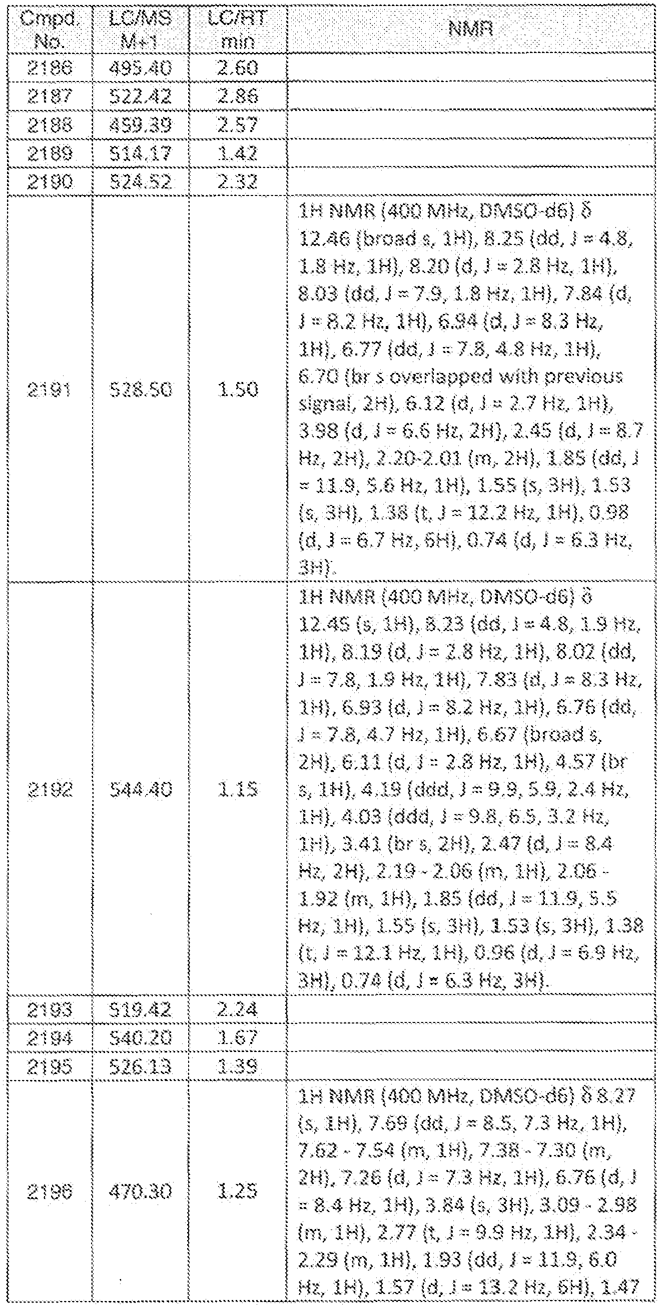

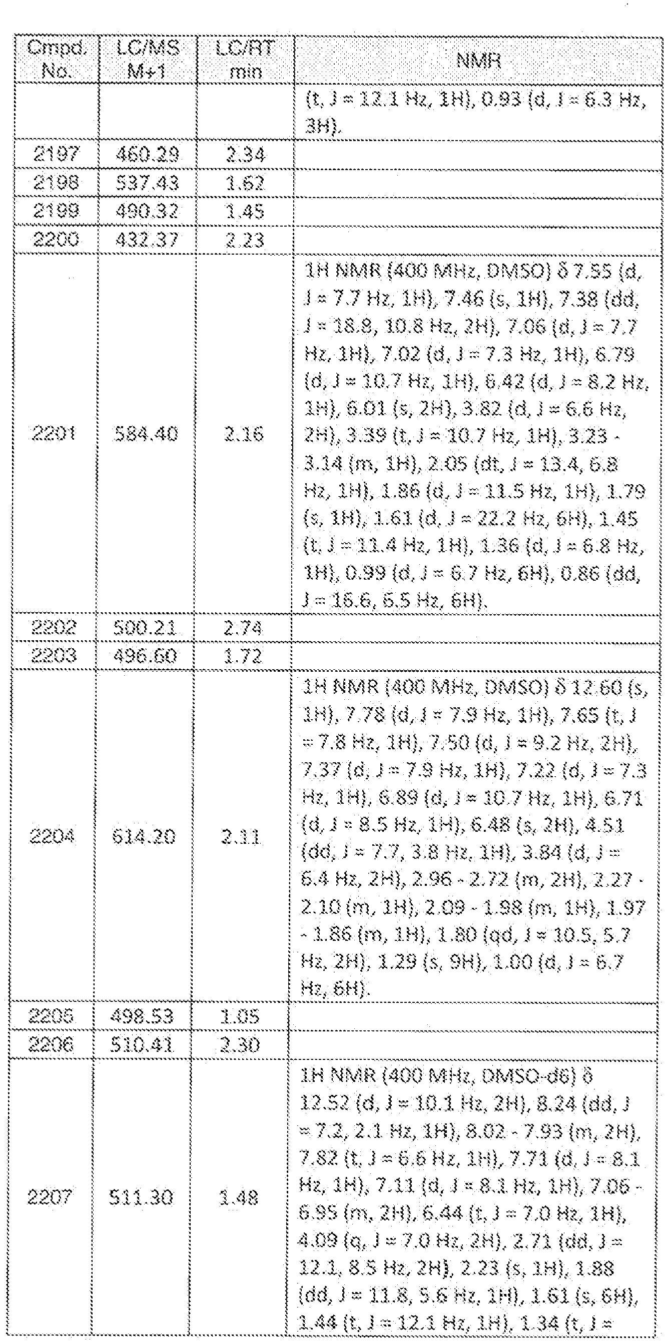

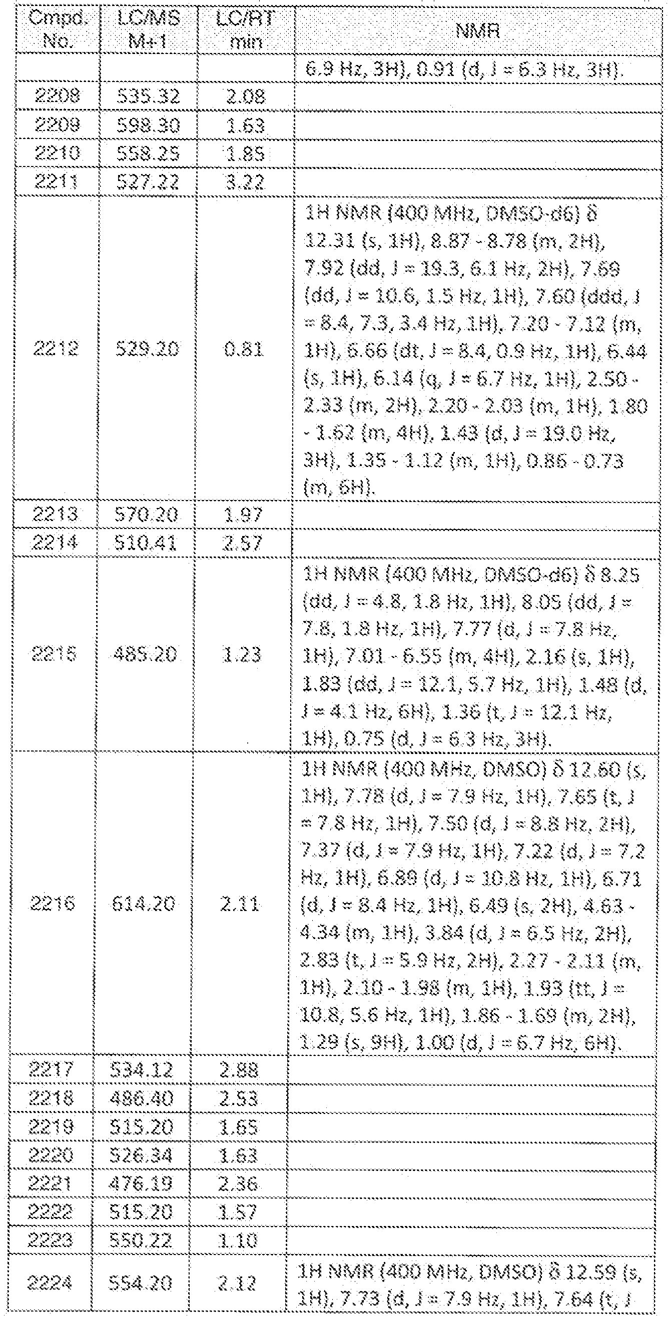

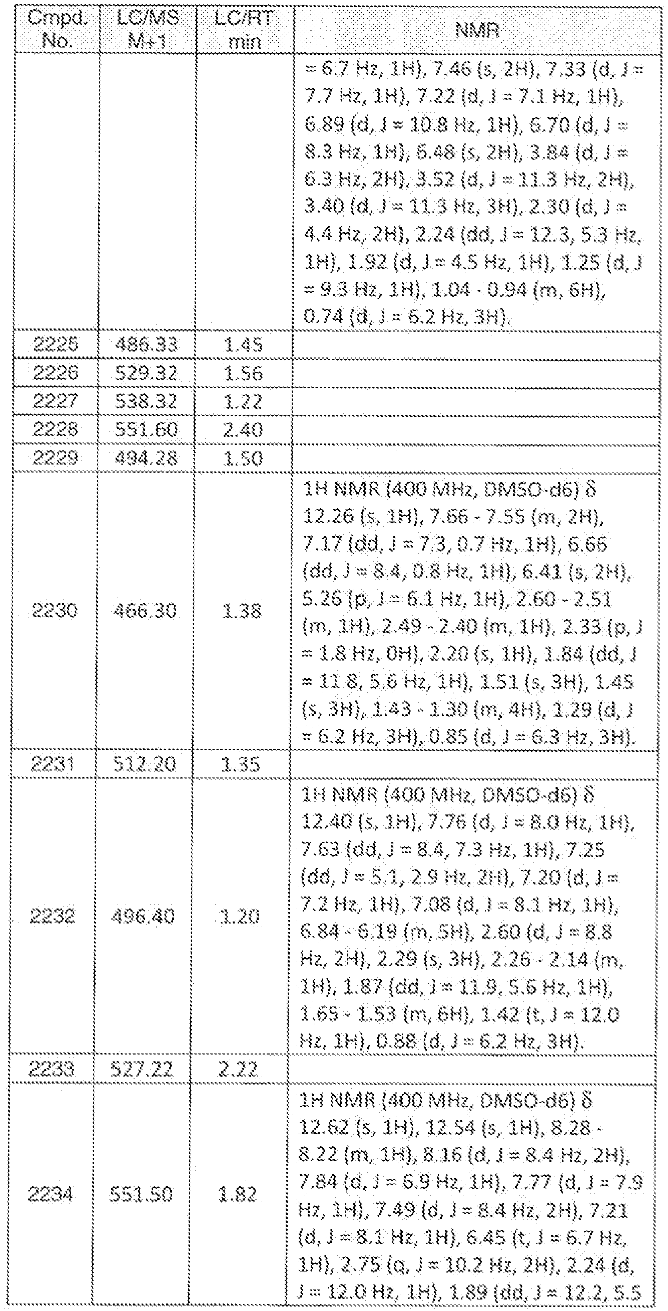

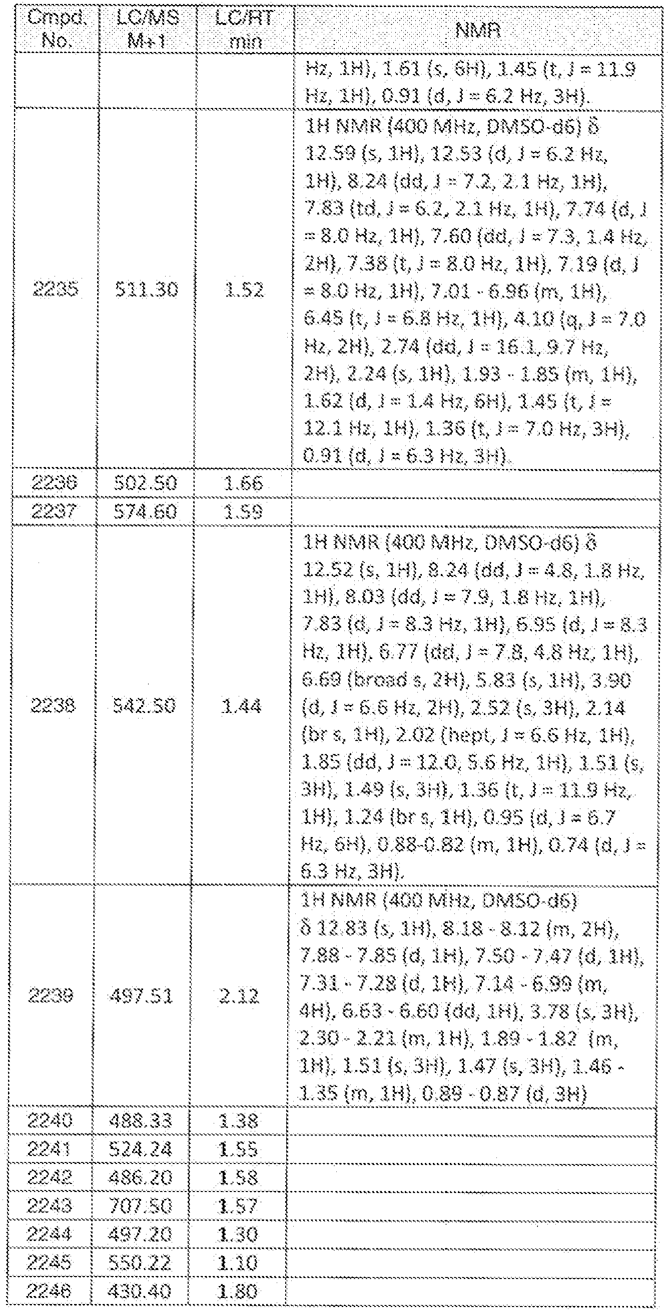

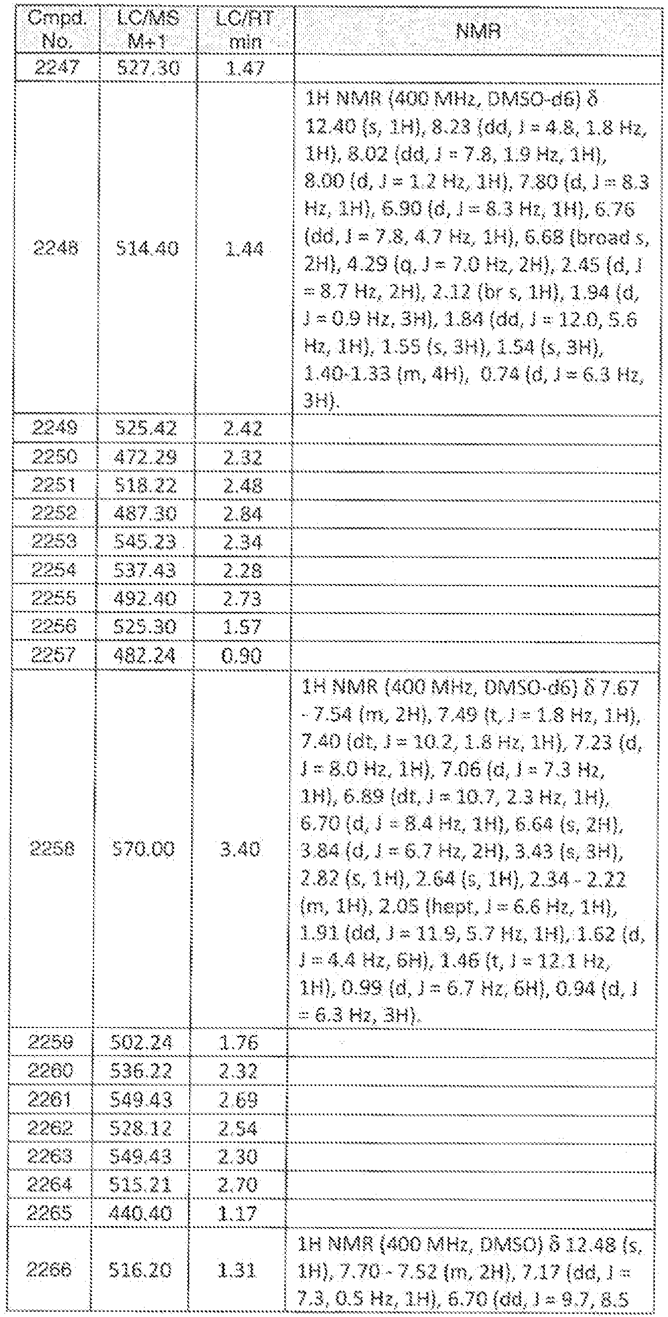

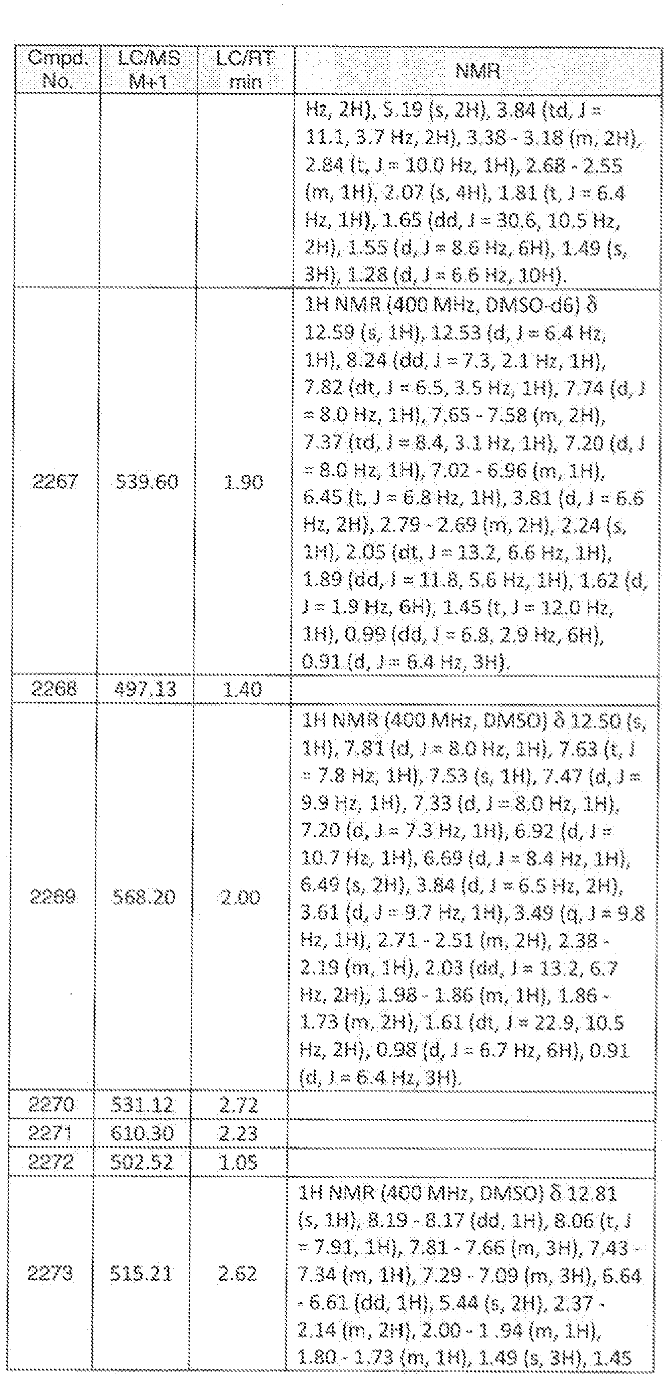

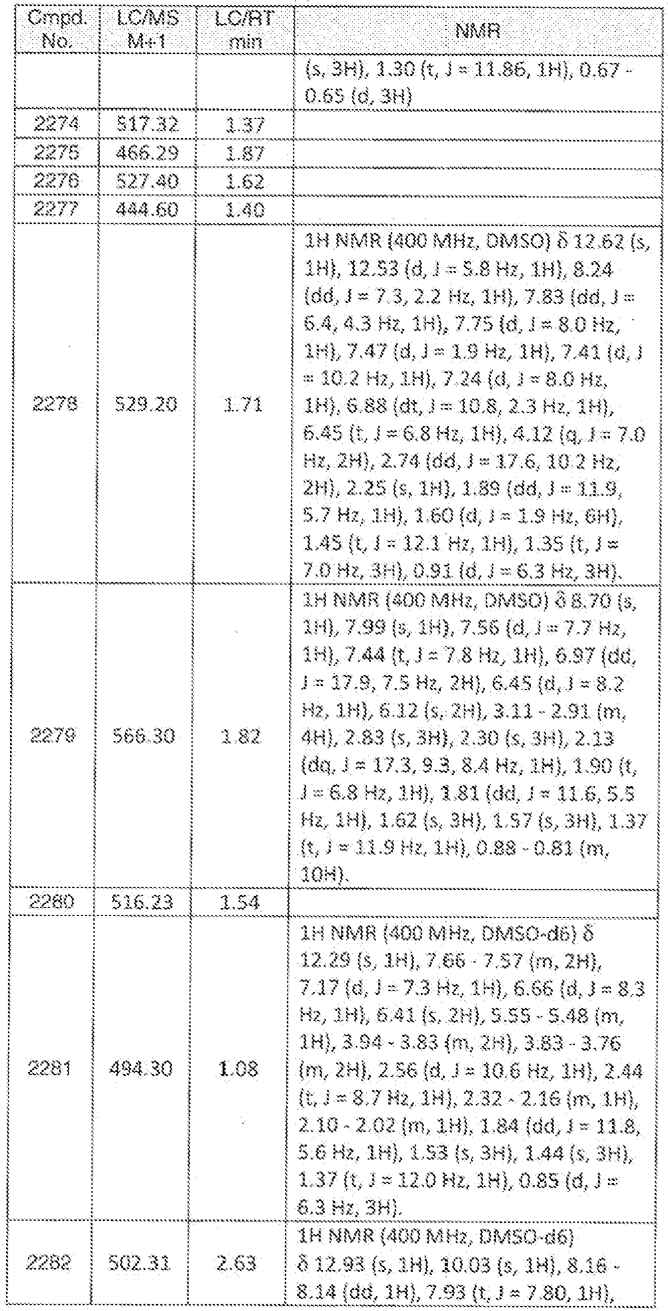

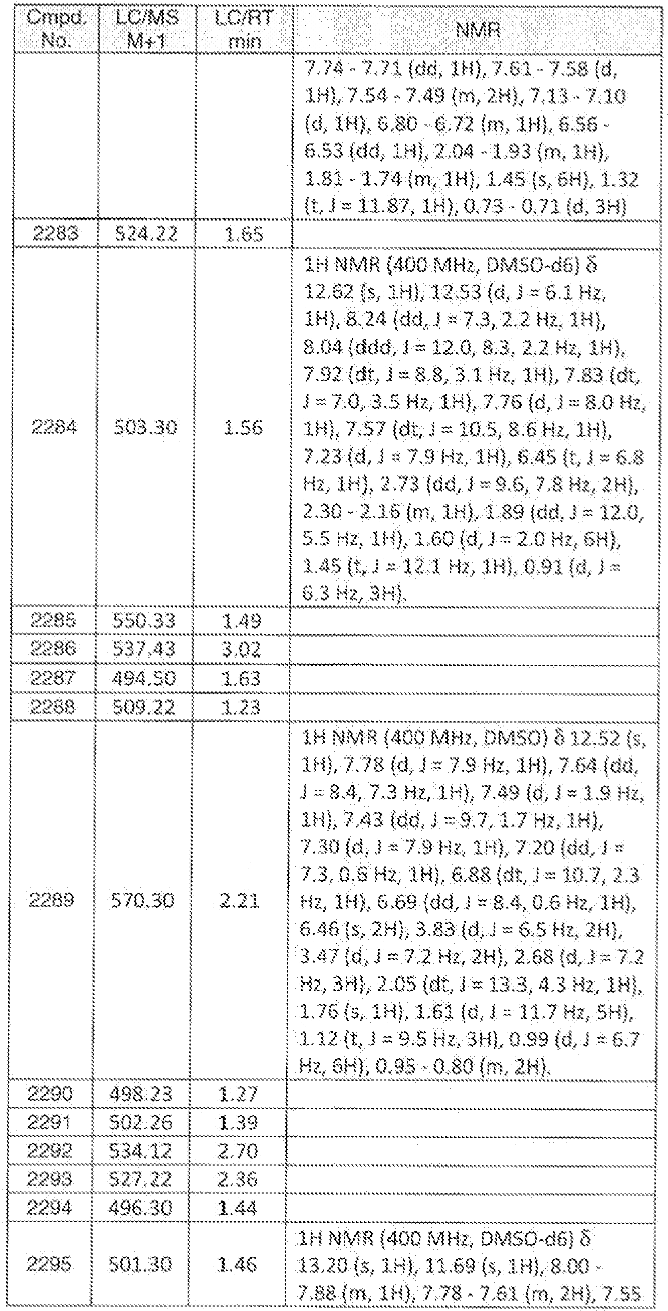

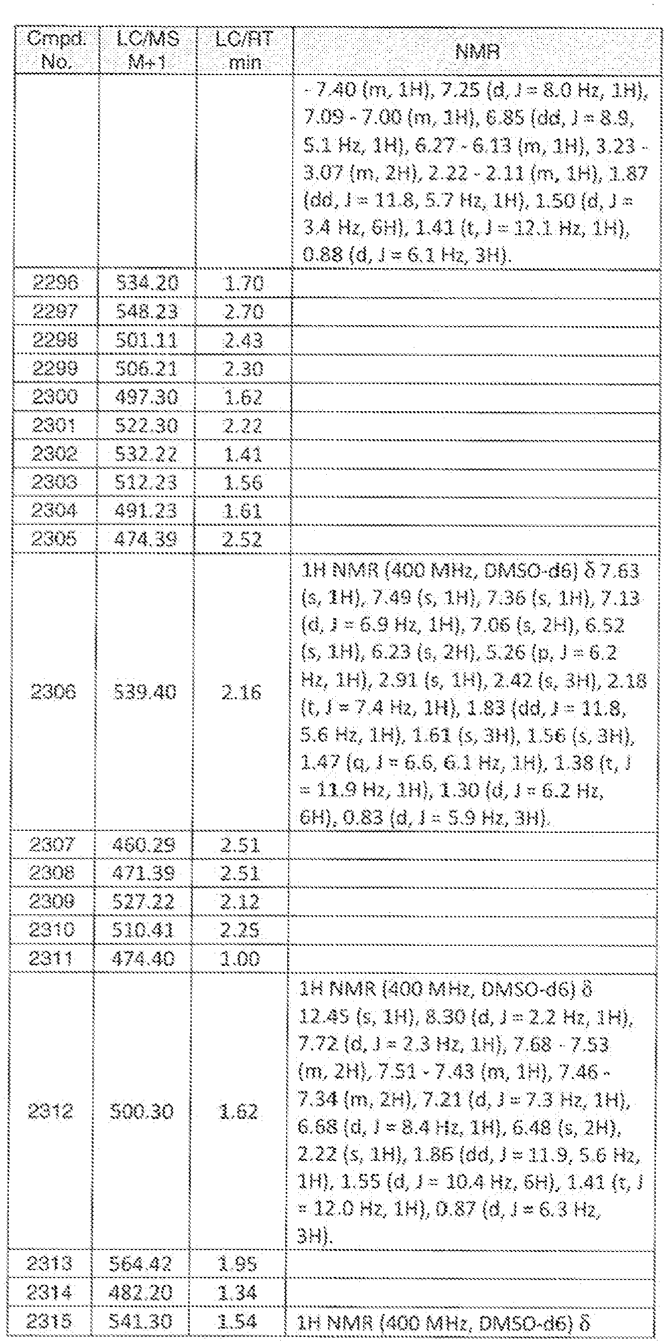

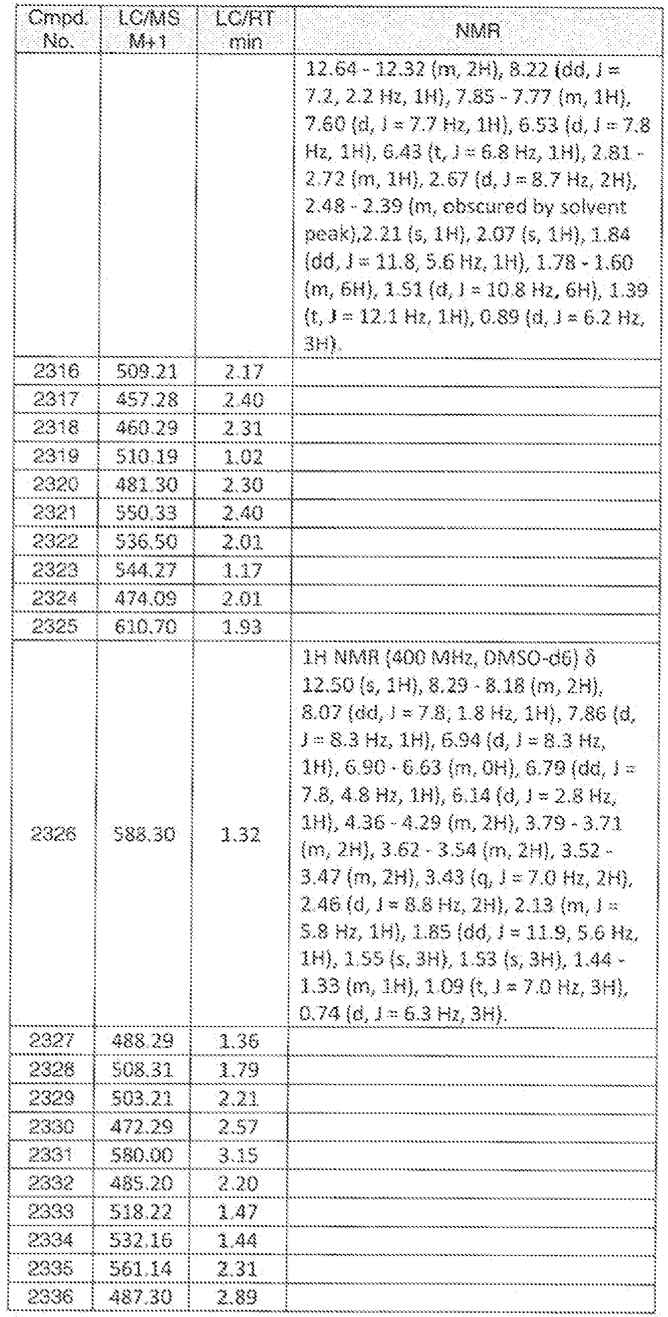

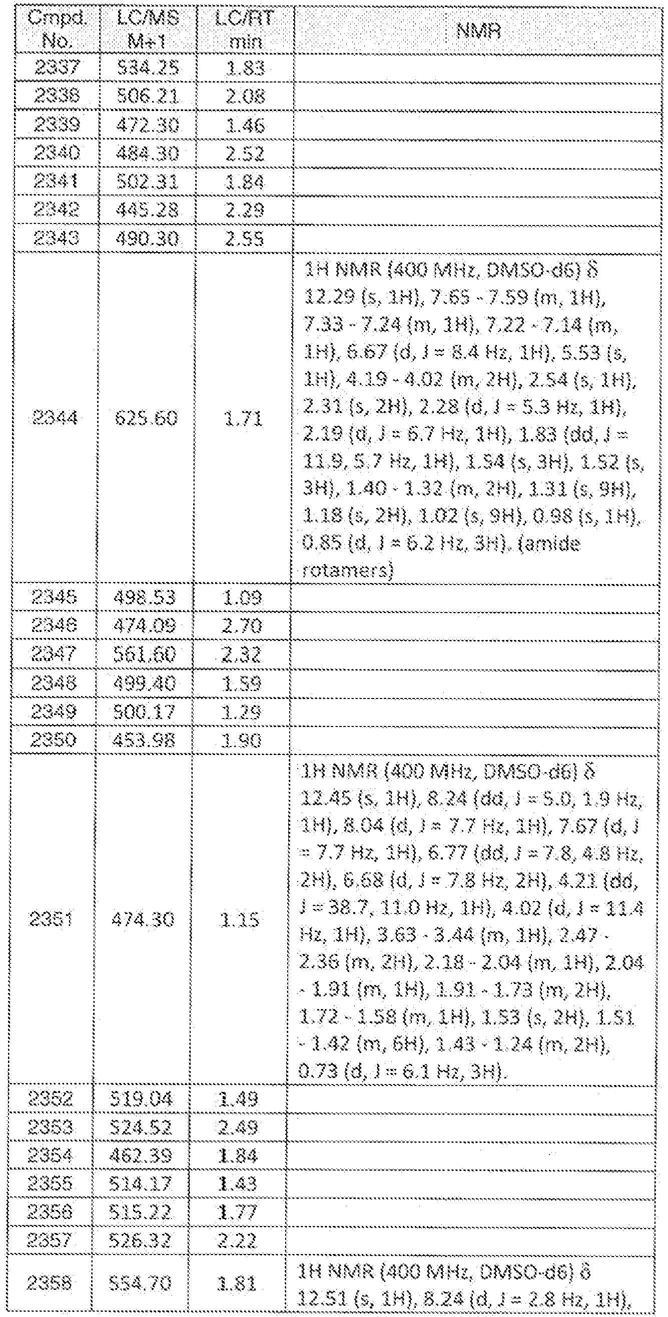

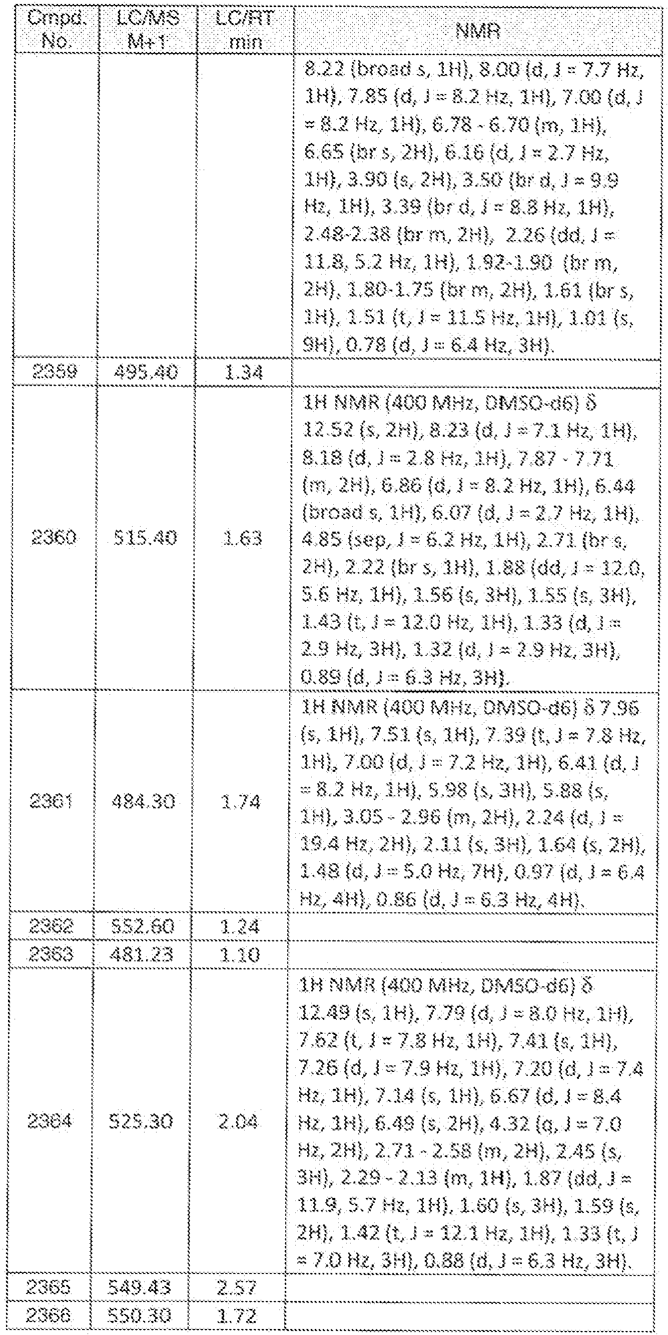

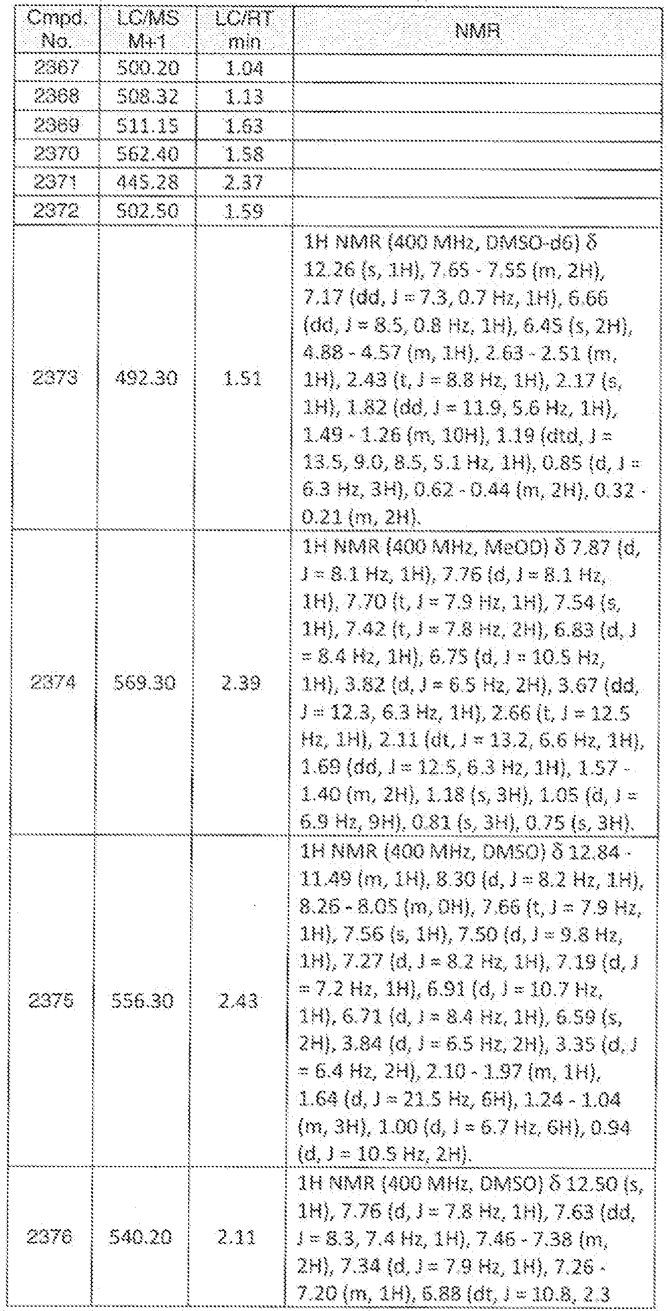

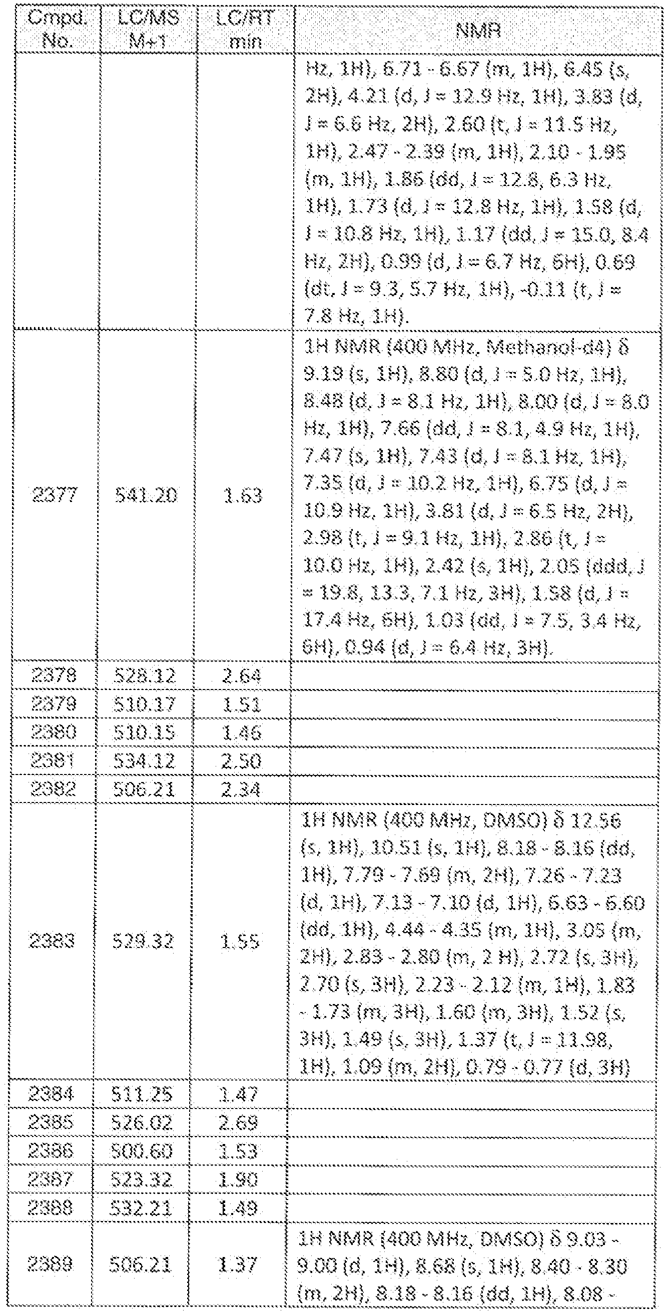

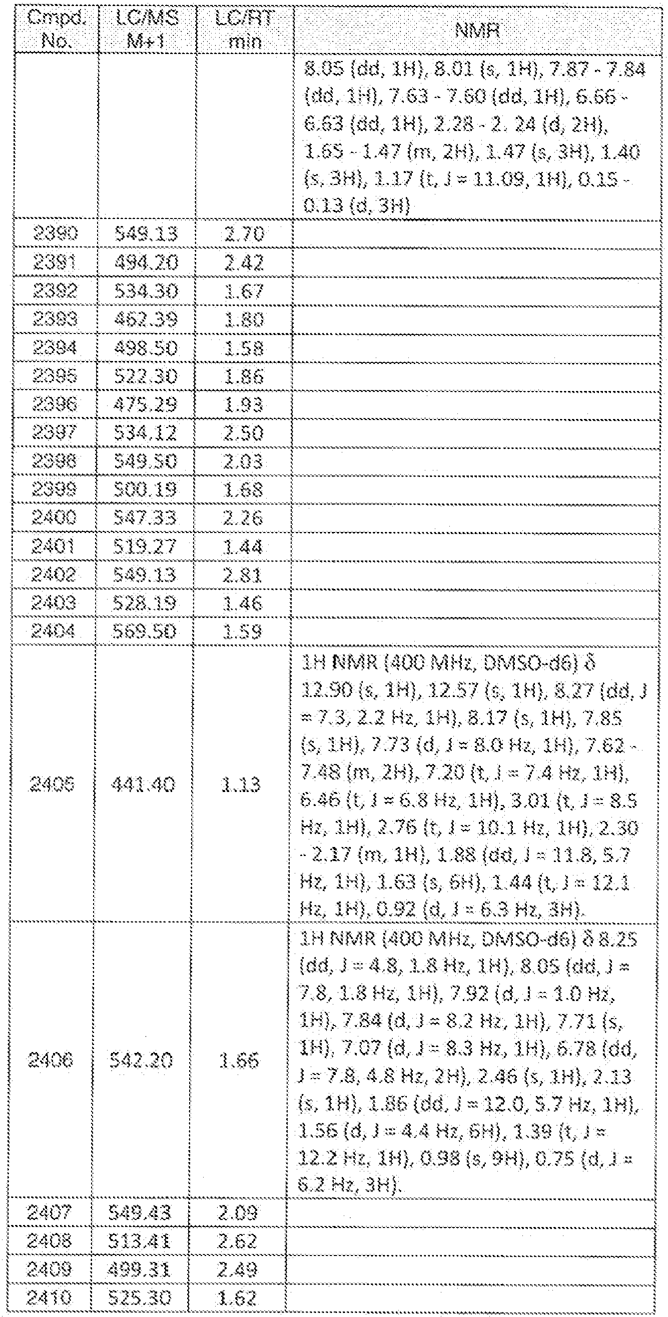

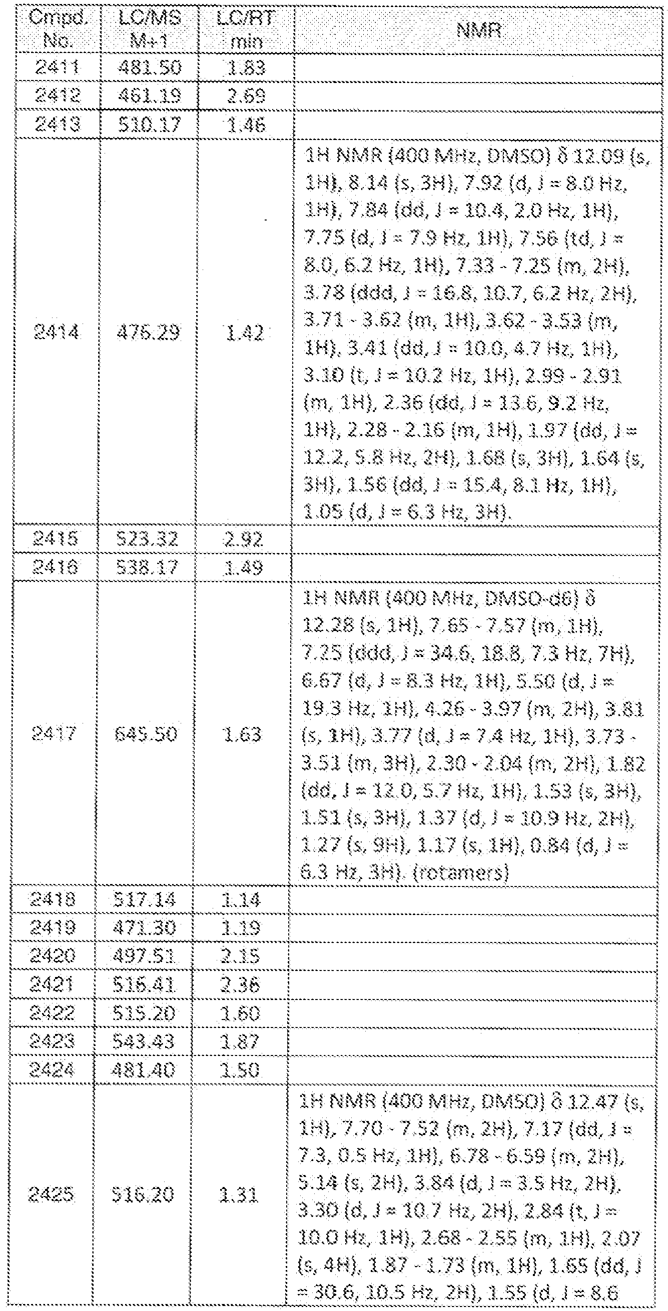

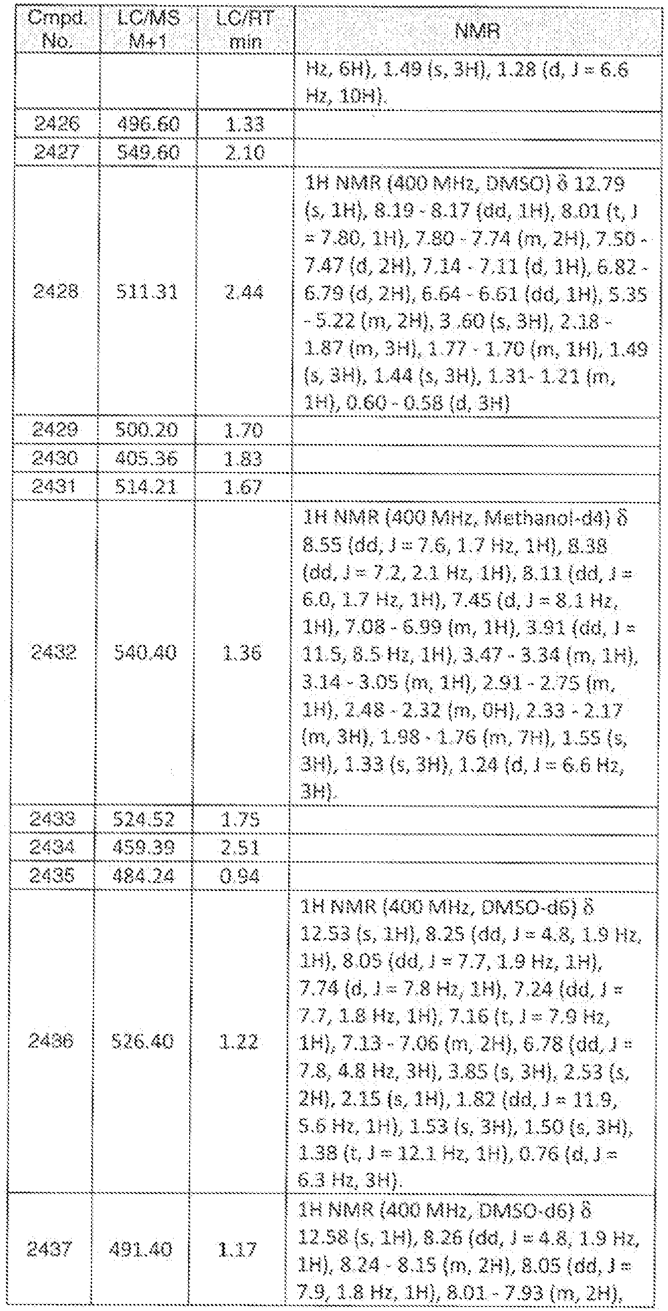

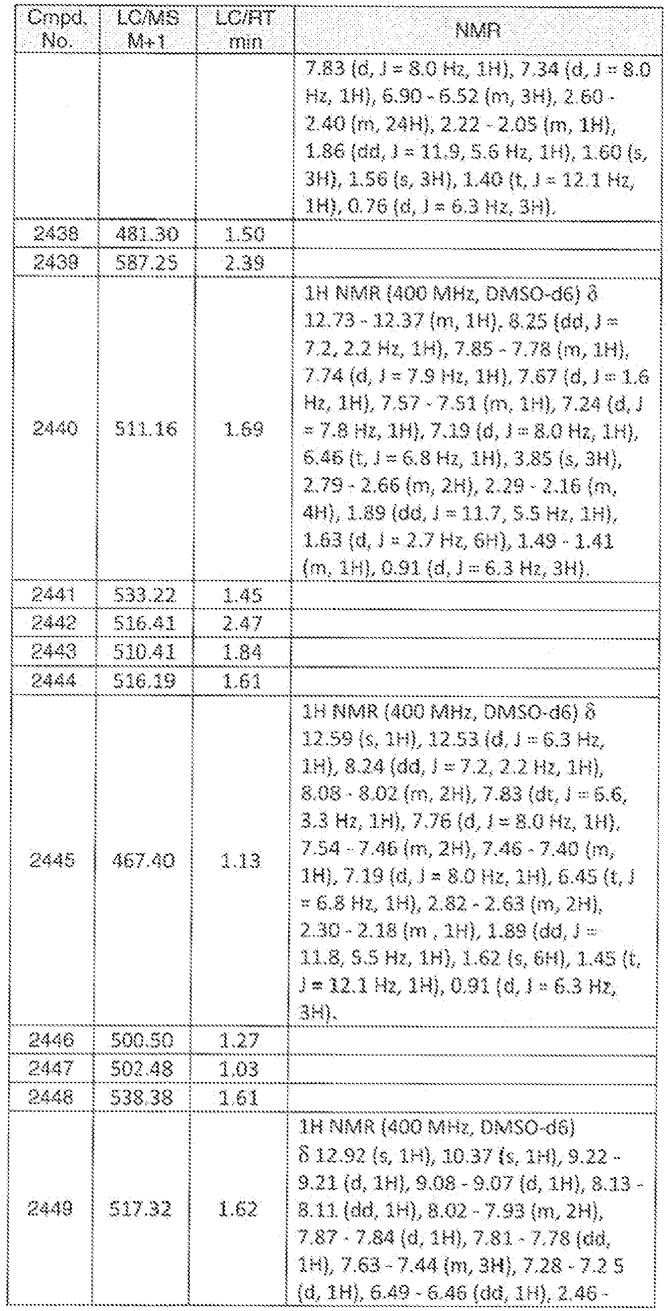

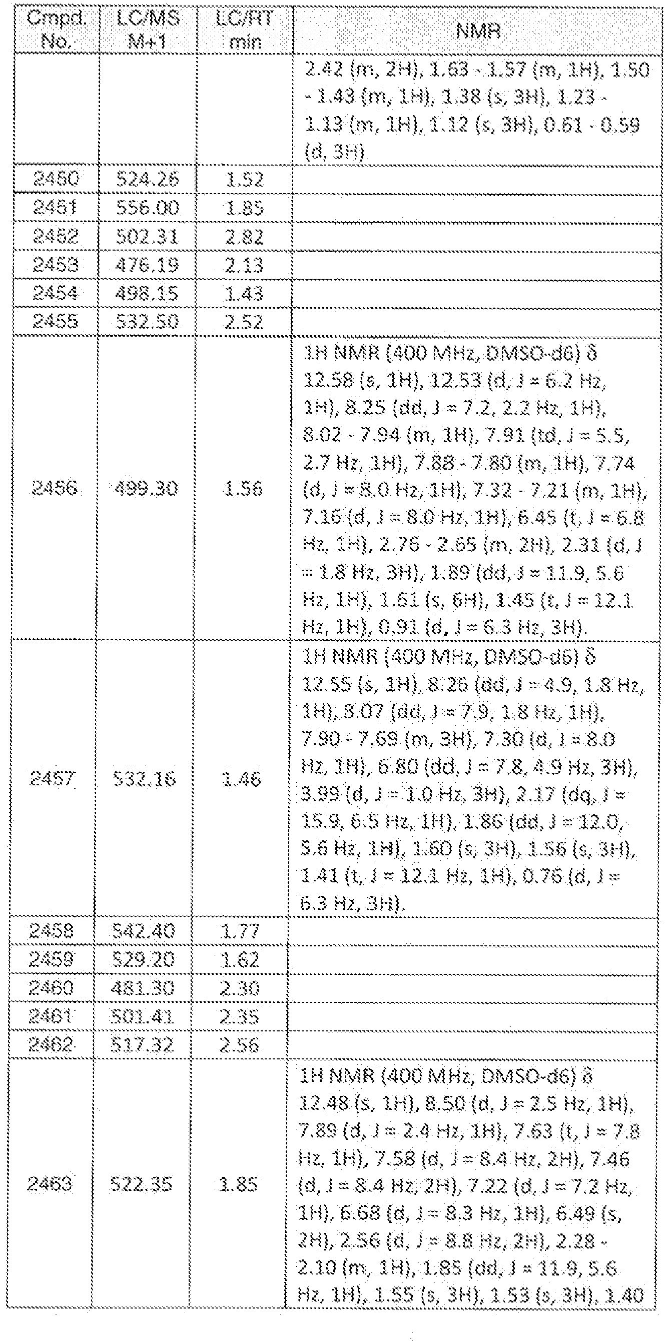

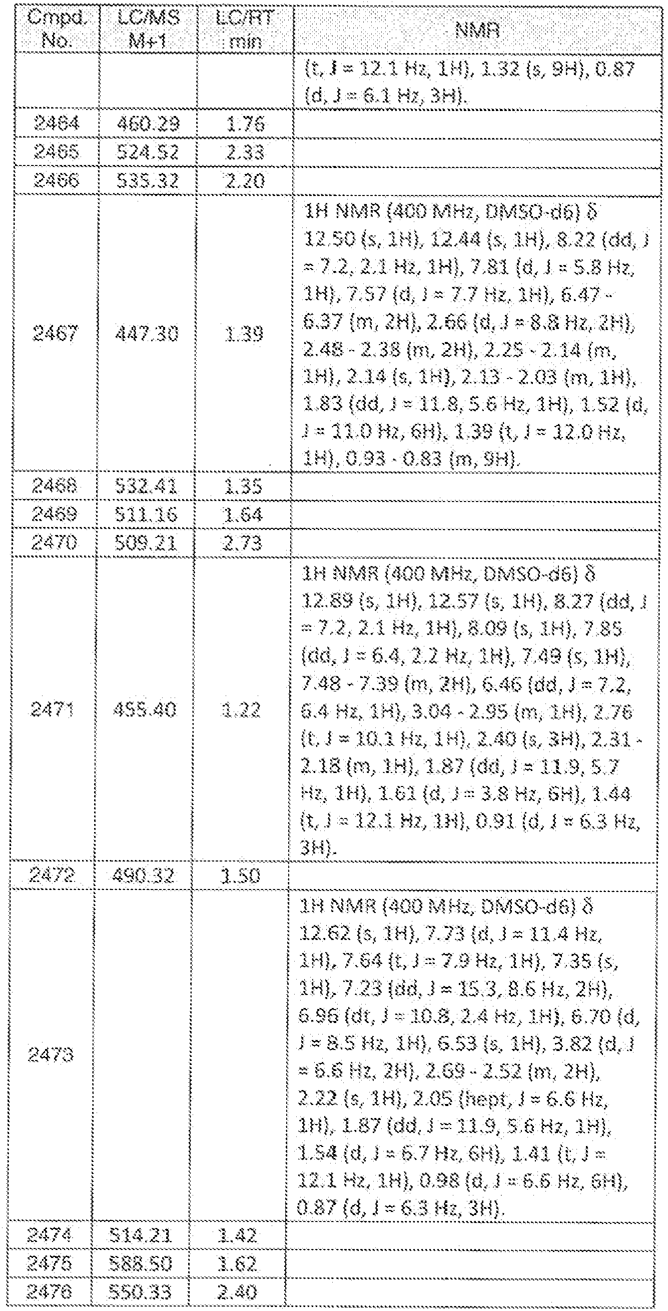

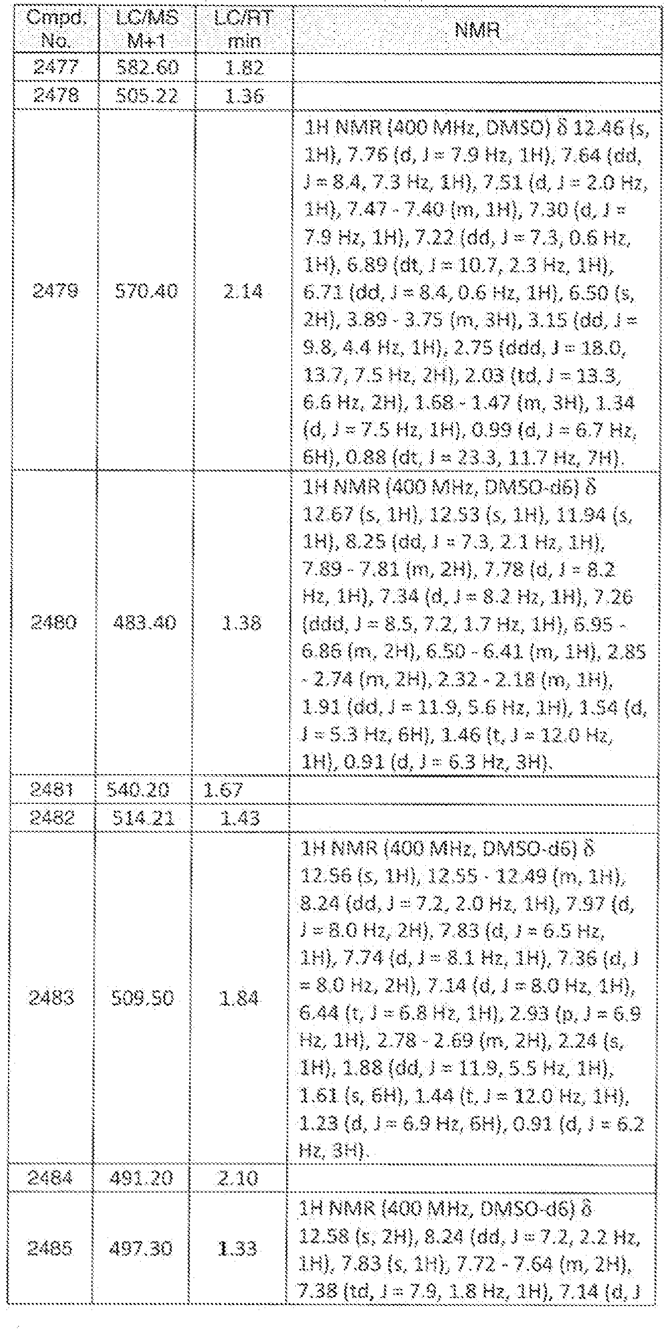

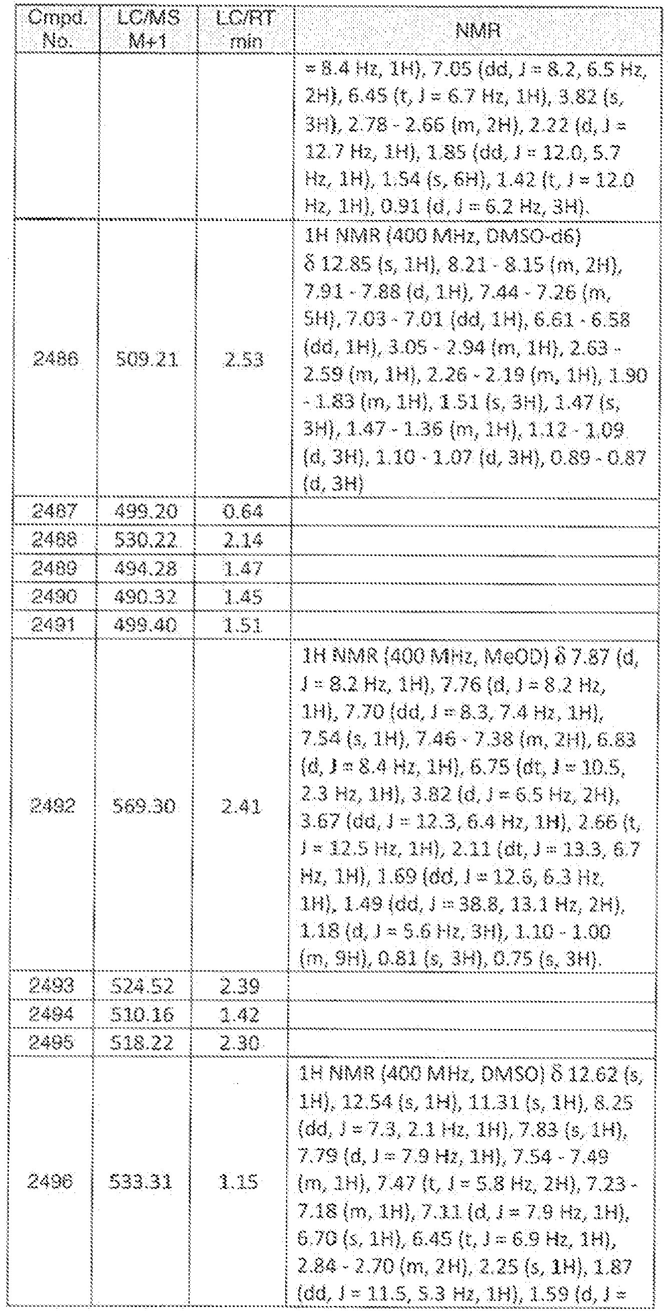

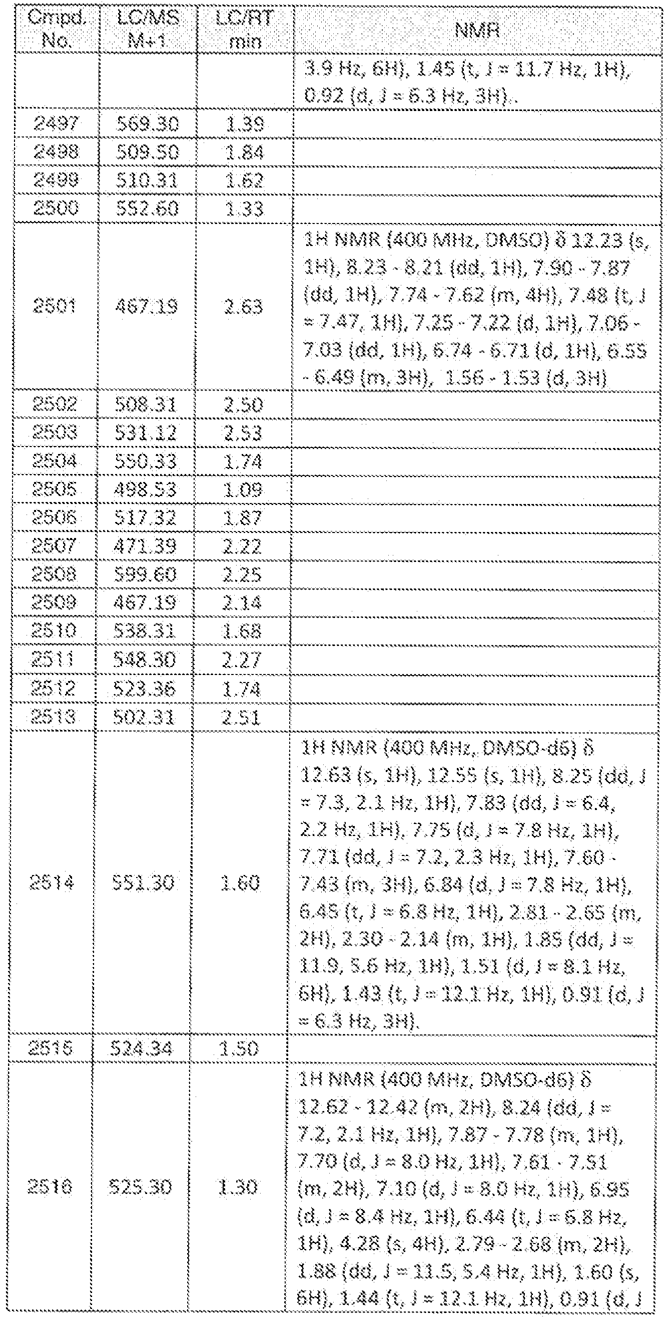

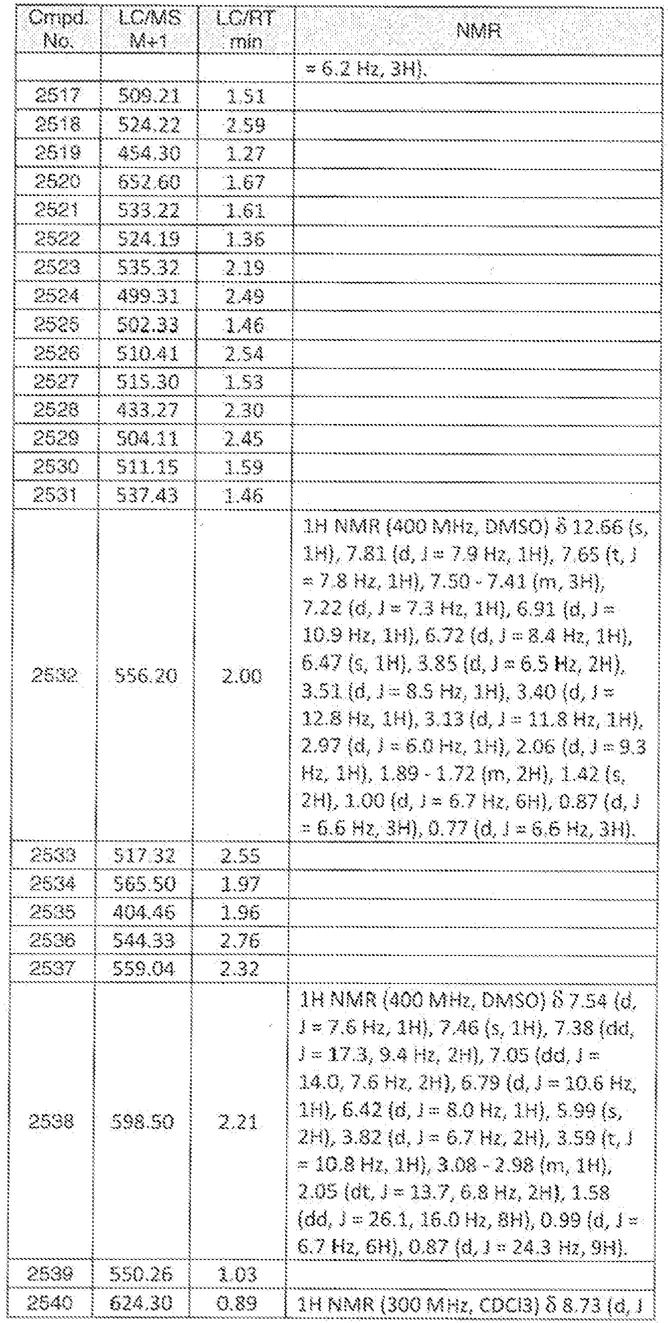

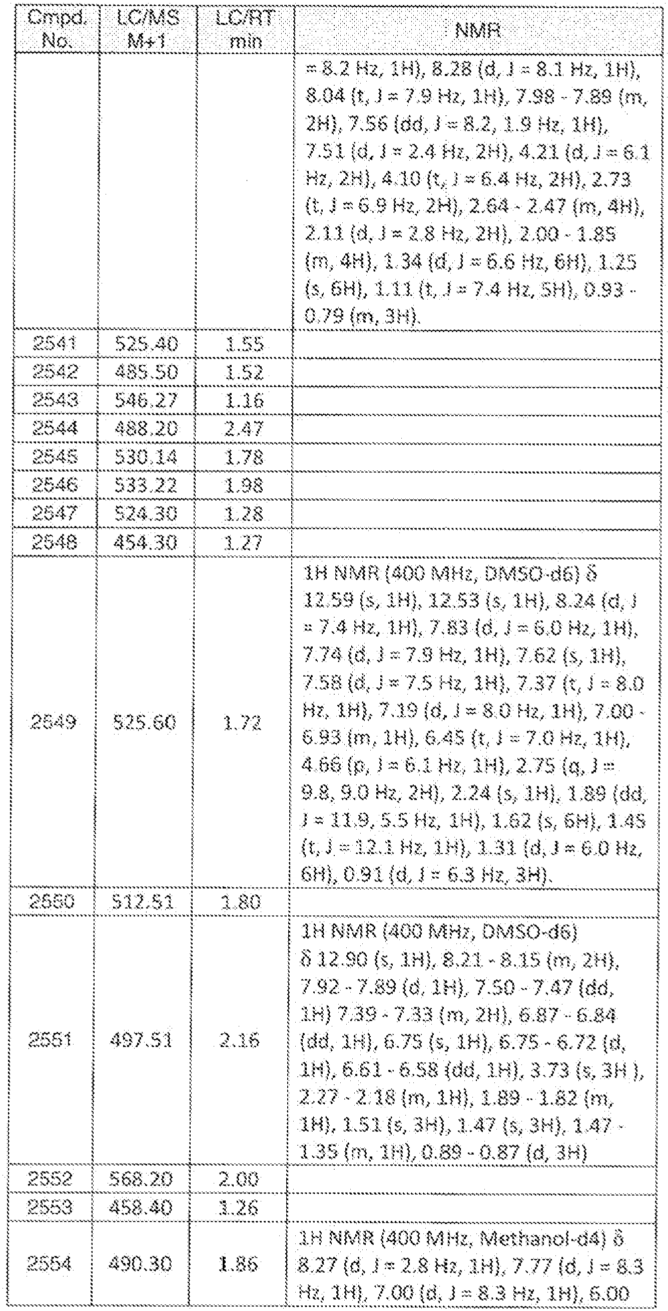

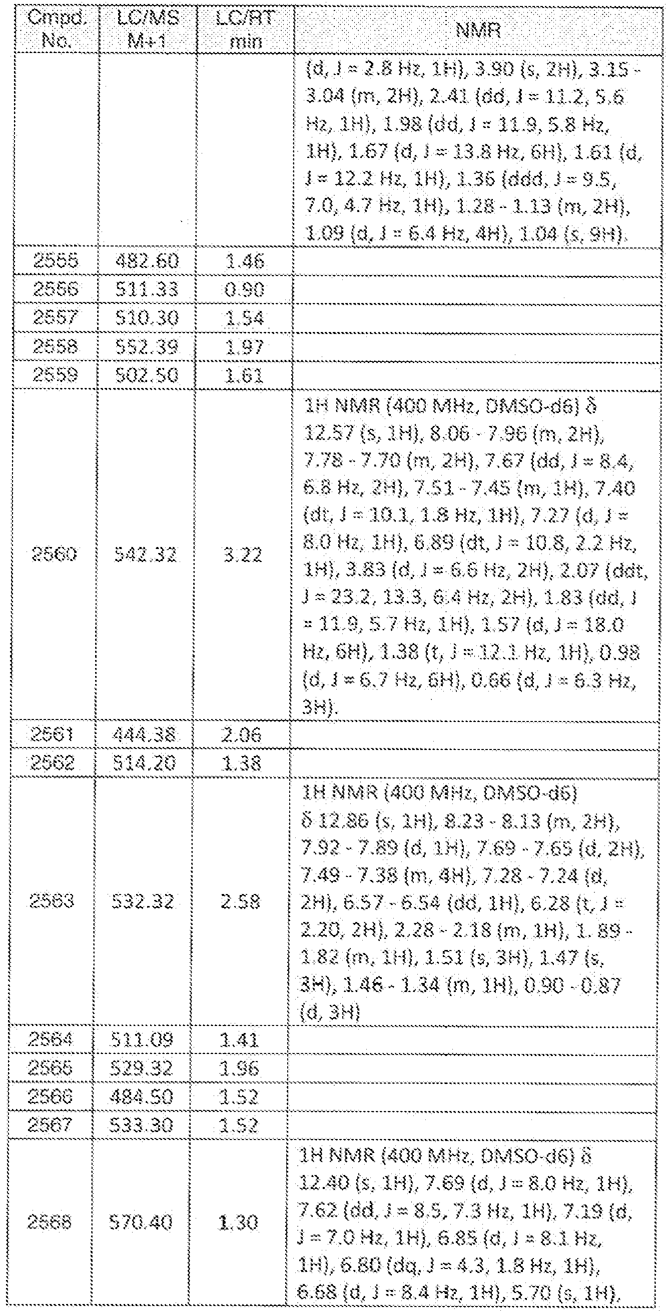

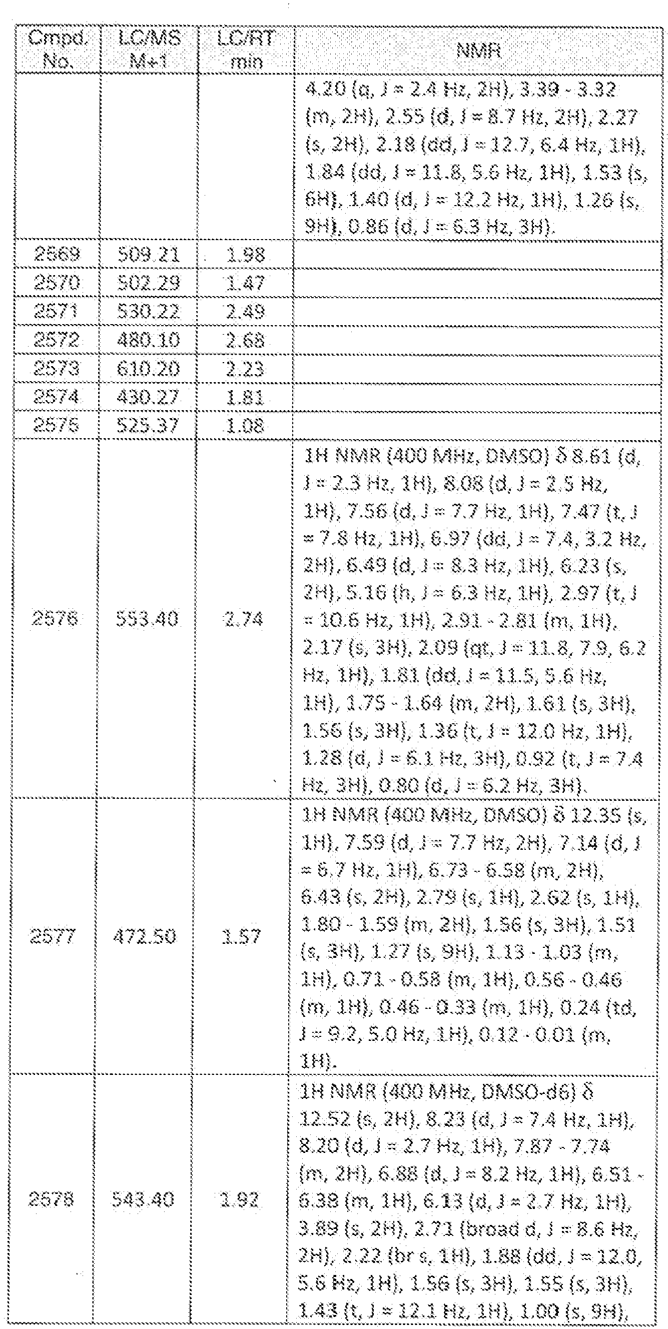

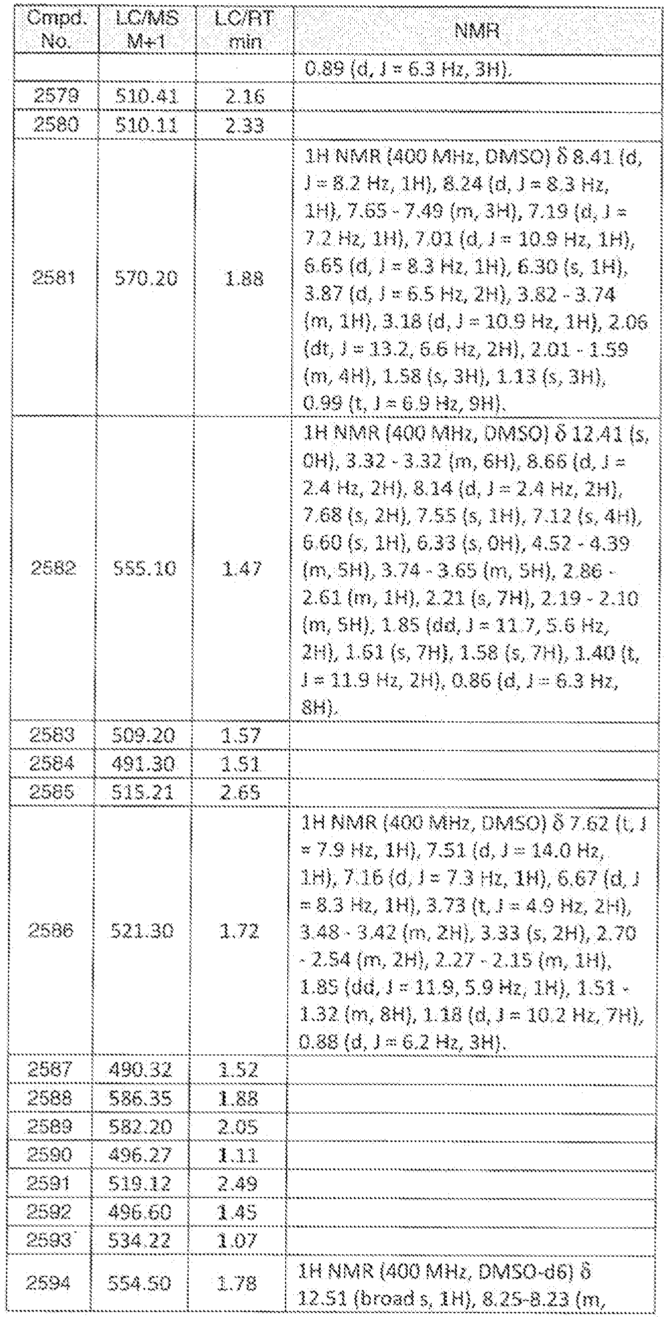

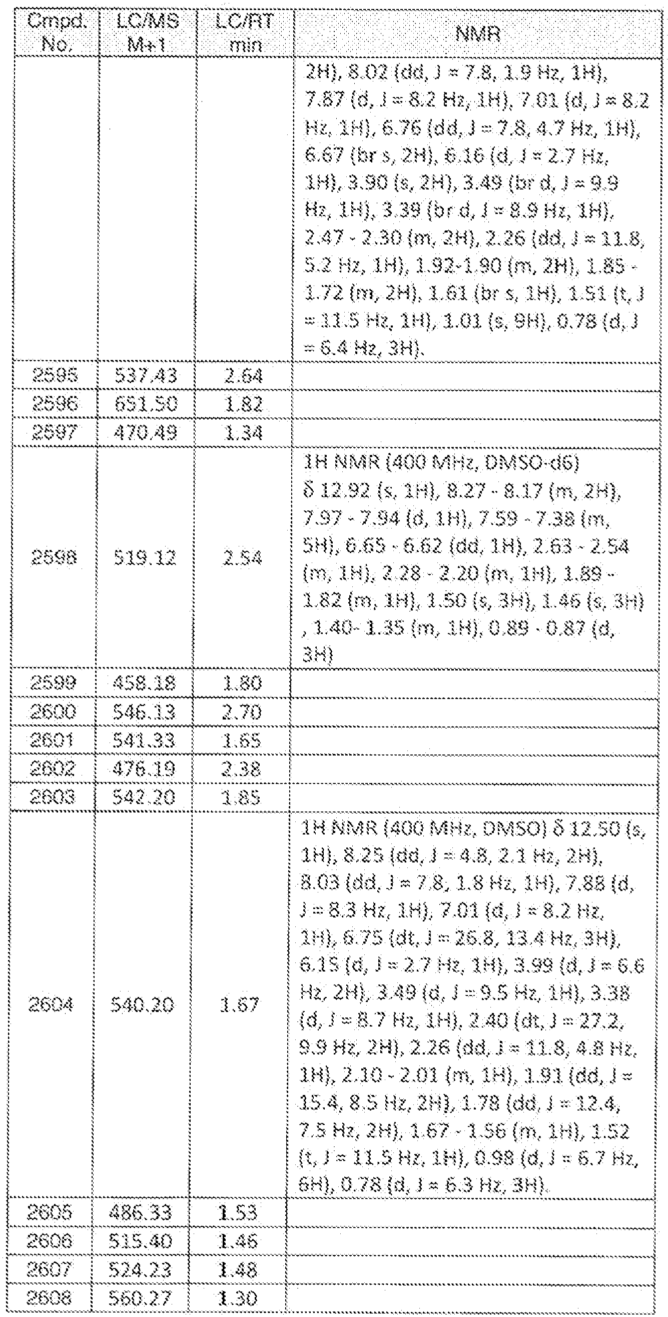

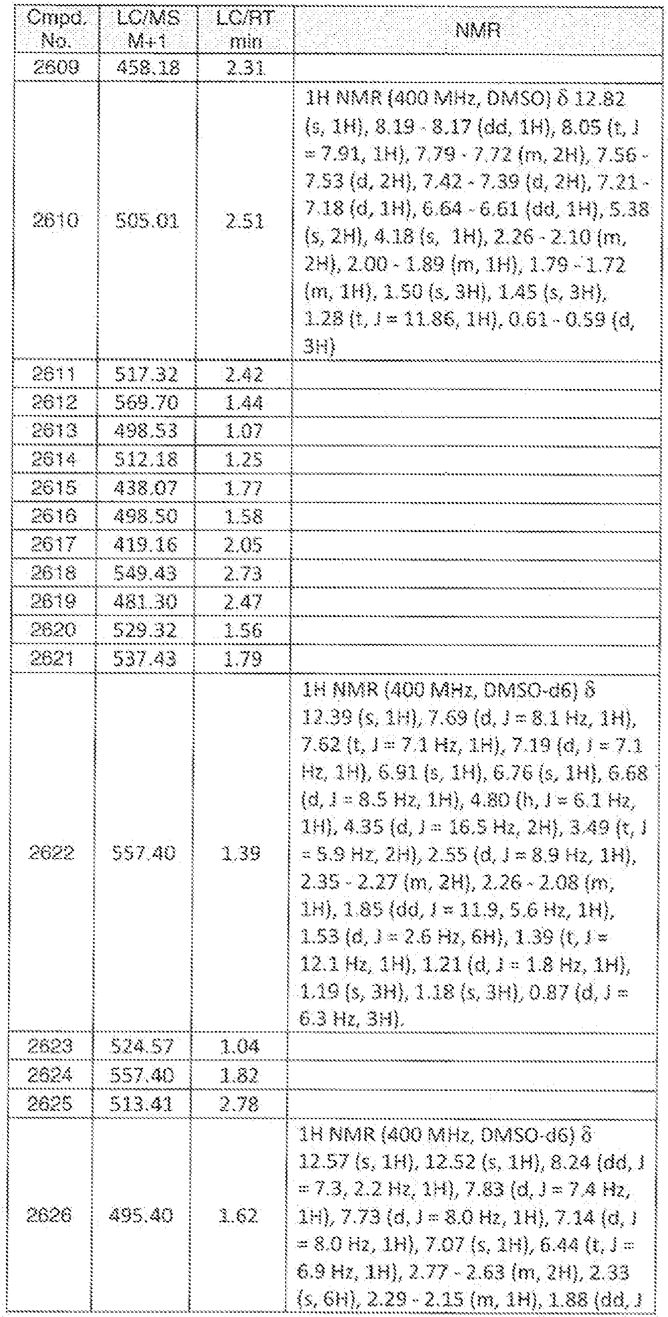

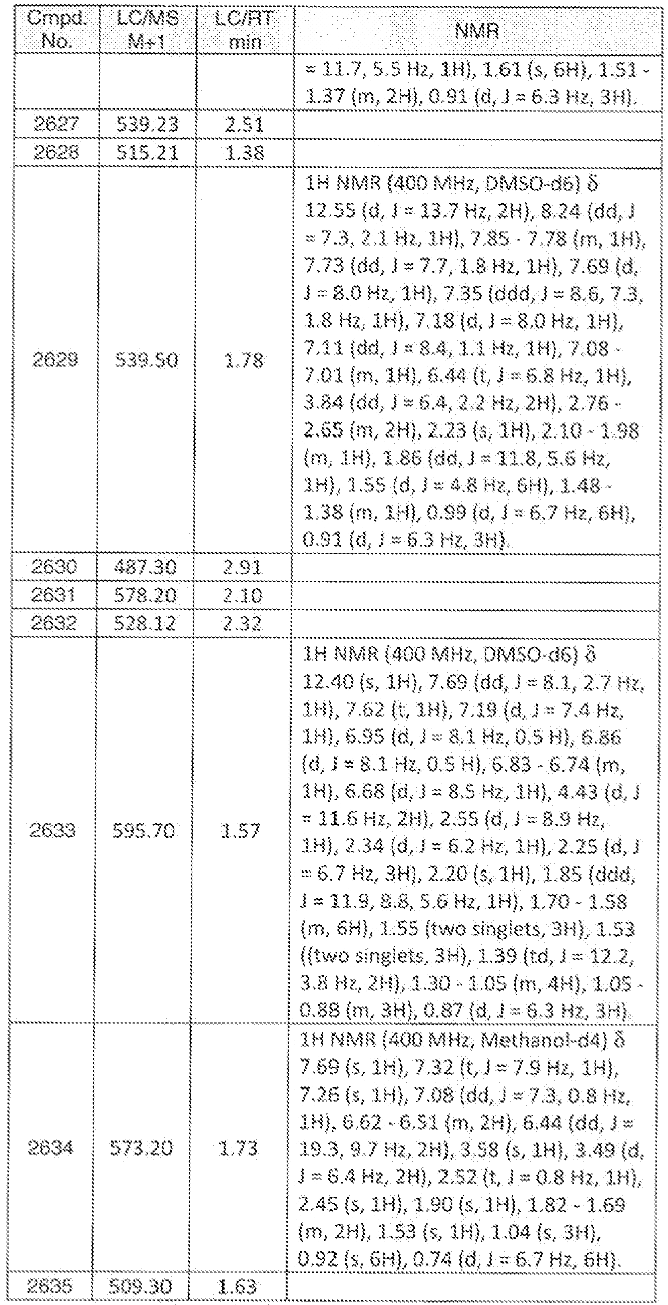

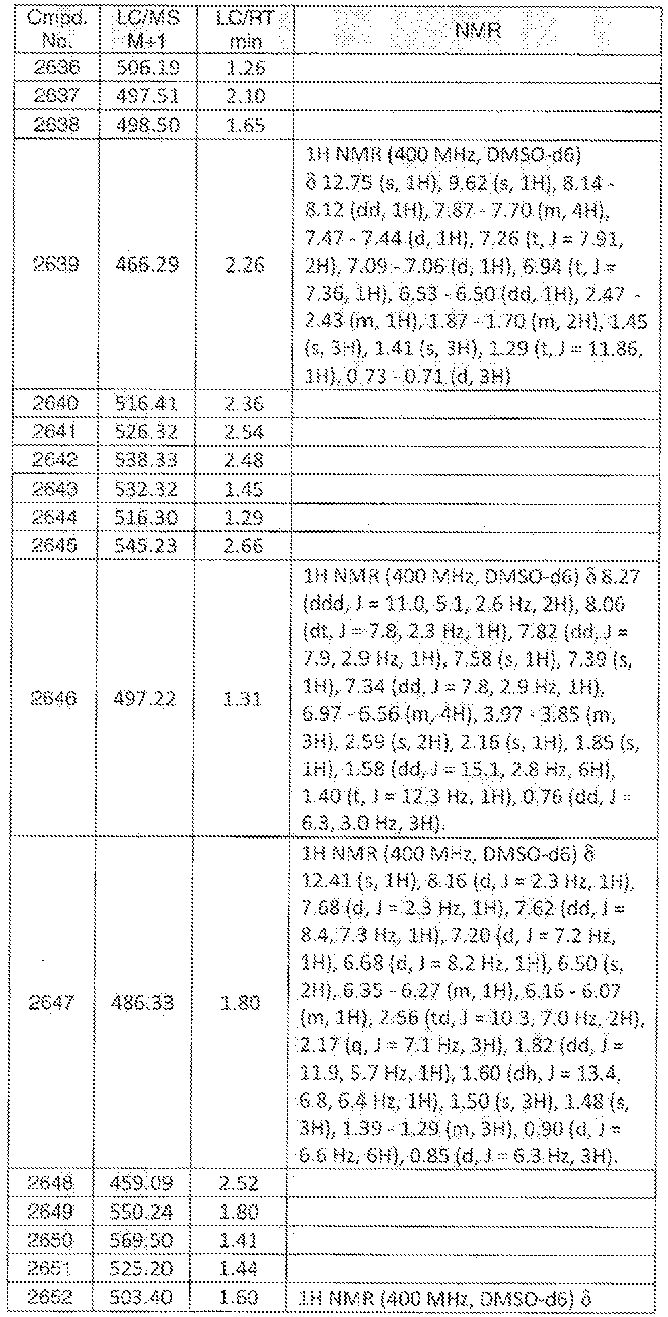

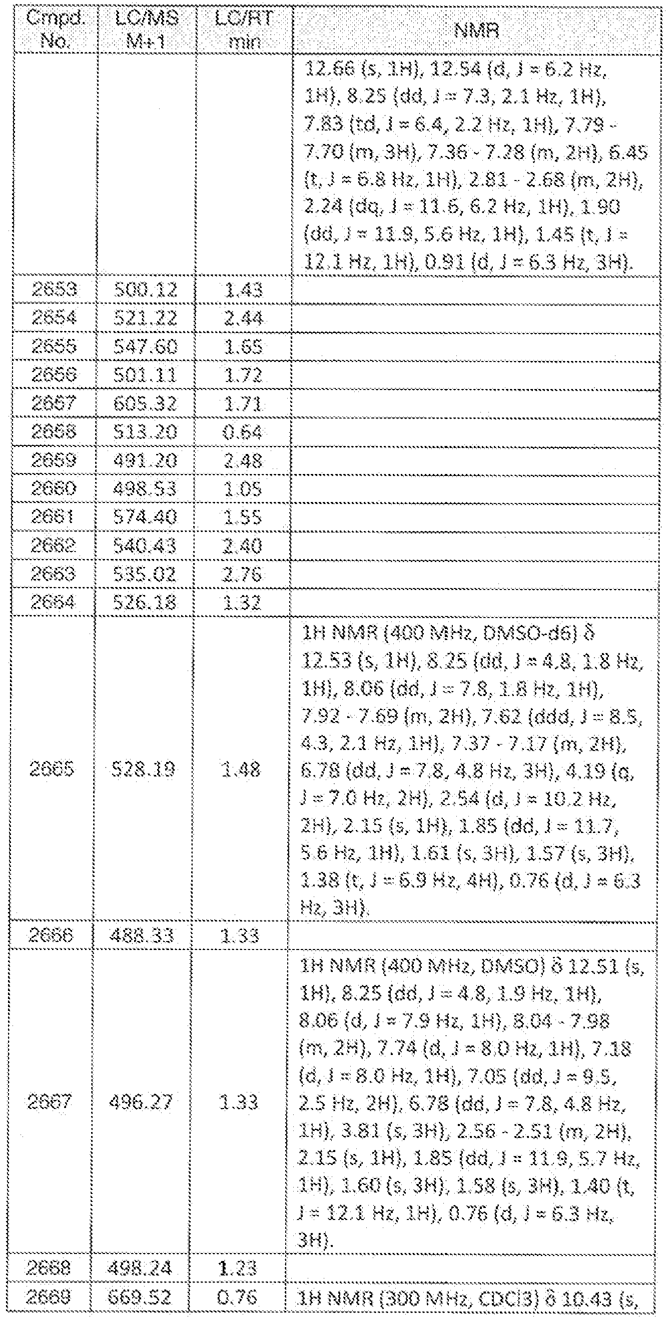

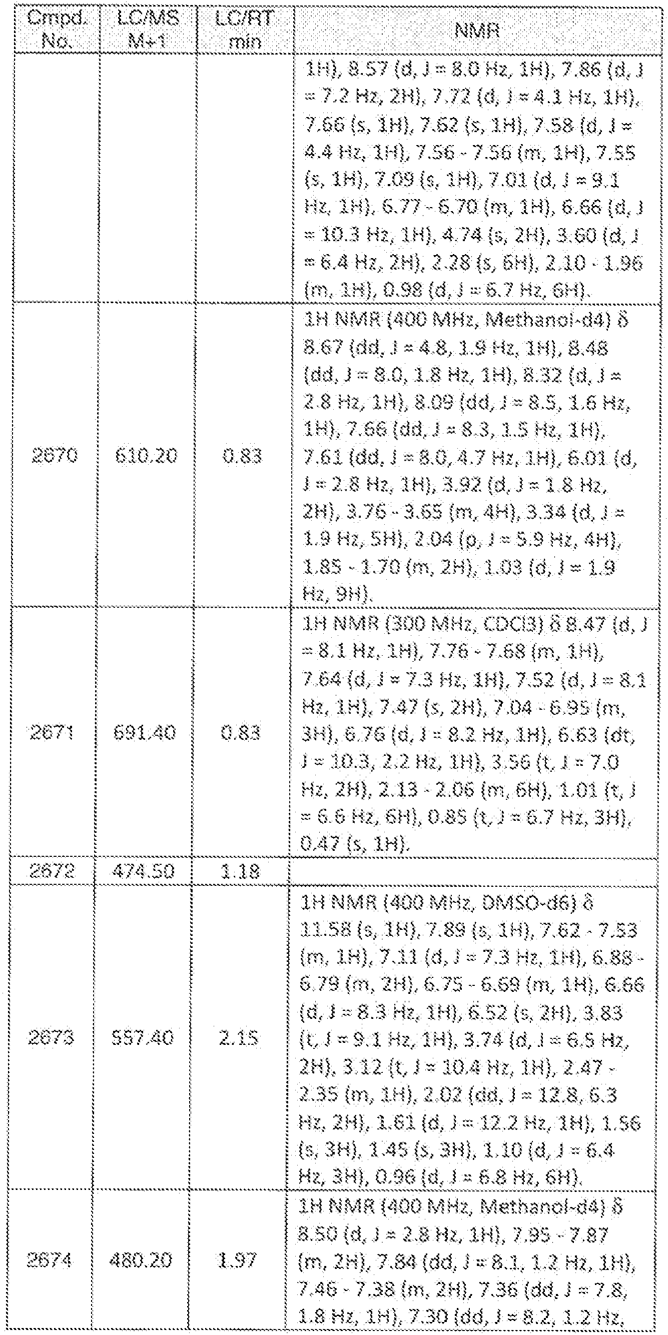

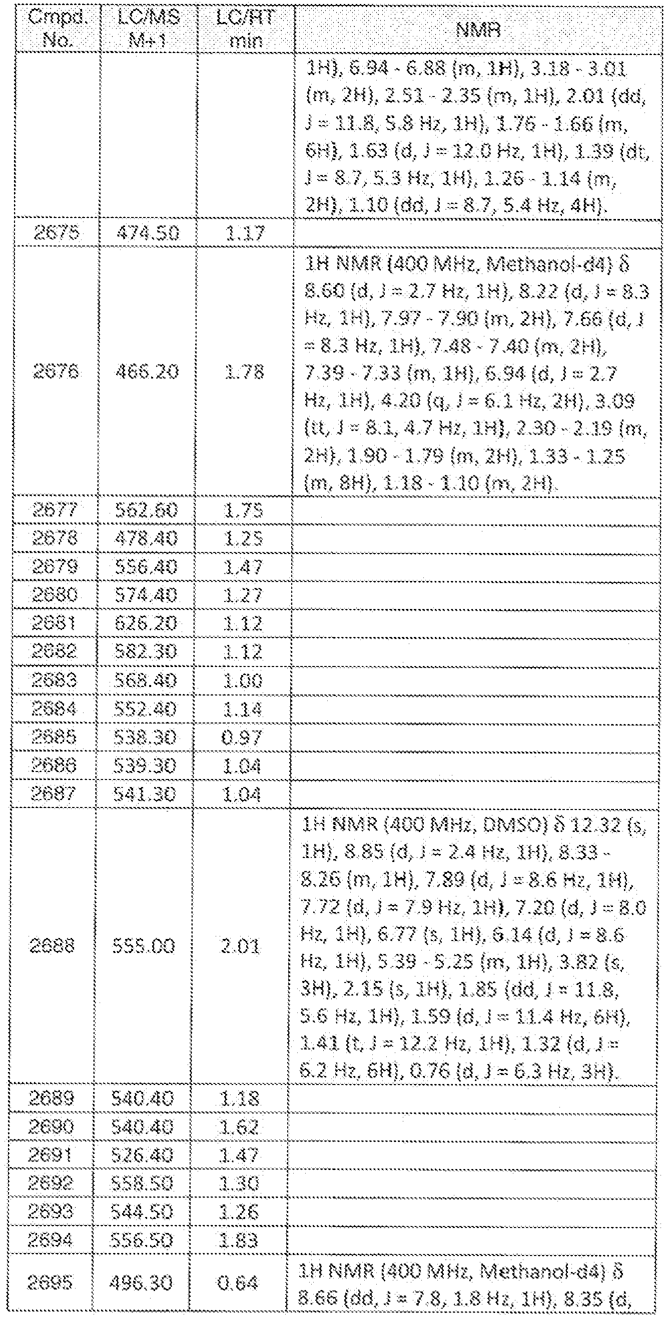

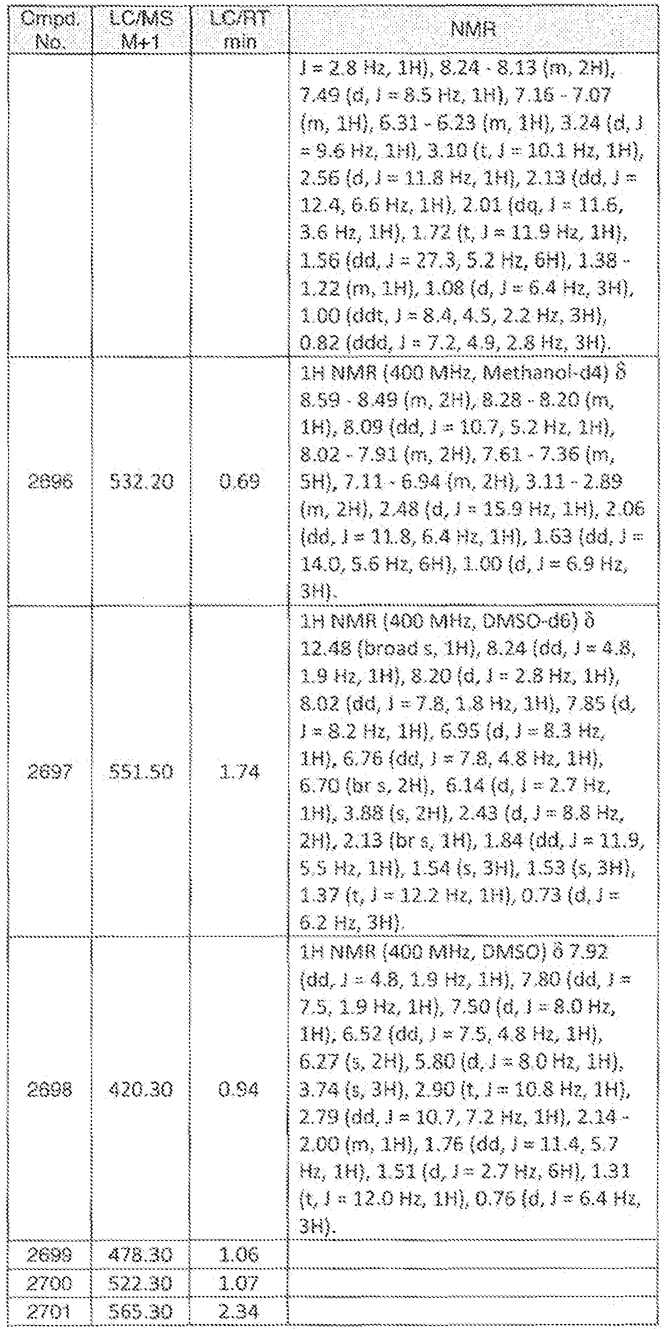

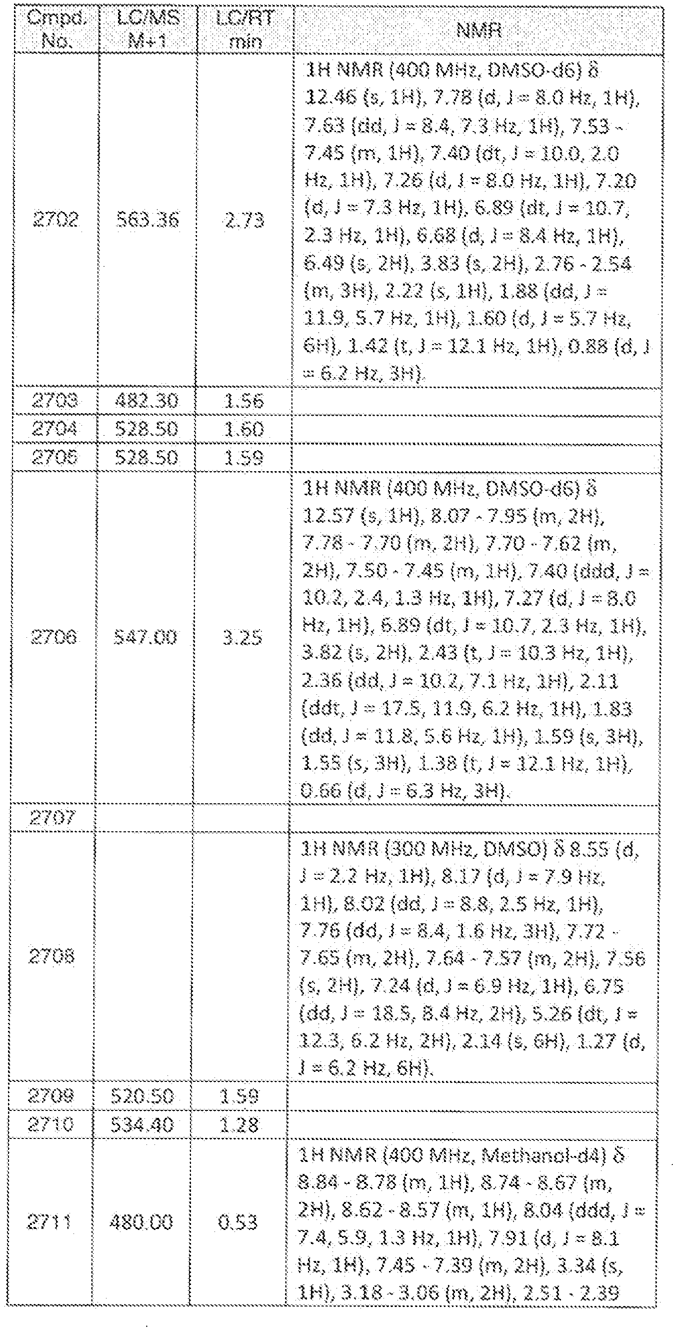

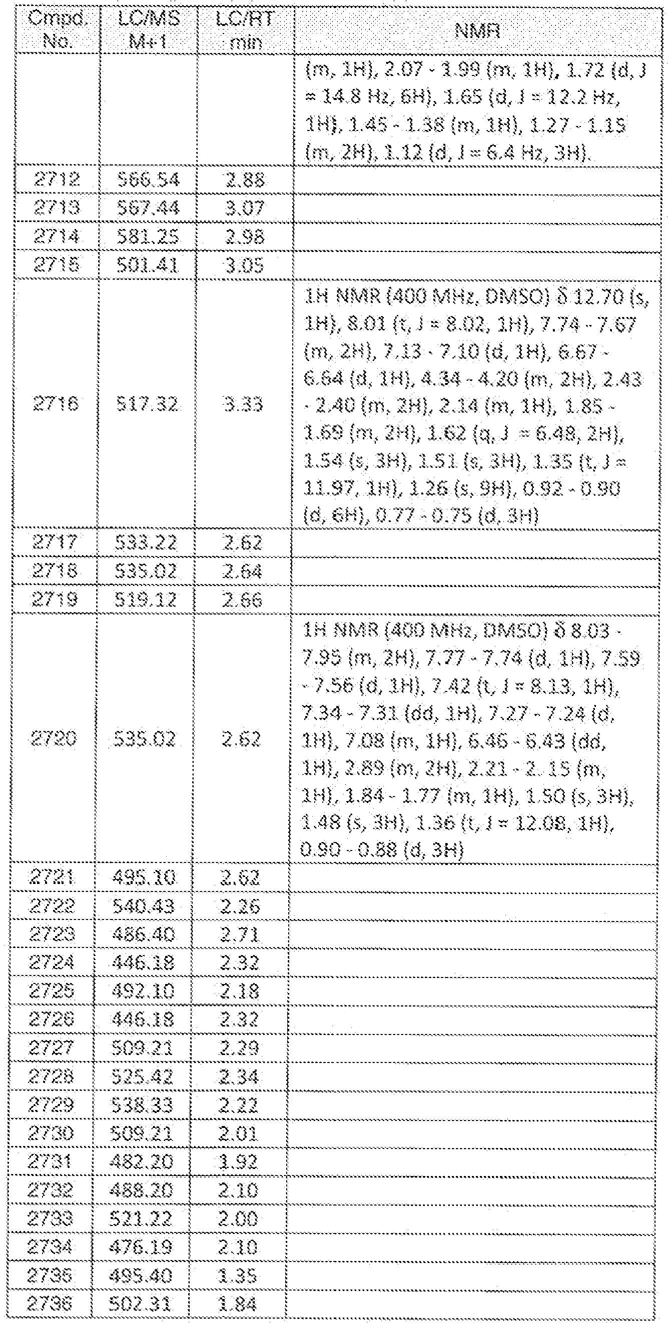

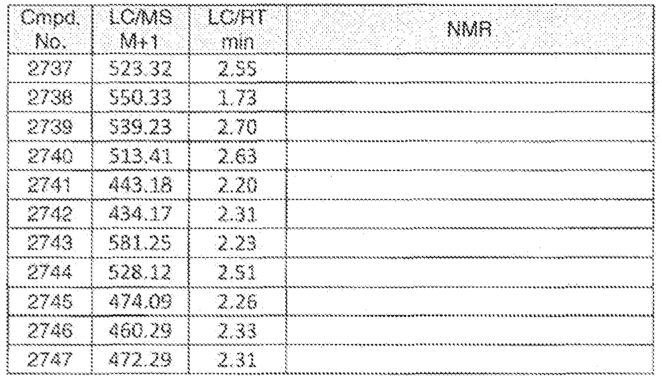

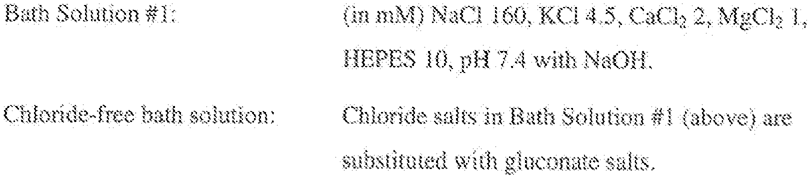

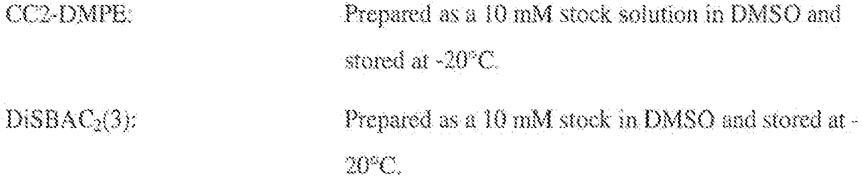

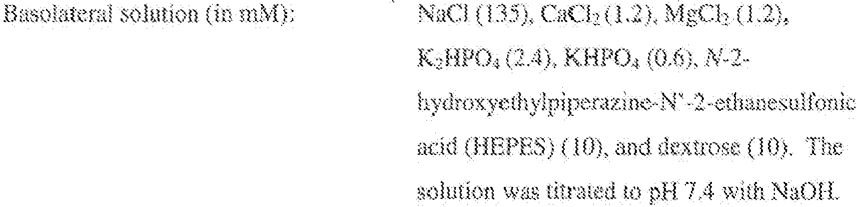

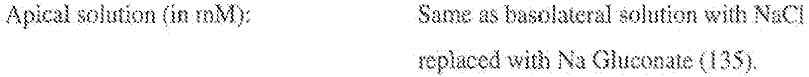

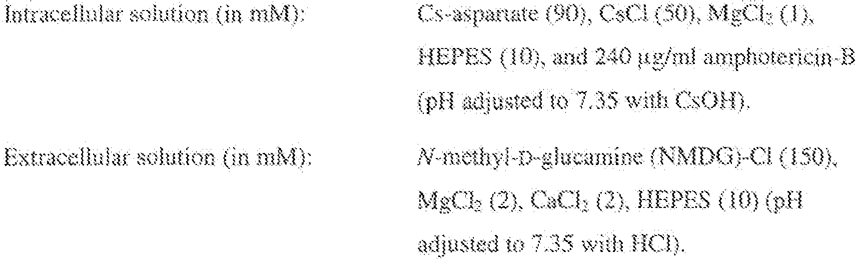

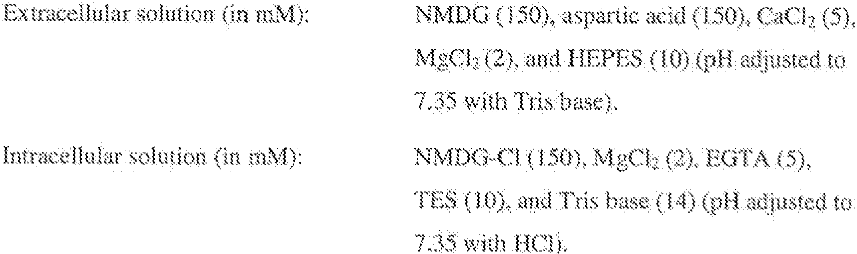

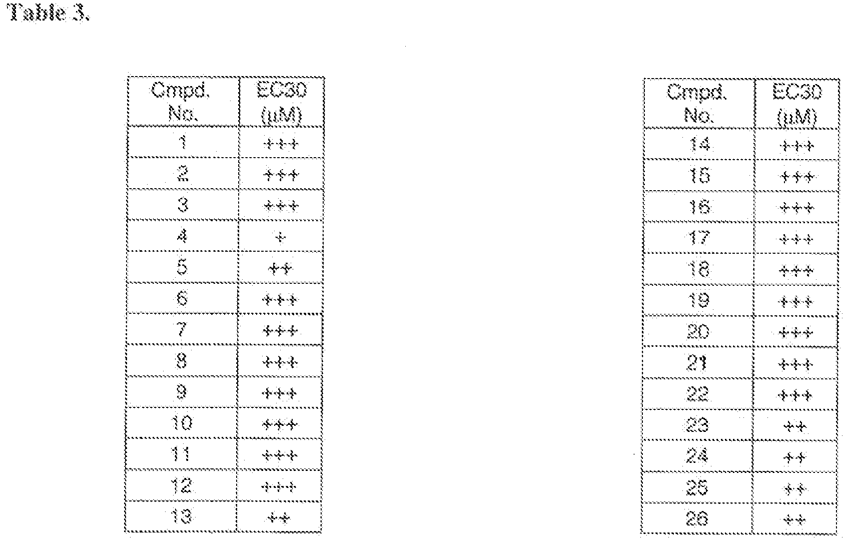

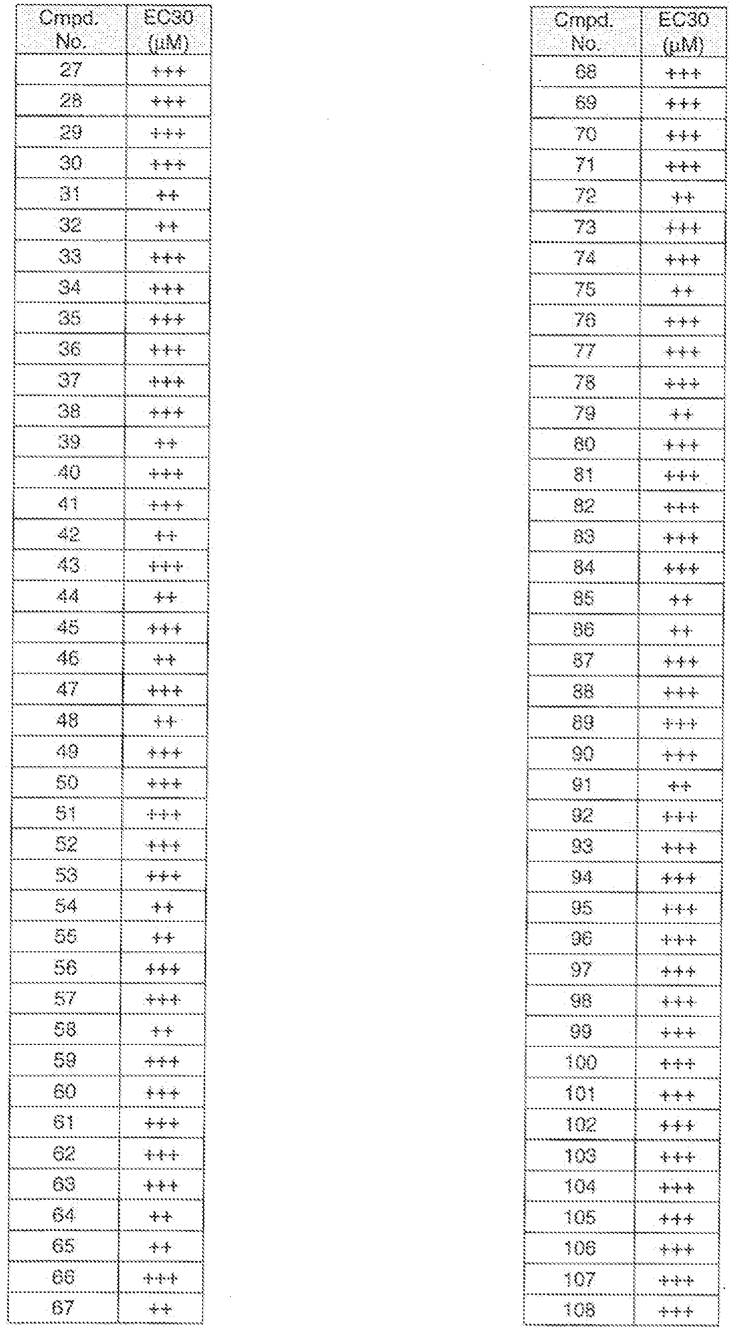

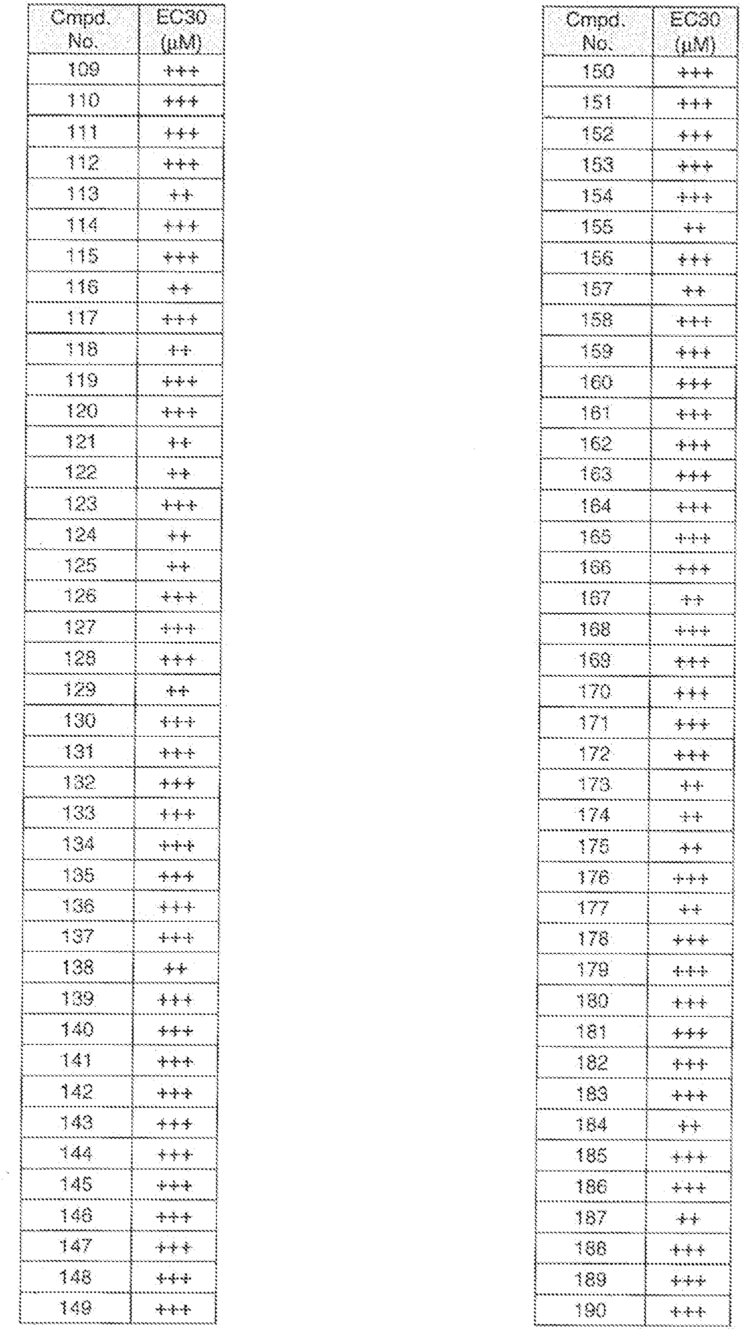

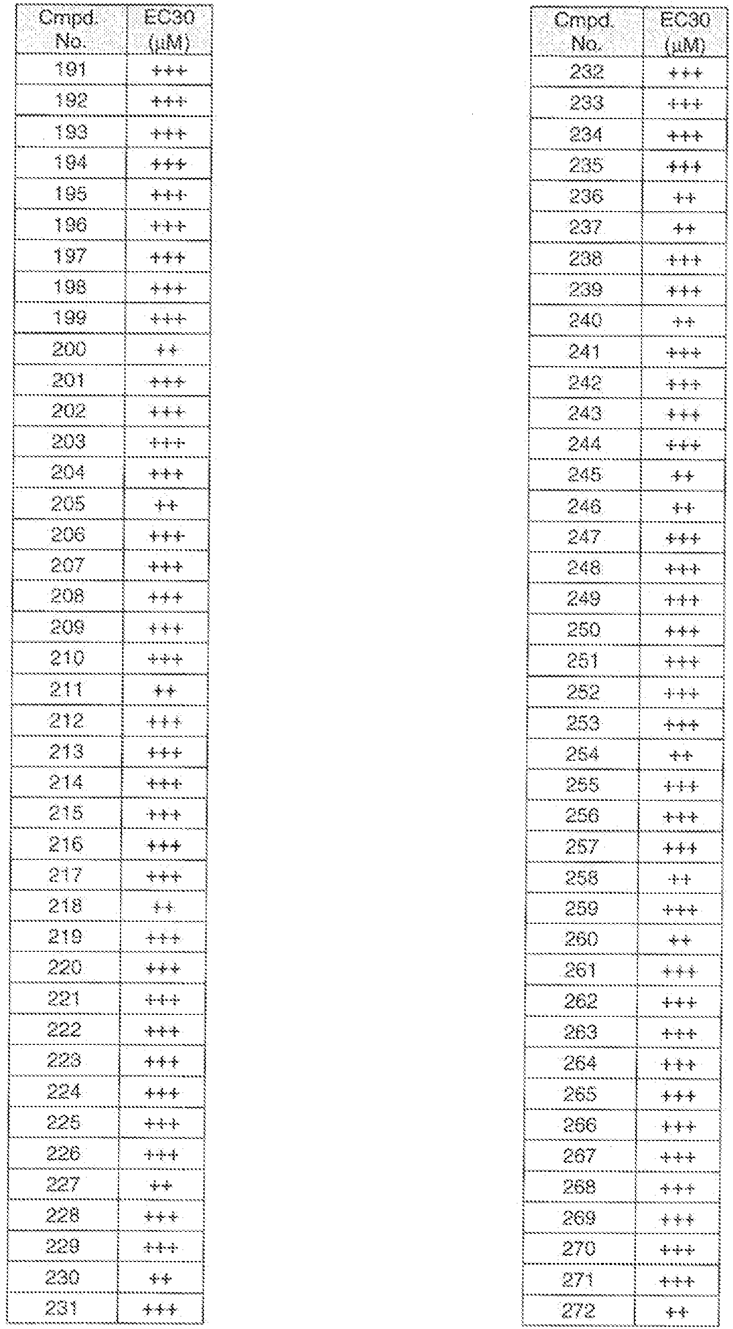

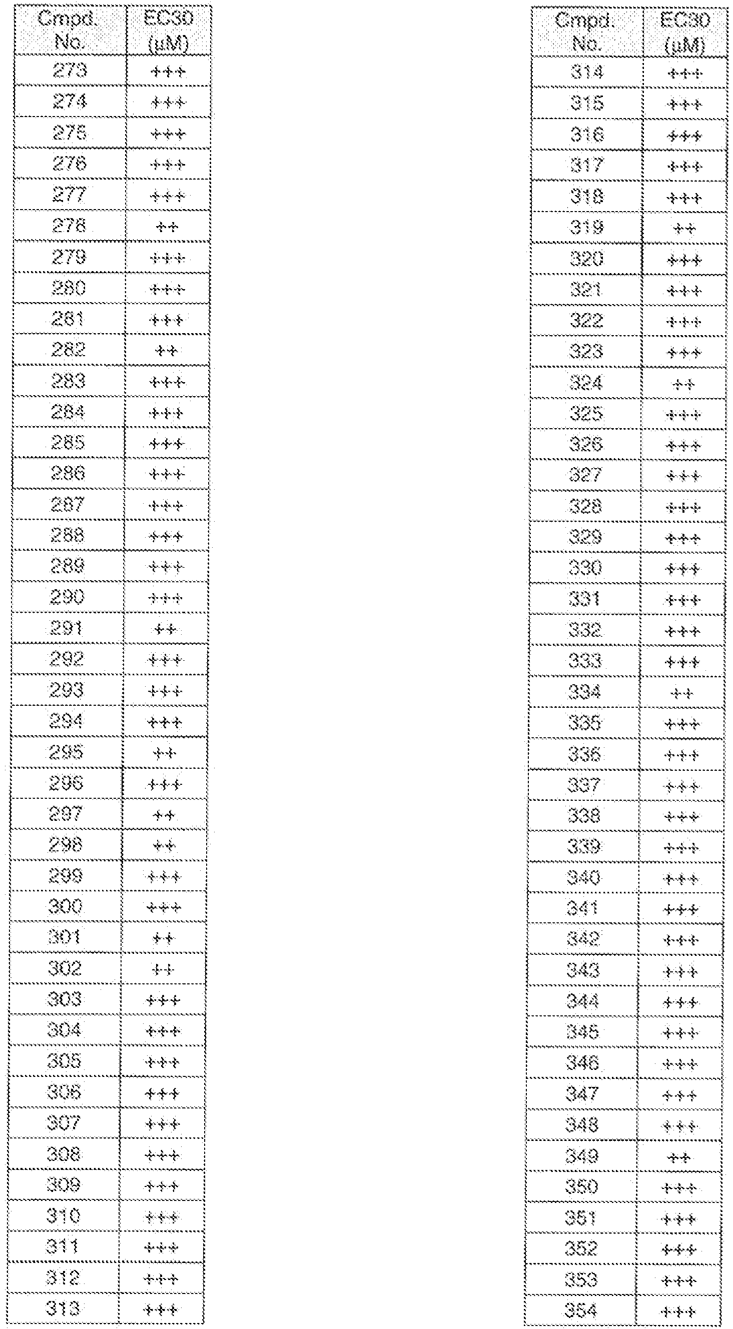

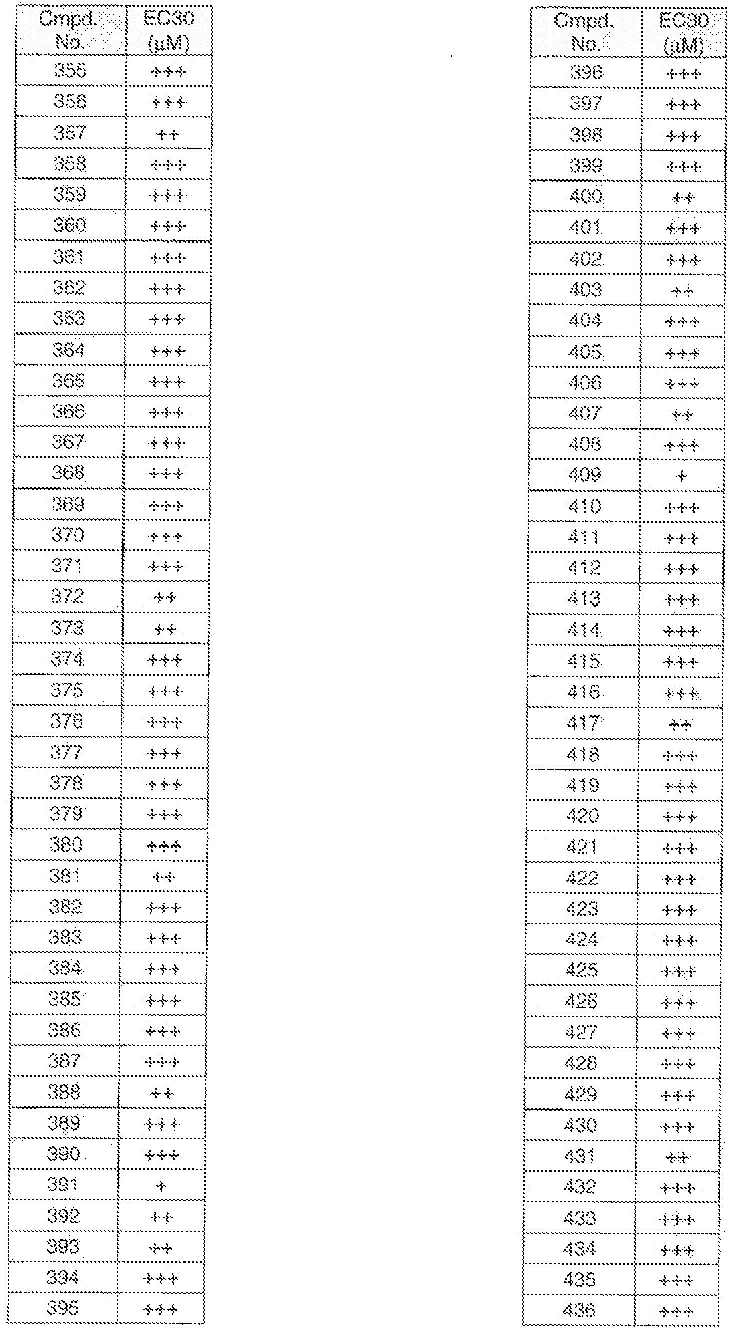

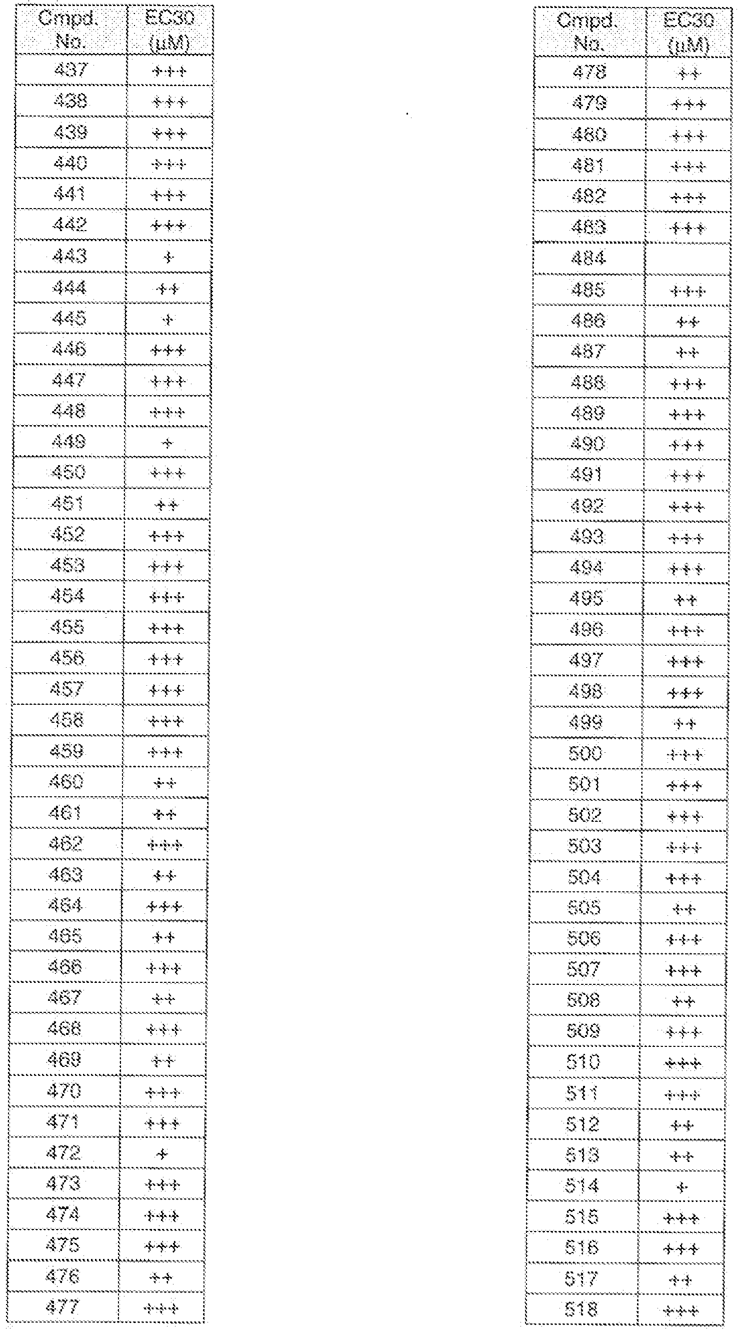

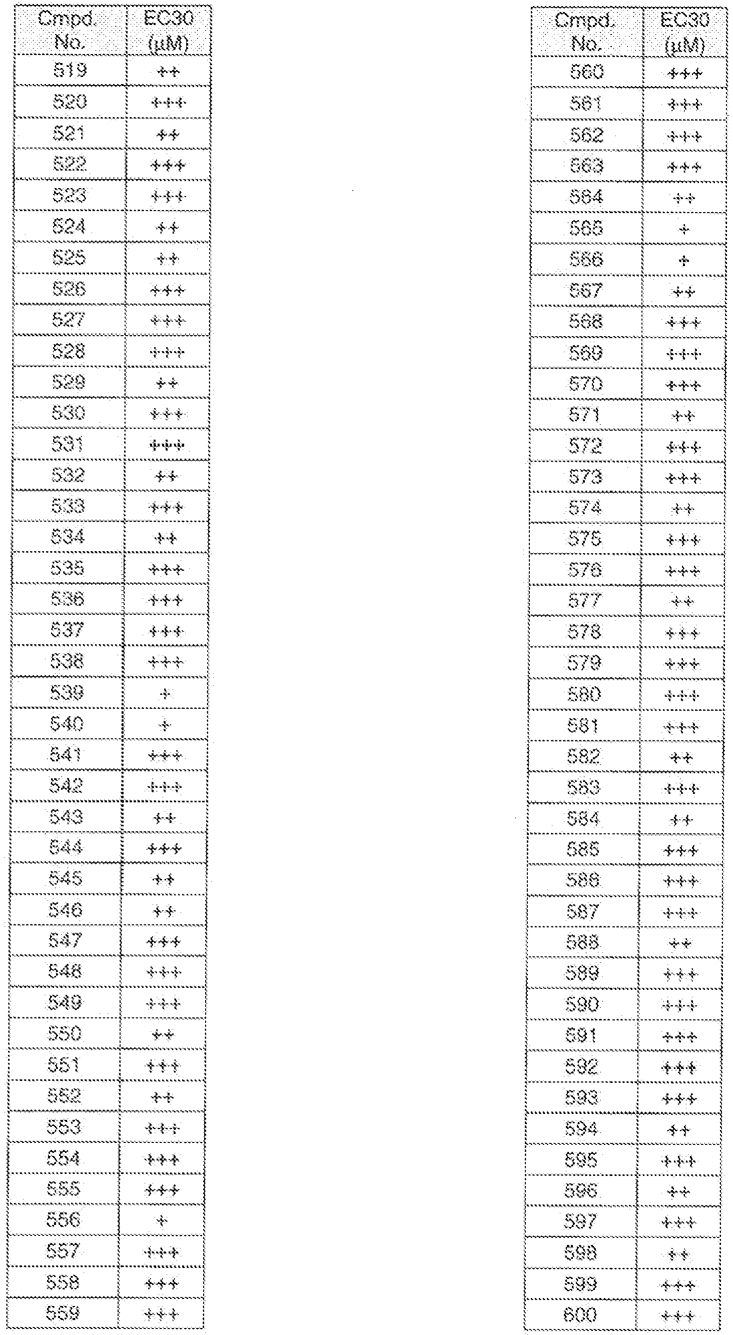

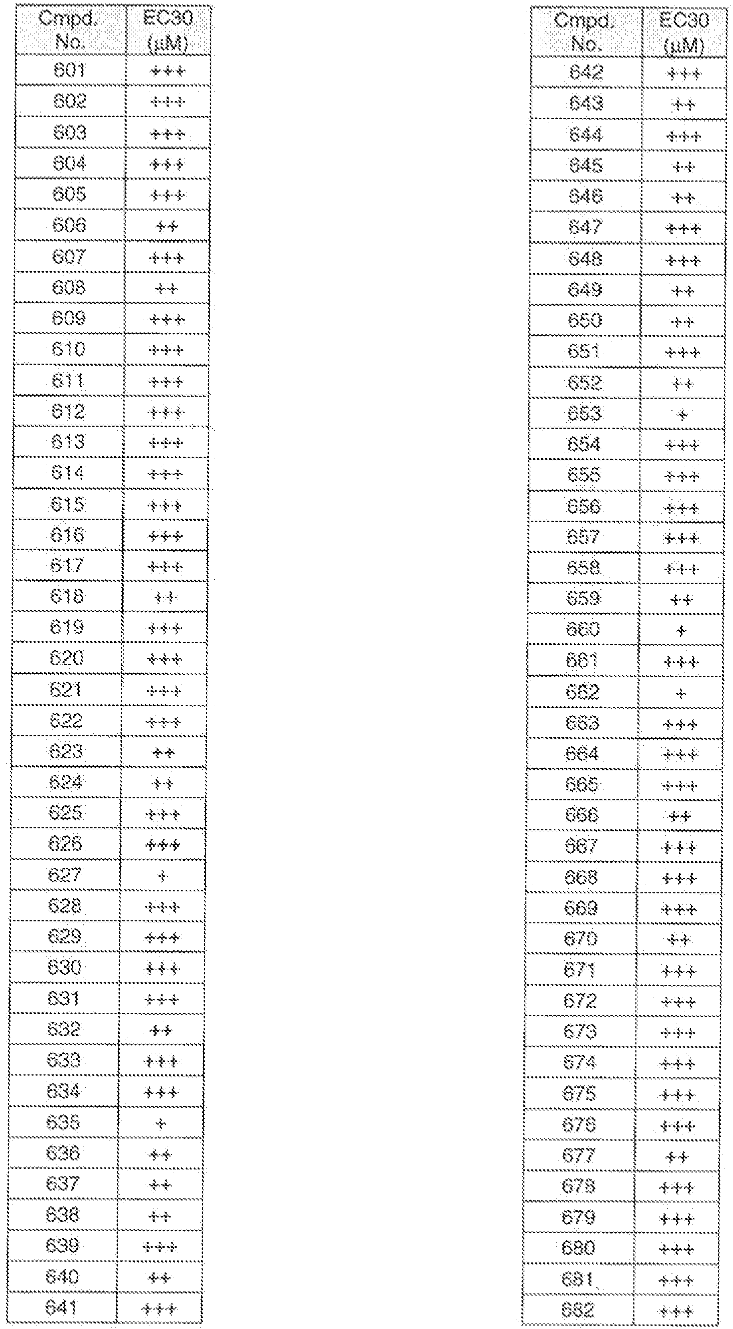

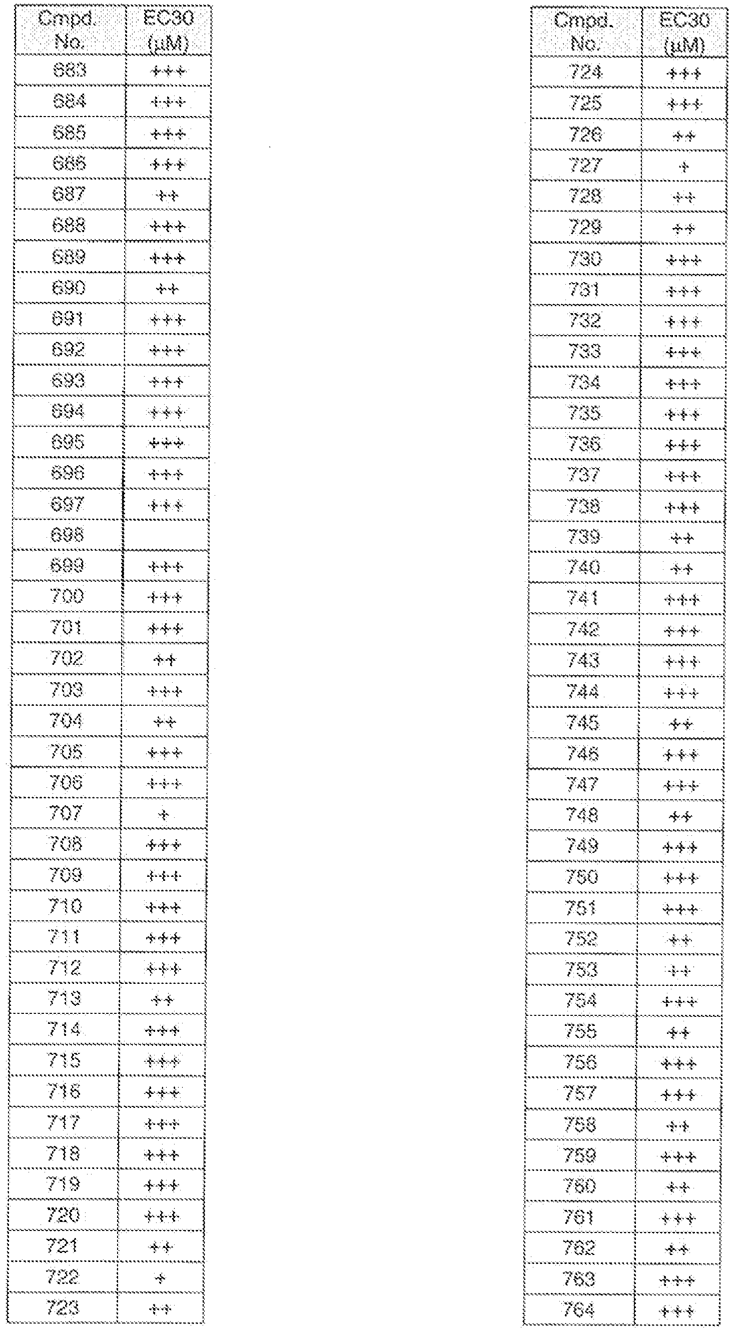

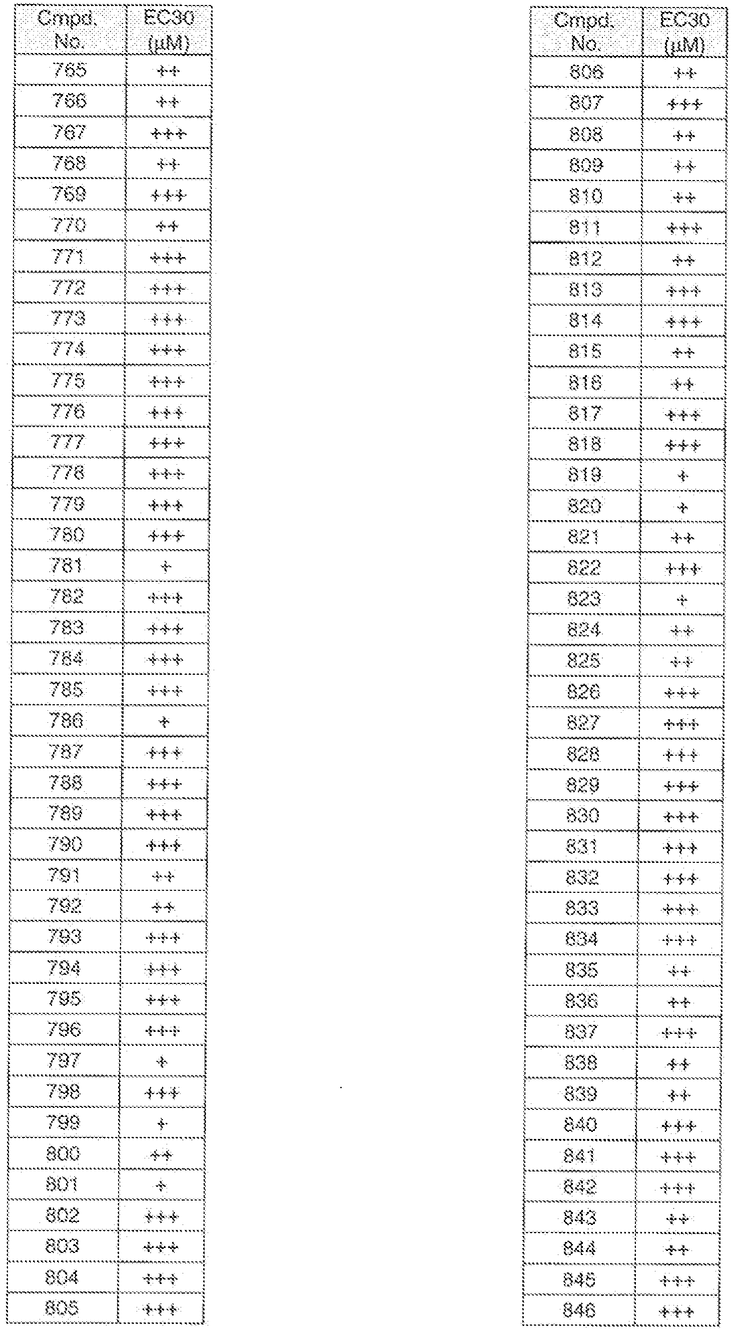

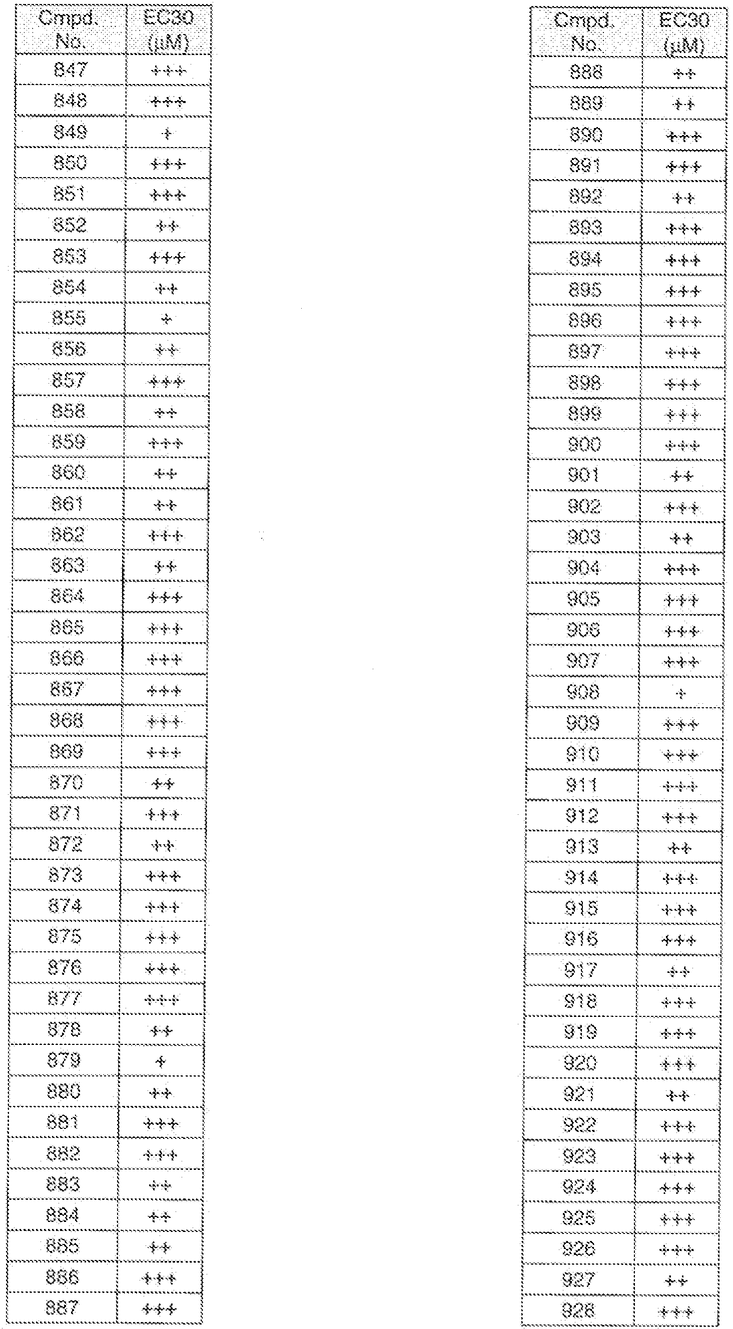

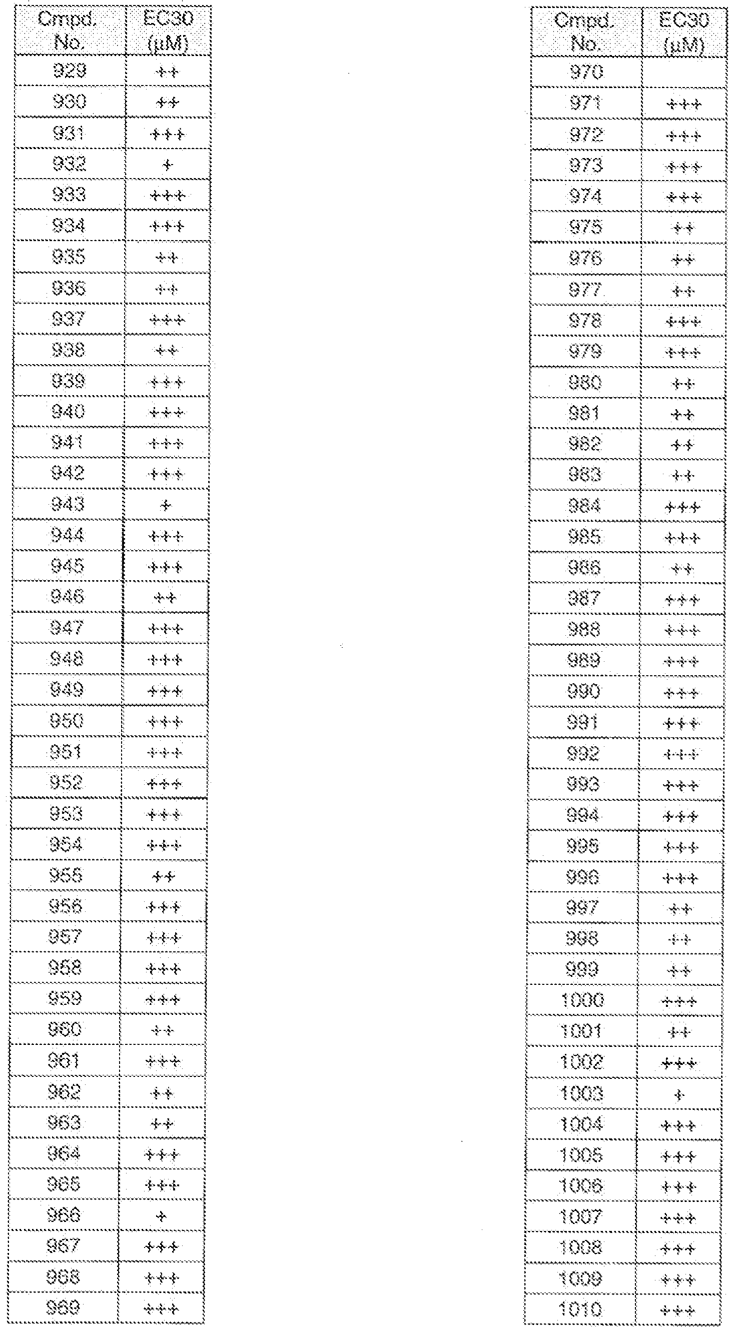

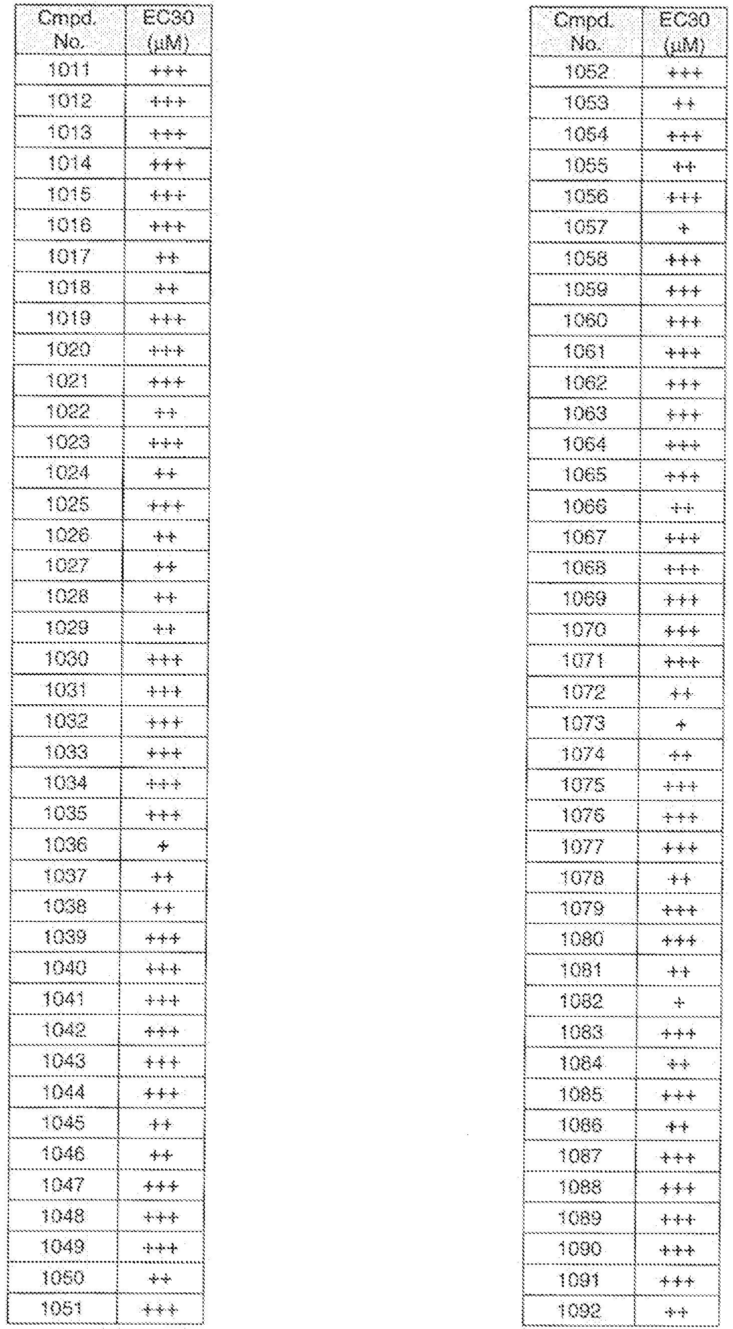

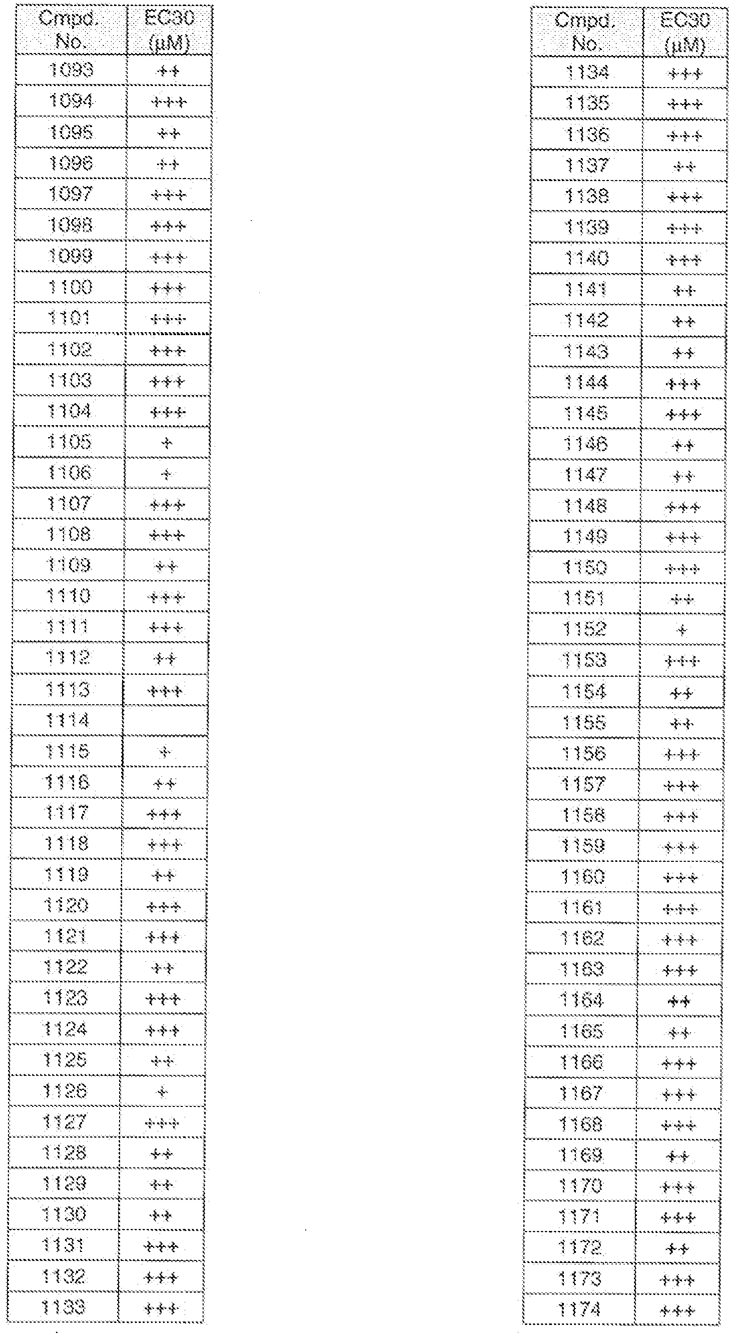

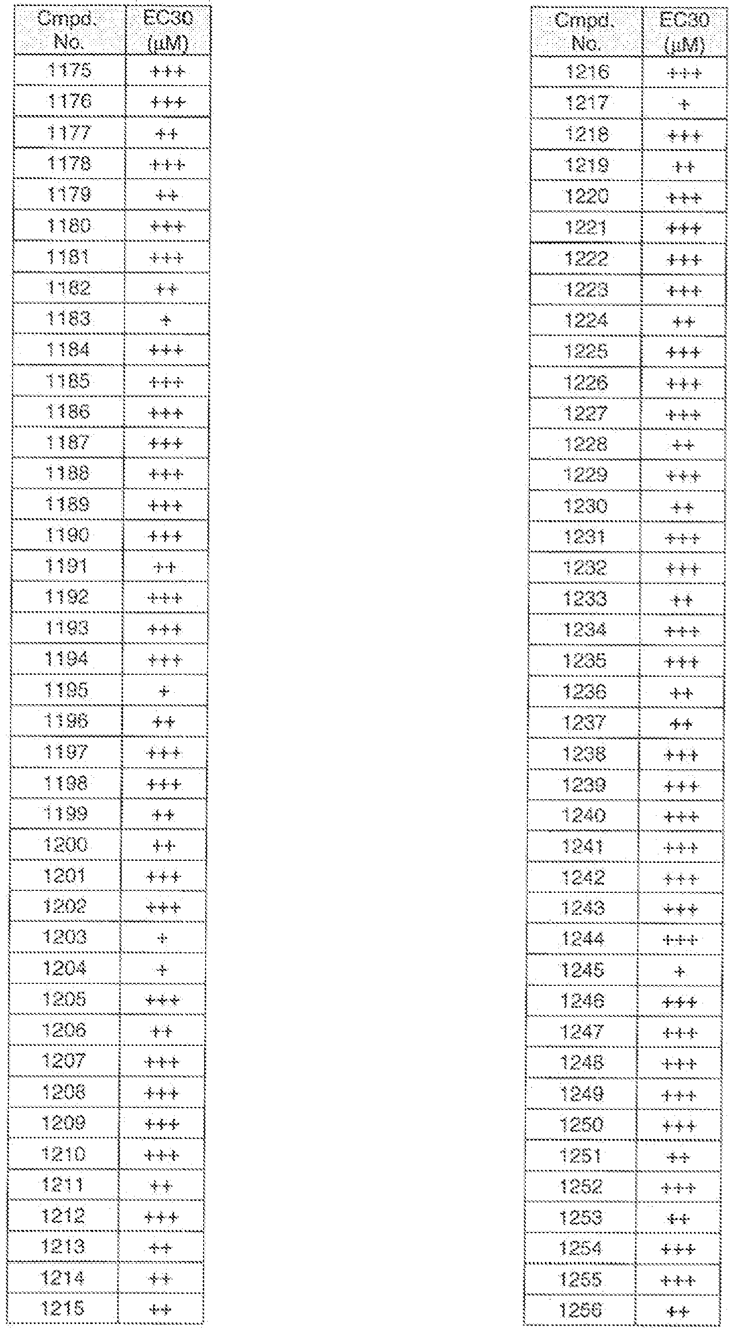

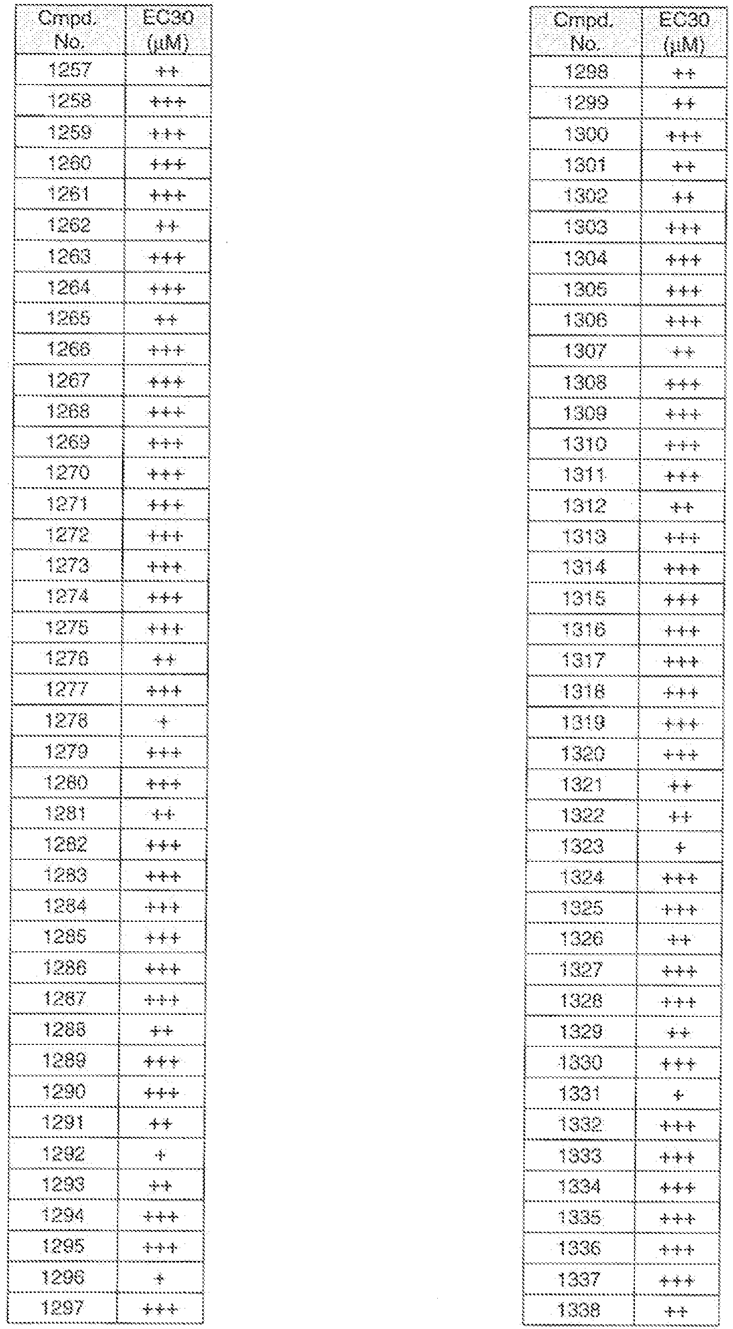

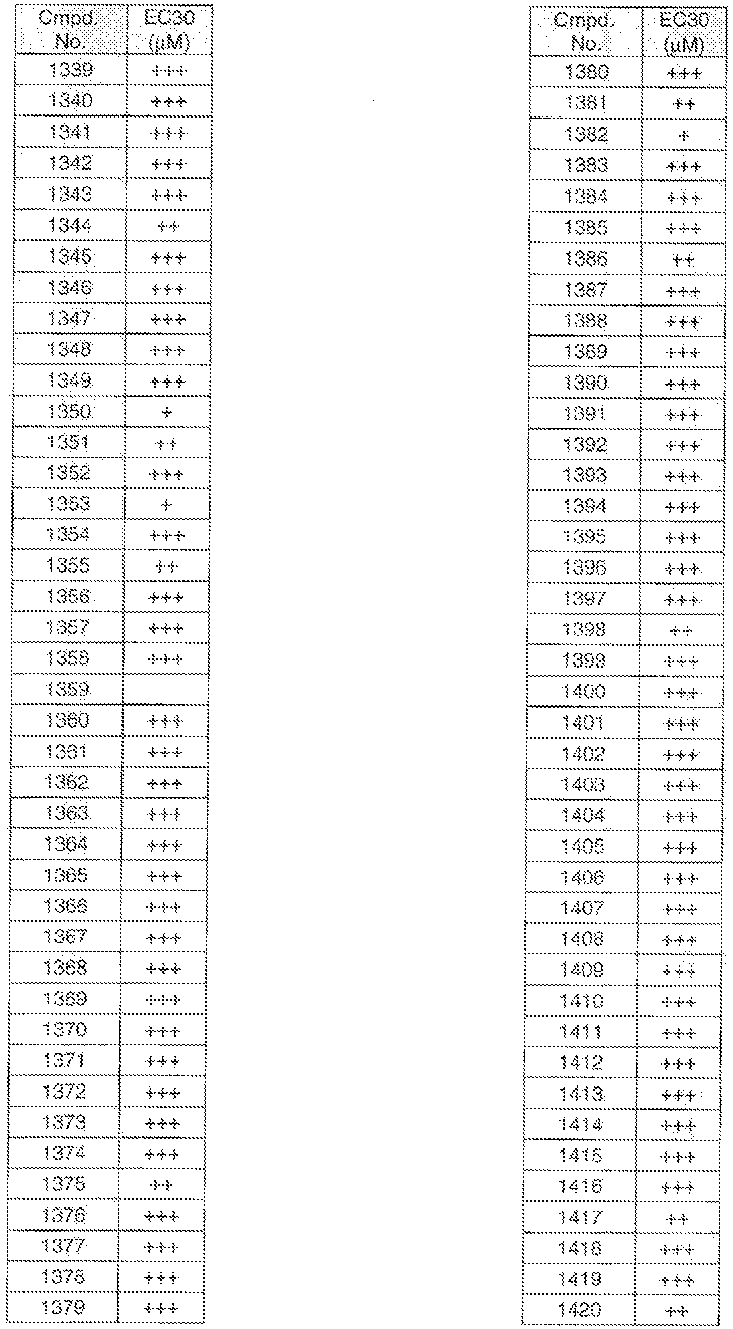

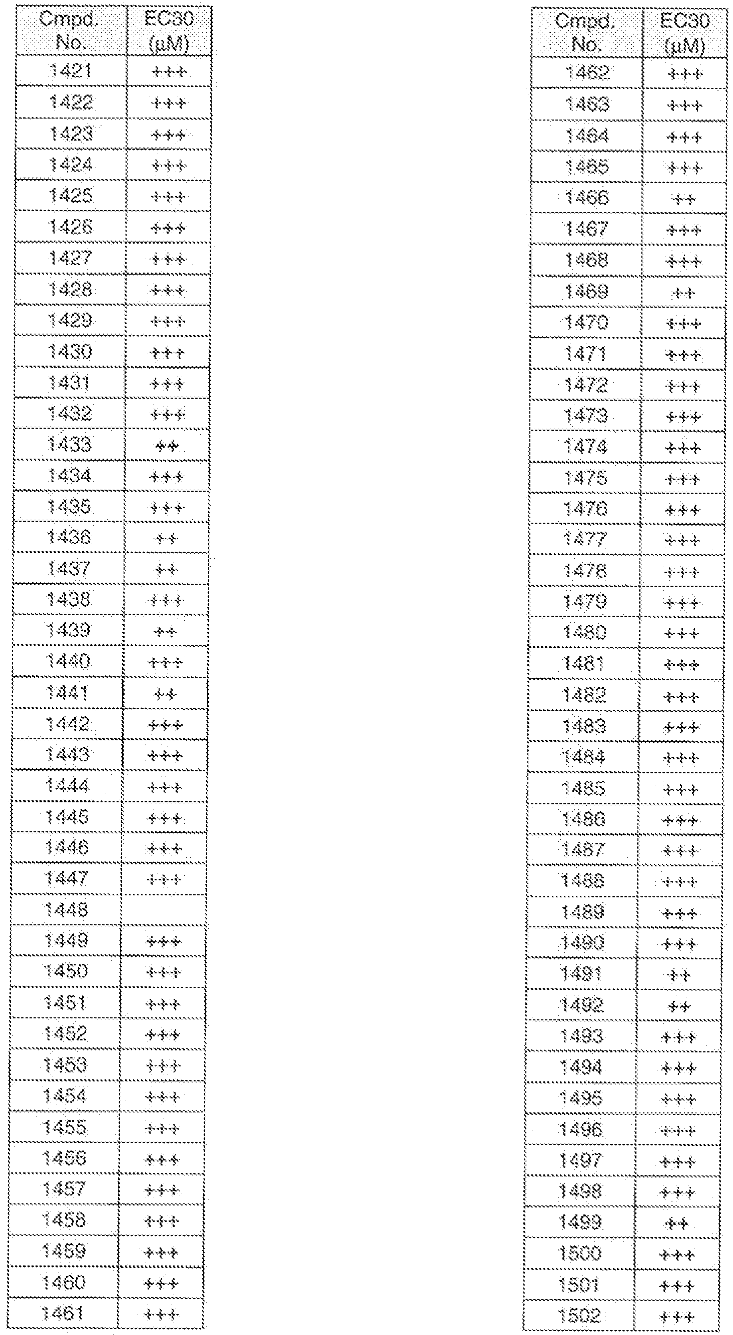

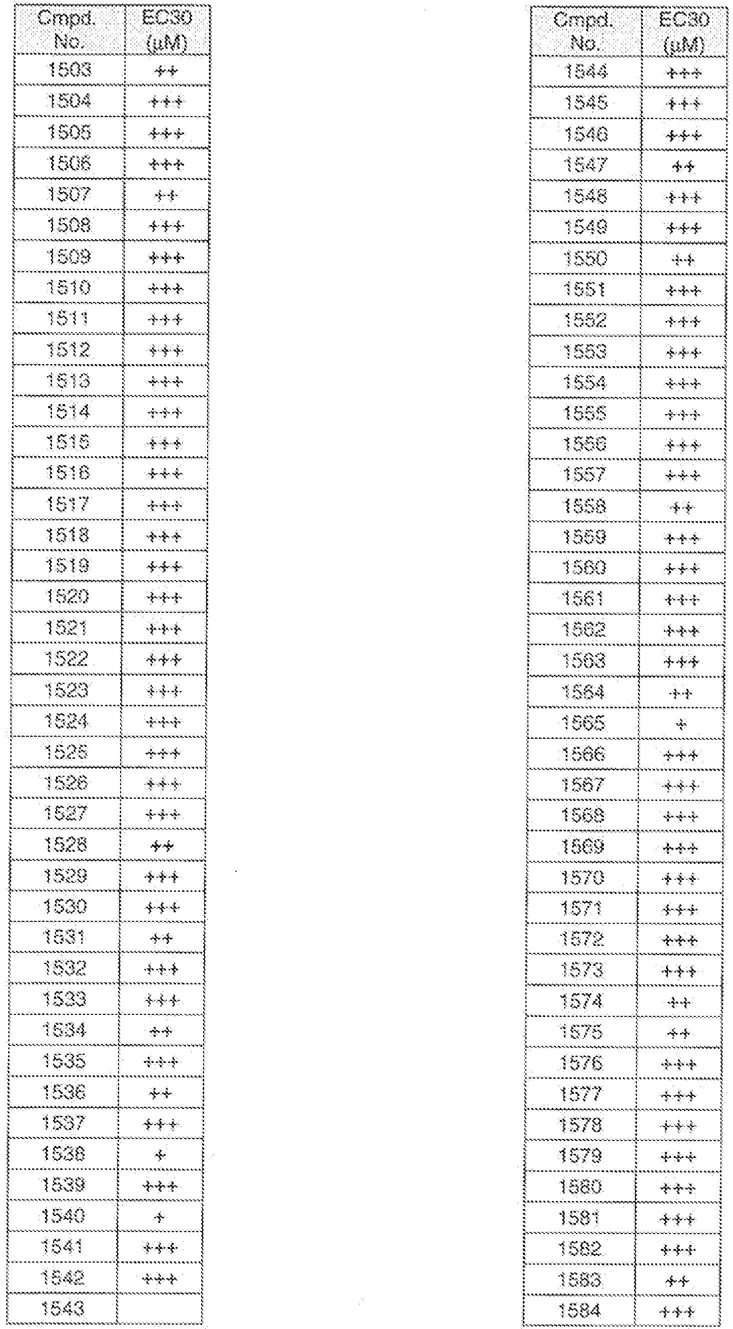

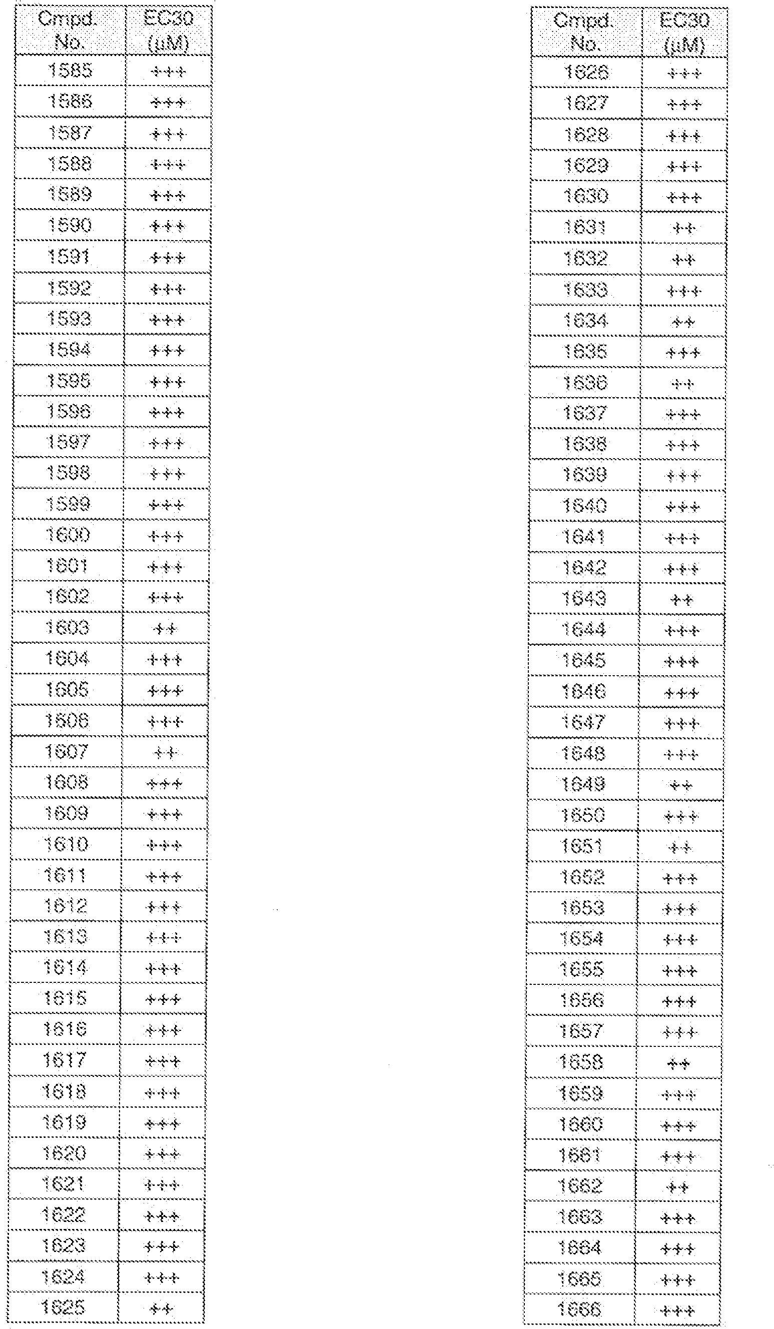

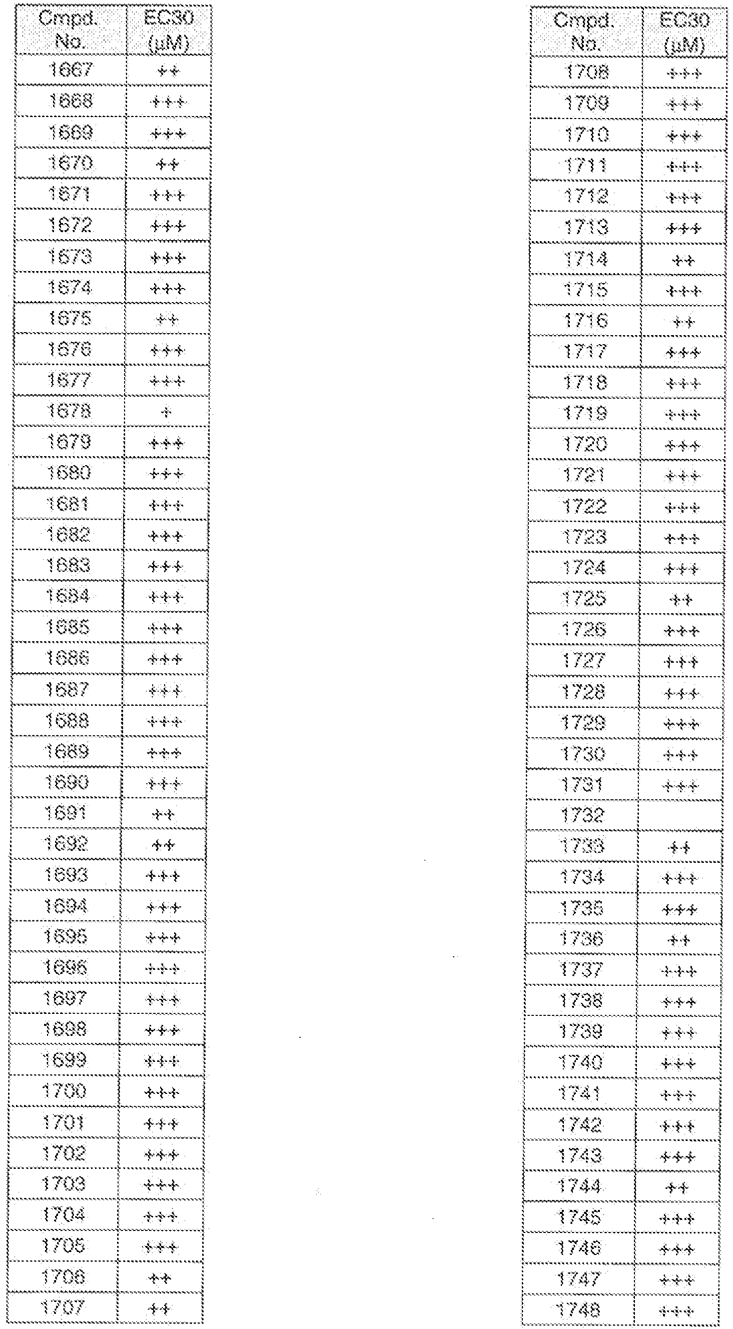

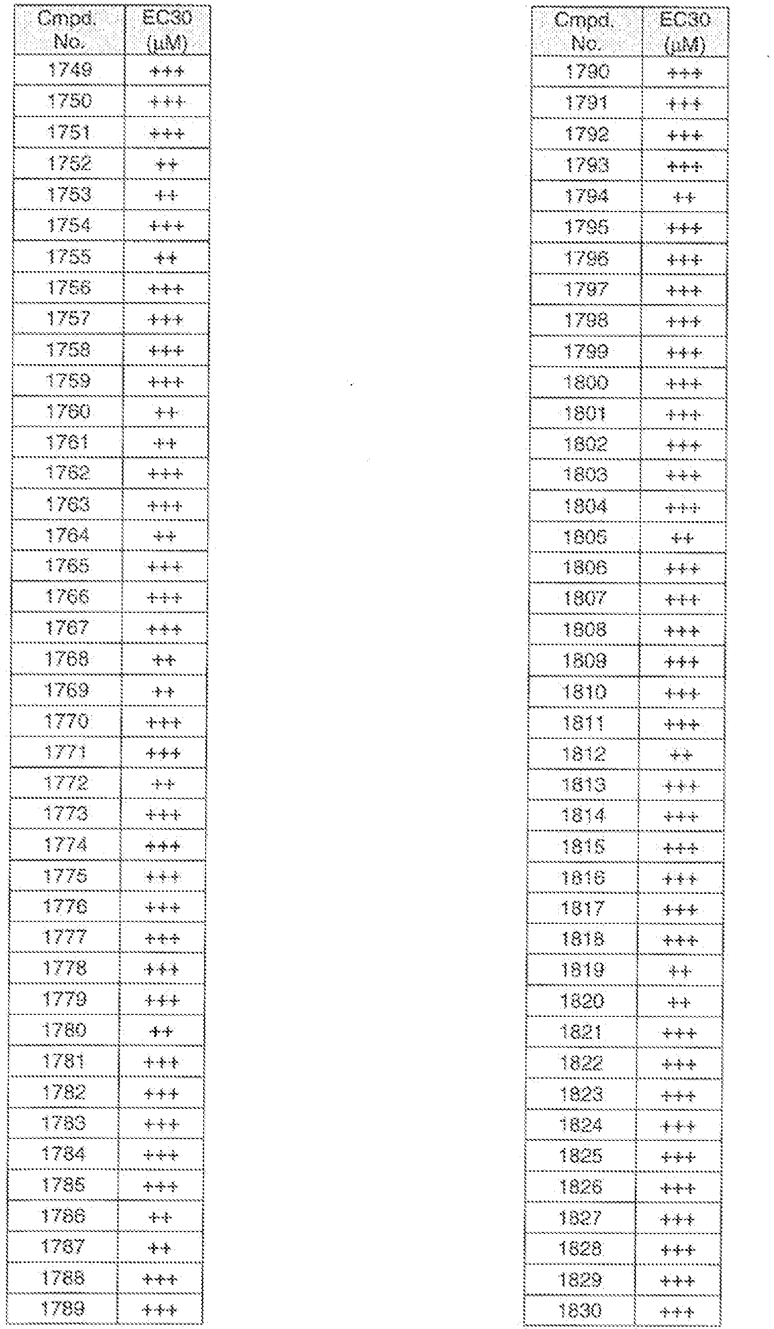

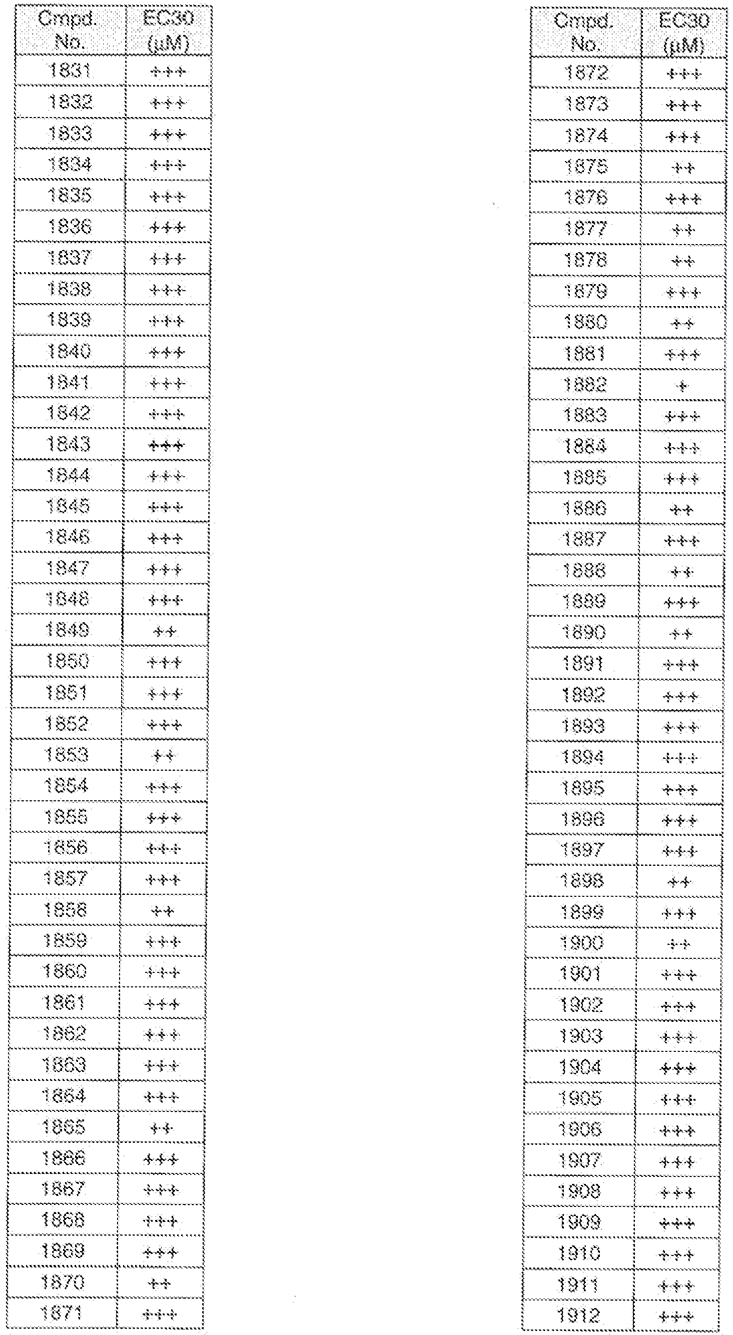

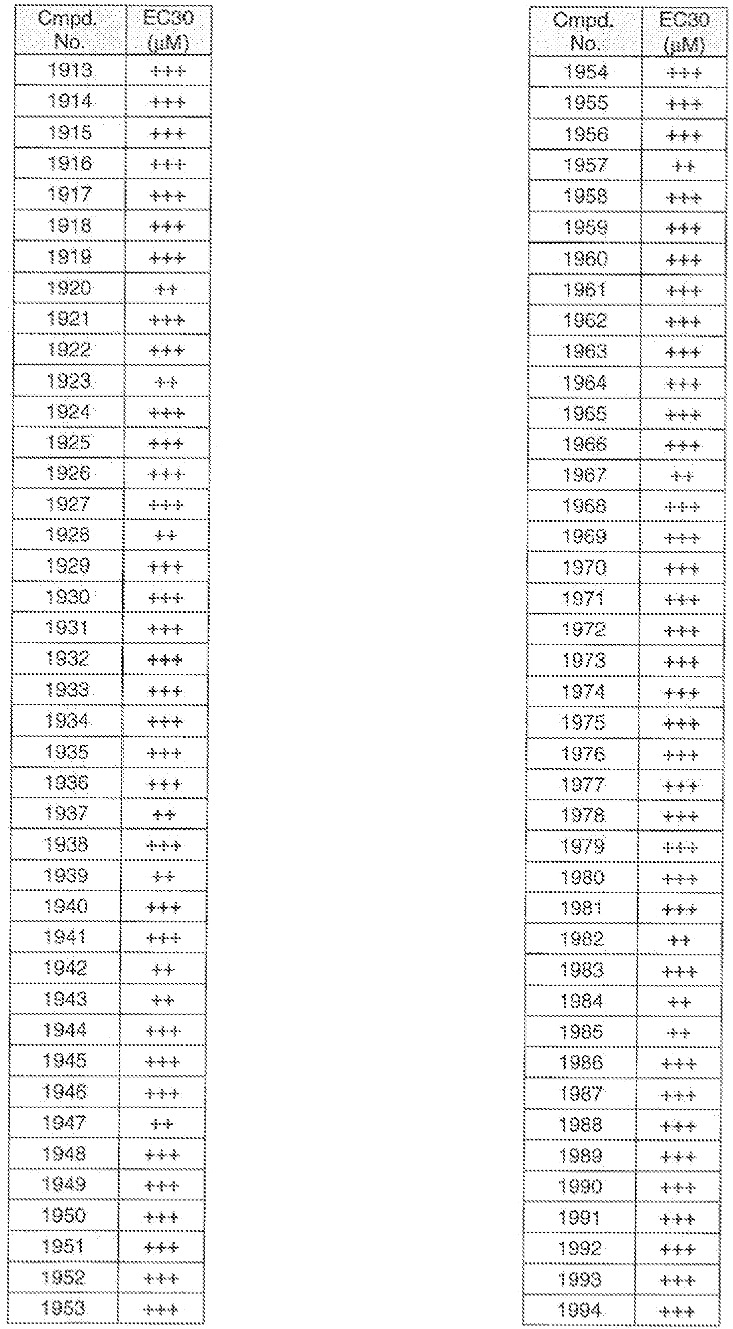

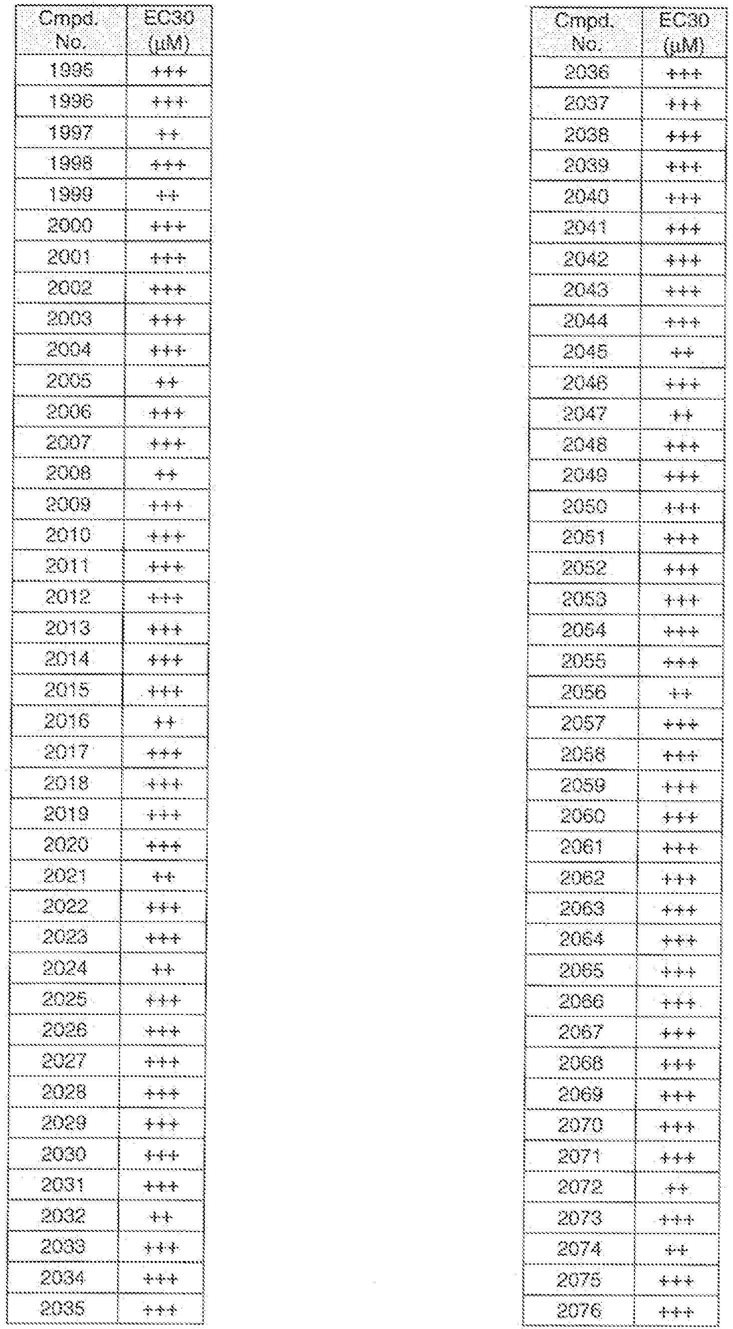

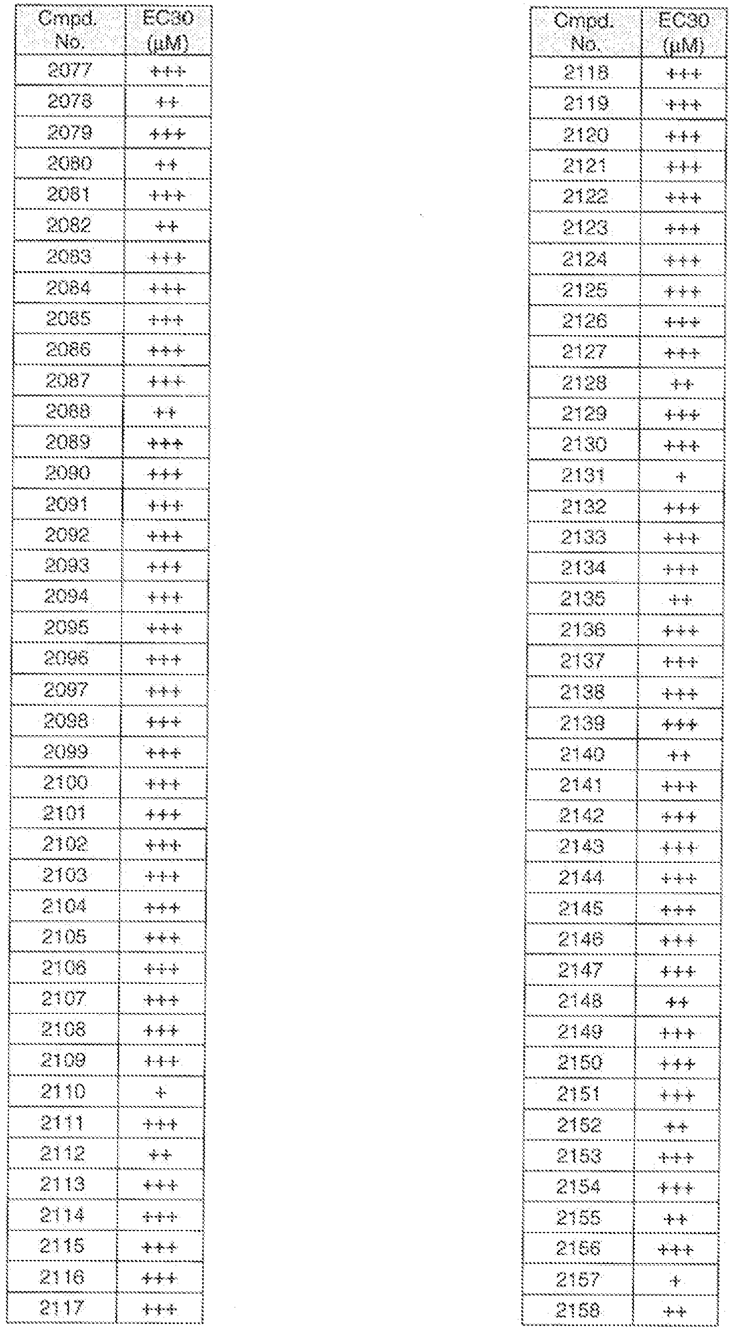

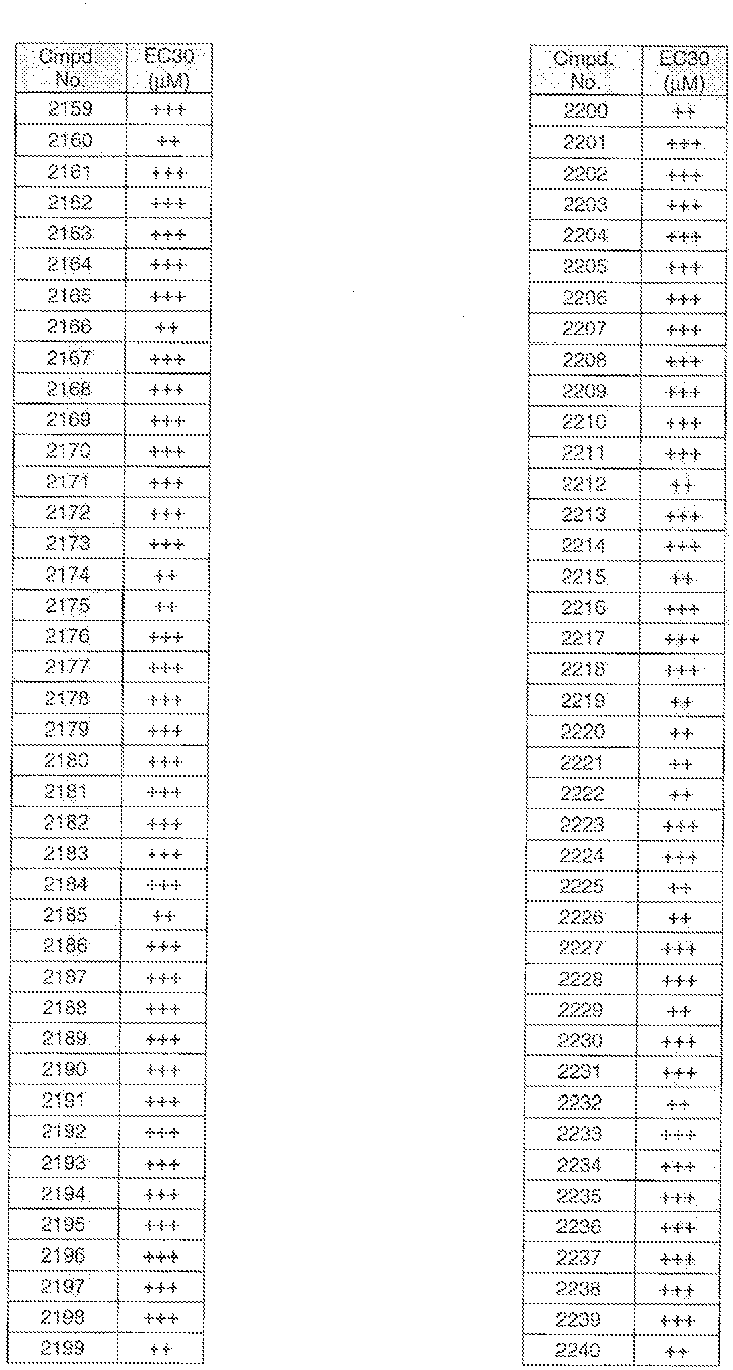

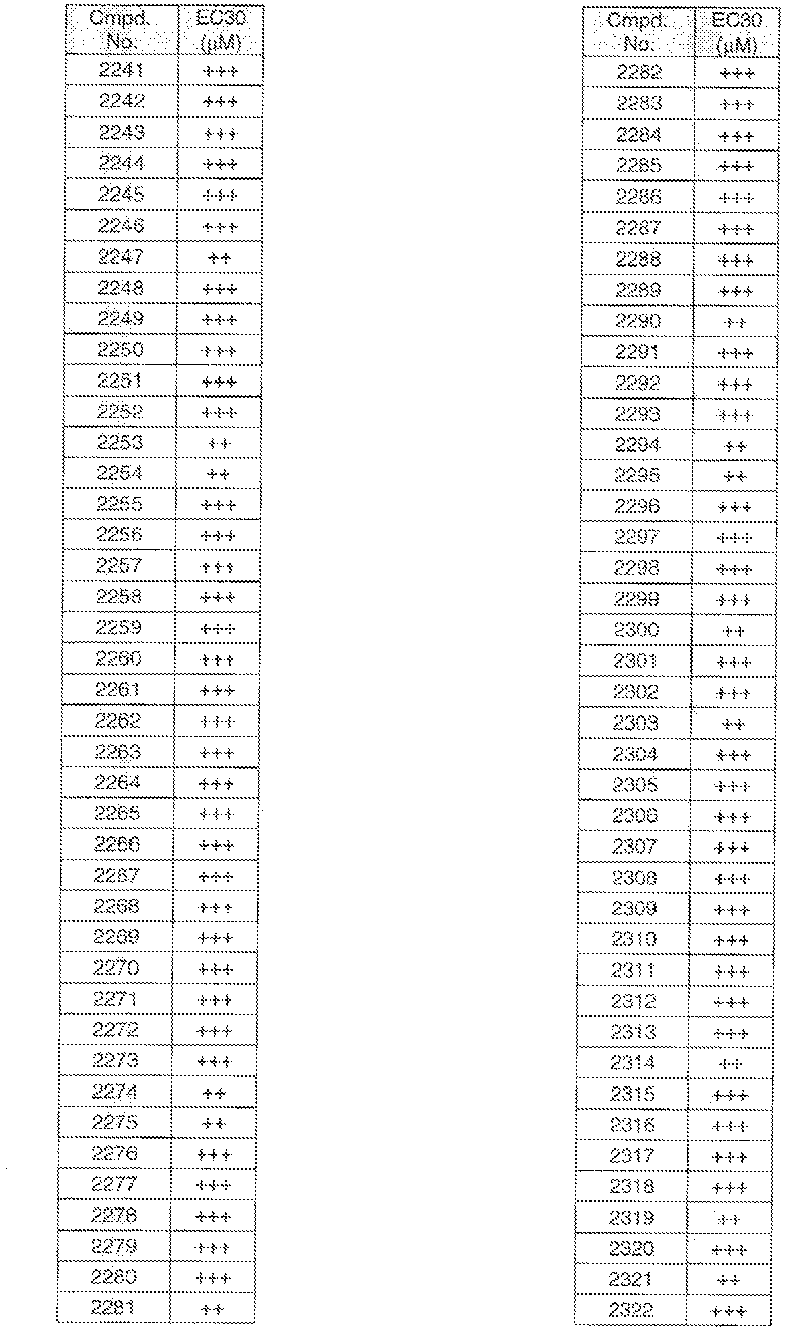

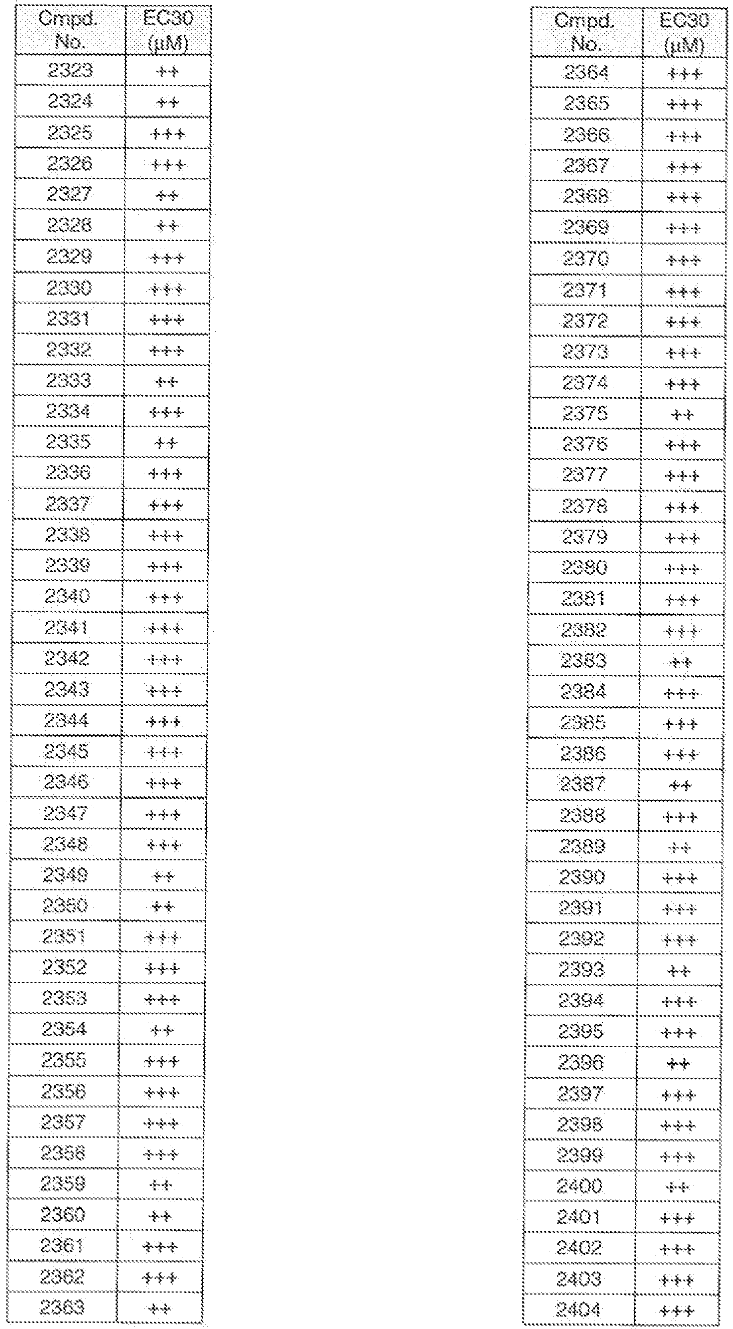

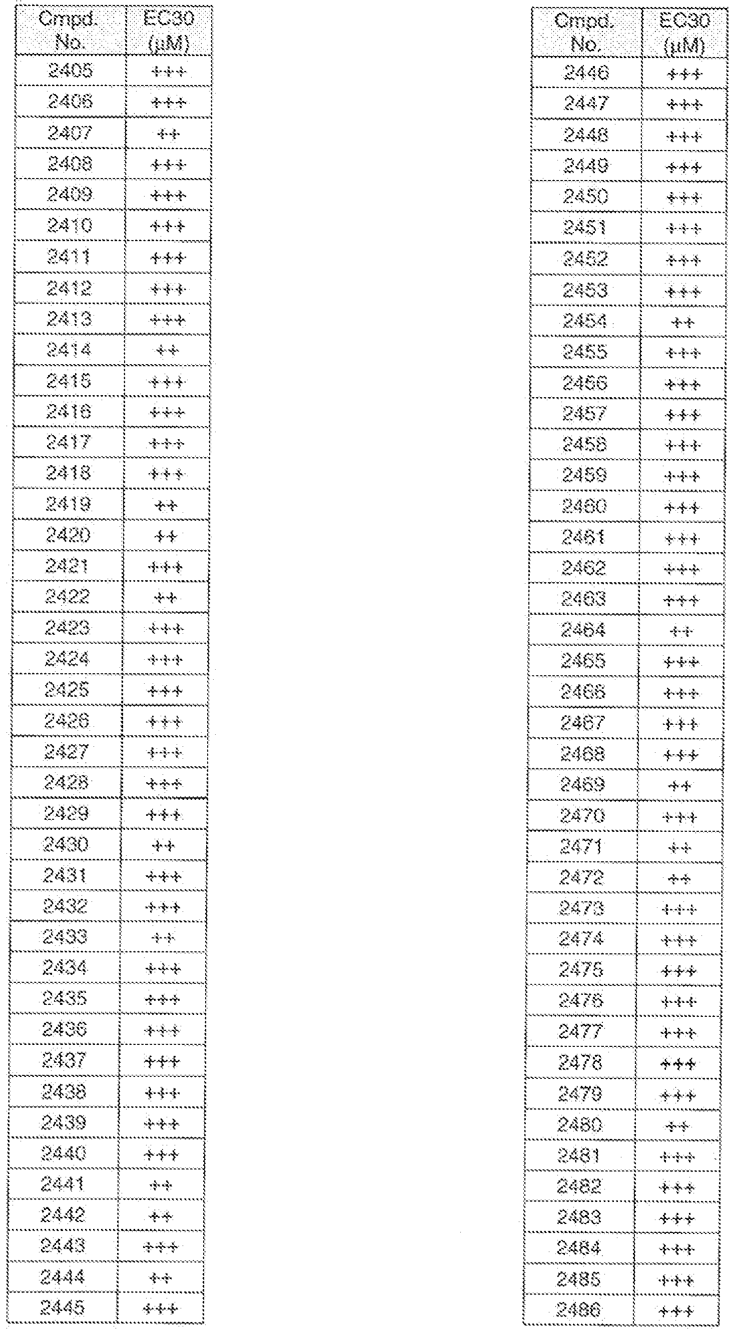

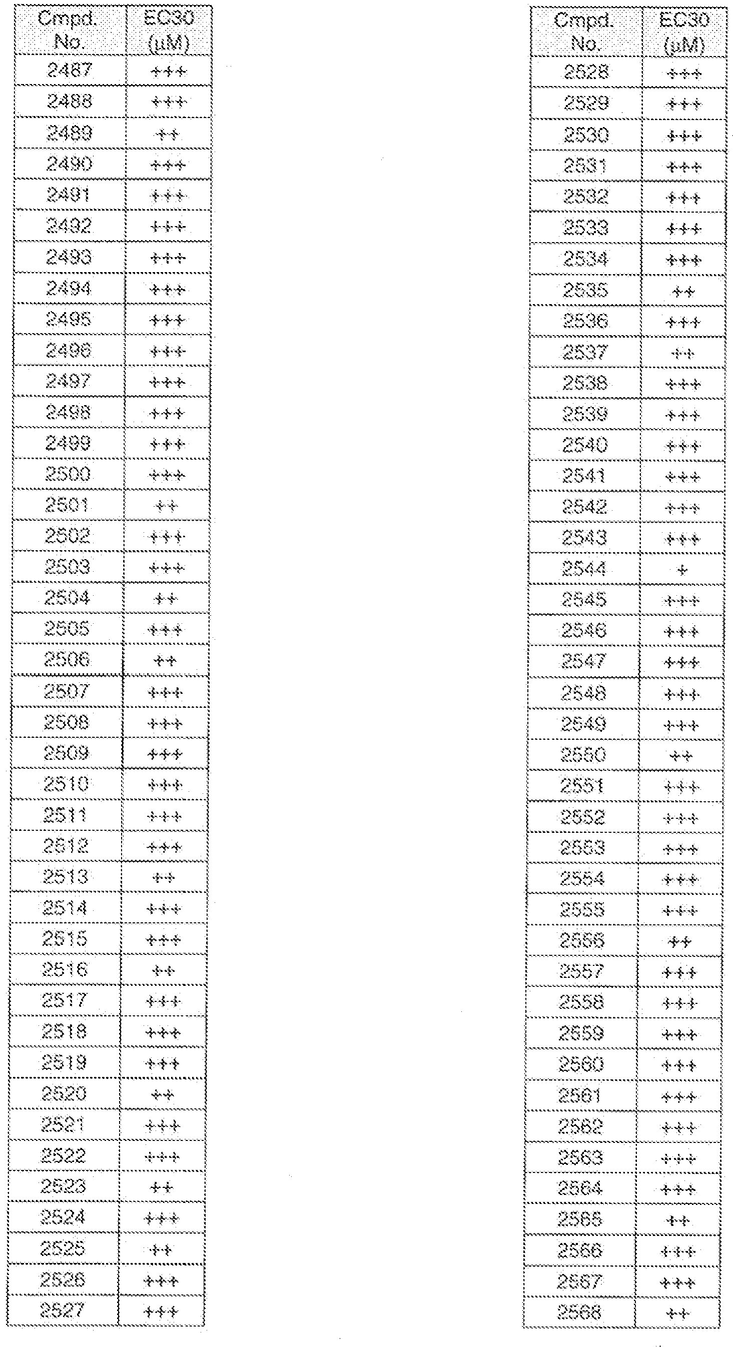

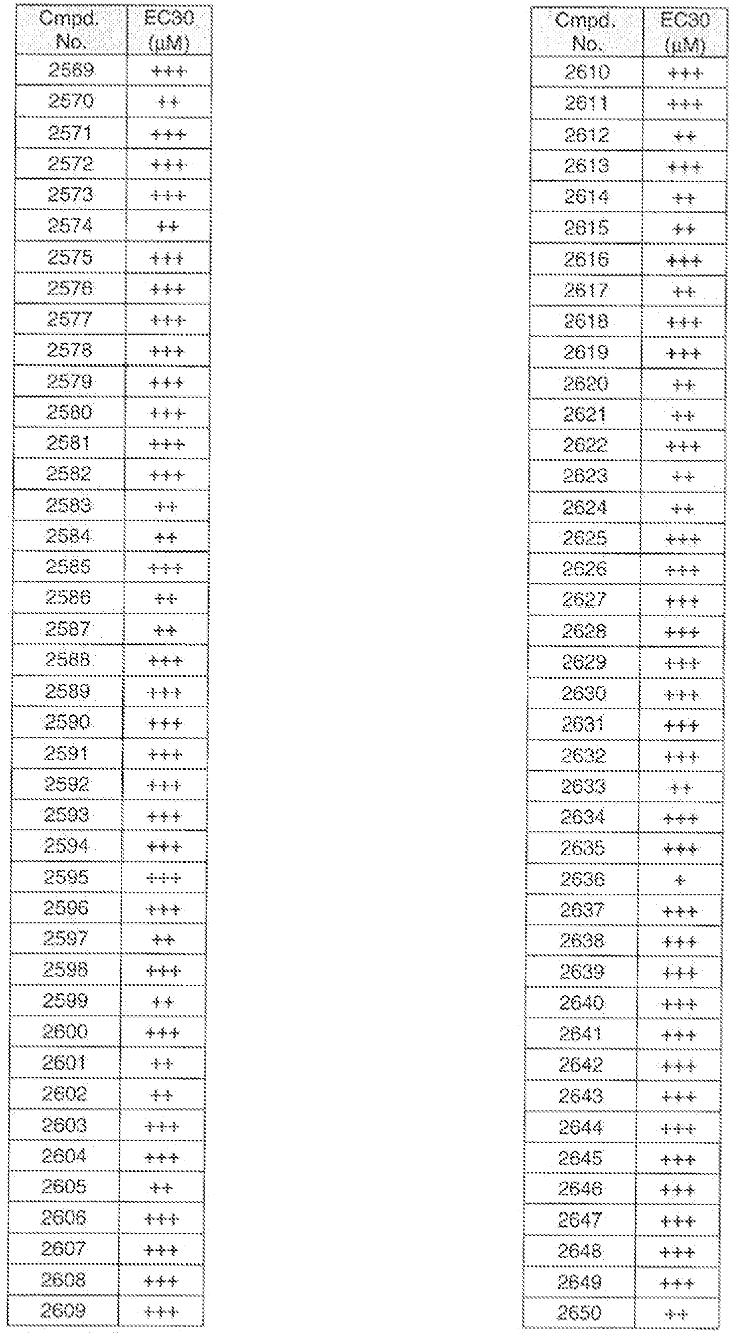

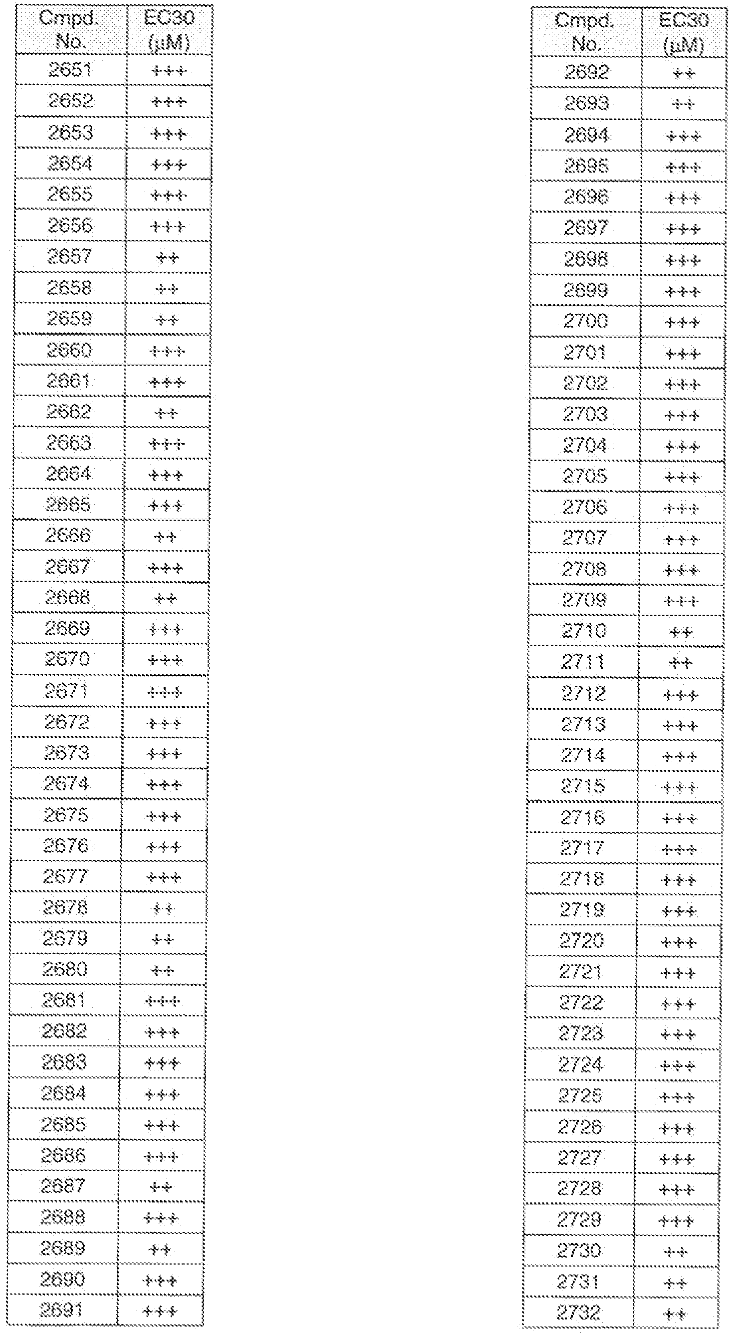

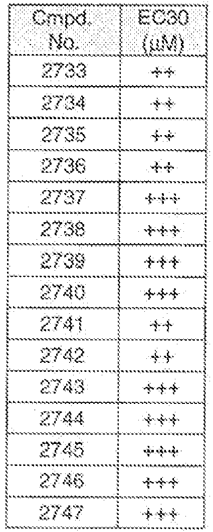

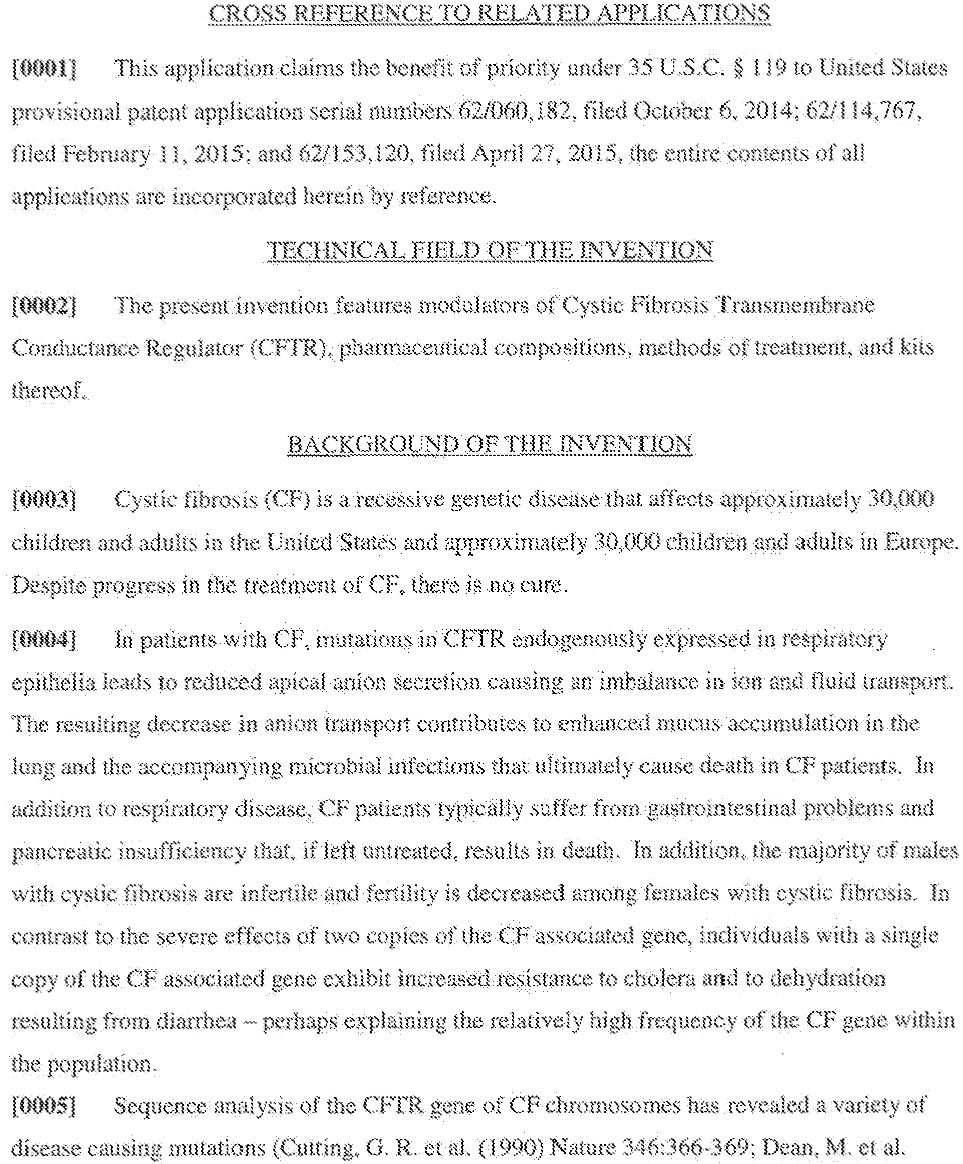

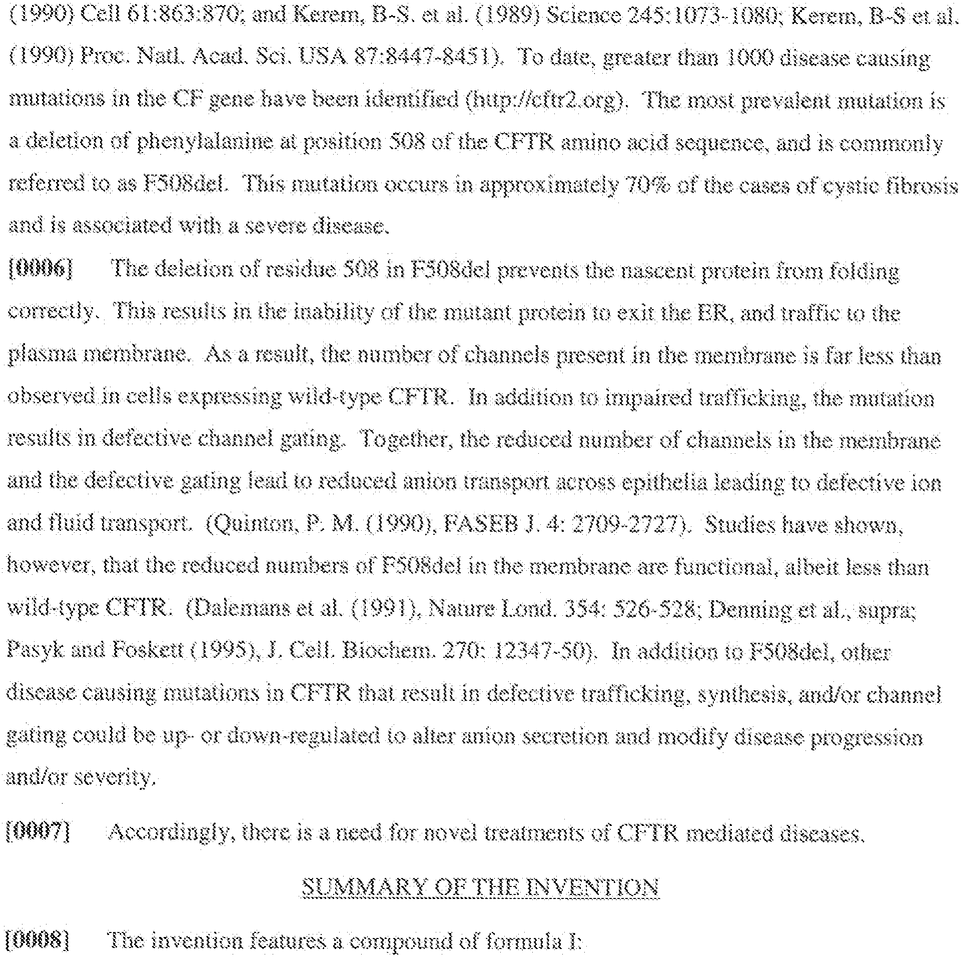

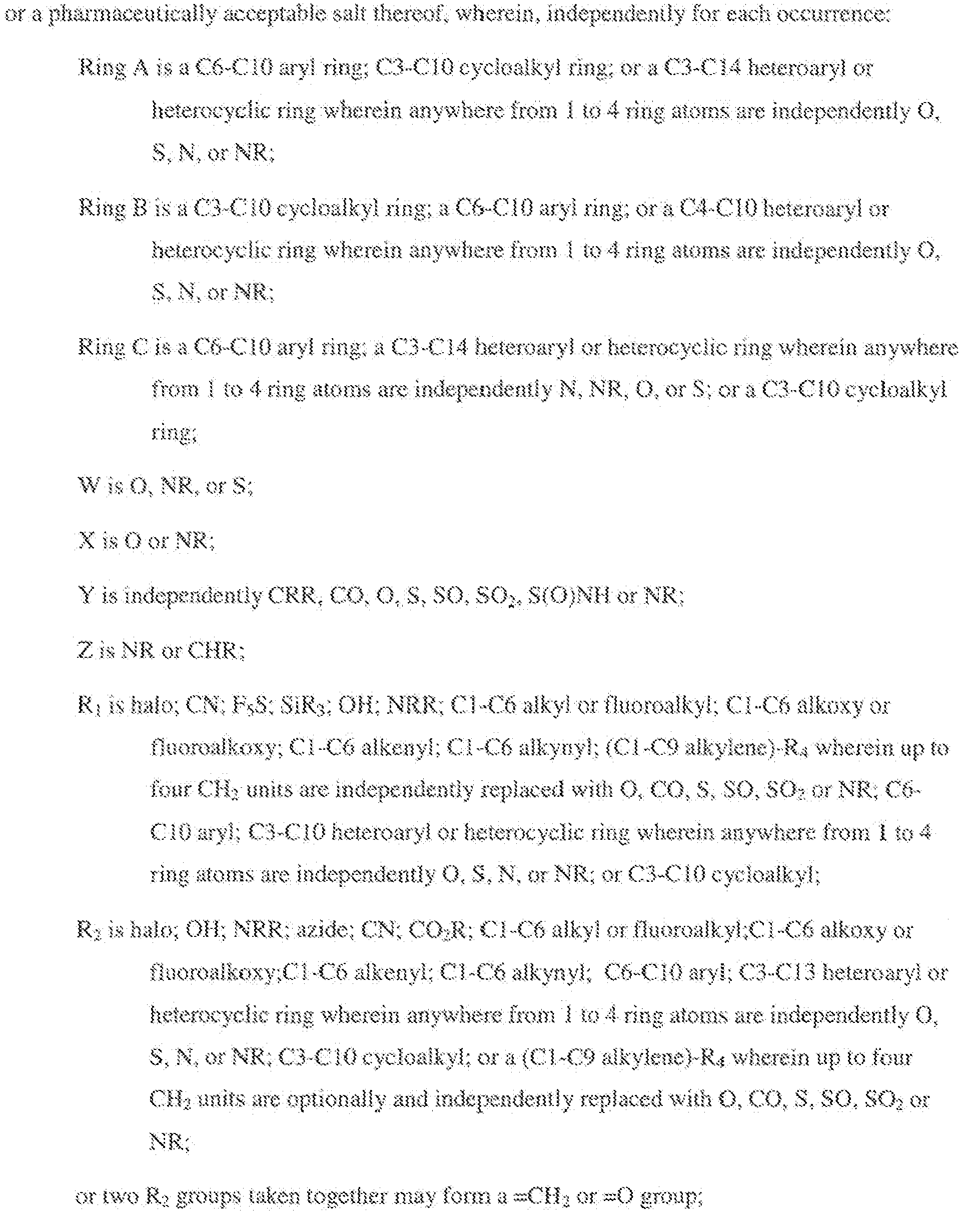

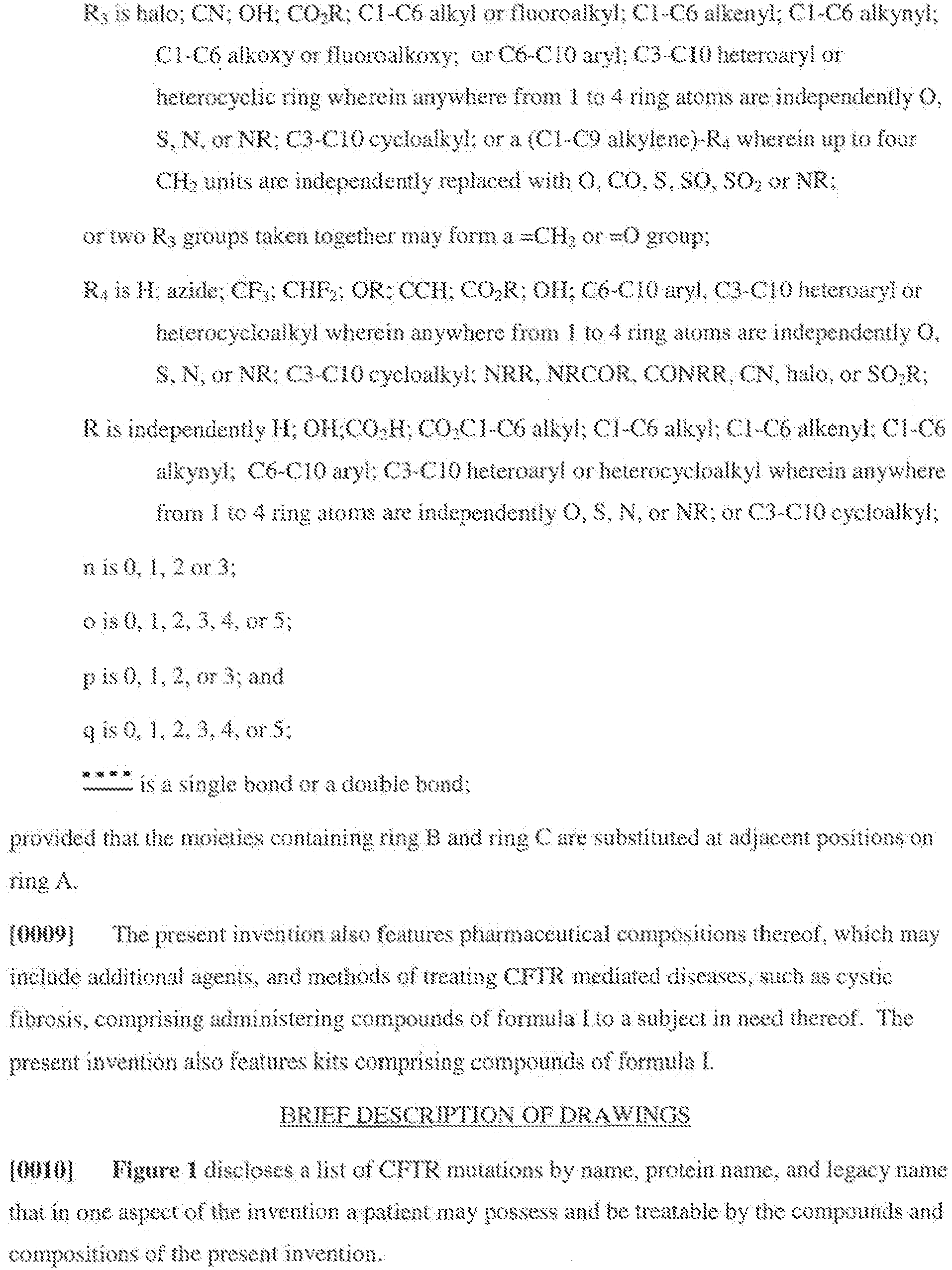

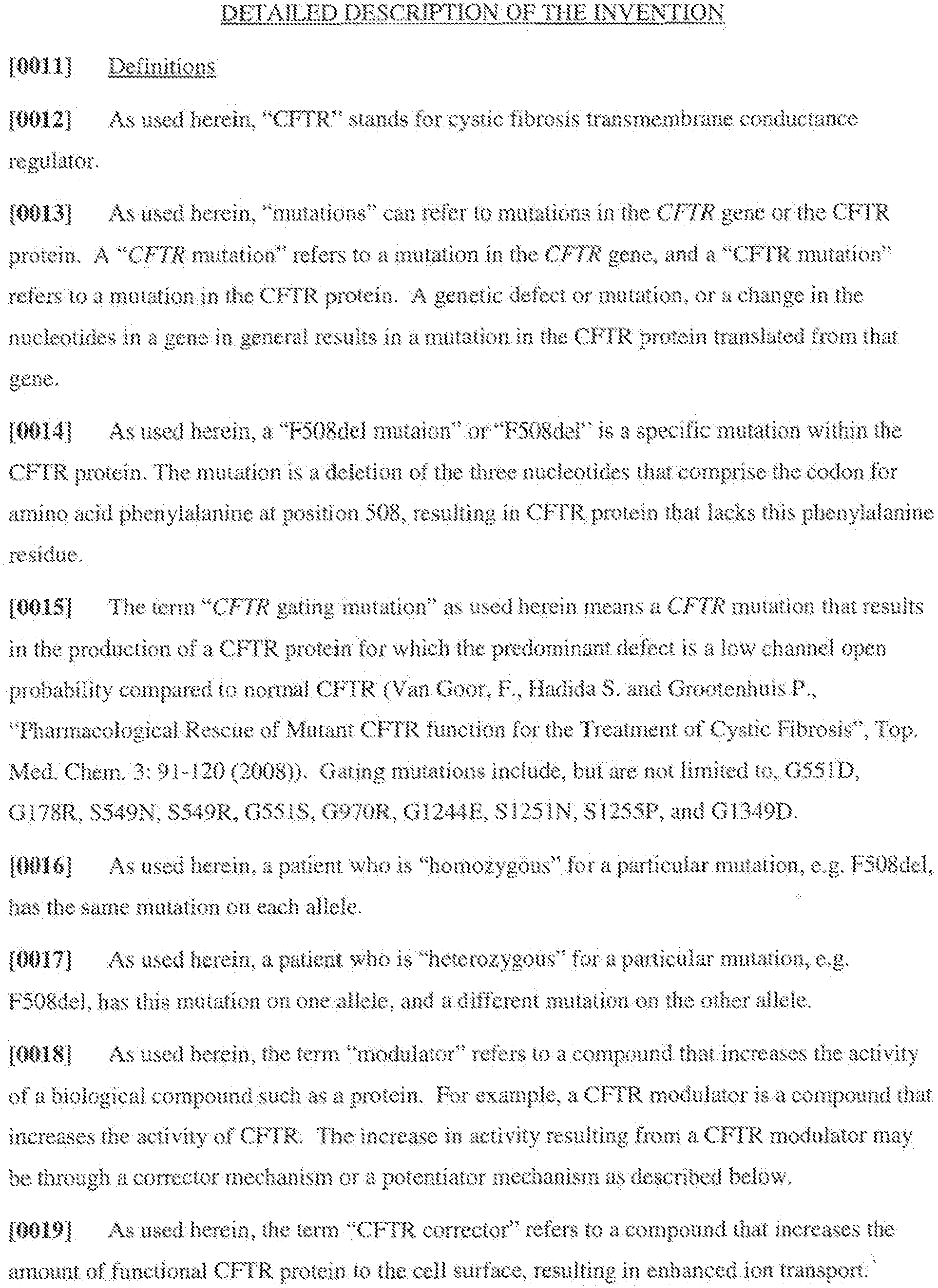

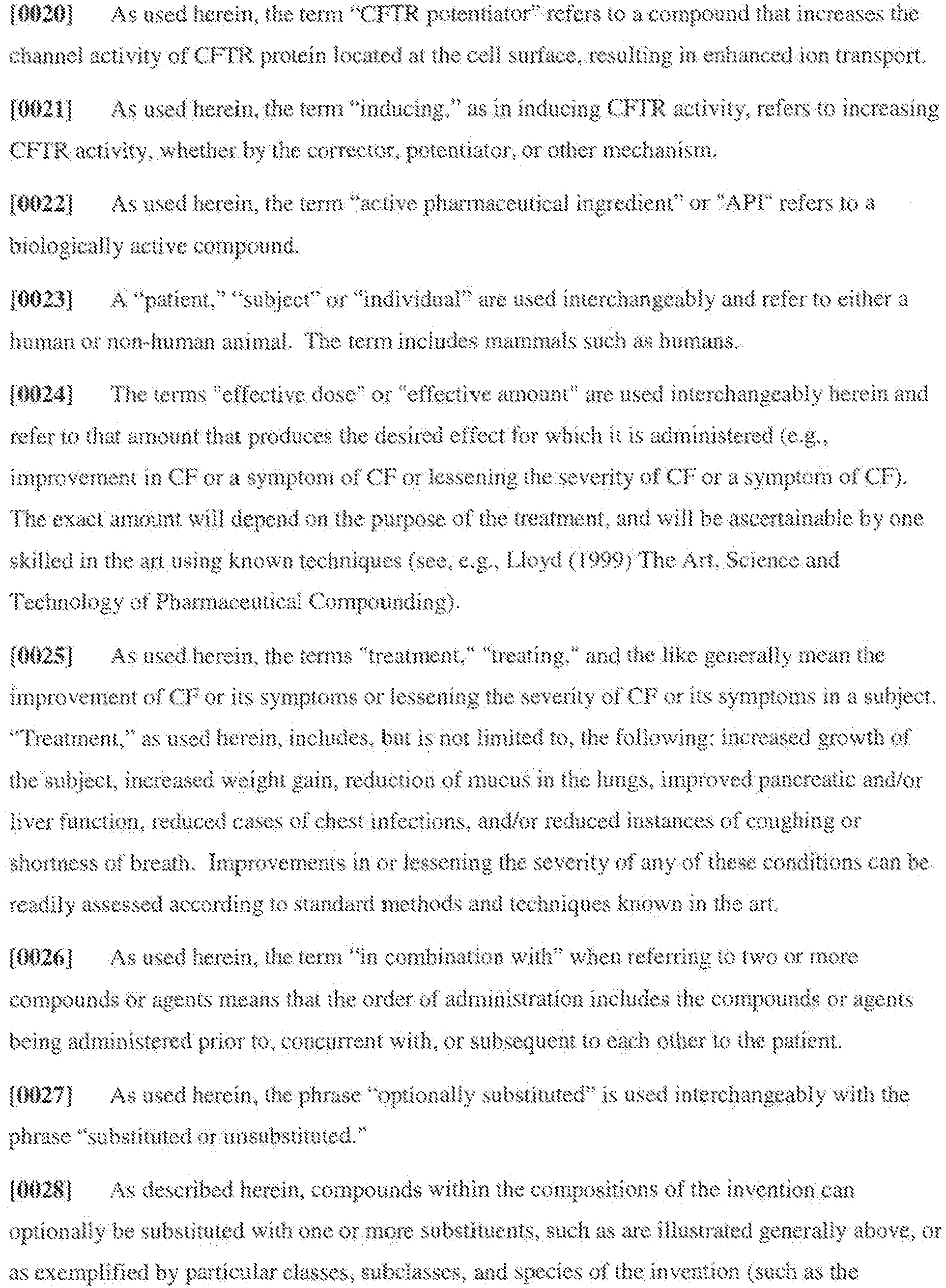

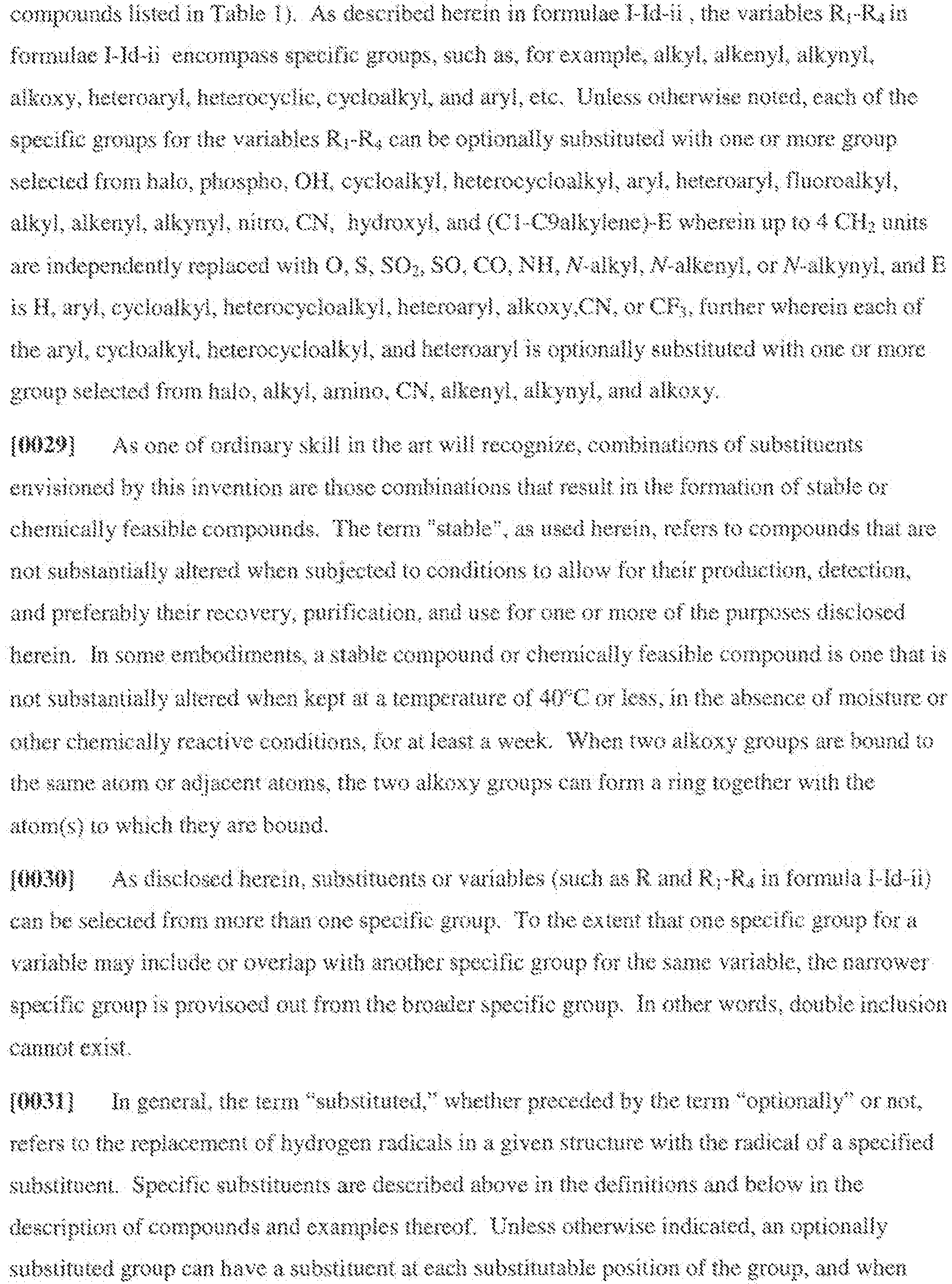

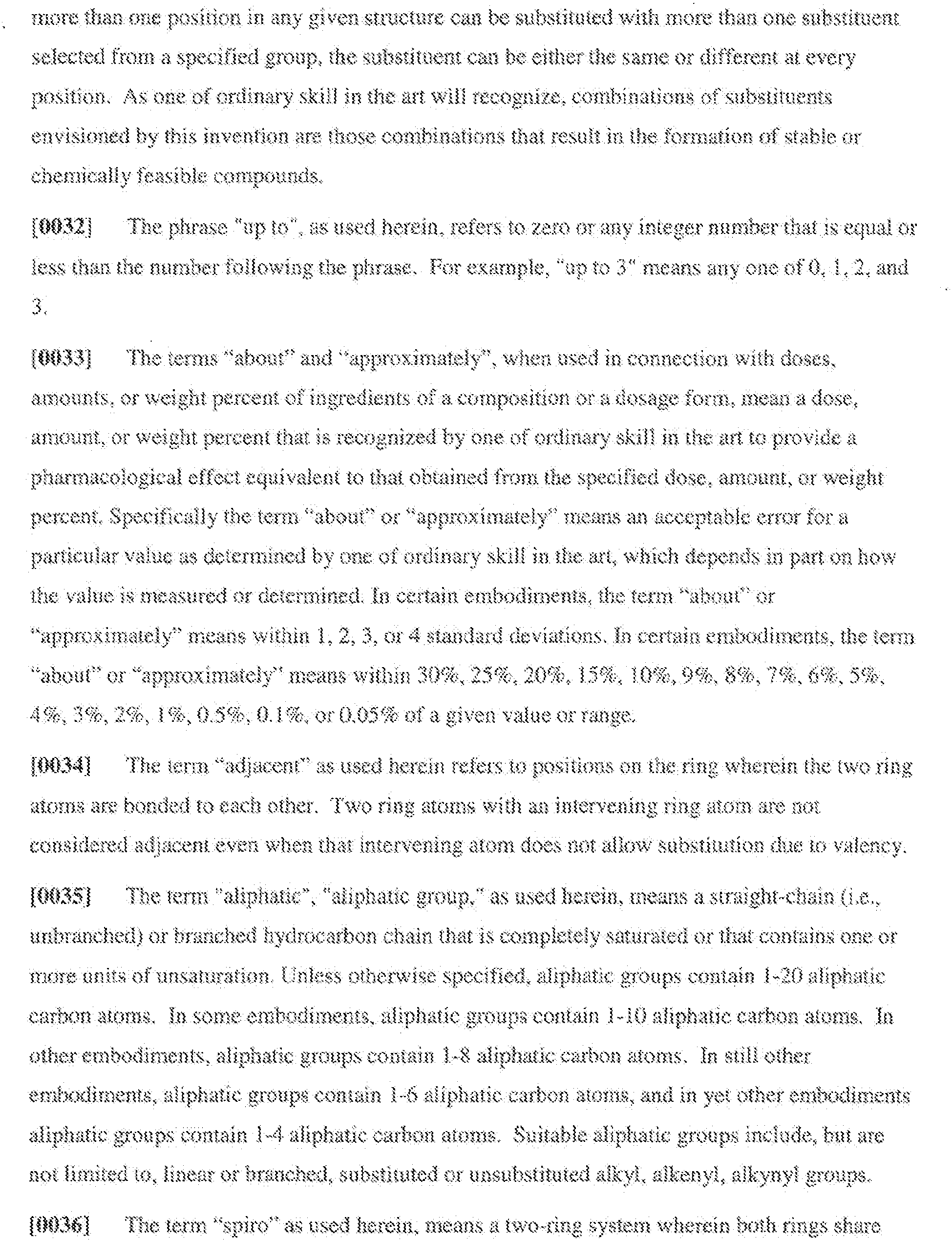

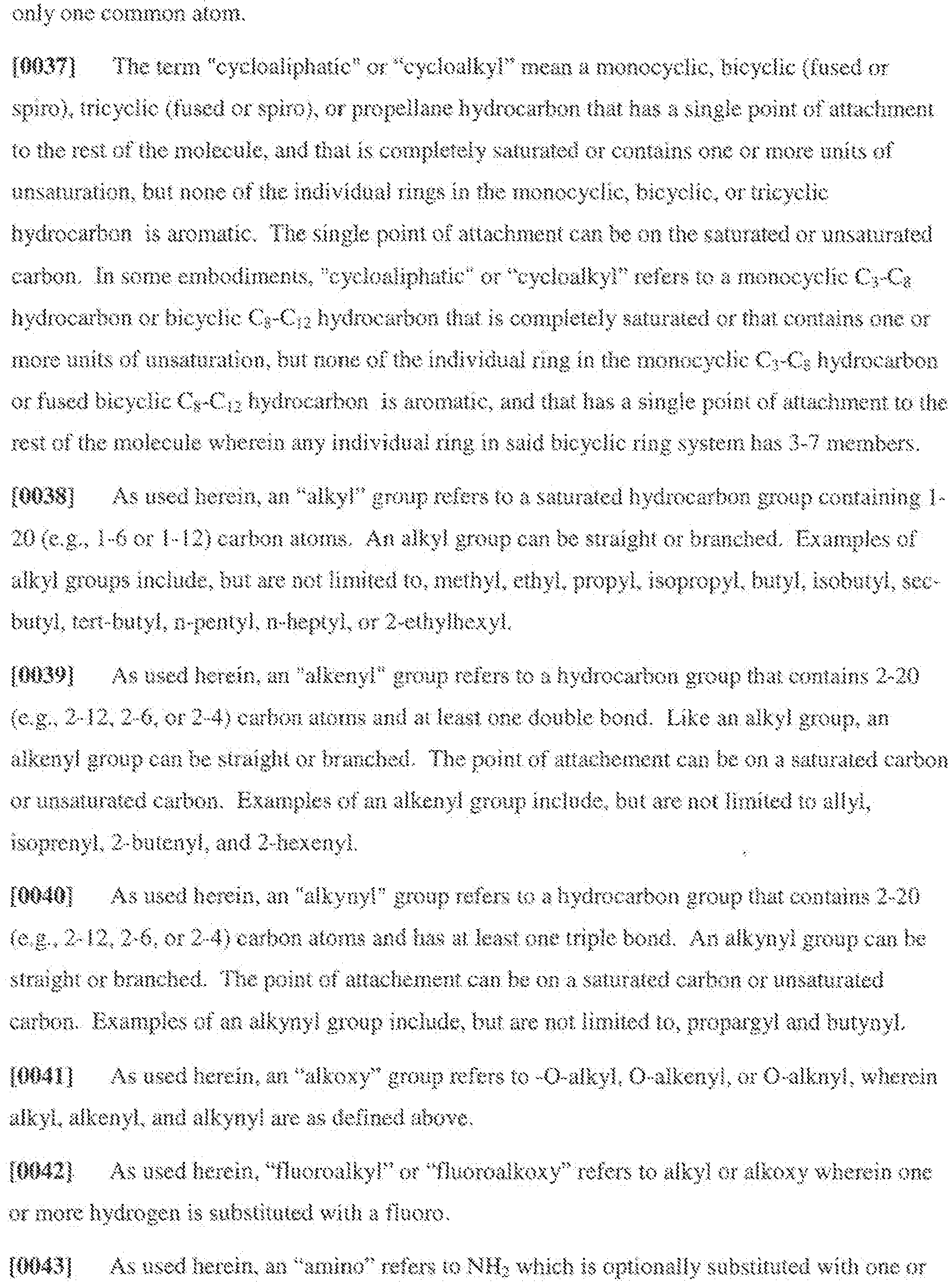

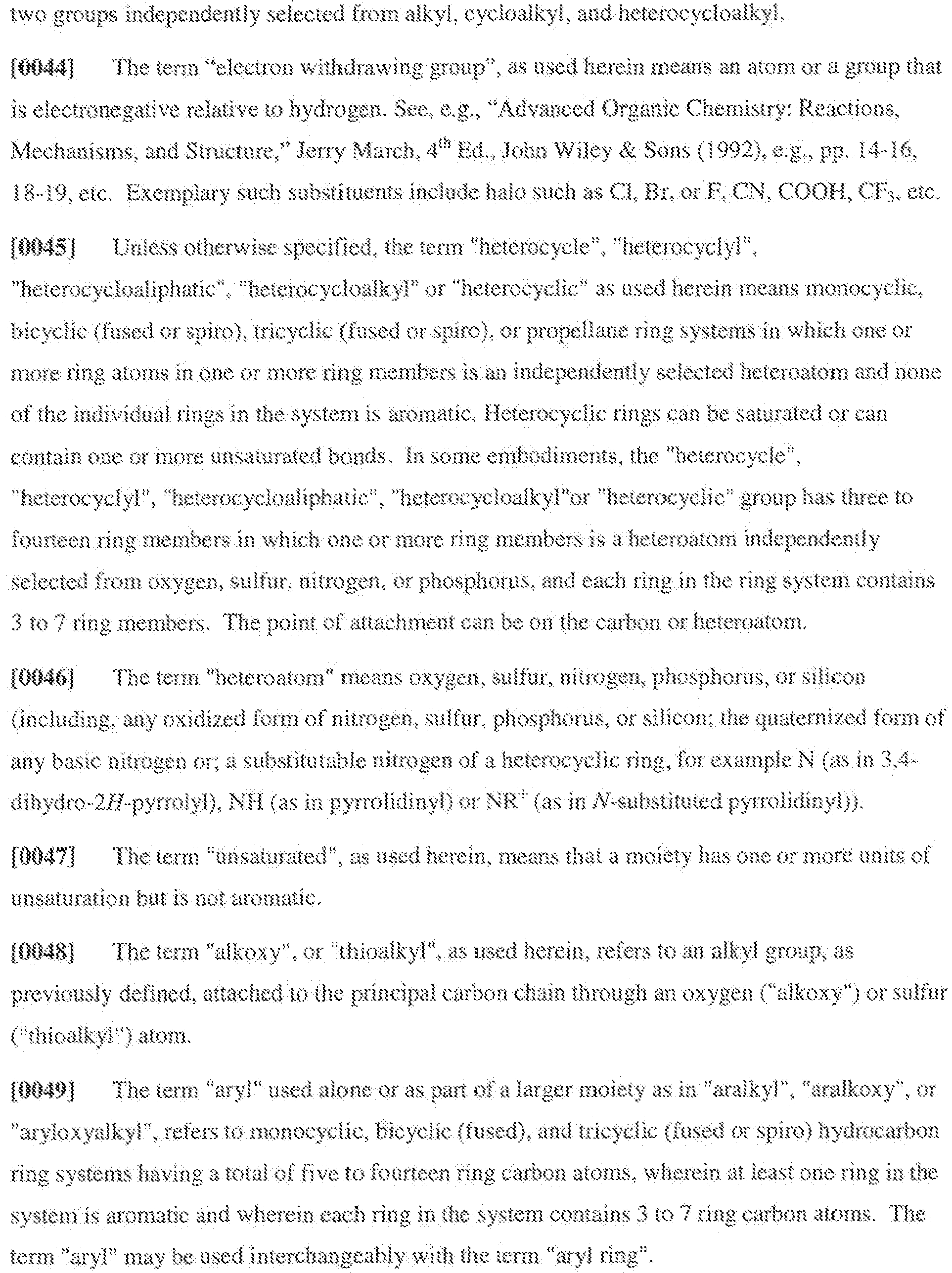

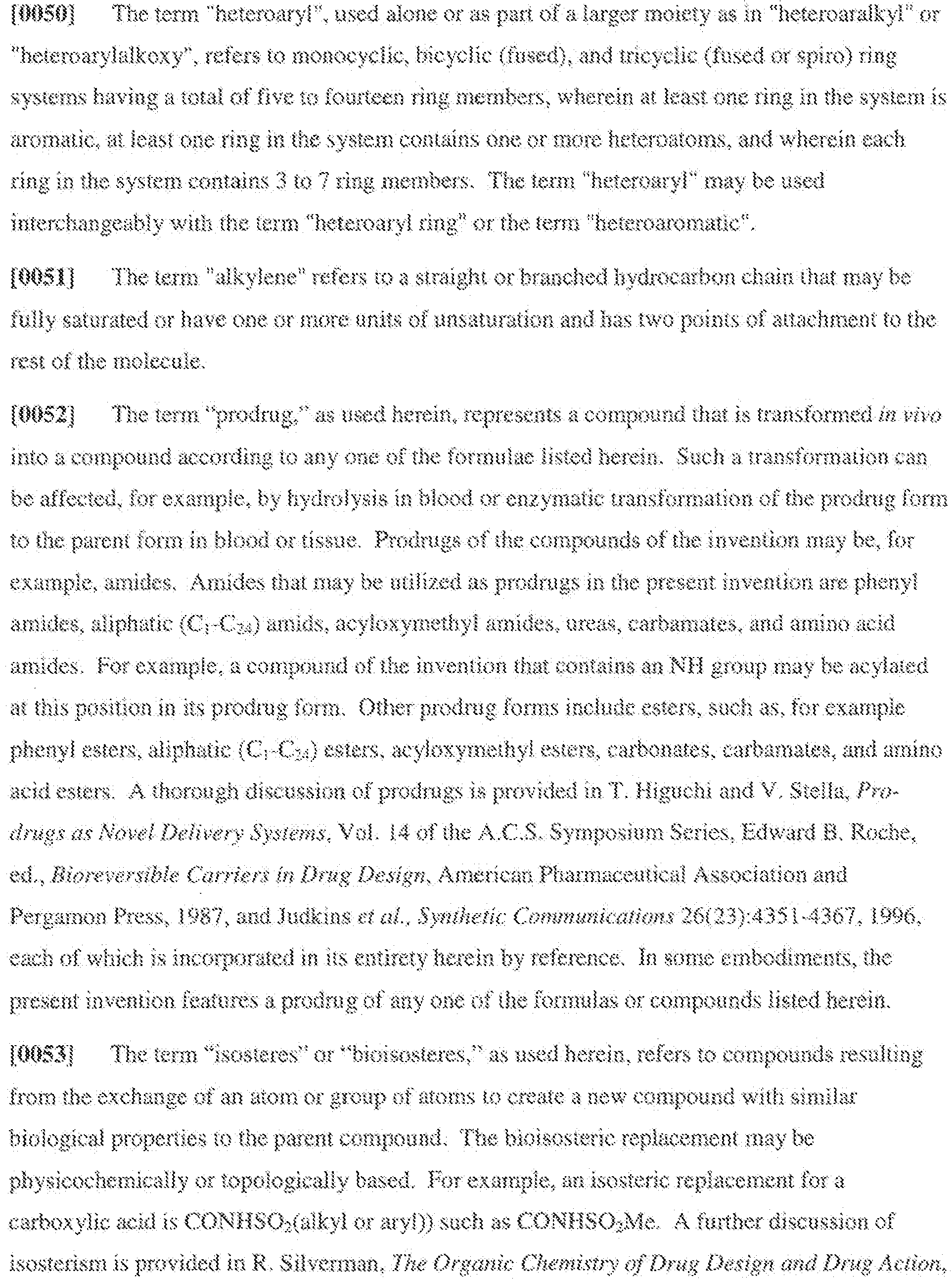

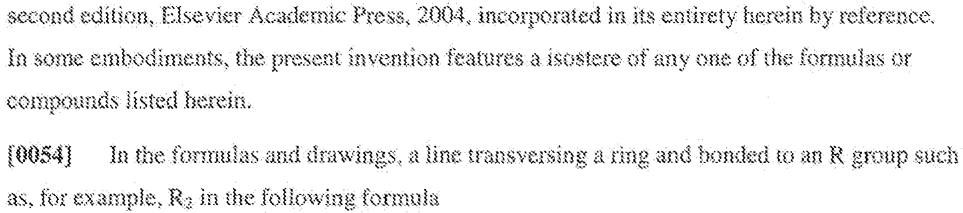

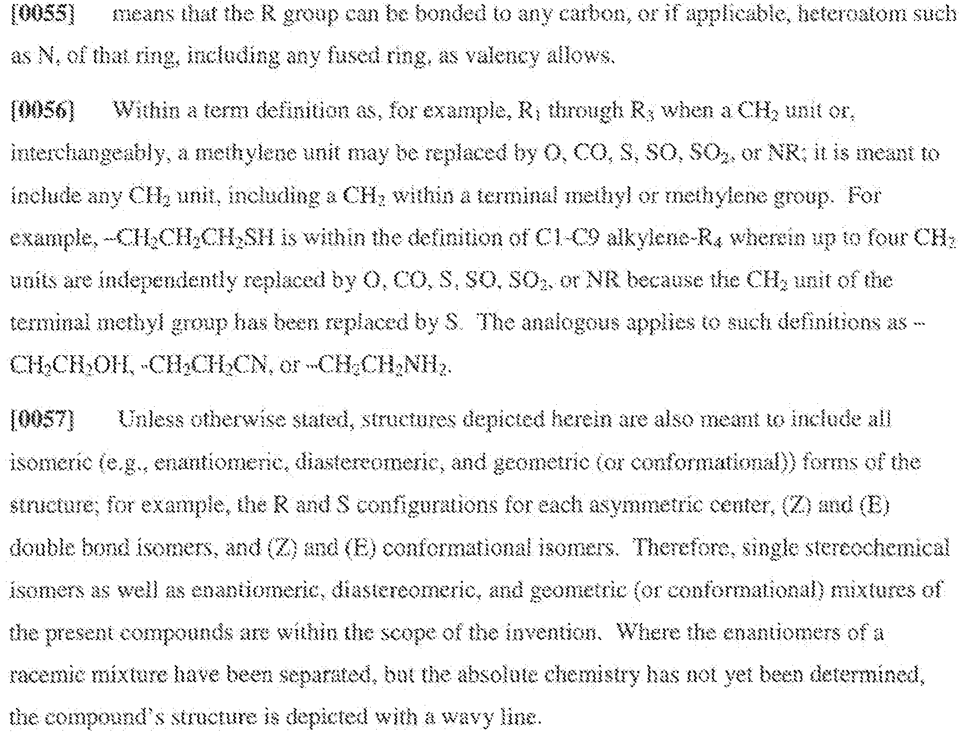

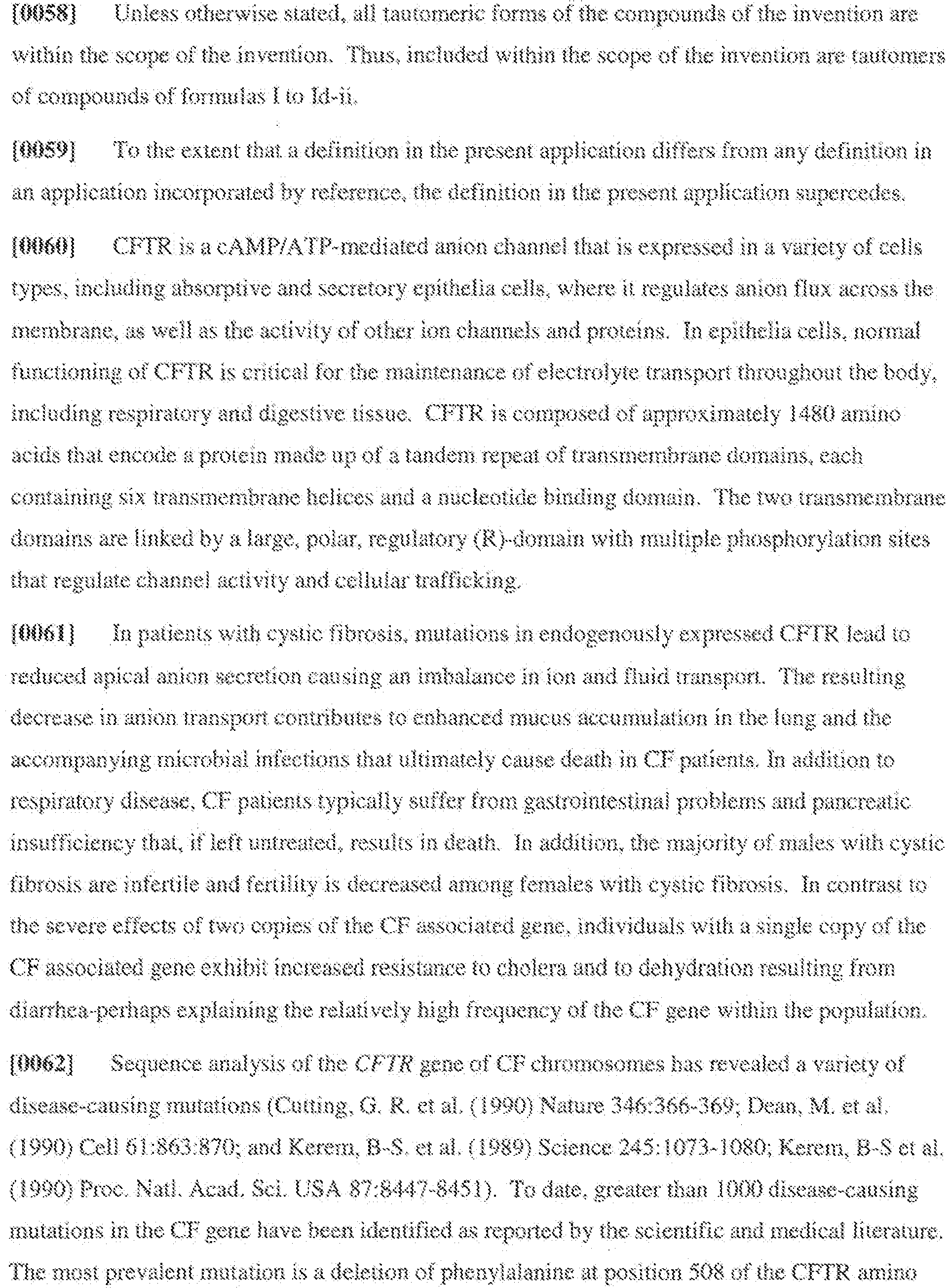

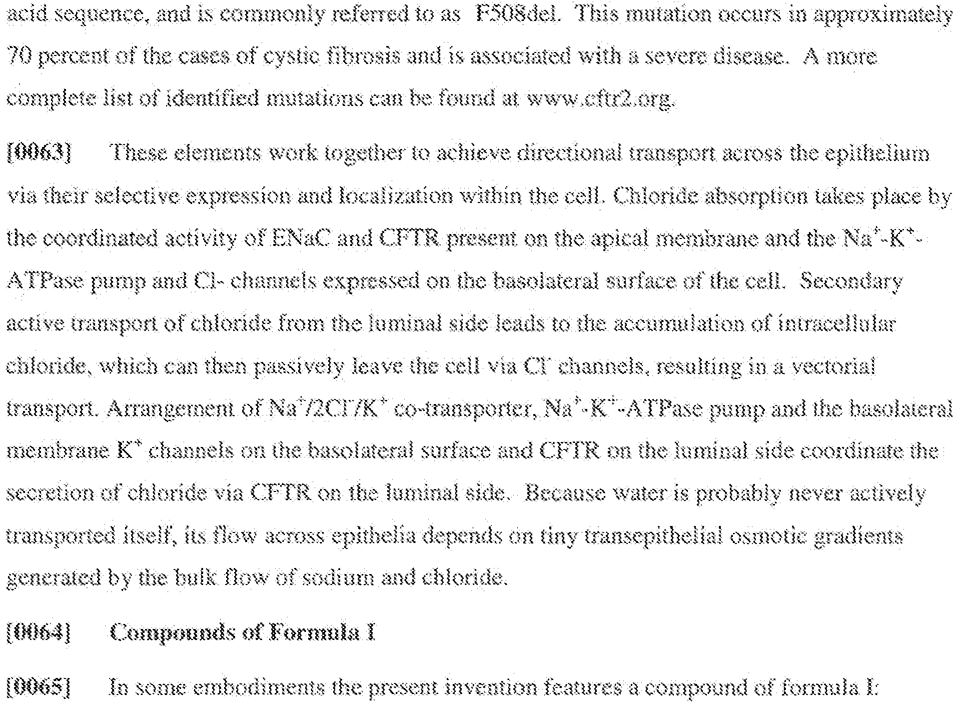

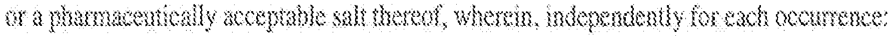

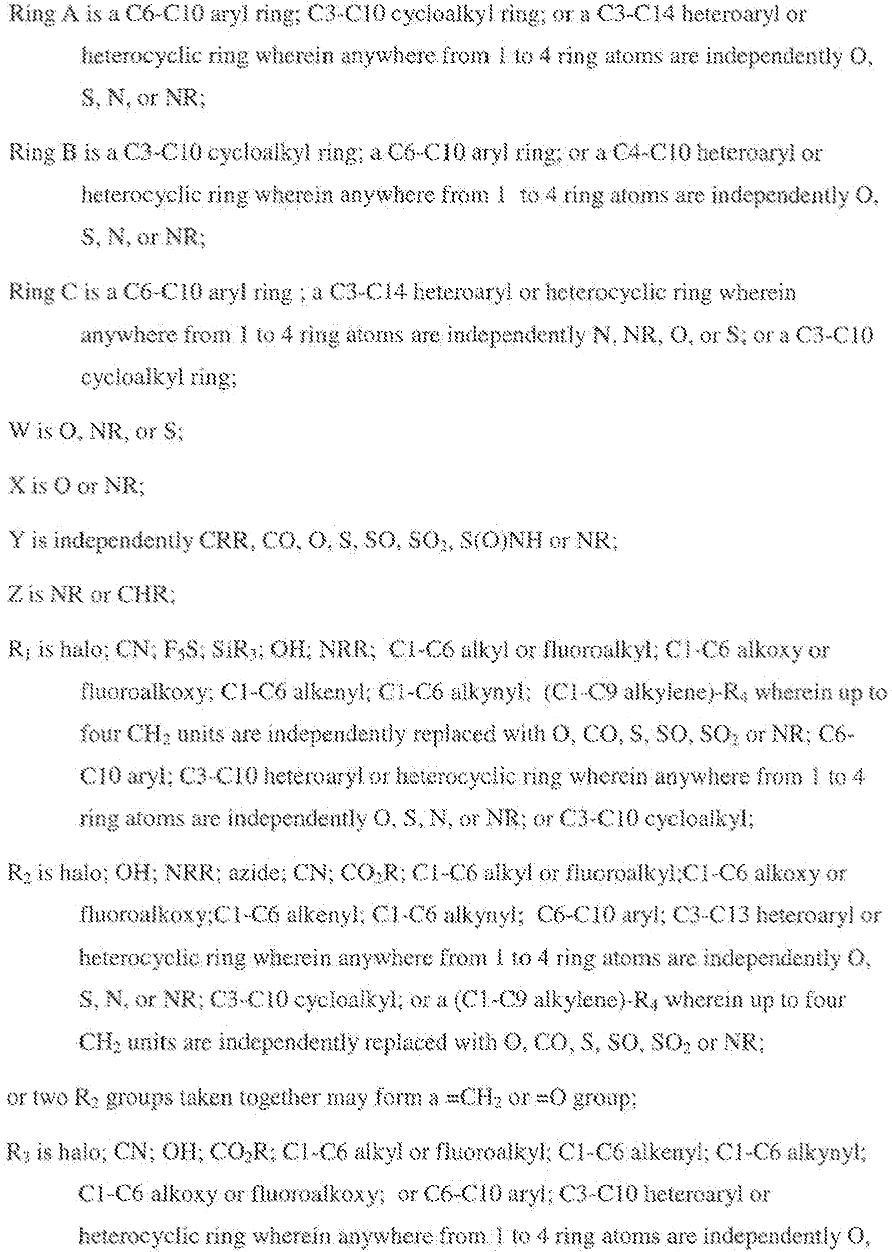

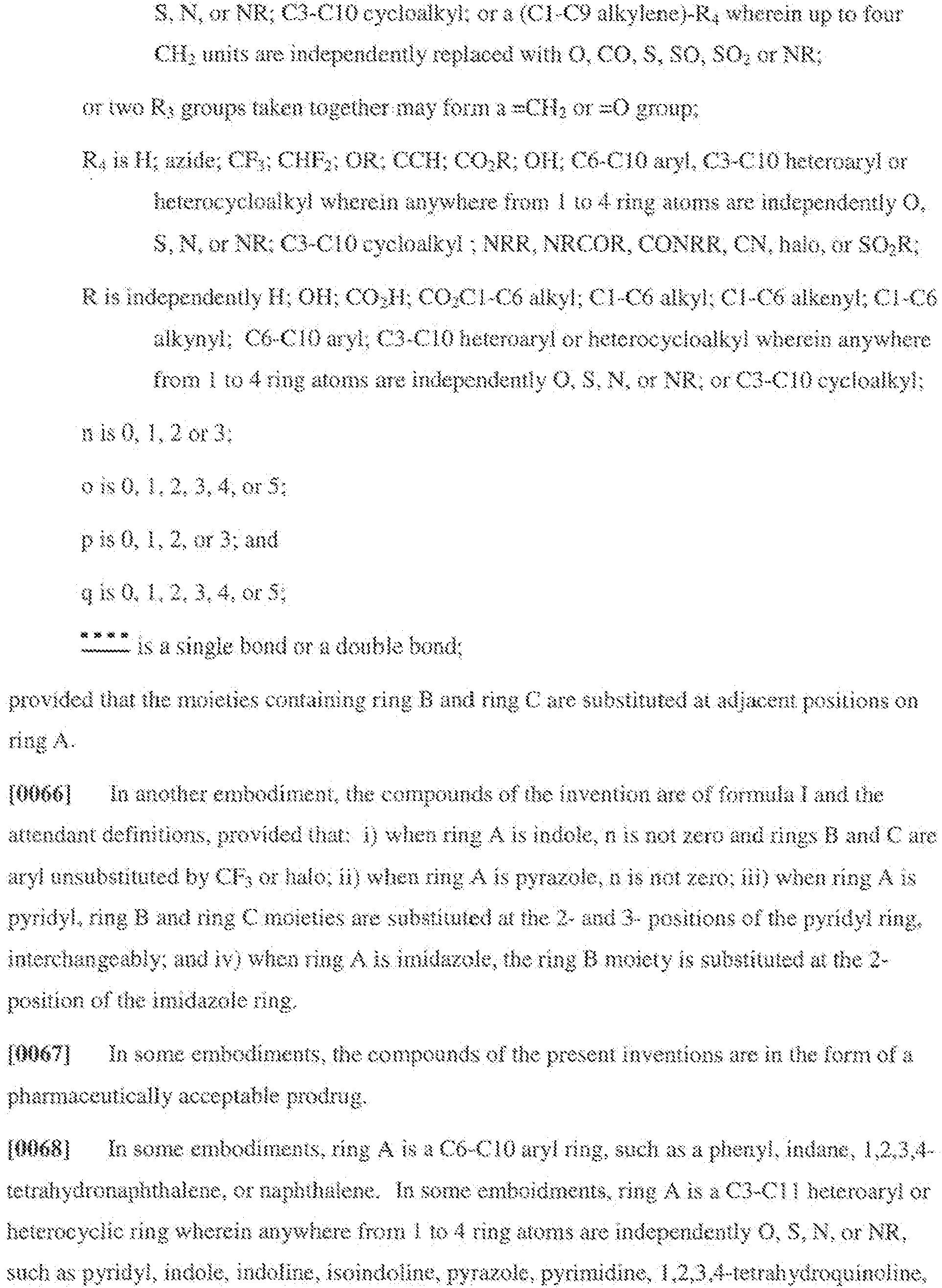

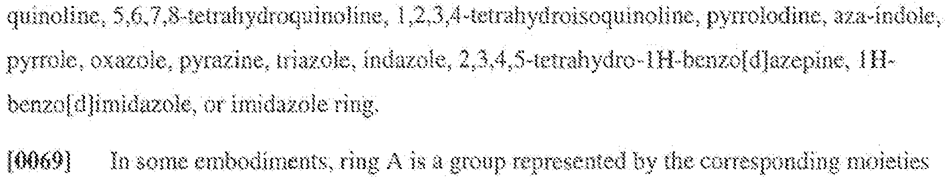

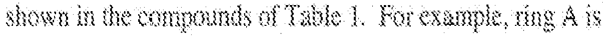

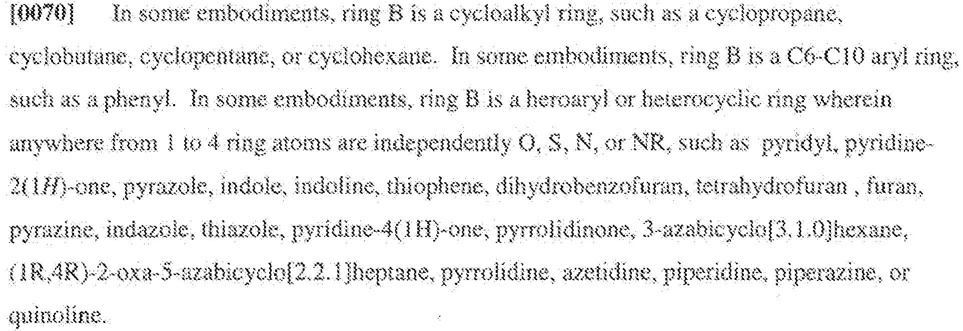

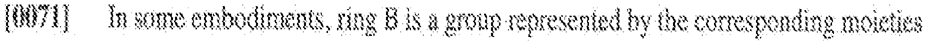

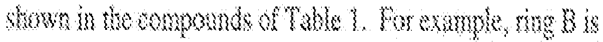

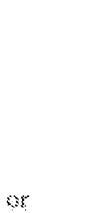

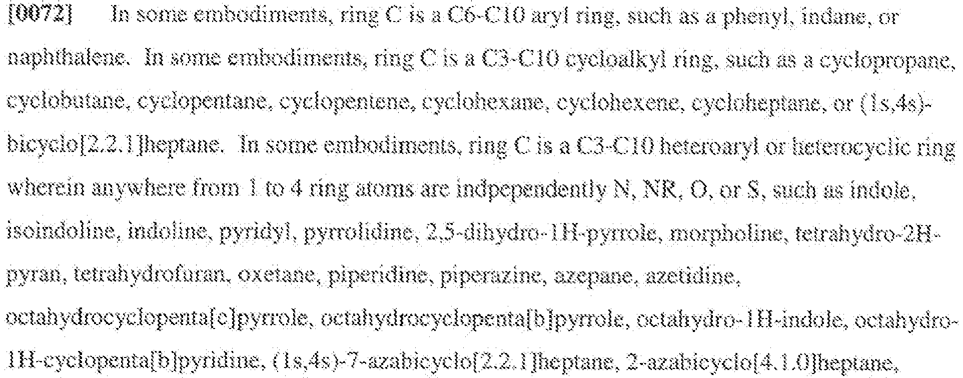

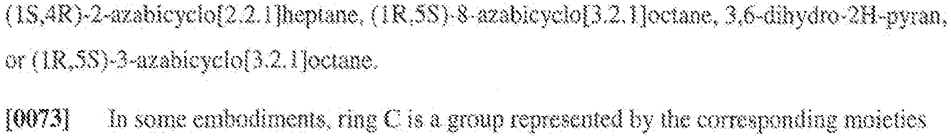

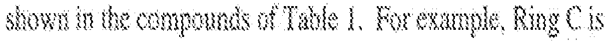

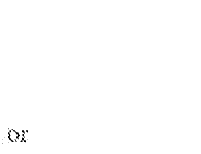

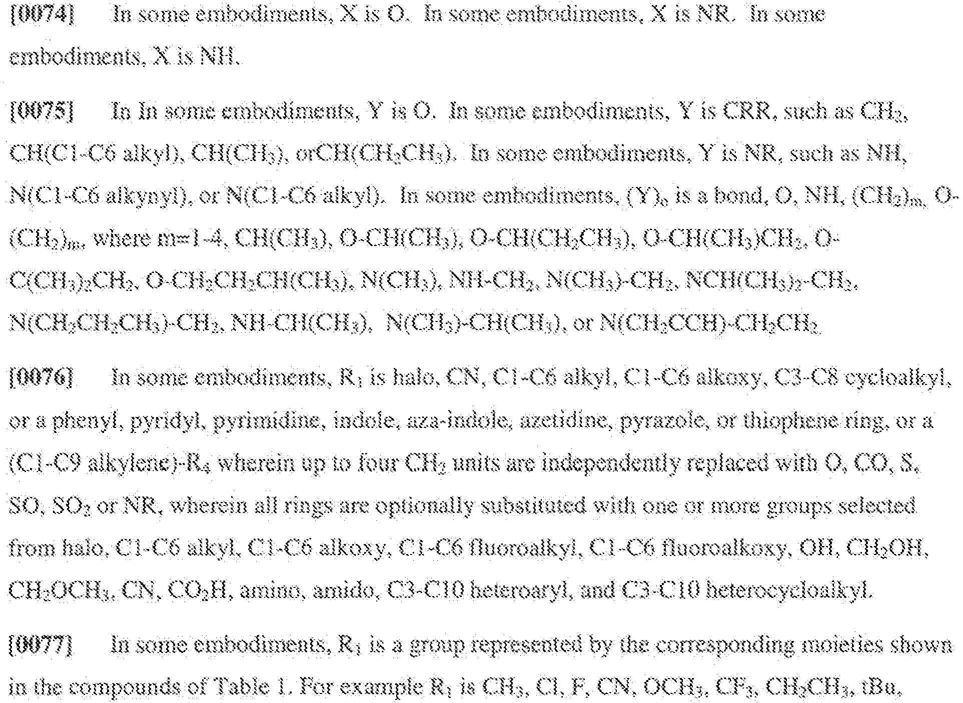

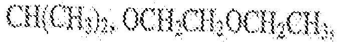

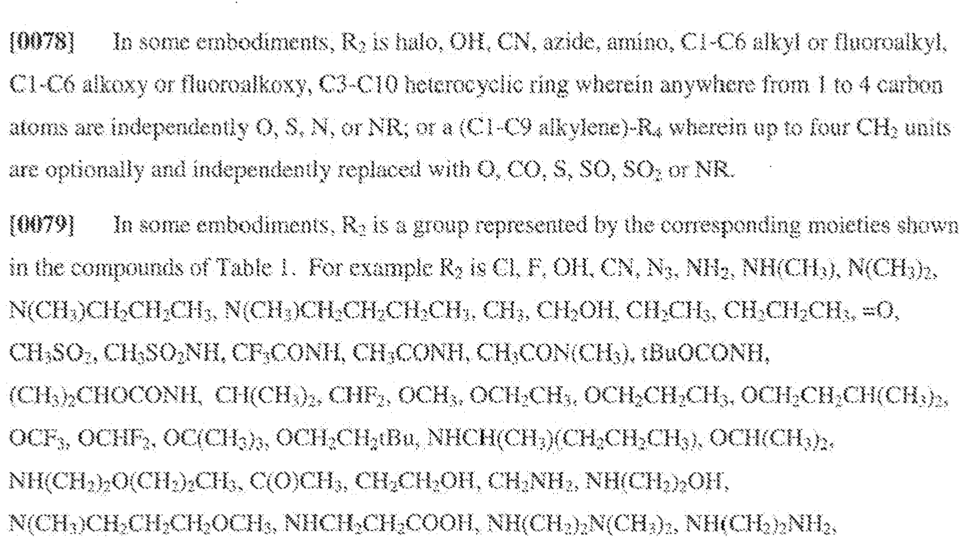

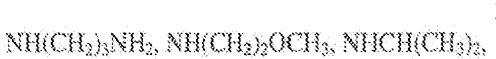

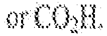

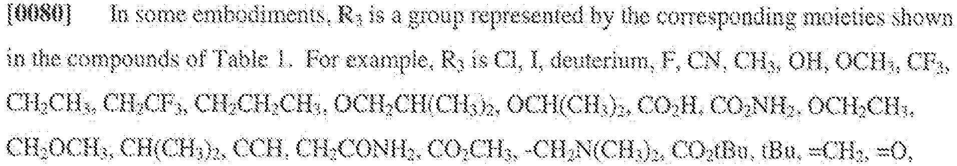

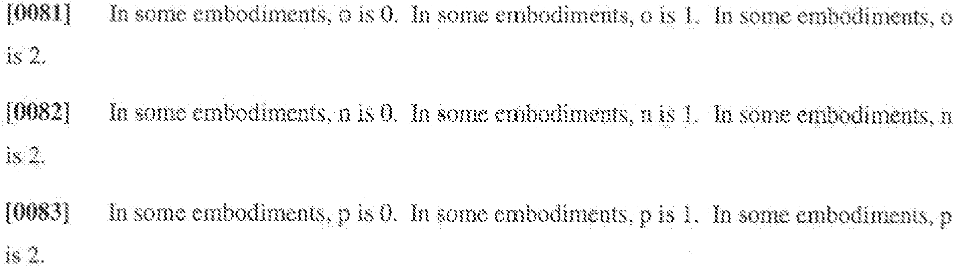

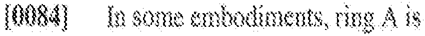

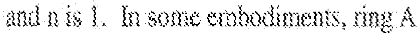

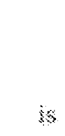

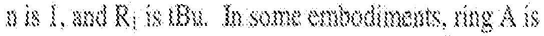

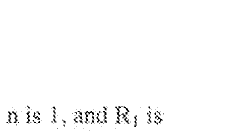

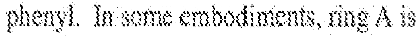

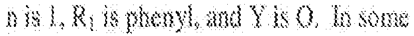

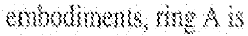

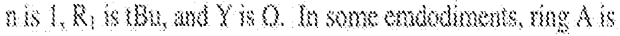

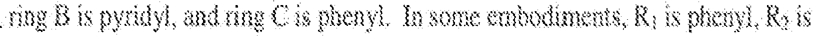

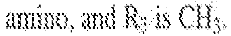

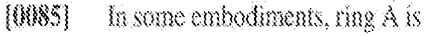

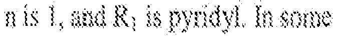

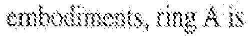

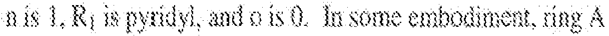

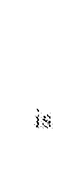

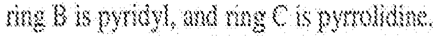

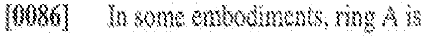

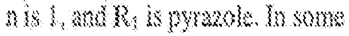

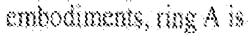

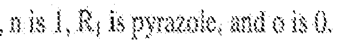

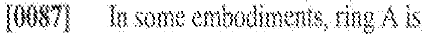

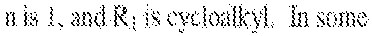

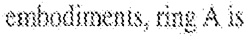

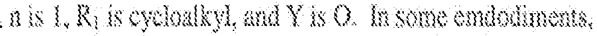

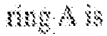

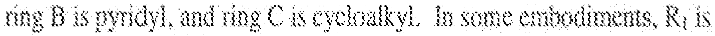

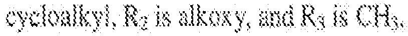

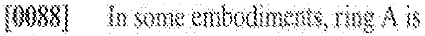

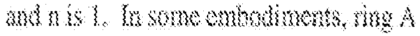

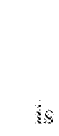

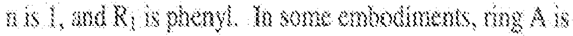

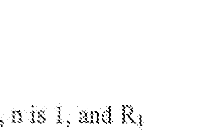

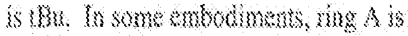

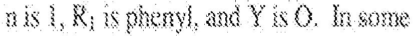

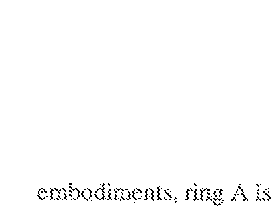

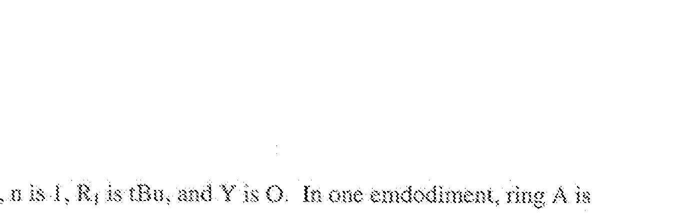

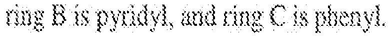

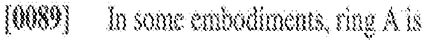

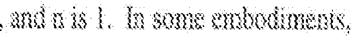

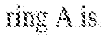

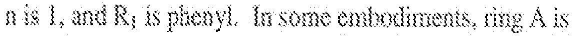

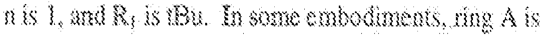

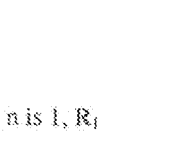

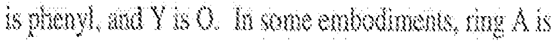

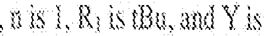

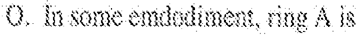

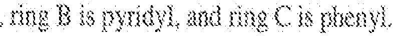

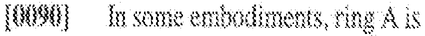

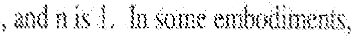

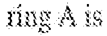

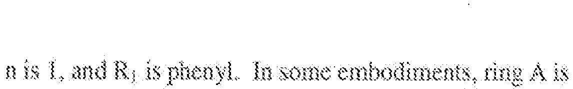

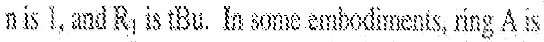

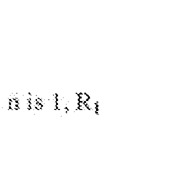

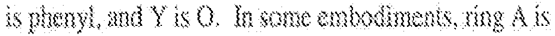

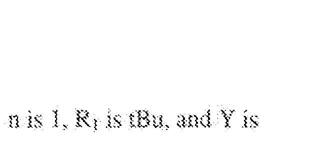

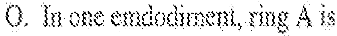

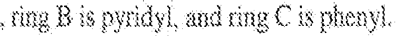

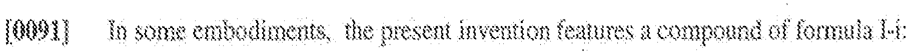

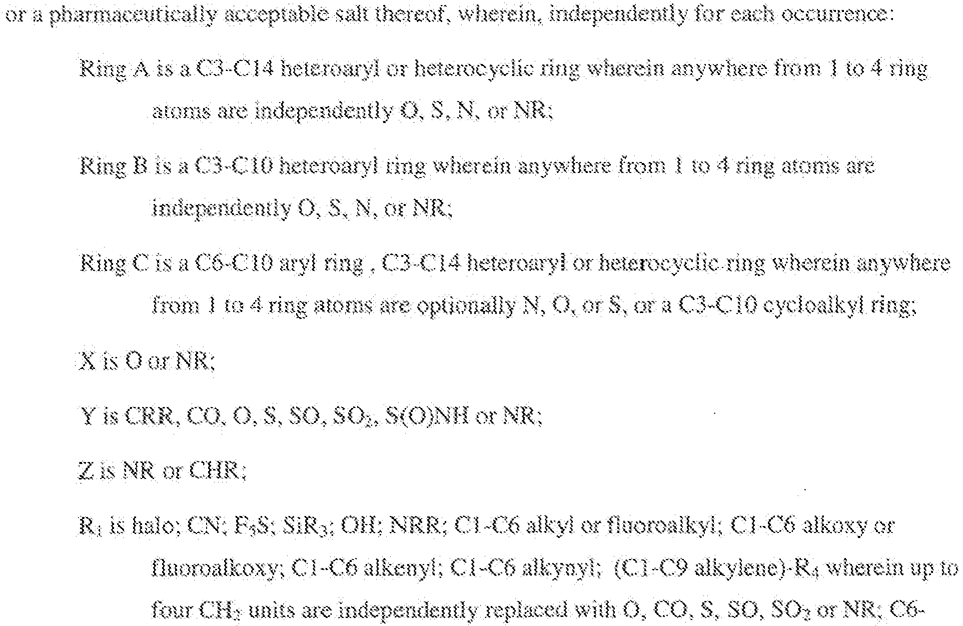

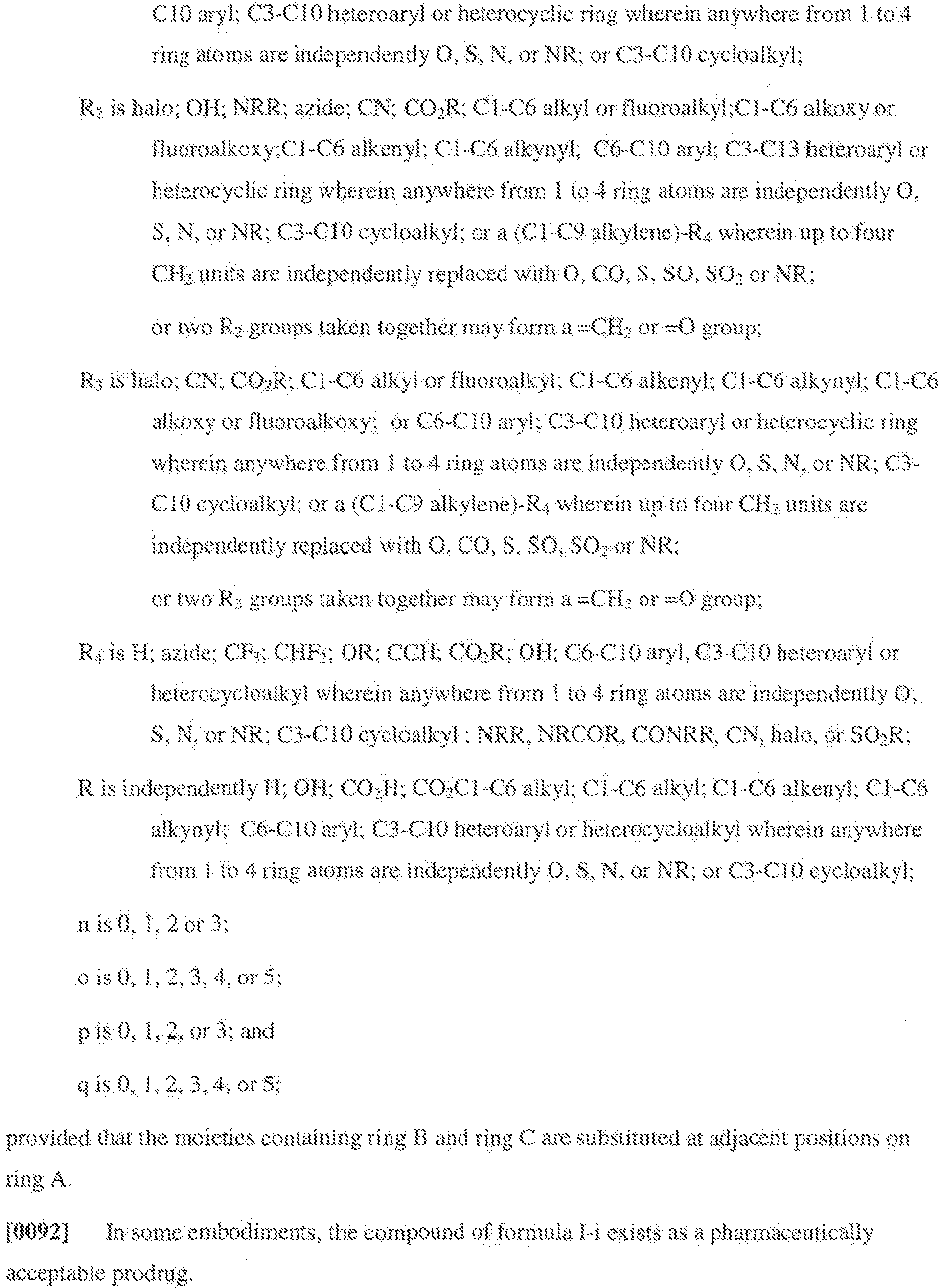

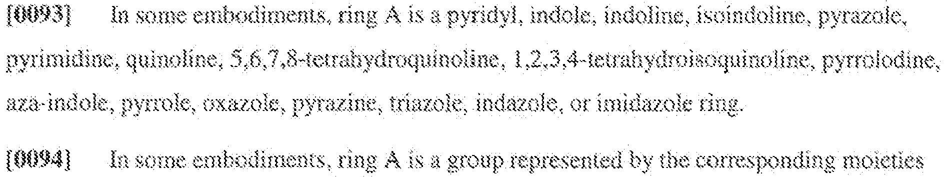

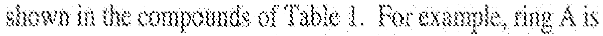

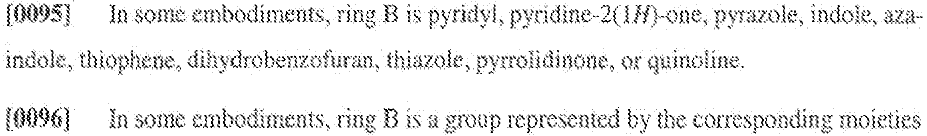

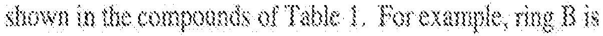

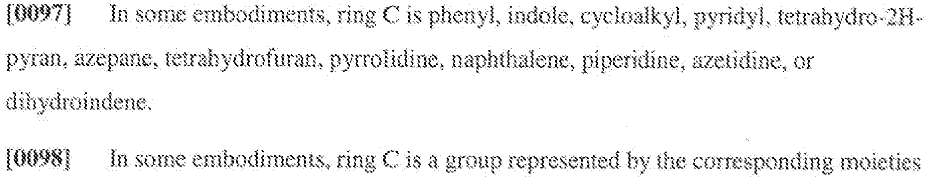

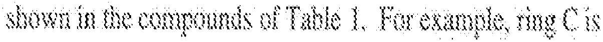

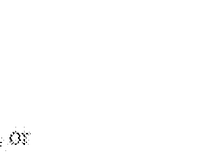

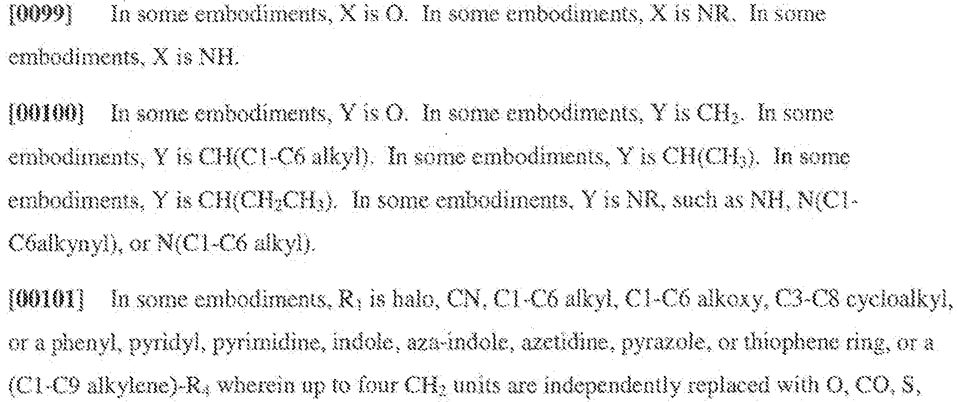

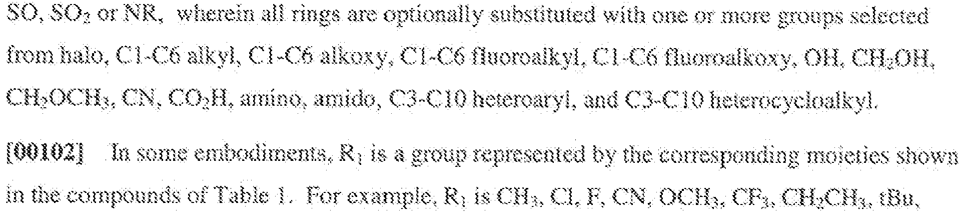

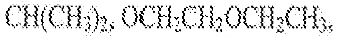

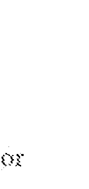

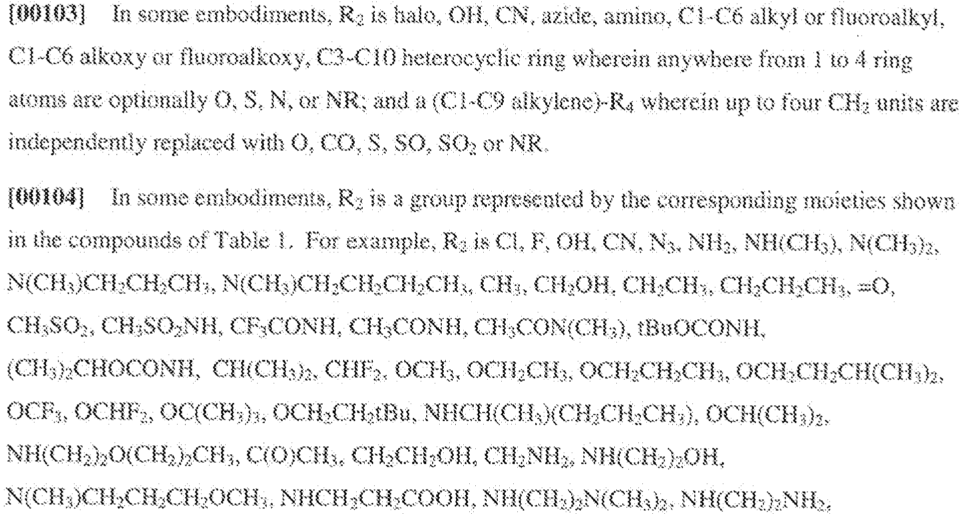

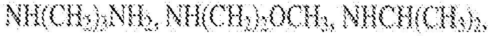

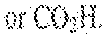

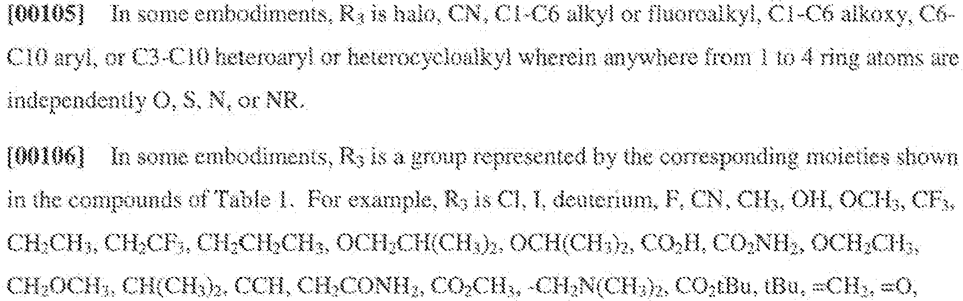

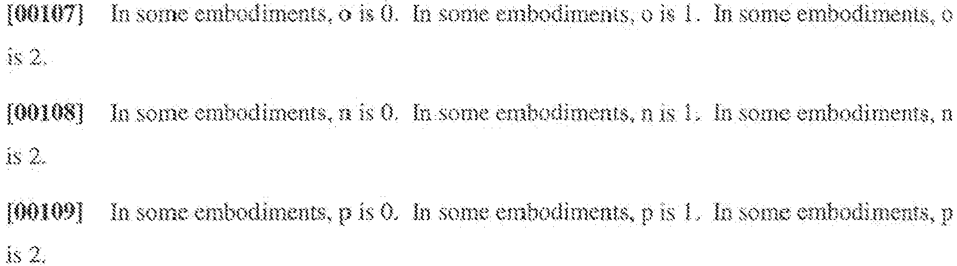

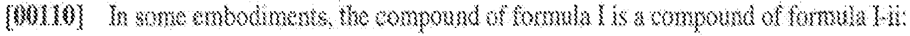

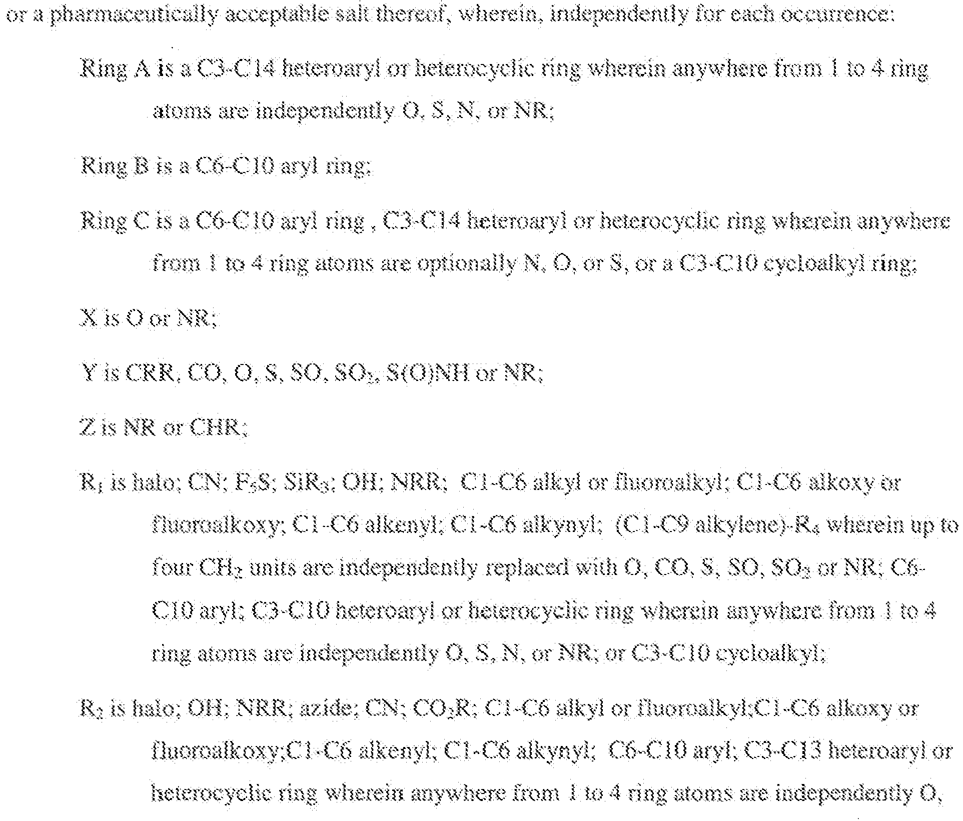

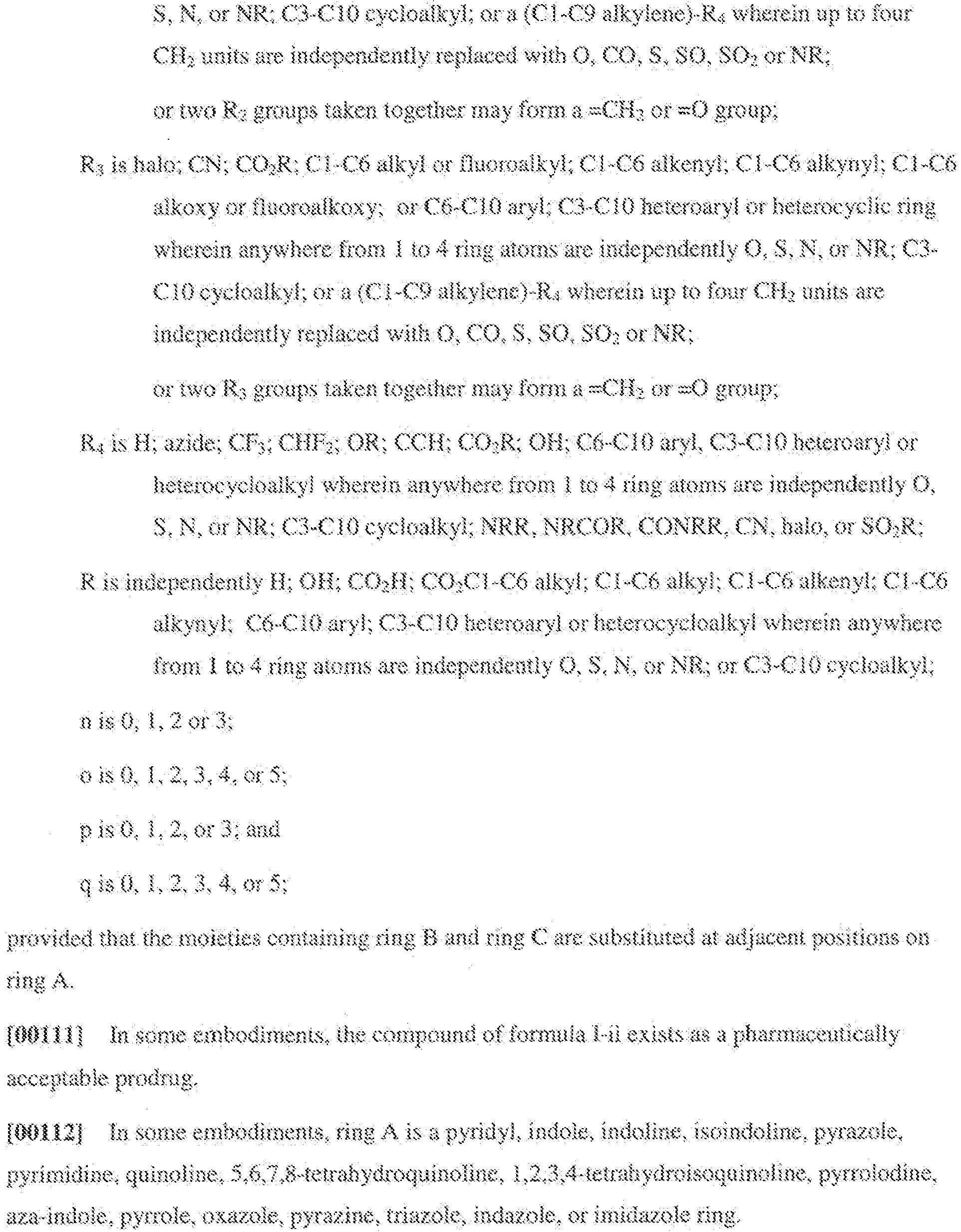

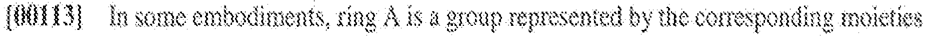

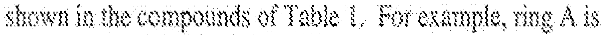

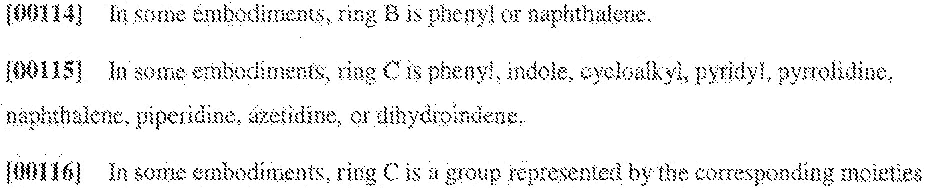

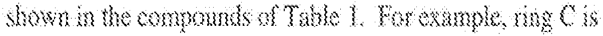

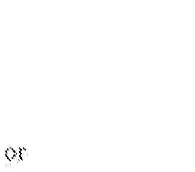

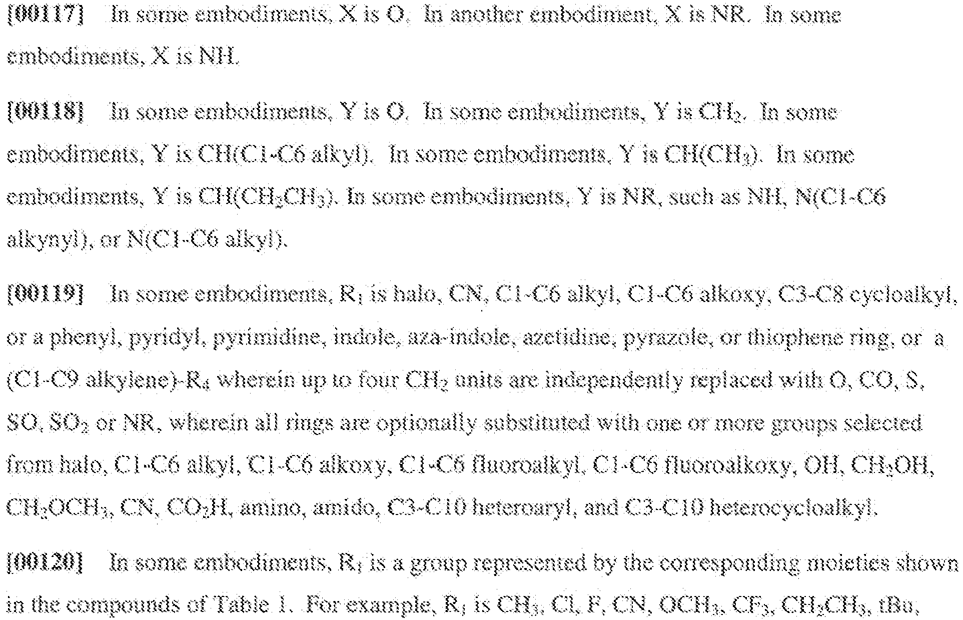

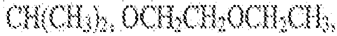

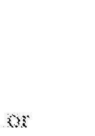

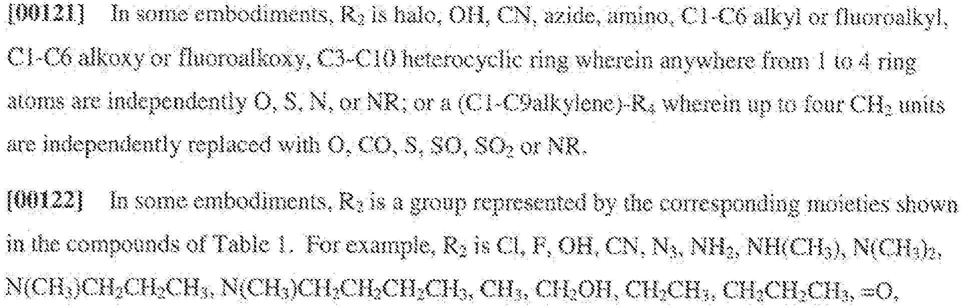

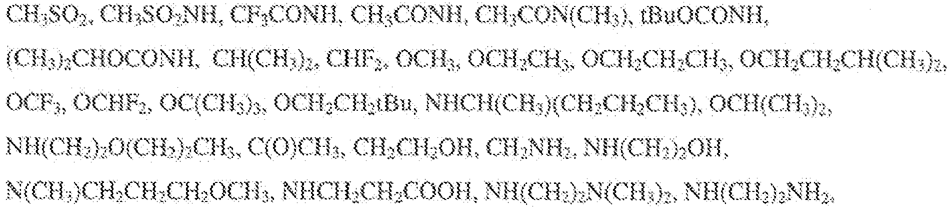

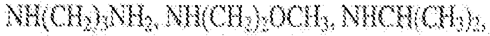

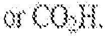

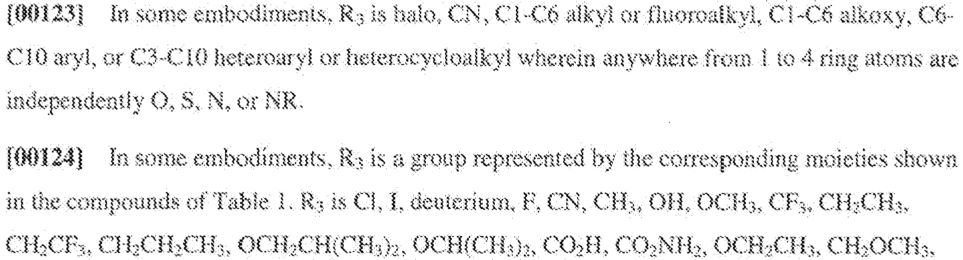

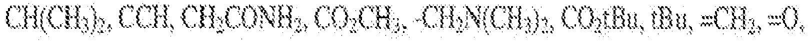

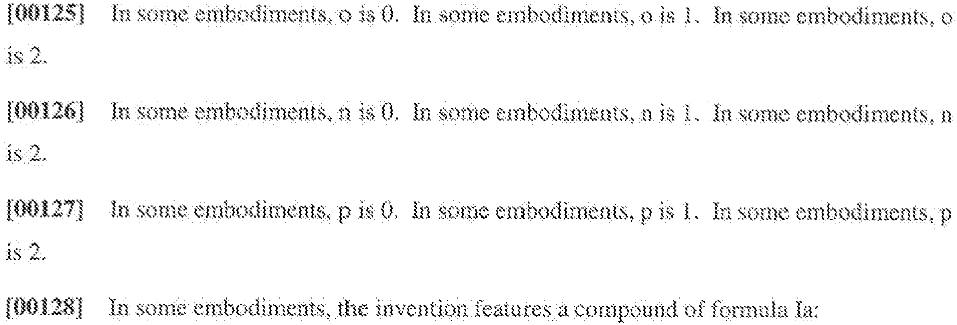

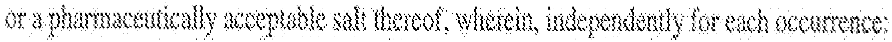

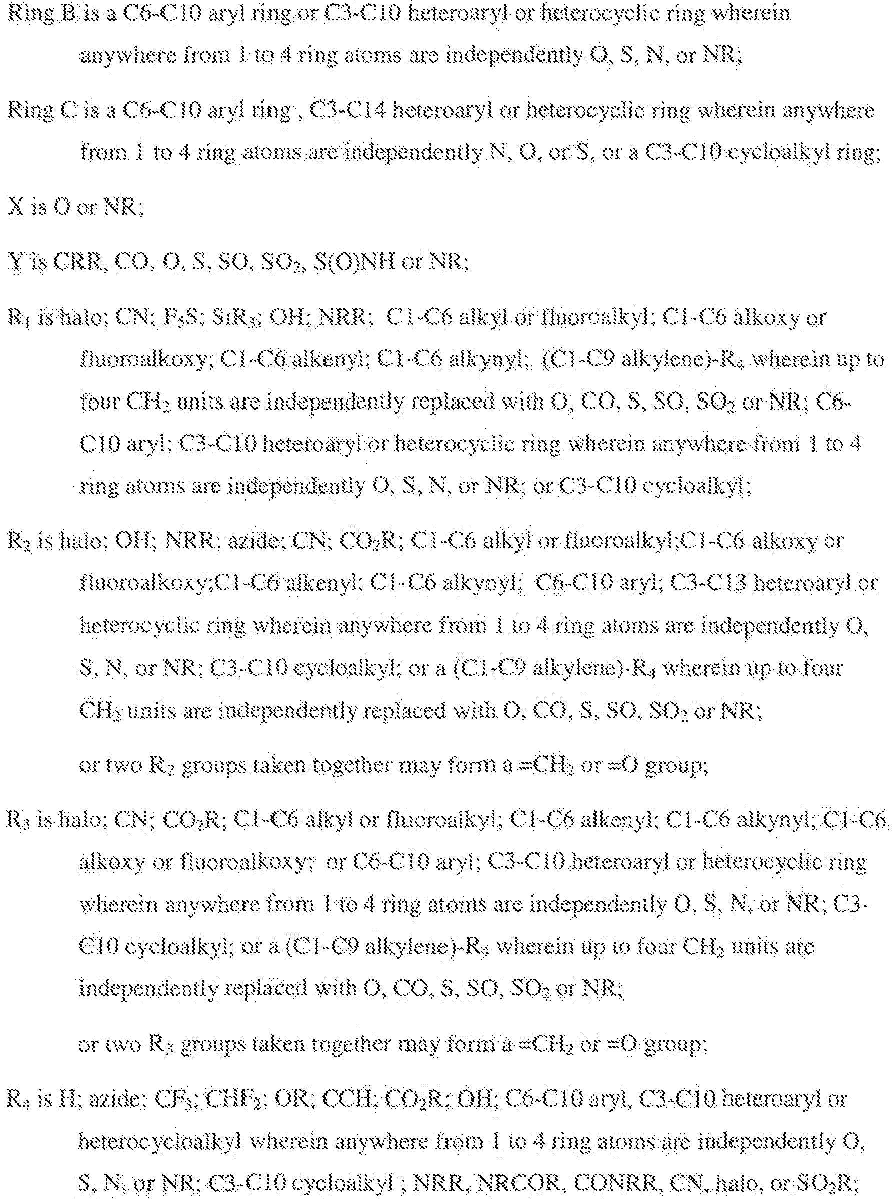

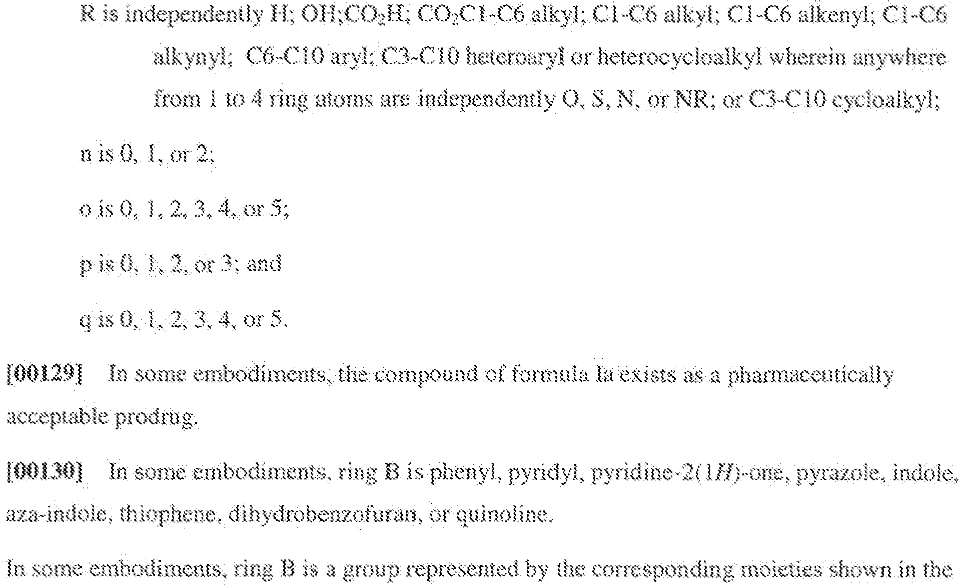

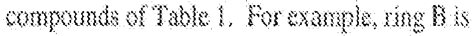

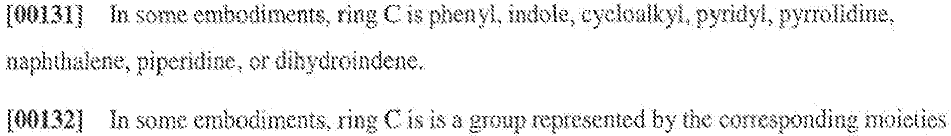

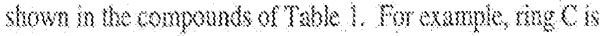

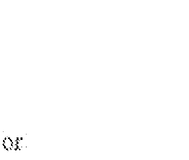

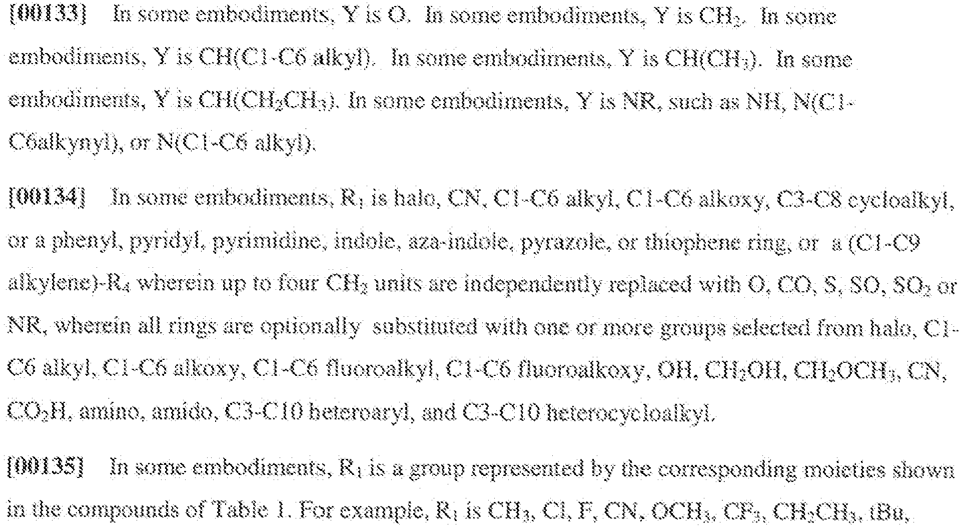

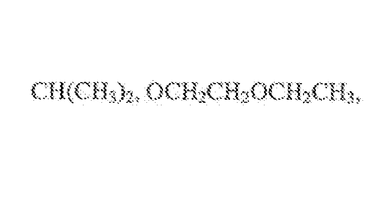

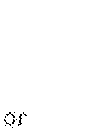

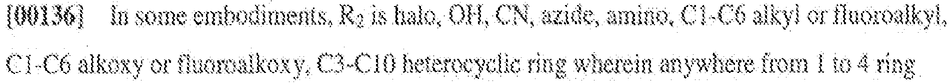

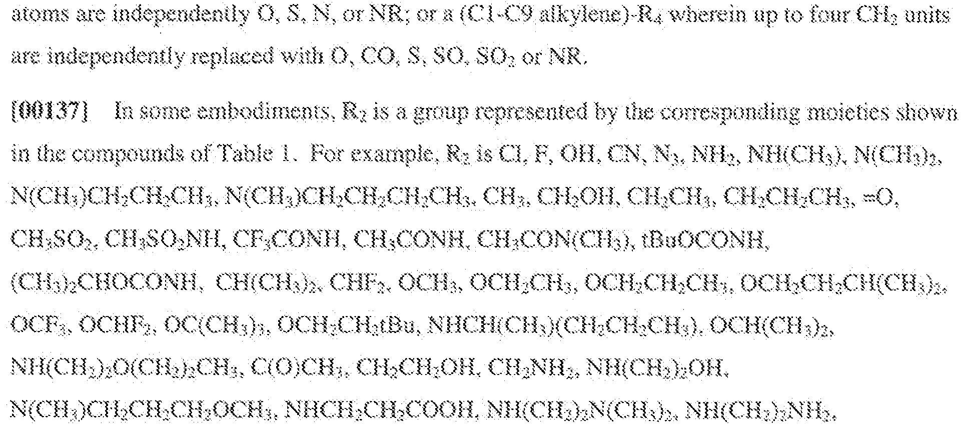

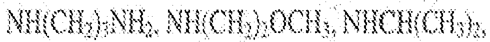

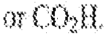

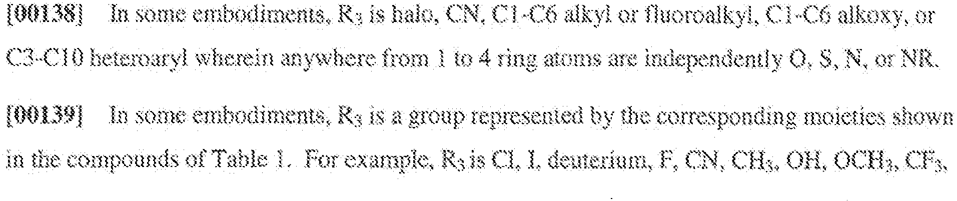

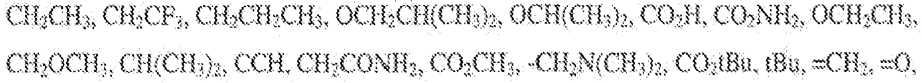

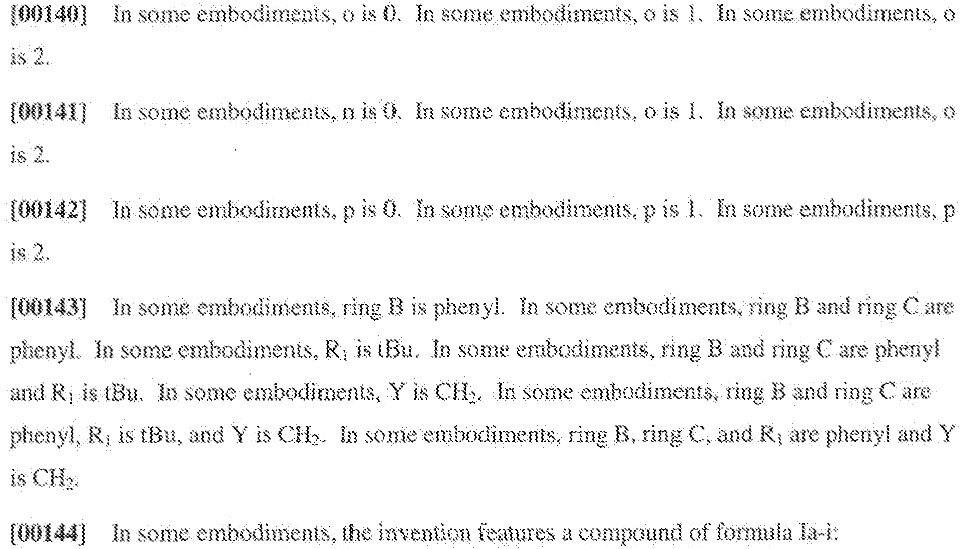

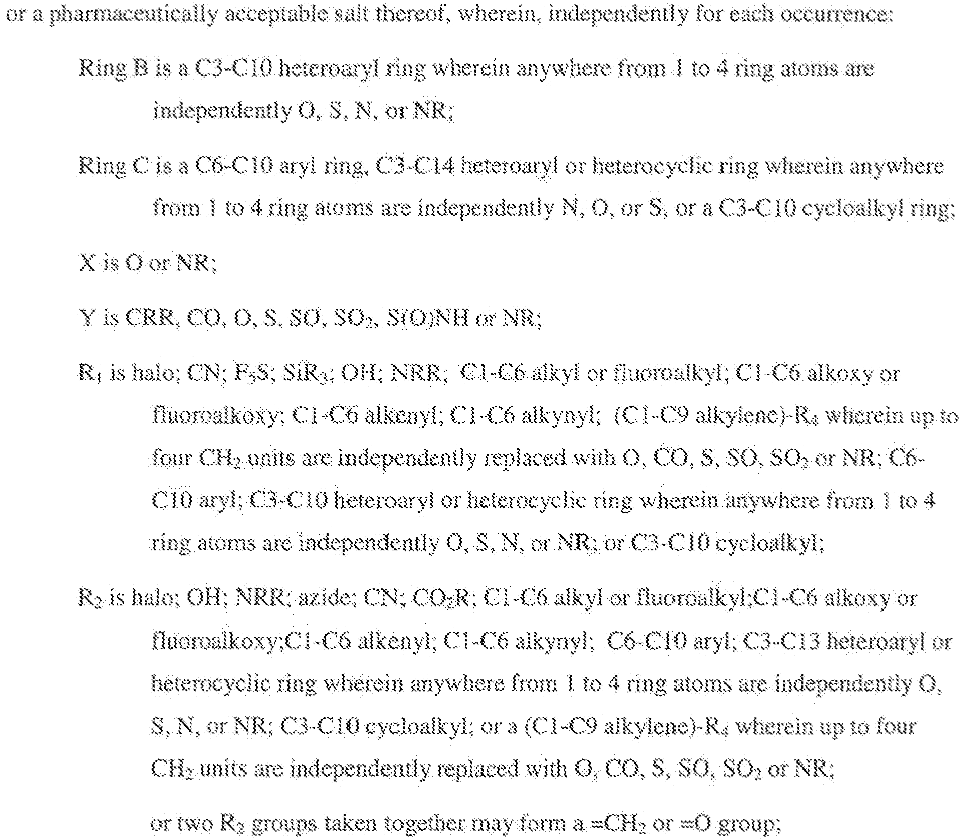

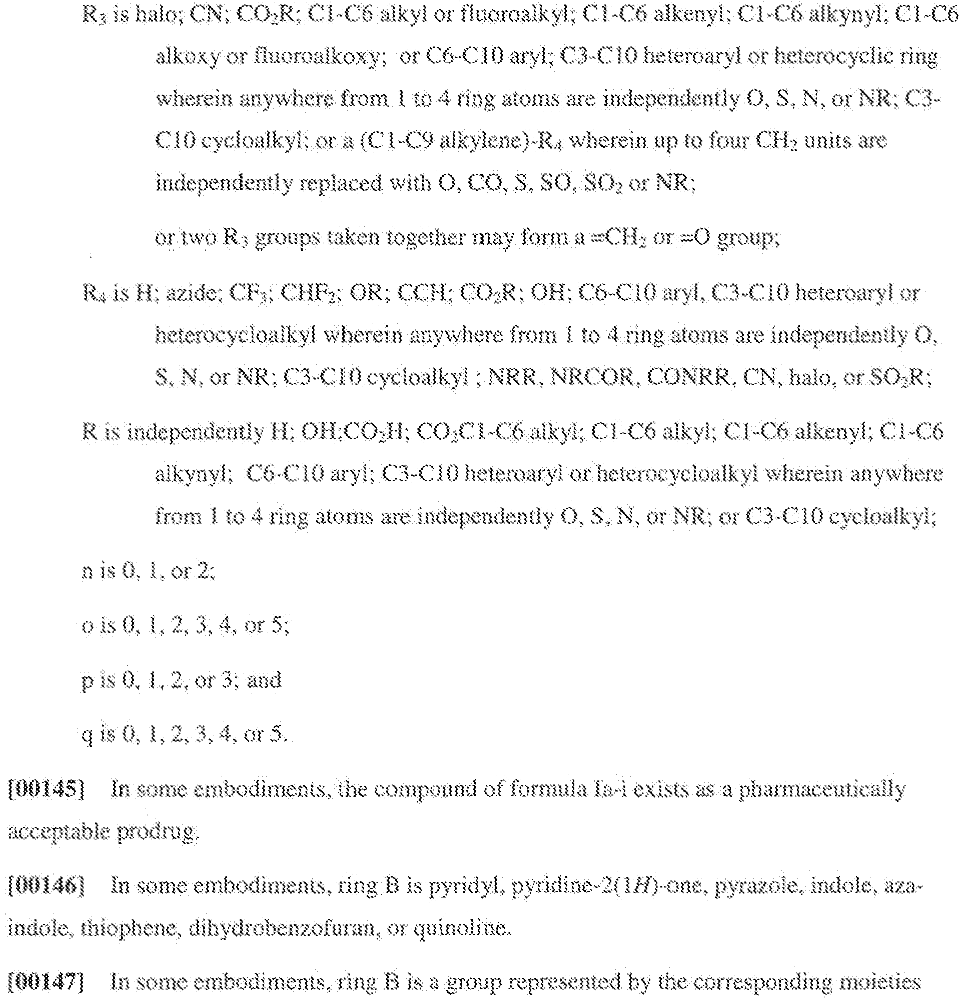

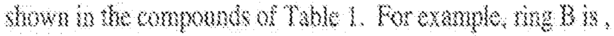

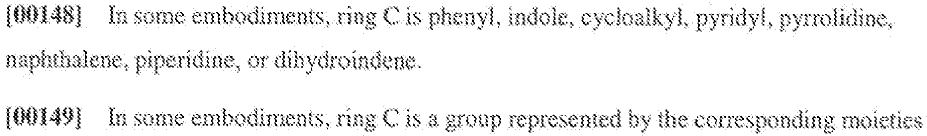

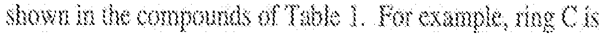

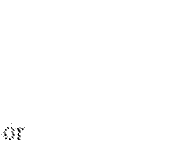

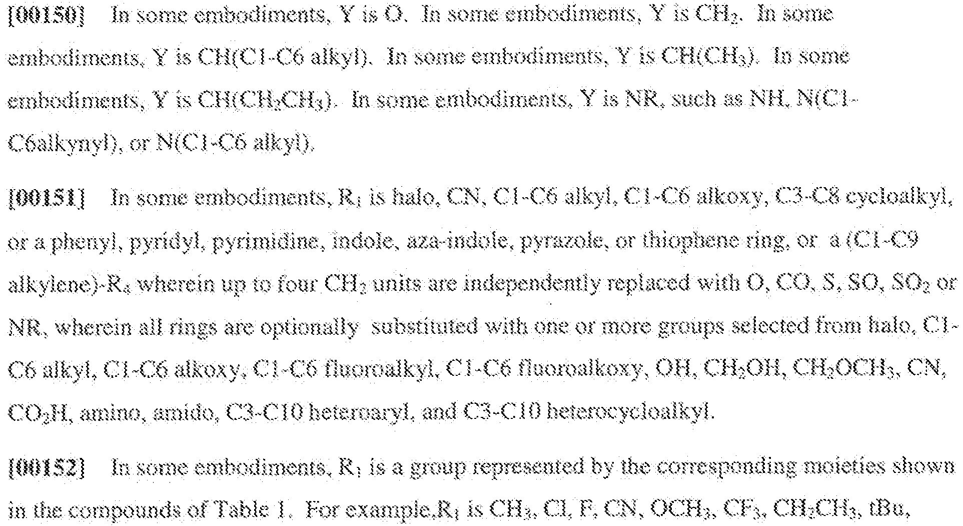

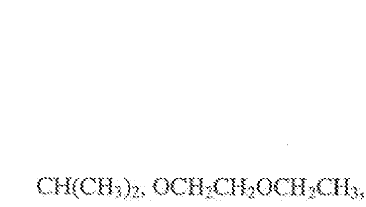

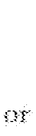

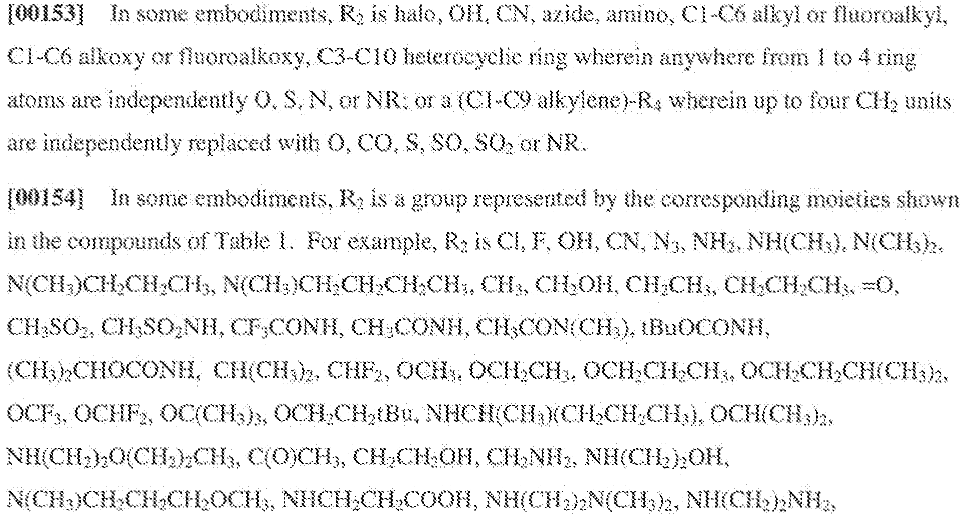

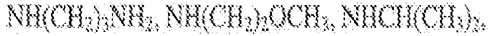

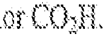

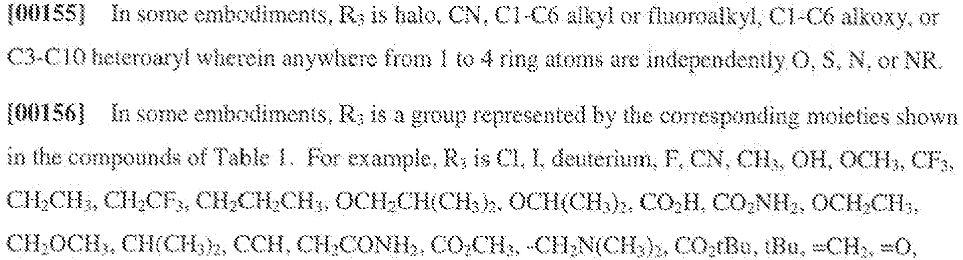

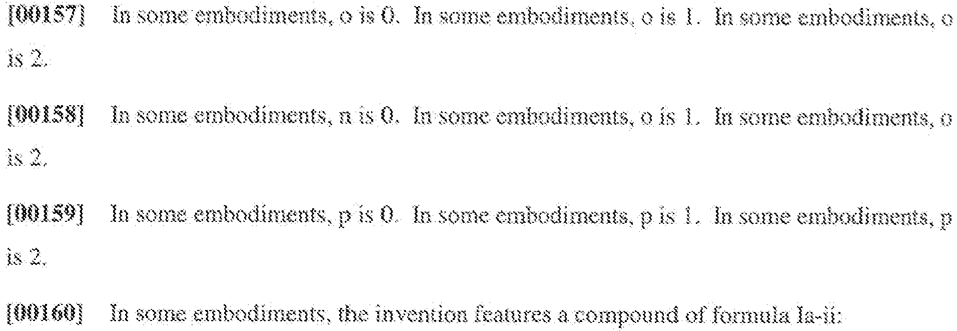

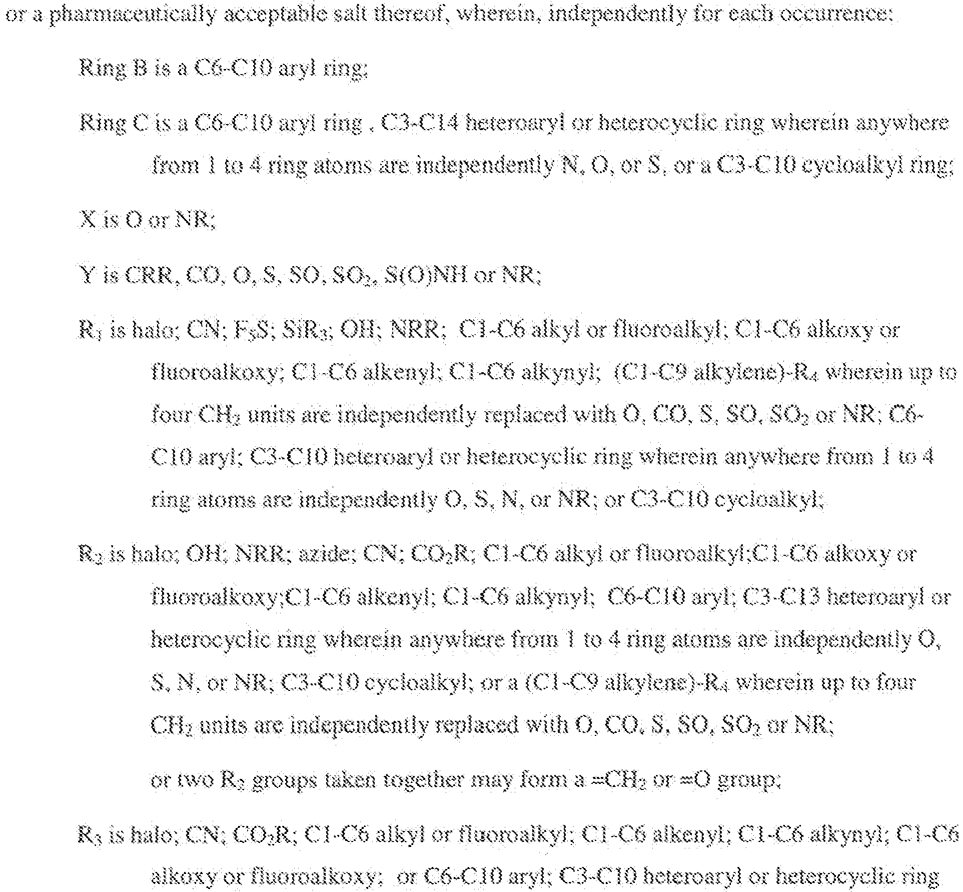

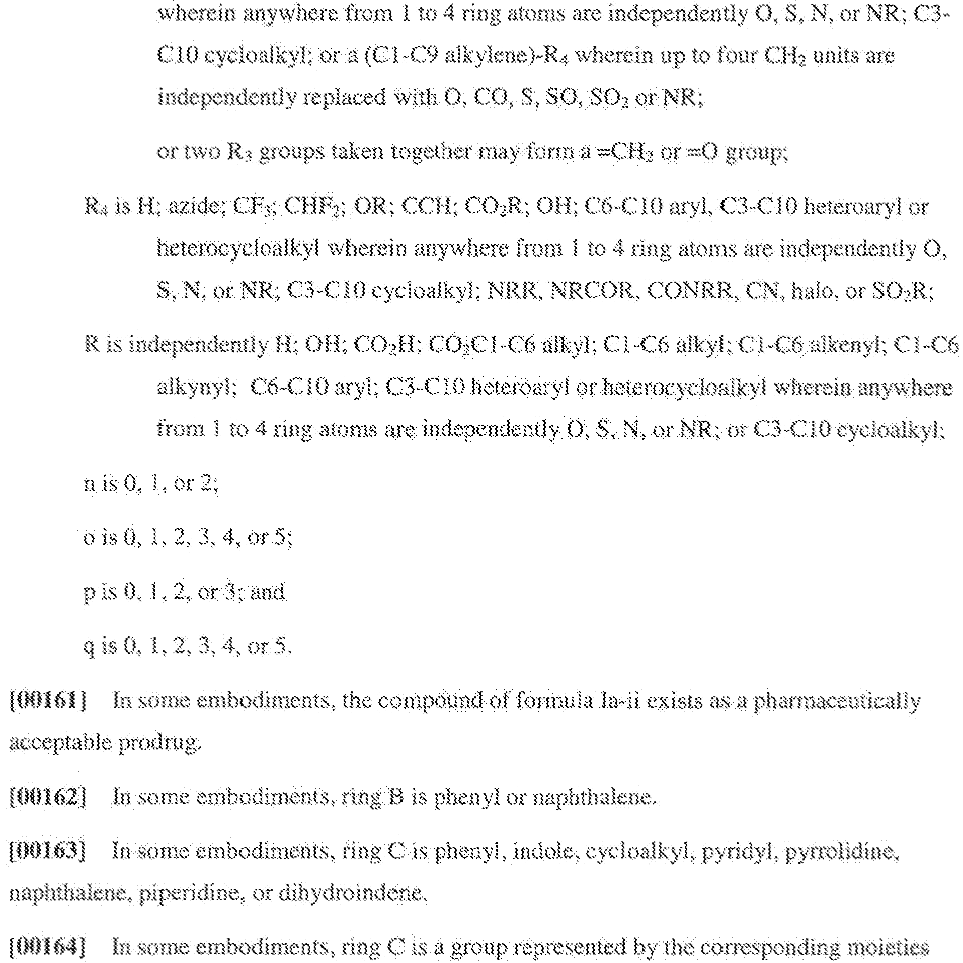

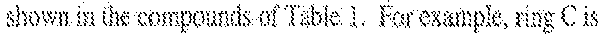

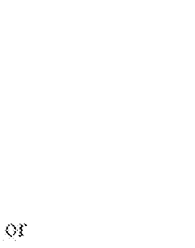

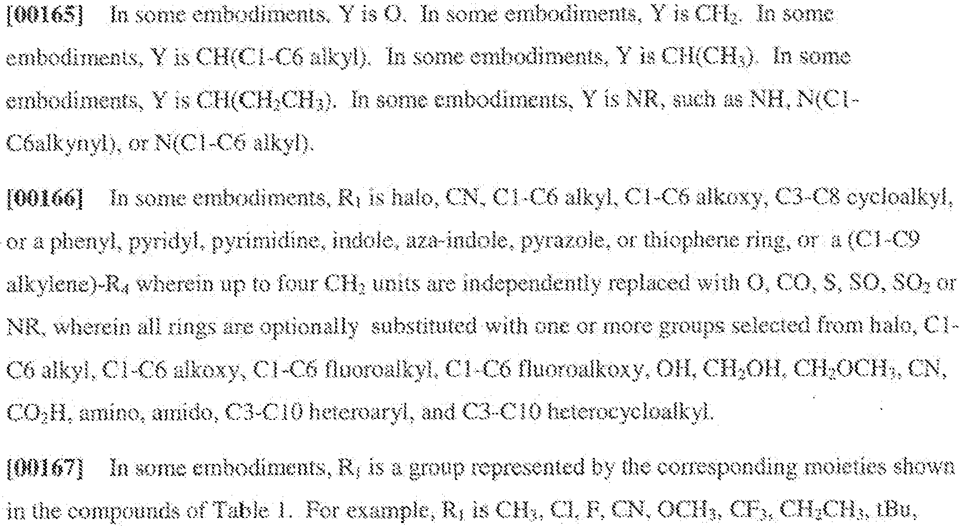

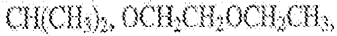

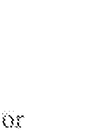

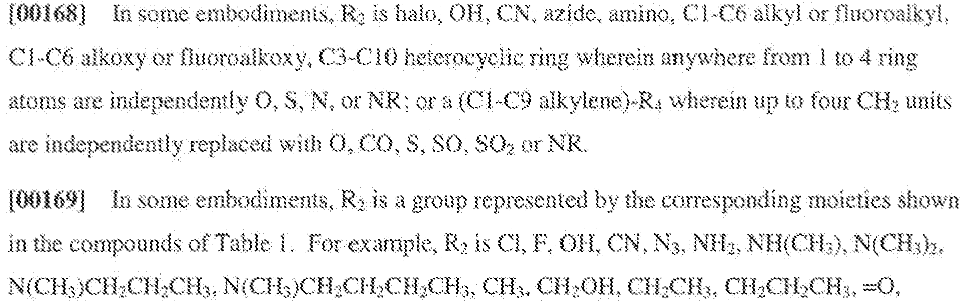

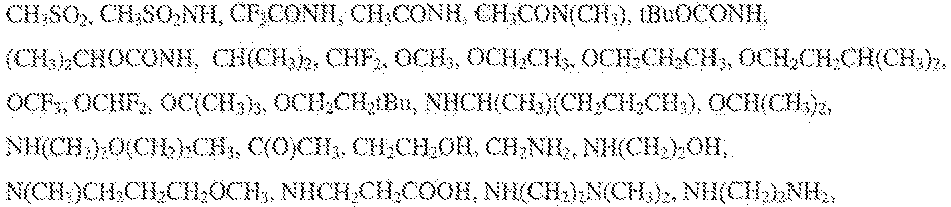

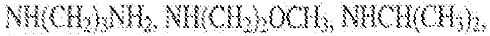

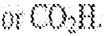

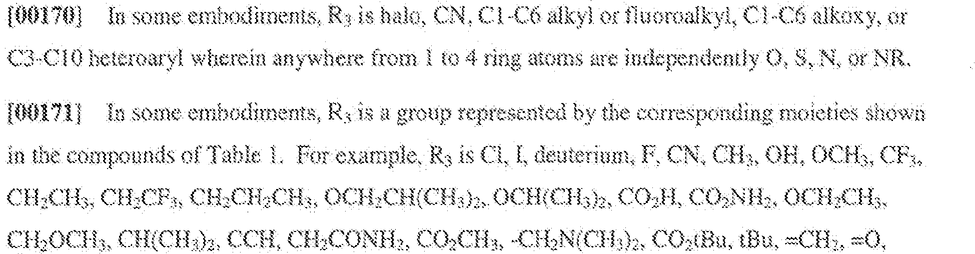

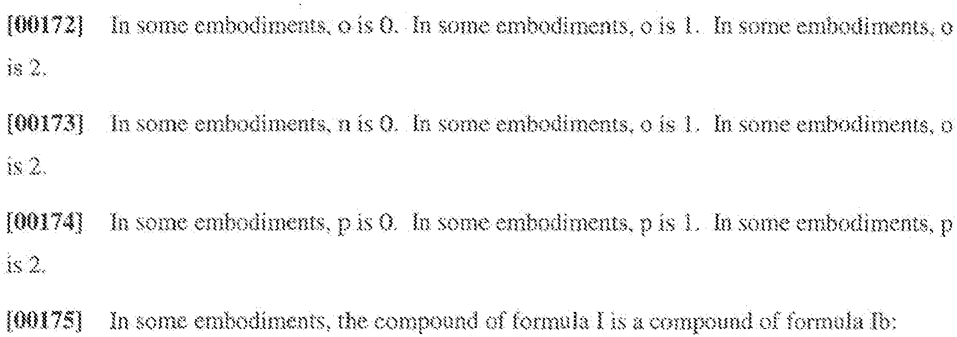

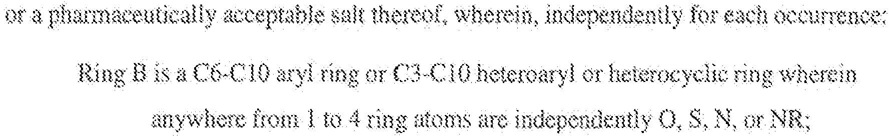

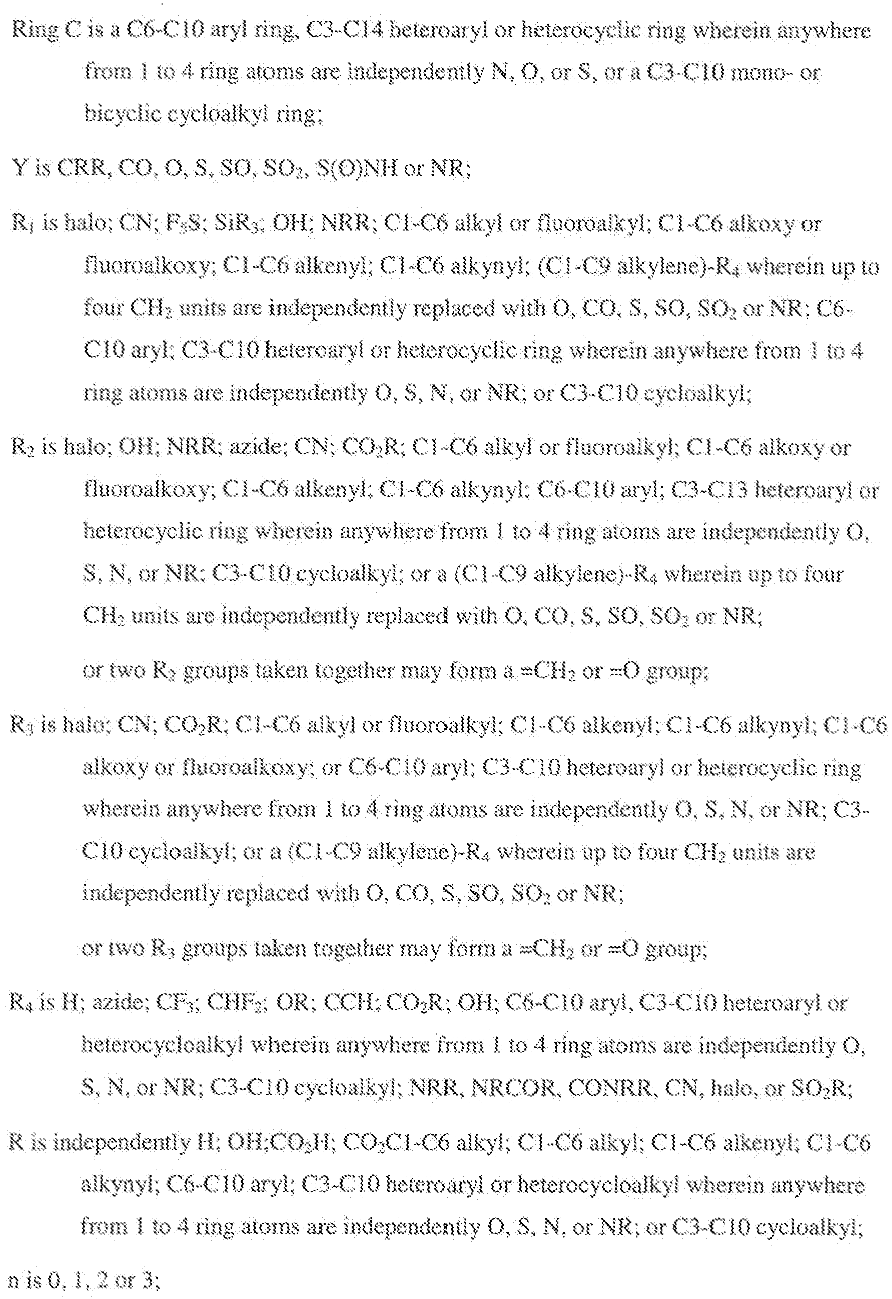

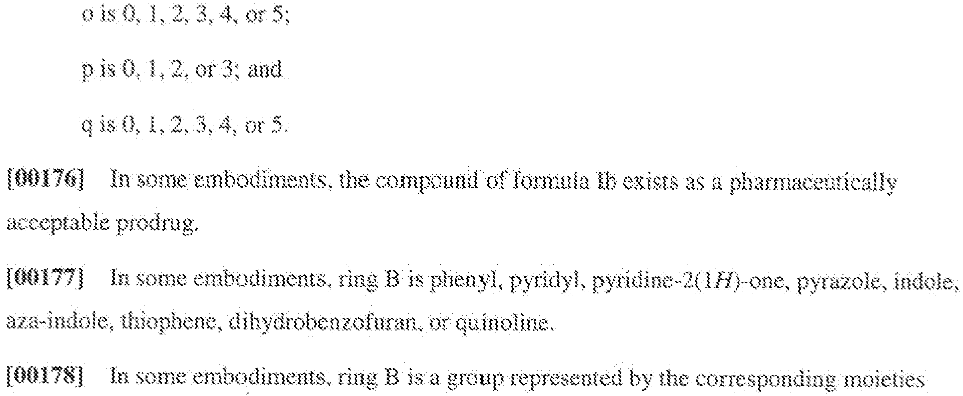

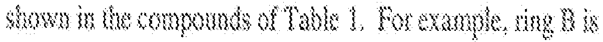

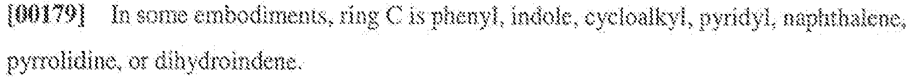

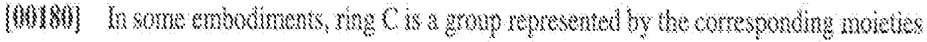

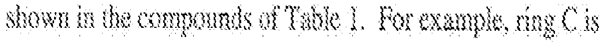

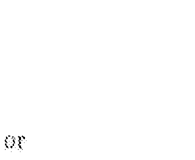

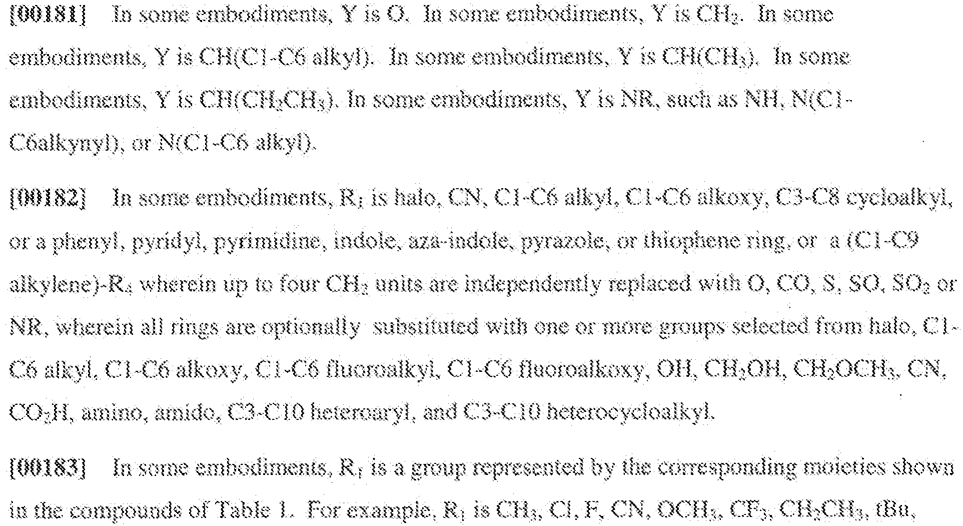

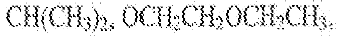

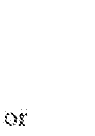

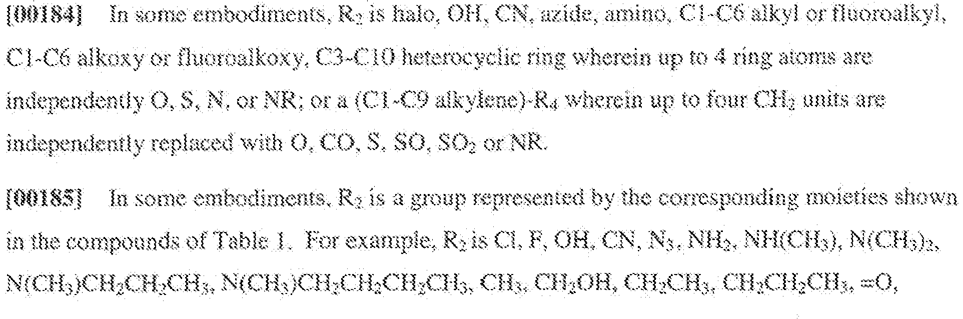

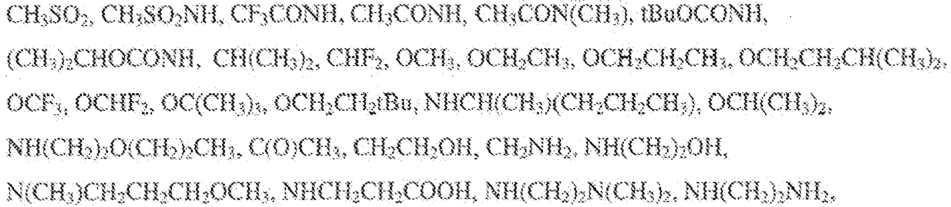

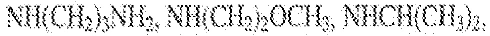

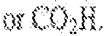

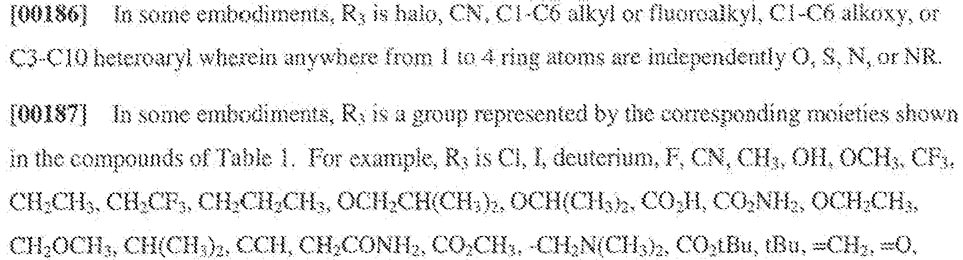

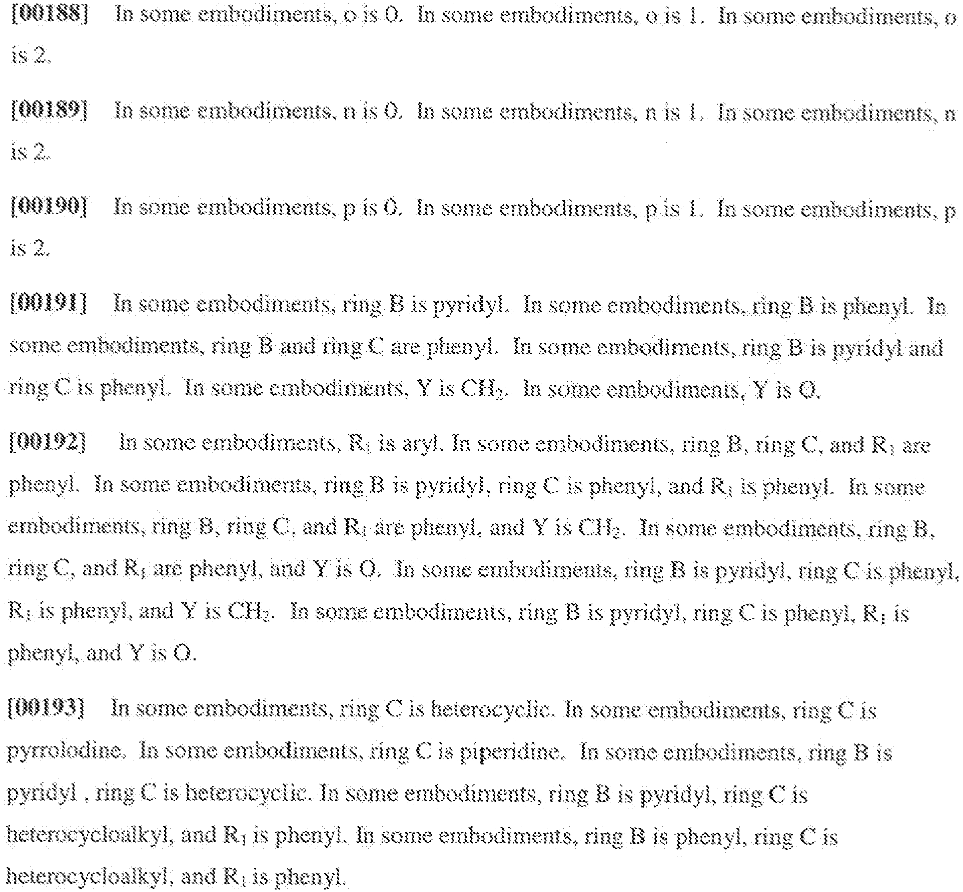

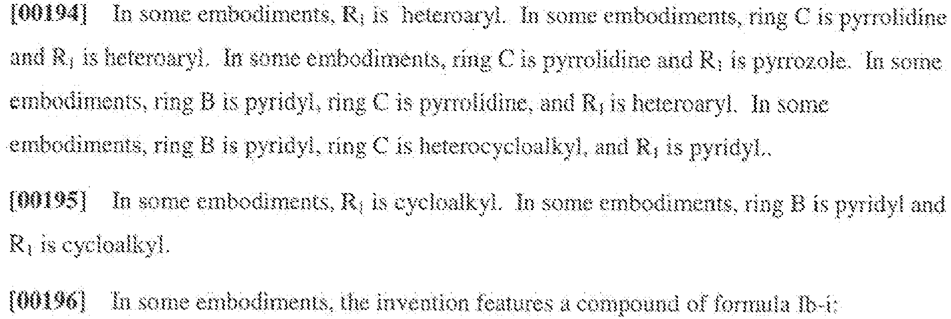

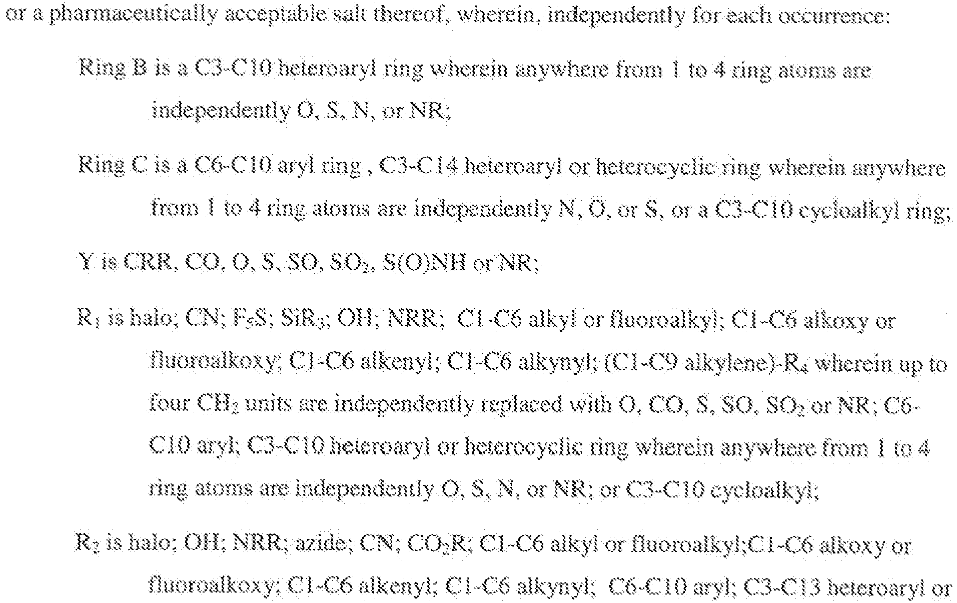

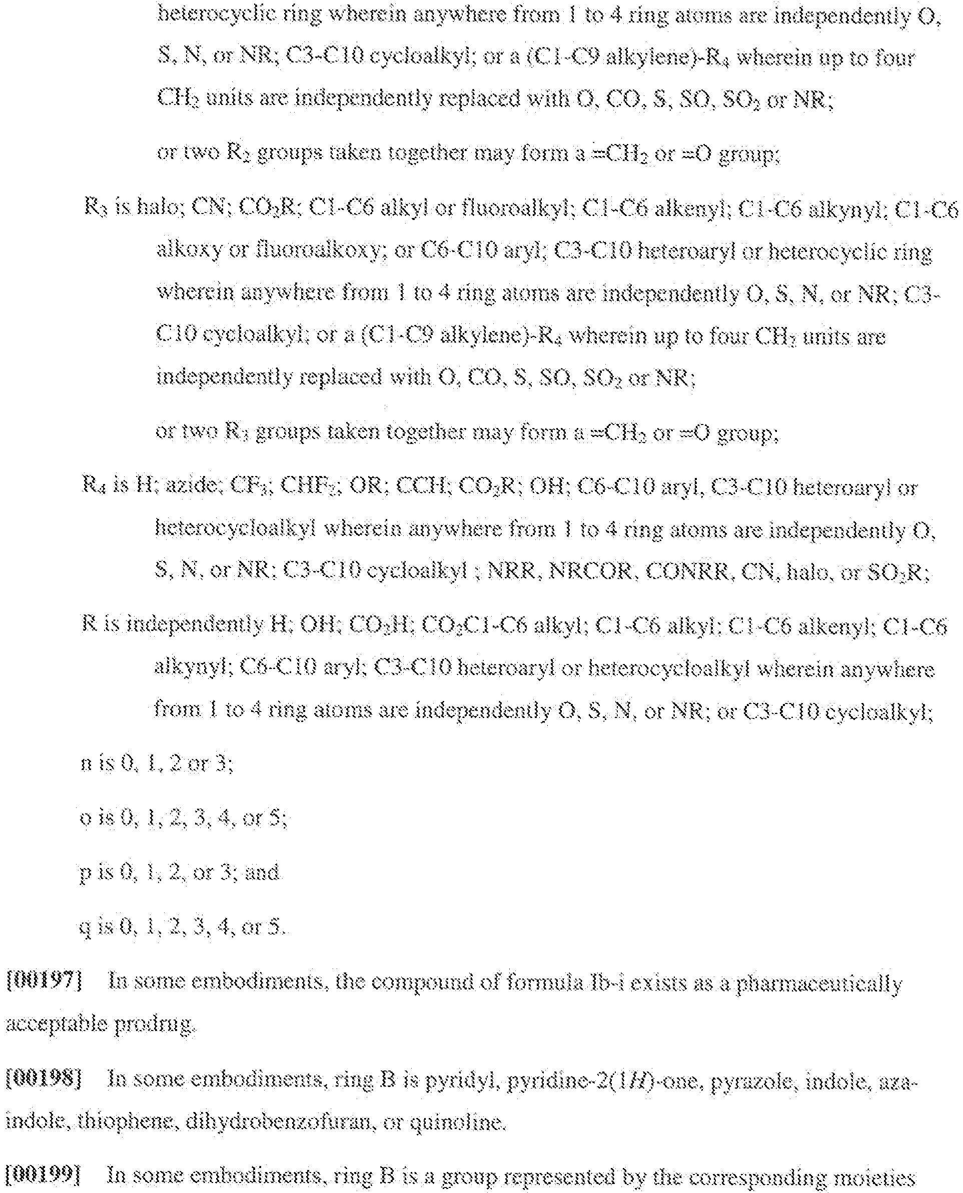

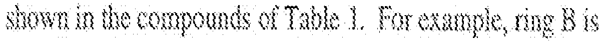

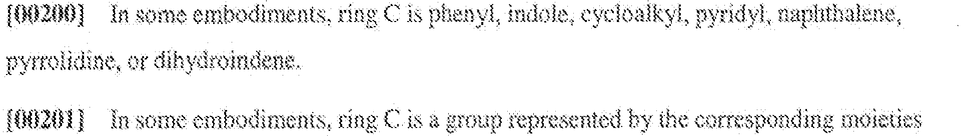

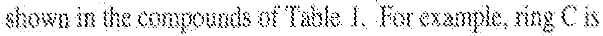

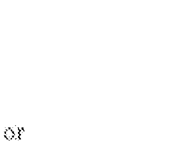

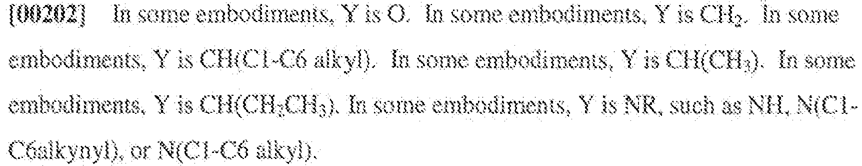

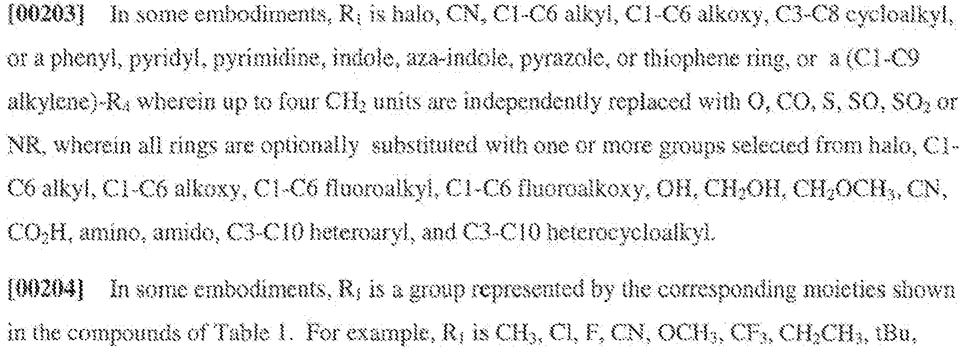

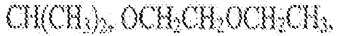

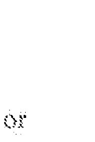

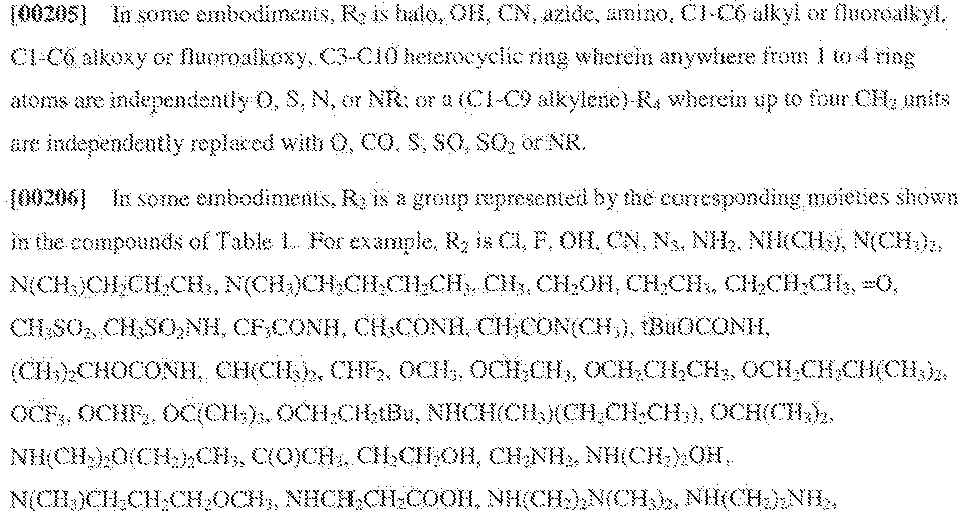

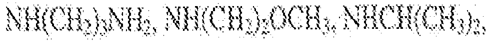

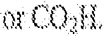

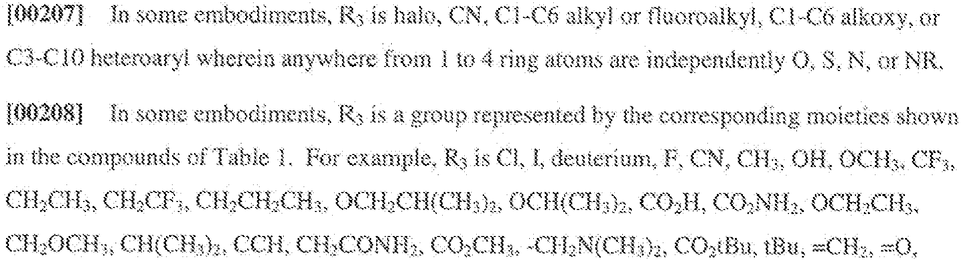

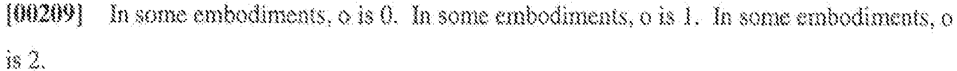

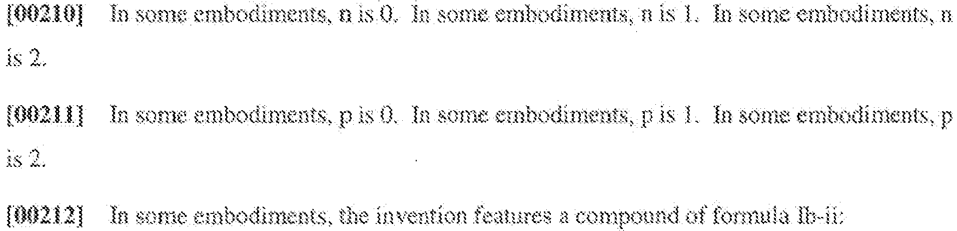

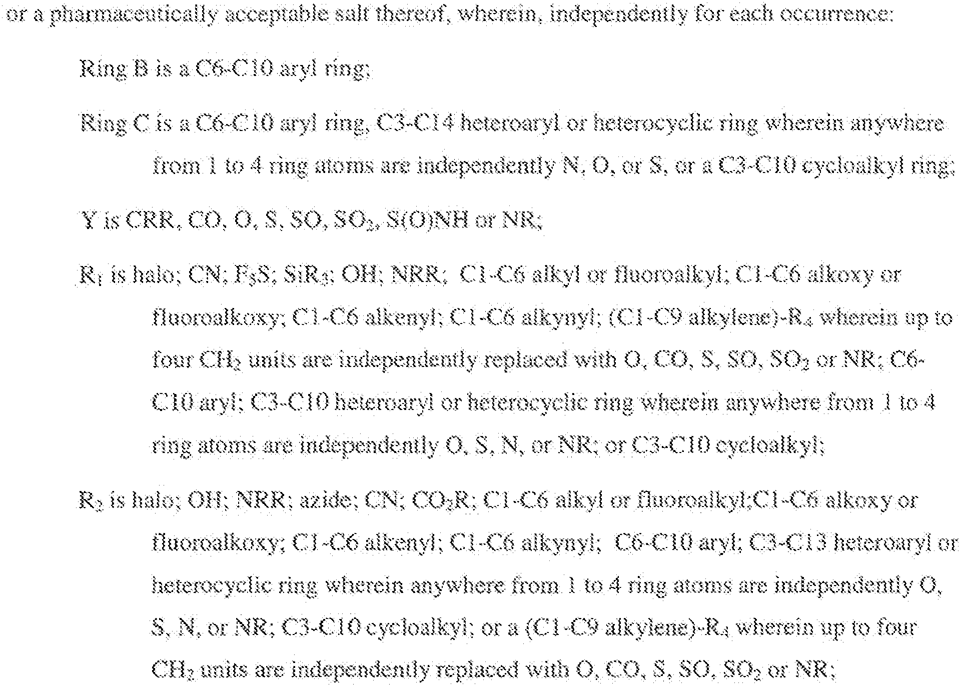

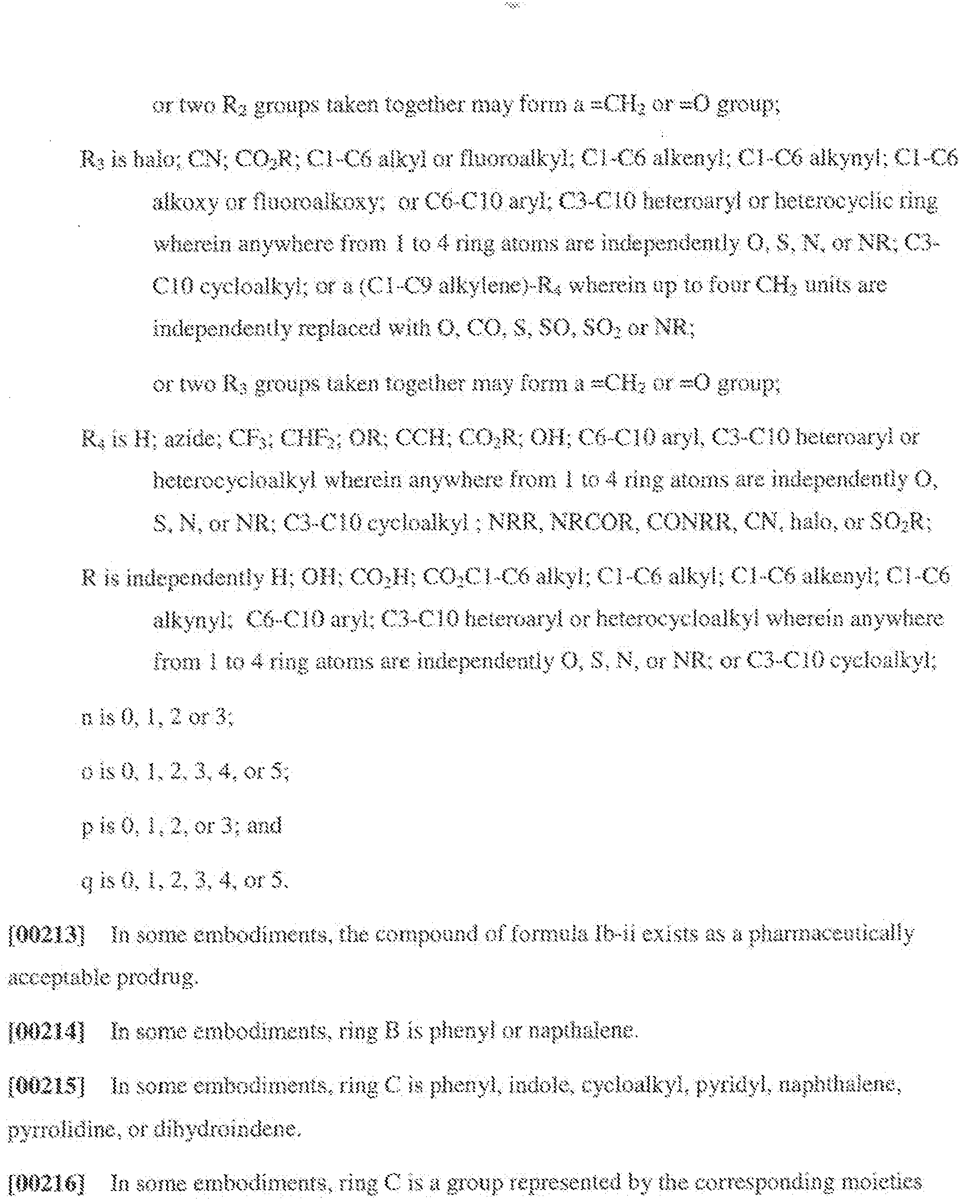

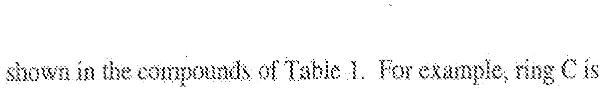

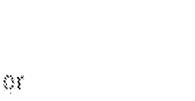

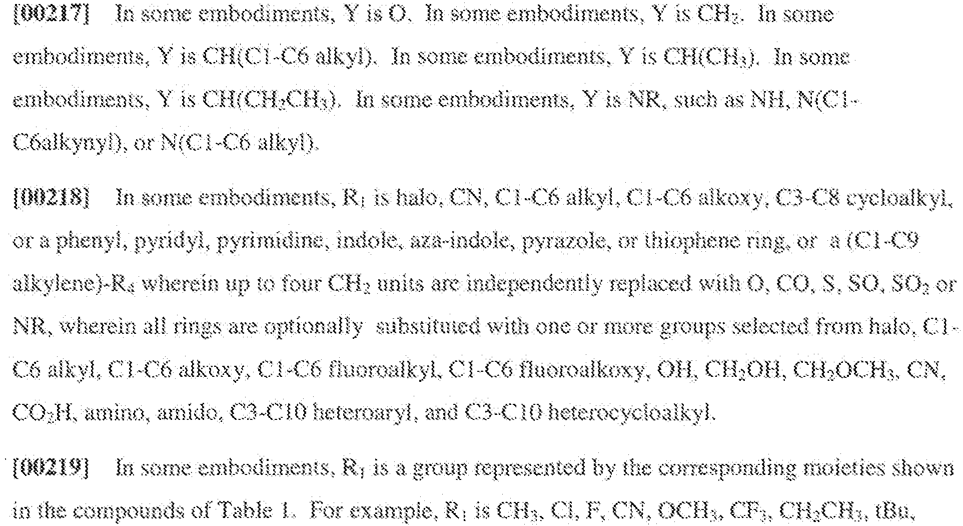

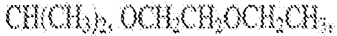

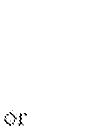

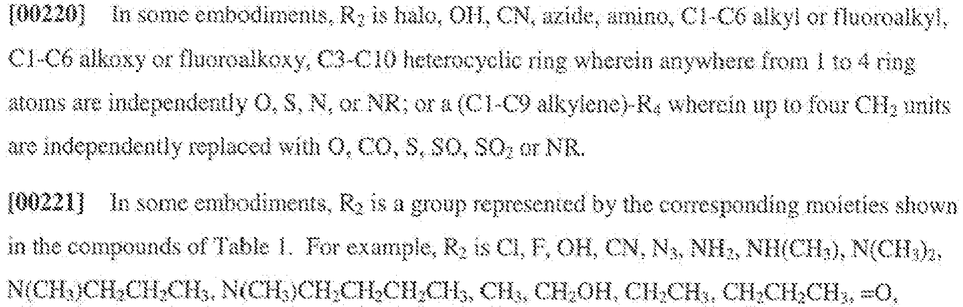

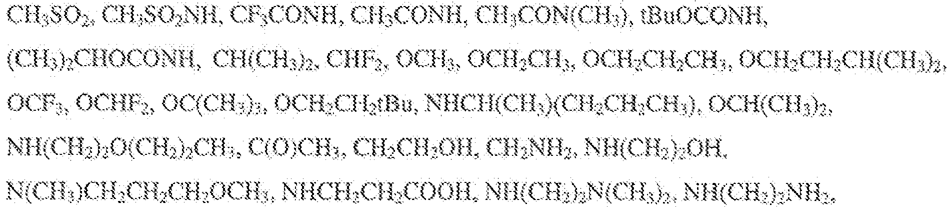

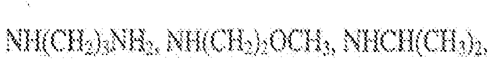

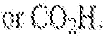

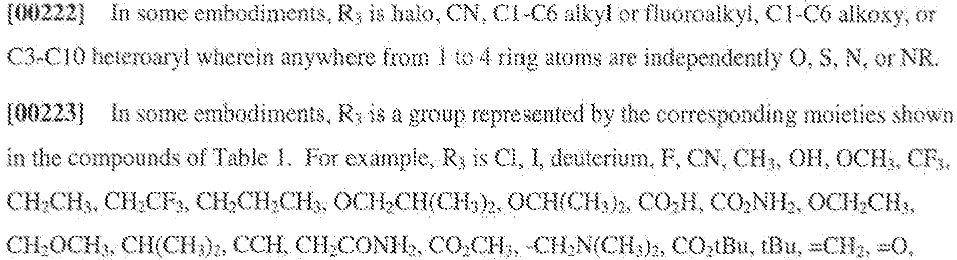

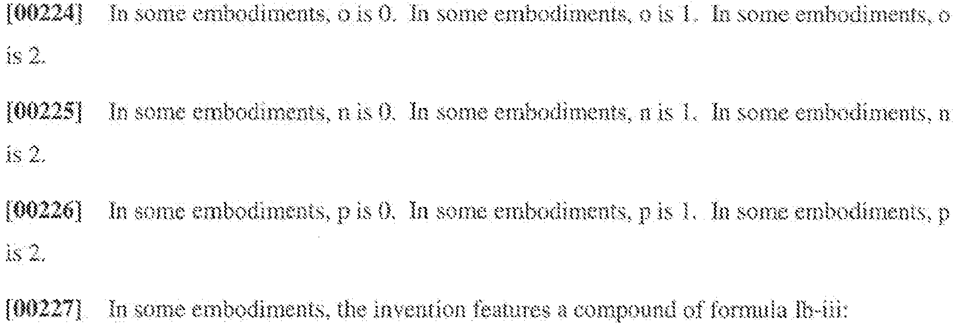

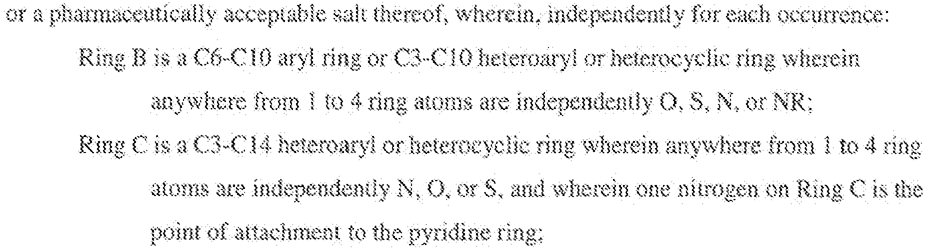

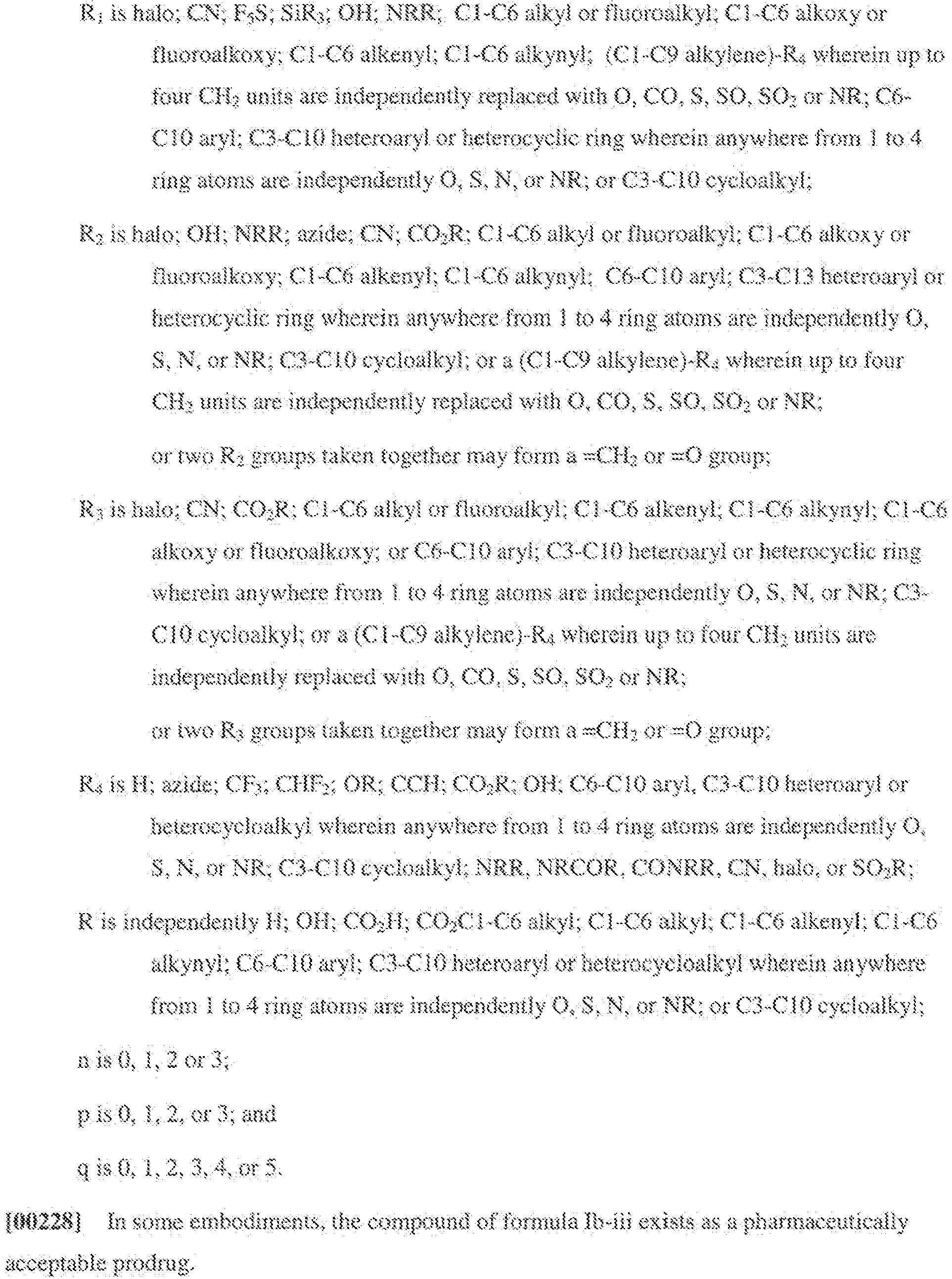

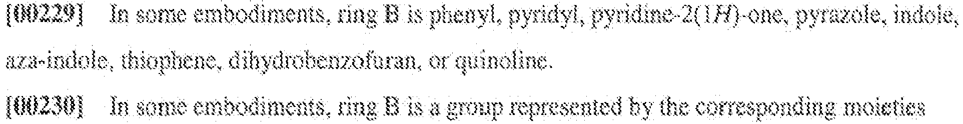

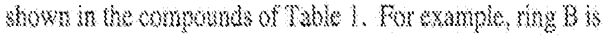

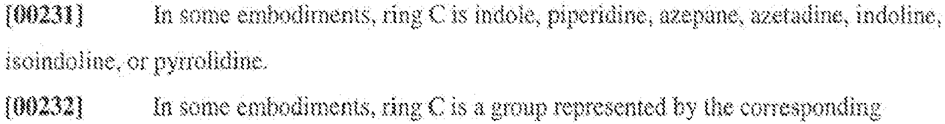

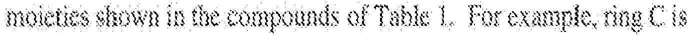

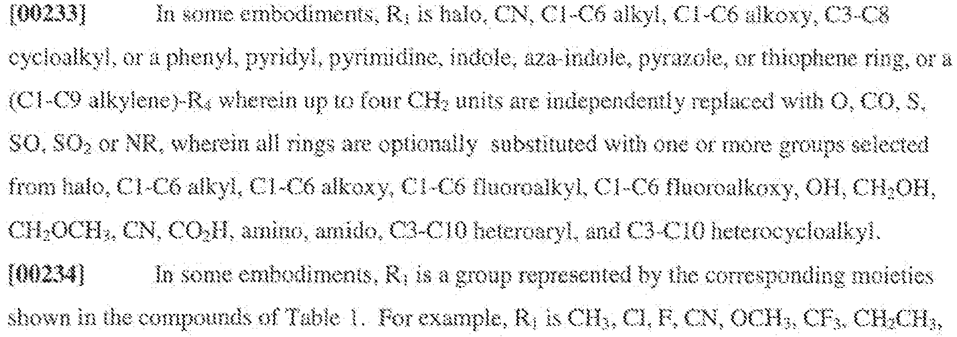

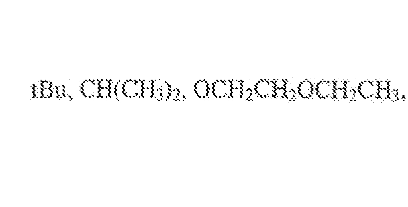

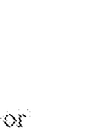

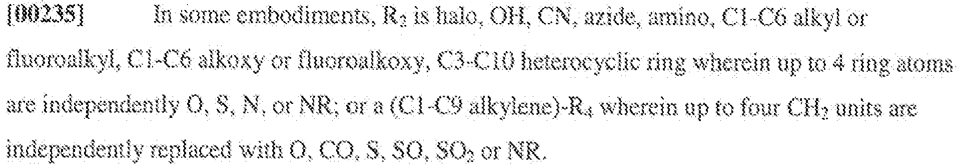

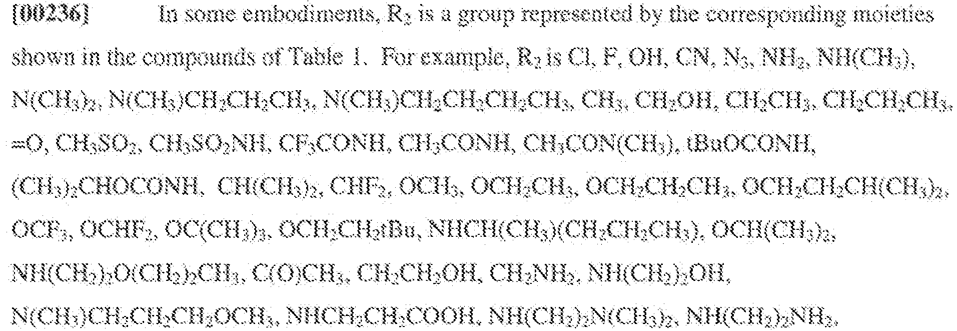

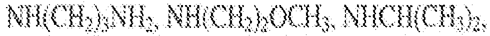

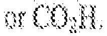

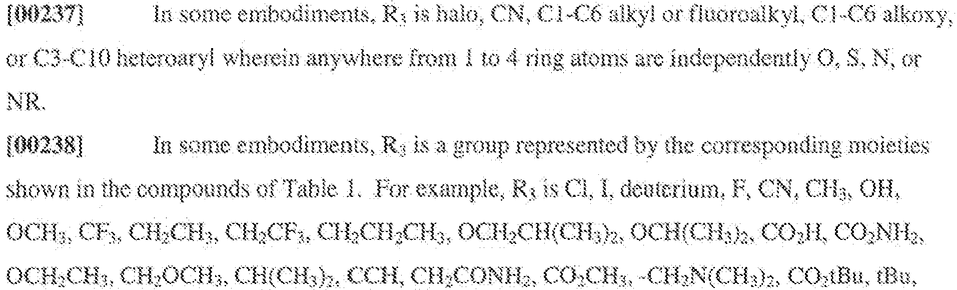

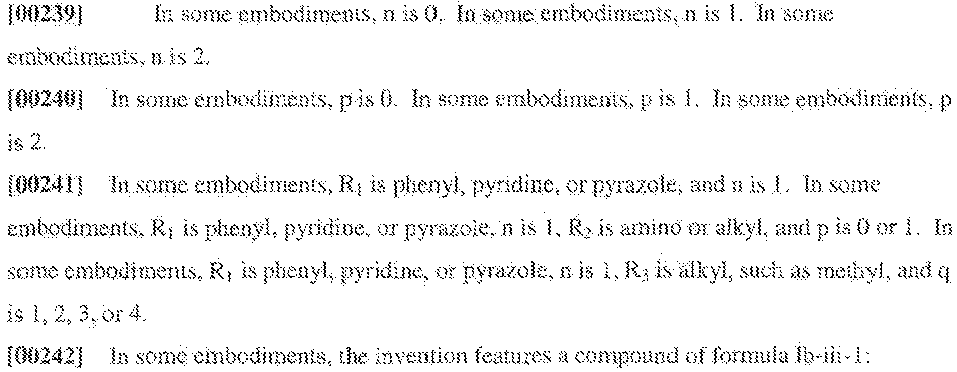

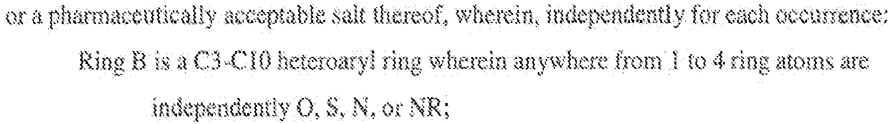

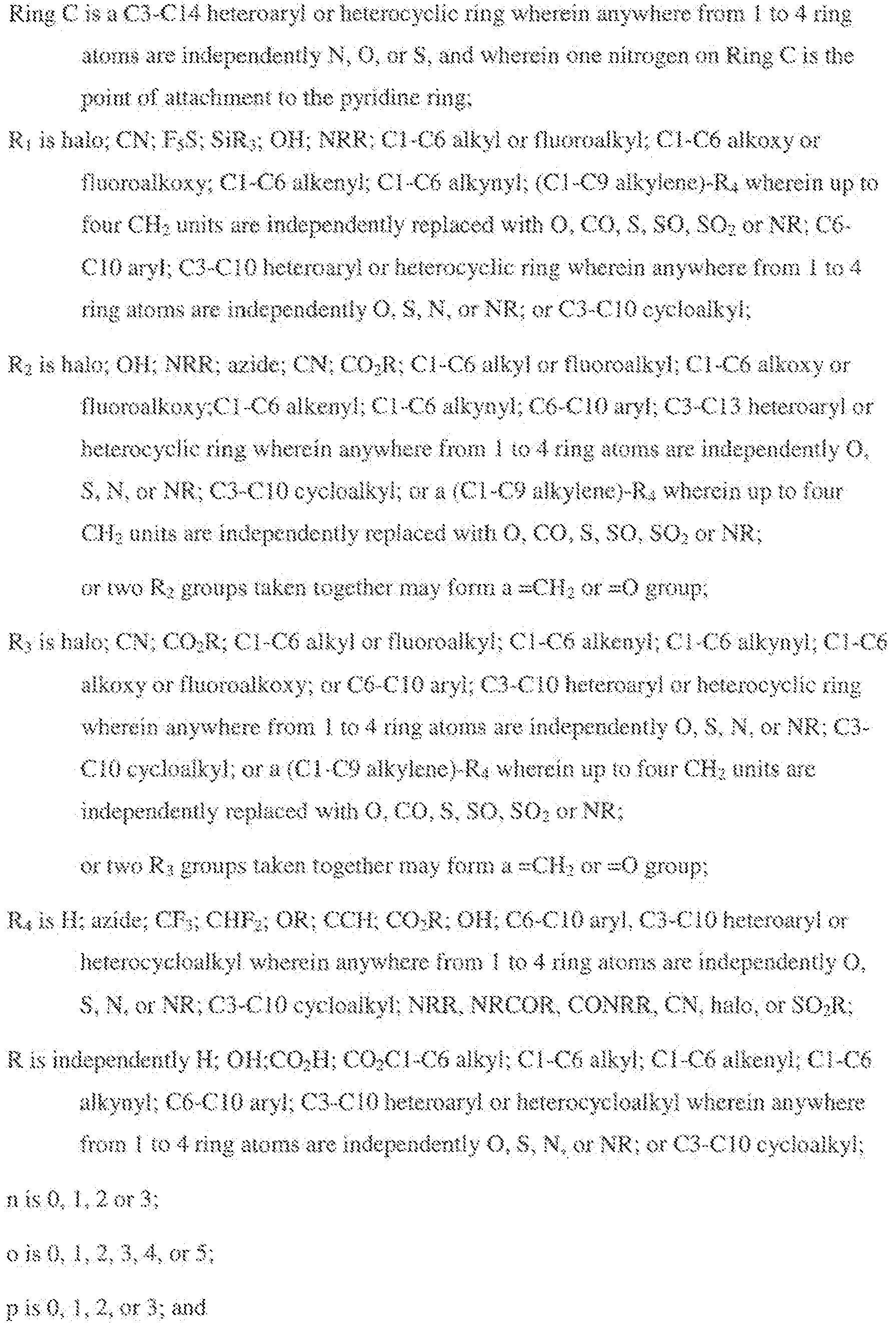

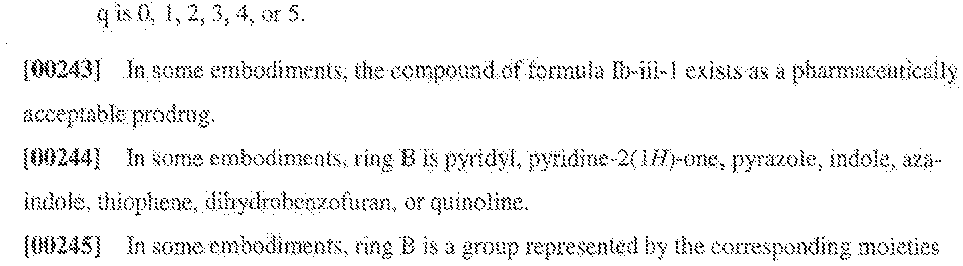

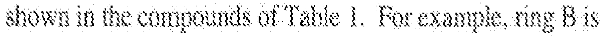

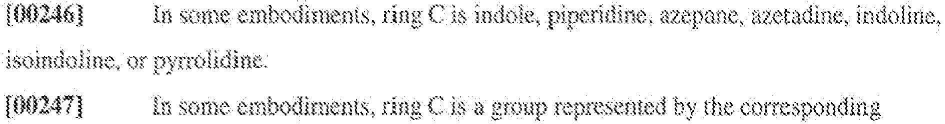

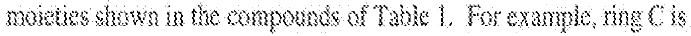

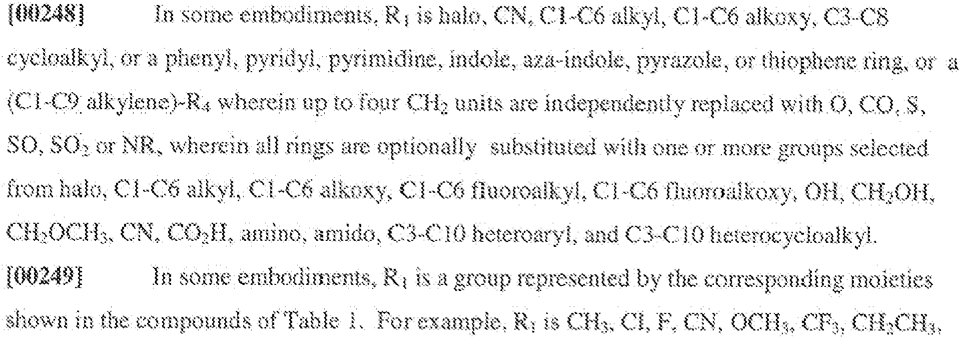

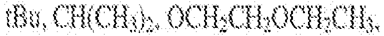

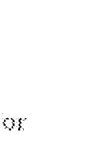

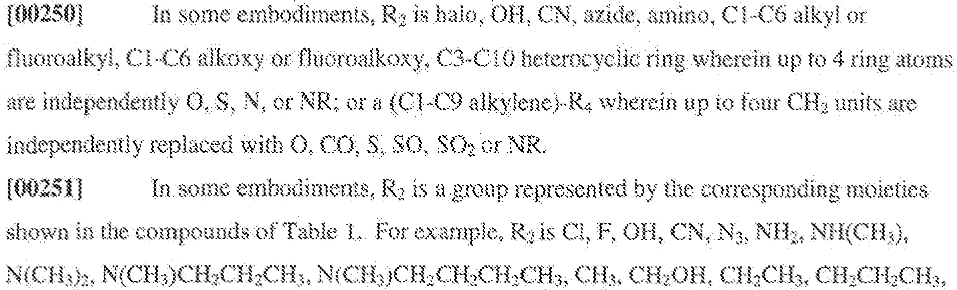

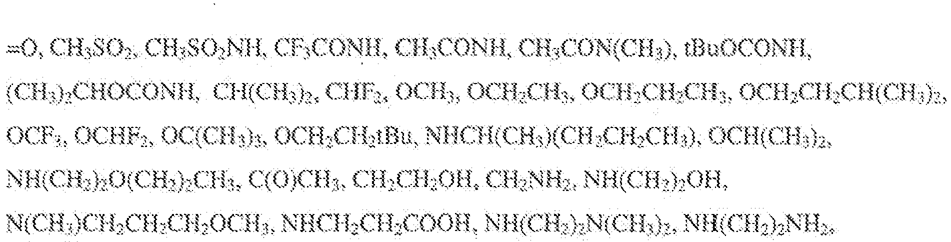

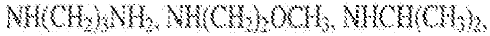

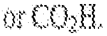

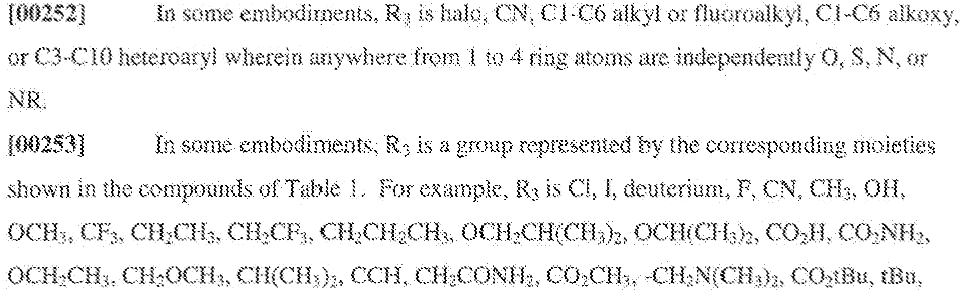

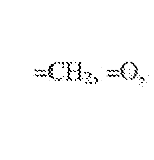

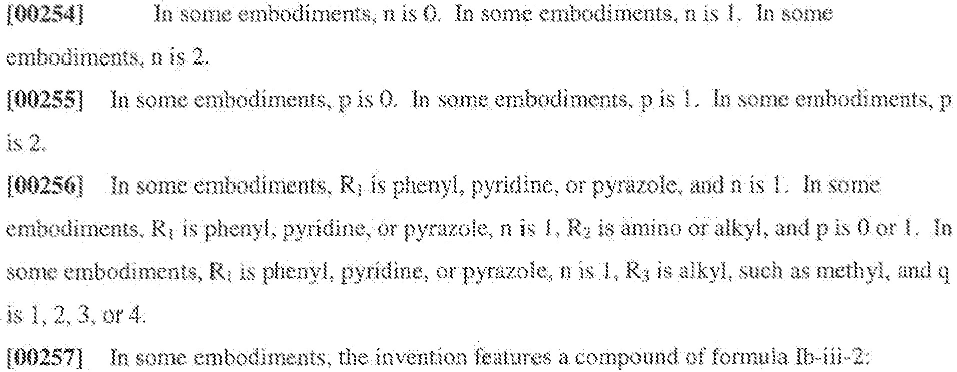

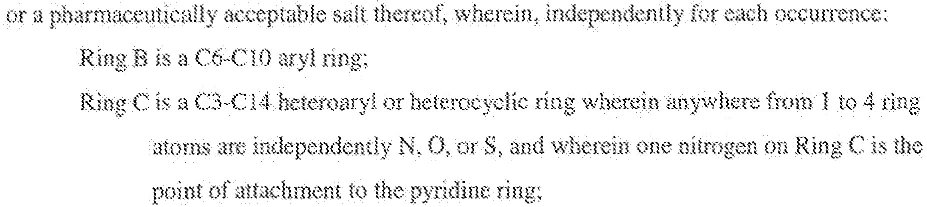

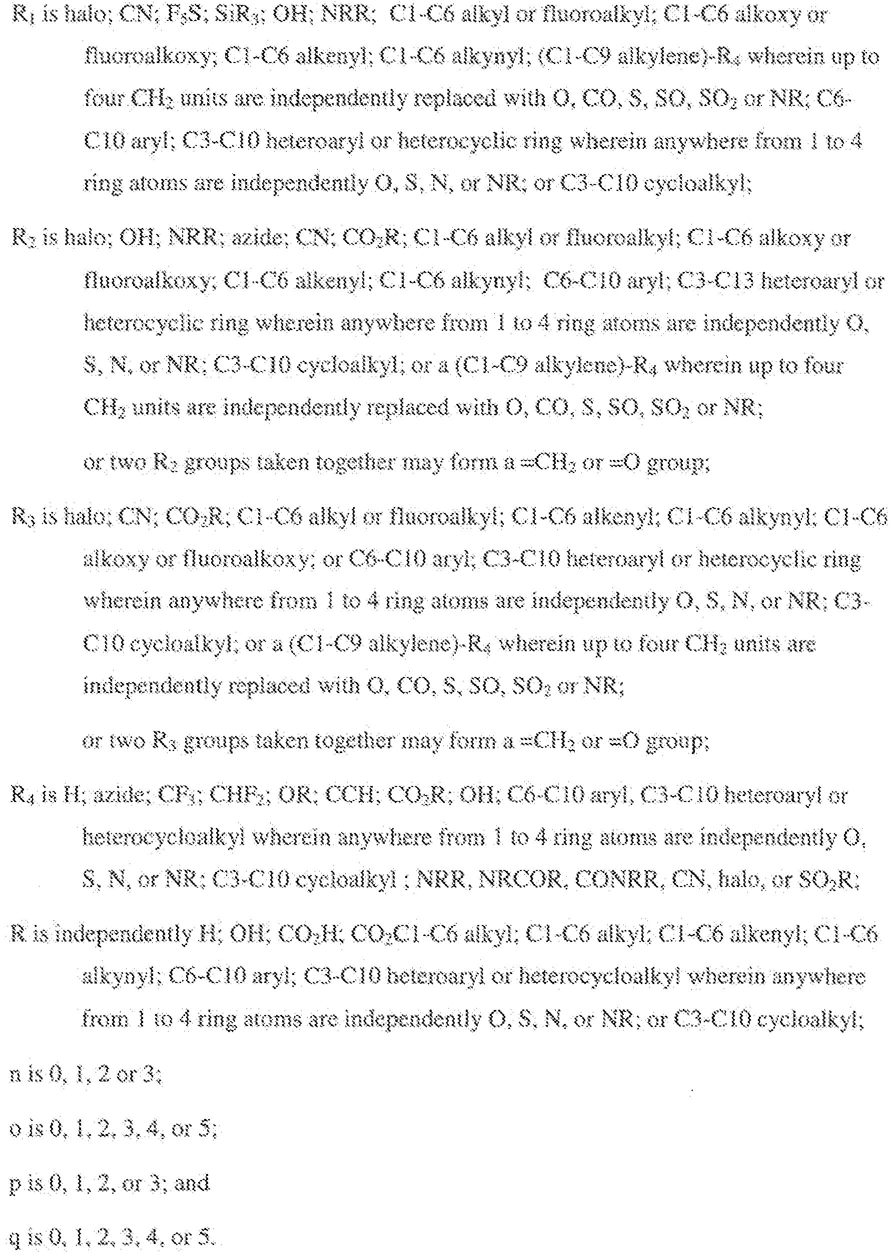

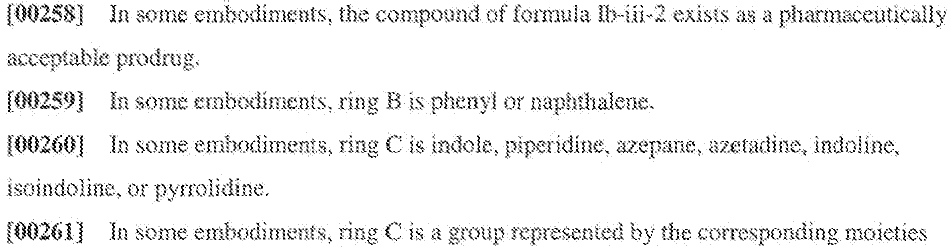

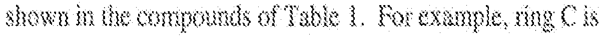

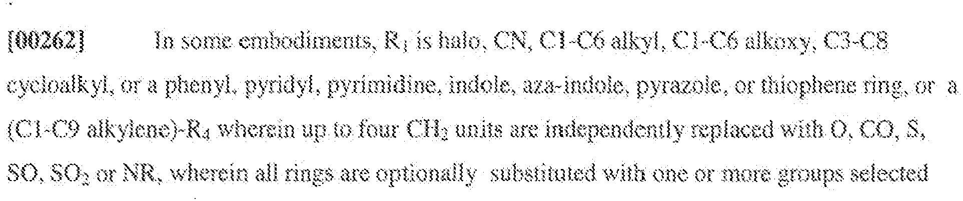

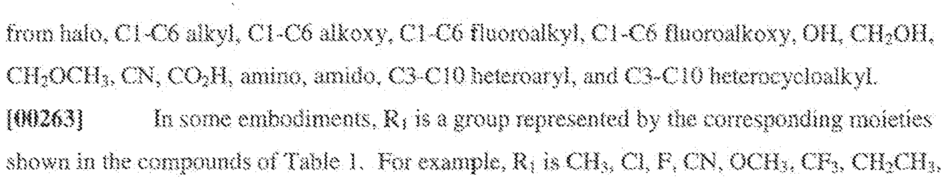

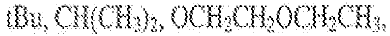

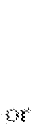

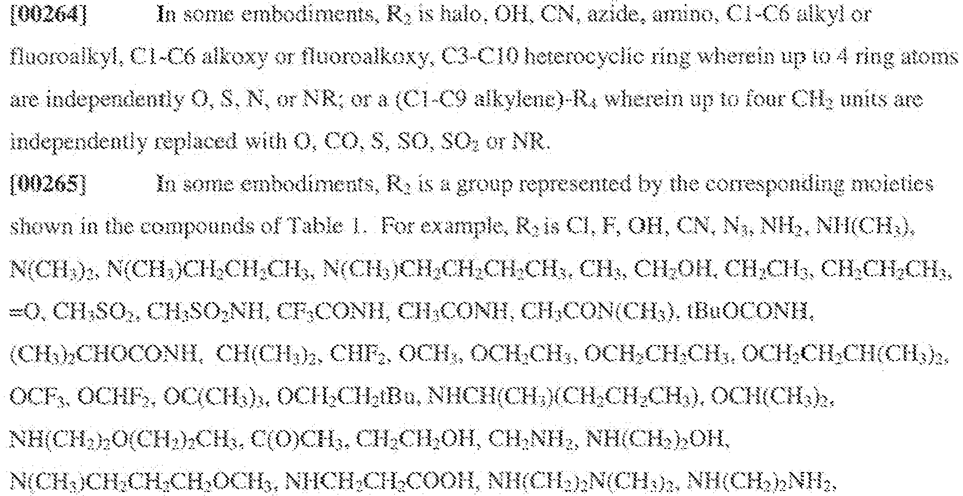

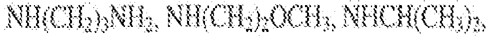

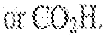

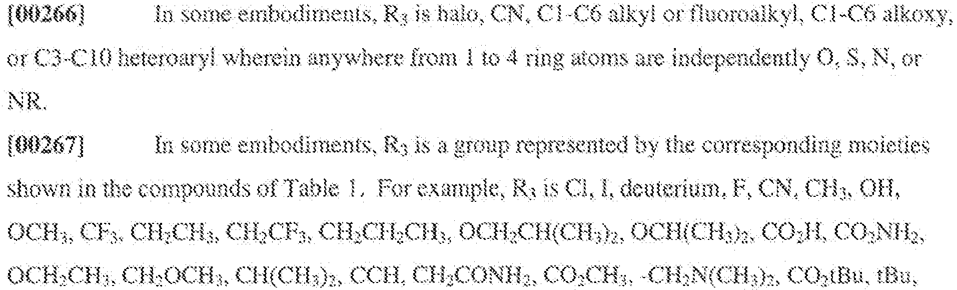

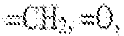

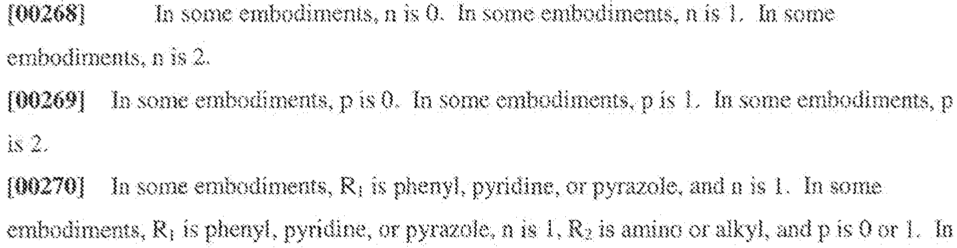

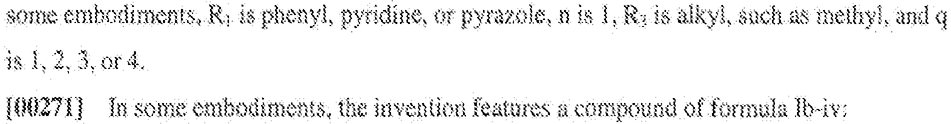

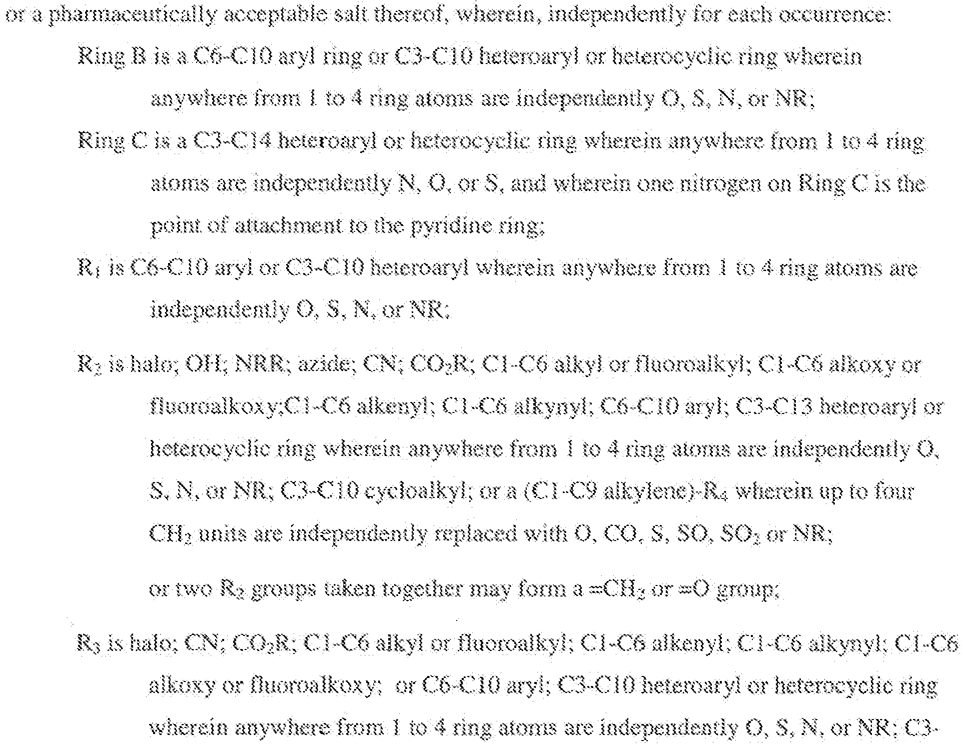

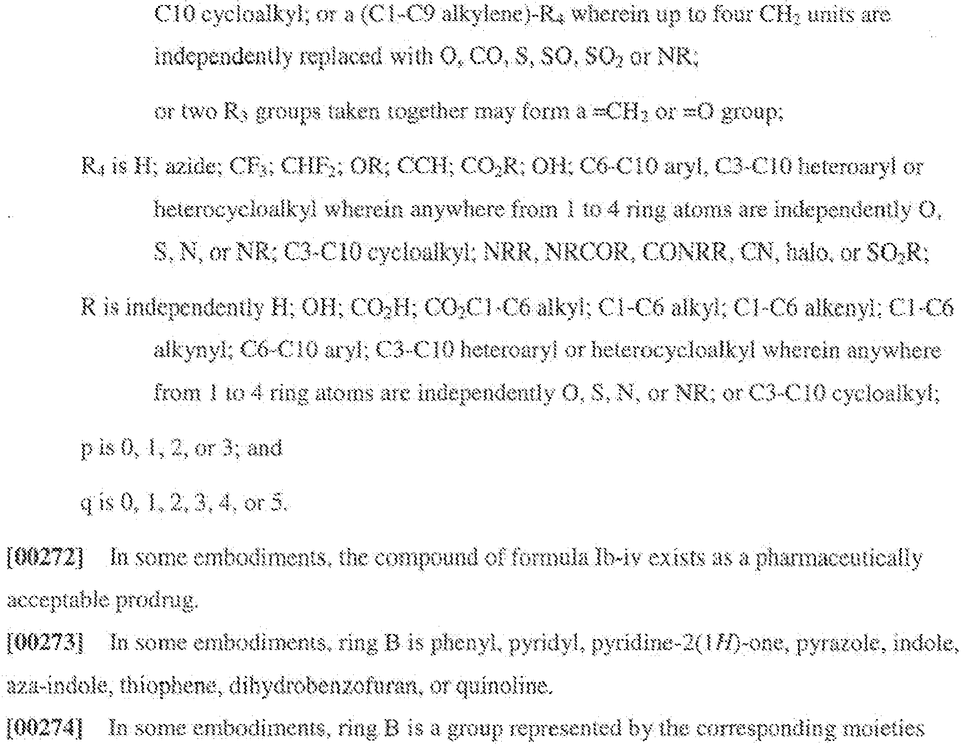

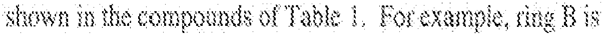

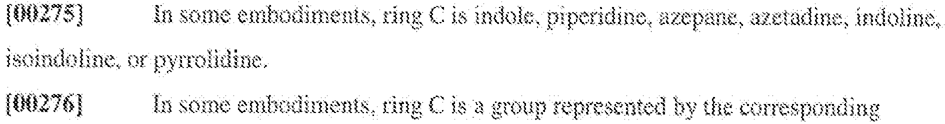

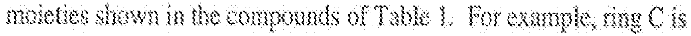

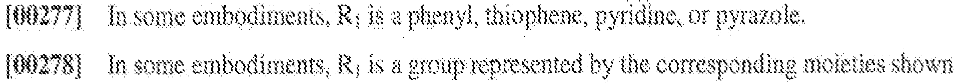

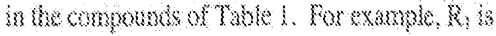

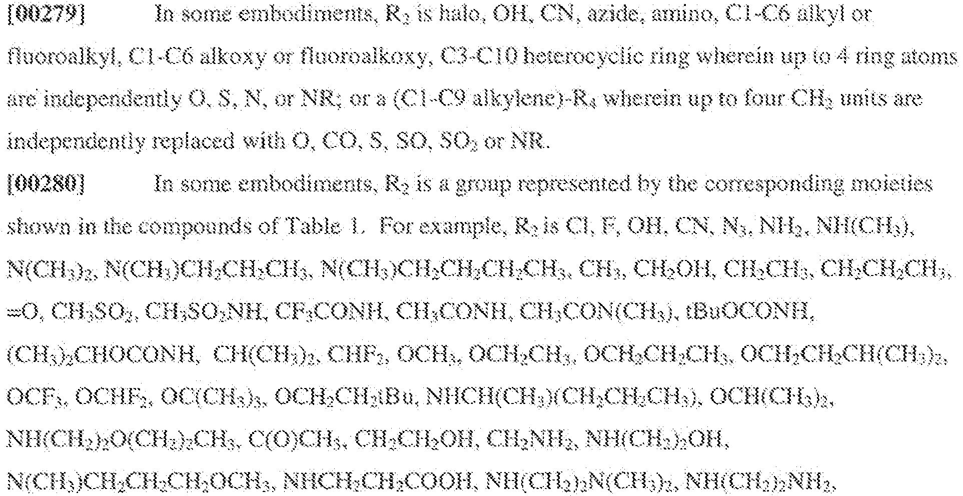

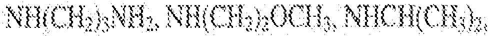

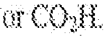

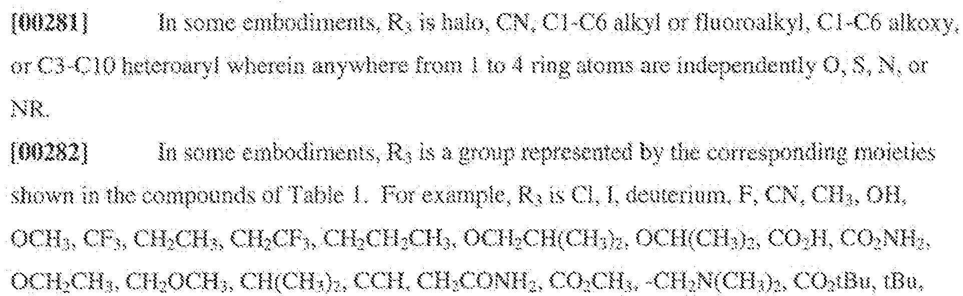

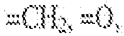

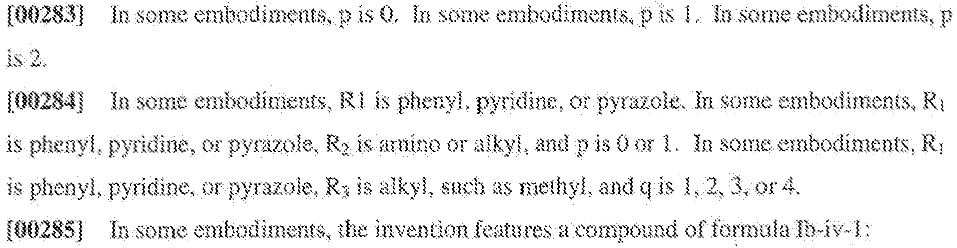

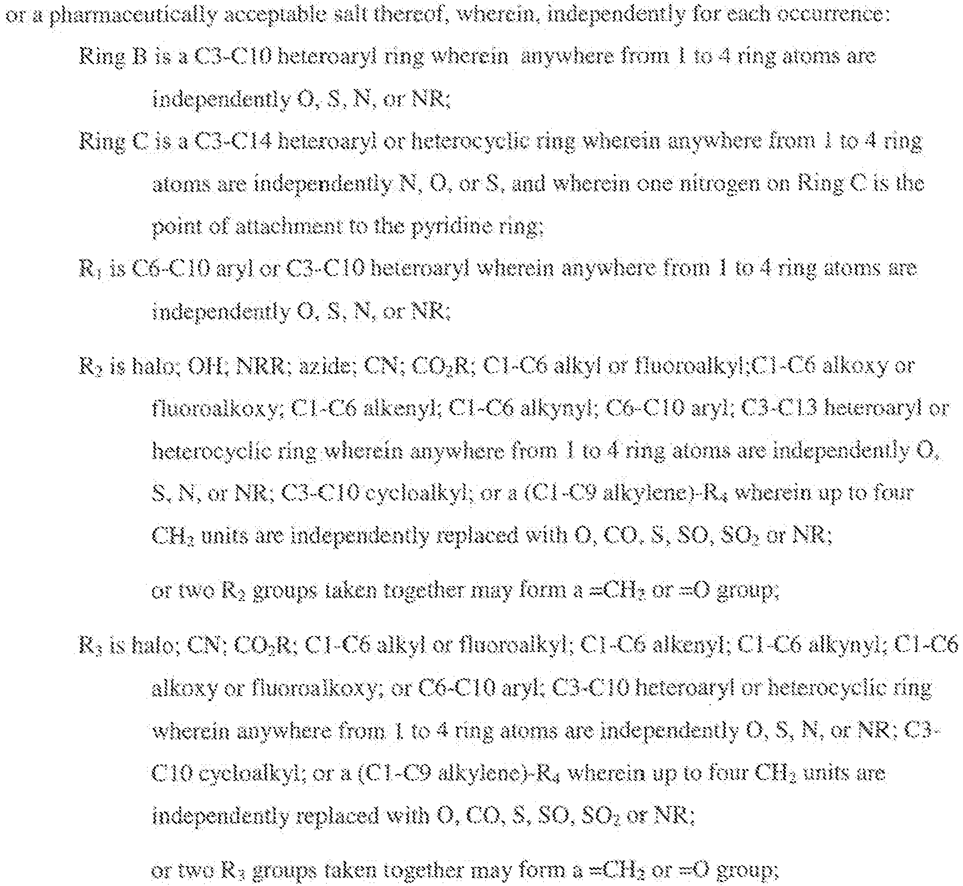

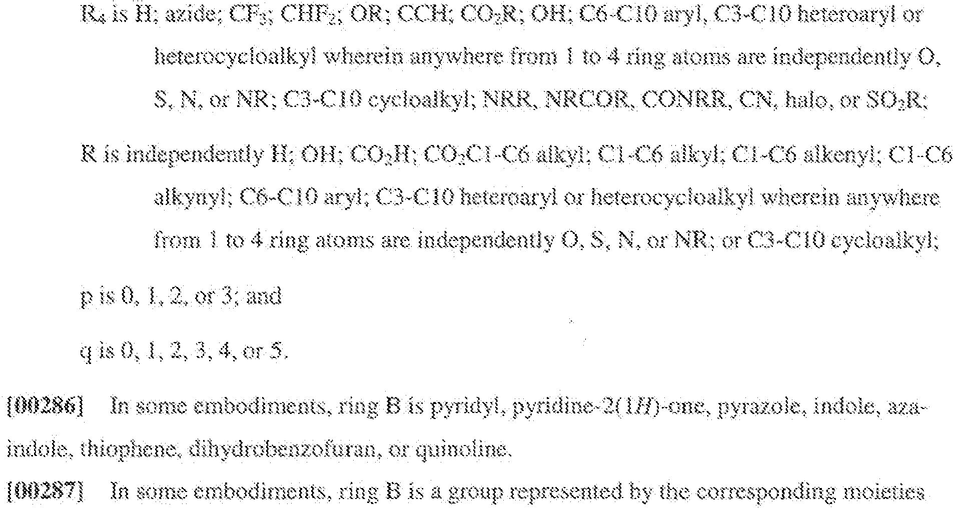

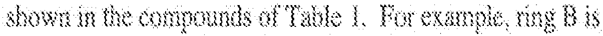

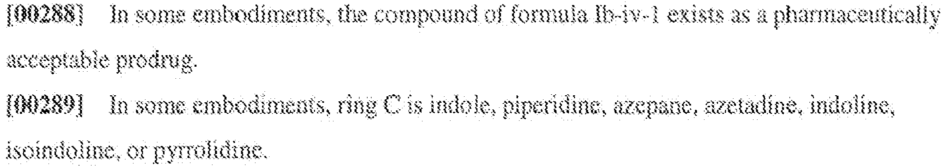

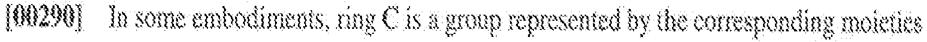

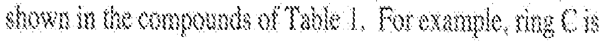

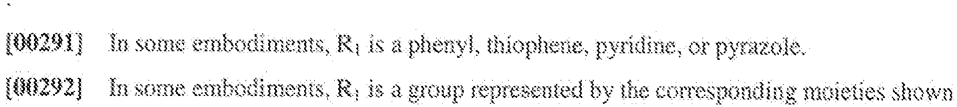

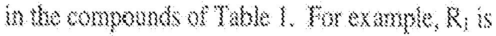

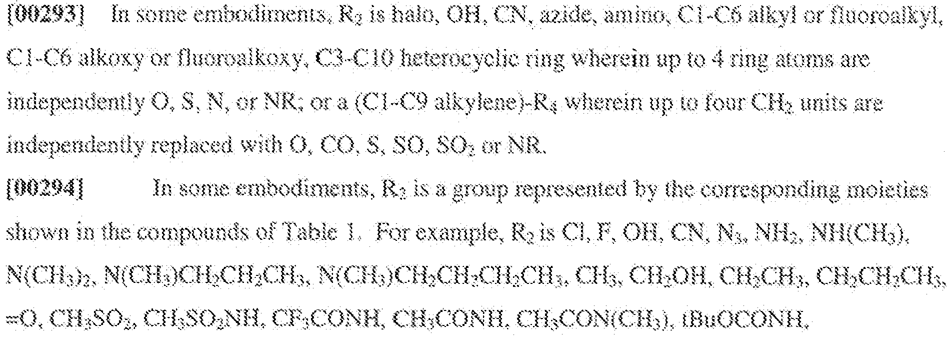

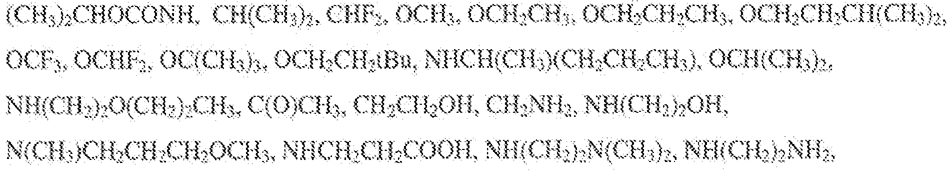

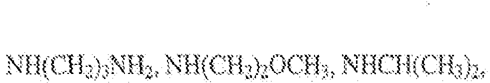

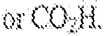

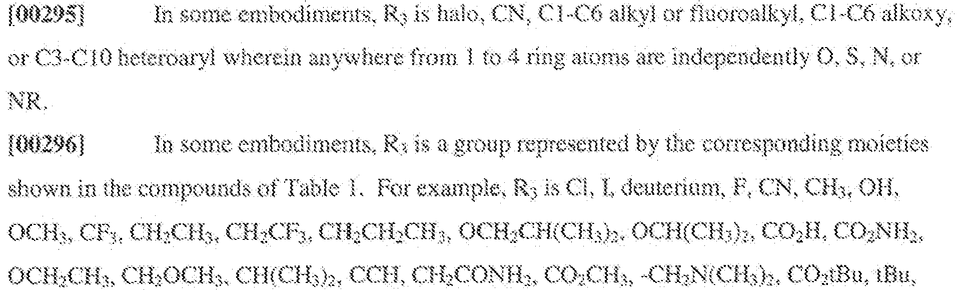

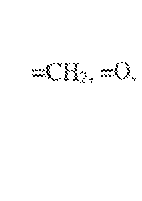

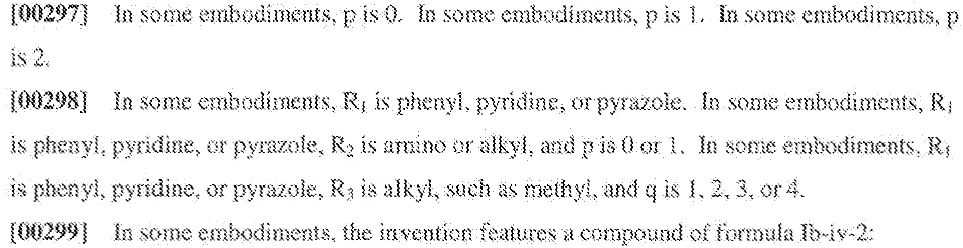

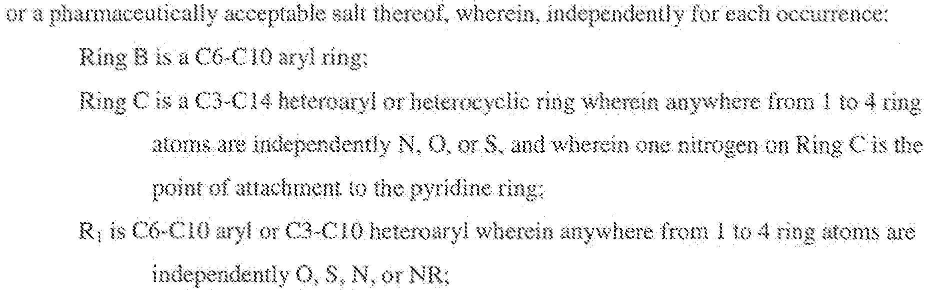

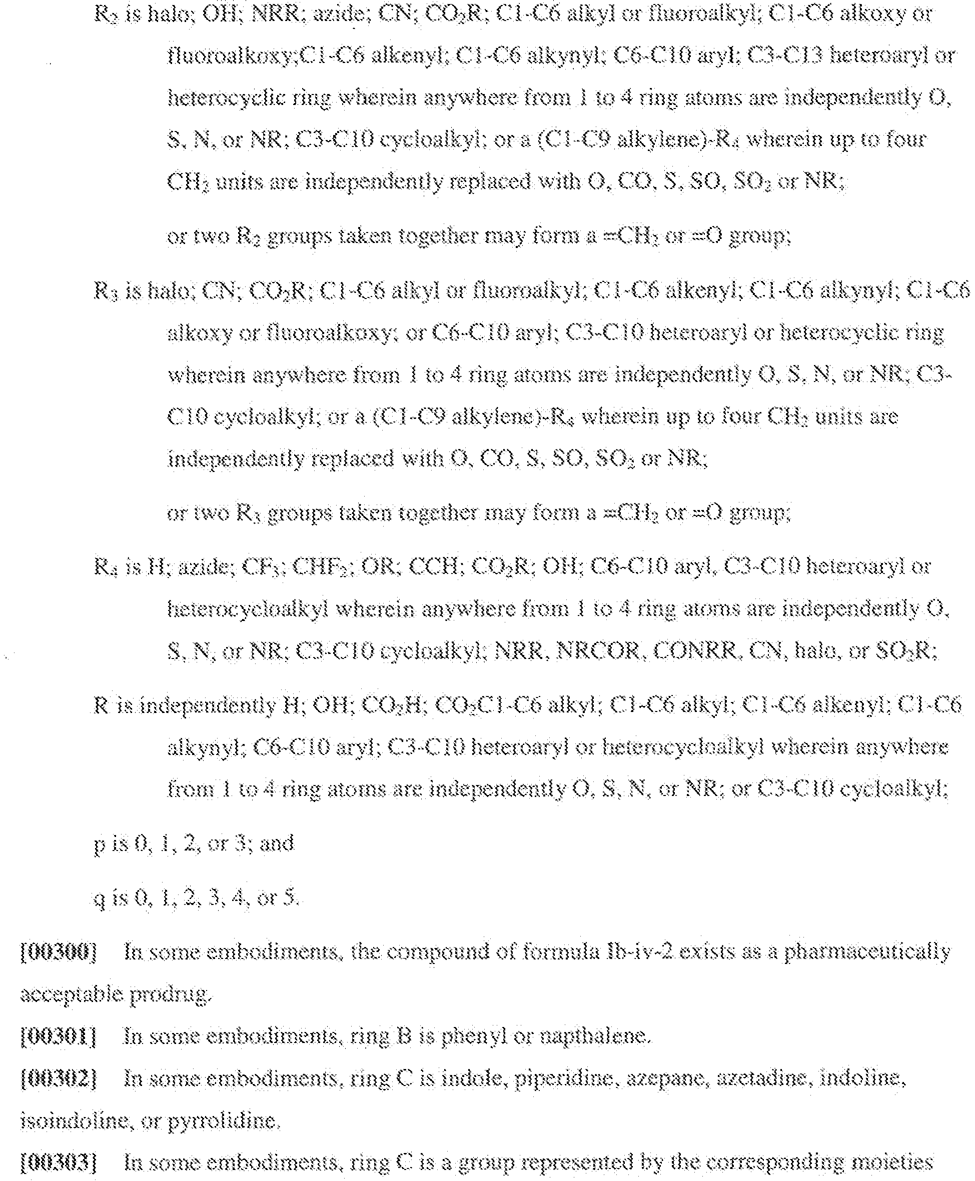

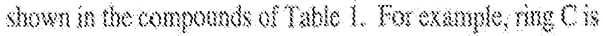

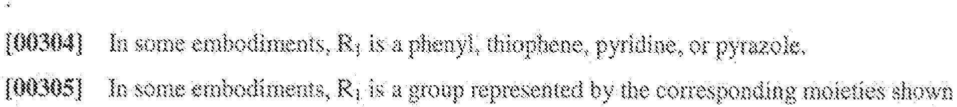

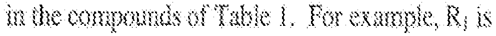

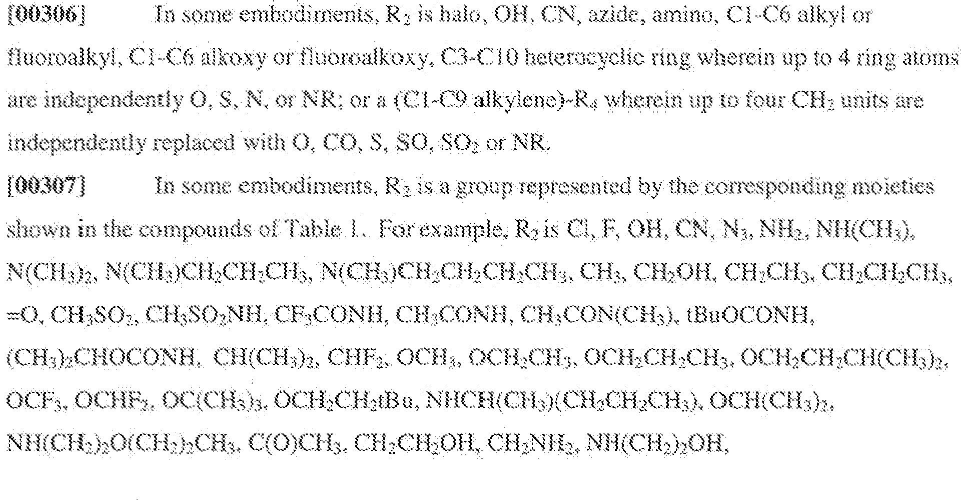

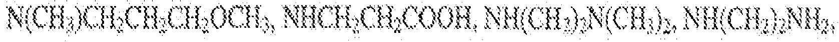

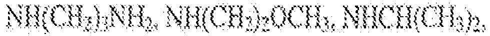

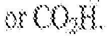

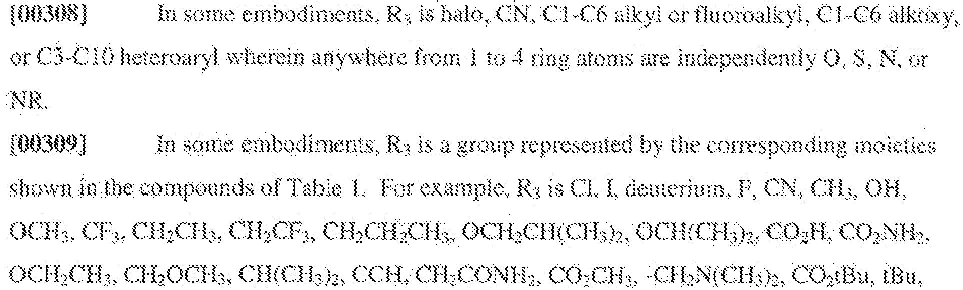

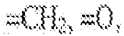

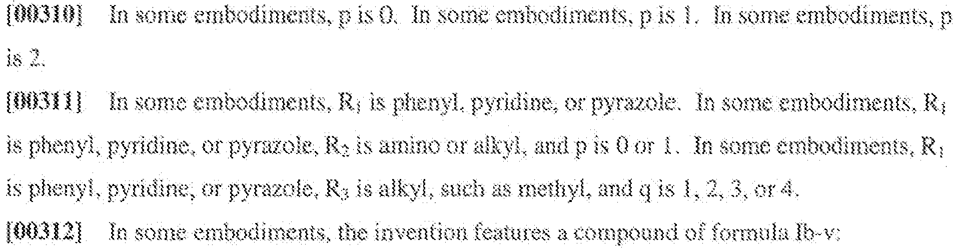

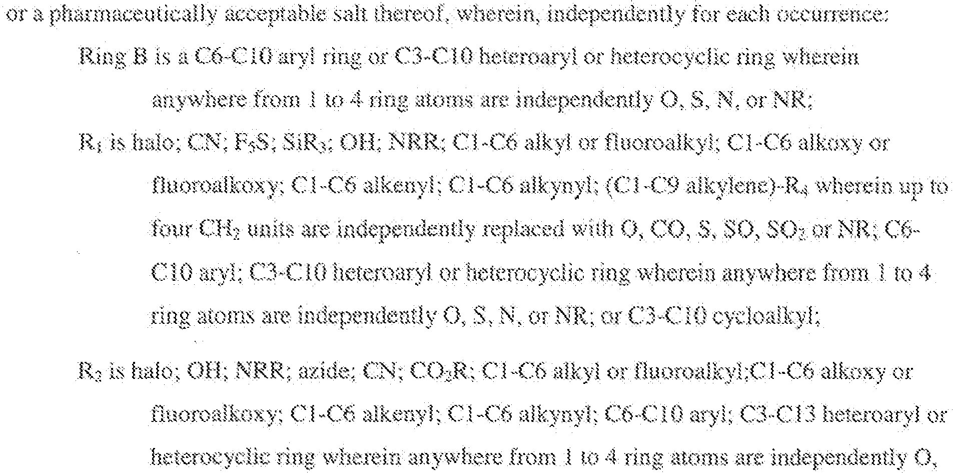

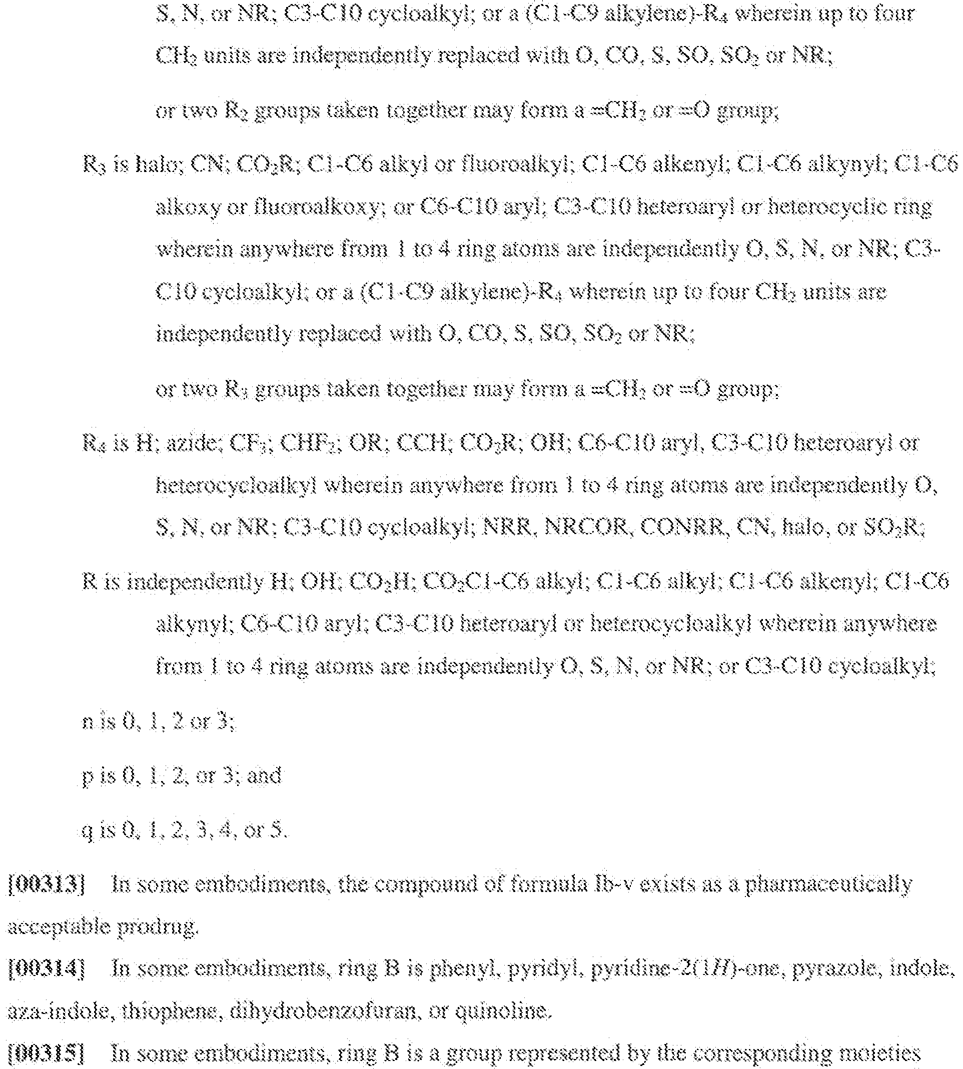

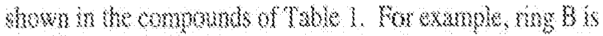

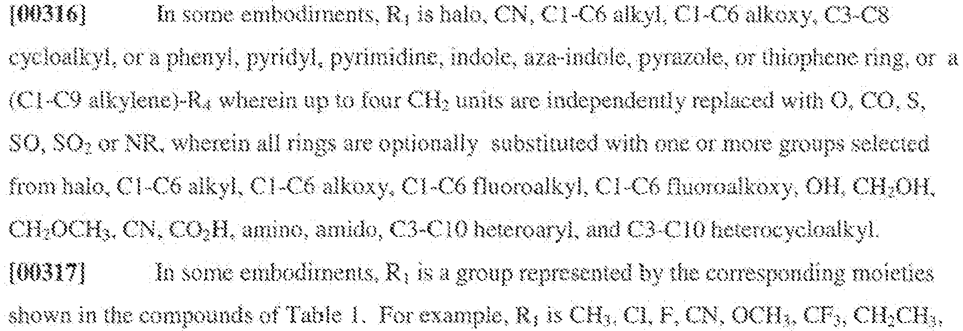

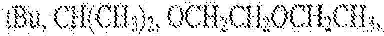

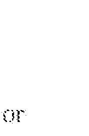

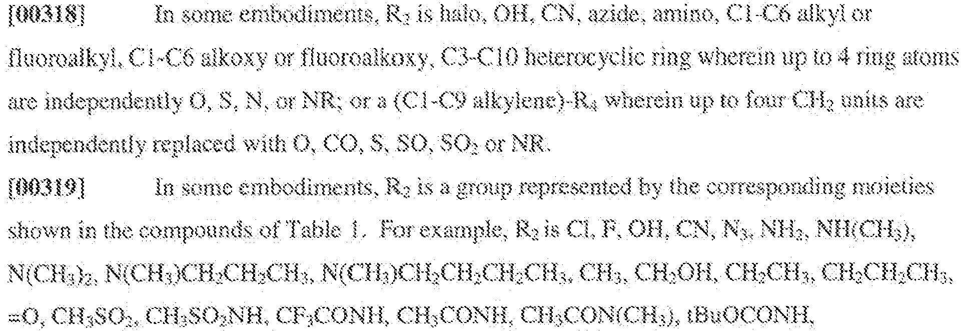

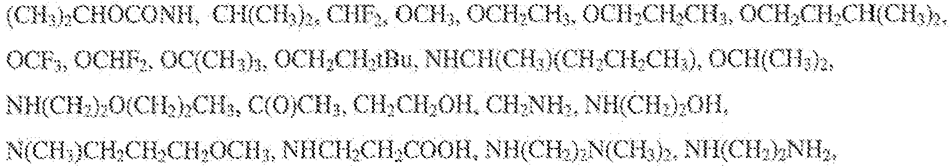

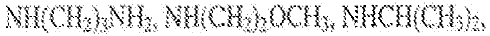

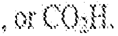

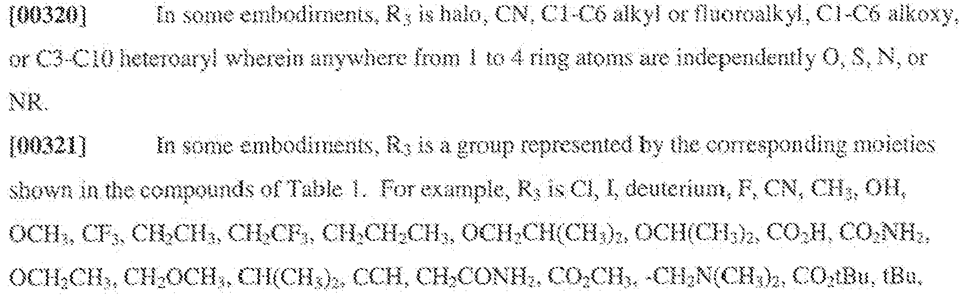

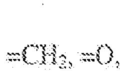

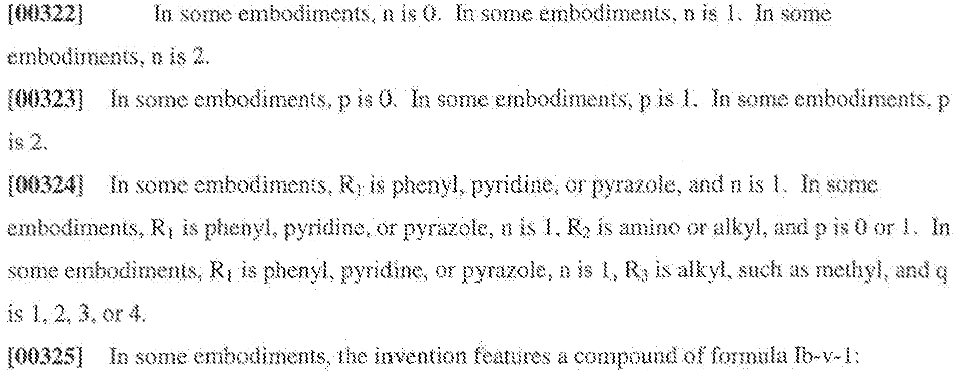

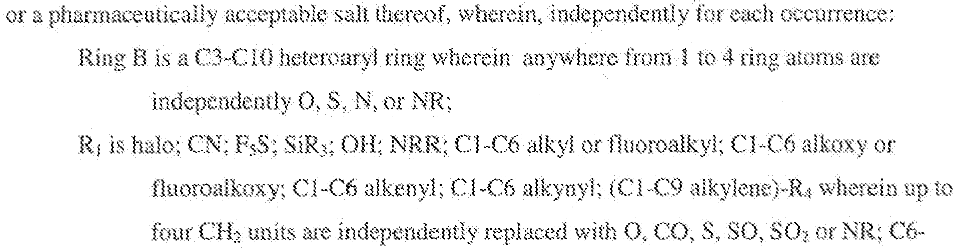

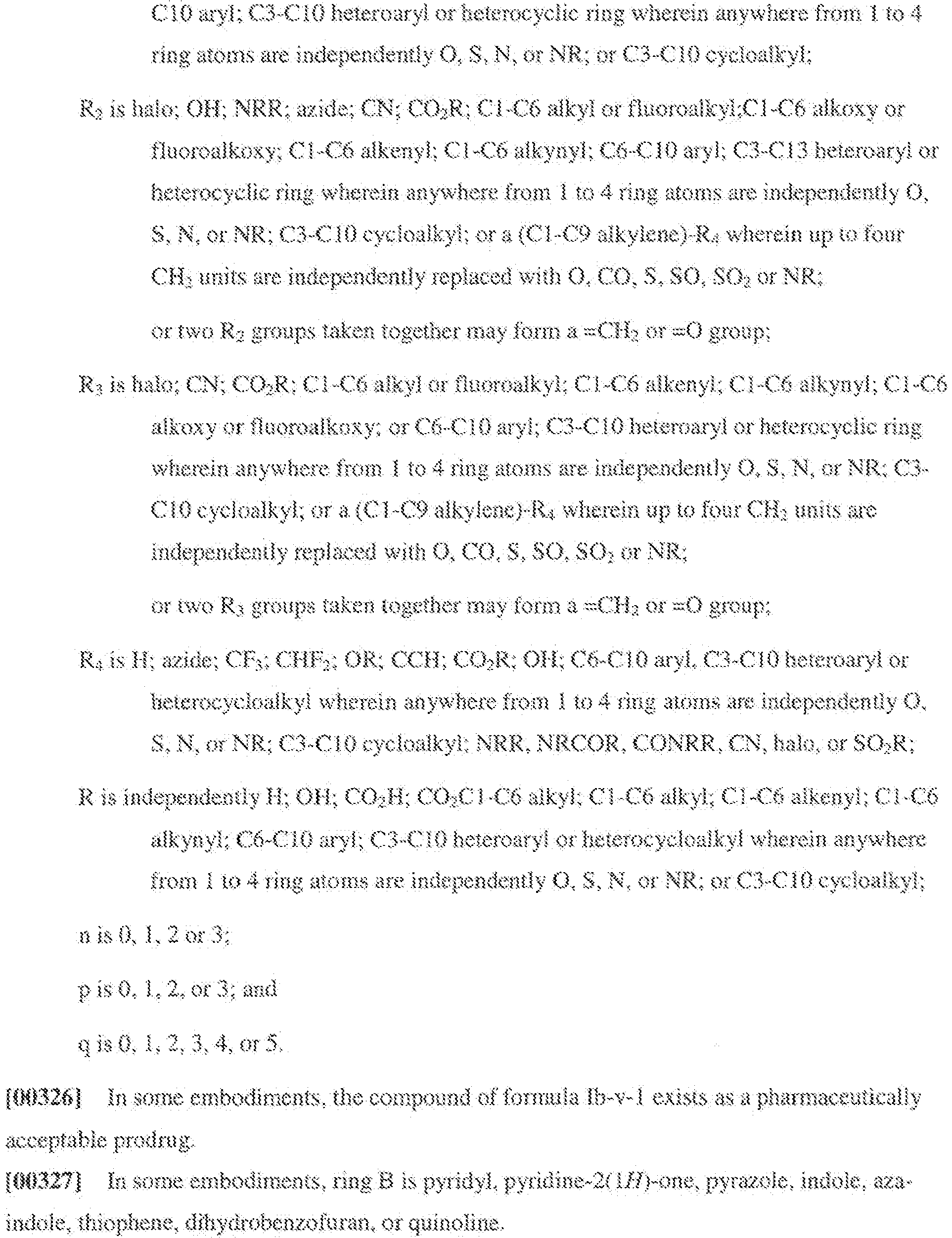

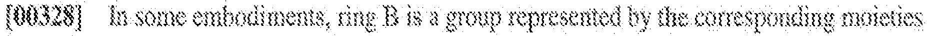

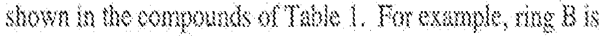

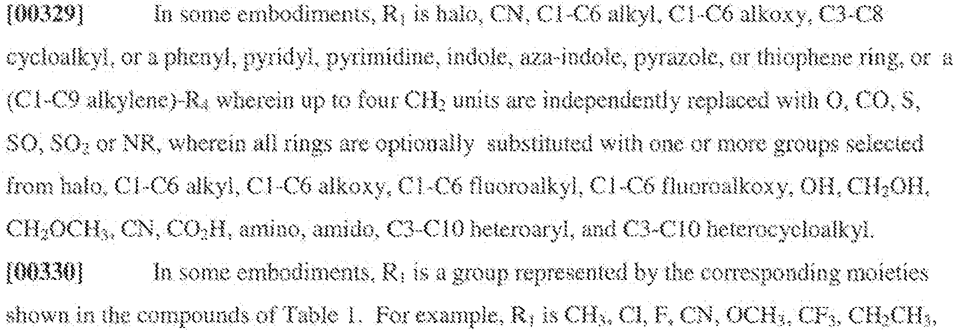

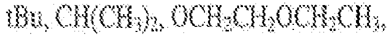

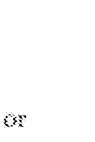

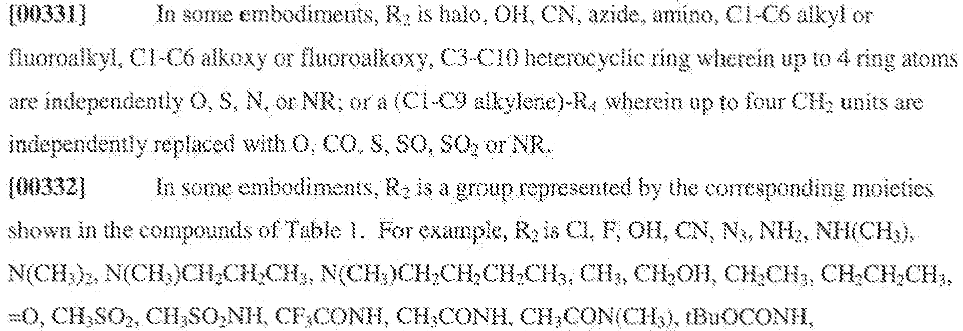

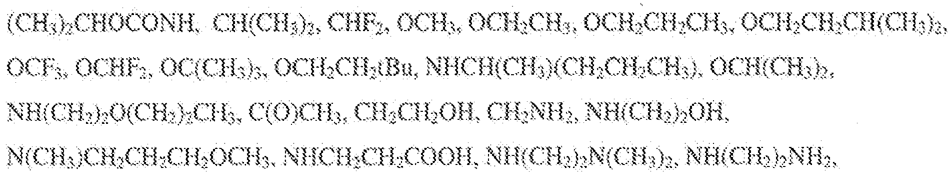

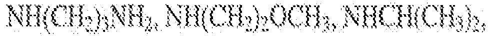

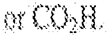

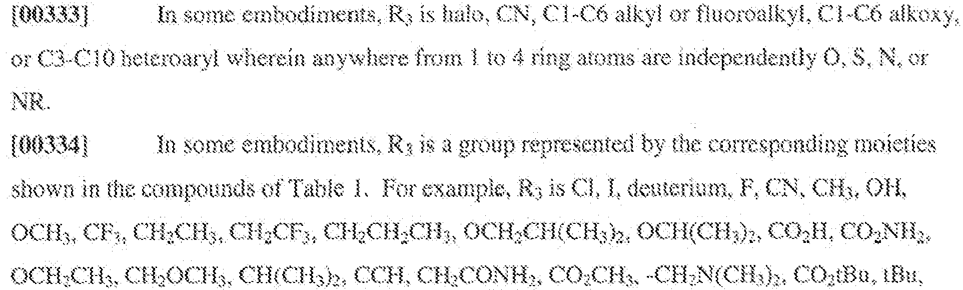

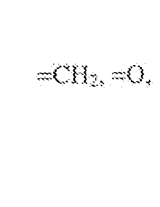

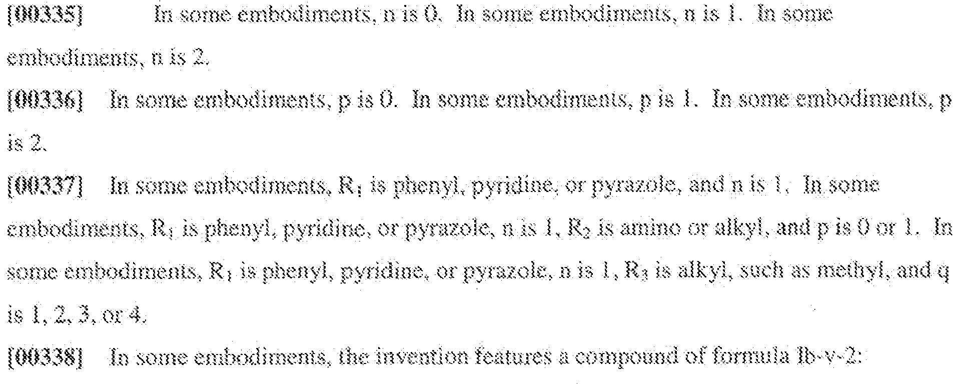

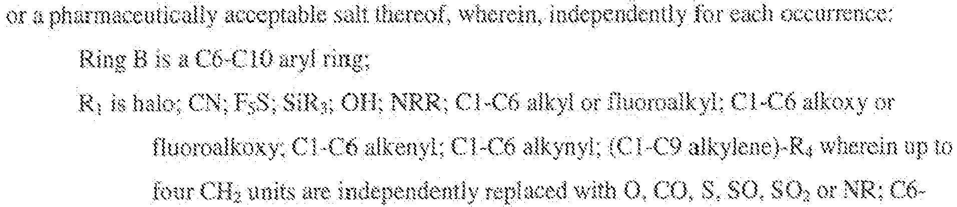

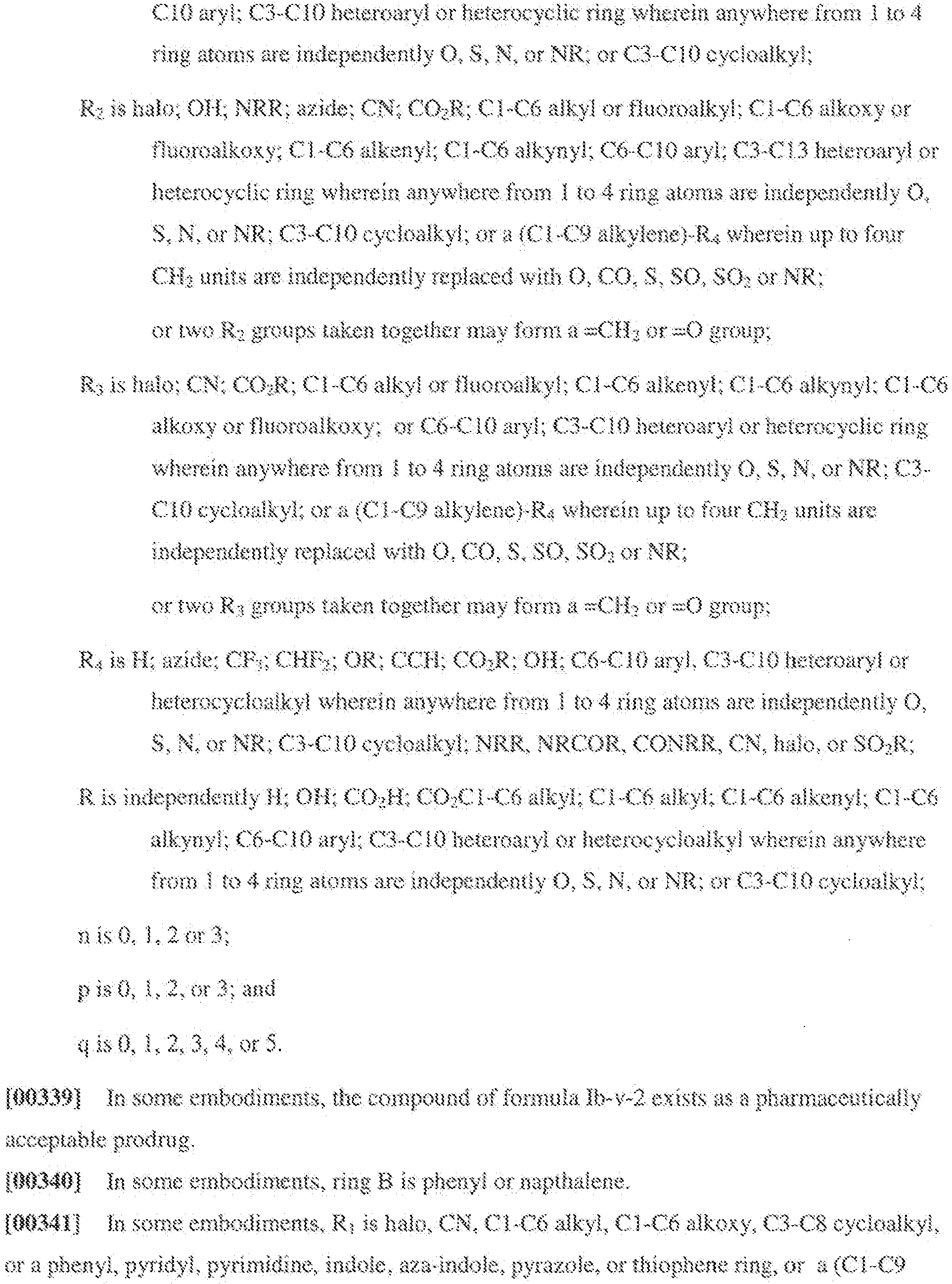

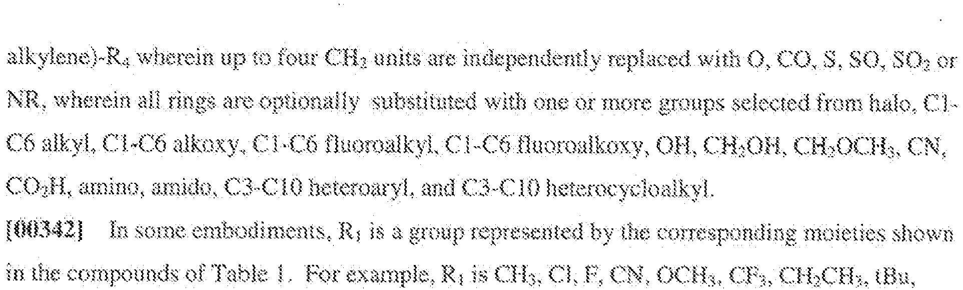

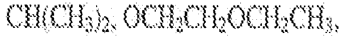

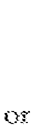

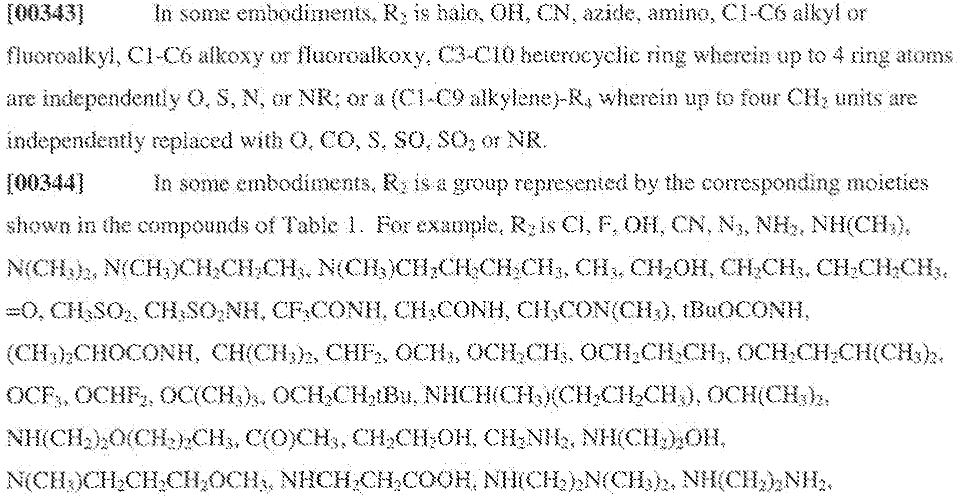

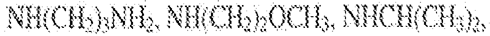

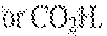

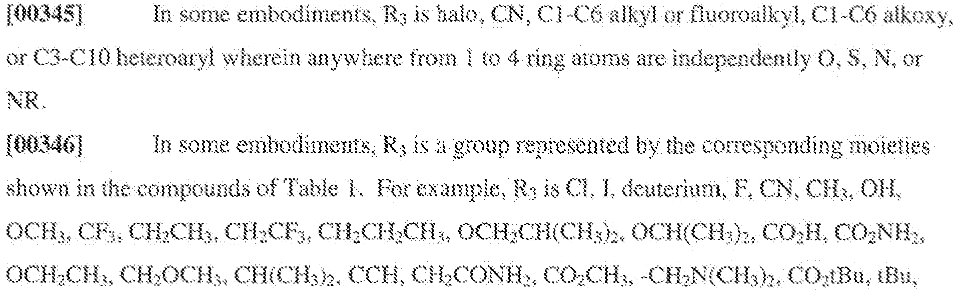

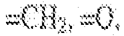

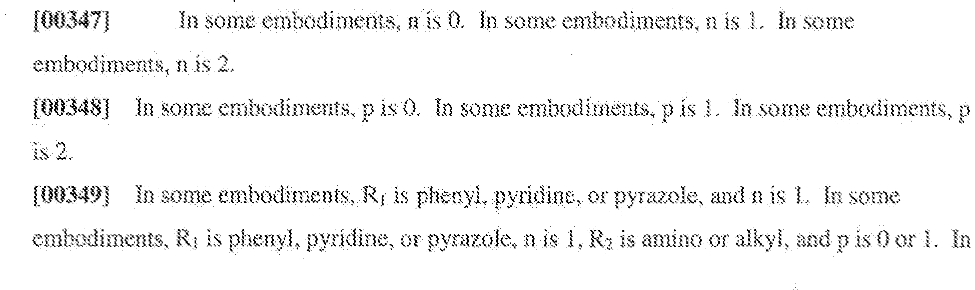

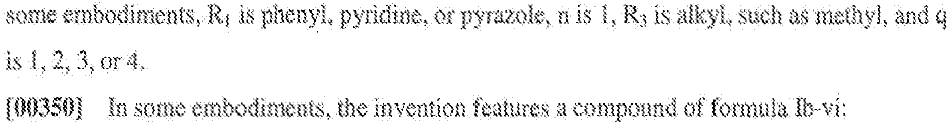

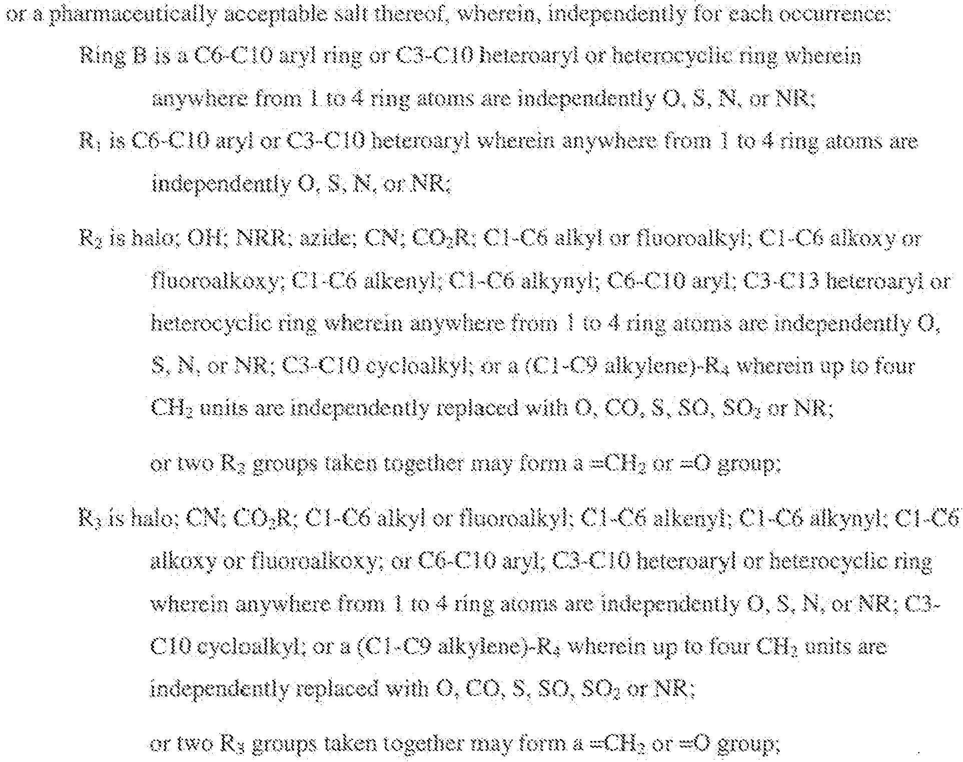

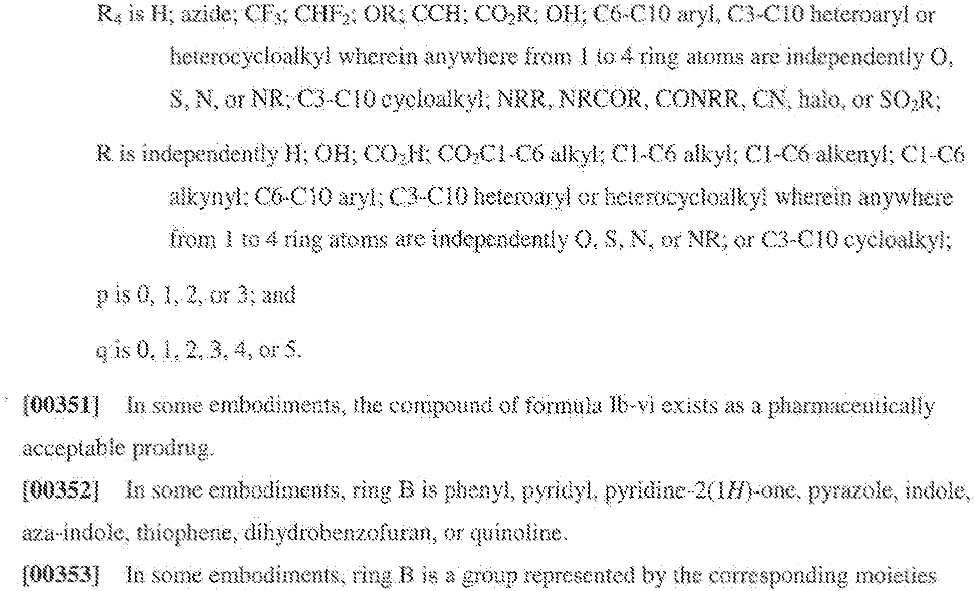

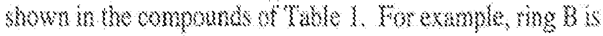

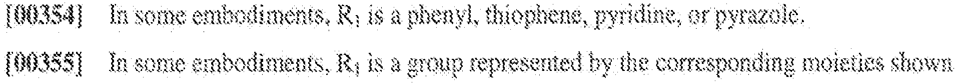

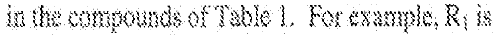

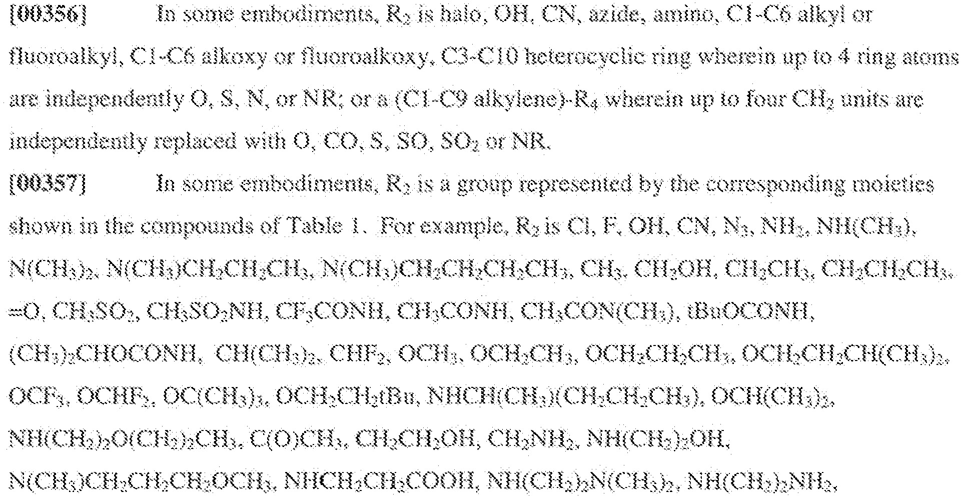

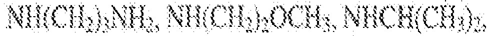

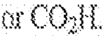

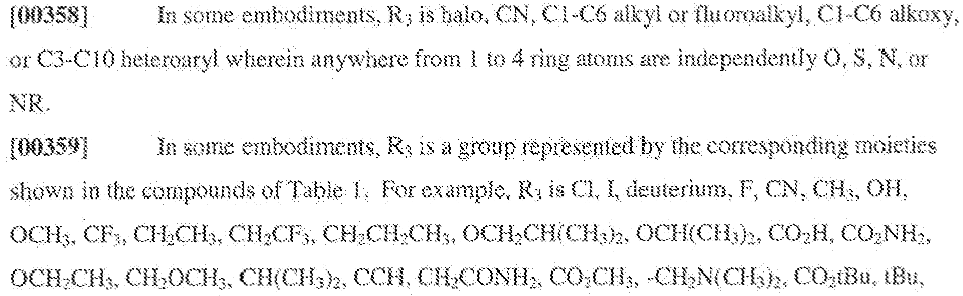

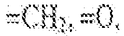

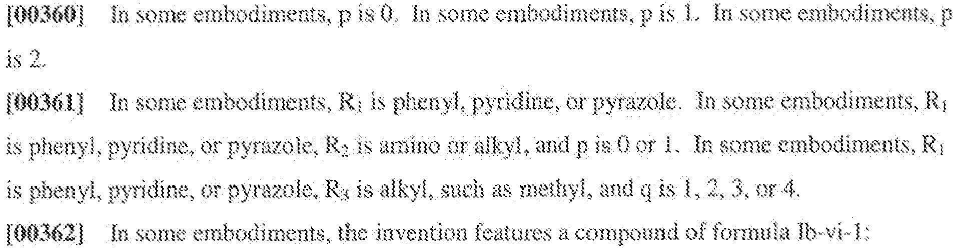

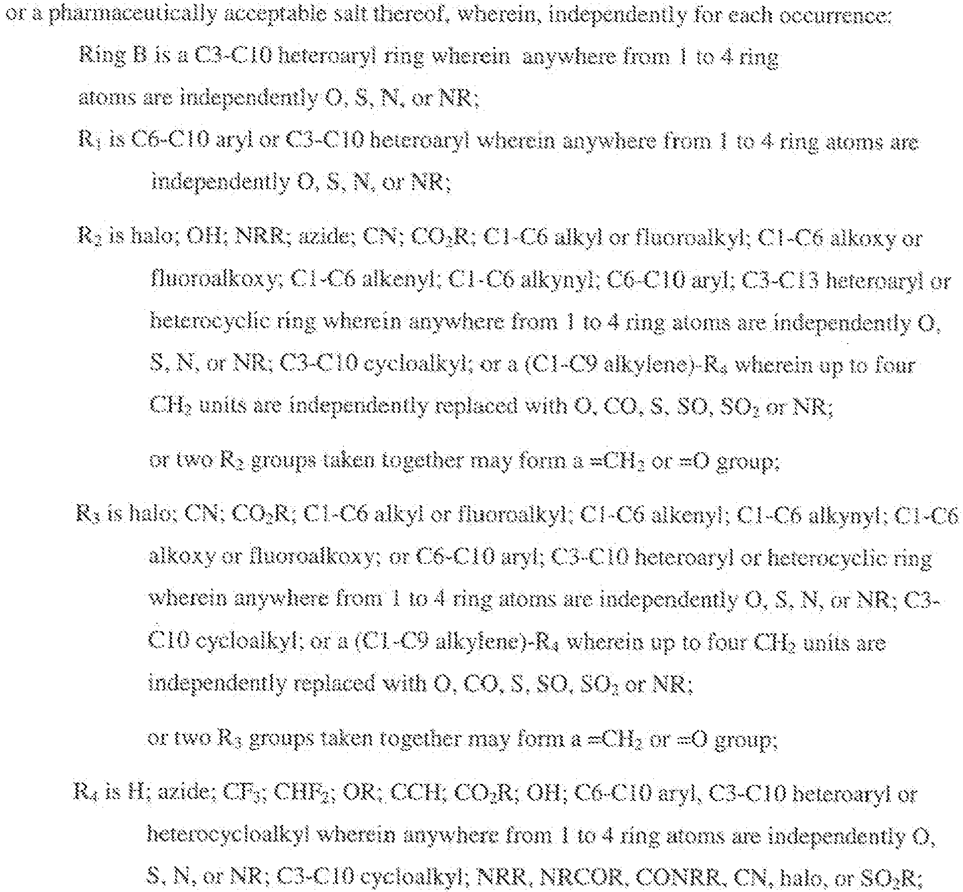

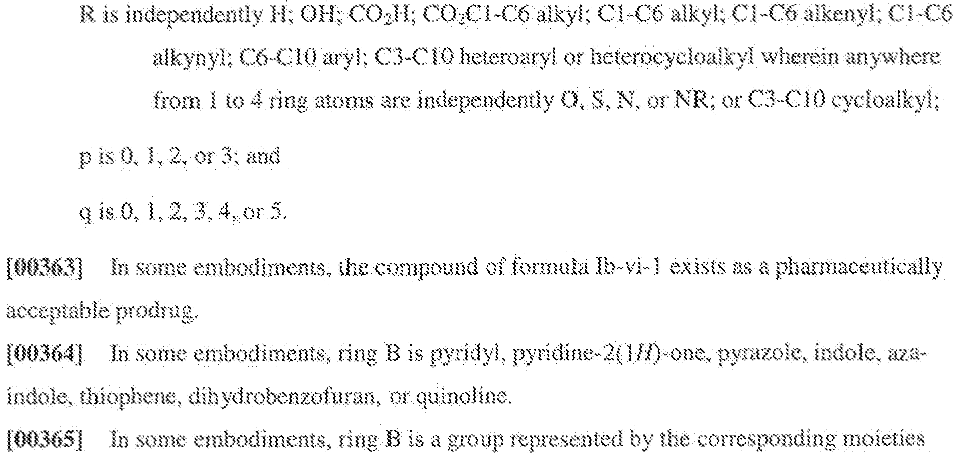

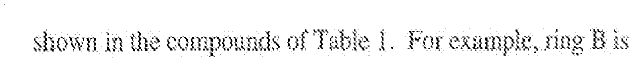

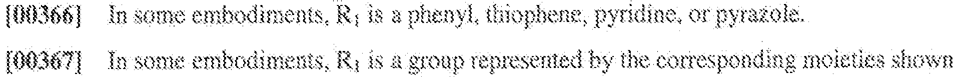

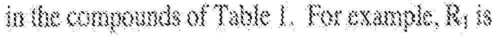

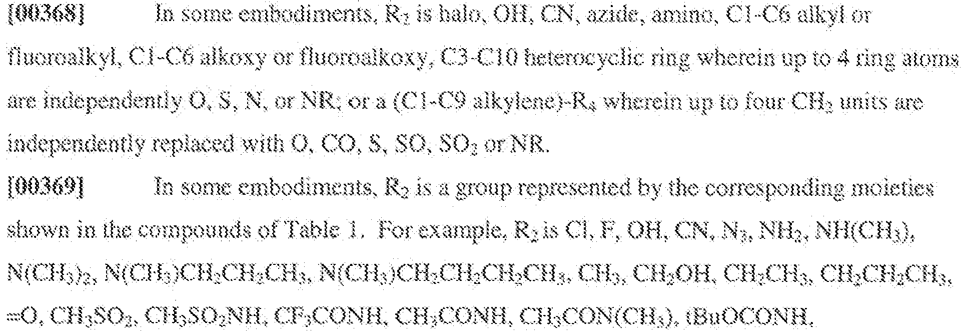

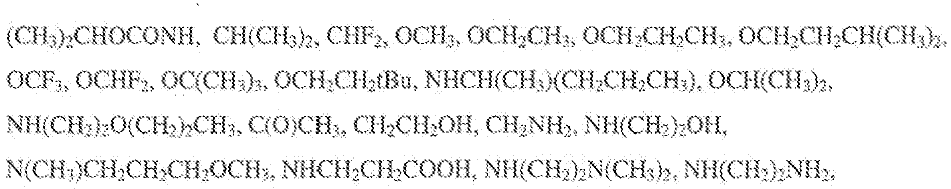

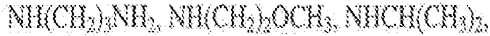

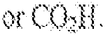

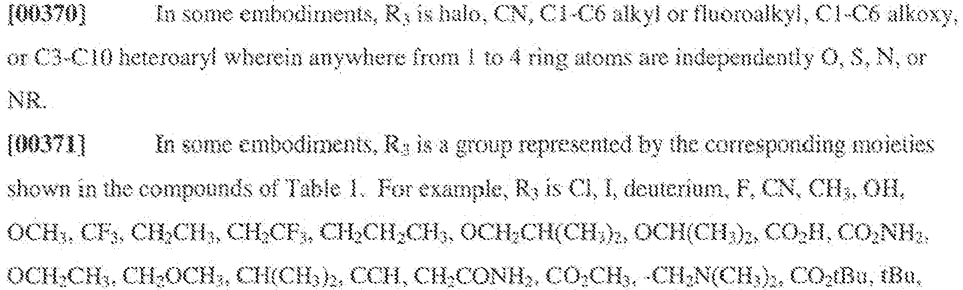

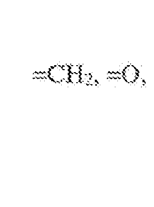

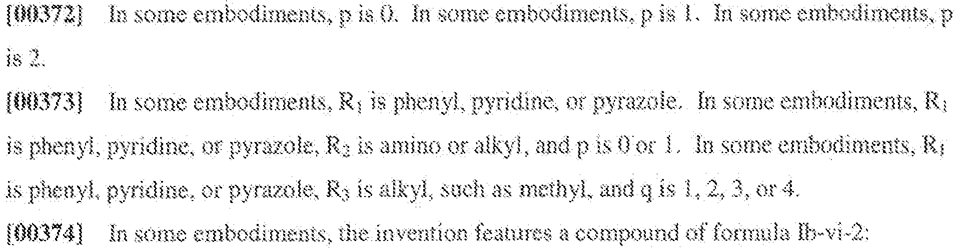

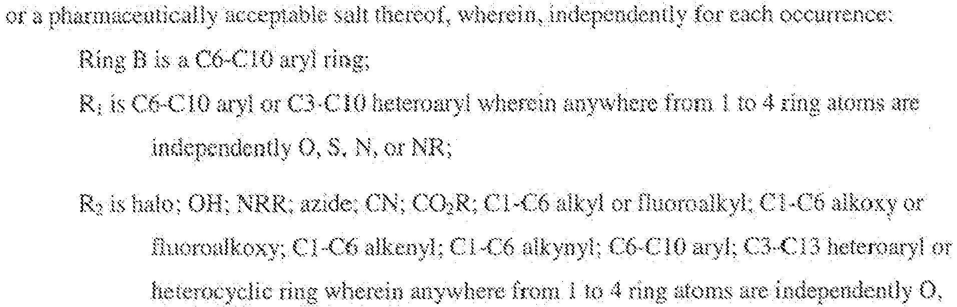

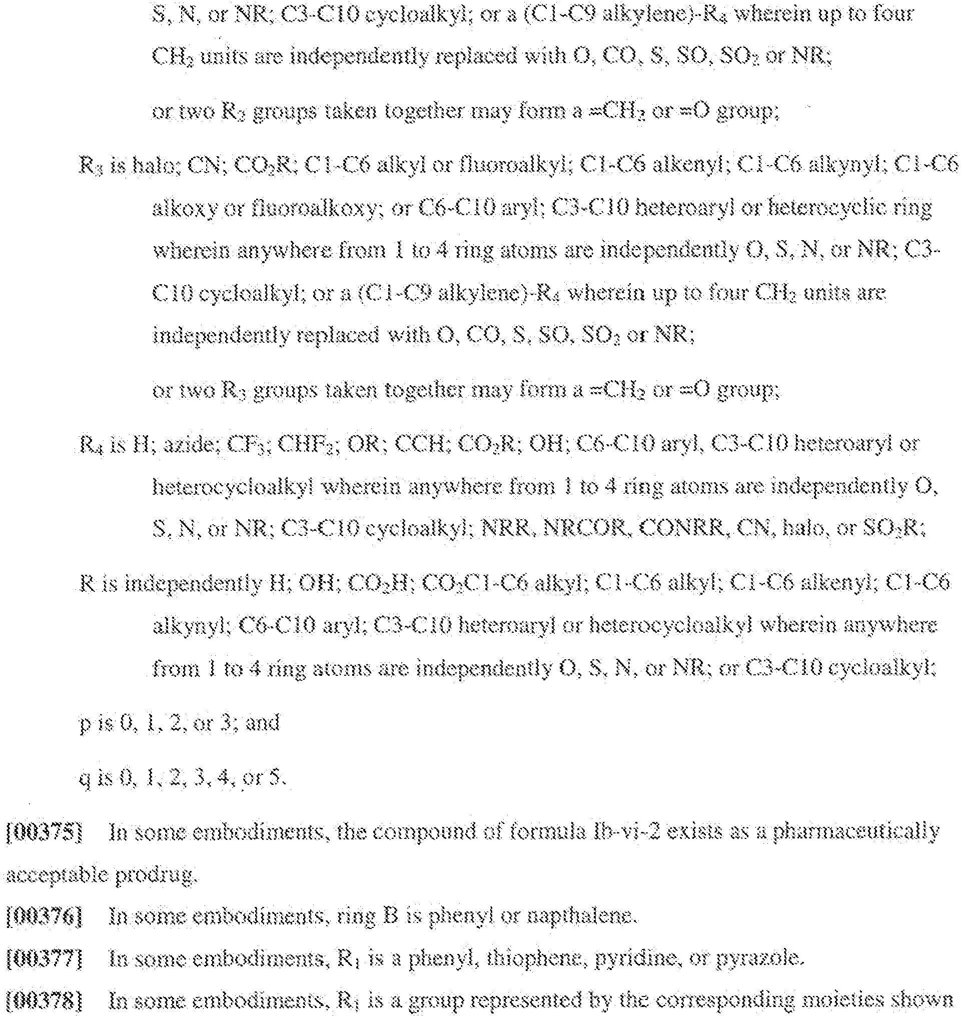

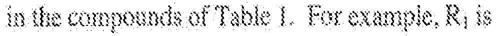

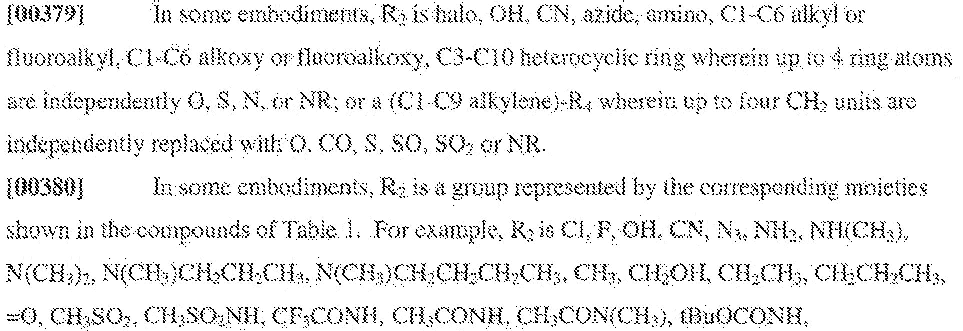

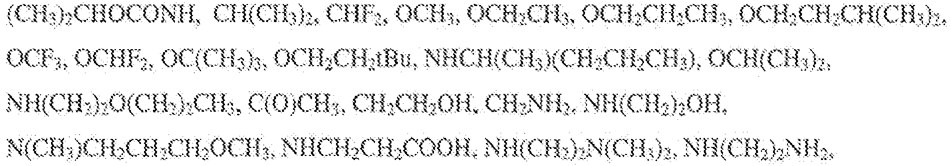

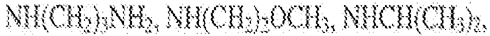

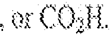

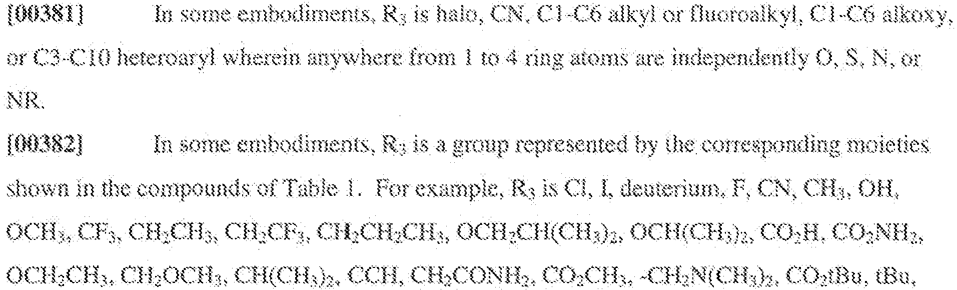

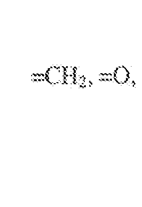

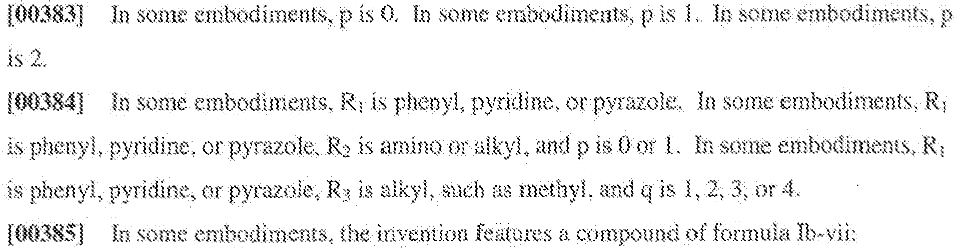

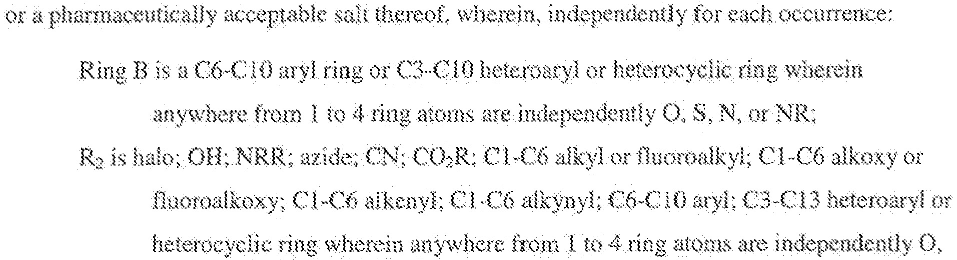

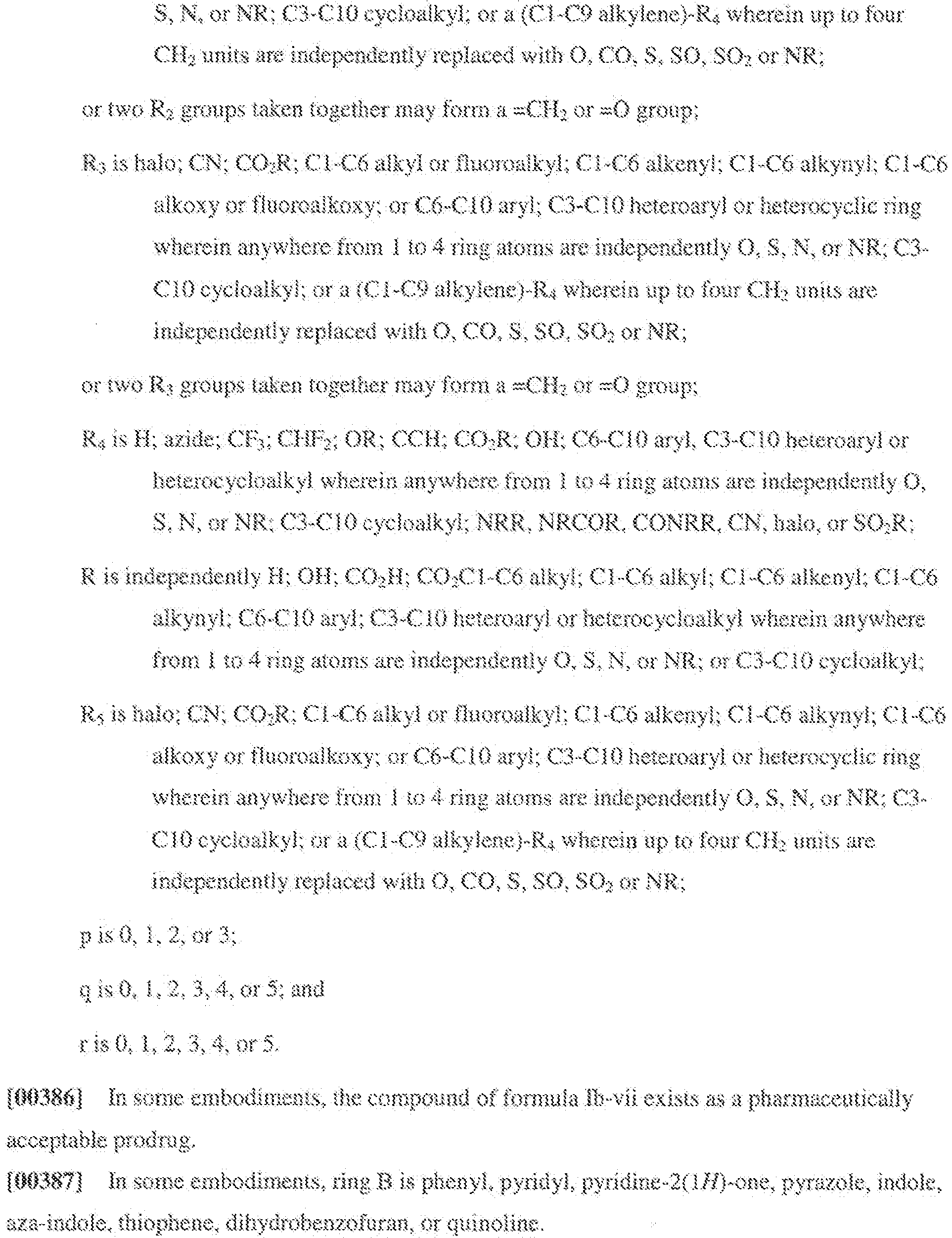

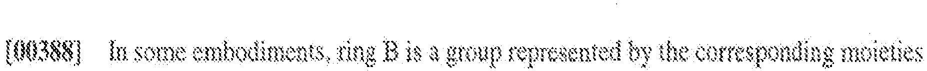

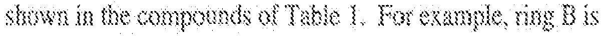

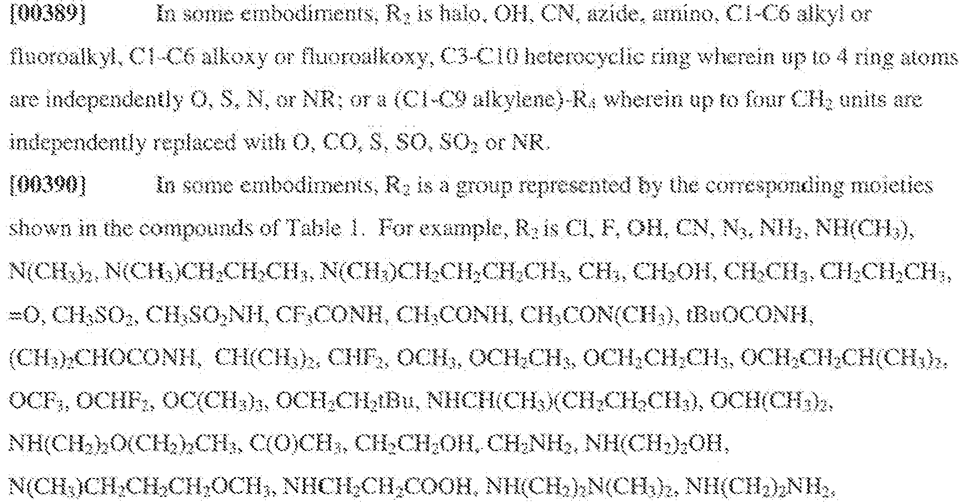

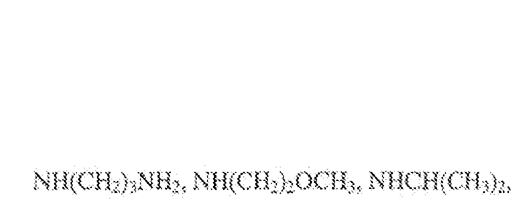

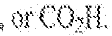

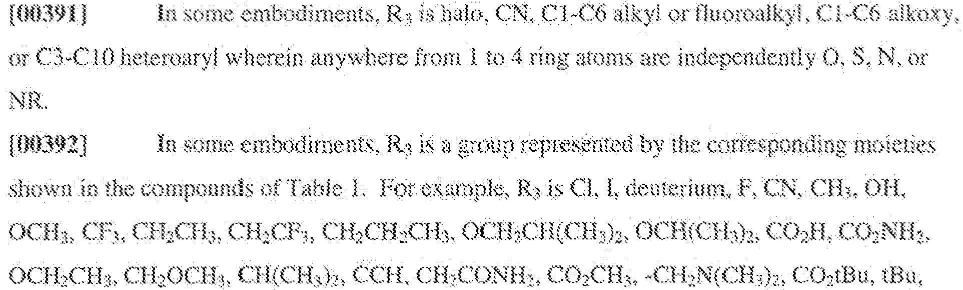

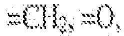

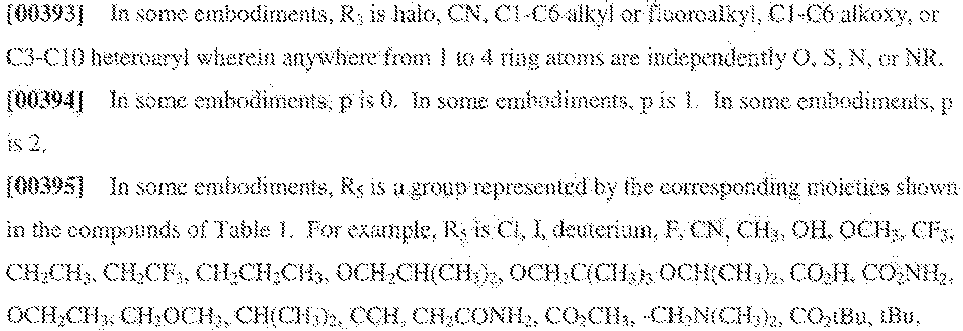

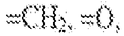

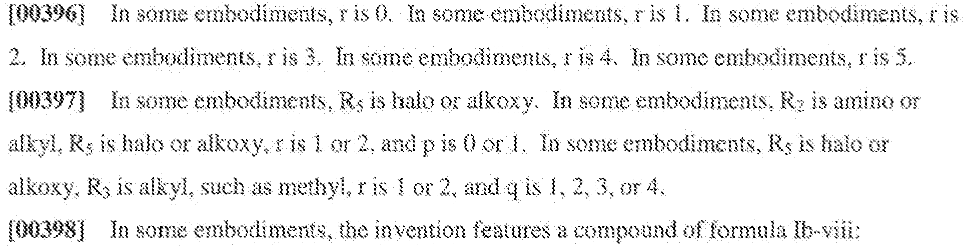

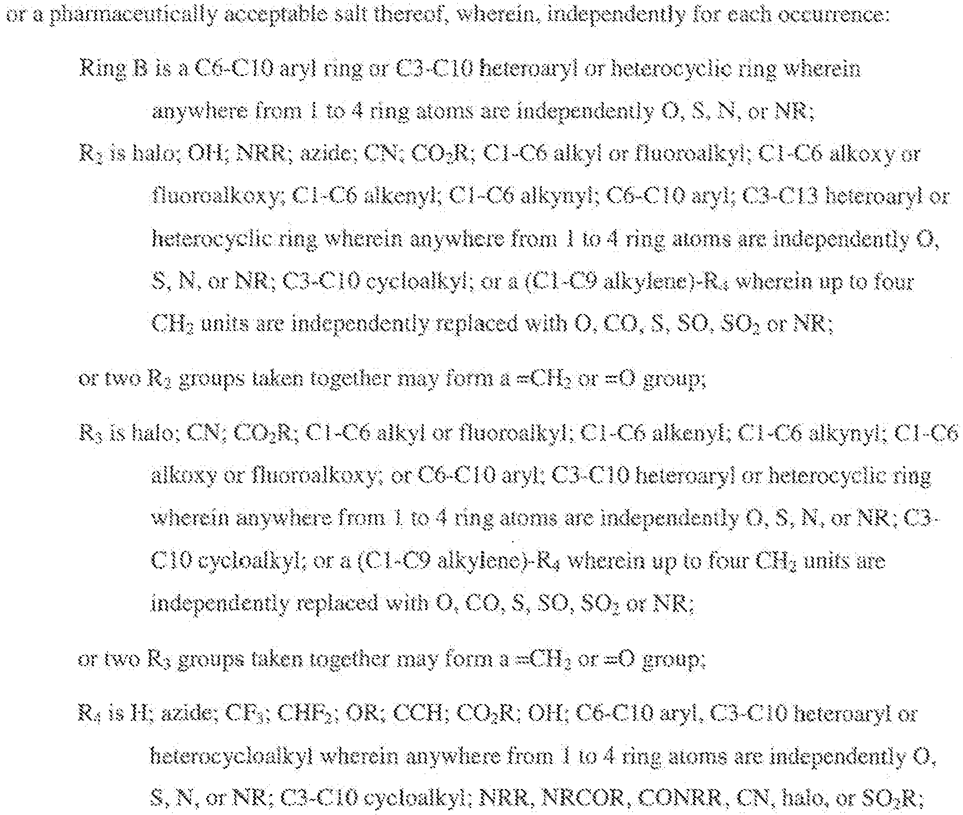

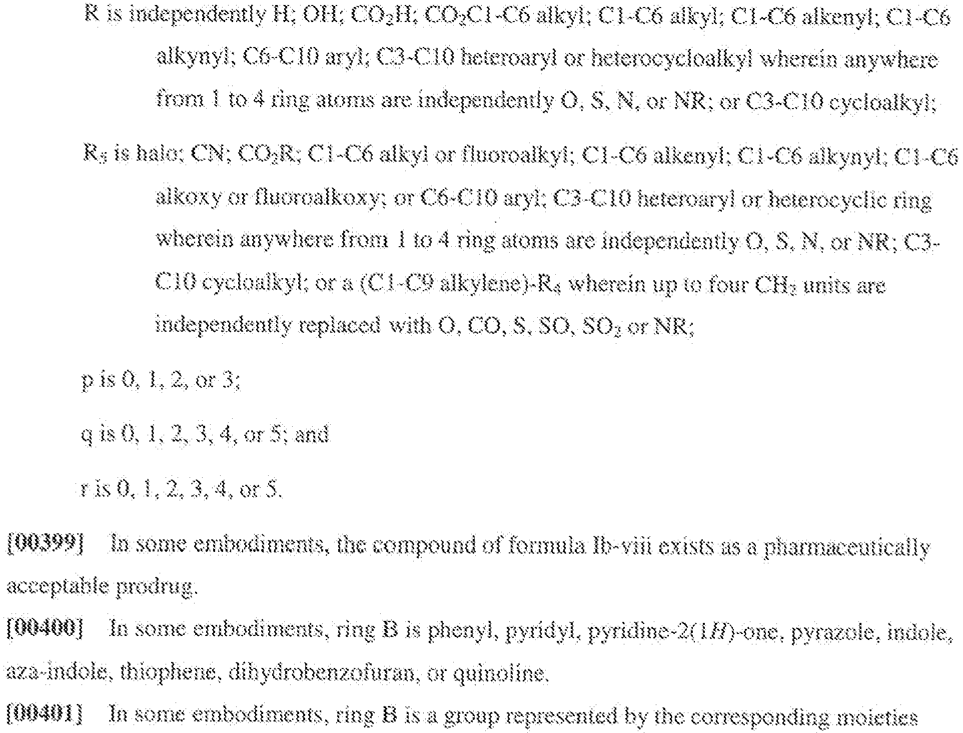

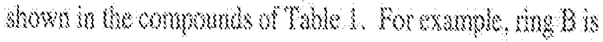

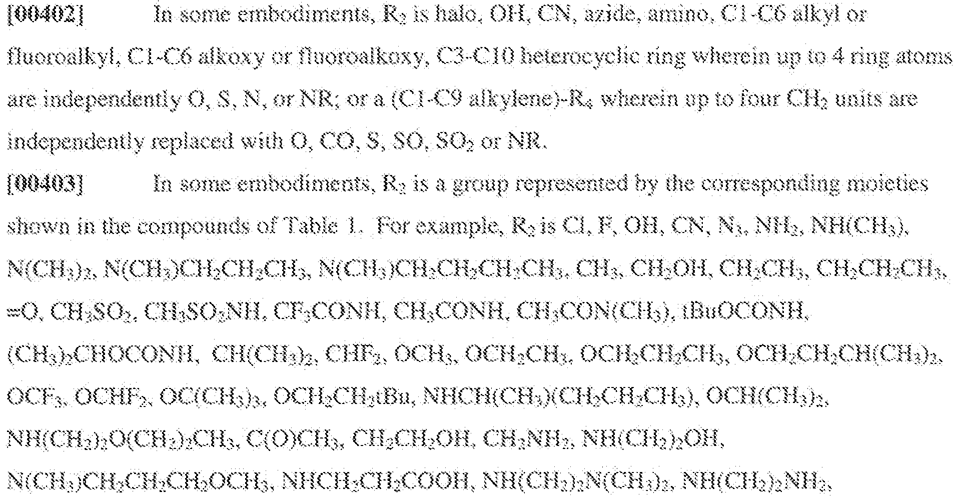

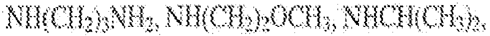

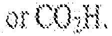

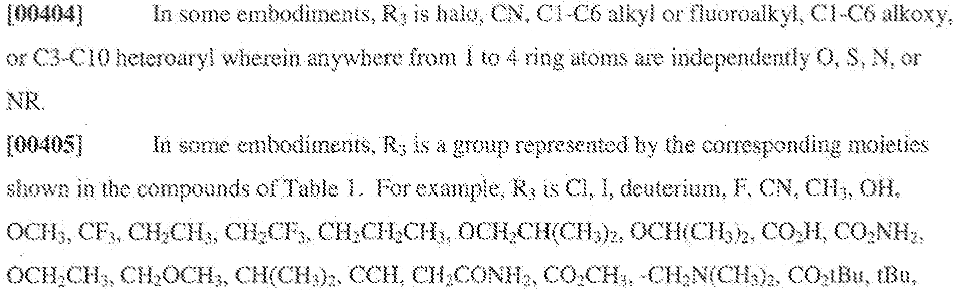

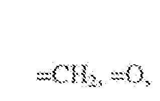

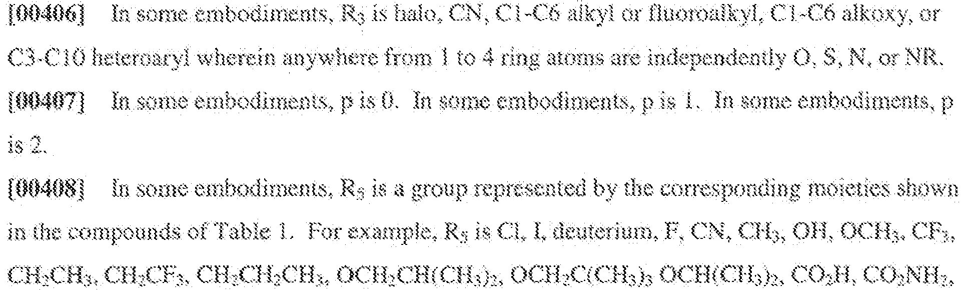

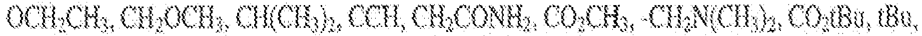

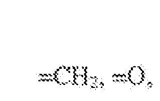

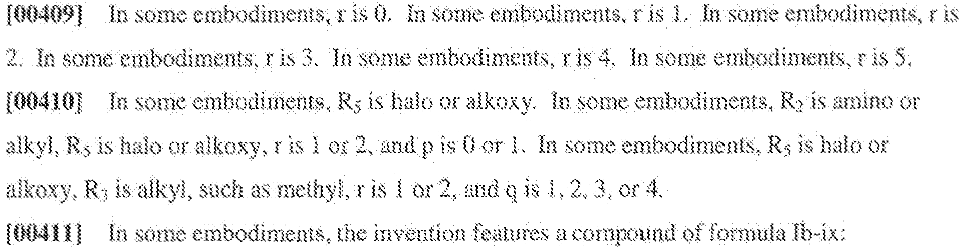

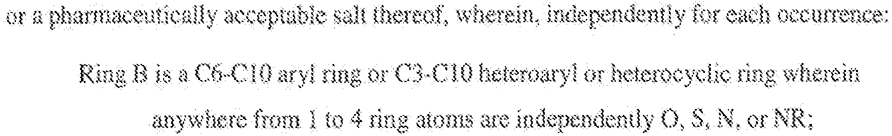

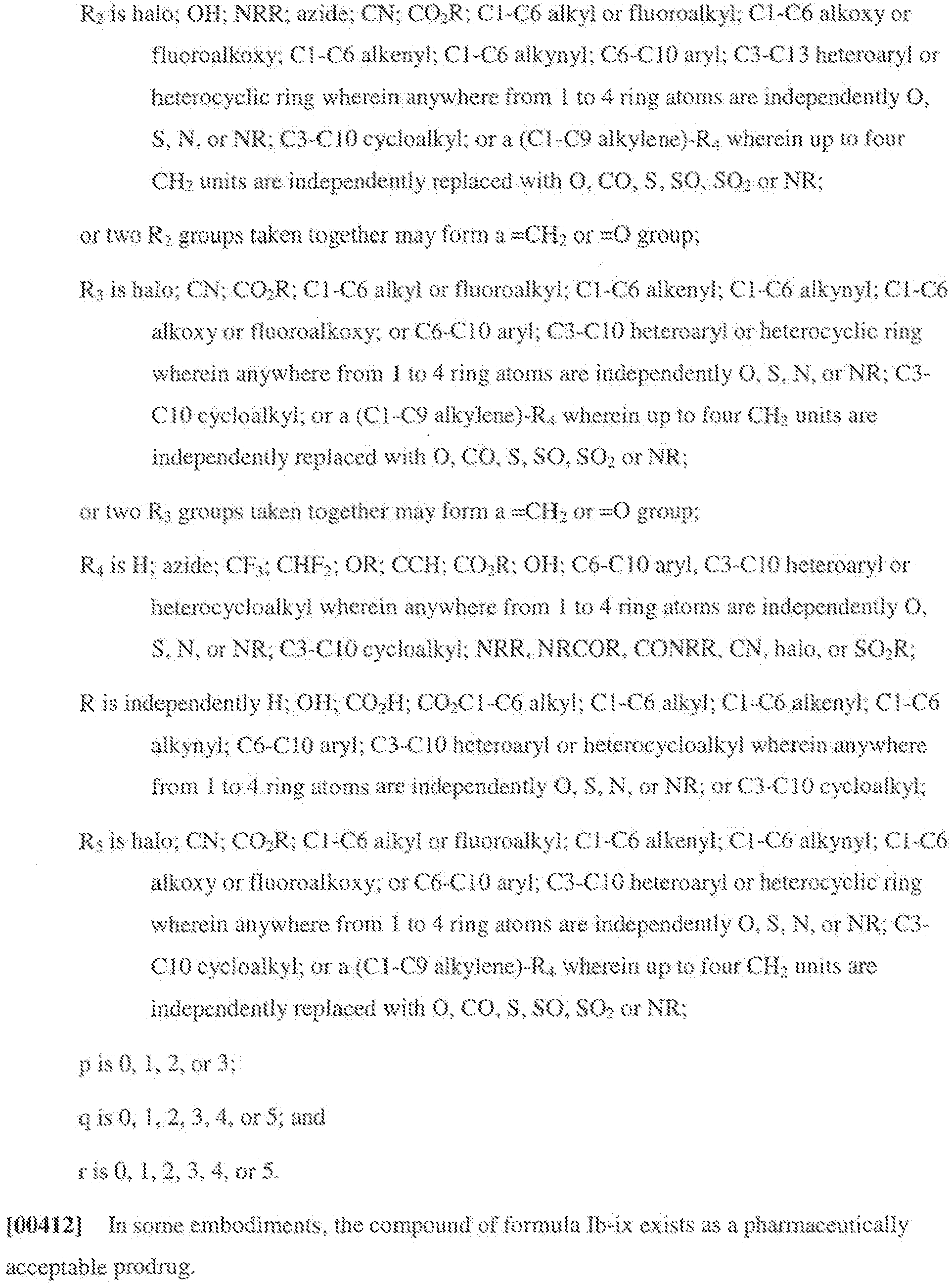

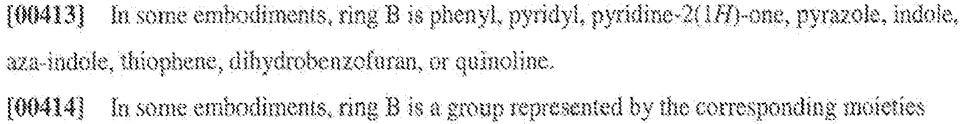

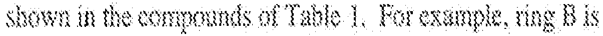

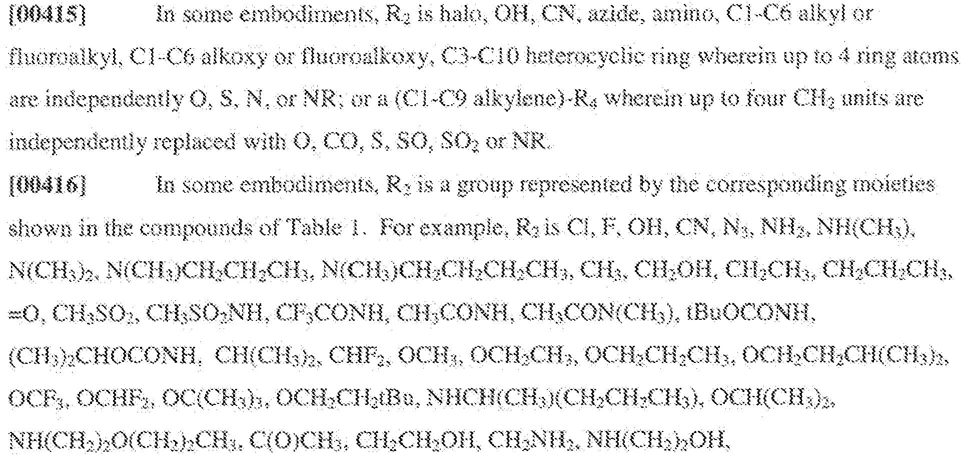

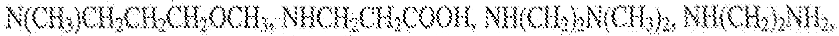

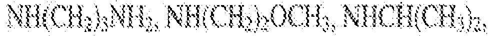

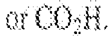

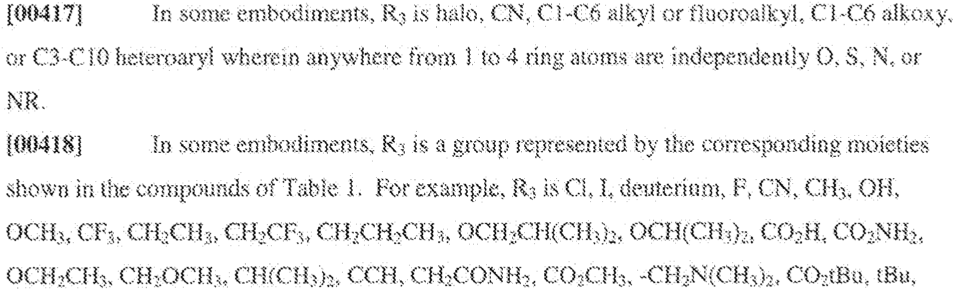

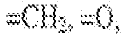

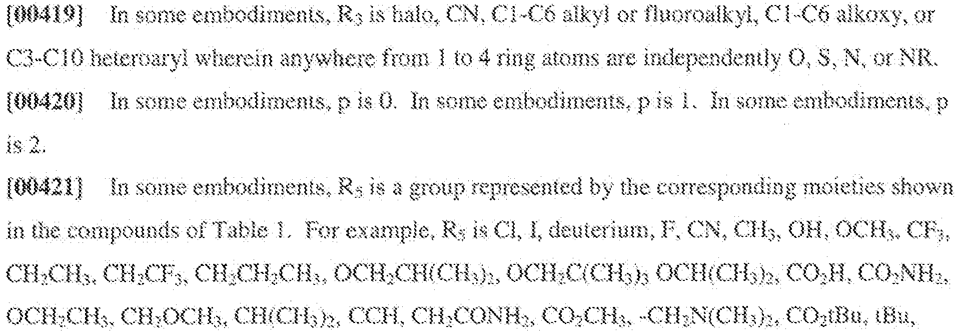

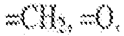

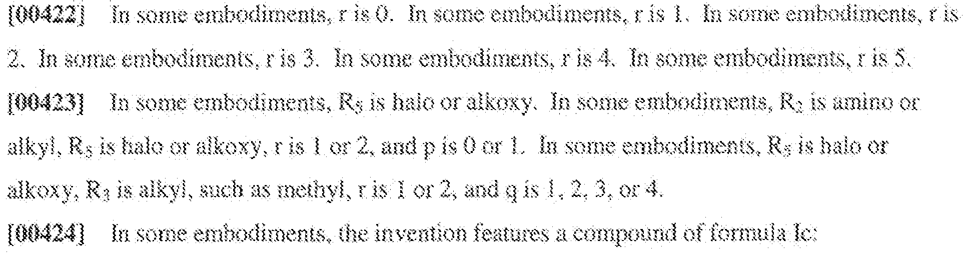

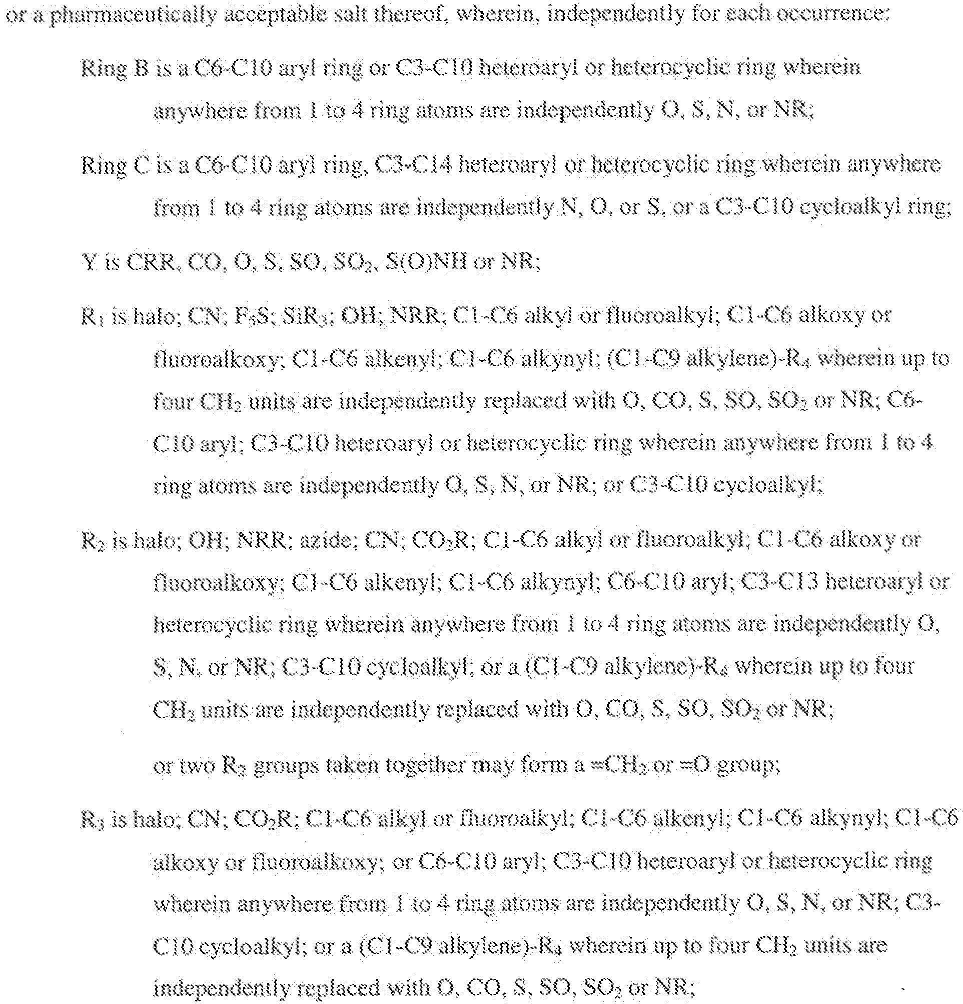

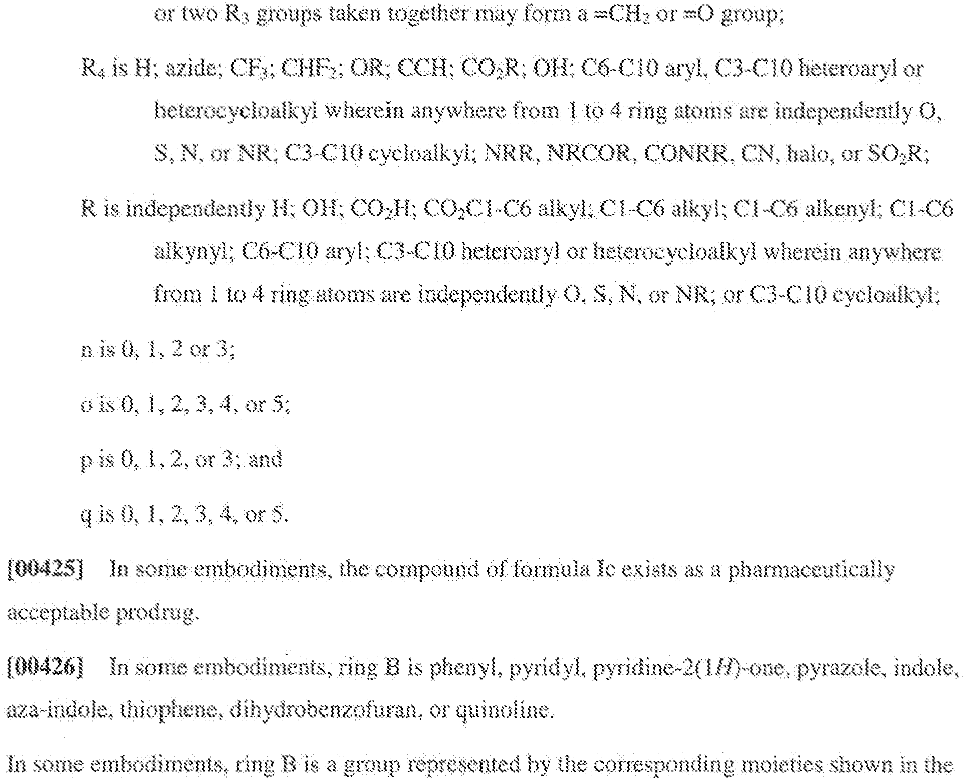

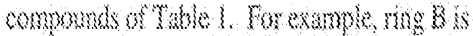

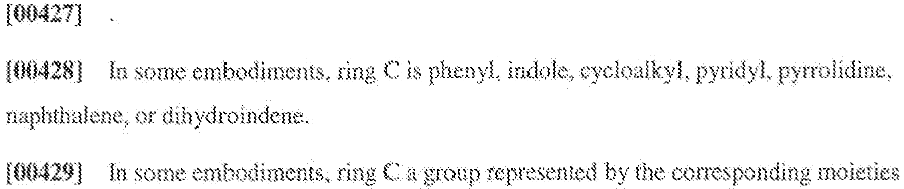

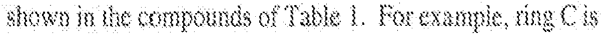

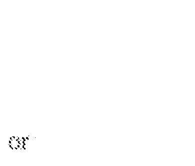

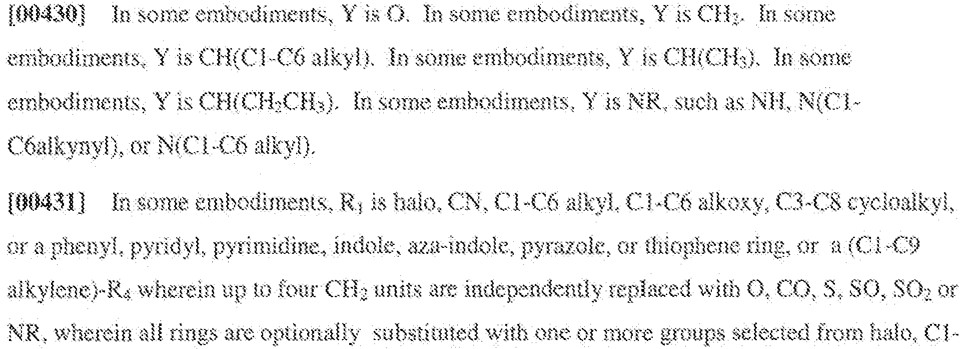

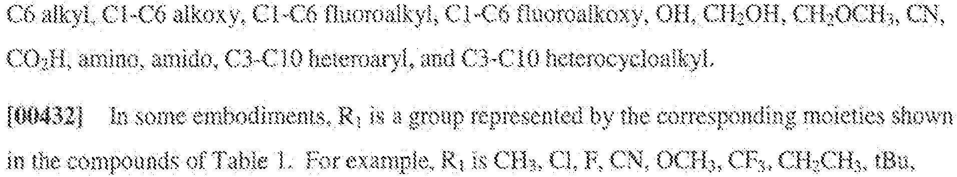

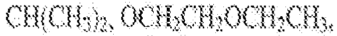

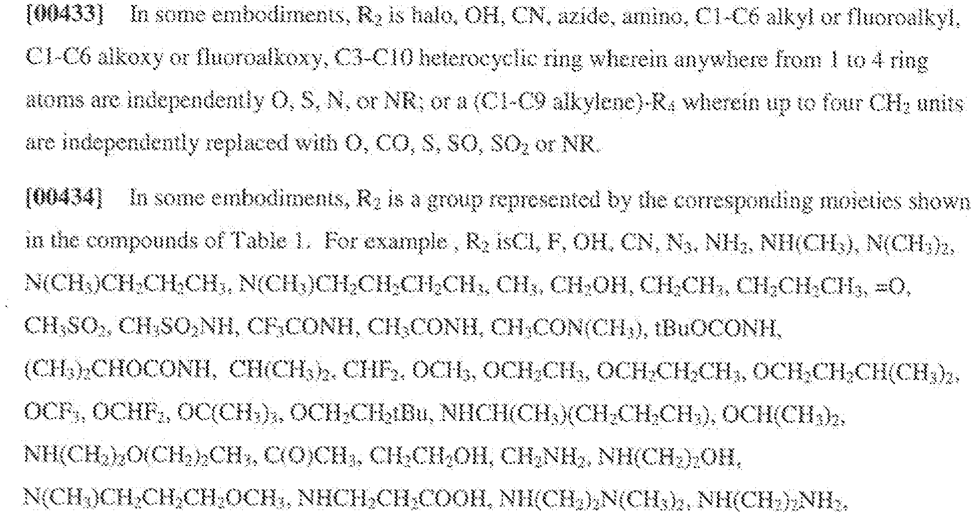

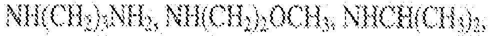

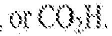

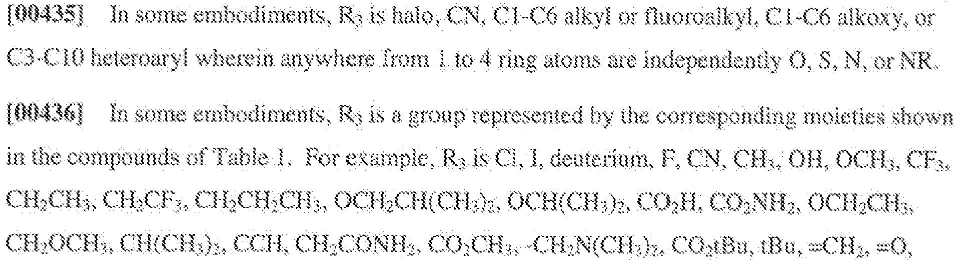

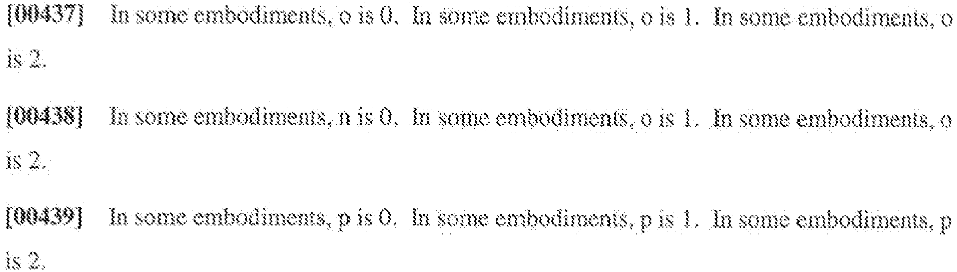

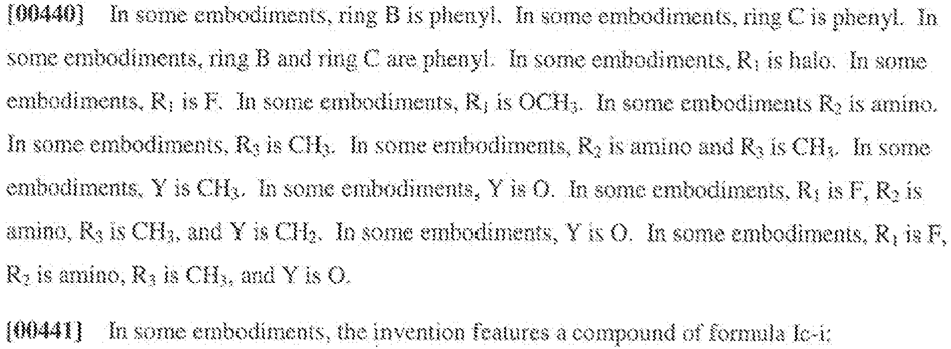

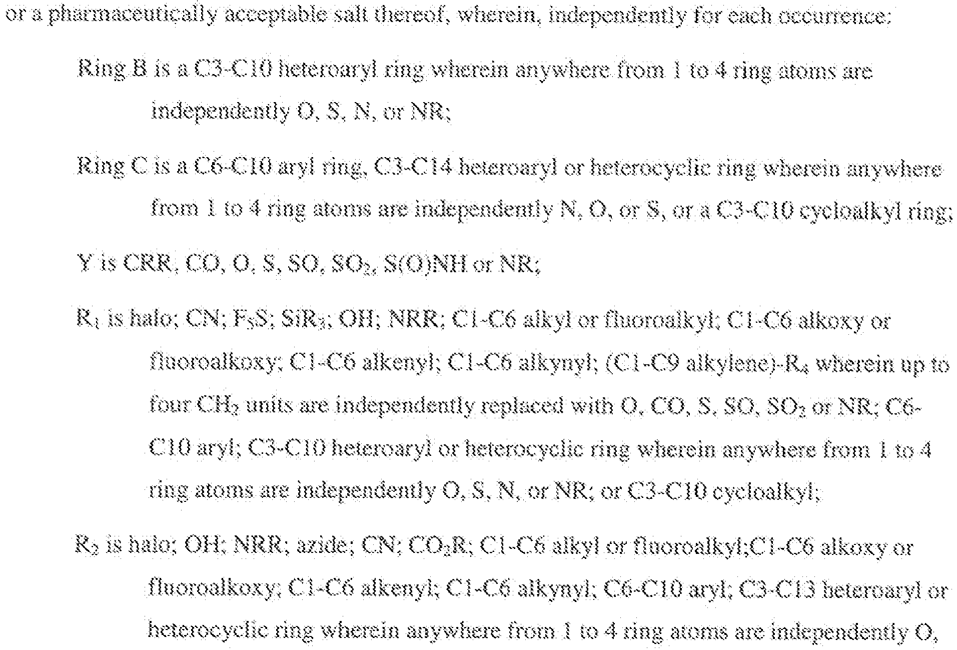

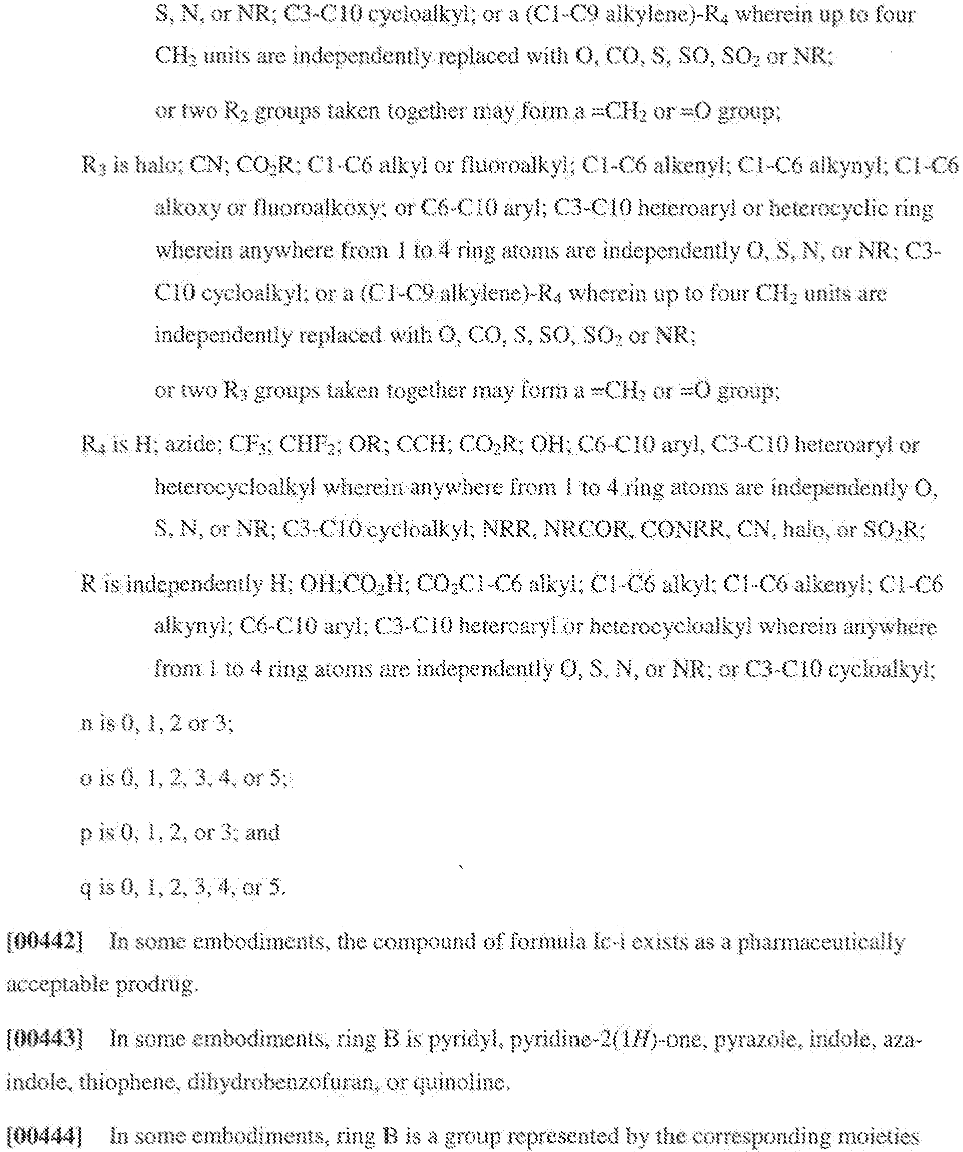

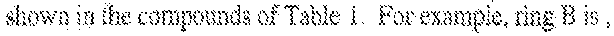

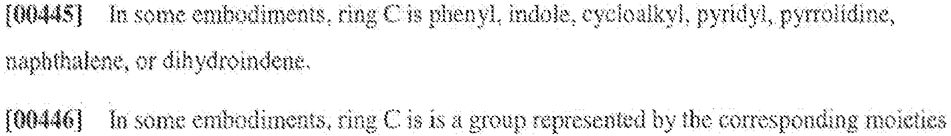

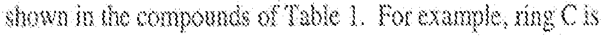

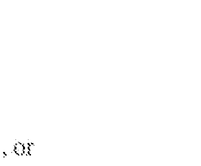

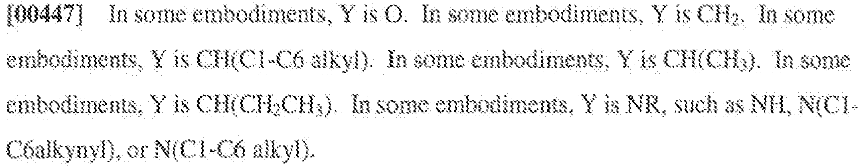

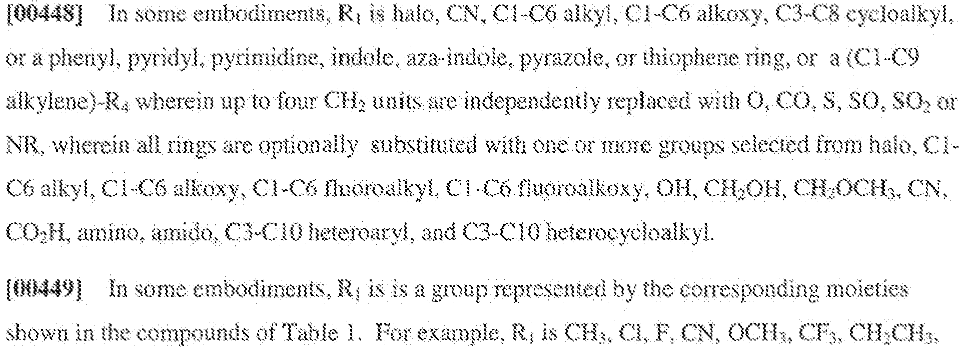

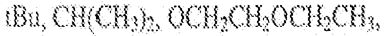

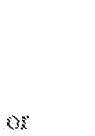

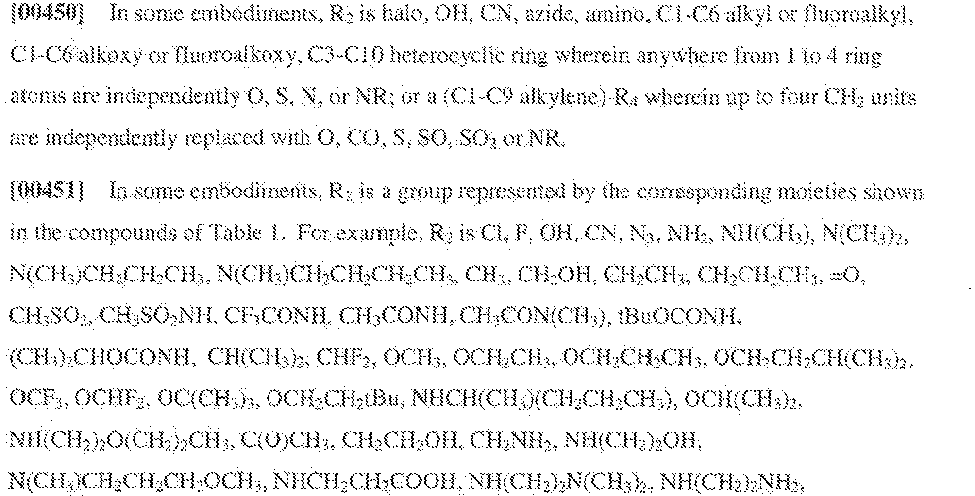

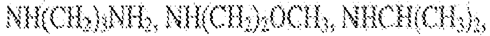

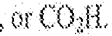

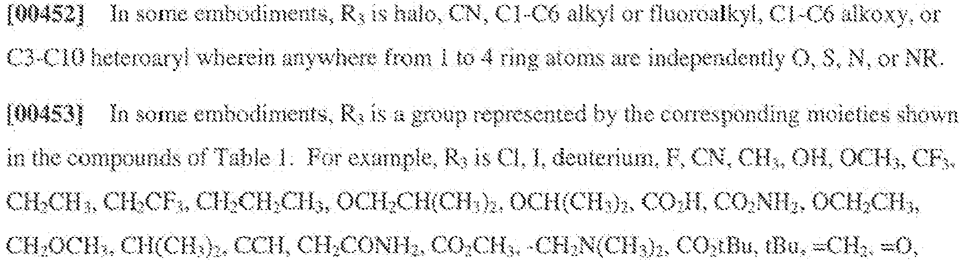

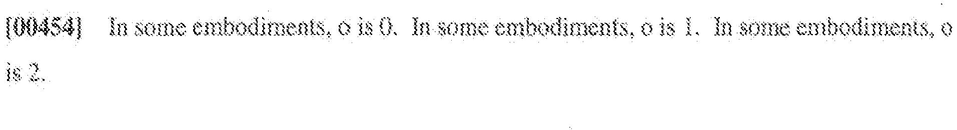

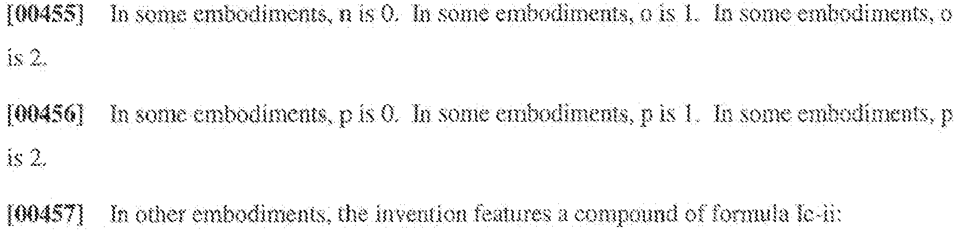

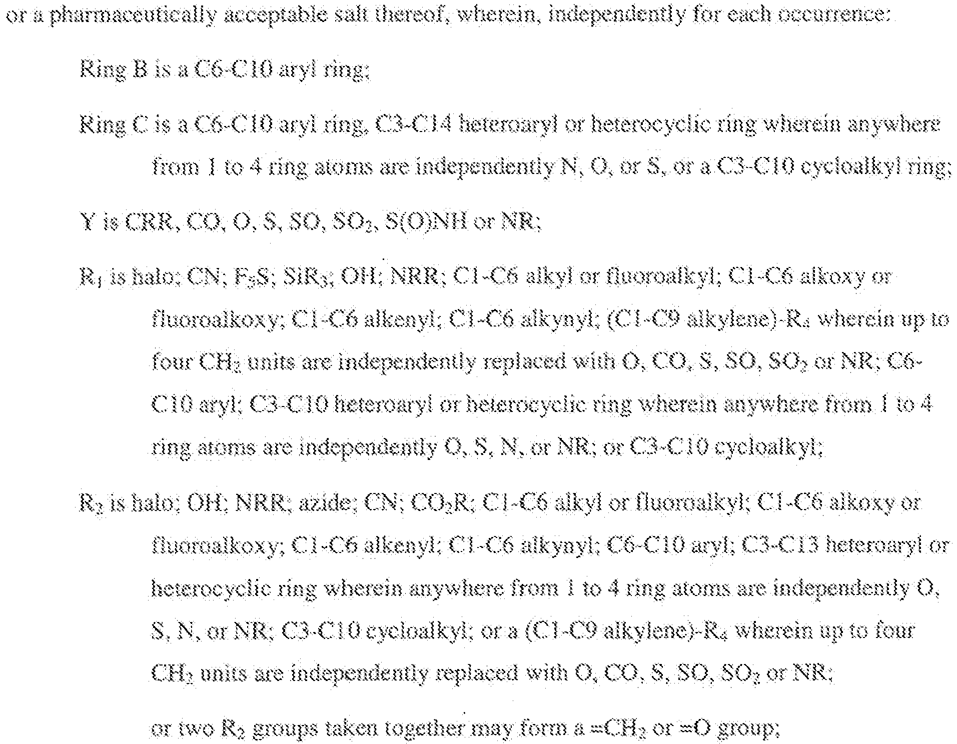

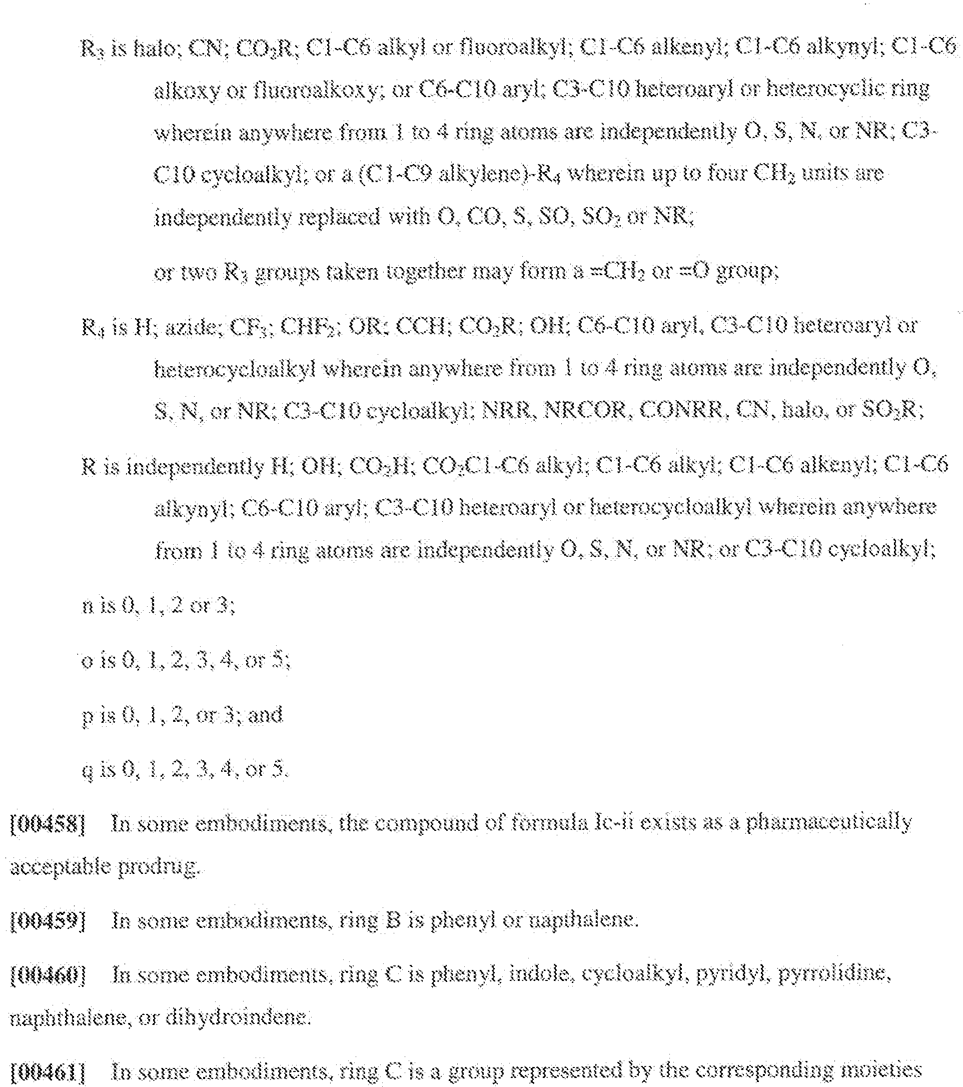

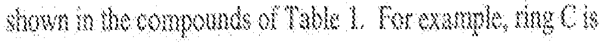

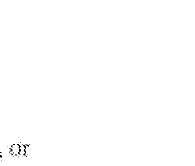

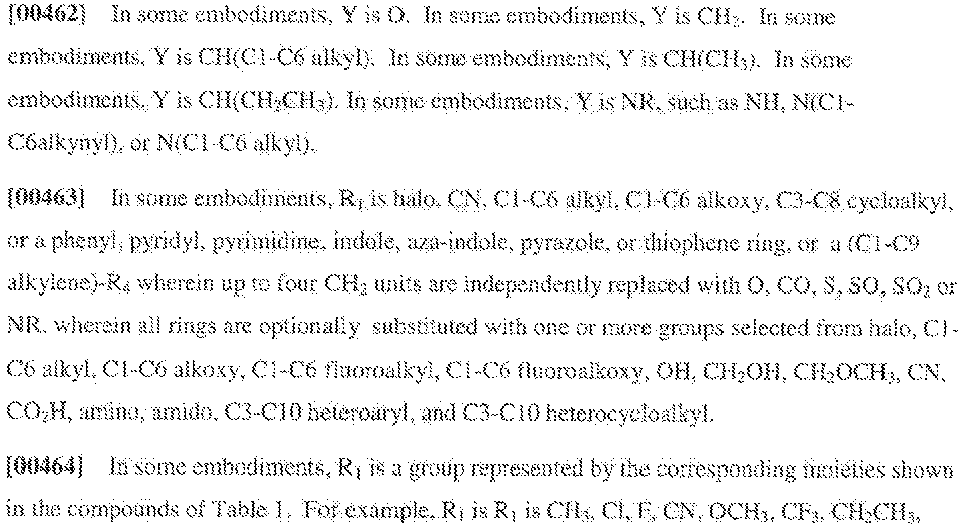

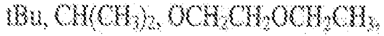

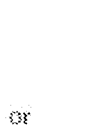

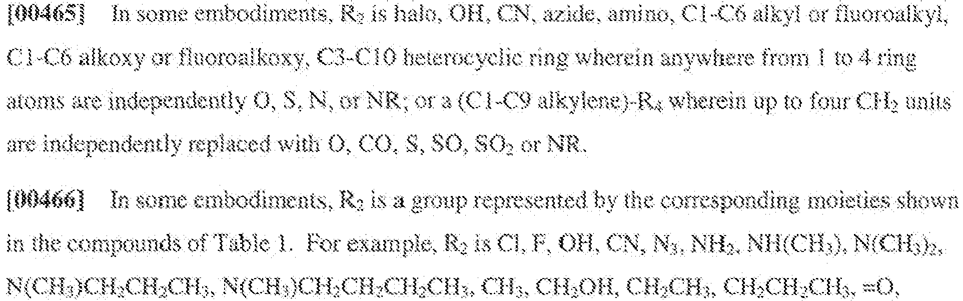

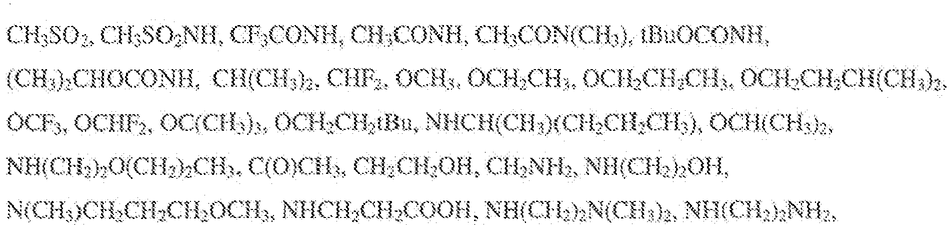

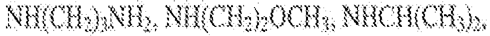

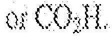

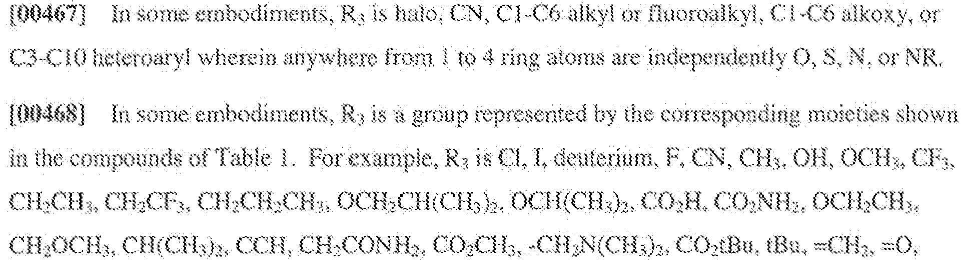

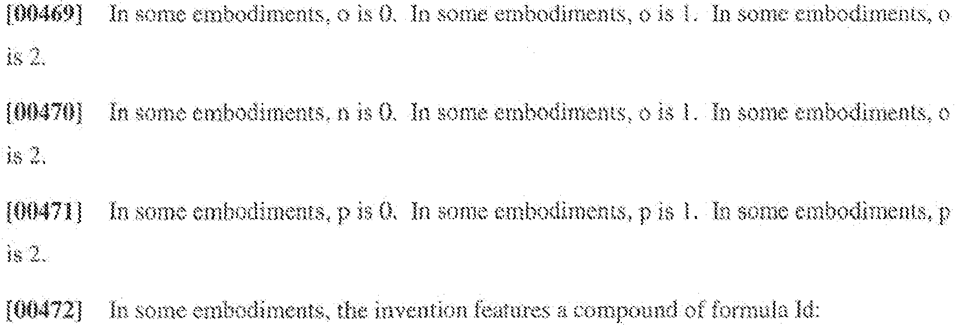

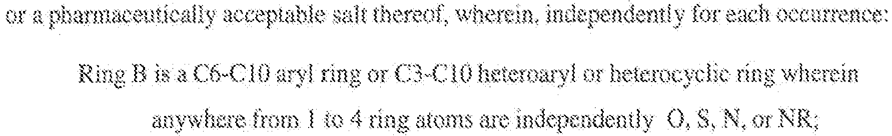

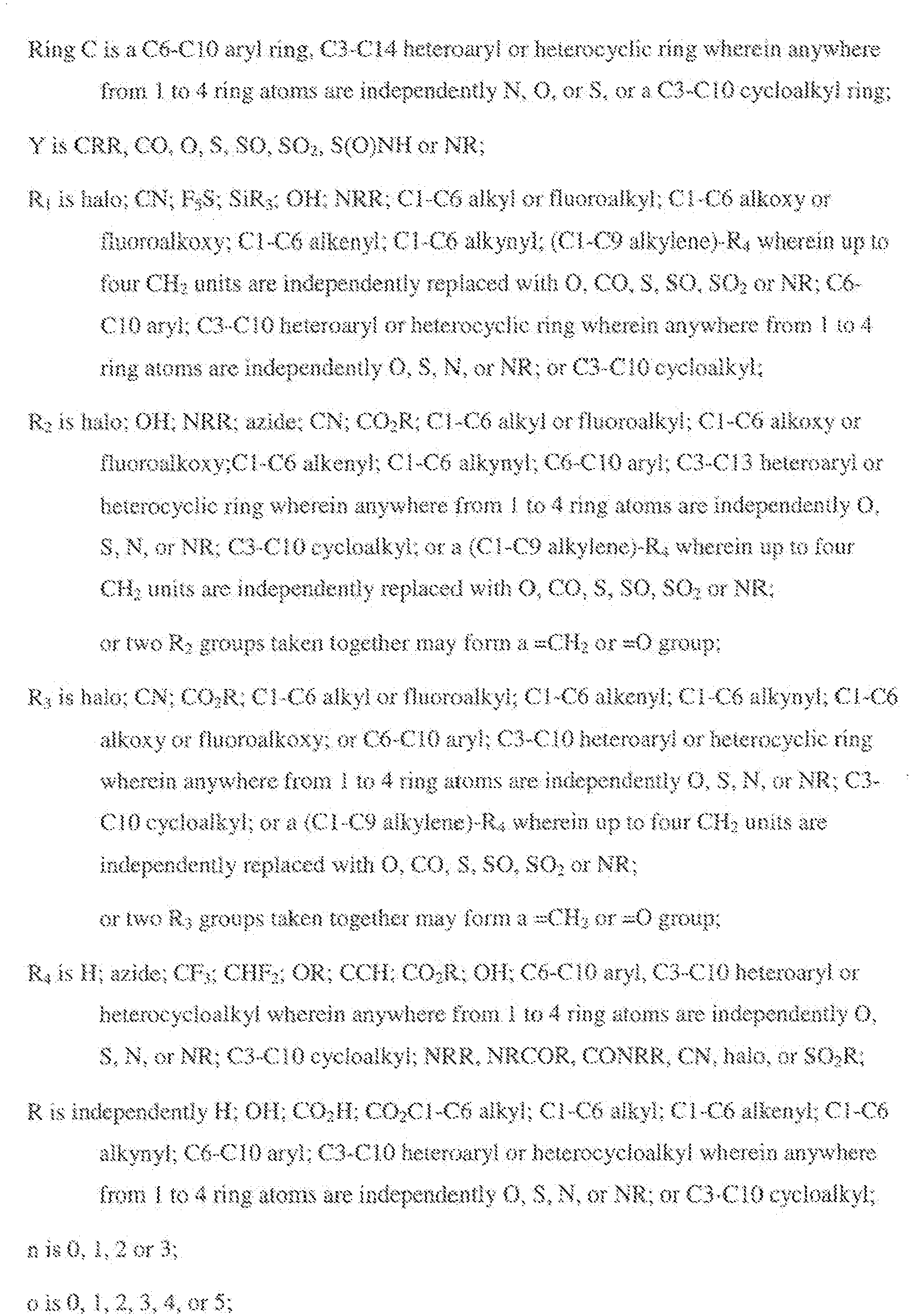

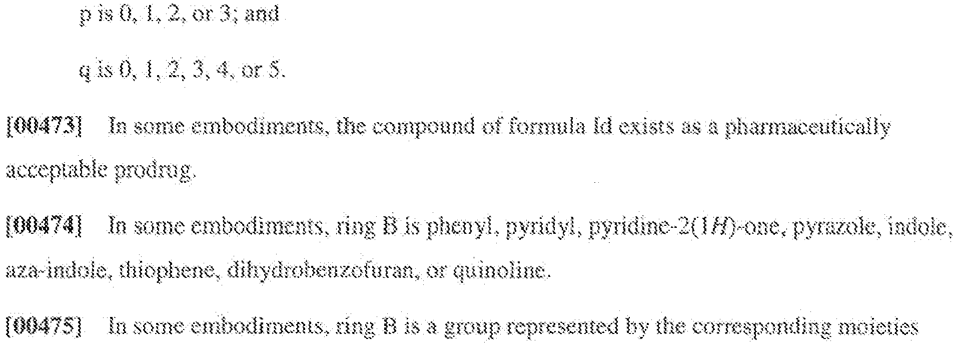

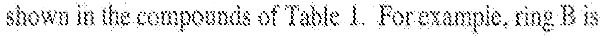

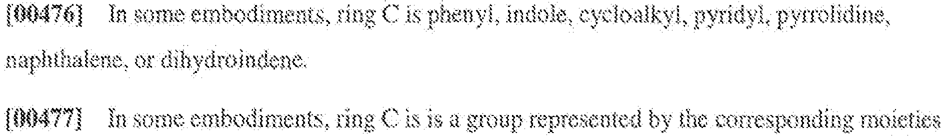

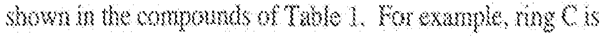

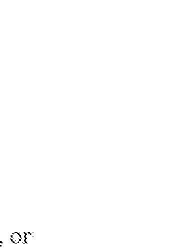

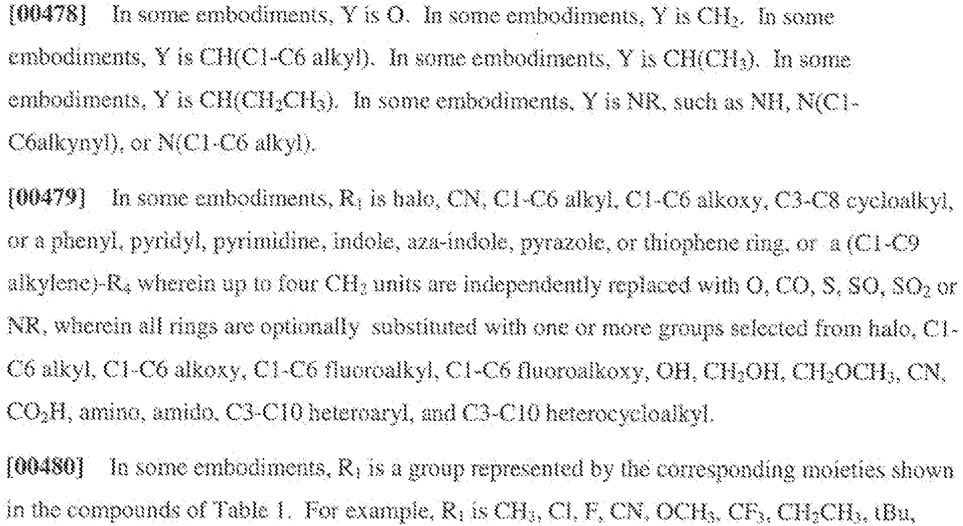

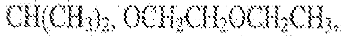

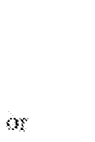

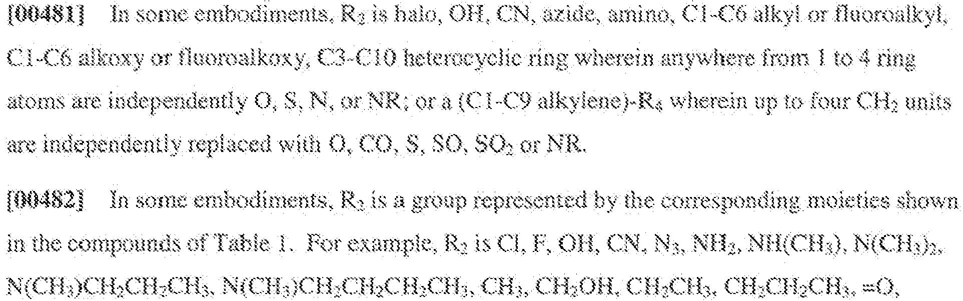

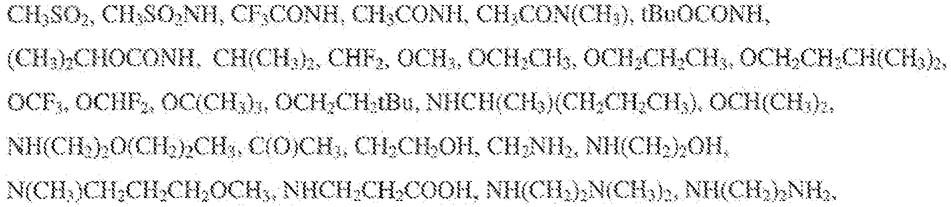

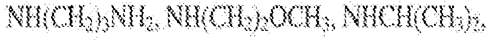

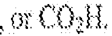

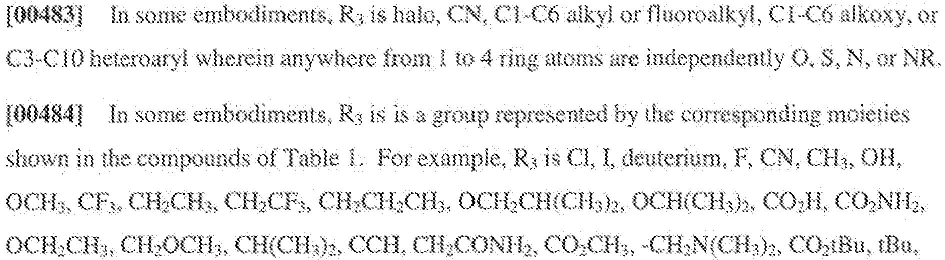

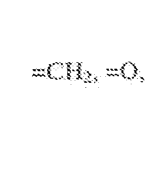

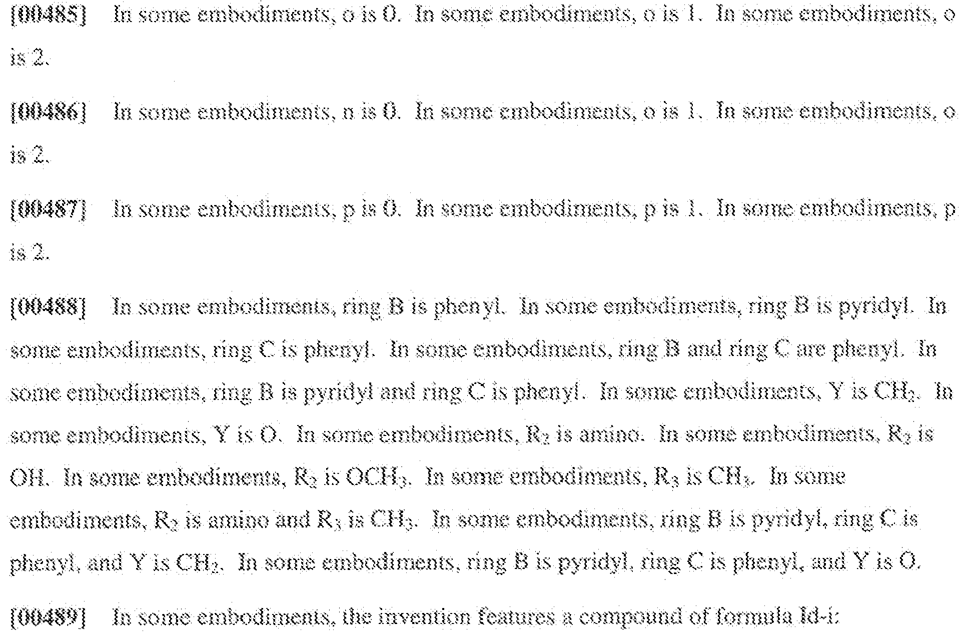

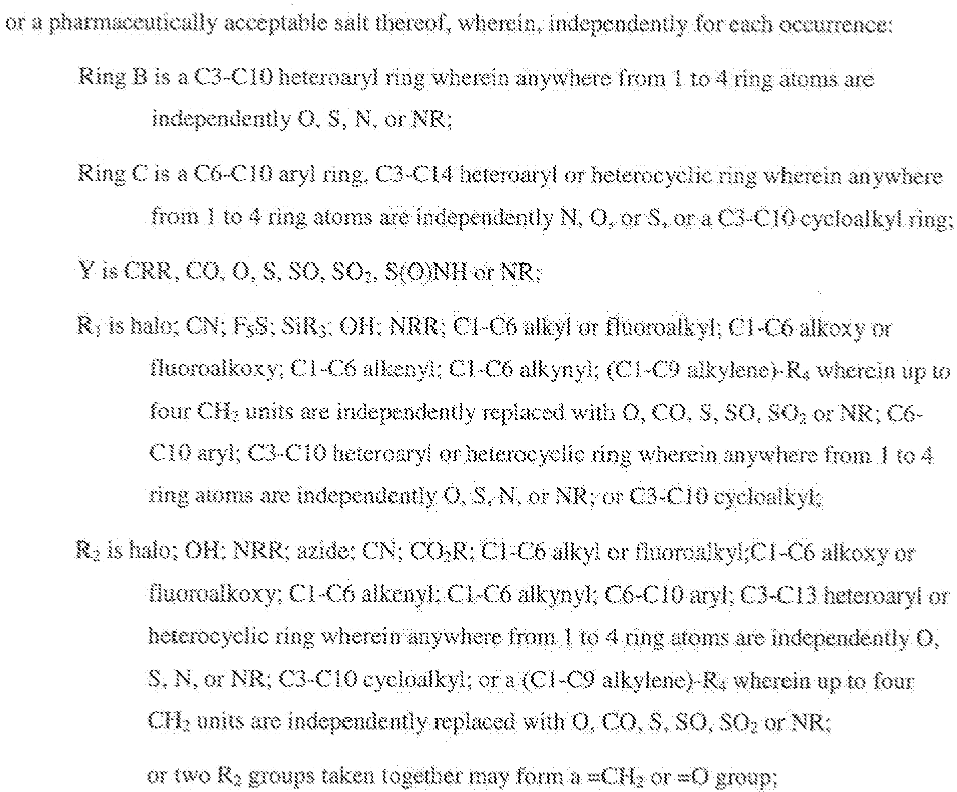

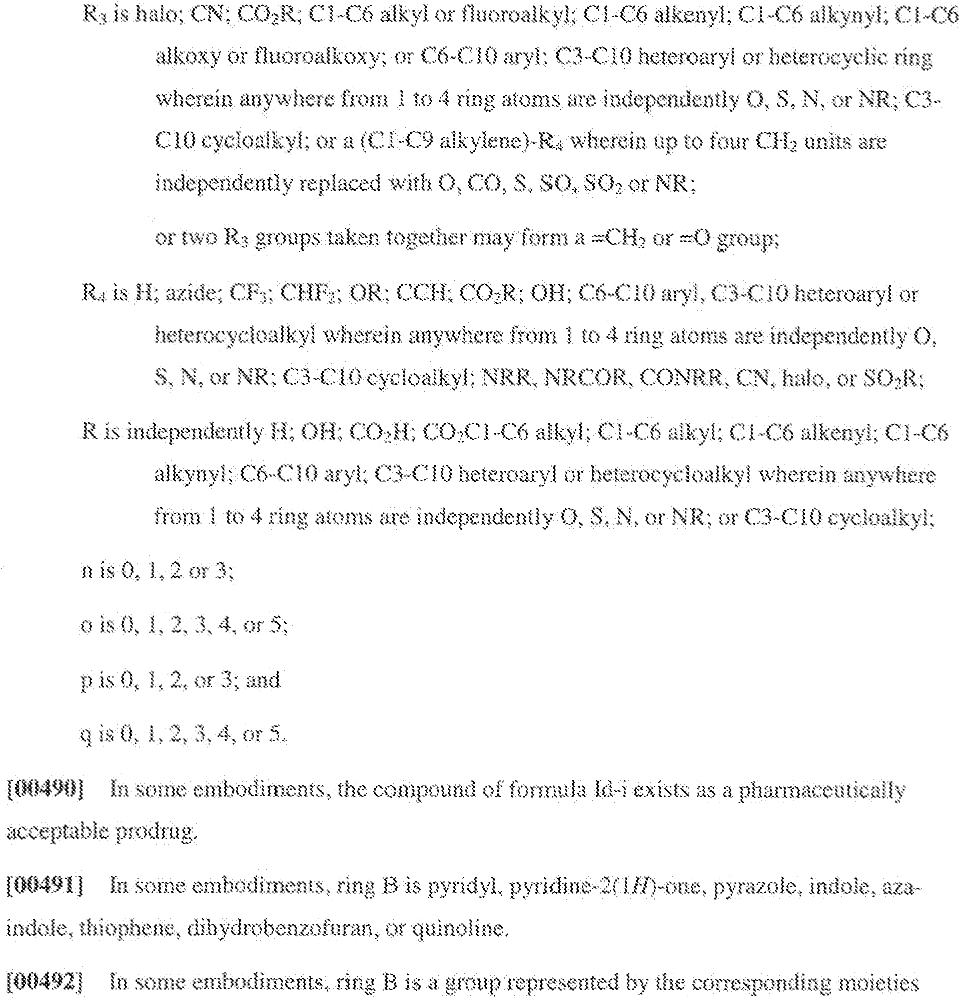

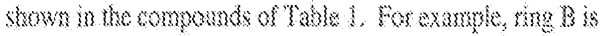

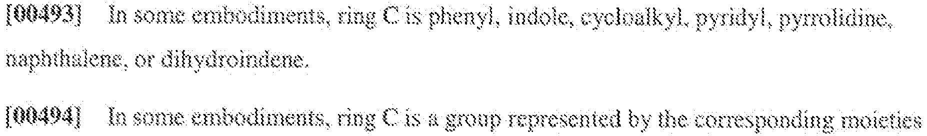

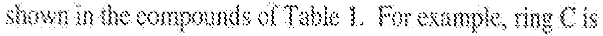

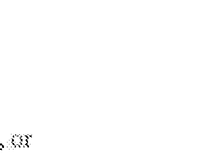

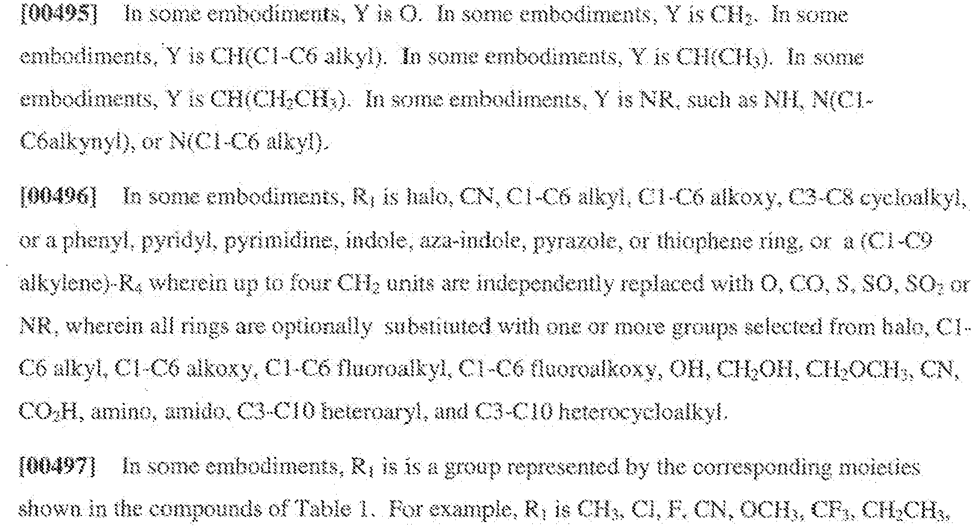

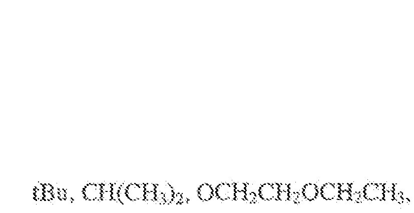

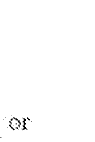

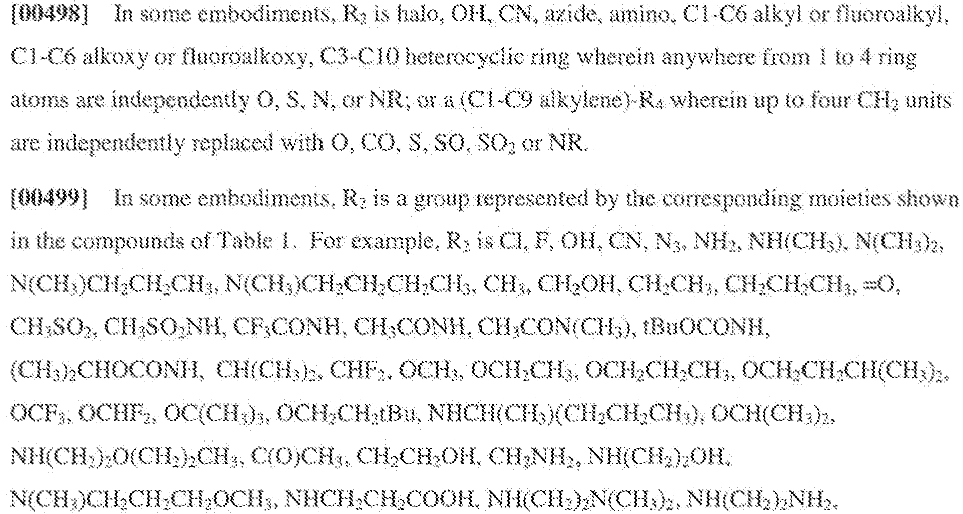

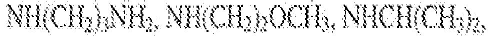

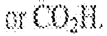

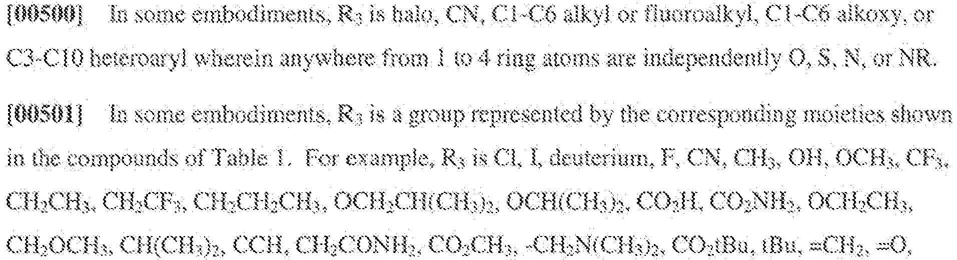

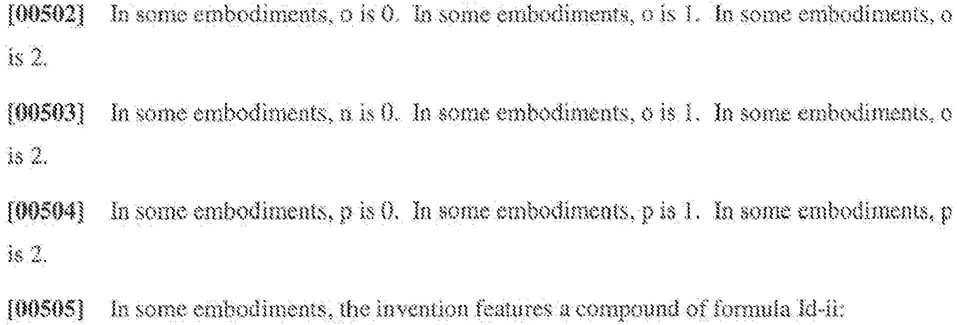

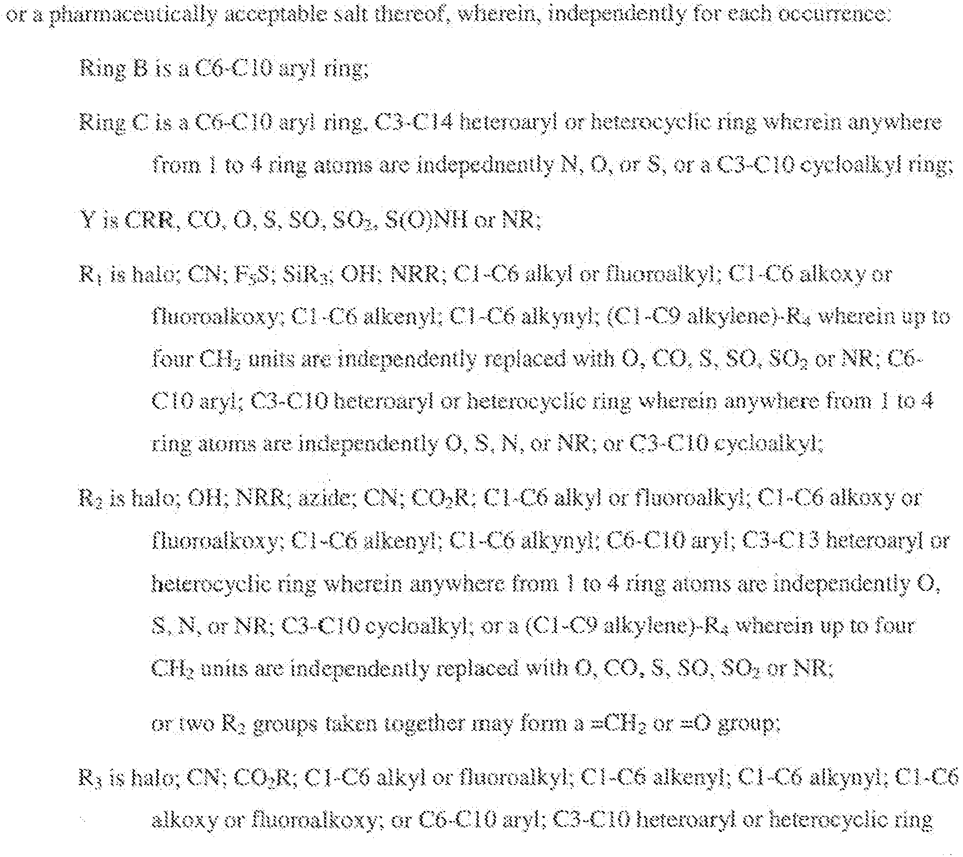

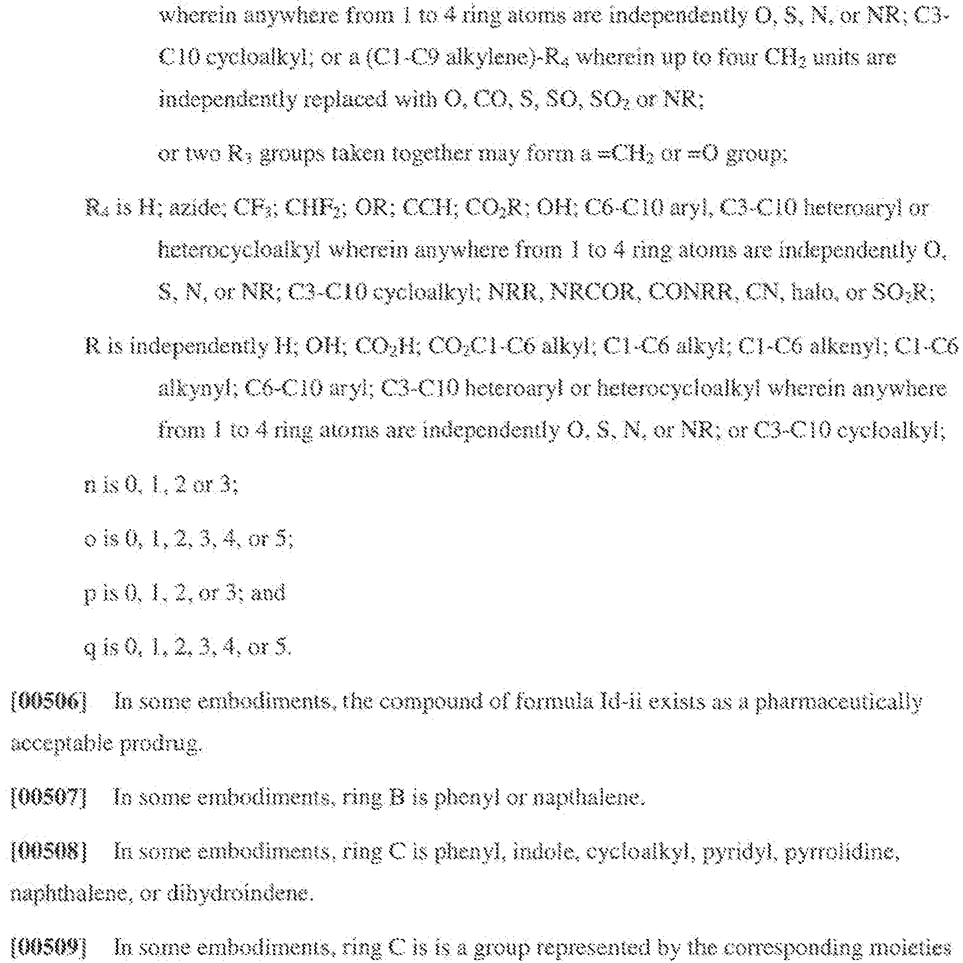

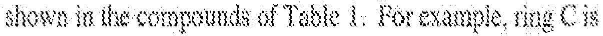

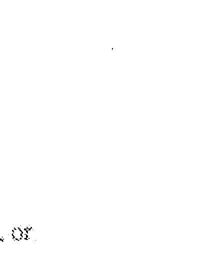

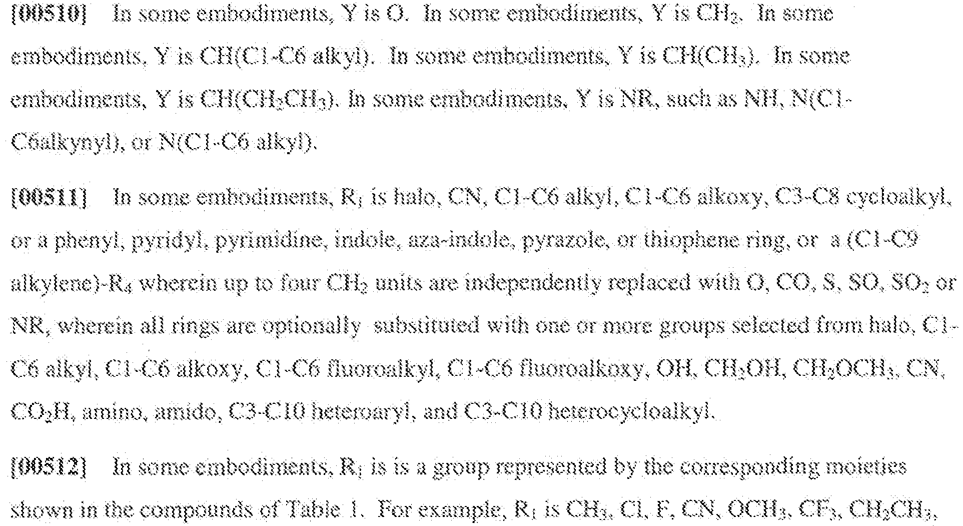

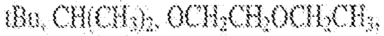

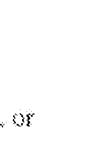

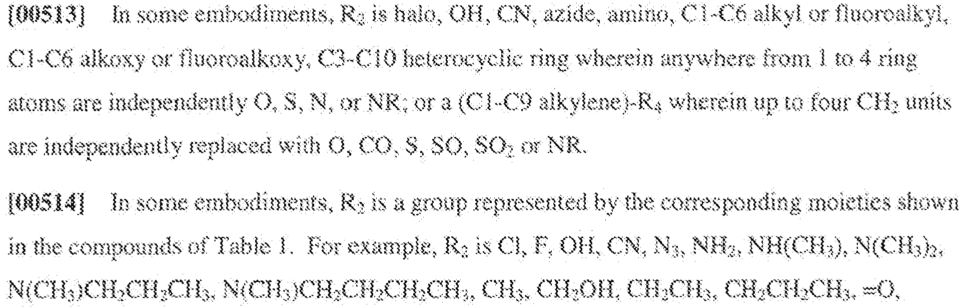

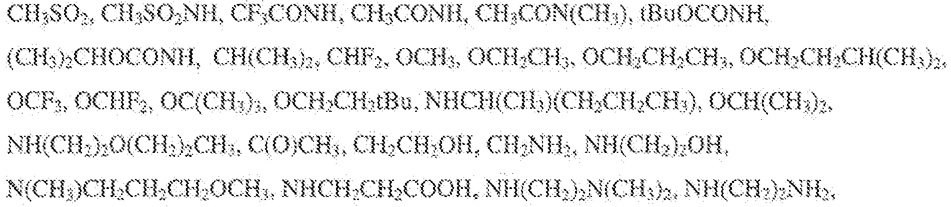

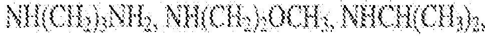

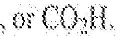

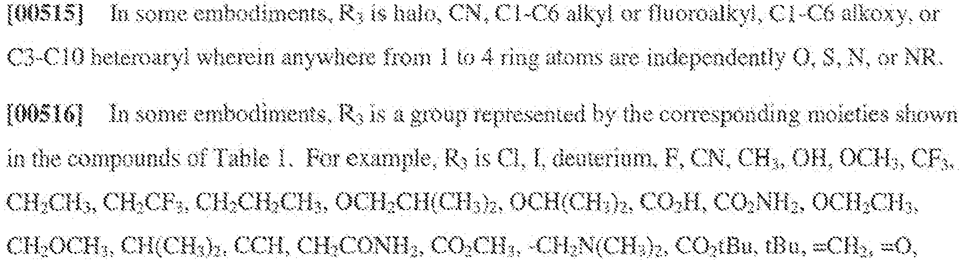

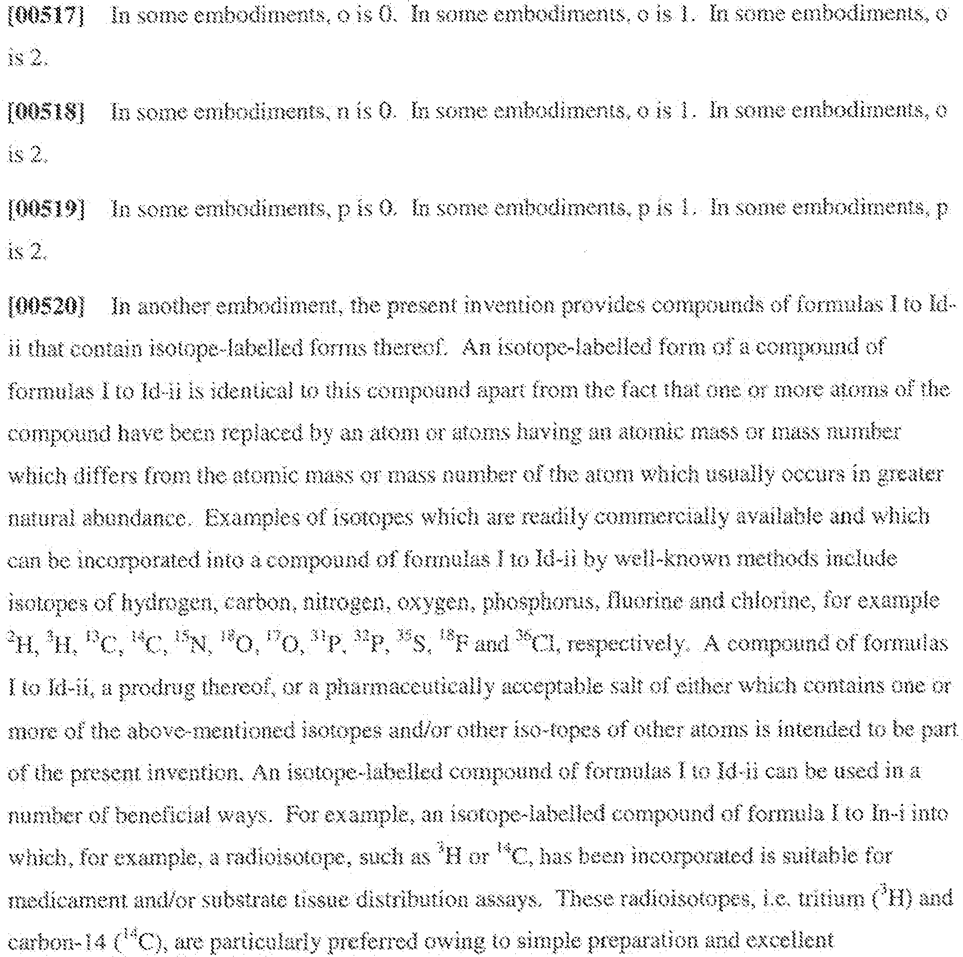

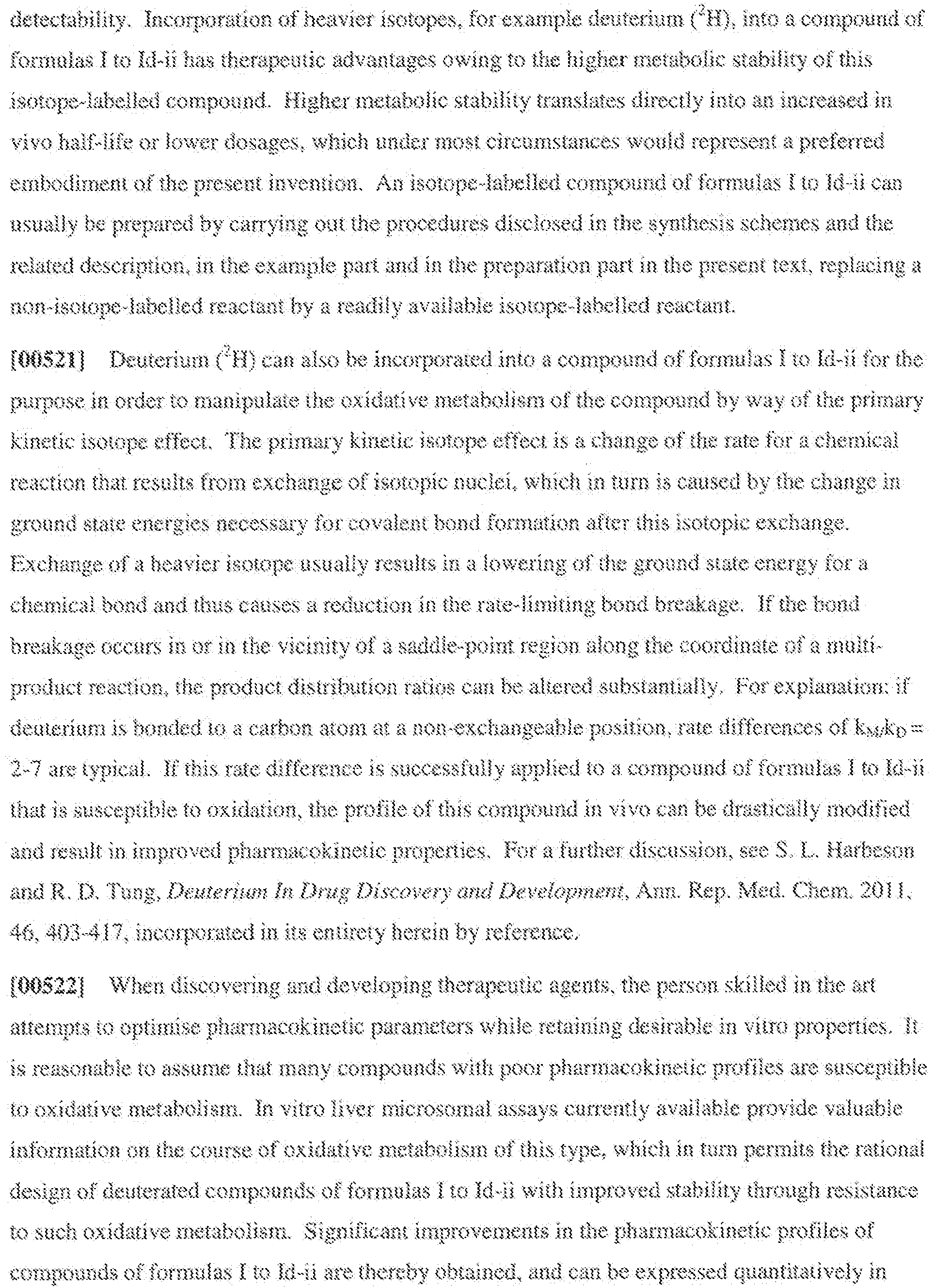

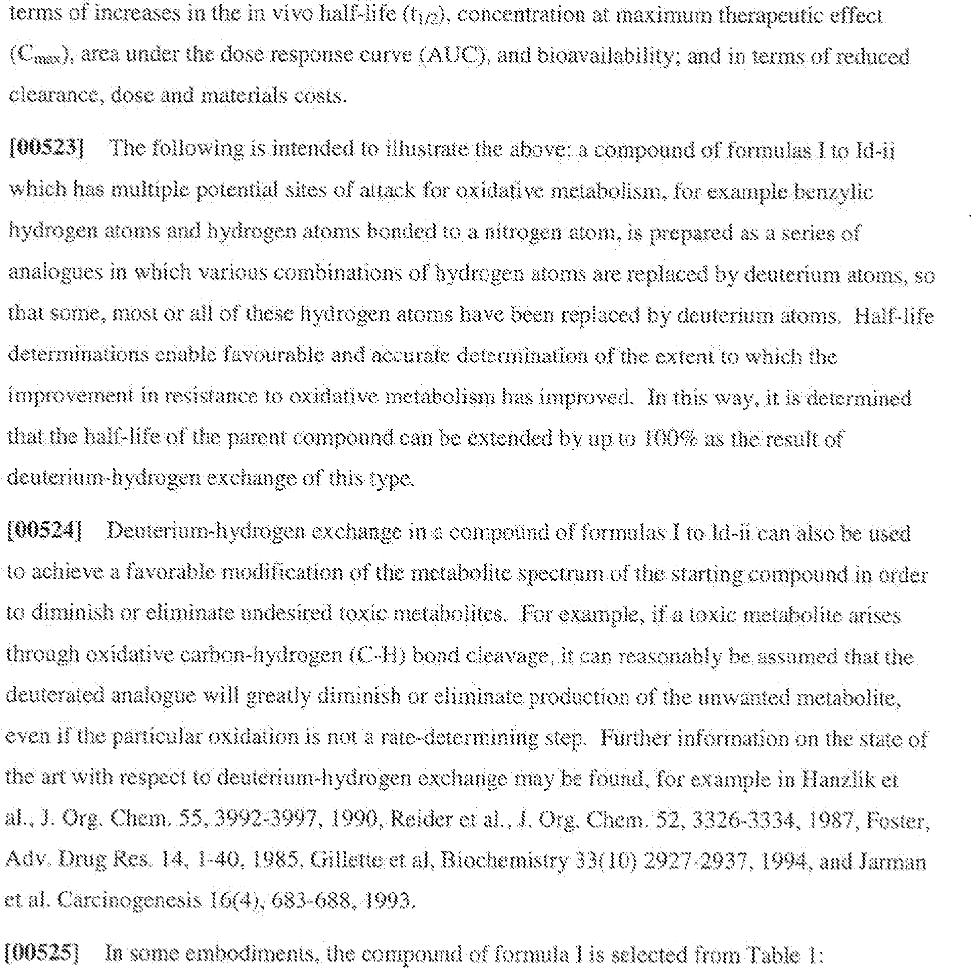

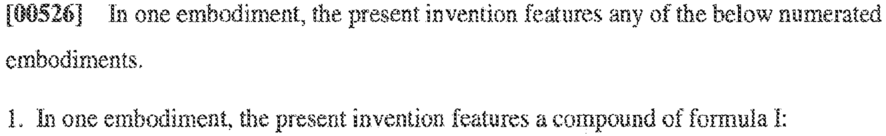

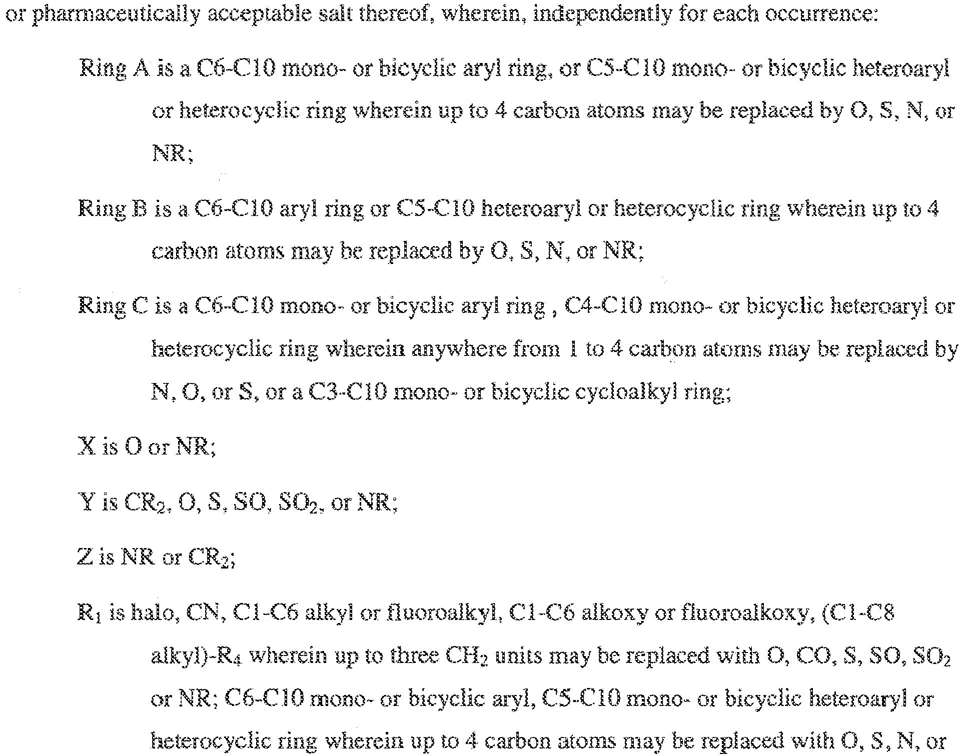

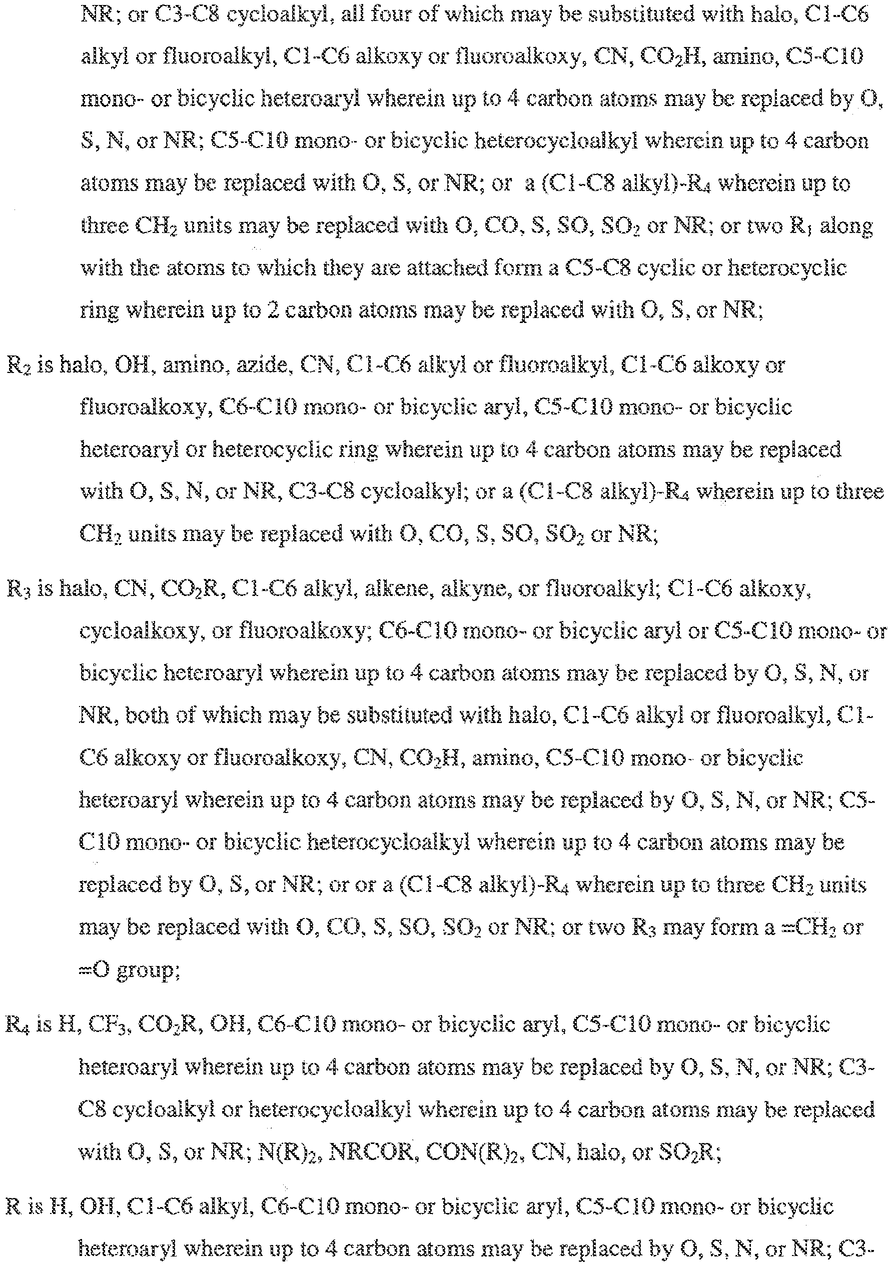

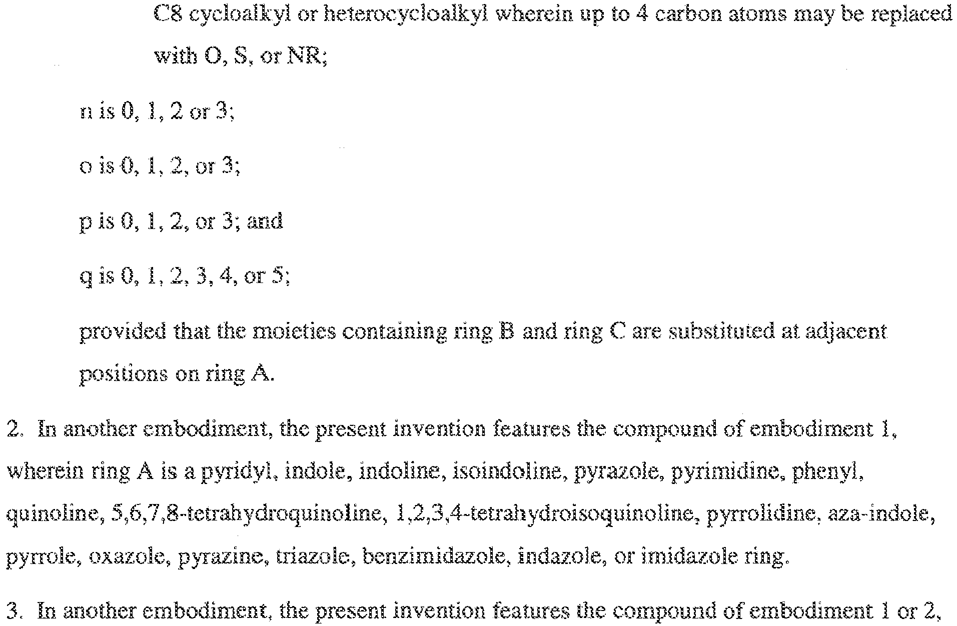

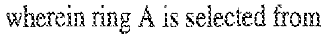

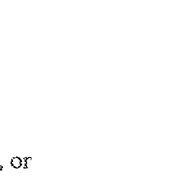

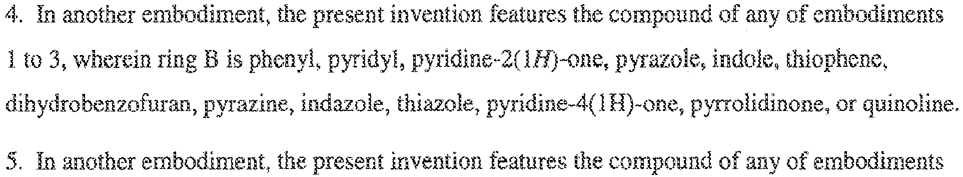

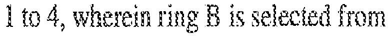

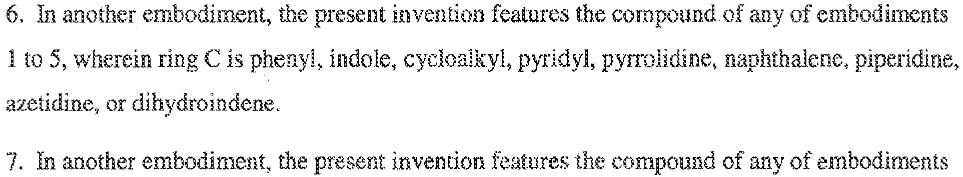

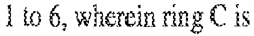

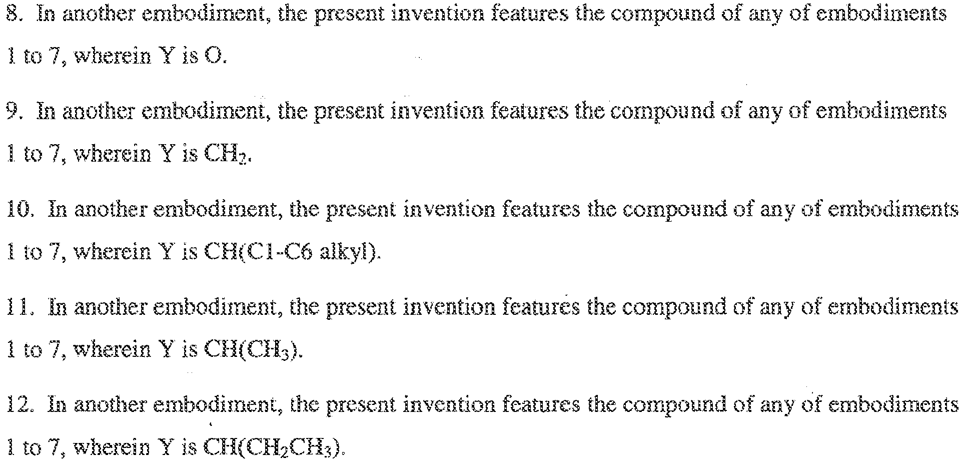

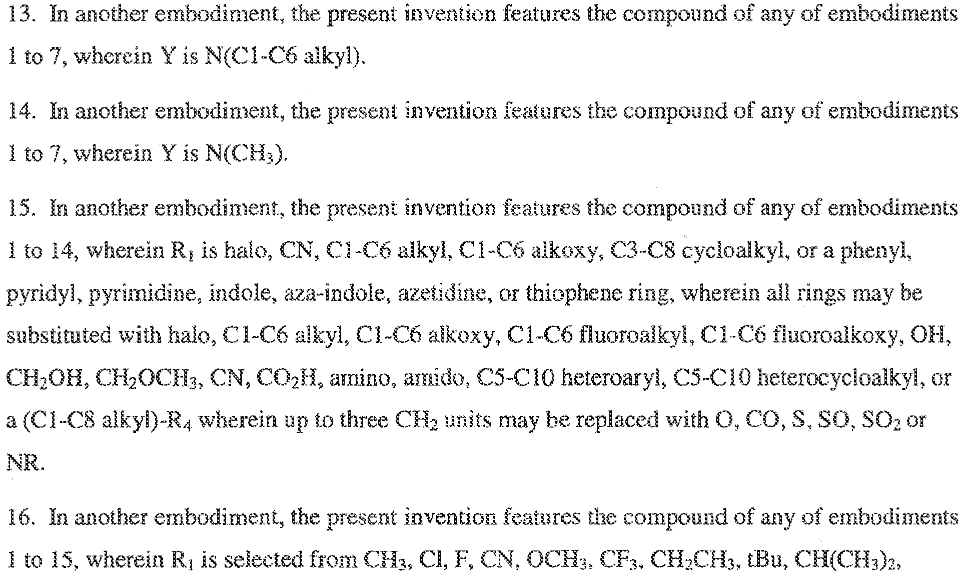

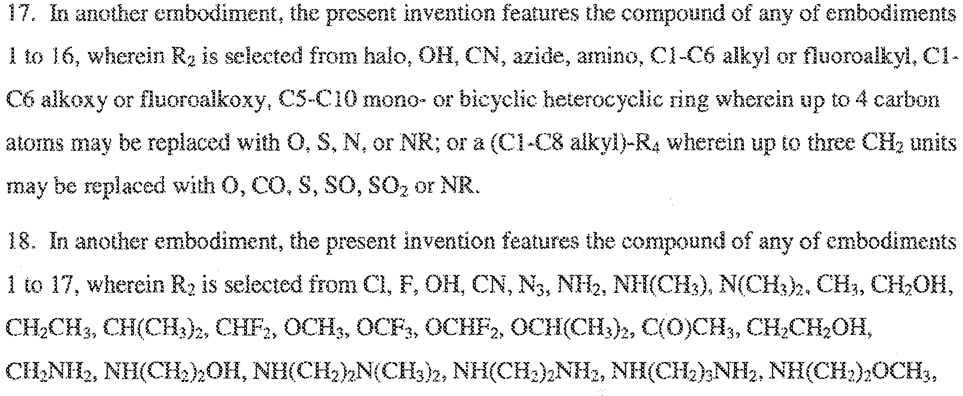

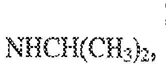

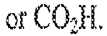

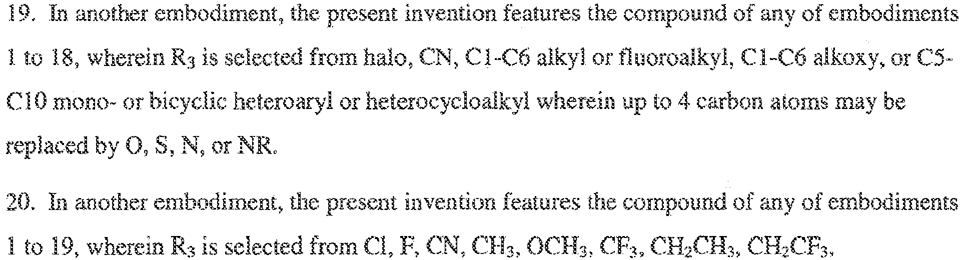

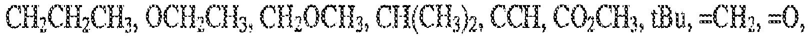

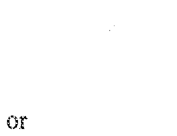

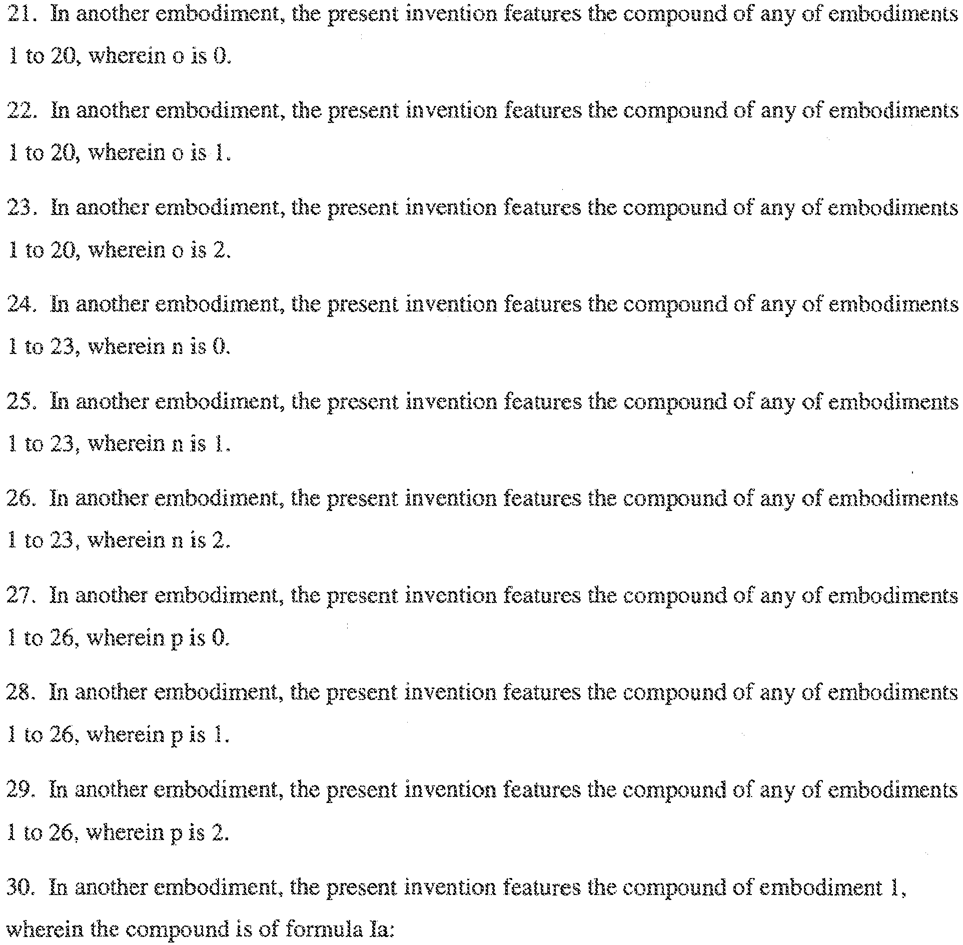

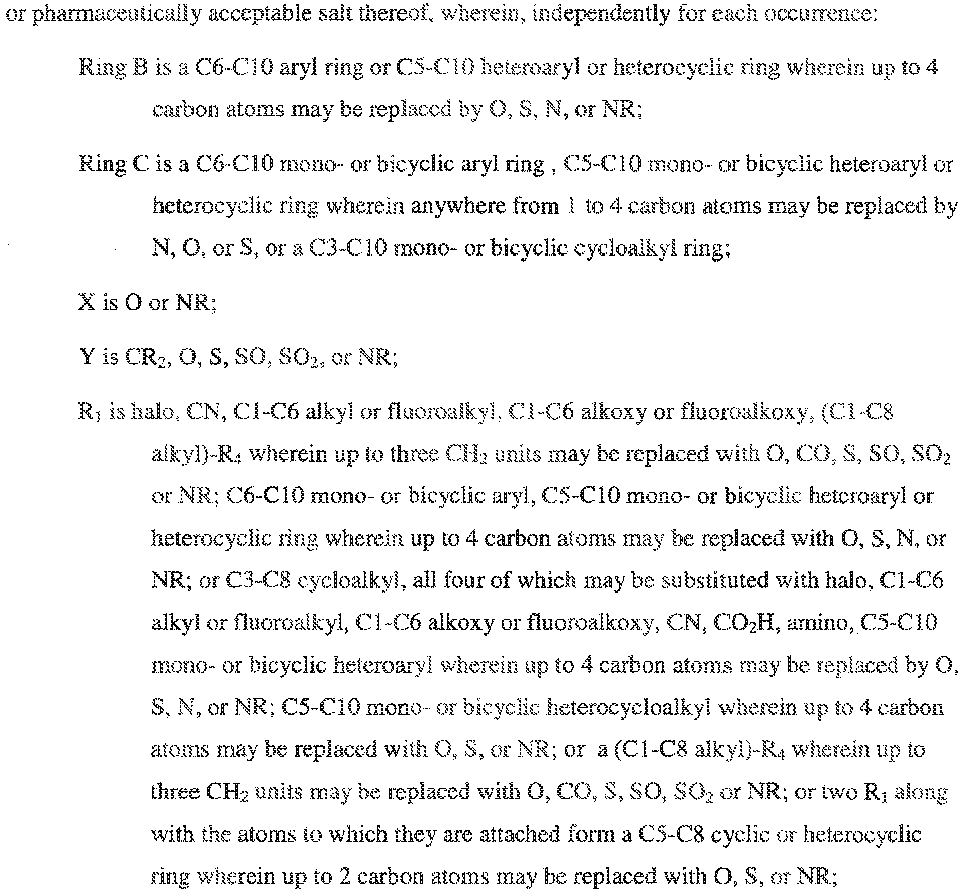

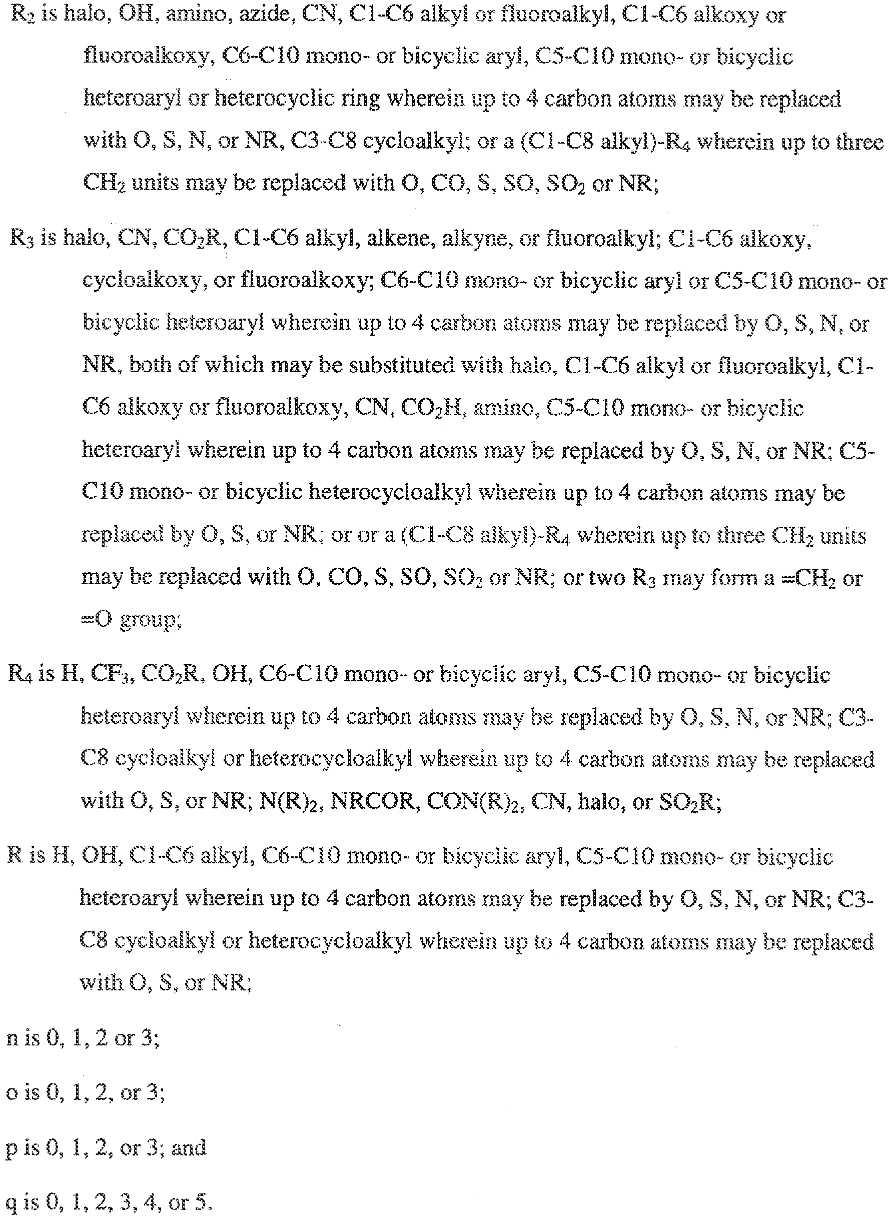

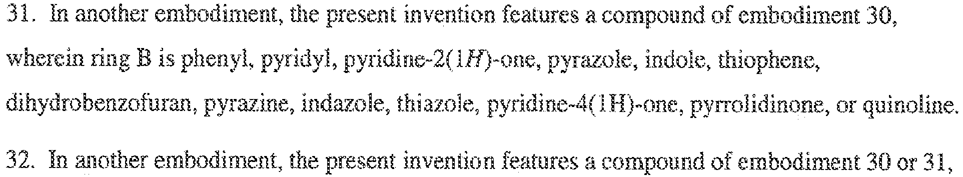

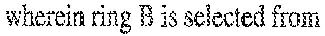

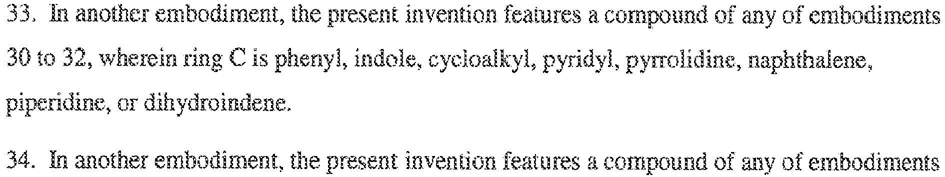

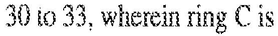

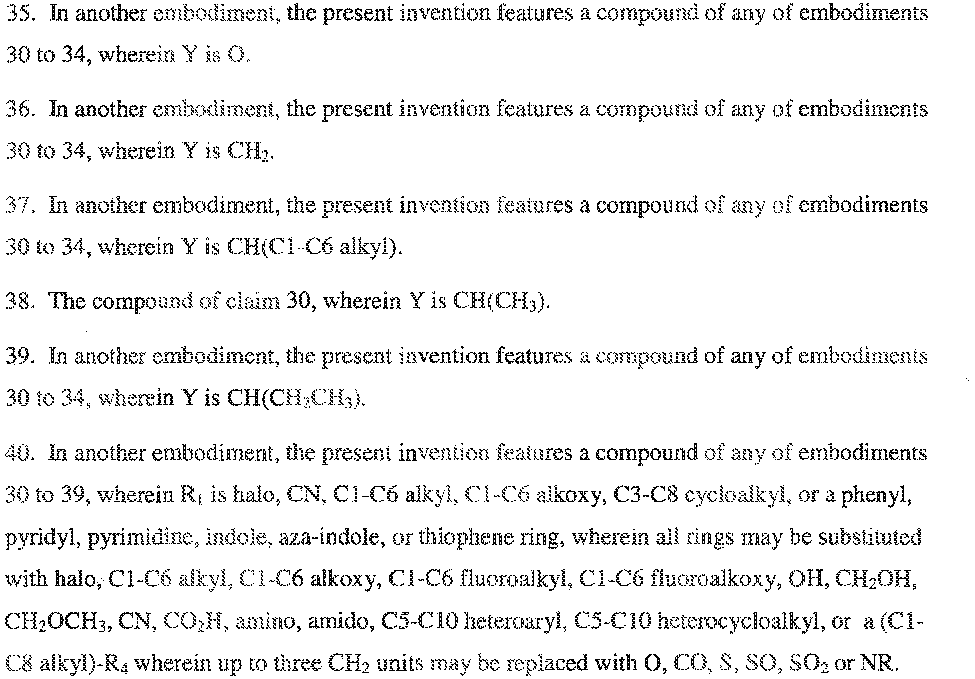

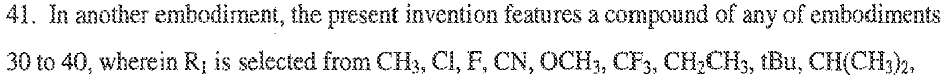

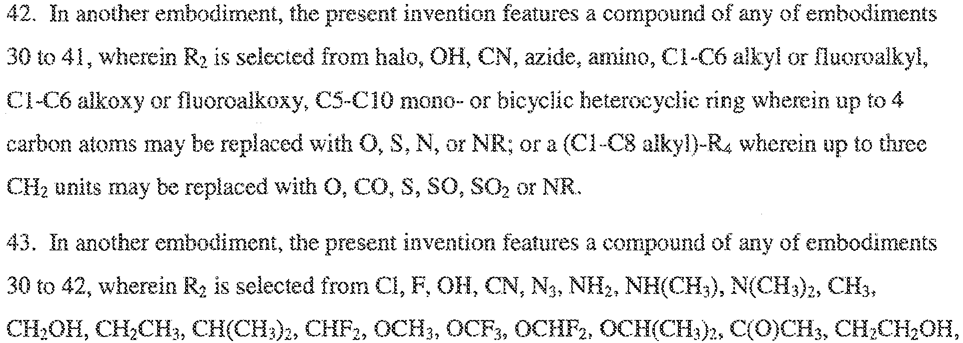

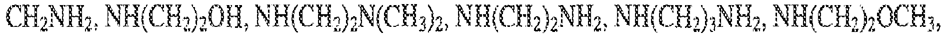

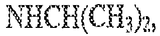

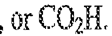

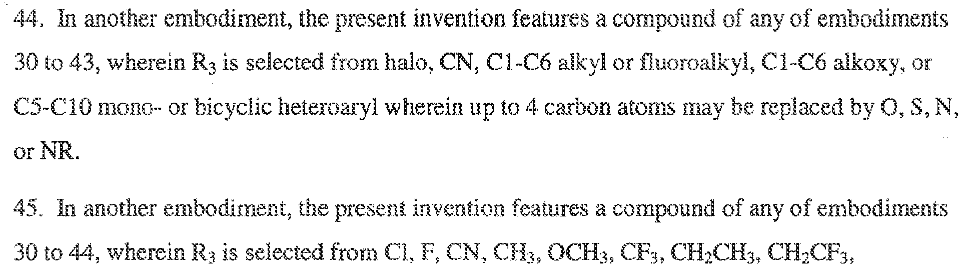

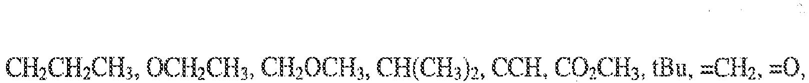

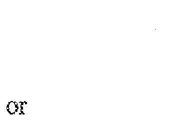

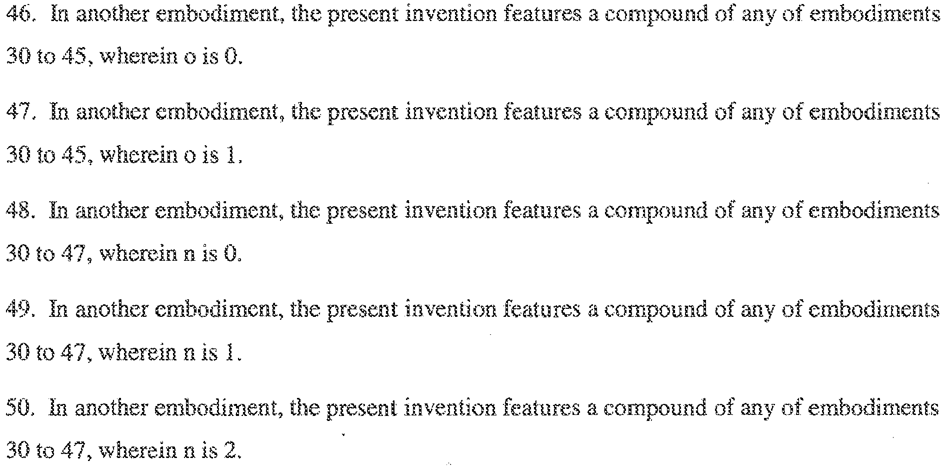

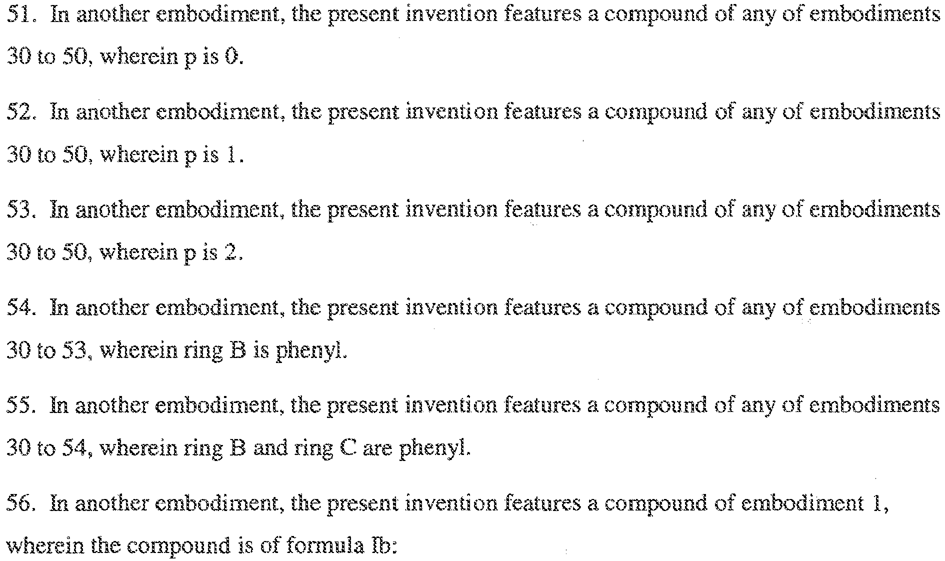

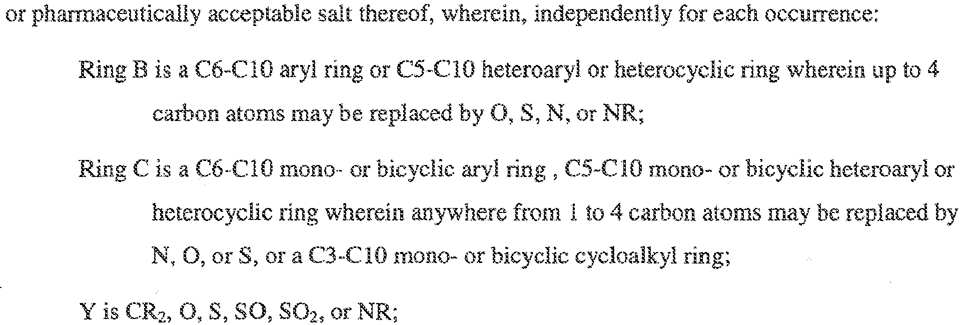

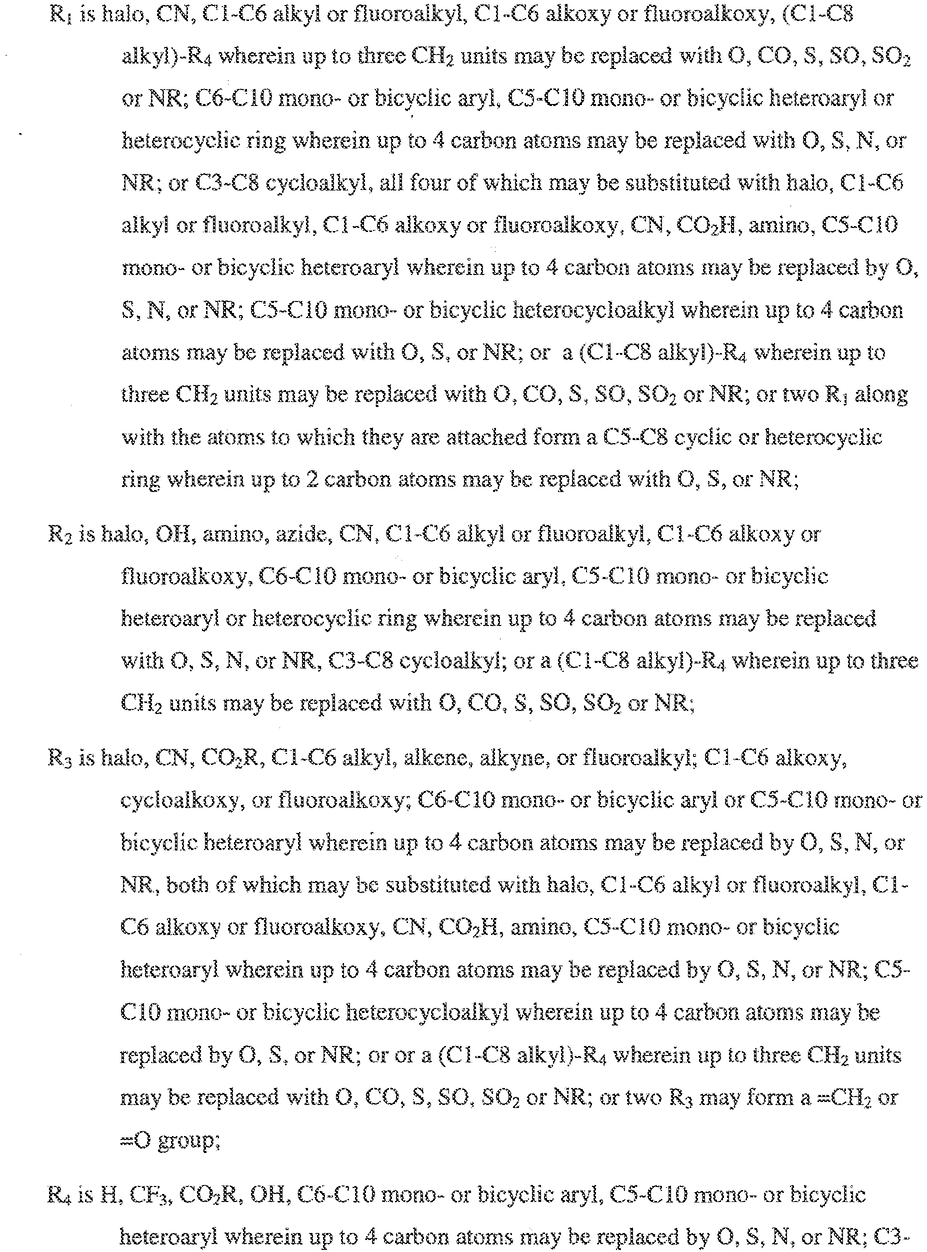

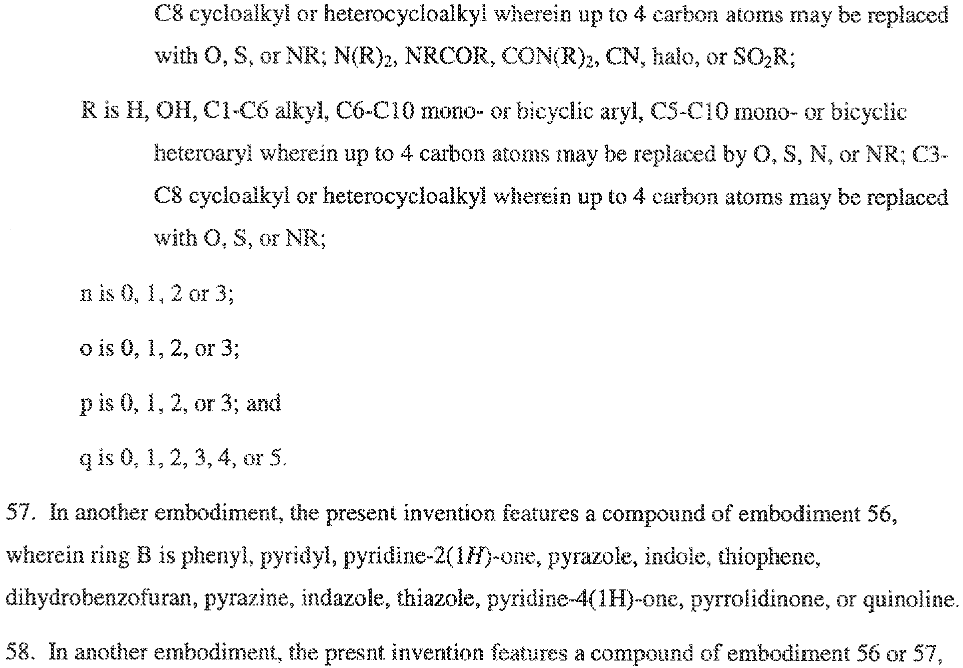

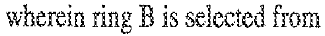

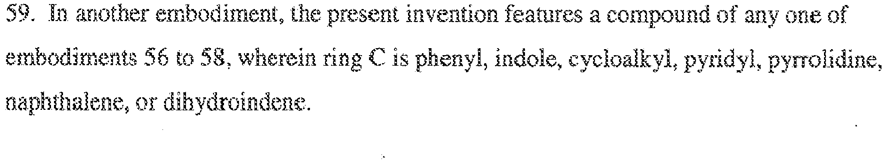

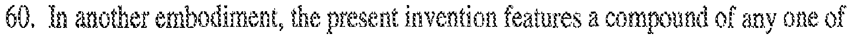

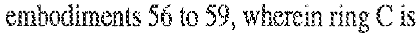

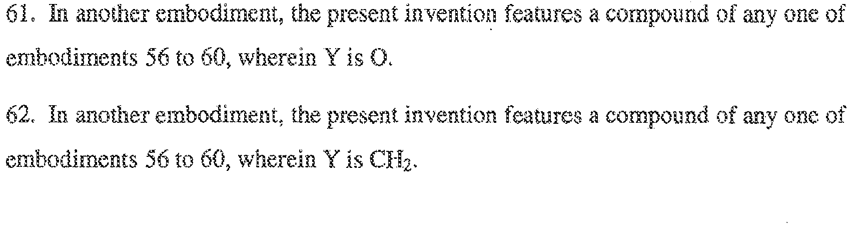

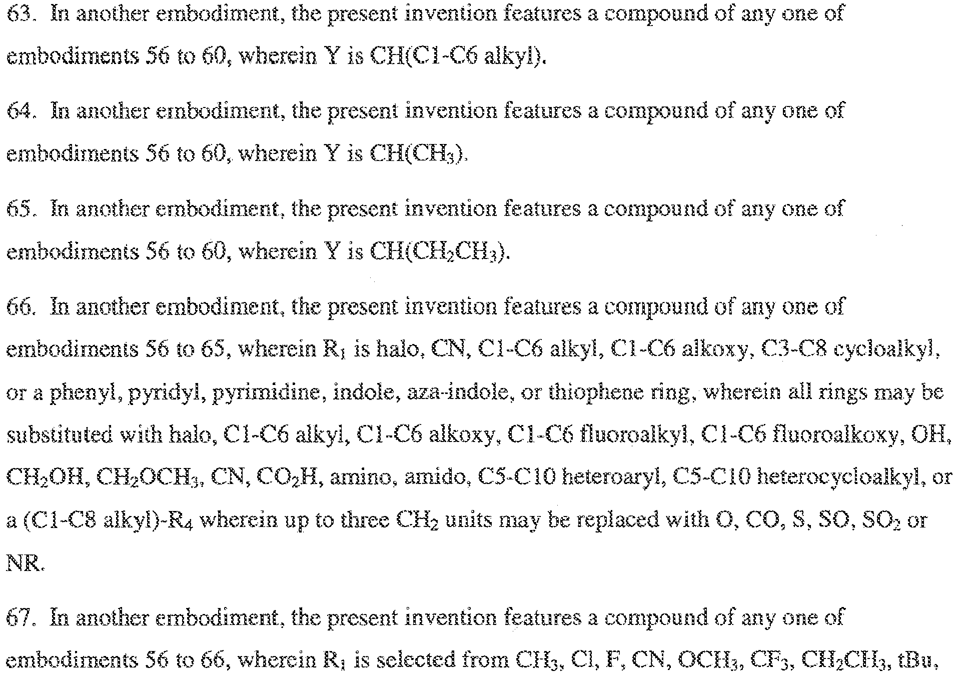

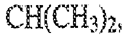

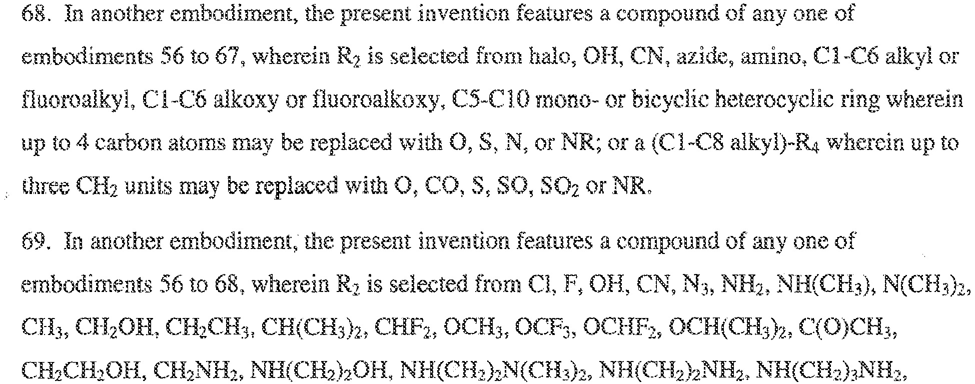

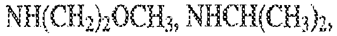

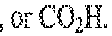

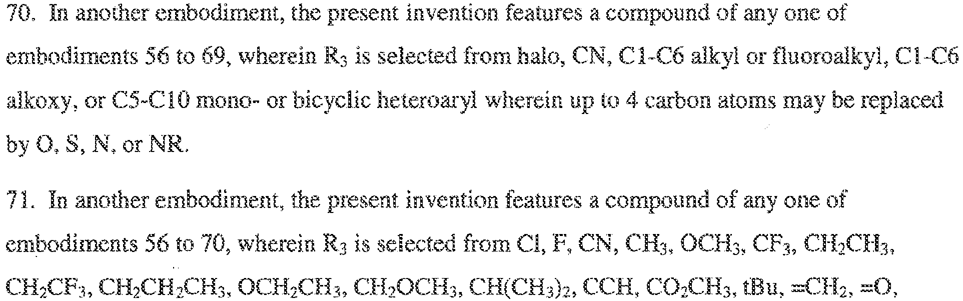

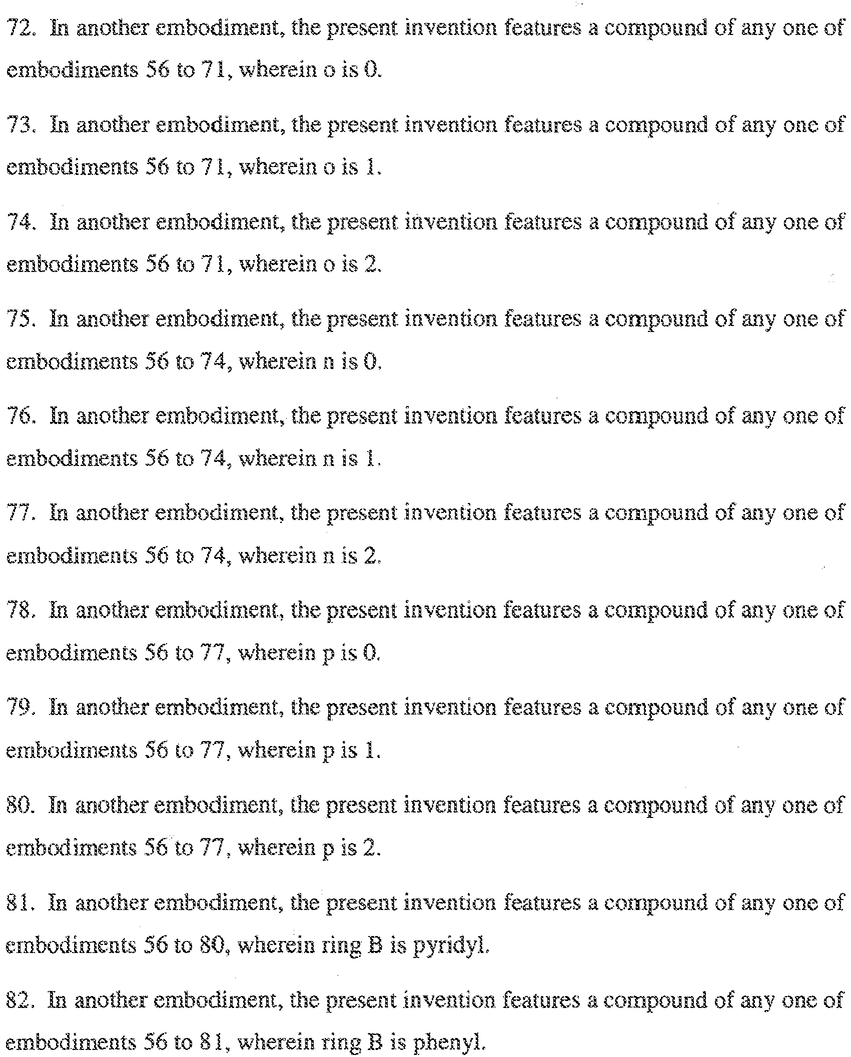

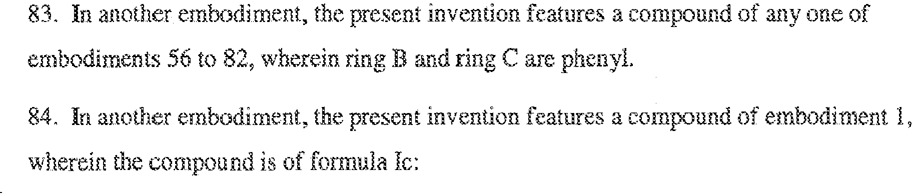

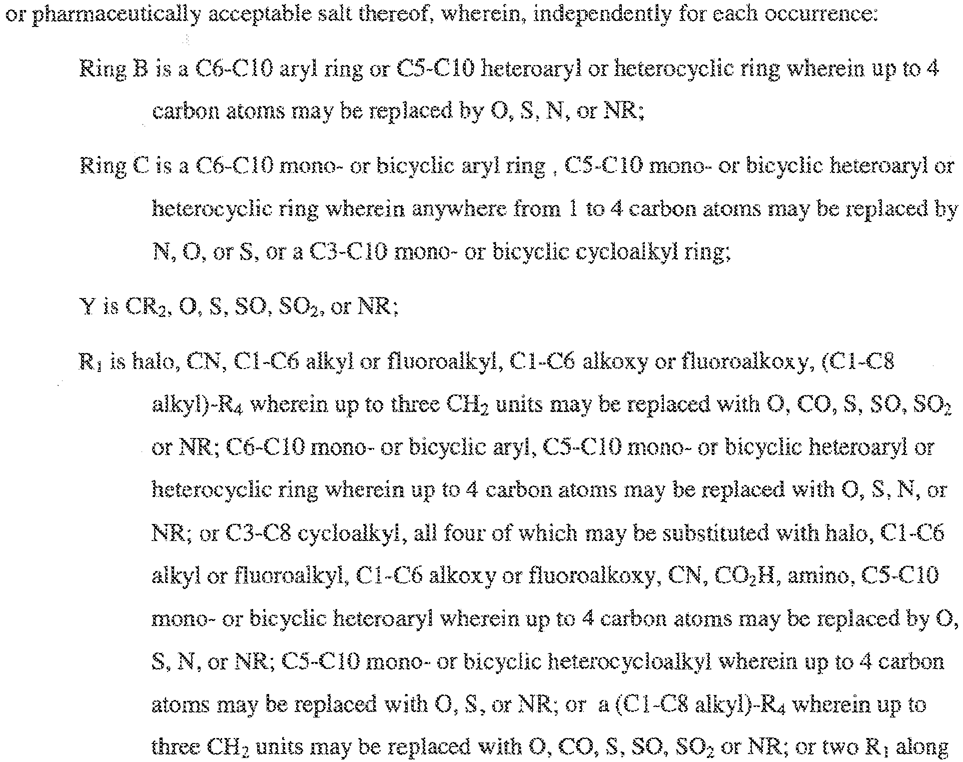

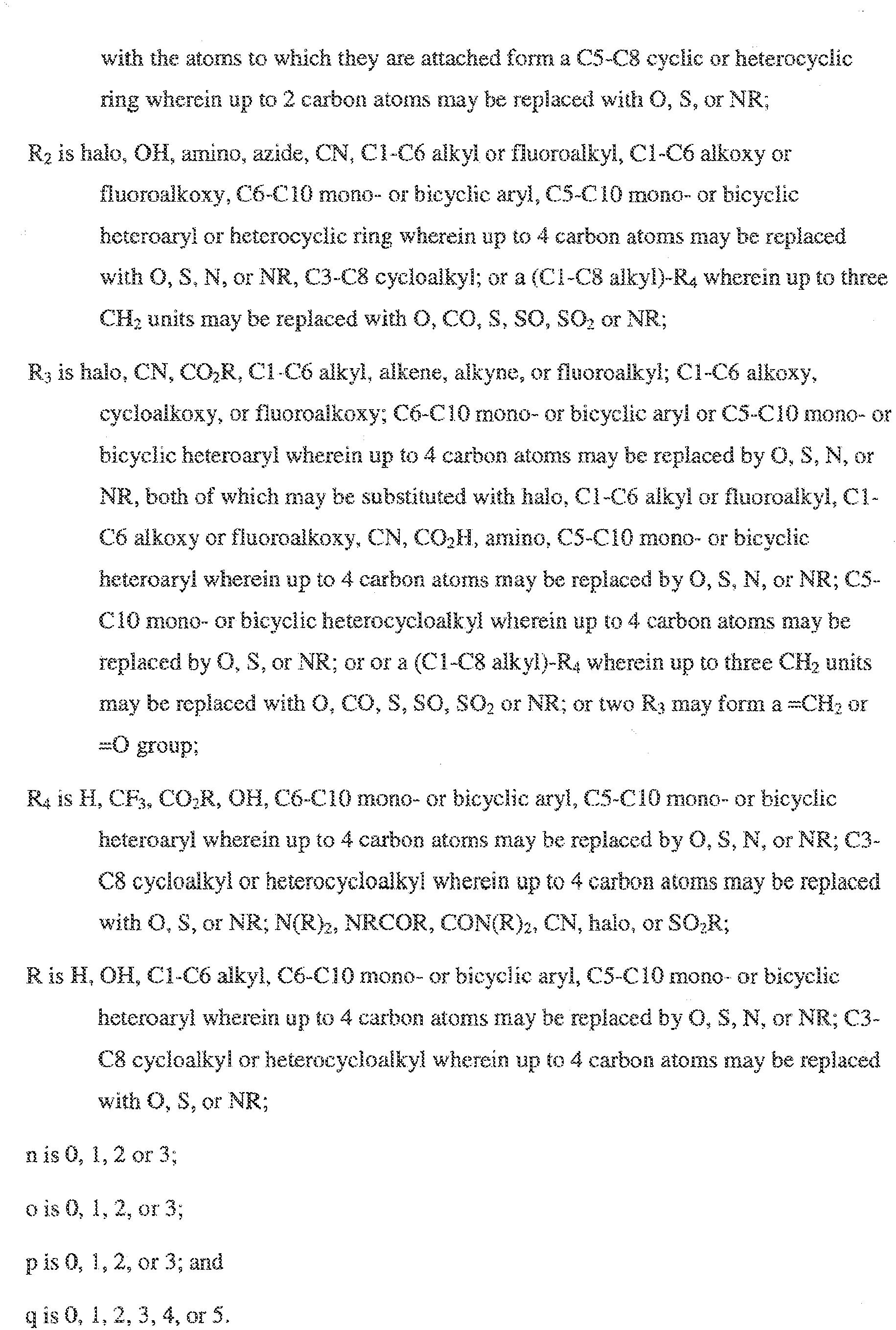

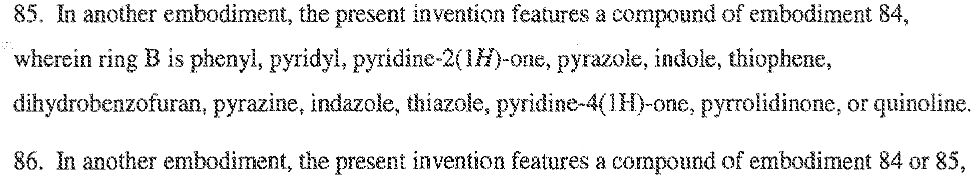

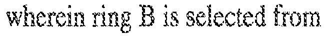

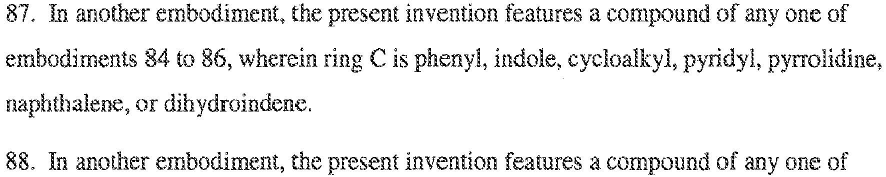

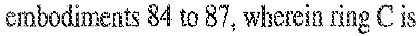

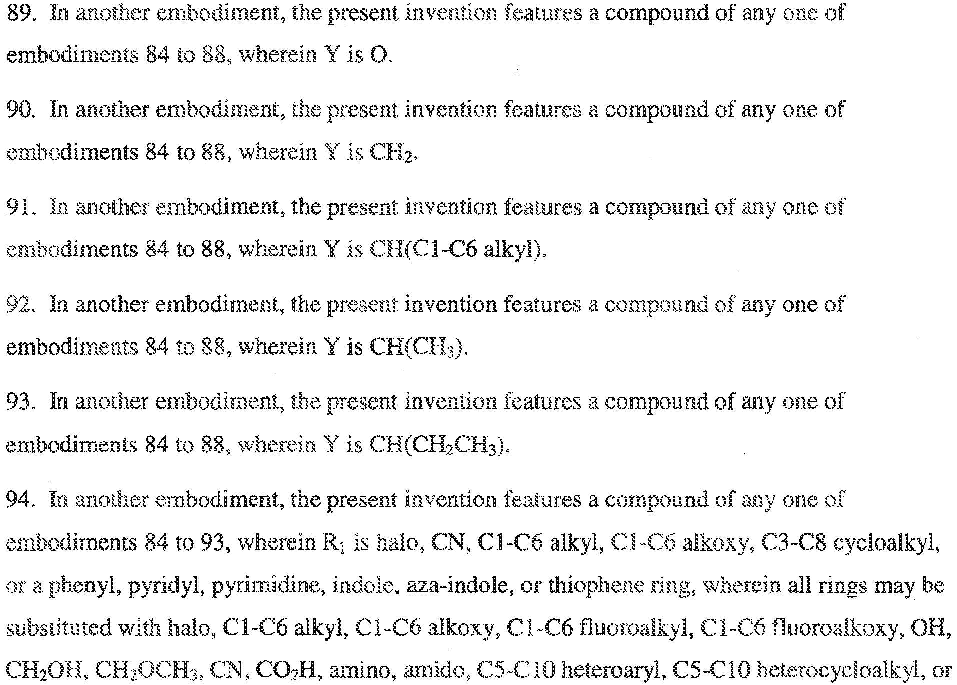

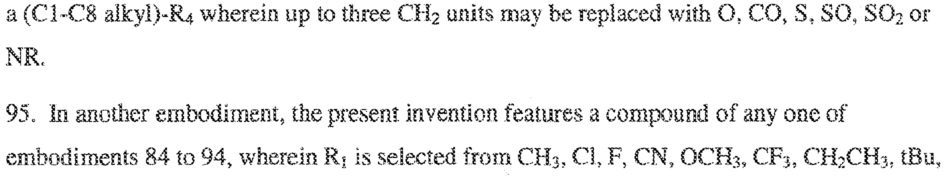

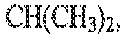

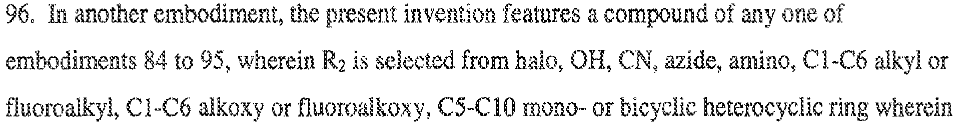

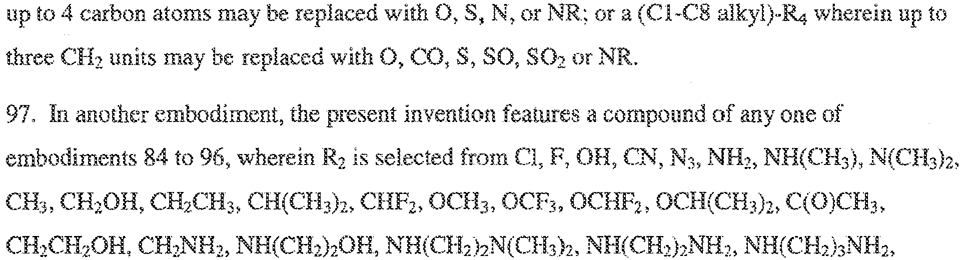

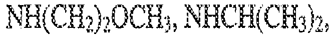

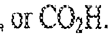

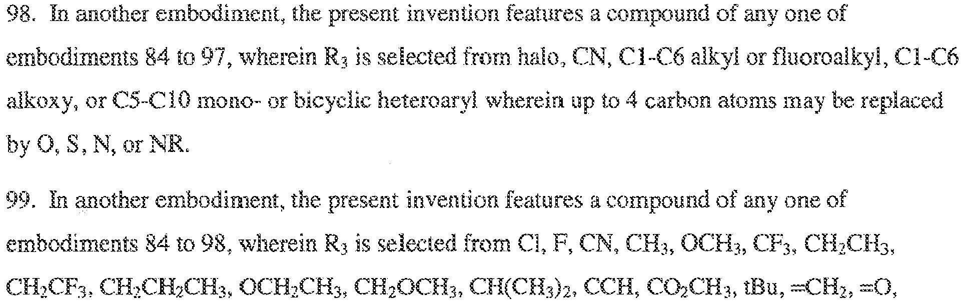

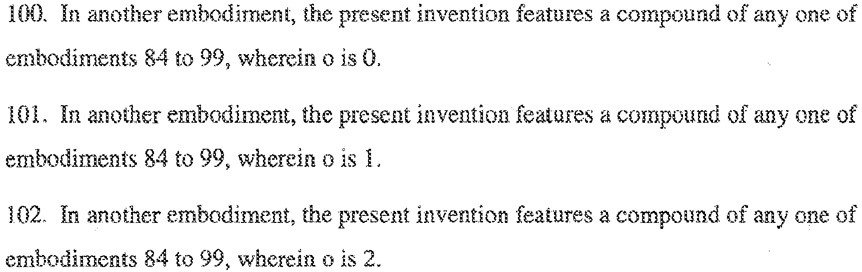

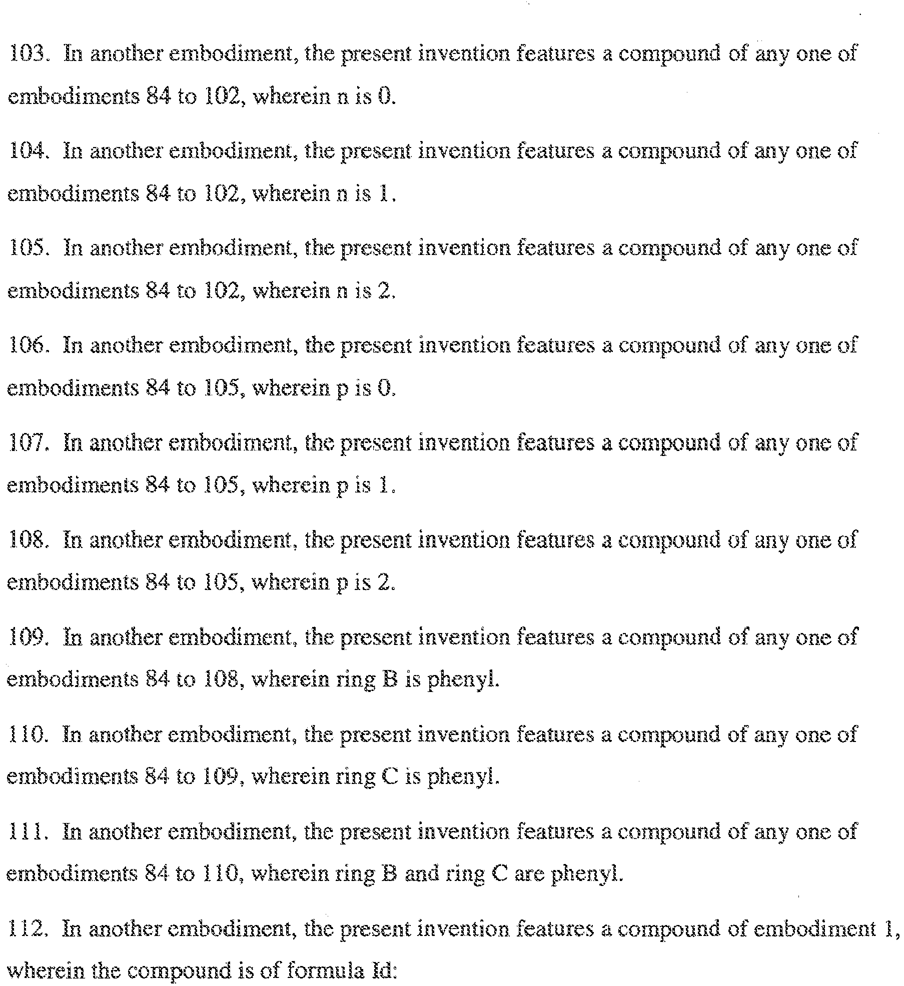

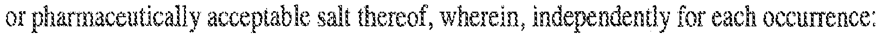

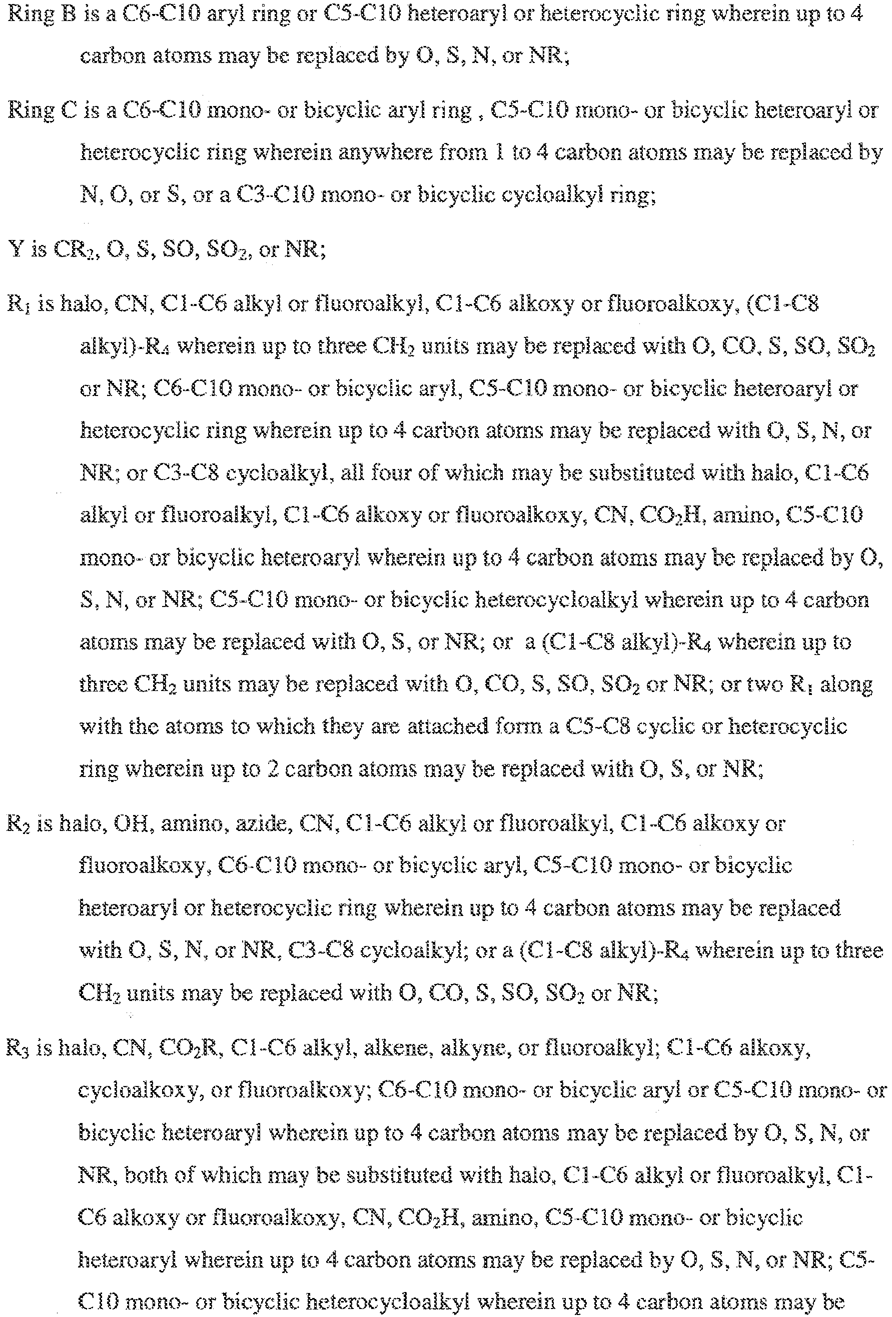

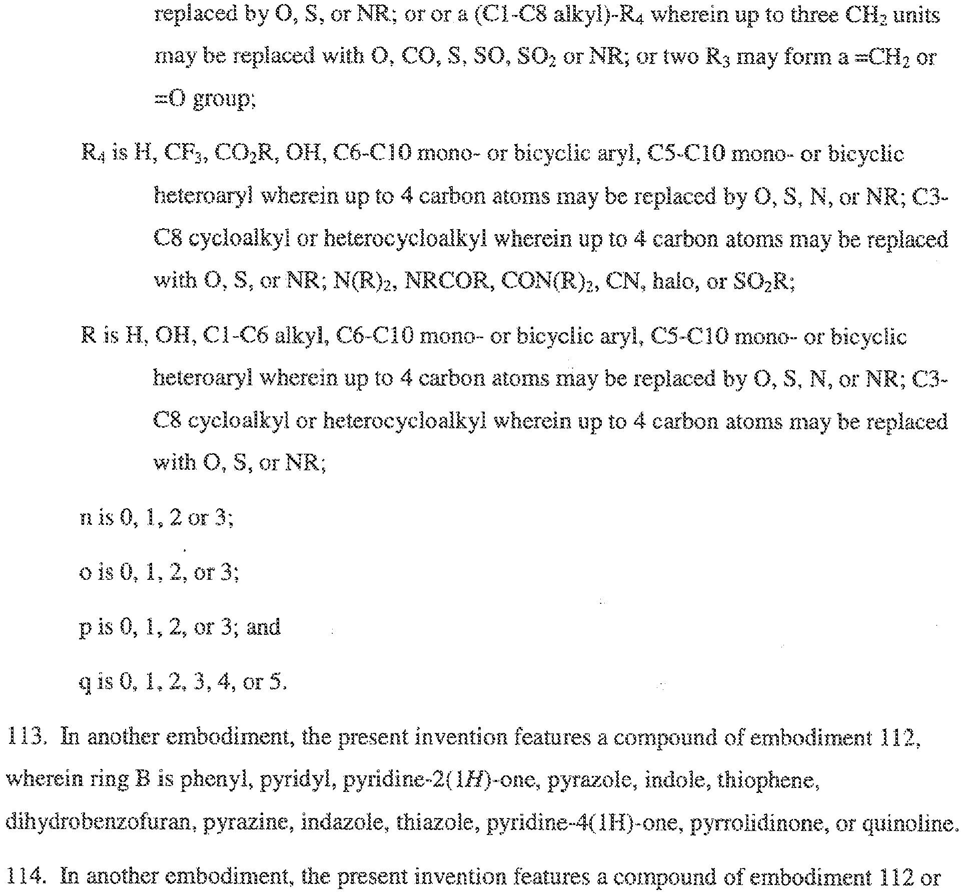

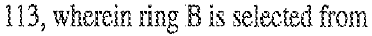

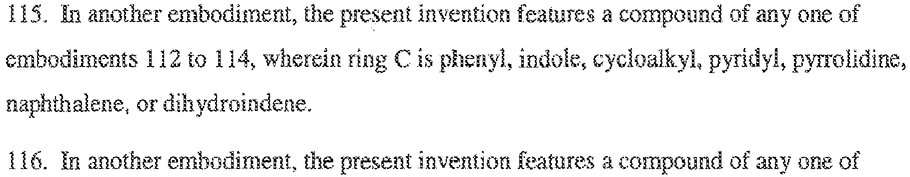

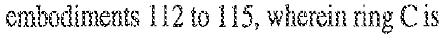

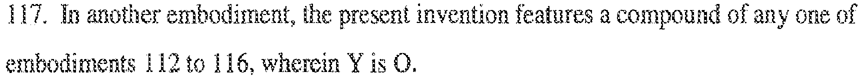

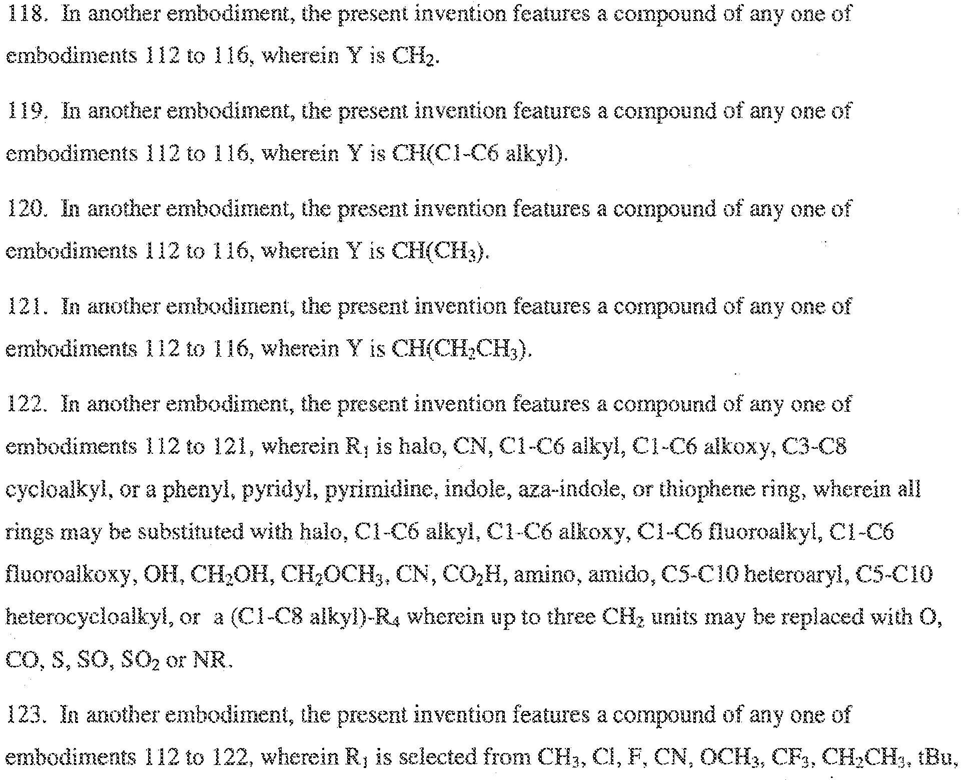

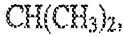

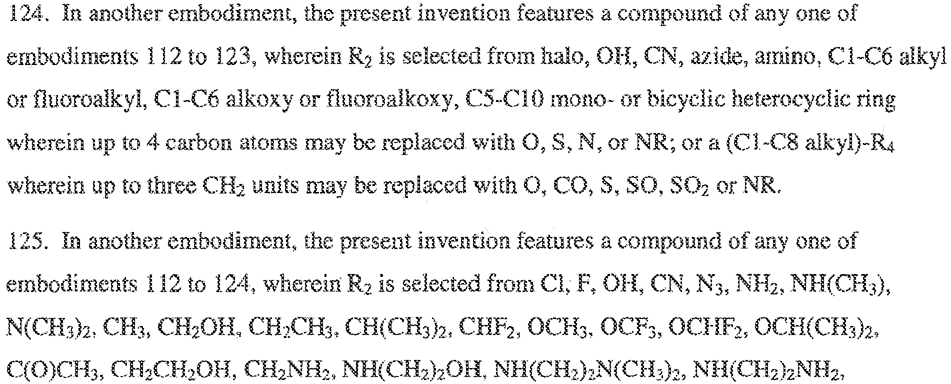

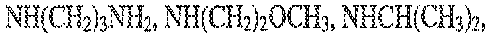

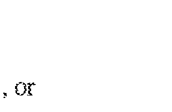

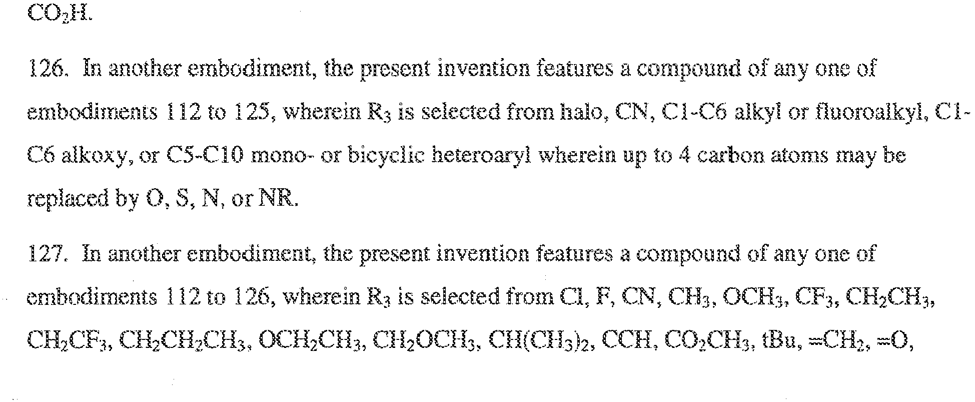

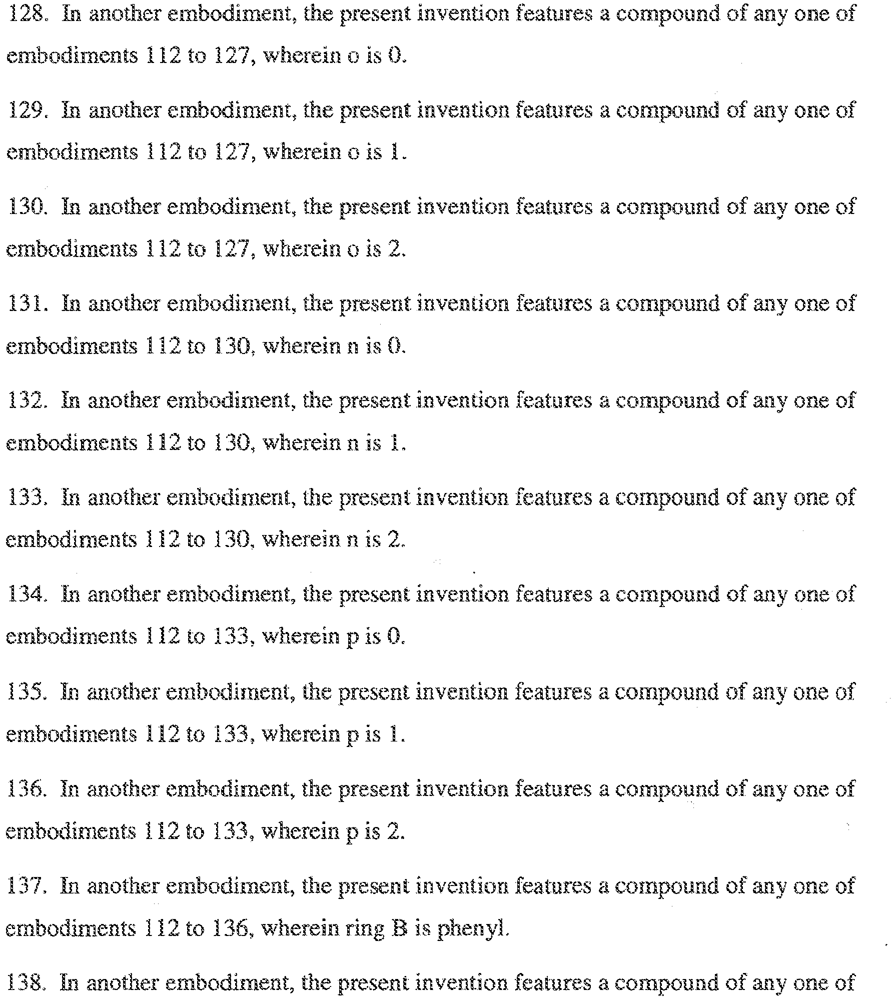

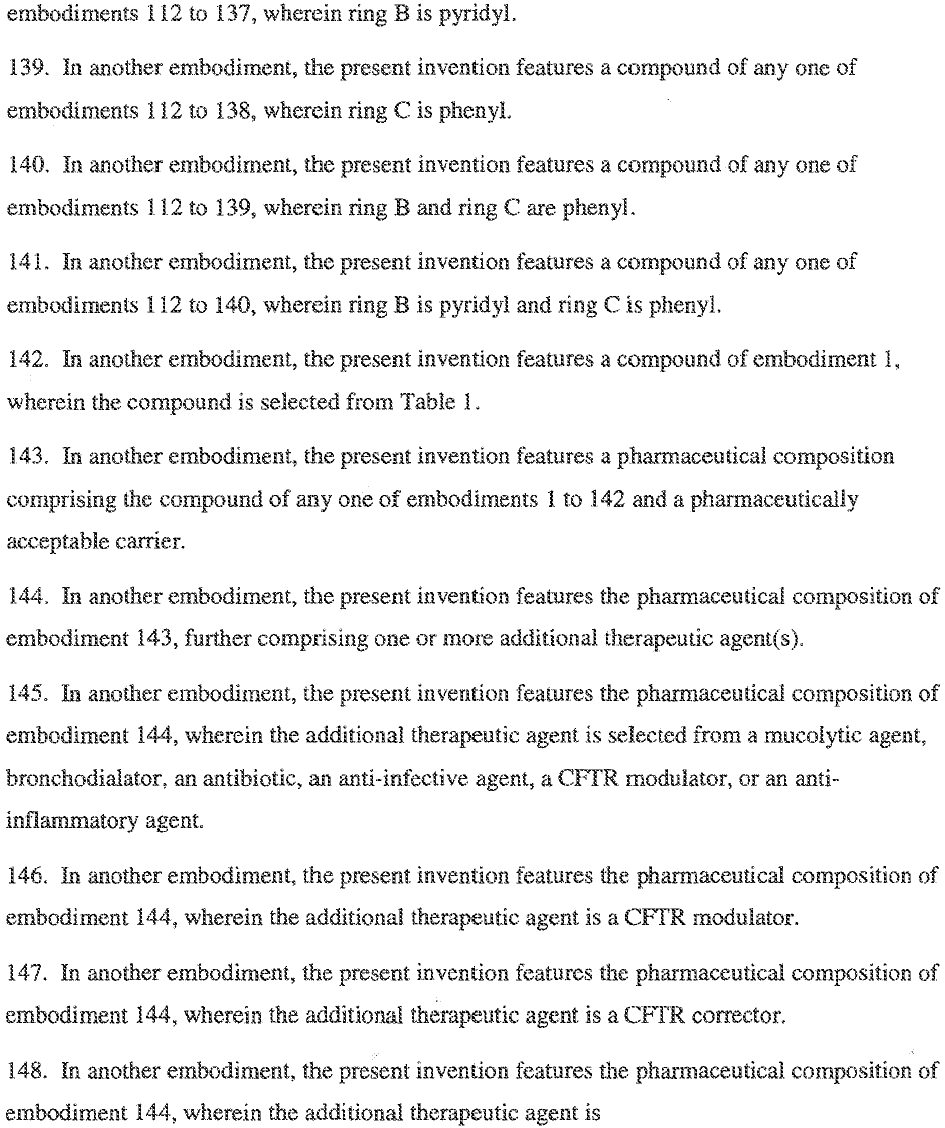

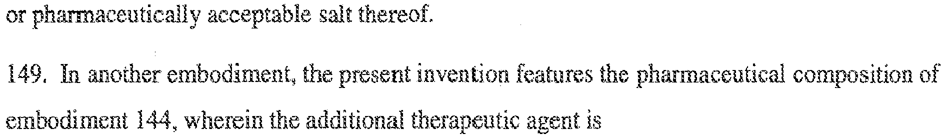

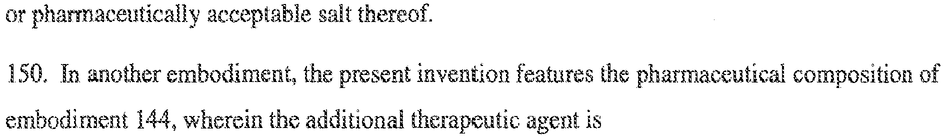

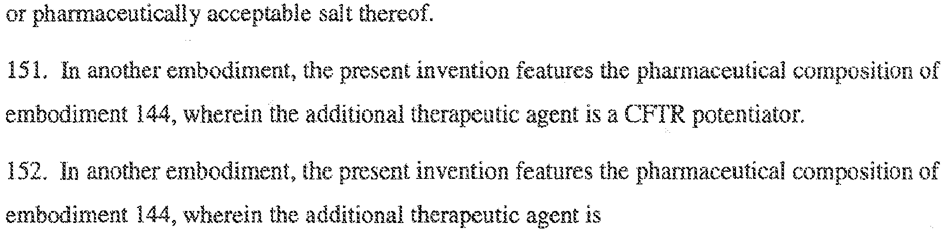

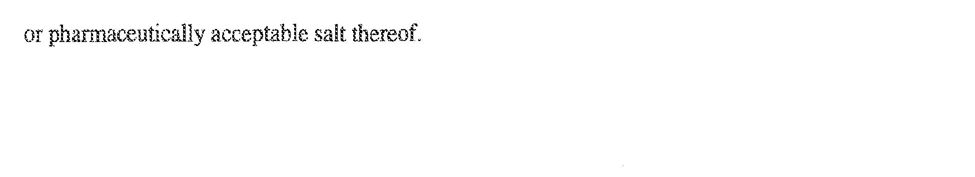

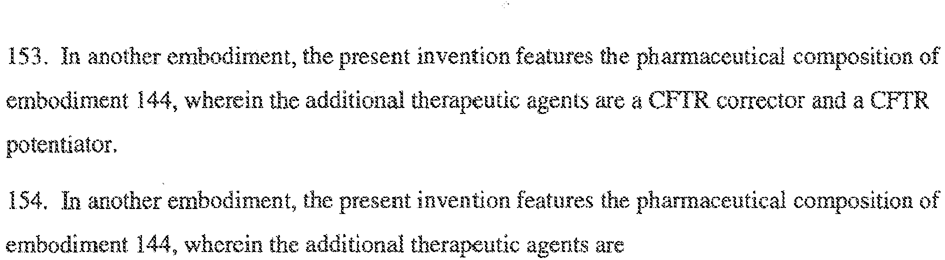

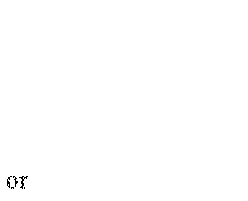

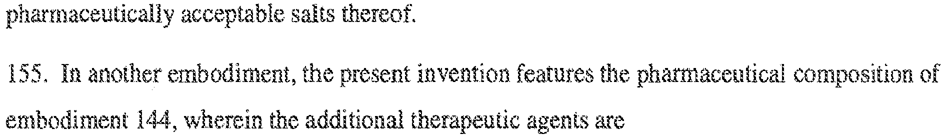

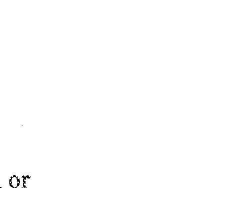

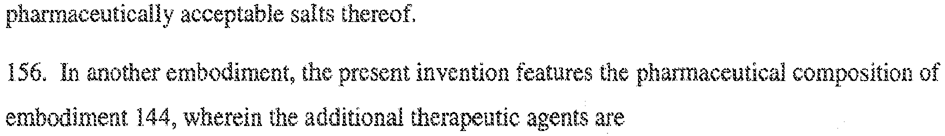

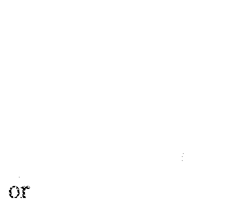

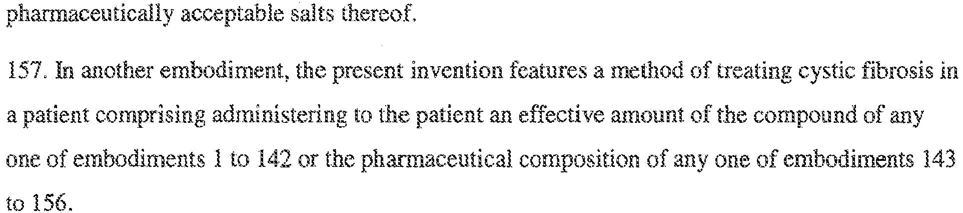

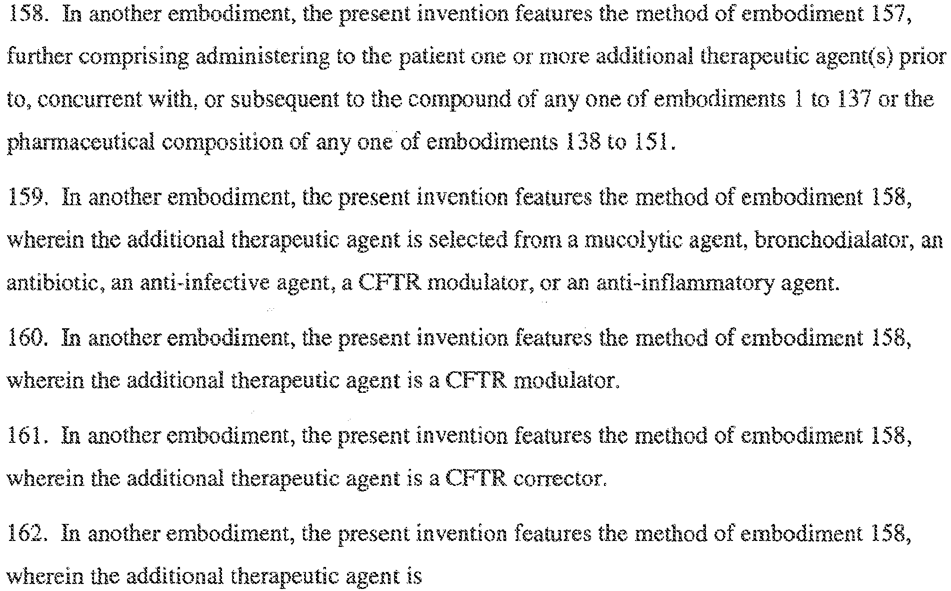

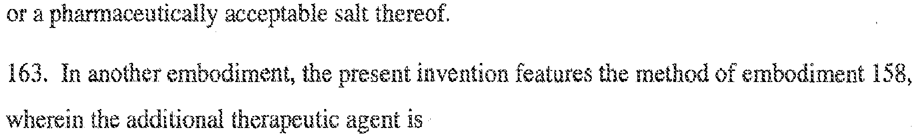

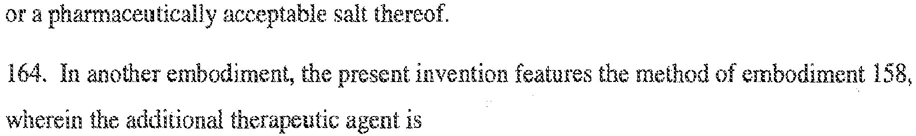

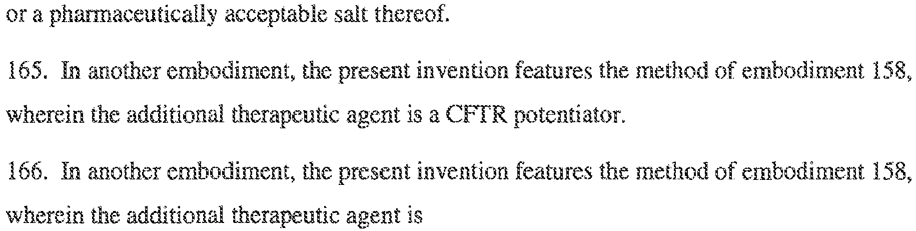

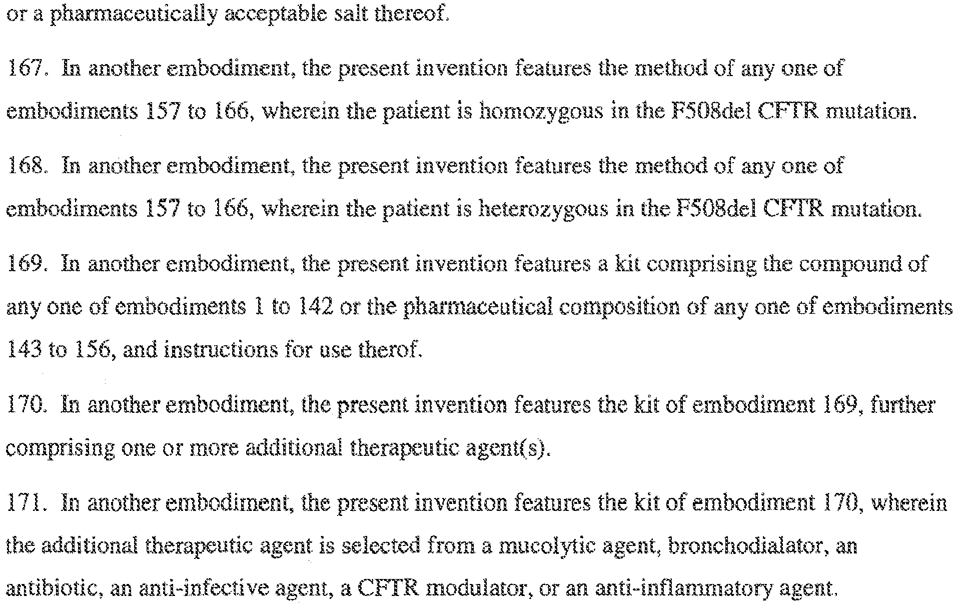

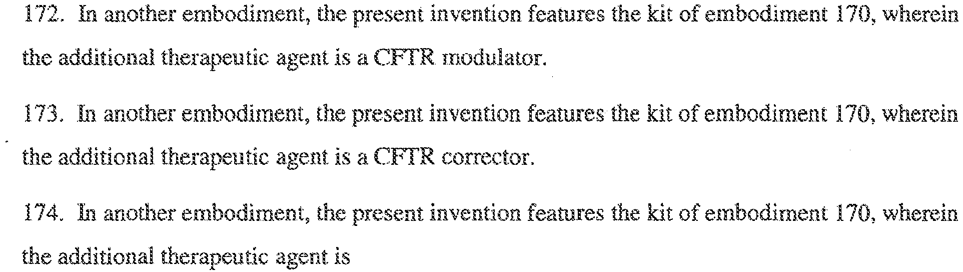

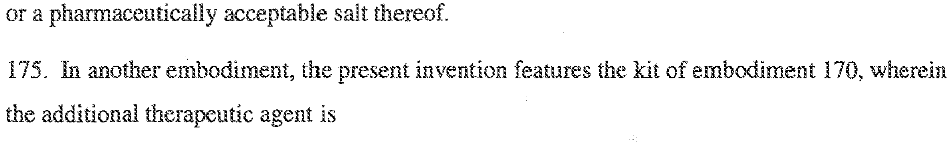

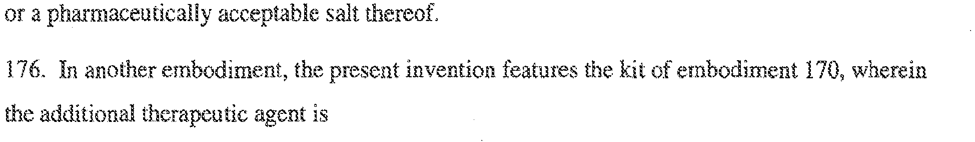

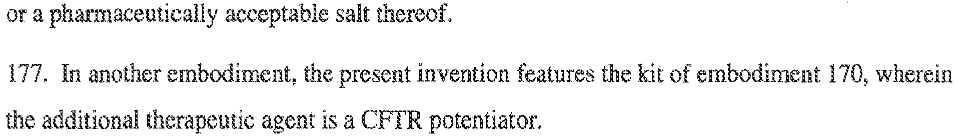

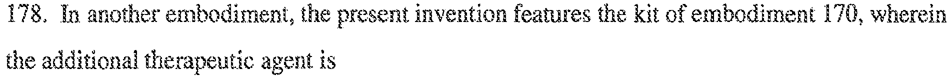

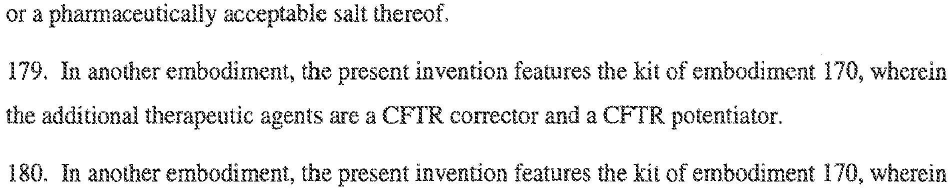

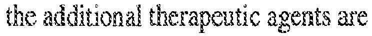

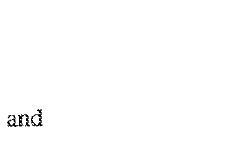

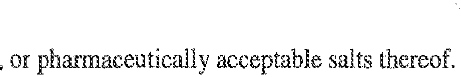

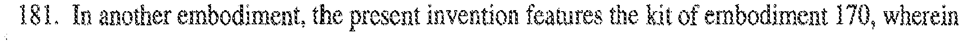

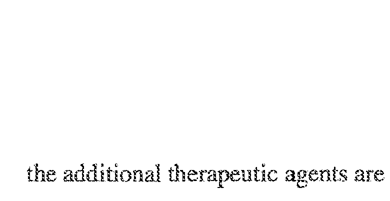

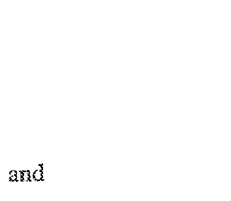

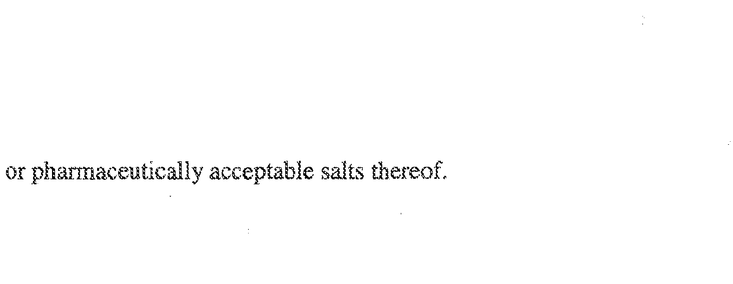

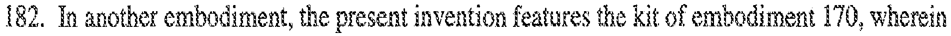

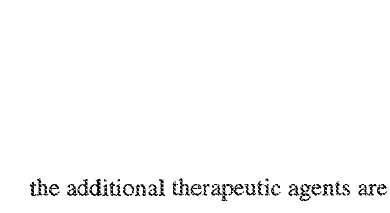

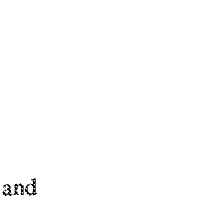

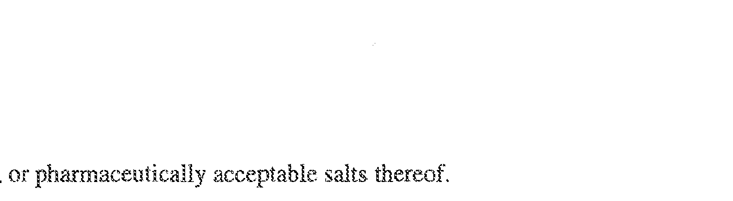

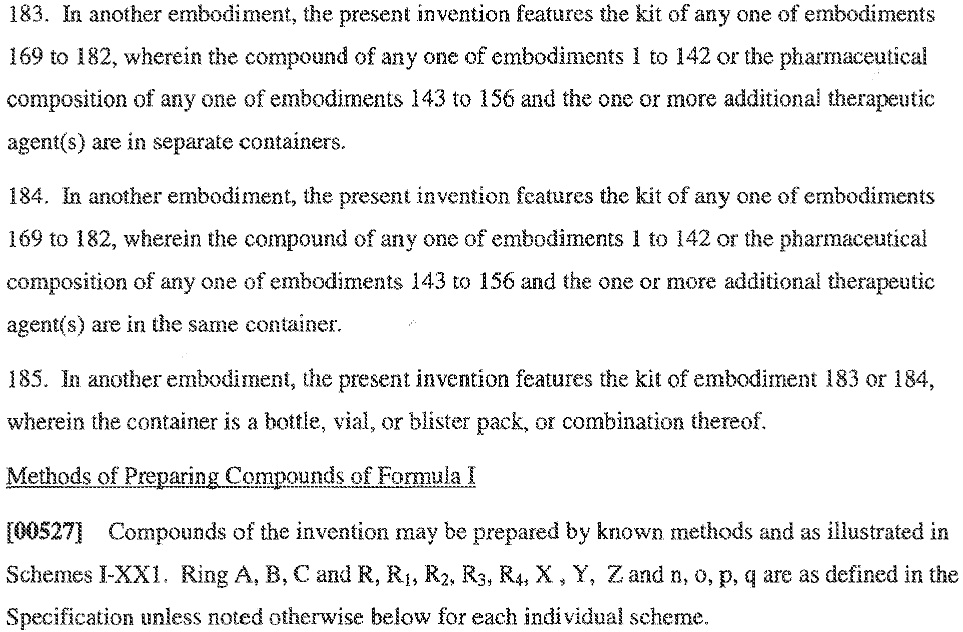

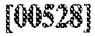

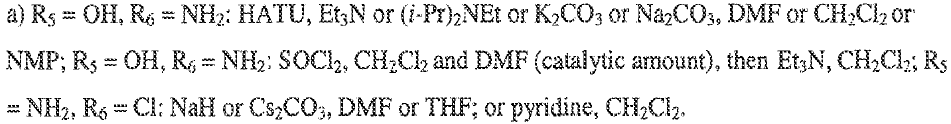

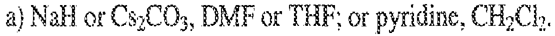

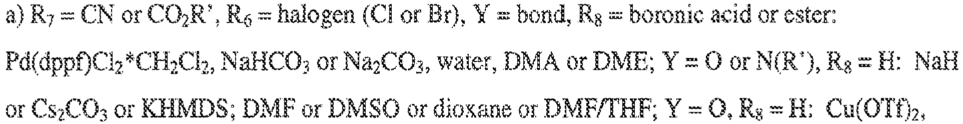

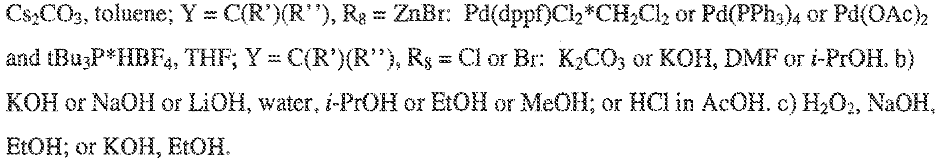

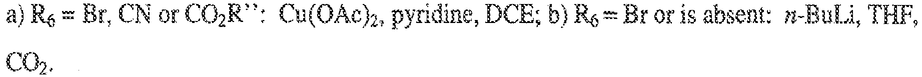

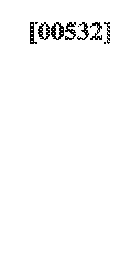

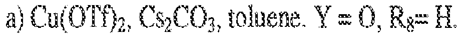

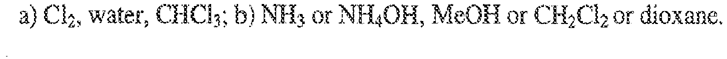

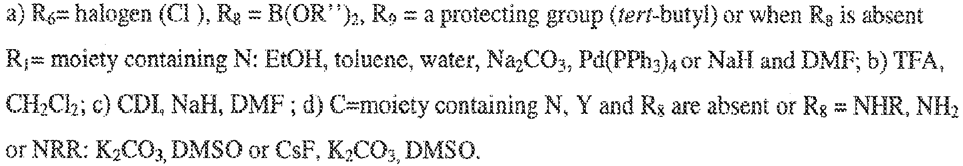

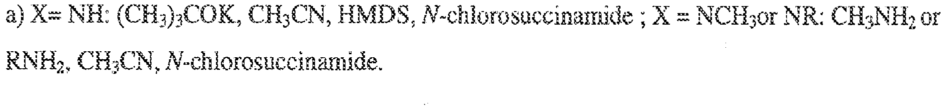

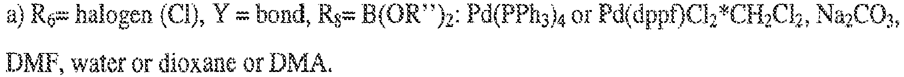

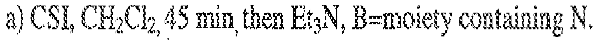

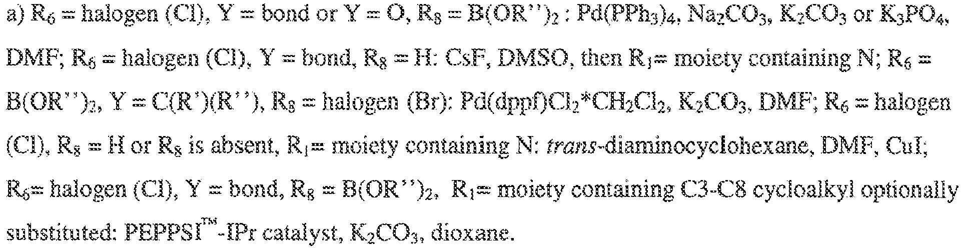

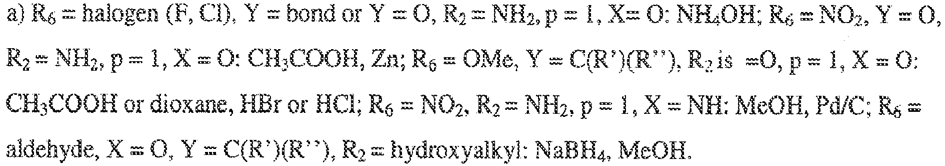

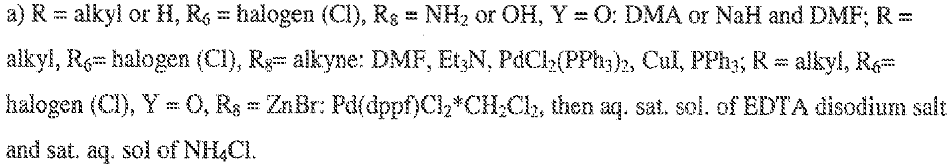

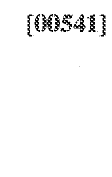

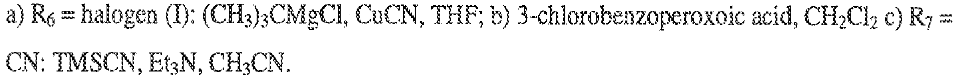

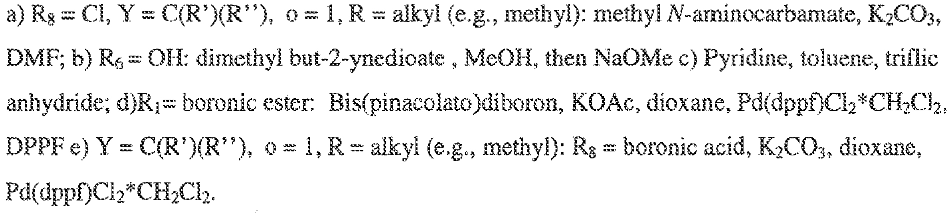

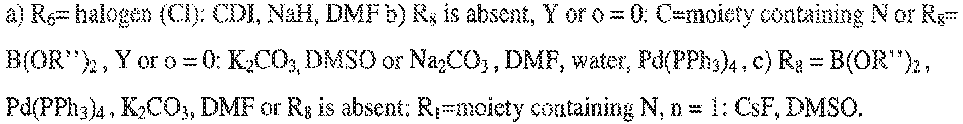

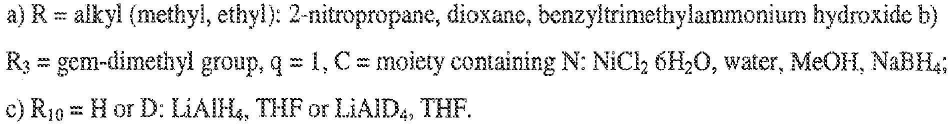

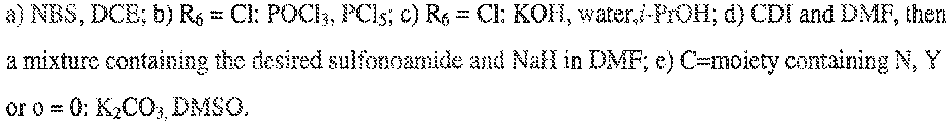

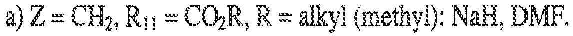

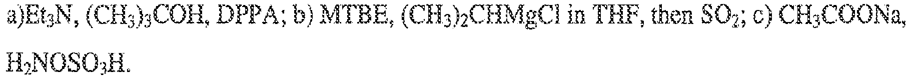

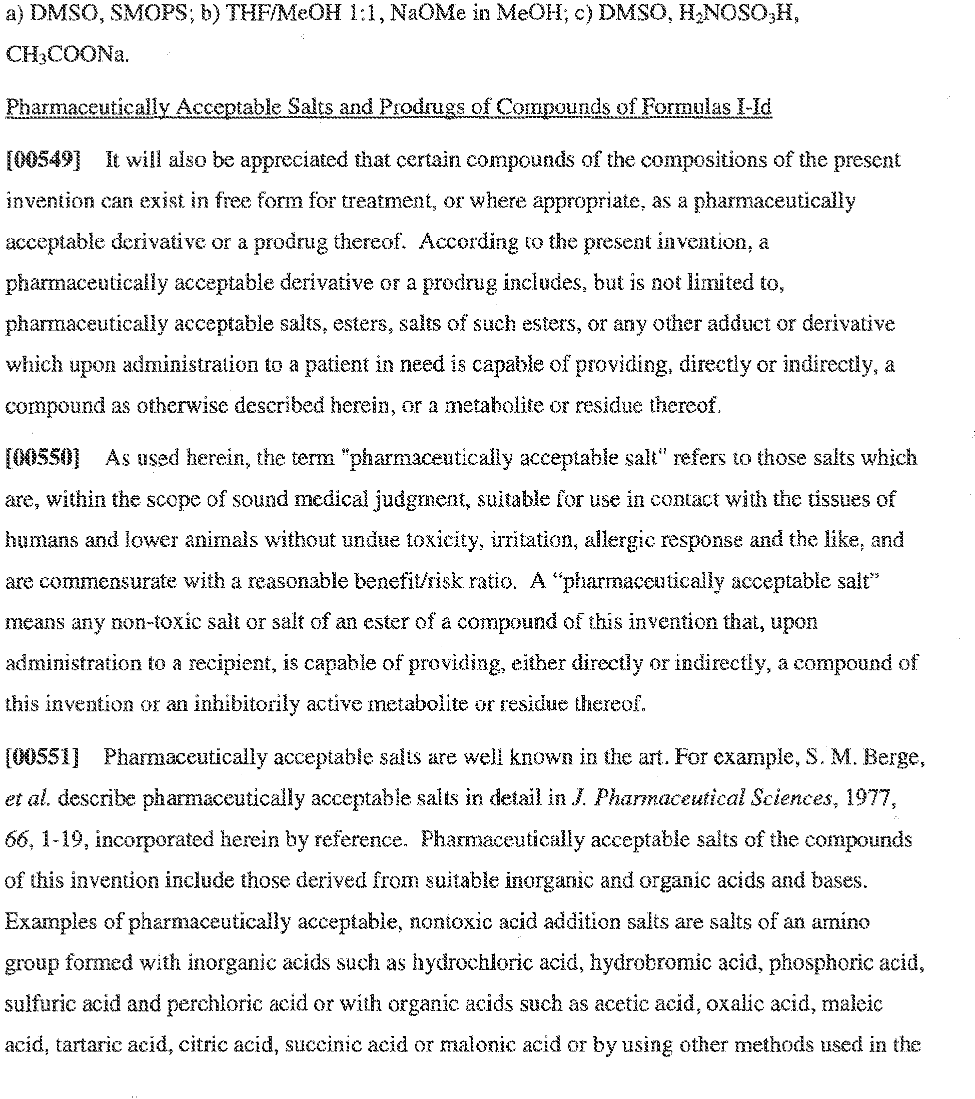

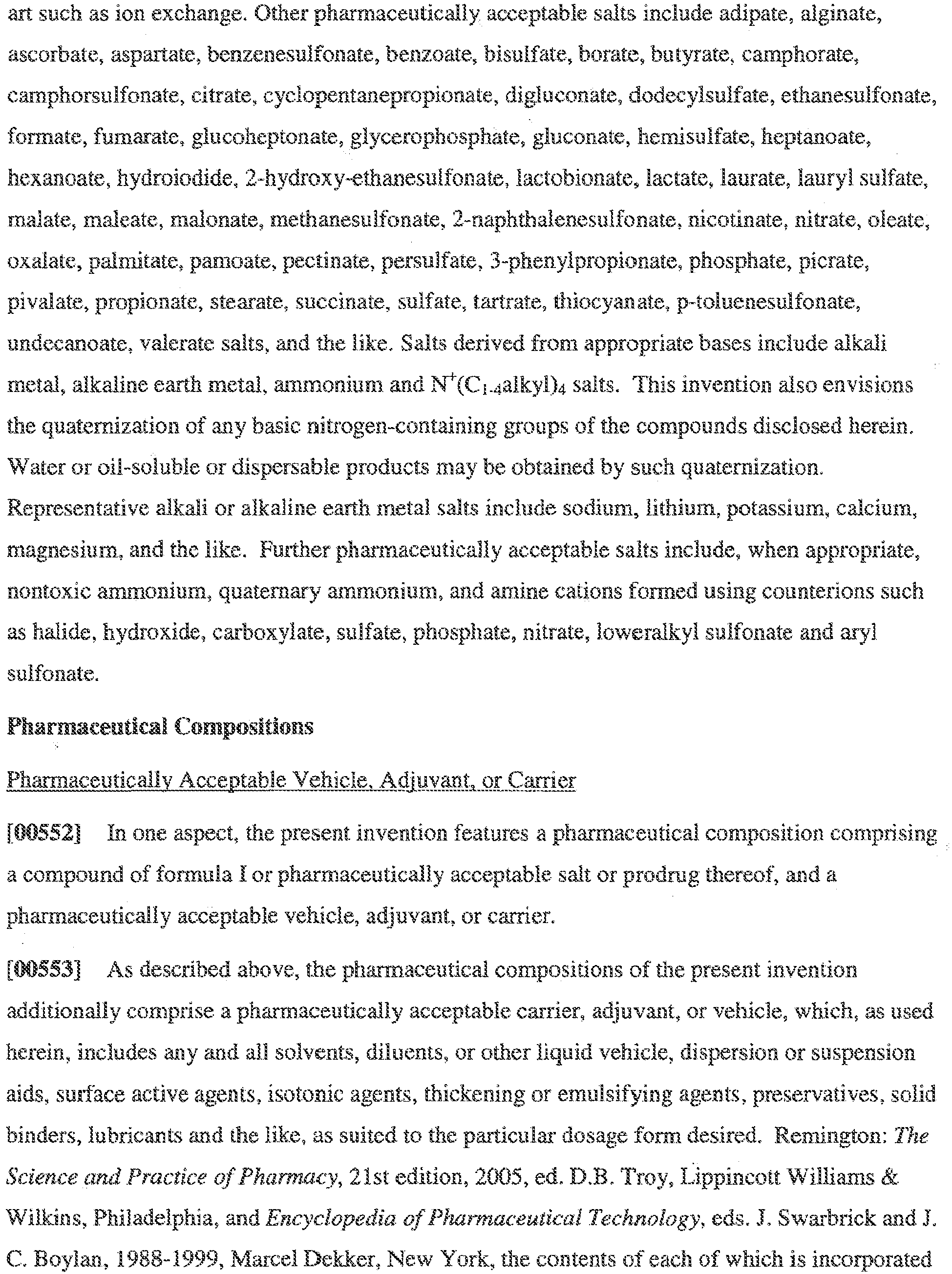

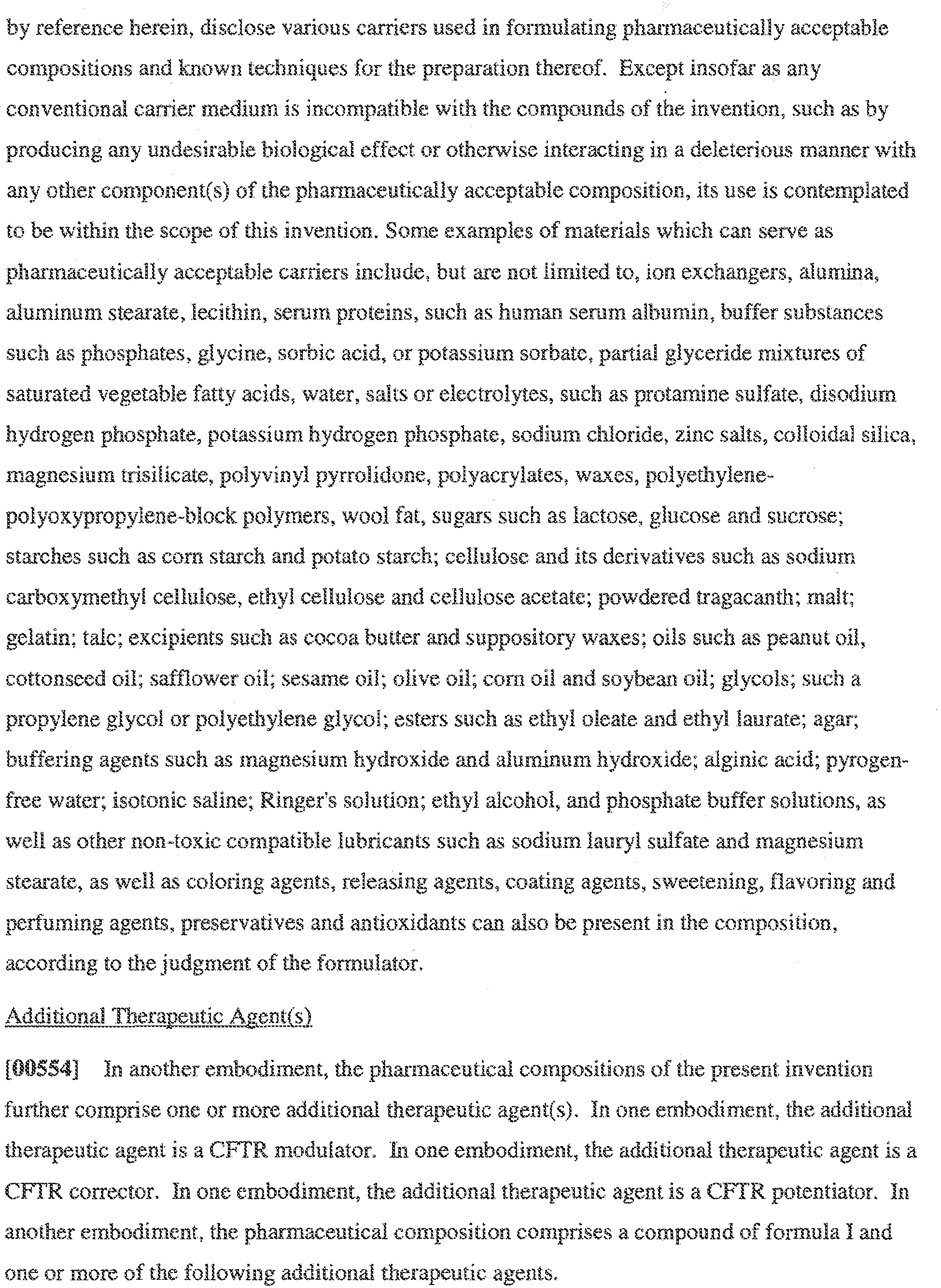

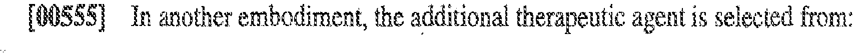

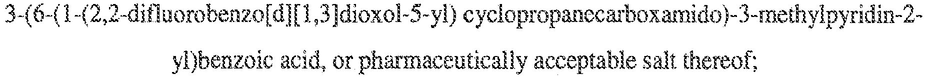

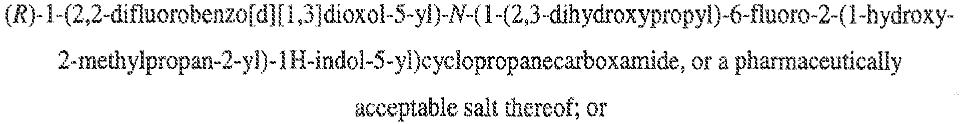

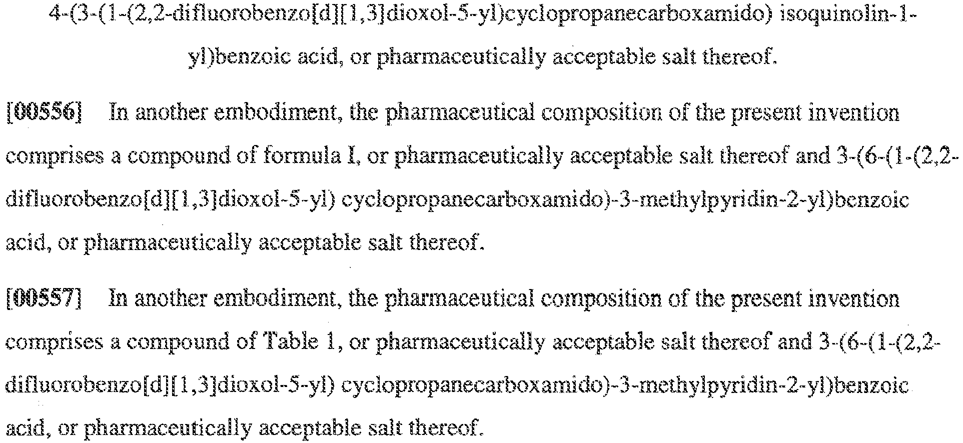

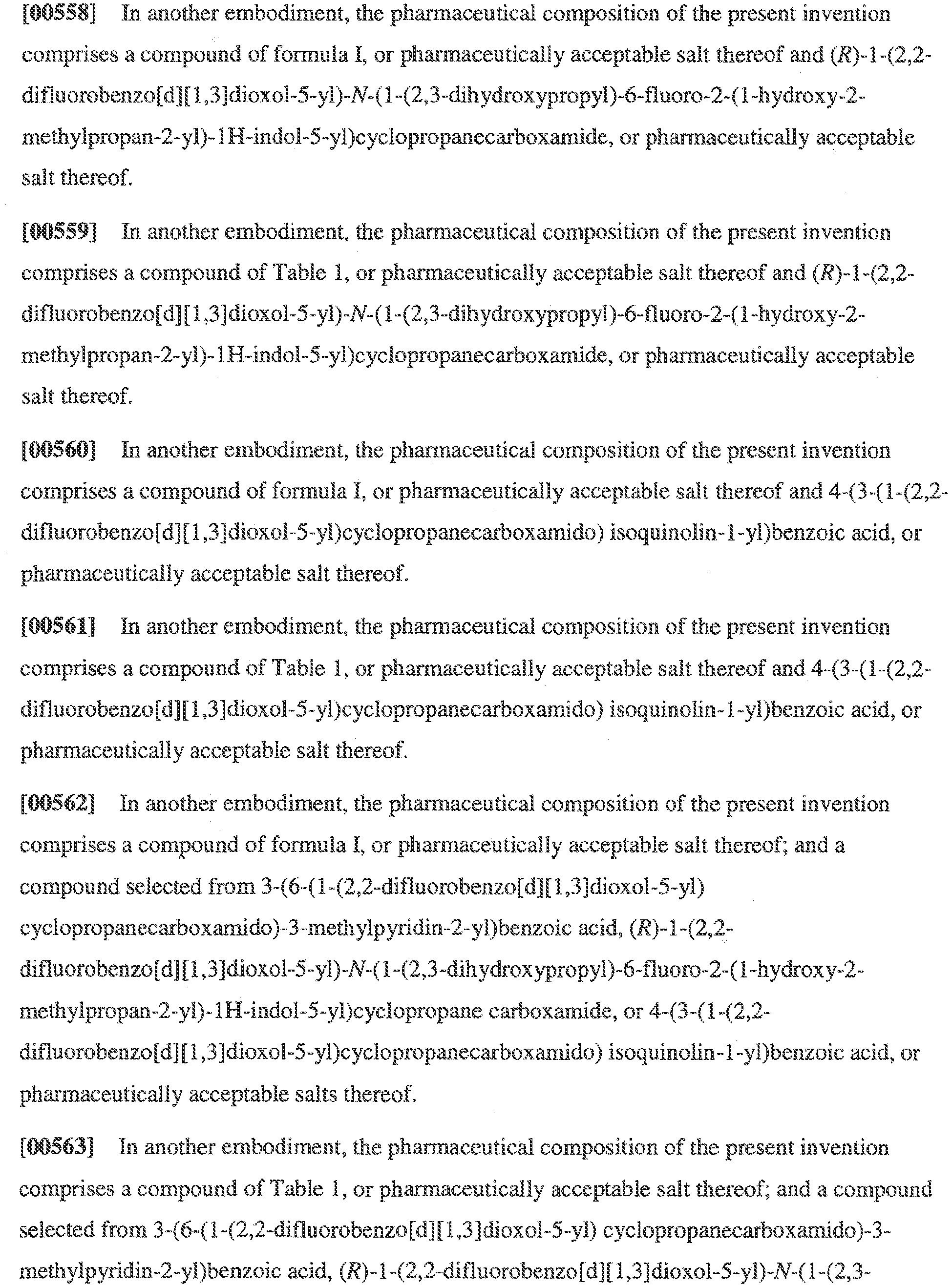

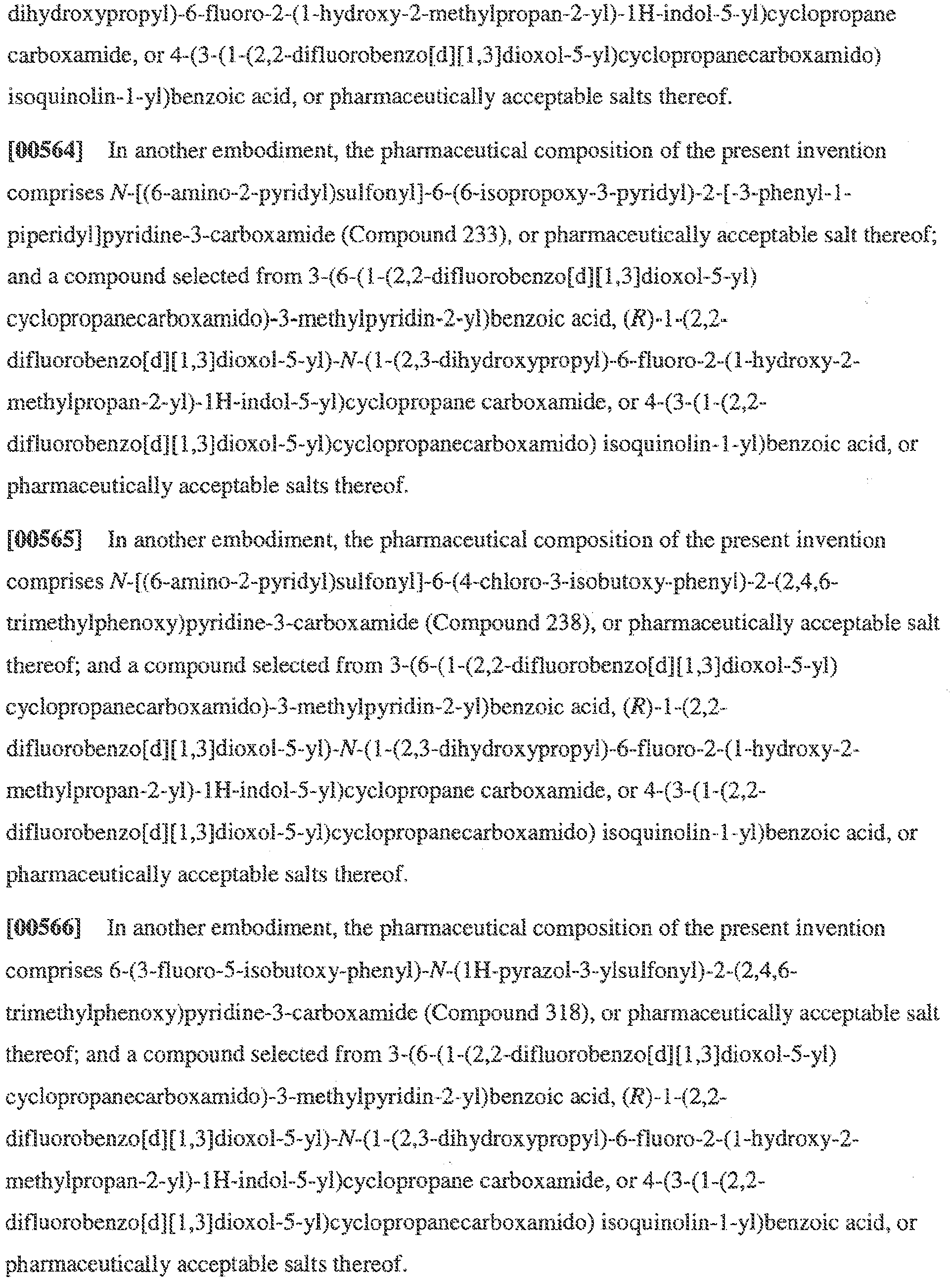

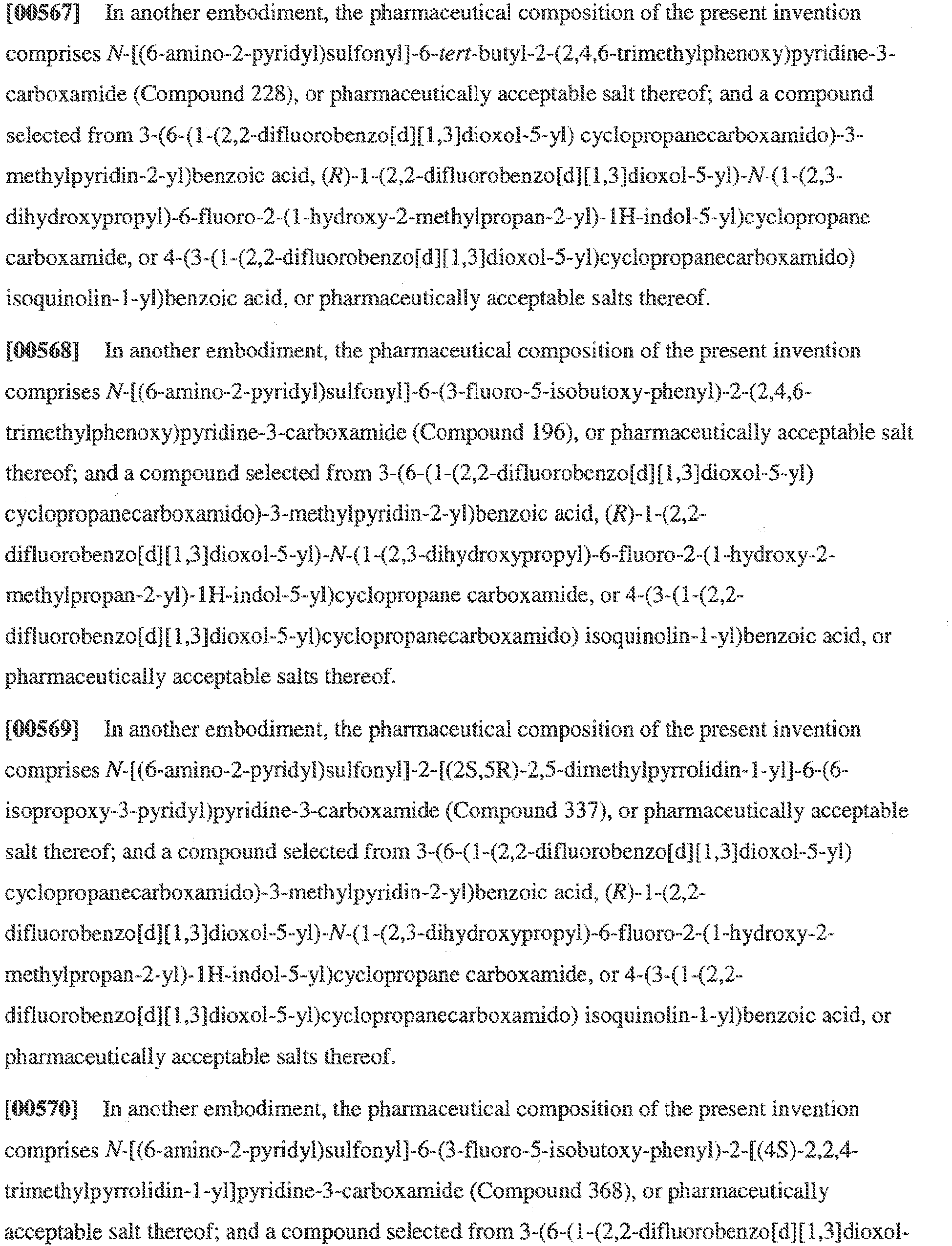

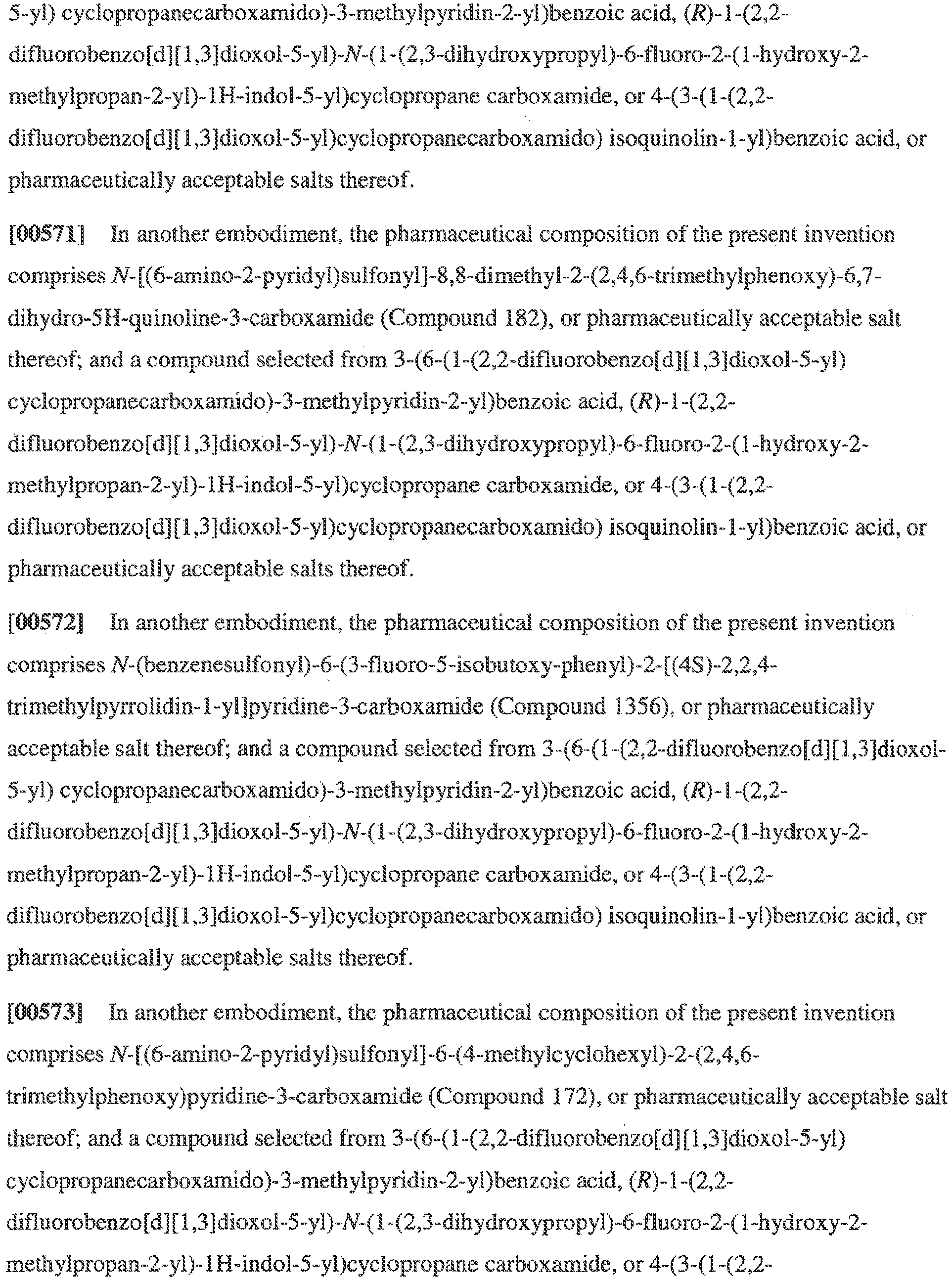

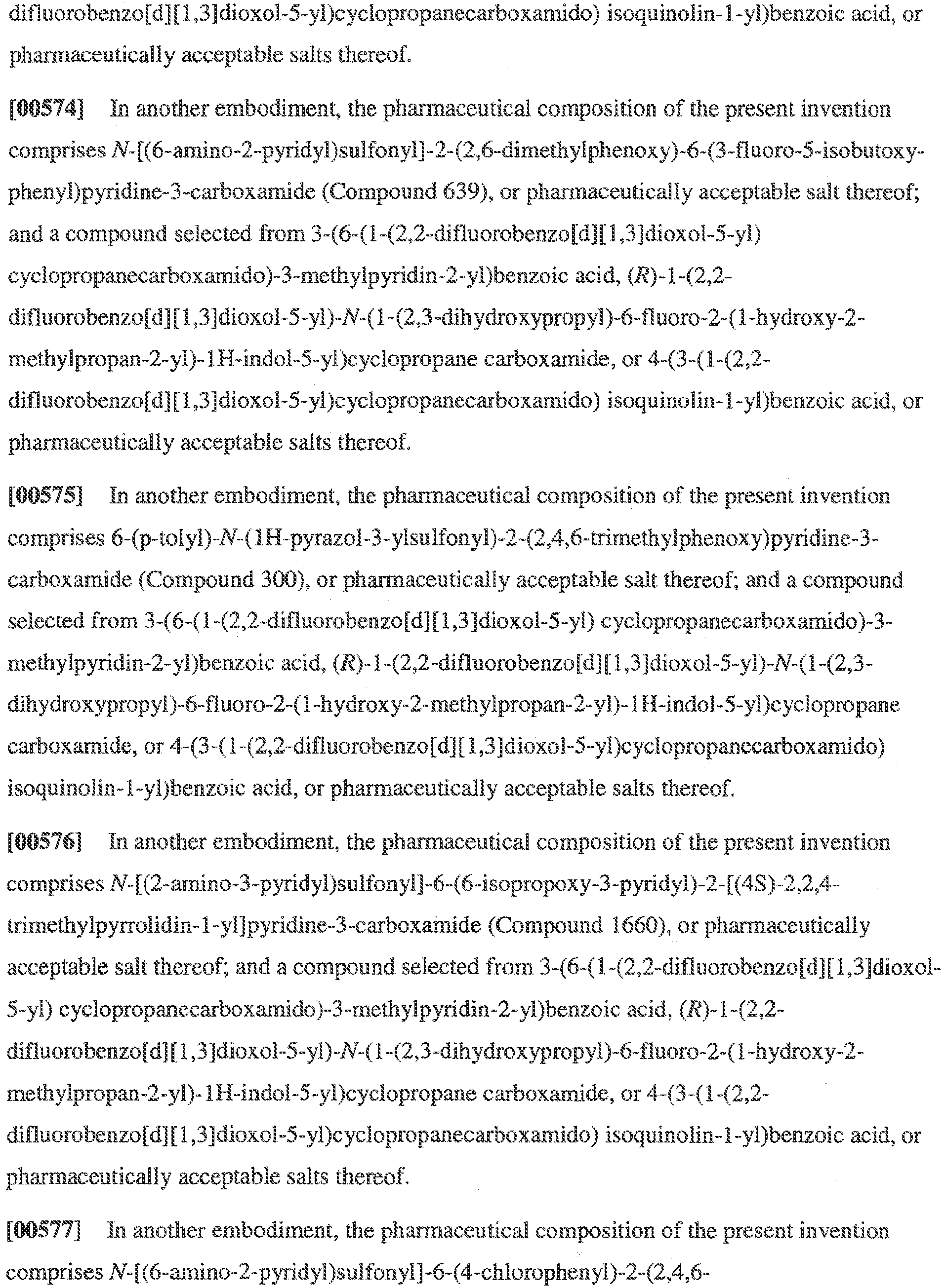

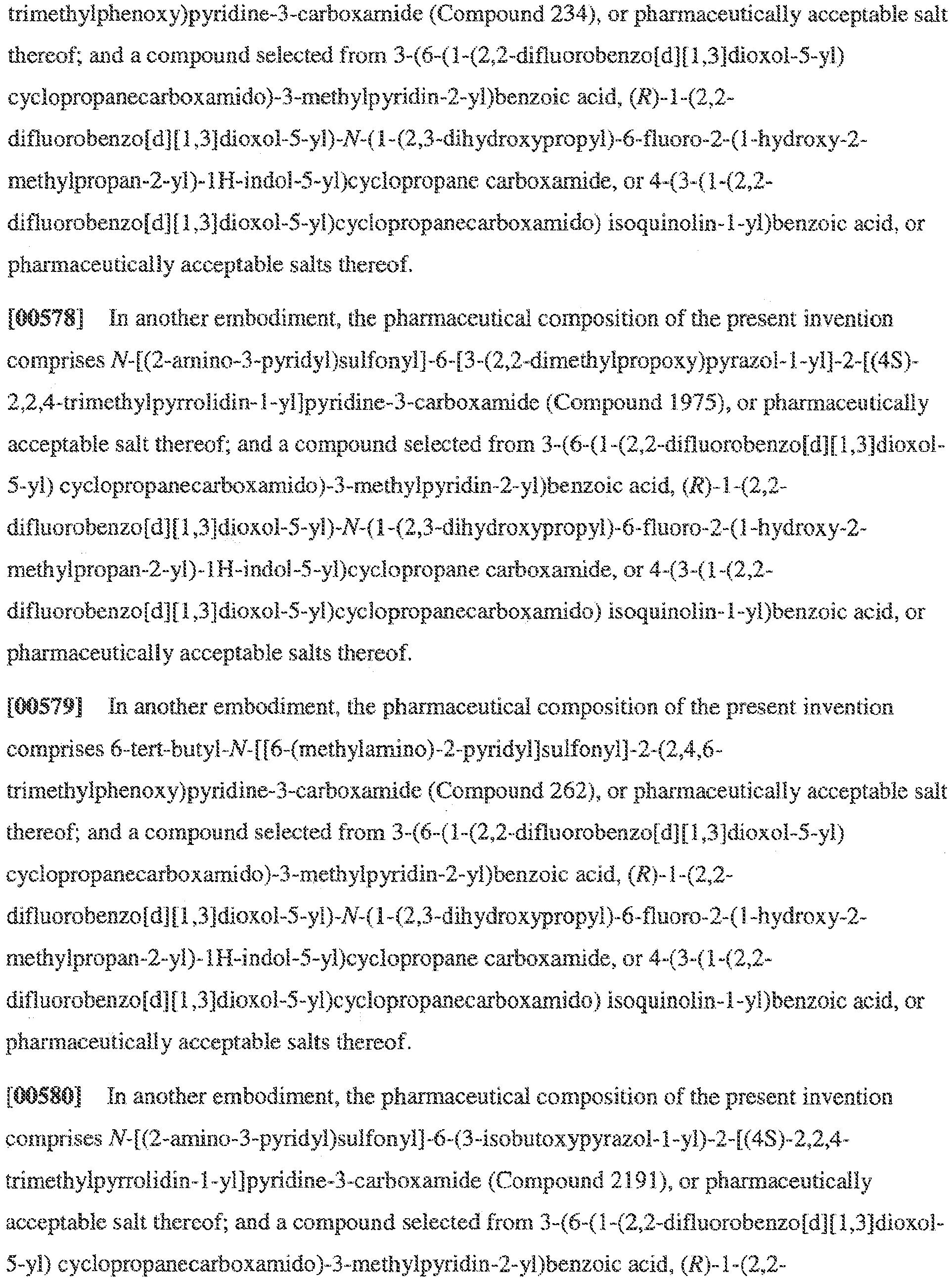

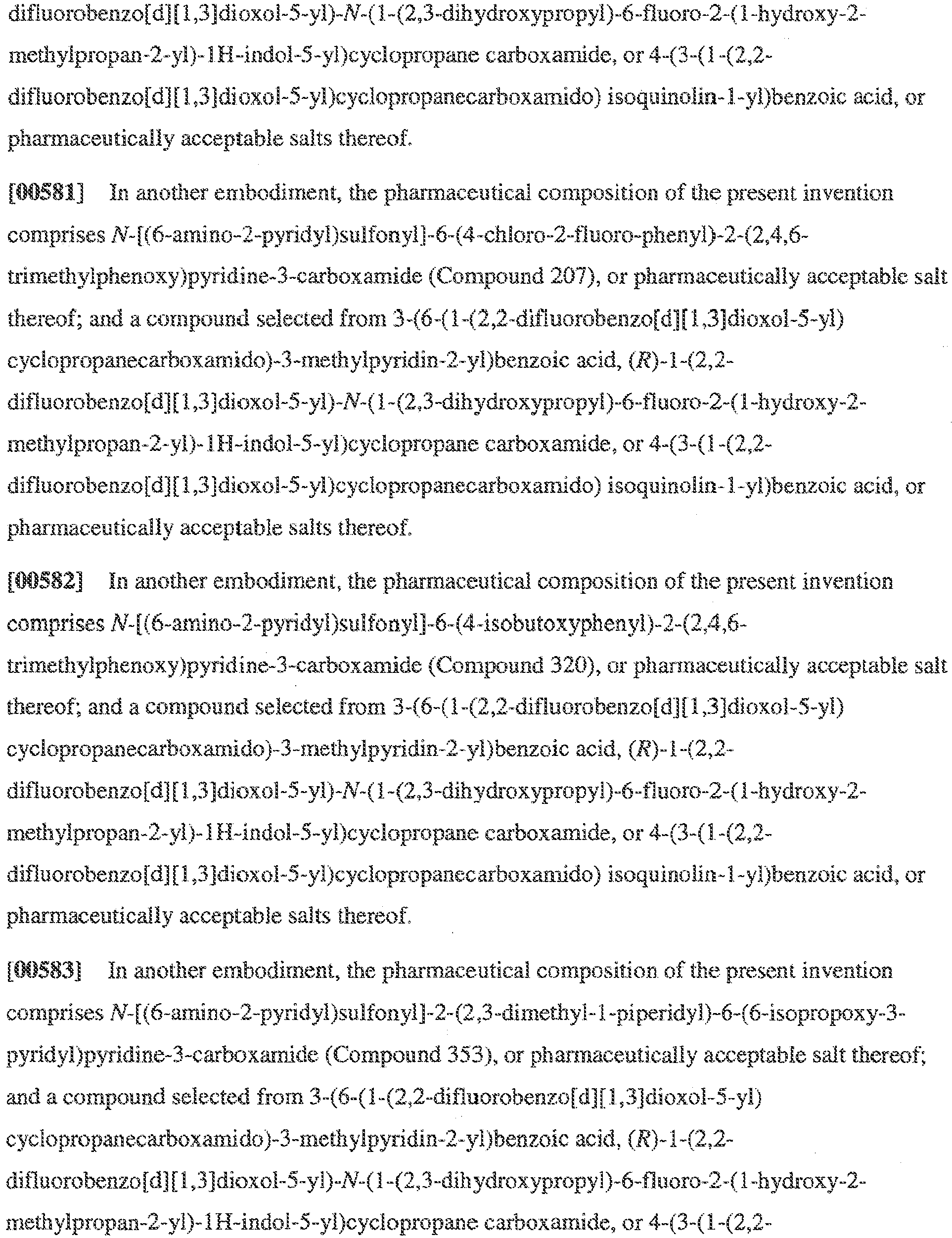

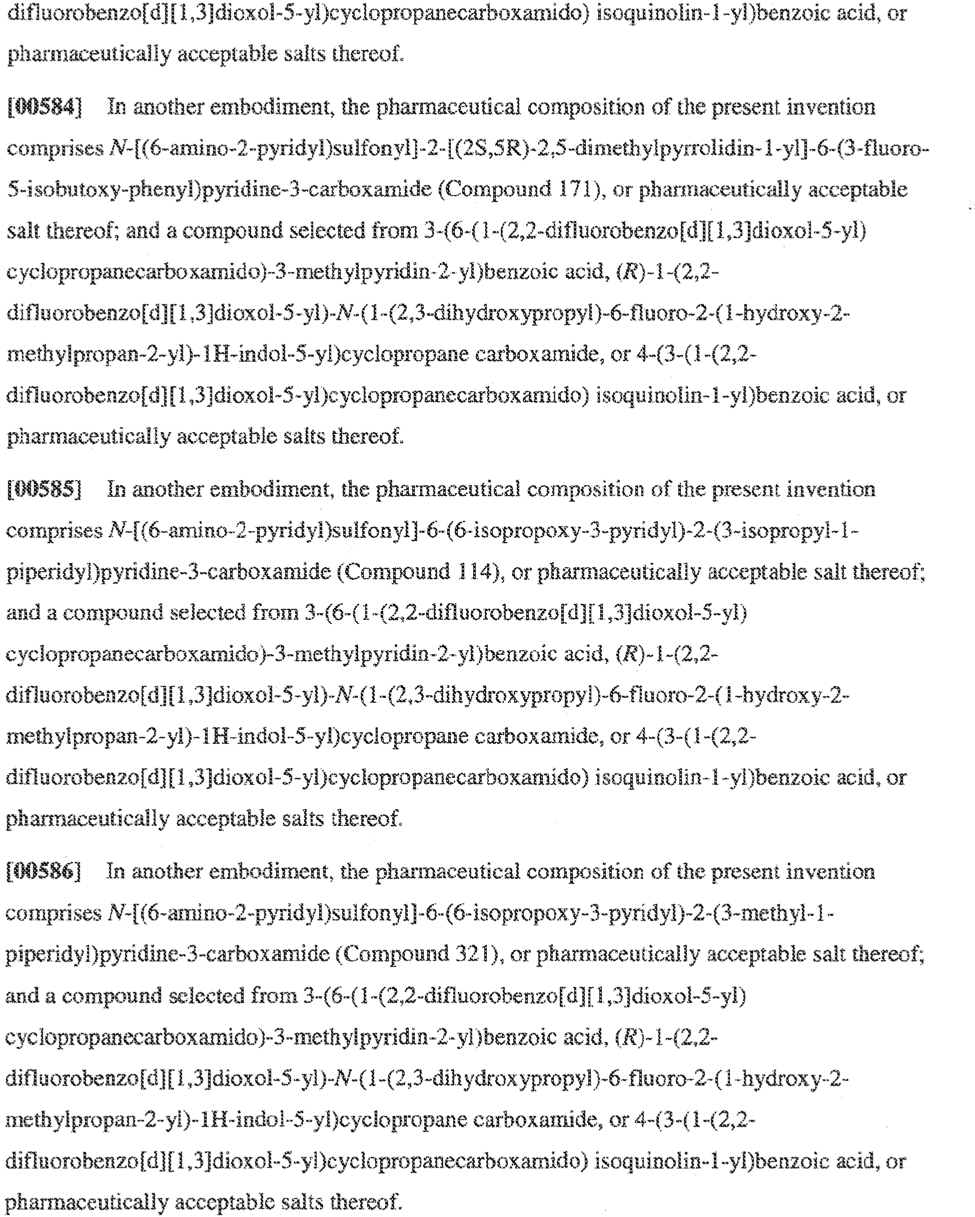

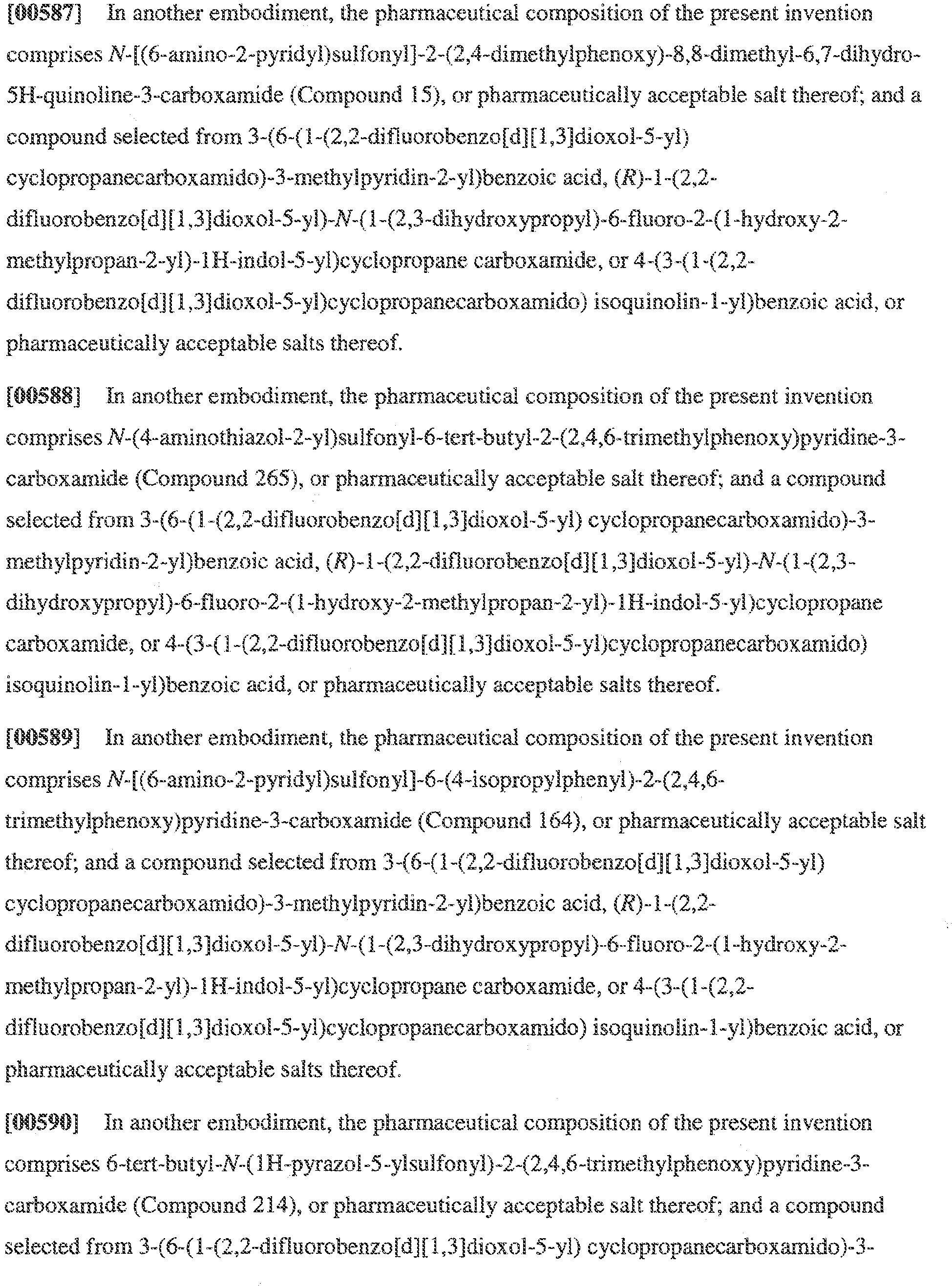

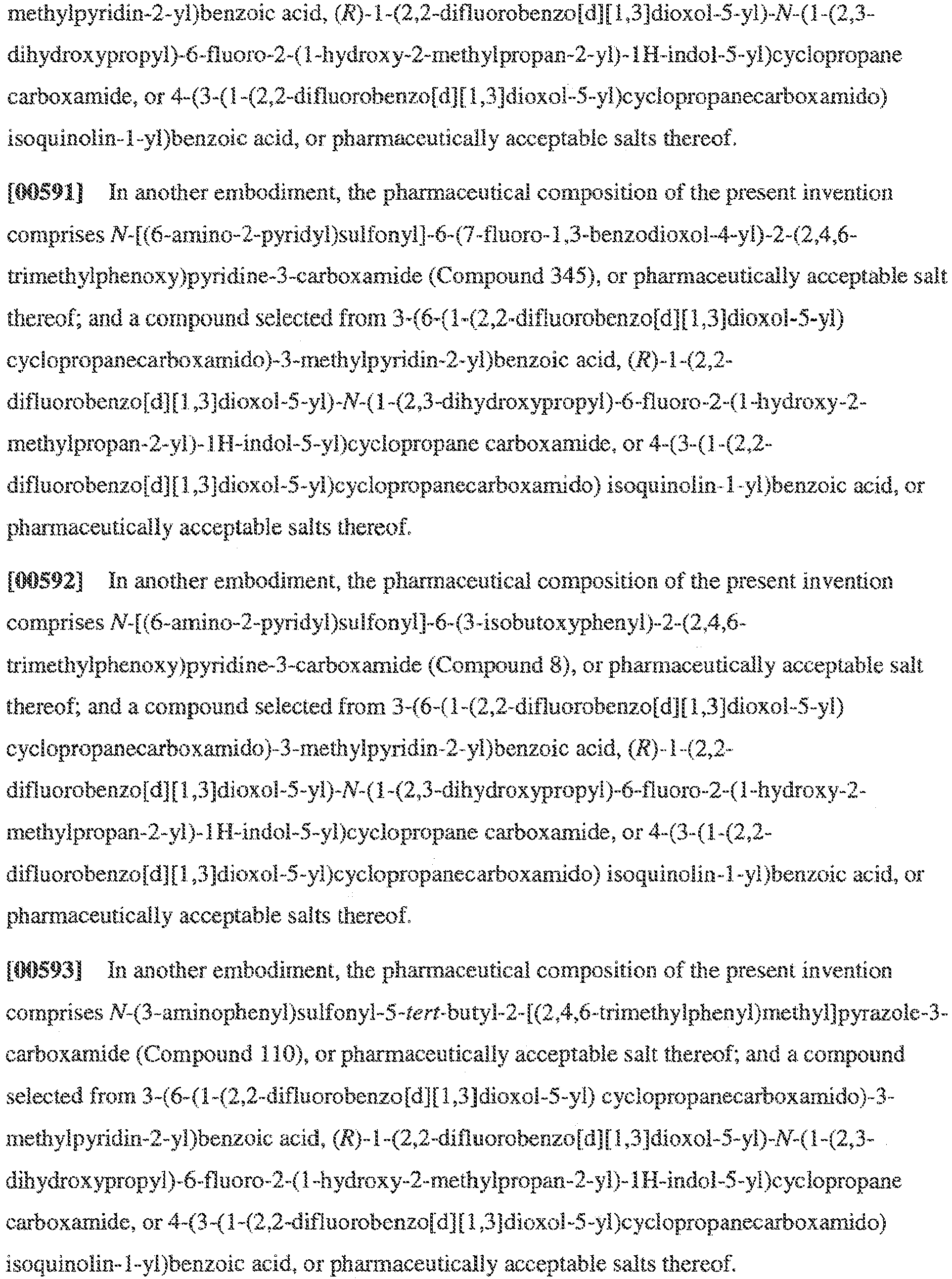

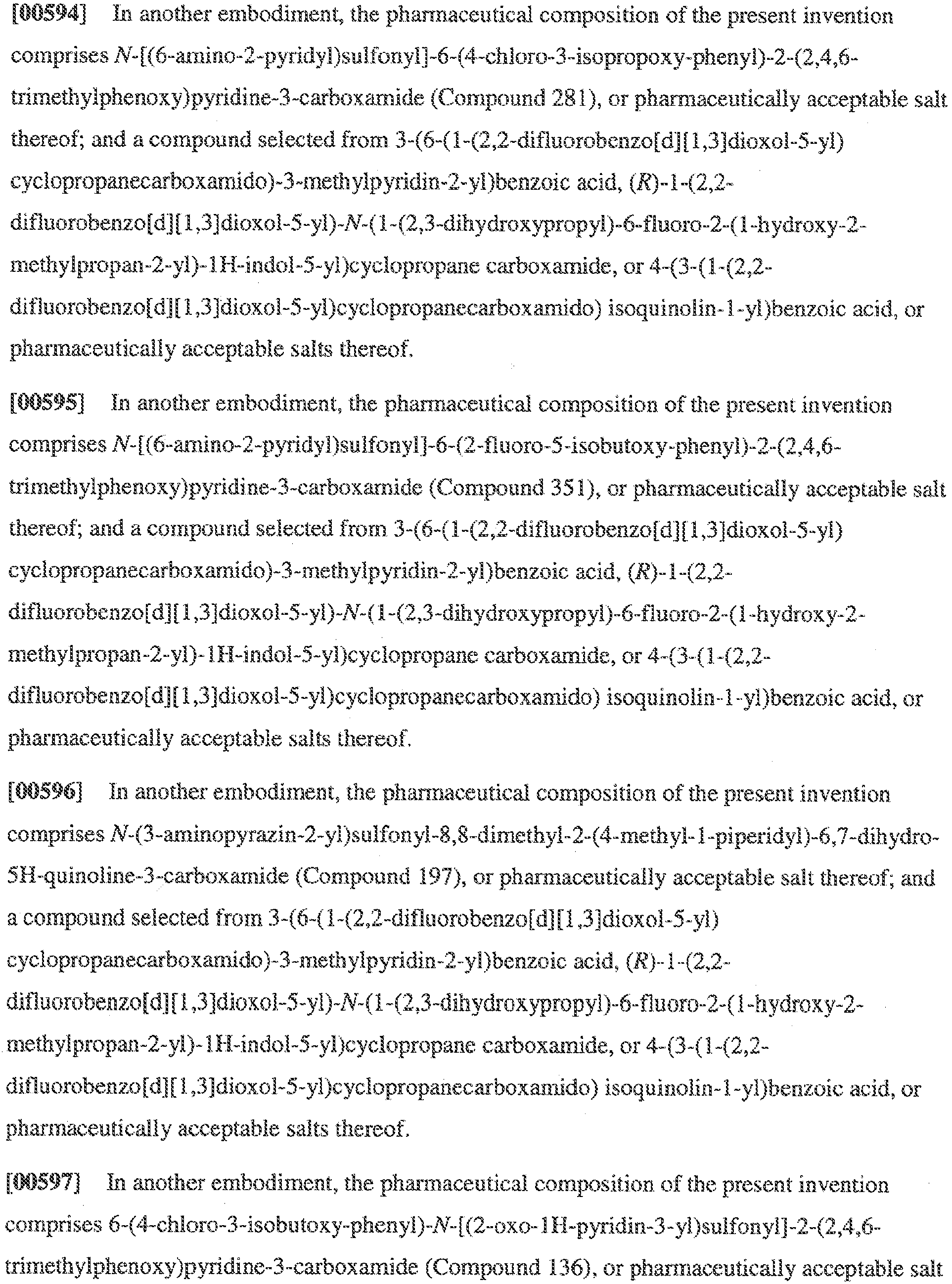

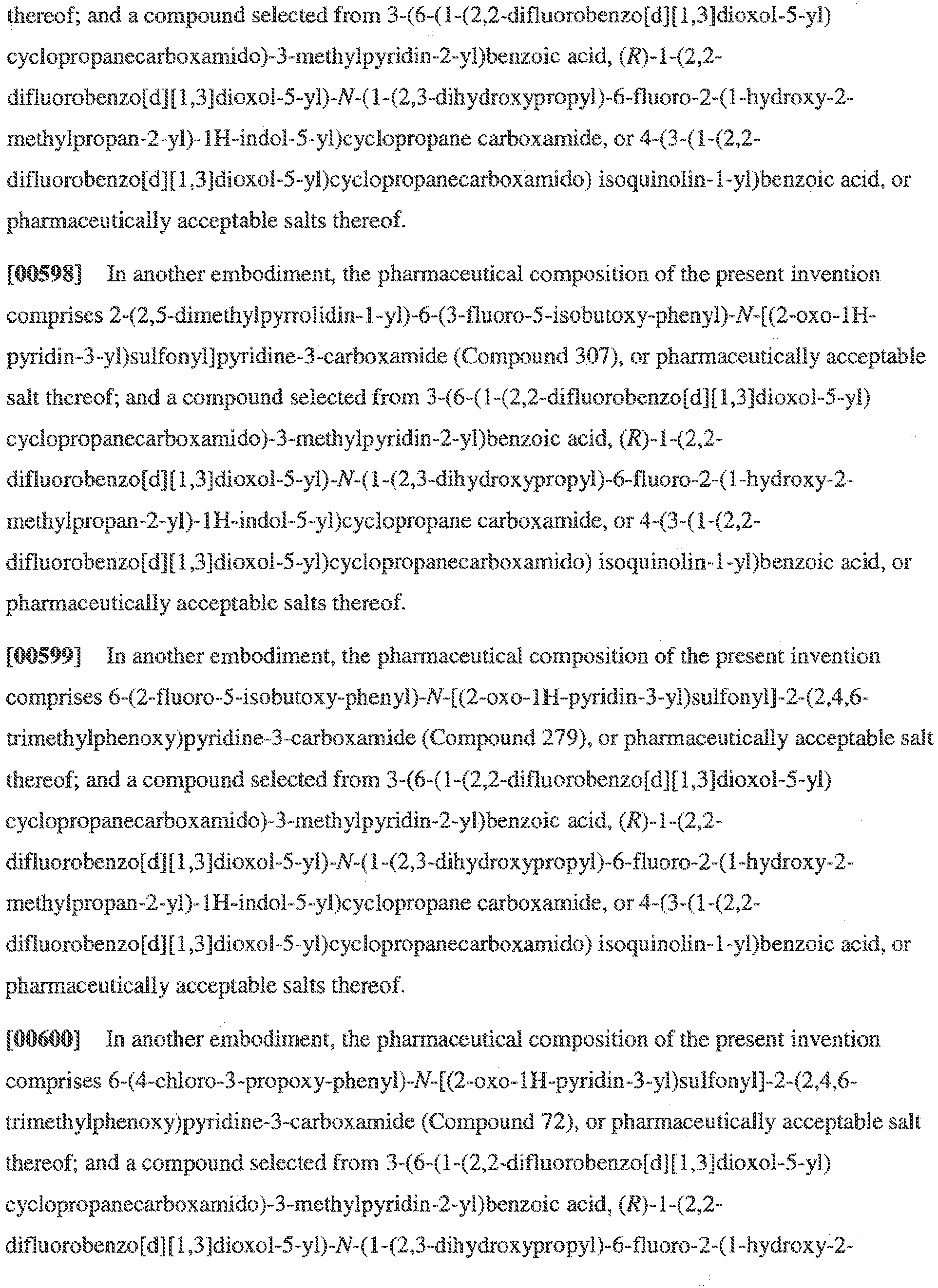

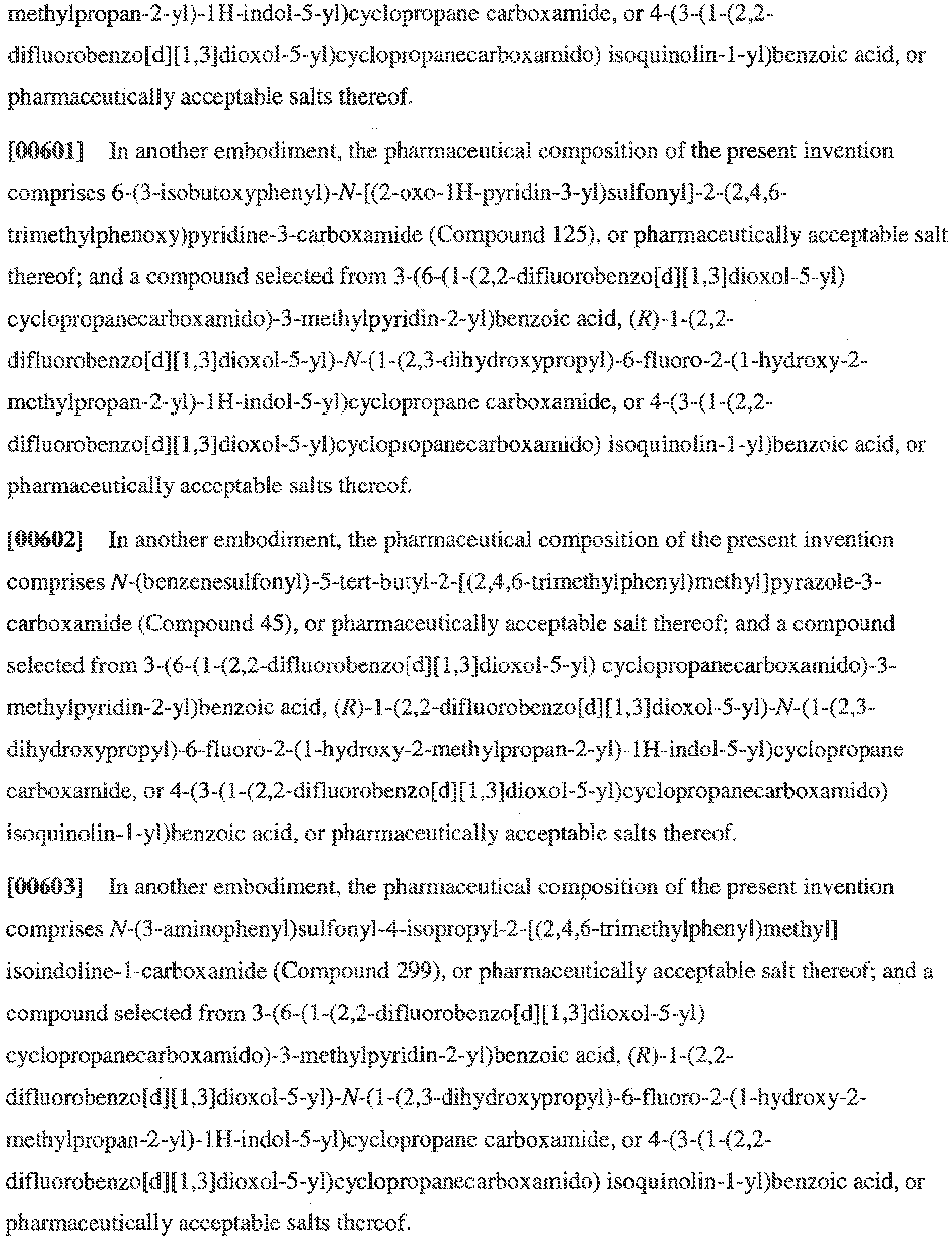

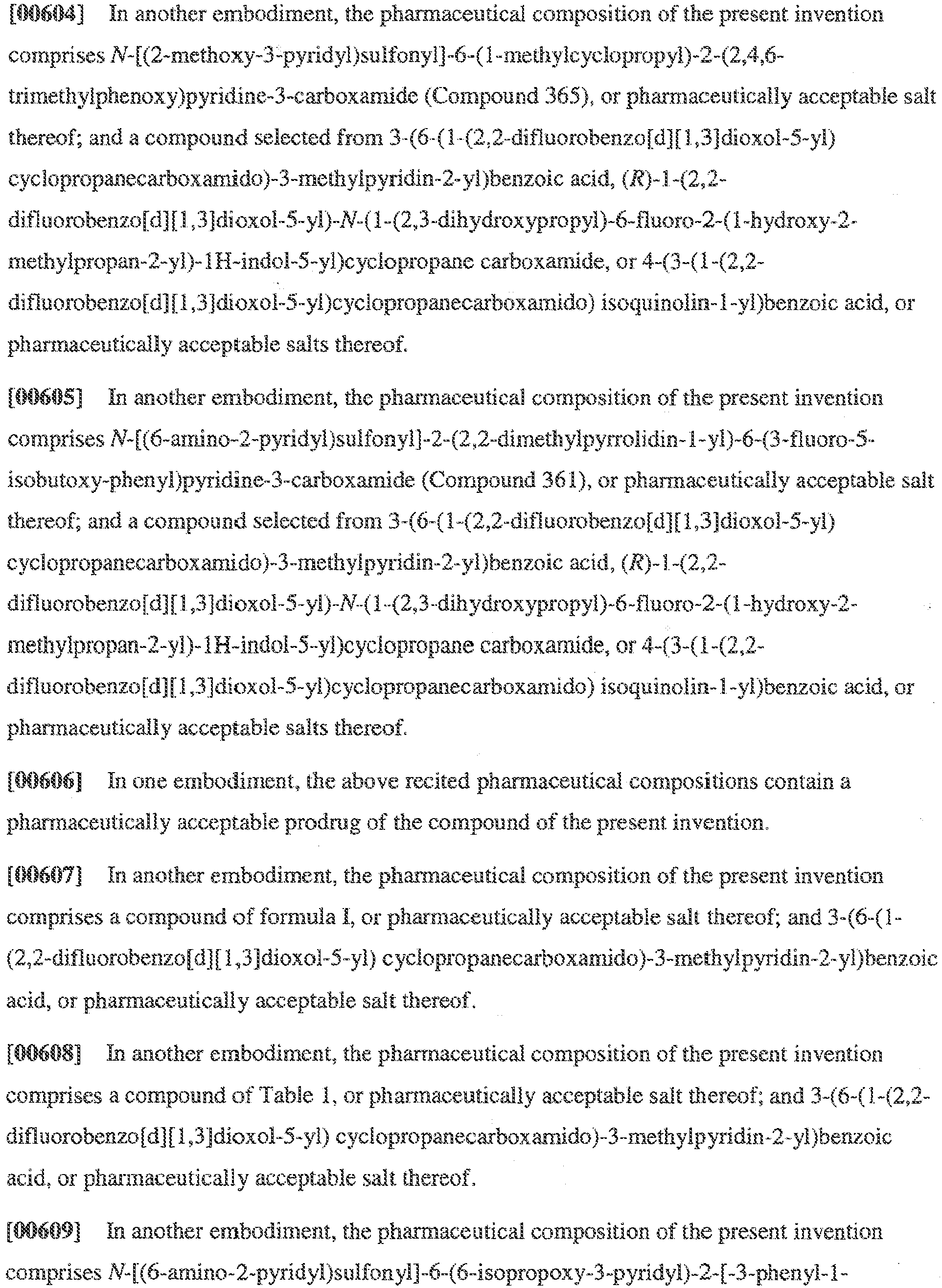

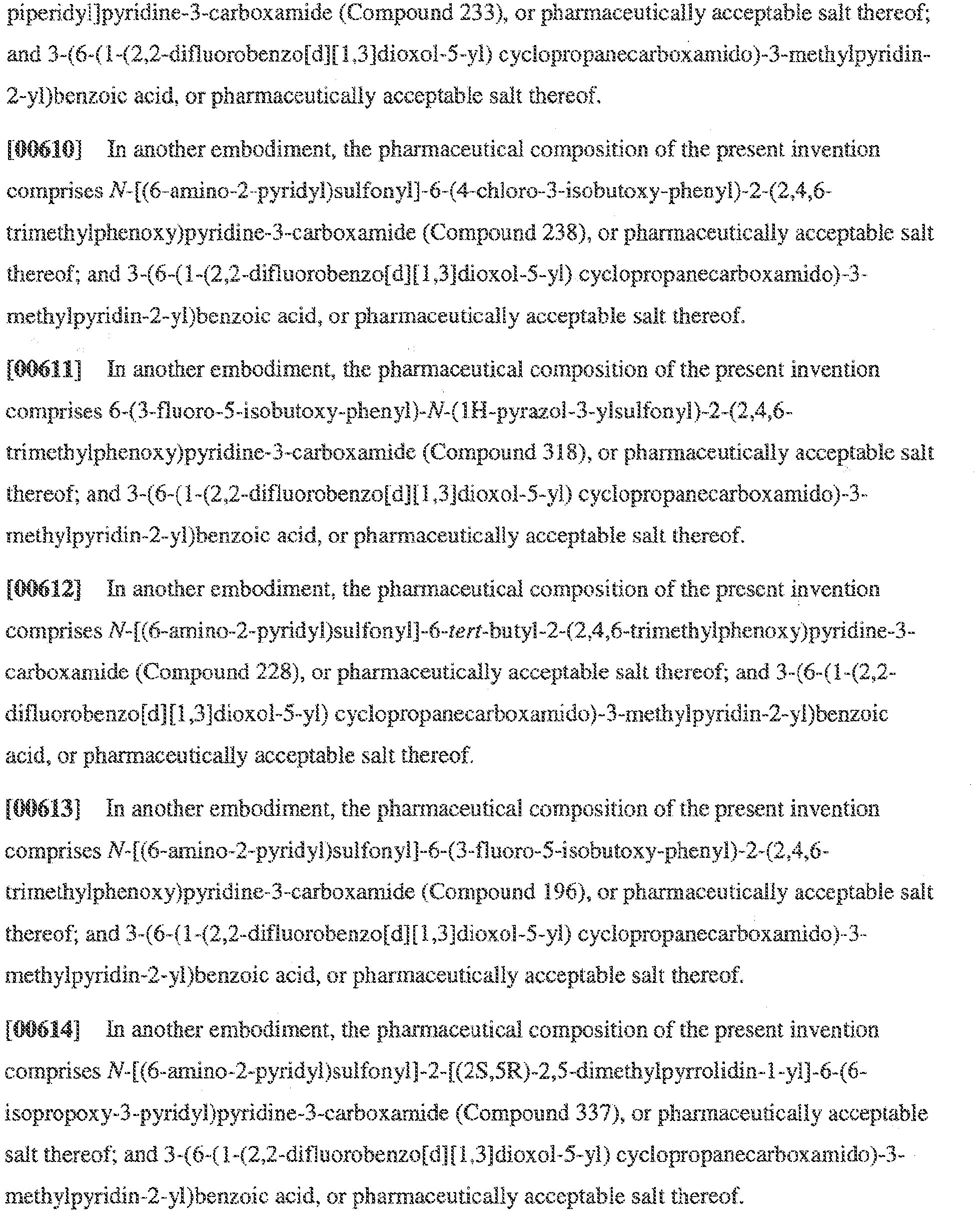

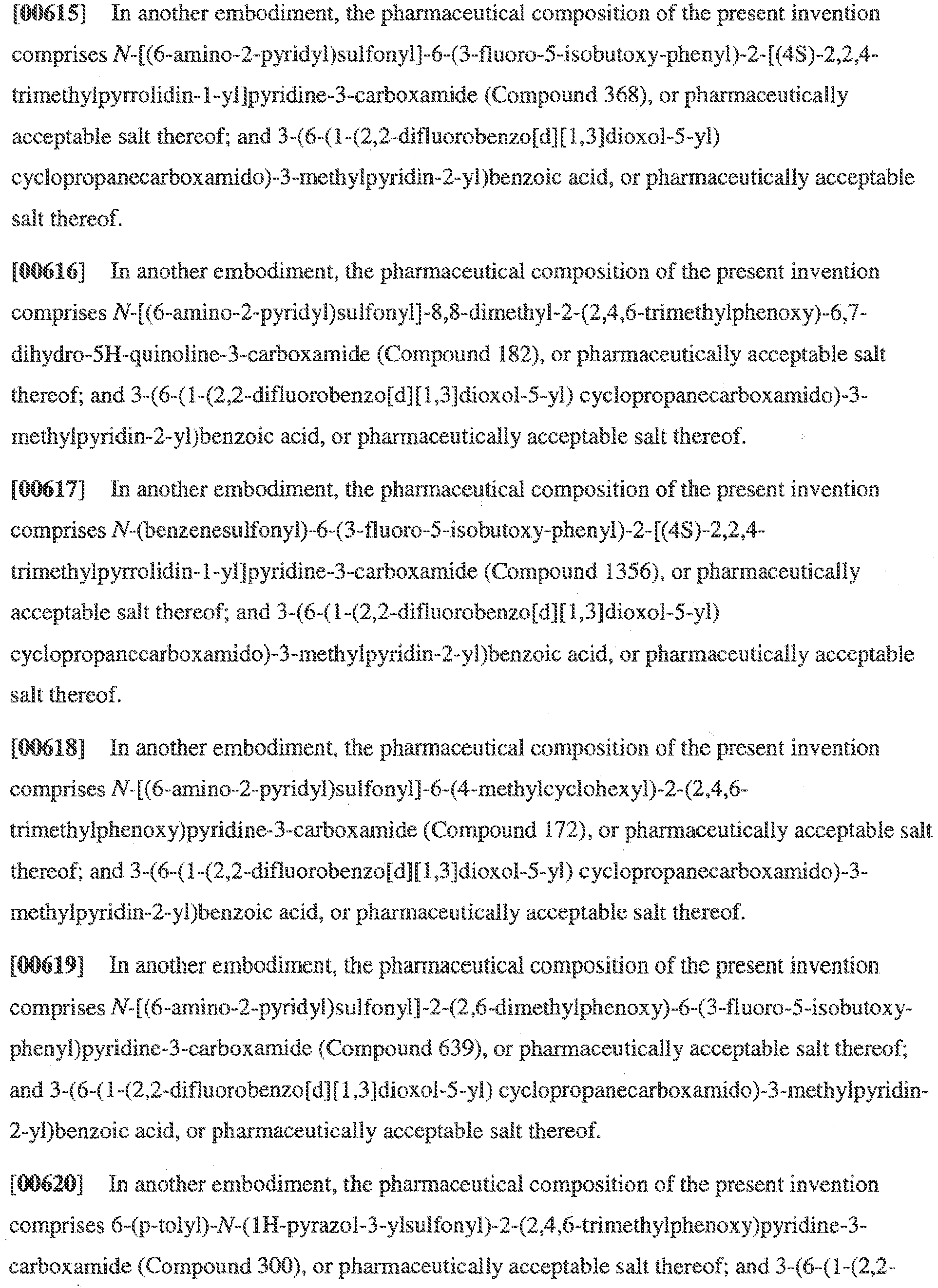

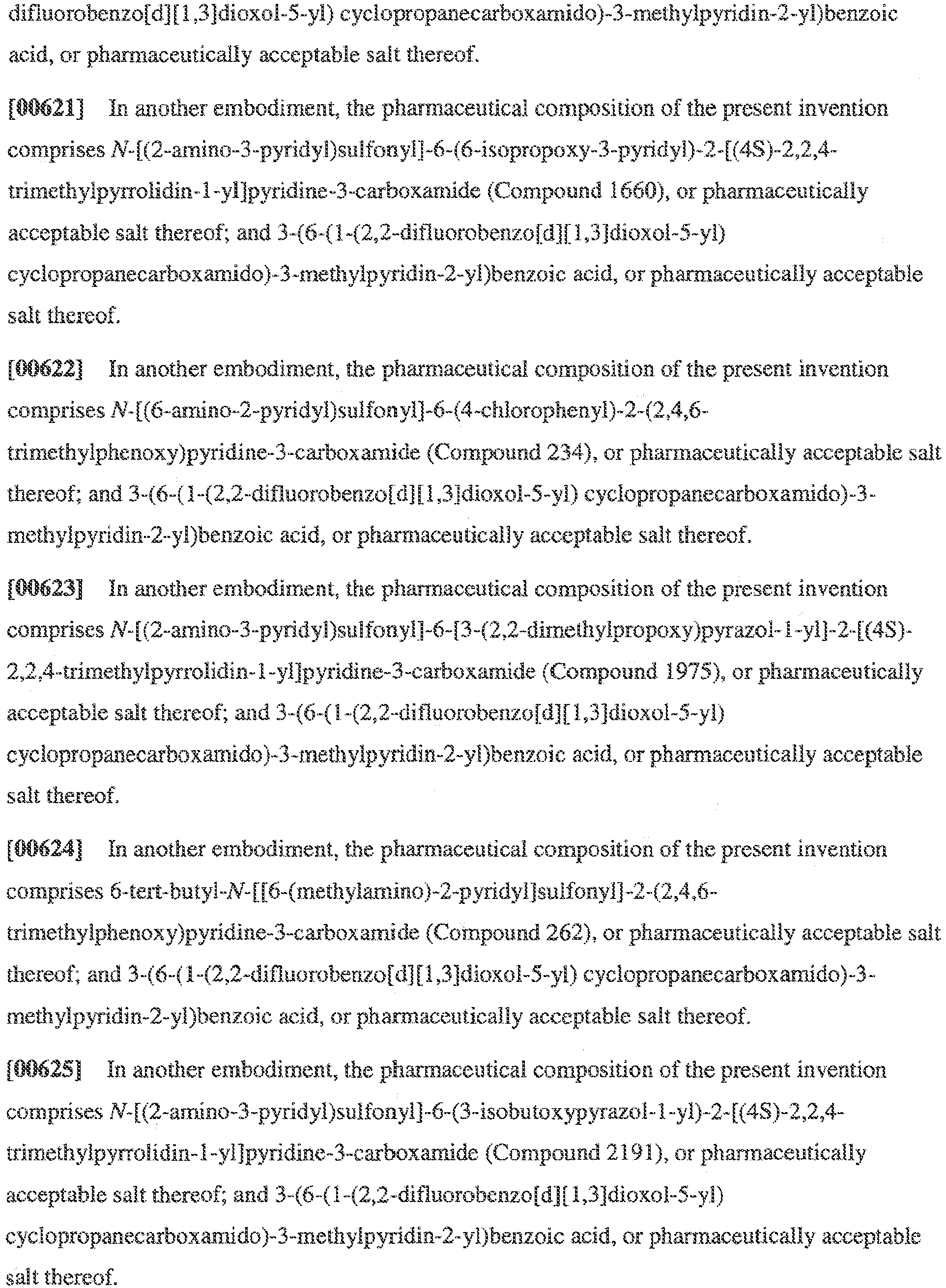

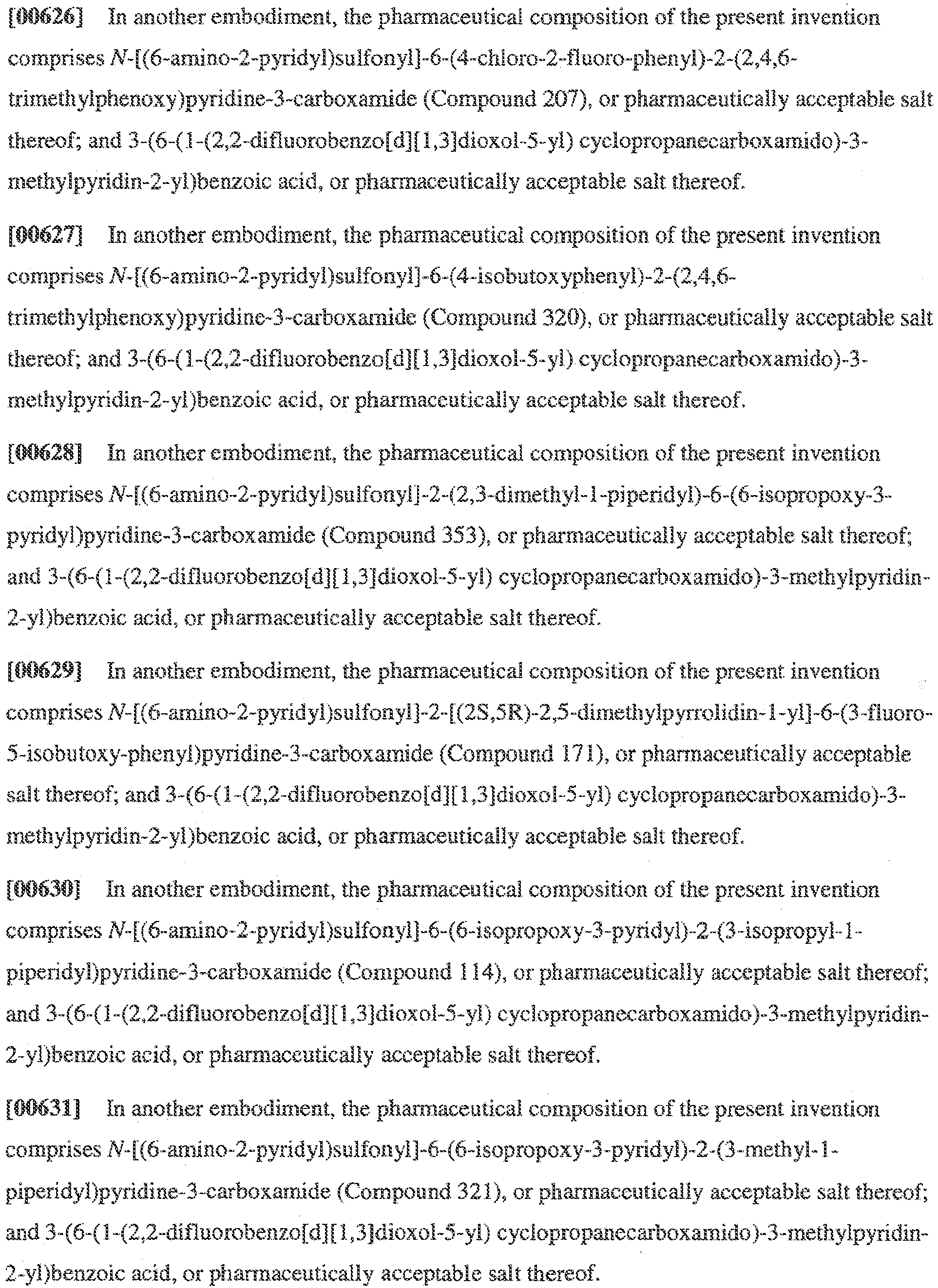

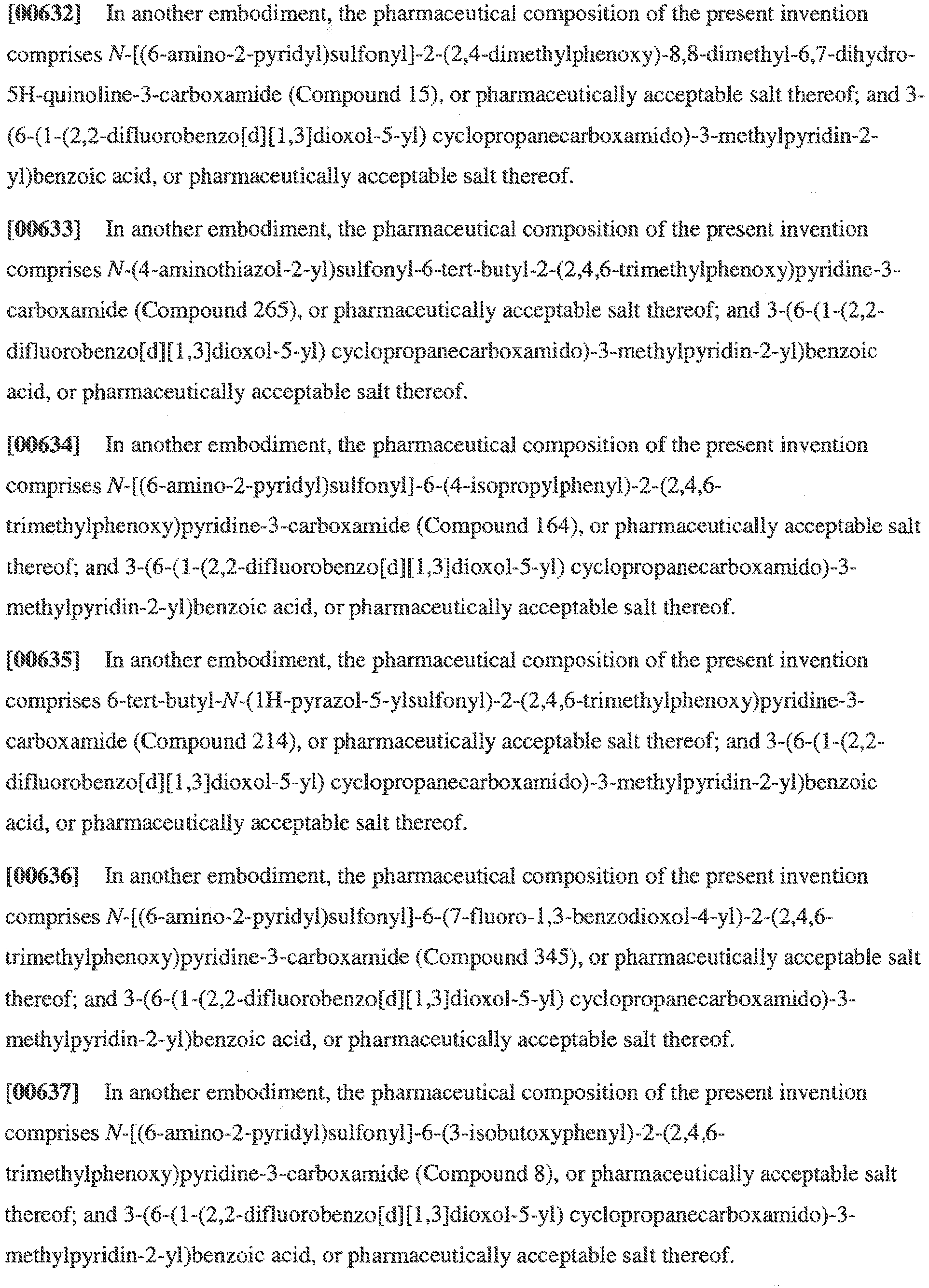

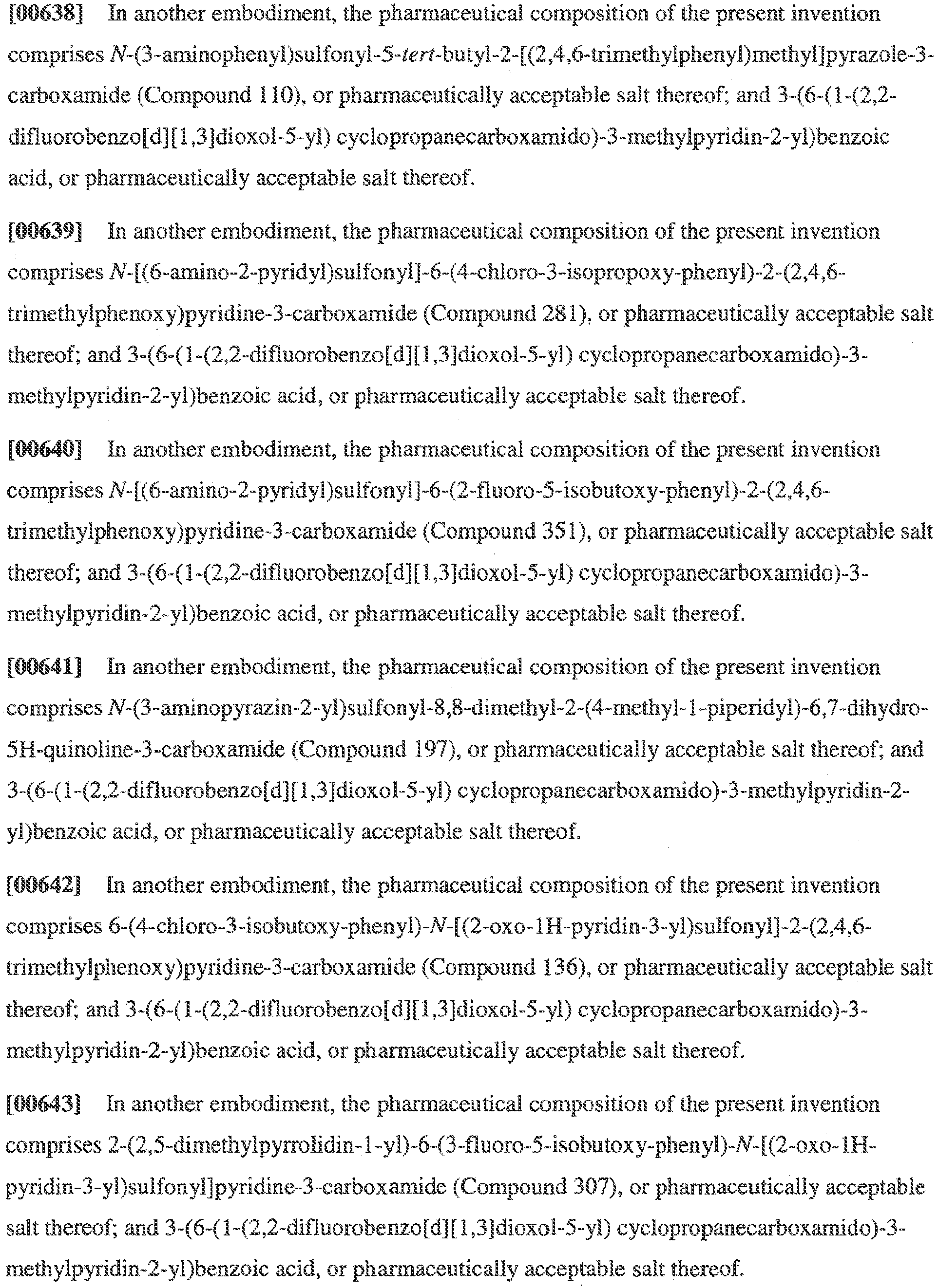

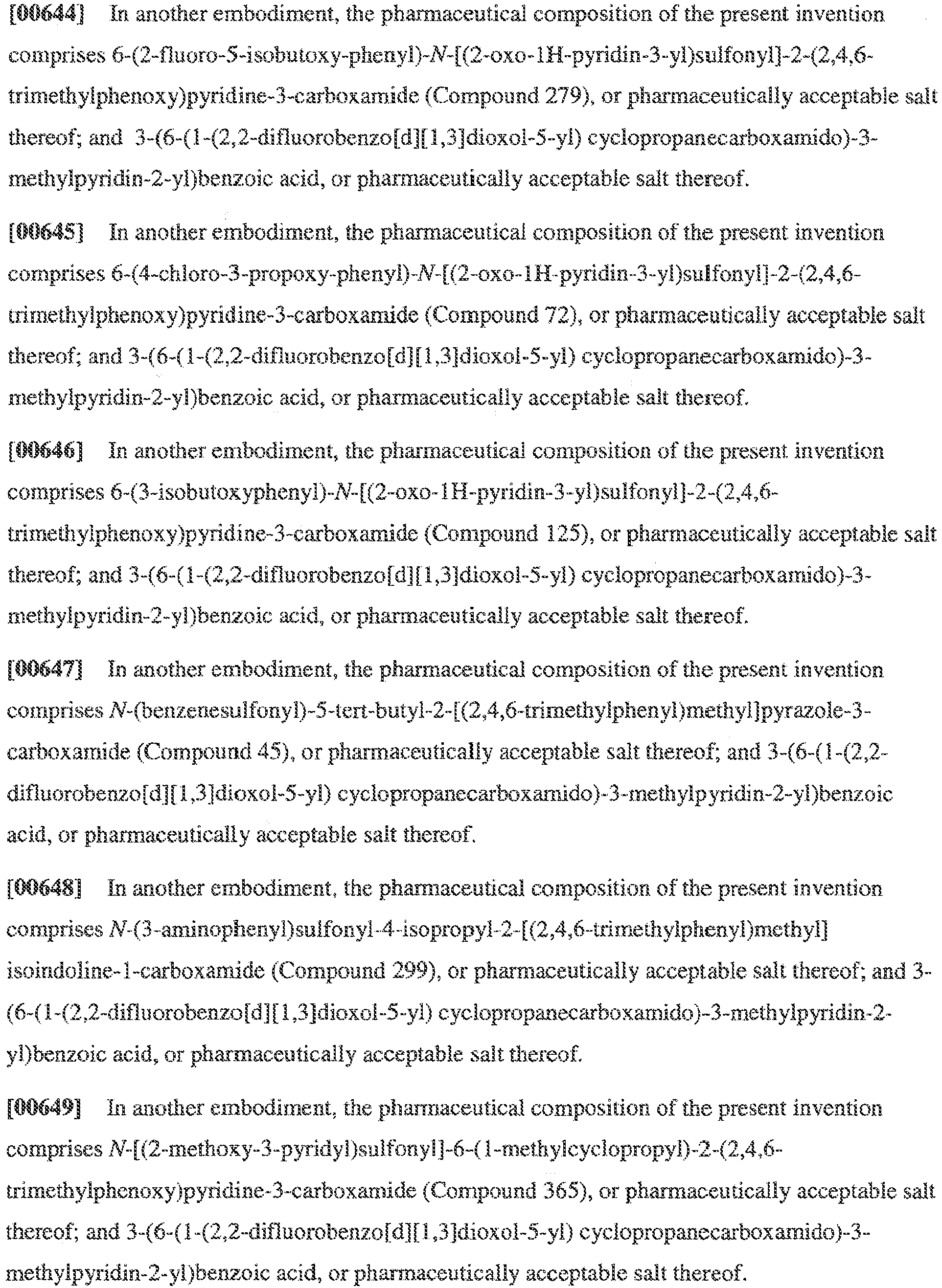

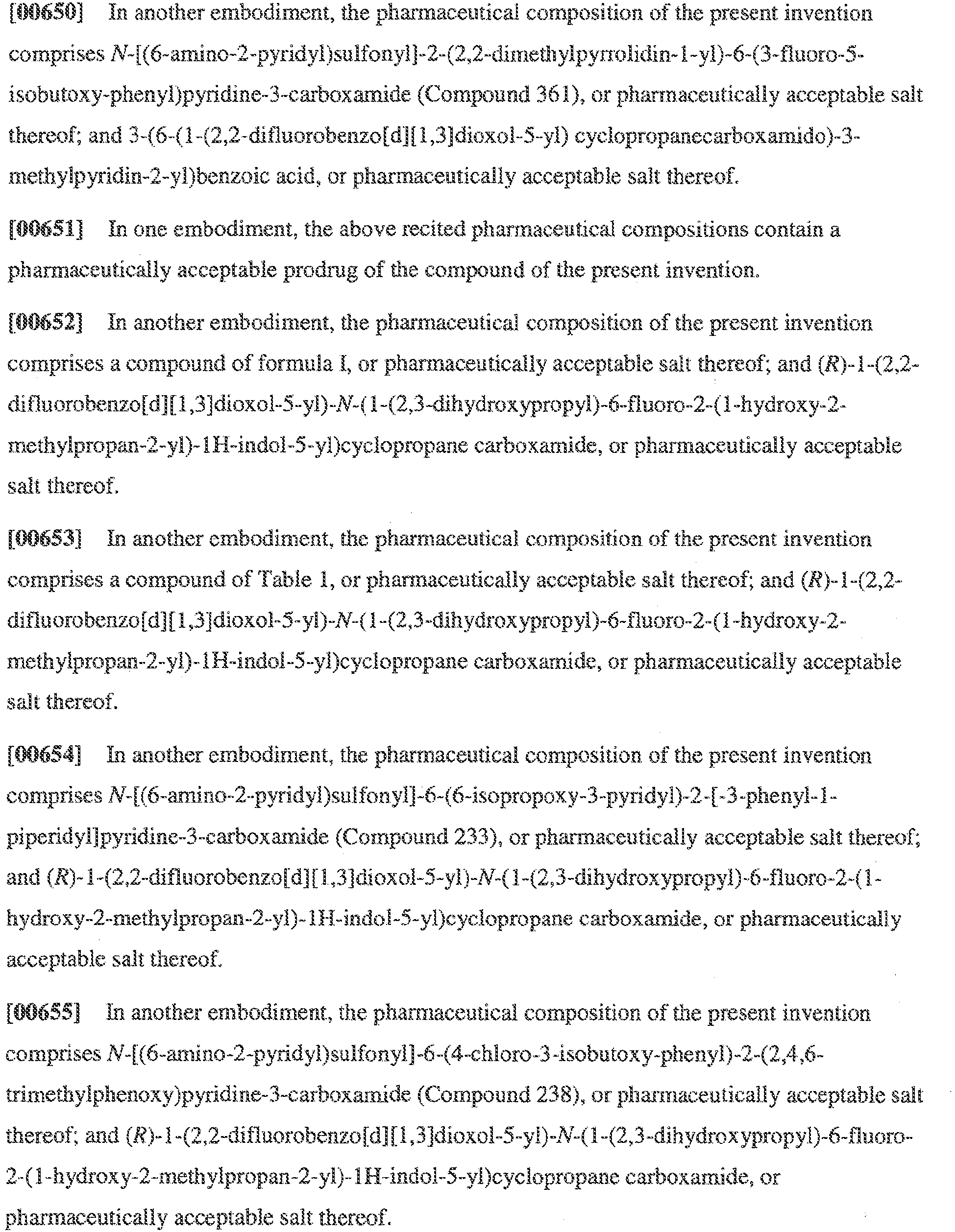

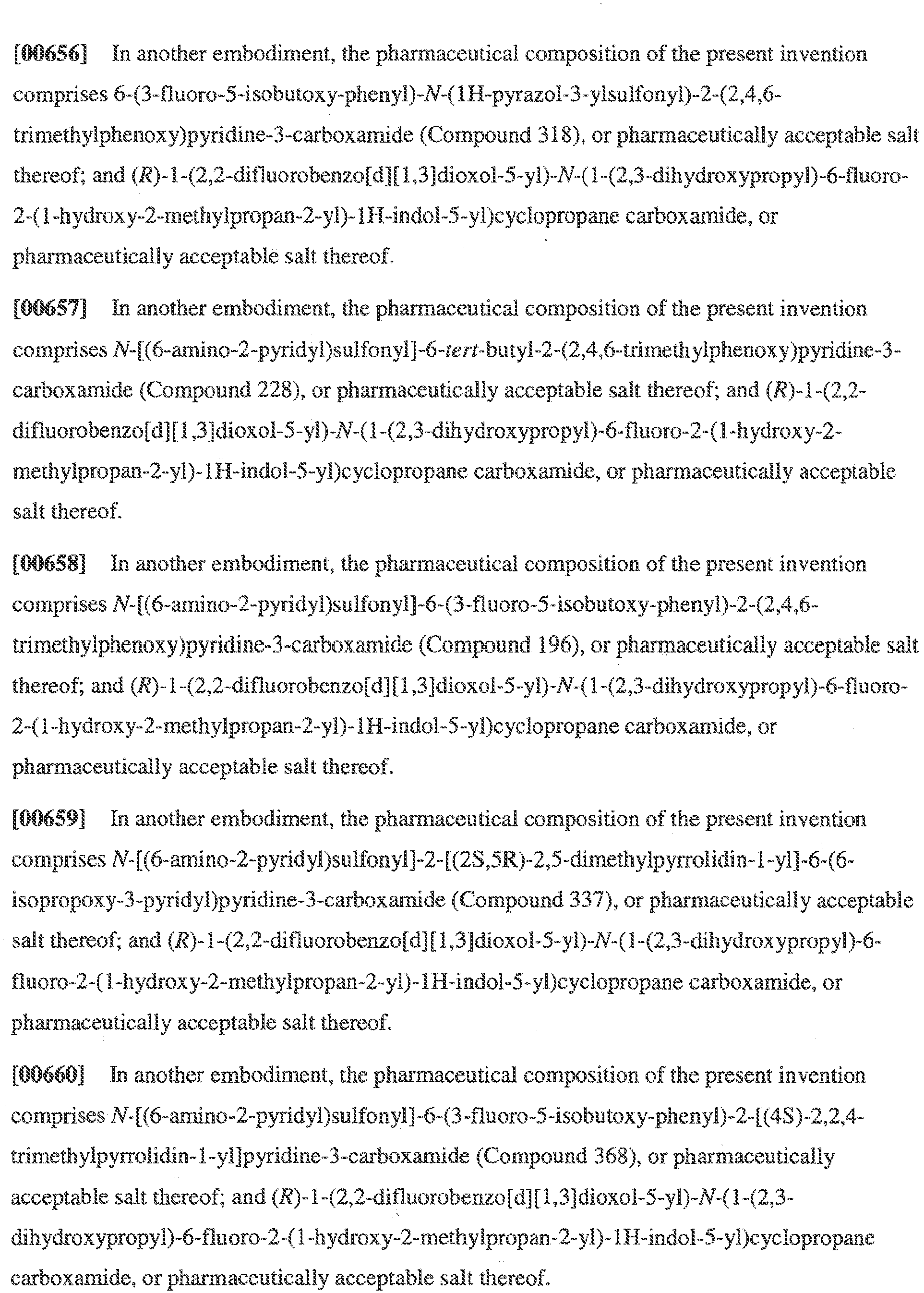

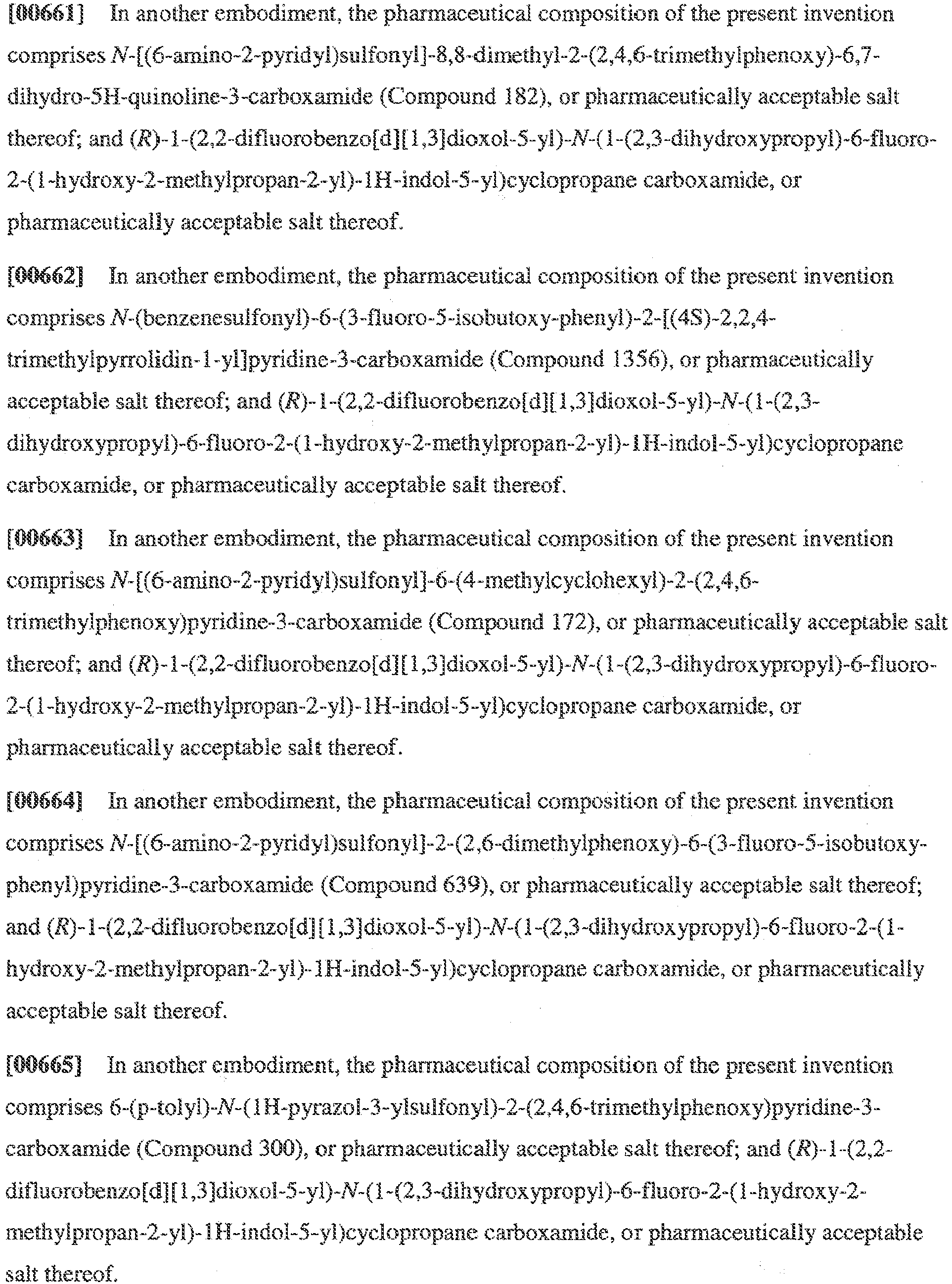

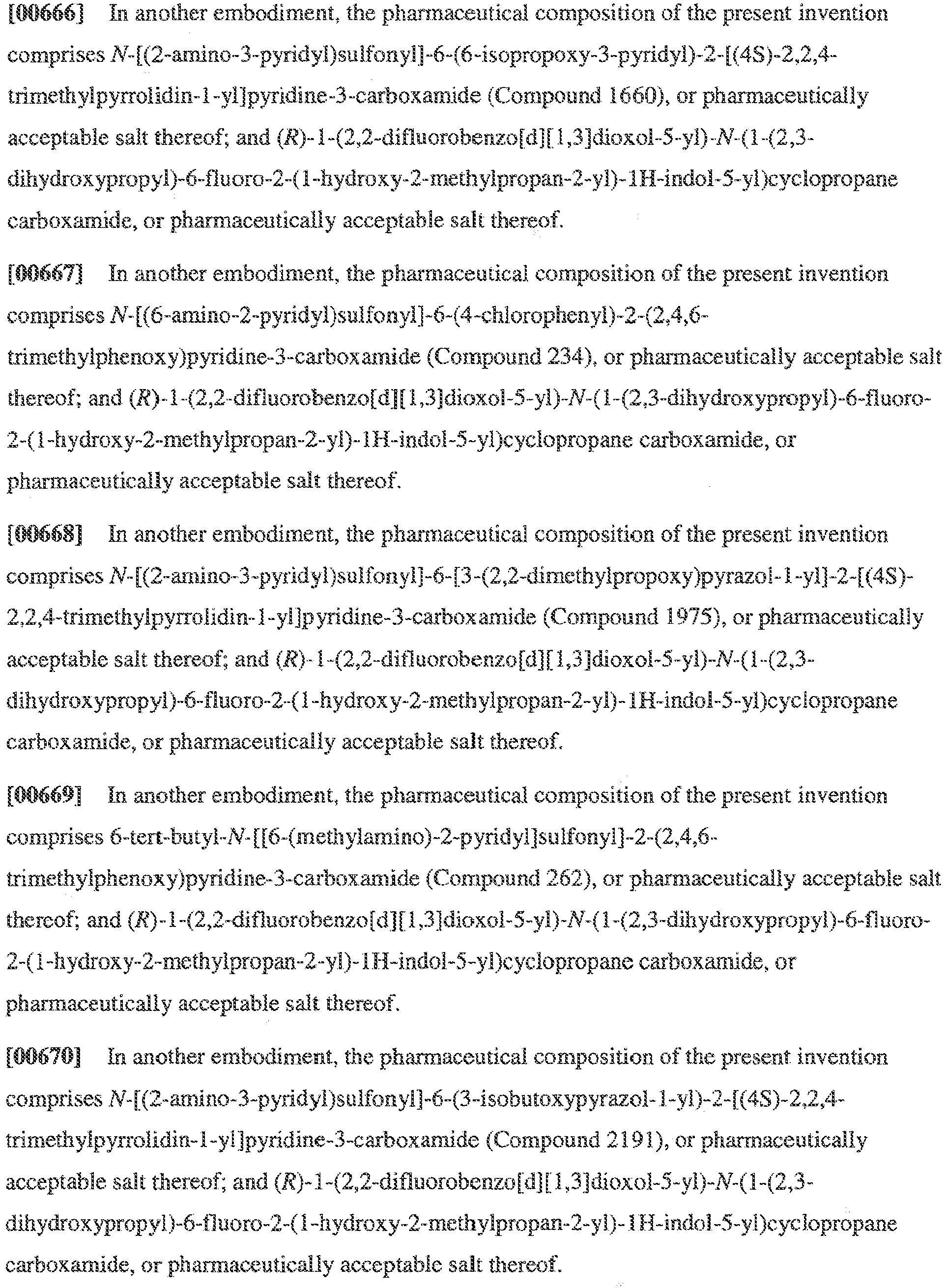

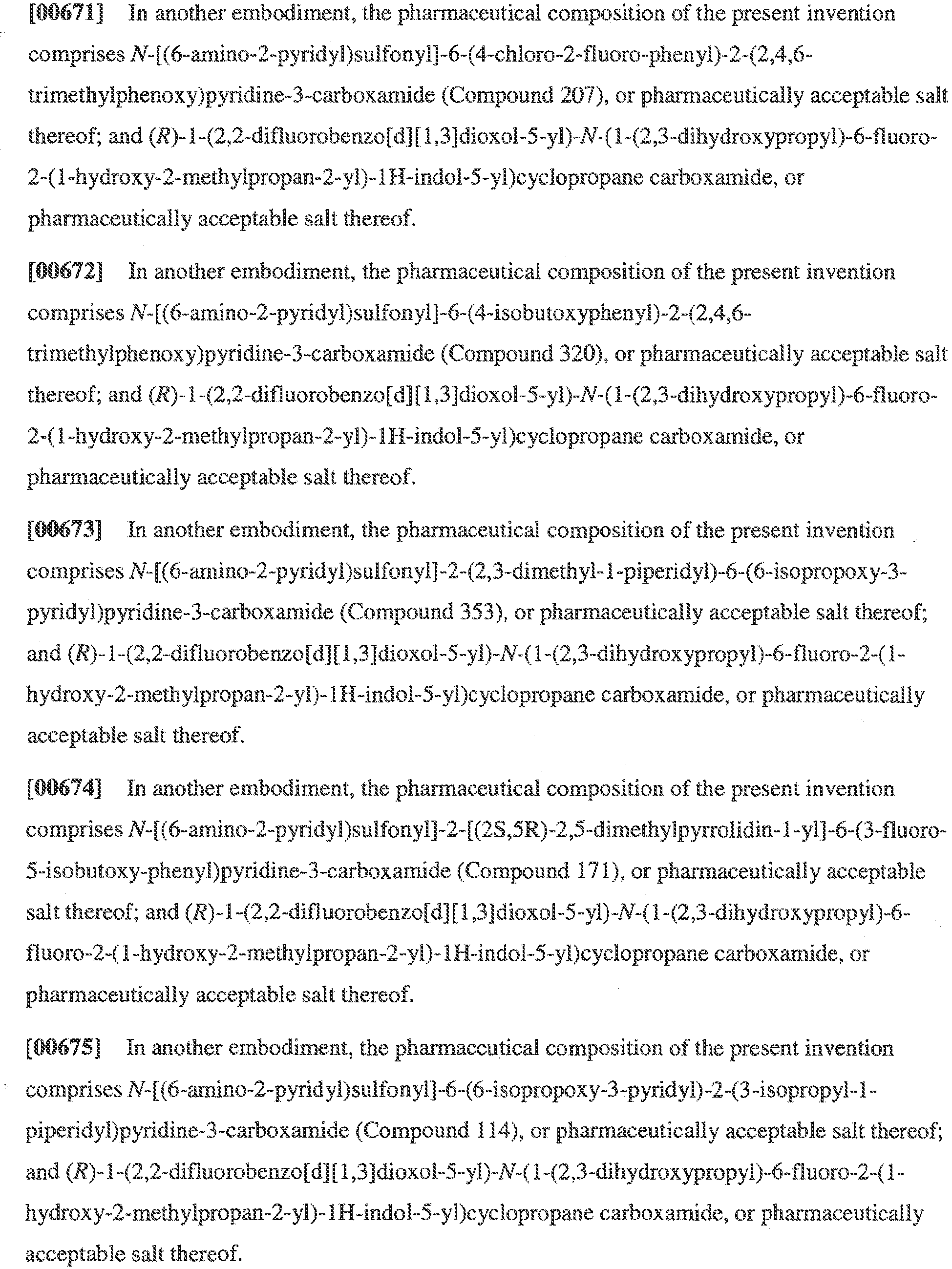

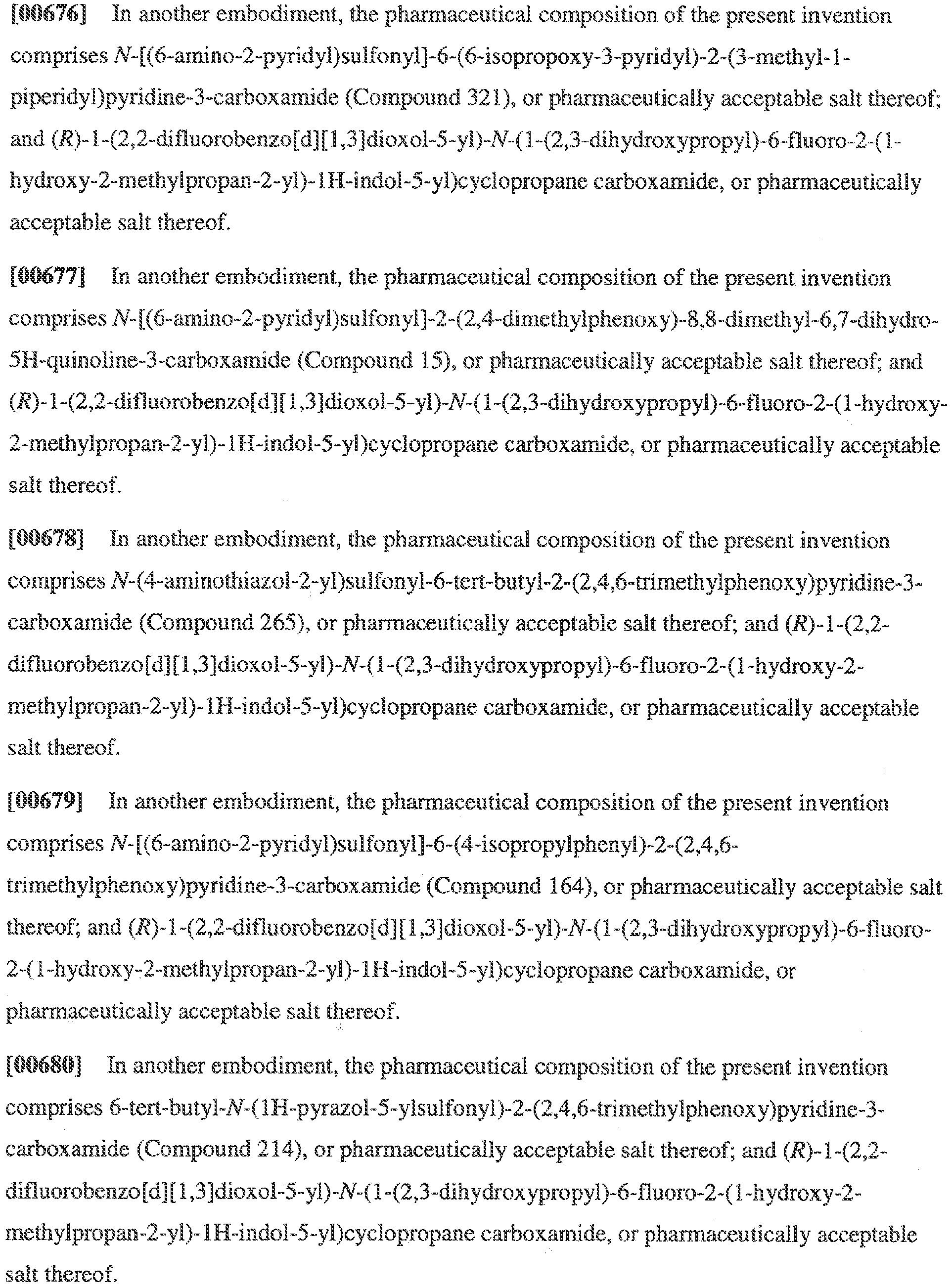

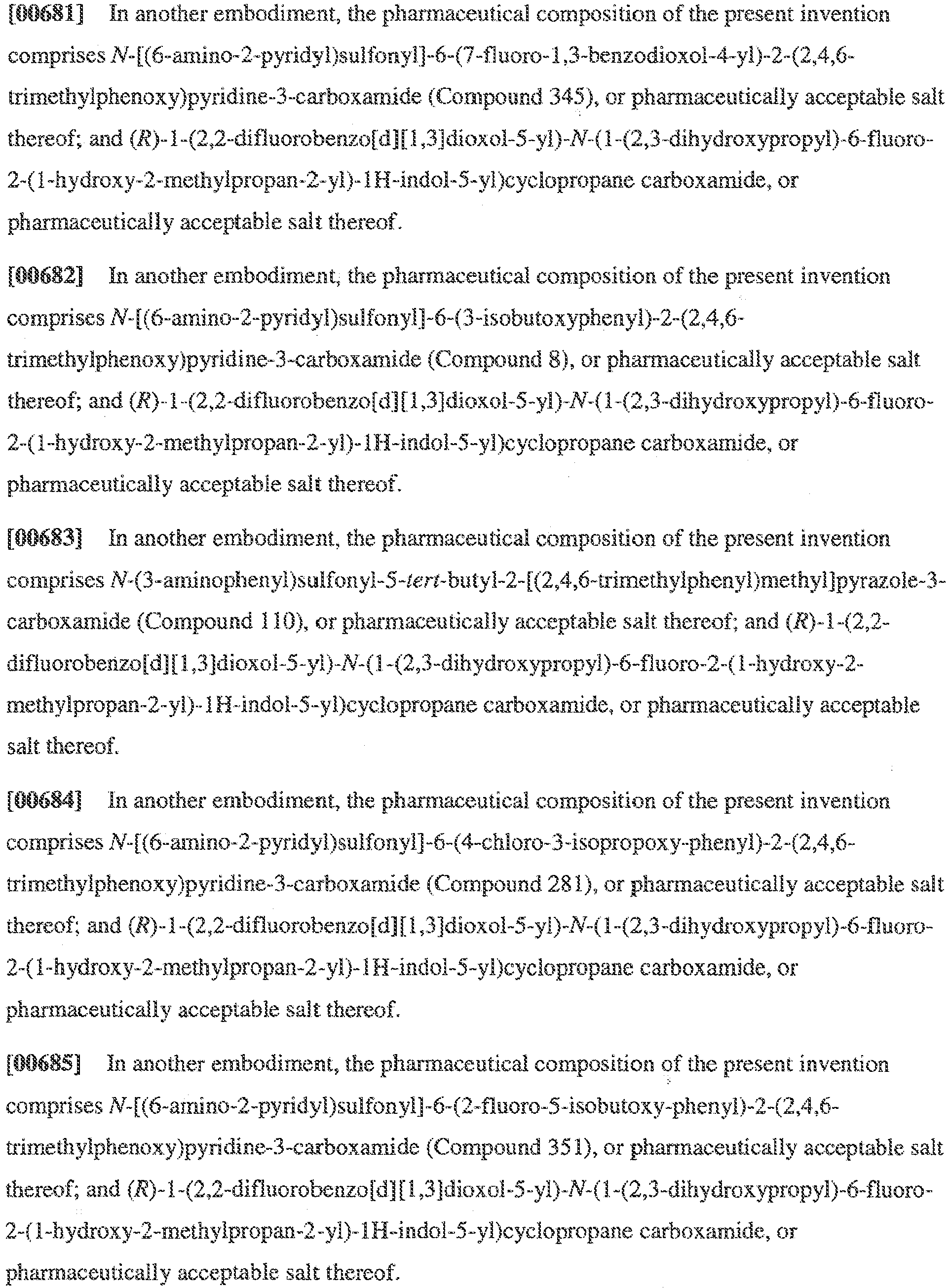

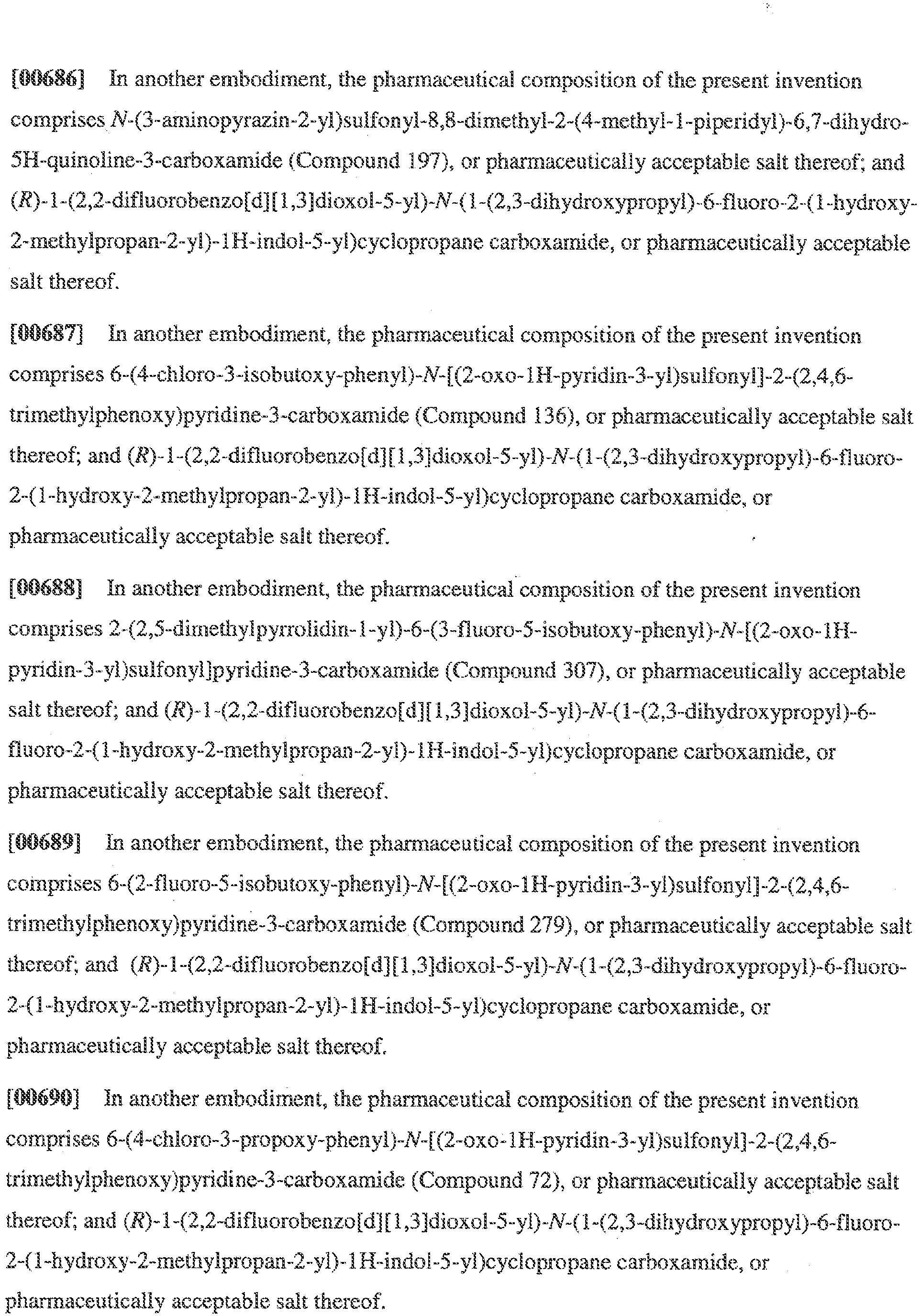

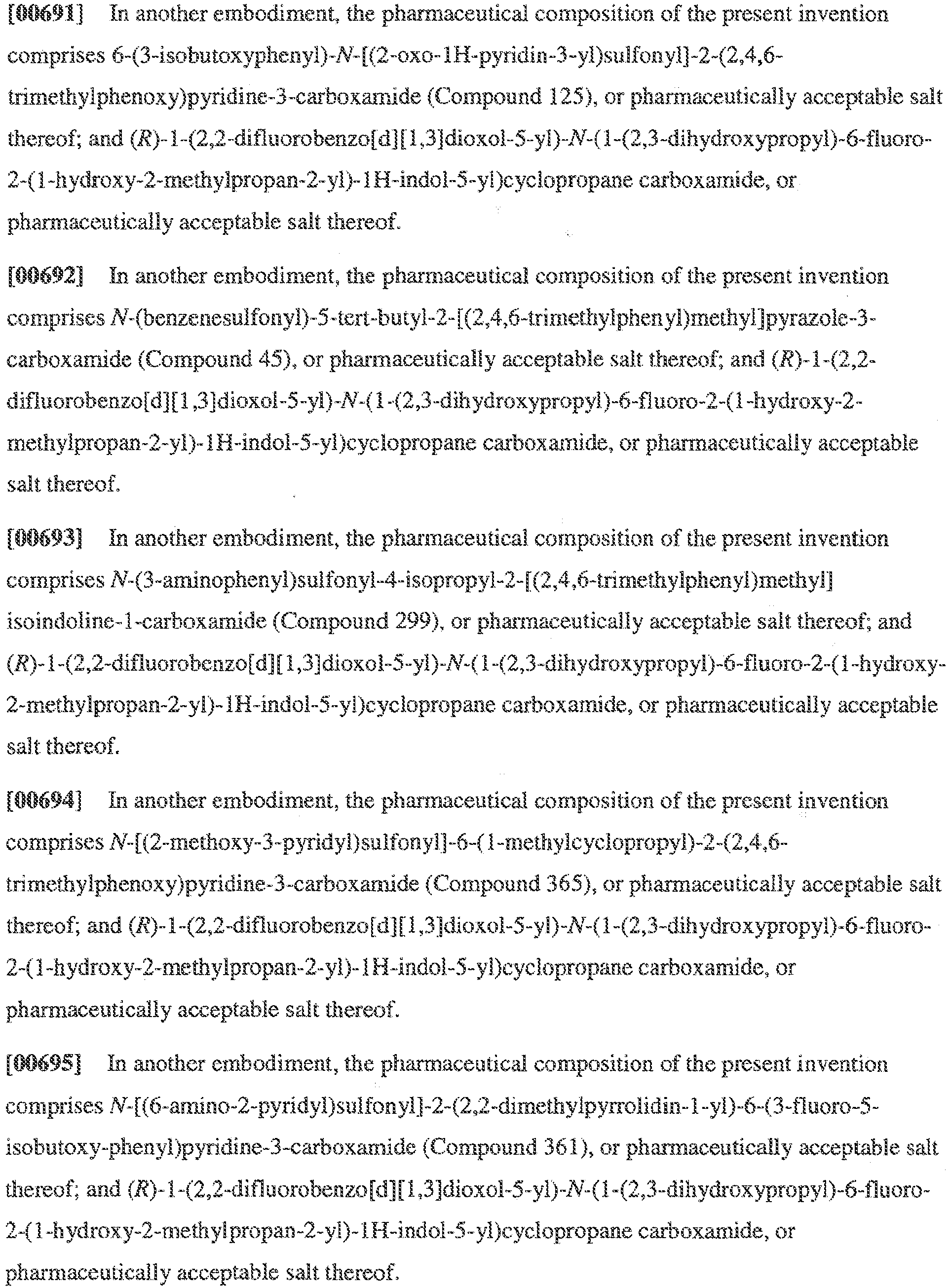

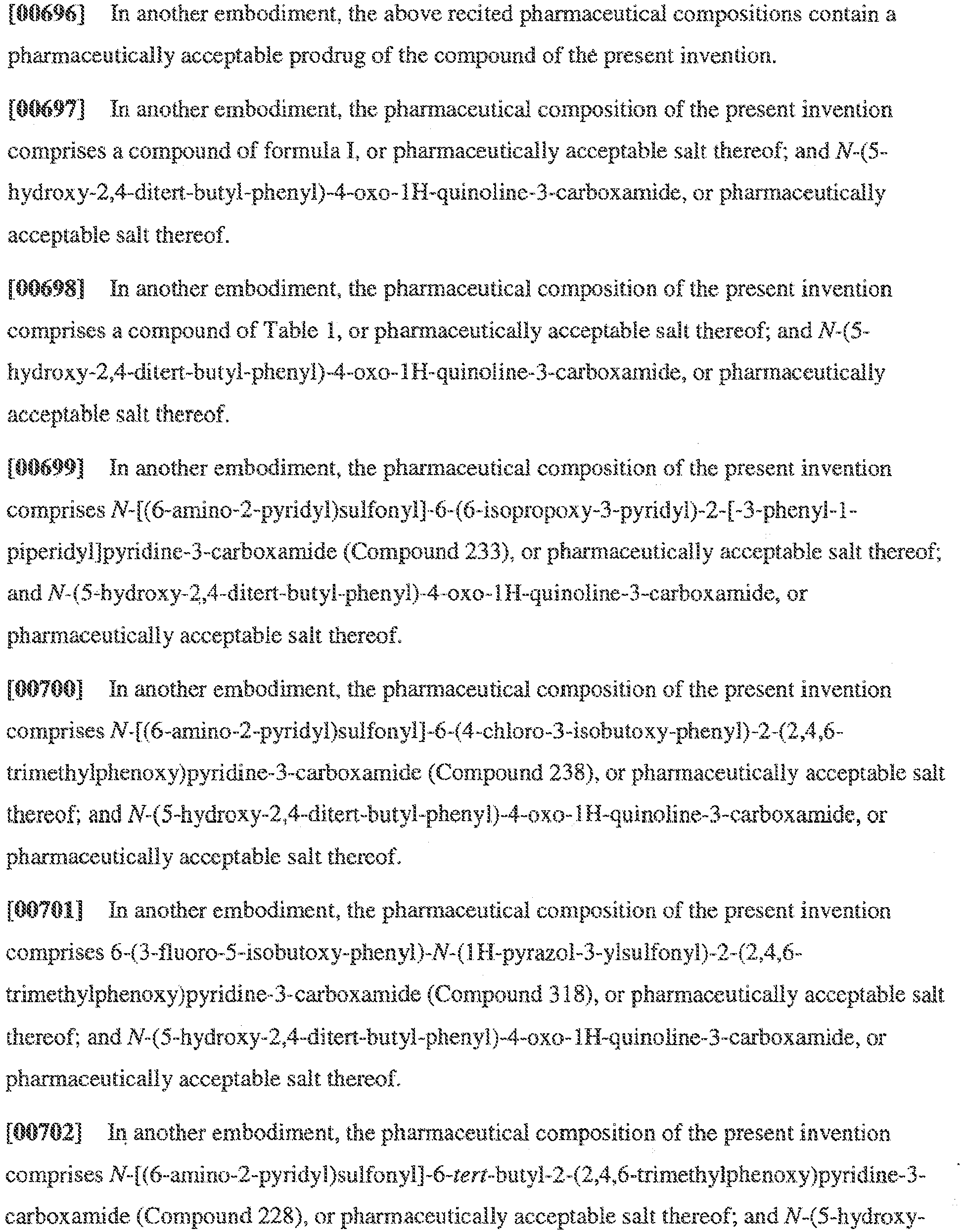

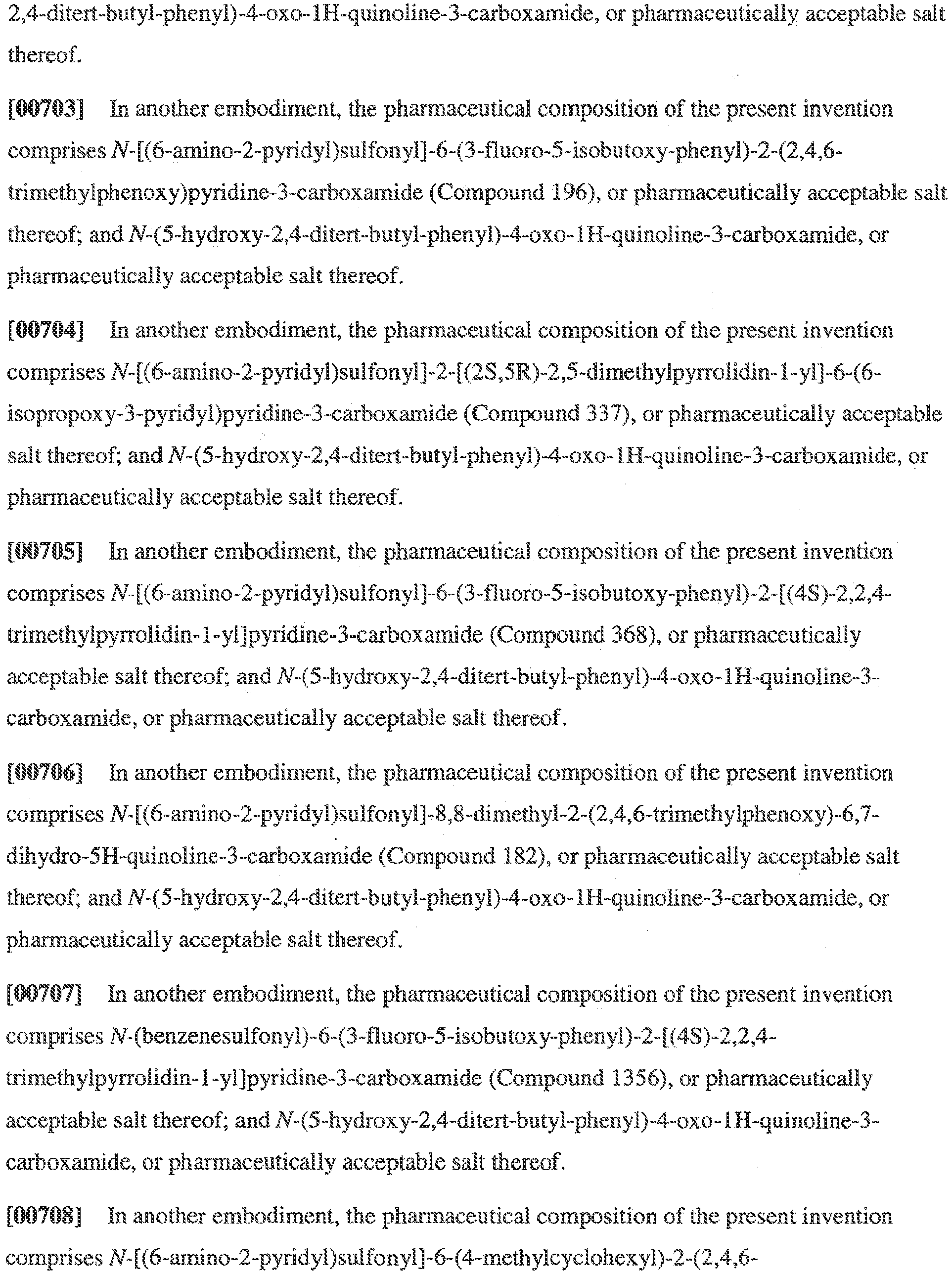

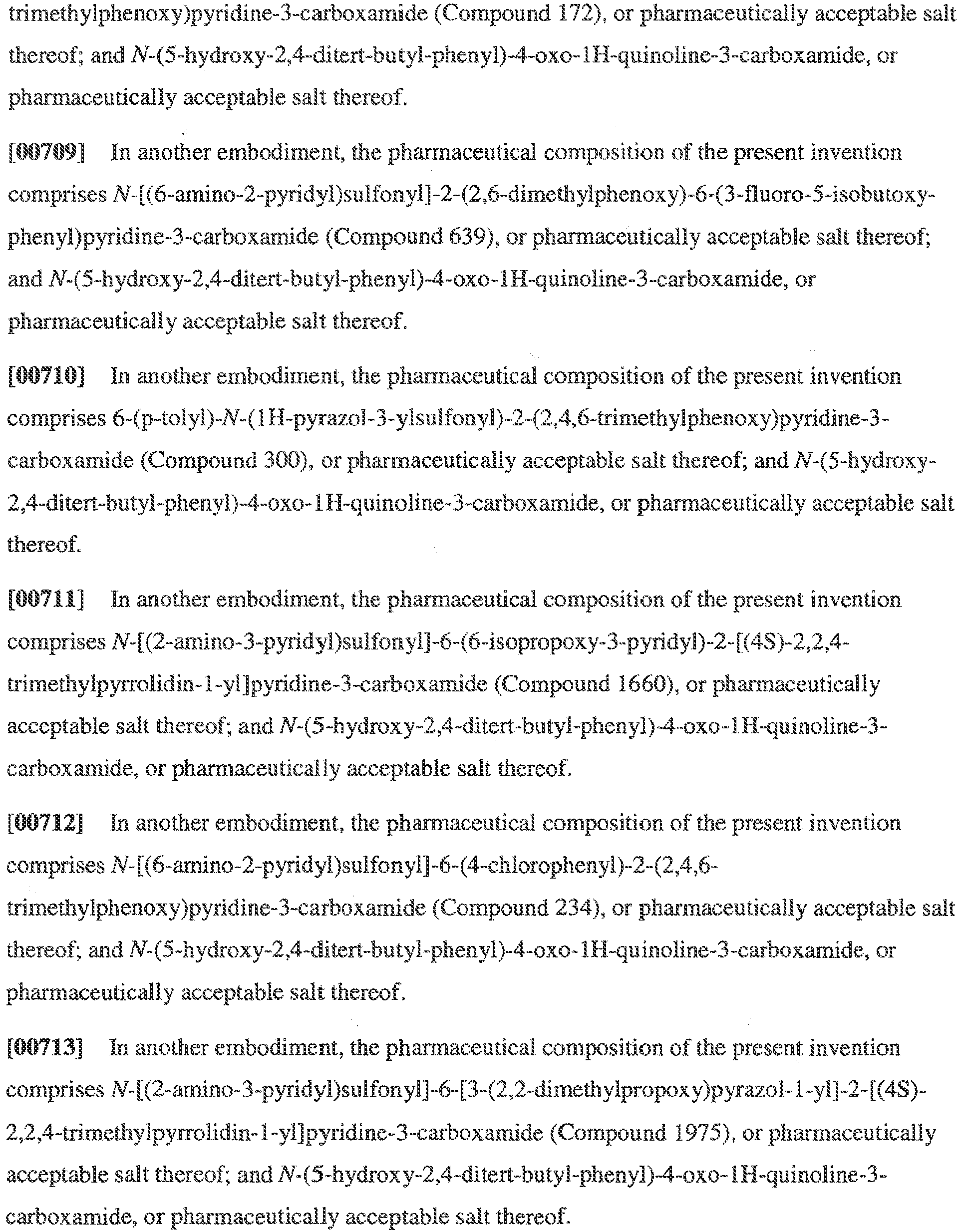

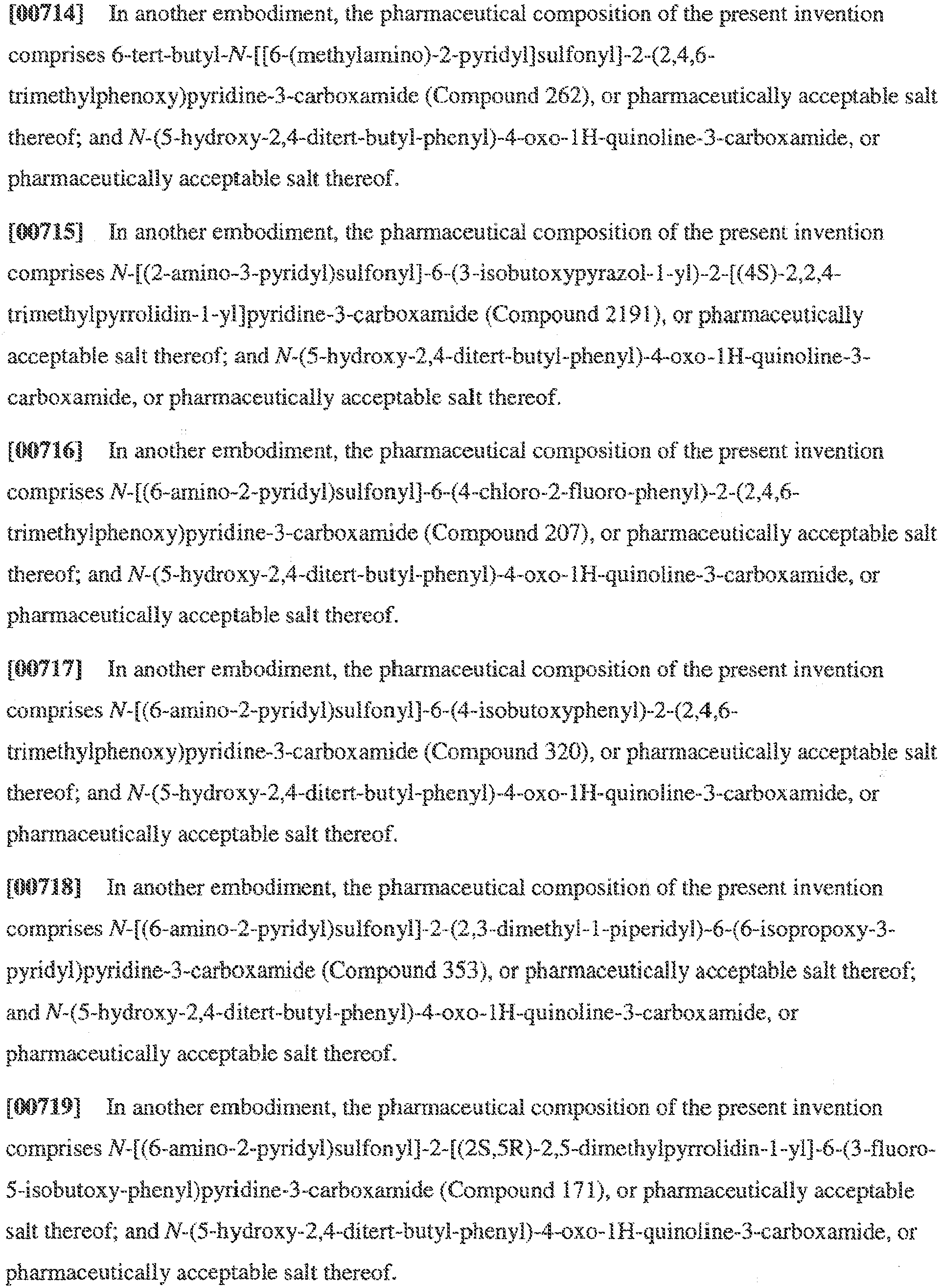

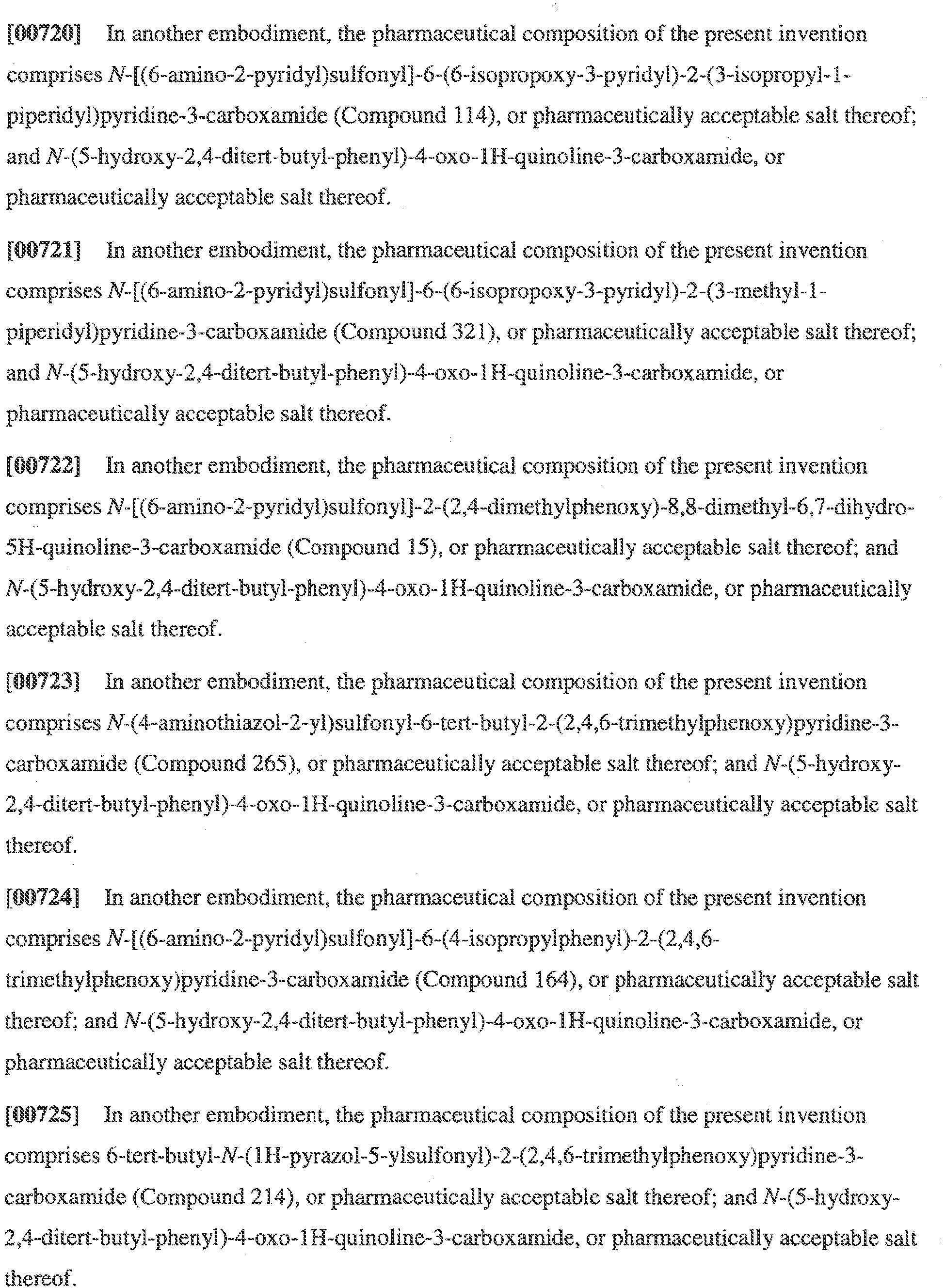

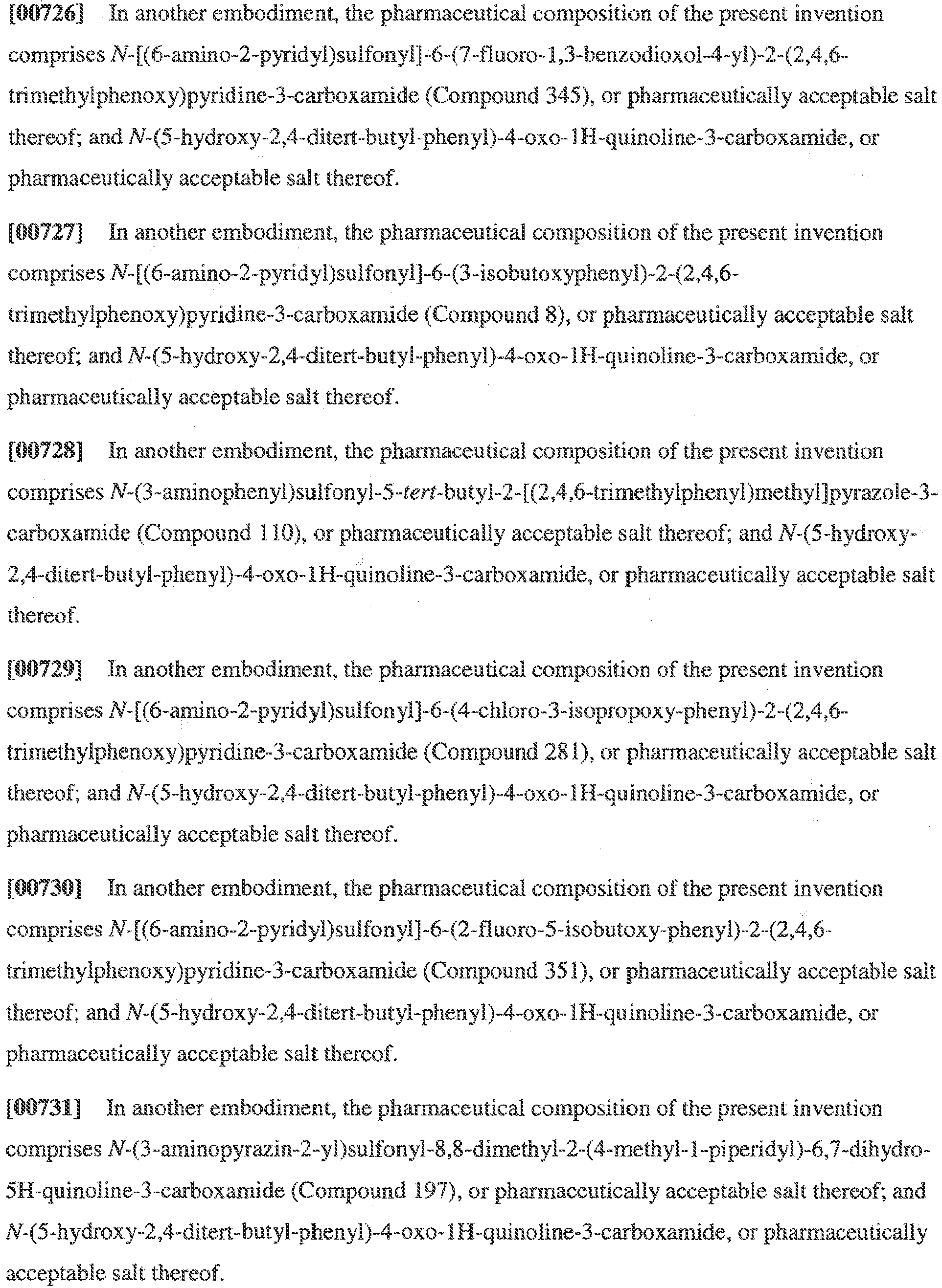

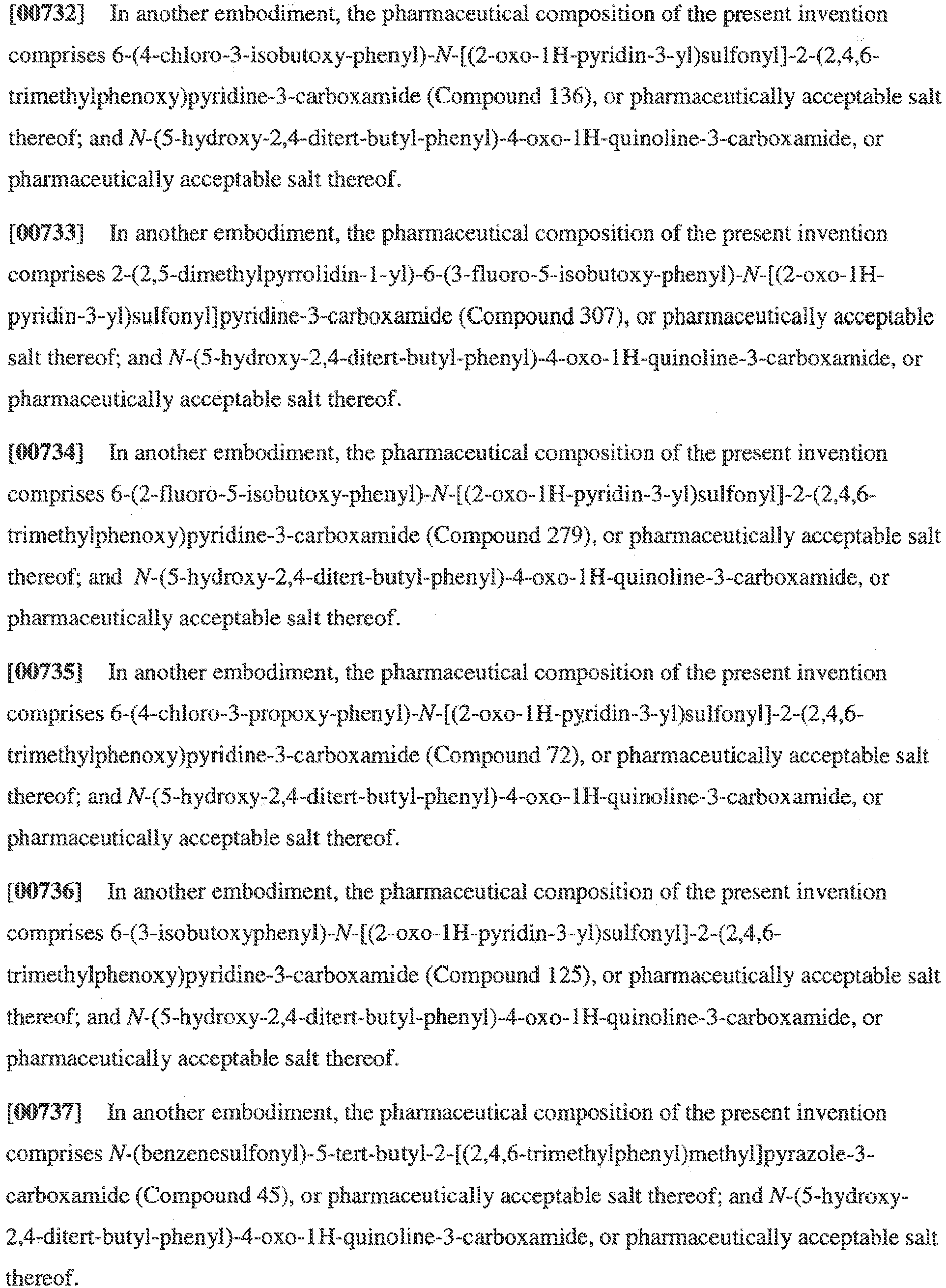

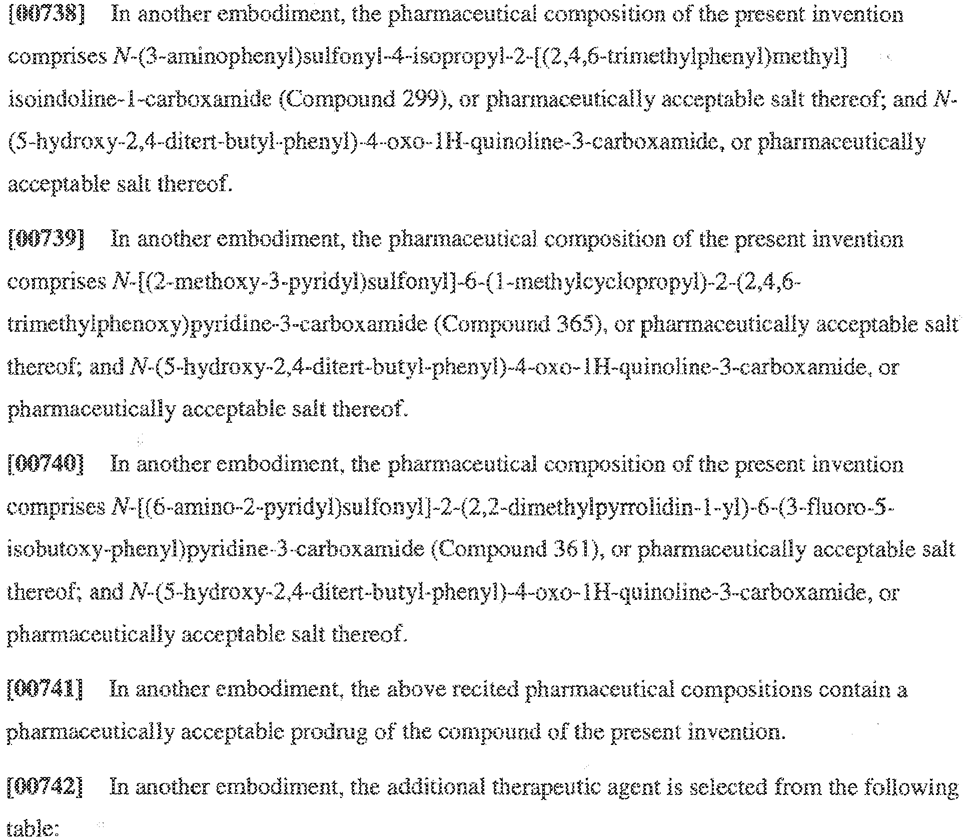

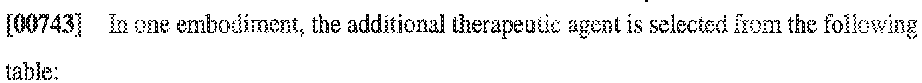

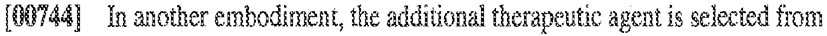

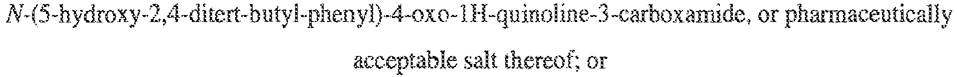

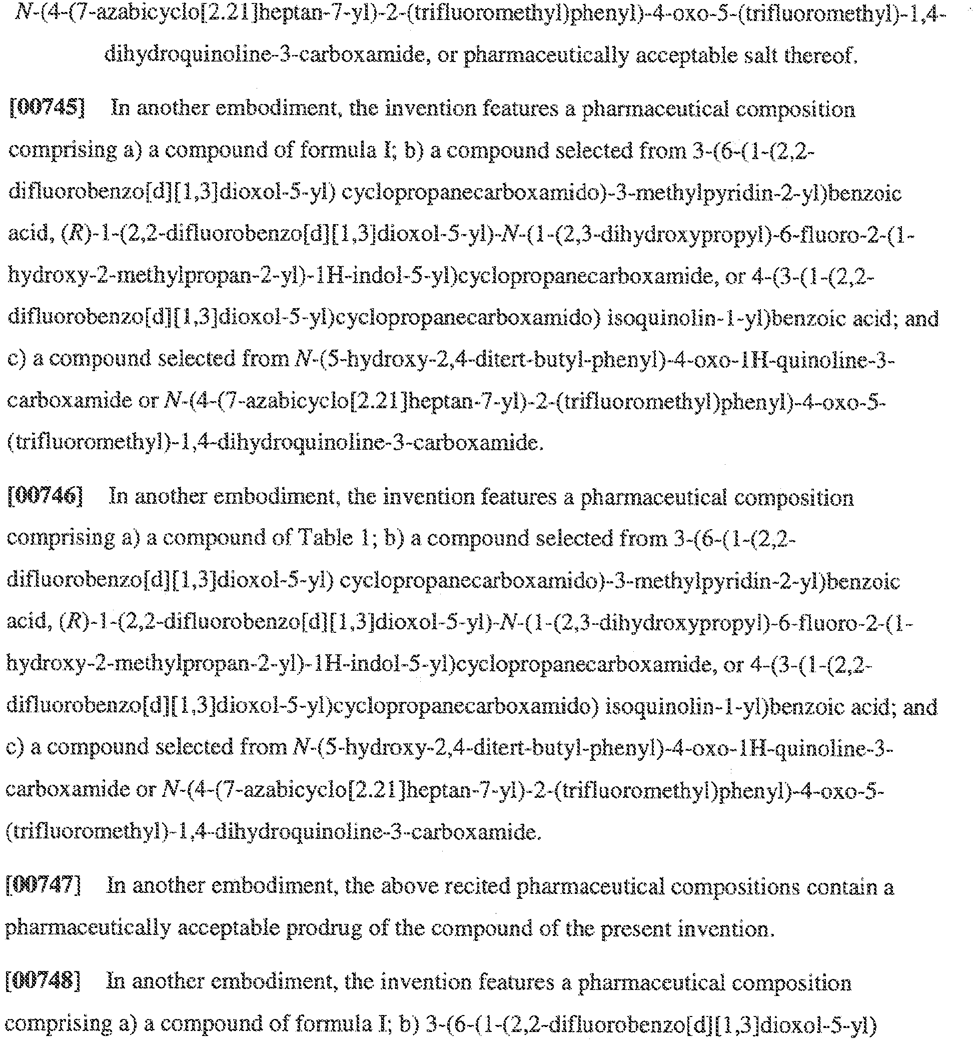

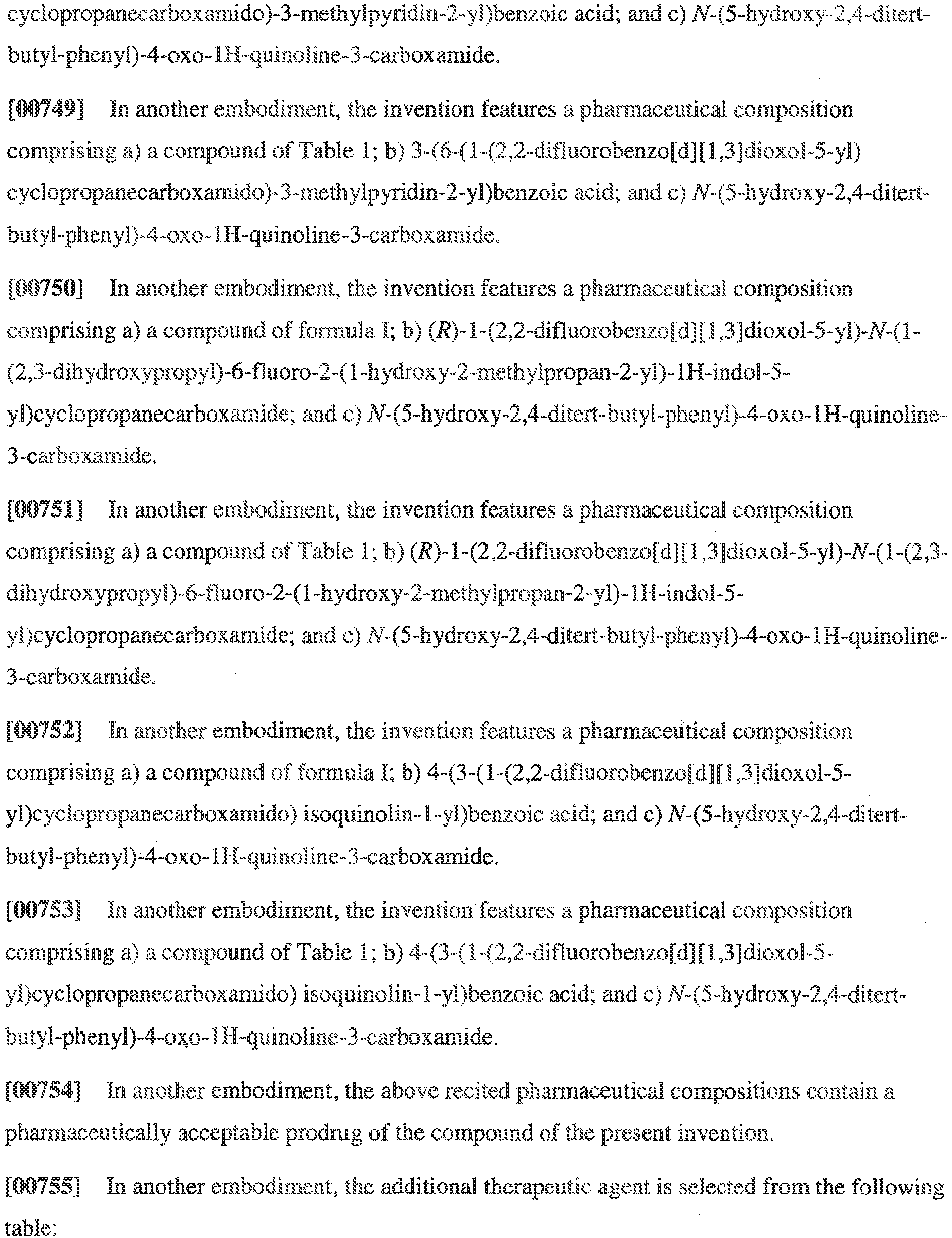

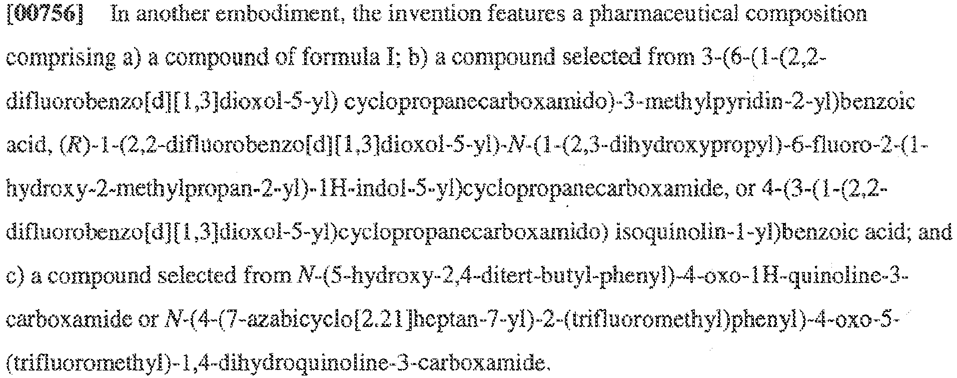

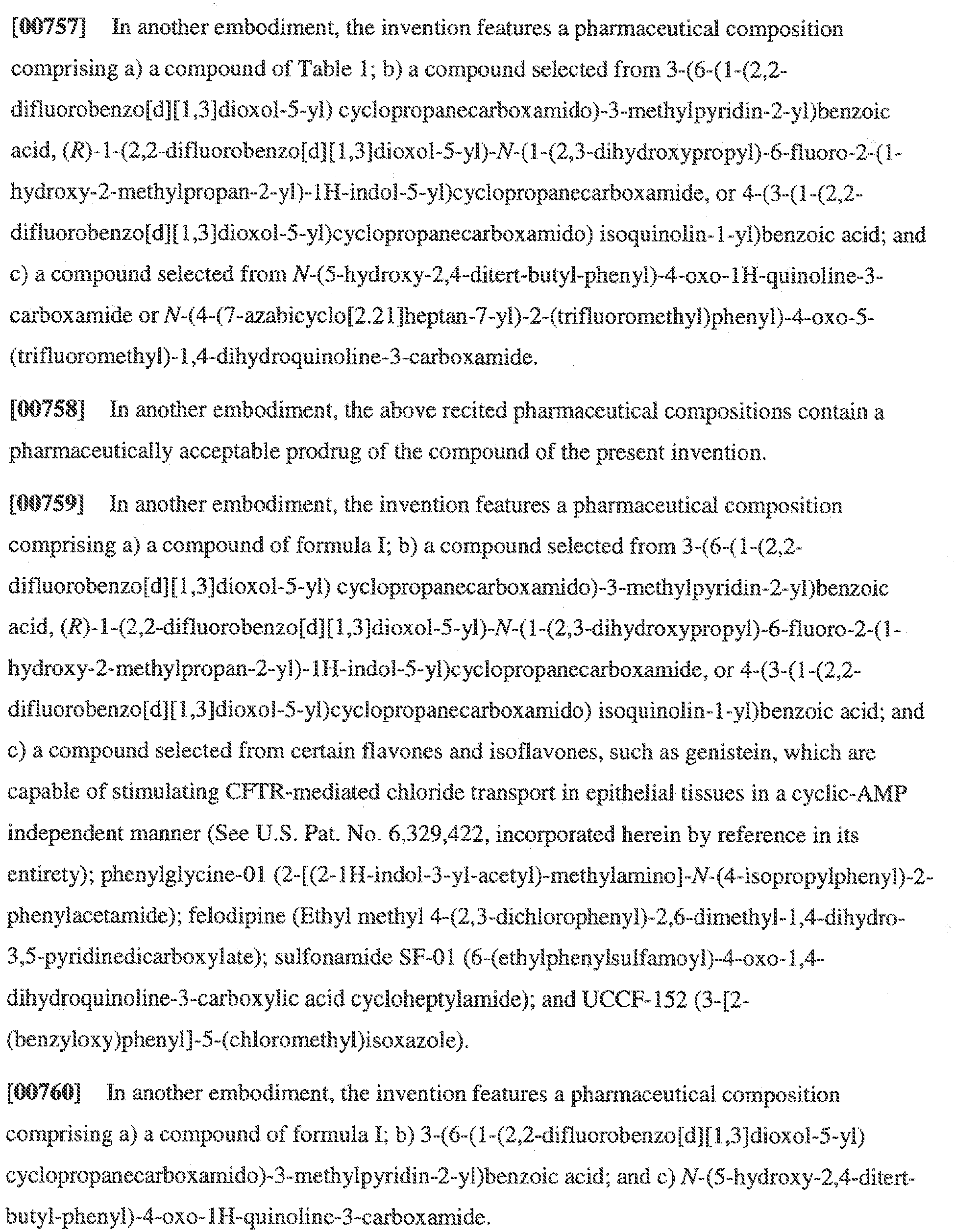

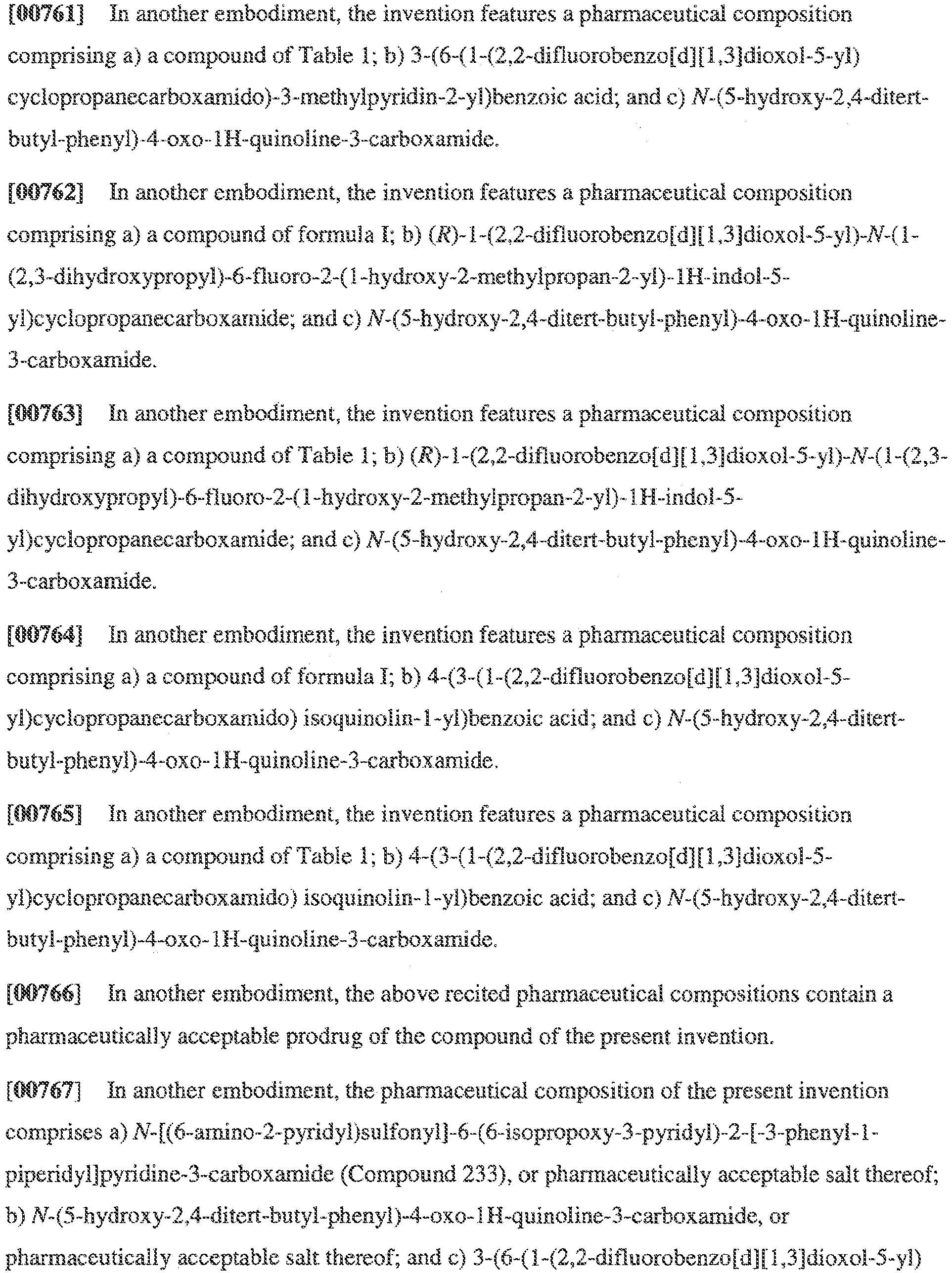

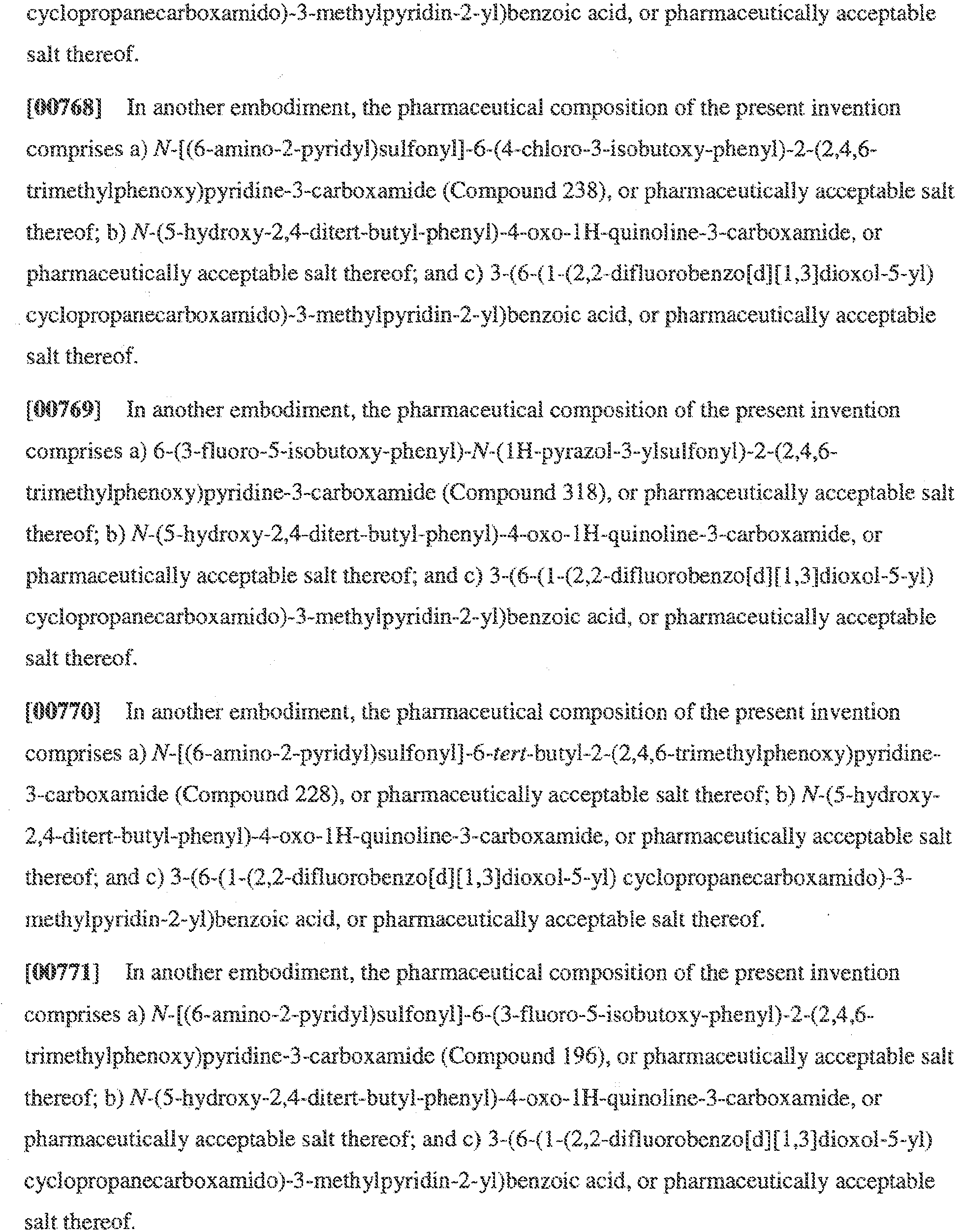

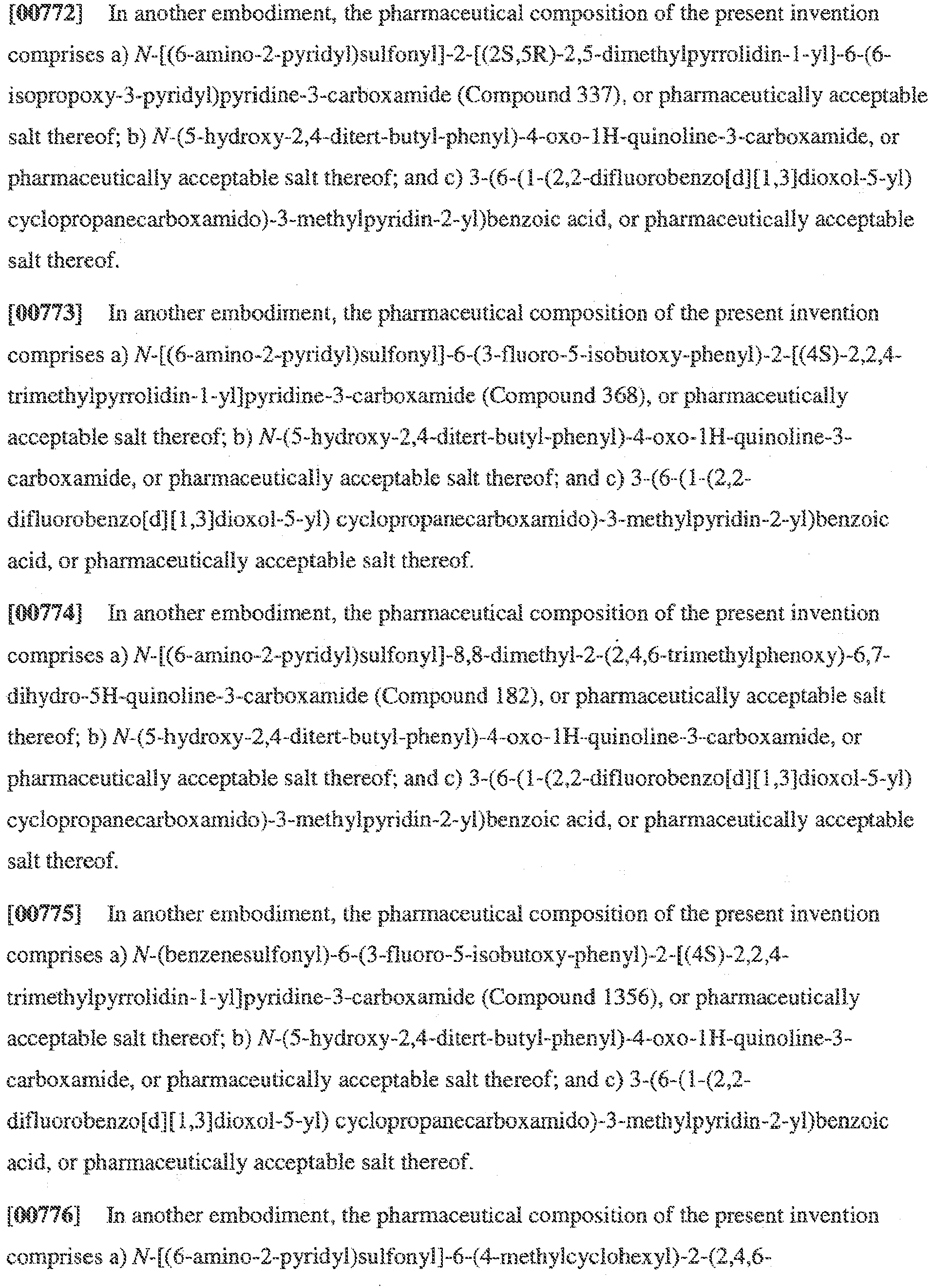

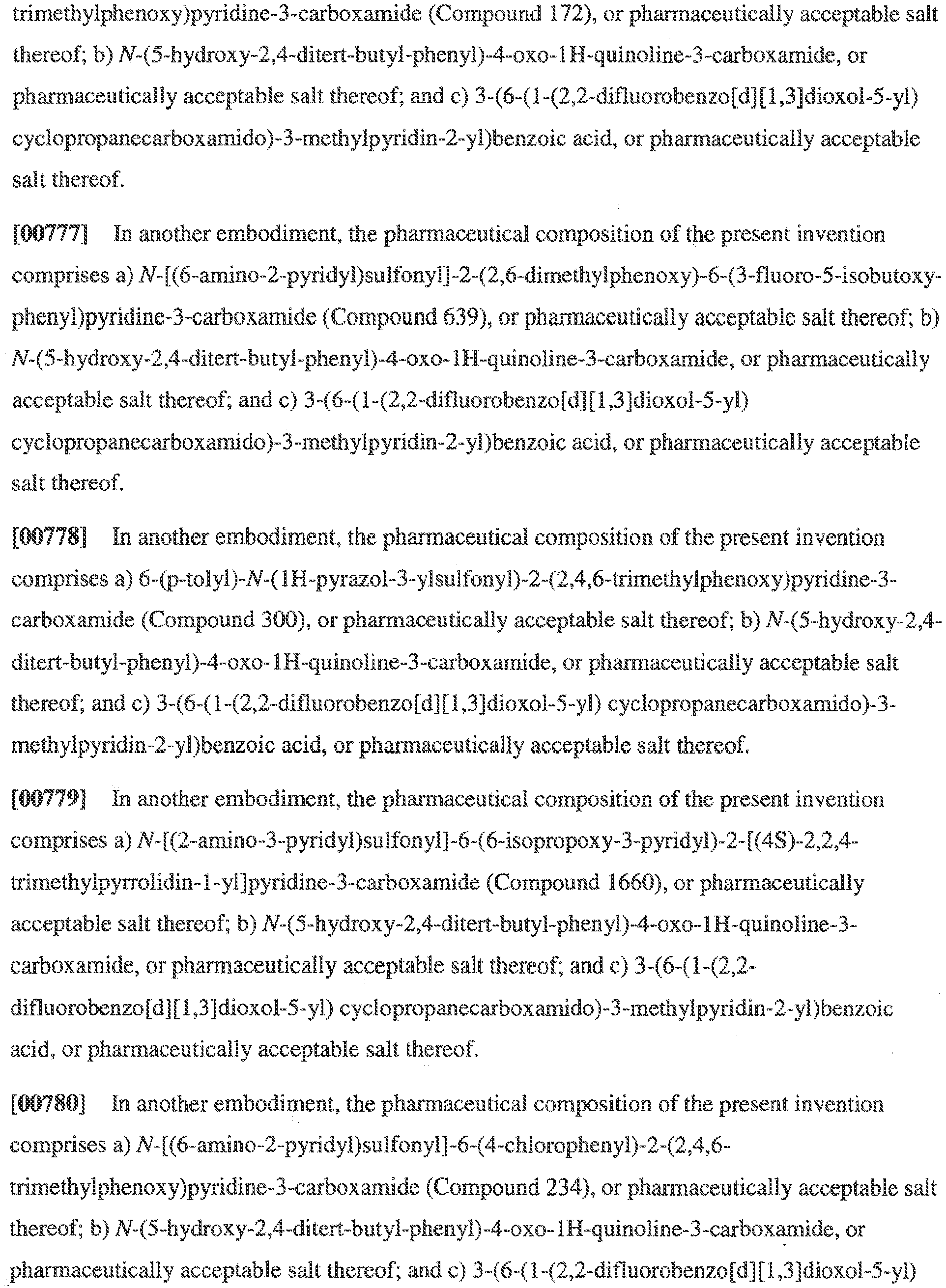

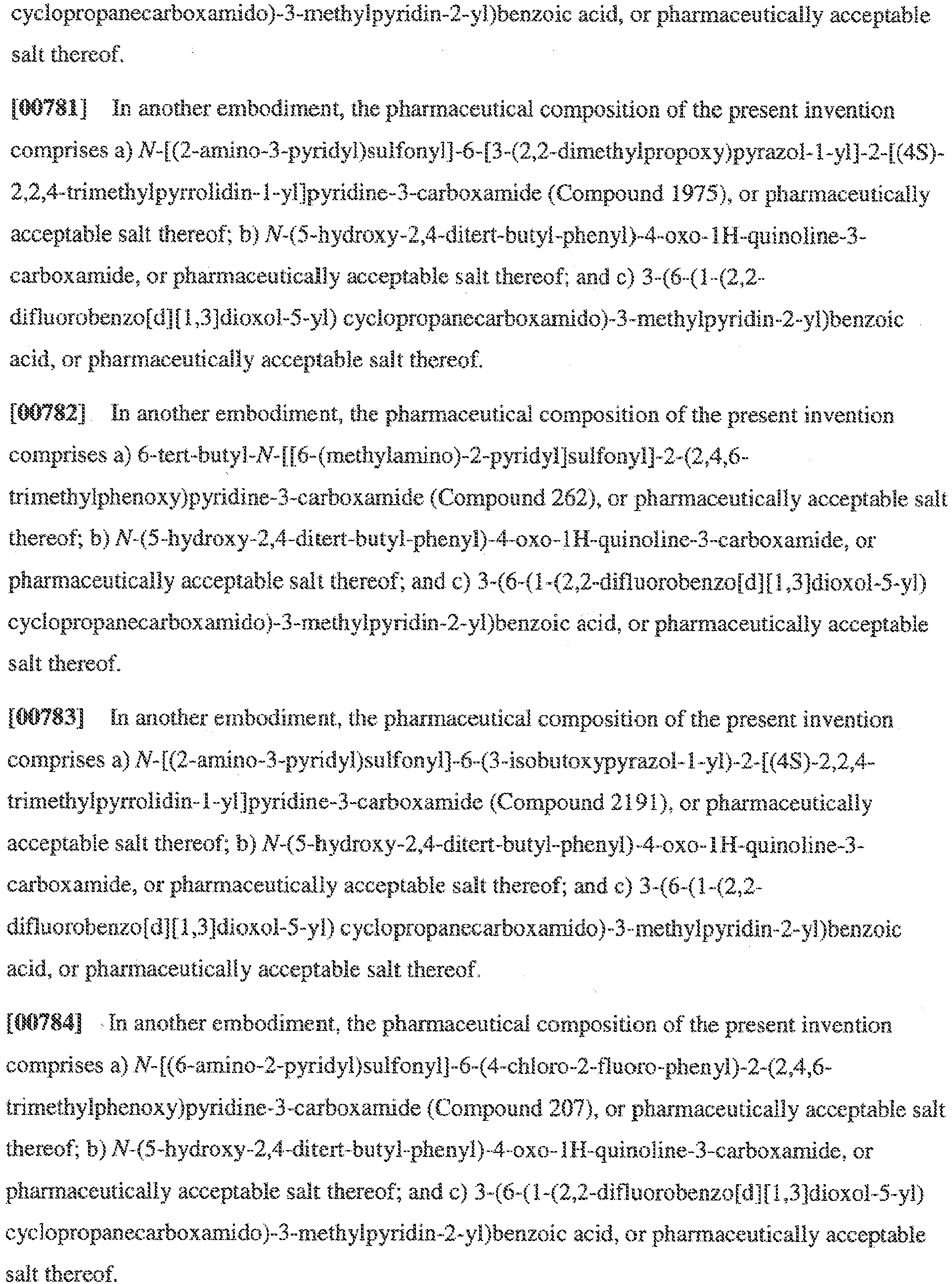

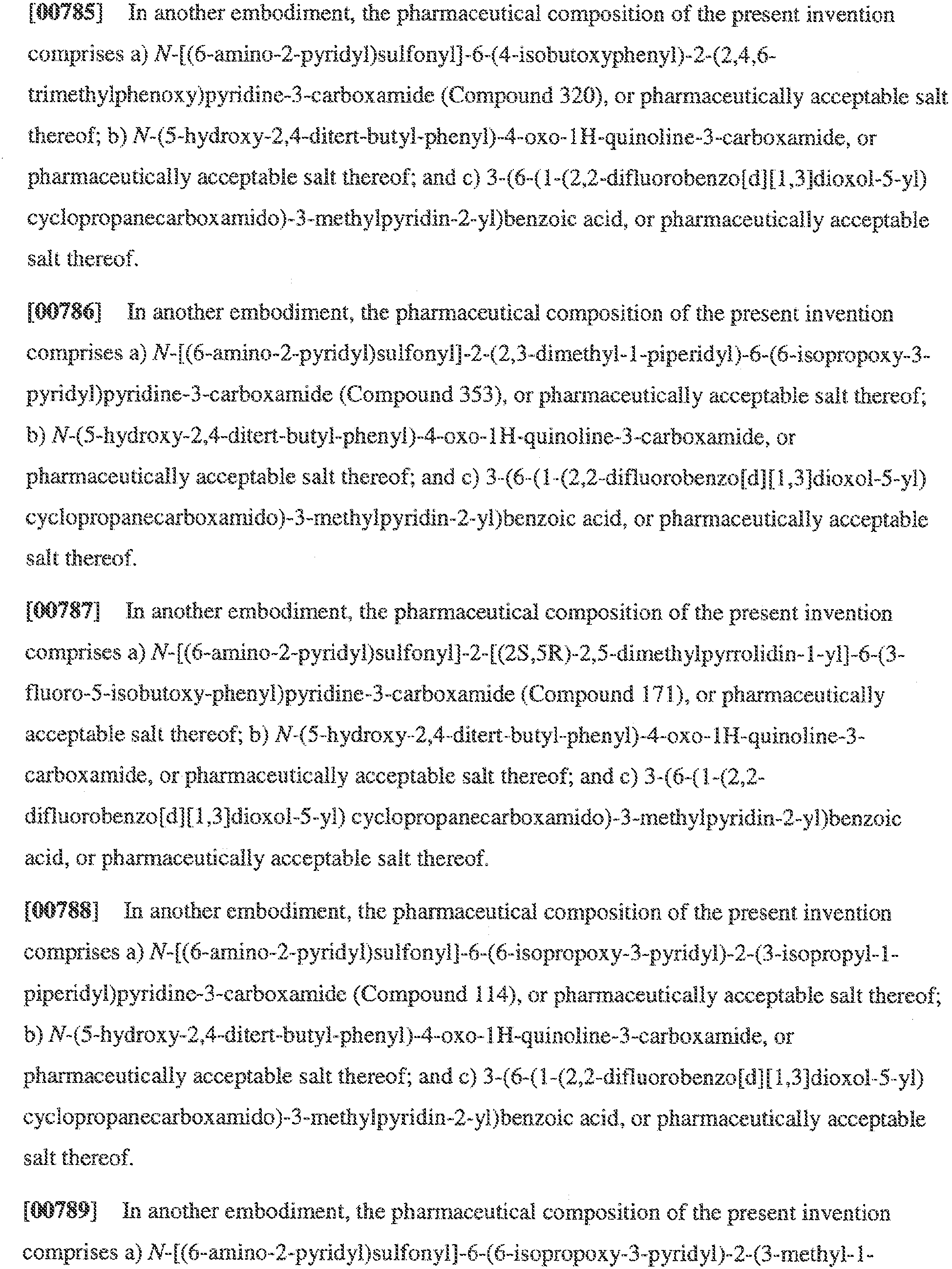

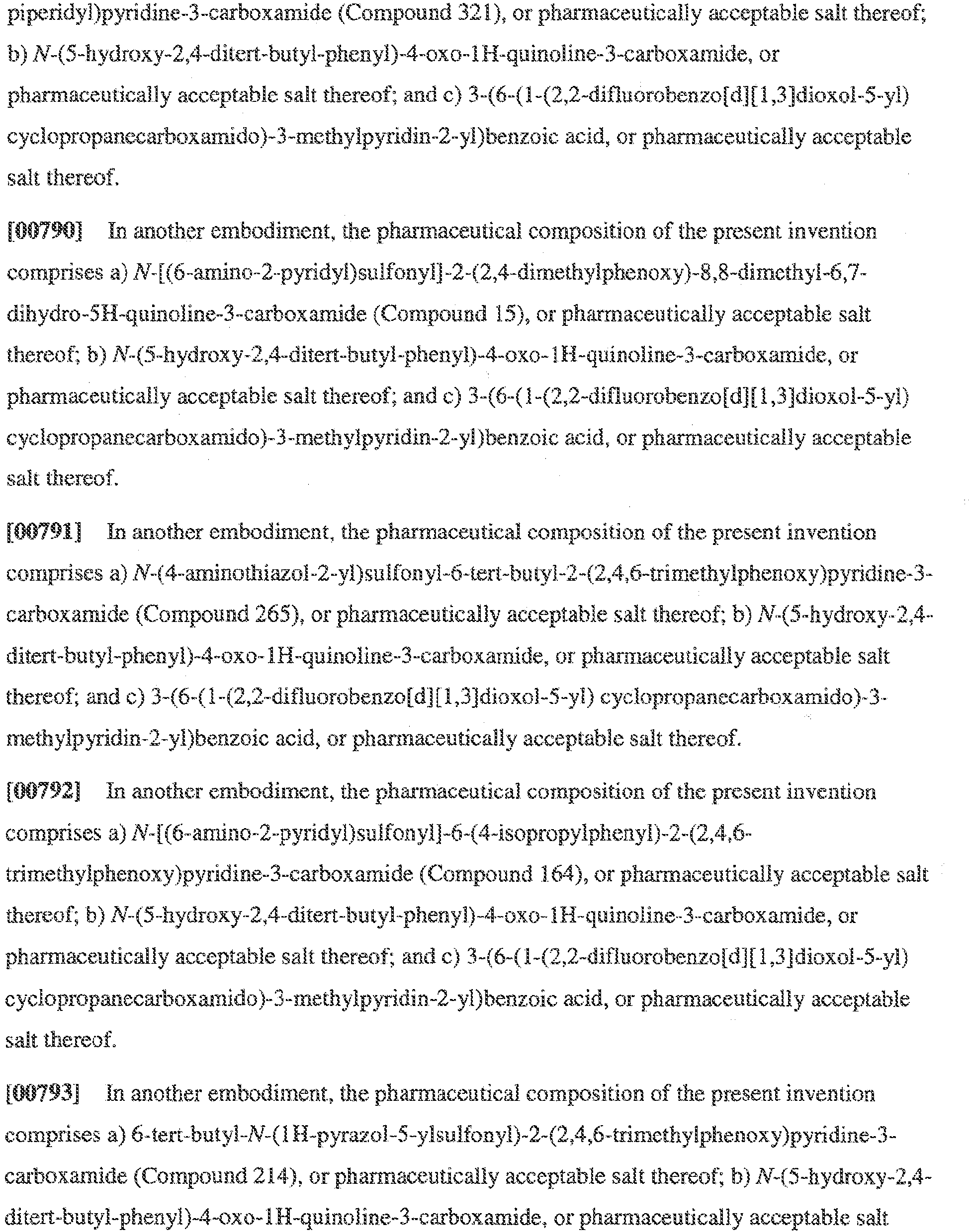

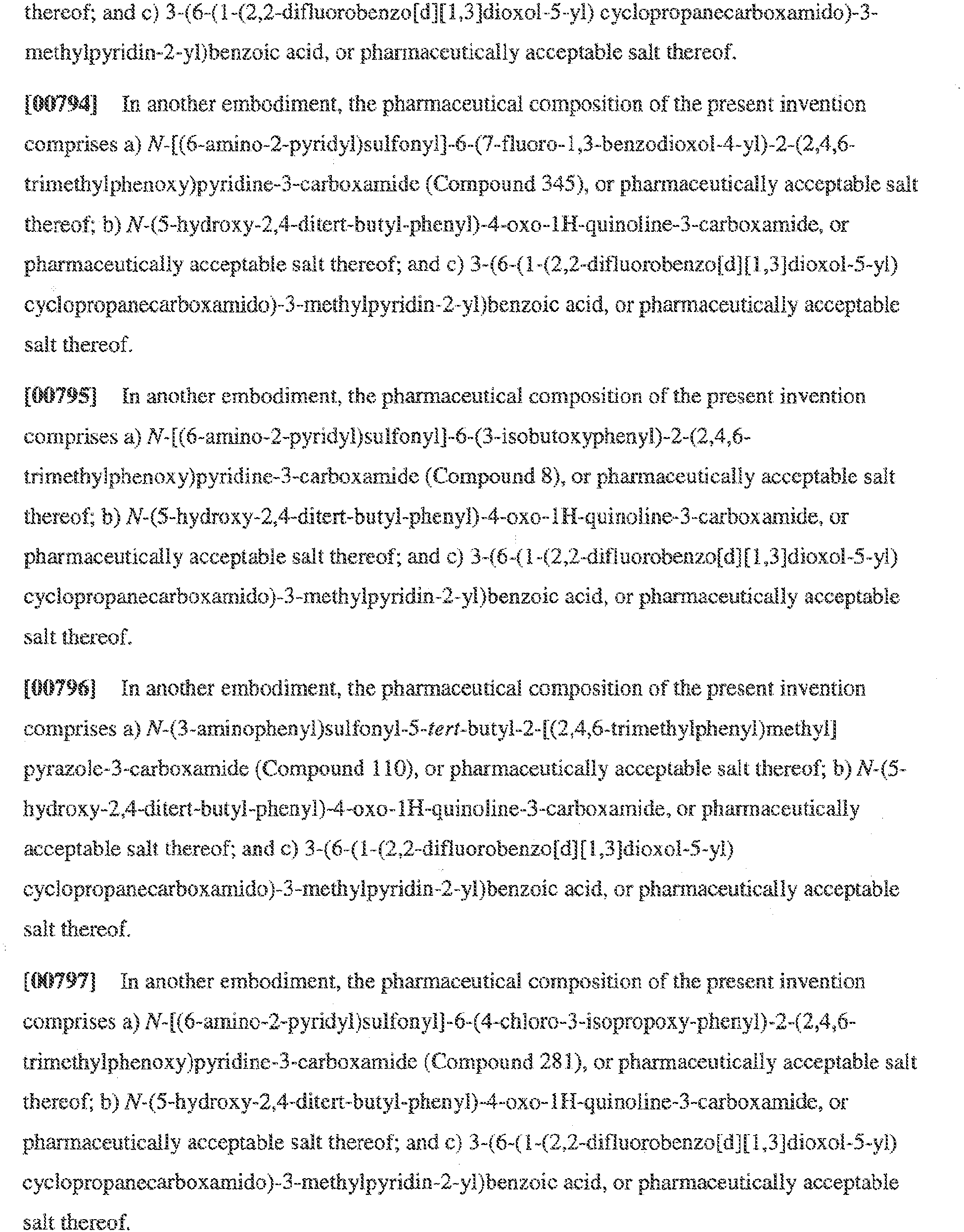

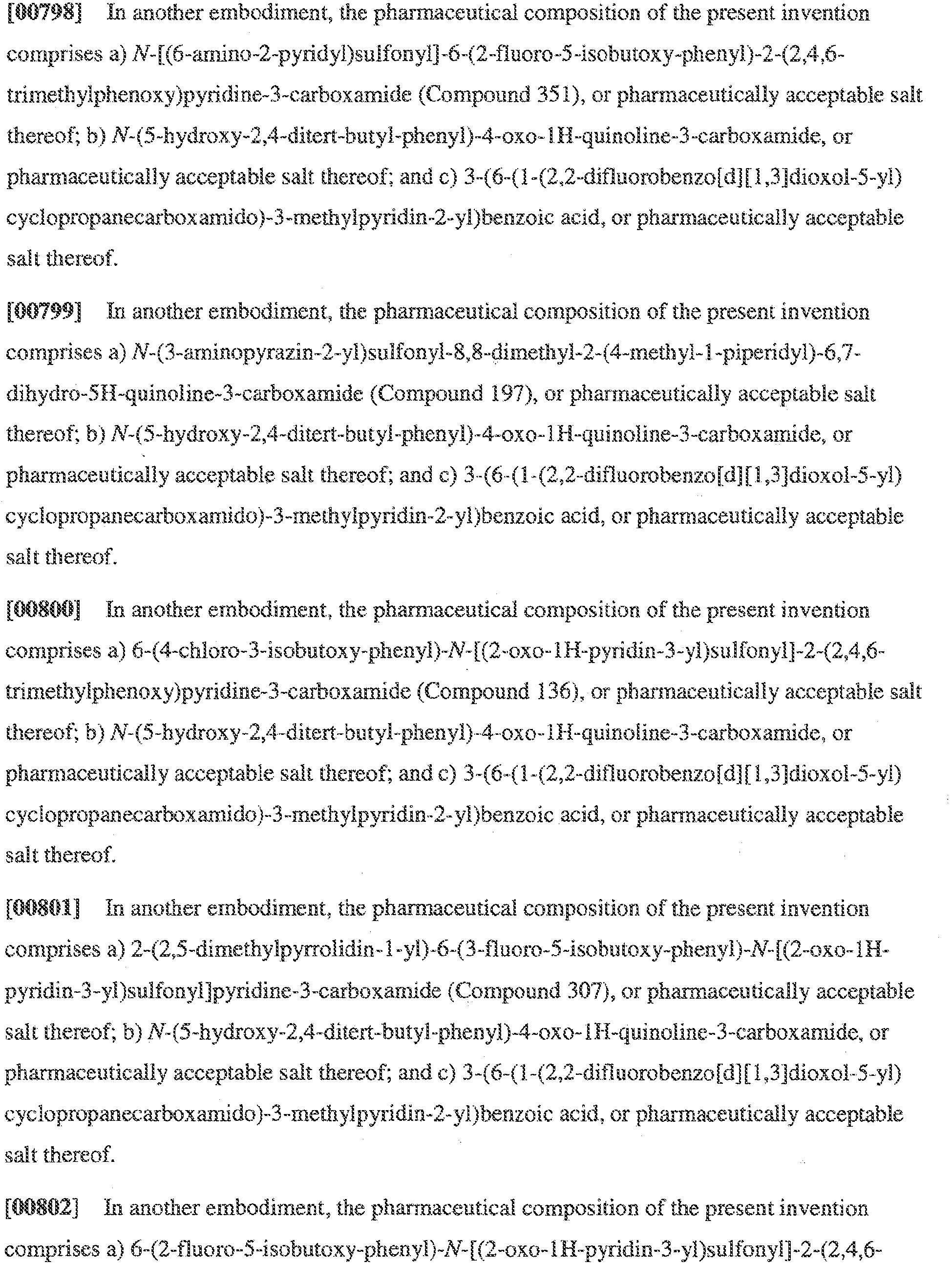

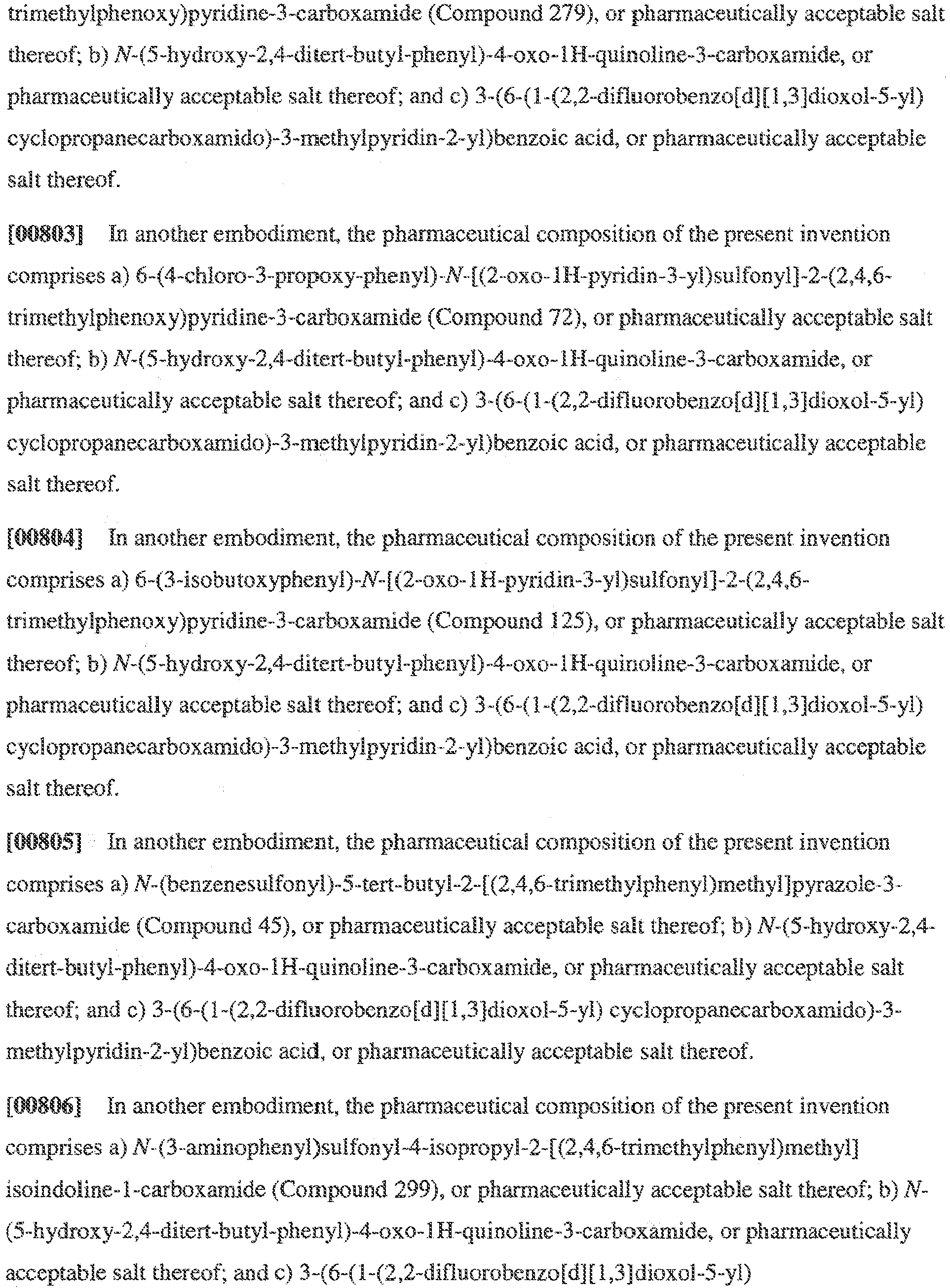

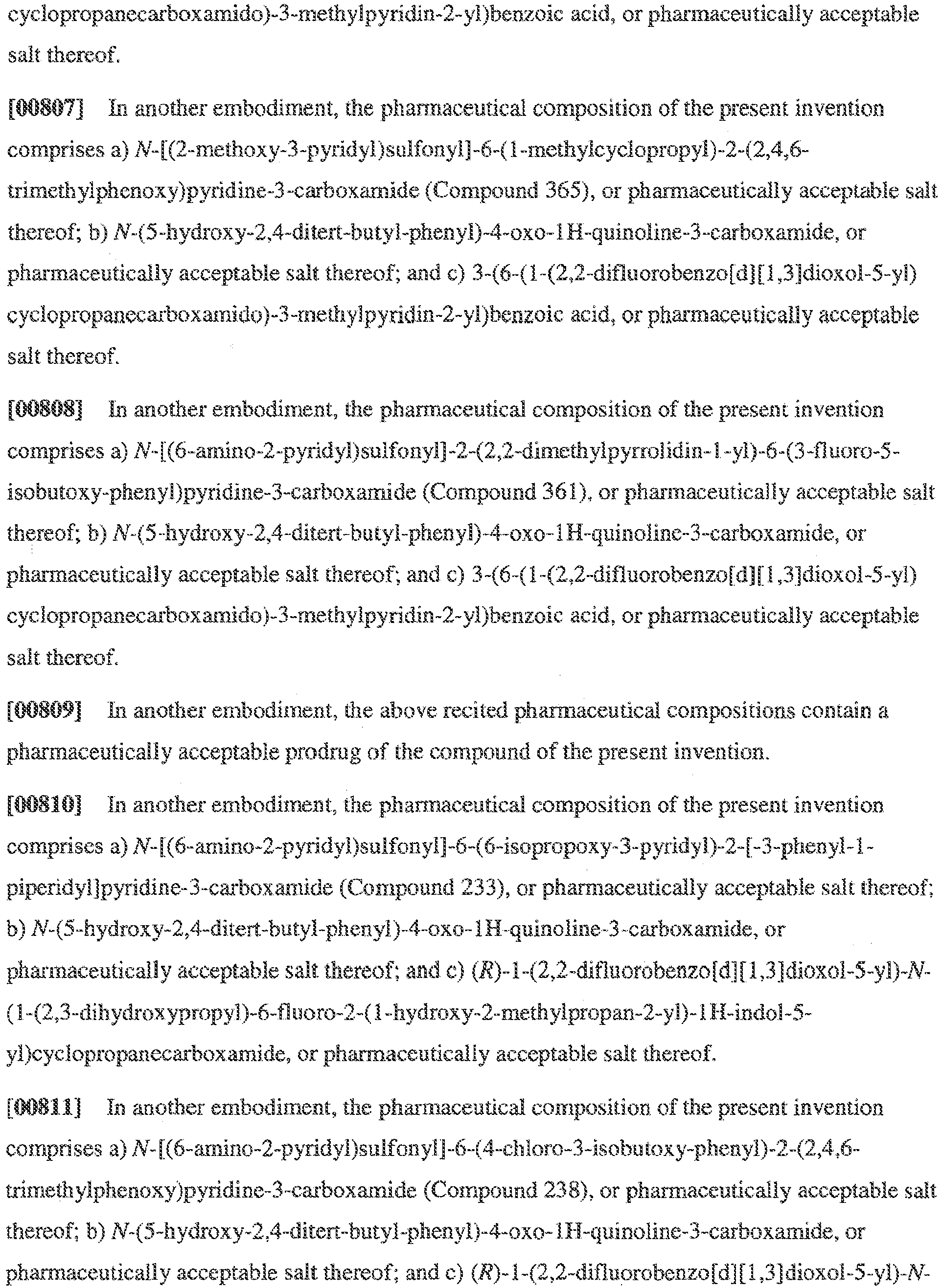

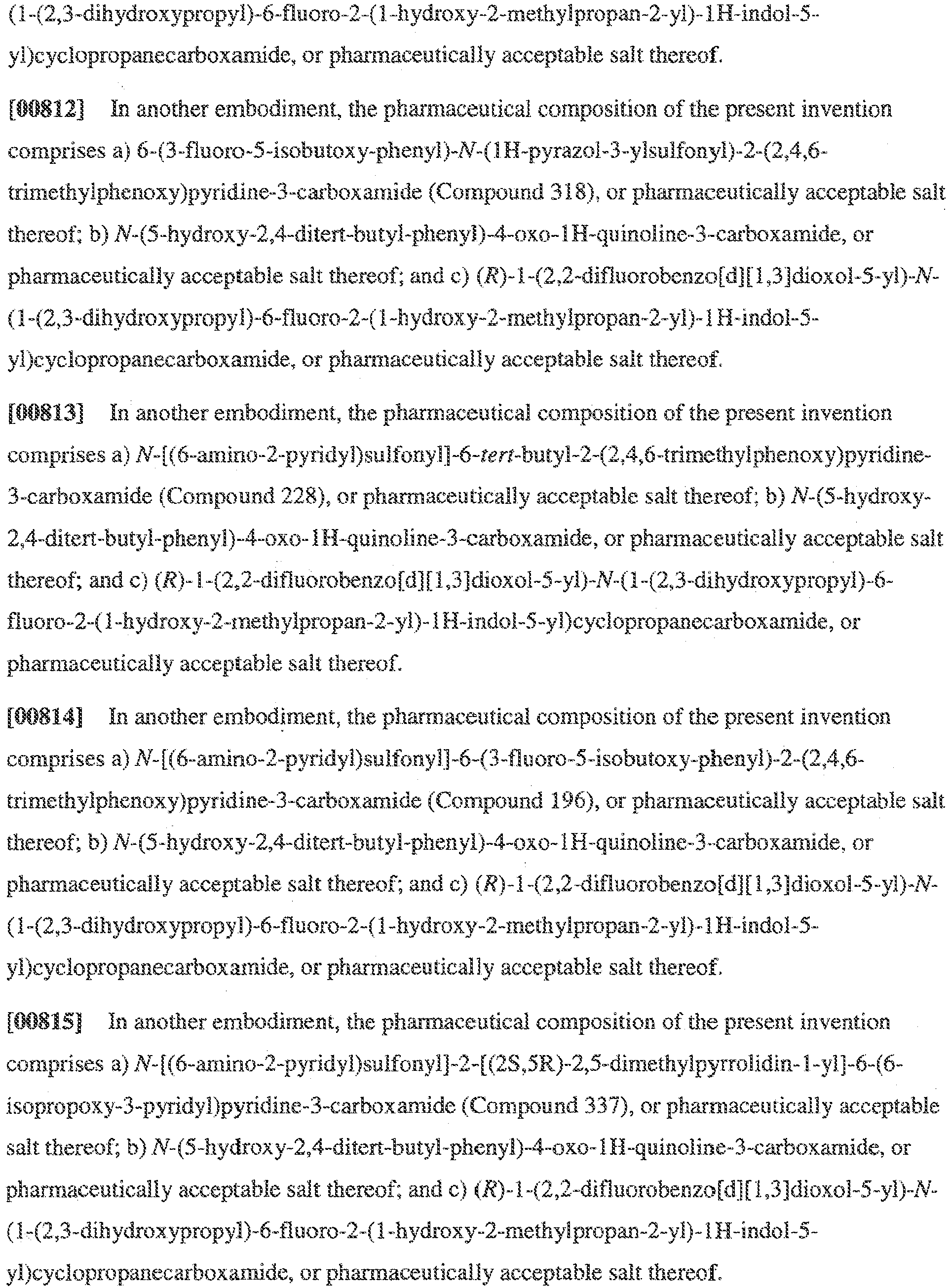

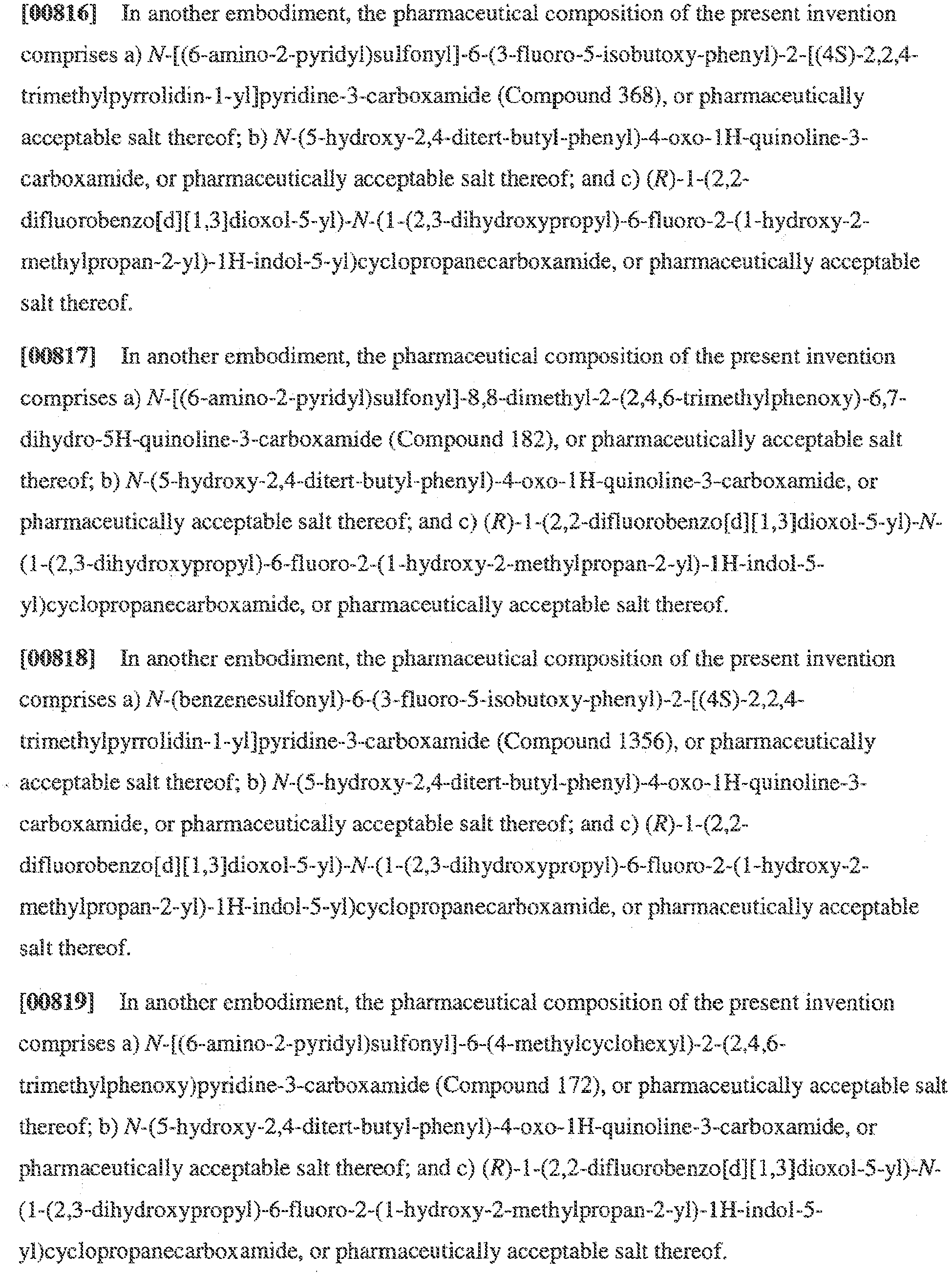

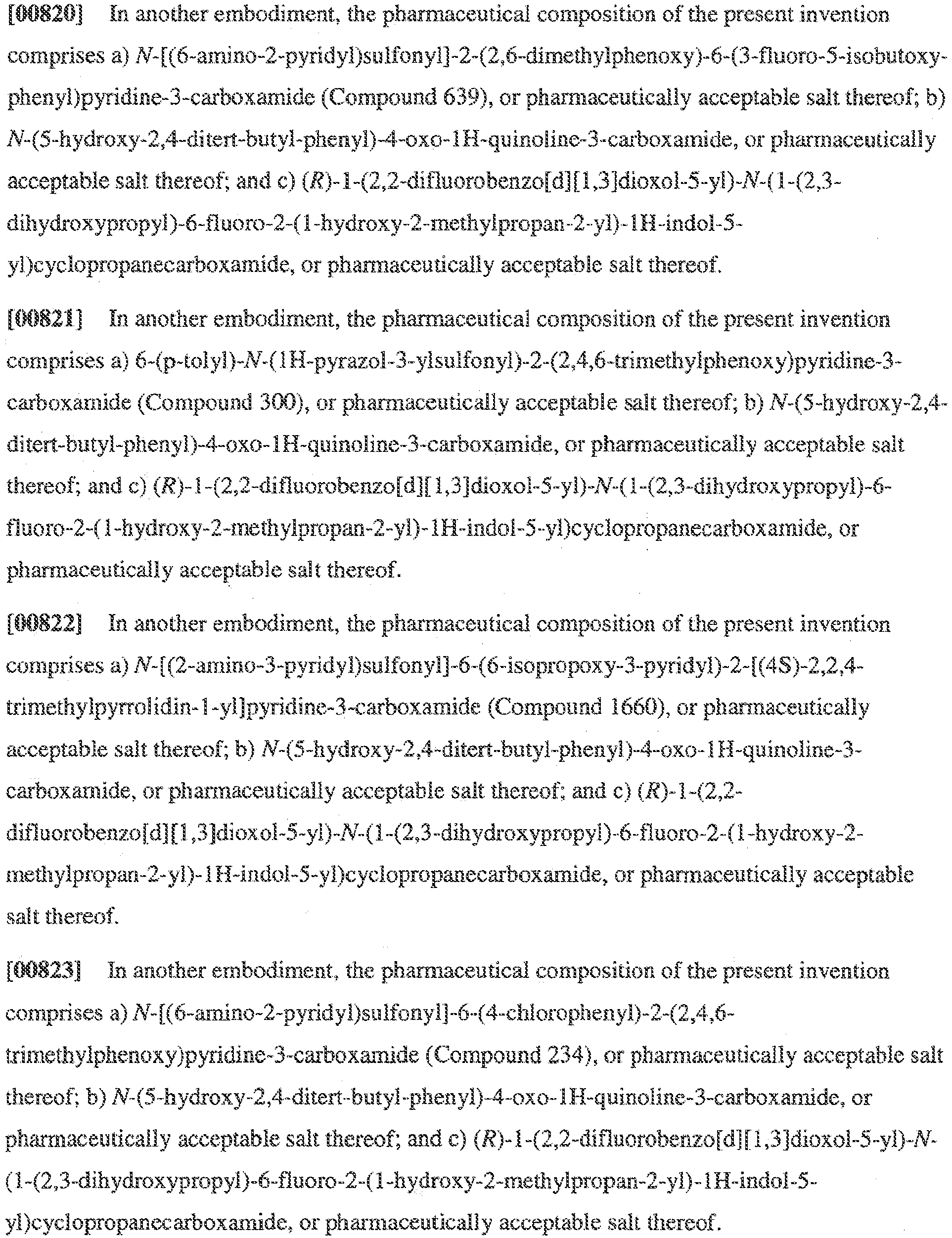

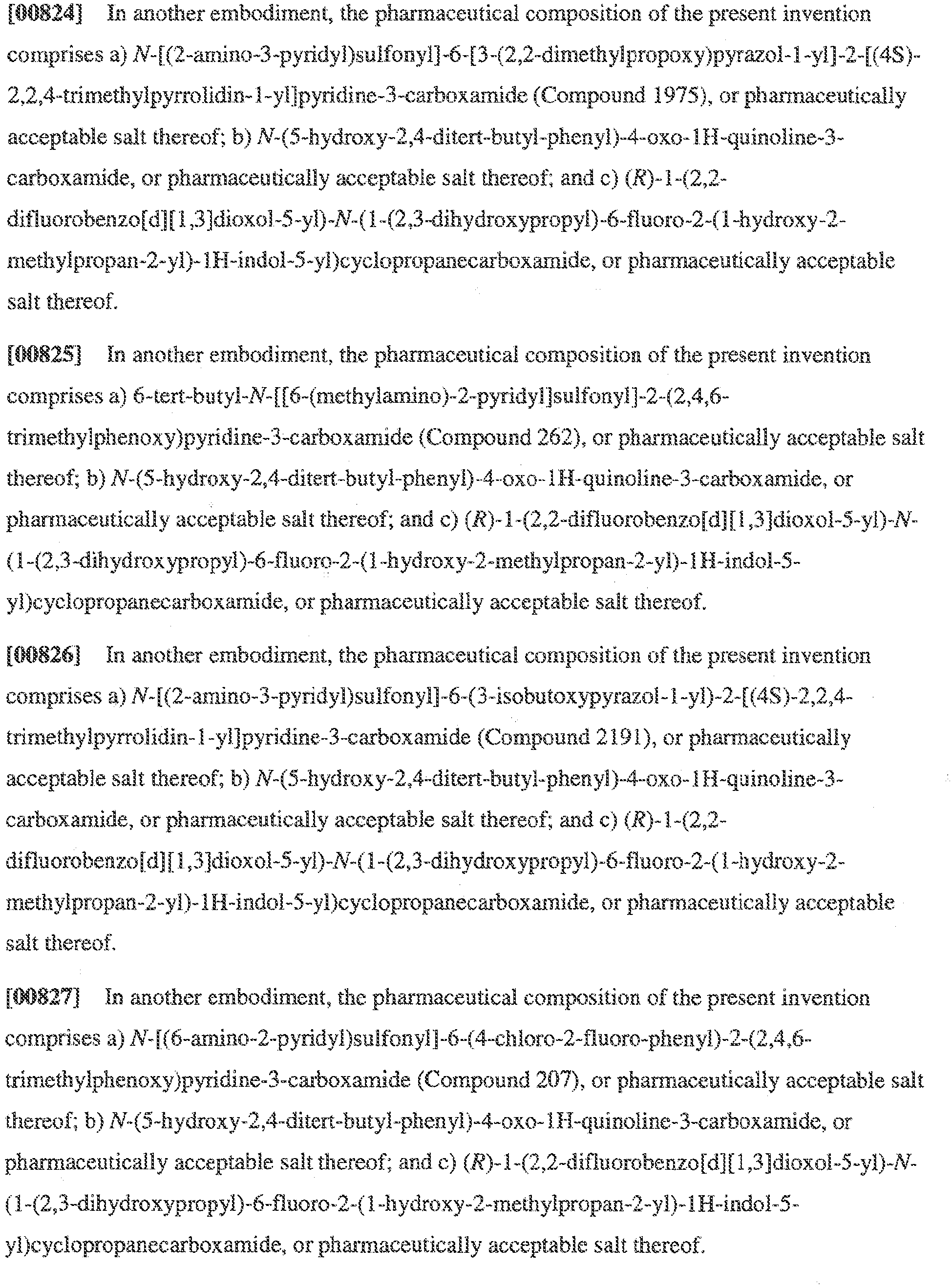

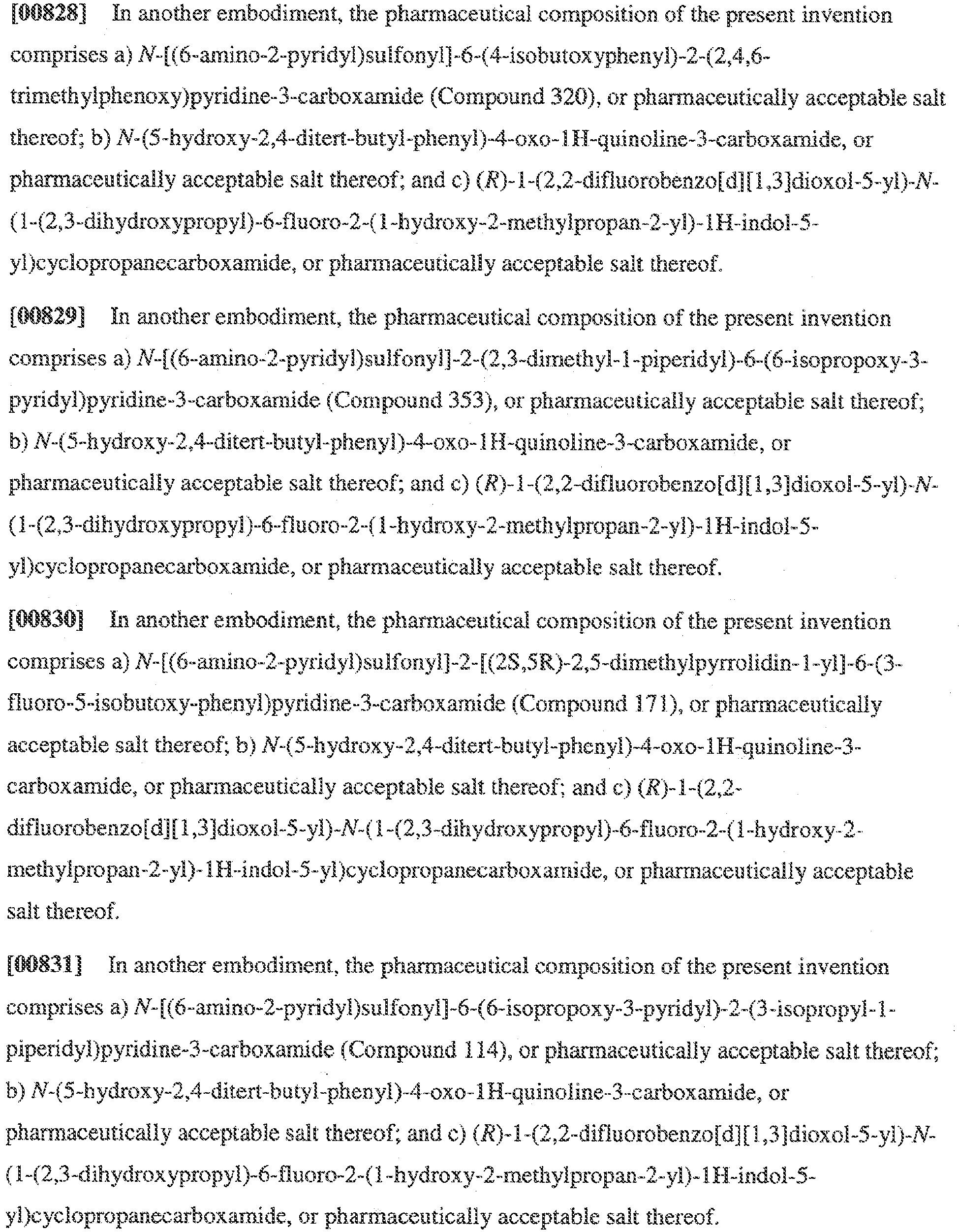

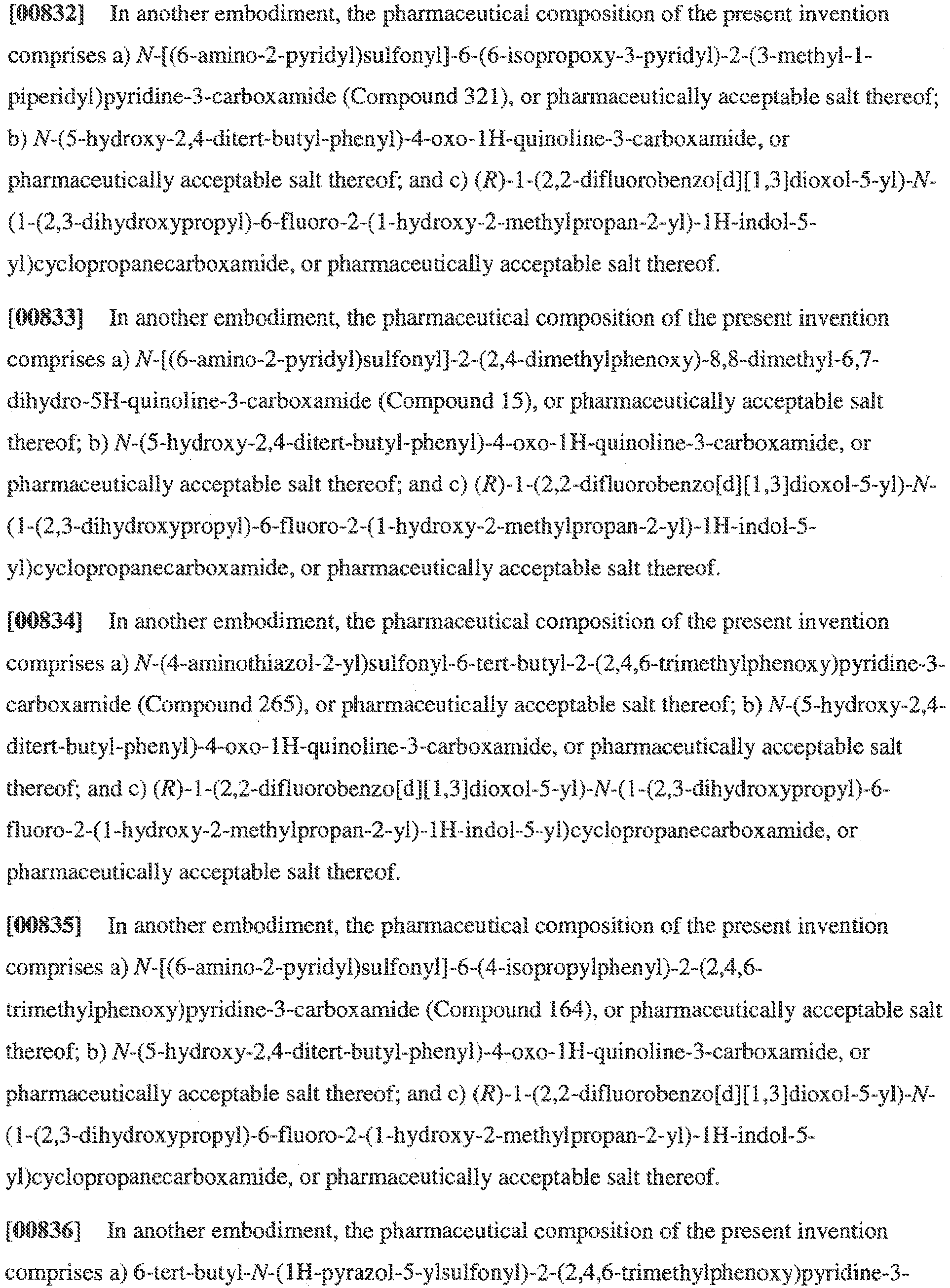

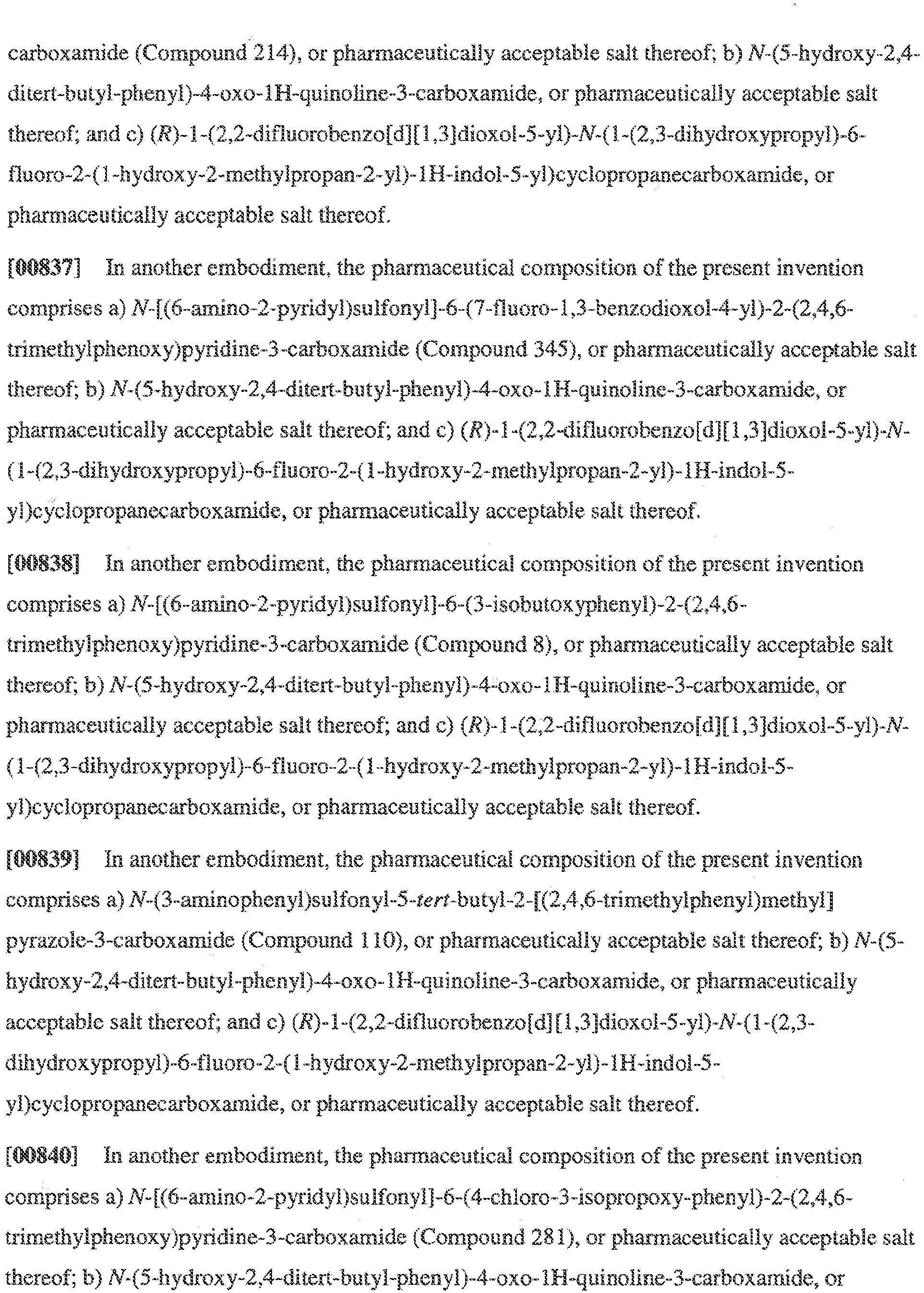

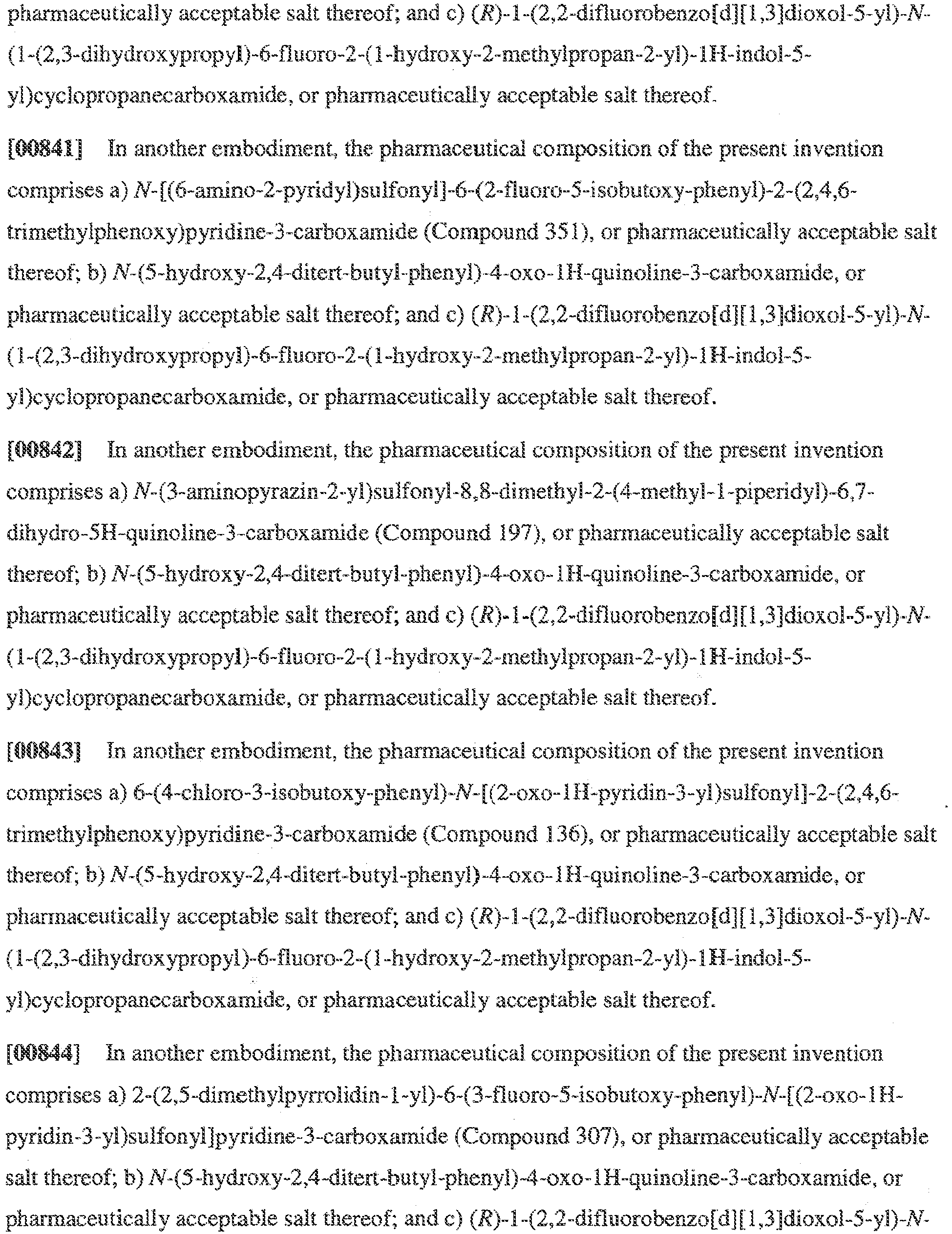

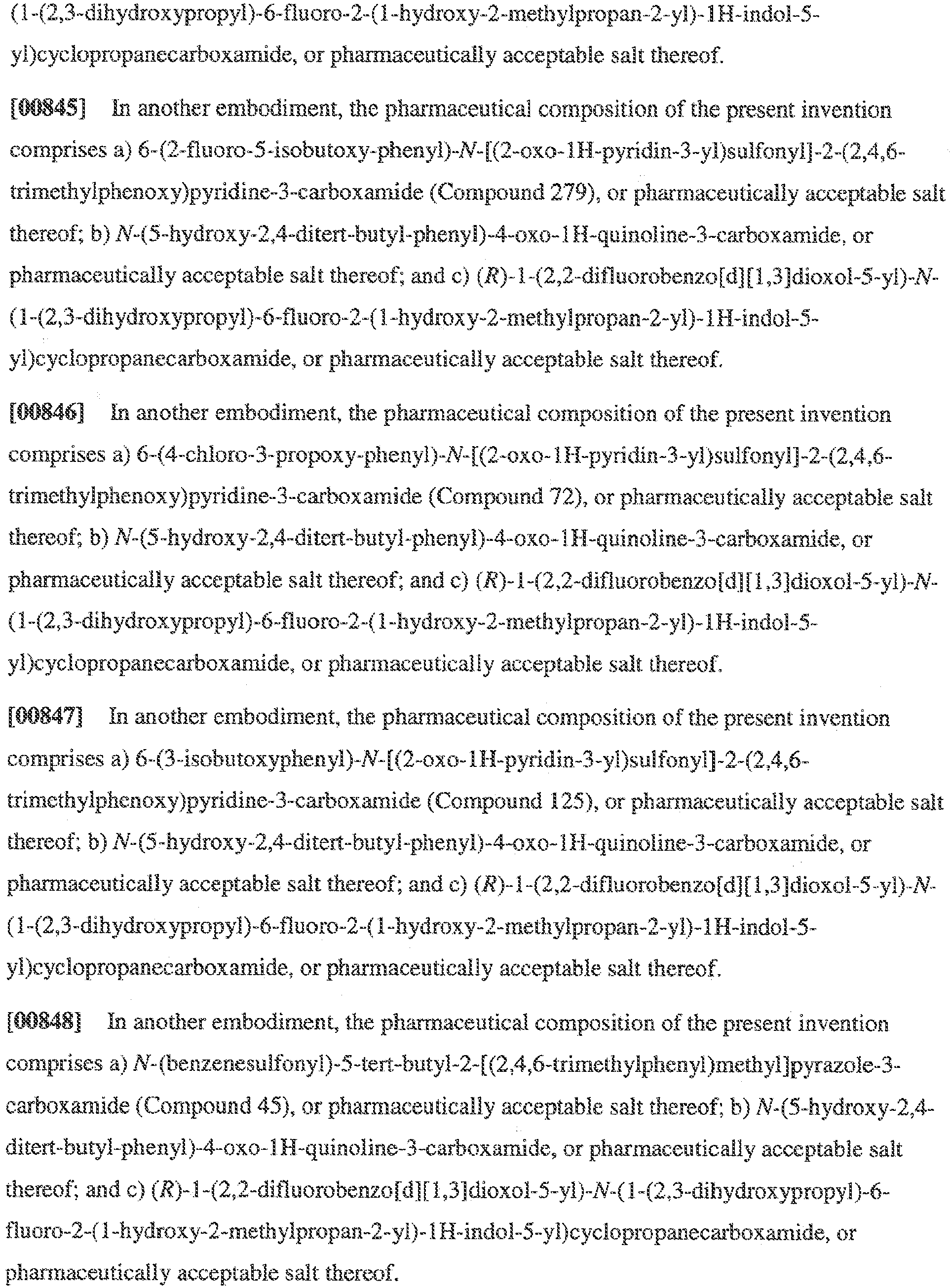

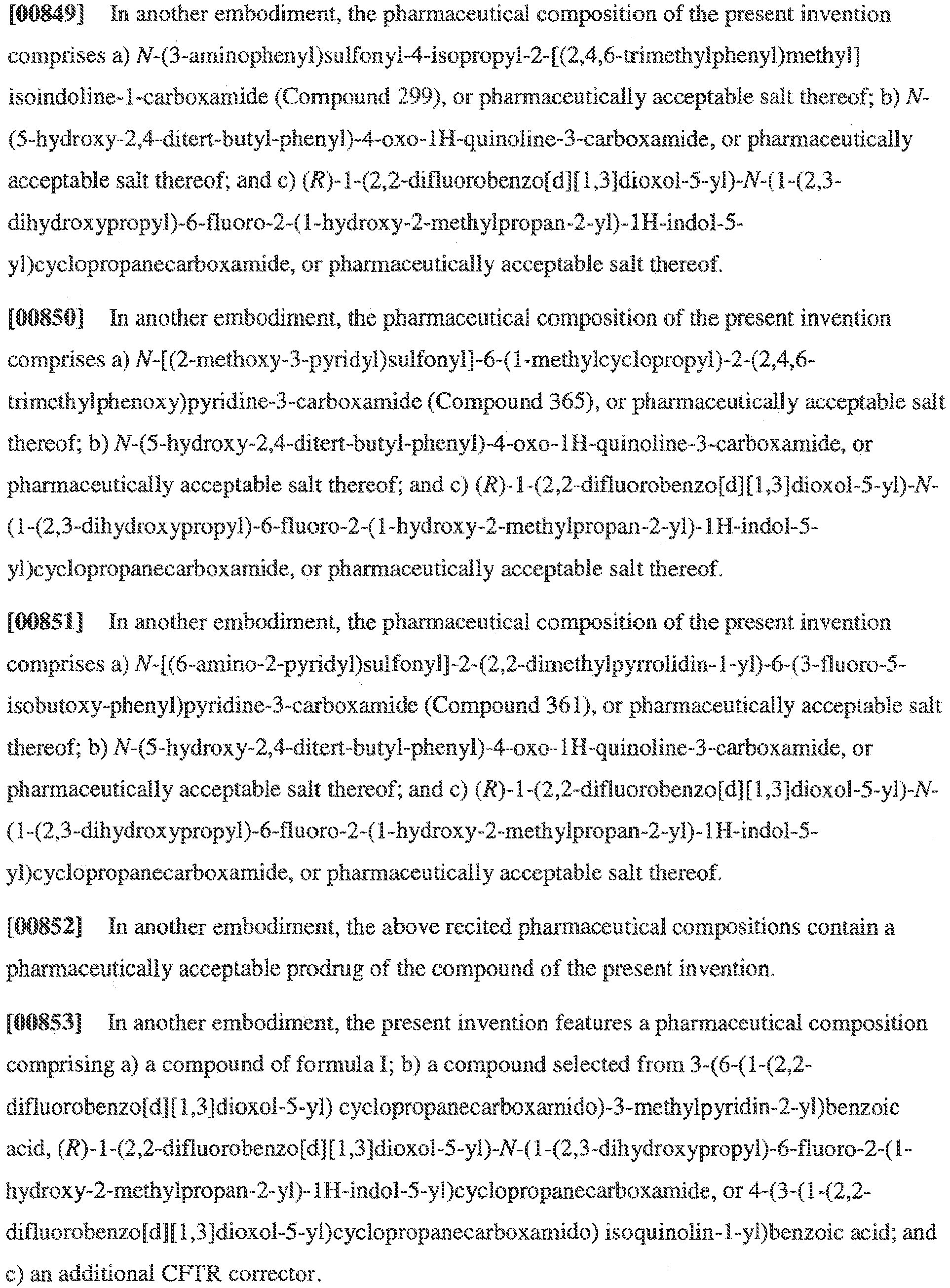

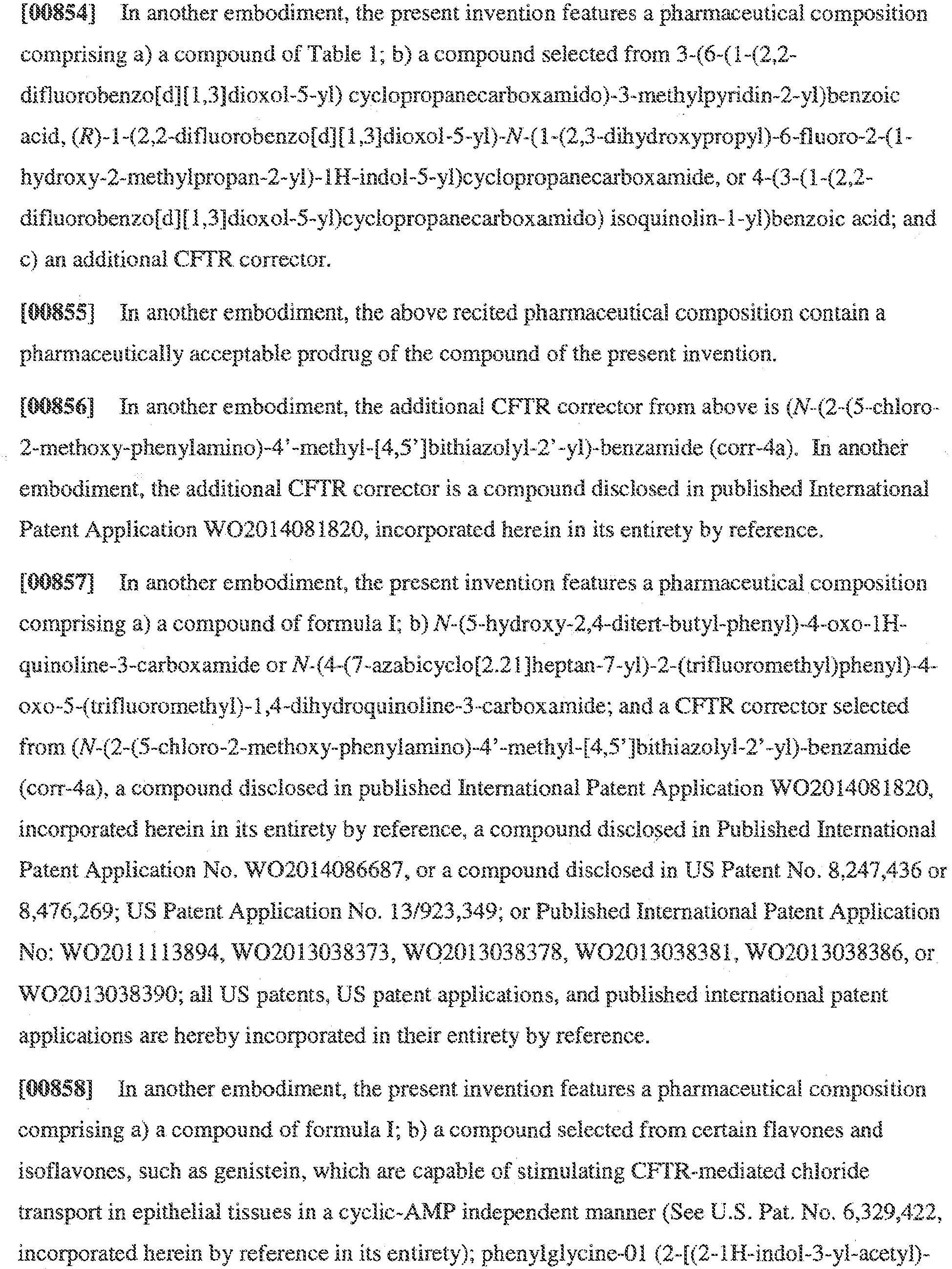

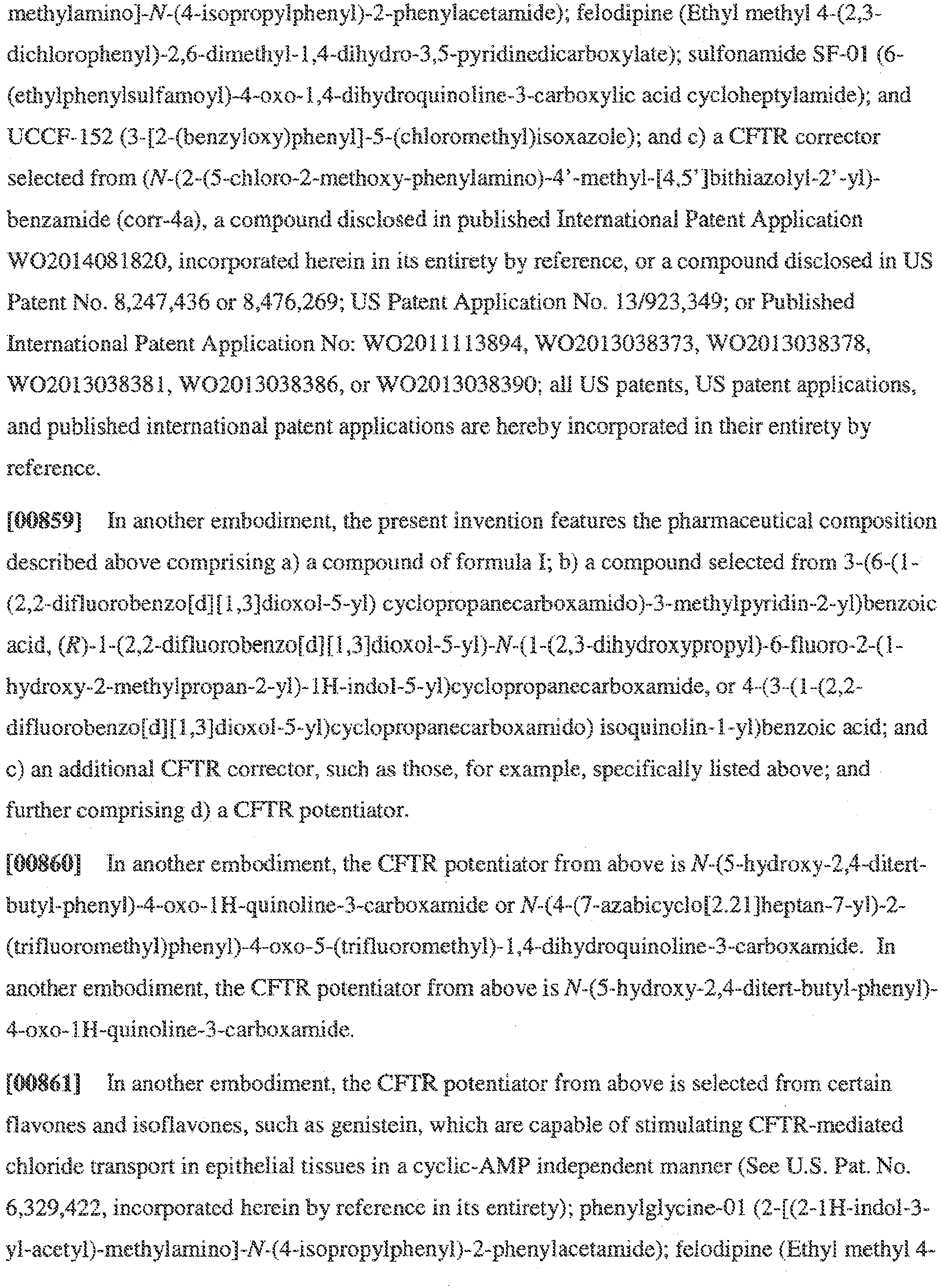

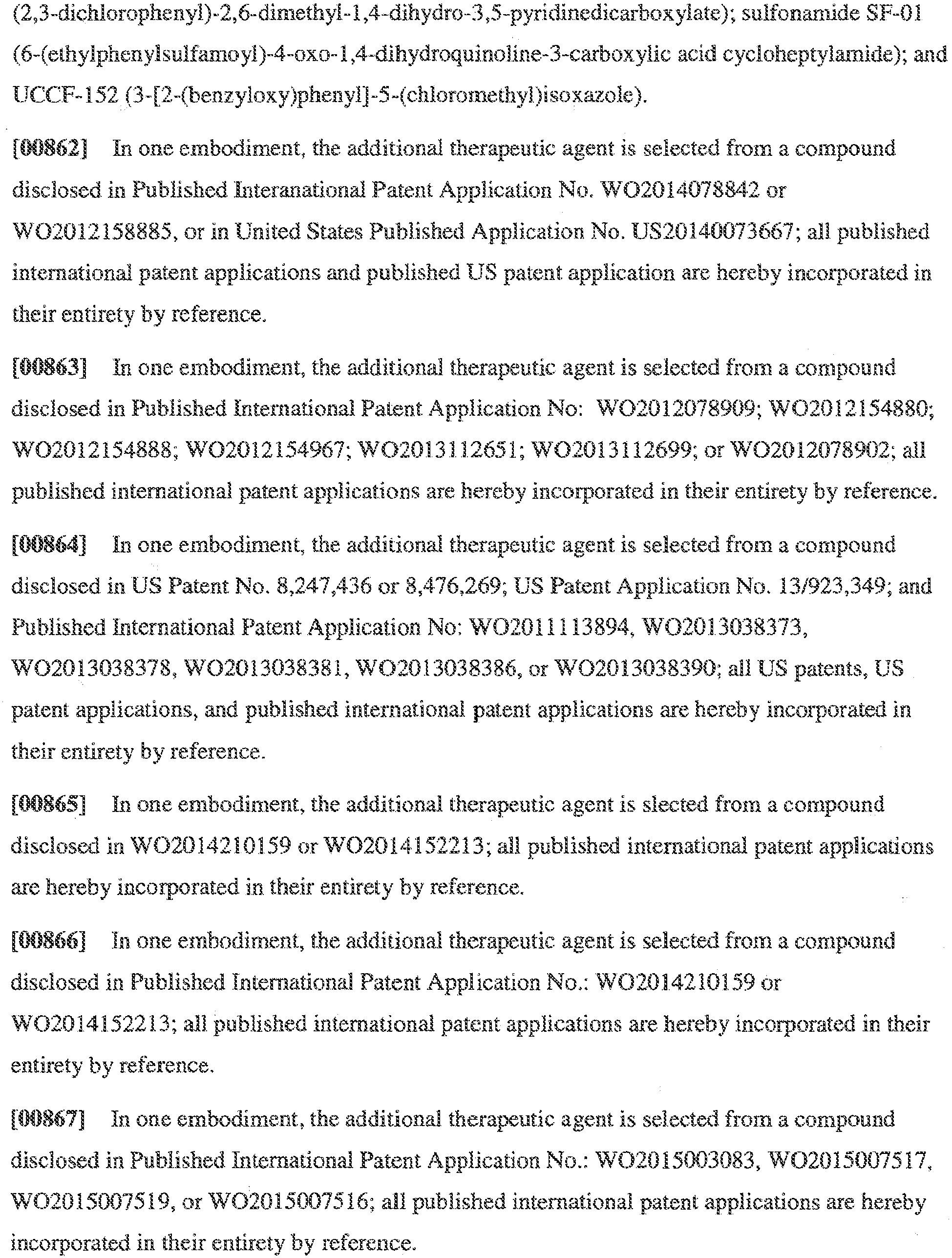

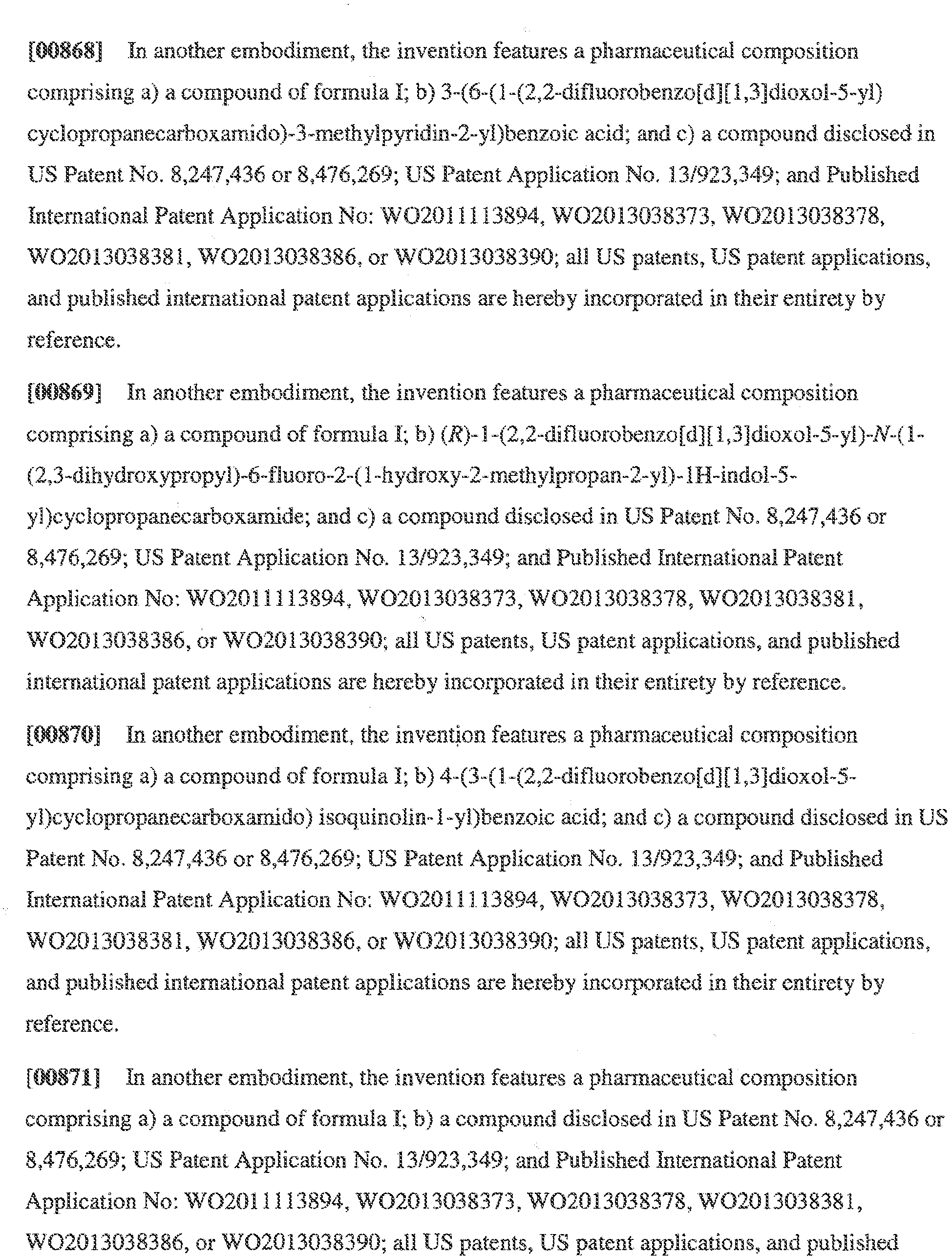

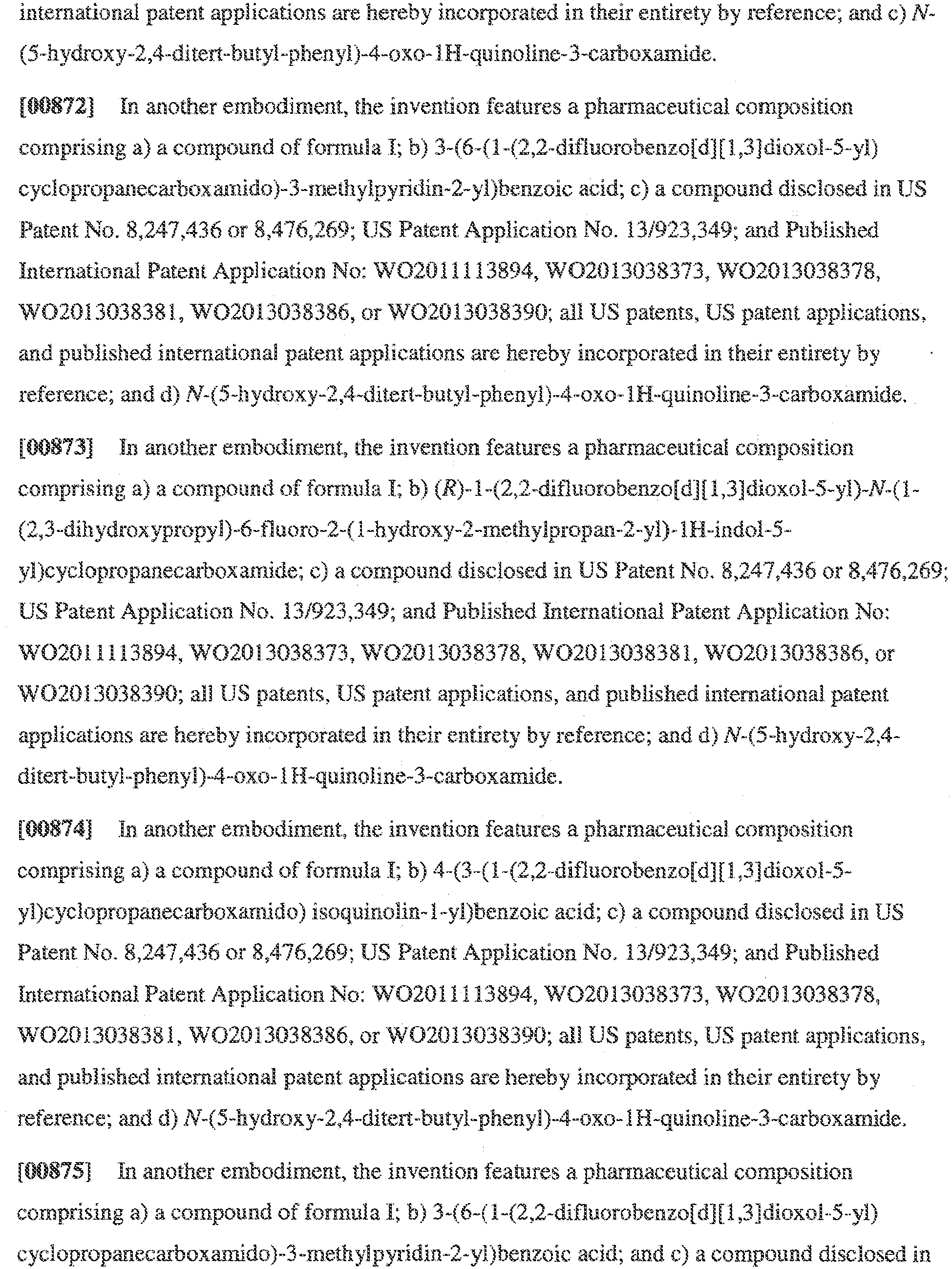

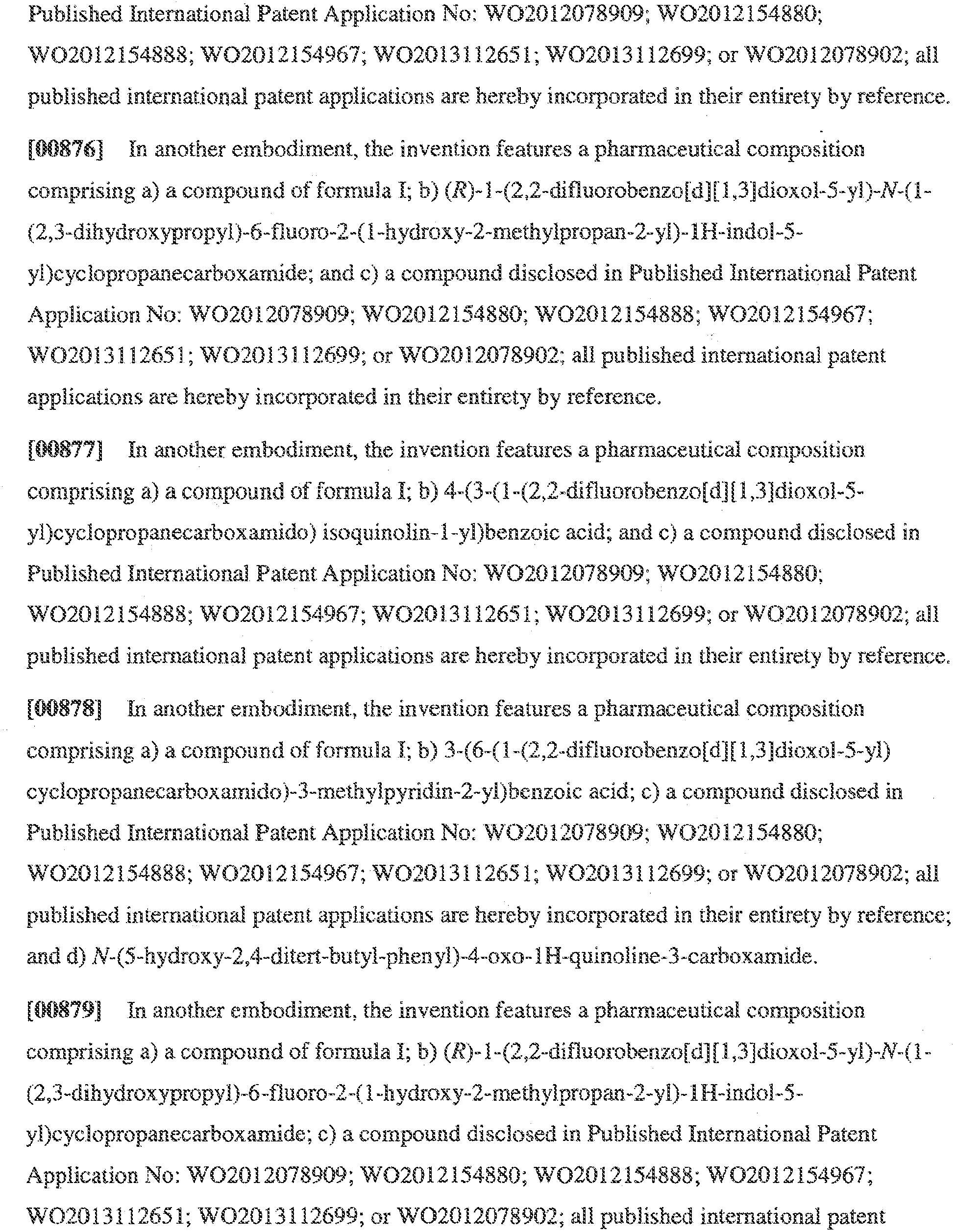

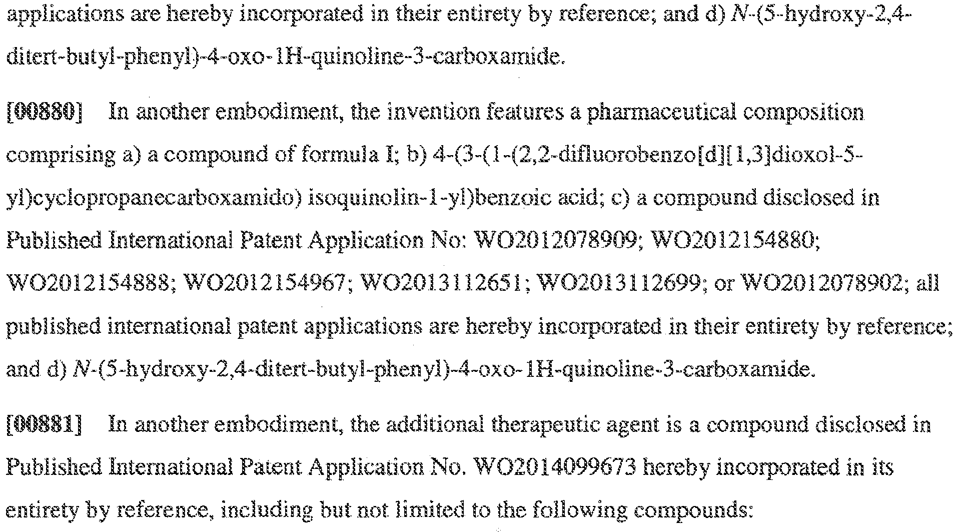

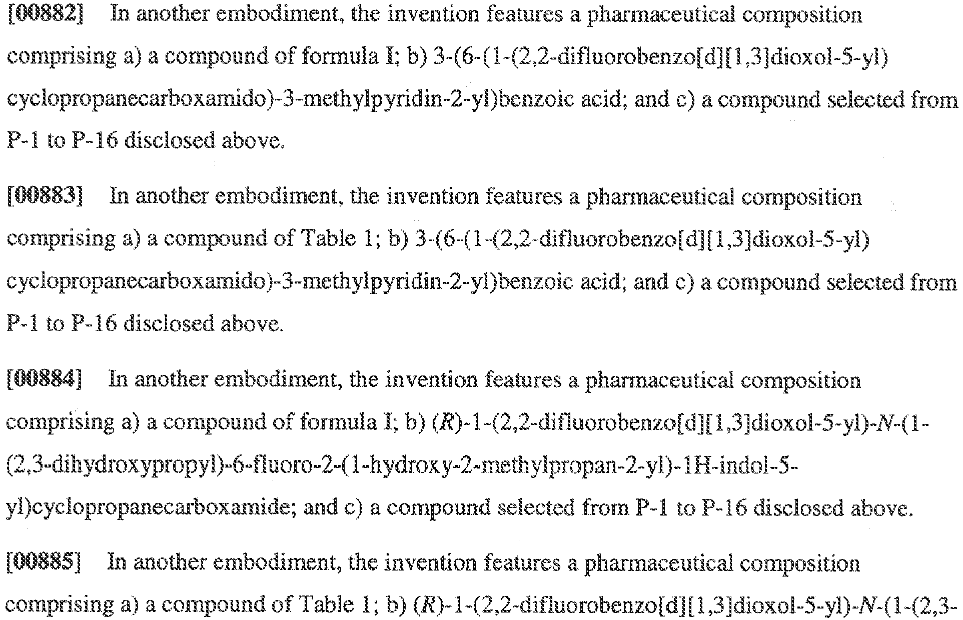

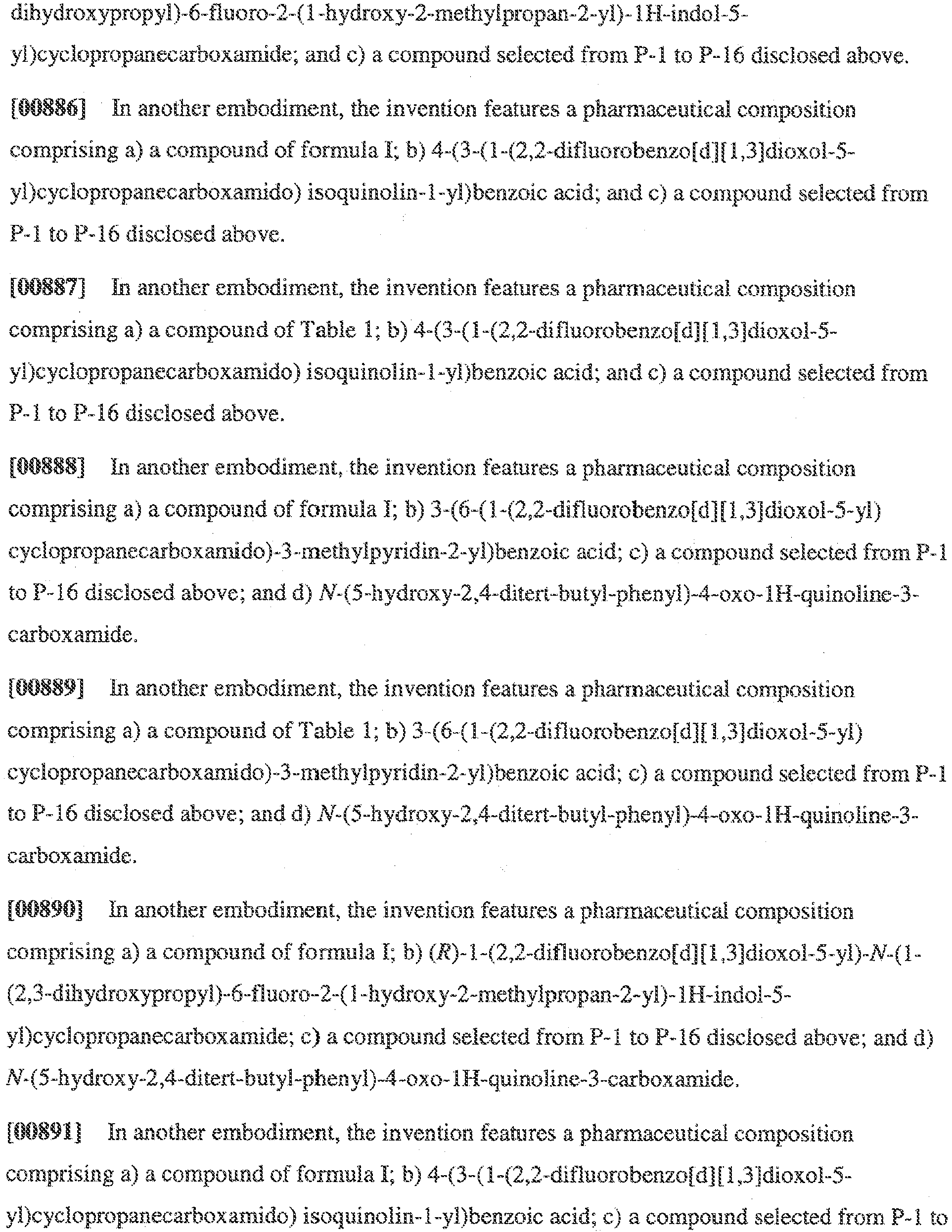

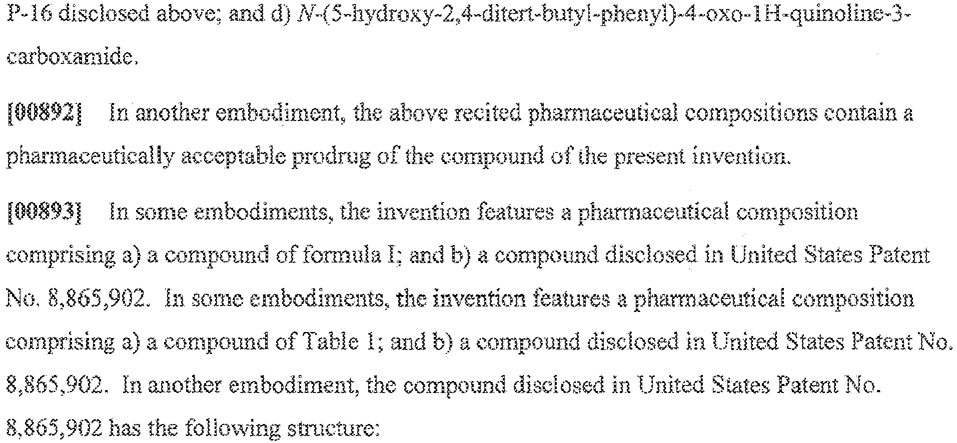

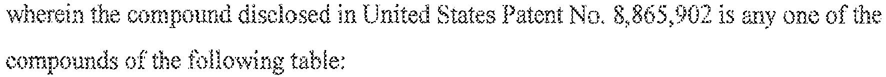

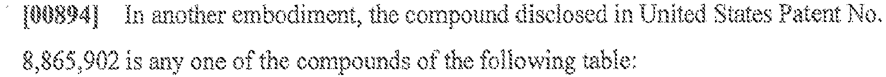

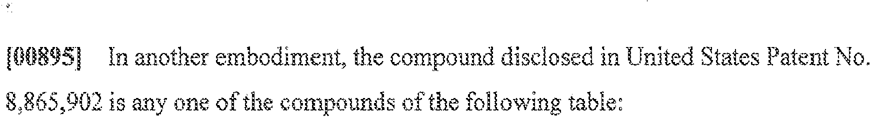

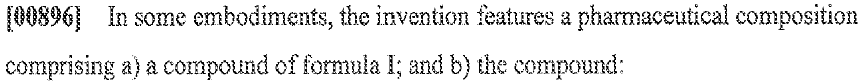

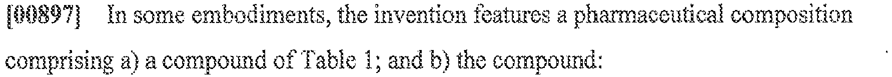

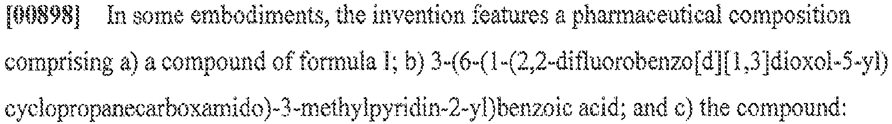

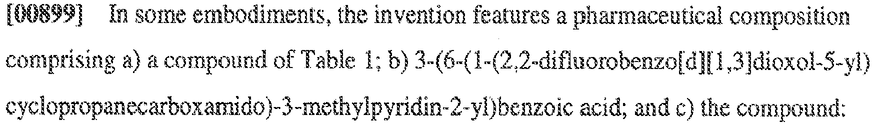

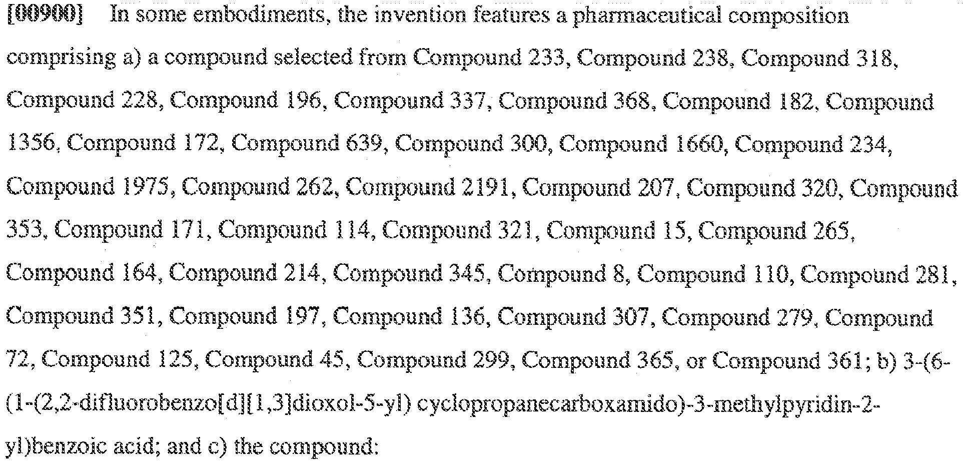

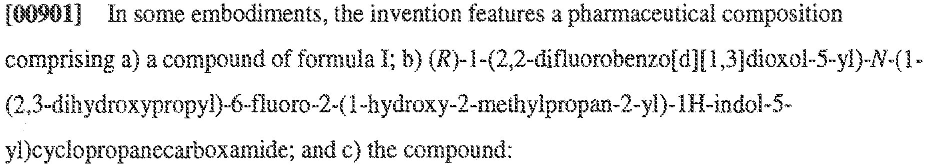

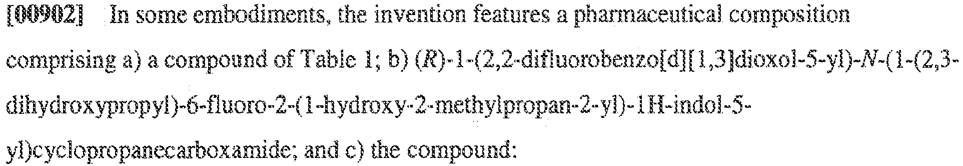

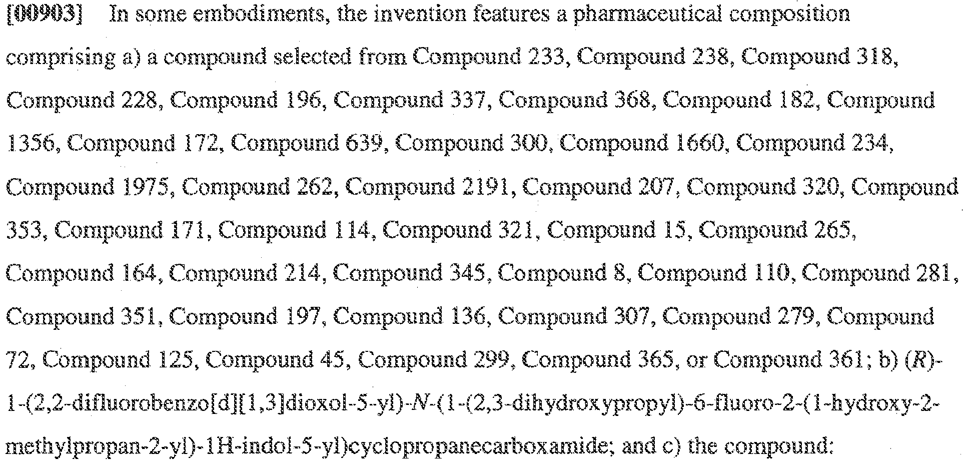

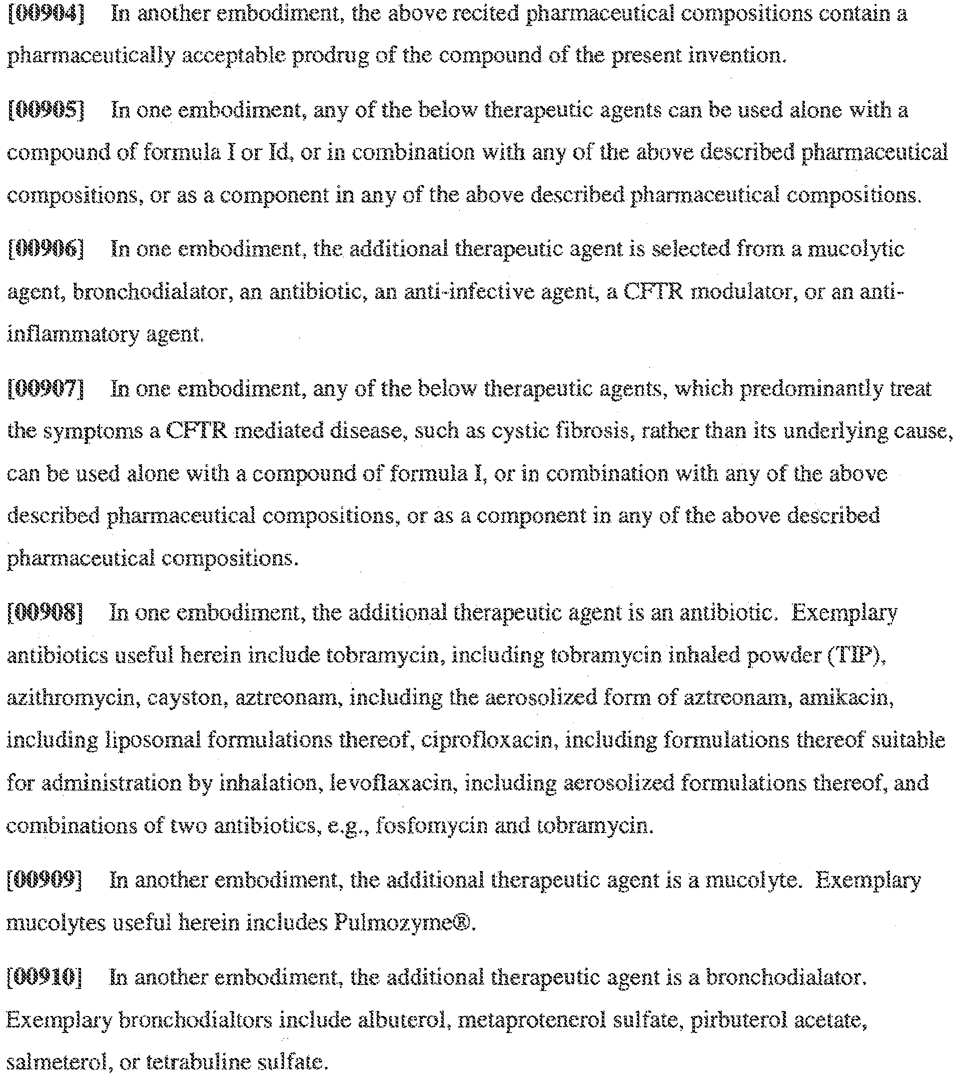

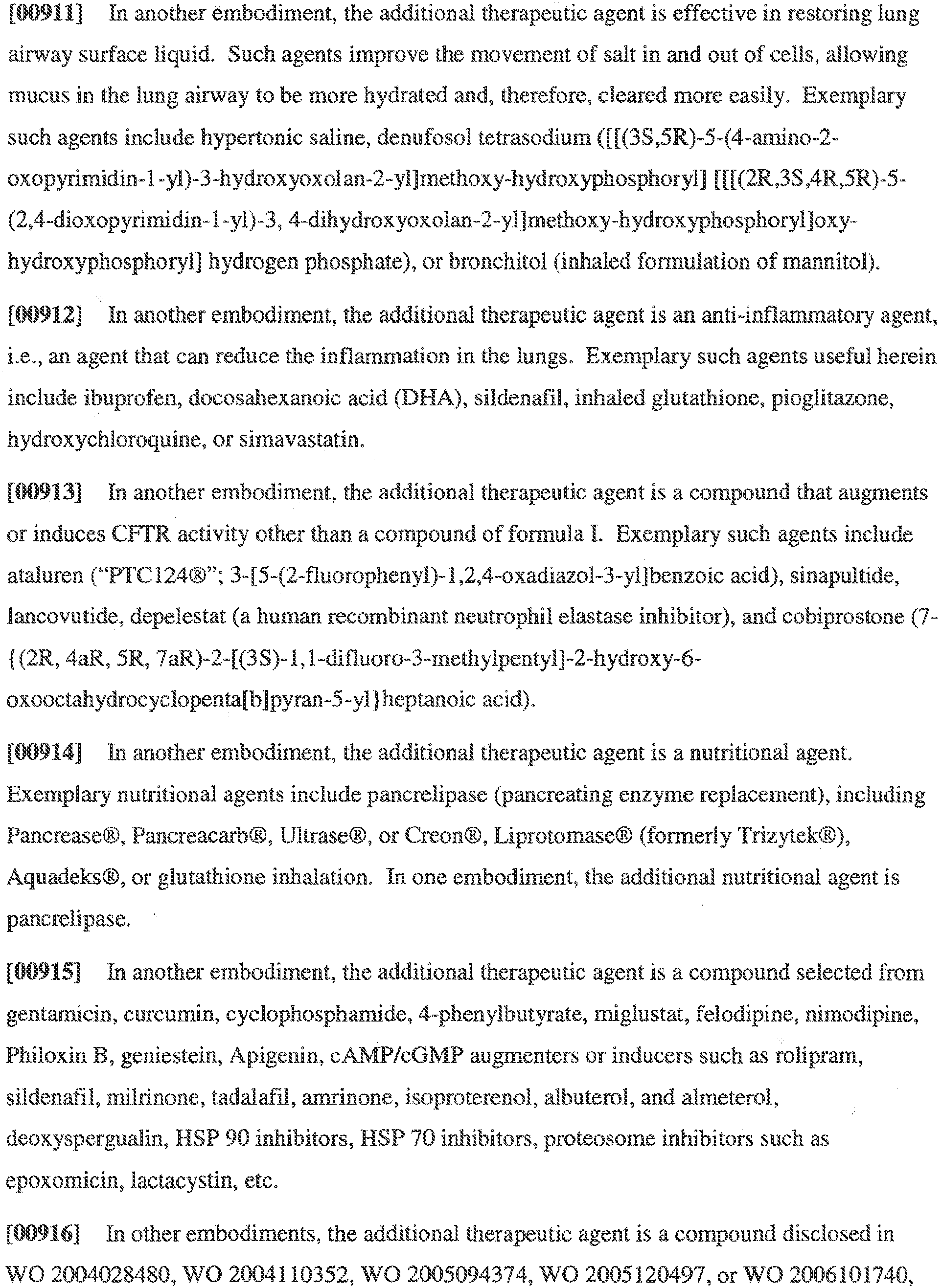

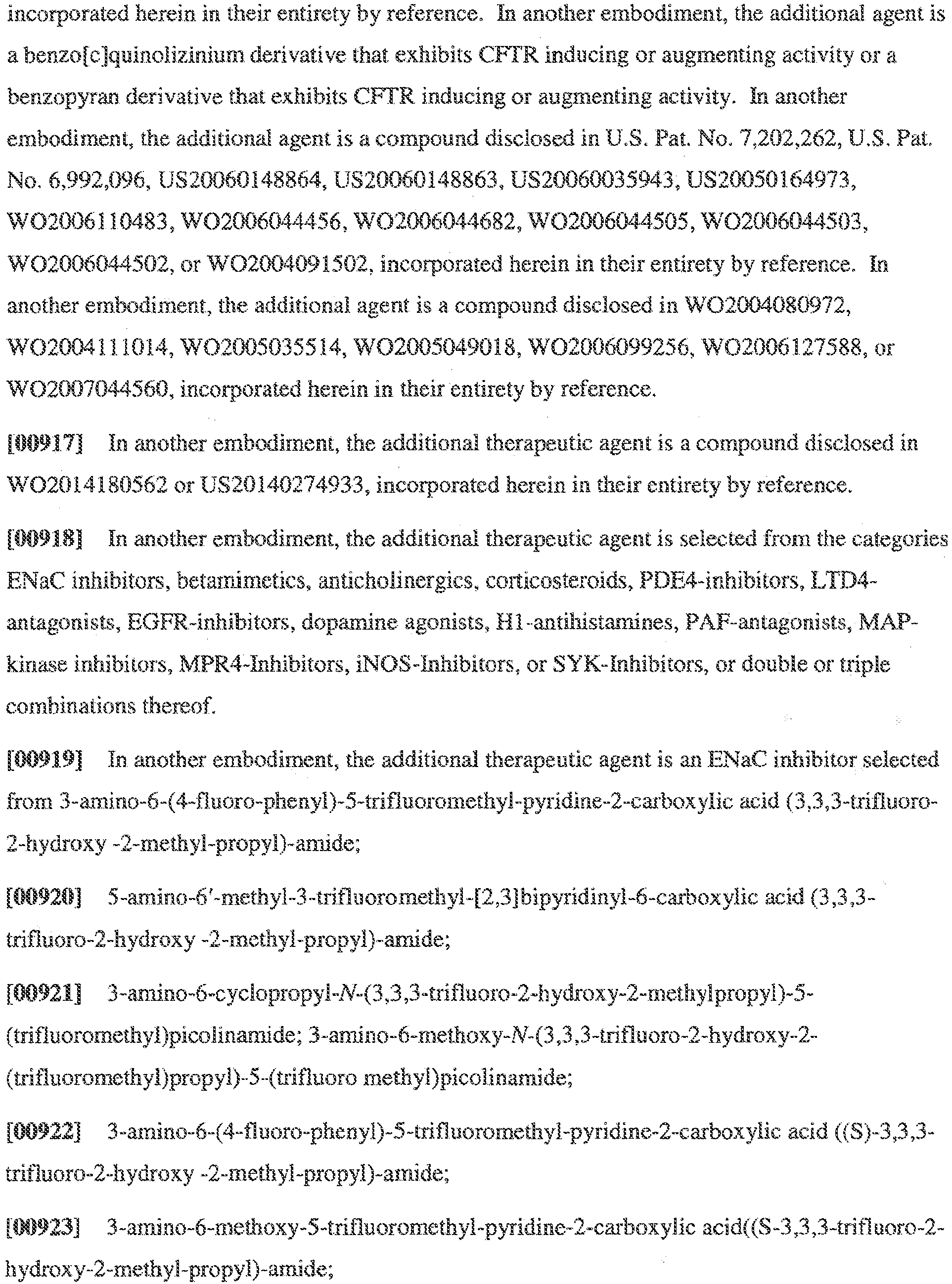

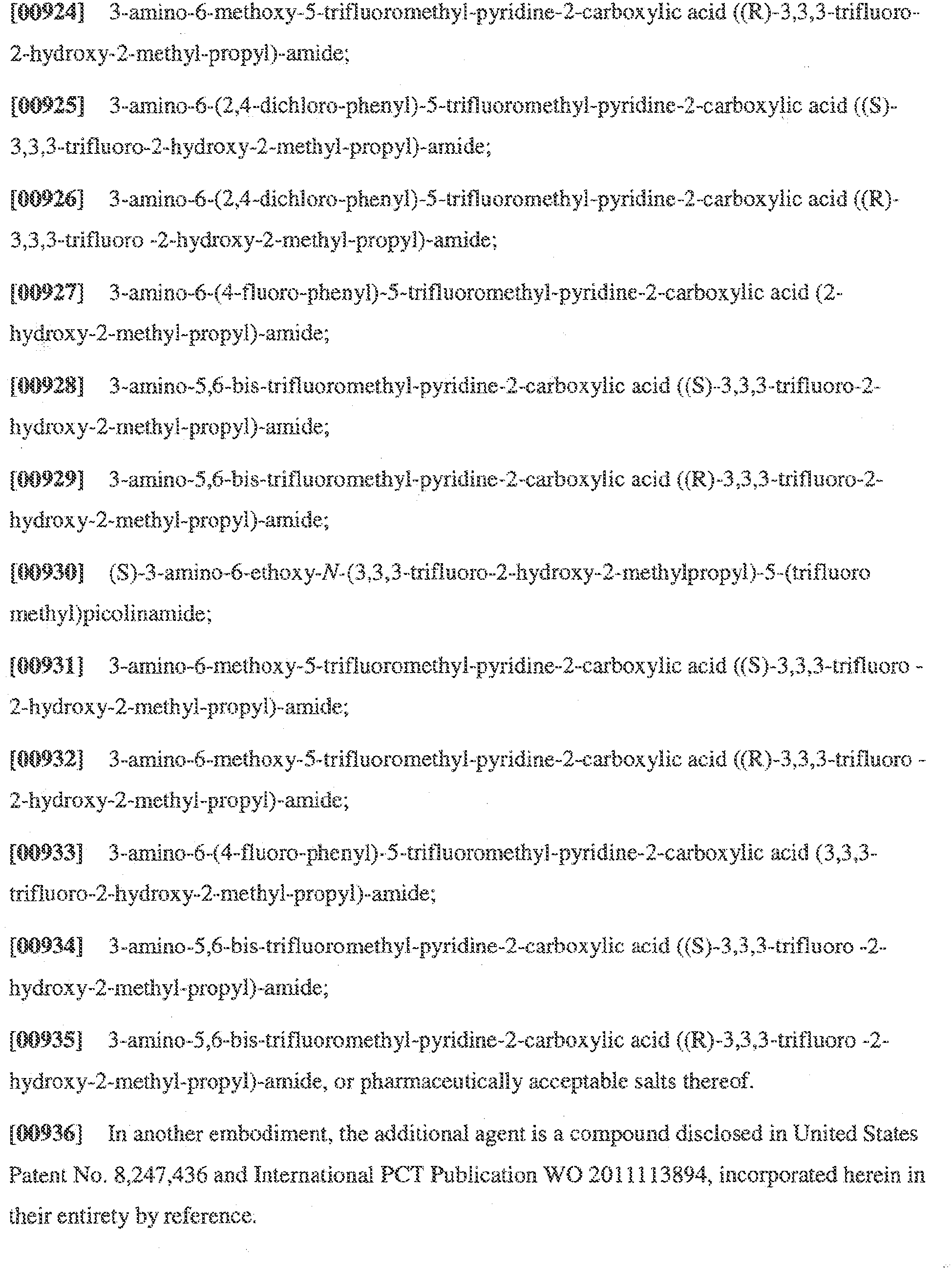

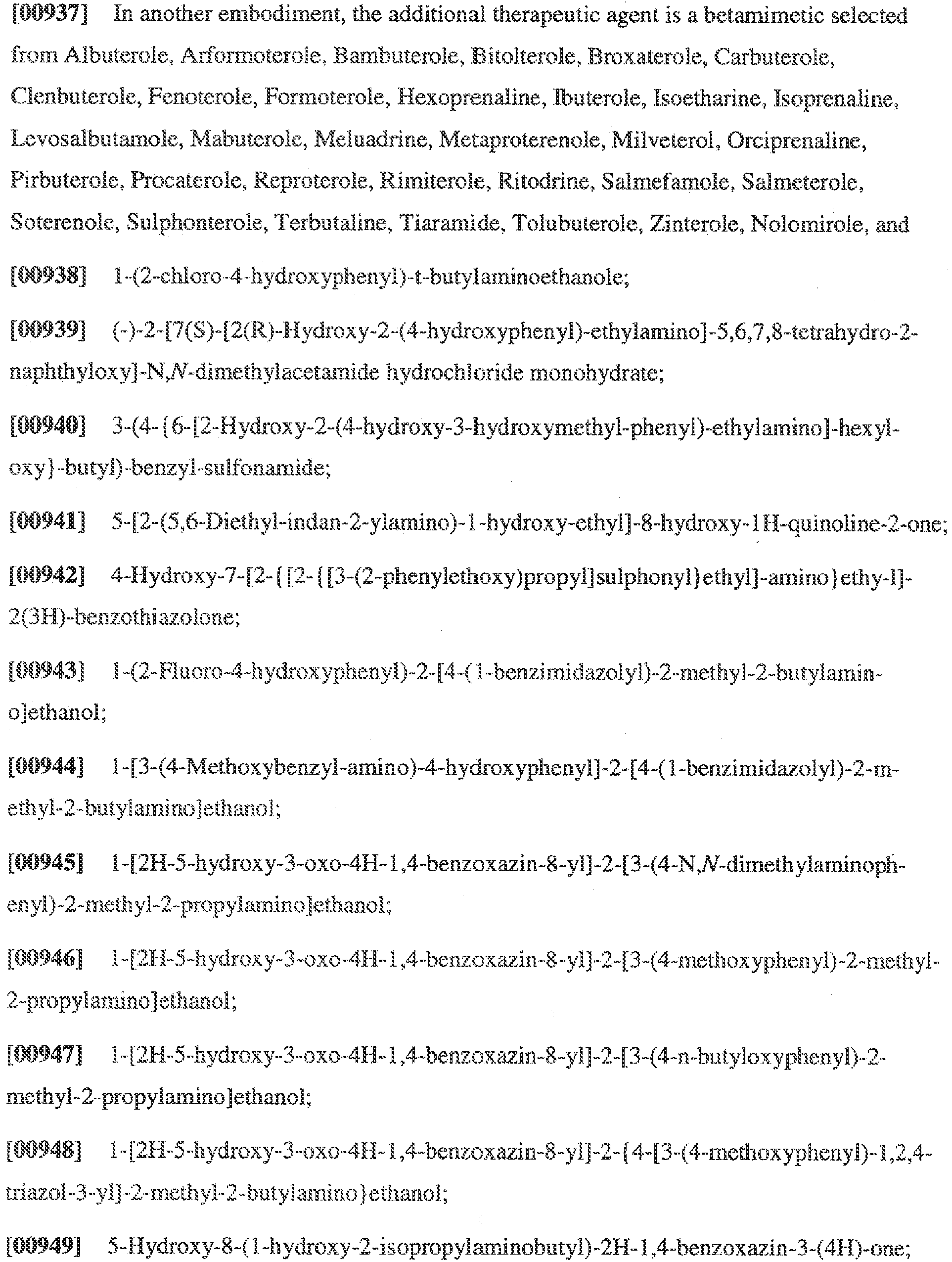

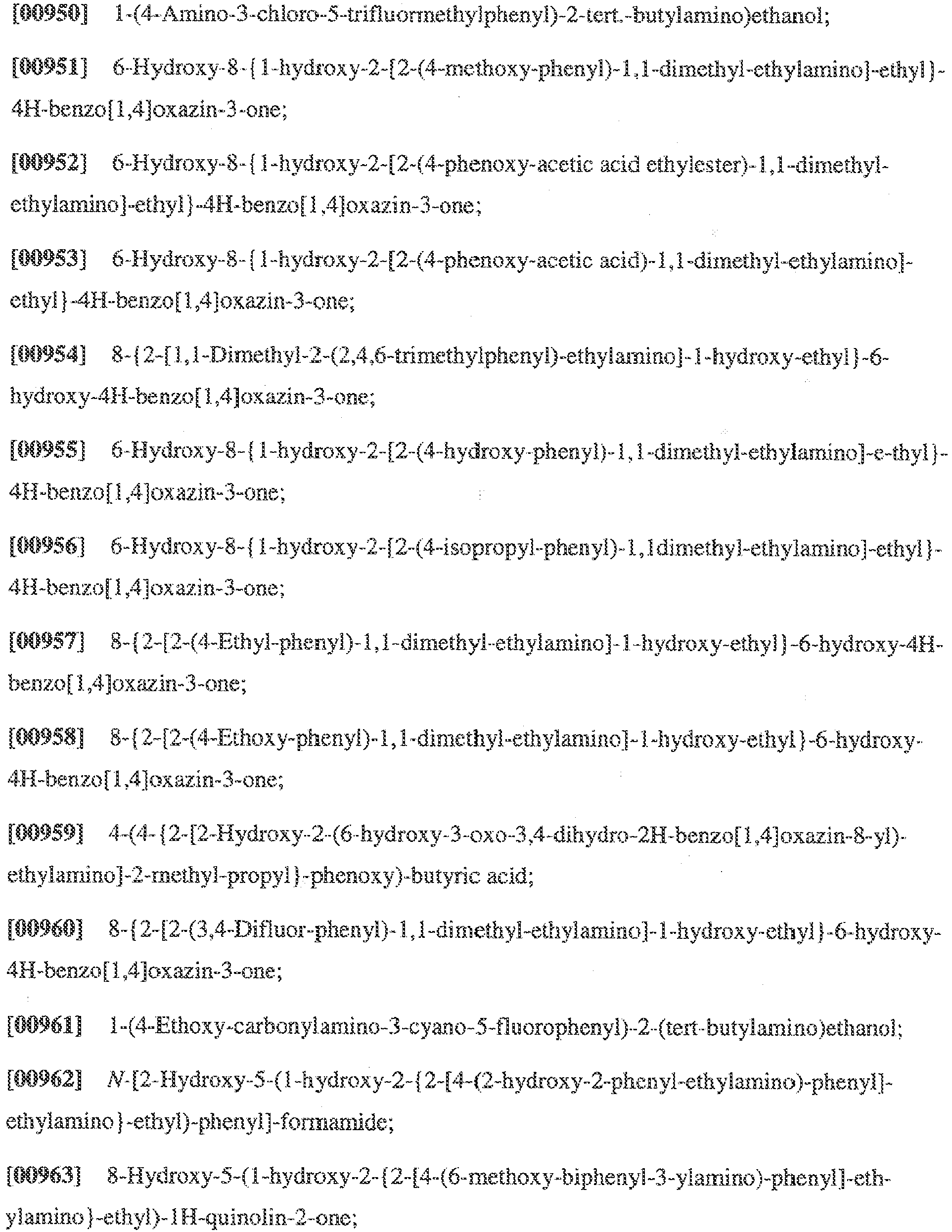

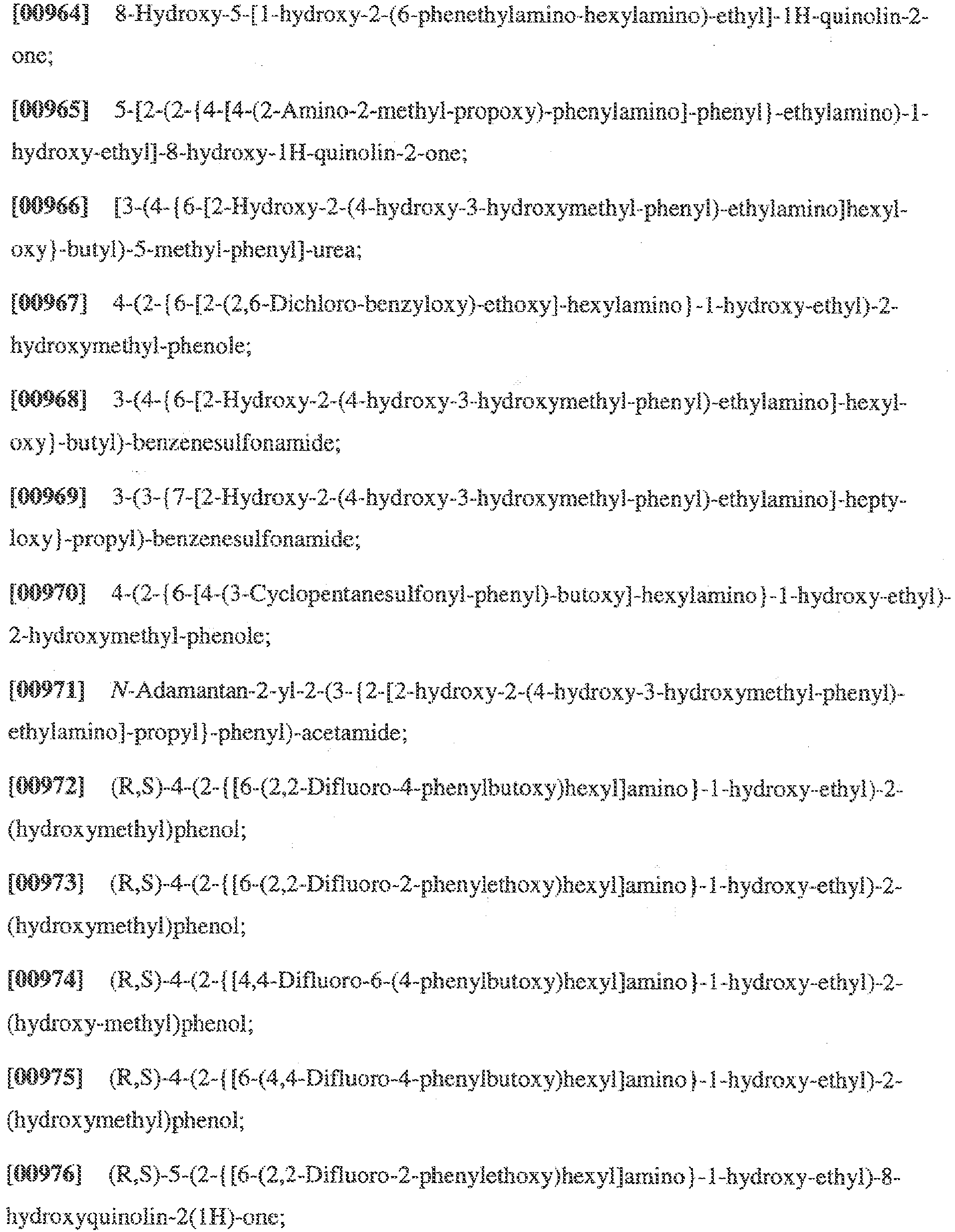

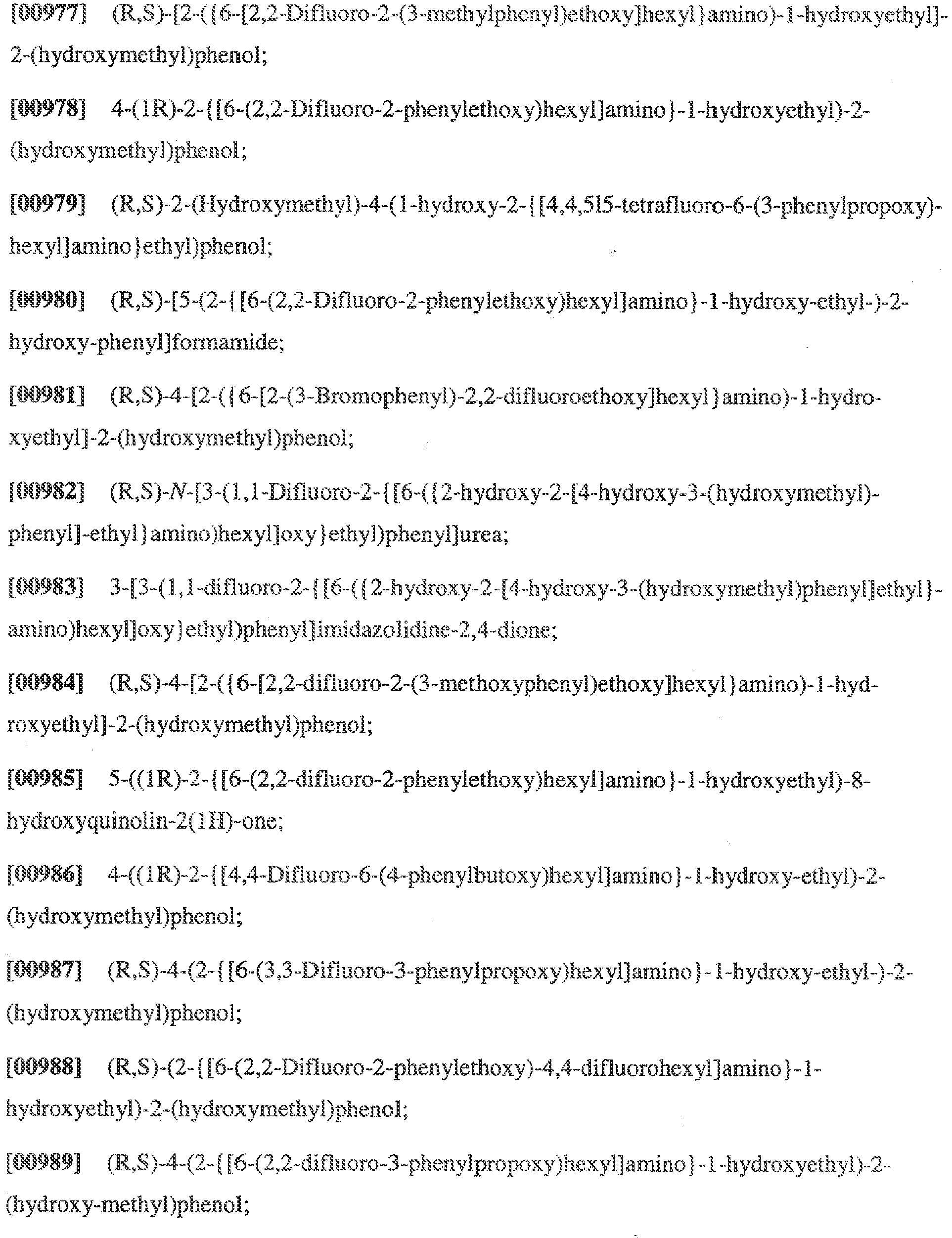

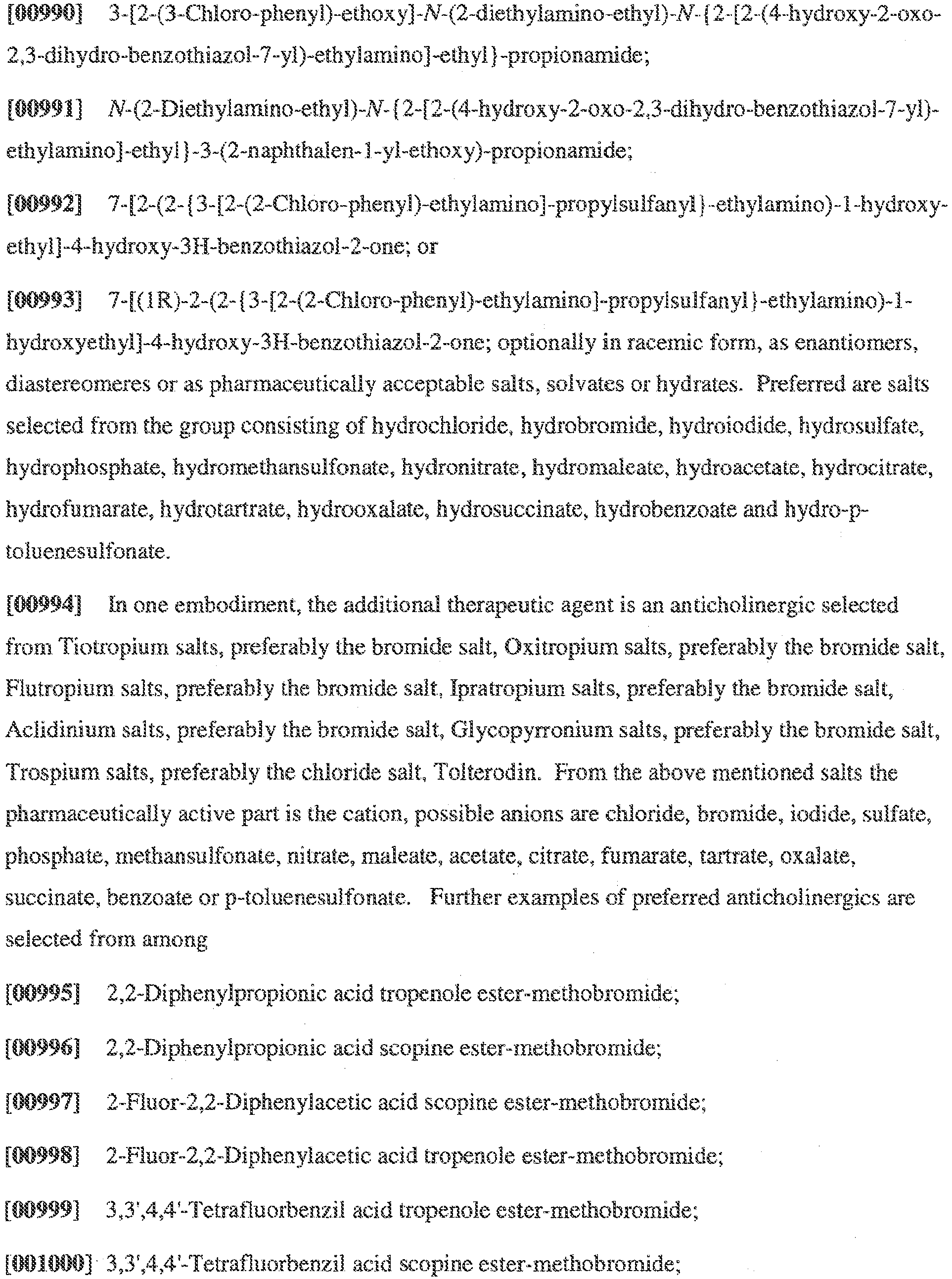

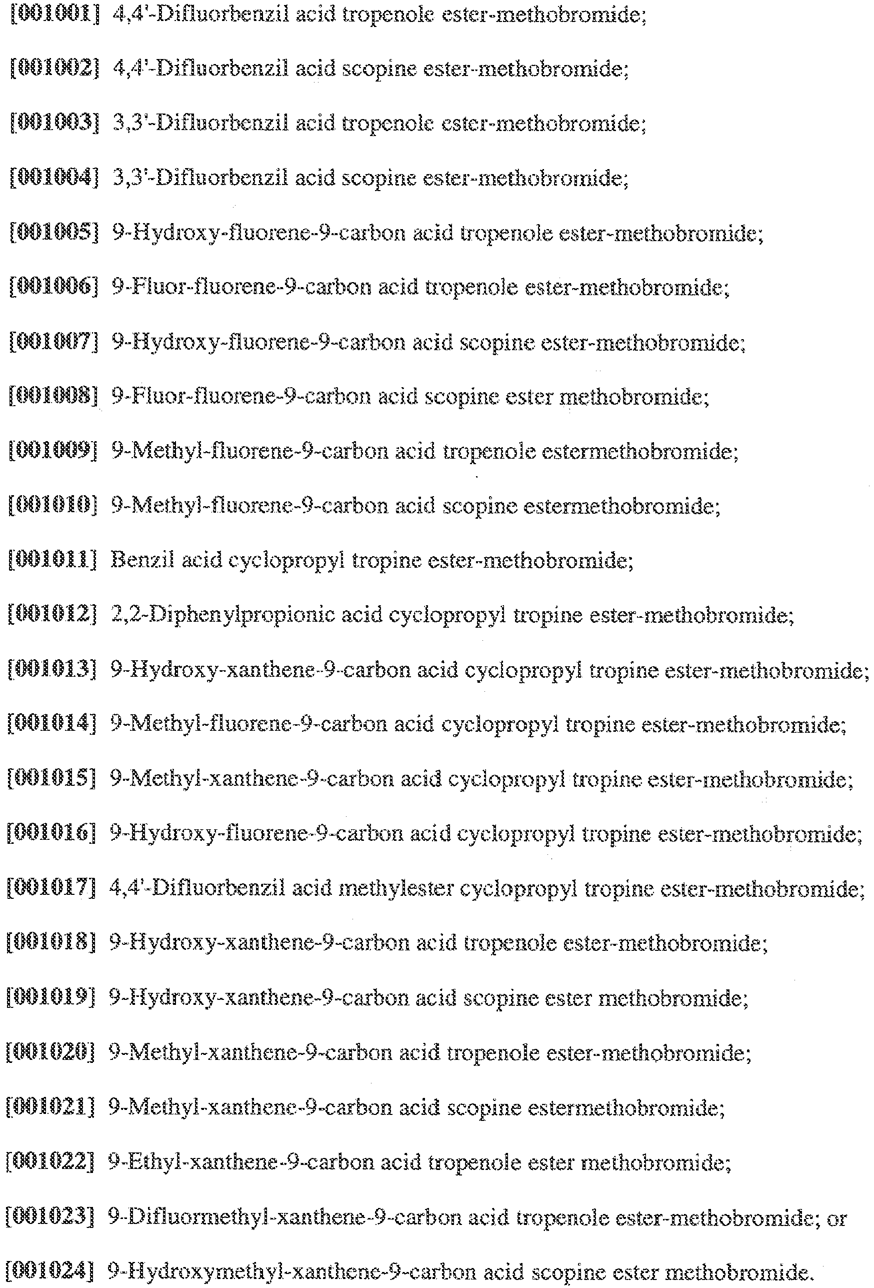

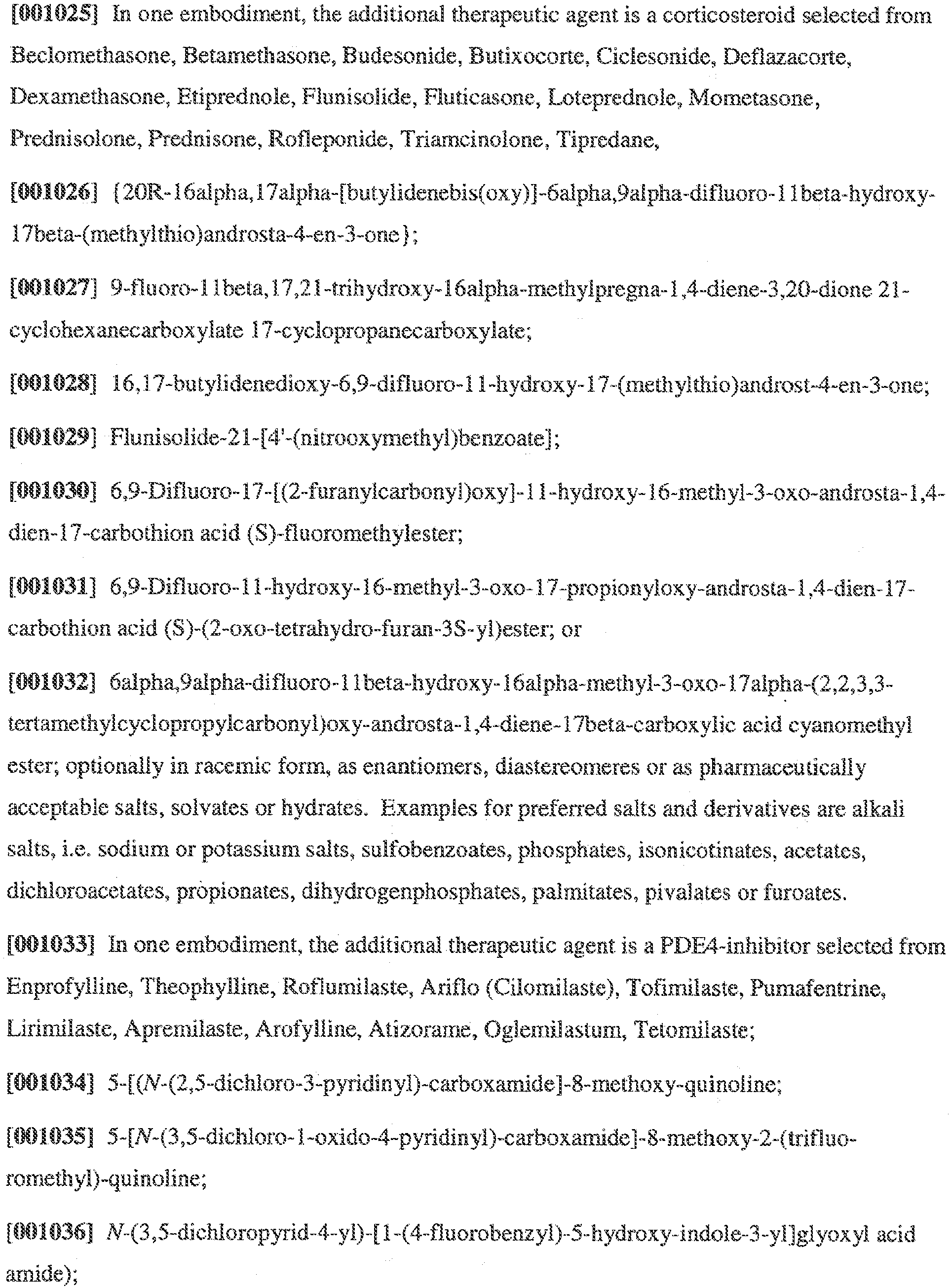

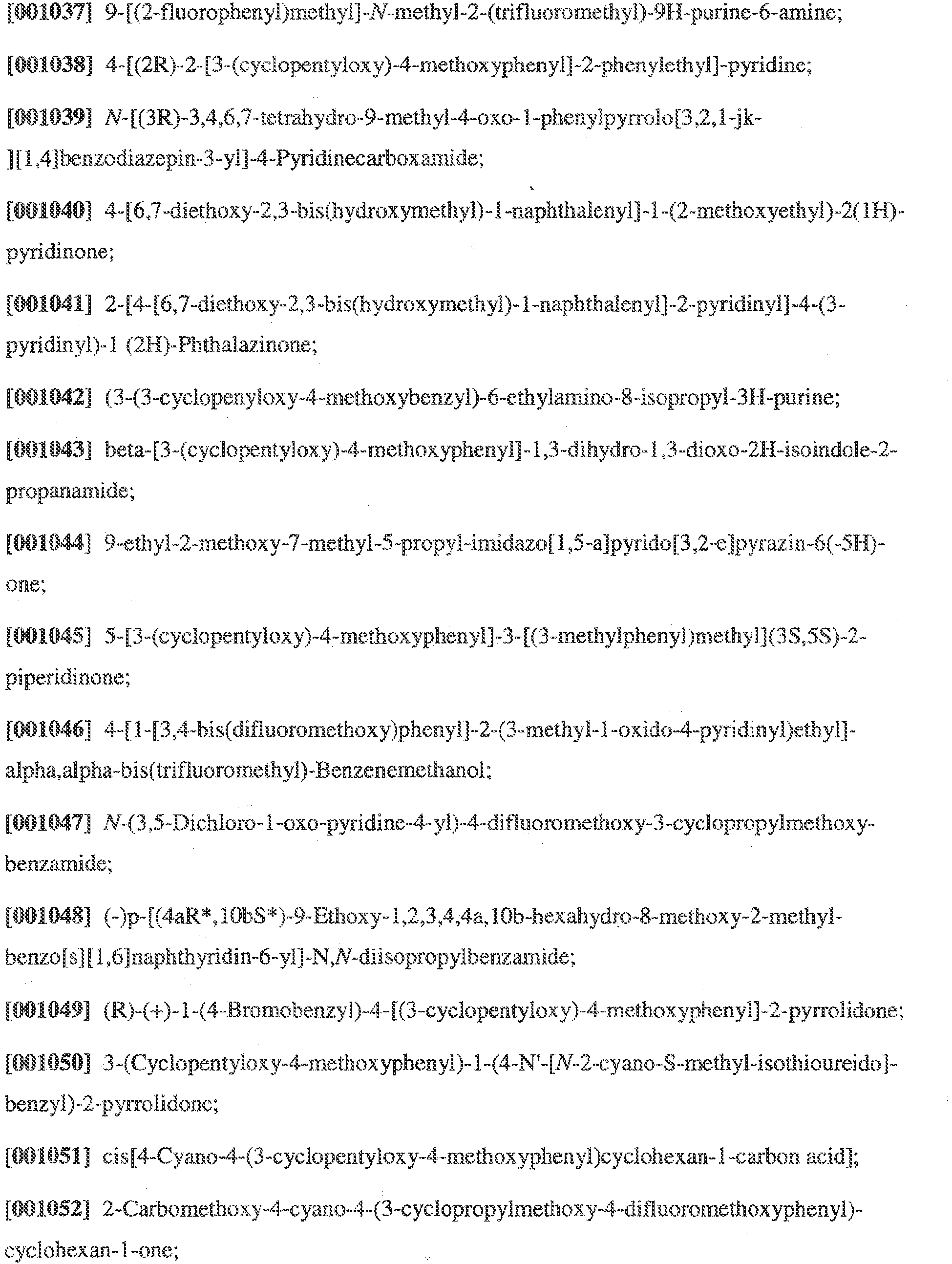

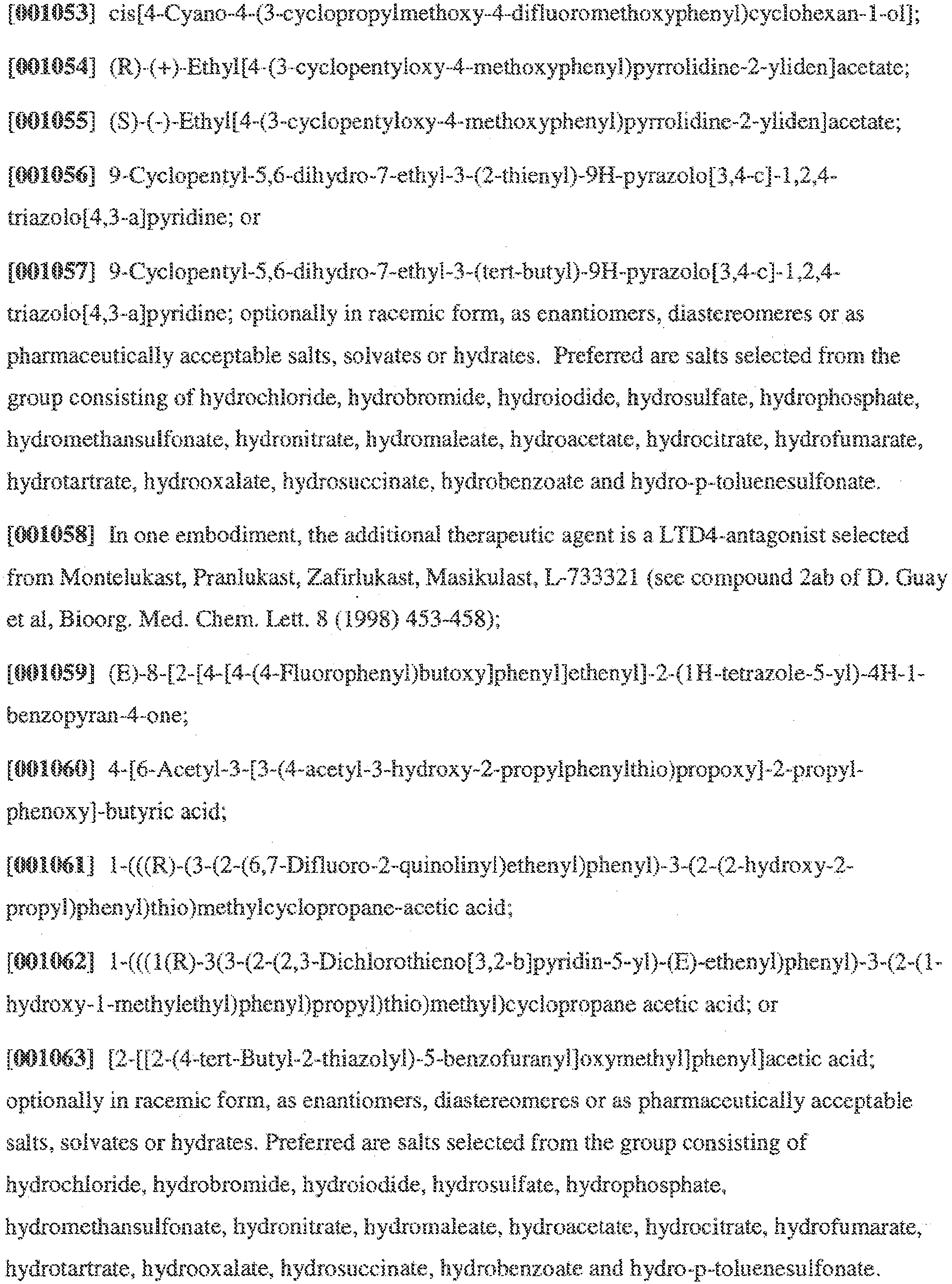

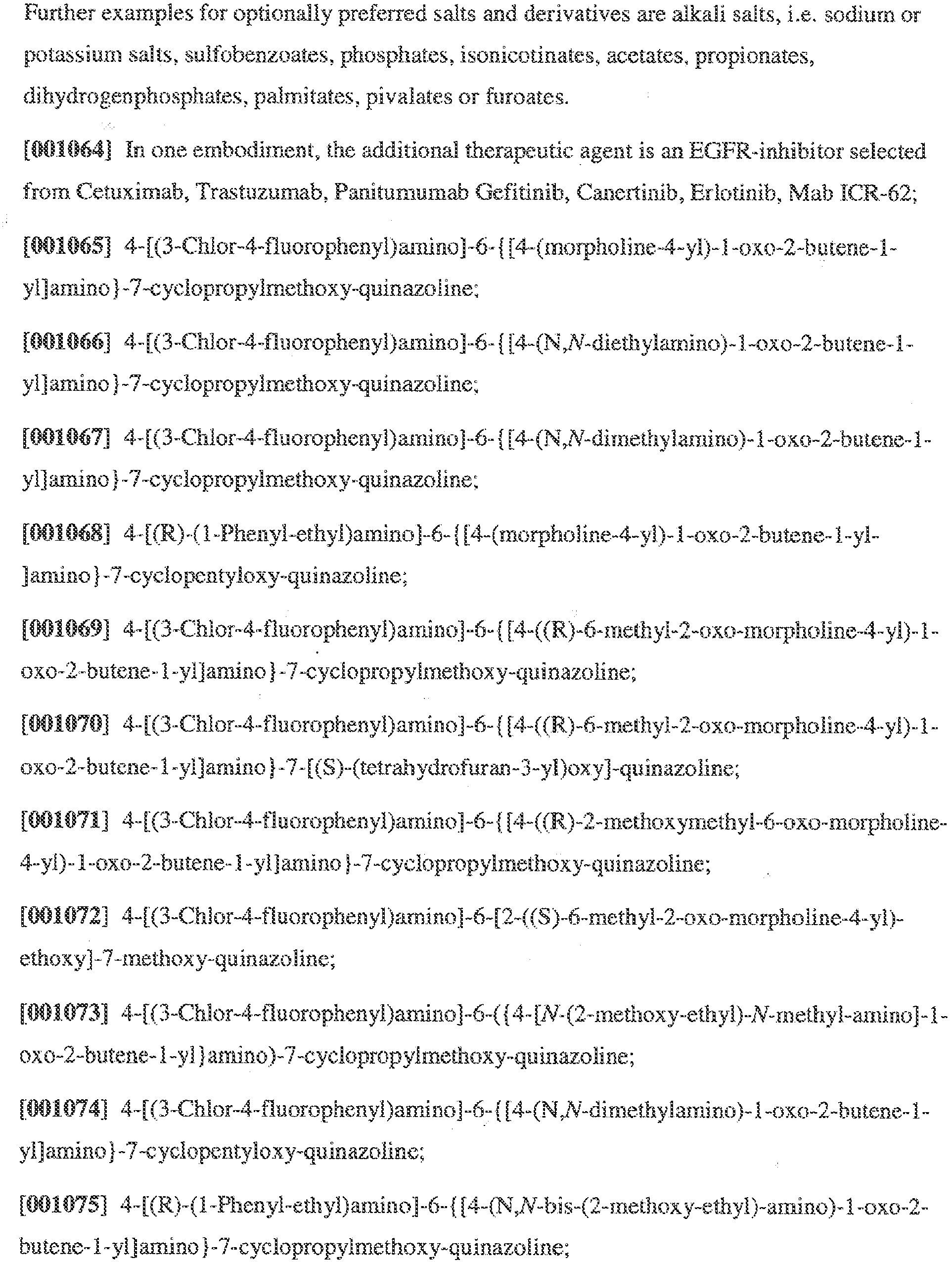

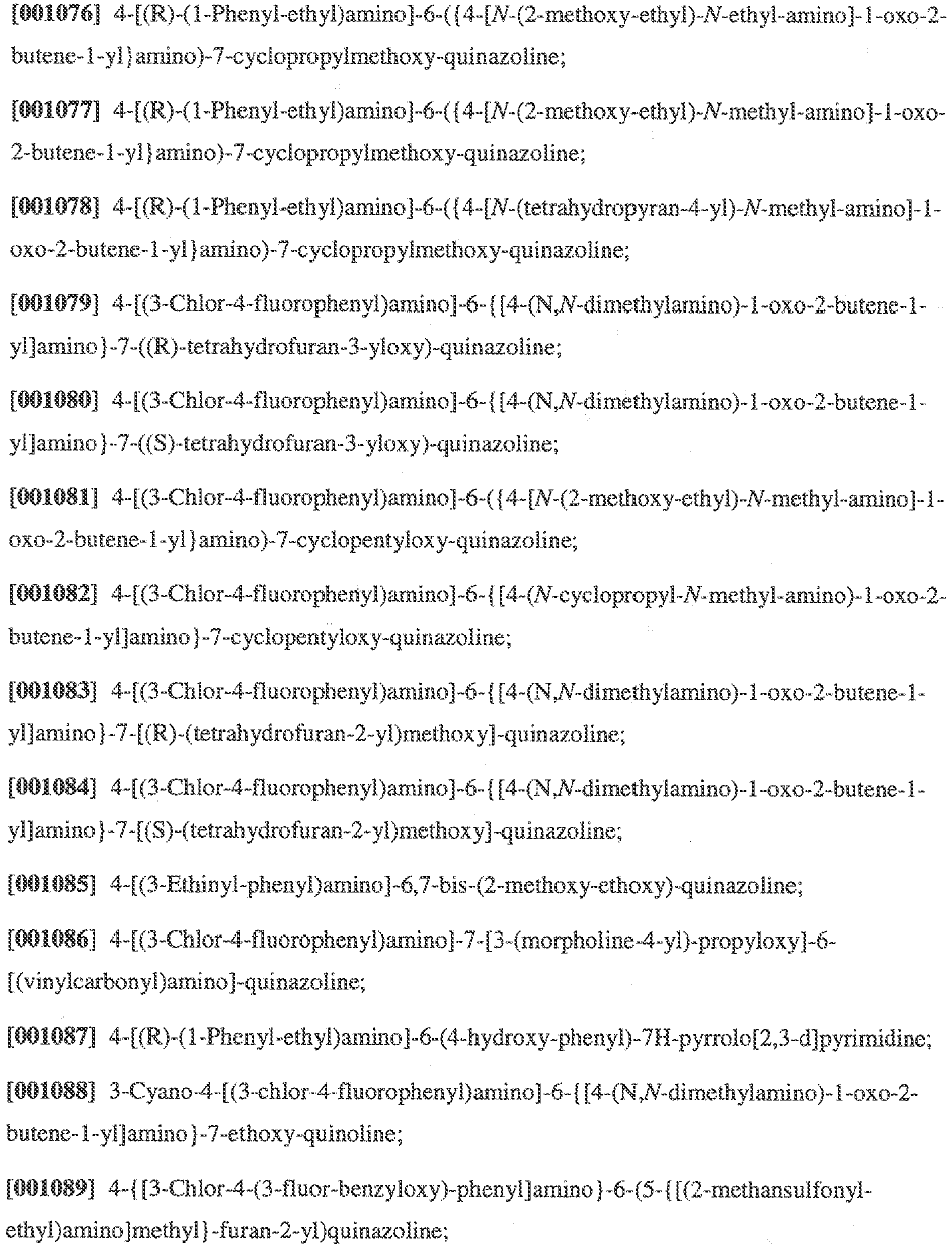

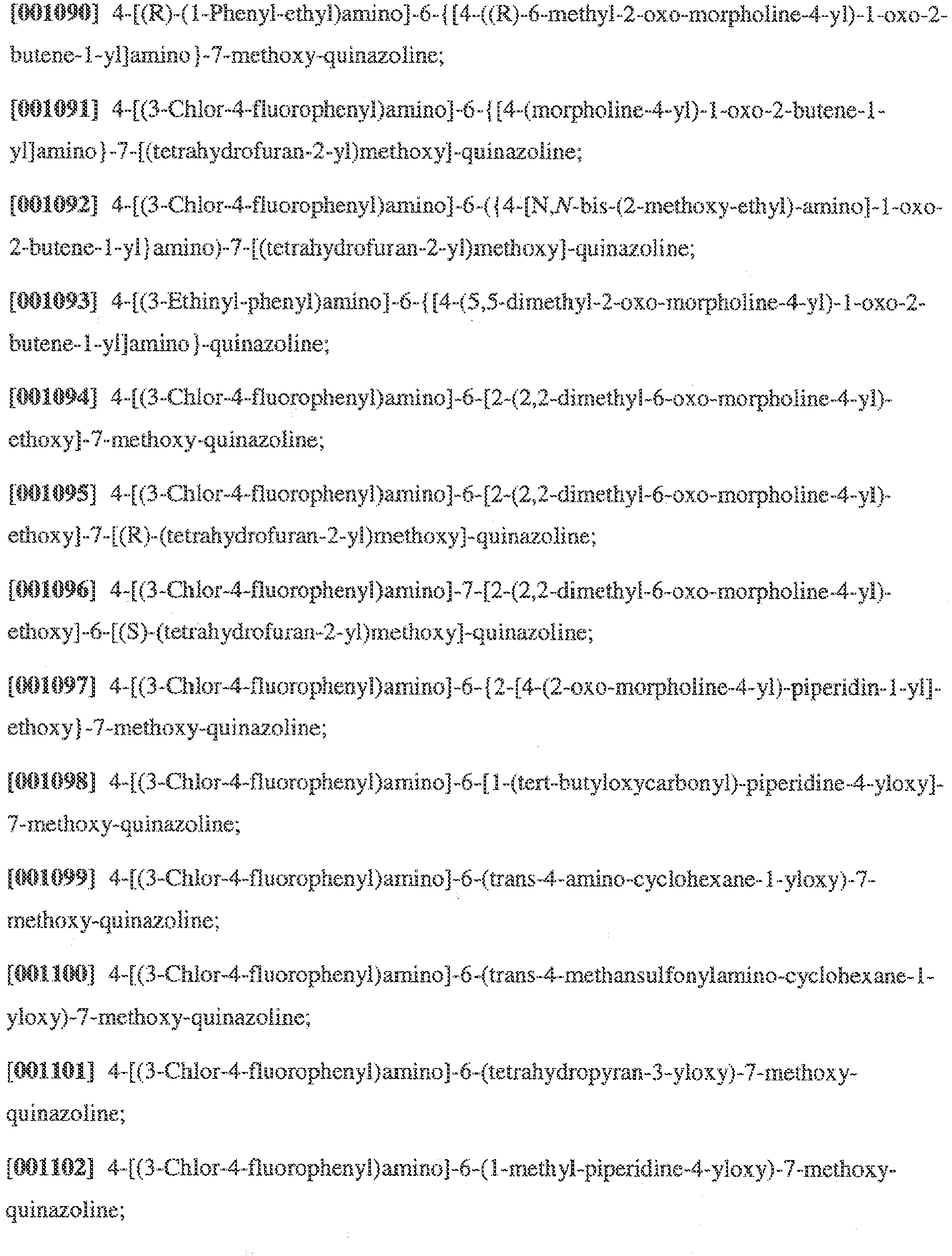

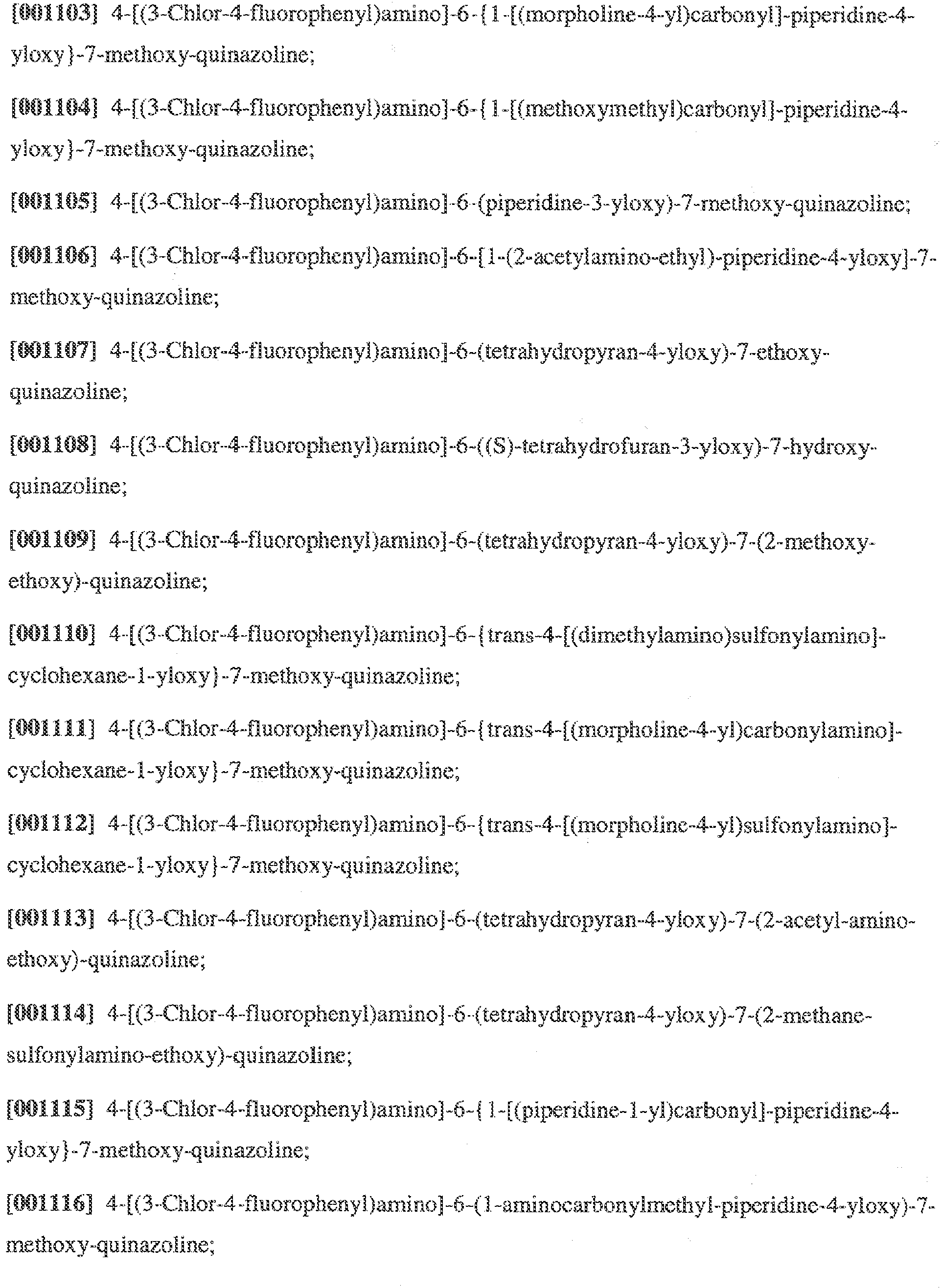

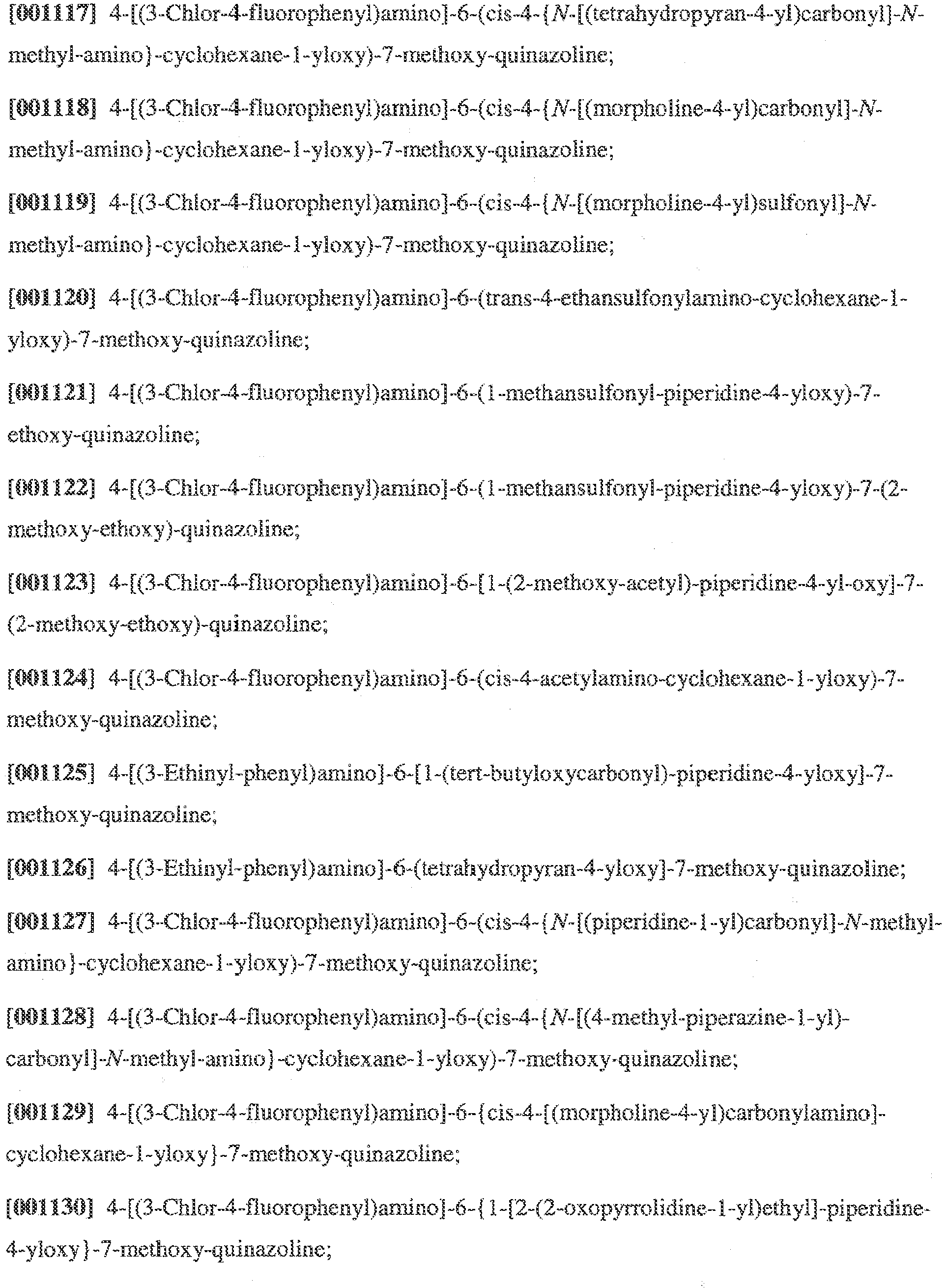

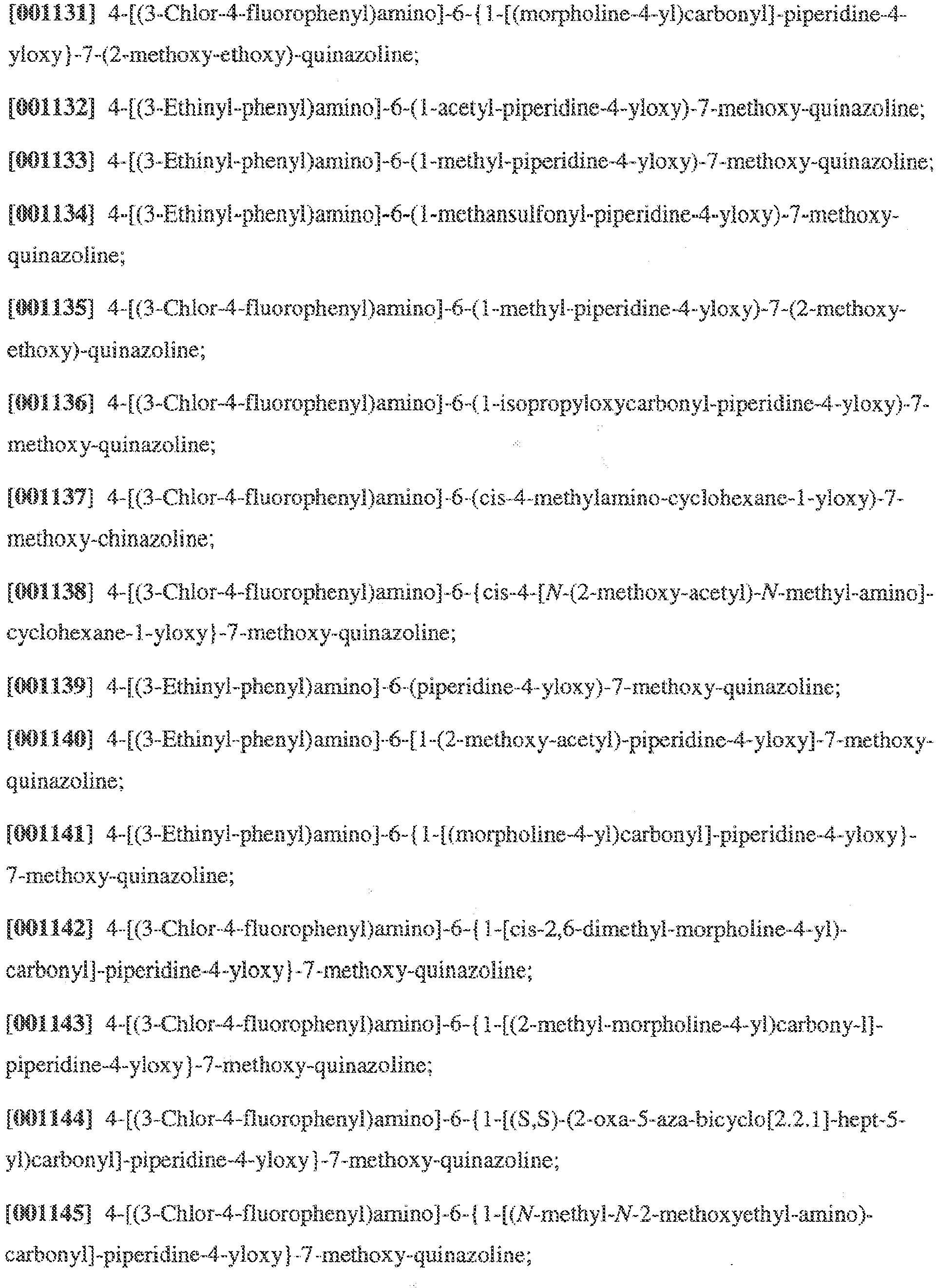

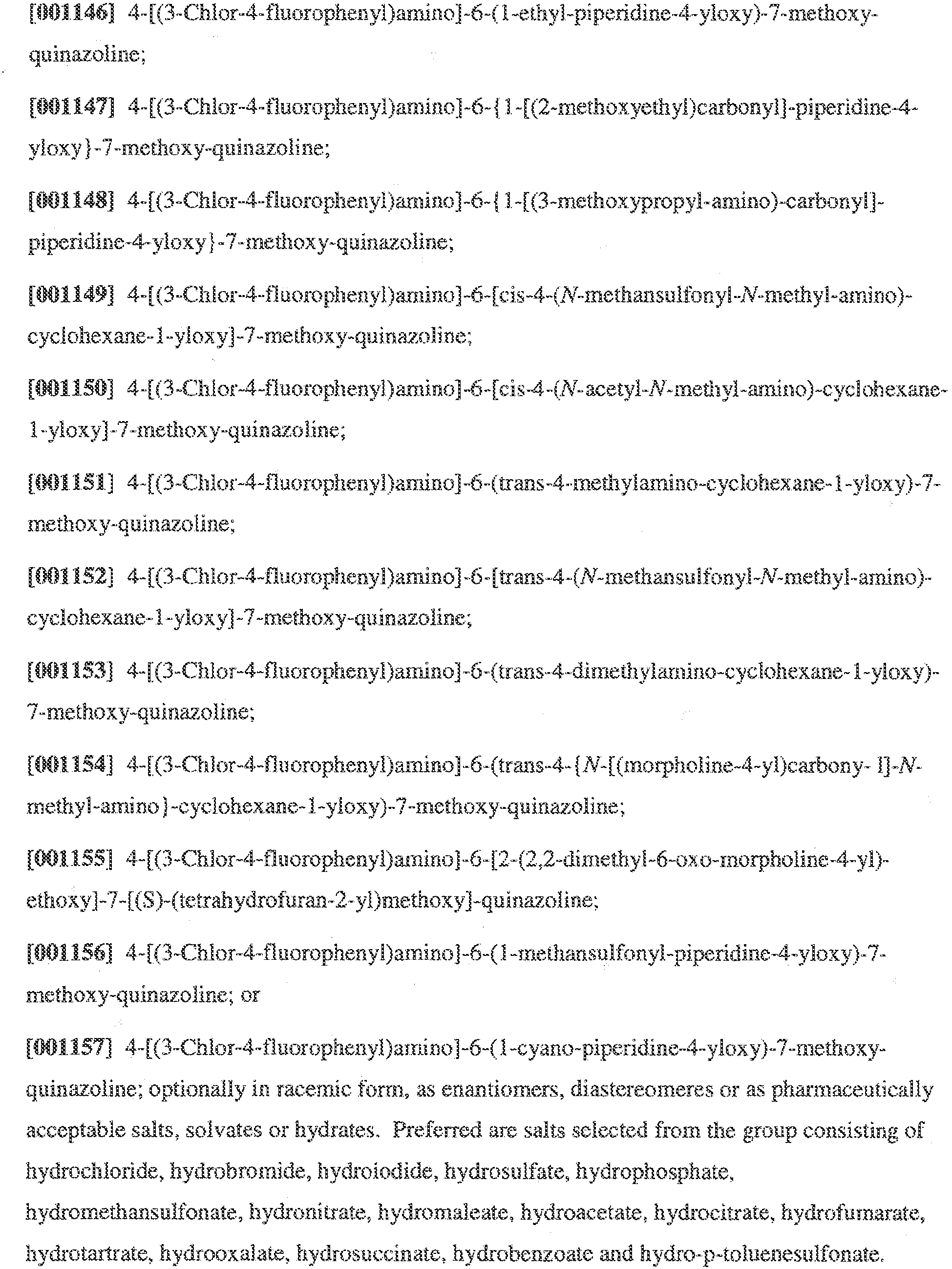

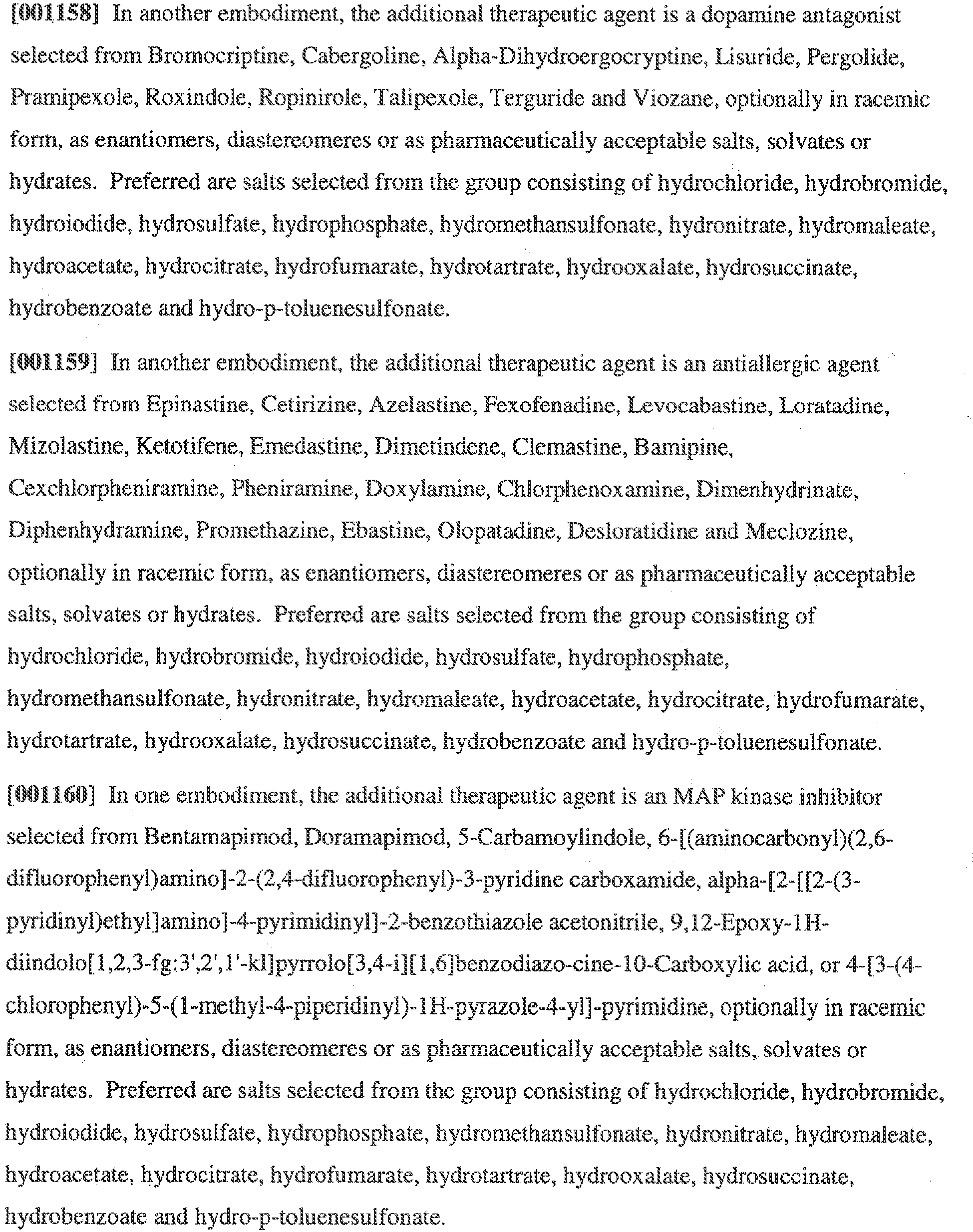

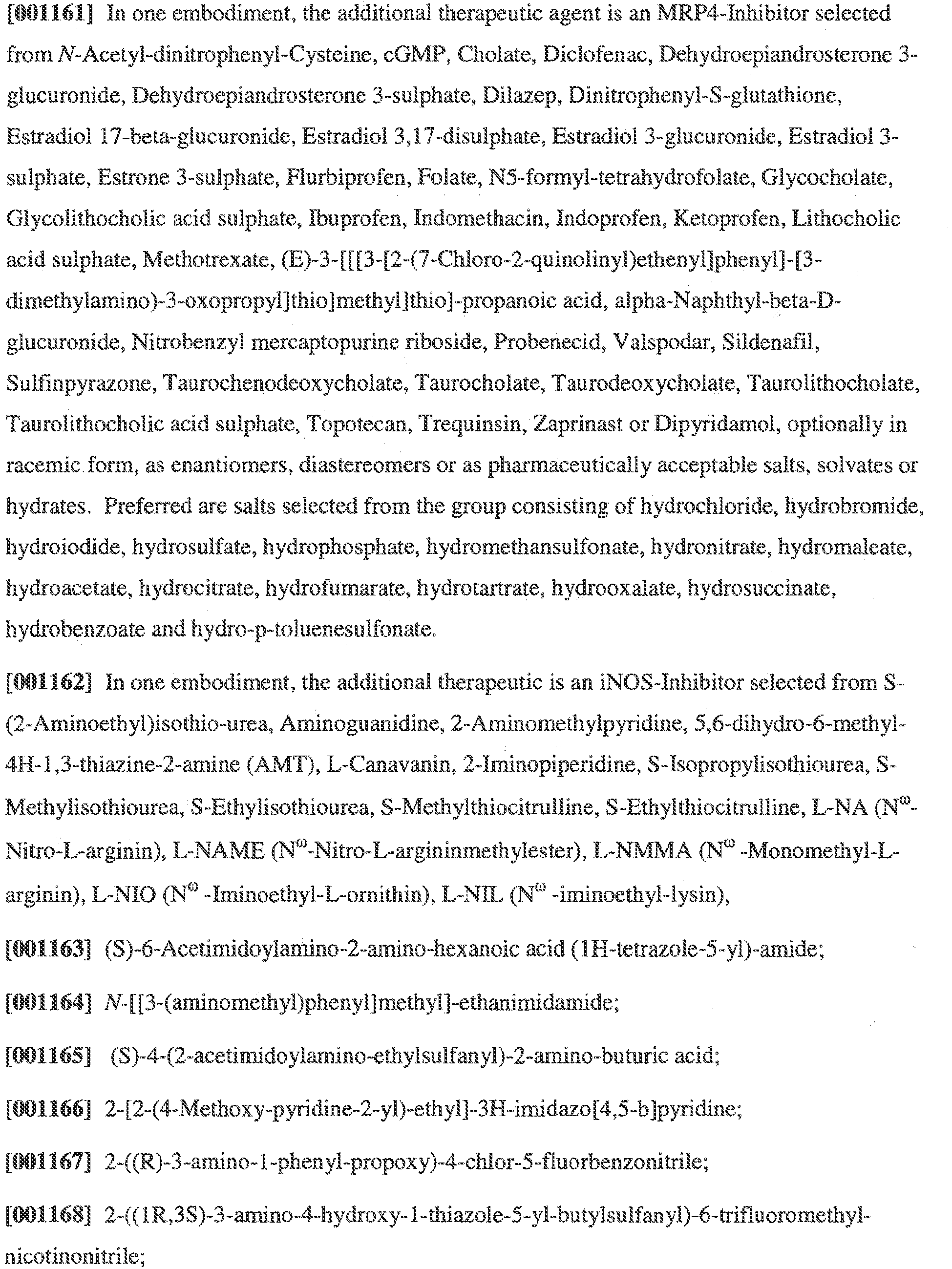

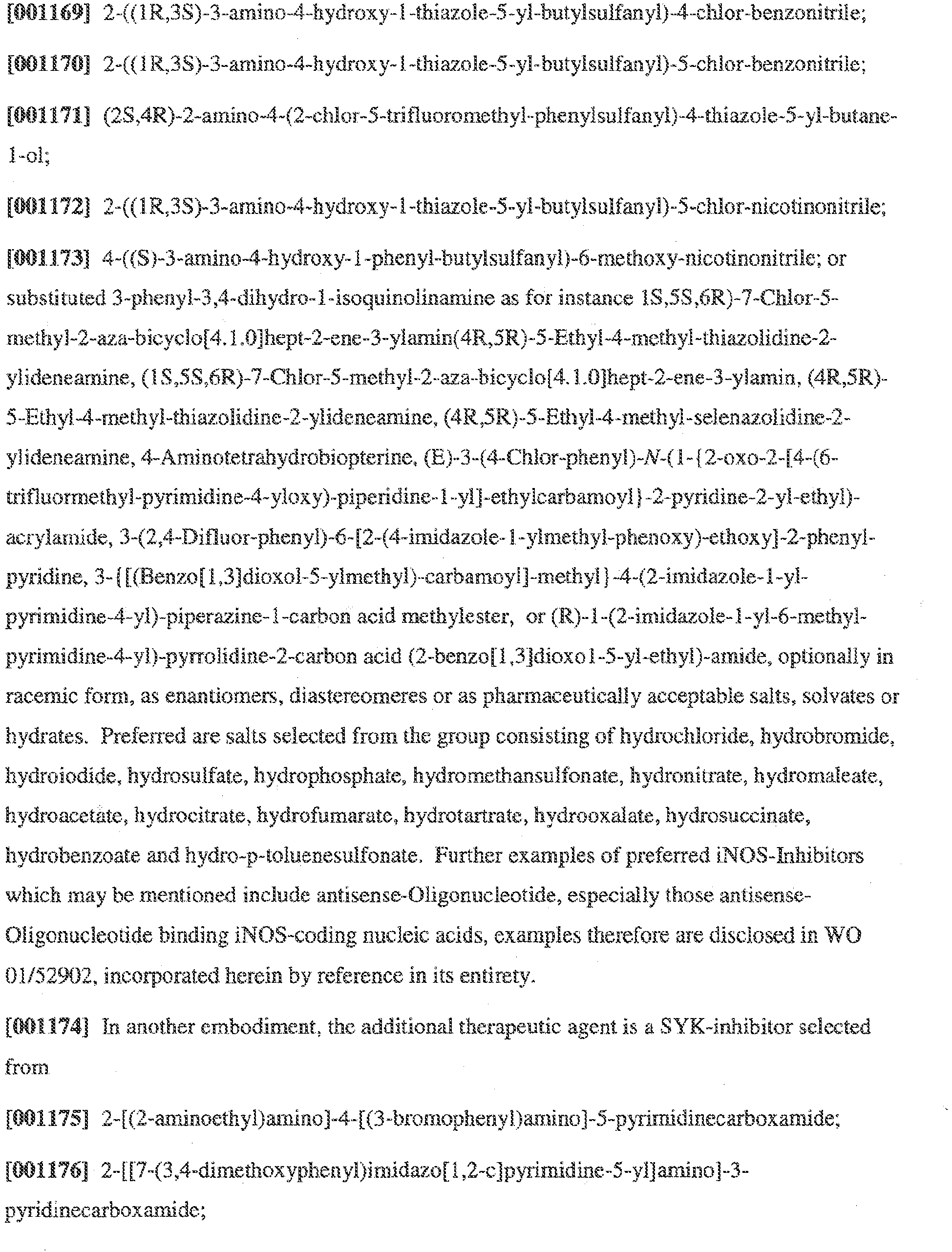

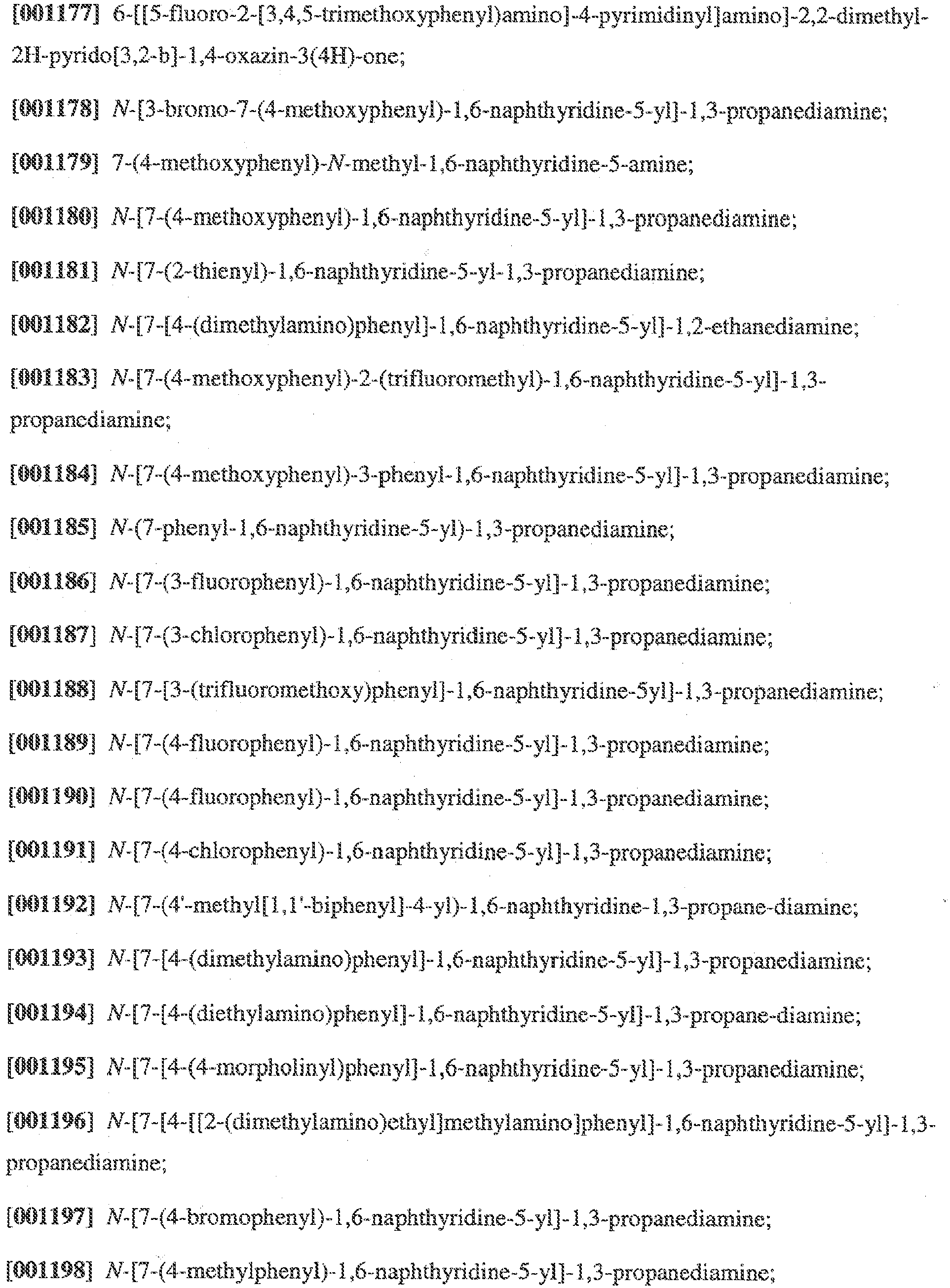

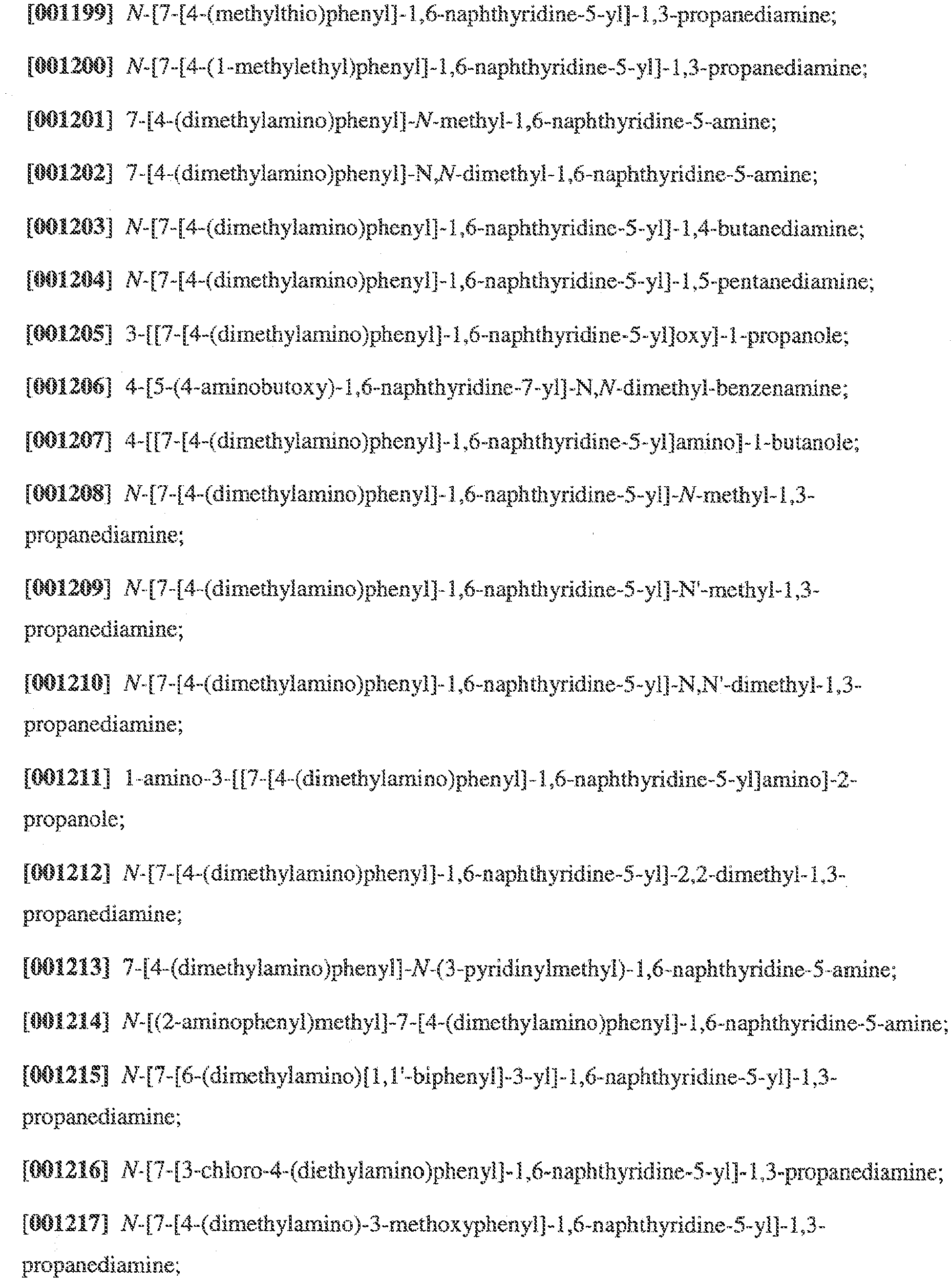

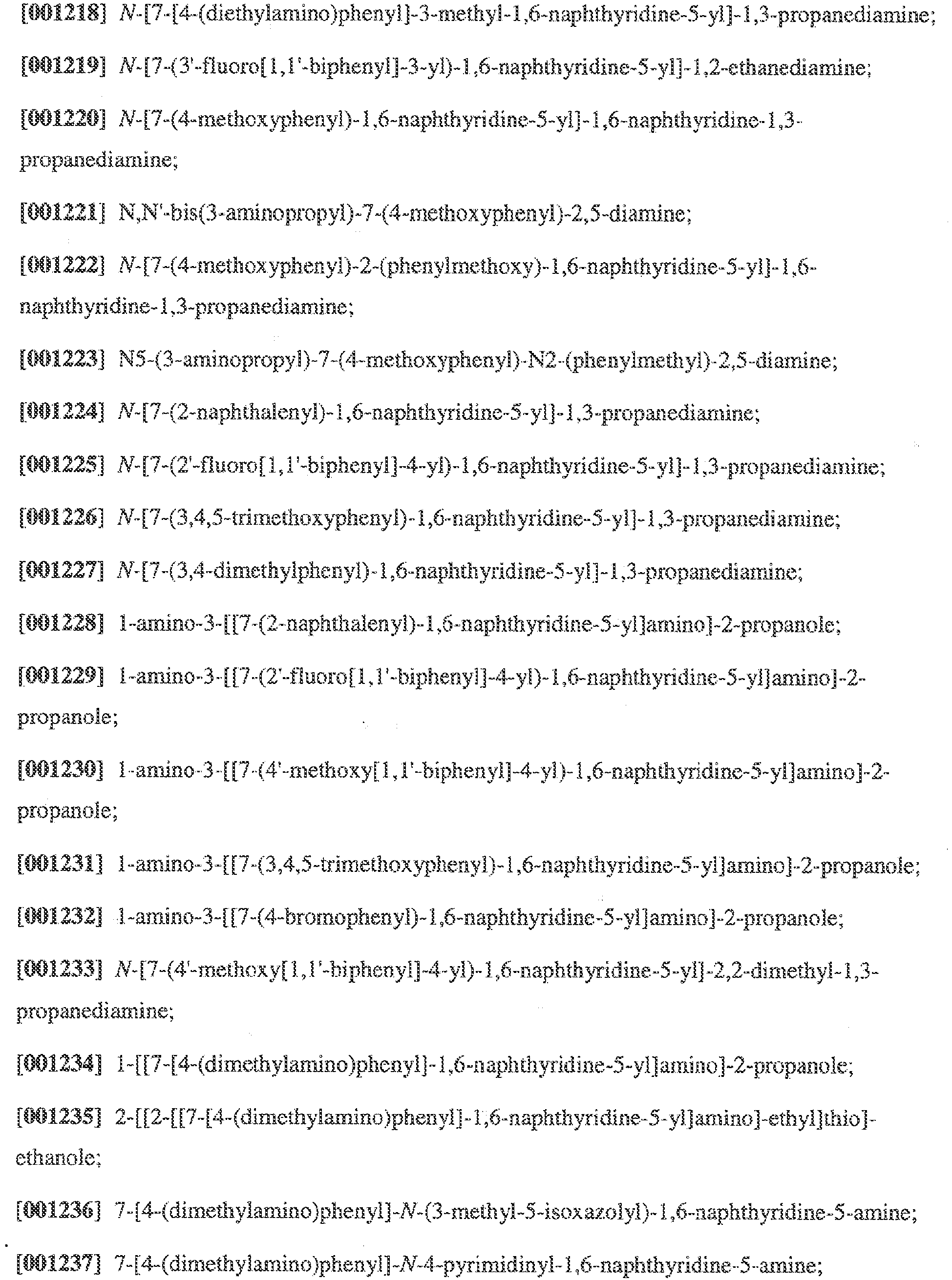

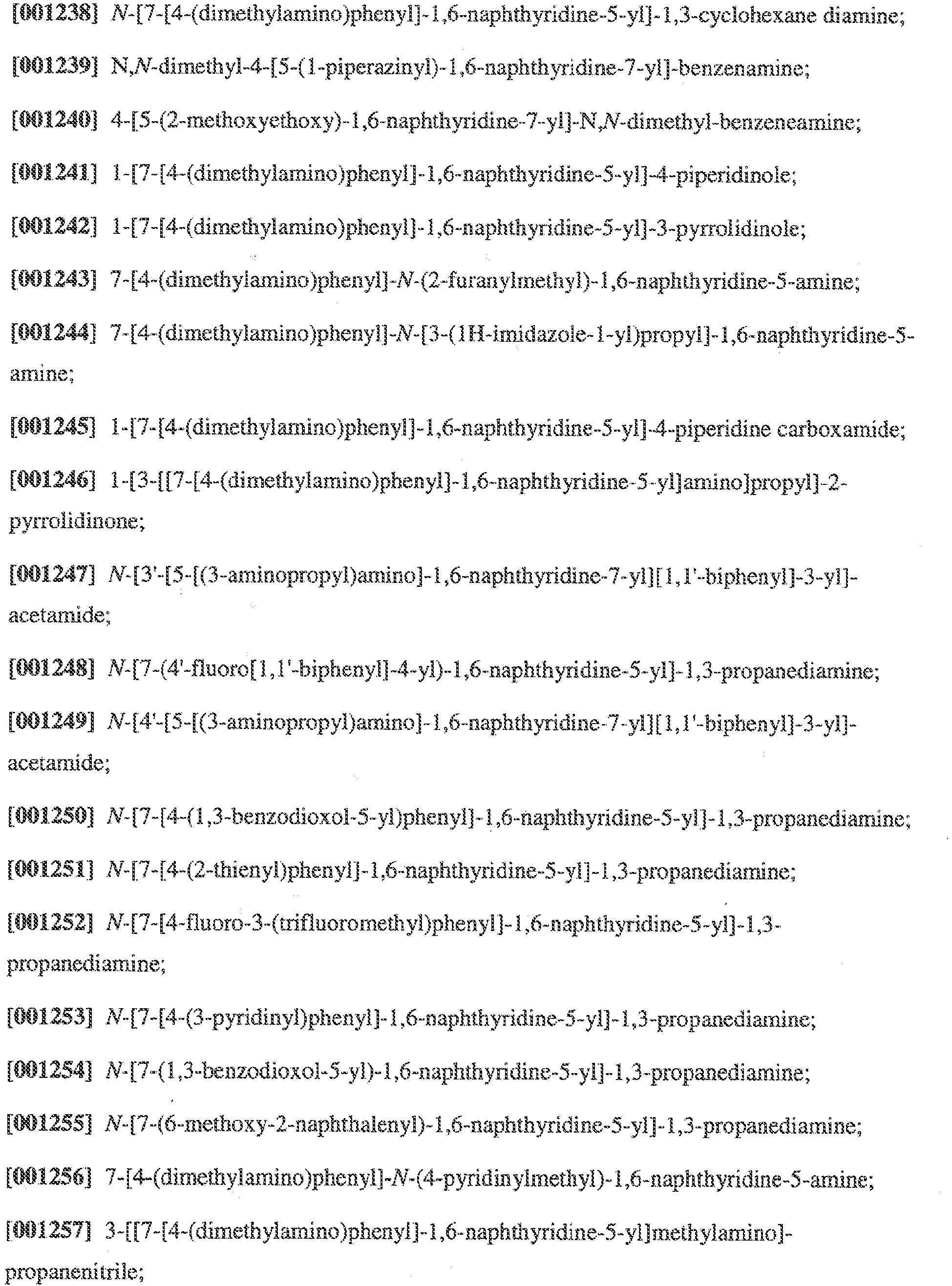

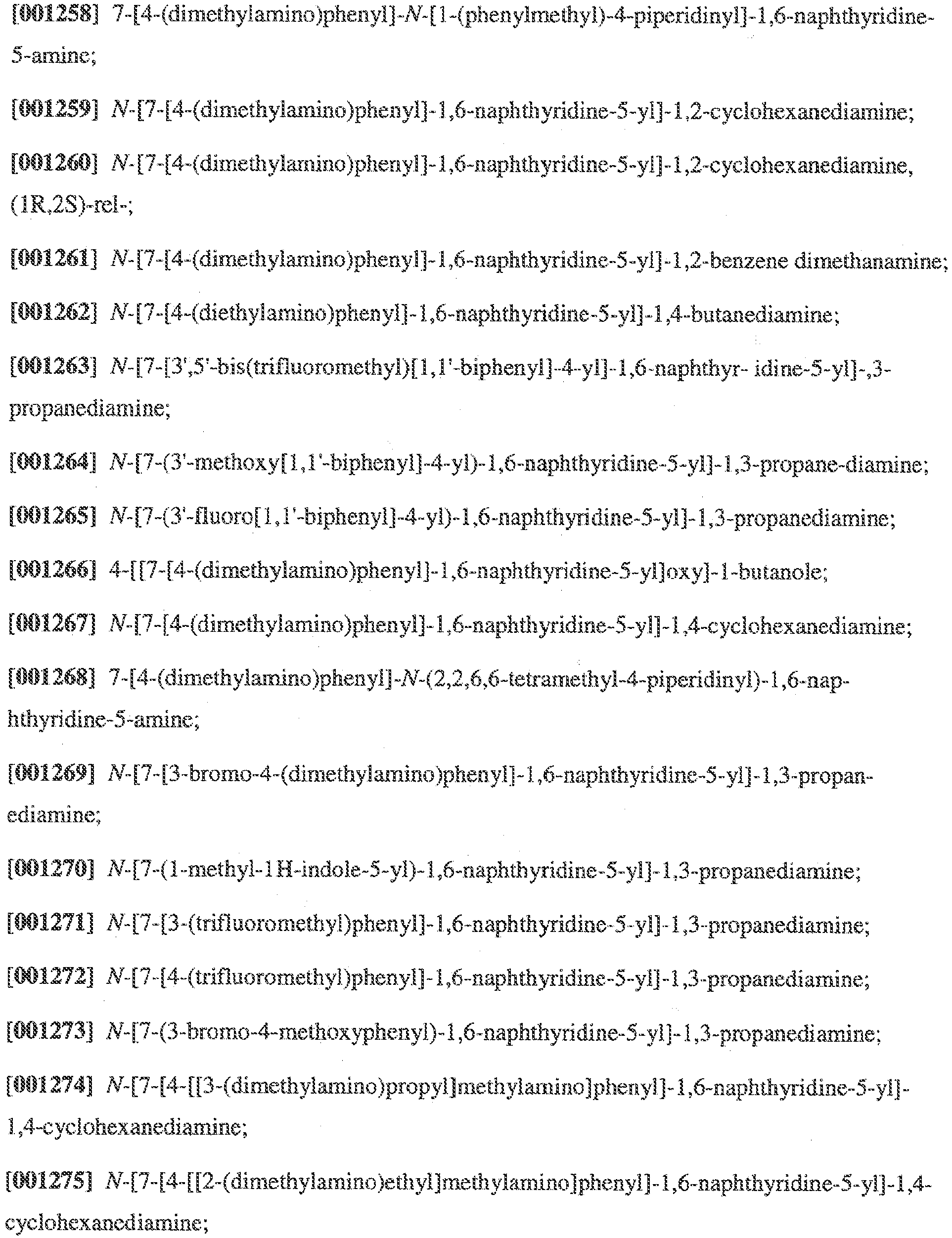

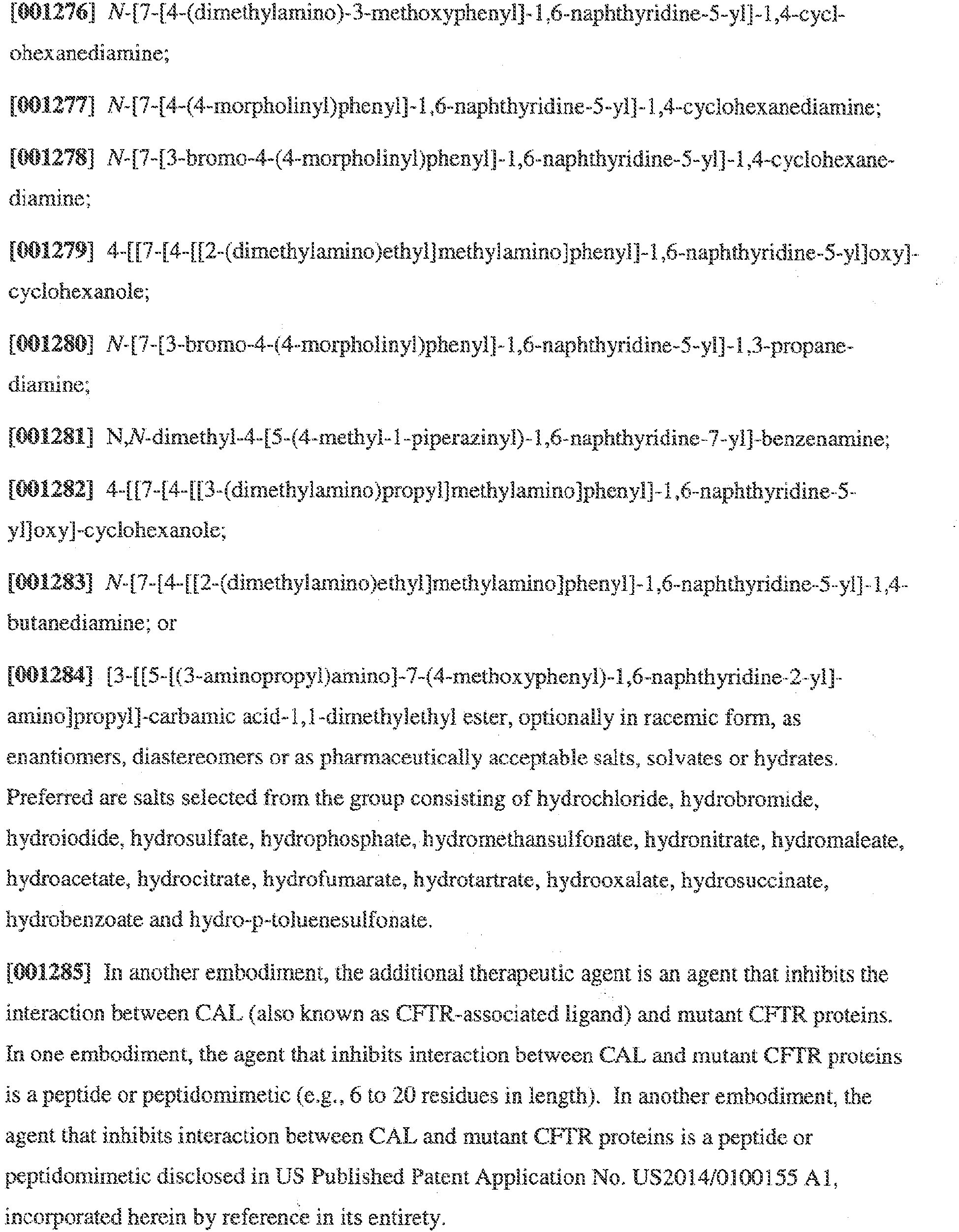

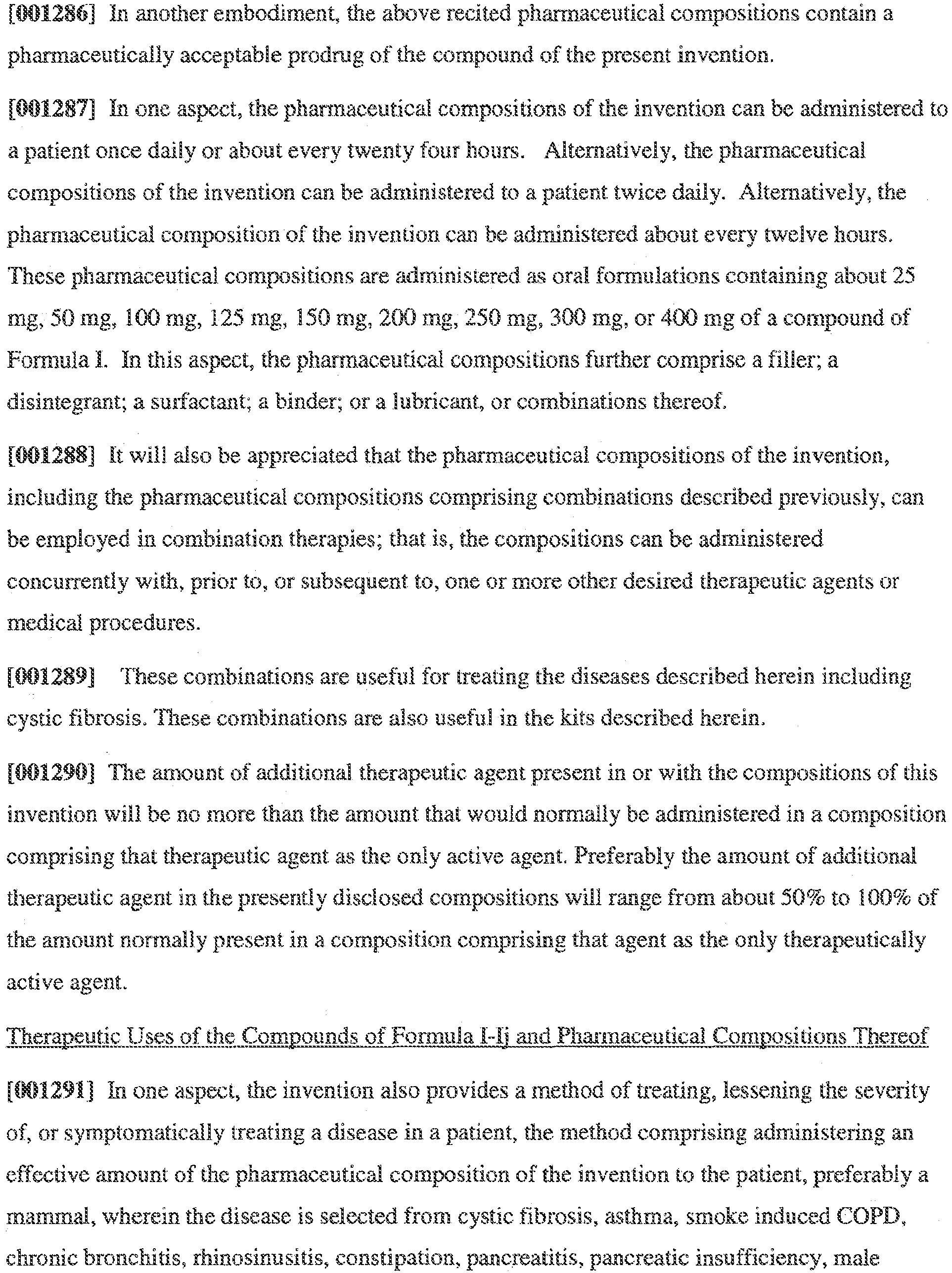

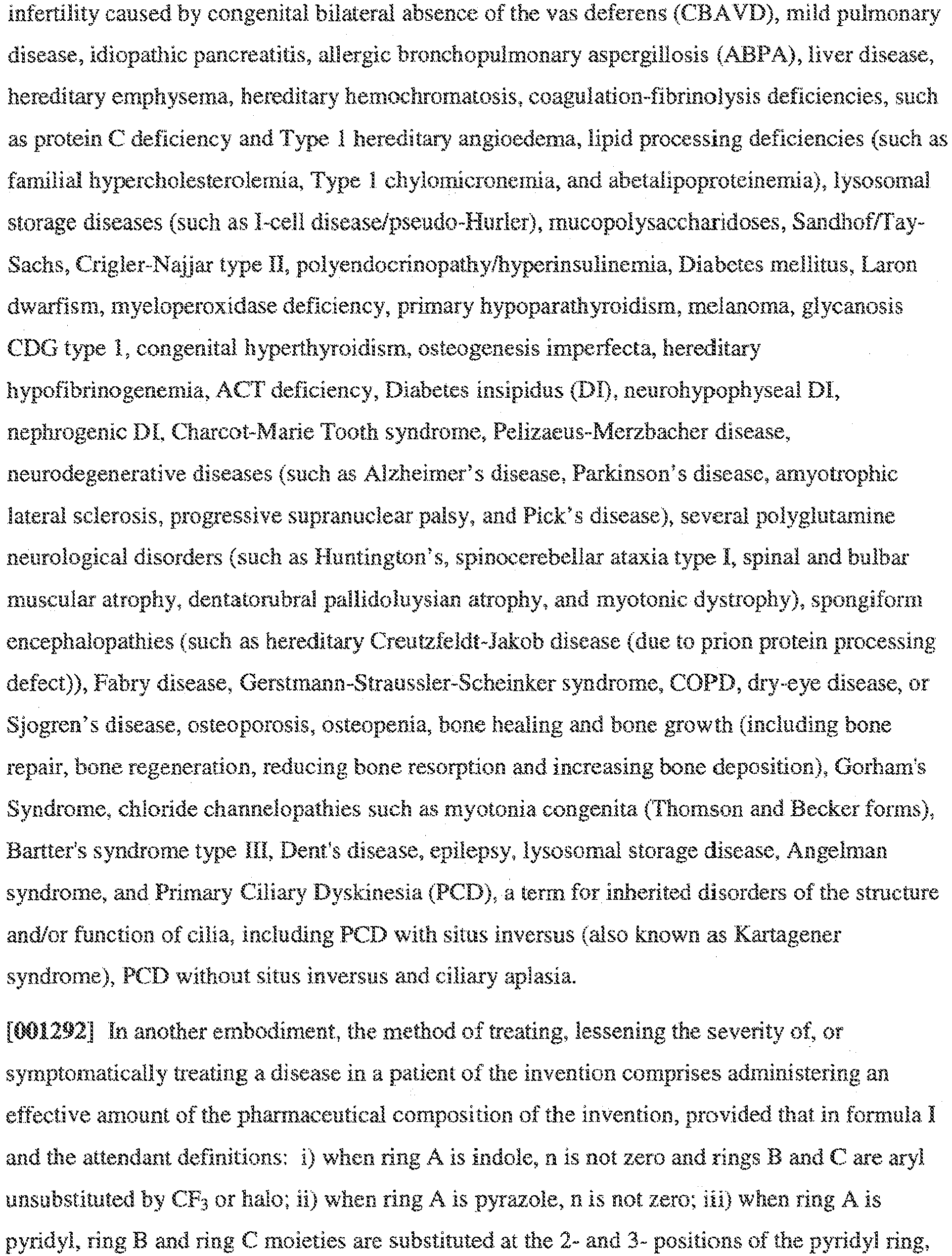

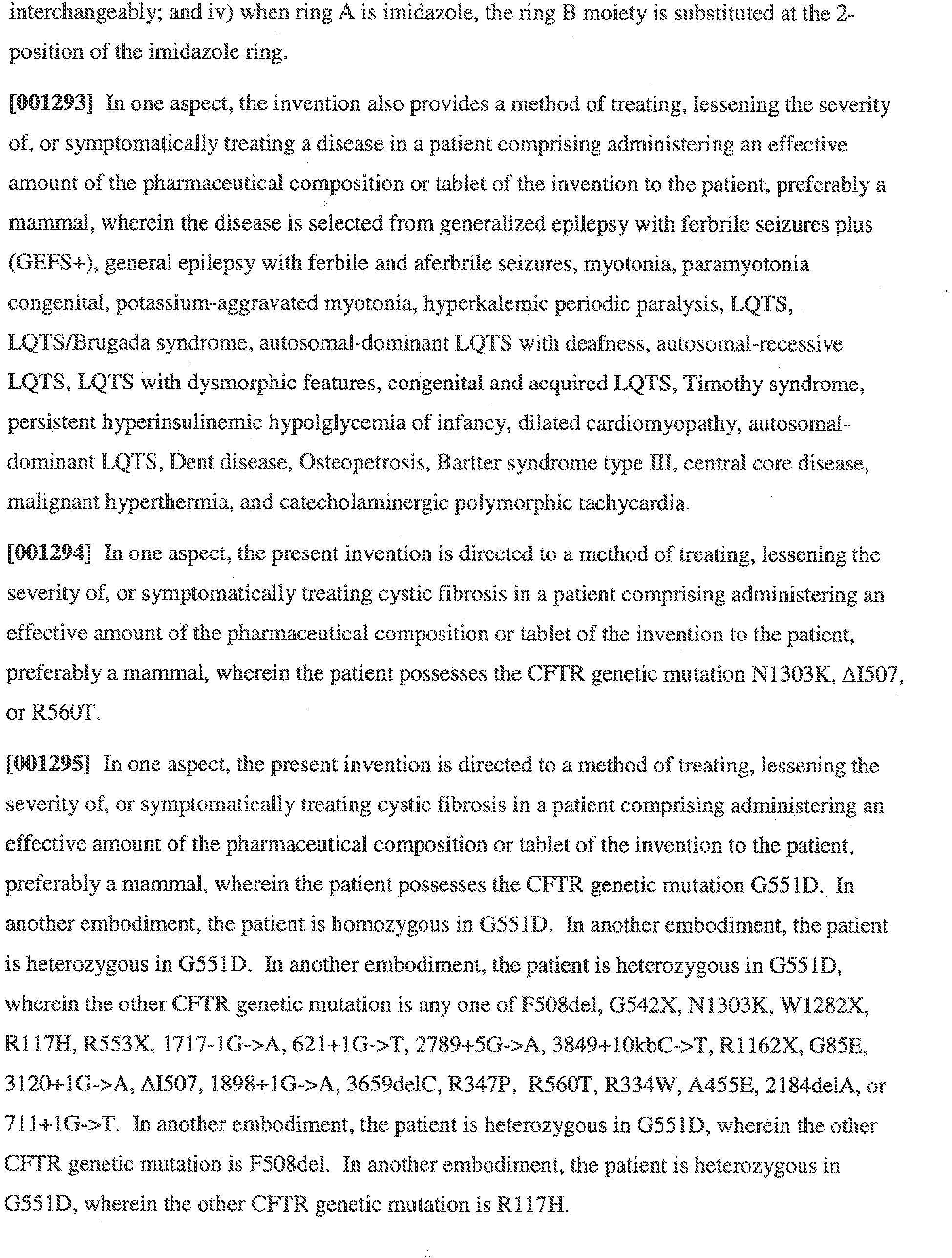

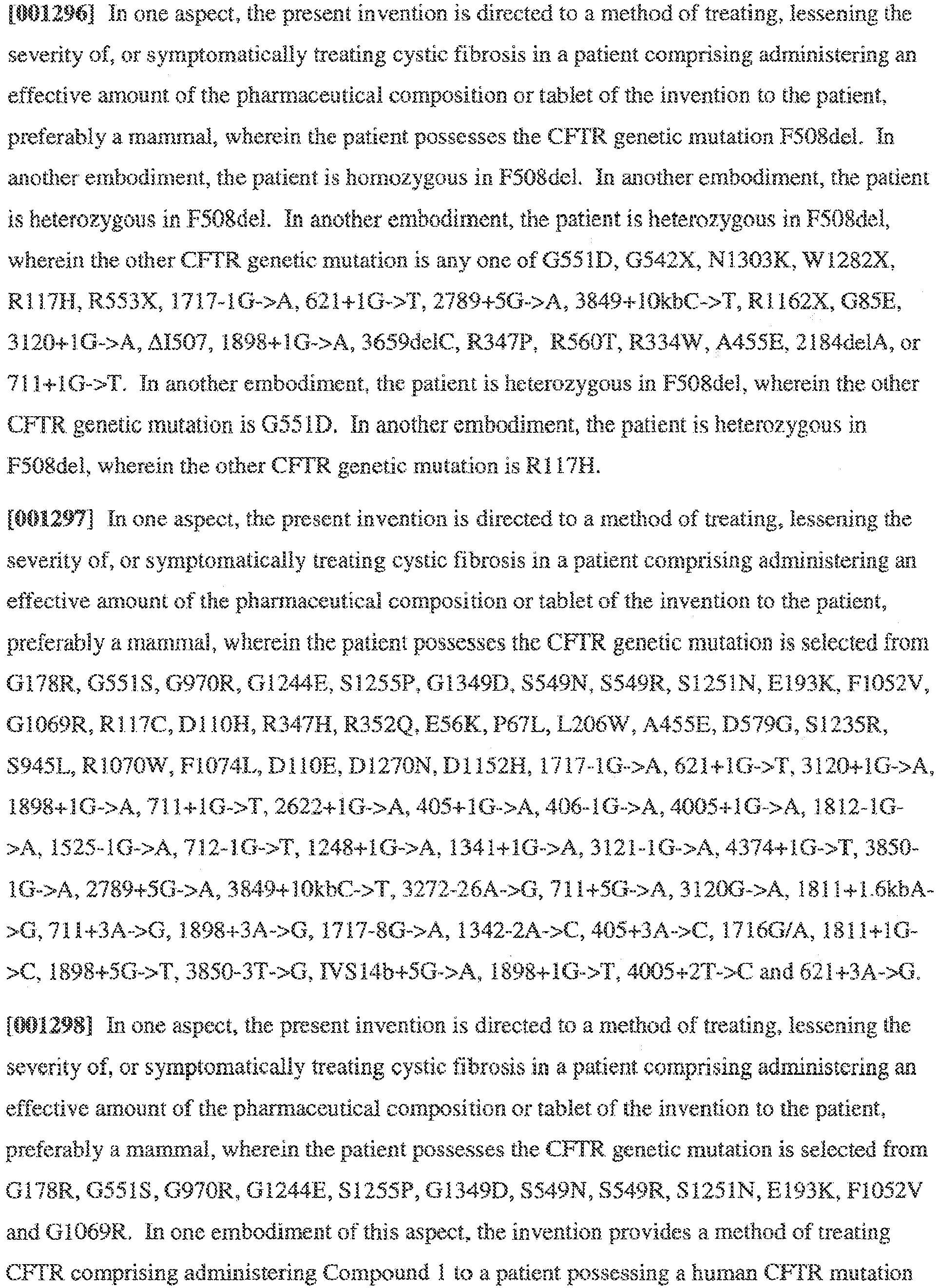

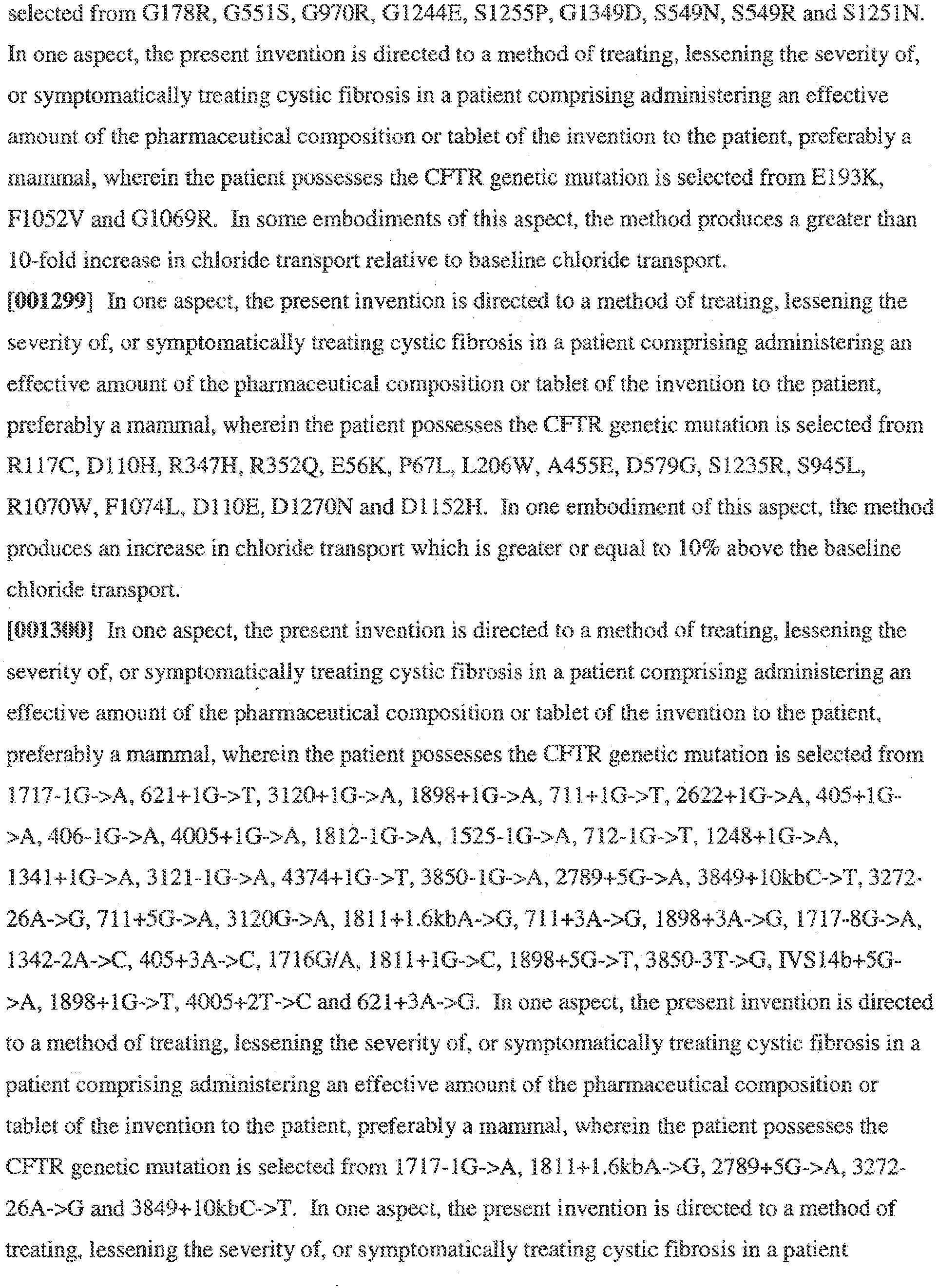

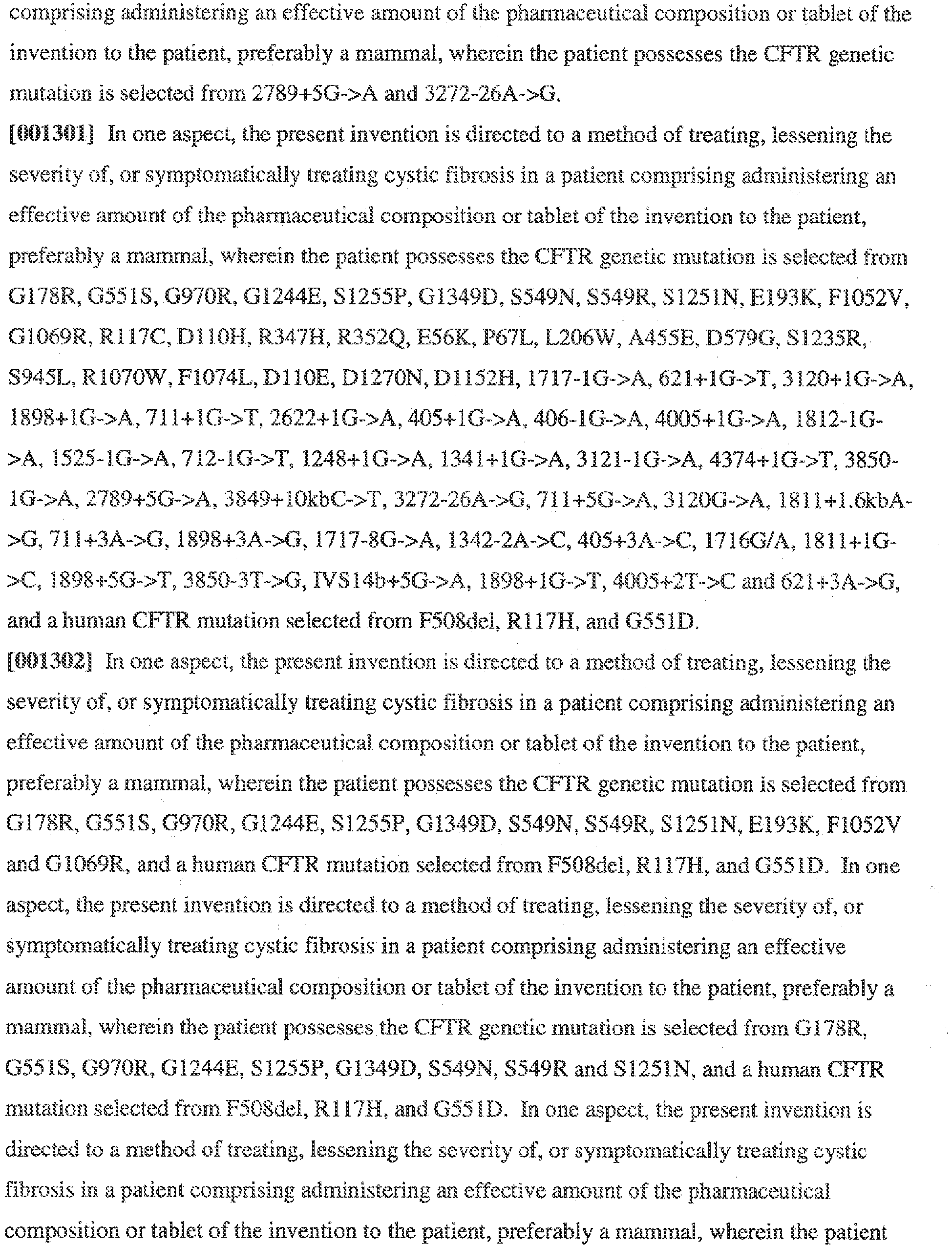

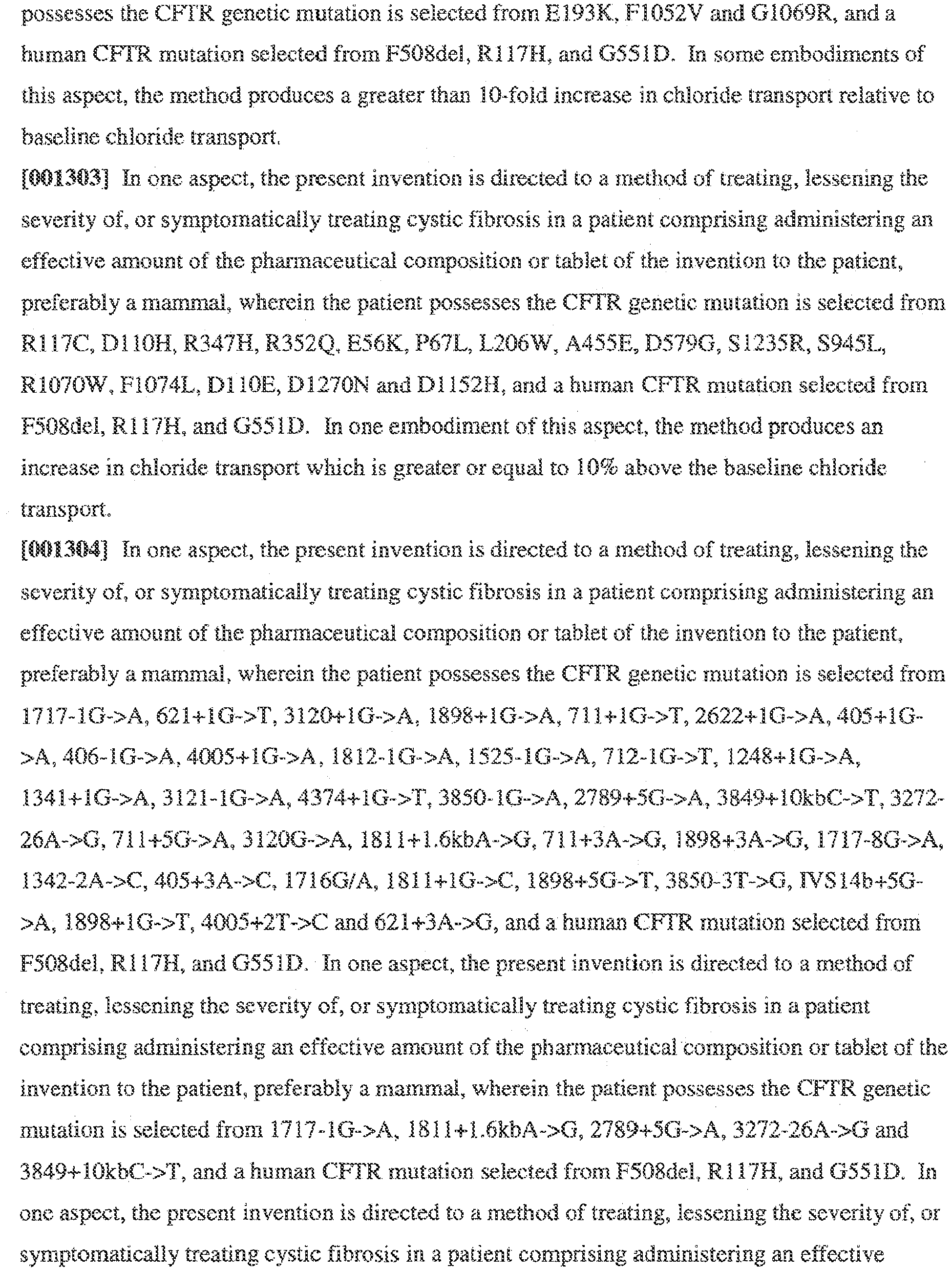

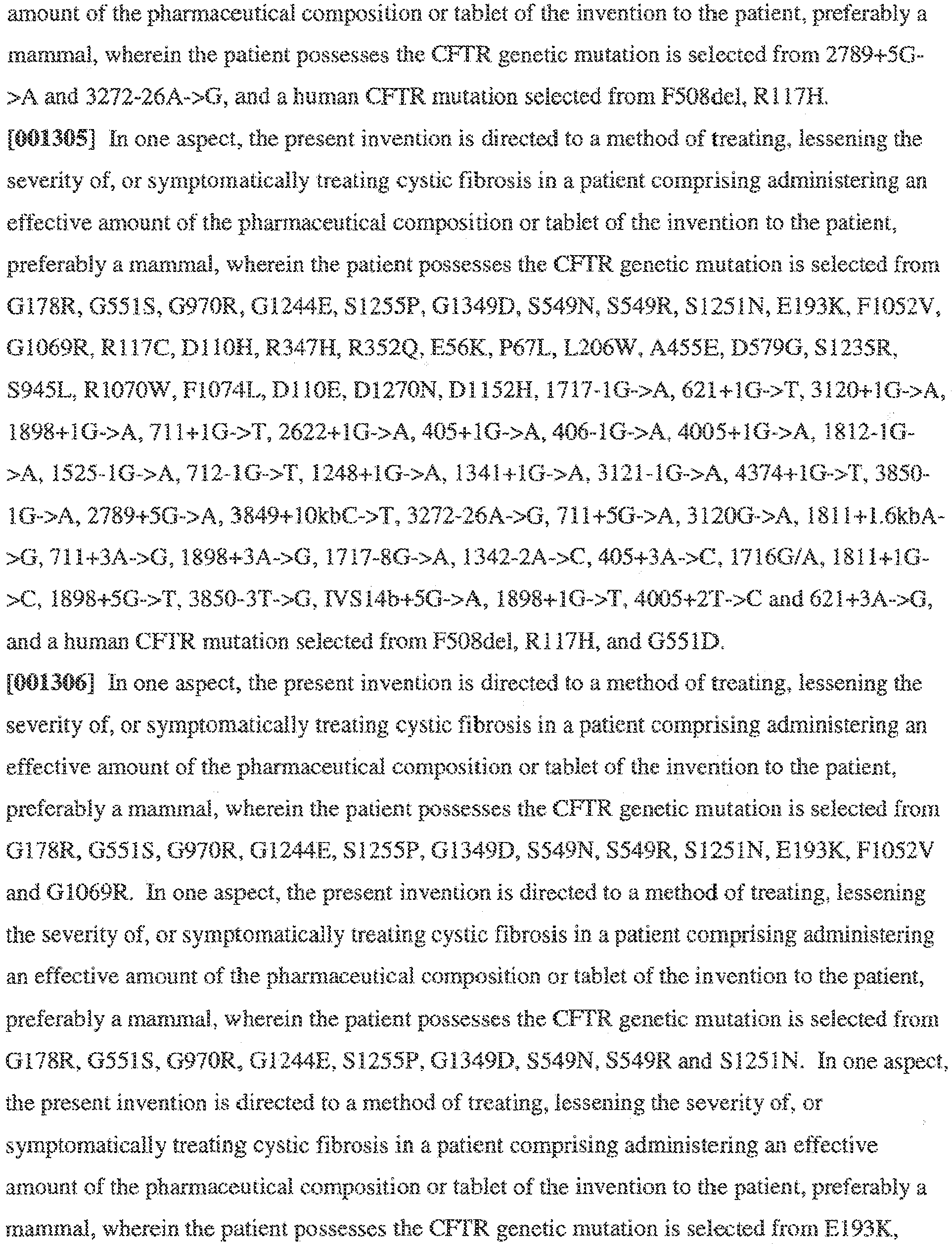

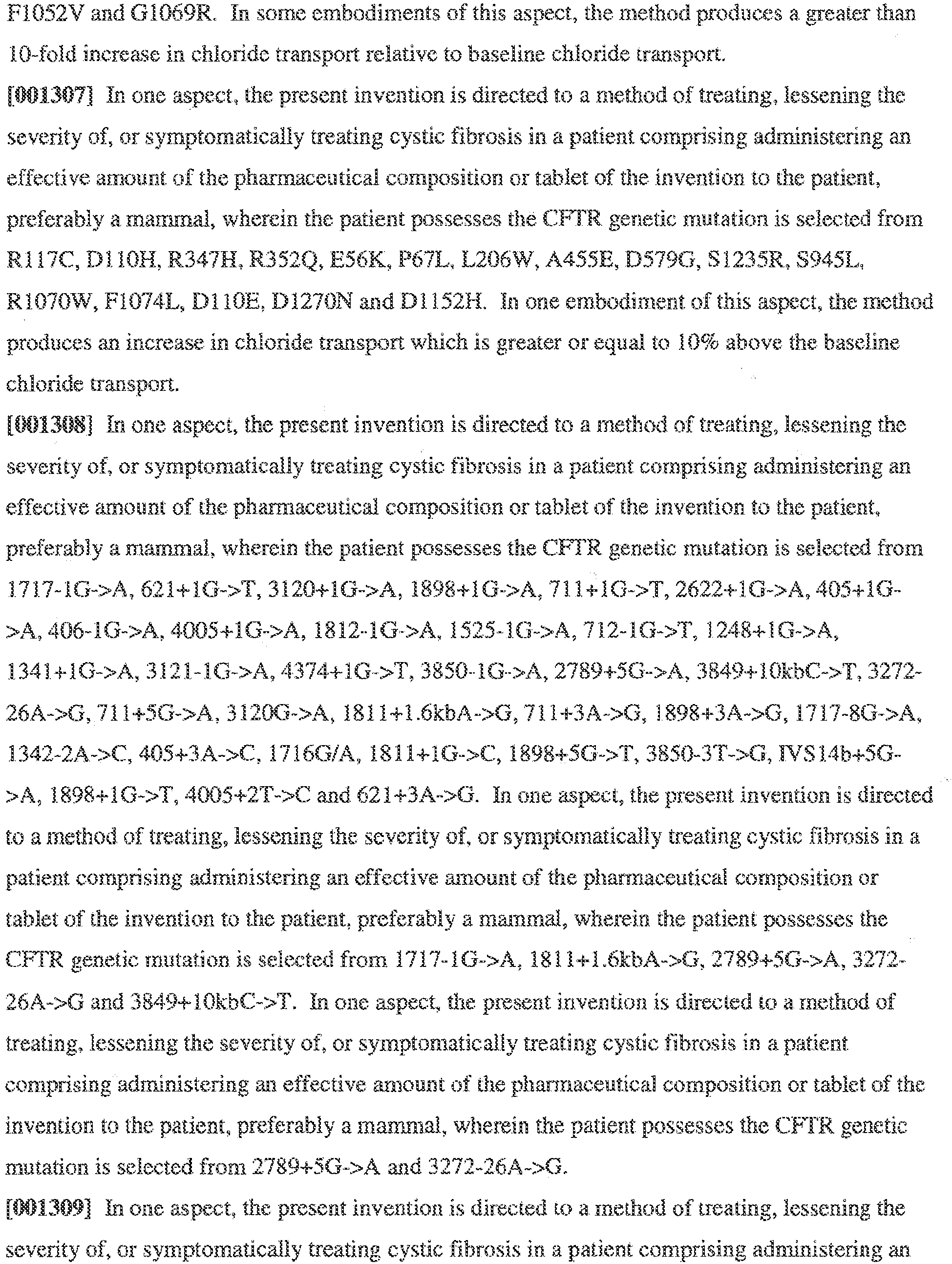

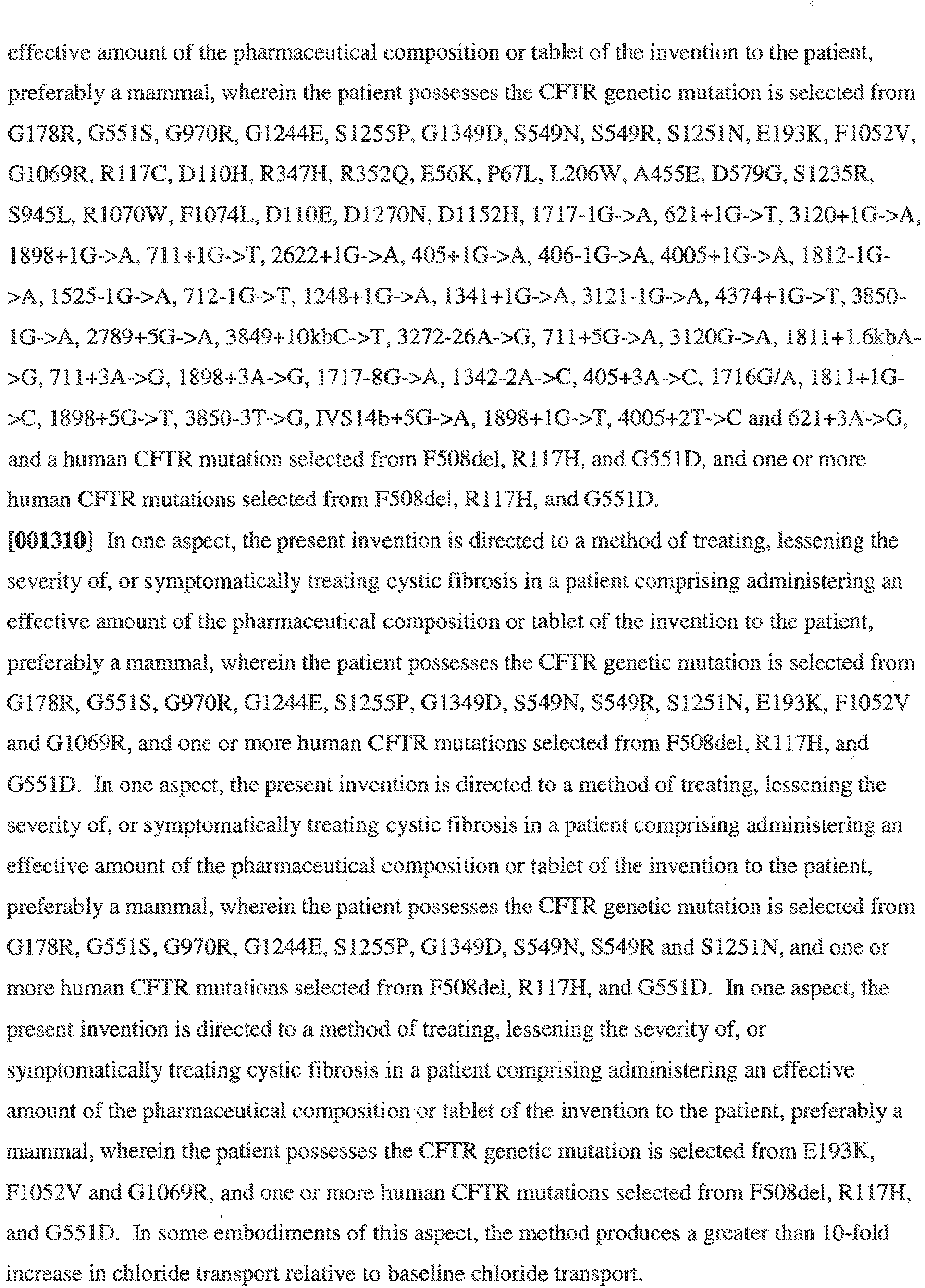

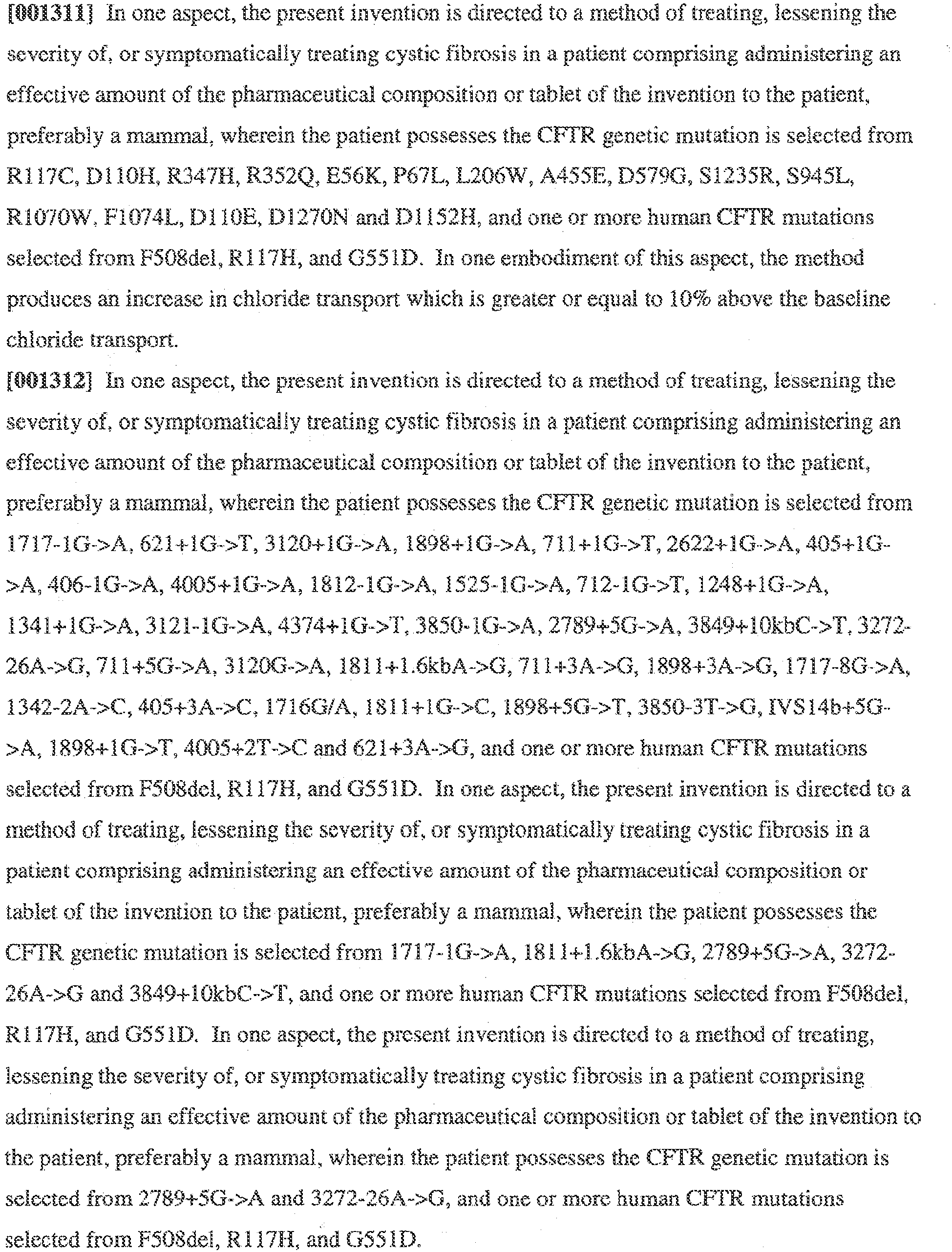

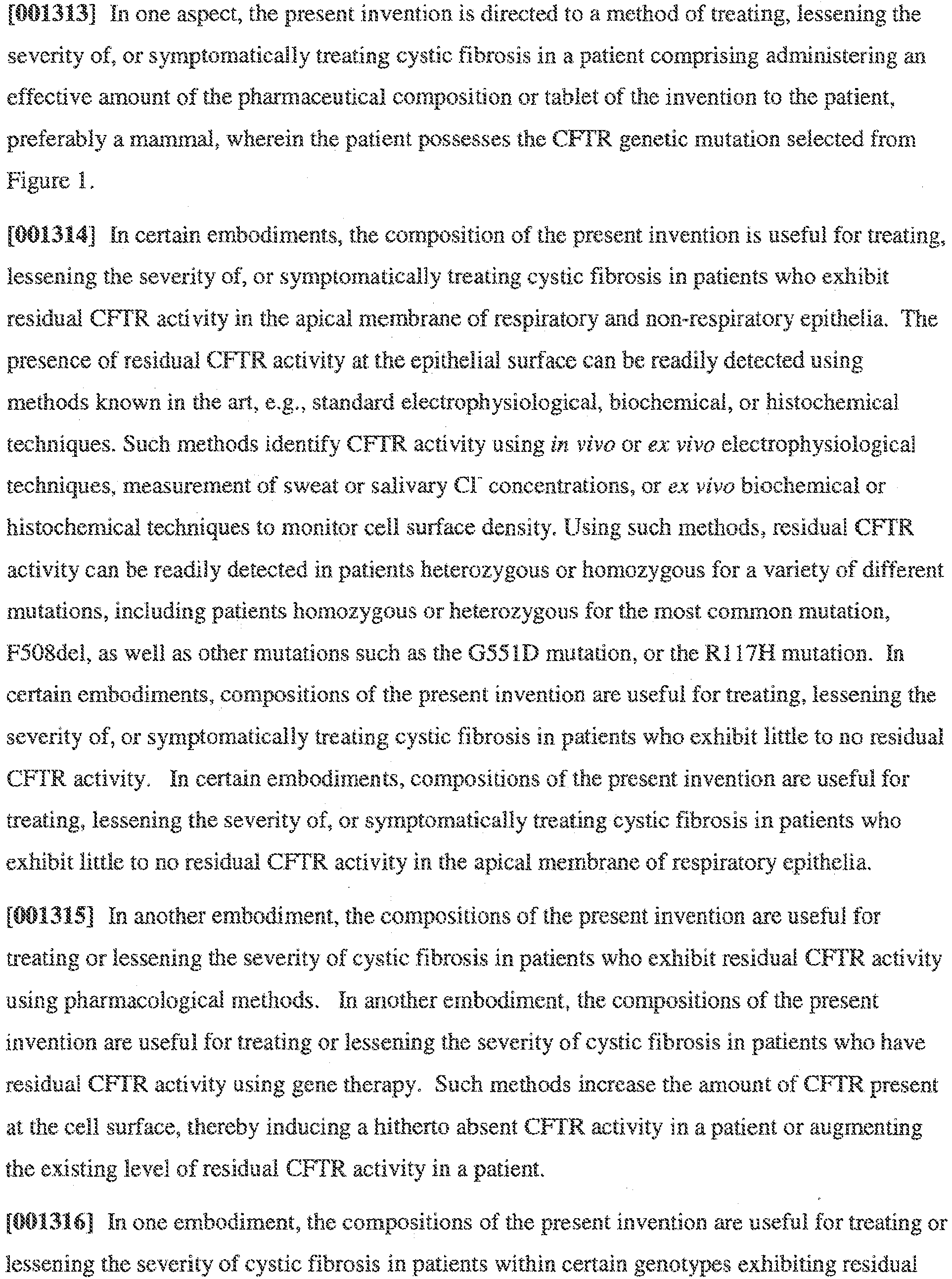

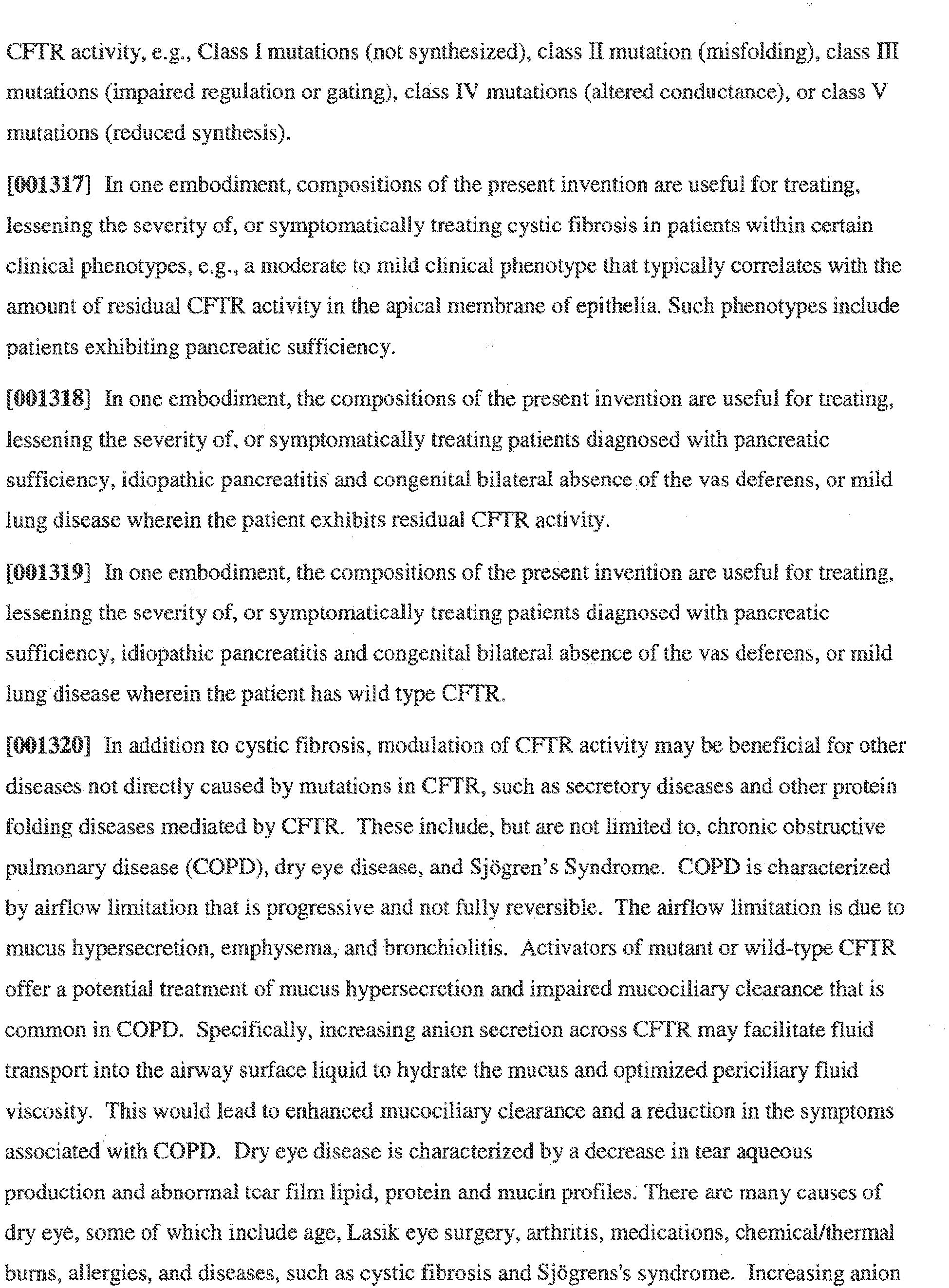

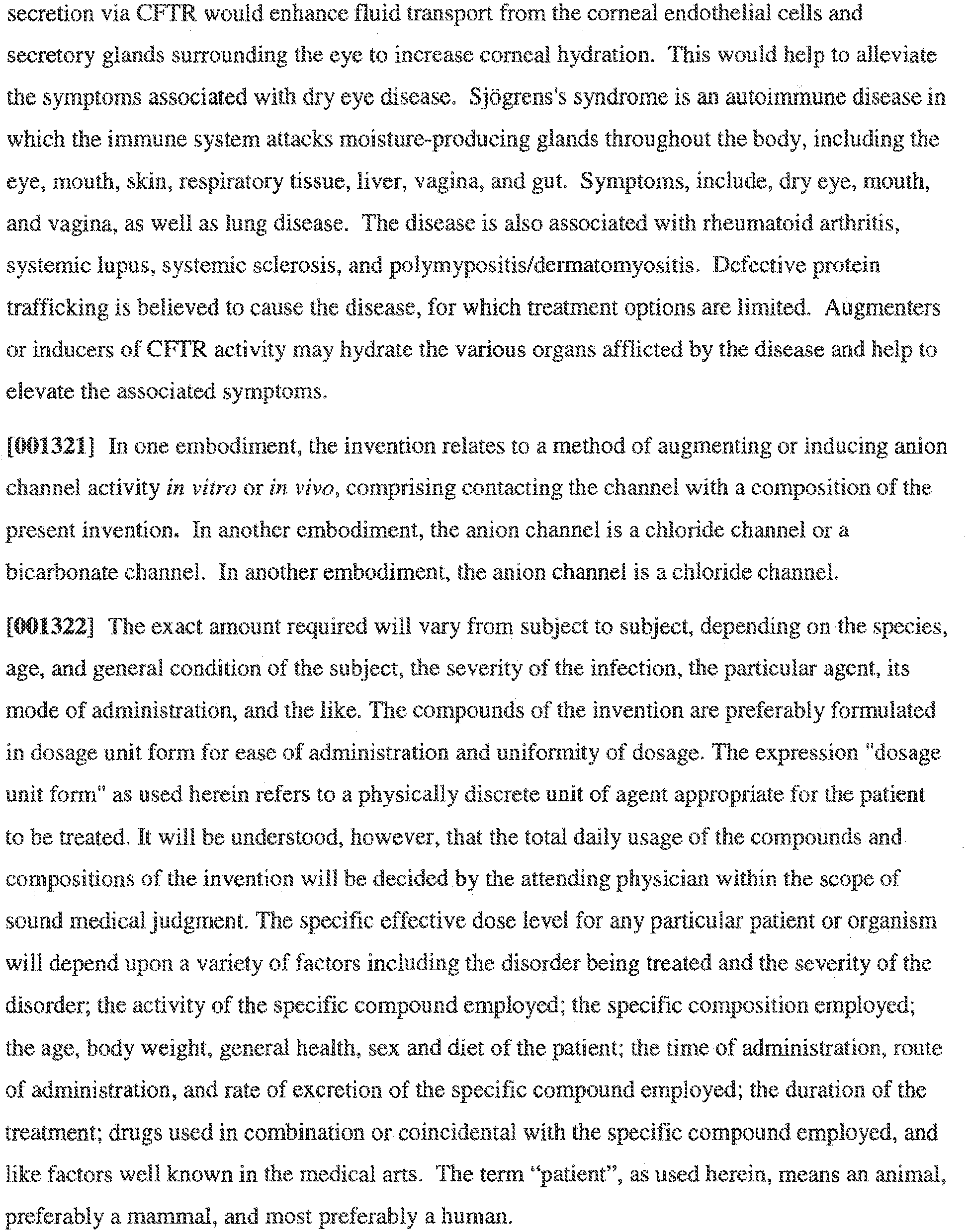

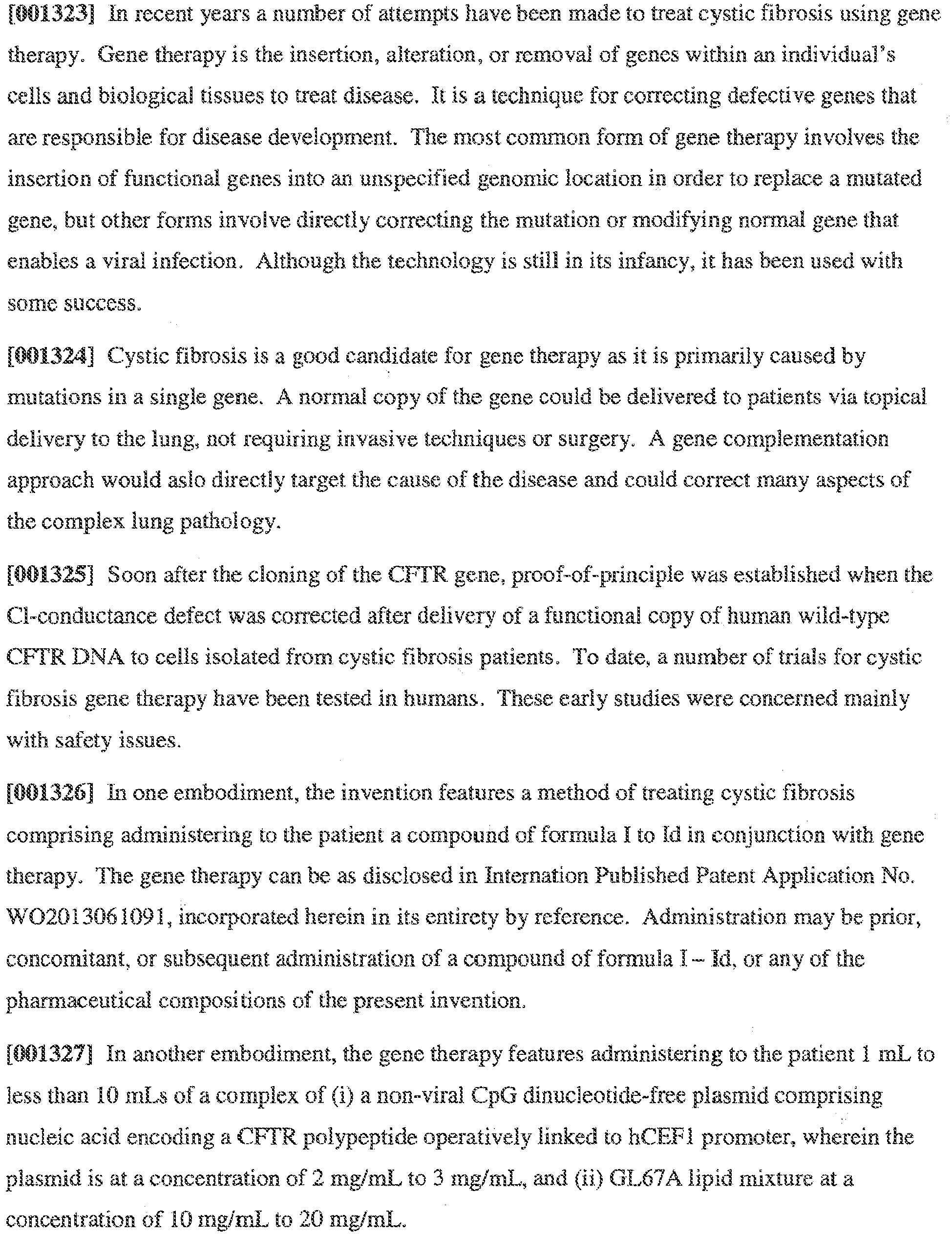

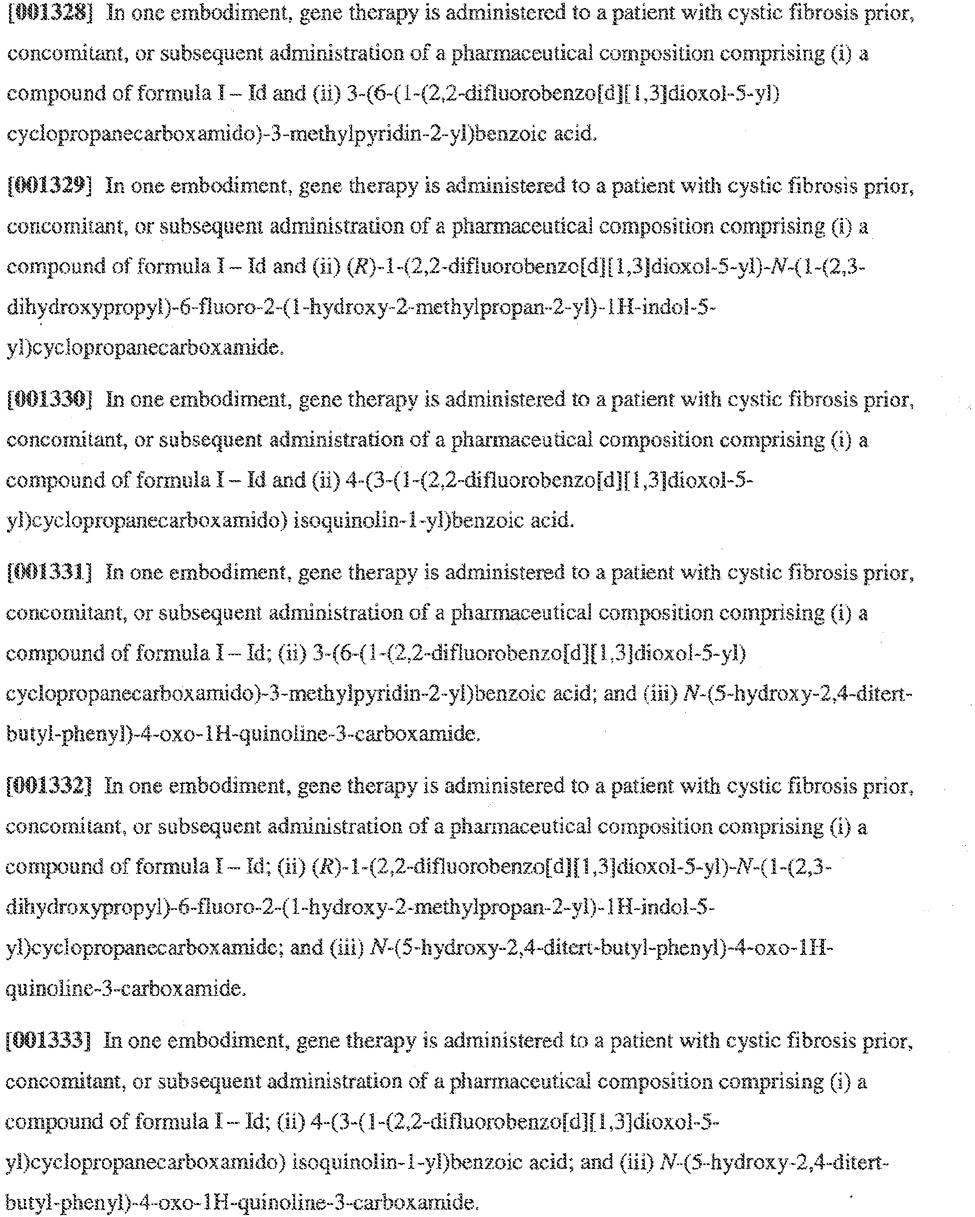

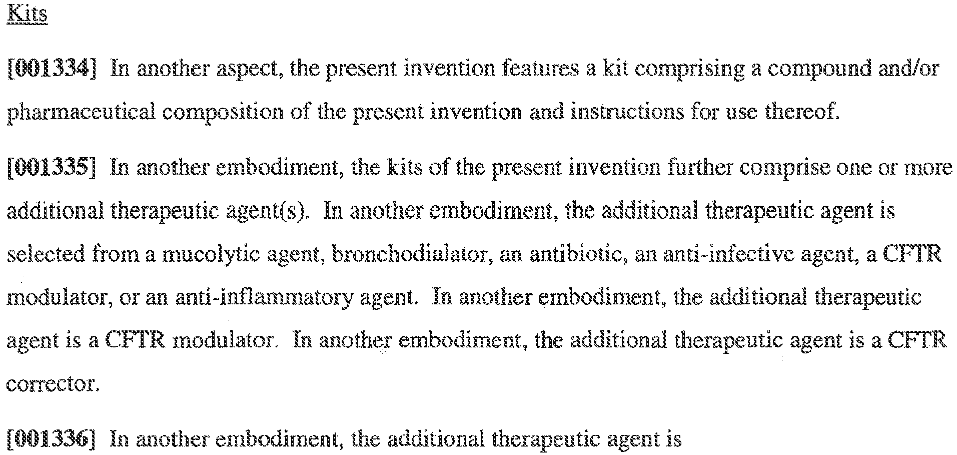

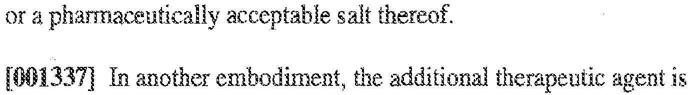

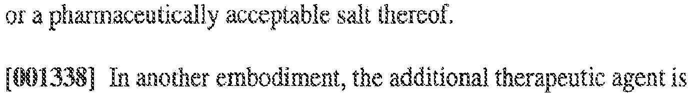

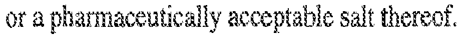

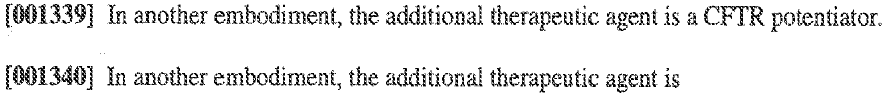

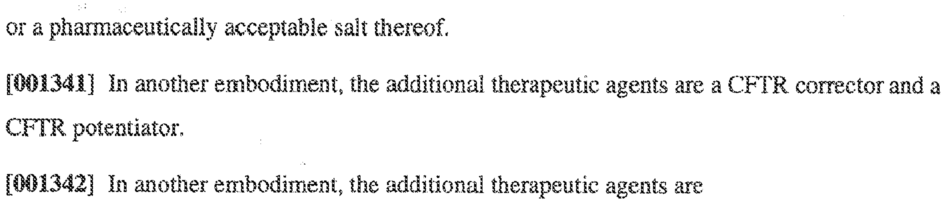

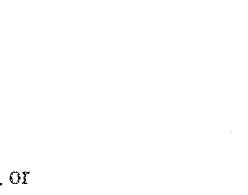

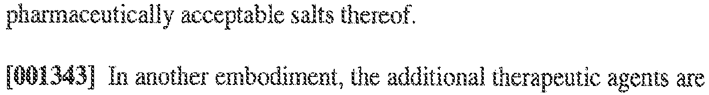

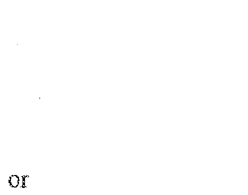

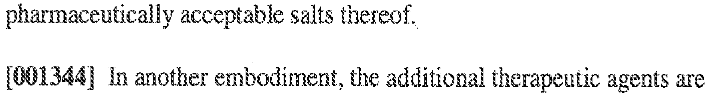

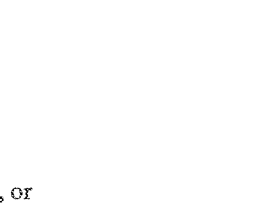

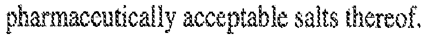

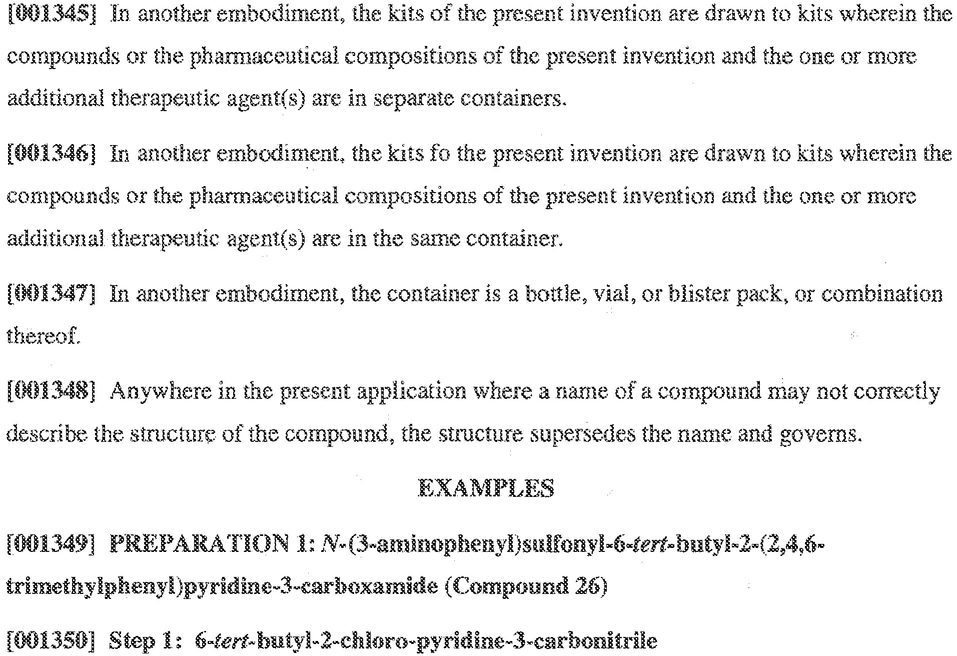

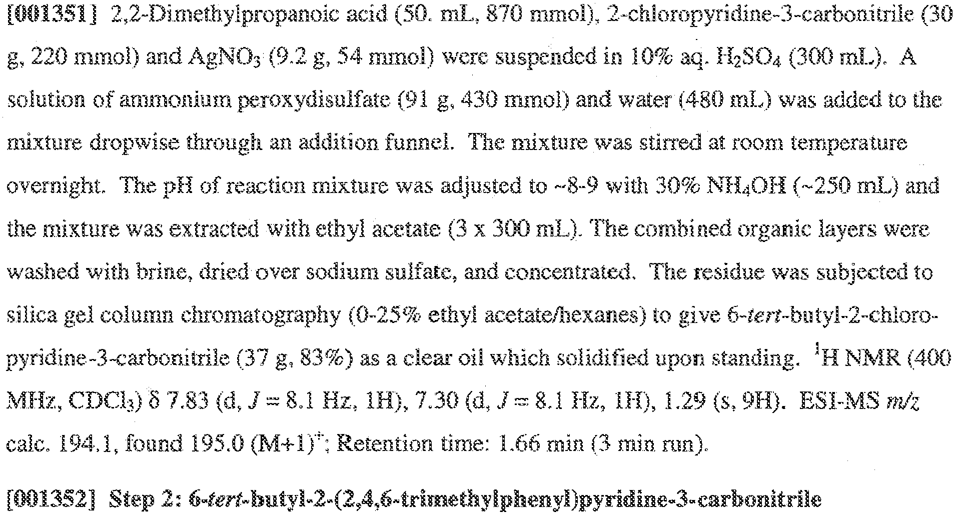

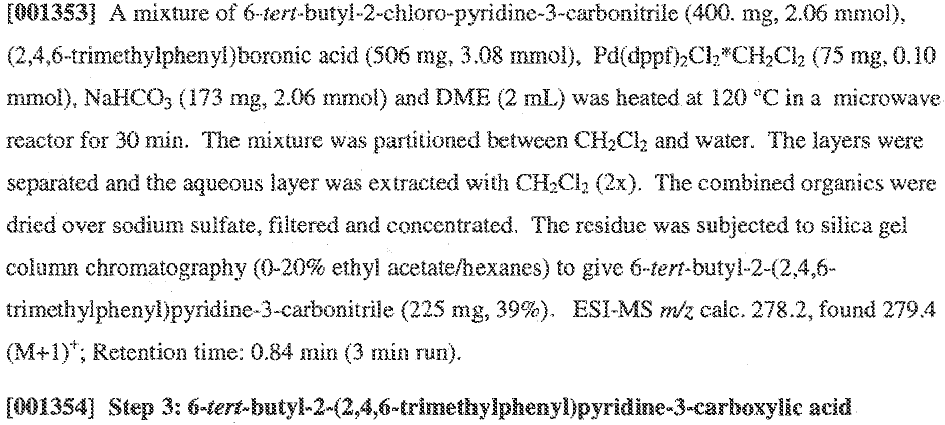

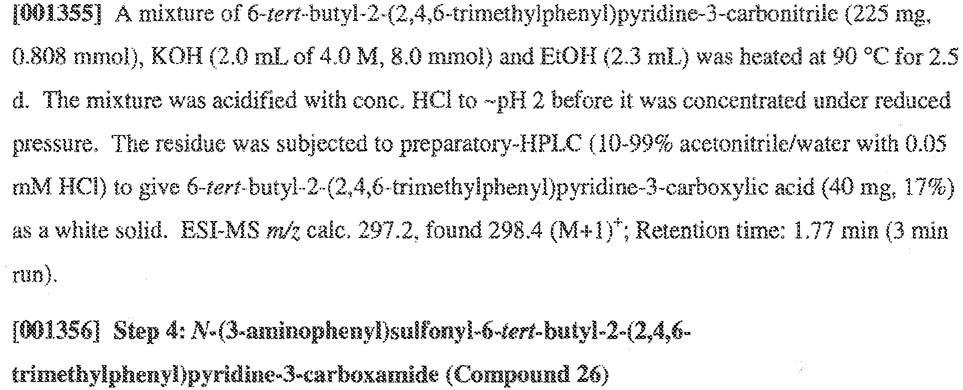

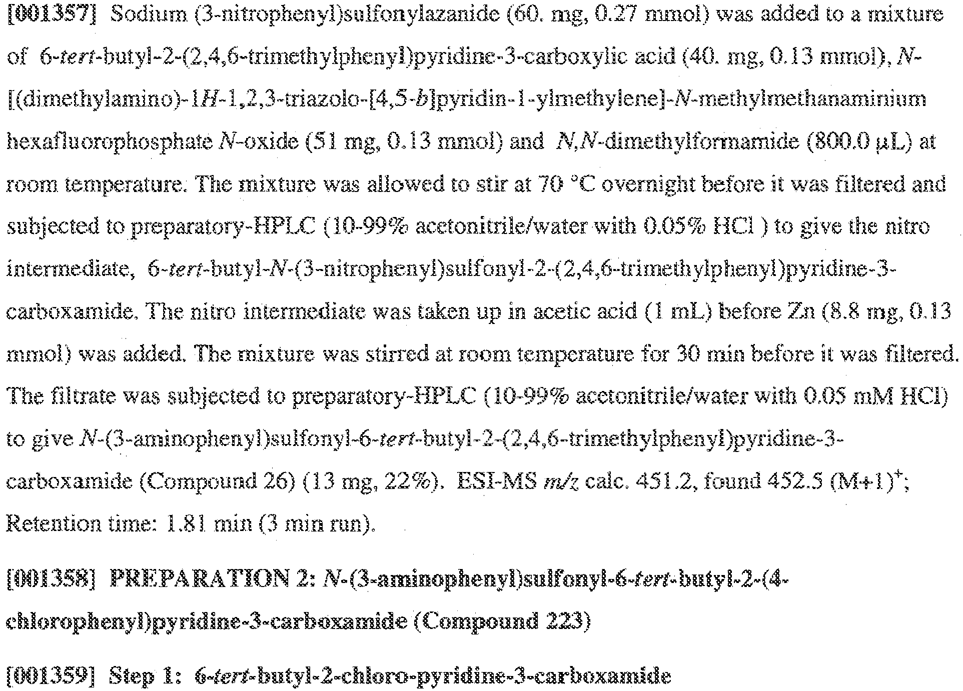

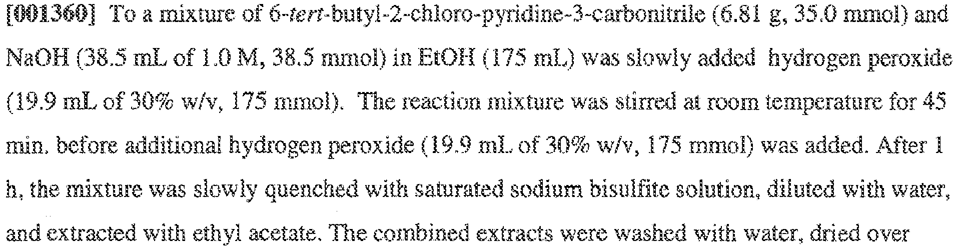

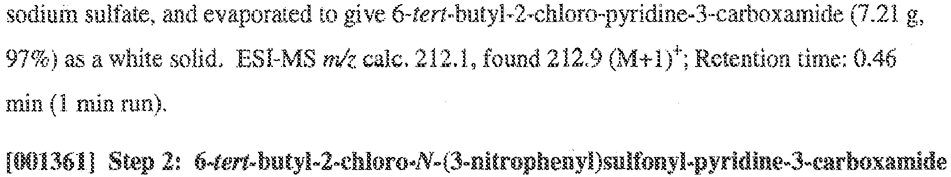

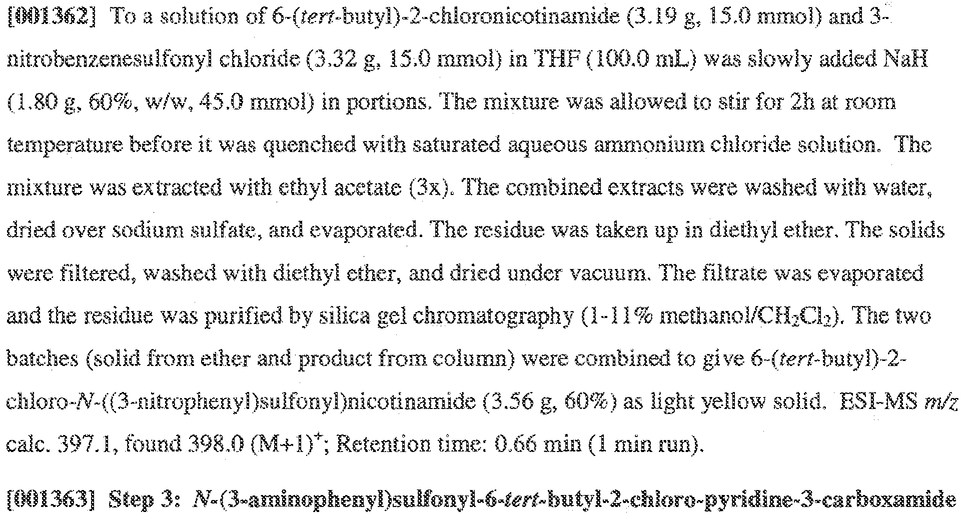

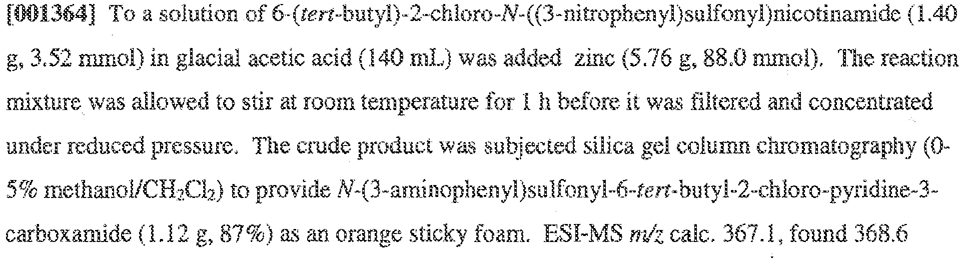

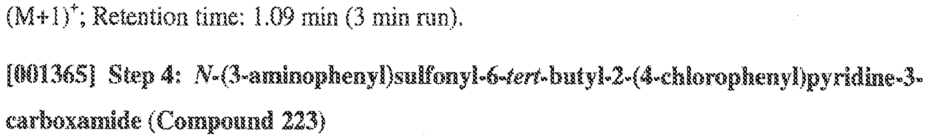

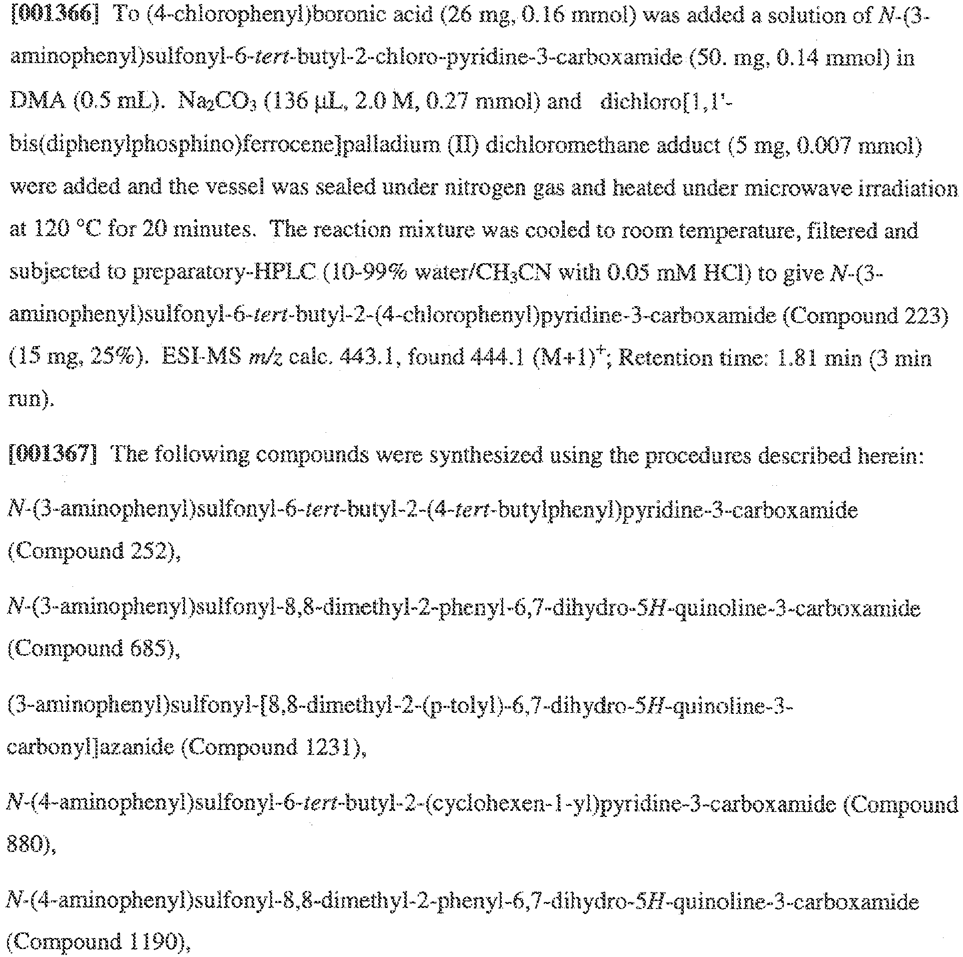

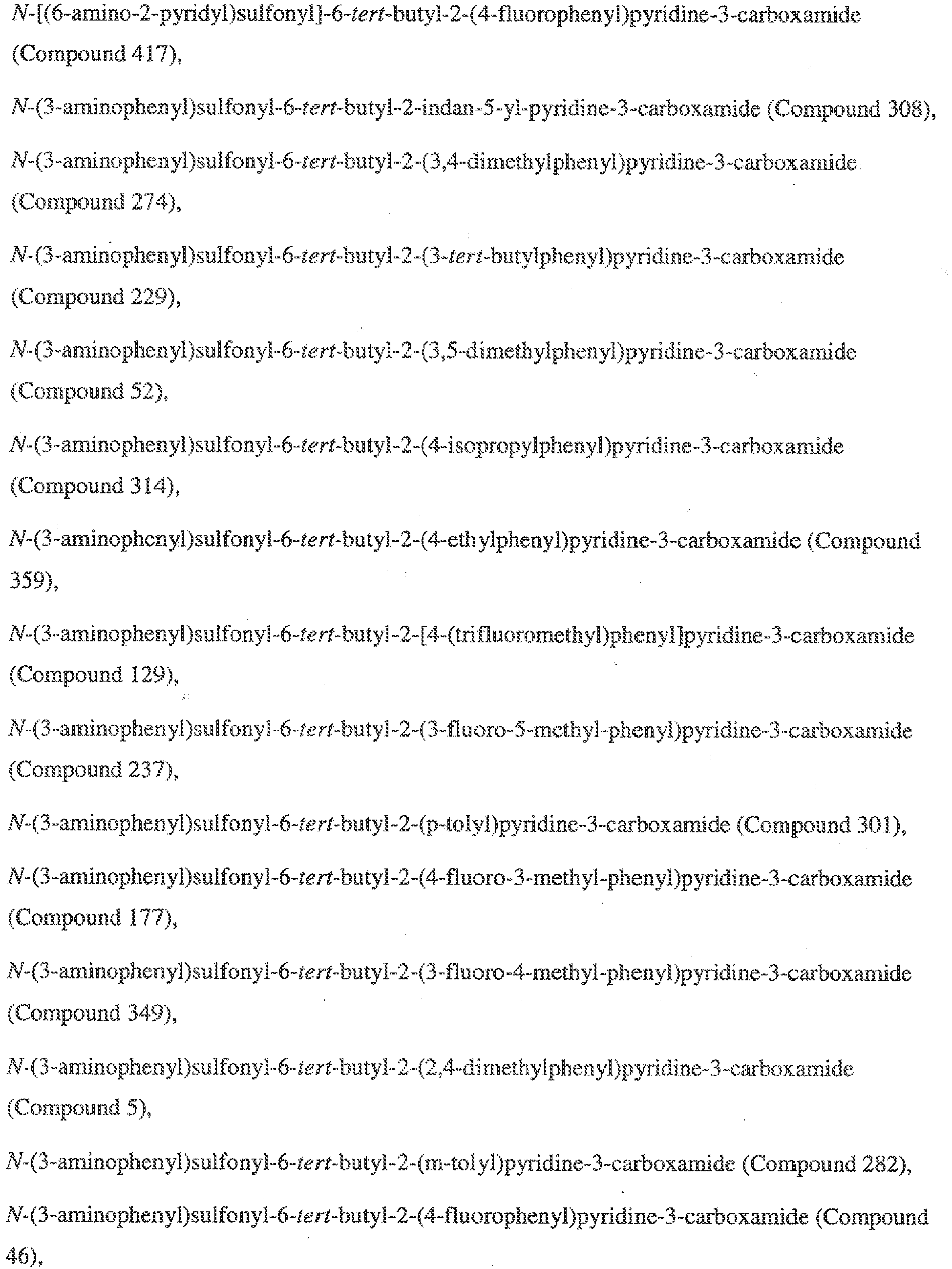

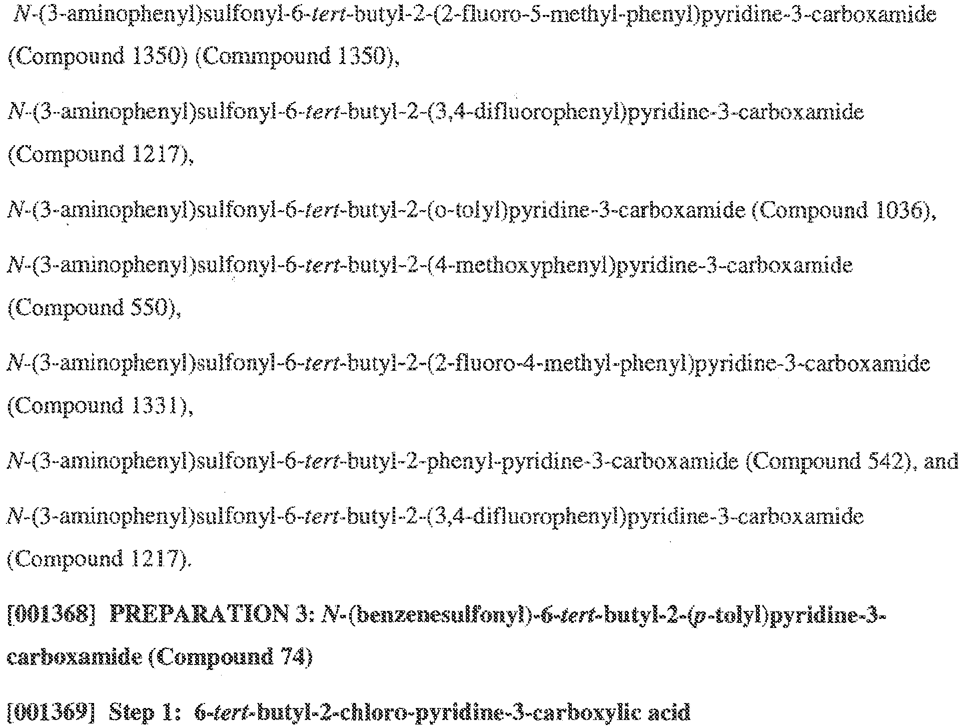

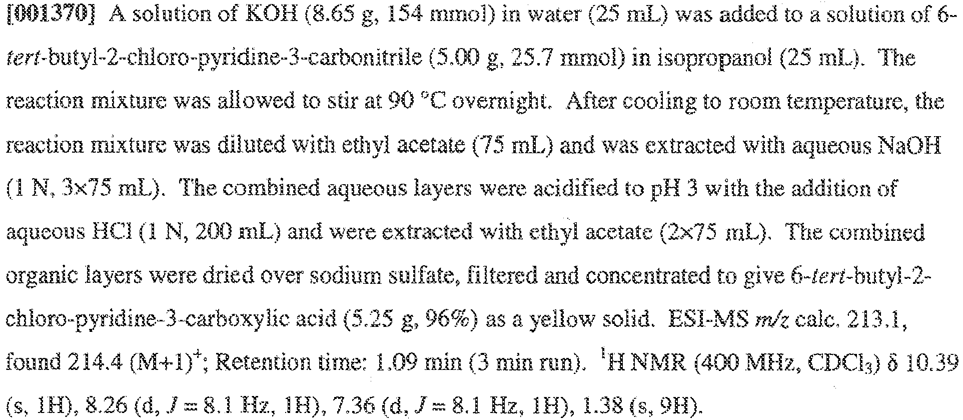

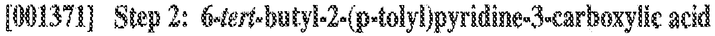

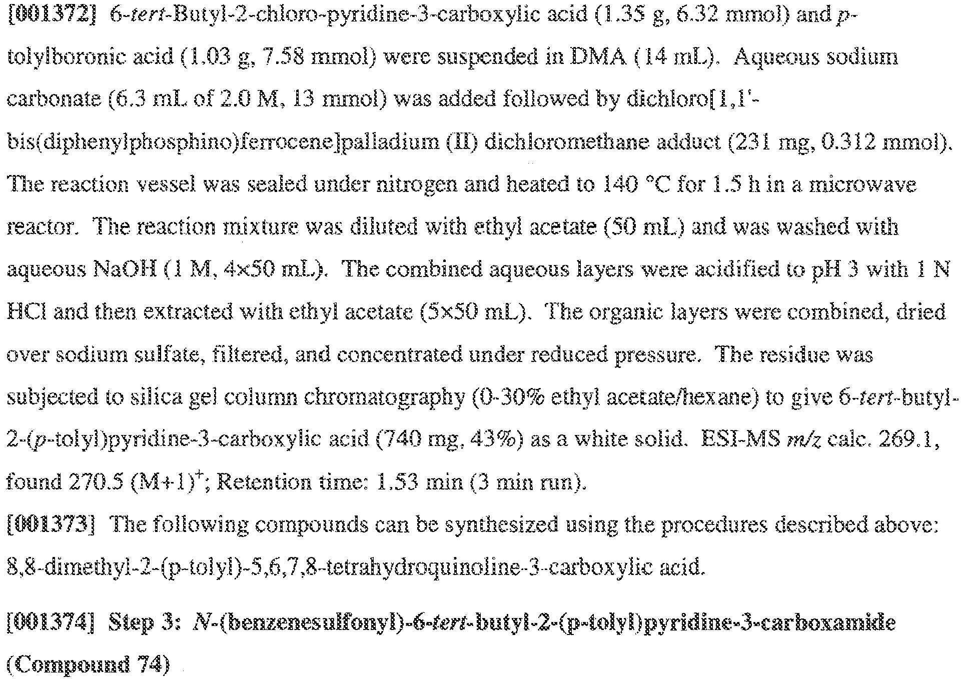

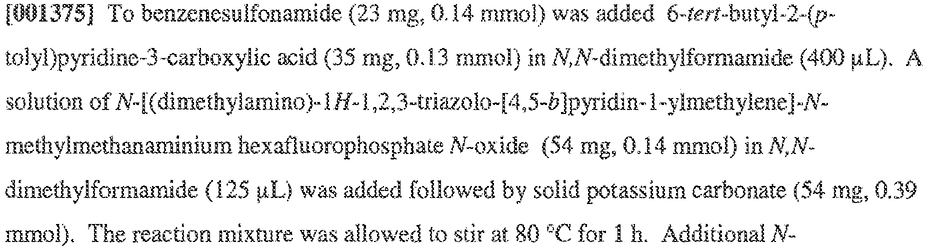

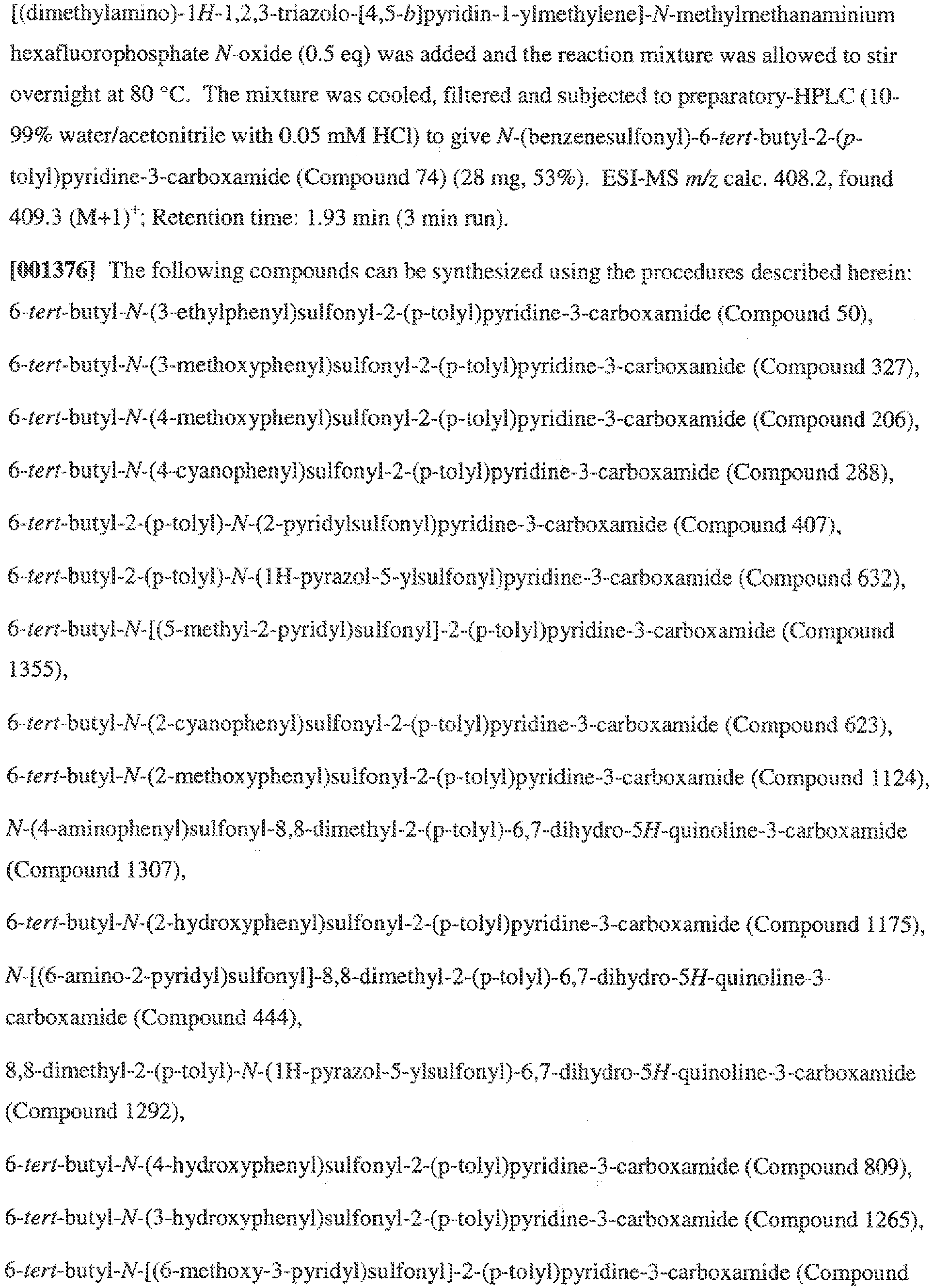

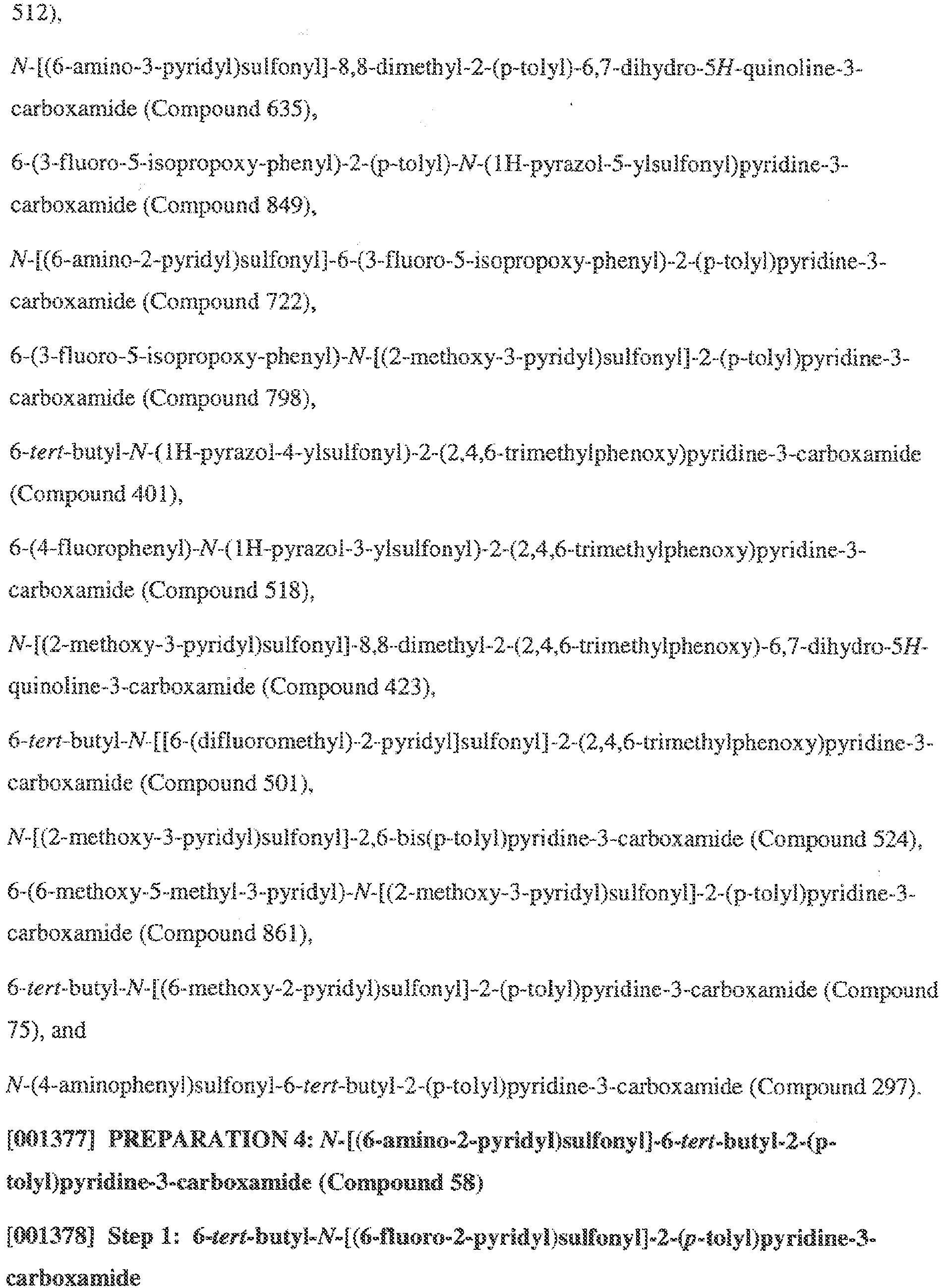

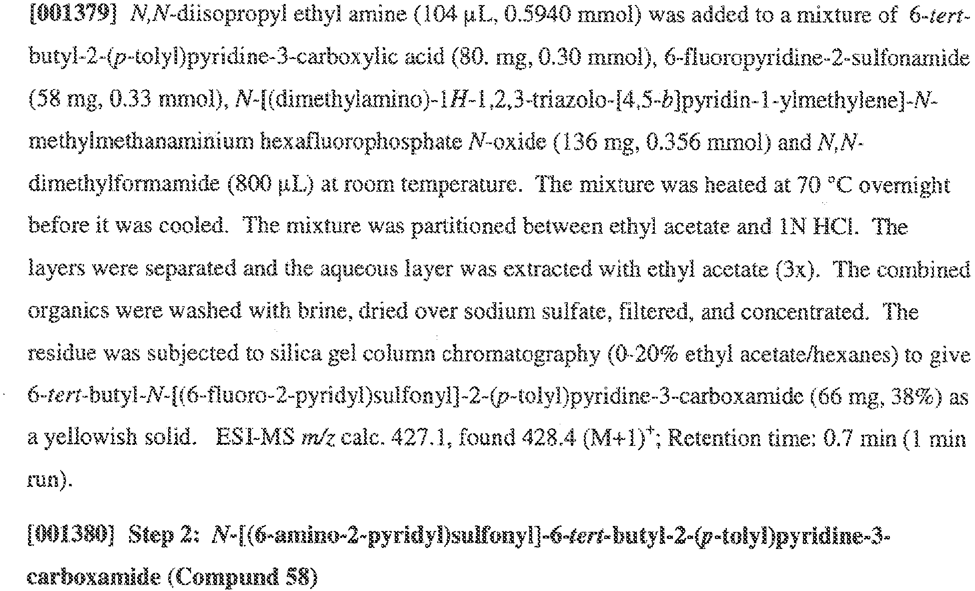

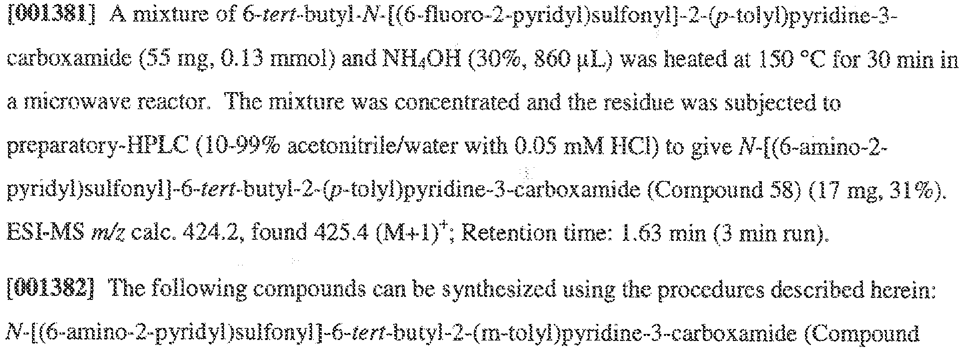

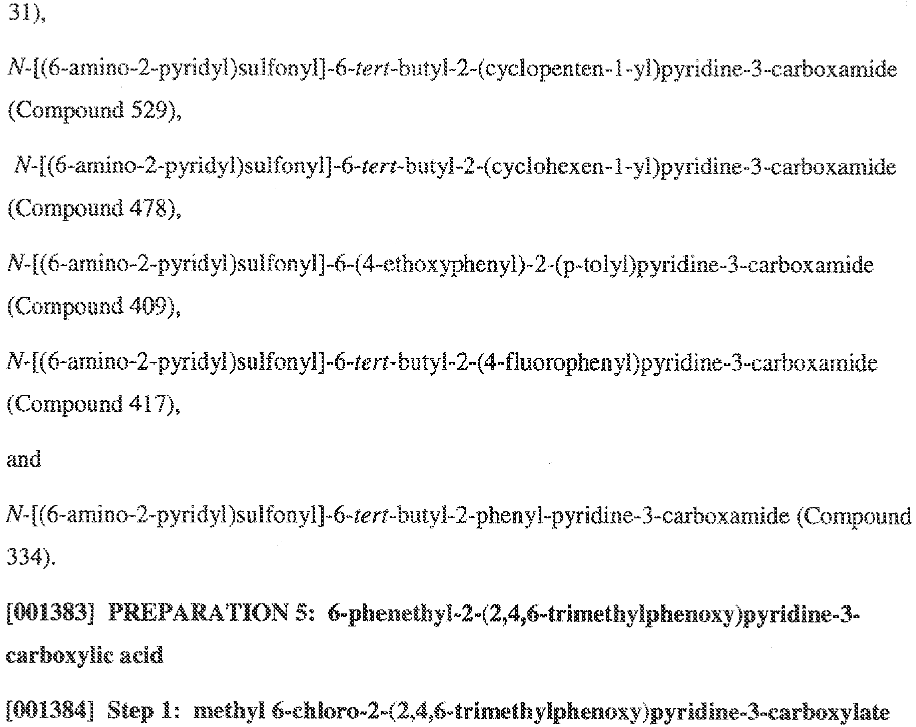

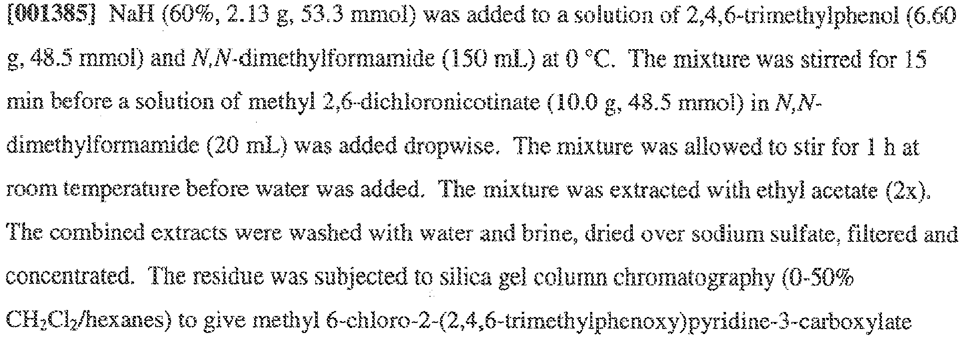

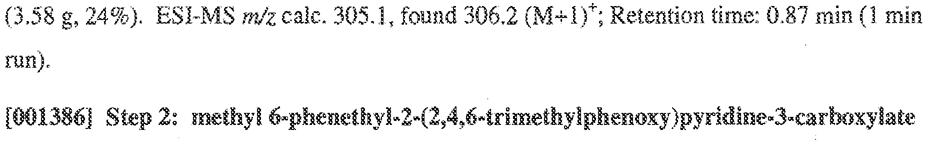

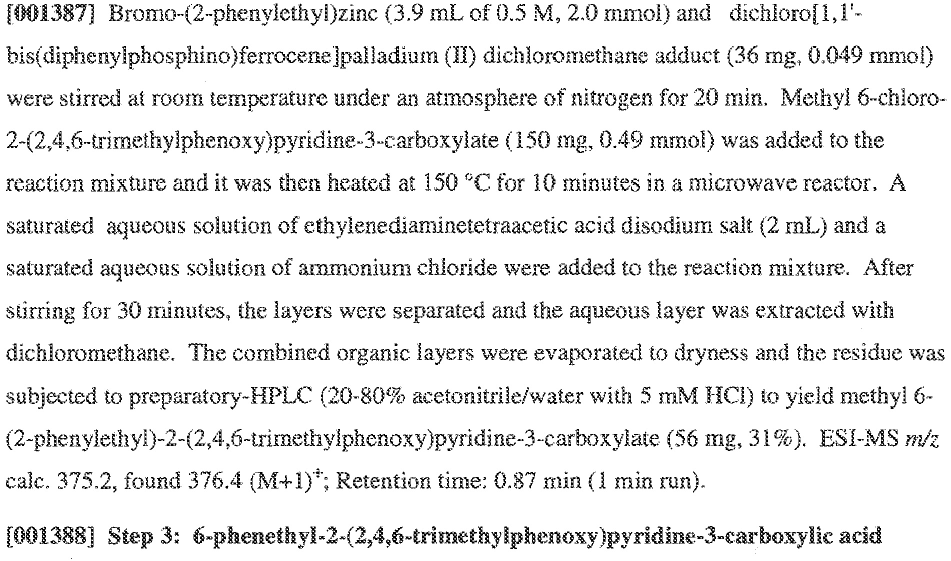

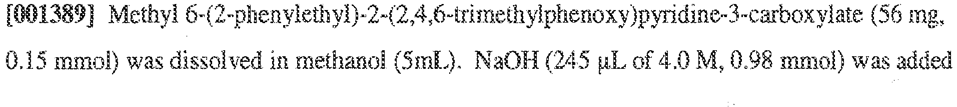

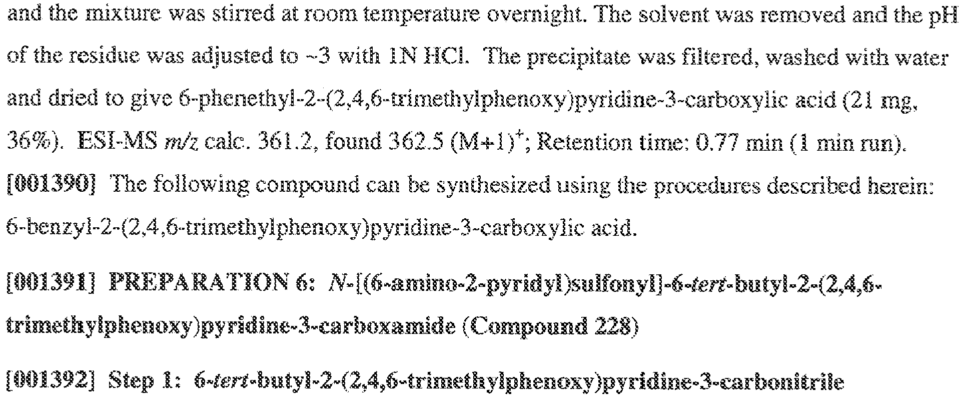

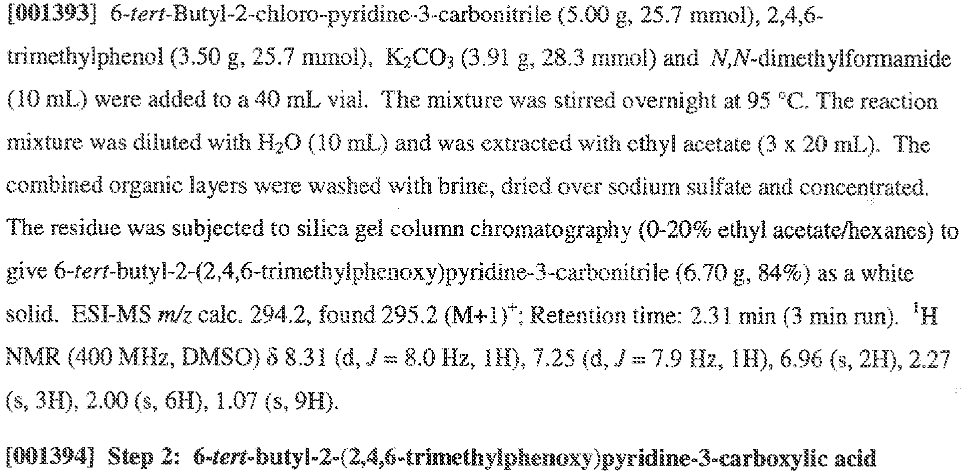

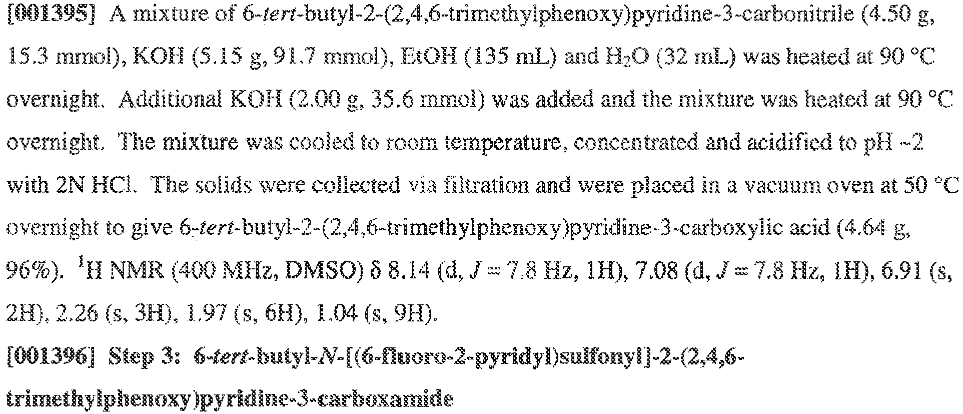

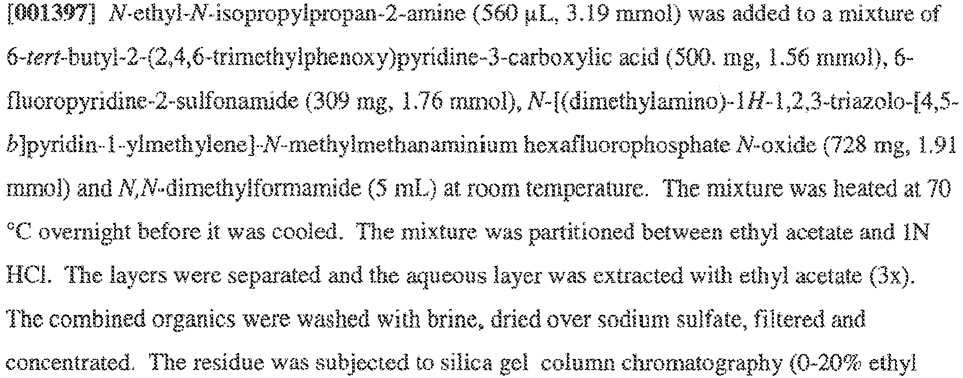

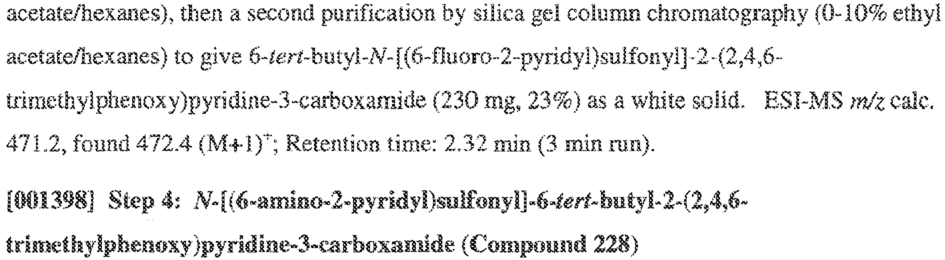

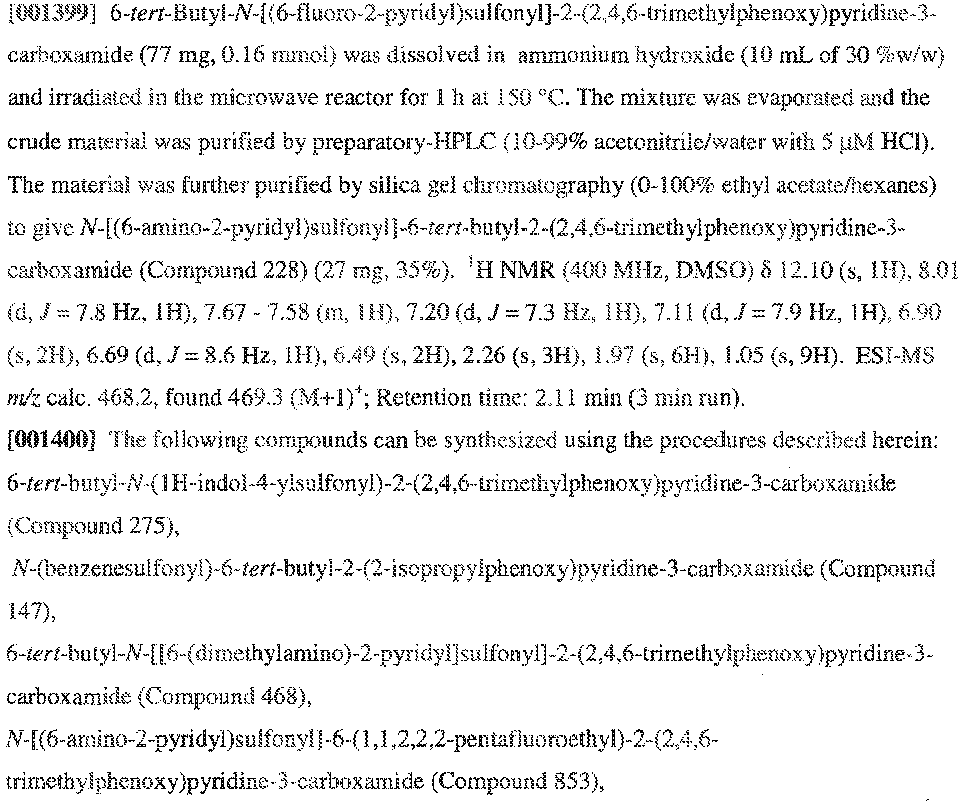

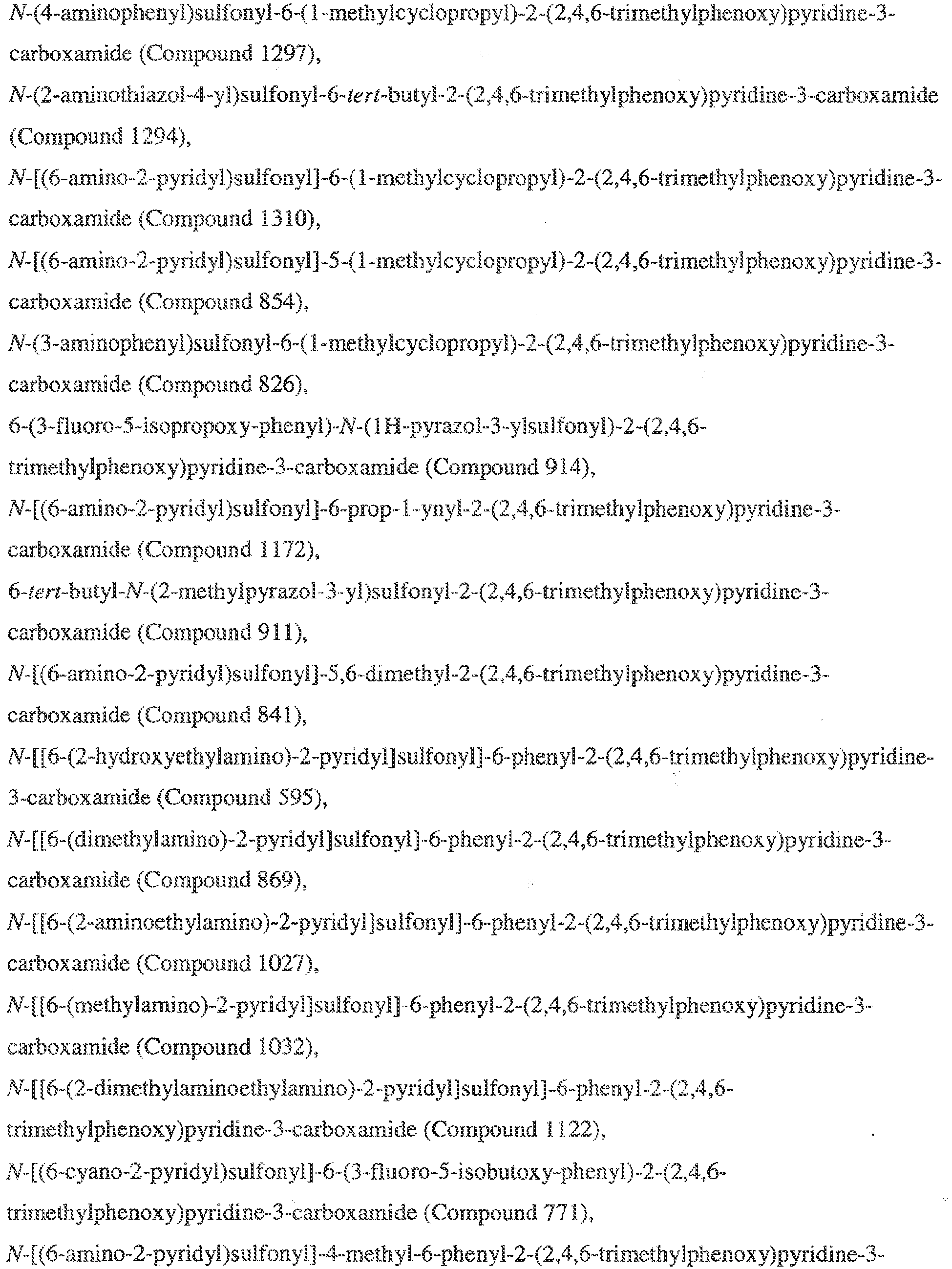

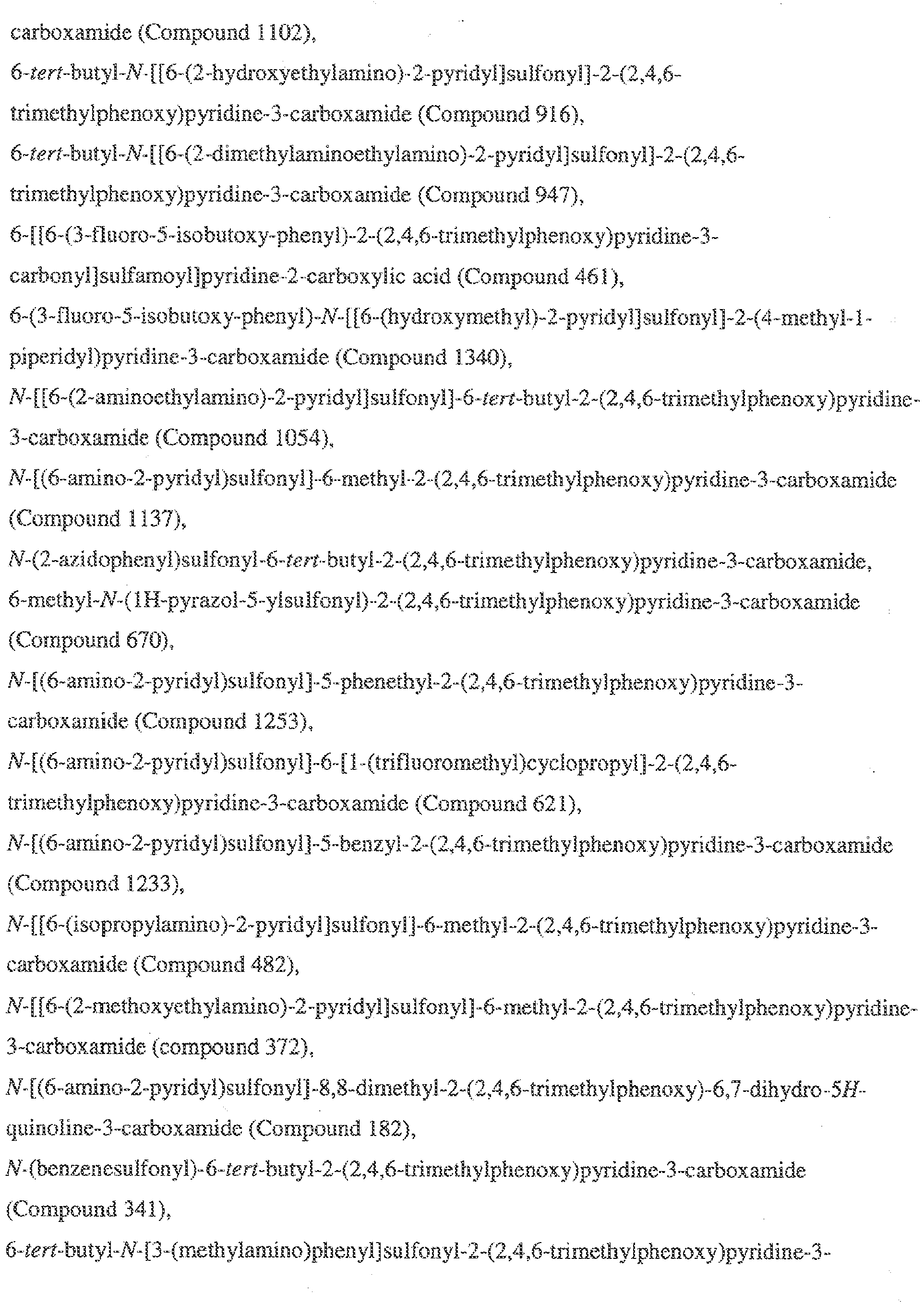

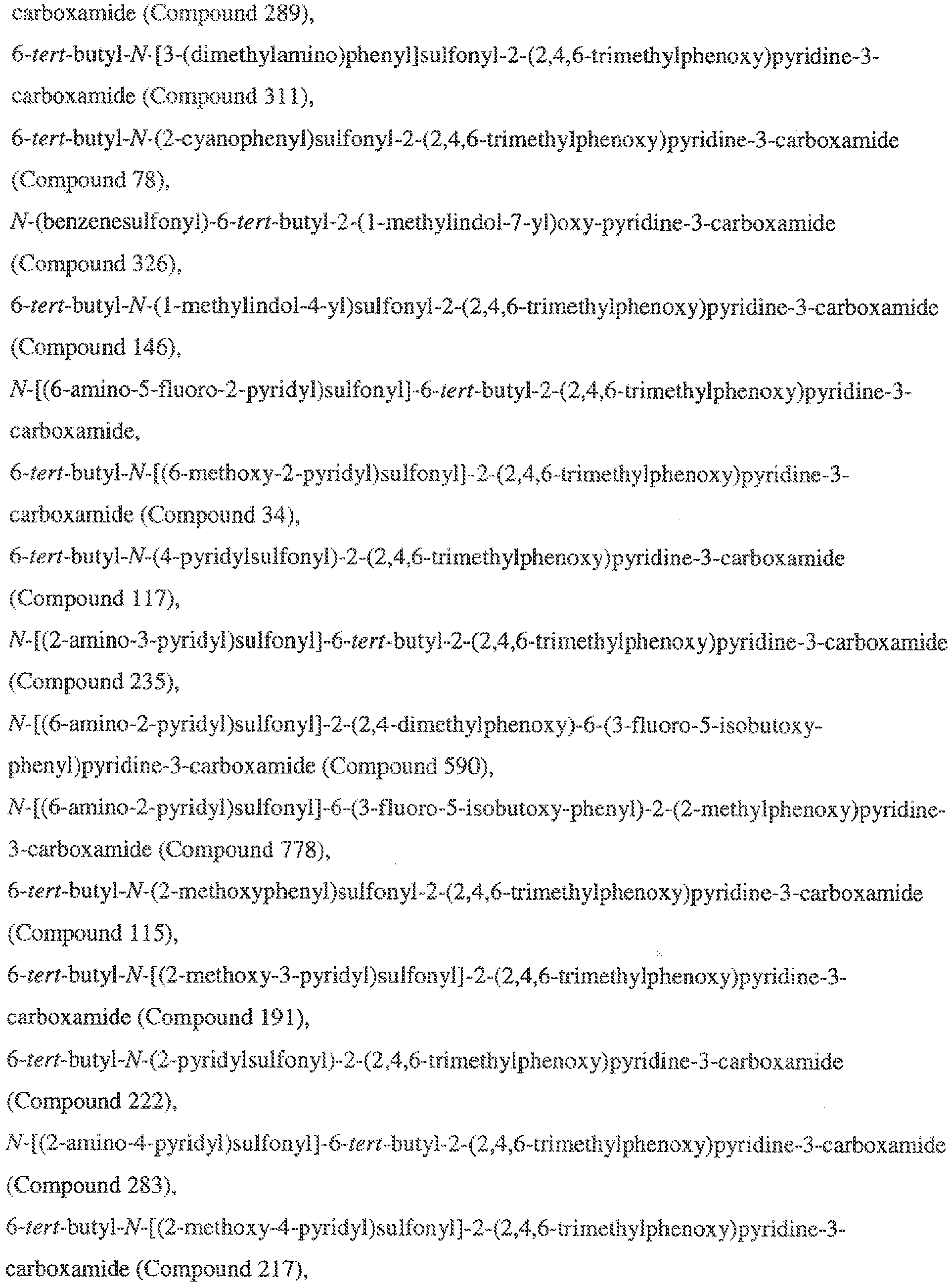

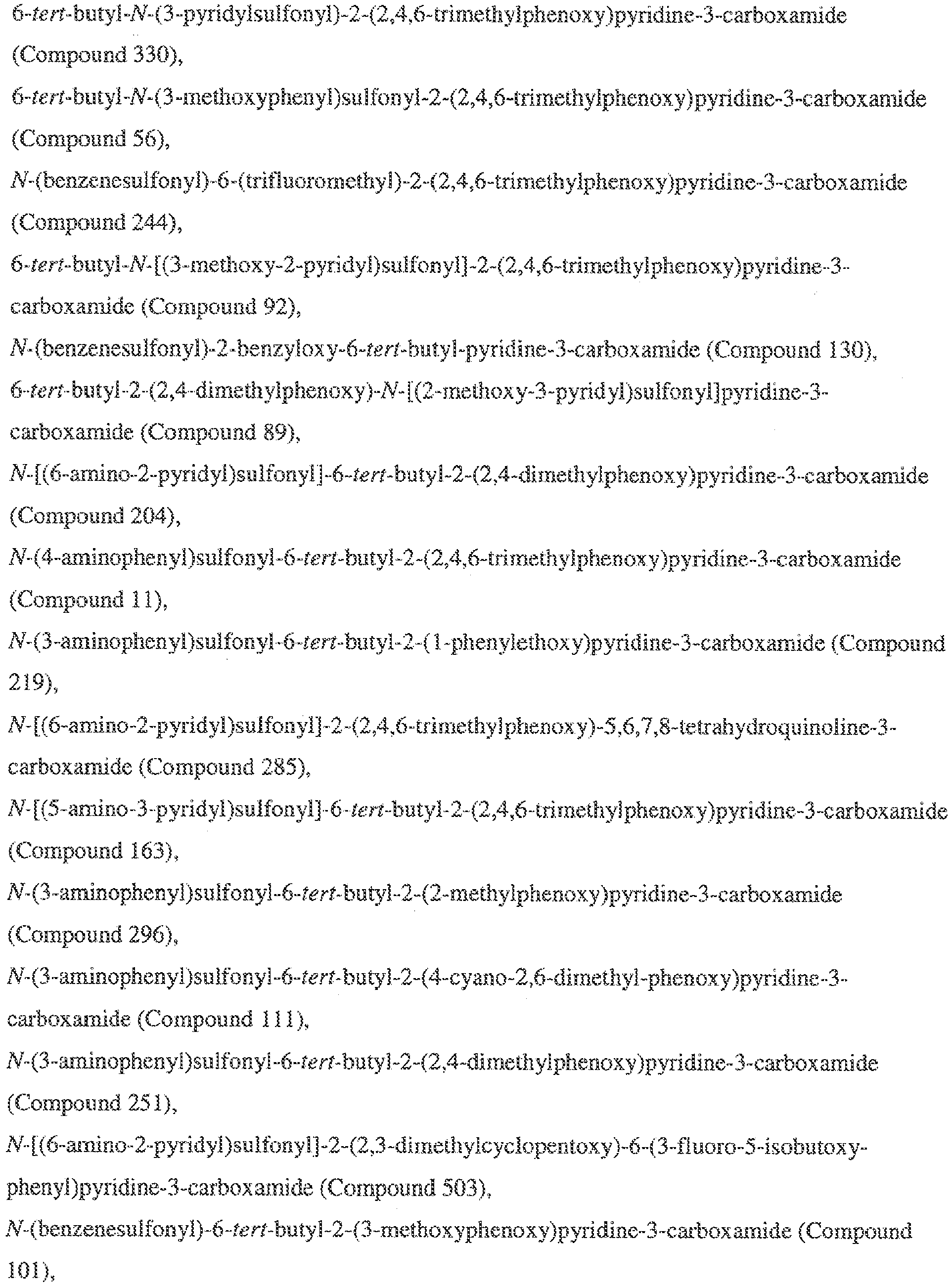

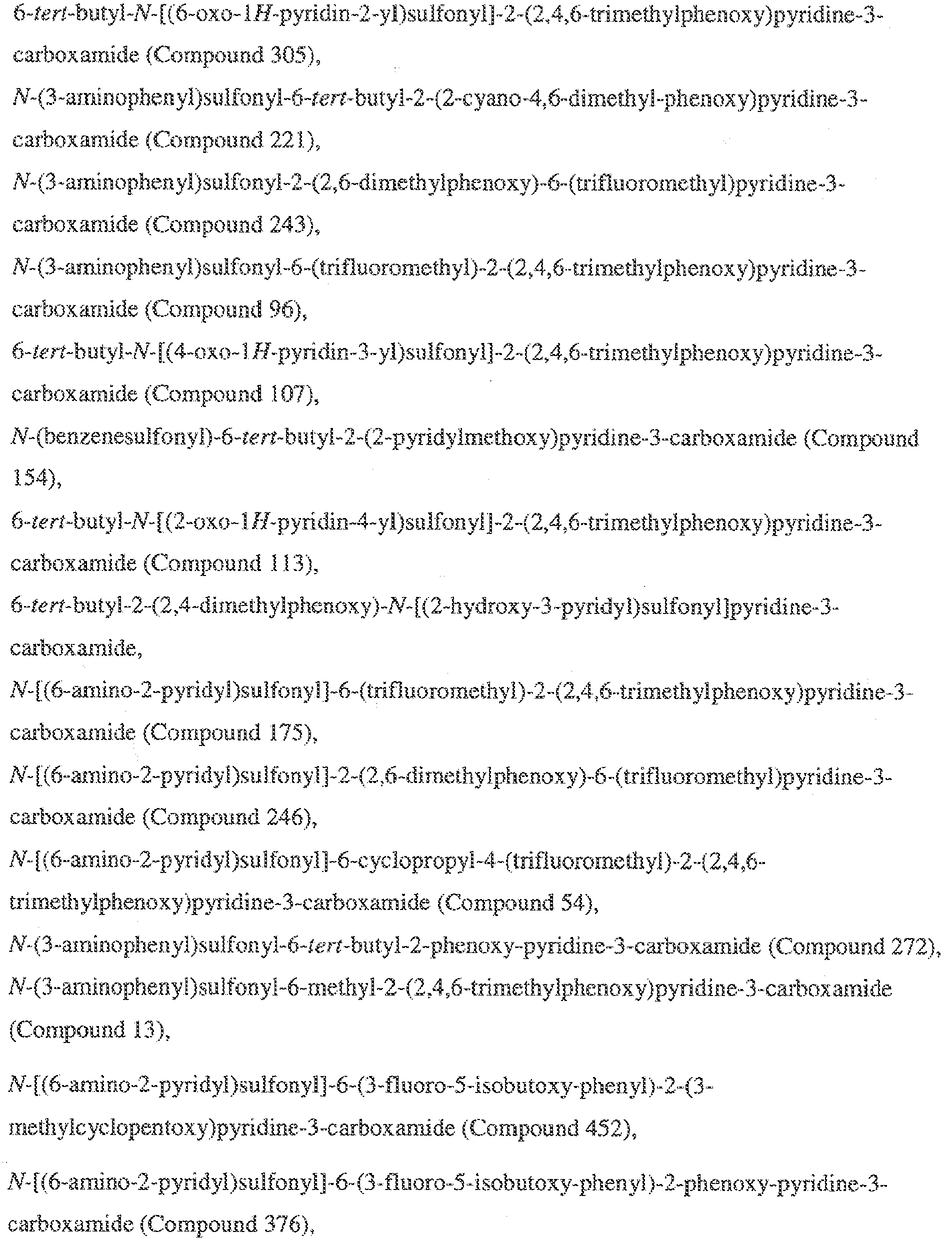

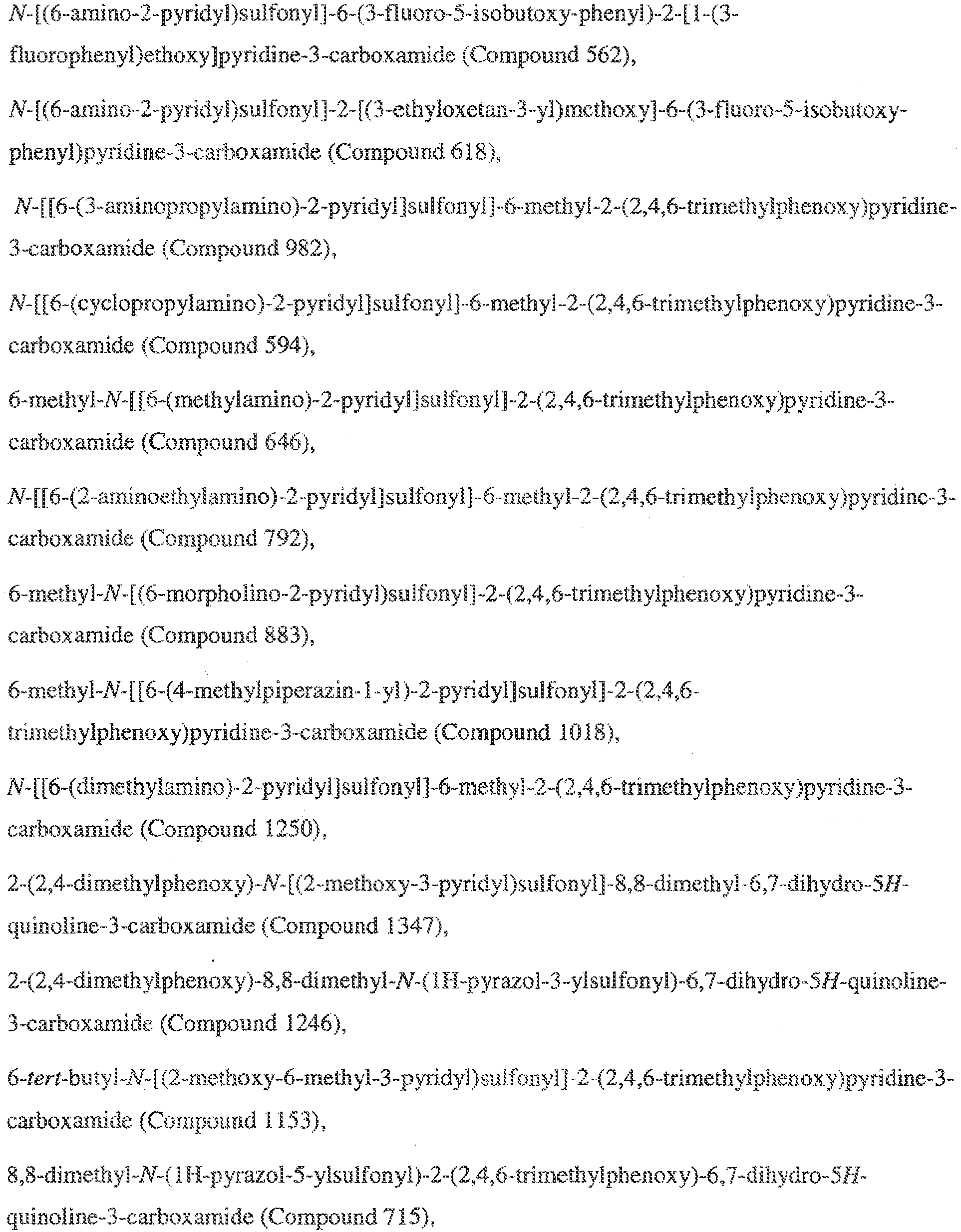

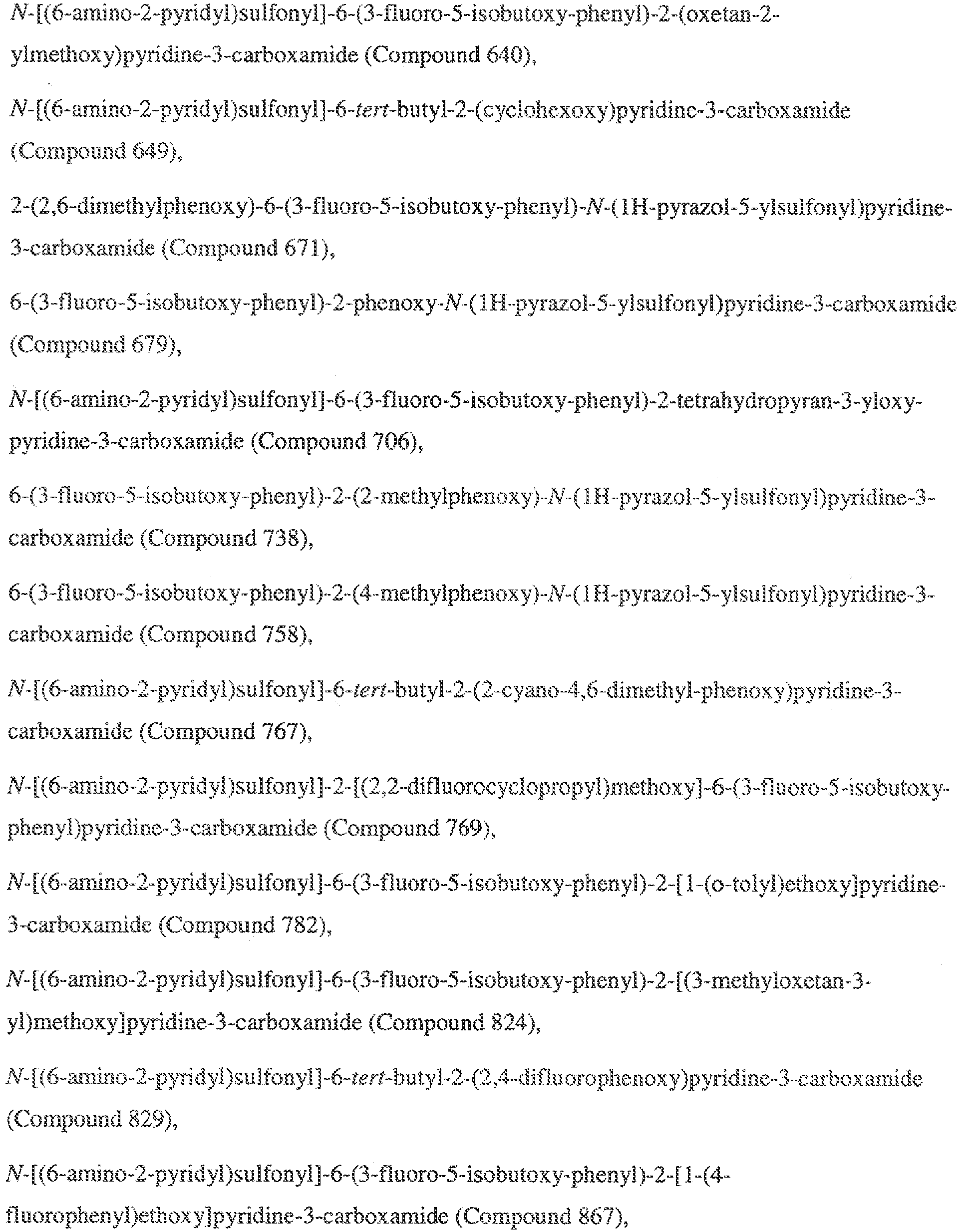

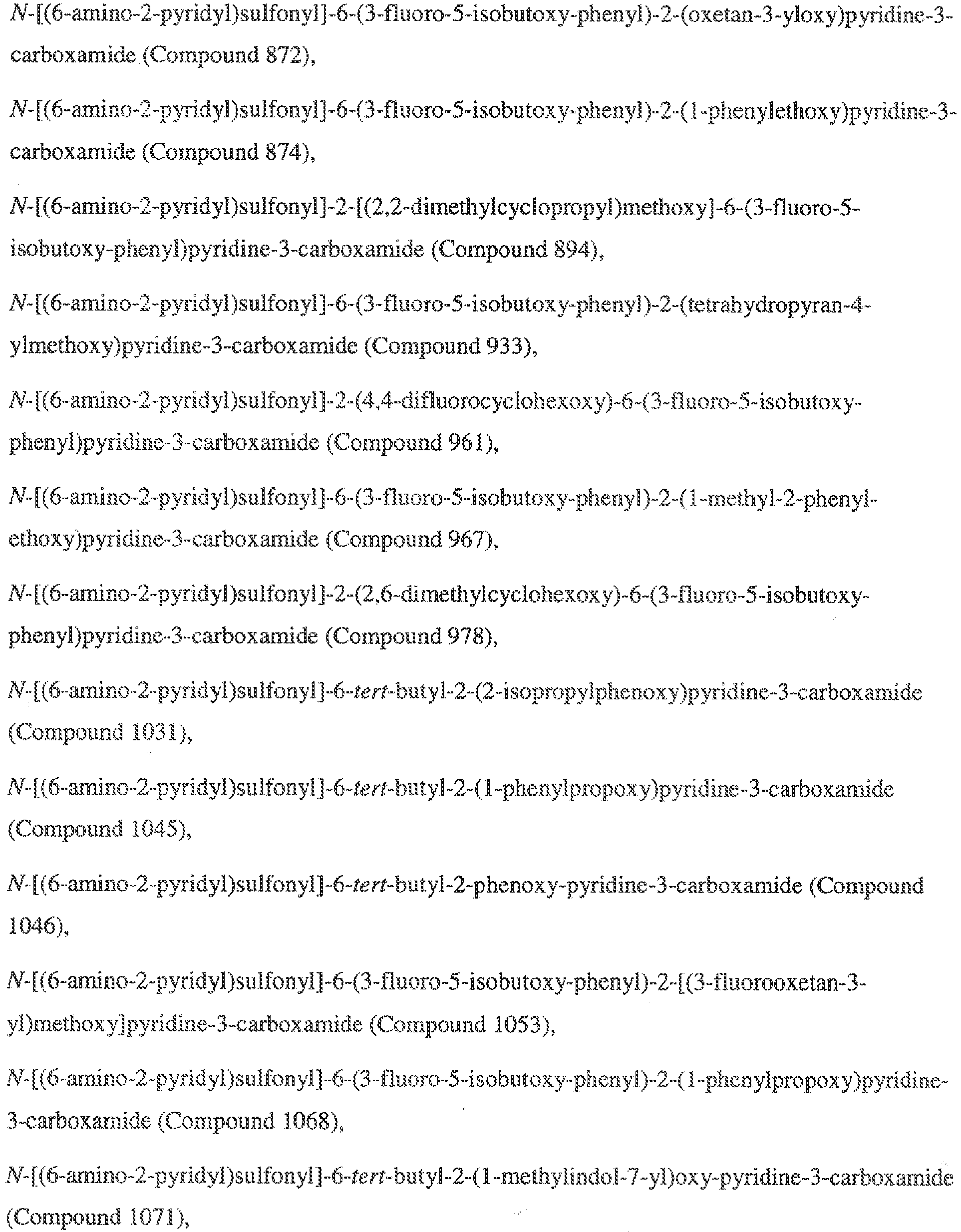

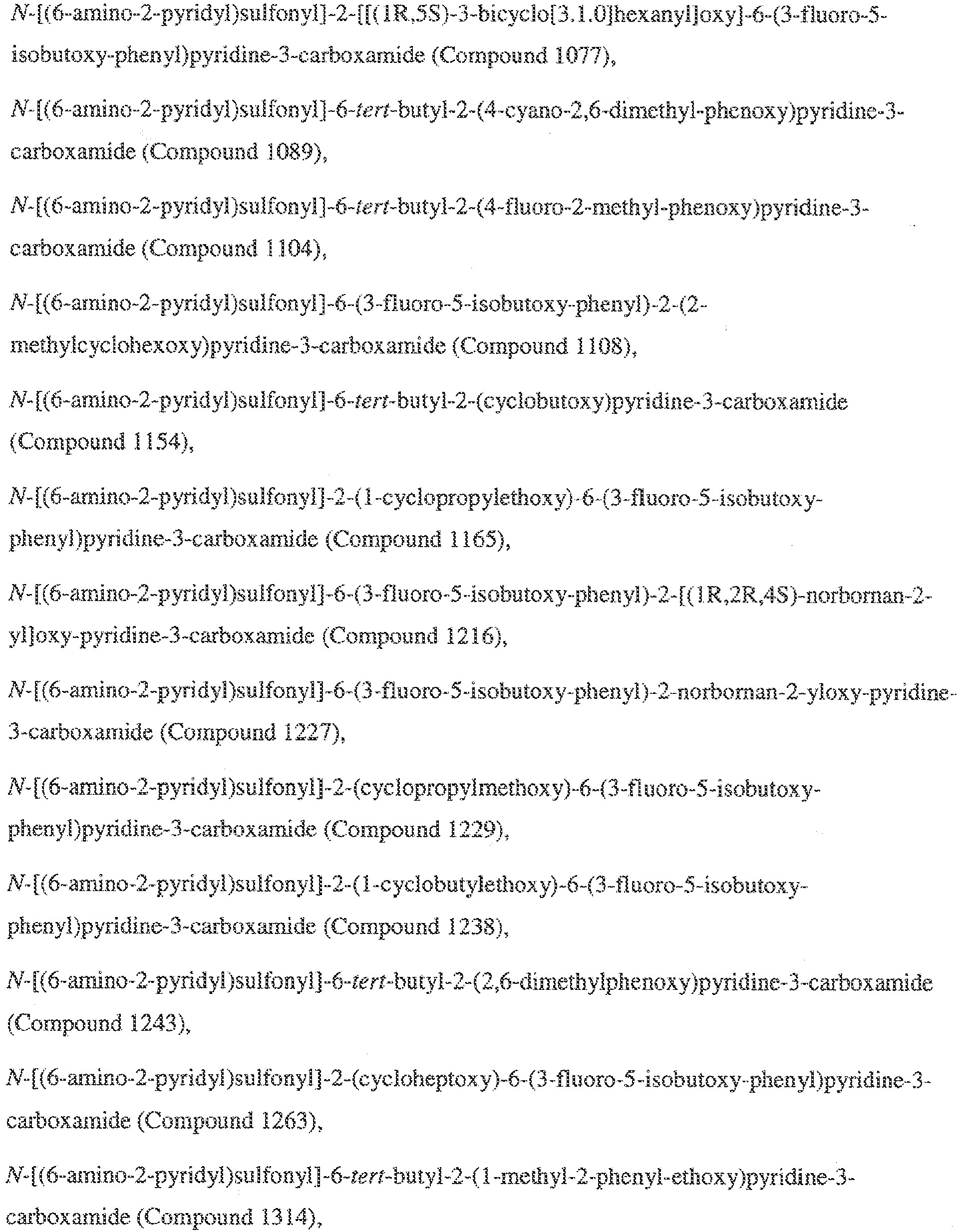

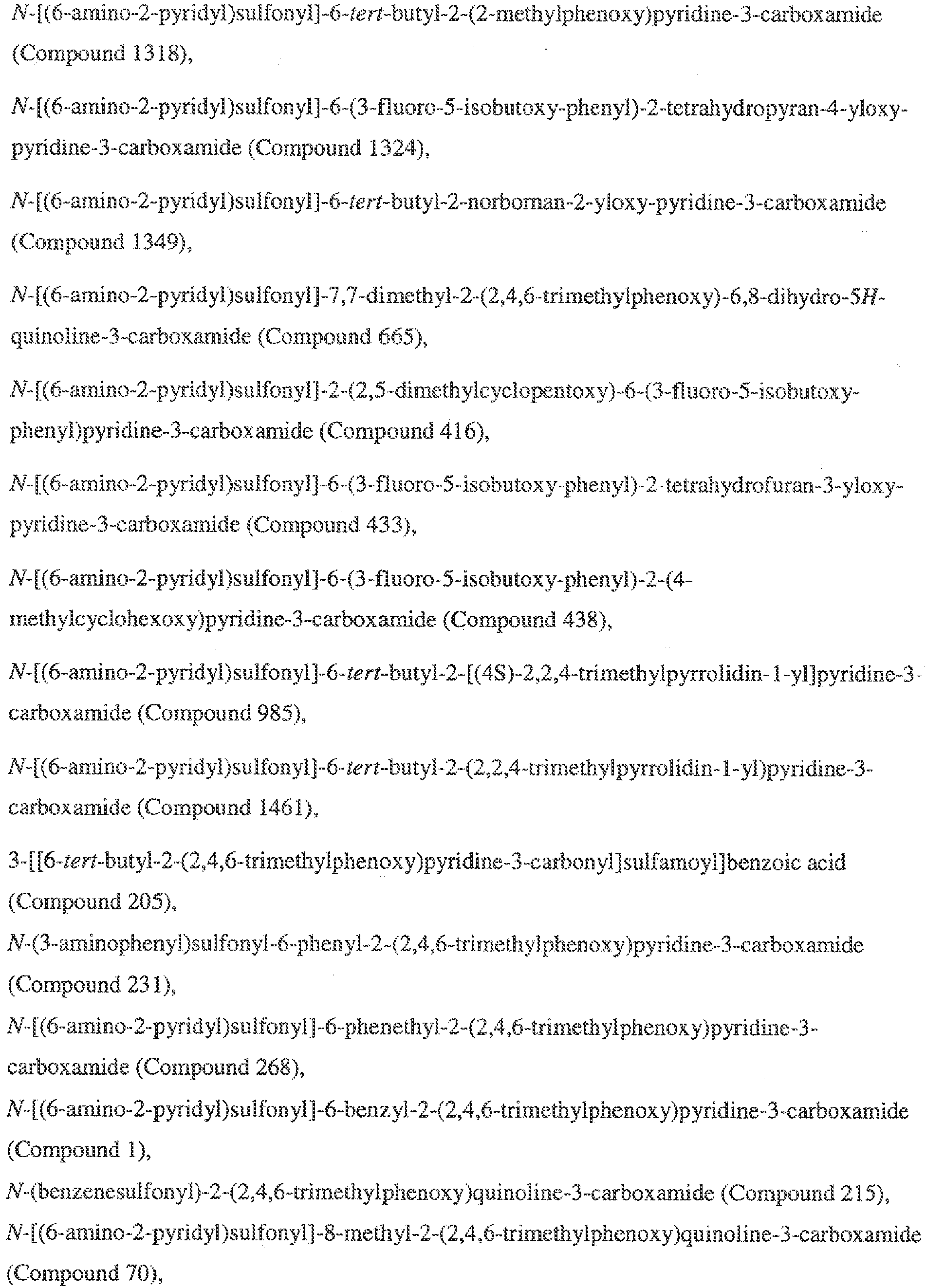

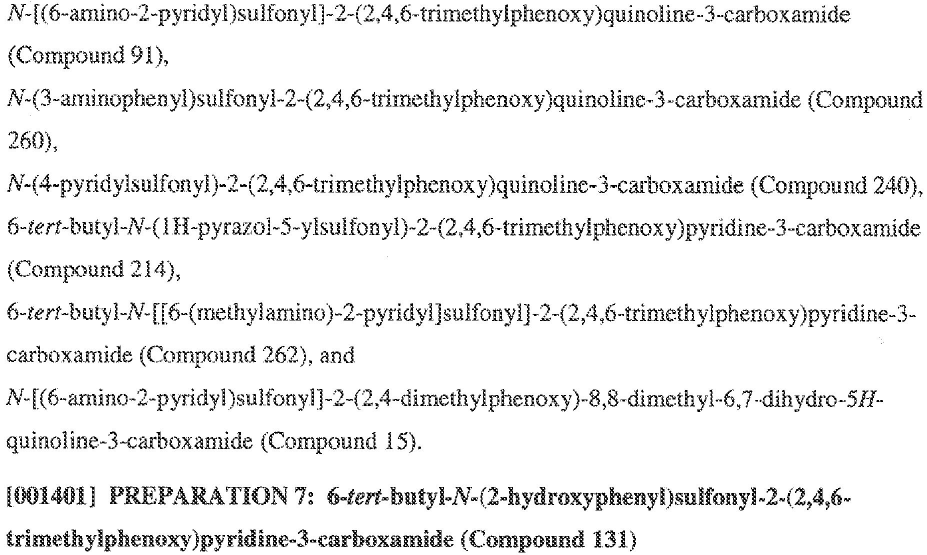

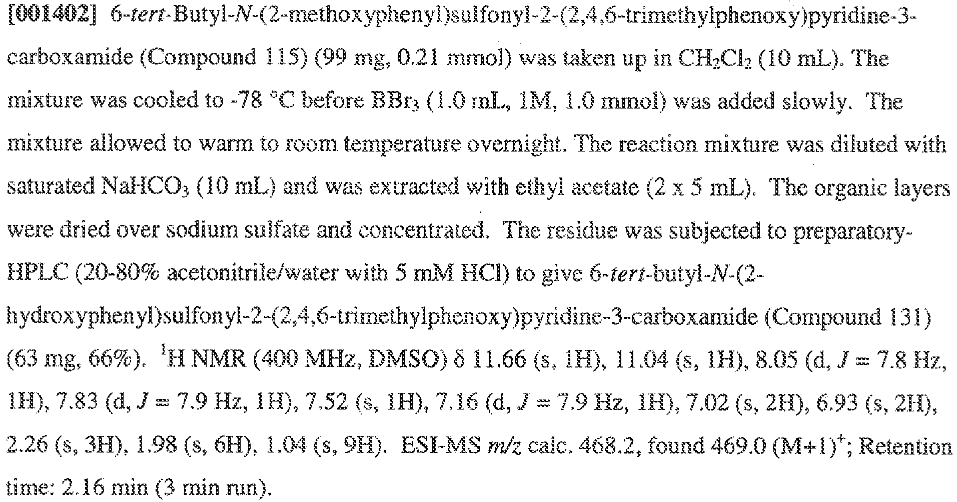

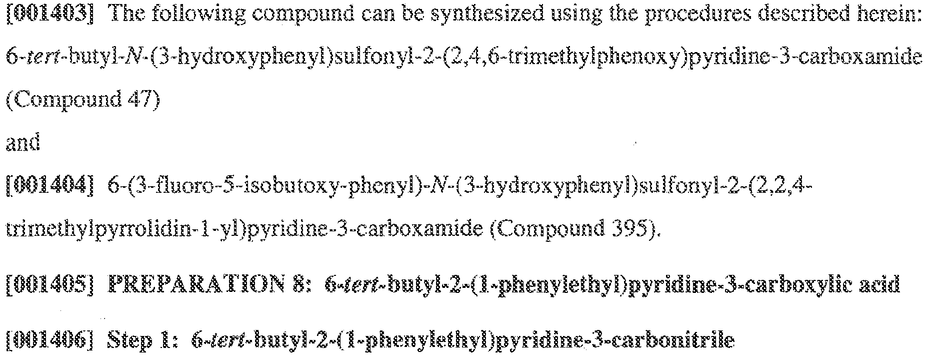

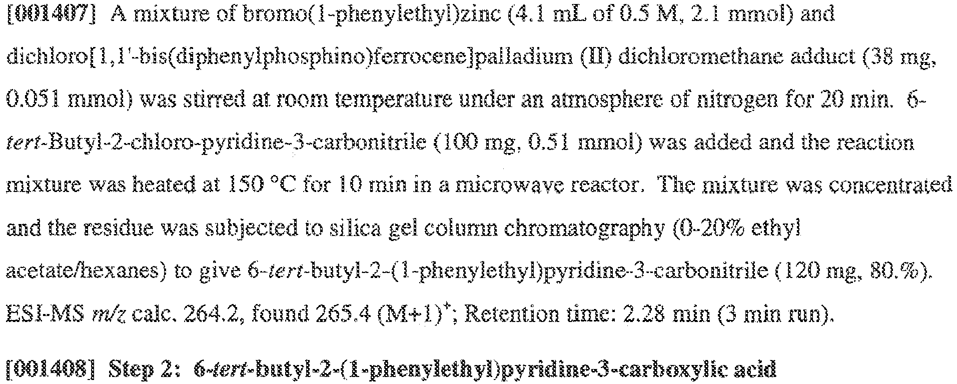

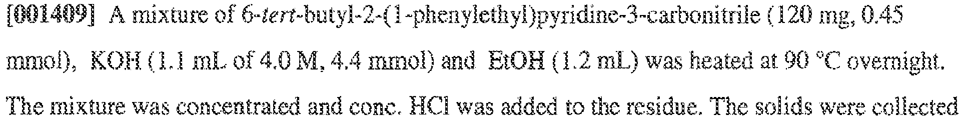

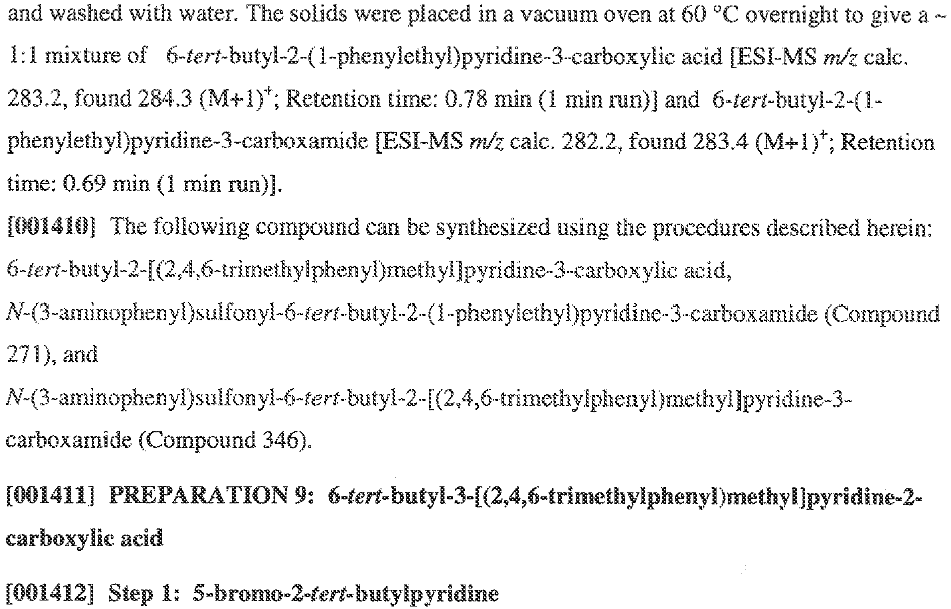

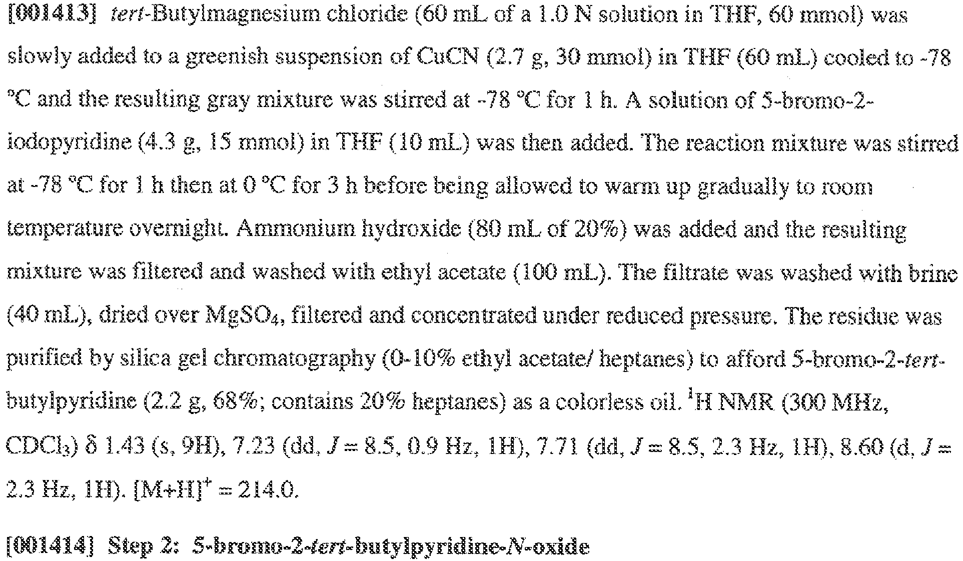

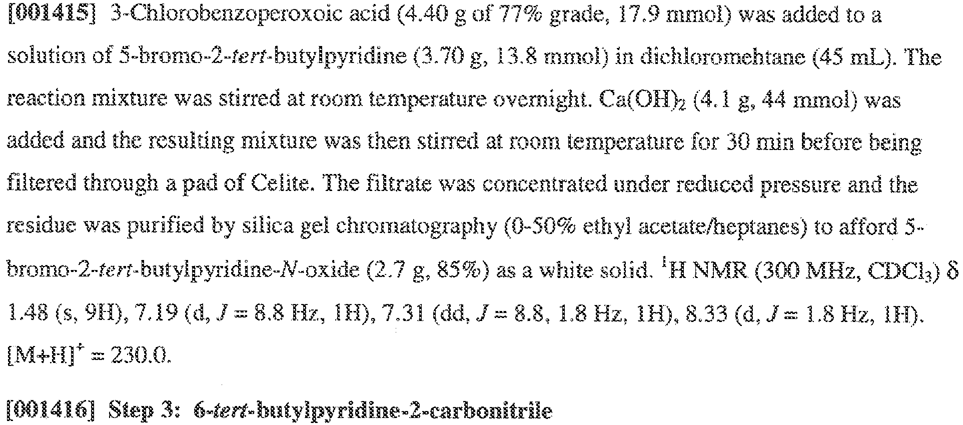

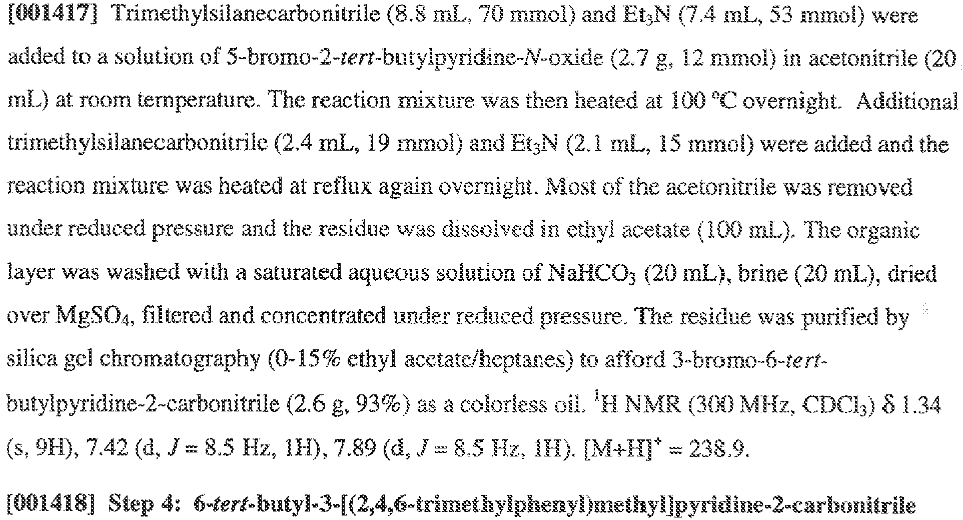

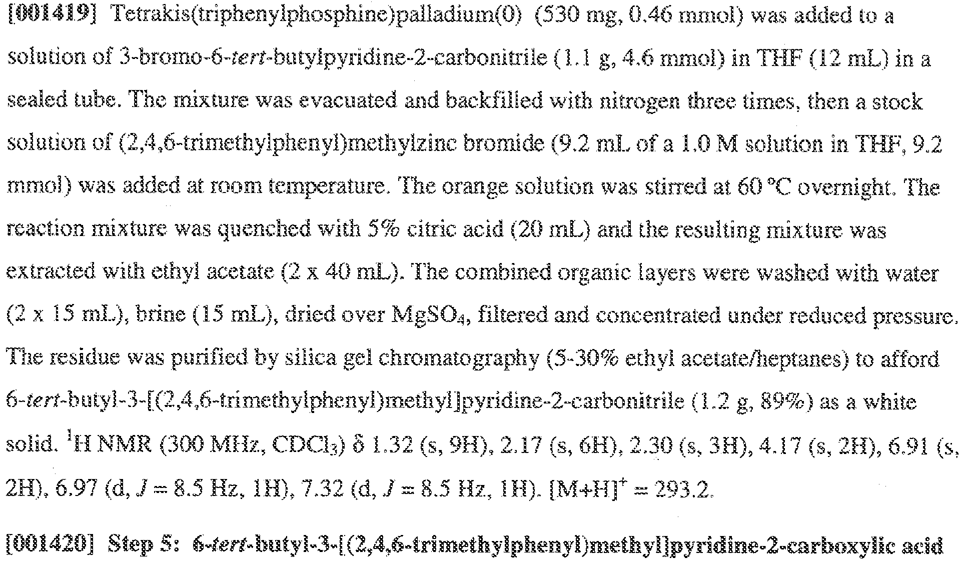

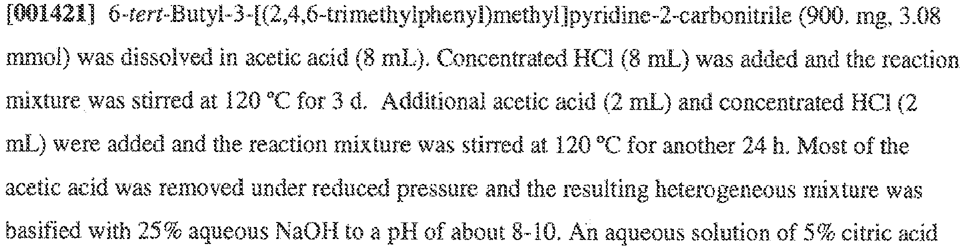

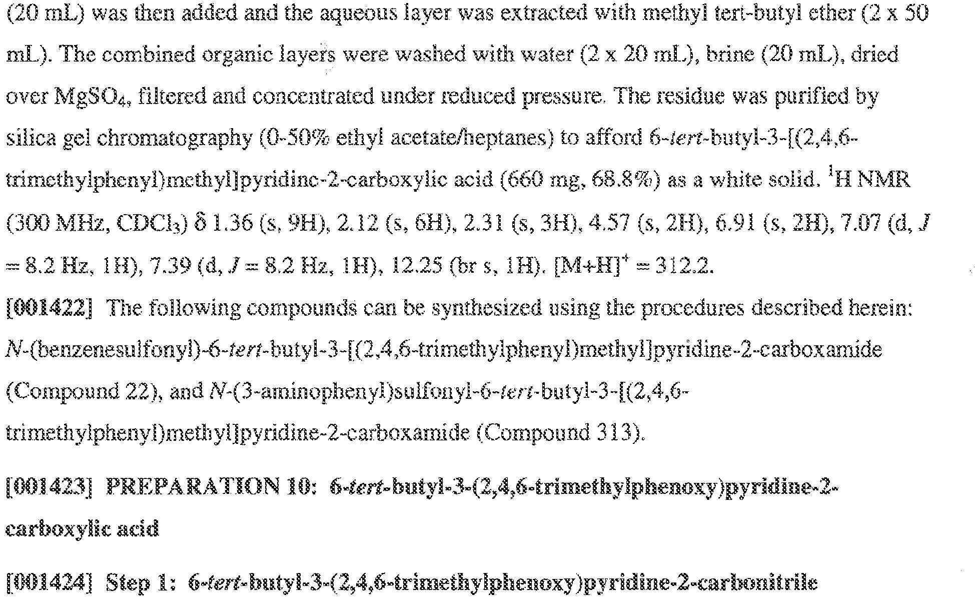

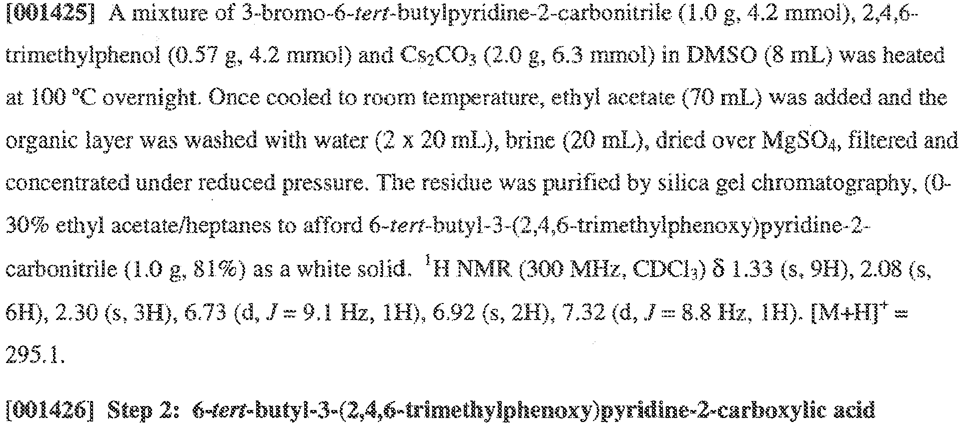

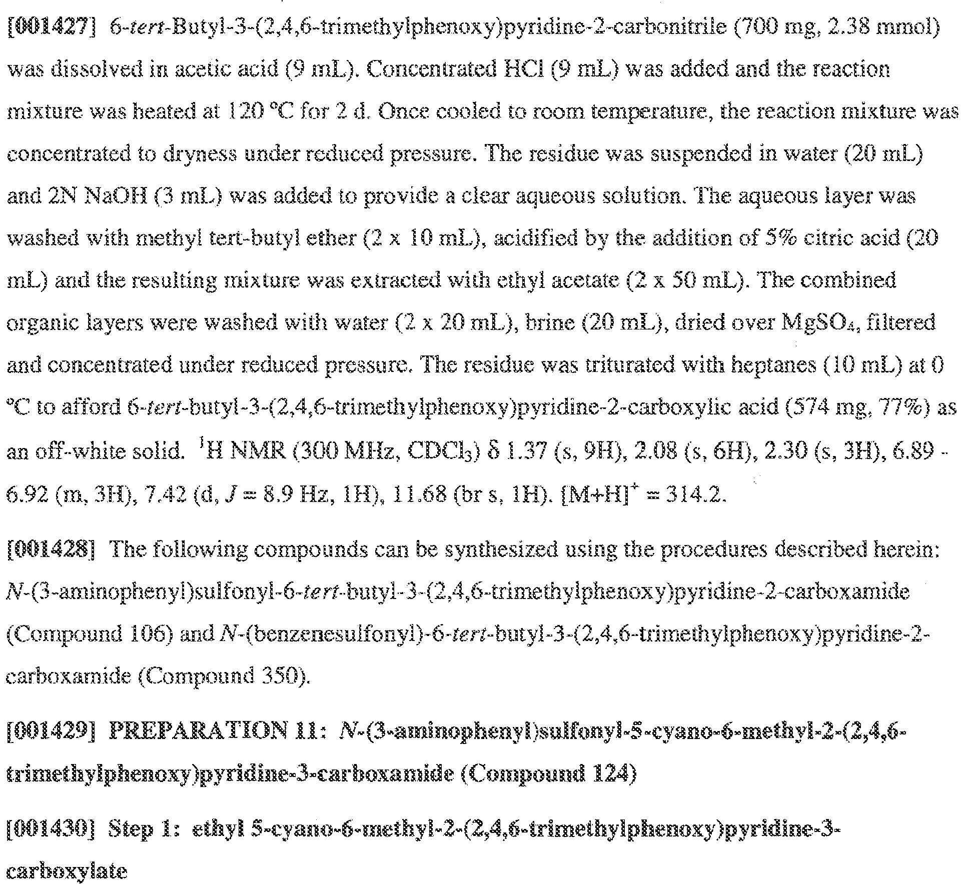

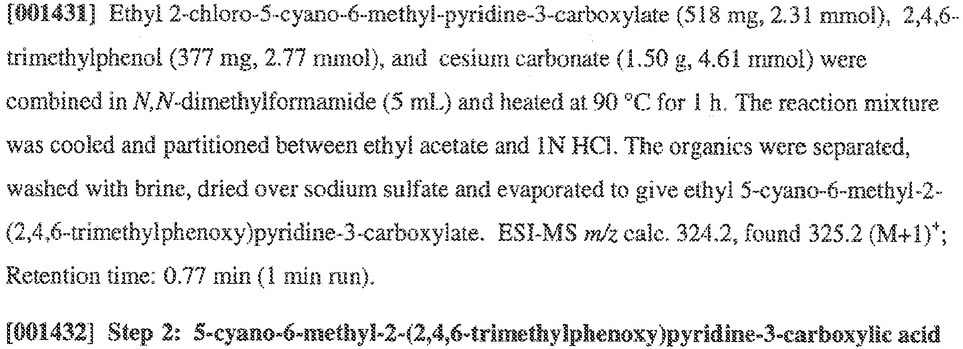

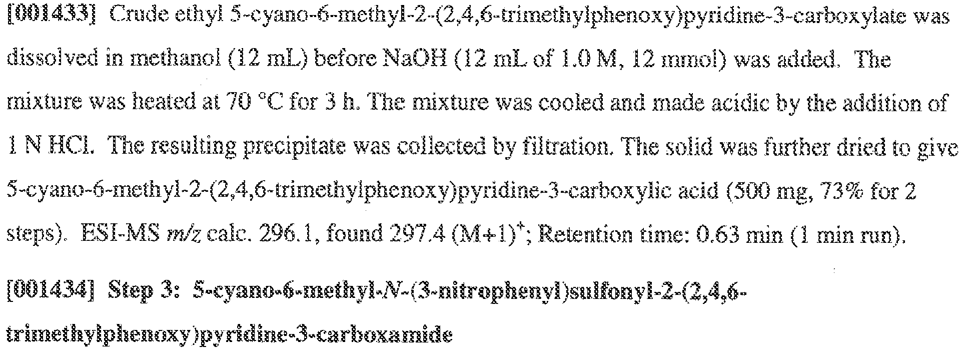

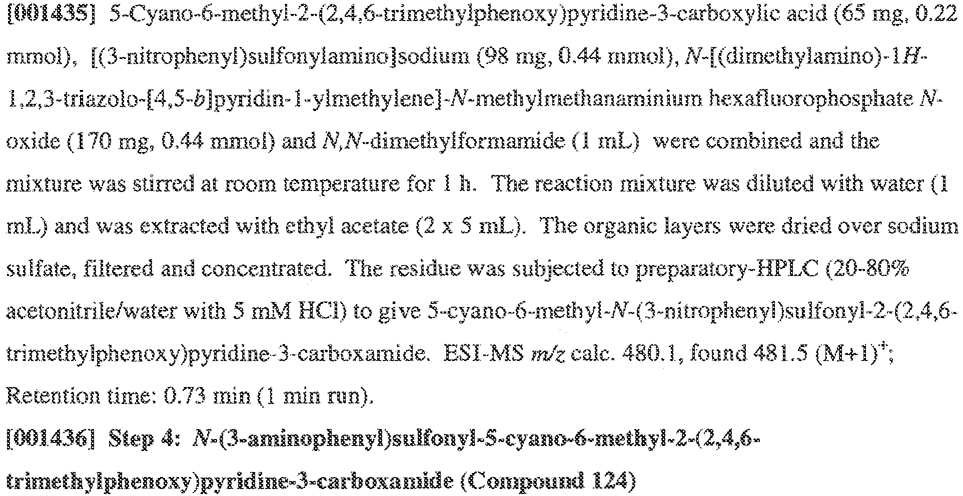

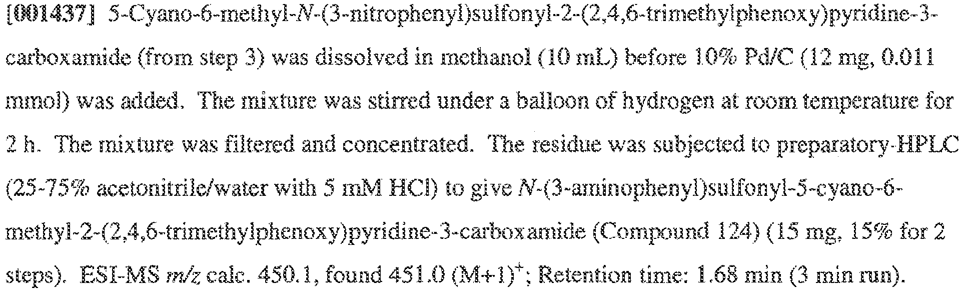

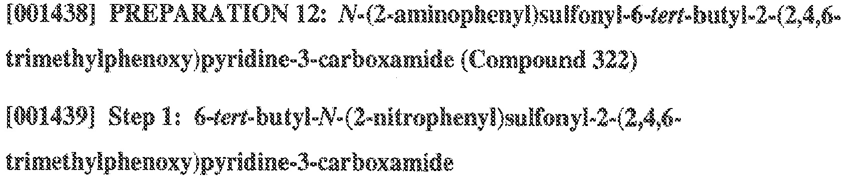

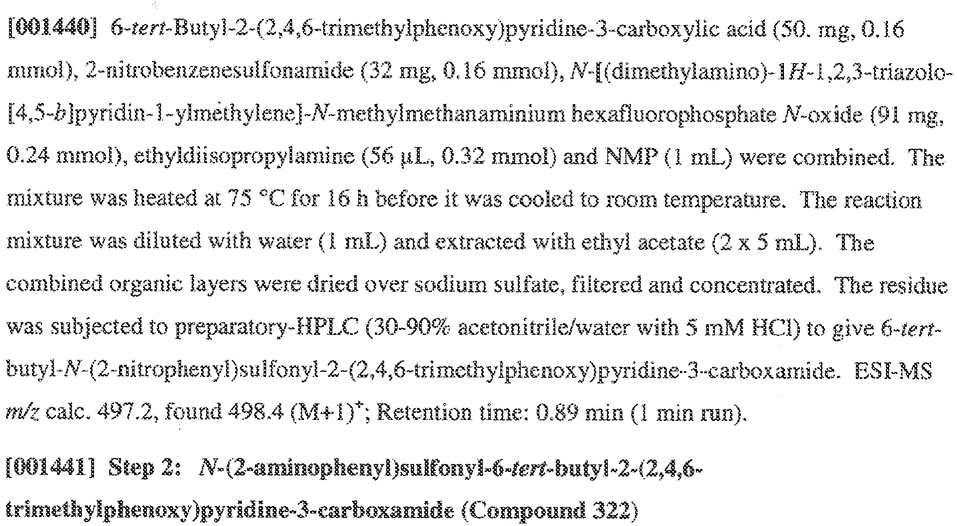

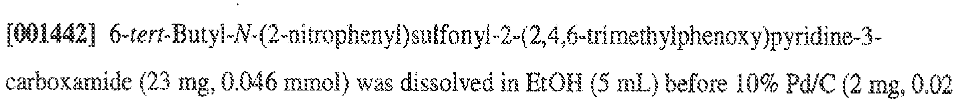

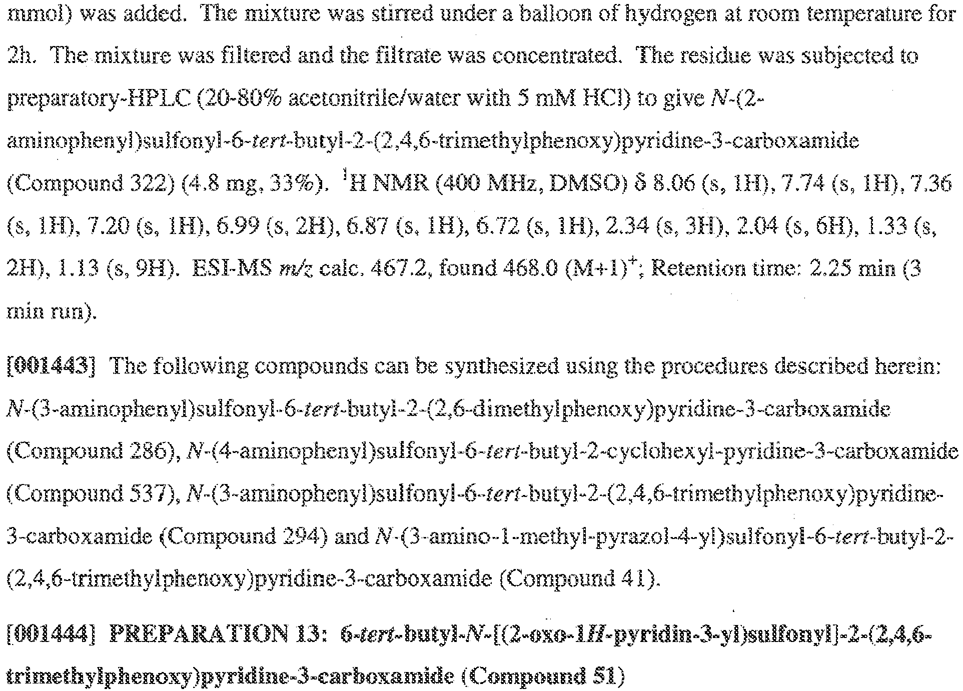

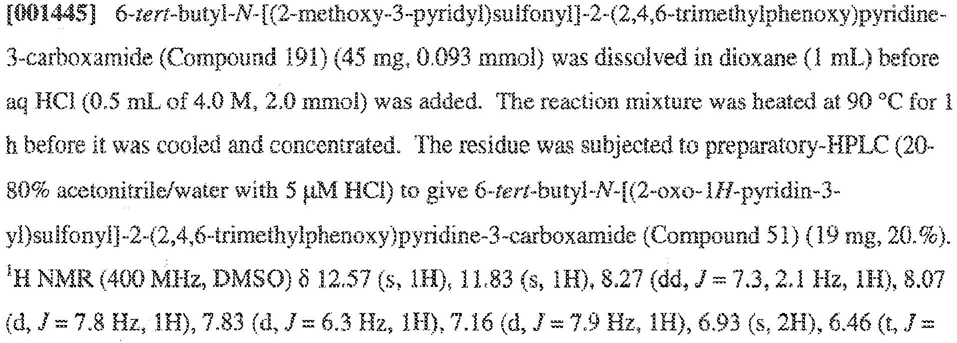

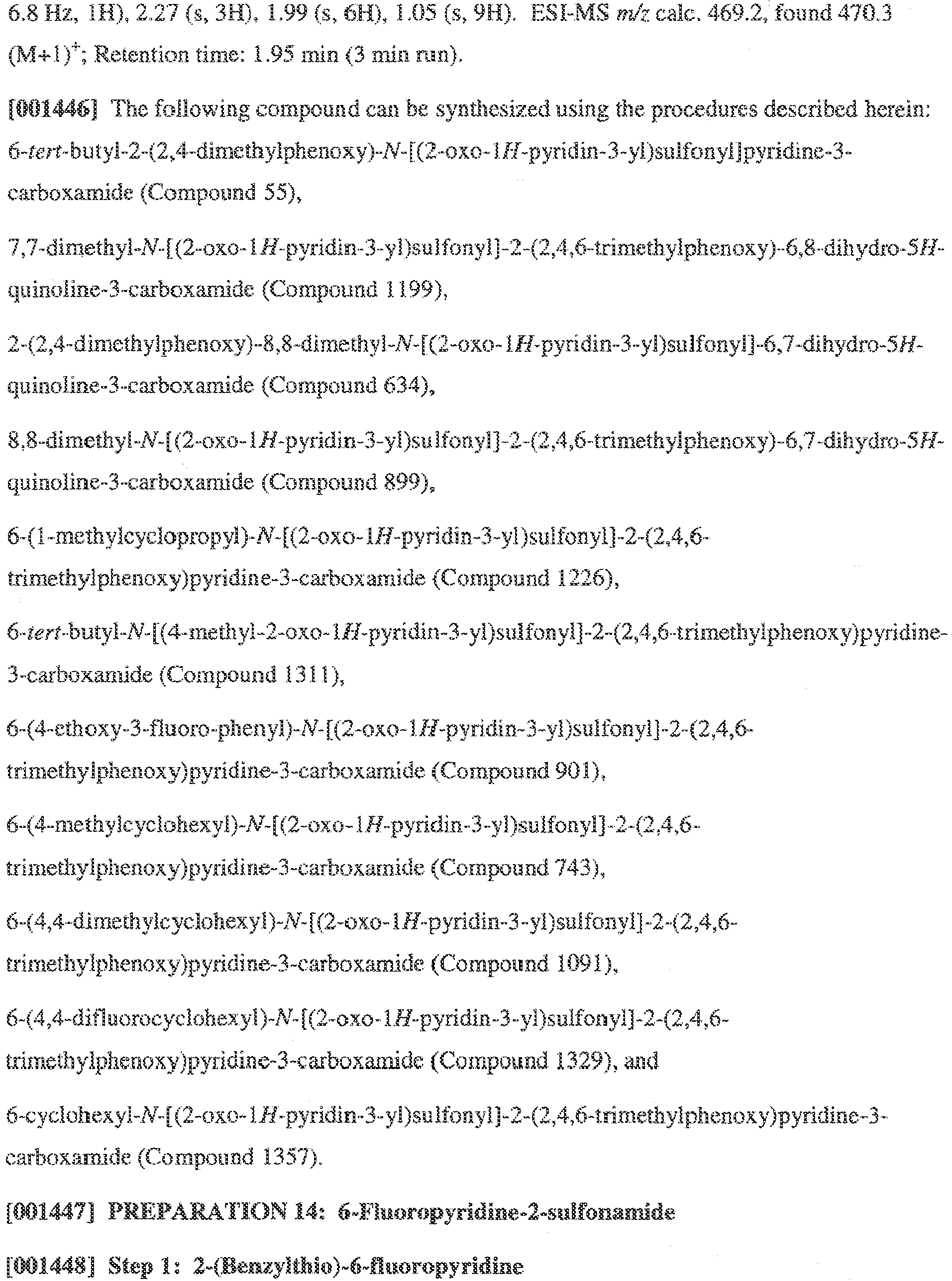

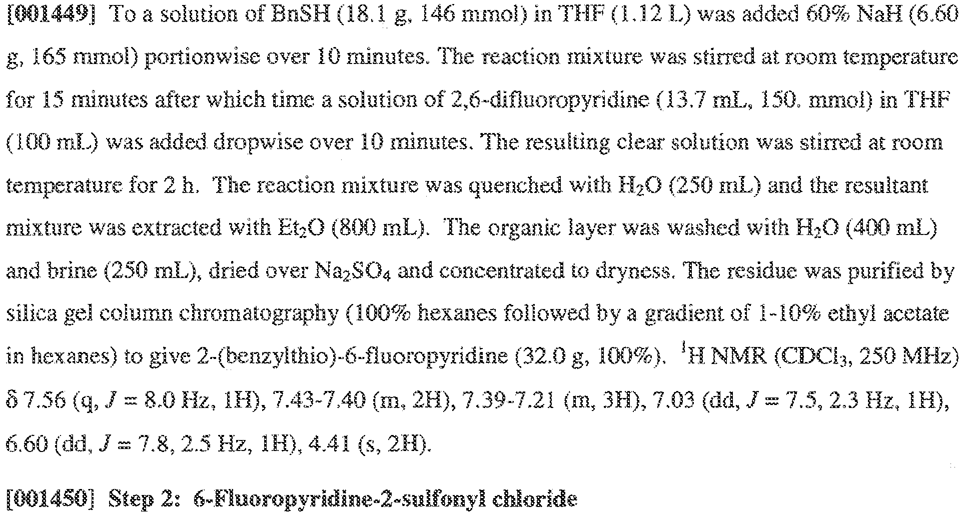

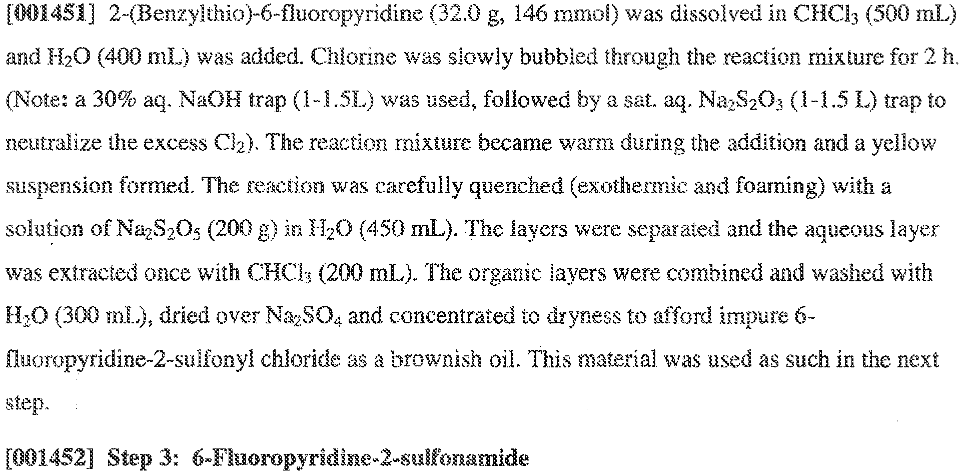

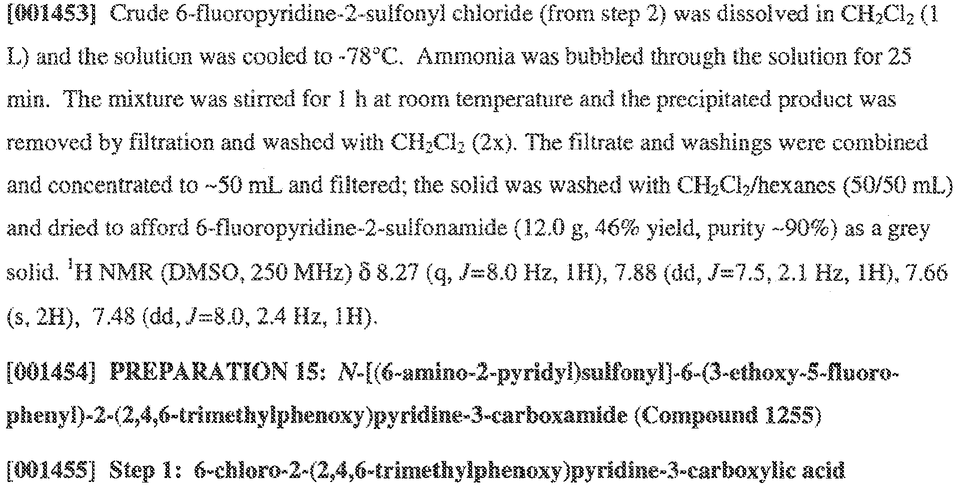

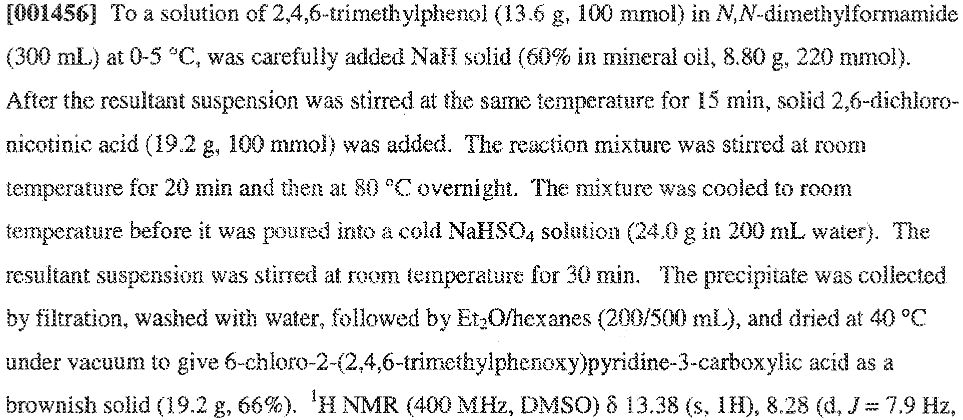

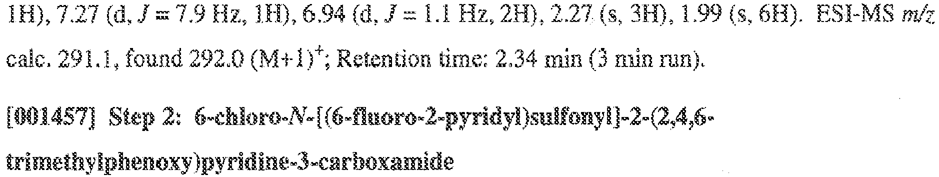

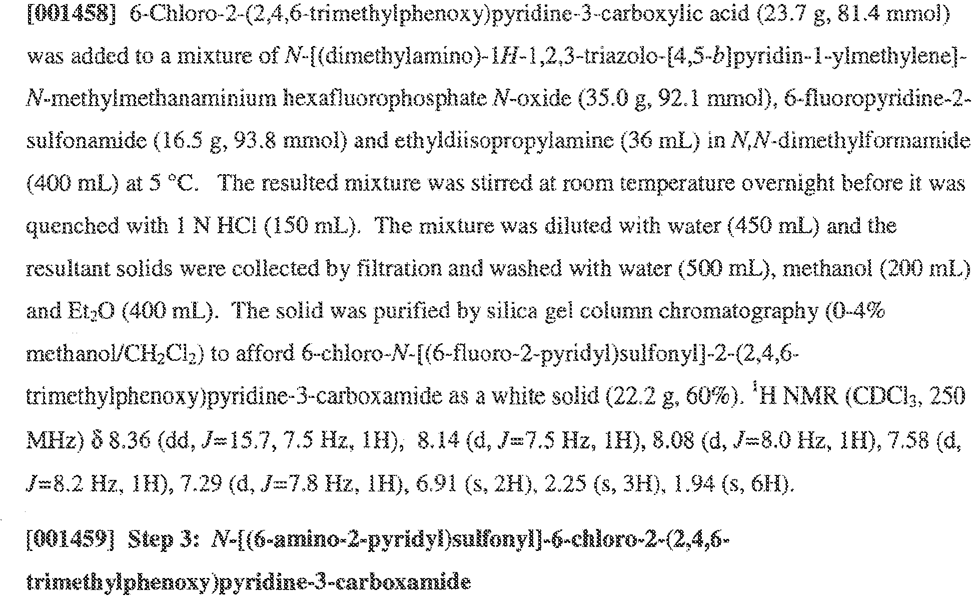

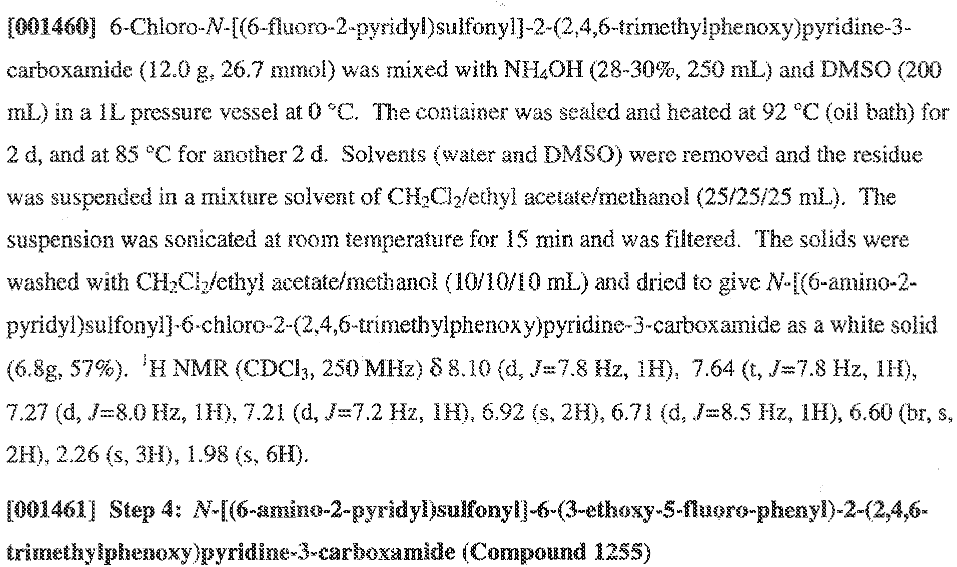

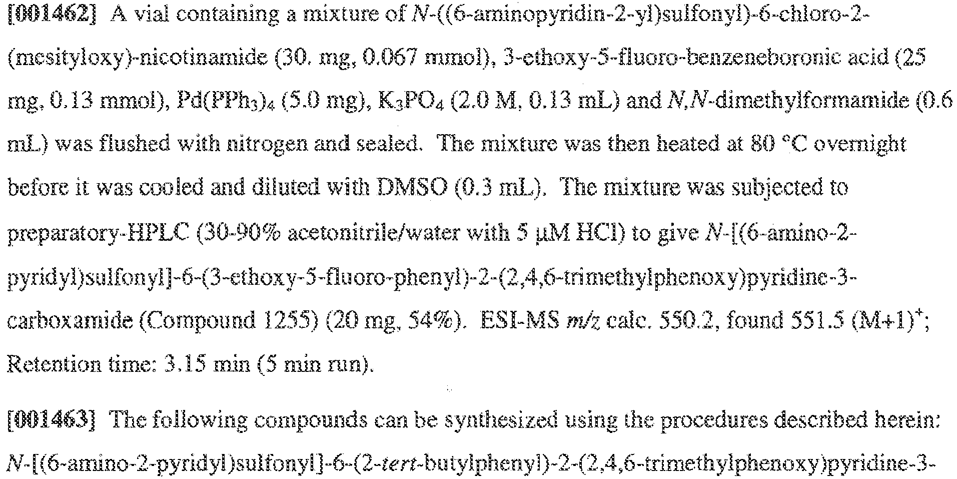

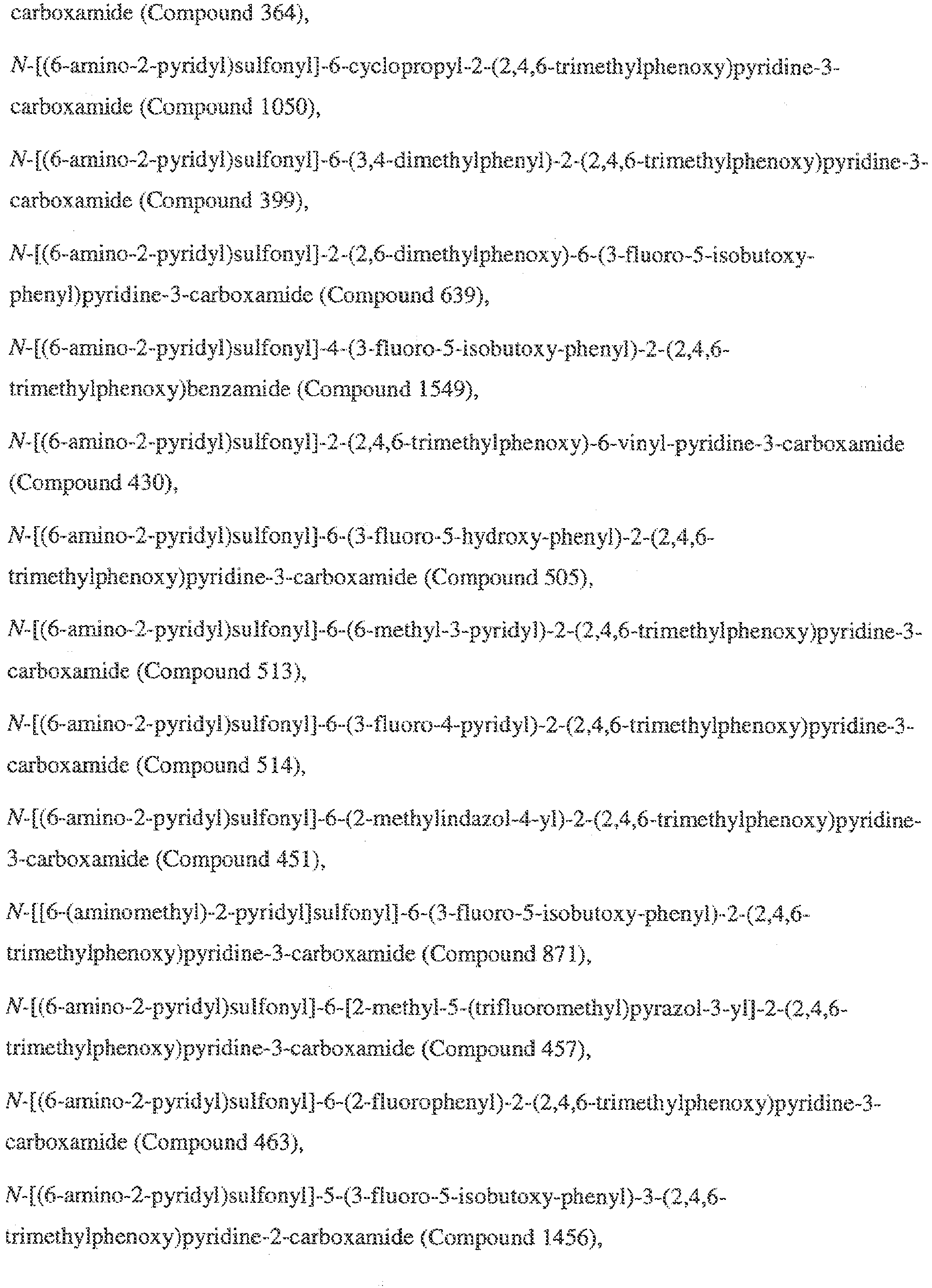

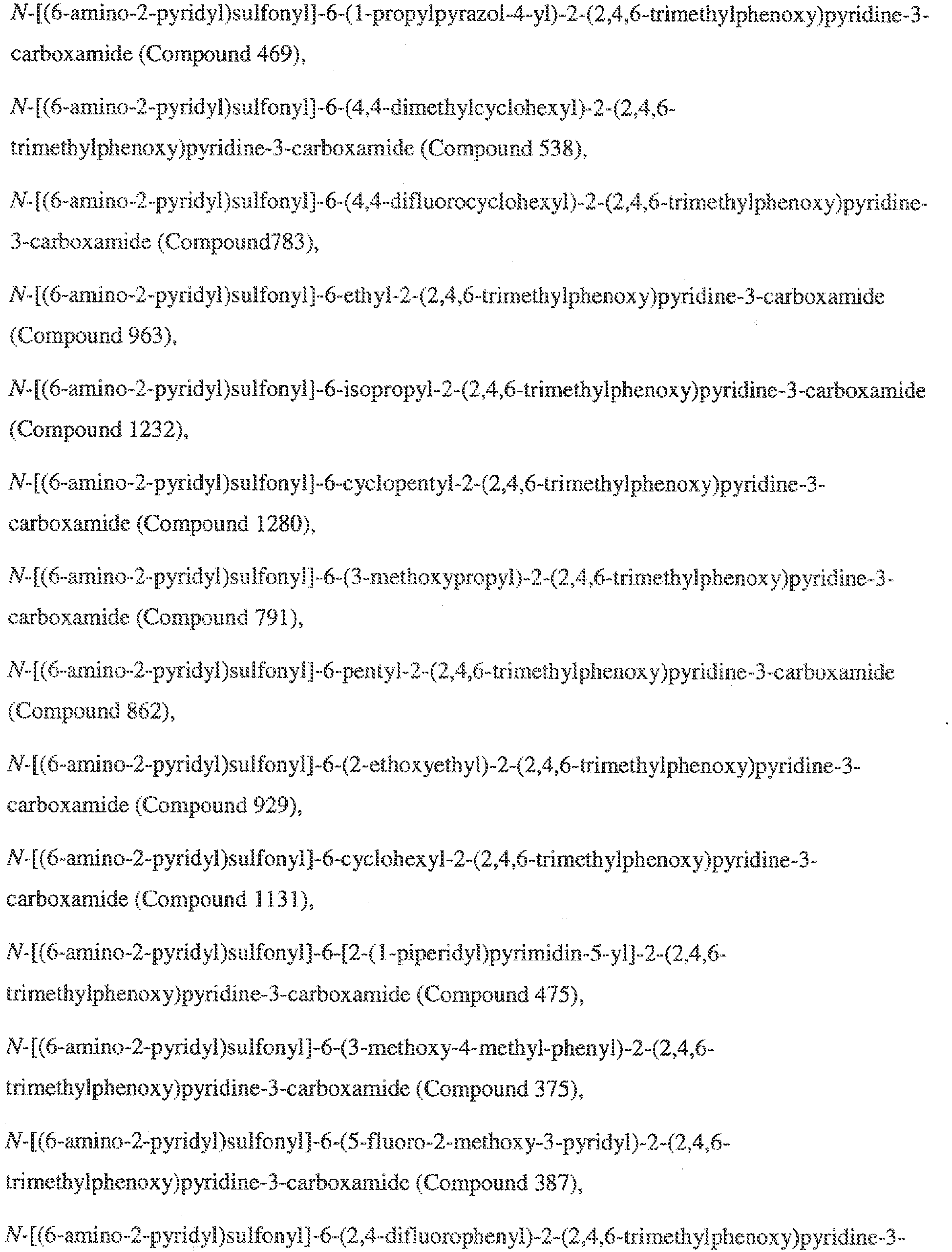

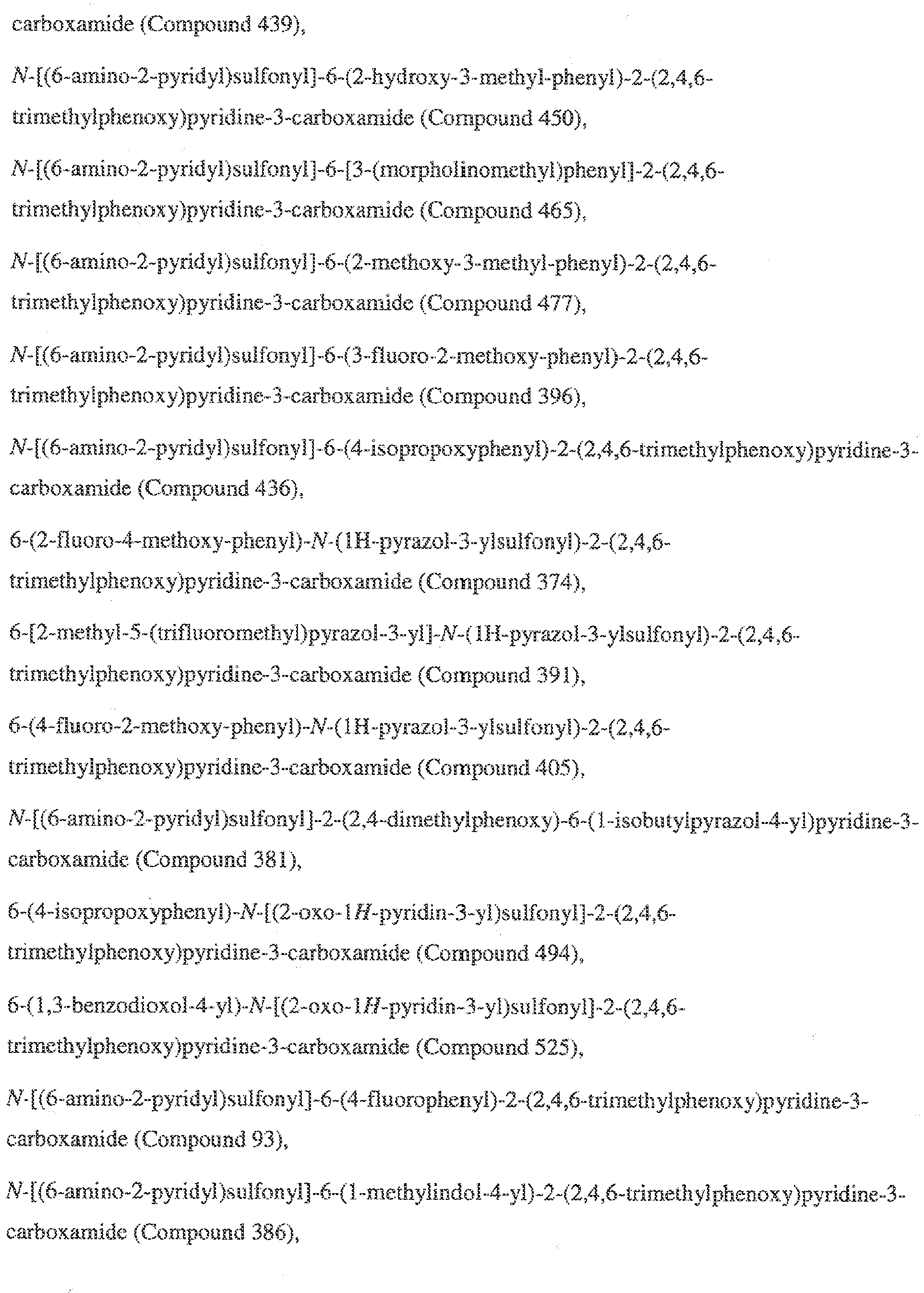

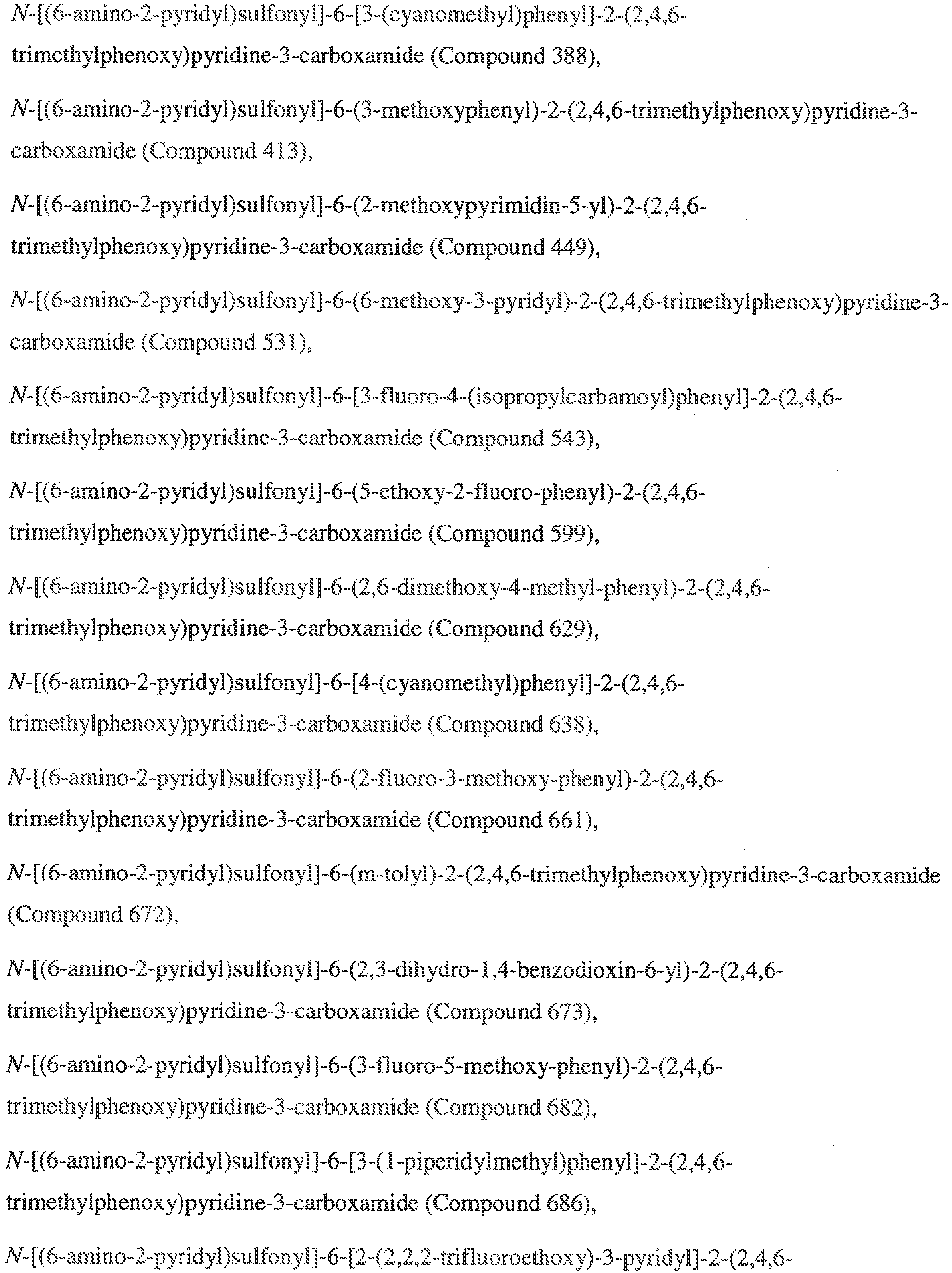

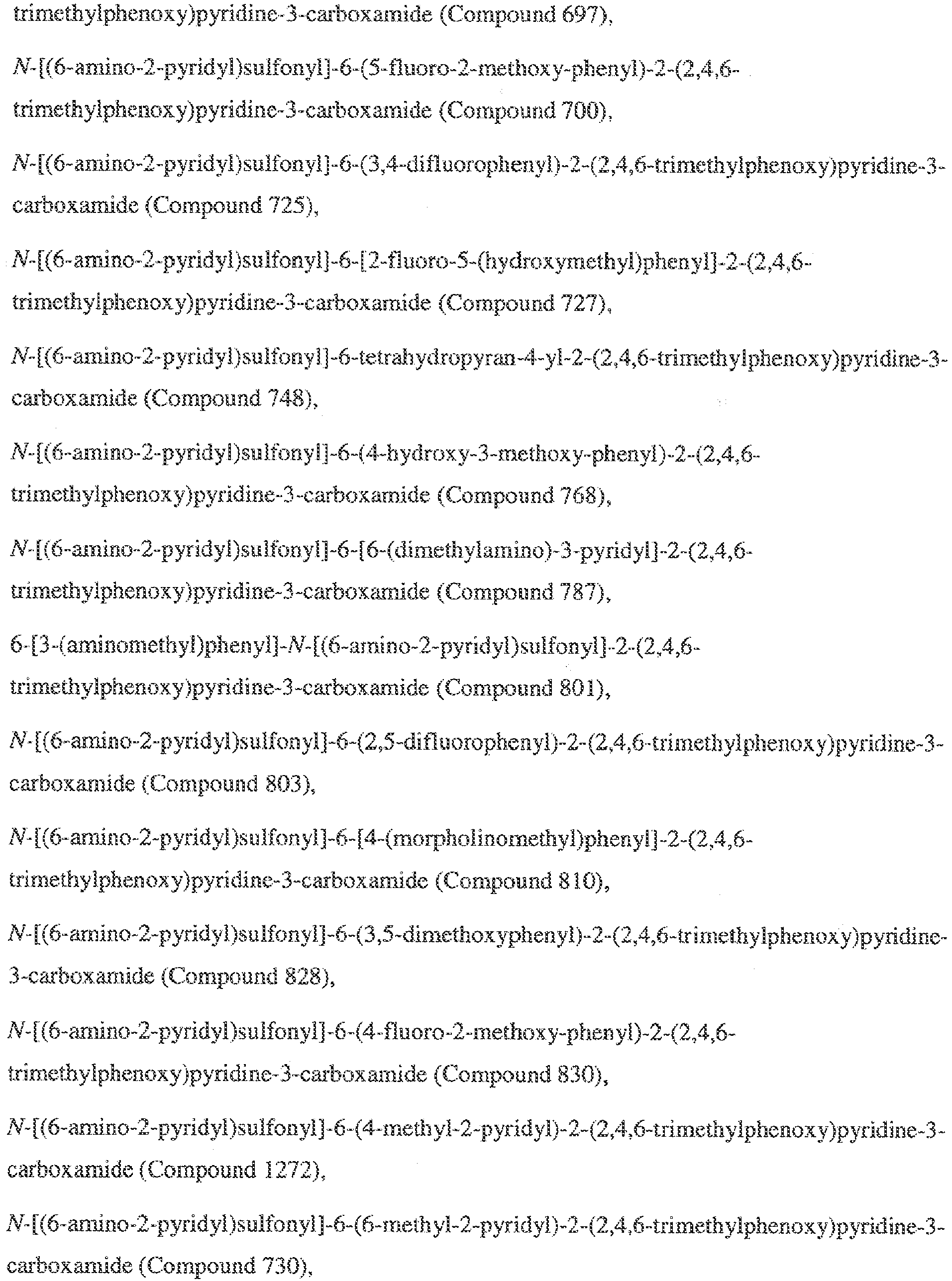

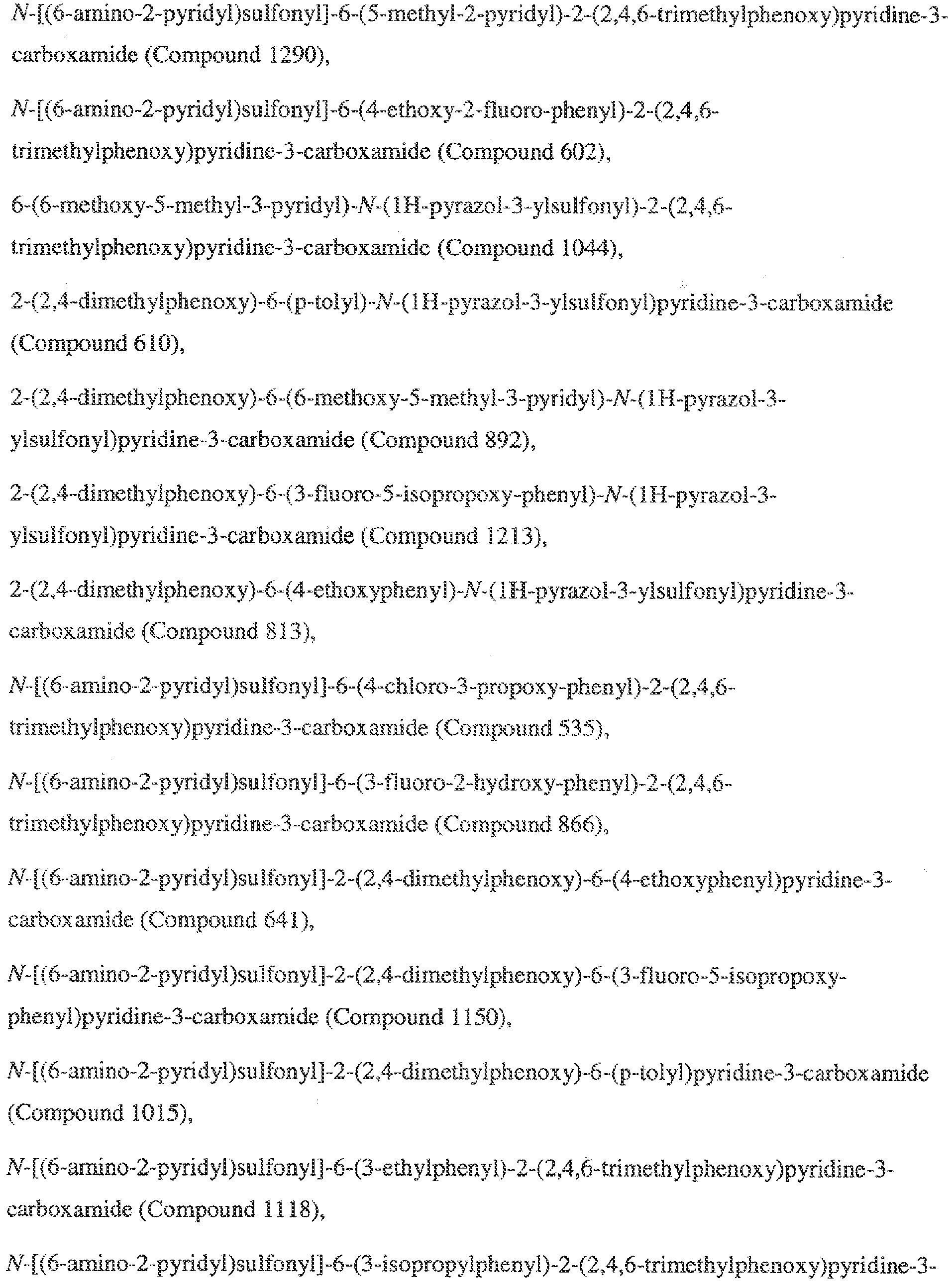

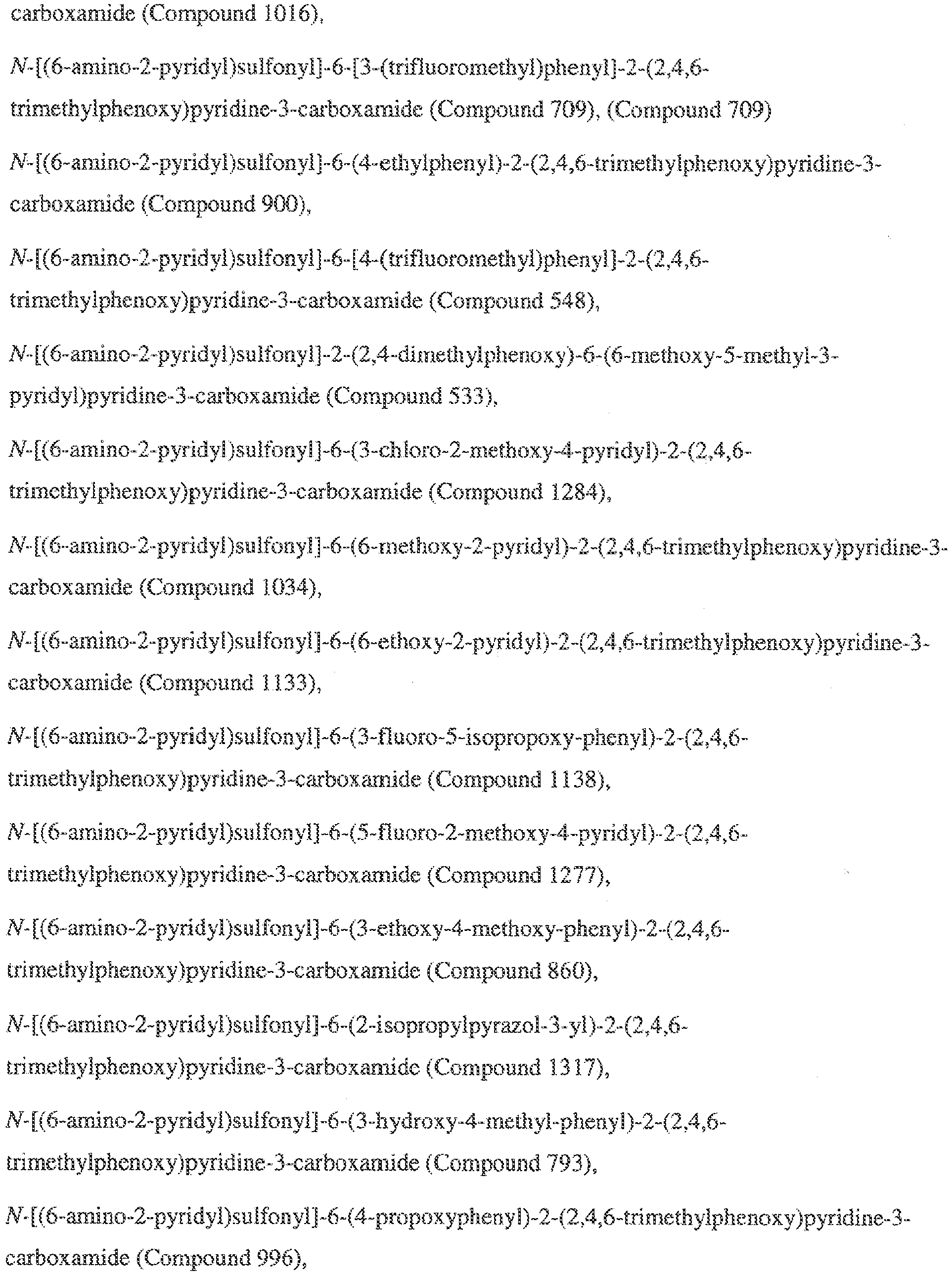

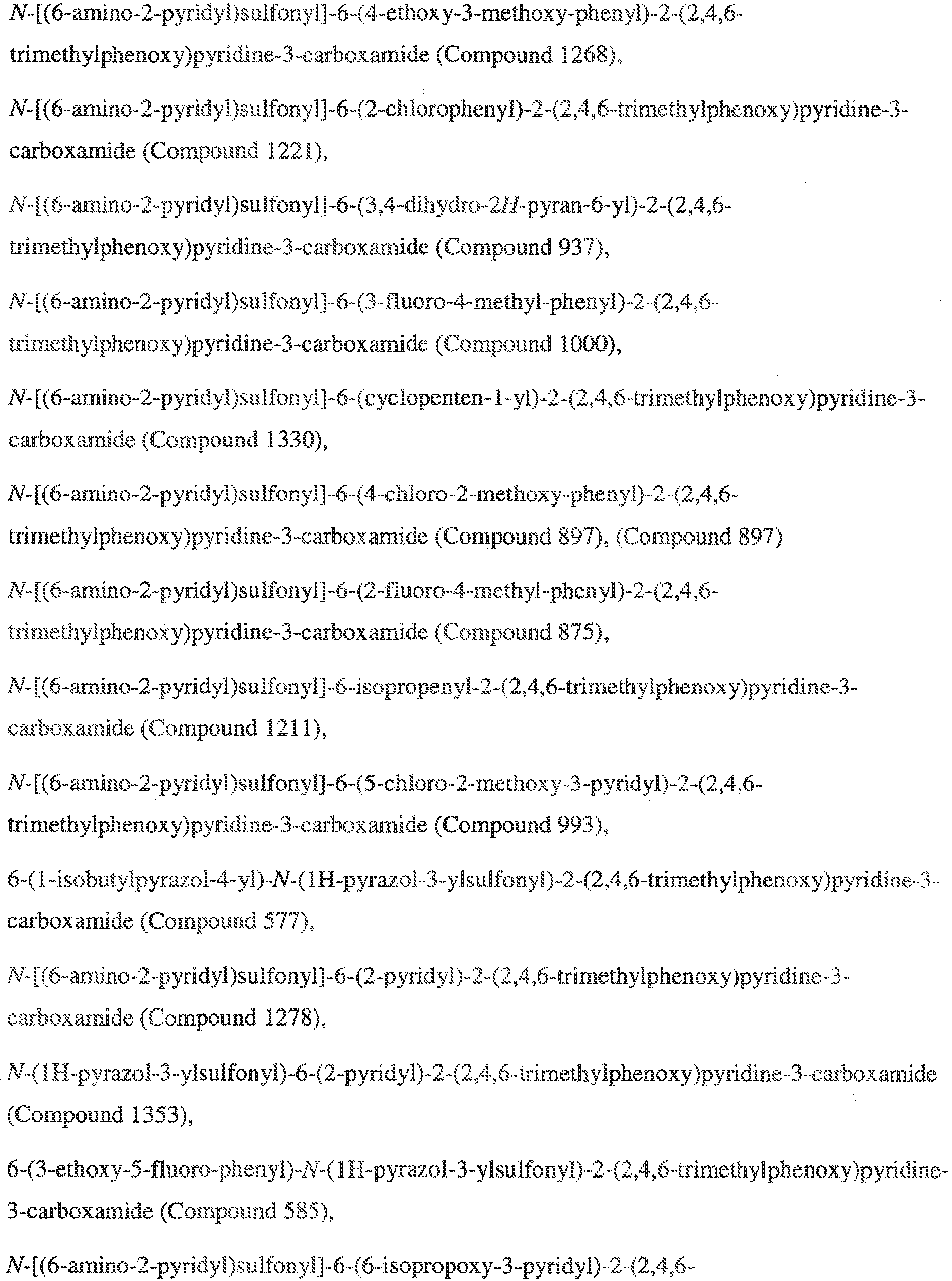

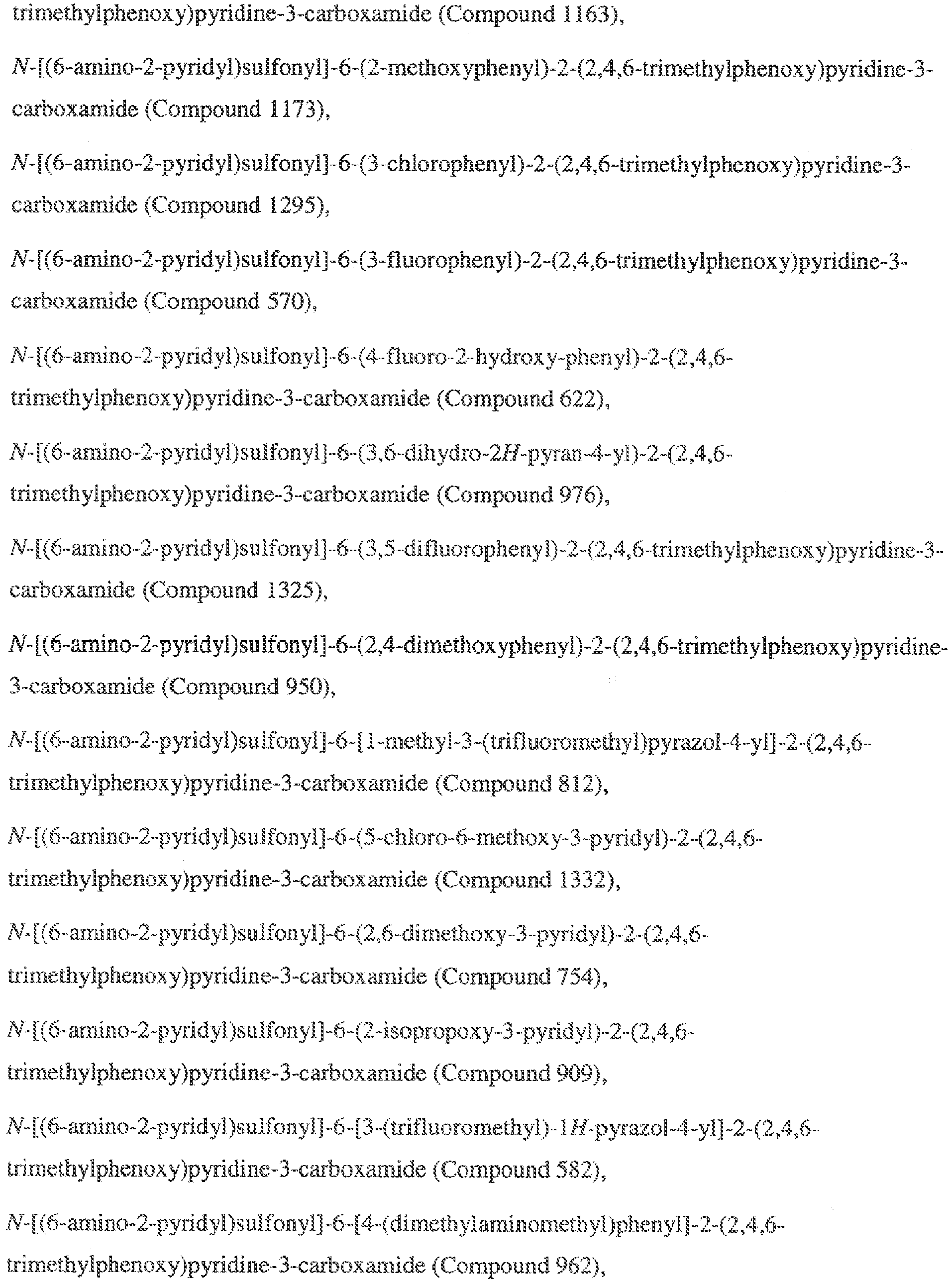

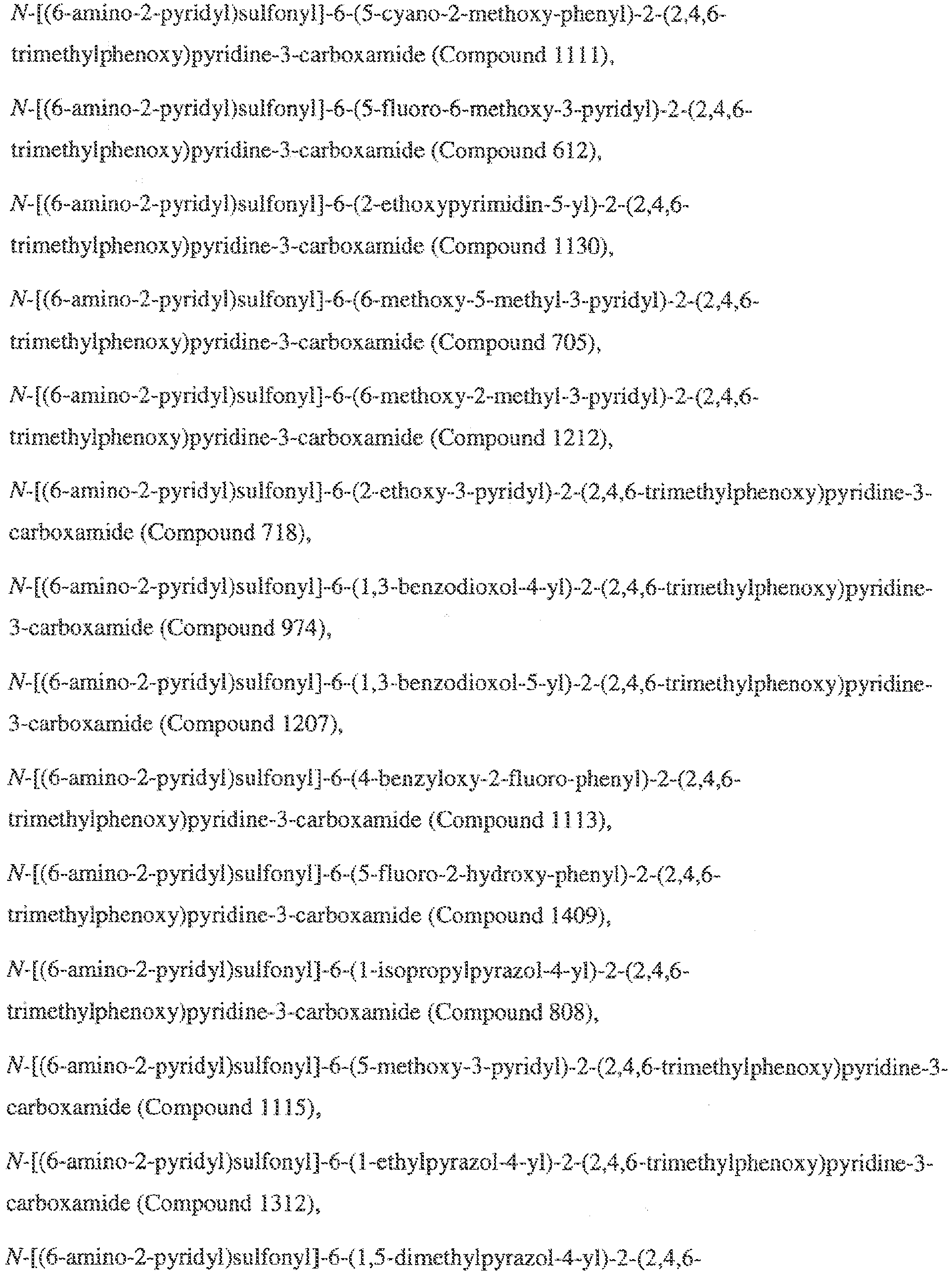

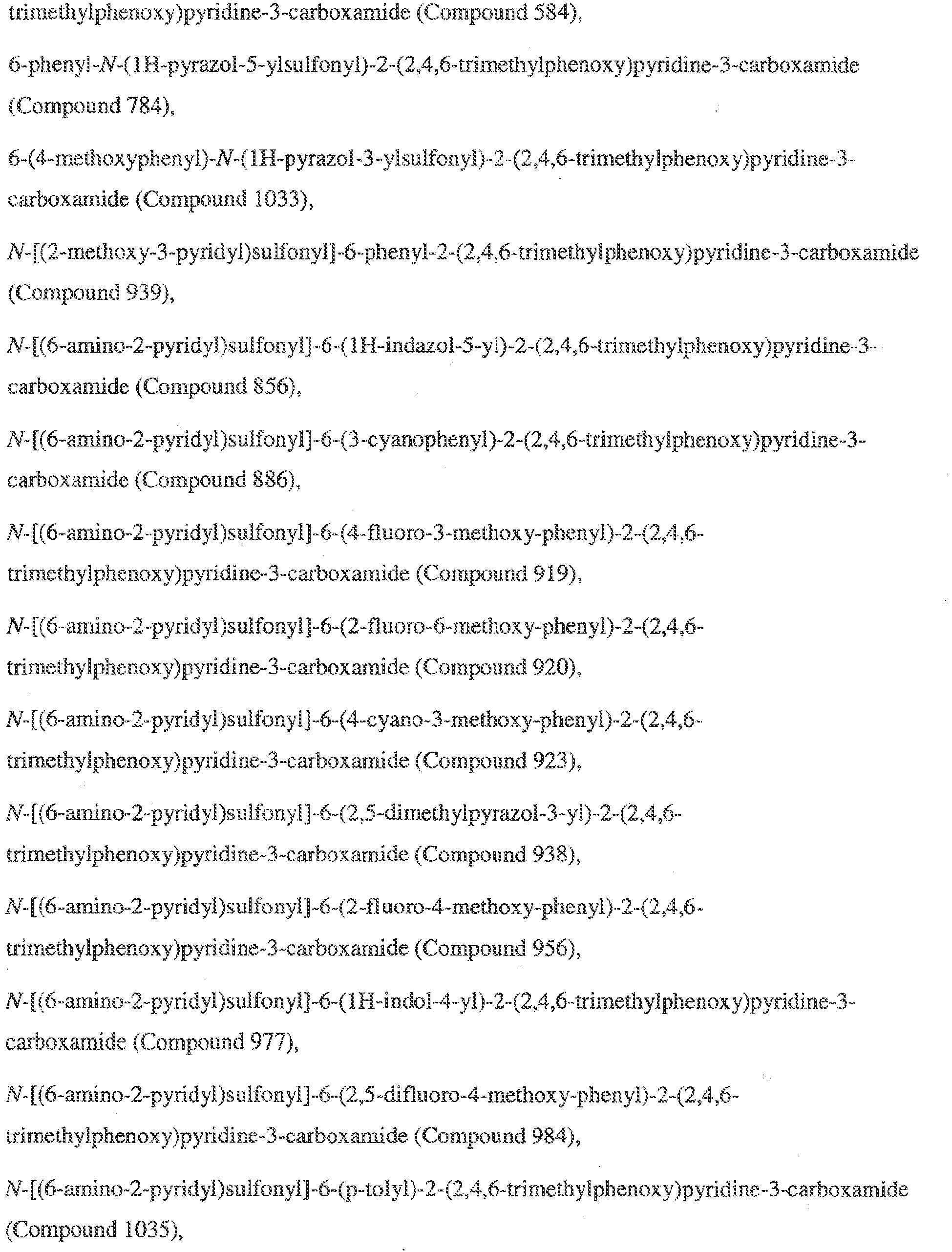

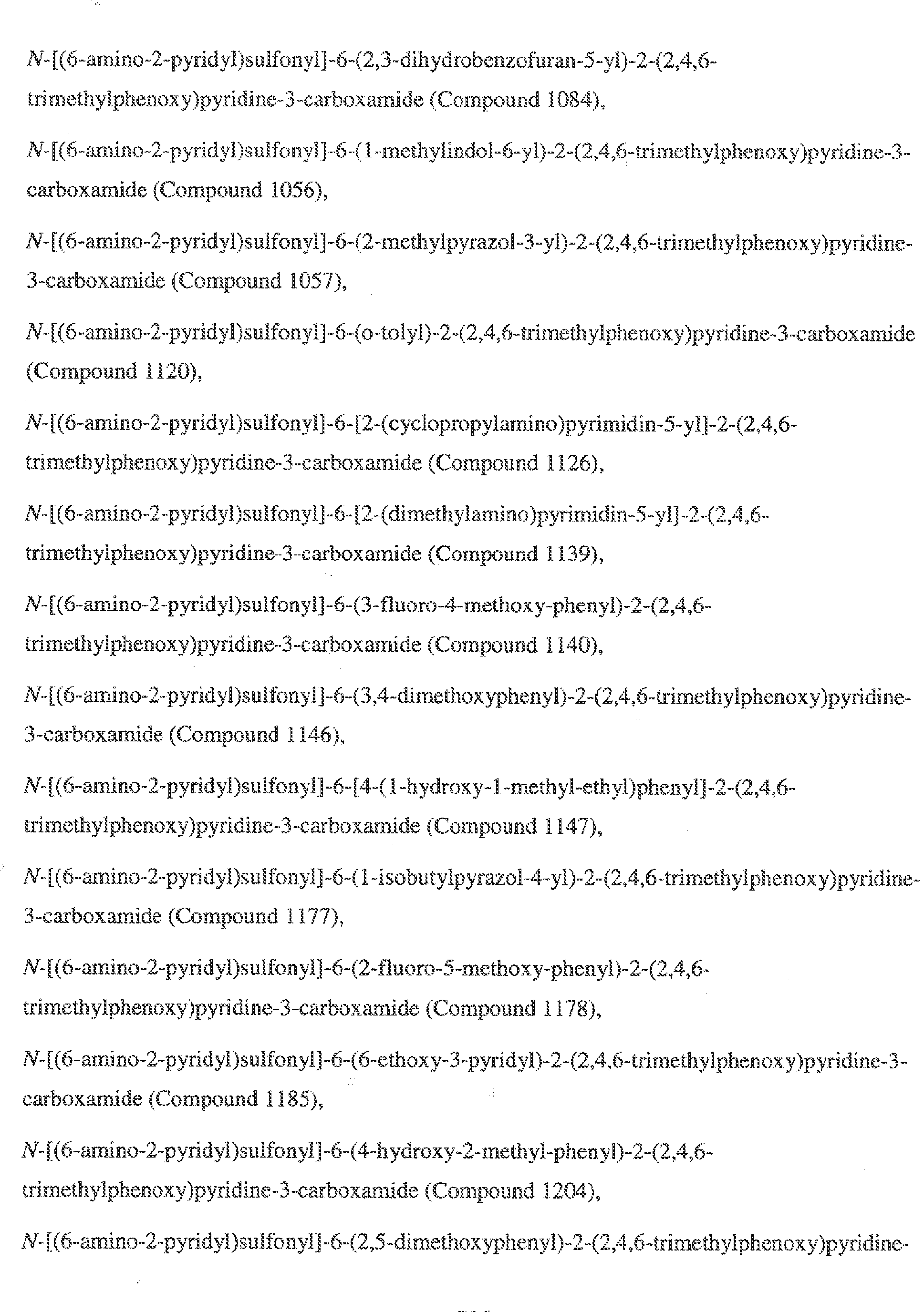

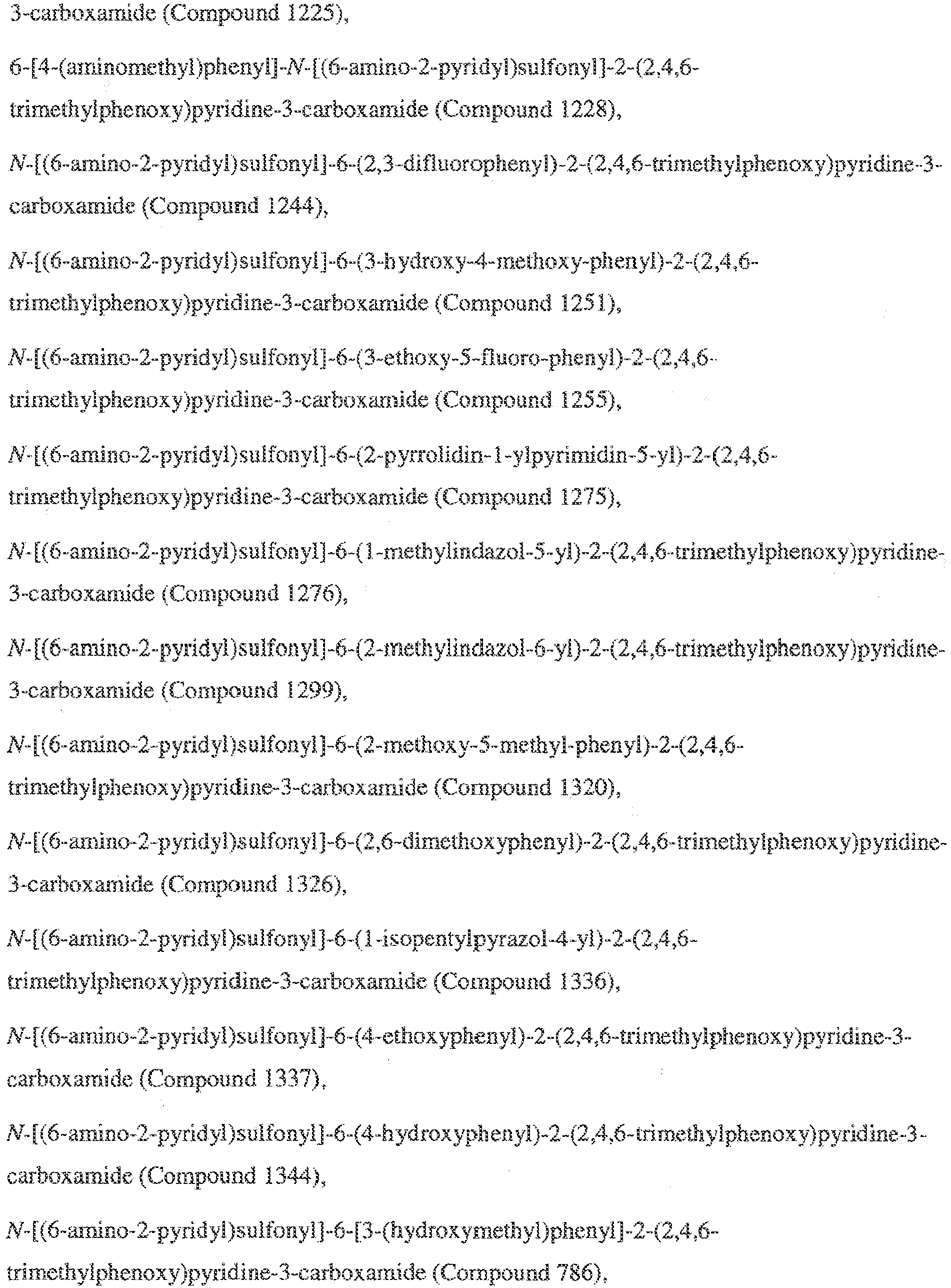

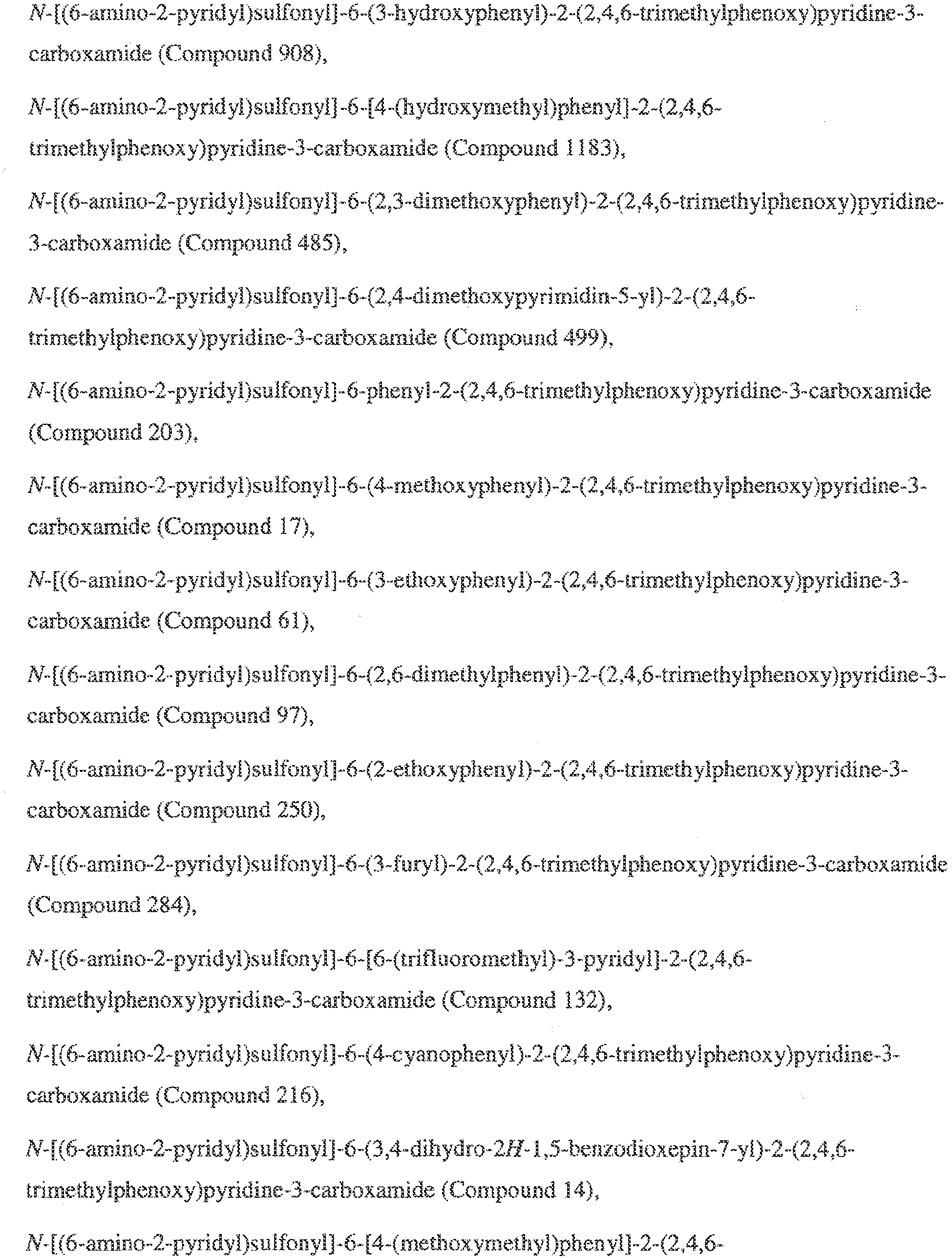

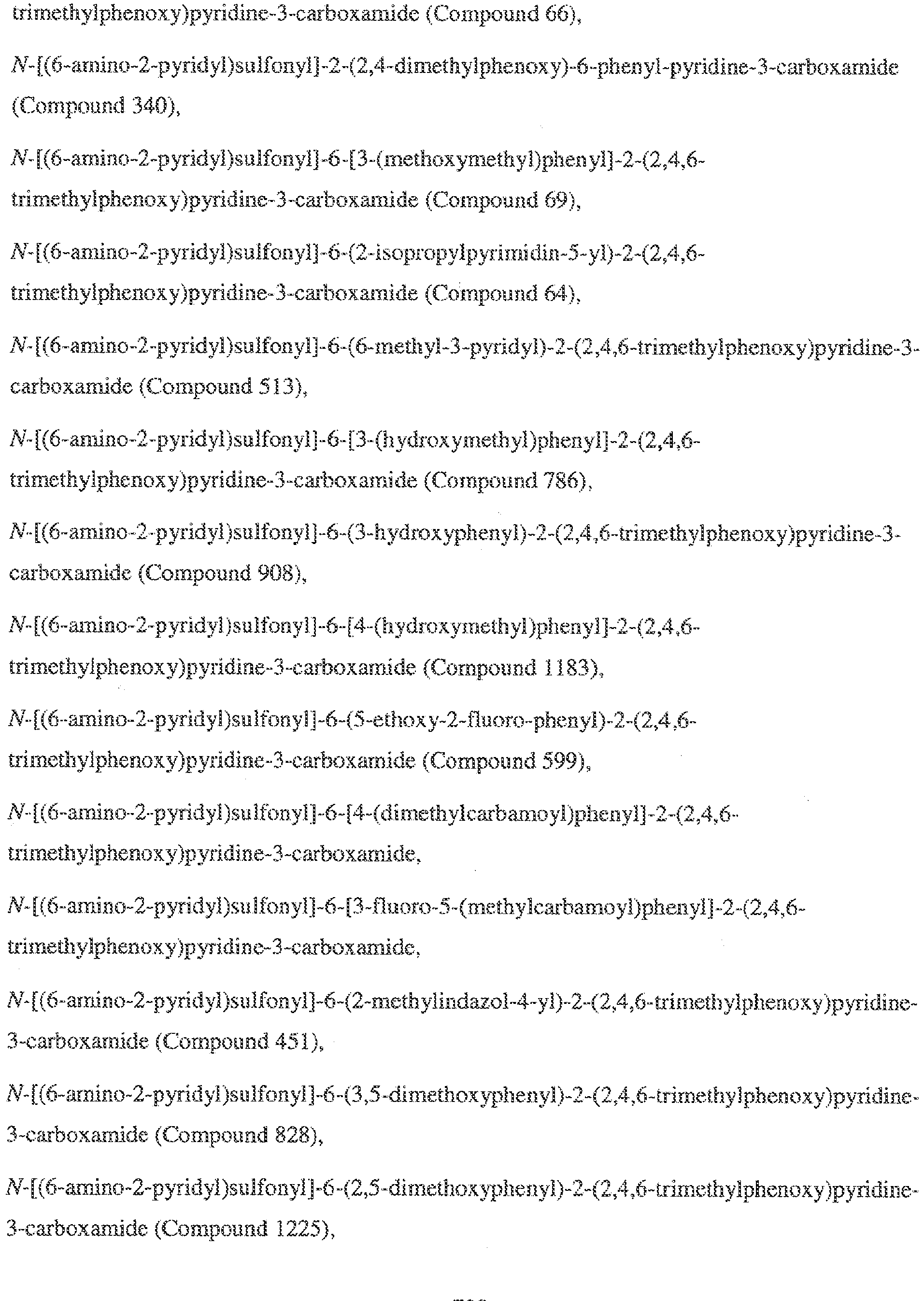

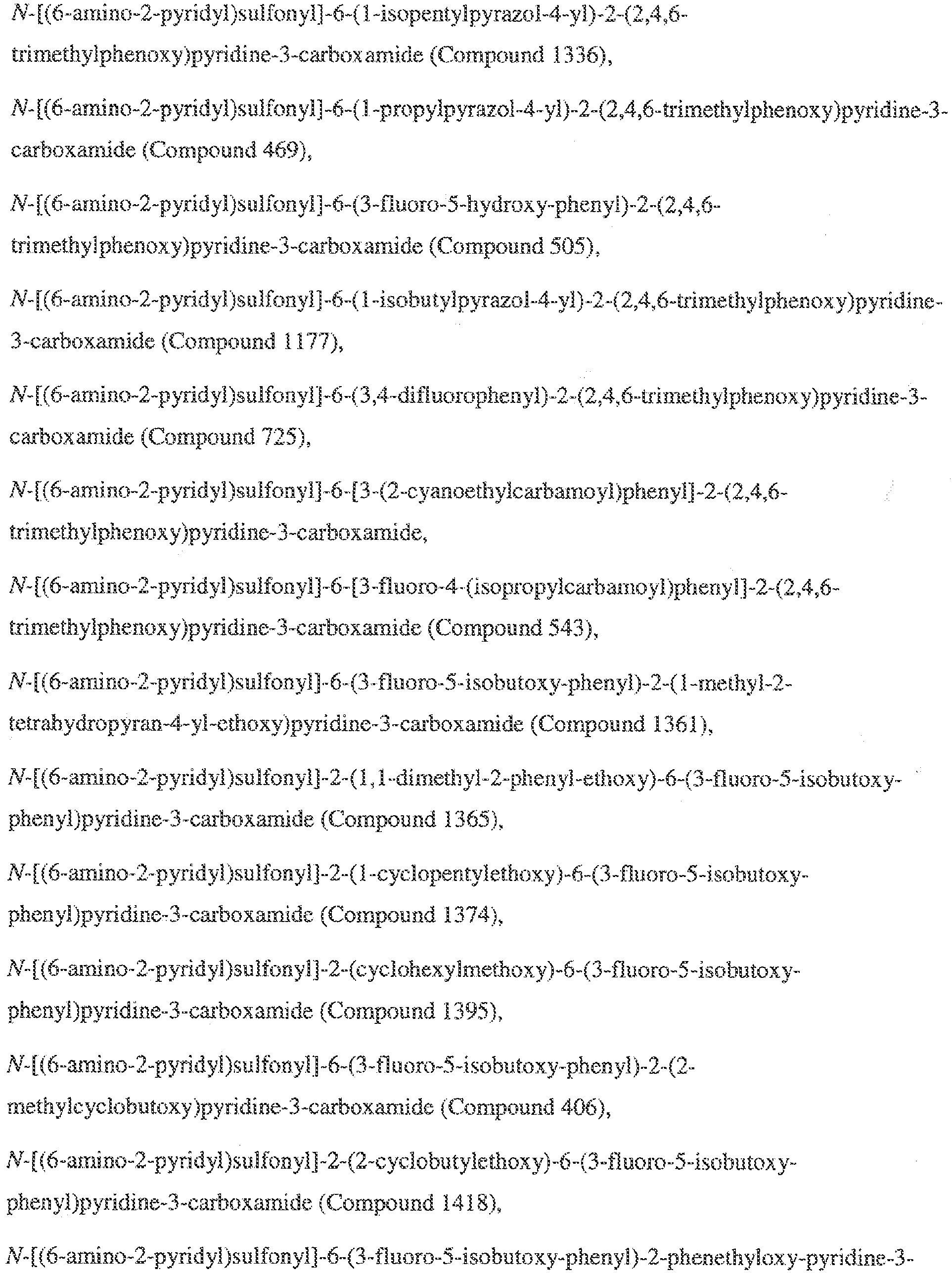

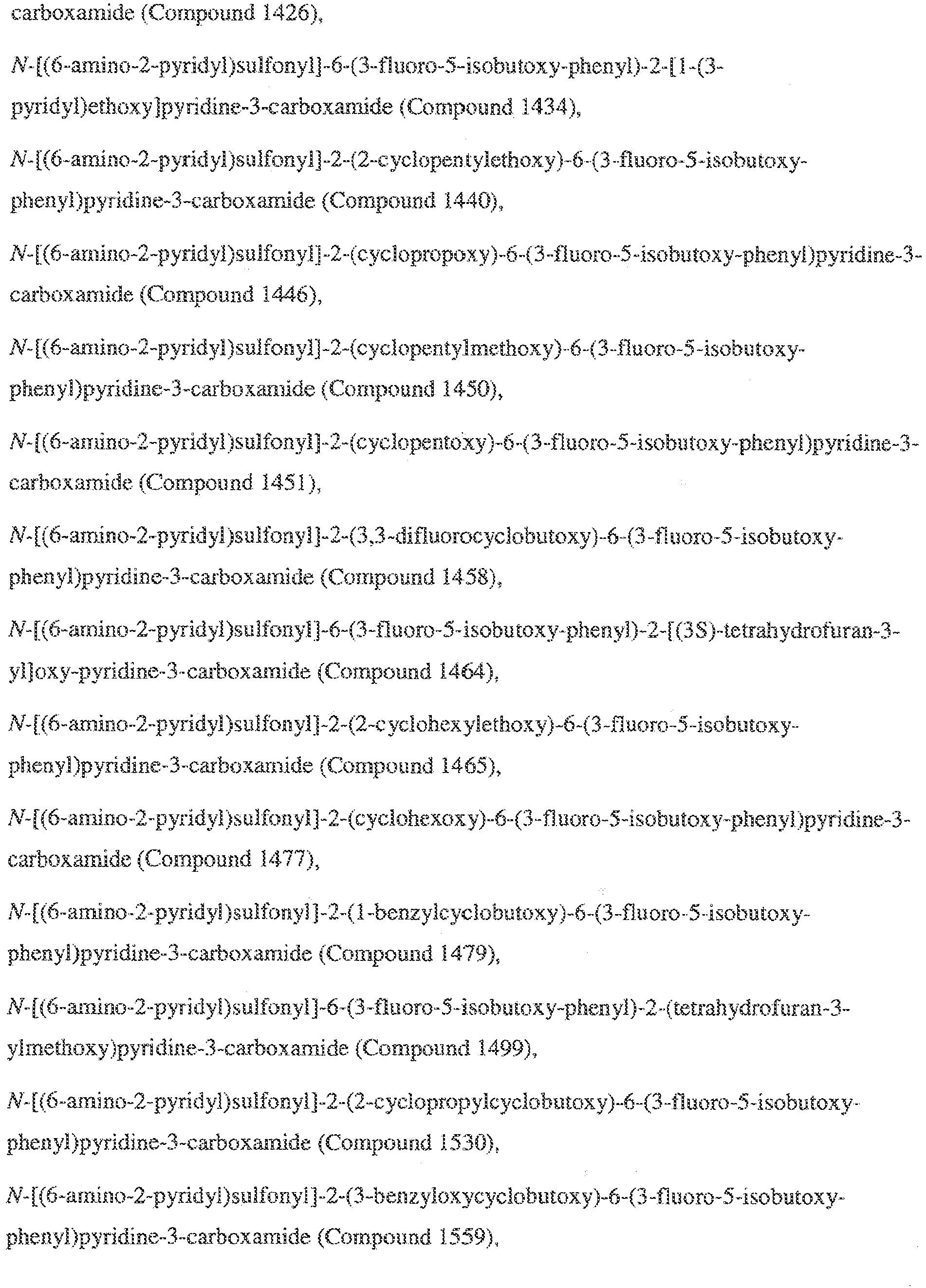

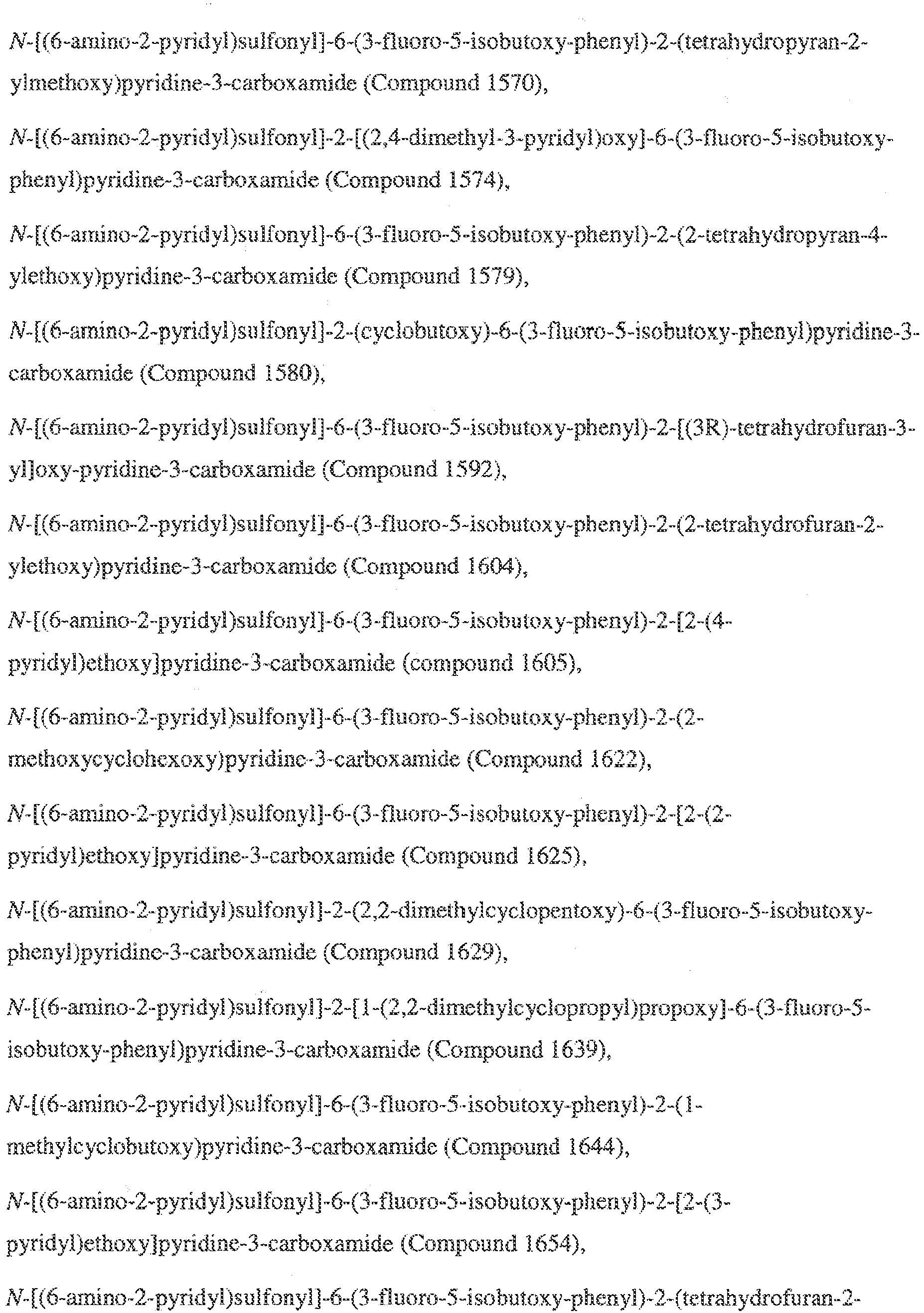

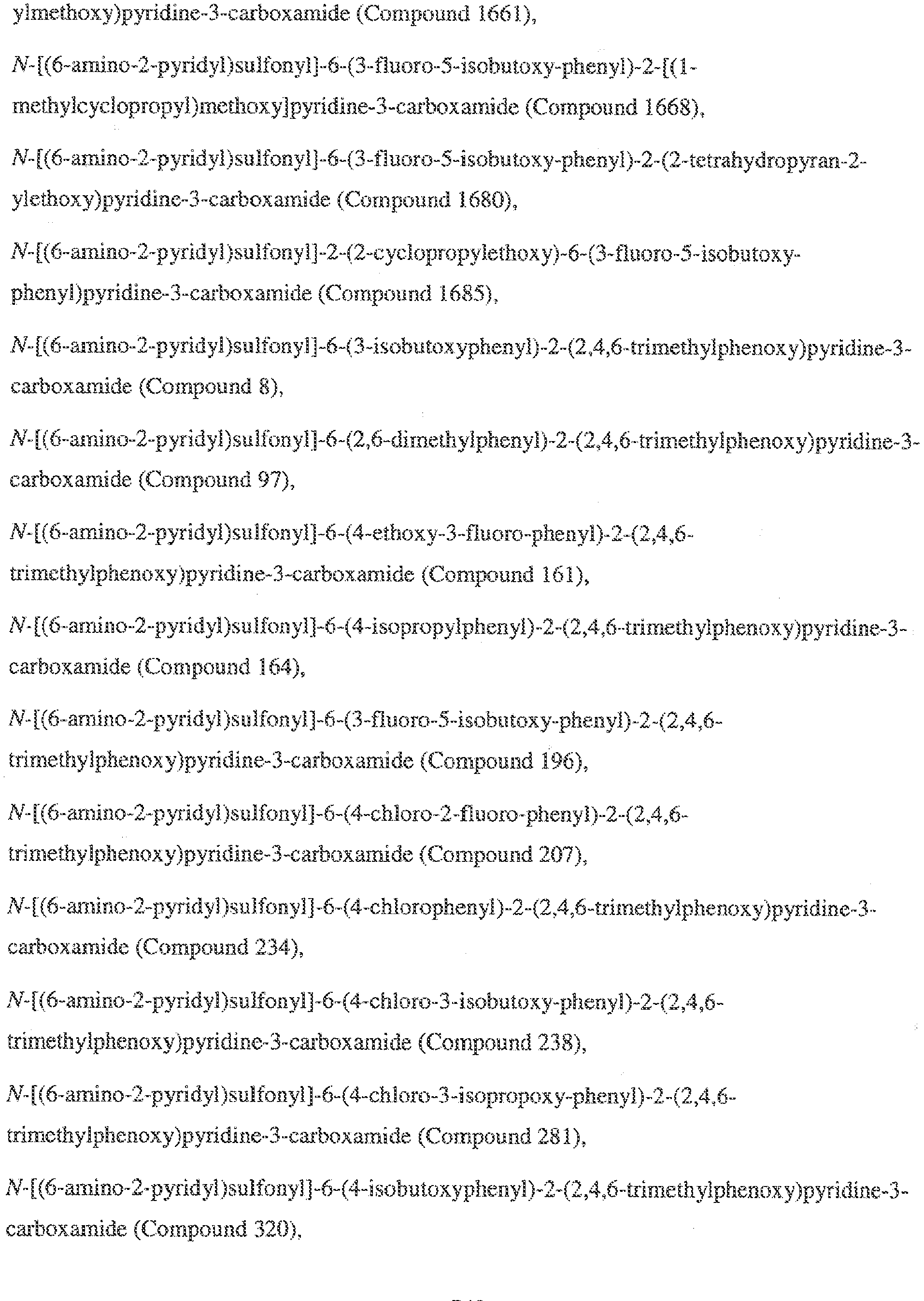

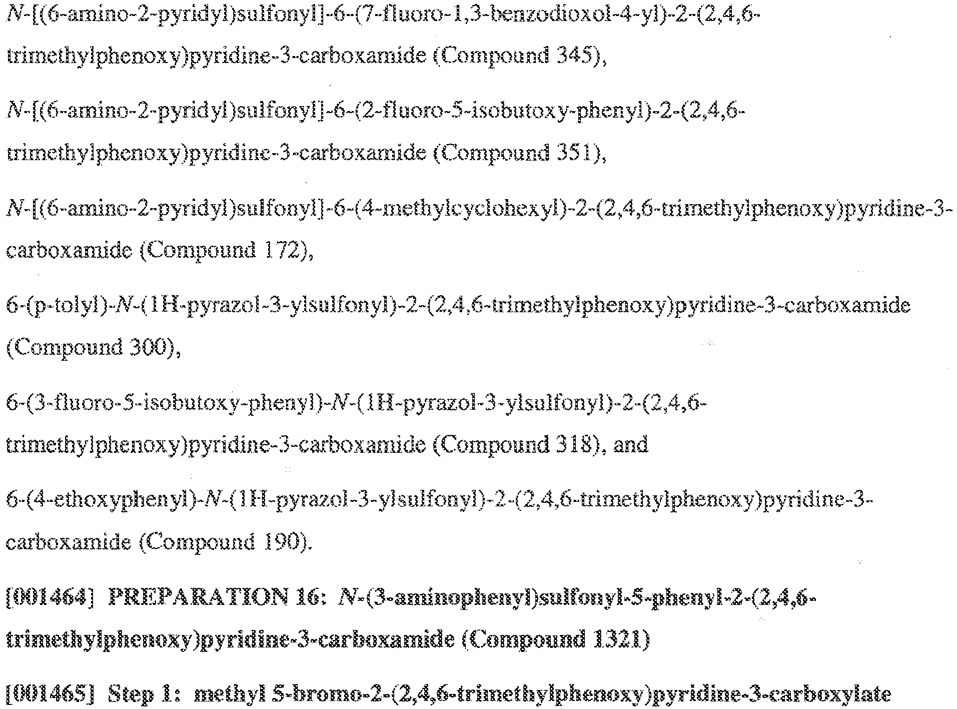

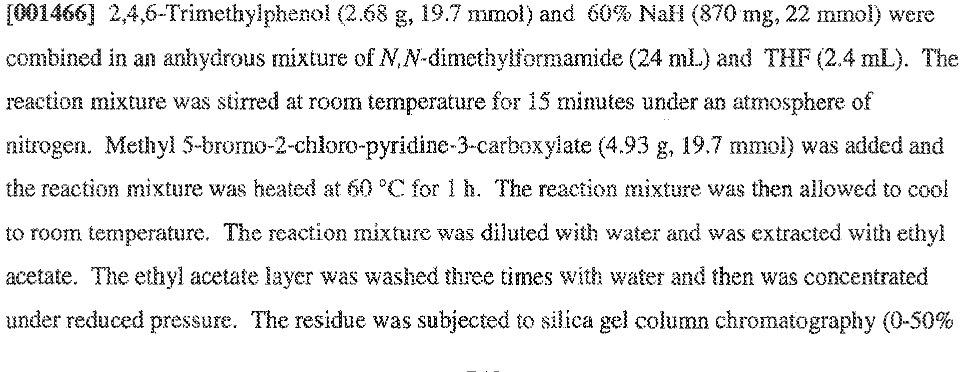

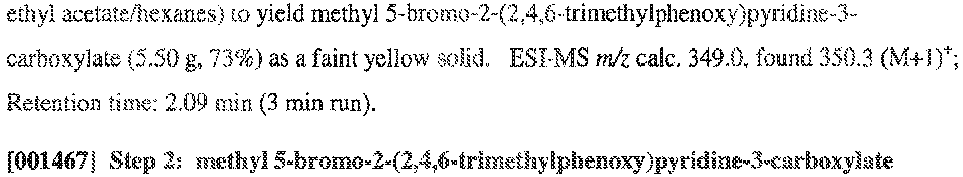

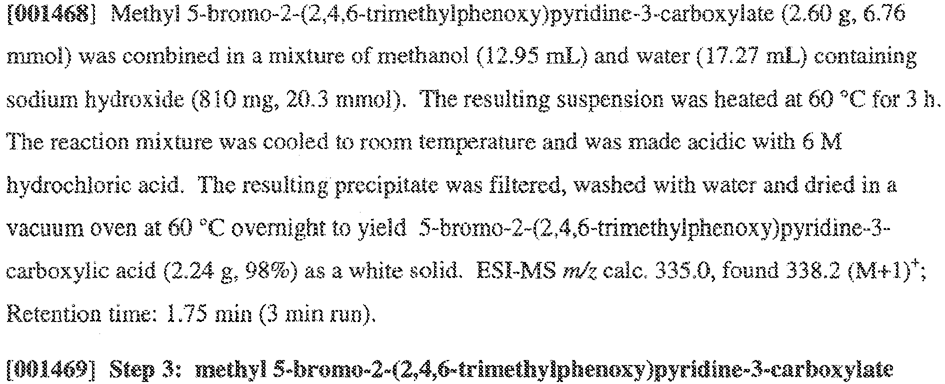

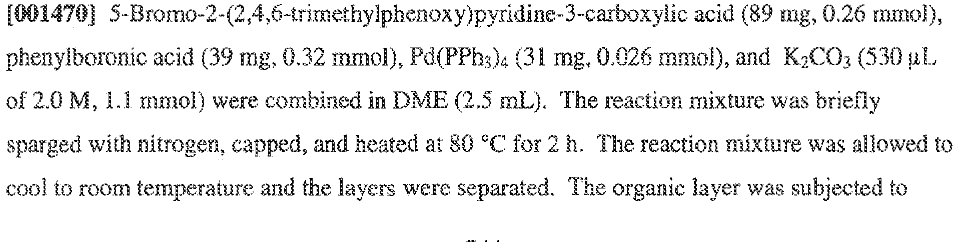

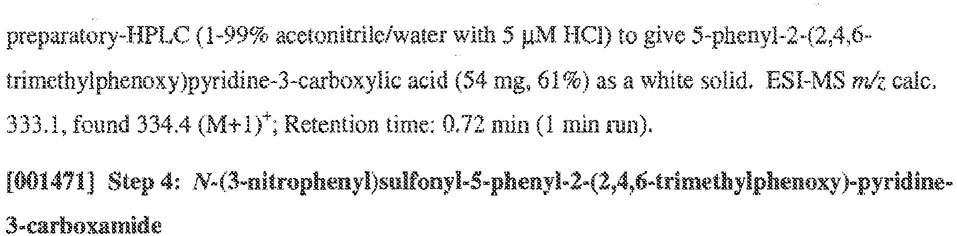

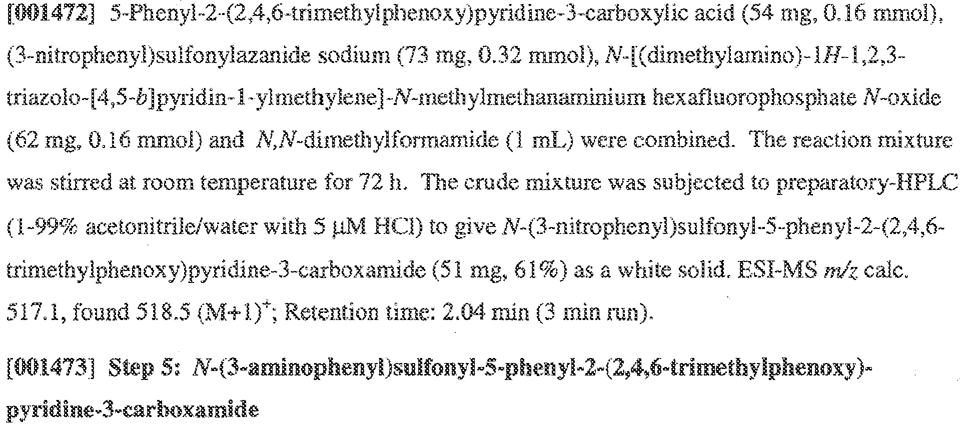

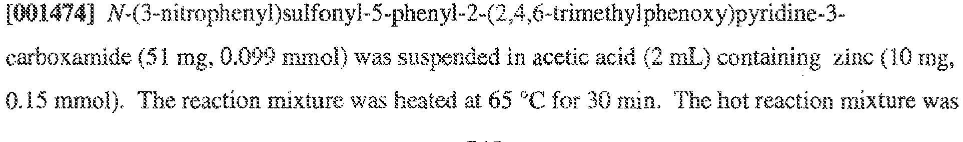

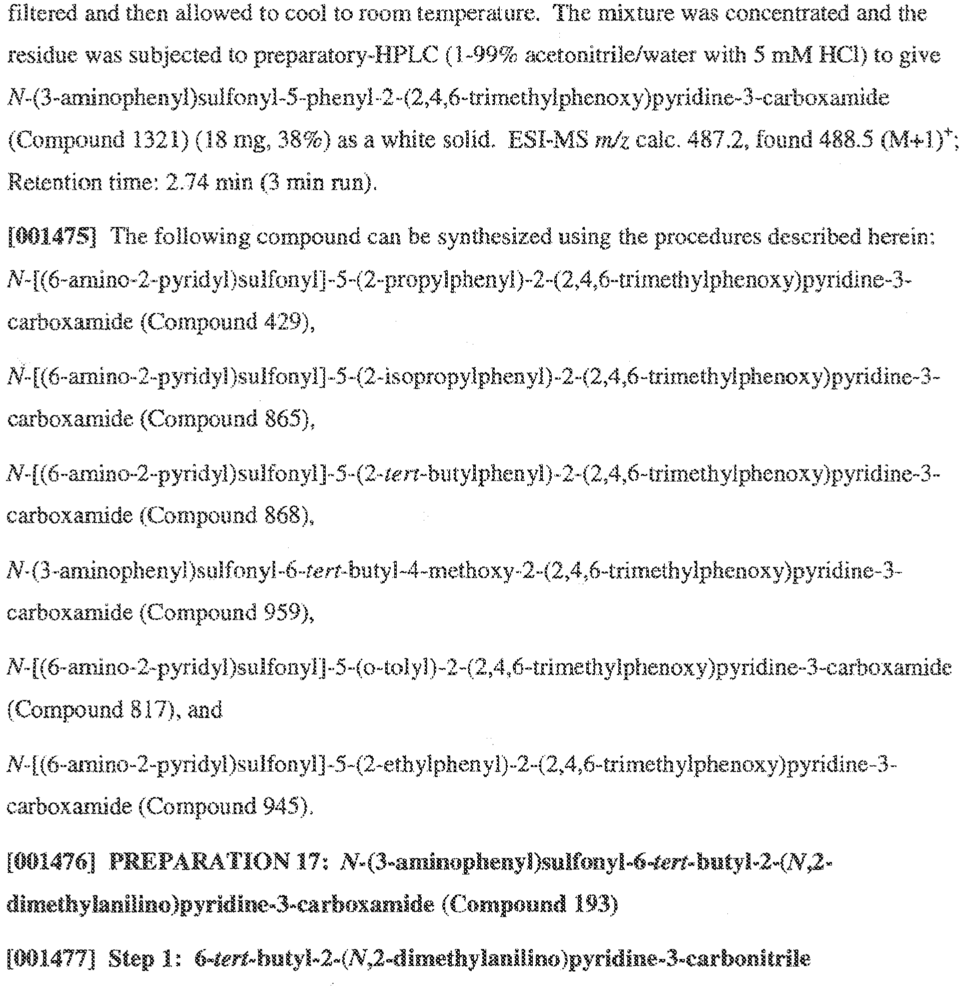

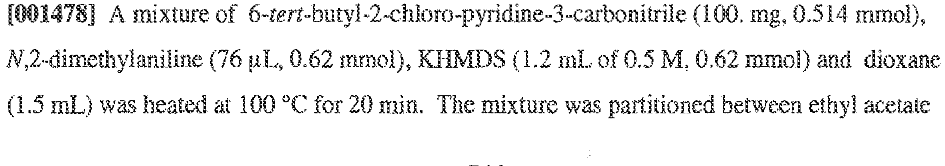

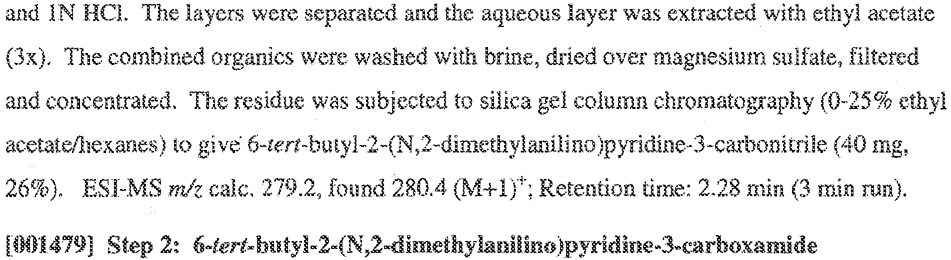

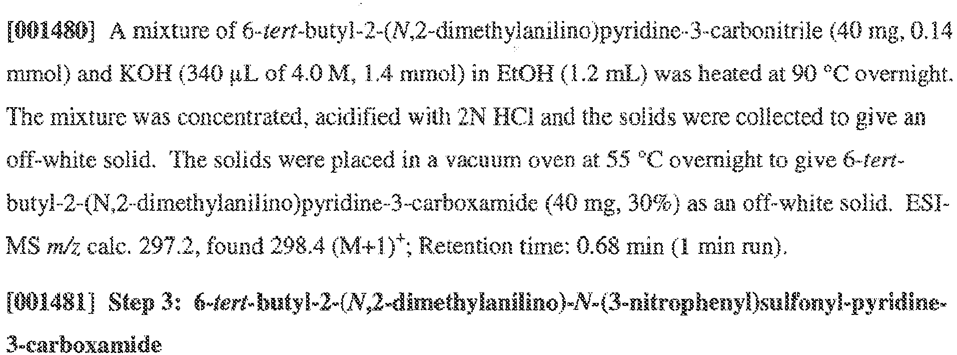

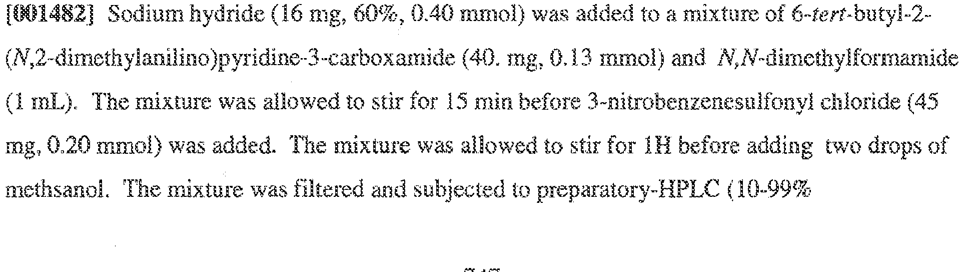

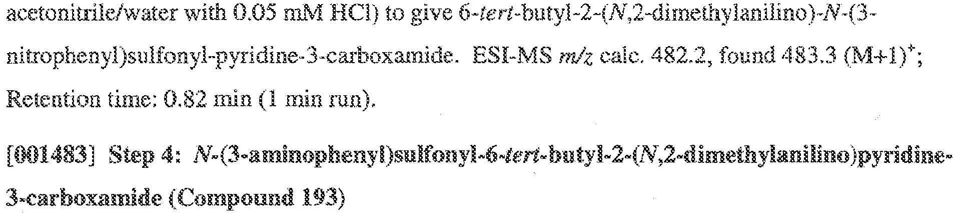

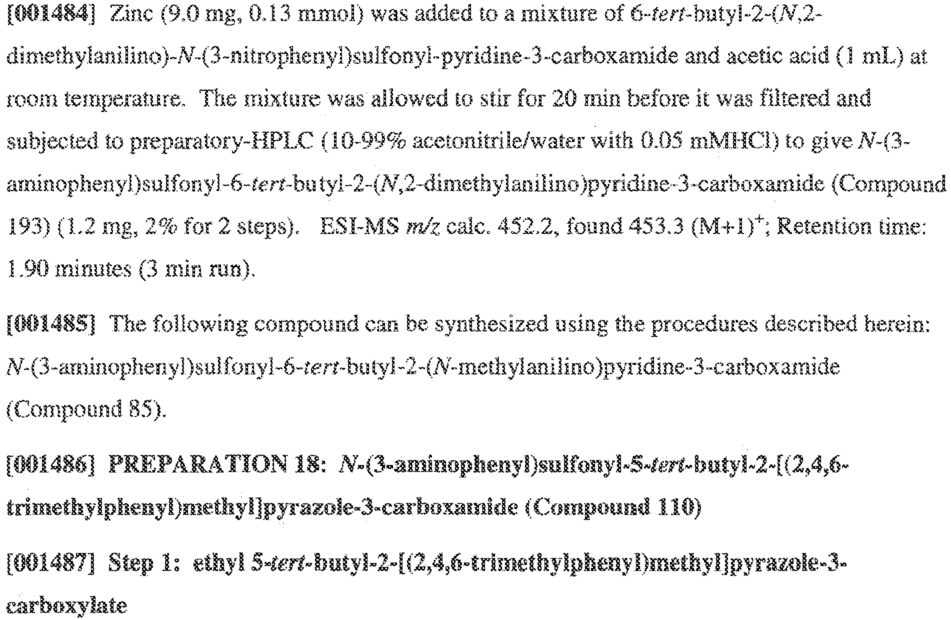

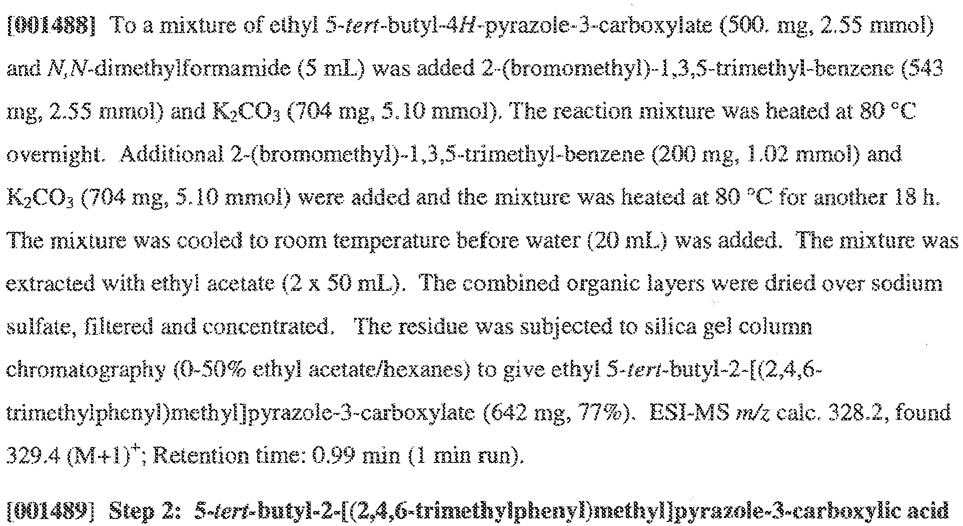

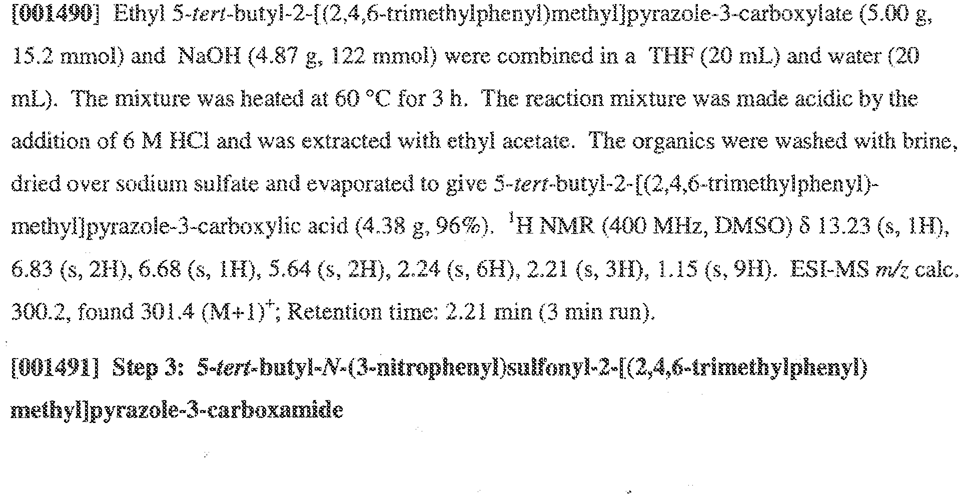

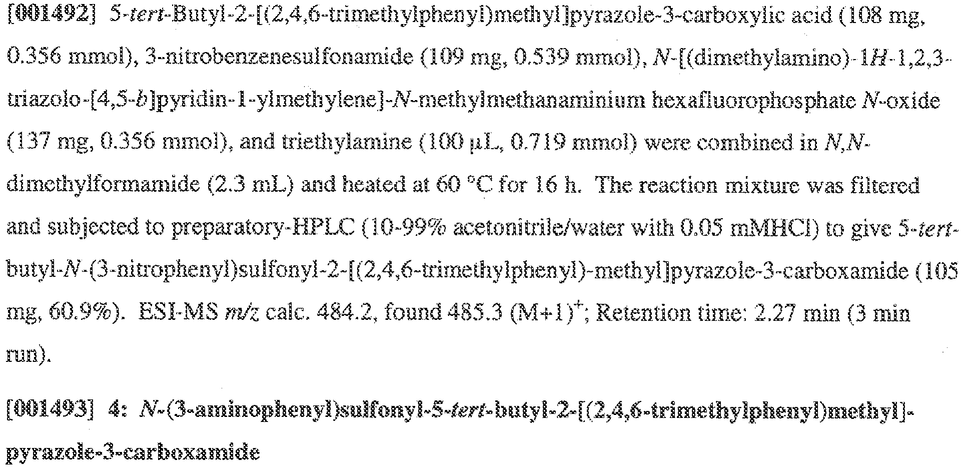

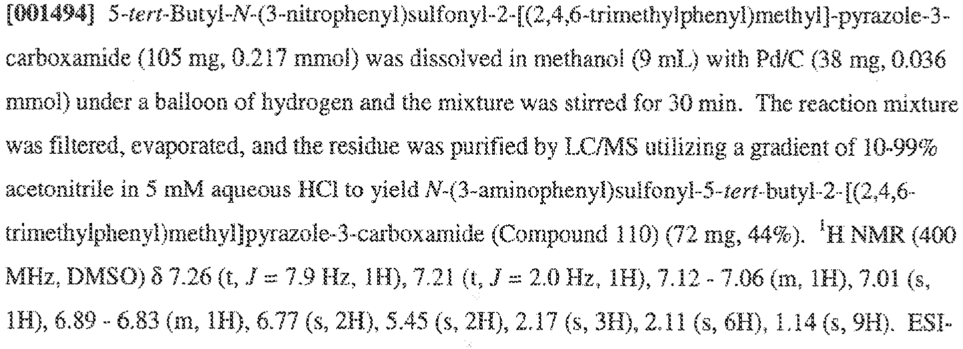

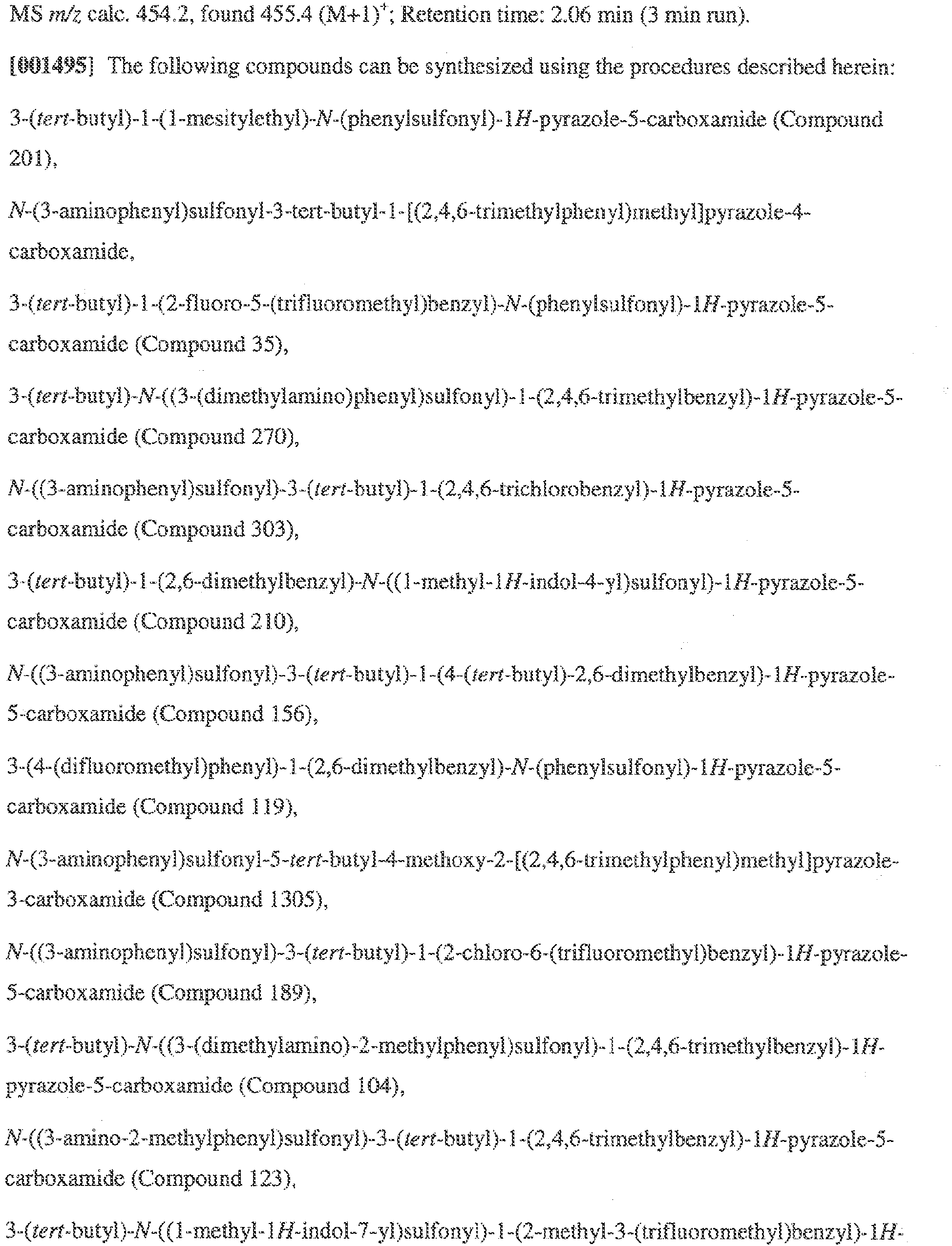

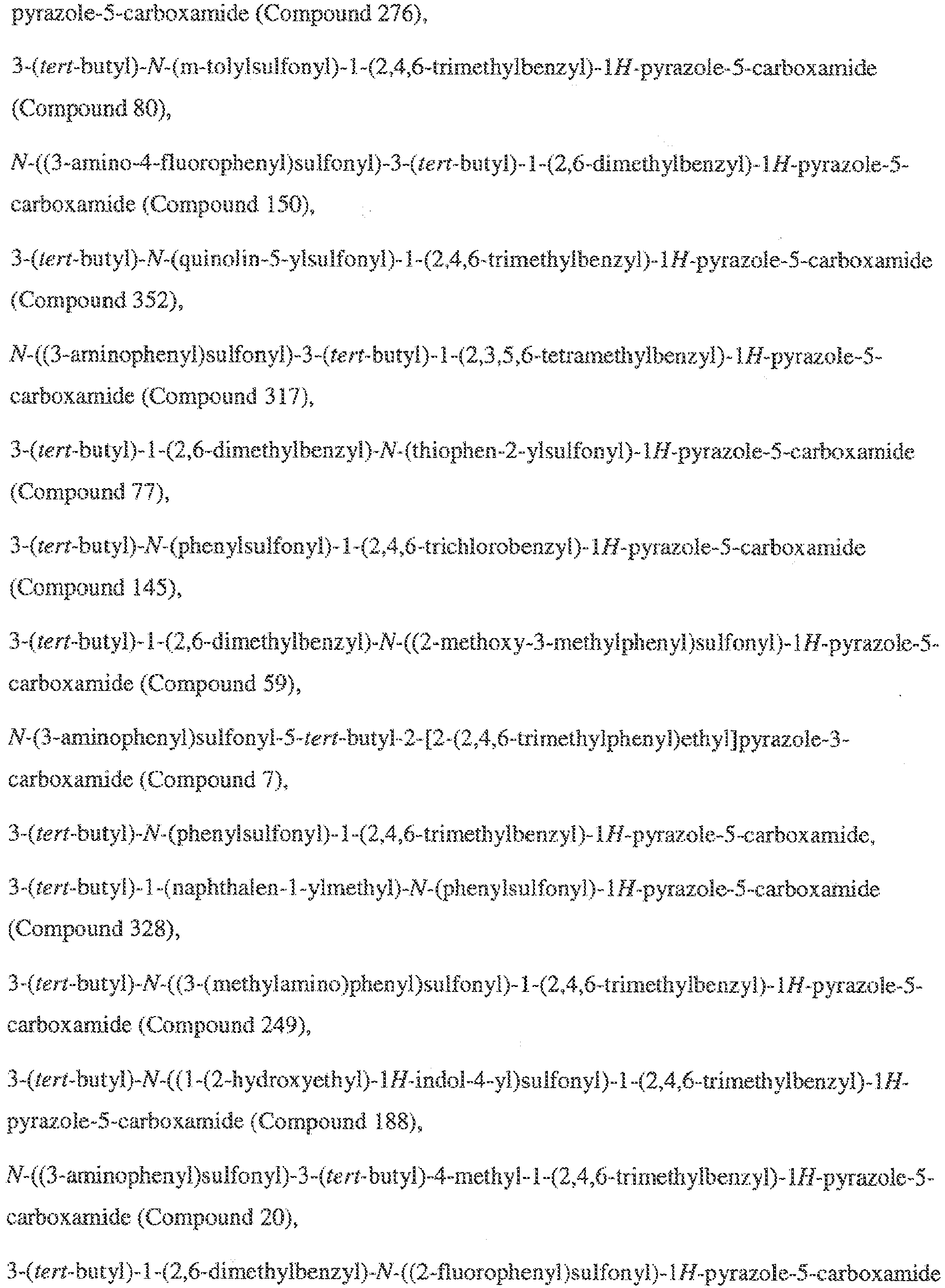

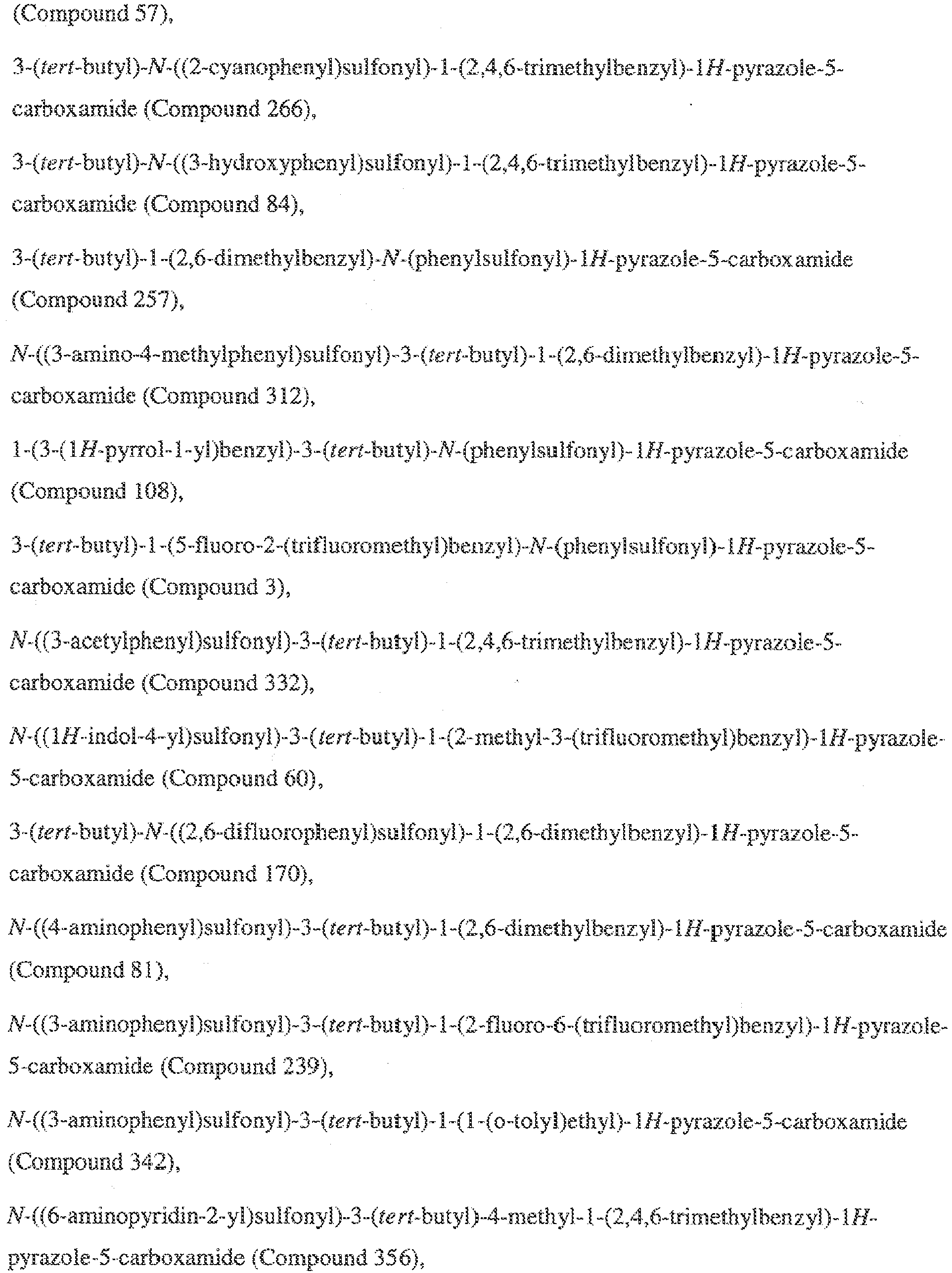

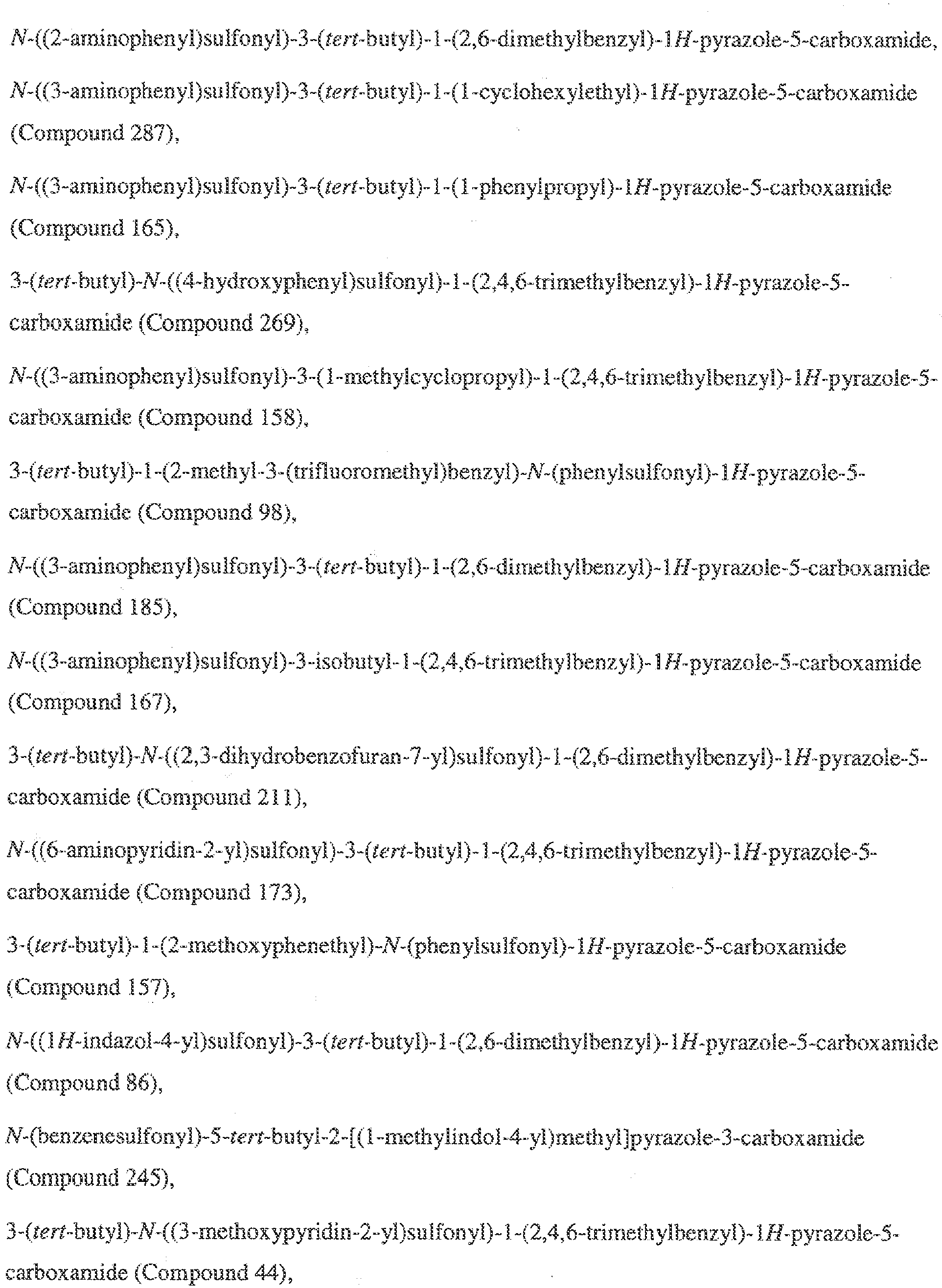

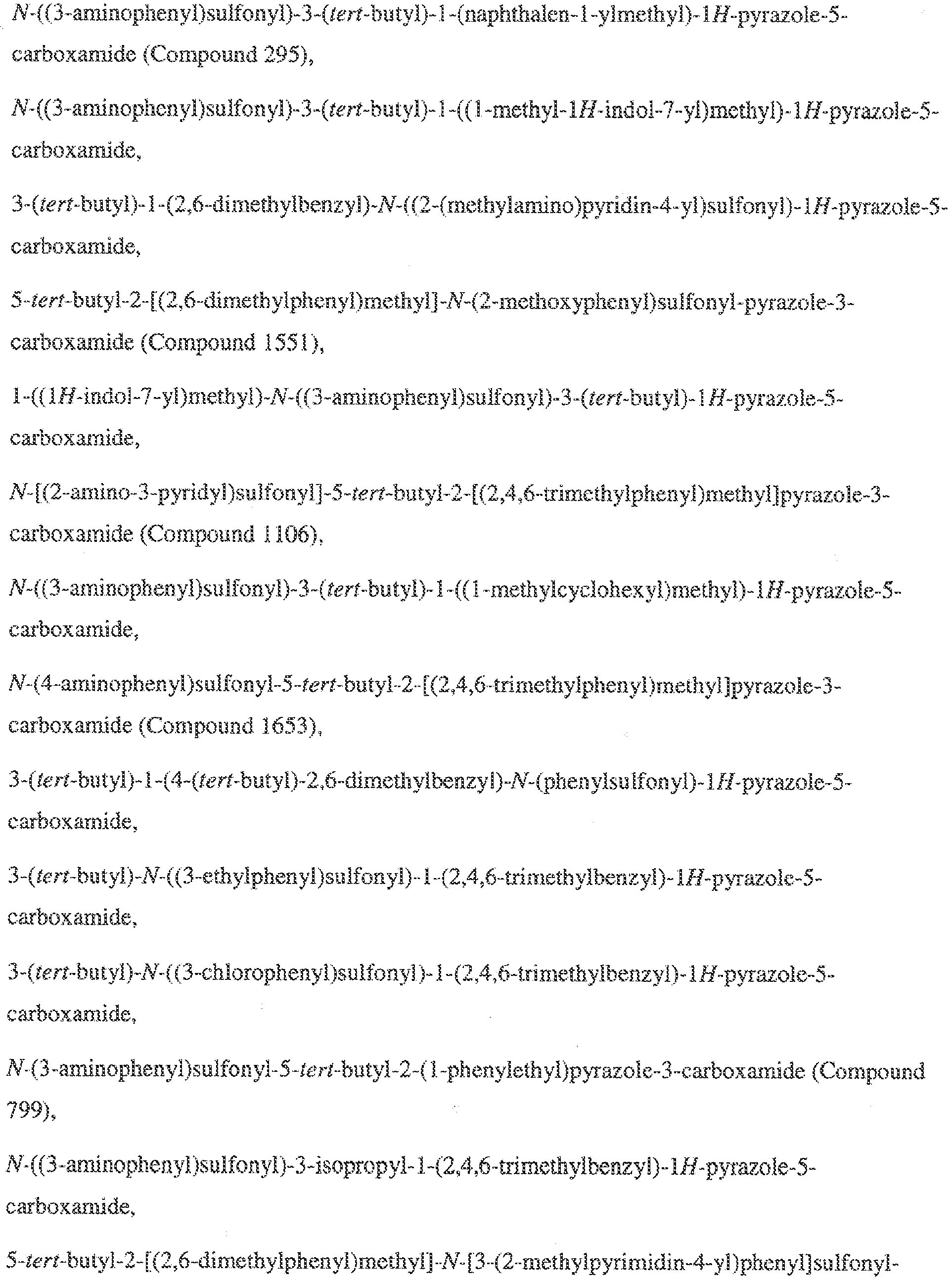

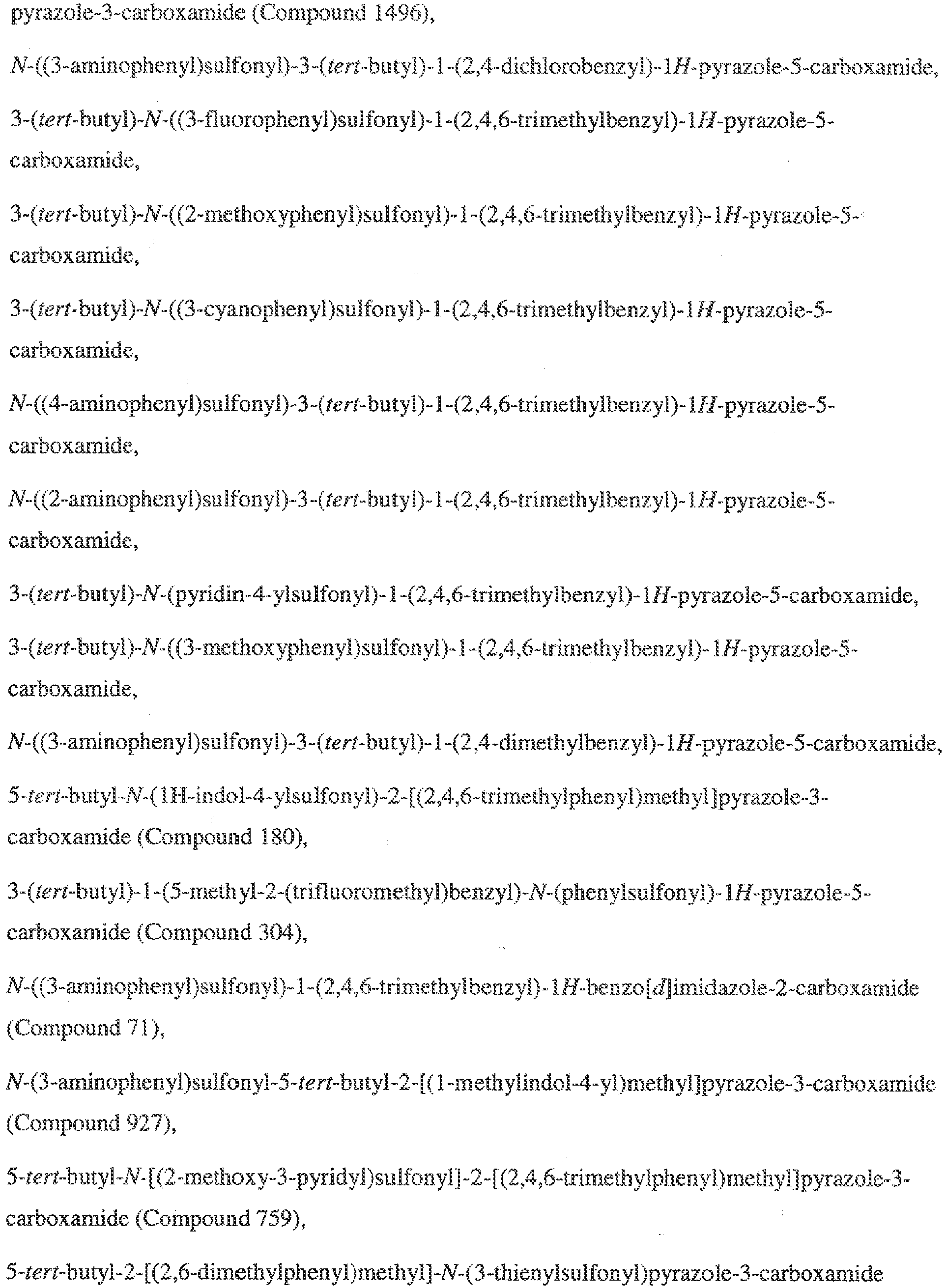

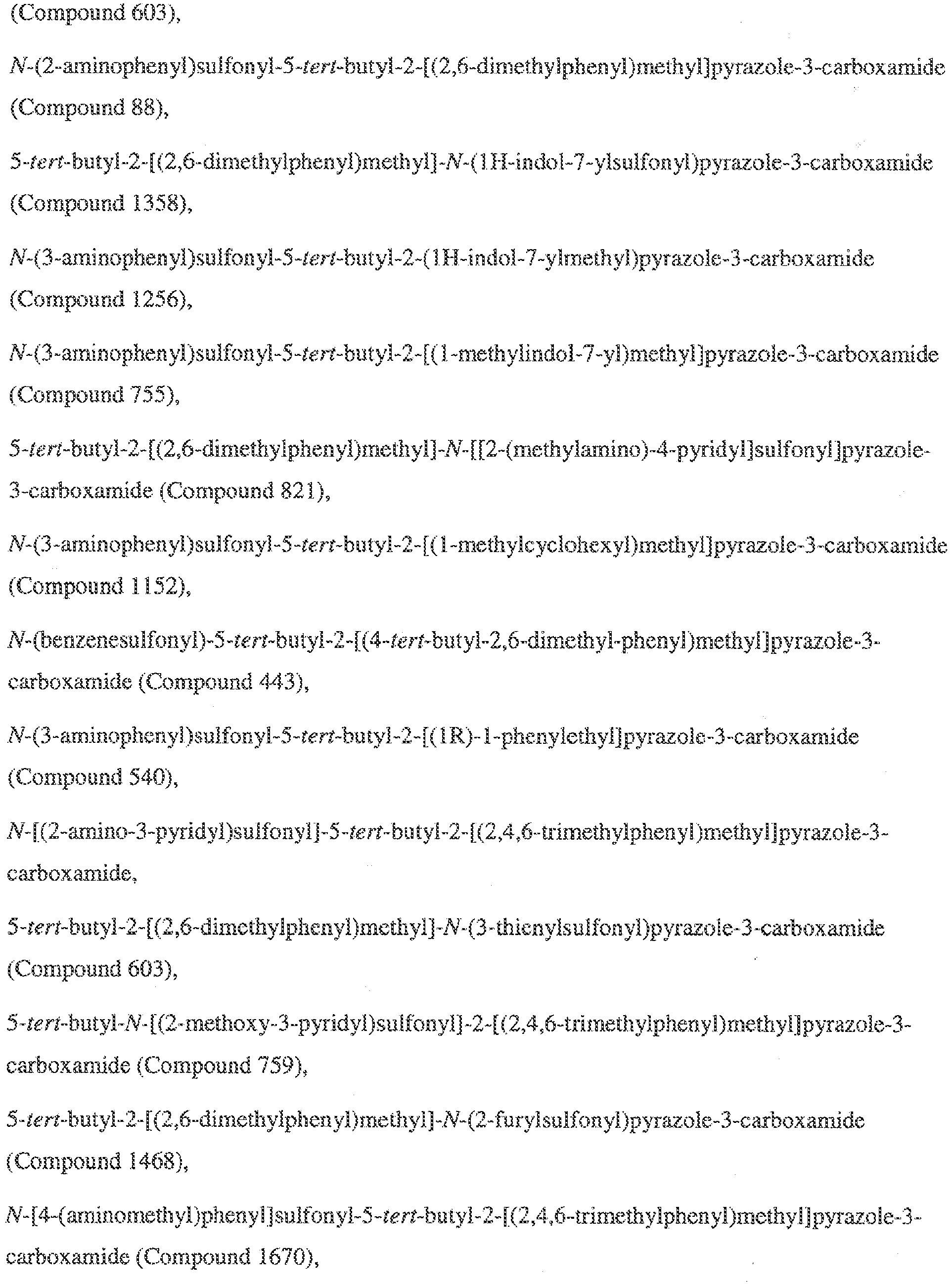

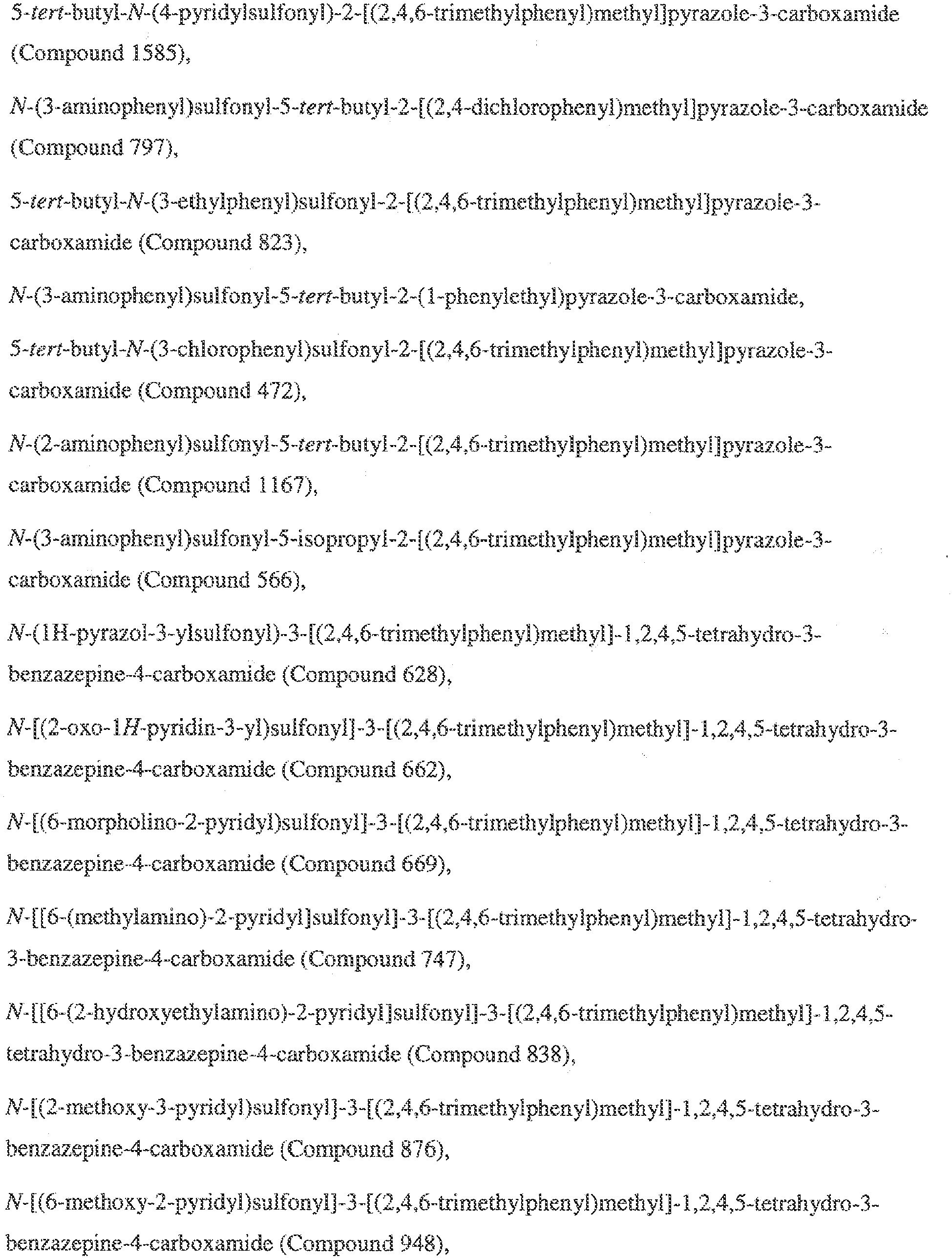

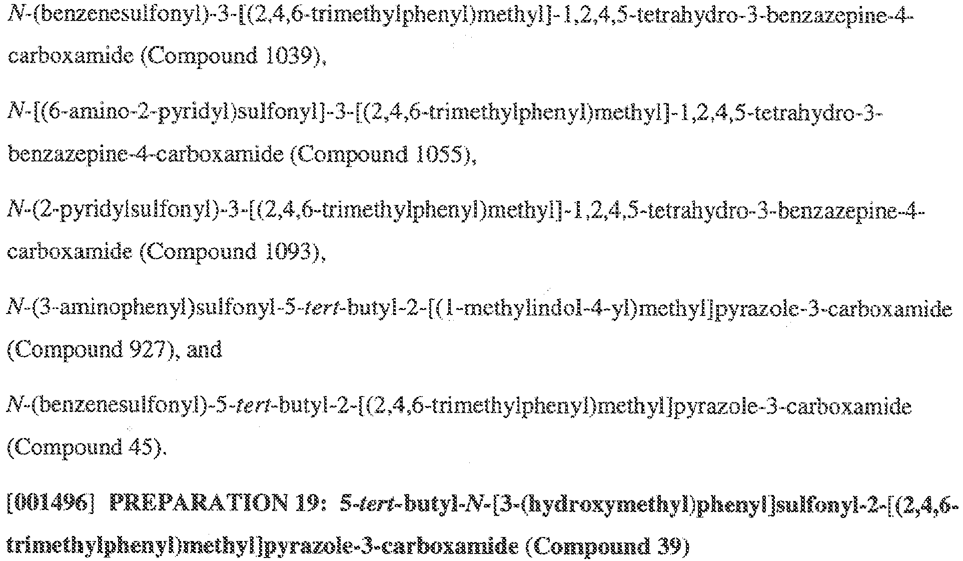

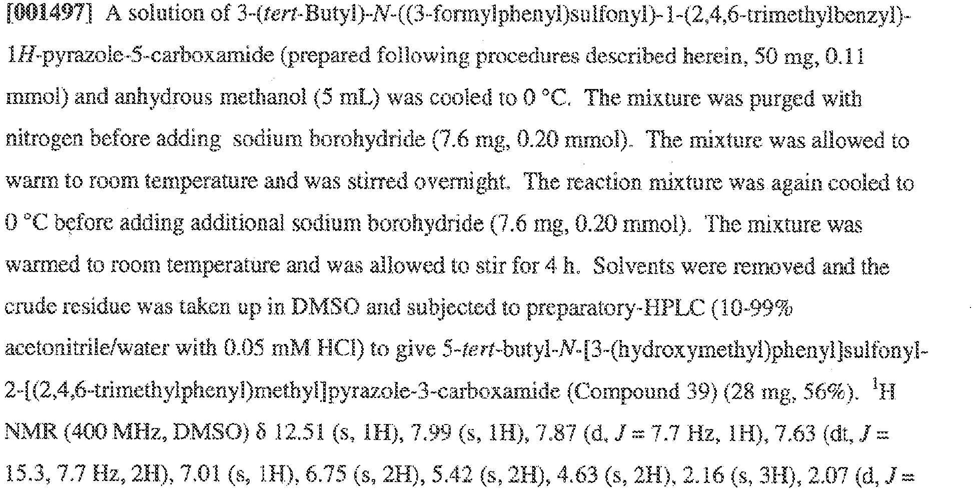

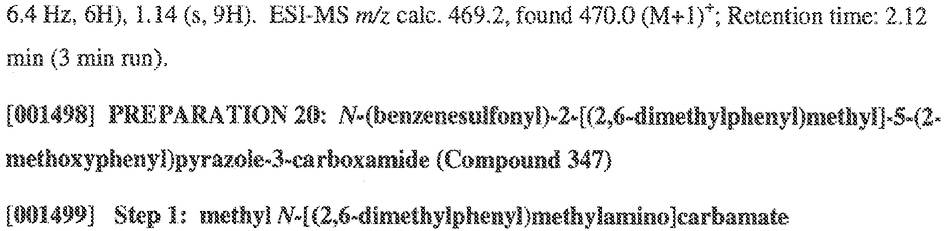

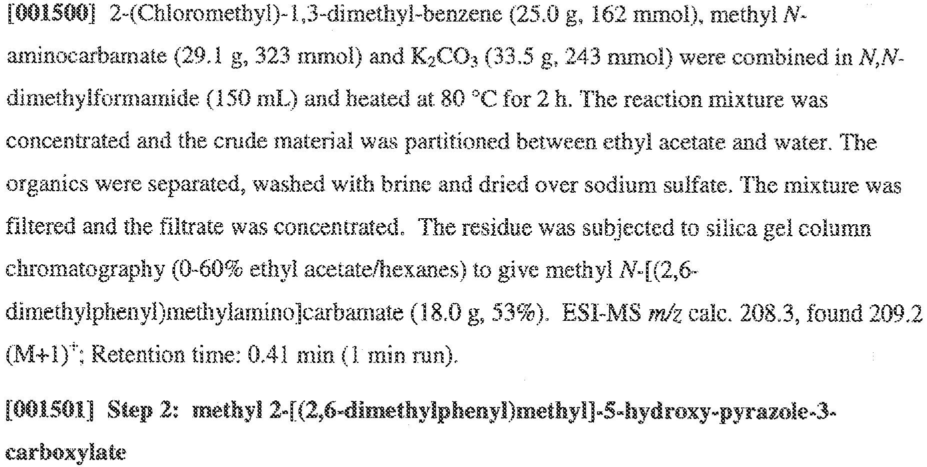

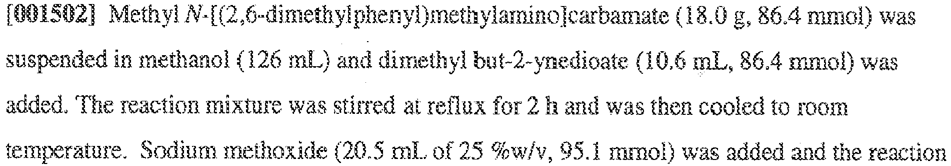

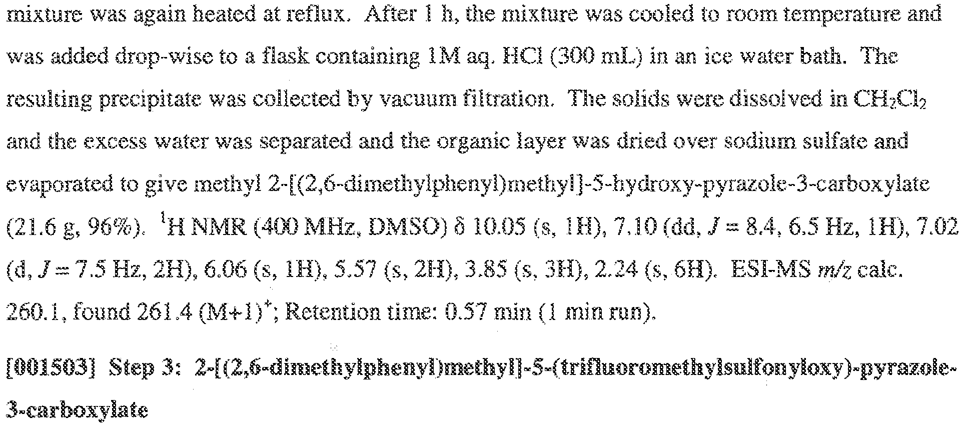

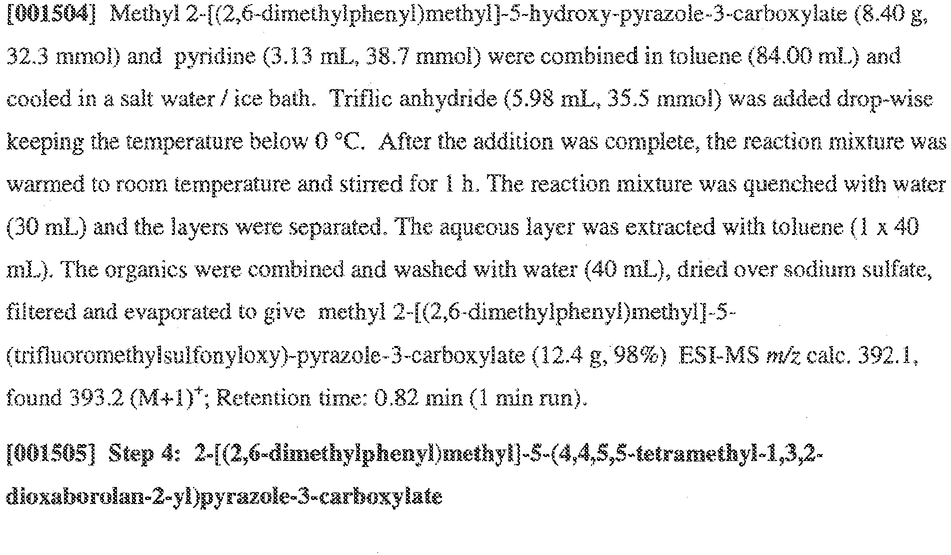

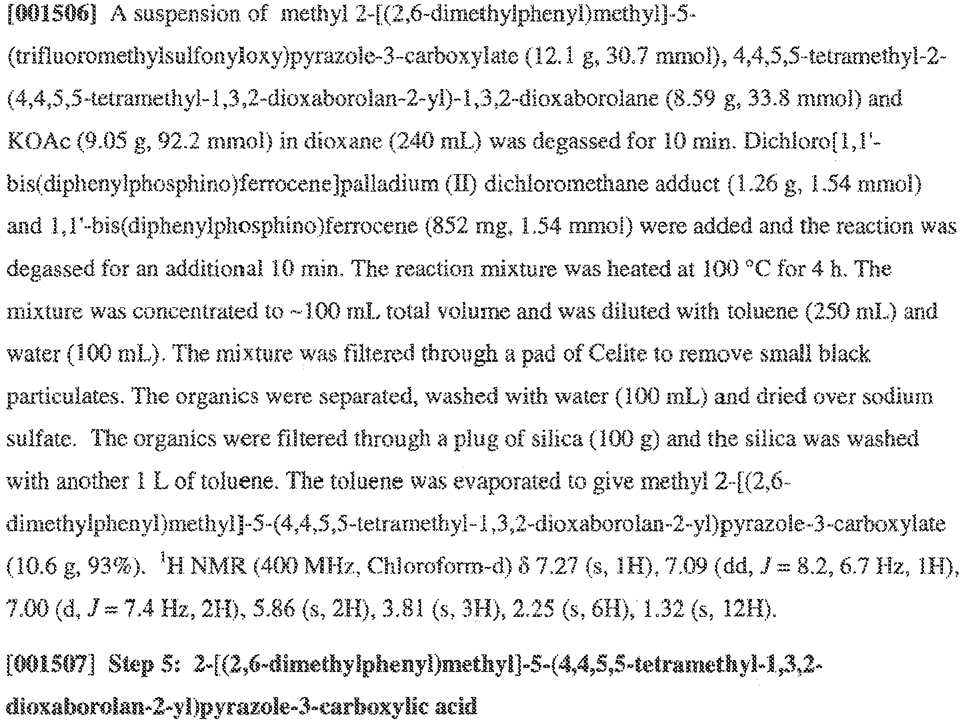

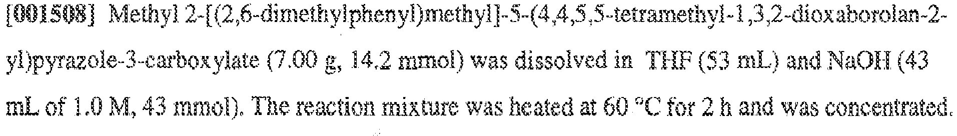

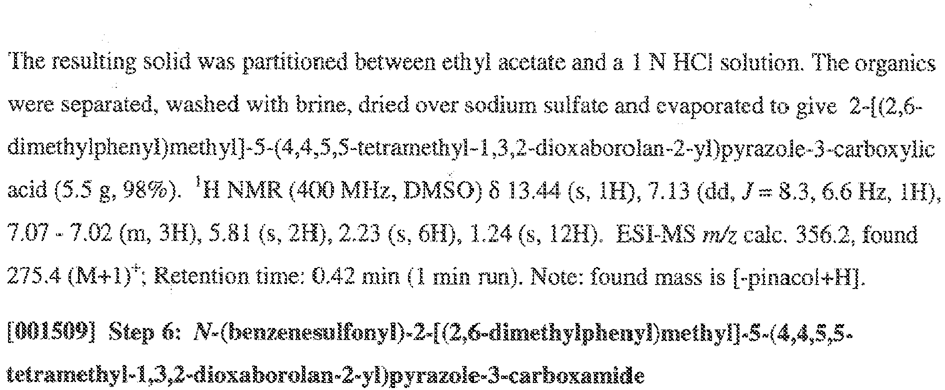

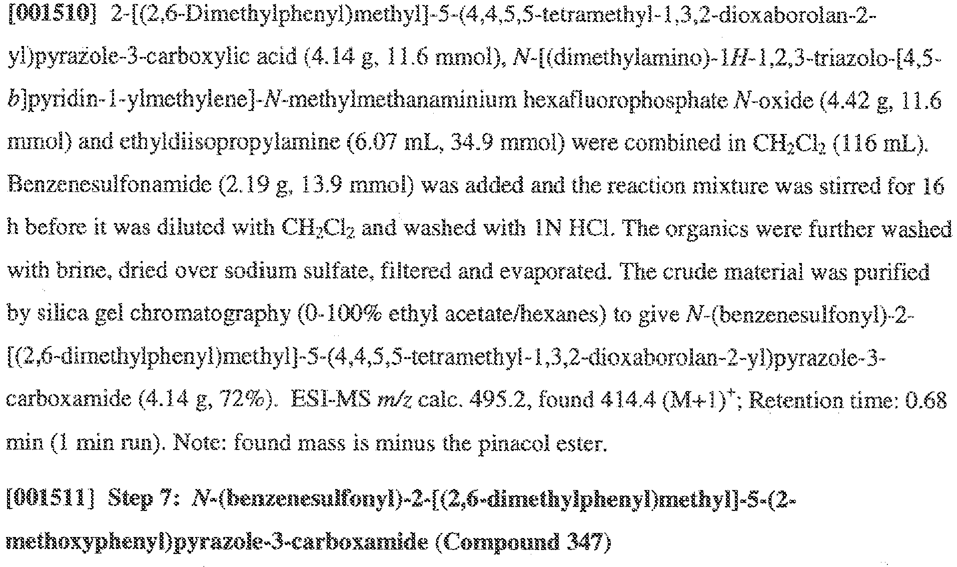

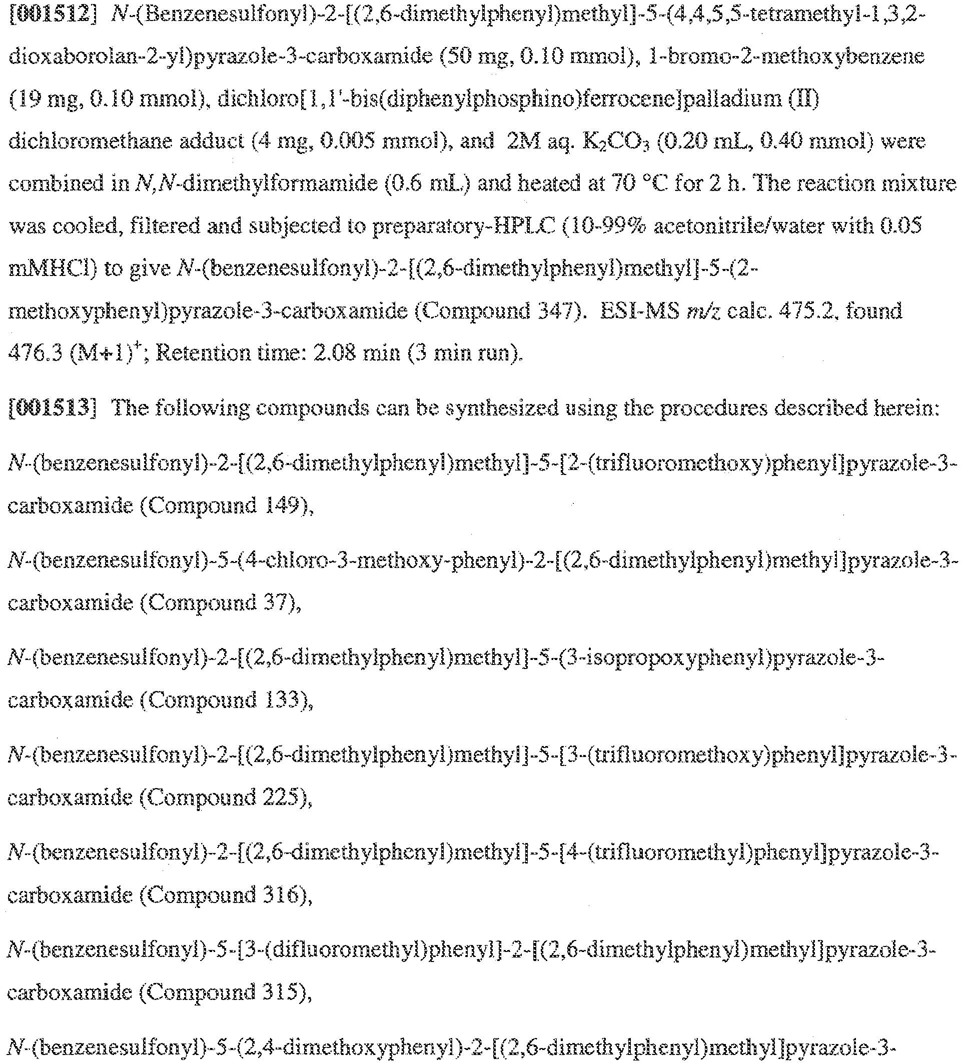

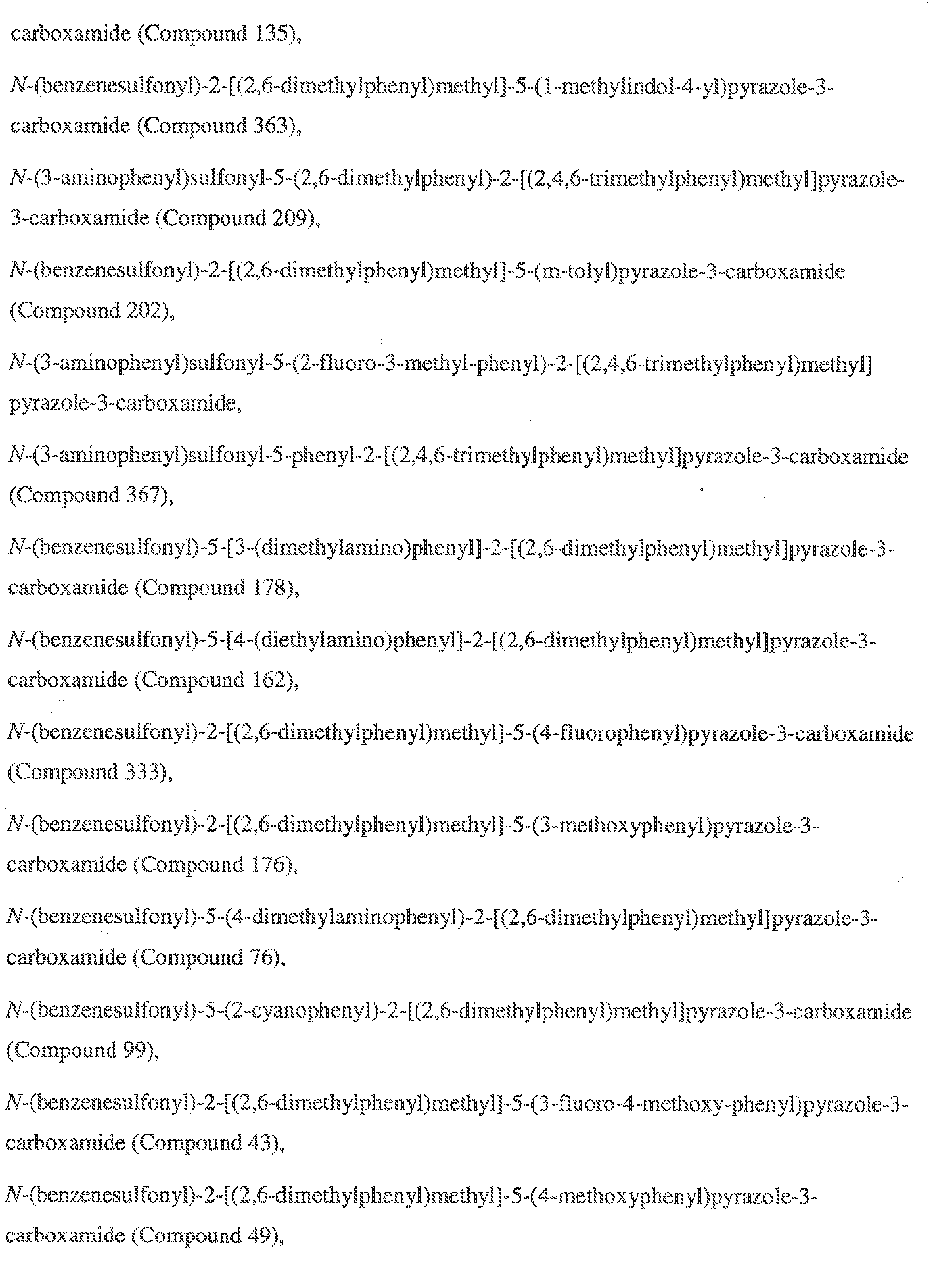

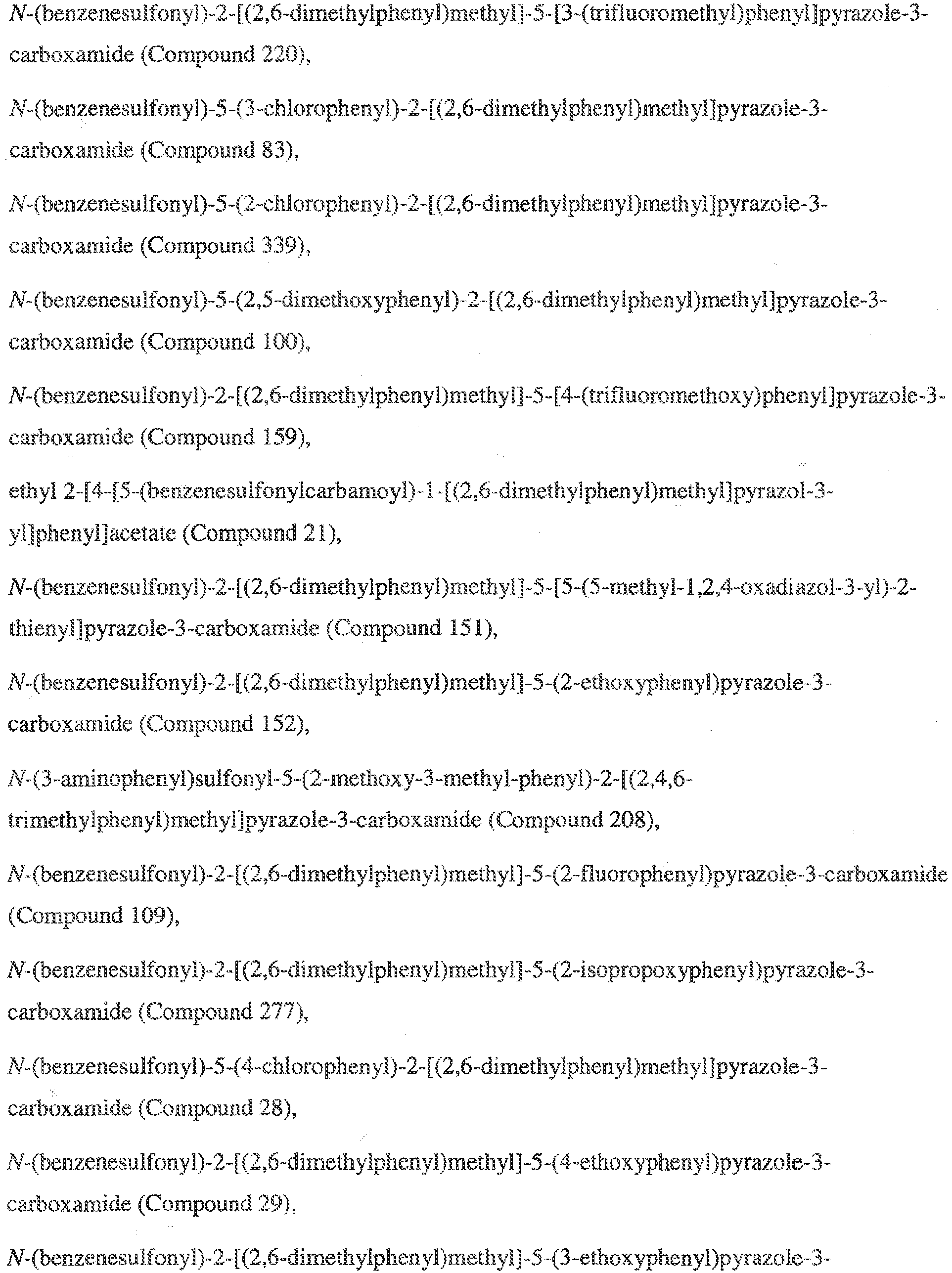

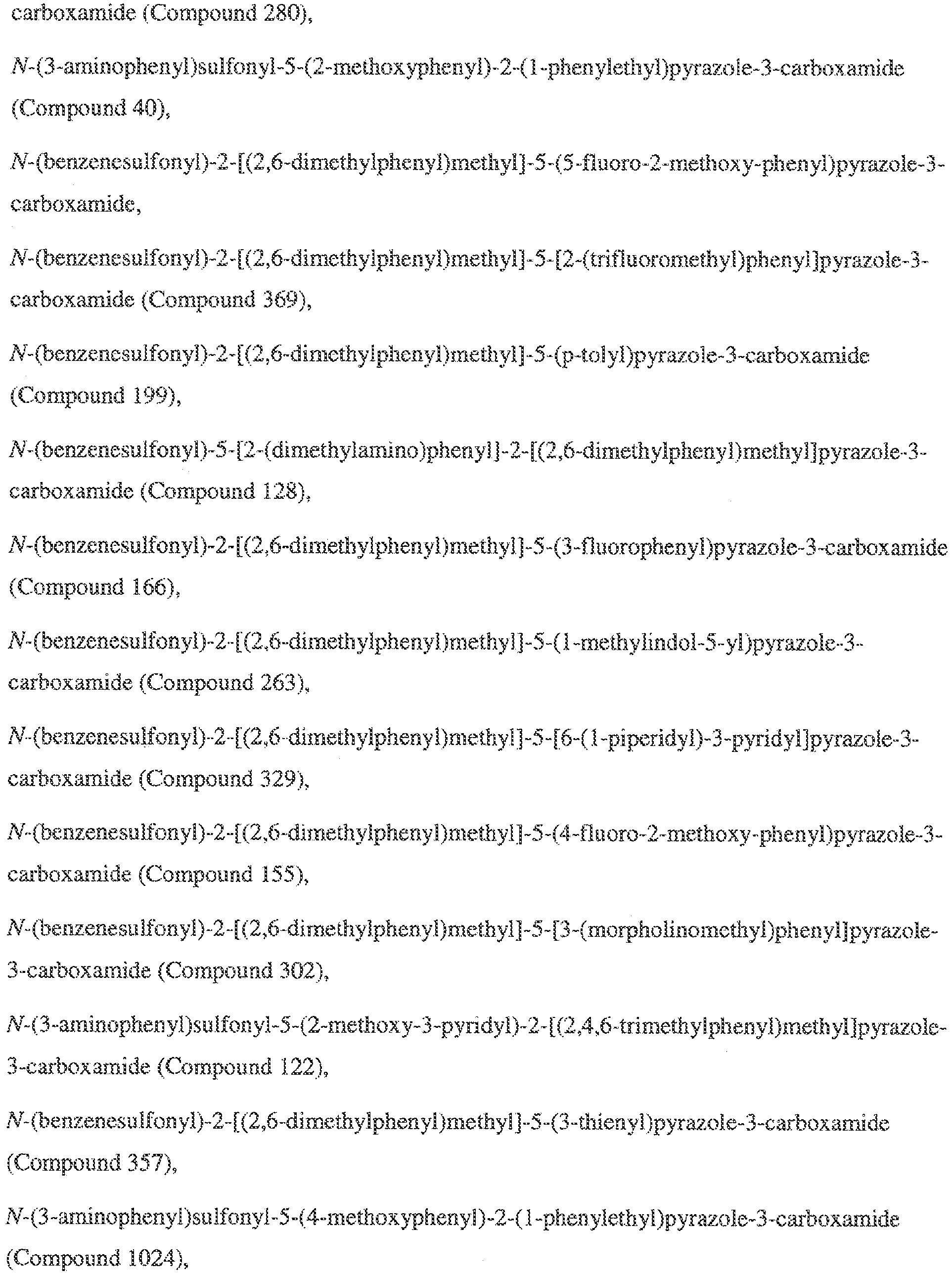

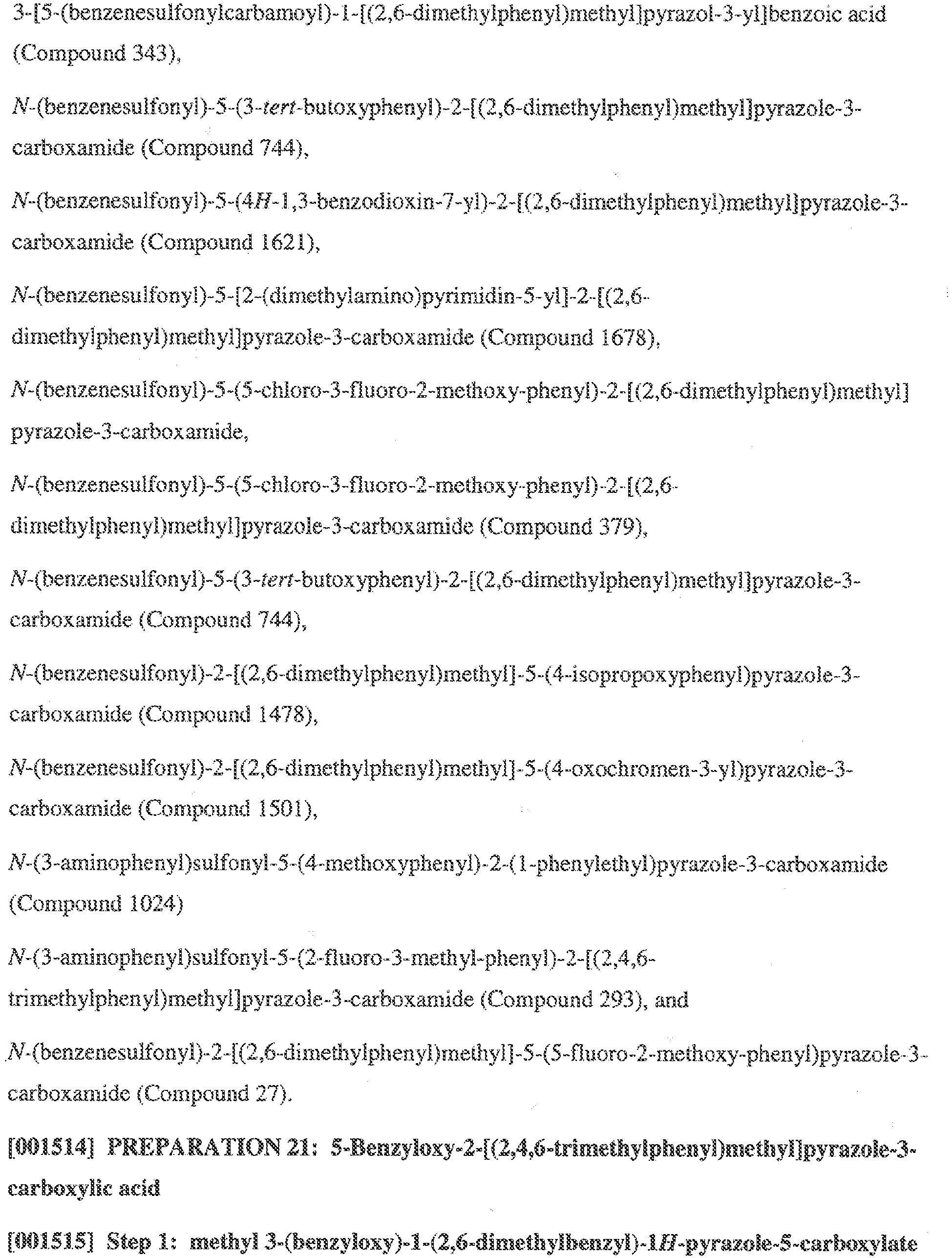

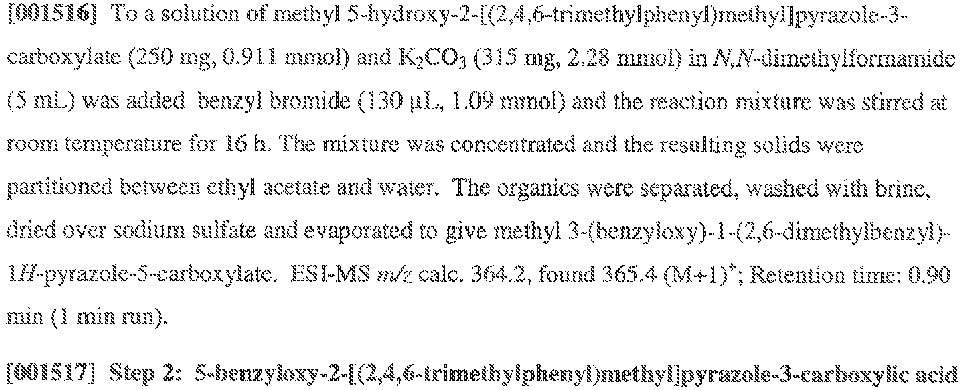

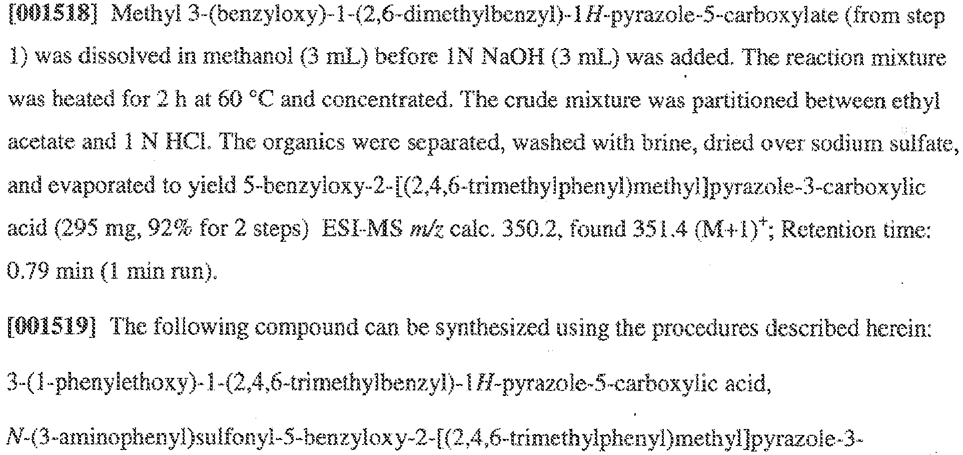

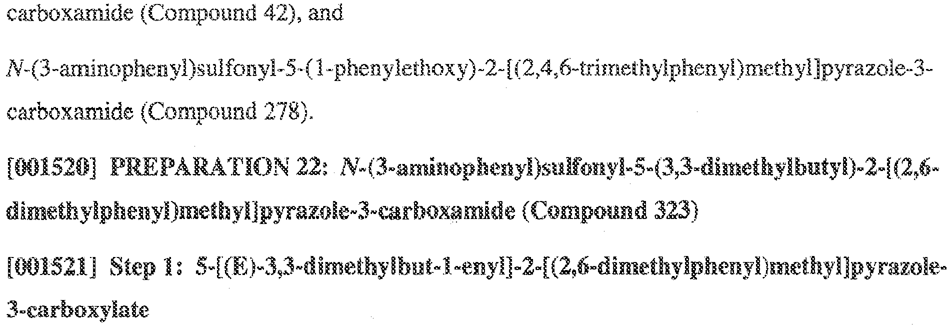

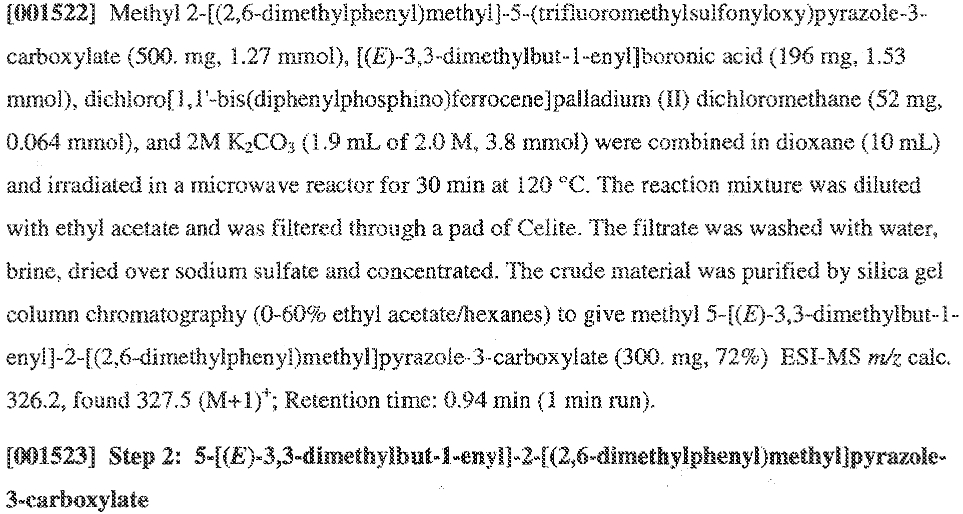

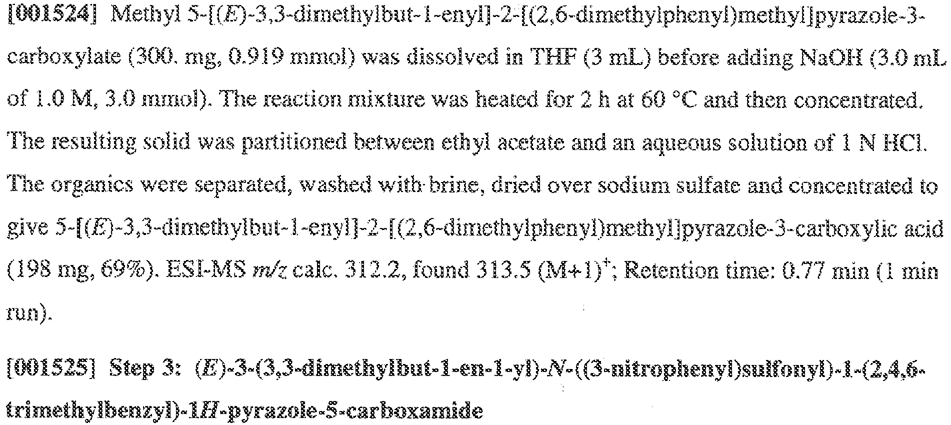

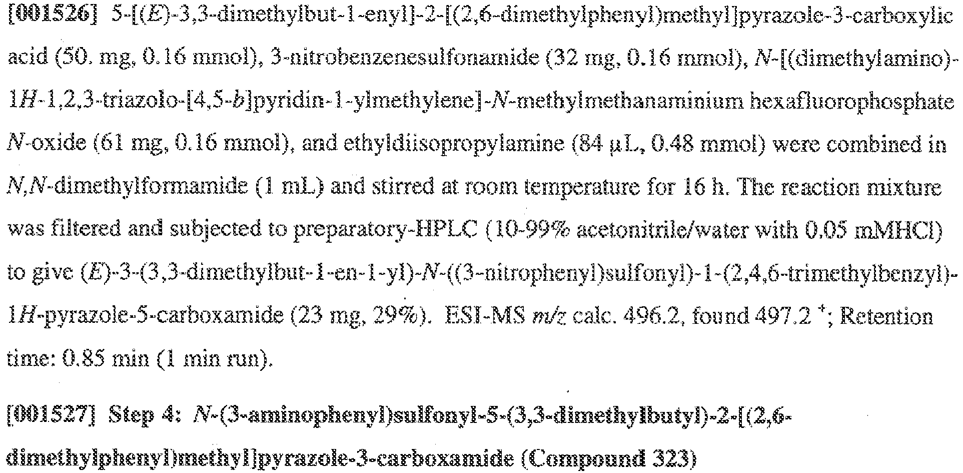

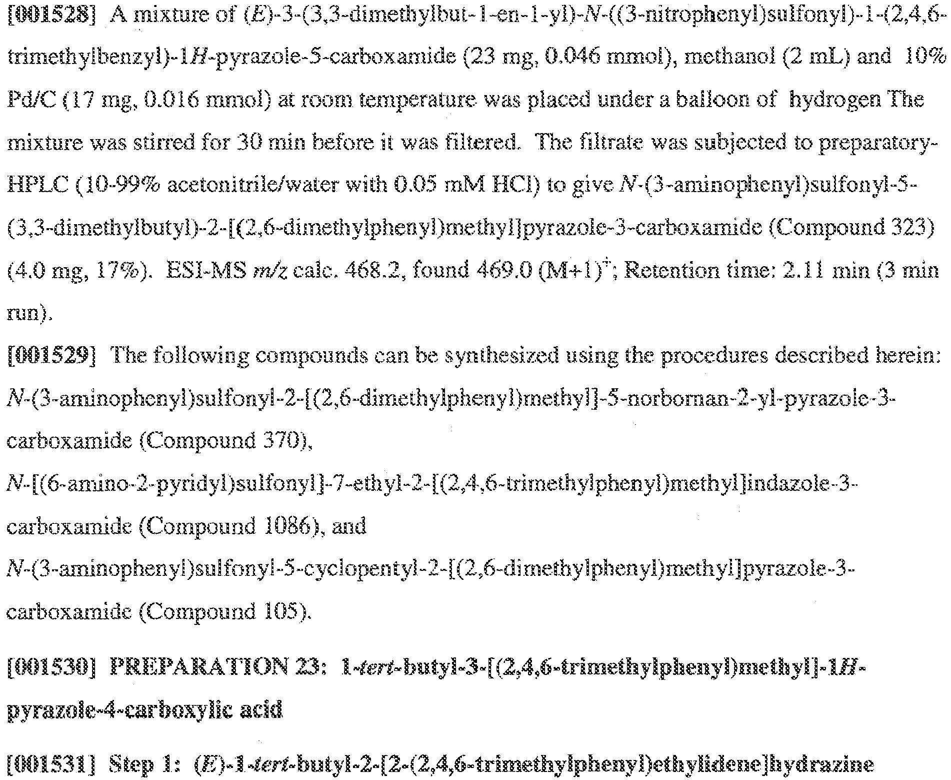

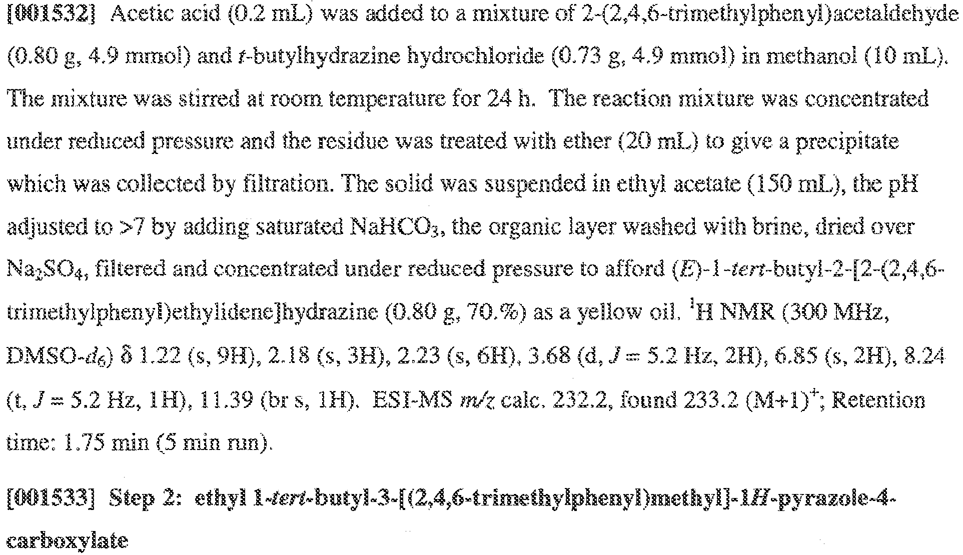

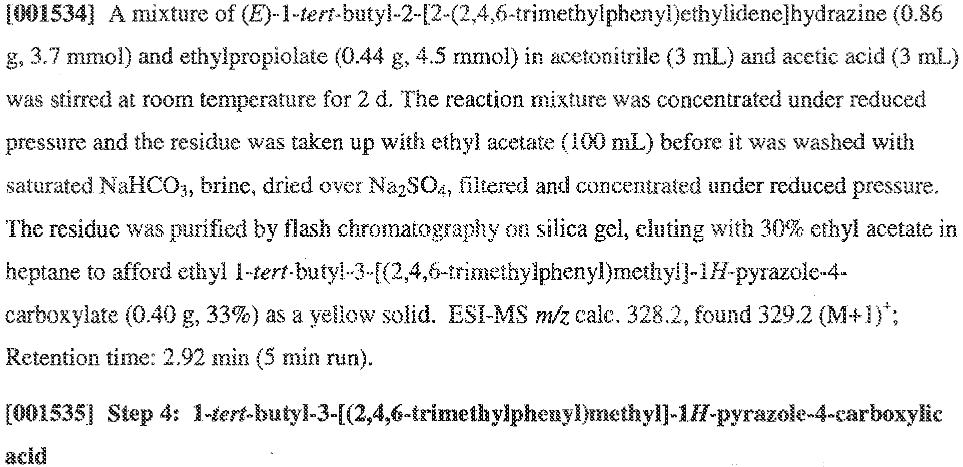

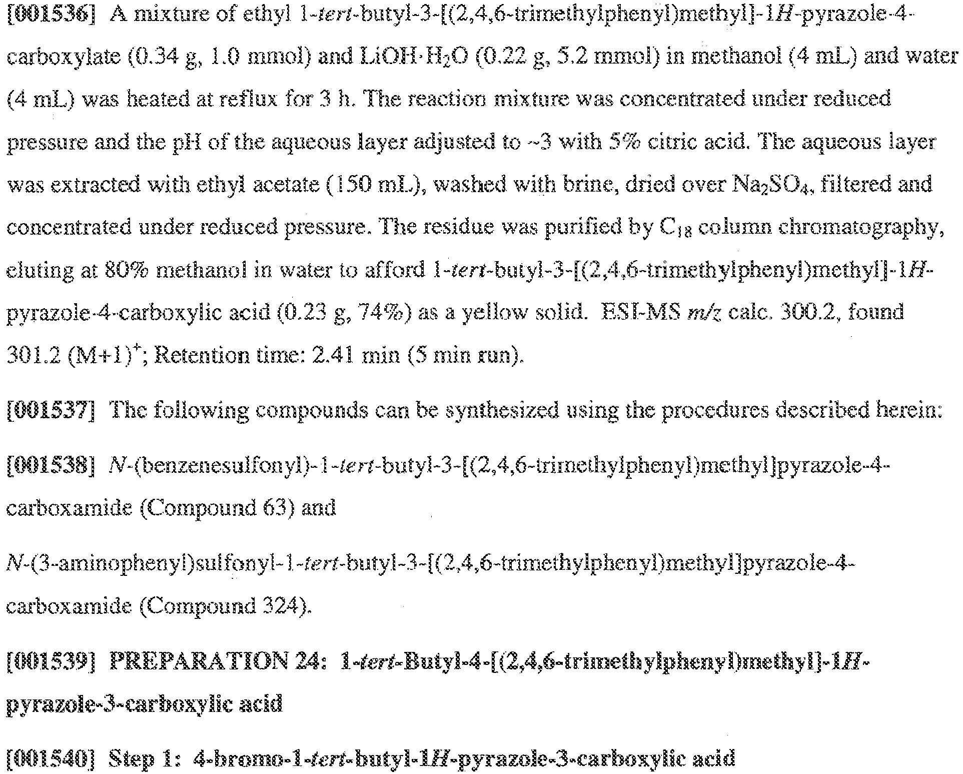

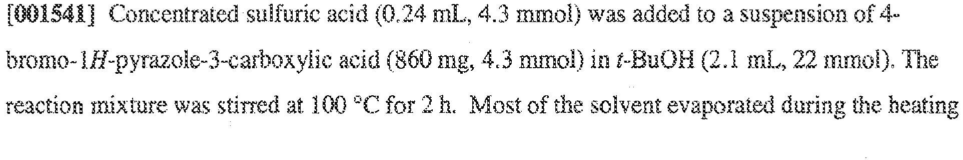

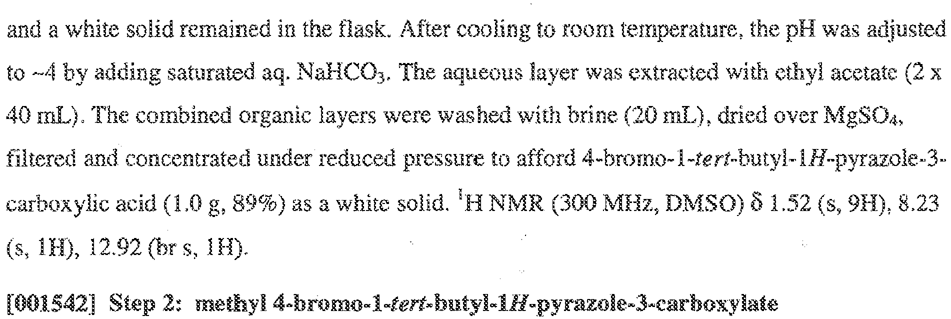

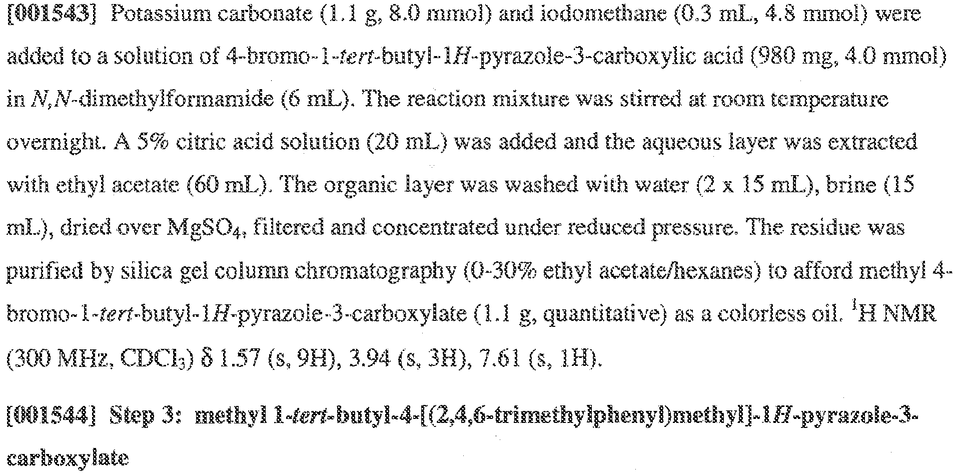

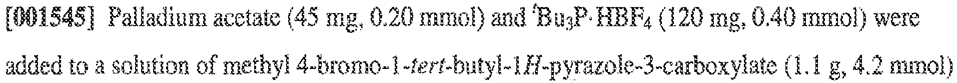

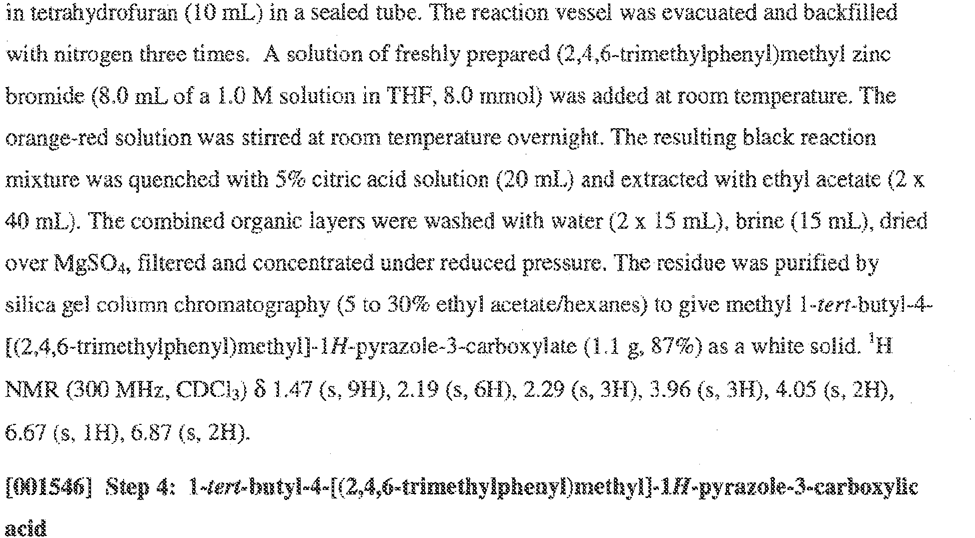

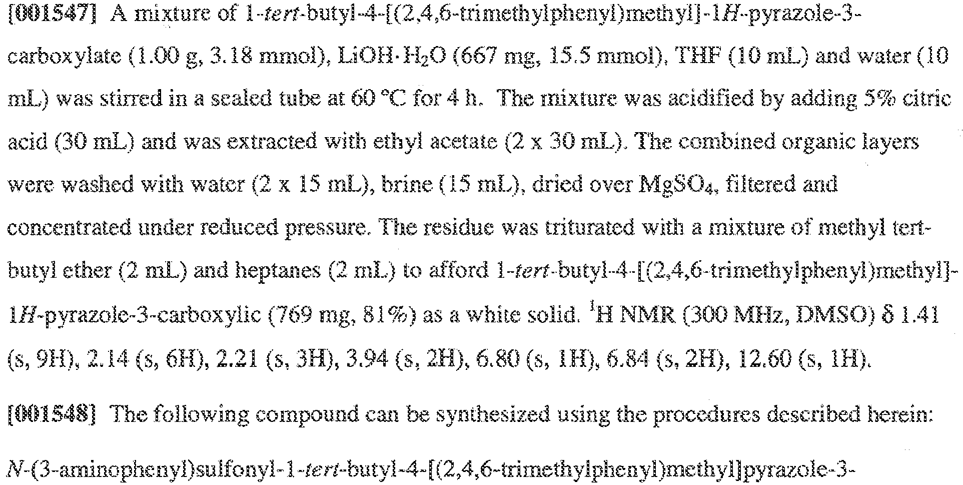

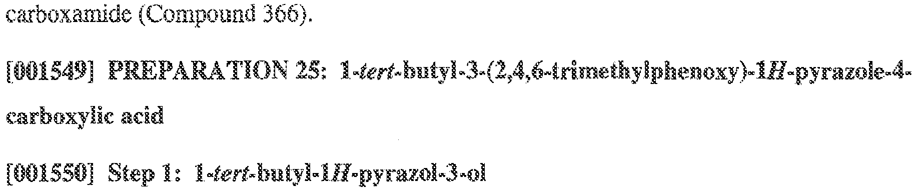

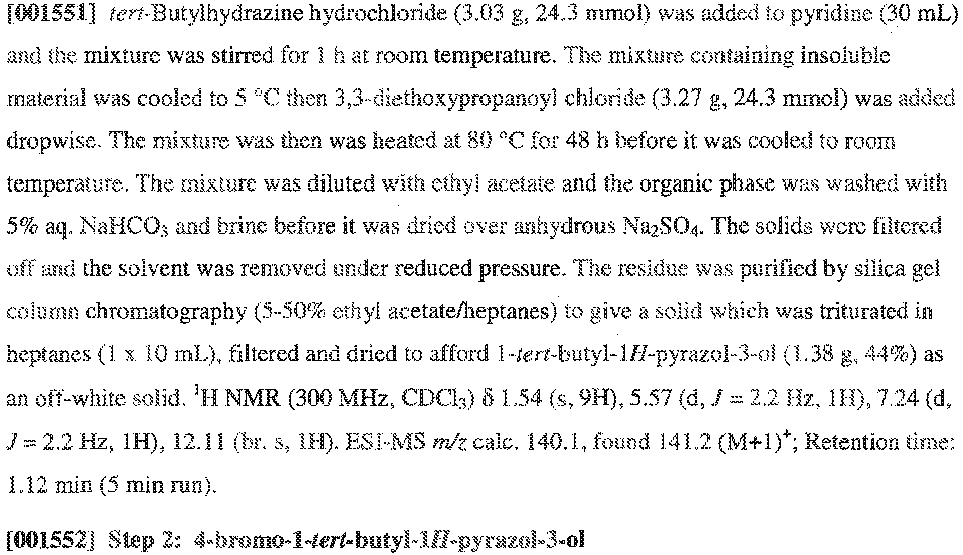

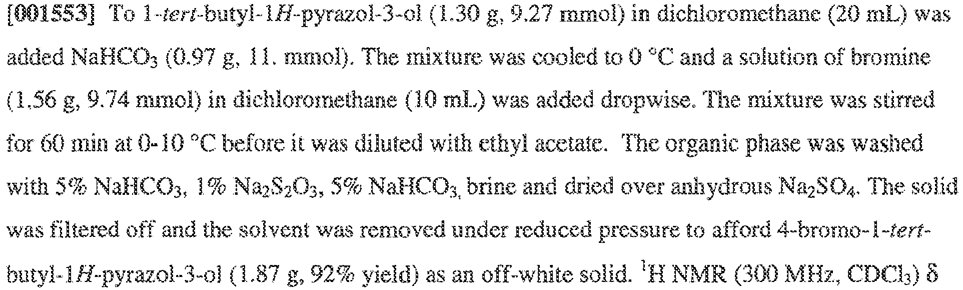

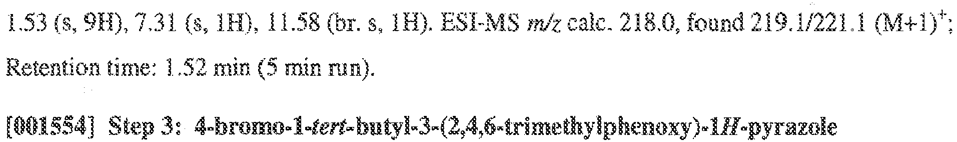

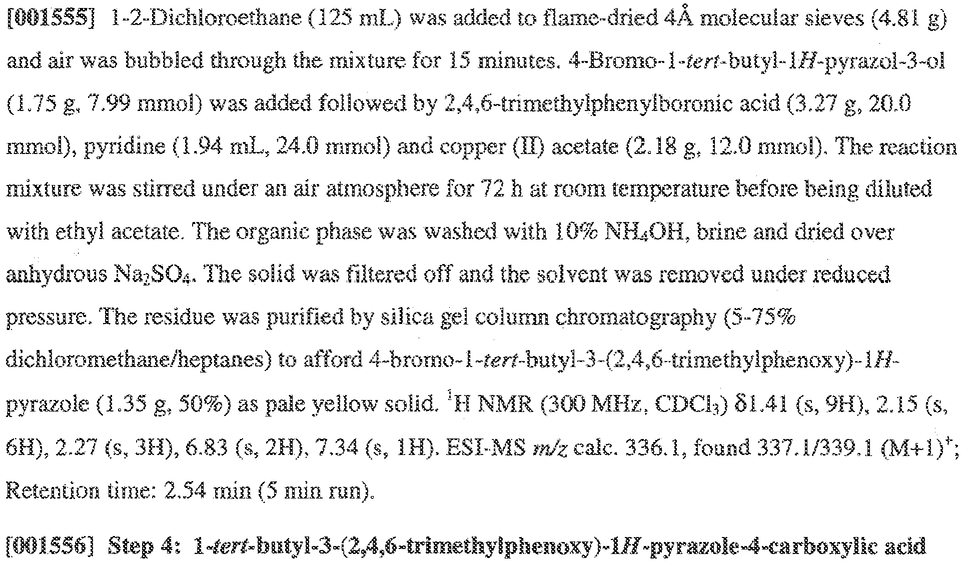

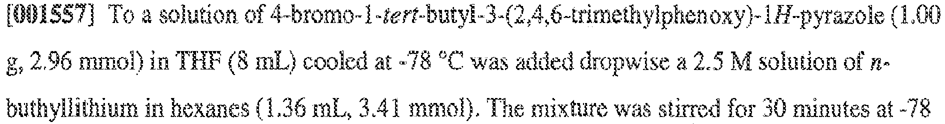

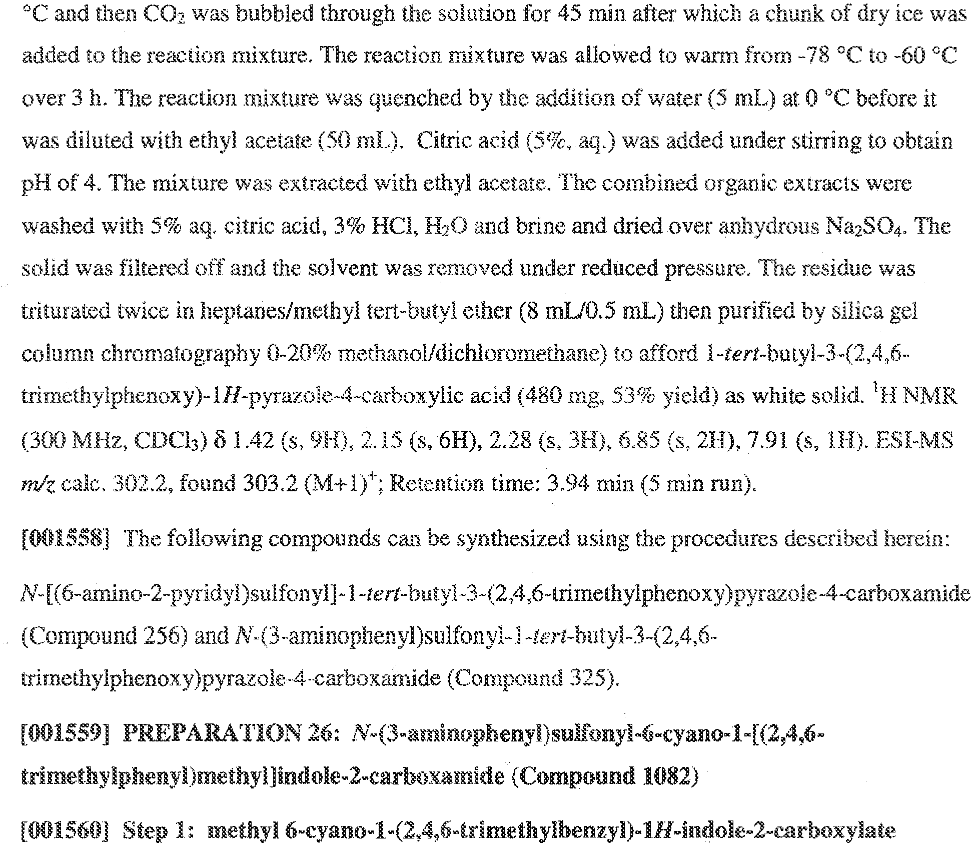

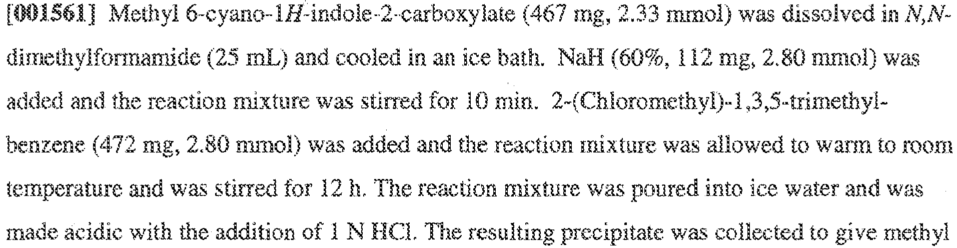

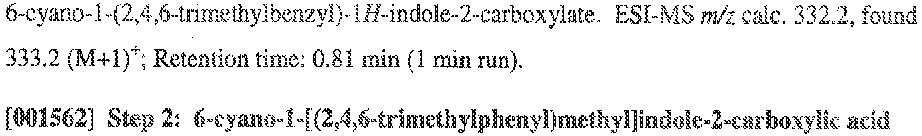

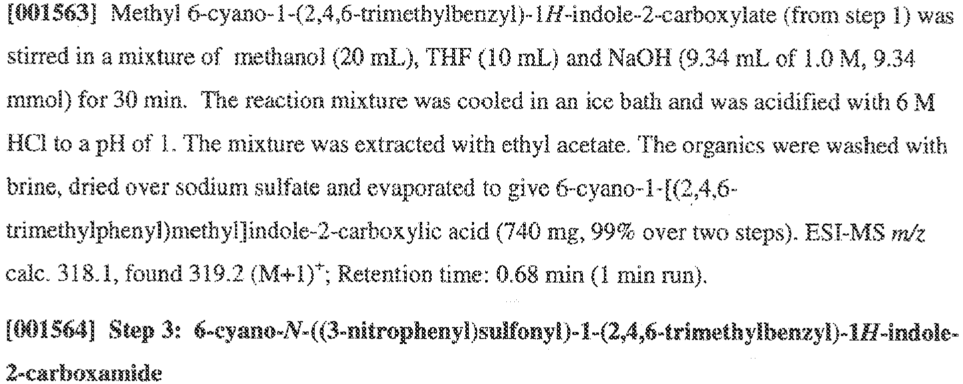

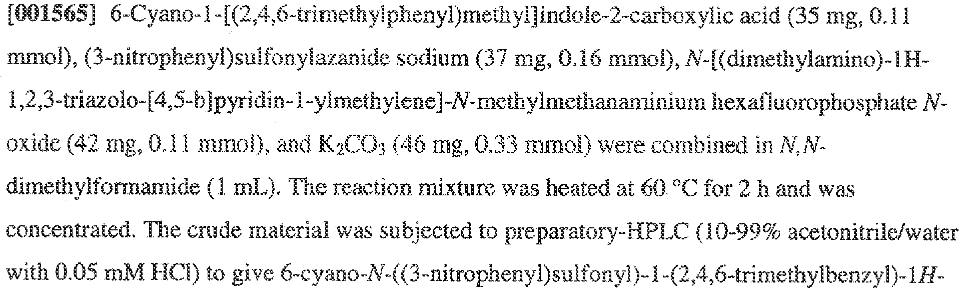

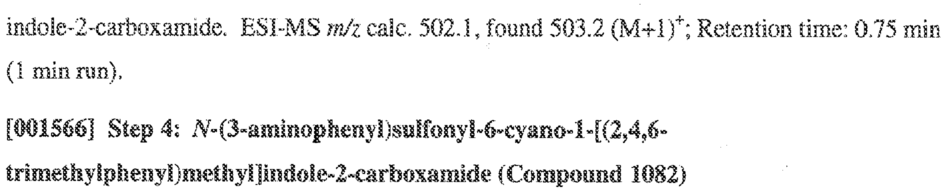

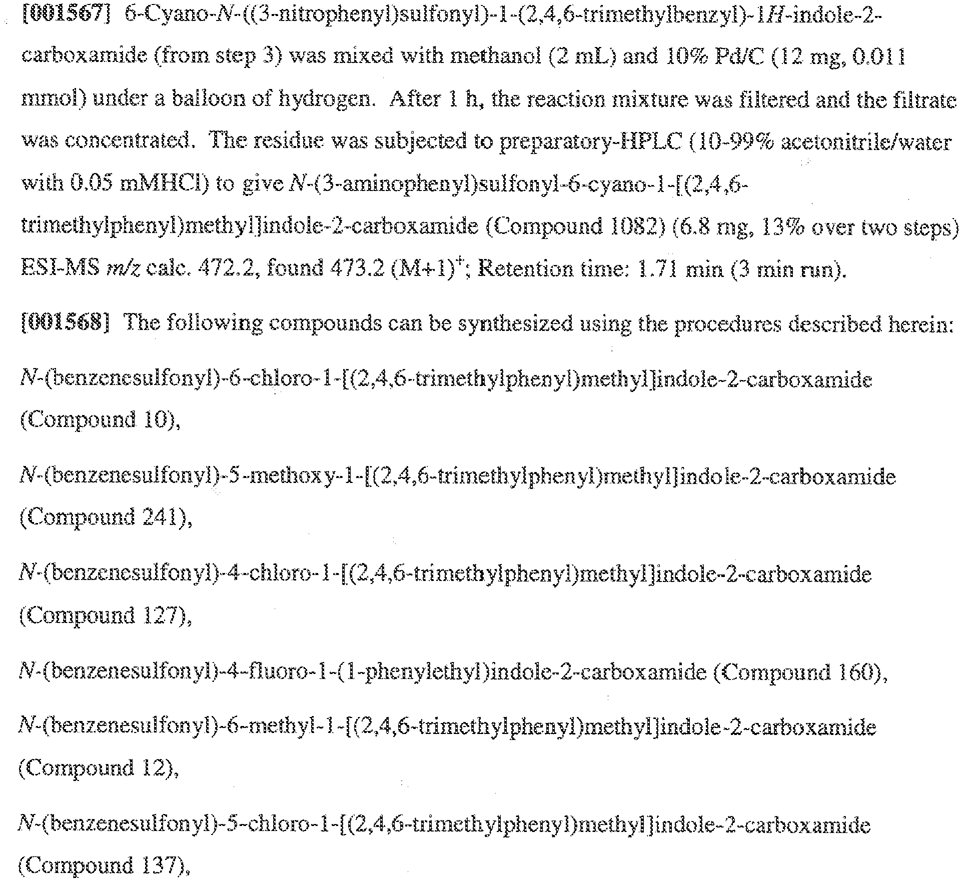

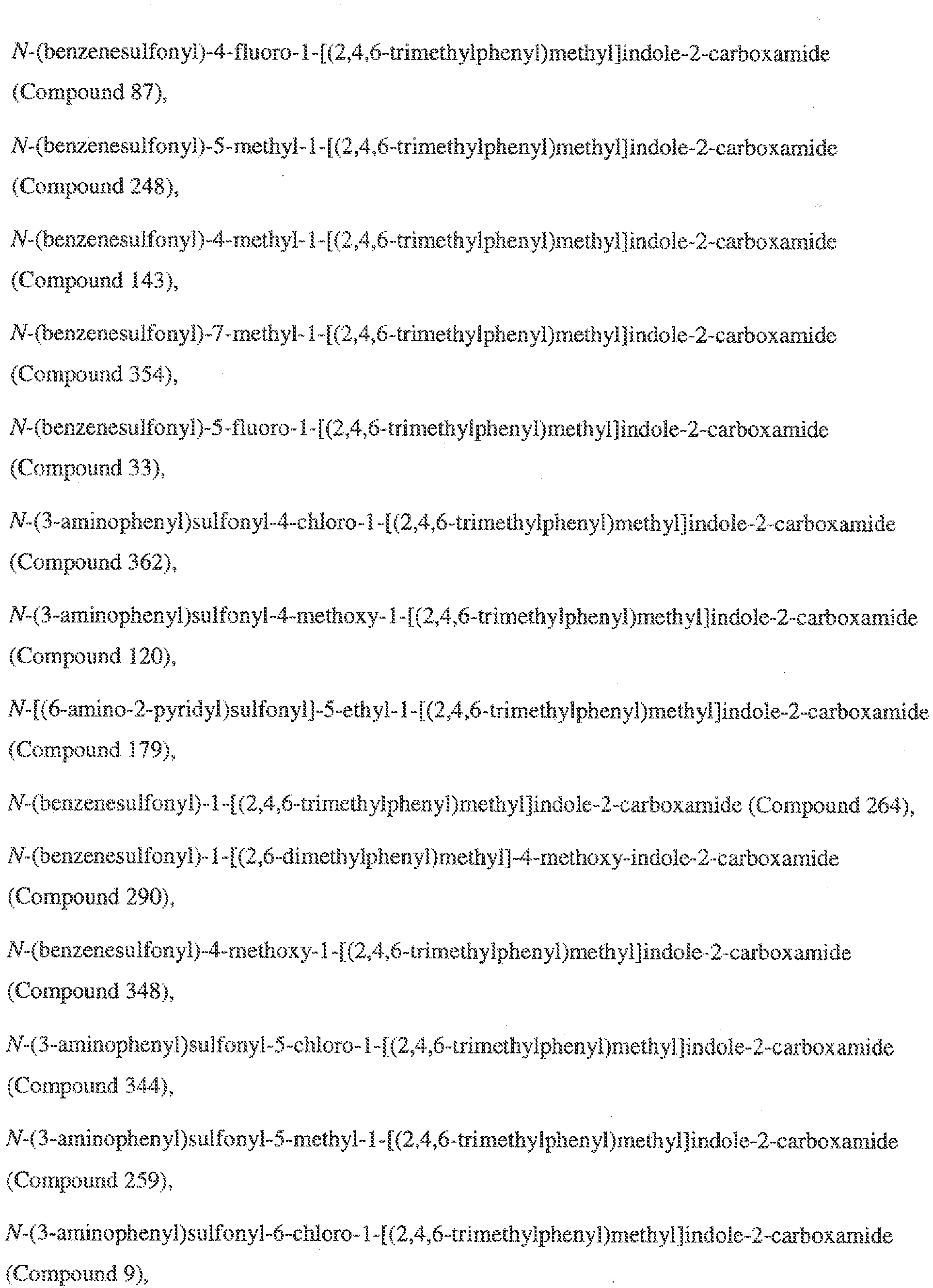

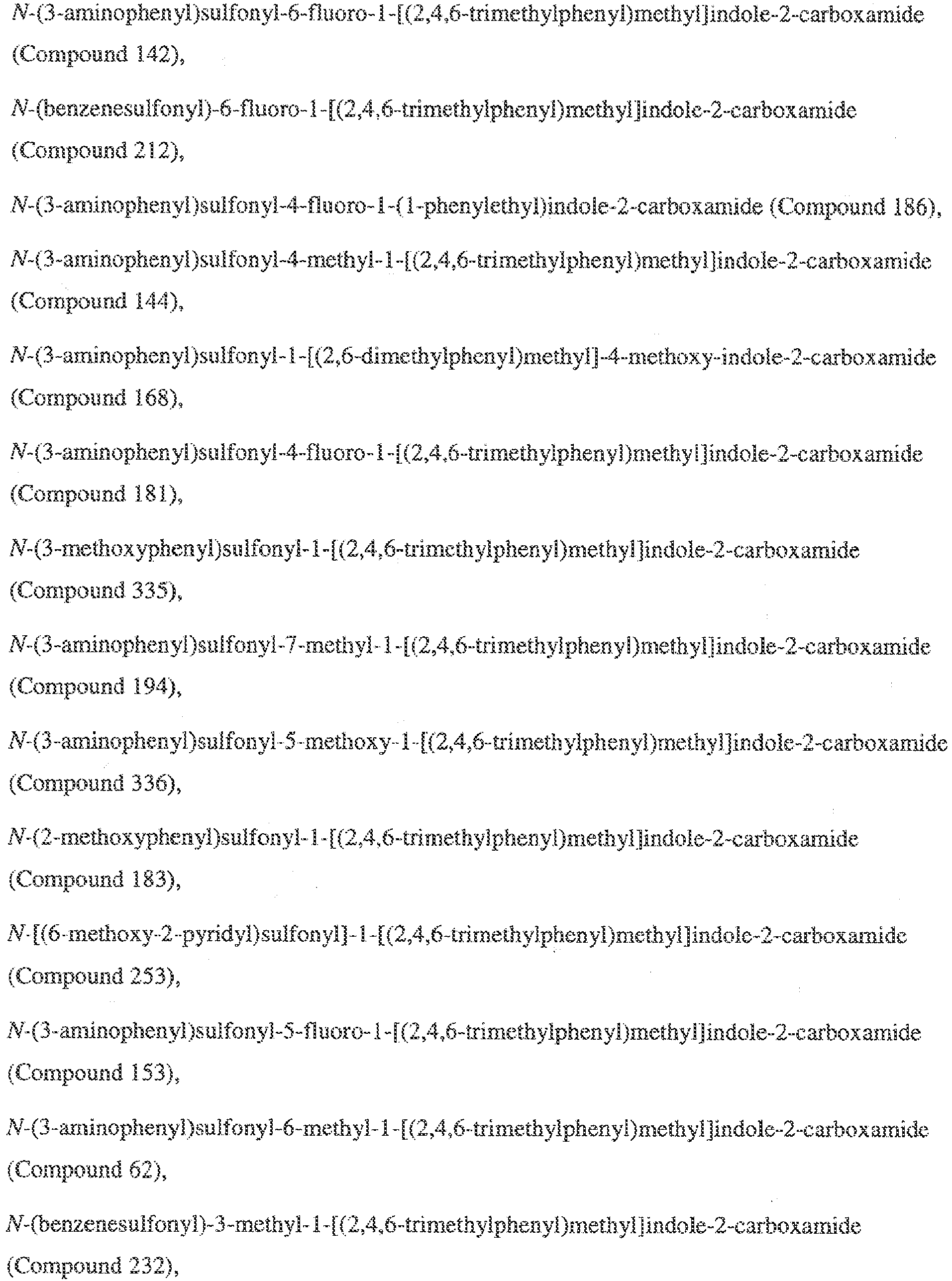

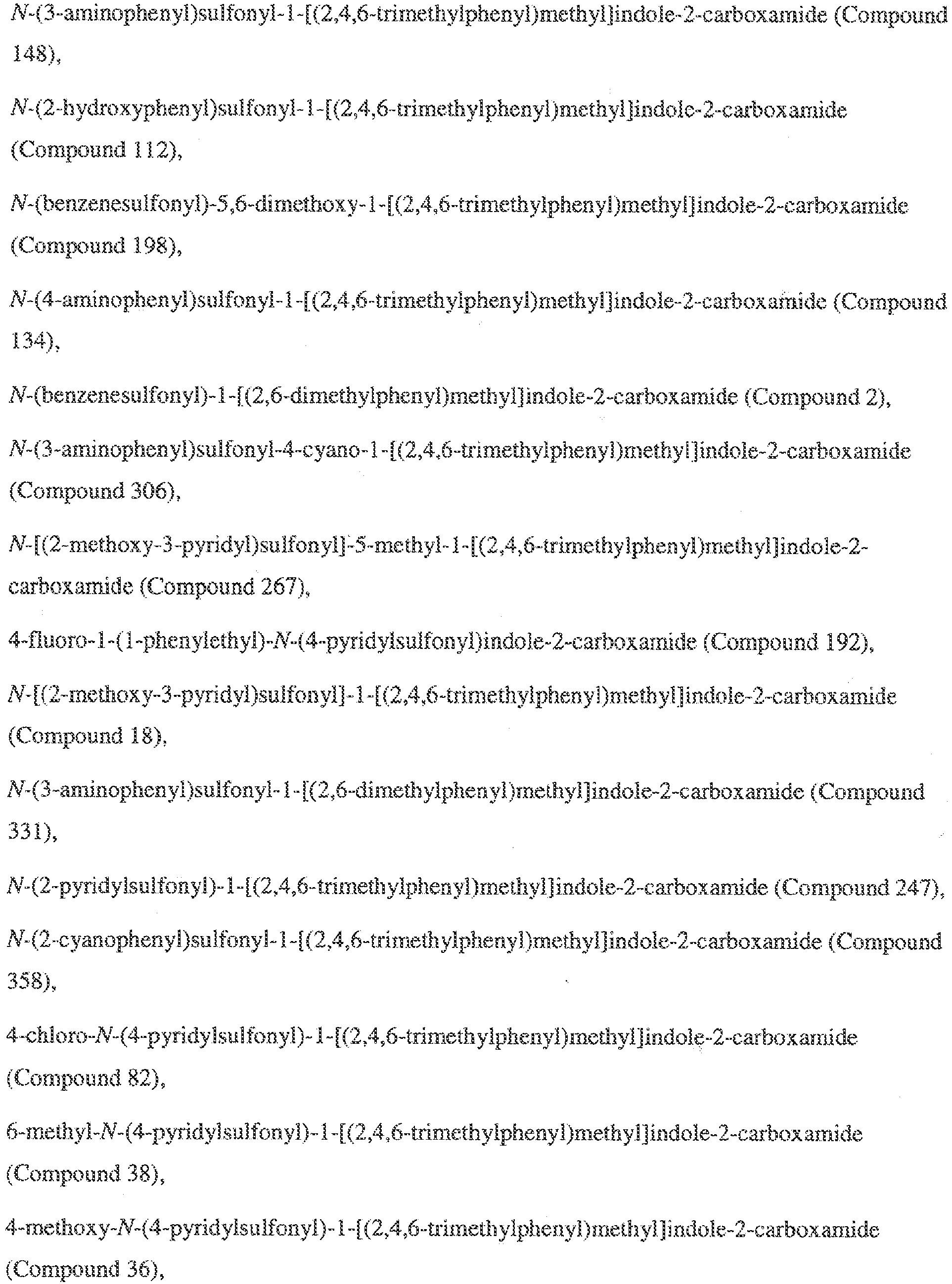

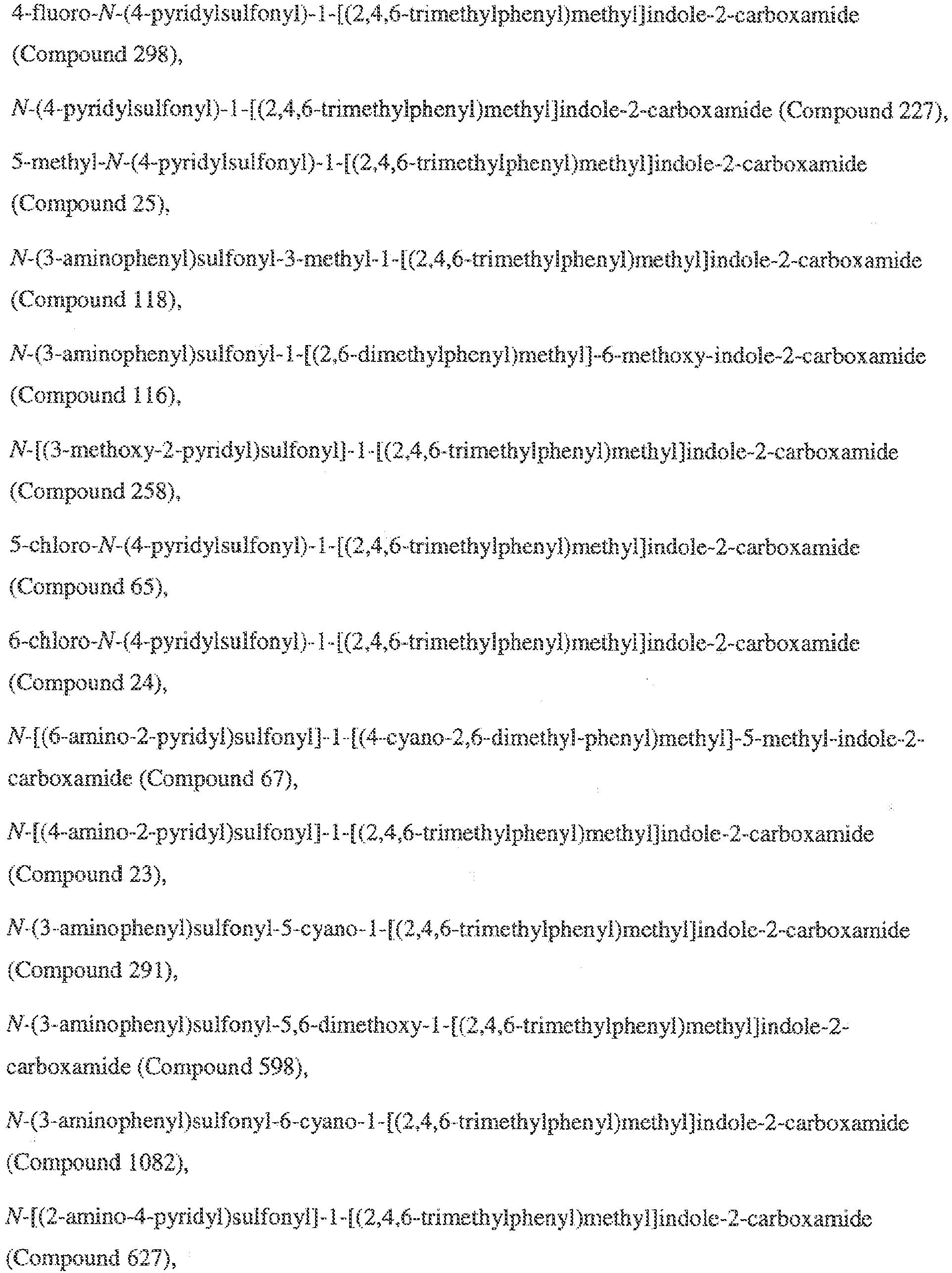

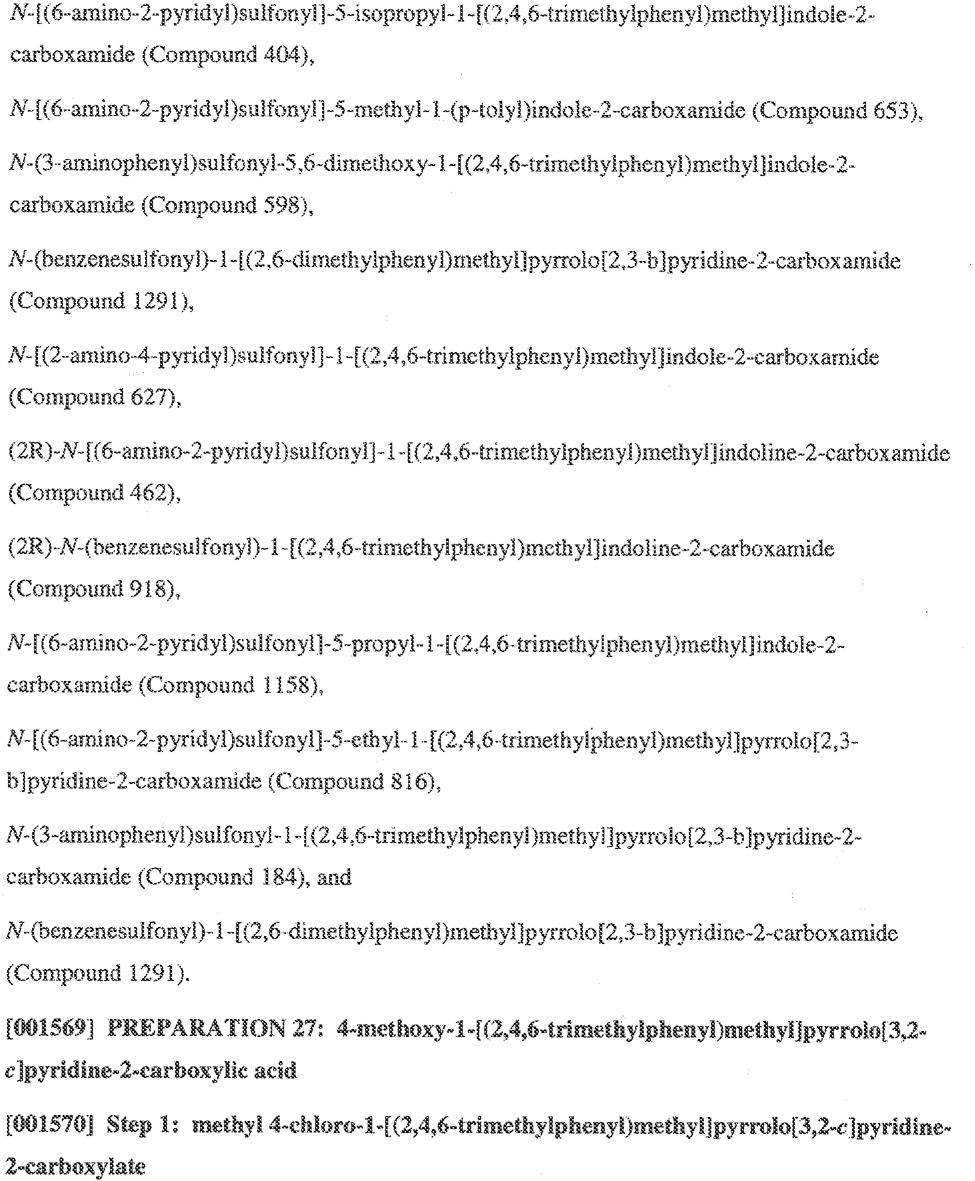

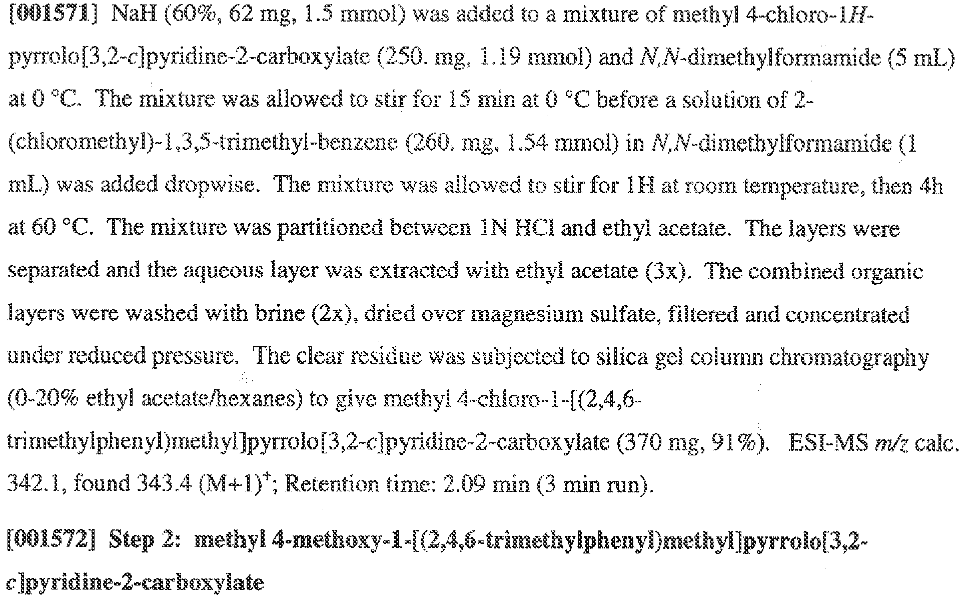

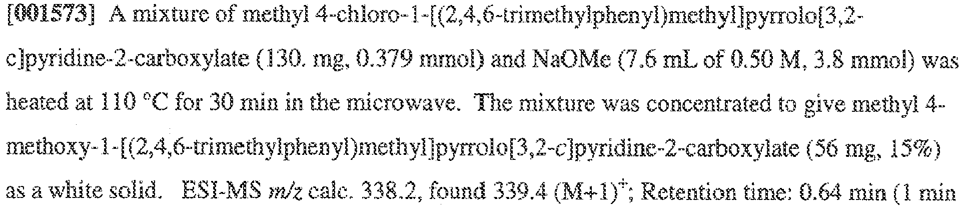

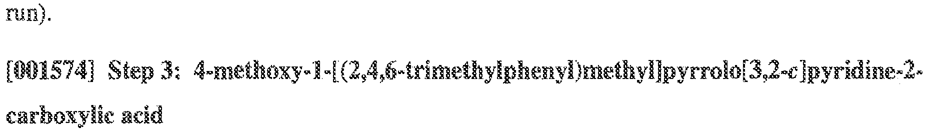

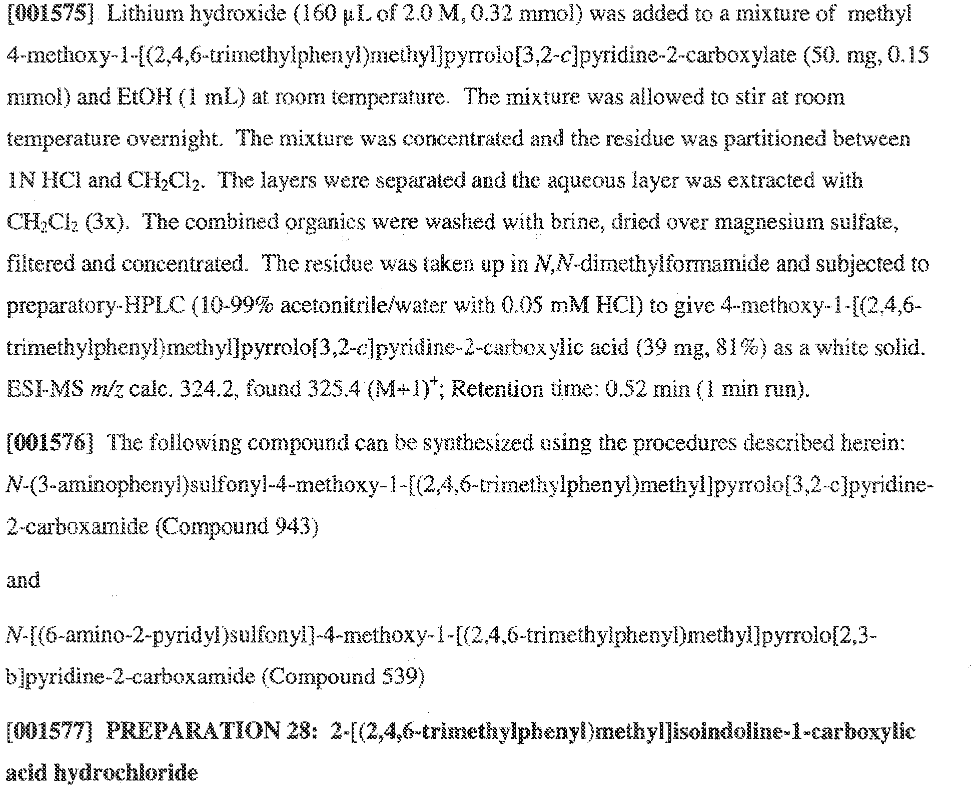

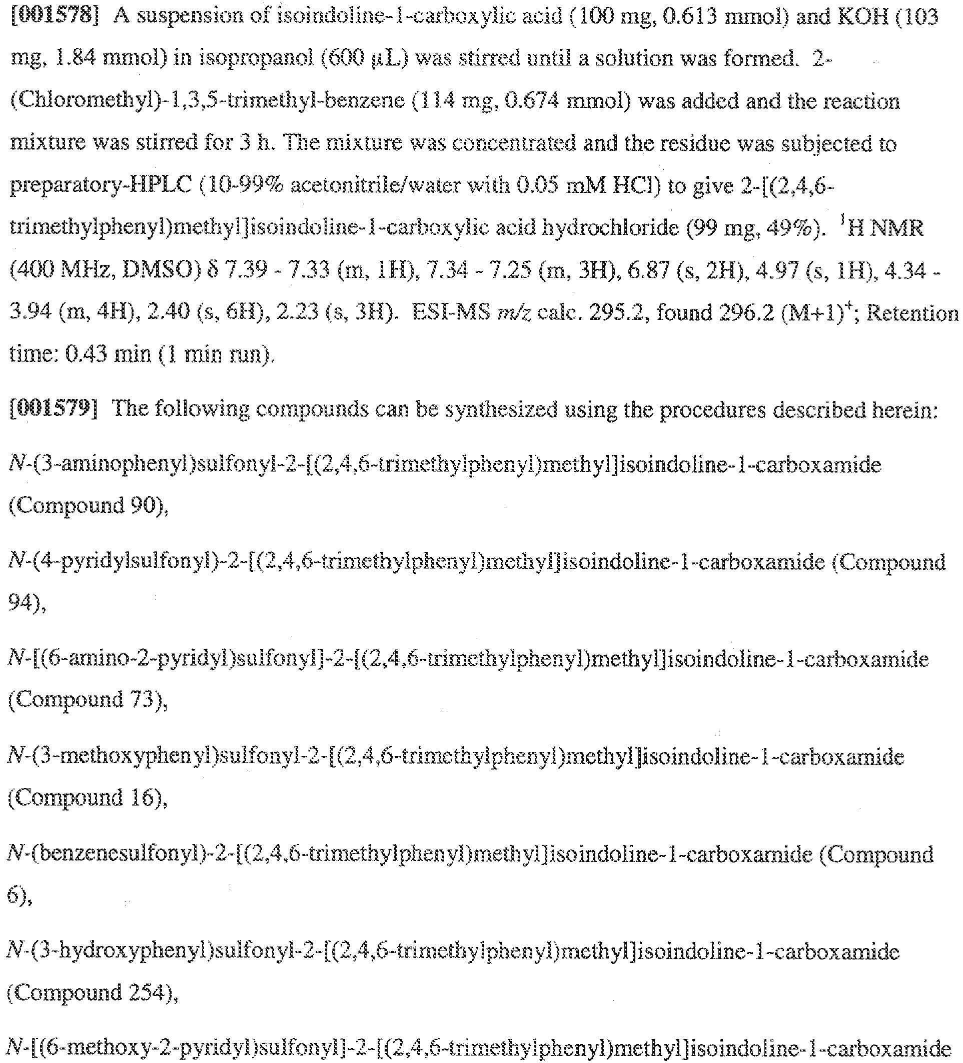

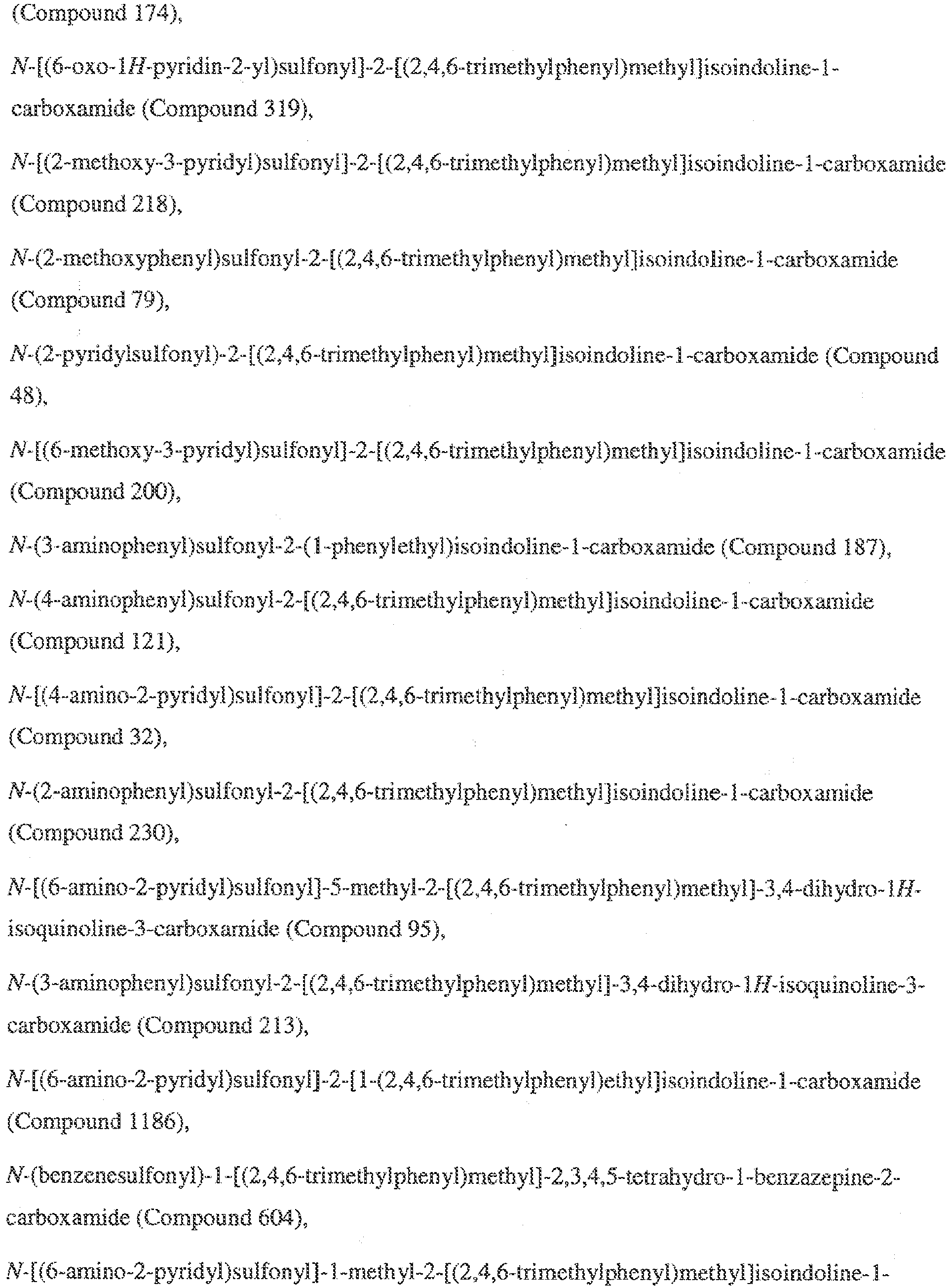

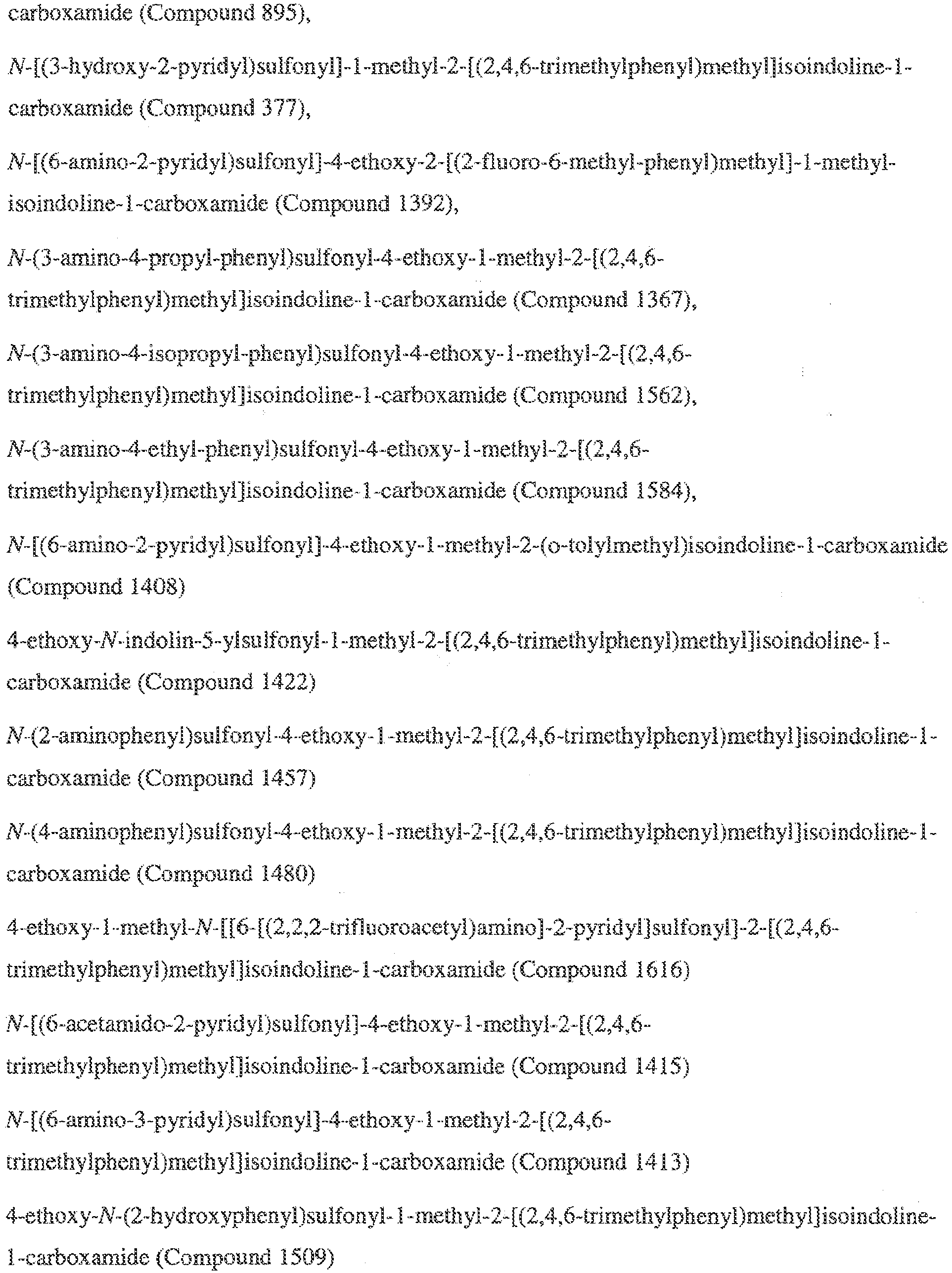

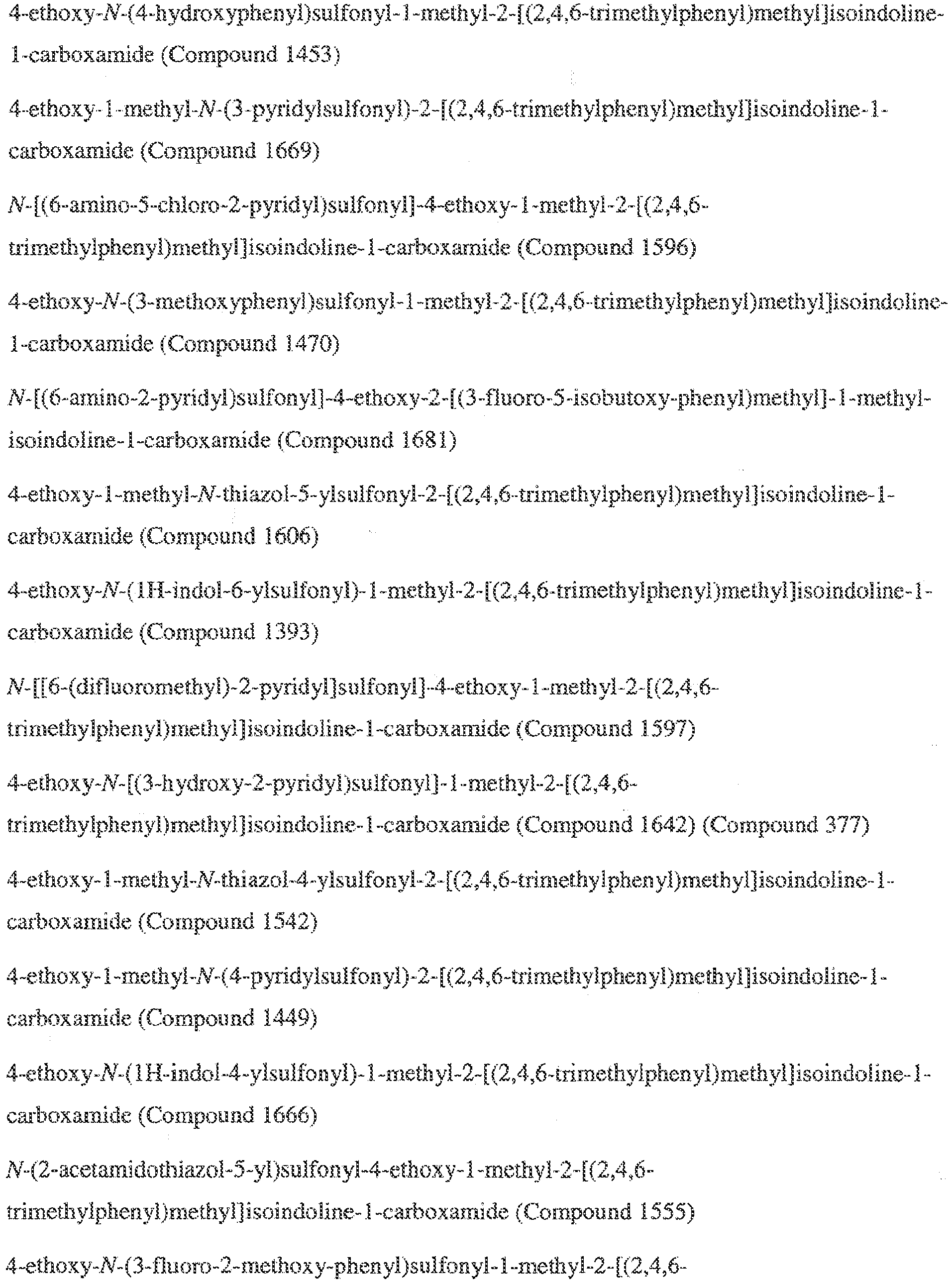

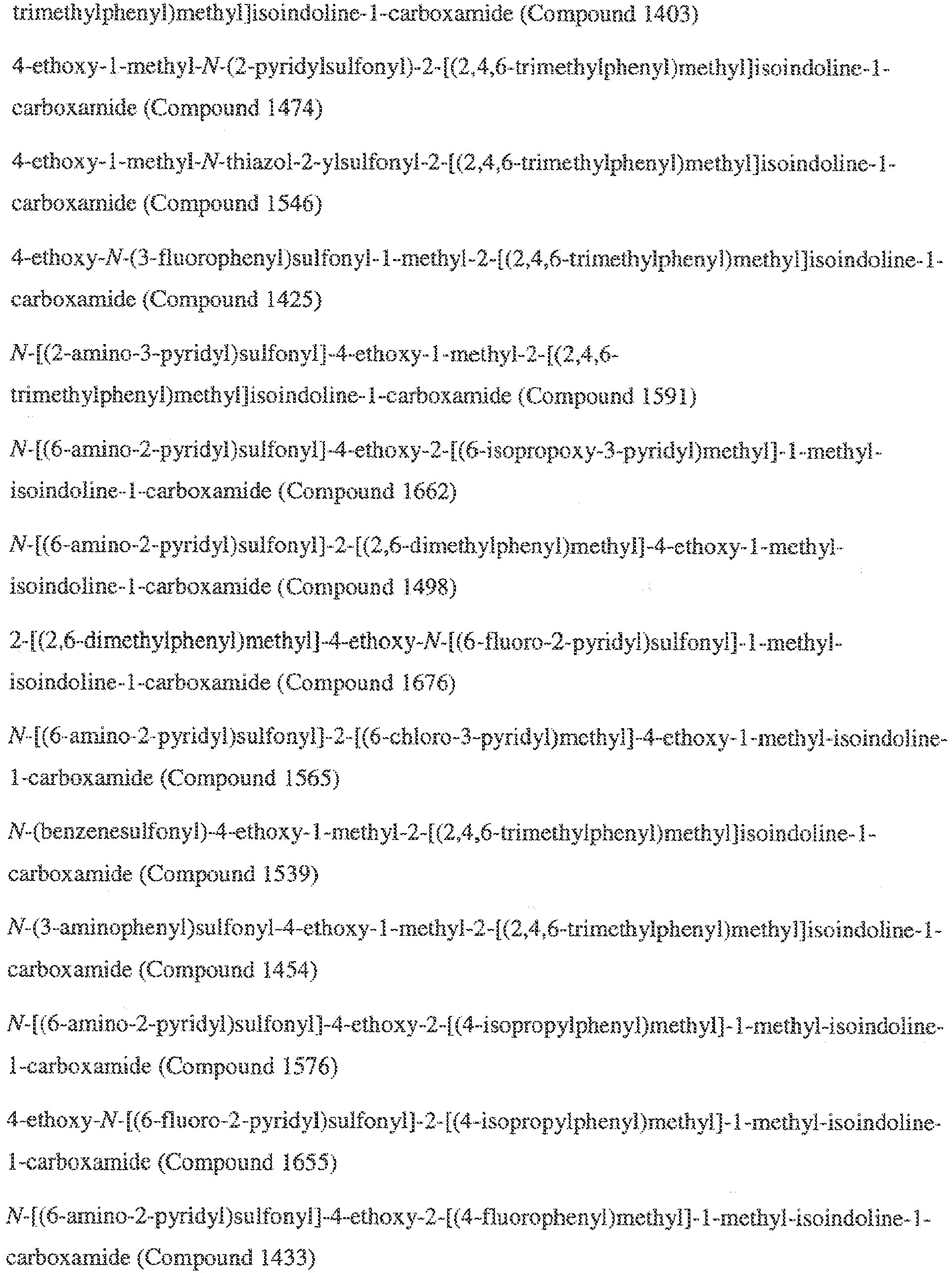

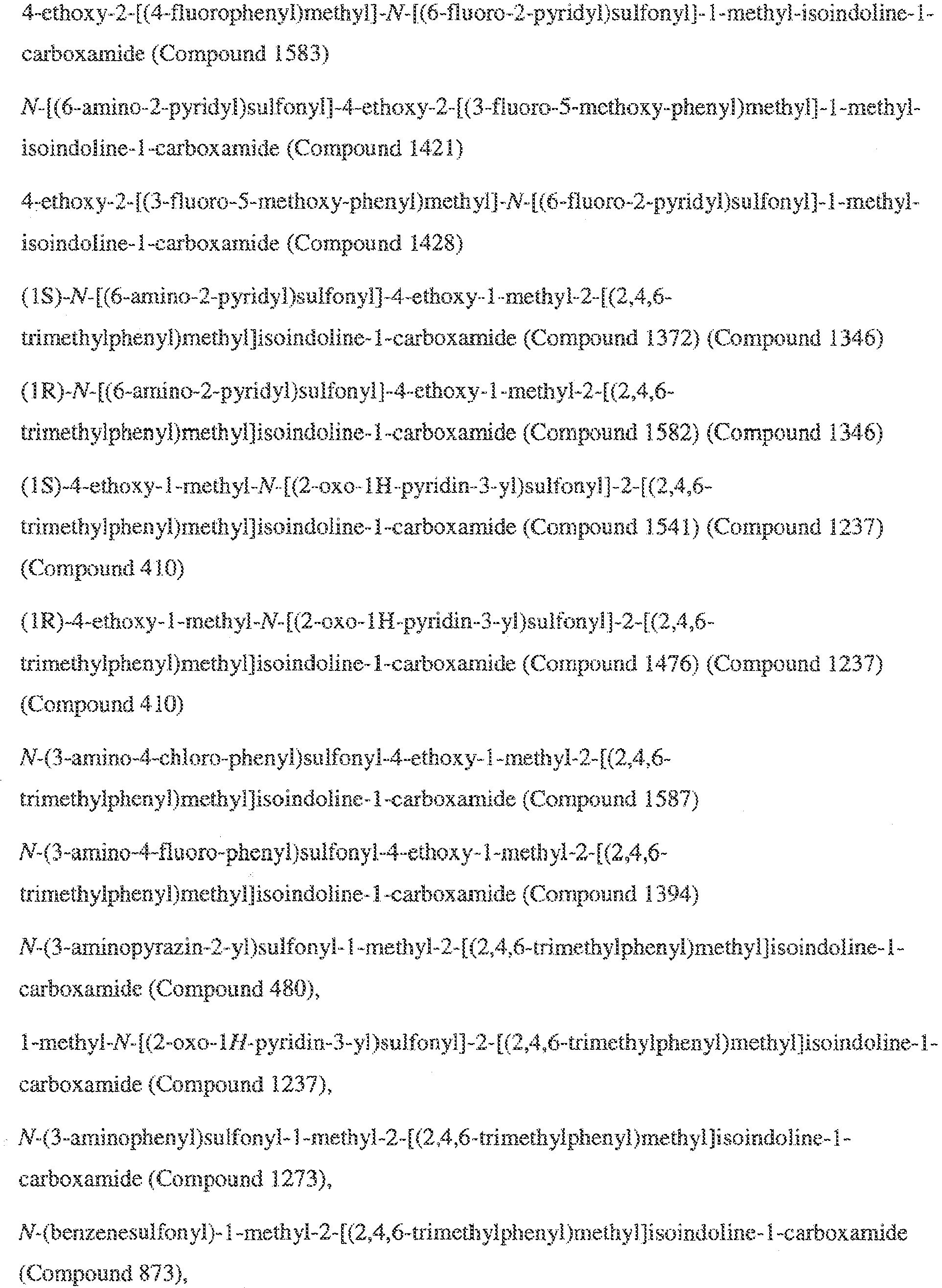

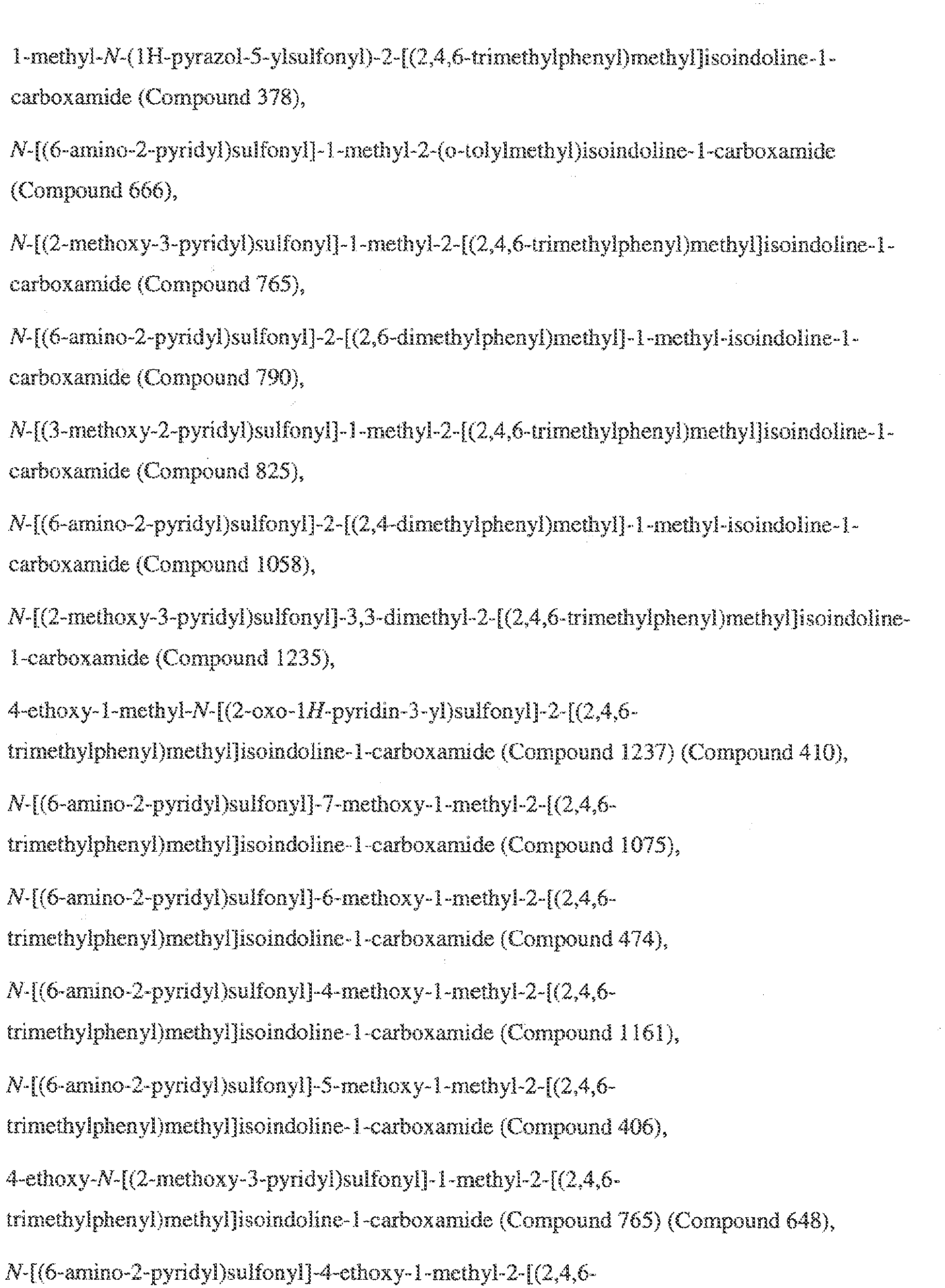

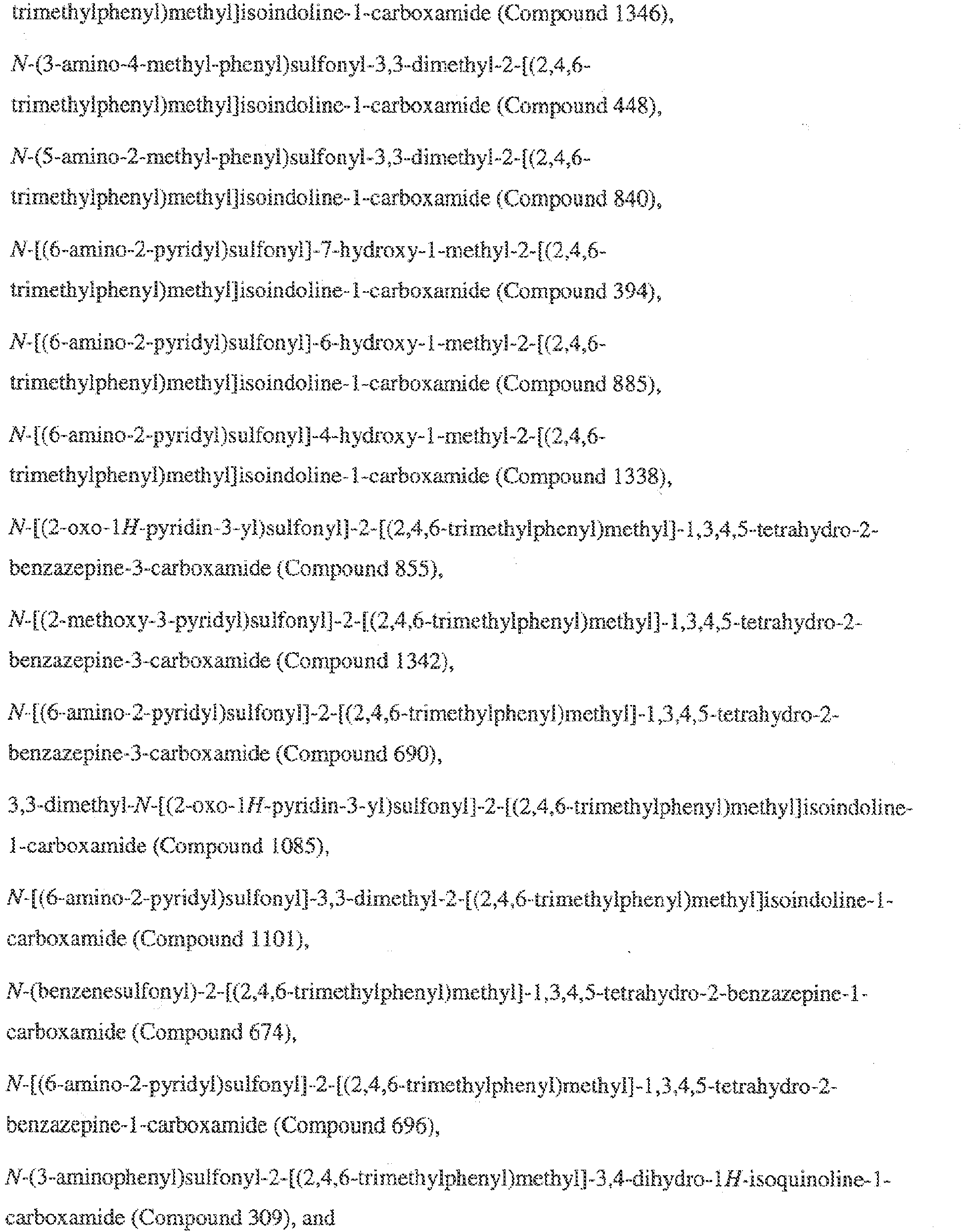

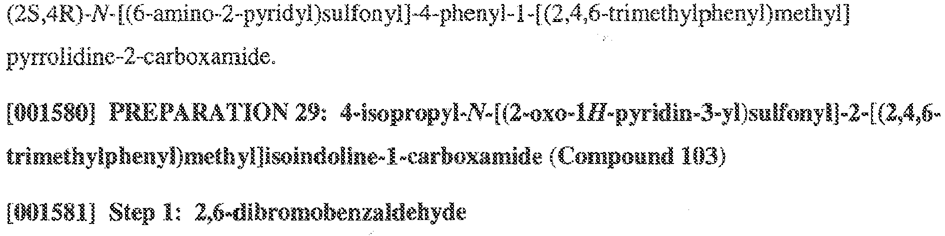

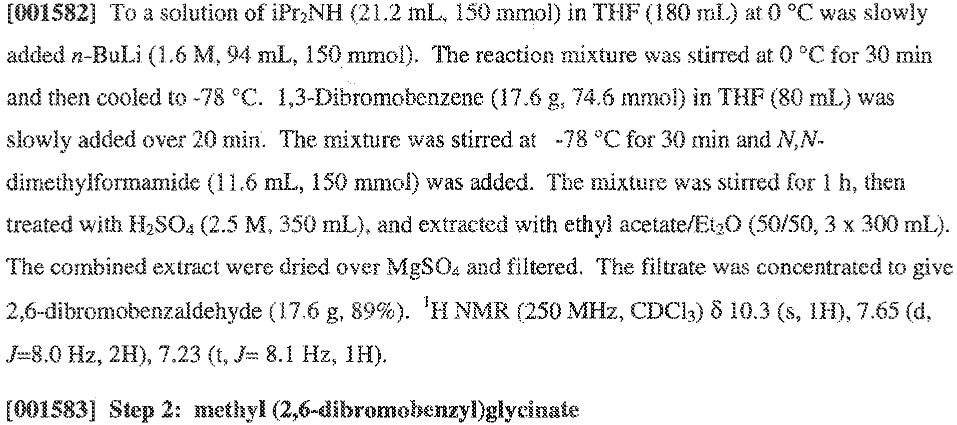

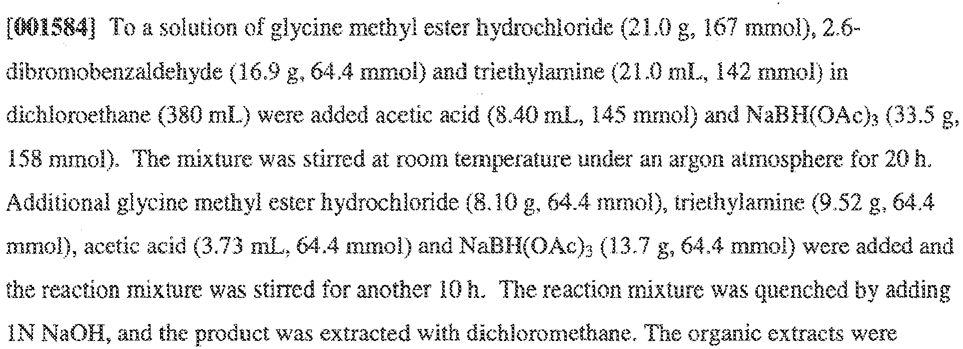

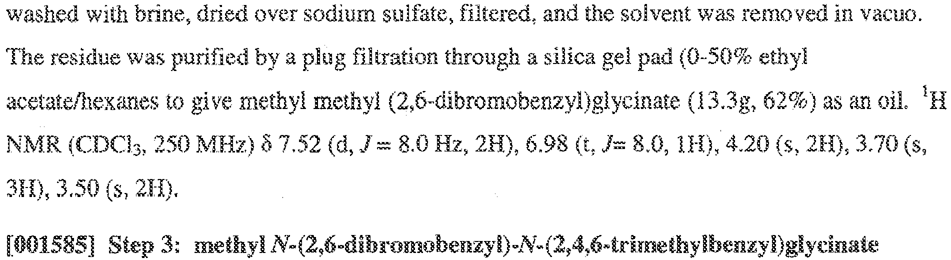

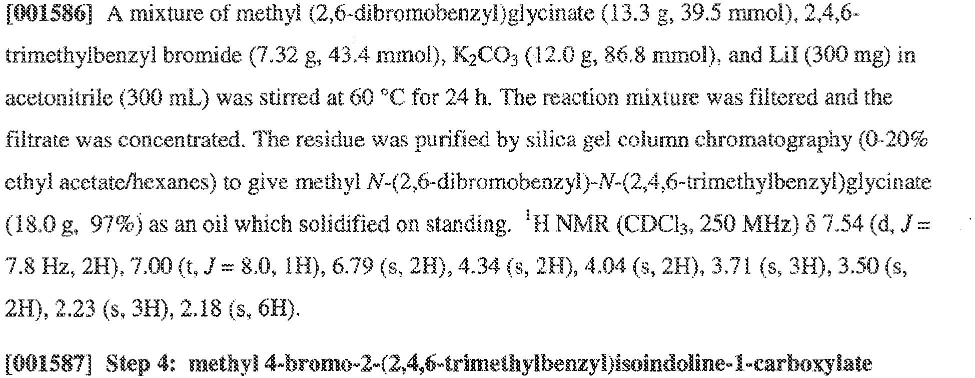

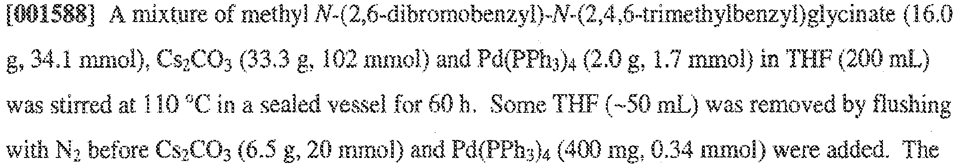

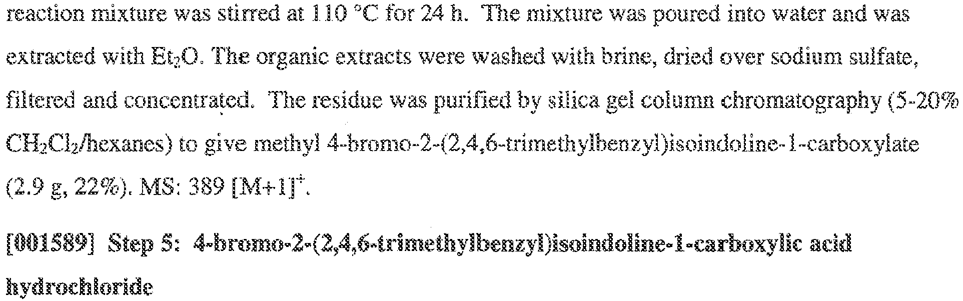

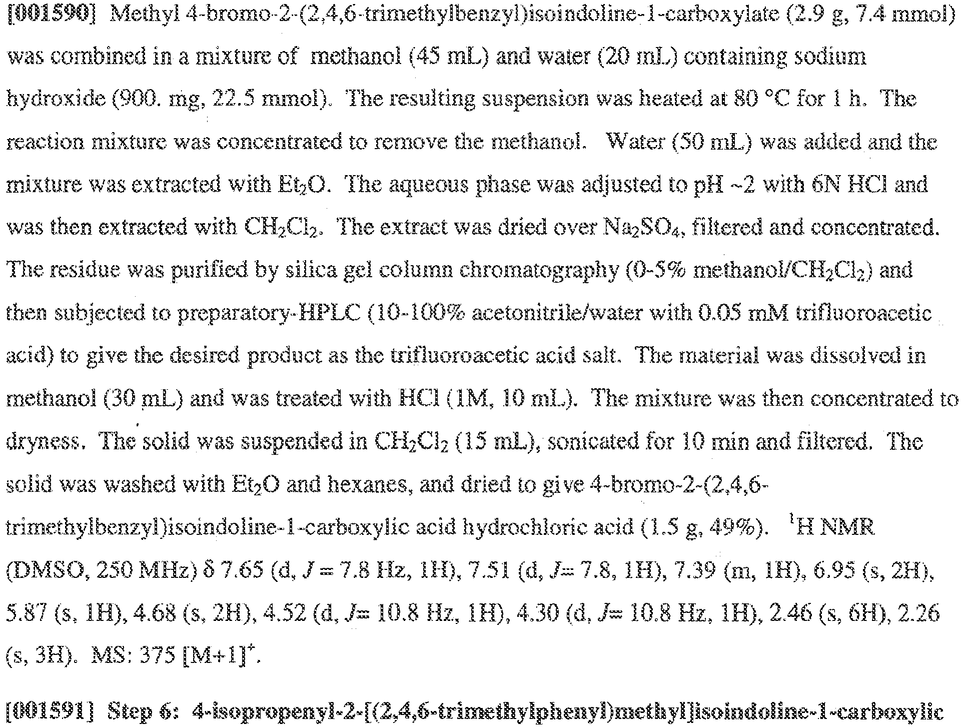

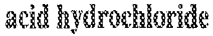

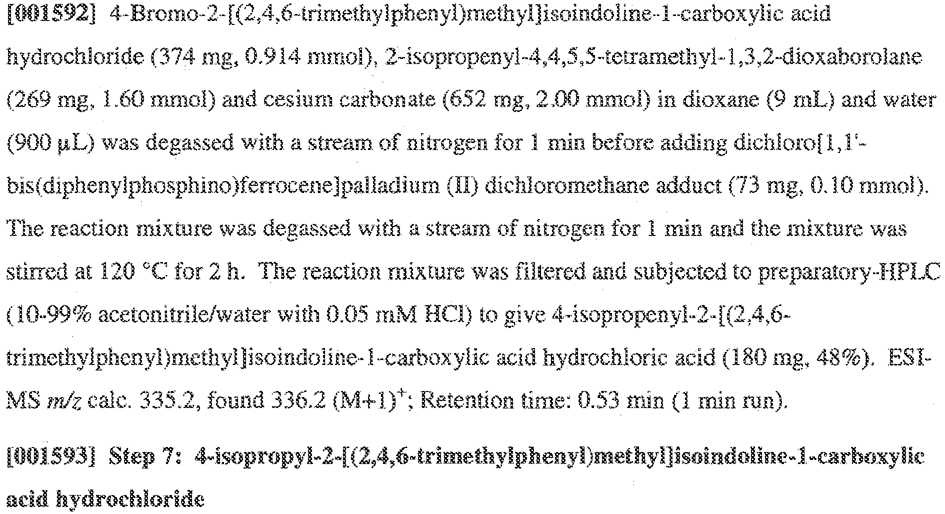

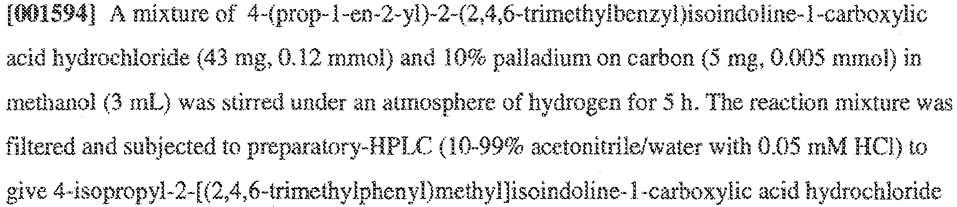

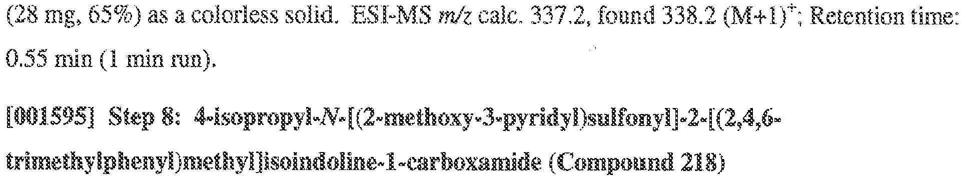

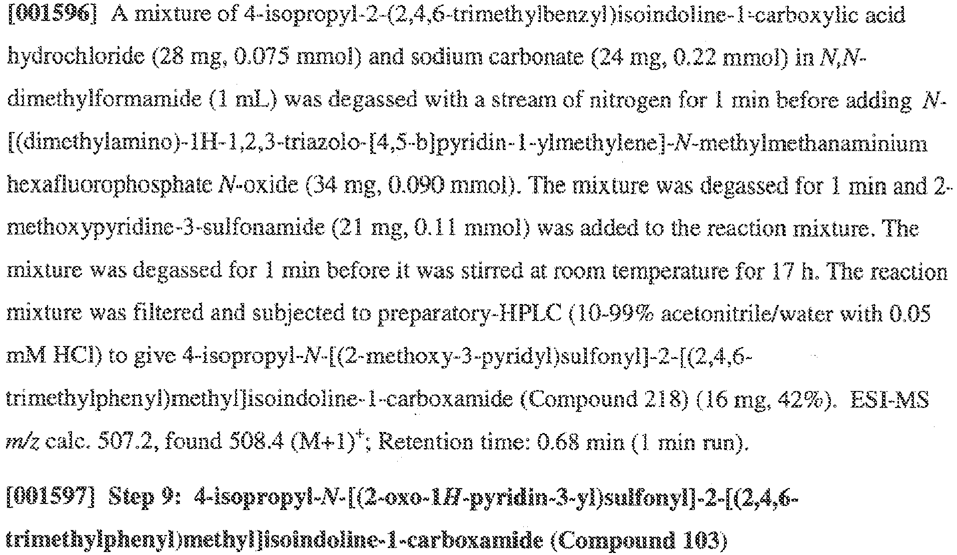

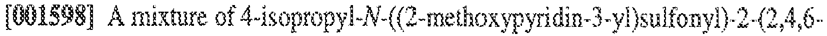

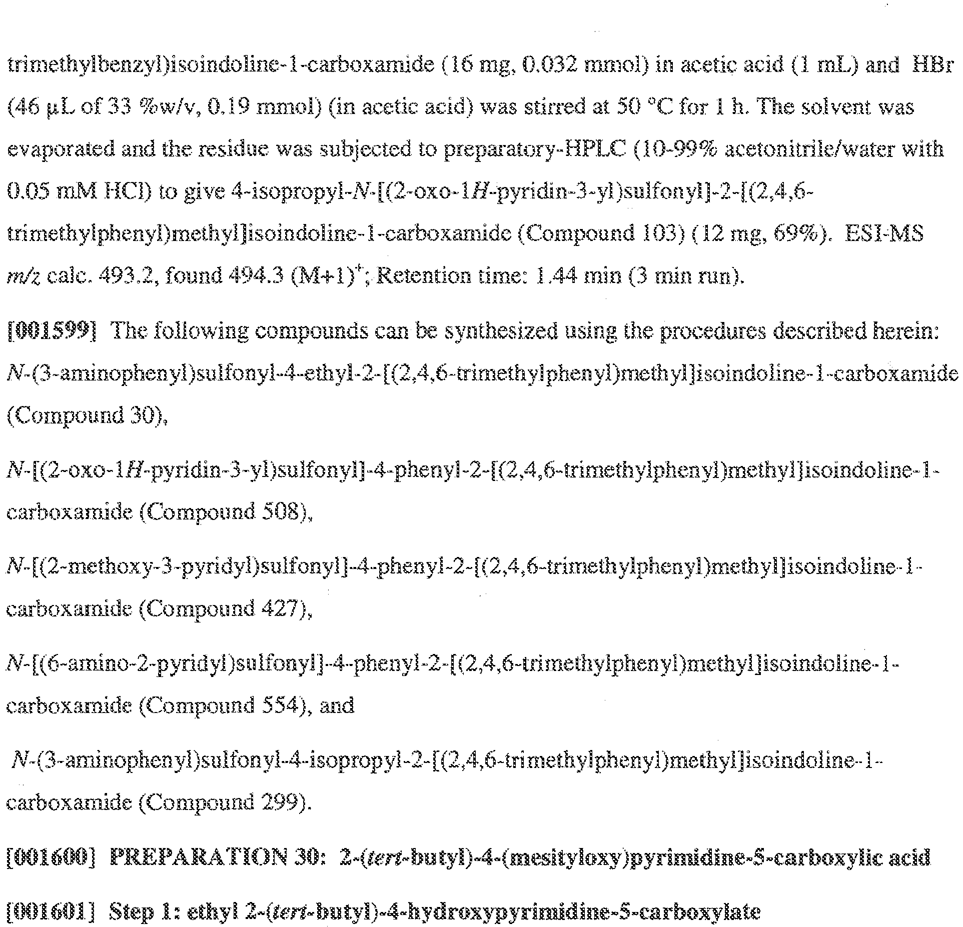

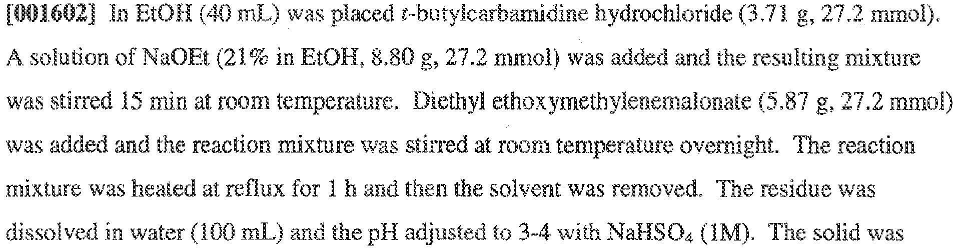

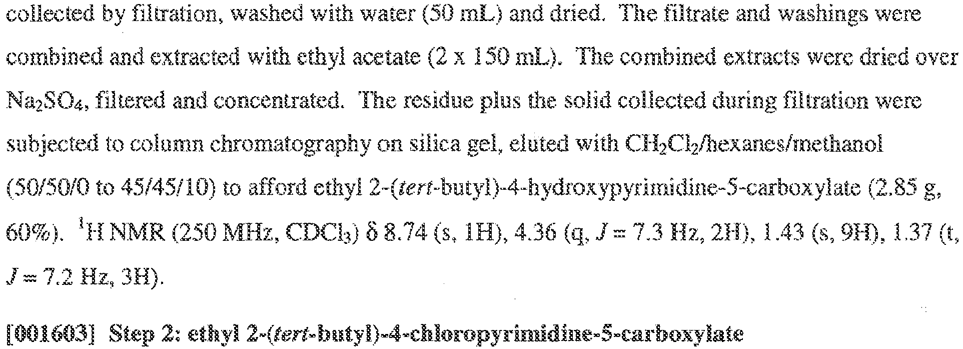

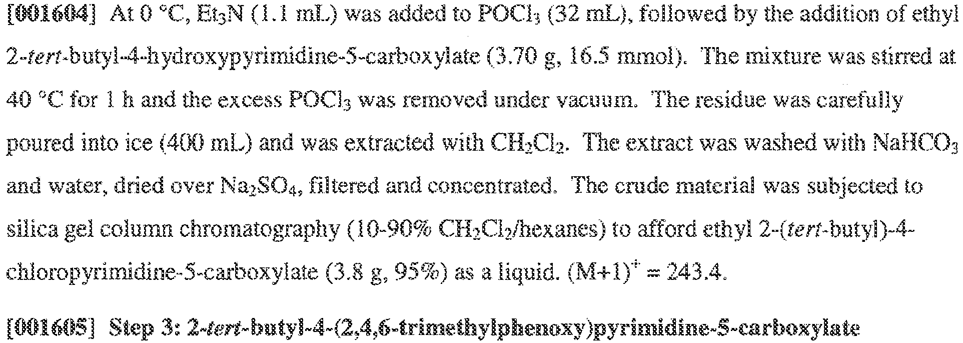

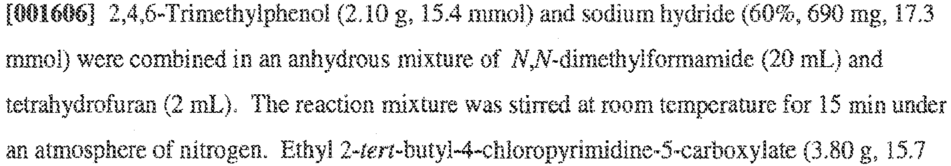

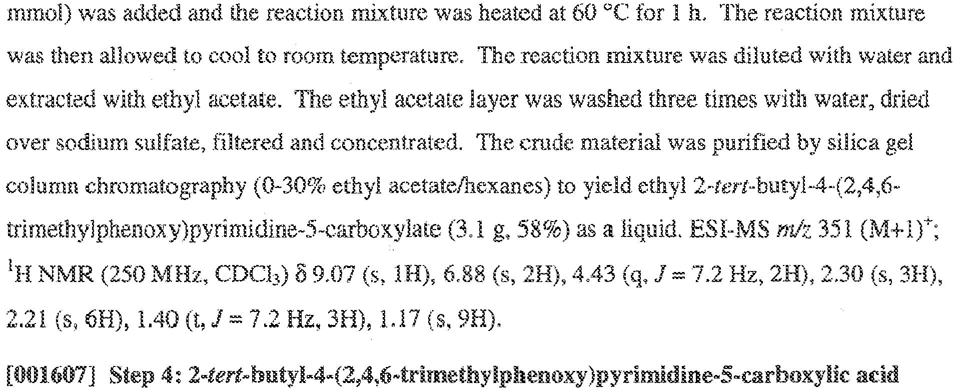

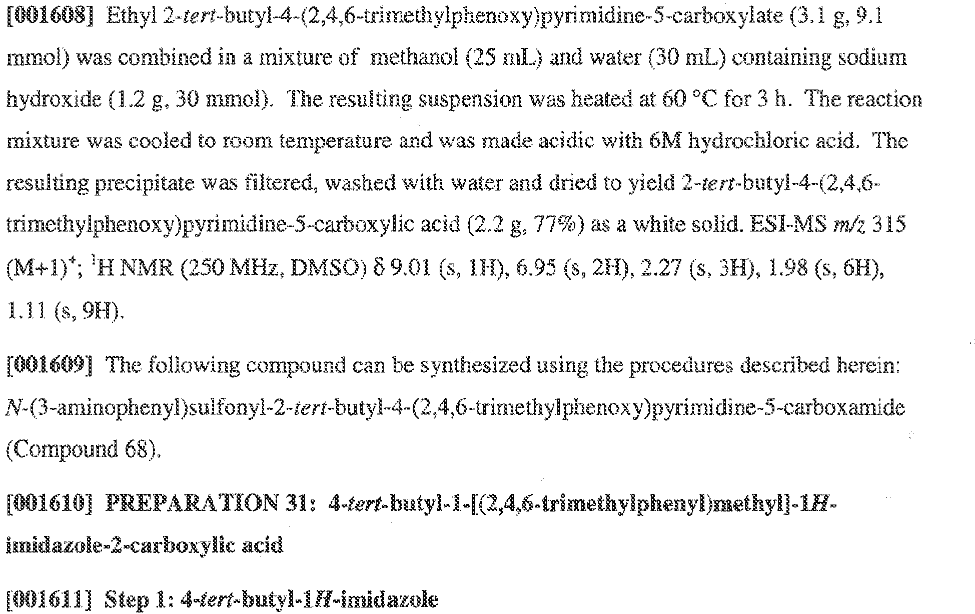

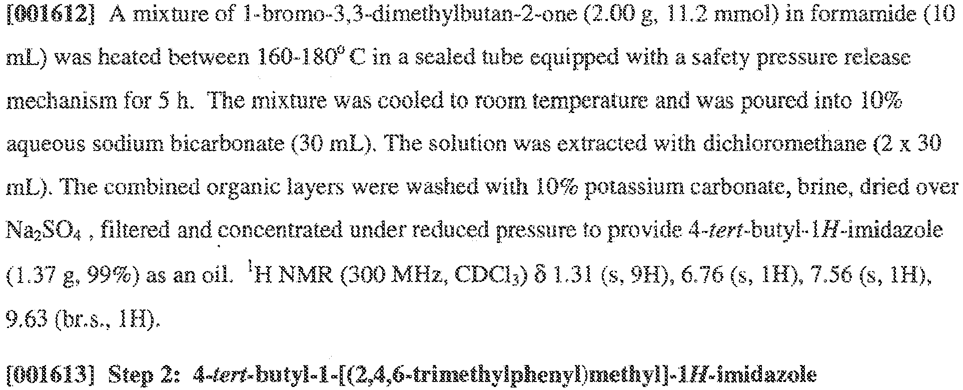

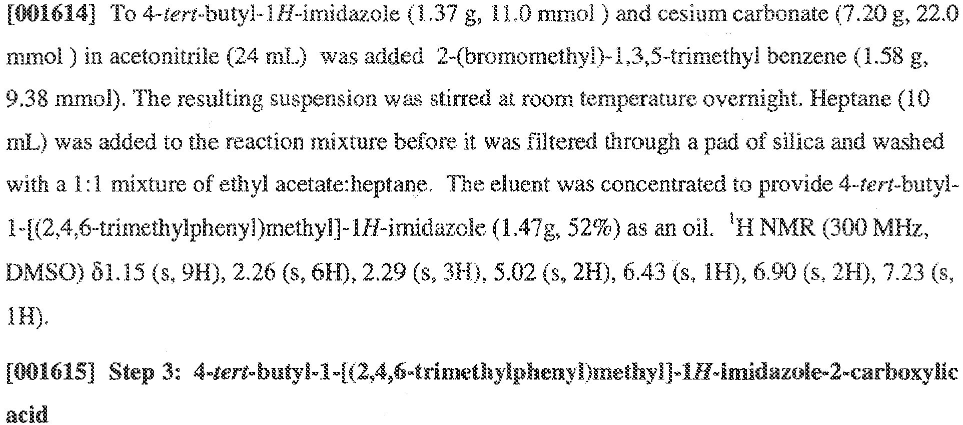

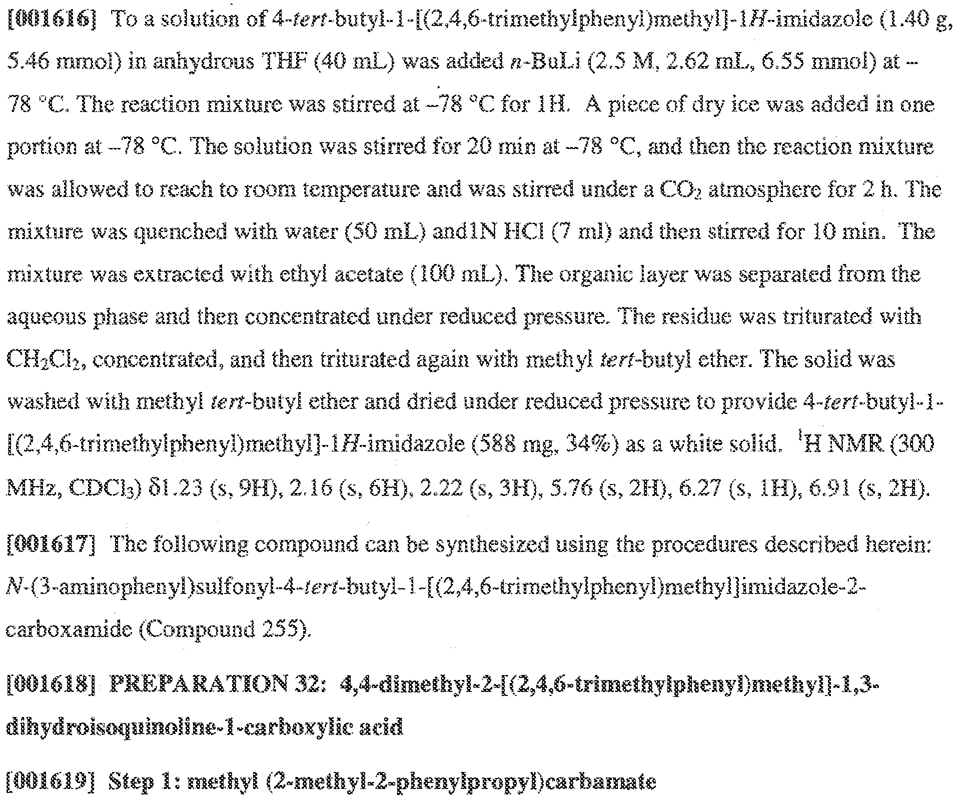

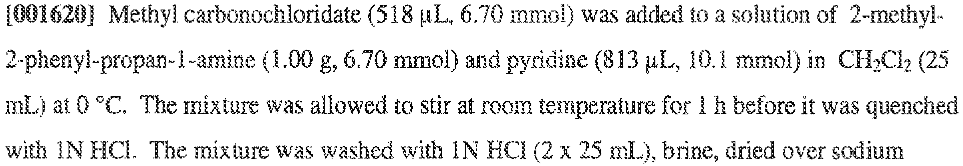

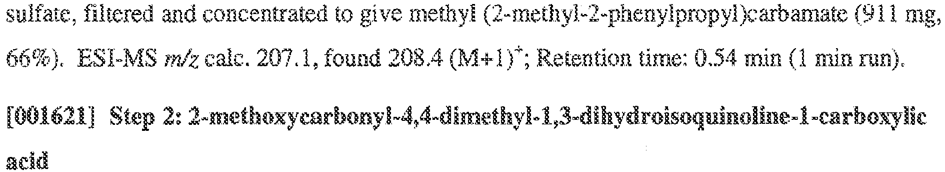

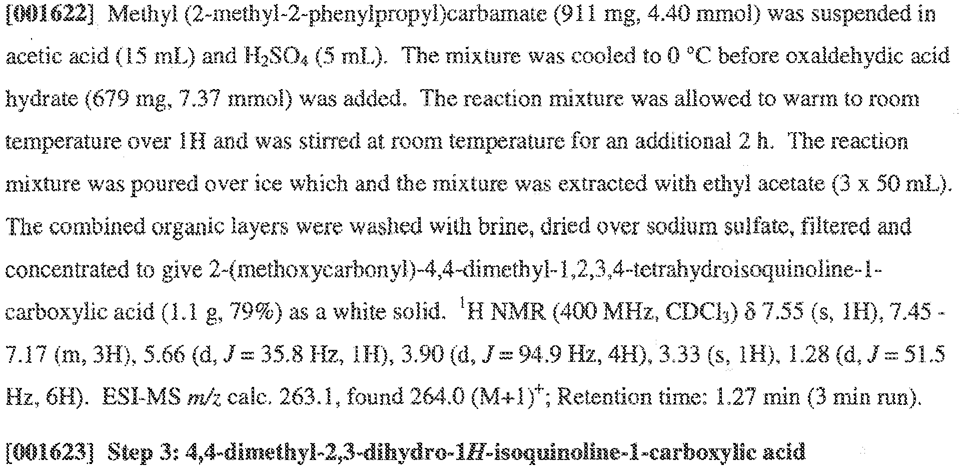

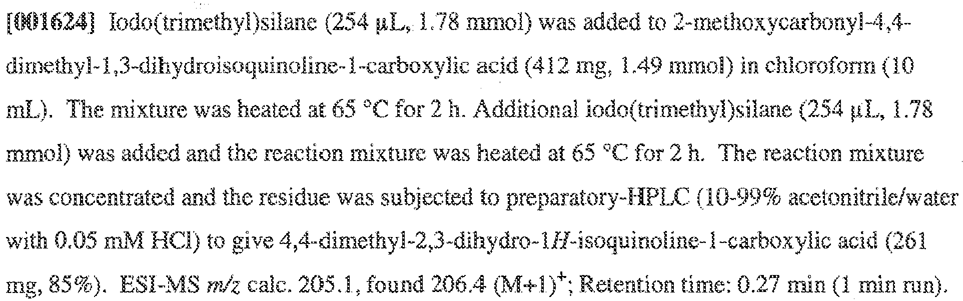

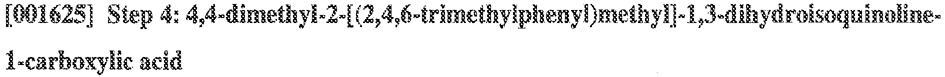

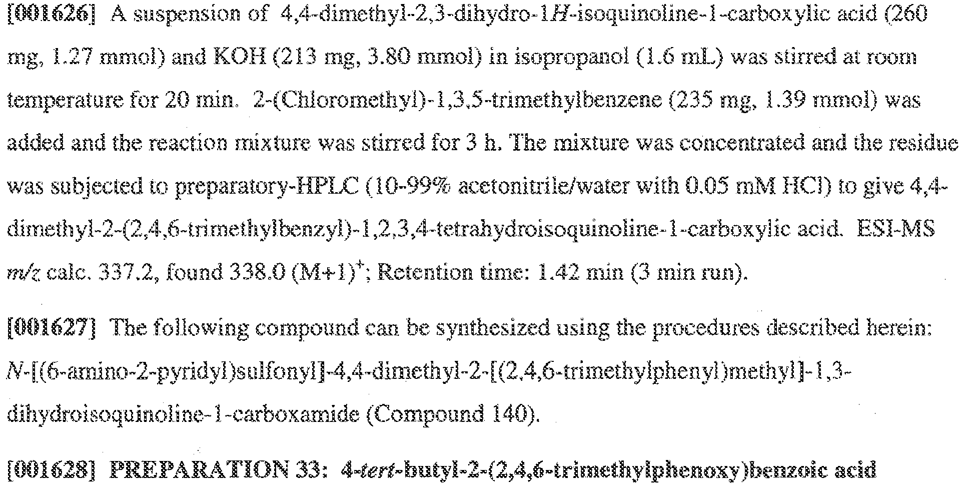

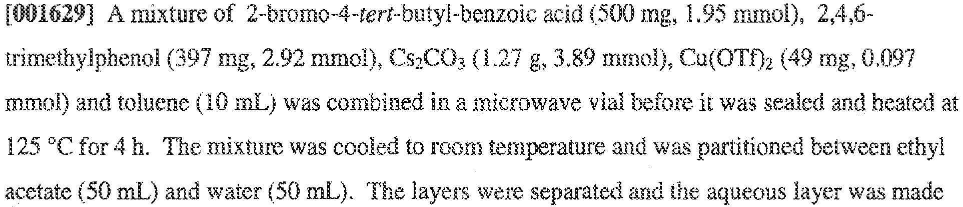

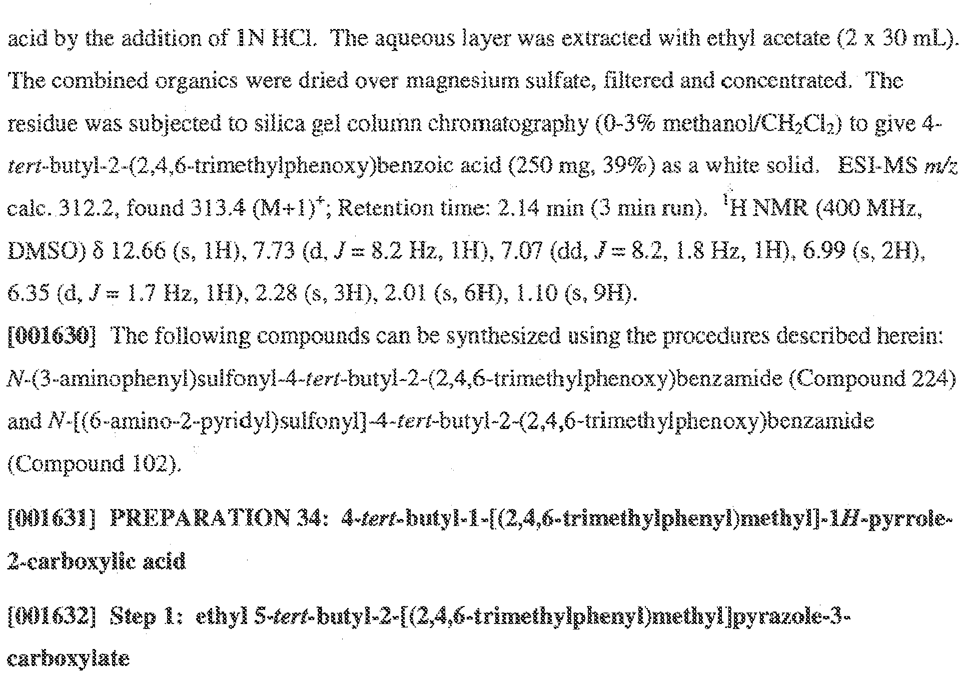

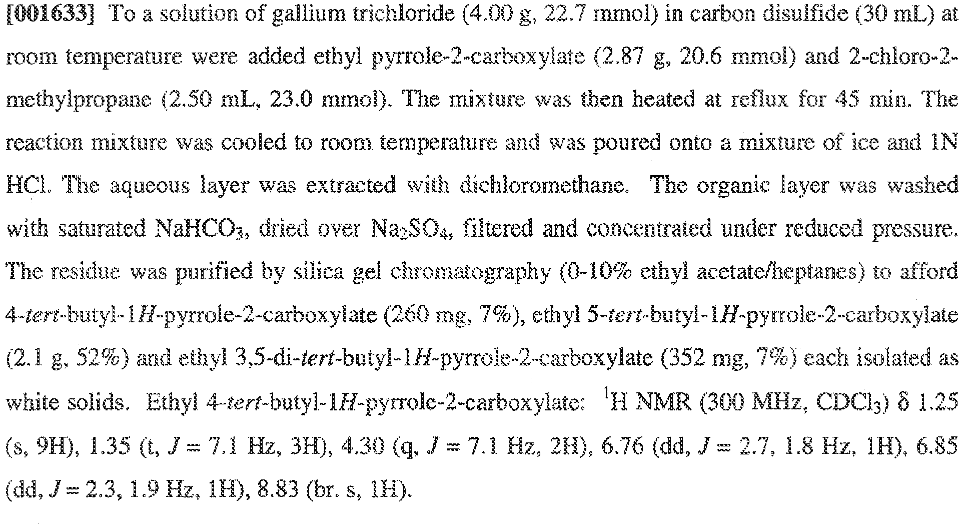

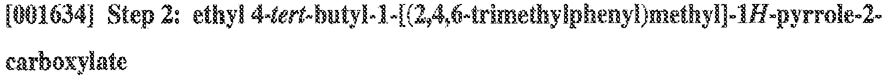

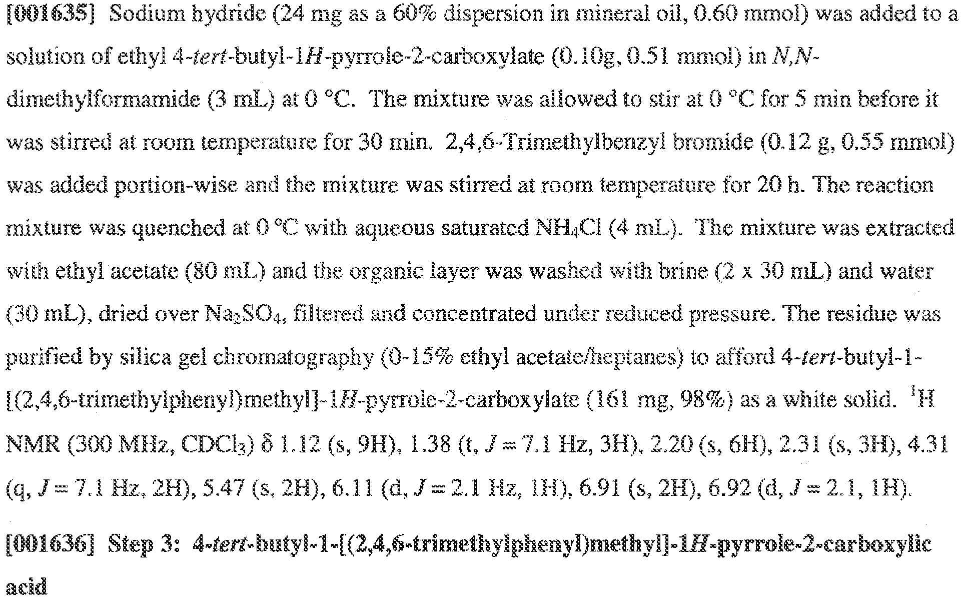

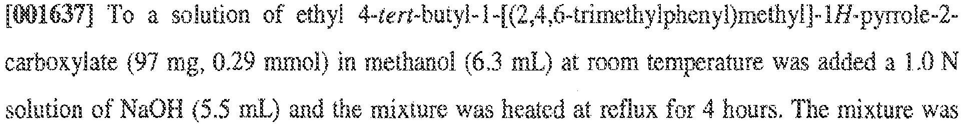

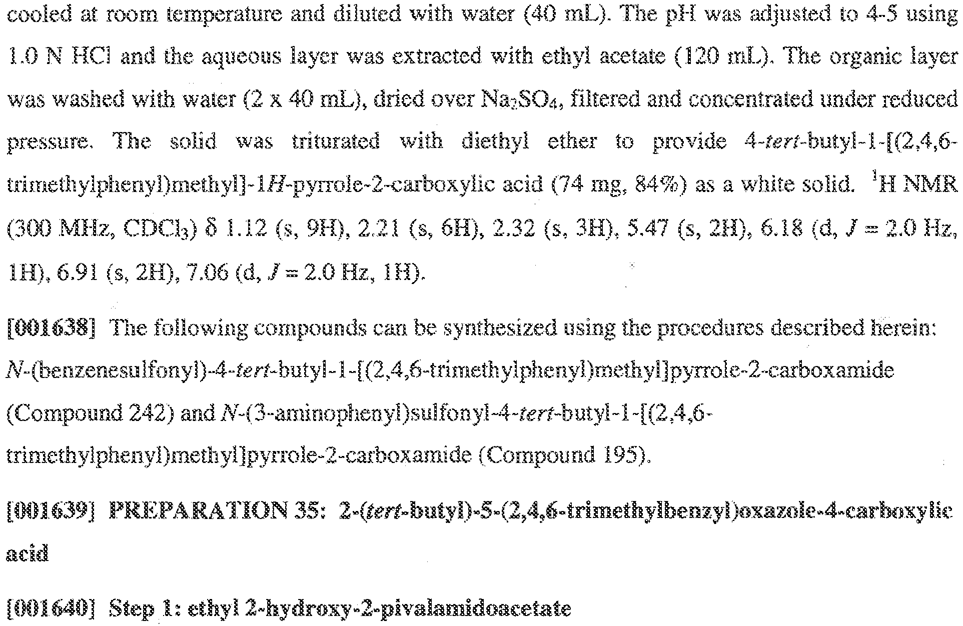

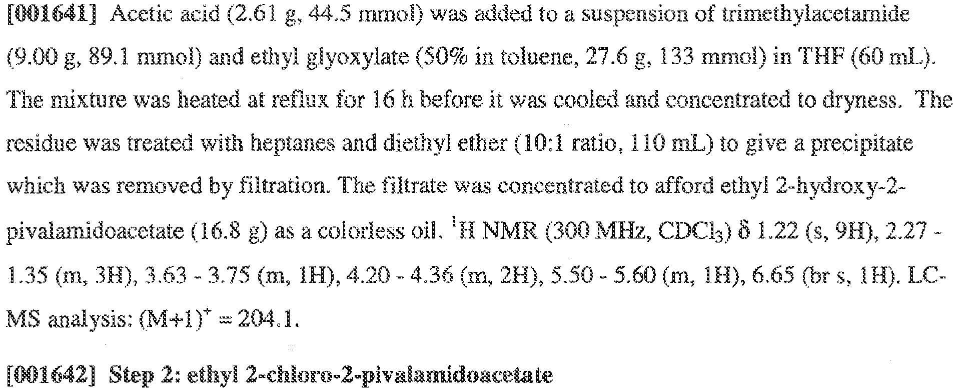

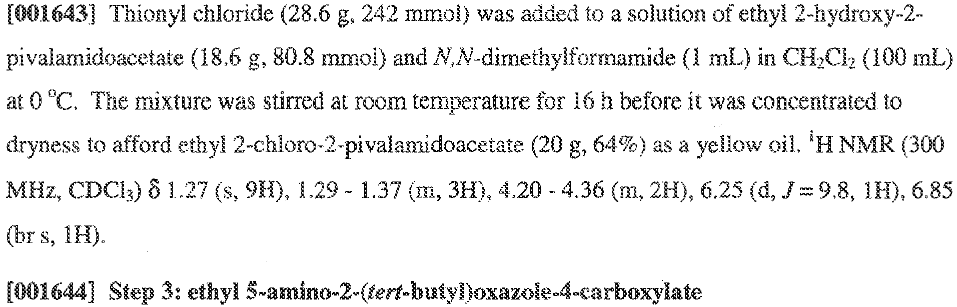

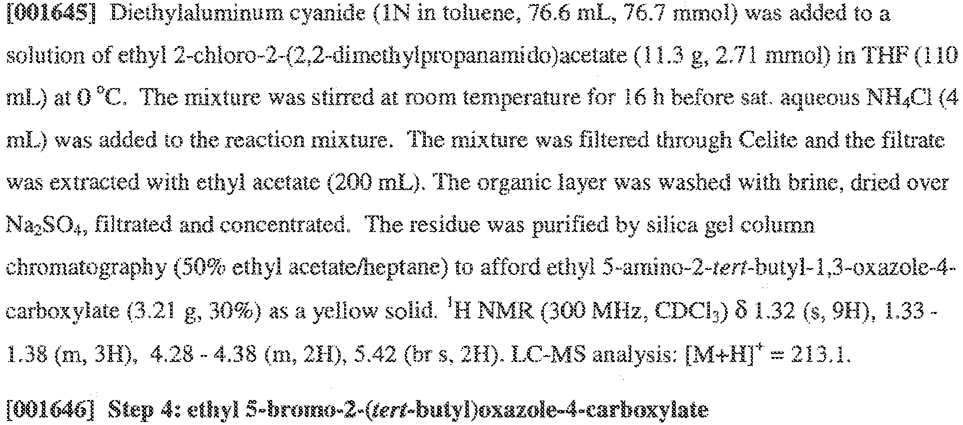

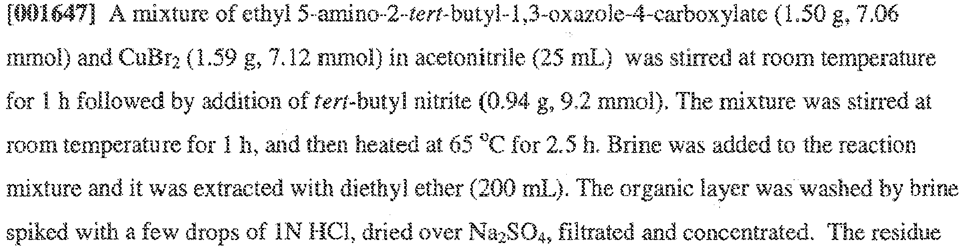

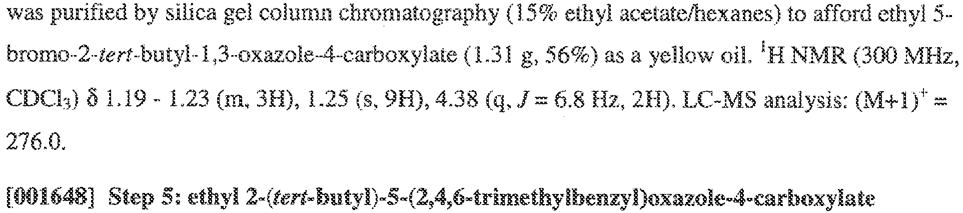

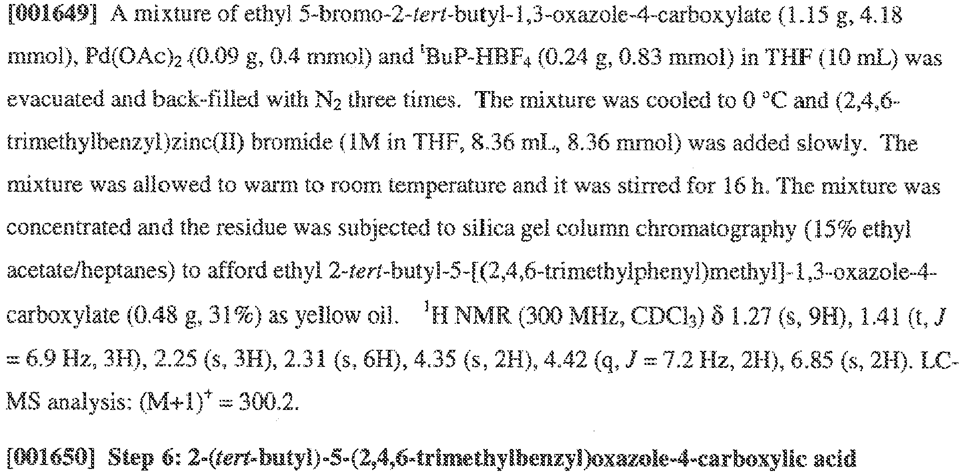

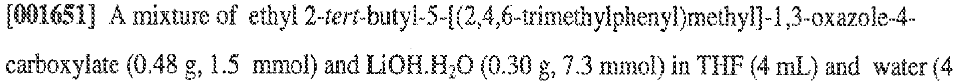

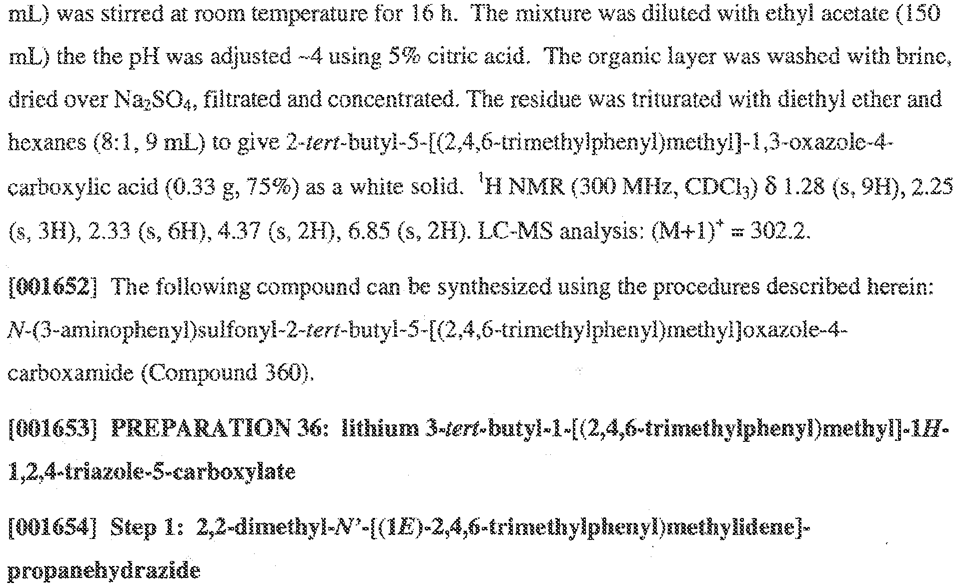

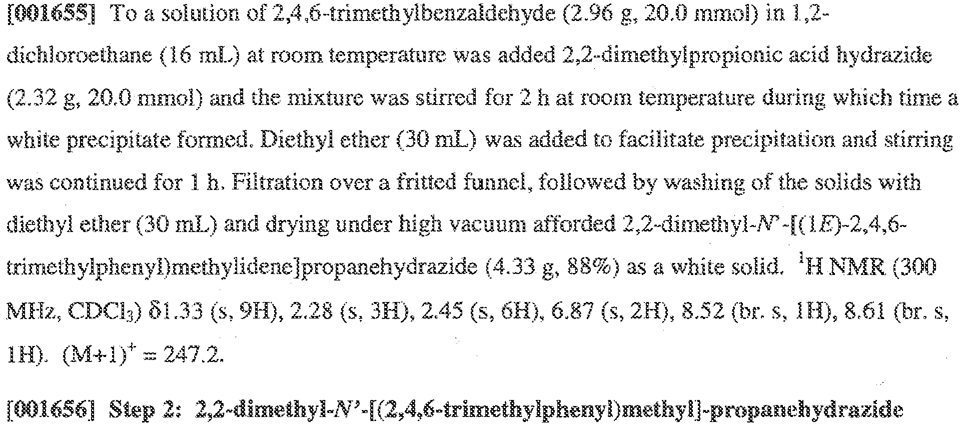

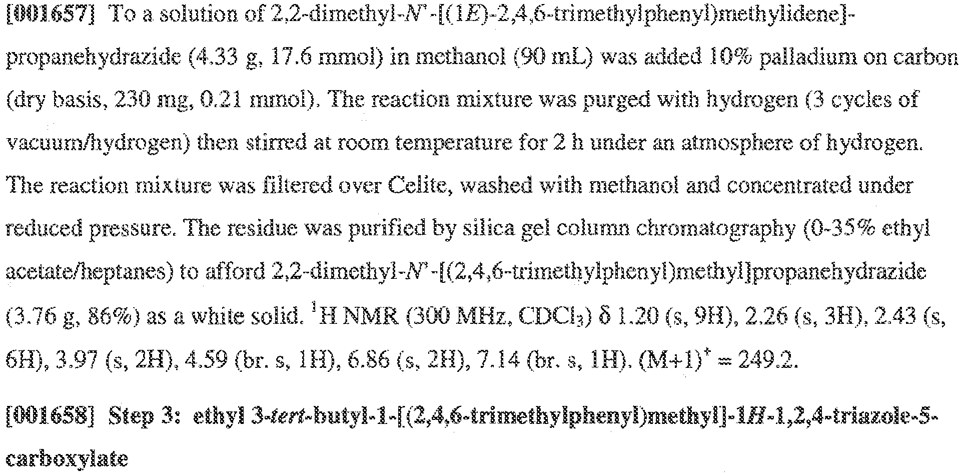

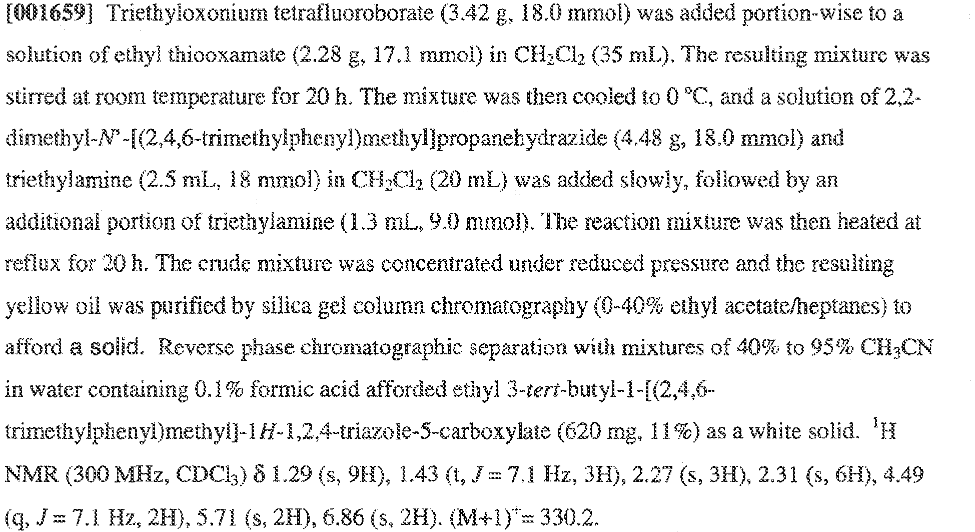

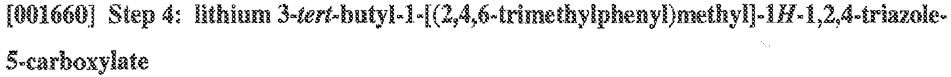

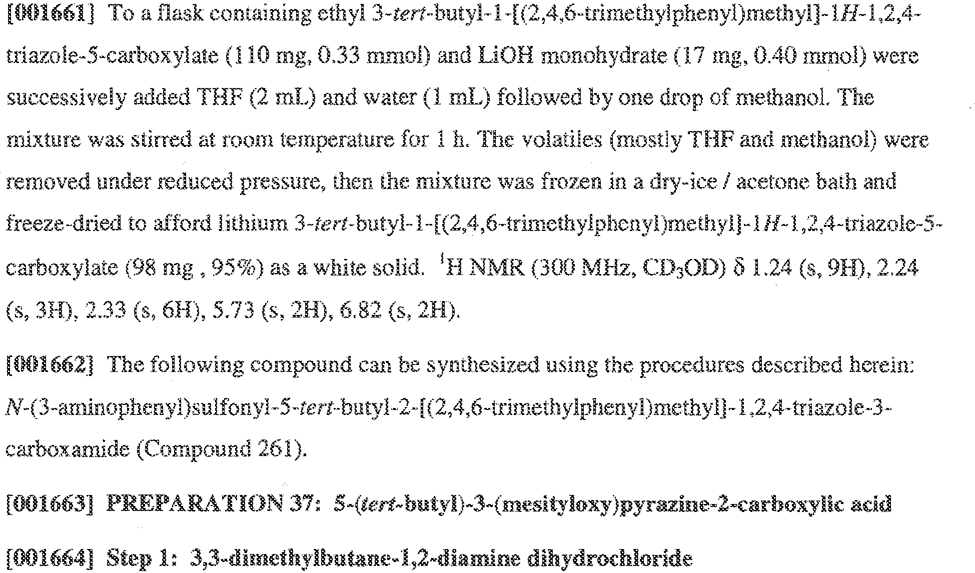

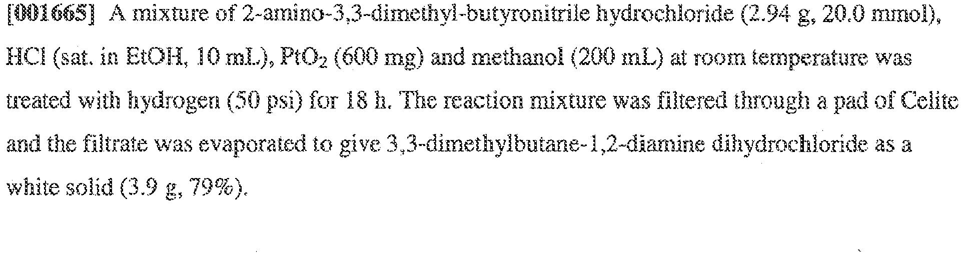

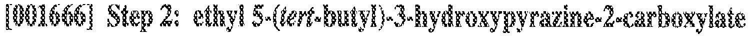

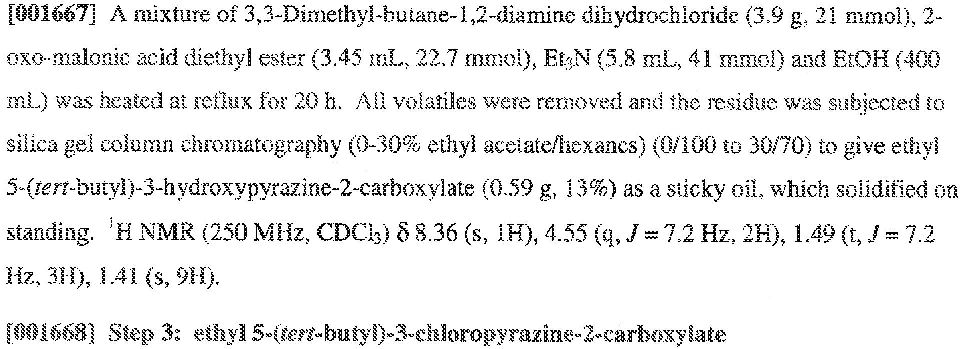

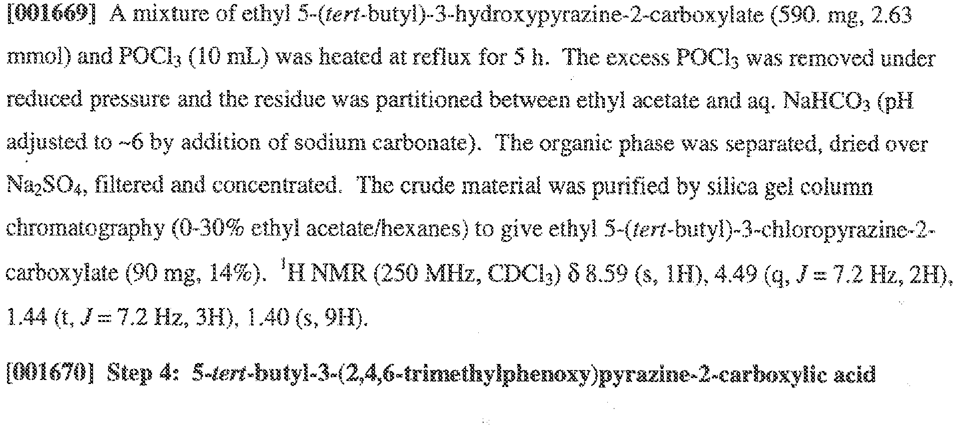

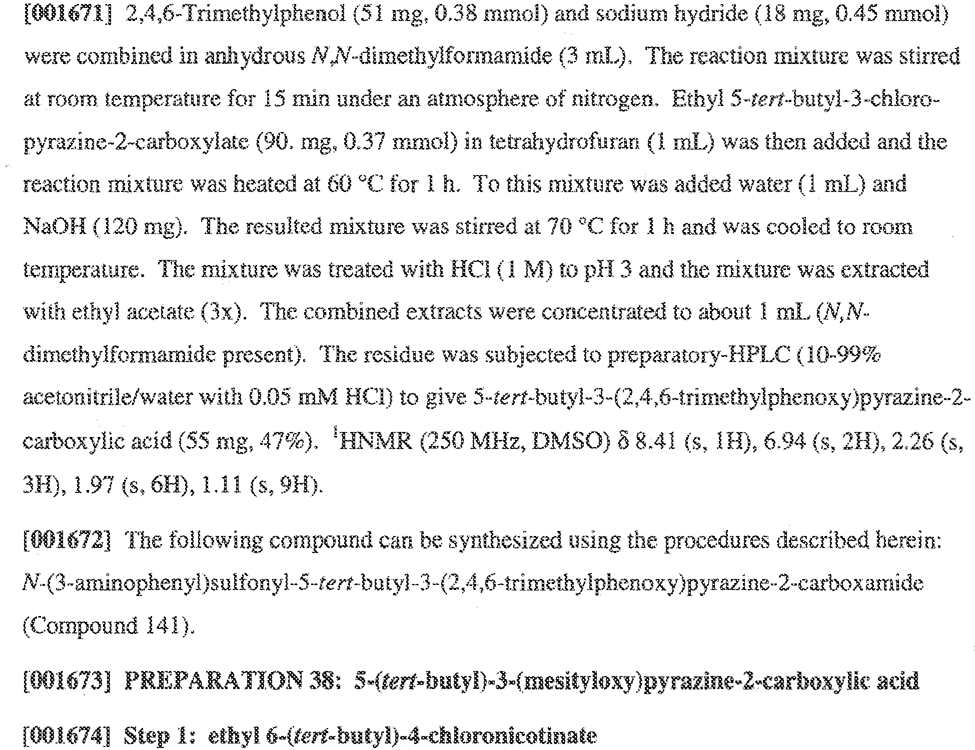

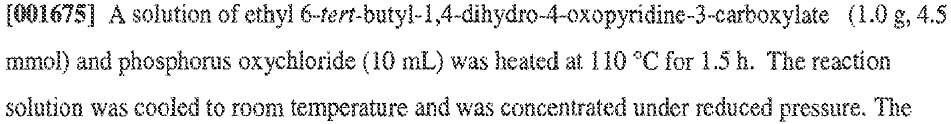

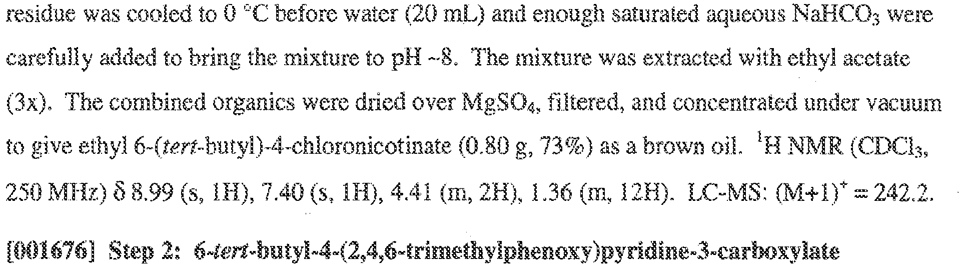

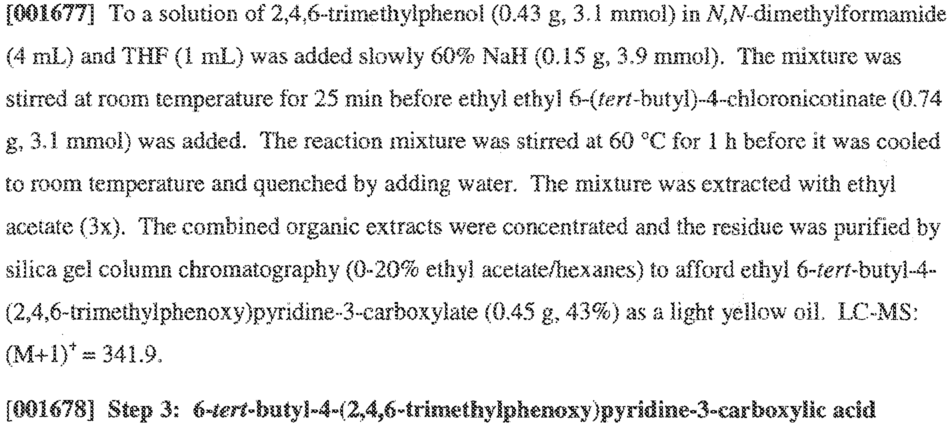

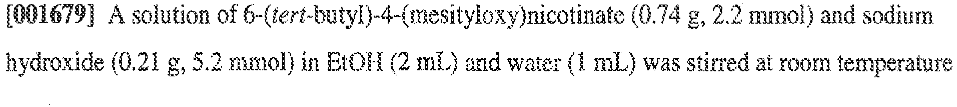

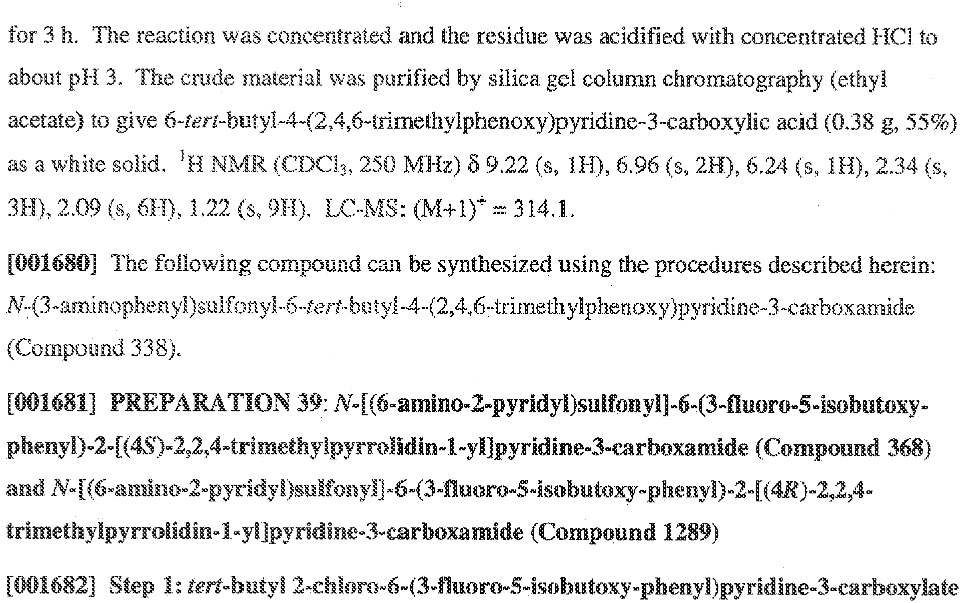

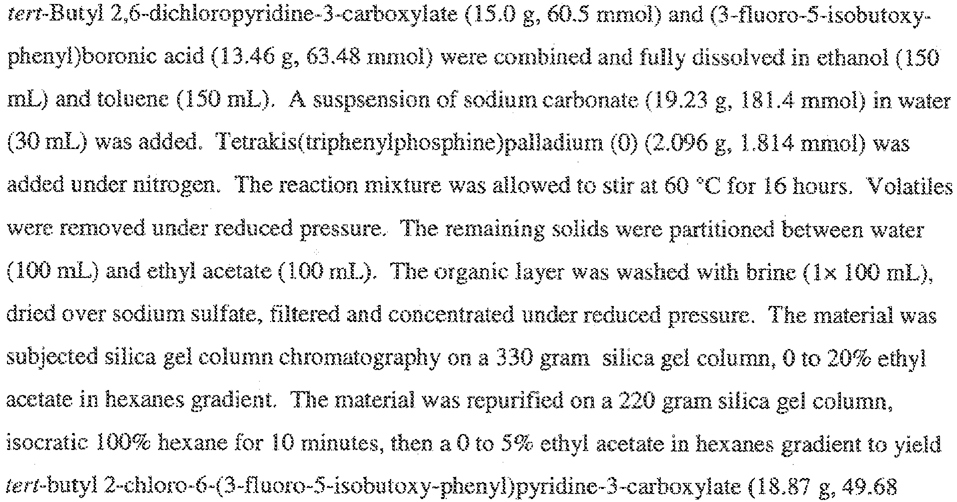

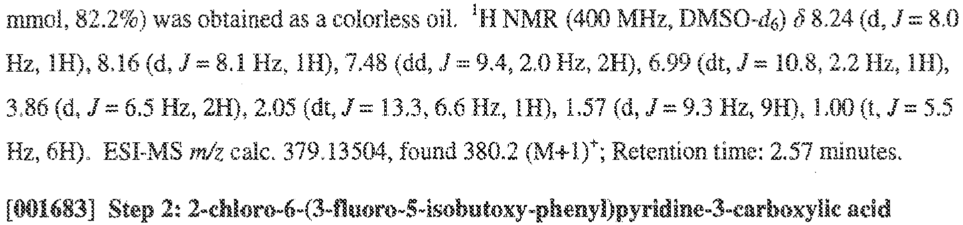

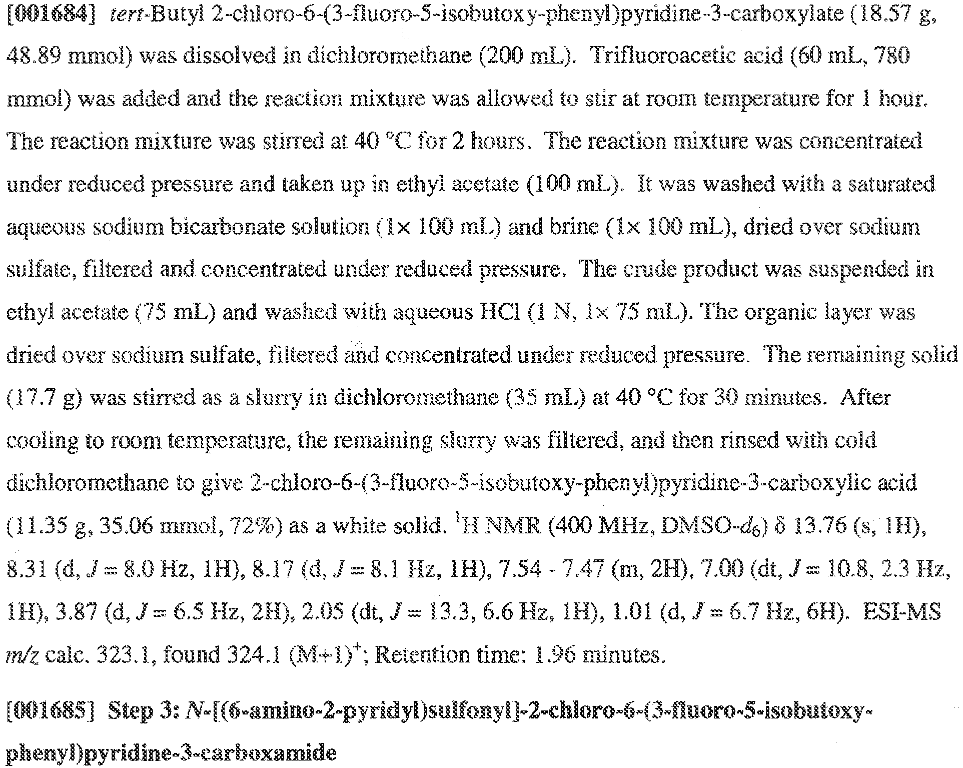

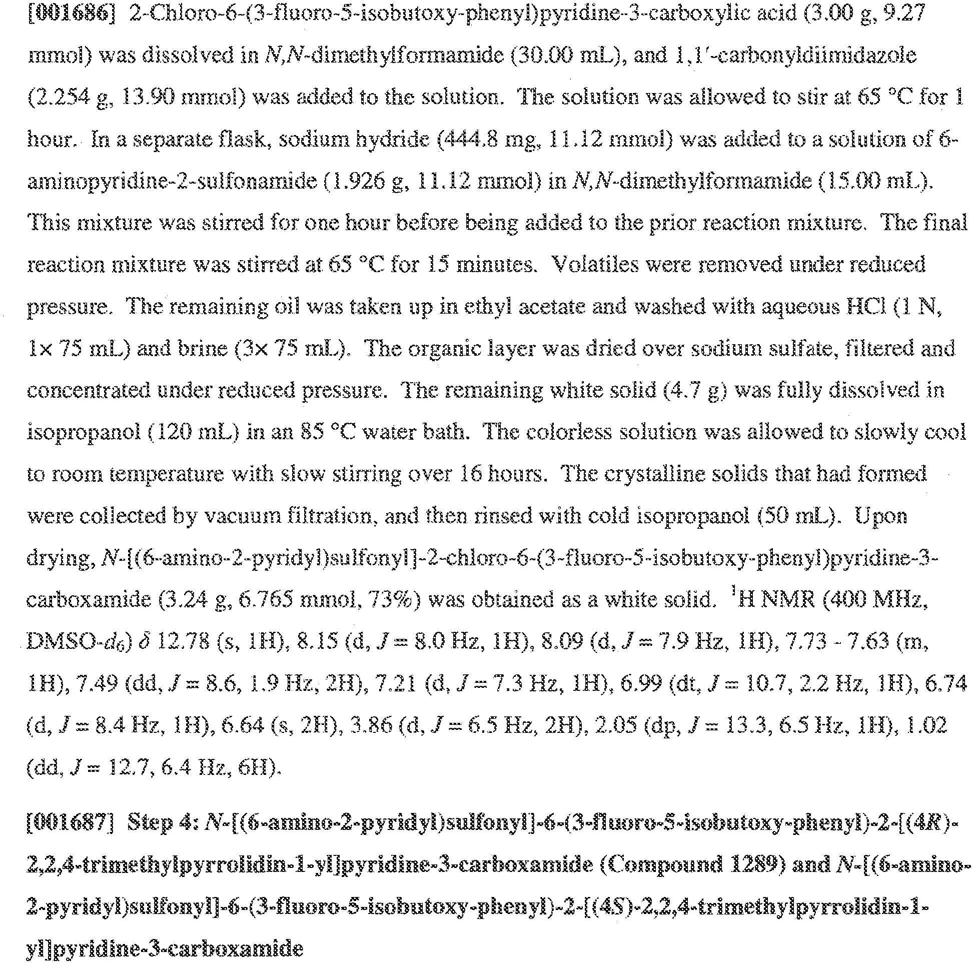

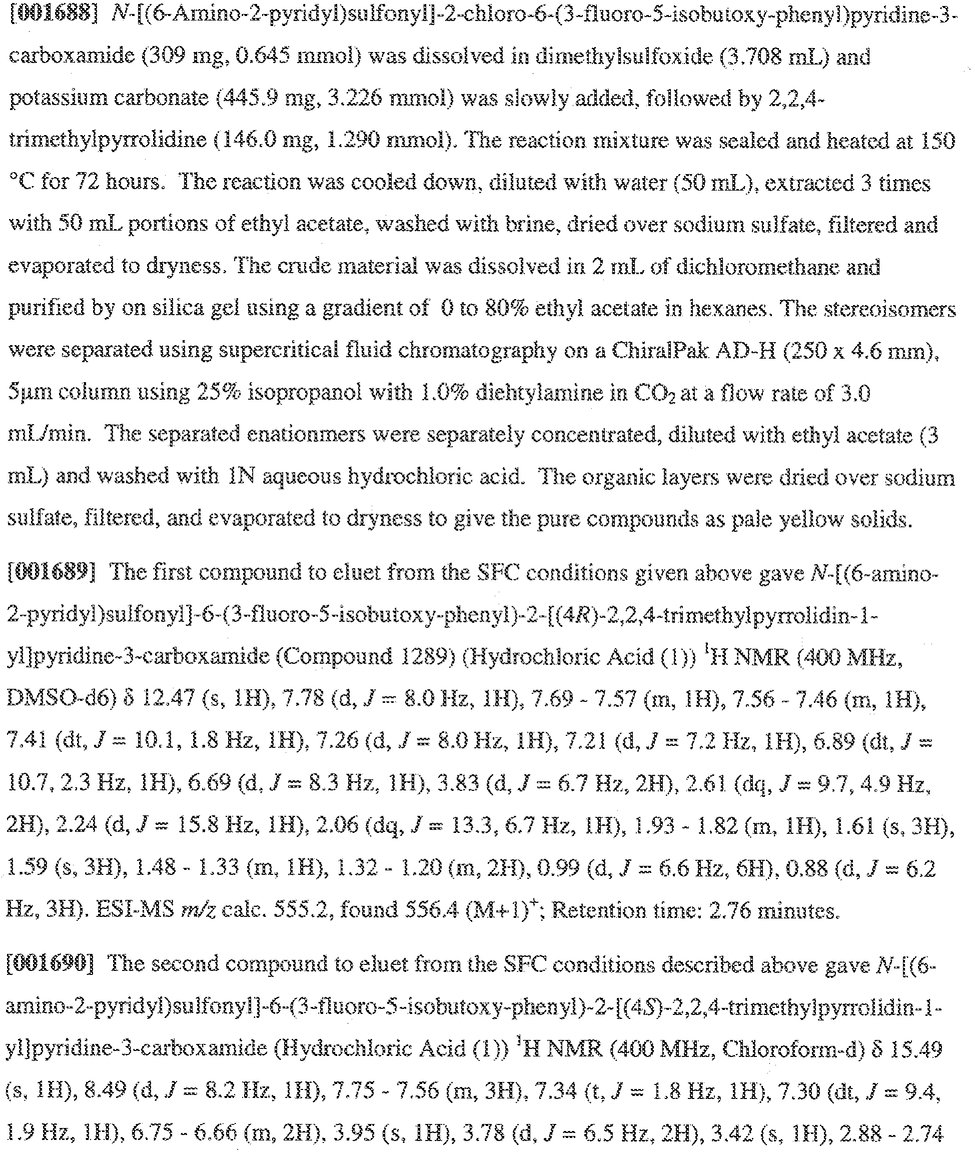

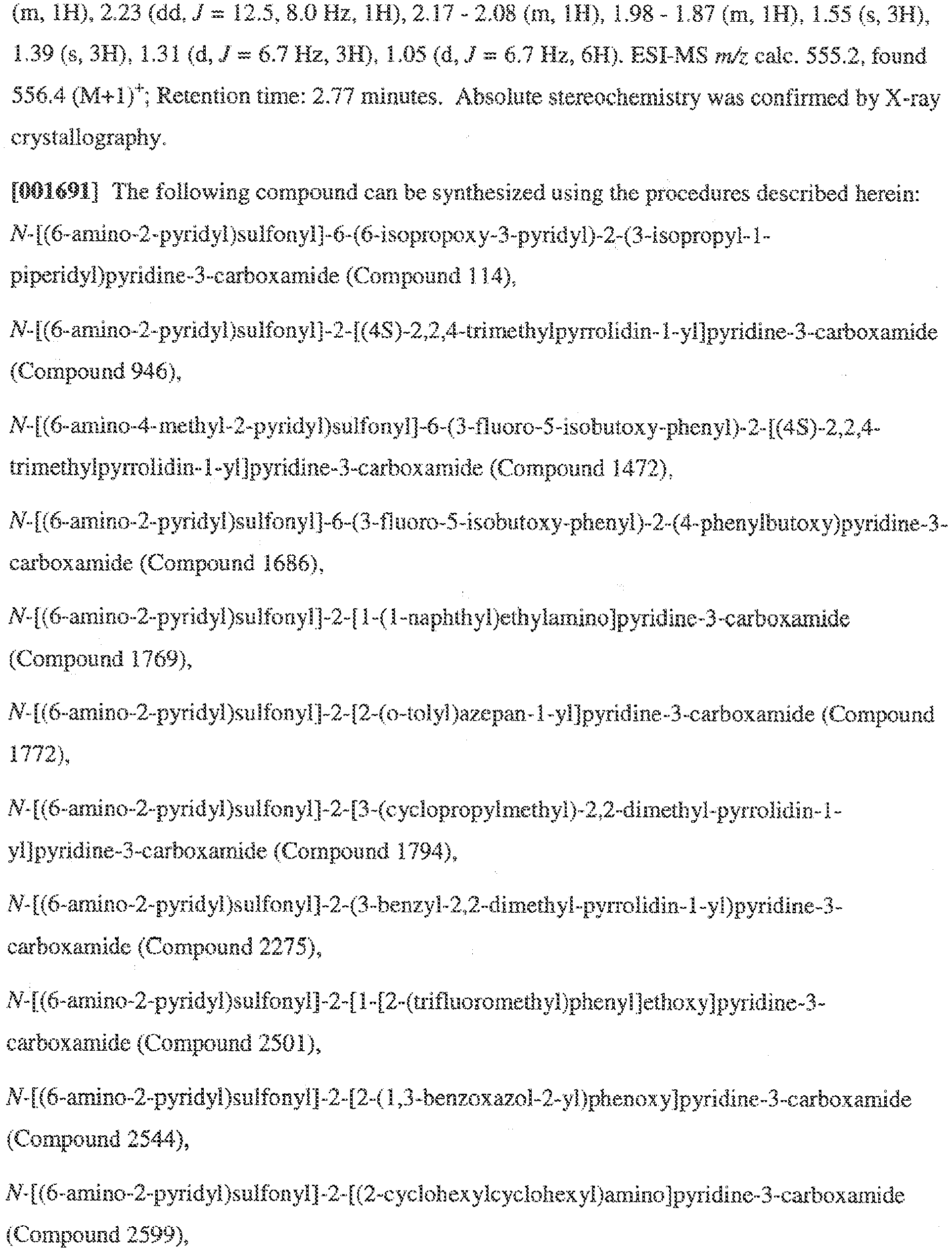

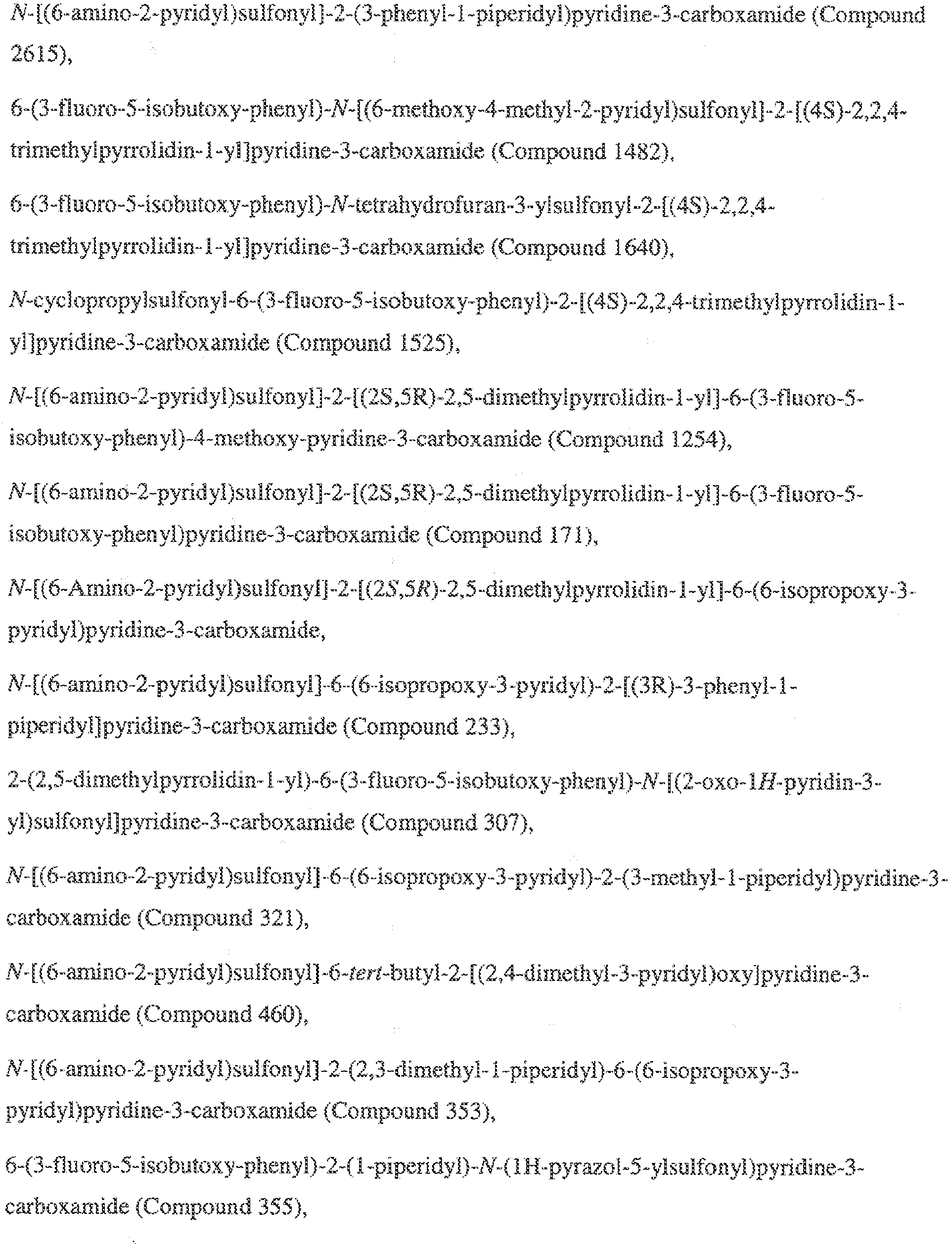

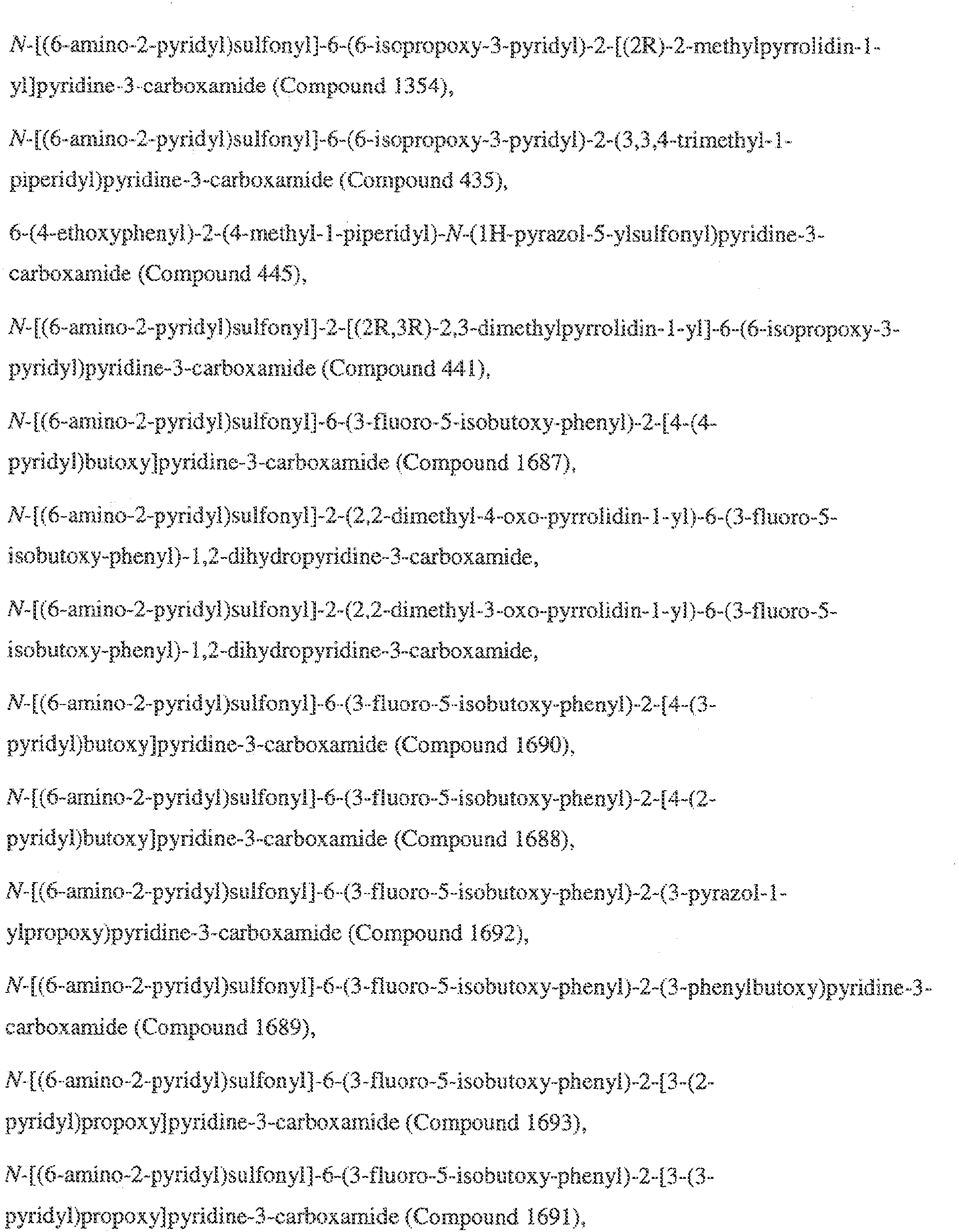

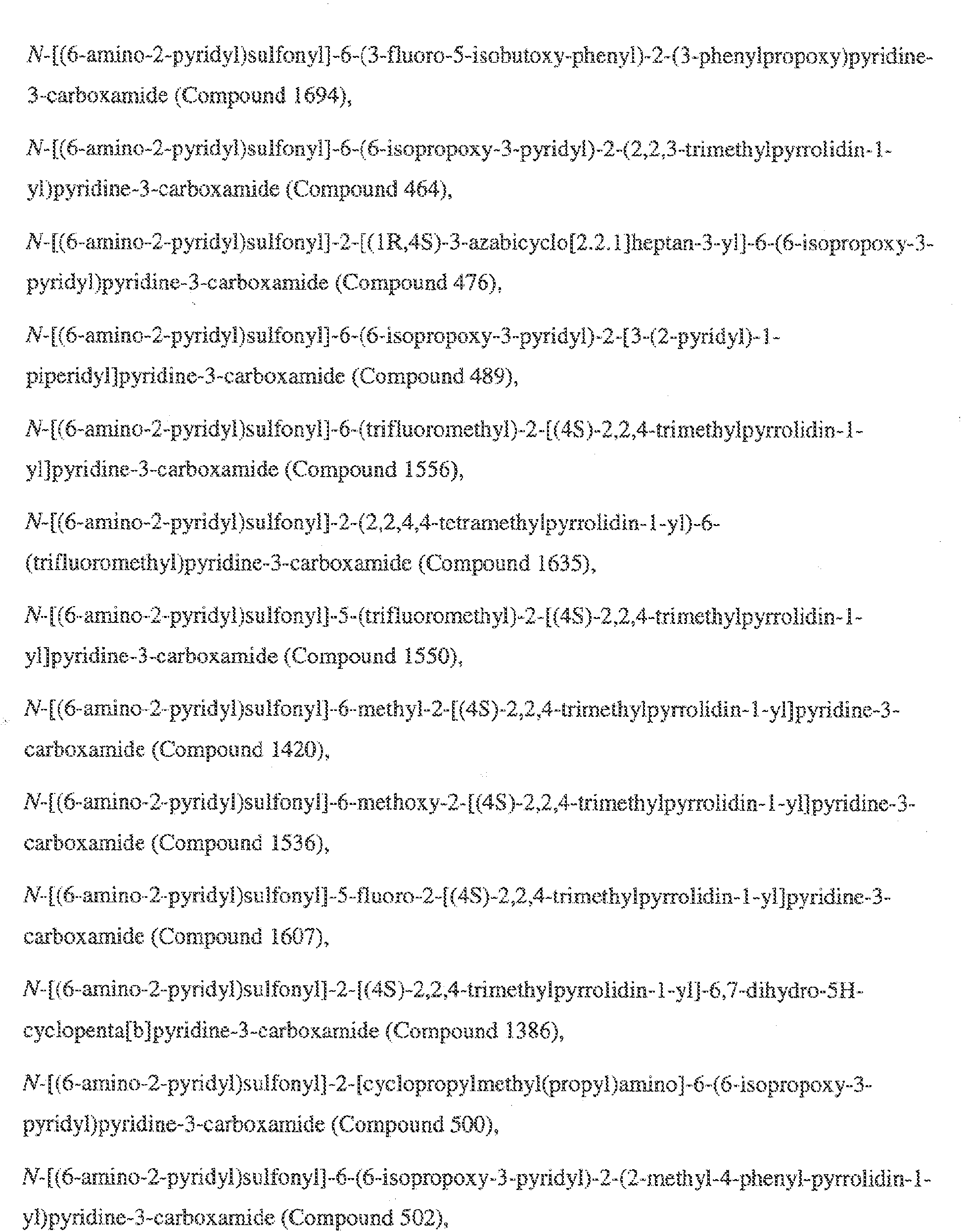

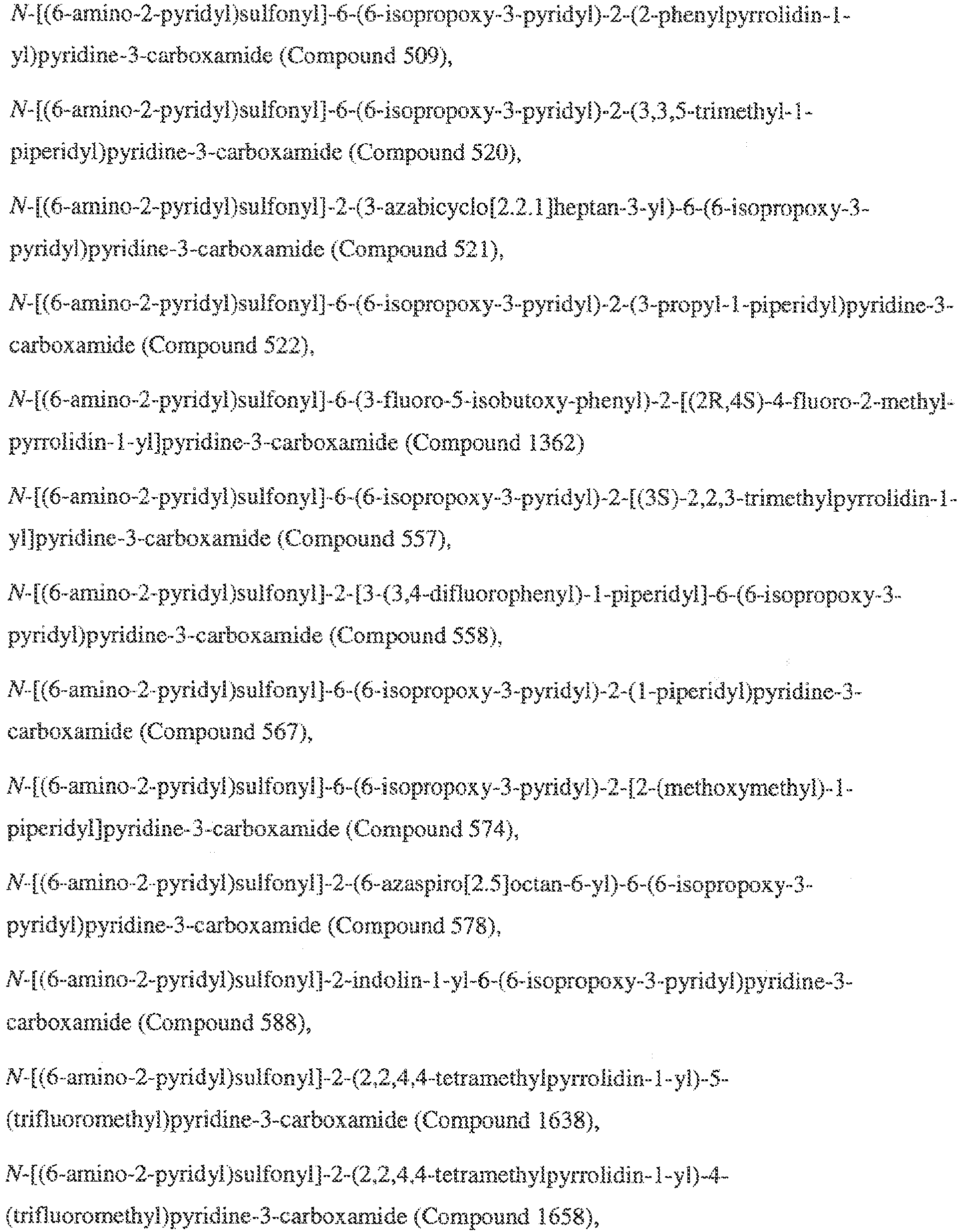

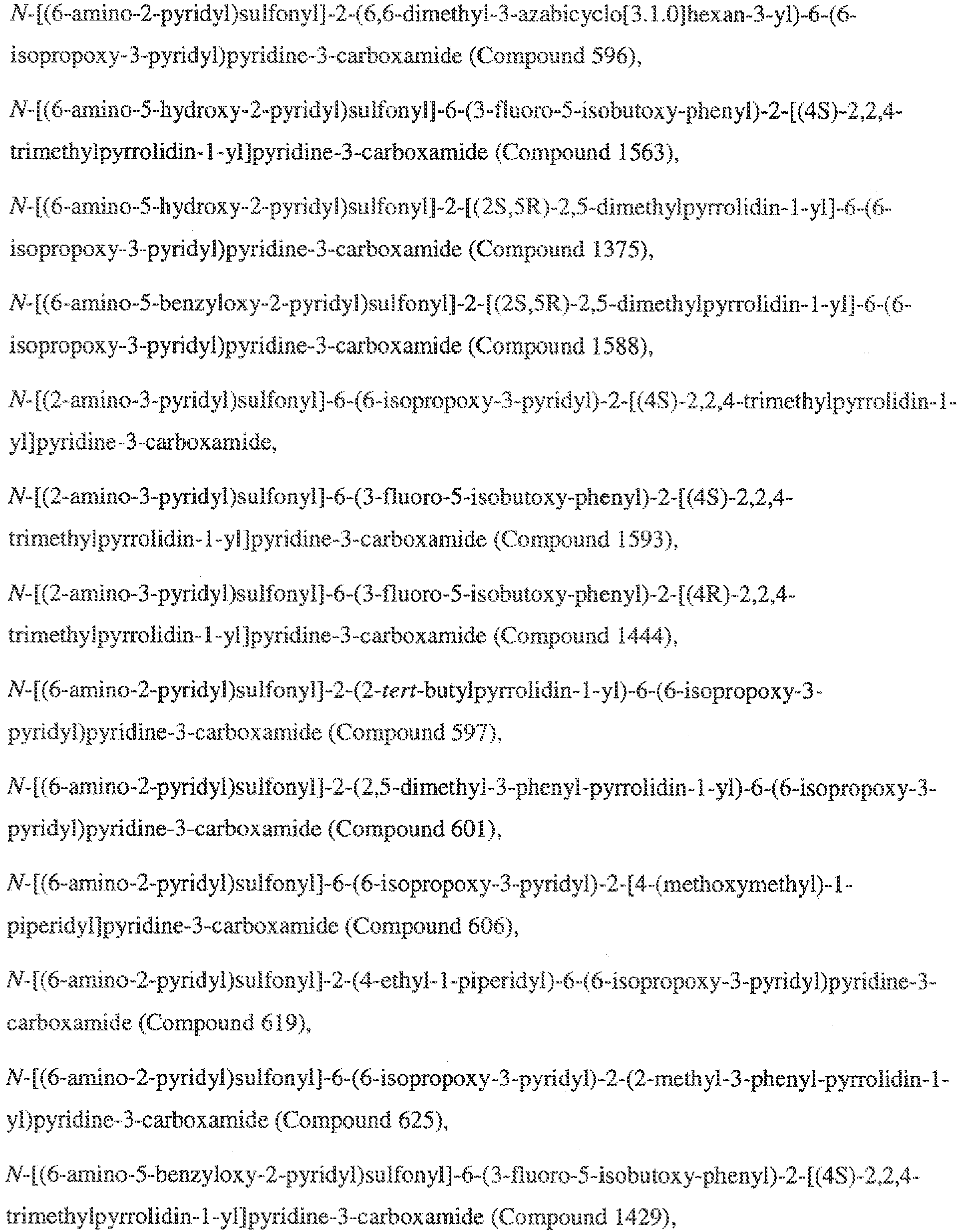

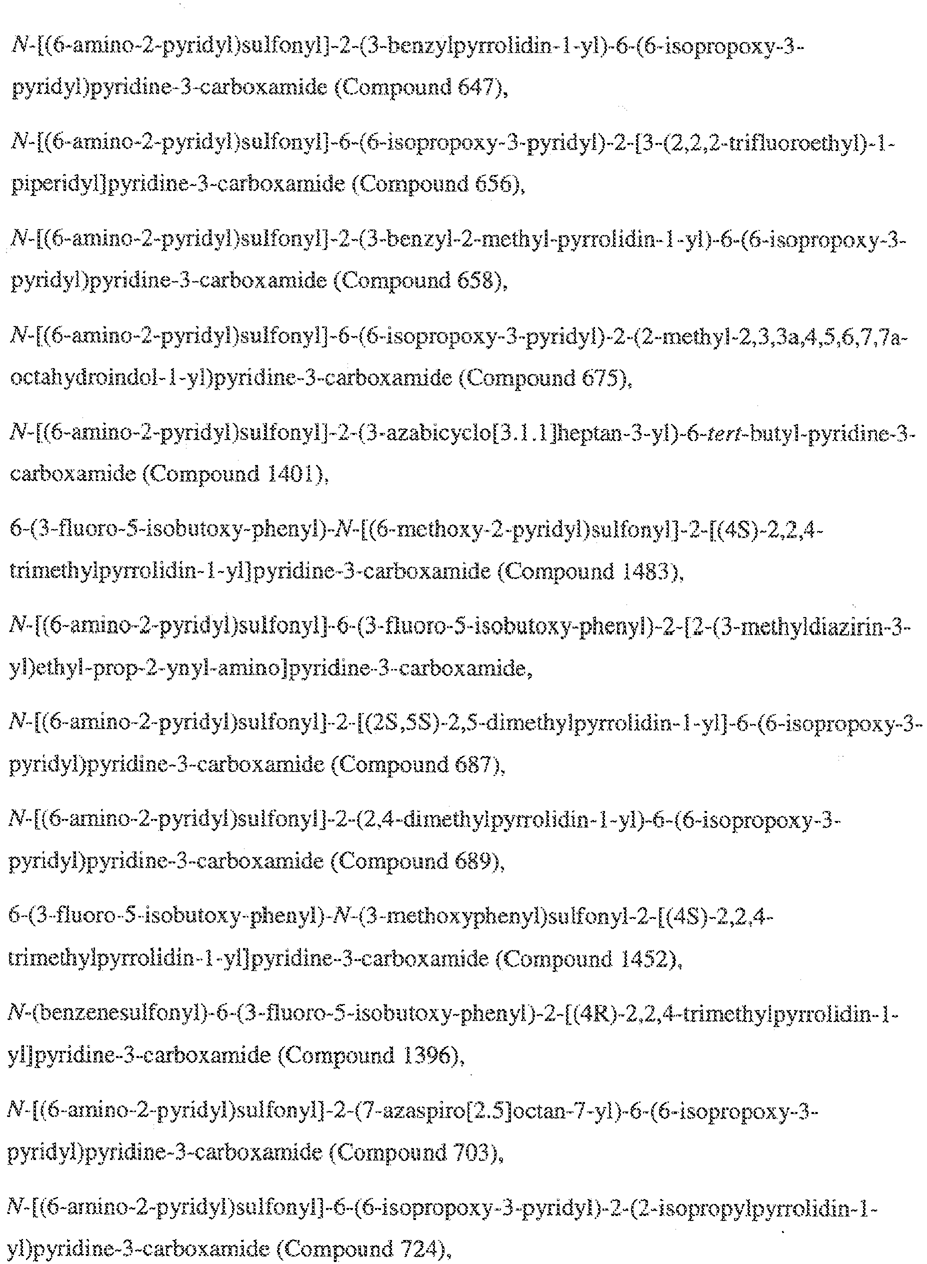

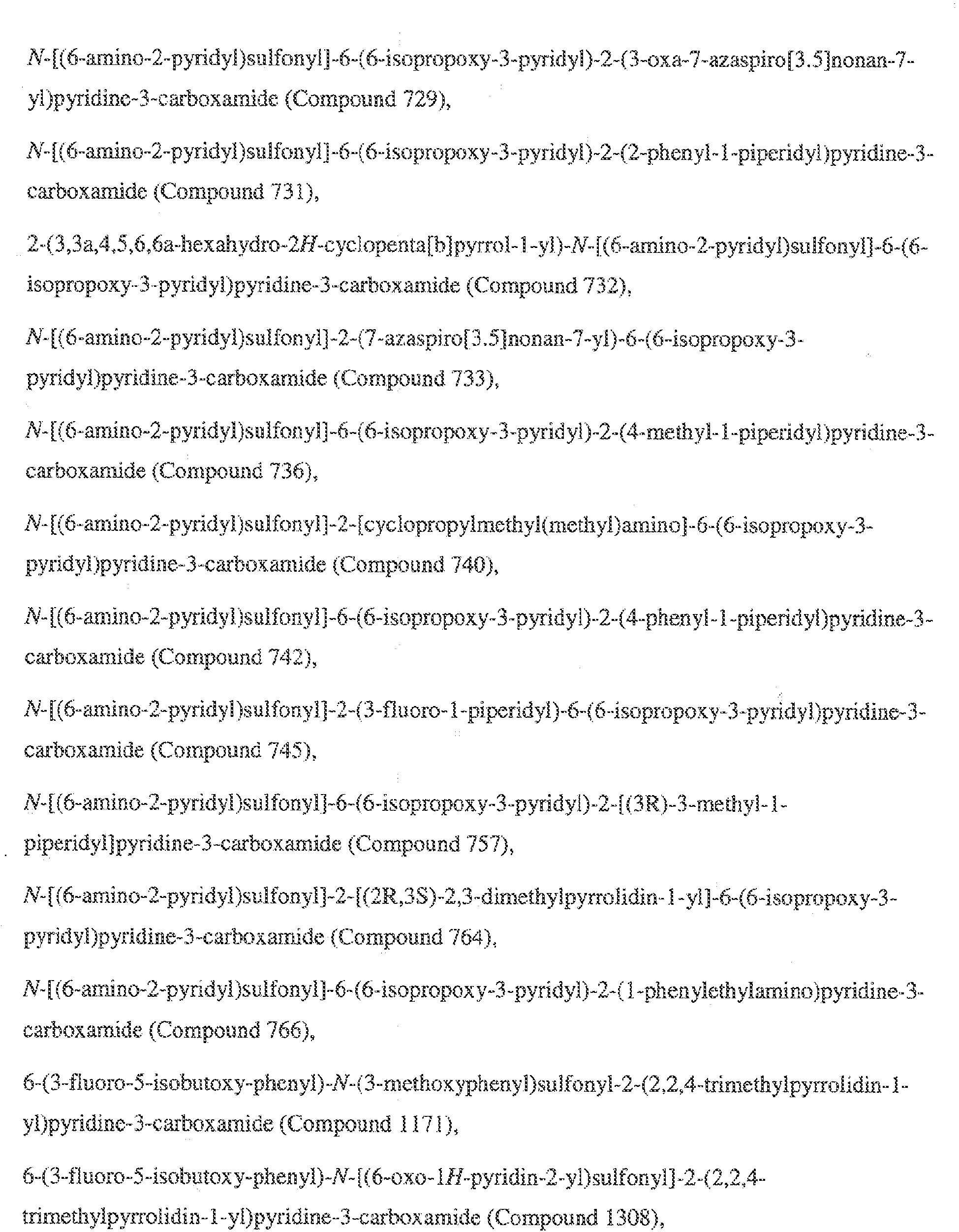

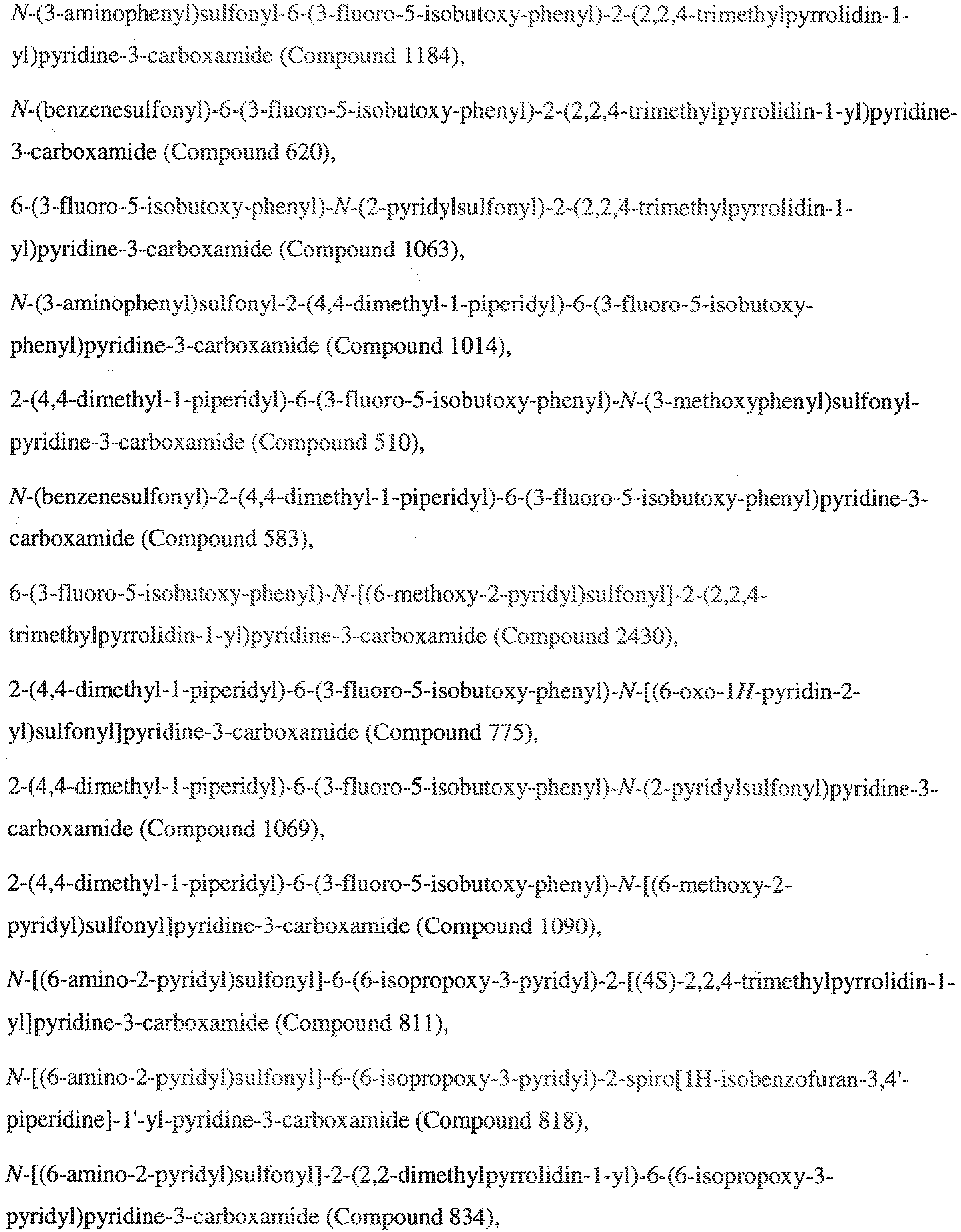

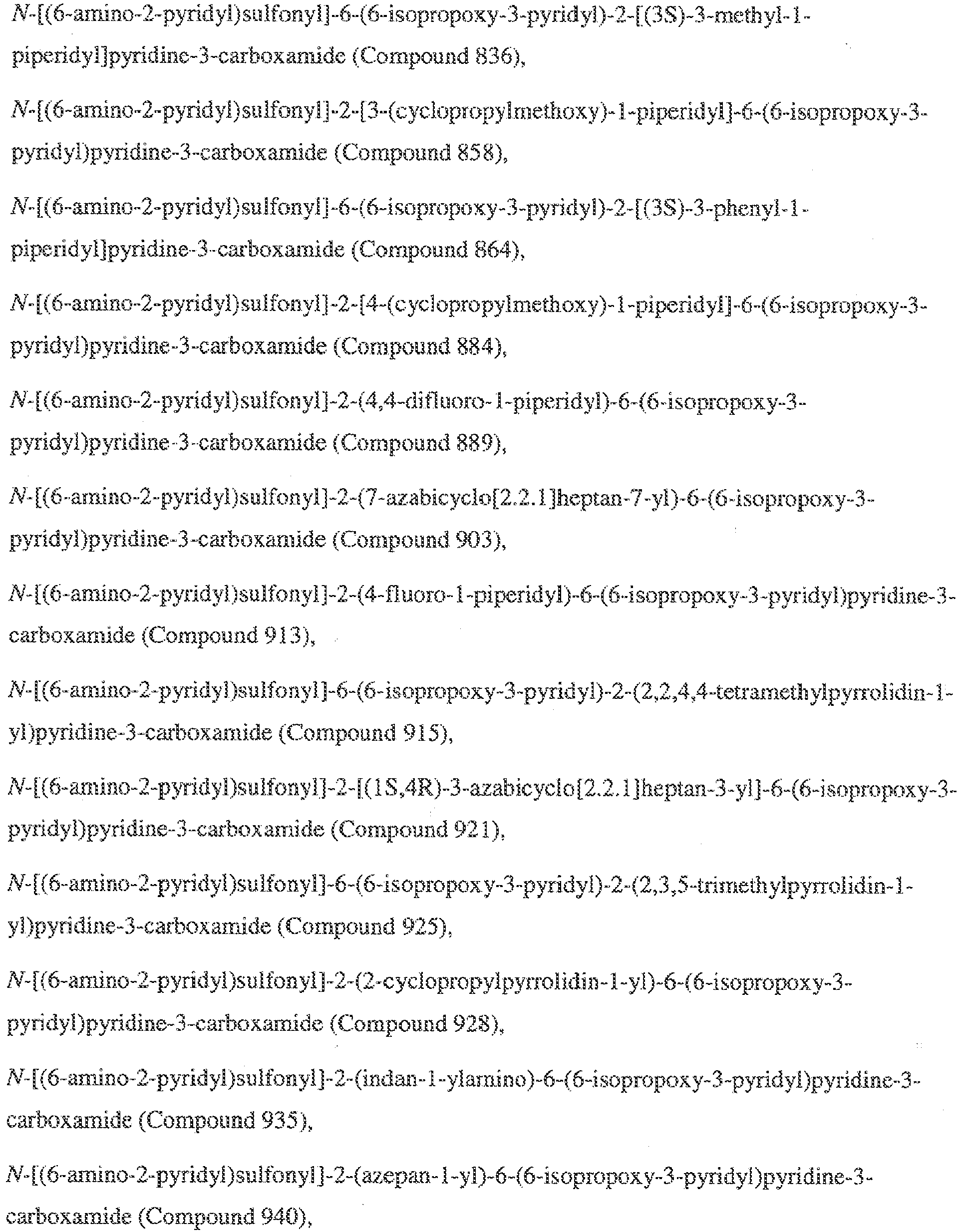

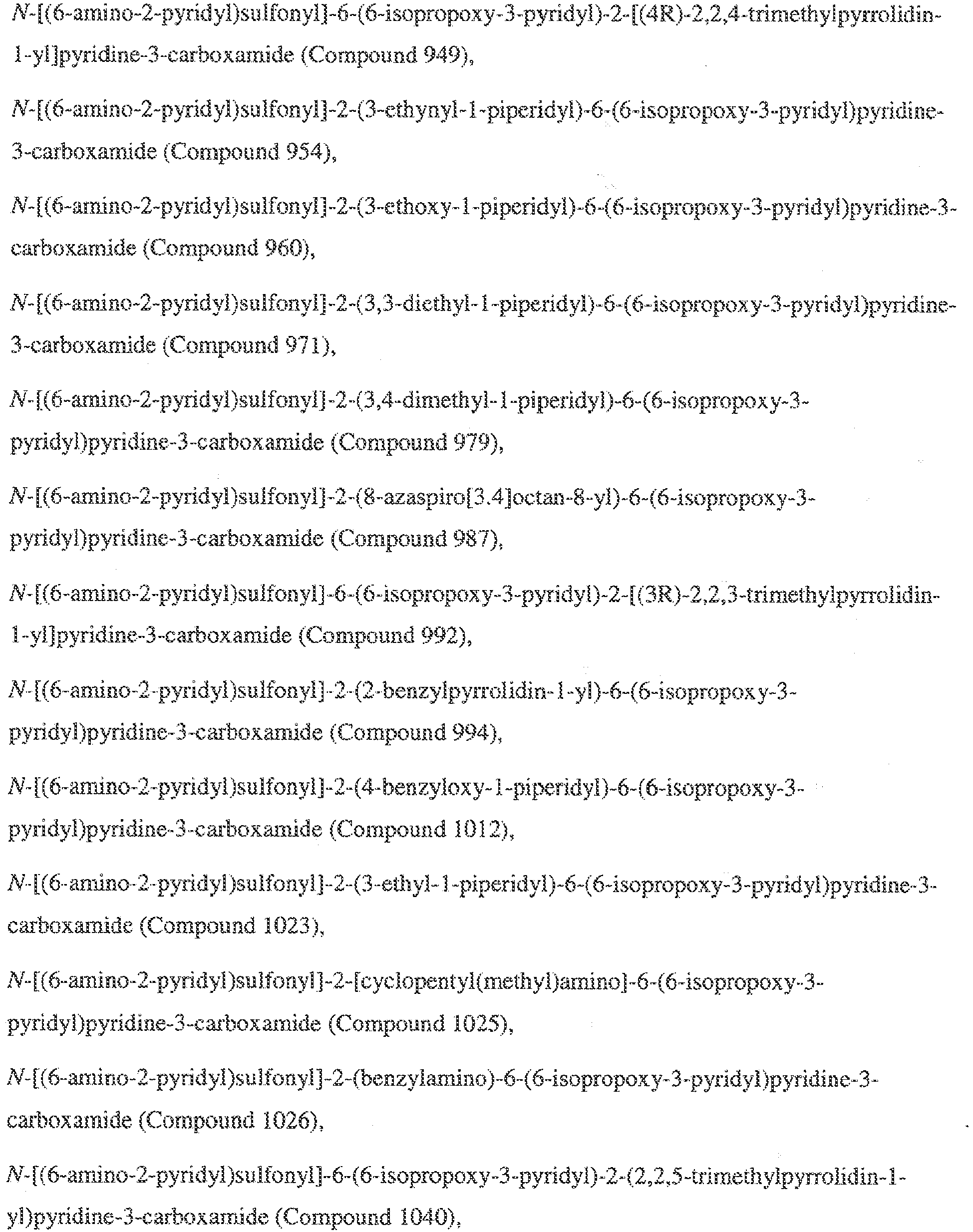

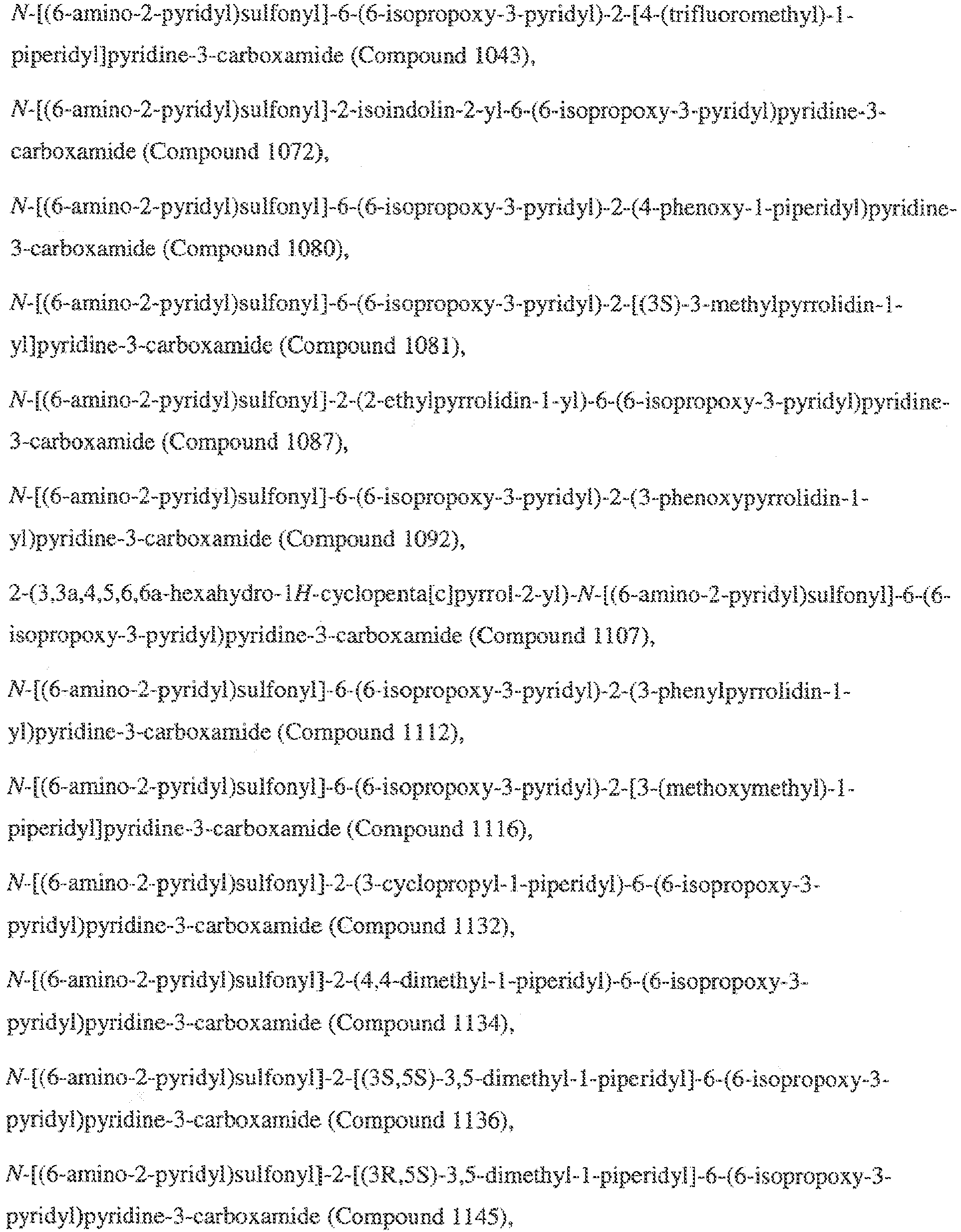

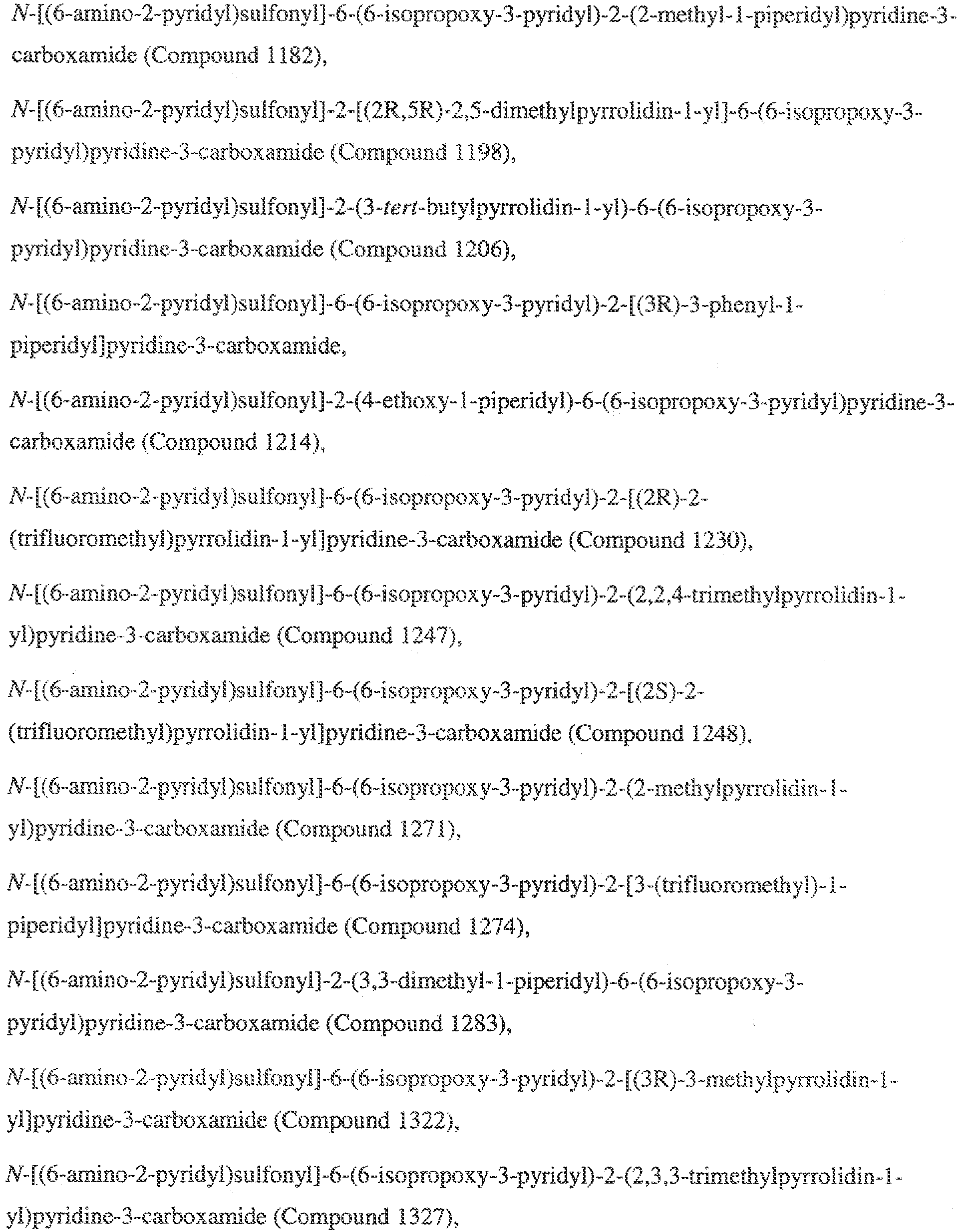

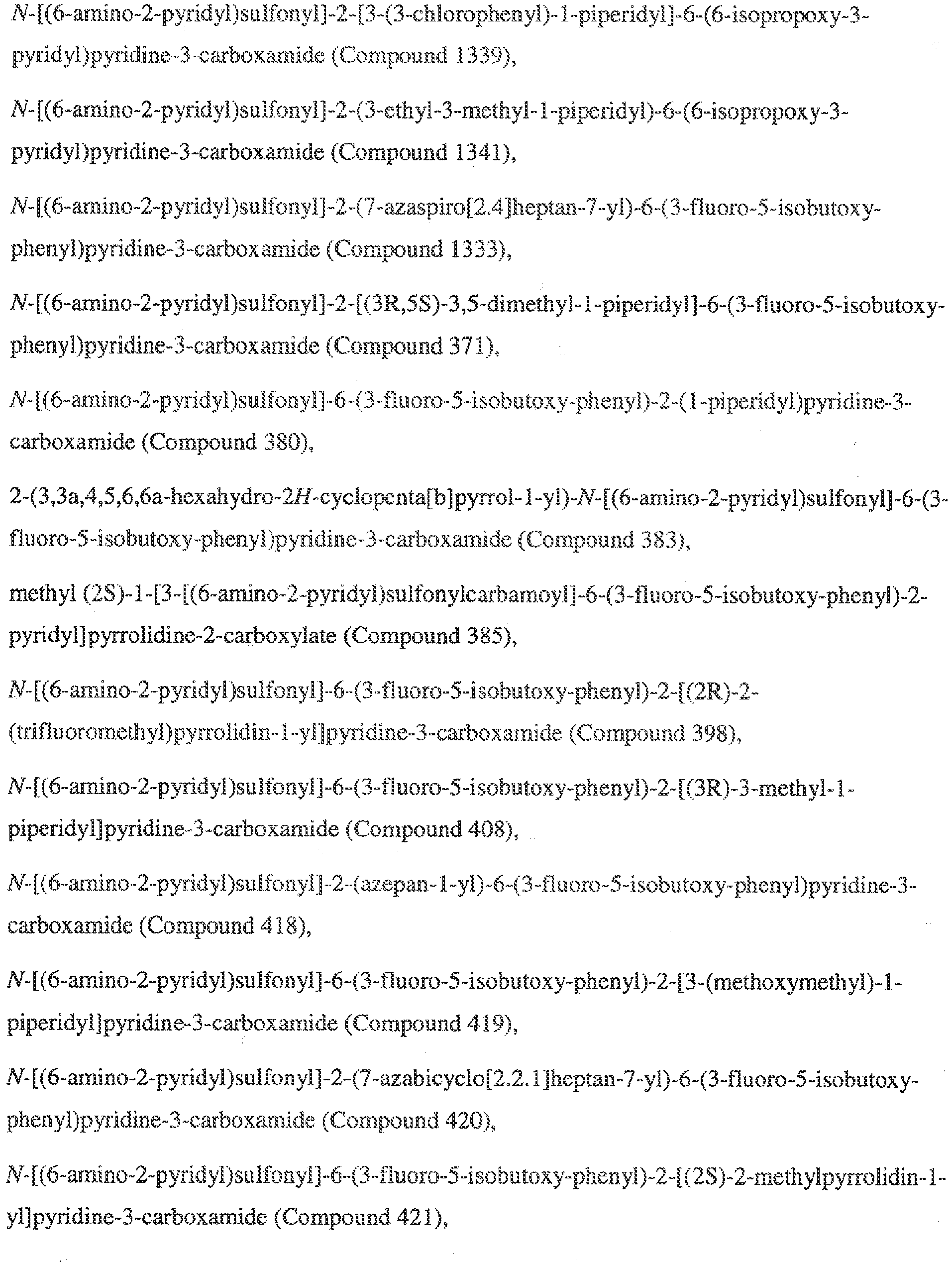

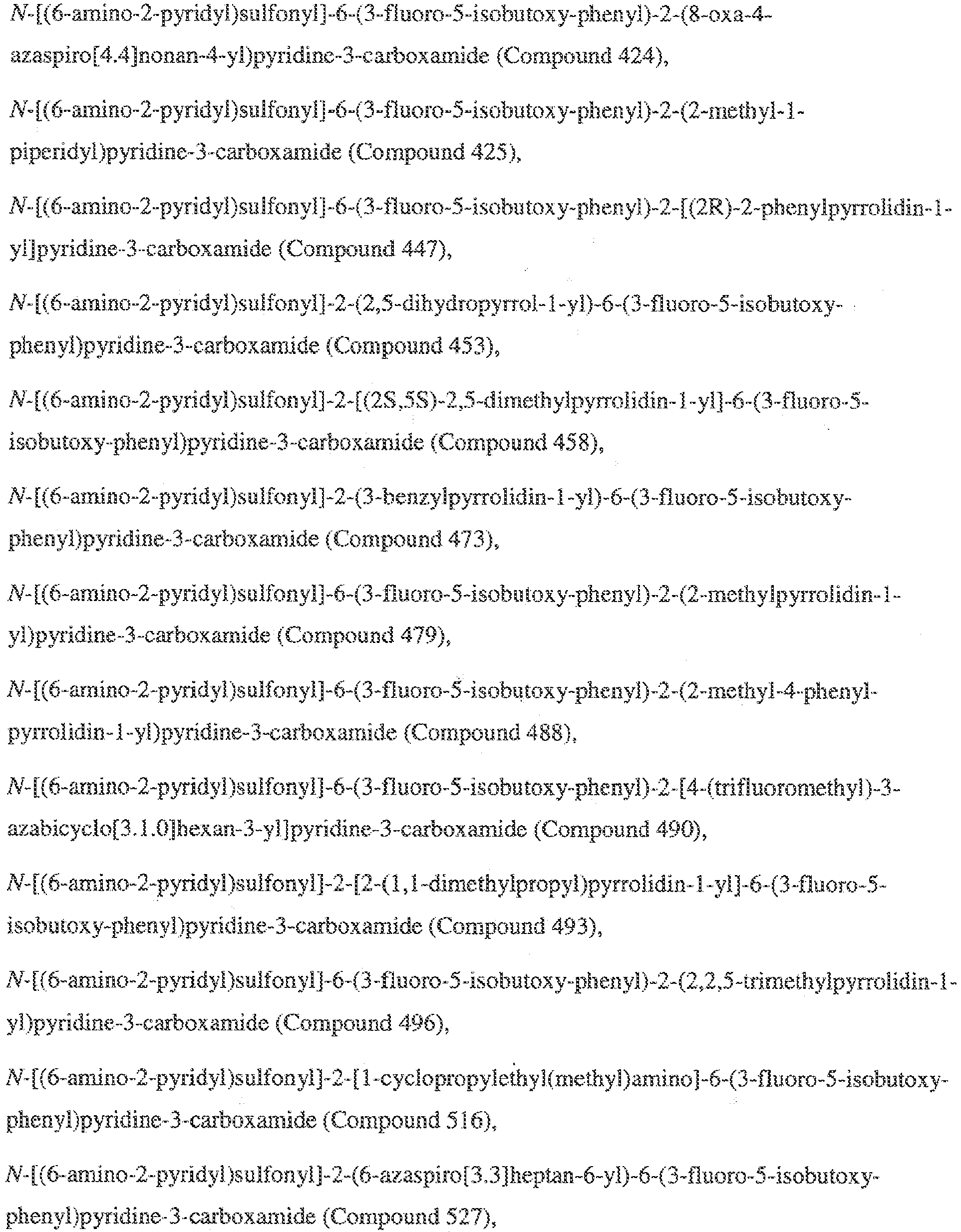

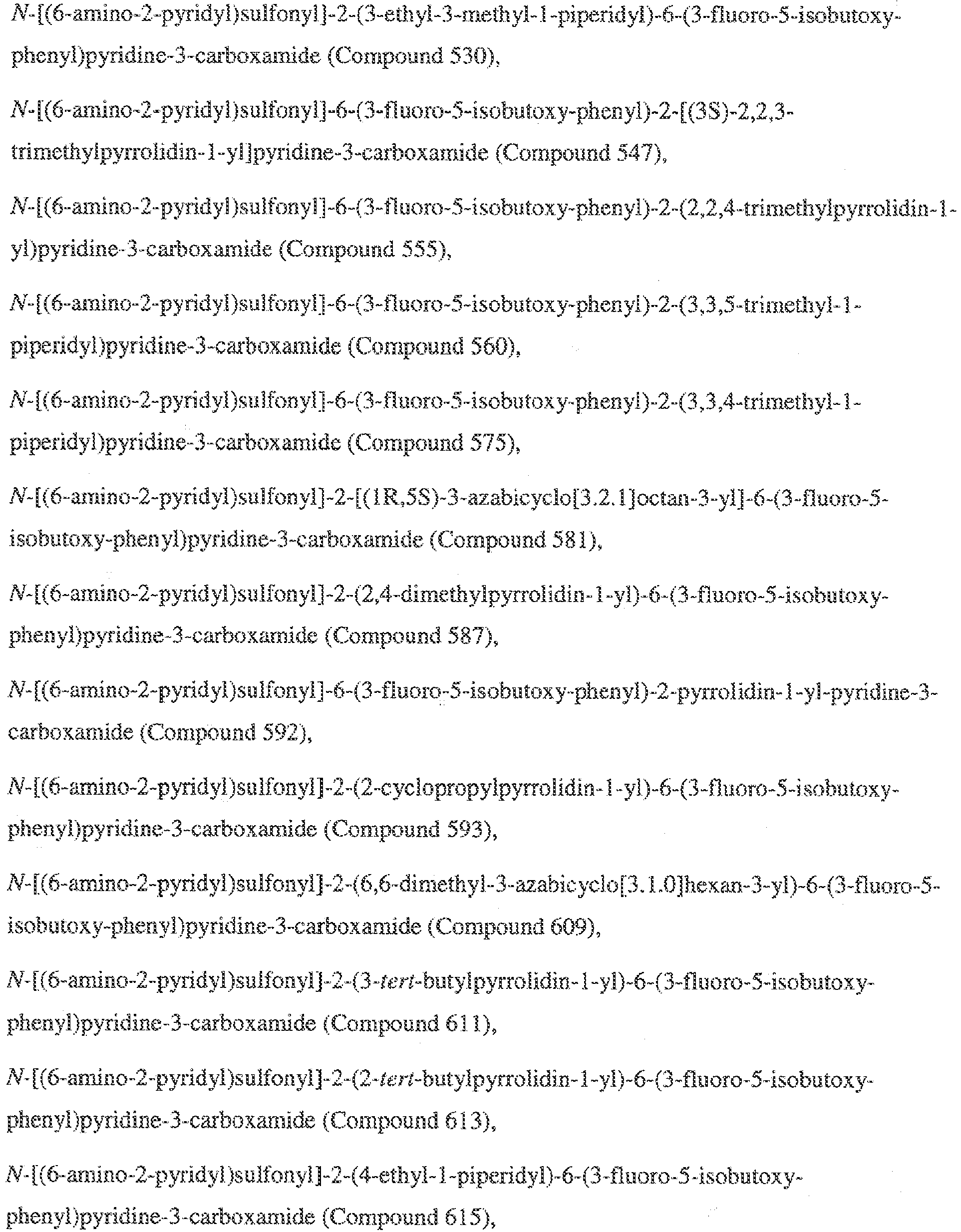

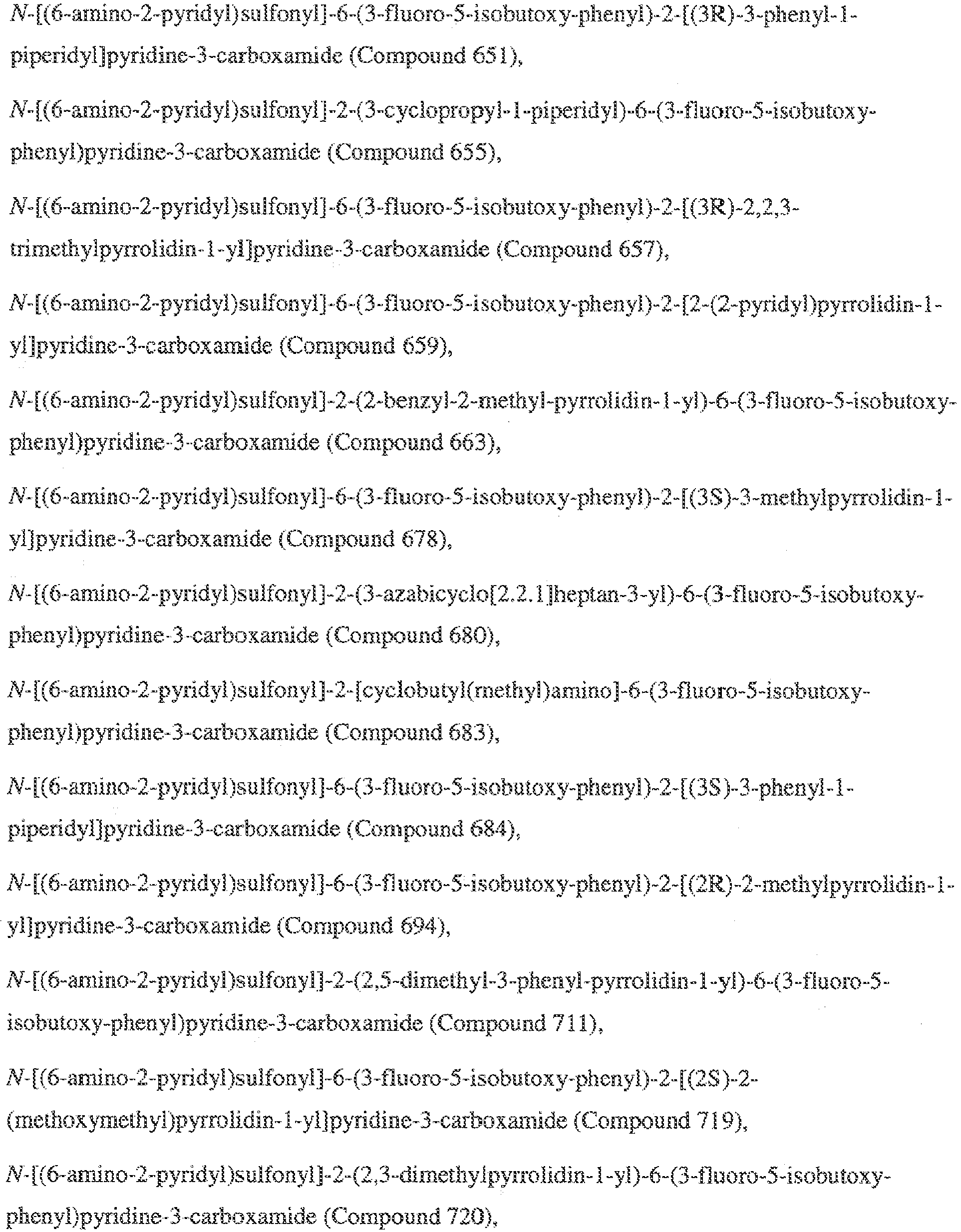

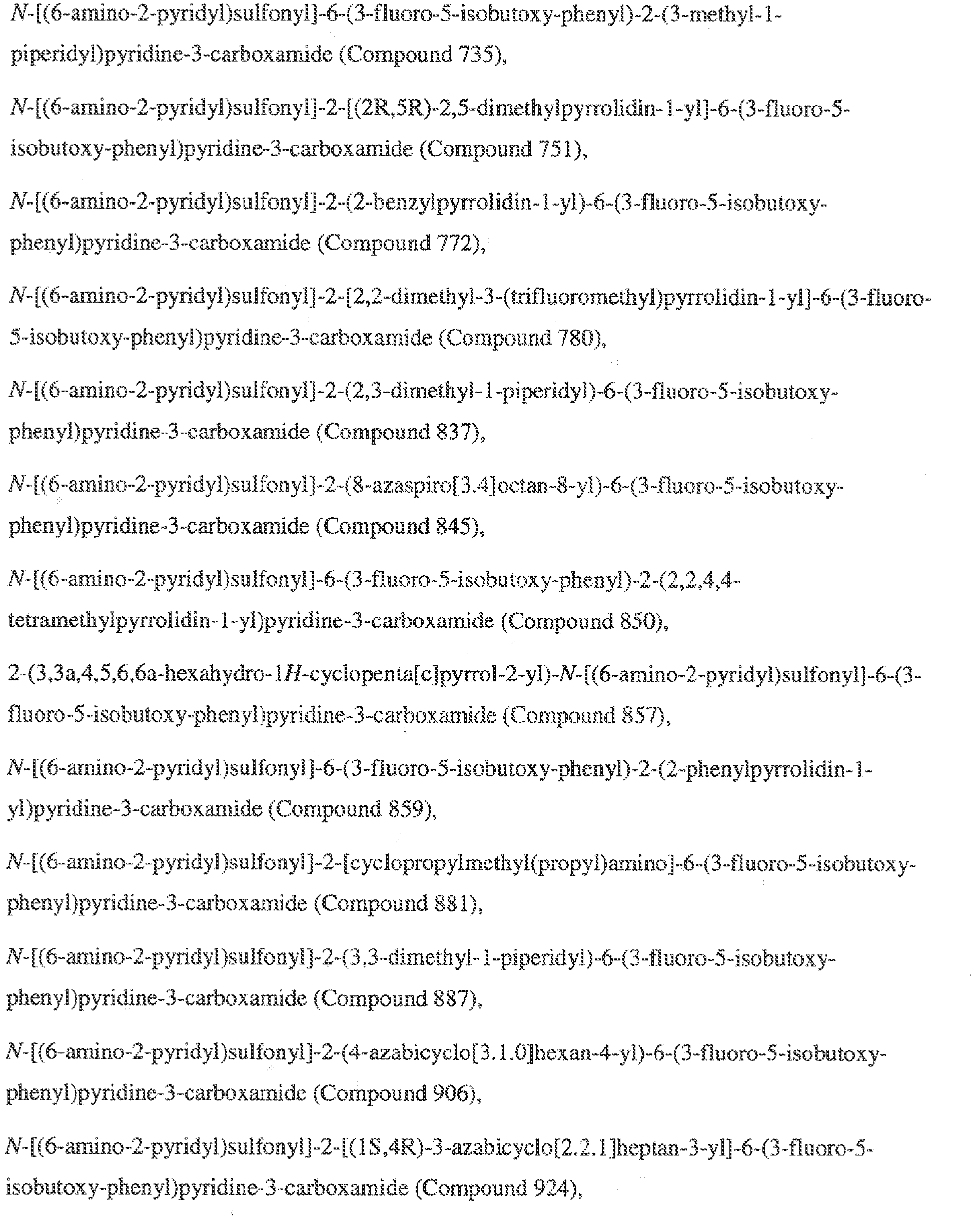

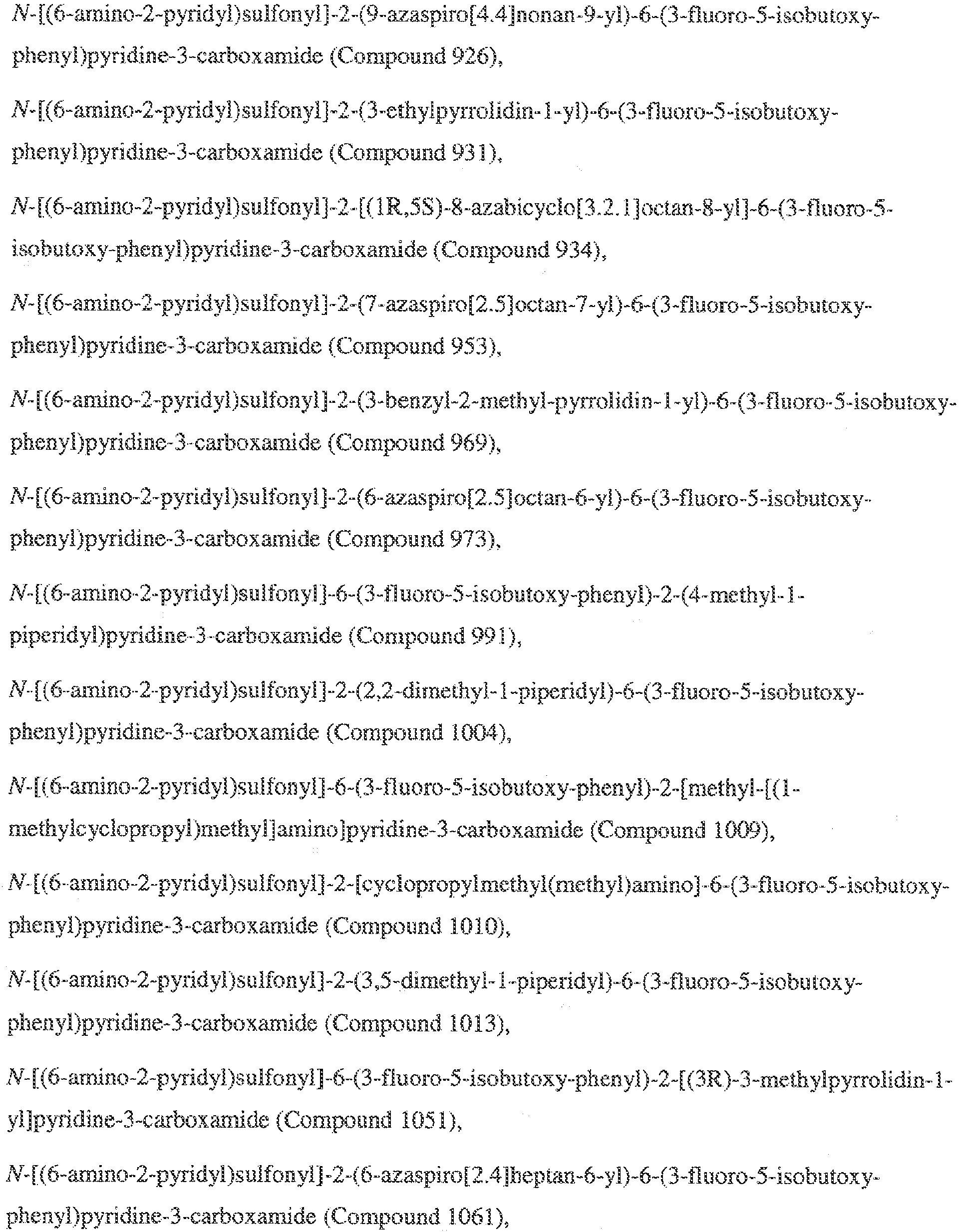

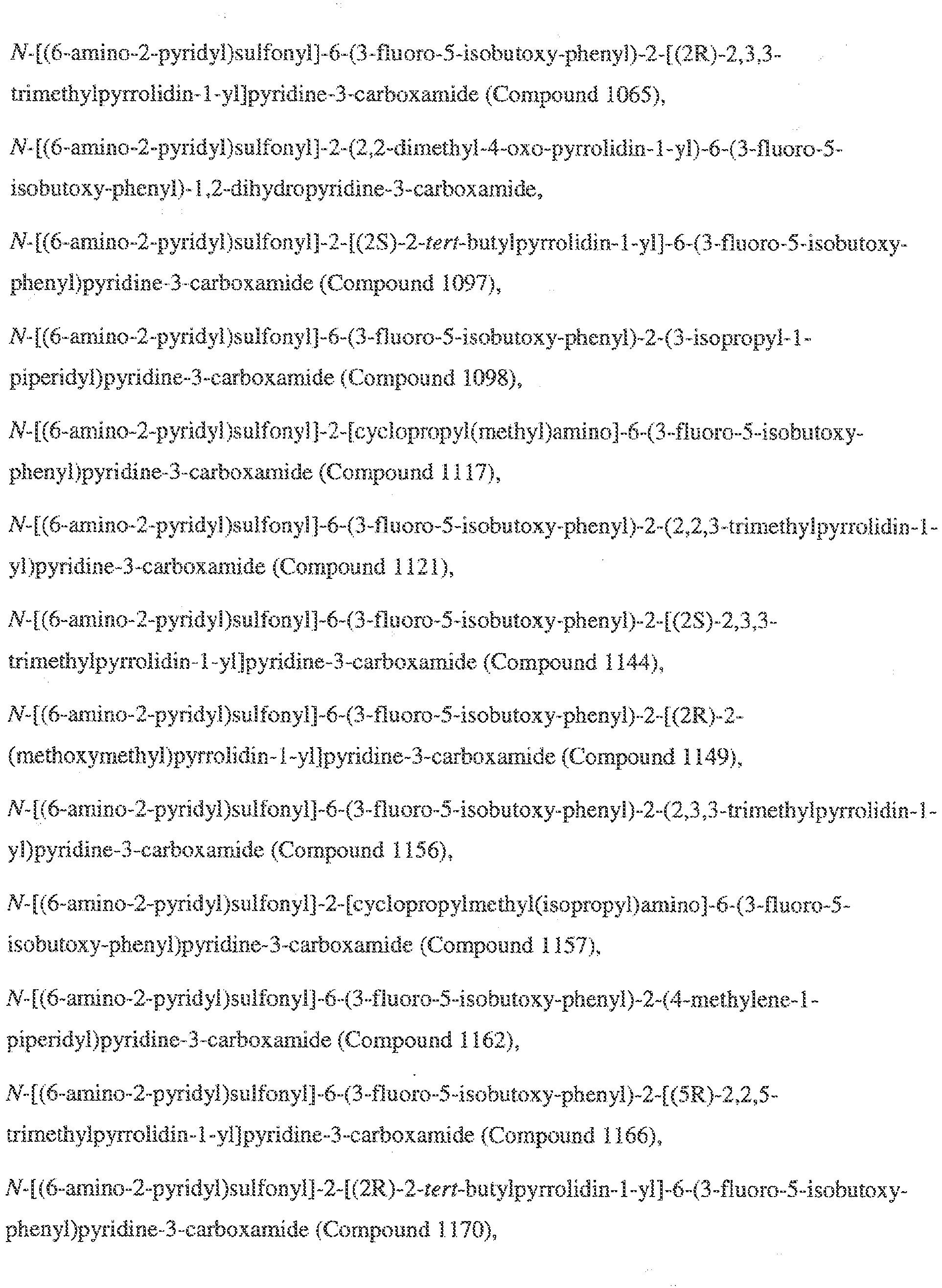

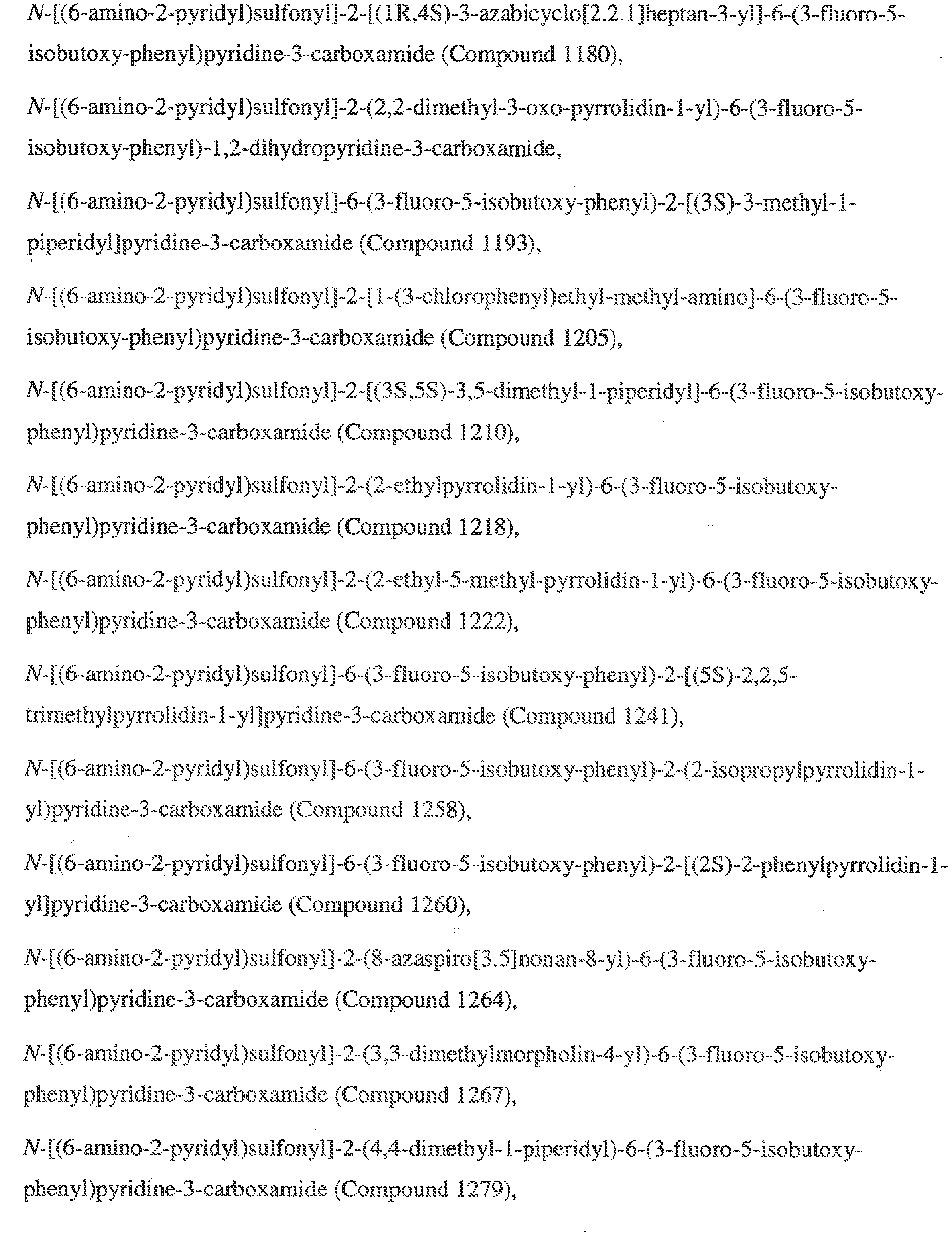

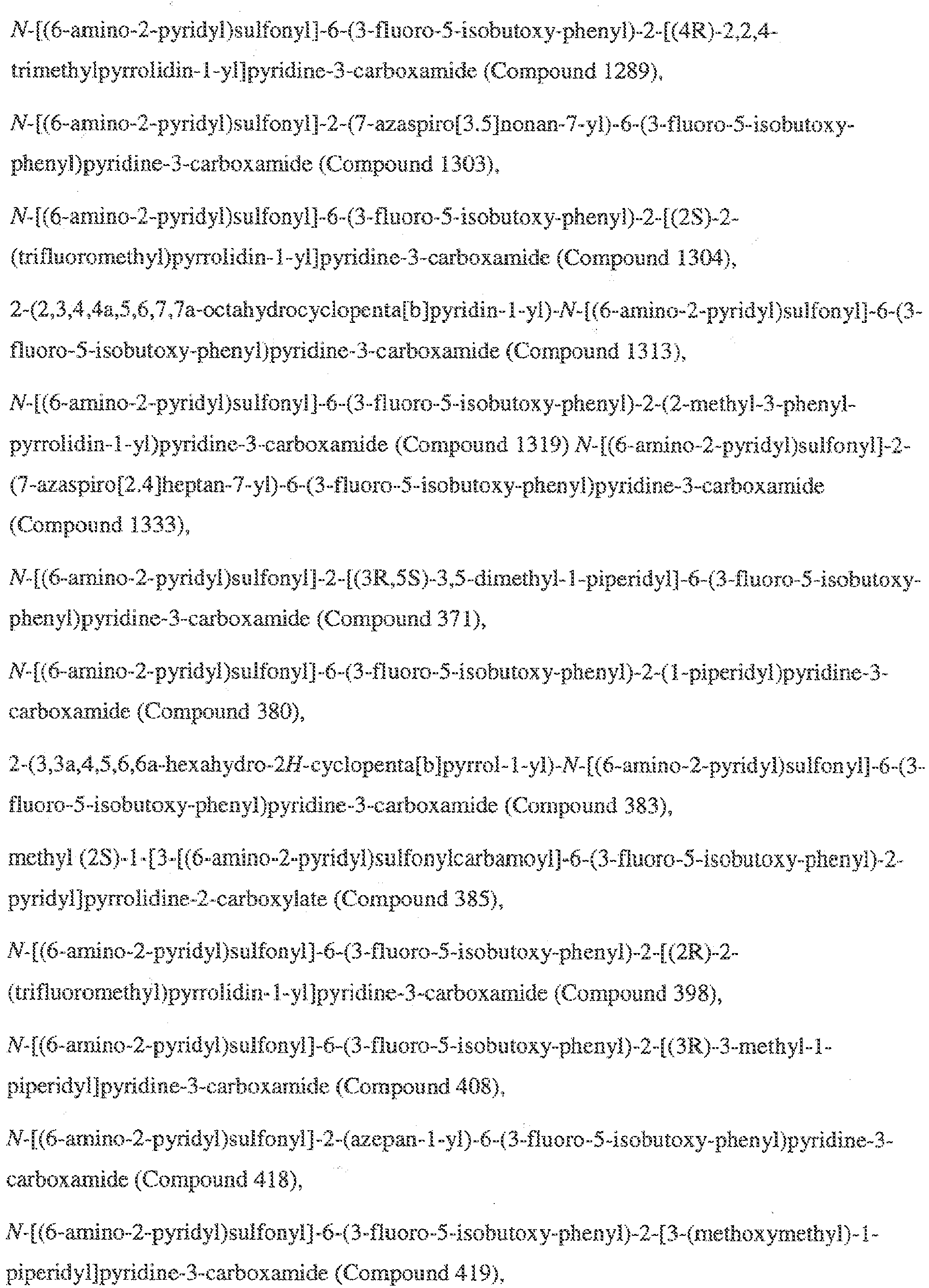

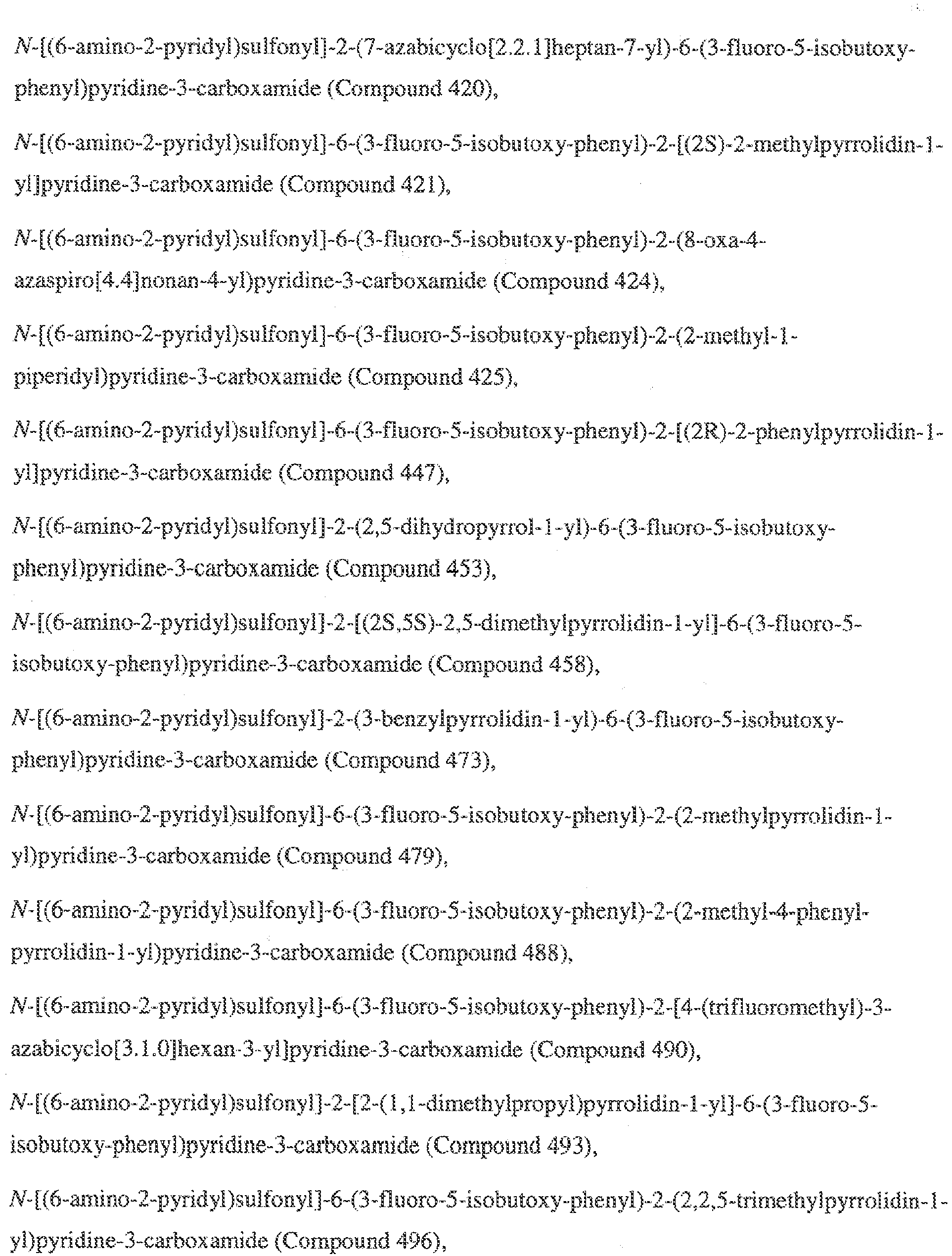

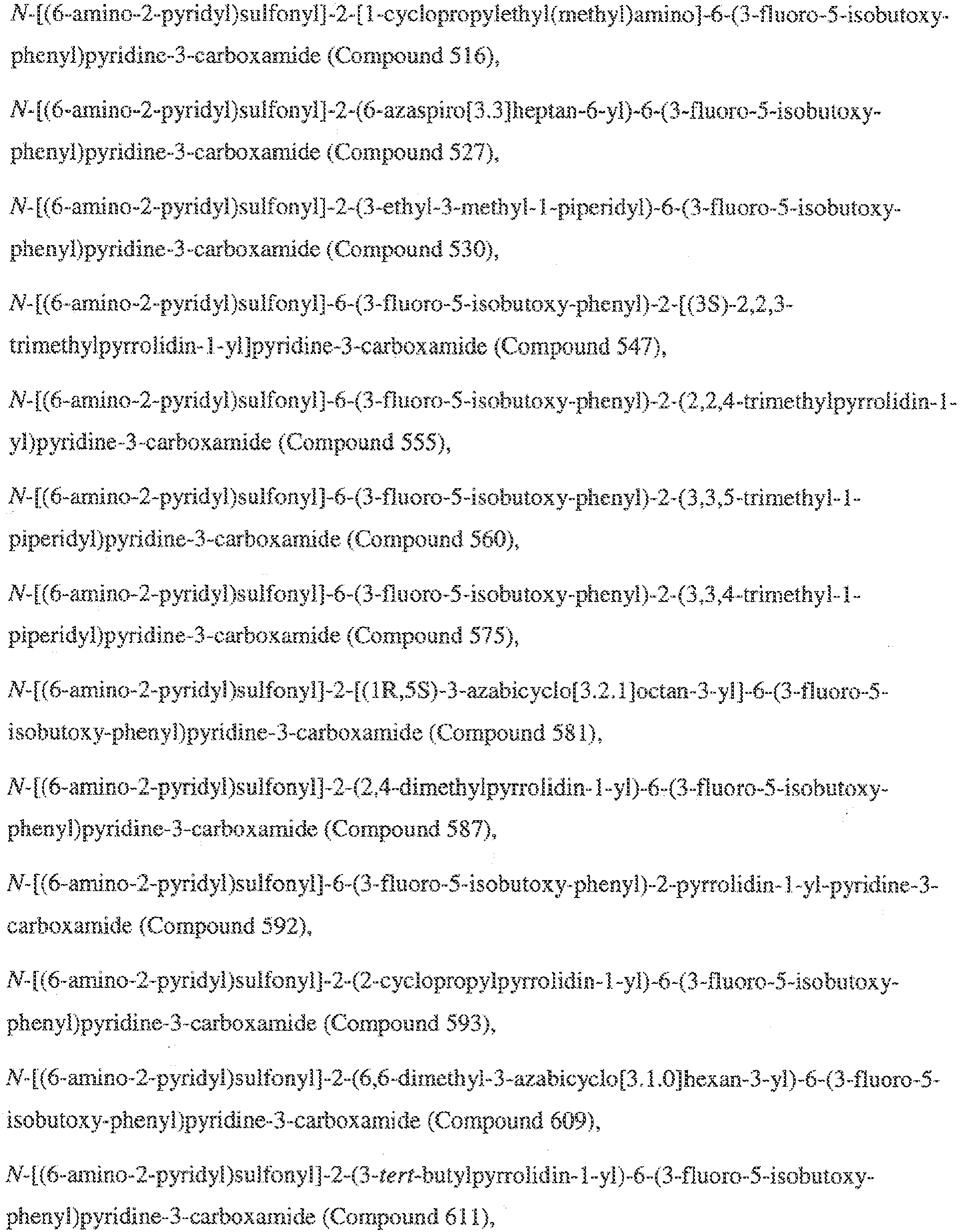

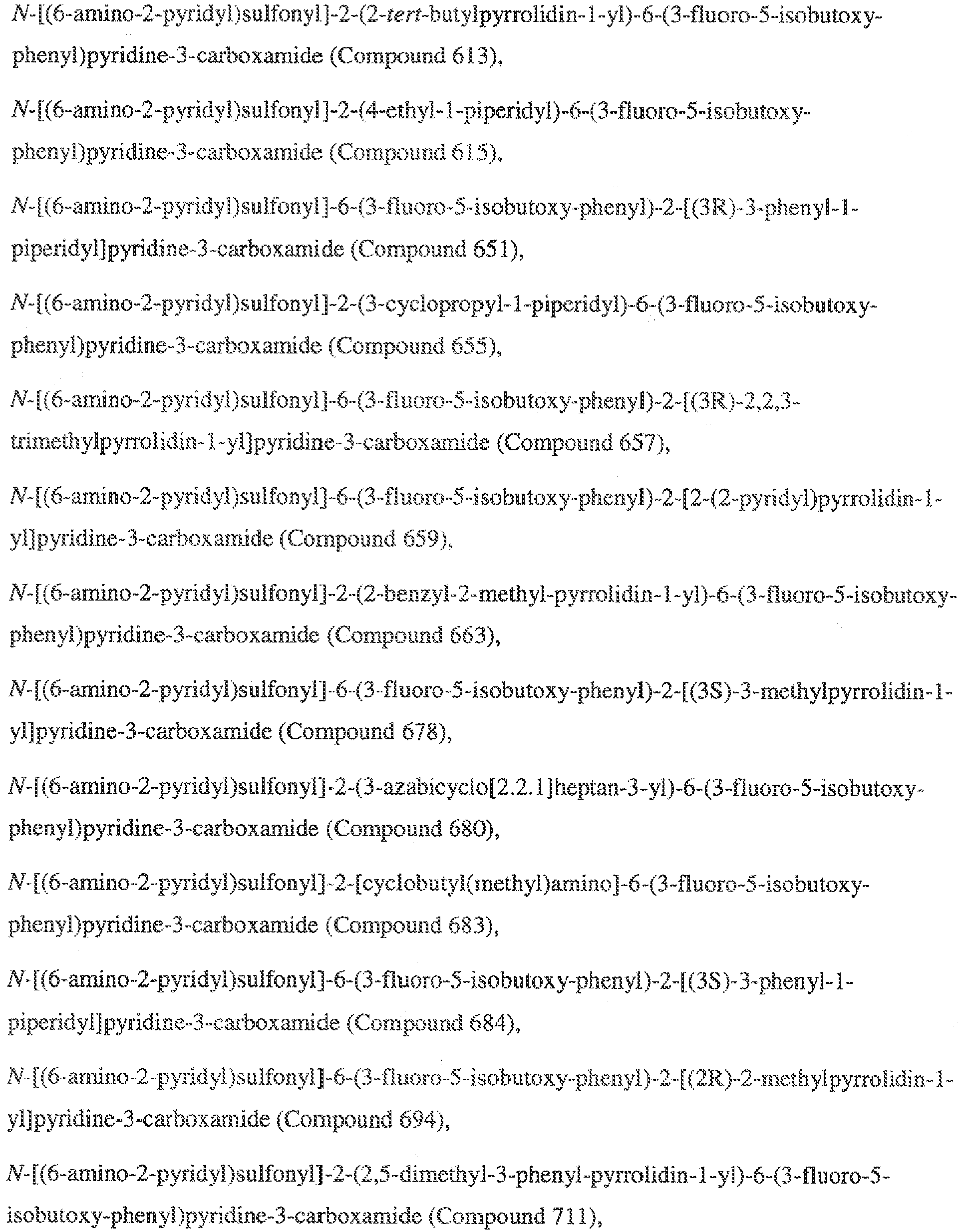

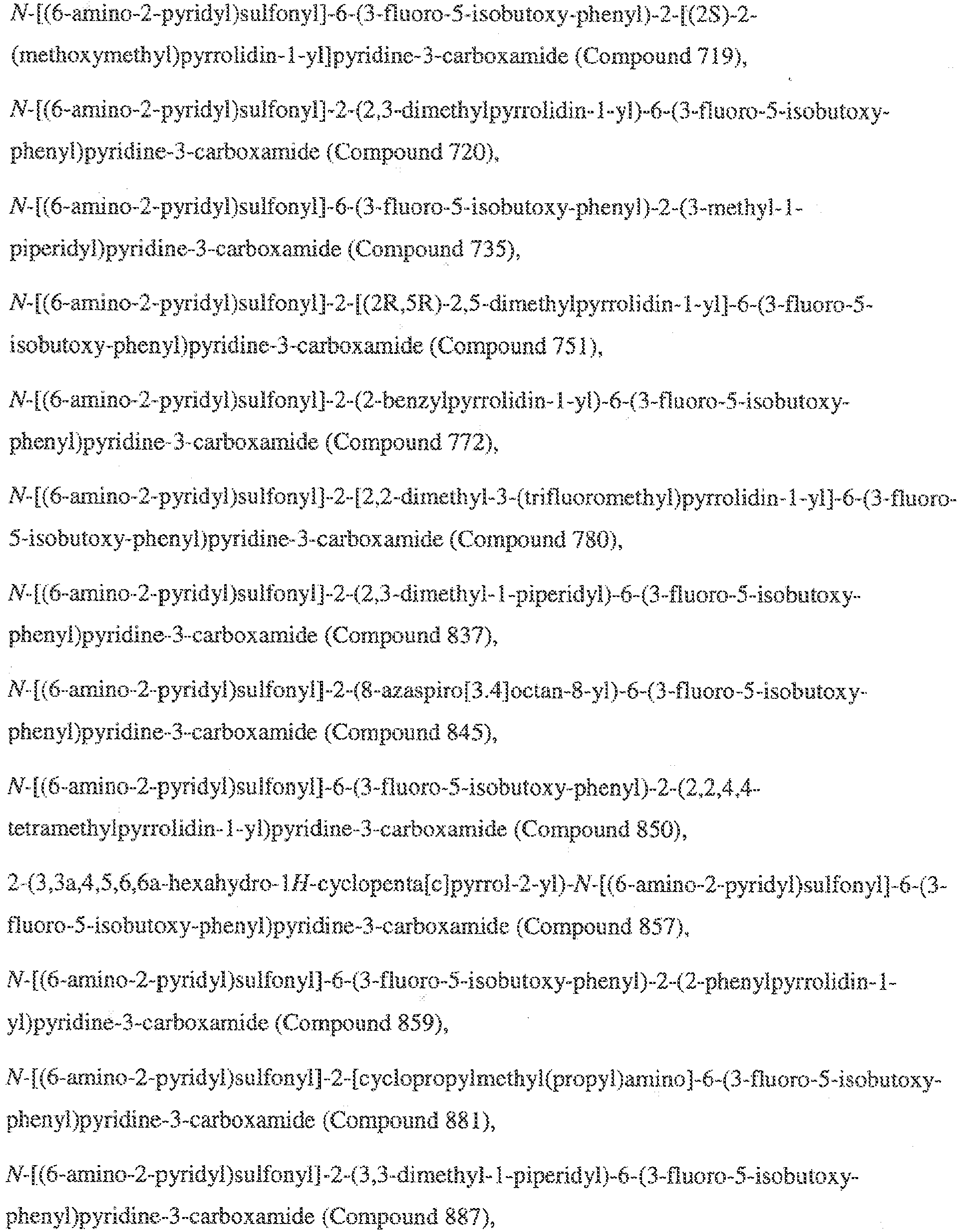

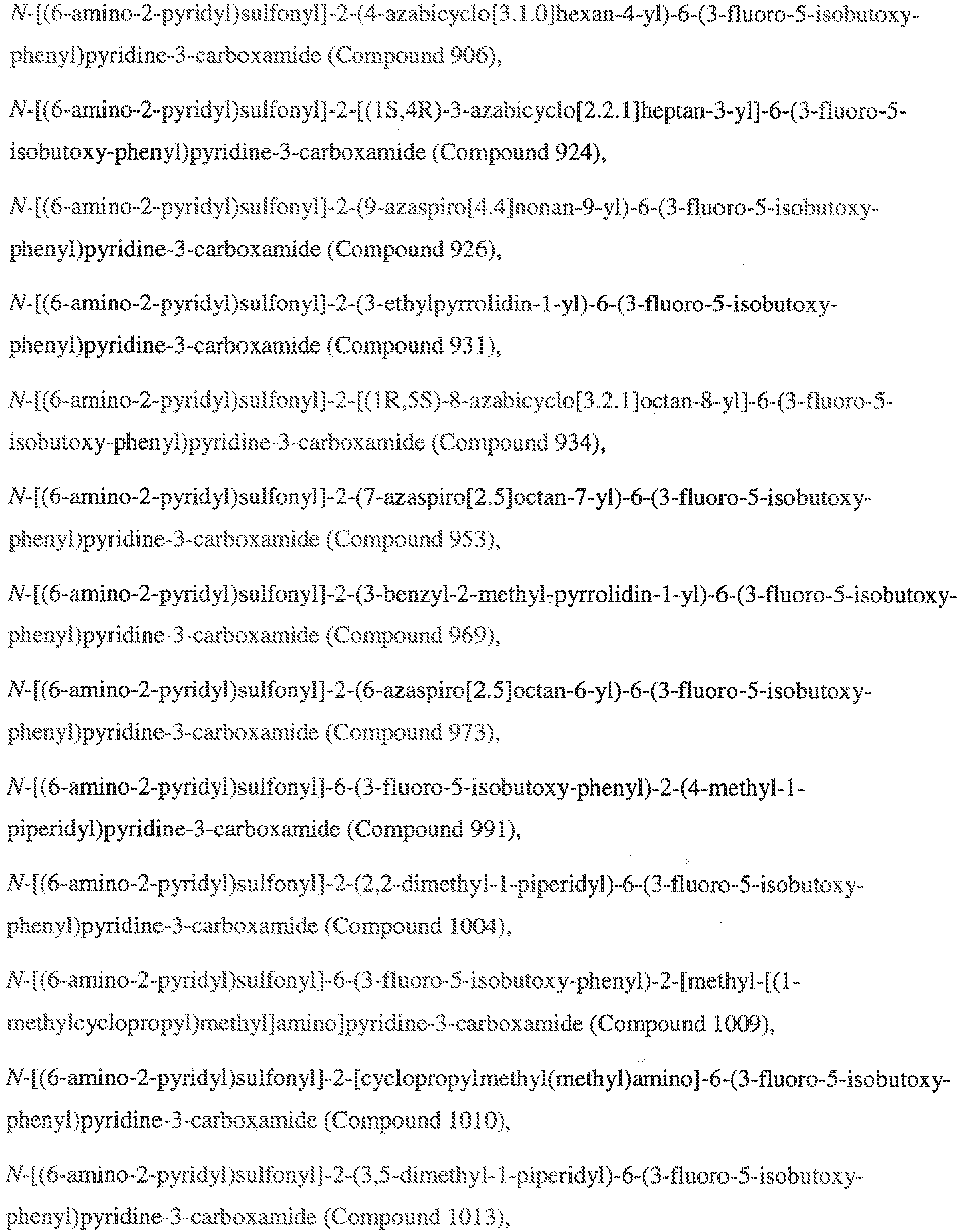

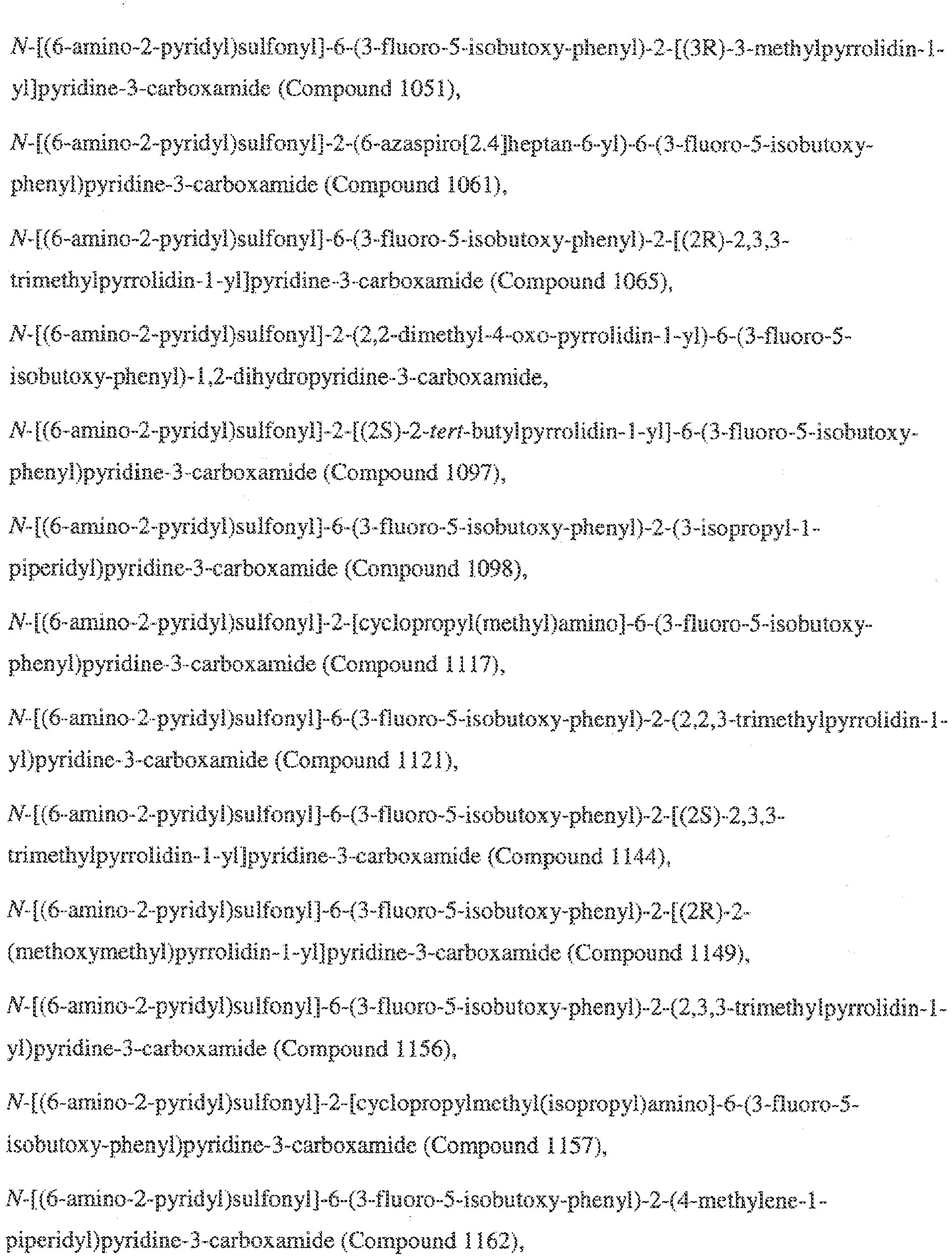

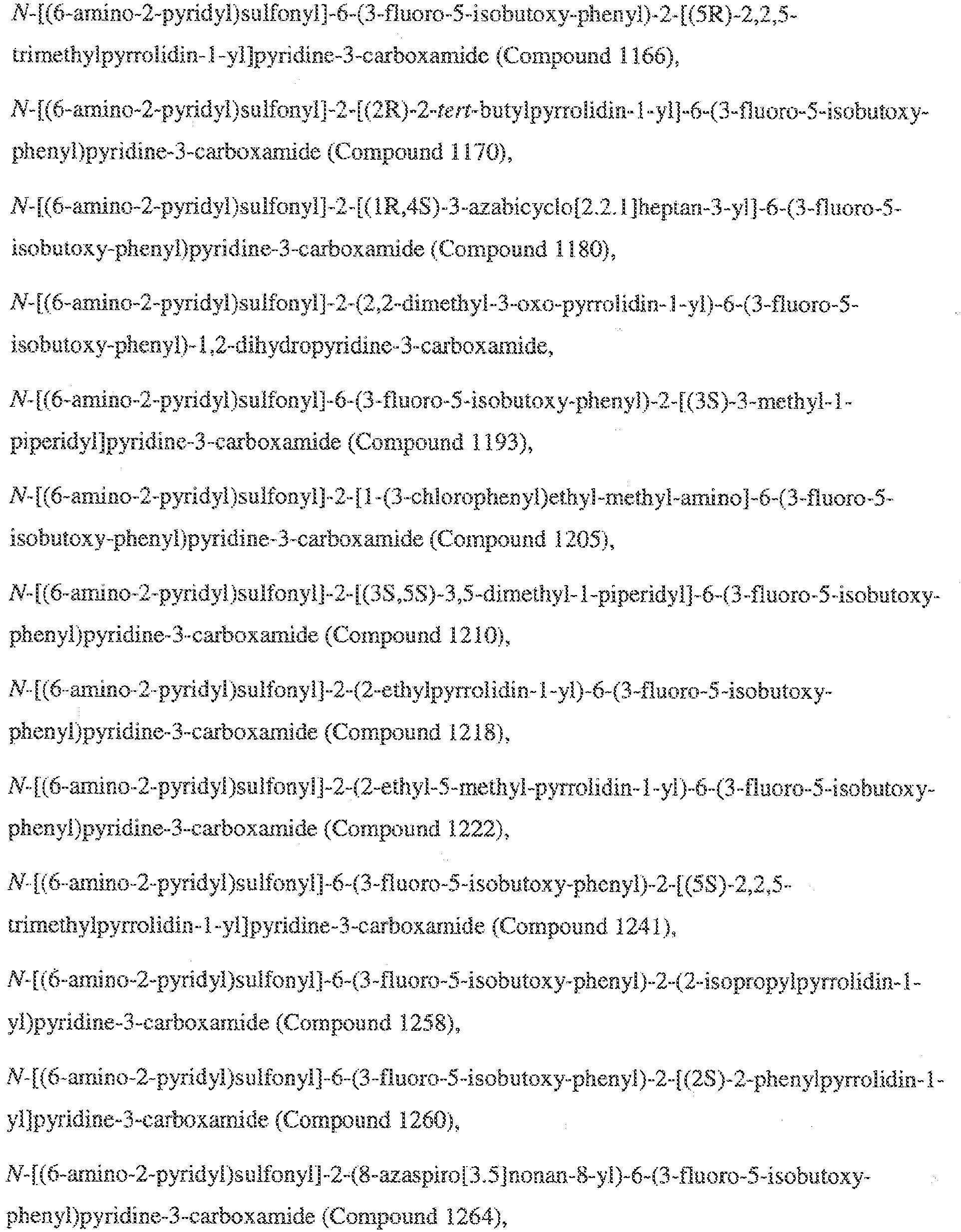

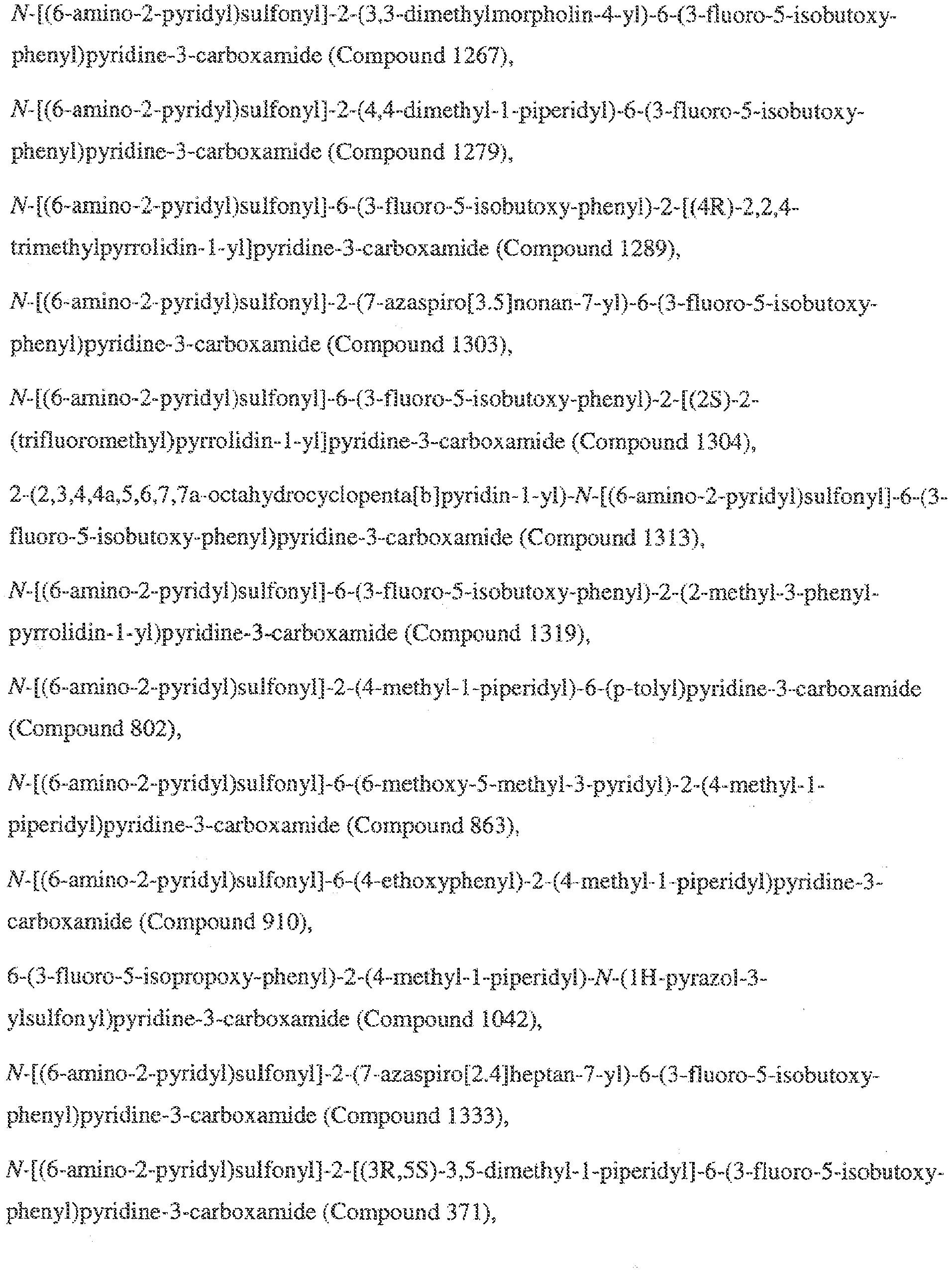

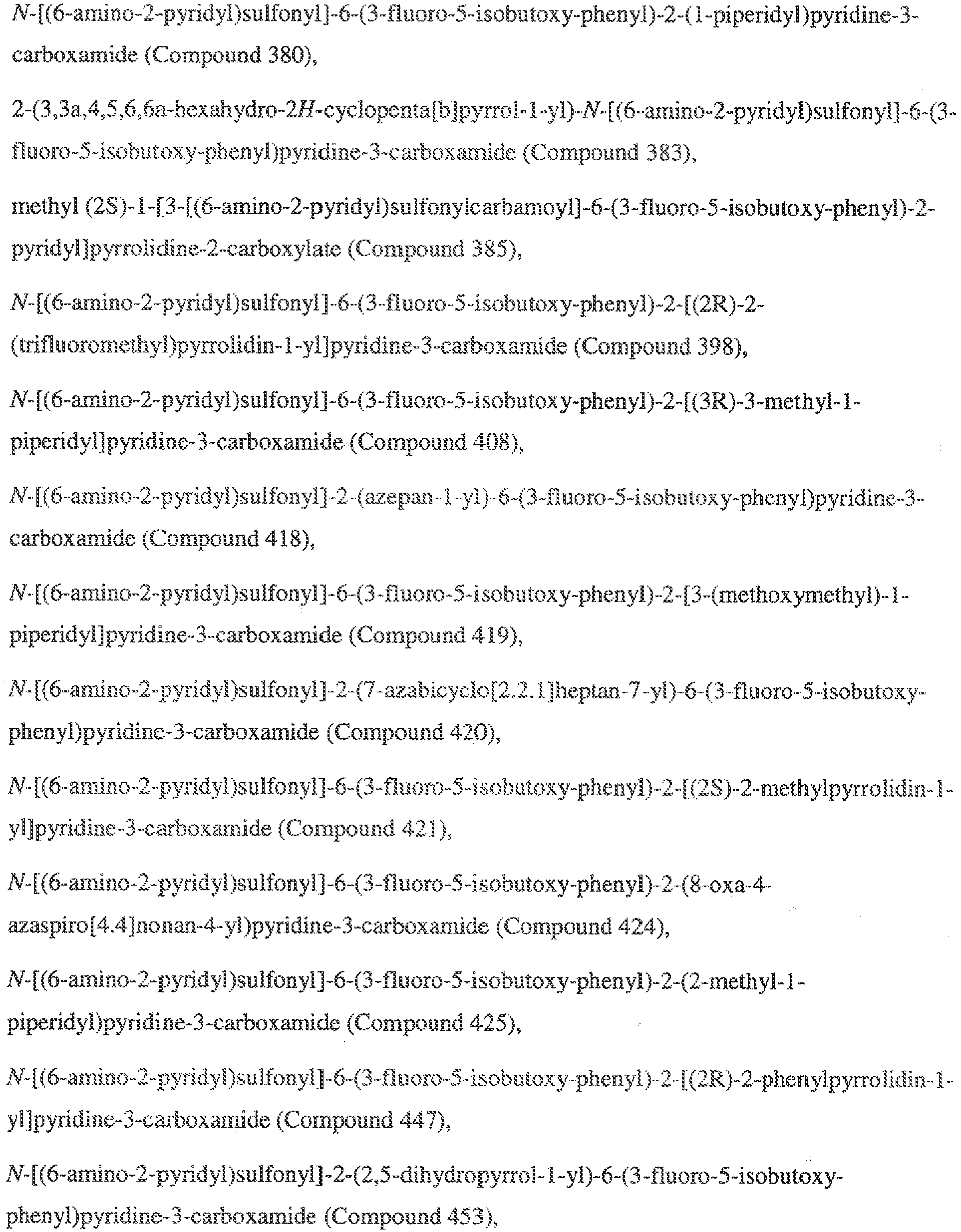

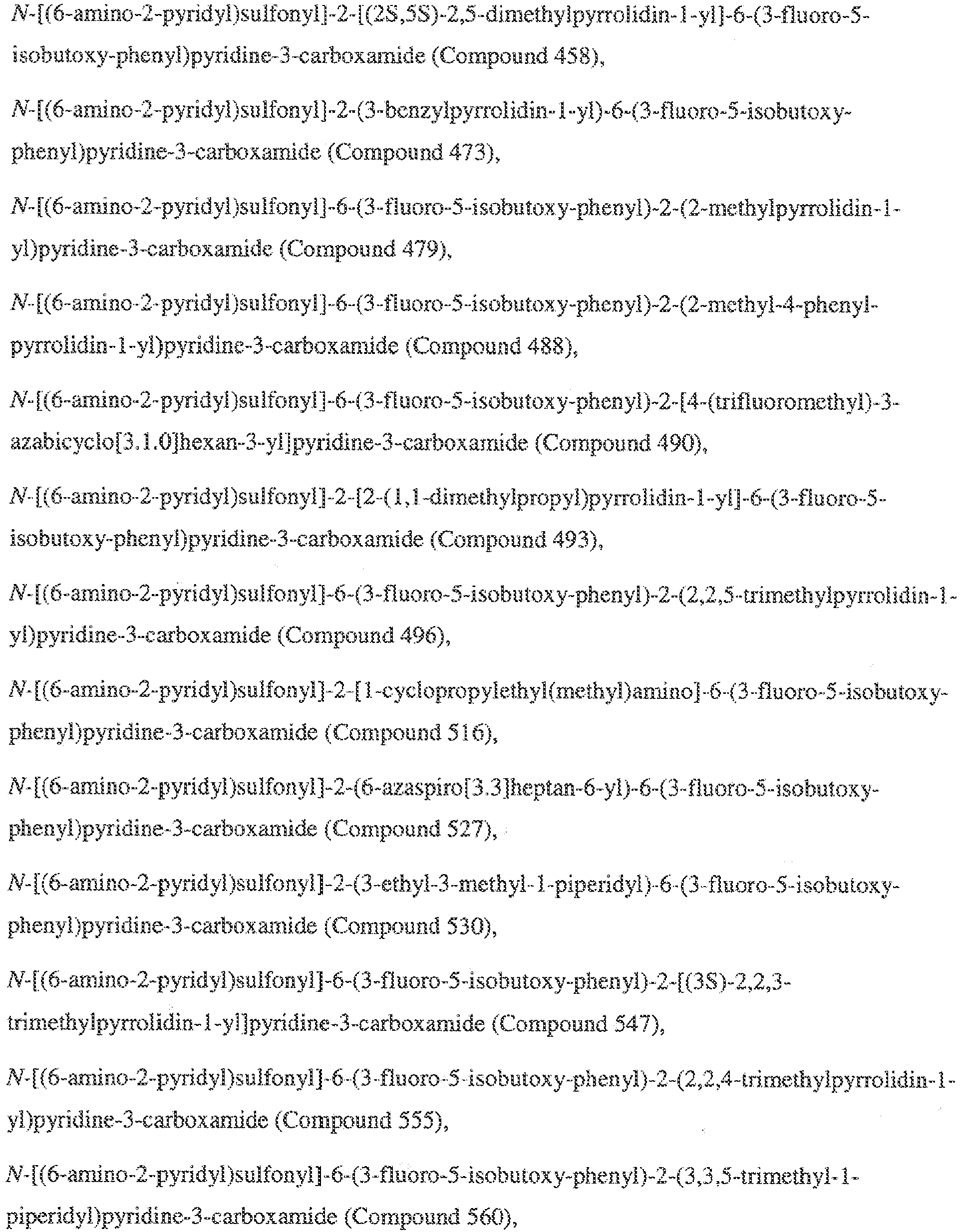

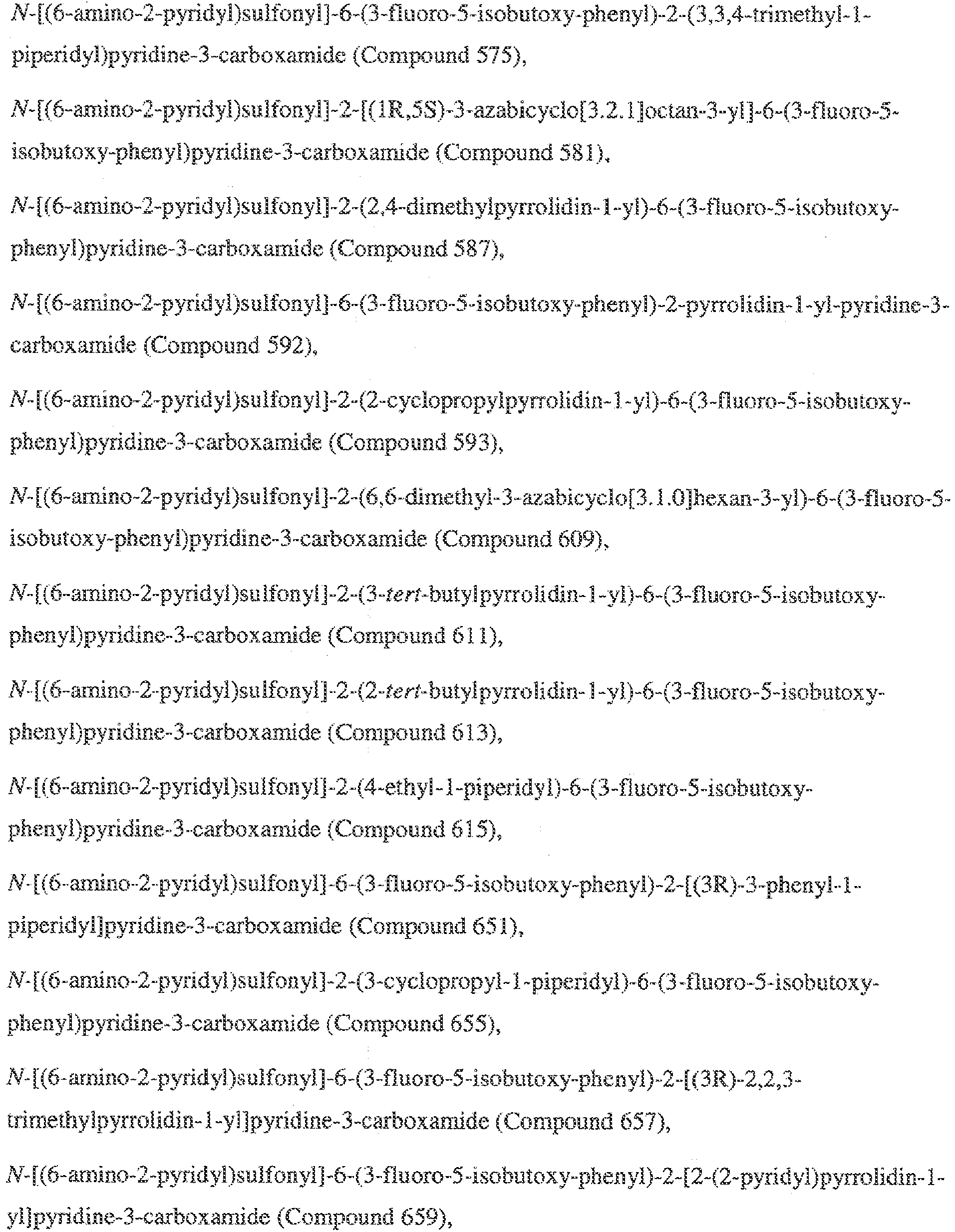

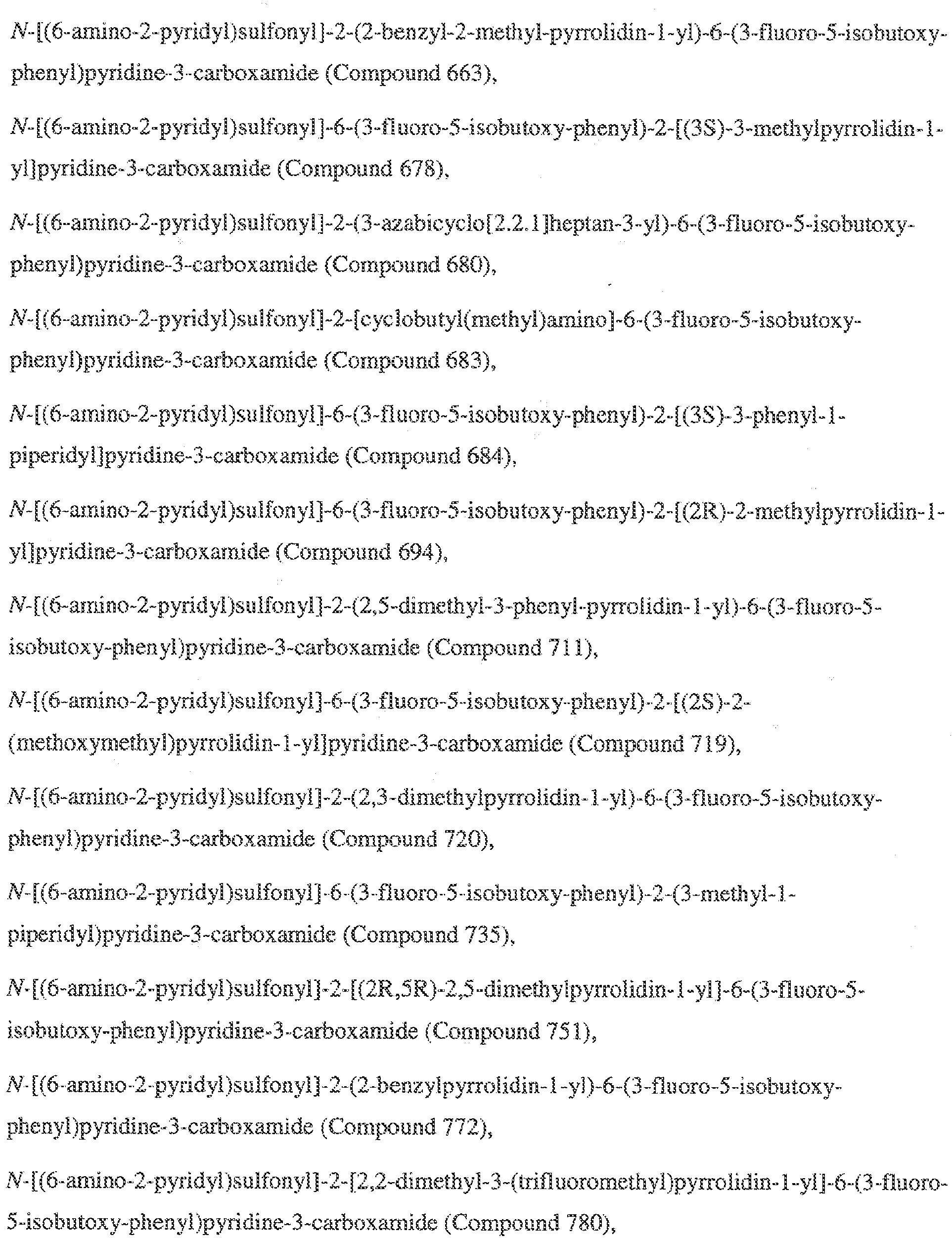

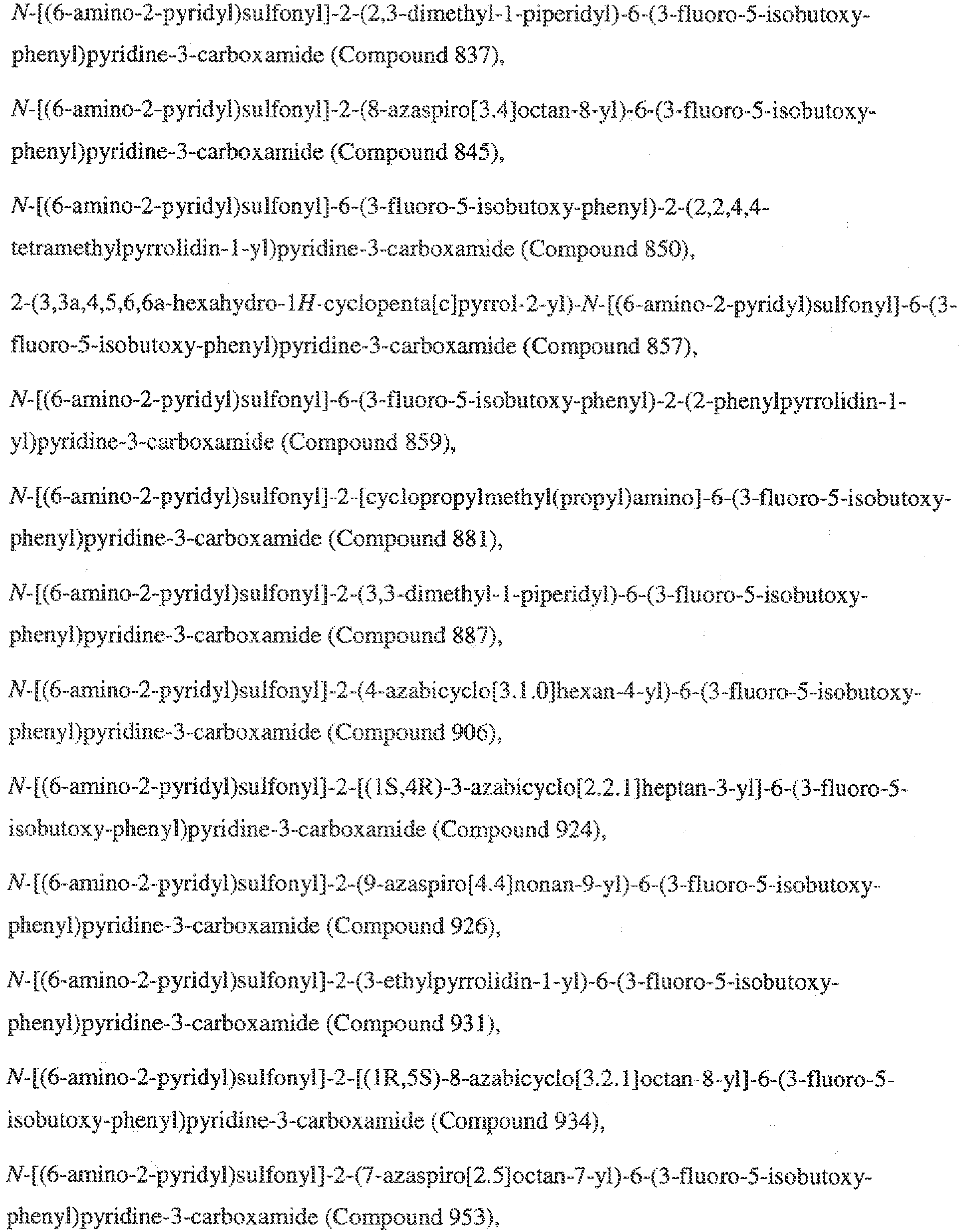

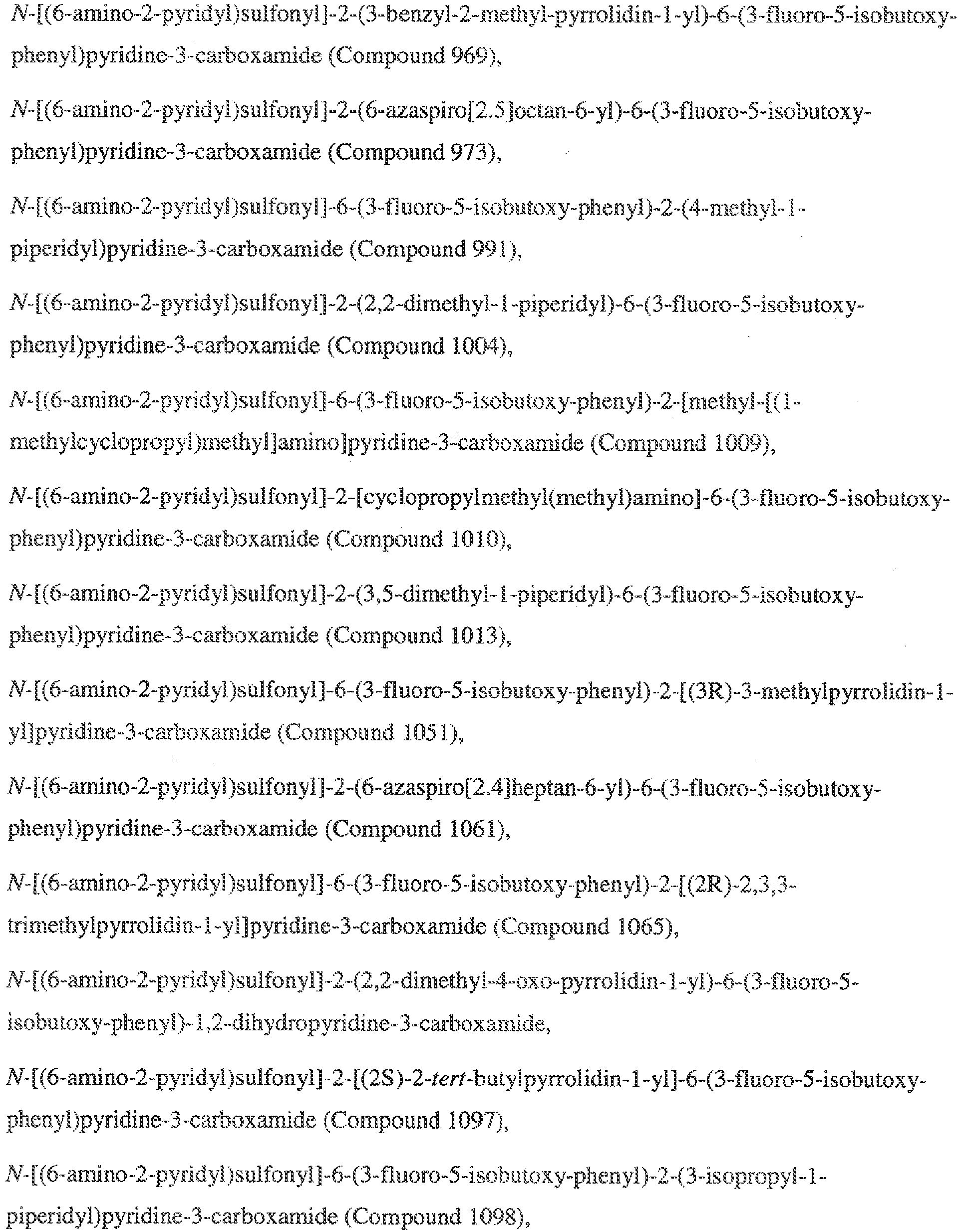

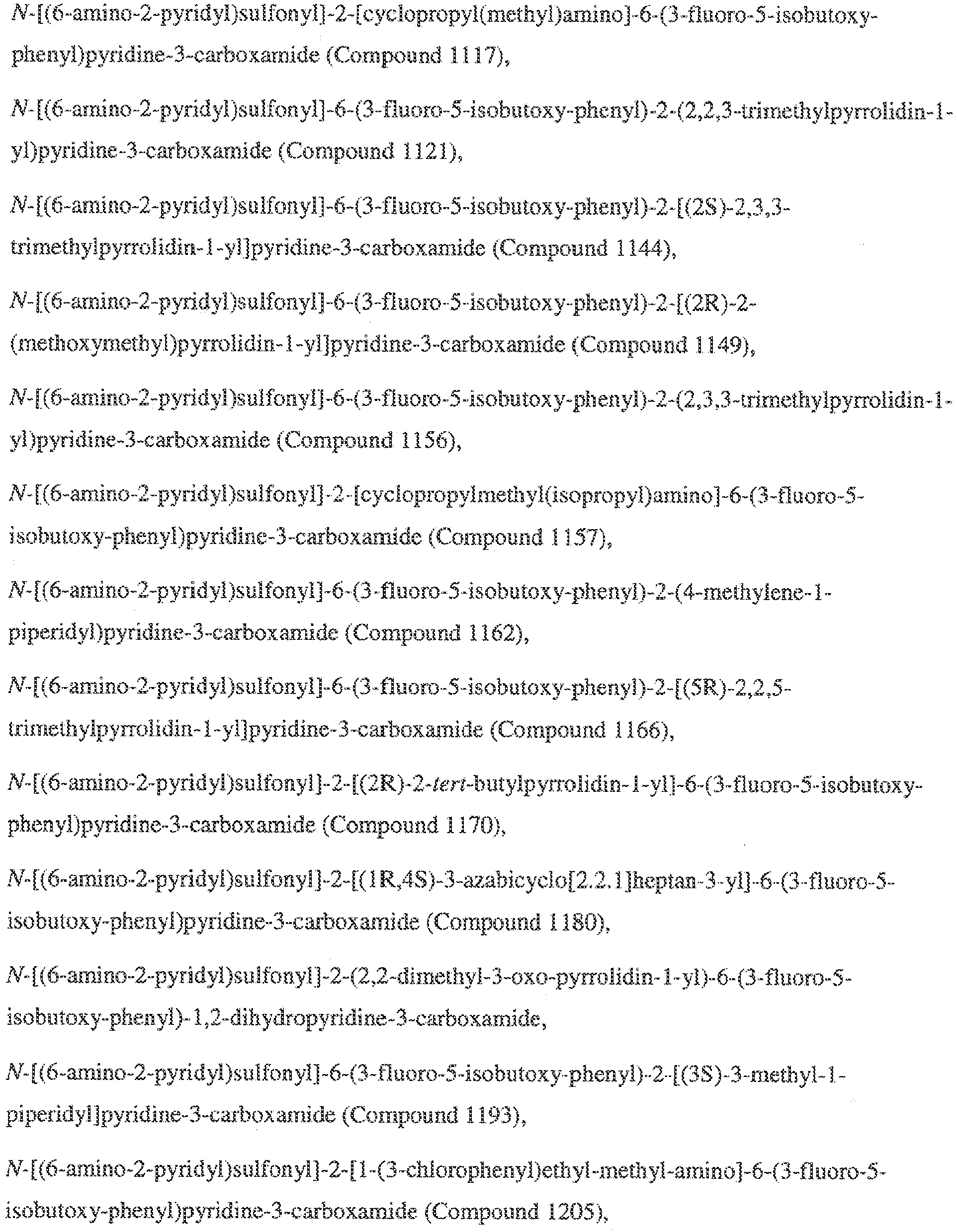

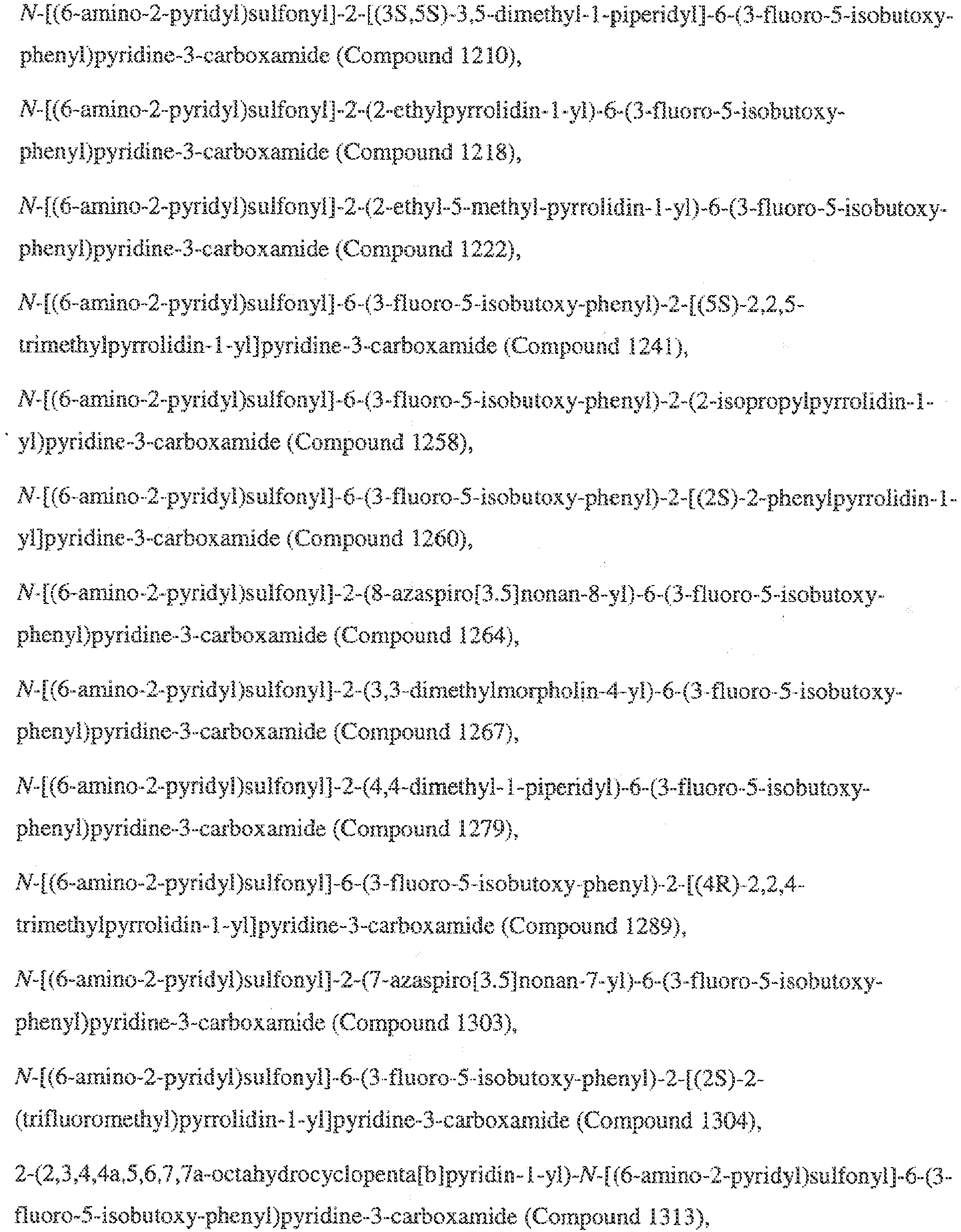

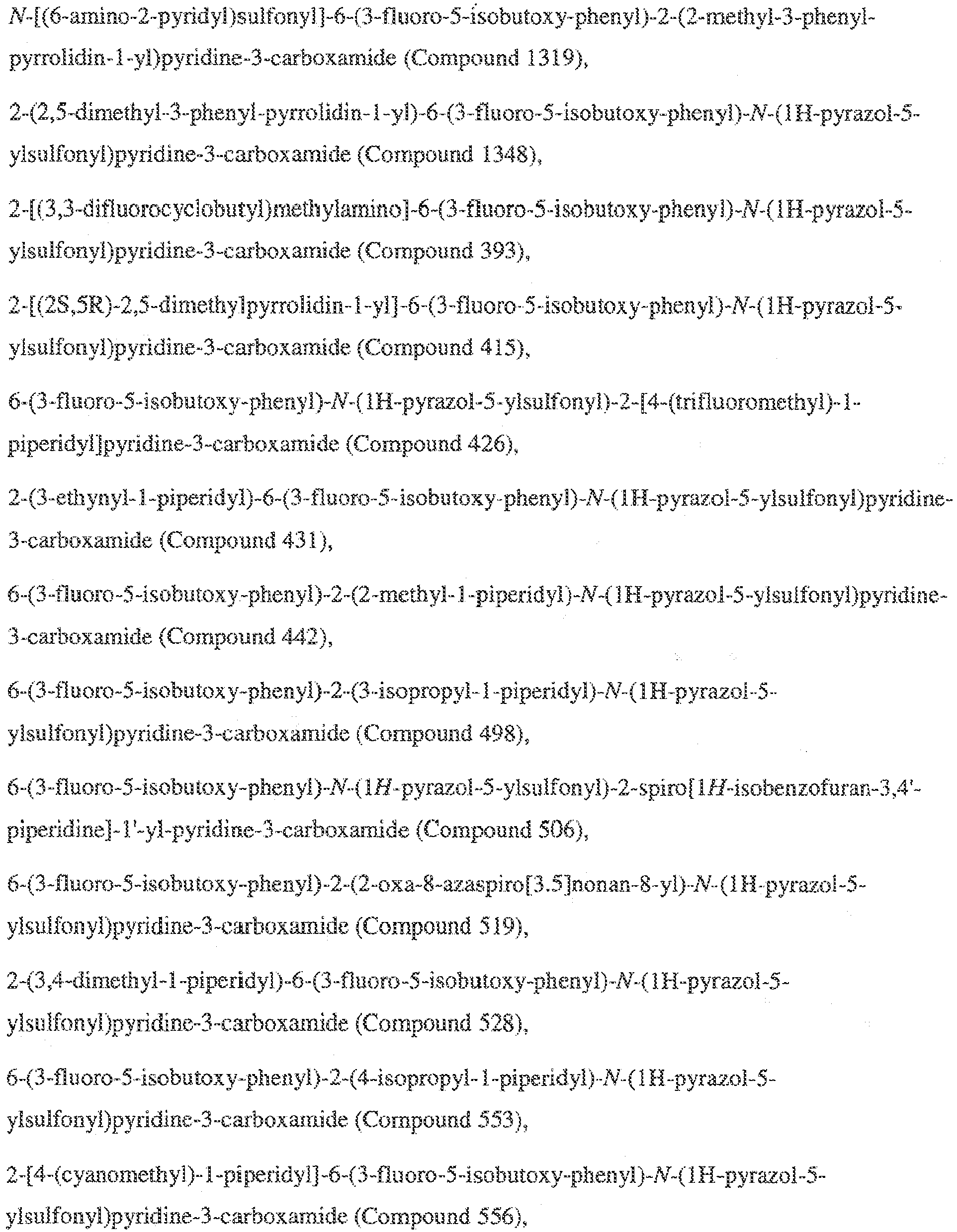

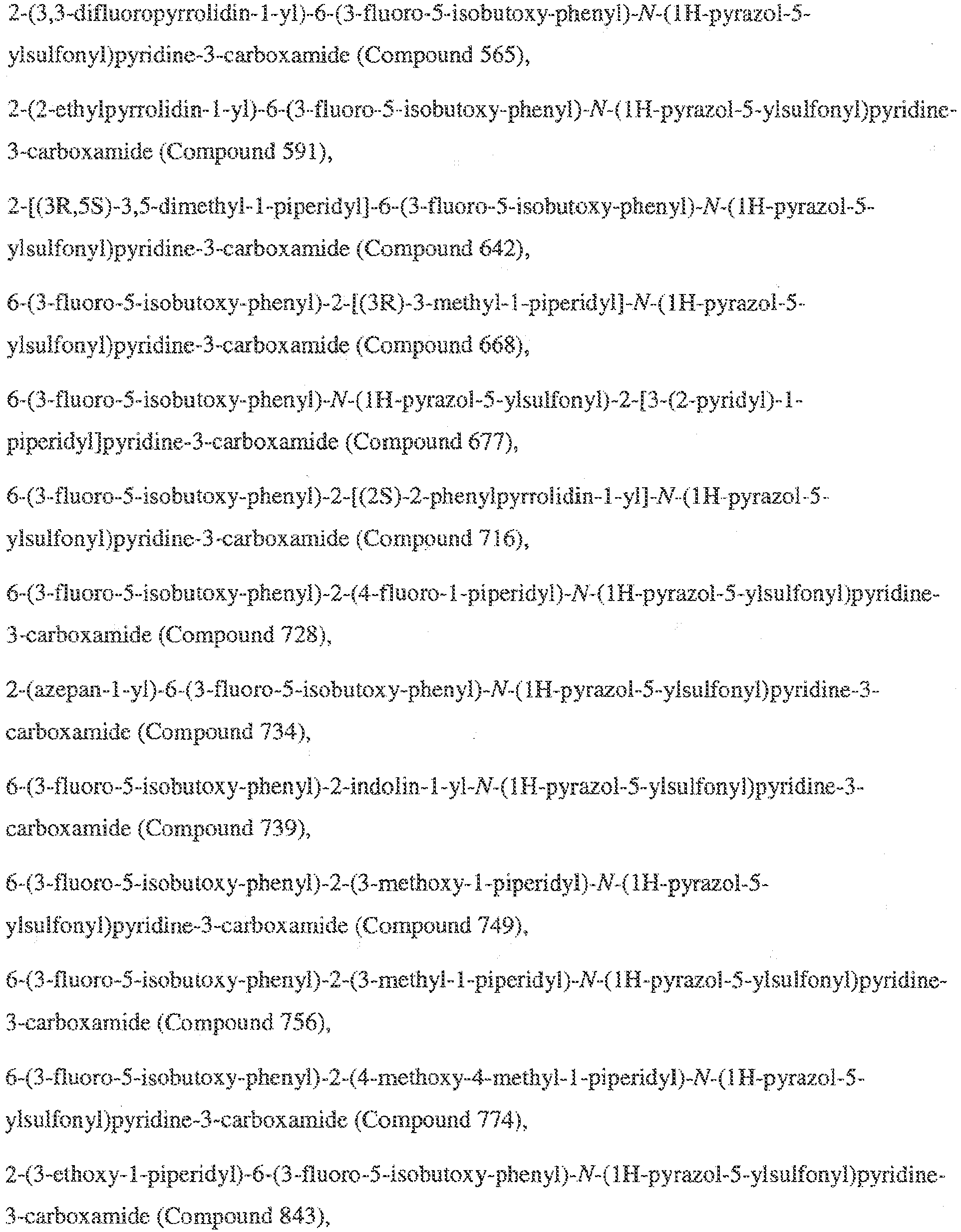

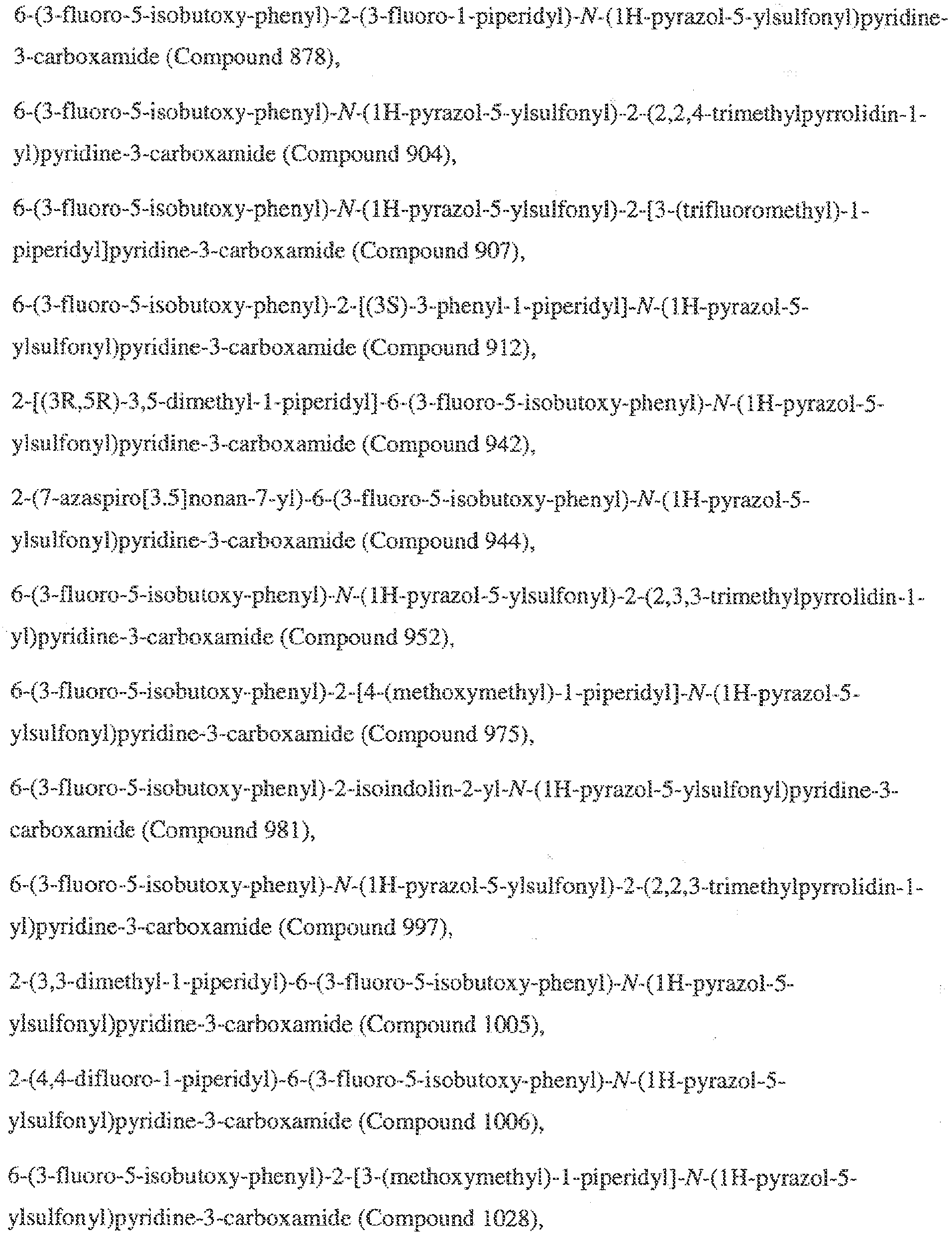

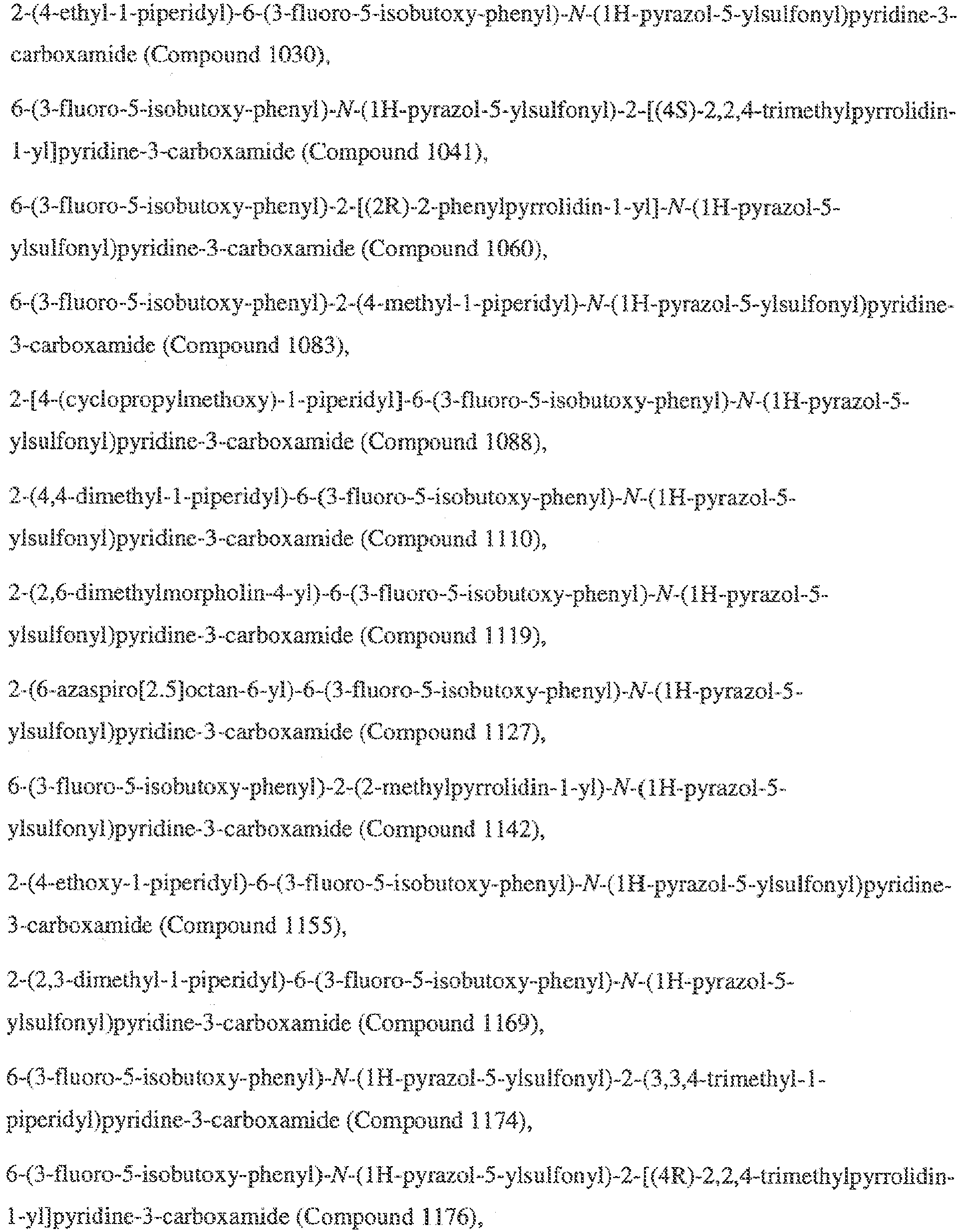

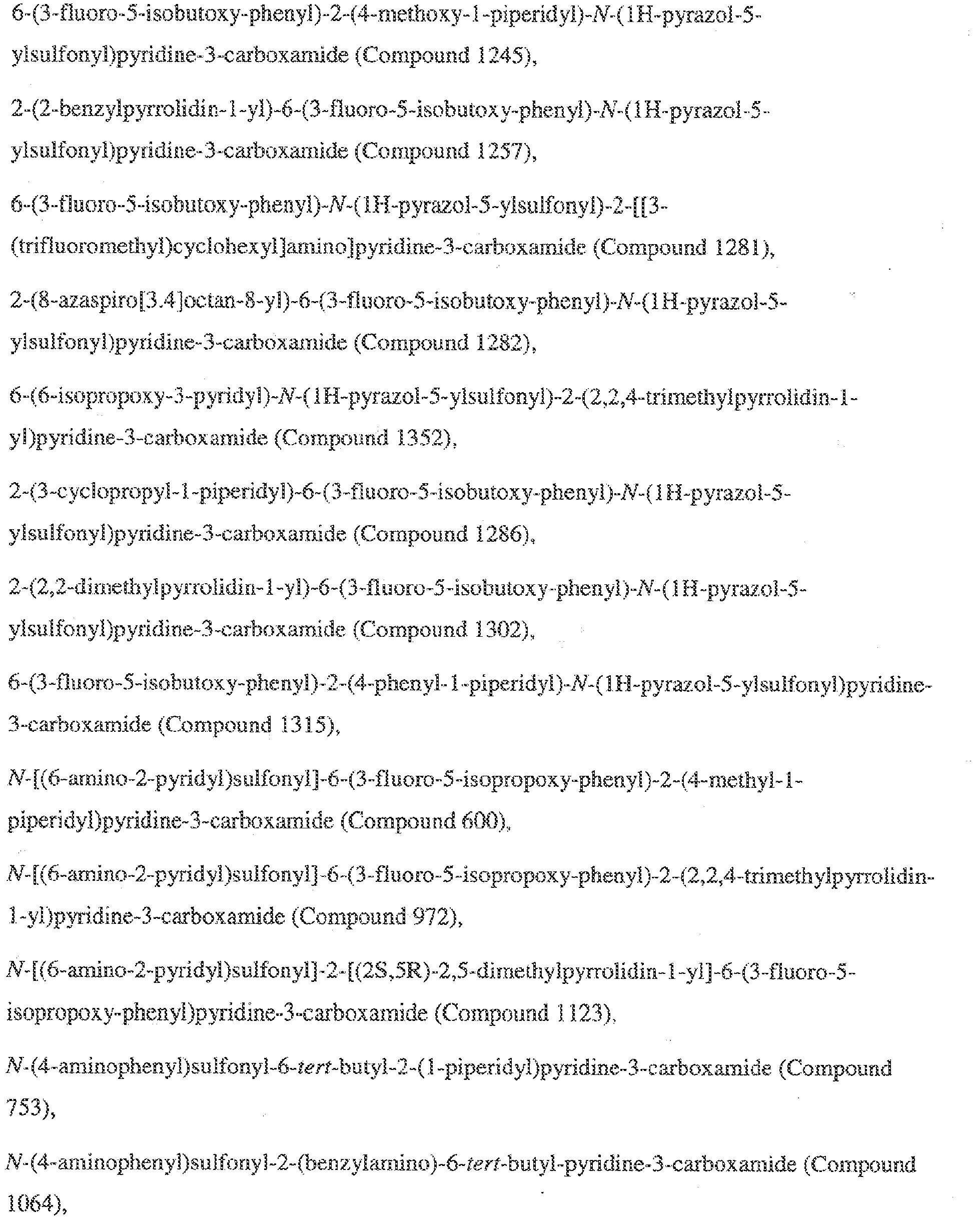

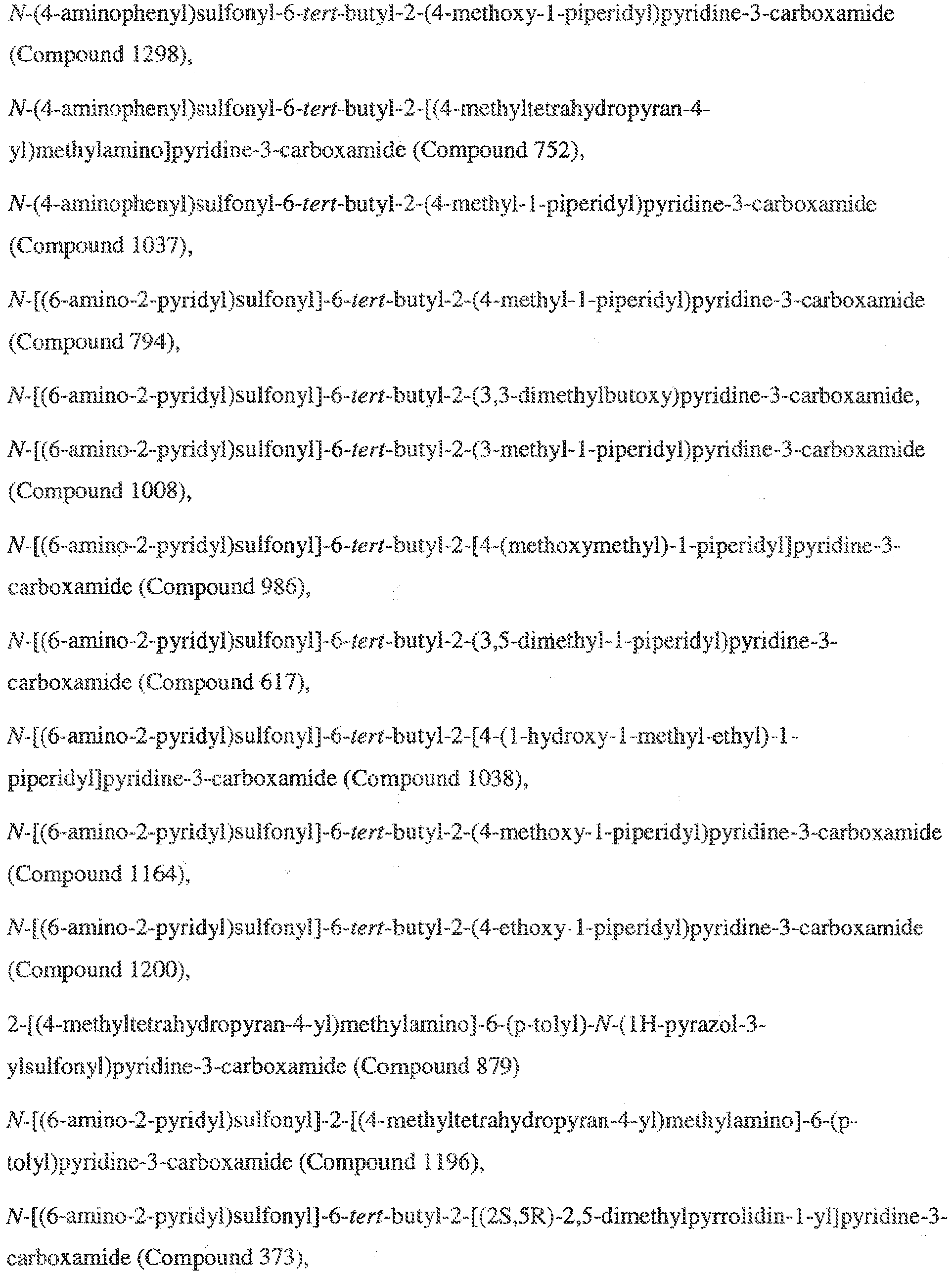

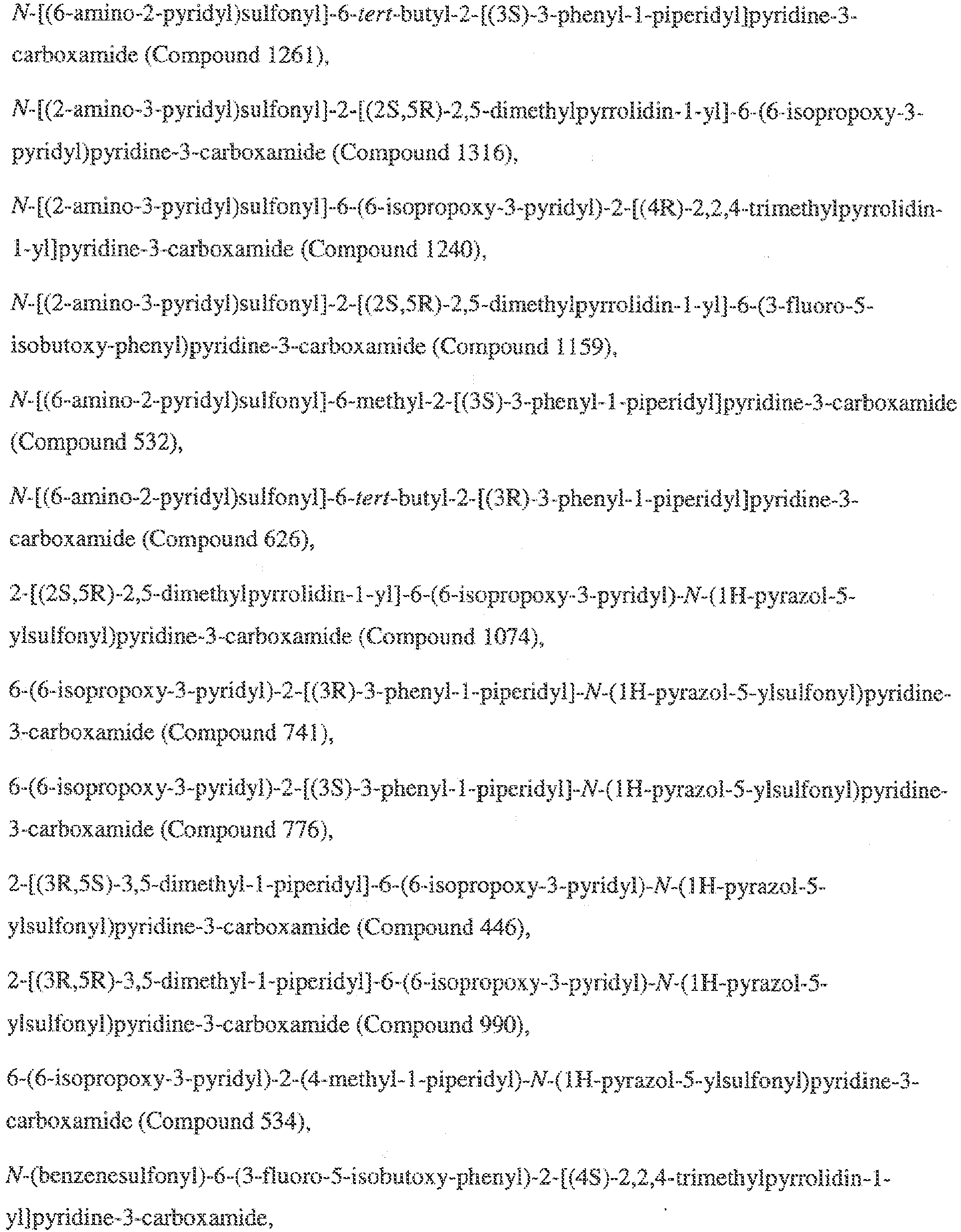

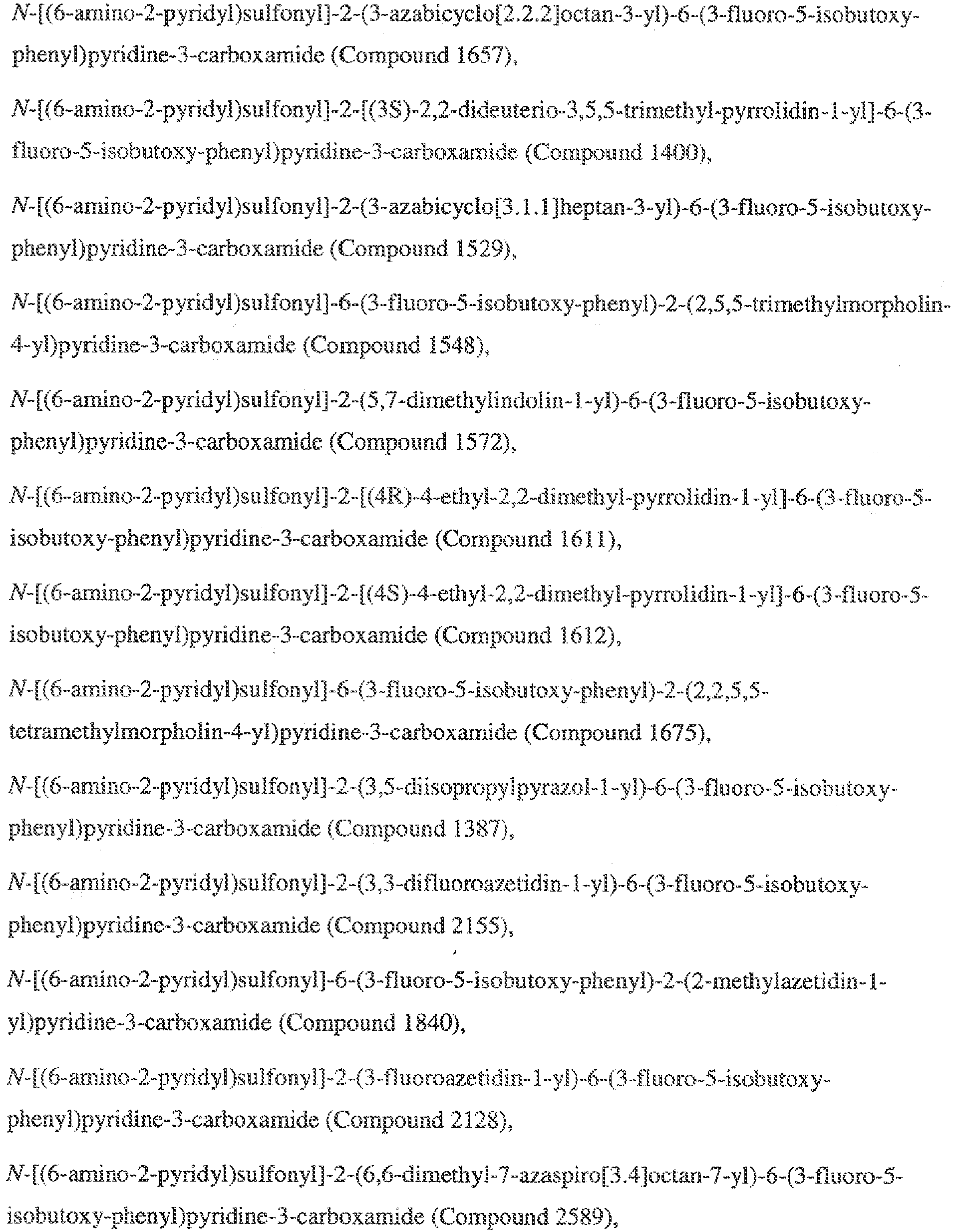

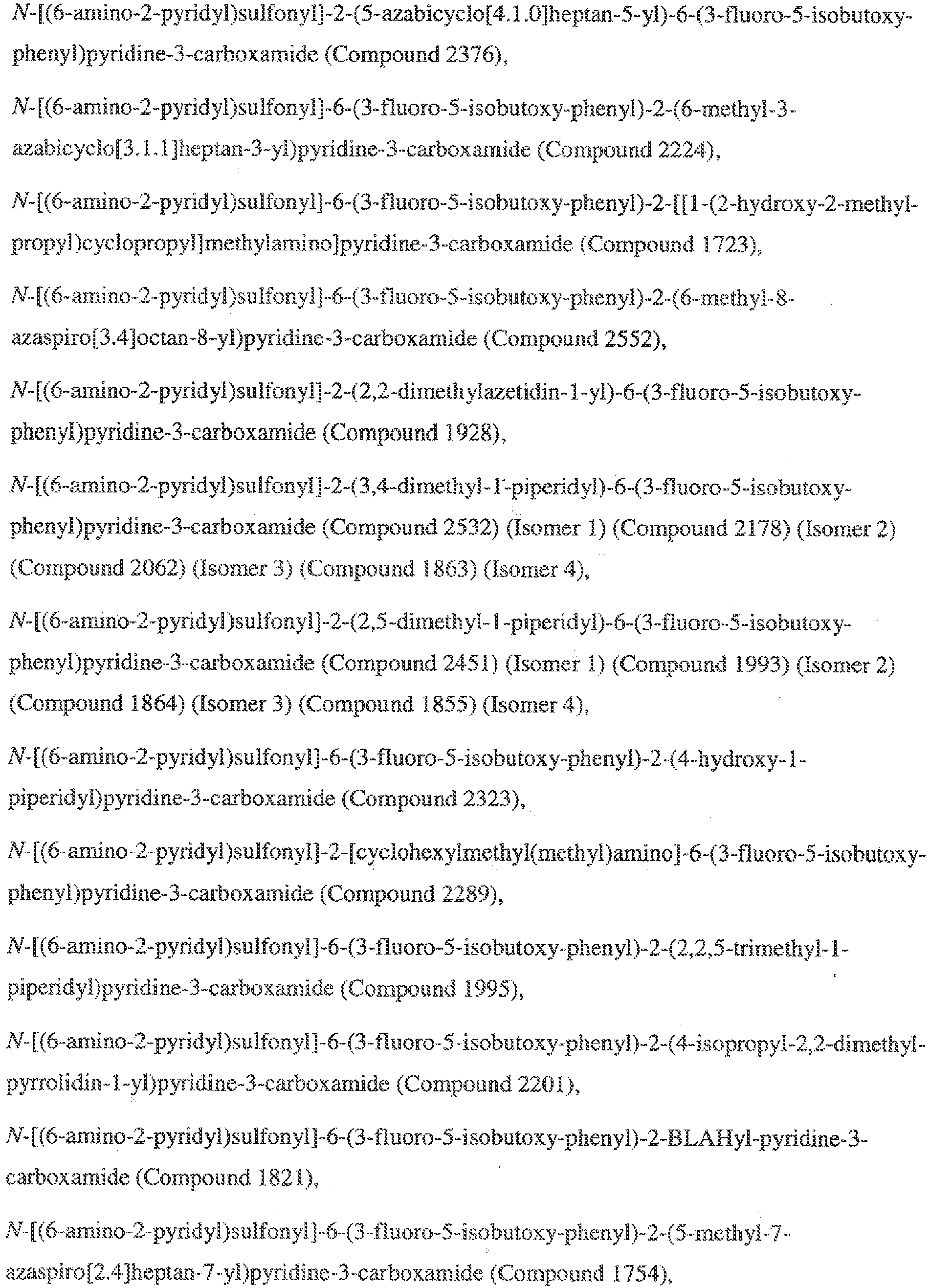

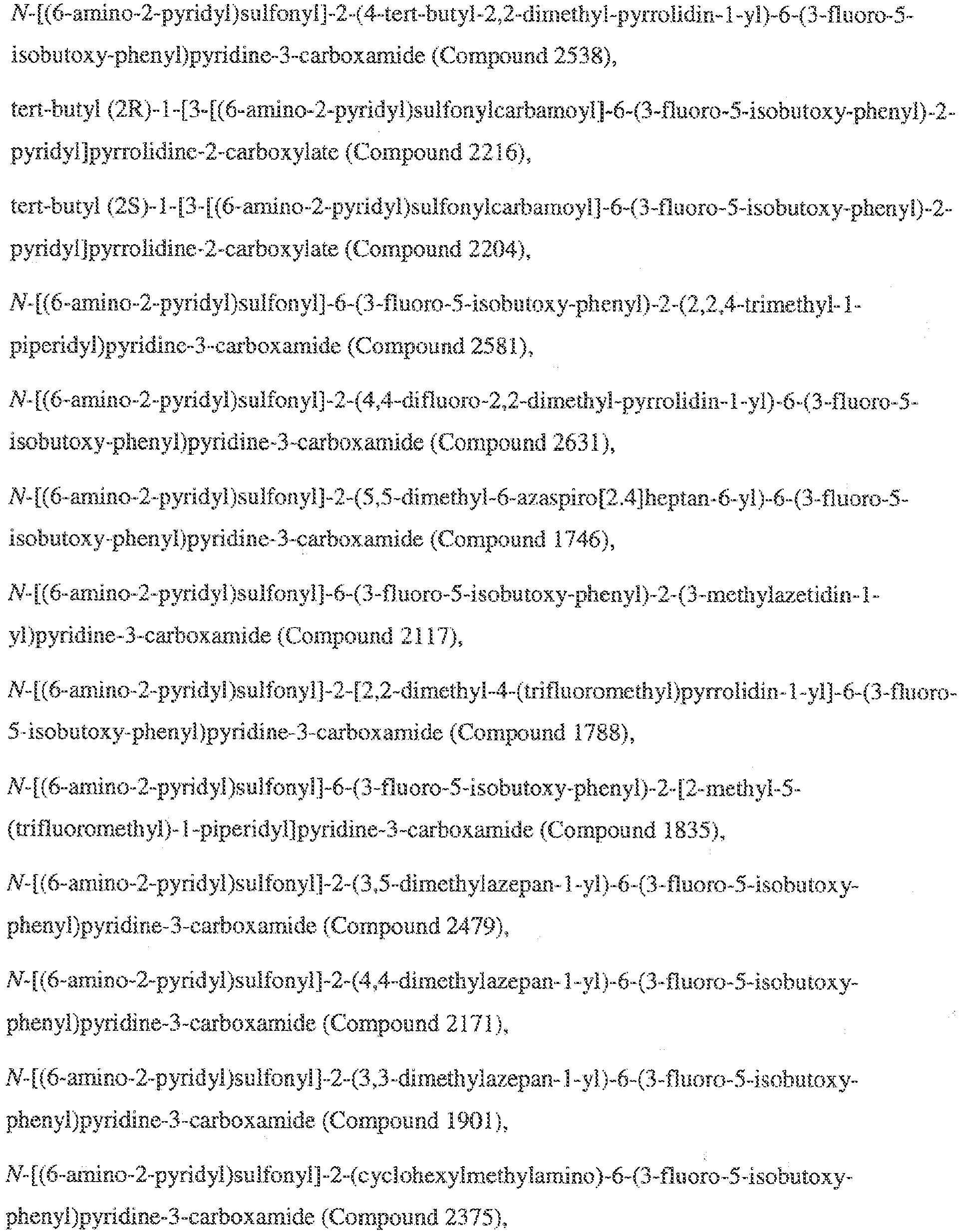

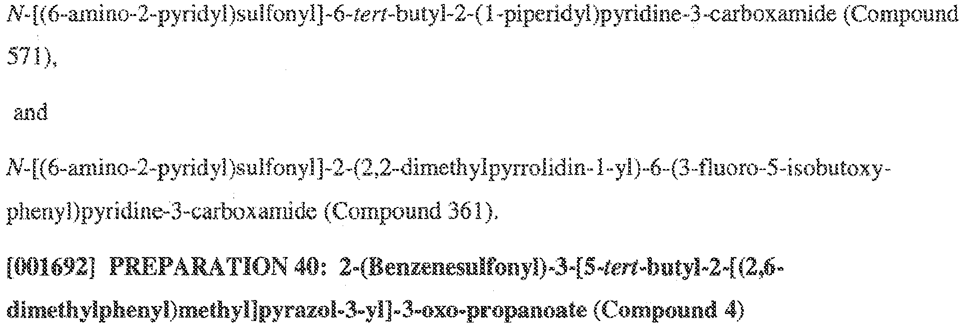

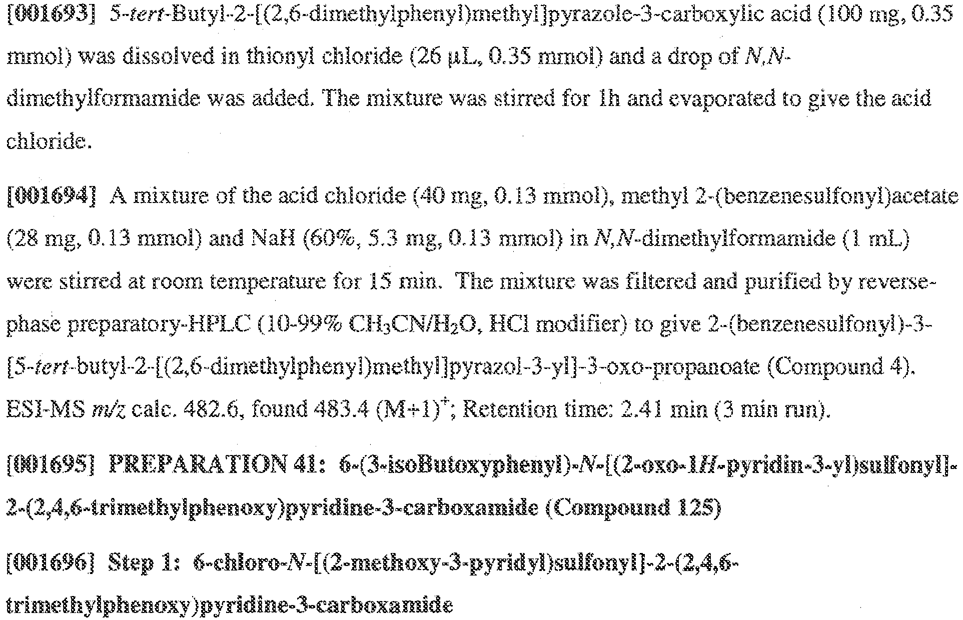

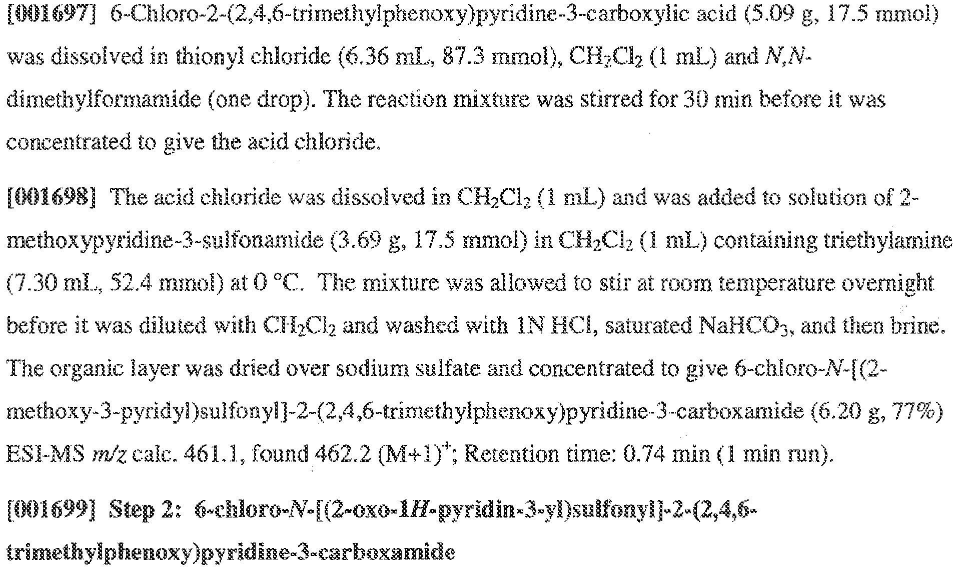

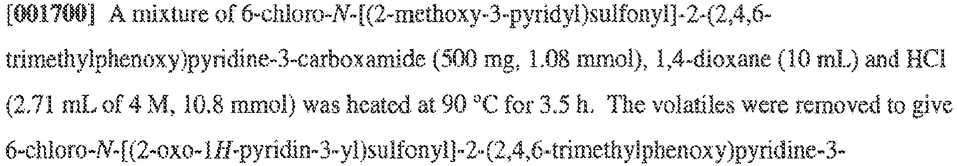

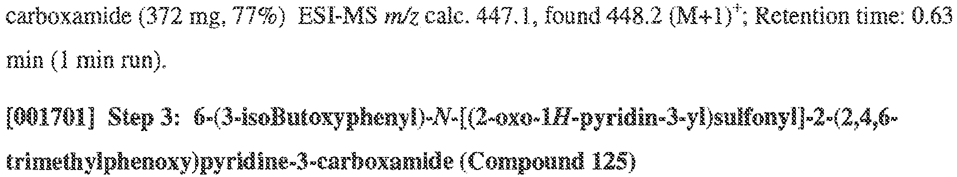

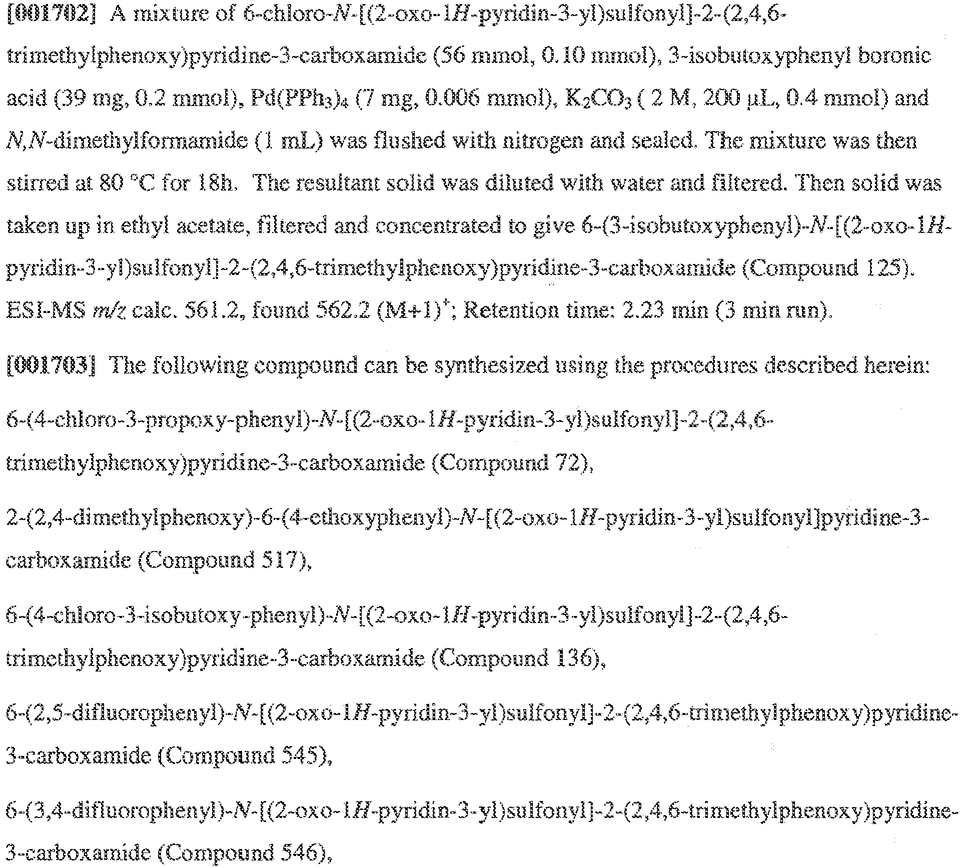

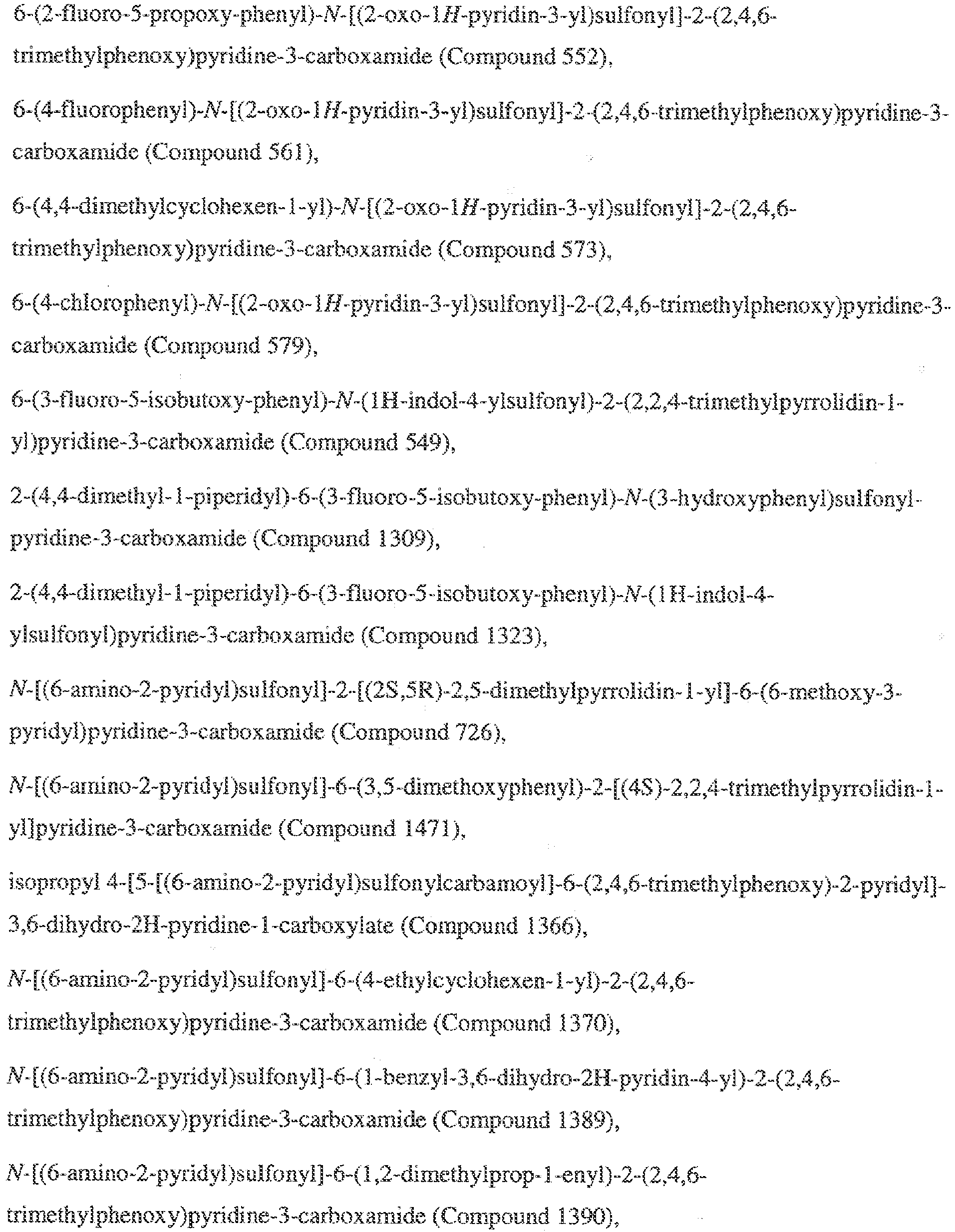

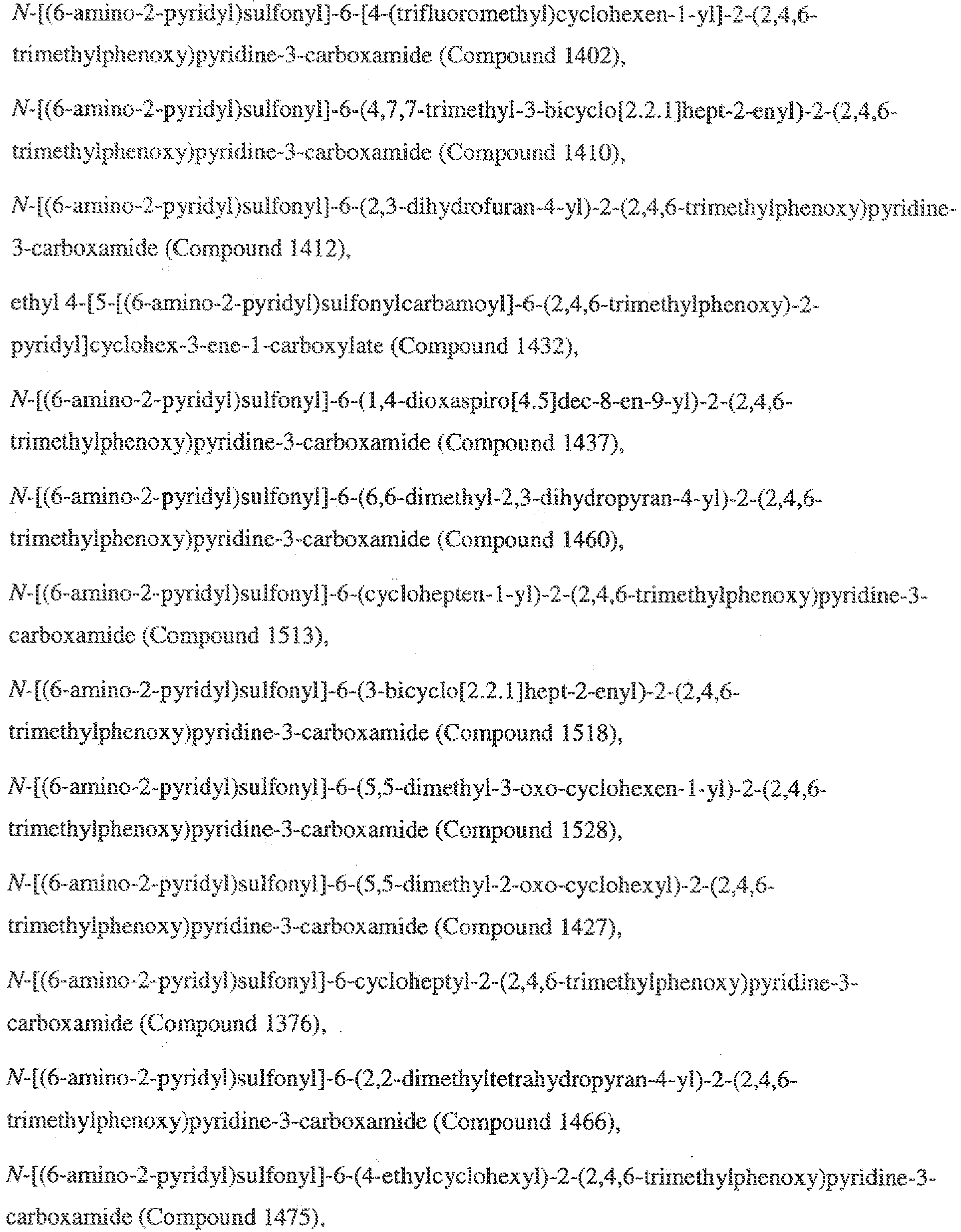

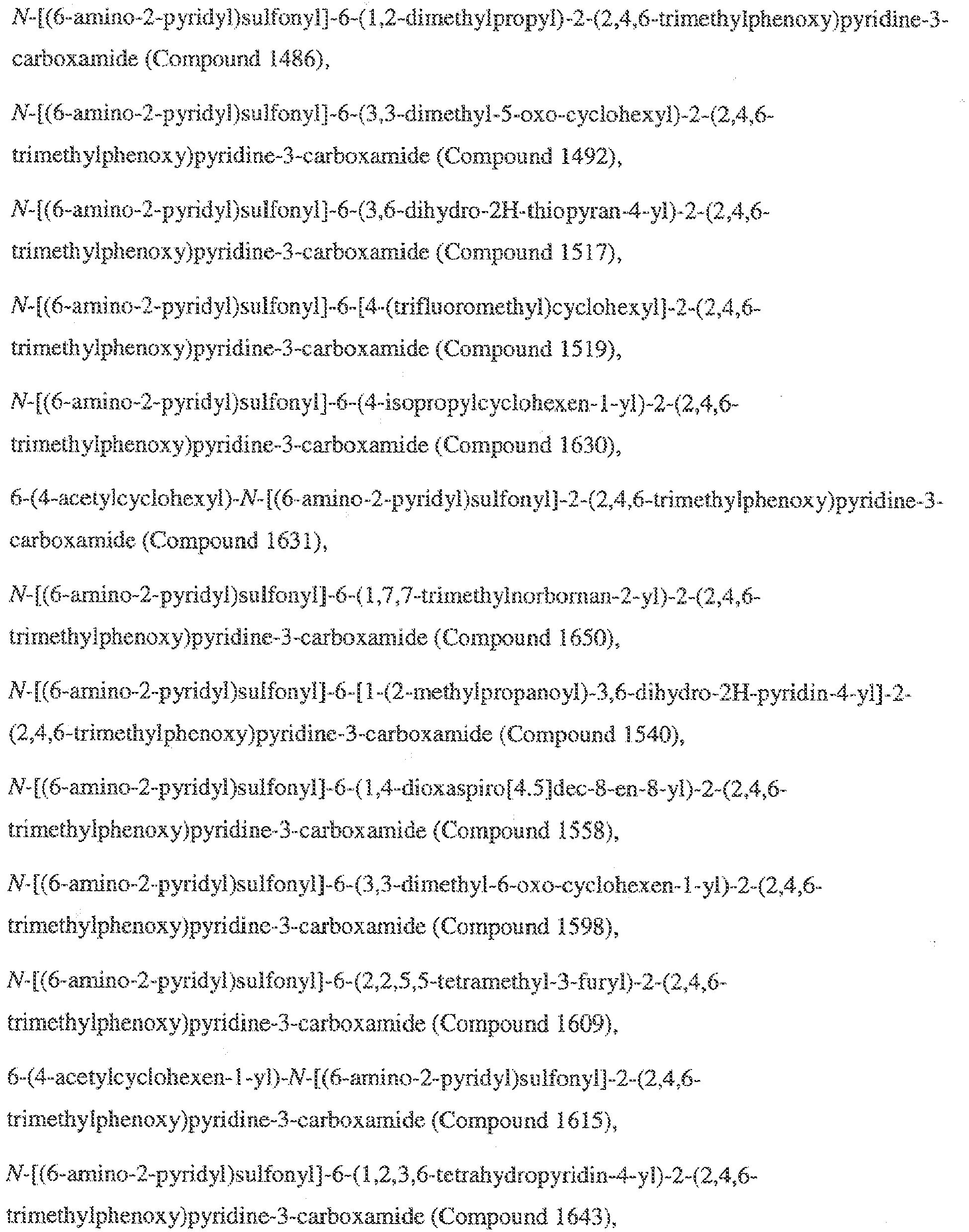

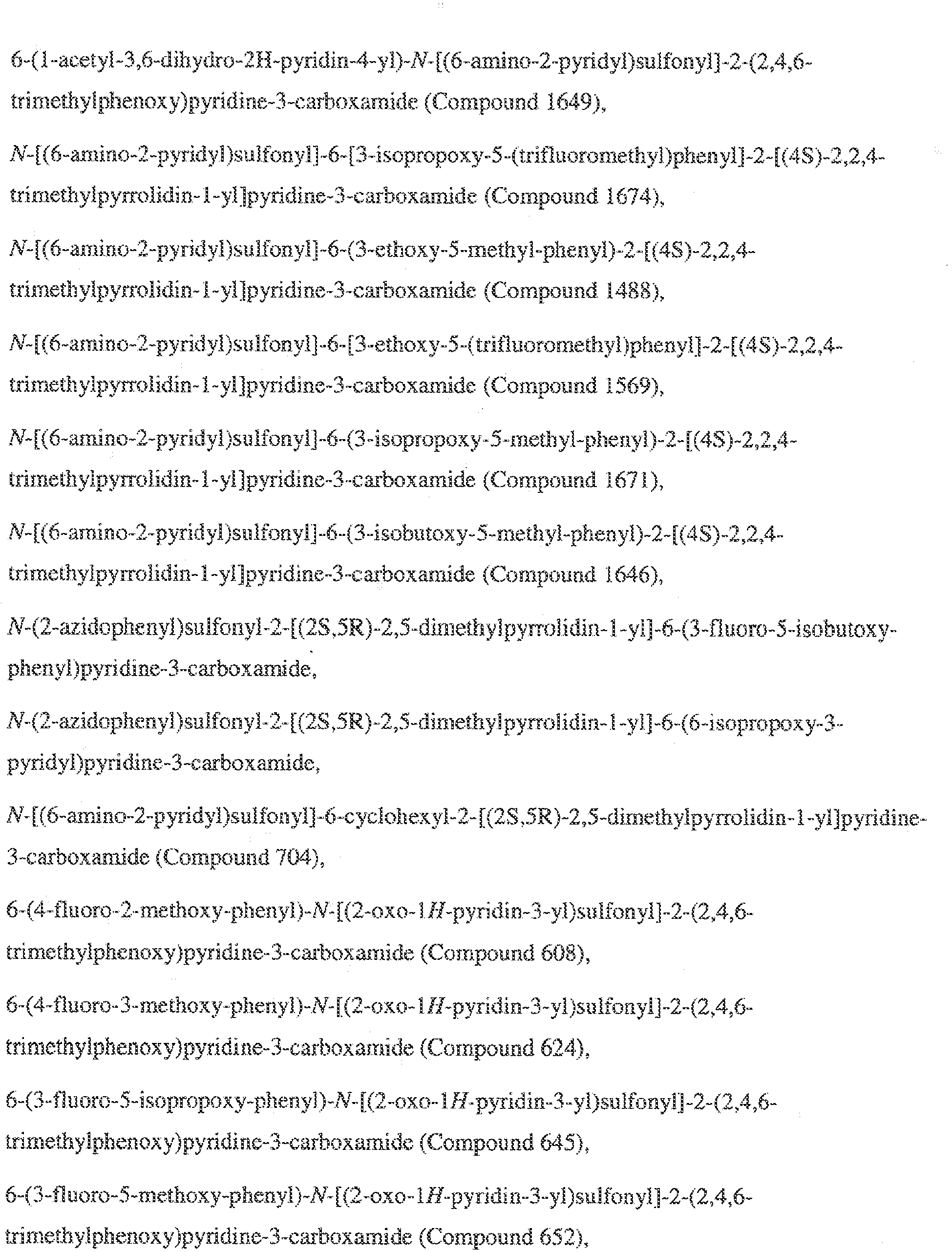

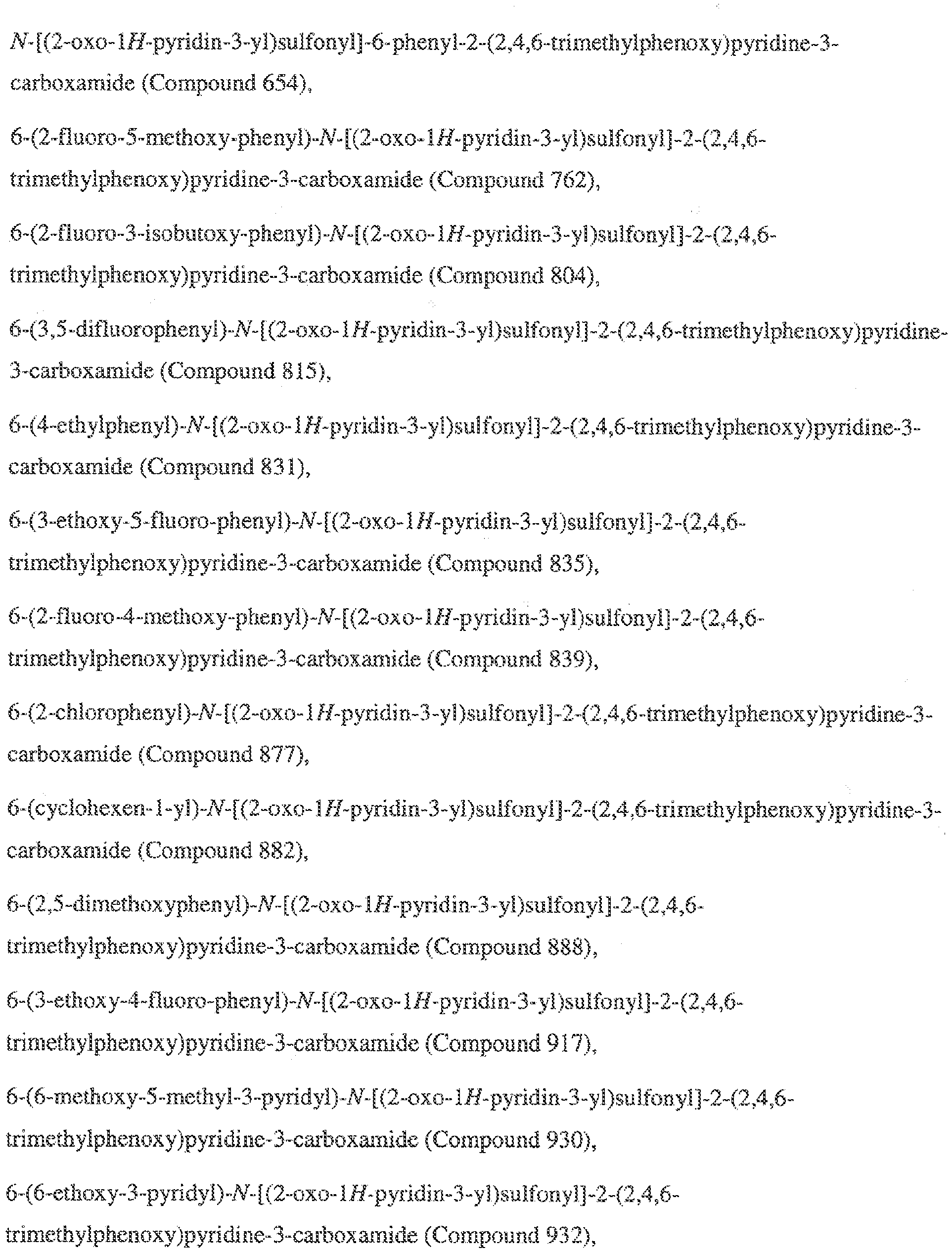

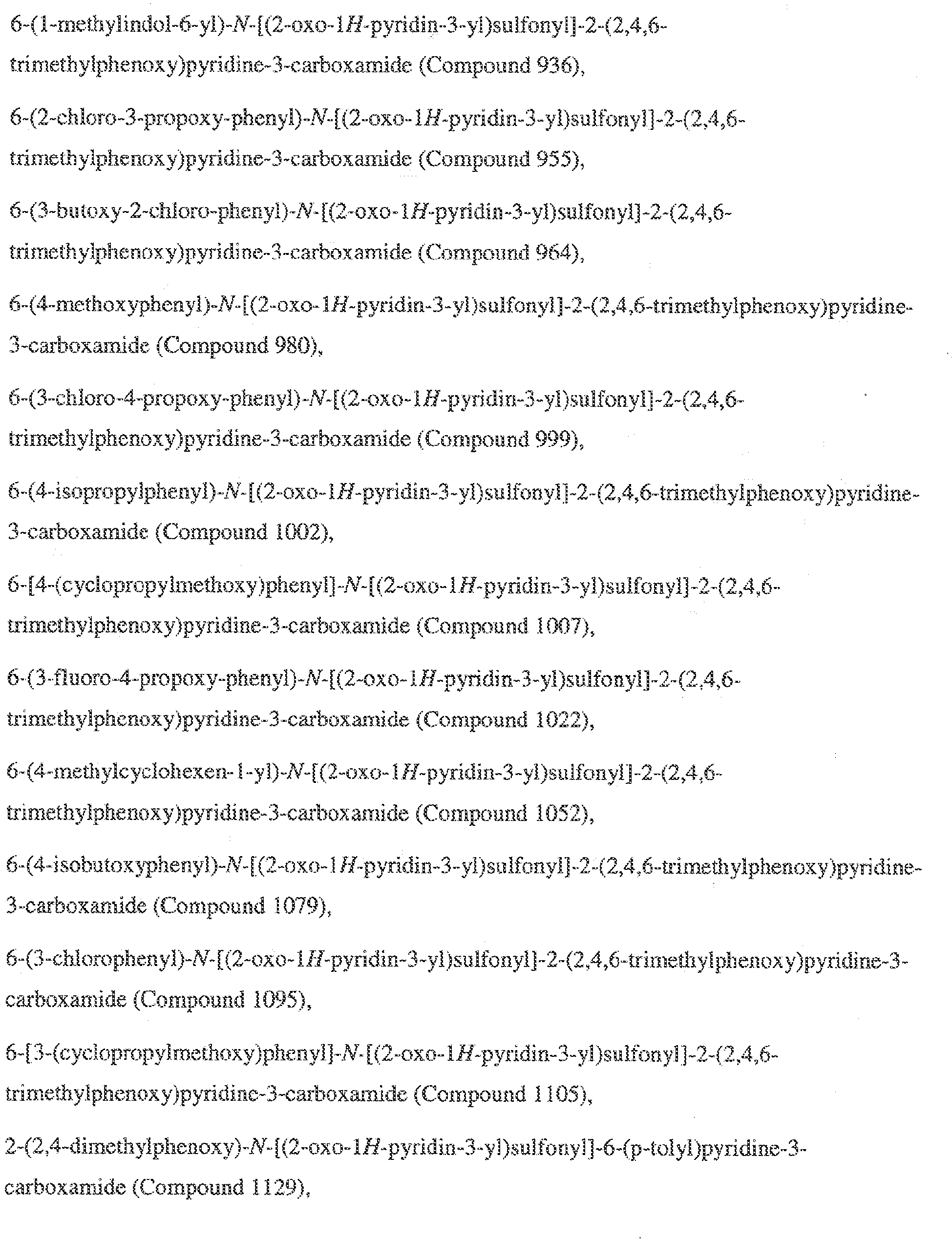

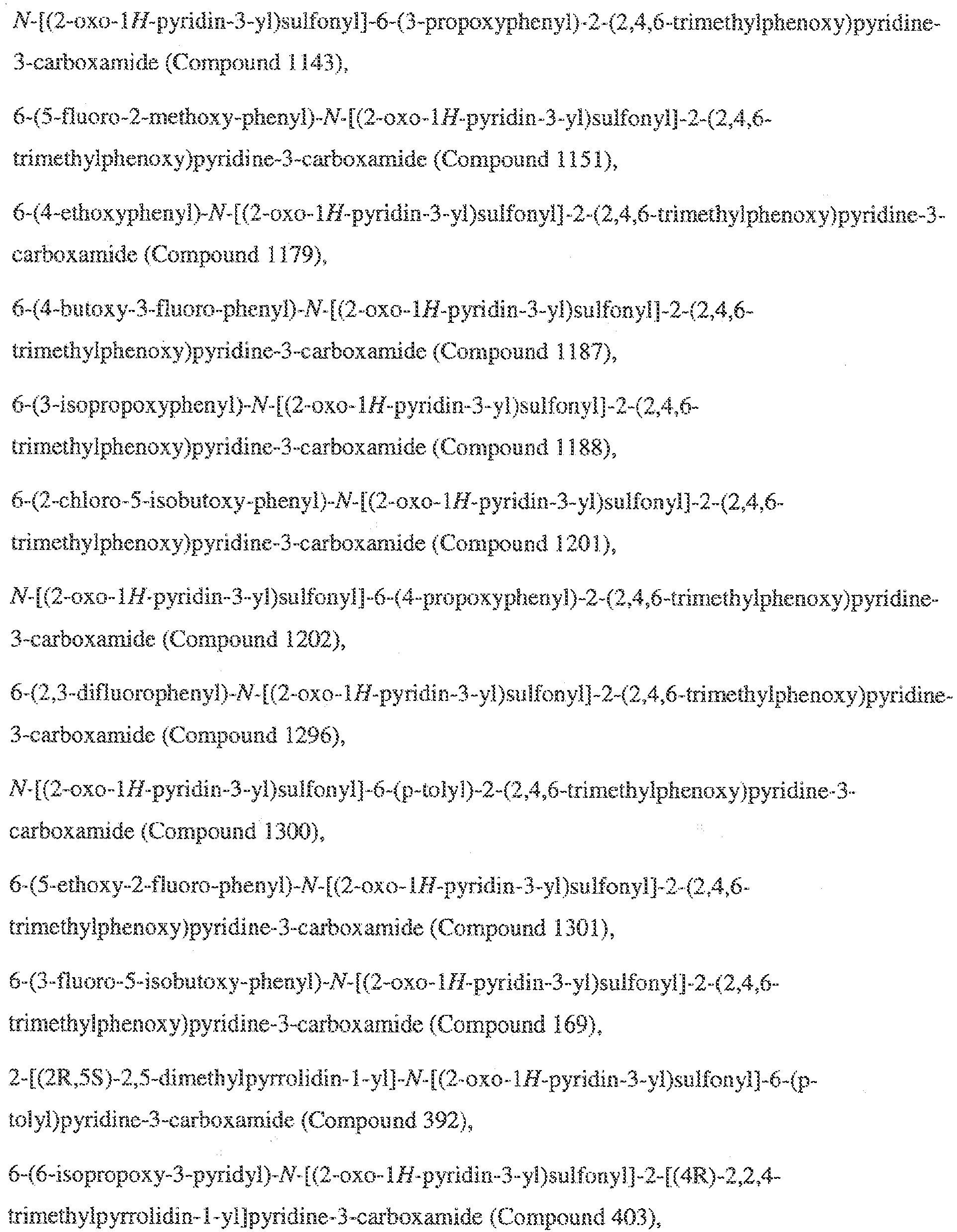

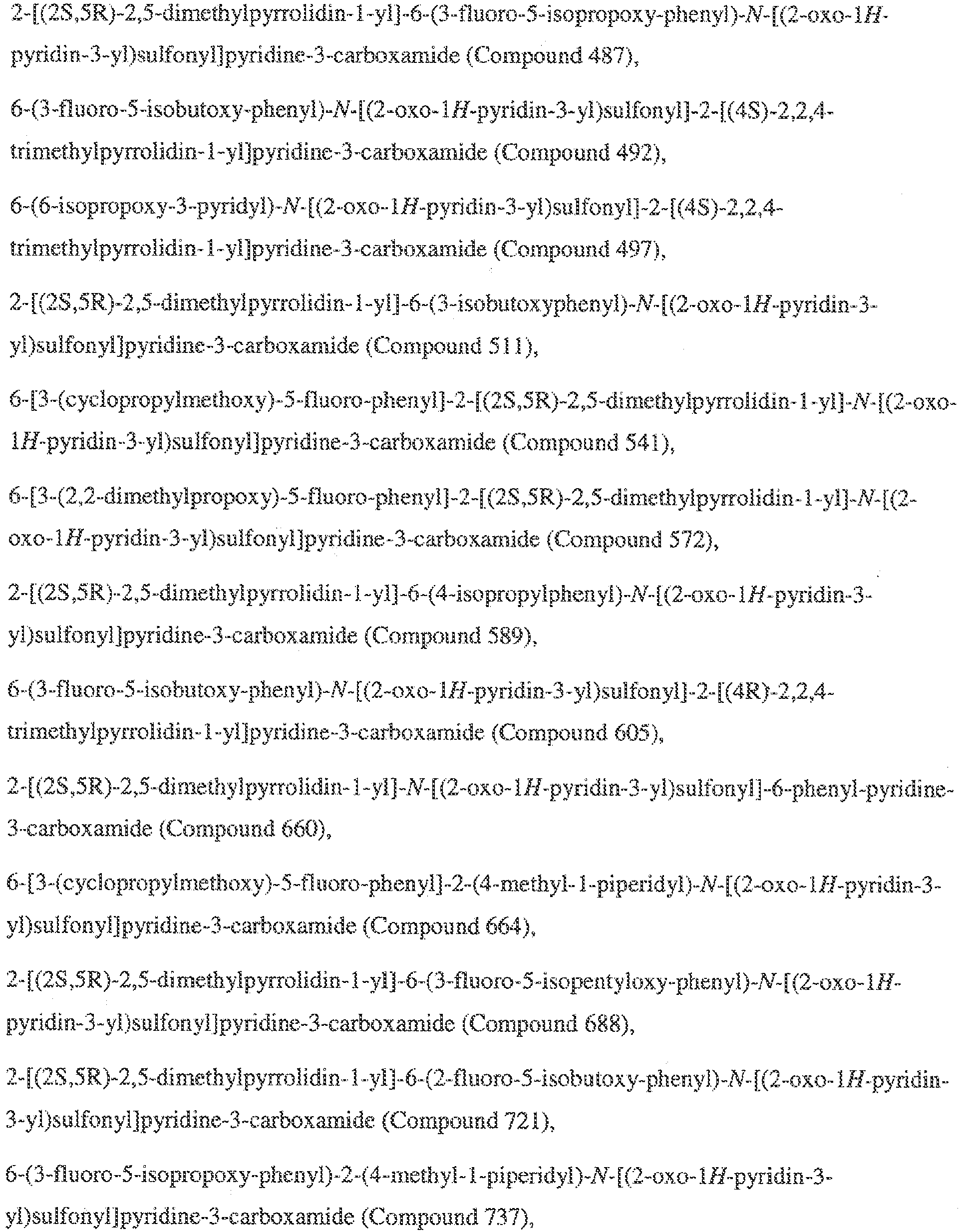

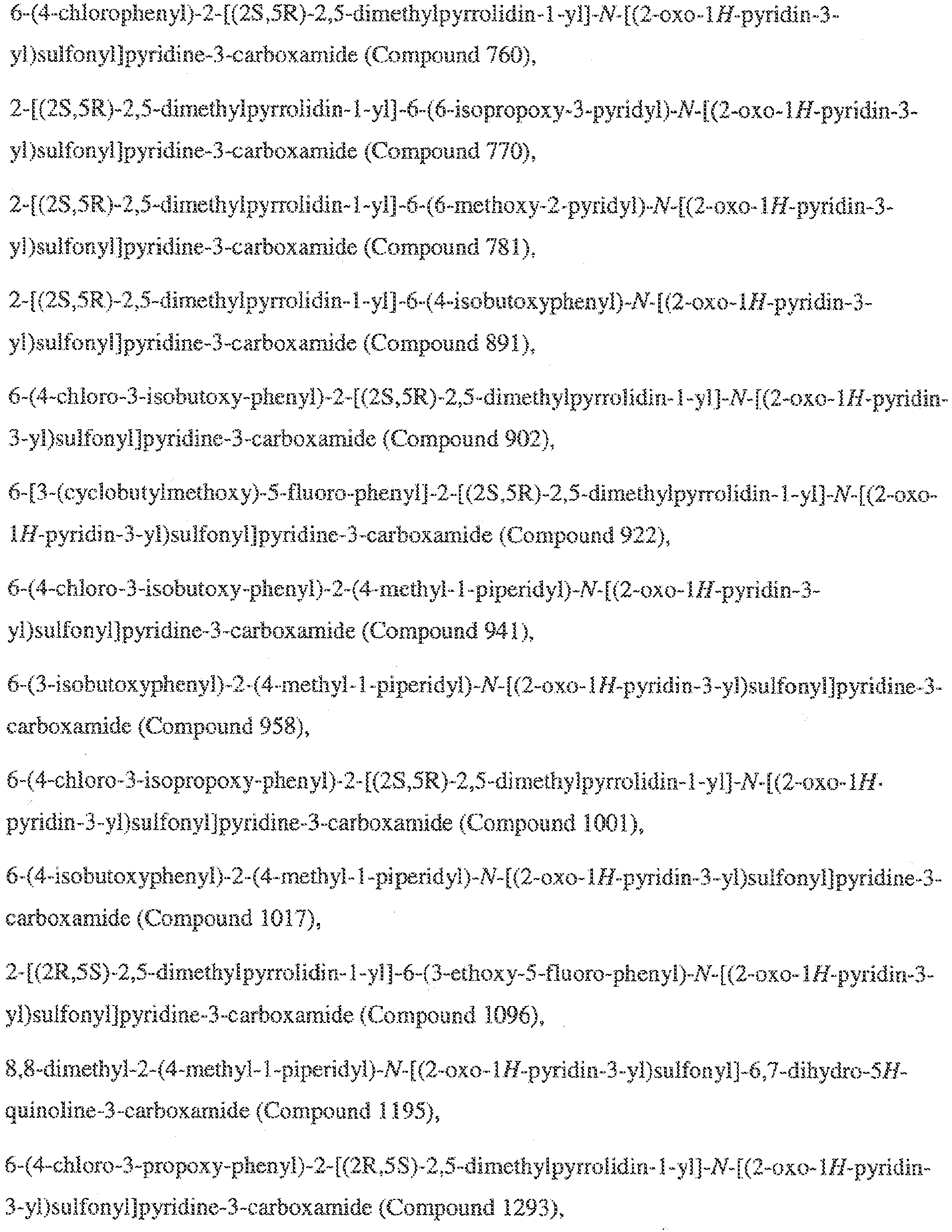

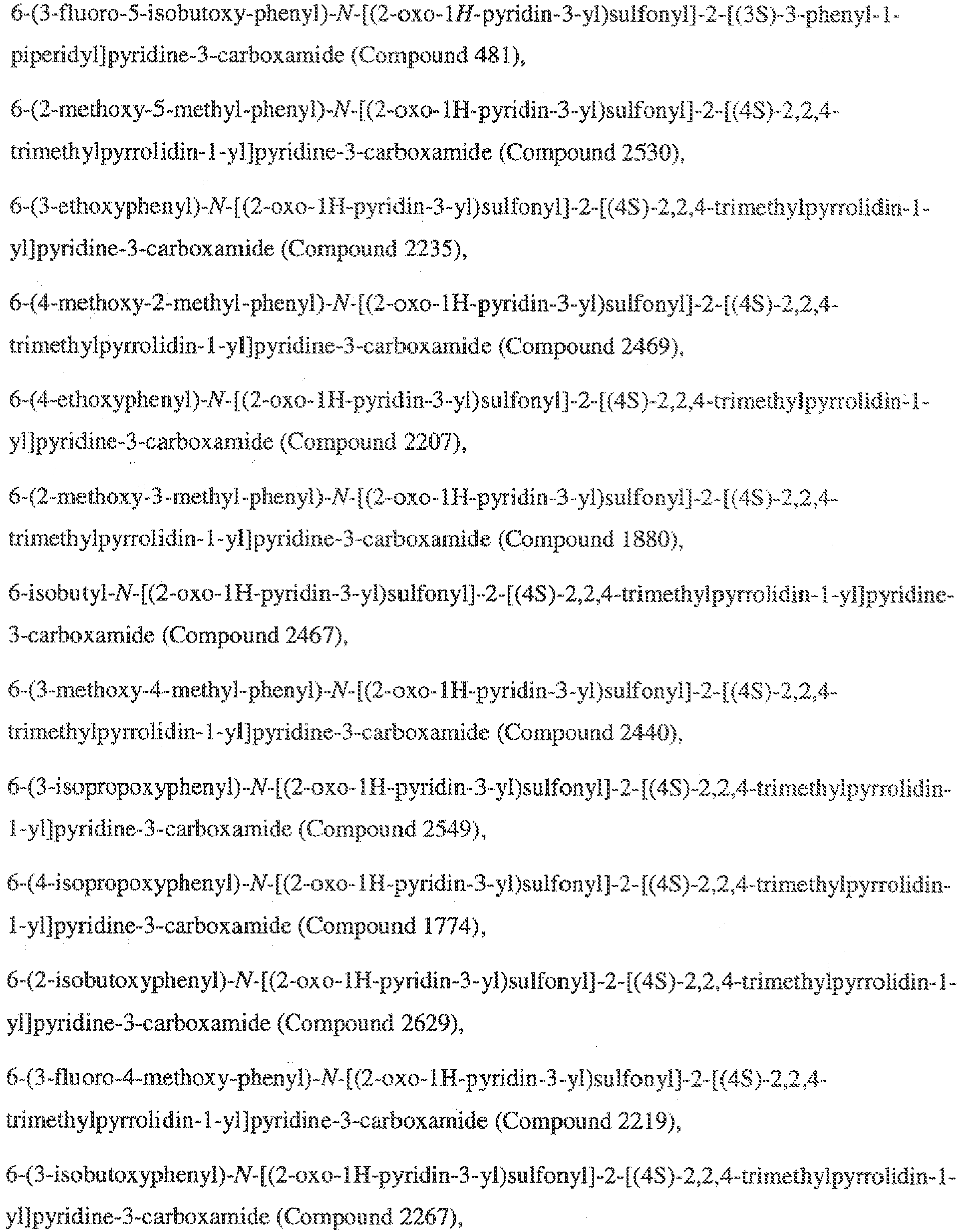

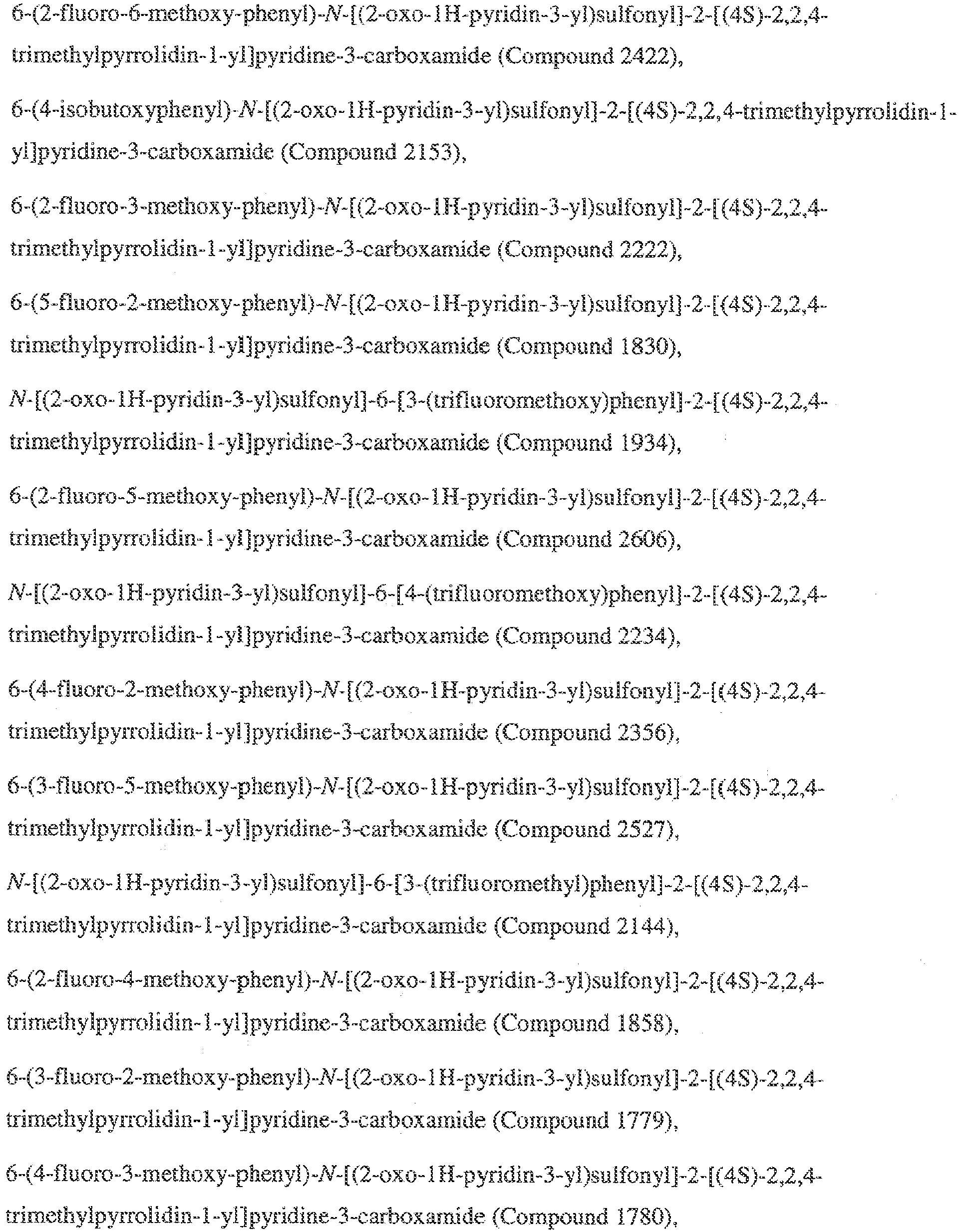

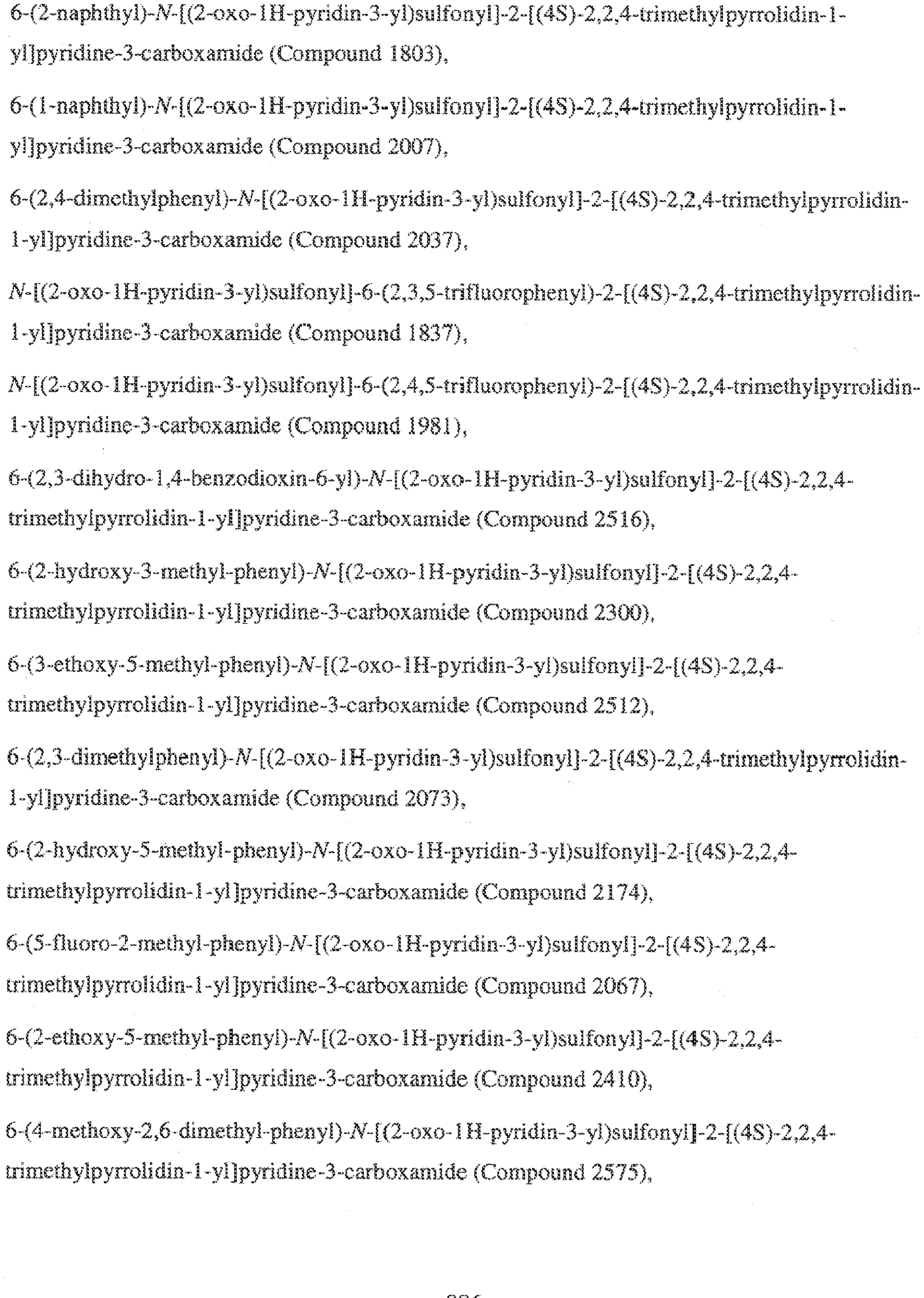

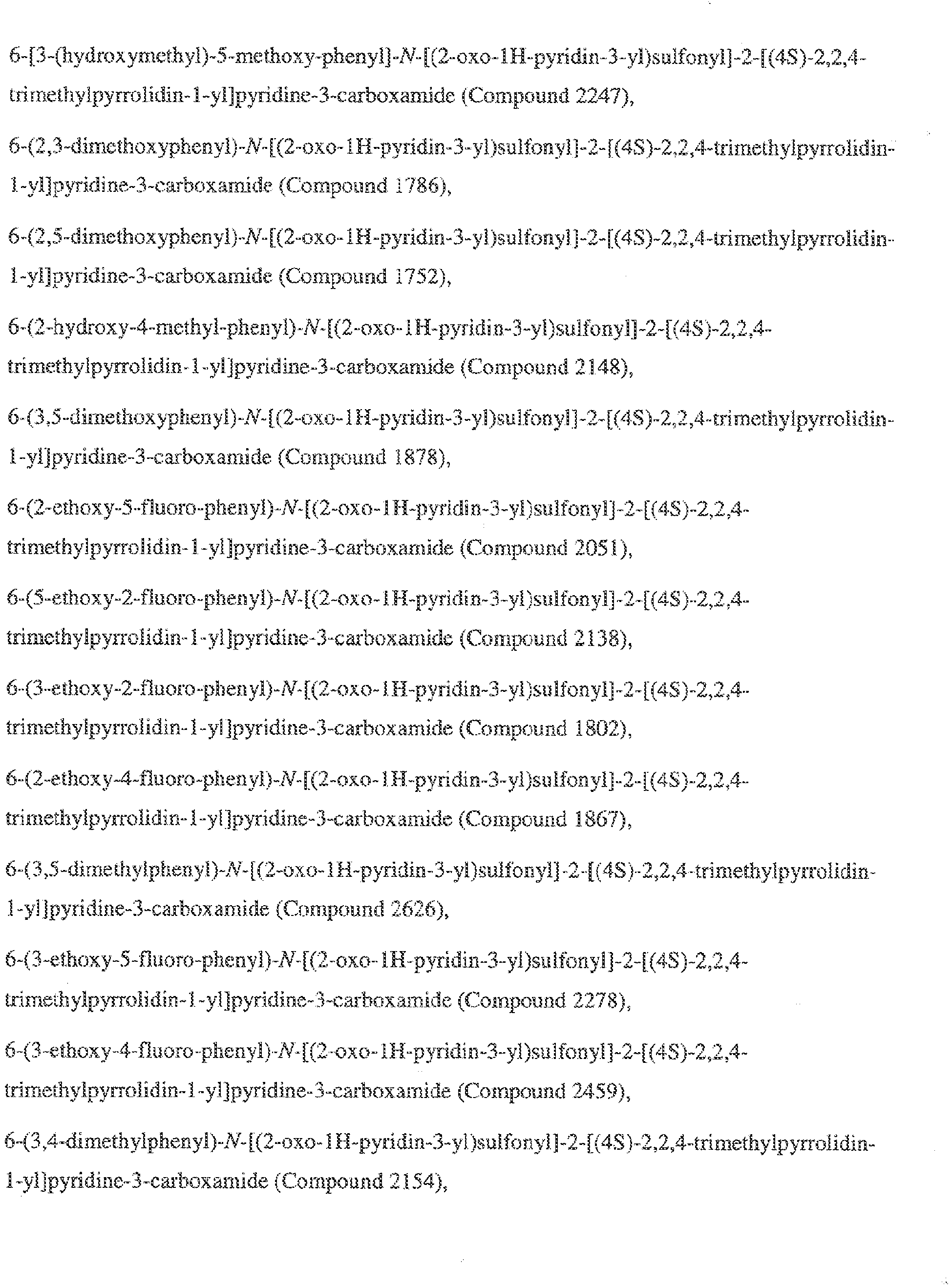

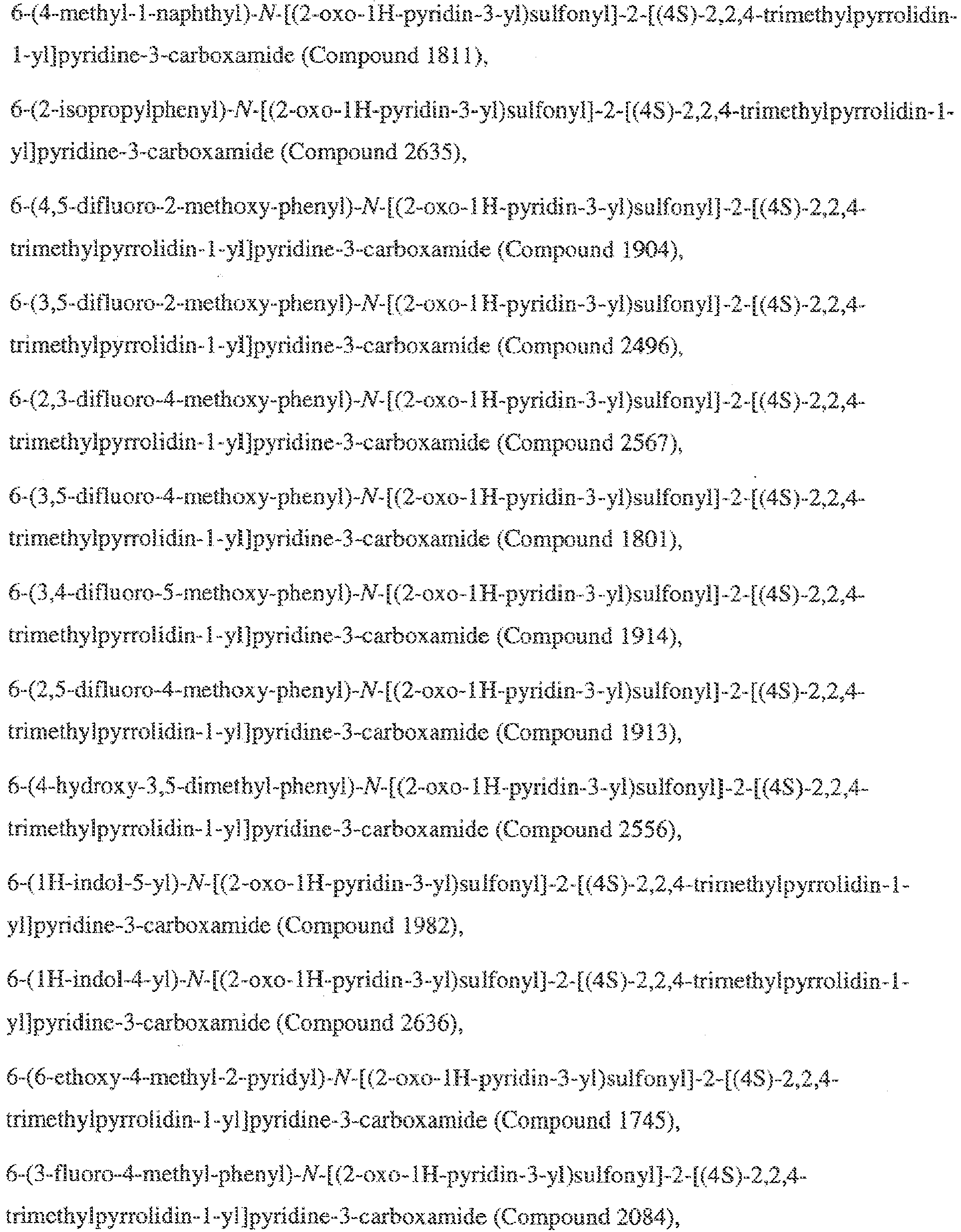

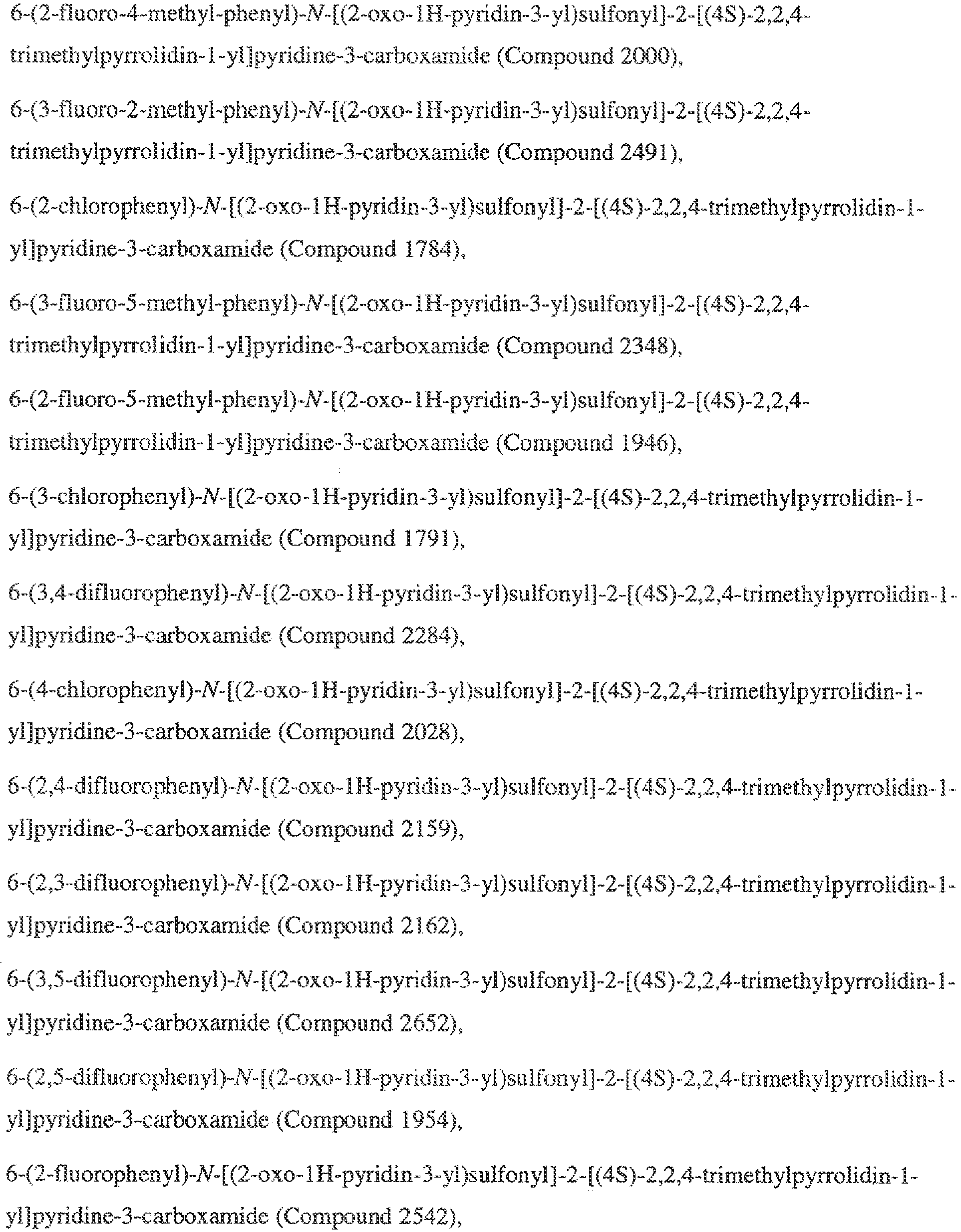

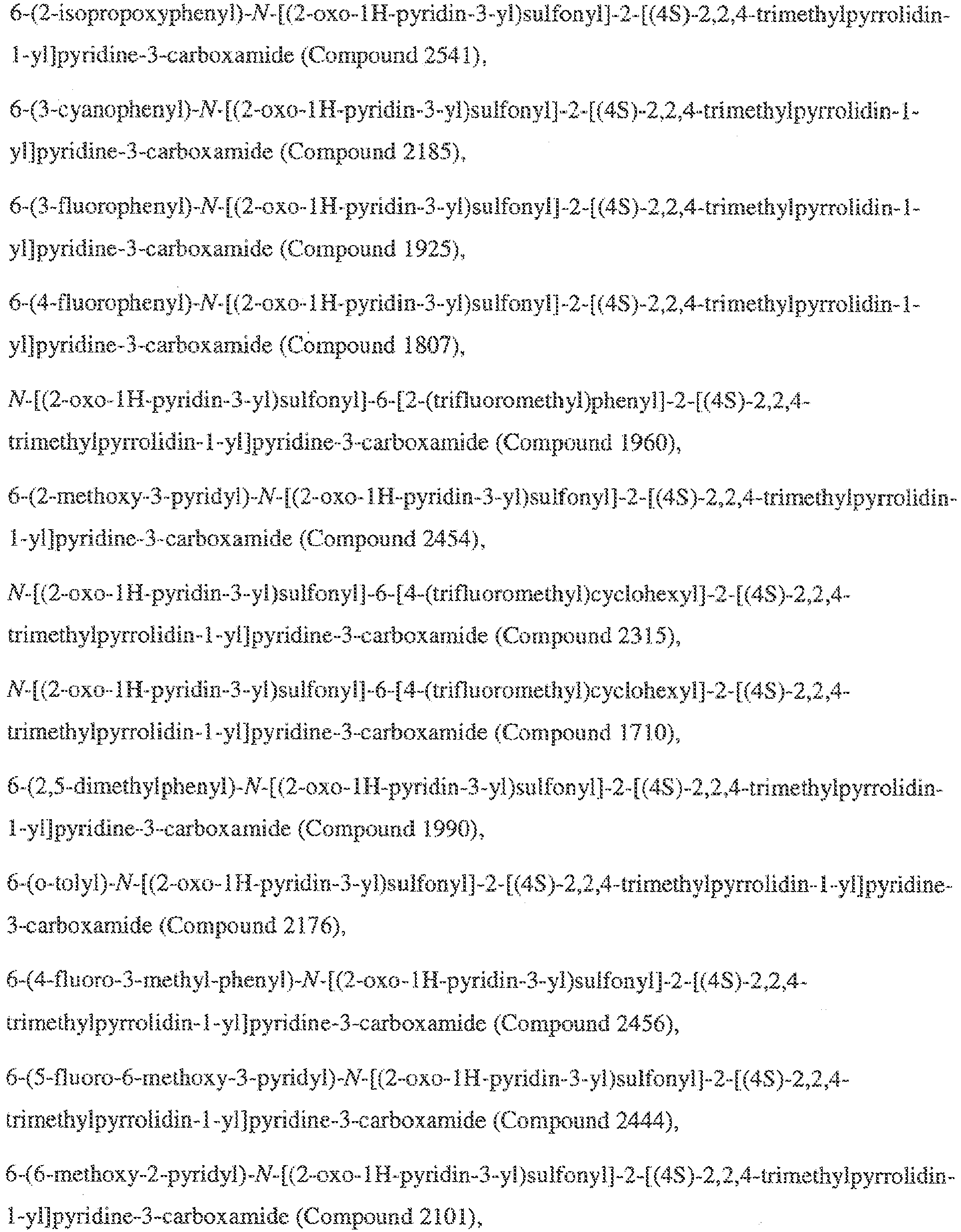

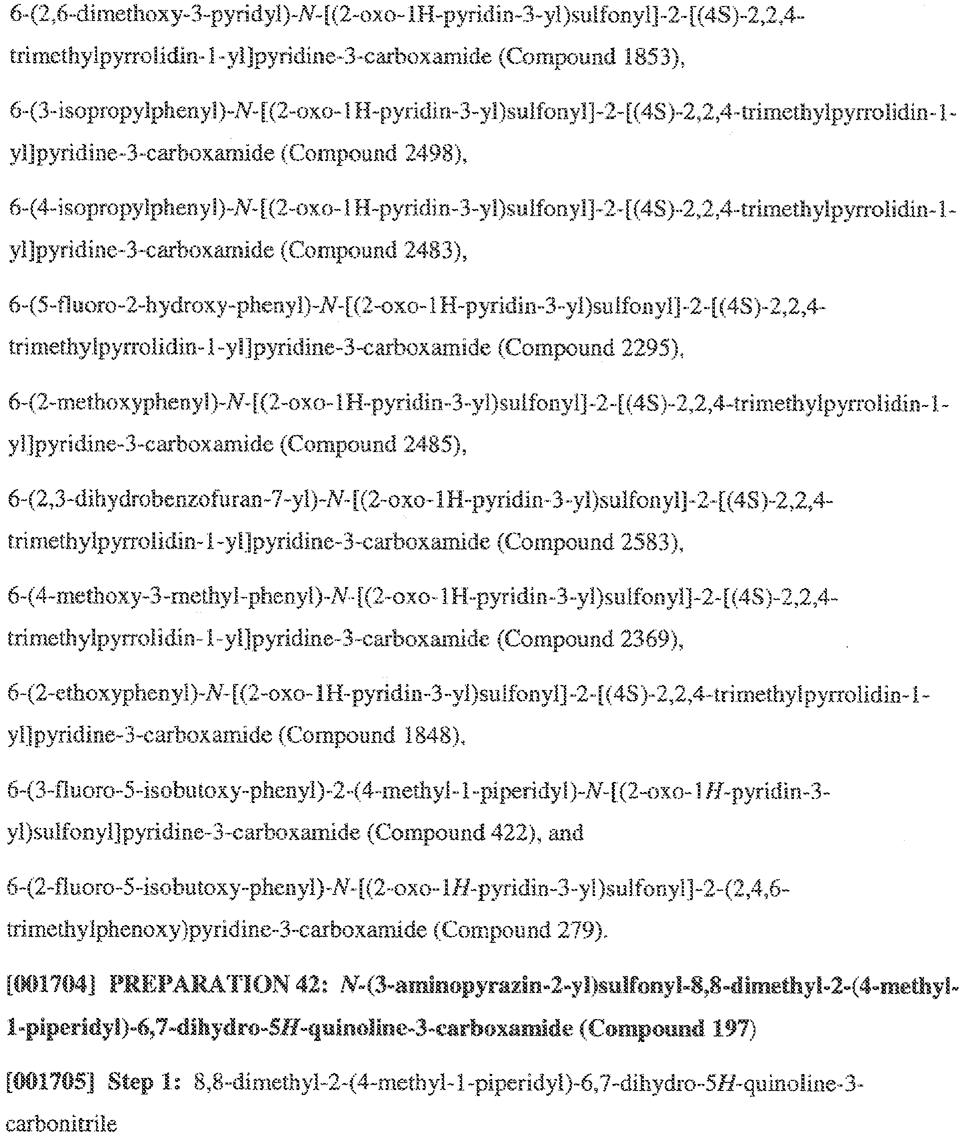

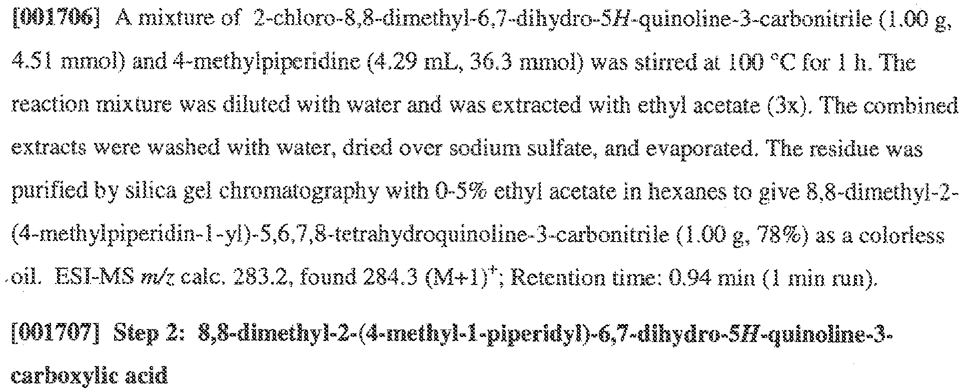

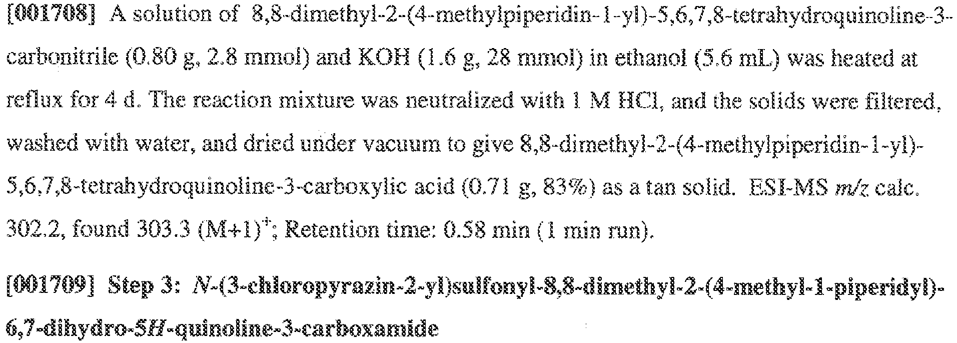

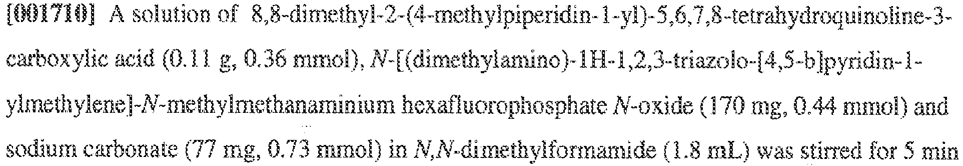

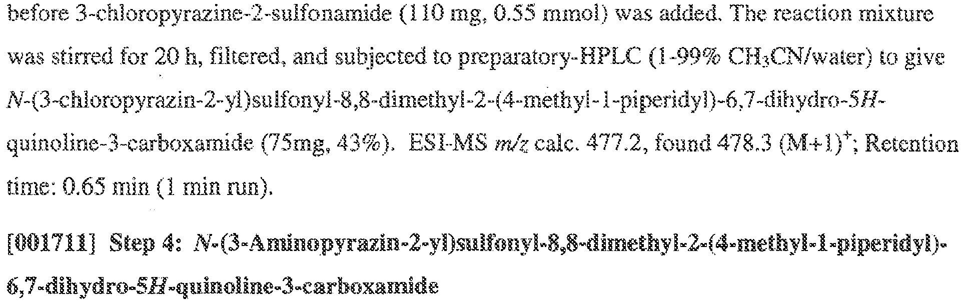

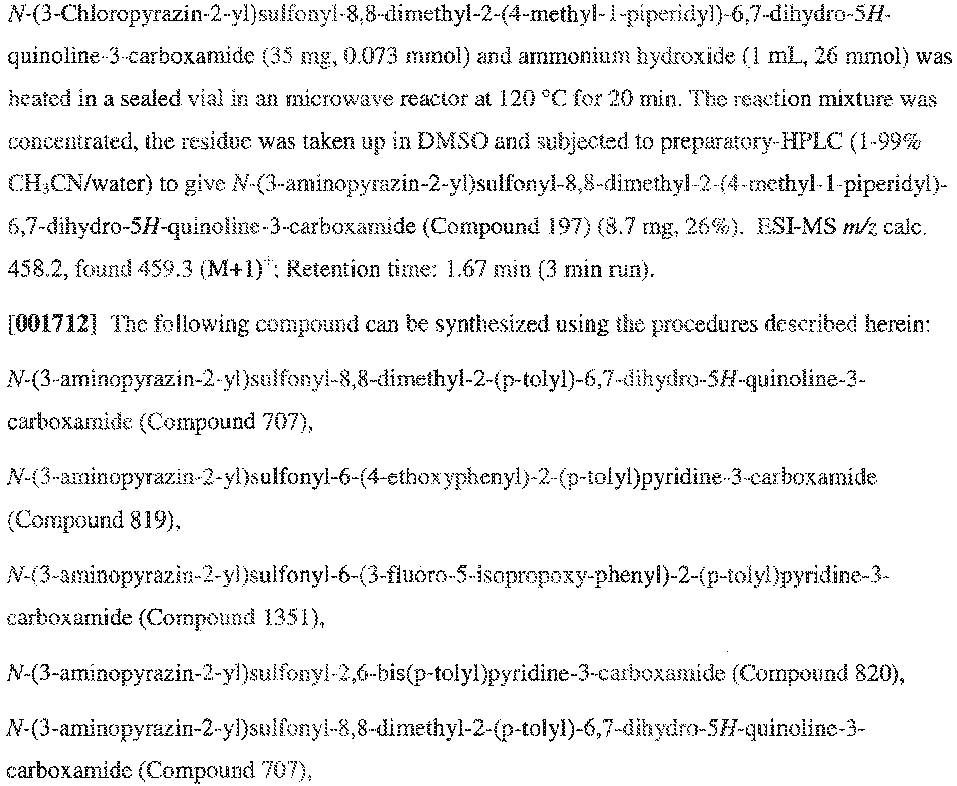

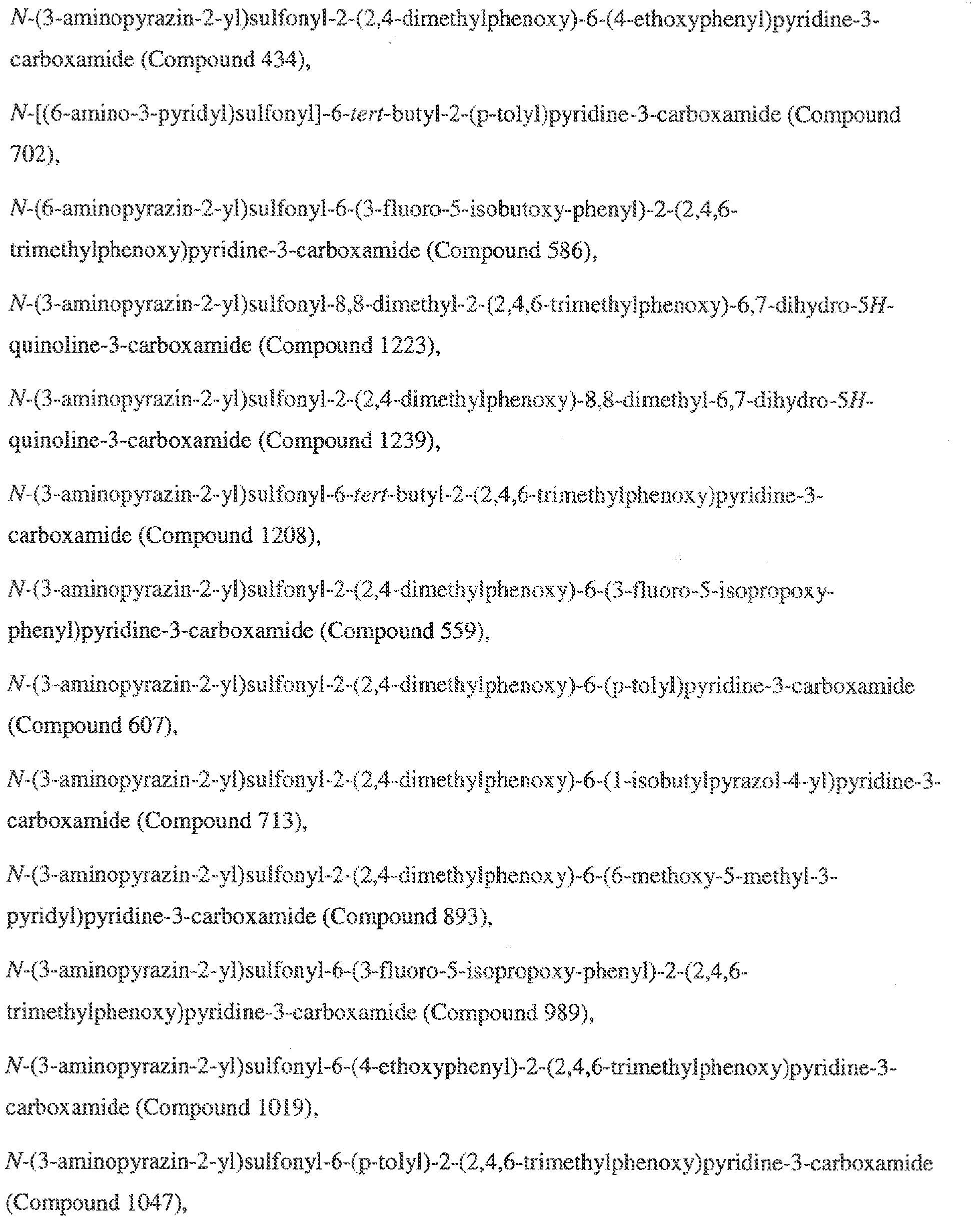

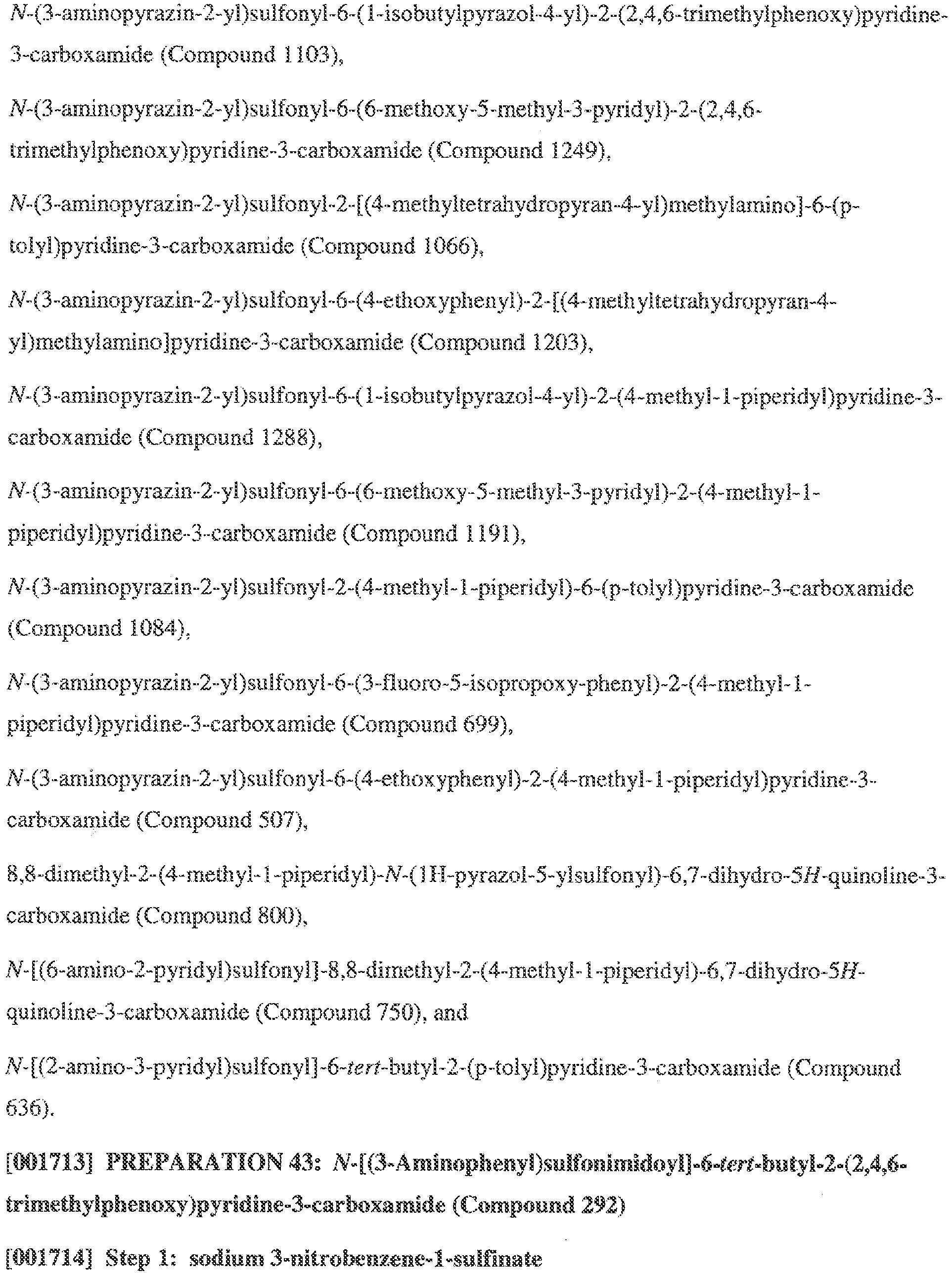

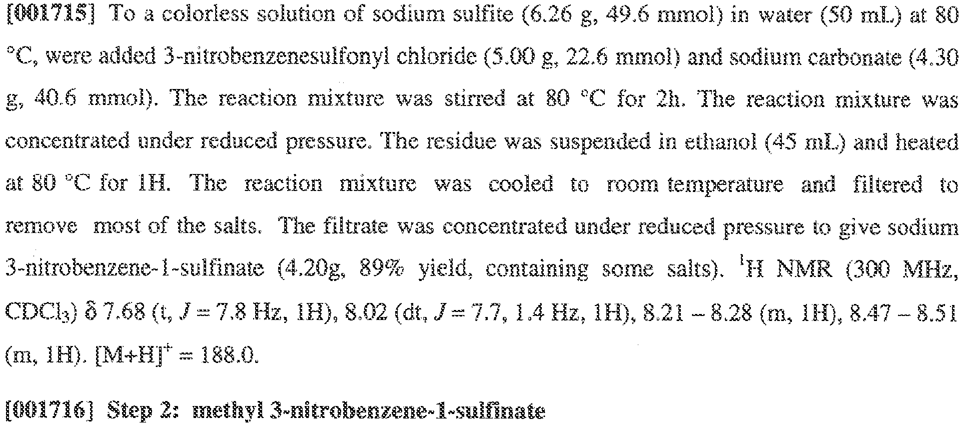

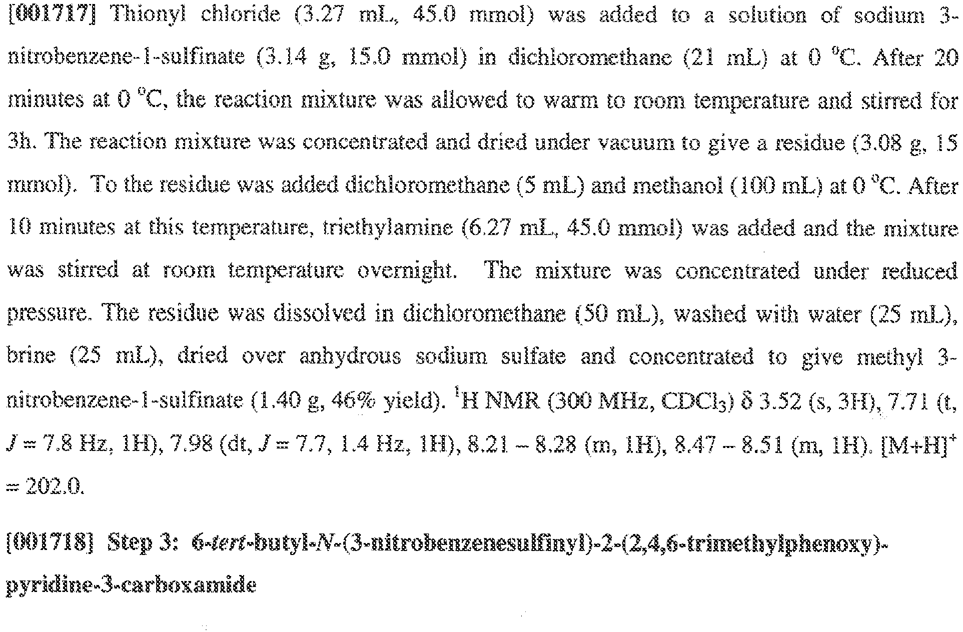

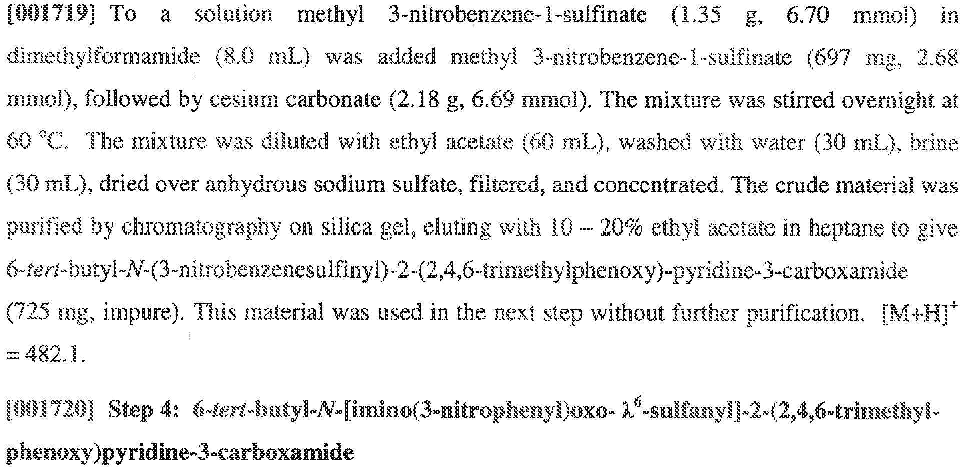

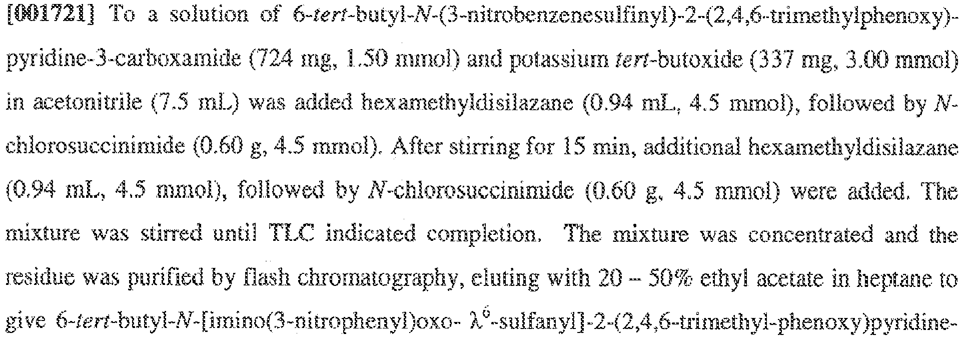

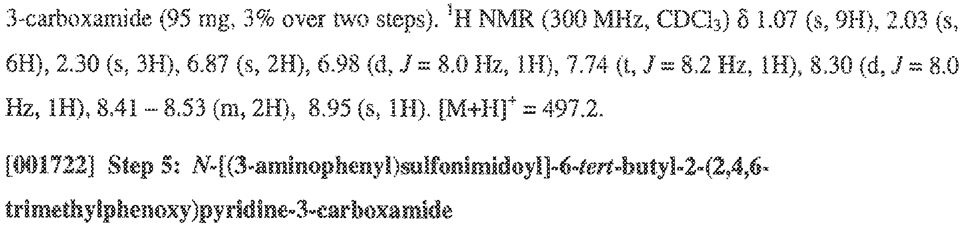

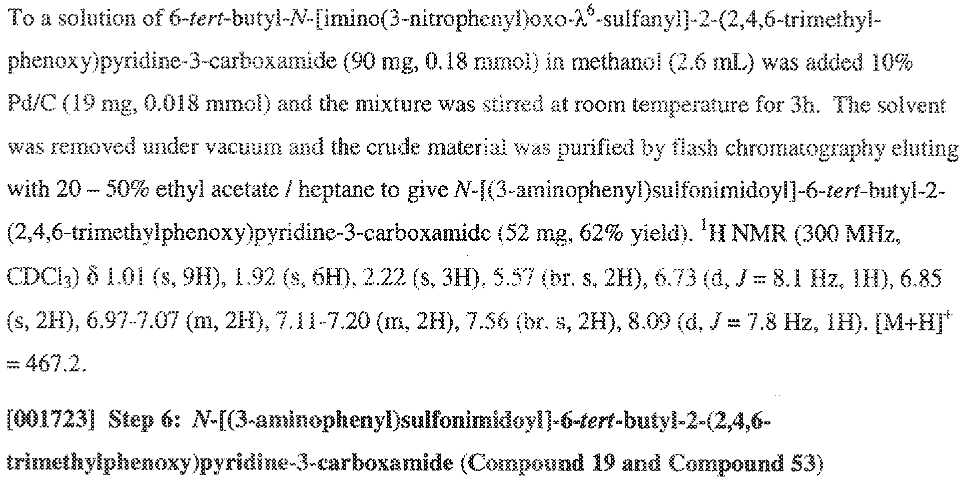

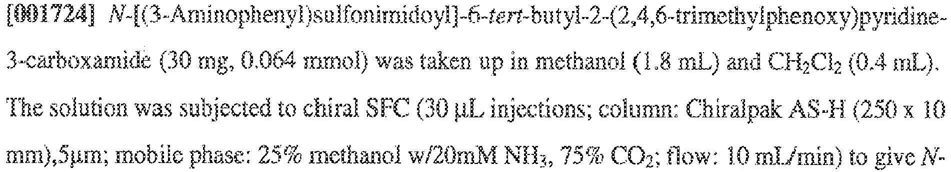

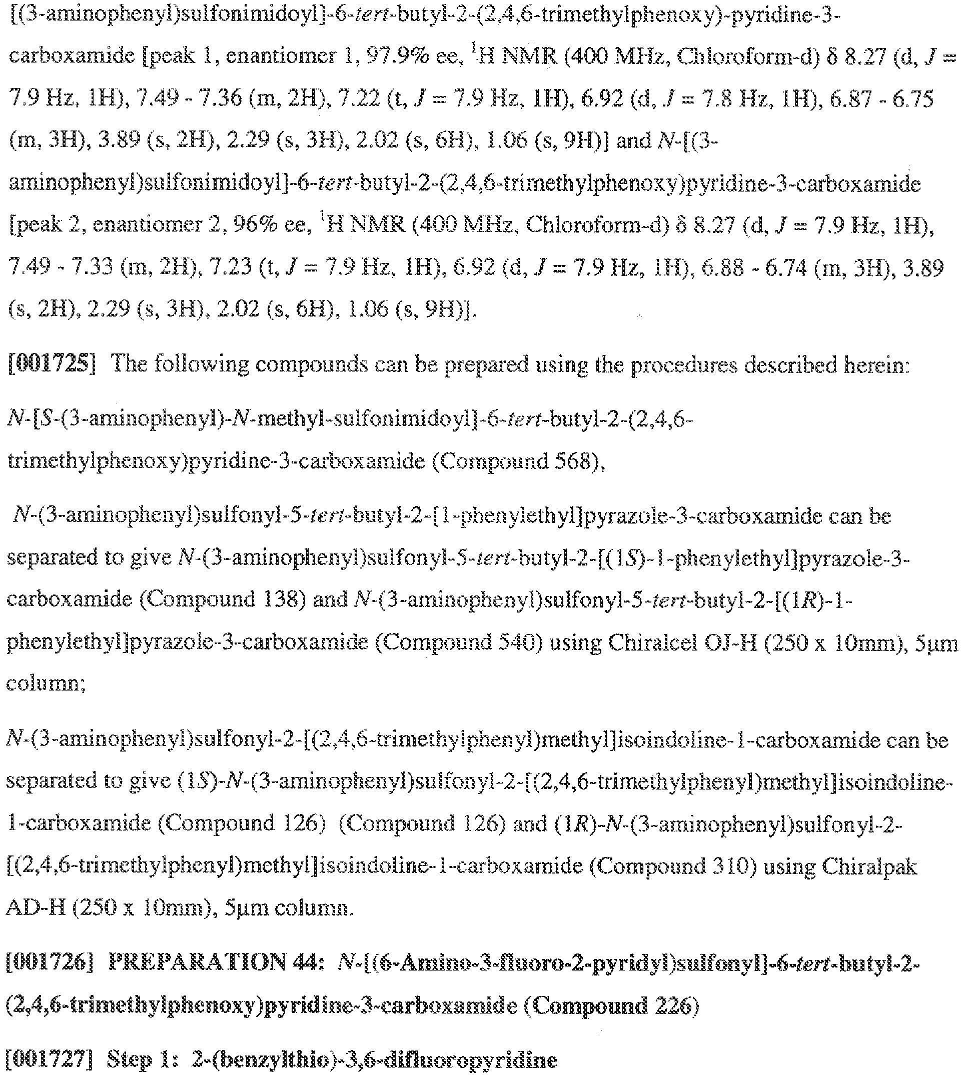

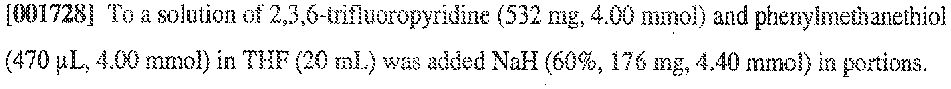

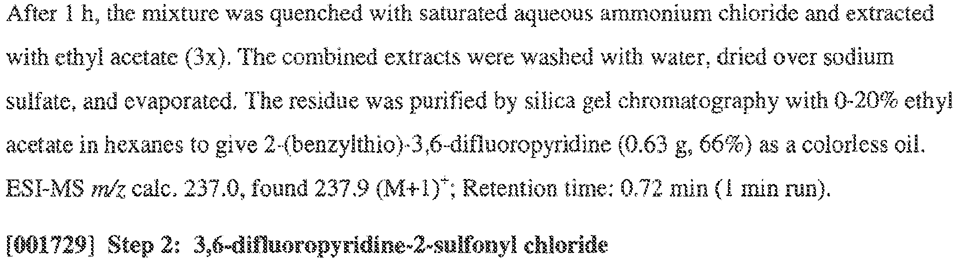

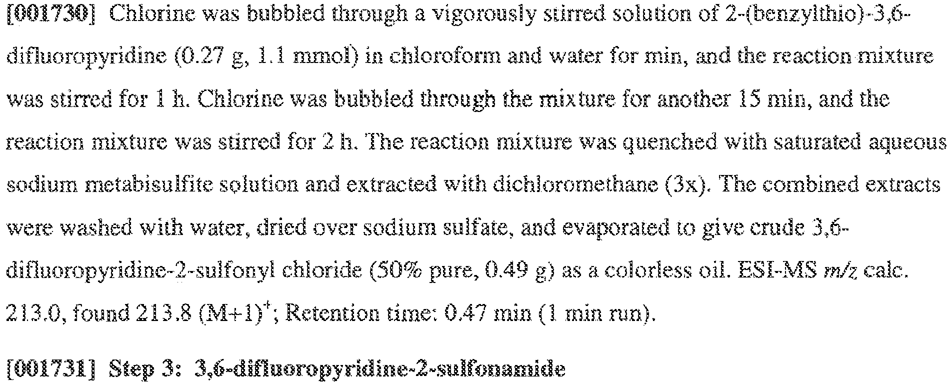

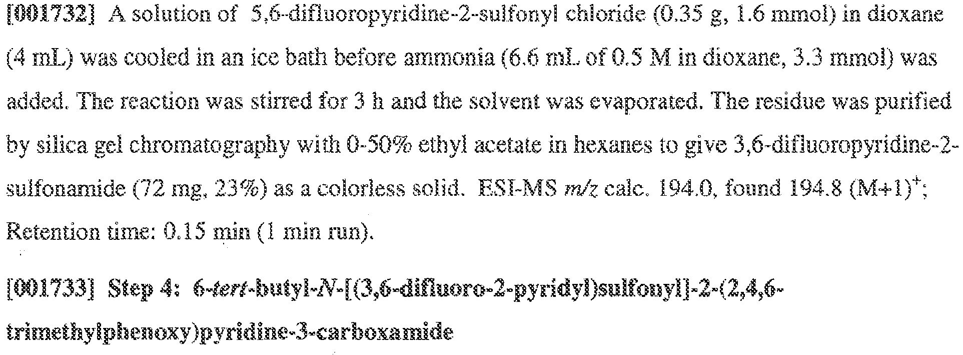

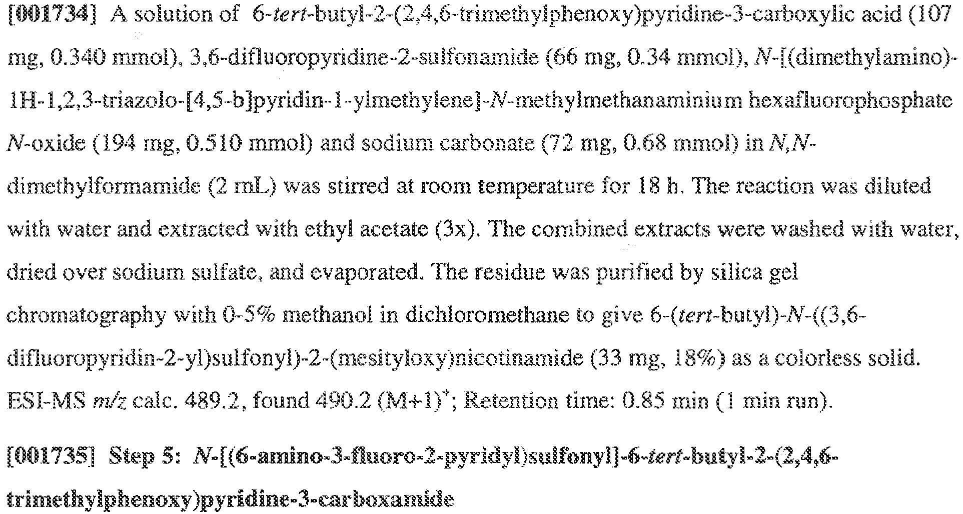

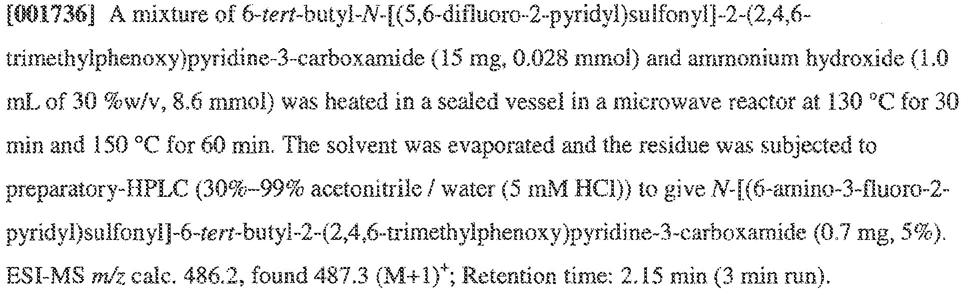

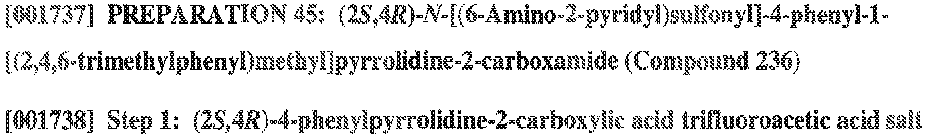

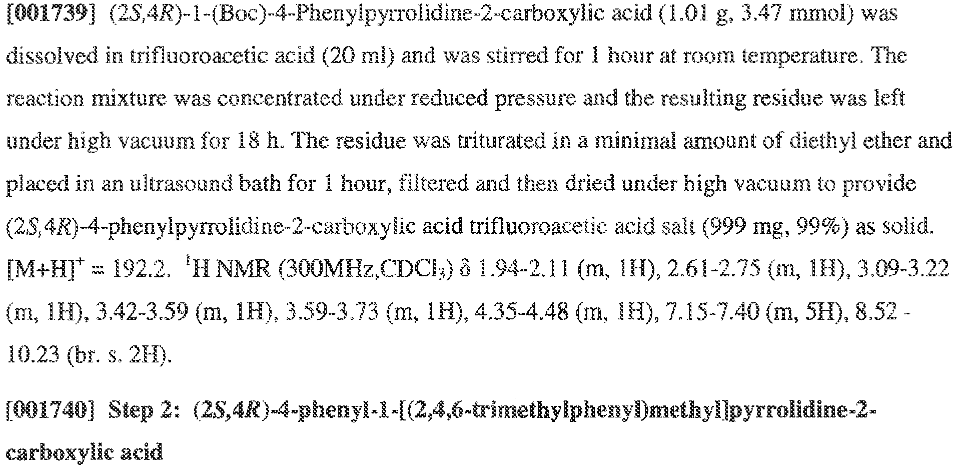

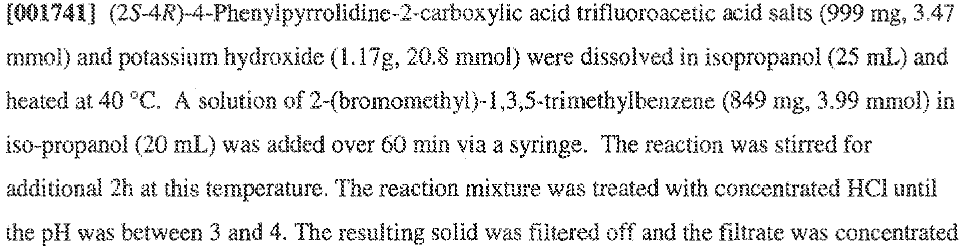

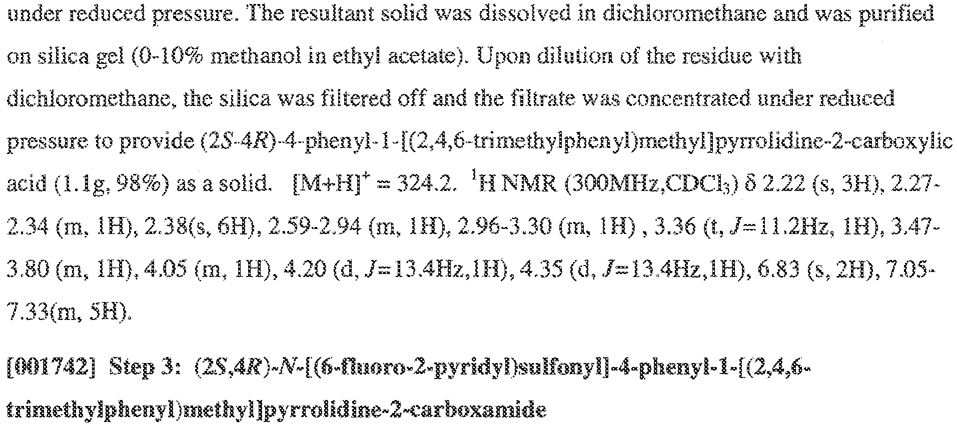

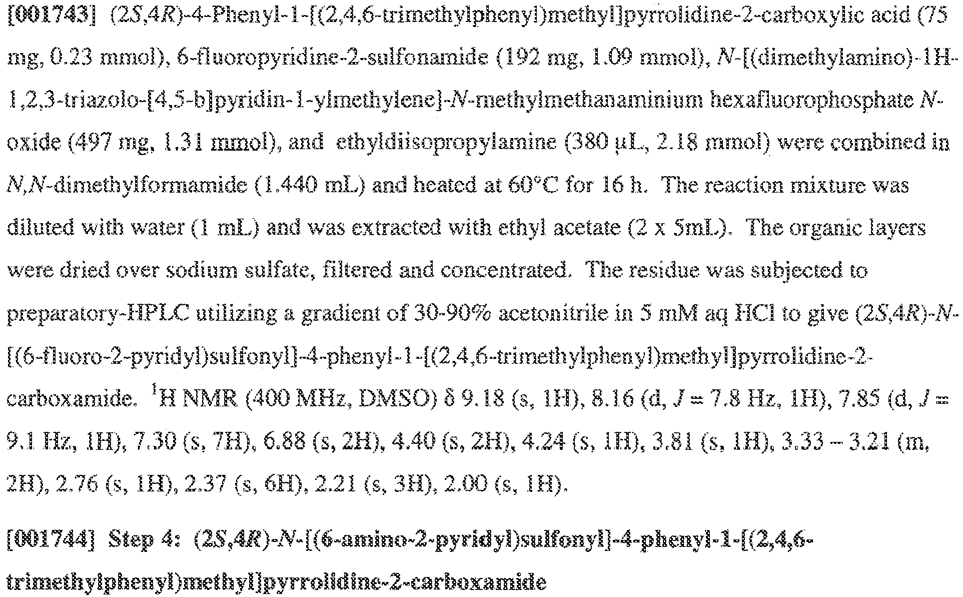

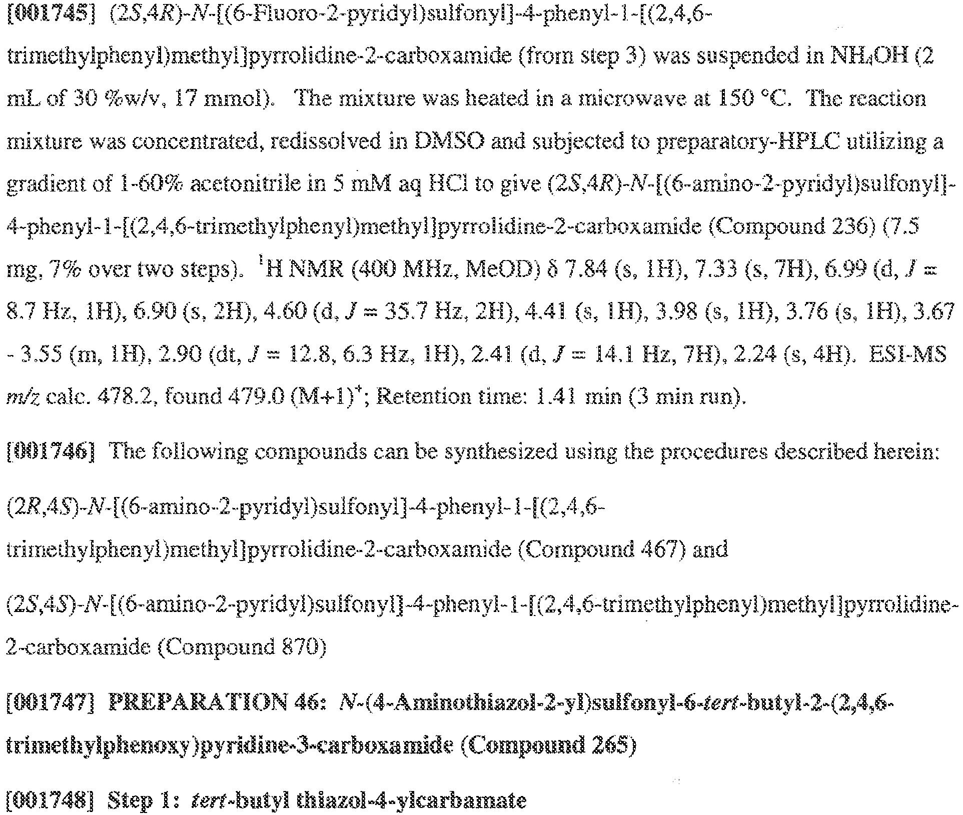

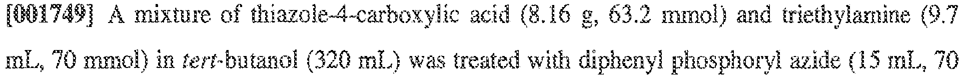

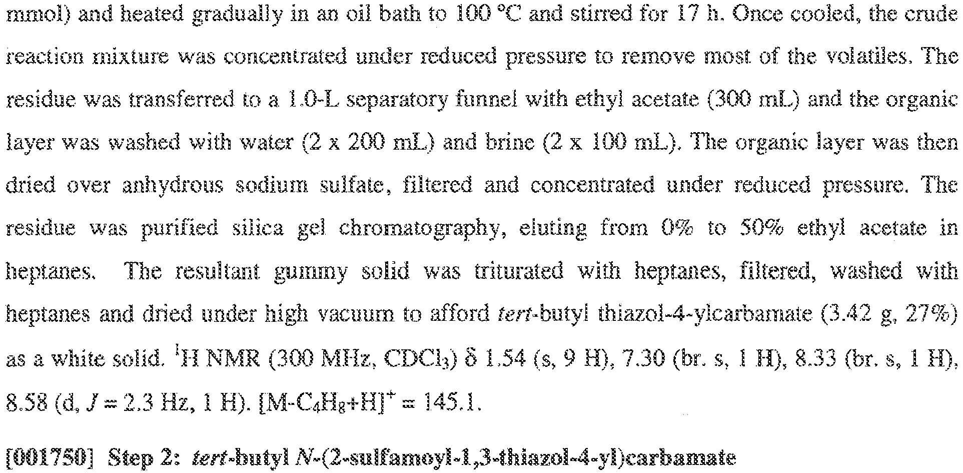

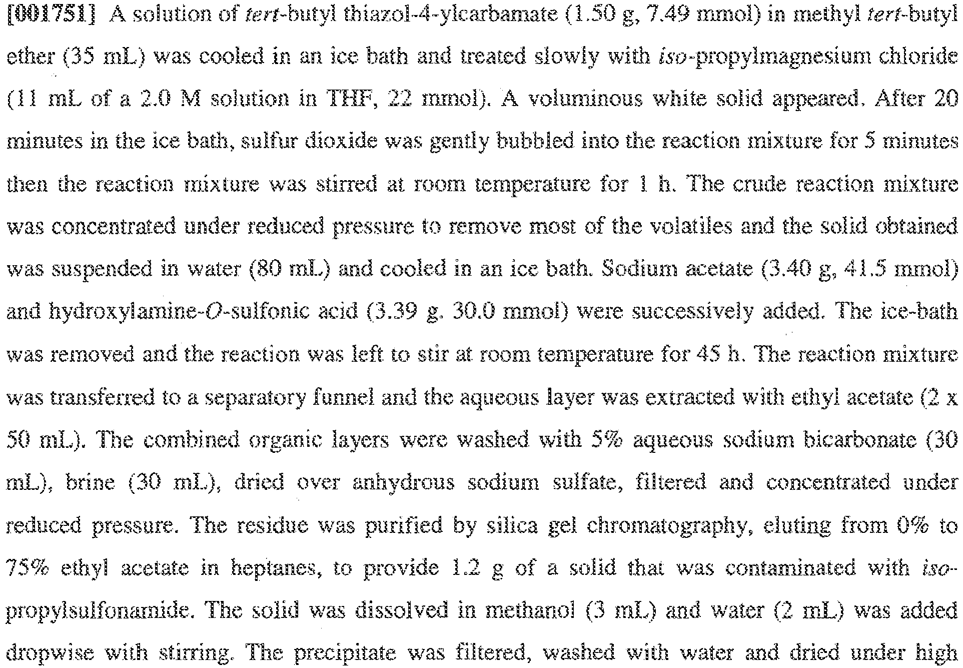

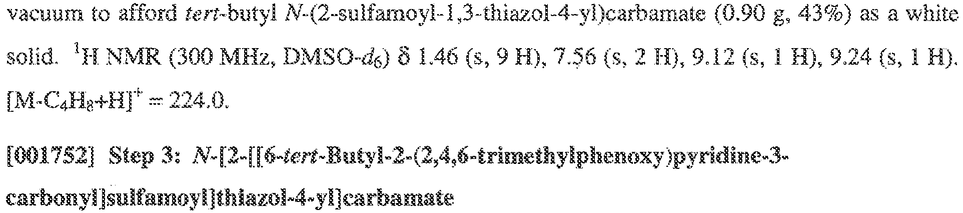

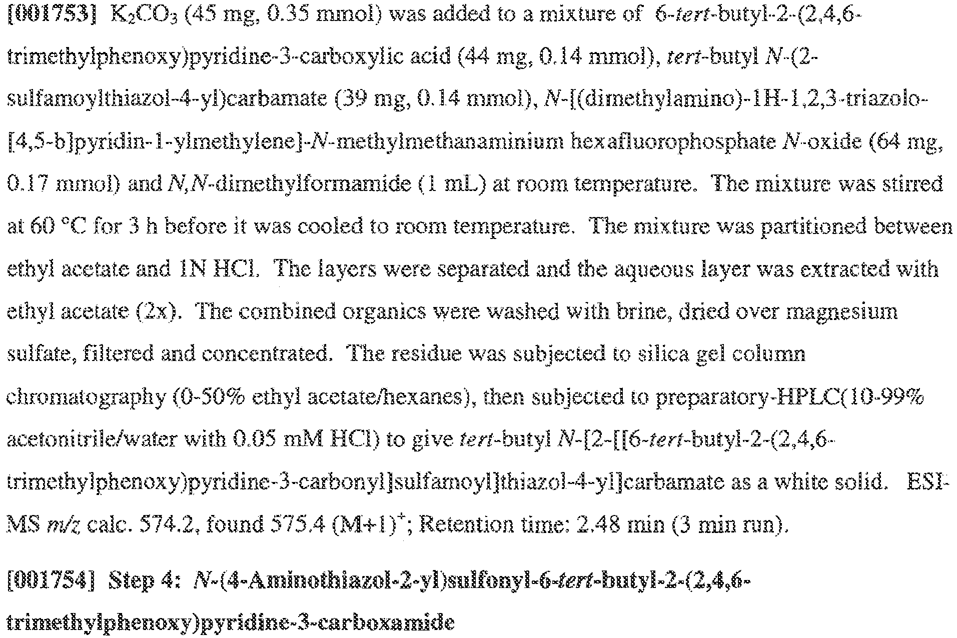

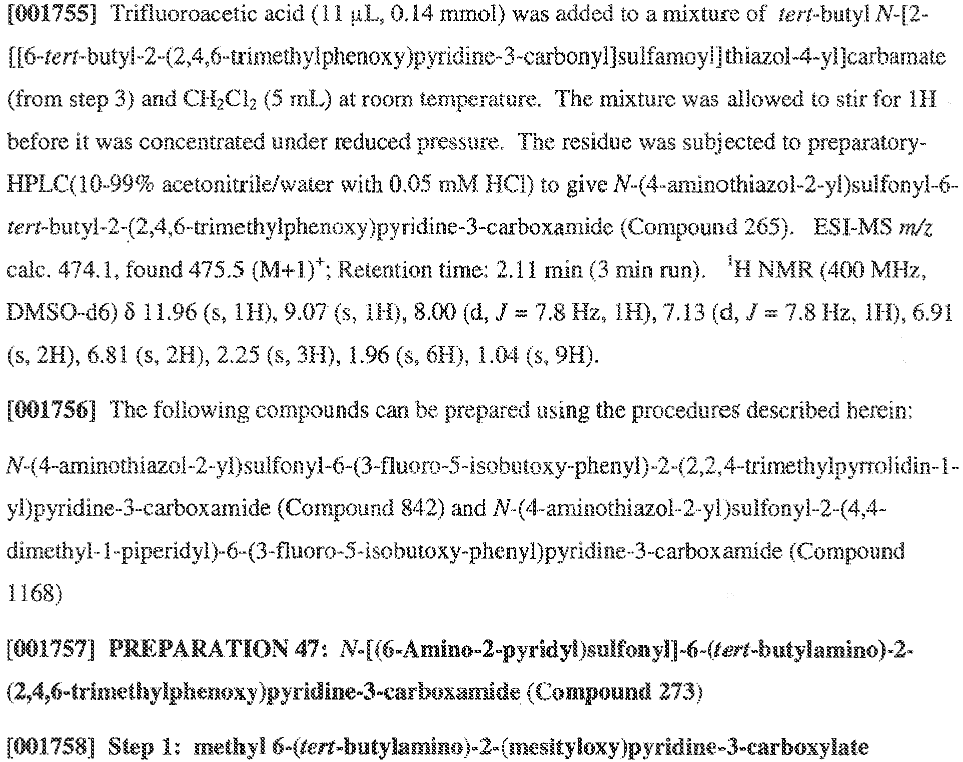

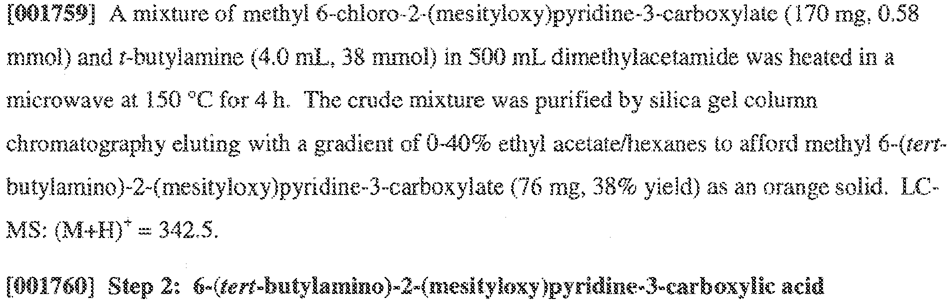

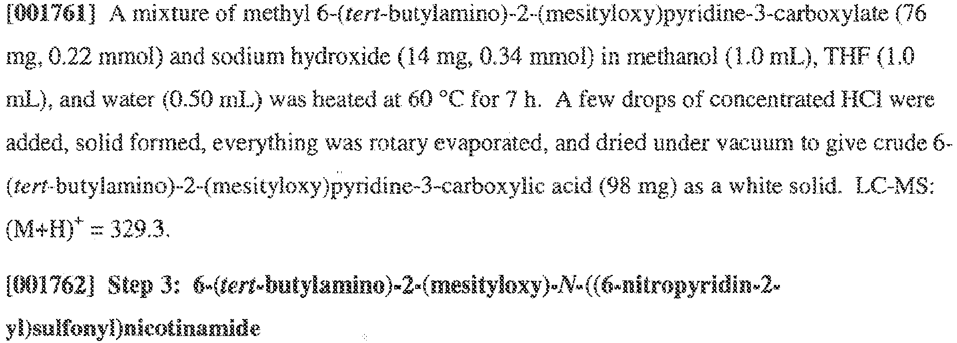

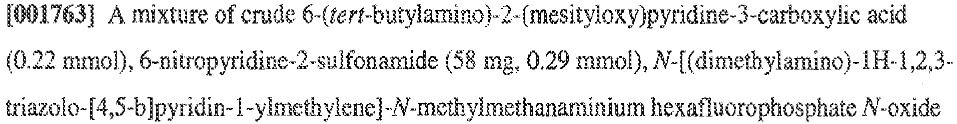

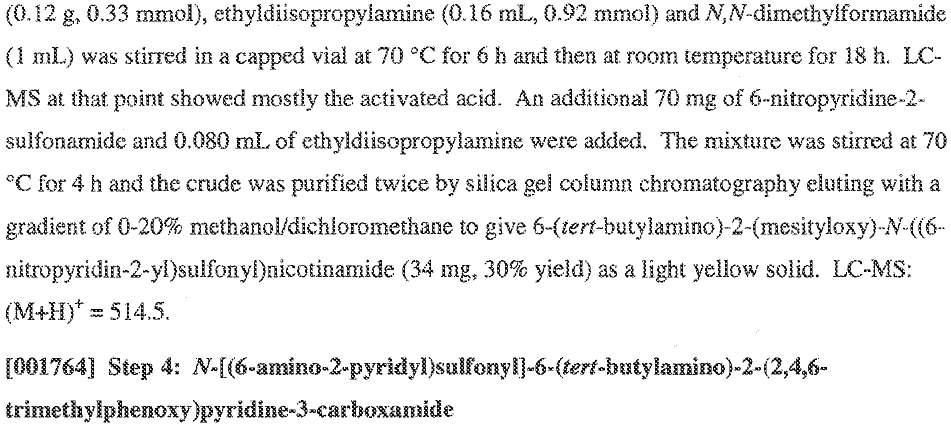

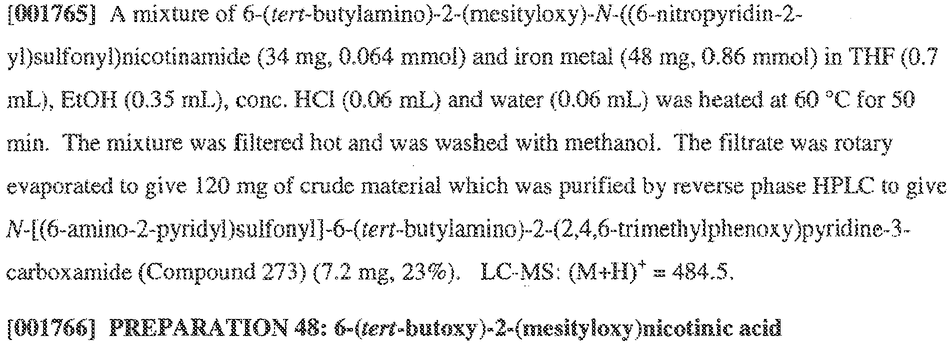

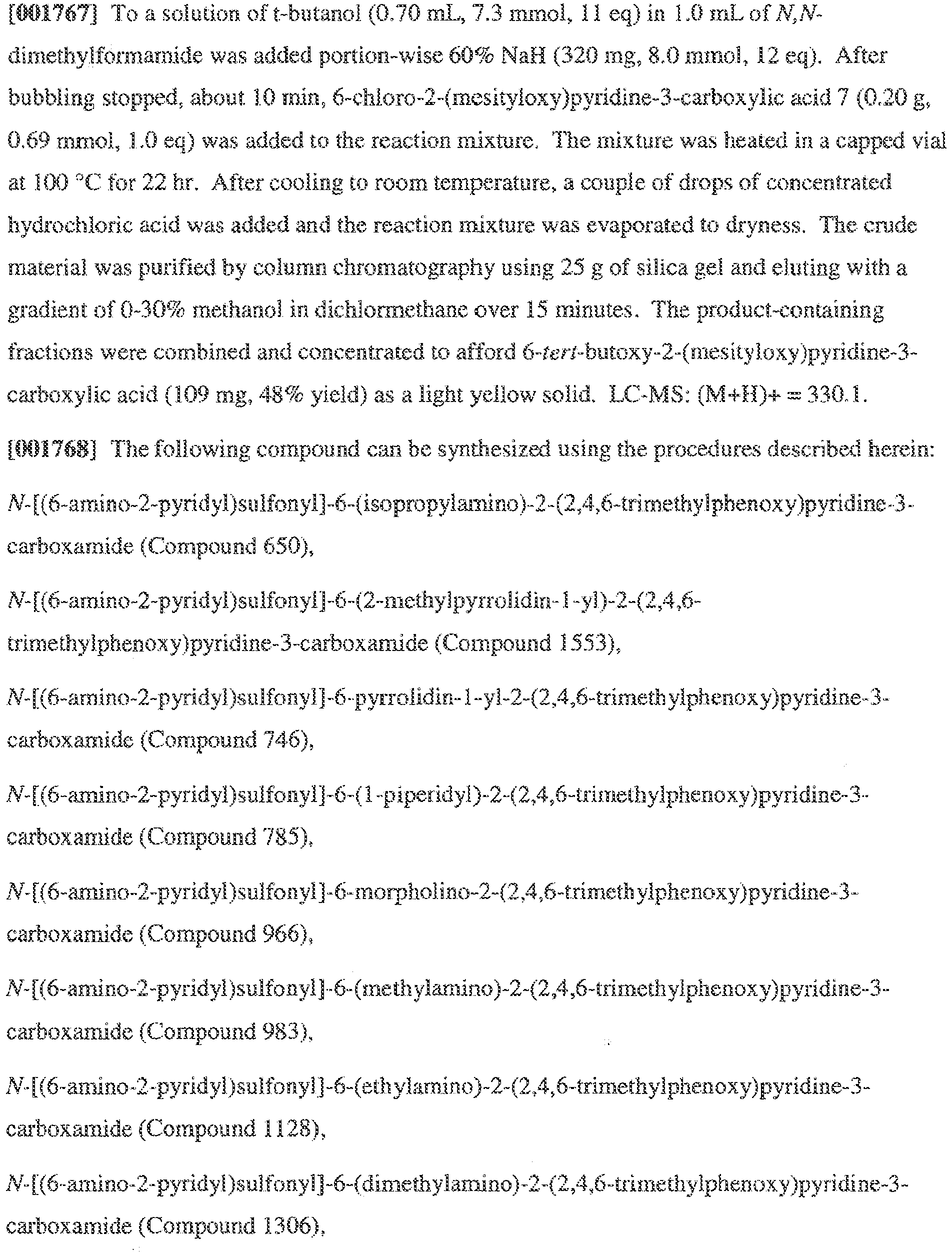

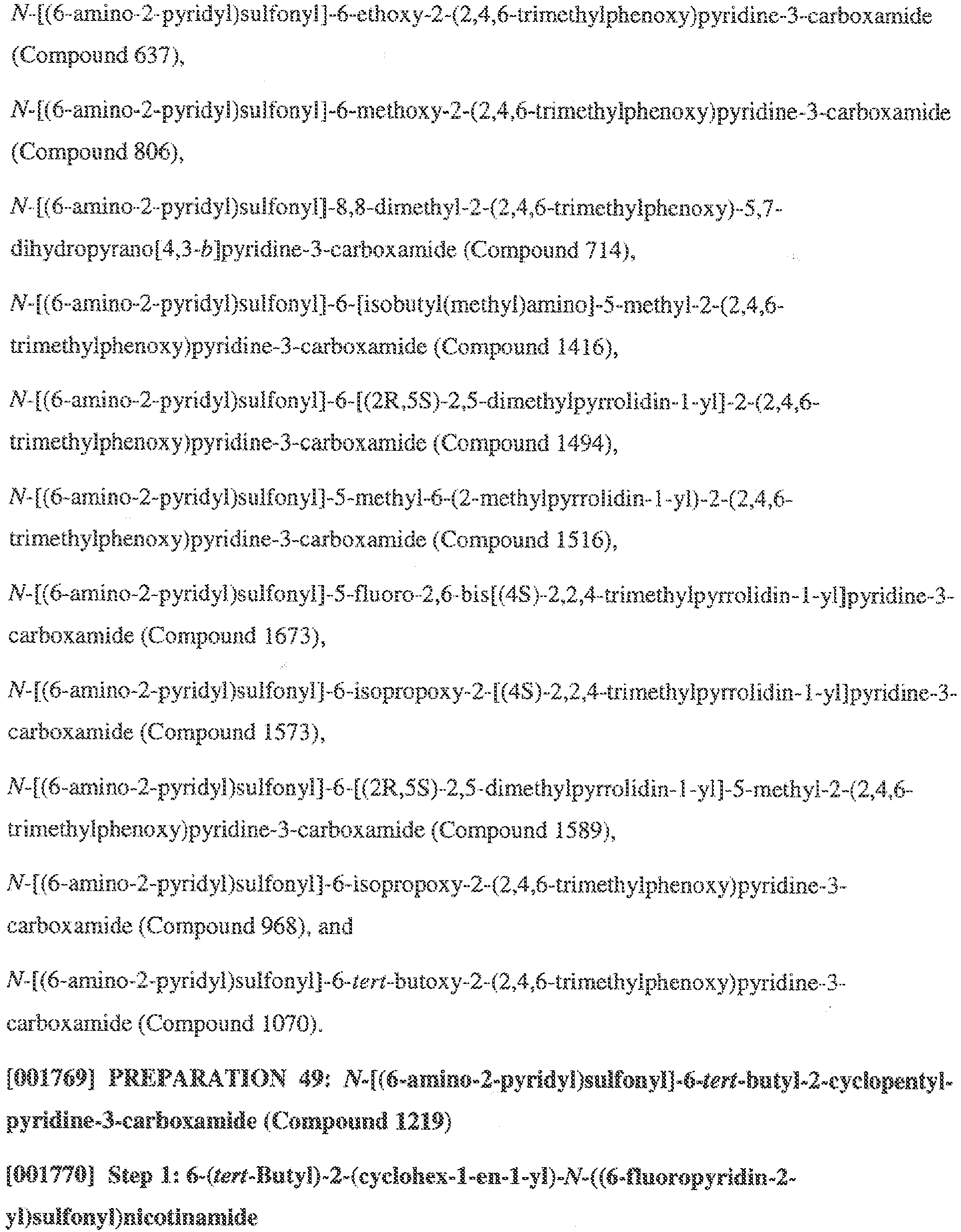

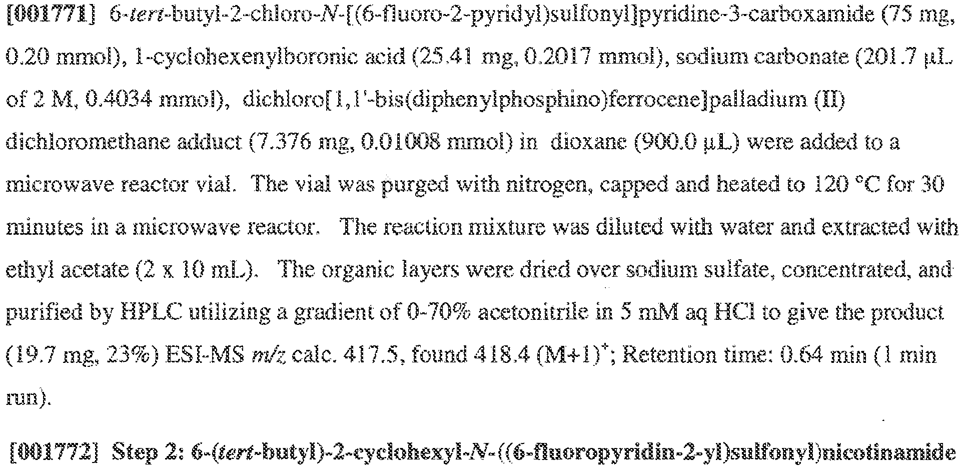

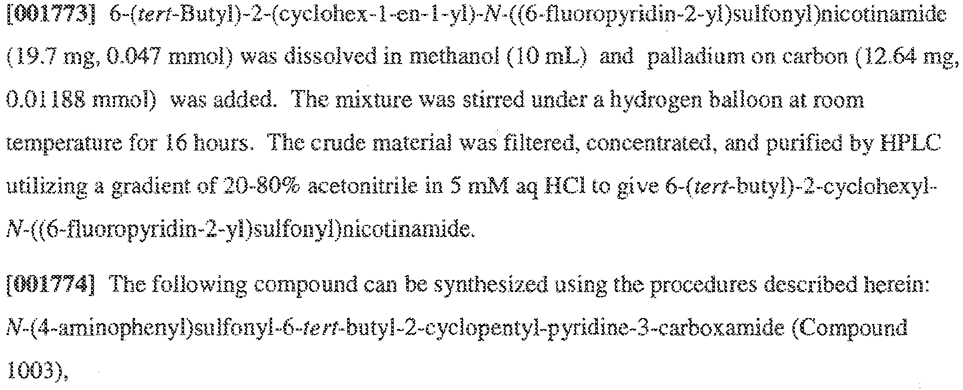

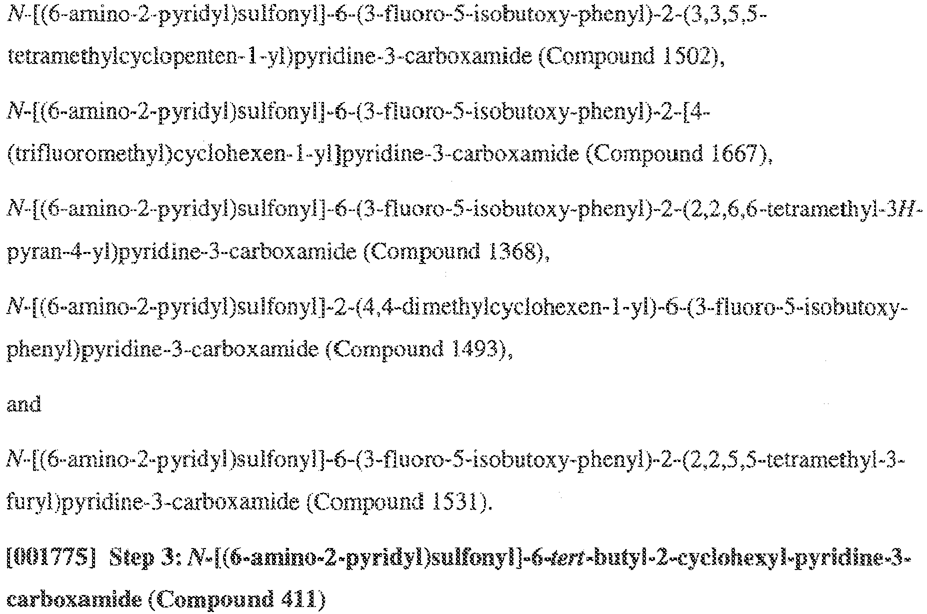

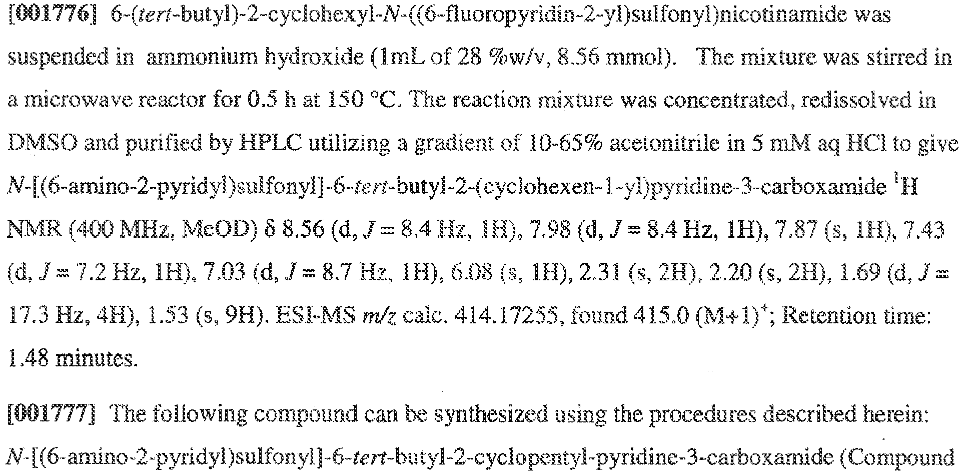

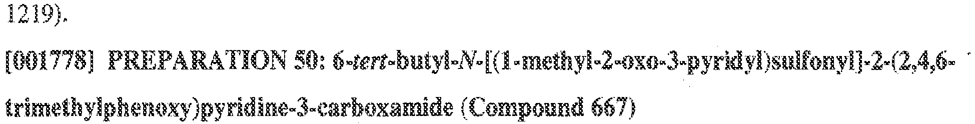

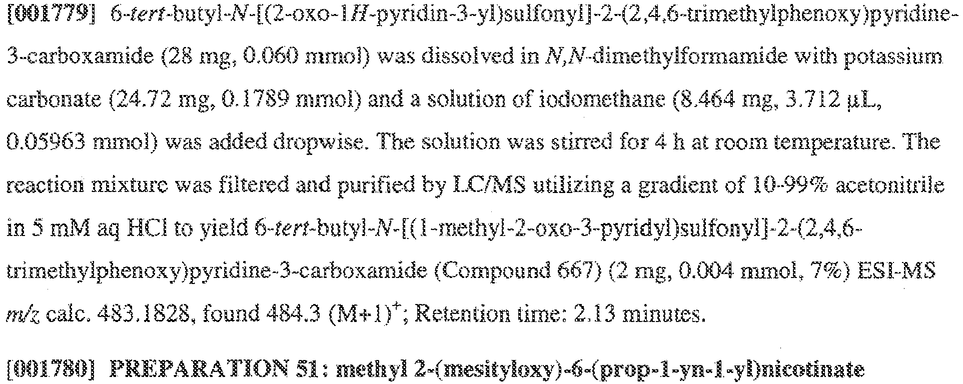

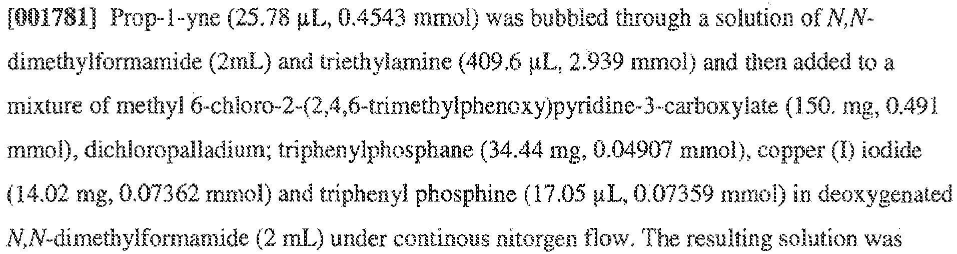

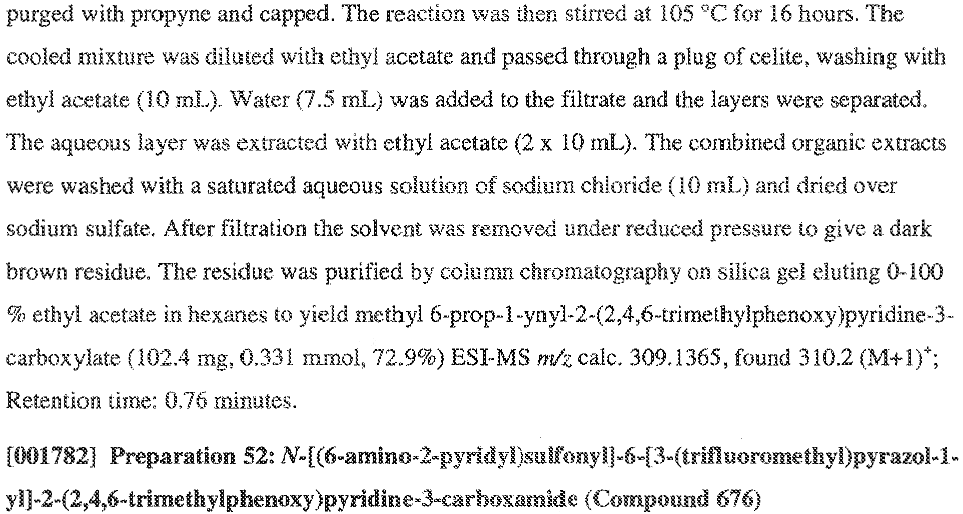

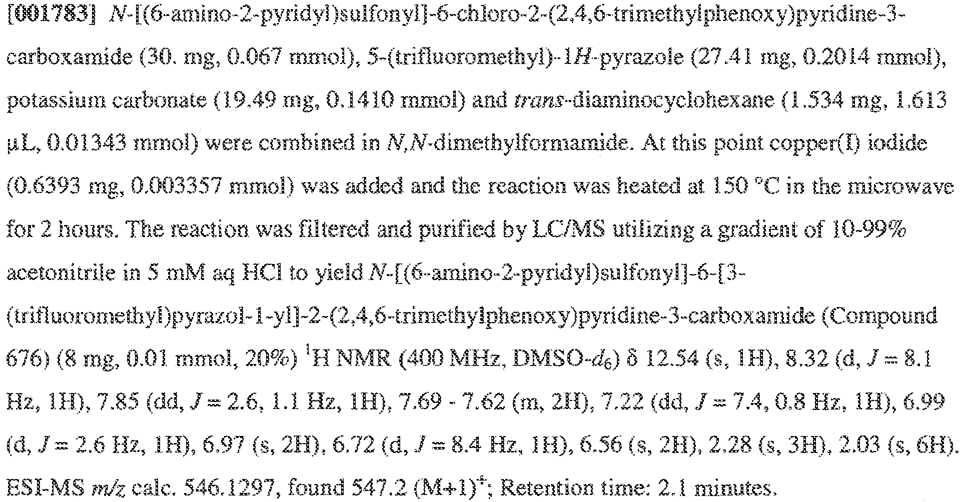

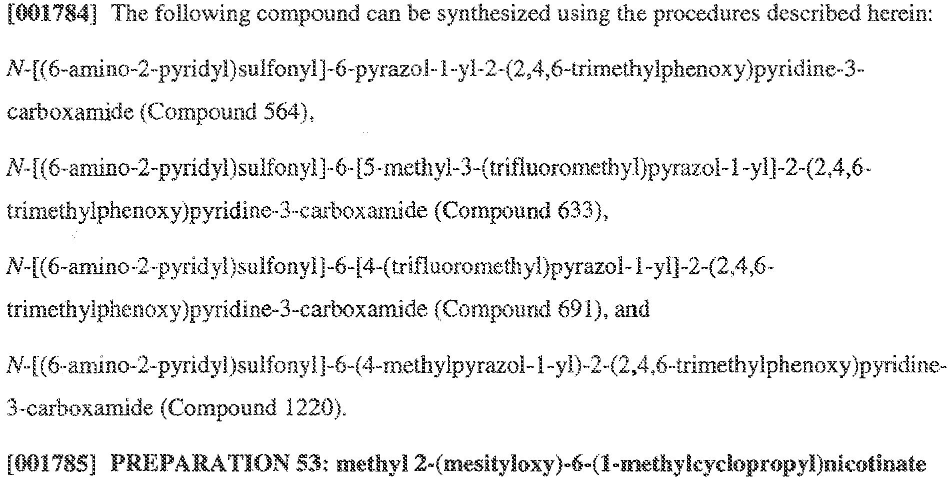

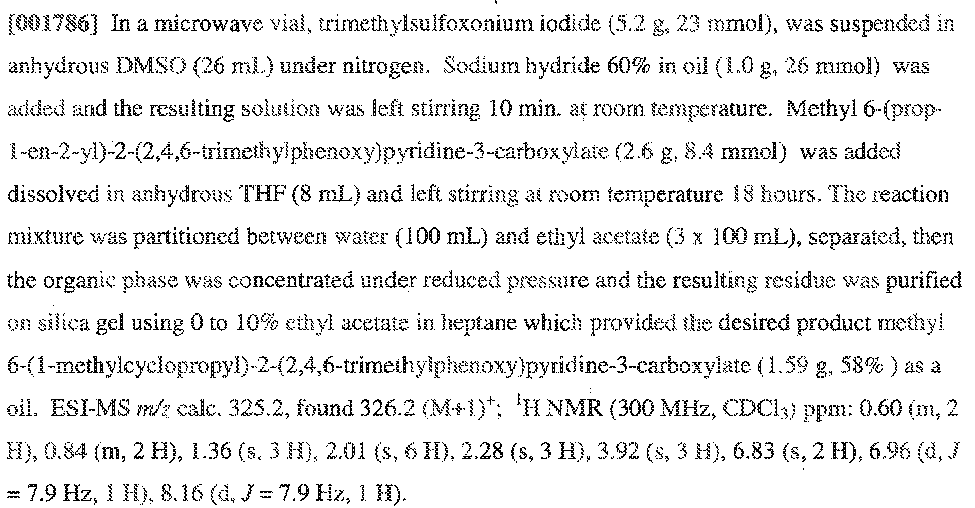

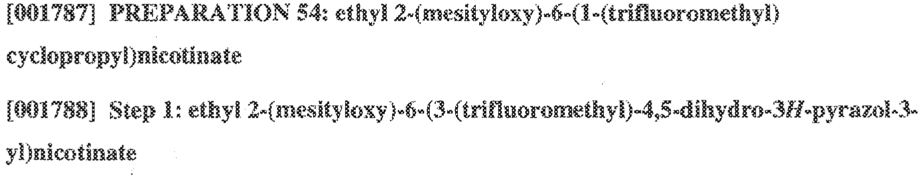

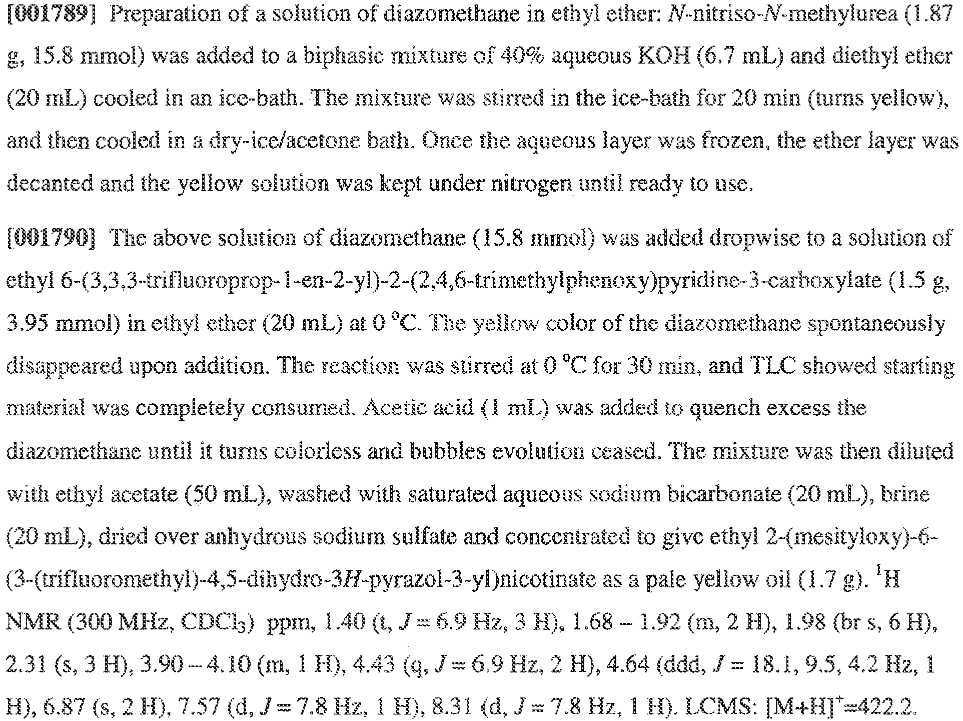

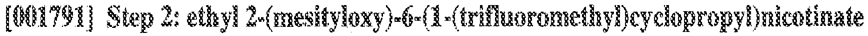

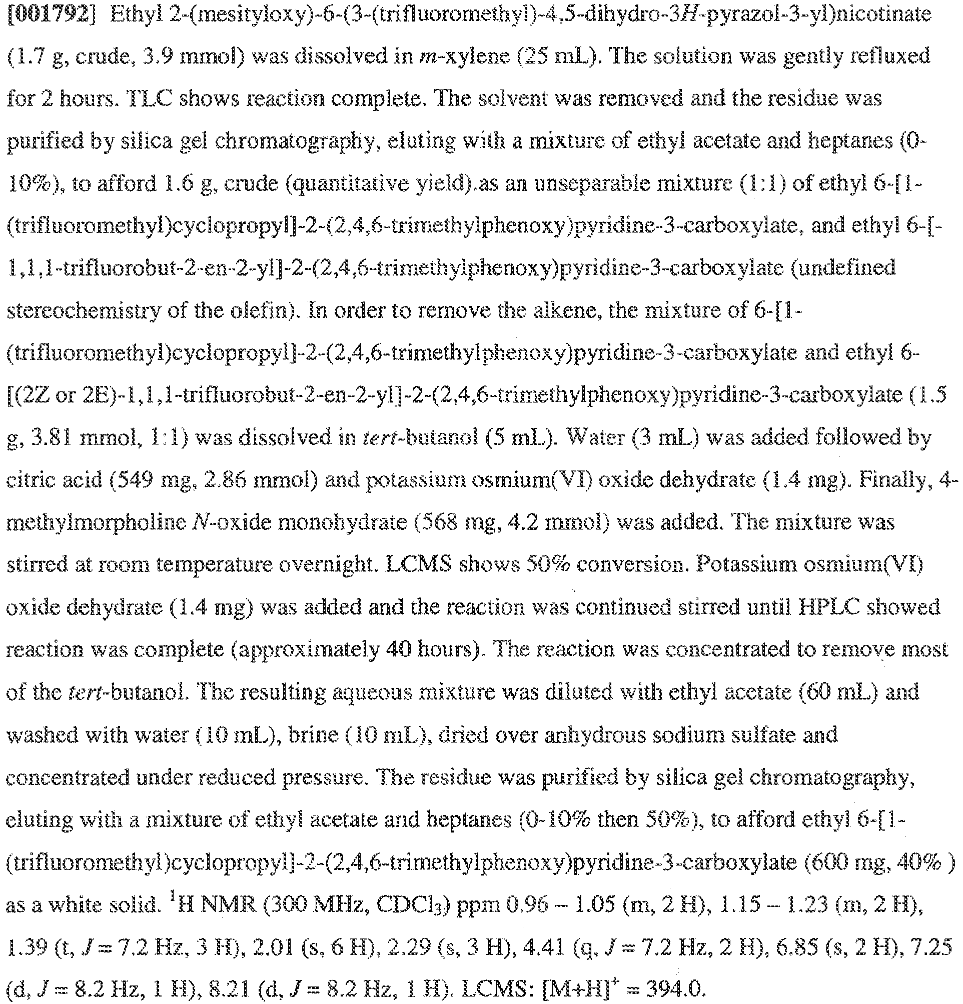

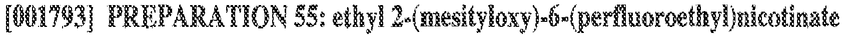

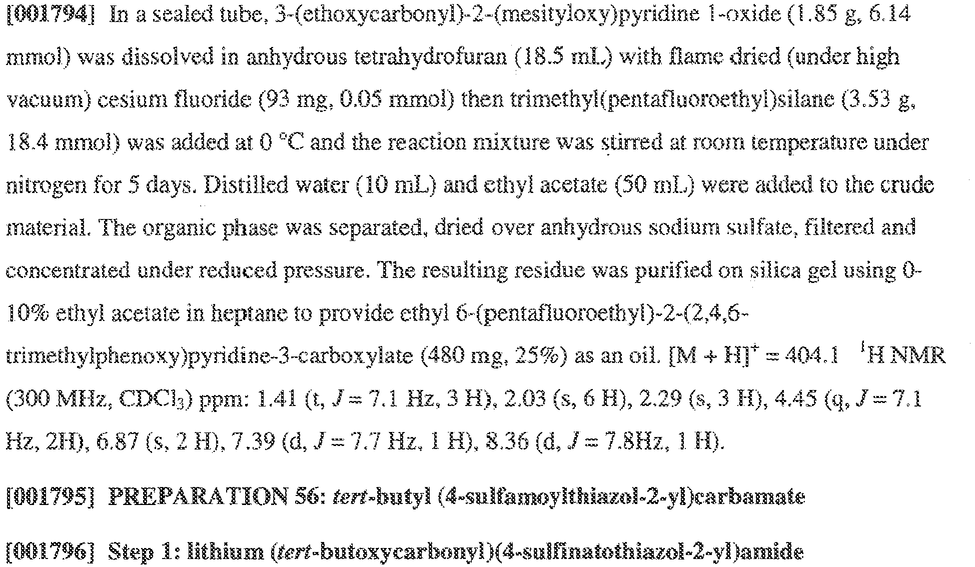

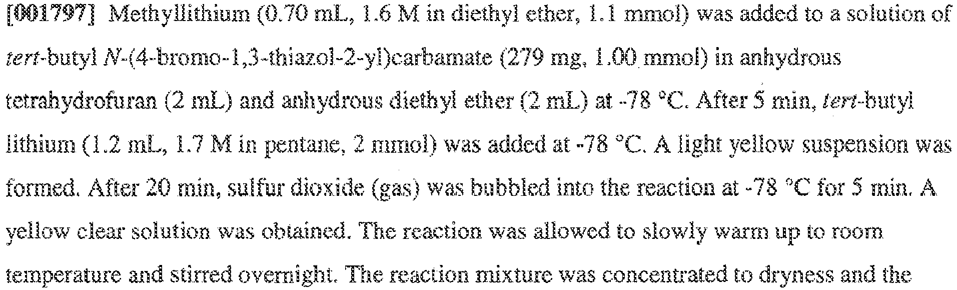

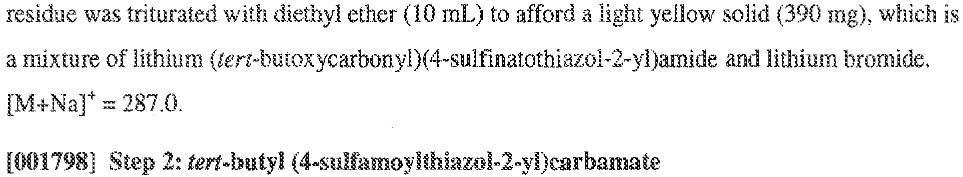

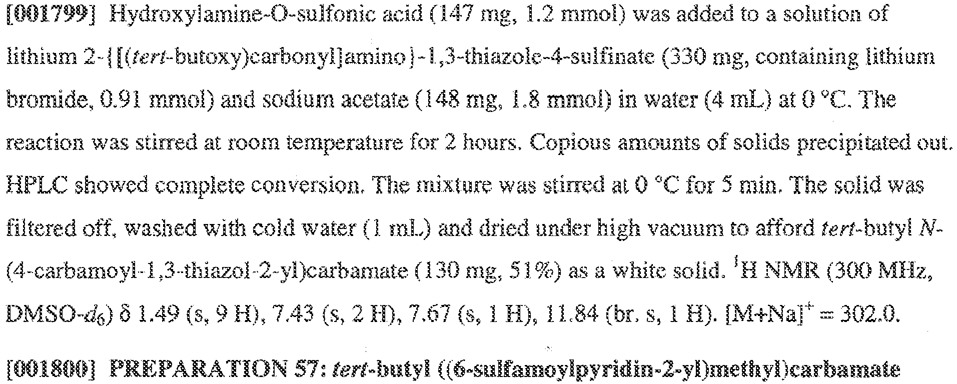

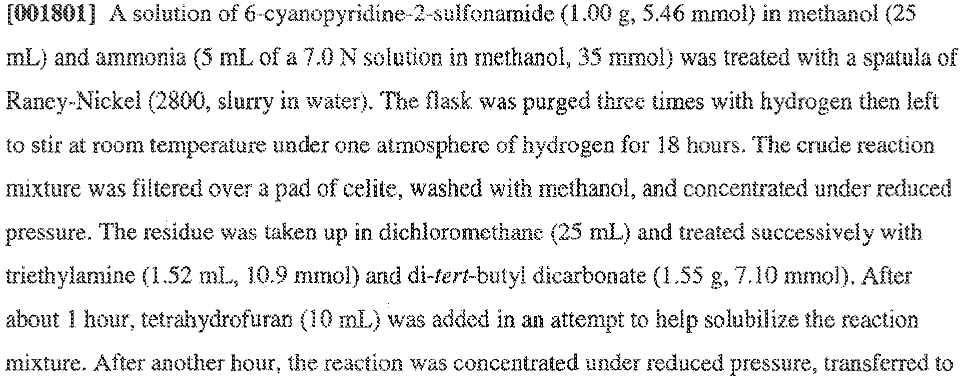

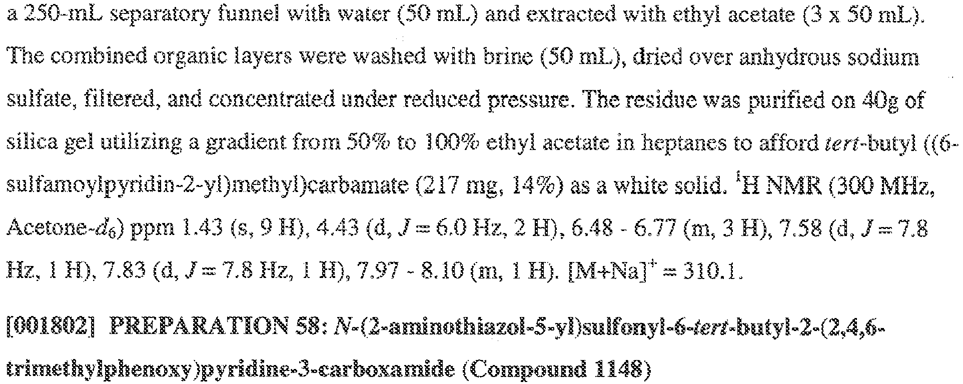

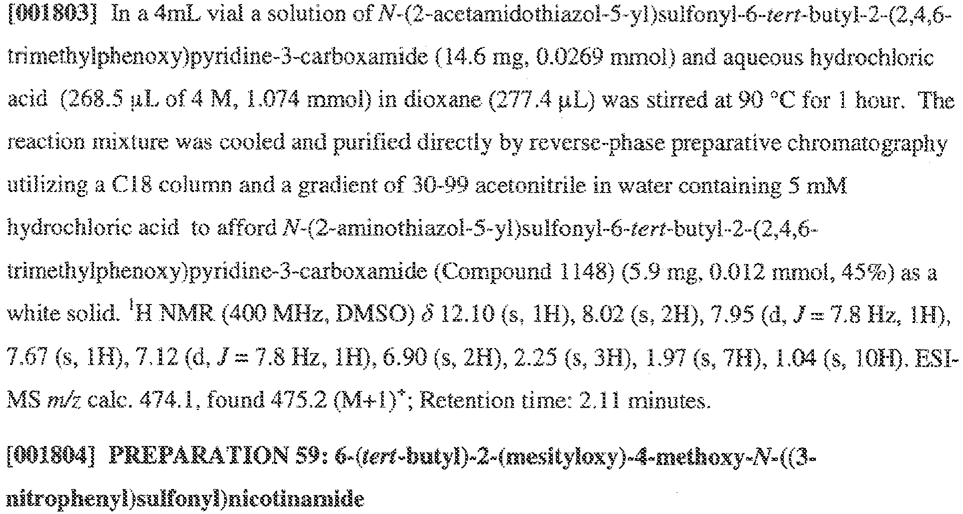

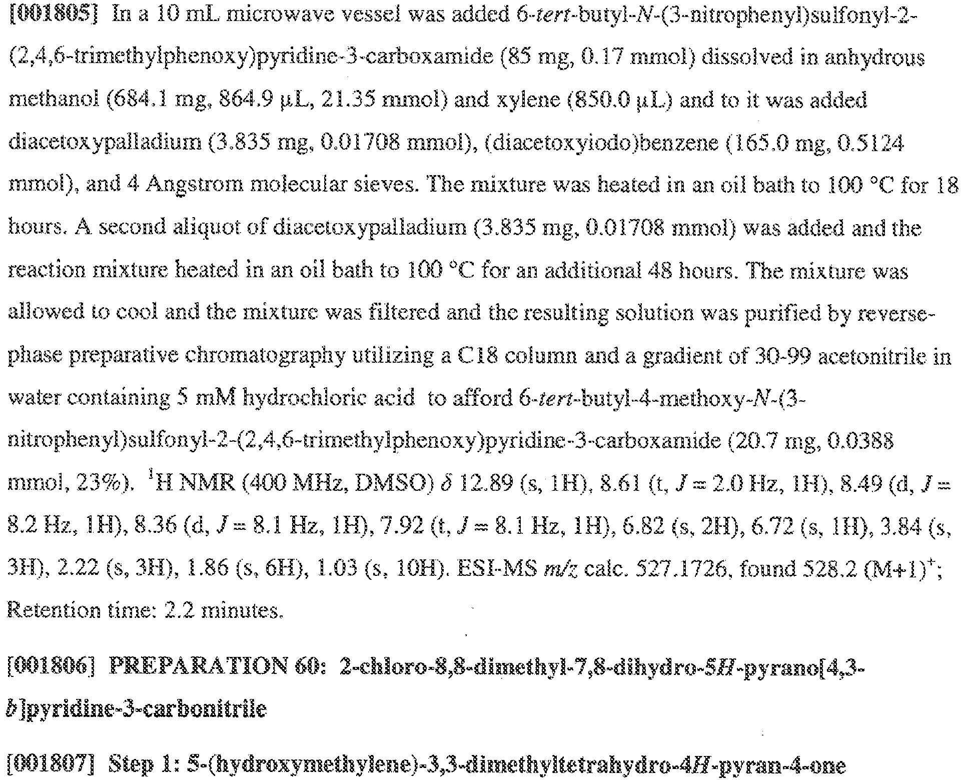

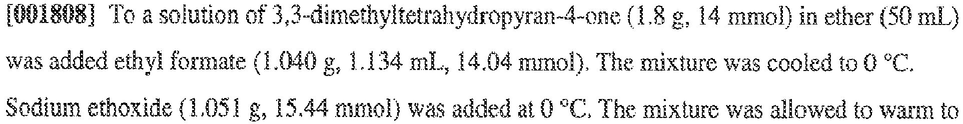

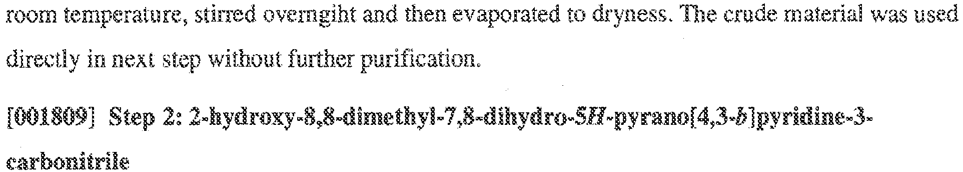

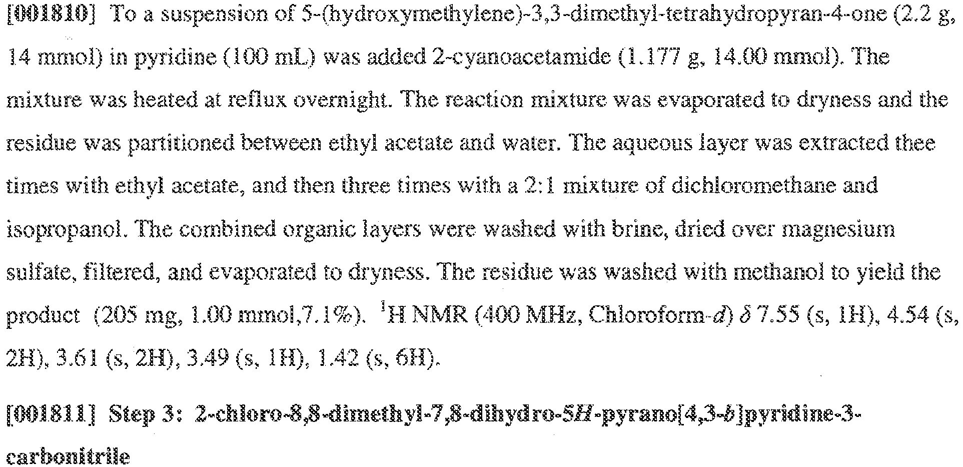

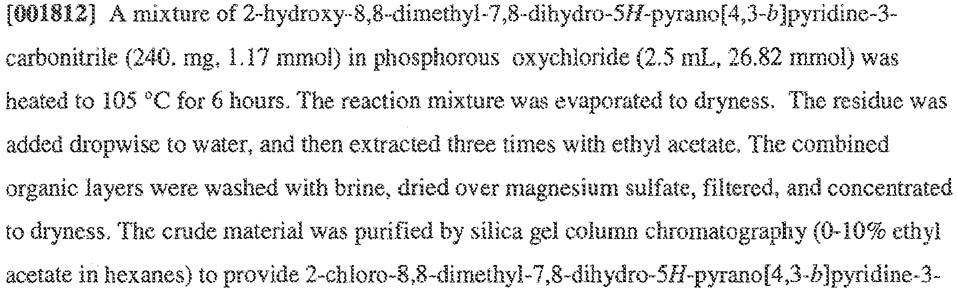

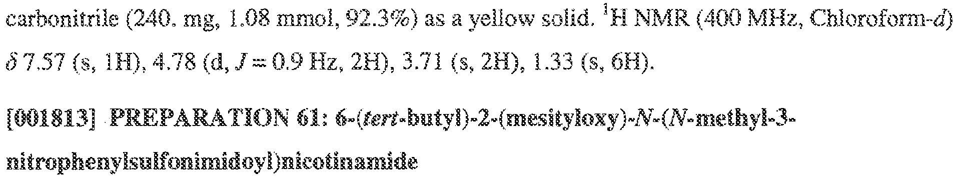

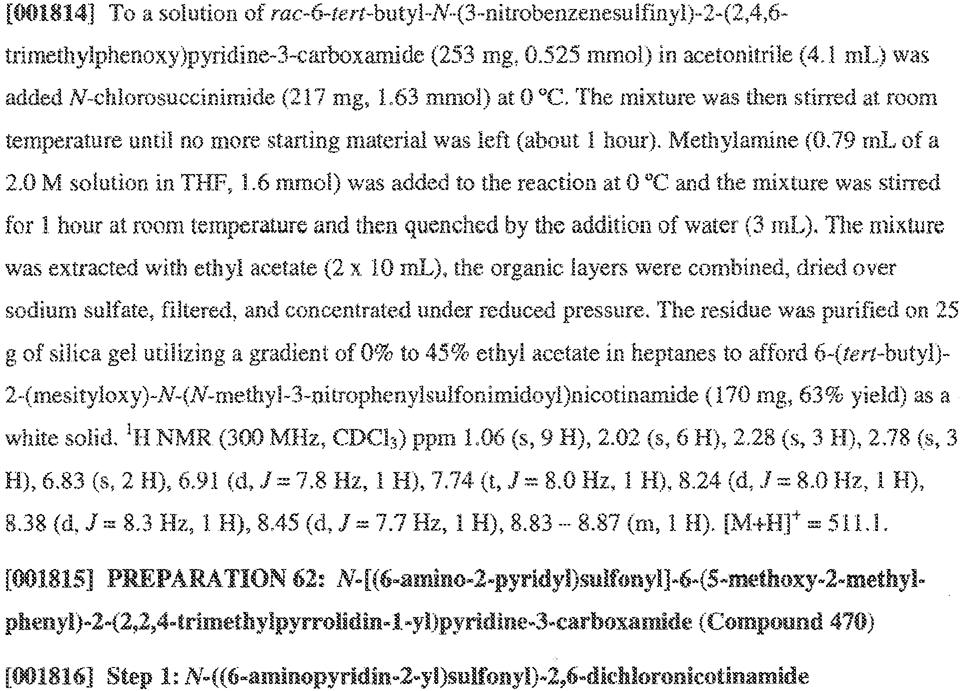

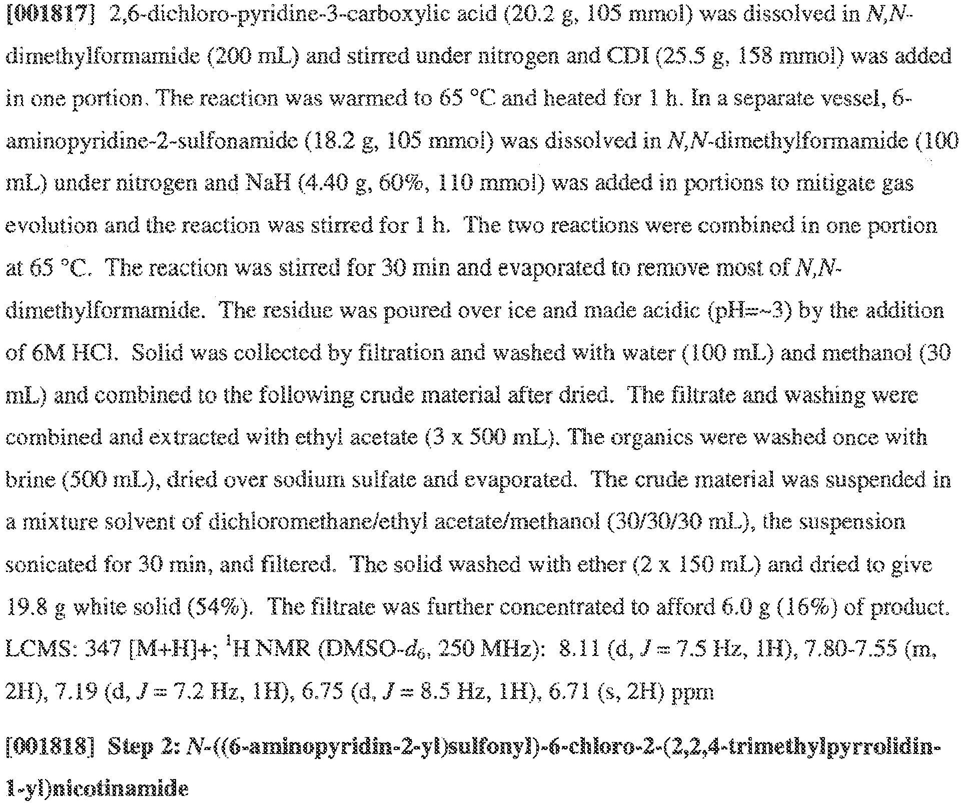

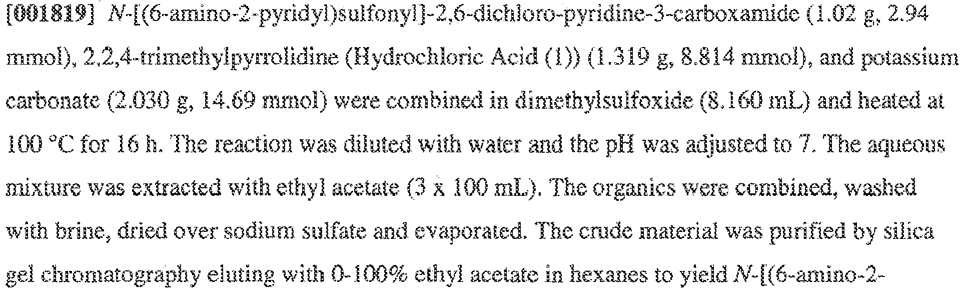

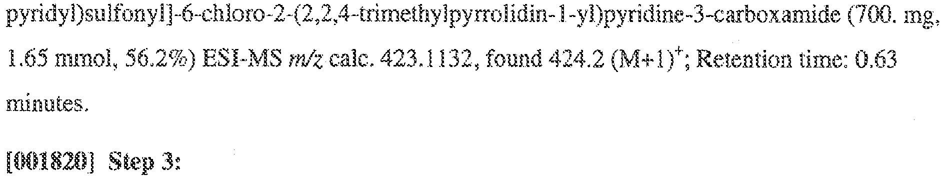

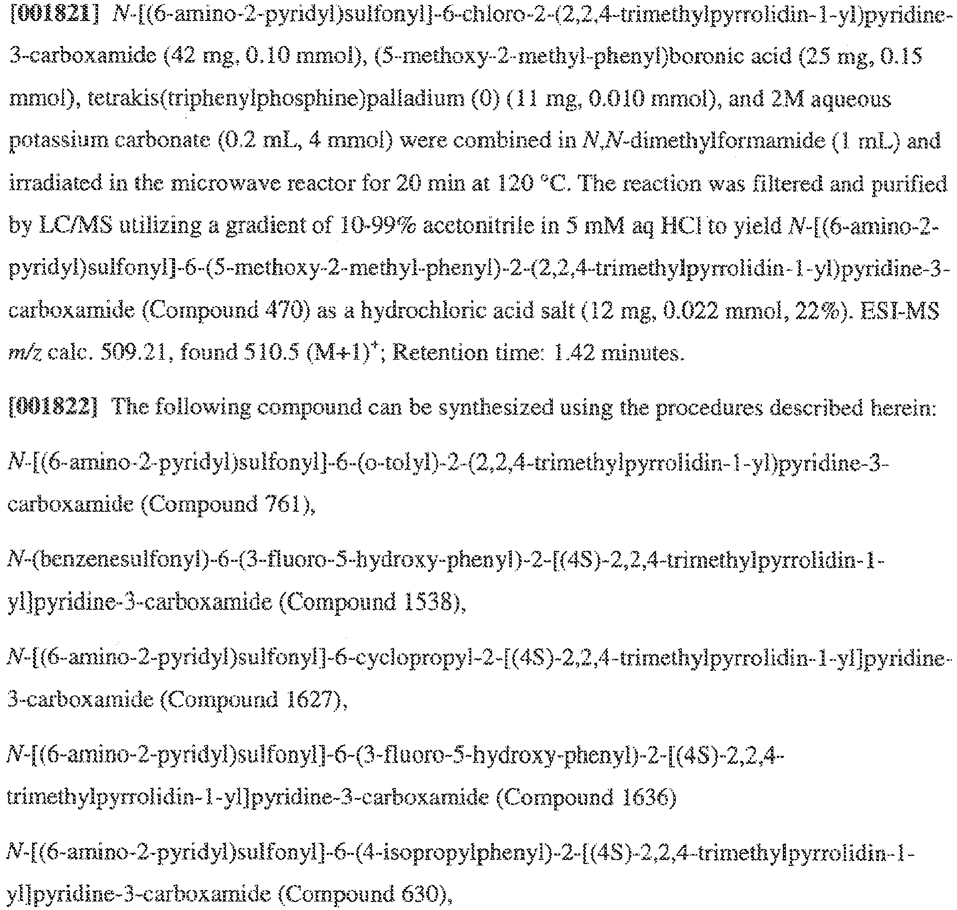

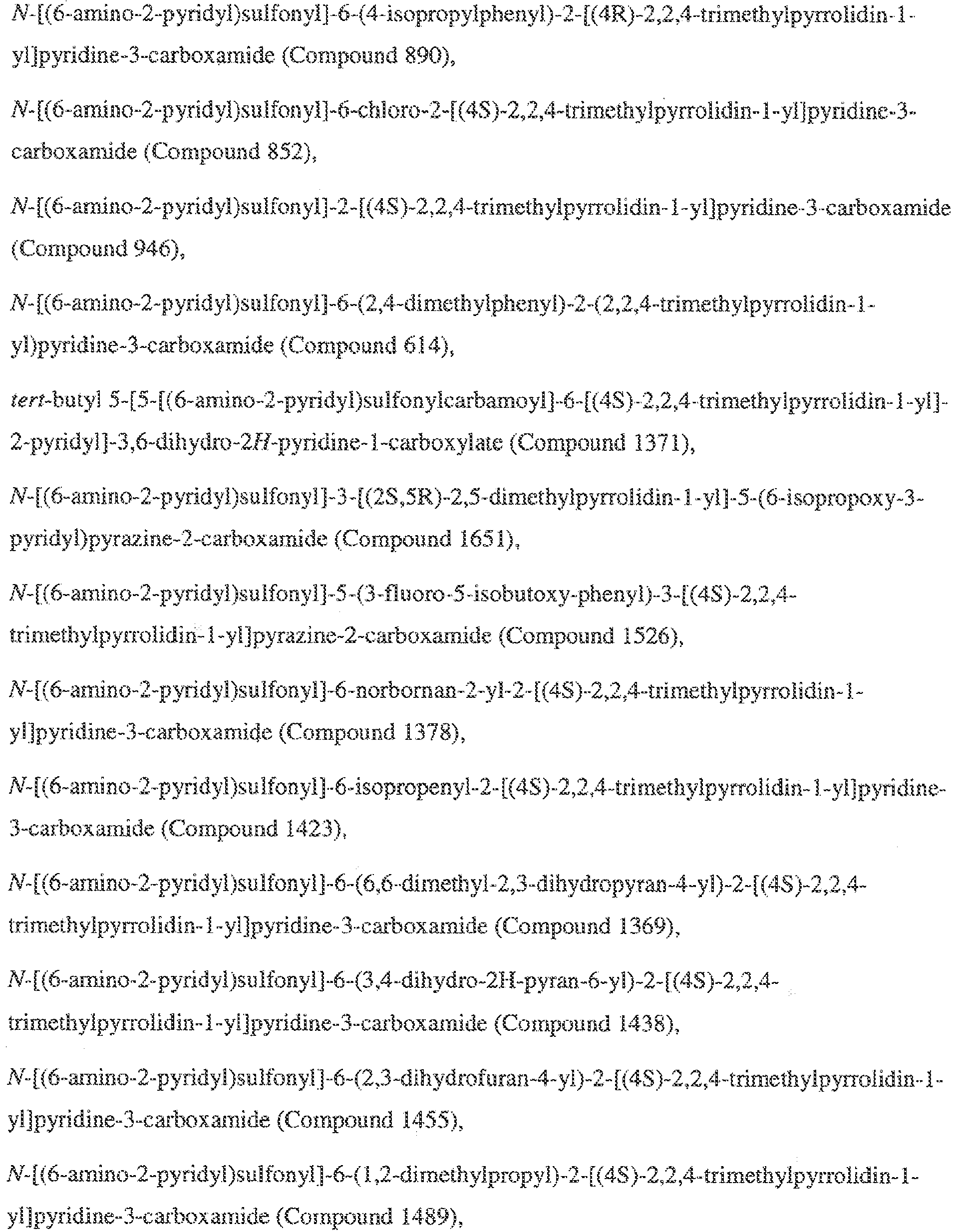

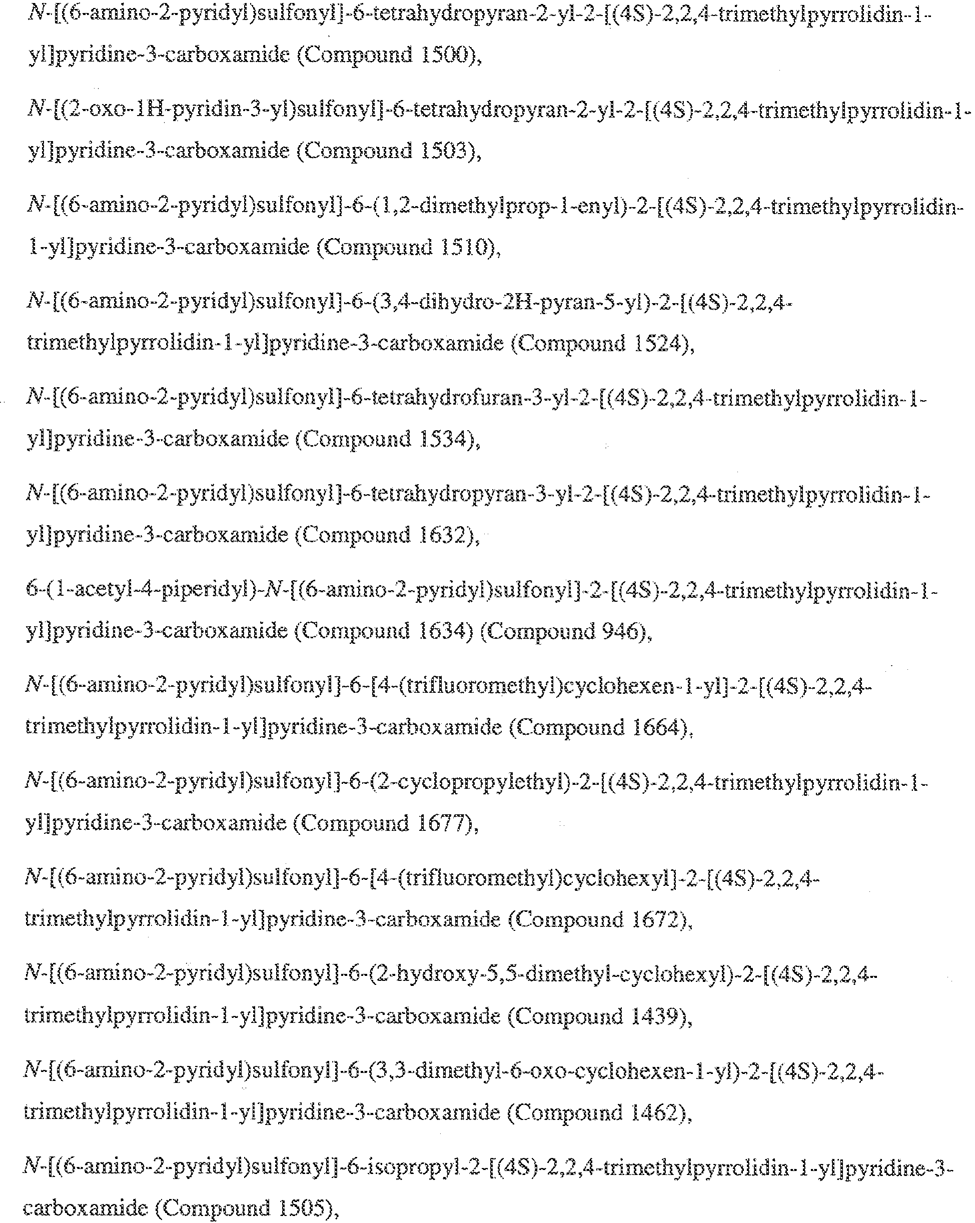

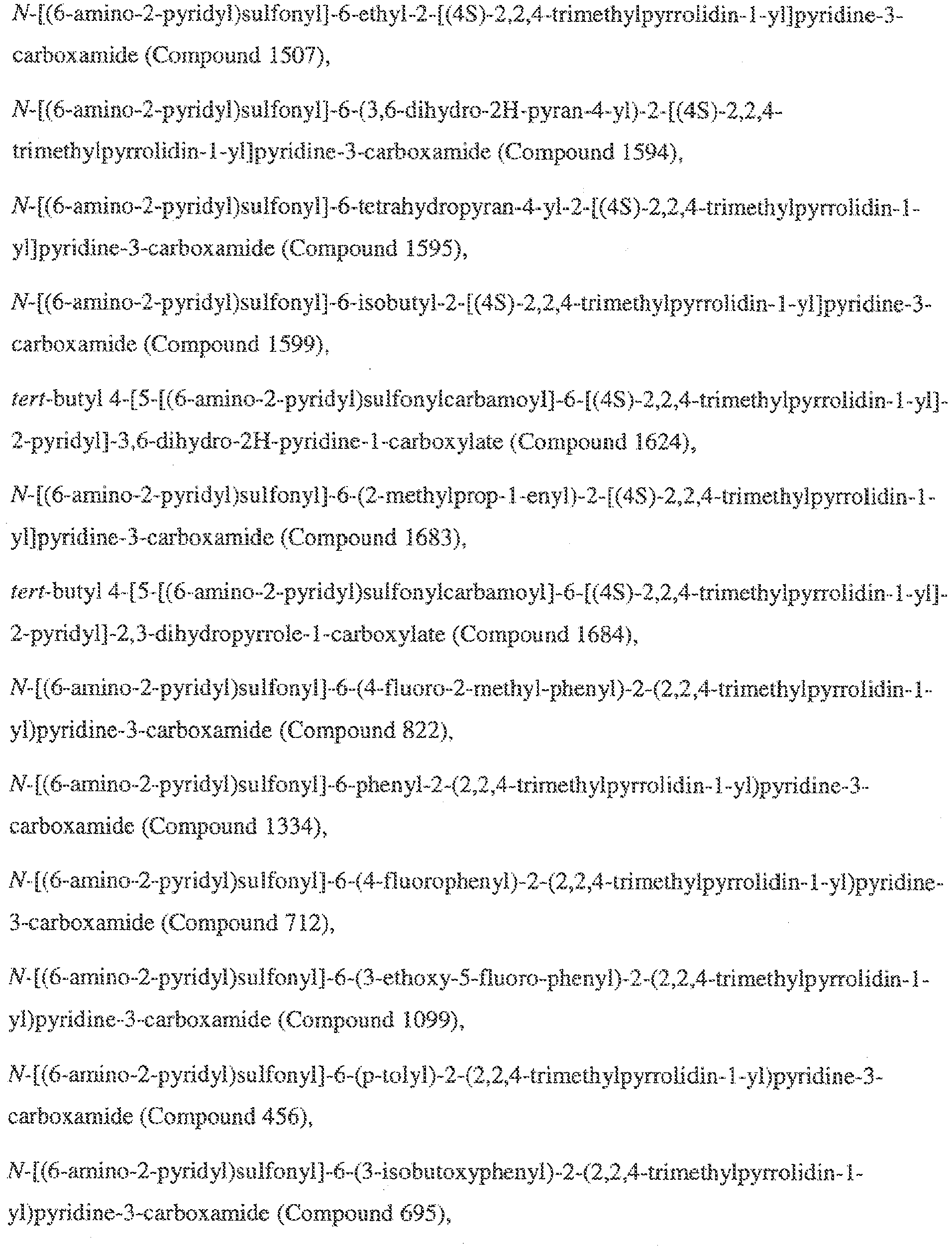

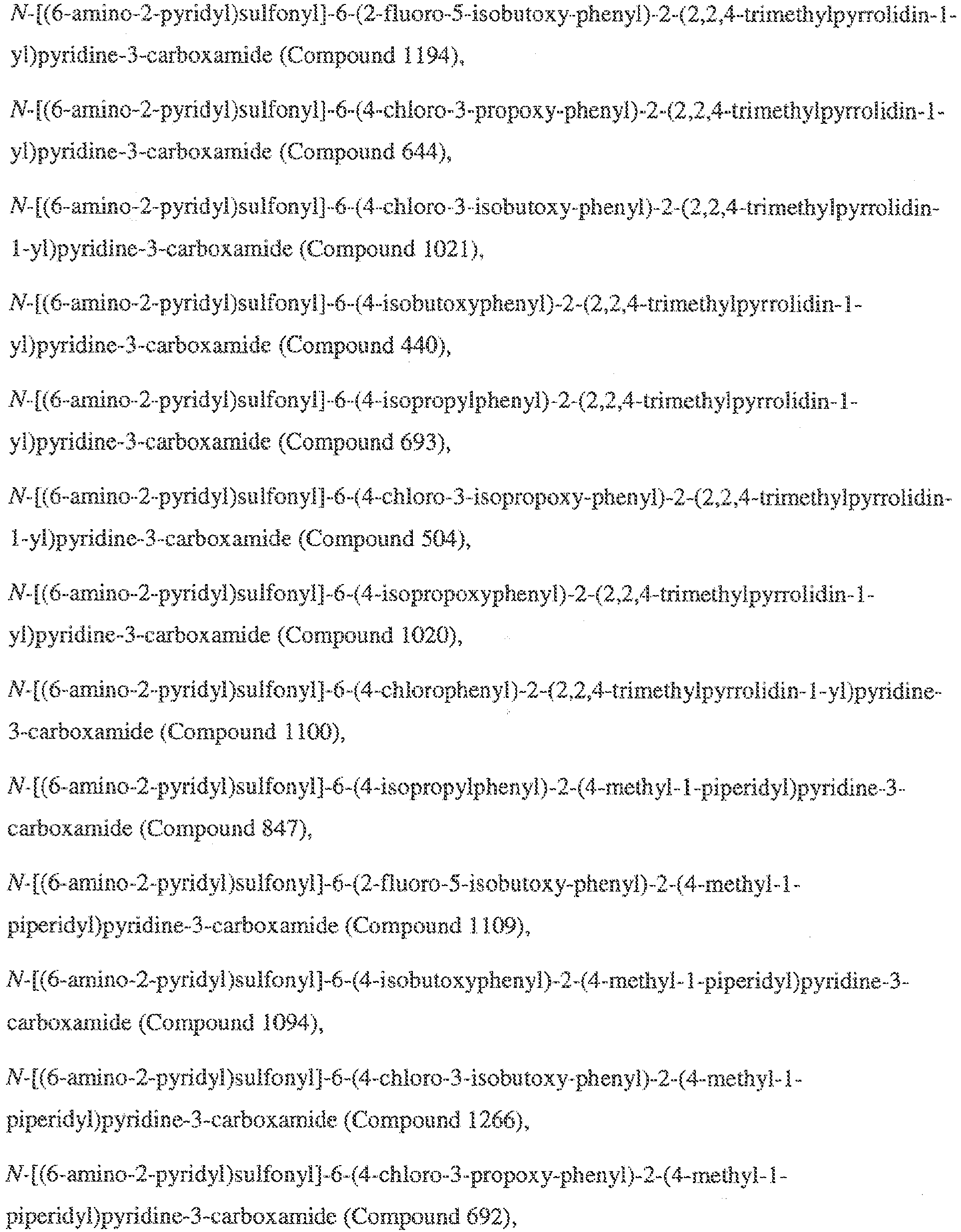

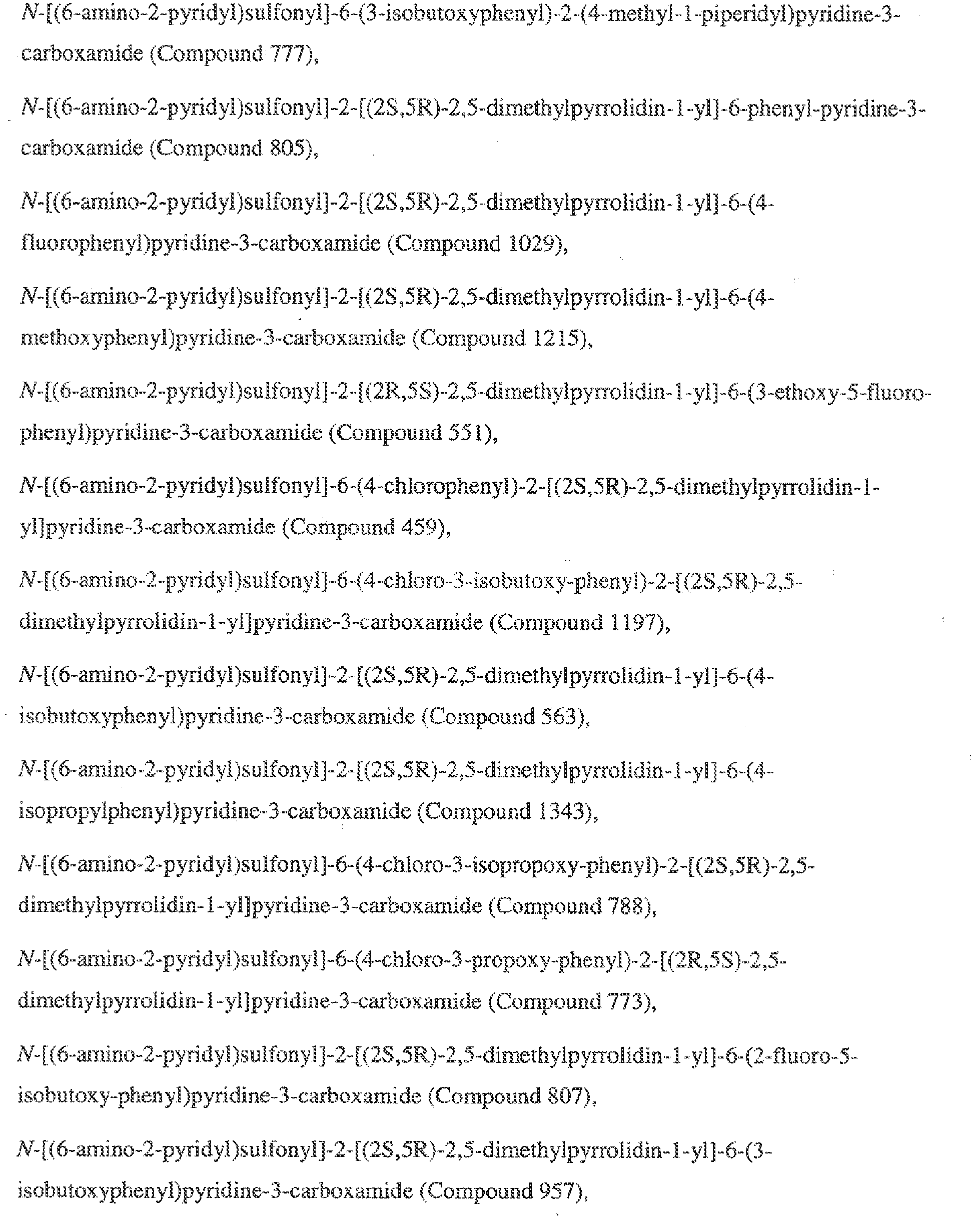

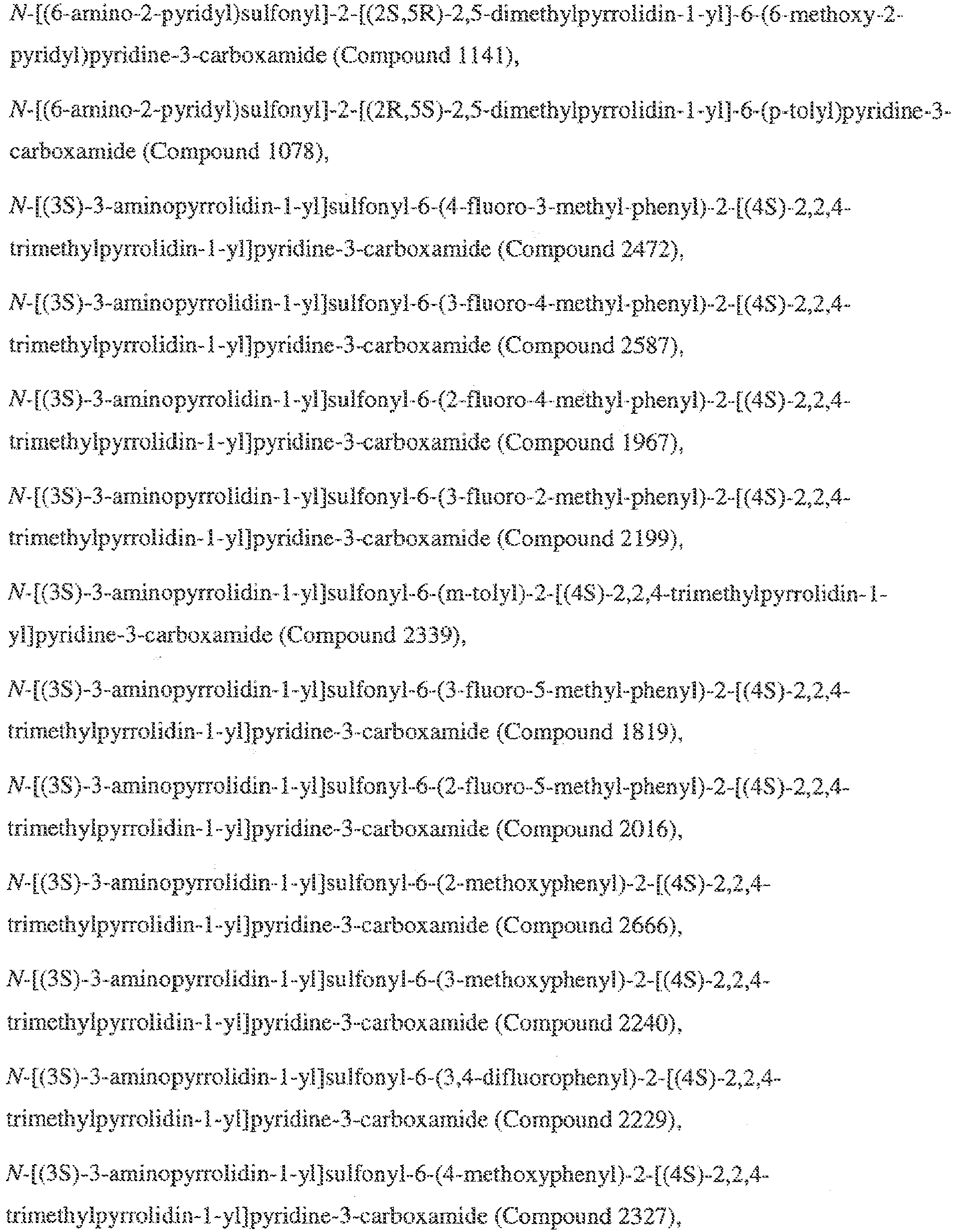

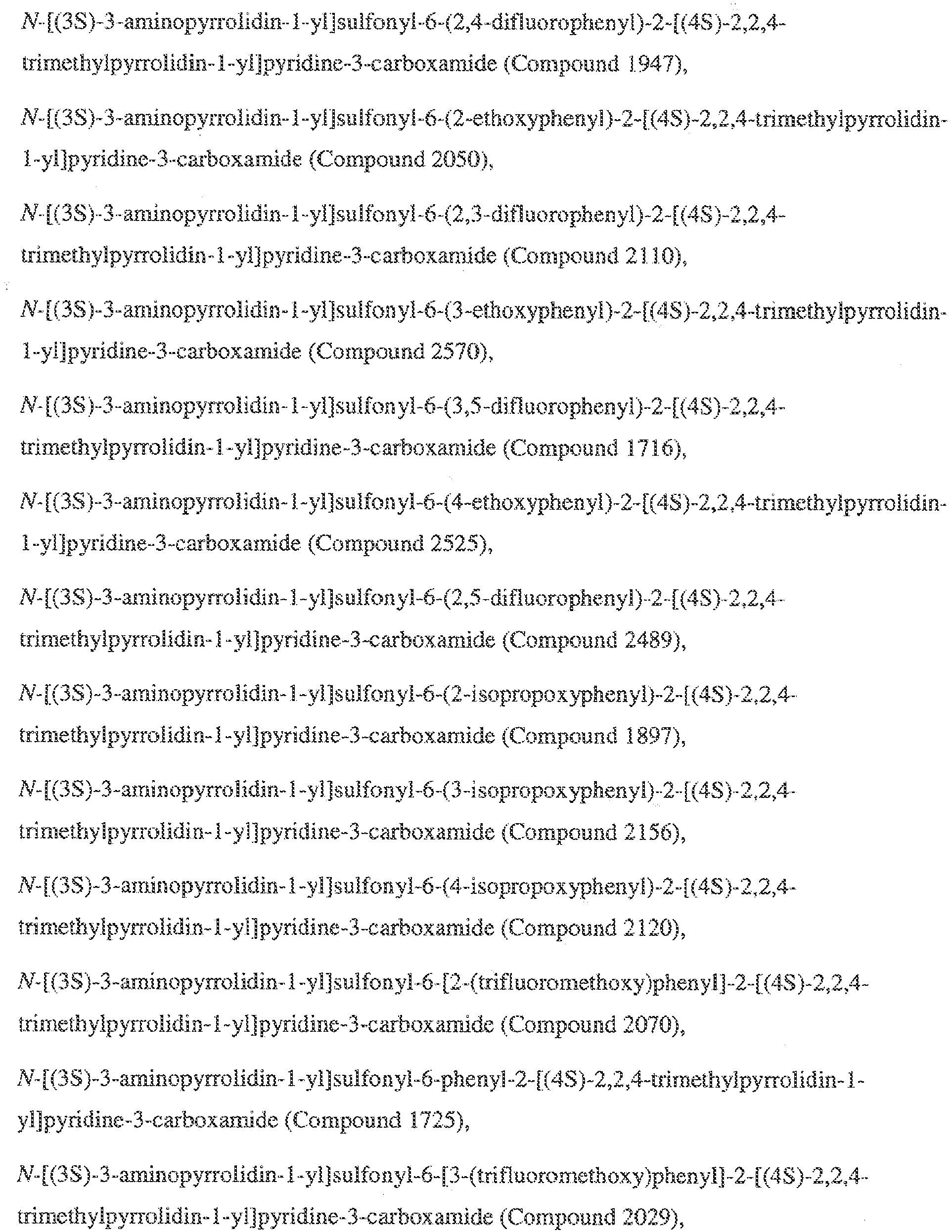

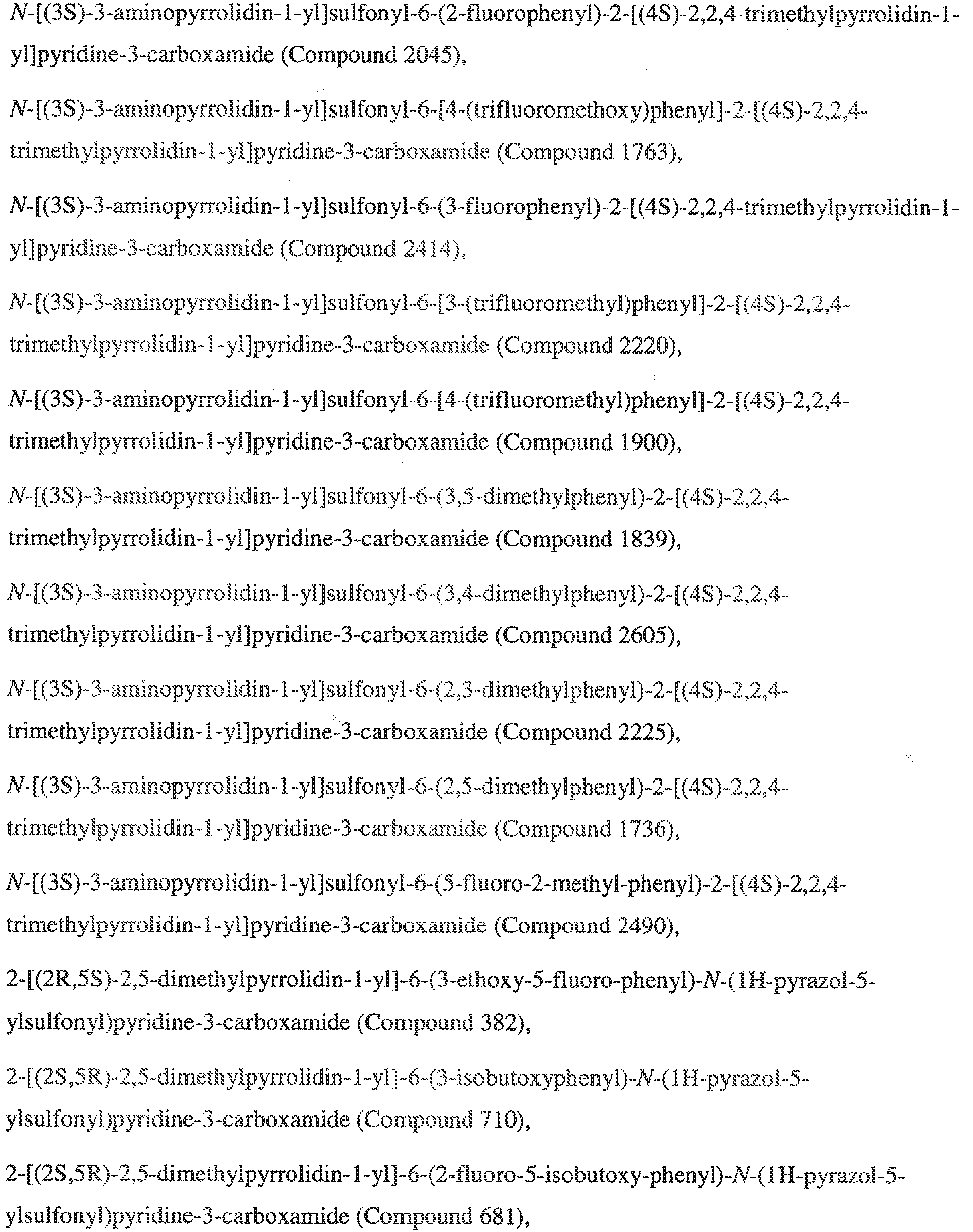

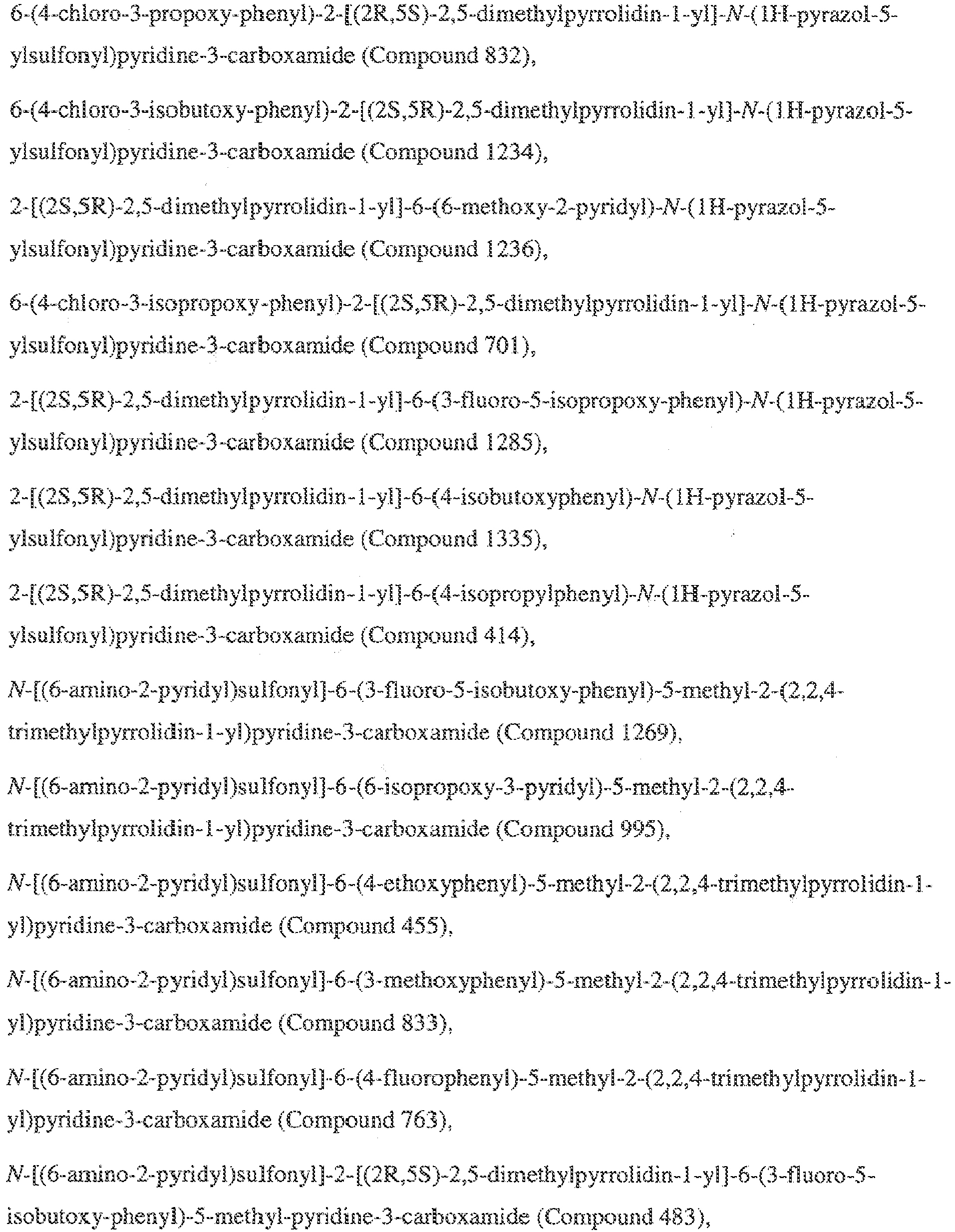

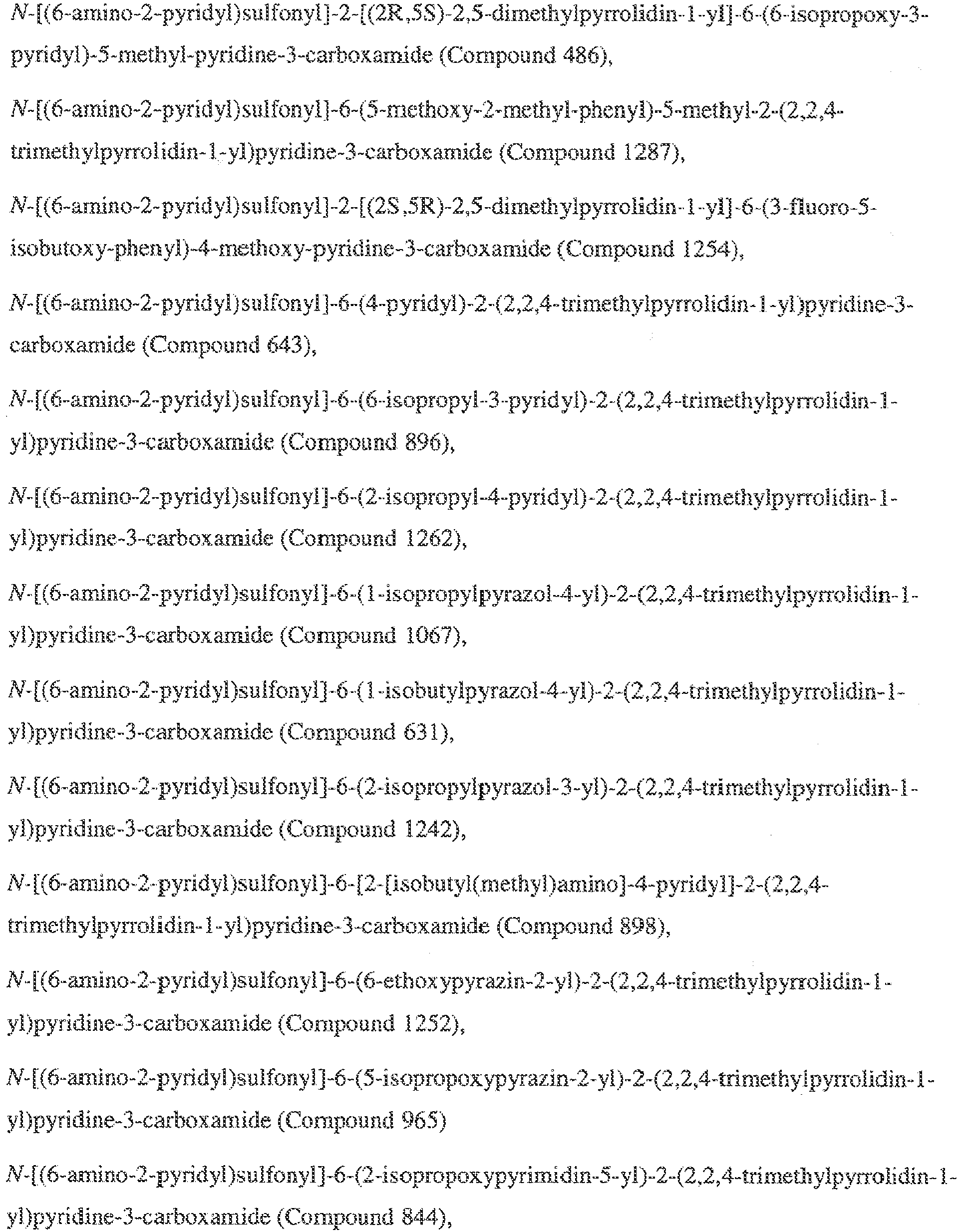

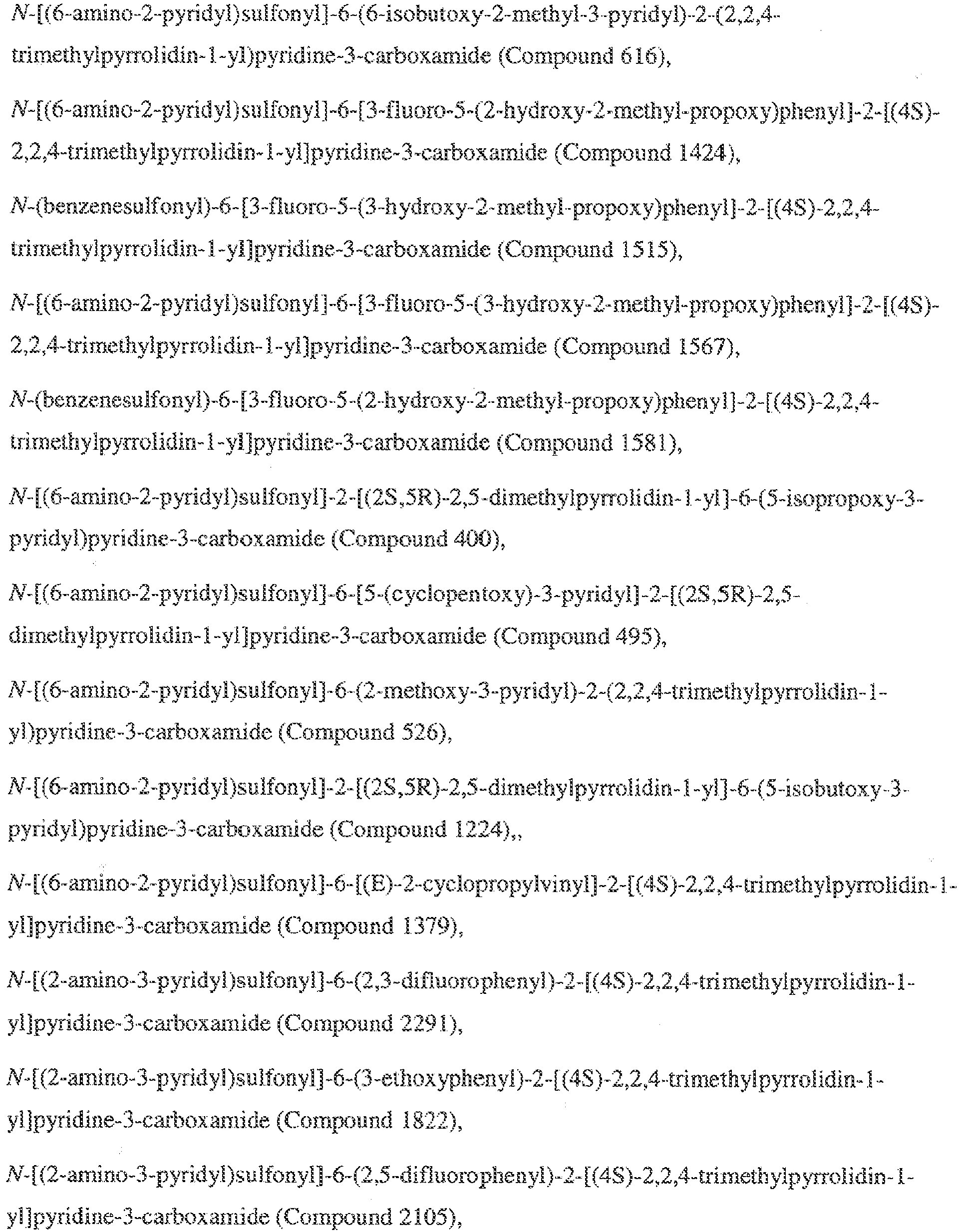

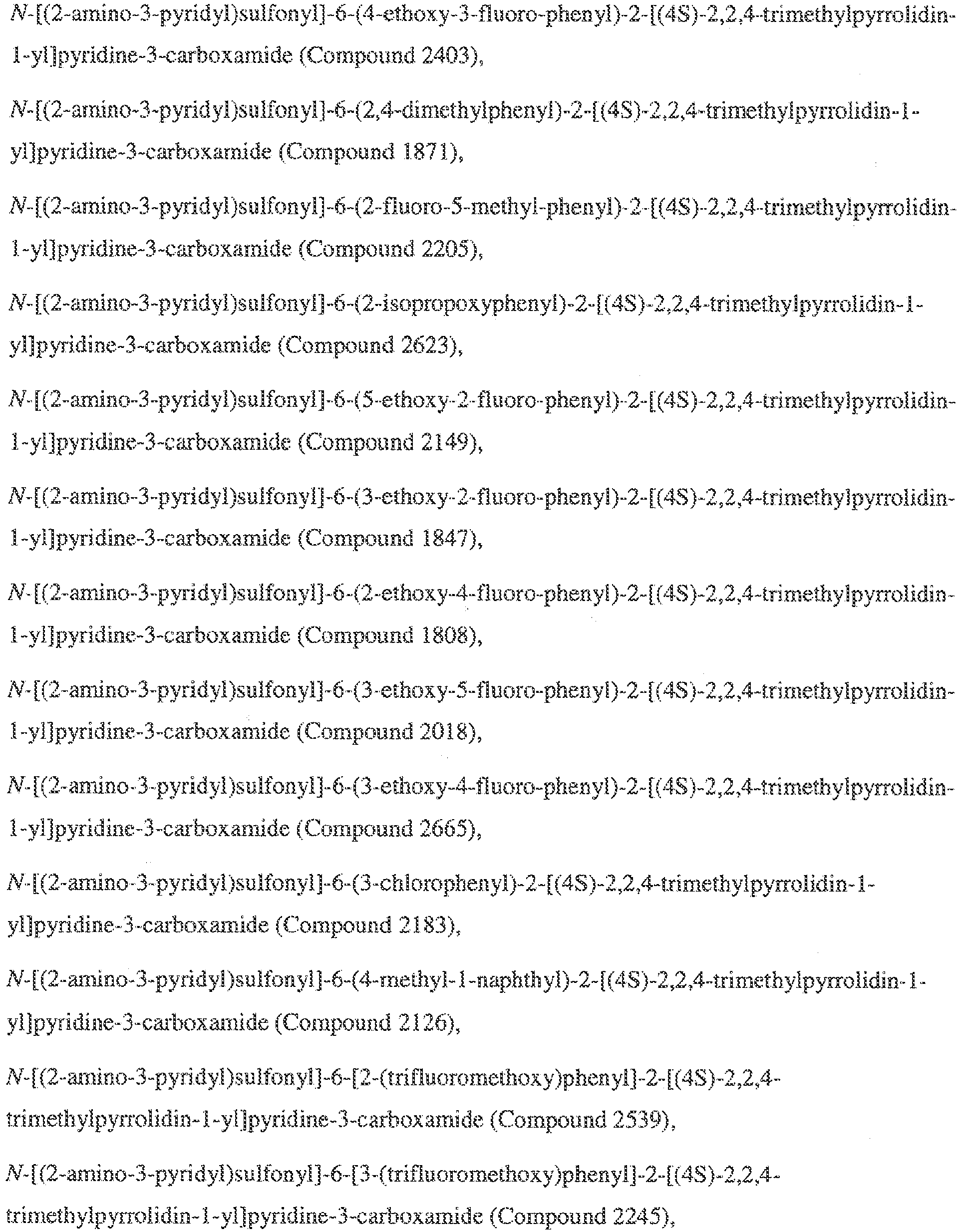

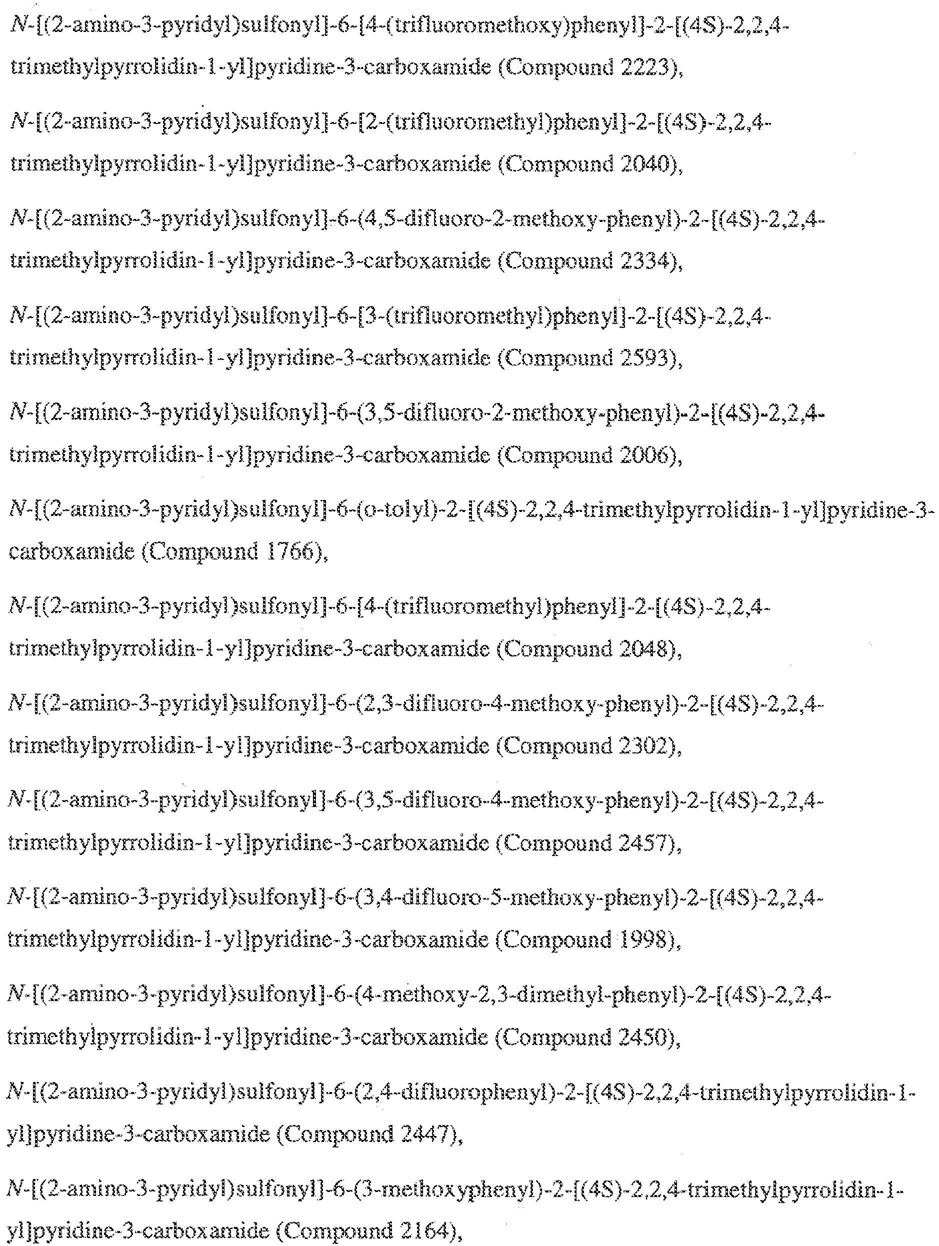

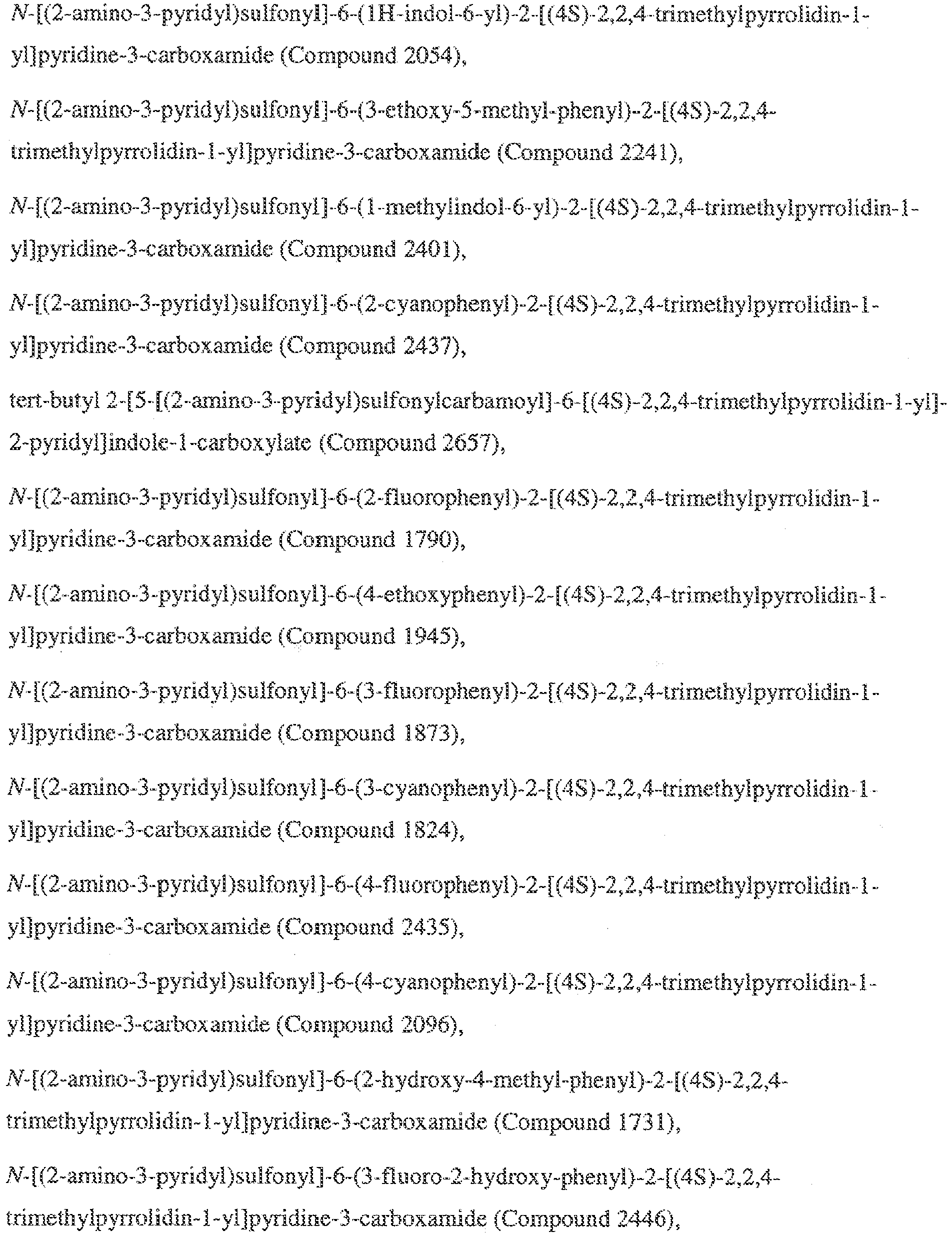

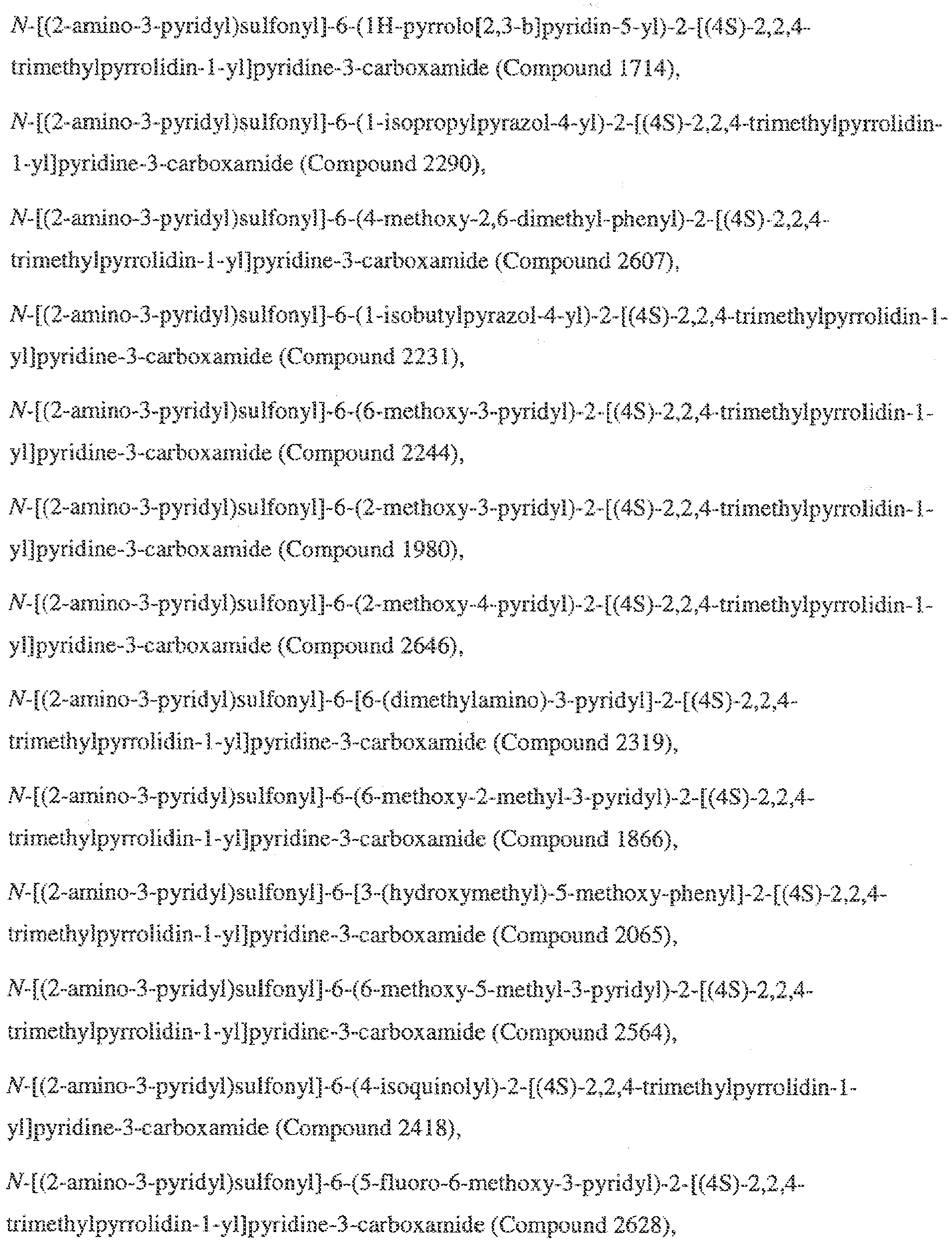

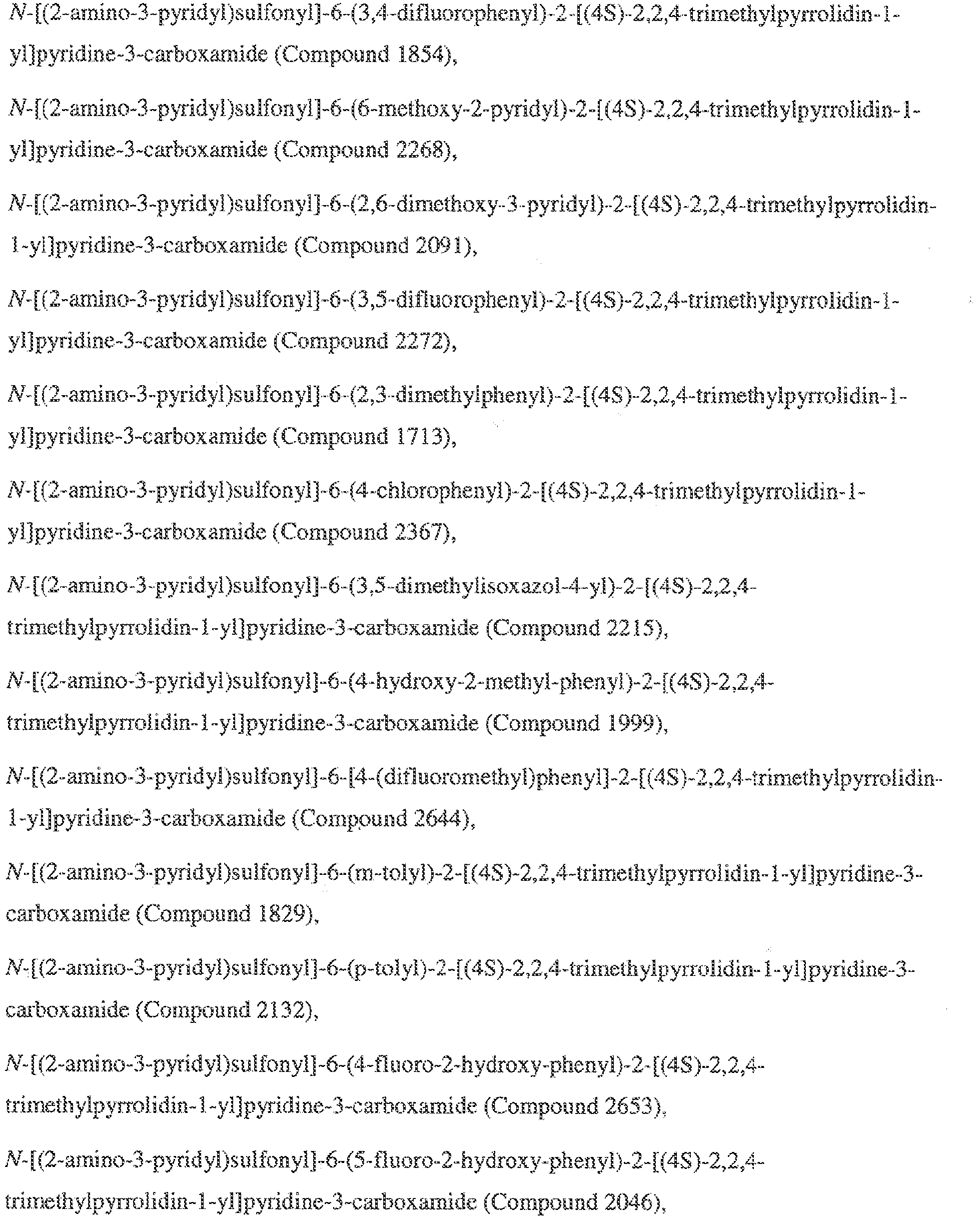

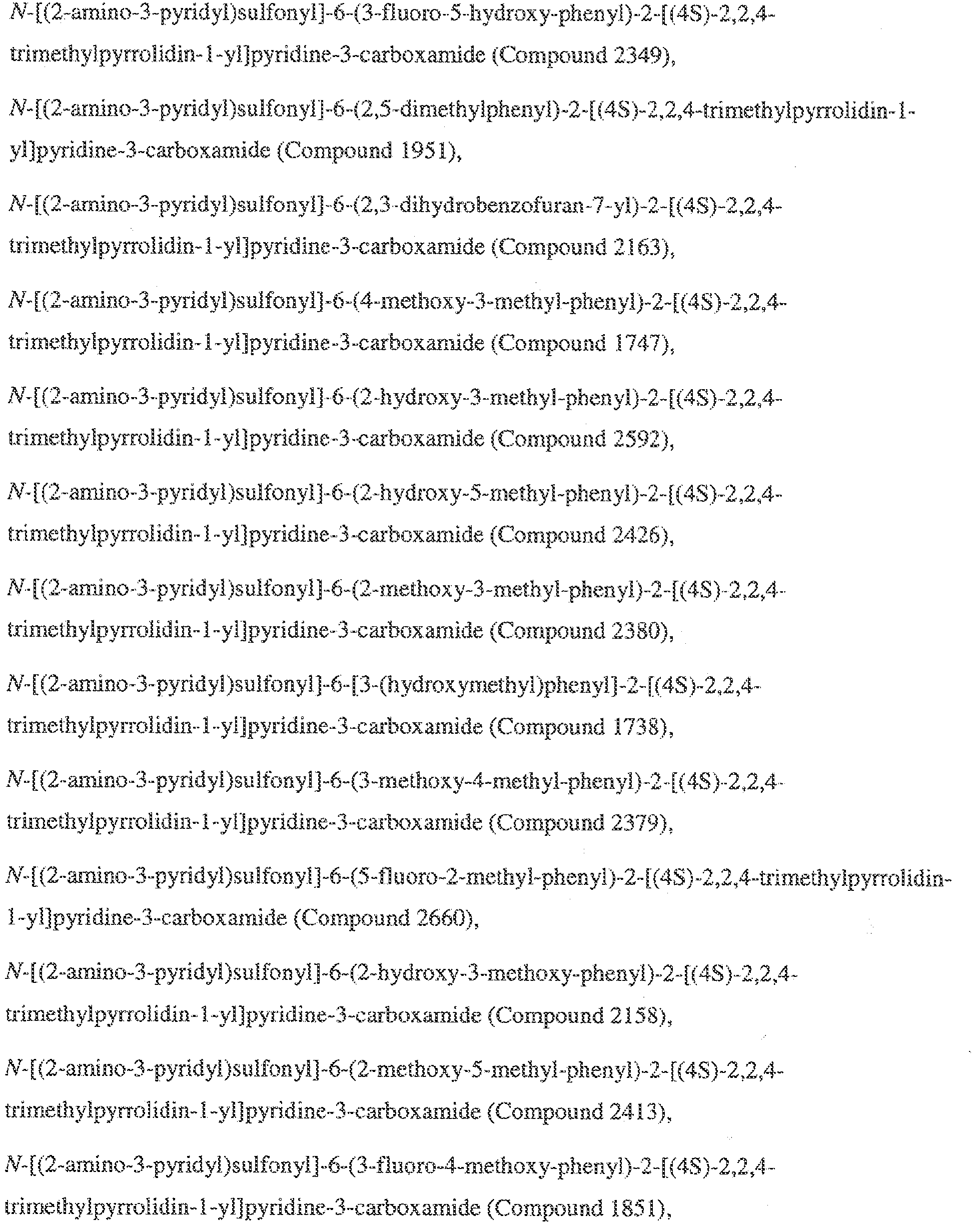

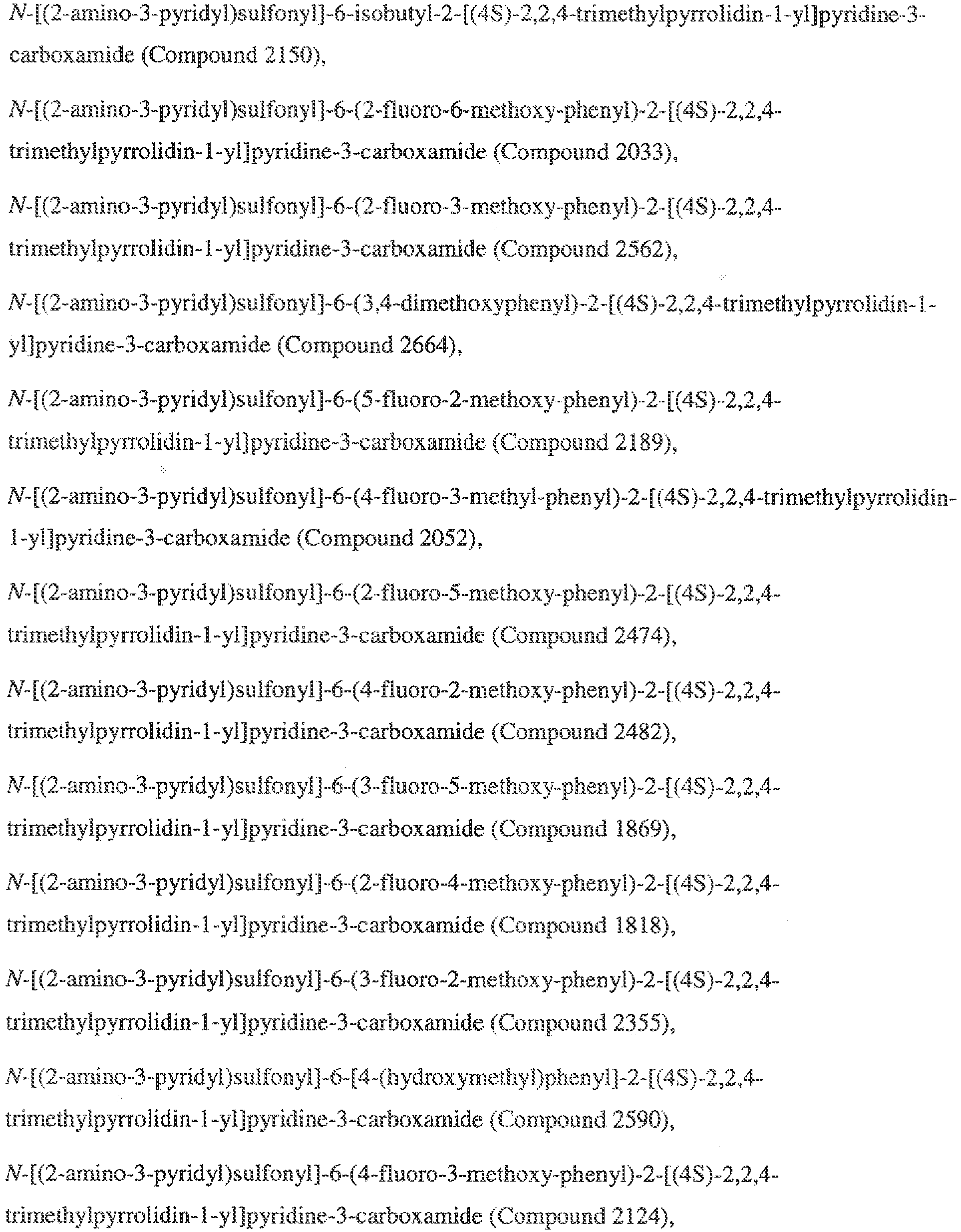

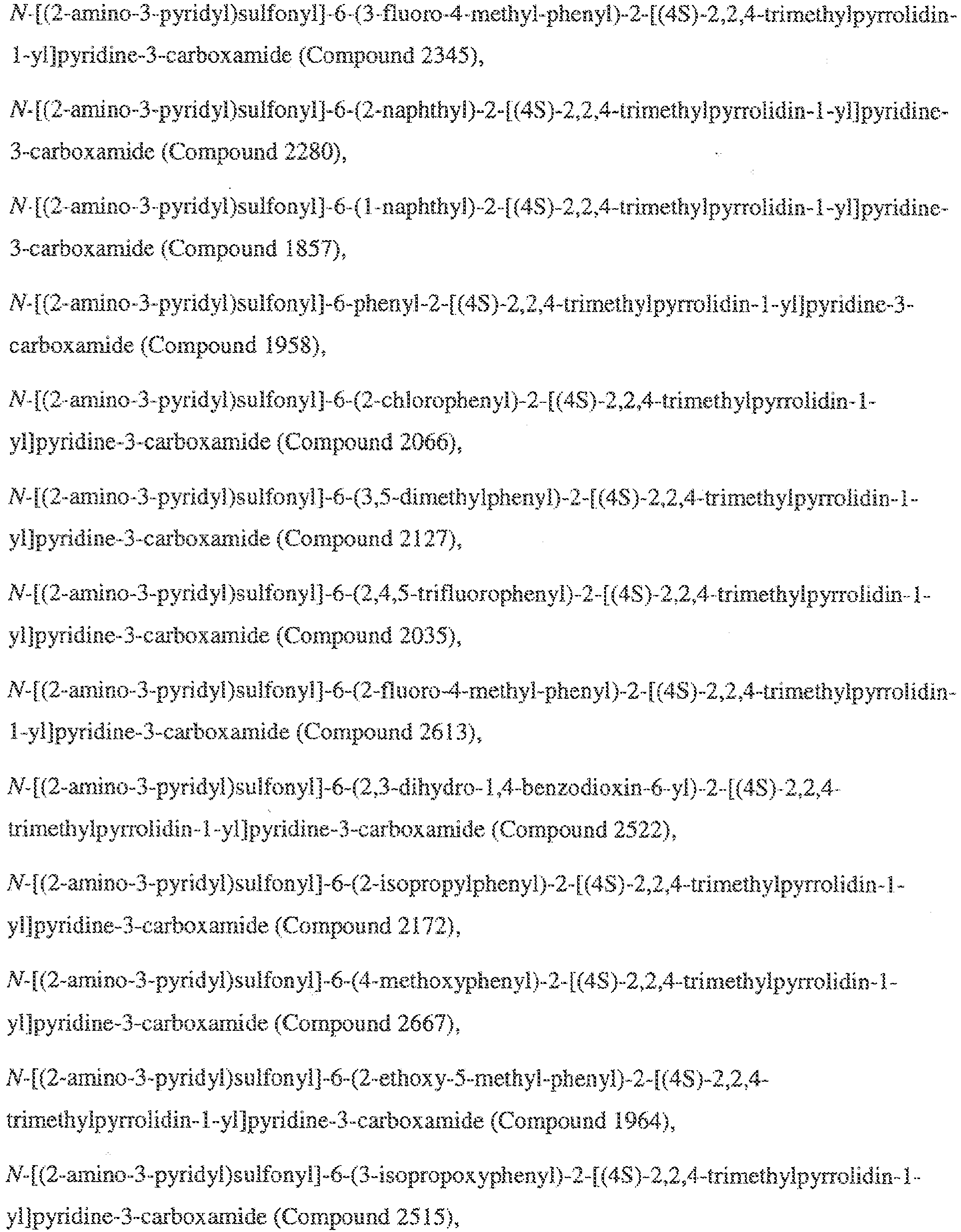

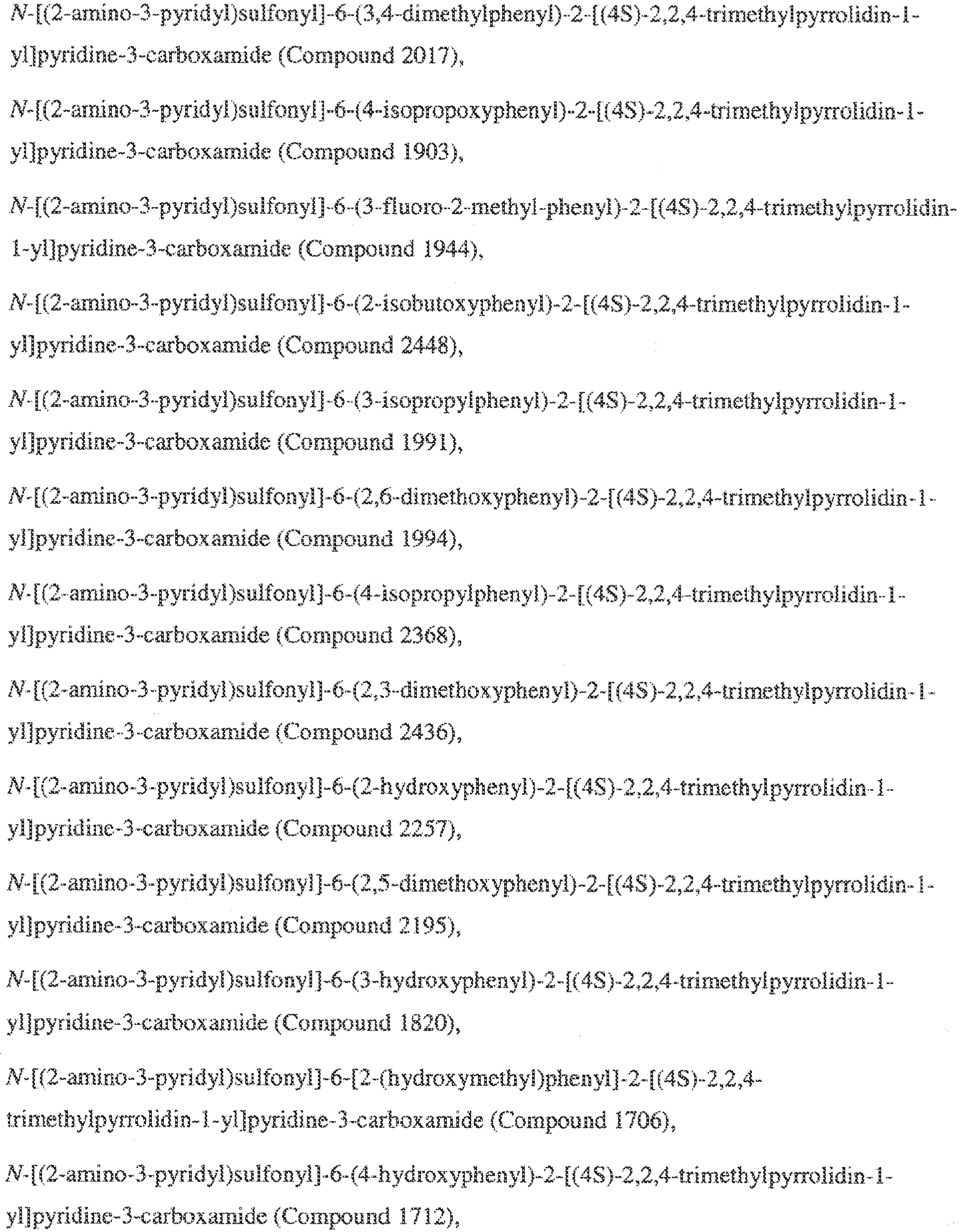

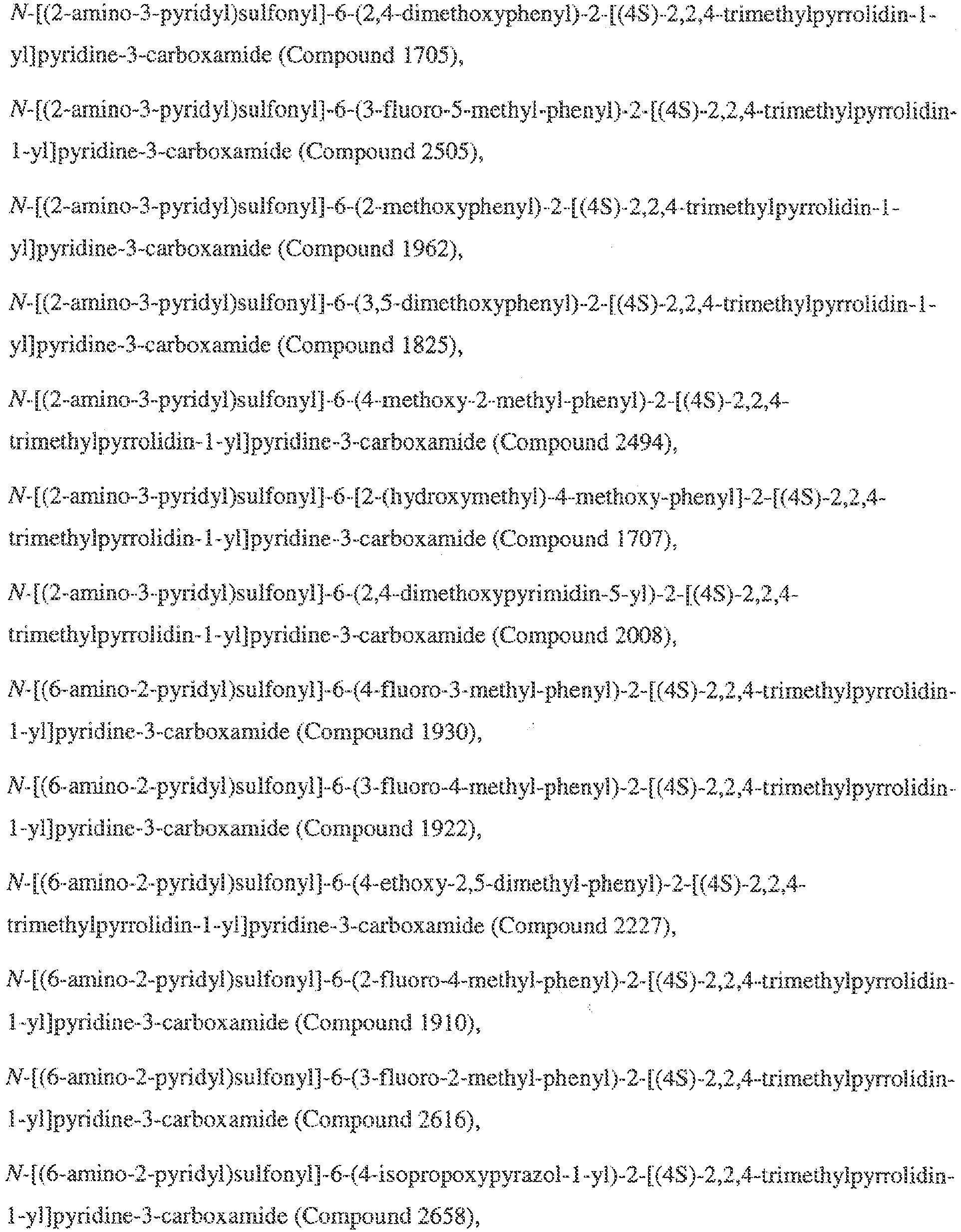

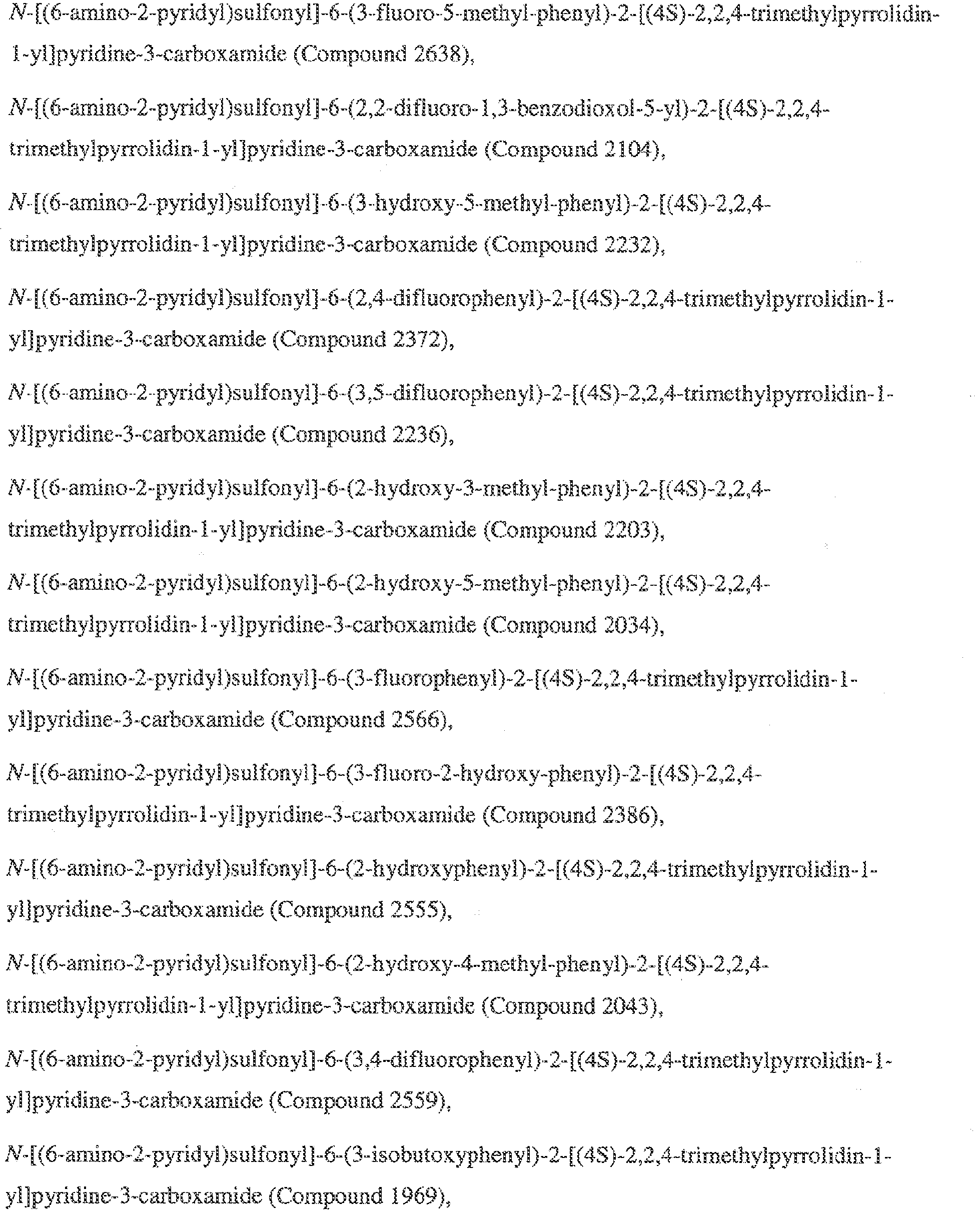

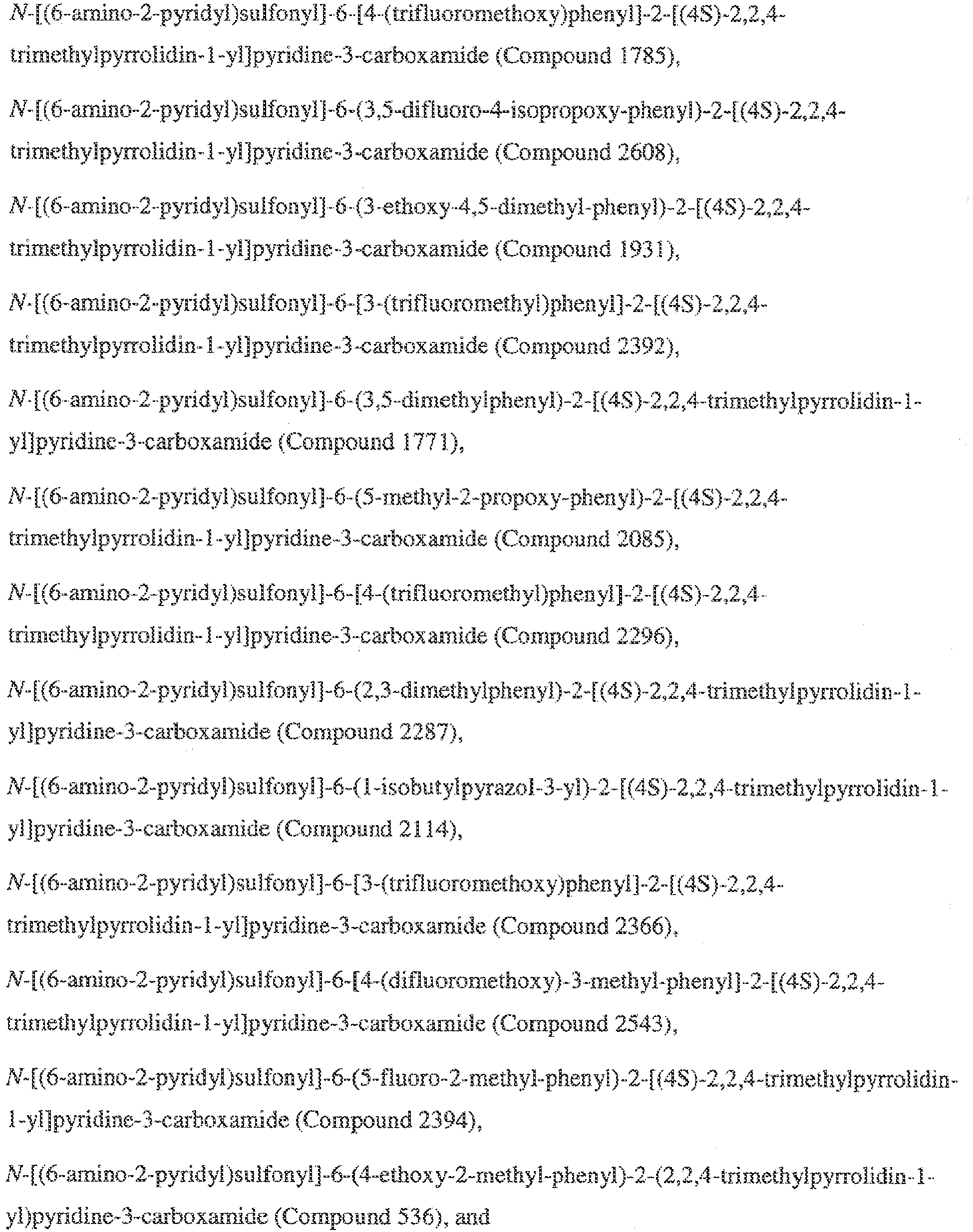

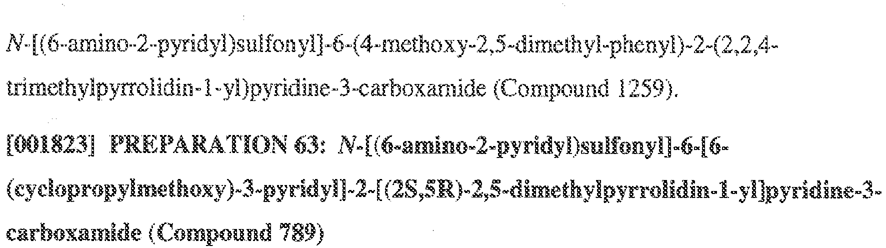

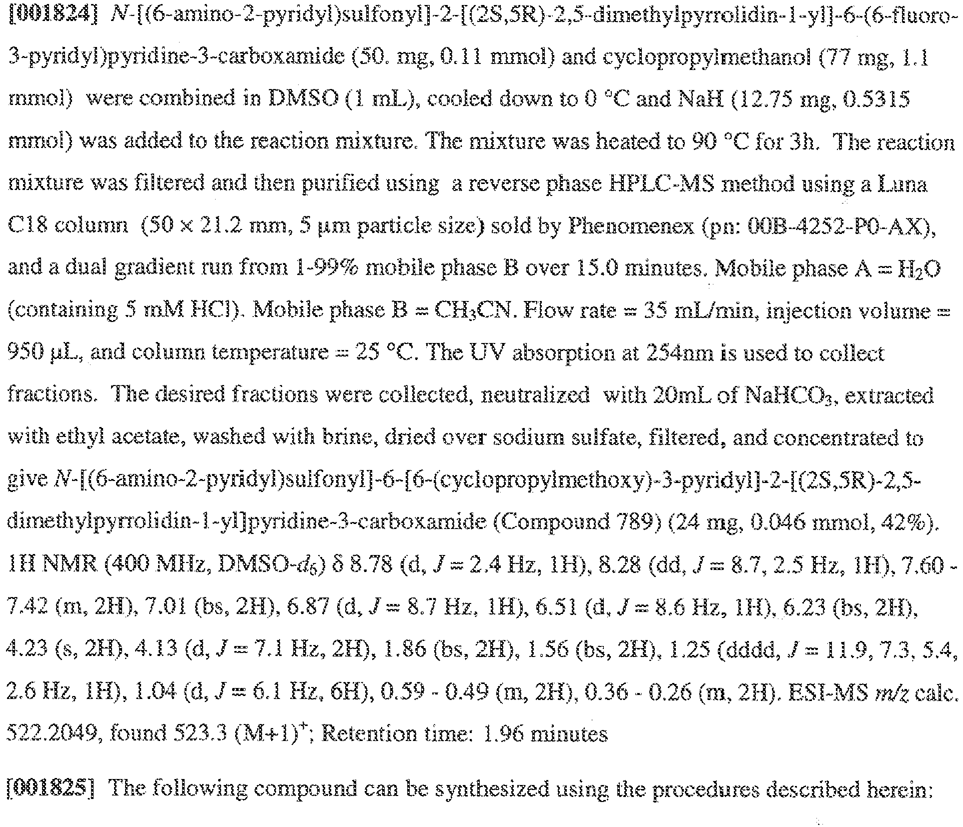

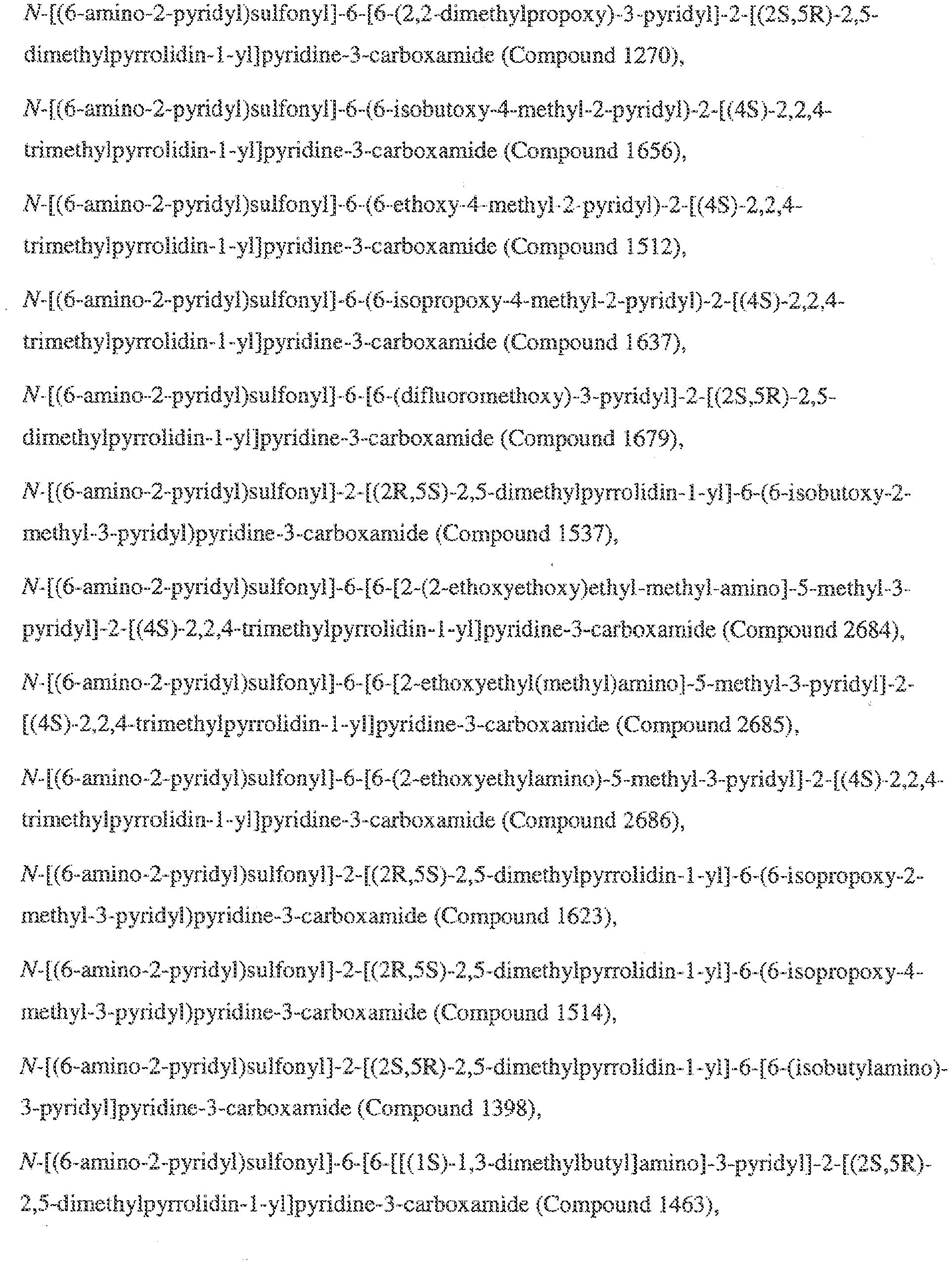

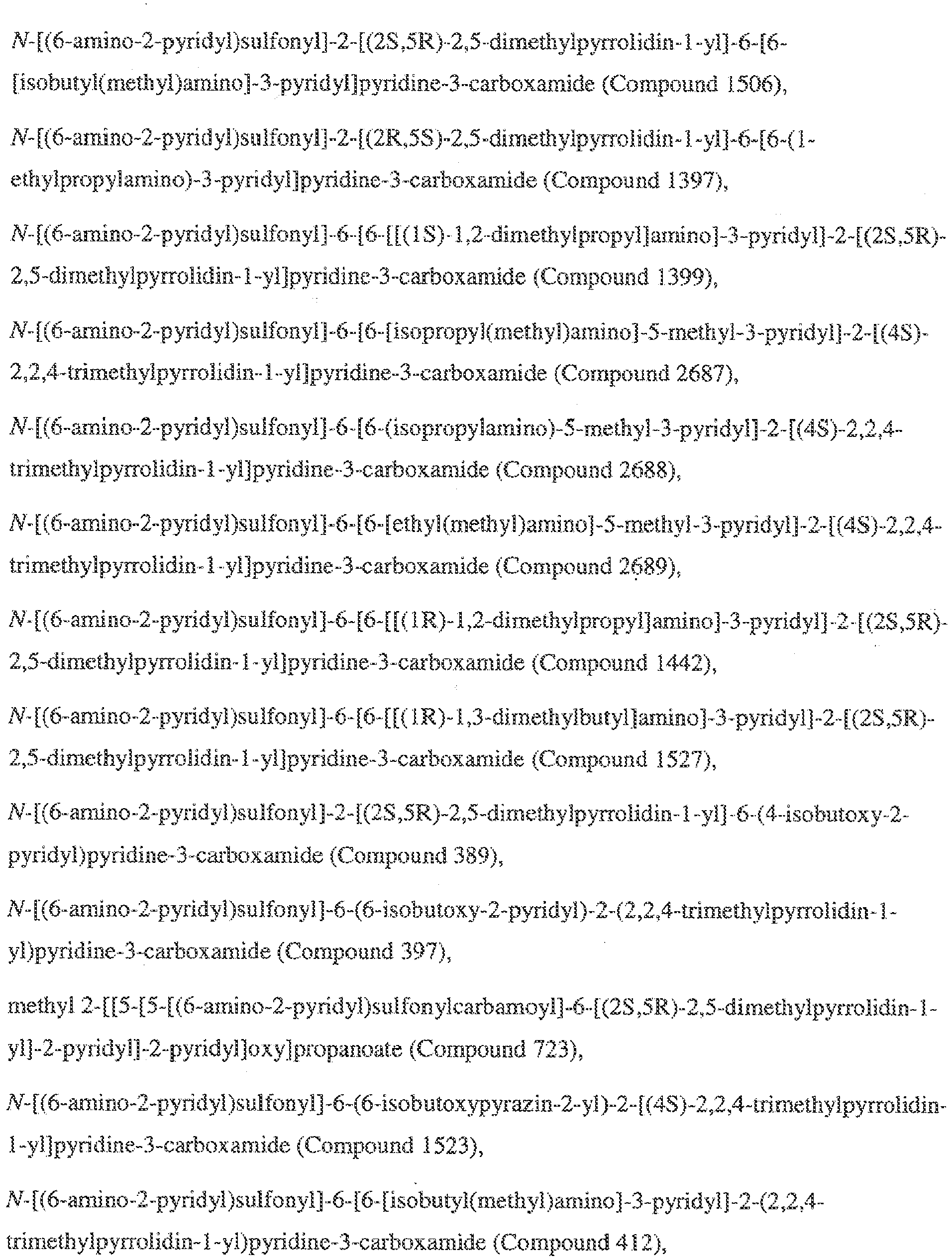

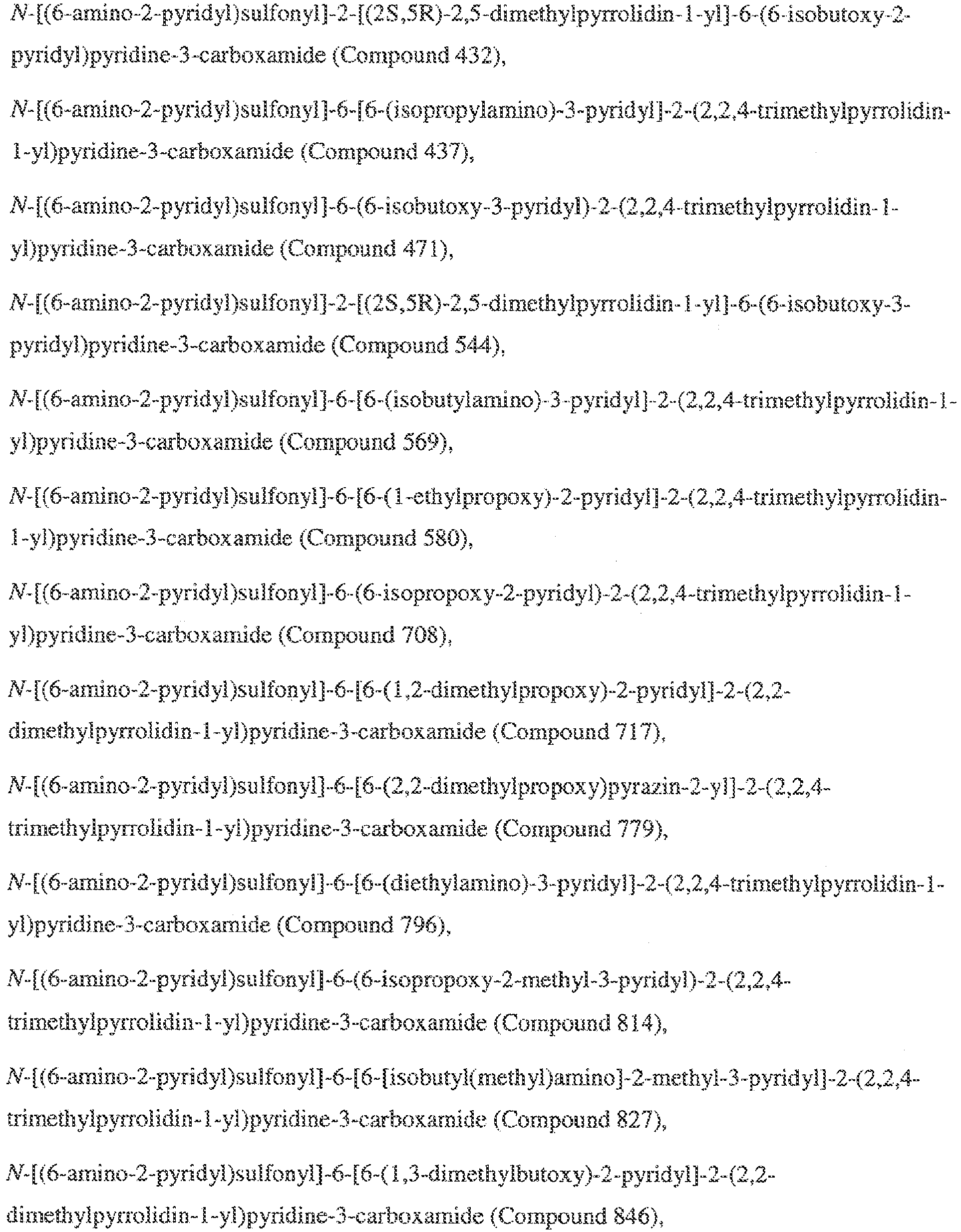

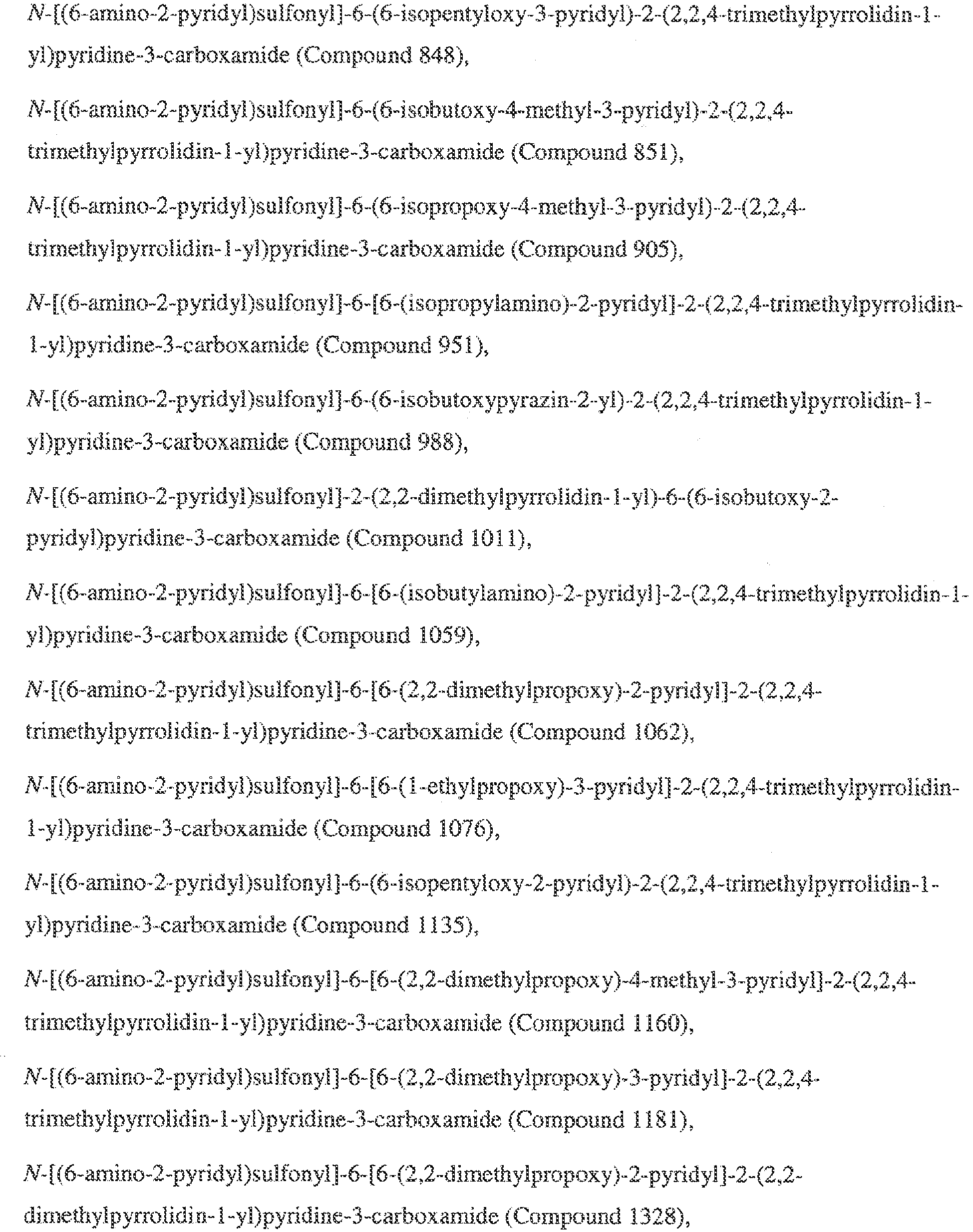

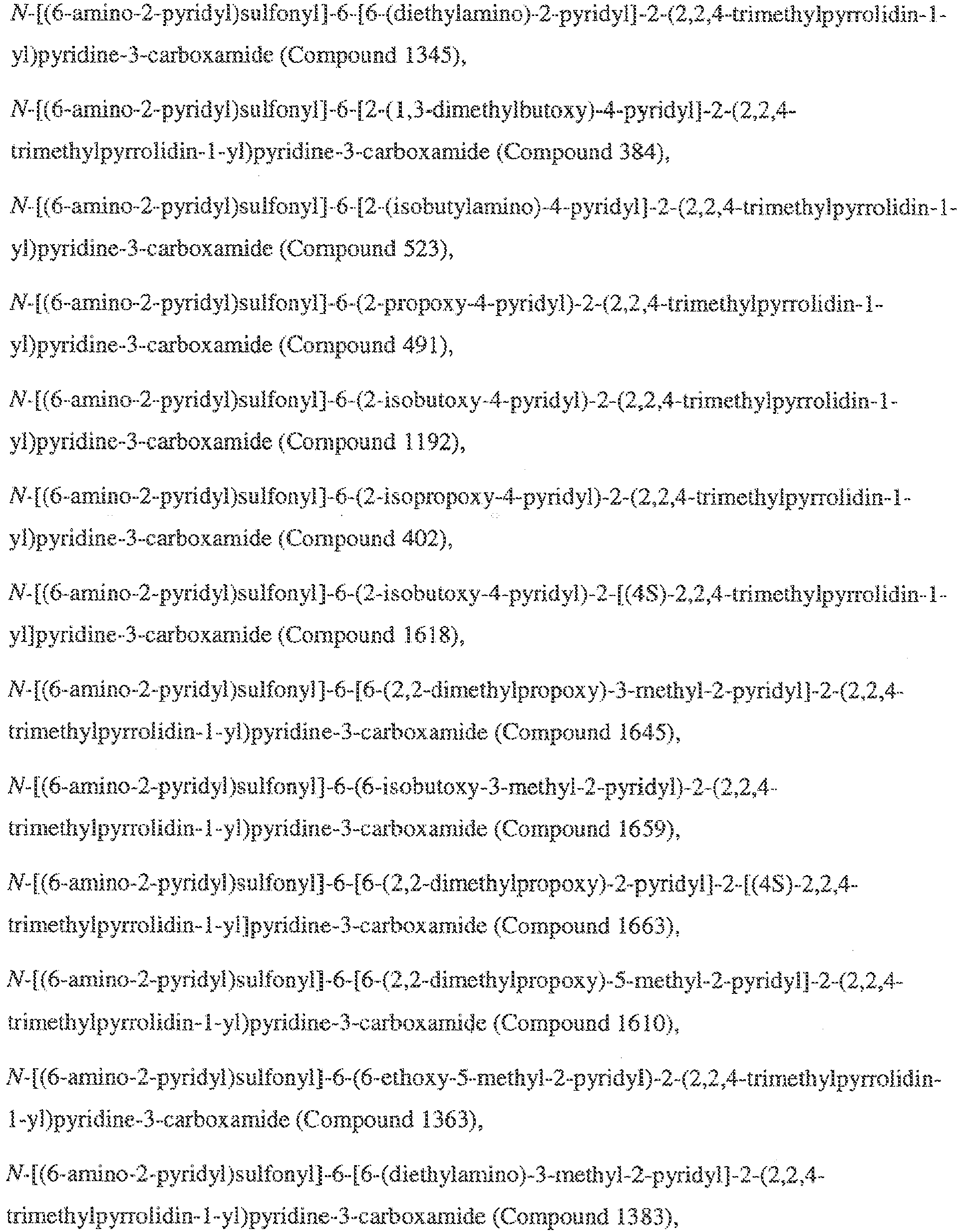

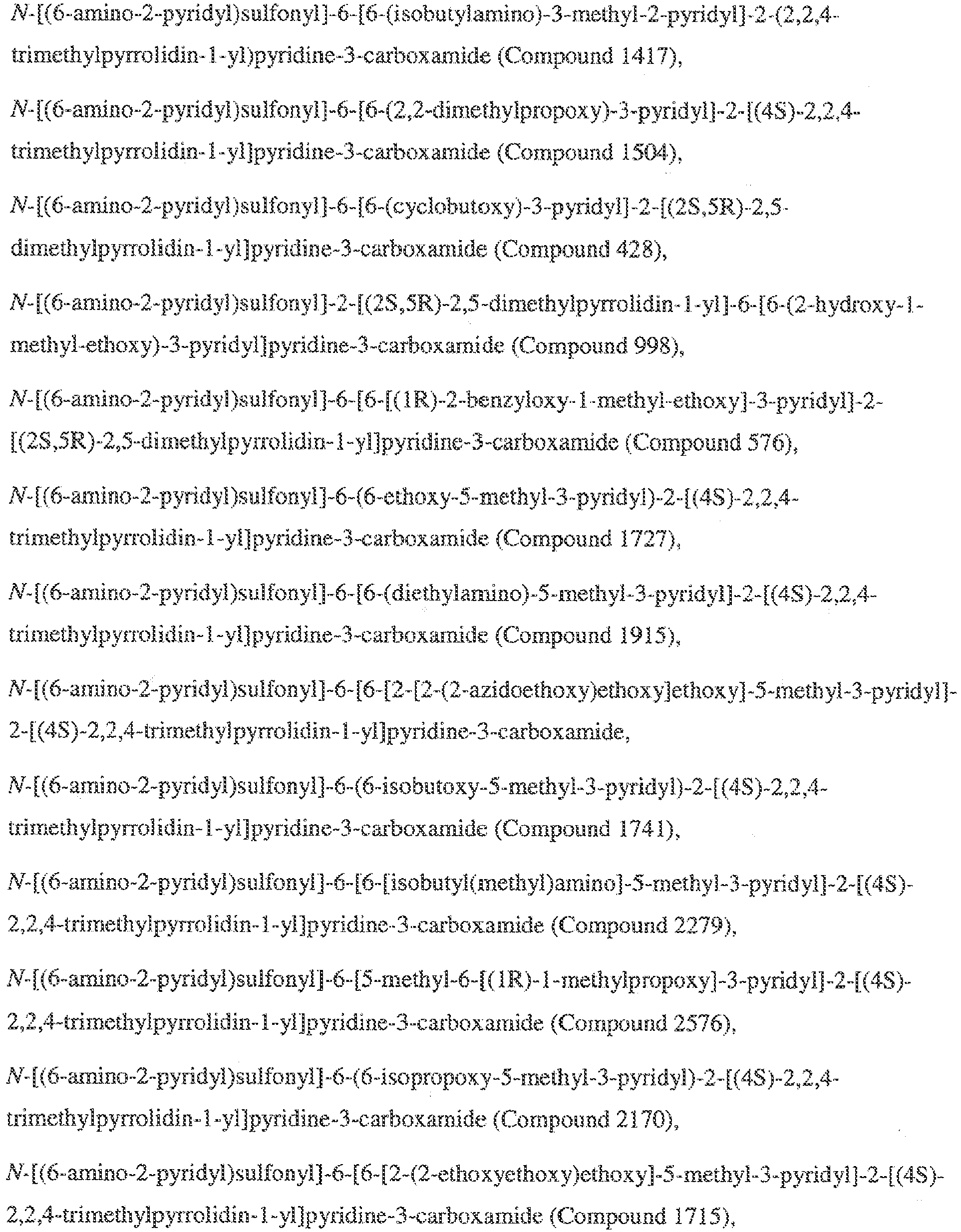

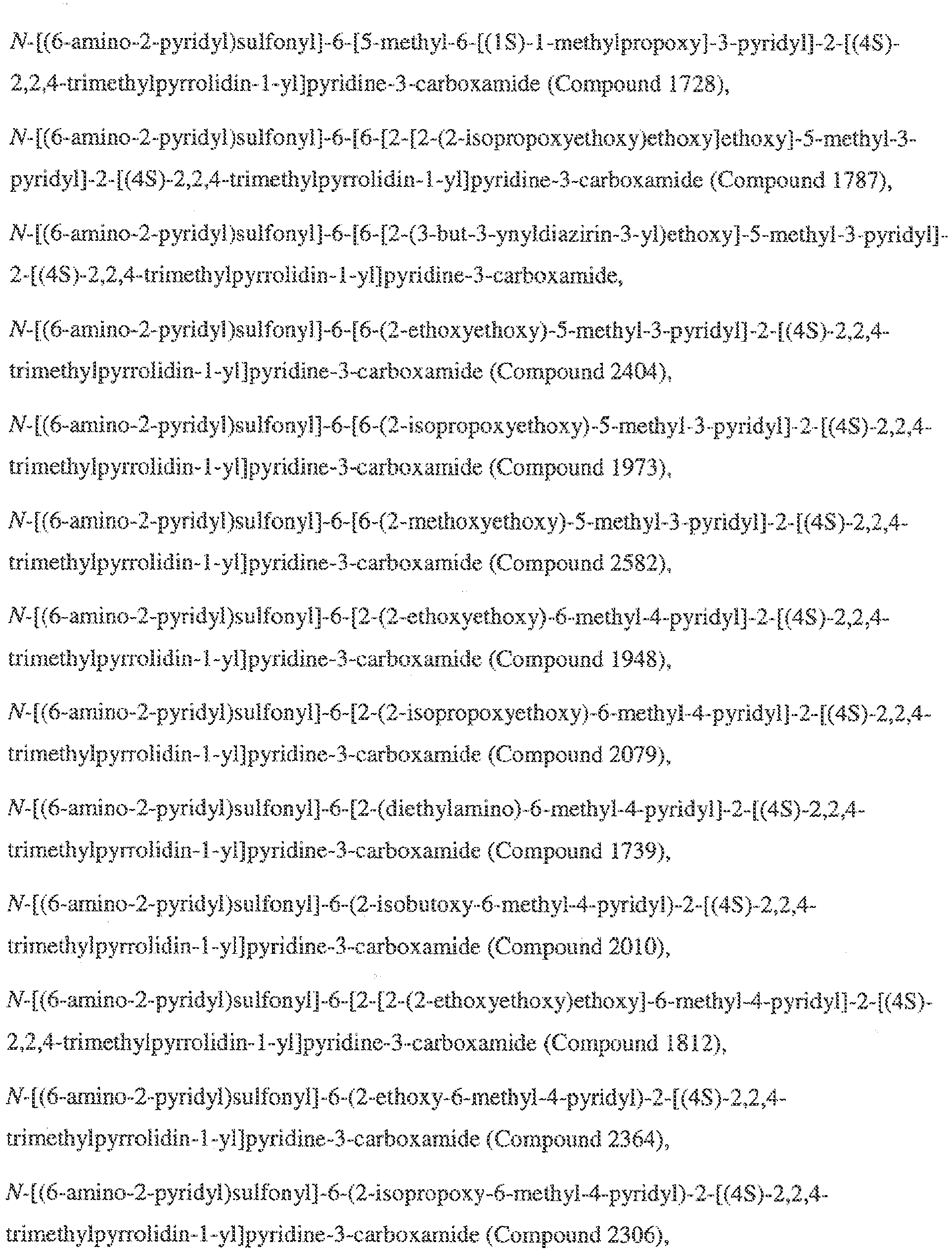

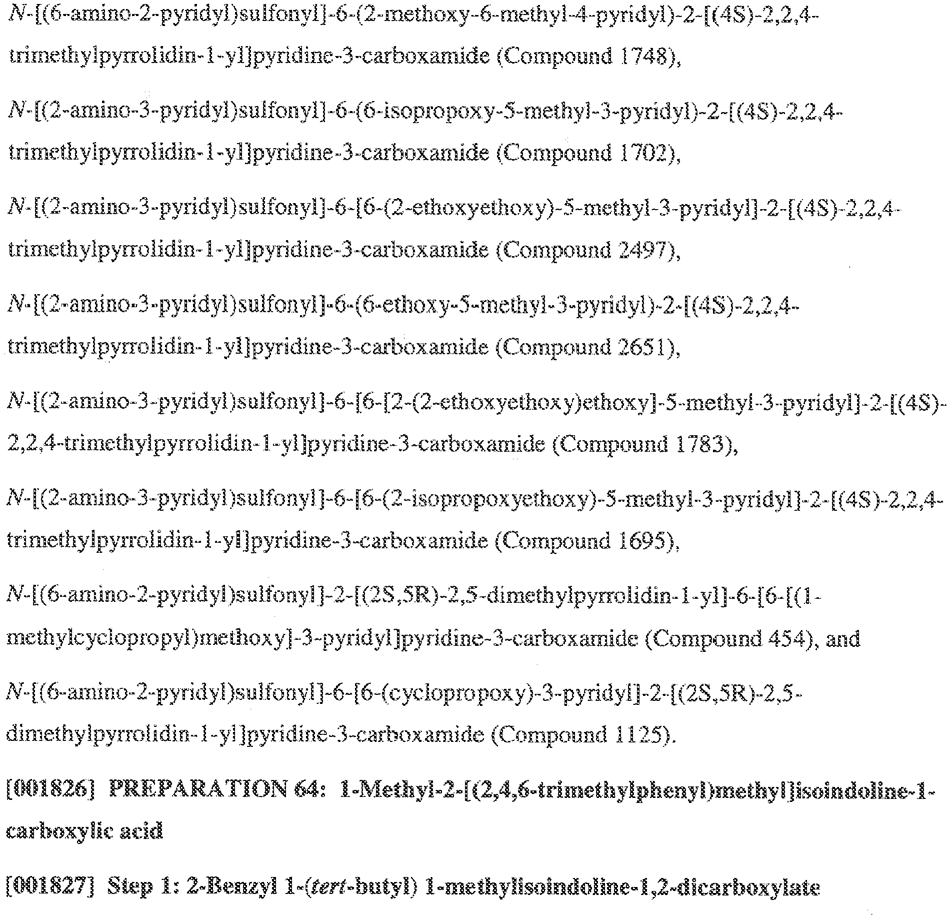

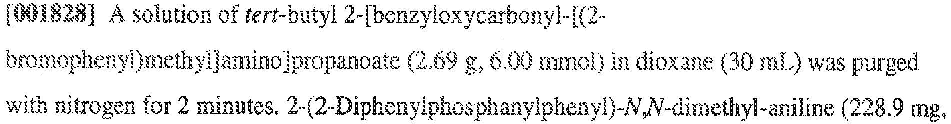

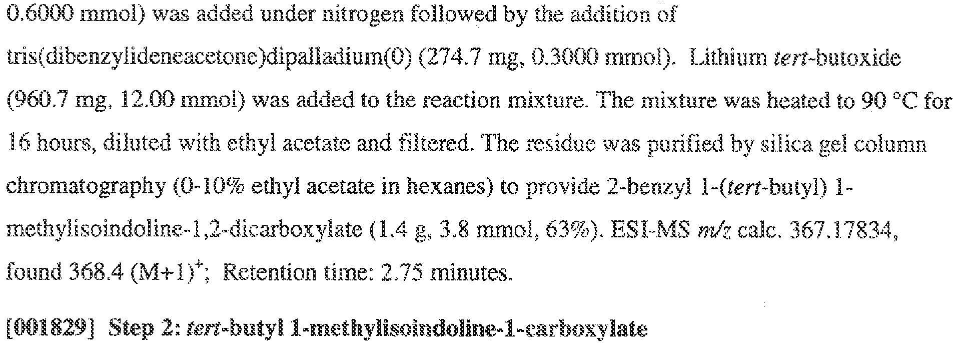

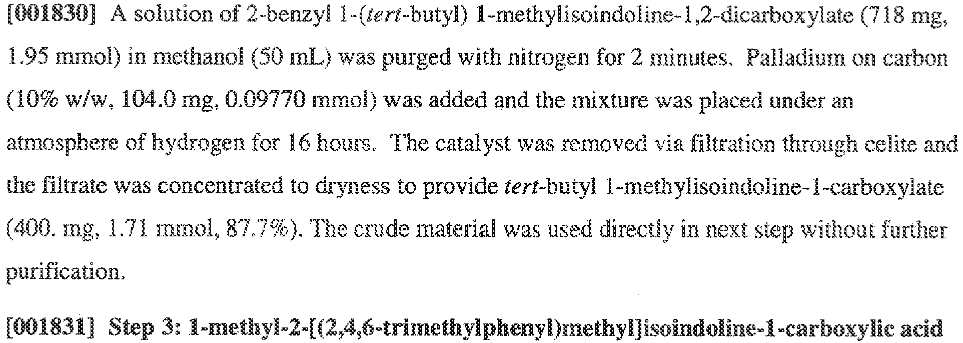

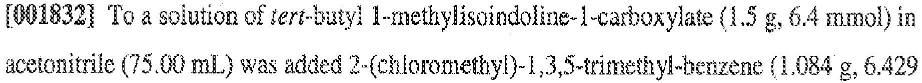

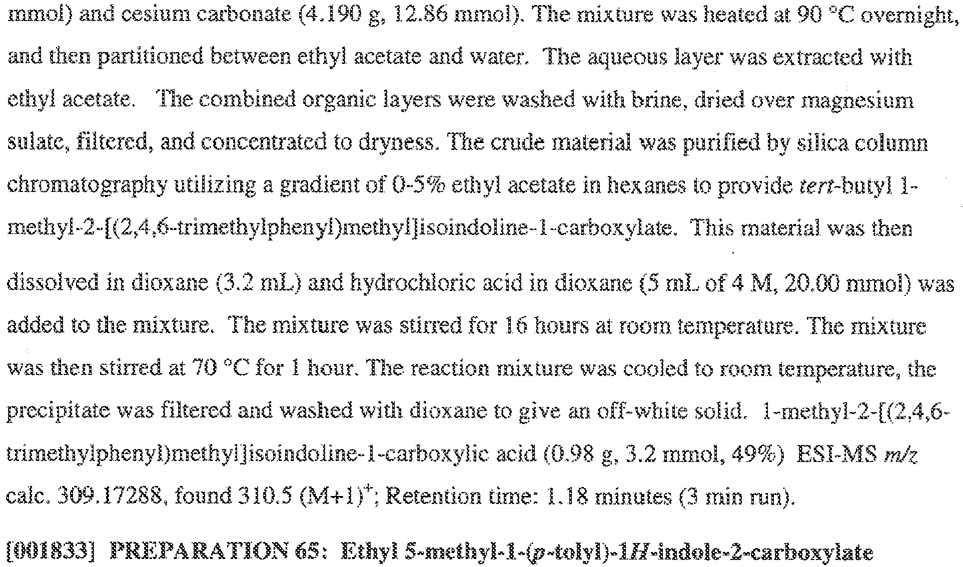

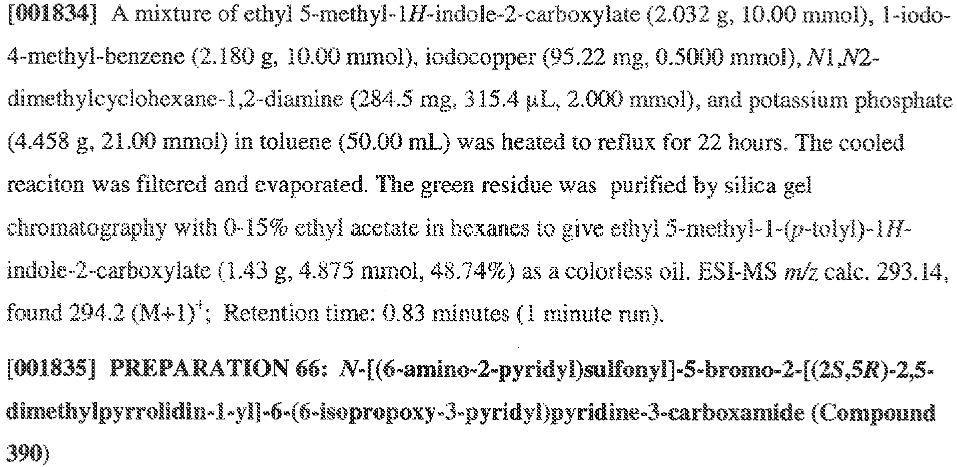

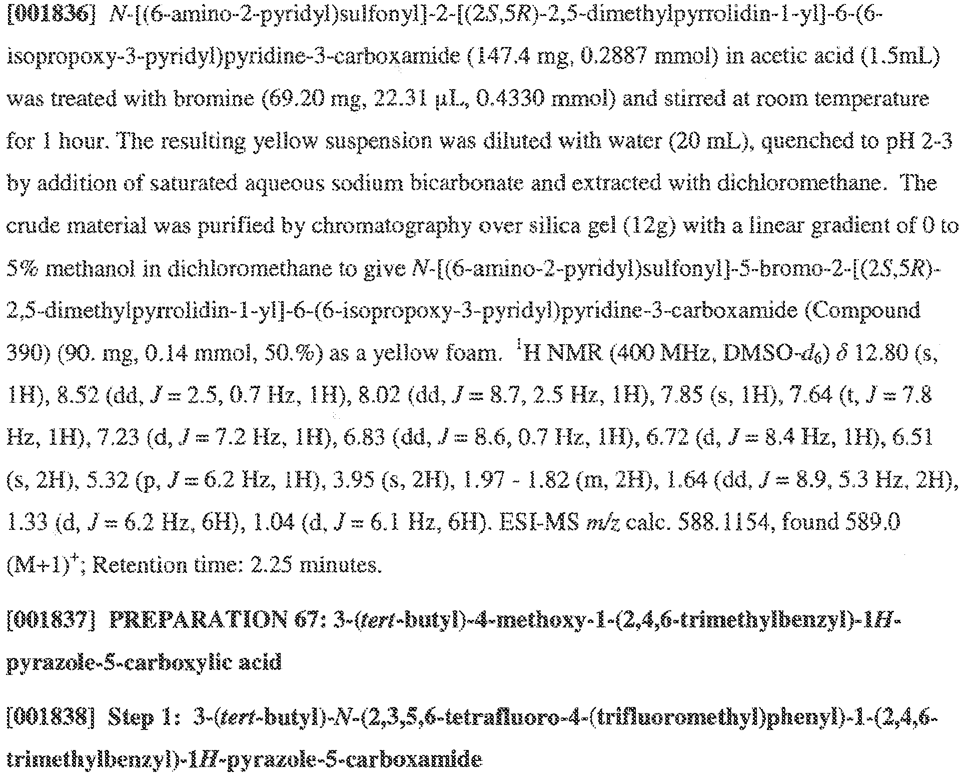

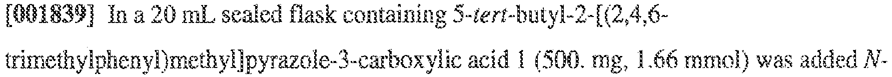

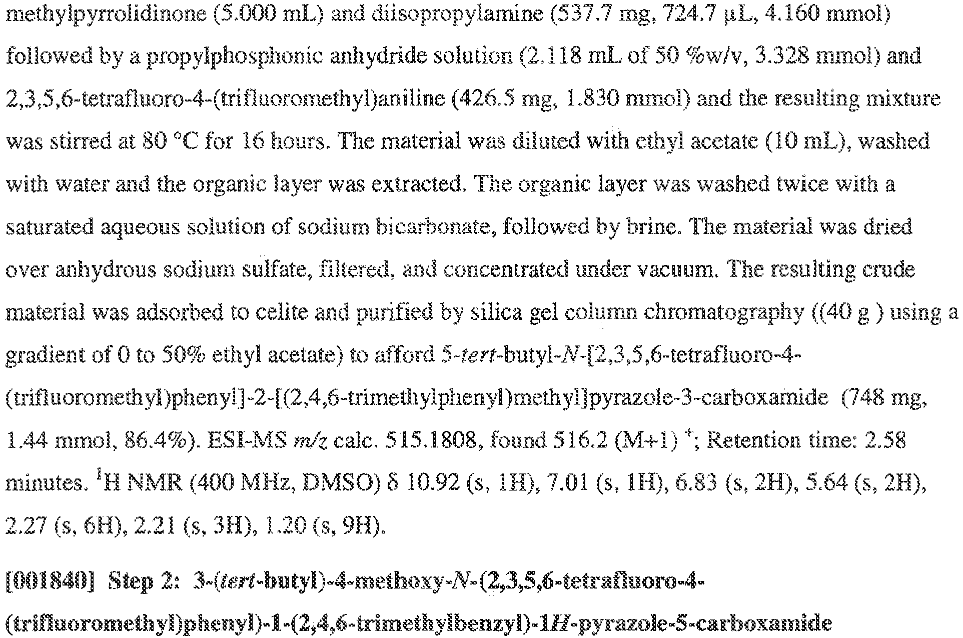

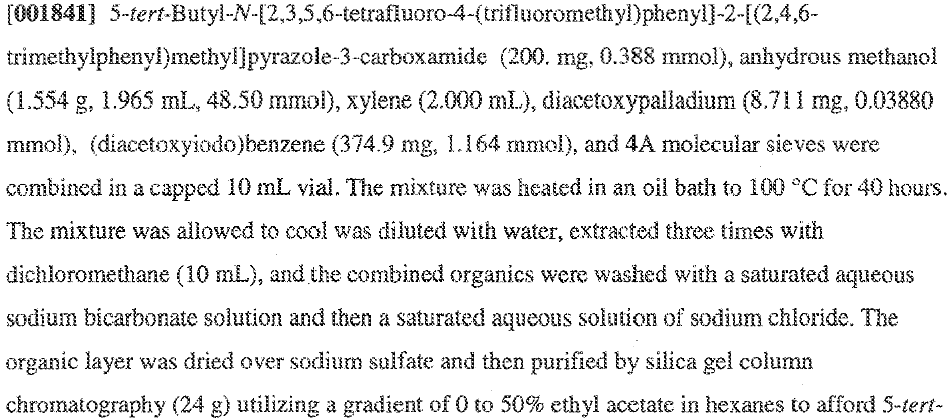

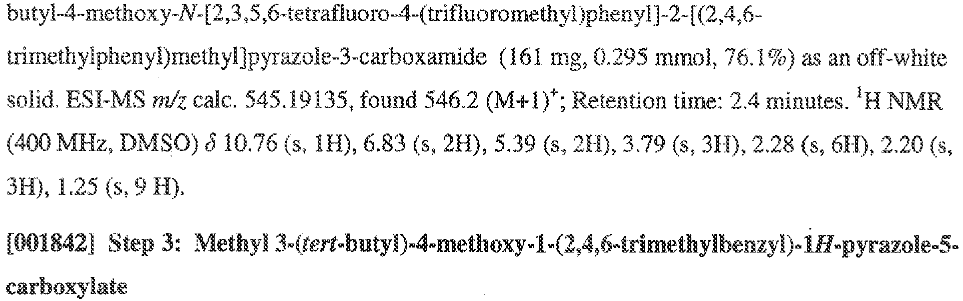

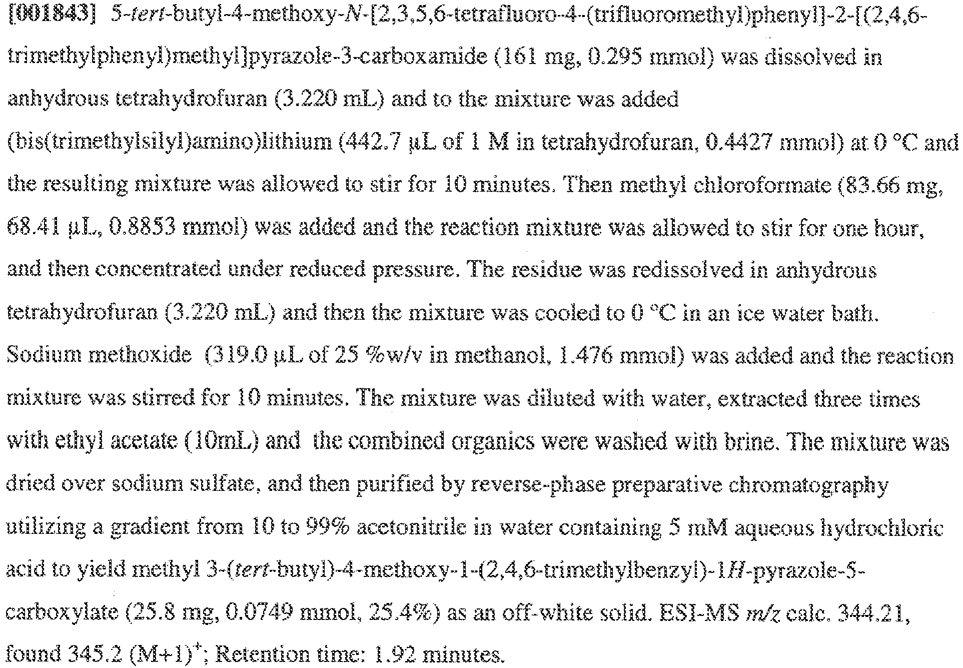

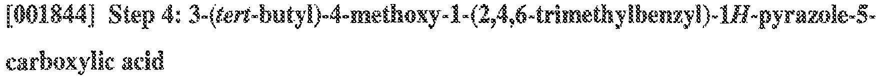

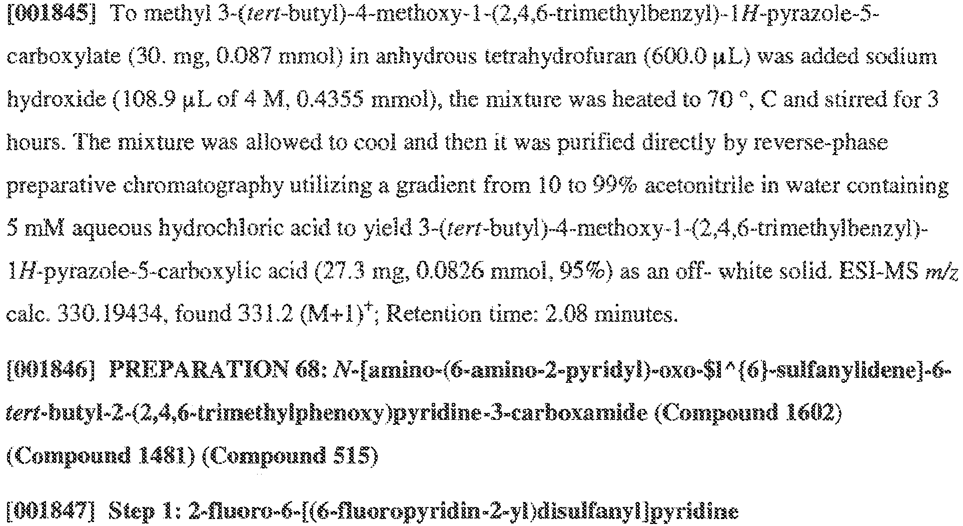

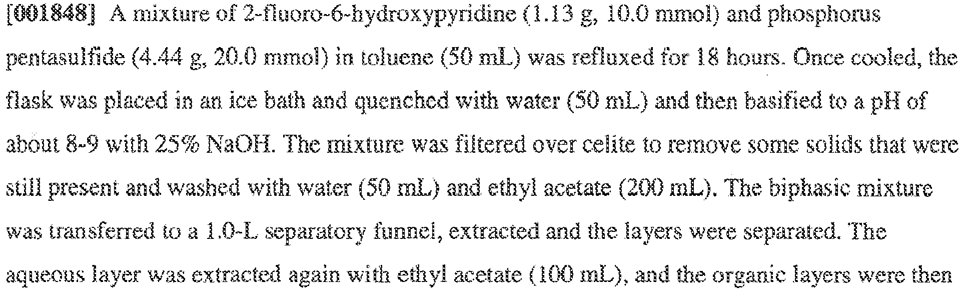

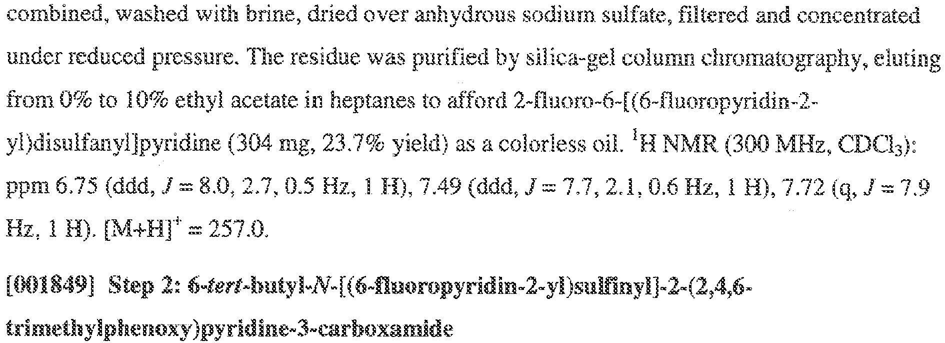

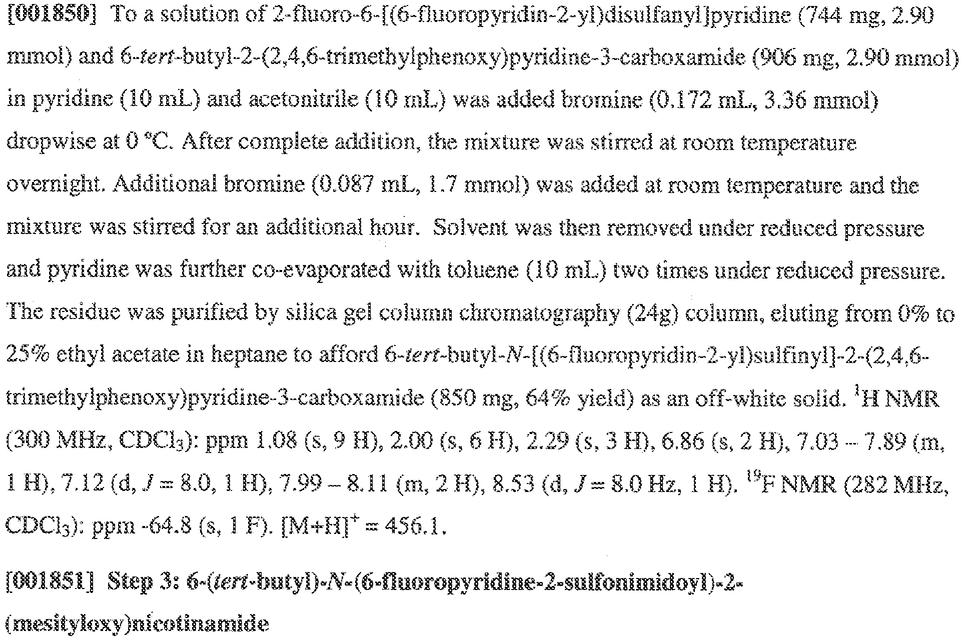

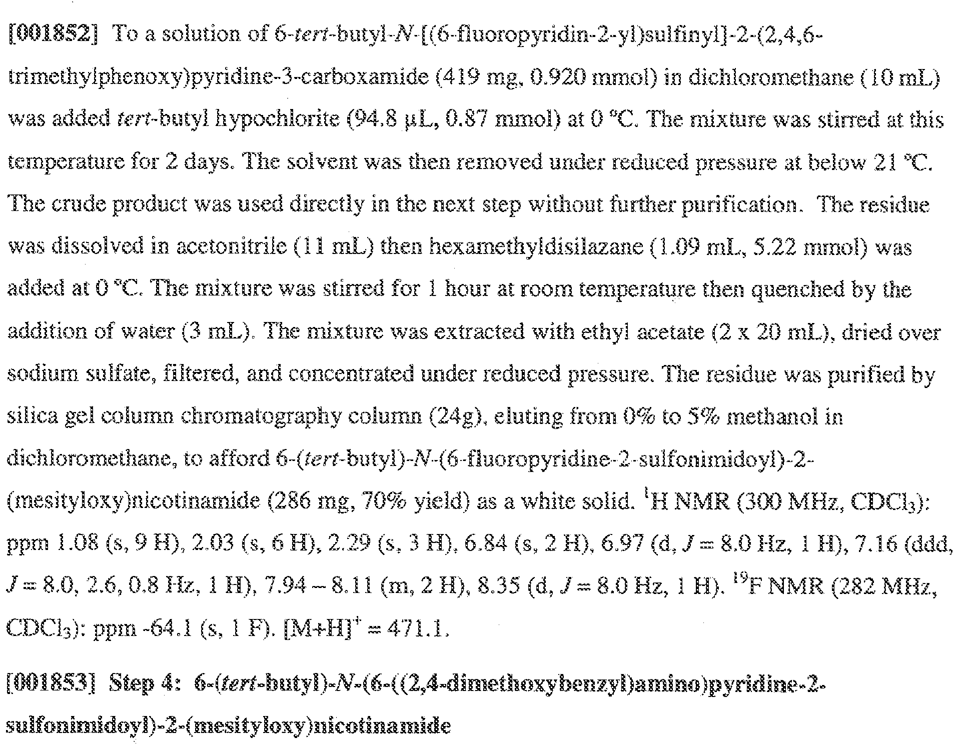

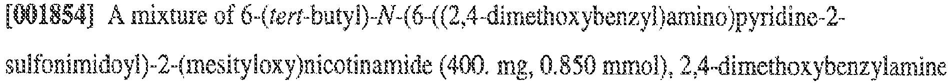

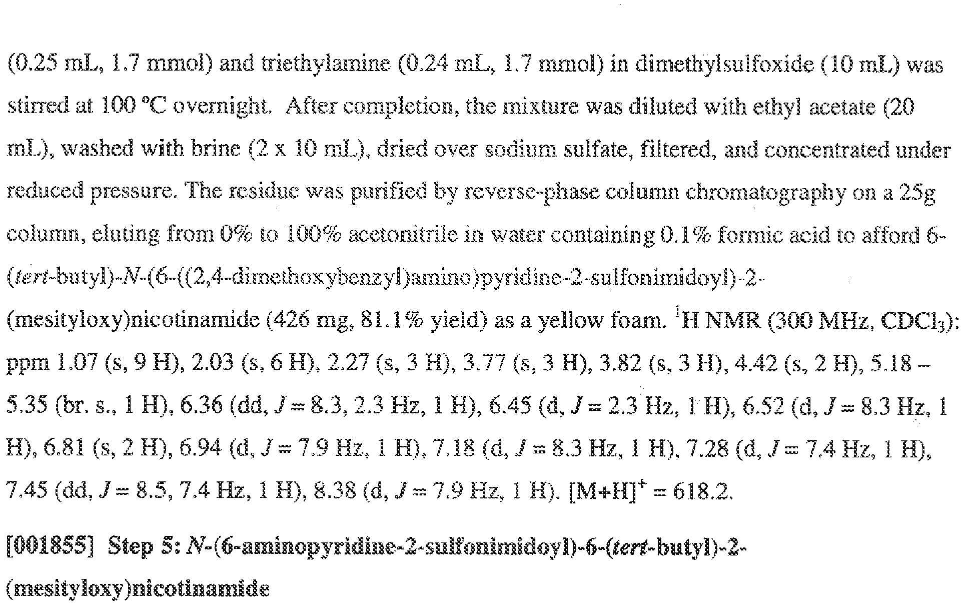

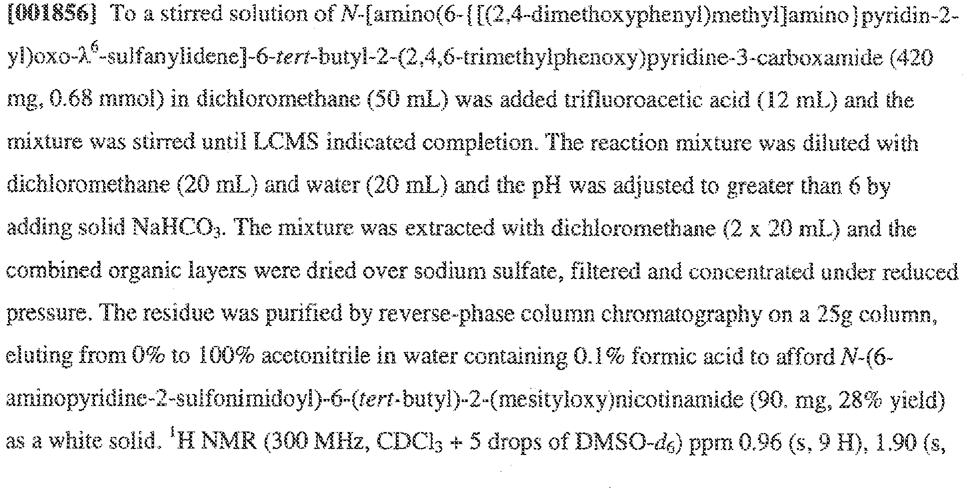

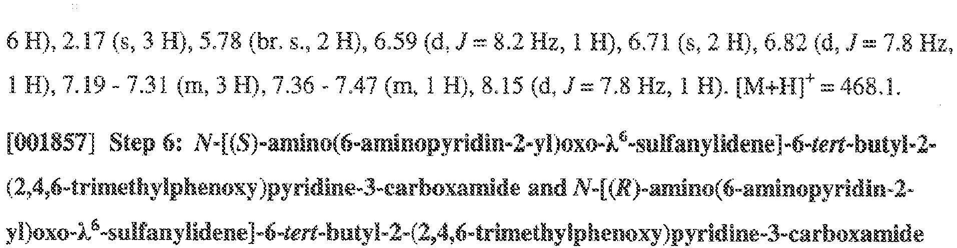

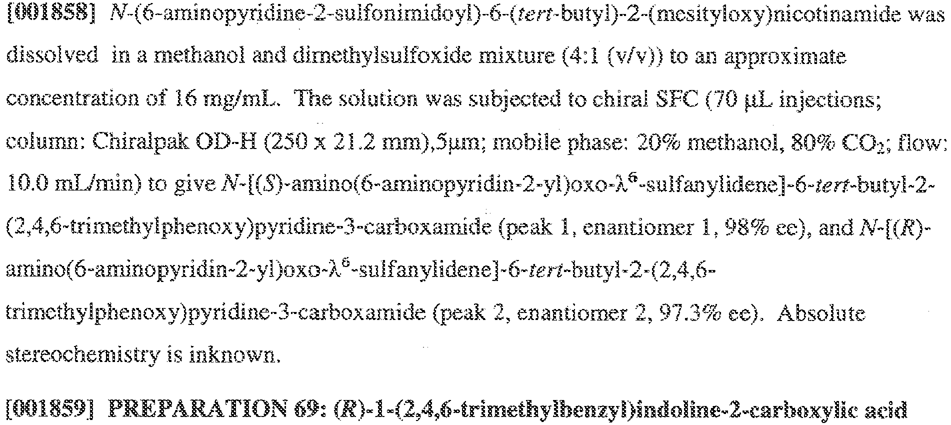

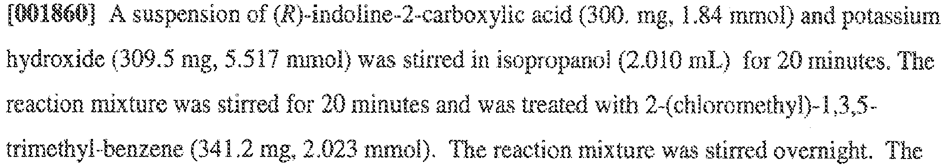

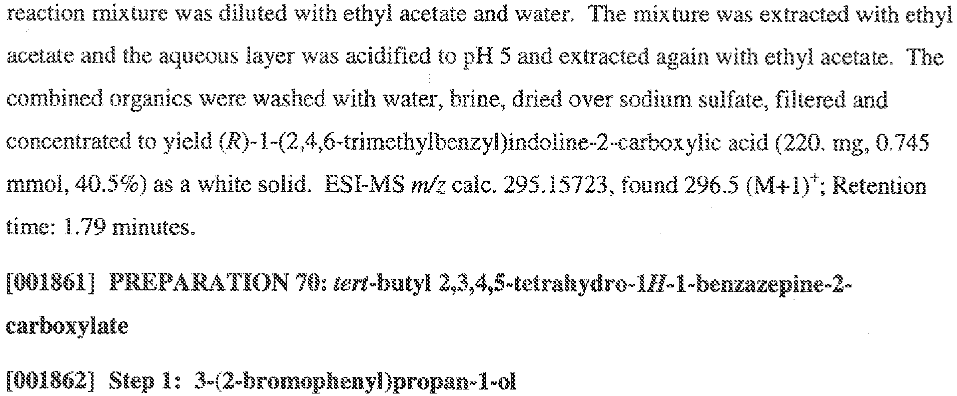

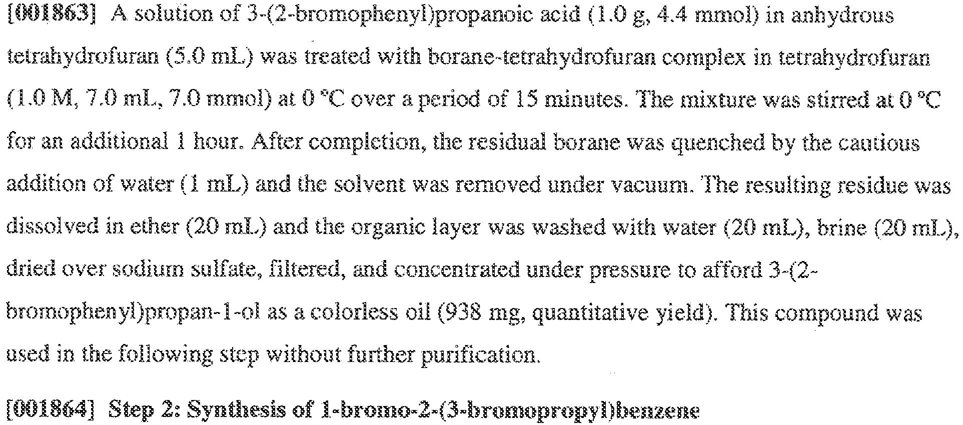

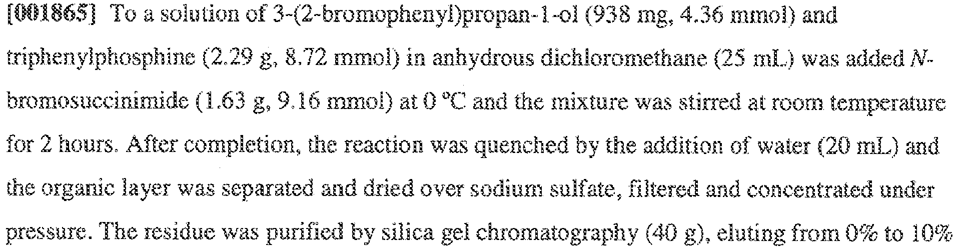

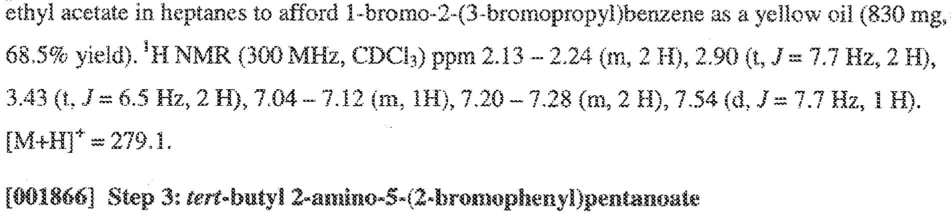

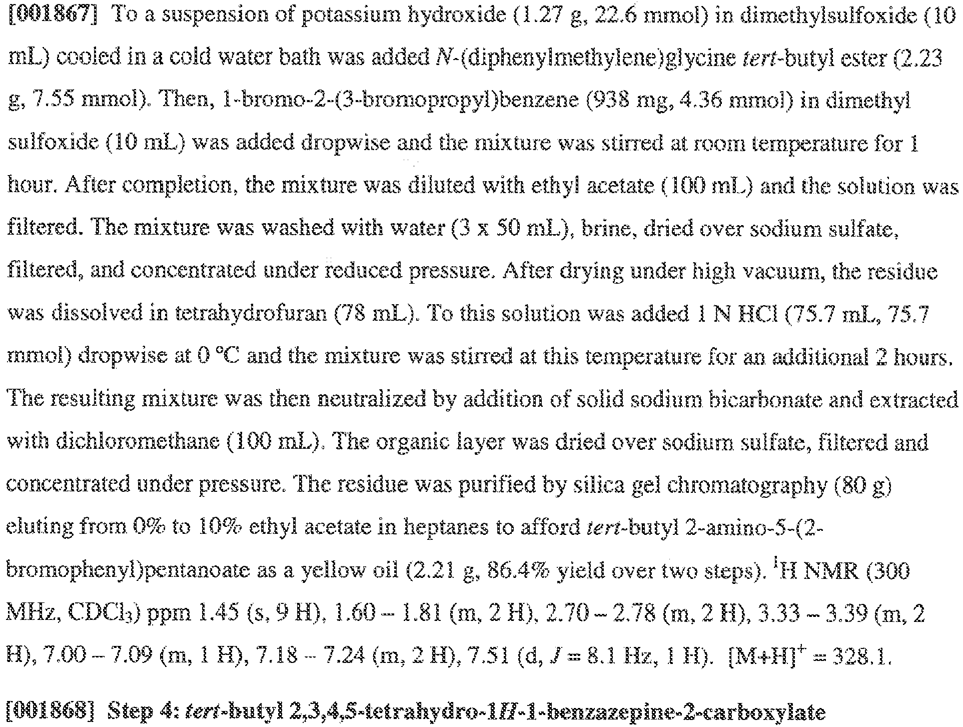

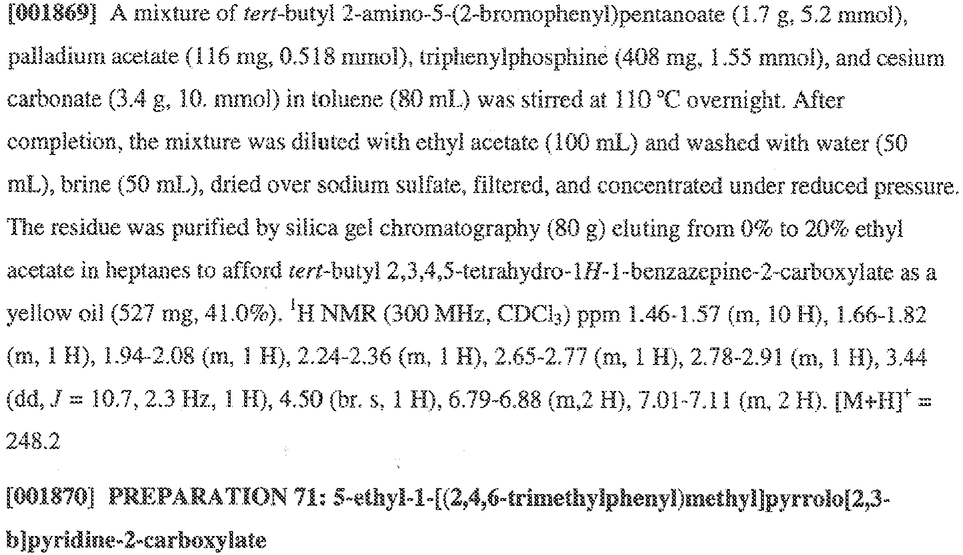

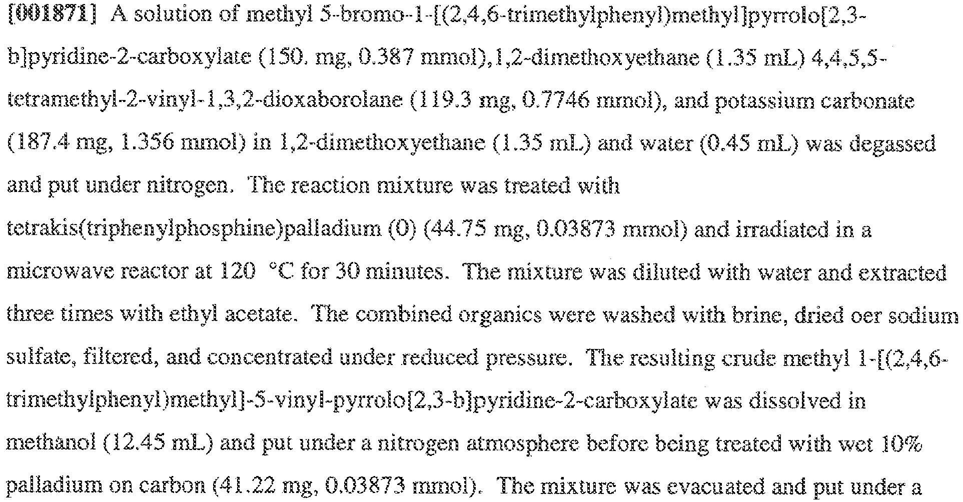

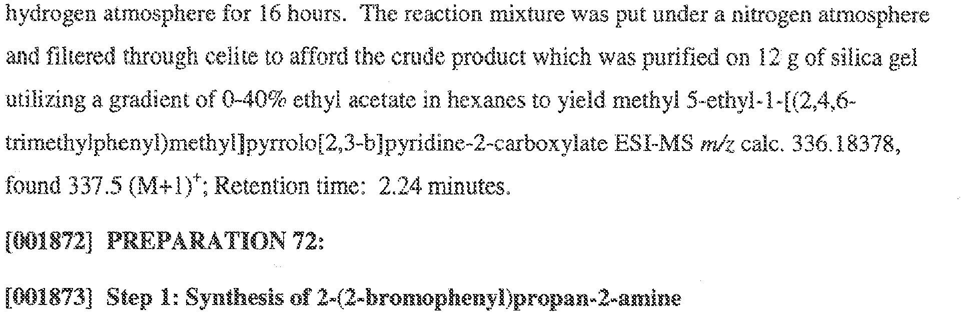

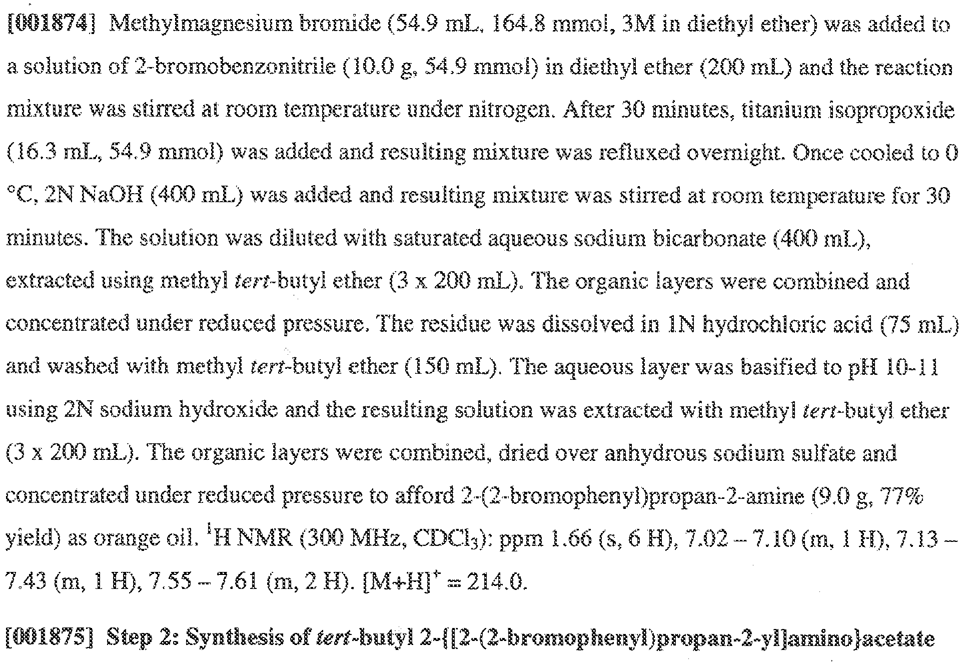

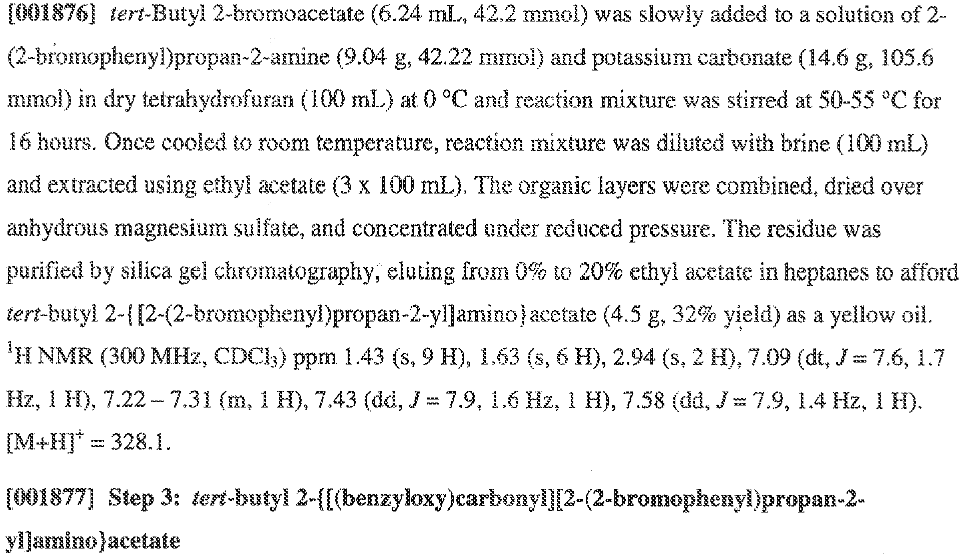

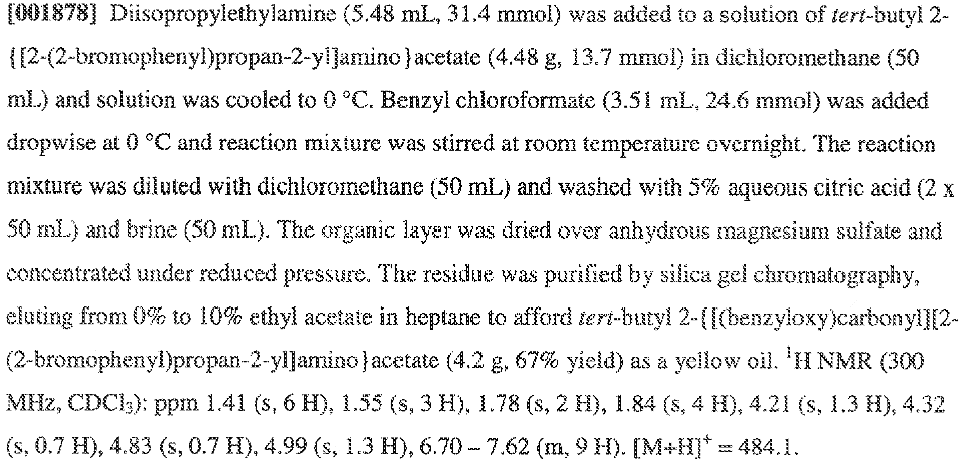

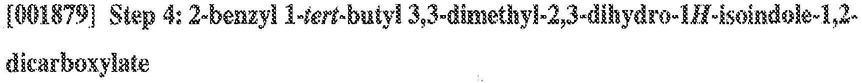

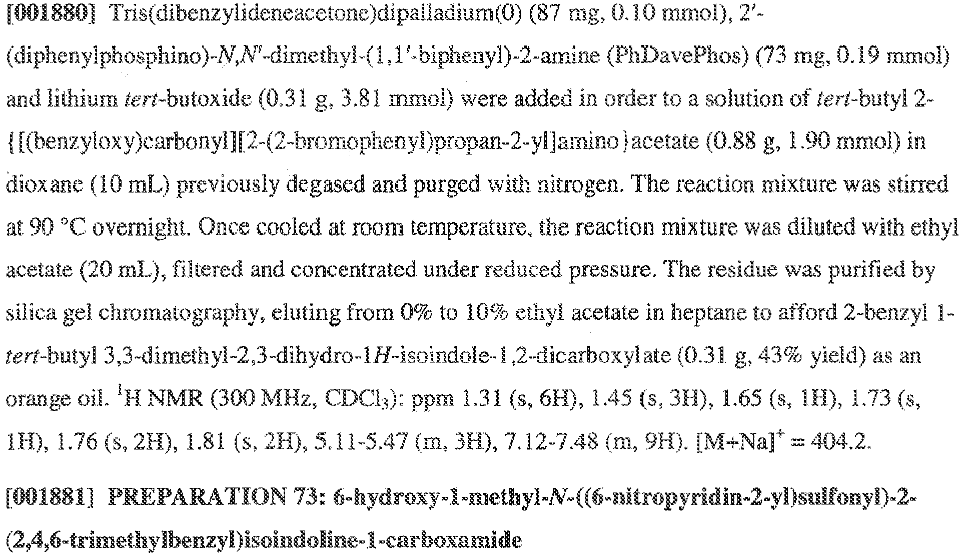

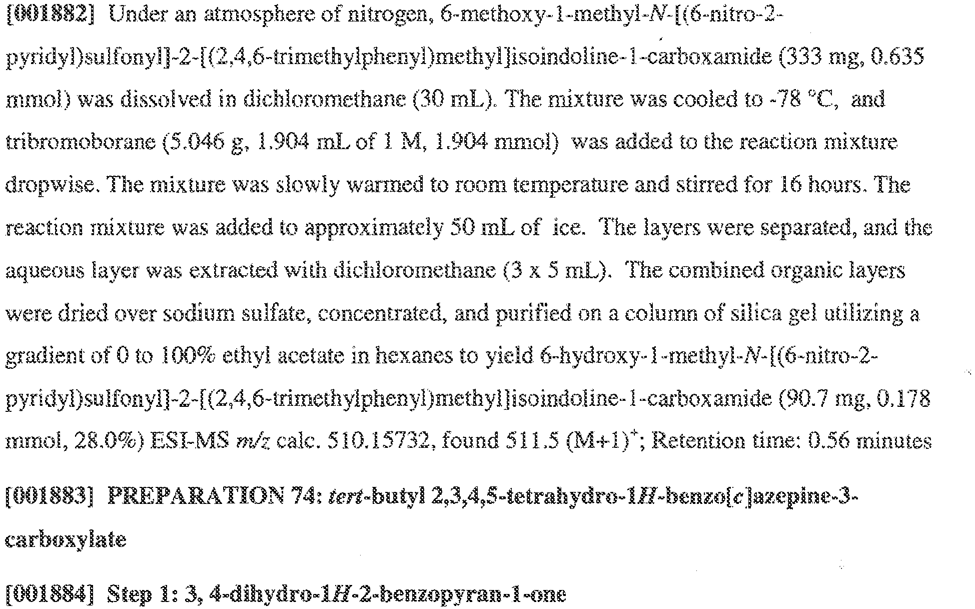

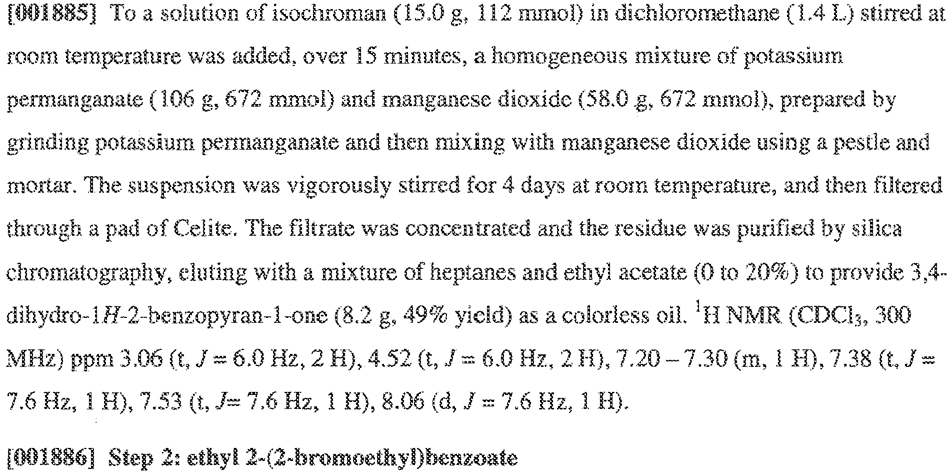

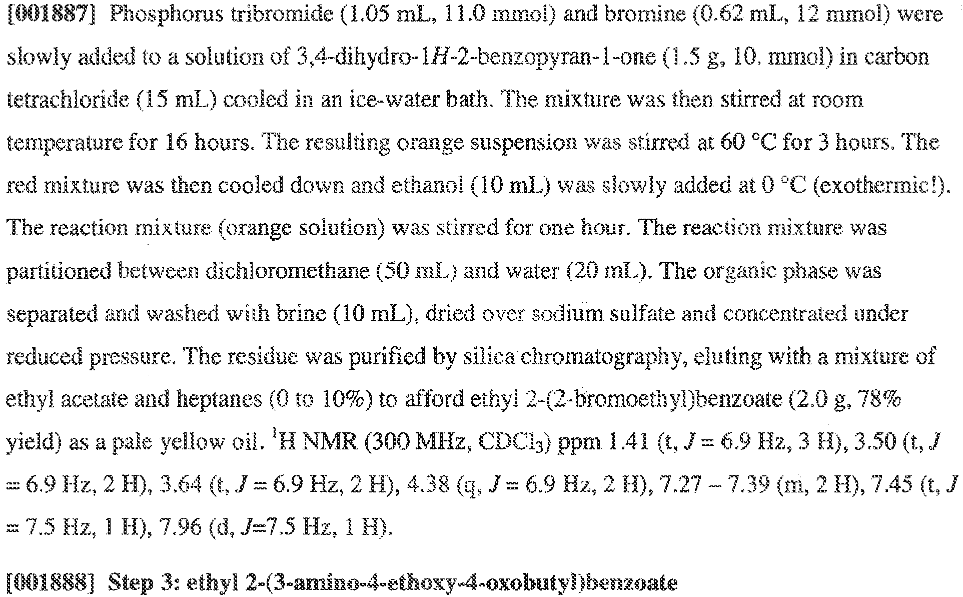

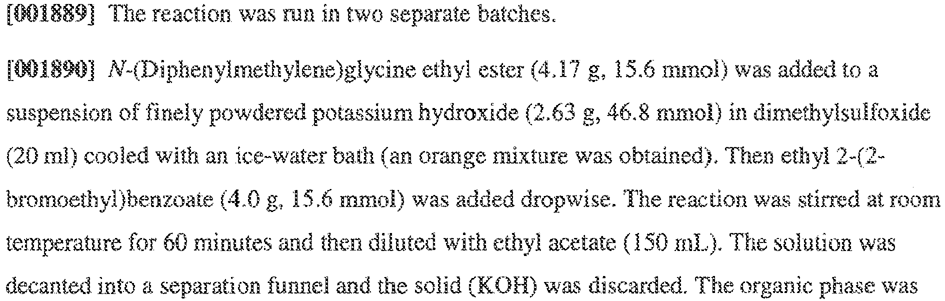

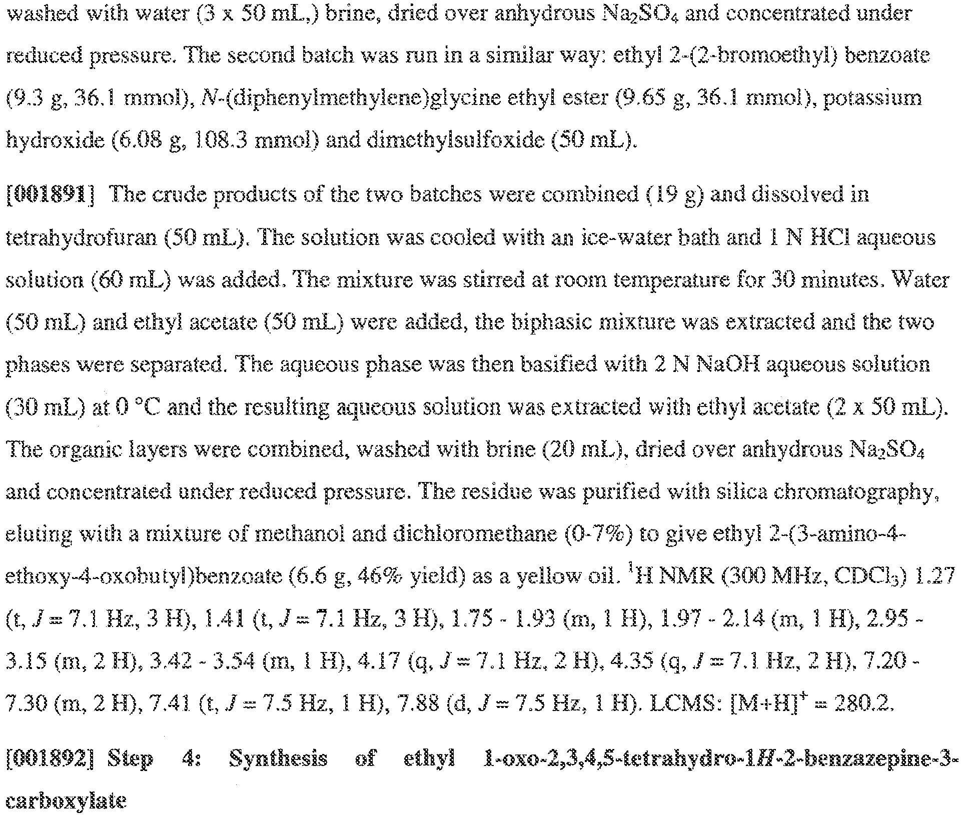

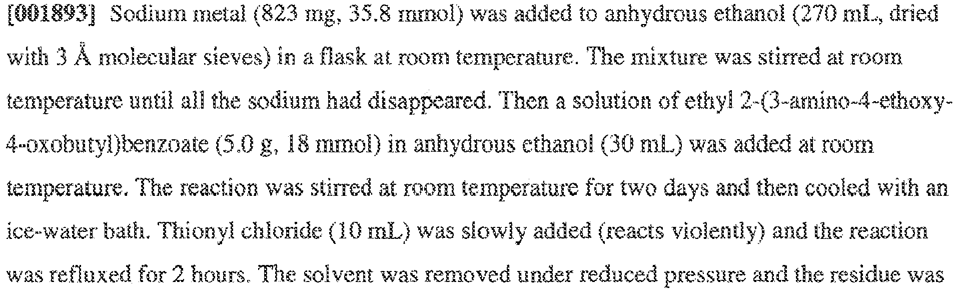

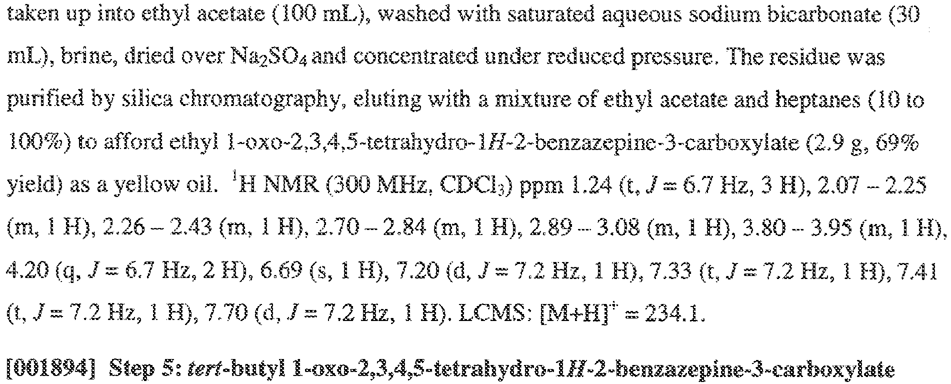

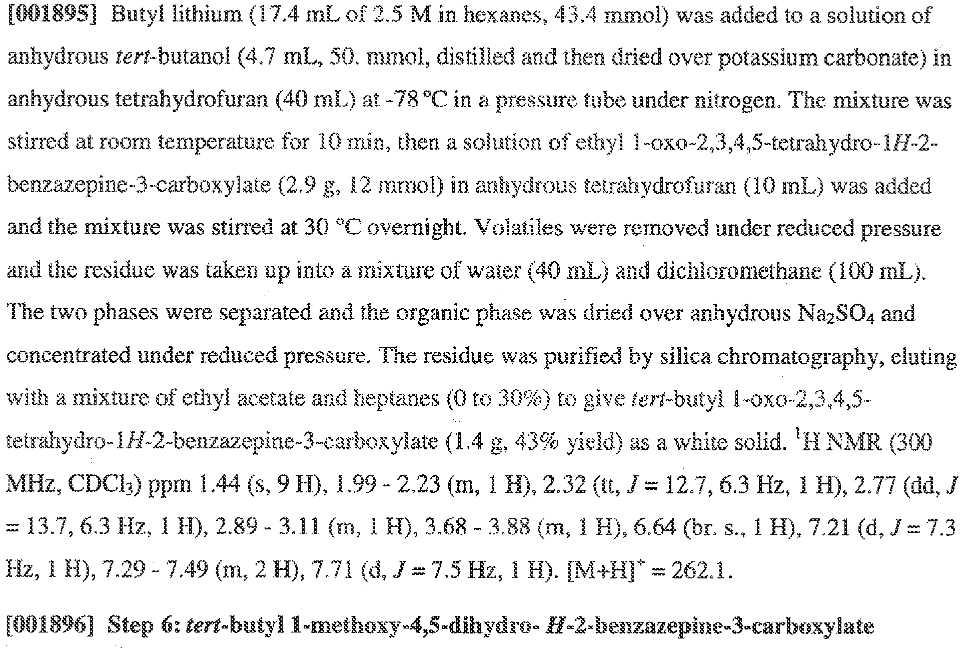

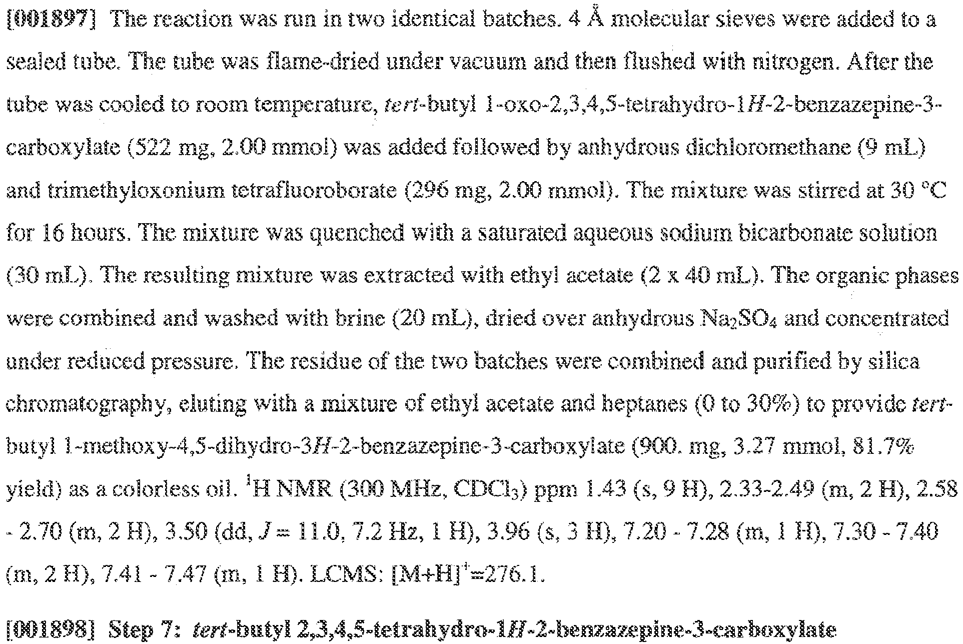

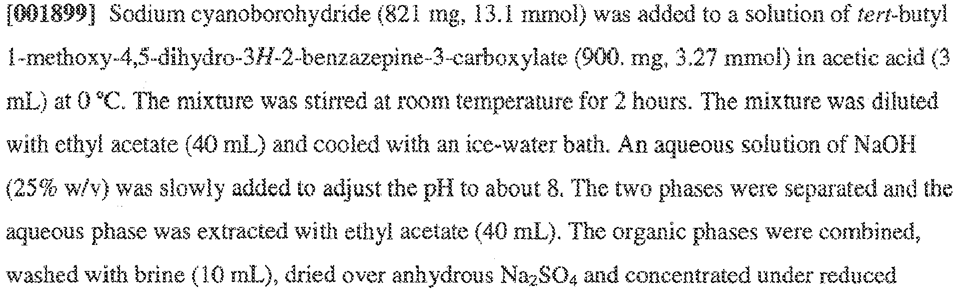

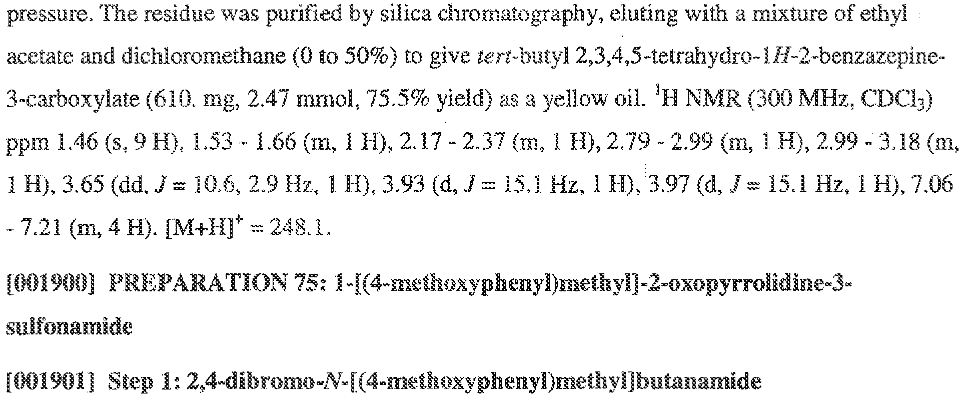

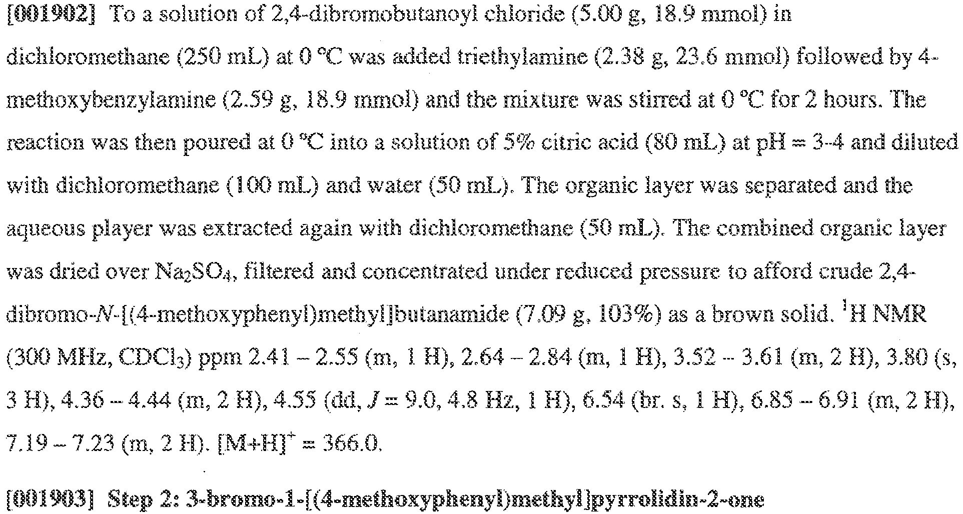

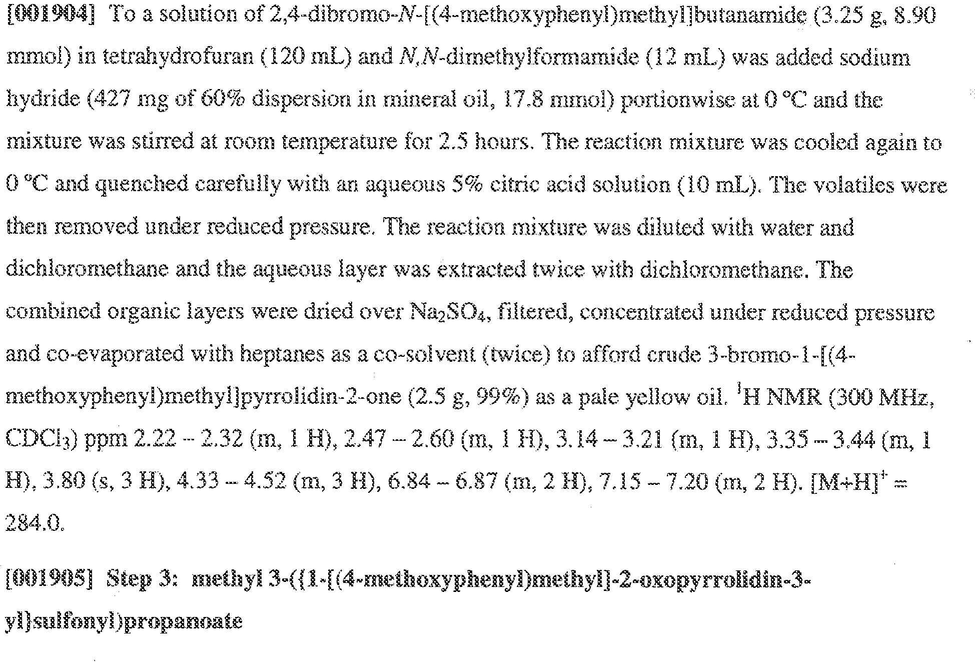

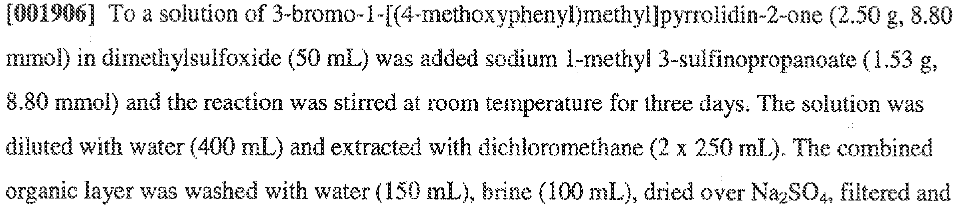

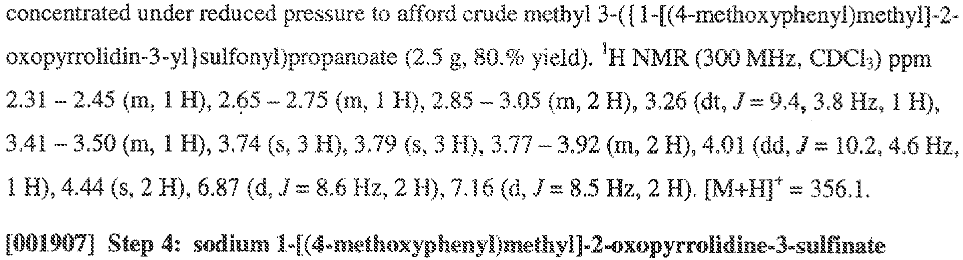

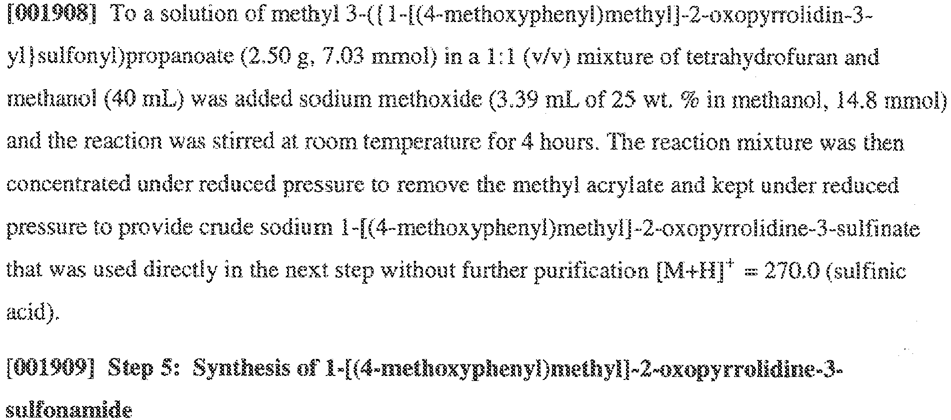

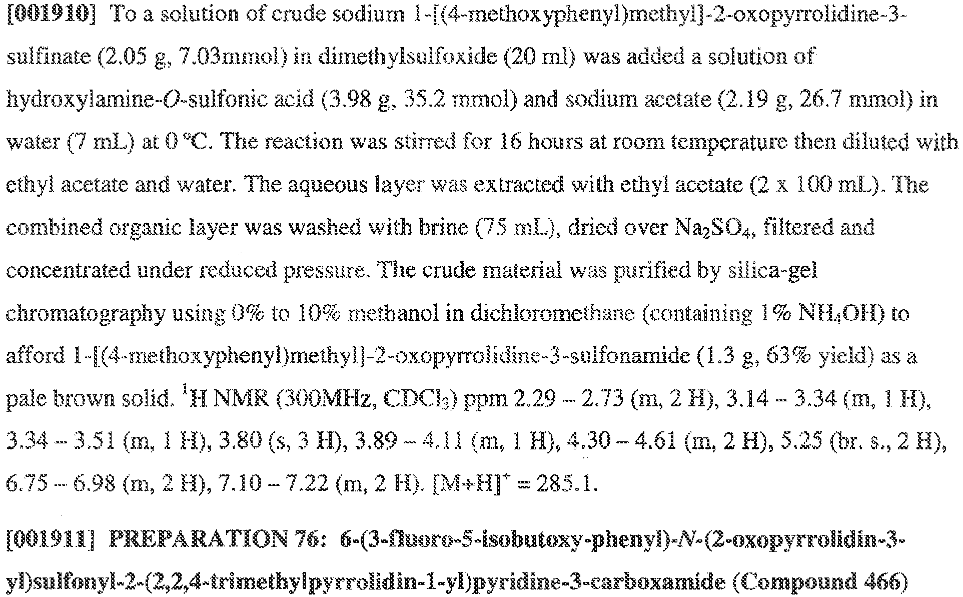

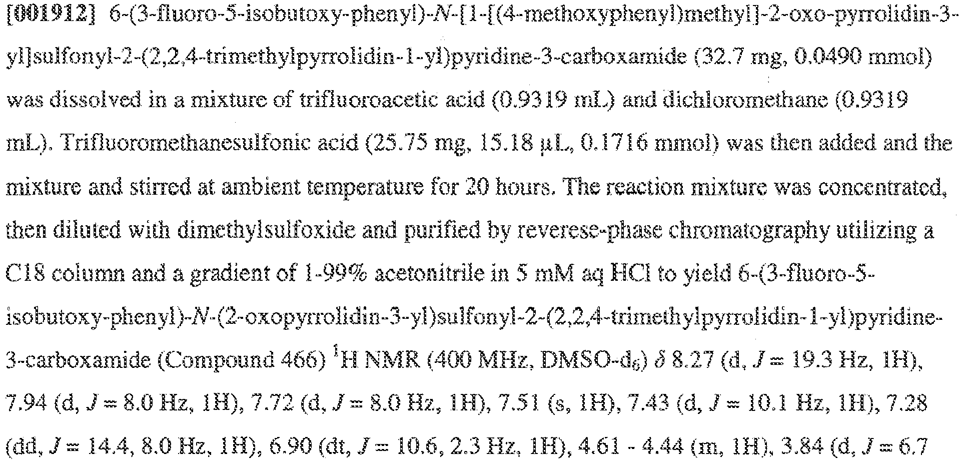

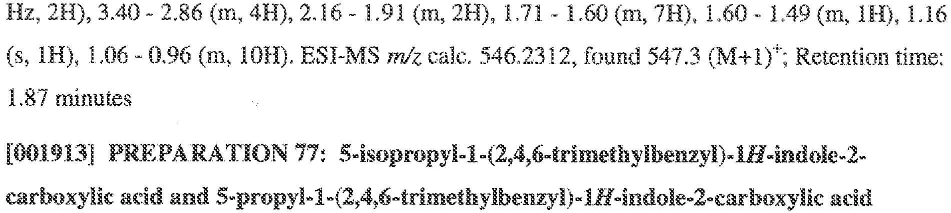

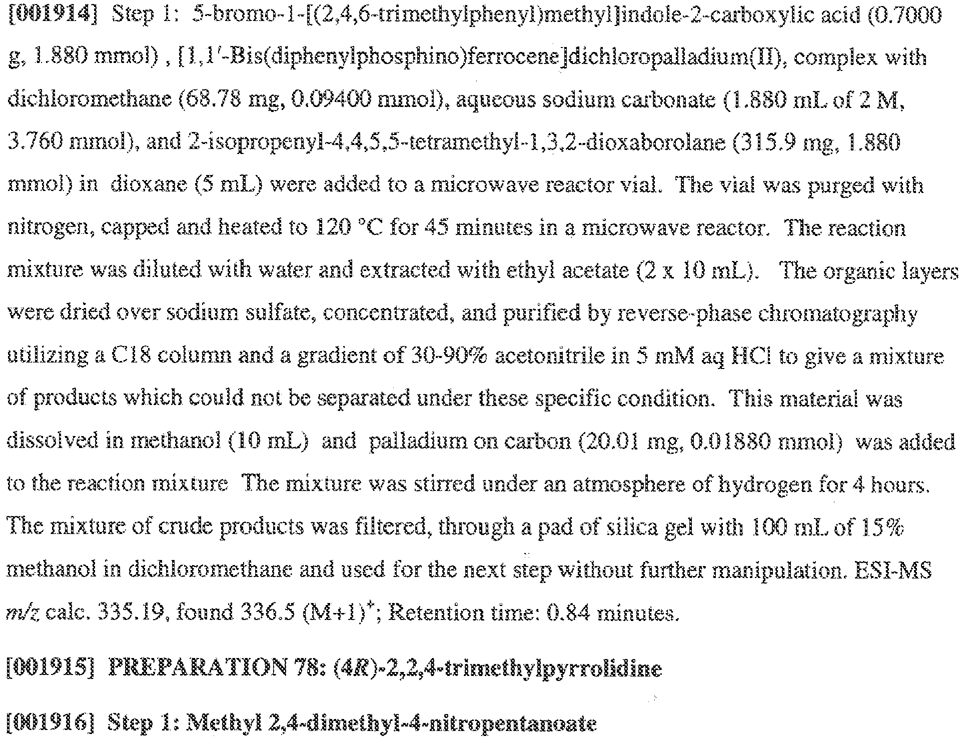

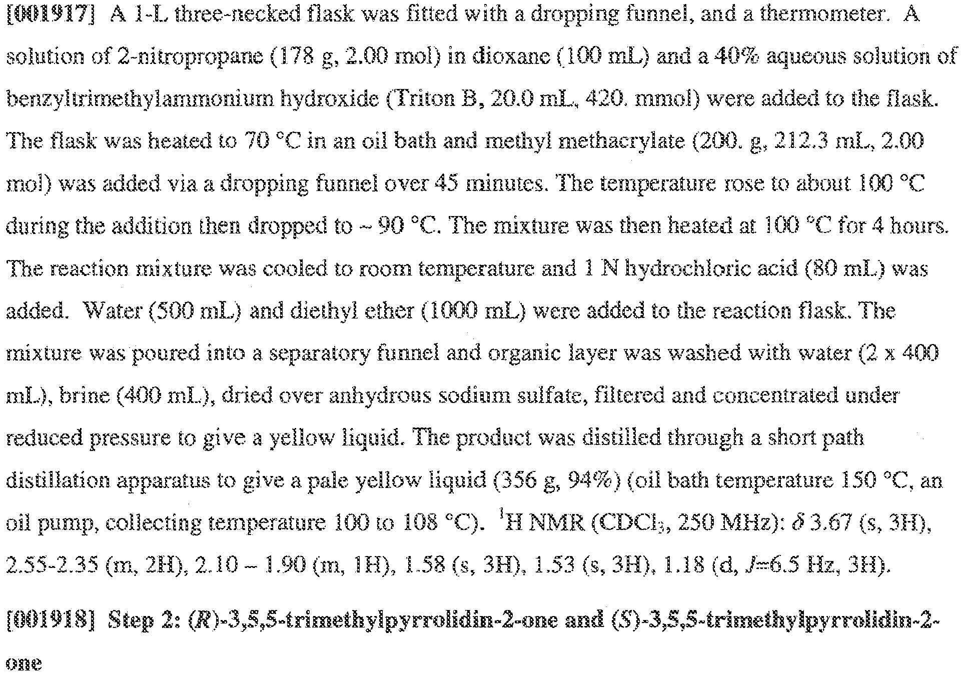

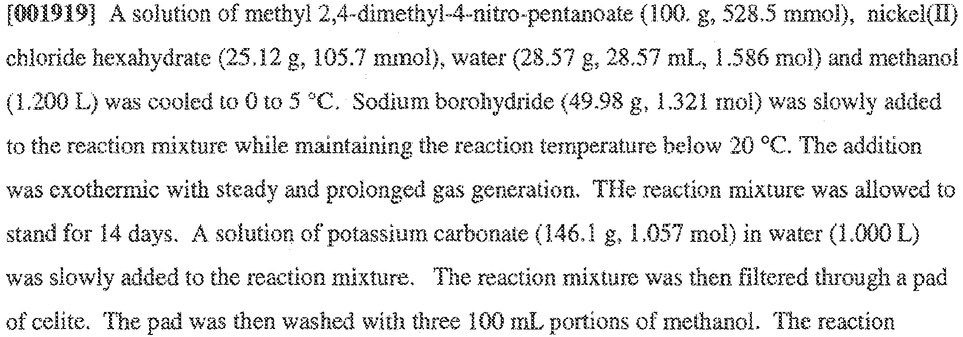

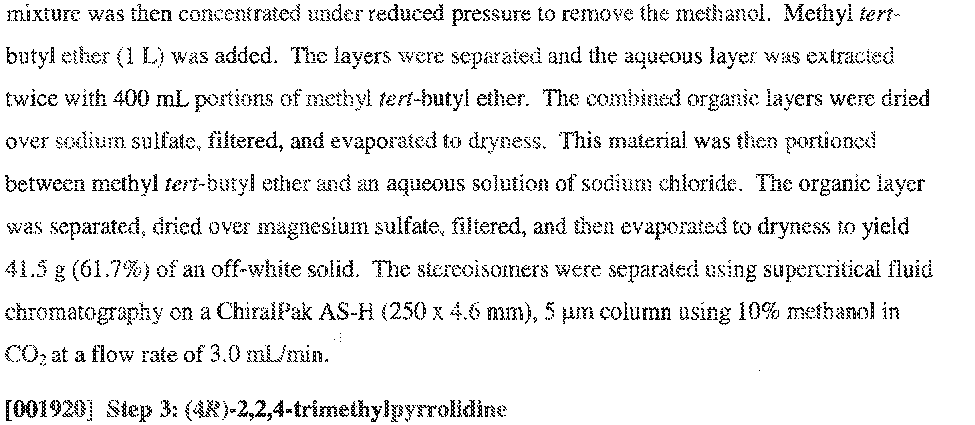

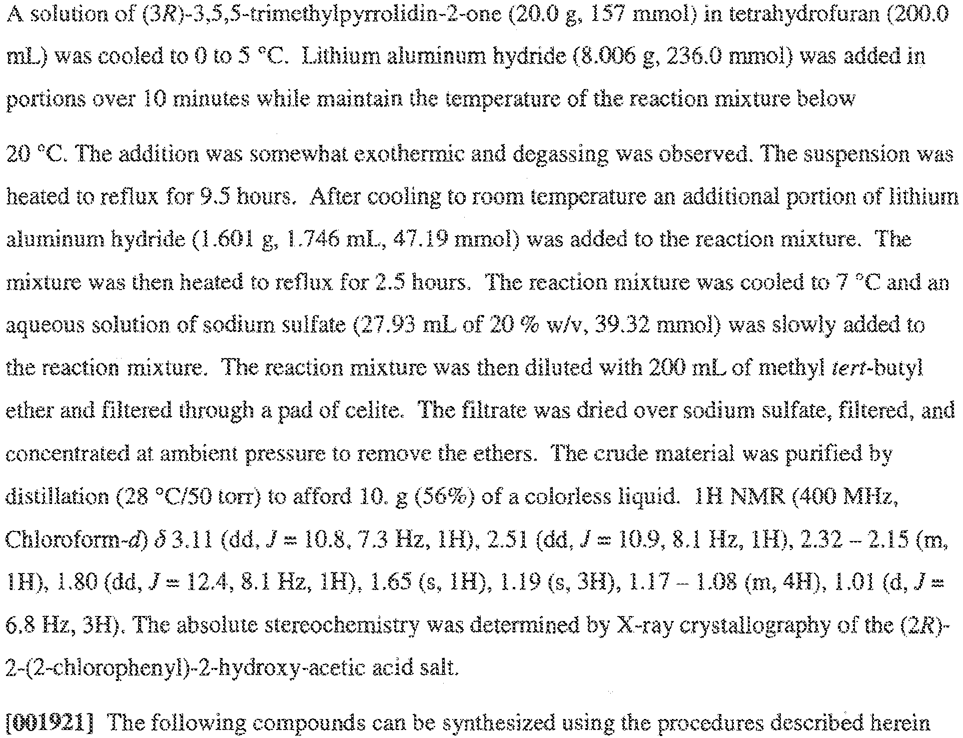

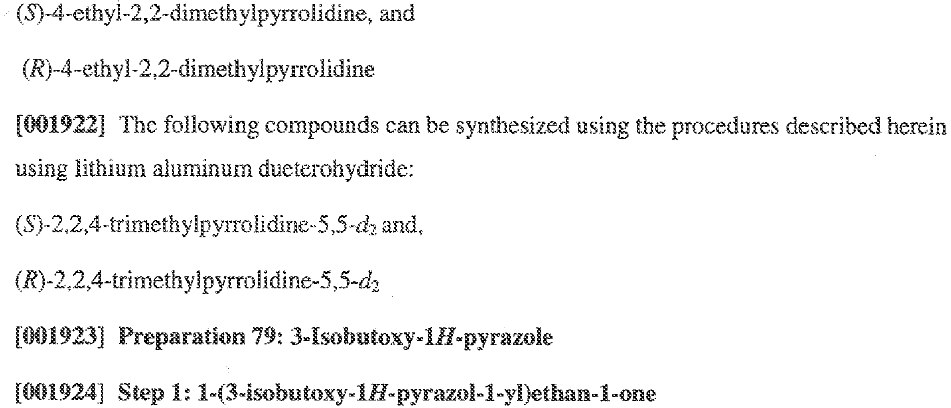

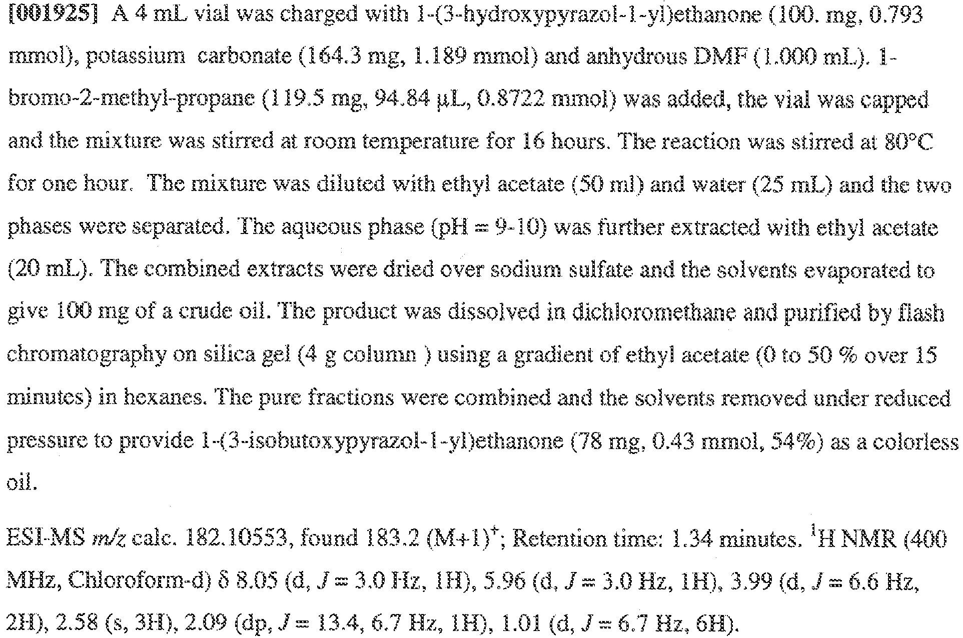

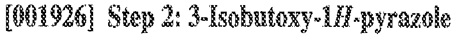

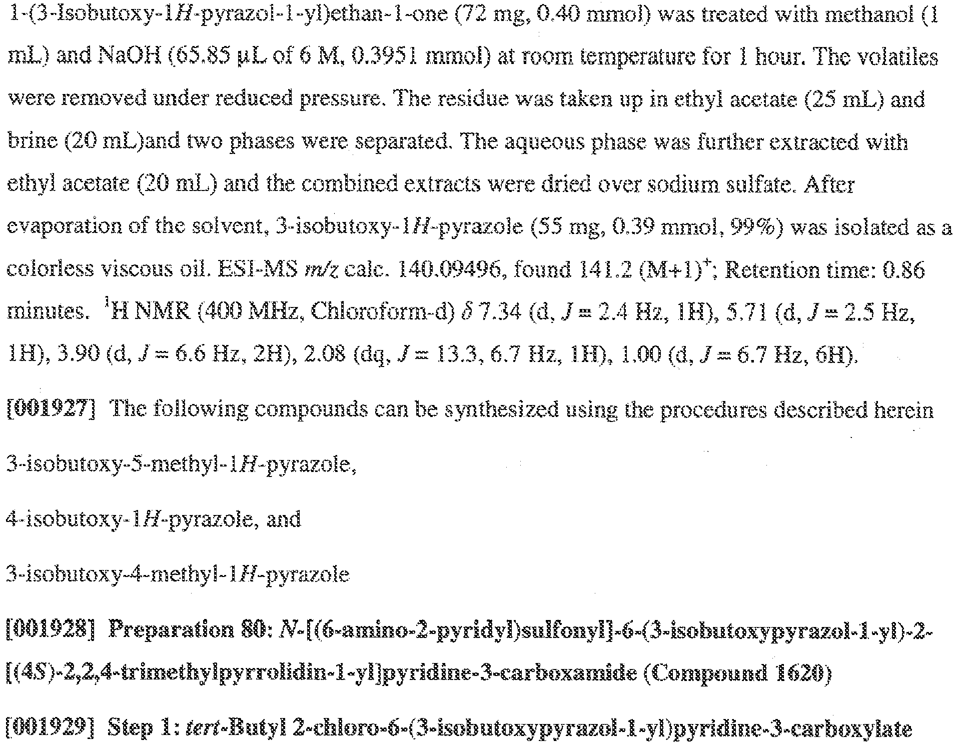

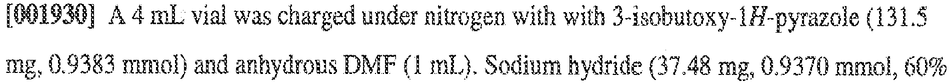

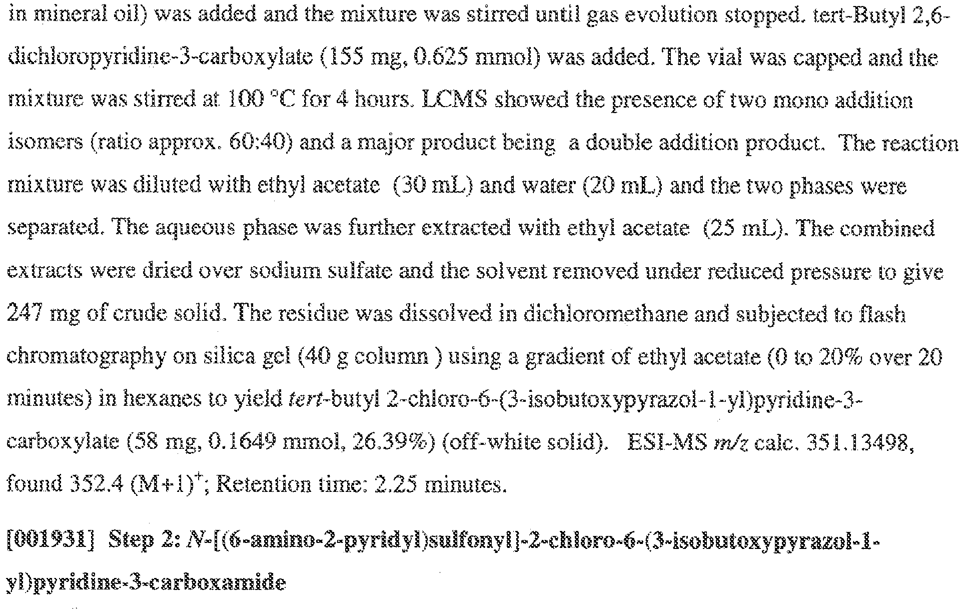

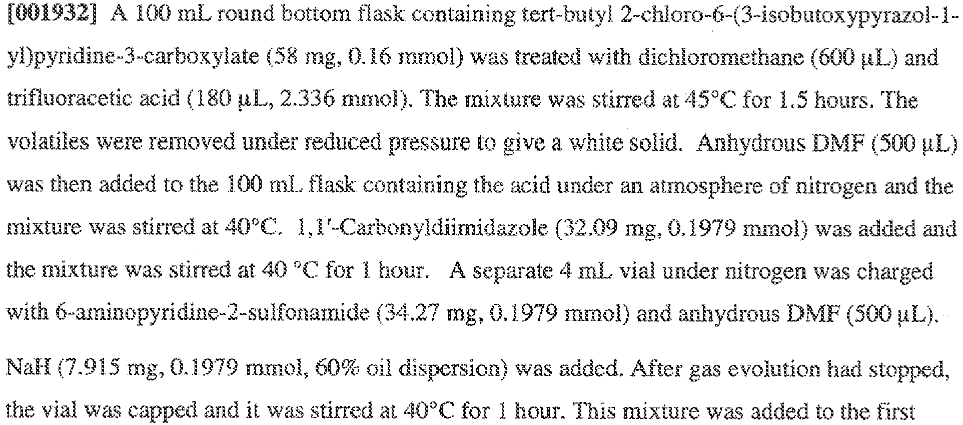

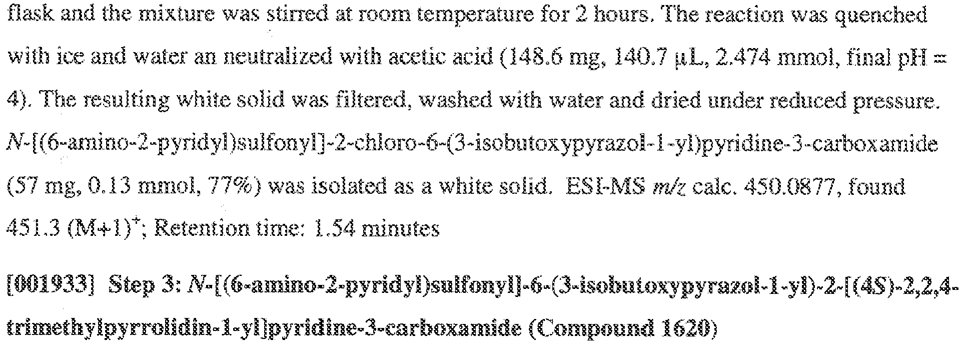

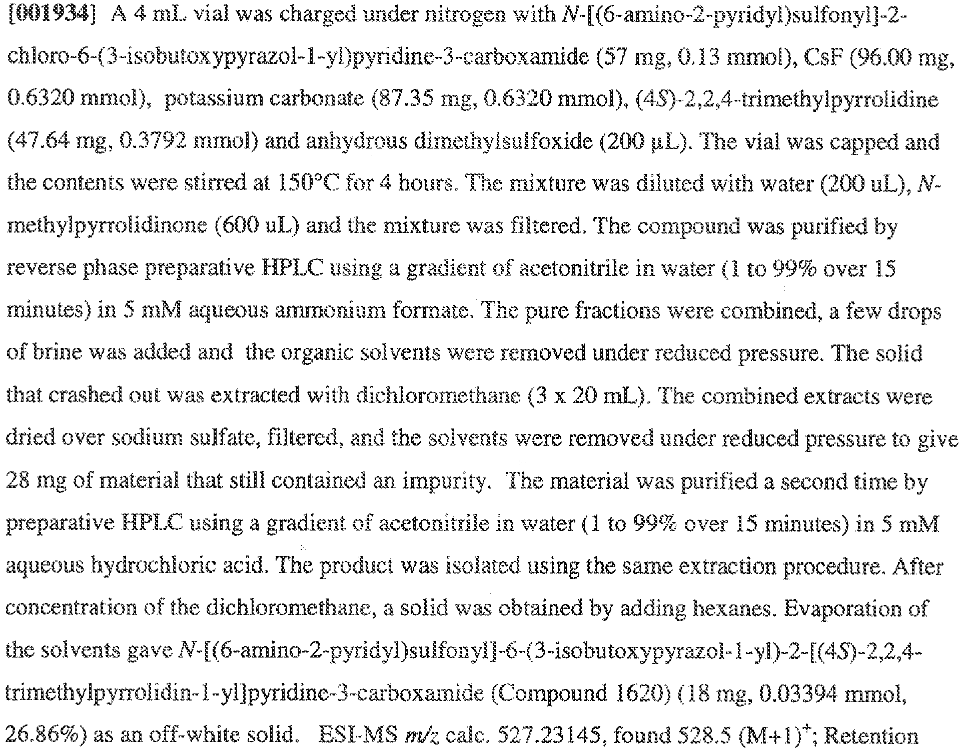

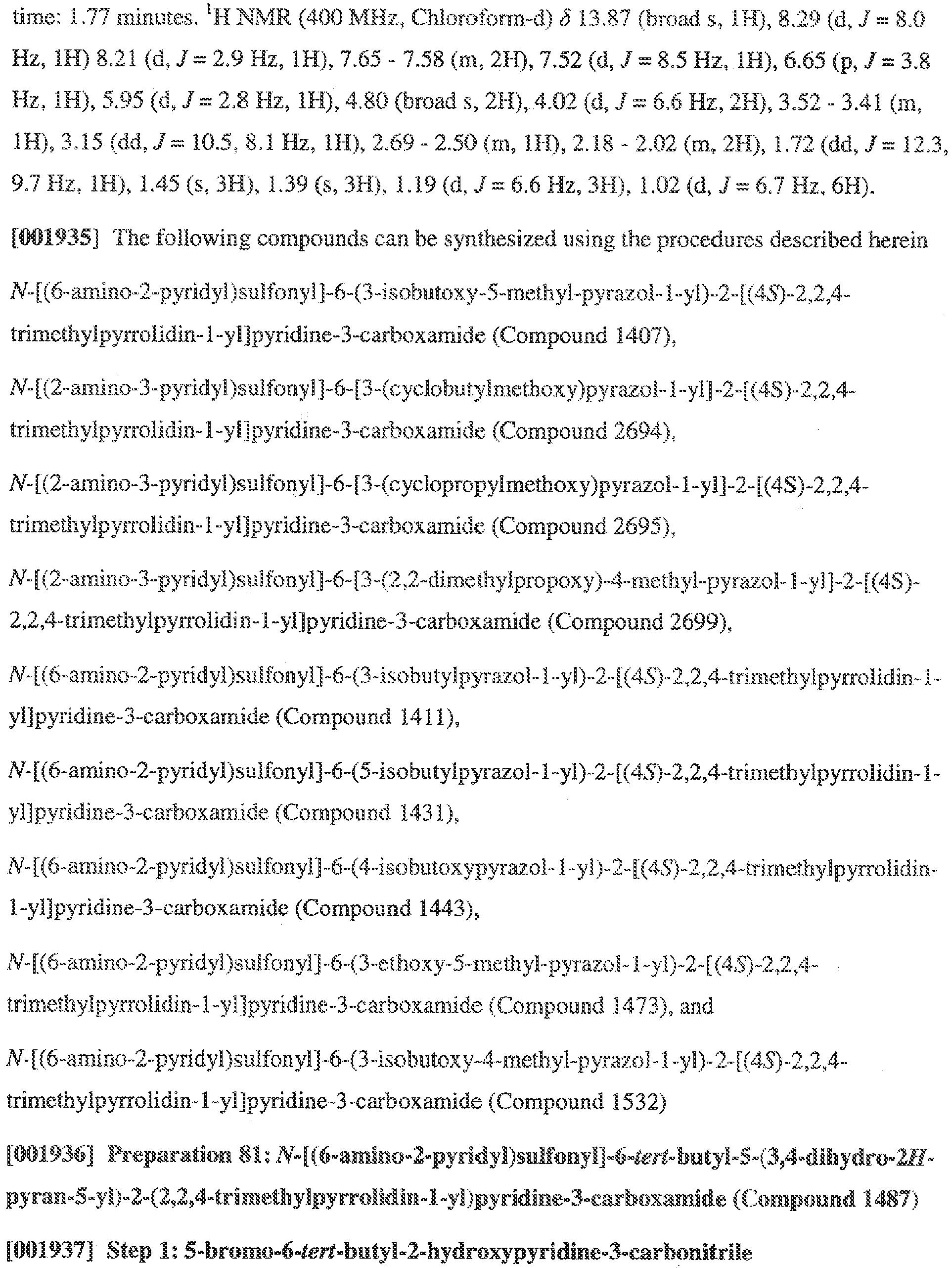

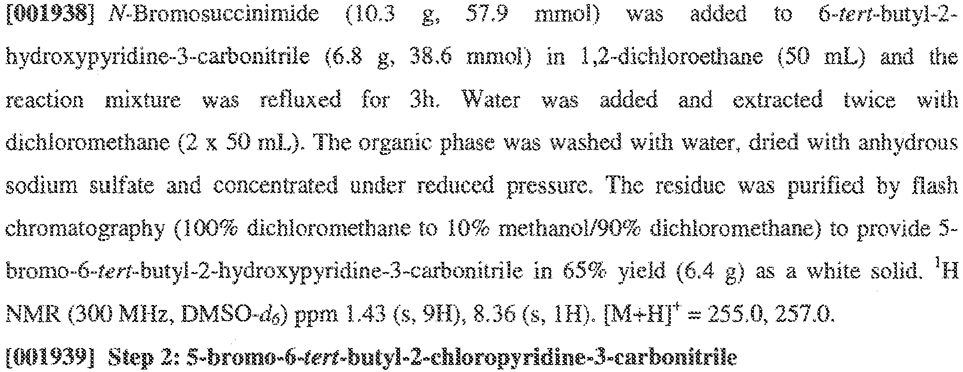

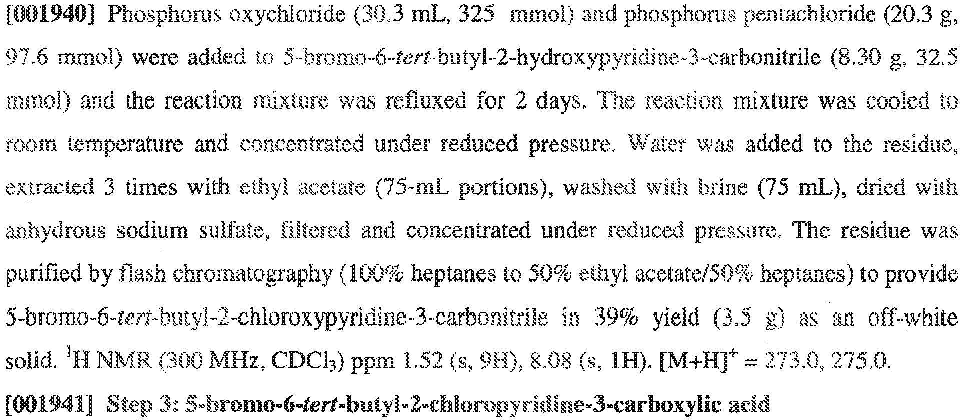

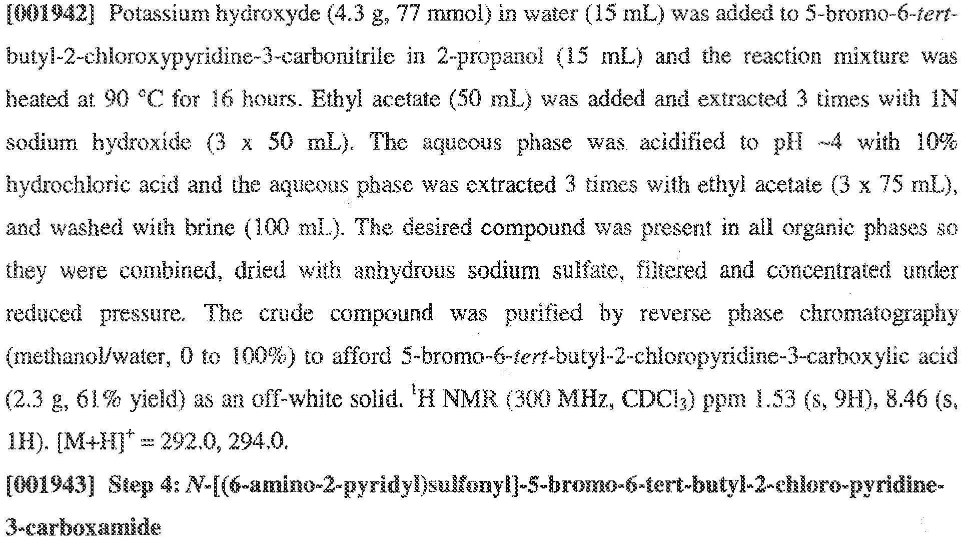

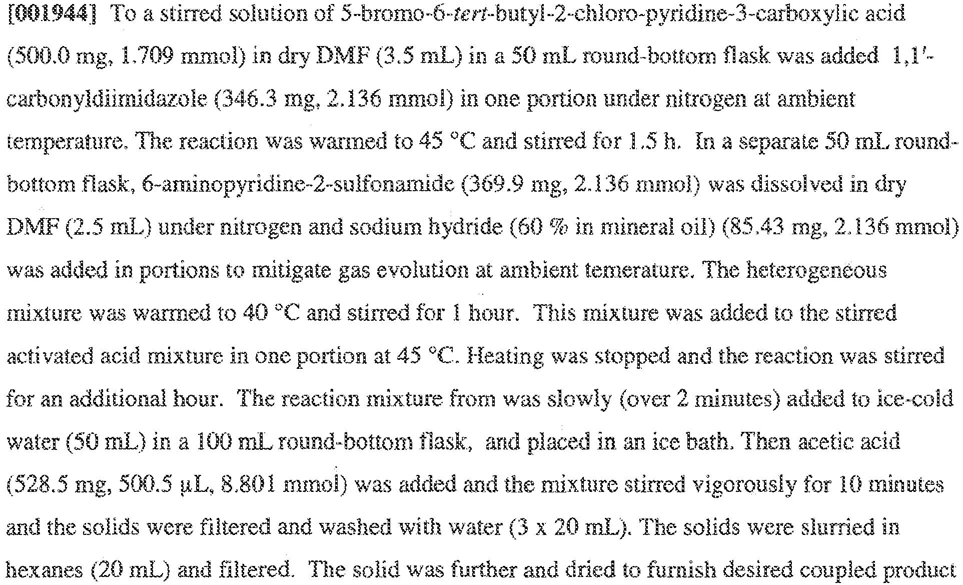

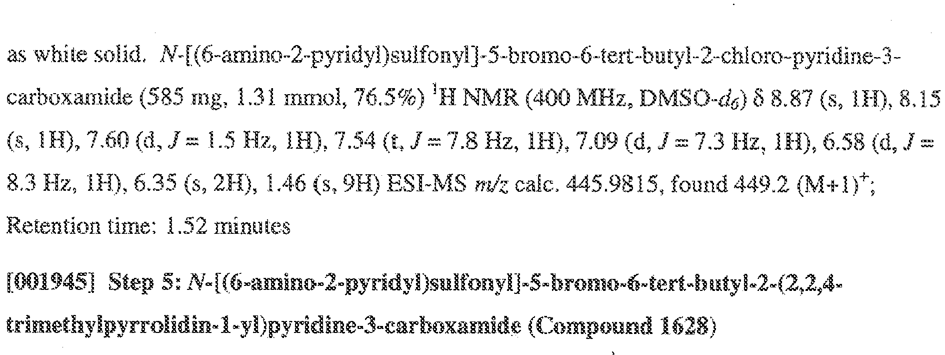

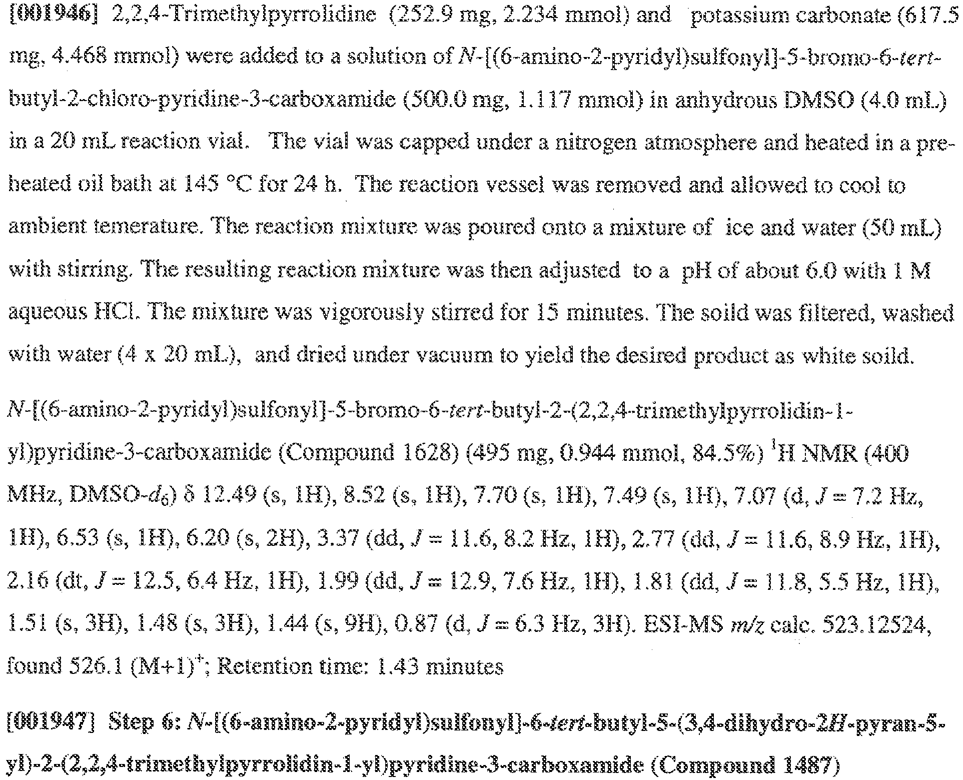

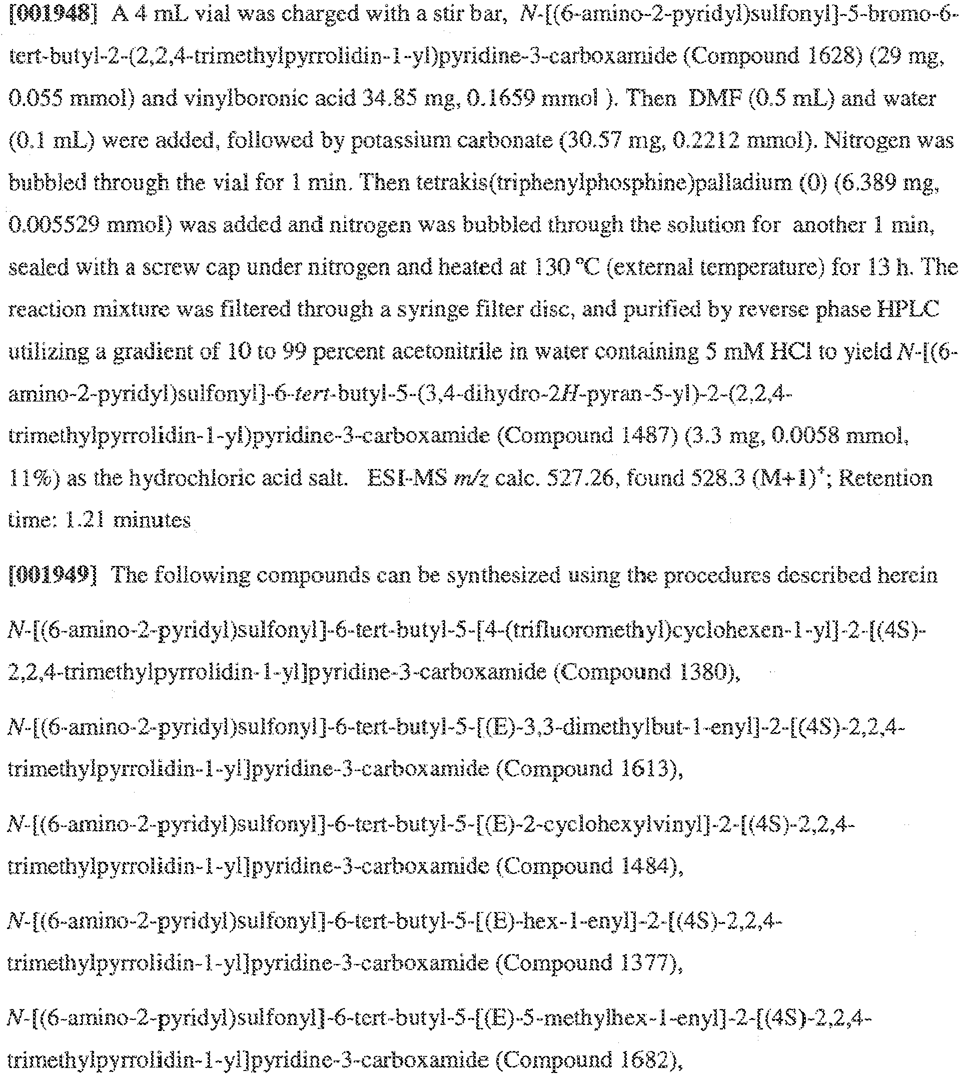

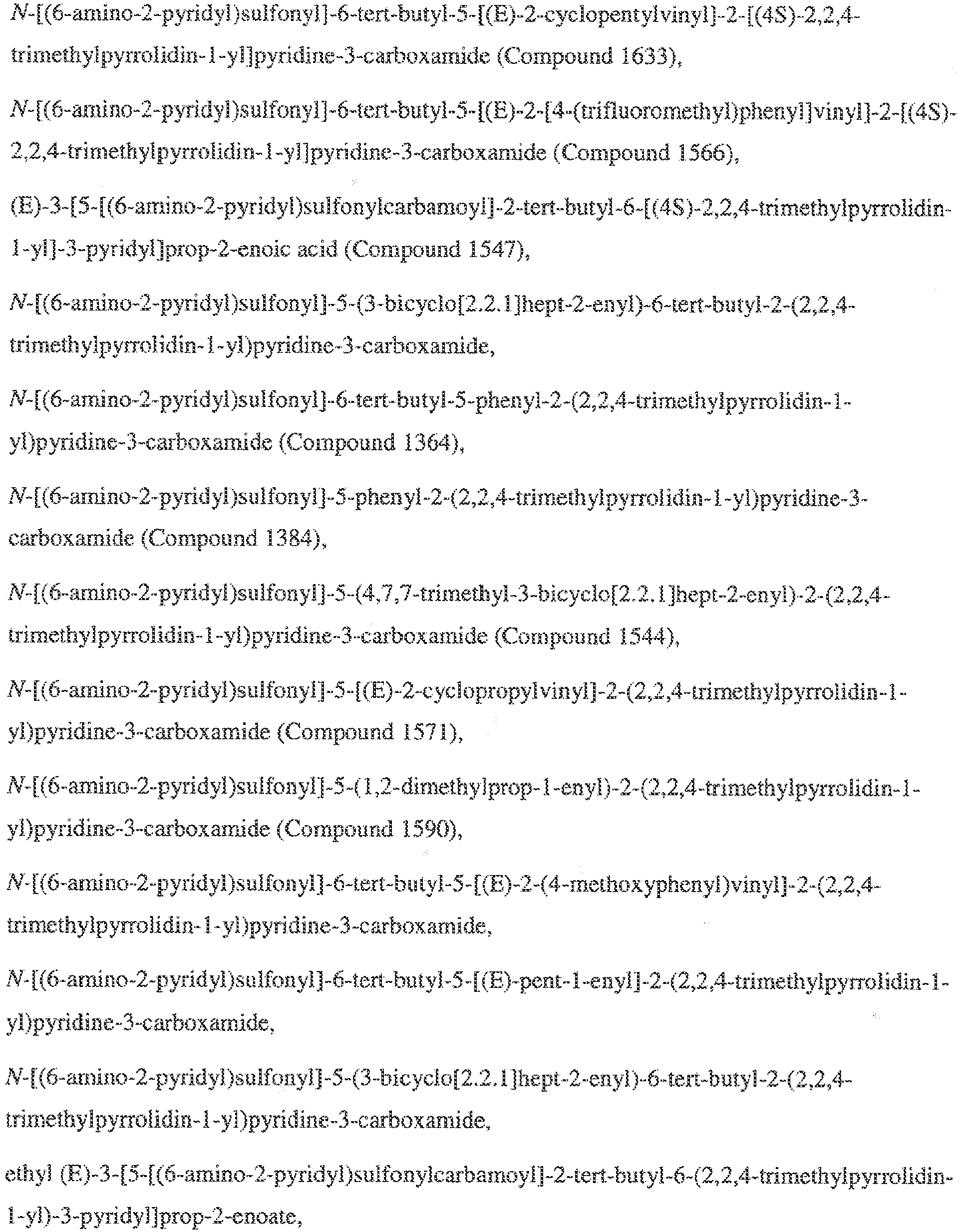

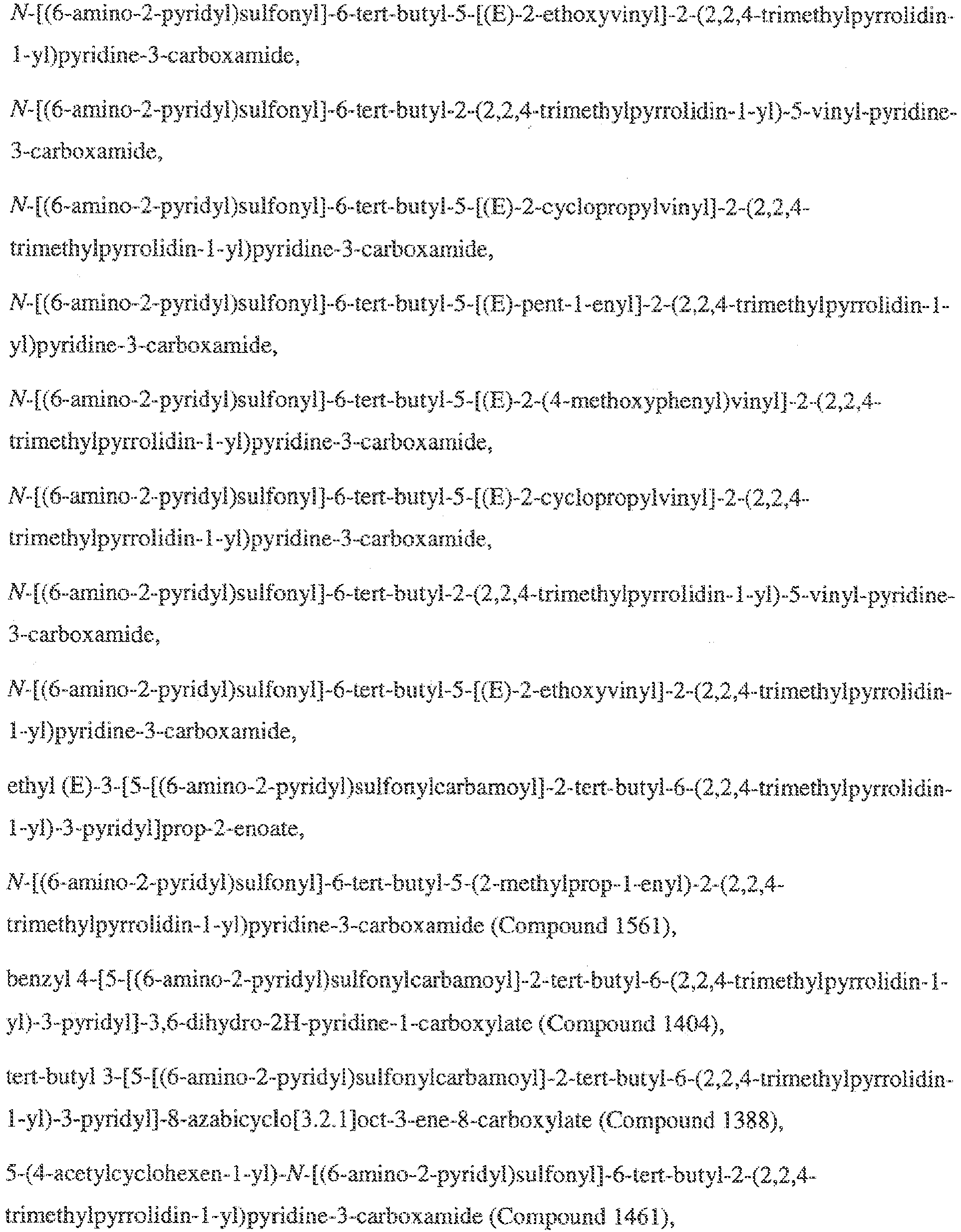

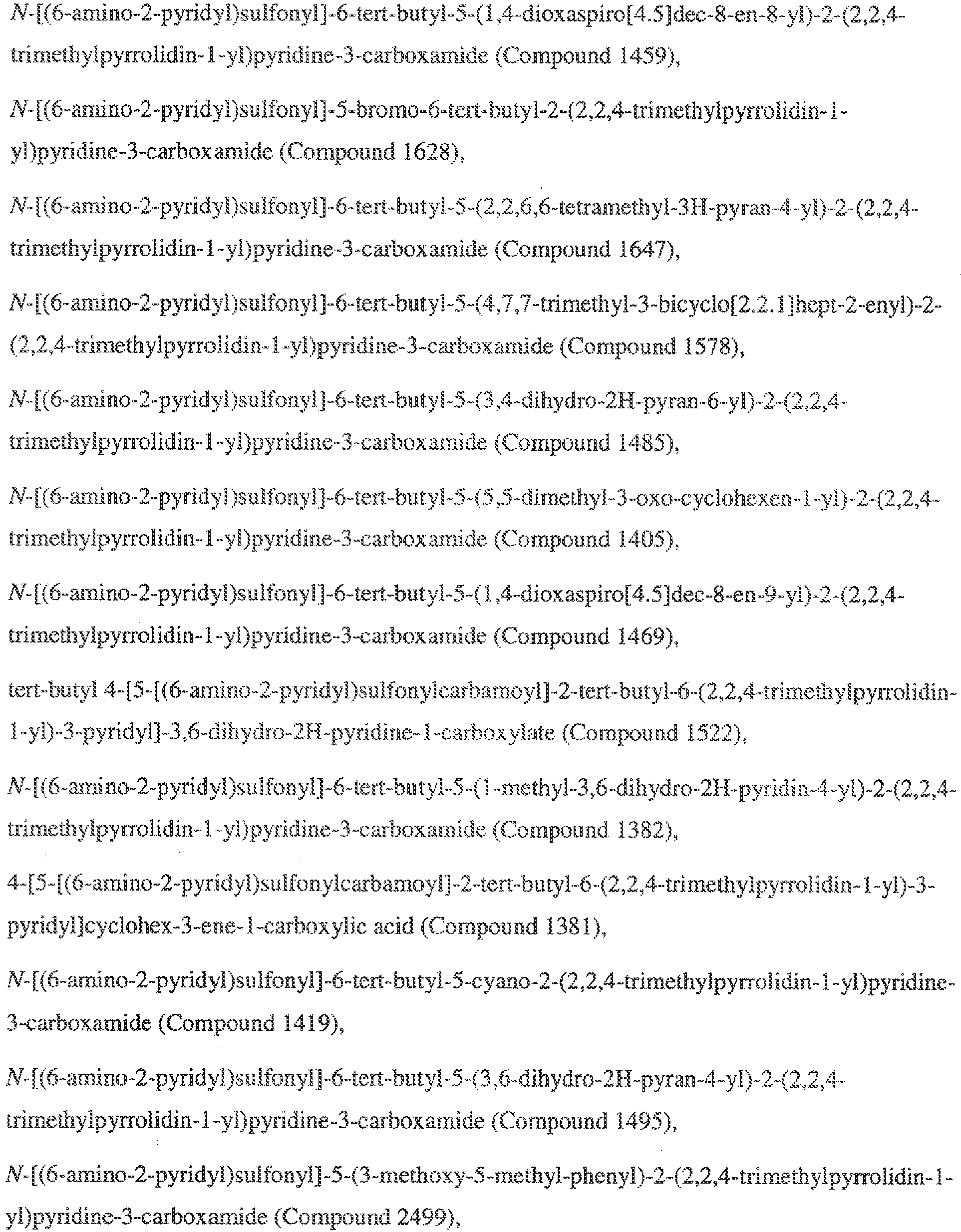

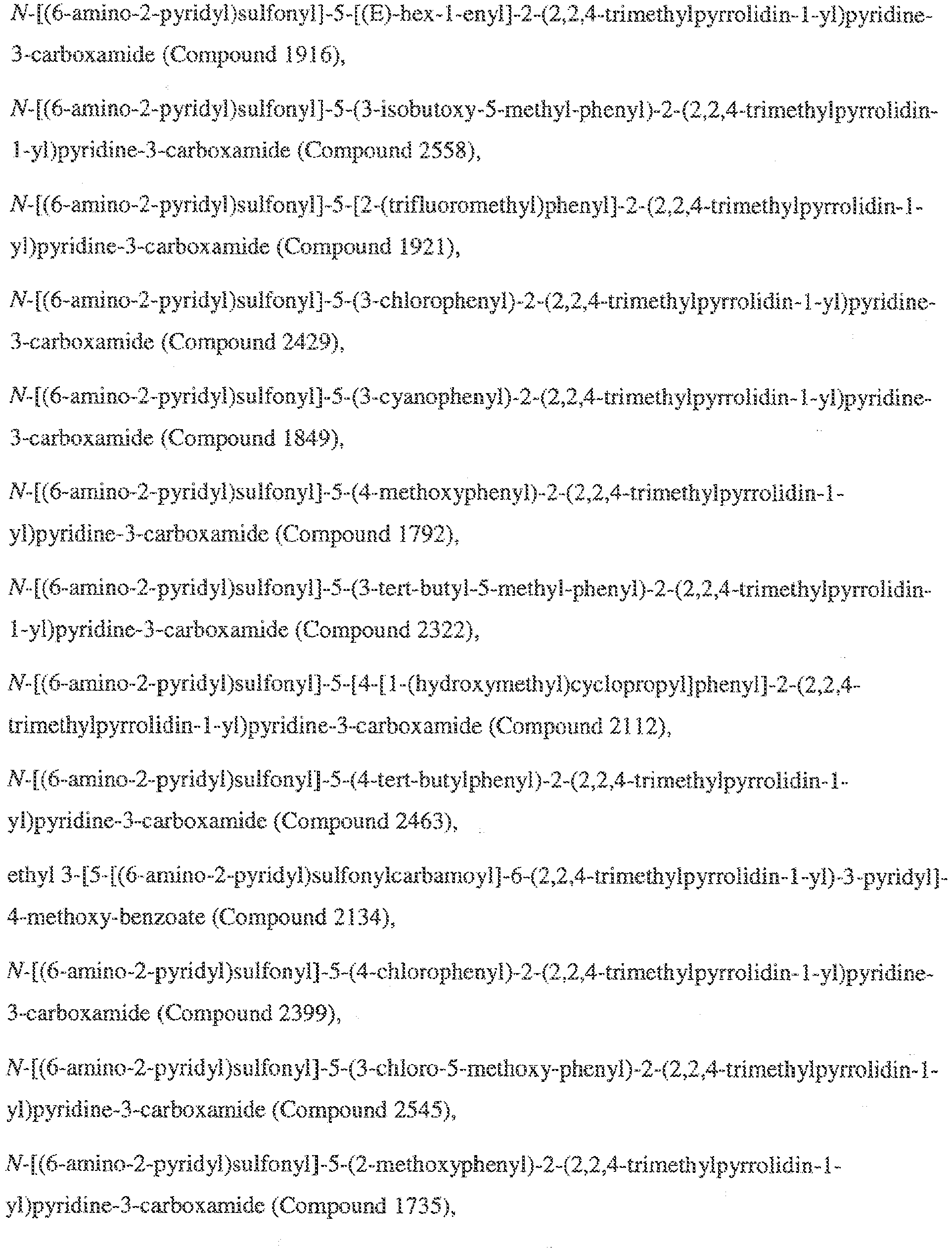

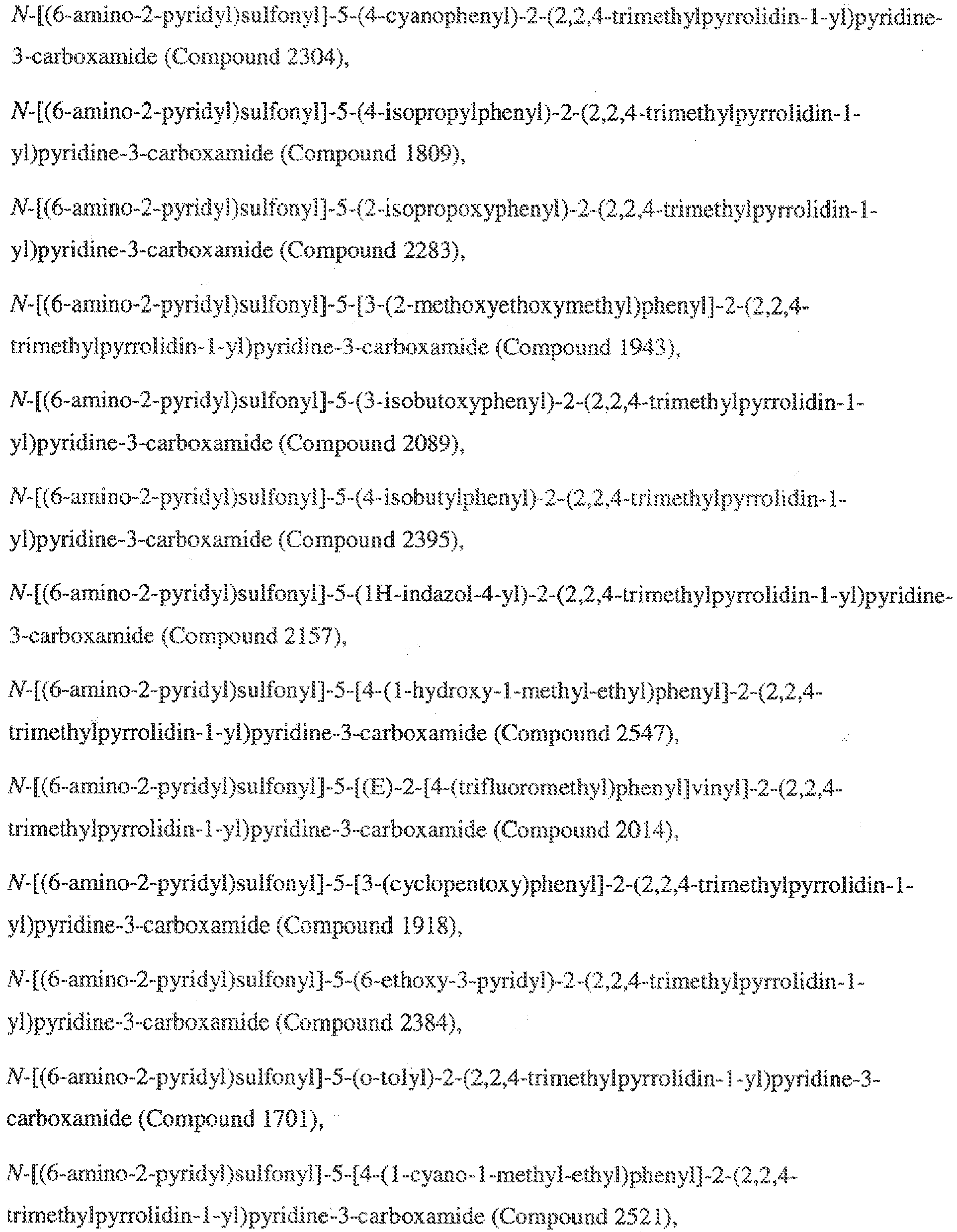

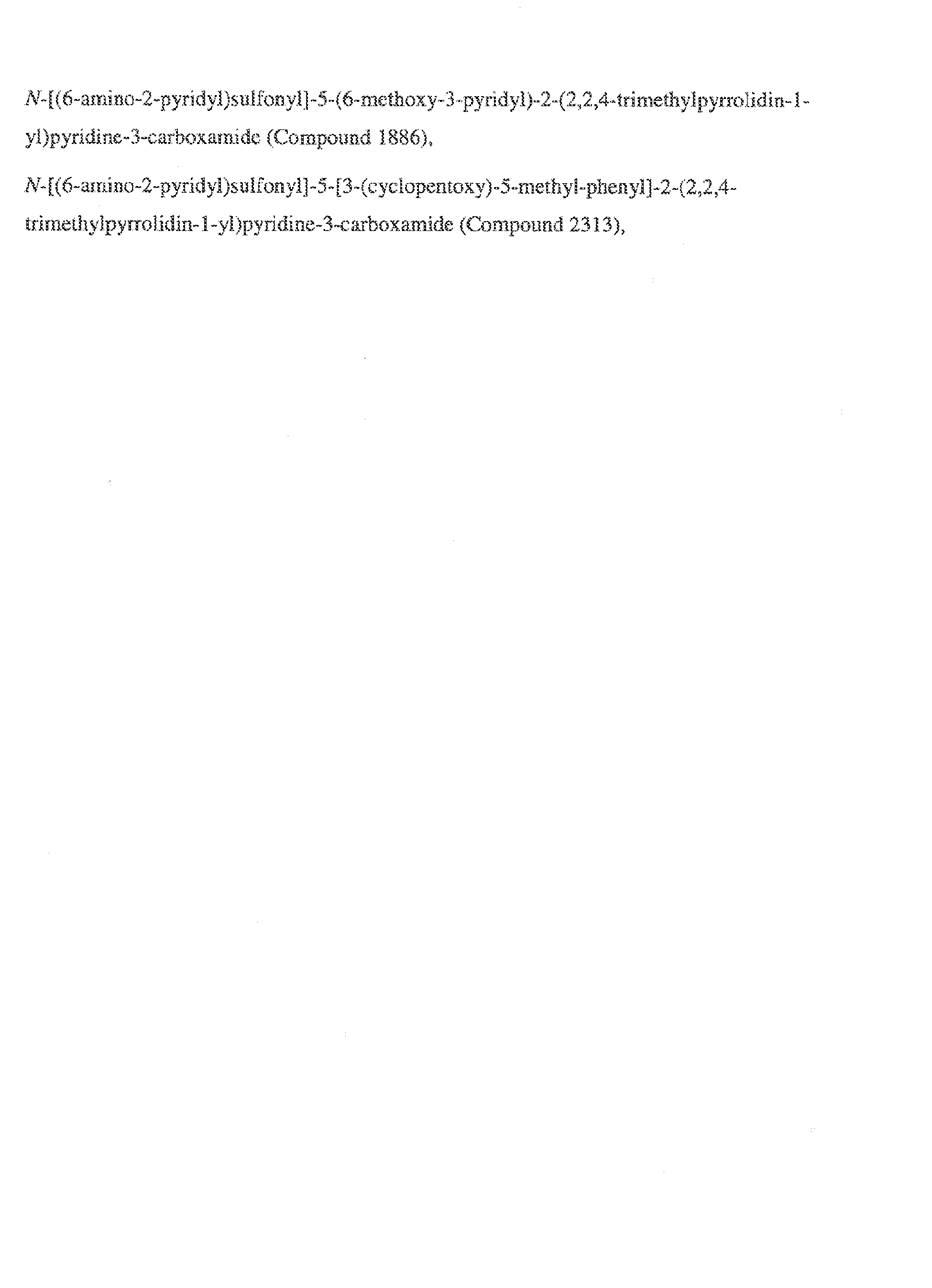

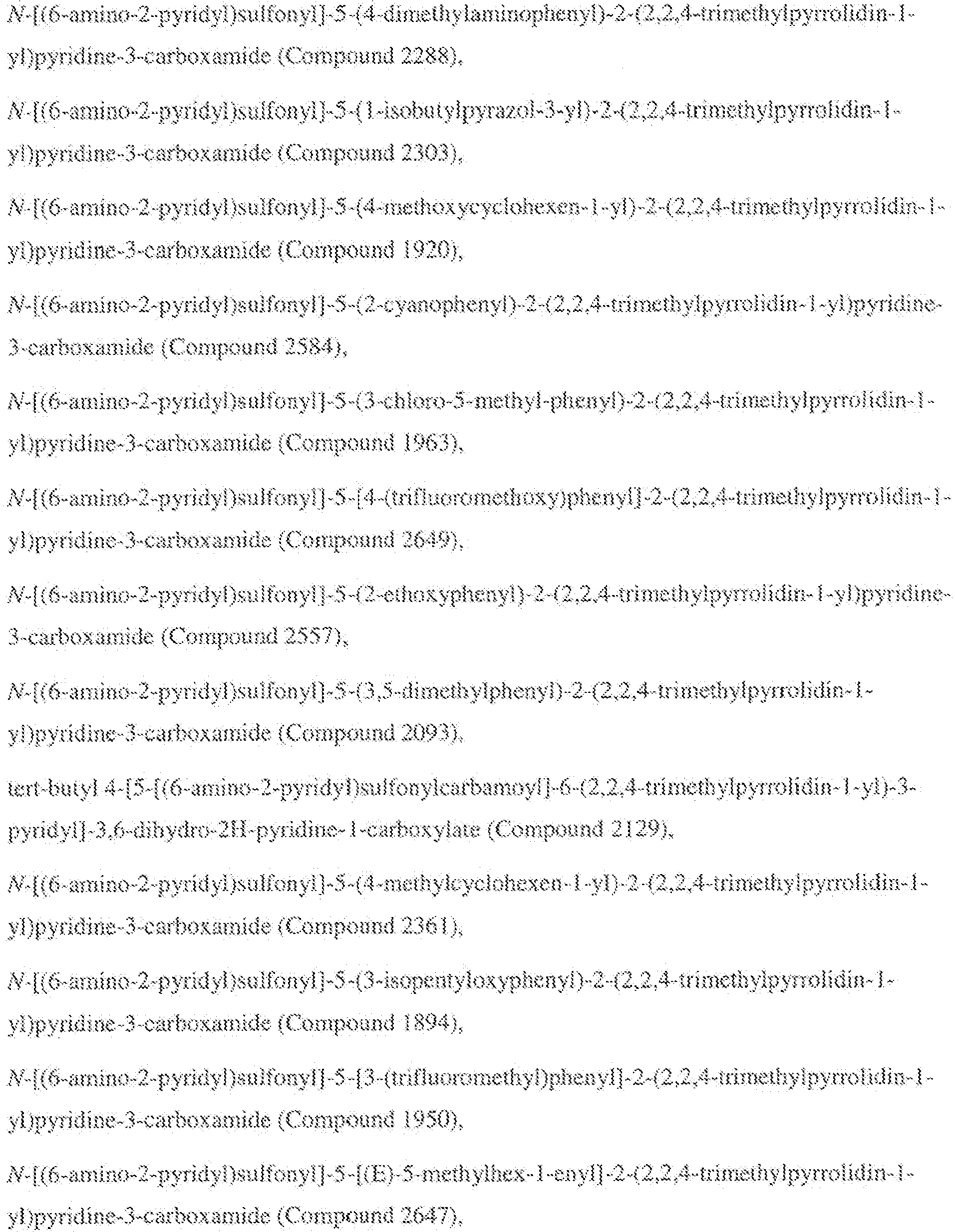

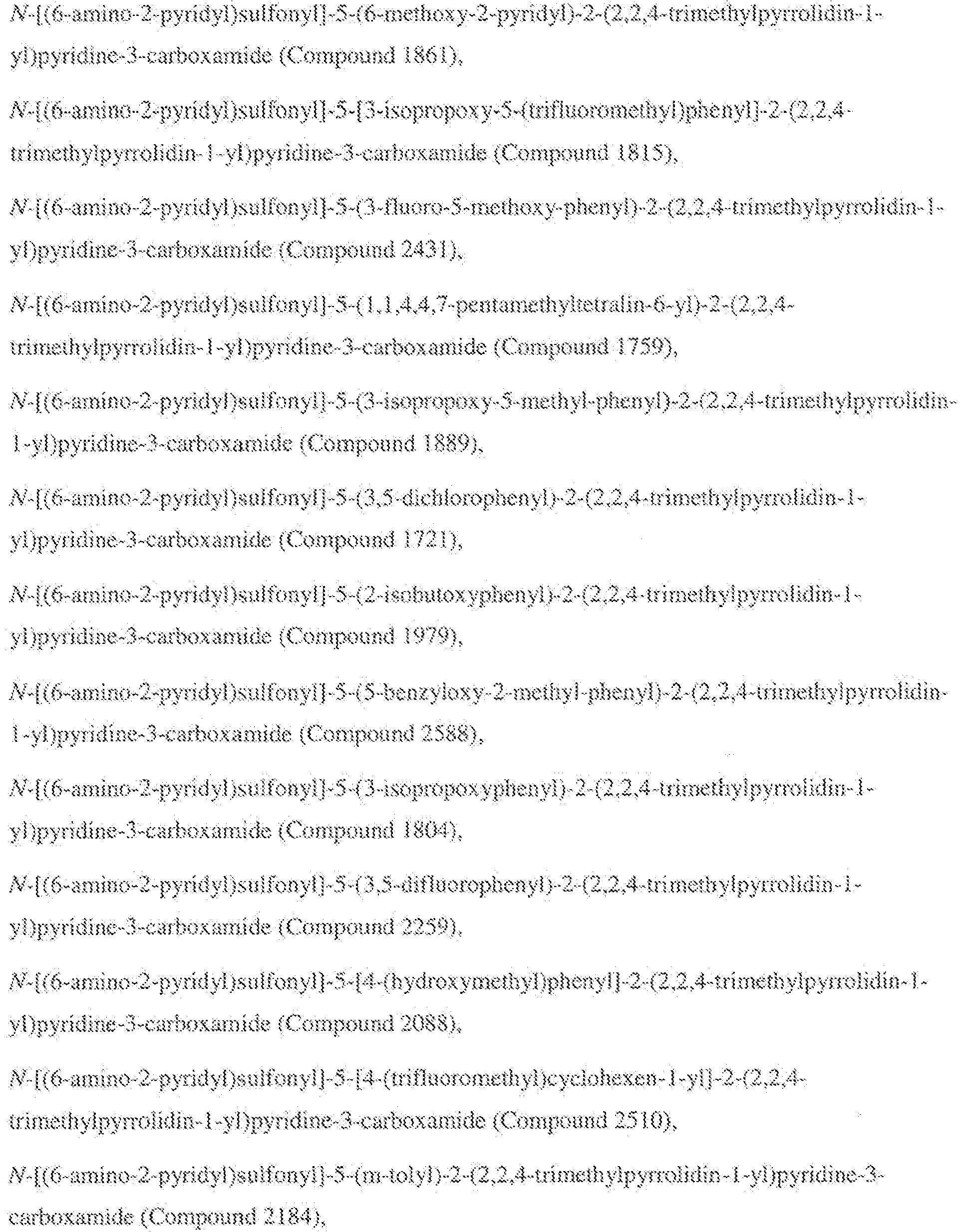

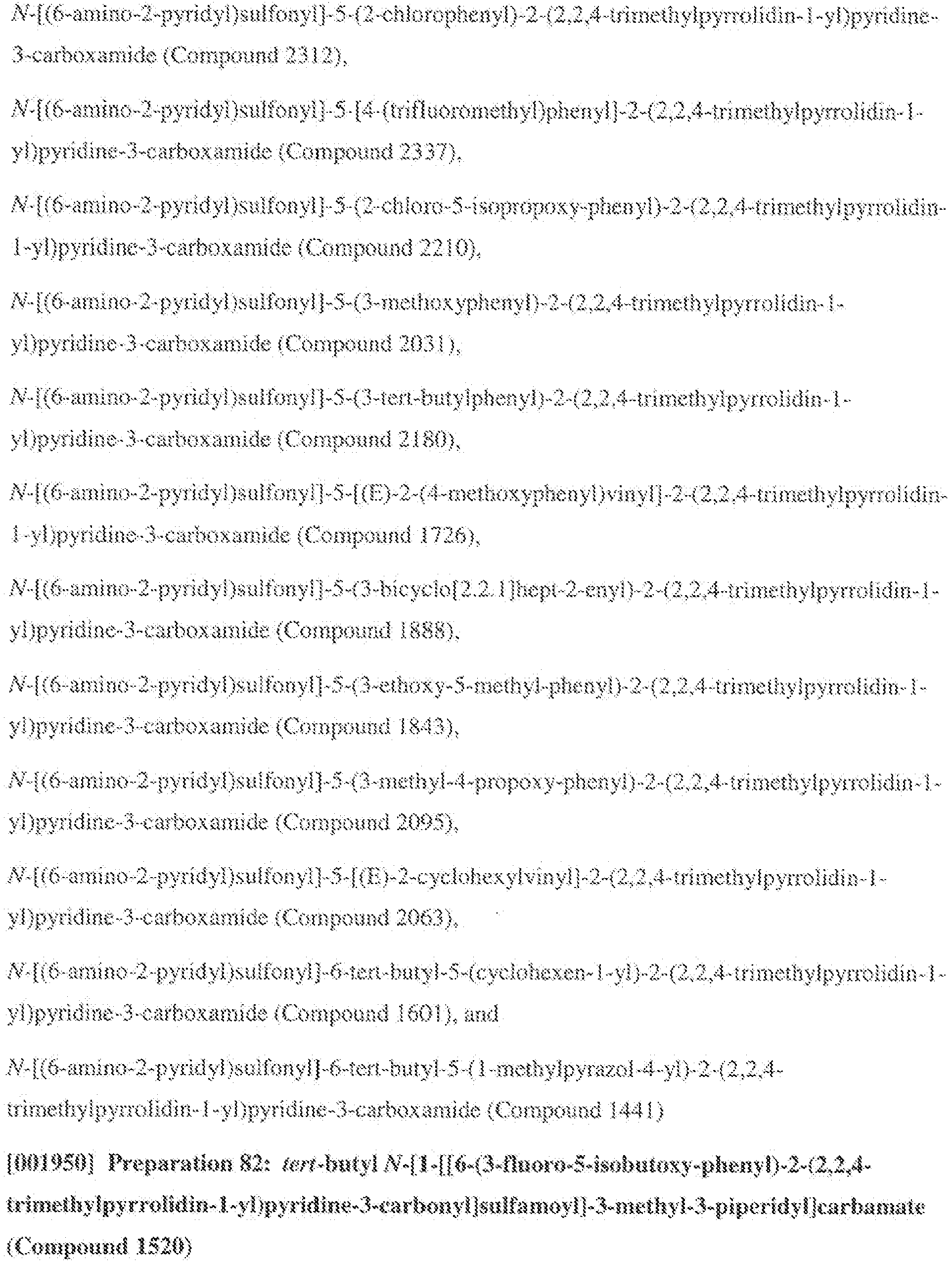

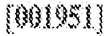

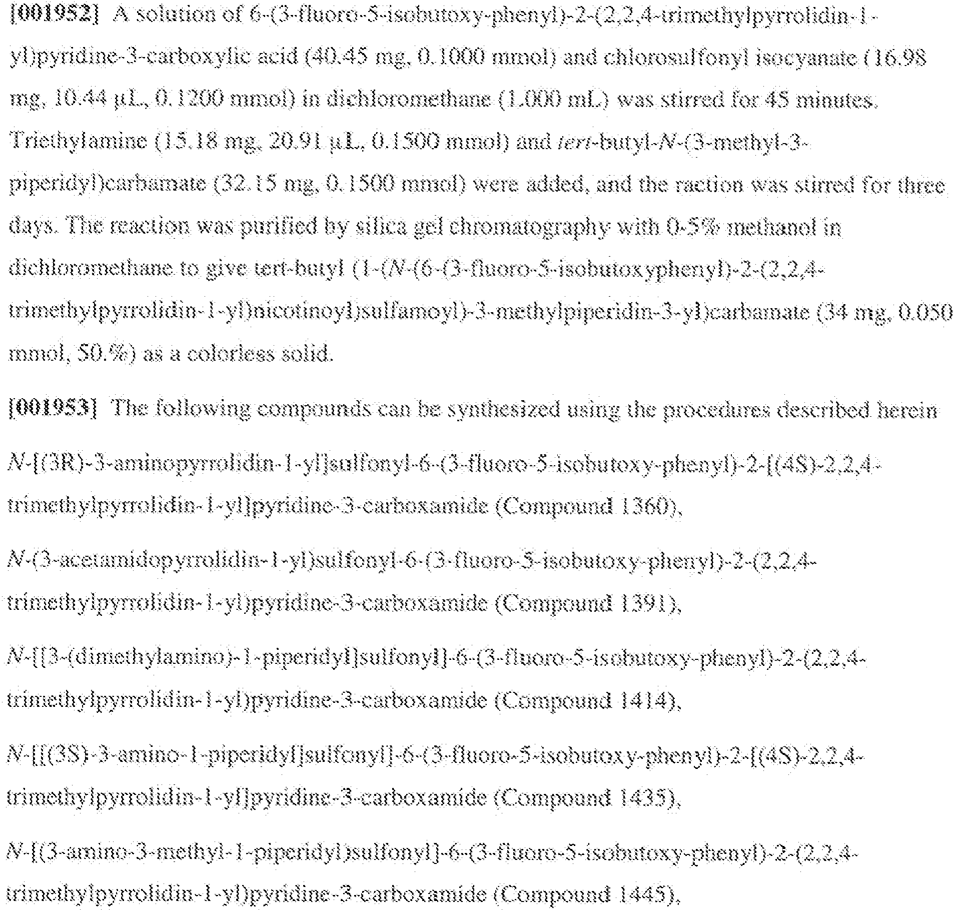

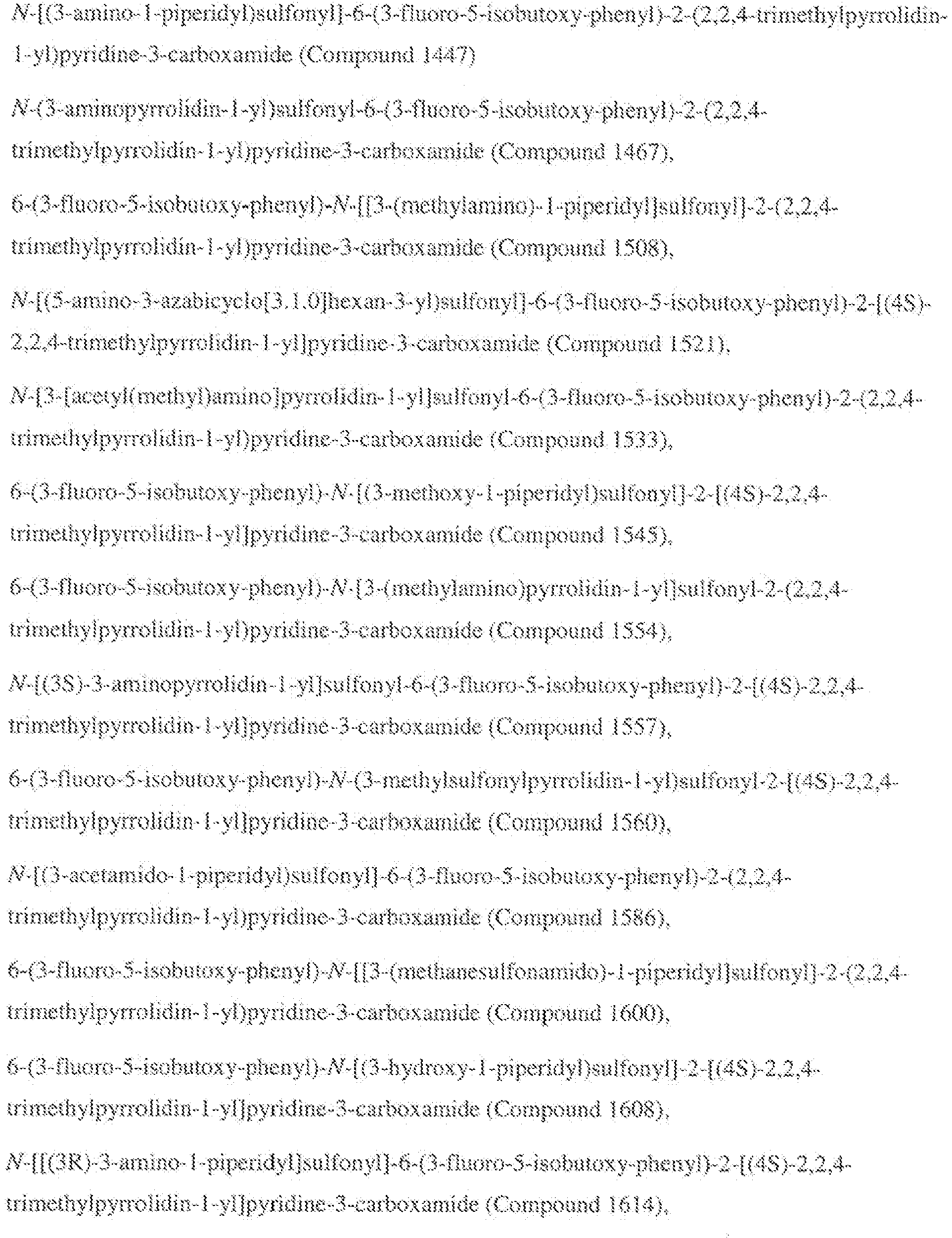

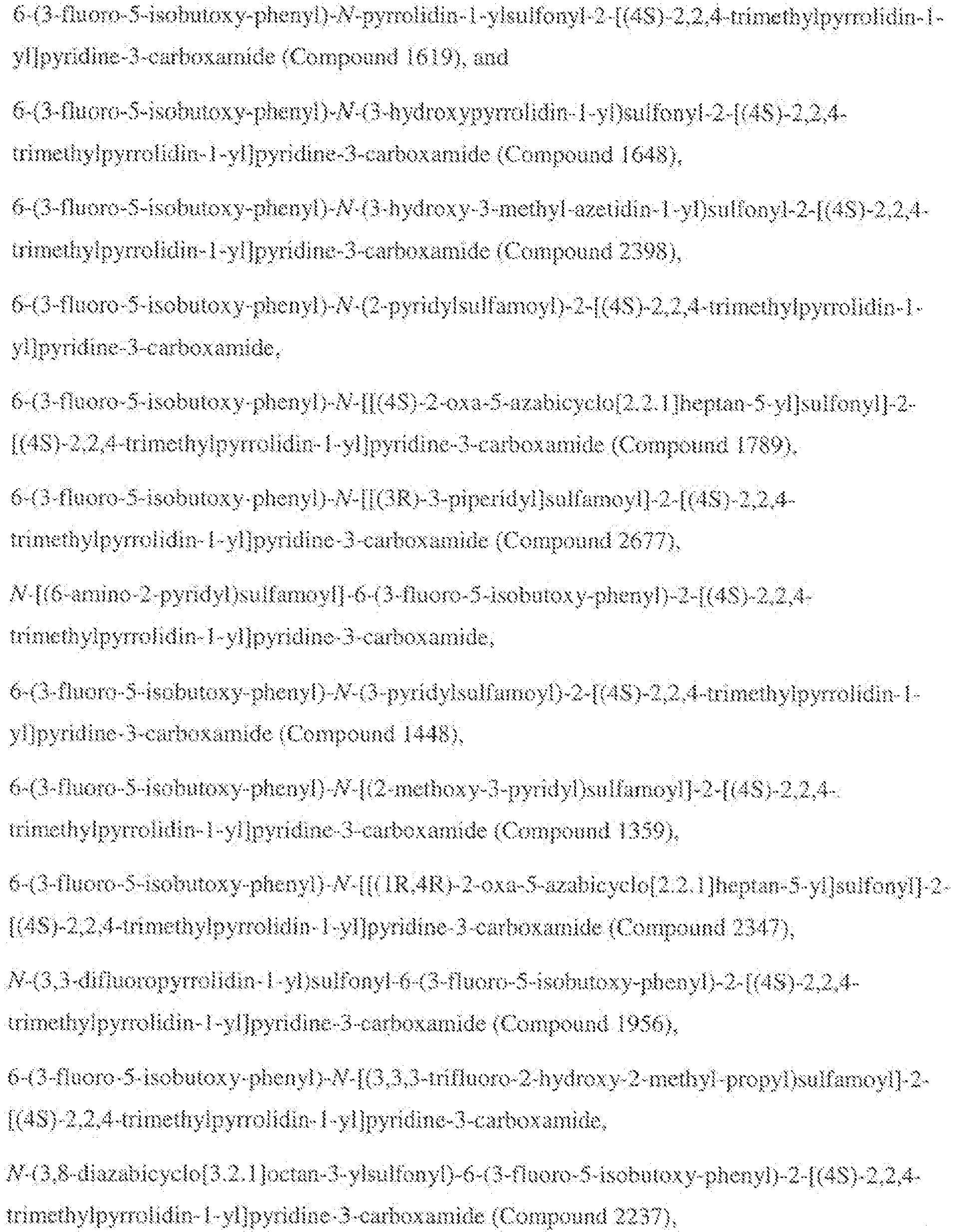

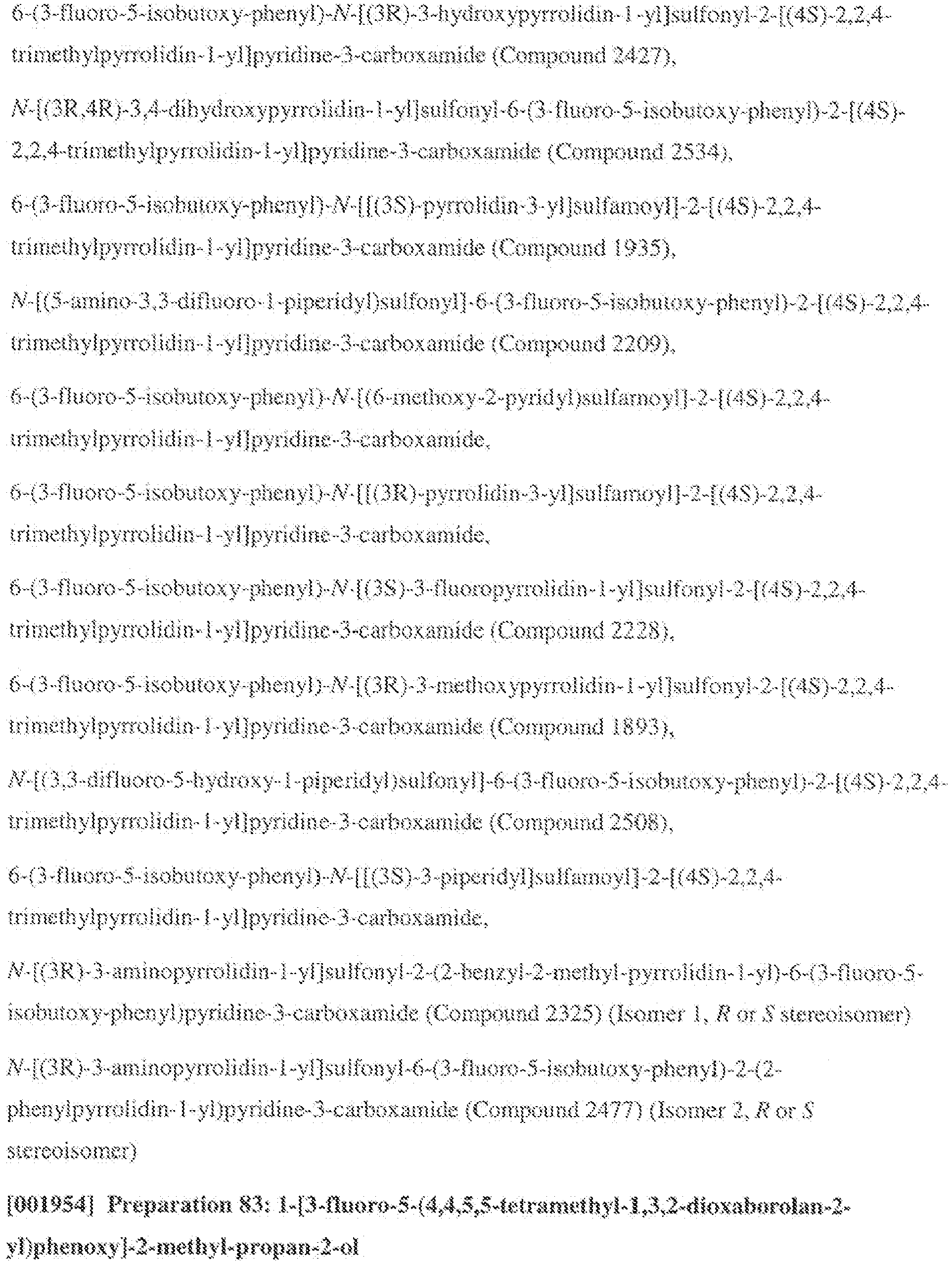

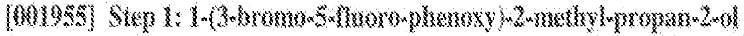

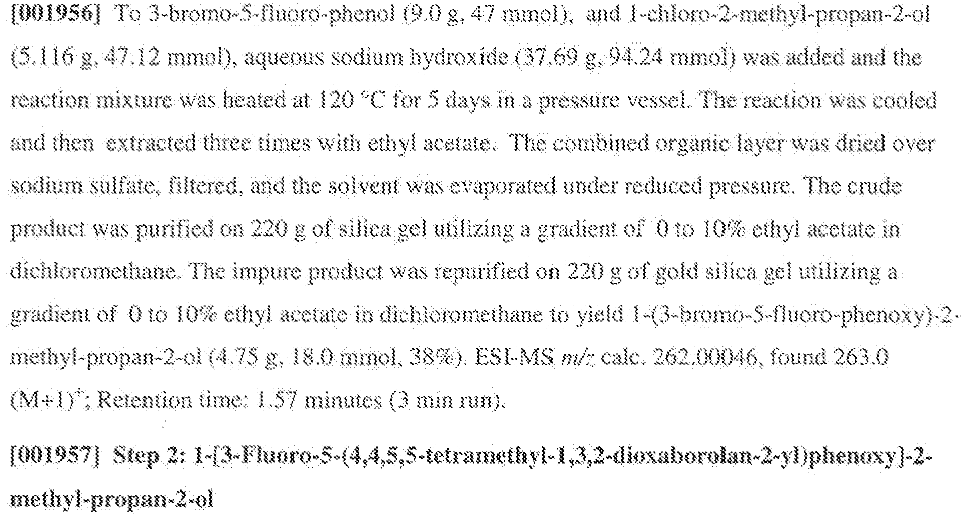

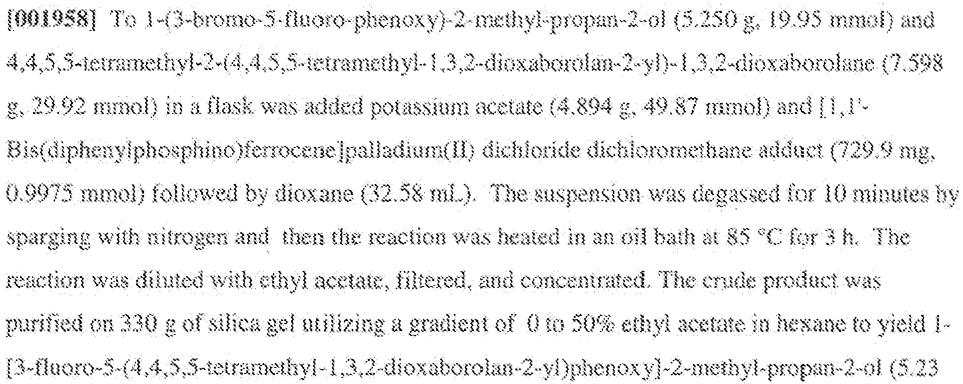

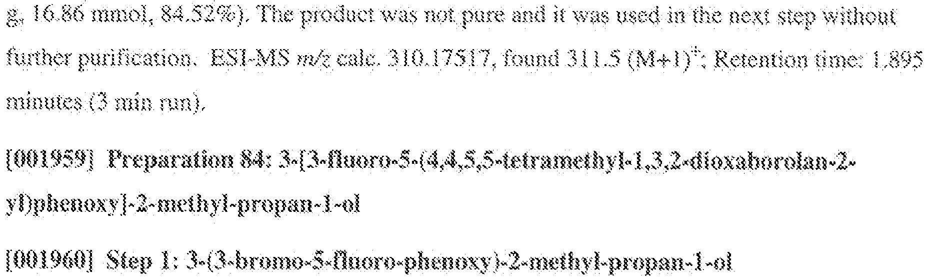

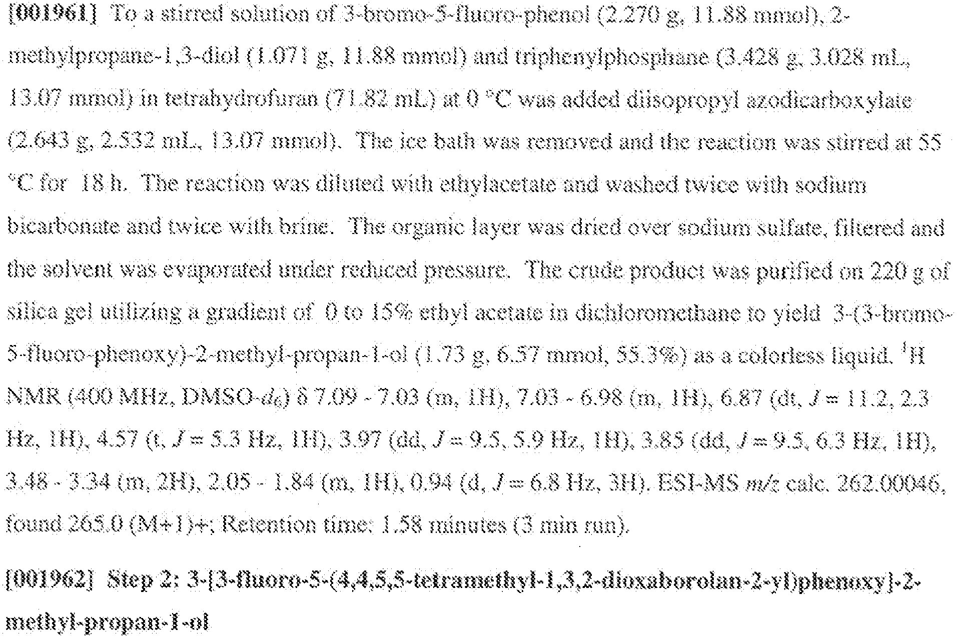

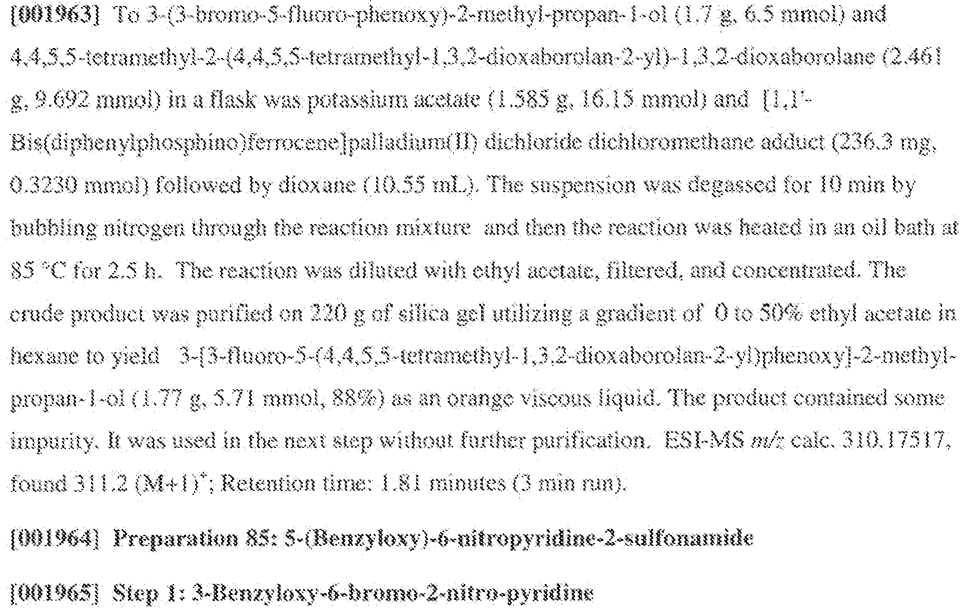

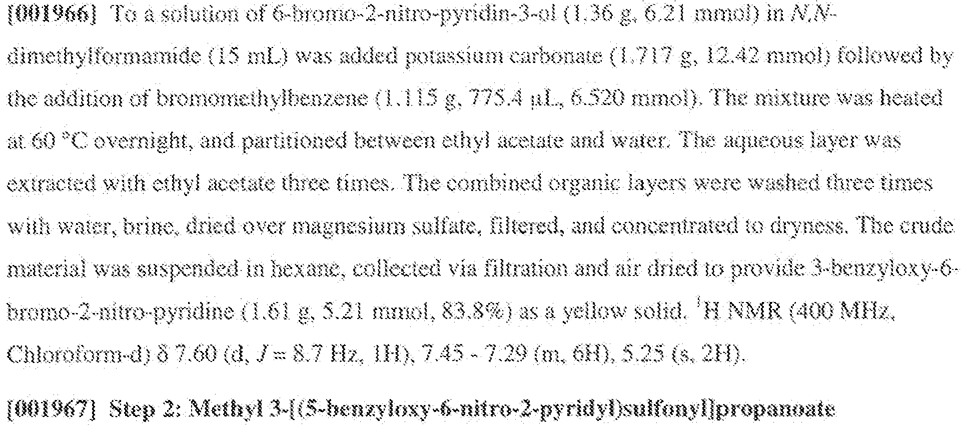

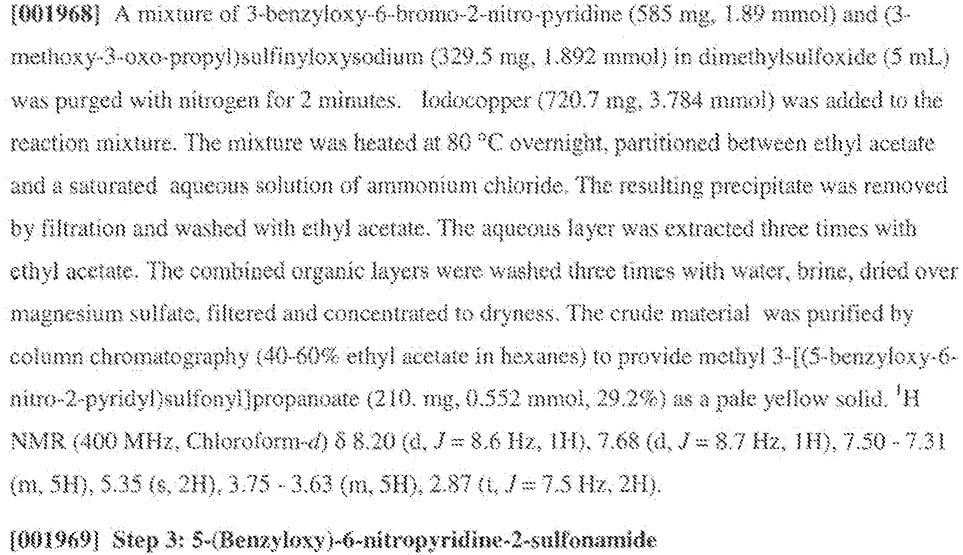

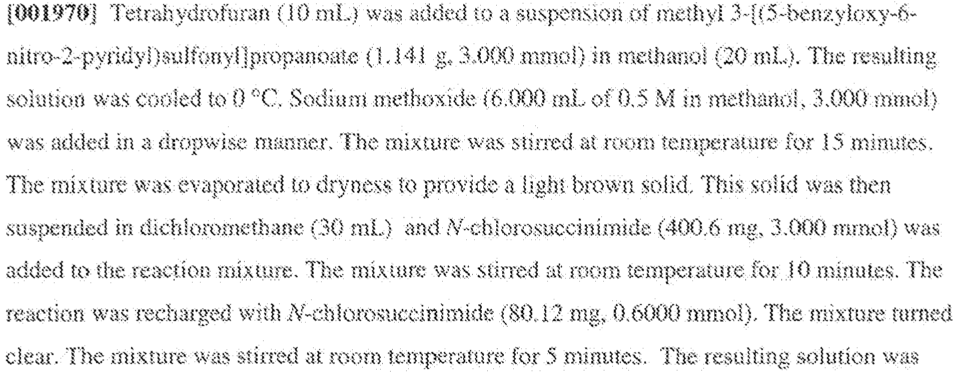

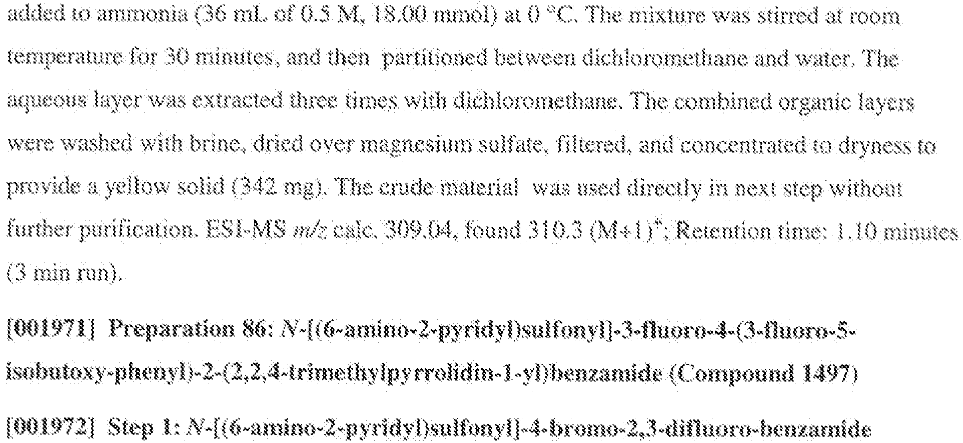

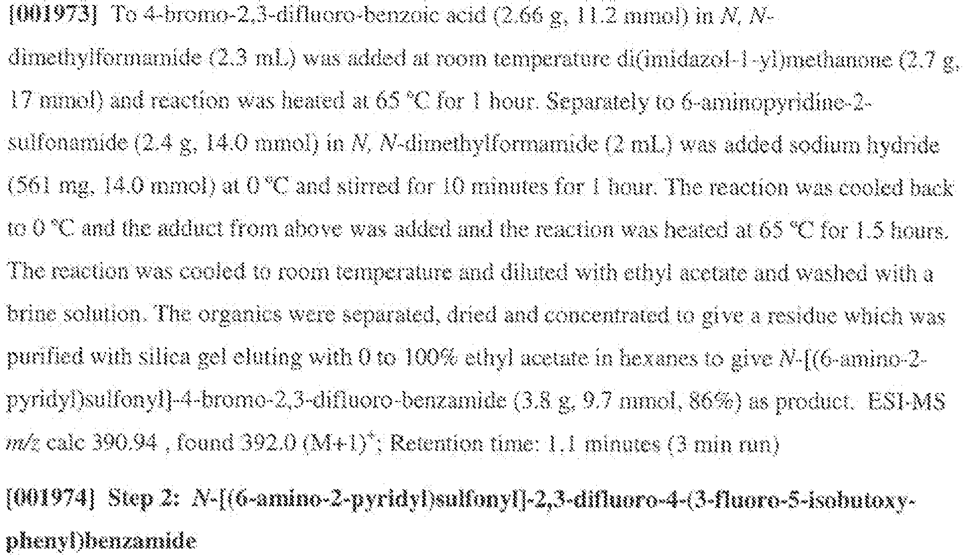

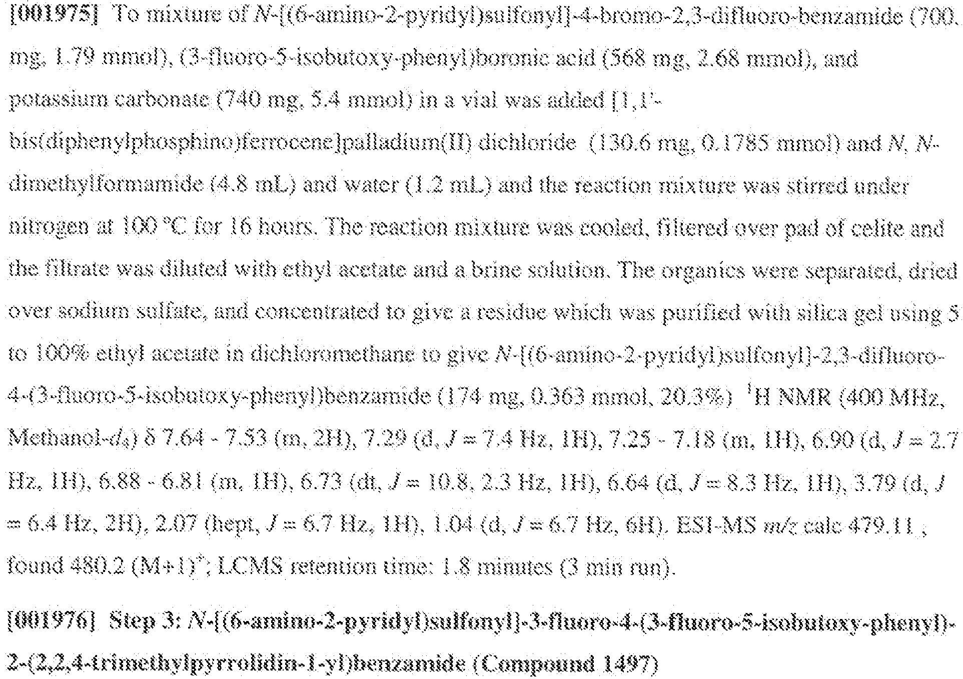

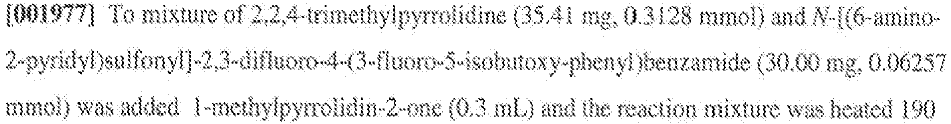

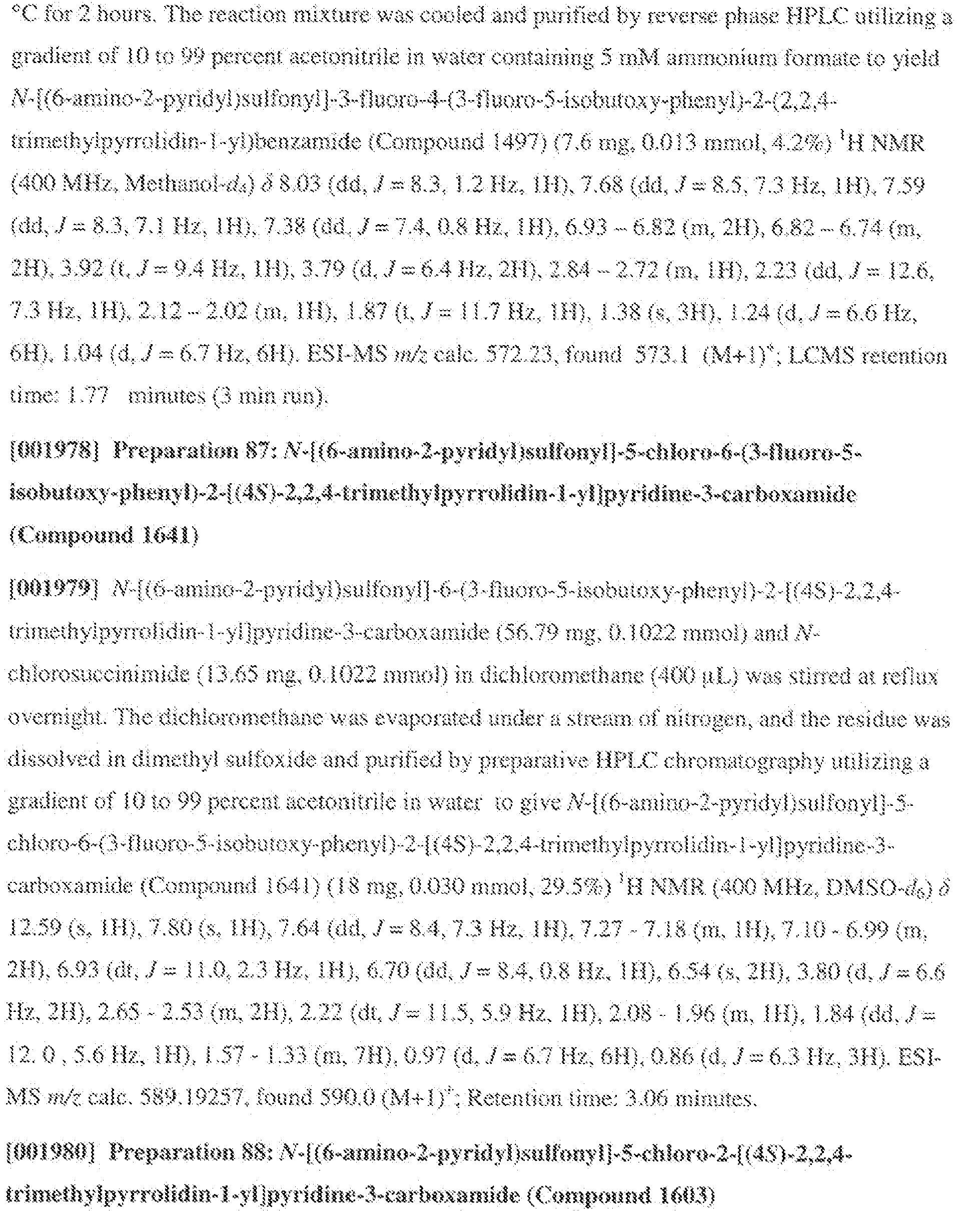

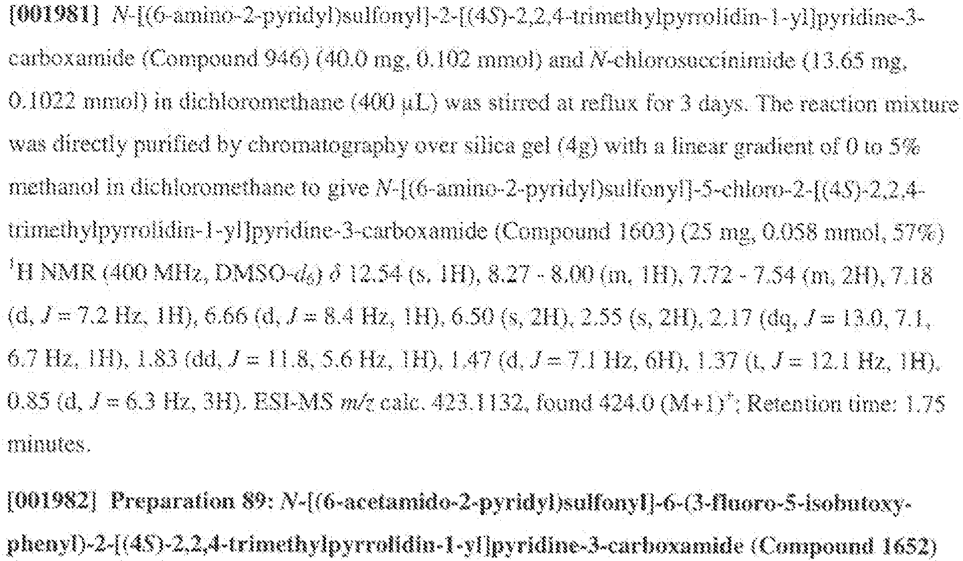

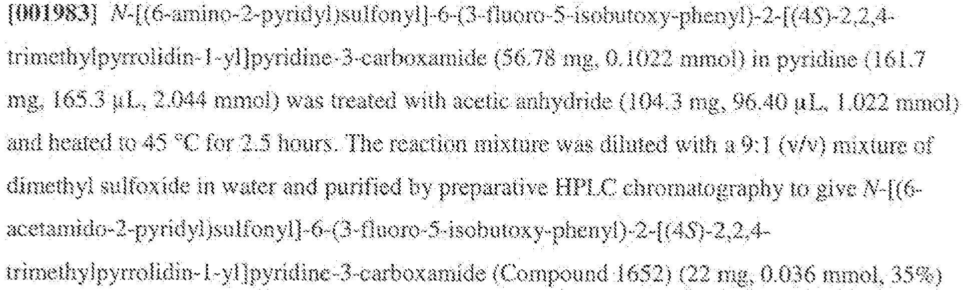

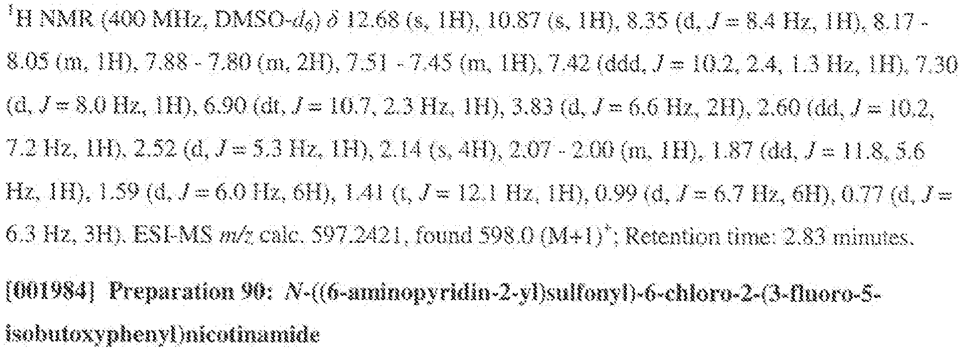

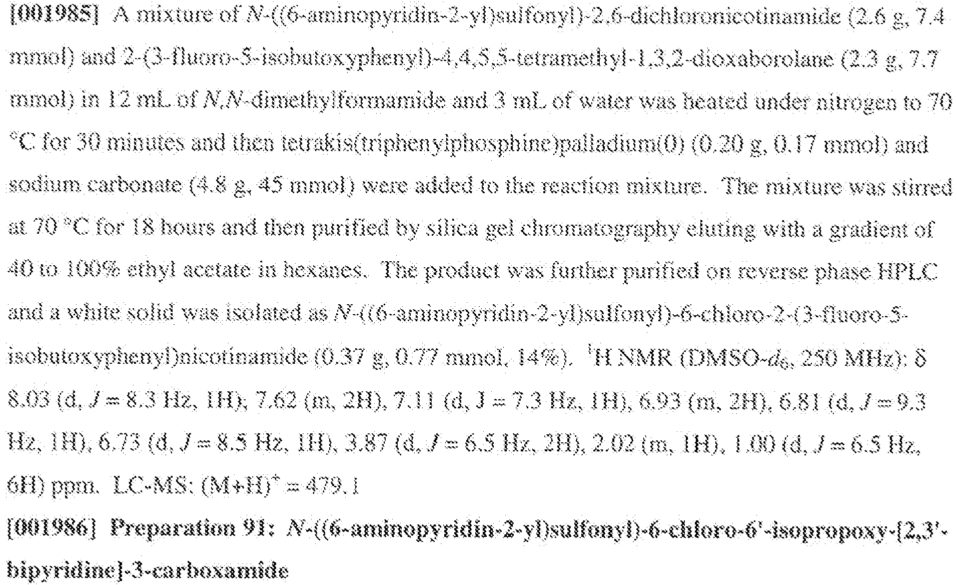

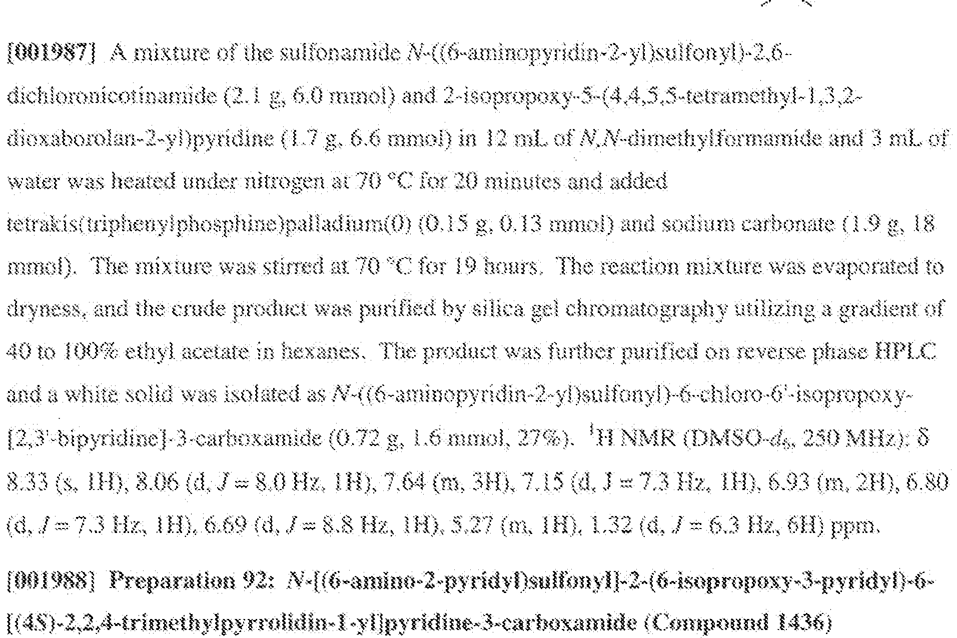

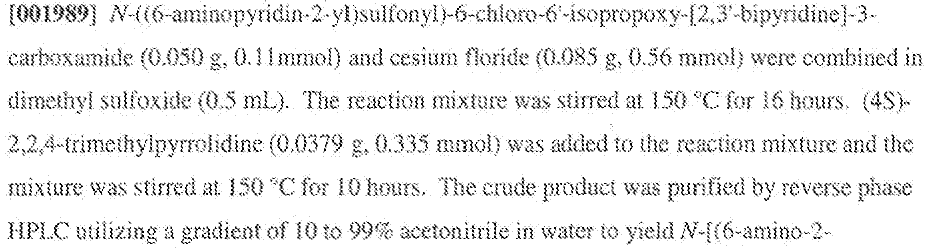

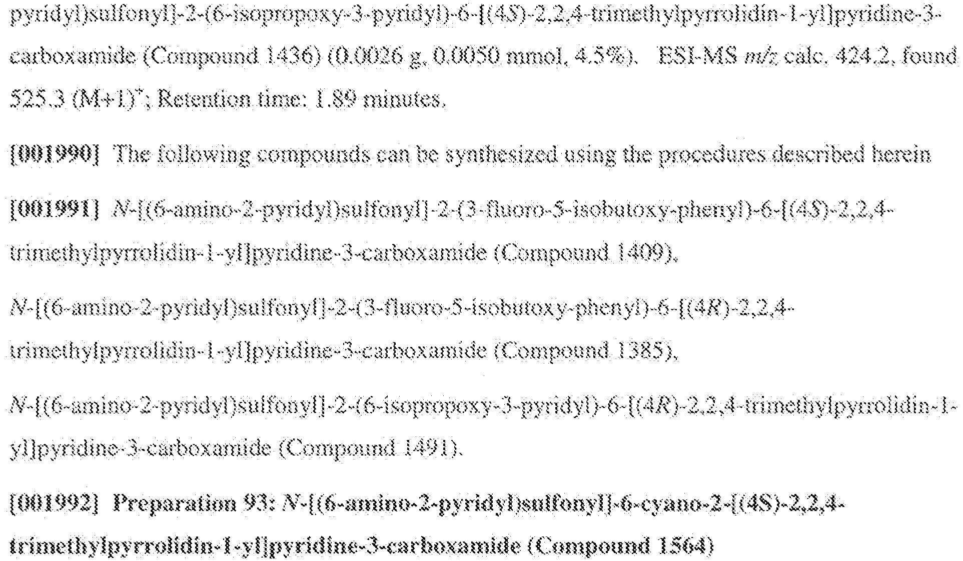

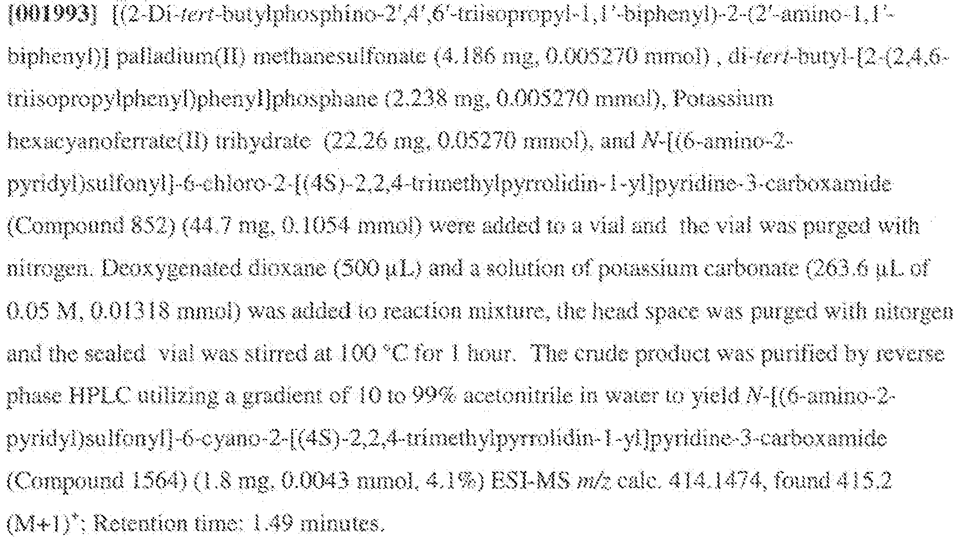

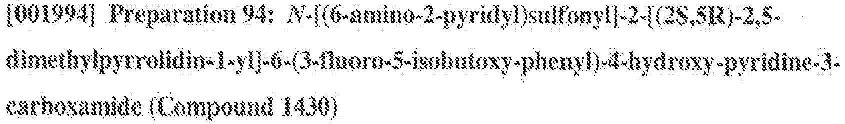

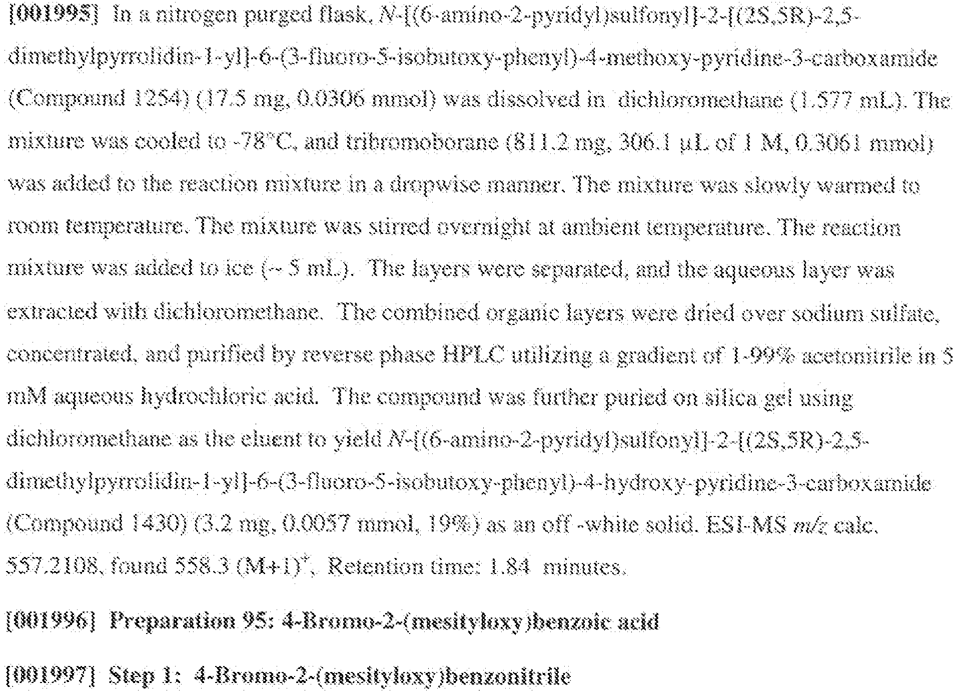

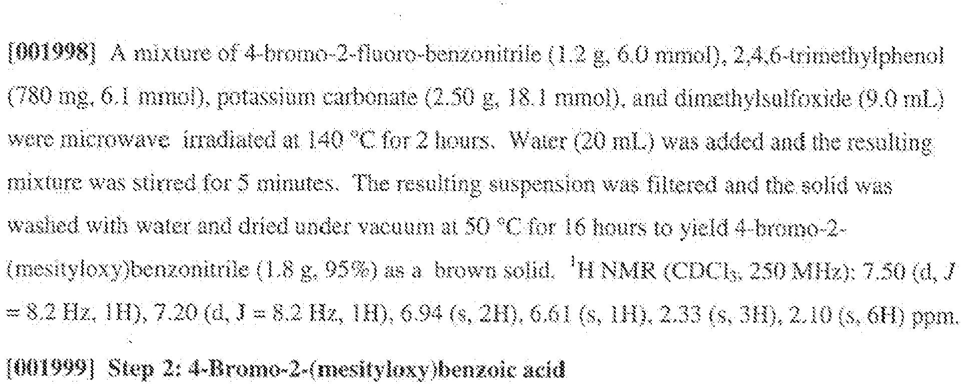

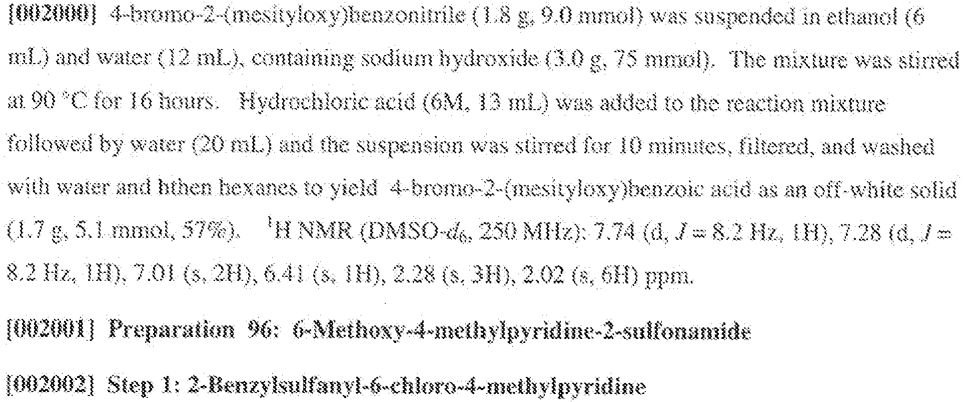

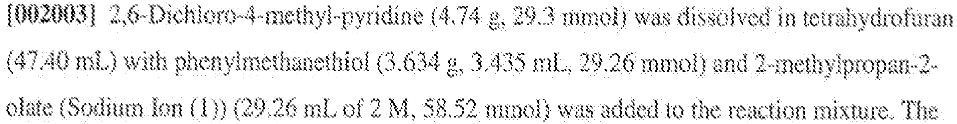

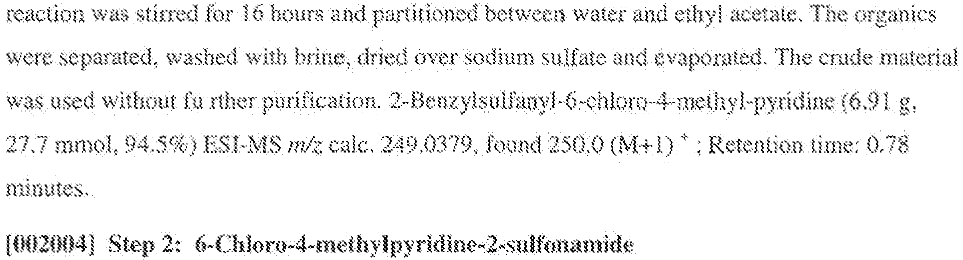

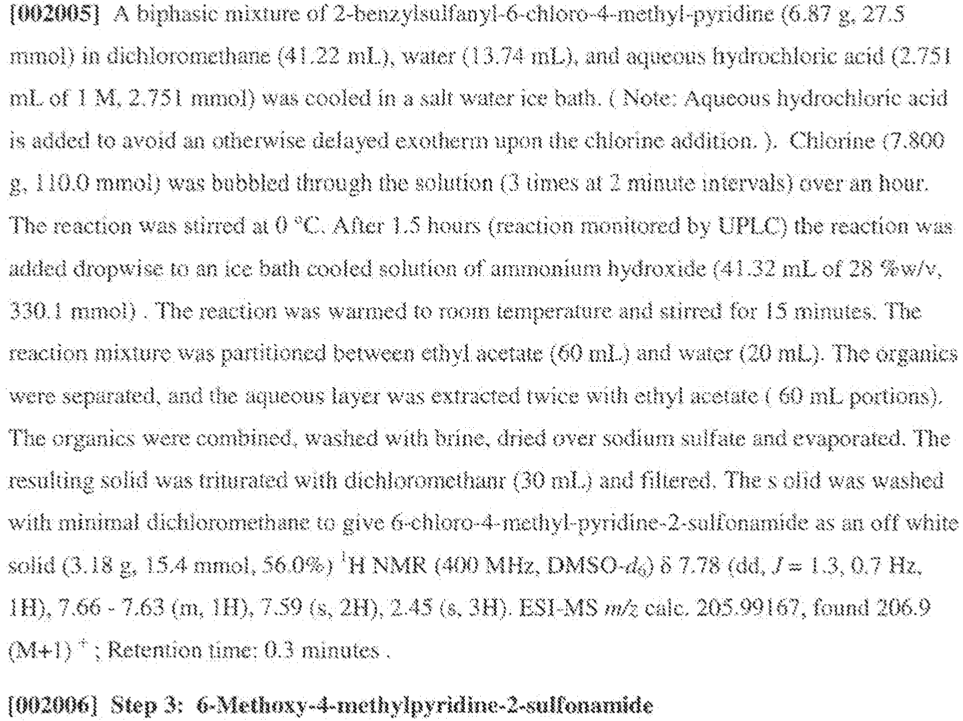

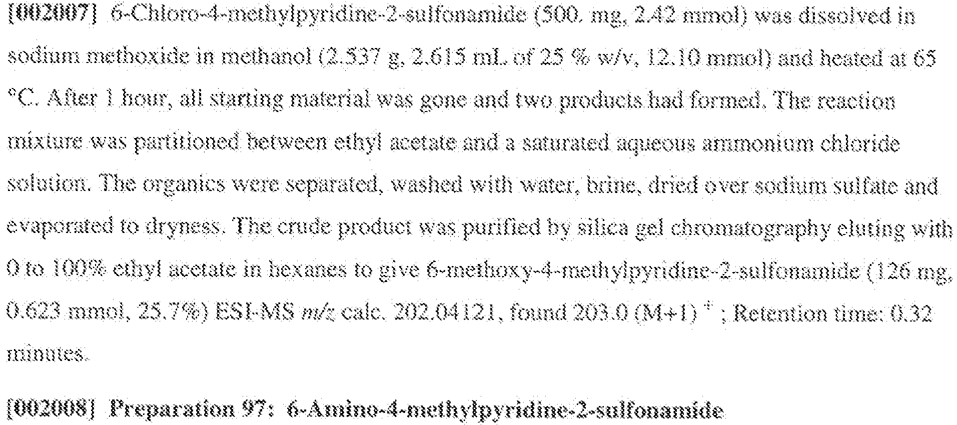

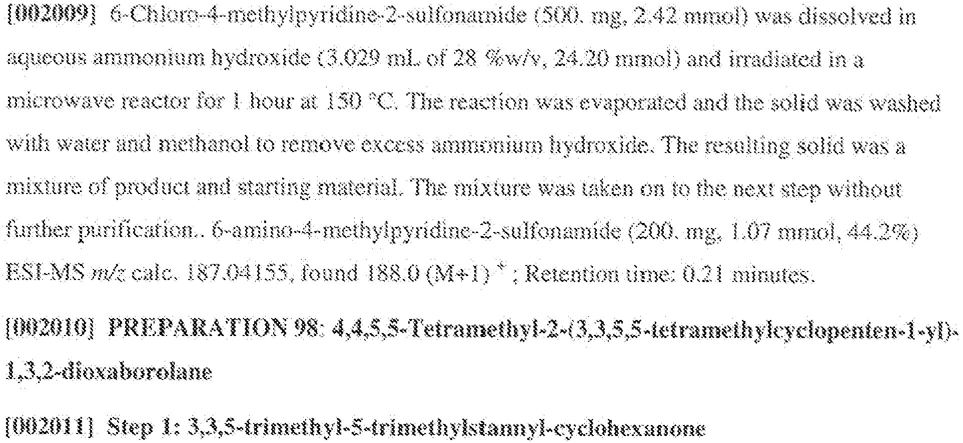

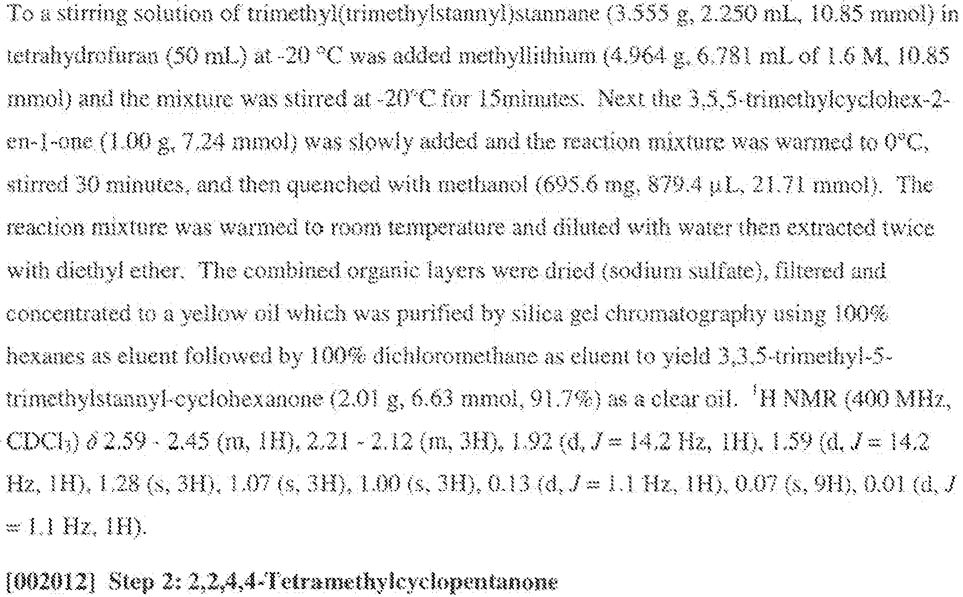

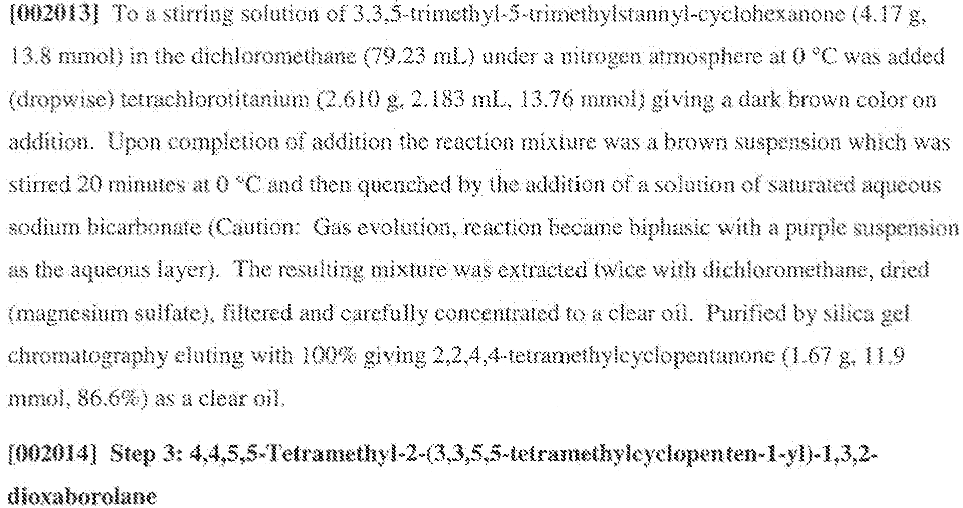

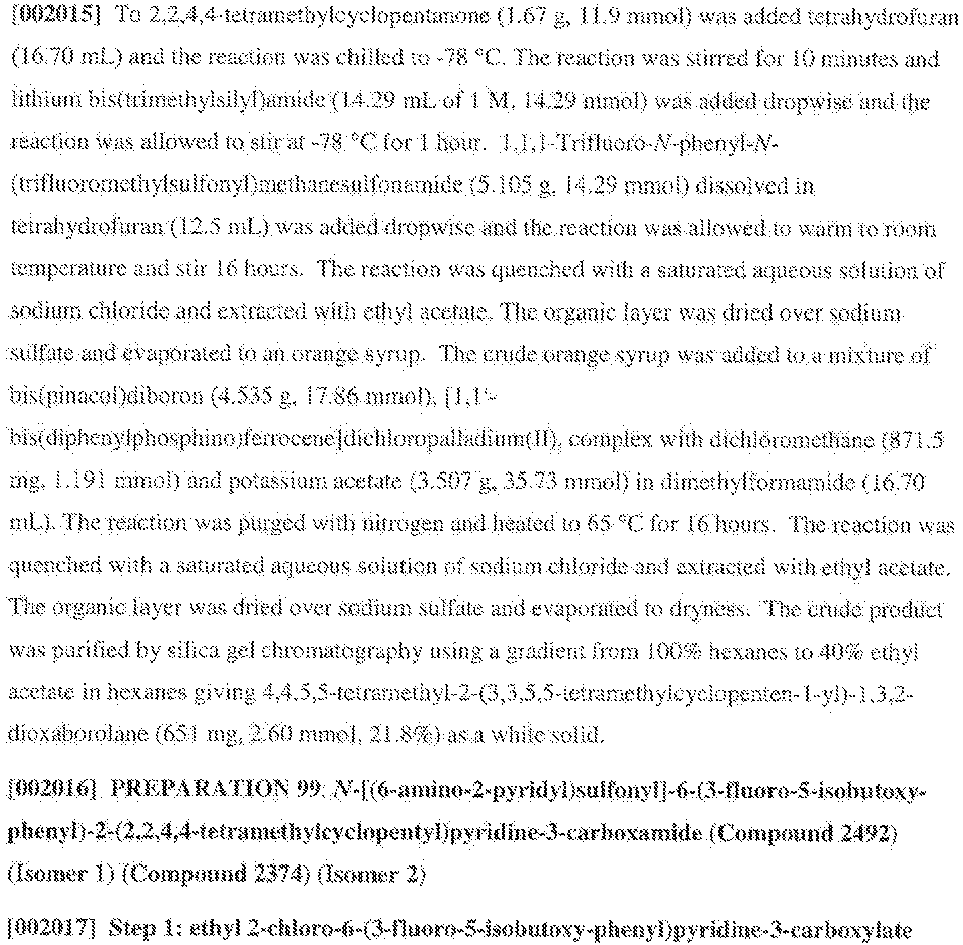

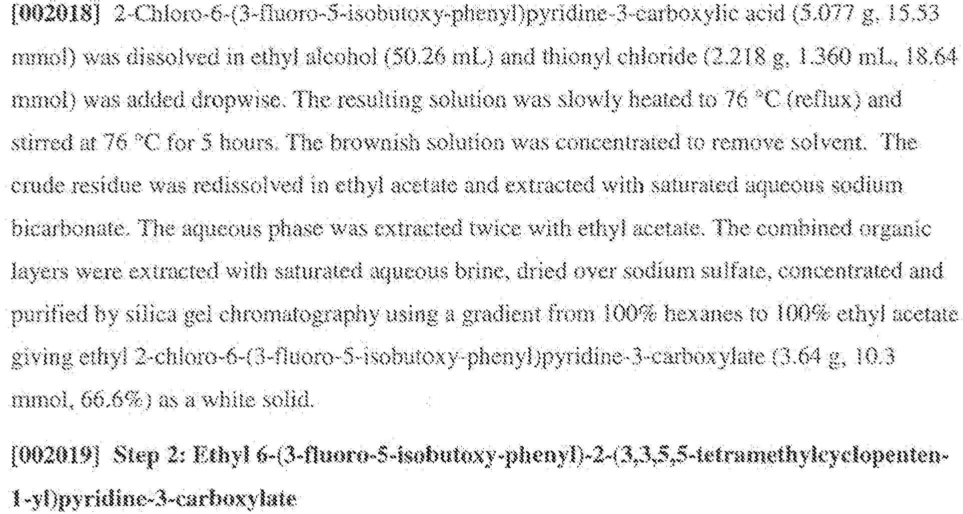

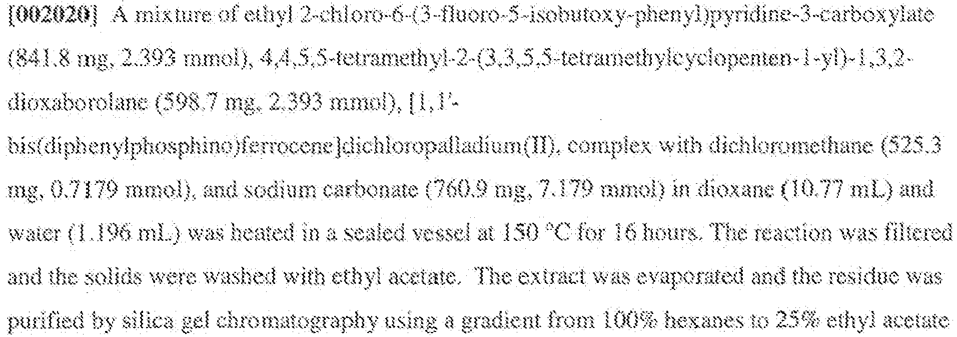

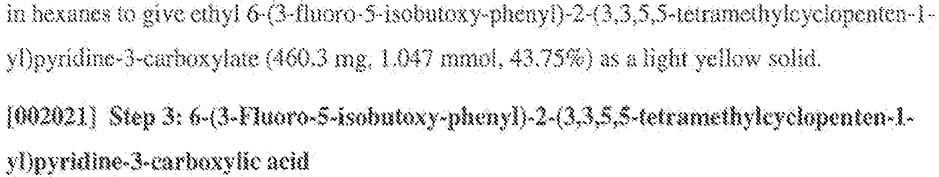

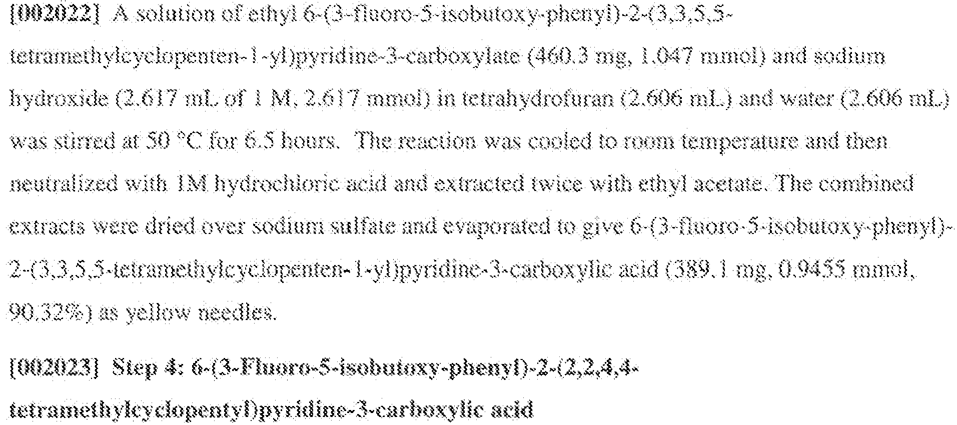

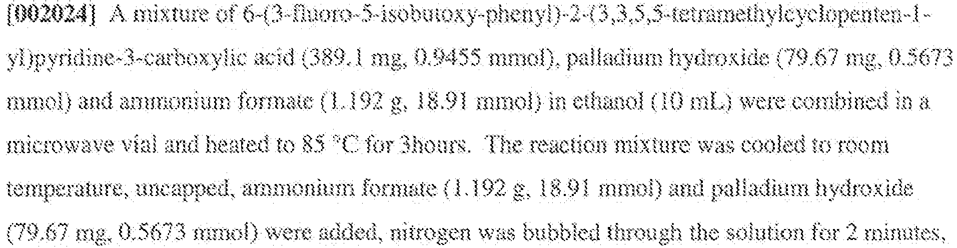

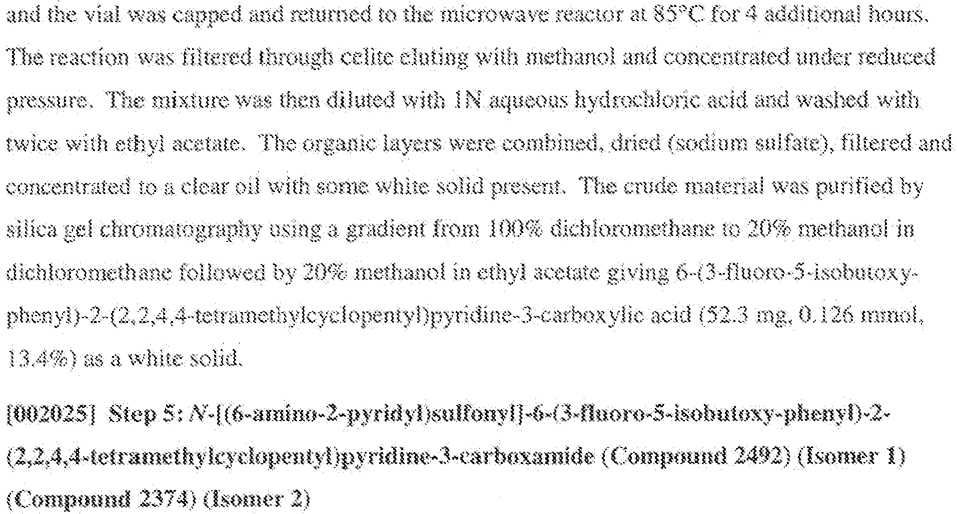

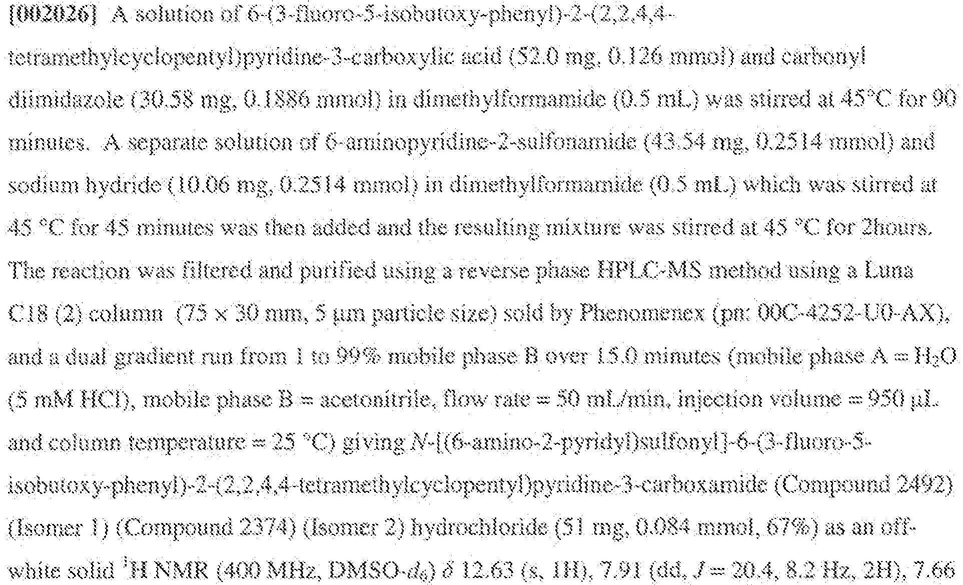

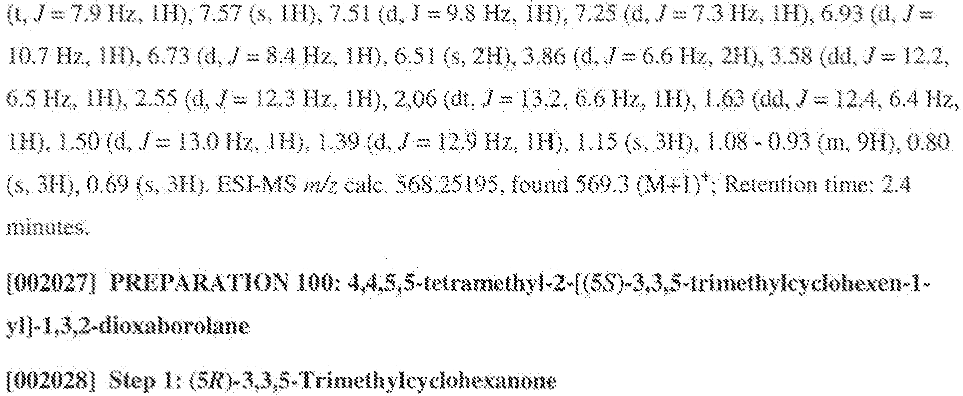

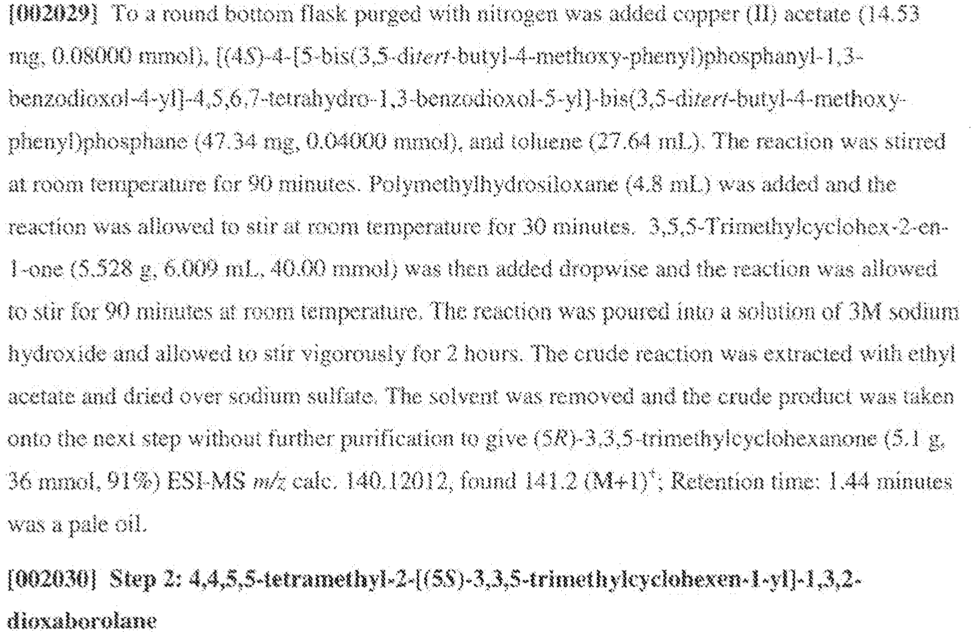

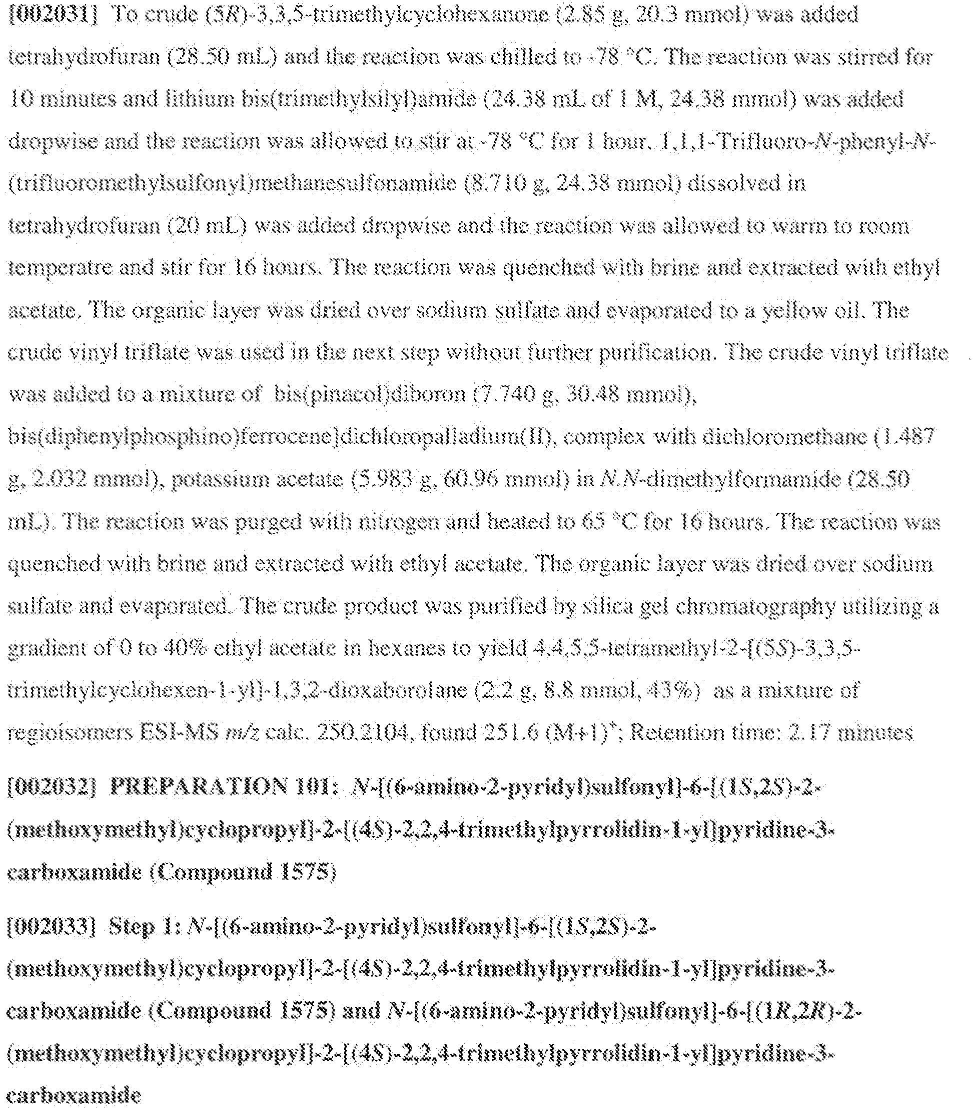

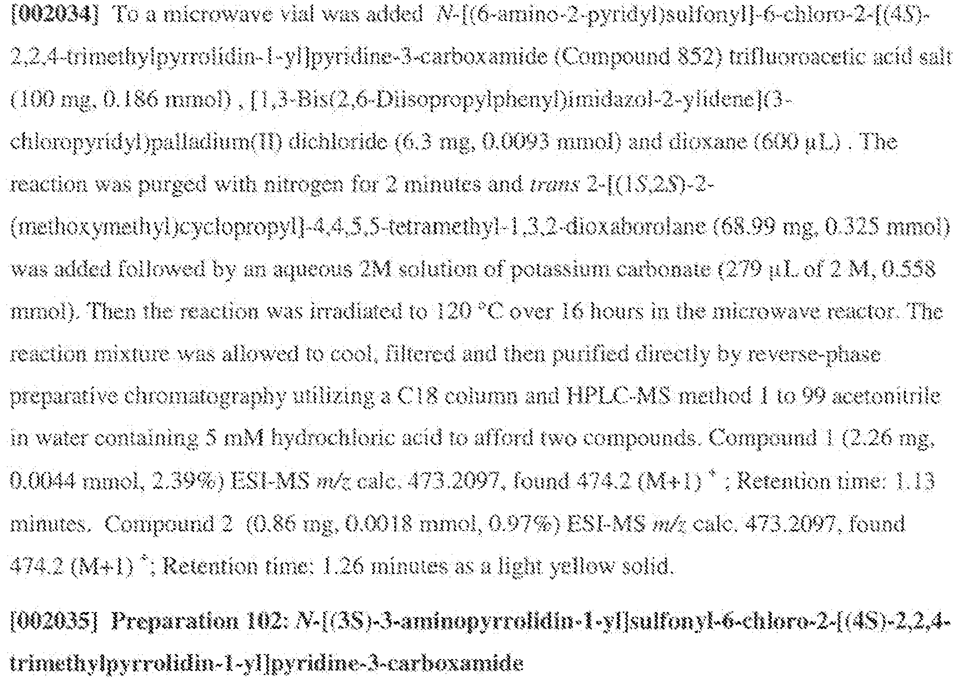

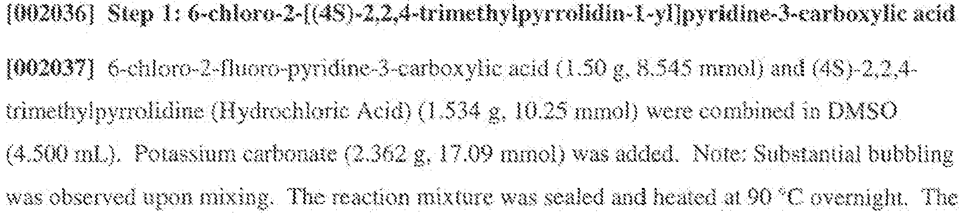

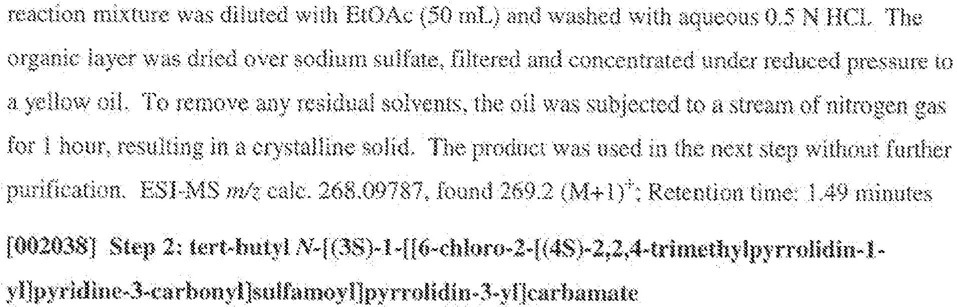

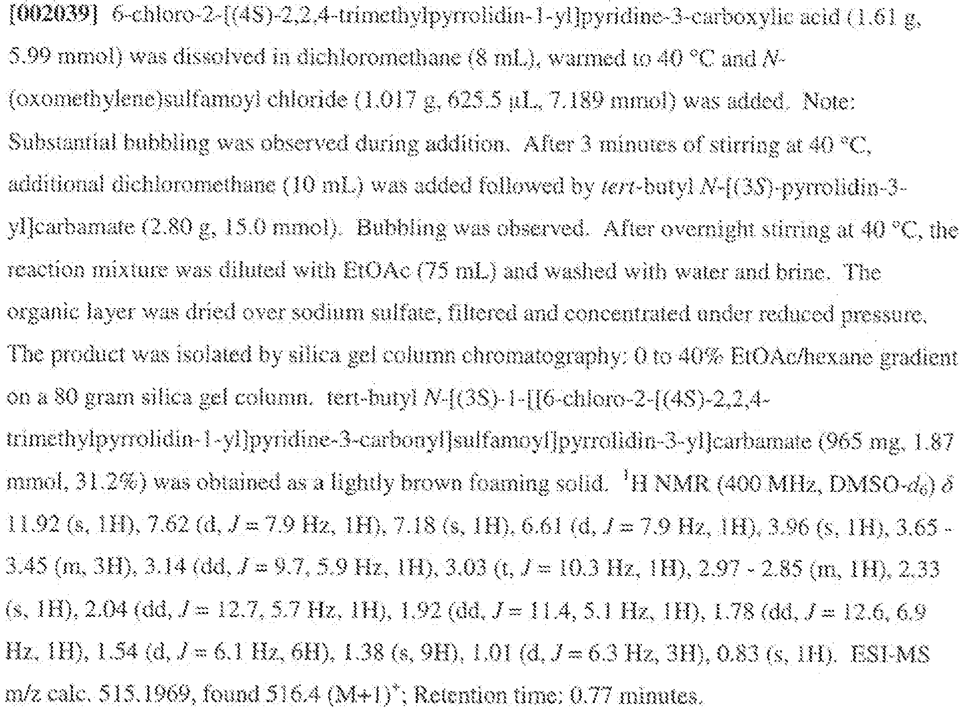

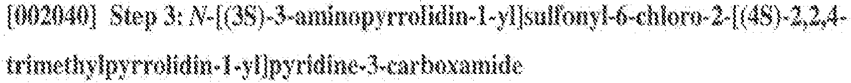

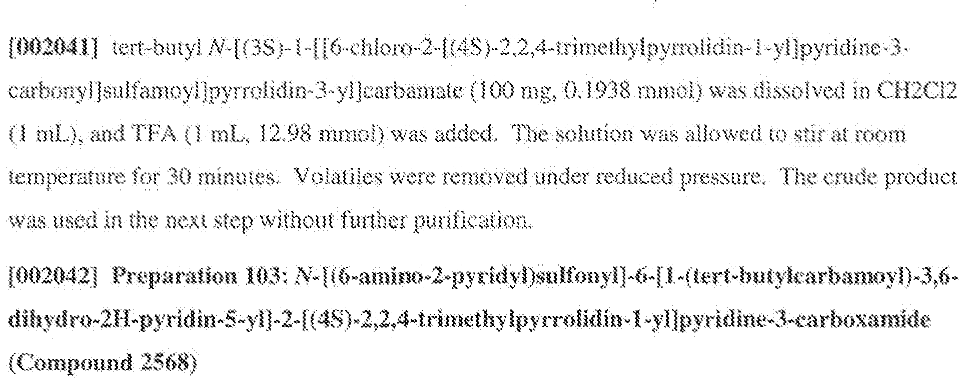

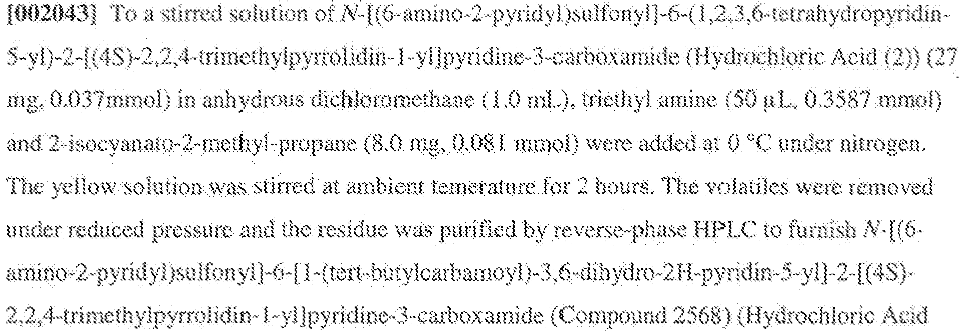

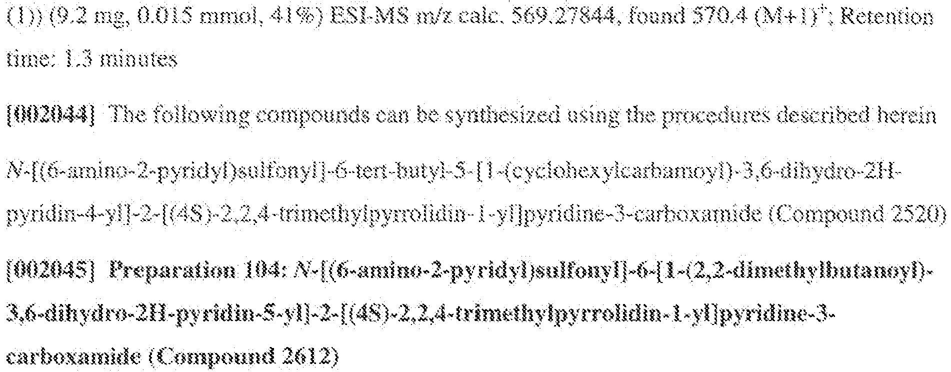

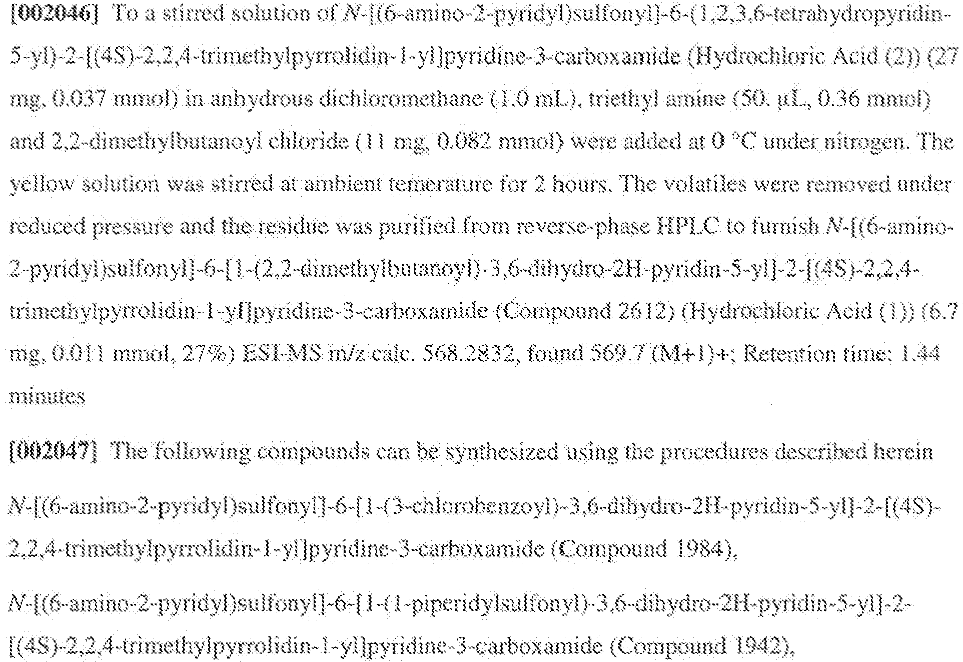

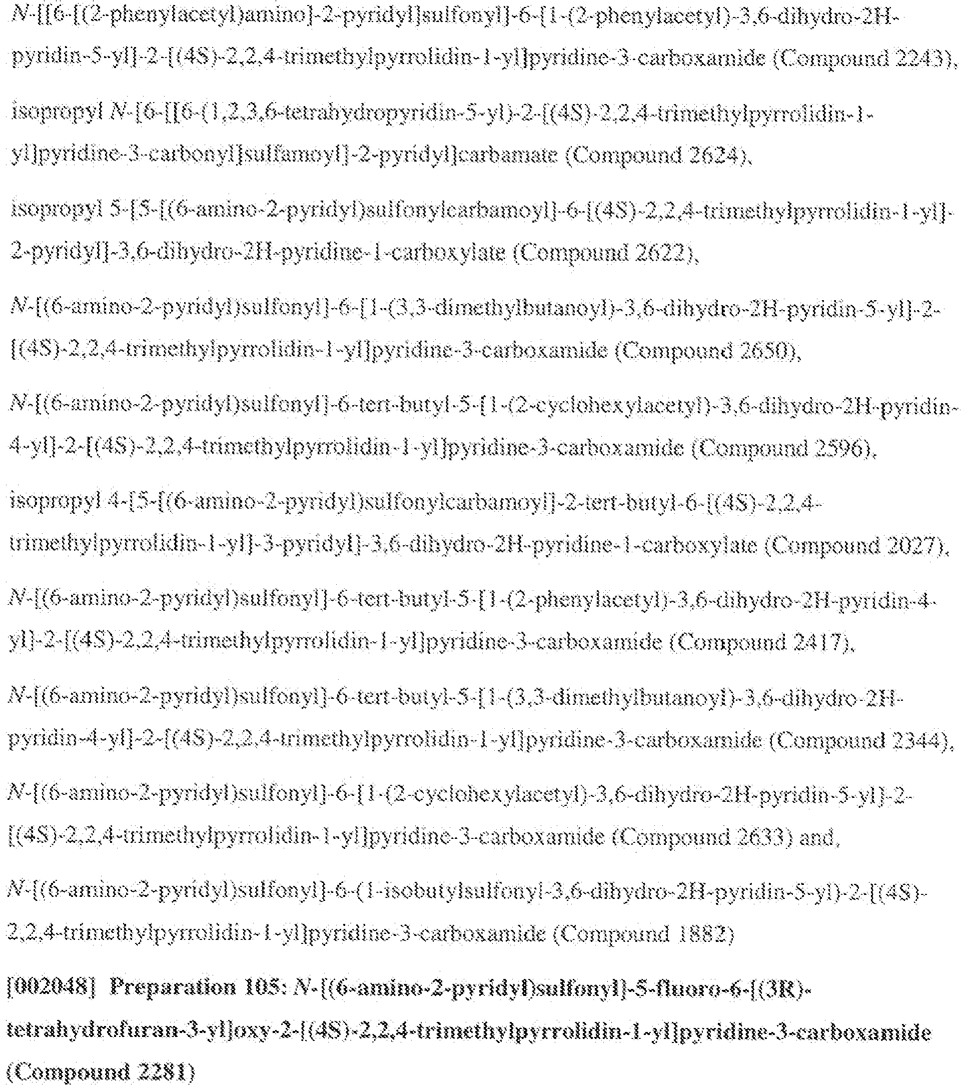

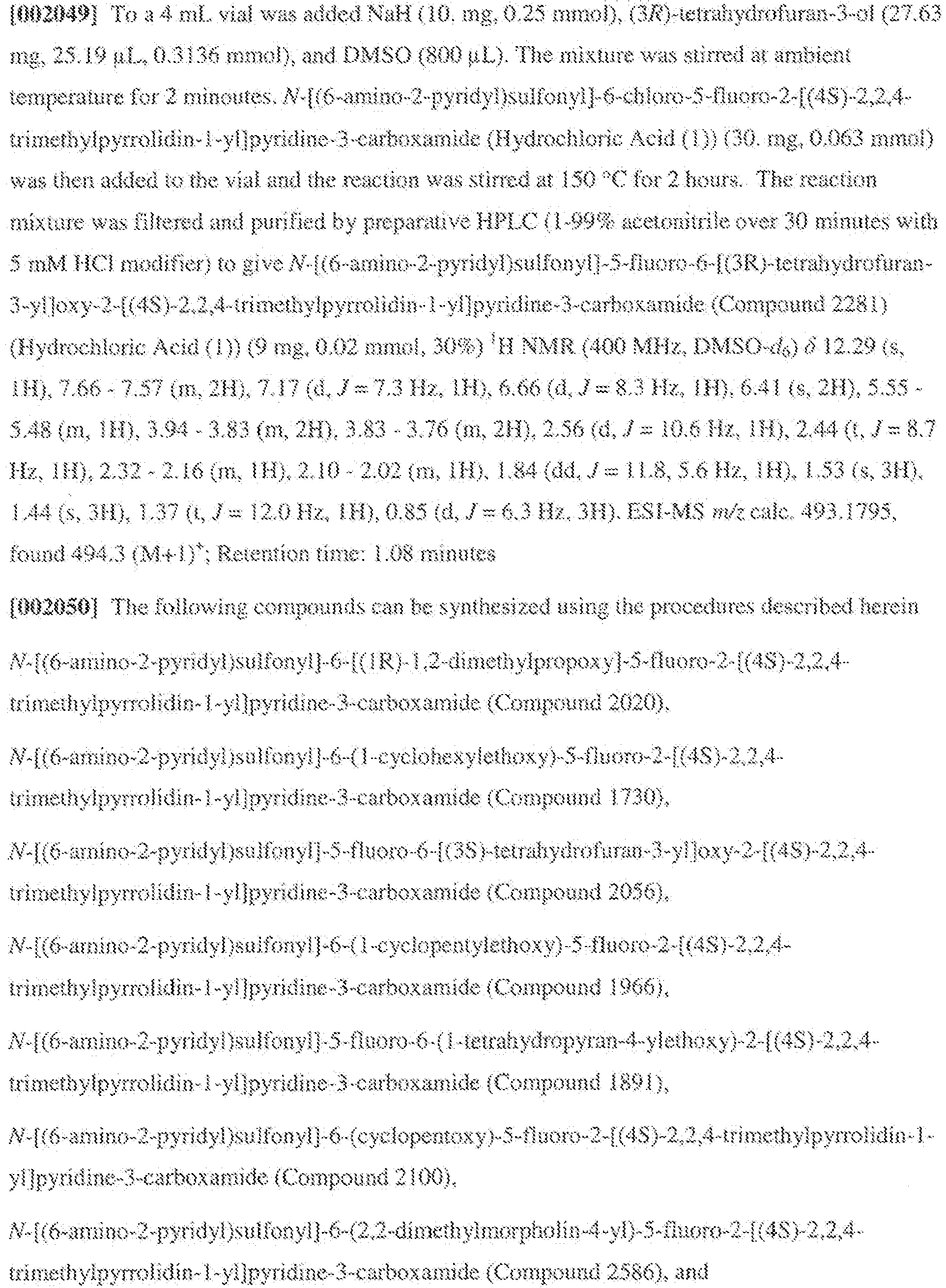

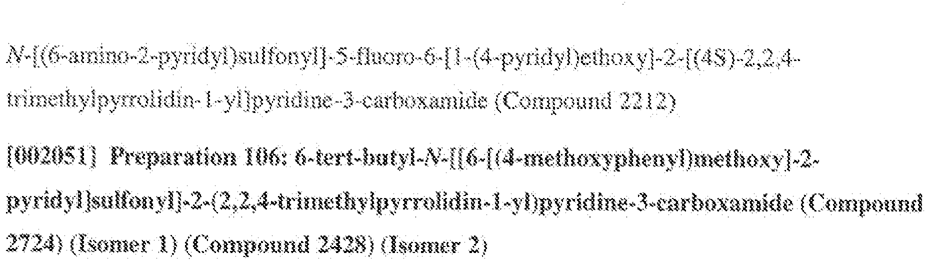

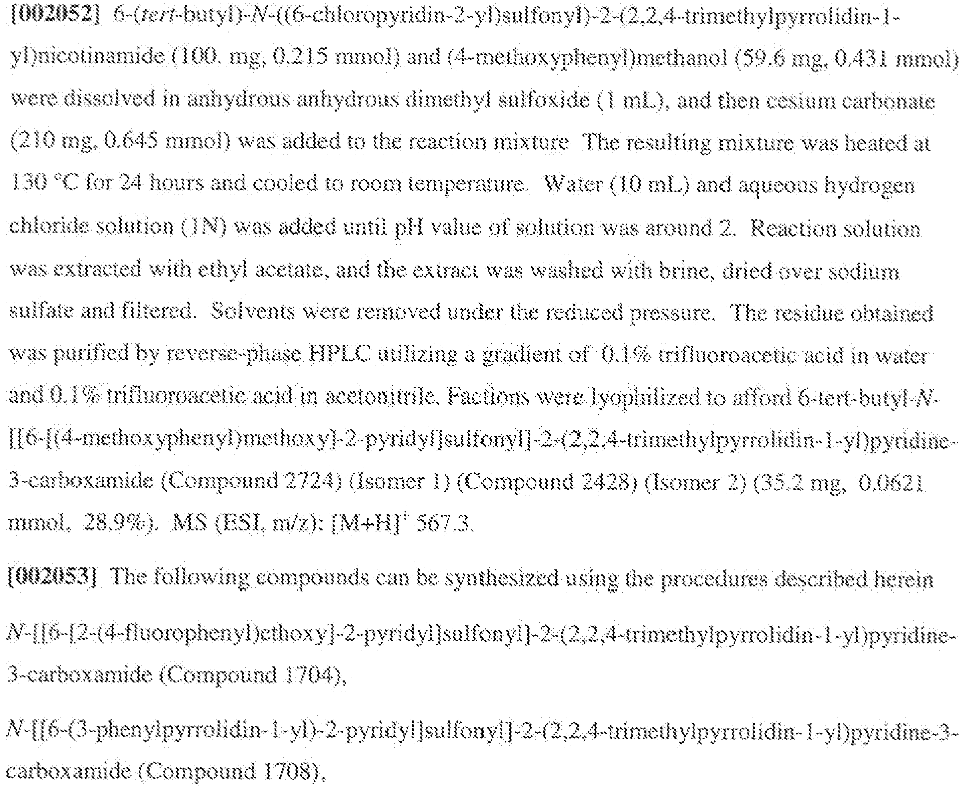

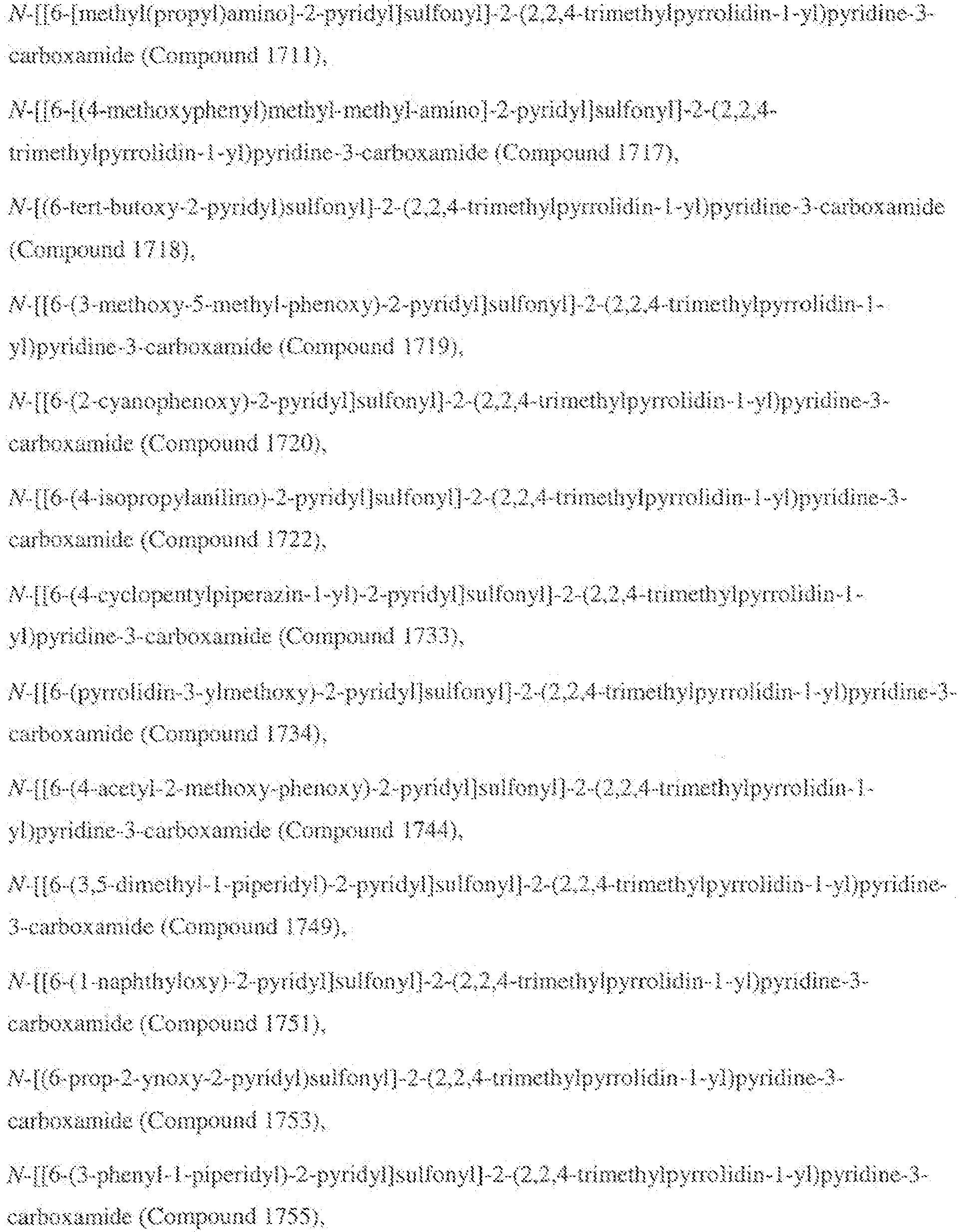

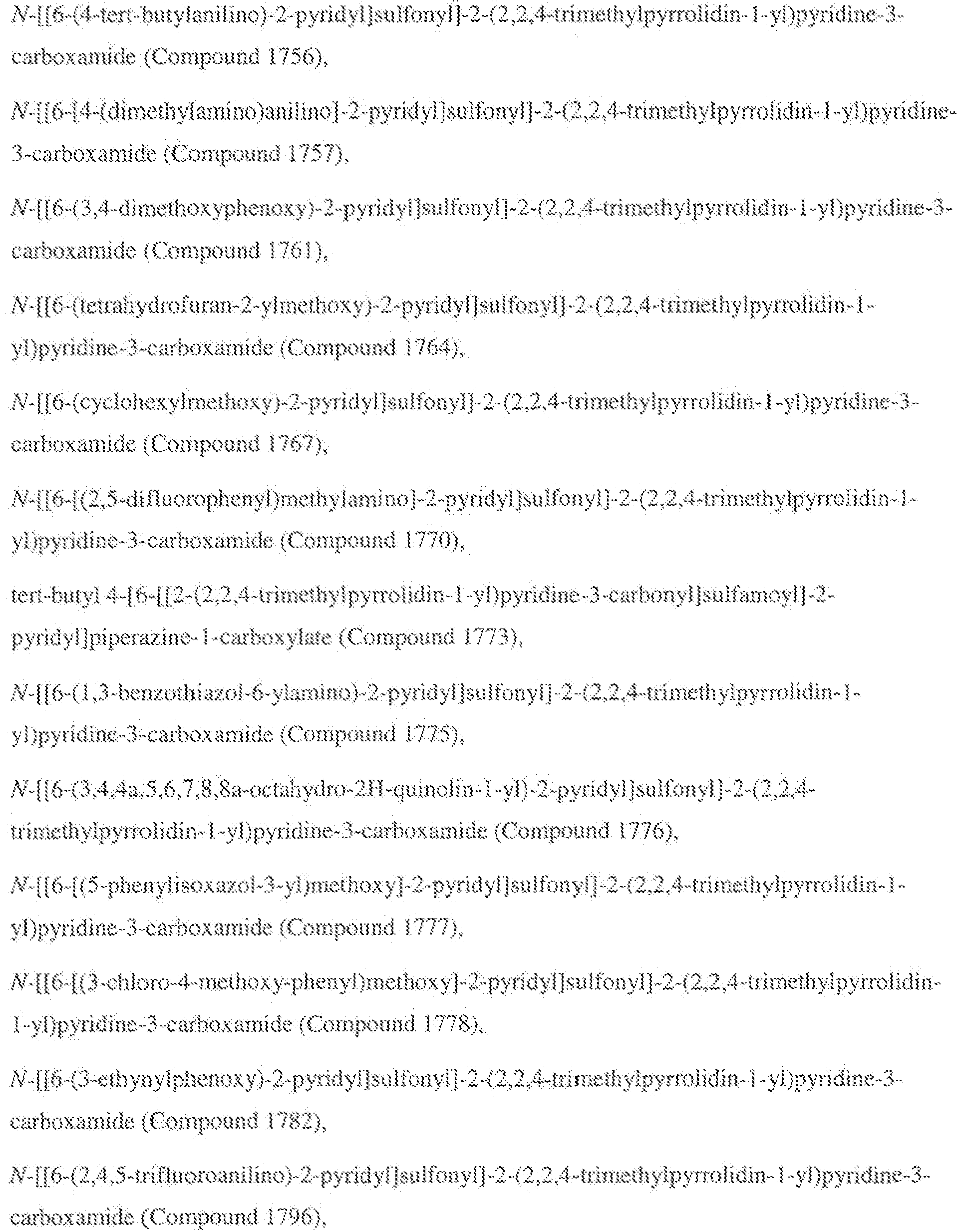

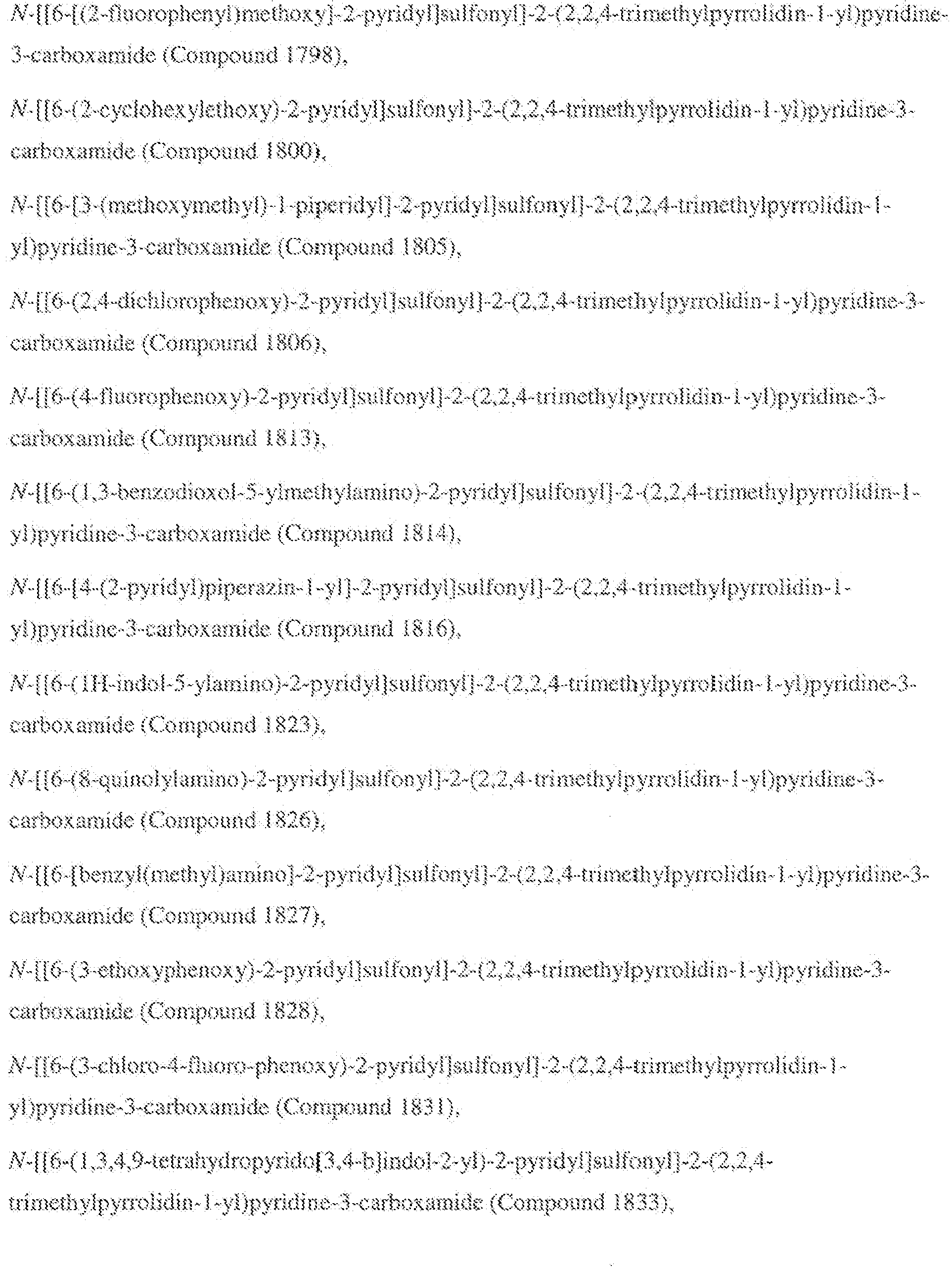

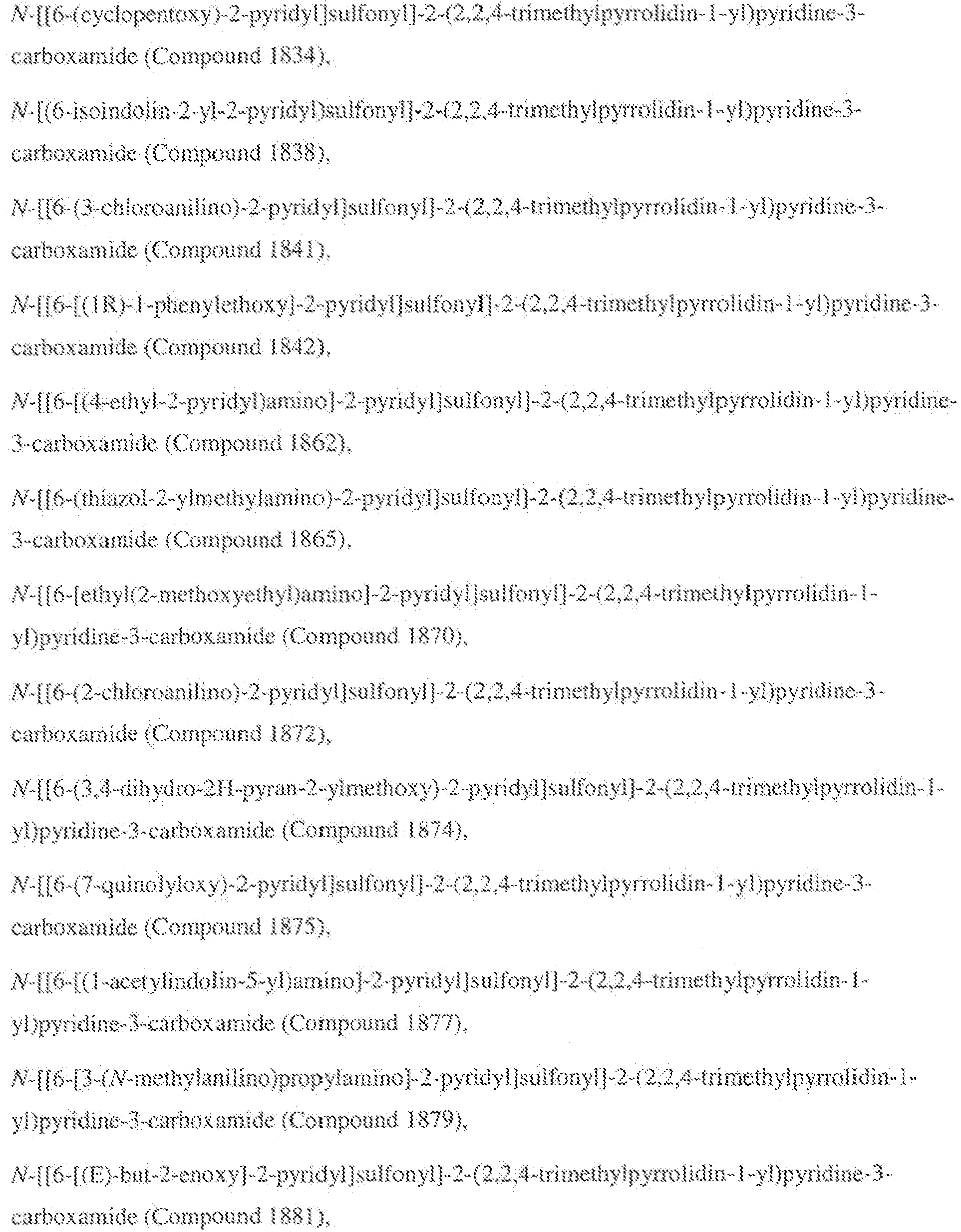

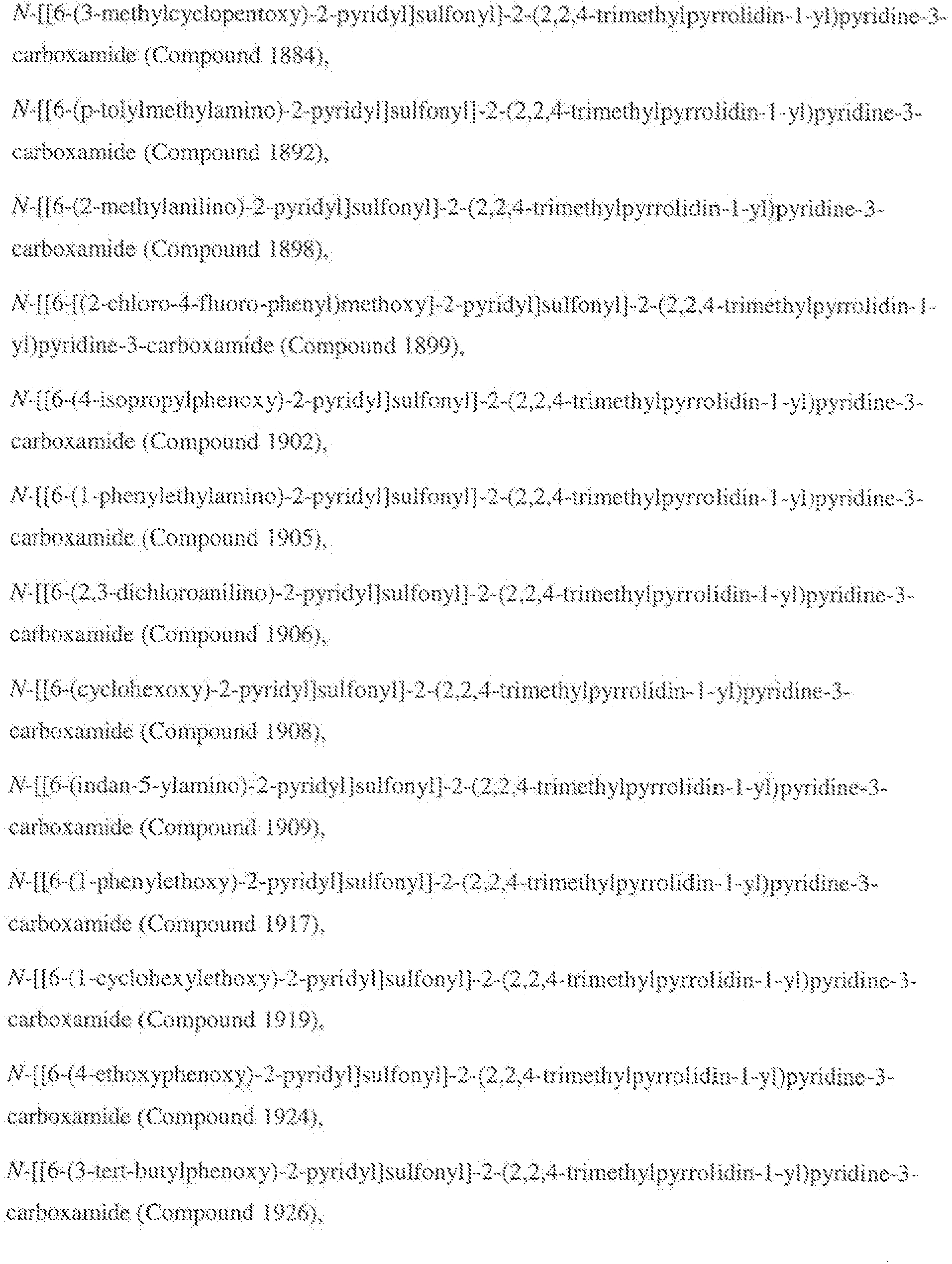

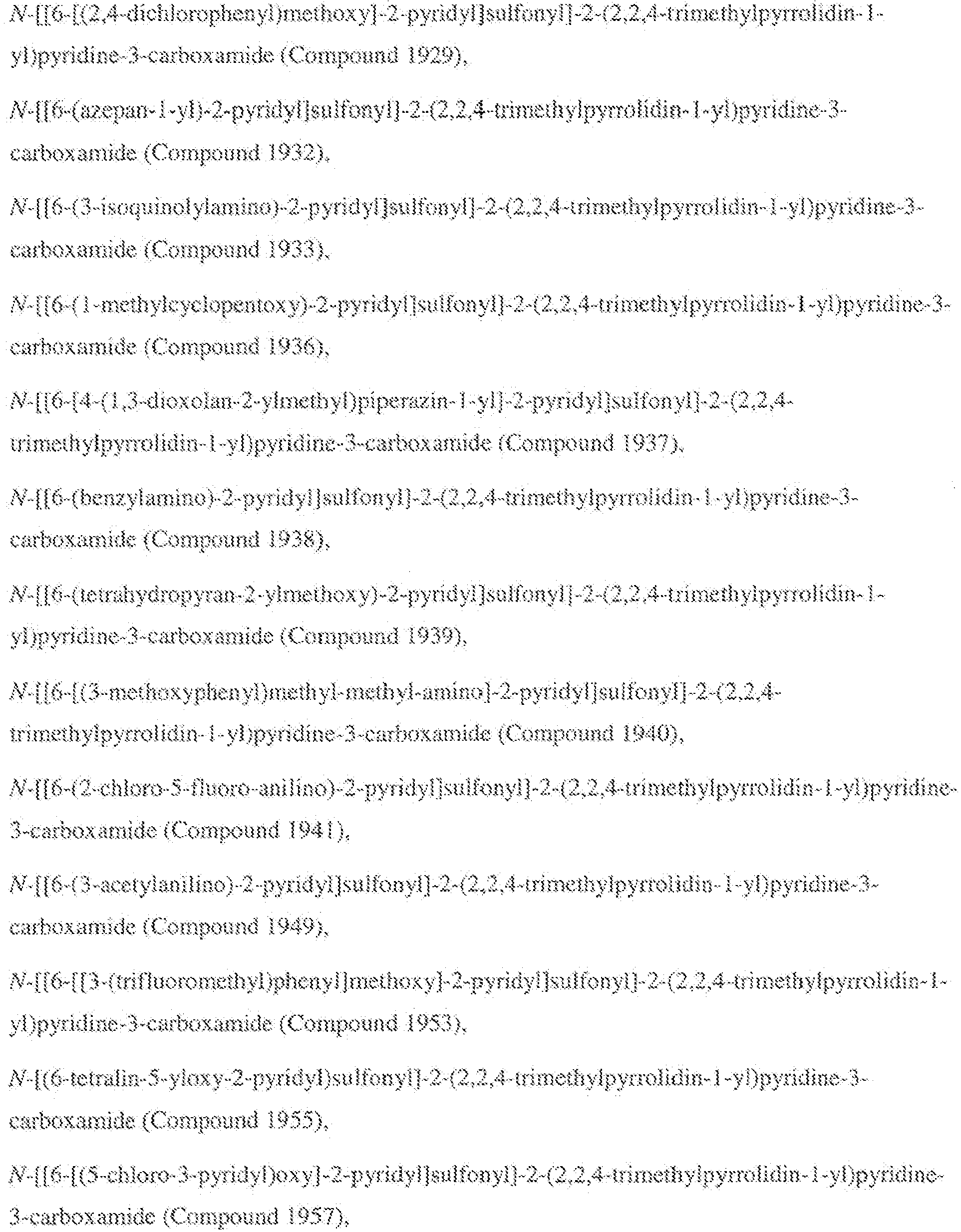

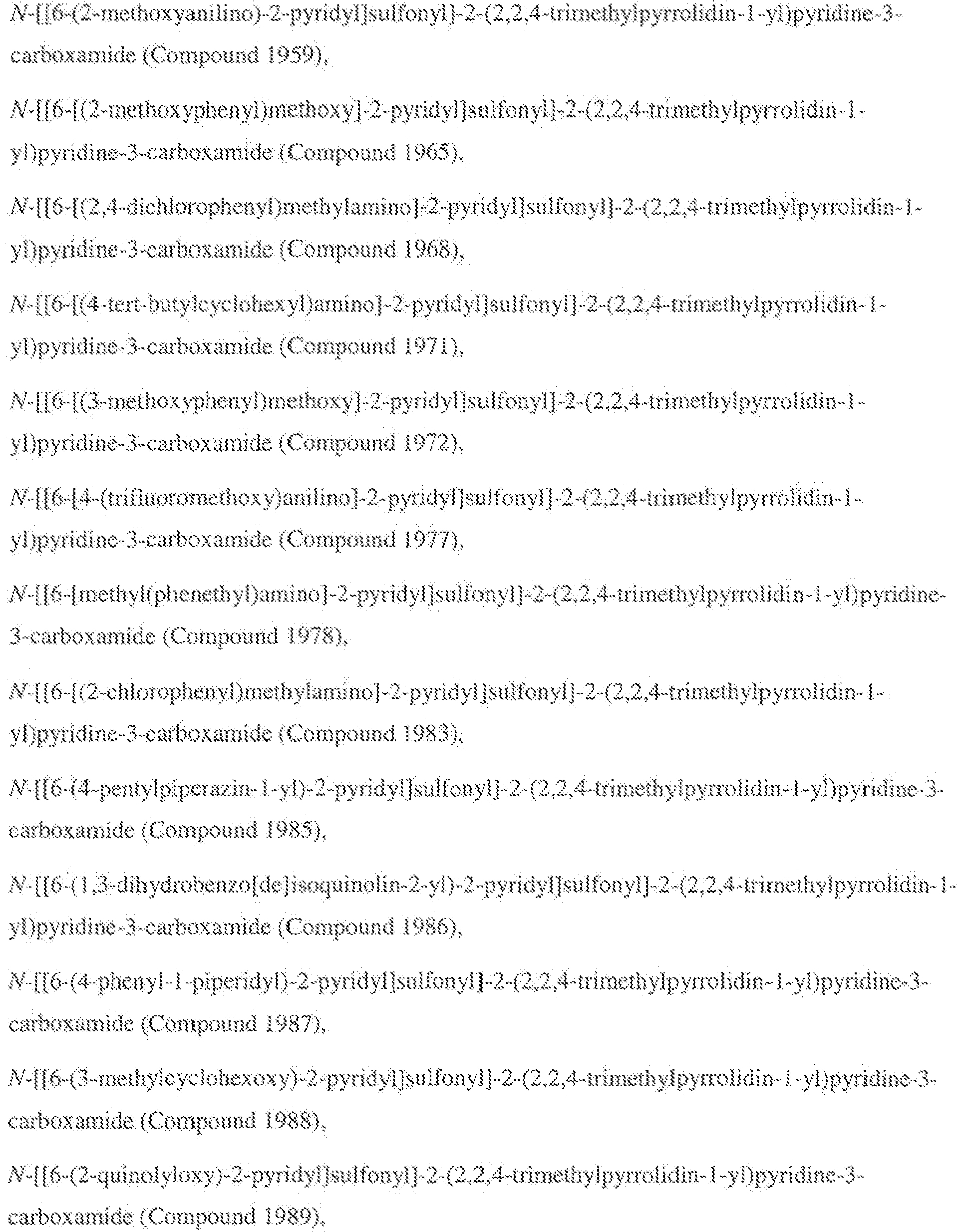

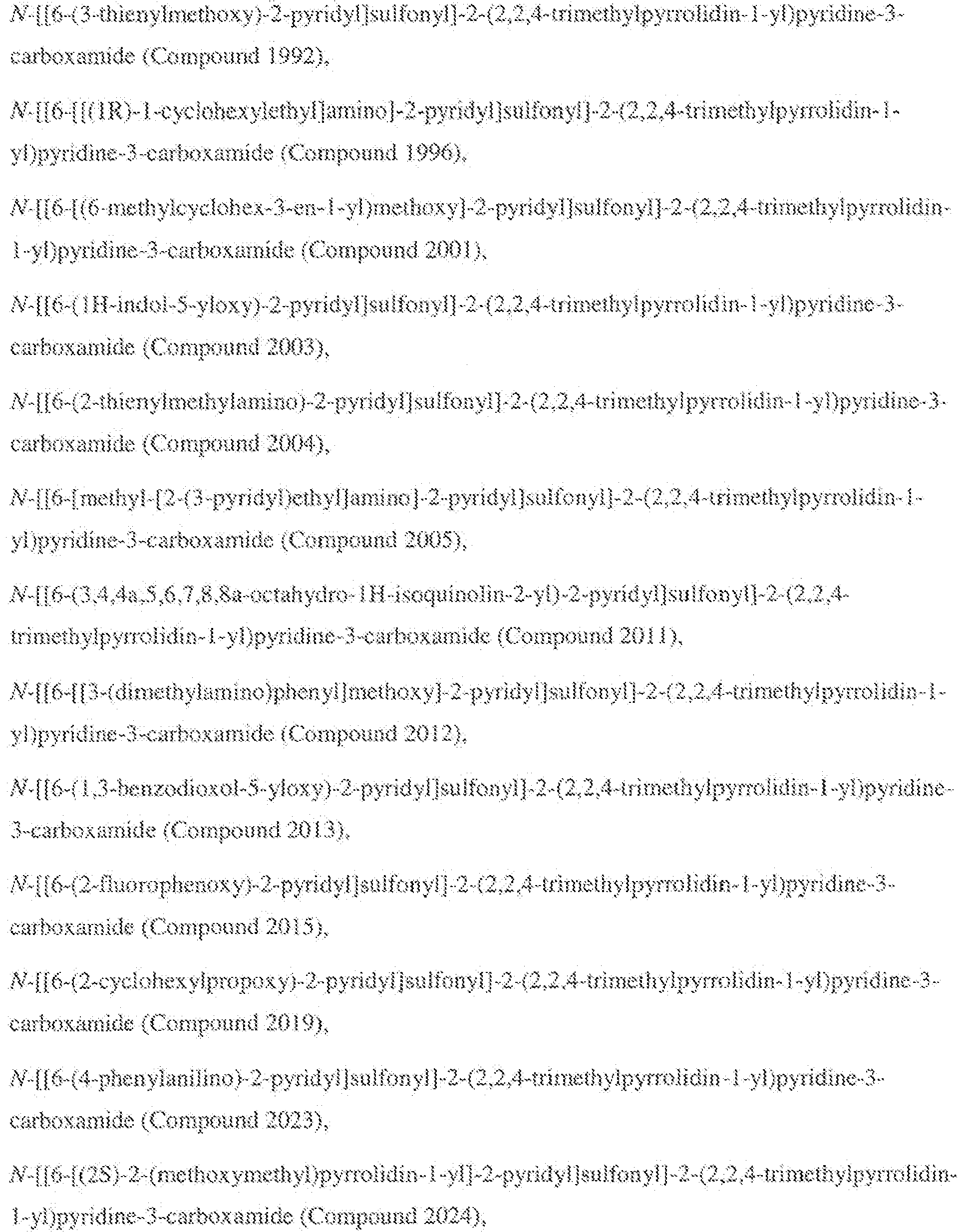

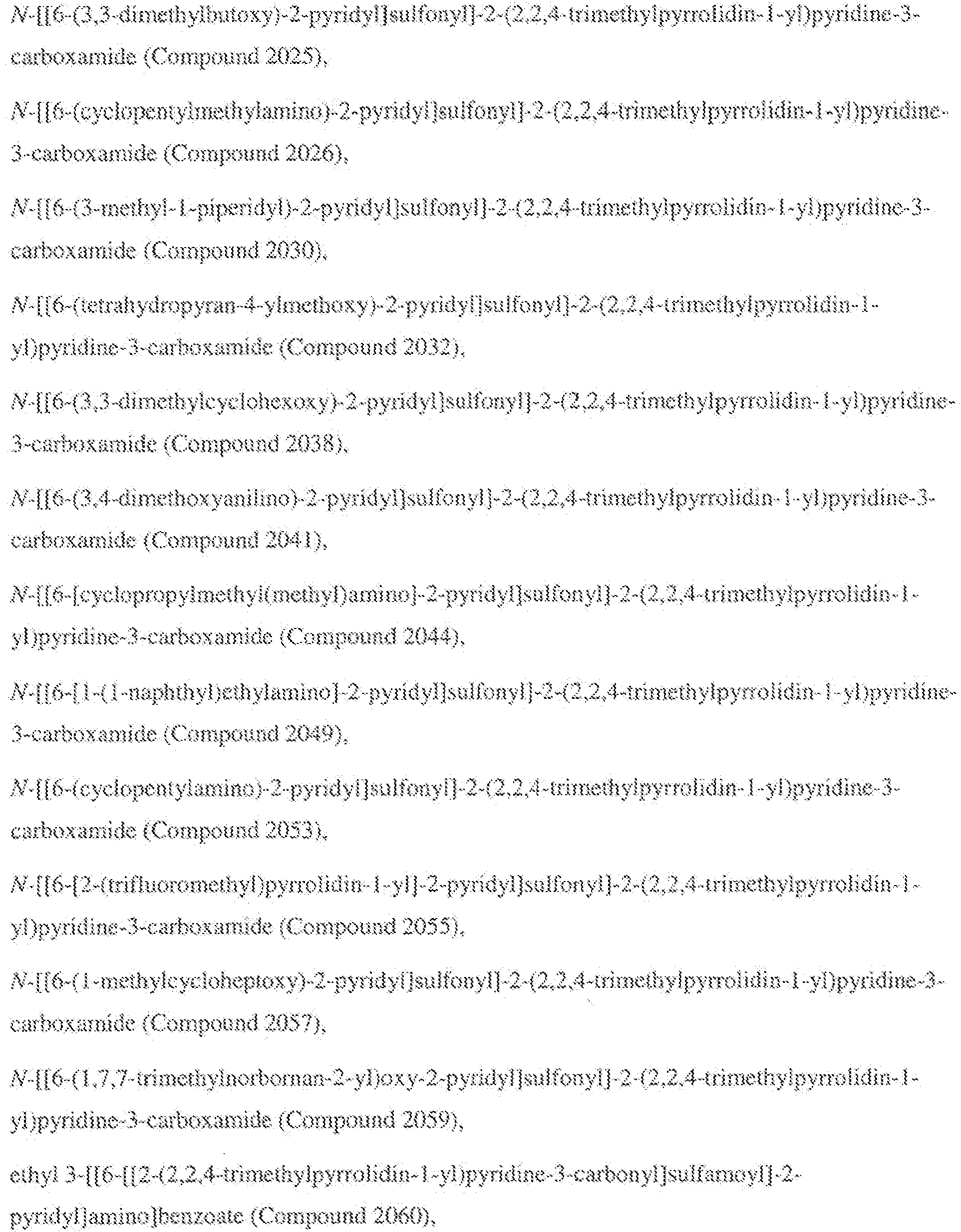

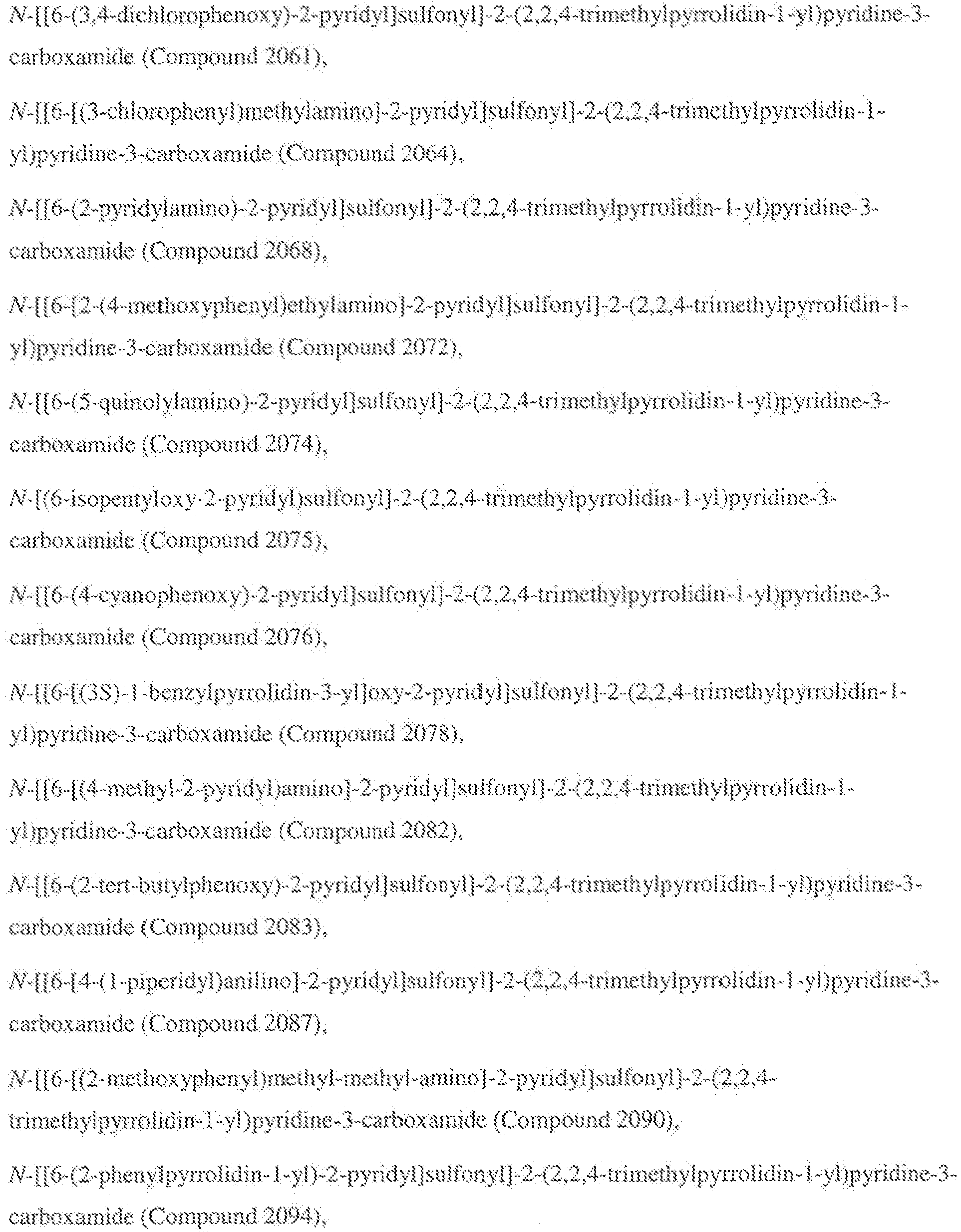

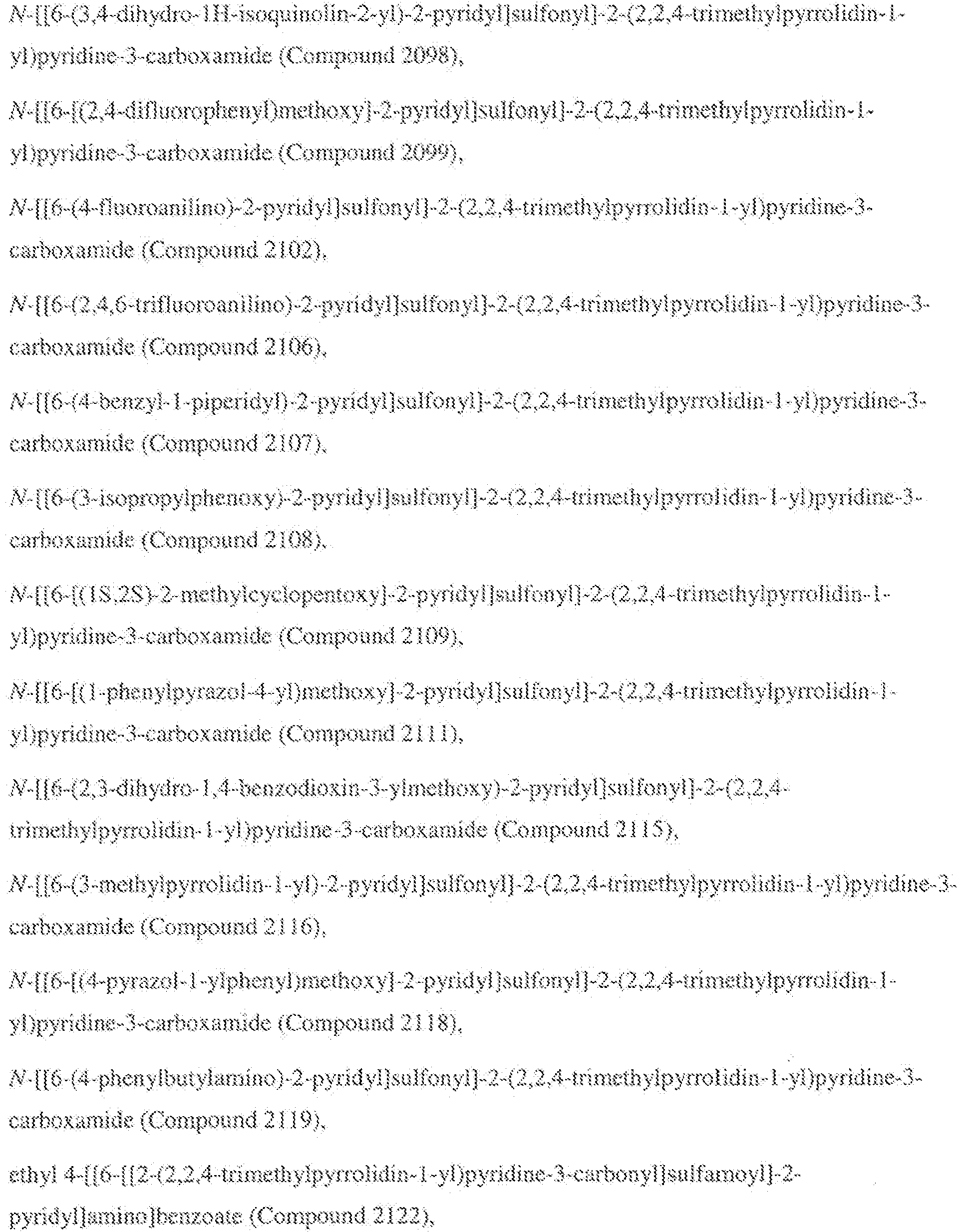

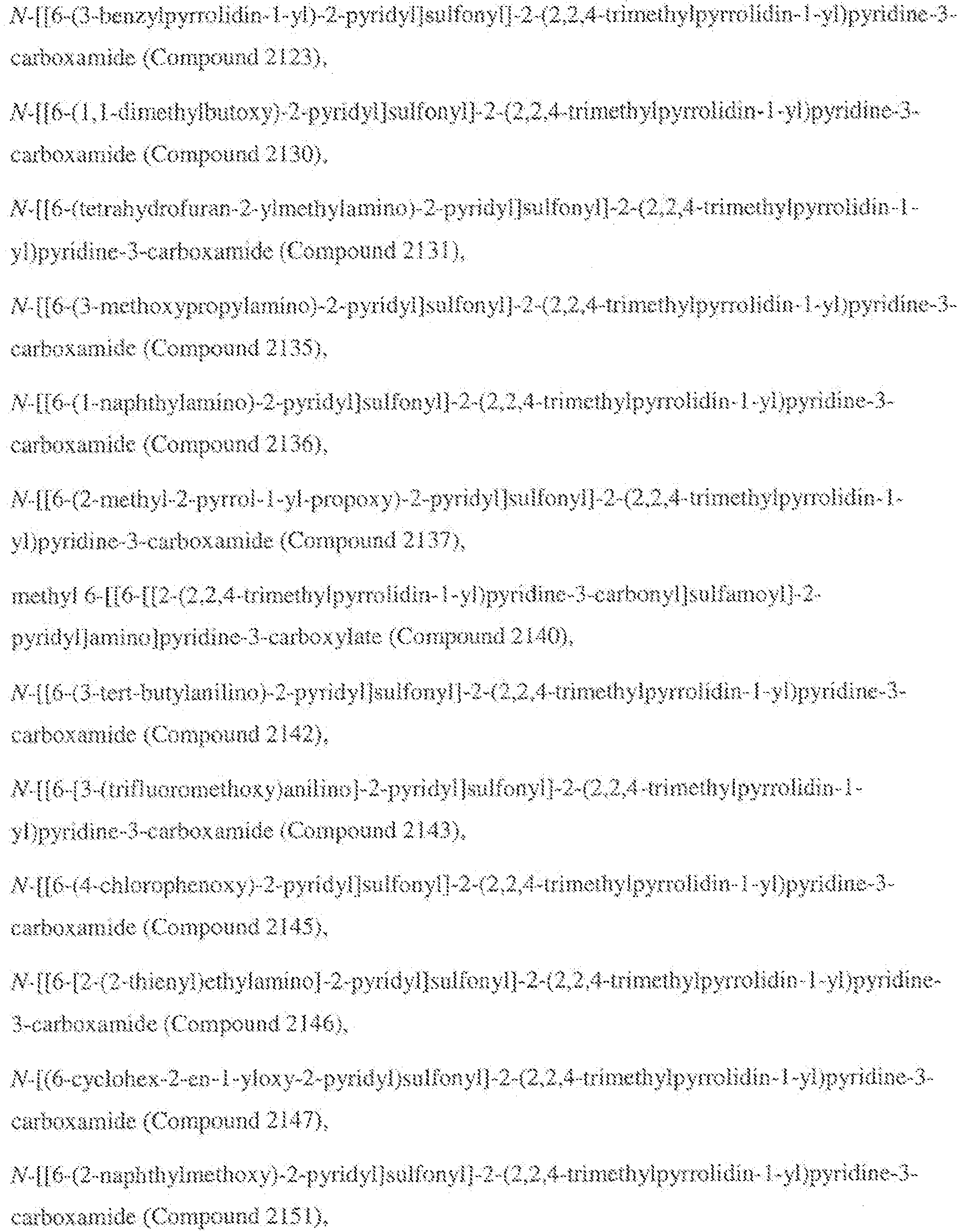

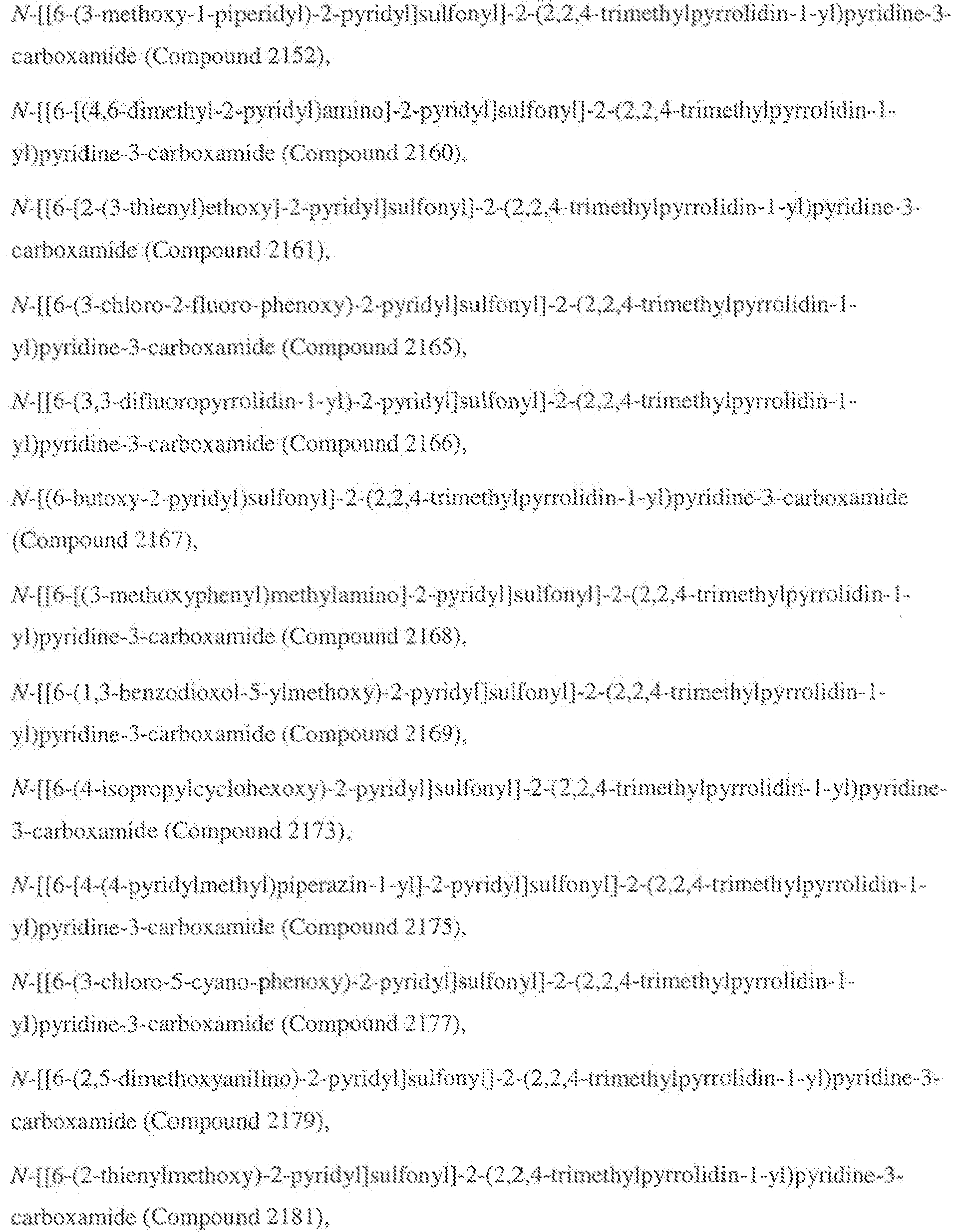

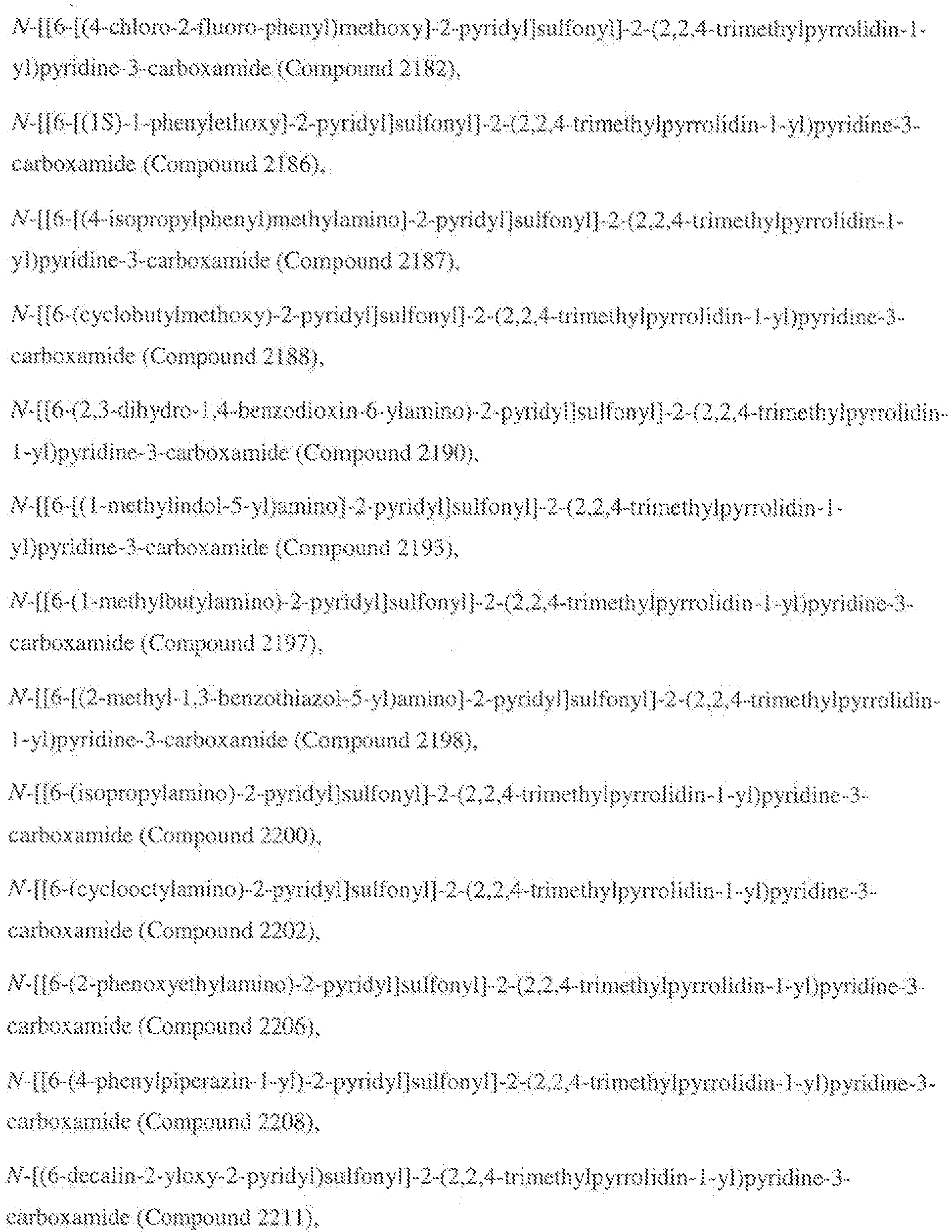

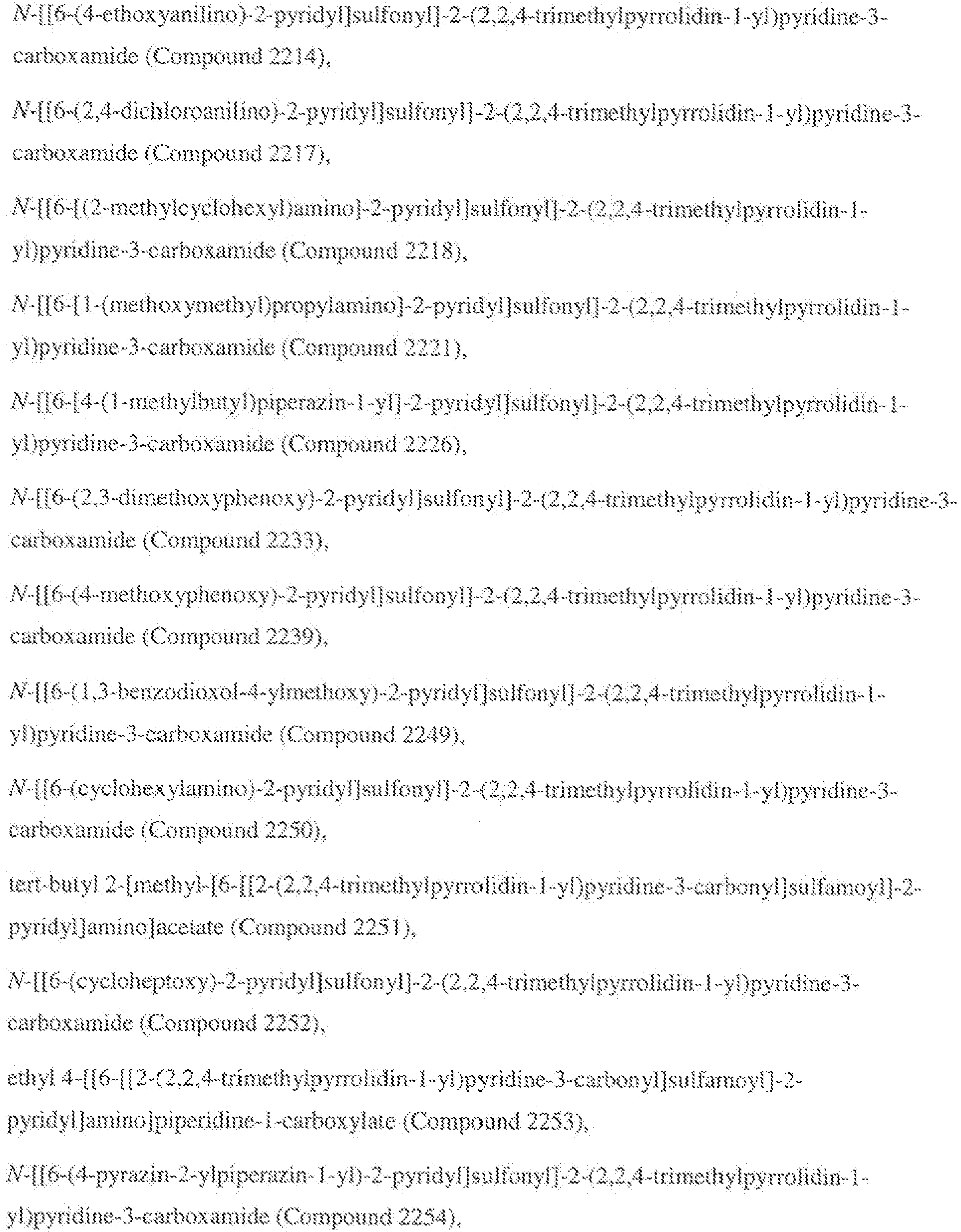

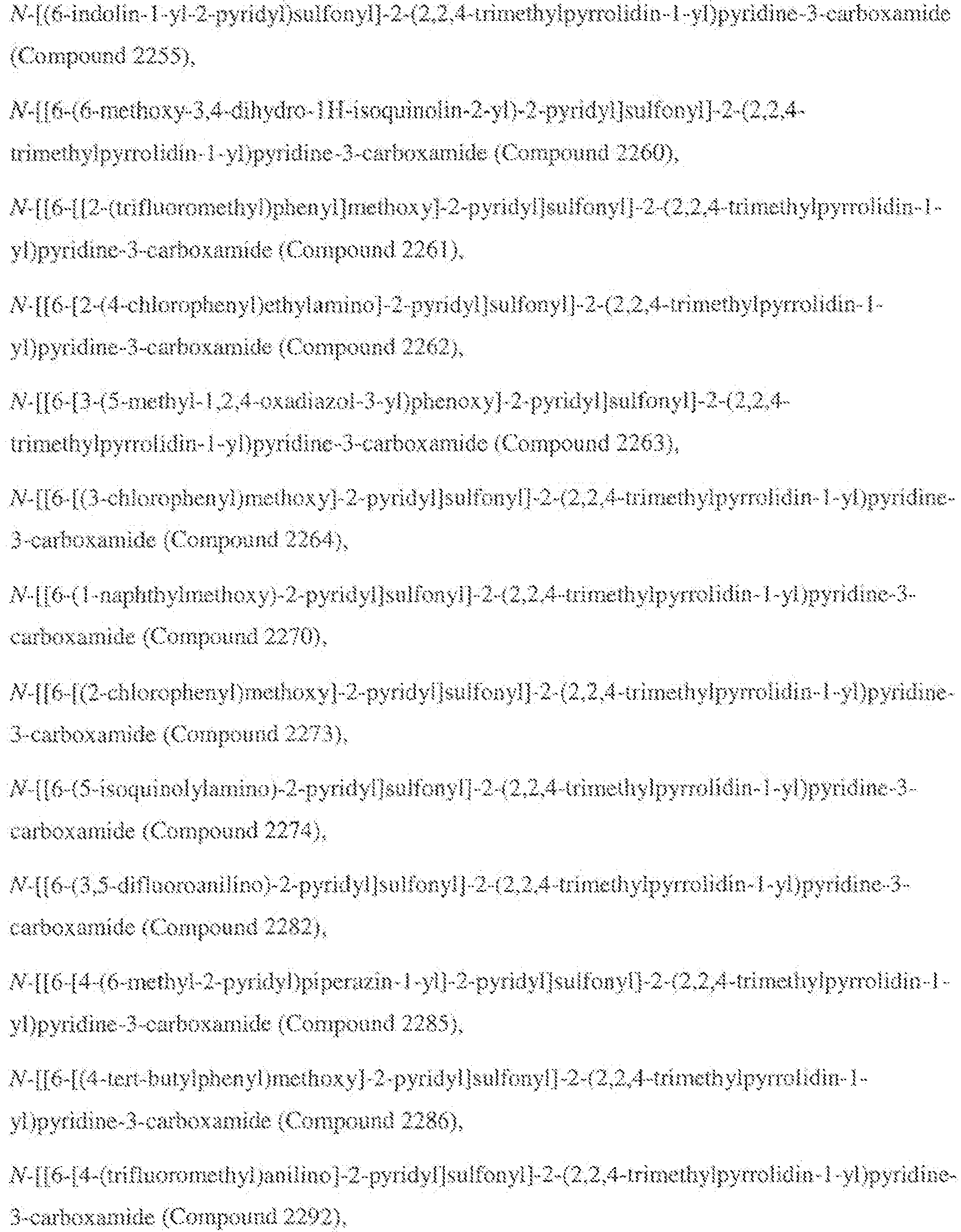

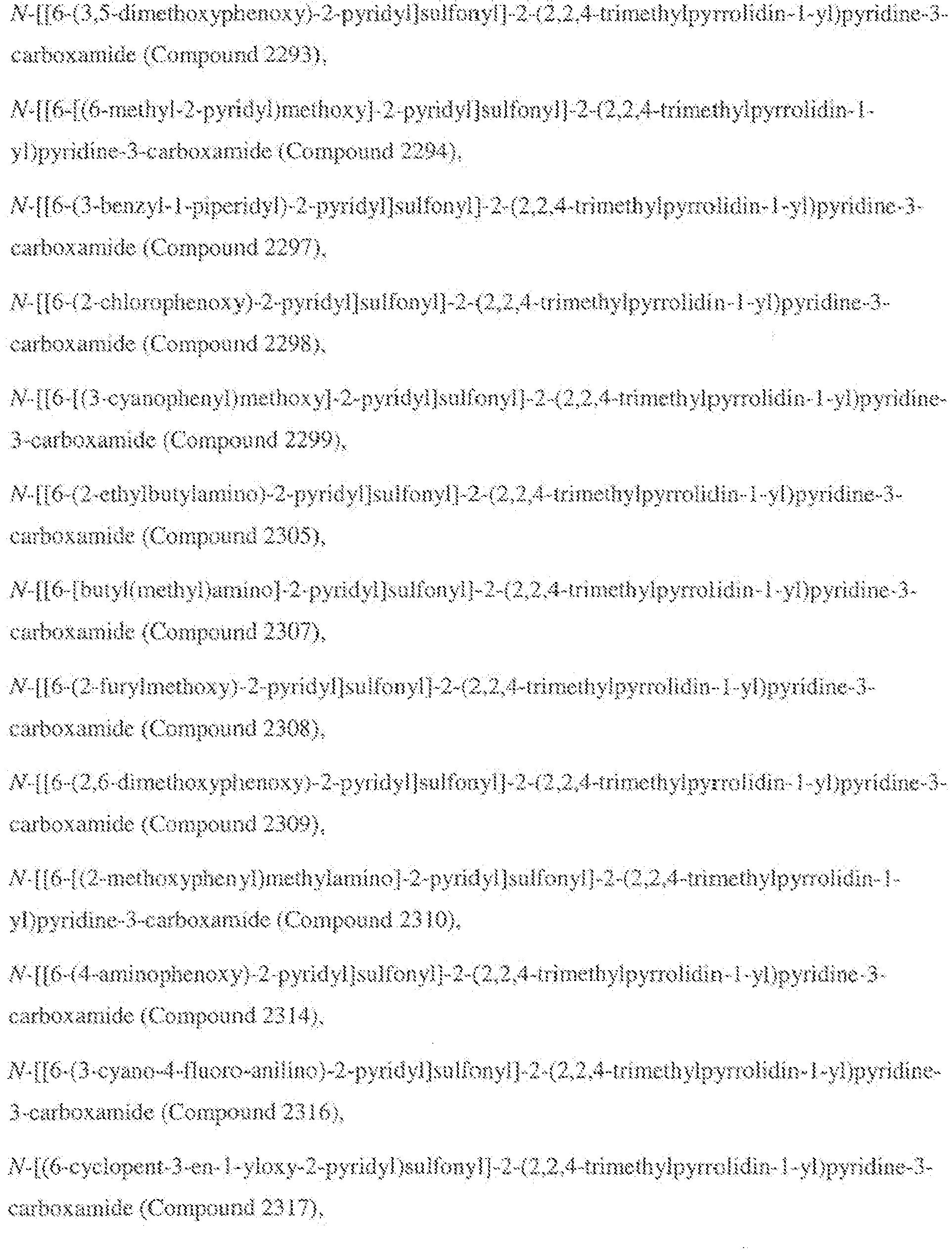

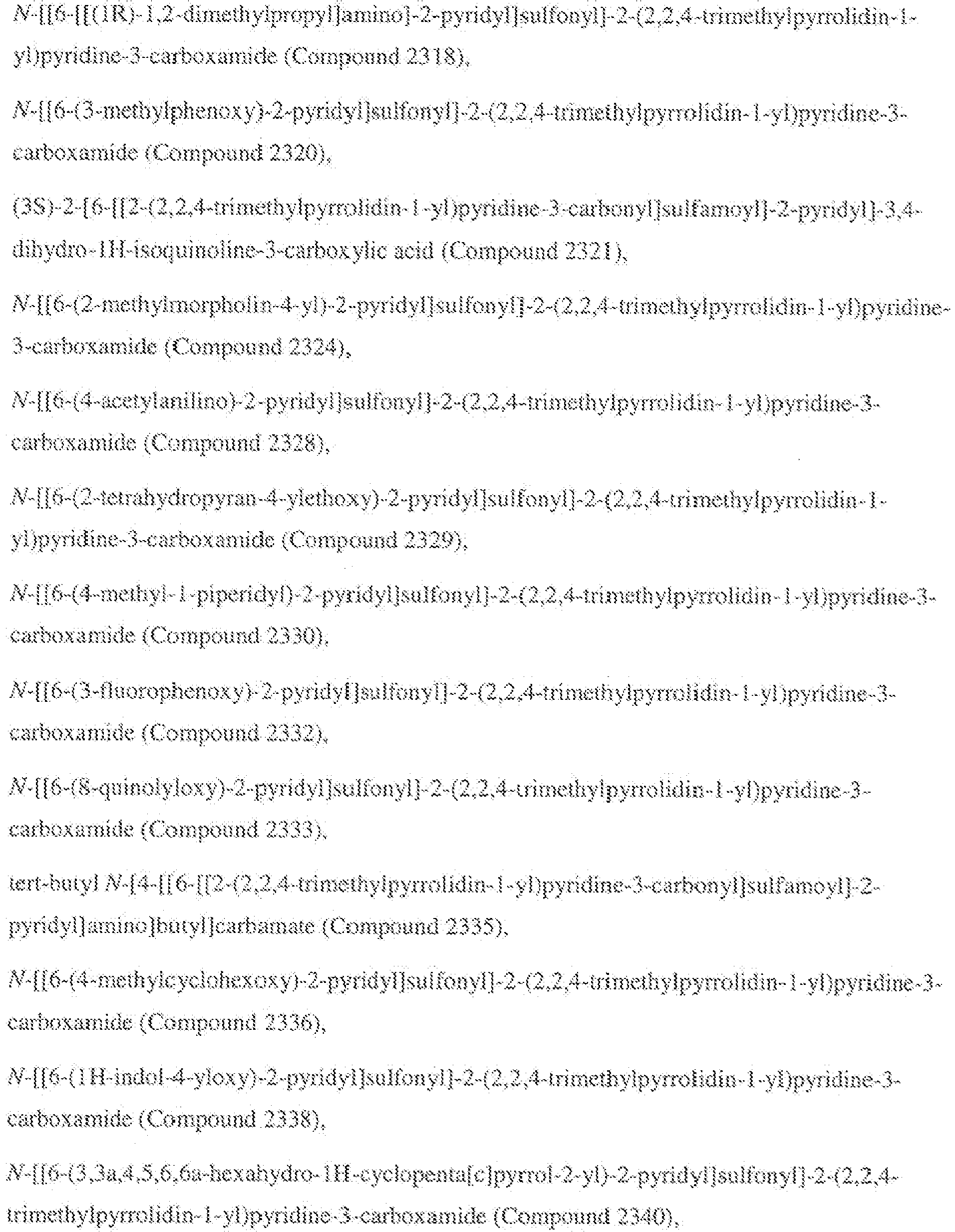

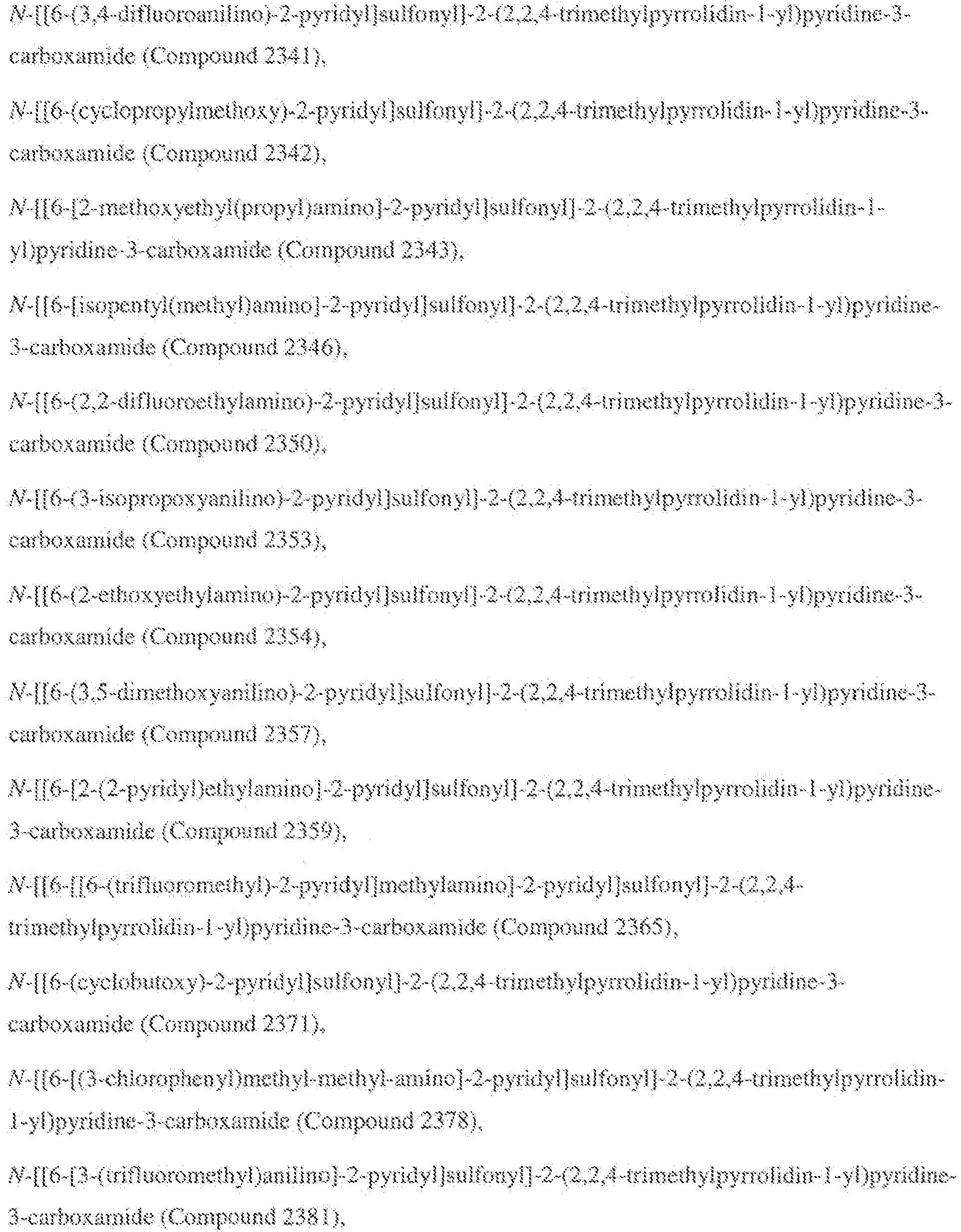

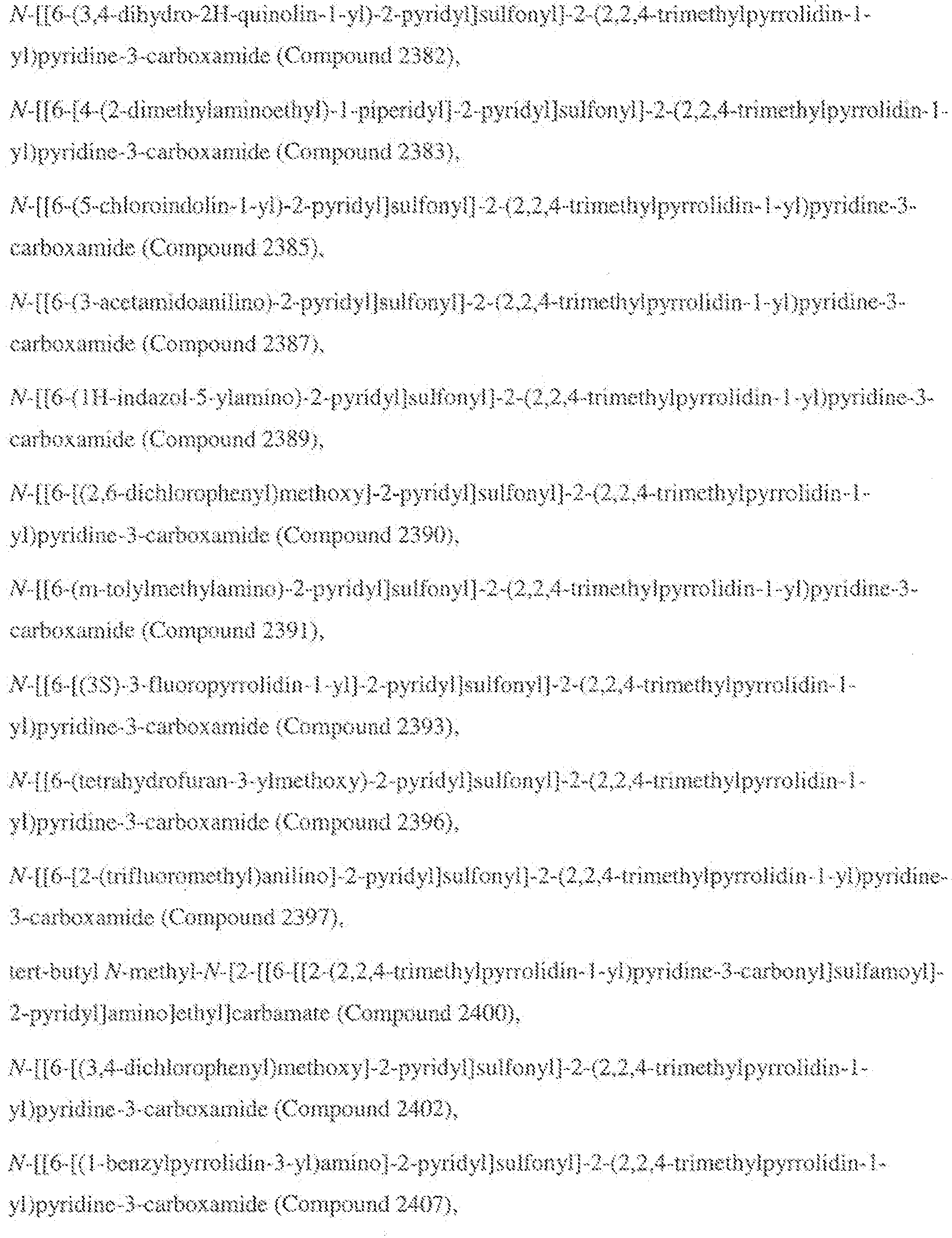

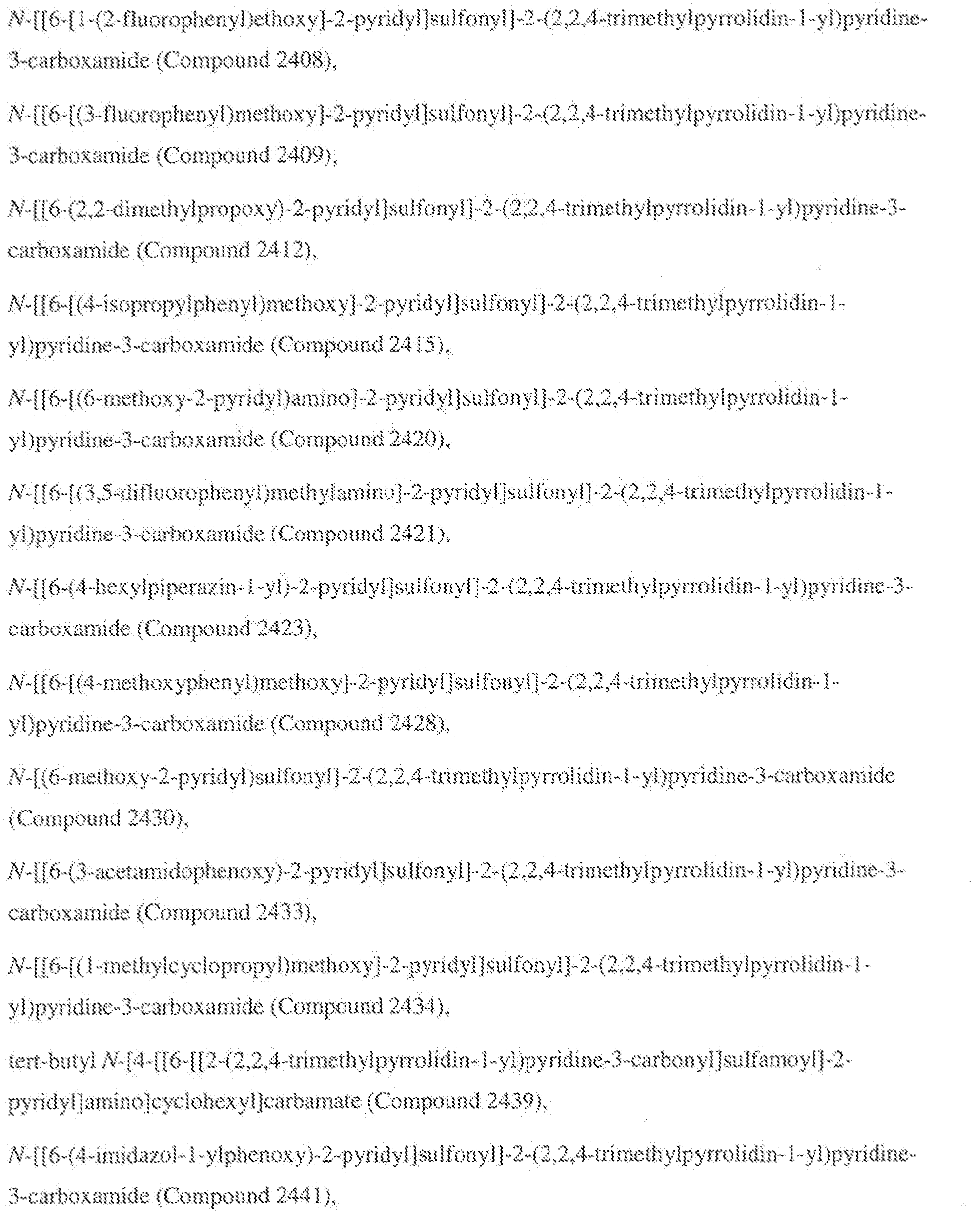

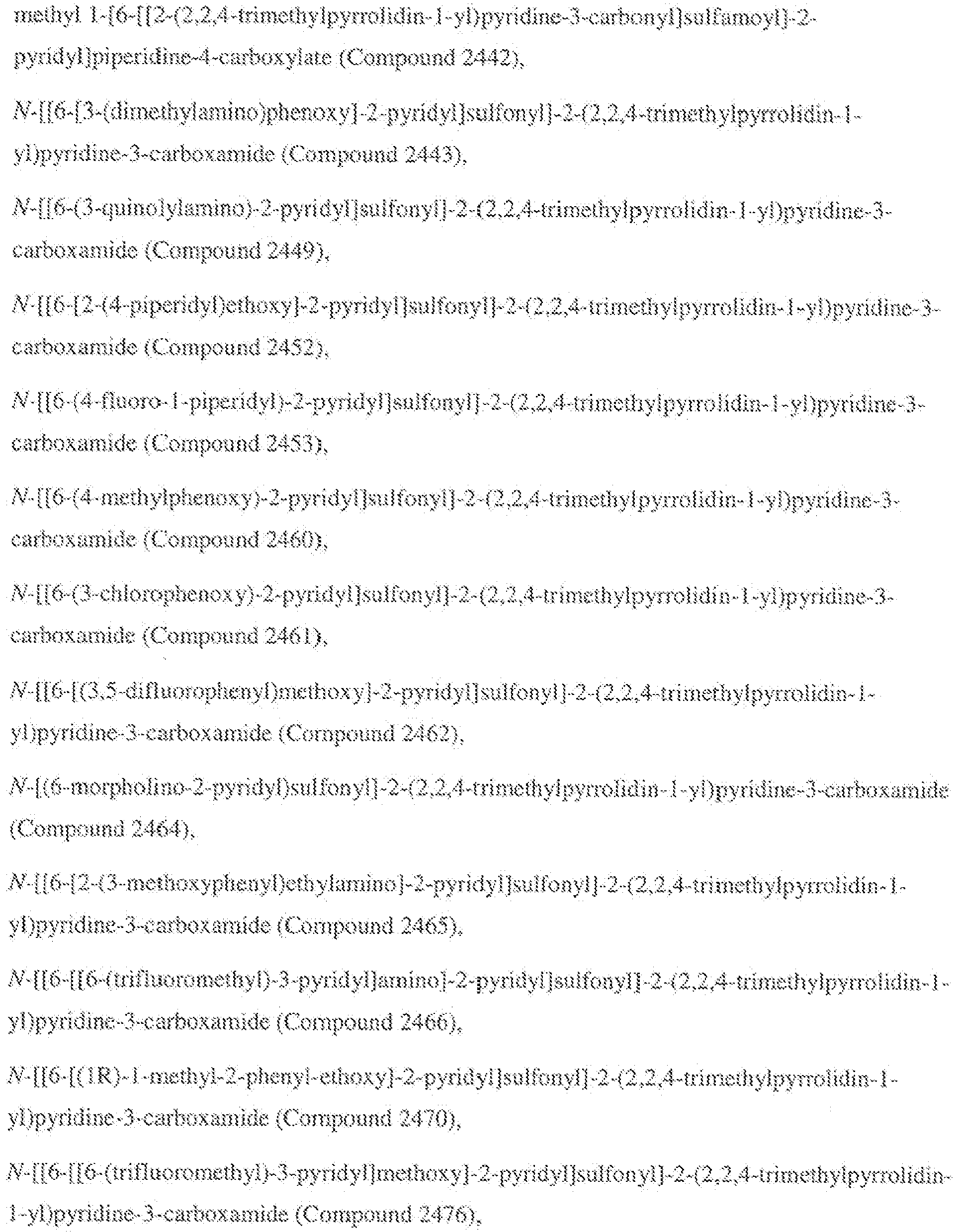

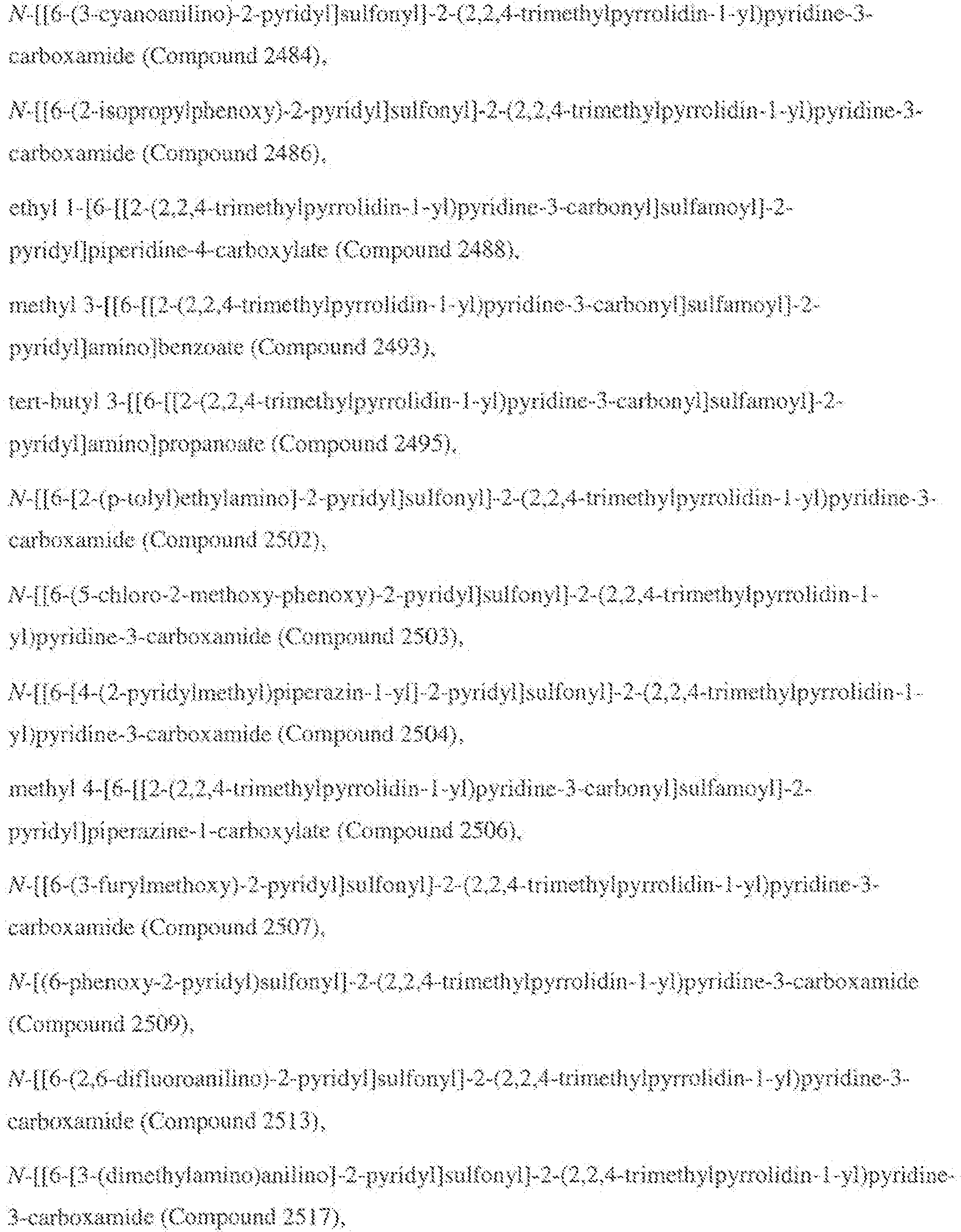

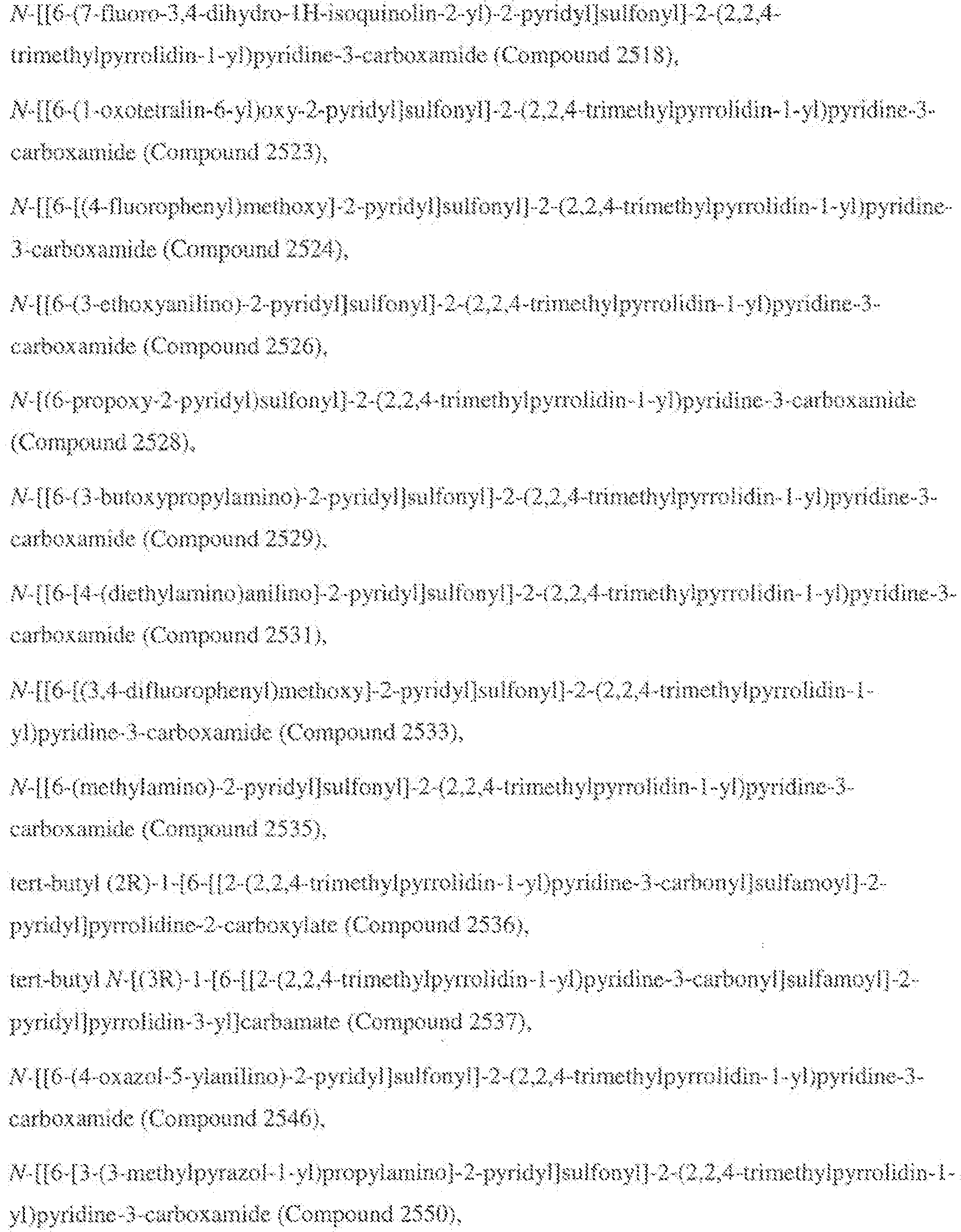

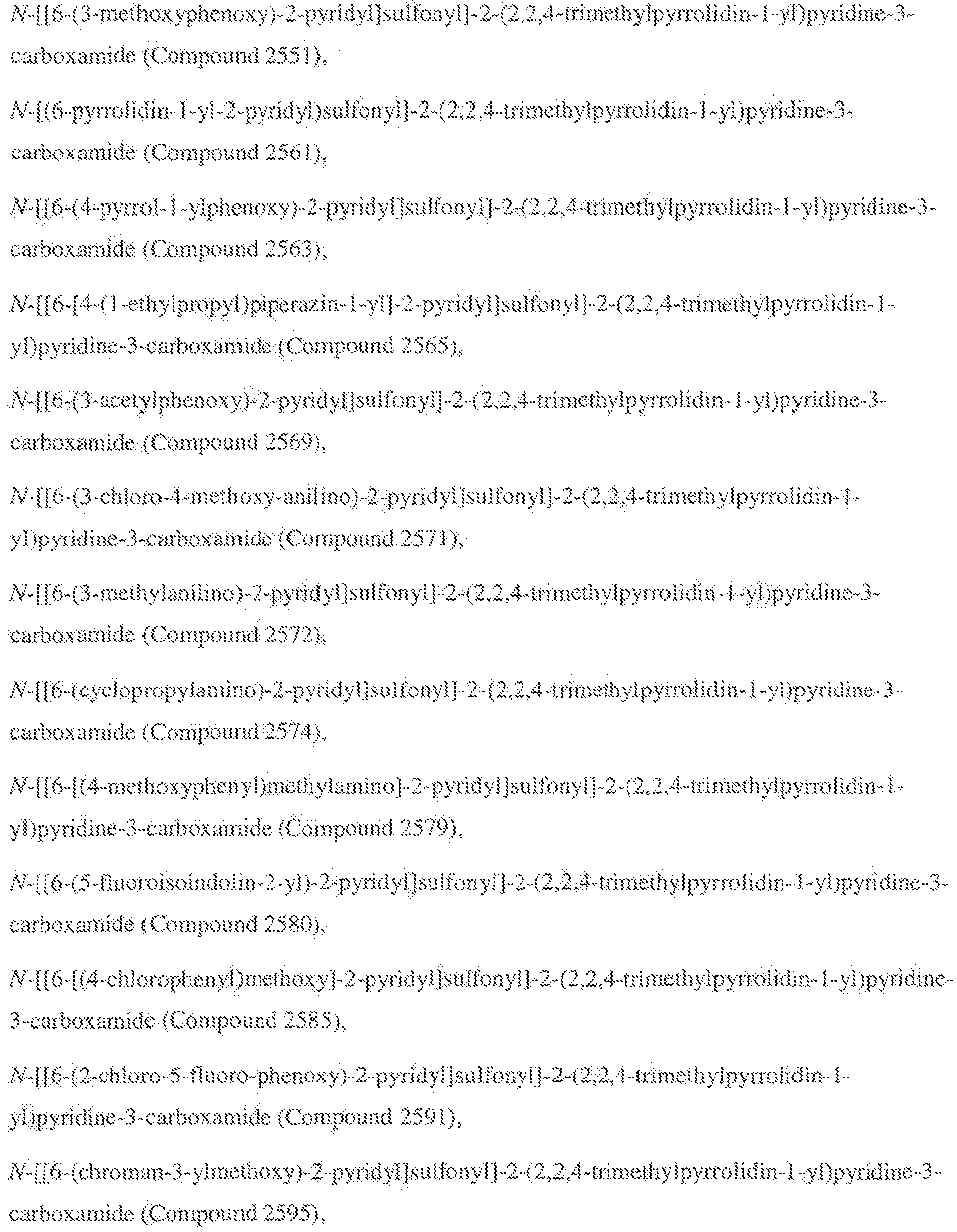

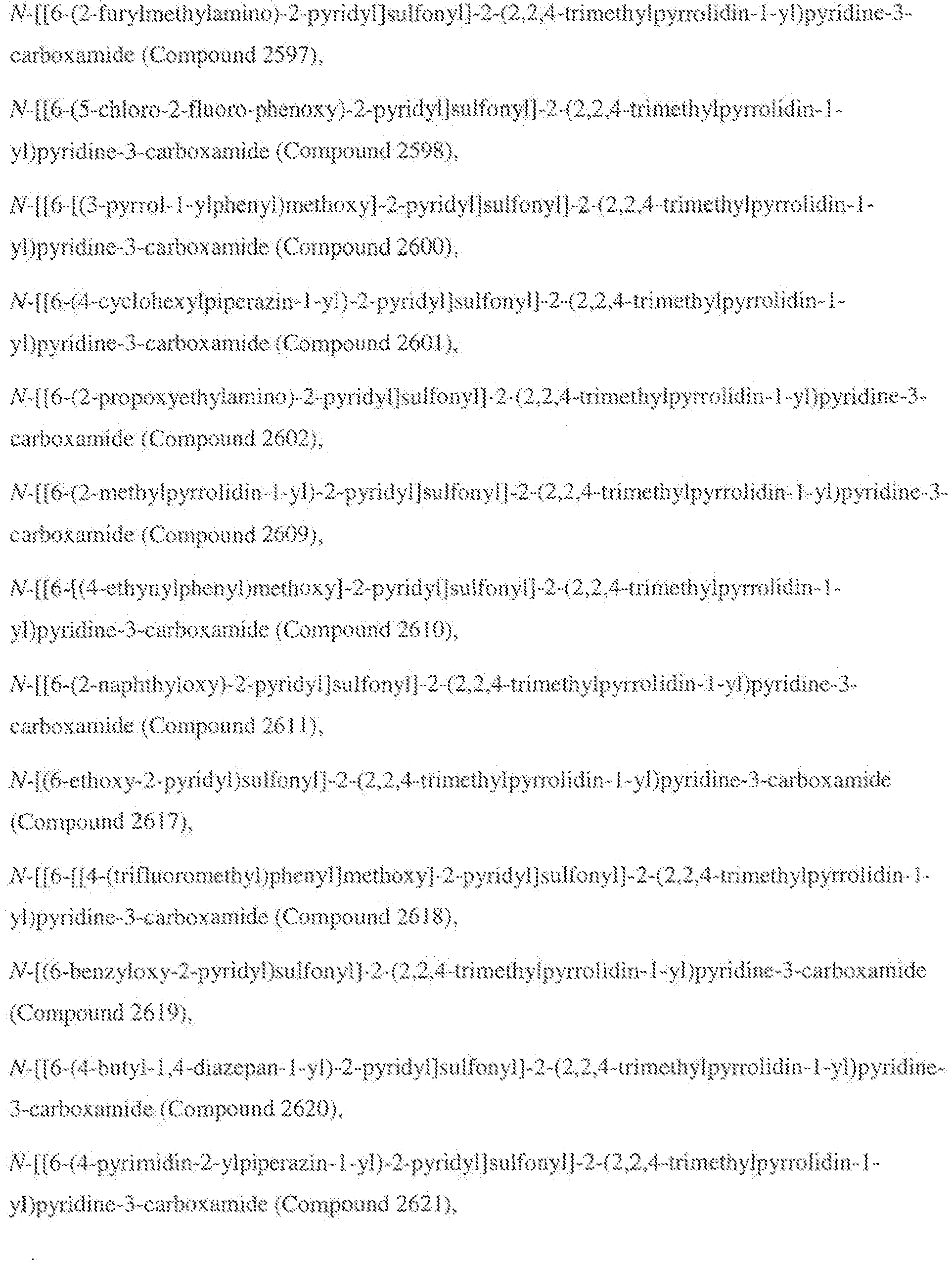

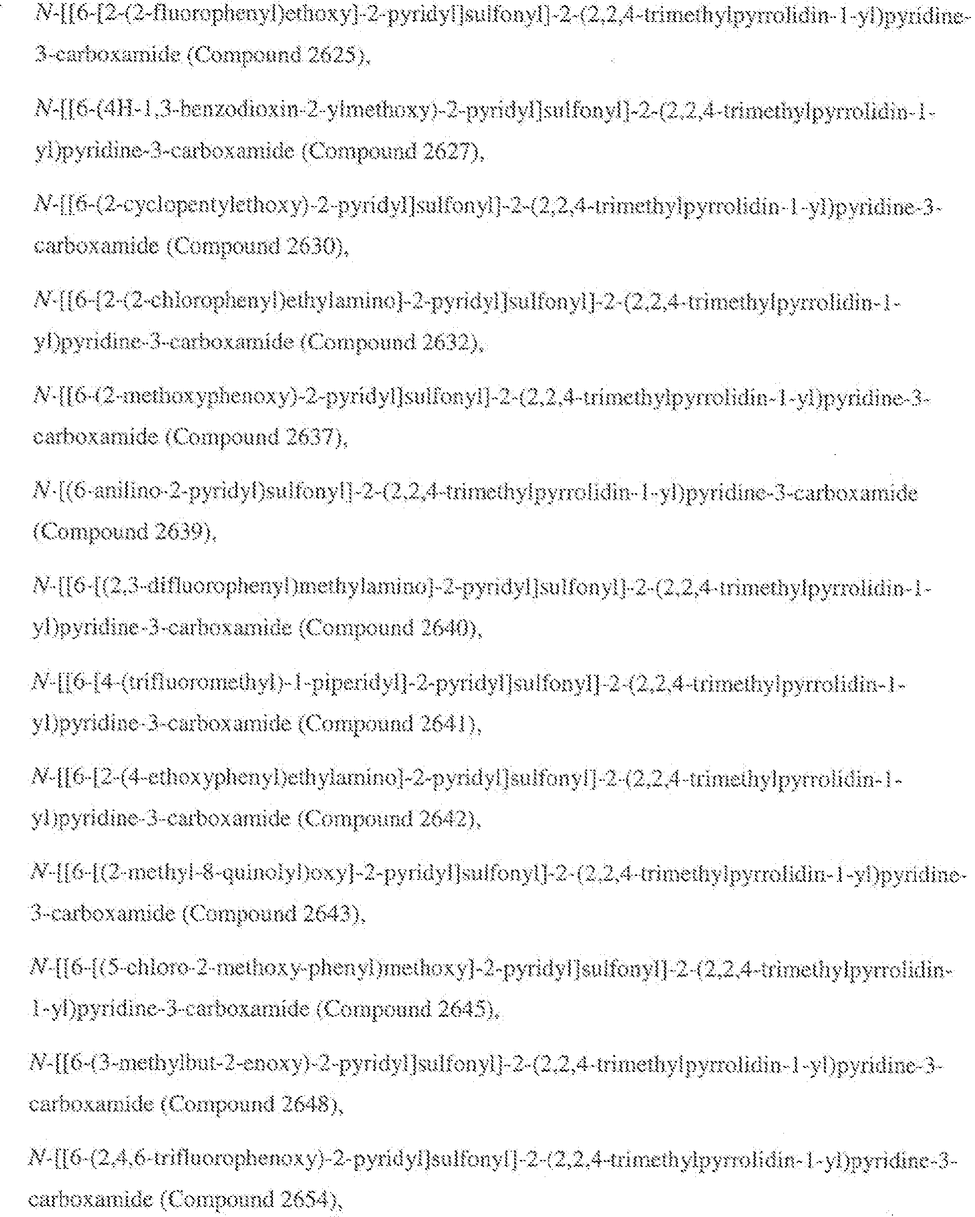

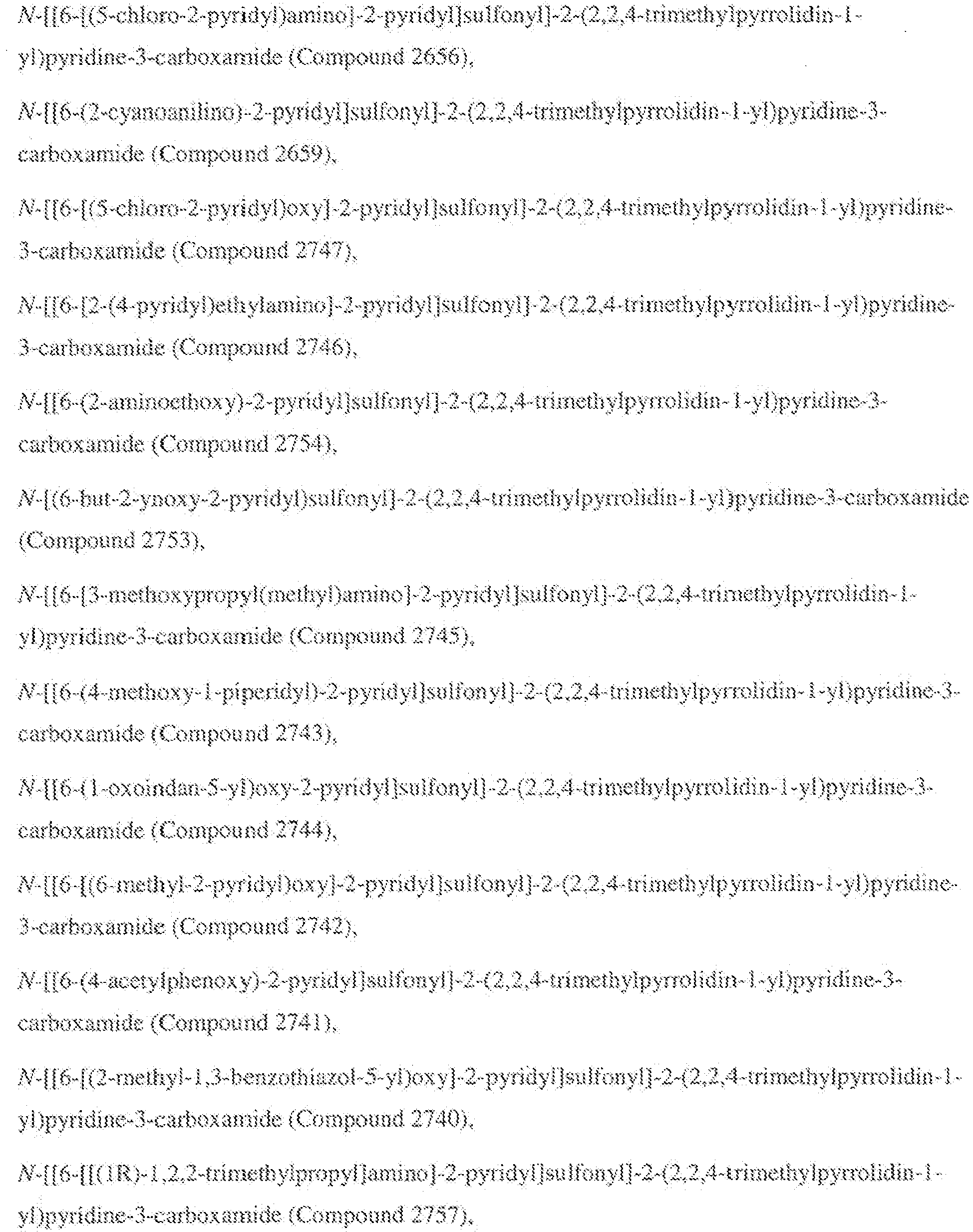

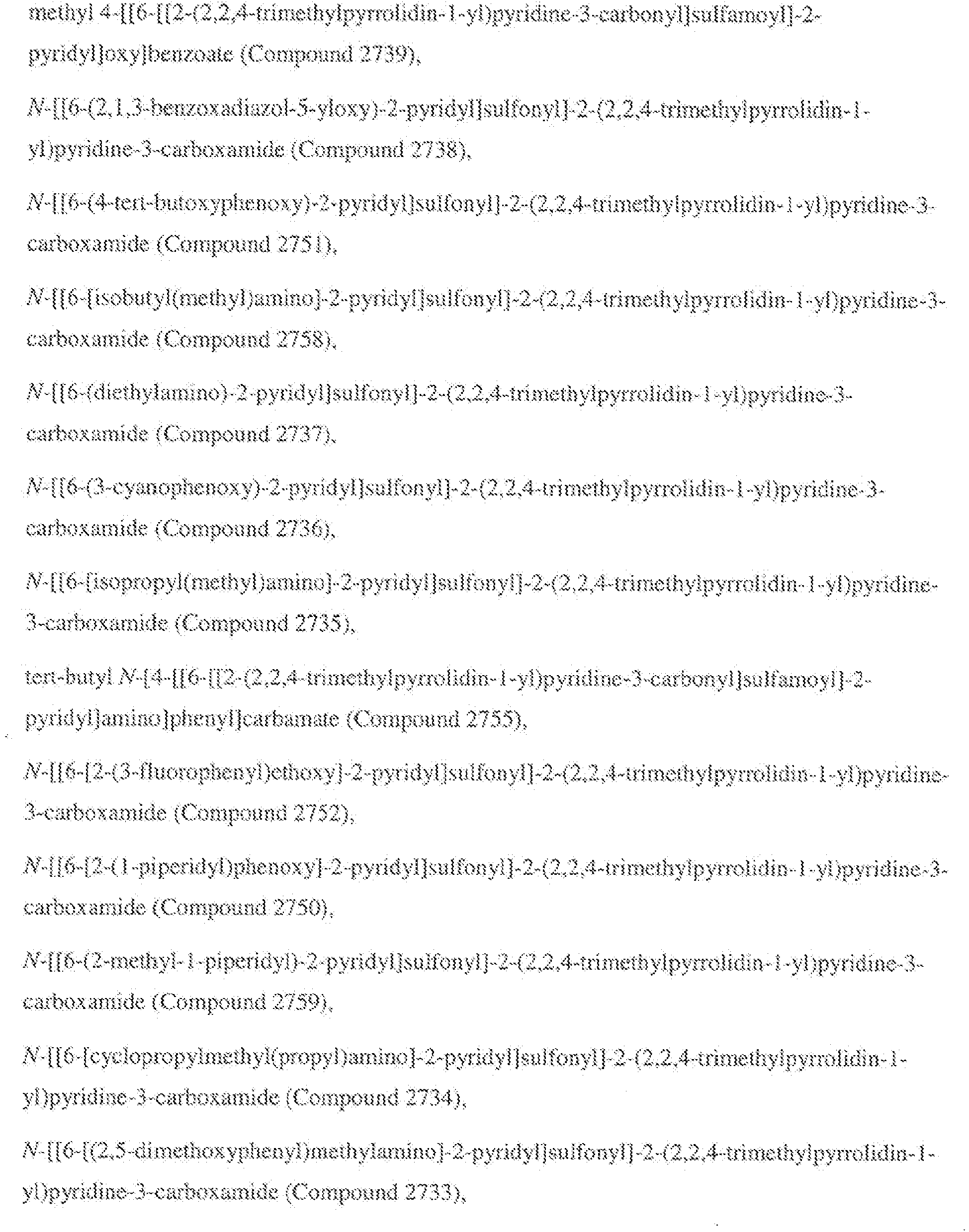

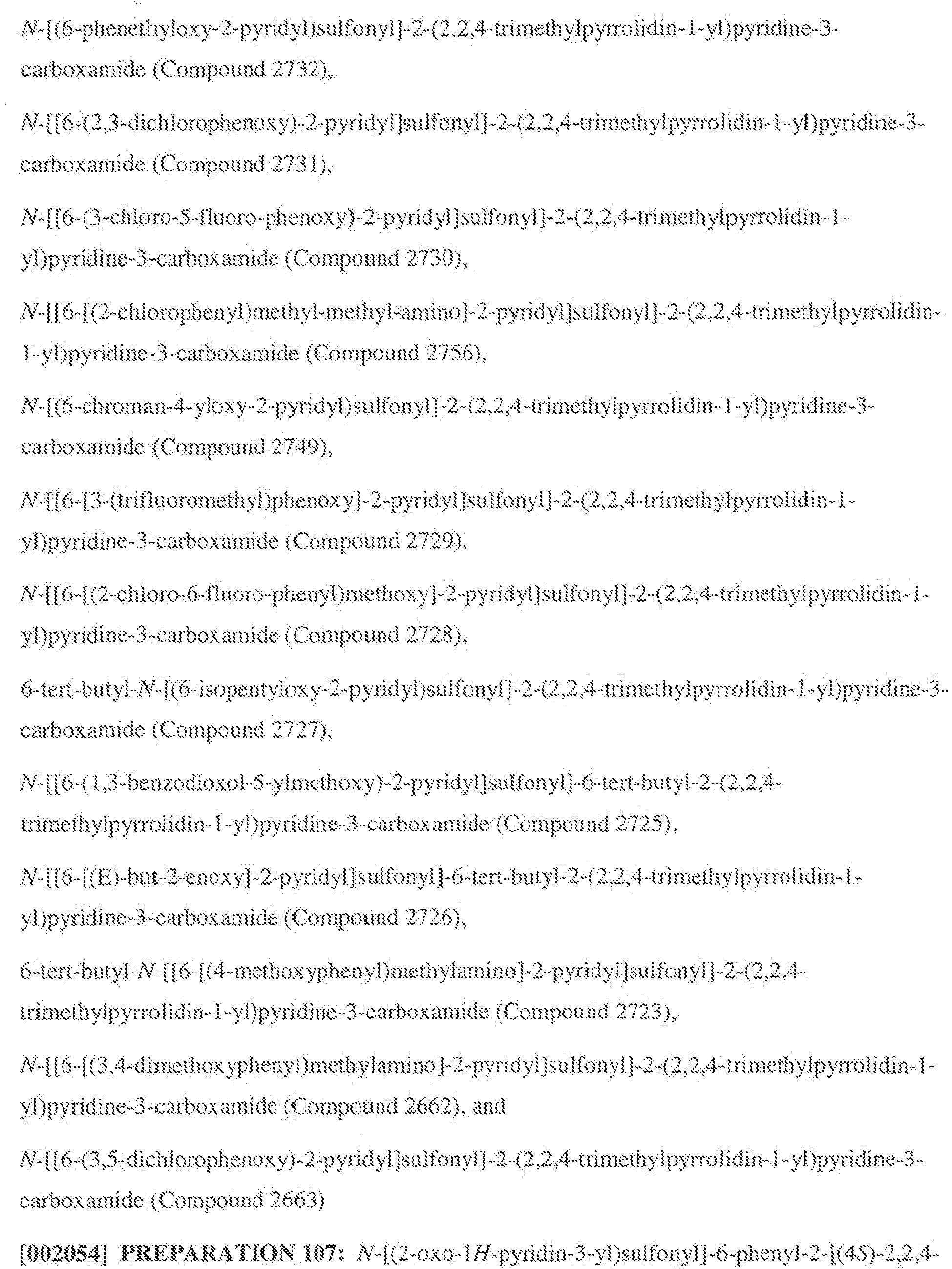

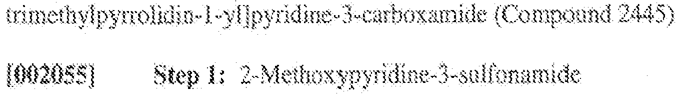

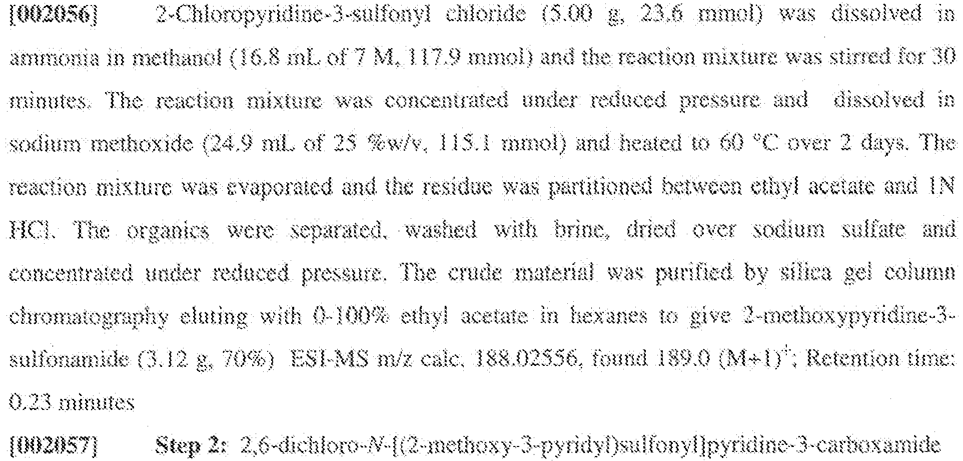

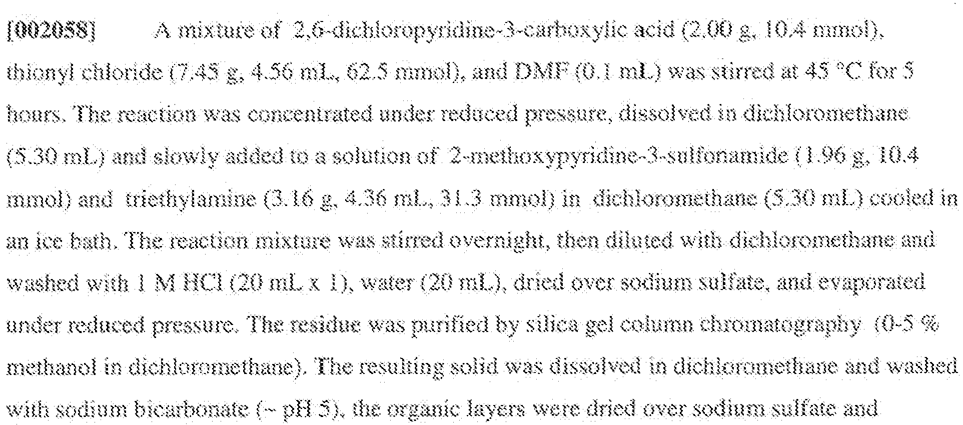

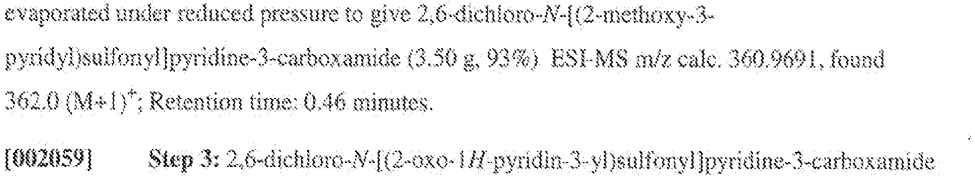

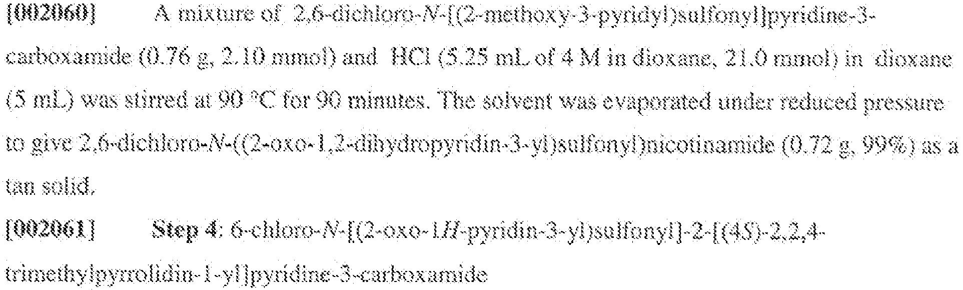

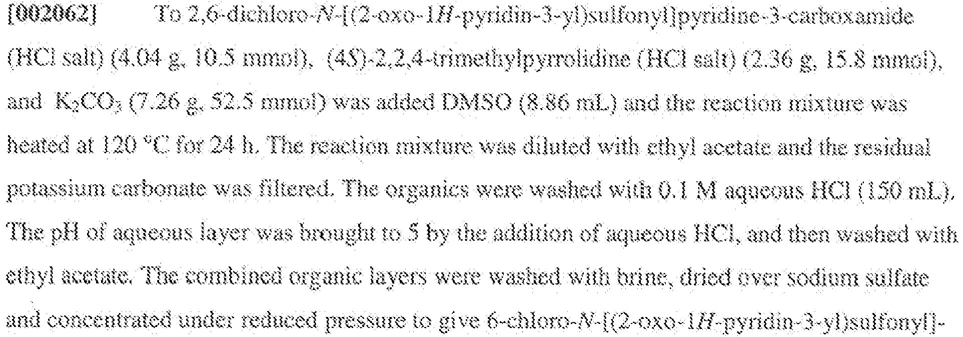

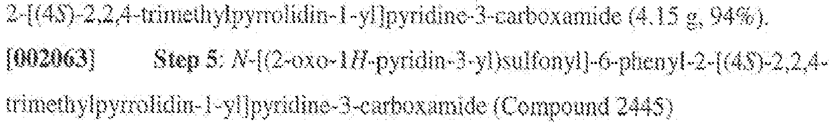

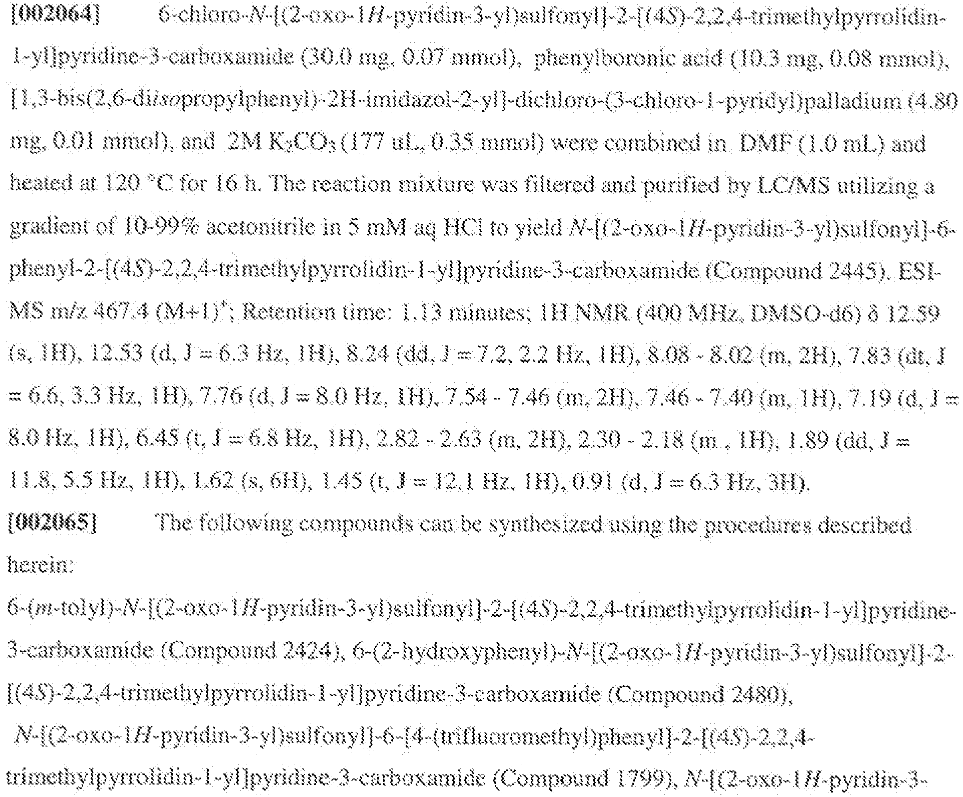

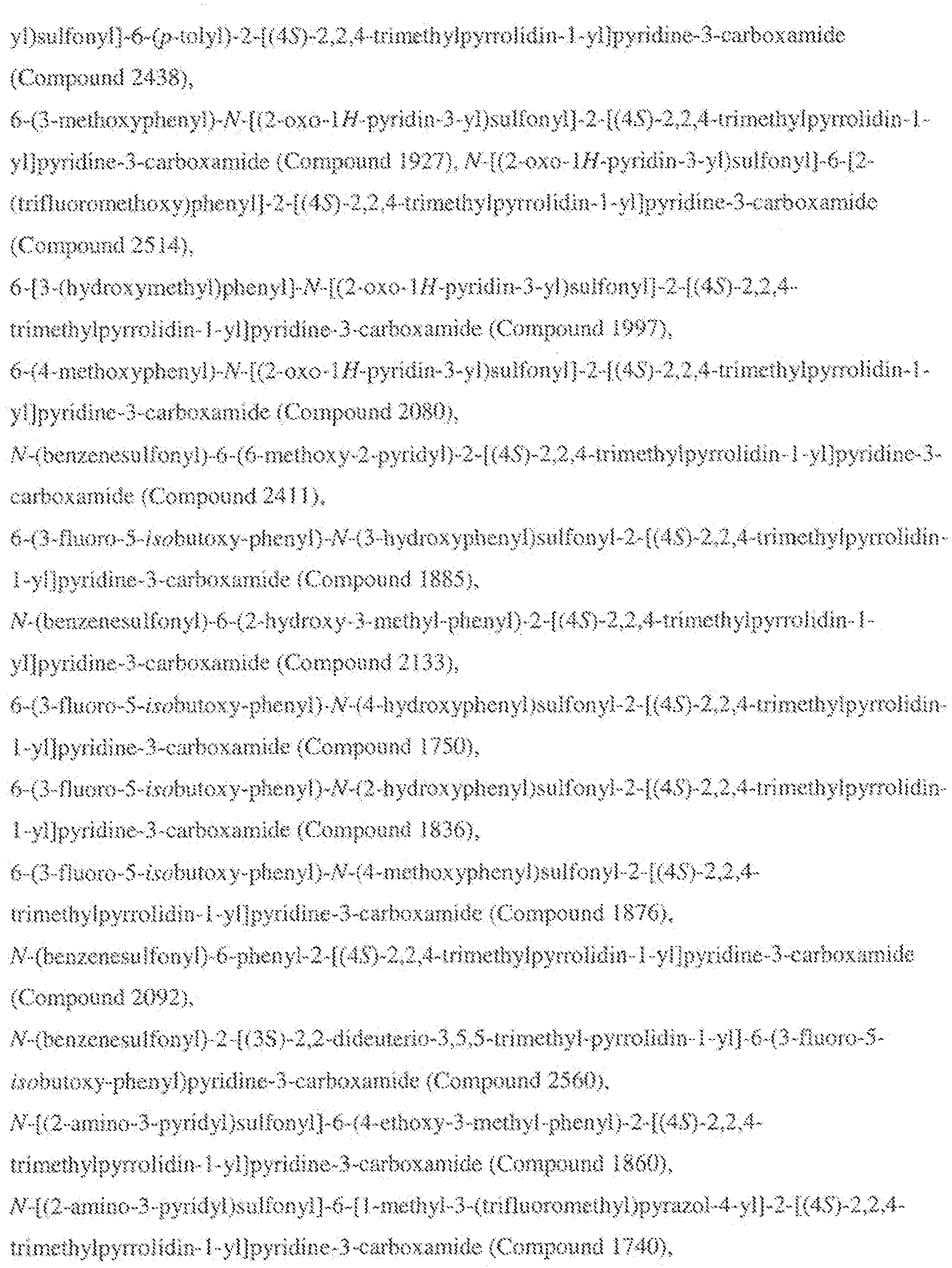

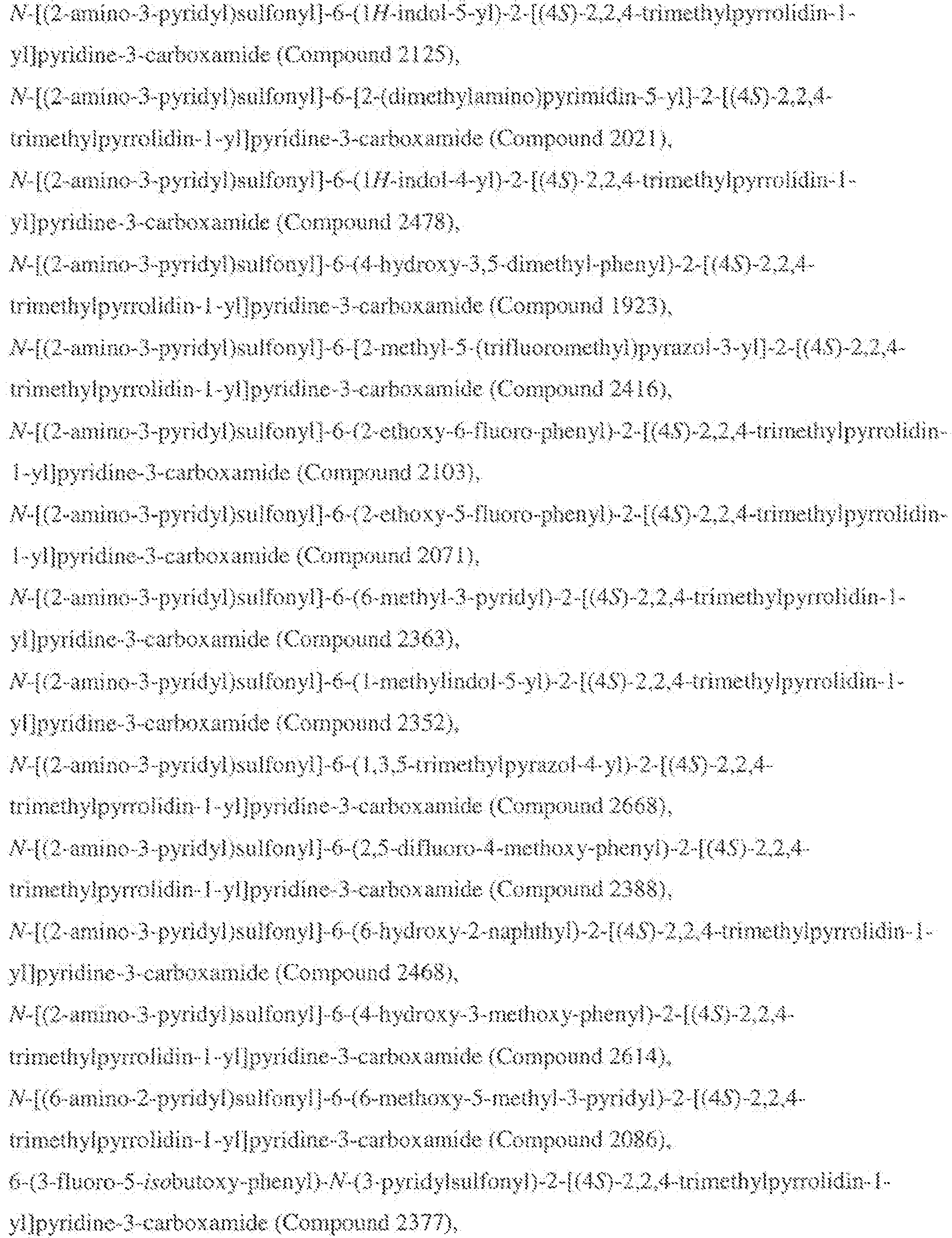

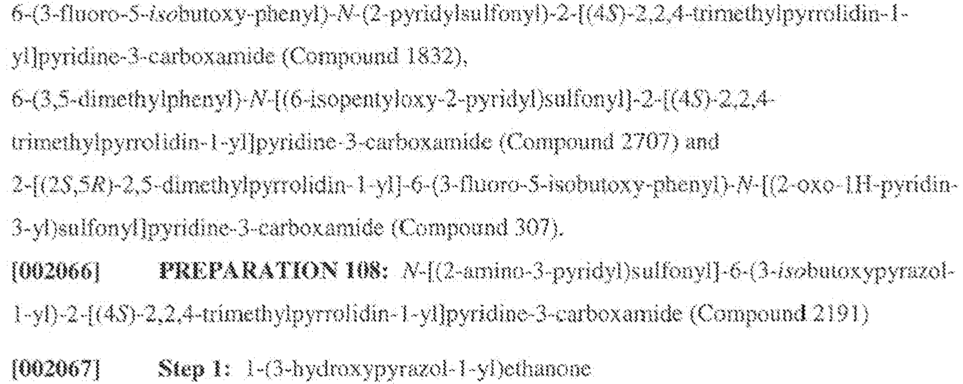

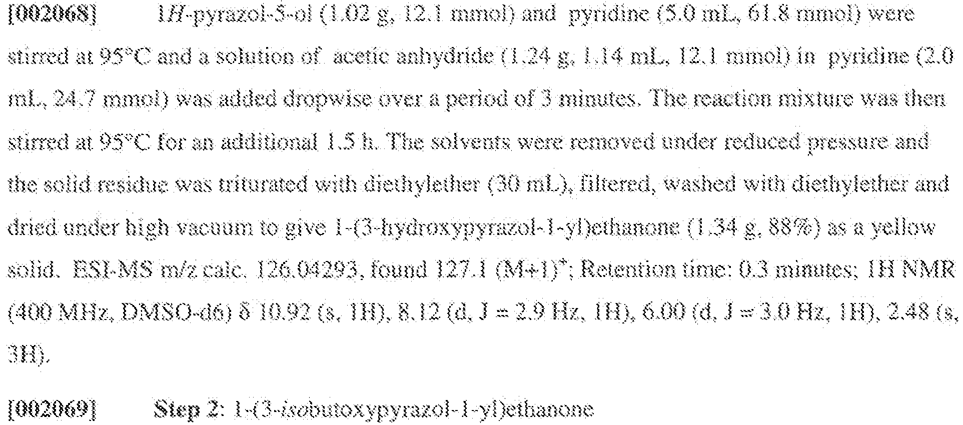

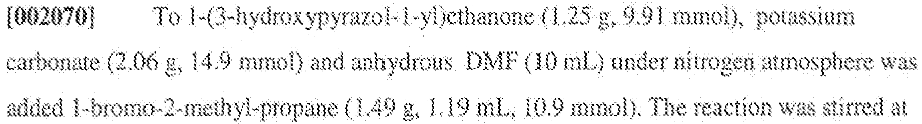

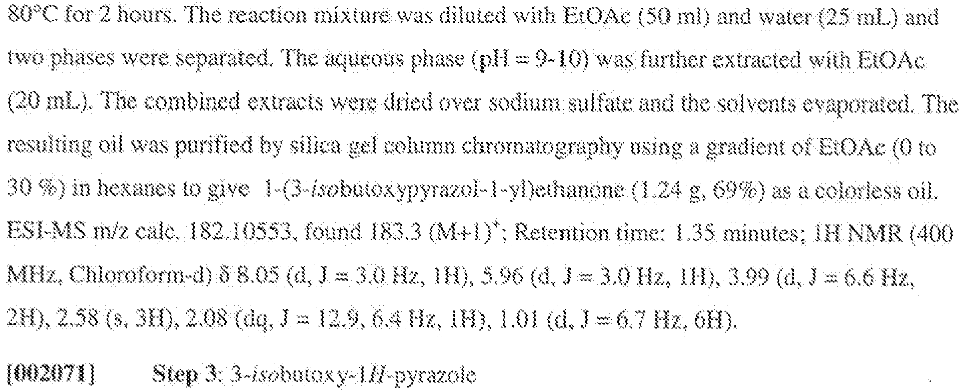

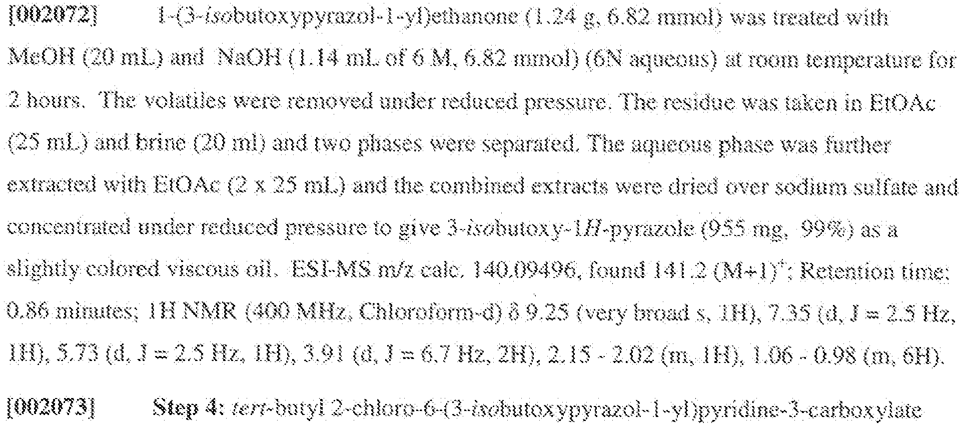

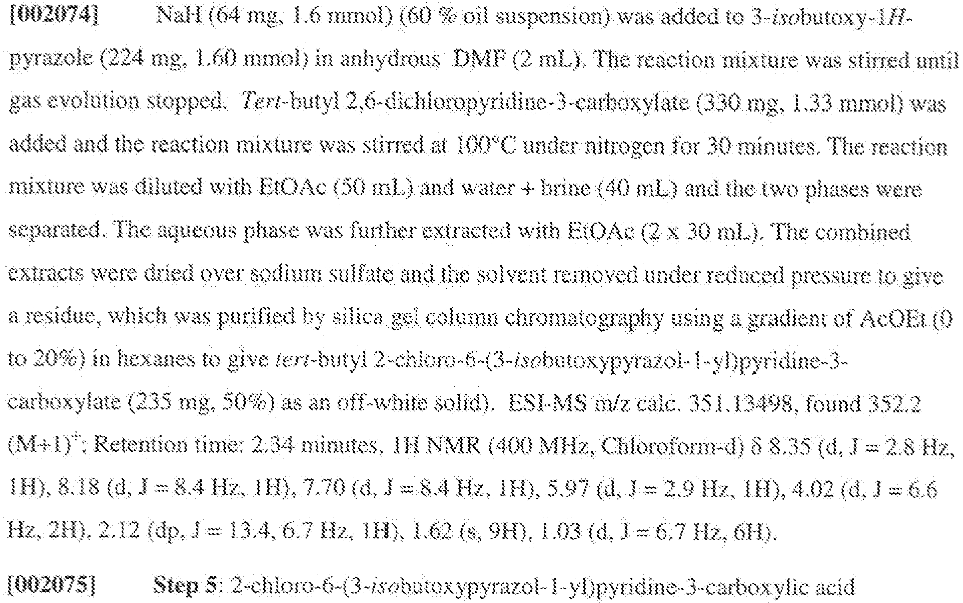

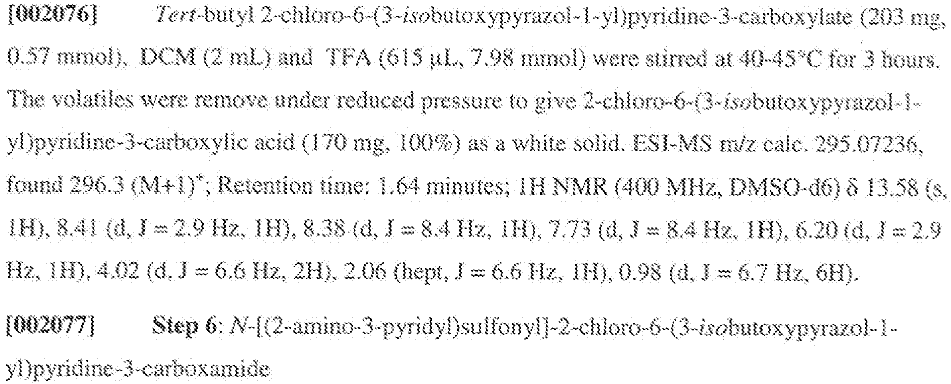

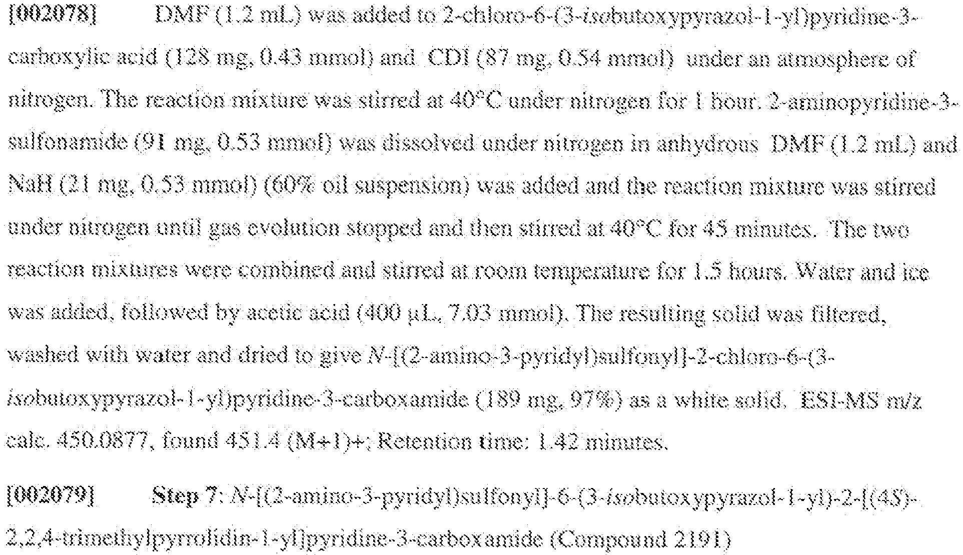

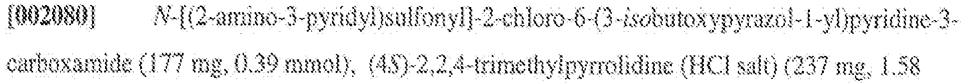

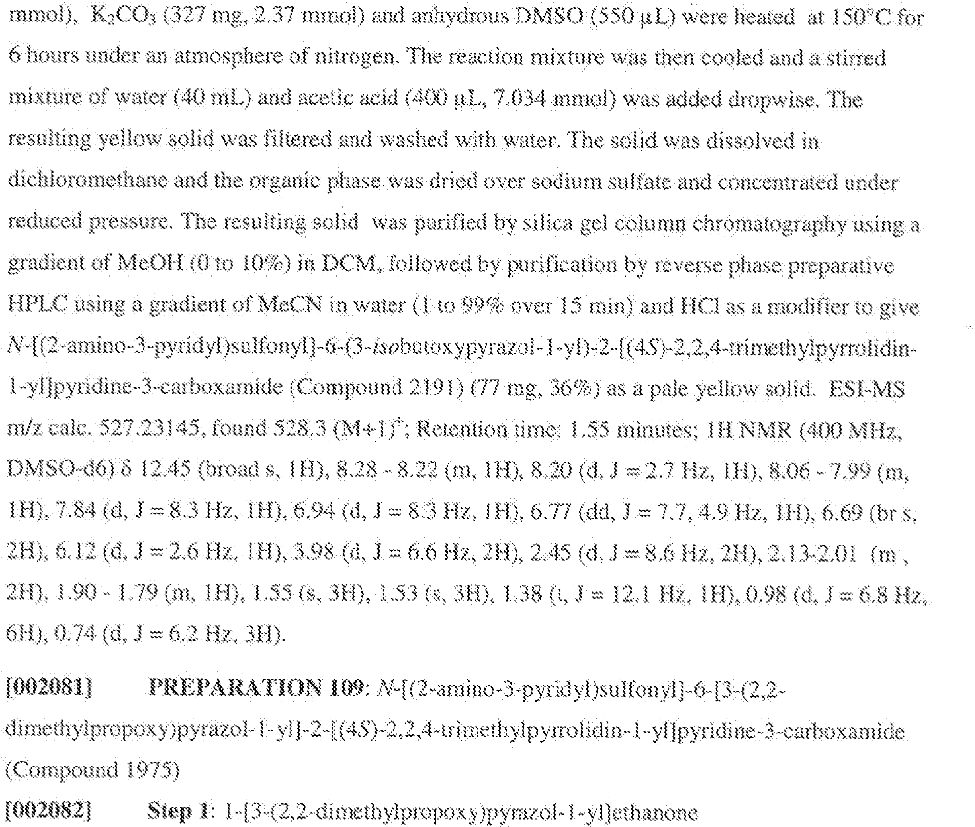

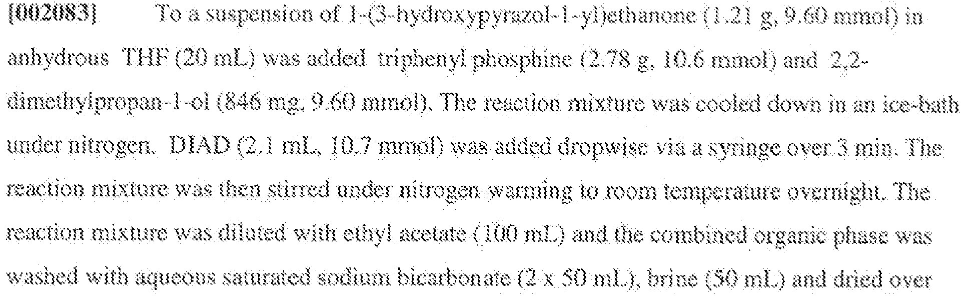

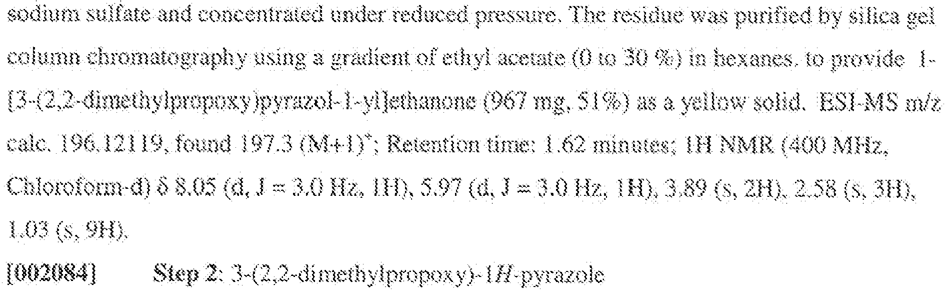

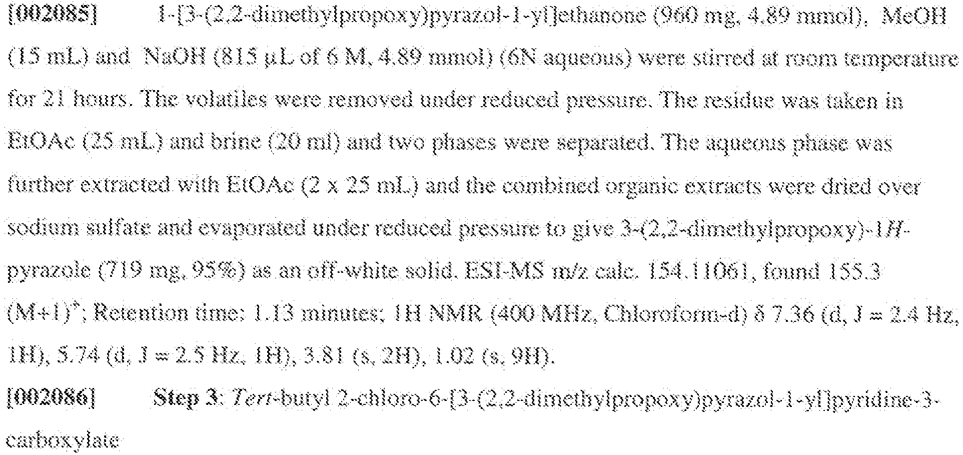

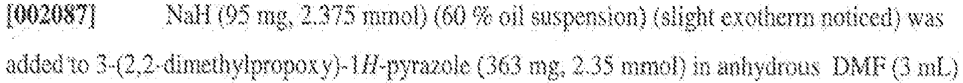

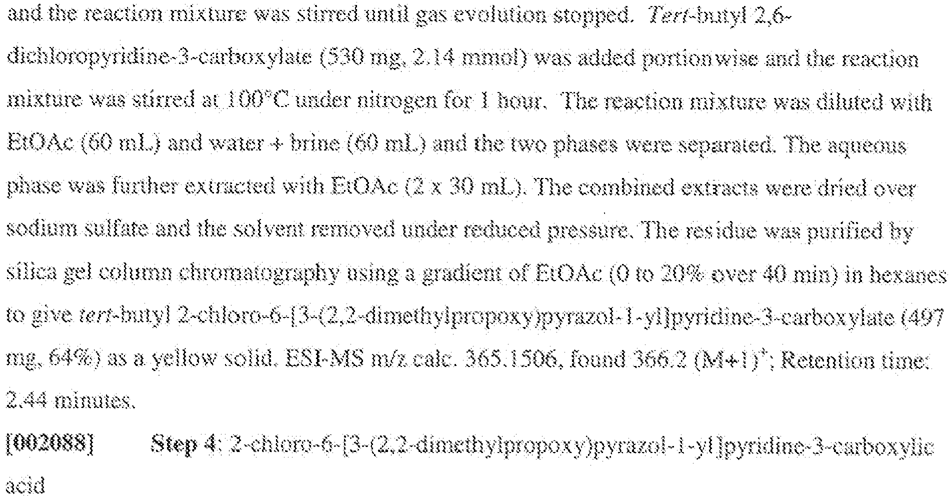

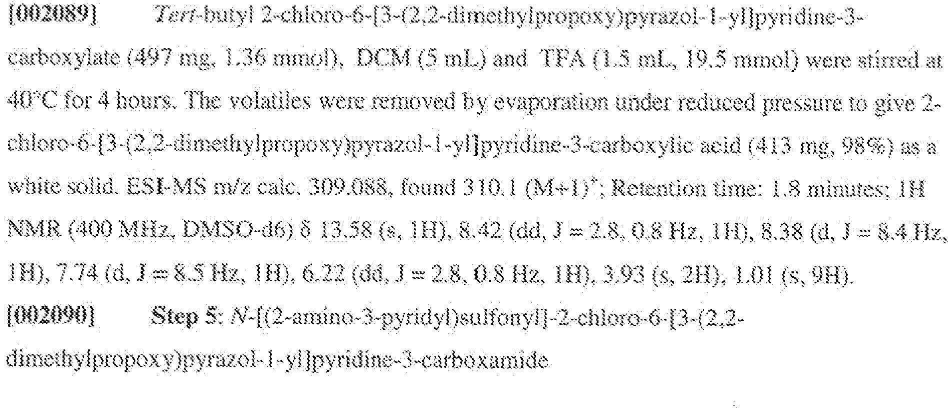

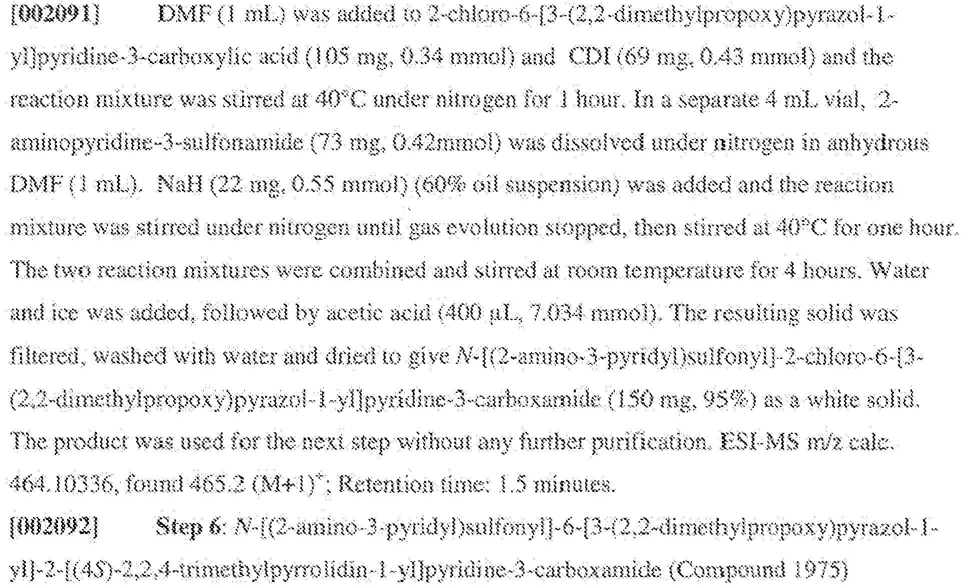

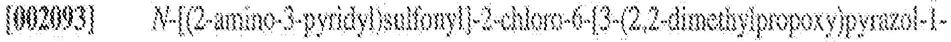

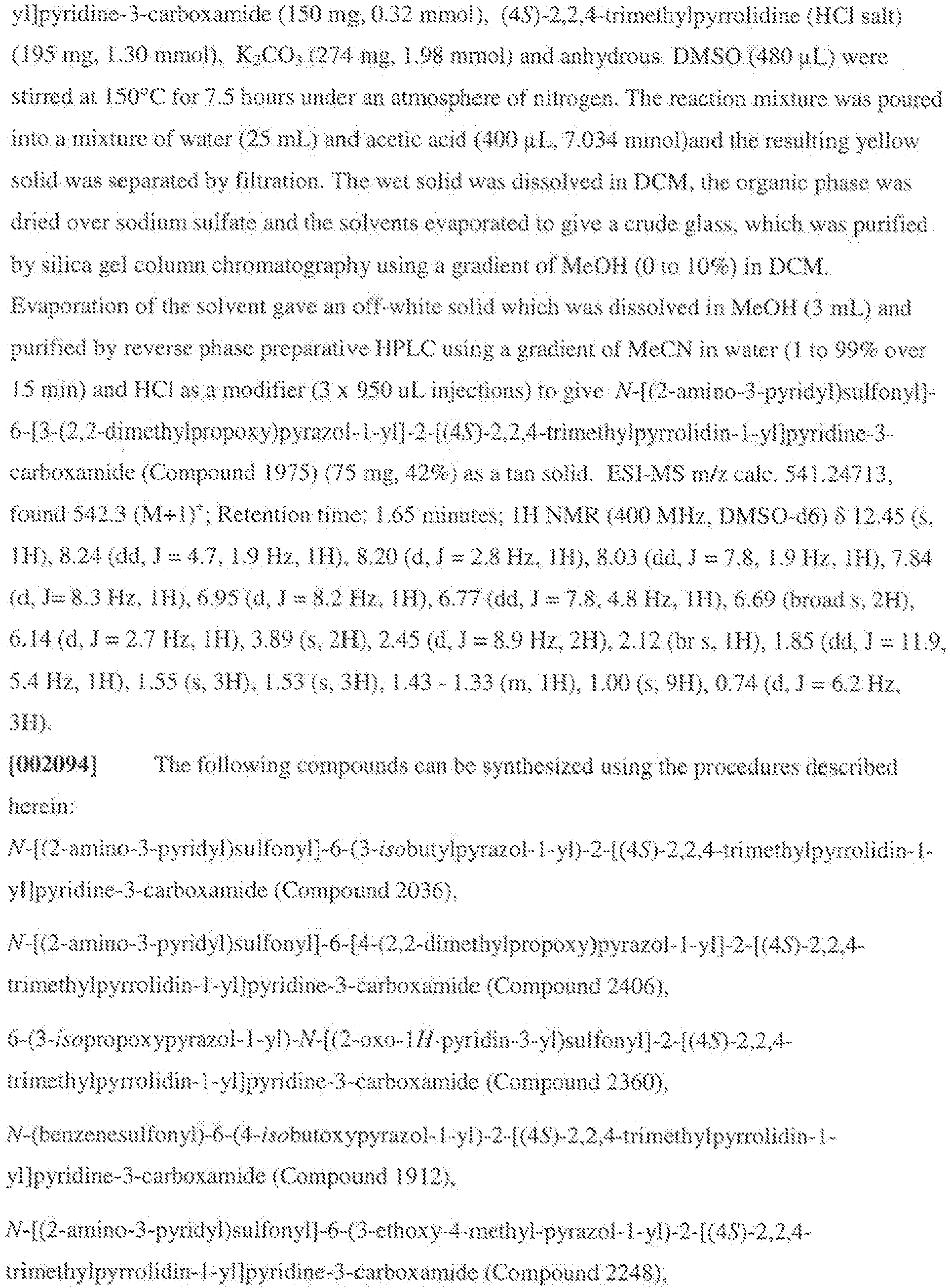

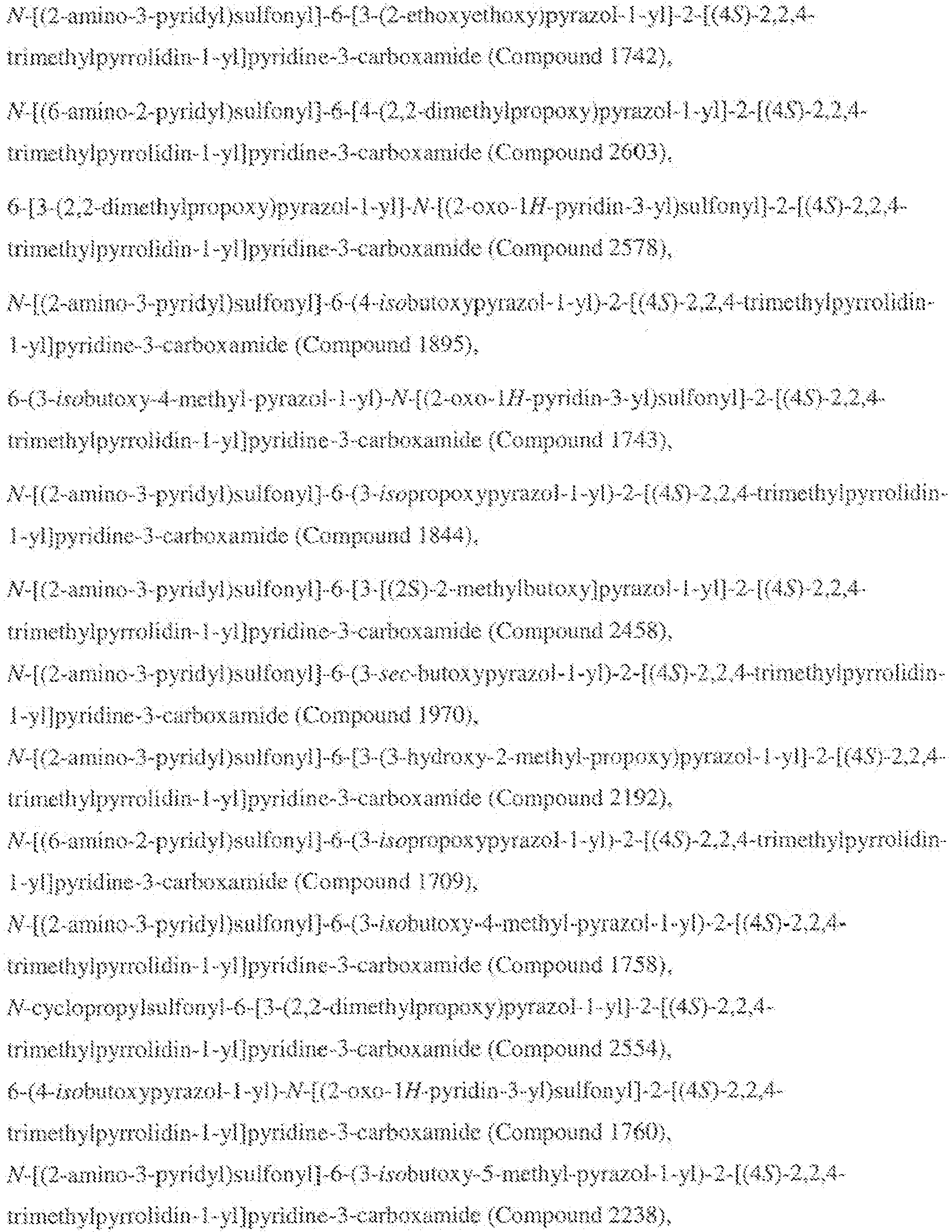

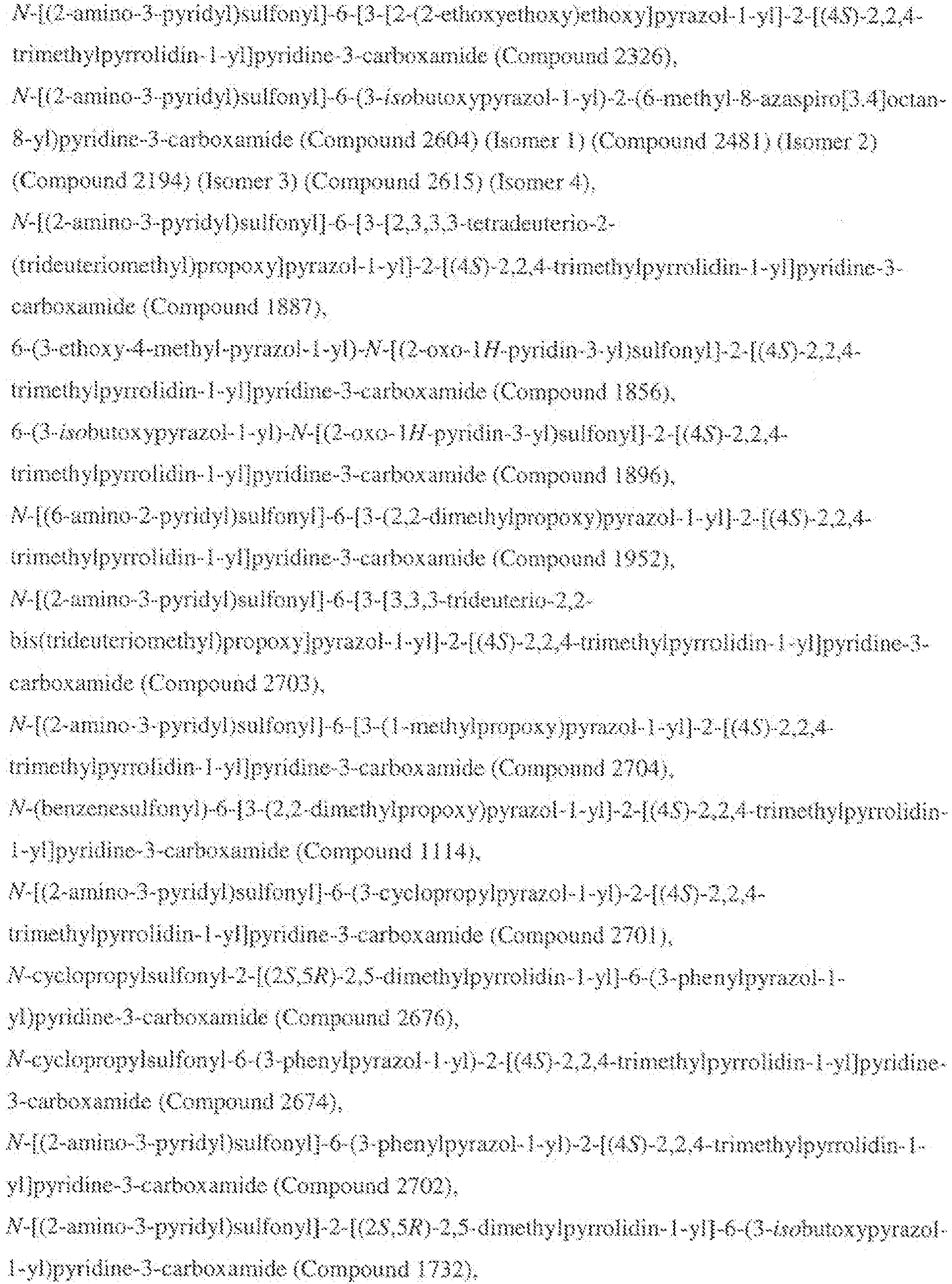

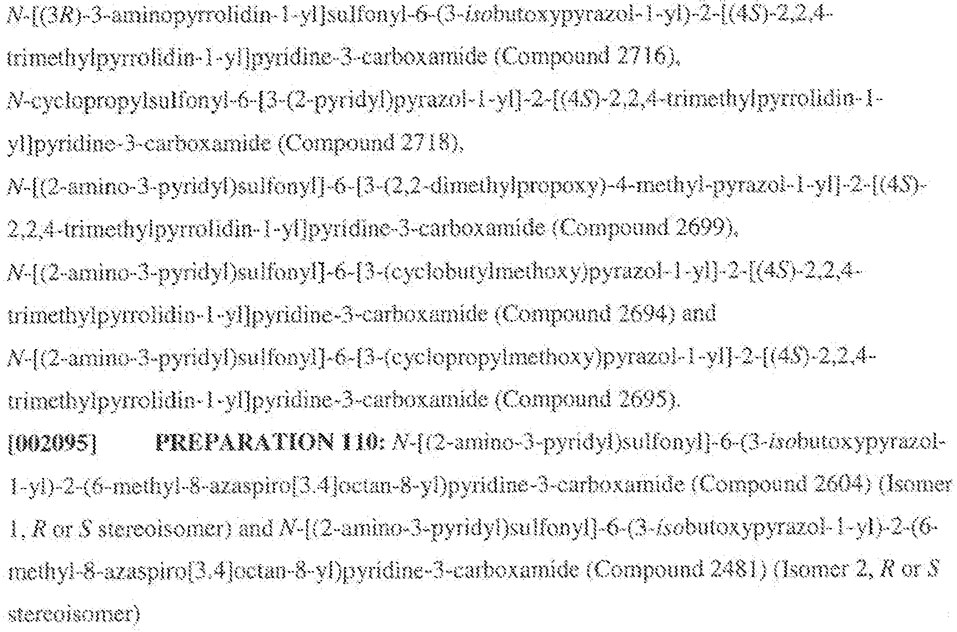

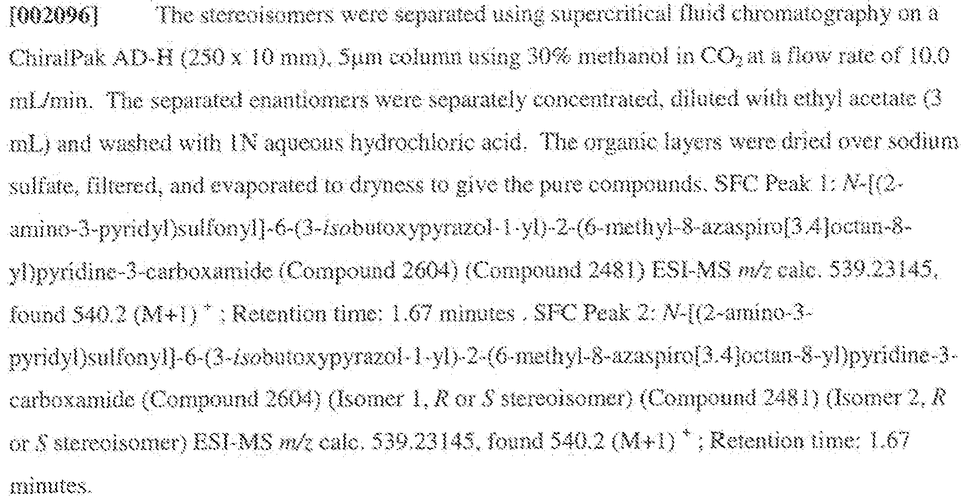

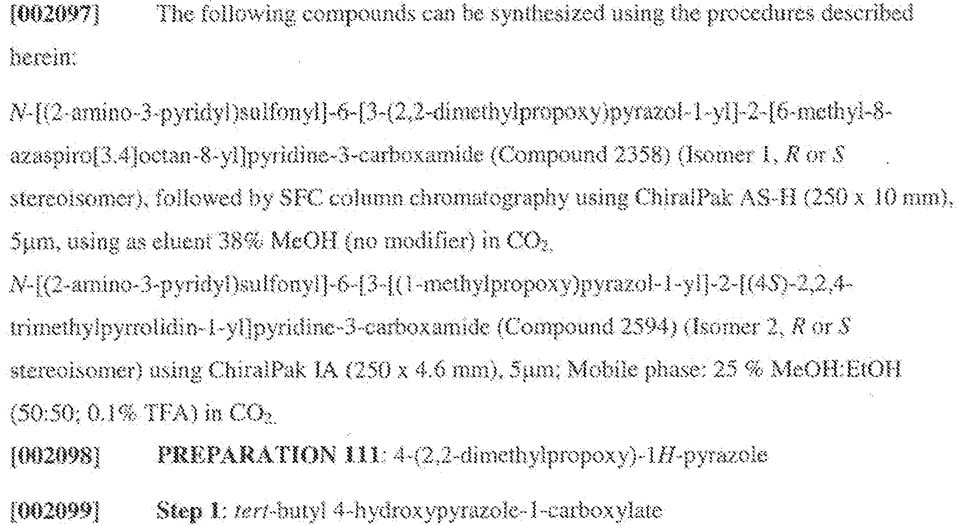

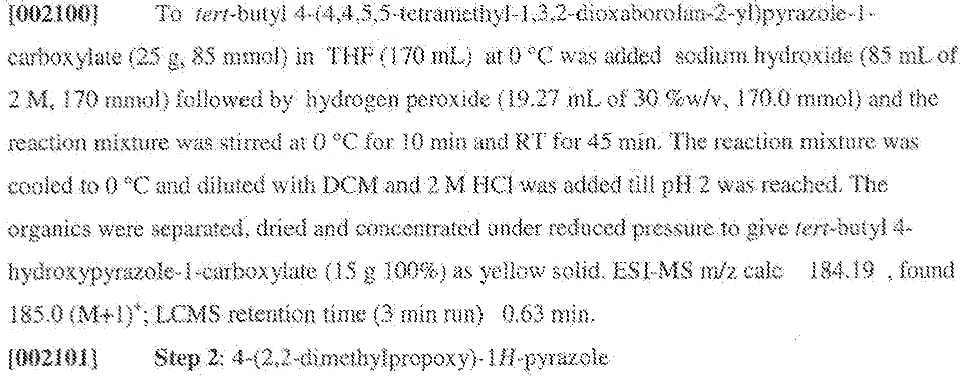

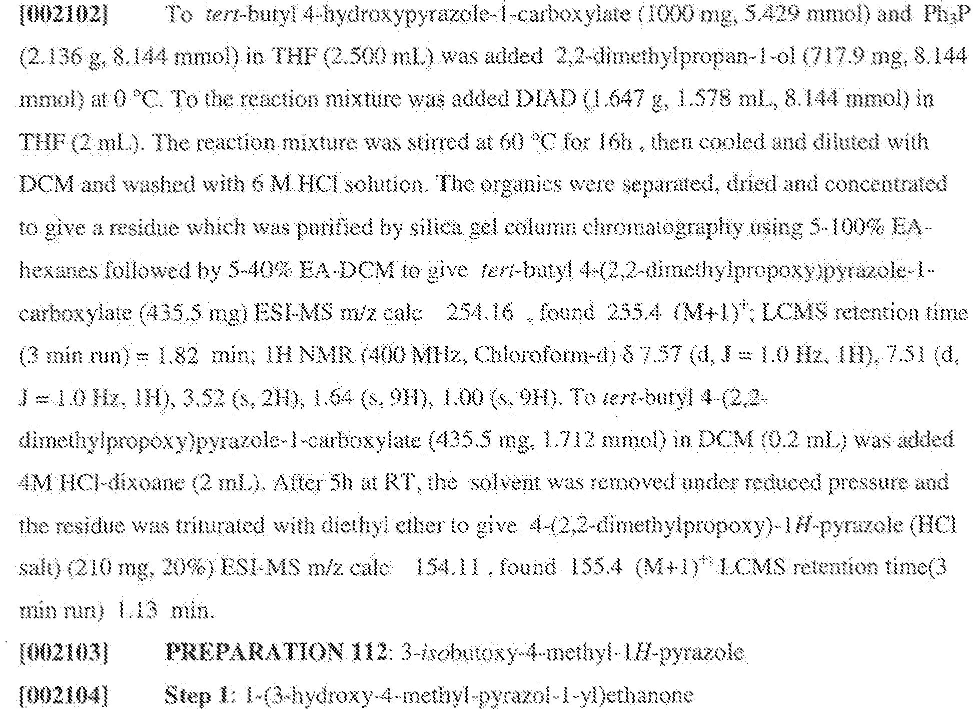

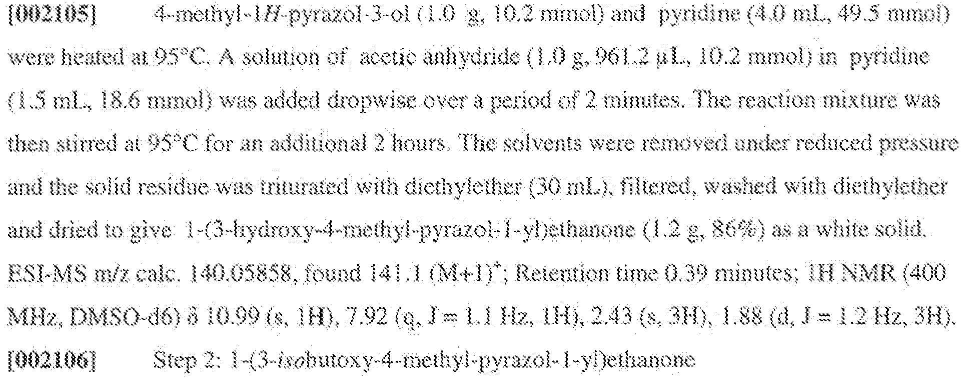

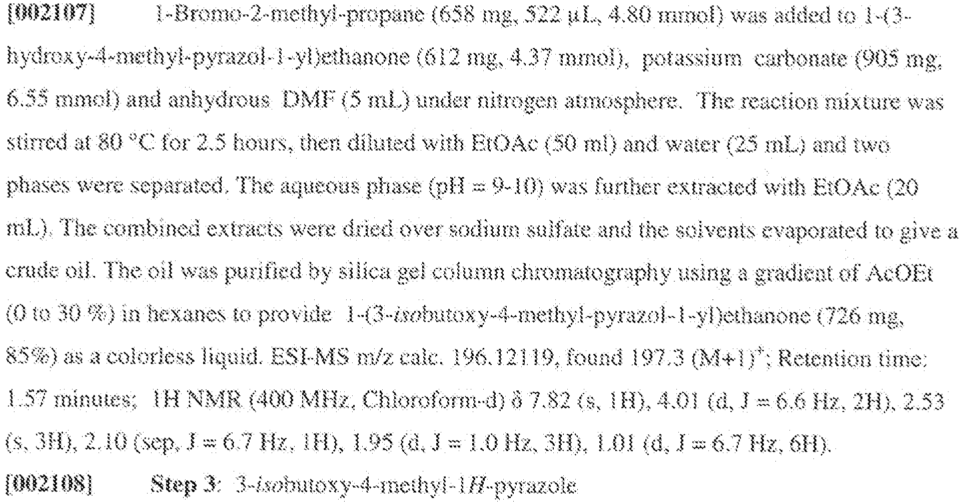

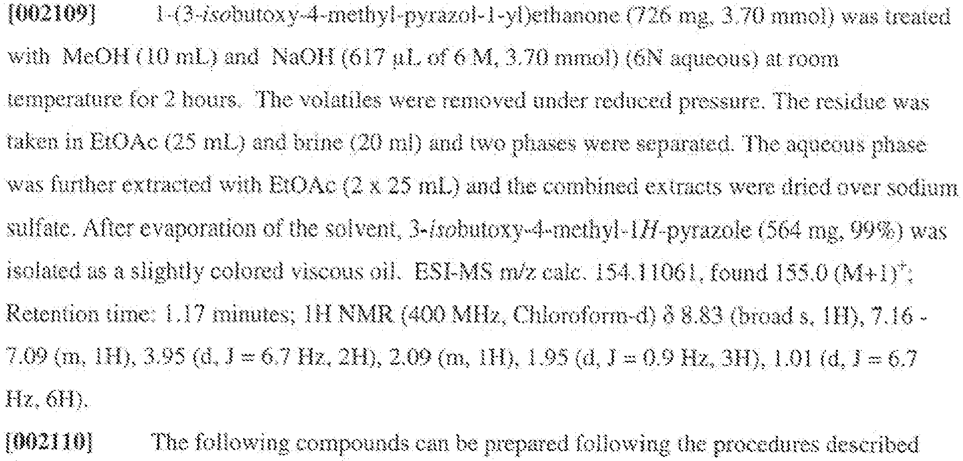

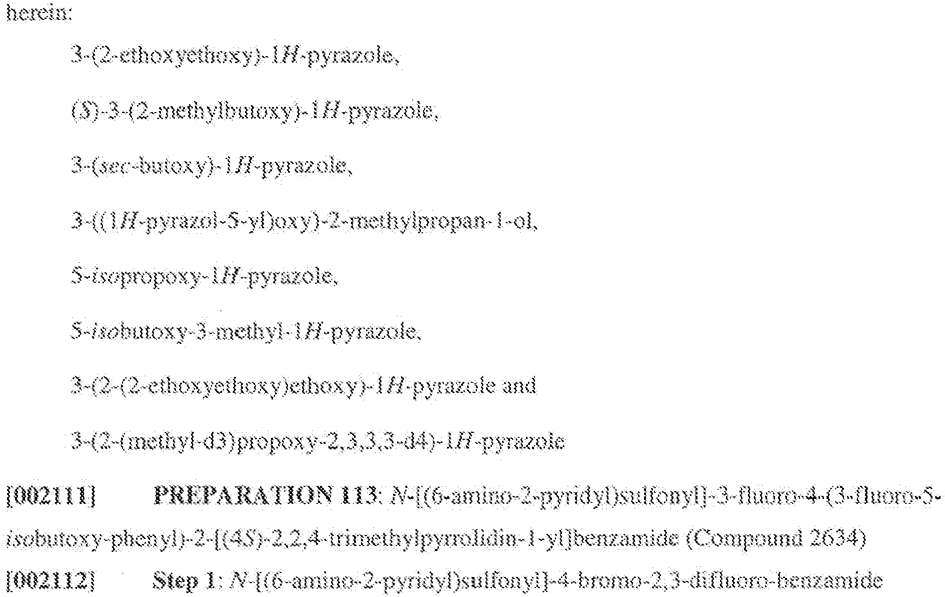

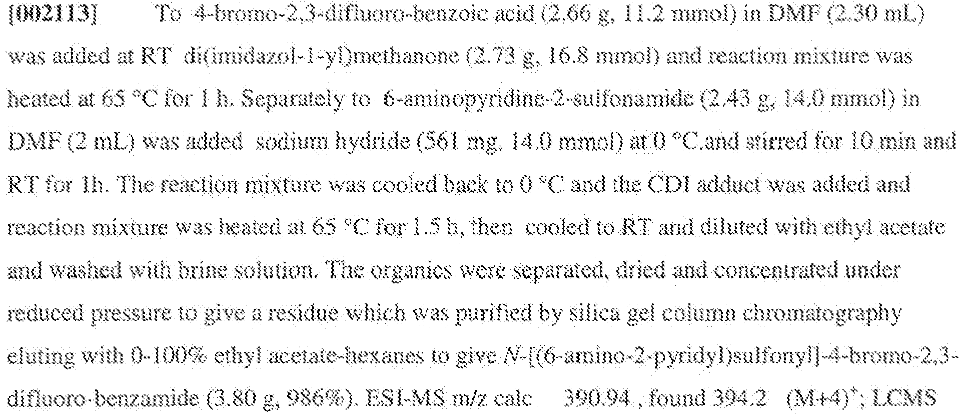

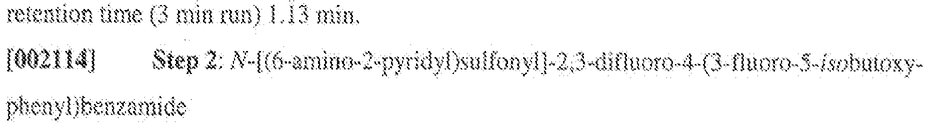

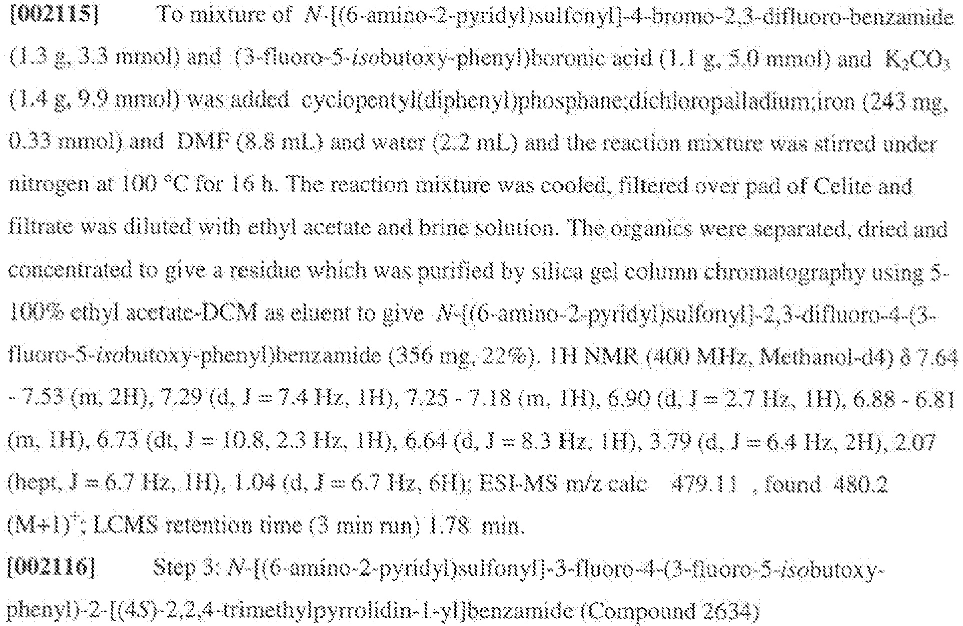

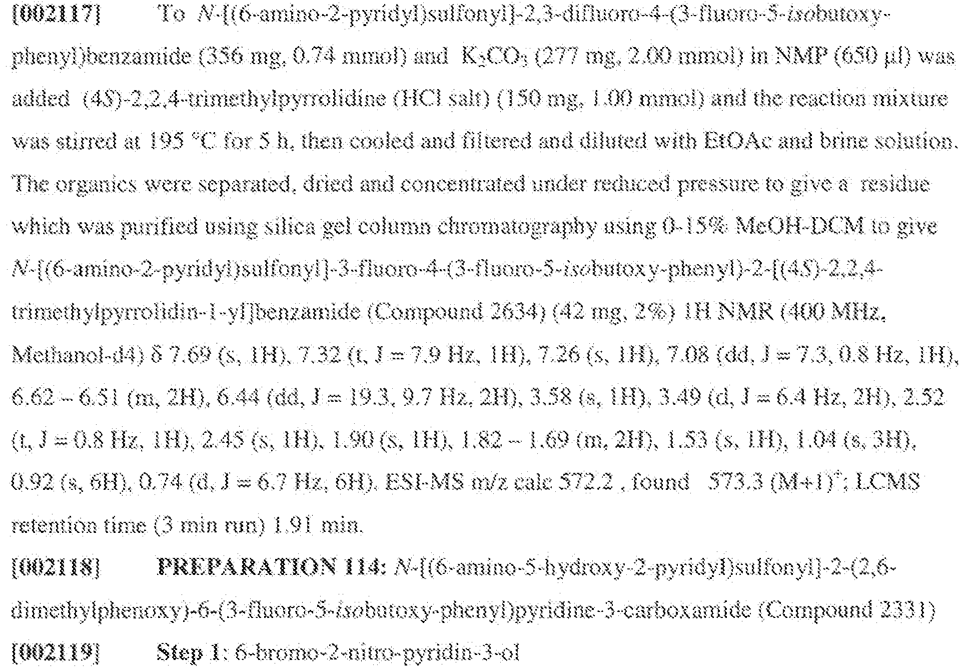

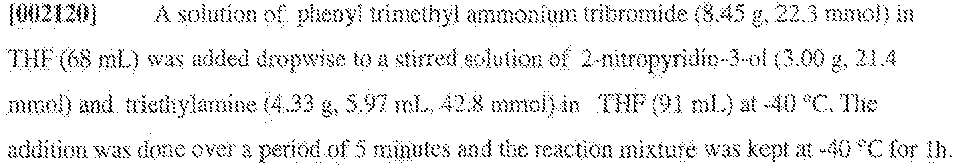

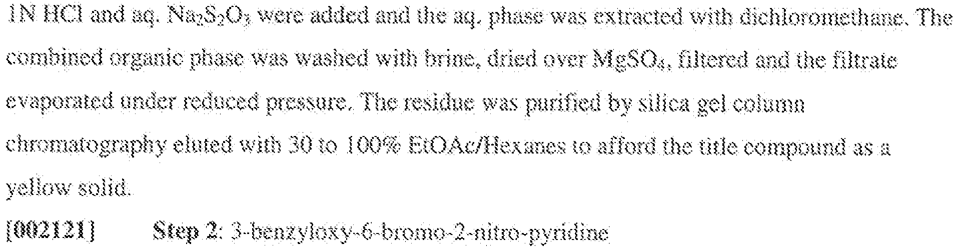

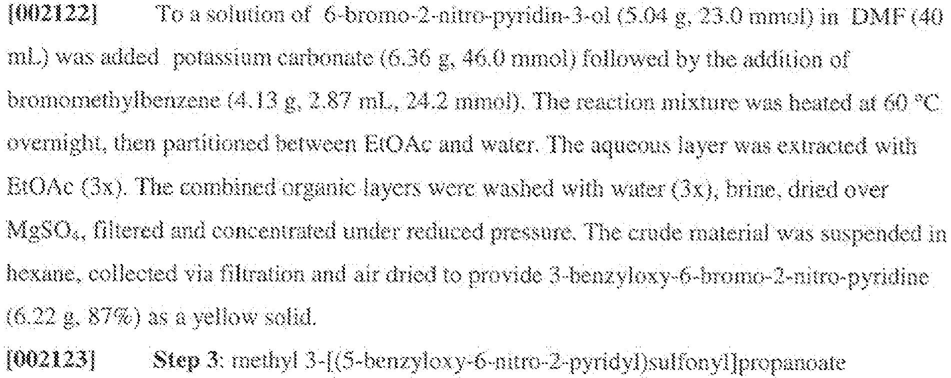

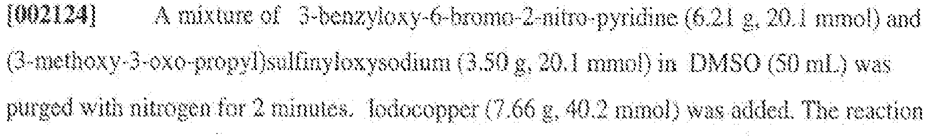

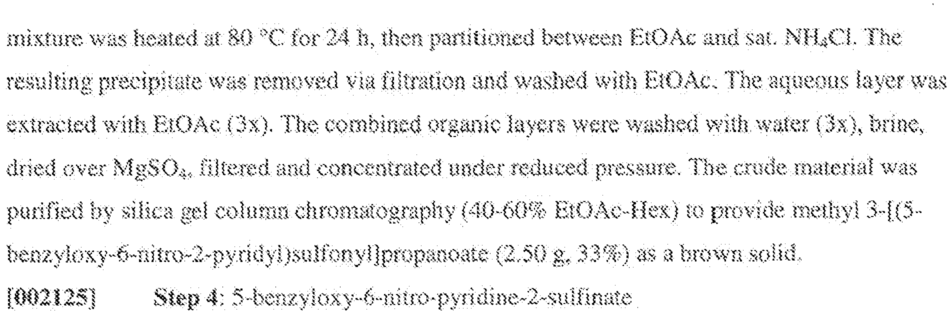

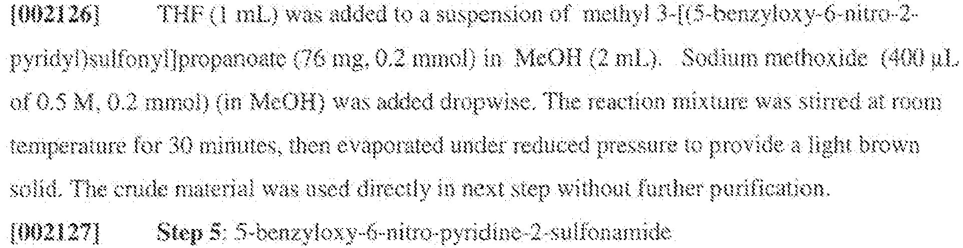

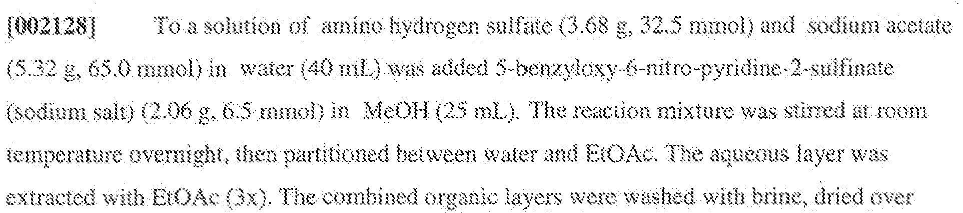

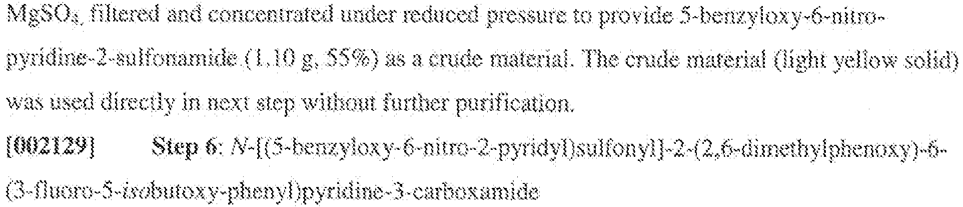

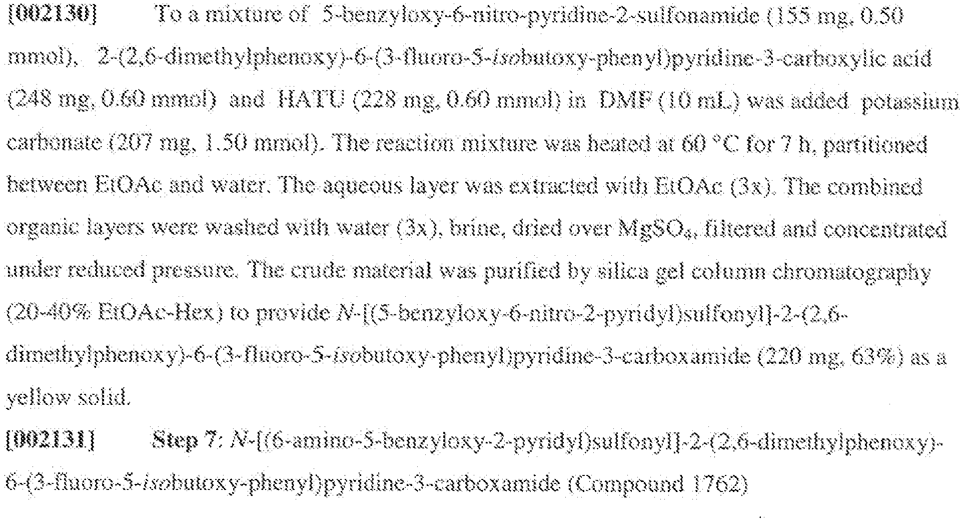

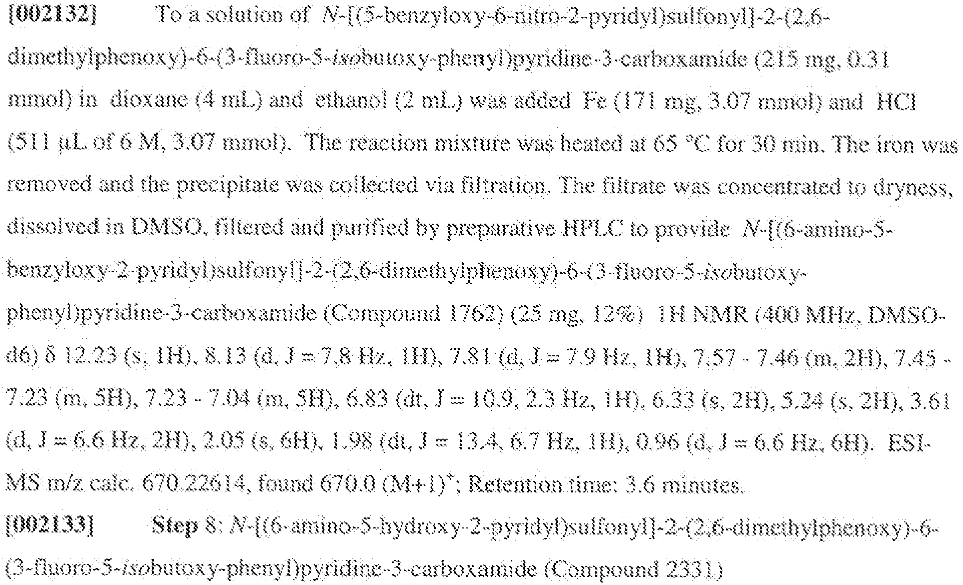

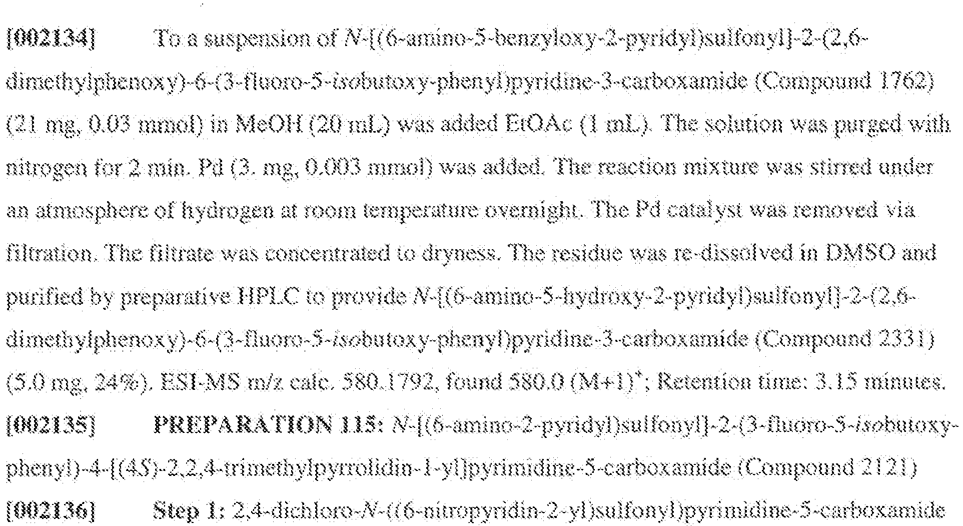

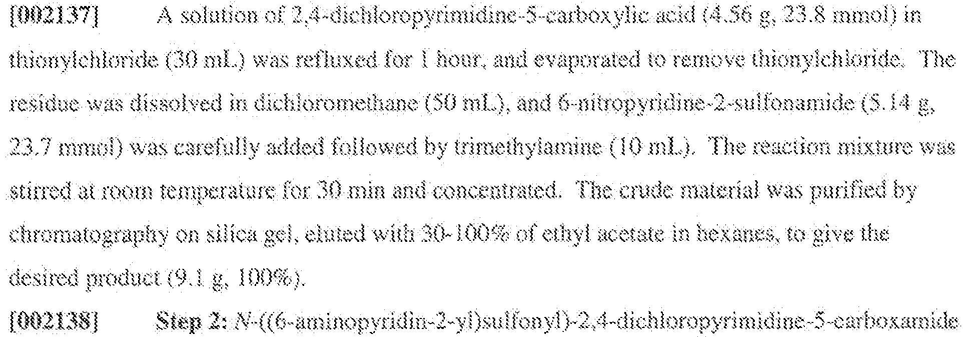

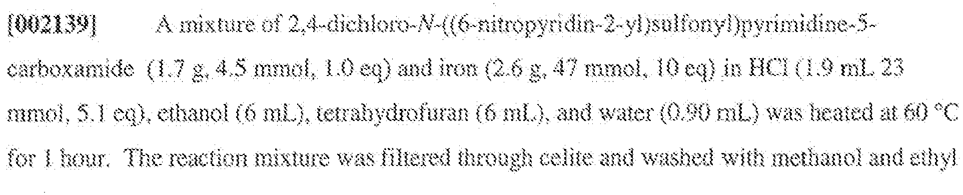

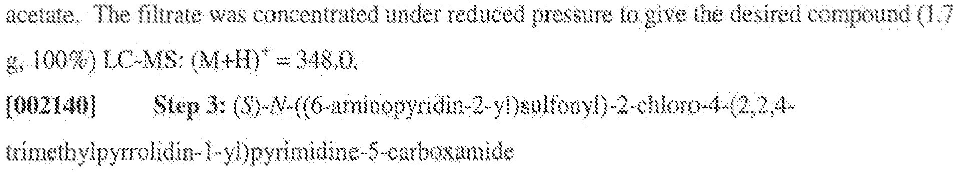

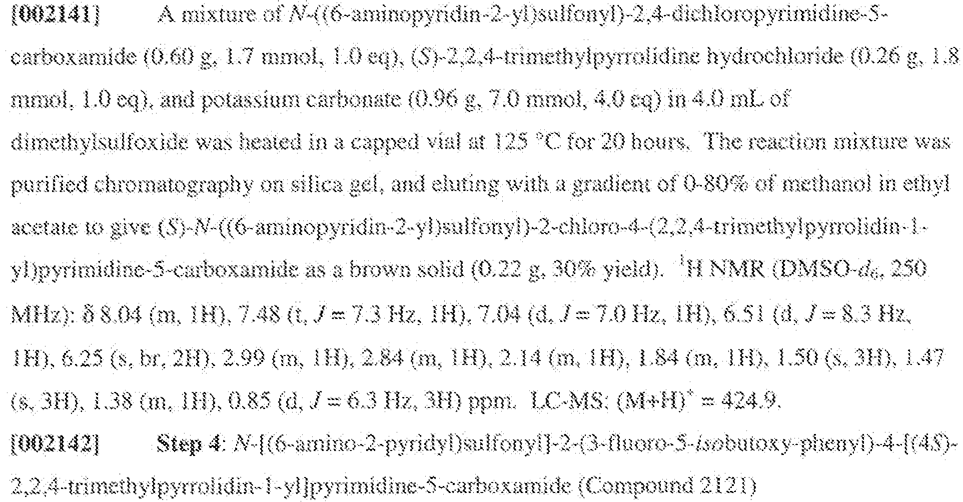

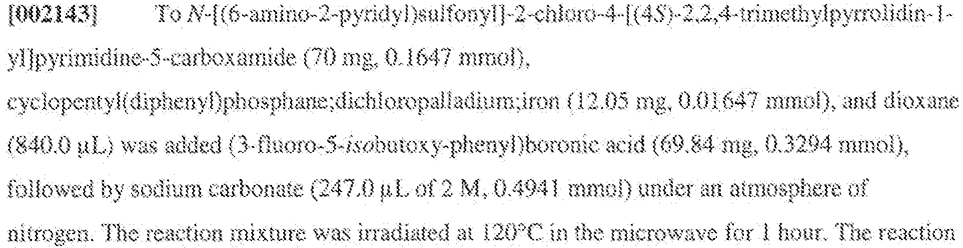

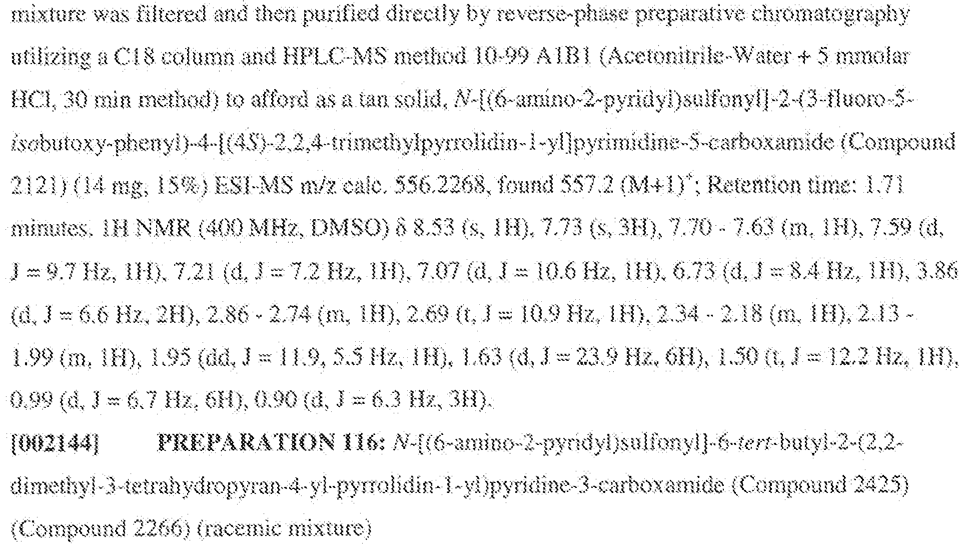

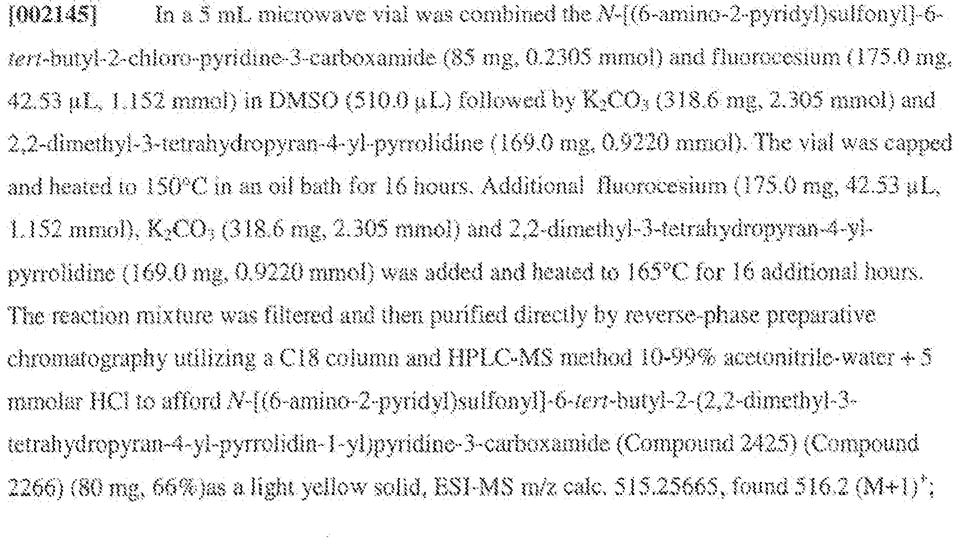

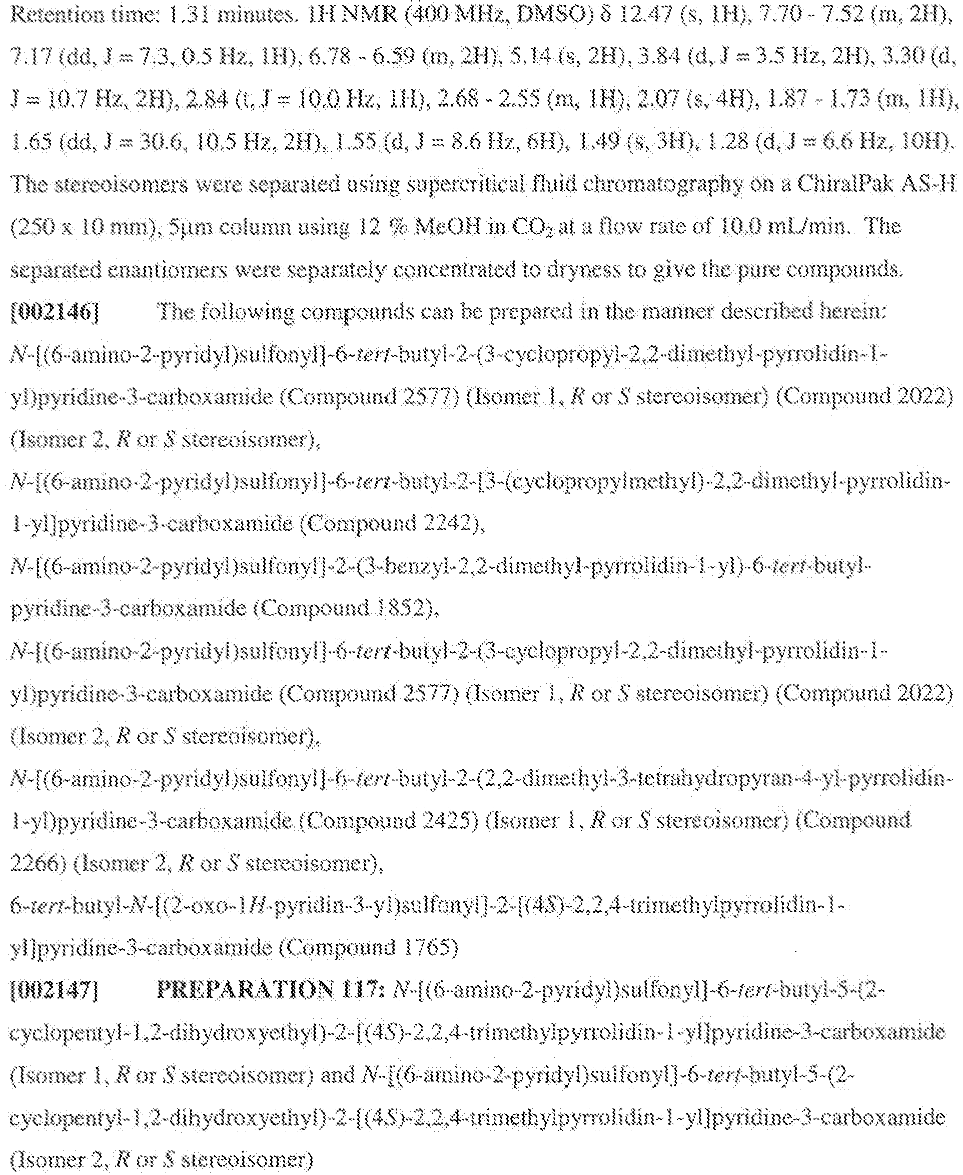

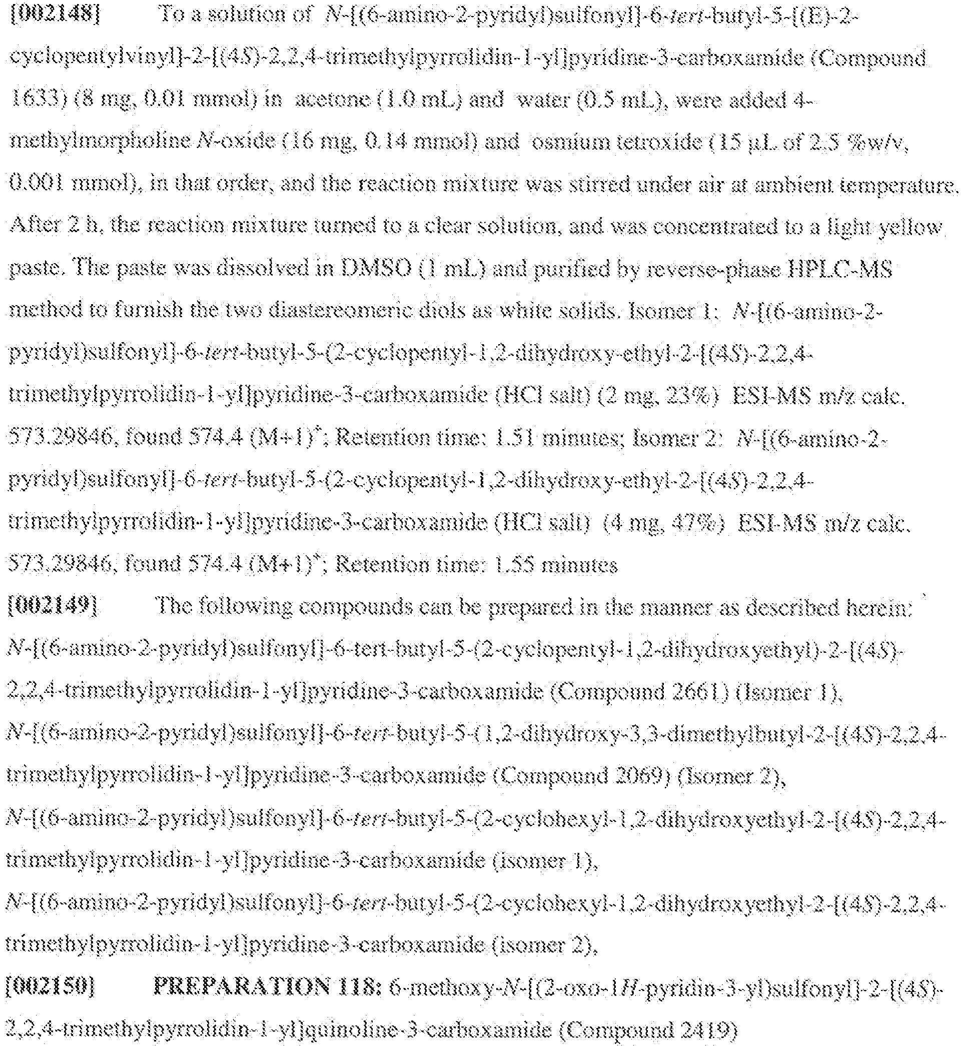

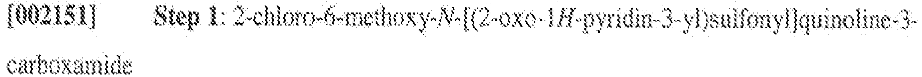

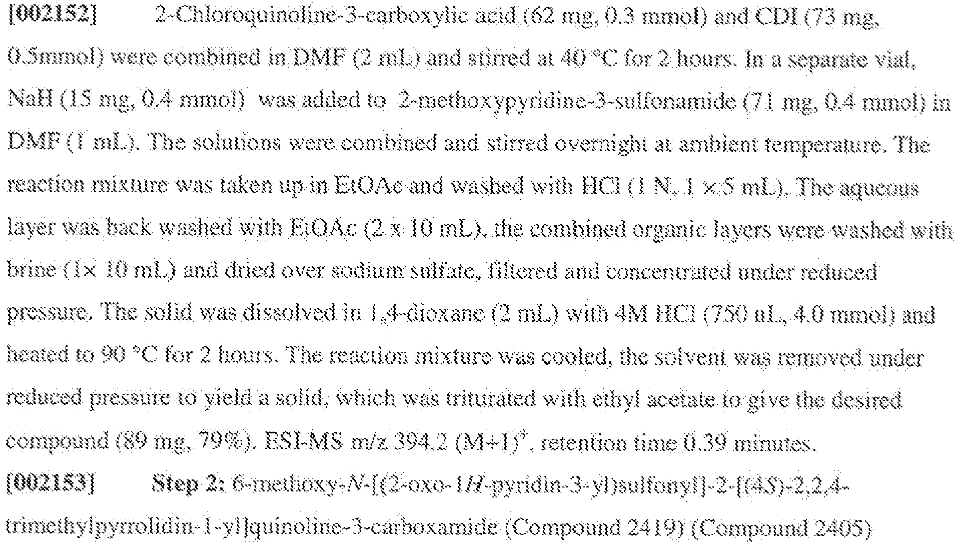

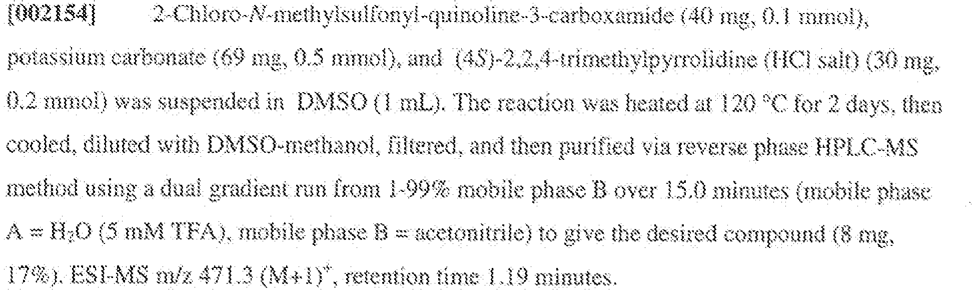

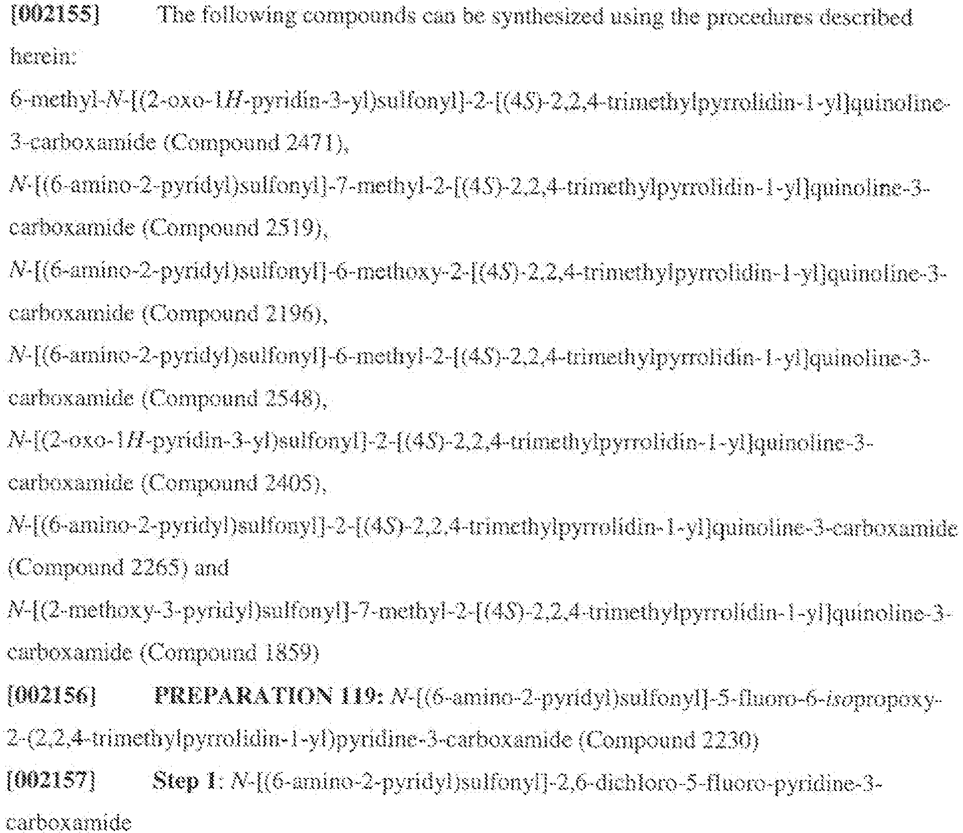

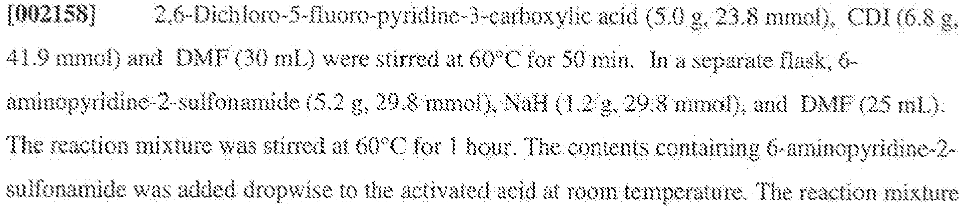

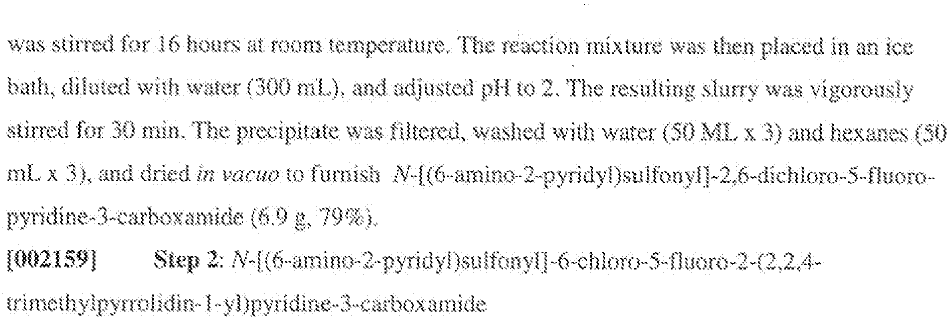

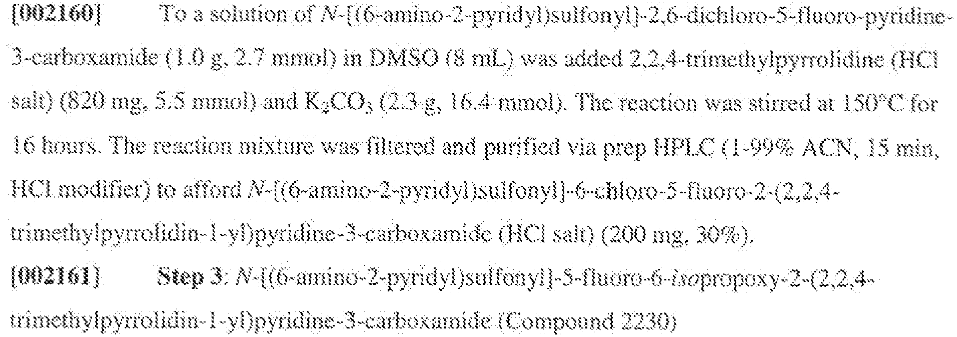

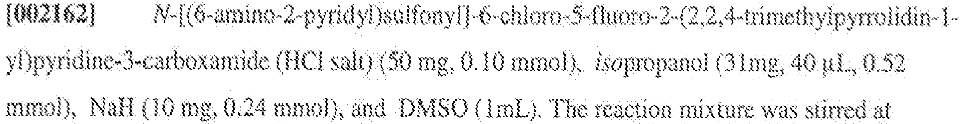

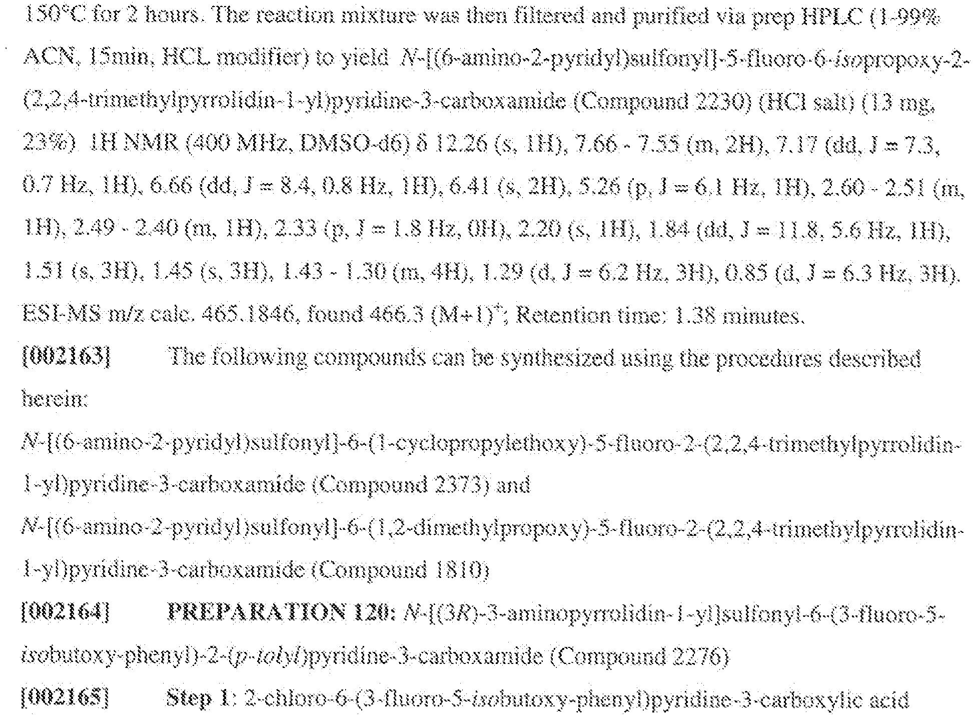

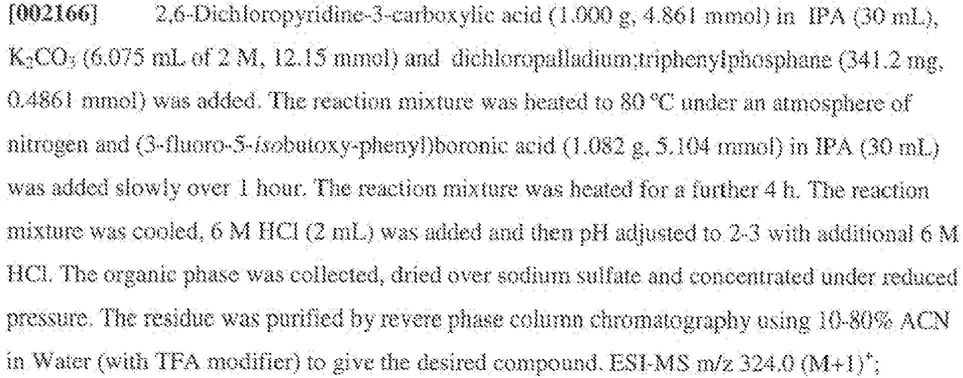

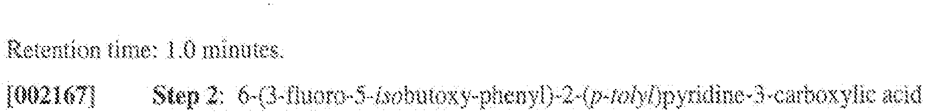

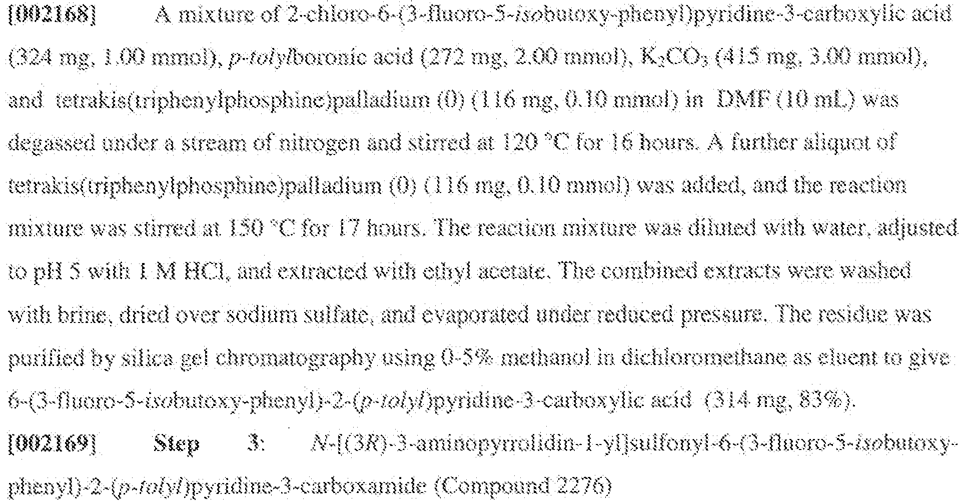

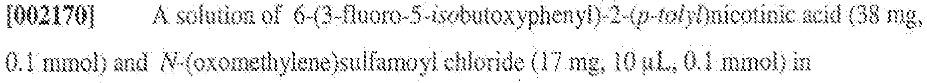

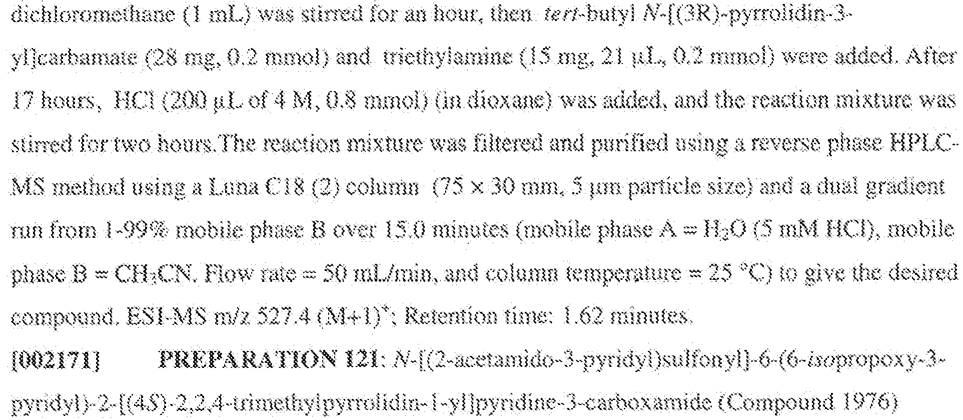

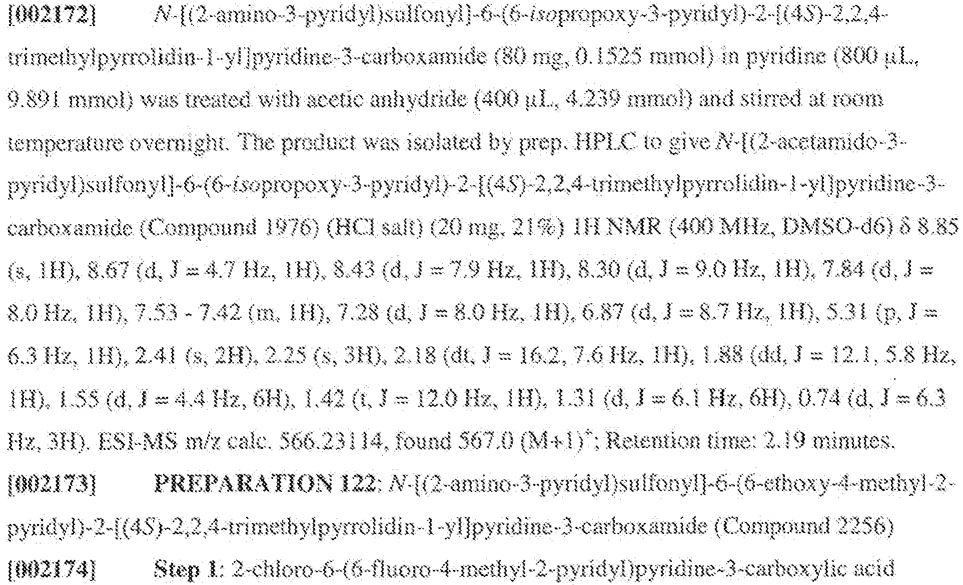

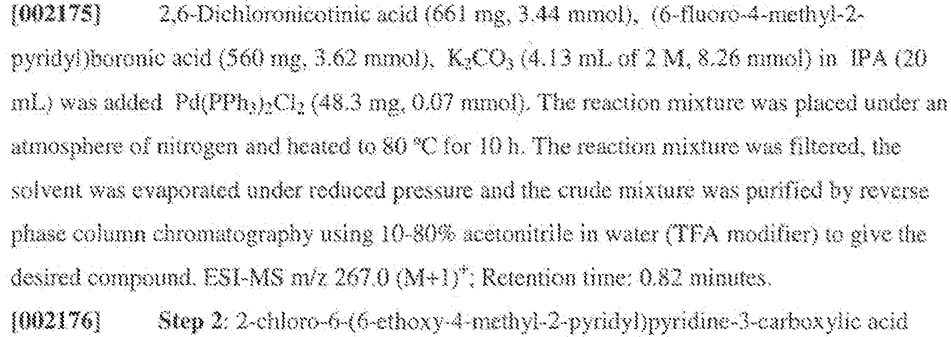

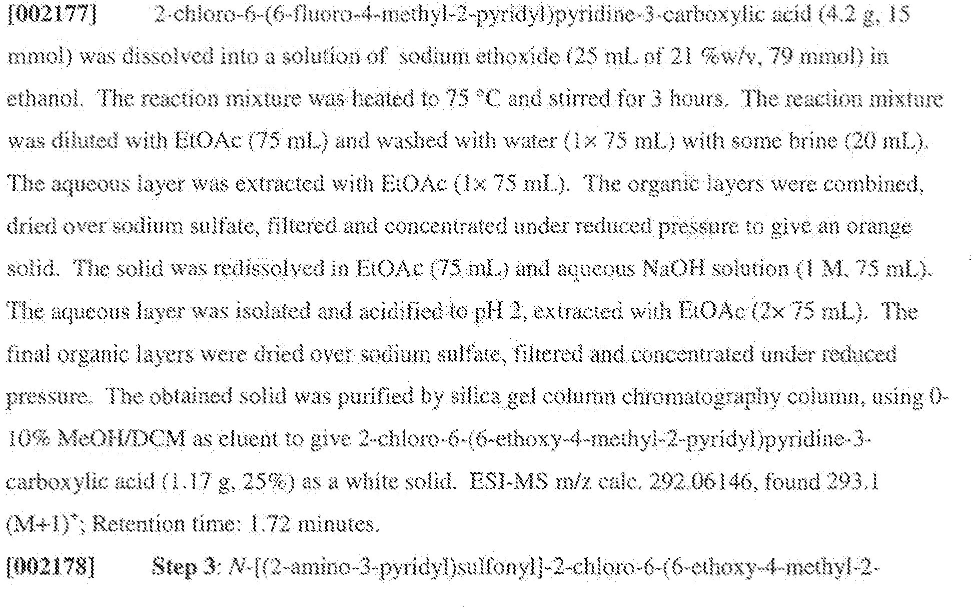

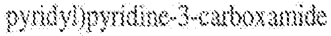

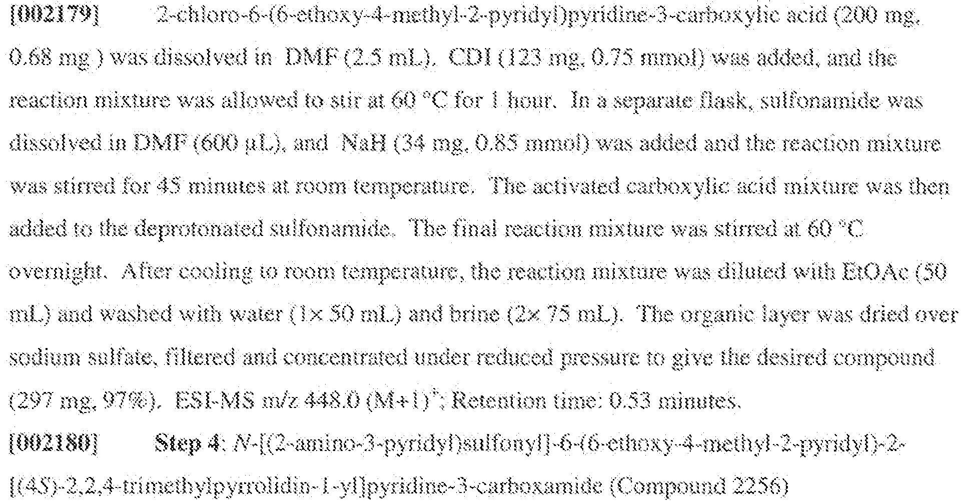

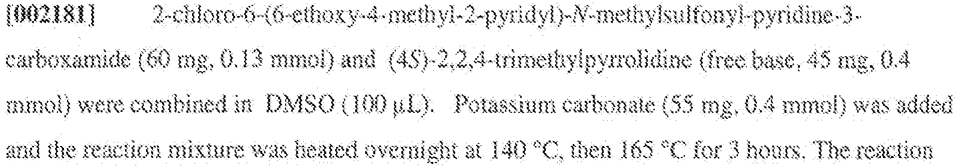

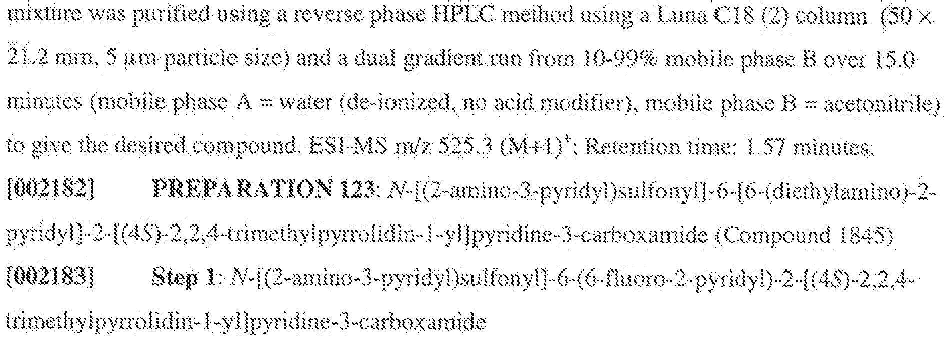

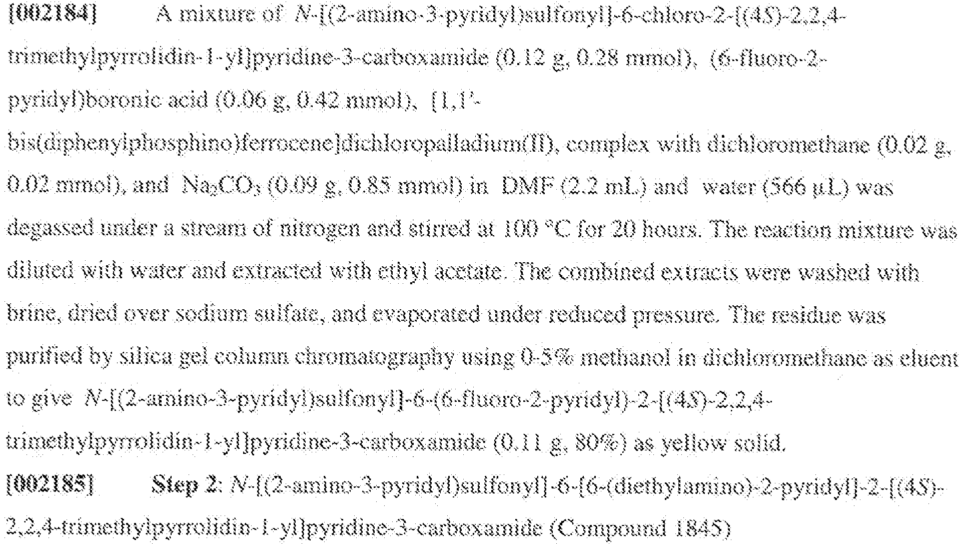

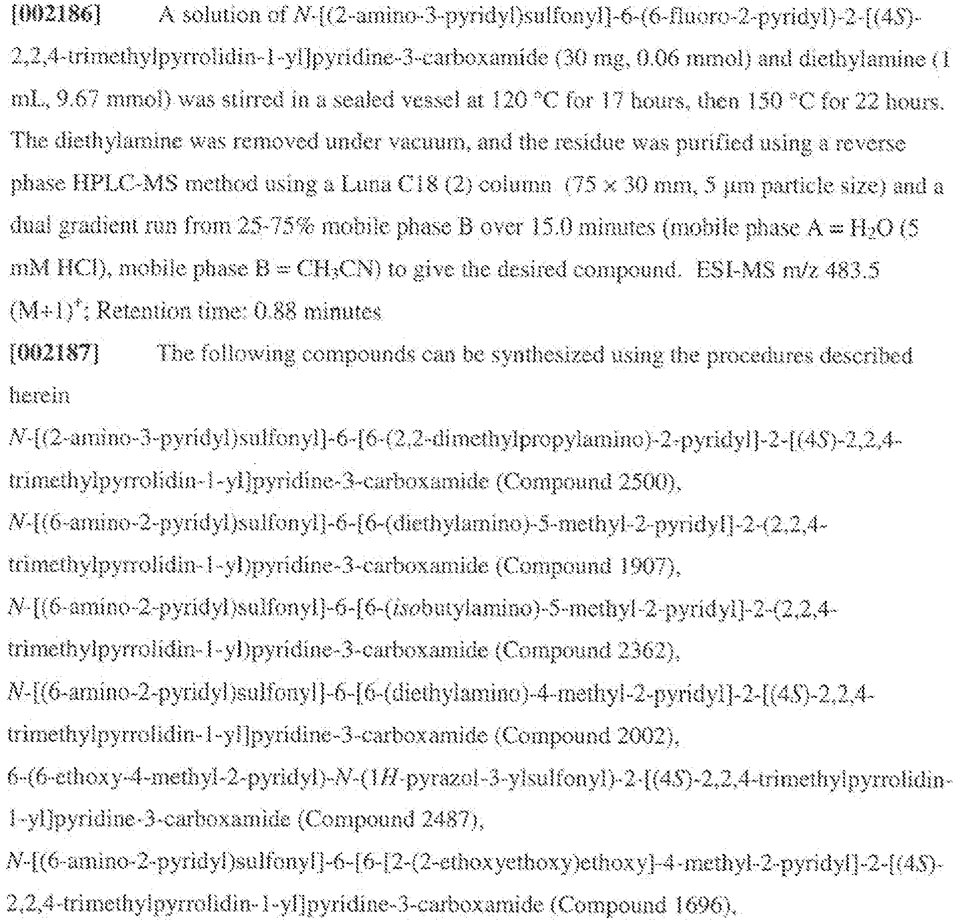

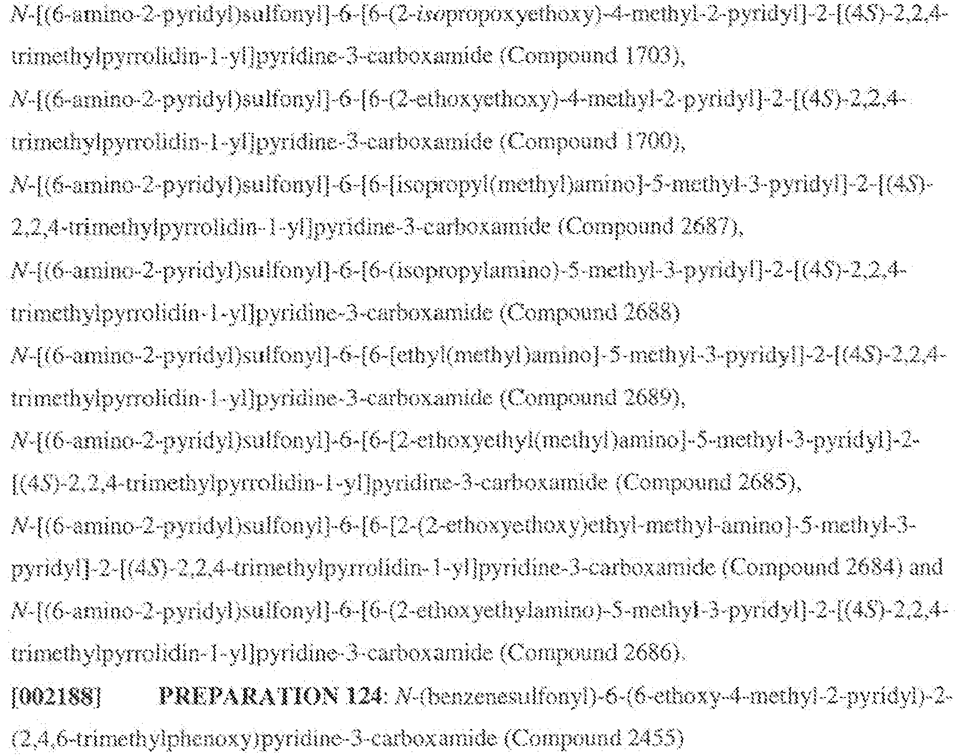

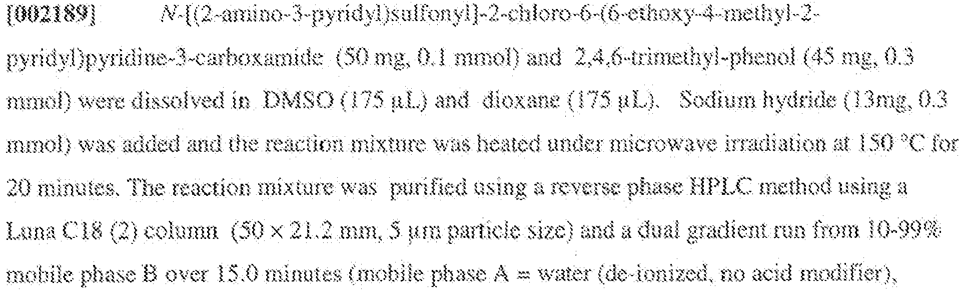

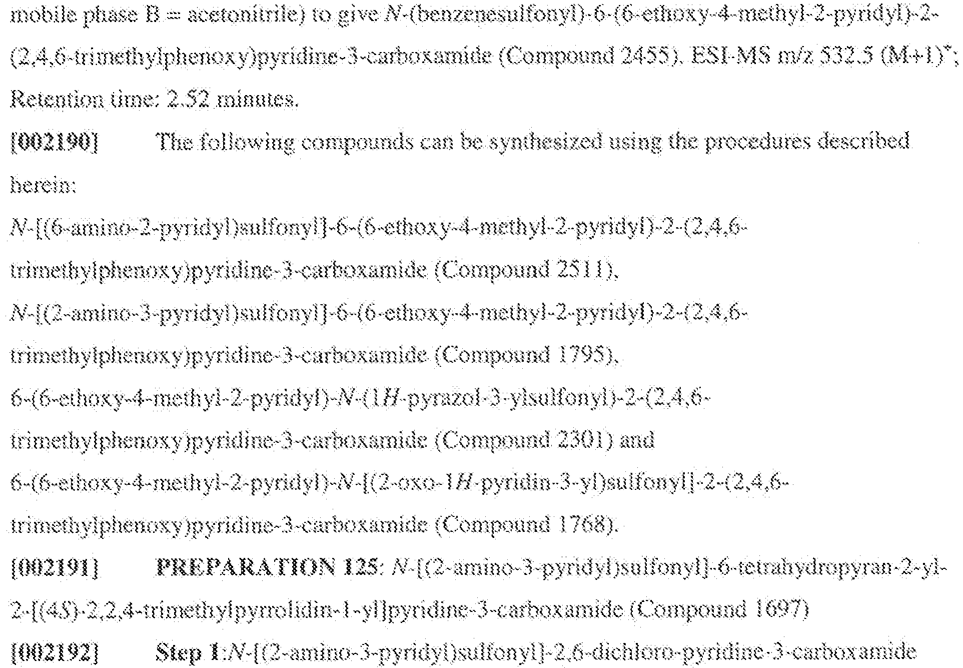

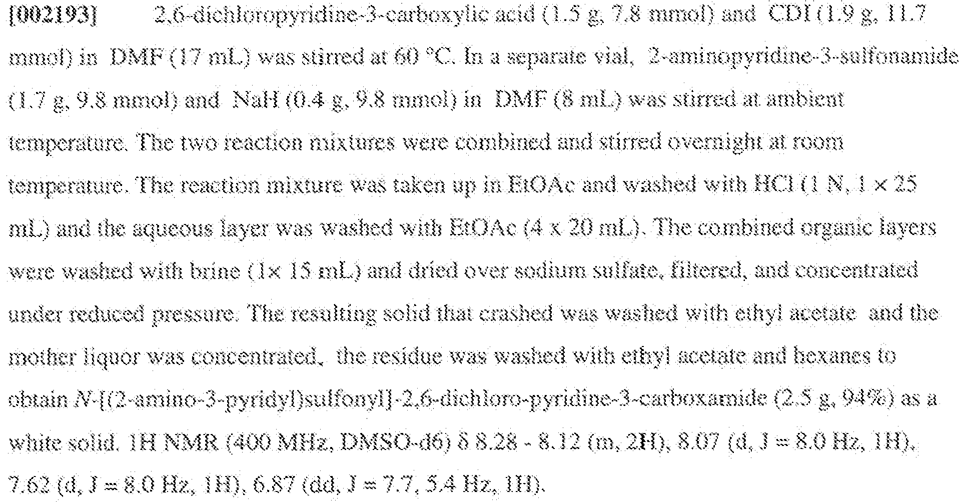

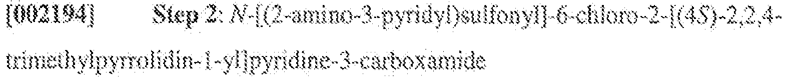

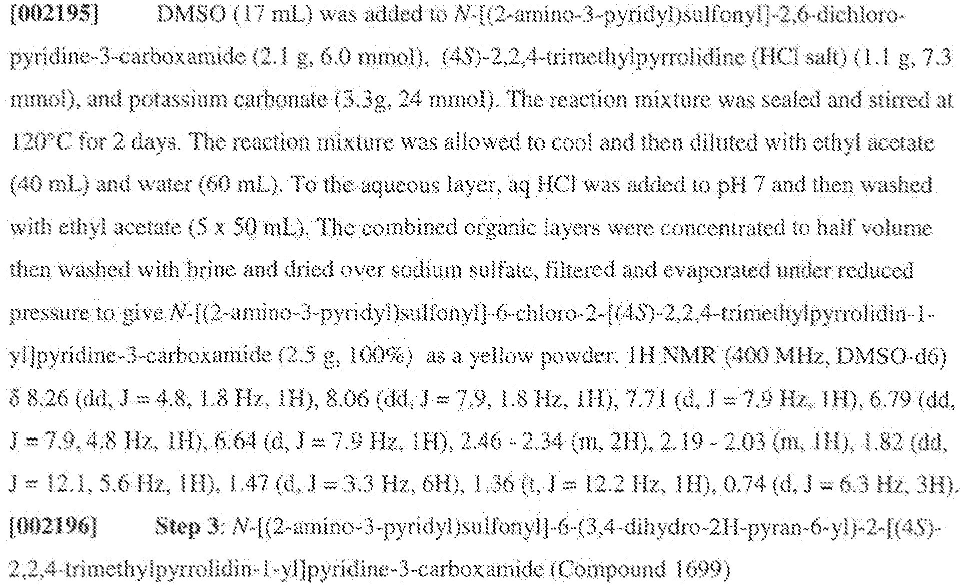

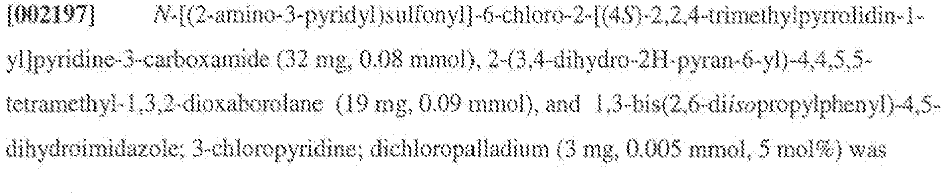

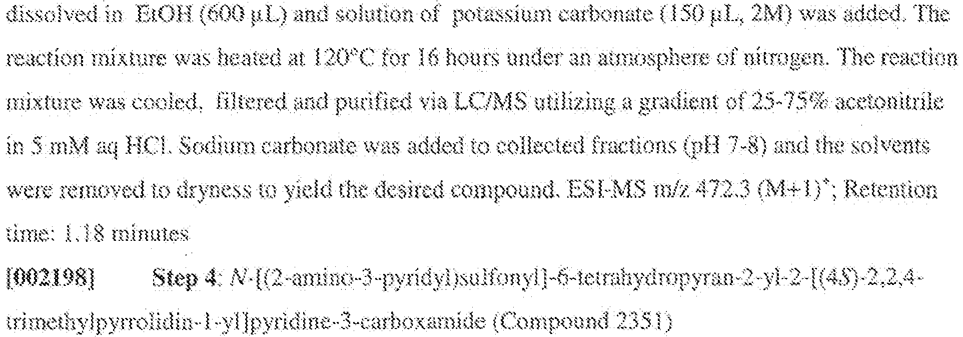

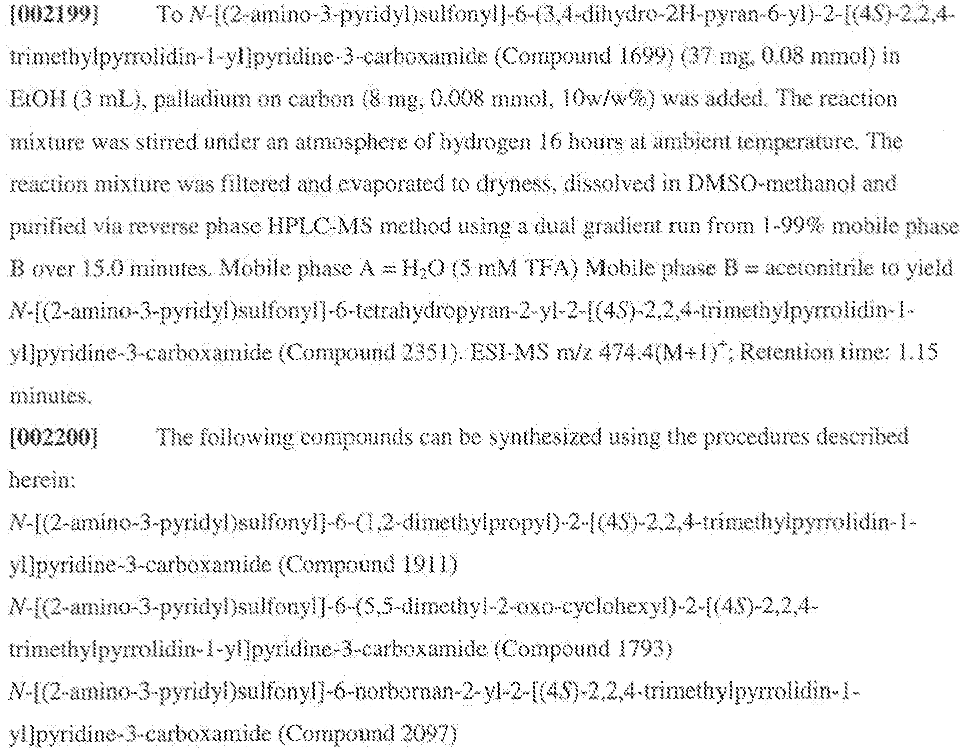

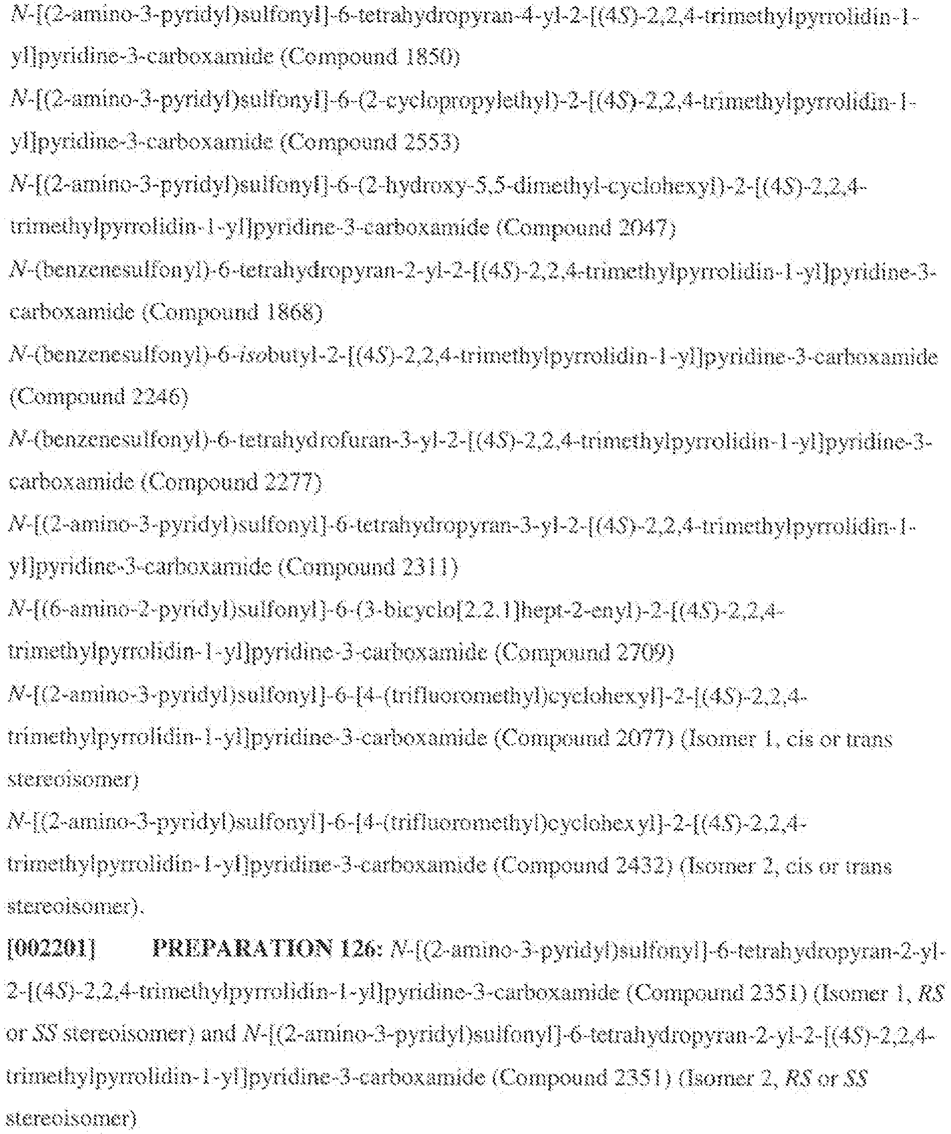

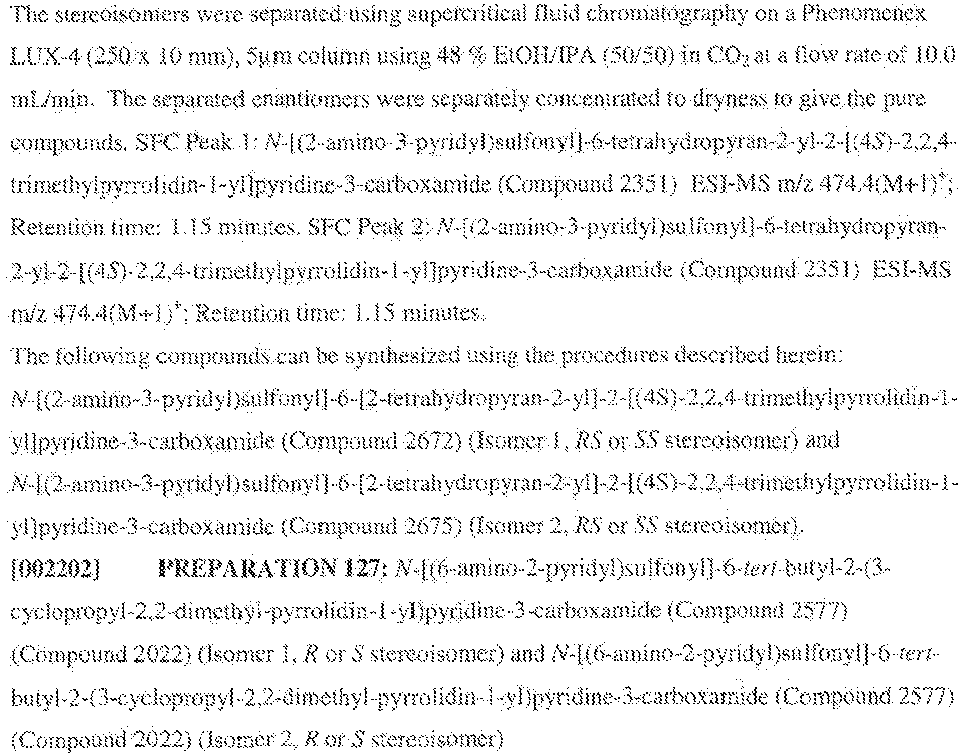

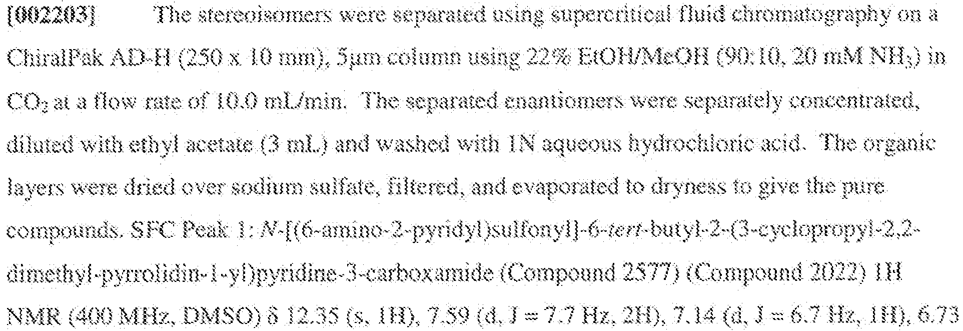

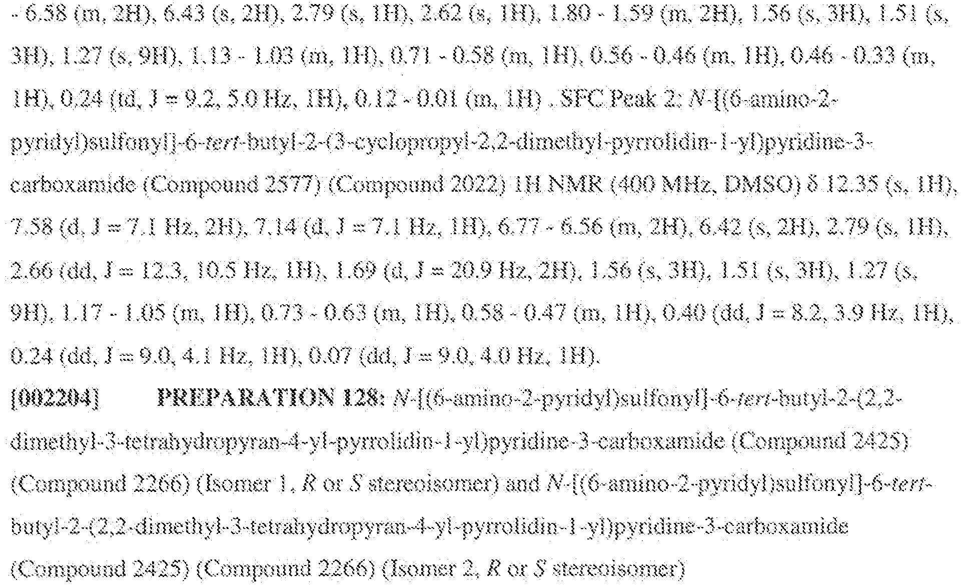

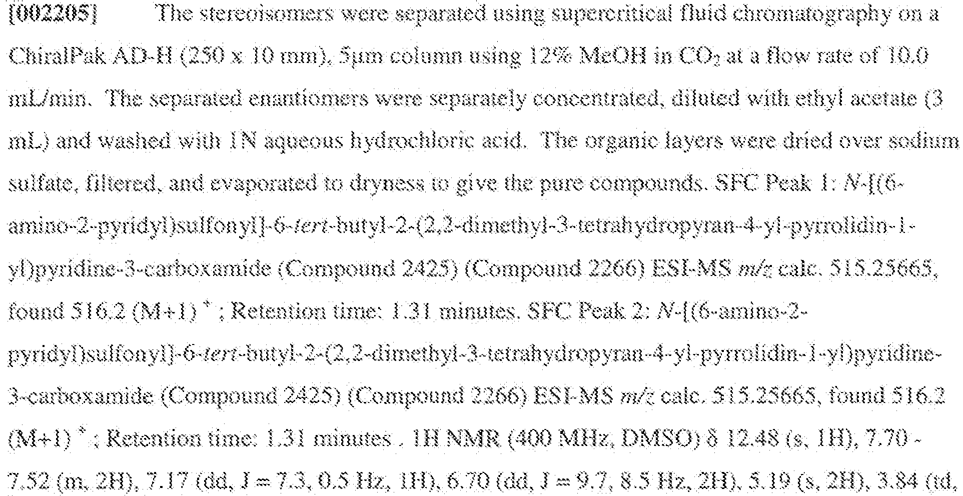

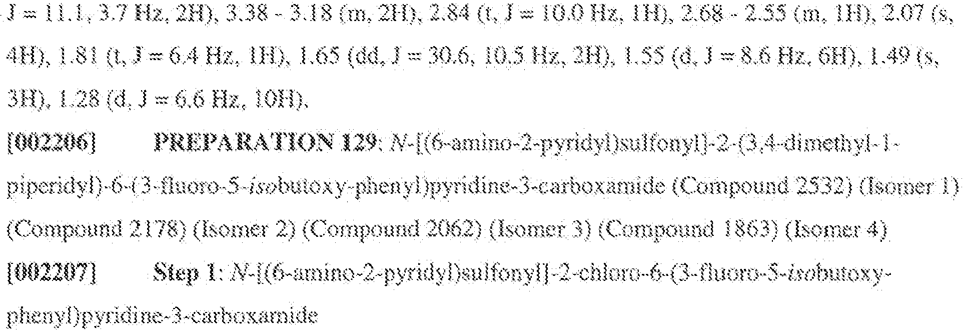

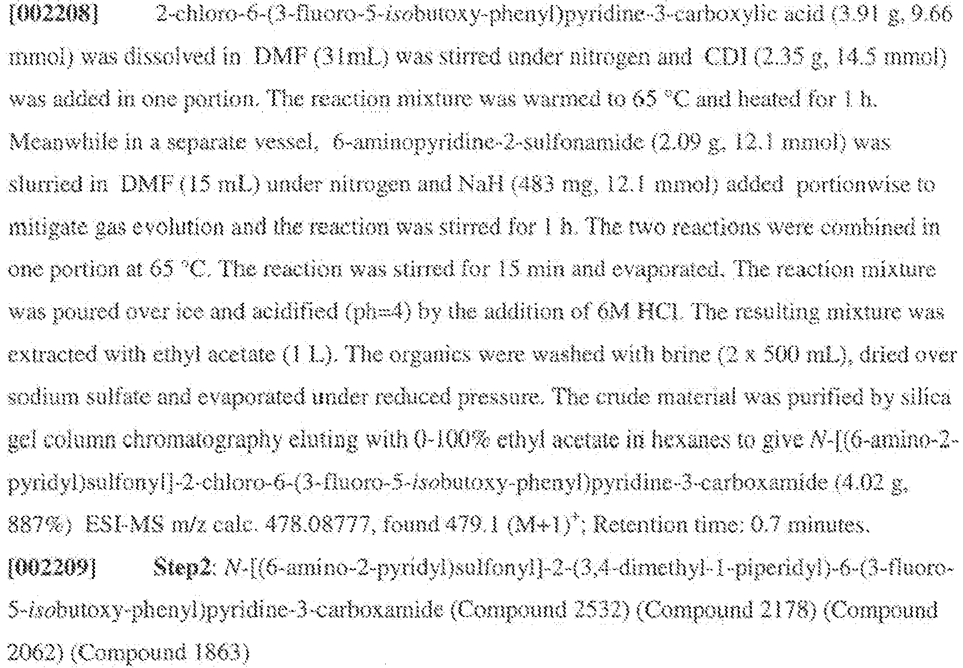

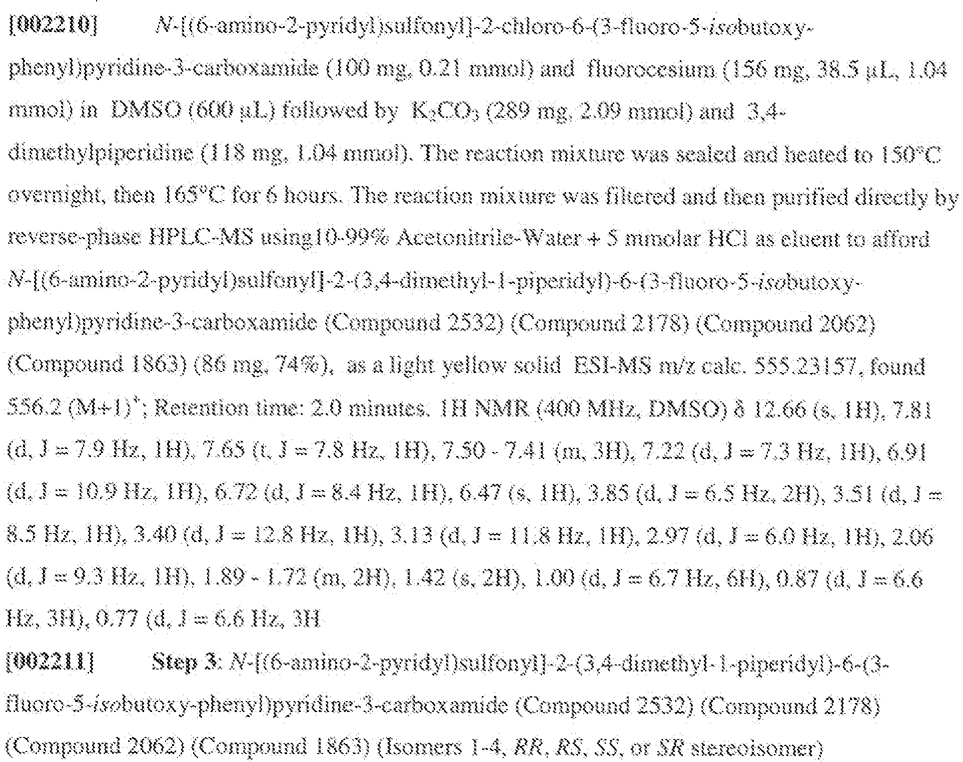

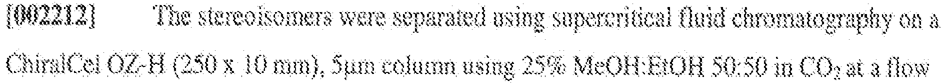

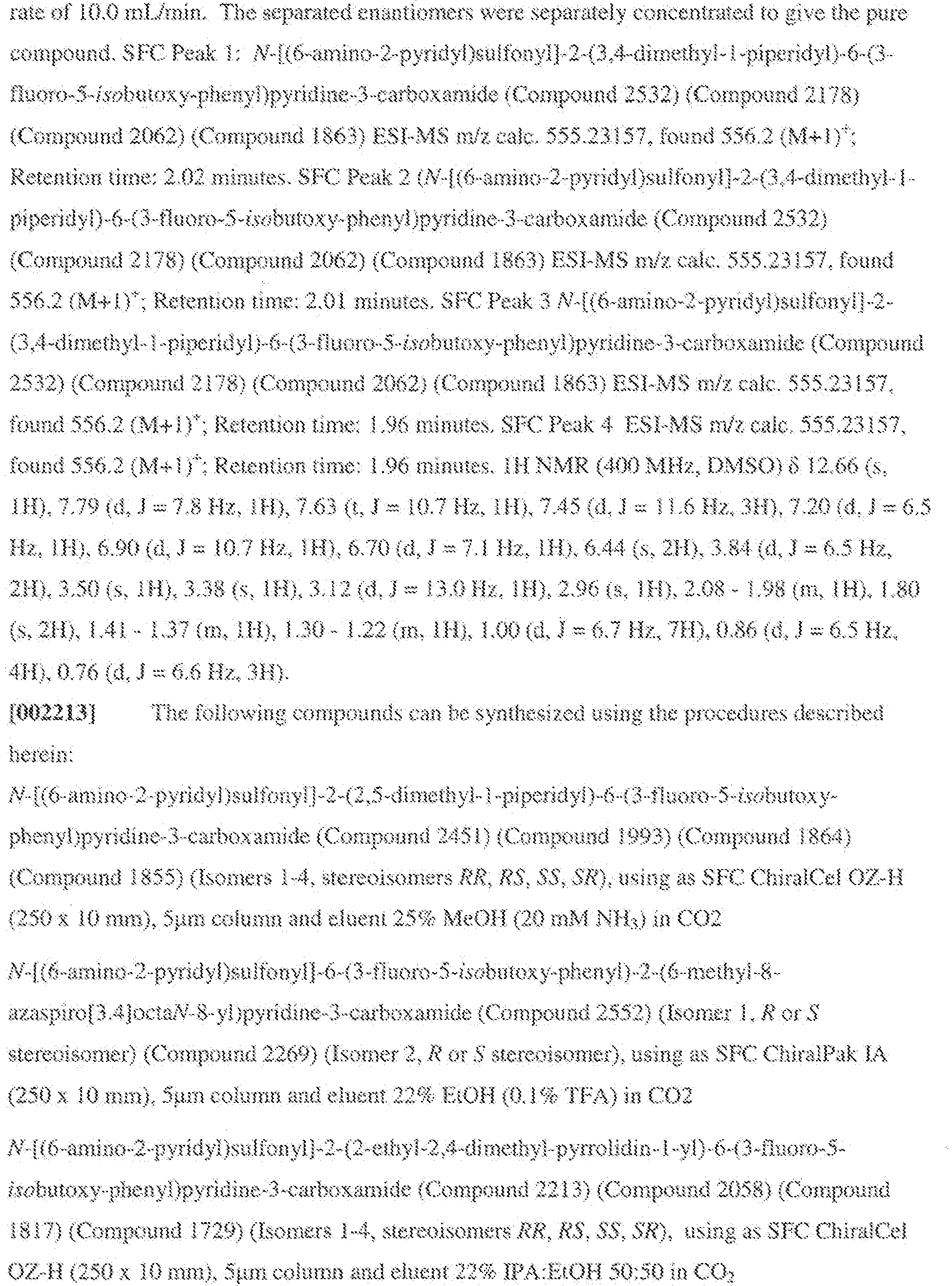

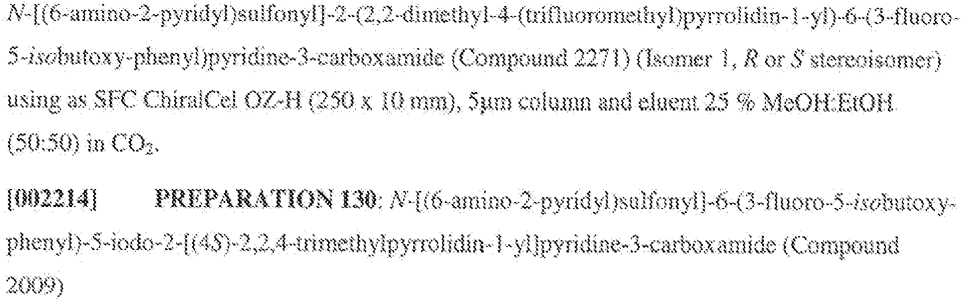

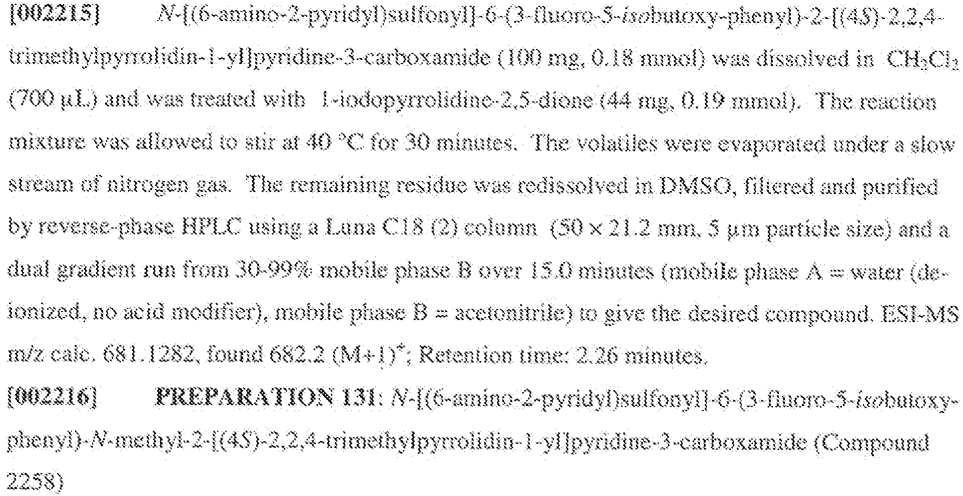

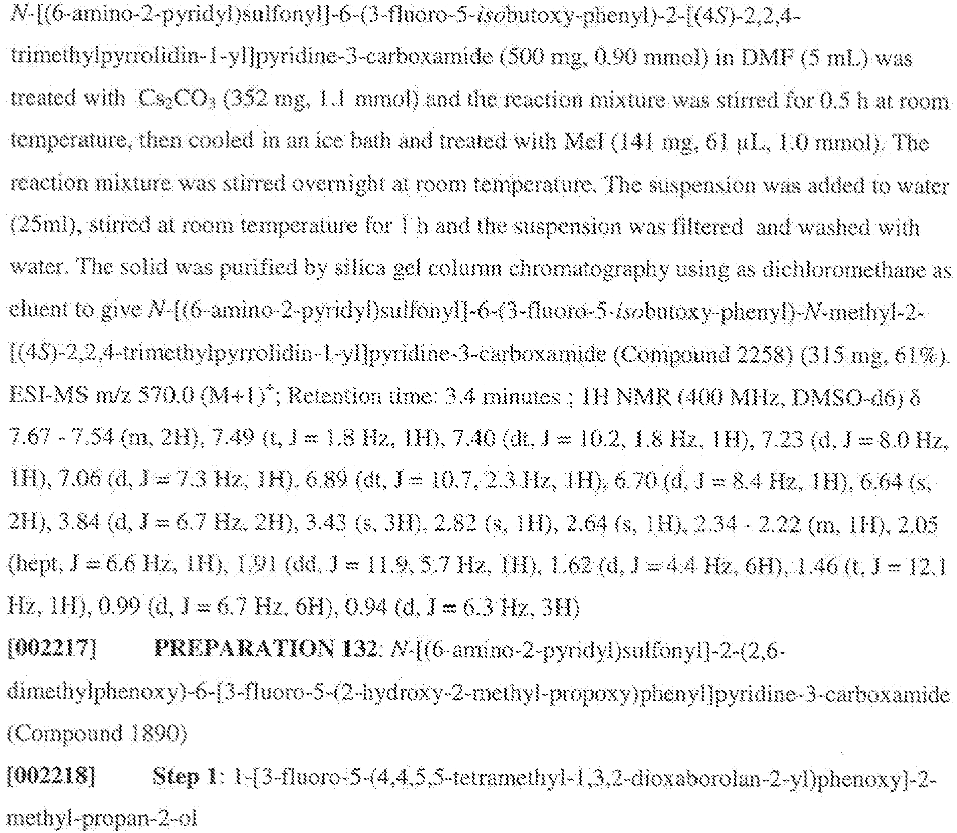

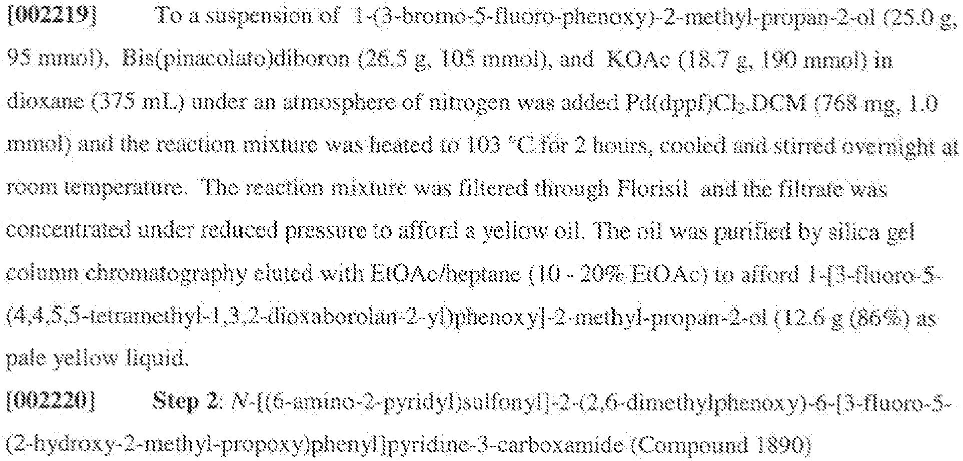

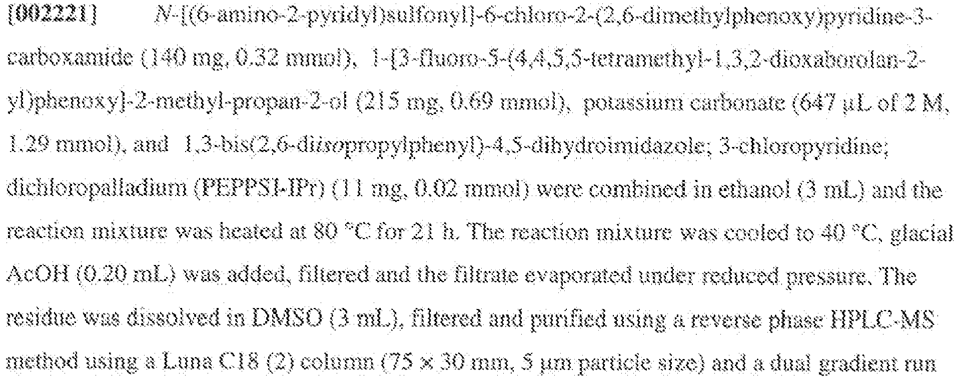

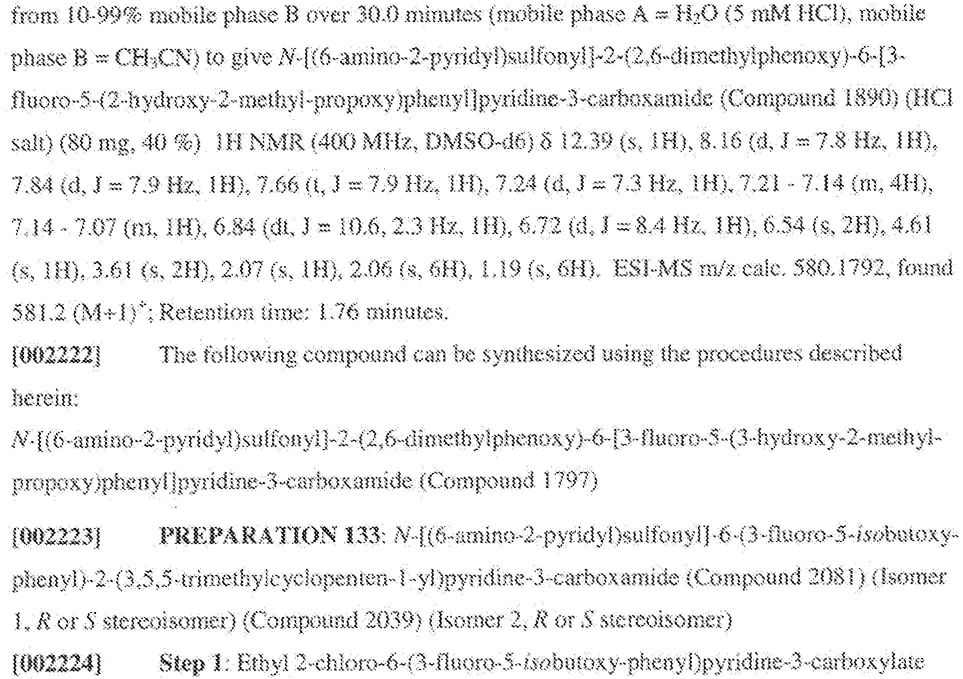

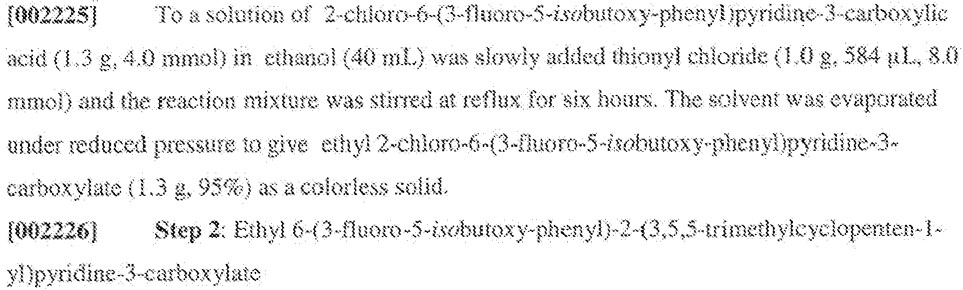

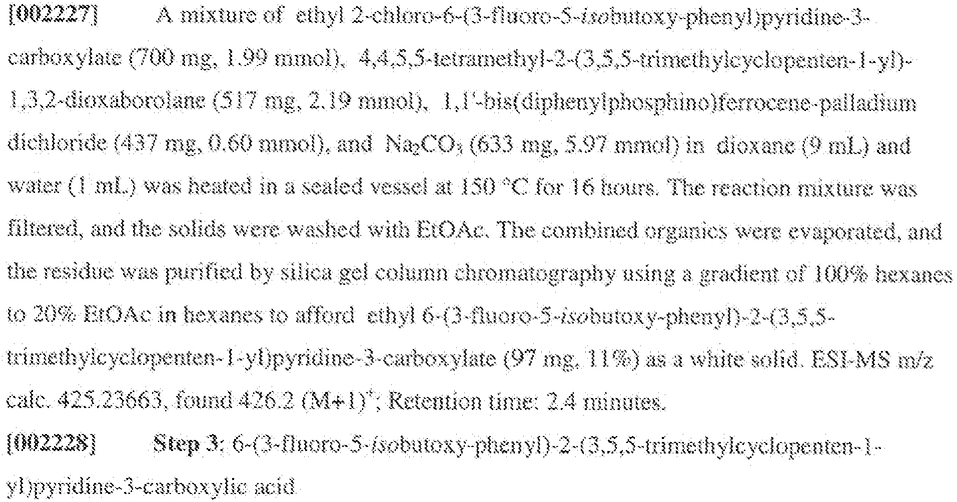

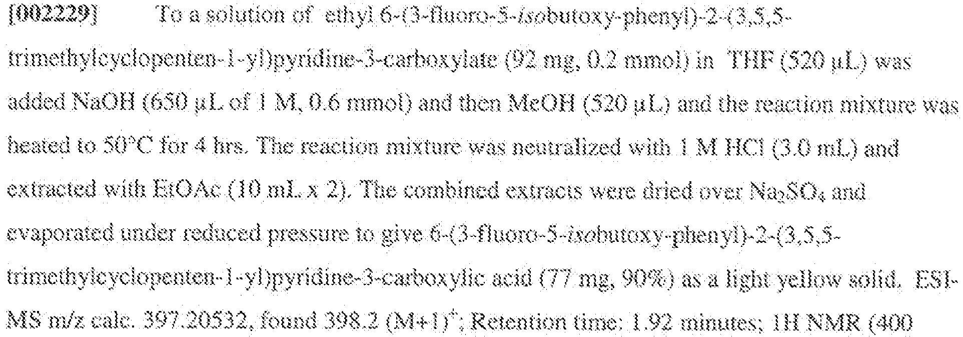

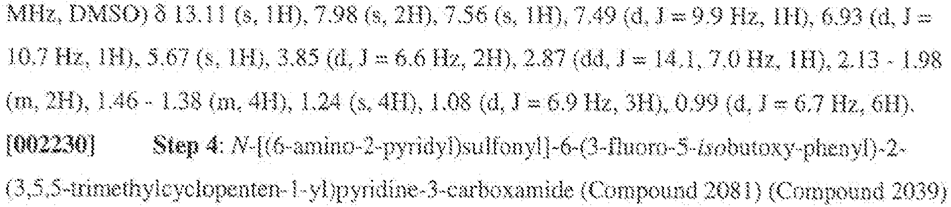

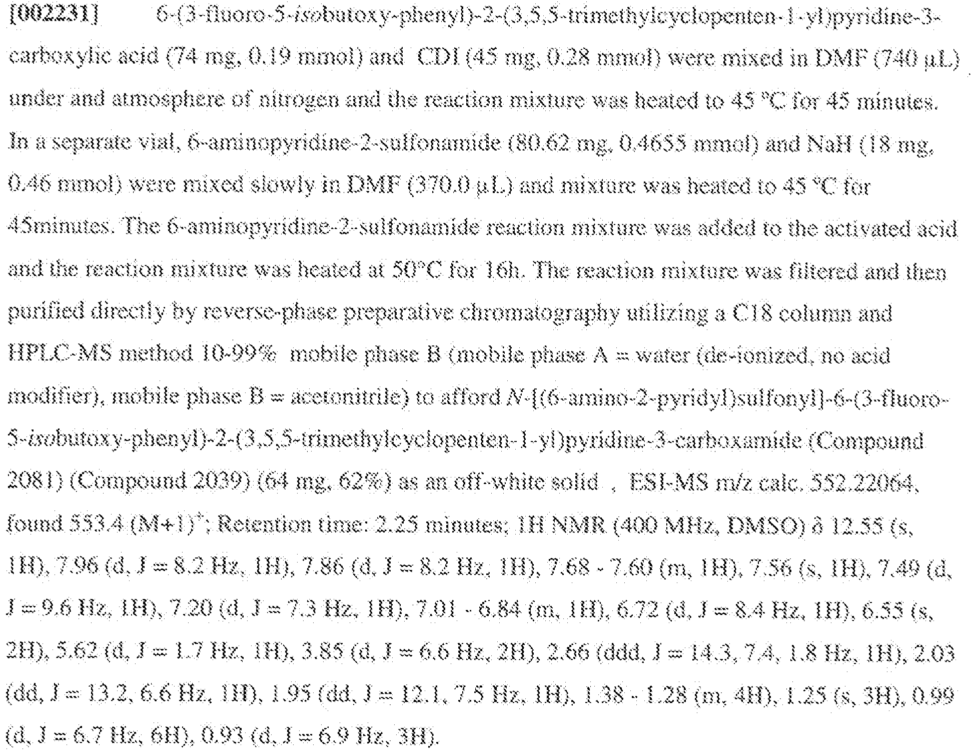

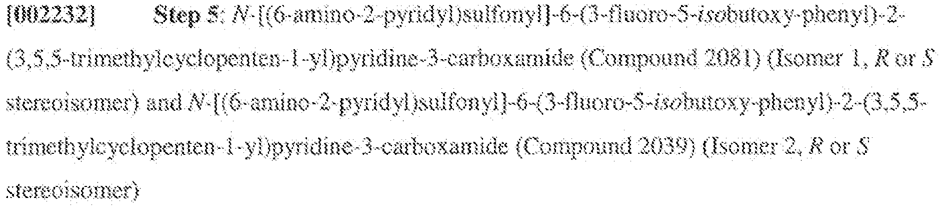

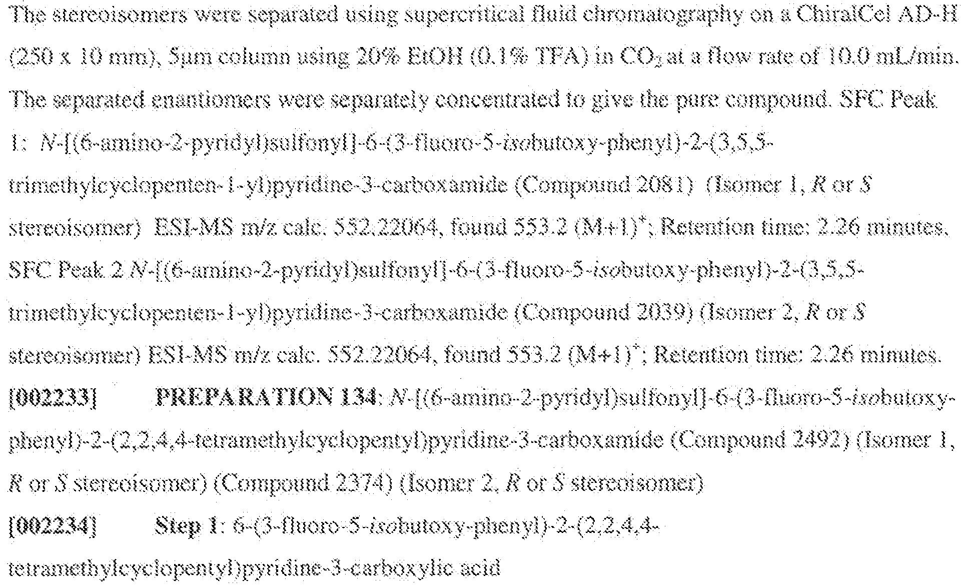

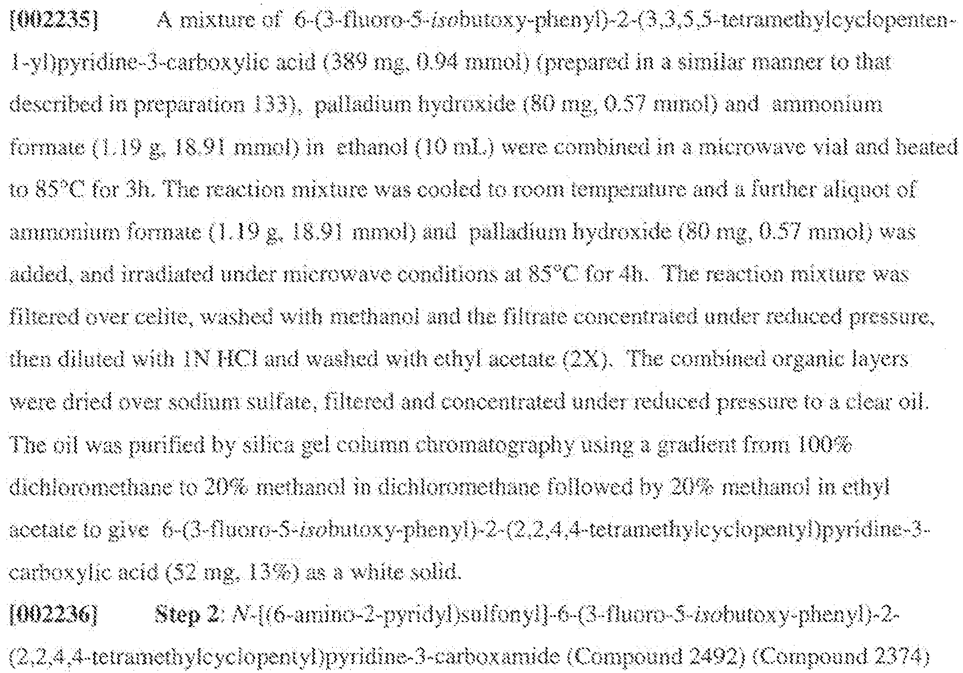

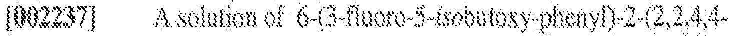

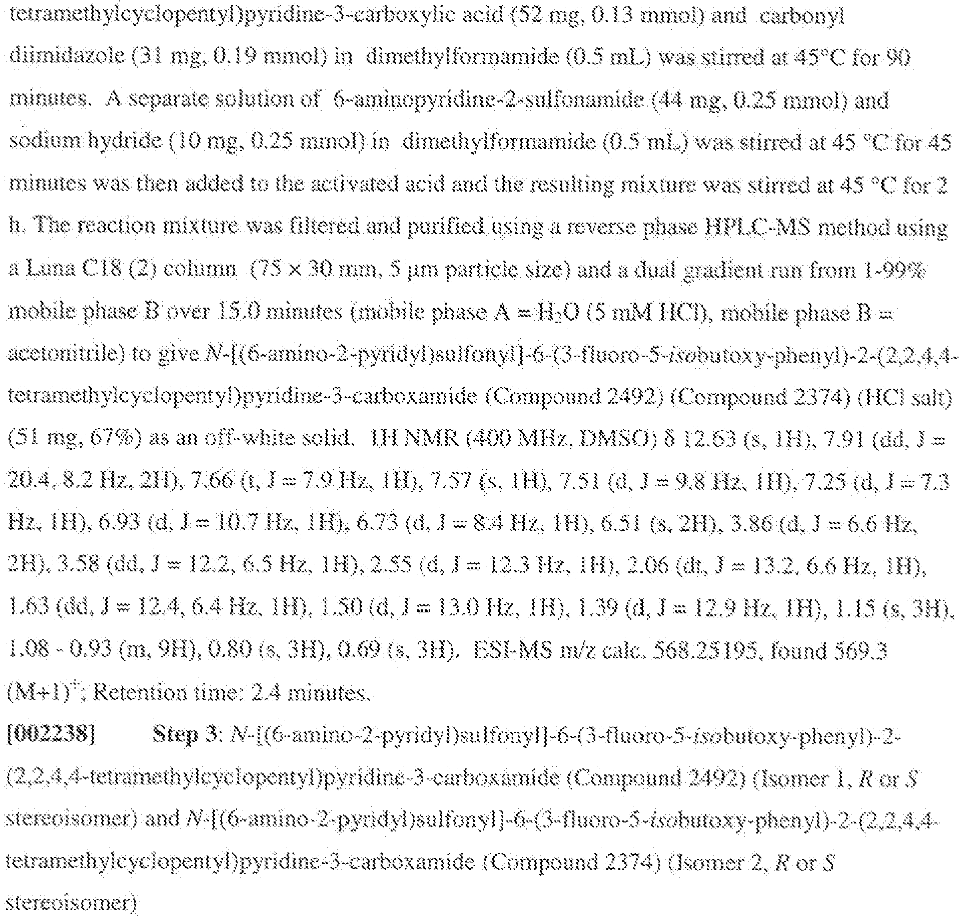

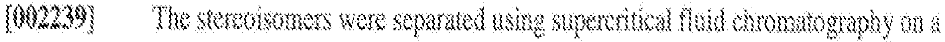

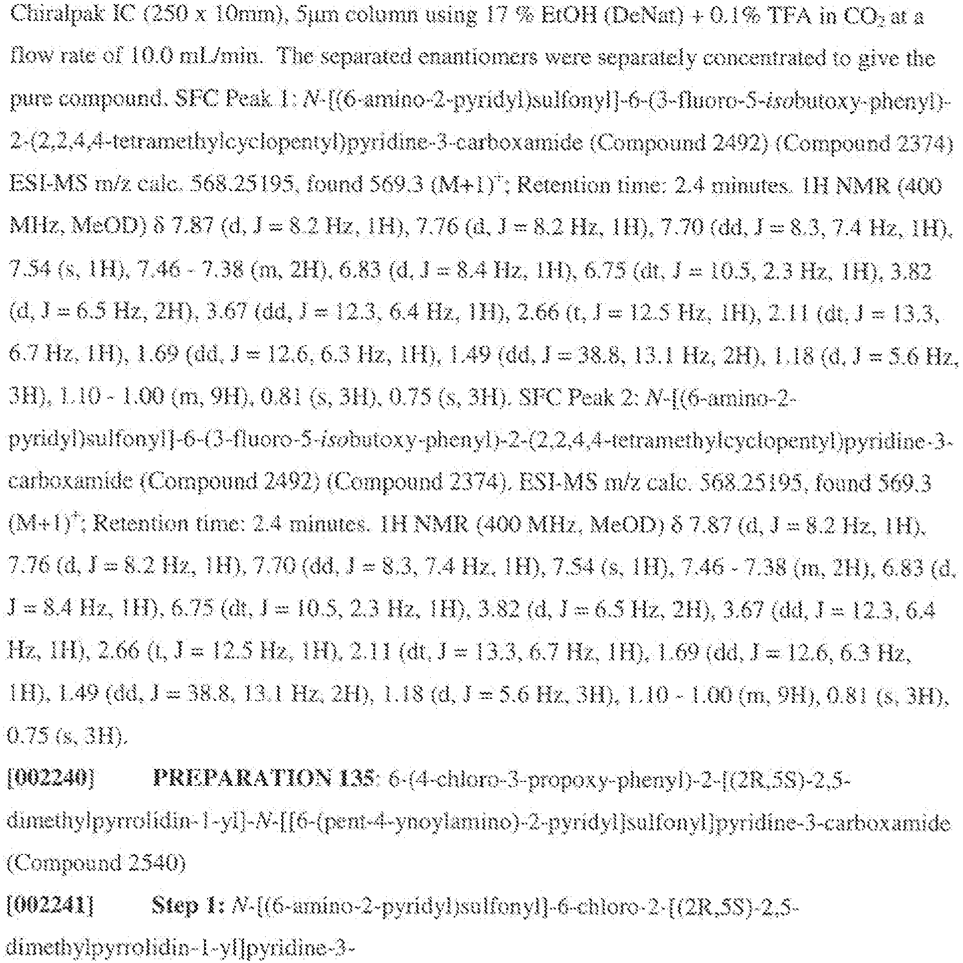

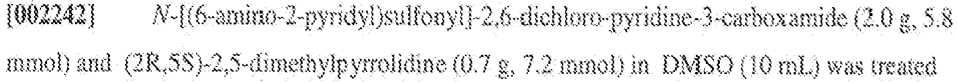

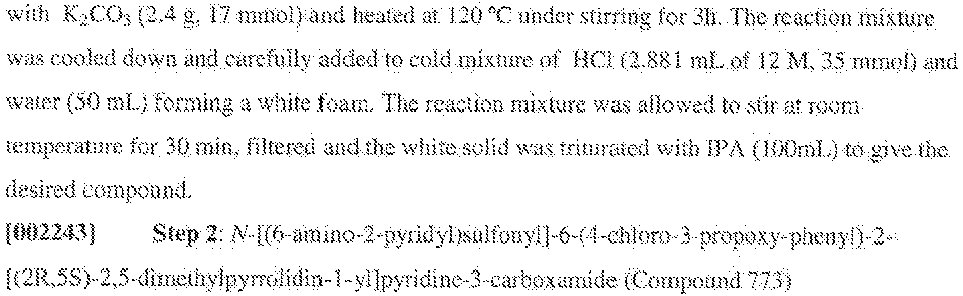

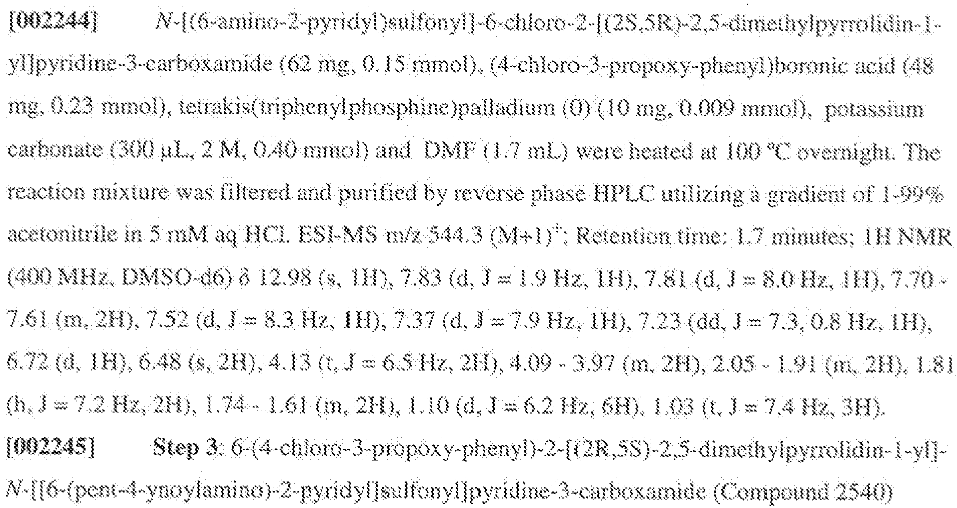

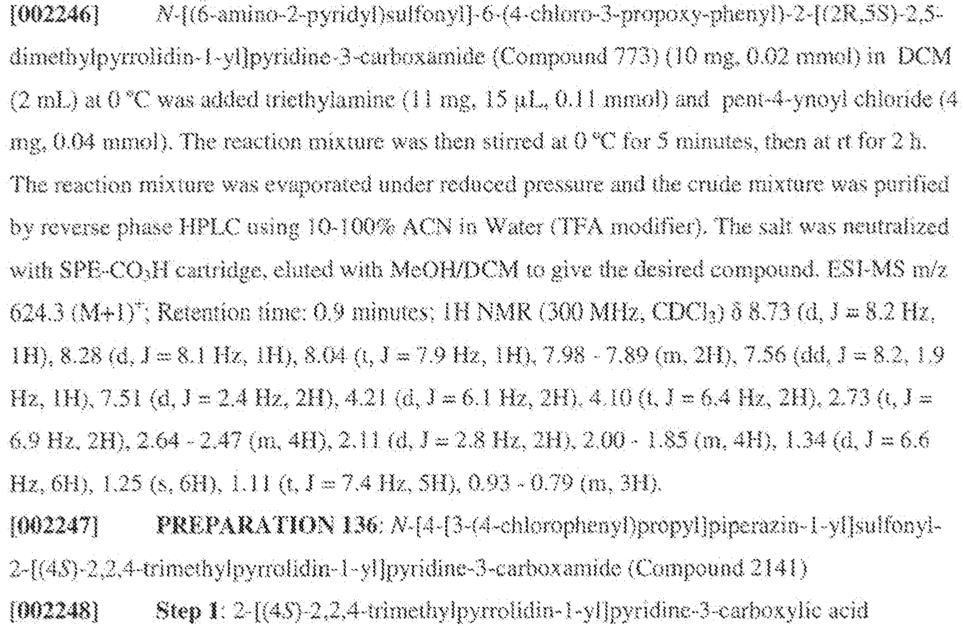

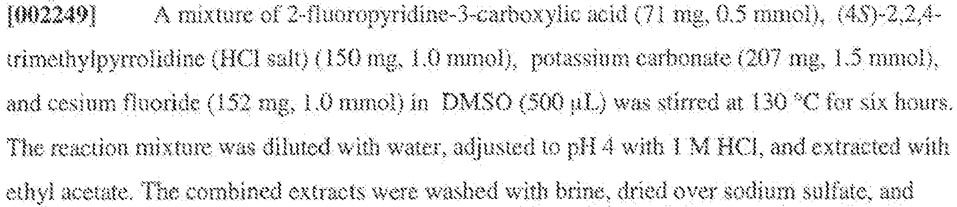

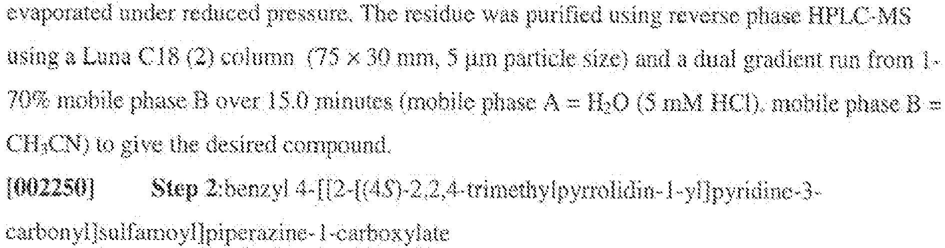

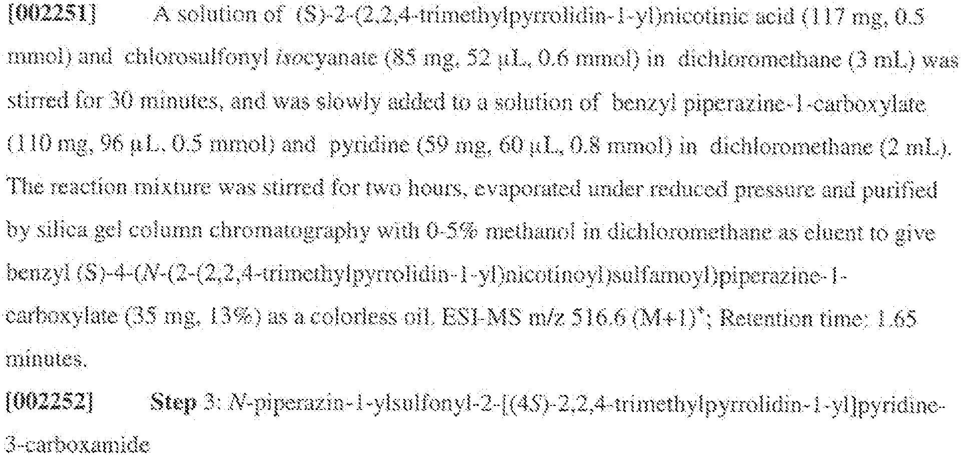

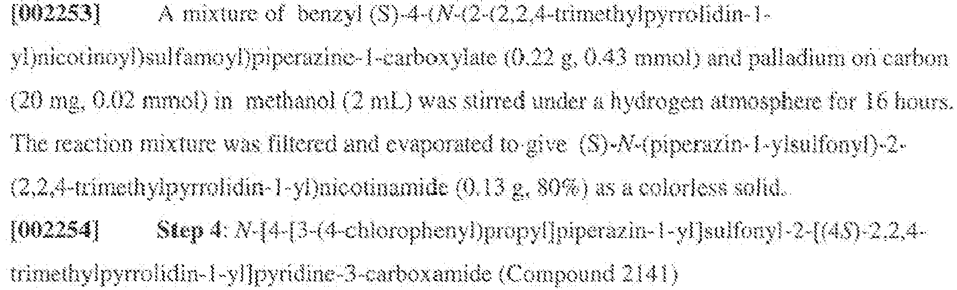

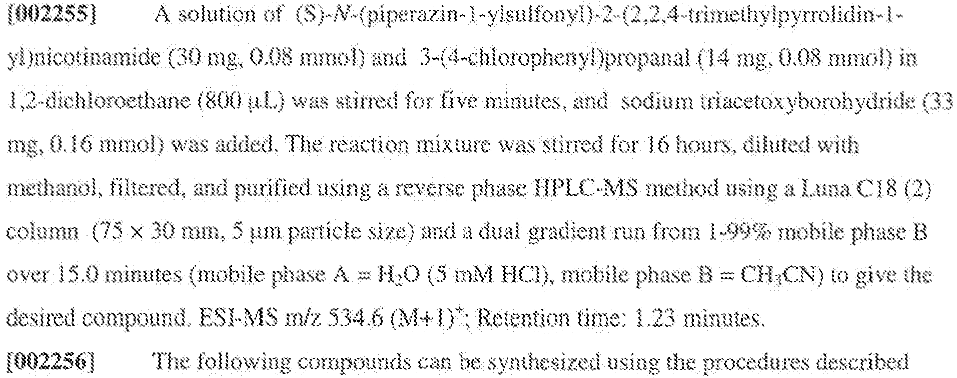

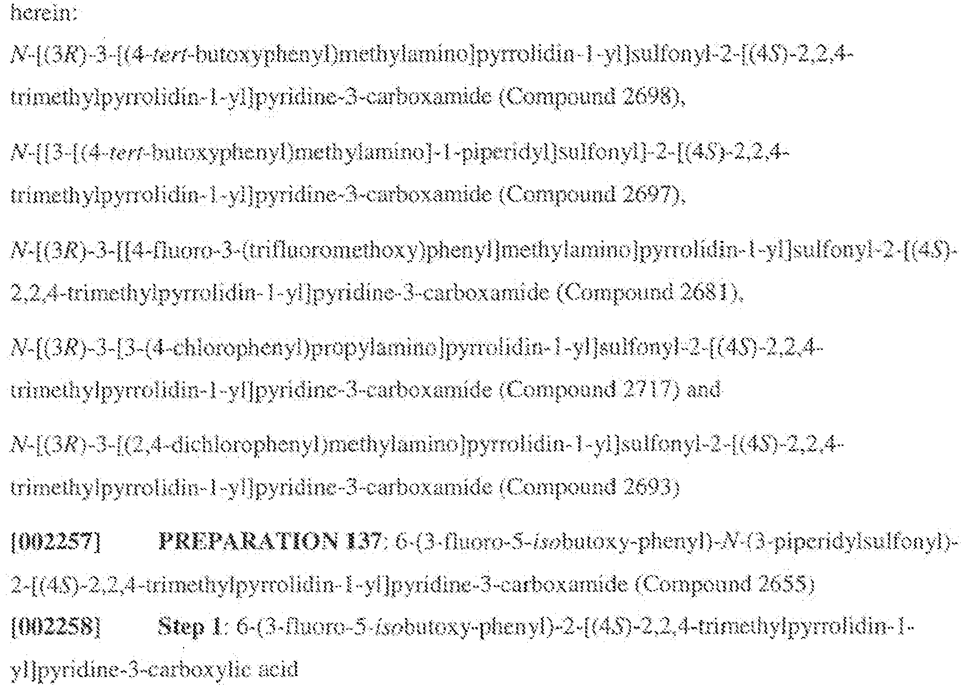

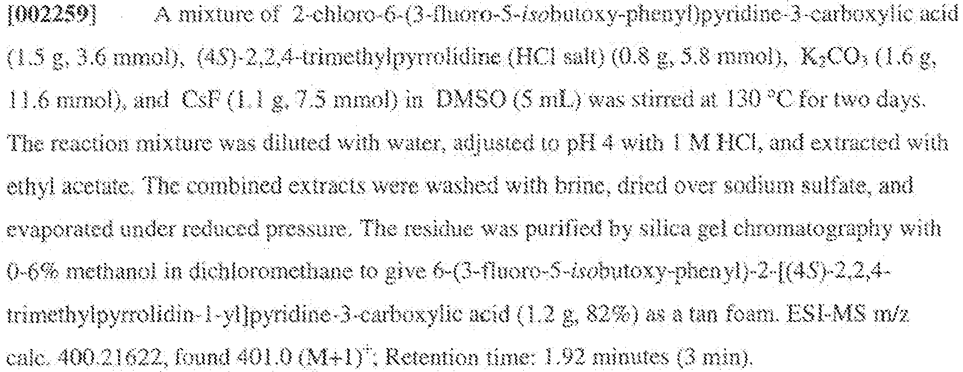

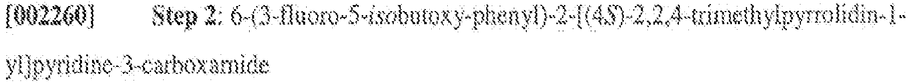

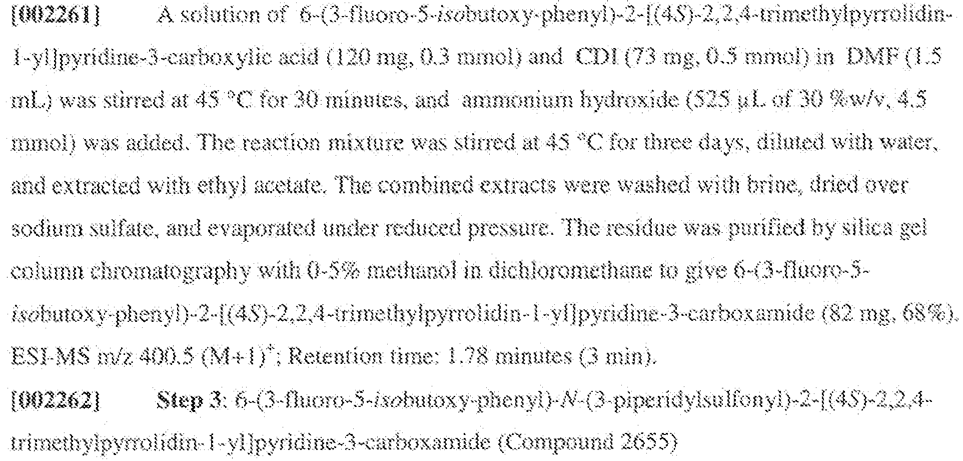

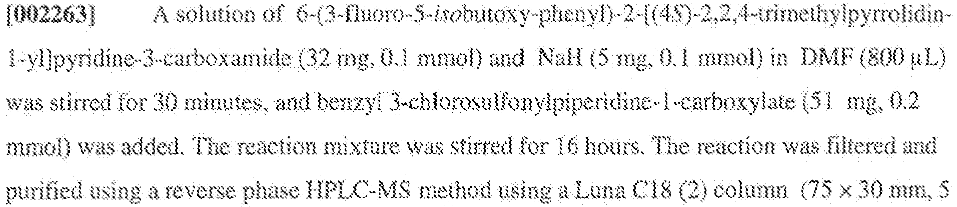

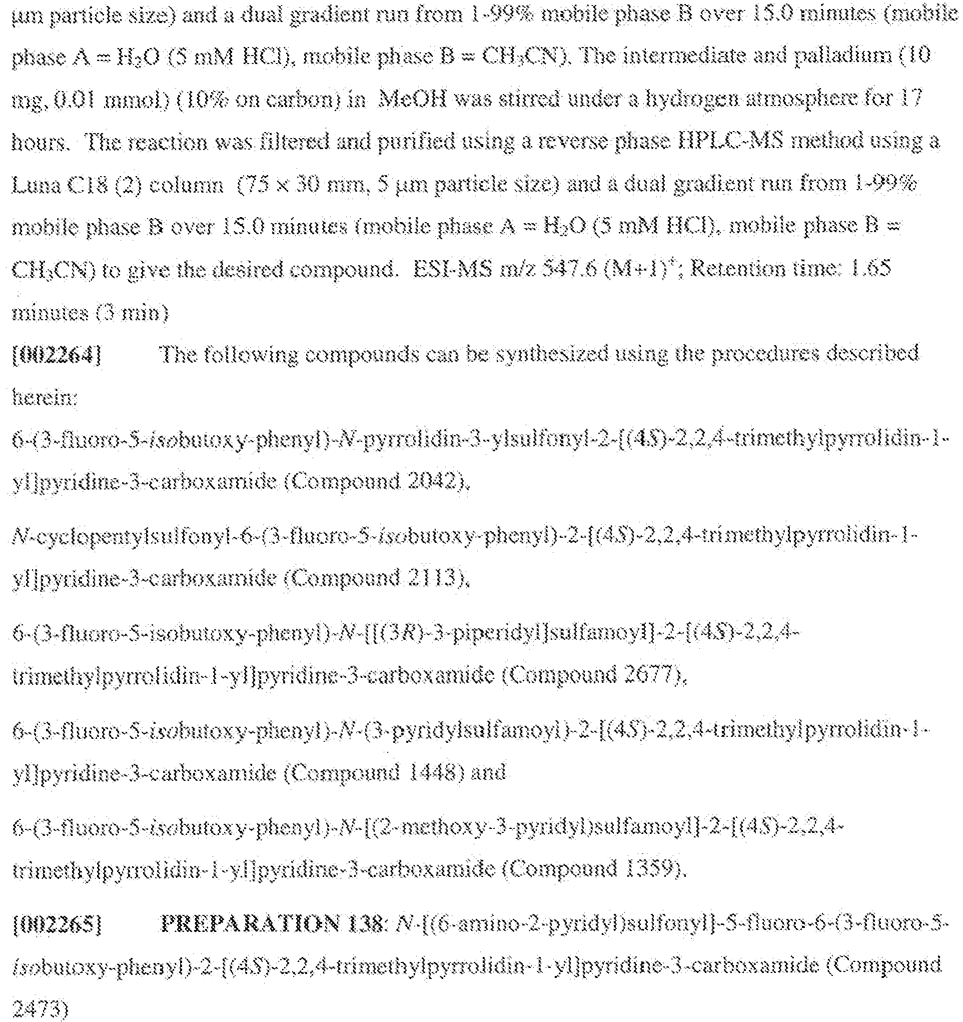

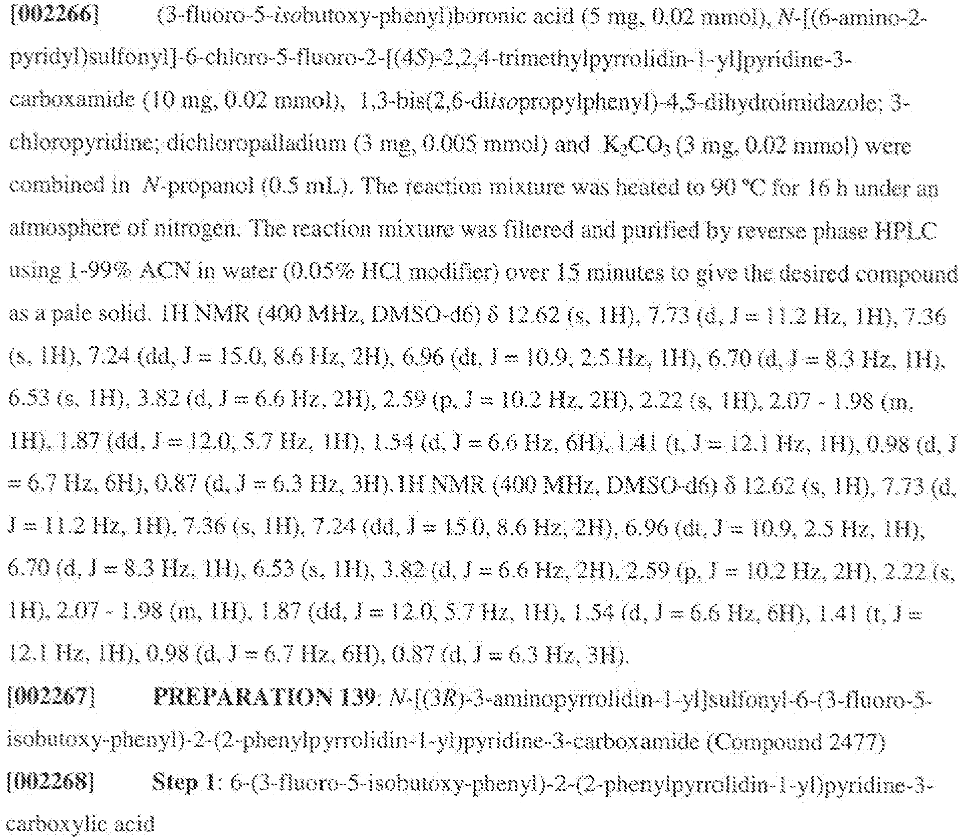

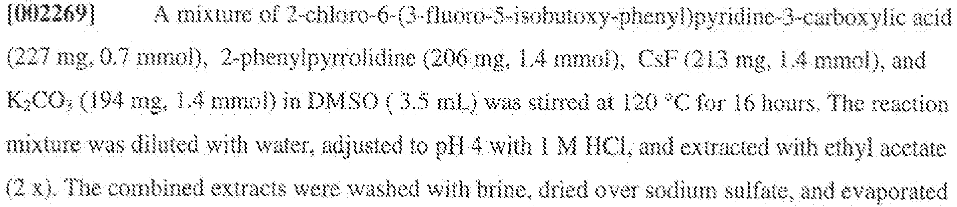

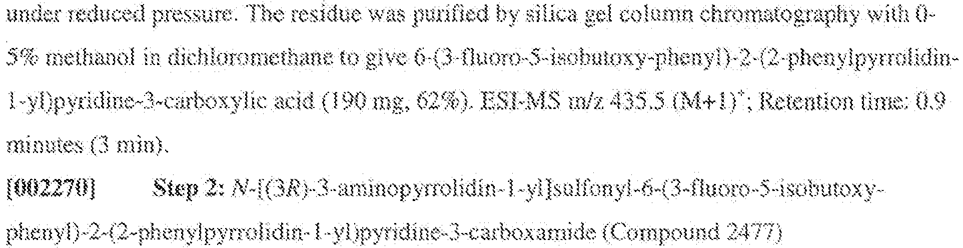

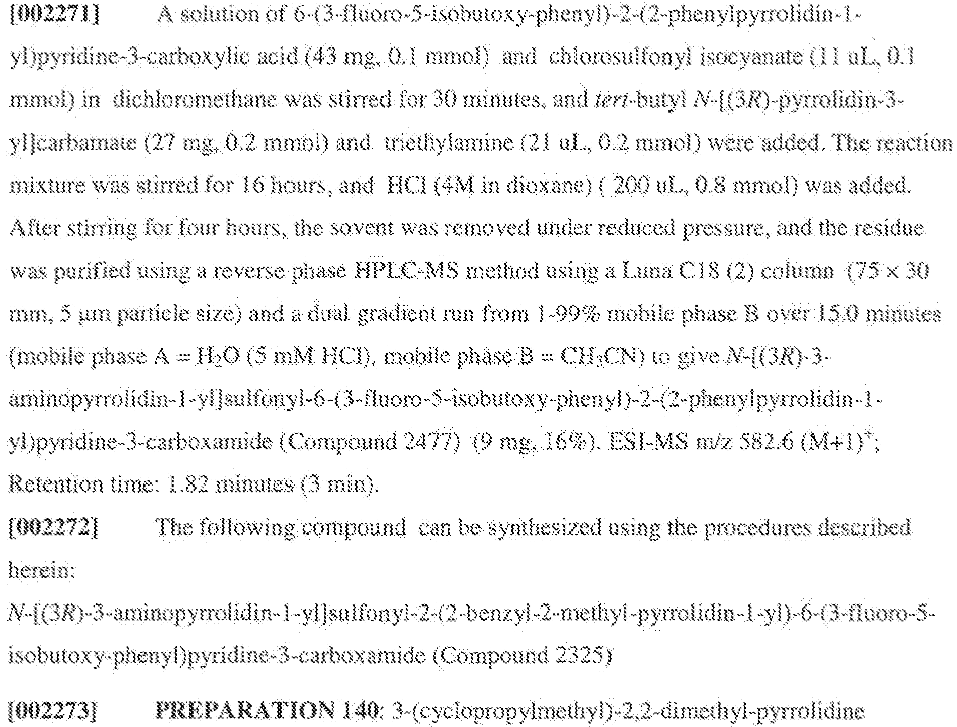

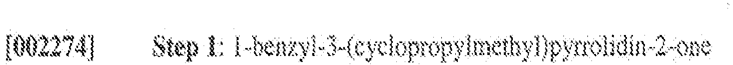

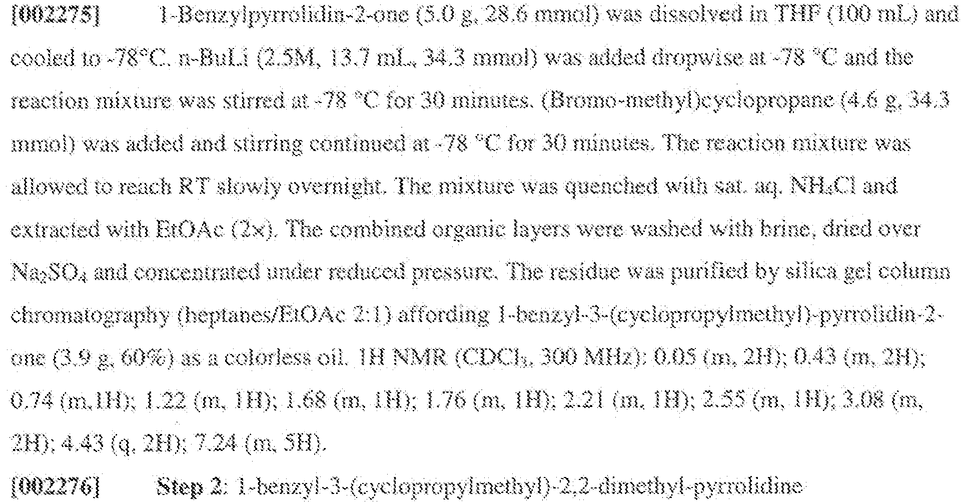

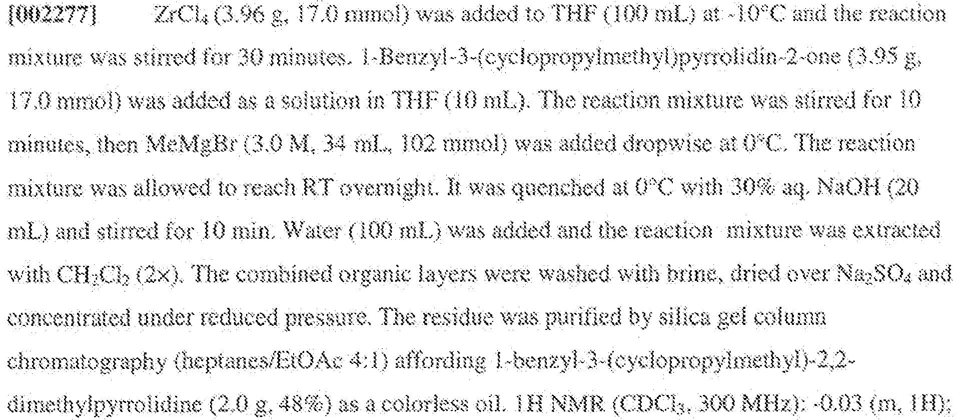

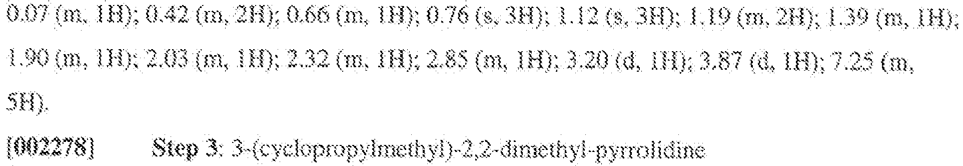

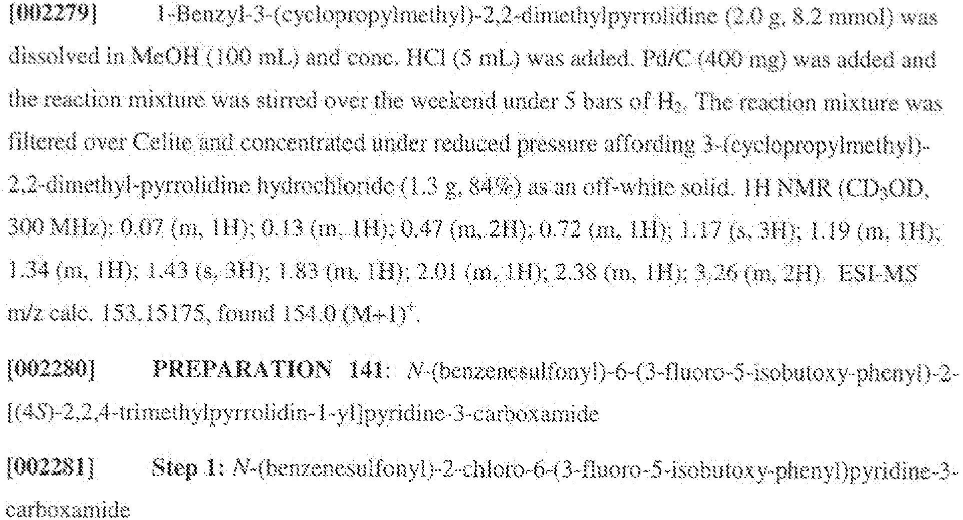

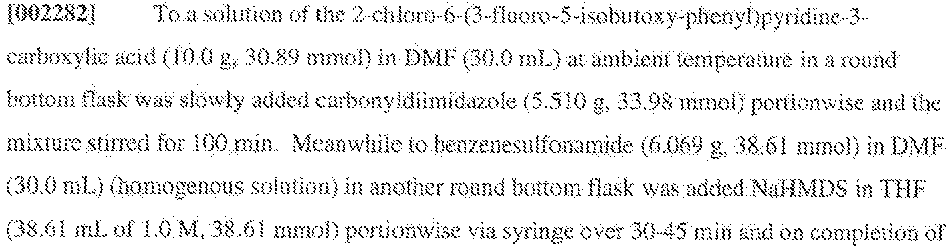

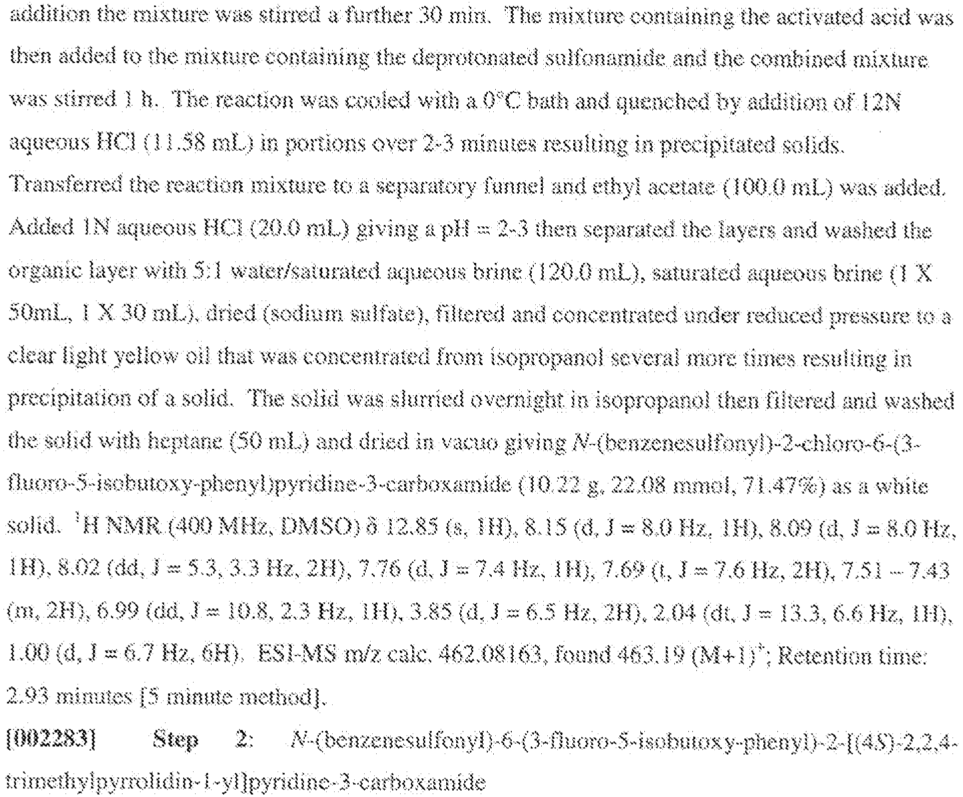

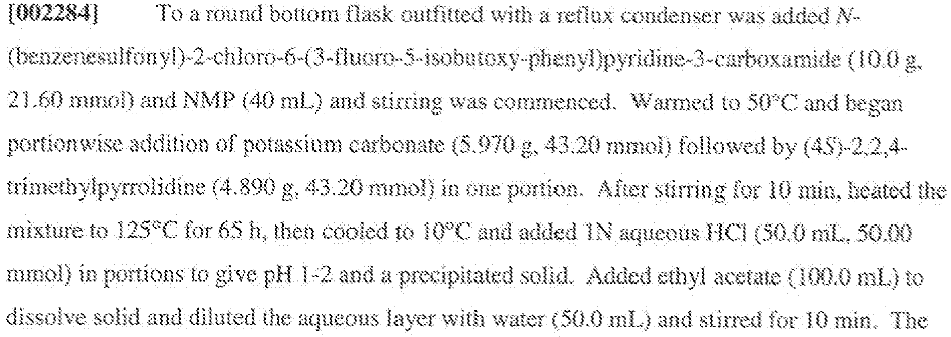

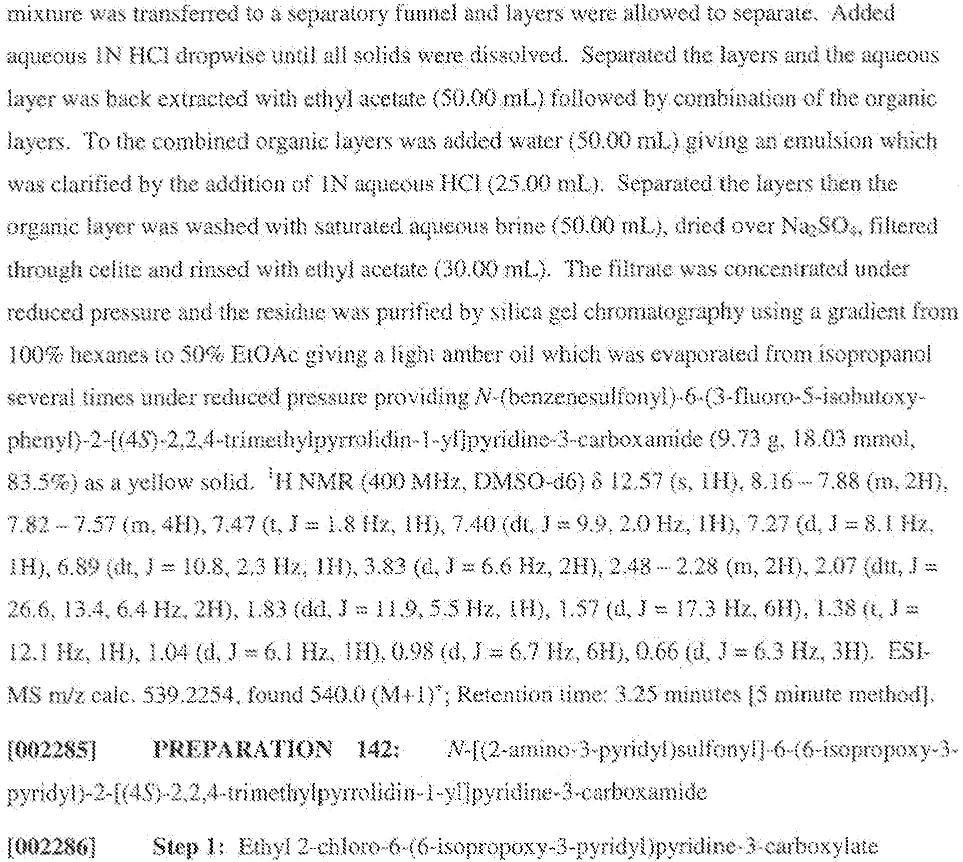

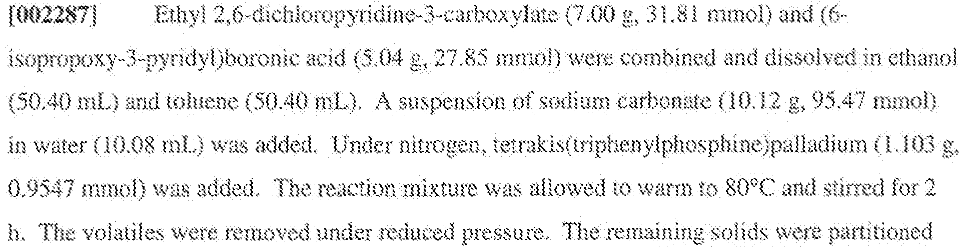

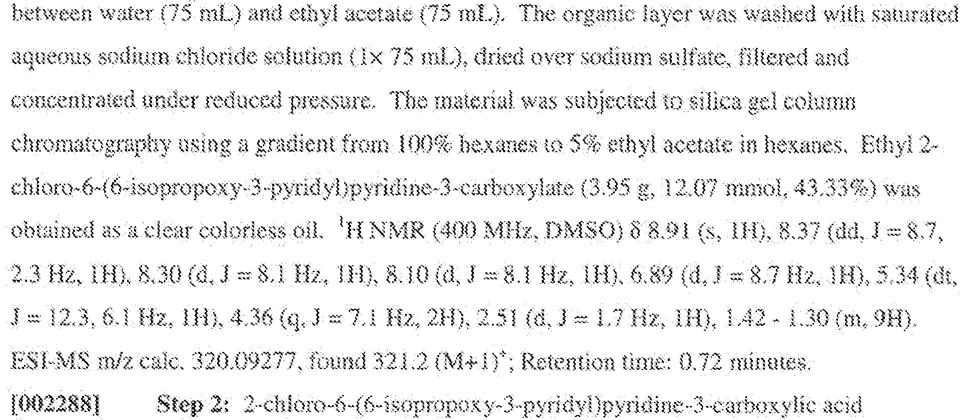

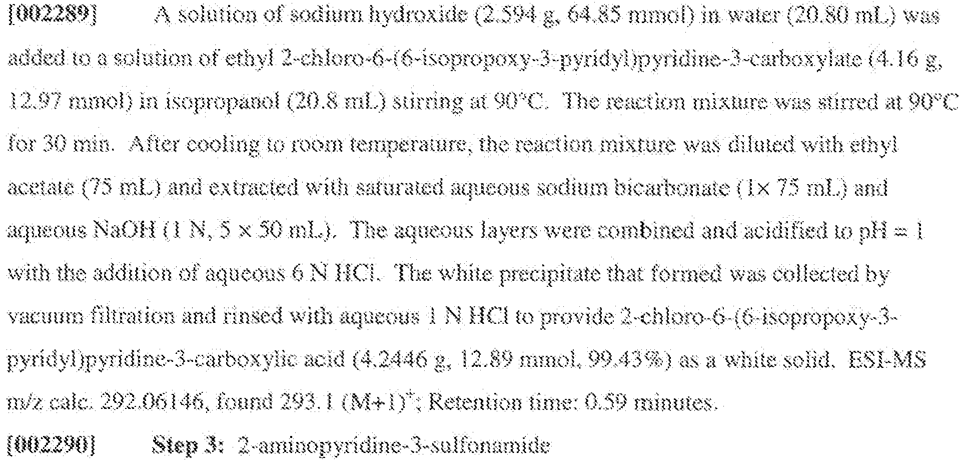

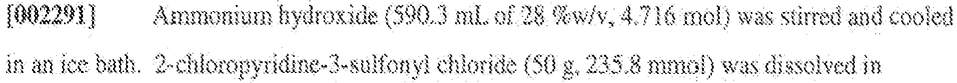

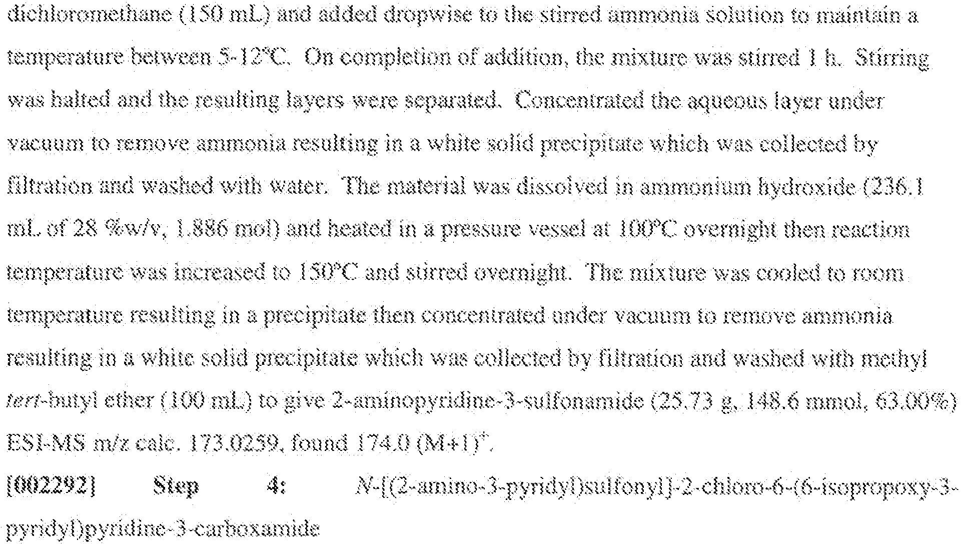

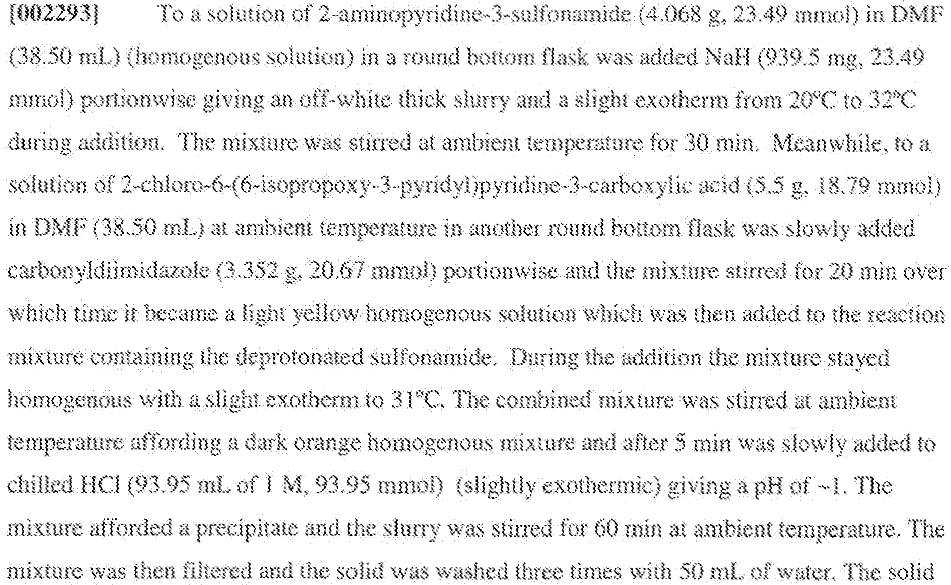

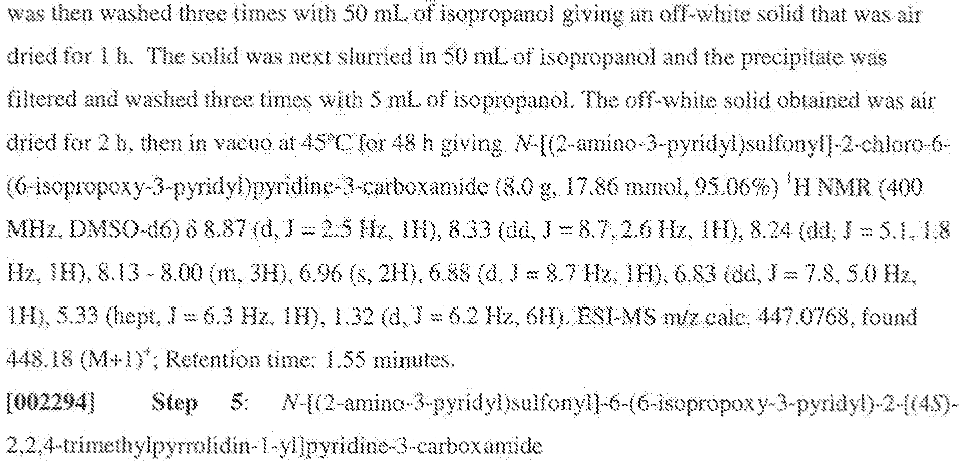

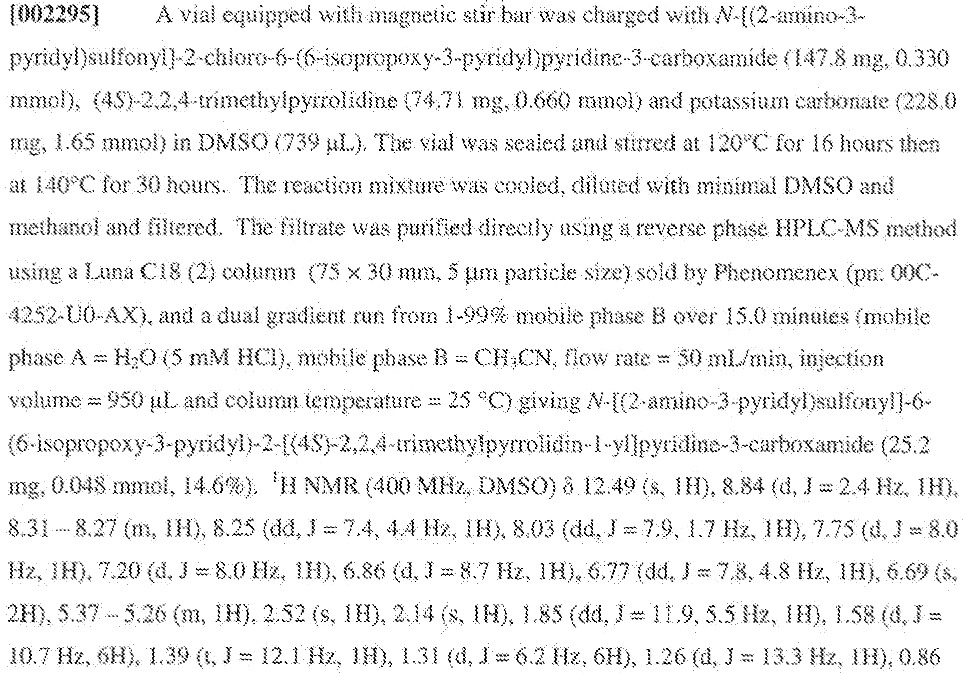

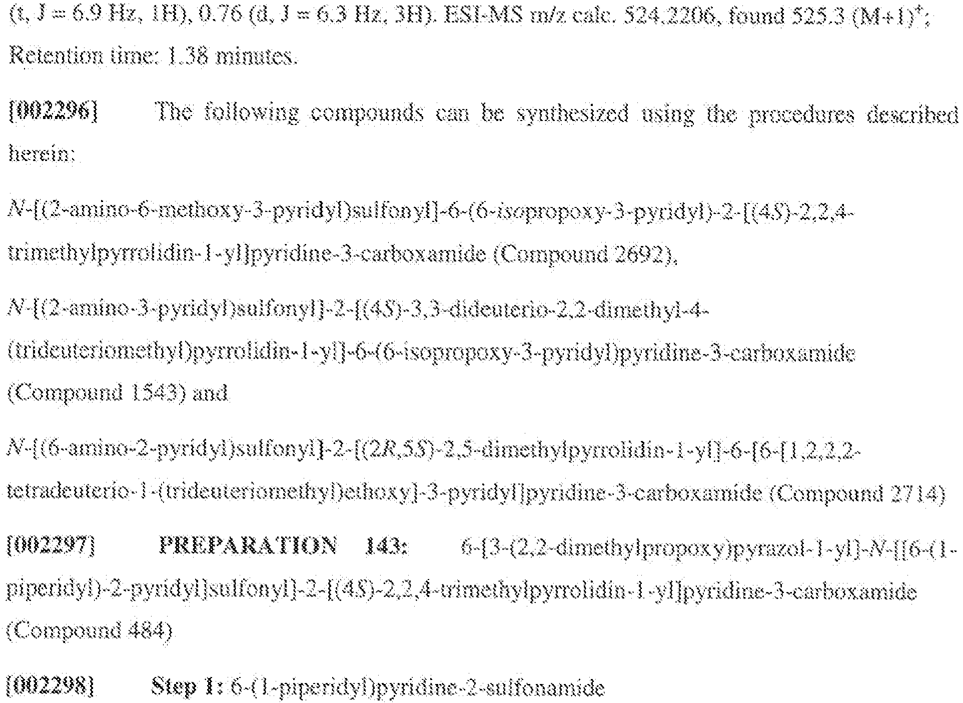

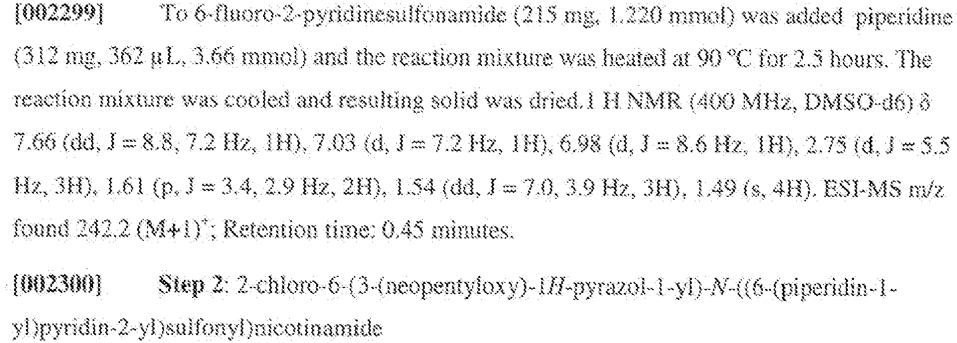

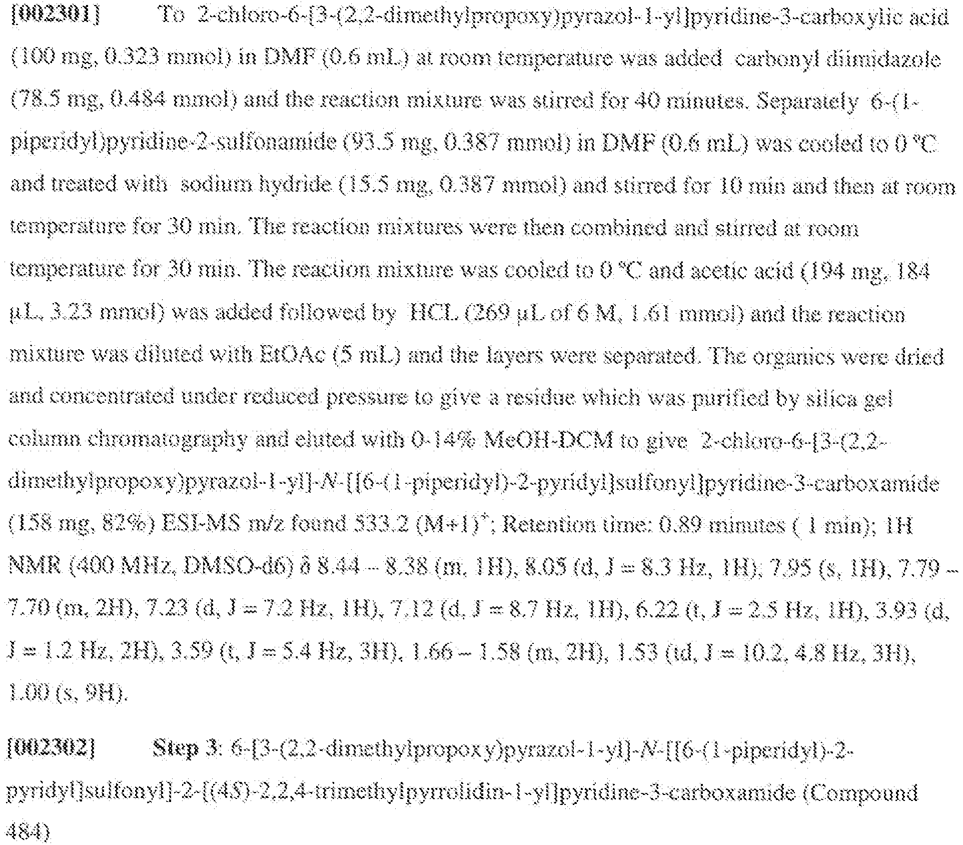

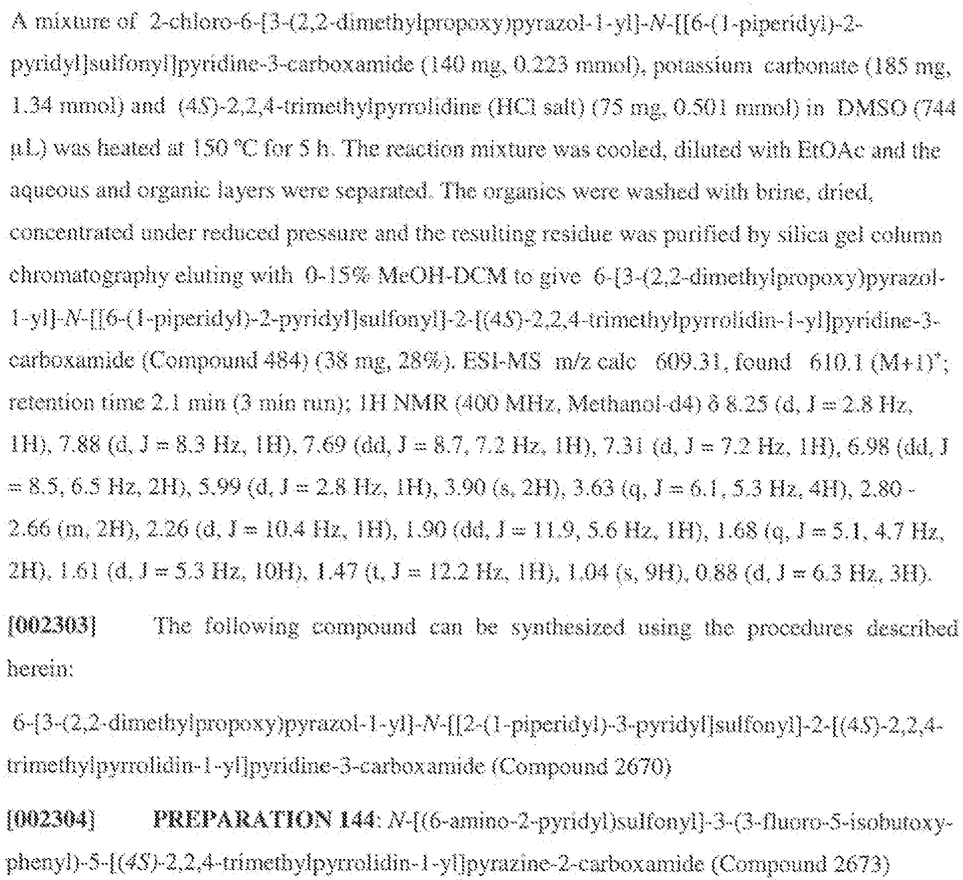

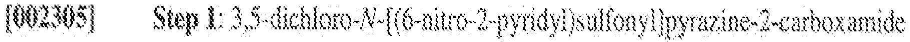

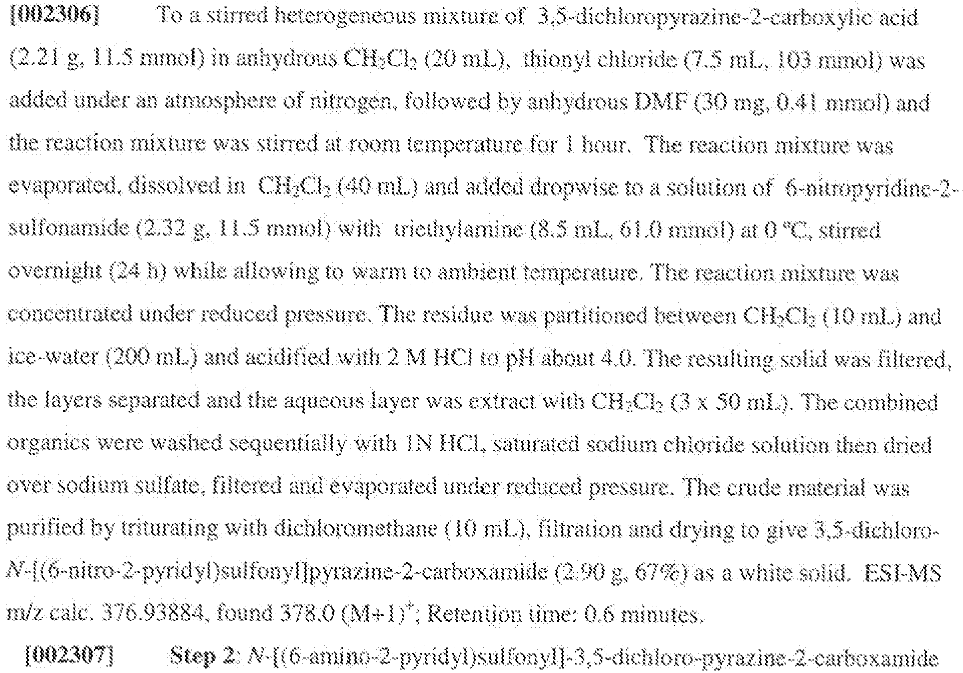

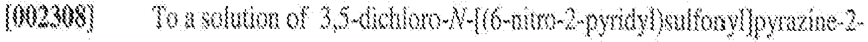

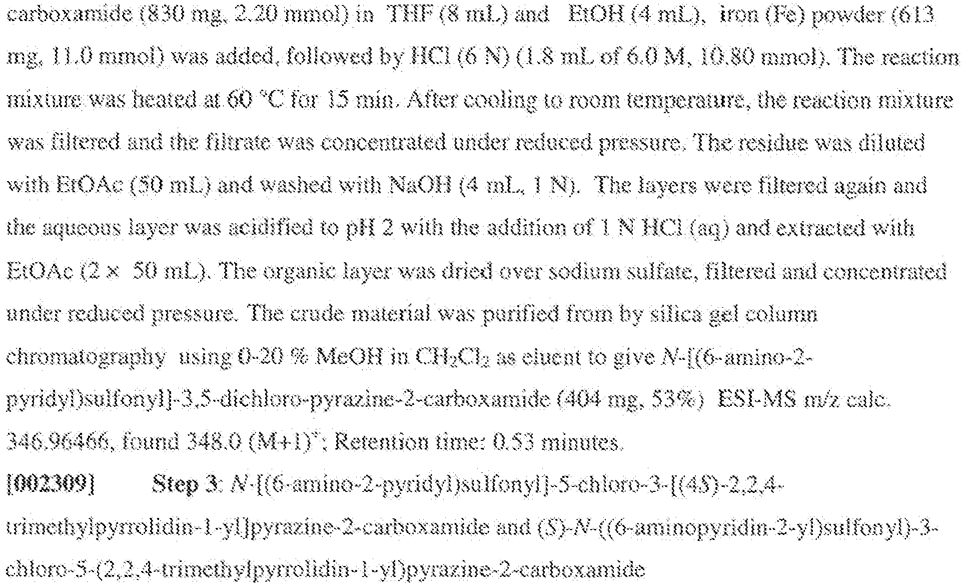

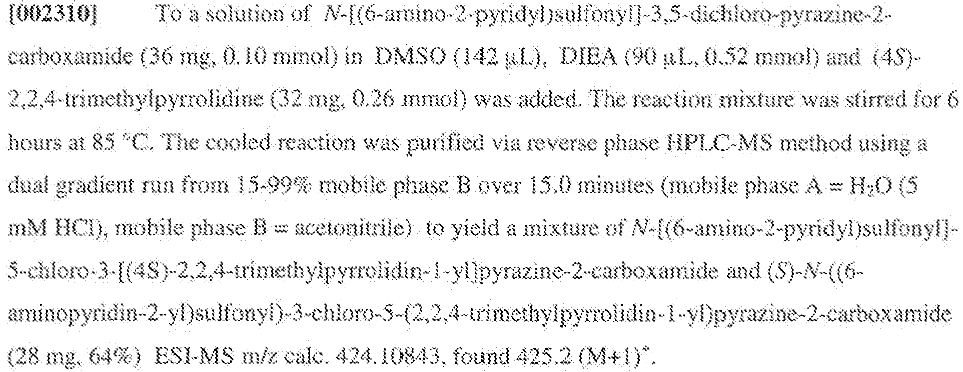

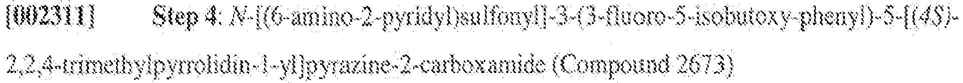

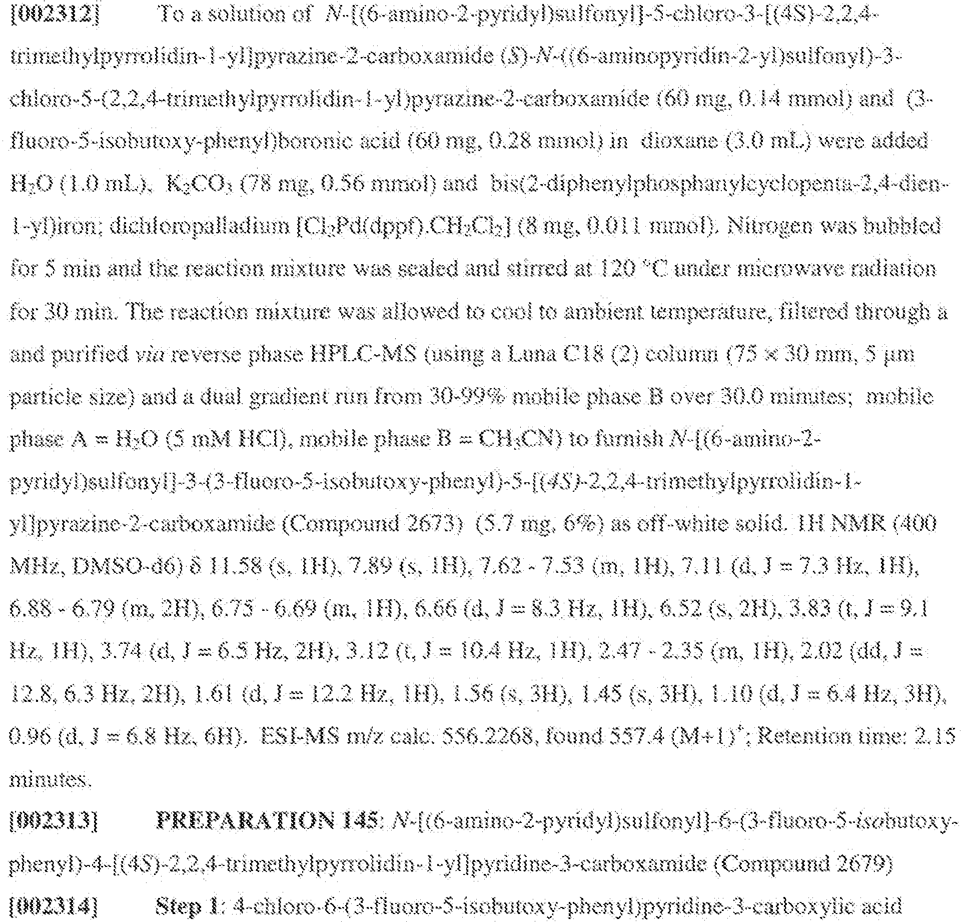

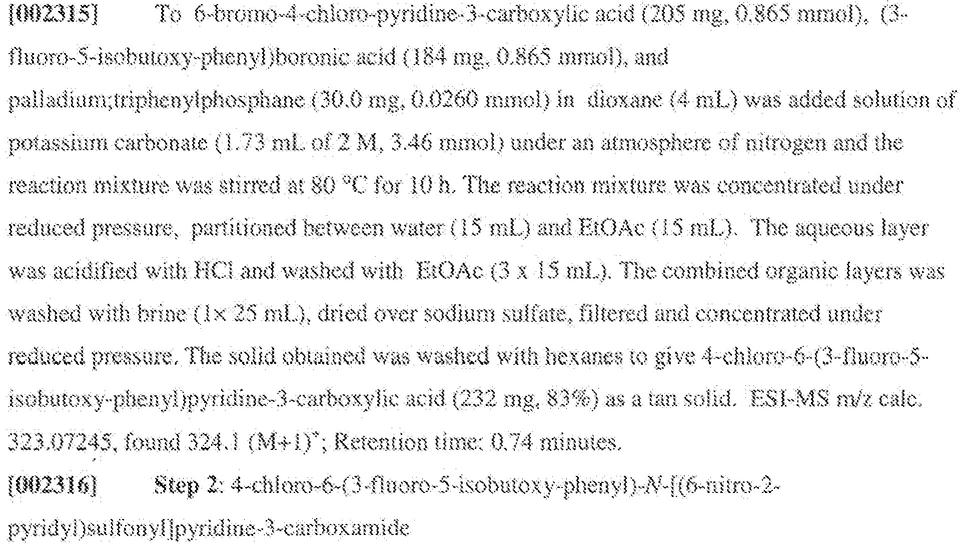

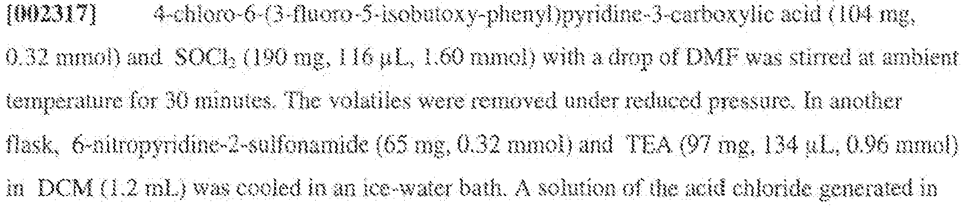

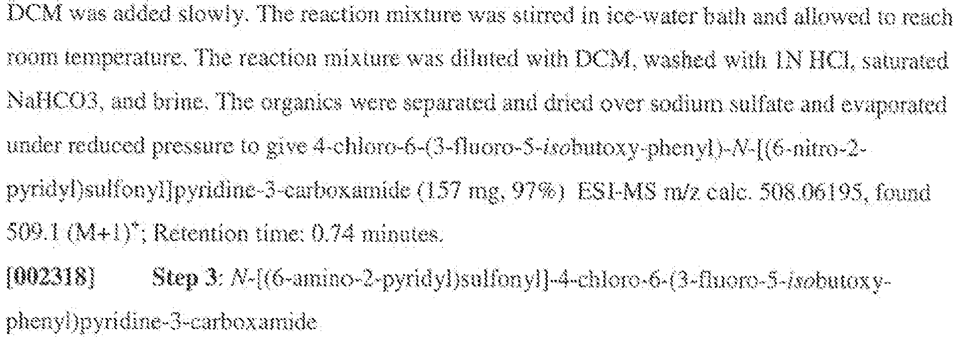

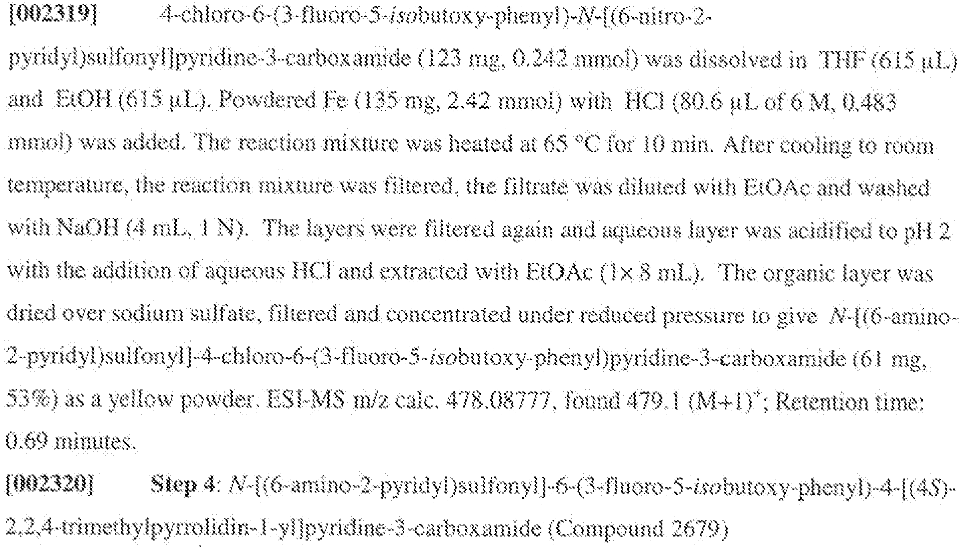

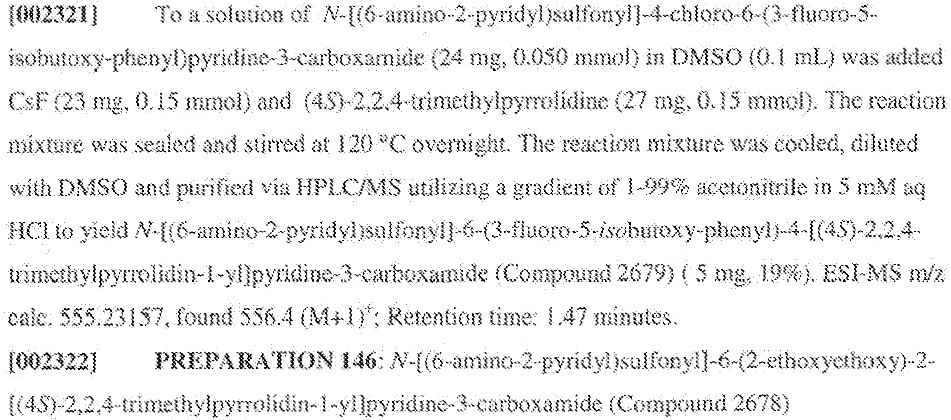

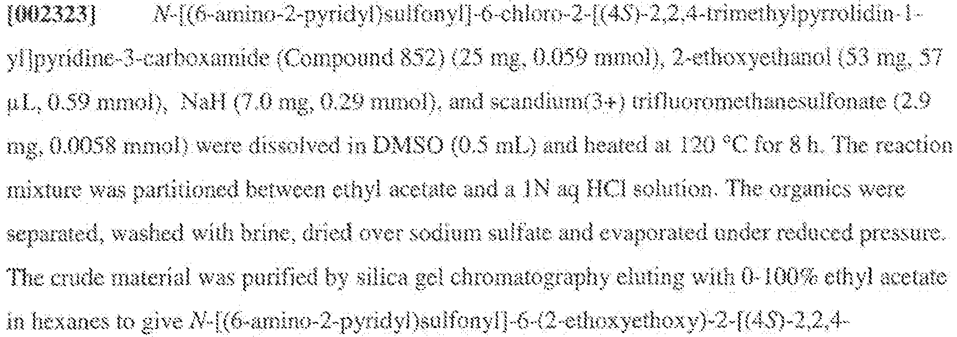

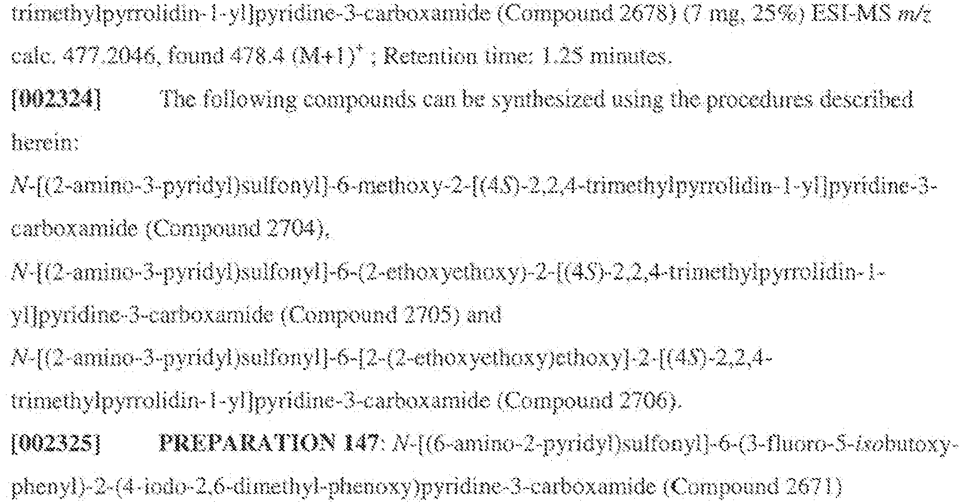

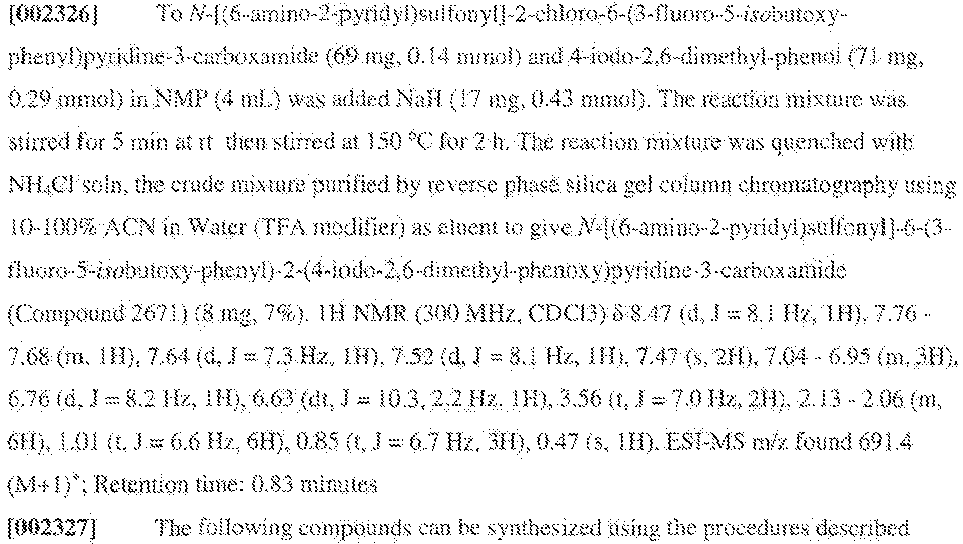

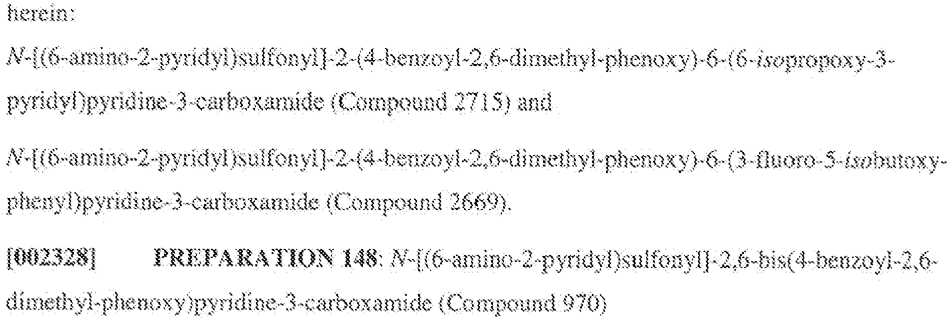

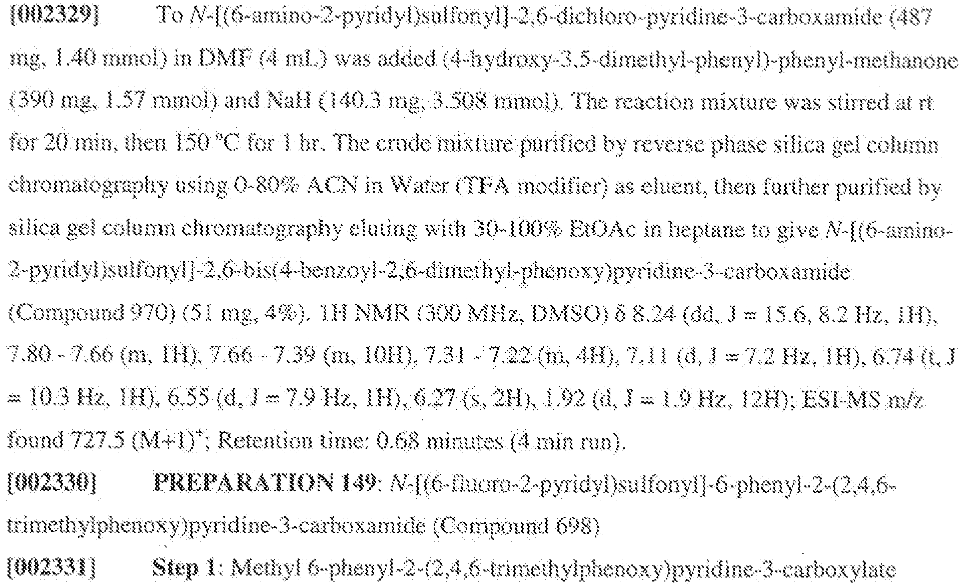

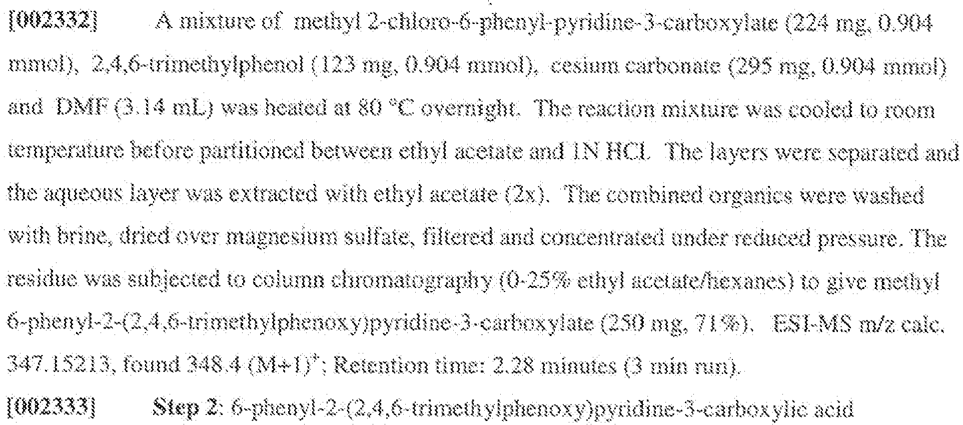

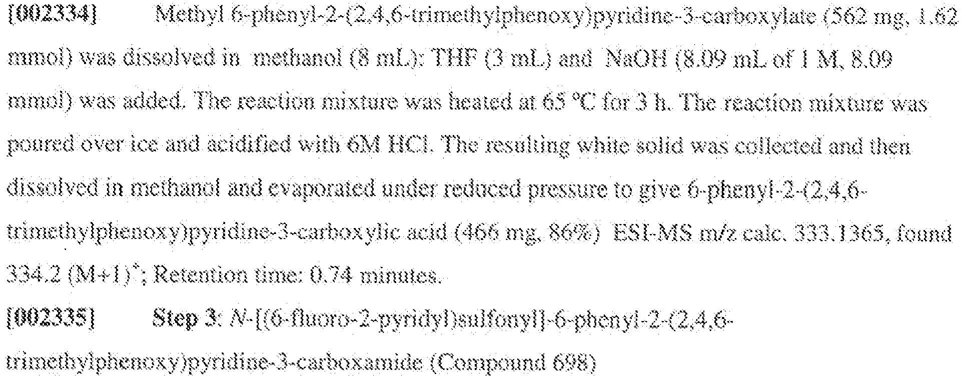

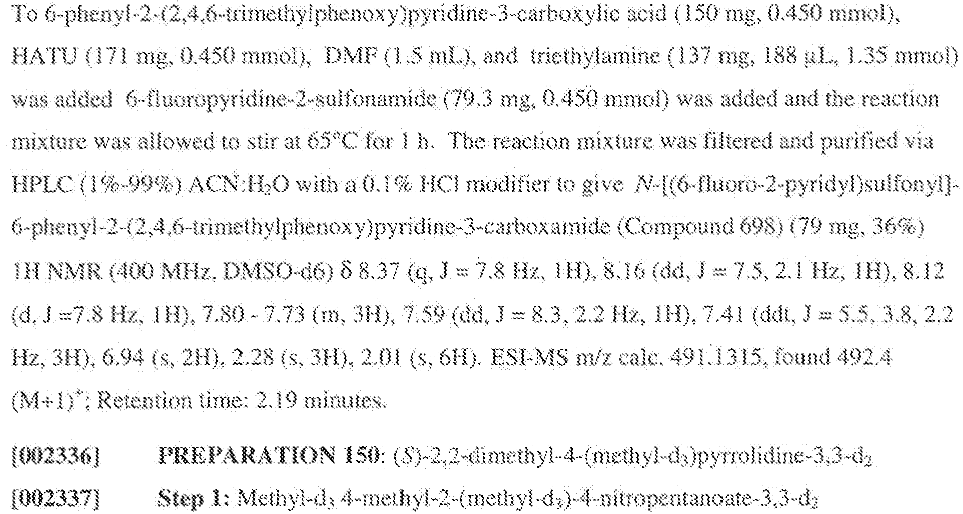

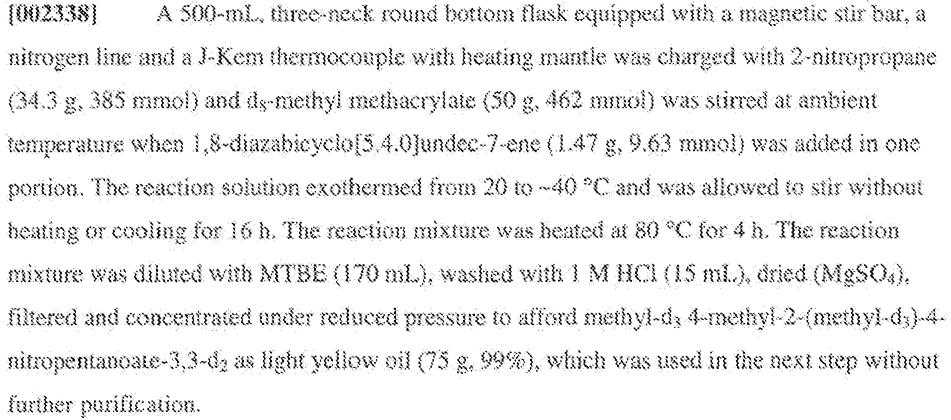

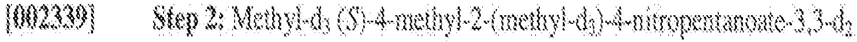

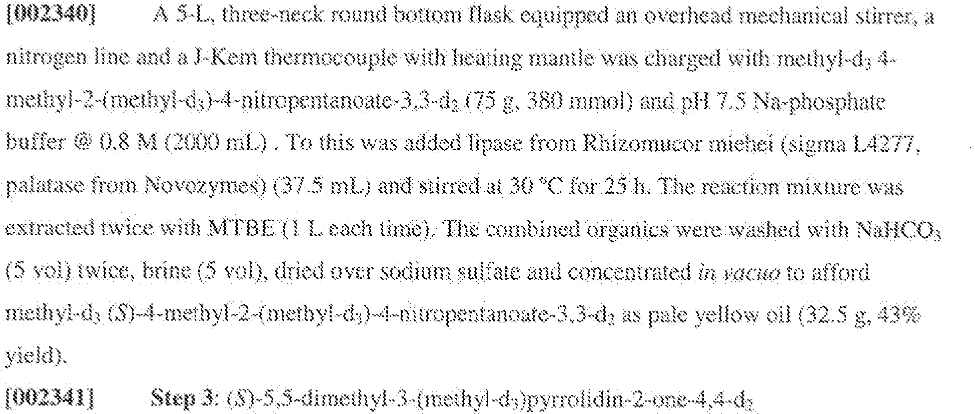

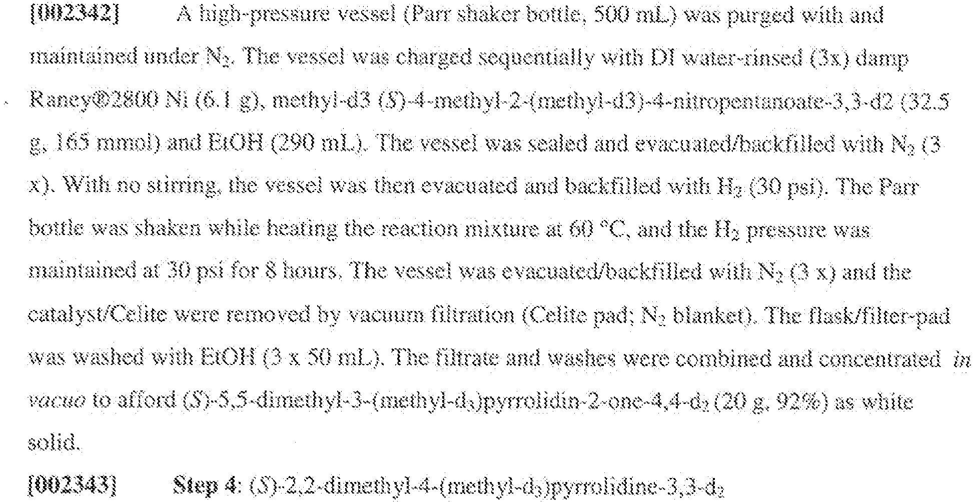

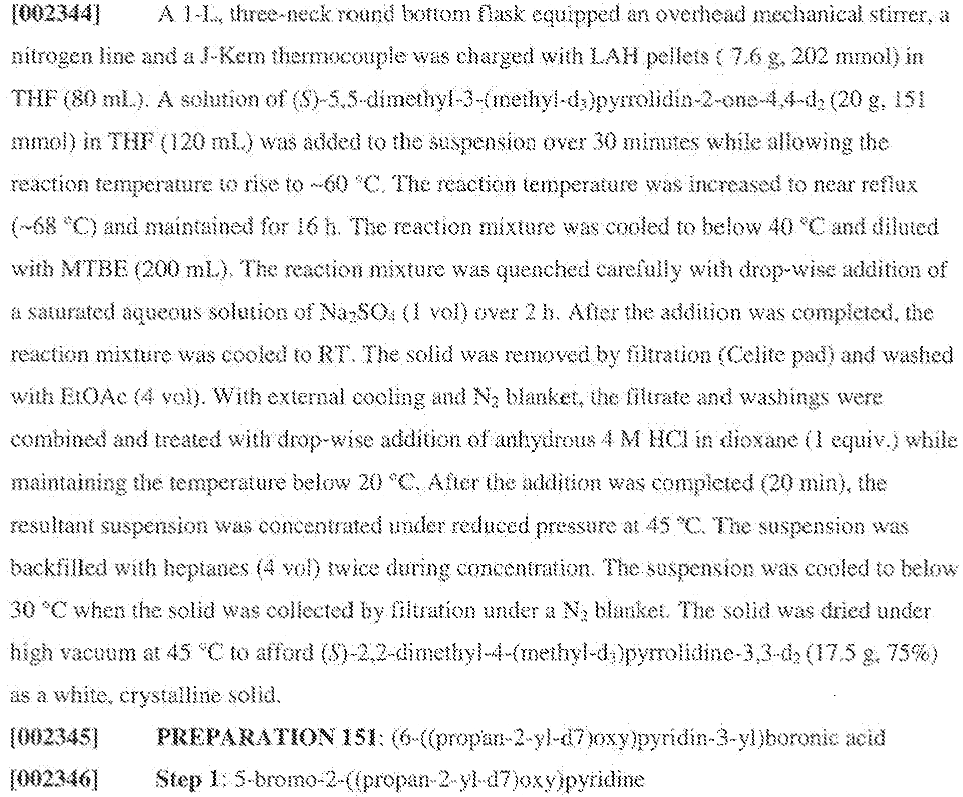

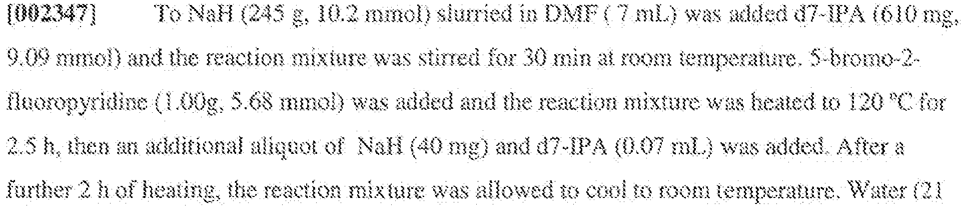

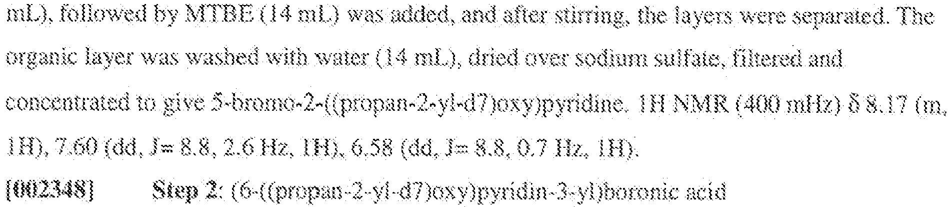

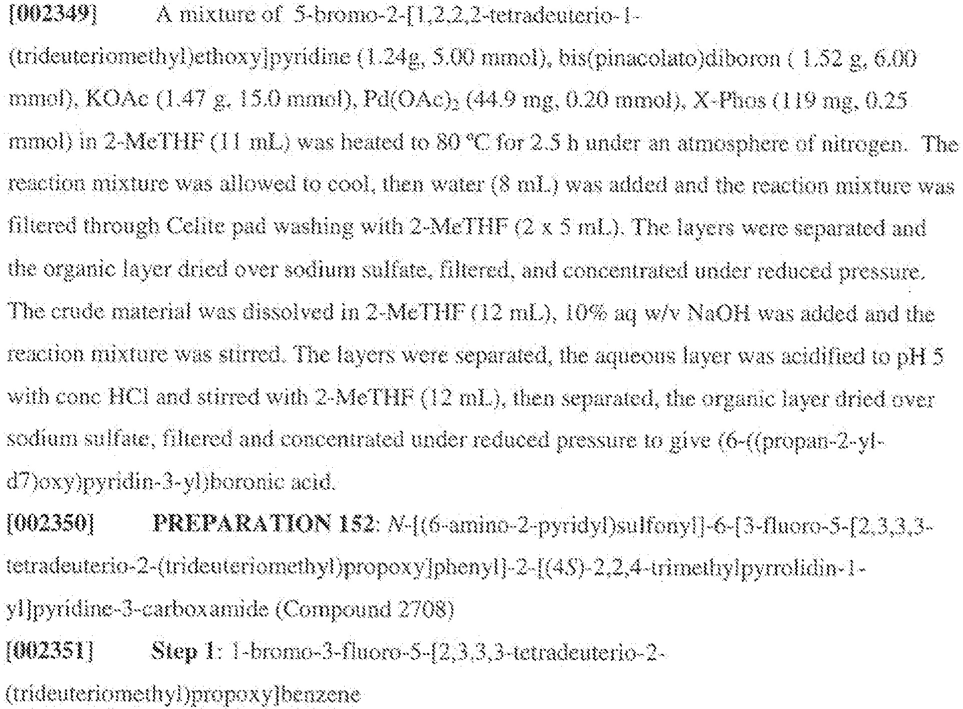

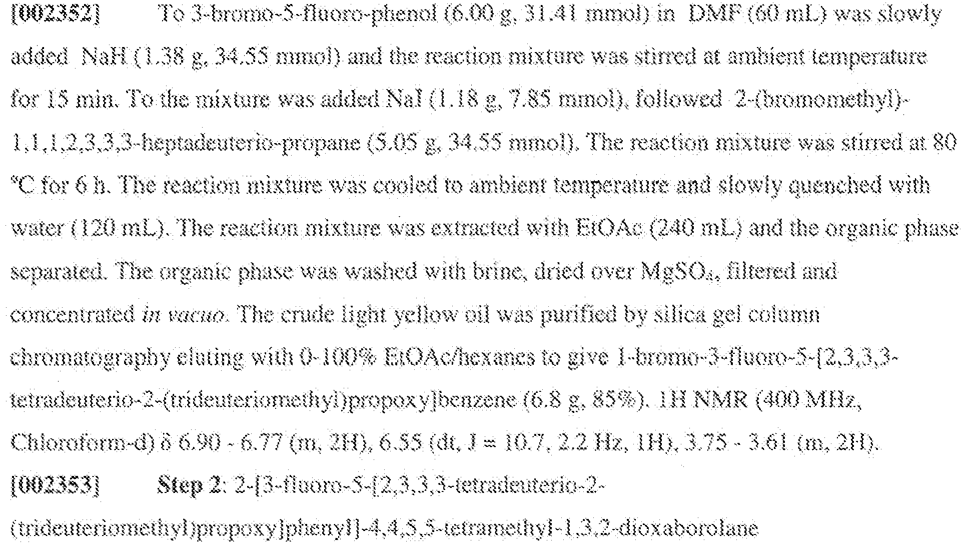

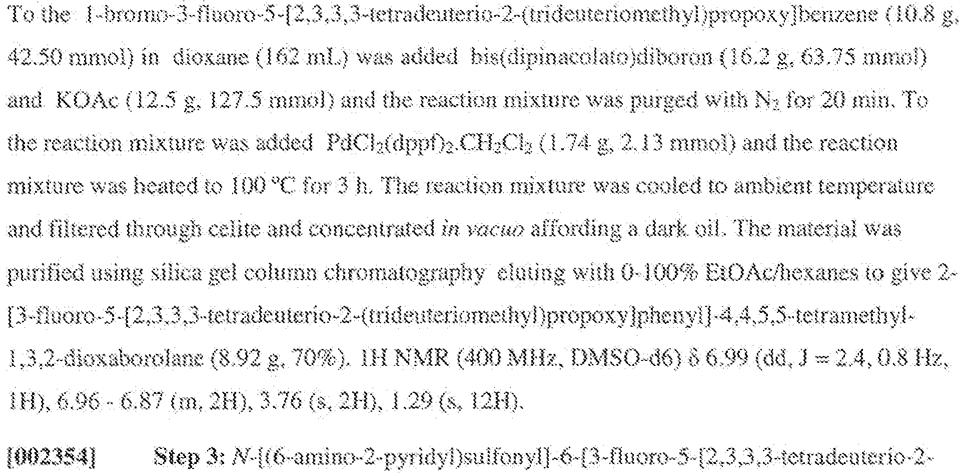

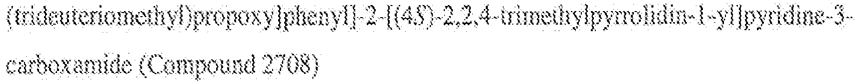

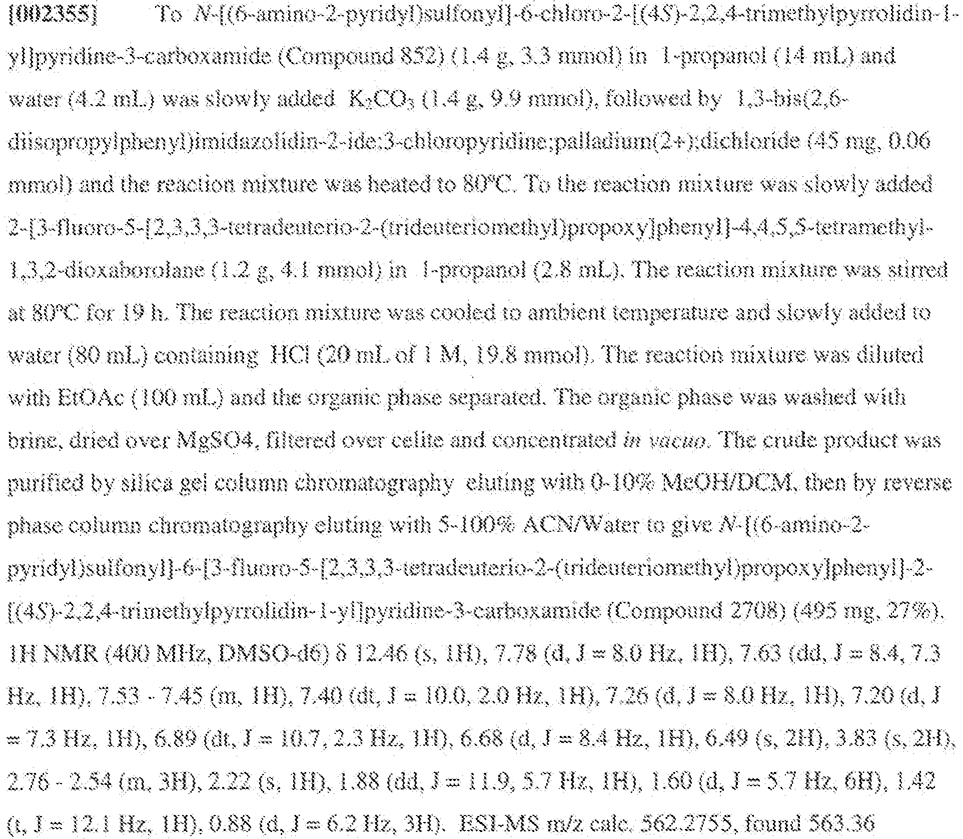

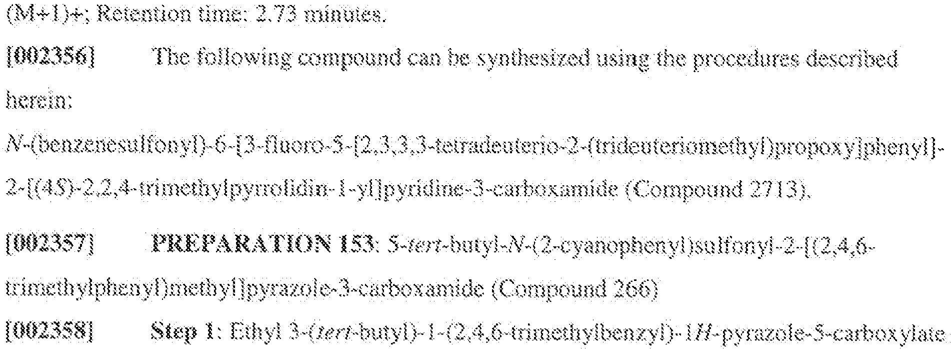

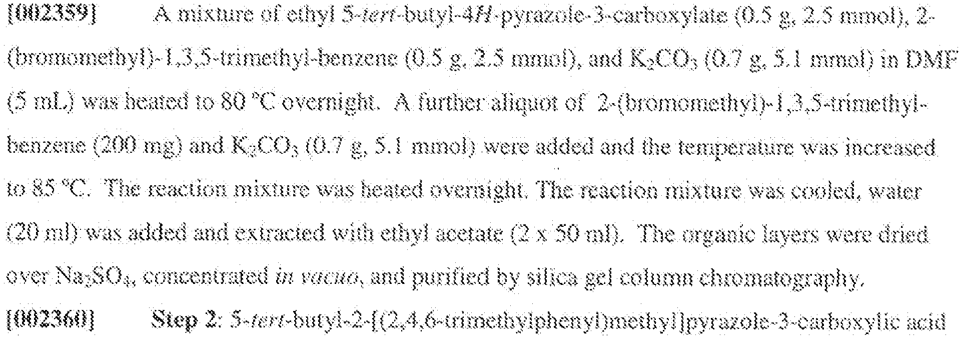

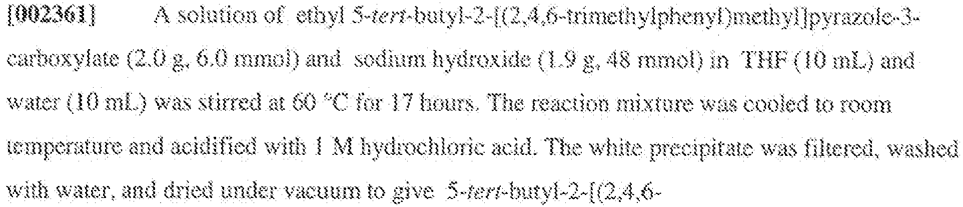

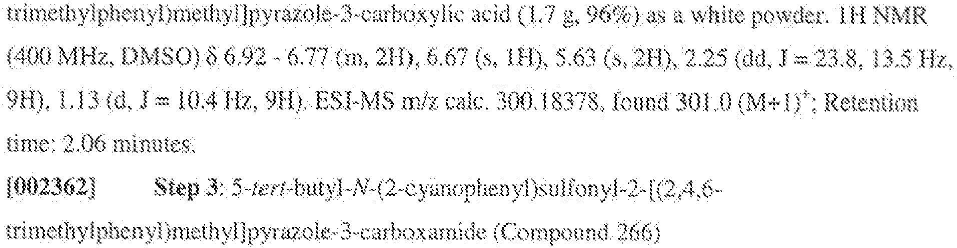

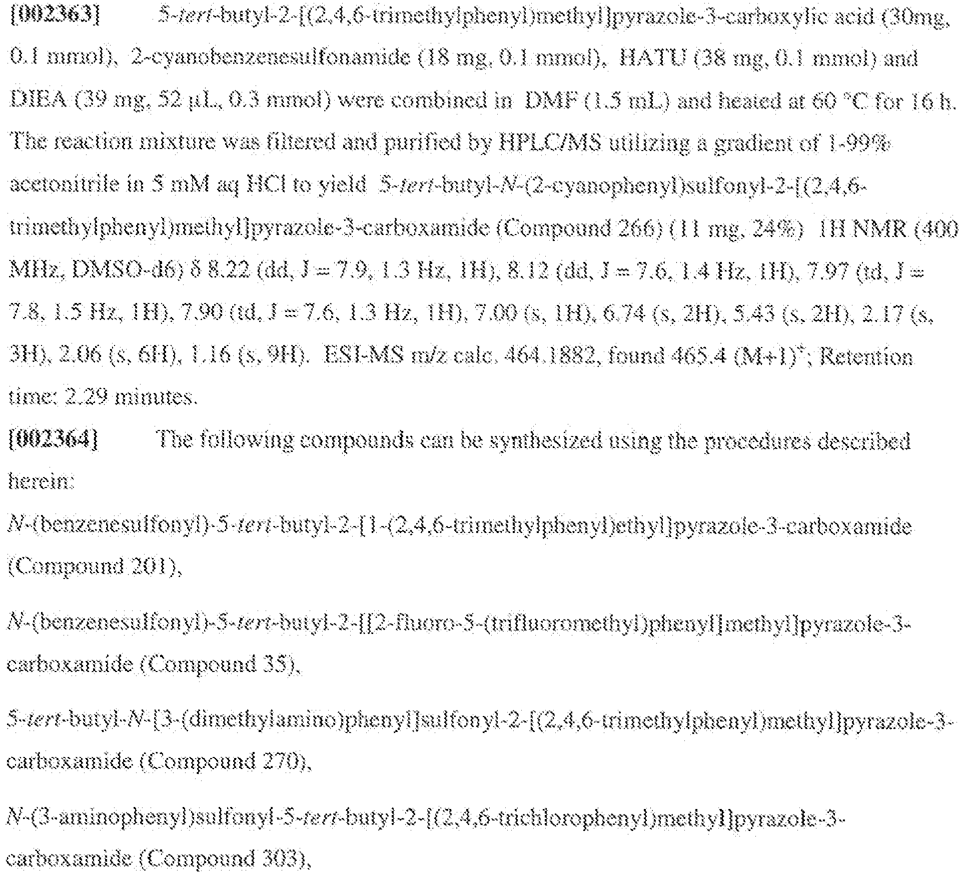

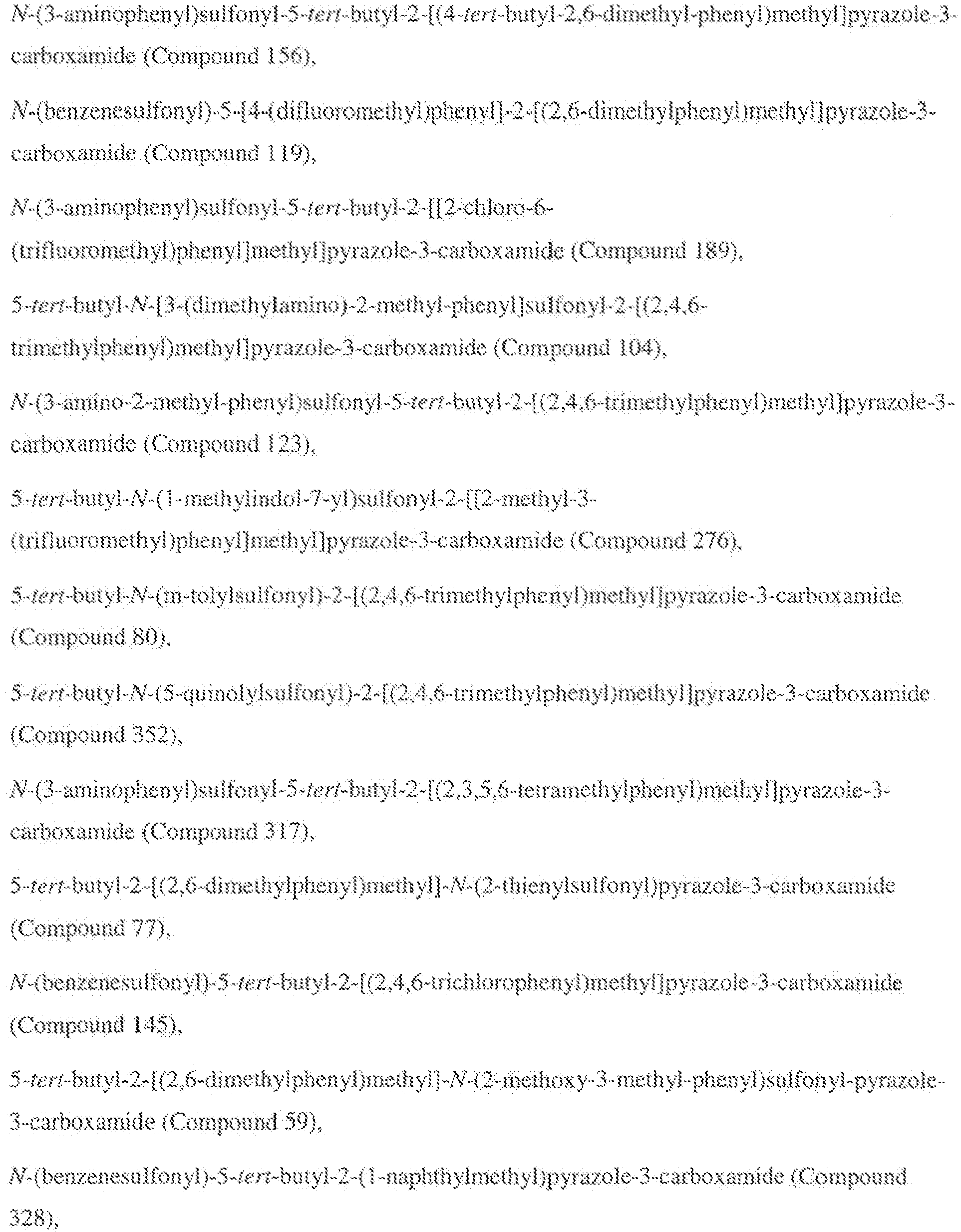

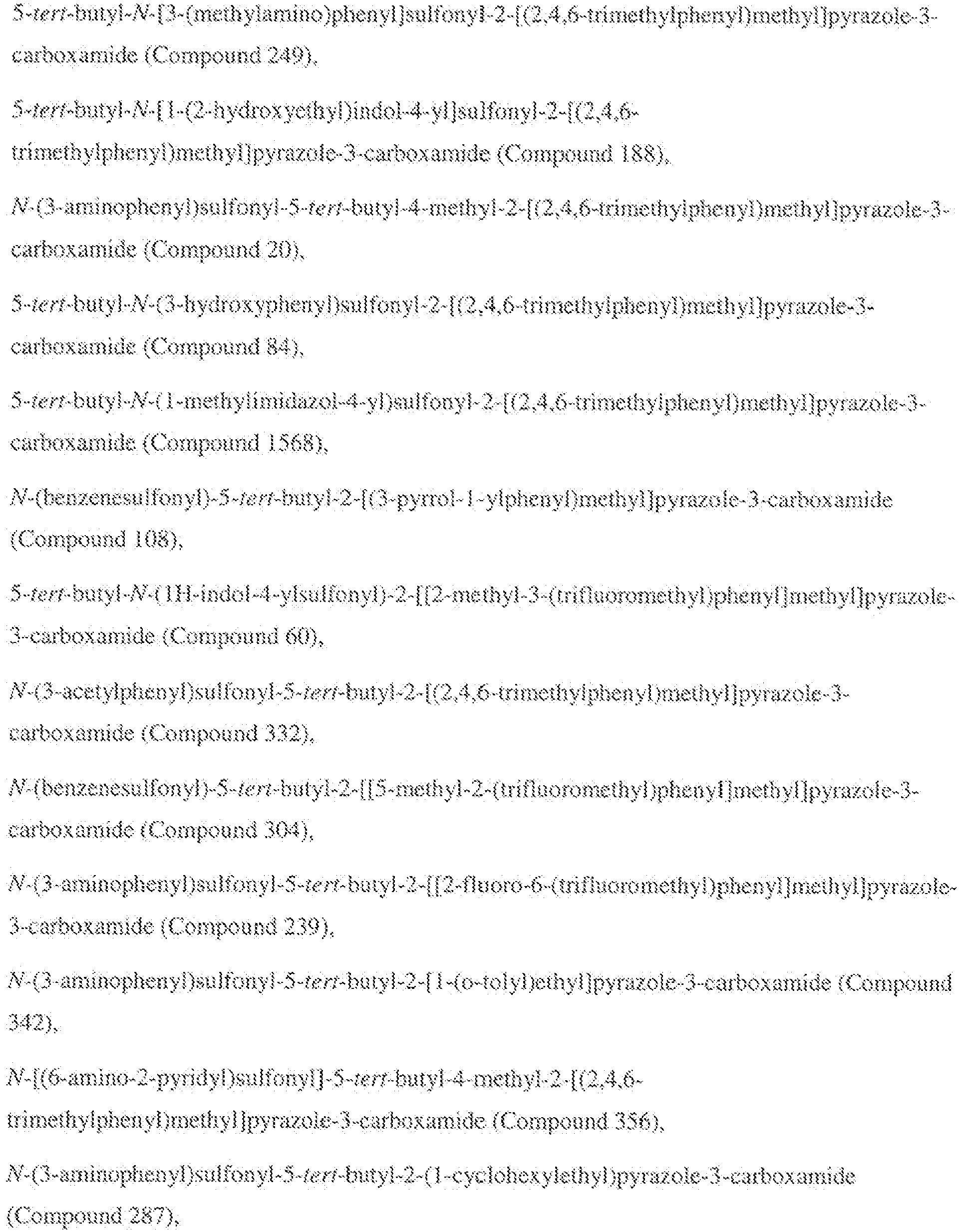

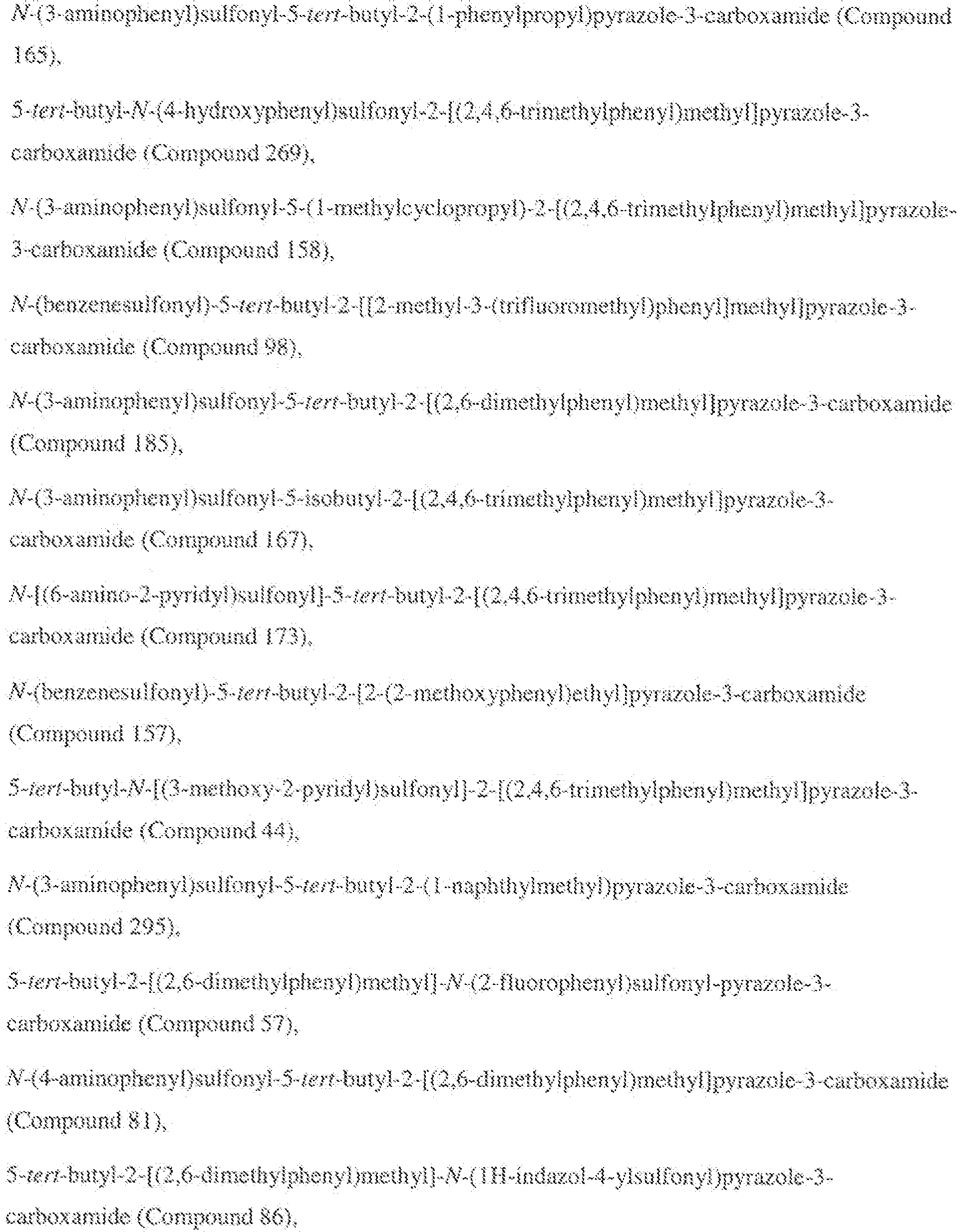

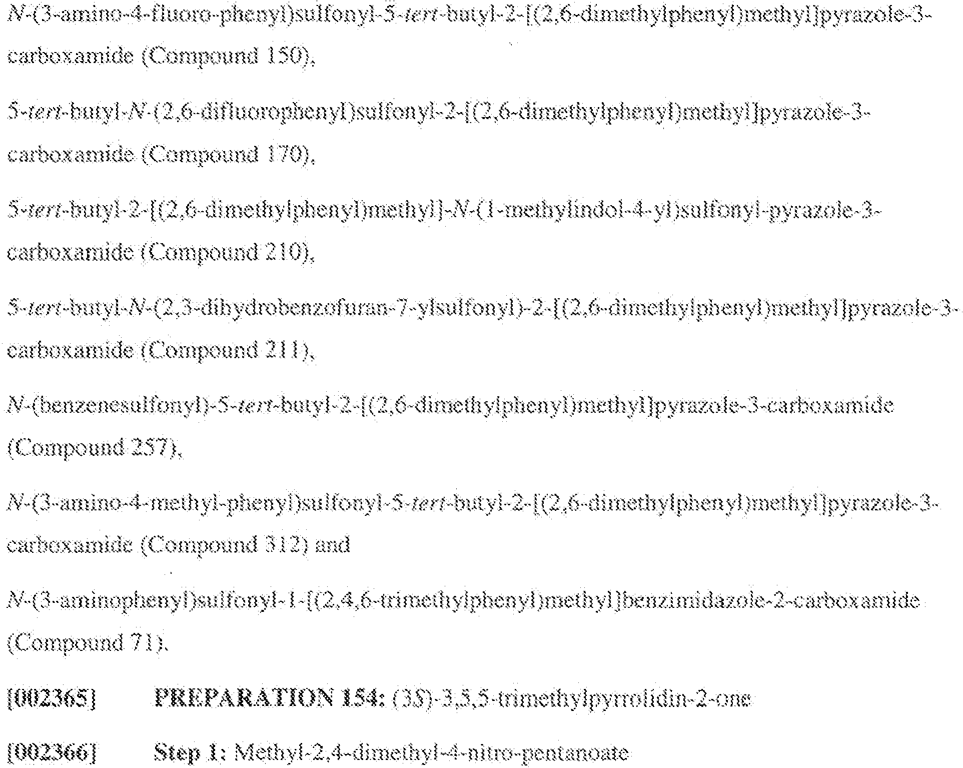

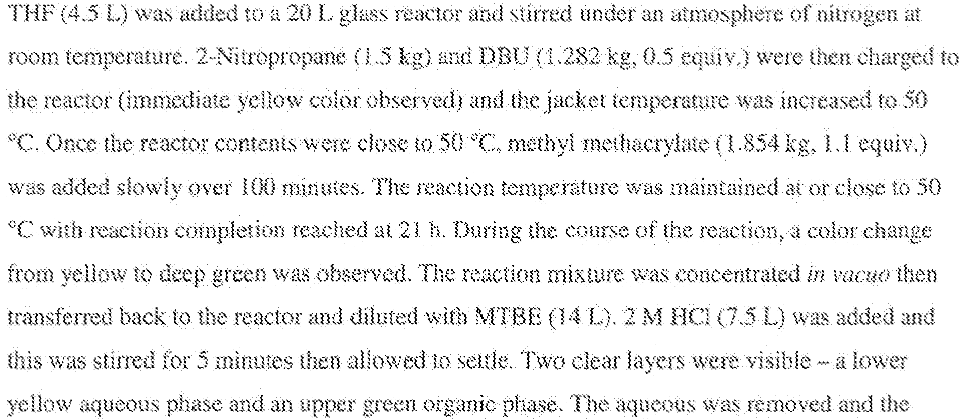

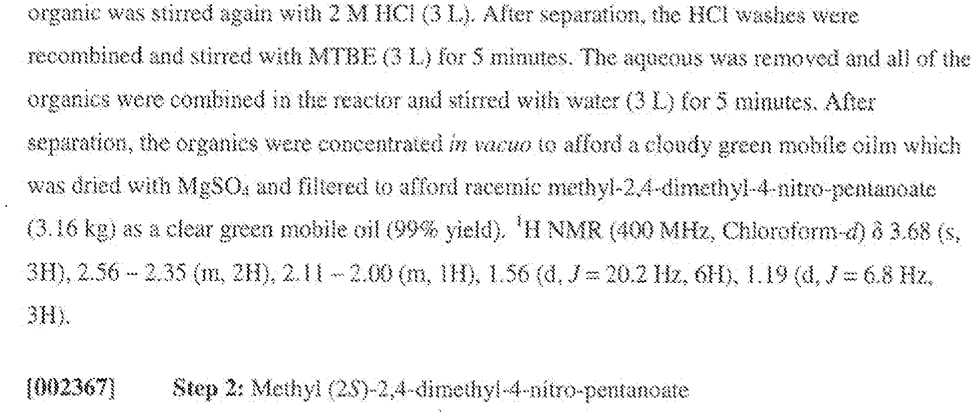

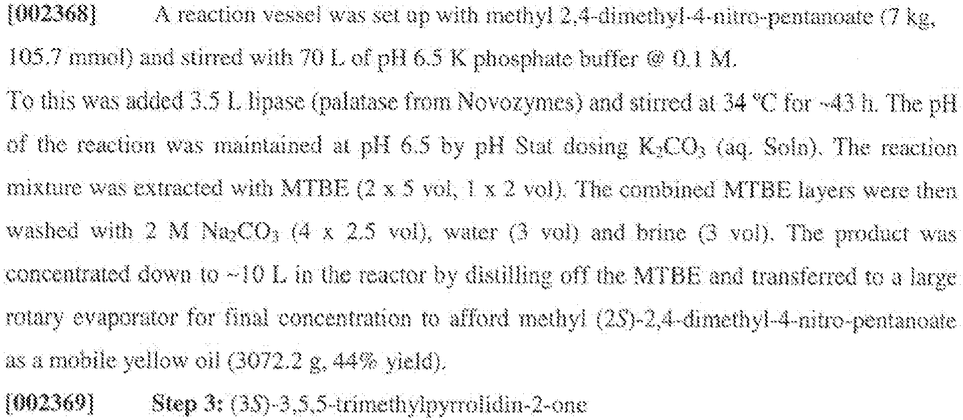

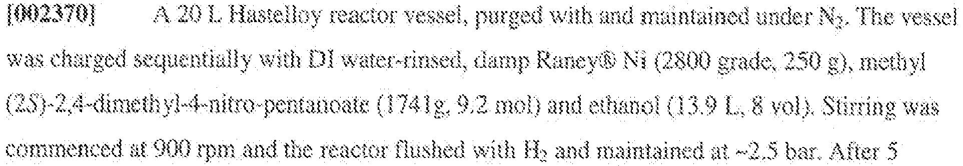

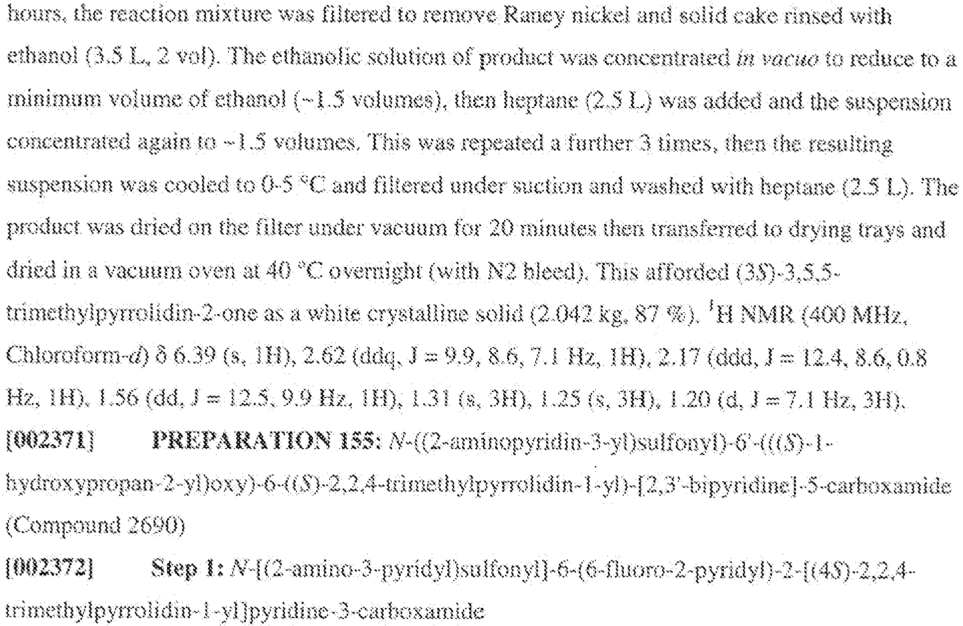

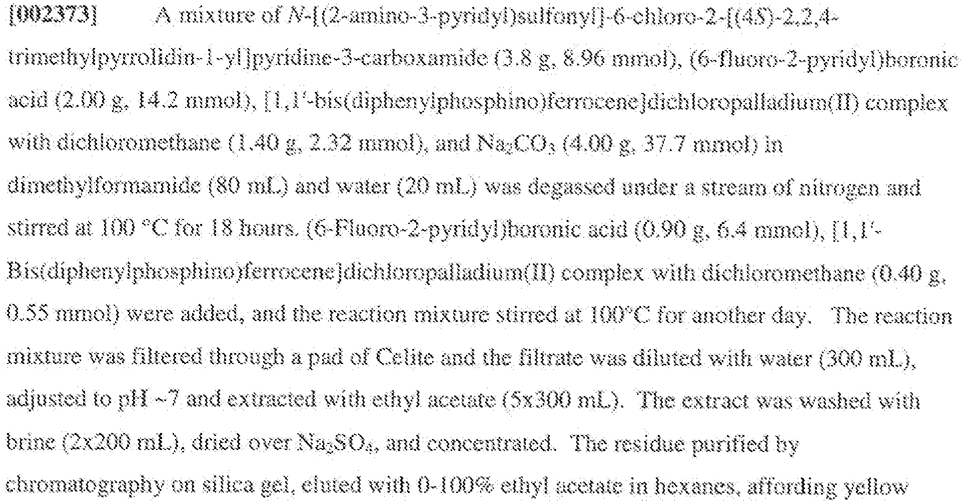

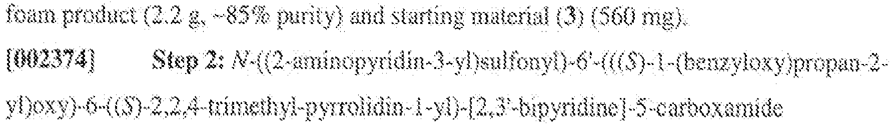

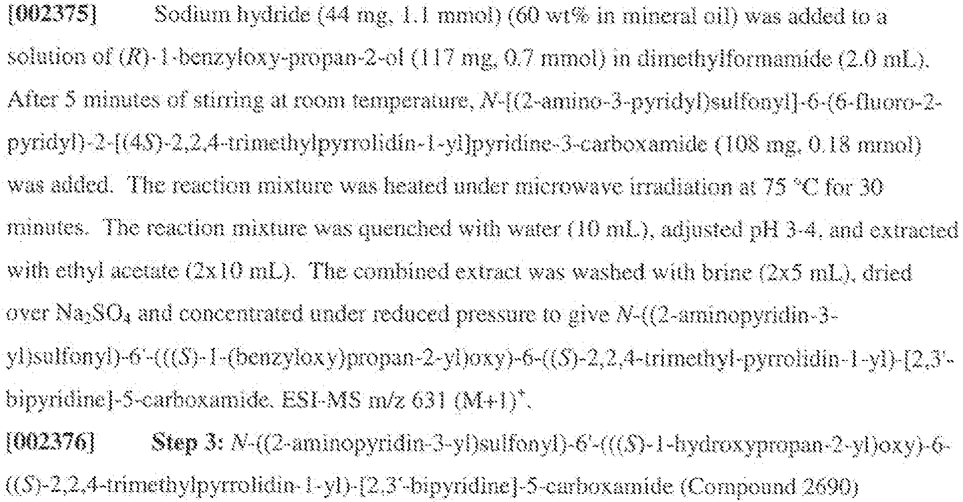

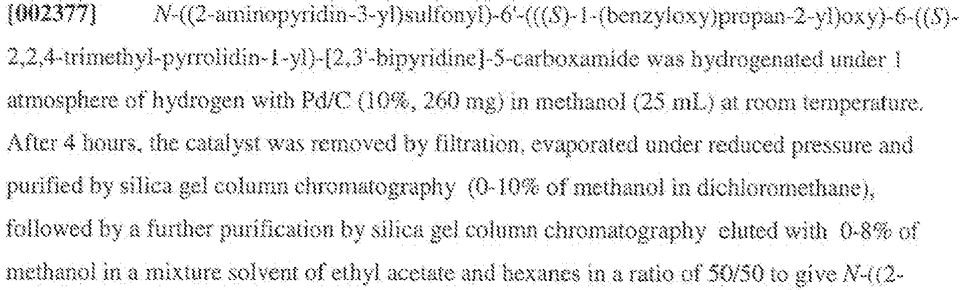

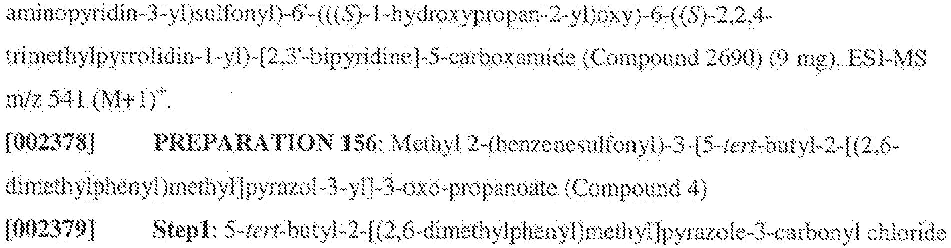

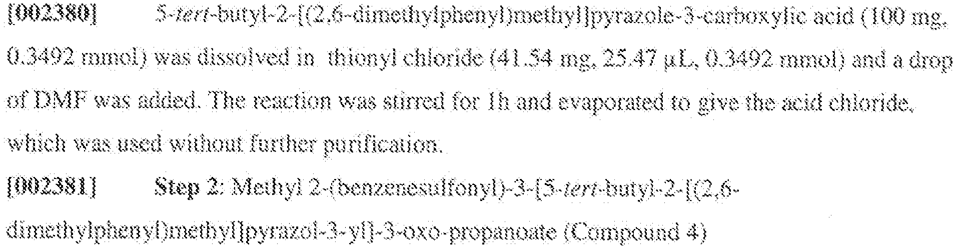

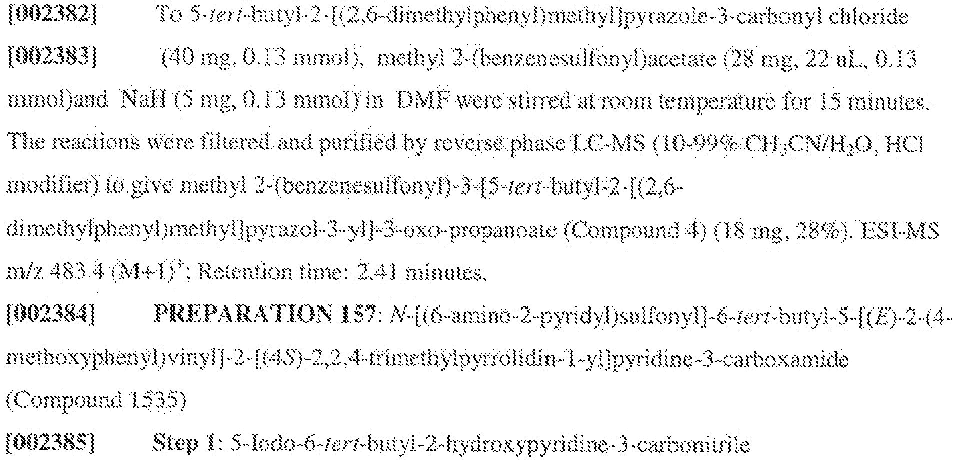

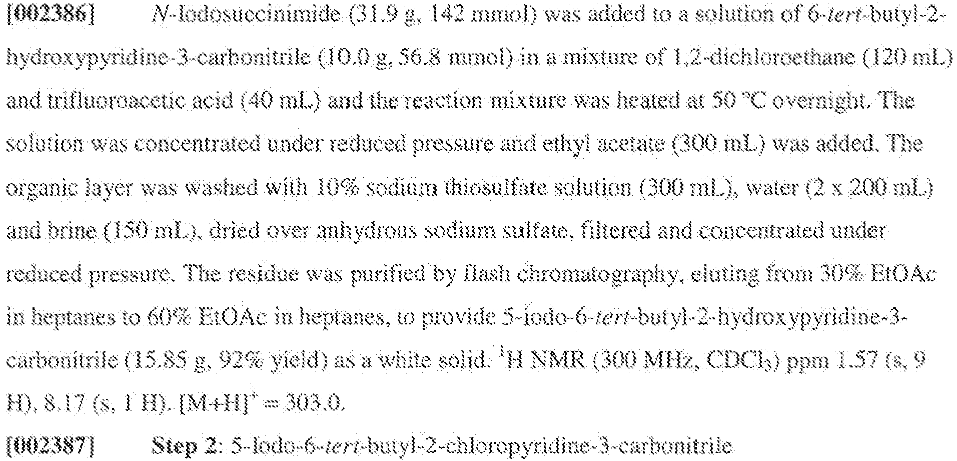

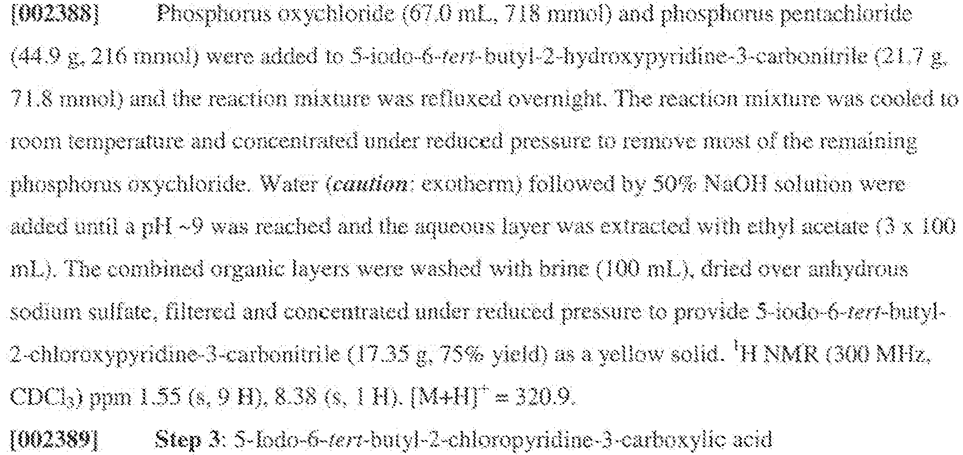

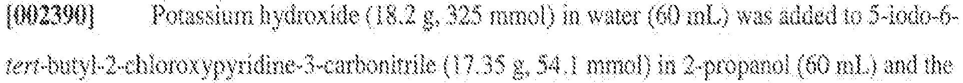

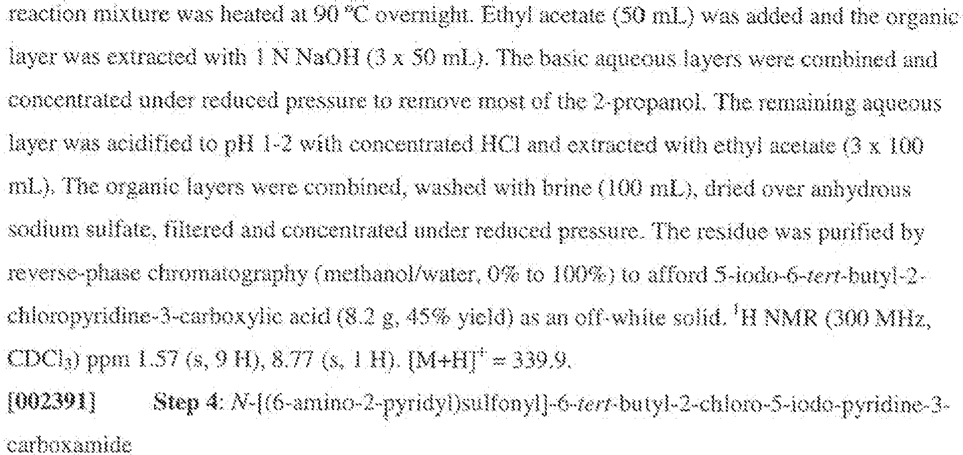

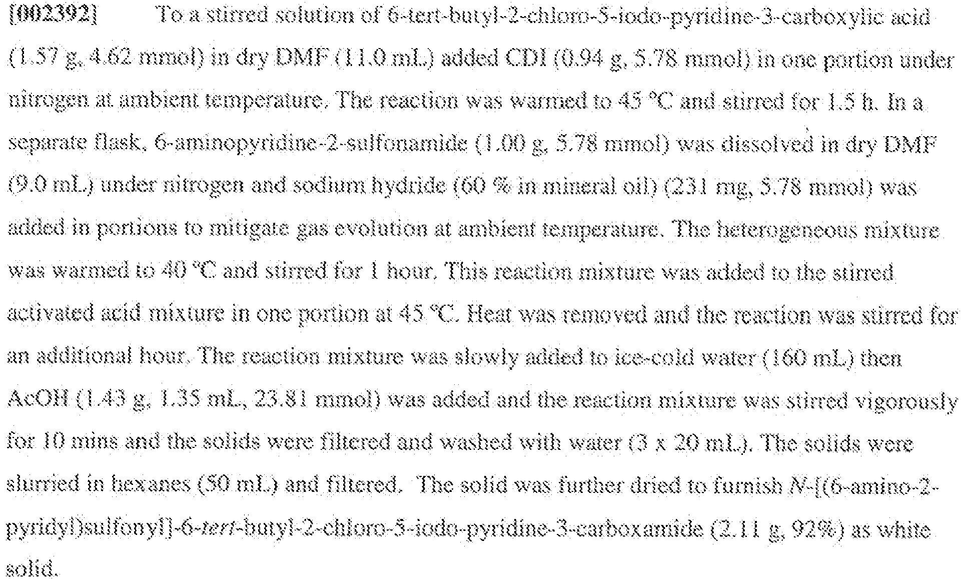

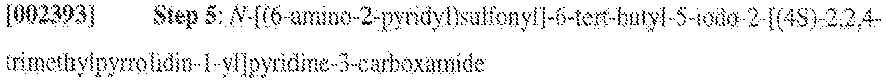

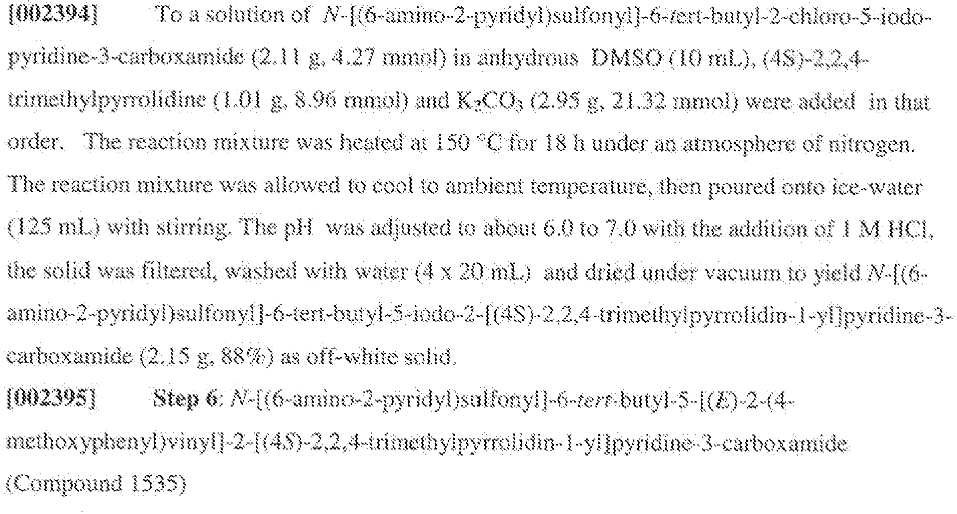

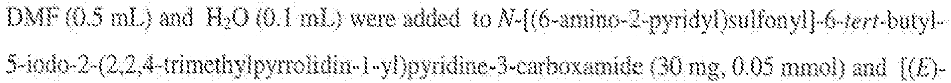

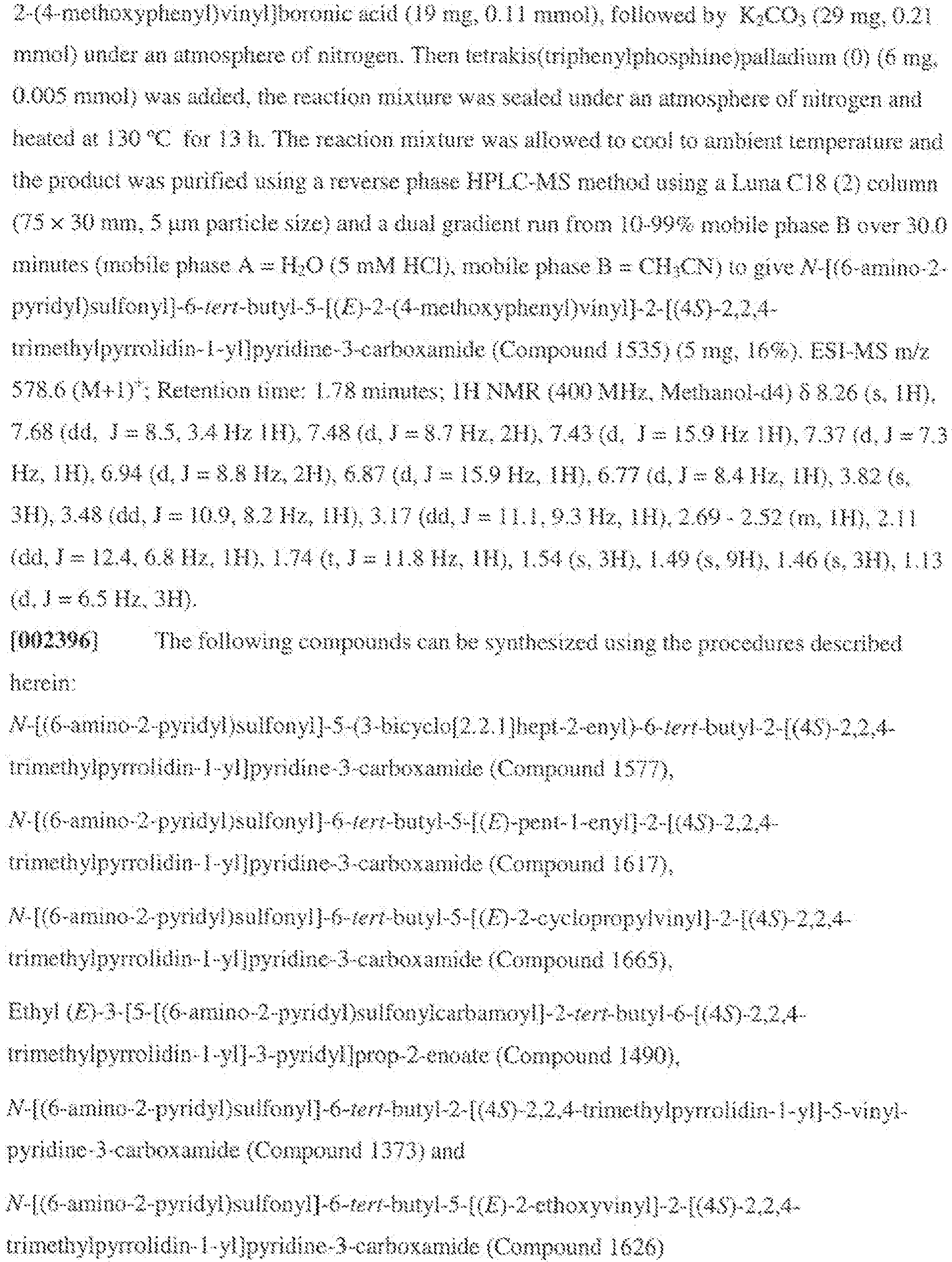

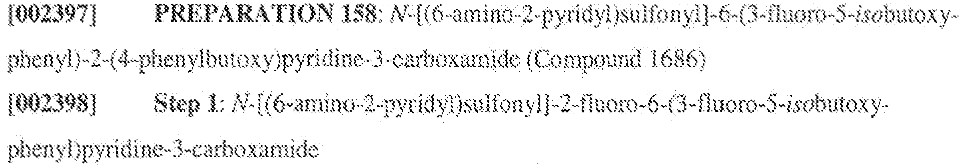

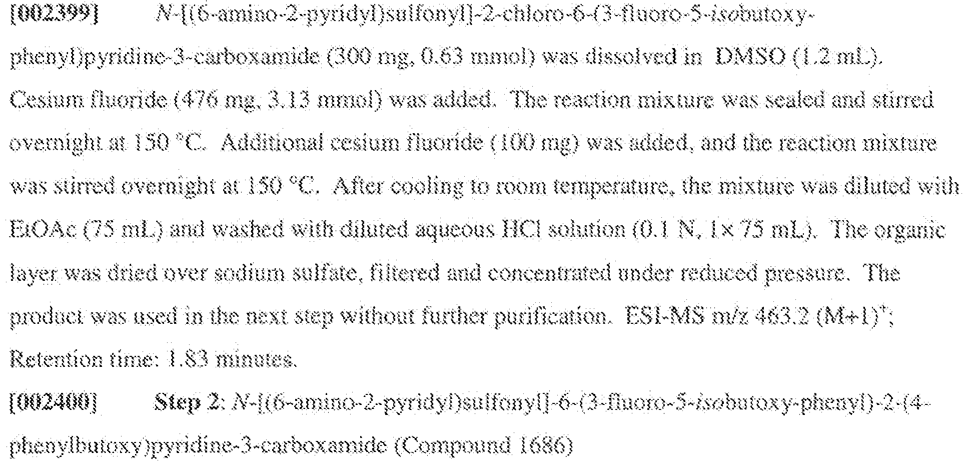

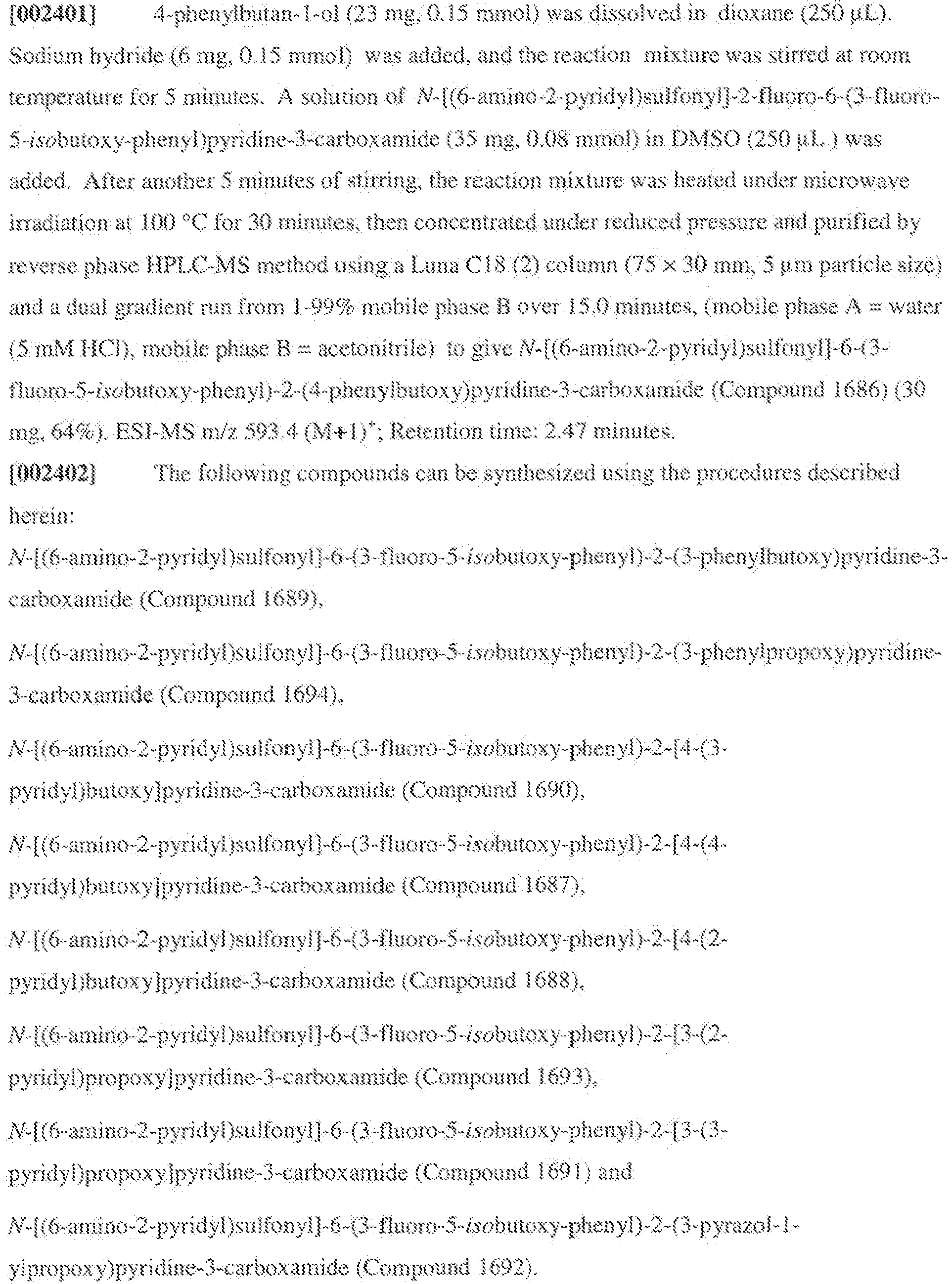

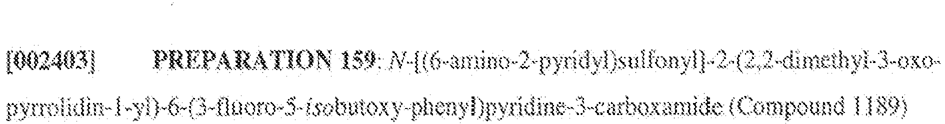

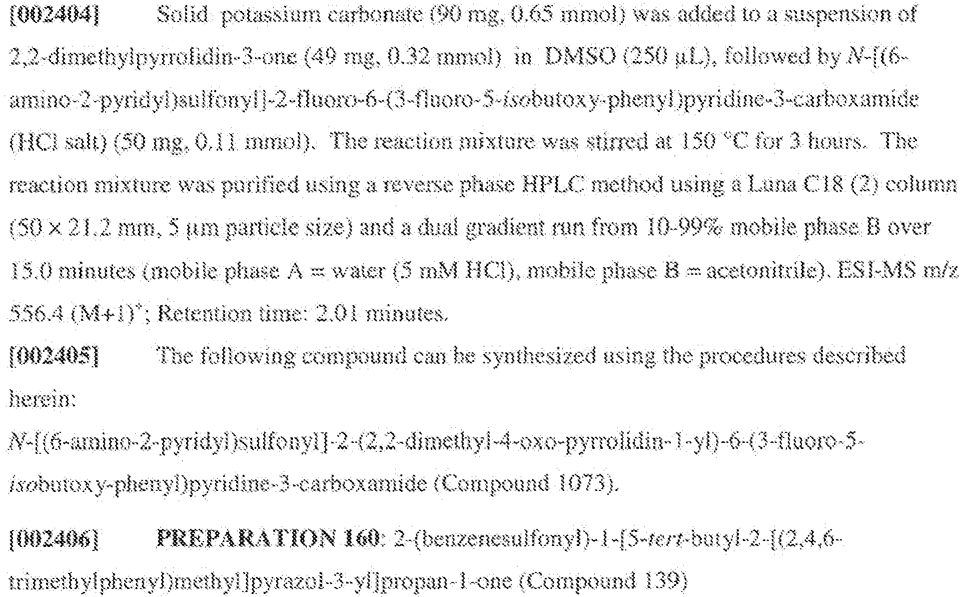

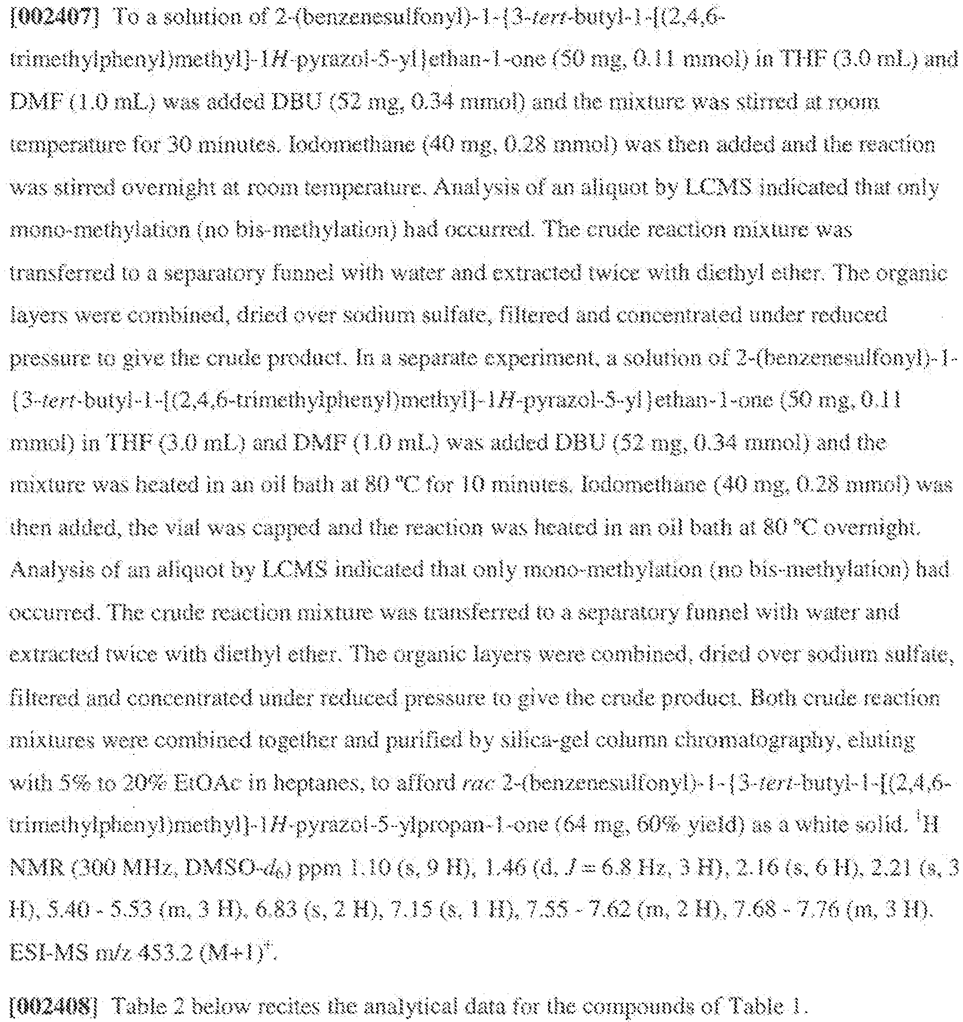

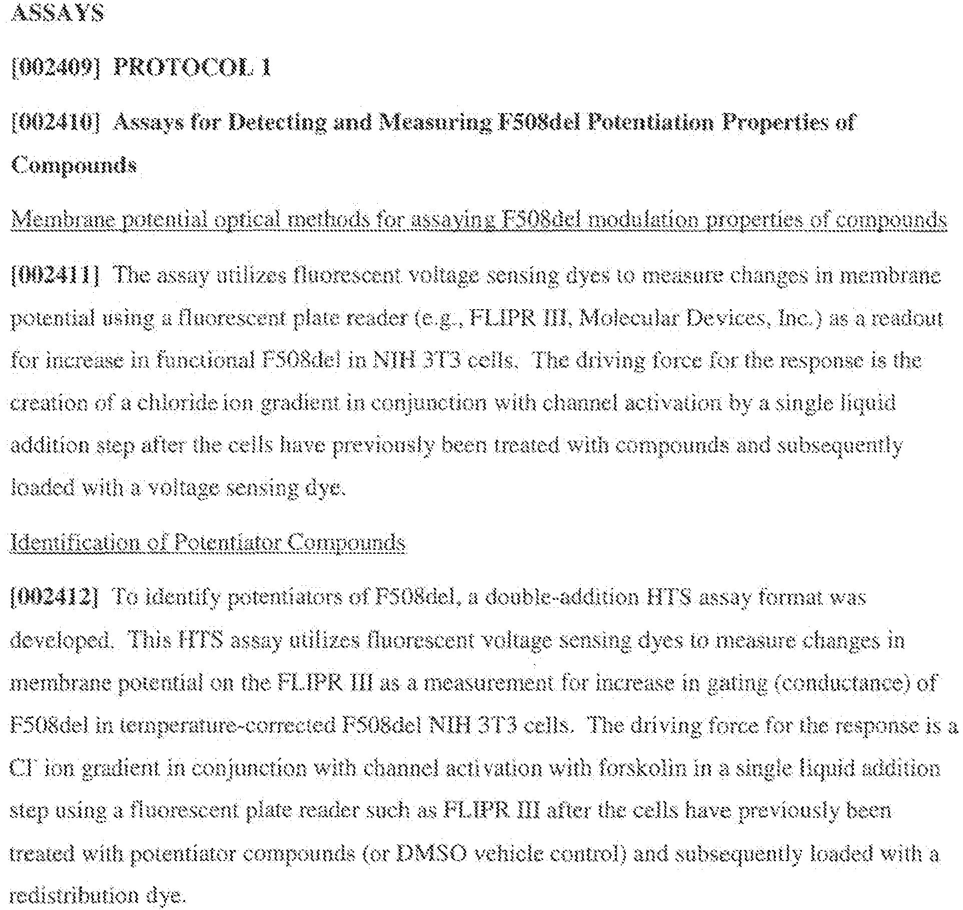

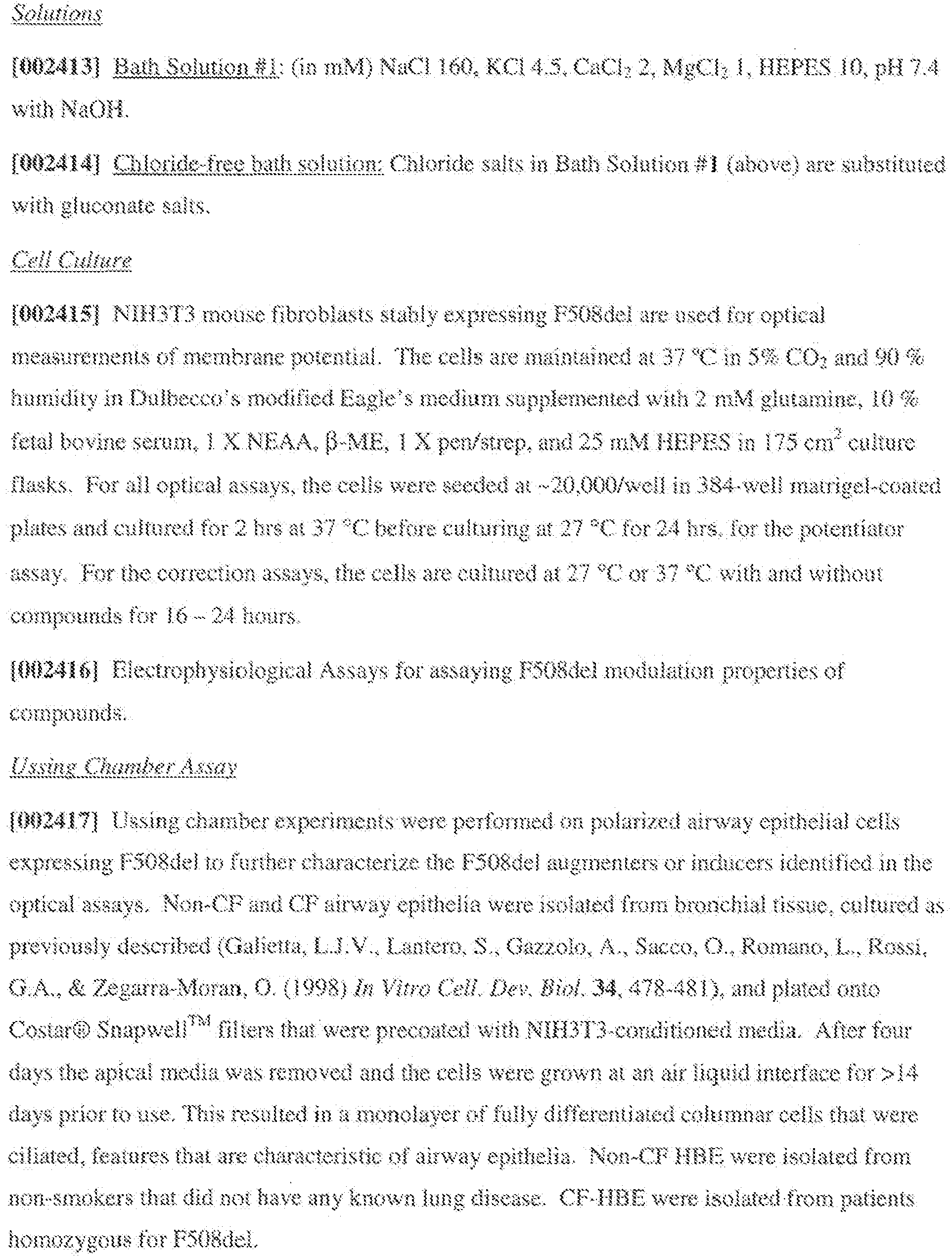

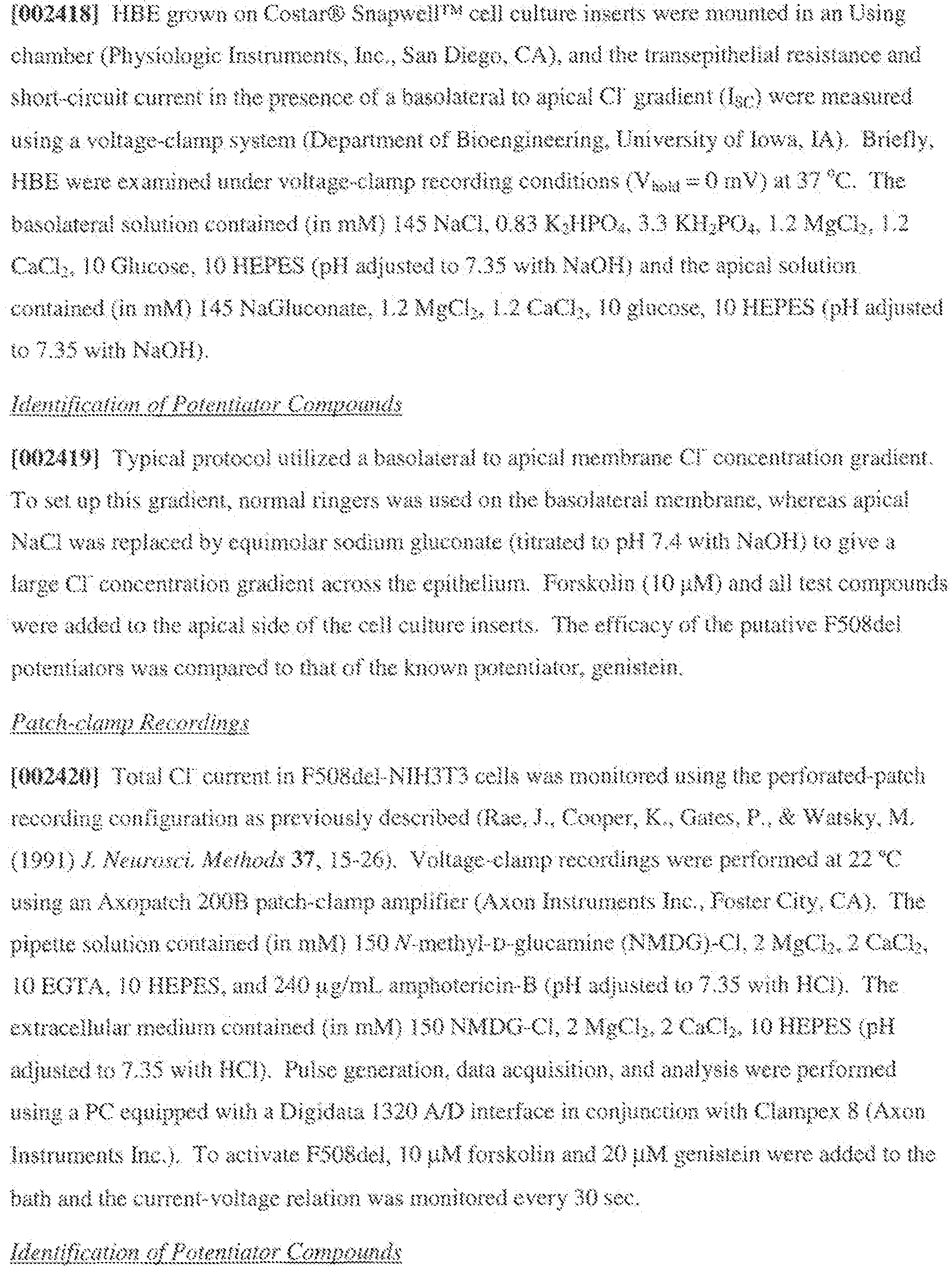

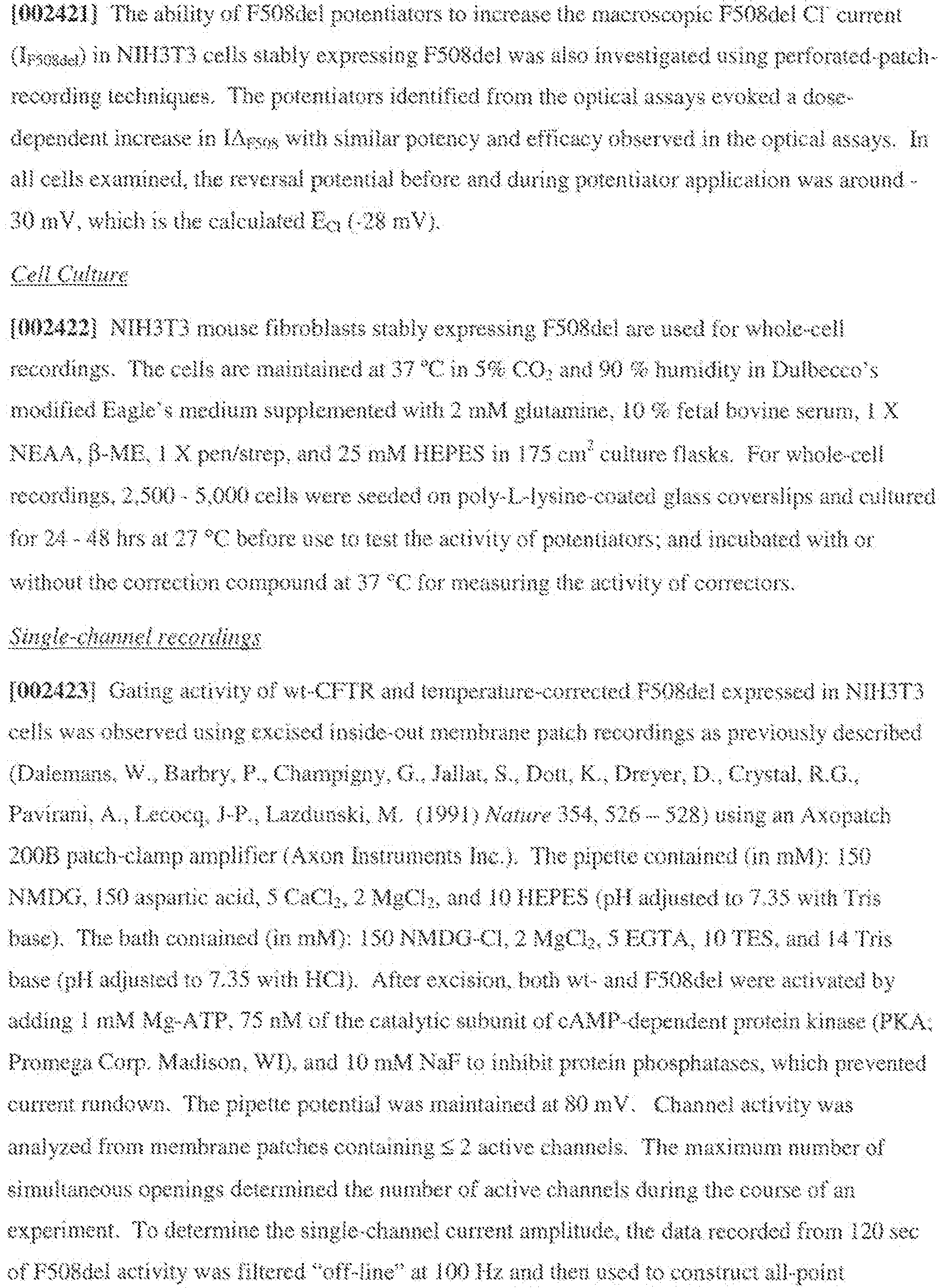

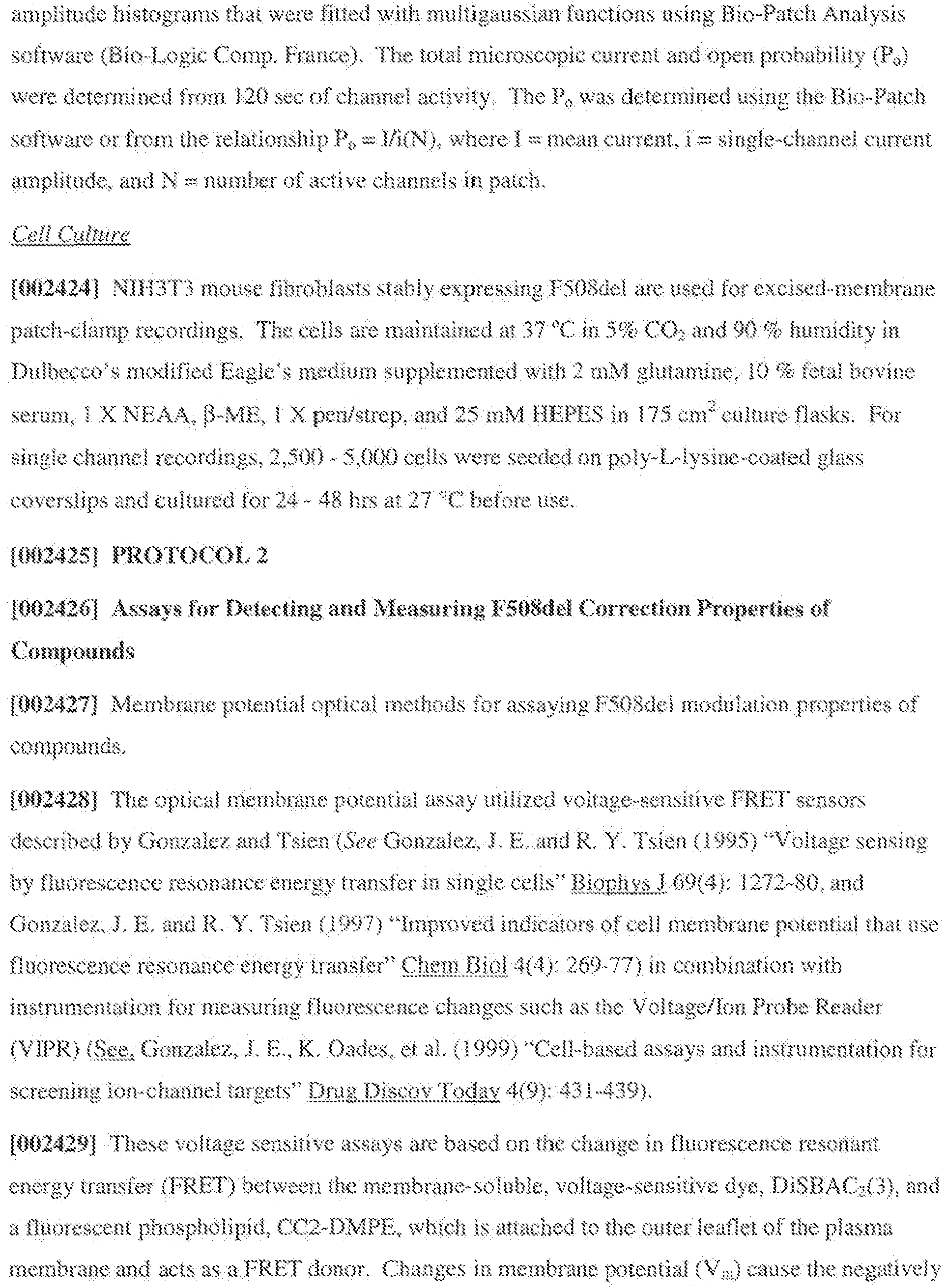

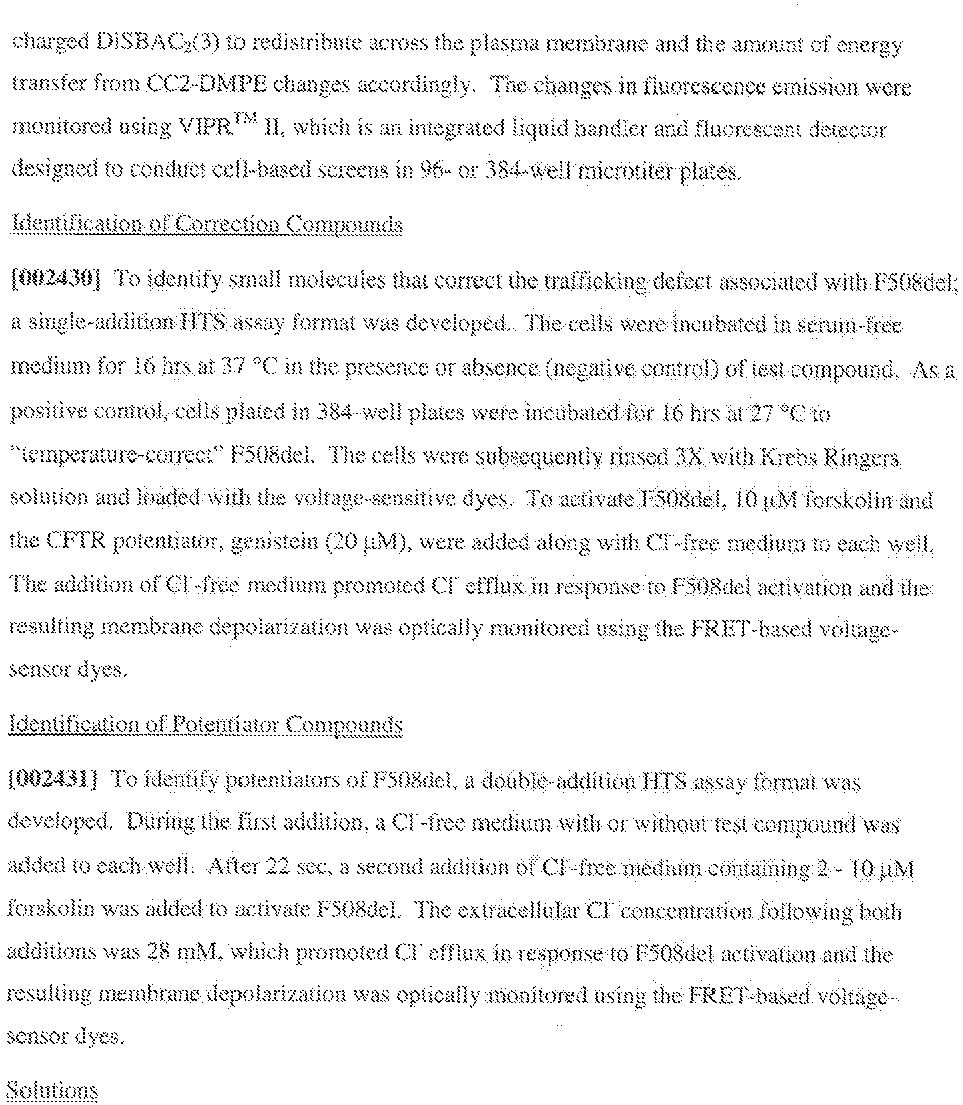

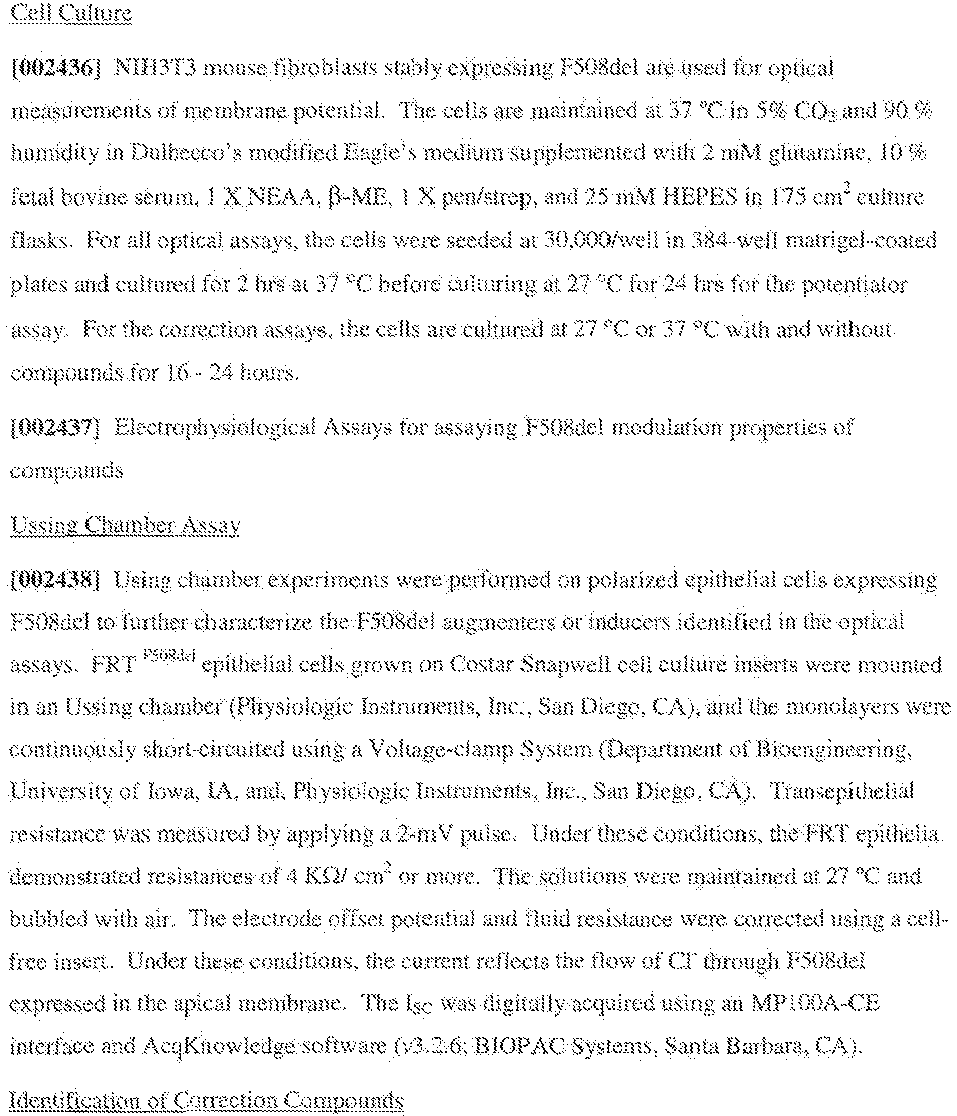

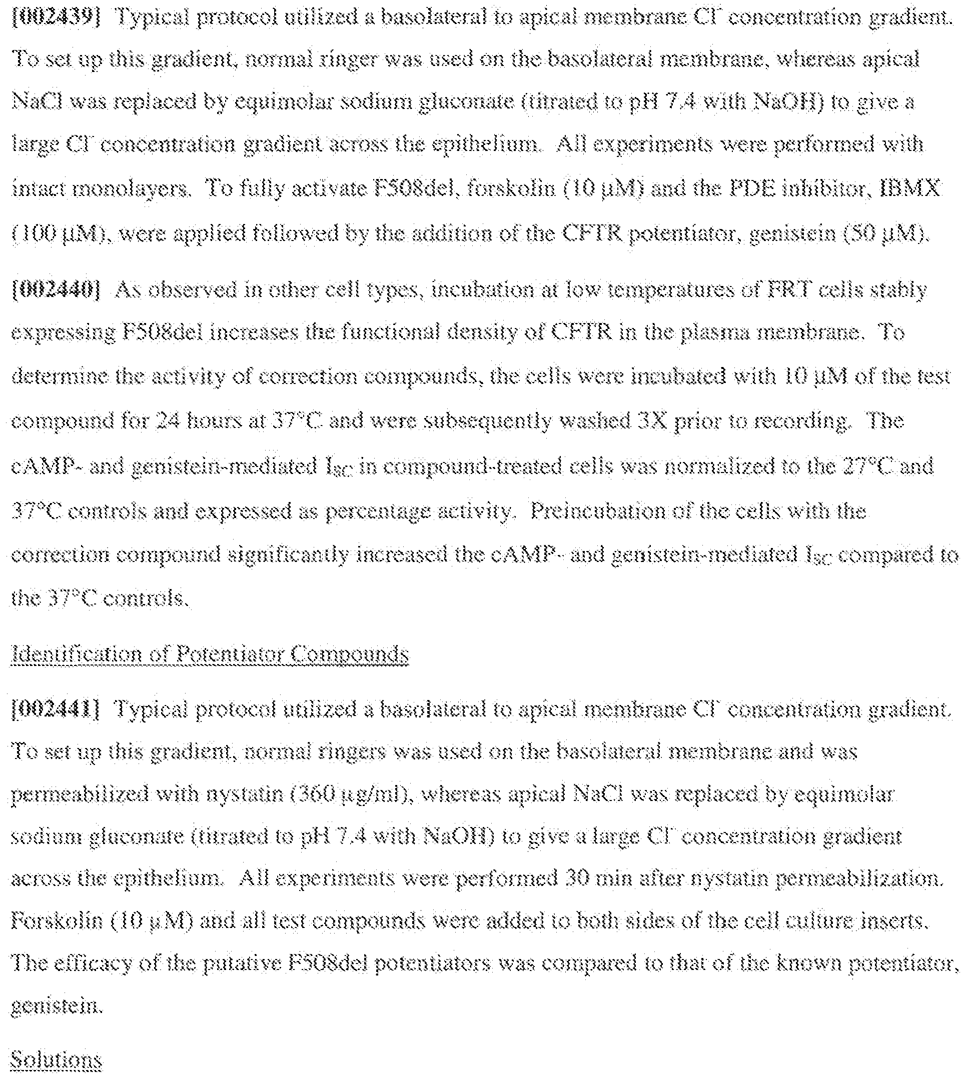

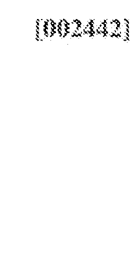

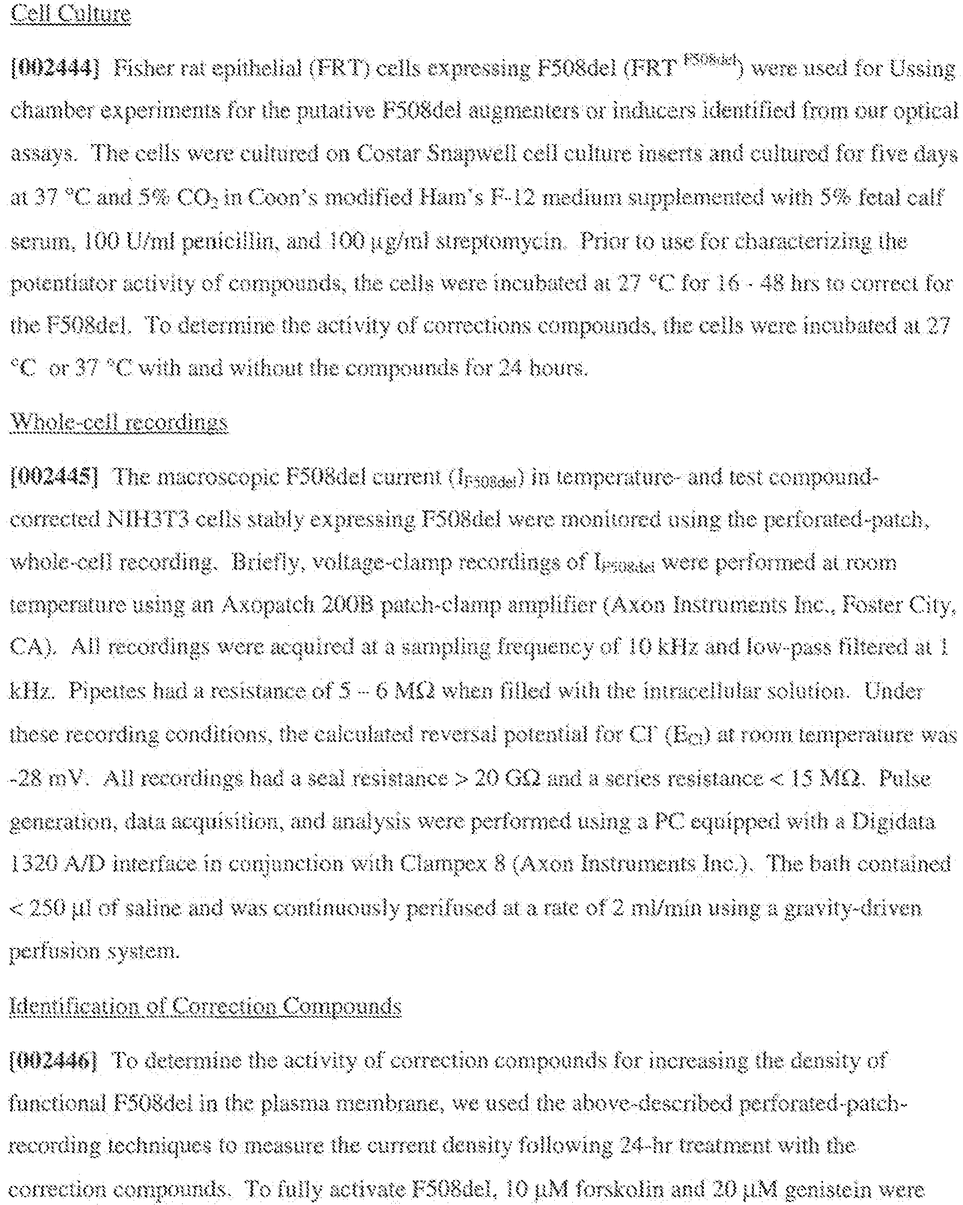

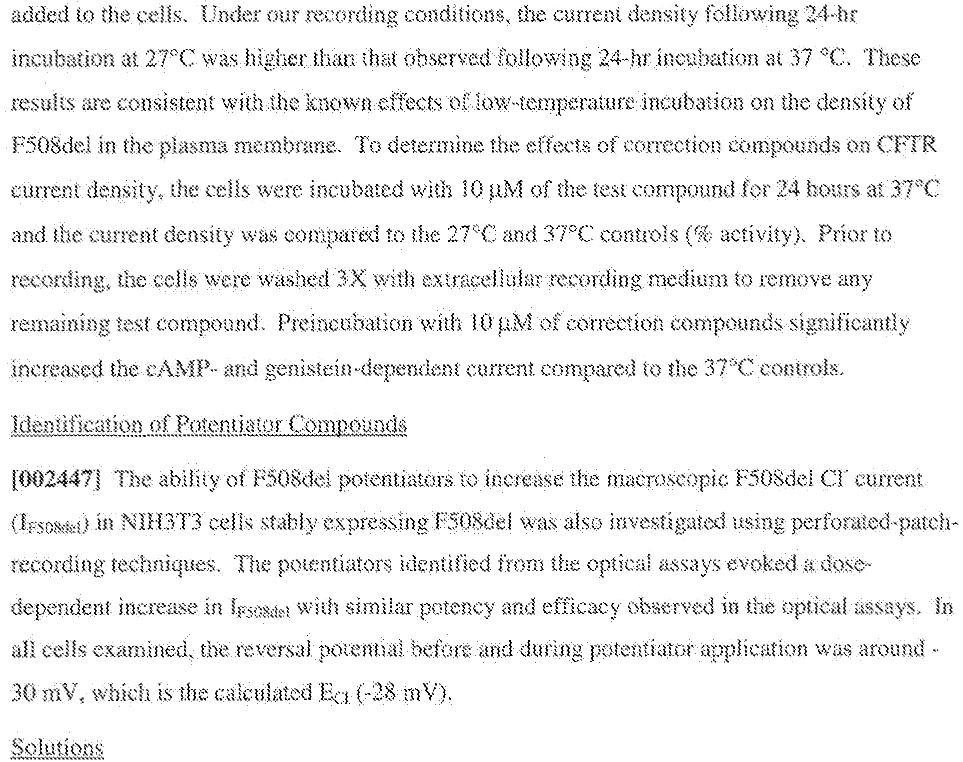

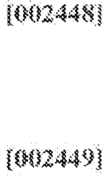

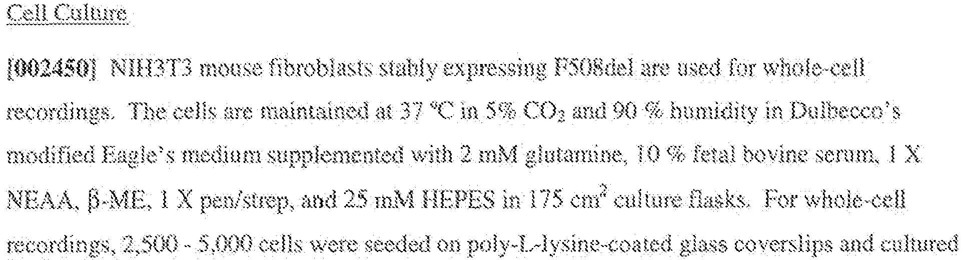

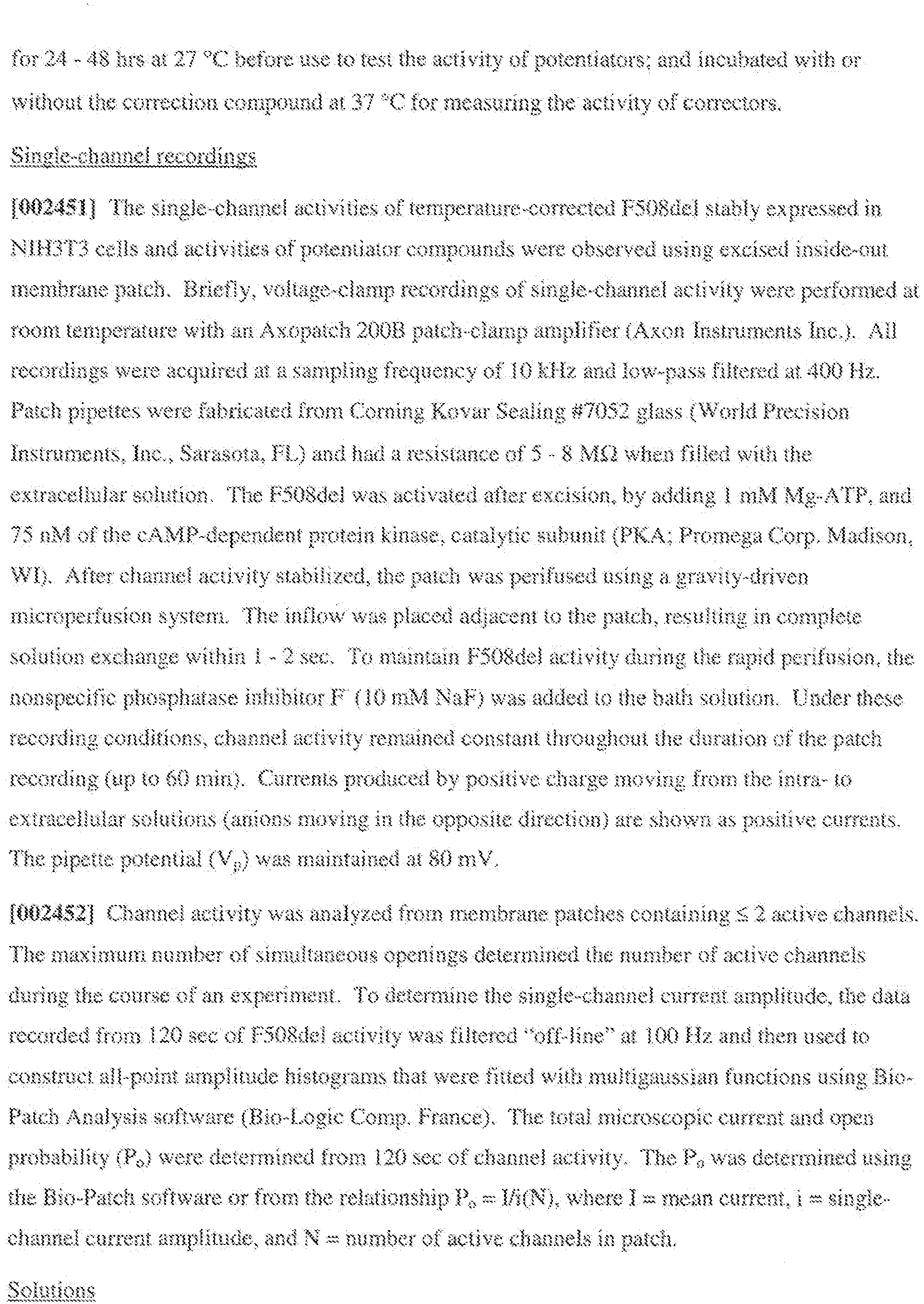

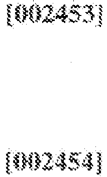

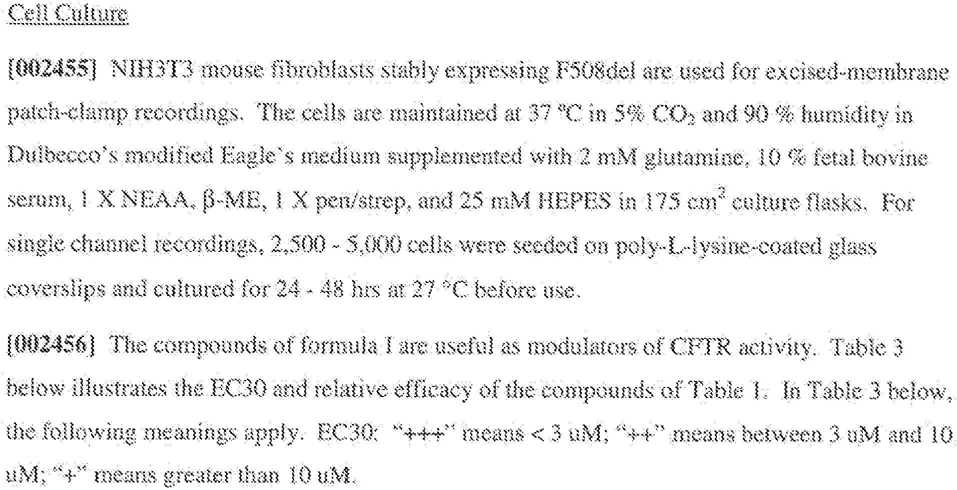

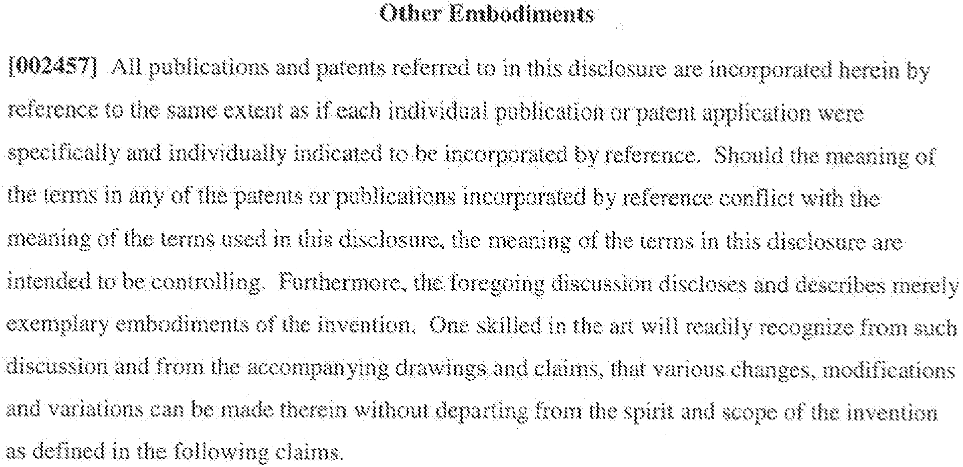

The invention provides the following numbered aspects:
1. A compound of formula I: 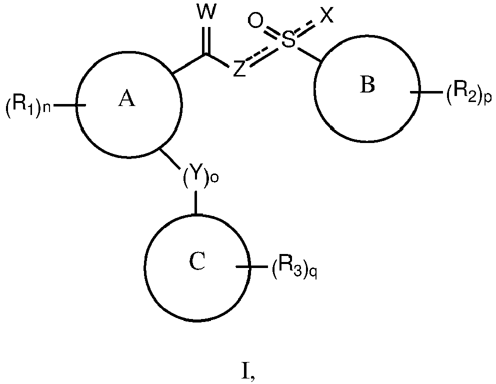 or a pharmaceutically acceptable salt thereof, wherein, independently for each occurrence:
   Ring A is a C6-C10 aryl ring; C3-C10 cycloalkyl ring; or a C3-C14 heteroaryl or heterocyclic ring wherein anywhere from 1 to 4 ring atoms are independently O, S, N, or NR;
   Ring B is a C3-C10 cycloalkyl ring; a C6-C10 aryl ring; or a C4-C10 heteroaryl or heterocyclic ring wherein anywhere from 1 to 4 ring atoms are independently O, S, N, or NR;
   Ring C is a C6-C10 aryl ring; a C3-C14 heteroaryl or heterocyclic ring wherein anywhere from 1 to 4 ring atoms are independently N, NR, O, or S; or a C3-C10 cycloalkyl ring;
   W is O, NR, or S;
   X is O or NR;
   Y is independently CRR, CO, O, S, SO, SO₂, S(O)NH or NR;
   Z is NR or CHR;
   R₁ is halo; CN; F₅S; SiR3; OH; NRR; C1-C6 alkyl or fluoroalkyl; C1-C6 alkoxy or fluoroalkoxy; C1-C6 alkenyl; C1-C6 alkynyl; (C1-C9 alkylene)-R4 wherein up to four CH₂ units are independently replaced with O, CO, S, SO, SO₂ or NR; C6-C10 aryl; C3-C10 heteroaryl or heterocyclic ring wherein anywhere from 1 to 4 ring atoms are independently O, S, N, or NR; or C3-C10 cycloalkyl;
   R2 is halo; OH; NRR; azide; CN; CO₂R; C1-C6 alkyl or fluoroalkyl;C1-C6 alkoxy or fluoroalkoxy;C1-C6 alkenyl; C1-C6 alkynyl; C6-C10 aryl; C3-C13 heteroaryl or heterocyclic ring wherein anywhere from 1 to 4 ring atoms are independently O, S, N, or NR; C3-C10 cycloalkyl; or a (C1-C9 alkylene)-R₄ wherein up to four CH2 units are independently replaced with O, CO, S, SO, SO₂ or NR;
   or two R2 groups taken together may form a =CH₂ or =O group;
   R3 is halo; CN; OH; CO₂R; C1-C6 alkyl or fluoroalkyl; C1-C6 alkenyl; C1-C6 alkynyl; C1-C6 alkoxy or fluoroalkoxy; or C6-C10 aryl; C3-C10 heteroaryl or heterocyclic ring wherein anywhere from 1 to 4 ring atoms are independently O, S, N, or NR; C3-C10 cycloalkyl; or a (C1-C9 alkylene)-R4 wherein up to four CH2 units are independently replaced with O, CO, S, SO, SO₂ or NR;
   or two R3 groups taken together may form a =CH₂ or =O group;
   R4 is H; azide; CF₃; CHF₂; OR; CCH; CO₂R; OH; C6-C10 aryl, C3-C10 heteroaryl or heterocycloalkyl wherein anywhere from 1 to 4 ring atoms are independently O, S, N, or NR; C3-C10 cycloalkyl; NRR, NRCOR, CONRR, CN, halo, or SO₂R;
   R is independently H; OH; CO₂H; CO₂C1-C6 alkyl; C1-C6 alkyl; C1-C6 alkenyl; C1-C6 alkynyl; C6-C10 aryl; C3-C10 heteroaryl or heterocycloalkyl wherein anywhere from 1 to 4 ring atoms are independently O, S, N, or NR; or C3-C10 cycloalkyl;
   n is 0, 1, 2 or 3;
   o is 0, 1, 2, 3, 4, or 5;
   p is 0, 1, 2, or 3; and
   q is 0, 1, 2, 3, 4, or 5;
   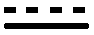 is a single bond or a double bond;
   provided that the moieties containing ring B and ring C are substituted at adjacent positions on ring A.
2. The compound of aspect 1, wherein ring A is a pyridyl, indole, indoline, isoindoline, pyrazole, pyrimidine, phenyl, quinoline, 5,6,7,8-tetrahydroquinoline, 1,2,3,4-tetrahydroisoquinoline, pyrrolidine, aza-indole, pyrrole, oxazole, pyrazine, triazole, benzimidazole, indazole, or imidazole ring.
3. The compound of aspect 1 or 2, wherein ring A is selected from 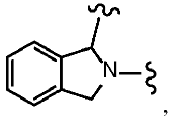 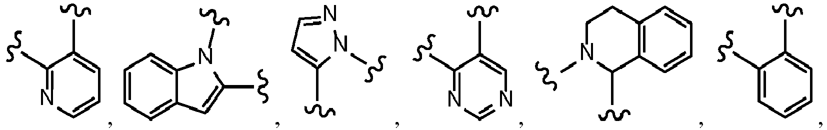 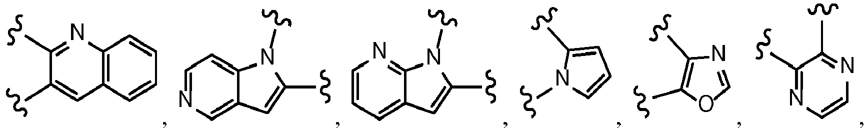 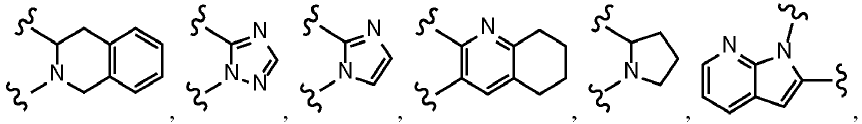 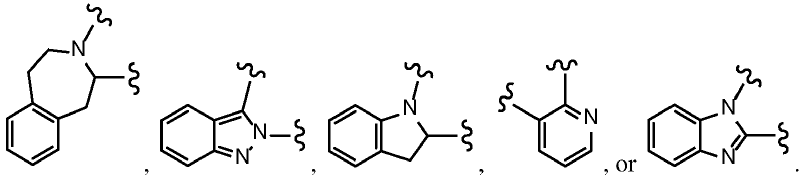
4. The compound of any one of aspects 1 to 3, wherein ring B is phenyl, pyridyl, pyridine-2(1*H*)-one, pyrazole, indole, thiophene, dihydrobenzofuran, pyrazine, indazole, thiazole, pyridine-4(1H)-one, pyrrolidinone, or quinoline.
5. The compound of any one of aspects 1 to 4, wherein ring B is selected from 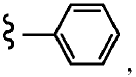 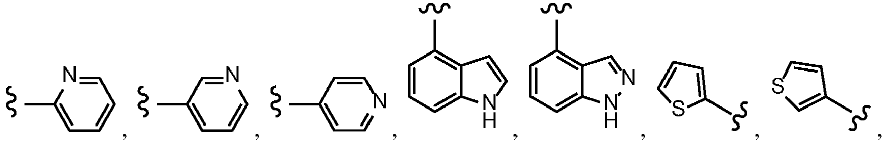 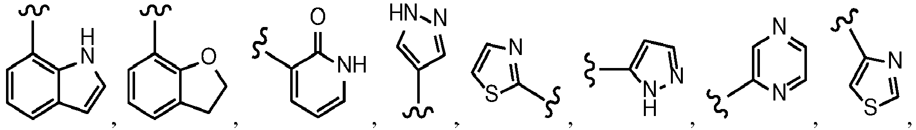 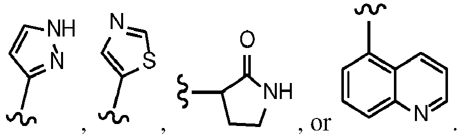
6. The compound of any one of aspects 1 to 5, wherein ring C is phenyl, indole, cycloalkyl, pyridyl, pyrrolidine, naphthalene, piperidine, azetidine, or dihydroindene.
7. The compound of any one of aspects 1 to 6, wherein ring C is 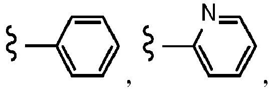 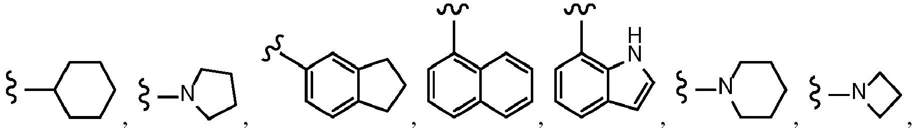 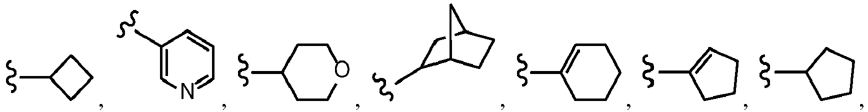 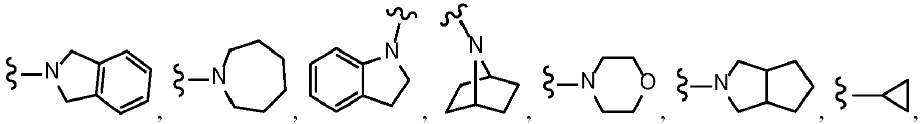 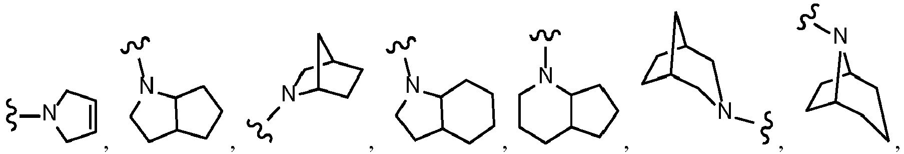 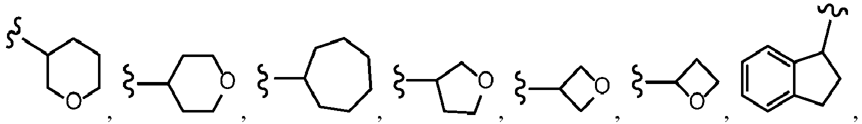 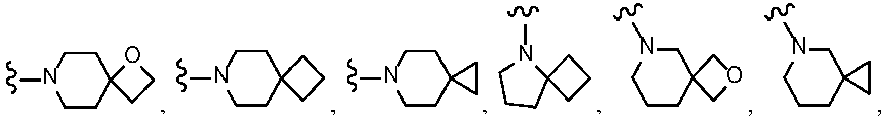 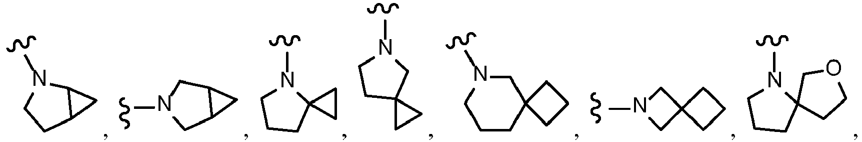 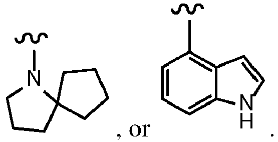
8. The compound of any one of aspects 1 to 7, wherein Y is O.
9. The compound of any one of aspects 1 to 7, wherein Y is CH2.
10. The compound of any one of aspects 1 to 7, wherein Y is CH(C1-C6 aliphatic).
11. The compound of any one of aspects 1 to 7, wherein Y is CH(CH3).
12. The compound of any one of aspects 1 to 7, wherein Y is CH(CH2CH3).
13. The compound of any one of aspects 1 to 7, wherein Y is N(C1-C6 aliphatic).
14. The compound of any one of aspects 1 to 7, wherein Y is N(CH₃).
15. The compound of any one of aspects 1 to 14, wherein R₁ is halo, CN, C1-C6 aliphatic, C1-C6 alkoxy, C3-C8 cycloalkyl, or a phenyl, pyridyl, pyrimidine, indole, aza-indole, azetidine, or thiophene ring, wherein all rings may be substituted with halo, C1-C6 aliphatic, C1-C6 alkoxy, C1-C6 fluoroaliphatic, C1-C6 fluoroalkoxy, OH, CH₂OH, CH₂OCH₃, CN, CO₂H, amino, amido, C3-C10 heteroaryl, C3-C10 heterocycloalkyl, or a (C1-C8 aliphatic)-R4 wherein up to three CH2 units may be replaced with O, CO, S, SO, SO₂ or NR.
16. The compound of any one of aspects 1 to 15, wherein R₁ is selected from CH₃, Cl, F, CN, OCH₃, CF₃, CH₂CH₃, tBu, CH(CH₃)₂, 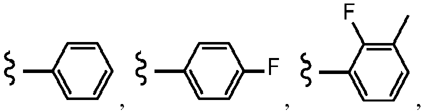 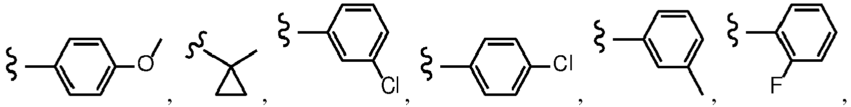 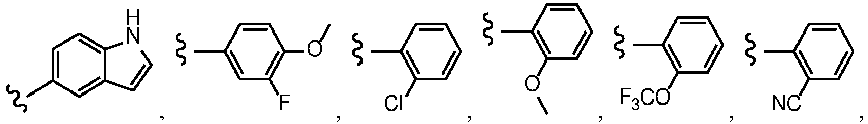 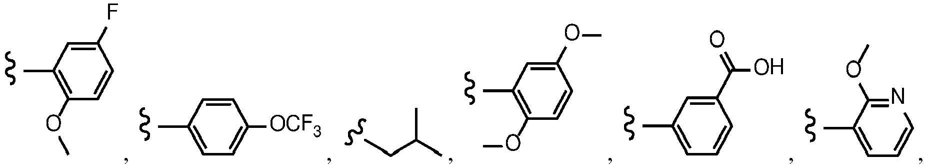 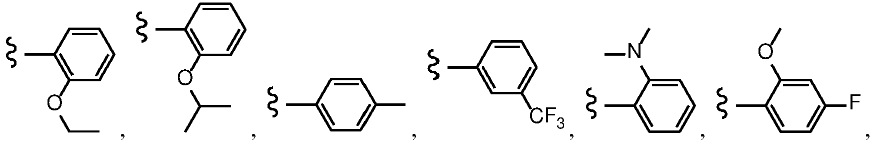 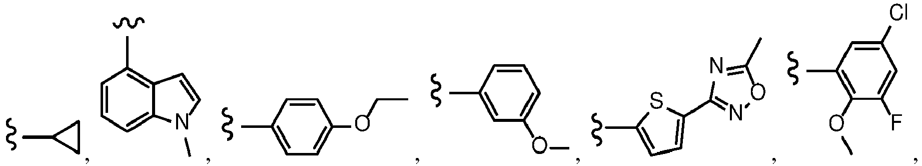 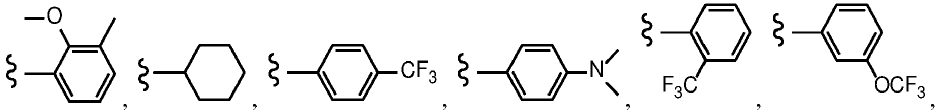 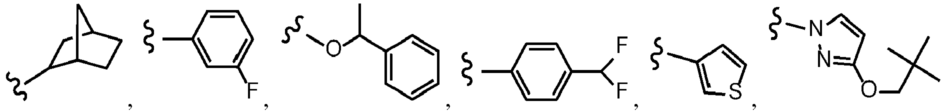 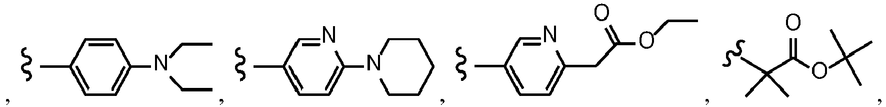 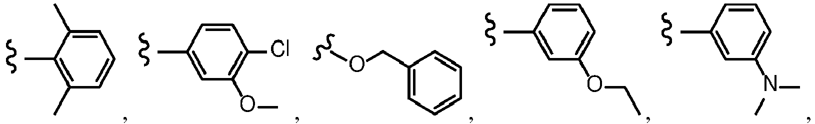 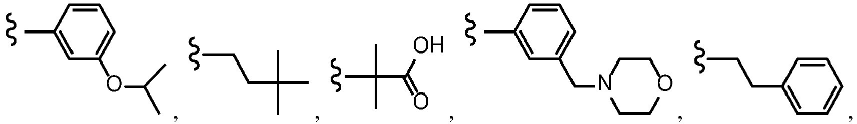 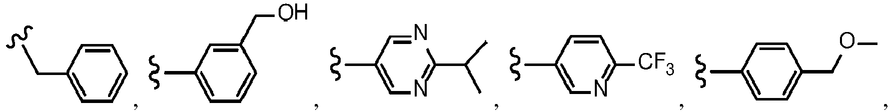 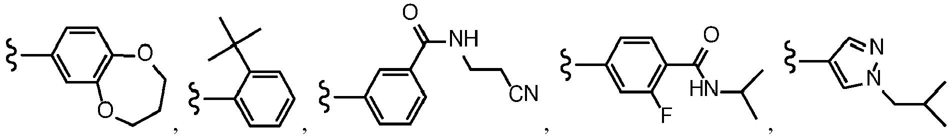 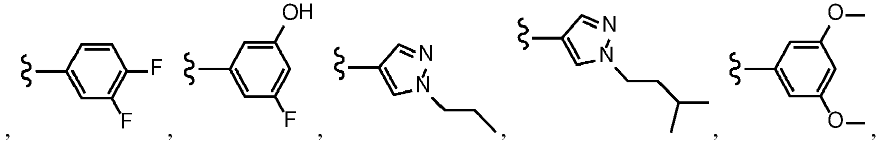 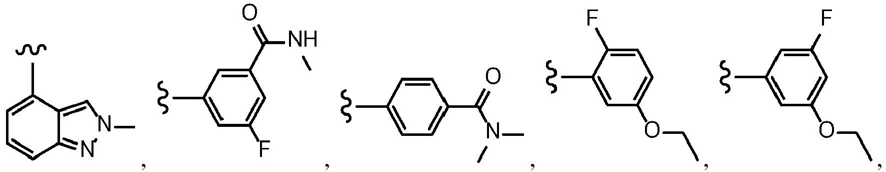 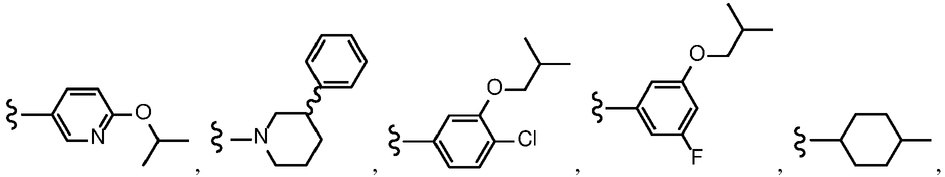 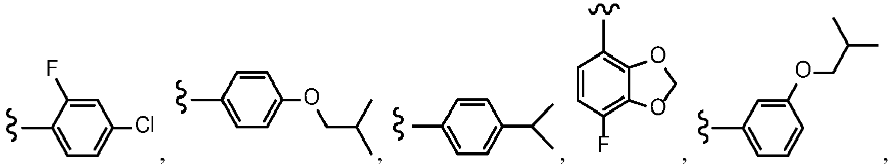 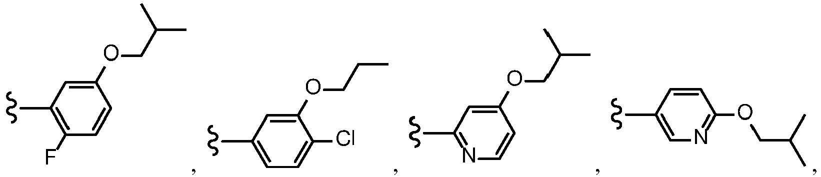 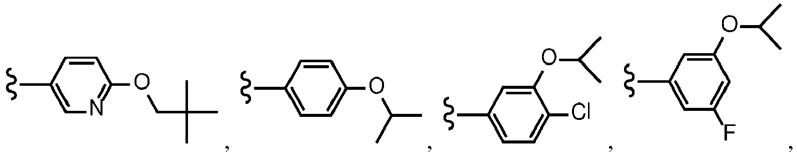 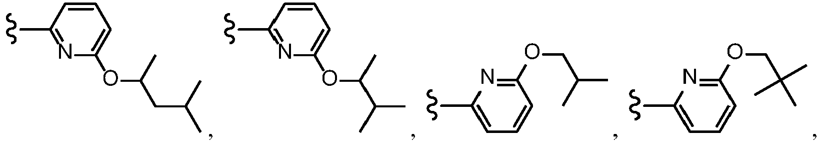 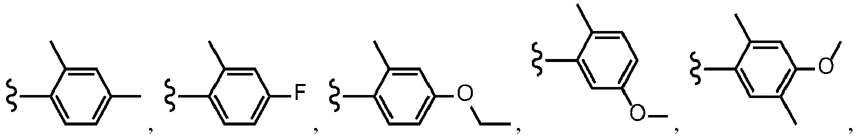 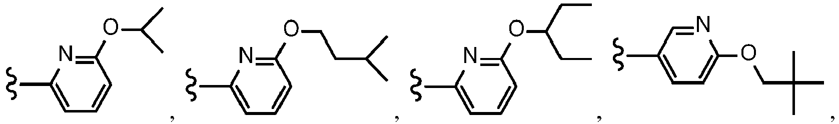 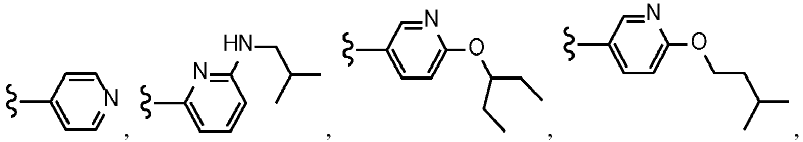 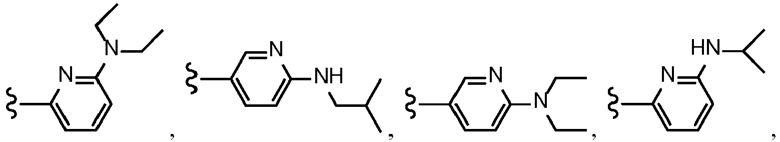 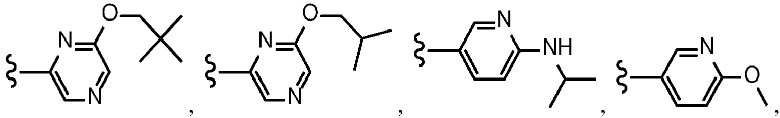 or 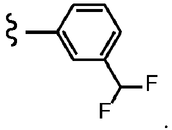
17. The compound of any one of aspects 1 to 16, wherein R2 is selected from halo, OH, CN, azide, amino, C1-C6 aliphatic or fluoroaliphatic, C1-C6 alkoxy or fluoroalkoxy, C3-C10 mono- or bicyclic heterocyclic ring wherein up to 4 carbon atoms may be replaced with O, S, N, or NR; or a (C1-C8 aliphatic)-R4 wherein up to three CH2 units may be replaced with O, CO, S, SO, SO₂ or NR.
18. The compound of any one of aspects 1 to 17, wherein R2 is selected from Cl, F, OH, CN, N₃, NH₂, NH(CH₃), N(CH₃)₂, CH₃, CH₂OH, CH₂CH₃, CH(CH₃)₂, CHF₂, OCH₃, OCF₃, OCHF₂, OCH(CH₃)₂, C(O)CH₃, CH₂CH₂OH, CH₂NH₂, NH(CH₂)₂OH, NH(CH₂)₂N(CH₃)₂, NH(CH₂)₂NH₂, NH(CH₂)₃NH₂, NH(CH₂)₂OCH₃, NHCH(CH₃)₂, 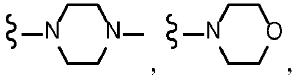 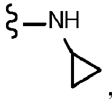 , or CO₂H.
19. The compound of any one of aspects 1 to 18, wherein R3 is selected from halo, CN, C1-C6 aliphatic or fluoroaliphatic, C1-C6 alkoxy, or C3-C10 mono- or bicyclic heteroaryl or heterocycloalkyl wherein up to 4 carbon atoms may be replaced by O, S, N, or NR.
20. The compound of any one of aspects 1 to 19, wherein R3 is selected from Cl, F, CN, CH₃, OCH₃, CF₃, CH₂CH₃, CH₂CF₃, CH₂CH₂CH₃, OCH₂CH₃, CH₂OCH₃, CH(CH₃)₂, CCH, CO₂CH₃, tBu, =CH₂, =O, 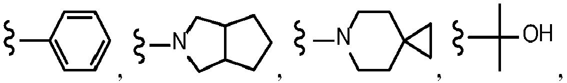 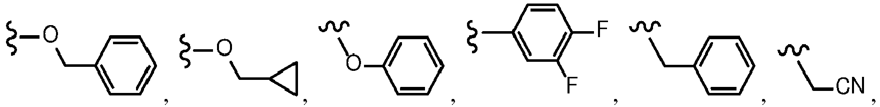 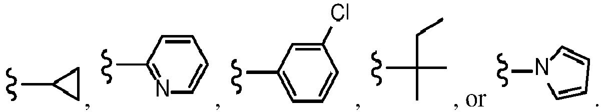
21. The compound of any one of aspects 1 to 20, wherein o is 0.
22. The compound of any one of aspects 1 to 20, wherein o is 1.
23. The compound of any one of aspects 1 to 20, wherein o is 2.
24. The compound of any one of aspects 1 to 23, wherein n is 0.
25. The compound of any one of aspects 1 to 23, wherein n is 1.
26. The compound of any one of aspects 1 to 23, wherein n is 2.
27. The compound of any one of aspects 1 to 26, wherein p is 0.
28. The compound of any one of aspects 1 to 26, wherein p is 1.
29. The compound of any one of aspects 1 to 26, wherein p is 2.
30. The compound of aspect 1, wherein the compound is of formula Ia: 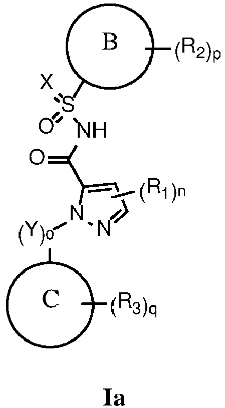 or a pharmaceutically acceptable salt thereof, wherein, independently for each occurrence:
   Ring B is a C6-C10 aryl ring or C3-C10 heteroaryl or heterocyclic ring wherein anywhere from 1 to 4 ring atoms are independently O, S, N, or NR;
   Ring C is a C6-C10 aryl ring, C3-C14 heteroaryl or heterocyclic ring wherein anywhere from 1 to 4 ring atoms are independently N, O, or S, or a C3-C10 cycloalkyl ring;
   X is O or NR;
   Y is CRR, CO, O, S, SO, SO₂, S(O)NH or NR;
   R₁ is halo; CN; F₅S; SiR3; OH; NRR; C1-C6 alkyl or fluoroalkyl; C1-C6 alkoxy or fluoroalkoxy; C1-C6 alkenyl; C1-C6 alkynyl; (C1-C9 alkylene)-R4 wherein up to four CH2 units are independently replaced with O, CO, S, SO, SO₂ or NR; C6-C10 aryl; C3-C10 heteroaryl or heterocyclic ring wherein anywhere from 1 to 4 ring atoms are independently O, S, N, or NR; or C3-C10 cycloalkyl;
   R2 is halo; OH; NRR; azide; CN; CO₂R; C1-C6 alkyl or fluoroalkyl;C1-C6 alkoxy or fluoroalkoxy; C1-C6 alkenyl; C1-C6 alkynyl; C6-C10 aryl; C3-C13 heteroaryl or heterocyclic ring wherein anywhere from 1 to 4 ring atoms are independently O, S, N, or NR; C3-C10 cycloalkyl; or a (C1-C9 alkylene)-R₄ wherein up to four CH2 units are independently replaced with O, CO, S, SO, SO₂ or NR;
      or two R2 groups taken together may form a =CH₂ or =O group;
   R3 is halo; CN; CO₂R; C1-C6 alkyl or fluoroalkyl; C1-C6 alkenyl; C1-C6 alkynyl; C1-C6 alkoxy or fluoroalkoxy; or C6-C10 aryl; C3-C10 heteroaryl or heterocyclic ring wherein anywhere from 1 to 4 ring atoms are independently O, S, N, or NR; C3-C10 cycloalkyl; or a (C1-C9 alkylene)-R4 wherein up to four CH2 units are independently replaced with O, CO, S, SO, SO₂ or NR;
      or two R3 groups taken together may form a =CH₂ or =O group;
   R4 is H; azide; CF₃; CHF₂; OR; CCH; CO₂R; OH; C6-C10 aryl, C3-C10 heteroaryl or heterocycloalkyl wherein anywhere from 1 to 4 ring atoms are independently O, S, N, or NR; C3-C10 cycloalkyl; NRR, NRCOR, CONRR, CN, halo, or SO₂R;
   R is independently H; OH; CO₂H; CO₂C1-C6 alkyl; C1-C6 alkyl; C1-C6 alkenyl; C1-C6 alkynyl; C6-C10 aryl; C3-C10 heteroaryl or heterocycloalkyl wherein anywhere from 1 to 4 ring atoms are independently O, S, N, or NR; or C3-C10 cycloalkyl;
   n is 0, 1, or 2;
   o is 0, 1, 2, 3, 4, or 5;
   p is 0, 1, 2, or 3; and
   q is 0, 1, 2, 3, 4, or 5.
31. The compound of aspect 30, wherein ring B is phenyl, pyridyl, pyridine-2(1*H*)-one, pyrazole, indole, thiophene, dihydrobenzofuran, pyrazine, indazole, thiazole, pyridine-4(1H)-one, pyrrolidinone, or quinoline.
32. The compound of aspect 30 or 31, wherein ring B is selected from 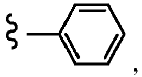 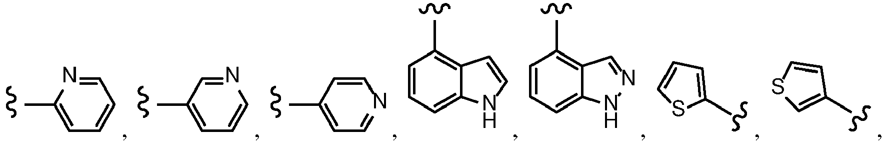 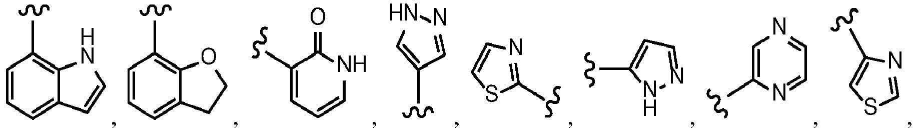 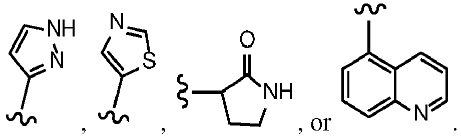
33. The compound of any one of aspects 30 to 32, wherein ring C is phenyl, indole, cycloalkyl, pyridyl, pyrrolidine, naphthalene, piperidine, or dihydroindene.
34. The compound of any one of aspects 30 to 33, wherein ring C is 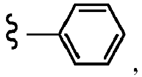 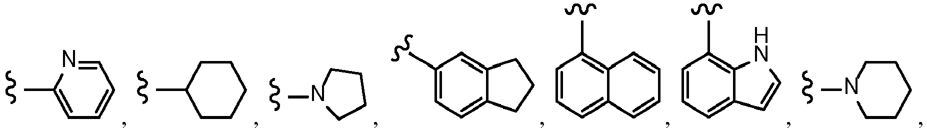 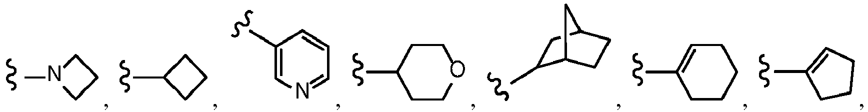 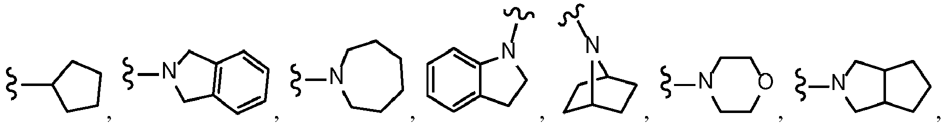 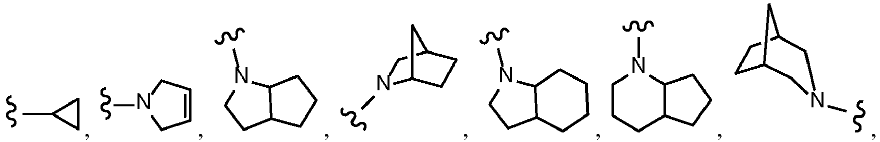 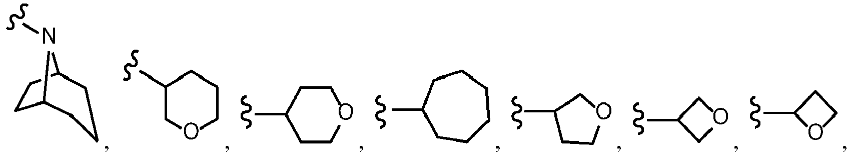 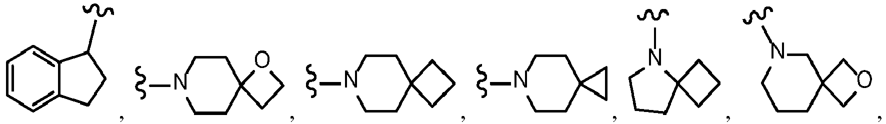 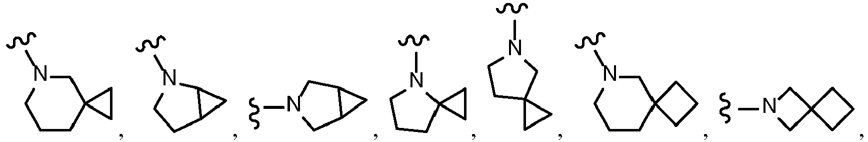 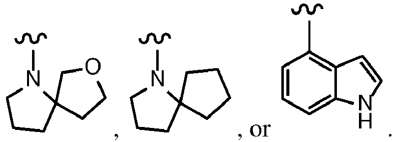
35. The compound of any one of aspects 30 to 34, wherein Y is O.
36. The compound of any one of aspects 30 to 34, wherein Y is CH2.
37. The compound of any one of aspects 30 to 34, wherein Y is CH(C1-C6 aliphatic).
38. The compound of any one of aspects 30 to 34, wherein Y is CH(CH3).
39. The compound of any one of aspects 30 to 34, wherein Y is CH(CH2CH3).
40. The compound of any one of aspects 30 to 39, wherein R₁ is halo, CN, C1-C6 aliphatic, C1-C6 alkoxy, C3-C8 cycloalkyl, or a phenyl, pyridyl, pyrimidine, indole, aza-indole, or thiophene ring, wherein all rings may be substituted with halo, C1-C6 aliphatic, C1-C6 alkoxy, C1-C6 fluoroaliphatic, C1-C6 fluoroalkoxy, OH, CH₂OH, CH₂OCH₃, CN, CO₂H, amino, amido, C3-C10 heteroaryl, C3-C10 heterocycloalkyl, or a (C1-C8 aliphatic)-R₄ wherein up to three CH2 units may be replaced with O, CO, S, SO, SO₂ or NR.
41. The compound of any one of aspects 30 to 40, wherein R₁ is selected from CH3, Cl, F, CN, OCH₃, CF₃, CH₂CH₃, tBu, CH(CH₃)₂, 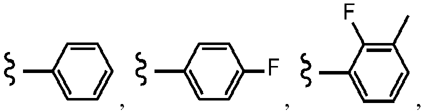 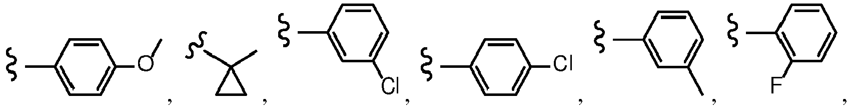 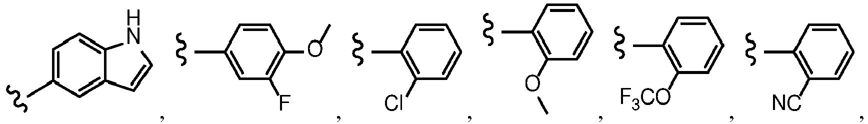 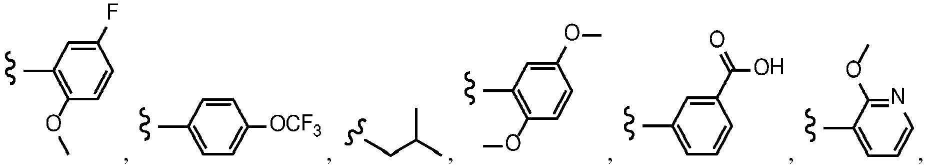 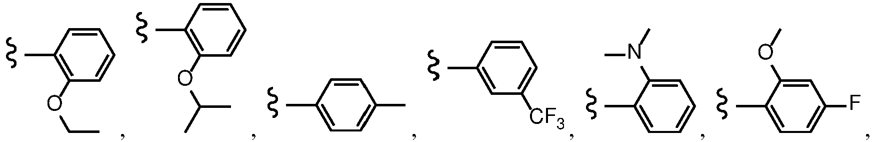 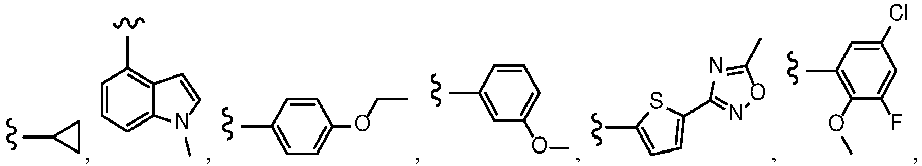 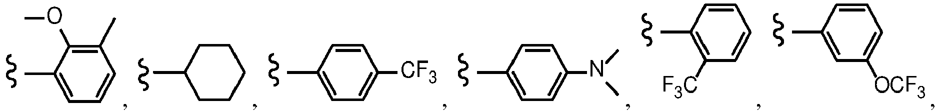 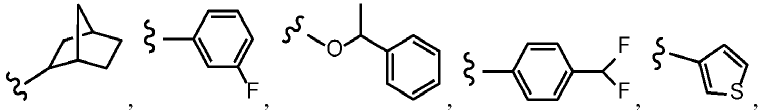 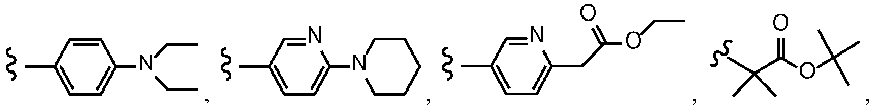 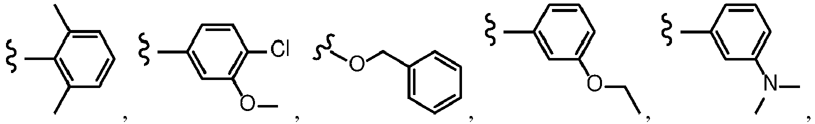 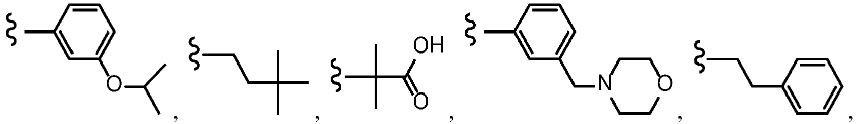 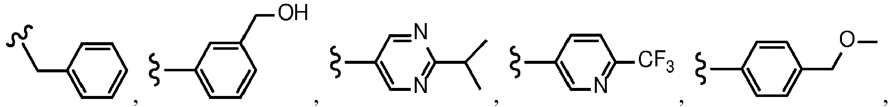 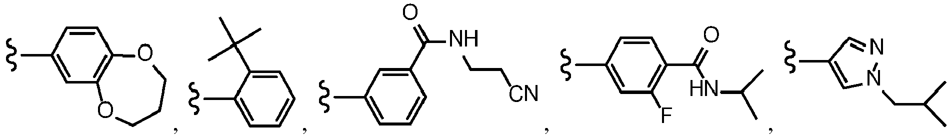 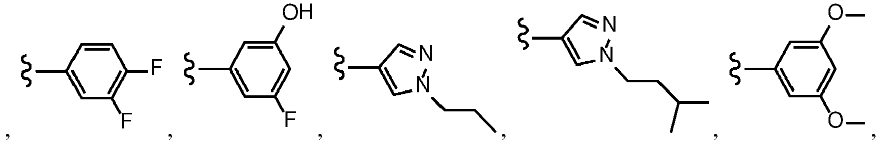 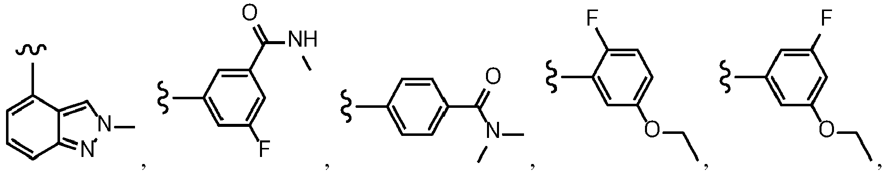 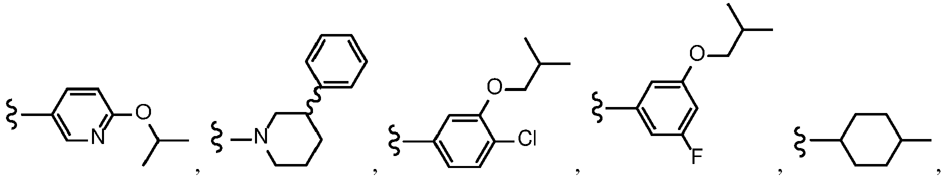 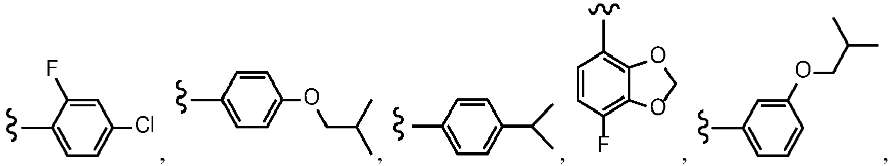 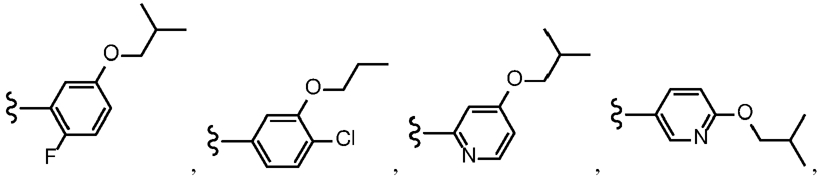 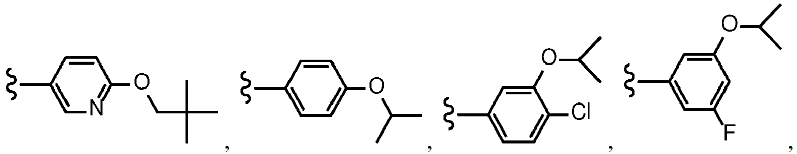 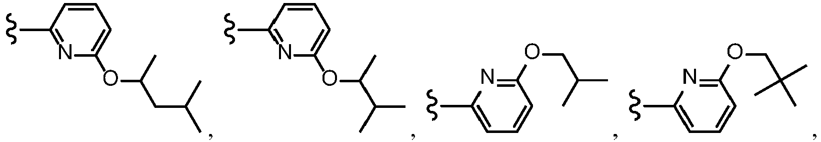 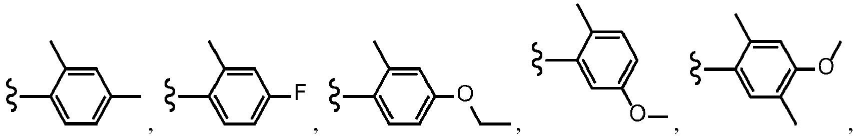 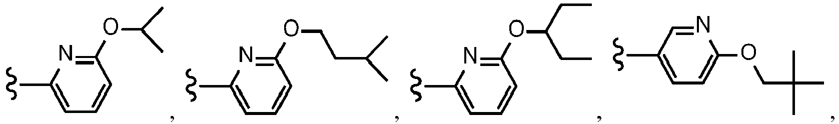 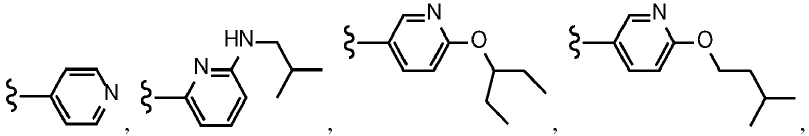 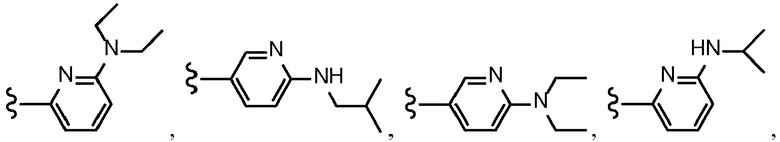 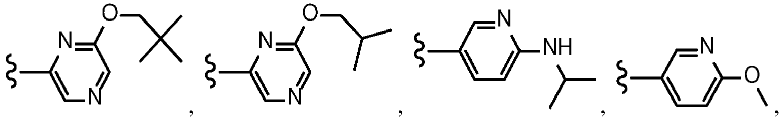 or 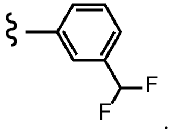
42. The compound of any one of aspects 30 to 41, wherein R2 is selected from halo, OH, CN, azide, amino, C1-C6 aliphatic or fluoroaliphatic, C1-C6 alkoxy or fluoroalkoxy, C3-C10 mono- or bicyclic heterocyclic ring wherein up to 4 carbon atoms may be replaced with O, S, N, or NR; or a (C1-C8 aliphatic)-R4 wherein up to three CH2 units may be replaced with O, CO, S, SO, SO₂ or NR.
43. The compound of any one of aspects 30 to 42, wherein R2 is selected from Cl, F, OH, CN, N₃, NH₂, NH(CH₃), N(CH₃)₂, CH₃, CH₂OH, CH₂CH₃, CH(CH₃)₂, CHF₂, OCH₃, OCF₃, OCHF₂, OCH(CH₃)₂, C(O)CH₃, CH₂CH₂OH, CH₂NH₂, NH(CH₂)₂OH, NH(CH₂)₂N(CH₃)₂, NH(CH₂)₂NH₂, NH(CH₂)₃NH₂, NH(CH₂)₂OCH₃, NHCH(CH₃)₂, 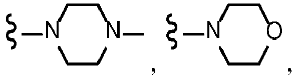 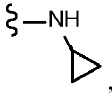 , or CO₂H.
44. The compound of any one of aspects 30 to 43, wherein R3 is selected from halo, CN, C1-C6 aliphatic or fluoroaliphatic, C1-C6 alkoxy, or C3-C10 mono- or bicyclic heteroaryl wherein up to 4 carbon atoms may be replaced by O, S, N, or NR.
45. The compound of any one of aspects 30 to 44, wherein R3 is selected from Cl, F, CN, CH₃, OCH₃, CF₃, CH₂CH₃, CH₂CF₃, CH₂CH₂CH₃, OCH₂CH₃, CH₂OCH₃, CH(CH₃)₂, CCH, CO₂CH₃, tBu, =CH₂, =O, 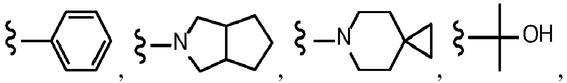 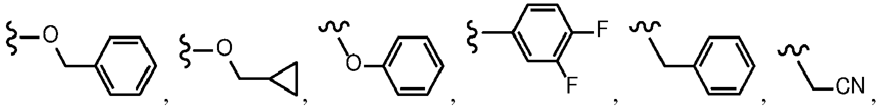 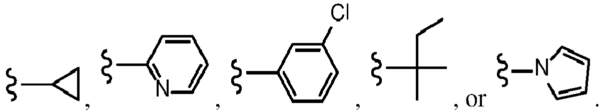
46. The compound of any one of aspects 30 to 45, wherein o is 0.
47. The compound of any one of aspects 30 to 45, wherein o is 1.
48. The compound of any one of aspects 30 to 47, wherein n is 0.
49. The compound of any one of aspects 30 to 47, wherein n is 1.
50. The compound of any one of aspects 30 to 47, wherein n is 2.
51. The compound of any one of aspects 30 to 50, wherein p is 0.
52. The compound of any one of aspects 30 to 50, wherein p is 1.
53. The compound of any one of aspects 30 to 50, wherein p is 2.
54. The compound of any one of aspects 30 to 53, wherein ring B is phenyl.
55. The compound of any one of aspects 30 to 54, wherein ring B and ring C are independently phenyl or pyridyl.
56. The compound of aspect 1, wherein the compound is of formula Ib: 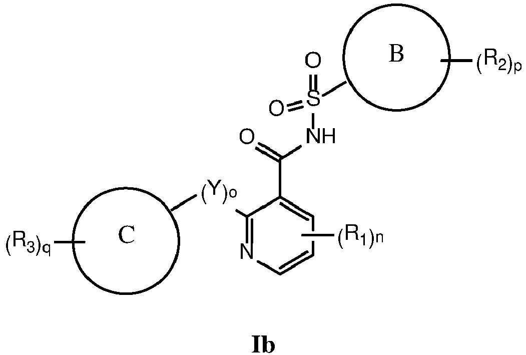 or a pharmaceutically acceptable salt thereof, wherein, independently for each occurrence:
   Ring B is a C6-C10 aryl ring or C3-C10 heteroaryl or heterocyclic ring wherein anywhere from 1 to 4 ring atoms are independently O, S, N, or NR;
   Ring C is a C6-C10 aryl ring, C3-C14 heteroaryl or heterocyclic ring wherein anywhere from 1 to 4 ring atoms are independently N, O, or S, or a C3-C10 mono- or bicyclic cycloalkyl ring;
   Y is CRR, CO, O, S, SO, SO₂, S(O)NH or NR;
   R₁ is halo; CN; F₅S; SiR3; OH; NRR; C1-C6 alkyl or fluoroalkyl; C1-C6 alkoxy or fluoroalkoxy; C1-C6 alkenyl; C1-C6 alkynyl; (C1-C9 alkylene)-R4 wherein up to four CH2 units are independently replaced with O, CO, S, SO, SO₂ or NR; C6-C10 aryl; C3-C10 heteroaryl or heterocyclic ring wherein anywhere from 1 to 4 ring atoms are independently O, S, N, or NR; or C3-C10 cycloalkyl;
   R2 is halo; OH; NRR; azide; CN; CO₂R; C1-C6 alkyl or fluoroalkyl;C1-C6 alkoxy or fluoroalkoxy; C1-C6 alkenyl; C1-C6 alkynyl; C6-C10 aryl; C3-C13 heteroaryl or heterocyclic ring wherein anywhere from 1 to 4 ring atoms are independently O, S, N, or NR; C3-C10 cycloalkyl; or a (C1-C9 alkylene)-R₄ wherein up to four CH2 units are independently replaced with O, CO, S, SO, SO₂ or NR;
      or two R2 groups taken together may form a =CH₂ or =O group;
   R3 is halo; CN; CO₂R; C1-C6 alkyl or fluoroalkyl; C1-C6 alkenyl; C1-C6 alkynyl; C1-C6 alkoxy or fluoroalkoxy; or C6-C10 aryl; C3-C10 heteroaryl or heterocyclic ring wherein anywhere from 1 to 4 ring atoms are independently O, S, N, or NR; C3-C10 cycloalkyl; or a (C1-C9 alkylene)-R4 wherein up to four CH2 units are independently replaced with O, CO, S, SO, SO₂ or NR;
      or two R3 groups taken together may form a =CH₂ or =O group;
   R4 is H; azide; CF₃; CHF₂; OR; CCH; CO₂R; OH; C6-C10 aryl, C3-C10 heteroaryl or heterocycloalkyl wherein anywhere from 1 to 4 ring atoms are independently O, S, N, or NR; C3-C10 cycloalkyl; NRR, NRCOR, CONRR, CN, halo, or SO₂R;
   R is independently H; OH; CO₂H; CO₂C1-C6 alkyl; C1-C6 alkyl; C1-C6 alkenyl; C1-C6 alkynyl; C6-C10 aryl; C3-C10 heteroaryl or heterocycloalkyl wherein anywhere from 1 to 4 ring atoms are independently O, S, N, or NR; or C3-C10 cycloalkyl;
   n is 0, 1, 2 or 3;
   o is 0, 1, 2, 3, 4, or 5;
   p is 0, 1, 2, or 3; and
   q is 0, 1, 2, 3, 4, or 5.
57. The compound of aspect 56, wherein ring B is phenyl, pyridyl, pyridine-2(1*H*)-one, pyrazole, indole, thiophene, dihydrobenzofuran, pyrazine, indazole, thiazole, pyridine-4(1H)-one, pyrrolidinone, or quinoline.
58. The compound of aspect 56 or 57, wherein ring B is selected from 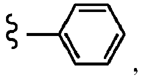 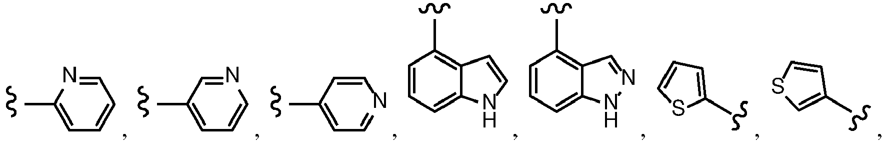 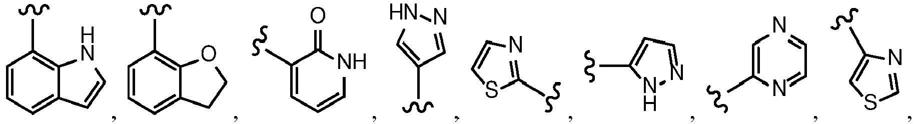 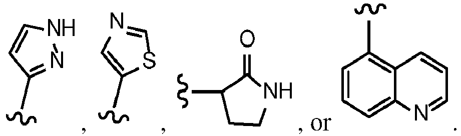
59. The compound of any one of aspects 56 to 58, wherein ring C is phenyl, indole, cycloalkyl, pyridyl, pyrrolidine, naphthalene, or dihydroindene.
60. The compound of any one of aspects 56 to 59, wherein ring C is 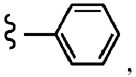 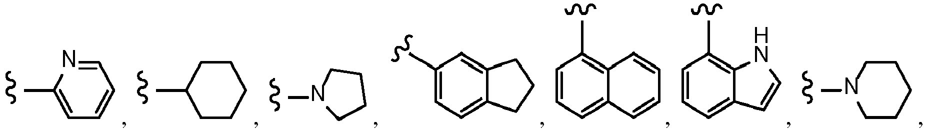 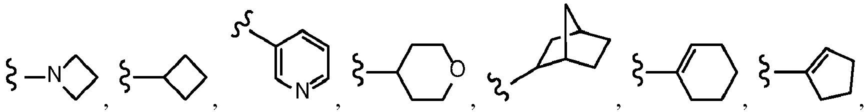 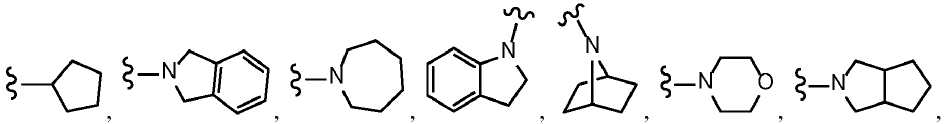 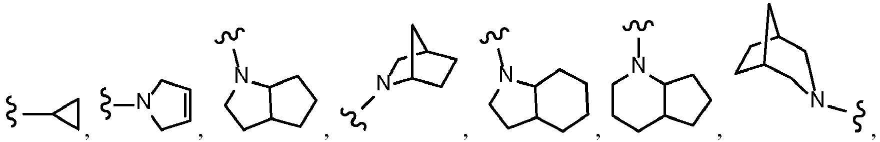 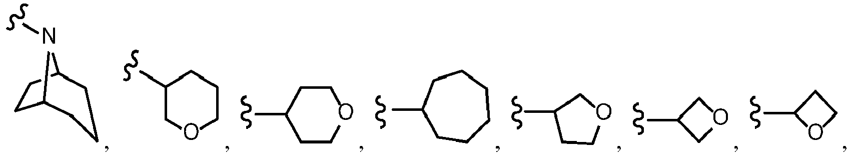 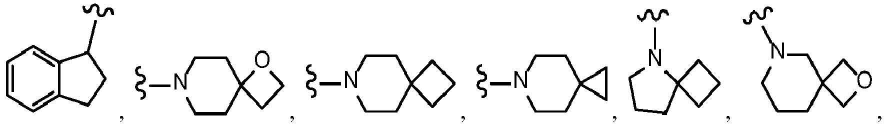 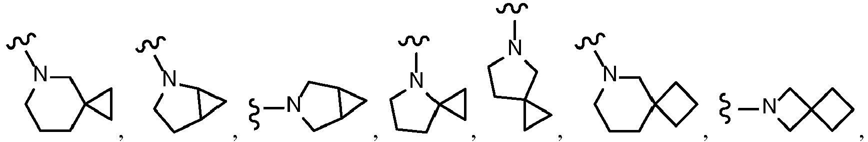 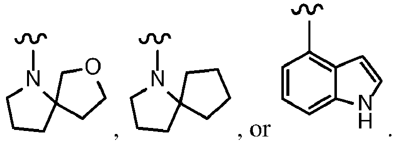
61. The compound of any one of aspects 56 to 60, wherein Y is O.
62. The compound of any one of aspects 56 to 60, wherein Y is CH2.
63. The compound of any one of aspects 56 to 60, wherein Y is CH(C1-C6 aliphatic).
64. The compound of any one of aspects 56 to 60, wherein Y is CH(CH3).
65. The compound of any one of aspects 56 to 60, wherein Y is CH(CH2CH3).
66. The compound of any one of aspects 56 to 65, wherein R₁ is halo, CN, C1-C6 aliphatic, C1-C6 alkoxy, C3-C8 cycloalkyl, or a phenyl, pyridyl, pyrimidine, indole, aza-indole, or thiophene ring, wherein all rings may be substituted with halo, C1-C6 aliphatic, C1-C6 alkoxy, C1-C6 fluoroaliphatic, C1-C6 fluoroalkoxy, OH, CH₂OH, CH₂OCH₃, CN, CO₂H, amino, amido, C3-C10 heteroaryl, C3-C10 heterocycloalkyl, or a (C1-C8 aliphatic)-R₄ wherein up to three CH2 units may be replaced with O, CO, S, SO, SO₂ or NR.
67. The compound of any one of aspects 56 to 66, wherein R₁ is selected from CH3, Cl, F, CN, OCH₃, CF₃, CH₂CH₃, tBu, CH(CH₃)₂, 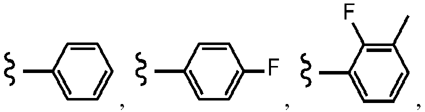 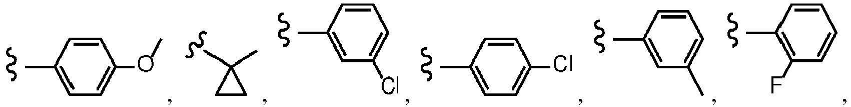 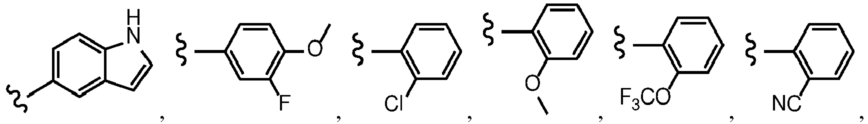 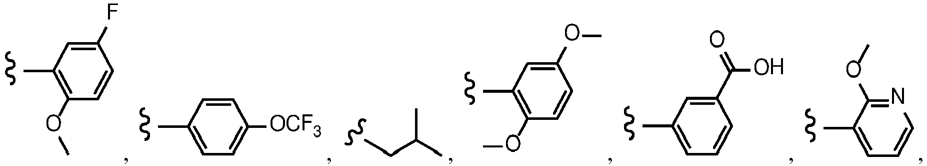 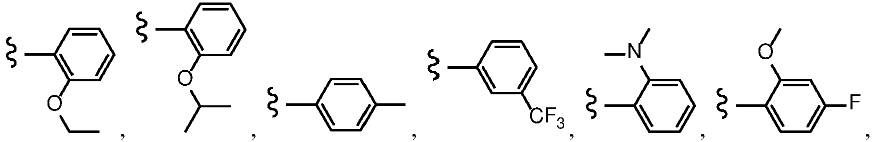 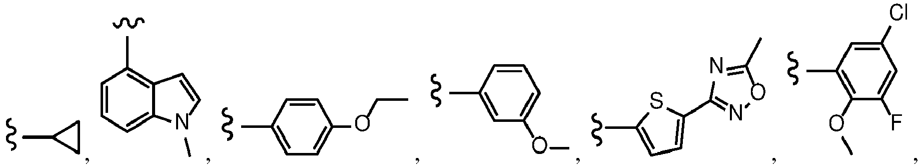 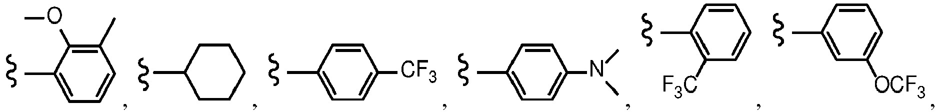 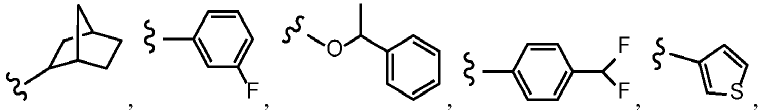 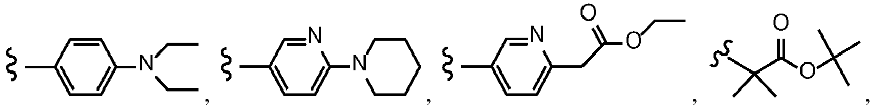 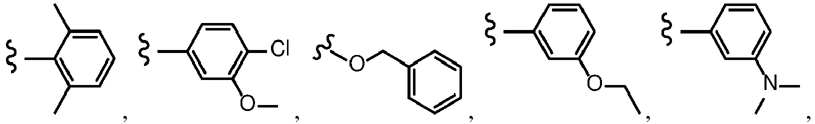 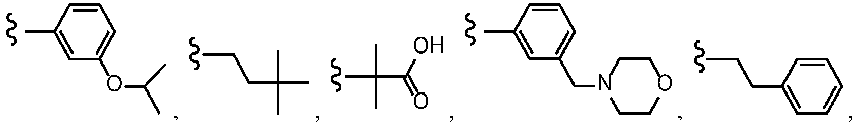 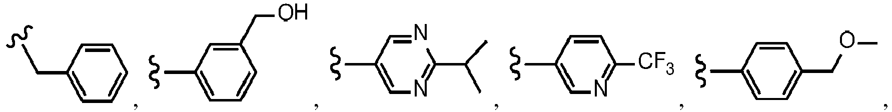 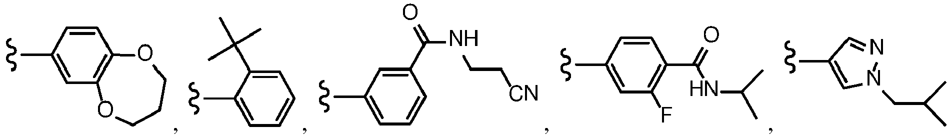 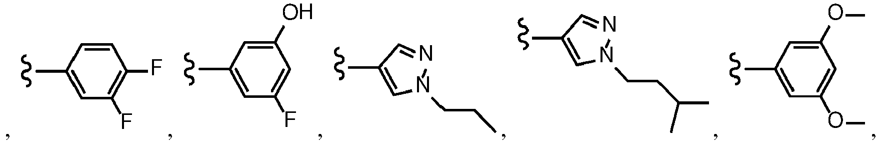 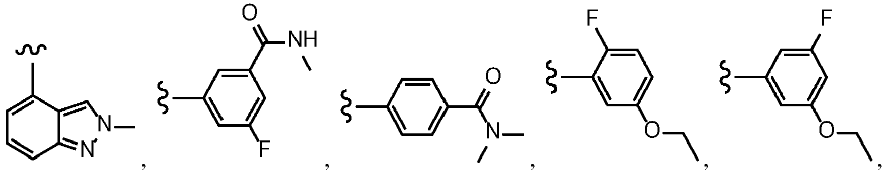 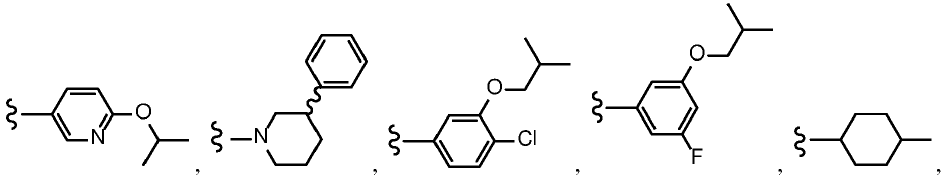 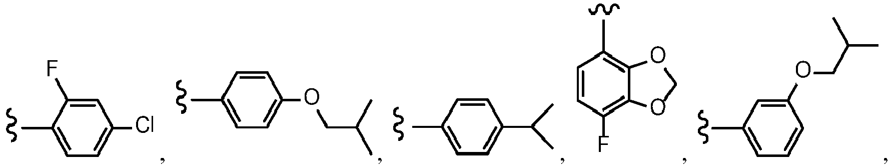 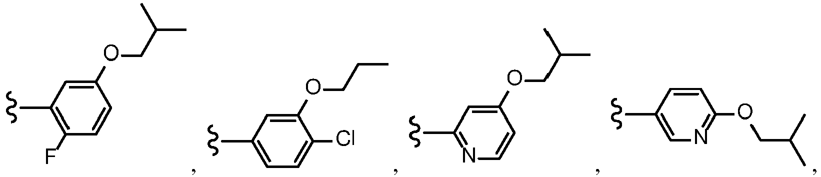 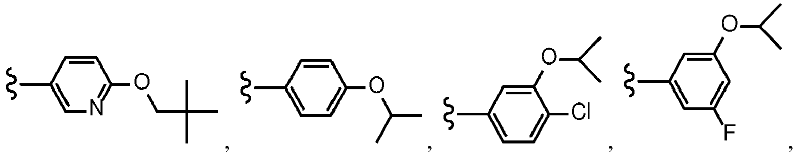 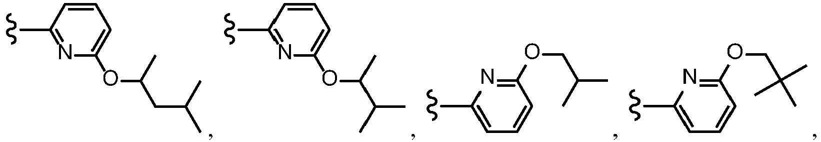 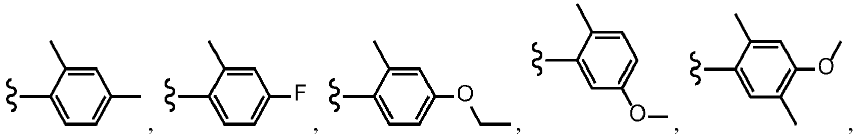 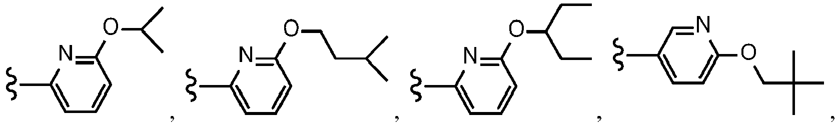 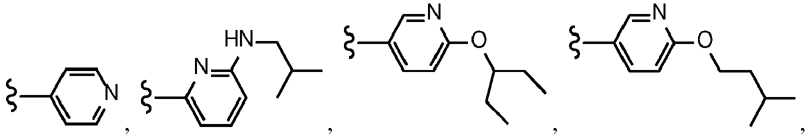 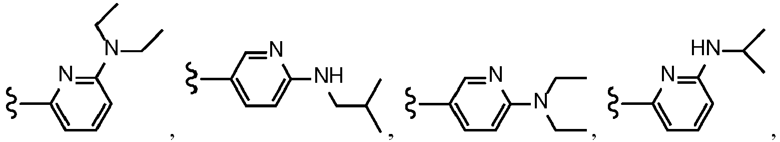 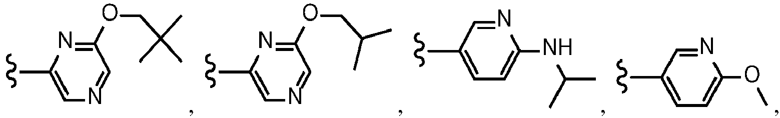 or 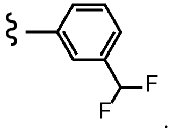
68. The compound of any one of aspects 56 to 67, wherein R2 is selected from halo, OH, CN, azide, amino, C1-C6 aliphatic or fluoroaliphatic, C1-C6 alkoxy or fluoroalkoxy, C3-C10 mono- or bicyclic heterocyclic ring wherein up to 4 carbon atoms may be replaced with O, S, N, or NR; or a (C1-C8 aliphatic)-R4 wherein up to three CH2 units may be replaced with O, CO, S, SO, SO₂ or NR.
69. The compound of any one of aspects 56 to 68, wherein R2 is selected from Cl, F, OH, CN, N₃, NH₂, NH(CH₃), N(CH₃)₂, CH₃, CH₂OH, CH₂CH₃, CH(CH₃)₂, CHF₂, OCH₃, OCF₃, OCHF₂, OCH(CH₃)₂, C(O)CH₃, CH₂CH₂OH, CH₂NH₂, NH(CH₂)₂OH, NH(CH₂)₂N(CH₃)₂, NH(CH₂)₂NH₂, NH(CH₂)₃NH₂, NH(CH₂)₂OCH₃, NHCH(CH₃)₂, 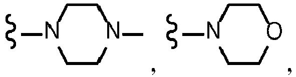 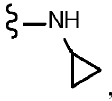 , or CO₂H.
70. The compound of any one of aspects 56 to 69, wherein R3 is selected from halo, CN, C1-C6 aliphatic or fluoroaliphatic, C1-C6 alkoxy, or C3-C10 mono- or bicyclic heteroaryl wherein up to 4 carbon atoms may be replaced by O, S, N, or NR.
71. The compound of any one of aspects 56 to 70, wherein R3 is selected from Cl, F, CN, CH₃, OCH₃, CF₃, CH₂CH₃, CH₂CF₃, CH₂CH₂CH₃, OCH₂CH₃, CH₂OCH₃, CH(CH₃)₂, CCH, CO₂CH₃, tBu, =CH₂, =O, 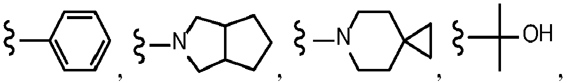 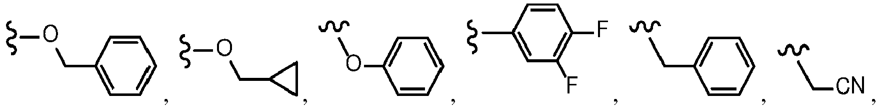 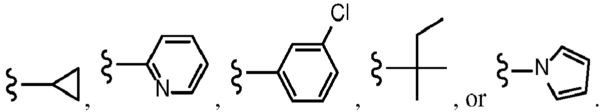
72. The compound of any one of aspects 56 to 71, wherein o is 0.
73. The compound of any one of aspects 56 to 71, wherein o is 1.
74. The compound of any one of aspects 56 to 71, wherein o is 2.
75. The compound of any one of aspects 56 to 74, wherein n is 0.
76. The compound of any one of aspects 56 to 74, wherein n is 1.
77. The compound of any one of aspects 56 to 74, wherein n is 2.
78. The compound of any one of aspects 56 to 77, wherein p is 0.
79. The compound of any one of aspects 56 to 77, wherein p is 1.
80. The compound of any one of aspects 56 to 77, wherein p is 2.
81. The compound of any one of aspects 56 to 80, wherein ring B is pyridyl.
82. The compound of any one of aspects 56 to 81, wherein ring B is phenyl.
83. The compound of any one of aspects 56 to 82, wherein ring B and ring C are phenyl.
84. The compound of aspect 1, wherein the compound is of formula Ic: 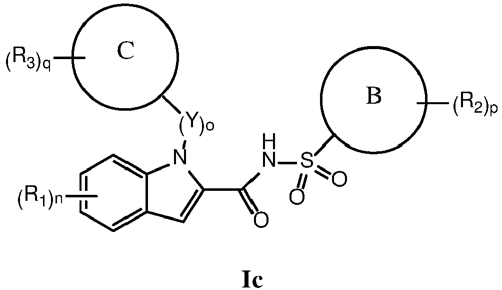 or a pharmaceutically acceptable salt thereof, wherein, independently for each occurrence:
   Ring B is a C6-C10 aryl ring or C3-C10 heteroaryl or heterocyclic ring wherein anywhere from 1 to 4 ring atoms are independently O, S, N, or NR;
   Ring C is a C6-C10 aryl ring, C3-C14heteroaryl or heterocyclic ring wherein anywhere from 1 to 4 ring atoms are independently N, O, or S, or a C3-C10 cycloalkyl ring;
   Y is CRR, CO, O, S, SO, SO₂, S(O)NH or NR;
   R₁ is halo; CN; F₅S; SiR3; OH; NRR; C1-C6 alkyl or fluoroalkyl; C1-C6 alkoxy or fluoroalkoxy; C1-C6 alkenyl; C1-C6 alkynyl; (C1-C9 alkylene)-R4 wherein up to four CH2 units are independently replaced with O, CO, S, SO, SO₂ or NR; C6-C10 aryl; C3-C10 heteroaryl or heterocyclic ring wherein anywhere from 1 to 4 ring atoms are independently O, S, N, or NR; or C3-C10 cycloalkyl;
   R2 is halo; OH; NRR; azide; CN; CO₂R; C1-C6 alkyl or fluoroalkyl;C1-C6 alkoxy or fluoroalkoxy; C1-C6 alkenyl; C1-C6 alkynyl; C6-C10 aryl; C3-C13 heteroaryl or heterocyclic ring wherein anywhere from 1 to 4 ring atoms are independently O, S, N, or NR; C3-C10 cycloalkyl; or a (C1-C9 alkylene)-R₄ wherein up to four CH2 units are independently replaced with O, CO, S, SO, SO₂ or NR;
      or two R2 groups taken together may form a =CH₂ or =O group;
   R3 is halo; CN; CO₂R; C1-C6 alkyl or fluoroalkyl; C1-C6 alkenyl; C1-C6 alkynyl; C1-C6 alkoxy or fluoroalkoxy; or C6-C10 aryl; C3-C10 heteroaryl or heterocyclic ring wherein anywhere from 1 to 4 ring atoms are independently O, S, N, or NR; C3-C10 cycloalkyl; or a (C1-C9 alkylene)-R4 wherein up to four CH2 units are independently replaced with O, CO, S, SO, SO₂ or NR;
      or two R3 groups taken together may form a =CH₂ or =O group;
   R4 is H; azide; CF₃; CHF₂; OR; CCH; CO₂R; OH; C6-C10 aryl, C3-C10 heteroaryl or heterocycloalkyl wherein anywhere from 1 to 4 ring atoms are independently O, S, N, or NR; C3-C10 cycloalkyl; NRR, NRCOR, CONRR, CN, halo, or SO₂R;
   R is independently H; OH; CO₂H; CO₂C1-C6 alkyl; C1-C6 alkyl; C1-C6 alkenyl; C1-C6 alkynyl; C6-C10 aryl; C3-C10 heteroaryl or heterocycloalkyl wherein anywhere from 1 to 4 ring atoms are independently O, S, N, or NR; or C3-C10 cycloalkyl;
   n is 0, 1, 2 or 3;
   o is 0, 1, 2, 3, 4, or 5;
   p is 0, 1, 2, or 3; and
   q is 0, 1, 2, 3, 4, or 5.
85. The compound of aspect 84, wherein ring B is phenyl, pyridyl, pyridine-2(1*H*)-one, pyrazole, indole, thiophene, dihydrobenzofuran, pyrazine, indazole, thiazole, pyridine-4(1H)-one, pyrrolidinone, or quinoline.
86. The compound of aspect 84 or 85, wherein ring B is selected from 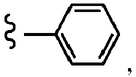 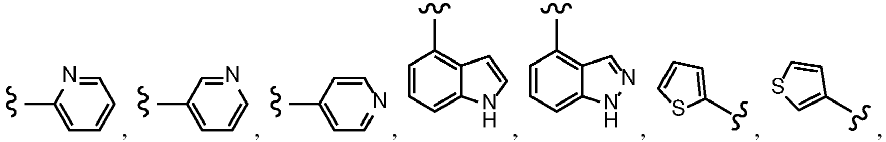 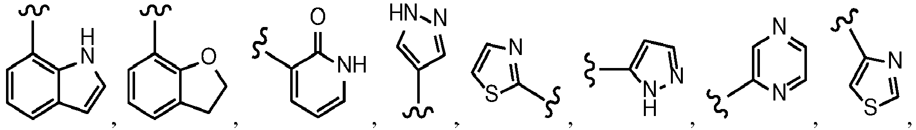 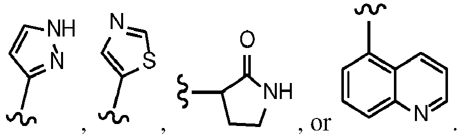
87. The compound of any one of aspects 84 to 86, wherein ring C is phenyl, indole, cycloalkyl, pyridyl, pyrrolidine, naphthalene, or dihydroindene.
88. The compound of any one of aspects 84 to 87, wherein ring C is 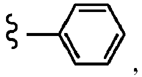 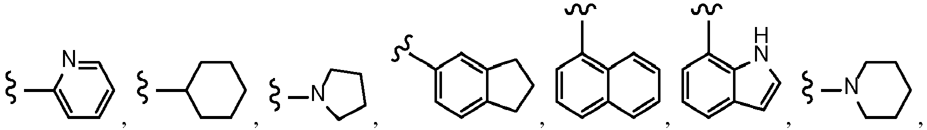 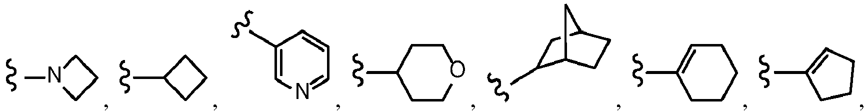 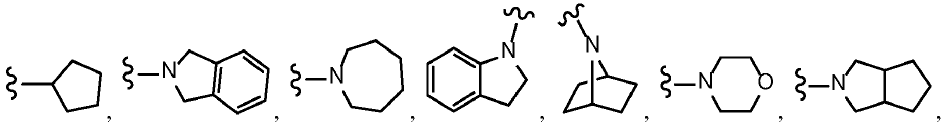 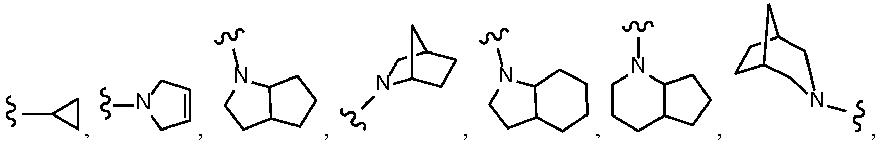 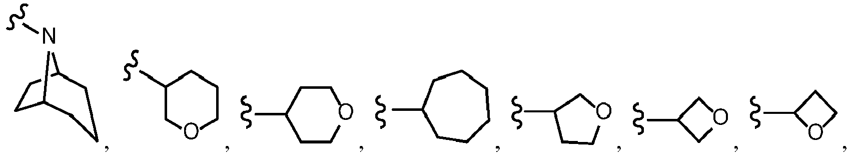 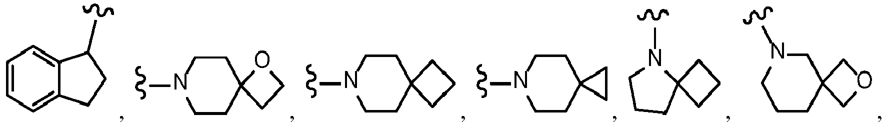 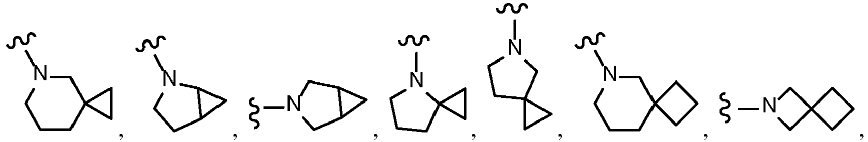 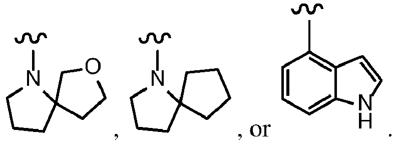
89. The compound of any one of aspects 84 to 88, wherein Y is O.
90. The compound of any one of aspects 84 to 88, wherein Y is CH2.
91. The compound of any one of aspects 84 to 88, wherein Y is CH(C1-C6 aliphatic).
92. The compound of any one of aspects 84 to 88, wherein Y is CH(CH3).
93. The compound of any one of aspects 84 to 88, wherein Y is CH(CH2CH3).
94. The compound of any one of aspects 84 to 93, wherein R₁ is halo, CN, C1-C6 aliphatic, C1-C6 alkoxy, C3-C8 cycloalkyl, or a phenyl, pyridyl, pyrimidine, indole, aza-indole, or thiophene ring, wherein all rings may be substituted with halo, C1-C6 aliphatic, C1-C6 alkoxy, C1-C6 fluoroaliphatic, C1-C6 fluoroalkoxy, OH, CH₂OH, CH₂OCH₃, CN, CO₂H, amino, amido, C3-C10 heteroaryl, C3-C10 heterocycloalkyl, or a (C1-C8 aliphatic)-R₄ wherein up to three CH2 units may be replaced with O, CO, S, SO, SO₂ or NR.
95. The compound of any one of aspects 84 to 94, wherein R₁ is selected from CH3, Cl, F, CN, OCH₃, CF₃, CH₂CH₃, tBu, CH(CH₃)₂, 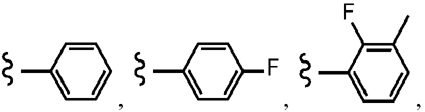 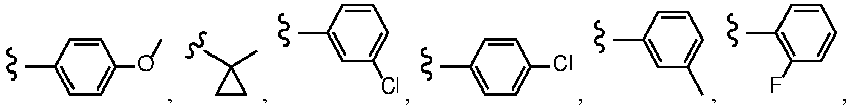 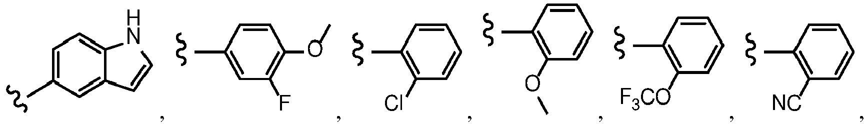 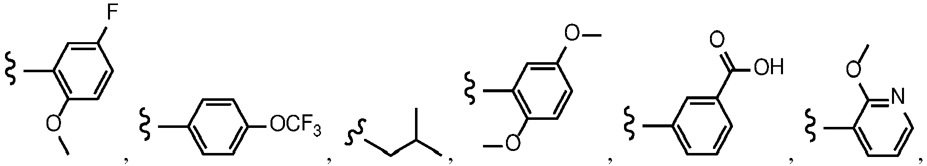 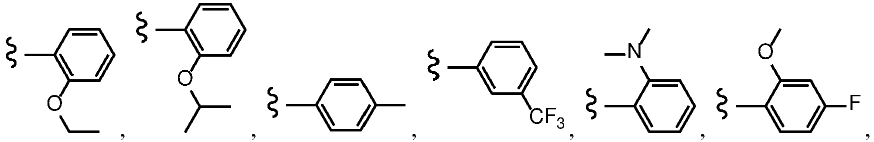 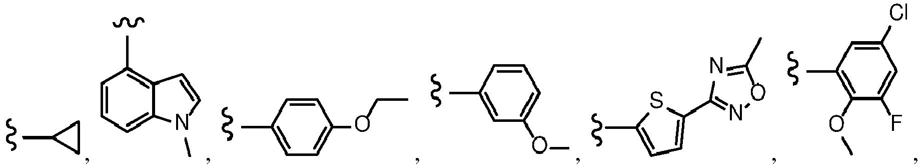 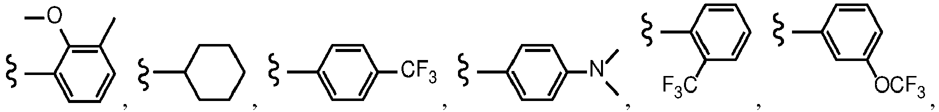 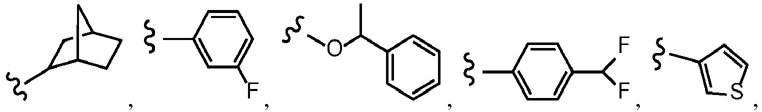 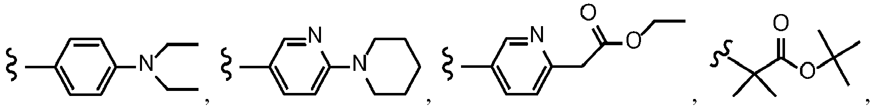 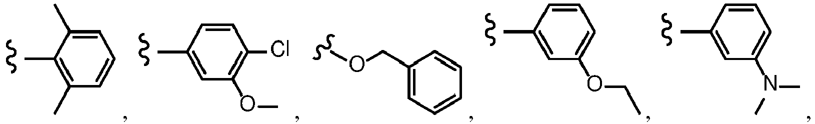 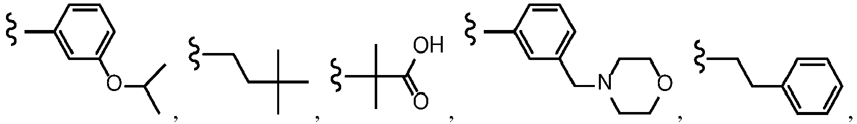 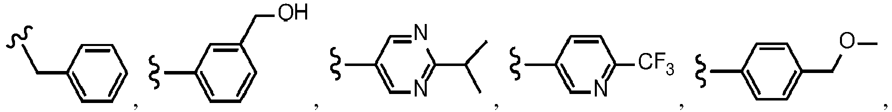 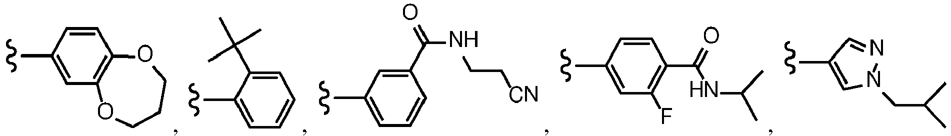 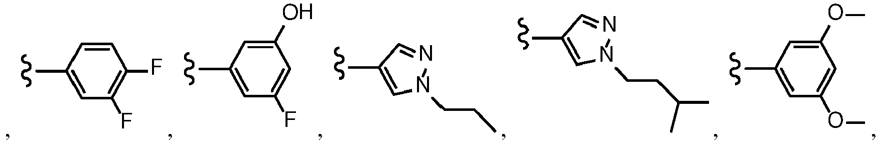 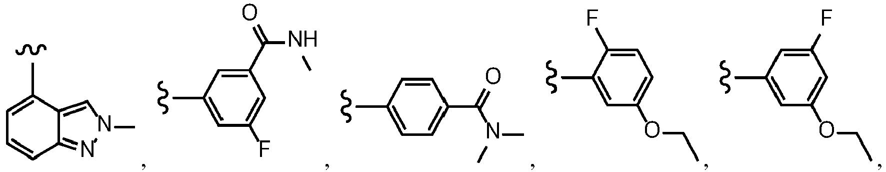 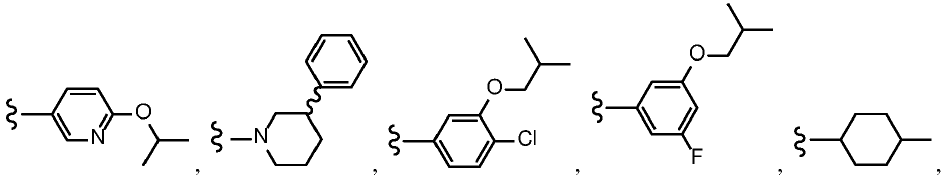 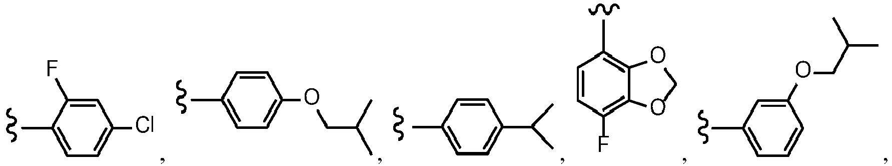 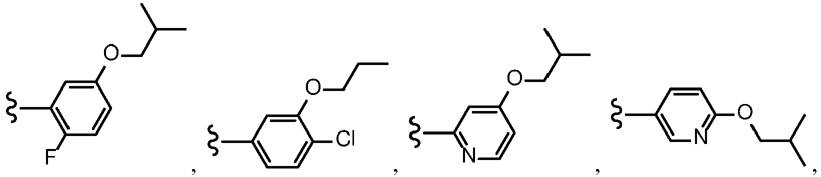 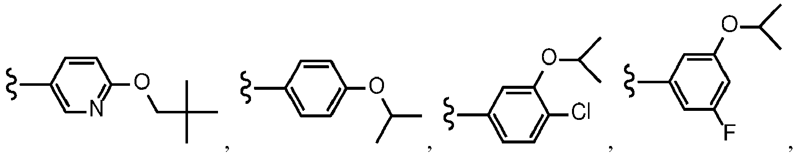 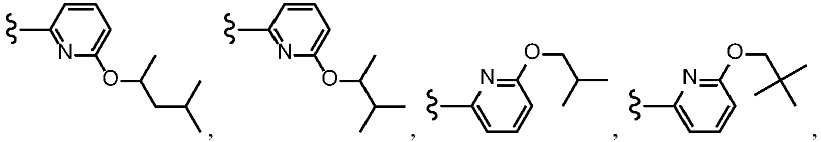 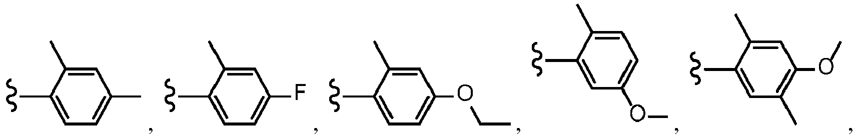 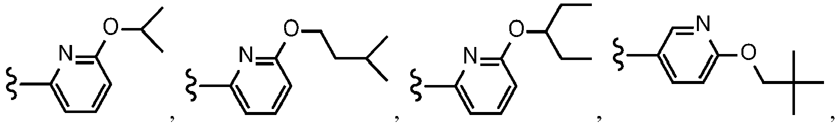 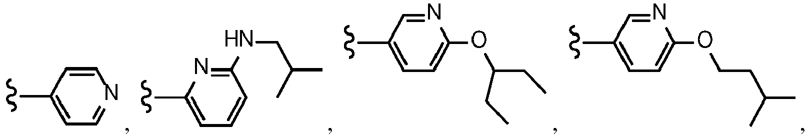 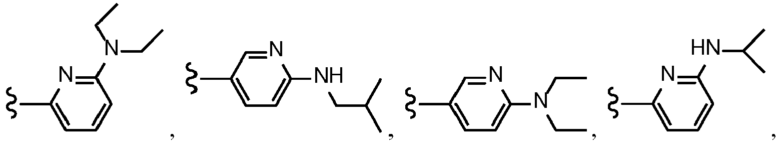 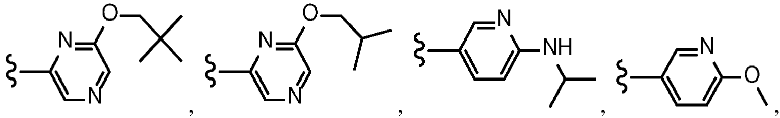 or 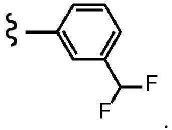
96. The compound of any one of aspects 84 to 95, wherein R2 is selected from halo, OH, CN, azide, amino, C1-C6 aliphatic or fluoroaliphatic, C1-C6 alkoxy or fluoroalkoxy, C3-C10 mono- or bicyclic heterocyclic ring wherein up to 4 carbon atoms may be replaced with O, S, N, or NR; or a (C1-C8 aliphatic)-R4 wherein up to three CH2 units may be replaced with O, CO, S, SO, SO₂ or NR.
97. The compound of any one of aspects 84 to 96, wherein R2 is selected from Cl, F, OH, CN, N₃, NH₂, NH(CH₃), N(CH₃)₂, CH₃, CH₂OH, CH₂CH₃, CH(CH₃)₂, CHF₂, OCH₃, OCF₃, OCHF₂, OCH(CH₃)₂, C(O)CH₃, CH₂CH₂OH, CH₂NH₂, NH(CH₂)₂OH, NH(CH₂)₂N(CH₃)₂, NH(CH₂)₂NH₂, NH(CH₂)₃NH₂, NH(CH₂)₂OCH₃, NHCH(CH₃)₂, 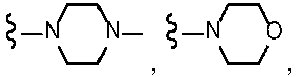 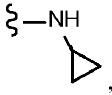 , or CO₂H.
98. The compound of any one of aspects 84 to 97, wherein R3 is selected from halo, CN, C1-C6 aliphatic or fluoroaliphatic, C1-C6 alkoxy, or C3-C10 mono- or bicyclic heteroaryl wherein up to 4 carbon atoms may be replaced by O, S, N, or NR.
99. The compound of any one of aspects 84 to 98, wherein R3 is selected from Cl, F, CN, CH₃, OCH₃, CF₃, CH₂CH₃, CH₂CF₃, CH₂CH₂CH₃, OCH₂CH₃, CH₂OCH₃, CH(CH₃)₂, CCH, CO₂CH₃, tBu, =CH₂, =O, 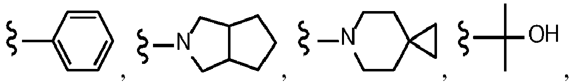 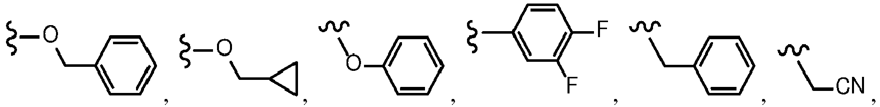 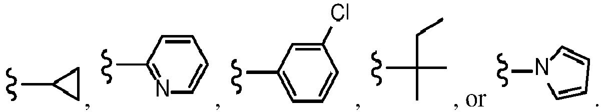
100. The compound of any one of aspects 84 to 99, wherein o is 0.
101. The compound of any one of aspects 84 to 99, wherein o is 1.
102. The compound of any one of aspects 84 to 99, wherein o is 2.
103. The compound of any one of aspects 84 to 102, wherein n is 0.
104. The compound of any one of aspects 84 to 102, wherein n is 1.
105. The compound of any one of aspects 84 to 102, wherein n is 2.
106. The compound of any one of aspects 84 to 105, wherein p is 0.
107. The compound of any one of aspects 84 to 105, wherein p is 1.
108. The compound of any one of aspects 84 to 105, wherein p is 2.
109. The compound of any one of aspects 84 to 108, wherein ring B is phenyl.
110. The compound of any one of aspects 84 to 109, wherein ring C is phenyl or pyridyl.
111. The compound of any one of aspects 84 to 110, wherein ring B and ring C are phenyl.
112. The compound of aspect 1, wherein the compound is of formula Id: 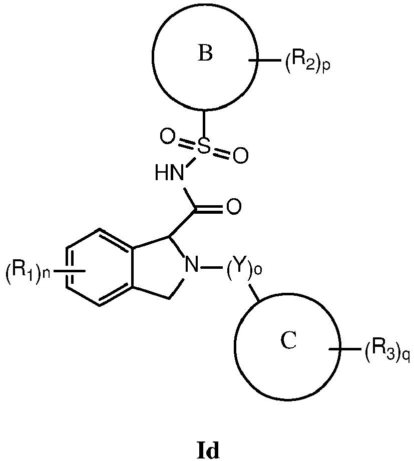 or a pharmaceutically acceptable salt thereof, wherein, independently for each occurrence:
   Ring B is a C6-C10 aryl ring or C3-C10 heteroaryl or heterocyclic ring wherein anywhere from 1 to 4 ring atoms are independently O, S, N, or NR;
   Ring C is a C6-C10 aryl ring, C3-C14 heteroaryl or heterocyclic ring wherein anywhere from 1 to 4 ring atoms are independently N, O, or S, or a C3-C10 cycloalkyl ring;
   Y is CRR, CO, O, S, SO, SO₂, S(O)NH or NR;
   R₁ is halo; CN; F₅S; SiR3; OH; NRR; C1-C6 alkyl or fluoroalkyl; C1-C6 alkoxy or fluoroalkoxy; C1-C6 alkenyl; C1-C6 alkynyl; (C1-C9 alkylene)-R4 wherein up to four CH2 units are independently replaced with O, CO, S, SO, SO₂ or NR; C6-C10 aryl; C3-C10 heteroaryl or heterocyclic ring wherein anywhere from 1 to 4 ring atoms are independently O, S, N, or NR; or C3-C10 cycloalkyl;
   R2 is halo; OH; NRR; azide; CN; CO₂R; C1-C6 alkyl or fluoroalkyl;C1-C6 alkoxy or fluoroalkoxy;C1-C6 alkenyl; C1-C6 alkynyl; C6-C10 aryl; C3-C13 heteroaryl or heterocyclic ring wherein anywhere from 1 to 4 ring atoms are independently O, S, N, or NR; C3-C10 cycloalkyl; or a (C1-C9 alkylene)-R₄ wherein up to four CH2 units are independently replaced with O, CO, S, SO, SO₂ or NR;
      or two R2 groups taken together may form a =CH₂ or =O group;
   R3 is halo; CN; CO₂R; C1-C6 alkyl or fluoroalkyl; C1-C6 alkenyl; C1-C6 alkynyl; C1-C6 alkoxy or fluoroalkoxy; or C6-C10 aryl; C3-C10 heteroaryl or heterocyclic ring wherein anywhere from 1 to 4 ring atoms are independently O, S, N, or NR; C3-C10 cycloalkyl; or a (C1-C9 alkylene)-R4 wherein up to four CH2 units are independently replaced with O, CO, S, SO, SO₂ or NR;
      or two R3 groups taken together may form a =CH₂ or =O group;
   R4 is H; azide; CF₃; CHF₂; OR; CCH; CO₂R; OH; C6-C10 aryl, C3-C10 heteroaryl or heterocycloalkyl wherein anywhere from 1 to 4 ring atoms are independently O, S, N, or NR; C3-C10 cycloalkyl; NRR, NRCOR, CONRR, CN, halo, or SO₂R;
   R is independently H; OH; CO₂H; CO₂C1-C6 alkyl; C1-C6 alkyl; C1-C6 alkenyl; C1-C6 alkynyl; C6-C10 aryl; C3-C10 heteroaryl or heterocycloalkyl wherein anywhere from 1 to 4 ring atoms are independently O, S, N, or NR; or C3-C10 cycloalkyl;
   n is 0, 1, 2 or 3;
   o is 0, 1, 2, 3, 4, or 5;
   p is 0, 1, 2, or 3; and
   q is 0, 1, 2, 3, 4, or 5.
113. The compound of aspect 112, wherein ring B is phenyl, pyridyl, pyridine-2(1*H*)-one, pyrazole, indole, thiophene, dihydrobenzofuran, pyrazine, indazole, thiazole, pyridine-4(1H)-one, pyrrolidinone, or quinoline.
114. The compound of aspect 112 or 113, wherein ring B is selected from 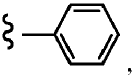 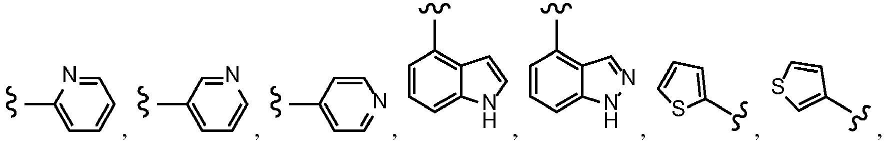 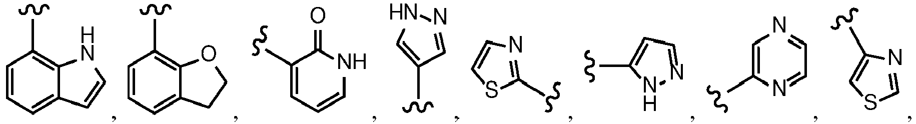 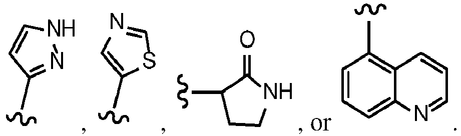
115. The compound of any one of aspects 112 to 114, wherein ring C is phenyl, indole, cycloalkyl, pyridyl, pyrrolidine, naphthalene, or dihydroindene.
116. The compound of any one of aspects 112 to 115, wherein ring C is 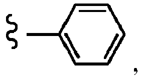 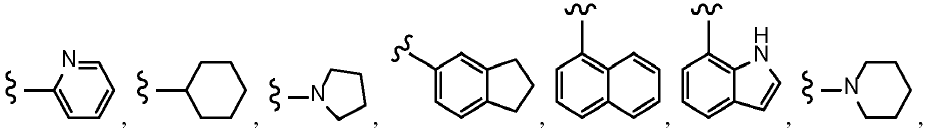 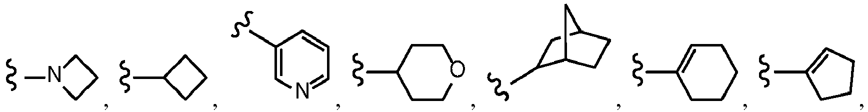 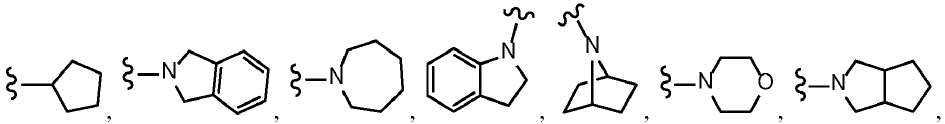 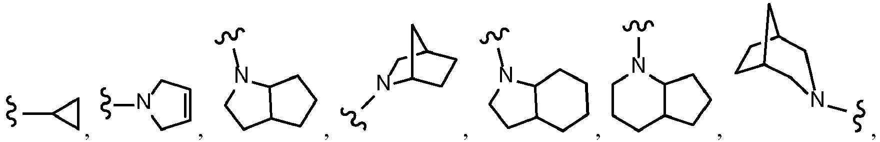 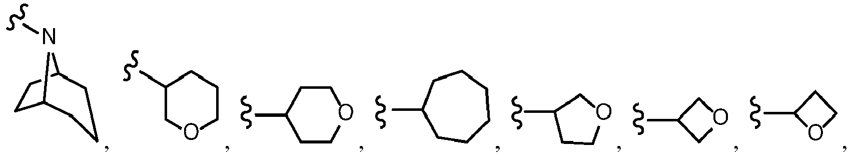 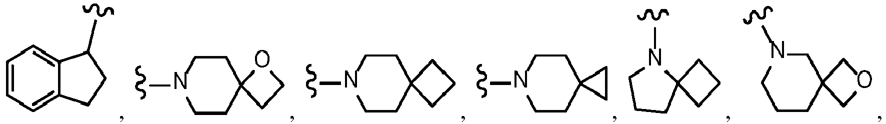 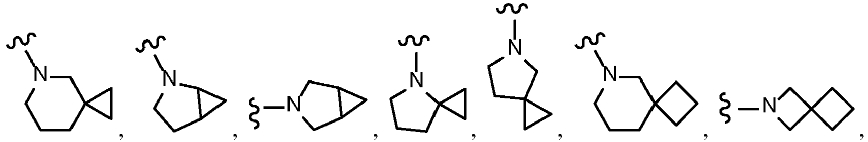 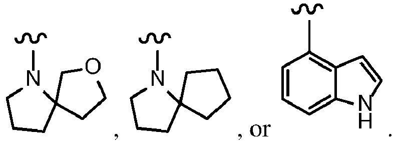
117. The compound of any one of aspects 112 to 116, wherein Y is O.
118. The compound of any one of aspects 112 to 116, wherein Y is CH2.
119. The compound of any one of aspects 112 to 116, wherein Y is CH(C1-C6 aliphatic).
120. The compound of any one of aspects 112 to 116, wherein Y is CH(CH3).
121. The compound of any one of aspects 112 to 116, wherein Y is CH(CH2CH3).
122. The compound of any one of aspects 112 to 121, wherein R₁ is halo, CN, C1-C6 aliphatic, C1-C6 alkoxy, C3-C8 cycloalkyl, or a phenyl, pyridyl, pyrimidine, indole, aza-indole, or thiophene ring, wherein all rings may be substituted with halo, C1-C6 aliphatic, C1-C6 alkoxy, C1-C6 fluoroaliphatic, C1-C6 fluoroalkoxy, OH, CH₂OH, CH₂OCH₃, CN, CO₂H, amino, amido, C3-C10 heteroaryl, C3-C10 heterocycloalkyl, or a (C1-C8 aliphatic)-R4 wherein up to three CH2 units may be replaced with O, CO, S, SO, SO₂ or NR.
123. The compound of any one of aspects 112 to 122, wherein R₁ is selected from CH₃, Cl, F, CN, OCH₃, CF₃, CH₂CH₃, tBu, CH(CH₃)₂, 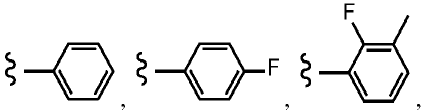 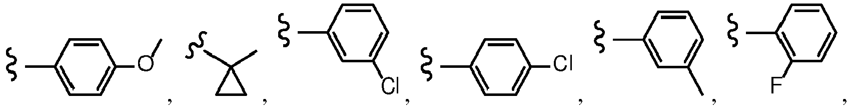 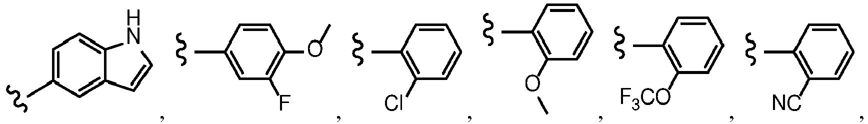 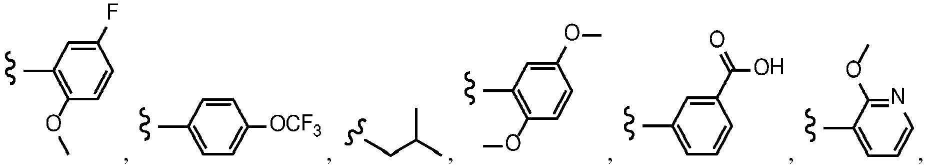 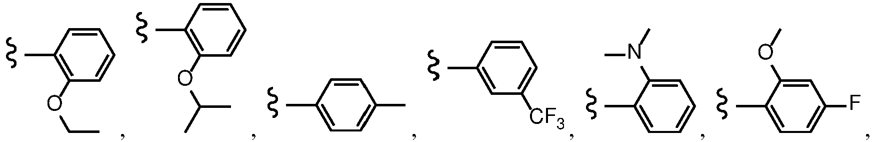 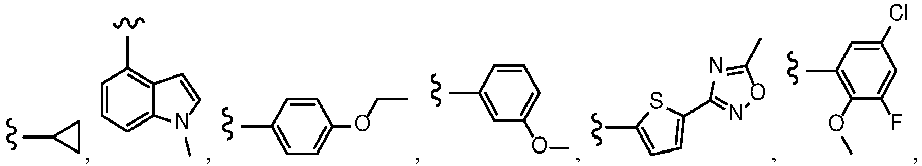 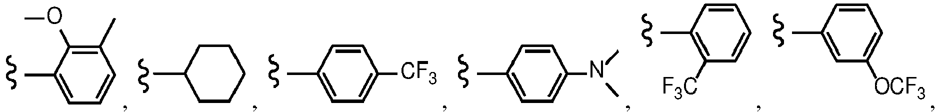 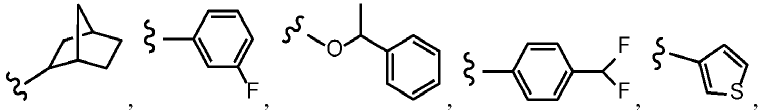 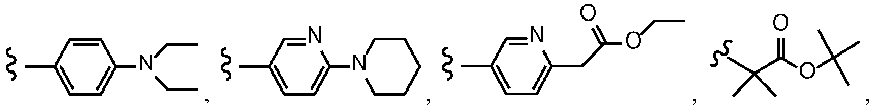 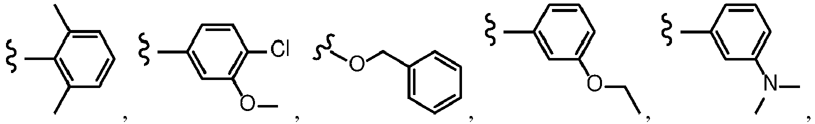 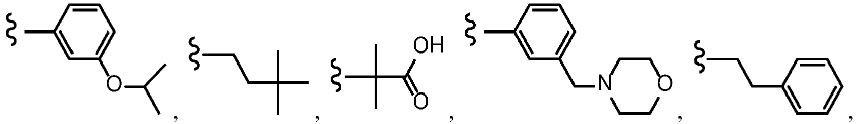 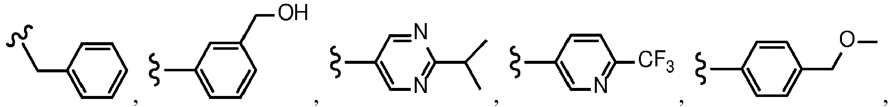 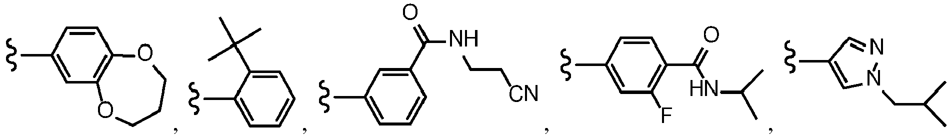 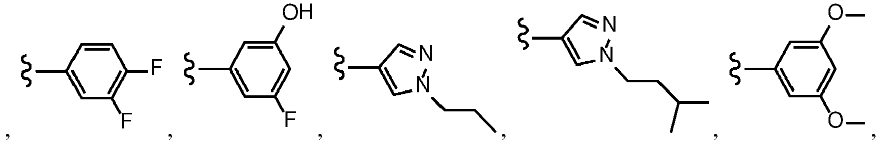 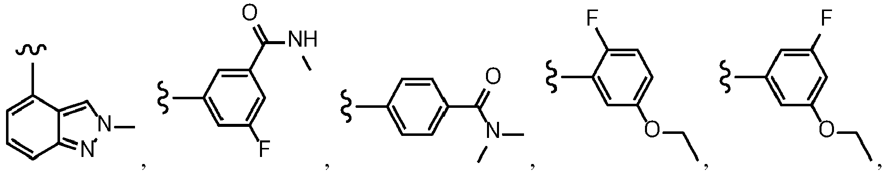 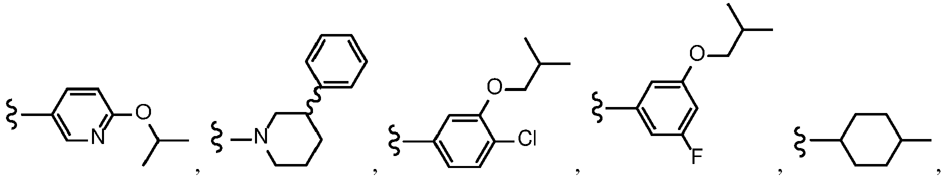 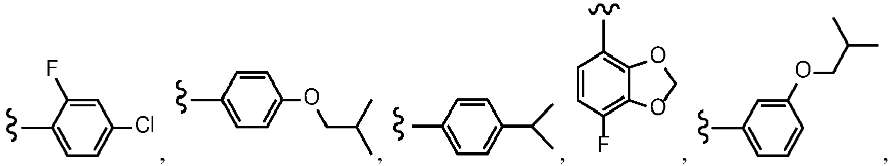 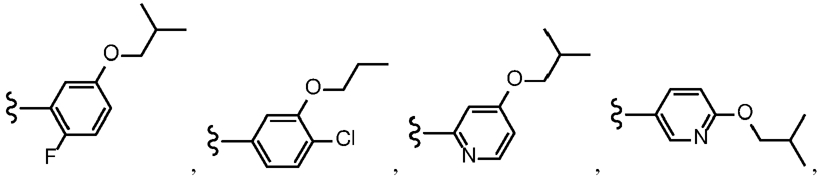 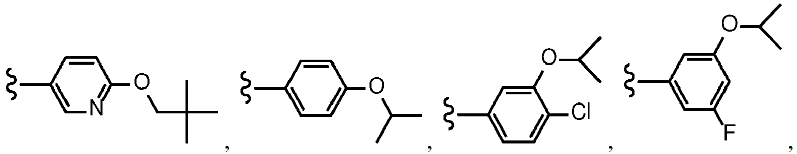 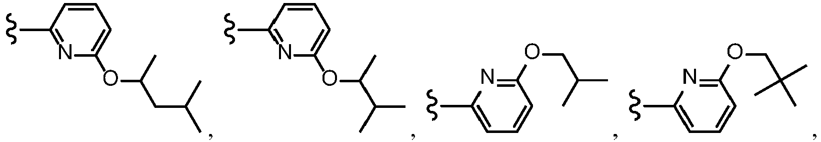 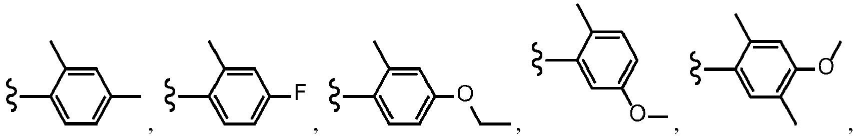 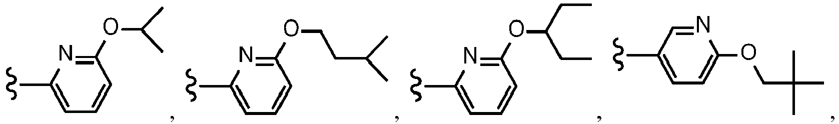 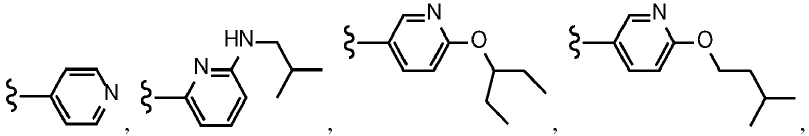 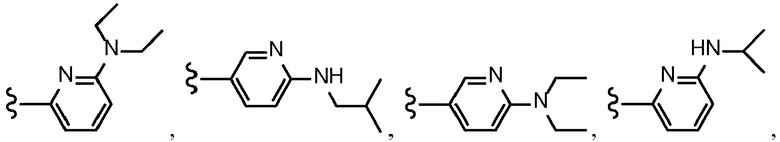 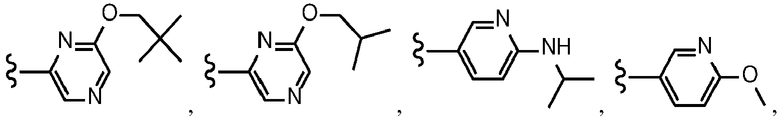 or 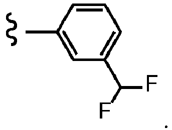
124. The compound of any one of aspects 112 to 123, wherein R2 is selected from halo, OH, CN, azide, amino, C1-C6 aliphatic or fluoroaliphatic, C1-C6 alkoxy or fluoroalkoxy, C3-C10 mono- or bicyclic heterocyclic ring wherein up to 4 carbon atoms may be replaced with O, S, N, or NR; or a (C1-C8 aliphatic)-R4 wherein up to three CH2 units may be replaced with O, CO, S, SO, SO₂ or NR.
125. The compound of any one of aspects 112 to 124, wherein R2 is selected from Cl, F, OH, CN, N₃, NH₂, NH(CH₃), N(CH₃)₂, CH₃, CH₂OH, CH₂CH₃, CH(CH₃)₂, CHF₂, OCH₃, OCF₃, OCHF₂, OCH(CH₃)₂, C(O)CH₃, CH₂CH₂OH, CH₂NH₂, NH(CH₂)₂OH, NH(CH₂)₂N(CH₃)₂, NH(CH₂)₂NH₂, NH(CH₂)₃NH₂, NH(CH₂)₂OCH₃, NHCH(CH₃)₂, 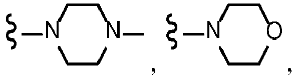 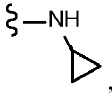 , or CO₂H.
126. The compound of any one of aspects 112 to 125, wherein R₃ is selected from halo, CN, C1-C6 aliphatic or fluoroaliphatic, C1-C6 alkoxy, or C3-C10 mono- or bicyclic heteroaryl wherein up to 4 carbon atoms may be replaced by O, S, N, or NR.
127. The compound of any one of aspects 112 to 126, wherein R₃ is selected from Cl, F, CN, CH₃, OCH₃, CF₃, CH₂CH₃, CH₂CF₃, CH₂CH₂CH₃, OCH₂CH₃, CH₂OCH₃, CH(CH₃)₂, CCH, CO₂CH₃, tBu, =CH₂, =O, 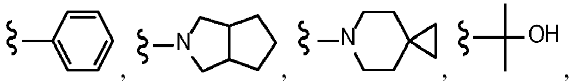 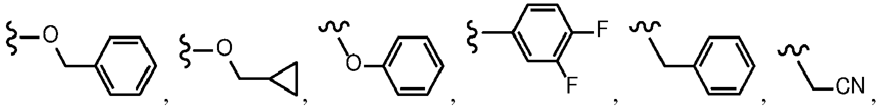 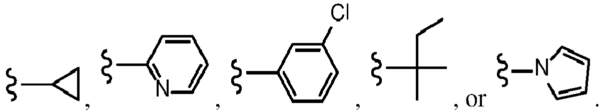
128. The compound of any one of aspects 112 to 127, wherein o is 0.
129. The compound of any one of aspects 112 to 127, wherein o is 1.
130. The compound of any one of aspects 112 to 127, wherein o is 2.
131. The compound of any one of aspects 112 to 130, wherein n is 0.
132. The compound of any one of aspects 112 to 130, wherein n is 1.
133. The compound of any one of aspects 112 to 130, wherein n is 2.
134. The compound of any one of aspects 112 to 133, wherein p is 0.
135. The compound of any one of aspects 112 to 133, wherein p is 1.
136. The compound of any one of aspects 112 to 133, wherein p is 2.
137. The compound of any one of aspects 112 to 136, wherein ring B is phenyl.
138. The compound of any one of aspects 112 to 137, wherein ring B is pyridyl.
139. The compound of any one of aspects 112 to 138, wherein ring C is phenyl.
140. The compound of any one of aspects 112 to 139, wherein ring B and ring C are phenyl.
141. The compound of any one of aspects 112 to 140, wherein ring B is pyridyl and ring C is phenyl.
142. A compound selected from Table 1.
143. A pharmaceutical composition comprising the compound of any one of aspects 1 to 142 and a pharmaceutically acceptable carrier.
144. The pharmaceutical composition of aspect 143, further comprising one or more additional therapeutic agent(s).
145. The pharmaceutical composition of aspect 144, wherein the additional therapeutic agent is selected from a mucolytic agent, bronchodialator, an antibiotic, an anti-infective agent, a CFTR modulator, or an anti-inflammatory agent.
146. The pharmaceutical composition of aspect 144, wherein the additional therapeutic agent is a CFTR modulator.
147. The pharmaceutical composition of aspect 144, wherein the additional therapeutic agent is a CFTR corrector.
148. The pharmaceutical composition of aspect 144, wherein the additional therapeutic agent is 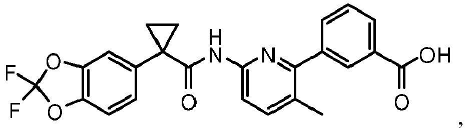 or pharmaceutically acceptable salt thereof.
149. The pharmaceutical composition of aspect 144, wherein the additional therapeutic agent is 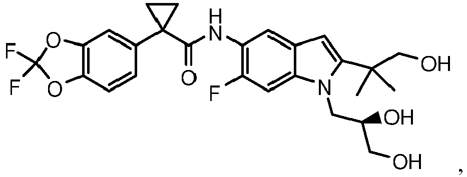 or pharmaceutically acceptable salt thereof.
150. The pharmaceutical composition of aspect 144, wherein the additional therapeutic agent is 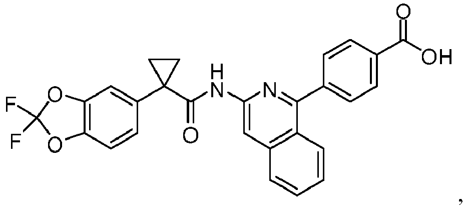 or pharmaceutically acceptable salt thereof.
151. The pharmaceutical composition of aspect 144, wherein the additional therapeutic agent is a CFTR potentiator.
152. The pharmaceutical composition of aspect 144, wherein the additional therapeutic agent is 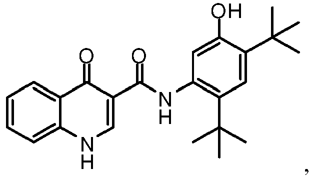 or pharmaceutically acceptable salt thereof.
153. The pharmaceutical composition of aspect 144, wherein the additional therapeutic agents are a CFTR corrector and a CFTR potentiator.
154. The pharmaceutical composition of aspect 144, wherein the additional therapeutic agents are 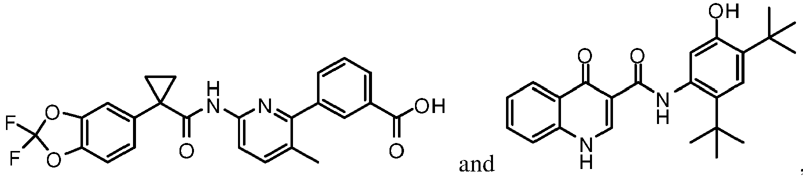 , or pharmaceutically acceptable salts thereof.
155. The pharmaceutical composition of aspect 144, wherein the additional therapeutic agents are 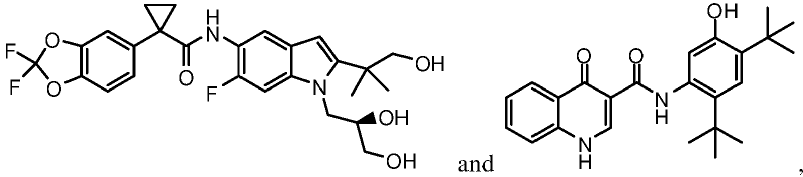 or pharmaceutically acceptable salts thereof.
156. The pharmaceutical composition of aspect 144, wherein the additional therapeutic agents are 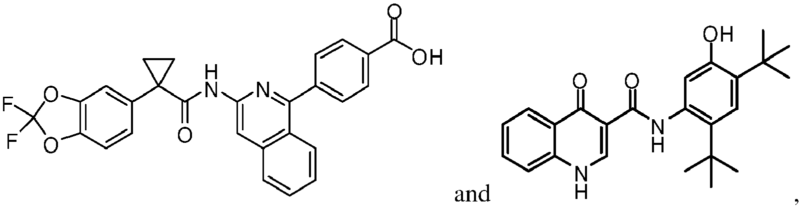 or pharmaceutically acceptable salts thereof.
157. A method of treating cystic fibrosis in a patient comprising administering to the patient an effective amount of the compound of any one of aspects 1 to 142 or the pharmaceutical composition of any one of aspects 143 to 156.
158. The method of aspect 157, further comprising administering to the patient one or more additional therapeutic agent(s) prior to, concurrent with, or subsequent to the compound of any one of aspects 1 to 142 or the pharmaceutical composition of any one of aspects 143 to 156.
159. The method of aspect 158, wherein the additional therapeutic agent is selected from a mucolytic agent, bronchodialator, an antibiotic, an anti-infective agent, a CFTR modulator, or an anti-inflammatory agent.
160. The method of aspect 158, wherein the additional therapeutic agent is a CFTR modulator.
161. The method of aspect 158, wherein the additional therapeutic agent is a CFTR corrector.
162. The method of aspect 158, wherein the additional therapeutic agent is 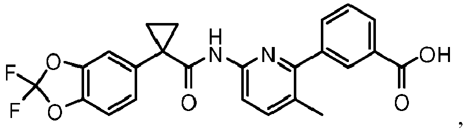 or a pharmaceutically acceptable salt thereof.
163. The method of aspect 158, wherein the additional therapeutic agent is 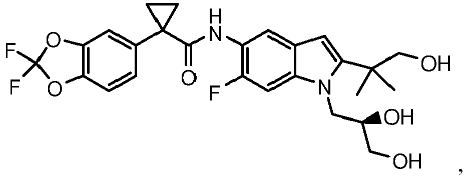 or a pharmaceutically acceptable salt thereof.
164. The method of aspect 158, wherein the additional therapeutic agent is 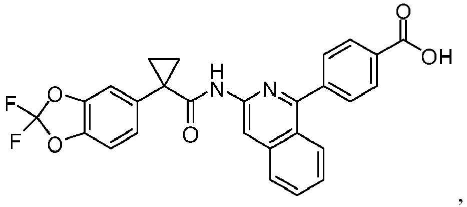 or a pharmaceutically acceptable salt thereof.
165. The method of aspect 158, wherein the additional therapeutic agent is a CFTR potentiator.
166. The method of aspect 158, wherein the additional therapeutic agent is 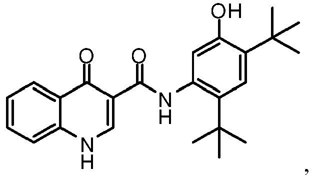 or a pharmaceutically acceptable salt thereof.
167. The method of any one of aspects 157 to 166, wherein the patient is homozygous in the F508del mutation.
168. The method of any one of aspects 157 to 166, wherein the patient is heterozygous in the F508del mutation.
169. A kit comprising the compound of any one of aspects 1 to 142 or the pharmaceutical composition of any one of aspects 143 to 156, and instructions for use therof.
170. The kit of aspect 169, further comprising one or more additional therapeutic agent(s).
171. The kit of aspect 170, wherein the additional therapeutic agent is selected from a mucolytic agent, bronchodialator, an antibiotic, an anti-infective agent, a CFTR modulator, or an anti-inflammatory agent.
172. The kit of aspect 170, wherein the additional therapeutic agent is a CFTR modulator.
173. The kit of aspect 170, wherein the additional therapeutic agent is a CFTR corrector.
174. The kit of aspect 170, wherein the additional therapeutic agent is 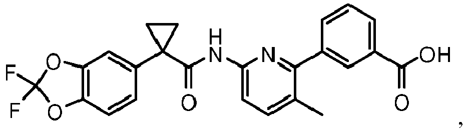 or a pharmaceutically acceptable salt thereof.
175. The kit of aspect 170, wherein the additional therapeutic agent is 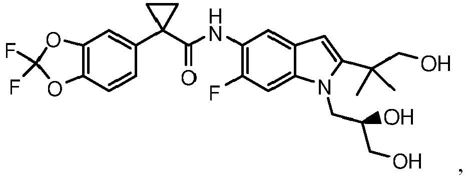 or a pharmaceutically acceptable salt thereof.
176. The kit of aspect 170, wherein the additional therapeutic agent is 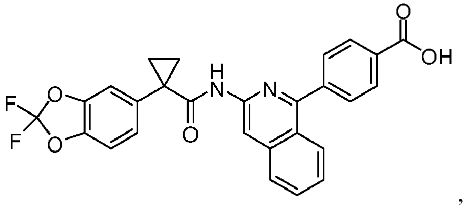 or a pharmaceutically acceptable salt thereof.
177. The kit of aspect 170, wherein the additional therapeutic agent is a CFTR potentiator.
178. The kit of aspect 170, wherein the additional therapeutic agent is 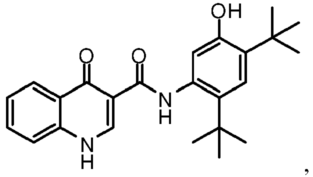 or a pharmaceutically acceptable salt thereof.
179. The kit of aspect 170, wherein the additional therapeutic agents are a CFTR corrector and a CFTR potentiator.
180. The kit of aspect 170, wherein the additional therapeutic agents are 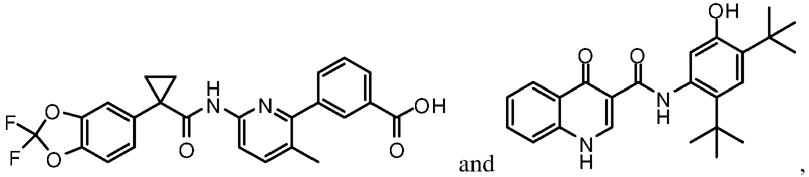 or pharmaceutically acceptable salts thereof.
181. The kit of aspect 170, wherein the additional therapeutic agents are 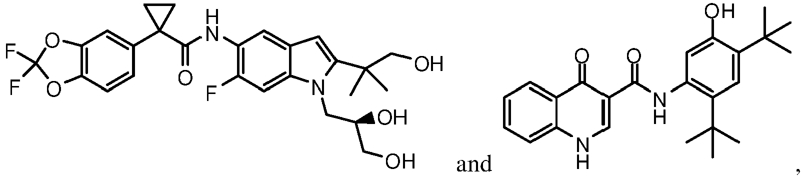 or pharmaceutically acceptable salts thereof.
182. The kit of aspect 170, wherein the additional therapeutic agents are 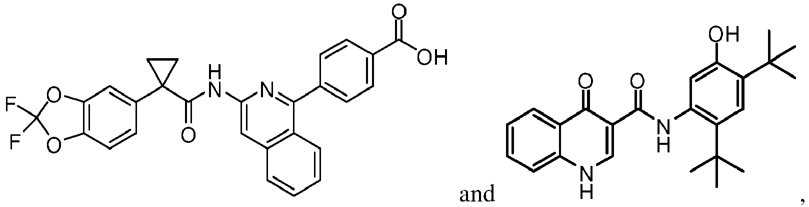 or pharmaceutically acceptable salts thereof.
183. The kit of any one of aspects 169 to 182, wherein the compound of any one of aspects 1 to 142 or the pharmaceutical composition of any one of aspects 143 to 156 and the one or more additional therapeutic agent(s) are in separate containers.
184. The kit of any one of aspects 169 to 182, wherein the compound of any one of aspects 1 to 142 or the pharmaceutical composition of any one of aspects 143 to 156 and the one or more additional therapeutic agent(s) are in the same container.
185. The kit of aspect 183 or 184, wherein the container is a bottle, vial, or blister pack, or combination thereof.

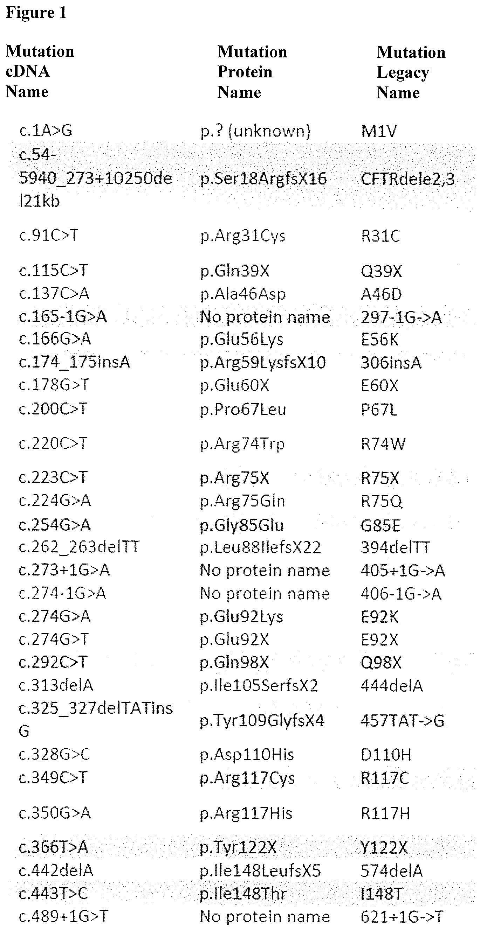

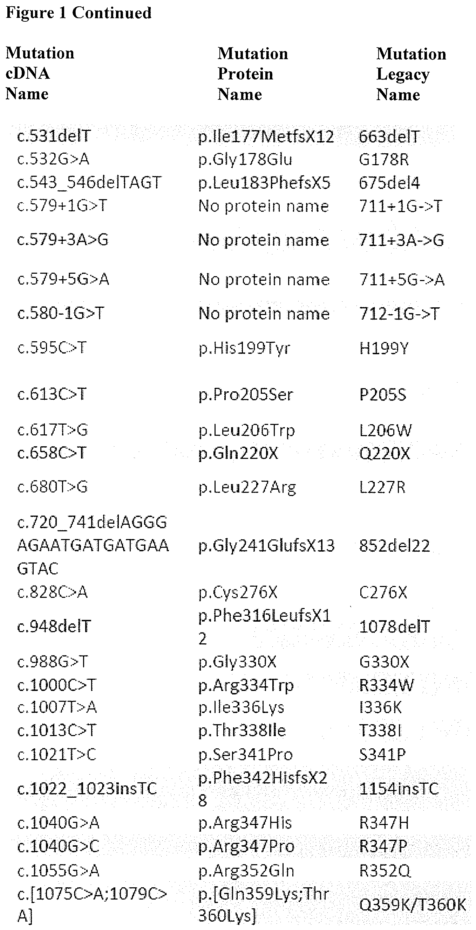

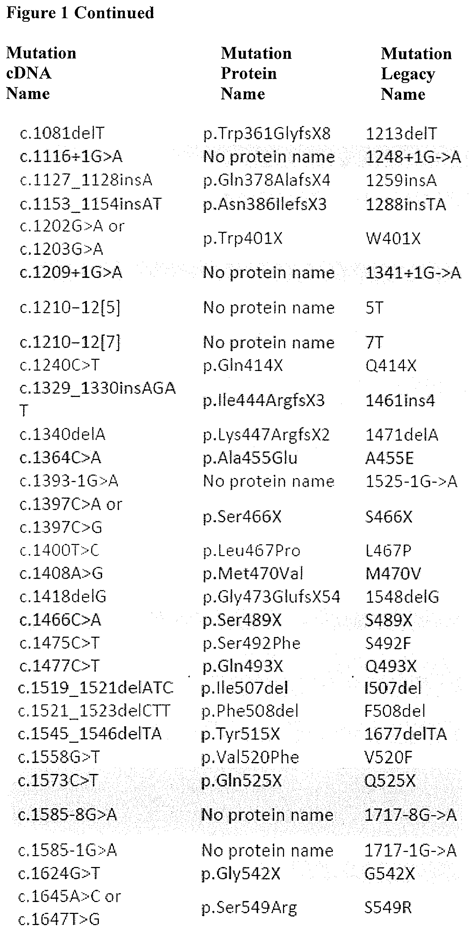

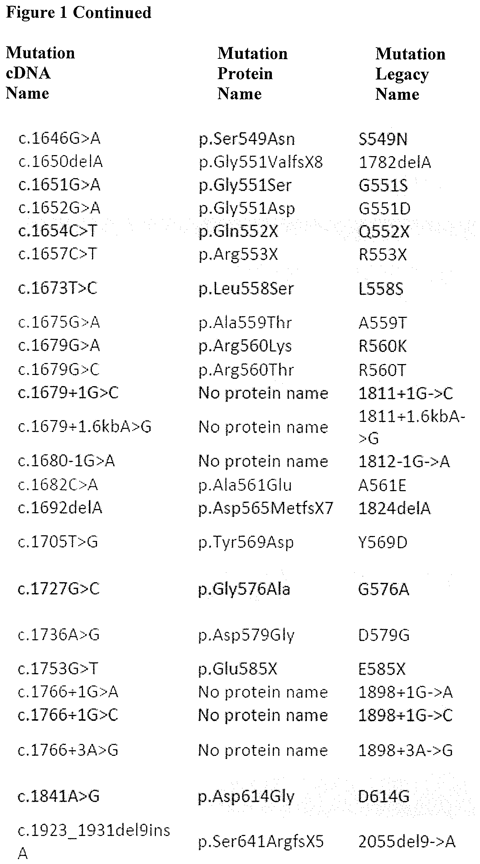

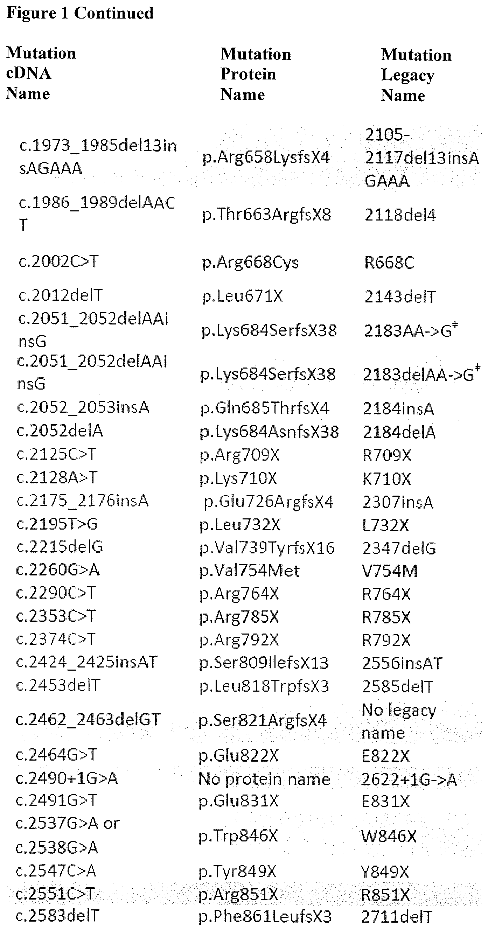

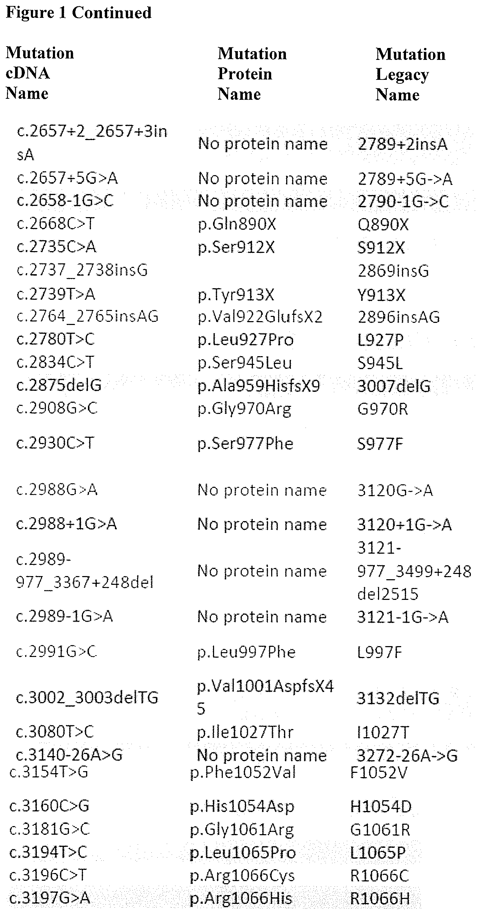

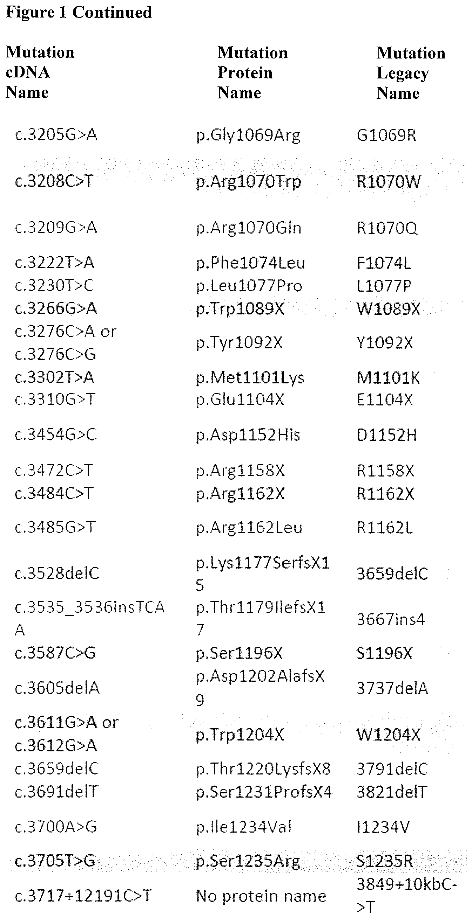

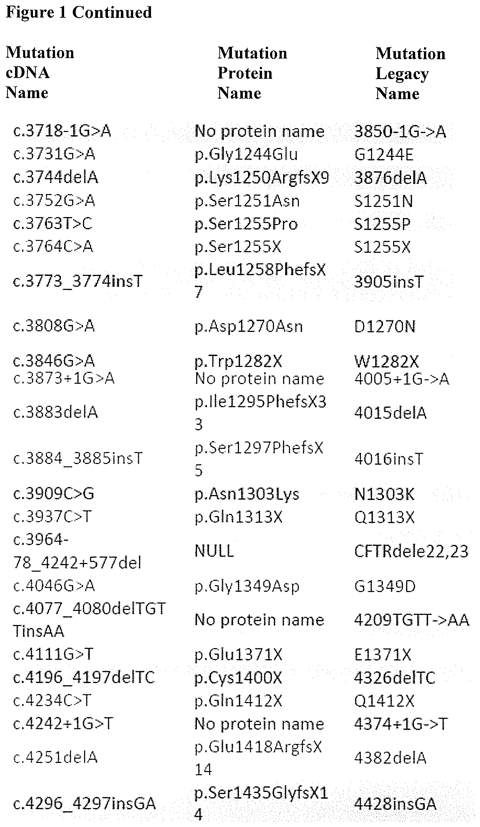

## Claims

1. A compound of formula Ic: 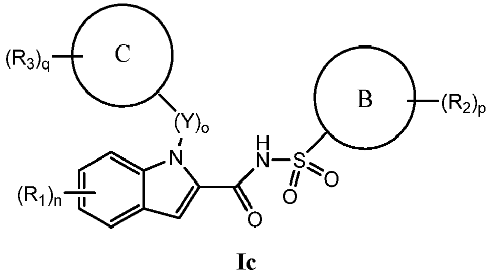 a deuterated derivative thereof, or a pharmaceutically acceptable salt of any of the foregoing, wherein, independently for each occurrence:
Ring B is a C6-C10 aryl ring or C3-C10 heteroaryl or heterocyclic ring wherein anywhere from 1 to 4 ring atoms are independently O, S, N, or NR;
Ring C is a C6-C10 aryl ring, C3-C14 heteroaryl or heterocyclic ring wherein anywhere from 1 to 4 ring atoms are independently N, O, or S, or a C3-C10 cycloalkyl ring;
Y is CRR, CO, O, S, SO, SO₂, S(O)NH or NR;
R₁ is halo; CN; F₅S; SiR₃; OH; C1-C6 alkyl or fluoroalkyl; C1-C6 alkoxy or fluoroalkoxy; C2-C6 alkenyl; C2-C6 alkynyl; (C1-C9 alkylene)-R₄ wherein up to four CH₂ units are independently replaced with O, CO, S, SO, or SO₂; C6-C10 aryl; C3-C10 heteroaryl or heterocyclic ring wherein anywhere from 1 to 4 ring atoms are independently O, S, N, or NR; or C3-C10 cycloalkyl;
R₂ is halo; OH; NRR; azide; CN; CO₂R; C1-C6 alkyl or fluoroalkyl; C1-C6 alkoxy or fluoroalkoxy; C2-C6 alkenyl; C2-C6 alkynyl; C6-C10 aryl; C3-C13 heteroaryl or heterocyclic ring wherein anywhere from 1 to 4 ring atoms are independently O, S, N, or NR; C3-C10 cycloalkyl; or a (C1-C9 alkylene)-R₄ wherein up to four CH₂ units are independently replaced with O, CO, S, SO, SO₂ or NR;
or two R₂ groups taken together may form a =CH₂ or =O group;
R₃ is CN; CO₂R; C1-C6 alkyl or fluoroalkyl; C2-C6 alkenyl; C2-C6 alkynyl; C1-C6 alkoxy or fluoroalkoxy; or C6-C10 aryl; C3-C10 heteroaryl or heterocyclic ring wherein anywhere from 1 to 4 ring atoms are independently O, S, N, or NR; C3-C10 cycloalkyl; or a (C1-C9 alkylene)-R₄ wherein up to four CH₂ units are independently replaced with O, CO, S, SO, SO₂ or NR;
or two R₃ groups taken together may form a =CH₂ or =O group;
R₄ is H; azide; CF₃; CHF₂; OR; CCH; CO₂R; OH; C6-C10 aryl, C3-C10 heteroaryl or heterocycloalkyl wherein anywhere from 1 to 4 ring atoms are independently O, S, N, or NR; C3-C10 cycloalkyl; NRR, NRCOR, CONRR, CN, halo, or SO₂R;
R is independently H; OH; CO₂H; CO₂C1-C6 alkyl; C1-C6 alkyl; C2-C6 alkenyl; C2-C6 alkynyl; C6-C10 aryl; C3-C10 heteroaryl or heterocycloalkyl wherein anywhere from 1 to 4 ring atoms are independently O, S, N, or NR; or C3-C10 cycloalkyl;
n is 0, 1, 2 or 3;
o is 0, 1, 2, 3, 4, or 5;
p is 0, 1, 2, or 3; and
q is 0, 1, 2, 3, 4, or 5;
wherein each of the specific groups for the variables R₁-R₄ can be optionally substituted with one or more groups selected from halo, phospho, OH, cycloalkyl, heterocycloalkyl, aryl, heteroaryl, fluoroalkyl, alkyl, alkenyl, alkynyl, nitro, CN, hydroxyl, and (C₁-C₉ alkylene)-E wherein up to 4 CH₂ units are independently replaced with O, S, SO₂, SO, CO, NH, *N*-alkyl, *N*-alkenyl, or *N*-alkynyl, and E is H, aryl, cycloalkyl, heterocycloalkyl, heteroaryl, alkoxy, CN, or CF₃, further wherein each of the aryl, cycloalkyl, heterocycloalkyl, and heteroaryl is optionally substituted with one or more groups selected from halo, alkyl, amino, CN, alkenyl, alkynyl, and alkoxy; and
when two alkoxy groups are bound to the same atom or adjacent atoms, the two alkoxy groups can form a ring together with the atom(s) to which they are bound; and
wherein the term "amino" refers to NH₂ which is optionally substituted with one or two groups independently selected from alkyl, cycloalkyl, and heterocycloalkyl.

2. The compound, deuterated derivative, or salt of claim 1, wherein ring B is:
(a) phenyl, pyridyl, pyridine-2(1*H*)-one, pyrazole, indole, thiophene, dihydrobenzofuran, pyrazine, indazole, thiazole, pyridine-4(1H)-one, pyrrolidinone, or quinoline; or
(b)

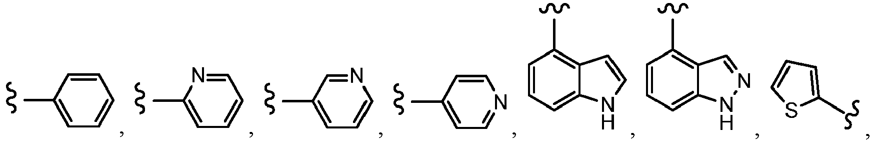

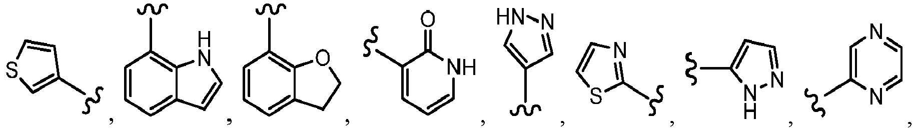

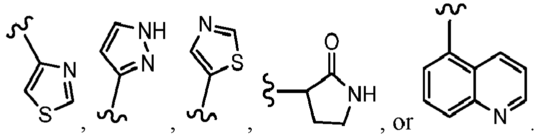

3. The compound, deuterated derivative, or salt of any one of claims 1 or 2, wherein ring C is:
(a) phenyl, indole, cycloalkyl, pyridyl, pyrrolidine, naphthalene, or dihydroindene; or
(b)

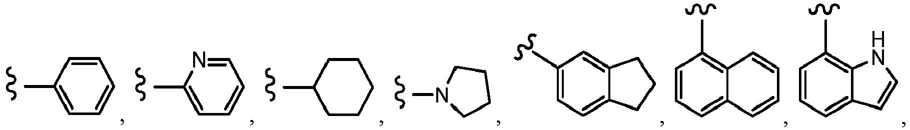

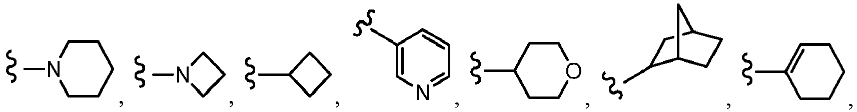

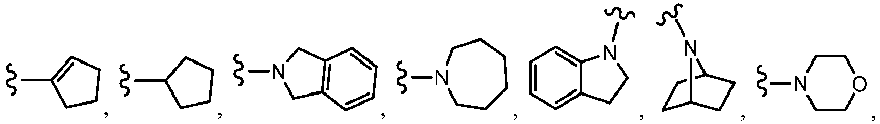

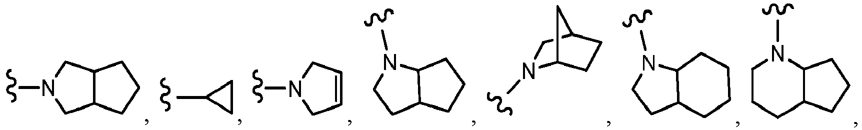

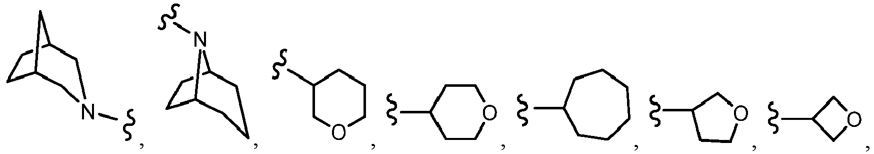

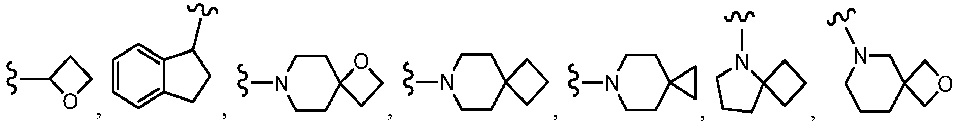

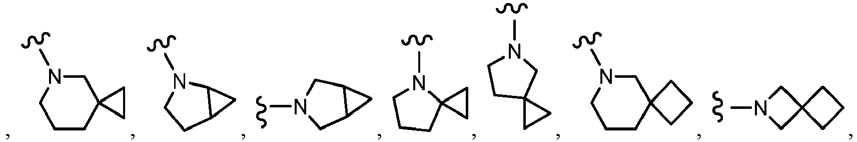

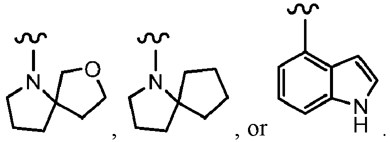

4. The compound, deuterated derivative, or salt of any one of claims 1 to 3, wherein Y is:
(a) O;
(b) CH₂;
(c) CH(C1-C6 alkyl);
(d) CH(CH₃); or
(e) CH(CH₂CH₃).

5. The compound, deuterated derivative, or salt of any one of claims 1 to 4, wherein R₁ is:
(a) halo, CN, C1-C6 alkyl, C1-C6 alkoxy, C3-C8 cycloalkyl, or a phenyl, pyridyl, pyrimidine, indole, aza-indole, or thiophene ring, wherein all rings may be substituted with halo, C1-C6 alkyl, C1-C6 alkoxy, C1-C6 fluoroalkyl, C1-C6 fluoroalkoxy, OH, CH₂OH, CH₂OCH₃, CN, CO₂H, amino, amido, C3-C10 heteroaryl, C3-C10 heterocycloalkyl, or a (C1-C8 alkyl)-R₄ wherein up to three CH₂ units may be replaced with O, CO, S, SO, SO₂ or NR; or
(b) CH₃, Cl, F, CN, OCH₃, CF₃, CH₂CH₃, tBu, CH(CH₃)₂, 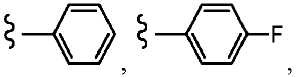 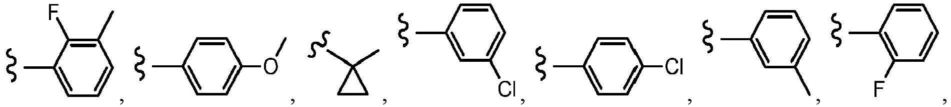 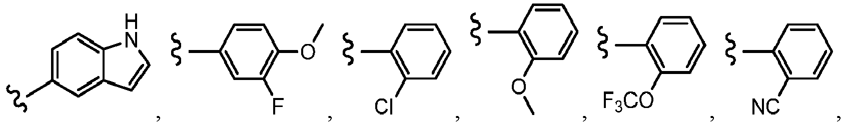 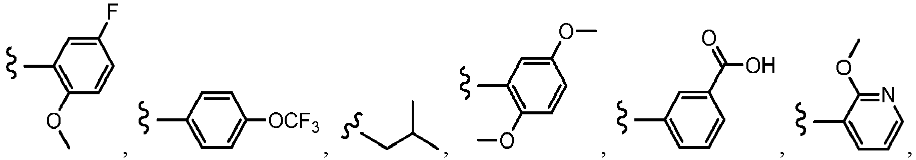 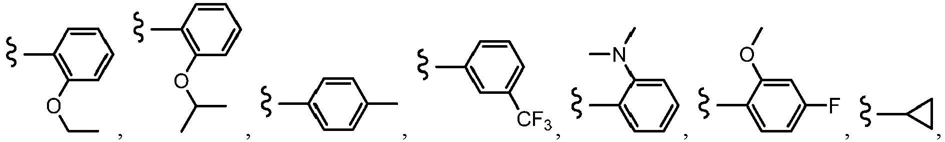 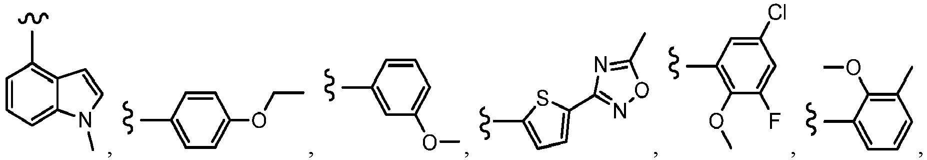 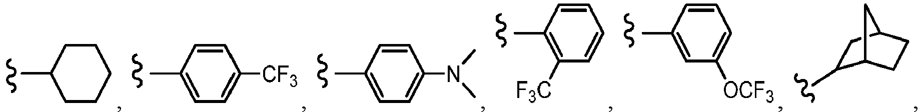 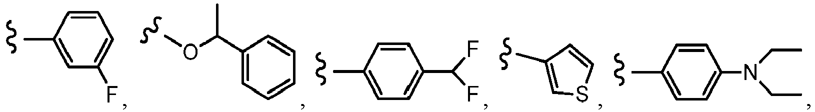 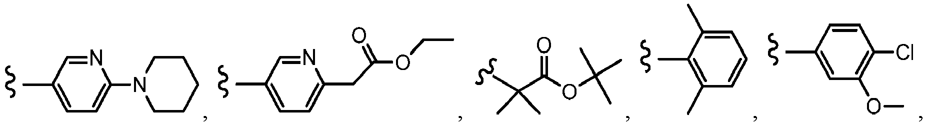 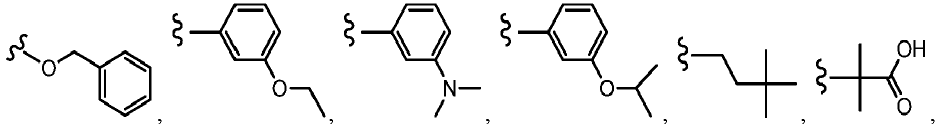 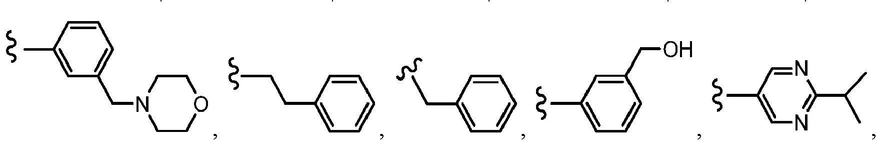 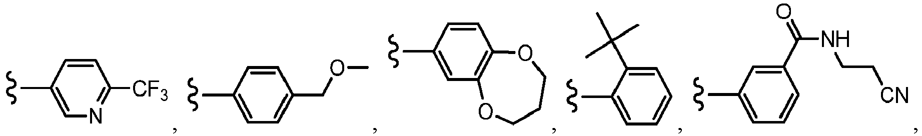 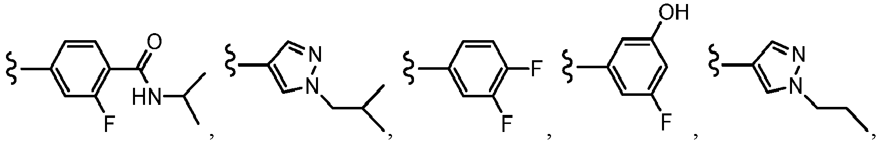 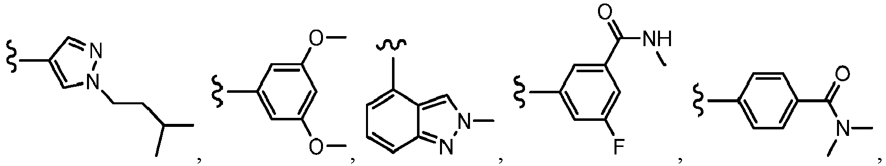 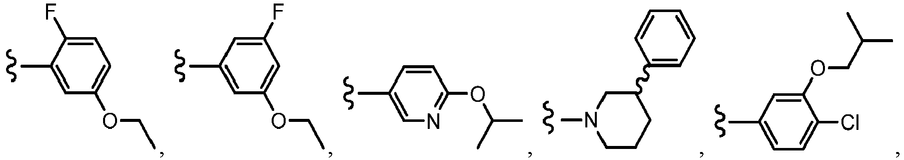 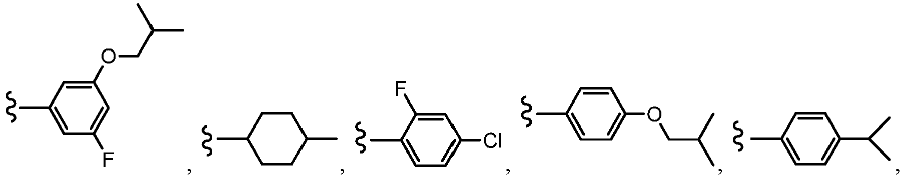 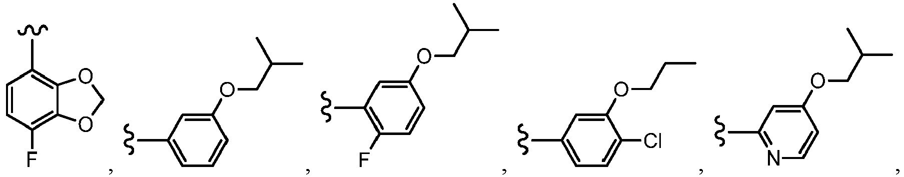 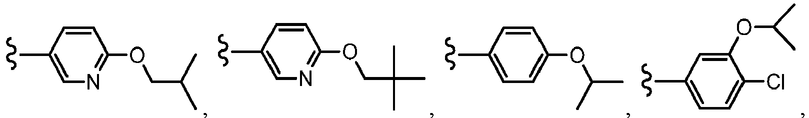 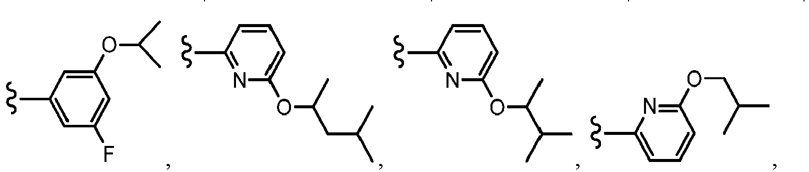 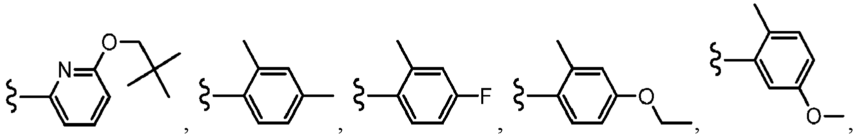 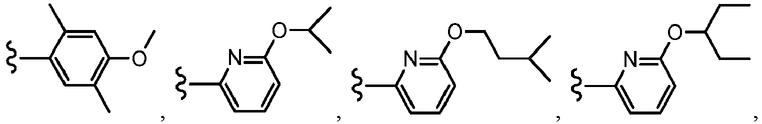 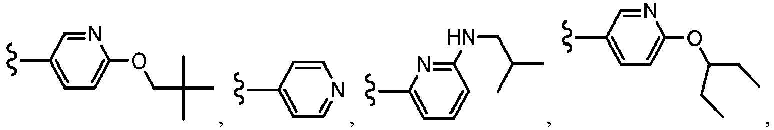 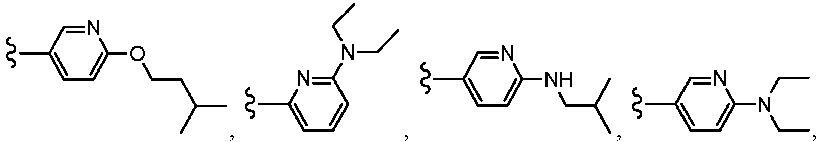 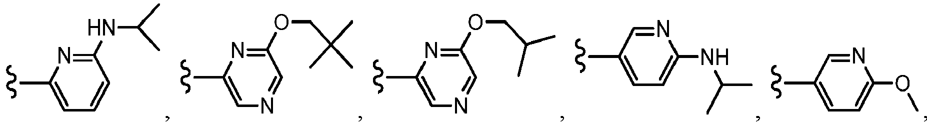 , or

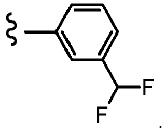

6. The compound, deuterated derivative, or salt of any one of claims 1 to 5, wherein R₂ is:
(a) halo, OH, CN, azide, amino, C1-C6 alkyl or fluoroalkyl, C1-C6 alkoxy or fluoroalkoxy, C3-C10 mono- or bicyclic heterocyclic ring wherein up to 4 carbon atoms may be replaced with O, S, N, or NR; or a (C1-C8 alkyl)-R₄ wherein up to three CH₂ units may be replaced with O, CO, S, SO, SO₂ or NR; or
(b) Cl, F, OH, CN, N₃, NH₂, NH(CH₃), N(CH₃)₂, CH₃, CH₂OH, CH₂CH₃, CH(CH₃)₂, CHF₂, OCH₃, OCF₃, OCHF₂, OCH(CH₃)₂, C(O)CH₃, CH₂CH₂OH, CH₂NH₂, NH(CH₂)₂OH, NH(CH2)2N(CH3)2, NH(CH₂)₂NH₂, NH(CH₂)₃NH₂, NH(CH₂)₂OCH₃, NHCH(CH3)2, 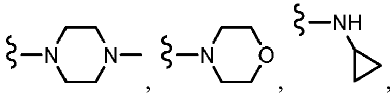 , or CO₂H.

7. The compound, deuterated derivative, or salt of any one of claims 1 to 6, wherein R₃ is:
(a) CN, C1-C6 alkyl or fluoroalkyl, C1-C6 alkoxy, or C3-C10 mono- or bicyclic heteroaryl wherein up to 4 carbon atoms may be replaced by O, S, N, or NR; or
(b) CN, CH3, OCH₃, CF₃, CH₂CH₃, CH₂CF₃, CH₂CH₂CH₃, OCH₂CH₃, CH₂OCH₃, CH(CH₃)₂, CCH, CO₂CH₃, tBu, =CH₂, =O,

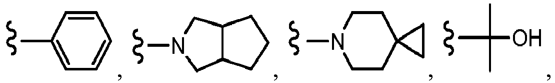

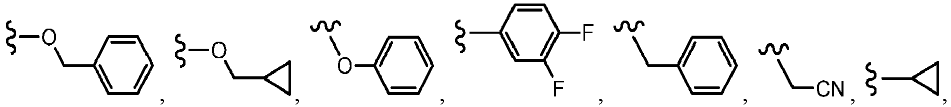

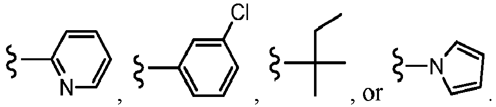

8. The compound, deuterated derivative, or salt of any one of claims 1 to 7, wherein o is:
(a) 0;
(b) 1; or
(c) 2.

9. The compound, deuterated derivative, or salt of any one of claims 1 to 8, wherein n is:
(a) 0;
(b) 1; or
(c) 2.

10. The compound, deuterated derivative, or salt of any one of claims 1 to 9, wherein p is:
(a) 0;
(b) 1; or
(c) 2.

11. The compound, deuterated derivative, or salt of any one of claims 1 to 10, wherein:
(a) ring B is phenyl;
(b) ring C is phenyl or pyridyl; and/or
(c) ring B and ring C are phenyl.

12. A compound selected from Table 1, wherein the compound is a compound of formula Ic, a deuterated derivative thereof, or a pharmaceutically acceptable salt of any of the foregoing.

13. A pharmaceutical composition comprising the compound, deuterated derivative, or salt of any one of claims 1 to 12 and a pharmaceutically acceptable carrier.

14. The pharmaceutical composition of claim 13, further comprising one or more additional therapeutic agent(s).

15. The pharmaceutical composition of claim 14, wherein at least one additional therapeutic agent is a CFTR modulator.

16. The pharmaceutical composition of claim 14 or 15, wherein at least one additional therapeutic agent is 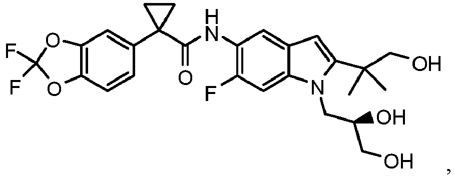 or a pharmaceutically acceptable salt thereof.

17. The pharmaceutical composition of any one of claims 14 to 16, wherein at least one additional therapeutic agent is 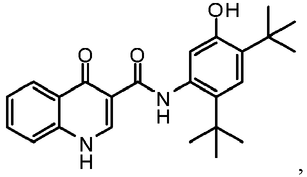 or a pharmaceutically acceptable salt thereof.

18. The compound, deuterated derivative, or salt of any one of claims 1 to 12 or the pharmaceutical composition of any one of claims 13 to 17 for use in a method of treating cystic fibrosis in a patient.

19. The compound, deuterated derivative, salt, or composition for use of claim 18, wherein the method further comprises administering to the patient one or more additional therapeutic agent(s) prior to, concurrent with, or subsequent to the compound, deuterated derivative, or salt of any one of claims 1 to 12 or the pharmaceutical composition of any one of claims 13 to 17.

20. The compound, deuterated derivative, salt, or composition for use of claim 19, wherein the one or more additional therapeutic agent(s) comprises a CFTR modulator.

21. The compound, deuterated derivative, salt, or composition for use of claim 19 or 20, wherein the one or more additional therapeutic agent(s) comprises 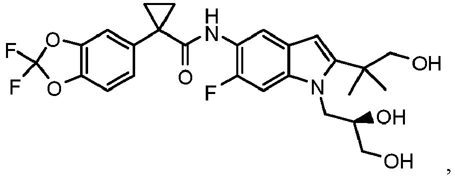 or a pharmaceutically acceptable salt thereof.

22. The compound, deuterated derivative, salt, or composition for use of any one of claims 19 to 21, wherein the one or more additional therapeutic agent(s) comprises 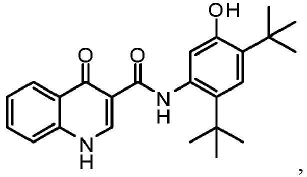 or a pharmaceutically acceptable salt thereof.

23. The compound, deuterated derivative, salt, or composition for use of any one of claims 18 to 22, wherein the patient is homozygous in the F508del mutation; or wherein the patient is heterozygous in the F508del mutation.
